# EUROPEAN PATENT APPLICATION

(11) **EP 1 676 927 A2**
(43) Date of publication of application: **05.07.2006**
(21) Application number: 06002091.4
(22) Date of filing: 02.07.2001
(51) Int. Cl.: C12Q 1/68, B01J 19/00, G01N 33/483, C07K 14/47

(54) **Diagnosis of diseases associated with development by means of assessing their methylation status**

(30) Priority: 30.06.2000 DE 10032529; 01.09.2000 DE 10043826
(62) Divisional of application: 01962813.0
(71) Applicant: Epigenomics AG, 10178 Berlin (DE)
(72) Inventor: Olek, Alexander, 10407 Berlin (DE); Piepenbrock, Christian, 10115 Berlin (DE); Berlin, Kurt, 14532 Stahnsdorf (DE)
(74) Representative: Krauss, Jan

(57) **Abstract**

The present invention relates to the chemically modified genomic sequences of genes associated with diseases associated with development, to oligonucleotides and/or PNA-oligomers for detecting the cytosine methylation state of genes associated with diseases associated with development which are directed against the sequence, as well as to a method for ascertaining genetic and/or epigenetic parameters of genes associated with diseases associated with development.

Sequence listing

<110> Epigenomics AG

<120> Diagnosis of diseases associated with
      development genes

<160> 354

<210> 1
<211> 16633
<212> DNA
<213> Artificial Sequence

<220>
<223> chemically treated genomic DNA (Homo sapiens)

<400> 1

aaatagatta ttagtgtgtt gagtaatgta gtattgaata tgttagttat tgtagtttgg      60
ttttatggta tttatttggt tagtttgtag aaagatagat aaatgatatg aataaaagta     120
tatttattga tttttttatt gatttgaagt atttattatg ttagttgtgt aaagttatat     180
tttttttaag tttaaggttt ttatttttaa atttaatggg aggatagaat ttagagtatg     240
gtttaattat tagtgttaaa tttgtatttt taagaataat tttgtgattg ttaaagtttg     300
agaggtttat tttgtattat atggttttta taatagagat gtgattaaat atattgtttt     360
ttgtattagg aaatttggaa tttgtatatt gtttcgttgg tttttagatt aaagaaaaaa     420
aattatagta gatgaatttt agaatagttt tttgtttaga gttaatattt tagattaaaa     480
ttatattttt taaatttttt tgtgtagtag attatttttg aaatttttag ggggtggggg      540
aagtaggaat ataaaatgtt agaaattgag taaattgtat tatttttgaa ttttaaatat     600
tgatgttgta attggatgtt gaagattttg ttagagttta ttgatttatg agttgaagag      660
ttttttatgt agggtcgtgt gtttatattt ggaggaaatt ttttaagatt tggagtttga      720
tagcgggaga tatagggttg gatgtatgaa agaattttta tgtggagttt tttgtttgaa      780
tgtaaatttt gagggtagga atttgattgt atttgatgtt atatttttag tgtttaaatt     840
agtgtttggt ttattgtagg tgtttagtat tttttggtta aaggtatgaa tgaatatggg     900
aaggaaaggt agtgaatttg ttgtagttta gaacgttttt tgtattttgt attttatgta     960
gtttattttt ggtttggttt ttgttaaggg ttaggaagtt ggttggaaga gagatttatt    1020
tatatgttag aatattagtt gtgtgttagt attgtgttag ttttggagat gatatggtat    1080
aaagattttt tttaaggttt tttttagtat tggattgttg ggggttttat tttttttttt     1140
gatagtgatt gtaataaggg atgttttttt agaatgattt tttttaaagt agtagagtga    1200
tttttgagtt taggggattg gattaattgt taattttttt tttttttttt ttgagatgga    1260
gtttttttgt gtcgtttagg ttggagtgta gtggtacgat ttgggtttat tgtaagtttt    1320
gtttttcggg tttatgttat ttttttgttt tattttttta agtagttgat tttataggcg   1380
tttgttataa cgtttagtta attttttaaa aatatttttt atatagatgg ggttttattg   1440
tgttagttag gatgatttcg attttttgat tttatgattt gtttgttttg gttttttaaa  1500
gtgttgggat tataggtgtt agttatcgtg ttttgttaat tttttttttt tataaagaga    1560
tggggttttg ttatgttgtt taggttggaa tgtagtggtt atttataggt aagattatgg    1620
tatattatag tttttaattt tgaatttttg ggtttaagta atttttttgt tttagttttt     1680
ttaatagttg gttttataag tgtgtgttgt tgtatttagt tttgggtgga tttttaattg    1740
taagtttaag taagttttga taattttgtt ttattttatt gtttttgaga agtagttagg    1800
taaatgcggt cgttaaaata tatgtttaag tagttttagt ggtttttttg gttttttttt   1860
gtttttttta agttagaagt tagggtaagt tttttttttt gttttatttt atttttttat     1920
aattttagat aaaataatga ttataaggat ttgaaatttt tgtgattgtc gaaggagaag    1980
ggttgagagt gagtgggttt tatttttttt tttatttttt tatgttaatt ttgagtttgt    2040
gtattttata gttttttttt tttagtatta aattgaaatt ttatttatat tttttttaat    2100
tttaatattt ttattttttg attttggggg attagaagtt aagtatggat aagaggagag     2160
tggttagggt attgagtaag tatgtatgta tattttataa tttttgttat atttggtagg    2220
ttgtgttgaa ttttttgggt aaaaggtttg aagttttgtt attattaggg gaataggaat    2280
tgtggttgtc gtcgggatta tagttaggag gttatttata gtttgtagtg agtggttgtg    2340
agtttattat ggttttgaat atgttttttg ttttaaagtt tttagtgttt ttgaagcgga   2400
ttttgttttt gttggttaga tagtagttaa tgataaagtg gacggttgta ggatgtggcg    2460
cgtgatcgtt ttggcggttt atagtttgta ggtgtttgtg ttttggttga tttgaattta    2520
gtcggagagt aggtgttttt tgtttttatg ttggtttttt ggtttttttt ttagttggtt    2580
ttattgatat ggagttgatg gattattcgt taattagagt ttattttttt tattttatat    2640
gtgttatgtt aggttaagat ttataatagg ttatgaattt agttttttta tttttttgag   2700
tttaggttaa atatttagat ttgttttgta aaaatggtta agattaggtg atattttgta    2760
tttttagttt atattaaggt atatggatta gaaggaaaag gtgaaaaagt aagtttttat    2820

aagaaataat taagaaagga aaattttttt tttgttttta tagtaatgta tgatatgcgt 2880
atttatgttg atttttggat gttttaatgg tggagtgata atttatgcgt tcgtttgcgt 2940
ttgtaggtga tggaaaattt taatttaggc gagttttaat aagatgaata tggaatggtt 3000
gttattttgt ggagtttagg gtaaaatagg ttttagattt gggttgattt agggtttaag 3060
tagtgttttt agagttttgt tatttttttt ttattttttt ttttgttagt tttattttta 3120
gtaggttttt tttttaaggt agtaaaatgg ttatggtata ttttatattt aagtataatg 3180
agaaaattat attttttttt ttagtagttt ttgtaaaatt ttaagagtta ttttgattgg 3240
gtgggtttag gttacgttat attttggaat ttagtgtagt gtgatgattg gtttttaatt 3300
atattttagt atgtagaata ggagggggtg tattttagat ttagtttagg agttgttgtc 3360
gagaaggaga gagggaggaa ttgaggaagt atagagtttt tgtagaatga ttttatgaag 3420
tatatattgg ggatttttat ttttataaaa agagtattgt ttttgggatt ttatatagtg 3480
ttgtttggtt tttaaaaagt attaagtttt tgtgtggttt ttgggtaagt ttggtatttt 3540
ttagaagtag ggagttagtg atttttttga tgtttttggg atgtttattg tatggtatta 3600
gattatagta ggttataatg gatgttatgt ttggtagttg tgtggtgtga tattagatta 3660
attagttttt tttgtacgtt attatattaa agagtgtcgt tggatagtta tttttaatat 3720
atgttttgtt tgtttttgaa aatacgtgtt ttattttttt ttgggttatt tagatttatt 3780
ttgggtgatt gttttgttag tttggggttg gggagagggg aggataggtt ttattttggt 3840
agtgttttaa gtttggagta gtattttgta gttagtatta taagttgtat aatttgttta 3900
tggaggttta tgtattatag aaatgaaaat tttttatttg atagtattta ttgtttagta 3960
tgtagtaatt tggttgttag aaatagttgg ttttgtaggg tgggataata gaaaggcgag 4020
attcgagaga ggttagtgtt tatttaagat ttttattgtg agaggattag gttattgaga 4080
ttgttattaa taatagattg taaggggttt tgggtatagt ttttagtgag taggagtttg 4140
tggtttagat agtgagtgat ttttttttgt tttgttgaat ggaataaaaa taaataaatt 4200
aaaaataaat tattaatttt tgttgatatt tagatgttta gttttgattt tgttttttat 4260
tgggtgatga gtatttttgg ggaagtaagt tttttttaat ttaagtatag ttattggtgt 4320
attgtagaaa atttaaaaag gagtggggtt gaggggtagg atggattgtt tgatatttgt 4380
atgttggagg gttgtaattt ttaggtttta tttatttaga aataatgttg aaaggattag 4440
gattggagat tttatgaggt aggtttaaat atgaagtaga aatggggttg tgcgtttttt 4500
ttagatgtat atatttggaa gagttatgta gtaagtagag gagaggaata gaggagttgt 4560
gtggtttgat ttttttcgga atttgtagtg tggttggttt tgtgtgtttt ttgtattgat 4620
tttatgattt acggagtatg agttaagtag aaagtatatt taaattatag aggagatagt 4680
tgtttgttat agaaggatat attttttggg tttaatggcg ttatattttt tttaatagtt 4740
tgtatattgg ggatgtattt attagtaatg tttgttaaag ggttttattt gttttttttt 4800
ttttgaataa aaaagatatt ttttaaaaaa attatttaaa tattattttt gattttattt 4860
ttataatagg attttttttt taggaaaata gtattaataa gtaatttgtt attatgggtt 4920
gttttttttt tttttatttt ttgatagtaa gggtttatcg gaagtttaaa gtaatgtagc 4980
gggaagtatt ttgttttttt gaagcgaata gcgttttaga gatattgggc ggtttaaggt 5040
tagttttttt gttggaattc gtttgttgtt tagtgcgatt ggaattgtat ttttttttgt 5100
ttttttttta aatttgtttc gtttttatat gatagaggtt gaagttttta gatgttgttt 5160
ggattaaaga gtgtgtgtgg ggaggggggg aaaagttttt ttgatgataa taatagagtt 5220
agattggttg gttttgtatt gataggaaaa tggagagata aaaattttat taatggtgtg 5280
tgtattgttt aagtagttaa taaaataatg ttgtttttat tggaagtcgg ttttagattg 5340
gtaacgtttc gtgttaattg taggagataa tagttgtaaa gaggggtaga gaattcgagt 5400
taagagttta cgtttaatga ttggtttgcg ggtagttttt tttgtttttt attttttttt 5460
tttttttttt tttttttttt tttttatttt ttgtttattt tttttgatga gtttttttta 5520
atgtttattg ttttagaaat tgaattatat ttgattaaaa tttaatggtt atttatagaa 5580
tgaagttggt tggtagtttt taagtttgtt tgtttgggtt gttttgagtg tgtgataata 5640
gaaattgtta ttttgaataa aatgtatttg agggttatag tttttaaatt ttgatagaat 5700
agttatttat tatagatgta tcgttttatt tttttttaaa tatttttaaa ttttcgtgtt 5760
cgaaattaaa ttaaaaaatt taaaagtttt ttttaaaaaa gtattttatt ttagaagata 5820
atggaaaatg gtaaaggttt gttttagtta gtttttattt tatatgtatt ttttgaattc 5880
gttttttgag tgaagttttt ttttgtgatt gtgggttagg taaggttgtg tttagacgag 5940
atttgatttt gggaatttat tgttttatgg ttaggtaaat ttttcgattt ttaaagggaa 6000
agaaagatat gttgtttgag tgatttagaa aagtattttt gtaaatttaa aagagagatg 6060
agttgattat ttagttttgt ttttgttata tgtgagtgta aaatttaaat tattttgatt 6120
tttatttatt attatatatg gggaagggaa aagatatttg agtgtttatt tttgtgttat 6180
attttgtgtt atgaatttta tgtatattag gaagtaggta tttgtagttt tattttatag 6240
gcgagagaaa cgtaaatttt aaggagttta tttatttaag gttgtaaagt ttgttttttt 6300
tgtttatggg tgtagatgta atgaagtata tgatatgaat taatttattt ggtatttatt 6360
ttatttaatt atagtagata tttagaagag gagatggagt gtttttattt taggtttttt 6420
agaatgttgg tgttgtgggg taagggtata tattattttt aaattttgga agttttataa 6480
attttggaag gttttataga ggaggagaga tattttttta ttttgtagta tatttttttg 6540
agtgtgttta ttgtgttagg tatttcggta gggattgggg atttagtgtt aaagaggata 6600
aagttttttt gttgttagaa ggtggtgatg attttttggt tgggaaagtt atgggaaggg 6660
agaataaggg tgggtattta agtattttgg aggagatgaa ttttgagtaa agtatttggc 6720
gaagagttta gttgggagtg acgtttgttt tggttttttt tttgaaatgg aggattttgt 6780

gtgtagatat aaggtggtgg ataaaatgtg tagggaggag taataaggaa tgtttttagg 6840
tataatttgt gttttttttt tggaaatttt atgtagattg tgagttgttt taaggttttt 6900
ggttgcggat tttttatttt taggttatat taagtggtta gtggttatag tattttaagt 6960
ttggtttttt tttttattat taaagatggt aaagtaaggt tacgggtaaa ttttgttttt 7020
atagataaag tttttatgtt aggtattttt aaaaaagtat ttgttttttt tgatatagtt 7080
ttaatttata tagtaaagta gagagtttgt tttgaggaaa tacgattttt tatgatgaat 7140
attagatatt gaatggtttt taatatgtta tgtatgtatt attgtgttag tttgtttttt 7200
tttaaatgtt aggtcgttat tgttaatttt atatgtttaa aagatttggg tgaattttag 7260
gtataatatt ttggttttta tttttatatt ttatagtagt tgaaaggttt tatggtattt 7320
ttgatagatg tttgtttgcg tttatattga tgaagaatag tagagtatat taaattaaag 7380
ggttgttggt tggttgtttg gtagtgattt gttttttgtt ttttgtttat tagaggatgt 7440
ttggaggatg taatttgtat attggttatt aatgtgaagg tttttttttg ttttgttagt 7500
aaattttatt ggttaataga tttttgtttt taattattgg tttttagttt tattgtgggg 7560
tagagttata ttgtagtgta ttgtgagtat tgttaagtaa tttattattt attattgagg 7620
taaagaaatt ttgatatacg ttggagttga tttagtataa tattttttta tgtgttttat 7680
tatgtttttt agagtgagaa aaaaatggaa tttaaagata agttaaagaa ttgtatgttt 7740
tagttagtaa aatatattat taagaaaaag aaaaagtgtt aaggaaagaa atgagtatta 7800
taaaagttag tattatggtt ataagagtat taataatatt aataatttat tttttaatgg 7860
tgtttatatt atgttttgtt gtgagtgttt tatatatatt aatttattta atttttagga 7920
taattttgtg aggtaggtat aatttttaat tgaaattttt tagatgaagg gaatgaggta 7980
tattaagtta gtttttgagg ttttaaagat agcgtatggt aggatcgtag ttttaattgg 8040
tttagttttg ggttttaatt atagtgtagg ggaggaaggg ggtatagtgg ttgaggatat 8100
atgggatgtg gtgggatgtg gagtgttggt agttttttaa aaaggaaata ttaggatttt 8160
tattaaaggt tttagttgta tttttagttt atatggattg ttttagttta tttgggttat 8220
tgtgataaaa ttttagaggt tgtgtagttt ataaagattc gacgtttatt ttatatagtt 8280
ttggaggttg ggtagtttaa gattaaagta ttggtagatt tagtgtttgg cgagggtttt 8340
ttaggttata gttggtattt ttttgttttg tttttatatg gtggaaggga taaggtagtt 8400
atttggggtt ttttttataa ggggattaaa tttatagggg tatttatttt acgaggtttt 8460
gtttttatga tttaattatt ttcgaaagtt ttatttttta atgttatttt attggtgatt 8520
aggttataat atatgaattt gagaggatat aaatatttag attatagtat ggattgaata 8580
gatatagtta tatattatat gtttttatat atgtatatat gaatatattt atttatttga 8640
tatatatttg tatgaattta tattgtattt gagaaatggt agtataaatg gtatatatgt 8700
gtgtgatata tatagttttt tatttttgtt gtttgatgta tttatataat aaattttata 8760
gttgggattt acgtatttat ttttagagaa agagtatatt tatatatgga agtttttttt 8820
aattttggaa tgtttttatt tttgtgtagt atttatattt atttgttttt taaaaaaaaa 8880
ttattaggtt gggtatggtg ggttacgttt ataattttag tattttggga ggtcgaggta 8940
ggtagattat ttgaggttag gagatcgaga ttattttggt taatacggtg aaatttcgtt 9000
tttgttaaaa atataaaaaa ttagttgggt atggtggtac gtatttgtaa ttttagttat 9060
ttaggaggtt gaggtaggag aatcgtttga attcgggagg tagaggttgt agtgagtcga 9120
ggttacgtta ttgtatttta gtttgggcga taggtgattt taaatttaat ttaaaattat 9180
atttaaaaga aaatagtttt tagatatgta tgtgatttgt tttttgtatg aataaagtat 9240
tattaaaaaa ttggaaggat attttatggt tgtgttaaat tagtttaaat ttggttagaa 9300
gttttaagag atttagttga atttatgtta gtatgtgttg ttttaagttt gagtgtatag 9360
tagtgttggt agaattaata aaagtgttgt atatgaaata attaaaatgg atttttaaaa 9420
aagttgttat ttaagagtta tttttattaa tatttaacgt atttaaaatt aaaattaata 9480
ggagttagtt tttttttttt tgatagtgtt agaaaataag ttattttaaa atttatagaa 9540
gattattatt ttttttttgg atttagaaag taaaaaaaaa aaataaaatt aggtagataa 9600
atgtttatga aatggattaa ggtttagtga tttgaaattc gttgaatttt ttttgtaatt 9660
gttgatagtg tgaatggtaa atagagataa ttgttaacgt tttatttttt ttttatatgt 9720
gtgttgtatt tttatgtttt attatattga agtaattttg aaagtattaa ttgtttttta 9780
ttatgttttt tttagtgata tttaattttt agttgtttat ttgagttatt tggagagttg 9840
ggataatggt tattttttat ttttattaga gagtattttt aggtattttt aatgtatagt 9900
taagggtgag aataattgag tttatttagt tttttaattt tggggttttg ggtaatgttt 9960
gtttgttttt tttttttagt ttatagagtt taagtatatg ttggatatta ggatatataa 10020
agatatagga agttaatttg tgtagtatgg agatagtttg ttaatttatt ggtttttgga 10080
ggaatttttg agagaattta gttggttgta gtattatttt tatttgagga aggatagggt 10140
tgataatttg ttagttttta gttttttttg aattgttttt gaagggtaga aatgtgtgat 10200
aaaaggttta tatatgtgtg ggtttatata tatatatttt ttttattgtt gtttggataa 10260
atagagtata ttttaaatga gaatggtgtt aaaatggtta tgaagaatta attttatatt 10320
agaaattttg gtttaaattt tgggaatttg ggttattgat aaatatagat tgtattaatt 10380
gtcgggtaag ttgtttttta agagttgtaa tattataaat gattaaaaag ttgatgtttt 10440
aatttgtgtt tatgtaaatt aattataggt gtggaatttt gaggtttgtg aagaaaagat 10500
ttttaatagt taaatttagg tatgttttat gttatagaat taatttataa aaaatgaatt 10560
ttgatgtaaa gttatgtgtt ttaattaata gtaatttaaa ttaagaggtt aaaattttat 10620
tagtggattg aatgttgagt gatatttgag gagagtaaag tatagaaaat gtttttgttt 10680
tgtggttatt tatataaaag gttttgtatt aaagagttta gagatttatg tattatagat 10740

aaggttattt tgataatagt atataatttt gggagtagat atatttttat ttttttattt 10800
tatattattt ttattgtaaa gtattattgg aattttaaag aggattttag gtaggttggt 10860
tttagttttg atttttgatt tgttattatt ttttttcgtt tttgtttttt ttgttagttt 10920
tttttgatat tttacgtgtt ttttggtaga ggttgtttta ttttaattat ttaattttat 10980
tttaattatt tgttatttat taatgttagt ttgaagttgg cggtgatttg tagattttta 11040
gtagtagttt ttatttgatt ggggggtggt gtttgtttgt tttatagatg ggatataggt 11100
agagagtaat gtgtttaaga tattaatgtt tgggaagtaa tatgtttagg tttatttttt 11160
aagttgtgcg attttagagt ttttgttttt tttataaaaa ttaattggga gataagttgt 11220
gtttaggtgg tgaagttagg tttaggagta tagtttagta tgtattcgag gtttaagttg 11280
tataaaaaag gttgtaagat tttttaaatt gatttttaaa ataatagttt tattgagtta 11340
gtttatatat taaaaatatt tattttttta taatgtatta tttagtggtt tttagtgtag 11400
ttatagaatt gtttaattga tattattatt taatgttaga atatttttat tattttgata 11460
gagaaattat tagtatgtaa gattaatttt aaagttaata ttattggagt agaagttaag 11520
gattgggttt ttttgattat tttgtgaatt gagtgagggt agaaattagt ttttggttta 11580
attttgtagt gggtatagag tagatattag tgttgatttt tatagaattt tttagaatag 11640
tgaattttta atttttgaaa ttttaattta gttttaattt tggtaaatta aaaaatttta 11700
ttttattttg tttatttgtt ttatgaaaat aataggattt aaaattgttt tttaagtatt 11760
tatttgtttt ttgatttttt tcgggtagat ttgttatttt tattattttg attggagagt 11820
tattggtatt ttaagatttt tgggttattt ttattgtagg tttgtttgaa tatatgaaat 11880
gatttagtgt agttatgagt ttagatgatt tacgtattaa gtaattttat gtgtaaaagt 11940
gattttagtt agtaaatgtt aataatagtt atttattatt ttttggattt ttttattttt 12000
ttttttaata tttgtttttg gttattgttt tttttttgtt atttttttta tgtttattaa 12060
agtaaattga ataatattat atagtaatgt gtgttatttg tttttttttt ttagtgtgat 12120
ataaatagtt tggggttgag aaaatgggta gtggggtggg gaaaaatttt taagaaggta 12180
aaggttaggt gtggtgtttt atttttgtaa ttttagtgtt ttgggaggtt aaggtgggag 12240
aattgtttga gattaggaat ttaaggttat agtgagttat gtttttgtta ttttatttta 12300
atttgagtaa tagagtgaga ttttgttttt aagataaaaa atgtagtaaa tgtaatattg 12360
ttatggtgag aggaagaata tttttttttt ttatttgagt ttttttagtt attttgattg 12420
ataatatttg gaagtaggtt agttagtgag agtttagtta agggaattgt atgggggtat 12480
gagtttaagt gattattttg ggaataattt tttgggaaaa aattatattt tttttttttt 12540
tttttggtga aataaagtat tttttttttt atattttatt gataataatt tagtttttag 12600
taatattata ttgttagtat ggtatataag tattttaagt tttgtttttt ttttttgtgt 12660
gtatataaaa tttagaaaaa agattatgtt ttaaaatgtt agagtaggga atattttgtt 12720
tttaaatgta agttataagg aagttgtggt tgagtatata tatttagttt atatattgtt 12780
tttttgtatg aaattagaaa cgttggtagt tttttttgtt tataatttag ggtttgggga 12840
agtttgtttt gttttttagt ttgagttaaa agttatgata atattgaaaa tgggttttag 12900
taggtttatt tttttttttt tattagtatt attatttttt ttttattttt taaattgttt 12960
atattttttt tggatggtat tttttttgtt ttgaatttat tattagggta tattatttat 13020
tattaatatt tatgaagggt tttttattta attttatttt gagacggagt ttgtttttgt 13080
tatttaggtt ggagtgtaat ggtatgattt cggtttattg aaattttcgt tttttgggtt 13140
taagtaattt ttttgtttta gttttttaag tagttgggat tataggagtt tgttattata 13200
tttggttaat ttttgtattt ttagtataga tggtatttta ttatgttggt taggttggtt 13260
tcgaattttt gattttaggt gatttatttg ttttggtttt ttagatagag tgttgggatt 13320
ataggcgtga gttattgcgt ttagtttttt ggtttattgt ttatgattat ttttgatgtt 13380
ttataggtga aaaatatttt tattttgaag agtttataga tttattaggg aaatagtaaa 13440
atttaaaaat taaaatgtat ttttaagggt tataatagtt aagtttggga aaaatagatg 13500
agggggtatg tgttaatatt ttagatagga tgtagatatg ggggttttgg gaggtgatgt 13560
tggggttgat tttgaagtat aggagtttgt tagatgaaaa aagatatttt aggtagataa 13620
gtgtaaaggt agaaggtgaa tatggttatg gtgtttttag ggagttgtaa gatataatta 13680
gggtgagggt atttatgaga tgataattcg atgatgttgg agatgtttgg ggttagatta 13740
tgaaaattaa gaaatttata ttagttagga gaattattga tggttttaag tagaggagag 13800
gtattagtga gcgttattta atgaataatt tattatattt atggtaggga tgtagaggag 13860
attttgaagg agttagttgg gttattgttg gtgggagagg ttttgggttg ggttgtaggt 13920
agcgatgggg tttgtaaggt aaatttggtt tttggttttg tttataagat tttttagtgg 13980
ttttattgta gaagatagga tataaagaaa gattagtaga tttgggatgg tgggtgatat 14040
ggataggatg tttttagttt tgggaatttg gatgtatttt tttgtttttt tgagagattg 14100
tttttggtag atttattgtt attgtatttt tgtttgtata ttttcgtatt tagttatttt 14160
ttaattggag agtaaatgat tggtgagtaa attaaattaa aagtaaattt tagttagata 14220
tagtggttta cgtttgtaat tttagtattt tgggaggttg aggtaggtgg attatttgag 14280
gttaggagtt cgagattagt ttggttaata tggggaaatt tttattttta ttaaaaatat 14340
aaaaattatt tgggtatggt ggtgggtgtt tgtaatttta gttatttagg aggttgaggt 14400
ataaaaatcg tttgagttcg ggaggtagag gttggagtga gttaagattg tgttattgta 14460
ttttagtttg ggcgatagag tgagattttg ttttaaaaaa taaataaata aataaaaaaa 14520
taaaaaatta atttttgagt taagaatgaa attgttaaat taaataaaaa gtattatgtt 14580
atttttatat tttttttttt aattagtggg ttgggtgtta tcgtagtata gaggtagatt 14640
ggaaagttat ttttttttgg aaatagagtt ttgttatttt tttgttgttt aggttggagc 14700

gtagtgatat gattttagta gtttttgttt tttaggttta agtaagtttt ttgttttagt 14760
tttttgagta gttgggatta tagggattta ttgttatgtt tatttaattt tttttttttt 14820
attttttttg agacggagtt ttgttttgtt gtttaggttg gagtgtagtg gcgtgatttc 14880
ggtttattgt aagtttcgtt ttttaagttt aatcgatttt ttcgttttag tttttcgagt 14940
agttgggatt ataggtattc gttattatgt ttggttaatt ttttttgtat ttttgtagag 15000
acggggtttt attatgttgg ttaggttggt tttgaatttt tgattttagg tgatttgttt 15060
gtttggattt tttaaaatgt tgggaatata ggcgtgagtt ttagcgttcg gttttttgta 15120
tttttttgta gagacgaggt tttgttatgt tgtttagaaa atttttgggt ttaagtaatt 15180
cgtttatttc ggttttttaa agtgttggga ttataagcgt gagttattat atttggttta 15240
aaaatagtat ttttaattaa agaatatttt ttgttgttag tataattttt gaggaatttt 15300
agtaataatt atgaaagaaa tatttaagaa gaatttttga gttagaagga aacgtagtga 15360
tttgggttat tgtttttttt ttatagttag gataaaagga taatagagaa gggtgagtga 15420
gaagggttag tgatttttga gtgttatata gtagtggtgc gggggtatgt ttagtttatc 15480
ggtttagtgt tatttttatc gatttggatg attttttgtt tagttttata gagacgatag 15540
agttgagttg aaagatatta gtgtttaaga ttattaagtt agcggtagat ttagagttgt 15600
ttaaattttt gattttaatt ttattttaaa ttaatttatt gtttataggt taataagtat 15660
ttgttgattg gttagtatgg aagggatatt attttaatta ttatataatt agagataagt 15720
aagagattat tttttttttt aagaagtgtg taggttaatt ttttagatta atttattata 15780
ggattggtaa atatttatat aatttatggt ttgtttgata taattatggg tatgtagtat 15840
atatatatat aaaattaatg agaaaacgag gttatataaa atattgttta tttttttttt 15900
tttttttatt taattttatt ggattttgtt tttttttgtt tttttgtttg ttattttgtt 15960
ttttttgtat agtgggtaat tagtttttag attagtttta gaaatgttta tatgttggtg 16020
ggtaaataat aaatagaatt aaataataaa aaataatatt gattgttttt tttttagttt 16080
atttttgttt aattataatt aggaagtatg atttggtgaa aatttttttt aggttggtaa 16140
tgatataaat atttgtagga aggtatttgg tattttttgt tattgttttg aatttaagtt 16200
tagggtagaa atttagttat ttttatttta gaaatggtaa gagtgataga gaagtttttt 16260
tttttttatt aagtttatga gtttgaaatt attaatgttt tttgtttttt tttttttttt 16320
tttttttttt tttgtttttt tttttttttt tttttttttt tttttttttt ttttttttta 16380
tttttttttt tttttttttt tttttttttt tttttttttt tttttttttt tttttttttt 16440
tttttttttt tttttttttt tttttttttt tttttttttt tttttttttt tttttttttt 16500
tttttttttt tttttttttt tttttttttt tttttttttt tttttttttt tttttttttt 16560
tttttttttt tttttttttt tttttttttt tatttttttt tttttttgtt tttttttttt 16620
tttttttgat agg 16633

<210> 2
<211> 16633
<212> DNA
<213> Artificial Sequence

<220>
<223> chemically treated genomic DNA (Homo sapiens)

<400> 2

tttgttaaaa aaaaaaaaaa agaaataaaa gaaaagaaag ataaagagag agaaggaaag 60
gaaaggaaag gaaaggaaag gaaaggaaag gaaaggaaag gaaaggaaag gaaaggaaag 120
gaaaggaaag gaaaggaaag gaaagggaaa gggaaaagga aaaggaaagg aaaggggaga 180
ggggagaggg gagaggggaa aaggaaaaga agaaggaagg gaaaggaaag gaaaggaaag 240
gaaaggagga aggtaggaaa agaaaggaag gaaggaaggg aaagaaagaa agagagaaat 300
agagagaaag agaaagaaag aaaaaaaaat aaaaagtatt aataatttta agtttataaa 360
tttggtaaag aaaaagaaat ttttttatta tttttattat ttttagggta gaaatggtta 420
aatttttatt ttaggtttaa atttaaagta gtagtagaaa gtattaaata tttttttgta 480
agtatttatg ttattattaa tttaagaaaa atttttatta gattatgttt tttaattatg 540
attgaataag aataaattga aaaaaagata attaatatta ttttttatta tttggtttta 600
tttattgttt gtttattagt atgtaggtat ttttaaagtt agtttagaaa ttgattgttt 660
attgtgtaga gagaataagg taataagtaa aaaaataaaa gaaaataaaa tttaataaag 720
ttaggtaaaa aagaaaagaa aaatgaataa tgttttatgt ggtttcgttt ttttattggt 780
tttgtgtgtg tatatattat atatttataa ttatgttaga taagttatga attgtataag 840
tgtttgttag ttttgtaatg agttaatttg aaaaattaat ttgtatattt tttgaaaaga 900
aaaatagttt tttatttatt tttgattgtg taataattgg ggtagtattt tttttatatt 960
aattagttaa taaatatttg ttggtttatg aatagtgagt taatttaaaa taagattaaa 1020
gttaagaatt taggtaattt tgggtttatc gttgatttgg tggttttgag tattagtgtt 1080
ttttaattta gttttatcgt ttttatggga ttgagtaggg agttatttag gtcgatggag 1140
gtaatattga atcgatgagt tagatatgtt ttcgtattat tgttgtgtga tatttagaag 1200
ttattaattt tttttattta tttttttttg ttattttttt attttaatta taaagaaaga 1260
gtagtggttt aagttattac gttttttttt agtttaaaga ttttttttag gtattttttt 1320

tatgattgtt attaggattt tttaagaatt atgttaatag taaggggtat tttttaatta 1380
aaggtgttgt ttttgggtta ggtgtagtgg tttacgtttg taattttagt attttgagaa 1440
gtcgaggtag gcggattgtt tgagtttagg aattttttgg gtaatatggt aaaatttcgt 1500
ttttataaaa aaatataaaa ggtcgggcgt tggggtttac gtttgtattt ttagtatttt 1560
gggaggttta ggtaggtaga ttatttgagg ttaggagttt aagattagtt tggttaatat 1620
ggtgaaattt cgtttttata aaaatataaa aaaaattagt taagtatgat ggcggatgtt 1680
tgtaatttta gttattcggg agattgaggc gggagaatcg attgaatttg ggaggcggag 1740
tttgtagtga gtcgagatta cgttattgta ttttagttta ggtaatagag taagatttcg 1800
ttttaaaaaa aataaaaaaa aaaaaattag gtgggtatag tagtgggttt ttgtagtttt 1860
agttatttag gaagttgagg taggaggttt gtttgagttt ggaaggtaga ggttgttgag 1920
attatgttat tgcgttttag tttgggtaat agagggatag taaaattttg tttttaaaaa 1980
aagatagttt tttaatttat ttttgtattg cgatggtatt taatttatta attaaagaaa 2040
aaaatgtaaa aataatataa tattttttat ttaatttagt agttttattt ttaatttaga 2100
gattagtttt ttgttttttt gtttgtttgt ttgttttttg agatagagtt ttattttgtc 2160
gtttaggtta gagtgtagtg gtataatttt ggtttatttt aatttttgtt tttcgggttt 2220
aagcgatttt tgtgttttag ttttttgagt agttgggatt ataggtattt attattatgt 2280
ttagataatt tttgtatttt tagtagagat gggggttttt ttatgttggt taggttggtt 2340
tcgaattttt gattttaagt gatttatttg ttttagtttt ttaaagtgtt gggattatag 2400
gcgtgagtta ttgtgtttga ttgaggtttg tttttaattt aatttgttta ttaattattt 2460
gttttttaat tgaaggataa ttgagtacga aaatgtataa atagaagtat agtaatagtg 2520
ggtttattaa gaatagtttt ttaagggagt aaaagaatat atttaggttt ttaagattga 2580
aagtattttg tttatgttat ttattatttt aaatttatta attttttttt atgttttatt 2640
ttttgtaatg gaattattga agaattttgt agataaagtt agaaattaag tttgttttat 2700
aaattttatc gttgtttata atttagttta ggattttttt tattagtagt ggtttaattg 2760
atttttttaa ggtttttttt atatttttgt tatgagtgta gtaaattgtt tattgagtaa 2820
cgtttattag tgtttttttt ttgtttaaag ttattaatgg tttttttagt tggtgtagat 2880
tttttgattt ttatgatttg gttttagata tttttaatat tatcgagtta ttattttata 2940
ggtattttta ttttaattgt attttatagt tttttgggga tattatgatt atgtttattt 3000
tttgtttttg tatttgtttg tttgaaatgt ttttttttat ttggtaaatt tttatgtttt 3060
aggattagtt ttagtattat tttttaaagt ttttatgttt atattttgtt tgaaatatta 3120
atatatgttt ttttatttgt tttttttaaa tttaattatt gtggttttta aaagtgtatt 3180
ttaattttta aattttatta tttttttggt gagtttgtgg attttttaaa gtaagagtat 3240
tttttattta tgaaatatta aggatagtta taaataatag gttaagaggt tgggcgtagt 3300
ggtttacgtt tgtaatttta atattttgtt tgggaagtta aggtaggtgg attatttgaa 3360
gttaggagtt cgagattagt ttggttaata tggtgaaatg ttatttgtat taaaaatata 3420
aaaattagtt aggtgtggta gtaggttttt gtaattttag ttatttggga ggttgaggta 3480
ggagaattgt ttgaatttag gaggcggagg ttttagtgag tcgagattat gttattgtat 3540
tttagtttag gtgataagag taaatttcgt tttaaaataa aattaaataa aaggtttttt 3600
ataaatgttg ataataaatg atgtattttg ataatggatt taaagtagaa aagatattat 3660
ttaagagaaa tatgaataat ttaaaaaatg aaaaagaaat aataatgtta atggaaaaaa 3720
ggaggtagat ttattggaat ttatttttag tgttgttatg gtttttaatt taaattaaaa 3780
aataaaataa atttttttaa attttaagtt atagataaag agaattatta acgtttttga 3840
ttttatataa aaagataatg tgtaaattaa gtgtatatat ttaattatag tttttttgtg 3900
atttatattt gaaaataaag tattttttat tttaatattt taggatataa tttttttttt 3960
aaattttata tatatataga gagagaaaat aaaatttgaa atatttatat attatgttaa 4020
taatgtaata ttattggaaa ttaaattgtt attaataaaa tgtaagaagg gagatgtttt 4080
gttttattaa aaaaaaaaaa aaaaaatgta attttttttt agagaattat ttttaagatg 4140
gttatttgga tttatgtttt tatataattt ttttggttaa gtttttattg gttggtttgt 4200
ttttagatgt tgttagttaa ggtgattgag ggagtttaag taggaaaaag aagtgttttt 4260
tttttattta tgataatgtt atatttgtta tatttttttgt tttagaaata gggtttttatt 4320
ttgttgttta agttggagtg gagtggtaga aatataattt attgtagttt tgaatttttg 4380
gttttaaata atttttttat ttttagtttt taaagtatta ggattatagg aatgagatat 4440
tatatttggt ttttattttt ttaaaggttt ttttttattt tattatttat ttttttagtt 4500
ttagattgtt tatattatat tgggaagaaa agataaataa tatatattgt tatataatgt 4560
tatttaattt attttaataa gtatgaaaaa ggtaatagaa aggaggtaat ggttaaagat 4620
aaatgttaaa gaaaagagta ggaagattta aggaataata aatggttgtt attagtattt 4680
gttggttggg attattttta tatatgaaat tgtttaatgc gtgagttatt tgaatttatg 4740
attgtattga attattttat gtgtttaagt aaatttgtaa tgaaaataat ttagaagttt 4800
tgagatgtta atgatttttt aattaaaata atgagggtgg tagatttatt cgagaaaaat 4860
taaaaaataa ataggtgttt agaaaataat tttaaatttt attgttttta taaaataagt 4920
gggtagagta aagtgagatt ttttagttta ttaaaattgg gattgggtta aaattttaaa 4980
agttgagaat ttattgtttt ggaggatttt gtgagagtta atattggtgt ttattttgtg 5040
tttattgtag ggttgggtta ggaattggtt tttattttta tttagtttat aggatgatta 5100
ggagagttta atttttggtt tttattttaa taatgttagt tttaaaatta gttttatatg 5160
ttaatagttt ttttgttagg gtggtgaaaa tgttttaata ttagatagtg gtgttagttg 5220
aatagttttg tggttatatt aaaaattatt gaatagtata ttataaaagg gtgaatgttt 5280

ttggtatatg aattaattta ataaaattgt tattttaaaa attagtttga aaggttttat 5340
agtttttttt gtataattta gatttcgagt atatgttggg ttgtattttt ggatttagtt 5400
ttattatttg gatatagttt gttttttagt taatttttgt gaaaagagta aaagttttgg 5460
aatcgtatag tttgaggaat gaatttaggt atgttatttt ttaggtattg gtattttaag 5520
tatattattt tttgtttgtg ttttatttat aaaataggta gatattattt tttaattaga 5580
tgagaattat tattgagaat ttgtaaatta tcgttaattt tagattaata ttagtaaatg 5640
gtagataatt aaaatgaaat taaataatta aaatgaaata gttttattta aaggatacgt 5700
gaggtattag aagaaattaa taggaaagat aaaaacgaga aaggatgatg gtaagttaag 5760
agttaggatt gaggttagtt tatttggaat tttttttggg attttagtga tgttttgtag 5820
taggggtgat atggggtggg aagatgggga tgtgtttgtt tttagaatta tgtgttatta 5880
ttaagatgat tttgtttatg atatataaat ttttggattt tttaatgtaa gattttttat 5940
atgagtaatt ataaaataga gatatttttt atattttatt ttttttaagt gttatttagt 6000
atttagttta ttaatggaat tttagttttt tgatttgggt tattattaat tagaatatat 6060
agttttgtat tagaatttat tttttataga ttaattttat aatataaaat atgtttgaat 6120
ttgattgtta ggagtttttt ttttataagt tttaagattt tatatttgtg attaatttat 6180
ataagtatag gttgaaatat tagttttttg attatttgtg atattgtaat ttttaagaag 6240
taatttattc gataattaat ataatttata tttattaata atttaaattt ttagaatttg 6300
ggttagaatt tttaatgtga aattaatttt ttatagttat tttaatatta tttttattta 6360
aaatgtgttt tatttattta agtaataatg aaaggaatat gtgtgtataa atttatatat 6420
atataagttt tttgttatat atttttattt tttaaaagta atttaagaag agttagagat 6480
tagtaaattg ttagttttat ttttttttag gtagaggtga tattatagtt agttgaattt 6540
ttttagaagt ttttttagag attagtaagt taataaattg tttttatatt gtataggttg 6600
gttttttgta tttttgtata ttttgatgtt tagtatatat ttgagttttg tggattgaag 6660
aggaagggta gataagtatt atttaaaatt ttagggttaa aggattaaat gagtttagtt 6720
gtttttattt ttggttgtat attaaaagtg tttaagagtg ttttttggtg gaaatggagg 6780
atggttattg ttttaatttt ttaggtgatt taaatgagta gttgggagtt gagtattatt 6840
gagagagata tggtaagaaa tagttaatat ttttaggatt gttttaatat gatgggatat 6900
gaaagtataa tatatatata aaaaggaaat aaaacgttag taattgtttt tgtttattat 6960
ttatattgtt aataattata agaaaaattt aacgagtttt aggttattaa attttagttt 7020
attttataaa tatttgttta tttgattttg tttttttttt ttatttttta aatttaagag 7080
ggaaatgata attttttgta agttttagaa tggtttattt tttaatattg ttaggaagga 7140
aaaggttagt ttttgttagt tttaattttg aatgcgttaa gtattggtgg aggtaatttt 7200
tagataatag tttttttaaa aatttatttt agttgtttta tgtgtagtat ttttgttggt 7260
tttgttagta ttgttgtgta tttaggttta agataatata tattagtatg aatttaattg 7320
agttttttag aatttttaat taaatttaga ttggtttaat ataattatga ggtatttttt 7380
tagtttttta ataatatttt gtttatataa agggtaggtt atatatatgt ttagaagtta 7440
ttttttttta aatataattt tggattaaat ttaaaattat ttgtcgttta ggttggagtg 7500
tagtggcgtg atttcggttt attgtaattt ttgtttttcg aatttaagcg atttttttgt 7560
tttagttttt tgagtagttg ggattatagg tgcgtgttat tatgtttagt taattttttg 7620
tgtttttagt agagacgggg ttttatcgtg ttagttagga tggtttcgat tttttgattt 7680
taggtgattt gtttgtttcg gttttttaaa gtgttaggat tataggcgtg atttattatg 7740
tttagtttgg taattttttt ttaagaggta aataaatgtg aatgttatat agaagtaggg 7800
gtattttagg gttgaaaaaa gtttttatat gtgaatatgt ttttttttta gaggtagatg 7860
cgtaggtttt aattgtgggg tttattgtat gaatatatta gatagtaaga atagagagtt 7920
atatatatta tatatatatg tgttatttat attgttattt tttaaatata gtatgaattt 7980
atgtagatgt atattaaata aataaatata tttatatata tatatataaa aatatatagt 8040
atatgattat atttatttaa tttatgttat ggtttaaatg tttgtatttt tttaaattta 8100
tatgttgtaa tttaattatt aatgggatga tattaggaag tgaggttttc ggaggtgatt 8160
aaattatgaa agtagggttt cgtgggatga gtgtttttgt ggatttagtt tttttataag 8220
agagatttta gatagttatt ttattttttt tattatgtga agatagagta agaagatgtt 8280
aattatgatt taagaagttt tcgttagata ttgaatttgt tagtgttttg attttgagtt 8340
gtttagtttt tagaattgtg taaaataaac gtcgggtttt tataagttat atagtttttg 8400
gaattttgtt atagtagttt aaatggatta aaataattta tatagattaa aaatgtaatt 8460
aaaattttta gtaagggttt tgatgttttt tttttaggaa attgttagta ttttatattt 8520
tattatattt tatgtgtttt taattattat gttttttttt ttttttatat tgtggttaaa 8580
atttagaatt gagttagtta gaattgcggt tttgttatgc gttattttta aggttttagg 8640
agttaattta atgtgtttta ttttttttat ttgaaaaatt ttaattaaaa attatatttg 8700
ttttatagaa ttattttaag aattaaatga gttaatatat gtaaagtatt tataatagaa 8760
tatgatataa gtattattaa agggtaagtt attaatatta ttgatgtttt tataattata 8820
atgttgattt ttgtgatatt tatttttttt tttaatattt tttttttttt ttaatagtat 8880
attttattaa ttaaaatata tagttttttg atttgttttt gagttttatt ttttttttat 8940
tttgaaagat ataatggaat atatgggaga gtattgtgtt gaattagttt taacgtatgt 9000
taaaattttt ttattttaat aatgaataat aaattattta gtaatattta taatgtattg 9060
tagtgtagtt ttgttttata gtagggttga aaattagtgg ttgaaaataa ggatttgtta 9120
attagtaggg tttattgata gaataaaggg aaatttttat attgataatt agtatgtaga 9180
ttatattttt taagtatttt ttagtgaata gaaaatagaa agtaagttat tgttaaataa 9240

ttagttaata attttttaat ttaatatatt ttattgtttt ttattaatat ggacgtagat 9300
aagtatttat taagaatatt atggagtttt ttaattatta tgaaatgtaa aaatgaaaat 9360
taaagtgtta tatttggaat ttatttaaat tttttaaata tataaaatta atagtgacga 9420
tttgatattt agaaaaagat aagttggtat agtaatgtat gtatgatatg ttaaaggtta 9480
tttaatattt aatatttatt atggggagtc gtattttttt agagtaggtt ttttattttg 9540
ttgtgtaaat taaaattgtg ttagaagagg taaatatttt tttggaaatg tttaatataa 9600
aggttttatt tgtaagaata aggtttgttc gtgattttat tttgttattt ttagtgatga 9660
gagaagaaat taggtttgag atattgtggt tattggttat ttggtgtggt ttgagagtga 9720
gaagttcgta gttaggaatt ttgagatagt ttataattta tatggggttt ttaaaaagga 9780
aatataggtt gtatttaaaa gtatttttta ttgttttttt ttatatattt tatttattat 9840
tttatatttg tatataaagt tttttatttt aggaaggaaa ttaggataag cgttattttt 9900
agttgagttt ttcgttaggt attttattta agatttattt tttttagggt gtttggatat 9960
ttatttttgt tttttttttt tatggttttt ttaattagga aattattatt attttttgat 10020
agtagaagga ttttgttttt tttagtattg agtttttagt ttttatcggg gtgtttgata 10080
tagtgagtat atttaagggg gtgtgttgta agataaagga atgttttttt ttttttgtga 10140
agttttttag gatttataag gtttttagaa tttaaaagta atgtgtgttt ttattttata 10200
atattagtat tttgaagggt ttagataaag aatattttat tttttttttt ggatatttat 10260
tgtaattagg tggaataaat attagatgaa ttggtttata ttatgtgttt tattatattt 10320
gtatttatga ataggaagaa tagattttgt agttttgagt aaatgagttt tttaagattt 10380
acgttttttt cgtttgtaaa atgaggttat agatatttat tttttaatat atataaagtt 10440
tatagtatag gatgtggtat aaagataaat atttaaatat tttttttttt ttttatgtat 10500
agtaatgaat aaaagttaaa gtaatttaaa ttttatattt atatgtaata gaggtagagt 10560
taggtaatta gtttattttt tttttgaatt tatagggatg tttttttaaa ttatttaaat 10620
agtatgtttt tttttttttt tggagatcga aggatttatt tgattatgag gtagtaagtt 10680
tttaaagtta ggtttcgttt gagtatagtt ttatttggtt tatagttata gaagagagtt 10740
ttatttagag aacgggttta ggaagtatat atagaatgaa agttgattaa ggtaagtttt 10800
tattattttt tattattttt taaaatgaga tgttttttta aaaaaaattt ttaaattttt 10860
taatttaatt tcgagtacgg aaatttaaag atatttgagg gaaaatagag cggtatattt 10920
gtgataagtg attgttttgt taaagtttaa ggattgtaat ttttagatat atttatttta 10980
aaataataat ttttattgtt atatatttaa agtaatttag ataaataggt ttggaaatta 11040
ttagttaatt ttattttgtg agtaattatt aagttttaat tagatgtggt ttaatttttg 11100
gaatagtgag tattaagagg ggtttattaa ggaaaatggg taaaagtagg aggagaagga 11160
ggaggaggag gagaggagga ggtagagaat aaaagaattg tttcgtaagt tagttattga 11220
acgtggattt ttgattcgaa tttttgtttt tttttgtag ttgttgtttt ttgtagttgg 11280
tacgaagcgt tgttagtttg ggatcggttt ttagtgagga tagtattgtt ttattggtta 11340
tttaggtaat gtatatatta ttaatggagt ttttattttt ttattttttt gttagtatag 11400
aattagttag tttgattttg ttattattat taagaaagtt tttttttttt tttttatata 11460
tattttttaa tttaaataat atttgggaat tttagttttt attatgtaaa agcgaaatag 11520
atttagaggg agagtaggag aaagtatagt tttagtcgta ttgaataata aacgaatttt 11580
aataggagaa ttaattttga gtcgtttaat atttttgaaa cgttattcgt tttaaaaagg 11640
taaggtattt ttcgttgtat tgttttaaat tttcgataga tttttgttat taagaggtgg 11700
gggaaaaaaa aataatttat agtgataagt tgtttattgg tattgttttt ttagaaggga 11760
agttttgttg tgaaataaaa gttaagaata atatttaggt gattttttta aagaatgttt 11820
tttttattta aaaaggaaaa aataataaag attttttagt agatattatt agtaagtata 11880
tttttagtgt ataaattatt aggaaaaatg tggcgttatt aaatttagaa aatatgtttt 11940
tttgtaatag atagttgttt tttttgtagt ttaagtgtgt tttttgttta gtttatgttt 12000
cgtgagttat ggaattaata taggaagtat atagagttag ttatattgta gatttcgagg 12060
agggttaagt tatatagttt ttttgttttt tttttttgtt tgttgtatgg tttttttaag 12120
tatgtatatt tggggagaac gtataatttt atttttattt tatgtttaaa tttgttttat 12180
aaggttttta atttggtttt ttttagtatt gtttttaggt aagtagaatt taaaaattat 12240
agttttttaa tatgtaggtg ttaagtaatt tattttgttt tttagtttta tttttttttg 12300
agttttttgt aatgtattaa taattatatt taggttagaa gggatttatt tttttagaaa 12360
tgtttattat ttagtagaag ataaaattag aattgaatat ttgagtgtta gtaaaggtta 12420
atggtttatt tttaatttat ttatttttgt tttatttagt aaaatagggg aaaattattt 12480
attgtttgag ttataggttt ttgtttattg gaagttgtgt ttagagtttt ttgtagtttg 12540
ttgttaatga tagttttagt aatttgattt ttttatagtg ggagttttga gtaagtattg 12600
gtttttttcg ggtttcgttt ttttattgtt ttattttgta gggttagttg tttttgatag 12660
ttaaattgtt gtatgttaag taatgaatgt tgttagataa ggaattttta tttttataat 12720
gtataggttt ttatggataa attatatagt ttatggtgtt ggttgtagga tgttgtttta 12780
agtttgaagt attgttaaaa tgaagtttgt tttttttttt ttttagtttt aggttgataa 12840
gatagttatt taaaatgaat ttgaatggtt tagggagaaa tggaatacgt gtttttagag 12900
ataaataaaa tatatgttga gaatagttgt ttagcgatat tttttgatgt aatggcgtat 12960
agggaaagtt aattgattta atgttatatt atataattat taggtatggt atttattatg 13020
atttattgta atttgatatt atgtagtgaa tattttaagg gtattaggaa aattattggt 13080
tttttgtttt tgggggatgt tagatttatt tagaaattat ataagaattt agtatttttt 13140
gagaattaag taatattgta tgaggtttta agaatagtgt tttttttatg agaatgaaag 13200

tttttaatat gtgttttatg gaattatttt gtagaaattt tatgtttttt tagttttttt 13260
tttttttttt ttcggtaata atttttgaat tgggtttggg atatattttt ttttattttg 13320
tatgttgagg tgtggttgaa aattaattat tatattgtat tggattttag ggtgtgacgt 13380
gatttgagtt tatttaatta gagtgatttt tgggatttta taggaattat tagaaaagaa 13440
agtgtgattt ttttattata tttgaatgta ggatatgtta tagttatttt gttattttga 13500
aaagagaatt tgttgaggat ggaattgata aaagaagaag tggaggggaa ataatagggt 13560
tttagggata ttgtttgagt tttgagttaa tttagattta aaatttgttt tattttggat 13620
tttatagaat ggtagttatt ttatgtttat tttattaagg ttcgtttggg ttggagtttt 13680
ttattatttg taaacgtaaa cgaacgtata aattattatt ttattattag agtatttagg 13740
aattaatata aatacgtata ttatatattg ttatgaaagt aagaaaaaag tttttttttt 13800
ttggttattt tttgtgagaa tttatttttt tatttttttt ttttagttta tatattttaa 13860
tgtgagttaa aagtgtaaag tgttatttga ttttggttat ttttgtaagg taaatttaga 13920
tatttgattt gaatttagag agatgaggaa gttaaattta tgatttgtta taaattttaa 13980
tttgatatgg tatatgtgga ataaagaaag tggattttgg ttagcgagtg gtttattagt 14040
tttatgttag tgaagttagt tgagaaaaag gttaggaagt tagtatgggg gtagggagta 14100
tttgtttttc ggttgggttt aggttagtta aagtataagt atttgtaggt tgtgagtcgt 14160
taaggcgatt acgcgttata ttttgtagtc gtttatttta ttattaatta ttgtttggtt 14220
agtaagggta gggttcgttt taagaatatt gggaatttta aggtaggagg tatatttaga 14280
attatggtgg gtttatagtt atttattata gattataaat agtttttggg ttgtgatttc 14340
gacggtaatt ataatttttg tttttttggt ggtaatagaa ttttaggttt tttgtttaag 14400
aaatttagta taatttatta ggtatggtaa aaattatgaa atgtatatat atgtttattt 14460
aatgttttgg ttattttttt tttgtttata tttggttttt aatttttaaa gattaaaaag 14520
tggggatatt aaagttgaaa aagatataaa taagatttta gtttgatgtt gagaaaaaaa 14580
aattatagaa tatataggtt taaaattgat ataggaaggt agggaggaaa ataagattta 14640
tttattttta attttttttt ttcgataatt atagggattt taaattttta tagttattat 14700
tttatttgaa gttataaggg gataggatga agtagggggg aagatttgtt ttgattttta 14760
atttgggagg agtaaaggaa gattaagaag gttattaaga ttgtttagat atgtatttta 14820
acgatcgtat ttatttagtt gttttttagg gataataaaa tgaaataaaa ttgttaggat 14880
ttatttaaat ttatagttaa agatttattt agagttgggt gtagtagtat atatttgtag 14940
agttagttat taggaaggtt aaggtaaaag gattgtttga gtttaaaagt ttaaggttgg 15000
aggttatagt gtattatgat tttgtttgtg aatagttatt gtattttagt ttggataata 15060
tggtaagatt ttattttttt ataaaaaaag aaaattggta gggtacggtg gttaatattt 15120
gtaattttag tattttggga ggttaaggta ggtagattat gaggttagga gatcgagatt 15180
atttggtta atatagtgaa atttatattg tatgaaaaat atttttaaaa aattagttgg 15240
gcgttgtggt aggcgtttgt agggttagtt atttgggagg gtgaggtagg agaatggtat 15300
gaattcggga ggtagagttt gtagtgagtt tagatcgtgt tattgtattt tagtttgggc 15360
gatatagaga gattttattt taaaaaaaaa aaaaaaaaaa ttaataatta atttaatttt 15420
ttgaatttag ggattatttt gttattttag ggggagttat tttagggaaa tattttttgt 15480
tataattatt gttaaaagaa aaagtagaat ttttaatagt ttaatattga agaagatttt 15540
ggaaaaggtt tttatattat attattttta ggattggtat agtgttggta tatagttgat 15600
gttttaatat gtggatggat ttttttttta attaattttt tgatttttaa taaaggttaa 15660
gttagaaata aattgtataa agtatagagt atagaaagcg ttttgaattg tagtaagttt 15720
attgtttttt ttttttatat ttatttatgt ttttggttag gaggtattga gtatttataa 15780
tgagttaggt attgatttag gtattggagg tatgatatta aatgtaatta agtttttgtt 15840
tttagagttt atatttaggt agaaaatttt atatggaagt ttttttatgt atttagtttt 15900
gtatttttcg ttgttagatt ttaaatttta gaagattttt tttaagtatg aatatacgat 15960
tttgtataag gaatttttta atttatgaat taatgaattt tggtaaagtt tttaatattt 16020
agttataata ttaatattta aggtttaaag atgatgtaat ttatttaatt tttagtattt 16080
tatgtttttg ttttttttat tttttgaaaa ttttagaaat aatttgttat atagaggaat 16140
ttaaggaatg taattttaat ttaaaatatt aattttgggt aggggattgt tttaaggttt 16200
atttattatg attttttttt tttgatttga aaattagcga ggtagtgtat agattttagg 16260
ttttttagta taaagaatag tgtatttaat tatattttta ttataaaagt tatgtgatat 16320
agaataaatt ttttagattt taatagttat aaaattattt ttaagaatat aaatttaata 16380
ttaataatta aattatattt taaattttat ttttttattg aatttggagg tagaggtttt 16440
gaatttagag gaagtatagt tttatatagt tagtatgata aatgttttag attagtaaga 16500
aagttagtaa gtgtgttttt atttatgtta tttgtttatt tttttgtaaa ttaattaaat 16560
aaatattata gagttagatt gtagtaattg atatatttaa tattgtatta tttaatatat 16620
taatagtttg ttt 16633

<210> 3
<211> 6133
<212> DNA
<213> Artificial Sequence

<220>
<223> chemically treated genomic DNA (Homo sapiens)

<400> 3

tttttgaaag ttttttttgt cgtggttttt attattttat tttttttagg tttttttttt 60
atttttatta ttgttttttt tgtttttttt tttgttggta ttgttgtatt tttagaattt 120
ttttgttttt ttgagttgtt tgaataataa tgatatgttg ttaattttta aatttatatt 180
tttgtggttt taggtatttt taaatttagt atgtttgata aggaatttag tttttatatt 240
tttaaaattt ttttattttt tggagacgga gtttttgtcg tttaggttgg agtgtagtgg 300
tataattttg gtttattgta atttttgttt tttgggttta agtgattttt ttgttttagt 360
ttattaagta gttgggatta taggtgttta ttattatgtt tagttaattt ttgtattttt 420
aggggttttg ttatgttggt tagttggttt tgaatttttg attttaggtg atttatttgc 480
gttggtttcg taaagtgttg ggattatagg cgtgagttat tgcgtttagt taaggaatta 540
attttttttt ttttaaattt gtttatttta gtttgttgaa tgaaattatt aggtagttag 600
ttatttaaga ttattttaga gttatatggg gttatttttt ttttttattt ttcgtattta 660
attaattttg ttatttttac gtttttgaat ttttgaattt gttttttttt tttattttta 720
tattgttgtt gtttggggtt aggtttattt tttattttgt ttgggttttg tttttttttt 780
ggttttttta taattagttt tgtttttttt taattagttt ttgtataaag tttaaattta 840
tttggtttag taaagtgatt tttttaatat aatatgatta gtttataaat atgattagtt 900
tagttttttt taaaatataa atatgattaa tttatattta gaattttttt aagtgtattg 960
aagagggttt tttattagat ttttatttta gtgtgtttta tttttaagta gatttttacg 1020
tttgtttagt ttattaatat tacgttttta ttgtttatta tttttagttt gtattttttg 1080
ggtgtatcgt taggttgttt acggtttttt taaatgttag gtagaatagt tttttgttat 1140
tttttgtttt tttgatatag gtttatttgg ggggattttt tgatttttta agaattagcg 1200
tagtttgttt tttcgtgaaa atttttttaa ttttttagtt tgttttttta tagttgaatt 1260
atttatgttt tttgagtggt ttttattggt tttttatagg tttgtgtttt cgtagagata 1320
attcgttgtt gttttttttt ttatgttttt ttgtattaga ttttaagttt tatgaaggcg 1380
aggattatga tttatttatg tttatatgtt gtgtttggtg tattgtaggt gtttagtgtt 1440
tgtggaatgg atgagtgaag atgaattatt gtttttttgg gatgaatgat tttgttgttt 1500
attaaggtaa atgatttttt aggtagtggt gtttttgtgc gttgcgatta atgtggtaat 1560
tgttatattg agttgttatt ttagttagtg ggattaagga gtaagatttt tttttggaga 1620
agtggttttt gttagttttg gggatttttt tgataaagag atagggtaga ggggaaaaga 1680
agtttataga ttgtgatgtg ggtttagttt taagattaag acggtttata aggttaggta 1740
cggtggttta cgtttgtaat tttaatattt tgggaggtcg aggtaggtag attacgagat 1800
taggagttcg agattagttt gattaatgtg gtgaaatttt gtttttatta aaaatataaa 1860
aattagttag gtatggtggc gtatgtttgt agttttagtt atttgggagg ttgaggtaga 1920
agaatcgttt gaatttagga ggcggaggtt gtagtgagtc gagattatgt tattgtattt 1980
tagtttggga gatagagcga gatatcgttt taaaaaagaa aaaaaaagaa tttgaaaaag 2040
aaaaaaaaga aggtttacga aaatggatat ttatcggggt ttttagggat ggtgaggaga 2100
attagtagga agtatttttg ggttaggttg tgggaaggag cgaagggtat aggtagggat 2160
taggtagttt ttatgtagcg cgggatttaa aaggaaggta gatatattat ttaggaaagt 2220
tttagtttgg agatgtttta aatttttata aatggggtgt gaggtatatg attaattttt 2280
tttttgtttt attttatttt ttatttttag ttgggttttg aagattagta tttagaaaag 2340
taggagatgt tatggtgttg aagaaagtat aggattagta ggttgtggtt ttaatgatgg 2400
attgtatgtg agttttggta aatcgtttat tttttttaag ttttagttat tttatcgggg 2460
tatattttga tatgttttaa ttatttttta ggattgtagt gaaggttaat ttataatgat 2520
gataatagtt atttttttta gattattttt tatgtgttag agattatgtt aaatgtttta 2580
ttatatttat taatattaat aatatttgta tgatatagaa ttattattag ttttatttta 2640
taagatgagt aaattgaggt ttagggaggt aaagtatttt gtttaaagtt taatgttgtt 2700
aagtgagttg atggagttga cggaggtggt atttggtttg tggagttagt attttggttt 2760
tttgatttgt atatttagtt gtgtggttga ttttattgtt taggagtgaa gtaattatag 2820
gttttttttg ggatttttgg tttcgaagtt atagtggtag tatttagtaa gaaataagga 2880
aagatatttt tttttttttt ggttttagat atgtgttttt gaggggttgg tttttttttt 2940
ttaattgtag gggttggtga ttgtattggg ttgaatagtg tttatttaaa attcgtgttt 3000
attaggaatt tgcgaatgta attttatttg gaacgaggat tttagtagta gatgtaatta 3060
attaagatta tttgtattta tattggatta gggtgggttt taatttaatg attggtattt 3120
ttataagaag agaggaatgt ggatagagat gtagatgtag agggaagata attatgtgaa 3180
gatatggggt tatgttttcg taaattaaat aatattaagg attttgggaa ttattagaag 3240
ttagaagagg taaggaagga ttttttttta gagtttttag agagtatatg gttttgttaa 3300
tattatggtt ttagattttt agtttttaga atagtgagag aatatatttg tattttaagt 3360
tatttagttt ggtagtagga aattaatata gtaatttagt attgtgtgta ttatagtttg 3420
gtggatagag tatggattgg aaattattta gacggattgg gtggggtgtg tttatttggg 3480
ggagatgagg gagaggagaa taagttattt atttatttag ttatattgat taatttgtag 3540
ataatgtttt tttatgattt tatgtgattg tgtgtatatt gtgtttggcg agtgtgtttt 3600
tagttttatt tttttcggat atgacgatgg gagtttttta ggttggtgaa atttagaagg 3660
tgagaatttt atttaagtaa ttgttttttt tattttttgg ttattttttt tatttttaga 3720
ttttagatgg tgaagttgtg tttagagagg tttaaatttt gtttaagttt ttatagttag 3780

ttagtaggaa gtagaattta gatttagaat tttttggttg taaaaatttt gttttttttt 3840
ttatattaag ttttttttta agttaaagaa attgattttt ttttagaaag atacggagtg 3900
atttgtttat tttttttaagt atttttttta gatttgaaag taatatttat ttgaaaaata 3960
tttagaaatt attgaaatgt taaaagtaaa aaaattatgg taataattat tgttattatt 4020
gggtaaatat ttttttagtt tttttttttt ataaatatat attattagta taatttgaat 4080
aatattgtaa atagtgttgt attttgtttt tttaatttaa tattattttg taagtatttt 4140
tttatgttaa tagtttttgt aaataggatt ttaaattatt aaataatgtt ttattatatg 4200
gatgggttat aatttaatat tttttattat tgtttattta gtttttattt tttttataat 4260
gataaatata tagttaatat tttttataaa tatttgtttt tttattattt tttgaggata 4320
ggggataata ttgggtaaat ggatatgaag attttaaggt atttgatgta taatgttaat 4380
tgtttattaa aagggtttta tttattagtt taattagagt gttttatacg gtatttttat 4440
ttatattgtt attaataaag agttttgtta tgttttgaaa aggcgcgtgt cgcgatttta 4500
ttatataggt tgtcgcgttt agaggatggt agggggcgag gcgaggtagg gaaattagtt 4560
aggagatcgc ggcgatgatt ttaggggacg cgttgggggt ttgaggttag gcgtagggat 4620
ggaggaattg gaatacgtac gggattcgta gaatattcga cgggggcggg ggatcgattt 4680
tgataagata attttgtatt gcgtggggtg taggagaggg aggagattgt acgttcggtt 4740
tcggtttttt tcgcgtcggg ttcggtatcg attcgcgaag ttggtcgagg cgtgagtttc 4800
gtttattttt ttaataggga tatttttttt taaggcgggc gttcgcgcgc gtttcggttt 4860
cgtttttcgg gggcgcgcgc ggcggtcgcg gcgtcggttt cgtcgggagc gtacgtgtag 4920
gcgtcgggcg gaggagaggg aggcgggagt cgggtcgcgg gaggagggga ggcgtcgggg 4980
gcgcgcgcgc gcgcgttggg cgttgttggg ttgcggcggc ggcggcggcg gcggtggtta 5040
ttatggcgga gtcggtcgga gttttttttt tttttttttt tgttgttttt ttgttcgtcg 5100
gtagcggcgg gttcgggttt cggggggttt agggtgagtt ttgggacggg gggcgggggt 5160
cgggatggag agggttcggt aagggcgagg tttcgagtcg cggtcgttgg attatagcgc 5220
gttttttttt tacgagtata atatggtcgg gtggggggcg tagaggagtc ggggtgattt 5280
ttagttagtt ttttcgattt tcgaggcgta gtcgttcggc ggcgacggag tttacgtcgg 5340
gtttttgggg attcgagttt tgttttaggc ggaattggat tcgggtagtt tttaggggtc 5400
gcgtttttag cgttttcgtg ttttagagag gggagtttga gtttttagtt ttaagatttg 5460
gaggttcgtg gtttttgttt tttatcgtgt ttaggggcgg ggggtttttt tttttttttt 5520
taggtgtttt tggggggcgt ttttttttcg ttaagattgt ggggttcggg cggtggggtt 5580
ttagtttttt cgagggaatt tgatgttgag agttcgcgta ttttagattg taggggaagg 5640
ggtttttaat ttttttttta ggttttgtat ttggcgtgtt tttttttttt tttttattgt 5700
ttttttaatg ttttgggaaa gggagttata tttttttttt atttaatttt ttgagtttag 5760
tggggtaatt tttttttcgt ttttcggtat ttatgtttcg tggaggttat ttggaggaag 5820
tgggacgaat aggtttttaa atttttttgg agtttggttt tggaattttt aattttatgg 5880
aatcgagttt tttaagggtt gatgtgaatt taggtgtatt tgtttttttt ttcgtttttt 5940
ttattttatt tcggggggtt tatttagtta attattttgt ggatgttttt taggtgaaat 6000
tttggaattt gtttttaaat ttttaagatt atttaaagat tttattattg gattgtattt 6060
tcggtttttg gttagtattt atttattttt ttatacgatg ttttttttta gttcgtttat 6120
ttttatatat ttt 6133

<210> 4
<211> 6133
<212> DNA
<213> Artificial Sequence

<220>
<223> chemically treated genomic DNA (Homo sapiens)

<400> 4

ggggtgtgtg agggtgggcg ggttgggaag aggtatcgtg taggaaggta aataagtatt 60
gattaagggt cgggaatata gtttagtggt gggattttta aataattttg ggggtttgga 120
gataggtttt aggattttat ttgaaaggta tttataaagt gattaattga atgggttttt 180
cggggtaggg tgggggaggc gaggaggaaa gtaaatatat ttagatttat attaattttt 240
ggggaattcg gttttatagg gttagggatt ttaaagttag gttttaaaaa ggtttagggg 300
tttgttcgtt ttattttttt taaatagttt ttacggggta tgagtgtcgg agggcggagg 360
ggggattgtt ttattgaatt taagggattg agtgaagggg aaatgtgatt ttttttttta 420
gaatattagg gaggtagtgg agggagggga agaaatacgt taagtgtagg atttgaagga 480
aaggttagag attttttttt ttatagtttg aggtgcgcgg atttttaata ttaggttttt 540
tcggagaggt taagatttta tcgttcgaat tttatagttt tggcgggaga aagacgtttt 600
ttaggagtat ttggagggag ggaagaaagt ttttcgtttt tggatacggt gaagggtaga 660
aattacggat ttttaaattt tggagttgga ggtttagatt tttttttttg gaatacgagg 720
gcgttgggag cgcgattttt gagaattgtt cgaatttagt ttcgtttgga atagggttcg 780
ggtttttagg ggttcggcgt gagtttcgtc gtcgtcgggc ggttgcgttt cggagatcgg 840
ggaagttgat tgggggttat ttcgattttt ttgcgttttt tattcggtta tattgtgttc 900

gtggggaggg ggcgcgttgt agtttagcgg tcgcggttcg aggtttcgtt tttgtcgggt 960
tttttttatt tcggttttcg tttttcgttt taggatttat tttggatttt tcggggttcg 1020
gattcgtcgt tgtcggcgag taggaggata ataaggggga agaaggagga ggtttcggtc 1080
gatttcgtta tagtaattat cgtcgtcgtc gtcgtcgtcg tagtttagta gcgtttagcg 1140
cgcgcgcgcg cgttttcggc gttttttttt ttttcgcggt tcggttttcg tttttttttt 1200
tttcgttcgg cgtttgtacg tgcgttttcg gcggagtcgg cgtcgcggtc gtcgcgcgcg 1260
ttttcgaagg gcggggtcgg ggcgcgcgcg ggcgttcgtt ttaaagggga gtgtttttgt 1320
taaaggggtg ggcggggttt acgtttcggt taatttcgcg gatcggtgtc gagttcggcg 1380
cggaggaggt cggagtcgaa cgtgtagttt tttttttttt ttgtatttta cgtagtgtag 1440
aattatttta ttagagtcga tttttcgttt tcgtcgaata ttttacgaat ttcgtgcgtg 1500
ttttagtttt tttatttttg cgtttagttt taagttttta gcgcgttttt tgggattatc 1560
gtcgcggttt tttaattggt ttttttgttt cgtttcgttt tttgttattt tttggacgcg 1620
gtagtttgtg tgatggaatc gcggtacgcg tttttttaga atatagtaaa gttttttatt 1680
gatgatagta tgggtgaggg tgtcgtgtga ggtattttga ttgggttggt aggtagaatt 1740
tttttggtaa ataattggta ttatgtatta aatgttttaa aatttttata tttatttatt 1800
tagtattgtt ttttgttttt aagaaataat aagaggatag atgtttatga aaaatgttaa 1860
ttgtatattt attattgtaa aaaaggtaga aattaagtag ataataatag aaaatgttaa 1920
attatggttt atttatatga tggaatatta tttagtaatt taaaattttg tttataaaaa 1980
ttattgatat gagaaaatgt ttataagata atgttaaatt aaaaaagtaa aatgtaatat 2040
tgtttatagt attatttaaa ttgtattgat aatatgtgtt tataggaaaa aaagattgga 2100
aggatattta tttaatagta atagtagtta ttgttataat ttttttattt ttaatatttt 2160
aataattttt aaatattttt taaataaatg ttatttttaa atttagaaga aatgtttaag 2220
aaagtaaata aattatttcg tatttttttg aaaagaggtt agttttttta gtttgagaaa 2280
gaatttgata taaaggaaag ggtagaattt ttgtagttag agaattttgg atttgaattt 2340
tgttttttat taattagttg tgaaaatttg ggtaaggttt gaattttttt aagtatagtt 2400
ttattatttg agatttaggg gtggaaagga tggttaagag atagggagaa tagttgttta 2460
aatggaattt ttatttttta agttttatta atttgaagaa tttttatcgt tatattcgga 2520
aaaaataaag ttgaagatat attcgttaga tatagtgtat atataattat ataaagttat 2580
ggggaggtat tgtttataag ttggttagtg tagttggatg agtaaataat ttattttttt 2640
tttttttatt ttttttaaat aggtatattt tatttagttc gtttaagtgg tttttaattt 2700
atgttttatt tattaagttg tggtgtatat agtgttaggt tattgtgtta gttttttatt 2760
attaaattgg gtagtttaaa atataaatgt atttttttat tgttttggag gttagaaatt 2820
tgaaattatg gtgttggtag ggttatgtgt tttttgaagg ttttagggaa gaattttttt 2880
ttgttttttt tagtttttgg tggtttttag gatttttggt gttgtttggt ttgcggaagt 2940
ataattttat gtttttatat ggttattttt tttttgtatt tatatttttg tttatatttt 3000
ttttttttta taaggatatt agttattgga ttagggttta ttttaattta gtatgagtgt 3060
aaatgatttt aattggttat atttgttgtt aagattttcg ttttaagtaa gattatattc 3120
gtaggttttt ggtagatacg aattttgggt ggatattatt taatttagta tagttattaa 3180
tttttgtagt taagaaggga aagttaattt tttaggagta tatatttgaa gttagggggg 3240
agggaggtat ttttttttat tttttgttaa gtgttgttat tgtggtttcg aggttagggg 3300
ttttagaggg gatttgtggt tgttttattt ttagatagta aagttagtta tatagttaaa 3360
tatatagatt agggaattag aatgttggtt ttatagatta aatgttattt tcgttagttt 3420
tattagttta tttgatagta ttgaattttg ggtaagatat tttatttttt taaattttaa 3480
tttatttatt ttgtaaaatg gagttaataa tagttttatg ttatatagat attgttagta 3540
ttgataaata tggtaaagta tttagtataa tttttgatat ataggaagtg atttaaaaag 3600
aatagttatt attattatta tgagttgatt tttattataa ttttaggaag tggttgaggt 3660
atgttagagt gtgtttcgat gaagtgattg agatttagag aaggtaagcg atttgttaag 3720
gtttatatat aatttattat taaagttata gtttattggt tttgtgtttt ttttaatatt 3780
atagtatttt ttgttttttt aggtattggt ttttaggatt tagttgaagg tggaaaatga 3840
ggtggagtag gggaggggtt aattatatgt tttatatttt atttgtgaga gtttggggta 3900
tttttaaatt agagtttttt taggtgatgt gtttattttt tttttaggtt tcgcgttata 3960
tagagattgt ttaattttta tttgtgtttt tcgttttttt ttatagtttg gtttaaggat 4020
gttttttgtt agtttttttt attatttttg aaggtttcgg taagtgttta ttttcgtaag 4080
tttttttttt tttttttttt aaattttttt tttttttttt ttgagacggt gtttcgtttt 4140
gttttttagg ttggagtata gtggtatgat ttcggtttat tgtaattttc gttttttggg 4200
tttaagcgat ttttttgttt tagtttttta agtagttggg attataggta tgcgttatta 4260
tgtttggtta atttttgtat ttttagtaga gatagggttt tattatattg gttaggttgg 4320
tttcgaattt ttgatttcgt aatttgtttg tttcggtttt ttaaagtgtt gggattatag 4380
gcgtgagtta tcgtgtttgg ttttgtaaat cgttttaatt ttagggttgg atttatatta 4440
tagtttatga attttttttt tttttgtttt tgtttttttg ttaggagagt ttttagagtt 4500
agtaggagtt atttttttag gaaaaagttt tattttttag ttttattgat tagagtgata 4560
atttagtgtg ataattgtta tattggtcgt aacgtataga gatattatta tttaggggat 4620
tatttgtttt ggtgagtagt agggttattt attttaagaa ggtaatagtt tatttttatt 4680
tatttatttt ataagtatta agtatttata gtatattagg tataatatat agatatggat 4740
aagttatagt tttcgttttt atggaattta aaatttaata tagaaaagta tgagaaaaag 4800
agtaataacg ggttattttt gcgaggatat aagtttgtaa agagttaatg agggttattt 4860

aggggatatg aatgatttag ttgtgagggg ataggttgag gagttgagaa agtttttacg 4920
aagagatagg ttacgttgat ttttggaaga ttagagagtt tttttagata ggtttgtgtt 4980
aggagggtag gagatggtag gagattgttt tgtttggtat ttaggagaat cgtgagtagt 5040
ttagcggtat atttagaaga tgtaggttga agatgatgga tagtggaaac gtaatgttgg 5100
tgagttaggt agacgtggaa gtttatttgg aaatggagta tattggagtg ggagtttaat 5160
aagagatttt ttttagtata tttggaaggg ttttaagtgt aaattgatta tgtttgtatt 5220
ttaggaaagg ttaaattgat tatatttgta aattgattat attgtgttag gaagattatt 5280
ttgttgaatt aagtgaattt gaattttgta tagaggttgg ttggaggaag ataagattgg 5340
ttgtaaggaa attaaagaga aggtaaagtt taggtaagat aggaggtgga tttgatttta 5400
ggtagtagta atgtgaaggt ggaaaagaga aatagattta agagtttaaa gacgtagaaa 5460
tgataagatt gattggatac gggaaatgag gaaaggggat ggttttatat gattttagga 5520
taattttagg tgattagttg tttggtagtt ttatttagta gattagaatg ggtaggtttg 5580
ggaaaaagaa aattagtttt ttggttgggc gtagtggttt acgtttgtaa ttttagtatt 5640
ttgcgaggtt aacgtaggtg gattatttga ggttaggagt ttaagattag ttggttaata 5700
tggtaaaatt tttaaaaata taaaaattag ttgggtatgg tggtgggtat ttataatttt 5760
agttatttgg taggttgagg taggagaatt atttgaattt aggaggtaga ggttgtagtg 5820
agttaagatt gtgttattgt attttagttt aggcgataga gatttcgttt ttaaaaaata 5880
aaaaaatttt aaaaatgtaa aaattgagtt ttttgttagg tatattgagt ttgaaggtat 5940
ttggagttat agagatgtag atttggaagt tagtagtata ttattgttgt ttaaatagtt 6000
taagggagta agagaatttt ggaaatatag taatattagt aaaaaagaag atagaaggaa 6060
tagtgataga agtaggaaga gaatttggga aaaatagaat aatagaaatt acggtaagag 6120
aggtttttaa gag 6133

<210> 5
<211> 6113
<212> DNA
<213> Artificial Sequence

<220>
<223> chemically treated genomic DNA (Homo sapiens)

<220>
<221> unsure
<222> (4643, 4650..4651, 4654..)

<400> 5

ttatattttt atttttttcg atatgttttt ttgattttat tggtatagtg taatgttata 60
tagatggacg ttaagtagaa ggggaggaat aaatagtagt aaaagttagg atgtagagaa 120
tttttttttt attttgggta tttaaggaga gggggatatt tataaaagat atatcgatta 180
tttttttttt ttttggattt attaagttat taaatttatt ttttatgtat tatgttttat 240
agcgttggta tataagaaat tcgttatttg tgttatttta agaatgttta tgttatttga 300
atatagattt ttatagtagt tagaaagtag tacgaaagtt tttaagtttt ttatttaagg 360
ggaatttaat attttttata ttttaaaggt ttttagttat ttttttgaag atgaaggaaa 420
agagattaga cgtgtgttgt tgaagtgttt gttggtgggt ttaaacgtaa gtttaattat 480
aatttagttt atttttagtt ttattttaga attgaagatt taaatttttt tatatttttt 540
tgagttatga ttttgttaat ataaaaagaa atttttattt ttggaagtag tttgtgttat 600
ttatttattt ttattttttt atttagttaa tattttagat tgaattggtt ttttaagatt 660
tttttatttg ggaataattt ttagtttaat tgaatttggt aggattttga attttaaatt 720
ttagtattgg agtggttata aggttaggtt ttggggttgg gaagtgaatg aagttagtta 780
aagatgttta ttttataatg ttcgatgttt tggtcgatta gaatttatgt tttttttttt 840
tttttttttt tttttatttt ttttttttaa attaggtttt atattttgta atgtgttagg 900
aagtggttgt tggatttatg gattttaaaa agtgatttta gaaaataagg aggaaagaag 960
ggtaatgggg ttagttgtag ttattttttt ttaagttttg aagtttcggt ggaaattagt 1020
tataattttt tgttttttta ggttatatta tttttgaatt aaaaaaaaaa acgaaggggt 1080
aattgaatat atttaggaat agaatagttg cgttattggt tgttaatatt ttttttatgg 1140
ggttttttgt ttttgttaga aaggaatgtt ttcgtgaaga ttggttgttt ttttttagta 1200
ttatagaaaa ataattttta tgttttcgtt tttataatgg taaatttagt tggtaagata 1260
aggaatatat ttataagttt tagttgtatt attcgttatt ttagttattt tatttgttta 1320
ggttttataa ttaaagaaat attgaagtga tgtatagata agataagagt ggggtttgat 1380
tttaattgat attaaatttt ttaatgaatt ttaagtgttt ttagagtaaa atatattttt 1440
ttattttaga atcgttagta taatttttaa tttaattaga aaatggaagg tttatatata 1500
tttttttgat taatttataa agataggaga attatatgag attatttaga tatttttaat 1560
atcgatttgg gattttgtaa ttgttttaat attagttaaa ttttgagtat tacgatttga 1620
gaattggtaa ttttggagag atttaaagag ttacgttgat tattaataat tttgataagt 1680
tgtagaaatg ggtagataat tataggatga atttaataga tagaaatata gagtaattta 1740

gaggaagtaa attgttgtaa atataagtga gaagatttgg ttttgtggga aaaggtggga 1800
attagtaaat tataaatgaa atatgaatta gttatattat atagttgttt tattaaaaat 1860
taaaattttg tagagtatat taatagagat tgtatttaag agaaaggtag ttatttttta 1920
gttatttata taattattta ttaagtattt atgtgttagt tgatattttt gggtgttgga 1980
ataaaataag gaaaaaatta agatttttat ttttacgaag tttattaaag tgtggtaaat 2040
gatagagtaa ttatttttaa tagtttttag tttgtatttt gtaattaatt gaatatagta 2100
aatattaaaa gttatttgag gtagggattt attttatttt tatttgttgg attttatttc 2160
gtgtttagta tattagtttg taagtaatcg atatggatta aggggtaaaa ataggttttt 2220
aaatagttaa aattttgata agtaattatt tgtttgatta ttaaatatgt cgttcgaaat 2280
ttttattttt ttttttaaag cgtagtaaat aagtatttgc gtatttatta cgtgtaaaat 2340
agtttttaat ataatgtttt gtgtgtaaaa ggaacggcgg gaatggattt aagttgcgta 2400
tcgtgataaa tggcgtgaat attttgagcg ggtggatagt ttttttttcg tgagtttttt 2460
tttttttttt tttagttatt aagatagtac gcgtgtgtag aagttgtagc ggtgggacgt 2520
agtgttaagt ttgggggcgt atatattagt tcgcgcgtcg cgagttcgtt tgggtagttc 2580
ggcgggcgcg gcgaaatagt ttttacgttc gttttttatc gatttttcgg tattagagcg 2640
gacgaggcgt ttttttagag gaaagattgg gcgtcgtaga cgttttttgg agaagttttt 2700
ggttattcgt tttatttcgg ggtttttttt tttttgggtt ttttttgatt ttttgggtgg 2760
gcgttttaga taggttcgtc ggacgcgatt tggaaacggg tcgtgttcgg tttagttcgt 2820
atttattcgt tcggttatag tttagttata gtttagggat tttgaggcgt tcgcgttttt 2880
ttgtcgcggt atttttagtt taagtaggtc ggacgtcgag gtttcgcggt atcgagtttt 2940
cgttcgtttc gttttttcgt tttttatttc gtttcgtttt cgttttttta tttttttttc 3000
gttttcgttt cgttttttcg ataatttttt cgcgttttcg gttcggtacg cgcgttgcgt 3060
tcggtcgtcg ttgtcgtcgt cgtcgtcgtc gtcgttgtcg tcgagcgttt tttcgttgcg 3120
tttggttttt agtttcgggt cggaatcgga agtttggggg gcggagttcg gcggaggtta 3180
ggagatcgaa aacgcggtcg agttcggagt tcggagttgg agttagagtt tggattagaa 3240
tttggtcgtc gtttgtatcg tcgtcgtcgt tgtcgttcgt cgtttttttt tcgcgtcgta 3300
gtcgtttcgt cgttatcgtc gcgagttcgg tcgttagtgg ttttcggatt ttaggtgttt 3360
gggttgaagg tcggttcgga tatcggattt agttagtttt tagattgttc gggcggtagc 3420
ggacgtcgtt gtcgtcgtcg ttttttcgtc gttttagttt ggcgttttgt ttcgagagac 3480
gggagaggcg agcggagttg atagtgattt tgatagtgat ttaaattcgt ttttgttgtt 3540
gttggttttt cgttgtttgg ttttgtgtgt tgtgcgtgtg tgtggcggtt tttttttcgt 3600
ggtgtatttt attttttatc gttgtgtttt tttttttttt tttttttttt aatttagtga 3660
gcgttttttt tttttttttt tttttttttg ggtcggggtt ttcgaaaatg ttttaggagg 3720
gttgatttta tattaggagg tttgttttcg ggaaaaattt agggaattgg atatttaggg 3780
aaaatttttt tcggattttt cggtttttcg gaaaaatggg agttgttgta aagttggggt 3840
ttgggttttt tttatttttt gttttttagg aaggtaaagg gatttcggcg cgttgggtgt 3900
ttttttgggc gcggggggtc gtgttatggg ggtcgggtgg tgttttatat ttcgagagtt 3960
tatgggaggt tgtgggggat ggtttatttt cgtgtcgtcg ggggggggaa gggttttttt 4020
tttaattata agttgtagtt gtgatggaaa atcgcgaggc gttgtttttg ttttgagtag 4080
gggcgggacg cgaaaaaaga gggttggtgt tggttgtgag agtgaaaaag atgtgtttgt 4140
tggtaaaaat acgggagtaa gttcgtgttt gtgtgtaagt aggttttaaa tagtagaggg 4200
gaaattgtgg ataggtgtga gtacggaagg agttttggtg tggggttcgt taataaaggg 4260
ggtgtttttg gtttgttgaa gtatgtcgtc gtcggtaatg ttaaataata gtgatcggag 4320
ggtggtgaat gttggtggtg gggataaaaa gcgttattat aacgttttat attcgacgga 4380
ttttatcgtt ttaatgttat atttcggatt aaatcgaaat ttggggggaa ttttcgcgag 4440
ttataattta aatacgttta attcgagttc gaattggatt aagttggaaa atatttggtt 4500
cgtcgtttaa ttatataatt aattgttagt gtatatatta gcgttgagtt agtaatttta 4560
attttcgagt tttggtttta gtgttttacg aatttggttt tcgggtatag agcggttcga 4620
taattcgatt taataaattt ttntataaan nttnnttagc ggggaggttt cggttttttt 4680
tttatttaat tgttttaatt ttgggttttt tttttagaaa tcggggaggc gggcggggtt 4740
tattattatt tttataagga attaaaattt tttttttttg tggtgggttt ttcgtggttt 4800
gggttttggg ggtgggcggg ggggtggcgg gttttttttt cgggggtaat ttatttttta 4860
acggtggggt tttttttttt tttttttttt ttaattttag tgaggggttt tttttttttt 4920
tttttttttt tttggtttgg gttttcgaat atgttttatg acggttgatt ttatattagg 4980
aggtttgttt tcgaggaaga tttagggaat tggatattta gcgagaattt ttttcggatt 5040
tttcggcgtt tcgggaaaat gggagttgtt gtaaagttgg cgtttgtcgt tttttttatt 5100
ttttgttttt taggtaggtg tagggagcgc ggcgcggtgg ggttgttttt gcggtcgcgg 5160
cggtcgttgt tgggggtcgc ggttggtgtt gagtcggaga gtttagtgga gtttggggag 5220
gttgtttatt ttttgcgttt ttcggttgtt atcgtttttt tttttcgttt ttaggtttga 5280
ttgtgatttg gaaattttta tgtatttttt tttttttgag ttgggtcggc gagttgataa 5340
gagtgagtgt tgtgttgttg tggaagtgag aaagttatgt tttgtggtta taatattgga 5400
tgatagcgtt ggttgggttg taattaagtc gtaaatatta taagtgataa tttatgtaaa 5460
ggttgagatt tgaaaggagt atttagtgtg ggttttttta aaataagggt tgttttttgt 5520
tatcgtttaa ttagtgtttt atcggtgaat attacgatat agattaagtt agttgttgtt 5580
taattttggt tgtgggaaat aatatatttt attagtaatt taatttgggt ttttttttac 5640
gtttttttta attttagcgt ttagtagtgt taaaaatagt tgtagttgtt gttggagtat 5700

taattataat ttagtgtaga agaggtatgt cgtttggtat tggtaatgtg ttggcgttta 5760
attattattt gtgtgttttt ttggggtata gattttgttt atttacggaa tatttatttt 5820
aaacgttttg tattgaagta ttgttttcgg tttagaataa tttatgtgaa attgtttttg 5880
ttaaatatag tatatttata aaataggttt tagtttaagg ttgggggaat aaacgggtta 5940
ttttatattg gaaattttga ttttgtgagg acgttttatg gttgtaatta gtggttggaa 6000
aaattattta gttttttgta tttgaaattt tataaataaa tatagtattt agttgaaagg 6060
tgtgtagatg tatataatag gagagttagt ttaatagatg aaatatttta aat 6113

<210> 6
<211> 6113
<212> DNA
<213> Artificial Sequence

<220>
<223> chemically treated genomic DNA (Homo sapiens)

<220>
<221> unsure
<222> (1459..1460, 1463..1464, 1471)

<400> 6

atttaaaatg ttttatttat tgaattaatt tttttgttat gtatatttgt atatttttta 60
gttagatgtt gtatttattt atagaatttt aaatataaaa gattagatag tttttttaat 120
tattggttgt aattatagaa cgtttttata aaattaaaat ttttaatata aaatggttcg 180
tttatttttt taattttgag ttaaaattta ttttatgagt gtattatatt taataaaaat 240
aattttatat gggttgtttt aaatcgaaaa taatgtttta atataaagcg tttaaaataa 300
gtatttcgta ggtaagtaaa atttatgttt taaaagagta tataaatggt ggttgaacgt 360
tagtatatta ttaatattaa gcggtatatt ttttttgtat tgggttatag ttaatatttt 420
aataataatt ataattattt ttaatattgt tggacgttga agttgaagaa aacgtgaggg 480
gagatttaga ttaaattgtt gataggatgt attgtttttt atagttaaaa ttaagtagta 540
attggtttga tttatgtcgt gatatttatc ggtgaagtat taattaaacg atgataaaaa 600
gtaatttttg ttttggagga gtttatatta aatatttttt ttagatttta atttttgtat 660
aaattgttat ttgtggtatt tgcggtttaa ttgtaattta attagcgttg ttatttagtg 720
ttatggttat aaaatatgat ttttttattt ttataataat atagtattta tttttattag 780
ttcgtcgatt taatttaaaa agagaaaaat gtatggaggt ttttagatta taattagatt 840
tgggggcggg gggggggggc ggtggtagtc gaggggcgta gggagtgggt aattttttta 900
ggttttattg gatttttcga tttaatatta gtcgcggttt ttagtagcgg tcgtcgcggt 960
cgtaggagta gttttatcgc gtcgcgtttt ttgtatttat ttgaagaata ggagataaga 1020
aagacggtaa acgttaattt tgtagtagtt tttatttttt cgaggcgtcg gggaattcgg 1080
aggaagtttt cgttagatat ttagtttttt gggttttttt cggagataaa ttttttggtg 1140
taaaattaat cgttataaaa tatattcgga agtttagatt aaaaaaaaaa aaaaaaaaaa 1200
aaaaaatttt ttattggggt taaaaaaaaa aaggaaaaaa agggttttat cgttaaaaaa 1260
taaattgttt tcggggggaa aattcgttat tttttcgttt atttttaaaa tttaaattac 1320
gaaaggttta ttataaaaaa agggggtttt aattttttgt aaaaataatg gtaggtttcg 1380
ttcgtttttt cggtttttgg gaaaaaaatt taaggttagg gtaattaaat gggggggggg 1440
tcggggtttt ttcgttgann aanntttatg ngaaaattta ttgggtcggg ttgtcggatc 1500
gttttatatt cggagattaa attcgtggga tattggggtt agaattcggg ggttggggtt 1560
gttggtttag cgttggtata tgtattggta attggttgtg tggttgaacg gcgggttaaa 1620
tattttttag tttggtttaa ttcgggttcg ggttgagcgt gtttgggttg tggttcgcgg 1680
gggttttttt taaatttcgg tttggttcga agtgtaatat tggggcgatg gaattcgtcg 1740
ggtatggggc gttgtaatag cgttttttgt ttttattatt aatatttatt atttttcggt 1800
tattattgtt tgatattgtc ggcggcgata tattttaata gattaaaagt attttttttg 1860
ttggcgagtt ttatattaaa atttttttcg tatttatatt tgtttataat tttttttttg 1920
ttgtttgggg tttatttgta tataaatacg agtttatttt cgtgtttttg ttaataaata 1980
tatttttttt atttttataa ttaatattag tttttttttt tcgcgtttcg tttttattta 2040
aaataggaat aacgtttcgc ggttttttat tataattata gtttgtggtt gggggggggg 2100
tttttttttt tttcggcggt acgaaaatgg gttatttttt atagtttttt atgaattttc 2160
gaaatataaa atattattcg gtttttataa tacgattttt cgcgtttaag ggagtattta 2220
acgcgtcggg gtttttttat ttttttgaag aataggggat aagaaaaatt taaattttaa 2280
ttttgtagta gtttttattt tttcgggggg tcggggaatt cgggggaagt tttttttaga 2340
tatttagttt tttgggtttt tttcggagat aaattttttg gtgtaaaatt aattttttta 2400
aaatattttc gggggtttcg gtttaaaaaa aaaaaaaaaa aaaaaaaaaa cgtttattgg 2460
gttaaaaaaa aaaaagaaaa aaaagagtat aacgataaaa aataaaatgt attacggggg 2520
aaaaatcgtt atatatacgt ataatatata aaattaaata acgaaaagtt aataataata 2580
aaagcgggtt taaattattg ttaaaattat tgttagtttc gttcgttttt ttcgtttttc 2640

ggaataaaac gttaggttga ggcgacgaag aggcggcggc ggtagcggcg ttcgttgtcg 2700
ttcggatagt ttgggaattg attgggttcg atgttcggat cgatttttaa tttagatatt 2760
taaagttcga ggattattgg cggtcgagtt cgcggcggtg gcggcgaggc ggttgcggcg 2820
cggggaaggg gcggcgggcg gtagcggcgg cggcggtgta ggcggcggtt aagttttggt 2880
ttaggttttg gttttagttt cgggtttcgg gttcggtcgc gttttcggtt ttttggtttt 2940
cgtcgggttt cgtttttttaa atttcggtt tcggttcgaa gttggaagtt aggcgtagcg 3000
gaggaacgtt cggcggtagc ggcggcggcg gcggcggcgg tagcggcggt cgggcgtagc 3060
gcgcgtgtcg ggtcgggagc gcgaggagat tgtcggggaa gcgaggcggg agcgggggag 3120
gggtgagaga gcgagggcgg ggcggagtgg agggcgagga ggcggggcgg acggaggttc 3180
gatatcgcga ggtttcggcg ttcggtttgt ttgggttaga gatatcgcga taaaggggcg 3240
cgggcgtttt aaagttttta agttgtggtt gggttgtggt cgggcgagta gatgcgggtt 3300
aggtcgggta cgattcgttt ttaaatcgcg ttcgacgggt ttgtttggag cgtttattta 3360
aagggttagg agaagtttaa gaggaagagg atttcgaggt ggggcgagtg gttagggatt 3420
tttttaaggg gcgtttgcgg cgtttagttt ttttttgggg gggacgtttc gttcgttttg 3480
atatcgagga gtcggtggaa ggcggacgtg ggagttgttt cgtcgcgttc gtcgagttgt 3540
ttaggcgagt tcgcgacgcg cgagttggtg tgtgcgtttt tagatttagt attgcgtttt 3600
atcgttgtag tttttgtata cgcgtgttgt tttggtgatt agaaaagaaa aagaaaaaat 3660
ttacggagga agggttgttt attcgtttag gatatttacg ttatttatta cgatgcgtaa 3720
tttgaattta ttttcgtcgt tttttttata tataaagtat tgtgttgaaa attgttttgt 3780
acgtaatagg tgcgtaaata tttgtttatt gcgttttgaa gaaaaaaatg aggatttcga 3840
acgatatatt tgatgattag ataagtgatt atttgttaaa attttagtta tttaagggtt 3900
tgtttttatt ttttaattta tgtcgattgt ttataagttg gtgtgttagg tacggaatgg 3960
gatttaatag gtaaaaatag gatggatttt tattttaagt agtttttagt atttgttgta 4020
tttagttggt tgtagaatat aaattagaga ttattgaagg tagttgtttt gttatttatt 4080
atattttagt gagtttcgtg aaggtagaga ttttgatttt ttttttattt tattttagta 4140
tttagaagta ttaattagta tataagtgtt taataaataa ttgtataaat gattaaggga 4200
tgattgtttt ttttttaaat atagtttttg ttaatgtatt ttataaagtt ttggttttta 4260
ataaaataat tgtataatat ggttaattta tgttttattt gtggtttatt gatttttatt 4320
tttttttata aagttaagtt tttttattta tatttatagt agtttatttt ttttaaatta 4380
ttttgtattt ttgtttatta agtttatttt gtaattattt gtttattttt ataatttatt 4440
agggttatta ataattaacg tggtttttta aattttttta agattgttag tttttaaatc 4500
gtgatgttta gaatttgatt gatgttggaa tagttataaa gttttaagtc ggtattaaag 4560
gtgtttgaat gattttatgt agttttttta tttttatgag ttggttaaag gaatatgtgt 4620
aagtttttta ttttttggtt gaattaagag ttgtattaac ggttttggaa tggaagagta 4680
tattttattt tagaaatatt tgaagtttat tagagaattt gatgttagtt agggttaggt 4740
tttatttttg ttttatttgt gtattatttt agtatttttt taattgtgaa gtttgagtaa 4800
gtggaataat tggaatagcg agtaatataa ttgaaatttg taaatatatt ttttatttta 4860
ttaattaaat ttgttattat aagaacgaga gtatgaaaat tatttttttg tagtattaaa 4920
gagaagtagt tagtttttac gaggatattt ttttttaata aagatagaaa attttatggg 4980
aaaagtatta ataattagta acgtagttat tttgttttta aatgtgttta attatttttt 5040
cgtttttttt tttaatttaa aaataatgta atttaaaaag ataaggaatt gtgattaatt 5100
tttatcggag ttttaaagtt taaaaagagg tagttatagt taattttatt gttttttttt 5160
tttttttgtt ttttagaatt attttttaaa atttataaat ttaataatta ttttttaata 5220
tattgtaagg tgtaaaattt ggtttgggaa ggaaaatgag aaaggagagg aaaggaaaag 5280
gaaatatgaa ttttaatcga ttagagtatc gggtattatg agataaatat ttttagttaa 5340
ttttatttat tttttagttt taagatttag ttttatggtt attttaatgt tagaatttga 5400
agtttagagt tttgttaagt ttaattgaat tgaaaattat ttttaggtgg aagaattttg 5460
agaagttagt ttaatttaga gtgttggttg gatgaggagg tagaggtaaa taagtgatat 5520
agattgtttt tagaataaaa gatttttttt tgtgttaata aagttatggt ttaaaaagat 5580
gtggagaagt ttaaattttt agttttgaag tagagttgga aatgaattgg gttataattg 5640
aatttgcgtt tggatttatt aatagatatt ttagtaatat acgtttaatt tttttttttt 5700
tatttttagg aagatgattg aaaatttttg gagtatggag gatgttaagt tttttttaaa 5760
tgaaggattt agaagttttc gtgttgtttt ttagttatta taaaaatttg tgtttagatg 5820
atatgggtat ttttgaagtg gtataagtaa cgaatttttt atatgttaac gttgtaaggt 5880
ataatgtatg gggaataaat ttggtaattt ggtgagttta aggaagggag aaatgatcga 5940
tgtatttttt ataaatattt tttttttttt aagtgtttaa ggtgggaaga aaatttttta 6000
tattttagtt tttgttgtta tttatttttt tttttttatt taacgtttat ttgtatagta 6060
ttgtattgtg ttaatggggt taaaaagata tatcgaggag gataagaatg tgg 6113

<210> 7
<211> 10053
<212> DNA
<213> Artificial Sequence

<220>
<223> chemically treated genomic DNA (Homo sapiens)

<220>
<221> unsure
<222> (30)

<400> 7

gaattaaatg gatattttag aattgtaaan ggtagtaatt gaaataaaaa tttattggat 60
gagcgtaata atagggtgga aggatagaga aaataatttt tgaattggat aaaaattggt 120
tgttttgttt tgagaggtag ttttgttttt gtcgtttagg ttggagtgta atggcgagat 180
ttcggtttat ttcggtttaa gtttttggga ttggggaagg atttaagttt tagttttttt 240
agtagttggg tttgtcggtg ttcgttatta cgttcggtta ttttttgtgt ttttggtaga 300
gatggggttt tattttgttg gtttggttgg ttttaggttt ttgattttag gtgatttgtt 360
tattttagtt ttttaaagtg ttgggattat aggtatgagt tatcgcgttc ggtttttata 420
agtttttttt tatgtttaaa atttttaatt ttatttttgt atttaatttt ttttttttat 480
taaatatata tattagttta ggtttatata gggttaggat tattattatt attgtttttt 540
atttttatat tttgttttat tggtaggttt ttaggggtag taatgtgtgg aattgttgtt 600
ttttgtaata ataatgtttt ttggaatatt ttttgaagga tttgtttgag gttgttttaa 660
agttaatttt tttaaaaaat agaagatgta tattttaaaa tataataata aaaagtatag 720
gatattaaat atataaatta gtagtattgt tatttattat taagtattgt ttattgaata 780
tagttgtata tgttgtatac gtttttatgg ttagtagcgt agtgggtttg tttatattag 840
tattagtata aatatatgag taatgcgatg cgttattacg acgttagtag gtaataggaa 900
ttttttggtt ttatttataa ttttatgcga ttattattgt atgtgtggaa tatttttgat 960
tgaaatttta ttatgtagta aattattata ttagtaaata aaattgagtt atatataatg 1020
aagaagtata tattataatt aagtgggttt attttaggga tgtaaggttt ggtttattat 1080
tagaaaatta gttaatatag gttgggtacg gtggtttacg tttttaattt tagtattttg 1140
ggaggtcgag gcgggtggat tatttgaggt cgggagttta agattagatt gattaatatg 1200
gagaaatttt ttttttatta aaaatataaa attagttagg tgtggtgata ggtatttgta 1260
attttagtta ttagggaggt tgaggtagga gaatcgtttg aatttaggag gtagatattg 1320
tagtgagtcg agattacgtt attgtatttt agtttgggta ataagagtaa aattttgttt 1380
taaaaaaaaa aaaaaagaaa aagaaagaag agaaaattag ttaatataat ttatcgtatt 1440
aatagattaa taaagagaaa ttatataatt cgtattagtg gatgtagaaa aggtatttga 1500
taaaatttaa tagttattta tgataagaat atatatatat atataaaaga ggaatggggg 1560
aattttttta atttgatata gagtggttat tataagaaat ttacggttaa tattatgatt 1620
aatagattga gtgtgttttt ttaagagaaa taaggtaagg atggttgttt tgttttgatt 1680
atttttattt aatataatgt tagaagtttt agttagtgta gttaggtaag aaaaggaaat 1740
aaaagatatg tatattggaa ggtaagaaat aatattgttt ttatttgtag atgtagatga 1800
ttgtcgatgt agaaaatttt aaagaattta tgaggggtag aaaatttaaa tattttattt 1860
ttttgaataa gtgagtttag taaggttata gtatataaga ttaatatgta aaaatcgatt 1920
atatttttat ataatttaat gaataaatgg atataaaaat taaaaatagt atattttgtt 1980
tataattgtt tttaaaaagt ataagtgtaa taaaatatat aaaggagtta aatgttgaaa 2040
attataagaa tattgataaa taaaagagat ttaaataaat ggaaaaaaaa tatattatta 2100
agaattttaa gatggtaatg atagcgtatt aaattaagta tggggttttg tgtatttgta 2160
taggttatat ttttatgaag ttagtttcgt attagtatta agagtgatgt ttgagtttgg 2220
gtggggtggt ttatgtttgt aattttagta ttttgggagg ttgagtgggt ggattagttg 2280
aggttaggag ttcgagatta gtttgtataa tatggtgaaa tttcgttttt attaaaaata 2340
taaatattag ttaggcgtgg aggtacgtat ttgtaatttt agttatttgg gagattgaga 2400
taggagaatt atttgaattt gggaggtaga tttgagatta cgtaattgta ttttagtttg 2460
ggcgatattg tgagatttta ttttaaaaaa aaaagaatga tgtttgaatt taatatttat 2520
agttggtttt gaaaggtgaa ggtgtttatt gttttgtttt tttgatagat tttatgttat 2580
ttatttttta tagatgagaa tttgtttgag gttttatttt atatagaatt tttaggtttt 2640
tgttttttgt agtgttggga aatttttttg agtatttaaa tttaaatttt tgttttttgg 2700
gtttgttttt ttttgttttg tttttattga atatggggaa tagttggttt ttttgagtaa 2760
ttaatttttg tagatttgga tatagtttgt aatttttttt tttttttttt attgggatga 2820
ttttttaggg ttttttttaa ttttggattt ttaattttga tatttttttt aggttttttg 2880
tgtgttttgg aaggtatggg gtttataaga gttttaaaga gtattagtta ggggttagag 2940
ggtttagtgt tttggggaga tggggaagat tttagtattg atgaatagat tttagttttt 3000
tttgggtttt tttttttagt ttttaaaatg tattttattt ttatttgttt tacgaacgtt 3060
ggtttagata gttttttatt ttttattgat tgttagaagt gatttttagt ttttgtttat 3120
ttcgaagttt ttgtgtttag tgtgtttttt agattaaagg ttttttttgg gaagtttcga 3180
ttttcgtttt ttaggataga gatttagggg ttgggggagg aaaaggttga ttagaagtta 3240
tagcggagta gggagagaga gtgtgaaaga tagattcgcg gttaggtttt tagtttttta 3300
gttcgtagag ggtttaagtg gtcgttataa tttgaaagag tagatatcgt aattttatag 3360
tattttttat tggttgtagg atatagtttt gttttgatat tgaaatttgg gtgtgttagg 3420
gttttgggaa tttataacgt ttataatttg tgaagtagtt gtggggtggg ggatggggag 3480
ggttttagta gaggaagtga ggttagttaa taattgatgt ttgtttgagt ttttagttat 3540

tatttttttt agtttttatt tttgttaaaa ggtggggcgg ggtaggagga ggggtttttg 3600
gtttattttg tagaattttt atattagagt aagatatttt agaggtttat ttttgttttt 3660
aatatttacg tttttttaag tgtttttgag gttatttttt ttttagtttt tttatttatt 3720
tatttaatta acgaacgttt ttattgaggg ttttttttga gttaggttta gttagttagt 3780
atttttgtta tgagttgaga taagtattat tttcgtttat ttttataatt attttatgag 3840
gttggtacgg tttattatgt ttatttagta gatgggggat tgaagtatgg agaggtgtta 3900
ttagtttacg gtaatataat tagtttgtat ttttagtagg tagtttgatt ttagagtttt 3960
tgtggataat taggaggtta ggattaagat tagagttttg taggtattta gatggttgga 4020
gtaaagtagg gtagtgaggt tagtgttttt agtttgtgta ggagtattag gaagagtttg 4080
tgtttttttt ttttgtcggt atgaagttat tttgtttttt tttttagttg ttttgtgtta 4140
gtagagtttt agggaggttt tattttttat ttttatttaa agttttattt gttggggtgg 4200
gggttttgtt tggaagggga aggtttaagg ttgtttttag cgtgtttttt tattttgatt 4260
gtttttggcg gggcgggggt gtttttgtta tttagttgta taacggttag gaagggttta 4320
aattattttt agggttaatt taaggtcgtt ttttgggttt gtatattttt gtgttgagtg 4380
cggatcggga gaggttgttg aagataggag gggataaatg ggggacgaag gggttcgagg 4440
gaggggattg aaggatttgg gttaagtcgg gagttttcga gggcggagtt aaaacgtatt 4500
tggattttgt tagttttaaa ttttgttttt attgttgtaa gttttttaga tcgaggattt 4560
tcgggttgag ggtggggtaa ggataggtag tgtttttttt cgttttattt tcgtttgttt 4620
ttttttcggt ggtttttttt cggcgtttcg attttaggcg gttttttcgt tgttgttagt 4680
cgattttttt ttatttttat ttattatttc ggtcgttaga cgttgtttat agtttcggtt 4740
ttgtttttta cggttgtggg ttcggatttt acgggatttt tatgggattt ttataggatt 4800
tttacggttt gagtttaagg ttcgtttttt cggggaggcg gatgtgggag gttcggttcg 4860
ggtgcgggtt agcgattcgg gagttgcggg cggttgggag gggaggtcgt tttgaggggt 4920
tgggagcggc gcgggggtgg gtttcggttt tgtagtttta gcgaggggcg agcggcggtt 4980
agtcggcgag ttgggtaata aggaaacggt ttattaggag ggagtggtgg agttgggtta 5040
ggtaggaaga cgttggaata agaaatattt ttgttttagt ttttatttta gtttcgggag 5100
gttgtcgcgt tagttgcgtc gagcgagttt tttttcggtt ttagttcggt tcggggtcgc 5160
gttcggattt tagttcgtcg tttagcgttg gcggtgtaat tgcggtcgcg cggtggaggg 5220
gaggtggttt cggttcgtcg aaggttagcg tttcgttatt cgtagagcgg gtttagaggt 5280
gagtcgaggt tcgcggacgg gatcgggtgg cgggcggttt gattttcgtt ttagtgggtt 5340
tttttttcgg gcggatttta gagttatcgt agagtggtcg cgggaggttt agttttagtt 5400
tattagaaag gtaagttgtt tttggttgat tacgtatagt ttttatgatt ttatttgaga 5460
tttggagggg aatggacgag attggattgg aaattagaaa tttggtttta gtcgggtgat 5520
ttgggggttag tgatttttatt tttttgtttt tttatttttt ttataaaatg ggtagtggga 5580
tttttgtttt gtttatttta tatagaaggg attatacgtt ttttaggtcg ttgggtggat 5640
ttggagtgaa tgtttatttt tatagttgtt gagtttttta gtagagttta ttttttttag 5700
attagttttt ttagttgttg tttgaggaat ttgatattat tattttaatt taggagtttt 5760
attttaggcg cgtatggtta gttatttttt tgttacgatt tttttttagg atttaggtta 5820
aaggggattt tagttttttt ttaggttggg attttagatt tttttatgga ggttagtgtt 5880
gtgaaattta tggttgggtt ggttgtttta gttttttttt ttttttttaa ggataggggt 5940
ataatggata gttgttttta ttttttttat gtatggttag tttttggttg agttagggtt 6000
tgttttaata tttgtttttt tatttgtttt tttattttat tggtttgtta gaaatagtta 6060
ttattgattg agtattaatt atgtagatat ttagatatat atattttttt ttatgtaatt 6120
ttggtcgtaa tttagtggaa gaggggttat tatttttttt ttatagaaaa ggaaatagat 6180
atgaatatgg gatttgttta aaattttttg gatggtgggg tgaatggtat ggttggggtt 6240
ttgataatat tttagtgttt attgtgtatt atttatattt tgttaaataa ttgtattata 6300
ttttatttat gagataatat tattattatt tttattttat atatgatggg aagtttaagt 6360
tttagaaagt ttaagtaacg ttttaaattt tatattttta agtggtaaag gtaggatttg 6420
aatttaggtt tttatgtttt agagtttata ttttaatttt tgtgttatat tgtgttgttt 6480
ttatggtttg tttgagattt gaaaatttag gtattatttg gatgttttat tgtattattt 6540
cgttgtgaag ggttaataaa ttatatggat gaatcgtaag ggtaatttaa tatgaatagt 6600
tgggtttgtt tttttgttgt tggggtaagg tttattgaag tgtagagtga gtttagaagg 6660
ttgtgtttga tatggcgttt ttttagtttt gtgaagatta ttgttgtgtt gagttattgt 6720
cgttgatggg agtaggtttt attaattttt taggtgtgtt agggagaaaa atattgataa 6780
gtatttttcg ggtatattat tattttggtt ttttttattt gaggtttaga ttttgattaa 6840
gtttatggta aatagatgat ttttgggtag taatattatt tttgttgttt gggttaatat 6900
agtgagattt tatttttatt aaaaataaaa ttgggtatgg tgttgtattt ttgtagtttt 6960
ttgggatttg ggaggttgag gtgggaggat tatttgagtt taggagttta aggttatagt 7020
gagttatgat tgtattattg tattttagtt tgggcgattg agtaagattt tgttttaaaa 7080
aaaattaaaa ataaataaaa acgttgtttt tatttaaata aatttgagta gggtattcgt 7140
taataataat agtagtgggt aatttgtata cgtttatcga gatttatgtt tttttttttt 7200
tttgagggat ttatttaatt ttaaataatt ggaggaataa taatgattaa ggaatgattt 7260
attattagta atttaagttt attaagatac gtttagtgat tatggataag aaattgaata 7320
tttttgagtt ttattttttt tcgttggatt gttgtgatga ttaaattaga taaattattt 7380
aagtttagta tttagtatat aattagtatt tagagaaggt taattgttat tattaagtat 7440
tttatagtgt ttagttaacg tttgaaatta gtttaacgtt tgtaattttt ggttaataat 7500

agtgatgttt ttgttagtag gagatttttt ataaagtttt gtttagaagt gaaaggattt 7560
tgttaggttt taggagtttg tttgtgttat agttagattt tagggtaaat cgtatagagt 7620
tagttttgag tttggattgg tcggatttta gtattttttt gtatttgatt ttatattggg 7680
tttagttaaa aattggatag ttttaatttt attttattat atttttagga ttagattata 7740
gatgggtttt gtagatgtag atagtttcgt tttatagtgt tattacgttt tagttttggt 7800
gtttattttt ggttgttttt tttgtagtag atggggagta ttttgggtgg gagtgagtgt 7860
tttttttttt tgtattttat ttttatttat ttagtagggt tttgtatagt aaaatgaaag 7920
tagaggaatt agatttagcg ttgtgtttat ataaattgat tagaattttt tggggaattt 7980
ttaaaaaatg tatgtttttt gttgatttta ggattgaatt agagtttgga gtgagggtga 8040
aattagtgaa tatgtttttt ttagtgagta ttttaggtga ttttgatgcg tgataaagta 8100
gaaggagggt ggattggggt acggttagag aagtagaggt acggatagag gattttagag 8160
gaatgtcgga ttttagtttt attgttagtt attttttttt ttttatttta aatttttttt 8220
tttgtagttt tttgggttag taaaggttgt taaggatggt tttttgttag tattttagtg 8280
ggtagattta gtttttttag ggtgtttttt ttattttatt ttatcgttag ttttgtcgta 8340
tagtgttagt tagagttttt attggttgcg ggaggggtaa gaggtaggag gttaggtaga 8400
ggttggggat atagttgtag tagtttaggg ttttgttttt ttttttagtt tgggtttttg 8460
gggtagagga agtttaggta ggaaggttag gtttgttatt agttagagag gtttatgaag 8520
tttttttatt ttatttttag ttatattgtt tttagagttt tagtattttg ggtttttatt 8580
tggtattatt tttagattat gggttttttg aagtttttgt ggttatgtgt ttagttttgt 8640
taggtttatt atgttatgtt aaaggatagt ttttttgttt tgtgttttgt ttgaggattt 8700
agtttttaga tatatatttg tatatgtttt attatttaga aagttttgtt gttgtttgat 8760
tagggttttt tttgttgtag ttttagttaa tattttttgg ttttaaattt gtgagaaatg 8820
aagattgggt ggttttagaa gtattagttg gtgtgggatt tggattgggg attattatag 8880
attagaatta tttttttttaa tatttttttaa taaatttttg gaaaagttta atttagttta 8940
gttatggaag gttaaatatt tagtgtttaa gtgtgtttta ttttttttgt tatattattg 9000
gttttttatt tatttttttt tagatggtaa ggggaaagaa gggggttagg gaatagtttc 9060
gagagggtag tttatatttt ggtgagtttg tagagaggtt tttttatatt tggtatgatt 9120
ttgtttcgtt cgagttttgt atggttgtta tagttttgag ggtttagata tgggttgtta 9180
ttattggata gggatagagc gaagagagta tttgattagg agtttggaaa tttgggatgt 9240
acgtttgttg gtgttagtga gttttgtttg attcgggata agtttttttt tttgtatgag 9300
ttttattttt tttttttgta aaataaggag ttggattttg tgatttatag gatgtttatt 9360
tagaattttg ttatgtgggt gttttaggta taaggaatta agggtagtaa ggaattaagg 9420
gtatatttaa aaagtttttt tagattttgt ggttgttgta gtttattttg aaaatatgtt 9480
tttagttttt tgttgtgcgt gatttttgtt tgttttattt agattttaat ttaaataatt 9540
ttgatttttg tttttggttt ggtaggattt tgaatggtag gaagtgagga taggagtttg 9600
tttatgtttg aggtagttag ggttgggggg gtattgagaa agggtaggta ggggtggaag 9660
ttgtgaggga gtgggaaaga gatatagaat agaaggggag ttgttggaga gatgtttttt 9720
ttattttaat tgggtgtttt ttgcggtttt tttaatttgt ttgtagtttg agtttagtag 9780
taggagtgta gagttagggt ttttttaagt ttttagttat ttaaatattg ttttttattt 9840
tttattttta ataggatggt ttttatgggg aagtgaatta ggattttttt tgtaggtttc 9900
gttttaaagt taggcggtag ggagtaggga ggcggttttt ttgttttttt tttaaaaaaa 9960
attttgtgat tttttttggg taggagggag ttacggttag cggttgttat attttatggt 10020
ttttatttta ttttttttgt ttttttttgt agg 10053

<210> 8
<211> 10053
<212> DNA
<213> Artificial Sequence

<220>
<223> chemically treated genomic DNA (Homo sapiens)

<220>
<221> unsure
<222> (10024)

<400> 8

tttgtagaga ggagtaagag aggtggagta agagttatga agtgtgatag tcgttggtcg 60
tggttttttt ttgtttagag gaaattatag agtttttttt ggaggggagg tagggaggtc 120
gtttttttat tttttgtcgt ttggttttgg ggcggggttt gtaggggaag ttttggttta 180
tttttttatg gaaattattt tgttagaggt gaagggtgga gagtaatgtt taagtgattg 240
agagtttggg gaaattttag ttttgtattt ttgttgttga gtttagattg taggtaggtt 300
gggagagtcg tagggagtat ttaattgggg tagaaggagt atttttttag tagttttttt 360
tttgttttgt gttttttttt tattttttta tagtttttat ttttgtttat ttttttttaa 420
tgttttttta gttttggttg ttttaaatat aaataggttt ttgtttttat tttttgttat 480

ttagggtttt gttaagttag gaataggaat taagattgtt tagattgggg tttggataga 540
ataggtagga gttacgtata gtagggaatt gagggtatat ttttagggtg ggttgtagta 600
gttataaagt ttggaggggt tttttaaata tgtttttagt tttttgttat ttttagtttt 660
ttgtgtttgg gatatttata tgatagaatt ttaggtggat attttgtgaa ttataaaatt 720
tagttttttg ttttatagaa gagaggagta aggtttatat agggaagaag atttgtttcg 780
ggttagatag gatttattgg tattagtagg cgtatatttt aggtttttag atttttaatt 840
aagtgttttt ttcgttttgt ttttatttag tggtggtagt ttatgtttga gtttttaggg 900
ttataataat tatgtaaagt tcggacgggg tagggttatg ttaggtgtag agaaattttt 960
ttgtaagttt attagagtat aagttgtttt ttcggagttg ttttttggtt tttttttttt 1020
tttttgttat ttaggaaagg gtgggtggag ggttagtgat gtggtagggg aagtgaaata 1080
tatttagata ttgaatgttt gattttttat ggttgattag ggttgaattt ttttagaagt 1140
ttgttggaga gtgttagggg gagtaatttt ggtttatgat agtttttaat ttaggtttta 1200
tattaattag tgtttttgaa gttatttagt ttttattttt tataggttta gaattagaag 1260
atattggtta aagttgtagt aagaaggatt ttggttagat agtaatagga ttttttgaat 1320
ggtggagtat gtgtaggtgt gtgtttgggg attggatttt taggtagggt ataaggtagg 1380
gagattgttt tttggtatga tatggtgagt ttgatagggt tgggtatatg gttatagagg 1440
ttttagaaag tttatagttt aagaatggtg ttaaatgaag atttaagatg ttgaggtttt 1500
gagggtagta tggttagggg tggggtaggg gggttttata ggtttttttg gttggtggta 1560
ggtttggttt ttttgtttgg gttttttttg ttttagaggt ttaggttggg ggaggaaata 1620
gagttttggg ttgttgtagt tgtgttttta gtttttgttt gattttttgt tttttgtttt 1680
tttcgtagtt agtgaggatt ttggttagta ttgtgcggta ggattgacgg tgggatggga 1740
tggagagagt attttagagg agttgaattt gtttattggg gtattagtag gaagttattt 1800
ttggtagttt ttgttagttt agaaagttgt aaaggagagg gtttgaggtg gaaaggaaaa 1860
agtggttaat aataaggtta aaattcggta tttttttagg attttttgtt cgtattttta 1920
tttttttggt cgtgttttag tttatttttt ttttatttta ttacgtatta gaattatttg 1980
aagtgtttat tgagaaaagt atatttattg gttttatttt tattttagat tttgatttag 2040
ttttggaatt agtaagaaat atgtattttt taaaagtttt ttagggagtt ttgattagtt 2100
tgtgtgggta taacgttaag tttggttttt ttgtttttat tttgttgtat agggttttgt 2160
tgggtgagtg ggagtgggat gtagagggag gggatattta tttttattta ggatattttt 2220
tatttattat agaagggata gttaagggta aatattaaag ttgggacgtg gtgatattgt 2280
ggagcgaggt tgtttgtatt tgtagaattt atttatggtt taattttggg aatgtgatgg 2340
agtaaaattg ggattgttta atttttaatt ggatttagtg tgagattaag tataggagga 2400
tgttgagttt cgattagttt aaatttagag ttgattttgt acgatttatt ttgaaatttg 2460
attgtagtat agatagattt ttggggtttg gtaggatttt tttattttta agtaaaattt 2520
tgtgaaagat tttttgttga tagaagtatt attattgttg gttagaaatt atagacgtta 2580
aattaatttt aaacgttagt taaatattat gaaatattta ataataatag ttaatttttt 2640
ttgagtatta attatgtgtt aagtattgaa tttgaatggt ttatttgatt taattattat 2700
aatagtttaa cggggaaag tgaggtttag agatgtttag tttttttgttt atagttattg 2760
agcgtgtttt aataaattta aattgttgat aataaattat tttttggtta ttgttatttt 2820
tttagttgtt tggggttaga tgagtttttt aaaggaagaa aaagatatgg gtttcggtgg 2880
acgtgtataa attgtttatt attgttgtta ttaacgggtg ttttatttag atttgtttaa 2940
atgaagatag cgtttttgtt tgtttttaat tttttttgag atagggtttt gtttagtcgt 3000
ttaggttgga gtgtagtggt gtaattatag tttattgtag ttttgaattt ttgggtttaa 3060
gtgatttttt tattttagtt ttttaagttt taagggatta taggggtgta gtattatgtt 3120
tagttttatt tttagtagag atagggtttt attatgttga tttaggtaat aaaggtggtg 3180
ttgttgttta gaaattattt gtttgttata ggtttagtta aagtttgagt tttaggtggg 3240
gagagttaga gtagtggtat gttcgaaagg tgtttattaa tgtttttttt tttagtatat 3300
ttgaaggatt gataaaattt atttttatta acggtagtgg tttagtatag tagtgatttt 3360
tatagggttg gagaagcgtt atattaagta taattttttg gatttatttt gtattttagt 3420
aggttttatt ttaatagtag aggagtagat ttagttgttt atattgggtt gtttttacga 3480
tttatttatg tgatttatta gttttttata gcggggtgat gtagtggaat atttaggtga 3540
tgtttggatt tttaggtttt aggtagatta tggaggtagt atagtgtagt atagaggtta 3600
gggtgtgggt tttggagtat ggagatttgg gtttaaattt tgtttttgtt atttagaggt 3660
atgaaatttg agacgttatt taaatttttt gaggtttagg ttttttatta tatgtaaagt 3720
gggaataata atagtgttat tttataggta aaatgtagta taattattta gtagagtata 3780
agtagtatat agtaagtatt gaaatgttat taagatttta attatgttat ttattttatt 3840
atttaaggag ttttggataa gttttatatt tatgtttgtt tttttttttg taggaagggg 3900
atagtaattt tttttttatt aggttgcggt taggattata tgagaaaaaa tatatatatt 3960
taagtgttta tatagttagt atttaattaa tggtagttat ttttaataaa ttaatgaaat 4020
ggggggatag gtgagaggat aggtattagg atagattttg atttagttag aagttggtta 4080
tatatagaga gagtgagagt aattgtttat tatgtttttg tttttggaga aggaaagagg 4140
agttggggta gttagtttag ttatgggttt tatagtattg gtttttatgg aaaaatttgg 4200
ggttttagtt tgggggggaga ttgggatttt ttttggtttg gattttgaag gaaggtcgtg 4260
gtaaggaggt gattgattat gcgcgtttag ggtgagattt ttaggttgga gtgatagtgt 4320
tagatttttt aagtaatagt tggaaggatt gatttgggaa aggtgagttt tgttgggagg 4380
tttaatagtt gtggaagtag gtatttattt taggtttatt tagcggtttg gaggacgtgt 4440

gatttttttt atgtgagtag agtaagatag gaattttatt gtttatttta tgaagagggt 4500
aggagaatag agaagtgaag ttattgattt taggttattc gattagaatt aagtttttga 4560
tttttagttt agtttcgttt attttttttt aagttttagg tagggttatg ggagttgtgc 4620
gtggttagtt aggagtagtt tgttttttta atgagttggg attgagtttt tcgcgattat 4680
tttgcggtga ttttggggtt cgttcgaagg aagggtttat tgaagcgggg gttaggtcgt 4740
tcgttattcg gtttcgttcg cggatttcga tttatttttg ggttcgtttt gcgggtggcg 4800
gggcgttagt tttcggcgga tcggggttat tttttttttta tcgcgcggtc gtagttgtat 4860
cgttagcgtt ggacggcggg ttggggttcg ggcgcggttt cggatcgggt tggagtcggg 4920
gaggggttcg ttcggcgtag ttggcgcggt agtttttcgg gattgggatg ggggttggag 4980
taaaaatgtt ttttatttta gcgttttttt gtttggttta gttttattat ttttttttaa 5040
taaatcgttt ttttattgtt tagttcgtcg attggtcgtc gttcgttttt cgttggggtt 5100
gtaggatcgg gatttatttt cgcgtcgttt ttagtttttt agggcggttt ttttttttag 5160
tcgttcgtag ttttcgggtc gttggttcgt attcgggtcg ggttttttat attcgttttt 5220
tcgagggggc gggttttgga tttaggtcgt gggggttttg tgggggtttt ataggggttt 5280
cgtggggttc ggatttatag tcgtgggaga tagagtcgag attgtaggta acgtttggcg 5340
gtcggagtag tgggtggggg tagaggggga tcggttggta gtagcggagg ggtcgtttgg 5400
aatcggggcg tcgggaaggg gttatcgagg aagggatagg cgggggtggg acggggaggg 5460
atattattta tttttatttt atttttagtt cgggggtttt cggtttagga ggtttgtagt 5520
aatgagggta gagtttgggg ttagtaaaat ttagatgcgt tttgatttcg ttttcgggaa 5580
ttttcgattt ggtttaaatt ttttagtttt ttttttcggg ttttttcgtt ttttatttgt 5640
ttttttttgt ttttagtagt ttttttcgat tcgtatttag tataggggta tataggttta 5700
gaggacggtt ttgggttagt tttgaggatg gtttgagttt tttttggtcg ttgtgtagtt 5760
gggtgataaa gatattttcg tttcgttagg gatagttagg atggagggat acgttgggag 5820
tagttttgga tttttttttt ttaggtaggg tttttatttt agtaaatgga gttttagata 5880
gaggtgggga atggagtttt tttggaattt tgttgatata aggtagttgg ggagggaagt 5940
agaatggttt tatatcggta ggggaggggg atatagattt tttttgatgt ttttgtatag 6000
gttgggagta ttgattttat tgttttgttt tgttttaatt atttaagtat ttgtaggatt 6060
ttggttttag ttttagtttt ttggttattt atagagattt tgaagttaaa ttatttatta 6120
ggaatgtagg ttggttgtgt tatcgtaggt tagtgatatt tttttatgtt ttagtttttt 6180
atttgttaaa taggtatagt aaatcgtgtt agttttatag ggtggttgtg agaatagacg 6240
gaaatgatgt ttattttagt ttatagtaaa gatgttggtt ggttgagttt gatttagagg 6300
aggtttttaa tgaaggcgtt cgttaattaa atgaataaat gaaaaggttg gggagggggt 6360
ggttttagag atatttggaa agacgtgggt attagaagta ggagtagatt tttaaggtgt 6420
tttattttaa tatggggatt ttataagatg agttagggat tttttttttt gtttcgtttt 6480
attttttgat aggaatagag gttgggggag ataatggtta aaagtttaga taggtattaa 6540
ttattgattg attttatttt ttttgttgaa atttttttta ttttttattt tatagttgtt 6600
ttataagttg tgagcgttgt gaatttttag aattttgata tatttaaatt ttagtgttag 6660
gatagaattg tgttttgtag ttaataggaa gtattatggg attacgatat ttgttttttt 6720
agattatagc ggttatttgg gttttttacg agttggagaa ttgggagttt ggtcgcgggt 6780
ttgtttttta tatttttttt ttttgtttcg ttatggtttt tggttaattt tttttttttt 6840
taatttttgg atttttgttt tgagaagcga gagtcggggt tttttaaagg aagtttttag 6900
tttgagaggt atattgagta tagaaatttc gagatggata aaagttgaga gttattttta 6960
atagttagtg ggggatggaa gattatttgg attagcgttc gtgaaataga taaaaataga 7020
gtatattttg ggagttggaa gagaggattt agggggggtt ggagtttgtt tattagtgtt 7080
ggaatttttt ttattttttt agagtattga gttttttggt ttttgattgg tgttttttgg 7140
ggtttttgtg aattttatgt tttttaaagt atatagagaa tttgggggag gtgttagagt 7200
tggagattta gagttggaga aagttttgga gagttatttt aatgagaaaa gggaaaagga 7260
attatagatt gtgtttaagt ttataagaat taattattta gagggattag ttgtttttta 7320
tatttagtga ggatagaata agaggaaata ggtttaaagg ataaagattt agatttagat 7380
atttagaagg attttttaat attgtaggaa gtagaggttt ggagattttg tgtggggtga 7440
aattttaagt agatttttat ttgtggaagg tgggtgatat ggagtttgtt aggaaggtag 7500
ggtaatggat atttttattt tttaggatta gttatgagta ttaagtttaa gtattatttt 7560
tttttttttg agatggagtt ttatagtgtc gtttaggttg gagtgtagtt gcgtgatttt 7620
aggtttgttt tttaggttta agtgattttt ttgttttagt tttttaagta gttgggatta 7680
tagatgcgtg tttttttacgtt tggttaatgt ttgtattttt agtagagacg ggttttattt 7740
atgttgtata ggttggtttc gaatttttga ttttagttga tttatttatt tagtttttta 7800
aagtgttgag attataggta taagttattt tatttagatt taaatattat ttttgatatt 7860
agtgcggggt tggttttatg ggagtgtgat ttgtgtagat gtatagggtt ttatgtttgg 7920
tttaatgcgt tgttattgtt attttaaaat ttttaatgat atattttttt tttatttatt 7980
taggtttttt ttatttatta gtgtttttgt agtttttagt atttaatttt tttatgtgtt 8040
ttgttatatt tatatttttt aaaagtaatt ataaatagga tatgttattt ttaatttttg 8100
tgtttatttg tttattaaat tatatagaaa tataatcgat ttttatatat tgattttgta 8160
tattgtgatt ttgttgaatt tatttattta agggggtgag gtatttgggt tttttatttt 8220
ttatggattt tttgggattt tttatatcga tagttatttg tatttgtaaa tagagatagt 8280
attatttttt attttttaat atgtatattt tttatttttt ttttttattt gattgtatta 8340
gttaaaattt ttagtattat gttgaataag agtggttaaa atagaatagt tatttttgtt 8400

ttgttttttt taggagaata tatttaattt attaattata atgttagtcg taagtttttt 8460
gtagtagtta ttttatatta gattgagaaa gtttttttat tttttttttg tgtgtgtgtg 8520
tgtattttta ttatggatgg ttattgaatt ttgttaaatg ttttttttgt atttattgat 8580
acgaattgtg taattttttt ttgttagttt gttaatacgg tagattatgt tgattgattt 8640
tttttttttt tttttttttt tttttttttt ttgaaataga gttttgtttt tgttgtttag 8700
gttggagtat aatggcgtga tttcggttta ttgtaatatt tgttttttgg gtttaagcga 8760
tttttttgtt ttagtttttt tagtagttag gattataggt gtttgttatt atatttggtt 8820
aattttgtat ttttagtaga gaaggggttt ttttatgttg gttagtttgg ttttgaattt 8880
tcgattttag gtgatttatt cgtttcggtt ttttaaagtg ttgggattag aggcgtgagt 8940
tatcgtgttt agtttatgtt gattgatttt ttaatagtga attaggtttt atatttttgg 9000
aataaattta tttggttata atgtgtattt ttttattata tatagtttaa ttttatttgt 9060
taatatagta atttgttgta taatgaagtt ttagttaaaa atattttata tatataatga 9120
tggtcgtata agattataaa tggagttaaa aaatttttgt tgtttattga cgtcgtagta 9180
acgtatcgta ttatttatgt gtttgtgttg atgttggtat aaataaattt attgcgttgt 9240
tagttataga aacgtatata gtatgtatag ttatgtttaa taaataatat ttgatgataa 9300
atgataatgt tattggttta tgtatttaat attttatatt ttttattatt atattttaga 9360
gtgtgtattt ttttattttt aaaaaagtta attttaaagt agttttaagt aagtttttta 9420
ggaagtattt tagaaggtat tgttattata ggagatagta gttttatata ttattgtttt 9480
tgaagattta ttagtgggat aggatgtgga ggtggaagat agtgataatg atgattttga 9540
ttttgtgtaa gtttaggtta atgtgtgtgt ttaataaaaa aaaaaaatta agtataaaaa 9600
tgaaattaaa agttttaaat atagaaaaaa atttatagaa gtcgggcgcg gtggtttatg 9660
tttgtaattt tagtattttg ggaggttgag gtgggtagat tatttaaggt taggagtttg 9720
agattagtta ggttaatagg gtgaaatttt atttttatta aaaatataaa aaatagtcgg 9780
gcgtggtggc gggtatcggt aagtttagtt attagggagg ttgaggtttg agtttttttt 9840
taatttaaga ggtttaggtc ggggtgagtc gagatttcgt tattgtattt tagtttgggc 9900
gataagagta aaattgtttt ttaaaataaa ataattagtt tttgtttagt ttaaggatta 9960
ttttttttgt ttttttattt tgttgttgcg tttatttaat gagtttttat tttagttatt 10020
gttntttgta gttttaaaat gtttatttgg ttt 10053

<210> 9
<211> 6020
<212> DNA
<213> Artificial Sequence

<220>
<223> chemically treated genomic DNA (Homo sapiens)

<400> 9

tttttaaagt gttgggatta taggtgtgag ttcgttcggt cgagagtgag gttttttttt 60
taggtgttaa atttaagagg ggttaggcgt ggtggtttat atttgtaatt ttagtatttt 120
gggaggttta ggagggtaga ttgttggagt ttagaagttt aagattagtt tgggtaatat 180
ggtgaaattt tatttttatg aaaaataaaa attagtcggg cgtggtggta tacgtttttt 240
atttcggata ttcgagaagt tgaggtgggt agattgtttg agtttgggag gtcgaggttt 300
tagtgagtcg aagttatatt attgcgtttt agcgtgggtg atagagtaag aattttgttt 360
taaaaaaaaa taaaaaagtt aaggggatgt taaatattta agttattagg ataaatatta 420
ttttgatgta atttattttt attttttgag atggagtttt tttttgtcgt ttagggtgga 480
atgcgatggc gttaattgaa gaattttgtt ttaaaatttt ttgtggtttt ttagtgtttt 540
ttaggtaaaa ttttagtttt ttagtttagt agtacgtgtt gttatcgtgt ttcggttaga 600
agttatattg tagagtgttt aaaggtttaa ggttaagatt aaaggtttgg gataatatcg 660
tggttttgtt ttttatttat ttggttaagt tattttattt ttttgtgatt tatttttttt 720
atttttttttc gttttttttt tttttatttt ttattttttgg agatagagtt ttattttgtc 780
gtttaggttg gagtgtagtg gtacgatttc ggtttattga aattttcgtt tttcgggttt 840
aagcgttttt tttgttttag tttttagagt agttgggatt ataggcgttt attattacgt 900
tttaattttt gtatttttag tagagacggg gtttcgttat gttggttagg ttagtattaa 960
atttttgatt tttaagtgat ttgtttgttt cggtttttta aagtgttggg atgataggtt 1020
tgagttattt tgtcggtttg tttttttttt atttttaaaa tgggtgtgat aattgtattt 1080
aaaggttaaa gtgtttaagg tagtttttgg tgcggagtaa aaatatatag atttgtgtgg 1140
tttggttgat tatttatagt gtgggttatt attttgggtt gttttttatg tagaaaaaaa 1200
atttattttt taaaaacgat gattttataa tgattttttt ttttataggt ttaggttttt 1260
gtgggtggtt tagtttttat tttatcgtgt ttaaggtatt tgtgtttagt tttgtttttt 1320
ttgatagtgt gggggtttcg aaaatggggt tgtttggtta ttgggcgggt atattgatat 1380
cggaaggcgt ttgtttaaat aaatattggt aggtggtttt ttaggagagg gggtgtttgg 1440
tttttacggt agtgggggtg gggtggtagg ggtgttagcg gtaggattgg ttttaggggt 1500
agtttaggaa cgtagttttt tagggttttg taggaaaaat aagggtgttg cggttggttg 1560
aaggtttggt tgttattaag tagtattaat ttttgttttt ttgttttttt tttttttgag 1620

atagagtttc gtttttgtcg tttaggttgg agtgtagtgg tataattttg gtttatcgta 1680
atttttattt tttaggttta agtaattttt ttgttttagt tttttaagtg gttaggatta 1740
taggcgtgag ttattatgtt aggttaattt tgtattttta gtagagacgg agttttttta 1800
tgttggttag gttggtttta agttttttat tttaggtgat tcgtttgttt cggtttttta 1860
aagcgttgag attataggtg tgagttatta tattcggtta ttaagtagta gtaattttaa 1920
aaatttaaaa ttttaatttt ttttagtttt attttttttt tttttttttt tttgagatag 1980
agttttattt tgttatttag gttggagtat agtggcgcga ttttggttta ttgtaattat 2040
tattttttag tttttagcga tttttttgtt ttagttttta agtagttgga attagaggcg 2100
ttcgttaaaa tatttcggta atttttgtat ttgtagtagt gacgaggttt tattatgttg 2160
gttaggttgg tttcgaattt ttgagtttgt gatttattgg tttcggtttt ttaaagtgtt 2220
gggattatag gtatgagtta ttgttttcgg tttataaaaa atgttaaaat tagttgggta 2280
tggtggtgta tttttgtagt tttagttatt taggatgttg aggtcggagg attatttgag 2340
tttaggagtg ggaggttgta gtgagttatg attgtattat tgtattttag tttatgtaat 2400
agaggattgt gtttgaaaaa atttttttag gtcgggtgtg gtggttcgcg tttgtaattt 2460
taggattttg ggaggtcgag gtaggtagat tacgaggtta ggagatcgag attattttgg 2520
ttaatatagt gaaatttcgt ttttattaaa aatataaaaa attagtcggg cgtggtggtg 2580
ggcgtttgta gttttagtta ttcgggaggt tgaggtagga gaatggtata aattcgggag 2640
gtggagtttg tagtgagtcg agatggtatt attgtatttt agtttgggtt atatagtgag 2700
attttatttt aaaaaaaaaa ttttttttta ataaaaatat taaaatattg ttttaaagat 2760
tattaatttt gattattgaa ggttattatt tttatttgtt aaggtaagga gtttggggtt 2820
ttaagtttat tttatagatg ggaatattta aggtttaaat taattgtatt tatttaaggg 2880
agtaggttgt ttagtggatg acgatgatga agagattgtg tttcgtattt gttaaagggg 2940
gattttaatg ttatagtttt attttagtag tttttttggg atttgttttt tgcgtggtag 3000
agagagggat tttttaataa tcgttattag gttttatttt tttttttttt acggtttttt 3060
aggattttta ttttatttag agtaaaagtt taagtttagg gttaggcgta gtggtttata 3120
tttgtaattt ttgtattttg ggaggtggag gcgggaggat tatttgatgt taggatttta 3180
agattagttt ggttaatatg gtgaaatttg tttttattaa atatataaaa attagttagg 3240
cgtggtgatg cgtatttgta attttagtta tttaggaggt agaggtagga gaattatttg 3300
aattcgggtg gtagaggttg tagttagtta atattatatt attgtatttt agtttgggta 3360
atagagtgag attttatttt aaaaataaat aaatagttga ttacggtggt ttacgtttgt 3420
aattttagta ttttgggagg tcgaggcggg tagattattt gaggtcggga gtttaagatt 3480
agtttgatta atatggagaa atttcgtttt tattaaaatt ttaaaattag cggggcgtgg 3540
tggtacgtgt ttgtaattta gttattcggg aggttgaggt aggaattatt tgaatttagg 3600
atgcggtgag ttgagattgt gttattgtat tttagtttgg gtaataagag cgaaatttta 3660
ttttaaaaaa taaattaatt aattattagg tttcgttttt ttttttttta tagtttttta 3720
gggtttttat tttatttaga gtaaaatttt aagttttttt taagatttat aagtttttgt 3780
tggattggtt tgttttatta gttatagagt tagtagggtt tagtgtaaaa gaaaatttaa 3840
aggttttttg tttaaaaatg attagaaatt ttaaaatgta gtagtaaata ttaatttaag 3900
tataagtttt gttgtggggt gggggggttt tggataaaag tacgaagttt tcggttttgt 3960
tttttttcgg ttttttgat ttttttcgg gtatttgtt gttaggtata gtttgtttt 4020
agggtttttg tatttgttgg ttttgttttt gattattttt gtattttatt tttttgtttt 4080
tttttttttt ttaggtcgtt ttaggggtgg aggaagtcgt ttttgatttt tttttttatt 4140
tttttaattt tgtatttttt tattttgtta ttttttcggt tggttttttt gtagaattgt 4200
ttttattatt tgatttgtta ggaatttagt ttgtgaattt gtttttgttt gtttttaggt 4260
attattatta gggtagggat ttattttcgt ttcgttttat tttagggttt tggagtagcg 4320
gggtttggta tatagtaggc gttttataaa tgtgttataa gaatggtttt ttttaggaag 4380
gcggtattat cgttgttttt ttgtagaaaa ggaaattaag gtttagtaag aattatcgtt 4440
tagttagagt taaggttata gagtacgttc gtggtacgcg tttaggtcgt gttatagtag 4500
ggtcggcggt ttcgacgcgg gtagtcgggt tttttttttt tgatagatgg ggaaatcgag 4560
gttcggtcgg ttgtcgggta ggagagggtg gcgggggtcg ttgttcgggg cgggggcggg 4620
tcgggtttaa ggttatcgcg gcgggcgggt tacggggagg cgggtcggcg tggggattcg 4680
taggtttttt cgggcgcgcg cgtttttttt ttcggcggcg cgtacggggt acgggcggcg 4740
gtaggtggag tcgaagtttt cgtagtagtc gagtcgcggt tattgtcggc gtcgtcgtta 4800
ttagataata gcggtggggt cggggagcgg ggaggcgggc ggcggggagg ggcggggagg 4860
ggcgtcgggc gcgtaggaag cggggttcgg ggcgcgtcgg ggtcgggcgc gcggggaggg 4920
ggcgtaggaa gcgggcgagg tcgggcgcgg gaggcggcgg cggtttcggg agttgtcgat 4980
cggcgtcggg ataaaggcgc ggtcgttcgg cgtttttagt agttcgagtc ggggcgtata 5040
gtcggggcgt agttcgcgtt tttcgtcgcg attgatatga tgtttttata aagtaggtat 5100
tcggtaagcg gggttcgttc ggtcggcgtt tttatttcgg attcgggttt tacgttttcg 5160
attttcggtt ttcggtttcg cgttttttaa tttttaggtt cggtgttttc ggttcggcgt 5220
tttttttaat ttttcggttt tgtttttttt ttttagtttt tagttcggcg cgattttttt 5280
ttttttttgt cgtcggtttt cggatatttt ttttttagtt ttcgatttat tttagttttc 5340
gggtttcgag ttttttcgtc gtagttttcg gtttggcgtt tttttttttt aattcgtcgg 5400
gatagttagg gtttcggacg gttttggttt cgggtttggg gcgtttcgga agtttcgcga 5460
ggtattttta gttcggggat tttttggggg tttgtggtgg ggggggtgat tcgaggcgta 5520
tttttttcgg aagagcgtgt tttcgaggaa gggcgttggg aatttggggt tgggtttttt 5580

cggaagattt cgagtatcga gagagagatt ttttttttaa gaggattttg tagggaggaa 5640
tcgttgtttt ttaatttggg gaggggtttt tttagggcgc gtttttattt ttttcggatg 5700
tgtatttttt ttcggcgttt tggagggcgg tagagggcgt attttgtttt ttatttaggg 5760
cgggttttaa aacgtatttt tattgtttgg gggtggcggg ggacggggcg gtttcgggcg 5820
tatttattcg ttcggttttt ttcgggagag gcggggggta ggaagcgcgg gtggggtcgc 5880
gttaggggtc gtaaatacgg gtttaggaag tggggtagga gggggggcgt cgtagttgcg 5940
cggtcgtttt tttttagtcg gtttttgagg ggggtttagt ttacgtttgg gtttgattcg 6000
ggtcgtttta tttttatagg 6020

<210> 10
<211> 6020
<212> DNA
<213> Artificial Sequence

<220>
<223> chemically treated genomic DNA (Homo sapiens)

<400> 10

tttgtaggga tggggcggtt cgggttaggt ttaggcgtgg gttggatttt ttttaagggt 60
cggttaggag ggagcggtcg cgtagttgcg gcgttttttt ttttgtttta ttttttgggt 120
tcgtgtttac ggtttttggc gcgattttat tcgcgttttt tgtttttcgt ttttttcggg 180
ggaagtcgag cgggtgggtg cgttcggagt cgtttcgttt ttcgttattt ttaggtagtg 240
ggggtgcgtt ttaaggttcg ttttagatgg ggggtagggt gcgtttttta tcgtttttta 300
aagcgtcggg agggagtgta tattcgaggg gggtagaaac gcgttttagg gagttttttt 360
tttaagttgg ggggtagcga tttttttttg taaagttttt ttgggggagg ggtttttttt 420
tcggtgttcg aaatttttcg ggggggttta attttagatt tttagcgttt tttttcgggg 480
atacgttttt tcgggagggg tgcgtttcgg attatttttt ttattatagg tttttaaagg 540
attttcgggt tagaggtgtt tcgcggggtt ttcggagcgt tttaggttcg aggttaaggt 600
cgttcgaggt tttgattgtt tcggcgggtt gggggggggg ggcgttaagt cgggagttgc 660
ggcggggggg ttcggggttc ggaagttggg gtggatcggg aattggaggg ggggtgttcg 720
ggggtcggcg gtaggagggg aggggggtcg cgtcgggttg ggagttggag agggaagata 780
gggtcgggga gttgggggg gcgtcggtc ggaggtatcg ggtttgggg ttgggggcg 840
cggggtcggg agtcgggggt cgggggcgta gggttcggat tcggggtggg ggcgtcggtc 900
ggacgggttt cgtttatcga atgtttgttt tgtggaaata ttatgttaat cgcggcgggg 960
ggcgcgggtt gcgtttcggt tgtgcgtttc ggttcgggtt gttgggggcg tcgggcggtc 1020
gcgtttttgt ttcggcgtcg atcggtagtt ttcggggtcg tcgtcgtttt tcgcgttcgg 1080
tttcgttcgt tttttgcgtt tttttttcgc gcgttcggtt tcggcgcgtt tcgggtttcg 1140
ttttttgcgc gttcggcgtt ttttttcgtt ttttttcgtc gttcgttttt tcgtttttcg 1200
gttttatcgt tattgtttaa tggcggcgac gtcggtagtg gtcgcggttc ggttgttgcg 1260
gaggtttcgg ttttatttgt cgtcgttcgt gtttcgtgcg cgtcgtcgag gggggggacg 1320
cgcgcgttcg gggaggtttg cggattttta cgtcggttcg ttttttcgtg gttcgttcgt 1380
cgcggtgatt ttgggttcgg ttcgttttcg tttcgggtag cggttttcgt tatttttttt 1440
tattcggtag tcggtcgggt ttcggttttt ttatttgtta aagaaaaggg attcggttgt 1500
tcgcgtcgaa gtcgtcggtt ttgttgtggt acgatttgga cgcgtgttac ggacgtgttt 1560
tgtgattttg gttttggttg ggcggtggtt tttgttgagt tttagttttt ttttttgtag 1620
aaaggtagcg gtaatgtcgt ttttttgagg gaagttattt ttgtgatata tttatagaac 1680
gtttattgtg tgttaggttt cgttgtttta gggttttggg gtggggcggg gcgggggtgg 1740
gtttttgttt tggtggtggt gtttggagat agatagagat aaatttataa gttgaatttt 1800
tggtaggtta gatggtgggg atagttttgt agaaaaatta gtcgggagaa tggtagggta 1860
ggaaggtgta aagttaaaga ggtgggaggg aggattagag acggtttttt ttatttttaa 1920
agcgatttga agaaggaggg agaatagggg gatggggtat agaggtagtt agaggtagga 1980
ttaataggtg taaaggtttt ggggtaggat tgtgtttggt agtagagtgt tcggggggag 2040
gttaggaagg tcggagggag gtagggtcgg gggtttcgtg tttttgttta gggttttttt 2100
attttatagt agggtttgtg tttgggttaa tatttattgt tgtattttga gatttttaat 2160
tatttttgaa taaggggttt ttgagttttt ttttgtattg ggttttgtta attttgtagt 2220
tggtgaagta ggttagttta gtaaggattt gtgggttttg gggaggattt gggattttgt 2280
tttgagtgag gtaggagttt tggaaggttg tgggaggagg agggacggga tttggtggtt 2340
ggttggtttg ttttttgaga tggagtttcg tttttgttgt ttaggttgga gtataatggt 2400
ataattttag tttatcgtat tttgggttta agtgattttt gtttagtttt ttcgagtagt 2460
tggattatag gtacgtgtta ttacgtttcg ttaattttgg agttttagta gagacggggt 2520
ttttttatgt tggttaggtt ggttttgaat tttcgatttt agatgatttg ttcgtttcgg 2580
ttttttaaag tgttgggatt ataggcgtga gttatcgtga ttagttgttt gtttgttttt 2640
gagatggagt tttattttgt tgtttaggtt ggagtgtagt gatgtgatgt tggttgattg 2700
tagtttttgt tattcggatt taagtgattt ttttgttttt gttttttgag tagttgggat 2760
tataggtgcg tattattacg tttggttaat ttttgtatat ttagtagaga taggttttat 2820

tatattggtt agattggttt tgaaattttg atattaggtg attttttcgt ttttattttt 2880
tagagtgtag ggattatagg tgtgagttat tgcgtttggt tttggatttg ggtttttgtt 2940
ttgagtgagg tgggaatttt ggagagtcgt gggaagagga gggatgggat ttggtggcgg 3000
ttattaaagg attttttttt ttgttacgta gagaataggt tttaaagagg ttattgaaat 3060
aaggttatga tattaaggtt tttttttggt aggtgcgaaa tatagttttt ttattatcgt 3120
tatttattag gtagtttgtt tttttgaatg aatgtaattg atttgggttt tgggtgtttt 3180
tatttgtaaa atagatttgg aattttaggt tttttatttt ggtaggtaag ggtgatagtt 3240
tttagtgatt aagattaata atttttaaag tagtgtttta atatttttat taaaaaaaaa 3300
tttttttttt gaggtggagt tttattgtgt ggtttaggtt ggagtgtagt ggtgttattt 3360
cggtttattg taagttttat ttttcgggtt tatgttattt ttttgtttta gtttttcgag 3420
tagttggggt tataggcgtt tattattacg ttcggttaat tttttgtatt tttagtagag 3480
acggagtttt attgtgttag ttaggatggt ttcgattttt tgatttcgtg atttgtttgt 3540
ttcggttttt taaagttttg ggattatagg cgcgagttat tatattcggt ttaaaaaaat 3600
ttttttagat atagtttttt gttgtatagg ttagagtgta gtggtgtaat tatagtttat 3660
tgtagttttt tatttttggg tttaagtgat ttttcgattt tagtattttg agtagttggg 3720
attataggag tgtattatta tgtttagtta attttaatat tttttgtaag tcgggagtag 3780
tggtttatgt ttgtagtttt agtattttgg gaagtcgagg ttagtggatt ataagtttag 3840
gagttcgaga ttagtttggt taatatggtg aaatttcgtt attattataa atataaaaat 3900
tatcggggtg ttttggcggg cgtttttaat tttagttatt tggaggttga ggtaggagaa 3960
tcgttggaag ttgggaggtg gtggttgtag tgagttaaga tcgcgttatt gtattttagt 4020
ttgggtgata gagtgagatt ttgttttaaa aaaaaaaaaa aaaaaaagat gaggttagaa 4080
aggattagga ttttggattt ttaagattat tattatttgg tggtcgggtg tggtggttta 4140
tatttgtaat tttagcgttt tgggaggtcg aggtaggcgg attatttgag gtgaggagtt 4200
tgagattagt ttgattaata tggagaaatt tcgtttttat taaaaatata aaattagttt 4260
ggtatggtgg tttacgtttg taattttagt tatttgggag gttgaggtag gagaattgtt 4320
tgaatttggg aggtggaggt tgcggtgagt taagattgtg ttattgtatt ttagtttggg 4380
cgataagagc gaaattttgt tttaaaaaaa aaagaaataa agaaatagag attaatatta 4440
tttagtaata gttaagtttt taattaatcg taatattttt gttttttttg tagggttttg 4500
agagattgcg tttttagatt gtttttgaag ttagttttgt cgttagtatt tttgttattt 4560
tatttttatt gtcgtgggga ttagatattt ttttttttgg gaggttattt gttagtgttt 4620
atttaggtag gcgtttttcg gtattagtgt gttcgtttag tgattaaata gttttatttt 4680
cgaagttttt atattgttag gggaagtaga attggatata gatattttga gtacggtgag 4740
gtgagggttg ggttatttat agggatttag gtttgtgaag gagagagtta ttgtgaaatt 4800
atcgttttta gaaaataggt tttttttttg tatagaaggt agtttagaat ggtggtttat 4860
attataaata attaattaag ttatataggt ttgtatgttt ttgtttcgta ttaggggttg 4920
ttttaagtat tttaattttt aggtatagtt gttatattta ttttagagat ggggggaaaa 4980
taggtcggta gggtggttta ggtttgttat tttagtattt taggaggtcg aggtaagtag 5040
attatttgag gattaggagt ttgatattag tttggttaat atggcgaaat ttcgttttta 5100
ttaaaaatat aaaaattaag gcgtggtggt gggcgtttgt aattttagtt attttggagg 5160
ttgaggtagg agaagcgttt gaattcggaa ggcggaggtt ttagtgagtc gagatcgtgt 5220
tattgtattt tagtttgggc gatagaatga gattttgttt ttaaaaataa aaaataaaaa 5280
aaaagaaaac gaaaaaaaat ggggaaagtg agttatagag aagtgaagtg atttggttaa 5340
gtgggtaggg ggtagaatta cgatgttatt ttaggttttt gattttgatt ttgggttttt 5400
gagtattttg tagtgtggtt tttggtcggg atacgatggt agtacgtgtt gttgggttga 5460
ggagttggga ttttatttgg agggtattag ggagttatag gaggttttag agtaagattt 5520
tttagttagc gttatcgtat tttattttgg gcgatagagg gagattttat tttaaaaaat 5580
aaaagtagat tgtattaaaa tagtgtttat tttgatgatt taggtatttg gtattttttt 5640
agttttttta tttttttttg agatagggtt tttattttgt tatttacgtt ggagcgtagt 5700
ggtgtgattt cggtttattg aagtttcgat tttttaggtt taagtaattt gtttatttta 5760
gtttttcgag tattcgggat gaaaagcgtg tgttattacg ttcggttaat ttttgttttt 5820
tatagagatg gggttttatt atgttattta gattggtttt gaatttttgg gttttagtag 5880
tttgtttttt tgggtttttt aaagtgttgg gattataggt gtgagttatt acgtttggtt 5940
ttttttaagt ttgatatttg aaaaaaggat tttattttcg gtcgggcggg tttatatttg 6000
taattttagt attttgggag 6020

<210> 11
<211> 6012
<212> DNA
<213> Artificial Sequence

<220>
<223> chemically treated genomic DNA (Homo sapiens)

<400> 11

tttataaaaa aaaaaaaaaa aatagtttgg tgtggtggtt tatatttgta gttttagtta 60

tttaggaggt tgagatagga agattatttg aatttaggaa ttgaaggttg tagtgagtta 120
tgattttatt attgtatttt agtttgggtg atatagtaag attttatttt taaaaaatat 180
ttttttaatt aaaaaaaatt aatttggggg tatagttatt aaaggttttt tgaggattag 240
aataaatttt taaggggtat tatttttagt ttttagtatt atttttggta tatatatggg 300
aagtagggaa ttagttgaga gtttagtttt tggagttaga ttaattcggg tttgaatttt 360
ggttttatta tttattggtt gagggagatg agataagtta tttatttttt ttgagttttt 420
gtttttttgg ttttaaaata ggattaatta tagtgtgttt tttttaggat tattgtgagt 480
attaaacgag atgaggttta tgtttaaaat gttgttgggt attatgttgg tatggattta 540
agtttggata tatgtaattt ttaaaggacg tagttgtgat aggtgtttat agatgagtta 600
tagatttaag gatgaataaa atttagtgta tgtttttttt agttagtttt tttgtagagg 660
tagataggtg ttataggtag tacgtgtttt tgttgtgatc gaggacggta aattggatta 720
taataggaat atgatggggg attttatgga ggaagggttt attattaaag atcgtgagga 780
ttgtagtttt tttttgatat atatttgagt tattttttga aattttgaga gtttatttat 840
tatttataga atgaagttaa tttttatagt ttaattttgg aggttttttt agaatttggt 900
tttattttgt ttttaatttt atttaatgtt atattatttg gatttgtttt tttttttttg 960
ttttgtgtgt ttagtttttt atttttagaa ggtttttttt ttatttttat atgttatatt 1020
gtatttgttt attaaggtta aatttaaatt ttattttttt tttgaagttt ttttaggttt 1080
tttaaattag atgatgtaga ttttgtattt ttttaatttt tagagttcgt tttttttgtt 1140
ttttattgag aagggttttt tttgtattgt aggaattttt tttgagataa ggtttttttt 1200
tattgtttag gttggggtga agtggttgta ttagtttatt tttacgttgt taataaatat 1260
atatttaaga ttgggtaatt tataaaggaa agaggtttaa atgatttata gtttagtata 1320
gttgggaagg ttttaggaag tatataatta tggtggaagg ggaagtaaat atattttttt 1380
ttatatgatg gtaggaagga gaagtgttga gtaaaggggg aaaagttttt tataaaatta 1440
ttagatttta tgagaattta ttattattaa gagaataata tgagggtaat tatttttatg 1500
attcgattat tttttattgg gtttttttta tgatatatgg gaattatggg aattataatt 1560
gaaaatgaga tttgggtgga gatatagtta aattatatta gtggtataat tagggtttat 1620
tgtagttttg attttttagg tttatgcgat ttttttattt tagtttttcg agtagttgga 1680
attatagttg tatattatta tggttggtta gtgttgtaga gataggggga agggttgggg 1740
gatgaggggt ggttttttta agttgtcggg gatgattttt ttatgttgtt taggttggtt 1800
ttaaattttt gggtttaagt aattttttgg ttttggtttt ttaatattgt aggatttatt 1860
tttttattgt atttttgttt tatatagtga gtttttgggg gtagatttat agttggttta 1920
tttatagttt tttgttagta tttgttatag agttttttat atggtgagtg tttgatagag 1980
gaaaggaaag aggaggaaag gtaagaagtg tttggaattt ttttggatga aaggtaatta 2040
tttttgttat gtgaggttta gaaagaaggt tgttttaagg agggaaagta gtgttattga 2100
aggatgttta tgggatgaag aggttttgtt tattgtttat ttagttaata ttgaattatt 2160
ttggttagga tgtattgatt ttatggatta gggtgggagg tgtttaagtt tattttttgg 2220
tttagtaatt tgttagaagg gtttatagaa tttagtaaaa ttgttatatt tatagttacg 2280
gtttattata gtaaaaggat atagatgaaa attagtaaag ggaagaggta taaagggtag 2340
gggttaggag agattaggta tgagttttta gttgtttttt tttagaggag ttatgtagat 2400
agtattgttt ttttttagaa ataatatgtg taatatttat agagttttgt taattaggga 2460
agtttatttg agttttggta tttaggggtt atattgcggt gtggttatat agatatggtt 2520
gattttagtt tttagttttt ttagaggtta ggttgatatt tcgtgtttta aggtttttat 2580
cgtatattat attattagtt taggttattt agtatggttt aagtttttta tataaataga 2640
tttttattag ataggatatt ttaagggttt agaggttgtt ttttggtagt tagggttaag 2700
ggtttaattt tttttcgggt aaagttaatt ttttgttgta tagaaggatt gttttgtgaa 2760
tgttttgaaa ttatatagaa aatttaggga gcgtgaatat tatttttttaa gaattgtgtt 2820
tatagttttt attagttttt aaagagaatt atgatttaag gagttaatta tatgtttttt 2880
ttttttttga gatggagttt cgtttttgtt gtttaggttg gagtgtaatg gcgtgatttt 2940
ggtttattgt aattttcgtt ttttaggttt aagtaatttt tttgttttag ttattatgtt 3000
tggttaattt tttgtatttt tagtagagac ggggtttttt tatgttgagg ttagtttcga 3060
atttttgatt ttaggtgatt tgttcgtttt agttttttaa agtgttggga ttataggcgt 3120
gagttattac gtttggtcgt taattatatg tgttttatga ggtgaggttt tttttagatt 3180
ttggaattag gggttgtaat tagggtttaa ataatgaggt tggattatag ataatttatt 3240
ttttttttta tttttgatta gatttaagga ttaaatttta agaattcgag tatttgtttt 3300
taaaattgaa aggattggat tagttatttt ttgttttttt atagttatat tttaggtatt 3360
tggtttttgt tttgtttaga aatttagtta taattttata tatttgatgg ttttttttgg 3420
taagtaggta tttttattag gatttttagt tgttagatat atttattgga ggttatttat 3480
taaatttggg tttaattttt atatagggag gttattgaag tattatattg ggttttttag 3540
ttttttttta tagaggaaag atttttttgt tttgtagggt tggtagttag cgttaagtaa 3600
agggaattta gtttttggtt taagattttt gtttaggaaa ggtatttgcg tttgttggaa 3660
attttgggtt gaagtatatt tttttttaaa aatttaggtt tgatatttat ataaagtttg 3720
aaattaatgt agagaaaatt tttaagtgtt tggattggag tagaaggttg agaataggaa 3780
ggggttggtt tttggtatta gttttggttt tttggtggtt tttttttttt ttgttttttt 3840
ttatagaata gggtaaagtt gagtgttttt ggatgagtga agtaggagga ttaattatgt 3900
ttagtgtttt tttattttaa attggttttt ttgggaattt gtaattgaga gtggggaggg 3960
gtaagaatcg tgggaaaagg ttgatggtgt ttagttaaat ttatttttta cgtgtttatt 4020

tttttatagg tatatgtttt ggggttattt acggttgtag ttattttatt tttaggaagt 4080
attattggtt ttttttttcg gtatttggat ttagtattat ttttagtttt ttggagatgt 4140
agtttttatt tagtatatag tttagttttg agttttgttt ttgtttttag atgtttttgg 4200
ggttatttat tattttttgt ttggtggttt aggtttattt ttttttattt ttgtattttt 4260
tttagtagaa aaggagtgag aatggatatt tttatgggtc gtgtgtgtat tttcggttat 4320
ttttgatagt tttatttaga gttatttttt tttttggggt tttttatgtg ttttaaggtt 4380
gaggtaggaa gattgtgaga ttaggtgata tttaatagtt atgatcggtt ttaagattaa 4440
tagttttggt cgggcgtagt ggtttacgtt tgtaatttta atattttggg aggtcgaggt 4500
aggtagatta cgagattagg agattaagat tattttggtt aatatagtga aatttcgttt 4560
ttattaaaaa tataaaaaat tagttaggcg tggtggcggg tatttgtagt tttagttatt 4620
taggaggttg aggtaggaga atggcgtgaa tttaggaggc ggagtttgta gtaagttaag 4680
attgcgttat tgtattttcg ggtgatagag cgagatttcg ttttaaaaaa aaaaataata 4740
ataataataa aaagattaat atttttttta tttttaaatt taatataaag ggatattgtt 4800
tagtgattaa gagaatttat ttatttaata aatatttgtt gagtatttat tatgtgttaa 4860
gtattgtttt aggtatcgga aatatagtag tgagaaaaat taaaaaaatt ttttgttttt 4920
atggggtgta tatttaagta gttgaaatag atagtgaata aataaaaaag gataataatt 4980
ttaaataata atgatgttat cggttaggtg tggtggttta tgtttataat tttagtattt 5040
tgggaagtta agttaagcgg attatttgag gttaggagtt taagaatagt ttggttagta 5100
tggtgaaatt ttatttttat taaaaatata aaaattagtt agatatggtg gtatatattt 5160
gtaattttag ttatttggga ggttgacgta ggagaattgt ttgagttcgg gaggtggagg 5220
ttgtagtgag ttaagatttg ataggttttt agtattattg tattttagat tggttgatag 5280
agcgagattt tgttaaaaaa aaaaaagtta taaatagatt ttaatagggt aatatgatag 5340
ggagggaggg ataggggagt agggtggtta aggaagggat atttaaatag gttagaatga 5400
taatggttag cgagggaaag atttagaagt gtgtgttgga agaagaaaga gtaagtataa 5460
aatttttagg ataaaattag ttcgtgtggt taaggtatag ttagaatgtg gcggggttga 5520
gggtttgggg aatagtgatt tgagtaggag taggagcgga ttatatgggg ttttataggt 5580
tttgggaagg agttggattt ttttttaaga atatttattt ttttttgtta tgaagtttat 5640
aggttaagtt tttttttgtt tttatttttt ttagtaggat tagattattt agggaatacg 5700
aggatttttg gtgggtgatt tggaaggaat aagaggaagt tttaagtaag ttgttataag 5760
gaattattat tttgtatttt tttttttaat tggaaataag taatttttag aggttttatt 5820
tatgaaaatt ttggttaaag gtgttaagaa atttttttag atttatttag atagtatatt 5880
atgagttaaa ggtatgaatt taggttgtgt ttttaagata tttatttttt agtgtattat 5940
gttatttttt aagaggtttg agaagaggtt atttaggaaa ttgtatagag attgggttta 6000
aagtggattt ta 6012

<210> 12
<211> 6012
<212> DNA
<213> Artificial Sequence

<220>
<223> chemically treated genomic DNA (Homo sapiens)

<400> 12

tgaggtttat tttggattta gtttttatgt agttttttga atagtttttt tttaagtttt 60
ttgggagata atatggtata ttagaaggtg ggtattttaa gaatataatt tgaatttata 120
tttttgattt ataatgtatt atttaggtga atttggggaa gttttttggt atttttggtt 180
agaattttta taggtgaggt ttttggaaat tatttatttt tagttaggag aagagatata 240
gagtgatgat tttttgtggt aatttatttg gaattttttt ttattttttt taagttattt 300
attagaagtt ttcgtgtttt ttgggtagtt tgatttgttt ggagggggtg ggagtaaaag 360
ggagtttggt ttatggattt tatggtagaa aaggatgaat gtttttggag aaaaatttag 420
ttttttttta gggtttgtga ggttttatgt gattcgtttt tgtttttgtt tagattatta 480
ttttttaaat ttttaatttc gttatatttt agttgtgttt tgattatacg agttgatttt 540
gttttaaggg ttttgtgttt gttttttttt tttttagtat atatttttgg attttttttt 600
cgttggttat tgttatttta gtttgtttaa atgttttttt tttgattatt ttgttttttt 660
attttttttt ttttattatg ttattttgtt aaagtttatt tatagttttt ttttttttga 720
tagagtttcg ttttgttagt tagtttagag tgtagtggtg ttgaaggttt gttagatttt 780
ggtttattgt aatttttatt tttcgggttt aagtaatttt tttgcgttag ttttttaagt 840
agttgggatt ataggtgtgt gttattatgt ttggttaatt tttgtatttt tagtagagat 900
ggggttttat tatgttggtt aggttgtttt tgaatttttg attttaggta attcgtttga 960
tttggttttt taaaatgttg ggattatagg tatgagttat tatatttggt cgatagtatt 1020
attattattt gaaattattg tttttttttg tttgtttatt gtttgtttta gttatttgaa 1080
tatatatttt atgagggtag ggagtttttt tggttttttt tattgttgta ttttcggtgt 1140
ttagaatagt gtttggtata tagtaggtgt ttaataagta tttgttgaat gaatgaattt 1200
ttttaattat taggtaatgt ttttttgtat taggtttgga agtagaagga gtgttaattt 1260

ttttgttgtt gttgttgttt ttttttttga gacggagttt cgttttgtta ttcgggagtg 1320
tagtggcgta attttggttt attgtaagtt tcgttttttg ggtttacgtt atttttttgt 1380
tttagttttt tgagtagttg ggattatagg tgttcgttat tacgtttggt taattttttg 1440
tatttttagt agagacgggg ttttattgtg ttagttagga tggttttgat tttttgattt 1500
cgtggtttgt ttgtttcggt tttttaaagt gttgggatta taggcgtgag ttattgcgtt 1560
cggttaggat tgttaatttt aagatcgatt ataattgttg agtgttattt gattttatag 1620
tttttttgtt ttagttttag aatatataag aagttttagg agggagggta gttttgagta 1680
gagttgttag gggtagtcgg gagtgtatat acggtttatg gaaatattta tttttatttt 1740
ttttttgtta aaagaggtgt aagagtggag aaggatgggt ttgggttatt aagtagggag 1800
tagtaggtga ttttagagat atttagggat aaaaatagaa tttagagttg agttgtgtgt 1860
tgaatgaagg ttgtattttt aagaggttgg gagtgatgtt gagtttaggt atcggaaagg 1920
aaggttagtg gtgtttttta aggatggggt ggttgtagtc gtggatggtt ttaaagtatg 1980
tgtttgtaga agggtgggta cgtgaaggat gaatttggtt gaatattatt agtttttttt 2040
tacgattttt attttttttt atttttaatt gtaaattttt aggaaaatta gtttaaagtg 2100
gagaagtatt gggtatgatt aatttttttg ttttatttat ttagggatat ttagttttgt 2160
tttgttttgt gagaaaaaat aagaaaaaaa aaaattatta aaaaattaaa attagtatta 2220
gggattagtt tttttttgtt tttagttttt tgttttagtt taagtatttg gaagtttttt 2280
ttgtattaat tttagatttt gtgtaaatat tagatttgag tttttggaaa ggagtatatt 2340
ttagtttaaa gtttttagta ggcgtaagta ttttttttgg gtagggattt tgggttaaga 2400
gttaaatttt ttttatttgg cgttgattgt taattttgta ggatagagag attttttttt 2460
tatgagaagg ggttgggaga tttagtgtga tgttttagta gtttttttgt gtggaaattg 2520
agtttaggtt taataagtga tttttagtaa atgtgtttgg tagttgaggg ttttgatgga 2580
aatgtttgtt tgttagaaag ggttattaga tgtgtggagt tatagttgaa tttttggata 2640
aagtaagggt taggtgtttg ggatgtggtt gtagggaaat aaggggtgat tagtttaatt 2700
tttttagttt tggggataga tgttcgggtt tttggagttt agtttttgga tttagttaaa 2760
ggtaagaaag gagatgggtt atttgtagtt taattttatt atttggattt taattgtaat 2820
ttttgatttt agggtttggg aaggatttta ttttatagaa tatatgtagt tagcggttag 2880
gcgtggtggt ttacgtttgt aattttagta ttttgggagg ttgaggcggg tagattattt 2940
gaggttagga gttcgagatt agttttaata tggagaaatt tcgtttttat taaaaatata 3000
aaaaattagt taggtatggt ggttgaggta ggagaattgt ttgaatttgg gaggcggagg 3060
ttgtagtgag ttaagattac gttattgtat tttagtttgg gtaataagag cgaaatttta 3120
ttttaaaaaa aaaaaaaata tgtagttagt tttttggatt atggtttttt ttgagagttg 3180
atggaagtta tggatatagt ttttaggaaa tagtgtttac gtttttggga ttttttgtat 3240
gatttagggg tatttataga gtagtttttt tgtgtagtaa ggattaatt ttgttcgaag 3300
aaaggttggg tttttgattt tggttgttag gaggtaattt ttaagttttt ggaatgtttt 3360
gtttaataag aatttgttta tgtgggaggt ttgggttatg ttggatggtt taggttaatg 3420
gtgtgatgta cggtggggat tttggggtac gaggtattaa tttgattttt ggagggattg 3480
gagattaagg ttagttatgt ttatatgatt atatcgtagt ataatttttg aatattaagg 3540
tttaggtgag tttttttggt tagtaagatt ttgtggatgt tgtatatatt atttttggga 3600
gaaggtaatg ttgtttgtat gattttttta gaagaggata attggaagtt tatgtttggt 3660
tttttttggt ttttgttttt tgtgtttttt tttttttgttg atttttattt gtattttttt 3720
gttgtaataa atcgtaattg tgagtgtaat agttttgttg agttttgtga gtttttttag 3780
taaattattg aattagaggg tggatttgga tattttttat tttgatttat gaggttagta 3840
tattttggtt aggatggttt agtgttggtt gagtggatag tgaataagat ttttttattt 3900
tatgggtatt ttttagtgat attgtttttt ttttttggga tagttttttt tttggatttt 3960
atatggtagg aataattgtt ttttatttag aaaagtttta agtatttttt gttttttttt 4020
tttttttttt ttttttgtta aatatttatt atatggaaga ttttgtagta ggtgttgata 4080
gggaattata gatggattag ttgtgaattt atttttaggg gtttattgtg tgaggtaggg 4140
atgtaatggg ggaataaatt ttataatatt gggaggttaa agttagagga ttgtttgagt 4200
ttaggagttt gagattagtt tggataatat agggagatta ttttcgataa tttagggaga 4260
ttatttttta ttttttaatt tttttttttg tttttataat attaattagt tatggtggta 4320
tgtaattgtg gttttagtta ttcgggaggt tgaggtagga ggatcgtatg agtttgggag 4380
gttaaggttg tagtgaattt tggttgtgtt attgatatag tttggttgtg tttttattta 4440
aattttattt ttagttgtag tttttataat ttttatatat tatgggaggg atttagtggg 4500
aggtaatcga attatgggag tgattatttt tatgttgttt ttttgatagt gatgagtttt 4560
tatgaagttt gatagtttta taaggggttt tttttttttt gtttagtatt tttttttttt 4620
gttattatgt gaagaaggat gtgtttgttt ttttttttat tatgattgta tgttttttga 4680
ggttttttta gttgtgttaa attgtgagtt atttaaattt ttttttttta taaattattt 4740
agttttgggt atgtgtttat tagtagcgtg agaatggatt aatgtaatta ttttatttta 4800
gtttgggtaa tggagaaaga ttttatttta aaaaaaattt ttataatata aggagagttt 4860
tttttaatga ggggtagaga gagcgggttt tgggagttga gaggatgtag aatttgtatt 4920
atttgatttg ggagatttgg gagagtttta aaaaagaggt aggatttgag tttagttttg 4980
atggataggt ataatatgat atgtagaggt ggggaaaggg ttttttaaga gtaagagatt 5040
gaatatataa ggtagggaga aaggagtagg tttaagtggt gtagtattaa ataaagttag 5100
aaataaagtg gggttagatt ttgaagaggt ttttaagatt gggttataga aattgatttt 5160
attttgtagg tgatgaatag gtttttaggg ttttaaggaa tgatttaggt atgtattagg 5220

gagaaattat agtttttacg atttttggtg gtgggttttt tttttatgaa gtttttattt 5280
atgtttttat tatgatttag tttgtcgttt tcgattatag taggagtacg tgttgtttgt 5340
agtatttgtt tgtttttgta aggagattga ttgggaggag tatgtattgg gtttatttta 5400
tttttgggtt tataatttat ttgtgagtat ttattataat tacgtttttt aaaaattgta 5460
tatatttagg tttgagttta tgttagtatg gtgtttagta gtattttgaa tataagtttt 5520
atttcgttta atgtttataa tagttttggg aggggtatat tgtgattagt tttattttag 5580
aattaagggg atagaggttt agagggggta aataatttat tttatttttt ttagttagtg 5640
agtggtagag ttagaattta aattcggatt ggtttgattt taaaagttgg gtttttaatt 5700
agttttttgt tttttatgtg tgtgttagga atgatgttag gggttgaaga tagtattttt 5760
tggaggtttg ttttagtttt taaagggttt ttgataattg tgtttttaga ttaatttttt 5820
ttaattaaaa aaatgttttt tagagatagg gttttgttgt gttatttagg ttggagtgta 5880
gtggtgagat tatagtttat tgtagttttt aatttttggg tttaagtgat ttttttgttt 5940
tagttttttg agtagttgga attataggtg tgagttatta tattaagtta tttttttttt 6000
tttttttgta ga 6012

<210> 13
<211> 10710
<212> DNA
<213> Artificial Sequence

<220>
<223> chemically treated genomic DNA (Homo sapiens)

<400> 13

aatagaaggg tttgttttat attaagaatt agtttattaa aaatttttat ataagattcg 60
gcgtggtggt ttatatttgt aattttagta ttttgtaagg ttgaggtagg tggattattt 120
gaagttagga gtttgtgatt agtttggtta atatggtgaa attttgtttt tattaaaaat 180
aaaaaattag tagggtgtgg tagtgtatgt ttgtaatttt agttattcgg gaggttgagg 240
tagaagaatt gtttaaattt acgaggtaga ggttatagtg atttaaggtt acgttattgt 300
attttagttt gggcgataga gatttcgttt taaaaaaaaa aaaaaaaatt tttttataaa 360
atgatttttt tatggtttat gttagtattt gttatagaag aatttattta tttttatata 420
aaggtaatag gttaaattga ggtttattag agaagagata tattttcgtt tttttggttt 480
ttttttttta ttataattta attttagtaa gttgggtaga gagaatgagt agaaaattat 540
aggtttagtg gtggatttag gtttaagaaa tatgtgtata attattatta tatttatttt 600
tataaagttg gagtatagat ttattttttt ttatttttat ataagaattt gtggtataga 660
gaggttaagt gtttttttta atgaaataga ttaaattaat gaaagagaaa aatattttta 720
taaattttat tttaaagtag atttagtttt gggttgttag aattatagtt aaggattttg 780
ggtgtttata atataaagat tttttttttt ttttttttat tattttgttt tttttttttt 840
tttttttttt tgtttttttt tttttttgtt tgagattatg agtgaggagg aagtattaga 900
tttataggtt tatttattat tttttagttt gtttttttag gaagaatgtt attaatgaga 960
aataattttt tgggttttta aatttagaat ttttatatga attaatttag gattttagta 1020
tttaaatagg ataaaggtaa tattaattgt tttttttttg aaggaaaaaa agtaattgag 1080
ttttttttgt tggaaggaaa ttttgtgtat gttaagtaaa ttttagattt gaggttattt 1140
tttttttatc gtttttattt tttttggaaa tttattgggg gaaaaagaag tatatgtttt 1200
ttttgtggtg taggttgagg gaagtagaga atgaaacgga aagtggtgtt ggggttgatg 1260
tttttttaaa tgaagaattg attttatagt tgaggtttag agttaggaat tttttttata 1320
agattagatt ttattttaat ttatagttaa atgaggatga ttttttttaa cgtggatgag 1380
atatttttat tttagtaaag tgtaagtatt gtgaaggtag ggattatatg tcgtgtgtgt 1440
tattatattt tggtattagt aaatggggat tatatggtag gtttttttgt taaatgaatg 1500
aattaaatat ttagttggtg taaaatagtt agtaagtatt atttttattt aaaacgtagt 1560
tttttttagt agatggagag gggttttgtt tagttttttt attatagggt ttggaattat 1620
ttggatttcg ttatttattt atttttattt ttatggataa tttttagtg ataaatgttg 1680
ttataattta gttggttatt aaatagtata aatggtgaat ttataaattt ttttaaaatt 1740
ttgttttata tttggttgta ttggttataa attttttaag tgttaagtat tttgttttat 1800
ttggttttag tttagtagaa attatttttt atttattgta ttagtggtcg tagttaggta 1860
tacgagtttt aatagatgtt atttgttgat tatgaaggta gtagtgtaat gtttttaggt 1920
tattttggtt ttagtttttt tttttggaag gagttttagt ttttttattg ggaaaatgag 1980
ggtgtatagt gaatgatttt agtttcgtta attgaagaag gattgaggag tcgtgtggag 2040
tgggggatcg cggatttgag tgtaaatttt ggttttggta ttaattggtt gtgtttattt 2100
gaataaatgg tttttttttg gttttaggtt ttttttttag aaaatagggt atttatttta 2160
tagagttgtg aagattaaat aaattaattt tagataggta tggtggtata tgtttgtaat 2220
tttagttatt cgggaggttg aggtaggaga attatttgaa tttgggaggt ggaggttgta 2280
gtgagttgag attatggtta ttgtgtttta ttttgggtaa ttgagtgaga ttttatttaa 2340
aaaaaataaa ataaaataaa aattaataaa ttaatgtacg tagaatatat gagttagtat 2400
ttagtatatt gtaggtatac gtaagtgtta gttgttgttt ttatttataa tatgagggtt 2460

tttgtgatat tttatgattt tttagttttt ttaagaagga aaatttatta attttgagat 2520
tgttgttttt gaggttttta tgtatttgtt ttattaaatt tttttcggat tatatataga 2580
tagataaaag tttatataaa cggtttaaaa gataagtgga aaagtttttt tttcgtgtgt 2640
tgatttgtgt ggttttggaa gaaaattttt ttaggaaggt taattttatt tatttataaa 2700
atttttatgt aatttagggt tttttttttt tttagtttaa ttggtaatat tattttagta 2760
tggaaattgg ttttgtttag agagtgggag aatgttaaga gaaatttttg ggtttgaaag 2820
tggatgtagg tttttgtgtt tttttgttta gttttagttt taggtttatt tattttgaag 2880
ttaggagttt tttatttatt tagtgatgga atttattgga gatggtaatt attattatta 2940
ttagtattta ttaaattaat atagtgtatt aaaatacgaa tgttttgtaa atattttatt 3000
tttatgtgtg gtttattttt ttgatgagga aattgaggtt tagtaaggtt gttatgggag 3060
gtagaagatt tagtttttga tttaggttgt tttgtttaaa ttgttggtga tatttttttg 3120
ggtgatattt ttatttttta tagtaaattt tttgataatt gttgaataga ataggtattt 3180
taaattgatg tttttttaga aagttaataa tatttaattt cgtaggtatg gggaagagag 3240
aaaagaaaat aatatttaat taataattat tgaaaaattt tattaatggg taaaggtgat 3300
cggtttaaat tggttgttta gagttggatt tattagattt tggagaaagg atttttttgg 3360
tgaggttaga agggaagttt aattttaagt cgagtttttt ttttgattag aaatattagt 3420
tttaggtgtt tttatttttt tggttagttt gtatttcgtg attaggggtt ttaatggtta 3480
taatgttttt aaggtggtgt tgaaattaat aagtttttag agaagttatg gtatgtttaa 3540
taacggttta atgatttttt gtgagatgta aggtttttgg tttttgtttg gttttgattt 3600
atagtttaat tttggttgag gagttaagaa atttttttta agtttttagc ggaagaggga 3660
ggtgtttata ttaggttttt tggatttttt taaaaattga gaatttaaat gaaatataaa 3720
tgatgtttga ttttttattt tttttgttag ttttcggggg tattgataat aaaaatagtt 3780
taacgtagtt ttacgggttt tttaatttat gggaagtgag tagggttttc gggtttttgg 3840
gtattttttt ttagatttta ggtagggtgg tggttatttt ttttttttga aattttaagt 3900
tattgtttta attttgtttt tttttggttg gggagattta gtttttagaa gatgtagttt 3960
tatttttatt ttttttgaat cgttaggttt gggggagtat agttttgagg ttcgagttgt 4020
agatagtttt tttagtgggt ttttagatat ttgggttatt tgtttattga aacgtattta 4080
ttttaagttg ttgttagagg aaatattagt ttttttgttt attttttatt tgttttaaag 4140
gttgtttttg taggatattg tttagttttg agttgtttat gggagttttt ttgtttgttt 4200
tggttttgtt tttatttttg aaatatattt taaatatata gaaagattta gggttggttt 4260
ggttcgattt ttatagttag atgaggatat taatgattgg cgtaaggaat tatagttaat 4320
taatgatagt gtttaggttt aagtttttta gtattttatt tttagtttag atttgttttt 4380
atataatatt tttaatagtt ttttttattg tgagaaataa gagattttta agggaggtgt 4440
tttttgttga ggtaatattt tgtttagttt ttagcggaga tttaagtaaa attgtagttt 4500
aaggtttgtt tgtttgttcg tttaggtttt tattttaggt tgatgtttag agtttaggtt 4560
taaaagtgtt tgggggggat gtttatacgg ttttattttt ttaggggtaa gtgtttttag 4620
gttggatatt aggattacgg gtgttgtttt gagtaagaga tttatttgga tgggtagata 4680
aagggatttt gttttagaga ggaagttgtt ttattttttt ttgagttgtt tgggattggg 4740
ttttgggtga atggggggag gggtttttcg ggtttcgagg ggttaattcg tacggggtta 4800
tacgtgacgt ggatggtttt agtattaagt tagaggcgcg gttttttttt ttgtttttcg 4860
tttttcgttt ttcgcggcgc gcgtcgtttt cgggggtttc gcgttttcgc gtttaggttt 4920
tttttttttg gcgggcgttt ataggcggcg cgggtagcgc gagtttcgga gtttcggagg 4980
tttgtgcgtt ttgagtcggt atggatttcg agtattgcgc gttttttcgc gtggggtttg 5040
tattcggttt ttatgtggtt tcgggggacg agttttcggg ttcgtaggga attttcgtcg 5100
ttgcgtttta tttgtatttc gcgtcgtttc gcggttcgcg gttgattcgt ttttcggttt 5160
gcgggttttt ggagttttat tttttagagt cggttaagtc gttcgttaag tatttgtagg 5220
atttcgggtt cggttcggtt tttaacggcg gttattttta cgagggtttc gcggaaggta 5280
acgcgcggcg cggggcgtag cgcgtagttc gatttttttt tttttttttt tattcgggtt 5340
tattcgggtt tttttgttta tttttttcgt tcgtttattt tttaattttt tgtttttttc 5400
gtttttcgtt acggttttta cggaagtcgg gttggcgttg gttcgggtta ggtaaaggtt 5460
agtgtggggc gggagggtga attatcgttt ttcggcggcg ggaatggaag gcgggaggtc 5520
gagggaaggt gtagtggcga cggttcggag tttagtcggg gttcgaaagt agttggattt 5580
gggaaggaga gagtatgttt tgtcgaagtt ggattggaat tgttatttat tttgaagttt 5640
agattcggtc gagggttgtg gagttatatt gagatttgac gaagaagcgc ggtttttgtg 5700
tttggcgttt tttgagggtt ttgaggttgg gttttaattt agagtggtta aatcggggtc 5760
ggttggggat tgcggtttgg ttttggttcg tttttttttt tcgggtttag tttttatagg 5820
aagggtgttg tttgtgcggt agattggtat agtttggtcg cgattggtcg tgtaatgtta 5880
gggttgtttt taagacggaa taattgtatt tttatttttt atattaagtt ggtttttttg 5940
tcgttatgta ttatgatagt cgtttttttg gtagttttgg ttttttcgcg ttggattatt 6000
atcgttgggt agttaggagt agttttgggt agtttaattt tttggcgtcg cgggtttacg 6060
ggtcggtgtt ttgtgttaag gtcggttgga tcgcgtcggt attcgagaat gcgtttgaag 6120
gtaggggttt ggtaggttga tcggtagttt tgcggtcgtt cgtcgtattt tatgatttgt 6180
aaggcgtttc ggttttcgta ttgtatttat tttatttgcg ttgttatatt tcgtagttta 6240
ggtttagaat tggatttttt aagattcgtt ttaatttttt gtcgtcgttc ggttggcgtt 6300
tcgcgggttt ttacggagtg gtcgggtatt ttcggttttc gtttagtcgg ggcggtggga 6360
atgtgtaggg gagggcgggg aggtgtgtgt gggttaacgg aggaaatgaa cgagttcgcg 6420

gagtttcgtg tttgtgtttt ttgaaagagg tttttttgat aaaaattata gttgggattt 6480
aatgagaagt agttggtttg gtttttttga ttttagttag acggtagagg tcgcgtagtg 6540
cggtgagtat cgttagagat aggtattcgg tttggtttag cgtttcgcgt cgggtaggag 6600
ttattttttt aggttcgcgt tttgttaggg cgcgtcgtat tcggtatcga tagttcgggt 6660
tttcggatta tatcggtgtc gcgcgaattg tagagagcgt tggtgcggaa cgagcggtcg 6720
ggagaaattt agtatttcgg gattcgacgg taagggtggt tttgataacg aaatatcgag 6780
aaagaaaatt aaagaaagcg agcgataatt aagaaaaggg gaagggaaat tgaataaaat 6840
aacggagagc gcggcgagaa cgagagggta aatggtagtc ggcgagttgg gatgaattaa 6900
aatgaataaa tgaggaacga acgaataaaa aaagtaggag taagatcgaa cgaaacggat 6960
aattagagaa gggggaaagg ataaagaggg agggagtaga ttaaataaaa gaaaacggag 7020
tatgtttaaa gcgtagaata tattaaaatg agaataaggg ggtaagaaaa ttaacgattt 7080
tagttgaacg gaggataggt taaacgaaag ggaggtaggc gggtgcgagt agagtttggt 7140
agagattttg gcggtcgtta gtttttggcg tttttgtcgg tgtttaacgg gttgttagcg 7200
tttttgcggt agagcggatt tcggcgtagc gcggcgcgcg gtggagtttg gttgtttgag 7260
gatagttagt ttttagggtc gtatagttta tttggtggga agtggcgggc gaagagggaa 7320
ttcgttttat ttttcggttt ttaaaataat cggttcggag cgtttttagt ttatacgagt 7380
gagcgcgtag ttgcgtttta tttgcggcgc gattttttac gaggttgggt agttgcgcgt 7440
tttagagttt ggcgtatttc gttttttttt cgtttgtagt tcgtcgttcg cgtagtttta 7500
ggtcgttttt tgttgagagc gtttagtttt gttttgaatt taggttgcgt gttggcgttg 7560
ttagttattt tcgcgtcgtt cgcgtttggt tagtttgttt cgagtttggt tttgacgtcg 7620
aaatacgttt tcggtaagag atttttatta gagttttgtt cgttgtcgtt gaggttttga 7680
gtcggatgat tcggtcgatc gttaaagtcg aggagagttt taagttttta gattcgggtt 7740
gaggtttttg ggttaacgtt gagaattttt aagaagggta tcgaggtttt atcggggttt 7800
tgtttacggc gtttatgttt attttggttt tgagttttcg gcgggagttg taggagtttg 7860
ggttttagcg agttttgtag gtcgtttagt gattttatag tggtcggagg aaatagtgcg 7920
gcgcgggata aggttgtagc gaaatgtgaa agttaaggtt ggtttcgcgg ttttaatgga 7980
tgttttttgt ggggatagtg gagtattcgg gtttgttgcg gcgttgtttt ttggttgtat 8040
agtggtttag aagtagtcgt tggtaggggt tttggtattt tttcgggtga ggggtgttta 8100
gggtacggta ggtttgagag ttgggttagg gatagagagt ttttttatta cgttaaaagg 8160
tttttggttt ttacgtttaa tagttgttgt tgtggttttg cggttatgta ggtagaagat 8220
attttagcgt tgttttaggt tgtttttaat atgagtattt cgttttaatt atttagtgtt 8280
cggattaggt gtagtgattt ttttgtataa tttatatata ttttagatgt gtttcgtgtt 8340
tagtagagta gtttttattt ttttataagt ttaggtttaa gttgttggtt gttatttttg 8400
gtttgttttt tttggttttt gtttttattt tgttttagtt ttattttttt tttttttgg 8460
gttttgtttt ttaggaggtt gttttgtgtt ttttgaggag tgtttttttt ttaaagtgga 8520
gggatgttat tttttttttg gtgtttttta ggtagattaa ggaggtttta gggtaatgat 8580
aatgaggaga gtagtttcgt ggttttttga gagggttgta gattgggtag gtatgggagt 8640
tgggtatagg ttagtaggtt tagttttttt tttggggaag ttgttttgag aagttttatt 8700
gtatttttat tttttgtttt tggtagtttg gtggtttaag gtcgggaacg taagttttag 8760
ggttttagat ttggtttttt tttttgggta ggttttattg agatttaaga ggtagatgag 8820
taagttttag ggttttggag ggtttttgtt aggatgttag tatttttgaa ttttagtttt 8880
gatttttagt tttgagttat ttttattttt tttttgtttg gagtttagta tagttttttt 8940
ataaatataa gatattaaga tttttttttt ttaggttgaa tatttttatg gtttgttgtg 9000
tgtatgtatg tggtgggggt ggcgggggcg gggggggggg atgttttatt ttagtgtttt 9060
tagttttttt attattttta attatttggt ttaatttttg tgagtttttt tttttaagtt 9120
tttattaaat ggaattgttt ttaaatttga aatgtgttgg tttaagaatg gaagttagag 9180
attggaatat tttttttttt gtgttttatt tgagttgtga aggagttttg gtttttgggt 9240
tgtagttgtg ggttatttgt gtataggttt gtgtataggt tgggtatggt ttaaggttag 9300
gtagttttgt tcgatttttt tagtttttta ggatatatat taggagttta ttttatggtt 9360
tgggtagttt tggaaagtaa ttgattattt tttagttgag gaggattttg ttttatttgt 9420
tttttatagt gtagataggg atttattaga tttagatttt gagaggttta tttgtttagt 9480
tttatttttt tttaattata gttgtgattt tgatattaag ttttgggaag atattgtttt 9540
ttgtgttagt tagttgggtt tttttagggt taggttttta ttggagatgg gtttagagtt 9600
aggtaagagg ttgtgtgagt tagtggggcg tttttgtggc gttttagttg ttattttagt 9660
ggattgttga ggagggattt tatggggata tcgttggggg tttttgttag agggagaggg 9720
tgtgggtaat tagagtgatt tattttttat tatgttttgt cggtcgattt attatggtga 9780
ggtttcgtta agtttttgga gtaggggtta gtatgttaga gtggggttgt tggaaattgt 9840
tttagtttgt aattaagttt ttttttgttt gttaaatata ggtaatgggg gagtagttag 9900
aaatttttga ggaaggaggg ggtggttaat gatgggaatg atggggatag gggttgtgtt 9960
ttgttgtgat attggttttt aatttttttt ttttgagtat agtttatttt agttttgttt 10020
ttttattgtt ggagtttaag tttttgttta gatattataa ttttaggagg aatggagaga 10080
agtaattttt ttggaagttt ttttgtttta ttttgttttt tttatattag agttggggaa 10140
tttgatattg atttgagatt tggggtttag agaataaagt ttagttagat tagttaaagg 10200
tggtttgagt atttacgttt atagtttgtt tggttttggg aatagaggcg tgggtataag 10260
ggtttgggaa ttaaggtttg tagagttagg aataaggaag tattttttgg taggagtagg 10320
agatgttaat tattttattt ttatttcgtt ttttgttttt atttttggtt ttgttagggt 10380

ggtggttttg ttgtggcgtt agttttttta cgtttttgtg gaacgtatta ttatagagta 10440
ttttttcgaa atgaatttta ttttggtttt agtagattcg tttagattat agagtatata 10500
gttttttagg gttttttttt tagttggagg tttagtaggt aggtttttaa atgttaggtt 10560
tttatggagg gtggttgtta ggaggaaggg aagggaaagg aagggggagg cggagattga 10620
ggcgttaatg tttttttttt tttttttatt ttaatttata aaagtttttg gtttagttgt 10680
tatttttttt tttgtgtttt ttattgtagt 10710

<210> 14
<211> 10710
<212> DNA
<213> Artificial Sequence

<220>
<223> chemically treated genomic DNA (Homo sapiens)

<400> 14

gttgtaatgg agaatataaa agggaggatg gtaattgaat taggggtttt tgtgggttgg 60
aatggaggga gggggaagaa tattgacgtt ttagttttcg tttttttttt tttttttttt 120
tttttttttt tggtagttat tttttatggg agtttggtat ttagaggttt gtttgttgag 180
tttttagttg gggagggggt tttggggaat tatgtgtttt gtggtttggg cggatttgtt 240
ggggttagag tggggtttat ttcggggaag tgttttgtgg tggtgcgttt tatagggacg 300
tgagaggatt ggcgttatag taggattatt attttggtaa ggttaagaat gggggtagag 360
ggcggggtga ggatggggtg gttgatattt tttgtttttg ttagaaagtg tttttttgtt 420
tttaattttg taggttttgg tttttaggtt tttatgttta cgtttttgtt tttagggtta 480
ggtaggttgt ggacgtgggt gtttagatta tttttggttg gtttggttga gttttatttt 540
ttgaatttta ggttttaggt taatattaaa ttttttagtt ttggtgtaag aagggtaaga 600
tgggataaag aagtttttag agggattgtt tttttttatt ttttttaaaa ttgtggtatt 660
tgggtaagga tttaagtttt agtaatagag aagtagagtt gagataggtt gtgtttaaag 720
aaggagagtt ggggattagt gttataatag ggtatagttt ttgtttttat tatttttatt 780
attgattatt tttttttttt ttaggagttt ttaattgttt ttttattgtt tgtgtttaat 840
aggtagggaa ggatttgatt atagattgga atagttttta gtagttttat tttgatatgt 900
taatttttat tttagaagtt tggcggagtt ttattatggt ggatcgatcg atagagtatg 960
gtgaaaaata ggttattttg attgtttata tttttttttt ttggtaagga tttttagcgg 1020
tgtttttatg aaattttttt ttaatagttt attgggatgg tagttgagac gttataaaga 1080
cgttttattg atttatatag ttttttgttt ggttttgggt ttatttttag tggaggtttg 1140
gttttgagga ggtttagtta gttggtataa gaggtagtgt ttttttagga tttggtgtta 1200
ggattatagt tgtggttggg ggaggatggg attggataaa tgggtttttt aggatttggg 1260
tttggtgagt ttttgtttgt attgtgaagg ataaatgggg tagggttttt tttagttgga 1320
aggtggttag ttatttttta ggattattta ggttatgaga taagtttttg gtgtgtgttt 1380
tggaggattg ggagagtcga gtagggttat ttagttttgg attatgttta gtttgtatat 1440
aggtttgtat ataggtagtt tatagttata gtttagggat tagggttttt ttataattta 1500
ggtggagtat aggggagaaa gtgttttagt ttttgatttt tatttttggg ttagtatatt 1560
ttaggtttag aggtagtttt atttgataga agtttaggaa ggaagattta tagaagttga 1620
attaaataat tgaaaatgat aggaagatta gggatattgg aatgggatat tttttttttt 1680
cgttttcgtt atttttatta tatgtatata tatagtaggt tatggaaatg tttagtttga 1740
gaagaaagag ttttggtgtt ttgtgtttgt agggaggttg tgttggattt taggtagaaa 1800
aggggtgagg gtagtttagg gttgagggtt agggttgggg tttaagagta ttgatatttt 1860
ggtagaggtt ttttaaagtt ttggggtttg tttatttgtt ttttgggttt taataggatt 1920
tgtttagaag aagggattag atttggggtt ttgagatttg cgttttcgat tttgagttat 1980
taagttgtta gggataaggg atgaaagtgt agtaggattt tttagagtag tttttttaaa 2040
gaagaggtta agtttgttga tttatgttta atttttatgt ttgtttagtt tatagttttt 2100
ttaagggatt acggaattgt tttttttatt gttattgttt tgaaattttt ttgatttatt 2160
tggagaatat tagagggagg gtggtatttt tttattttgg gagaggggta ttttttagag 2220
aatatagagt agttttttga ggagtaaggt ttagaagagg aaagggatag aattgggata 2280
aatggaaggt agaagttagg aaagataggt taggaatagt agttagtagt ttgaatttaa 2340
gtttgtaaag gagtgagggt tgttttgttg agtacggggt atatttgagg tgtgtatgga 2400
ttgtgtagag gagttattgt atttgattcg gatattaggt ggttaaggcg gggtatttat 2460
gttggagata gtttgagata acgttggggt gttttttgtt tgtataatcg tagggttata 2520
gtagtagttg ttgggcgtga aagttaggag ttttttaacg tagtaaagga gttttttgtt 2580
tttgatttag tttttaggtt tgtcgtattt tgagtatttt ttattcggag gagtgttaga 2640
gtttttatta gcggttgttt ttaagttatt gtgtagttag gaagtagcgt cgtagtaggt 2700
tcgagtgttt tattgttttt atagaaggta tttattgaga tcgcgggatt agttttggtt 2760
tttatatttc gttgtagttt tgtttcgcgt cgtattgttt ttttcggtta ttgtggggtt 2820
attaagcgat ttgtagaatt cgttgaggtt taggtttttg tagttttcgt cgaagattta 2880
aagttagagt ggatataaac gtcgtgggta ggatttcggt gaggtttcgg tgtttttttt 2940

gggggttttt agcgttggtt tagaagtttt agttcgggtt taagaatttg ggattttttt 3000
cgattttggc gatcggtcgg gttattcggt ttagggtttt agcggtagcg ggtaaaattt 3060
tagtaggagt tttttgtcga gggcgtgttt cgacgttaga gttaaattcg ggatagatta 3120
gttaagcgcg gacggcgcga gagtggttgg tagcgttagt acgtagtttg ggtttagagt 3180
aaggttgggc gtttttagta aagggcggtt tggggttgcg cgggcggcgg attgtaggcg 3240
ggagaagagc gaggtgcgtt aggttttggg gcgcgtaatt gtttagtttc gtgaaagatc 3300
gcgtcgtaga tggggcgtag ttgcgcgttt attcgtgtgg attggaaacg tttcgagtcg 3360
gttattttaa aaatcgggaa ataaggcggg tttttttttc gttcgttatt ttttattaag 3420
taggttgtgc ggttttgggg gttgattgtt tttaagtagt taggttttat cgcgcgtcgc 3480
gttgcgtcga ggttcgtttt gtcgtaggga cgttggtagt tcgttgaata tcggtaagag 3540
cgttagaggt tagcggtcgt taggattttt attaggtttt gttcgtattc gtttgttttt 3600
ttttcgtttg gtttgttttt cgtttaattg aaatcgttaa tttttttatt tttttgtttt 3660
tattttgata tattttacgt tttaaatatg tttcgttttt ttttgtttag tttgtttttt 3720
ttttttttgt tttttttttt tttttagtt attcgtttcg ttcgatttg tttttgtttt 3780
ttttattcgt tcgtttttta tttatttatt ttagtttatt ttagttcgtc gattgttatt 3840
tattttttcg ttttcgtcgc gtttttcgtt gttttgttta attttttttt tttttttttt 3900
ggttgtcgtt cgtttttttt ggtttttttt ttcggtattt cgttgttaag gttatttttg 3960
tcgtcggatt tcggggtgtt gggttttttt cggtcgttcg tttcgtatta gcgttttttg 4020
tagttcgcgc ggtatcggtg tggttcgggg gttcgagttg tcggtgtcgg atgcggcgcg 4080
tttagtaggg acgcgggttt gggggggtgg tttttgttcg acgcggagcg ttgagttagg 4140
tcgggtattt gtttttggcg gtgtttatcg tattgcgcgg tttttgtcgt ttggttggga 4200
ttagaggagt taggttaatt gttttttatt aagttttaat tgtggttttt attaggaaag 4260
ttttttttaa agggtataga tacgaagttt cgcggattcg tttatttttt tcgttgattt 4320
atatatattt tttcgttttt ttttatatat ttttatcgtt tcggttgggc gaaagtcgga 4380
gatgttcggt tatttcgtgg aggttcgcga ggcgttagtc gggcggcggt agggggttga 4440
ggcggatttt ggaggattta gttttgggtt taggttgcgg gatatggtag cgtagataag 4500
gtgggtgtag tgcggaagtc gagacgtttt ataggttata gggtgcggcg gacggtcgta 4560
gagttgtcga ttagtttgtt aggtttttgt ttttaggcgt attttcggat gtcggcgcgg 4620
tttagtcggt tttagtatag ggtatcggtt cgtgagttcg cggcgttagg gggttaggtt 4680
gtttagggtt gtttttgatt gtttagcggt gatgatttag cgcggggaag ttaagattgt 4740
tagaagggcg gttattatag tgtataacgg tagggaggtt agtttagtat gagaaataag 4800
aatatagtta tttcgttttg aggatagttt tggtattgta cgattagtcg cggttagatt 4860
gtgttagttt gtcgtatagg tagtattttt tttgtgaagg ttaggttcgg ggaggagaga 4920
cgggttaaga ttaggtcgta gtttttagtc gatttcgatt tgattatttt aggttgaggt 4980
ttagttttag ggtttttaaa gggcgttaga tataaaagtc gcgttttttc gttaggtttt 5040
agtgtggttt tatagttttc ggtcgggttt gggttttagg gtaggtggta gttttagttt 5100
aatttcggta gagtatgttt tttttttttt aggtttaatt gttttcgggt ttcgattgga 5160
tttcgggtcg tcgttattgt attttttttc gatttttcgt tttttatttt cgtcgtcgag 5220
gaacggtggt ttattttttc gttttatatt ggtttttgtt tggttcgggt tagcgttaat 5280
tcggttttcg tggaagtcgt ggcgaaaggc gagaggggta aaaagttgag aaataggcga 5340
gcgggagaga taagtaggaa ggttcgggtg ggttcgggta aggaagaaga agagagggtc 5400
gggttgcgcg ttacgtttcg cgtcgcgcgt tatttttcgc ggggttttcg tagaagtggt 5460
cgtcgttgag ggtcgggtcg ggttcgaggt tttgtaggta tttggcgggc ggtttggtcg 5520
gttttgggag gtagggtttt aggggttcgt aggtcggaaa gcgggttagt cgcgggtcgc 5580
ggggcggcgc ggggtgtagg tgaggcgtag cggcgggggt tttttgcggg ttcggaggtt 5640
cgtttttcga ggttatatag gggtcgggtg taggttttac gcggaaaggc gcgtagtgtt 5700
cggggtttat gtcggtttag ggcgtatagg ttttcggggt ttcggggttc gcgttgttcg 5760
cgtcgtttgt gagcgttcgt taagggggag ggatttgggc gcgggggcgc ggagttttcg 5820
ggagcggcgc gcgtcgcggg gggcgggggg cggggggtag gggagggggt cgcgtttttg 5880
atttaatgtt ggaattattt acgttacgtg tggtttcgtg cgggttaatt tttcggagtt 5940
cgggaatttt ttttttttat ttatttagag tttagtttta ggtagtttag gagaggatga 6000
agtagttttt tttttagggt aggatttttt tgtttattta tttaggtgag ttttttgttt 6060
agagtagtat tcgtggtttt gatatttagt ttagaggtat ttgtttttag aggggtgagg 6120
tcgtgtgggt atttttttta aatatttttg ggtttggatt ttggatatta gtttgggatg 6180
agggtttggg cgggtagata ggtaggtttt aggttgtagt tttatttagg ttttcgttag 6240
gagttggata aaatgttgtt ttagtaaaaa atattttttt tagagatttt ttgtttttta 6300
tagtgaaagg agttattggg agtgttgtgt ggaagtagat ttaagttggg aatggggtgt 6360
tggggagttt ggatttggat attattatta attagttatg gttttttgcg ttaattatta 6420
gtgtttttat ttgattgtgg aggtcgggtt aagttaattt tgggtttttt tatgtatttg 6480
aggtgtgttt tagaagtaga gatagggtta gagtagatag ggagattttt atgggtagtt 6540
taggattgaa tagtgttttg taggggtagt ttttggaata ggtggaggat gagtagaagg 6600
gttggtgttt tttttggtag tagtttggag tgaatgcgtt ttagtgggta gatgatttaa 6660
gtgtttgggg atttattgga gagattgttt gtagttcgga ttttagggtt gtgttttttt 6720
aggtttgacg atttagaagg gatgagggta gaattgtatt ttttaagagt tgagtttttt 6780
taattaaaag gagataggat tagagtagta gtttagaatt ttagaaaaga gaaataatta 6840
ttattttatt taagatttgg ggagaaatat ttaaggattc gaggattttg tttatttttt 6900

atggattgag gagttcgtgg ggttgcgtta aattgttttt attattaatg ttttcggaga 6960
ttagtaagag aggtggggag ttaaatatta tttgtatttt atttgaattt ttaattttta 7020
aaggaattta agaggtttgg tgtgaatatt tttttttttc gttgggggtt tagaaggggt 7080
tttttggttt tttagttaga gttgggttgt aaattaagat taaataggaa ttagaagttt 7140
tgtattttat aaggagttat taagtcgtta ttaaatatat tataattttt ttgaagattt 7200
attggtttta atattatttt ggaggtatta tggttattag ggtttttaat tacggaatgt 7260
agattggtta gagagatggg agtatttggg gttggtattt ttagttaaga gggaggttcg 7320
gtttgaaatt aagttttttt tttggtttta ttagagggat ttttttttta ggatttagtg 7380
gatttagttt tgggtaatta atttgaatcg attatttttg tttattaata aggtttttta 7440
atagttgttg attggatatt gttttttttt tttttttttt tatatttgcg aaattggata 7500
ttgttgattt tttaggagga tattaattta ggatatttat tttatttaat agttattaag 7560
aagtttatta tggaggatga aaatattatt taggaaaatg ttattaataa tttaaataag 7620
gtagtttgga ttaaaggttg agttttttgt tttttatgat aattttgttg agttttagtt 7680
tttttattag aaaggtgggt tatatatgga agtagggtat ttgtaaagta ttcgtgtttt 7740
gatgtattgt gttaatttaa tagatgttga tgatgatggt gattattatt tttagtgggt 7800
tttattattg aataggtgaa aggtttttga ttttagagtg aatgggttta aggttggagt 7860
tgagtagaga gatataggga tttgtattta tttttaagtt tagaaatttt ttttgatatt 7920
tttttatttt ttgaatagag ttaattttta tattgggatg gtgttattaa ttaaattgag 7980
gagagagaaa attttagatt atatgagagt tttgtgggta agtggagttg atttttttgg 8040
gagagttttt ttttagaatt atataaatta gtatacgggg aggagatttt tttatttgtt 8100
ttttaagtcg tttgtgtaaa tttttattta tttgtatgtg gttcggaaag agtttgatgg 8160
agtaggtgta tagaaatttt aaaaatagta attttagagt taataaattt ttttttttag 8220
gggggttggg agattatgga gtgttataaa ggtttttata ttataggtaa aaataatagt 8280
taatatttac gtatatttat agtgtgttgg gtgttagttt atgtatttta cgtatattgg 8340
tttattaatt tttattttat tttatttttt ttagatagag ttttatttag ttgtttaggg 8400
tggagtatag tggttatgat tttagtttat tgtaattttt attttttagg tttaagtgat 8460
ttttttgttt tagtttttcg aatagttggg attataggta tgtgttatta tgtttgttta 8520
agattggttt atttaatttt tatagttttg tgaaataggt attttgtttt ttagagaagg 8580
agtttgaggt taggagggag ttatttgttt aggtagatat agttagttag tgttagaatt 8640
agaatttata tttagattcg cggtttttta ttttatacga ttttttagtt tttttttagt 8700
taacgaagtt gaaattattt attgtgtatt tttatttttt tagtgggaag gttgggattt 8760
tttttagaaa ggaaggttga ggttagagtg gtttggggat attgtattgt tgtttttatg 8820
attagtaaat ggtatttgtt gagattcgta tgtttagttg cggttattga tatagtaggt 8880
agagggtggt ttttgttaag ttgagattaa atgaaataga gtgtttaata tttgagaggt 8940
ttatgattaa tgtagttaga tgtggggtag aattttaagg gaatttgtga atttattatt 9000
tgtgttgttt aataattaat tgaattataa tagtatttgt tattgaaaaa ttgtttatgg 9060
agtaagagat gagtggatgg cgagatttag ataattttag gttttgtaat gaaaaaattg 9120
aataaaattt ttttttattt gttaagaaga gttacgtttt aaatggaaat gatatttatt 9180
agttgtttta tattagttga atgtttaatt tatttattta ataggagagt ttattatgtg 9240
atttttattt gttggtatta ggatatagtg gtatatacga tatgtgattt ttgtttttat 9300
agtgtttata ttttgttgga atggaagtgt tttatttacg ttgaagaaaa ttatttttat 9360
ttggttgtga attagaatag aatttggttt tgtgagaaga gtttttggtt ttgggtttta 9420
attgtaaggt tagtttttta tttagggaaa tattagtttt agtattattt ttcgttttat 9480
tttttgtttt ttttagtttg tattatagga aggatatgtg tttttttttt ttttagtgga 9540
tttttagaag ggatagggac gatgagaaag aggtaatttt agatttgaga tttgtttgat 9600
atatataaaa ttttttttta atagggaaaa tttagttgtt tttttttttt taaaggaaag 9660
atagttggta ttatttttgt tttgtttaga tattgaaatt ttaaattgat ttatataaaa 9720
atttgggttt tgggaattta aaagattgtt ttttattagt gatattttttt ttggaagggt 9780
aggttgagga atggtaaata aatttgtgga tttggtgttt tttttttatt tatgatttta 9840
gatagagaga gaaaaagata gagagagaga gagagagaag aagtaaaatg ataggaaaag 9900
aaaagaaaaa atttttgtgt tgtggatatt tagaattttt ggttgtgatt ttaataattt 9960
aggttgaagt ttgttttgga ataagatttg tgggatgttt tttttttttt attgatttga 10020
tttgttttat tgggaaagat atttaatttt tttgtgttat aggtttttat gtgaaaatgg 10080
gaagaaatga gtttgtattt tagttttatg ggaatgaatg tgatagtaat tatatatatg 10140
ttttttgagt ttgaatttat tattgaattt atggtttttt gtttattttt tttgtttagt 10200
ttattgaggt tgggttgtaa tggagggagg gggttagaga ggcggagatg tatttttttt 10260
ttggtaggtt ttagtttgat ttattgtttt tgtgtgaagg tgaataggtt tttttgtggt 10320
aagtgttggt atggattata gagaagttat tttgtagagg agtttttttt tttttttttg 10380
agacggagtt tttgtcgttt aggttggagt gtagtggcgt gattttgggt tattgtaatt 10440
tttgtttcgt gggtttaagt aatttttttg ttttagtttt tcgagtagtt gggattatag 10500
gtatgtatta ttatattttg ttaatttttt atttttagta aagatagggt tttattatgt 10560
tggttaggtt ggttataaat ttttgatttt aggtgattta tttgttttag ttttgtaaag 10620
tgttgggatt ataggtatga gttattacgt cgagttttgt ataggagttt ttgatgaatt 10680
aatttttgat ataaaataga tttttttgtt 10710

<210> 15

<211> 6215
<212> DNA
<213> Artificial Sequence

<220>
<223> chemically treated genomic DNA (Homo sapiens)

<400> 15

ttttgggttt ttagtgtagt ttatttgttt tttttgtgtt tgtgtttgtt ttgtgtttat 60
tgttggcgtg gtggcgtggg tagtggacgg ttttttgttt cgggagtgat cggcggtgtt 120
gttttggttg tgtttttttt tgatttagaa ttattaggaa ggtggtgtcg gggggttttt 180
gggaattttt attgttcgaa gtttggttta tagttttagg tggtgatatt cgtgtttacg 240
aaggtttttc gttggatttt tcgtttttag aaatgtttga ttattgttag cggtagttat 300
gtttttatag aaggggggtt ttgtttttat gtttttatag aaaggggatt tcgtttttga 360
gggttttgac ggtagtaggt cgcgattttt tgtttaacgg gtttttgttt ttttttttta 420
ggattcgtat tttatgaaga attatttggg tttttatgaa tgtaaatttt gtttgatatt 480
ttataataat gaggtgcggt ttttgtttgg tttcgggcgg ttcgcggcgg gttgagtggt 540
tgacgttagg gggatttgtt tgtgtttgtt cgttcggttt cgagttatgc gtgttttttt 600
tttattttag gggagttatt tggtatatac gtaggggaag aagtattaga ttaatttgtg 660
agtattttat gtttttttga tgtttggtgt tttagtagtc gttggtttaa gtttttgtga 720
gtttcgcggg ttattgtttt atagtcgggg agttagtttg gtttgagttt ttttgggcgt 780
ttgagtacgg tttttgggga gtattagaga atagaggtat ggatggttga ggcgggtaga 840
ttatttgagg ttaggagttc gagattagtt tggttaatat tgtgaaattt tatttttatt 900
aaaaatataa aaattaggtt aggtatggtg gtatacgttt gtaattatag ttattatgga 960
ggttgaggta ggagaatttt gaatttggga ggtagagatt gtagtgaatt aaggtgttgt 1020
tattgtattt tattttgggc gatagagtga gatttttgtt ttattaaaaa aaaaaaaaaa 1080
gagtagaggt atggagttgt tggaagggtt ttagttaggg atagtgagga gtatgatagg 1140
aagagaatat gtttgtttta tttttgtttt tagttaaatt ttagggtttt ttttaggttt 1200
ttggtttttt ttatttttta gtttgtagtg agttttaagg ttagggggtt tttggtattt 1260
gggtttatgg ttttggtgtt tgtgtgtgga ggggttttag tagtattttt attttgttcg 1320
tagggttcgg cgagtagtta aggaggttaa ggaggttttt gtttagttcg cgtttgagaa 1380
ggttaaggtg gaggtgaaga agtttgtgaa gatcggtcgt tcgggttata aaggtgagtt 1440
tgttcgtagc ggggtcggtt atagtcgttg tcgtgatata agtgtgtttt ttagttttta 1500
gtgcgggagg tgtagttttg atagtttttt ttttgagtta ttgtttttcg gttttggtgg 1560
tcgtttttta tttatttgta gtttttggcg ttgtgtttgg gagttgttag gttgggttcg 1620
ataagtattt attttattta gtagtttatt ttttagttga gttatagttt gtgttttttt 1680
gttcgggttt tgtggaagtt tttgggaatt tttgttaagt aaaagagtgg aagtggaggt 1740
gtggtgagtt ttggtatatt ttttcggtta attttgaagt ttttgaagga ggatagggag 1800
aagcggttgt tagaggtagt ttttttcgga tttttagtag tgggaatggt tagaggtgat 1860
ttcggtttag aaagttgggg aagtgattag ggtggaagat agagatttgt gtttataggg 1920
tttaggaagg cgggtacgtt atttagtaag tgttttttat agaaggcggg gatagtttgt 1980
gtgtttggtt tttagataga agtttgtagg gtagaggttt cggggtaggt gtatcgggtg 2040
ggcgagggtg gttagagcgg taggtttttg gtggggaggt tttgataggt tttgggtttg 2100
ggtttaggac ggtgaggcgg tagagggttt gtgagtgtgc gtgtttgagg tcgtatggag 2160
agtggtcggg cggtaggtag tttagtagtt attttcgggg tttaggttgt tgagttttgg 2220
cgggaagggt attttttttc gtttatcgta tatatggtgt tagttttgtt taggcggttt 2280
cgttcgaatt ttagagttag ggtgtcgagg gttttttttt atttagtggt tttaattttt 2340
gttgggtggt attttagtat ttttggtgaa gggttttggg gattgggata aaggagtatt 2400
attttcggtt tttgtttgag tgatttcgta ggtagtttag ttttggcgta tttgtgtttt 2460
tatttatatt agaattttag agtttgggcg gagattgttt aatggttgtt agagttgtag 2520
ggtttttttc ggggtttcgt tttcgtttag tgggtggtat tagtttgttt taggtttttt 2580
agtttcggag aggataagta gtcgggattt gagagttttt tgtgttgtag tgattaagta 2640
gagagattcg gagatgggtt agtagagttt ttttttttag gtgagattag ggatttgggc 2700
gtggggggcg gttaaaggta tttaagaggc ggttttgtta ttcggtcgtg tttttgtaga 2760
ttgattattt tgagatcgtc gagggtatta tgttacgtta tcgttttatg tttgcgtacg 2820
agtagaggat cgagttttcg gatcggcgtt ggtagtattt gtttatggtc gtcgagtttt 2880
acgagattat tgtttttaag gtagcgtggt tgcggggttt tttgggtttt tttgagatgt 2940
gtaagttaga aggatttgta taggttgggt aggatgtttt ttttatcggg tttggggttt 3000
tttgggtttt ttttaagtcg gagttagaag ggtttgtata ggtcgggtag ggtatttttt 3060
ttattgggtt ttgtaggagg gtttgtttgg gttttattgg gttaatgaga agggaggtcg 3120
gtagggttga gcgttaggtt tgtgaggttt ttgttaagtg attttatttt ttgggttagg 3180
ttgttggagg gttttttata gtttgttttt atgtttggat agggcggtta ttaggaaata 3240
aataatataa acggtgattg tttagggagg gggttaatag cgtgttagta ttgaggttag 3300
gtttgtttta ttttagaatt gaattttgtg ttttacgaaa gttttattag tttttttttg 3360
ggtttttagg gtagagttcg ggttggggag gttagtgttt cgggtttttt gtttgatggt 3420

cgttttgagg ttatagggat taggagtttt ttttgttttt cgttttttta ggtgtcgagt 3480
agagagatcg ataaggcgga gggtaagttt tggatatatt ggaatcggga gattaagtag 3540
gtgagtggtt cgtttcgagt ttggtttttt tttatttagt tttggcggtt ttagttttgt 3600
tttagtttta ttcgtgtttg ttgaattttt tttcgttttt ttagtttttt ttttagtttt 3660
attttaagat ggagaagttt tcggttttat ttagtttttt tgttggtttt tttggggtga 3720
agcggttttt attttcgttg atgaacggtt tgttttttcg gttatcgttg tttgagtttt 3780
tgttatcgtt ttcgttagga ggtttgtttt tgttatttat gttttttata gggtttgcgt 3840
ttttagggtt ttcgggatta ttttagttat tttcgttagt tttaggggtt tatttttcgg 3900
ttttagtggt gtattttttt gtatttgggg tttatttttt agtttttggc gtttattttt 3960
tagtttttgg cgtttatttt ttagtttttg gggtttattt attaattttt ggggtttatt 4020
ttttagtttt tggagtttat tttttagttt tcggggttta tttattagtt ttcggagttt 4080
atttattagt ttttggggtt tatttattag ttttaggggt ttattttttt ttattagcgg 4140
gggtttattt ttaggtttcg ggggtgtatt tagtagtttt cgtcgtttat ttttaggttt 4200
taggggtgta tttattagtt ttagggatgt atttttaggt ttcgggggtt tatttttaat 4260
ttttcggggt ttattcgtcg gtttttgggg tttattttta gttttcggga gtttattttt 4320
taaattttgg ggtgtatttt ttaattttta tgtttttaat gttgaggttt ttattttttt 4380
tcgaaggttt agggaatata tttttttttt ttttaattaa ttgagaagtt gttttttttt 4440
ttagtaagtt tagcgttagg tgtttttgtt tttttttatt gagagaaggt tgtttttttg 4500
tattgttttt cgtttattaa atagtttttt ttagttttga gtgtattgat gttcgtagcg 4560
tcgttttatt gtgttagtgt gtgtgggagt gttaggtata gtattatttt ttagtttggg 4620
tcgattgggg agggtttggg gttcgttagg agatatttgt gggaggtttg agagatggtt 4680
gtattttgga gatggtttgg tggaggatag attttattag ttagttagga ggggatttgg 4740
ggttttgttt tggggaggga agagtagatt ttacgatatt tttggggttt ttagatagtt 4800
tattaggggt ggggagggtg agtagggata gggcgttttt attgatttgg gatttttttt 4860
ttttaggttt atattttagt atttaggata tttgggtttt tcgtttttag cgttgtttag 4920
tttggttgtt tggtcgttat ttttagtttg gtttttattt ttgtgttttt tggggatggt 4980
ttttaaggac ggtatgttga tatattaggt ttagttttat tattggggag ggagataggt 5040
tgttagggat agaaagggtt ttttgagaag gttattttgt ttggagtggg ggcgtcgggt 5100
attgtttttt aaggtcgcgg tagaggagat aggggtttgt tttgtataaa tattttattt 5160
tttattcggt ttatttaagg taggtagttt agtttttggt agtatttacg atgcgggatt 5220
tgttttttat tagtttggtt ttagtgttgt ttgttttggg ggttttgttg gggattgagg 5280
tttttagagt agaggagtta gttgtgggta ttagtggttt tatttttcga gaagatttgg 5340
attggttttt aggtagttta taagagtttt tgtgtttggt ggtattgggc ggggatagta 5400
atggtagtag tttttttttg cgggtggtgg gggttttaag cgtttatgag taggtttttt 5460
tgggggtcgt gtagagggtt cgttggggtt ttcgagattt ggttattttc ggggtttgta 5520
atatcggtga taggtaggtt gttttgtttt ttttacggcg gttgggggtt tggttgcggg 5580
attttggggg gtagcgtttg gtggttttat atttggagga aggtatgtgg ggtttagttt 5640
taagtttggt atcgtcgttt ttttttaggt gggtcgggtt tttttaggga agattagggg 5700
ttggtagagt ttttattttg ggtagggagg ttgtggtttt gtttttagga ttgggttgcg 5760
ggttcgtggt ttggaaggtg ggtattatat tttgttttgt tttcgaagtt tagtttttag 5820
atttgttttt gtttcggtgt tagggagaga gttgttgttt tttttttatt tttgaagacg 5880
acgtagggtt cggggttagt ggaatttttt tttttatagt tttagtttgt ttttagggtc 5940
gttggtttaa gatatttttt gcggggaagg ggttttatcg ggtattttaa tttagagatt 6000
ttagggcggt agttttattt atagttttag acgtagtttt tgtttgtttt tgtcgttatc 6060
gtttttggt tgtaggaagg tagttaagag ggtatttttt gtttttcgtt tgaggttttt 6120
tgtatagtgg ttagagcggt agggatagat tttaaagatt ttcggggggt gtggttttta 6180
atggtttagg cgttttttgt tgtttcggtt gtagt 6215

<210> 16
<211> 6215
<212> DNA
<213> Artificial Sequence

<220>
<223> chemically treated genomic DNA (Homo sapiens)

<400> 16

attgtagtcg ggatagtagg ggacgtttga gttattgaag gttatatttt tcgggaattt 60
ttgggatttg tttttgtcgt tttggttatt gtgtagggga ttttaagcgg gggataaggg 120
tgtttttttt agttgttttt ttgtagttag ggagcggtga cggtaggggt aggtaggggt 180
tgcgtttgag gttgtgggtg gggttgtcgt tttggggttt ttgggttggg gtgttcgatg 240
aagttttttt ttcgtaggga gtattttagg ttagcggttt tgagaatagg ttggggttgt 300
gggaagaagg gttttattgg tttcgagttt tgcgtcgttt ttaggggtgg ggagaaggta 360
gtagtttttt ttttggtatc gaggtagggg taagtttaag agttgggttt cggggatagg 420
atagagtgtg gtgtttattt tttaggttac ggattcgtaa tttagtttta ggaataagat 480

tatagttttt ttgtttaggg tgggggtttt gttagttttt gatttttttt aggaggattc 540
ggtttatttg aaggaagacg gcggtgttaa gtttggggtt gggttttata tatttttttt 600
taggtgtagg attattaggc gttgtttttt agggtttcgt agttaggttt ttagtcgtcg 660
tagagagggt aaggtagttt gtttgttatc ggtgttgtag atttcgaagg tggttaggtt 720
tcgggggttt tagcgggttt tttgtacggt tttaggaaag gtttgtttat aggcgtttag 780
agtttttatt attcgtaggg gggagttgtt gttattgttg ttttcgttta gtgttattag 840
gtatagaggt ttttgtgggt tgtttggagg ttagtttaag ttttttcgga agatgaggtt 900
attggtgttt atagttggtt tttttgtttt gagggtttta gtttttagta gagtttttag 960
ggtagatagt attagggtta ggttggtgag aggtaggttt cgtatcgtgg gtgttgttag 1020
gggttgggtt gtttgtttta agtgagtcga gtggaaggtg gggtgtttgt gtaggataga 1080
tttttatttt ttttgtcgcg attttggggg atagtgttcg gcgtttttat tttaggtaga 1140
gtggtttttt taaagagttt tttttgtttt tggtagttta tttttttttt tagtgataga 1200
gttgggtttg atgtgttaat atgtcgtttt tgagggttat ttttagaaga tataggagtg 1260
ggaattaggt tgggagtgac ggttaggtaa ttaaattaga tagcgttggg ggcggggggt 1320
ttagatgttt tgggtgttga ggtgtgggtt tggaggagga gggttttaag ttagtggggg 1380
cgttttgttt ttgtttattt tttttatttt tggtgggtta tttggagatt ttaaggatat 1440
cgtggagttt gttttttttt ttttagaata ggattttaga ttttttttta gttggttggt 1500
ggggtttgtt ttttattagg ttatttttaa ggtatagtta ttttttaggt tttttatagg 1560
tgttttttgg cgggttttag gtttttttta gtcggtttaa attgggggat ggtgttgtgt 1620
ttggtatttt tatatatatt gatatagtag ggcggcgttg cggatattag tgtatttagg 1680
gttgggggag gttgtttaat gagcgggggg tagtataaaa gagtagtttt tttttagtgg 1740
gaaaaggtaa gagtatttgg cgttgggttt gttgggggag ggagtagttt tttagttggt 1800
tgggggaggg ggaggtatgt tttttgggtt ttcggaggga agtgggggtt ttagtattgg 1860
gggtatggga gttggggggt gtattttagg atttgagggg tgaattttcg gaggttgagg 1920
gtggatttta ggagtcgacg gatggatttc gggaggttgg gggtggattt tcggggtttg 1980
agggtgtatt tttgggggttg gtgggtgtat tttggggtt tgagggtgaa cggcgggggt 2040
tgttgggtgt attttcgggg tttgggggtg aattttcgtt gatgggggag gatggatttt 2100
tggggttggt gggtgaattt taggggttgg tgggtggatt tcgggggttg gtgggtgaat 2160
ttcgggggtt ggagggtgga ttttaggagt tggggggtgg attttagagg ttggtgggtg 2220
gatttagggg gttgggggat ggacgttagg agttgggggg tggacgttag gagttggggg 2280
atggatttta gatgtagggg gatgtattat tgggtcgggg gggtggattt ttggagttgg 2340
cgggggtagt tggggtggtt tcgggggttt tgagggcgta ggttttgtgg ggggtatggg 2400
tggtagaggt aggttttttg gcgggggcgg tggtaaagat ttaggtagcg gtggtcgagg 2460
gggtagatcg tttattagcg gggtggaggg tcgttttatt ttaggggggt tagtagggag 2520
gttgggtgga gtcgggggtt tttttatttt aaagtggaat tggaggaaga attagagaga 2580
cggagaaagg tttagtaggt acgggtgggg ttagggtagg gttagggtcg ttagggttgg 2640
gtgggaagga gttaggttcg ggcgagttta tttatttgtt tggtttttcg gtttagtgt 2700
gtttagaatt tgtttttcgt tttgtcgatt tttttgttcg gtatttggga gggcggggga 2760
tagagagggt ttttggtttt tgtgatttta gggcgattat taggtaggga gttcgaggta 2820
ttagtttttt tagttcggat tttatttggg gagtttagga gaaggttggt aagattttcg 2880
tggggtatag ggtttagttt tggagtgaaa tagatttggt tttaatgtta gtacgttgtt 2940
ggtttttttt ttgggtagtt atcgtttgta ttatttgttt tttgatggtc gttttgttta 3000
ggtatgggga taggttgtag gaagtttttt agtagtttgg tttaggaagt gaggttattt 3060
ggtaaaggtt ttataggttt ggcgtttagt tttgtcggtt ttttttttta ttggtttagt 3120
ggagtttaag taggtttttt tgtagagttt aatgggagag gtgttttgtt cggtttgtgt 3180
agattttttt ggtttcgatt tggggggggt ttaggggatt ttaagttcga tgggaggggt 3240
attttgttta gtttatgtag atttttttgg tttgtatatt ttaagggggt ttagggaatt 3300
tcgtagttac gttattttga aggtaatggt ttcgtagggt tcggcggtta tgagtaggta 3360
ttgttagcgt cggttcggag gttcgatttt ttgttcgtac gtagatatga agcggtgacg 3420
tggtatgatg ttttcggcga ttttagggta gttaatttgt agggatacgg tcgggtggta 3480
gagtcgtttt ttgggtgttt ttggtcgttt tttacgttta agtttttgat tttatttgga 3540
agaggaggtt ttgttggttt attttcgagt ttttttgttt ggttattgta atatagaaag 3600
tttttaggtt tcggttgttt gtttttttcg aagttgagga atttggggta ggttaatgtt 3660
atttattgaa cgagagcggg atttcggggg aggttttgta gttttggtaa ttattagata 3720
attttcgttt aggttttggg attttggtat aggtgaaggt ataggtgcgt tagagttgag 3780
ttatttgcgg agttatttag gtagggatcg aggatggtgt ttttttgttt taatttttag 3840
agttttttat taggggtgtt gggatattat ttagtaggag ttggggttat tgggtaggga 3900
ggggttttcg gtattttggt tttgggattc gagcggggtc gtttgaatag ggttgatatt 3960
atgtatacgg tgagcgagag aggatgtttt tttcgttaga gtttagtagt ttggatttcg 4020
agaatggttg ttgggttgtt tgtcgttcgg ttatttttta tgcggtttta gatacgtata 4080
tttataagtt ttttgtcgtt ttatcgtttt gagtttaagt ttaggatttg ttagggtttt 4140
tttattagga gtttgtcgtt ttagttattt tcgtttattc ggtgtatttg tttcgggatt 4200
tttgttttgt aggtttttgt ttggaggtta ggtatatagg ttgttttcgt tttttgtaaa 4260
gaatatttgt tgggtagcgt gttcgttttt ttgaatttta tggatataag tttttatttt 4320
ttattttggt tattttttta gttttttgaa tcggggttat ttttagttat ttttattgtt 4380
gagggttcgg gaggagttgt ttttaatagt cgtttttttt tgtttttttt taaaggtttt 4440

aaggttaatc ggagggatgt gttagggttt attatatttt tatttttatt tttttgtttg 4500
gtaagaattt ttagaagttt ttataagatt cggataggag ggtatagatt gtggtttagt 4560
tgggaaatgg gttgttgggt gagatgggtg tttatcgggt ttagtttgat agtttttaga 4620
tataacgtta gaggttgtag gtgggtgagg gacggttatt aaggtcggaa aatagtggtt 4680
tagggaggag gttattaggg ttgtattttt cgtattggag attaaagaat atatttatgt 4740
tacggtagcg gttgtggtcg gtttcgttgc gggtagattt atttttgtag ttcgggcggt 4800
cgatttttat aaattttttt atttttattt tgattttttt aggcgcgggt tgggtagggg 4860
tttttttggt ttttttggtt gttcgtcggg ttttgcggat aggatggagg tgttgttggg 4920
atttttttat atataggtat taaagttatg ggtttaggtg ttaggagttt tttgattttg 4980
gagtttatta taagttgaga gatgaggagg gttaggggtt tgggaggggt tttggggttt 5040
ggttgaggat agggatagga taggtatgtt ttttttttgt tatgtttttt attgtttttg 5100
gttggggttt ttttaataat tttatgtttt tgtttttttt tttttttttt aataagataa 5160
gagttttatt ttgtcgttta ggatggagta tagtggtagt attttggttt attgtaattt 5220
ttgtttttta gatttaagat ttttttgttt tagtttttat agtagttgtg attatagacg 5280
tgtgttatta tgtttggttt aatttttgta tttttagtag agatggagtt ttataatgtt 5340
ggttaggttg gtttcgaatt tttgatttta agtgatttgt tcgttttagt tatttatgtt 5400
tttgtttttt aatgtttttt aggaatcgta tttaagcgtt tagggaggtt taggttaggt 5460
tggtttttcg gttgtggagt agtggttcgc gaggtttata gagatttgag ttaacggttg 5520
ttggagtatt aggtattaaa gggatatgag gtatttatag gttggtttgg tgtttttttt 5580
tttgcgtatg tgttaggtag tttttttgga gtggaaagga aatacgtatg attcgaggtc 5640
gggcggatag atataggtag gtttttttga cgttagttat ttagttcgtc gcgagtcgtt 5700
cggagttagg tagaggtcgt attttattgt tgtgaagtgt taggtagagt ttgtatttat 5760
aggagtttag gtggtttttt atgaagtacg ggttttggag gggaagaata ggggttcgtt 5820
agatagaaga tcgcggtttg ttgtcgttaa ggtttttaga ggcgggattt tttttttgta 5880
gaggtatgga ggtagaattt tttttttgta gaggtatggt tatcgttgat agtggttaag 5940
tatttttgaa aacgggggat ttagcgggag gttttcgtga gtacgggtgt tattatttgg 6000
ggttgtgggt taggtttcga gtagtggggg tttttaggga gttttcgata ttattttttt 6060
ggtggttttg agttagaggg aggtatagtt aggatagtat cgtcggttat tttcgaggta 6120
gggagtcgtt tattgtttac gttattacgt tagtagtagg tataaggtag atataggtat 6180
aagaaaagta aatgggttat attgaagatt taaag 6215

<210> 17
<211> 5432
<212> DNA
<213> Artificial Sequence

<220>
<223> chemically treated genomic DNA (Homo sapiens)

<400> 17
   cgtgtgtggt atatttttat attttttagt tttttttata gttatttttt tttgtgtttt 60
   tttagtattt gtgtttttgt ttcgtgtttt tttttttttt tatttttttt ttttttaaat 120
   tttaatataa gattttaata taagattgtg tttatgttga tgatttttaa attttgagtt 180
   ttaatttgtt ttttgatttg gaagtttgtt tgttagatat ttttatttgg atgtttttta 240
   gataaattta atatagtaga aggagaaata attttttgtg ttttatattt tttttaataa 300
   tattatttat tgttttttga ttatttatat ttgaaatttt agaattattt tttagttttt 360
   ttttttattt attaatatta atttgattgt taattttgtt aatattattt atataatata 420
   tattaaattt tttttttttt ttttttttga gatggagttt cgttttgtcg ttaggttgga 480
   gtgtagtggc gtgatttcgg tttattgtaa ttttcgtttt ttaggtttaa gtaatttttt 540
   tgcgttagtt ttttgagtgg ttgggattat aggcgcgcgt tattacgttt agttaatttt 600
   tgtattttta gtagagacgg gtttttatta tgttggttag ggtggttttt attttttgat 660
   ttcgtgattt gttcgtttcg gttttttaaa gtgttgggat tataggcgtg agttattacg 720
   ttcggttttt ttttttattt ttatttttat tgttttagta tttattttgt ttattgttta 780
   ggttttgtaa ttgatttttt taaattttag tagttattta ttttttaatt gttattaggt 840
   ttttgtttaa aattttttgt ttttagggaa agtttaagtt ttttgatata gaattgaaaa 900
   tttgtttaat ttggttcgtt tttttttaag gtgatttttt gttttatatt ttatattgtt 960
   ttttttttat tttttttatt tttaatatat aagtttttgt gtttatattt ttatgttttt 1020
   atttttgtta ttttttttgt ttagaaggat tgtttttttt ttattattgt gtttattttt 1080
   gggattattt aaatattttt ttttttgtga aattttttgg gtattttttt tttatgtttt 1140
   aggtagaatt agtttatttg atttatttgt atagtttttt ttttatgttt ttagtaggaa 1200
   atatagtttt tcggttgttt gttaatattt atttgtattt ttatattatg agtttttaga 1260
   gggtagtaat tatattttta tttttgtagg tttagtattt aatgaaatat attttttttg 1320
   gtttcgcgta gtggtttacg tttgtaattt tagtattttg gaaggtttag gcggtggatt 1380
   atttgagatt aggagtttga aattagtttg gttaatatag tgaaatttta tttttattaa 1440
   aaatataaaa aattagttag gtatggtgat aggtatttgt aattttagtt atttgggagg 1500
   tcgaggtagg agaattgttt gaatttagga ggcggaggtt gtagtgagcg gagattgtat 1560
   tattttattt tagtttgaat gatagagtga gatttcgttt taaaaaaaaa ggaaattttt 1620
   ttttaaaaat aatataggtg ggggtttcgt tatgttattt agggggattt ttggtttttt 1680
   aaagtattgg gattatagat atgaattata gcgtttagtt tttattatta ttttaatttt 1740
   aaaatttaat agtttagagt aggtatagtt tttataaatt tgattggaat gtttttattt 1800
   tggaaagaat tagttattcg aagttttggg tgaggagatt gtttttttgt ttgattttta 1860
   cgttgttttt attttggagg agatagtttt ttgagatatt ttagataaaa gaagttattt 1920
   tatttttaaa ttttttttta tattttatta tatatattat tttagattta tttgatttgg 1980
   ggttagtgaa ggaaagtagt tggaagtata atattttatt ttattggtaa ggttttgtag 2040
   atttttggaa gggtttaaag aaggatgggc ggttatttcg agttttggtt atttttttcg 2100
   ttgttgtttt ataattattg gaatttttat tatttttttt ttttatgtag gttttttaga 2160
   agaaagattt tagaattaaa aagttagtat ttaagttggg cgtagtggtt ttcgtttgta 2220
   atttcggtat tttgggaggt cgaggggggc ggattatttg aggttaggag tttgagatta 2280
   gtttggttaa tatggcgaaa tttcgttttt attaaaaata taaaaattag tcgggcgtgg 2340
   tggtatgcgt ttgtagtttt agttatttag gaagttgagg tcggagaatc gtttgaattc 2400
   ggtaggtaga ggttgtagtg agtcgagatc gtattattgt attttagttt gggtgataga 2460
   gcgagatttc gttttaaaaa aaaaaaaaaa aatgtcggta tttagattgt tttttttggt 2520
   ttaagttaga ttattttaag aaagggtttt taggaagtta ttagggggat agaaagttag 2580
   ggaaatagag aataagtttt ttttgatttt taatttttaa ggagttagtg gtttataggt 2640
   gtggaagtga ttatttattt ttgttgtttt ttattttggg aaagagtttg gtggagttgg 2700
   atttttttgt ttttattgta gaataagtta tagttttttt tttttaattt tatgttgaaa 2760
   ttttttatat tttgtgttcg ttttttatat tttatagttt ttgaagttta gtaaatgtag 2820
   gaggtatatt atatgtatta gttattagag gaggtatttt ttgttttttt atcgtagtag 2880
   ggaatttttt ttttgttgat ttttggtggt gatagtttga attgtatttt ttttttttag 2940
   ttagtggttt gttggtaggt aatgatgtat atttttttaa taatttaaat ttaaatgttt 3000
   aagtatagta ttaagtggta attttttttt attgttgaat tttgtacgtt ttttaggtag 3060
   tatagtgtaa tataaagaat agagtcggtc gggtgcggtg gtttacgttt gtaattttag 3120
   tattttgggc ggtcgaggcg gagggattac gaggttaaga gatcgatttt ttcggtcggg 3180
   cgcggtggtt tacgtttgta attttagtat tttgagaggt cgaggcgggc ggattacgag 3240
   gttaggagat taagattatt ttggttaata cggtgaaaat tcgtttttat taaaaatata 3300
   aaaaattagt cgggcgtggt ggcggtcgtt tgtagtttta gttgttcggg aggttgaggt 3360
   aggagaatgg cgttaattcg ggaggtagag tttgtagtga gtcgagatcg cgttattgta 3420
   ttttagtttg ggcgatagag taagatttta ttttaaaaaa aaaaaaaaaa aaaaaaaaga 3480
   tcgattttat tttggttaat atggtgaaat ttcgttttta ttaaaagtat aaaaataata 3540
   ataaaaataa gattagaatt tggattttgg agttagattt gtttaagttt aaattttagt 3600
   tttgttattt tttagttgaa tgattttggg cgatttacgt aagtttagtt tttttattta 3660
   taaaatggaa ataataaaaa taataggtta ggtatagtgg tttatatttg taattttagt 3720
   attttgggag gttaaggcgg gtagattacg aggttacgag atcgagatta ttttggttaa 3780
   tatggtgaaa ttttgttttt attaaaaata taaaaattag ttggatatgg tggtgcgtgt 3840
   ttgtaatttt agttattcgg gaggttgagg taggagaatc gtttgaatta gggagttaga 3900
   ggttgtagtg agttaagatg gtattattgt attttagttt ggtgatagag cgatattcgg 3960
   ttttaaaaaa taaataaata aaataataat aataaatatt ttaggggaga attagatgag 4020
   atgattgtaa aatgtatagt tttagttgtg ttttagtata tagtaggtgt ttgataagaa 4080
   atttgttatt tttttaataa tgggtatttg tttagtttgt gaattaatgt gttttttttt 4140
   tttaagtaaa atttacggtt aggtatggtt tacgtttata attttagtat tttgggaggt 4200
   cgaggcgggt aaattatttg aggtaaggag ttcgagatta gtttggttga tatggtgaaa 4260
   tttcgttttt attaaaaata taaaaattag ttaggtgtgg tggtaggcgt ttgtaatttt 4320
   agttatttag gaggttgagg taggagaata gtttgaattt aggaggcgga ggttgtagtg 4380
   agttgcgatc gtgttattgt attttagttt gggggagaag agcgagattt cgttttaaaa 4440
   aaaaaaaaaa aaaaatttat atagtaaaat ttattattgt tattgttttt aatgtgtttt 4500
   ttaaaaattt aggtaagttt tgtatatttt ttgattttta agttggaaaa taaggtaatt 4560
   tttaatatgg gttgtgaggt ttttttttgt ttaagtagtt gtagatatga attttgaata 4620
   taaagagatt ttaggttaga aaggattttg ggtagagttg aatggtttta agtatttgat 4680
   tttatattgg tttttttttg agaagagtta atagagttta gtattttttt ttaaggttag 4740
   ggaagggtaa agatttgaaa ttaggtatta ttgggttttt agttgggttt ttaagttttt 4800
   ttttttttat tatgtagagt aaagaggagg ttgtagaaga gaagaggata tgagattggt 4860
   tttttgtttt tttttttttt tttttgtttt ttttatattt attttttttt ttatagtagt 4920
   tttttttttt atagaggttg ggataggatg aggggggcgg agttggggat tgagggatta 4980
   gaagttttag gatgttttgt atttgaagaa agatttggtt aggggtagtt gtgttggttt 5040
   atgttttttt ttttttgttg ttgttatttt ttagtaagat gttagggttt ttggggtttt 5100
   gggtattatt ttttatagtt gtggaaggta agtgtttata gggaggggaa gggttttttt 5160
   atttatttag taagggaaag gggcgtttga agtaagagtt atttttttgg aagagtggtg 5220
   agtgggttgg gtgagtaagg gtgaaggata gagttatgtg tttttatggt agggtttagg 5280
   ttttaggttt ttgttgattt tgtttttttt tgtggattta ttatattgac gttgggatgt 5340
   ggaatatgtt ttgtttattt ttttggttag ttttttgttt ttgtatttat ttttattttg 5400
   aatttttttt tttttttatt ttgggtttta gt 5432

<210> 18
<211> 5432
<212> DNA
<213> Artificial Sequence

<220>
<223> chemically treated genomic DNA (Homo sapiens)

<400> 18

gttgaggttt agggtgggga aaggaaaaga tttaaggtgg gagtgaatgt agaggtaaaa 60
aattggttaa aagaatagat aaaatatgtt ttatatttta gcgttagtat ggtaaattta 120
taggaggaag tagggttagt agaggtttgg aatttgagtt ttgttatggg gatatatggt 180
tttatttttt attttatttt atttagttta tttattattt ttttaaaggg gtggtttttg 240
ttttaagcgt tttttttttt tgttggatgg atggagagat tttttttttt tttgtagata 300
tttatttttt atagttgtgg gaagtaatgt ttaaagtttt aaagatttta gtattttgtt 360
ggaggatggt agtagtagaa ggagaagagt ataagttagt atagttgttt ttggttaaat 420
ttttttttag atatagggta ttttggggtt tttgattttt tagtttttaa tttcgttttt 480
tttattttat tttagttttt gtgggggaga aggttgttgt gggggagagg gtgggtgtgg 540
ggaaagtagg gaaagaaagg gaggagtaga gaattaattt tatatttttt ttttttttgt 600
aatttttttt ttgttttgta tagtgagagg aggggaattt ggaggtttag ttgagaattt 660
agtgatgttt aattttaaat ttttgttttt ttttgatttt ggaagaagat gttggatttt 720
gttgattttt tttaggagga aattaatgtg aggttaaatg tttagagtta tttagttttg 780
tttaagattt tttttagttt aagatttttt tatatttagg gtttatattt gtaattgttt 840
gggtagagga aggttttata gtttatatta gaggttattt tgttttttaa tttaagaatt 900
agaaagtata taaaatttat ttgaattttt aaaaaatata ttgaaaatag taataatggt 960
aaattttgtt atgtggattt tttttttttt ttttttgaga cggagtttcg tttttttttt 1020
ttaggttgga gtgtagtggt acgatcgtag tttattgtaa ttttcgtttt ttgggtttaa 1080
gttatttttt tgttttagtt ttttgagtag ttgggattat aggcgtttgt tattatattt 1140
ggttaatttt tgtattttta gtagagacgg ggtttattta tgttagttag gttagtttcg 1200
aattttttat tttaggtgat ttgttcgttt cggtttttta aagtgttggg attataggcg 1260
tgagttatgt ttagtcgtgg atttatttta aaaaaaaaaa atatattggt ttataggttg 1320
aataaatatt tattattaag gaggtaataa attttttatt aagtatttat tatgtgttgg 1380
ggtatagtta gggttatgta ttttataatt attttattta attttttttt gaaatattta 1440
ttattattat tttatttatt tattttttga gatcgagtgt cgttttgtta ttaggttaga 1500
gtgtagtggt gttattttgg tttattgtaa tttttgattt tttggtttaa gcgatttttt 1560
tgttttagtt tttcgagtag ttgggattat aggtacgtat tattatgttt agttaatttt 1620
tgtattttta gtagagatag ggttttatta tgttggttag gatagtttcg atttcgtgat 1680
ttcgtgattt gttcgttttg gttttttaaa gtgttgggat tataagtgtg agttattgtg 1740
tttggtttat tatttttatt atttttattt tataggtaag aaaattgaat ttacgtaagt 1800
cgtttaaagt tatttagtta ggaaatagta gagttggaat ttgaatttag gtaggtttga 1860
ttttaaagtt taggttttag ttttattttt attattattt ttgtattttt agtagagacg 1920
gagttttatt atgttggtta ggatggggtc gatttttttt tttttttttt ttttttttga 1980
gatggagttt tgttttgtcg tttaggttgg agtgtagtag cgcgatttcg gtttattgta 2040
agttttgttt ttcgggttaa cgttattttt ttgttttagt ttttcgagta gttgggatta 2100
taggcggtcg ttattacgtt cggttaattt tttgtatttt tagtagagac gggtttttat 2160
cgtgttagtt aggatggttt tgattttttg atttcgtgat tcgttcgttt cggtttttta 2220
aagtgttgag attataggcg tgagttatcg cgttcggtcg aaggggtcga ttttttaatt 2280
tcgtgatttt ttcgtttcgg tcgtttaaag tgttgggatt gtaggcgtga gttatcgtat 2340
tcggtcggtt ttattttttg tattatatta tattgtttga aagacgtata aaatttaata 2400
atgaaaaaaa attattattt aatattatat ttggatattt gaatttaaat tgttgagaaa 2460
atatgtatta ttatttgtta ataggttatt aattaaaaag ggagggtata gtttaaatta 2520
ttattattag aggttagtag aggagaaatt ttttgttgcg gtgagagggt agaaagtatt 2580
ttttttgatg attaatatat atagtatatt ttttatattt attaagtttt agggattata 2640
aaatgtgaag ggcgggtata ggatgtggag agttttagta tggagttggg aggagagggt 2700
tatggtttgt tttgtagtag agatagagga gtttaatttt attagatttt tttttagagt 2760
aagaaataat agaaataggt aattattttt atatttgtga gttattggtt ttttgagaat 2820
taagagttag aggggatttg ttttttgttt ttttagtttt ttgttttttt agtggttttt 2880
tggaaatttt tttttggaat gatttggttt agattaggga ggatagtttg agtatcgata 2940
tttttttttt tttttttgag acggagtttc gttttgttat ttaggttgga gtgtagtggt 3000
gcgatttcgg tttattgtaa tttttgttta tcgggtttaa gcgatttttc ggttttagtt 3060
ttttgagtag ttgggattat aggcgtatat tattacgttc ggttaatttt tgtattttta 3120
gtagagacgg ggtttcgtta tgttggttag gttggtttta aatttttgat tttaggtaat 3180
tcgttttttt cggtttttta aagtgtcggg attatagacg ggagttattg cgtttagttt 3240
gagtattgat tttttgattt tggggttttt tttttgggaa atttgtatga aaggaagaga 3300

tagtaaaagt tttagtagtt gtgaaatagt agcgggagga atgattaggg ttcgagatag 3360
tcgtttattt ttttttggat ttttttagga gtttgtaggg ttttgttagt gagatggaat 3420
gttgtgtttt tagttatttt tttttattag ttttagatta gataagtttg gggtggtgtg 3480
tgtggtgggg tgtgagggga gatttgaagg tgaaatagtt ttttttgttt ggaatgtttt 3540
aggagattat tttttttaga gtgggagtag cgtaaggatt aggtagaaag gtagtttttt 3600
tatttaaagt ttcggataat taattttttt taagatagaa atattttagt taggtttatg 3660
ggaattatat ttgttttggg ttgttaggtt ttagagttaa gatggtgata gaggttgggc 3720
gttgtggttt atgtttgtaa ttttagtatt ttgggaggtt aggagttttt ttgggtaata 3780
tagcgagatt tttatttata ttatttttaa aaaaaaattt tttttttttt tgagacggag 3840
ttttattttg ttatttaggt tggagtaaag tggtgtaatt ttcgtttatt gtaattttcg 3900
ttttttgggt ttaagtaatt tttttgtttc ggttttttaa gtagttagga ttatagatgt 3960
ttgttattat gtttggttaa ttttttgtat ttttagtaga gatggggttt tattatgttg 4020
gttaggttgg ttttaaattt ttgattttag gtgatttatc gtttaggttt tttaaagtgt 4080
tgggattata ggcgtgagtt attgcgcggg gttaaaaaaa atgtatttta ttaggtgttg 4140
ggtttataaa gatagaggtg tgattattgt tttttgggaa tttatagtgt agaaatgtag 4200
gtggatattg ataagtaatc ggaaaattgt gtttttattt aggggtatga ggaaagagtt 4260
atataaatag attaagtaag ttaattttgt ttagggtata gaagaaaggt gtttagagag 4320
ttttatagag aaggggatat ttaaatagtt ttaaaggtaa atataatggt aagaggagaa 4380
tagttttttt aagtaaaggg aatggtaaga gtaaaggtat ggagatataa atataaaggt 4440
ttgtgtgtta aaggtggaag gagtgaggag gaagtagtat gagatatgag atagaaaatt 4500
attttagaga gagacggatt agattggata ggtttttaat tttgtattaa ggagtttagg 4560
ttttttttaa aagtaaagaa ttttaggtag gggtttggtg gtaattggaa gatggatgat 4620
tattggggtt tggagagatt agttgtaaga tttaagtagt aaataaagtg agtattgaga 4680
tagtggaaat gaaaataaaa gaggaggtcg ggcgtggtgg tttacgtttg taattttagt 4740
attttgggag gtcgaggcgg gtagattacg aggttaagag atggagatta ttttggttaa 4800
tatggtgaaa attcgttttt attaaaaata taaaaattaa ttgggcgtgg tggcgcgcgt 4860
ttgtaatttt agttatttag gaggttgacg taggagaatt gtttgaattt gggaggcgga 4920
ggttgtagtg agtcgagatt acgttattgt atttagtttt ggcgatagag cgagatttta 4980
ttttaaaaaa aaaaaagagg aaaaatttga tatatattgt ataggtaatg ttgataggat 5040
tggtaattaa attagtgtta gtaggtgaaa gggagaatta aaagatgatt ttgaggtttt 5100
aagtatgagt gattaaggag tagtgggtga tattattaag ggagatgtga aatataaaga 5160
attatttttt tttttattgt gttgaattta tttggaggat atttagatga aaatgtttag 5220
taagtagatt tttaggttaa gagataagtt agggtttaag atttgggagt tattaatatg 5280
gatatagttt tatgttagag ttttatgtta gagtttaaga gggaaaaggg tagggaaagg 5340
aaaggatacg agataggaat ataaatatta aagggatata ggaagaagtg gttatgaagg 5400
agattaaaaa atatagaaat gtgttatata cg 5432

<210> 19
<211> 6641
<212> DNA
<213> Artificial Sequence

<220>
<223> chemically treated genomic DNA (Homo sapiens)

<400> 19

aaagaagaaa gaaagaataa attagagaat tttggaggaa atatagagta gagtgtagta 60
tgggaaattt taagtttttt agaaaagtta tatgttatta tgaagtattt gtaatagaaa 120
tagtagtttg aggttttaga agatcgttaa gtggtatgaa agaaatttag aattttttgt 180
ggaattttgt ggaagatgta gaagttagtt gaggattaag tagtagttta tttattaggt 240
agtggttgtg gtataaggtg aatagtttta aagaaaaggt tgtagggtgg atagttaacg 300
ttaggggtta gatggaggat ttggttgaag tttggagggt tagttgtatt attaggagtt 360
agggtaggaa tttcgaatag taagtaatga gagacgattg agttttttga tataattatt 420
gtgtattatt tatgttgaga gaataagtta taaattgtga atatttaagt agtgaataga 480
gtgaggtaaa atgagttata ttttaagtgg gatttagtag aggttatagg gattagattt 540
ttgtttttcg gtgttatgag tgagtttgta ttttatattg attatttgta taaaagagat 600
ttaatttaga atagattttt atattttaat tgataatgtt aatttttatt ttgttaaaat 660
tgtgttttta attaattgga ataggtgttt tagaatataa tgagattagt taaatagtta 720
tatttttata attaattttt taggtgaggt taattgggtt ttattatgta tatttttaaa 780
ttttaatata tgtatagaag ttagtagatg aatttttttt ttaaaaagag agaaaagaga 840
aaaatagtta agtaaagatt tttttaaatt atagtagata tttttatgtt ttgattgttt 900
ataatttttt taattgtttt aaataatatt ttgtttgatt attttttttt tagaaagttt 960
ttttttttaa tttataatta gttagtttta ttaatttttt tatatttaga gtagatatta 1020
gtatatttaa attttattaa ggagtttata gaattttttt taaaggaaat aggtagtaaa 1080
ataatattta attagtttgt atttaagttg ataatagaag atgtaaatga aataattaga 1140

aaataattta aggtagatta tggtgttttt gtttataaat gtgtttatta gataaagaaa 1200
gaattttgga aggaaggatt tgttttgtaa ttagaaaaat aaaaattgat ttacgaattt 1260
gaattgaaaa gtaggtaaag aattttagta tttatttacg tatttattta aatataaaag 1320
ggaattgatt attattttta ttataataat tatgtgttat acgtgtataa ggttagatta 1380
aagtggtagt taagaatatt atattttgta gttgtttaag gatgatagtt gagaaagttt 1440
gatgatagag gataaaaata atatttgttt gggtataaat ggtaggtttt ttttttaatg 1500
aaatatgtga atgttttttt ttttatttat aattttagaa atttaagaaa ataattttgt 1560
aatattaagt ttattatttt atcgaattat aattattttt attttttgaa atgaaaaaat 1620
aattatgttg taaatacgag aataaattga ggtaatgata atatattaat taattattgg 1680
gtttaattta attattagtt ttgttgtata ataaataata tgtataattt ttgtttaagt 1740
attttttttt atagttgtaa aataaagttt ttgataggag ttagttaata gtaatttaga 1800
gttgttttta aattgtagaa ataaaaatgt ttataatttg aatgtgtttt gttgataaag 1860
agattatggt ttattttttt tttttaagtg gaatttttag tttttagatt gatagttagt 1920
tataaagaga ggaaggtaat ttaaagtttt ttttggaggg tattattatt gtagtttagt 1980
attagtagtg ggtagaaatt atgtgtttta ttatattttg atttttatat tttaagttaa 2040
gagaattttg agtaaggtta gtttttttat ttatttagtg tgtattttgt atagatgtaa 2100
aaaaattttt tttatagaat ttgttttgtg gatttagttt atgtttgaat gaaatatgtt 2160
tttttttatg ttggtgattt tataagaaaa taaggttagg agaggtattt ttttaaatgt 2220
ttaaaggaga tattattatt attattatta tttgttaagc gatttataaa gtattttgag 2280
gtttaaggtt agtatgggag atgagttagg tttaggagaa gttaatataa agataaaggt 2340
tagtgttttt ttttttttta gttagttaat ataataaagt atttagataa taatttttag 2400
cggttaggta atgatgtatt ttttgtaatt attatatttg tgtttgttta ttttttttat 2460
gttagtgagg tttggaattg gaattgaaaa ttttttaggg aaaagttatt tttgtaattt 2520
agttggggtg tttttgtttt tgggtttttt tattgtatta gtttttagt ttggtaggat 2580
agttatgata gtgttttgtt ttttagatga aatgtagaat taaggatatg attattggtg 2640
tttattaaag attttttgtt attttttata agagtagggg cgttagattt ttgtttattg 2700
gttaaatttt aaattgggag attatatttt gtttttttaa atttttttag tagaagtagt 2760
tatttatggt attttatttt gttttttgtg ttaaattgtt ttgtgatgtt attaaatggt 2820
tttggttttt tattttagaa atgattattt tagtggggtg gggagtaaat cgatatttat 2880
tgtttgatat tatgtttagt ttagttttaa aaaaattaaa aggtagtttg tggtagttaa 2940
ggataaagta taaaataaaa tattttaacg tataaaatat tttaatatat tagttgttgt 3000
ggtgtttttt aaaaaaatta atttgttttt ttttttgtaa tatagataaa atattaatgt 3060
atttttttat tttattagta attgataaag aattgttatt tgttttatat ttaaggttaa 3120
ttttaatagt gatgaagttt gagaggatga aattatatat tatcgattaa ttaaattatt 3180
ttttttgttt ttaggttgta tttaattatt ttagattttt atttatttat tttataaaat 3240
ttttttggaa ggagattttt tatttttttt tggtaatata ttttagtgtt ttttaatttt 3300
tttataaaaa ttaagttttt atttttgttt aatttcggtt ttatttgttg gagtgtaatg 3360
ttattatttt ttgtttattt ttagttttgt tattatatat tgttagtgag aggtaatgta 3420
gtataatggt atggtttttg gaattagata tgttagtatt ttaattttag ttttgttatt 3480
ttttaggtgt gtgatgttgg gtaaattatt tttgaggttt aatagtttta tttgttaatg 3540
agtaatttta gagtattgtt gtgaagataa aacgagatag tattttattt tgaaggatta 3600
tggggtagga cgaggtatgg tattggagat ttaaaaagtg tttaatatat attataatag 3660
ttatttattt gattatgtgt atgtatttga tttaatttat aaattttttt agagcgggag 3720
gtatattttt tgagtacgtt ttggatttta atagaagtat agtgttgtgt atatagtatg 3780
gtgtagaggt ttaatattat tgaatgaata aattgttttg tttttatttt ttgagtggaa 3840
tgaatagttt ttagttattt gtaagatttt acgtatgtag aaaaaaggtg ataaaaatta 3900
atttgtagtt tttaaatatg tagatatttg attttattta gatgttttag tatggtgtag 3960
atataatgta aataatttac gttttttatt tttattacgt ttatttttac gaattttaat 4020
atttgattat tgttattatt tttattatta agttgtattt aggtatttag aaagggattg 4080
atttatatgt ttttttattt atttaagtat ttaattgaga aattttaaaa atgaatatta 4140
agagttaatt atatatttga ataaaatttt ttaatttgtg attaagtata ttattttttt 4200
tatttatttt taagatttga gtattataga taatattttt aattgttttg tatttaagtt 4260
aagaaataaa aattaaaatt agaaaagttt attattgtaa gttttaaagt ataattagta 4320
tttttgaatt agtgaagtat tagttaggat tttatttttg gtttttaaat gtttaaacgg 4380
ggtaaggagg gtaagttggg ggaataagta taagatatat agatttagtc ggatttgaat 4440
atttttttat aaggaaagga aagagtattt tggtgtgtgt ttaggttgga attaaaaaga 4500
tagtagcgtg gttatttttt ggattgtttt gtatatattt tataggggcg gttttaggta 4560
tggagttttg ggtatttgtt cgtttatatt ttaatagttt agaaattcgt gtatttgagg 4620
tcgggtggtt taggttttac gtaattgggt ttaagtgtta ttaatattgt ttgcggcggg 4680
gggttattga gttgtgtttt gggatttgga tgtttatttc ggattttttt tttttttttt 4740
ttttattttt attagttttt tttttttttt ttttttttcg ggcgtgttta tttttttttt 4800
ttgtttttat ttttcgttgt ttgatttttg ttttttttat tttttttttt aatcggtttt 4860
ttgtttttta atttttgtcg gtcggagaga ttgagattgg ggttggggtt ggggttgcgg 4920
tttagaagag gtacgagtcg ggcgggatag gcgcgaggcg tttagtcgcg aaagcgagga 4980
gcgcgtatcg aggcgggggc gcgcgcgggg tagtcgcgtg gttaggtaga cggggtcggg 5040
agcggcgcgc gcgcgtttcg ttagtttttg gcgcgttttc ggcggtttga gtcgtagcgt 5100

gttcgttttc gtcgttagtt cgttttattt atgggtcgga aacgttgcgt gggggagatt 5160
gttttaagat ggcggcgggt tgttgcgggg tgaagaagta gaaattgttt agttcgtttt 5220
tttttggttc gggtggcggt ggtggcgttt tttttttttt ttattgtagc ggagagagtt 5280
agtgtcgagt tggggagttg ggattaggag gcgtcggtac gcggtttaac gggttgggag 5340
gtttaatcgg aggaggtagc ggtagcggtt gtattttttt ttttttttag ggttgcggcg 5400
gcggcggcgg ggggatcgtt ttgtcgttat tttcgagttg cggagtgggg attttatttt 5460
ttatttcggt cgtcgttatt ttttttttat tttttttatc gttcgtcgtt tcgtttttat 5520
cgtcgggttt tcggaagatg gtggtgttag tagagatgtg ttgtttttgt ttcgatgtgt 5580
tttattgtta tttgtatgga tattagtagt ttcggatttt tcgatttatt aacgagtttt 5640
agtgagtatc gtgtttttcg tcgttttttt ttttgcgttc gggatggggt ttagagttga 5700
ggtaaagtgc gagttcgttt ttcggtcggc ggatgttttt gttttttcgt tgttcgggtt 5760
ttcggagtta tttaattgaa ttaagggtat tttgcgtttt ttgtttttcg aggtaggtat 5820
tttatatttg gagtagggtg ttgttgtaag gaatggtttc gtggttttta gaggtgttag 5880
tttttttttt gcgatgattt cgaggagttt tgattttttt atttgttttt tttattattt 5940
ttattatttt ttattttgtt tcggttttgg agatttgggt tatttaaggt gttgattttt 6000
ttttattttg gtttttatta ttttaagtga aagtttcgtt agtattttcg tttcgcgtat 6060
tttgtatatg gagaattatt attttattgt aggatgaaaa atagtaatta tttaattttt 6120
gttagtgatg gaatttagtt ttggagtttg tgaggttatt tatttttttt tttgggtttt 6180
tttgtgtttt gggaaggggg gaggtttttt aaaatatatt ttttttttat tatttatttt 6240
tatttaaata tatttattaa aatgtgtttt tcgtgtagcg agcgtattgt cggttttttt 6300
atatttgaag agtgcgattt tttttttttt tttttttatt ttatcgtttg gataatagtt 6360
tatttaaaga gaattgtttt tatagagtta cggttagttt gtatattaag cgtgtattcg 6420
tttttgtgtc gattttattt tttaagaaat ttatgttata tttgatgatt tgagaatagt 6480
taaatagtaa atgtgtagga attgttatgg ttttttttgg ttttgtattt taaattattt 6540
tattttttaa gtgtgtggtt gaaaaatttt aaaatagtat tgttatagtt aaaagttagg 6600
ttaattttaa tttttgttta tttggatgtg tgggaggtgg g 6641

<210> 20
<211> 6641
<212> DNA
<213> Artificial Sequence

<220>
<223> chemically treated genomic DNA (Homo sapiens)

<400> 20
   tttatttttt atatatttaa atggataggg attagaatta atttggtttt tagttgtaat 60
   agtgttattt taaaattttt taattatata tttgaagggt aaaataattt aagatataaa 120
   attaaaggaa attataataa tttttatata tttattattt aattgttttt aaattattaa 180
   gtataatatg gattttttaa aaagtgaaat cgatataaaa gcgaatgtac gtttaatgta 240
   taaattgatc gtggttttgt ggggatagtt ttttttgaat gagttattat ttagacggtg 300
   gaatggaaaa aaaaaaaaaa aaggtcgtat tttttaaatg tgaggaagtc ggtagtgcgt 360
   tcgttatacg agaaatatat tttaatgggt gtatttggat aaaaataagt agtaagggaa 420
   aaatgtgttt taagaaattt tttttttttt tagggtataa gaaaatttag aggagaagat 480
   gggtaatttt ataagtttta gagttaagtt ttattattaa tagaagttgg atagttgtta 540
   ttttttattt tgtaatggga tagtagtttt ttatgtgtaa gatacgcggg gcgggaatat 600
   tggcggggtt tttatttggg gtaatggaaa ttaaagtgag gagaggttaa tattttgagt 660
   aatttagatt tttaaaatcg gggtagggtg aggggtggtg ggggtggtga aggagataag 720
   taaaggagtt aaaatttttc gaagttatcg taaagagaaa gttaatattt ttaggagtta 780
   cggggttatt ttttgtagta gtattttgtt ttaagtgtgg ggtgtttgtt tcggagggta 840
   aaaagcgtag ggtgttttta gtttagttga gtagtttcgg ggattcgggt agcgagaggg 900
   taggggtatt cgtcggtcgg gaggcggatt cgtattttgt tttaattttg agttttattt 960
   cgagcgtaag ggagagggcg gcggagggta cggtatttat tagggttcgt tggtgaatcg 1020
   gggggttcgg ggttgttggt atttatatag gtgatagtag agtatatcga agtaaaagta 1080
   gtatattttt gttgatatta ttatttttcg ggagttcggc gatgaggacg aggcggcgga 1140
   cgatgaggag ggtgaggaag aggtggcggc ggtcggggta gaaagtaggg tttttatttc 1200
   gtagttcgga ggtggcgata gggcgatttt ttcgtcgtcg tcgtcgtagt tttggggggg 1260
   agagagggta tagtcgttgt cgttattttt ttcggttaga ttttttagtt cgttgagtcg 1320
   cgtatcggcg ttttttagtt ttagtttttt agttcggtat tggttttttt cgttgtagtg 1380
   ggaggaggag gaggcgttat tatcgttatt cgagttagag gggggcgaat tggatagttt 1440
   ttgttttttt atttcgtagt aattcgtcgt tattttggaa tagttttttt tacgtagcgt 1500
   tttcgattta taagtgaggc gagttggcgg cgggggcgag tacgttgcgg tttaggtcgt 1560
   cgggggcgcg ttagaggttg gcggggcgcg cgcgcgtcgt tttcggtttc gtttgtttag 1620
   ttacgcggtt gtttcgcgcg cgttttcgtt tcggtgcgcg tttttcgttt tcgcggttgg 1680
   gcgtttcgcg tttgtttcgt tcgattcgtg ttttttttga gtcgtagttt tagttttagt 1740
   tttaatttta attttttcgg tcggtagagg ttggaagata ggaagtcgat tagaaaggaa 1800
   ggtgaaggaa ataagagtta gatagcggaa agtgggagta aagggagaag gtgggtacgt 1860
   tcgggaagga aggagggaaa gggaaattag tgggggtagg ggggaagaaa ggagaaagtt 1920
   cgaagtaagt atttaggttt taaagtatag tttagtaatt tttcgtcgta agtagtatta 1980
   atgatattta agtttagtta cgtggaattt gagttattcg gttttaaata tacgggtttt 2040
   tgaattgttg gagtgtgagc gagtaggtgt ttagagtttt atatttgaga tcgtttttgt 2100
   aaagtgtgta taaagtaatt taggaaatgg ttacgttgtt gtttttttaa ttttagttta 2160
   agtatatatt agggtatttt tttttttttt tatggagaaa tatttaagtt cgattgaatt 2220
   tatgtatttt gtatttgttt ttttaattta ttttttttgt ttcgtttaaa tatttaaaag 2280
   ttaaaaataa agttttaatt aatattttat tggtttaaag atattaattg tgttttggga 2340
   tttgtagtgg tggatttttt tggttttggt ttttattttt tggtttgggt ataaagtaat 2400
   taggaatatt atttgtagtg tttagatttt agagataaat agggaaggta atgtatttag 2460
   ttataaattg aaagattttg tttaagtata taattagttt ttaatattta tttttaaaat 2520
   tttttaatta aatgtttgga tggatgagga gatatatgag ttagtttttt tttgggtgtt 2580
   taagtgtagt ttaataataa aaataatgat aataattaaa tgttaaaatt cgtagaaatg 2640
   aacgtgatag aaatagaaag cgtaagttat ttgtattgta tttatattat gttaaaatat 2700
   ttagataaag ttaggtattt gtatatttaa aaattgtaga ttgatttttg ttattttttt 2760
   tttatatgcg tgggattttg tagatagttg ggggttgttt attttattta gggaataaaa 2820
   ataaaataat ttatttattt agtaatattg agtttttgta ttatattatg tgtataatat 2880
   tatatttttg ttgaaattta gggcgtattt aggaagtatg tttttcgttt taaaagagtt 2940
   tatgaattaa gttagatata tgtatataat tagataaata gttattatag tatgtattga 3000
   atatttttta ggtttttaat attatatttc gtttatattt ataatttttt agaataagat 3060
   attatttcgt tttattttta taataatgtt ttgaggttat ttattaatag atgaggttat 3120
   taaattttag aagtaatttg tttaatatta tatatttaga aagtaataga gttaggattg 3180
   gaatgttggt atgtttgatt ttaaaaatta tgttattata ttatattgtt ttttattaat 3240
   aatatatgat aataaggtta aggatggata aaagatagtg atattatatt ttagtaggtg 3300
   gaatcgaggt taaatagaaa taagaattta gtttttatgg aaagattgag agatattgaa 3360
   atatgttatt aaggaaagat gaaaaatttt tttttaaaaa gattttatga gatagataga 3420
   tgaaaatttg gaatggttag atgtagttta aagataaaag ggatagttta attggtcggt 3480
   gatatgtaat tttatttttt taaattttat tattgttaga attaatttta agtatgaaat 3540
   aaataataat tttttgttaa ttattagtgg agtaaaaaaa tgtattaata ttttgtttat 3600
   gttatagaag agaaagtaga ttggtttttt taaaaagtat tataataatt ggtatgttag 3660
   ggtgttttgt acgttagggt gttttgtttt gtattttgtt tttaattatt ataggttatt 3720
   ttttaatttt tttgaaatta gattgaatat aatattagat aataggtatc gatttatttt 3780
   ttattttatt aaagtgatta tttttagagt aaaaaattag agttatttaa tgatattata 3840
   aaatagttta gtataggaag taaagtagga tattatggat ggttgttttt gttaggggaa 3900
   tttaggaaag tagaatgtaa ttttttaatt tagaatttgg ttagtagata agagtttaac 3960
   gtttttattt ttgtgaaaaa tagtaaggaa tttttaataa gtattagtag ttatgttttt 4020
   agttttgtat tttatttaaa agataaagta ttattatggt tattttgtta ggttaaggga 4080
   ttgatgtaat gggaaaattt aaagataagg atattttagt tgaattataa aaatagtttt 4140
   tttttagagg atttttaatt ttaattttaa attttattag tataaagaaa atagataaat 4200
   ataaatgtga taattgtaga aaatatatta ttatttgatc gttaggggtt attatttaag 4260
   tattttgttg tattggttga ttaaaagggg gaaaaatatt aatttttatt tttgtgttaa 4320
   ttttttttga gtttggttta ttttttatat taattttgag ttttaagatg ttttataaat 4380
   cgtttggtaa gtagtggtga tggtggtaat gtttttttta ggtatttgag gaaatgtttt 4440
   ttttggtttt gtttttttgt aaagttatta gtataaaaag aaatatattt tatttaaata 4500
   taaattaaat ttatagaata aattttgtga aaaaaatttt tttatattta tgtaaagtgt 4560
   atattgggtg gatagaaaaa ttgattttgt ttaggatttt tttgatttga agtataaagg 4620
   ttaaaatatg atagagtata tggtttttat ttattgttga tattggattg tagtgataat 4680
   attttttaga ggaaatttta agttgttttt ttttttttgt aattgattgt taatttggga 4740
   gttgaagatt ttatttaaaa aaaaaaaata ggttatagtt tttttgttaa taaagtatat 4800
   ttaaattgtg agtattttta tttttgtaat ttagaggtag ttttaaattg ttgttaatta 4860
   atttttgtta aaggttttgt tttataattg tggaaaaaaa tatttggata gaggttatgt 4920
   atattattta ttgtgtagta aagttagtaa ttgaattaaa tttaatagtt agttaatata 4980
   ttattattgt tttaatttgt tttcgtgttt ataatatagt tattttttta ttttagaaaa 5040
   taagaataat tataattcga tgaggtggtg aatttaatgt tgtaaaatta tttttttagg 5100
   tttttggggt tgtgggtaaa aggaggaata tttatatatt ttattgaaaa aaaaatttgt 5160
   tatttatgtt taaataagtg ttatttttat tttttgttat taaatttttt taattgttat 5220
   ttttgaataa ttgtagaatg taatattttt agttattatt ttgatttaat tttgtatacg 5280
   tataatatat ggttgttgtg gtggaggtgg taattagttt ttttttatat ttgaatgaat 5340
   acgtgagtaa atattgaaat tttttattta ttttttaatt taaattcgta aattagtttt 5400
   tgtttttttg attataaaat agattttttt ttttaaaatt ttttttttat ttaatgaata 5460
   tatttatgag taaaagtatt atgatttatt ttggattgtt ttttagttgt tttatttata 5520
   ttttttattg ttaatttgag tataaattaa ttgaatgttg ttttgttatt tatttttttt 5580
   agagaaagtt ttataaattt tttaatgaga tttagatatg ttaatattta ttttgaatgt 5640
   ggaagaatta ataaagttag ttagttgtag attggagaaa aaggtttttt aggaagaaga 5700
   tgattaaata gagtgttatt tggggtagtt agaggagttg tgaataatta aaatatgaag 5760
   gtgtttatta tagtttggga aagtttttat ttgattgttt tttttttttt ttttttttta 5820
   aaagaagaat ttatttatta gtttttgtat atatattggg gtttgaaaat gtatataatg 5880
   aaatttaatt ggttttattt gaaaagttaa ttataaaaat atagttgttt aattaatttt 5940
   attgtgtttt ggggtattta ttttagttgg ttggaggtat agttttggta gggtgaggat 6000
   taatattgtt aattaaaata taaaagtttg ttttgaatta agtttttttt gtataaataa 6060
   ttaatgtaaa gtataaattt atttatggta tcgaggagta gaaatttgat ttttataatt 6120
   tttgttagat tttatttgaa gtgtagttta ttttgtttta ttttatttat tgtttaaatg 6180
   tttataattt atggtttatt tttttagtat gagtaatata taatgattgt gttaggaagt 6240
   ttagtcgttt tttattattt attattcggg atttttattt tagtttttaa tagtgtagtt 6300
   aattttttaa atttagttta gattttttat ttggtttttg acgttggtta tttattttat 6360
   agtttttttt ttggggttgt ttattttatg ttatagttat tgtttggtaa gtaggttgtt 6420
   gtttaatttt tagttgattt ttgtattttt tataagattt tataagggat tttagatttt 6480
   ttttatgtta tttgacggtt ttttaaaatt ttaagttgtt atttttattg tagatatttt 6540
   atggtagtat atagtttttt taaggggttt gaaatttttt atgttatatt ttgttttgtg 6600
   ttttttttag aattttttgg tttgtttttt tttttttttt t 6641

<210> 21
<211> 11280
<212> DNA
<213> Artificial Sequence

<220>
<223> chemically treated genomic DNA (Homo sapiens)

<400> 21

aaacgggtaa agtttaggtt gttataaagt ttagcggtgg ggtaaggatt gttttgagtg 60
tagttttgtt agtttttatt tttgacgttt tggagttggg ggtgggggat ttaattattt 120
aaattttttt aggaaggtcg tatttaggcg tagggatgat ttagtattta ttatttattt 180
atatttatat tagttgttat tttgttggtt cgtagtaggt cgcgggtagg gggtagagtg 240
atcgggcgtt agagttgttt ttgatgttgg ttaaggtcga gtagtttata tattttgtag 300
ggattacgta gtttttaatt ttgtcgattt tagttttttc gtagcgagtt tggttttatt 360
tgttagagtt tttcgtattt cgtgttagcg ggattattta aaaatatttt ttagtaacgc 420
gcggggtatt ttcgtcggtc gggatcgacg tggttgtttt tgatttagtc gtttgatttt 480
atattatatt ttagaaaggg gaaattgagg tagggtgagg aagtgcggtt agtttattta 540
tttttaagta gtttggttcg gttcgcgtat ttagagttta gagttcggag ttttcgagtt 600
taggtcggtc gggttattag cggagtcggt tagtttcgga gtcgcgtatt tatcgttttc 660
gttatcgtcg tcgattttcg ggtgtcgcgt tcgcgcgtta tcgtagttag tttagtatcg 720
tttagttcga agtcggcgcg tttattattg ggtcgcgtag cgaggggcgg ggcggcgcgt 780
cgtatgtcgg cgtttcgttg gttgggcgcg ttgtcgttcg gaggttgtta ttttttggcg 840
tttcgttttc ggggttgcgt taaagtttcg ggttgcgcgg gcgtttgagc gttttttgat 900
ttagcggttt tcggggattt cgcggttttt tgtttgtagg gacgttatgg gaggtcgtag 960
tcgtttttga gtttatgttt ttatggatat tatttgttat ttgtttattt tgcggttttt 1020
ttcggaagag gtataggaat gtttattttc ggattggttt ttttagattg cgttttggtt 1080
tttttatttt gtttatttaa ttttattttt ggatttggtt attttttttt tggttatttt 1140
tttttttatt ttatggtttt taggtcggtt tttttttttt tgatgttttt gttattttta 1200
gtatgtattt ttagattttg gattaggtat ttgagtatta tattttgtat atttattatt 1260
ttttttattt ttatttttta gtgtagtttt tgagtttata tttaggttta ggttttagcg 1320
acgaggggtt ggtttagttt gatttgtgta gggtgagtag gtgggtattg attggggttt 1380
ttttaatagg atagaggtat tagttgggat cgttgttttt gtagtttttg ggttgtgttt 1440
gggtttatgt ggtagaaagg tggagagtta aagtttagag gtttaggttt tgttttaaag 1500
tgagtattta aaaaattatg atatggatga taaagatatt agggataatg ataatgtgta 1560
aagtatttta aatttaatgt ttgggttagt agttttttta ttttatttta tagatgtaaa 1620
tattgagatt tggagtagtt atgttattta gttgaagttg aaaaaggtgg taagtaagag 1680
aggttatatt agttttaatt tgtgttgaag atttaggtgt tttgatgttt tgtagaaaat 1740
tggggtttag ggatgggaag agtttggttg gtaattagta acggtaagag tttaatttgg 1800
atttatatgc gggttcgttg tgttttatat ttttgttatt ttatttgtgt tttttttgga 1860
atttaataga attttttata aggattttag atgtttaggg atttatggtt agcgggtcgt 1920
agatttagtt agatatggtt gtttttttta ttatttagta gtgggattat gtggggttgg 1980
tattagtgat ttttttttga gtagttaaat ttatgtaatt tatagggtta agaggtggtg 2040
gtggtggata agtgggattt ggggtttggg ggtgatggat tgtgttttgg ggtttatggt 2100
agtatttgga gaatgggttt ttttaaagta ttaggttatg tattttaaag ttggatatga 2160
gttgattaag gttttttttt gtttataaag agggatgtag taatatgtta gatggtggtt 2220
tgtattttgt tttgttatat tttgtggttt tgggttattt ttatttttag ttatattttt 2280
tttagttaga tagagttaga gtagatattt tgattttttt tgttattatt gatattttag 2340

tttggatgat atgcgatgtt aggaagtagg tgacggtaga tgaaatgggt ttaggatggg 2400
tatttaagtt ttgggatttt ggggttgtaa taggtttatt ggtatttaat ttttaattgt 2460
atataaattt agataggaag tttattattt tttgtggtta tttttttgat tttaatagtt 2520
gttatttttg aaaagttttt gatatttagt tgaaagtatt ttttttttgt ggtttttttc 2580
gggggtttta tagttgttcg tatggagtta ggtgagagga tgagagaggg agaatattgg 2640
gttattggtt tttgtggttt tttttgttgt gtttttttta tttttttttt tttttttttt 2700
ttttgagatg gagtttcgtt ttgtttttta ggttgtcgtg cggtggtgta atttagtttt 2760
attgtaattt tcgtttttta ggtttaagcg attttttgtt ttagtttttt aagtagttgg 2820
gattatgggc gtttattatt atgtttcgtt attttttttt gtattttttt agtagagatg 2880
gggttttgtt atgttggtta ggttggtttc gaatttttga tttttggtga tttgtttgtt 2940
ttggtttttt aaagtgttgg gattataggt atgagttatt gcgtttagtt ttttttgttg 3000
tgtttttgat ttttggttgt agtttaatgg gtcgattgtt ggatagcggt tttgagtttt 3060
gtttgaattg gtgttgtttc gatatttttt gattttagtt aatgattttg tttgtcgagg 3120
gtagataggt ttttgtaatt ttatgggtgg taggggtggt gatgagagga gaggtagttt 3180
tatttgtata gattttggtg tatggttttg ttttttgtat ttttttaata gaggtttgtt 3240
tagttttttt tgtaagtgtg gggagggggt ggtgtaggat tatgaggtaa ttgtgagaag 3300
aggggtttta gtagaattag ggtttaatgg ttttgaagag atggttgggg atagttggat 3360
ttattacgtt tatttttaaa tggaggaaac gattttttag ttatatagta tttgagttag 3420
aatgttatta tggtgttgtt ttataggatg atagttattt ggtttgtgag ggtttttatt 3480
ttagggatag ttattttatt ttgttttttt agagtagtaa gtaataattt tatgttagga 3540
ggttaattgg tacgttaagt gttagtttta atcgattgat agtagttgtt tggttttgaa 3600
aggtagttgg gatcgattta ttatgttgtt agtatataga tggatttagc ggtggtttta 3660
gtagtgagtt tttgttttgg gttattttat tttttttgtt ttggttaagg aatgattgga 3720
tgaatatatt ggatttttaa tatgggtgga taagataaga gtgtttggtt atattttttt 3780
attatttata agtatggttg tgggtagttt ggttttttag gcggttttgg agaatgtaat 3840
gagtcgagga attggttatt tttaggtgta tttagggtag gaaaggatga tagtatgcgt 3900
gagttagggt tattggttaa gaagttattt taggattttt ttttagaaaa gtttattggg 3960
tagtattttt gttggttttt ttttatatta taaggttacg tagagttggc ggagggttat 4020
ggttttattt atgttaggtg tttttaattt ggtaaggaaa tgtaatttac gtgaatttta 4080
ataggtagtg aagtatcgtt tttttttgat tttaggtagg gtgaagaaaa tgggatagta 4140
gtacggggtg cgggtataaa cgtataattt tgttttttta gacgtagagt tgtggggttg 4200
tgagaatgtt aggaggaggt aagaaagggc ggttttatgg ggggtttgta gggtgggata 4260
agtttaagag gtttttatat ttaggtttgg tgggggaggt gagtttttgg tttatcgagg 4320
gggttttttt ttgttttcgg aaatattgta gtttttattt ttatcgtttt ttcgttgcgg 4380
ggatttaggg gcgtgaggat gagagagttt ttaggtttta gggttagacg attgtgttta 4440
agtaagtgag aatttttttg aggttgtttt ttaattgtaa aatggggata gtagtagaat 4500
tttttttcgc ggtttgcgtg aagaatataa ttcgatgtcg ataggaggga gcggcgcgta 4560
gcgcgtagcg agtagtaggc gttgaagaag gatatttgtg aattgggagt ggtggcggag 4620
gttacgcggt tagagttcgg ggaaggggcg tcggttttgt tagtttttgt tcggggttgg 4680
atggtcgggg gatgttttcg taagtcggtt gggagggagc ggtttcgcgt attttgttat 4740
cgtcgtcgta gaggttcggg taggtgcggg gtcgcggttt tttcgcgagg gggtcggtta 4800
ttcgtcggga ttgatatttc ggaggtttaa tggtaagtcg ttattttcgc gtattcgttt 4860
aatcggcgtc ggttgtcgtg tcgcgtcggg tttttcgatt aatgggaaaa tttgttgtta 4920
gatggggcgg ggcggagatt cgcgtcgtcg gttcggttcg ttttgcgtac gggtcgcgtg 4980
agggcgggag ggttggttcg gggtttcggg ttgcgcgttg ggtttggagg gagggggcgg 5040
ttttcgtatc ggttcgagtt gcggtcgcgt ggattgcgat tcgcgtcgcg tcgtatcgcg 5100
tcgcgttttg ggaacgtcgt ttttcgcgcg ttaacggatt cggggaagtt tttttggggt 5160
tcgaggtcgc gttgcggggt cgtttacgtt gcgttttagg taagtttgag ttagtgggcg 5220
gggtgtggga ttcggggttg gggtttcggg tcggagtcgg gattgggggc ggggttgtag 5280
atatgggacg tatttcgggt agcggttcgg atagggtttt atttttggag tcgagattcg 5340
ggcgagggtt tgggtcggac gtcggagtta attttcgttt tattcgcgtt ttgttcgcgc 5400
gttttgcggc gttcgagatt tcgggtcggc gggggcgggt ttttttgtgc gtggttttgg 5460
ggttttattt tattcgttcg ggcgttttgt attgagtatt ttagtcgagc gcggttcgat 5520
ttcgtagtcg tgttcgtttc gcgcgacgtt tggcgtttag ttgttagggt tttgtagtcg 5580
tcgaaatttt ttaatagttc gaggggcggt aggtcgggaa tttttttggg gtagcgcgtc 5640
gtcgggaagt ttaggggtat cgacggttcg gaggtgtttt cgcgtcgcgt tgtttttgtc 5700
gtttcggggg tgagagaggg cgcgttattc ggattttcgg tttgggtttt tttttttacg 5760
tcgttttgcg ttttttcggt gtcggtgcgg agatatgtta tttttatttt gagggagatt 5820
gttcggtttg ttggggagtc gttttgggta ggtggtttga gagttttagt ttattttaat 5880
ttttaaataa ttaaatattg gttgtttata atattttatc gttttttagt tatgtgtaat 5940
gtaatatatt taaaatggaa ttaaaggatt ttagtttttt ttttttatat ttaggtatat 6000
ttagaaataa ggaaaagaaa ggtgtttcgt gggtattttt tagagtaggt ttttaatgtt 6060
gttgtttaat aattttaggt gagtatttag gttgttgtgg gggaaatttt ttttatggta 6120
tgggttaggg gggtgtagtg ggagttttta tttttttcga ttttagtttt tttggggtag 6180
ttgtttttat ttttttttat tttttaatta ttgttttatt tgtttttatt ttgggatttg 6240
ggcggtgttt agaggtttgg tttaaataag gtaggtgtta tttattttgt atttatttta 6300

ttggagttgg gatggtgttg aatgtagggg ggtagttagc gtttttttgt tcggtgtgtt 6360
atgttagtgg tcggttagtt tttaagtagg tgatttaggt tttagattag gcgatagtgt 6420
gagtcgtagc gtgtttggga aggtattttt tttagttttg ttttatttta ttcgtaatgt 6480
ttgtttttag ttgttttttg tttttatttt atttttattt tggggtttat tagcgtttag 6540
tagaggattg ggtatagaat attcgttaag ggatttaagt tttagttgag agggtagttg 6600
agtatagtta agttagtttt tgtttagttt ttggttgagt tgttttgtta ggttttttag 6660
tataaatata aggatttttt gattaaggtt ttttttggga gaataagttt tgatttttaa 6720
ttttgttaaa tttgagtttt tttatttatt atatttgggt tgtttggttt ttatgtttgt 6780
tttggtattt tttttagagg tggttttgta gttttttttt agtttcgggg ttttttgtat 6840
ttttgtggtt ttgtgagggt agggtagtat tttttttttt gtttgtgtta tatagggatt 6900
tagggtgagt tttttgtgtt tttttatttt aggagtttgg tttgggggag tatgaggttg 6960
ttttgagtaa ggatagaagg tgtaggagta gtaggatttt cggtttttgg gtggagtagg 7020
gagttttgta tacggatgtt aggtattatt ttttgtttag tttatttgtc gtgtttttcg 7080
tttggttttg gtagagggag tgatagggta gagttgggat agggagttag ggtttatttt 7140
gtagataggg ttaggaatgg agggtggggg tttagggata gtattgtaga gtattagttg 7200
ggttttgggg gttttgggga tttttttgat ggtggtttag ttatggggag gtgtttgttt 7260
ttttgttagt agttttaatt aagattagaa tgaggtcggg cgtagtggtt tatgtttgta 7320
attttaatat tttgggaggt taaggtaggt ggattatttg aggttaggag tttgatatta 7380
gtttggttaa tatggtgaaa ttttgttttt attaaaaata taaaaattag tcgggtatgg 7440
tggtatgcgt ttgtagtttt agttatttaa gaggttgagg taggagaatc gtttgaatcg 7500
gggaggtgag ggttgtagtg agttaagatt atattattgt attttagttt gggtgataga 7560
gcgagatttt atttttaaaa aataaaaata aataaaataa ttaggatggg ttggttacgg 7620
tggttcgtat ttgtaatttt agtattttgg gaggtcgagg taggtagata gattatttga 7680
ggttaggagt ttgagattag tttggttaat atggtgaaat tttgttttta ttaaaaatat 7740
aaaataaatt agttggattt ggtggtatat gtttgtaatt ttagttgttg gggagattga 7800
ggtaggaggg ttgtttgaat ttgggaggta gaggttgtag tgagttaaga ttgtgttatc 7860
gtattttagt ttgggtgata gggtgagatt tcgttttaaa aaaagaaaaa aaaatattta 7920
taaaaaaata aatagaaaaa tttaagatga agttttgggt tattttgagg ggatttggga 7980
ttagggattt tttagtaggt ttttttggtt tagggttttt atattttggt ttgttttagg 8040
tcgggaagat gatttagagt tggtgggtgt ttggggttat agagtttttt ttattttata 8100
attagttatg tataggtttt ttttattgtt tttgttgatt gaggggttgt cgtttttggt 8160
agagtaacgg ggagttgagt atggttatta gagggtagtt gggatcgtta tagtaggtaa 8220
gatcggtttt atttagtttt ttgcggtaga ttaggatttt tttttagata tttggtttgg 8280
atgatcggag ttcggttttt aagtcgttta ttgagggcgt ggagttggaa aggagttaag 8340
taaggttttt cggtattttt aatatgtttg gtgtaggtag aaggaagttt gaaggttagt 8400
tggtgggtga ttatatatag gtttagtagg gatagtgaag gattagtttt gtgtttgcgt 8460
agttttaaaa gtgtagtgtt ttttaagtgt gtgtttaggg gttttttaag tttttttagg 8520
tgtggtttgt gttttaatgt ttatagtaga agtgggcgtg tttttattat gtgtttaggg 8580
tggtagtagg tttaggagaa tattcggttt tgttgtagtg gatagtattg ttattgagta 8640
gtatggattt tttttgttta ttggttacgg ggtattttat ttatttgttt gtattggttg 8700
ttgttagggt atgtttttag ttatttatag ttagtgttat ttcgcgttta tagagtagga 8760
agcgaggaga tttggggagg gggtggtttg ttgaggttat attgtaggga gtgtagagcg 8820
agtttatttg ggttttttat tgtttattta gtttaggttg tattttttat tttagttttg 8880
ggtttagtgt gtatttatgg gagttgtttt gtgtcgttag ttatttaaag gattgtagtt 8940
tgttaatttg gttgtgtggt ttgggttagg tttatgtttt agtgtgagat tttttagttt 9000
tttggttgga ttatggatat cgtgttagaa ttatagggtt tattagggta ttagtggggt 9060
gtagttaggg gttgatagag ttttatggtt agttatcggg tttggatagt ttggtgggga 9120
gatttagtgt gggggtttta tggtatgggt aggtttaggt tatttttatt gggaaaaggg 9180
tttaataaag ggcgtggtta ttttttcgtt ttgaggcggt tggtattggg tttttggtta 9240
ggtaggtgat tttagagttt gtagagtatt taggggggtt ttttaaggtg agtatttttt 9300
tattttttta tatgatttta ttattagttt tgtttttata ttattgtttt gtttgtcgtt 9360
tttttgttcg tttattgttt agagtttttt taggttgttt atagatgttt tgttttgttt 9420
ttgtttgtac gatgttgatg tgtttagtgt atttttagga aggatattta tagttttggt 9480
tgtttttatt atttagtttt ttgtggtgtt gttatttttt gtttttattt atttttgggg 9540
ggaagttttt tttaatgttt tatttggatt tttttttagg gtttgttatt ggttttatta 9600
tatggttgta ggtttttggt ttggttggat tttatgaggt aggaggttta tgaggaagga 9660
tttatgtttt tttttggtta tattttttta attgggtggg ggggggggat tttgggaggt 9720
ttggaggtag gaagatttga gttaaatttt ggtttagttt ttgtttggtt gggatttagt 9780
ttttttatgt gtaaaatggg gtatatagtt aatttgagat atttggtaga gttagtgaga 9840
tgacggttta tagttagtgt gtttcggttt tgtggtatat ggtgatttcg ggggagaggt 9900
tggtgtttat tagggataaa aaaaaaaagt tatatggttt tttgtggtat aaaatttatg 9960
tgaatattta agttaaaatt agaggttttt tagaaatgtt atatttagag taaaagttgg 10020
tttttaagtt ttagattttg gagtaggagt agtatttttt ttttttcggg gcgtagtgtt 10080
ggtggatggg tttttaatcg ggtttgttta ttttaggtgt atatatatat ttatatttat 10140
atgtacgtat atgtatatgt atatgtgttt ggtttggttt ttagtttttt tttgttttag 10200
gttttgtatt ttattttttta tgatttattt ttgaggcgta gttagtcgtt tatttttatt 10260

tggatttttt tttatgatga agaggttgtc gtgagacggg gaaatatatc gttggggtat 10320
tagaatggat tatgattata aaggattatt gggcggggtt ttagaataat ttagaaaata 10380
tcgtagaggc gggggagaga gtttttatta tcgatgtttt ttagagatag ttttttattc 10440
gaggtttagt ttttttttgt ttgtttgtag tgttttgttt tttattttta tgatatgaat 10500
attttggtaa tattttcggg tttagatata aggagaggga atgtgttttc ggggttatta 10560
gttgagatag gaagtcgtag ggtttttttt tgaggaaggt tgaaagttgt gtgagtatag 10620
gtattttttt gttttttgtt ttagtttata agttttagtt ttttaggttt gagtgttagg 10680
ttttttattt tagataaatg gttatttata tttggttata gttttagggt taggaatttg 10740
tttgtttttt cgtttttttt gtattggaga ttgttggggg tagttgtttg tttgggaggg 10800
tttaggattt atgttggttt tagggaattt taggagaggt acgtggtttc gtttataagg 10860
cgggagcgga ttttagggag gtggagtaga tgttttatgg ggttgtgagt atttgcgtgg 10920
ggtaatagga ttttgagttt ggttattttg gggtgggaat attgttgtgt tgtgtgtgat 10980
ggggaagacg ttattattag tttatatttt tgaaggatta cgtttttgaa ggatggttaa 11040
tagtattaga tatgttgata aggaatcgtg gtagtttagt gttatttata gttttggata 11100
tgtgggggta agaaggtata tatgagtgtg tggttatgag ttaggagtat tacgggatga 11160
tttttagatt aggtatgata aggttttgtt tagaggacgg tttgggtttg tgtattttta 11220
ttcggtaggg atttatttag ttggattgat tttatttatg ttttttggtt tttttgtagg 11280

<210> 22
<211> 11280
<212> DNA
<213> Artificial Sequence

<220>
<223> chemically treated genomic DNA (Homo sapiens)

<400> 22
   tttgtaggga aattagaggg tatgagtgag gttaatttag ttgggtaggt ttttgtcgga 60
   taaaggtata taagtttaga tcgtttttta agtaggattt tgttatgttt gatttgaagg 120
   ttatttcgtg gtgtttttga tttataatta tatatttatg tgtatttttt tgtttttata 180
   tgtttaggat tgtaggtgat attgggttgt tacggttttt tgttagtatg tttaatgttg 240
   ttgattattt tttagaaacg tgatttttta gagatgtgga ttgataatga cgtttttttt 300
   attatatata gtatagtagt atttttattt tagggtagtt aggtttaggg ttttgttgtt 360
   ttacgtaggt gtttatagtt ttataagata tttgttttat ttttttagag ttcgttttcg 420
   ttttgtgggc gaggttacgt gttttttttg aggttttttg aggttaatat agattttggg 480
   tttttttagg taggtagttg tttttaatag tttttagtat agggagggcg ggaagtaggg 540
   taggtttttg gttttggagt tgtggttagg tgtgaataat tatttgtttg ggatggggga 600
   tttggtattt aagtttgggg agttgaggtt tatgggttgg agtaggagat aagggggtgt 660
   ttgtatttat atagttttta gtttttttta ggggaggatt ttgcggtttt ttgttttagt 720
   taatggtttc ggggatatat tttttttttt tatatttagg ttcggggata ttgttagagt 780
   gtttatgtta taaaaatgaa aaataggata ttgtagatag atagggaagg gttgggtttc 840
   ggatgggagg ttgtttttgg gggatatcgg taatgagagt tttttttttc gtttttacgg 900
   tgttttttag attgttttga ggtttcgttt aatagttttt tgtggttatg gtttattttg 960
   gtgttttaac ggtgtgtttt ttcgttttac gatagttttt ttattatggg aaggggttta 1020
   agtgagggtg ggcgattggt tacgttttag ggtgggatta tggggggtgg gatgtagggt 1080
   ttgaggtagg gaagaattgg ggattaagtt aggtatatgt gtatatgtat gtgcgtgtat 1140
   gtgaatatga gtgtgtgtgt gtatttgaga tgggtaggtt cggttgggag tttatttatt 1200
   agtattgcgt ttcgaggaag aggagatgtt gtttttattt taaggtttga ggtttaggaa 1260
   ttagtttttg ttttgagtgt gatattttta ggaaattttt ggttttagtt taagtgttta 1320
   tataggtttt gtattataga gaattatgtg attttttttt tttatttttg ataagtatta 1380
   gttttttttt cggagttatt atgtgttata gaatcgagat atattggttg tgagtcgtta 1440
   ttttattgat tttattaggt gttttaggtt ggttatatat tttattttat atatggagaa 1500
   attgggtttt agttagataa gggttgggtt aggatttaat ttaggttttt ttgtttttag 1560
   gtttttaaaa gttttttttt tttatttagt tggggaagtg tgattaaagg agaatatggg 1620
   ttttttttta tgggtttttt attttatggg gtttagttag gttaagaatt tgtaattata 1680
   tggtagagtt aatagtagat tttgggaagg ggtttaagtg aggtattgga gagggttttt 1740
   tttaggagat gggtgggagt agaggatagt agtattatag gaggttgagt ggtaggagta 1800
   gttagagttg tgggtatttt ttttaagggt gtattaggta tattagtatc gtataggtag 1860
   gggtagagta gagtatttgt ggataattta agggagtttt gggtagtgga cggatagagg 1920
   gacgatagat aggatagtga tgtgaaggta gaattagtga taggattata tgggaaggtg 1980
   gggaggtgtt tattttgggg agtttttttg agtgttttgt aagttttggg gttatttgtt 2040
   tggttaagga tttagtatta gtcgttttaa agcgggggaa tgattacgtt ttttattggg 2100
   tttttttttt agtgaggatg gtttgggttt atttatgtta tgggattttt atattgggtt 2160
   tttttattaa attgtttaga ttcgatggtt ggttatgggg ttttgttagt ttttggttat 2220
   attttattga tgttttagta agttttgtgg ttttggtacg gtatttatgg tttaattaga 2280
   gggttgagag gttttatatt ggggtataag tttggtttag gttatatagt tagattggta 2340
   agttatagtt ttttggatag ttgacggtat agggtagttt ttatgggtat atattgggtt 2400
   tagaattggg atggaggatg tagtttgggt tgggtaggta atgaggggtt taggtgagtt 2460
   cgttttgtat tttttgtagt gtggttttag taggttattt tttttttaag ttttttcgtt 2520
   ttttgtttta tgagcgcgag gtgatattga ttgtaggtag ttgagaatat gttttggtaa 2580
   tagttaatgt aggtaggtga atggagtgtt tcgtggttag tggataggga aggtttatgt 2640
   tgtttaatga taatgttgtt tattatagta aaatcgagtg tttttttagg tttgttgtta 2700
   ttttgggtat atagtgagaa tacgtttatt tttgttgtgg atattgaggt atagattata 2760
   tttgggaagg tttgggaggt ttttgggtat atatttgaag gatattgtat ttttggggtt 2820
   acgtaggtat agggttagtt ttttattgtt tttgttgagt ttgtgtgtgg ttatttatta 2880
   gttggttttt aggttttttt ttgtttgtat taagtatgtt gggggtatcg aggggttttg 2940
   tttggttttt ttttaatttt acgtttttaa taggcggttt ggaggtcgag tttcggttat 3000
   ttaagttagg tgtttgggga agggttttgg tttgtcgtag gaagttgggt ggagtcggtt 3060
   ttgtttgttg tggcggtttt agttgttttt tggtggttat gtttagtttt tcgttgtttt 3120
   gttagaggcg gtagtttttt aattagtagg ggtagtggga agggtttgtg tataattggt 3180
   tatggagtgg ggggaatttt gtggttttag gtatttatta gttttgggtt attttttcga 3240
   tttgaaatag gttaagatat gaaggttttg agttaggaaa atttattaag ggatttttga 3300
   ttttaagttt ttttagagtg atttaagatt ttattttggg tttttttgtt tgtttttttg 3360
   tgggtatttt tttttttttt tttgagacgg agttttattt tgttatttag gttggagtgc 3420
   gatggtataa ttttggttta ttgtaatttt tgttttttag gtttaggtaa tttttttgtt 3480
   ttagtttttt tagtagttgg gattataggt atatgttatt aggtttagtt aatttgtttt 3540
   gtatttttag tagaaatagg gttttattat gttggttagg ttggttttaa atttttgatt 3600
   ttaggtgatt tgtttgtttg tttcggtttt ttaaagtgtt gggattatag gtacgagtta 3660
   tcgtgattag tttattttgg ttgttttgtt tgtttttgtt ttttggagat agagtttcgt 3720
   tttattattt aggttggagt gtagtggtgt gattttggtt tattgtaatt tttatttttt 3780
   cggtttaagc gatttttttg ttttagtttt ttgagtagtt gggattatag gcgtatgtta 3840
   ttatgttcga ttaatttttg tatttttagt agagataggg ttttattatg ttggttaggt 3900
   tagtgttaaa tttttgattt taggtgattt atttgttttg gttttttaaa gtgttgggat 3960
   tataggtatg agttattgcg ttcggtttta ttttggtttt aattgaagtt gttgataaaa 4020
   gaatagatat ttttttatga ttaagttatt attagggaag tttttaggat ttttaaggtt 4080
   tagttggtgt tttgtagtgt tgtttttgga tttttatttt ttatttttgg ttttgtttat 4140
   agaatgggtt ttgatttttt attttagttt tattttgtta ttttttttgt tagggttagg 4200
   cggggagtac gatagatggg ttggatagag ggtggtgttt ggtattcgtg tgtagagttt 4260
   tttgttttat ttagagatcg ggatttttgt tgtttttgta ttttttgttt ttatttagga 4320
   tagttttatg tttttttagg ttagattttt ggggtgaagg agtataggga atttattttg 4380
   aatttttgtg tagtataaat agaaggaggg gtgttatttt atttttatag agttatagag 4440
   atgtagaggg tttcgggatt ggggggagat tatagggtta tttttggagg gagtgttaga 4500
   gtaaatatgg gagttaagta gtttagatgt ggtgggtggg gagatttaaa tttagtagga 4560
   ttgagggtta gaatttgttt ttttaggagg aattttggtt agaaggtttt tgtgtttatg 4620
   ttgaaaagtt tgatagggta gtttagttaa gagttgggta ggggttggtt tggttgtgtt 4680
   taattatttt tttagttggg atttgagttt tttagcgaat gttttgtgtt tagttttttg 4740
   ttgggcgttg gtggatttta gaatgggggt agggtgggga taggggatag ttgggagtag 4800
   atattgcgga taggatgggg taagattggg ggaaatgttt ttttagatac gttgcggttt 4860
   atattgtcgt ttgatttaag gtttggatta tttgtttagg agttgatcgg ttattggtat 4920
   ggtatatcgg gtaagagagc gttgattgtt tttttgtatt tagtattatt ttagttttaa 4980
   tagagtgagt ataagatagg taatatttgt tttgtttgga ttagattttt aaatatcgtt 5040
   tagattttag agtaaaggta gataaggtag tagttaagaa gtaggaagaa gtaaaggtag 5100
   ttattttaga gaagttaagg tcgggagagg tggaggtttt tattgtattt ttttggttta 5160
   tattataggg gaggtttttt ttatagtagt ttgggtattt atttgaggtt attagatagt 5220
   agtattaaga gtttatttta aaaaatattt acggggtatt tttttttttt ttatttttaa 5280
   atatgtttgg atgtagggaa agggagttga agttttttag ttttatttta agtatgttat 5340
   attatatata gttaagaaac ggtgaaatat tgtaagtagt taatatttaa ttatttaaaa 5400
   attaaaatag gttggagttt ttaggttatt tgtttagggc gattttttag taaatcgagt 5460
   agtttttttt agagtggaga tgatatgttt tcgtatcggt atcgaggggg cgtagggcgg 5520
   cgtggggagg gggatttagg tcgggggttc gggtggcgcg ttttttttta ttttcgaggc 5580
   ggtaggagta gcgcggcgcg aggatatttt cgggtcgtcg gtgtttttga gtttttcggc 5640
   ggcgcgttgt tttaggaaag ttttcggttt gtcgtttttc gggttgttgg aaagtttcgg 5700
   cggttgtagg gttttggtag ttaggcgtta agcgtcgcgc ggggcgggta cggttgcggg 5760
   gtcgggtcgc gttcggttgg agtgtttagt gtaagacgtt cggacgggta gggtagggtt 5820
   ttaaggttac gtataaagag attcgttttc gtcggttcgg ggtttcggac gtcgtagagc 5880
   gcgcggataa gacgcgggtg gggcggagat tggtttcgac gttcggttta gattttcgtt 5940
   cggatttcga ttttagggat aggattttgt tcggatcgtt gttcggaatg cgttttatat 6000
   ttgtagtttc gtttttagtt tcggtttcga ttcgaggttt tagtttcggg ttttatattt 6060
   cgtttattgg tttaggttta tttggagcgt agcgtgggcg gtttcgtagc gcggtttcgg 6120
   attttagaag ggttttttcg ggttcgttgg cgcgcgggga gcggcgtttt tagggcgcgg 6180
   cgcggtgcgg cgcggcgcgg gtcgtagttt acgcggtcgt aattcggatc ggtgcggggg 6240
   tcgttttttt tttttaggtt tagcgcgtaa ttcgagattt cgggttagtt ttttcgtttt 6300
   tacgcggttc gtgcgtaaag cggatcgggt cggcgacgcg aattttcgtt tcgttttatt 6360
   tgatagtaaa tttttttatt ggtcgagaga ttcggcgcgg tacggtaatc ggcgtcgatt 6420
   gggcggatgc gcggagatga cggtttgtta ttgggttttc gggatgttag tttcggcgga 6480
   tgatcggttt tttcgcggag gggtcgcggt ttcgtatttg ttcgaatttt tgcggcggcg 6540
   gtggtagggt acgcgggatc gttttttttt agtcgattta cgagaatatt tttcgattat 6600
   ttagtttcga gtagggattg gtagagtcgg cgtttttttt tcggattttg gtcgcgtagt 6660
   tttcgttatt atttttagtt tataggtatt tttttttagc gtttgttatt cgttgcgcgt 6720
   tgcgcgtcgt ttttttttgt cgatatcgaa ttgtattttt tacgtaagtc gcgagagaga 6780
   gttttgttgt tatttttatt ttatagttgg gaaatagttt tagagaggtt tttatttgtt 6840
   tgaatatagt cgtttgattt tggggtttgg gggttttttt atttttacgt ttttgggttt 6900
   tcgtagcggg gagacgatgg aggtaggagt tgtagtattt tcgagggtag gaagggattt 6960
   tttcggtaaa ttaggggttt atttttttta ttaggtttgg atgtagagat tttttgggtt 7020
   tgttttattt tgtaggtttt ttatggggtc gttttttttt attttttttt ggtattttta 7080
   tagttttata gttttgcgtt tggggaggta gagttgtgcg tttatgttcg tatttcgtat 7140
   tattgtttta ttttttttat tttatttgga gttaggaaga aacggtgttt tattgtttgt 7200
   tgagatttac gtaggttata tttttttgtt agattaagag tatttgatat gagtgggatt 7260
   atgatttttc gttagttttg cgtaattttg tggtgtaggg gggaattagt agggatgttg 7320
   tttagtgggt ttttttaggg ggaggttttg agatgatttt ttagttagtg attttggttt 7380
   acgtatgttg ttattttttt ttgttttgga tgtatttgga gatgattagt ttttcggttt 7440
   attgtatttt ttaaggtcgt ttggggaatt aaattgttta taattatgtt tatgagtggt 7500
   ggaggggtgt gattagatat ttttgtttta tttatttata ttgggagttt agtgtgttta 7560
   tttaattatt ttttggttag gataaagaaa atgaaatggt ttaaggtaag aatttattgt 7620
   tgggattatc gttgggttta tttgtgtgtt ggtagtatgg tgaatcgatt ttagttgttt 7680
   tttagagtta ggtagttatt attaatcgat tggagttggt atttgacgtg ttaattggtt 7740
   ttttggtata gggttgttgt ttgttgtttt gggagggtag aatgaagtag ttgtttttag 7800
   aataggggtt tttataaatt aggtagttgt tattttgtgg agtagtatta tggtgatatt 7860
   ttggtttagg tgttgtgtag ttgaggggtc gtttttttta tttaggagta gacgtaatga 7920
   gtttagttgt ttttagttat ttttttaagg ttattggatt ttggttttgt tggagttttt 7980
   ttttttatag ttattttata gttttgtatt attttttttt tatatttgta agagggattg 8040
   gatagatttt tgttgaaaga gtgtaaaaga tagaattata tattaaaatt tgtgtaagtg 8100
   agattatttt tttttttatt attattttta ttatttatag ggttgtagag atttgtttgt 8160
   tttcggtagg taggattatt agttgaggtt agaggatgtc ggggtagtat taatttaaat 8220
   aggatttagg atcgttgttt agtagtcggt ttattaggtt gtagttaggg gttagaaata 8280
   taataaggga ggttgggcgt agtggtttat gtttgtaatt ttagtatttt gggaggttaa 8340
   ggtaggtaaa ttattagagg ttaggagttc gagattagtt tggttaatat agtaaaattt 8400
   tatttttatt aaaagaatat aaaaaaaagt agcggggtat ggtggtagac gtttatagtt 8460
   ttagttattt gggaggttga ggtaggagat cgtttgaatt tgggaggcgg aggttgtagt 8520
   gagttgaaat tgtattatcg tacggtagtt taggagatag agcgagattt tattttaaaa 8580
   aaaaggaaaa aagaaaaaga taagaaaaat atagtaaggg agattatagg gattagtagt 8640
   ttagtgtttt ttttttttta tttttttatt tagttttatg cggatagttg tgaggttttc 8700
   ggagaaagtt atagagagaa aatgttttta gttgggtgtt aagaattttt taagagtggt 8760
   agttgttgaa attaaaagag tggttataga aggtagtgag ttttttattt gagtttgtgt 8820
   gtaattagag gttggatgtt agtgagtttg ttgtagtttt aagattttag ggtttaggtg 8880
   tttattttag atttatttta tttgtcgtta tttgtttttt ggtatcgtat gttatttaga 8940
   ttggagtgtt agtggtgata aagaagatta gagtgtttgt tttggttttg tttagttggg 9000
   aaaagtatgg ttgggggtgg ggatggttta aggttataag gtatgataga ataggatgta 9060
   aattattatt tgatatgtta ttgtattttt ttttgtgggt aaaggaaaat tttggttaat 9120
   ttatgtttag ttttgaggtg tatggtttgg tgttttgggg aagtttattt tttaggtgtt 9180
   gttatgaatt ttagggtata gtttattatt tttaagtttt agattttatt tgtttattat 9240
   tattattttt tggttttata gattgtatag gtttggttgt ttagagggaa attattggta 9300
   ttagttttat atgattttat tgttggatgg tgggagagat aattatgttt ggttgggttt 9360
   gcggttcgtt ggttatgagt ttttgggtat ttggagtttt tgtaaaaggt tttgttgggt 9420
   tttaggggaa atatagataa gatagtaaaa gtgtggagta taacgggttc gtatgtaggt 9480
   ttaggttaga tttttgtcgt tgttgattat tagttaggtt ttttttattt ttgagtttta 9540
   gttttttgta gggtattagg gtatttaggt ttttaatata ggttgaagtt aatgtggttt 9600
   tttttgtttg ttattttttt taattttaat tgaatgatat aattgtttta agttttagtg 9660
   tttatattta taaagtggga tgaaagaatt gttgatttag atattgggtt tagagtgttt 9720
   tatatattat tattattttt aatgtttttg ttatttatgt tatagttttt tgaatgttta 9780
   ttttgaggta gggtttgagt ttttgggttt tgatttttta tttttttgtt atataggttt 9840
   aggtatagtt tagaggttat aggagtagcg gttttagtta atgtttttgt tttgttggga 9900
   gggttttagt tagtgtttat ttgtttattt tgtataagtt aagttgggtt agtttttcgt 9960
   cgttggggtt tggatttgga tgtgagttta aaggttatat tggggggtgg aagtggggga 10020
   ggtggtgggt gtgtagggta tagtgtttaa atgtttagtt tagggtttgg gggtatatat 10080
   tgggaatggt agggatatta gggaggaaag agtcgatttg gaggttataa agtgaaggaa 10140
   gggatgatta agaggaagat ggttaggttt agaggtgggg ttgagtgaat aggatgggga 10200
   gattaggacg tagtttgagg gggttagttc ggaagtgagt atttttgtat ttttttcgag 10260
   agaggtcgta gggtgagtag gtgatagatg gtgtttatga gggtatggat ttaggggcgg 10320
   ttgcgatttt ttatggcgtt tttgtaggta gggagtcgcg gagttttcga aggtcgttgg 10380
   attaggaagc gtttagacgt tcgcgtagtt cgaagttttg gcgtagtttc gggggcgggg 10440
   cgttaggagg tggtagtttt cgagcgatag cgcgtttagt taacgggacg tcggtatgcg 10500
   gcgcgtcgtt tcgtttttcg ttgcgcggtt taatggtgaa cgcgtcggtt tcgggttggg 10560
   cggtattggg ttggttgcgg tggcgcgcgg gcgcggtatt cggaagtcgg cggcggtggc 10620
   ggaggcggtg agtgcgcggt ttcggggttg gtcgatttcg ttagtggttc ggtcggtttg 10680
   ggttcggggg tttcgggttt tgggttttgg gtgcgcggat cgggttaggt tgtttgaagg 10740
   tgggtgggtt ggtcgtattt ttttattttg ttttagtttt ttttttttga ggtatggtat 10800
   agaattaggc ggttgggtta ggggtaatta cgtcgatttc ggtcgacgaa aatgtttcgc 10860
   gcgttattgg aaggtgtttt tgataatttt cgttggtacg ggatgcgagg gattttggta 10920
   ggtgggatta gattcgttgc ggggaggttg agatcggtag ggttgggagt tgcgtggttt 10980
   ttgtagggtg tgtgggttgt tcggttttgg ttagtattag ggatagtttt ggcgttcggt 11040
   tattttgttt tttattcgcg gtttgttgcg ggttagtagg gtgatagtta atgtgggtgt 11100
   gagtagatag taagtgttgg gttatttttg cgtttggatg cggttttttt ggggaggttt 11160
   gggtagttag atttttattt tttaatttta gagcgttaga ggtaggggtt ggtagggttg 11220
   tatttagggt agtttttgtt ttatcgttgg gttttgtagt agtttgggtt ttgttcgttt 11280

<210> 23
<211> 6932
<212> DNA
<213> Artificial Sequence

<220>
<223> chemically treated genomic DNA (Homo sapiens)

<400> 23

ttggatagag taggttcggg tagtatagtt ttttgtggag tataggggcg aggaggtttt 60
aggaggttat tgggattagg gaaggttttt aggagatata acgttcgagt tggagaggag 120
gtagtgagtg atggtacgtg agggtgttgg gtttgaattt cgagggttcg gatatgattt 180
agttgggttg gatgttgggt aaggtttttt tatttttagg cggtgtatag atagaagtag 240
taaattgaga atttaggaga ttagggtaga ggtaggggta gtgtttaagg taagatgggg 300
atttggttgg agaagcggtt gggttgacgt atagtagagg gtaggggaga ggaagggtag 360
tgtttatatt gtcgatttgg tgttttttgt tgcggagggg attttttttt agaggaaagg 420
aggtcgagtt taatttgtaa taagttgagt ttgaggagtt gtaggaggat gggggtcggg 480
ttaggtgtta cgtggatata ttagtttttt ggagtagttg ttagtagatc gaggtttatg 540
ggttagattt agtttttggt ttgtttttgt attgtttaaa agttaggaat tttttttaaa 600
tttttaagtg gttaaaaaat taaaatttat atttattagt tattattaaa aaattattat 660
tattttagtt aggttgggta atatagttag attttatttt tataaaaaat aaaaatttgg 720
tcgggtatgg tggtacgtat ttgtagtttt agttattcgg gaggttgaaa ttagagaatg 780
gttggaattc gggaggtaga agtcgtagtg agttaagatt gcgttattgt attttagttt 840
gggagataga gcgagatttc gttttaaaaa taaataaaaa taaaacgaaa cgaaataaat 900
ttagaaagta ttaagtgtgg gcggggatgt ggagatatta gaatttcgtg cgtagtagga 960
atgtattgtg ggtagttatt gcggaaaata atatggtggt tttttaaaag gttaaataga 1020
ggtttgtcgt atgatttggt aatttcgttt tttcgtttat ttttaaaaga attgaaagta 1080
ggattttaaa gagatatttg tatttttatg tttatagtag cgttattgac ggtagtttaa 1140
gggtggaaat tattttagta tttattgacg gaggaacgga ttaatatcgt ggtttattta 1200
tatagtggaa tattatttat ttataaaaag agaggaaatt tttatgtagg ttacgatatg 1260
gatgaagttc gagggtatta gtattagtgt aagaagtcgg tttataaaaa gatagatgtt 1320
gtaggatttt atttatagga ggtttttaga gtagttaaat ttatagatat agagtggatg 1380
gtgggtgtta ggggttgggg gaagggttat gggaagttgt tgtttaatgg ggatagattt 1440
tcggtttggg aaaatgagaa atttttggag atggatggtg gtgatgattg tatagttata 1500
tgaatatatt gaattttacg gaatcgtgta tttataaatg gttaagaagg tttattttat 1560
gttatgtgta ttgaattata gtttgaaaat tcggagtatt ttgtaatagt gagtgtgatt 1620
tggattttat gttttagtga ttaggaagta agttttgtta tttttttttt gtttaagttt 1680
gtatttgcgt tttaaggtag acgattcgtg gtagagatat gtgatcgtta aggtcgaaaa 1740
tatttattac gtgttttttt gtagaaaaag tttgttttat ttttgtttaa gagtttagga 1800
atgaagtcgg agataggtag agtttgggag aggtgttagg gtgtttaggt aaaggtttta 1860
tgaggtcgta tgtttatgtt cgaaggtttt ttaattgttt tattaagata tagtttatat 1920
tttataaagt gtatatttta atggtttttt tgtgtgttgt atagagttgt gaagttaata 1980
ggataggtta tgttagaacg tttttatttt ttgggaaaga aattacgtat ttttttatag 2040
ttattttat tttttgttg ttattttta gttttggta atcgtgaatt tttttttgt 2100
ttttgtgaat ttatcgattt cgtatatttt atagattgag agttttacgt tacgtggttt 2160
tcggtgtttg gtttcgttta cgcggtttcg tgttttcgag gtttattagt ttcgtttttt 2220

tttagggttg agtaattttt tattattttg gattatgttt tgtttattta tttatttgtt 2280
gatggatatg tggttatttt gaattttgtt gttggaaata tttatgtggg tatcgtgttt 2340
attaaggttg tatttttaga acggattgag aggttaagag ttttagagaa gacggtttag 2400
atagtgtgga gatttattag tttaggagat agtatgtttt cgaattaagg ggggtgttgt 2460
agtgttaaat gttgtatagt cggaggtatt taggaatgaa ggaggggtcg aggtagggag 2520
agatataggt ttttagggaa ttatttttaa gttaggtttt gggggtagac ggggttttta 2580
tagcggtggg aggattagat tgggaagagg atgaagatag agagaggtgt tggtggtggg 2640
gggggattgg tttggatatg gtatagtaga cgtaggtggg tgtaggattg tgtttgaggg 2700
tagggtagta agaagttatg tttgggtagt gagaggtttt agtttttaga taggaagttt 2760
tggggtgatt tgaagagggt ttgtaggttt tgtttcggag attttattag ttgtggaggg 2820
gtttggacga agatcgggta gagagtaatt tagtcgttgt atcgttcgtt tttgttgatt 2880
ttcggggtgt atgtagttat atttagagta gtagagggtt tatttattaa gttatagaat 2940
tttgagaaat aatatatcgt tgtttagatc gttaaaattt tggtggtttt ttatatagta 3000
atagatgatc gagggttttt tagttcggga ggtggaaatt tagtagggat ttttaaggtt 3060
tagtttgtag ggttttagga tcgtttttag atttggtttt tgtagttttg ataaggggaa 3120
ggagggaggt agtagaagga gggtagaata atttatgtta ggttgtgatt tgttaagtga 3180
ttatttggga agaatgggtt tttagattag ggatagggag tagaggtaag ttcgtatttg 3240
ttttggttgt agaattcgga tttagattta gggtttcgat ttttgtttgg atcgttttat 3300
tgggcggagg agtgattgtg gatatattta gggttttttt taagtcggtt tttttatttg 3360
ttaaatagag atcgtagatt attagttttt aggtaggtgt tattttttcg gtttttttta 3420
aggtaggagg gttaggcgta ttcgagatat aggtgtgttg ggggtttagg tgggttagtt 3480
agtagtattt tgtagggtaa tgggagtagg tgggtatttc gaggttggta gtaaatattg 3540
gttatttgtt tttaggtttt taggaggggt tttgggtttt atttttttcg gtcggaatag 3600
gaaagtagtt ttaggtagtt gggtttataa aaattttcgt tttttgaggt tttagaggta 3660
gtggtttagg agtatttggt tattttcggg aaaaatcggt ttggtaaagg ttttttcgag 3720
ggtacgcgtt tttcggatag tgaggtagga tttaaatttt ttcgttaata ttatattttt 3780
cgtatttttg tagtgtttgt atttttaggt tttattattt tttcgtattt tttagggaga 3840
agttttcgac gttttatttt ttttggaagg gtgttgtttt tagagatttt taggttaatg 3900
gtttaatttt agtgttttta ggggagaggg gggtgtagaa aaatagtttg ggttataaaa 3960
gaggtgcgag ggttgtgaga tttcggaggt atcgacggga agcgagacgg agaataggag 4020
ggtaggacgg gttggaggtg ggggatattg tagatggagg gagttacggt gggggagggc 4080
gtggatttga tcgttttggt ataaggcggt cgggtgtaga tttttaggtt tttcggttta 4140
aggtgtcgtt tagagttttt aggtcggggg cgtacggaaa ttataggtag ggtgcgcgtg 4200
gagggacggg gaaagcgggg cgggttgggg aaggcgtttc gggaatttga atttttttatt 4260
tcgttttagt ttcgattatt ttttaagttt tatttcgttt taggtaaggc gtagtttatt 4320
tttattttta gtagattaac gtataggtgg gggcgcgttc gggatatagt tgcgttaggg 4380
gatagcgcgt ttagtttagt cgcgggggcg aggagtaggg cggggtttag taggaattta 4440
gttttgttag cgatgttttt cgtgagttac gcgttacgcg tacgcgtttt tttaatgggg 4500
tcgggcgtgg agtcgcgttt tgcgcgattg gttaaacggg tggtttacga ttggttgaga 4560
ttttggtttt cgttttttcg gttttaggag ggtggggcgt gggtgtgggt tgcgcggcgc 4620
gtgttgtttt cggggatttt gcgcgttttt cgaatagtcg tgttgtcgtt agggtcgcgt 4680
tttttttttt atagcgcgcg ttgcgcgtgc gaaggtttgg cggtttttgg gattggcggg 4740
gttgcgcgcg gggttagggt gggggtacgg gaaggtttaa tttaggattt gcgtattttg 4800
ttttgggggc gtattaagta tttgtcggga gtagggggcg tatcgggaat tcgtagattt 4860
cgttagttgg gcgtattggg gatttgtgga ttgcgttcgg gggatgggtt agggggatat 4920
gcgtacgttt tgggttttat agaatgtgat cgcgcgaggg ggagggcgaa gcgtggcggg 4980
agggcgaggc gaaggaagga gggcgtgaga aaggcgacgg cggcggcgcg gaggagggtt 5040
atttatatat ttaaaaatta gtcgtttgcg tcgcgtttgc ggagatttgg gagagttcgg 5100
tcgtacgcgc gggatacgag cgttttacgt tttttggcgc gtacggtttg ttattattag 5160
gttttttatt ttcgggtttt agacgattta ggacgcgtgt tttggggagt tgtttggcgg 5220
cgtcgtgtta gaagtttttt tggggcgtta tagttttttt cgtcgttttc ggtttttttg 5280
tttgtatttt tttgcggcgc gtcgggattt ggagcgggcg ggtggatgta ggcgcgatgg 5340
acggcggtat attgtttagg ttcgcgtttt ttgcgttttt cgtttttgtc ggttgcgttg 5400
ttcggcggag attcgcgttt tcggaattgt tgcgttgtag tcggcggcgg cgatcggtta 5460
tcgtagagat cggaggcggc gtagcggtcg tagcgcggcg taatgagcgc gagcgtaatc 5520
gcgtgaagtt ggtgaatttg ggtttttagg cgttgcggta gtacgtgtcg tacggcggcg 5580
ttagtaagaa gttgagtaag gtggagacgt tgcgtttagt cgtggagtat attcgcgcgt 5640
tgtagcgttt gttggtcgag tacgacgtcg tgcgtaacgc gttggcggga gggttgaggt 5700
cgtaggtcgt gcggtcgttt gcgtttcgcg ggtcgttagg gattatttcg gtcgtcgttt 5760
cgttttttcg cgttttttcg ttttcgggtc gcgggggtag ttcggagttc ggtttttcgc 5820
gtttcgttta ttcgtcggac gatagcggtt gcgaaggcgc gttgagtttt gcggagcgcg 5880
agttattcga tttttttagt tggttagggg gttattgagc gttttcgatt tatgaggtaa 5940
tagtcgggag gtagggagga gggagggtcg ggggtcgggg tggagggacg gggtgggtag 6000
gttcggcggg tcgcgttttt aggagttcgc ggagtcgagc gttaggttcg agcgatggtt 6060
tcgatttcgt ttatttttta ttttttttaa agttttttaa gttcgtgtaa gatcggcgtt 6120
tgtttgttcg ggattgtaaa attttttttc gcggtttagt cgtcgacgag ggaggggtag 6180

acgaggggag gggagcggtc gtcgggtcgt tgaggttttt agtgttggcg gattttgggg 6240
tagattgggg tgttggaggc ggggtgattt tgtattgtaa atcgcgtttt cgggtcgggg 6300
cggtagaaat gagtcggcgg gcgcggagtt ttgatttatc gcggtttcga gcgttcgttt 6360
cgttttcgtc gtgttttaga tcgagtcgcg gtatttacgg atttaagatt ttaaaattaa 6420
tcgagtaaac gaaattgtcg agttcgtttg ggggaggtgc gggtagggtc ggttcgggcg 6480
gggtttgttt cgggttcgcg ttcgcgttga cgcgcgtttg gtttttattt tttttttcgt 6540
agttttagtt tcggaagtcg agcgagcggt cggcgcgttt atcgtcgggg agttcgttag 6600
gtggatcggt tcgcgtttcg tttttagcga gtcggggatt tatttattat ttttcgtatc 6660
gtcgacgtcg tttcgttcgt tcggtttagt ttgattaatg tcgcggtgga aacgggtttg 6720
gagttggttt tataagggtt ggcggttttt ttcgacgtcg ttttttttta tagtttttcg 6780
attgtagtgg ggcggggggt attaatattt ggagattttt tcggagggga gaggattttt 6840
taagggtata gagaatttat tttttatata ttaatttgag ttgttggagg gatattgttg 6900
gtaaacggag atttattttt gtataaagaa tt 6932

<210> 24
<211> 6932
<212> DNA
<213> Artificial Sequence

<220>
<223> chemically treated genomic DNA (Homo sapiens)

<400> 24

ggttttttgt ataaaaatag gttttcgttt gttagtagtg tttttttagt agtttaagtt 60
aatgtgtaga aaatggattt tttgtgtttt tagaaaattt tttttttttc ggaaaaattt 120
ttaagtgttg gtgtttttcg ttttattgta gtcgagaagt tgtggggagg ggcggcgtcg 180
gaggaagtcg ttagttttta tggggttagt tttaagttcg tttttatcgc ggtattggtt 240
aggttgggtc ggacgaacga ggcggcgtcg gcggtgcggg gggtggtggg tgggttttcg 300
gttcgttggg ggcggagcgc gggtcggttt atttggcggg tttttcggcg atgagcgcgt 360
cggtcgttcg ttcggttttc ggggttgagg ttgcgggggg aaggtgggga attaaacgcg 420
cgttaacgcg ggcgcgggtt cggggtagat ttcgttcggg tcggttttgt tcgtattttt 480
tttaagcgaa ttcggtagtt tcgtttgttc ggttggtttt ggagttttga gttcgtgggt 540
gtcgcgattc ggtttgagat acggcggggg cggggcgggc gttcggagtc gcggtgagtt 600
agggtttcgc gttcgtcgat ttatttttgt cgtttcggtt cgggagcgcg atttgtaatg 660
taaagttatt tcgtttttag tattttaatt tgttttagga ttcgttagta ttagagattt 720
taacggttcg acggtcgttt tttttttttc gtttattttt ttttcgtcgg cggttgagtc 780
gcgaggggaa gttttgtaat ttcggataaa taaacgtcgg ttttgtacgg gtttgaaaaa 840
ttttggggga aatgaagagt gagcgaaatc gaagttatcg ttcgggtttg gcgttcggtt 900
tcgcgggttt ttgggggcgc gattcgtcgg gtttgtttat ttcgtttttt tatttcggtt 960
ttcggttttt tttttttttt gtttttcggt tgttatttta taggtcgagg gcgtttagta 1020
gttttttaat tagttggaga agtcgagtag ttcgcgtttc gtaggattta gcgcgttttc 1080
gtagtcgttg tcgttcgacg agtaggcgga acgcggggag tcgggtttcg agttgttttc 1140
gcggttcggg gacgaagaag cgcgggaggg cgaggcggcg atcggggtgg tttttggcgg 1200
ttcgcggggc gtagacggtc gtacggtttg cggttttagt tttttcgtta gcgcgttgcg 1260
tacggcgtcg tgttcggtta gtaggcgttg tagcgcgcgg atgtatttta cggttgagcg 1320
tagcgttttt attttgttta gttttttgtt ggcgtcgtcg tgcggtacgt gttgtcgtag 1380
cgtttggaag tttaagttta ttagttttac gcggttgcgt tcgcgtttat tgcgtcgcgt 1440
tacggtcgtt gcgtcgtttt cggtttttgc ggtggtcggt cgtcgtcgtc ggttgtagcg 1500
taatagtttc ggggacgcgg gttttcgtcg ggtagcgtag tcgataggga cggggggcgt 1560
agggggcgcg gatttgggta gtgtgtcgtc gtttatcgcg tttgtattta ttcgttcgtt 1620
ttaggtttcg gcgcgtcgta ggaaggtgta ggtagaggaa tcggaggcga cggggaaaat 1680
tgtggcgttt taagggggtt tttggtacgg cgtcgttagg taatttttta gggtacgcgt 1740
tttaggtcgt ttggagttcg gggataggag gtttagtggt ggtaggtcgt acgcgttagg 1800
gagcgtggga cgttcgtgtt tcgcgcgtgc ggtcggattt ttttaggttt tcgtaggcgc 1860
ggcgtaggcg gttggttttt aaatgtatag ataatttttt ttcgcgtcgt cgtcgtcgtt 1920
ttttttacgt tttttttttt tcgtttcgtt ttttcgttac gtttcgtttt ttttttcgcg 1980
cgattatatt ttgtaaggtt taaagcgtgc gtatgttttt ttagtttatt tttcggacgt 2040
agtttataga tttttagtgc gtttaattgg cgaaatttgc gagttttcgg tgcgtttttt 2100
gttttcggta ggtgtttagt gcgtttttaa agtaaggtac gtaggttttg ggttgagttt 2160
tttcgtattt ttattttaat ttcgcgcgta gtttcgttag ttttaagagt cgttagattt 2220
tcgtacgcgt agcgcgcgtt gtgggaggga aggcgcggtt ttggcgataa tacggttgtt 2280
cgggaggcgc gtaagatttt cgggggtagt acgcgtcgcg tagtttatat ttacgtttta 2340
tttttttggg gtcgaggagg cgggggttag ggttttagtt aatcgtgggt tattcgtttg 2400
gttaatcgcg tagggcgcgg ttttacgttc ggttttattg aggaagcgcg tacgcgtggc 2460
gcgtggttta cggggagtat cgttaataaa gttgggtttt tgttgggttt cgttttgttt 2520

ttcgttttcg cgattgggtt gggcgcgttg ttttttagcg tagttatgtt tcgagcgcgt 2580
ttttatttgt gcgttaattt attgggaatg ggggtggatt gcgttttatt tggggcgggg 2640
tggggtttaa ggagtggtcg agattgaggc ggggtgggag gtttaggttt tcggggcgtt 2700
ttttttaatt cgtttcgttt ttttcgtttt tttacgcgta ttttgtttgt ggttttcgtg 2760
cgttttcggt ttgagggttt tgggcggtat tttaattcgg agggtttgga ggtttgtatt 2820
cgatcgtttt gtgttaggac ggttaggttt acgttttttt ttatcgtggt tttttttatt 2880
tgtagtattt tttattttta gttcgttttg tttttttgtt tttcgtttcg tttttcgtcg 2940
gtgttttcgg gattttatag ttttcgtatt ttttttgtga tttaggttgt ttttttgtat 3000
tttttttttt tttgagggta ttgagattgg gttattggtt tgaaggtttt tgggagtagt 3060
atttttttag gggaggtggg acgtcgagaa ttttttttta agagatgcgg ggaaatggtg 3120
gggtttgaga gtgtaaatat tgtagaaatg cgaaaaatgt agtgttaacg gaagagttta 3180
ggttttgttt tattgttcgg gaaacgcgtg ttttcggggg agtttttgtt aagtcggttt 3240
ttttcgaagg tgattagatg tttttgggtt attgtttttg agattttagg gaacggagat 3300
ttttgtggat ttagttgttt ggagttgttt ttttgtttcg gtcggaggag gtgaggttta 3360
agattttttt tgggagtttg ggggtagata gttagtgttt attgttagtt tcggggtgtt 3420
tatttgtttt tattattttg taggatgttg ttggttggtt tatttgggtt tttagtatat 3480
ttgtgtttcg agtacgtttg gtttttttgt tttgggaggg gtcggaagag tagtatttgt 3540
ttgggagttg gtggtttgcg gtttttattt ggtagatgag gaagtcgatt tggagagaat 3600
tttggatgtg tttatagtta ttttttcgtt tagtggagcg atttaggtag aaatcggggt 3660
tttgagtttg aattcgggtt ttgtaattag ggtagatgcg ggtttgtttt tgttttttgt 3720
ttttggtttg agagtttatt ttttttagat ggttatttgg taaattatag tttggtatgg 3780
attgttttgt tttttttttg ttgttttttt tttttttttt gttaaggttg taagattagg 3840
atttaggaac gattttggag ttttgtaaat taggttttgg aaatttttgt tggattttta 3900
tttttcgggt tgggagtttt tcggttattt gttgttgtgt aaggagttat taggatttta 3960
gcggtttgaa taacgatgta ttatttttta ggattttgtg atttgatggg tgggtttttt 4020
gttgttttgg gtgtggttgt atatatttcg ggggttaata gggacgagcg gtatagcggt 4080
tgggttgttt tttattcggt tttcgtttaa gtttttttat agttggtaag attttcggag 4140
taggatttgt aagttttttt tagattattt tagaattttt tgtttaaaaa ttgaagtttt 4200
ttattgttta ggtatggttt tttgttattt tgtttttagg tatagttttg tatttatttg 4260
cgtttgttgt gttatgttta ggttagtttt tttttattat taatattttt ttttattttt 4320
attttttttt taatttggtt tttttatcgt tgtggaaatt tcgtttgttt ttaaagttta 4380
gtttaaaaat aattttttag ggatttgtgt ttttttttgt ttcggttttt tttttatttt 4440
tgggtgtttt cggttgtgta gtatttgata ttgtagtatt ttttttaatt cggaagtatg 4500
ttgttttttg gattggtgag tttttatatt atttgagtcg ttttttttgg aatttttggt 4560
tttttagttc gttttgagaa tatagttttg gtaagtacgg tgtttatatg aatgttttta 4620
gtagtaggat ttaaaatagt tatatgttta ttaatagatg agtggataaa taaaatatgg 4680
tttagaataa tggaagatta tttagtttta aaaagagacg aagttggtga atttcgagaa 4740
tacgaggtcg cgtgaacgaa gttagatatc gaggattacg tagcgtgaga tttttagttt 4800
atgaaatgtg cggagtcgat aaatttatag agatagaaag gagatttacg gttgttaggg 4860
gttggggagt gataatagag ggatgggggt gattgtgaaa gggtacgtgg tttttttttt 4920
agaggataag aacgttttaa tatggtttgt tttgttggtt ttatagtttt gtataatata 4980
taaaaaaatt attgaaatgt atattttgtg gaatgtgaat tgtattttga taaagtagtt 5040
agaagatttt cgaatataag tatgcggttt tatggggttt ttgtttgggt attttggtat 5100
tttttttagg ttttatttat tttcgatttt atttttgagt ttttgaatag gggtaaggta 5160
aatttttttt gtaaaggaat acgtggtaag tattttcggt tttgacggtt atatgttttt 5220
gttacgagtc gtttgttttg gggcgtaaat gtaggtttgg gtagggaaga aataataaaa 5280
tttgtttttt ggttattgaa atatgaagtt taggttatat ttattgttat aaaatatttc 5340
gaatttttag attgtggttt aatatatatg atataaaatg gattttttta attatttgta 5400
agtgtacggt ttcgtggaat ttagtatatt tatgtggttg tgtaattatt attattattt 5460
atttttaaaa gtttttattt tttttaaatc gaaagtttgt ttttattaaa tagtagtttt 5520
ttatgatttt ttttttagtt tttggtattt attatttatt ttgtgtttgt agatttgatt 5580
gttttggaga ttttttgtaa gtggaatttt atagtatttg tttttttgtg gatcggtttt 5640
ttatattgat gttgatgttt tcgagtttta tttatgtcgt agtttgtata aggatttttt 5700
ttttttttat gggtgaataa tattttattg tatgggtaga ttacggtgtt gattcgtttt 5760
ttcgttagtg gatgttgggg tggtttttat ttttgggtta tcgttagtga cgttattgtg 5820
gatatggggg tataaatatt tttttgagat tttgttttta gtttttttgg ggatagacgg 5880
agaagcggag ttgttaggtt atacggtaaa tttttgttta attttttgag ggattattat 5940
gttgtttttc gtagtggttg tttatagtat atttttgttg cgtacgaggt tttgatgttt 6000
ttatattttc gtttatattt ggtgtttttt gggtttgttt cgtttcgttt tgtttttgtt 6060
tgtttttgag acggagtttc gttttgtttt ttaggttgga gtgtagtggc gtaattttgg 6120
tttattgcga tttttgtttt tcgagtttta gttatttttt agttttagtt tttcgagtag 6180
ttgagattat agatacgtgt tattatgttc ggttaaattt ttattttttg tagagataga 6240
gtttgattat gttgtttagt ttggttgagg tgataatagt tttttgatga tagttaatgg 6300
gtatggattt taatttttta attatttaag aatttaaaga aaatttttag tttttgggta 6360
atataaaagt aggttagggg ttggatttgg tttatgggtt tcggtttgtt gatagttgtt 6420
ttagaggatt ggtatgttta cgtgatattt ggttcgattt ttattttttt gtagtttttt 6480

aaatttaatt tgttgtaggt tgaattcggt tttttttttt ttaaggaaag atttttttcg 6540
tagtagagaa tattaggtcg gtagtgtggg tattgttttt tttttttttt gtttttgtt 6600
gtacgttagt ttagtcgttt ttttagttag gttttattt tgttttggat attgttttg 6660
tttttgtttt ggttttttgg gtttttagtt tgttgttttt gtttgtgtat cgtttggaag 6720
tgggggggtt ttatttagta tttagtttag ttagattatg ttcgggtttt cggggtttag 6780
gtttagtatt tttacgtgtt attatttatt gttttttttt tagttcggac gttgtatttt 6840
ttggaagttt tttttgattt tagtggtttt ttgaagtttt ttcgtttttg tgttttatag 6900
ggagttgtgt tgttcgggtt tgttttgttt aa 6932

<210> 25
<211> 6212
<212> DNA
<213> Artificial Sequence

<220>
<223> chemically treated genomic DNA (Homo sapiens)

<400> 25

gagagtatat agataagata gaggggagga ggtgggtatt ttttatattt tttttgttt 60
gtcgttgttg agattgtagt atttattgta atgtaaatgt attttgaagg tggggaggaa 120
tgtttaattt attatgttcg tgtgttattt tggtttgtgt ttttttttgt ttgtagtaag 180
attttgatga taattttgtt ttttatttgt ttatttagta ttttgttttt ttttcgttaa 240
gttgttttat ttttttaatg attatttttt tattattgag gttttggtga agttttttgt 300
agttgtttta tttttttta acgatagtaa taggaaatga tttttagta tcgatatttt 360
aatattaatt tagggtagag atttttgttt tttttttgtt atagattagg aaattgagaa 420
ttagggttaa ttttgattat atttagaggt tttttgttt tttttttttt taataaggat 480
tttttatggt ggttttagtt ttatttgtat aaaggtattg agagggaata aaaaatataa 540
agttgagaat tttgagagag tttatttatt ttgtttgttg gttagattta aatgagagtt 600
aaaaaaaaaa aaaaaaattt gtatgtttga gtattatttt ggatgaattt agaaggtatg 660
gggtagagtt tgatagggtt tttgtgtatt tattgggtat tcgttagagg taagggagag 720
gagaggattg atagagtgtt gtaaaagtat agattattta ttgagataaa ggatttggtt 780
tttttgttat gagtattaaa aaaatttaag tttttttaag tttgtatttt tgattaaatt 840
ttatataaaa tattggaagg aggttgggtg cggtggttta tgtttgtaat tttagtattg 900
ggaagttaag gcgggtggat tatttgaggt taggagttcg agattagttt ggttaatatg 960
gtgaaatttc gtttttacga aaaagataaa aataagttgg gcgtggtggt aggcgtttat 1020
aattttagtt attcgggagg ttgaggtagg agaataattt aaattcggga ggcggaggtt 1080
atagtgagtt gagatcgtgt tattgtattt tagtttgggt gatagagtga gattttattt 1140
taaaaaaata aaaattggaa gaagagttta aaaaagataa gattttaaag agttttaagt 1200
tatttaagtt gagtgtaatt gttatttaag gaaggtaaat gagtttatta ttttttttaa 1260
agagtatttt ttttaattgt tggataaaat tataattttg ttattttata aggaagaatt 1320
ttttaagaag tttttagaat tagaagtaat gtgaattttt agcgttggtt ttggtgggtt 1380
tgttgattat gattgggtaa gtcgtttttt ttgttgttat tttttttatt ataaagtgtg 1440
gaatataggt aattgttttg agatttttgg atagaagagg ataatatttt gtatttgttt 1500
ttttttttaa attttggggg aaagatgagt aatttttttt attattttgt ttttttgttt 1560
agtaattttt attttgttt gaagtaatag tattttttat tttttattt agagattttt 1620
gtgtgtgtgt tattaaggtt agtaaatttt atacgtagtt taatatttaa aaaatgtatt 1680
tattatttta aatttaataa attttagagt ttattttggt tttttttttgt tgttttttt 1740
aaaaaatgag ttgaagatga tagtattttt ttttatatgt ttggttatga tttttaaagt 1800
tttatttaaa taaatgttga agtttaagtt taaagaagcg ttgtagaggt ttacggtttt 1860
ttgggtttcg gttatttgtt tatattttat atttgttgat tgtgtttttt gtattttatt 1920
taagttgaga gtttaaatag ttttgaaggg gaattagttt taggatggaa ggatttagga 1980
gaggtttcga ggtgggaggg ttttgtaaat atagattatt gcgagtgttt agagtttttt 2040
gttatgatat ttttttggga ttgatttggt tgagaacgtg ttttgttaga ggatttgtta 2100
gaattttgtt tttttgtttg aaatttaagg ttaaggagaa tgataggaga tttaggatag 2160
agttgatttt tgtattaggt tgggaggttg tagttttttt agatgttatt tattgtaagt 2220
ggttagaata ttagagaggt gggttttatg gttaaatgta tagtaggtgt ttatttttat 2280
atttggatta ttttgtagtt tttaaatttt tggtttttga ggttaagttt ataatttgtt 2340
ttttagttat ttgtttgttc gtagtggtgg tggatgtgtt agttggtaga tttggaatta 2400
gttattagtt tttttgtatt gttttggtta gttttatata agtaggggta gtttagtttt 2460
gaggtttaga gatttgttgt tttttttttt tttgagggag gaaataaaat tgcggaatat 2520
aatgtttttt tatagtatgg gaagaagaaa ataaatattt tttttttaat agttgtgatt 2580
tgtgtgtgtc gtatagaata tttttttgtg ggagtggtat tgggttttta tttttagtta 2640
ggggaatttt tgaagtgttt gagttaggtt tttataaaag aatggatggg ataggttgtt 2700
atgggagtta tttttttatg atttttttag tgttaaaatt ggggtagtgt tattttaata 2760
tttaattatt tgtatagtta tttgttagga ggtattgttt atagtaaaag gagaatacgt 2820

tttgtgggtt tttagttttg tttttggaag atatggttaa tggtttaggt atttttattg 2880
tttttgtatg gaaaggttag aaaatttttg tatttttttt ttttttaaag atagggtttt 2940
gttttgttgt ttaggttgag ttgtagtgtt gtgattatag tttattgtag tttggaattt 3000
ttgggtttaa gtaatttttt tatttcggtt tgagtaggtg gggttatagg ttggagttat 3060
tgtttttggc gttaattttt gtatatttta atatttacga ttttttaagg agaaagaaaa 3120
aatatatttt tattttagta tttagtatgt tttgtgtata ttagttttaa agttgttggt 3180
tttattttat taaataataa tgaagagttt tttgaggtag taggaggatg tgatggagag 3240
tggtagaagt tagggttagg agatttgagt tttatgaata gtggtagtaa tagtaattat 3300
tattgggcgt ttatatgtat taggtaggta ttgtgttaag tgttttatat gtatgatttt 3360
attttatgag gtaaggatat ttattagtag ttttatttta taaatgagga aattgaggtt 3420
tagagagtat aagttattta gttaaaatgt tttagttagg atttttttgg atgcgttttt 3480
taaaatttgt tttttaattt ttattttata ttttttttat tgggagtttt gtgattttga 3540
gtaagaagtt ttattttatt gggtttcggt ttattattgg aaaggatttg tgggcgaggt 3600
tgaatgagcg tagtagatgg tttgtggtta tttagagtag tgtttttttg gttgtttgat 3660
attatagcgt ttatatttgt gtggtttagt gaaatggagg cggaaattta aagtttttat 3720
ttttataggg tttgattgga aggggttgaa ggatgatggg gaggtttttt gtatttttaa 3780
aatagattat ttttttttaa cgtataattt ttagtaagtg gtcgtgggtt tttaagcgtt 3840
tttttttttt gggttatggg cgtgattgtt gaaaagcggg ttaggtcggg gagtttagaa 3900
ttcggagtga attcgaatta ttttagggag acggtagcga agaaaatttt gtttttagga 3960
gaacgtaatt ttcggatatt agaagttttt ttatgatgtt attttgtggt agttttgtat 4020
ttgagtcgga tgaaaattgt aaaaatttag gttgggcgat aggacggcgc gttttttcgg 4080
ttttttttta gagtttagga ggagttatgt agatttagtc ggtttttggg ggtaggtgga 4140
taggttcggc gcgttttttc gaggtttttt taatttttcg ggggtagtag cgtcgtgtcg 4200
gttatgaatg gggtttttgg aaaatttagg tttggaatag gtaatttttc ggttttcgta 4260
ggttataaat tattattcgt aatcgaagga cgcgtaggtt gggagcggcg gcggggttat 4320
tagtgcggat ttttattgag ggcggcggga ttcgatcgta agggttggtt tttgaggtta 4380
ttaaggggtg cgttgggtta aggtcgtttg ggggtaggag ttagcgcgat cgtggcgggt 4440
tagtaggcgg ggttggttga ggattagtag cgattcgaga ttttaatttt ttttacgcgg 4500
atttcgggat ttttttttta gtagtcgttt atttagggtt ttgagttaat aagagttcga 4560
gatatatatg atttatcggg tttgggttaa ttattttgac gcgcgttttt cgttagtatt 4620
tcgcgcgata atttggcgtt ttcggttttt ataataaaaa tttttcgtcg gaaaacgatt 4680
ttttttcgtt tattgcgttg atatcggaag cgaggcgtgt ttgggagatt atttcgcgtt 4740
tcggcggcga aggaaagaac ggattttgat tatagaagtt tagtaaagta gtatattttt 4800
tttttttcgt gaggttataa gaataaattt tttttttcgt tatagttacg ttttgggtat 4860
aaacggttgg ggcgttaaag ggagggaggg aagggagcgg gcgggaggag acggttacgt 4920
ggtcgcggcg gaaggatgcg tttgtgttgc gtttttatag agacgaagtt tataaagggt 4980
cggcgggcgg ttacggtagt tatttttatt ttgttcgttt atagatgtag ttttttttgc 5040
gcgtgtgcgt tttttttttt tttcgttttt agggtttacg gttattatgg cgtattaggg 5100
gtagtagtgt ttgcggtagt attggttttt gtagcggcgg tagtagtatt aggttttgta 5160
gcggtaattt ttagcggttt aagttatggc gtgagtatcg gggcgggtcg tttagttgtg 5220
tttttggggt cggcgcgggt tttggattgg tggggtgcgg tttggggtta gggcggtgtc 5280
gttaaggggg aagcgattta acgagcgttc gggacgcgtg gtttttgttt gggtgttttc 5340
gagacgttcg cgtgtttggg atcgggaaag cgtagtcggg tgttcggatt gtttttttag 5400
gagttttata gttttcggat ttcgagtttc gtaagggttt taggggtttt ggttgttgtt 5460
ttacgaaacg tggtaggaat taagatggcg gcggtagggc ggcggcgcgg gcgtgagtta 5520
agggcgggcg gtgggcgggg cgcggtcgtt ttggtcgtat ttggacgtgg ggacggagcg 5580
tttttttttt ggcggtcggt ggaagaattt tttggttttc gtgagcgttt attttgtgga 5640
atttgagttg taagtaggga ggggtaaata taattgtttt gttttcgatt ttttagatgg 5700
tcgatttaga gaagtttcgt tttataagtg gaaggatgaa atttttagaa tagttaattt 5760
ttaatgggag ttggtttttg atttttattt aggtttttta cggtatttag tagtagcgtt 5820
gttgtaatcg ataaagatat tttcgaatta agtatatttt tcgattttag taaagtatcg 5880
taatatgatc gaaatgagtt ttttgagtag cgaggtgttg gtgggggatt tgatgttttt 5940
tttcgattag tcgggtttgg gggttgaaga aagtttaggt tttttagatg attatttgga 6000
ggtggttaag tattttaaat tttatgggtt ttttagcgat aaggttaagg cgggtttttt 6060
cgaatggttg gttgtggatg ggttggttag tttttttaat aatagtaagg gtgagtgggt 6120
tattattata tcgttttggt gggatttagg gttaggggtt ttttattttt gtatttgatt 6180
tatttggttt tattatttaa atgttttgta ga 6212

<210> 26
<211> 6212
<212> DNA
<213> Artificial Sequence

<220>
<223> chemically treated genomic DNA (Homo sapiens)

<400> 26

tttgtaaaat atttgagtga tggggttaag tgagttagat ataaaggtag gaggttttta 60
attttagatt ttattaggac gatgtggtgg tggtttattt atttttgttg ttgttggagg 120
gattgattaa tttatttata gttagttatt cggaggagtt cgttttagtt ttgtcgttgg 180
agaatttatg aggtttgaag tgtttggtta tttttaggta attatttaag agatttaggt 240
tttttttagt ttttaaattc gattggtcga agggggatat taagtttttt attaatattt 300
cgttgtttag gaagtttatt tcggttatgt tgcggtgttt tgttggaatc gaggaatgtg 360
tttaattcga aggtgttttt gtcggttata gtaacgttgt tgttgaatgt cgtgagaagt 420
ttgaatgaga attagaagtt aatttttatt agaggttagt tgttttgaga attttatttt 480
tttatttatg aggcgggatt tttttagatc ggttatttag agaatcggga atagggtagt 540
tgtatttgtt tttttttgtt tgtaatttag gttttataaa atggacgttt acggagatta 600
ggggattttt ttatcggtcg ttaagaggaa agcgtttcgt ttttacgttt aaatacggtt 660
agggcggtcg cgtttcgttt atcgttcgtt tttgatttac gttcgcgtcg tcgttttgtc 720
gtcgttattt tggtttttgt tacgtttcgt ggggtaatag ttaagatttt tgggattttt 780
gcggggttcg gggttcgagg gttgtagggt ttttggggaa gtagttcggg tattcgatta 840
cgttttttcg attttaggta cgcgagcgtt tcggggatat ttaagtaaag attacgcgtt 900
tcgggcgttc gttaaatcgt tttttttttg gcggtatcgt tttggtttta ggtcgtattt 960
tattaattta aaattcgcgt cggttttagg agtatagttg gacgattcgt ttcggtattt 1020
acgttatggt ttaagtcgtt gggggttgtc gttgtagagt ttggtgttgt tgtcgtcgtt 1080
gtaaaggtta atgttgtcgt aggtattgtt gtttttaata cgttatggtg gtcgtggatt 1140
ttgagggcgg ggaggaggga aaacgtatac gcgtagagaa aattatattt gtgggcgggt 1200
aaagtagaaa tggttgtcgt ggtcgttcgt cggtttttta tagatttcgt ttttatgggg 1260
acgtagtata gacgtatttt ttcgtcgcga ttacgtgatc gttttttttc gttcgttttt 1320
tttttttttt ttttttgacg ttttaatcgt ttatgtttag ggcgtagttg tgacgagaaa 1380
aggagtttgt ttttatggtt ttacgaaagg agagaggtgt attattttat taggttttta 1440
tgattagagt tcgttttttt tttcgtcgtc ggagcgcgga gtgatttttt agatacgttt 1500
cgttttcggt gttagcgtag tgggcgggga aaggtcgttt ttcggcgaag agtttttatt 1560
gtgaaggtcg aggacgttag gttatcgcgc gaggtgttgg cgaagagcgc gcgttaaggt 1620
gattggttta ggttcggtga attatatata tttcgagttt ttattggttt agggttttag 1680
atgggcggtt gttgggagag ggatttcggg gttcgcgtgg gaagggttgg ggtttcgggt 1740
cgttgttagt ttttagttag tttcgtttgt tggttcgtta cgatcgcgtt ggtttttgtt 1800
tttaggcggt tttagtttag cgtatttttt agtggtttta gggattagtt tttgcggtcg 1860
gatttcgtcg tttttagtgg aggttcgtat tggtggtttc gtcgtcgttt ttagtttgcg 1920
cgtttttcgg ttgcgggtgg tgatttgtgg tttgcggaaa tcggggggtt atttgtttta 1980
agtttgagtt ttttagaggt tttatttata gtcgatacgg cgttgttgtt ttcgggaggt 2040
tgaaggggtt tcgagagggc gcgtcgggtt tgtttattta tttttaaagg tcggttgagt 2100
ttgtatggtt ttttttaaat tttggaaagg agtcgggaag acgcgtcgtt ttgtcgttta 2160
atttgggttt ttataatttt tattcggttt aagtgtaagg ttgttataga atagtattat 2220
agaggaattt ttggtgttcg aagattgcgt ttttttaaga ataagatttt tttcgttgtc 2280
gtttttttgg ggtagttcgg atttatttcg gattttgaat ttttcggttt aattcgtttt 2340
ttagtagtta cgtttatggt ttagggagga gaggcgtttg ggaatttacg gttatttatt 2400
gggggttgtg cgttgggaag gggtagtttg ttttaagaat ataagaaatt tttttattat 2460
tttttagttt tttttagtta ggttttgtga aggtggaaat tttgagtttt cgtttttatt 2520
ttattgggtt atataagtat gagcgttgtg atattaaata attagggaag tattgtttta 2580
aataattata ggttatttgt tgcgtttatt taatttcgtt tataaatttt ttttaatggt 2640
aaatcgaggt ttaatgggat gaagtttttt gtttaaagtt ataaggtttt tagtggaggg 2700
agtatggggt aggagttaga agatagattt tggggagcgt atttaaaaga attttggttg 2760
ggatattttg gttaaatgat ttatattttt tgagttttag tttttttatt tgtaaaatga 2820
gattattgat gagtattttt gttttatgaa gtgagattat atatgtaaag tatttagtat 2880
agtgtttgtt tggtatatgt aagcgtttaa taatggttgt tgttgttgtt attgtttata 2940
gaatttaggt tttttgattt tgatttttgt tattttttat tatatttttt tgttgtttta 3000
gaggattttt tattattatt taatgagatg aaattaataa ttttaaaatt ggtatatata 3060
aggtatattg gatgttggaa taaaagtatg tttttttttt ttttttgggg aatcgtgagt 3120
gttgagatat atagaagttg gcgttaggag tagtggtttt agtttgtagt tttatttatt 3180
taagtcgagg tgggaggatt gtttgagttt aggaatttta agttgtagtg agttgtgatt 3240
ataatattgt aatttagttt gggtaataga gtaagatttt gtttttaaaa aaaaaaaaaa 3300
tataggagtt ttttagtttt tttatgtaaa ggtagtaaga atatttgagt tattagttat 3360
gttttttaaa ggtagaatta gggatttata ggacgtgttt tttttttgtt atagatagta 3420
ttttttgata gatgattgtg taaatggttg agtattaaag taatattgtt ttaattttga 3480
tattgaaaag gttataaaag aatgattttt atggtagttt gttttattta tttttttata 3540
gagatttagt ttaaatattt taaaggtttt tttagttgaa aatggagatt tagtattatt 3600
tttataggaa aatgttttat acgatatata taagttatag ttgttggaaa ggagatgttt 3660
attttttttt ttttatgtta tggaaggata ttgtatttcg tagttttatt ttttttttta 3720
aggagaaaaa ggatagtagg tttttgggtt ttagggttaa gttgttttta tttatataga 3780
gttaattaag atagtataga aagattggtg attgatttta aatttattag ttaatatatt 3840

tattattatt gcgggtaggt aagtgattag aaggtaagtt gtggatttgg ttttagggat 3900
taagaattta aagattataa ggtgatttaa atgtaaaggt aaatatttat tgtgtatttg 3960
attatggaat ttattttttt ggtattttgg ttatttatag taagtggtat ttaaaagggt 4020
tgtagttttt tagtttggtg taagggttag ttttgtttta agttttttat tatttttttt 4080
ggttttgagt tttagataaa agggtagagt tttaataaat tttttgatag aatacgtttt 4140
tagttaagtt agttttaagg aagtattatg gtagagagtt ttggatattc gtagtagttt 4200
gtatttatag aattttttta tttcggggtt ttttttgggt ttttttattt tgaagttgat 4260
ttttttttaa gattatttga atttttaatt tgagtggagt gtagggagta tagttagtaa 4320
atgtggaata tggataggtg gtcgggattt aggagatcgt gggtttttgt aacgtttttt 4380
taaatttgag ttttaatatt tatttaaata aaattttaaa agttataatt aagtatgtaa 4440
agaaaaatat tgttattttt agtttatttt ttaggaaggg taatagagga gaattaaaat 4500
aaattttgga atttattagg tttaagataa tgagtgtatt ttttaagtgt taggttacgt 4560
ataaagtttg ttaattttga tggtatatat ataaaaattt ttagatggag aagtagaaga 4620
tgttgttatt ttaggtaaaa gtaagagtta ttgaatagga aggtagagta gtggggaaag 4680
ttatttattt tttttaaag atttaaaaaa ggggtaggt gtagaatgtt gttttttttt 4740
atttaagaat tttaaagtaa ttgtttatgt tttatatttt atgatgagga agatggtagt 4800
aaaaagaacg gtttgtttag ttatggttag taaatttatt aggattagcg ttgagagttt 4860
atattgtttt tggttttgag gattttttaa gaggtttttt tttgtaaaat gataaagtta 4920
tggttttgtt taatagttaa aagagatgtt ttttaagaag gatgataaat ttatttgttt 4980
tttttaaatg ataattatat ttaatttaaa taatttggga ttttttaaaa ttttattttt 5040
tttaagtttt ttttttaatt tttatttttt tgaaataggg ttttattttg ttatttaggt 5100
tggagtgtag tggtacgatt ttagtttatt gtaattttcg tttttcgggt ttaagttatt 5160
tttttgtttt agtttttcga gtagttggga ttataggcgt ttgttattac gtttagttta 5220
tttttatttt tttcgtggag acggggtttt attatgttgg ttaggttggt ttcgaatttt 5280
tgattttaag tgatttattc gttttagttt tttagtgttg ggattataag tatgagttat 5340
cgtatttagt ttttttttaa tgttttatgt gaaatttggt tagagatgta agtttgggaa 5400
aatttaaatt tttttaatat ttatggtagg gaaattaaat tttttatttt aatgaataat 5460
ttatattttt gtaatatttt attaattttt tttttttttt tatttttaac ggatatttag 5520
tgggtatata ggaattttgt taagttttat tttatatttt ttagatttat ttaggatggt 5580
atttaggtat atagattttt tttttttttt ttaattttta tttgagtttg attaatagat 5640
agggtagatg agttttttta agatttttag ttttatgttt tttgtttttt tttaatattt 5700
ttatgtaaat gaaattgaaa ttattataag aaatttttgt taaggggaaa agaggtaaaa 5760
agatttttaa atatagttaa ggttaatttt agtttttaat tttttaattt gtgataaaag 5820
aggggtagga atttttattt taaattgatg ttgaggtatc gatgttaaag agttattttt 5880
tgttattatc gttgggaagg aatgagatag ttgtagagag ttttattaga attttaatga 5940
tggagaggta gttattgaaa aaataagata atttgacgga aggaaaataa aatattgaat 6000
gggtagatga gaaatagaat tattattaga gttttgttat aaatagggaa aaatataaat 6060
taagatgata tacggatatg gtagattaaa tatttttttt tatttttagg atatatttat 6120
attgtaataa atattgtaat tttagtagcg gtaaataagg aggaatgtag gaaatgttta 6180
tttttttttt tttgttttat ttgtgtgttt tt 6212

<210> 27
<211> 6906
<212> DNA
<213> Artificial Sequence

<220>
<223> chemically treated genomic DNA (Homo sapiens)

<400> 27
   aatttggttg tttggggtcg agagtttgga gttgtagatt atgggtatcg aaaggtaggt 60
   tttggggagg agcggttatt tgttatggta tttacgagtt agagtttaga gttttagatt 120
   agaaagttta agagtttttt agttttttgt tatagttttt taggttgagg gtaggtggcg 180
   tatagttttg ttatttgttt ttttttagta gattatgggt ggtggatttt tttttttttt 240
   tttttttttt tttttttttt ttgagatgga gttttgtttt gtcgtttagg tttattgtaa 300
   ttttaatttt ttgtgtttaa atgatttttt tgttttagtt tttcgagtag ttgggattat 360
   aggcgttttt tattacgttt ggttaatatt tgtagtttta gtagggatag ggttttatta 420
   tgttggttag gttggtttcg aatgtttgat tttaggtgat tcgtttgttt tggtttttcg 480
   aagtgttggg attatacggg tgagttatta tatttagttt tttttttttt tttttttatg 540
   gatatttaga tagagttata atttttgtat gttttaaagt tttgaaggtt taatgatagg 600
   ttatttcgta tttaaaaggg ttcgttattt attaagttgg agagtaagag ttttgtatag 660
   ttttgaggtt aggattttgt tttttatttt ttggattttt ttggtttata tttttatgtg 720
   tgtgtgtgtg tgtgtttttt tttttttttt ttttgaggga gtttcgtttt gttatttagt 780
   ttggcgtgta gtggtgttat tttggtttat tgtaattttt ttattttcgt tttttaggtt 840
   taagcgattt ttttgtttta gtttttcgag tagttgggat tataggtacg tgttattata 900
   ttcggttgat ttttgtattt ttagtagaga cggggtttcg ttatattagg taggttggtt 960
   tcgaattttt gattttagat gattttttag tatgggtttt ttaaaatgtt gggattatag 1020
   gtatgagtta tcgttttcgg ttttttgttt tttaaaagga agaaaagtta attattcgtt 1080
   tataatataa ggaattattt tatagtagta gtagaataaa tatataatgt tttgggtttg 1140
   tgatatattt ttttatttta acggagaaga gaaggttgtg tgtgttttat aaacgttttt 1200
   gggtaaatgt gggagaaatt taaaggtgat aatggggtga ggagttttcg atatattaaa 1260
   acggtatgta tgttttgtcg tttggagtgg gaaaaaagat atttttatcg gtttaatatg 1320
   taatatatgg tagaagaggg gattttggaa gagtaacggg tatgtagttg tttgggagag 1380
   atggttaatg tgtgtcgtta cgagggtttt atggttagta ggttaggaag ttggatttag 1440
   gtttttaggg tattgaggta tgttttttaa ttggtgttag tatttaggtt tatattggcg 1500
   gatttatatt ttatttttat atatgtataa acgtaattta tttattaatt gtagttttat 1560
   atttattcga atttagatgt acgtgtataa gcgcgtatag gtatgtatac gtttttagcg 1620
   gttgggttcg agttcgtttt atgatagcgg gttagttaat tttagggtcg gaattttttt 1680
   tataaagttt tttaagttta aatttttagg gaaatttgaa tagaggtaga aaagcgtttg 1740
   atgatagaaa aaaaaaaatt tgtttgtttg tagtttttgc gatggttata atgttattag 1800
   aaattgttgt attttagagt cggggagagc gagggtaggt agggggagga ggcgggagta 1860
   aagaggtcgg agaatttaaa agaagaaatt agaattcggg ttgagaacgt tttcggggtc 1920
   gcggggggtt ggtagggggc ggaggagagg tcggggaagg gttaacgcgg gagcggagcg 1980
   tgcggcgggg gcgggtcggg gcggggcggg gcgggcgcgg ggcgttgttt ttttaaggcg 2040
   ggcggcgcgg gtcggtcgag cgcggttgtg attggcgcgg cgggattatt ggtttcgcga 2100
   gtttggttcg ggggagtcgg ttggagtcgg ttgcgttttg ataaggtttt ggtaatttag 2160
   taatagttcg agagtcggga aataaaaata attttttaga gcgatggatt tcggggtcgt 2220
   tcggcggtcg aggcgttcgt cgaaggtttt gttgtaaaag gtgggggtgg aagttcggtt 2280
   ttagcggagt cgttttaaag aaagtttttt ttttataggg ggcgaggtcg cgtcgagtcg 2340
   cggcgtgggc gcggggggcg ggagggggtt cggttaggcg gaggtcgggt ttggaggggg 2400
   gcgcgggcgg ggcggttttg cggagcggtg gcggggttgc ggggggcggt tcggggcgcg 2460
   gtcggcgcgt attttttttt taggatcgtg cgttcggttc gcgtttttga agtgtatagt 2520
   taaatttata gcgtagtttt tgttggaagc gttcggagga tatttggtgc gtcgagtgat 2580
   tgttggaaat aggatttgcg ttgaaagtcg ggtcgggcgg gggcggaggt tcgttcggat 2640
   tttttgtata aaaggggcgg cggggcgcgt cgtttttttt tttcggtcgg tggttttcgt 2700
   tttgaattcg ggattgttgt ttttcgtatt ttttttttcg tcgtacgatt ttttatgttt 2760
   ttcgacgtcg cggtcggttc gggggggttc ggggttgtcg tcgcgagtcg agtcgggggt 2820
   cggggtcggg tttttgttag gggcgtttgc gaggtttcgt cgtcgagcgc gcgcggggat 2880
   taatggtgag tgtcgaggtc gaggcggtcg ggcggatcgc gcgttttgat gtgcggggcg 2940
   gttttttgaa gttggtaggt cggttatttc ggggcgcgtt aggggtcggc gcggagggga 3000
   gcggcggatt cggcgtttag cgtttcgggg ggtttggtcg ggtagttttg cgcgttttgc 3060
   gtcggagttt tttttttagc gcggtgatcg ggttggcggg gggtttagag aggagattta 3120
   ttcgagtggc ggtagtggcg ggggcggaga gggggtagat atttttcggg ggtcgcgtcg 3180
   ttgtcgcggt tatggatgtg agtcggcgtg tgagtgcgcg cgggagtgtt tttgggaggt 3240
   gggatgaaag ggtagcggcg tttttcgttt gttttttttc gtttttcgtt tttttttcgt 3300
   tttatttttt ttttttattt tttttttttt tttttttttt ttttttttgt cgcgggttcg 3360
   cgttaagggg tttatagcgt tggcggaggt ttagtttgcg cgtaggatcg gtttaagttc 3420
   ggggttttcg tgtttttcgc gttttttttt tcggttcggg tttttcgttc gtggtcgttt 3480
   cggttttttt tgcggagagg tagagatttc gcgcgtcgtt atttttggat taggttgttg 3540
   ttttttgtgt tttttttgcg ggcgtttttg taaacggcgg cgtcggggat cgatttttcg 3600
   ttcgggttcg gggcgttgtt ggcgtgtttc ggggtttcgg tttcgaggtg tgttcgttag 3660
   ggttttggag gcgtttagtt gtatttgggt cgggggatcg ggtcggaggg gttcggagga 3720
   tatgagagtt tggtttcggc ggttttcgtt ttttaggtaa cggggtcgtt ttatttttta 3780
   tcgttcgggt agcgcggtgt taacggtagt tttatttttt cgtgggtcgc gtcgtttagt 3840
   ttcgcggttc gaggttttgg tcgggtttag cgtgtattta ttttagattc ggggtttttg 3900
   gtttttcgtt cggggttgta tttcgtttag tttttatttt ttttagtttg atttttttcg 3960
   tgttagggaa agtagtttgt ttattggtta gataagattt ttttttttgg aaaaaagata 4020
   ggtttgaaat tagtagagtt tgggattttt ggtataggta gtagtggcgt gagacggttg 4080
   gtgatttagt tttgtaggtt tcgcggattc gggaggtggg ttgggggtac gggggcgacg 4140
   gtttcgaggt tttttagttt ttttttaaag tttgaagatg aaagtttttt ttgagtgagt 4200
   tggtttttgg tttgggggaa ggttagggaa gatagaggtg ttgattgagt tggatttttg 4260
   gtgggggtag ggggcgcgta gcgaatattt aaatttgagt tggggagggg gtgtttgcgt 4320
   gttgaatttg ggagttcggg agtttaattc gtttatgttg ggcgggtttt ttttgggtta 4380
   ggattttttg aatttttaag gagtgggaag agggagcgta gagggagggg gtgggtgggg 4440
   agaatgggaa ttagatgtcg gggaagggta cgtttggagt tcgagttatt tgtgtagggt 4500
   cgagtgggga aatatatcga aattggtttt atttgtatat agtttagcgg ttgggagtgc 4560
   gtttttttgg agttgatggt atcgggggag gggaagggag ggaaggcgag gcgcgggttg 4620
   agttgttttg cgtgcgtttt ggtcgagtgg gtttttggaa ggttgtgttt tatattttta 4680
   gtgttttttt gtcgttttta gtttttgttt tttttttatt tttttcgttt tttttttaat 4740
   agtttttttt tatttttaat ttcgaaaagt gtgtagggag tttagatttc gtttttgaaa 4800
   agtagcgttt gatattttag atgagtattt cgtagatagt tgggttgttt gatttgaatt 4860
   tgaggagttg aaaagtttgt ttattttttt gttttgatgt tgggtagatt tggttttgat 4920
   tagattaata tttttttttt ttttttattt tttttttttt tttttttttt tagagaggag 4980
   gtttagatga gtttttgttg atttgagaga gatagagaga ttacgtcgat tgttgagagg 5040
   aattggaaga agaaaaattt ttagatttag tgggaagagt ttttttatta tgagtagcgt 5100
   tatgttggtg atttgttttt cggattttag tagtagtttt cgtgaggatg tttcgcggtt 5160
   tttagtgtta ggggaagaag ggagattttt atcgtgttag ttaggggtgg gtaagggtta 5220
   ggttattaaa tttatgtttg ttttttttaa tattaggcgg aacgaagatg ggttggggga 5280
   gtcggagggg cgggtatttt cggatttttt tttgattcgg tggattaagt ttttatattt 5340
   tttattgggc gattaagacg gtgtttattt gtttcgaatt tttttggaga gggagaaatg 5400
   cgtggatatt ttagattttt ggtttgtttg taatggattt aggtagatga atttgaagga 5460
   tattaaaatt ttacgagtag ttaaagcgat ttataaaagg tatattgaga ataatagtat 5520
   tgtttttaag tagttgaagt ttgttattaa gatttatata agagatggta ttaagaagta 5580
   gtagattgat tttattatgt ttgattaggc gtagatcgag atttagtcgg tgatggagga 5640
   aaatgtttat tagatgtttt tgatttttga tatatatttc gaatatgtga ggagtggggg 5700
   agaaaatata gtttatatga gtaatggggg attcgggagt ttaaaggtcg tgtgtggtta 5760
   tttttttatt ttgaatgaag aagaggagtg gatttgtgtc gattttaagt gtaaattttc 5820
   gttaatcgtg gttggtttgt ttagtaaaat tttgagggtt acggcgagtg tgaggtttac 5880
   ggaaattgtt gatagtggat ataggtaaga ttttgacggg ggcgggtggg ttgatgttgt 5940
   ggattttgta gatgttttaa gtatggtgga tgggtggatg ggtgtgttgt agagattgat 6000
   aggtttgttg agttaggatt gttggttatg aatttttttt gattttgttt tttgttgttg 6060
   aaataggggg aaagcgatgt atatatagta aatatttgta gttttttagt gttgatttta 6120
   aatagtggta agtagtattt attaatgtgg gttatttatt atttcgggtt agtgttttag 6180
   aagaggttga gagagagaga gaggatagta gtttttgatg tttttgagtt attagattta 6240
   ttaagcgtcg gttttggaag gggttatttt attattaatt agtagagggt ggaattaatg 6300
   tggttaggga ggtagttggg ttgtggtgga ggtataggaa ataatatgtg tatttattga 6360
   ggggtaagtg ggattttttt ttgaattttt ttgttaagat aagagttagt aattggttta 6420
   attgagtttg tggttttttt tgtagaatta gatatttatt tagtaagaaa ggtttttaat 6480
   tagggaaggt tttgtgattt ttttaaatga aggattttta tttttagagt ttgttggtat 6540
   attaatttag gttttaaatg tttatgagtt cgagggttag attttagttg attattttgt 6600
   tagtgttgat ggagaagaga ttttggttcg ggagtattgg ggttttggag tatttttttt 6660
   ttgaggtttt aaagataaat agttttttgt agaatggtta agttttaaga tttttgattt 6720
   ttggaggtaa ttttagattt tttgttagaa tgagtttttt tgtttttttt ttttttttcg 6780
   atttttaaat attcgaaaag agaatgagtt ttttaatttc ggggtgtggt tttatgatag 6840
   gtaggggatg gggttgtatt tgattgtttt ttgaatgatt tattttgttt tttgtgtgaa 6900
   tttttt 6906

<210> 28
<211> 6906
<212> DNA
<213> Artificial Sequence

<220>
<223> chemically treated genomic DNA (Homo sapiens)

<400> 28

gagaaattta tatagagagt aagataaatt atttagagag tagttaagtg tagttttatt 60
ttttgtttgt tataaaatta tatttcggaa ttaagaggtt tatttttttt tcggatattt 120
ggaagtcggg aggaggggag aaaatagaaa aatttatttt aataagaggt ttgaagttgt 180
ttttaaaggt taagaatttt aagatttggt tattttgtaa agaattattt gtttttgaag 240
ttttaaagag aaagtatttt agggttttag tattttcggg ttaagatttt ttttttatta 300
gtattaataa aataattaat tgaagtttga ttttcggatt tatgagtatt taaaatttgg 360
attaatatgt tagtaagttt taaaggtaag ggttttttat ttgggaaagt tataggattt 420
tttttagtta gaggtttttt ttgttgggta aatatttaat tttataaagg gaattataag 480
tttagttaag ttaattgttg gtttttattt taatagaaaa gtttaaaaaa aaattttatt 540
tattttttaa tgggtgtata tattattttt tgtgttttta ttataattta gttgtttttt 600
tagttatatt aattttattt tttattgatt agtggtgaag tgattttttt taaaatcggc 660
gtttagtgga tttggtgatt tagaggtatt aaaagttgtt gttttttttt ttttttttaa 720
ttttttttga gatattagtt cgggataata agtaatttat attagtgaat attgtttgtt 780
attgtttgaa gttagtattg gaagattgta ggtatttatt gtatgtgtat cgtttttttt 840
ttgttttaat aataaaaagt aagattaaaa agggtttatg gttagtagtt ttaatttaat 900
agatttgtta gtttttgtag tatatttatt tatttattta ttatatttaa aatatttgta 960
aagtttatag tattagttta ttcgttttcg ttaaagtttt atttgtattt attgttaata 1020
gttttcgtgg atttatattt cgtcgtggtt tttagagttt tgttggataa gttaattacg 1080
gttggcgaaa gtttgtattt gaagtcggta taagtttatt tttttttttt atttaaggtg 1140
gggagatagt tatatacgat tttaggtttt tcgagttttt tattatttat gtaagttgtg 1200

tttttttttt tattttttat atattcgagg tatatattag aagttaaaaa tatttggtag 1260
gtattttttt ttattatcga ttggatttcg gtttgcgttt ggttaaatat gatggaatta 1320
atttgttgtt ttttgatgtt attttttatg taggttttgg tggtaggttt tagttgtttg 1380
gagataatgt tgttgttttt aatgtatttt ttgtagatcg ttttggttat tcgtaaagtt 1440
ttggtatttt ttaggtttat ttgtttgaat ttattgtagg taaattagaa gtttaaggta 1500
tttacgtatt tttttttttt taggaaagtt cggaataggt aagtatcgtt ttgatcgttt 1560
aataaggagt gtaaggattt ggtttatcgg gttagagggg aattcggaga tgttcgtttt 1620
ttcggttttt ttaatttatt ttcgtttcgt ttggtgttgg aagagatagg tatgggtttg 1680
gtgatttggt ttttgtttat ttttggttga tacggtgggg tttttttttt tttttttggt 1740
attgggggtc gcggggtatt tttacggaag ttgttgttgg ggttcgggag gtaagttatt 1800
aatatagcgt tatttatggt gagggagttt tttttattga gtttgggaat tttttttttt 1860
ttagtttttt ttagtaatcg gcgtggtttt tttgtttttt ttaagttagt aggggtttat 1920
ttgaattttt tttttggaaa gaaaaggaag gggggaggtg ggagagagaa aaagggtatt 1980
gatttaatta aaattagatt tatttaatat taaagtaaga aagtaaatag gttttttaat 2040
tttttaaatt taaattaagt aatttagtta tttgcgaggt gtttatttaa agtattaggc 2100
gttgtttttt aaaagcgaaa tttgagtttt ttgtatattt ttcggggttg ggggtgggag 2160
ggagttgttg ggaaagaggc ggggaaggtg agaggaaggt aagagttggg gacggtaggg 2220
ggatattgga ggtgtaagat ataatttttt aaaaatttat tcggttaggg cgtacgtagg 2280
gtagtttagt tcgcgtttcg tttttttttt tttttttttt ttcgatatta ttaattttaa 2340
aaaggcgtat ttttagtcgt taggttgtgt gtaggtgaaa ttaatttcgg tatatttttt 2400
tattcggttt tgtatagata attcgagttt taaacgtgtt ttttttcggt atttggtttt 2460
tatttttttt atttattttt tttttttgcg tttttttttt ttatttttta aaaatttaga 2520
agattttggt ttaagagaag ttcgtttagt ataggcggat tgggttttcg gatttttaga 2580
tttagtacgt aaatattttt tttttaattt agatttgagt gttcgttacg cgttttttat 2640
ttttattaag aatttagttt agttaatatt tttgtttttt ttgatttttt tttaaattag 2700
aaattaattt atttagggga gatttttatt tttaaatttt gagagagagt tgagaagttt 2760
cggaatcgtc gttttcgtgt ttttaattta tttttcggat tcgcgaaatt tataaaattg 2820
gattattagt cgttttacgt tattattgtt tgtgttaaga attttaaatt ttattgattt 2880
taagtttgtt ttttttttaa agaaaaaagt tttatttaat taataaataa gttgtttttt 2940
ttagtacgga aaagattaaa ttaagaaggg taggggttga acggggtgta atttcgggcg 3000
agaagttagg gatttcggat ttgggatggg tgtacgttga attcgattag agtttcgagt 3060
cgcggggttg ggcggcgcgg tttacggaga agtggggttg tcgttagtat cgcgttattc 3120
gggcggtggg aggtggagcg gtttcgttat ttggaagacg aaggtcgtcg aagttaggtt 3180
tttatgtttt tcgagttttt tcggttcggt ttttcggttt aaatgtaatt gaacgttttt 3240
agagttttgg cgggtatatt tcgaggtcgg aatttcggga tacgttagta gcgtttcgag 3300
ttcgggcggg gggtcggttt tcggcgtcgt cgtttgtaga ggcgttcgta gaaaaagtat 3360
agaaagtaat agtttagttt agaaatggcg acgcgcggag tttttgtttt ttcgtaaaag 3420
gggtcggggc ggttacgggc gaggggttcg ggtcgggagg agggacgcga ggatacgggg 3480
ggtttcggat ttgggtcggt tttgcgcgta gattgagttt tcgttagcgt tgtgggtttt 3540
ttagcgcgga ttcgcggtag ggaggggaaa aggggagggg gaagagaagt aggaggagag 3600
gatggggcgg aggagaggcg gggggcgggg gagggtaagc ggggggcgtc gttatttttt 3660
tattttattt tttaaaggta ttttcgcgcg tatttatacg tcgatttata tttataatcg 3720
cgatagcgac gcgattttcg gggggtattt gttttttttt cgttttcgtt attgtcgtta 3780
ttcggataag tttttttttt gggtttttcg ttaattcggt tatcgcgttg gggagggggt 3840
ttcggcgtag ggcgcgtaag gttgttcggt taagtttttc ggaacgttgg gcgtcgggtt 3900
cgtcgttttt tttcgcgtcg gtttttagcg cgtttcgaaa tagtcggttt gttaatttta 3960
aagggtcgtt tcgtatatta aagcgcgcgg ttcgttcggt cgtttcggtt tcggtattta 4020
ttattgattt tcgcgcgcgt tcggcggcga gatttcgtag gcgtttttgg taggggttcg 4080
gtttcggttt tcggttcggt tcgcggcggt agtttcgggt tttttcgggt cggtcgcggc 4140
gtcggggaat atggggagtc gtgcggcggg ggagggggtg cgggaaataa taatttcggg 4200
tttagagcga aagttatcgg tcggggaggg ggaacggcgc gtttcgtcgt tttttttatg 4260
taaaagattc gagcgggttt tcgttttcgt tcggttcggt ttttaacgta ggttttgttt 4320
ttagtagtta ttcggcgtat taaatgtttt tcgggcgttt ttaataaaaa ttgcgttgtg 4380
gatttaattg tgtattttaa aggcgcgagt cgagcgtacg gttttgaaag ggaggtacgc 4440
gtcggtcgcg tttcgggtcg tttttcgtag tttcgttatc gtttcgtagg gtcgtttcgt 4500
tcgcgttttt ttttaggttc ggttttcgtt tggtcgggtt ttttttcgtt tttcgcgttt 4560
acgtcgcggt tcggcgcgat ttcgtttttt gtaagagggg ggtttttttt gaagcggttt 4620
cgttggggtc gagtttttat ttttattttt tatagtaggg ttttcggcgg gcgtttcggt 4680
cgtcgggcgg tttcgaaatt tatcgttttg aggggttatt tttatttttc ggttttcggg 4740
ttgttattga gttgttagga ttttattaaa gcgtagtcgg ttttagtcga ttttttcggg 4800
ttaggttcgc ggagttagtg atttcgtcgc gttaattata gtcgcgttcg gtcggttcgc 4860
gtcgttcgtt ttaaagggat agcgtttcgc gttcgtttcg tttcgtttcg gttcgttttc 4920
gtcgtacgtt tcgttttcgc gttaattttt tttcggtttt tttttcgttt tttgttagtt 4980
tttcgcggtt tcgagggcgt ttttagttcg agttttgatt ttttttttta agtttttcgg 5040
tttttttgtt ttcgtttttt ttttttgttt gttttcgttt ttttcggttt tggaatgtaa 5100
tagtttttgg tagtattatg gttatcgtaa gaattgtaag taagtagatt tttttttttt 5160

tattattaaa cgttttttta ttttatttta agttttttttg ggaatttggg tttggaggat 5220
tttataaagg gagtttcggt tttggggttg gttggttcgt tgttataggg cggattcggg 5280
tttagtcgtt ggaggcgtgt gtatgtttgt gcgcgtttgt gtacgtgtat ttgagttcgg 5340
gtaaatatag gattgtagtt ggtggatgaa ttgcgtttgt gtatgtgtga aagtggggtg 5400
tgaattcgtt agtataggtt tgagtgttaa tattagttag gaggtatatt ttagtgtttt 5460
gaagatttag gtttagtttt ttaatttgtt ggttataaga ttttcgtagc ggtatatatt 5520
ggttattttt tttaaataat tgtatattcg ttattttttt aaagtttttt tttttgttat 5580
atgttatata ttaaatcgat aggaatattt ttttttttat tttaaacgat aaggtatgta 5640
tgtcgtttta atgtatcgaa aattttttat tttattgtta tttttgaatt ttttttatat 5700
ttgtttaaaa acgtttataa ggtatatata attttttttt tttcgttaaa atgagaaaat 5760
atgttataaa tttagagtat tgtgtattta ttttgttgtt gttatagaat gattttttat 5820
gttatgagcg aataattaat tttttttttt tttaaaaaat aaagggtcgg ggacggtggt 5880
ttatgtttgt aattttagta ttttgggagg tttatgttgg aggattattt gaggttagga 5940
gttcgagatt agtttgttta atatggcgaa atttcgtttt tattaaaaat ataaaaatta 6000
gtcgggtgtg gtggtacgtg tttgtagttt tagttattcg ggaggttgag gtaggagaat 6060
cgtttgaatt taggagacgg gggtgggggg gttgtagtga gttaagatgg tattattgta 6120
cgttaggttg ggtgataaag cgagattttt ttaaaaaaaa aaaaaaaaaa aatatatata 6180
tatatatata taaaggtgtg aattaaagag atttaagaag tgaaaggtag agttttggtt 6240
ttagagttgt gtaaggtttt tattttttaa tttaatggat gacgagtttt tttaaatgcg 6300
agatggtttg ttattaggtt tttagaattt taaaatatgt aagaattgtg gtttatttta 6360
ggtatttata gaaaaagaga aggaagaggt tgggtgtggt ggtttattcg tgtaatttta 6420
gtatttcggg aggttaaggt aggcggatta tttgaggtta ggtattcgag attagtttgg 6480
ttaatatagt gaaattttgt ttttattaaa attataaata ttagttaggc gtggtggggg 6540
gcgtttgtag ttttagttat tcgggaggtt gaggtaggag aattatttaa atataggagg 6600
ttaaggttgt agtgagtttg ggcgatagag taagatttta ttttaaaaaa aaaaaagaaa 6660
agaaaaaaag aggaggaaga tttattattt atgatttgtt ggaaaggggt aggtggtagg 6720
attgtgcgtt atttgttttt agtttaaggg attgtgataa gggattagaa agtttttaga 6780
ttttttagtt taagattttg gattttggtt cgtgggtgtt atgataggtg gtcgtttttt 6840
tttaaaattt gtttttcggt gtttatggtt tgtagtttta ggttttcgat tttaggtagt 6900
taggtt 6906

<210> 29
<211> 17580
<212> DNA
<213> Artificial Sequence

<220>
<223> chemically treated genomic DNA (Homo sapiens)

<400> 29
   attttagggg gacggtataa gtttattatg ataggagtag taaggagtcg gttagaggag 60
   ggggtagtta cgatttttag gattttgggt aagaagcggt agataaattt ggtatagggg 120
   tttagggtga gggggattgg ggtttgggta ttttgtgggg gagggagggg gattacgttt 180
   gtggggtgga gtagagattg tggtggtggt cgtttggttt tggtatttgg gagaagagta 240
   gaggagtggt tttcgtttgg ttgtttttat attagttcgg tttacgttcg atggtattat 300
   attagttttt agagtttaga ttgttcgata tattgtagta gagaaatagg gttagtttta 360
   ggatagtgat tagggttata gttataagag ttgtaggagg aagataggtt tagaatagaa 420
   tttttgtgat ttagattttc ggggtttagg ataaagtata tttttaggta agaggtggag 480
   gttttagtaa taggaattta ggggatttta tttagtatag atggggaagt aaaggttatg 540
   gtaggggtgg aaggtaagat tcggttattt cggtttttta tagtttgatg gagttagcgg 600
   ggtgggcggg tagggtaggg gtattgaggt agtgttacgg tttttatttg gtgtttttat 660
   ttcggttgtg tttttaggag tagtttttat ttttatagtg tttagttcgg ttaaagaagt 720
   aggatcgaga ggtaaagatt tgtttattga ggatgtggtt ggtgttttgg gggaggaagt 780
   tattatcgat atttattttt gttttgtatt ggttttttag gtttaatttt agattttaat 840
   ttaggatttg taatattcgt tttttttatt ggttttatag aagagggagt agatttttat 900
   taggttttgt gggggtcggt gtattcggaa gaagtttatt agtagggtgg tgggtagtag 960
   tttttatacg aagaggatga ggttaaatat taggtagttt ttgtttttta ggttatttat 1020
   taggttcgtt tgtgggaagg taggggttgg tgttagtttg tagttacgtt aaaggggtag 1080
   tgggataggg tttaggttag ttggggttaa ggtgttacgg ttattaagga ggtggtagat 1140
   atgcgtatgt ttatttggtt agatatattg tattagtcgt aatcgaaggt gtttagtcgg 1200
   ttttgggggg ttaaggttag tgttgttagg ttgtagtagg ttcggttggt atagagtagg 1260
   attatggcgt tatttatcgc ggtcgtttgg tatatattgg ttttttggta taggtaataa 1320
   tagaggattt ttagggagag aaggttttta gtttttttta tattttgaga atcgttgtcg 1380
   agagatttaa aagatatttg ggtattagtg ttgtagtttt attttggttt ttaggtagat 1440
   gttagtggag ggcgttcgtt tggcgacgag gtagaggtag gtagtaagag atagcgcgta 1500
   gatgacgaat agggagtcgt ttattaggac gcggataagt agtagggttt agggttgtgt 1560
   tcggcgtcga tgggagagta tagtatatag tacgtttatt agtagaaaga gtagcgaggt 1620
   ttttataaag gtttttcgga tagcgagttt gtagttatag tatagttagc gcgggttttt 1680
   aatagttttt agtatttatt atttgtttta ttttttgtag gaaattttaa tgtcgataaa 1740
   atttagggta tttagtttga gttttgtttt tttattttta tagatggaga aattgaggtt 1800
   taaaaaggtt agaaggtgat tgatagttat ggtatttcgg aaggtgtatt aattggagtt 1860
   agaagttttt gtttgagttt tagttttgcg attttgattt tgttgtttta tttggttggg 1920
   ttttagtttt tttatttttt aagggaggat agtagttttt tagggaaatt gtataaataa 1980
   aagatggttg ggaaagtttt ttataaatcg taagatattg tataaatgac gagggtattg 2040
   cggttttagg ttatttagga gtgtatatgg ttttggtgtt tttatgggtt ggagttttta 2100
   gttcgttttt tatggtggat gatattgggg tttttttttt tatattatta taagtatttt 2160
   tatttttgtg ttttgtagat ttgtaaggtt tttgttgagt tatttgcgtt aggtgtttta 2220
   atttttttaa gattttttat agatattttt tgggttgttt ttgttttagt tttgagaggt 2280
   gggaagggat ttatatggga ttgttatttg tttttgattt ggaggttgag tttattagtg 2340
   tttcgagtat ttataagttt cggtttattt tcggtcgacg ttttattttg gttttgaata 2400
   ttatttagga gtagagatgt tttttatttt tgtgggtttt tgaggtttgt tatttttcgt 2460
   tttttattcg ggattatagt tggttatttg ggtaaagtag aggtttataa gcgttaaggt 2520
   gaagaattgt aggtagacgg ggtagtagta gagaagttag aagggtaagg gttttaggcg 2580
   gttggcgcgg ggagtatttc ggaagtagaa ggagaagagg gtggtacgta aggcggttta 2640
   gagtagatag agggttagga atatcgtttg atagttgaga cgtttgtgtt tatatagaag 2700
   tattagttag agttgggtat agacggagaa gaagagtagg gtatataggg tggtgtaggt 2760
   agttgttagt tttagggtta tagtaggtgg tagcgtaggt attagttcgg tagtaggtat 2820
   taggttagat aggttatttt ttatgttagg gagggaggtt tgaatttcgg agatagataa 2880
   ggagggattg tcggcgggat tagggaaata aatacggagg ggtgggaatt gtagggtgta 2940
   gagagggaga tggggcgttt aggaggaaag aagttgtggt tgataggttt ttttttttta 3000
   atagagagat tttgtttttt ggggaatata ggtagtttgg gttttaggtt ttttatttag 3060
   gggtatggaa gggtagtttt ttttttttag gatagatcgg ataggggttg ggggtgtttt 3120
   ttttttgggt ttattttaga gttaaggatg gggggatgta gaggtttaag gagaggattt 3180
   tttggtttcg gtcgtttatg tcgttttggg tggaggggga gagtcggttg atattcgtcg 3240
   ggttagggta gcggtcgttg cgaggggcgt ttaatgtttc gttttttttt tttttaagtt 3300
   gggggaggtt tttttttttt taataatgcg gatggggagg gtttttgttt tttgttttcg 3360
   taattttcga aagttgtttt cgttttattt ttttttatcg cgtttacgtt ttagttttaa 3420
   ggggagggag gaggcggacg ttaggttttt aggtcgttga tgggagagtc ggtttaggtc 3480
   gtggggattc ggggtttcgg tttttcggtt ttcgttgttt cgtttttttt ttcgttcgcg 3540
   tttgcgcggg cgtcgttcgt tacgtaaatt gcgtagcggt tcggttggat tcgtagttta 3600
   gcgtcgtatt cggcgtgaaa tcgtacgaga attggcgcga gttgcgtcgt tggcgggcgt 3660
   tcggggttac gggaggcggt aggcgggcgg tcgagttgcg ggggtcgtgt cgtatttagg 3720
   gggttttcgg gtgggtcgtg tttcgtgtcg ttcgttttta tttgtatggg ggttcgttag 3780
   tttttacgtt cgtttttttt atatttaaat cggggtcgtg tcgttcggtc gggggcggta 3840
   acggattttc gtttttcggt ttttgaagaa aattttcgtt tatagttaag atggttttcg 3900
   ggtttcgttt tcgggtttga attcgcggag tttcgaattt ttcgcgcggg ttcgtttttc 3960
   gtttcggcgc gttcgcggtt ttgtttattt ggtcggtcgt ttgttaggcg tgaagttttt 4020
   acgggtttta cggttttttt ttatcgggtt ttcggttttt ttttcgcgtt cggtagttta 4080
   ttttagttag ggaggaggtt ttttttttta tttttagtgt tttcgagatg gcgcgggagg 4140
   tgtttttcga ggcgggcggc gcgaagtttt cgcgtttggg aggaggagcg gggagcggga 4200
   tttttcgatc gggtatttag tttaggcgga gcggtaataa tagtaataat aattatattc 4260
   gacggttgag ttttagttgt ggttgtgtta agcgttttcg ttcgtagtaa ttattttttt 4320
   tataaagttt ttattggttt tcgggggagt tgttattatt tttattttat tggggggaaa 4380
   ttaaggttta gagaggttga aggatttgtt ggtagaagtg ggtttttagg ttttaaatgg 4440
   ggattttaga gaattttttt tttgttttta tatacgttta aggtgtgagg tagcgaggat 4500
   gtgagtttcg gggtcgaagg agggaggaaa aaggttgcgg acgaggagtt ttggattcgg 4560
   cgggggggcg gggtcgggtc gggttgattt cgtttttgga tttattcgag tgacgtttcg 4620
   agaggtgatt gggttaggtc gttgatagtt ttcgttgttt aagtaattag ggttcggagt 4680
   tcggggtgtt ggagggaggc ggtaggttcg ggttaggggt ttcgagatcg ggtttggggt 4740
   gagatttgtg cgtcgttatt acgggcgggg cggggtttgg gtttatcggg gttttgaggg 4800
   gagattgagg ttttgagtcg atagttttag ttttttgtta ggttagattc ggtagataga 4860
   tgagggttta ggaggtttgg cgggtttggg ggcgttacgg tgggagagga agttaggggt 4920
   atttgttttt gttttttagg gttatcgttg gttttagttg tgttttgatt acgtaatatt 4980
   ttgtttttat agtttagagt atgttttaga ttttagagtt tgagtcgagt gagtaggaag 5040
   atttagtttt tgtagagagg ggtttgggtt ttagtttcgt aggggacggg tttttaggtt 5100
   tcggtaagta ttatcgttag gttttaggtt ttttgtggga cgttagttat tagtaggagt 5160
   agttaattag tagtagttat tatggaggta agtattttat tatttttatt tgtcgggata 5220
   ttttattttt agtttattgg ttttttgtat tttagttgta tttataggta ttgaatgttt 5280
   tgggtcggtt tttaaatttt tagagtttta gttttttttt tcgtaaaata gtaattaata 5340
   attattattg taggattgtt gagttaatta gtaggaatat ataggatgta tttgattata 5400
   gagttggtgt atattaaagt taaaaaatgg ttgttgtcgg gcgtggtggt ttatatttgt 5460
   aattttagta ttttgggagg ttaaggtagg aggatcgttt gagtttagaa gtttaaggtt 5520
   agtttgagtg atttagggag attttagttt ttaaaatata tatatatata tatagtagtt 5580
   attagtgtgg ggttaggata gaaattagtg ataggtaggt agtgattcga aaaggaaaag 5640
   gggtttgttt aaggttatat ttttttggtg tttttttttt tagaatgtgg gggttggaag 5700
   tttttttggt gttttgtttt aggttatatg ttttttttgg gattaggttt tttagttttt 5760
   agatgtgttt ttttttgttt tgtggttcgt atttatagta ggattgtttt ggtatagtgg 5820
   ttaggggtag atgattttaa ggggtagata gtttttttag atagtatggt tgtgtggggt 5880
   gagaagtata ggttttaaga tggaaggatg gatattgggg ttttatgttt attttttagt 5940
   aggttttagt atttttgtgt ttagtattgg ttttggtatt tggaataata atagatattt 6000
   tttgaatata aaaattaggt taagttgggt ggggtggtat atgtttgtag ttttagtaat 6060
   ttaggaggtt ggtttgagtt taggaattta agattagttt gggtaatata gttagatttt 6120
   atttttaaaa atagaaaatt aggttatgtt attttggtat tgtagtttaa aagatttttt 6180
   ttttattttt aggattaaga aggtttaaga ggtttttttt ttttcgtttt tttacggata 6240
   aatatttata ttcgattttt ttagattgga ttttatagtt tgtttttttt ttttttgttt 6300
   gttttgtttt gttttgagat agggtttcgt tttattattt aggagggagt atagaggtgc 6360
   gattttagtt tattgtagtt cggaattttt gggtttaagt gattttttta cgtaattggg 6420
   aatataggtg tgtggattat atttagttaa tttatttttt attttttttt tttttttttt 6480
   tttttttttt gagacggagt tttattttgt tgtttaggtt ggggtgtagt ggtgtaattt 6540
   cggtttattg taatttttgt tttttgggtt taagtagttt ttttgtttta gtttttagag 6600
   tagttgggat tataggcgcg tgttattatg tcgggttaat tttttgtatt tttattagag 6660
   atggggtttt atcgtgttag ttagggtggt ttttattttt tgatttcgta atttattcgt 6720
   tttagttttt taatagtgtt gggattatag gcgtgagtta ttatgtttag tattttttat 6780
   atttttatga gatgagggta ggggcggggt ttttttttgt tgtttaggta ggttttaaat 6840
   ttttgggttt aaacgatttt tttgttttag tttttagaat agttgggatt ataggtgtat 6900
   gttattatat tagtttattt tttatttttt gtagaggtgg ggttttgttt tgttgtttag 6960
   ttttgtttcg aatttttggg tttaagtaat ttttttattt tggtttttta gattgttgag 7020
   attattggcg tgagttatta tgtttagttt ttatagtttt ttttaatttt ttgagtttgt 7080
   atttggttat ttttttgttt ataaattttt tttgtttttt gttttttata tatatttagt 7140
   atttattcgc gttatatagt gagtgtttat cgtgtgatat aggttgtttt agttgttagg 7200
   gggtttcggt gaatttaagc gttaggaatt tttgtttttg tggagtttat tttttagtga 7260
   gttggtgggg ttatgagtaa ataagtataa aagtatttta ttatgcgtga gaagatttgt 7320
   atgggggtag tatgtaaaag agttaagaga ggtcgggcgc ggtggtttac gtttgtaatt 7380
   ttaatatttt gggaggttta ggtgggcgta ttataaggtg aagagatgga gattattttg 7440
   gttaatatgg tgaaatttta tttttattaa aaatataaaa attagttggg tatggtggcg 7500
   cgtatttata gttttagtta ttcgggaggt tgaggtagga gaatcgtttg aattcgggag 7560
   gcggaggttg tagtgagtta agatcgtgtt attgtatttt agtttggcga tagagtgaga 7620
   tttattttaa aaaaaagagt taagagaatt cggagttata ggggcggggg gagtgatggt 7680
   agtagaggtg gttagtattt attttggtga gaagtattat tggagttaag gttataggga 7740
   tgatgggaag agagttttag gtagagagaa gggtagatgg taaggtttcg agggtaatat 7800
   ttgtttttgg ttagggtggg tttaggggag ggggtttagg aaagggtatt tggagattta 7860
   aagttgaagg ttattttaag gattttggta ttgtttttga gtaaaaattt ggaaaggatt 7920
   tgagtagaag tagtatggtt agatttagat atttagagag ttattaggga ggtggtgaga 7980
   agtagttgat ttttggattt atttttaaag tagaattggt aggatttgtt gatggattgg 8040
   atgtggggtg cgagggaaag agaggagtta ggatgatgtt aaggaatttt aaaaaaatta 8100
   ttaggttggg cgcggtggtt tacgtttata attttagtat tttggtagtt cgaggtgggt 8160
   agattatgag gttaggagat agagattatt ttggttaata cggtgaattt cgtttttatt 8220
   aaaaatataa aaaatttttt gggcgtggtg gtgggcgttt gtagttttag ttattttgga 8280
   ggttgaggta ggagaatggc gtgagttcgg gaggcggagt ttgtagtgag tagagattgt 8340
   attattgtat tttagtttgg gtgatagagt aagatttcgt tttaaaaaaa aaattattat 8400
   tggtcgggcg tggtggttta tatttgtaat tttagtatat cgggaggttg aggtaggtag 8460
   attatttgag gttaggagtt taagaatagt ttggttaatt tggtgaaatt ttgtttttat 8520
   tataaatata taaattagtt ggatgtggtg gtgggcgttt gtaattttag ttatttgaga 8580
   ggttgaggta ggagaattat ttgaatttag gaggtggagg tcgtagtgag ttgaaatttt 8640
   gttattgtat tttagtttgg gtgatagagt aagagttcgt tttaaaaaat aaaaaataaa 8700
   taaaaataaa ataaattagg ttaggcgcgg tggtttacgt ttgtaatttt agtattttgg 8760
   gaggtcgagg cgggcggatt acgaggttag gagattaaga ttattttggt taatacggtg 8820
   aaatttcgtt tttattaaaa atataaaaaa ttagtcgggc gtggtggtgg gtgtttatag 8880
   ttttagttat tcgggaggtt gaggtaggag aatggtatga attcgggagg tagagtttgt 8940
   agtgagtcga gatggtgtta ttgtatttta gtttgggcga tagagtaaga tttcgtttta 9000
   aaaaaaaaaa ttattattat tattattatt attattttat tttatttttg atatggagtt 9060
   ttgttttgtc gtttaggttg gagtgtagtg gtattatttt agtttattgt aagttttgtt 9120
   tttcgggttt acgttatttt tttgttttag tttttagagt agttcggatt atagtttttc 9180
   gttattatgt ttagttaatt tttttgtgtg tttttagtag agatggggtt ttattatgtt 9240
   agttaggatg gtttcgattt tttgattttg tgattcgttc gttttggttt tttaaagtgt 9300
   tgggattata ggcgtgagtt atcgcgtttg gttaaattat tattaatttt agagataagg 9360
   ttttgttatg ttggttaggt tggttttgag tttttggttt taagtgattt ttttattttg 9420
   gttttataaa gtgttgggat tataggtatg agttatagtg tttagttaat attaaggaat 9480
   ttggtttgag tattcggtag gatgaagttg tttttaatgg agaagaaata ttgaagggtg 9540
   agggaggatt tggaggttat tttagagggt gggatagagg gggatttttt tttttttttt 9600
   tttttgagat agagttttat tttgttattt aggttggagt gtagtggtac gatttcggtt 9660
   tattgtaatt tttatttttt gggtttacgt tatttttttg tttgagtttt tttagtagtt 9720
   gggattatag gtatgtatta tgtttagtta atttttgtgt ttttagtgga gacggggttt 9780
   ttattatgtt ggttaggttg gtttcgaatt tttgatttcg tgatttattc gttttagttt 9840
   tttaaaatgt tgggattata ggtatgagtt atcgcgtttg gtcgttagag gtggttttta 9900
   agtatttggt gggattttta gtagggtgtt agtgggagtt ggtagggatt tggagtttga 9960
   attagagttg tgtttgtttt agtttagtta ggggtagggg ttttaggtgt gtgtaaagga 10020
   aggaatggta gatgtggggt ttaagttggt tgagttttag ggaagggagt aagttttggg 10080
   aagtcgtagt atcgttggag ttggggtttc gggggtatga tttatatagg ttgggttagt 10140
   tggtgggaag tttgggatgg agttatttga tggtgataag gtttaggtta tgtttttggg 10200
   aatgagtttg aggggagagt gggataagat tatggtagaa ggagtagtaa ggtatagaaa 10260
   ggagataggg tgttggtgga gtggtagagg ataggagtaa tattttgtat aggttaggga 10320
   cgatattttt ttttagtttt agttgtagat tcgtaggaaa gagggttgcg ggagaagata 10380
   gttgttaggt gaggatagag ttggattttt taattttttg atttagtttt tagtgttttt 10440
   aaaagtaagt ggttttttag aaaagattag taagtgttag tgtttcgtta gagttacggg 10500
   gtttttgttt tttttatttg cgtagagtaa tttatatttg gttcggttat tttttatagt 10560
   ttttgttatt aggttttttt gttggatggt tgaggtgttt ttagggtagg gattgtagtt 10620
   attttgtgtt gtgatagttt atatagttta gtgtttgaaa taggtgtgga tagtggagag 10680
   gtagaaagaa gtagtttagt gttagttttt gggttagatt gtagggttta aacgtggttt 10740
   tatttttttt agtaggttga gtttgagtag gttaattaat tttatttgtt tgagtttttt 10800
   tatttgtaaa tgggaataga attagtttta tttcgtaggg ttgtggcgag gatttgatga 10860
   gttaatatag gaaaagtgtt tggaaatagt gtttggcgta gaggaggggt taagtgtttg 10920
   cggtttttat tattagaatt gttattgtta ttatttatgt atatatatat ttatatataa 10980
   atatatatat ataatttttt tttttaagat ggagttttat tttgtcgttt aggttggagt 11040
   gtagtggtgt aatttcgttt cgtagtaatt tttatttttt aggtttaagt gatttttttg 11100
   tttttatgta tatttttggt tttatatatt tataaagttt taaatttttg ggttttagtt 11160
   atttttttat tttagttttt taagtagttg ggattatagg cgtacgttat tatgtttggt 11220
   taattttttt attttaattt ttgtagagat aagggttttt ttgtgttgtt taggttggtt 11280
   tcgaattttt ggttttaagt agtttttttg ttttggtttt ttaaagtgtt gggattatag 11340
   gtatgagtta ttatattggg tttatatttt taagttatgg ataaaaattt aaagaattat 11400
   attttgtaac gtgaatattt tatgaaattt aaattttaag gtttttaaag aaagtgttag 11460
   tggaatatag taaggtgtag tgtatttatt tatatattga ttatggtttt tttttagttt 11520
   tggttataaa ggtgagtggt tgttatagag attacgtggt ttattgaatt ttttttatag 11580
   aaaatatttt tagtttattt aggaaaaggg aaaagaaagt gaataaatat ttgttaatta 11640
   attttttata tttgtaattt tagtattttg ggaggttaag gtaggaggat tatttgagtt 11700
   taggagttcg agattagttt gggtaagatg gtaagatatt tttttaaaaa ttttttaaat 11760
   taaaaaatta gtcgggtatg gtgatgtttg tagtttaagt tatttaggag gttgaggtgg 11820
   gaagatcgat tgcgtttagg attttgaggt tgtagtaagt tgagatcgtg ttattttatt 11880
   ttagtttggg tgatagagag agattttgtc gttgtttttt aaaaataaaa aggaaatatt 11940
   tgttaatttt agagtatttt ttgattttat gagttttgag taaggttggg ggtgtttttt 12000
   gtaggtttga aggggttttt gtatcggaag gttatagtta aggttgaggt ttataggtgt 12060
   cggaggcgga taggttgcga gtgtttgttt agggtggggt ggtagtattg gttattgtgt 12120
   tggttattga cgttagtttt tttcggtagt agattgtgtg tggtcggtat gtggtagggg 12180
   taggatttaa atttagtttt tgtttgatgg tagagtttta ttgttttaaa cggtttggaa 12240
   tattattgtt ttaattaagt gtgtaattta gtgtgtaagt gttttgtaat taagtgtgtt 12300
   tttgtttttt tatttataaa atagggagaa tttcgtattt ttttttttgg gaatttgagg 12360
   atttgaaaaa ttttagtgta gggatgttcg cggaagtatt agtagggatg ttcgttttag 12420
   tcgttgattt agaagtttaa tacgtagaga atgtaaagtt agaggtgtta tgttggggtt 12480
   tggggtattt ttagttatag ttattatttt ttatttttat atttagtttc gggttaggtt 12540
   ttaggcggat ggtaagagtg ggtaggtttg ggtatttatt tacgtttata tttaaggagg 12600
   agttatattt tttttttttt ttagagaagg gagagtttgg tttaggtttt gtttagtttt 12660
   tttttttttt ttagggaatt tatagatatt tttttagggt ttttttttgt aaatagaata 12720
   ttttttaaaa atttaattta gggttgggta tcgtggttta cgtttgtaat tttagtattt 12780
   tggggggtcg aggtatttgg attgtttgag gttaggagtt aaagattagt tcgattaata 12840
   tggtgaaatt tcgtttttat taaaaatata aaaattagtt gggtatggtg gcgggtattt 12900
   gtaattttag ttatttagga ggttgaggta ggagaattat ttgaatttgg tgagtagagg 12960
   ttgtagtgag ttaagattat attattgtat tttagtttgg gtgataaagt aagatttttt 13020
   ttaaaaaaaa aaaaatatat atatatatat atatatatat atatatatat atatatatat 13080
   ataaatatat atatatatat atatataaat atatgtatat ttgtatttat atatatataa 13140
   tttaatttat ataaatgggt attattttga taataattag tttggttaag tttacgaatt 13200
   tggtgaaaaa tttttatttt ataggatagg tttttggtag aattggtgtt agtagaaggt 13260
   ttaaaggggt tttttttagt ttaggttttt aaggtagttt aaggtatagt tggaaggagt 13320
   ttggggtatg atagttaagt atatagtttt tgaagttata ttggttgggt tgggatttta 13380
   gttttgttat ttattagatt agtgattttg ggtaagttat ttatgttttt tgttttagtt 13440
   tttttttttg taaaatggta attatgagga atttttttga tagggtgtgg taaggattaa 13500
   atgaatgaat atatagggag ggttttcgat tatttattat atttgtttag tgttttttgt 13560
   ttgttttgat ttttgggtaa ggggtagtgt ttaggttaag tgtttgggtg taagttattt 13620
   attttttatt tgtggggttt tgtgttcggt aagcgtagtg gttttgtggt tttatttaga 13680
   gttagtggag gagaatagtt gtcgtttttt gcgtatttag gtggttaagt aggttgtttt 13740
   agttatacgg ttttaggtaa gtttttaatt tttgtttttg tttttttatt tgtataatag 13800
   ggagagttgg atatttattt ggttgggatt tttagaggtt ttaggttatg aggggttggt 13860
   agggggtttt tttgttttta tttgttgtta tggcgatttt ggtagatggg gaattgtttt 13920
   tattttatag aagatattgg ggttttaaag ggttggagaa tgggttaagg ttagagtagg 13980
   aatgtggtta gggtttggtt tatttagttg atttttgttt tatagttatc gtattataga 14040
   attggaaatt attttgtaga taggtttgtt tcgttagtgt tttttttgtt ttcggttttt 14100
   ttgtttttat tcgaaatttt ttagtttttt aaggtgttgg gattataggt atgagtcgtt 14160
   acgtttggtt gttatttgta atttttattt ttgatttagt atgttttttt ttattgttat 14220
   tttatttttt tagggttttg aggtattttt atttgatttg ggggtagttt tgggttttgg 14280
   tgatgtttag aatgtttagg tattttgatt atagtttatt ttattttagg ttttttcgga 14340
   tttcgttttt tcgttttttt attcgttttt tttttttttt ttttttttga gataaagttt 14400
   tattttgttt tttagattgt tagattggag tgtaatggtg agattttagt ttattgtaat 14460
   tttcgttttt taggtttaag tgatttttgt gttttaattt ttttagtagt tgggattata 14520
   ggtttatatt attatgtttg gttagttttt tttttttttt gagatagagt tttattttgt 14580
   tatttaggtt ggagtgtagt ggtgtaattt cggtttattg taatttttgt tttttgggtt 14640
   taagtgattt tttcgtttta gttttttgag tagttgggat tataggttcg cgttattata 14700
   tttggttaat ttttgtattt ttagtagaga tagggtttta ttatgtgggt taggttggtt 14760
   tcgaattttt gatttcgtga tttatttgtt ttgggttttt aaagtgttgg gattataggc 14820
   gtgagttatt atatttggtt tttttttgta tttttagtag agatgggttt ttgttatgtt 14880
   gggttaggtt gatttcgaat ttttggtttt aagtgattta tttgttttgg ttttttaaag 14940
   tgttgggatt attggcgtga gttattgcgt ttatttgttt tttttttttt tttcgaggta 15000
   gagttttatt ttgttattta ggttggagtg tagtggcgtg atttagtttt attgtaagtt 15060
   tcgtttttcg ggtttacgtt attgttttgt tttatttttt cgagtagttg gaattaaggc 15120
   gttagttatt atgtttagtt aattttttgt atttttagta tagatggggt tttatcgtgt 15180
   taggatggtt tcgatttttt gatttcgtga tgcgtttgtt tcggtttttt aaagtgttgg 15240
   gattaaaggc gtaagttatc gtattcggtt atttatttgt ttttttagtt atgtgtttta 15300
   tttatgtgtt ttagttggga tttttggagg ttttgtattg gtttataggt taggttttta 15360
   ggttttttga ggggagaaga gatattttaa tggttatttt tcgtttttag tttttggttt 15420
   gtaatttatt ttttttaaag agtttttttg atagatatat tttgttataa atatttagta 15480
   tgttttttta tttgtttttt tttttttttt tttttttttt tttttttgag atgggtgtag 15540
   tggcgtgatt ttggtttatt gtaatttttg ttgtttaggt ttaagcgatt tttttgtttt 15600
   agtttttcga gtagttggga ttataggtgt ttgttatcgc gtttggttaa tttttttgta 15660
   tttttagtag agacggggtt ttattatttt ggttagtttg gttttaaatt tttgatttcg 15720
   tgattttttt aatttcggtt ttttaaagtg ttgggattat aggcgtgagt tattgcgttt 15780
   agttttttat ttgtttttta ttttgttatt gtattttgag tgtttgttta ggggagtggt 15840
   tttaagtttt tatcggtttg ggtagaggag tggagttggt aggagttggg agatggtata 15900
   gagtatagtt taaatagatt atagtaggcg ggtttttgaa taataattta tagtgtattt 15960
   gtagtttata aagtattttt tttttttttt tgttattatt attattattt gagatagagt 16020
   ttcgttttgt tatttaggtt ggagtgtagt ggtataattt cggtttattg taatttttat 16080
   ttattgggtt aaagtagttt ttttgtttta gttttttgag tggttgggat tataggcgtg 16140
   aattattata tttaattaat ttttgtattt ttagtagaga cggagtttta ttagagtttg 16200
   aggttaggtt ggtttcgaat ttttgatttt aggtgattta tttgttttgg ttttttaaag 16260
   tgttgggatt attggcgtga gttattacgt ttggtttttt tttattaatt ttttaaatta 16320
   atagagatgg ggttttgtaa tgttgtttag gttggtttcg aatttttgaa tttaagtaat 16380
   ttatttgttt tagtttttta aagtgttggg attataggcg tgaggaatta cgtttagtta 16440
   taaagtattt ttatattttt taattttatg tgatgtttat atagttattg ggagttaggg 16500
   ttaggattat ttttttataa aggtgaggat ataggttaag agagtcgaag gttggttaga 16560
   taaggggaga gatatagagt ttcgttgatt attattggat tttggatttt attaaatttt 16620
   tggaattttt tagttttagg tttttatagt tttatagaaa ttgggtaagt tttaattttt 16680
   taagtttttg tttttttatt taagtatgtt tggtatttat tttttagggt ggttaggagg 16740
   ataataaggg aatgtttcgg tttatacgta agtgtattat taatggtatt taaaaacgtt 16800
   aatagtttta gatattgttt ttagagaagg ttttgtgggg gatgcggata tgtagcggtt 16860
   aagtttttgt tttttagggg ttttgtaggt tttttgtaga ggaggtattt aattttgtcg 16920
   gttgtggtag gatatttttt tatttaaggg attggttttt ttcgatgtag aatagatatt 16980
   atgaaatagt tattaattta ggggttgtta ggaggtttta tgaaggtttt gtattaataa 17040
   tattttttgt tttttttttt ttttttttga gaaaaaaagt ttgtcgttta ggttggagtg 17100
   tagtggcgtg atttcggttt attgcgattt tttttttttg ggtttaaacg atttttttgt 17160
   tttagttttt cgagtaattg ggattatagg tacgtgttat tatgtttagt taatttttgt 17220
   gttttgagta gagatagggt tttattatgt tggttaggat ggtttcgatt ttttgatttt 17280
   gtgatgtaat tagtattttt tatgttttta ttttgtgtta ggtatagggt tttatttatt 17340
   attggtattc ggtaaggatt cgtgggatta atgagaagtt aatttaaatt tttattaaag 17400
   gtttttgagt tttttttcga tttatatata agggattggg gttttgagag gtgggttgtg 17460
   atgcgttttt ggaagattta cgagtgggtt tgtagagttg atggggaggg tttaggtagg 17520
   gttaggggtt tagaggagtt gagattaacg tcgttttttt atttattgat tttttgtagg 17580

<210> 30
<211> 17580
<212> DNA
<213> Artificial Sequence

<220>
<223> chemically treated genomic DNA (Homo sapiens)

<400> 30

tttgtagagg gttagtgggt aggggggcga cgttaatttt agttttttta agtttttggt 60
tttgtttgaa ttttttttat tagttttgta ggtttattcg tgggtttttt aggggcgtat 120
tatagtttat tttttagagt tttagttttt tgtatgtgaa tcgaggggaa gtttaggggt 180
ttttggtgaa gatttaggtt aattttttat taattttacg aatttttatc gagtgttaat 240
aatgggtgag gttttgtgtt tagtataaaa taagggtata agaaatatta gttatattat 300
aaggttagga gatcgagatt attttggtta atatggtgaa attttgtttt tatttaaaat 360
ataaaaatta gttgggtata gtggtacgtg tttatagttt tagttattcg ggaggttgag 420
gtagaagaat cgtttgaatt taggagggag aggtcgtagt gagtcgagat tacgttattg 480
tattttagtt tgggcgatag gttttttttt ttaaaagaaa aaaaaaaaaa gtaagaagta 540
ttattagtgt aagattttta tggggttttt tggtaatttt tgggttggta gttattttat 600
agtgtttgtt ttatatcgga ggaagttaat tttttggata ggaaagtgtt ttgttatagt 660
cggtaggatt gaatgttttt tttgtaaggg atttgtagag tttttggaga ataagaattt 720
ggtcgttata tgttcgtatt ttttatagag ttttttttgg agatagtgtt tggaattgtt 780
gacgttttta ggtattattg atggtgtatt tacgtgtagg tcgagatatt tttttattat 840
ttttttaatt attttgggag gtgagtatta aatatgttta ggtgagaaga tagaagttta 900
gagagttaag atttgtttag tttttgtgaa gttgtggagg tttgaggttg gaaggtttta 960
aaggtttggt ggaatttaga gtttagtgat gattagcgga gttttgtgtt tttttttttg 1020
tttagttagt tttcgatttt tttagtttgt gtttttattt ttatgagggg ataattttag 1080
ttttgatttt tagtggttgt gtgaatatta tataaaatta aaaaatgtga aagtgttttg 1140
tggttgggcg tgatttttta cgtttgtaat tttagtattt tgggaggttg aggtaggtgg 1200
attgtttgag tttaggagtt cgagattagt ttaggtaata ttgtaaaatt ttatttttat 1260
taatttaaaa aattaataaa agaaggttag acgtggtggt ttacgttagt aattttagta 1320
ttttgggagg ttaaggtagg tggattattt gaggttagaa gttcgagatt agtttggttt 1380
taaattttgg tgaaatttcg tttttattaa aaatataaaa attagttagg tgtggtggtt 1440
tacgtttgta gttttagtta tttaggaggt tgaagtagga gaattgtttt aatttagtgg 1500
gtggaggttg tagtgagtcg agattgtgtt attgtatttt agtttgggtg gtagagcgag 1560
attttgtttt aaataataat aataataata aaagaaaaaa gaaagtgttt tgtaggttgt 1620
aagtgtatta tgggttatta tttagaggtt cgtttattat ggtttatttg aattgtgttt 1680
tgtgttattt ttttaatttt gttaatttta tttttttatt tagatcgata aagatttaga 1740
attatttttt tgagtaaata tttagagtgt aatgatagag tgagaagtag gtggggggtt 1800
gggcgtagtg gtttacgttt gtaattttag tattttggga ggtcgaggtt ggggagatta 1860
cgaggttagg agtttaagat tagattgatt aagatggtaa aatttcgttt ttattaaaaa 1920
tataaaaaaa ttagttaggc gcggtggtag gtatttgtaa ttttagttat tcgggaggtt 1980
gaagtagaag aatcgtttga atttgggtag tagaggttat agtgagttaa gattacgtta 2040
ttgtatttat tttaaaaaaa aaaaaaaaaa aaaaaaaaaa gagaggtagg tagggaaata 2100
tgttgggtgt ttatggtaag atatgtttgt tagggaggtt ttttggagga ggtaaattat 2160
aggttaggaa ttgaaggcgg gggataatta ttggaatgtt tttttttttt ttagaggatt 2220
tgaggatttg gtttgtgggt tagtgtaggg tttttagggg ttttagttag gatatataaa 2280
tgagatatat gattaagaaa gtaagtaggt ggtcgggtgc ggtggtttac gtttttaatt 2340
ttaatatttt gggaggtcga ggtaggcgta ttacgaggtt aggagatcga gattatttta 2400
atacggtgaa attttatttg tattaaaagt ataaaaaatt agttgggtat ggtggttggc 2460
gttttagttt tagttattcg ggagggtgag gtaggataat ggcgtgaatt cgggaggcgg 2520
agtttgtagt gagttgagat tacgttattg tattttagtt tgggtgatag agtgaaattt 2580
tatttcgaga aaaaaaaaaa aagtaagtag gcgtagtggt ttacgttagt aattttaata 2640
ttttgggagg ttaaggtagg tggattattt gaggttagga gttcgagatt agtttgattt 2700
aatatggtaa aaatttattt ttattaaaaa tataaaaaaa ggttaggtgt ggtggtttac 2760
gtttgtaatt ttagtatttt gggagtttaa ggtaggtgga ttacgaggtt aggagttcga 2820
gattagtttg atttatatgg tgaaattttg tttttattaa aaatataaaa attagttagg 2880
tgtggtggcg cgagtttgta attttagtta tttaggaggt tgaggcggga gaattatttg 2940
aatttaggag gtagaggttg tagtgagtcg agattgtatt attgtatttt agtttgggtg 3000
gtagagtgag attttgtttt aaaaaaaaaa agaaattagt taggtatggt ggtgtgggtt 3060

tgtagtttta gttattaggg aggttaaggt ataagaatta tttgaatttg ggaggcggag 3120
gttgtagtga gttgagattt tattattgta ttttagtttg gtagtttggg agatagagtg 3180
agattttgtt ttaaaaaaaa aaaaaaaaaa aaaagcgagt aagggaacga gaggacggaa 3240
ttcggaaagg tttagggtaa gataggttgt gattagagtg tttgggtatt ttgagtatta 3300
ttaaagttta gagttgtttt taggttaggt ggggatattt taagattttg gaggagtgag 3360
gtggtagtgg ggagaggtat attaggttag gagtaaagat tatagatgat agttaggcgt 3420
ggcggtttat gtttataatt ttagtatttt gggaggttga aggatttcgg ataggggtag 3480
gagagtcggg aatagaaggg gtattgacgg aataggtttg tttgtagagt aatttttagt 3540
tttgtggtgc ggtgattgtg gagtaggggt tagttaggtg ggttaggttt tggttatatt 3600
tttgttttga ttttagttta ttttttaatt ttttggggtt ttagtgtttt ttgtgaaatg 3660
ggggtagttt tttatttgtt aggatcgtta tgataataaa tgagagtagg aaagtttttt 3720
attagttttt tatagtttga ggtttttgag agttttagtt aggtgaatat ttagtttttt 3780
ttgttgtata gatgaggaaa tagaggtaga ggttaagaat ttgtttgagg tcgtgtggtt 3840
gggatagttt gtttagttat ttaaatacgt aggaaacggt agttgttttt ttttattagt 3900
tttgagtggg gttataggat tattacgttt gtcggatata aggttttata ggtggaaggt 3960
gagtgatttg tatttaggta tttggtttgg gtattgtttt ttgtttaggg attagaataa 4020
gtagaagata ttgggtaggt atggtggatg gtcggaagtt ttttttgtgt atttatttat 4080
ttgatttttg ttatatttta ttagggaggt tttttatggt tgttatttta tagaggaaga 4140
aattaaggta gagagtatag gtaatttgtt taaggttatt gatttagtag gtagtagagt 4200
tgggatttta atttaattaa tgtggtttta ggggttgtgt gtttagttgt tatgttttag 4260
gtttttttta gttgtatttt gggttgtttt ggaggtttag gttggggaag gtttttttgg 4320
gttttttgtt agtattagtt ttgttaggaa tttgttttat ggggtgggag ttttttatta 4380
aattcgtgga tttagttaag ttagttgtta ttaagatgat atttatttgt gtaaattgaa 4440
ttatatatat gtaaatataa atatatatat atttatgtgt gtgtgtgtgt atatatttgt 4500
gtgtgtatgt atgtgtgtgt gtgtgtgtat atatatatat atgtattttt ttttttttag 4560
agggagtttt gttttgttat ttaggttgga gtatagtgat gtgattttgg tttattgtaa 4620
tttttgttta ttaggtttaa atgatttttt tgttttagtt ttttgggtag ttgggattat 4680
aggtgttcgt tattatgttt agttaatttt tgtattttta gtagagacgg ggttttatta 4740
tgttggtcgg gttggttttt aatttttgat tttaggtaat ttaagtgttt cggtttttta 4800
aagtgttggg attataggcg tgagttacgg tgtttagttt taaattgaat ttttaaaaag 4860
tgttttattt atagggaaaa atttgggggg agtgtttgtg aattttttgg gggaaggaga 4920
gggttggggt agggtttggg ttagattttt ttttttttgg ggaagggaag gggtgtggtt 4980
tttttttggg tgtgaacgtg ggtgggtgtt taggtttgtt tatttttgtt attcgtttag 5040
agtttggttc ggagttgggt gtggaagtgg agggtagtgg ttgtggttag aagtgtttta 5100
ggttttagta tggtattttt agttttatat tttttgcgtg ttgggttttt gagttagcgg 5160
ttggggcgga tatttttgtt gatgttttcg cgagtatttt tgtattggga tttttagagt 5220
ttttagattt ttaagaagga gaatacgaag ttttttttgt tttatagatg aggaaataga 5280
ggtatattta attataagat atttgtatat tgaattgtat atttaattaa aatagtggtg 5340
ttttagatcg tttggagtag tgaagttttg ttattaggta agggttgggt ttggattttg 5400
tttttattat atgtcgatta tatataattt gttgtcgggg gaggttgacg ttagtggtta 5460
gtatagtggt tagtgttgtt attttatttt gggtagatat tcgtagtttg ttcgttttcg 5520
gtatttgtag gttttagttt tgattatagt ttttcggtat agaagttttt ttaggtttgt 5580
agagaatatt tttaattttg tttagagttt ataaaattag aaagtatttt aaagttggta 5640
aatgtttttt ttttgttttt aaaaaatagc gatagggttt ttttttgtta tttagattgg 5700
agtggggtgg tacgatttta gtttattgta gttttaaaat tttaggcgta atcgattttt 5760
ttattttagt tttttgagta gtttggatta taggtattat tatgttcggt taatttttta 5820
atttaaaaaa tttttagaga agtgttttgt tattttgttt aggttggttt cgaatttttg 5880
agtttaagtg atttttttgt tttggttttt tagagtgttg ggattatagg tatgaaaggt 5940
tggttggtaa atgtttgttt attttttttt tttttttttt aagtgagttg aaaatgtttt 6000
ttataaaggg ggtttagtgg gttacgtggt ttttgtgata attatttatt tttgtggtta 6060
gagttgaaag gaaattataa ttaatgtgta aatgaatata ttgtattttg ttgtgtttta 6120
ttaatatttt ttttagggat tttgaaattt gaattttata gaatgtttac gttataaaat 6180
atagtttttt ggatttttgt ttataattta aaaatatagg tttaatgtgg tggtttatgt 6240
ttgtaatttt aatattttgg gaggttaagg taaaaggatt gtttgaggtt aggagttcga 6300
gattagtttg agtaatatag aaagattttt gtttttataa aaattaaaat aaaaaaatta 6360
gttaggtatg gtggcgtgcg tttgtagttt tagttattta ggaagttgag gtgggaggat 6420
ggttggagtt tagaaatttg aggttttata aatatataaa gttaagaatg tatataaagg 6480
taagaaaatt atttgaattt gggaggtaga ggttgttgcg aggcgagatt gtattattgt 6540
attttagttt gggcgataga gtgaggtttt attttggaaa aaaaaattat atatatatat 6600
ttatatataa atatatatat atataaataa taataatggt agttttaata ataagagtcg 6660
taaatattta gttttttttt tgcgttaggt attgttttta agtatttttt ttatattaat 6720
ttattaggtt ttcgttatag ttttgcgagg tgggattagt tttattttta tttatagatg 6780
aggaaattta ggtagatgga gttaattaat ttgtttaagt ttaatttgtt aggaagggta 6840
gagttacgtt tgaattttgt agtttggttt aggggttagt attgggttgt ttttttttgt 6900
ttttttattg tttatatttg ttttagatat tgggttgtgt gggttgttat agtataaggt 6960
ggttgtagtt tttgttttgg ggatatttta gttatttagt aggggggttt ggtggtaggg 7020

attatgagga gtgatcgaat taagtgtgag ttgttttgcg taggtagaga gggtagaggt 7080
ttcgtggttt tggcggggta ttggtatttg ttgatttttt ttgggaaatt atttgttttt 7140
ggagatattg aaaattaaat taaaaggtta agaaatttag ttttgttttt atttggtagt 7200
tgtttttttt cgtagttttt ttttttacgg gtttgtagtt ggggttgggg ggaggtgtcg 7260
tttttggttt gtgtagaatg ttatttttgt tttttgttat tttattagta ttttgttttt 7320
tttttgtatt ttgttatttt ttttattatg attttgtttt attttttttt tagatttatt 7380
tttagggata taatttaggt tttgttatta ttaaataatt ttattttaag ttttttatta 7440
gttgatttaa tttgtgtgga ttatgttttc gggattttaa ttttaacgat gttgcggttt 7500
tttagggttt gttttttttt ttgaagttta gttagtttgg attttatatt tattattttt 7560
ttttttgtat atatttgaag tttttatttt taattggatt gagataagta taattttggt 7620
ttagatttta agtttttgtt agtttttatt agtattttgt taaaaatttt attagatgtt 7680
taaaagttat ttttagcggt tagacgcggt ggtttatgtt tgtaatttta gtatttgggg 7740
aggttgaggc gggtggatta cgaggttagg aattcgagat tagtttggtt aatatggtga 7800
aattttcgtt tttattaaaa atataaaaat tagttgggta tggtgtatgt ttgtaatttt 7860
agttattggg gaggtttagg taggagaatg gcgtgaattt aggaggtgga agttgtagtg 7920
agtcgagatc gtgttattgt attttagttt gggtgataga gtaagatttt attttaaaaa 7980
aaaaaaaaaa aaaaaatttt tttttatttt attttttagg gtagttttta gatttttttt 8040
tattttttag tgtttttttt ttattaaaag taattttatt ttatcgggtg tttaagttag 8100
attttttggt gttggttggg tattgtggtt tatgtttgta attttagtat tttgtgaggt 8160
taaggtggga ggattatttg gggttaggag tttaagatta gtttggttaa tatagtaaga 8220
ttttgttttt aaaattaata ataatttggt taggcgcggt ggtttacgtt tgtaatttta 8280
gtatttgggg aggttaaggc ggcggattta taaggttagg agatcgagat tattttggtt 8340
aatatggtga aattttattt ttattaaaaa tatataaaaa aattagttgg gtatggtggc 8400
gggaggttgt agttcgagtt attttggagg ttgaggtagg agaatggcgt gaattcggga 8460
ggtagagttt gtagtgagtt gagatggtgt tattgtattt tagtttgggc gatagagtaa 8520
gattttatgt taaaaataaa ataaaataat aataataata ataataataa tttttttttt 8580
tgagacggag ttttgttttg tcgtttaggt tggagtgtag tggtattatt tcggtttatt 8640
gtaagttttg tttttcgggt ttatgttatt tttttgtttt agtttttcga gtagttggga 8700
ttataggtat ttattattac gttcggttaa ttttttgtat ttttagtaga gacggggttt 8760
tatcgtgtta gttaggatgg ttttgatttt ttgatttcgt gattcgttcg tttcggtttt 8820
ttaaagtgtt gggattatag gcgtgagtta tcgcgtttgg tttaatttat tttattttta 8880
tttatttttt gttttttgag acggattttt gttttgttat ttaggttgga gtgtagtggt 8940
aggattttag tttattgcga tttttatttt ttgggtttaa gtgatttttt tgttttagtt 9000
ttttaggtag ttgggattat aggcgtttat tattatattt agttaatttg tgtatttgta 9060
gtagagatag gattttatta agttggttag gttgtttttg aatttttgat tttaagtgat 9120
ttgtttgttt tagtttttcg atgtgttggg attataggtg tgagttatta cgttcggtta 9180
ataataattt tttttttgag acggaatttt gttttgttat ttaggttgga gtgtagtggt 9240
gtaatttttg tttattgtaa gtttcgtttt tcgggtttac gttatttttt tgttttagtt 9300
tttagagtag ttgggattat aggcgtttat tattacgttt agagaatttt ttgtattttt 9360
agtggagacg gggtttatcg tgttagttag aatggttttt attttttgat tttatgattt 9420
gtttatttcg aattattaaa gtgttgggat tataggcgtg agttatcgcg tttagtttaa 9480
tagttttttt aaaatttttt ggtattattt tgattttttt ttttttttcg tattttatat 9540
ttagtttatt agtagatttt gttagttttg ttttaaaaat aaatttaggg attagttatt 9600
ttttattatt tttttggtga ttttttgaat gtttaaattt gattatgttg tttttgttta 9660
aatttttttt aggtttttat ttaaaggtaa tgttagagtt tttagaatga tttttaattt 9720
taggttttta aatatttttt tttaaatttt tttttttagg tttattttag ttaggagtag 9780
gtgttgtttt cgaggtttta ttatttgttt ttttttttgt ttggaatttt ttttttatta 9840
tttttgtggt tttggtttta gtggtgtttt ttattaaggt gagtgttgat tatttttgtt 9900
attattattt ttttcgtttt tgtgatttcg gattttttta attttttttt ttgagatgag 9960
ttttattttg tcgttaggtt ggagtgtagt ggtacgattt tggtttattg taattttcgt 10020
ttttcgggtt taagcgattt ttttgtttta gtttttcgag tagttgggat tataggtgcg 10080
cgttattatg tttagttaat ttttgtattt ttggtagaga tggggtttta ttatgttggt 10140
taggatggtt tttatttttt tattttgtga tacgtttatt taggtttttt aaagtgttgg 10200
aattataggc gtgagttatc gcgttcggtt ttttttaatt tttttatatg ttgtttttat 10260
ataaattttt ttacgtatga taggatgttt ttgtgtttgt ttgtttatgg ttttattagt 10320
ttattagaag gtaagtttta taagggtaag gatttttgac gtttgggttt atcgaggttt 10380
tttagtaatt ggaatagttt gtgttatacg gtgagtattt attgtgtggc gcgagtgaat 10440
gttgagtgtg tgtggggggt agagagtagg ggaagtttgt gagtagaaaa atggttaagt 10500
gtaagtttag gaagttaaag aaagttgtgg aggttgagta tggtggttta cgttagtaat 10560
tttaatagtt tgggaggtta aggtgagagg attgtttggg tttaggaatt cgagataaga 10620
ttgggtaata aagtaagatt ttatttttat aaaaaataaa aaatgagttg gtgtggtggt 10680
atgtatttgt ggttttagtt gttttggagg ttgaggtagg aggatcgttt gagtttagga 10740
gtttgaggtt tgtttgggta ataaaaggag atttcgtttt tgtttttatt ttatgaaaat 10800
gtgaaaaatg ttgggtatgg tggtttacgt ttgtaatttt aatattgttg ggaggttgag 10860
gcgggtggat tacgaggtta ggaaatggag attattttgg ttaatacggt gaaattttat 10920
ttttaataaa aatataagaa attagttcgg tatggtgata cgcgtttgta gttttagtta 10980

ttttggaggt tgaggtagga gaattgtttg aatttaggag gtagaggttg tagtgagtcg 11040
agattgtatt attgtatttt agtttgggta gtagagtgag atttcgtttt aggaaaaaaa 11100
aaaaaaaaaa aagaaaaagt aaaaaataaa ttaattgggt gtggtttata tatttgtatt 11160
tttagttacg tgggaggatt atttaagttt agaagtttcg ggttgtagtg agttgagatc 11220
gtatttttgt attttttttt gggtgataga gcgagatttt gttttaaaat aaaataaaat 11280
aaatagaaaa aaagaaagta agttatgggg tttaatttga gagaatcgag tgtgggtgtt 11340
tattcgtaag agagcgaggg gaaagaggtt ttttaagttt ttttaatttt gaaaatgggg 11400
aggaggtttt ttaagttgta gtgttagagt gatatgattt agttttttat ttttagagat 11460
ggggtttggt tatgttgttt aggttggttt tgaatttttg ggtttaagtt agttttttga 11520
gttgttggga ttatagatat gtgttatttt atttagtttg atttggtttt tgtgtttaga 11580
agatatttgt tgttgtttta ggtgttaagg ttagtgttgg gtataggggt gttagagttt 11640
attgagaaat ggatatagaa ttttagtgtt tattttttta ttttggagtt tgtgtttttt 11700
attttatata gttatgttgt ttgggaggat tgtttgtttt ttgaggttat ttgtttttgg 11760
ttattgtgtt aggatagttt tgttgtgggt acgagttata gggtaagaaa gaatatattt 11820
gggagttgga aggtttaatt ttagggaaag tatgtagttt ggggtagggt attaaggagg 11880
tttttagttt ttatatttta aggaaagaaa tattaggaag gtgtggtttt aggtaggttt 11940
tttttttttt tcggattatt gtttgtttgt tattgatttt tgttttaatt ttatattaat 12000
agttgttata tatatatata tgtattttaa gagttggagt ttttttaagt tatttaggtt 12060
ggttttgaat ttttgggttt aagcgatttt tttgttttgg ttttttaaag tgttgagatt 12120
ataggtgtga gttattacgt tcggtagtaa ttatttttta gttttaatgt gtattagttt 12180
tgtaattaag tgtattttat atgtttttgt taattaattt aatagtttta taatggtggt 12240
tattaattgt tgttttacga aagaggaaat tggggttttg agagtttggg gatcgattta 12300
aagtatttag tgtttgtgga tgtaattggg atgtagggag ttagtgggtt gggggtgagg 12360
tgtttcggta ggtggaggtg gtggggtatt tatttttatg atggttgttg ttggttggtt 12420
gtttttgttg gtgattggcg ttttatagga ggtttggggt ttggcgatga tgtttgtcgg 12480
agtttgaggg ttcgtttttt gcggggttgg ggtttaggtt tttttttgta gagttggagt 12540
ttttttgttt attcggttta aattttggga tttggaatat gttttgggtt gtgaggataa 12600
gatgttacgt agttaaggta tagttggggt taacggtggt tttggaaggt agaggtaggt 12660
atttttggtt ttttttttta tcgtagcgtt tttaggttcg ttaggttttt tgggttttta 12720
tttgtttgtc gggtttggtt tggtagggag ttgaggttgt cggtttagga ttttagtttt 12780
tttttagaat ttcggtggat ttaggtttcg tttcgttcgt ggtgacggcg tataggtttt 12840
attttaagtt cgatttcgag gtttttgatt cgggtttgtc gttttttttt agtatttcgg 12900
gtttcgggtt ttagttgttt gggtaacggg aattgttagc gatttggttt aattattttt 12960
cggaacgtta ttcgggtagg tttagaggcg gggttaattc ggttcggttt cgttttttcg 13020
tcggatttaa ggtttttcgt tcgtagtttt tttttttttt ttttcggttt cggggtttat 13080
attttcgttg ttttatattt tgggcgtgtg tgggagtagg agagggattt tttggggttt 13140
ttatttggaa tttggagatt tatttttgtt aataagtttt ttagtttttt tgggttttag 13200
ttttttttta gtgaagtggg gatagtgata gttttttcgg gagttagtga aaattttgtg 13260
aagggagtga ttattgcggg cgaaggcgtt tggtatagtt ataattgaag tttagtcgtc 13320
ggatgtgatt attgttgtta ttgttatcgt ttcgtttgga ttgggtgttc ggtcggaaag 13380
tttcgttttt cgtttttttt tttaggcgcg gaagtttcgc gtcgttcgtt tcgggggata 13440
tttttcgcgt tatttcgggg gtattggaga taaggaaagg aatttttttt ttggttggga 13500
tggttgttcg ggcgcgggag gagggtcggg gattcggtga gggaggatcg tagggttcgt 13560
gggggtttta cgtttggtag gcggtcggtt aggtgagtag ggtcgcgggc gcgtcggggc 13620
gaggggcggg ttcgcgcggg ggattcgggg tttcgcgggt ttaggttcgg gggcggggtt 13680
cgggggttat tttggttatg ggcggaagtt tttttttagag atcgaggac gggaattcgt 13740
tgtcgttttc ggtcgggcgg tacgatttcg atttgggtat aggagagacg agcgtgggaa 13800
ttggcggatt tttatataag taaaggcggg cggtacgggg tacggtttat tcggggattt 13860
tttggatgcg gtacggtttt cgtaattcgg tcgttcgttt gtcgtttttc gtaatttcgg 13920
gcgttcgtta gcggcgtagt tcgcgttaat tttcgtacgg ttttacgtcg ggtgcggcgt 13980
tggattgcgg atttagtcgg gtcgttgcgt aatttgcgta gcgaacggcg ttcgcgtagg 14040
cgcggacggg gaagggggcg gagtagcggg agtcggggag tcggagtttc gggtttttac 14100
gatttgagtc ggttttttta ttagcggttt gaggatttgg cgttcgtttt tttttttttt 14160
ttgaggttgg agcgtggacg cggtggggga gggtggggcg gggtagtttt tcgagaatta 14220
cgaggatagg aggtagaggt tttttttatt cgtattattg gaggagagag agtttttttt 14280
agtttgggga gggggagagc ggggtattgg gcgtttttcg tagcggtcgt tgttttgatt 14340
cgacgggtat tagtcggttt ttttttttta tttaggacga tatgaacgat cgaggttagg 14400
gagttttttt tttgggtttt tgtatttttt tatttttggt tttggggtag gtttagggag 14460
gagatatttt taatttttat tcggtttgtt ttggagaaaa gagattgttt ttttatgttt 14520
ttgagtgagg ggtttggggt ttaggttgtt tgtgtttttt aagggtaagg gtttttttgt 14580
tgaggaggag gggtttgtta gttataattt tttttttttt gagcgtttta tttttttttt 14640
tgtattttgt aatttttatt ttttcgtatt tatttttttg gtttcgtcga tagttttttt 14700
ttgtttgttt tcgggattta ggtttttttt tttgatatgg agagtaattt gtttggtttg 14760
gtgtttgttg tcgggttggt gtttgcgttg ttatttgttg tgattttggg gttgatagtt 14820
gtttatatta ttttgtatgt tttgtttttt tttttcgttt atgtttagtt ttggttggtg 14880
tttttgtatg ggtataagcg ttttagttat tagacggtgt ttttggtttt ttgtttgttt 14940

tgggtcgttt tgcgtattat tttttttttt ttttattttc gagatatttt tcgcgttaat 15000
cgtttggggt ttttgttttt ttggtttttt tattgttgtt tcgtttgttt gtagtttttt 15060
attttgacgt ttatgaattt ttattttgtt taggtaatta attgtggttt cgggtggggg 15120
gcgggaggtg gtaagtttta aggatttata ggagtgaagg gtatttttgt ttttaggtgg 15180
tgtttaaggt taaggtgaag cgtcggtcgg agatgagtcg aggtttgtaa gtattcggga 15240
tattggtggg tttagttttt aggttagggg taggtgatag ttttatgtag attttttttt 15300
attttttagg gttgagataa aggtaattta gggaatgttt gtggaagatt ttggaaaagt 15360
taaagtattt ggcgtagatg gtttagtaga ggttttgtag atttgtaagg tataggaata 15420
ggaatgtttg tggtagtgtg agggagaggg ttttagtgtt atttattatg gagggcgagt 15480
tggaggtttt agtttatgga agtattaaag ttatatgtat ttttgggtga tttggggtcg 15540
taatgttttc gttatttgta tagtgtttta cggtttataa agagtttttt tagttatttt 15600
ttatttgtat aatttttttg agaggttatt attttttttt gagagatgaa gaaattgagg 15660
tttagttagg taagatagta ggattaaggt cgtagagttg gggtttagat agagattttt 15720
gattttagtt ggtgtatttt tcgaagtatt atggttgtta gttatttttt gatttttttg 15780
ggttttagtt tttttatttg taagagtgga ggaatagggt ttagattgga tgttttgagt 15840
tttgtcgata ttggggtttt ttgtaggagg tggggtaggt ggtgagtgtt gagggttgtt 15900
gagggttcgc gttgattgtg ttgtggttgt aggttcgttg ttcgaggggt ttttgtgggg 15960
gtttcgttgt tttttttgtt ggtgaacgtg ttgtgtgttg tgttttttta tcggcgtcgg 16020
gtatagtttt gggttttgtt gtttgttcgc gttttggtga gcgatttttt gttcgttatt 16080
tgcgcgttgt tttttgttgt ttgtttttgt ttcgtcgtta ggcgggcgtt ttttattagt 16140
atttatttgg aggttaaggt agggttgtag tattgatgtt taggtgtttt ttgggttttt 16200
cggtagcggt ttttagggtg tagaggaagt tgggagtttt ttttttttga gggttttttg 16260
ttgttatttg tgttagggga ttagtgtgtg ttaggcggtc gcgatgggtg gcgttatggt 16320
tttgttttat gttagtcggg tttgttataa tttgatagta ttggttttgg ttttttagag 16380
tcggttggat attttcgatt acgattggta taatgtgttt gattaggtgg gtatacgtat 16440
gtttgttatt tttttagtag tcgtggtatt ttggttttag ttggtttggg ttttgtttta 16500
ttattttttt ggcgtgattg tagattggta ttagtttttg tttttttata ggcggatttg 16560
gtgaatgatt tggggaataa aggttatttg gtatttggtt ttattttttt cgtgtgggag 16620
ttattgttta ttattttgtt ggtgggtttt tttcgggtgt atcggttttt ataggatttg 16680
gtaagggttt gttttttttt ttgtggggtt agtggagggg gcggatgttg taaattttgg 16740
gttggagttt ggggttgggt ttgggaggtt agtgtaggat aggagtaagt atcgatgatg 16800
gttttttttt ttagagtatt agttatattt ttaatgggta ggtttttgtt tttcggtttt 16860
atttttttga tcgggttggg tattgtgaag atgagggttg tttttgggag tatagtcggg 16920
gtgagagtat taggtaggag tcgtggtatt gttttagtat ttttgtttta ttcgtttatt 16980
tcgttggttt tattaagtta tgggggatcg agatagtcgg gttttgtttt ttatttttgt 17040
tatggttttt gtttttttat ttgtattagg tgaggttttt tgggttttta ttgttaaagt 17100
ttttattttt tgtttagaga tgtattttgt tttgggtttc gaaggtttag gttatagggg 17160
ttttgtttta agtttgtttt ttttttgtag tttttgtgat tgtggttttg gttattgttt 17220
tgagattgat tttgtttttt tgttgtagta tgtcgggtag tttaggtttt gggagttggt 17280
atggtgttat cgggcgtgag tcgggttggt atgggggtag ttagacgaag attatttttt 17340
tgtttttttt ttaggtgtta ggattaggcg gttattatta tagtttttat tttattttat 17400
agacgtgatt tttttttttt ttttatagaa tatttaggtt ttagtttttt ttattttagg 17460
tttttgtgtt aagtttgttt gtcgtttttt gtttaggatt ttgggggtcg tggttatttt 17520
tttttttggt cggttttttg ttgtttttgt tatagtgagt ttgtgtcgtt tttttaggat 17580

<210> 31
<211> 6887
<212> DNA
<213> Artificial Sequence

<220>
<223> chemically treated genomic DNA (Homo sapiens)

<400> 31

gttgtggaat gtaggatagg gtaggaggtg ggttaggggt ttgttgtggt tttcgttttg 60
aaatcgattt taatacgttc gcgttttgtt ttagtttttt ttttttggtt ttggagcgag 120
gtgagttttt aatggaggtg ttgatcgtgt ttgggtgagt ttttaaaatg aaatattttc 180
gttgttagat ttttggtagc gcgaggttgt gtatacgaaa ttagaagtaa aatagttcgt 240
ttcgtgtatt atttatttta gtattttttt ataaaagtac gtataatttt agagggatgt 300
tcgttaggtg gcgttcgttt tttattatat ttaggagggt tcgaggttgg tacgtttttt 360
taatattttg aggggtgatt tttaattttg gttttttagg taagaaaatt aagttttaaa 420
gagggttttg cgtgaggtcg tttggttacg ttttagaatc gcgcgtataa tttggttttt 480
gaatttaatg agtttttatt atttggttgt ttttttttat atacggttta gtttgttgat 540
tataataaaa tggtgtgttt tttttttttt atataacgag gtttacgttt agattcgtag 600
gttttggtgt gatttgtttt taaatgcgtg gaatagaaaa aaagttatta gatttgtttg 660

attaaatttt ttttttgatt attttaaatt cgaacgaagt ttttttttta atgaataagt 720
aattaaaaat gggtaaggtt tatgatggga aataattaat aaggttaatg agaaaagtcg 780
attagagtag aaaatttaat tggatttgat attagataaa gtttgattta aattttgata 840
gttatttatt gatagttagg agttttagtt tttttatttg taaaacgggt ataataatgt 900
tttttattta ttttaatttt aggggtgtgt atatttaatg aaataatagg ttttggtttt 960
tggtatattg ttagtgagta atatttgtta aaatataaaa ttatttttta tattattatt 1020
tatagtatta taagttggtt agttataata tagttttttt ttgtagtttg atatattttg 1080
atcgtgtgta tgaaaaacgt tgtagggcgt tggattattt aggaacgtag gcgcgggtgt 1140
tggacgtttg ggatgtttag gcggtttgga tcgttttcgg gagtttgcgg attgggatcg 1200
acggttagtg ttgggcgcga tttttgtttg gatttggttt tggggagtcg gtaacgttgt 1260
tttttttttt tttcgttttc gtttttattt taaatatata tatatatttt ttatttgttt 1320
atttagtgaa gtatcgggtt tagaaggtat cgggtttaaa ttttcgtttt aaaggattgg 1380
aagttagaga aggtaggtga ttttaattaa ggttatcggt atttgaggga cgaatttcgt 1440
atggtttttc gttgttgggt agtattcggc gcgcgcgatt ttcggtcgtt ggcgttaggg 1500
aaggcggttt agggtatagg tggcgggcgt cgggggcgag gcgcgcggtg gcgtagttag 1560
gaaatttcga gtttattggg cgtttttttc gggagcgcgg gtacgtgtgt agtagtttat 1620
tgcgcgtttt cgagggtcgg ggtttttttt ttttcgtttt tttggttttc gtagttaaat 1680
ttcgtttttc gttcgtcgtt cgcggaagtt ttatcgcgta gtagtatttt tagttcgggc 1740
ggtttcgggg atcgaagggc gcggtacgta gggggttcgt aggatttaaa ttgcggagga 1800
gaagtaggag gagggaggag gaagaagata aaggtgtgga ggaggagggg agggaggaag 1860
aaggaagaga ggtagaagat taggaaaaaa tgggttcgtg ggcgcggagg gggcgcggcg 1920
gggtagagag gcgcggacgt tgagcgagtt cggtgttttt cgaaagtttt atcggtcgtt 1980
cggtcgggtc gcgtagtttc gttaattttc gggtcggttt aagtcggagc gtggcgttcg 2040
tcgggaggtt gtagagtttt tggagtttat agttgagttc gaaggaagtc gttttcgatg 2100
ggtaggaagc gggatttagg tacgttcgcg cggtcgcgtt tagatttgtc gtttttcgat 2160
aggcgtaggt ttgttgcgtt ttcgttatat atcgttattt tgggtttgga tttcgtagtt 2220
acgtcgggat atttttgggt atataattat tttttttttt ttatagtttt tttattttta 2280
ttcgattttt tgttaggagt agtttttagt tgtagtttaa gattcgcgag tttatttttc 2340
gcgttaagta tttaattttc ggttttgttt atagttattt atagatttaa tgatcgatta 2400
agcgtcgtaa tggttcgaga tgatattagg agattttttt gttgtgcgtt ttcggaagac 2460
gcggaacgta gttttttttt tatatttttg ggatataaaa atattcgata atttaaagtc 2520
gttaaattat atttttttat gttttgaaga tttttgttta aggttgtggt aggagtatgt 2580
ttaattgtga tttgggattt tttttaaata tcgaattaaa gggaagaggg gagggattcg 2640
taattaaatg gtagttgttt ttaagtaagt ttttacgtag ttttaaattt attttagtat 2700
tcggtgattt agttttaaaa tatttttgtt tgtttttatg ttcggatttt attttattat 2760
ttgttagtat taagtattaa atttataaag cgatcgttgt acgggatatt aaatttatat 2820
ttgttcgttt tttatttttt aggtaattag tgttaatttt tttattatat ataattttta 2880
atatttttgt tcgaagtgag gagaacggta atttaaataa tatagcgggg gaggagagga 2940
tatattgttt ttgaaagatt gtaagtattt tgttgtttta ttttgcgttt ttatatttgt 3000
tgagtattga tattaaattt aaatggcgtt tttttaaaat tgatgtaatt gtaaagagtt 3060
gtaaagggta atgattttaa gtggatgttt ttttttaggt attttataag gtttgtataa 3120
agtaagaaag tattatgtaa tcgaaatgtt gaaaagtata gatgttgtaa aattttattt 3180
tgagattgag aattatgaat agttagtgta tttttttttg cggtgtagaa aaagaataat 3240
taaaaagaaa gtaataaggg gagatgttat ttattttaat gatattaaaa ttattaattt 3300
tagtaattta ataaattaaa attatagttt tttaaatgta tttaatggtt taagtttgtg 3360
tatttggtgt taattttttg gtaaagttta tggaaaatag tgaagtacgt taattgagta 3420
aattttatat taaaaaagga ataatatatt ttaaaatgtt tttatatatt tataatattt 3480
gtgtttttta gtttggtttt tattattata taatataatg tattttttat tttttaaaga 3540
gtaaaaagtg ttgttatttt tggattattt tatggtaatt tttaggtata tttttaagta 3600
aataaatata tttatttata atttatttta atttataagt aagagatatt taaaaaaaat 3660
aaattgaaag tttttttttt ttttttgatg taaaatttaa taaattttaa ttaaatttaa 3720
tattggtttg ttagggaaac gattataaaa tttatagaaa tttttttaaa gatgtttttt 3780
aatttttttt ttgattttag ttttaattat attatttttt tataattttt ttataaaaat 3840
cgaattaaat taaagacgac gaggaaagaa aaatattttt agagtgattt taattaattg 3900
attattttag taaagagtat tagtaatgaa gaagcggcga aattgtaggg agaaaaaaaa 3960
ggatgtattt tgtttttcgt gtttataatt ttttttagag aatagaaatt acgagtatga 4020
tttatggaat tgtattattt tggatttttt tcgtagattt gatttaattt agtattagtg 4080
ttttgttaaa gcgttggaat aagtggatat atttgtagag gtaagaaacg tggtttgggt 4140
cggatgagtt tttttgttgt tggagggtgt ttcgaggatt aggtttagga gagaaggggt 4200
taagttagat tttgttttta gcggcgtaac gtgtttagtt ttgtttgtta aagttaacgt 4260
cgtttaattg gagttagtta atggacggtc gggagcgttc gggatagttt agtggagggg 4320
tgtggtttta ggttgttatt tagtagtgag gaatgatatt cgggcgggag gggggtggta 4380
tagtggaacg taagggaagg aaagagaaaa gaaatagata aaattagggt agggggtaaa 4440
aaagtttttc gcgtttttcg gaatttagcg gtcgcggtcg cggttatcgt cgtcgtcgtc 4500
gggggtgggt ttcggaagag tttgtttagt agggaagggg aggacgggtt aggagcgaaa 4560
ttttgtaggc gaggaaggga tagacgcgta aagaaaaagt gattcgggtt gtgcgtttcg 4620

gcggcgttcg ggaggggatt gttgttacgg aggggttttc gttaattttt ttttacgttt 4680
ttcgaggggg tagattttag ttcgttttta agggcgagaa gcgatgagat aatttagttg 4740
agttgcgaaa ggagagcgtt tggttttgga ttcgggggga gggggttttc gggcggggtg 4800
ggtttttaag gtgggggcga gggtcggcgt tgattttggg acggagtggg gcgggagttg 4860
ttgggtcgcg tagtagtggg cggcggcggt ttcgtatatt tagtttattc gcgttgcggt 4920
cgtcggcgtt ttttgtttcg ttcggagttg ttcggcgttt tagttgttcg tttcgtcggt 4980
cgtttttgtt cgcggcgtag atggttgtgc gcggcgttaa tattttgacg ttttttttta 5040
tttaggcgat ttttaataag aaagaggagc gcggcgggtt ggtcgcgtta gaggggcgtt 5100
cggcgttcgg gggtatagcg gtatcggtgg tcgcggtttt cgttgtttgt tgttggcggt 5160
tttttgggga gagggacgcg ggcgcgttgg ggggcgtcga ggattttttg ttggcgtttt 5220
ttgtcggtat tagaatagtt gcggggcgga ttgcggagag ttcggaaggt tgggattcgg 5280
atttcgcgtt tagcgaggag aacgagagta ggcggcgttg cgcggacgcg cggggggtta 5340
gcggggtcgg ttttgcgggg ggatttttga gtttcggtta gtcggtttgt gagttggtcg 5400
tttttaaaga tttagaggag gaagtcgcgg gtcggagcga tagcgagatg ttcgttagcg 5460
ttttaggttt gttgcggttt cgcggggatg ggggtcgggt tgaggggatg gacgcggagg 5520
gtaattacgt cgagtggggt ggtcggtttg agtattttgc gttttggggt tcggaggttg 5580
gttaaatttc gaagttttgc gttttggcgc gagttgtttt attatttcgg gaattgagtt 5640
cgcgagagat cggagtagtt ttttagagtt ttacgttttg gggtgaggtt tgagatattt 5700
tgggttaatg tgtattagat tgggttggga gaatcgttgt tttttgcgtt cgggagtttt 5760
tgagttcgtc gttagtgttt tttgtcgtgt ttgtttttaa atttttaaaa ttagggaagc 5820
ggaaaagtga ggggtttaga gattttaggt agttagtgat ttagttttgg attagagatt 5880
tgttagttta aggttttagg gtcgggtttt atagtaggtg gataggtggt gaagaatatt 5940
ttcgagtggg ttagagttga gaatttgtag aattagagtt ttggggaaag ggttggtttg 6000
gtttttttag attttaggtt tgggagagta gttttaggtc gaagatttcg gattgggttt 6060
ttagtttatt ttggaggtag gagagaggtt tttttagtcg tggtttgttt tgttttgtgg 6120
tttggaagat tcgggtgttt gttttagttg ggtataagtt tttgtaggtt ttaagtatag 6180
gggatcgttg gatttttttg ttggagtttt tcggaagggc gggtacgaga tcgagaagtt 6240
ggtgtgggat tagggtttaa aaattcgatg taggggtaat tttttttttt tattttttag 6300
tttatttttt tatttaaggt ttcggtttag tttgttttta tgttttttat agtaatttta 6360
ggcggttgag ggtgaaagat tttaaatatt tttggtttta ggattcgttt ttattttttt 6420
ttatttgtat tagataagtg tgtgttgcga ggacgcgcgc gtgtgtgcgt gtgaattaag 6480
tgtggagagg cgcgtgaatt taagtagaga gacggaaatg tagtttagaa ttttaatgtt 6540
taacgttgag agtcgggagg gttttcgcgg atgttcgggt cgagtgtttt aagttattga 6600
ggggagtttg gggtttagag agggtgggga gttggtttcg ataatatagc gttgatatag 6660
cggttgagtt tgtcgttttt tttagaagag gtttagatgg gatatttagt ggggtaagag 6720
tttaagattg gtttttcggg tttagggttt tttagatttt cgtttcgttt gagtgggatt 6780
aaagtgtatt ttcgttttat tttcgtgatt tttgtagttg aggttcggtt ttatttcgtt 6840
tgtggcgttg atagtttttt gtttttgttt ttatcggggt tcgtagg 6887

<210> 32
<211> 6887
<212> DNA
<213> Artificial Sequence

<220>
<223> chemically treated genomic DNA (Homo sapiens)

<400> 32

tttgcggatt tcggtgggaa tagaaataag agattgttag cgttatagac gaggtgaggt 60
cgggttttaa ttgtaggggt tacgggagtg gggcggaaat atattttgat tttatttaag 120
cggagcggag gtttgggagg ttttgggttc gggagattag ttttagattt ttgttttatt 180
gggtatttta tttaggtttt ttttggggag ggcggtagat ttagtcgttg tgttaacgtt 240
gtgttgtcga gattagtttt ttattttttt tgggttttag gtttttttta gtaatttggg 300
gtattcgatt cgagtattcg cgaaagtttt ttcggttttt agcgttgagt attgggattt 360
tagattgtat tttcgttttt ttgtttgggt ttacgcgttt ttttatattt agtttatacg 420
tatatacgcg cgcgttttcg tagtatatat ttgtttggtg taggtaaggg aaggtggagg 480
cggattttgg ggttaaaggt atttagaatt ttttattttt agtcgtttgg gattgttgtg 540
agagatatgg aaataggttg agtcgaggtt ttagatgaga ggatggattg gagagtaaag 600
agggagggtt gtttttgtat cgagtttttg gattttgatt ttatattagt ttttcggttt 660
cgtattcgtt ttttcgaaga attttagtag aaaggtttag cggttttttg tgtttgaggt 720
ttatagaagt ttgtatttaa ttagggtagg tattcgggtt ttttagatta taggatagga 780
taggttacgg ttgaggaggt tttttttttg tttttaggat gaattaaaga tttaattcgg 840
gattttcggt ttagggttgt ttttttagat ttggggtttg agaaagttaa attagttttt 900
tttttaaagt tttagttttg tagattttta gttttggttt attcggaggt gttttttatt 960
atttatttat ttattgtggg gttcggtttt gggattttga attggtaggt ttttggttta 1020

gagttaggtt attggttatt tgaggttttt gaatttttta ttttttcgtt tttttgattt 1080
tggggatttg gggatagata cggtagaaag tattggcgac gaatttaaaa attttcgaac 1140
gtaaggggta gcggtttttt taatttagtt taatgtatat tggtttagga tgttttaggt 1200
tttattttag gacgtagggt tttgaggagt tatttcggtt tttcgcgggt ttagttttcg 1260
aagtgataga gtagttcgcg ttagagcgta gaatttcggg atttggttag ttttcgagtt 1320
ttagggcgta gggtgtttaa gtcgattatt ttattcggcg tggttgtttt tcgcgtttat 1380
ttttttagtt cggtttttat tttcgcgaag tcgtagtaga tttgagacgt tggcggatat 1440
ttcgttgtcg tttcggttcg cggttttttt ttttaggttt ttggaagcgg ttagtttata 1500
gatcggttgg tcgaggttta aggatttttt cgtaaggtcg gtttcgttgg tttttcgcgc 1560
gttcgcgtag cgtcgtttgt tttcgttttt ttcgttgagc gcggagttcg agttttagtt 1620
tttcgggttt ttcgtagttc gtttcgtagt tgttttggta tcggtaggag acgttagtag 1680
agagttttcg gcgtttttta acgcgttcgc gttttttttt ttaaagagtc gttaatagta 1740
gatagcggga gtcgcggtta tcgatgtcgt tgtgttttcg ggcgtcgggc gtttttttgg 1800
cgcggttagt tcgtcgcgtt tttttttttt gttgaggatc gtttggatgg agaaggacgt 1860
taaggtgttg gcgtcgcgta tagttatttg cgtcgcgggt aggagcggtc ggcggggcgg 1920
gtagttgggg cgtcgagtag tttcgagcgg gatagagagc gtcggcggtc gtagcgcgag 1980
tgagttgggt gtgcgaggtc gtcgtcgttt attgttgcgc ggtttagtag ttttcgtttt 2040
atttcgtttt aggattagcg tcgattttcg tttttatttt agaggtttat ttcgttcgga 2100
gatttttttt tttcgaattt agagttagac gttttttttt cgtagtttag ttggattatt 2160
ttatcgtttt tcgtttttag gggcgggttg gggtttgttt tttcggggga cgtgaaggag 2220
gattggcggg ggttttttcg tggtagtagt tttttttcga gcgtcgtcgg ggcgtatagt 2280
tcgagttatt ttttttttgc gcgtttgttt ttttttcgtt tgtaggattt cgtttttggt 2340
tcgttttttt tttttttgtt gggtaggttt tttcggagtt tattttcggc ggcggcggcg 2400
gtagtcgcgg tcgcggtcgt tggatttcga gaggcgcggg gaattttttt attttttgtt 2460
ttggttttat ttgttttttt tttttttttt ttttttgcgt tttattgtat tatttttttt 2520
tcgttcgggt gttatttttt attgttgagt gatagtttga ggttatattt ttttattggg 2580
ttgtttcggg cgttttcggt cgtttattgg ttggttttag ttgggcgacg ttgattttaa 2640
tagataaaat tgggtacgtt gcgtcgttaa agatagggtt tgatttaatt tttttttttt 2700
taagtttggt tttcgaagta ttttttagta ataaggaggt ttattcgatt taaattacgt 2760
tttttatttt tgtagatgta tttatttatt ttagcgtttt aatagaatat tgatattaag 2820
ttgagttaaa tttgcggaga aaatttaaga taatgtagtt ttataaatta tgttcgtagt 2880
ttttgttttt taggaaggat tataaatacg aagaataaaa tgtatttttt tttttttttt 2940
tatagtttcg tcgttttttt attgttaatg ttttttgttg aaatgattag ttaattaaga 3000
ttatttaga aatattttt tttttcgtc gttttgatt tgattcgatt tttatagagg 3060
aattataaaa aaatggtata gttagaatta aagttaggag aagaattgaa aggtattttt 3120
gggagaattt ttgtaaattt tatggtcgtt tttttggtag attaatattg aatttaattg 3180
gagtttgtta agttttgtat taggaagaaa aaaaaaattt ttaatttgtt ttttttaaat 3240
attttttatt tgtgagttga ataaaattat aaatagatat gtttgtttgt ttagaagtat 3300
atttaggaat tattataaag tgatttagaa gtgataatat tttttgtttt ttaaaaaata 3360
agaaatgtat tgtattatgt aatagtagga attaagttaa gaaatataaa tattgtgggt 3420
atataaaaat attttaaaat gtgttatttt ttttttaata taaaatttgt ttaattaacg 3480
tattttattg ttttttataa attttattag agagttaata ttaaatatat aaatttgaat 3540
tattaaatat atttaaagga ttatgatttt aatttattaa attgttagga ttggtagttt 3600
tggtgttatt aaaataaata atattttttt ttgttgtttt ttttttggtt gttttttttt 3660
tgtatcgtag aaggaaatat attgattatt tatagttttt agttttagaa tgaggttttg 3720
tagtatttat attttttagt atttcggtta tatgatgttt ttttgttttg tgtaaatttt 3780
gtagggtatt tgggaaaaaa tatttatttg aggttattgt tttttatagt tttttataat 3840
tatattagtt ttgaaggaac gttatttaaa tttggtgtta gtgtttagta agtgtaagga 3900
cgtagagtgg aatagtagaa tgtttatagt tttttaaaaa tagtgtgttt tttttttttt 3960
cgttgtatta tttaaattat cgtttttttt atttcgggta gggatgttgg gaattatgta 4020
tagtgaggag attgatattg gttgtttgga aggtagggga cgggtaggta tgggtttggt 4080
gtttcgtata gcgatcgttt tgtgggttta gtatttggtg ttgataaata atggagtgag 4140
attcgaatat aaaggtaggt agagatgttt tggggttgaa ttatcgaata ttagagtagg 4200
tttggggtta cgtaaagatt tgtttgaaaa taattattat ttagttgcga attttttttt 4260
tttttttttt gattcgatgt ttaaaaagaa ttttaaatta tagttggata tatttttgtt 4320
atagttttgg gtagaagttt ttaaagtatg aaaagatgtg gtttggcgat tttgagttgt 4380
cgggtatttt tgtgttttag gagtgtgaga aaggggttac gtttcgcgtt tttcggaggc 4440
gtatagtaag ggagtttttt ggtgttattt cgggttatta cggcgtttag tcgattatta 4500
ggtttgtggg tgattgtagg tagagtcggg agttgggtgt ttggcgcgga gggtgaattc 4560
gcgggttttg agttgtaatt agaagttgtt tttagtaagg ggtcgggtgg agatgaaaag 4620
attgtgggga aggggaggtg gttgtatgtt tagagatatt tcggcgtaat tacggagttt 4680
aggtttagag tggcggtgtg tggcgaaggc gtagtaggtt tgcgtttgtc ggaggacggt 4740
agatttggac gcgatcgcgc gggcgtgttt gggtttcgtt ttttgtttat cgagggcgat 4800
ttttttcggg tttagttgtg agttttagga attttgtaat ttttcgacgg gcgttacgtt 4860
tcggtttggg tcggttcggg agttggcgga gttgcgcggt tcggtcgggc ggtcggtgag 4920
gttttcgaga ggtatcgggt tcgtttagcg ttcgcgtttt tttatttcgt cgcgtttttt 4980

tcgcgtttac gggtttattt ttttttagtt ttttgttttt tttttttttt tttttttttt 5040
tttttttttt atatttttgt tttttttttt tttttttttt ttgttttttt ttcgtagttt 5100
ggattttacg ggttttttac gtgtcgcgtt tttcggtttt cggaatcgtt cgggttgggg 5160
gtgttgttgc gcggtgagat tttcgcgggc ggcgggcgga gagcgggatt tgattgcgaa 5220
agttaagggg gcggggaagg ggaagtttcg gttttcgggg gcgcgtagta aattgttgta 5280
tacgtgttcg cgttttcgag gggggcgttt agtgggttcg ggattttttg gttgcgttat 5340
cgcgcgtttc gttttcgacg ttcgttattt gtgttttggg tcgttttttt tgacgttagc 5400
ggtcggaggt cgcgcgcgtc gggtgttgtt tagtaacgaa aggttatgcg gggttcgttt 5460
tttagatgtc ggtgattttg attgaagtta tttgtttttt ttagttttta gttttttaaa 5520
acgagggttt ggattcggtg ttttttgaat tcggtgtttt attgagtgag taagtgaagg 5580
gtgtgtgtgt gtgtttgggg tgggggcggg ggcgagggga gggaggggta gcgttgtcgg 5640
ttttttagag ttaggtttag gtagaggtcg cgtttagtat tgatcgtcgg ttttaattcg 5700
tagattttcg agggcgattt aaatcgttta agtattttaa gcgtttagta ttcgcgtttg 5760
cgtttttgag taatttagcg ttttgtagcg ttttttatgt atacgattaa aatatattag 5820
attgtagaga gaggttgtat tgtaattggt tagtttataa tattataaat aataatataa 5880
aaggtagttt tatattttaa taaatattgt ttattgatag tgtgttagga attaaagttt 5940
attgttttat tgaatgtata tatttttggg attggaatga gtagaaaata ttgttatgtt 6000
cgttttatag atgaagaaat tgagattttt gattattagt aagtggttgt taggatttaa 6060
attaggtttt gtttgatgtt aaatttagtt ggatttttta ttttaatcgg ttttttttat 6120
taattttatt aattgttttt tattatagat tttatttatt tttaattgtt tatttattgg 6180
aaaaggagtt tcgttcggat ttaaggtaat taaagaaaaa atttagttag ataaatttaa 6240
tgattttttt tttgttttac gtatttgaaa ataaattata ttaaggtttg cggatttgaa 6300
cgtaaatttc gttgtgtaga aagaaggaaa tatattattt tattataatt aataaattaa 6360
atcgtgtgta gagggagata gttaggtgat ggaaatttat taggtttaga aattagattg 6420
tgcgcgcggt tttgagacgt agttaagcga ttttacgtaa gatttttttt ggggtttagt 6480
ttttttattt aaaaagttag ggttggagat tattttttag ggtgttaaaa agacgtgtta 6540
gtttcgaatt tttttgggta tggtggagga cgagcgttat ttagcgggta tttttttagg 6600
gttgtgcgta tttttatggg gaggtattgg aataagtagt atacgaaacg gattgttttg 6660
tttttagttt cgtgtatata gtttcgcgtt gttagagatt taatagcgga gatgttttat 6720
tttaaaaatt tatttaggta cgattaatat ttttattgaa gatttatttc gttttagggt 6780
taaggggaag ggattggagt aggacgcggg cgtgttggga tcggttttag aacggaggtt 6840
atagtaagtt tttggtttat tttttatttt attttgtatt ttatagt 6887

<210> 33
<211> 19345
<212> DNA
<213> Artificial Sequence

<220>
<223> chemically treated genomic DNA (Homo sapiens)

<400> 33

ggttgtagtg agtcgagatt gtgttattgt attttagttt gggtaataga gtgagattgg 60
gtttaaaaaa aaaagaaaga aagatttatt tagttttttt taagtttatt tgttaagtga 120
atcgtatttg tgtttttttt taaaaagatg gtttatagtg tatgagagta gtttttgtaa 180
attaatatta ttattaaaaa taagttggtt tttatgtata ttttttttaa gttgttattt 240
gaggttgttg tgttgagttt tataatagta gttttggtat ggttttgggg tttaggggaa 300
tattaattta taaggttttt tttattttgt ttttatttgt ttgaaattat tttaaaacgt 360
agcgattgaa atataagttg ttaagtatat tttattagat tttaagaagt attttatttt 420
ttttttattt tttggagaaa agatggttgg ggagtgaatt agtgtattta taagatatgt 480
tttggttgtt tttgaatatt tatatttatc gatatatagt ttaaaattat ataaggagag 540
ttaaattaaa agttaagtta agattattta tatttagatt ttagaagtaa tttaagtgtt 600
attttaggat ttttaaaaat tattttgttt tattaaattt cgtgagtttt aattaagtgg 660
agatgttttg tgtgagttga aatgttaaag aggaaatttt ttggttttta gttgatagaa 720
agtaaagtaa ttaaatttag agtttttagt tttaatttgg taaagatgtt tttgtttttt 780
tttttttggt tgggttgtgt ttttaggaat taggtgttag aatgggggtg tgagtgtgag 840
gggttggggg tttggagttt tttttgaagt tttatggagg gtgtgaattt ttattttttt 900
tatgtttgtt tatgtgtggt ttagggtata ttcgtttgtt ttttcgtgtg tggtgtgttt 960
ttattttttt attttagggt gtaggagtag acgttgtttg ggtatagtta taggaggttg 1020
ggaagtgttt gtaggttttt agtagttgga aaaggtttga gttttgttgg tagatgtttg 1080
ttgttttagt tattttatta atgttggaaa taaaattttt tattattttg attaaagttt 1140
tattttatga ttgatagttg ggtgtgtttt tttttatag aagtttttta tagtttgaaa 1200
gtaggaaaga gtgagtggga aaaagaaaag ggagatgggg ggtaaaaagg gaattgggtt 1260
agatgttatt gttaaatatt cgaaatgaga gaggtttatt taaagagaaa gataaattga 1320
gtataattaa ttaagatttg atgttttatt taatatatta tttttttatt tatttattta 1380

tttatgattt atttattgag aatttattgt gtgttttatg ggtattggtg atataaatga 1440
ataatataat ataggcgtag ggtttatttg gttattgttt agtgagtgaa ataaatatgt 1500
aaaagataat atagtatagg atatgttatt atagaagtag gaatcggtag ttttggggag 1560
tggggagata ttttataaag gaggtgatat ttgagataag tttttaggtt ttggggtaga 1620
gaaggagggg ataggatgta aagggtattt tagtgaaagg gaataaattt gtataaaggt 1680
atggattatg aaagtttatt tttgtgaagt aggttttgtg gttattattt ataaatgaaa 1740
aaattgaggt ttagtttagg gagtaaaaga gagcgtaagg ttttagattt taagatcggg 1800
tatttttttt tttttattgt ataggggatt tgtgttcgta gatttttttt gagatttttt 1860
gtttttgtag cgcgaaatag aaaagatgga aaatttaagg aggtgaattg gaatttttat 1920
ttttttattt attaaaatat tgtagatttt aaattaaaga attcgtttgc gggagtgttt 1980
ggattttgtg agttttgtgt ttgtggtcga gtggaagcgg agaggagtga gagcgttggg 2040
cgacgcggtt agttaaggcg aggtcggttt ttttcgggtt tgggttttgt ttttgcgcgt 2100
gagtcgtttg tagtttttcg aggggtagta tcgcgattag gaatacgcga acgcgagttt 2160
agattcgggt ttggaaaaag ggaagaagga gaggaggaga agggaagtta aaaagagaga 2220
gagaaaataa ggatgaacga tggggagaag ggagggtaga agagagaaaa tagaagcggg 2280
aaattcgata gaggaggaga ggtttaaagg gcggggagta atgcggggaa gggagaagtg 2340
ggagggaaga agaggagagg agttagtggc ggtggaagga aggggaagaa ttagagagaa 2400
gaaaagaggt taaggaaaag aagagtaatg taaagtcggg gtttcgaata gtgtcgtatt 2460
ttgtgggtat acgtcgggta gatagaaagt atagacgcga cgcgttcggt ttttcgtcgg 2520
gcgtcgggtt ttggtgttgt cgtttaggtg ttggttttta gttttaagtt cgtttcgtag 2580
tcggcgttgc gggtgagatc gttcgtaagt atttagttta ttggcggttg tttattagcg 2640
gtttattttt ttcggggcgt ttaaagtttt tcgttcggta ttgtttcggg agcgaacgtt 2700
gagtttttgg gatttagttt cgattcggcg tagcggtttt tttttcggtt tgcgttcggg 2760
agaatttttt tcgtcgtggc gtcggacggc gatgttaggg ttgagcggtg agttgtttcg 2820
ggtttcgcgt tttgatcgcg gattagtttt agggtttcgt cgcggaagta gggggcgcgt 2880
ttggggttag tttgtagatt ttagagttcg gcggtaggat tttcggcgtt attcggatat 2940
ttggtagtag ttttcgtttg ttttgtagtc gtttttgtat ggagaaagag aatgaaagaa 3000
aataatttta gatggagagg aggggagggg aggttagttt gtaaatattt cgggaatgaa 3060
gtttttttaaa gacgcgttgc gggtttgttt taatgttagg ttgtgatttg gtttttttaat 3120
atttattttt ttacgtttgt aattttagta ttttgggagg cgaagcgggt gaattacgag 3180
gttaggagtt taagattagt ttggttaaga tggtgaaatt tggtttttat taaaaaatat 3240
aaaagttagt cgggcgcggt gataggcgtt tgtaatttta gtttttcggg agattgaggt 3300
aggagaatcg tttgaatttg gggggtagag gttgtaatga ggcgagatta cgttattgta 3360
ttttagtttg ggcgatagag tgaggttcgg ttttaaaaaa aaaagaaaaa atttattttt 3420
tttaatagtt taatgattag ttttttgggc gtgtattaag agtttggaaa aaaaaaaggt 3480
aaattattta ataaacgttt gttttcgcga ttttttaatt cggtcgtatc gcgagggatt 3540
cggatattga aagttttttt gtcggaagat tcgggcgttg ggagaagttc gtatgggata 3600
ggttgtttta gtgttcgtcg gttttttaga atcgtgtagt tttgattttt tttaacgagg 3660
aaaaaatgtt tttataaata atgttttaaa aaaaatcgta tattttttta aaaagttaga 3720
atttcgtttt tgtcgtttag gttggagtgt agtgacgaga tttcggttta ttgtagtcgc 3780
gaatttttgg gtttaagatt tttttttgtt taaatttttt taatagttgg gattataggt 3840
ttttttttgt tttaattttt ttagtagttg ggattatagg cgtgtattat tatgtttgtt 3900
tttaattttt tttttgtaaa gataggtttt ttttatattg attaggttgg tttcgaattt 3960
ttgattttaa gttatcgttt tacgtcggtt ttttaaatgt tgggattata ggtttgagtt 4020
atcgcgttcg gcgataattt atatttttat tagttttttt ttttttattt tttttttttt 4080
tttagttttt tatgagggta acggacgggg ttagggttat ttatgatttg ggattttggg 4140
gtagttaagt ttgtaatttt agtttaaaag ttttacgttc ggagcgcgta ggtttagcgc 4200
gggttgcgag attgcgagtg gagggtttgg gaagtcgcgg ttttcgggaa atcgtttacg 4260
cggggaagga gaaaggtacg gtgggttttc gggggtattg gttttgtggt tcgaagtttc 4320
gggagtttag tgaggattag attttttttt agtaagagat ttgaacgtgt tttttcgttg 4380
ggttgcggat attgggagtc gggtcggcgg gtggtaggat aggtagtaag gtgtttatgg 4440
ggaatattgt ttcggggagt tatcggtttg ggttttggtc gcgggttggg gcggtgcgcg 4500
tttttcgtat cgcgcgtata tttttgtata tatatttgga gcgcgtttcg gggggagggg 4560
ggaggagagt taaaacgagc gttttaaaat agagagttag gtagaggagc gcgagatatt 4620
agagcgggag gtagcgattg agagtcgtcg ggtattaggc gcgtaggtcg gcgtcggagt 4680
ggtacggcgg ggcgggggcg gggtgggcgc ggacgttttt tagtatatat ttcgtattcg 4740
gttttgtagg aagttcgcgt tgattgatag ttgcgttgtt ttaaaaaggt ttagcgaaga 4800
tgttgatttg cgggtgggtt tagacgcgcg gtaggcgcgt tcgttattcg ttttttttcg 4860
cgcgttattc gagtttcgtc gtttttttag ttgtcgggag cggggtttag gtttcgtcgt 4920
cgcgttagtt tcgcggtttt agcgggtcgg gtattcgtag tcgcgttcgg gtcggcggac 4980
gttcgcgtcg gtttattatg tattgttacg tcgagttgag gttgagttcg ttcggttagt 5040
ttaaagtagt taggcggcgt tataagattt ttatgatcga cgagattttt tttaaggaga 5100
tttgcgatta ttttgagaaa tttttttttt atttcgtgtg ttcgtcgttg gtcgtgcgat 5160
ttaagttttt gtatttttgt acgggtaaga cgtttcgtta ggggataagt ggggttcggt 5220
agtttttacg ttcgtgtagg tggtttgtgt tttgcgattc gagggcgaga ggaaggttta 5280
agttaaggga tttttttttt gcgttttagt aagcggtggg tattgtaaag gtatttgcga 5340

cgtggtcggg aaggattacg taaggtgtgg atggtacgag tggaaataaa ggcggtaggg 5400
ttttaggagt gggttgttcg cgtaacgttt gattgttttg ttcggaggag aacgtttttt 5460
cgttttttgt tgaaagttta aatatttgag taggtttagc gtttaaatcg ttgttttagg 5520
aagtggtggt gtgtgtgaat tttaaatttg agtttggcgg ttttgtattt tattttgagt 5580
taatttgttt agttttttat tgtttttatt gtgaattttg taagtaaatt agtattttga 5640
ggtttttaat aaaatttcga atttattgag ttttgtttat aatgatcgtt ttttgagaaa 5700
gtagttttga agtatattgt ttttgtggta taagtcgaaa aggttatttt cgtttgtttt 5760
aagttaagtc gaaaaggtta ttttcgtttg ttttaagtga ttaaaattag taatggtaat 5820
aatgaaaata ttatttatat ttggtttttt ttttttacgg gaggtaagag taatagtaaa 5880
gataaagaga atttaatata tatattgaaa acgtagtaga gtttttatcg aaaataaatt 5940
taaggtattt ttaaaatttt gaatgtatta agagaagatt taagttttaa agtatttcgg 6000
tattgttagg atttaggtta tggaatatgg gtataataaa acgaaaaaga agtaggagta 6060
tttttattta gaaaaaagtt attgagtgtg tttttattta aaggtaagta taaagtattt 6120
ttaaattaaa agaatttttg agattgttag ttgtatgaga ggtatgagat aaattaaatt 6180
atatttaaaa tgaaaaaatg aagggttaat agttttggaa gatatttttt ggaatatttg 6240
agttgttaat gtttaatgcg tggaattttg tatttttttt tggttttatt gatagttttt 6300
atatgataat tagtaaatat atgataaata tatatagtga agttttttta aaattaaatt 6360
gtttttatat gtatttggtt gtaaaatgta tattgtattt agttatgttt ttttaaagtt 6420
aatttgtttg taaggtagaa ttaggtttga tgttttataa taagttttgt aatattgaat 6480
ttttaaaatt aagaaatata tgaaataagg aataaataag aaattttaga aataagatag 6540
attttttgag aattgatatt tttatatttg tgtataggtg gttgtggatt tttggatata 6600
tatatattga ataggggtga taagttgaat attgttttga gtataaatta ttttttgtta 6660
taataattta atttttttcg aggaaaaaga gaggatttgt tttagttatt aatattgcgt 6720
atgggatatt tttaggataa aatagaataa tttttaagta tttaggtaga gtttagtgtc 6780
ggtagaattt ttagtgattt tgtgttgtgt tgatttgaag gtagtttttt ttggtttata 6840
ggagttatag aattggatta tagaagaaaa cgtggtagaa gaggagtaat aaatagttgt 6900
agagaatagt tatttagtta gaggtggttg agtaagtgtc gtgttttatt gaattttgtg 6960
tgggaaataa attagaggaa aaaaaaatga tttatatgta gttttgtgag tgttataagt 7020
gaaatgtacg tattttatag atttaagtgt tgtttagttt tgtgtttttc gaagtttatt 7080
gatggttcgt ggtagagggt ggttgttggt aatggttttt tggtttcgtt attttagtat 7140
tttttttttt cggattgtta gttgaaaatg tgtcgttatt ttgttttagt tgagaattaa 7200
aatatagaaa ttgggatttg ttggtggaat taaatttata aaaattggtt ataatagttt 7260
atatttttag attataagtt tttagtgggt ttatagttat agttaaagtt ttatatgtat 7320
tagattatgg gaattttatt agtgtttttt taggggtttt gtaaatatta tatttttaaa 7380
gtatttttgt ttttaatagt taaggttagg ataaaaattt ttgattagat gaatgtaatt 7440
ttttttttta ttaattaaaa ataataataa taagttgttg gtattttaaa ggatattgta 7500
attttttttt tttttttttt tttttttttt tttttttttt tttttgttag atggaattaa 7560
atgttgagta ttggtggtgg aagtaatgta taaaaagttt aagtgaaagg tatatattgg 7620
aattaaagtt attgaatttt tttagttttt tatttgttga tttaagattt aaagacgtgt 7680
aatagaagtg aattgtgtta ggtaaaataa tgaaaattaa gagaaaaaaa aggttttaaa 7740
attaaaaagt tattagtatg tataataagt gtataaaatt gggatggaaa gaaaattata 7800
gtattaaaaa aaaaaaaaat attgtataat gaaatagttt atgttttgtg gatttagtaa 7860
tttaaaggtt aatttatggt attaaatatt aggtagaata gtatgtattt aagtaaattg 7920
agaaatggga atagttttta ttttaggtag ataattattt gataggaatt tattgttcga 7980
tattttagtt ttgatggaat ttttattttt taaaaattgt tgtttaatag acgttttttt 8040
attataaata taataatttt agttttggaa agatgttttt aaattttttt cggtttttta 8100
gtacgtttcg taaattattt taatagatat tgtaaaaggg aatttttttt ttgtaagttt 8160
tattatagaa cggtttatta ttatatttaa attaaaattt aaatagttaa gtttaaaaga 8220
atgtttttat ttgaaaagag ttgtgttatt taggttattg gtgaggggtg gaatatgttt 8280
agaattaagt tagtatataa aaagttggtt ttatttttaa aaaataagga ttgttataat 8340
tattttaagg gtttttatat gaagatattg ataaatgaat ttatggtatt aattattttt 8400
attataattt tttttattta gaatttagaa ttattttata tttatatatg tatatttgtg 8460
gggaggtgag tgtagatatt tggatatttt atagtttgcg aataggatga gatttttaat 8520
tattttatta atttataatt taattttttt tttttttttt ggtagaatgg gtttgttgaa 8580
ttttttattt gtttgatttt tggtggttag atttatagag ttggagaggt ttataaaatt 8640
atagattaat atttttaagt agaaggtatt ttttgtaaag tgttagggtt acgttagtgt 8700
ttcgttatag ttttagttta ttgtatgtta tttttttttt tgttatagta tttagaaatt 8760
aggaagattt ttttaatttg gttatatatt cgggattaat tcgtgtagtt atatttaggg 8820
gatagttaat tagttagtta gaagggatat ttggggaagg gtgtttattt tttaggaggt 8880
ggtattaaga atattttatt ttgtggtgga tttaagagaa gtaagttttt tggggtagtg 8940
tttttgggtg cgtttttatt ataaatattg ggtgttattt tagtaaaaaa ttaaatgcgg 9000
tgtaggtatg tatagtgttt cgtttgggag ttagggagtt ggttagtgtt ttatgaagtt 9060
agttttggtg attttttaaa ttttagtttg gtttttttta gttagagatt taaatttata 9120
ggattttagg aatttgagag ttggatattg gaatgttaat ggagaaatat aaattaaggt 9180
ttgggaaaaa tgatcgttat taataagtat ttgtattttt gttagaggta tataggtttt 9240
tagtagtaat tatttataat tgtttttttt gtttttattg taagggtaaa taaatagagg 9300

gatgtacggg atttgggatt tatttagggt tagtatatat ttggaattga gtttttttgg 9360
gatttttaat tgttatttta ttattttttt gttagagata ttaagggatt ttttaggagt 9420
tagttttttt tttttttttt ttaatagcgg gattttggtt tattgtaatt tttatttttt 9480
gggtttaaat gatttttttg ttttagtttt ttgagtagtt gggattatag gcgtttatga 9540
ttatatttag ttaatttttg tatttttagt agagataggg ttttattatg ttggttaggt 9600
tggtttcgaa tttttaattt cgtgatttat tcgtttcggt tttttaaagt gttgggatta 9660
taggtatgag ttatcgcgtt cgttgtgaag tcggttttaa tttatgaata gtttattagc 9720
ggtgtttttt ttggttttga ttttttttaa agtaaatgaa aaaacgatta gaaatttcgt 9780
ttacgggttg aaatgttttt ttaaatgttt attgtttatt attatttaat ttagtaattt 9840
tatgtaataa atatttatat ttgtttggtg atggtattgt tattgagttt attattttga 9900
aatttaattt aattgttgat tagtagttag ggtgtttaaa aaattttttt taattatttg 9960
tagaattatt gttaagtttt tttttgtttg ttttgttttg ttttgtttgt ttgtttgttg 10020
ttgttttttt ttagtgtatg tgagaggtag ttcggaattg ggatagggga atatttaata 10080
tttttgttta ttatgtggaa tttttttttt gaatttattt tatagggtgt tattattagt 10140
aaaattgtta aattaatttt gttatatata atttaaatga aattttatta tgaggttgat 10200
tttttataga tatgtttgtt ttttgttggt tgtatttttg gatttaagat tgaatttgtg 10260
atagaaatat ggatagtata aagttgttag ttttagtata ttttagaaat tagagtggaa 10320
agattaaatg taatatattt taaaaattta tatgaaaagg agttaggatt gtaaaggatt 10380
atttatttta taaagagaga ttgaaaggaa aaaaaagaag ggaagaagga aagtaagagt 10440
gggttttttt gaaatttttg aggttgtata gatttagttt tatagtatag tattttttcg 10500
aagggttgta taggtaaaat aaagttggtt tgtgaaattt tgtcgtttta tgggtattaa 10560
atggttaaag ttgattttat ttagacgcgt gttgggtttt ttttaaggtt gagatttaga 10620
gtttaagttt tgtatagtat ttagttttcg ttttggaaga ttgagtaaat gttaaattag 10680
aagtaatttt aggtgttatt tgtattttgt tttgaggtta ggtaggagga gtagtagaga 10740
tgtaaaggta ggtacgttat atgggttgag gtttttttat ttgtttttgt ttttgtttta 10800
tttttttgaa ataggatttt ggtttgttgt ttaggttgga gtgtagtggg gcgattttgg 10860
tttattgtaa tttttgtttt tcgggtttaa gcgatttttt tattttagtt tttttagtag 10920
ttgggattat agatatgtgt tattatgttt ggttaatttt tgtattttta gtagagacgg 10980
ggttttatta tgttggatag gttggtttcg aatttttgat tttaggtgat ttgttcgttt 11040
tggttttttg aagtgttggg attataggcg tgagttattg tatttagtcg aggtttttta 11100
atttgtattg agttcgtgaa cgtgtatgtt atggagtttg agggtttttt ttgttatacg 11160
ttttgtttac gattgtagtt agtagattag agttggtggg tttatggtta agtttattga 11220
taagttttta gttggaatat ggttagaggg ttgtaaggat ttaggtggga tgtataaatt 11280
aatattttat tttgtatttt ttgttgggga tgtagttttg ttttatattt tagttttgtg 11340
gaatgggtgt ttttagtgtg gttttaggga cgtatttata gttggattaa ttattttttt 11400
ttcgagaatt tgttaaagtt tataggtgtt taaaggatgg gtaaaagatt attgtgttgt 11460
aggtggtatt tttttttgga agtagtatga cgacgagaag aagagaagta ttgagggtat 11520
tcgtgtaaag attgtaagcg tggagaagta ttttgggttt gtttgtcggt gtttgttttt 11580
attttagttg tttgagagga tggaacggta gttttttttt atagtataga tggtaaaatt 11640
agaatttacg ttaggattta ttagtaacgg gagttagatt aggattttgt ataaatttgg 11700
tggtatttat aaatattttt taggaagaga ggtgttttgt ttgtttgttt tttaattaga 11760
aaatttttgt aaatttttag gatttaattt atttttatta tagtttagta atagttagaa 11820
gaaggaaagt tttgttaaaa taaatgtttt aaataaaaat atagttaatt ttttattatt 11880
atgtttaatt atttattatt attagggtat ttttatatgg taatttaatt ttttgaaagt 11940
ggaaatttgg tatacgttta aggaggaaag ttatgtattt agatagatat tagaaggcgg 12000
tataattata tttgggttcg agttgaattt tatatatgat tgttttgaag aagagagaag 12060
agagggtttt tgttcgtagt tattaaggag agattttggt tttttgagtg ttagttgaaa 12120
ttgaatatag agggagagaa ttttgaagat taggtgatgt ttaagtatat ttgaaaaggg 12180
tttttgttag ttattagttg aagttgattt tagggttagt tgagaagttt ggagggtagt 12240
tttgtaagtt tttttttggt agagtgtata tagatgatat agtttttttt tatgtatagg 12300
ttaaaagacg attgaagaga agttaagttg ggaggatttt agtattaaat taattacgtt 12360
ttatgtataa gtagatacgt ttgtaggatt tttttttatc gcgttttttt agtttattaa 12420
tatgttttta ggaatatatt tttttgggta ttgaggaaag atgagtattt tttgtttttt 12480
gtttttttgt ttgtttgttt ttgagataga gtttcgtttt gttattgagg ttggagtgta 12540
atggtgtaat ttcggtttat tgtaggtttt gttttttggg tttaagtgat ttttttgtcg 12600
tattttatta agtagttggg attataggcg tttgttatta tgttcggtta atttttgtat 12660
ttttagtaga gatggagttt tattatgttg gttaggttgg ttttaaattt ttaattttgt 12720
gatttatttg tttcggtttt ttaaagtgtt ggaagttatc gtattcggtt agatgagtat 12780
ttttataatg tgtatagaat aggattggtt gttgtttagg tgatgttaga ttatttttga 12840
tttttttttt tttttaaaaa attaatatat gtatttggta agtgatatga tgtttatttt 12900
ttggtttttt ttatttaagg attatttatt atgtggagaa atggagtggt tttgggtttg 12960
atgaaatttt atgaaatagt ggttatattt ttgttttggg aagtatttgg tattggtaag 13020
tttttgatat tgataatttg ttaattttta ttgtttattt ttttttttta gagttttttt 13080
tattcggtga attaattatt attgagattt aggatgatgg gtttttttta aaagaggtta 13140
tagtttcgtg tggattgaag gaagtgtaat ttttatgttt agtttaagta atatttaagg 13200
gagttgattt tgtatatttt ttaatggtaa tttatagata atggaatgtt gatattgtat 13260

ttttttattt tgaggaaata ttatgggtaa gaagagttgt tgagattttt ttttttgagt 13320
tttatagtat ttatttaagg ggagttataa atttgagaga aatgttagaa atattttata 13380
ttttaaatta tatttataga tagggtaata atagtaatag cgattgttta gaaattattt 13440
aatatgtaat aggtattaga tttagtgttt tttgattagt tttaaaatat ttttatgaaa 13500
taggtttatt attttttttg gtaatggtgg aattttagaa gttaaatgat ttttttaaag 13560
ttataagata taaagagagt ttttatgaag taataagaaa aatgggtaaa ggttataatt 13620
aggtaattta tagaaaggaa gattttaatg gttagtaaat atgaaaggat gtttaatttc 13680
gttgataagt gggaatattc gaattaatat gagatattat tttatattta ttgggtagtt 13740
taaaatgaaa ggataaaatc ggttgatgat aaggagatgg agtaataggt attttttata 13800
tttcggcggg agtgttgtat ggtacgatta ttttggagag taatttggta ataaatggta 13860
aagttgtaga cgtatatatt ttagggttta gtagttttat ttttgggtat gtgtttggag 13920
aaattagtgt atgagtgtaa aaggatattt gtaaaaatgt ttatttgtgt gttatgttag 13980
taaaaaataa aaaataattt agttgttttt ttagaaggaa aaaggttaaa ataatatatg 14040
ttatatttat gtaatgaaat tttatgtagt aattaaaata aatgaattag atatatttgt 14100
attaatatgg atatatttta ggaatataat gttgagaaaa aaagttgtag attgatattt 14160
attgtttgat attatttatt taaatggttg aaaaggtata aattaatatt atatattgtt 14220
tagagaggta ggggtatggg agtggggagt gttaattata ggtttttatt tttttatgta 14280
atttatttat ttattaagat aaaaaggttt aatgtaaata tggttaagta ttacgatgtg 14340
aagaaatttg ggttatggtt atatagaagt taatgatatt attttttgta tttttttata 14400
tatttgaaat tttttataat atgaagattg aaaagtgata gtataagaga tcgtatatgt 14460
ttatagaatt tttaattttg tggaattttt gatttttttt ttaatagttt ttttattttg 14520
aaatagtagt atttaaatat agagttttgt tgtatatagt tttatagtgg gaaattgaga 14580
atattttata attaaatata tgtataattt atattgaaat aattattgtg gtttttgaag 14640
tggtttttgg atttattaat tttgtttggt tgatgttata cgaataagtg gtcgggttga 14700
gattgaaatt tgtatttgtt ttttttaata tagattttag tattggatat gattatatga 14760
aaatgtgtaa attagaatat gtttgtttta ttatatatag gtaatatatt gtgtgtgtgt 14820
gtgtgtgtgt gtgtagatat aaaaaatata tatggtaagg tttaattaat gtattagttt 14880
tgttggaatt tattttgaat ttgttaattt ttttatatat atttatatta atttaatgtg 14940
tgttagttcg ttttttattt tttgtggtga ttggggtttt tgaatgattt tagagggatt 15000
ttaagaggcg gtggggtttt gcggggaagg gaggtgattg ggagattgta gatttgttat 15060
tatttggttg tgtgattttt tattttttat tgttttgggg tttgtgttcg tgtttgtgta 15120
gtattcgtta ttaatttttg ggttacggtg tttttattag ttgttatttt ttgaataaag 15180
tttttttagg ggaattaagg gtggaatttt tatttattta tttattatta attagagaga 15240
aagtgttgaa taattaaagt taattaaatg ttttgagagt taatatttcg gatatttttt 15300
atggaatatt tattaggttt agtgagaaaa aatgtttggt cgttgattgg tattatttaa 15360
aagaacgaga ttattttttt atattatatt tggttgagtt ggagtggatt ttgggaattt 15420
ttatttttaa aaattttttt gtatgattgt aatgaaaagt taattttggg aattataata 15480
atgtagataa tagtttaaat atgagattga tgtaatttgt attttacgat ttaaatgtat 15540
gaaattattt agtggagtaa ttttattgtt ggggttttga gttagacggt tttgggtttt 15600
attgtatttt attgtttgtg tgattttagg taaattattt aatttttatg atatttagtt 15660
tttttatttg tgaaatgaag ataataataa ttatataata gggttagttg tggaggttaa 15720
atgagataat gtatataaag tttttagtat tttattggtt gtgtgatttt aggtaaattt 15780
aatttttatg atacgtaatt tttttatttg taaaatgaag ataataataa ttatatgata 15840
gggttagttg tggaggttaa atgagataat gtatataaaa tttttagtat agtttgtggt 15900
aataaagtgt ttagtatttg atagttttta ttaaaaataa ttattttata atagtaattt 15960
tattattgaa gtataatttt ttagggaaag attattatta ttggtgaatt aaatttaaga 16020
aaaatttaaa ttttgaaaaa ttttatttta ttttagggta tttttttggg ttatagcgat 16080
attagttgtt tttaagattt tttgtaattt gggtggtttg tgaattgttt gagtttaagt 16140
ttttatgaat taggtatgtt ttattttttt tatttttttg ttttgtaata tgaatttaga 16200
attttataaa tttataaatg agtggtataa ataggttagc gttataatat tataagatgt 16260
gttttgttat tttatttttt tttttaataa gagtttttta ggatttattg tttttaggaa 16320
atgttttatt atatagttgt aagagtttta agaattgttt tagaaaagtg gtagaatttt 16380
ttaatacgtt tgattttttt ttttttttta agagtagagt tagggatagt tttttttttt 16440
tttgaaaaaa aatgaaatta tttttttttt tttattttcg agtattgaga gaggaaaata 16500
attttattga gatgtttttt tttaattagg tagtatggaa aatggtagta gtatgataat 16560
taattatttt tttagtatat tattgggttt gtgatttaga gtgagtttta aatgaagtgt 16620
ttttgtgata gtttttaaat tattggtggg gttagttttt gaatgtattt taagtggtaa 16680
tgatggatgg ttgttacgtg gaggaattgt ttgaggaaga gttttatgtg gatttttttg 16740
ggattgtgtt ttttagttgg ttattagtgt tttatgaagt ttagtatttg ttggtgaaat 16800
attttaattg atgaattttt tttacggtgt gagaagaatt tttttgatga ggagttttta 16860
ttttttatgg tgtaaaggta tcgttagtga atagggtgat gtttttgttt agttatttat 16920
aatgatgatt tcgataggta atttttagtg gttttattta ggttttgtgg tgggtggggg 16980
ttaagtggga taagataggt ggatgtgtta ttattattga gacggagtgg tatgtattta 17040
tgaagagtga gaaggttgat tttgtttttt agggttttta gaattataga gtttgagttt 17100
tttgaattgt tgaaaatagg atttagttag agatatggat cgttggtatt ttgttgagaa 17160
tattttattt agtttgtgga tttagaaaaa taaaatgtat atgtttgtgt tttagtagga 17220

ggggagtagg taggaatggg gtgggagtgg aaaaggaatg ttggttaatg gggaagggag 17280
agtgaggggt aaggaaataa gttagtttat tgaaataggg atttaaatgt ttatttagta 17340
tttgttgttt ttaagagaaa aataaattga aaaatgagaa tggaagattt tatttggaaa 17400
tggttttgtg agtacgtgtt tatttttaga ggcgtttttt ataattgata gtagttaatg 17460
atttttgatt gggtaatggt ttggttagtt atataggtag tttaattatt atatgtatta 17520
atggtgtatt taaaaatttt attatttggg gttataaaag attatgacga ttgtattata 17580
atataataat ggtatttatg tagtggcgta tagaaaattg tgtgtatttg acgtataggc 17640
ggtgtggaag taggatttaa agtagaattt ttgttaaaaa tttttttttt agagttttta 17700
ttgttttatt ttgtgatgtt ttaatacgtt agtttttgtt gttggtaaaa tatattttat 17760
tttttagtta tgttggggag tagtgattga tgaaggagga ttaatttgtt gtttttcggg 17820
agggattttt tgtagttttt aggggattat atgtattatg gtgtttttat agaaagttag 17880
attagggatt ggttaaattt ggtttattac gaattgtttt attattataa gttaaggatg 17940
gtttttatat tcgtaaatcg aggaaaagaa attaaaagcg gaatatttcg tgatttgtga 18000
aaattatgtg aaatttagat attagtgttc gtatataaaa atgttattgg aatatagtat 18060
atgtattagt ttgcgtgttg tttgtggtag tttttatgtt gttatagtgg agggagaggt 18120
tgtgatagat atagtatggt ttataaagtt taaaatattg atttgttggt tttttataag 18180
gaaggttcgt taattttttt attagattgt tggaagtaga gttttttagt tacgaaataa 18240
ggttttttag tatattagag tttgaaggat tttttagaat tagttagttt ttttatttat 18300
tttatagatg atgaaattga gagataaaat gatttatata ggtaatgatt tagatatttt 18360
aagttttgtg ttgaataatt aaagggaaaa gtttttattt tgaattttat agatttaata 18420
gattgttgga tagaaaagaa tttttgtttt gaaattttta acgtaggagt ggtatataat 18480
tggtatgtat gttattatat tttgtttcgg ttatatgttt atagtagata ttgttaatcg 18540
atttagtatt ttttttttag ttaagttttg gttagtttga gaattttttt taatatagtg 18600
ttggaggcgt tagttatatc gatttgagtt tttatgtgat ttaaaatttg tttgtttttt 18660
ttttttgaat gatgtattta tgtgatttat gatgtgttga tagtatggga ttttaagtta 18720
gaagaatttg gtttgatttt tttatttgtt gttttttagt tacgttattt taggtaattt 18780
atttaatttt tttggggtaa tttatttttt ttgagatgag aaagttgaat taaataattc 18840
gaaagtattt tgttaagttt tgaaaaatgt tatgttttaa ggtatataga ttttttagga 18900
agaatttata gttataagta tgttttttta taaagattat aggtggtttt taggaagttt 18960
gtatattata aatttatttc gggttttgga aatatgtgtt tttagttttt tttgattatt 19020
tttttttatt tgtttggttt tatggattaa gaatttaggg atttcgaagg gagagagggg 19080
aaagatttgt ttaaaattag aggatttgta ggaatttttt ttgggtcgtg ggtttatttt 19140
ttttatatag agtagagtag atttttgtgt ttgttttgta gttgtaggtt ttgagttatt 19200
tttttttttt ttttttttat ttgtattttt ttaagagtag ggtttttttt gttagataga 19260
ttttagttag cggtttttcg taggtttggg tttgtttttt tatatcgttt tttgtggtga 19320
tttgtttttt tgtttgtttt ttagg 19345

<210> 34
<211> 19345
<212> DNA
<213> Artificial Sequence

<220>
<223> chemically treated genomic DNA (Homo sapiens)

<400> 34

tttggaaaat aagtagggag gtaggttatt atagggaacg gtgtggggaa gtaggttagg 60
atttgcggag ggtcgttggt tgaggtttgt ttgataggga gggttttgtt tttaaggagg 120
tatagatgga ggaaggggaa ggagaggtaa tttaagattt atagttgtag ggtaggtata 180
gaggtttgtt ttgttttgtg tgggaagatg aagtttacgg tttagaagag gtttttgtaa 240
gttttttggt tttaagtaga tttttttttt tttttttttc ggaattttta aatttttggt 300
ttatgggatt aggtagatga aaagaagtag ttaggaaaag ttagagatat atattttaga 360
aattcgaaat agatttgtaa tatgtaaatt ttttgagaat tatttgtggt ttttataaaa 420
agatatgttt gtagttatga attttttttg agaagtttgt gtattttgaa gtatagtatt 480
ttttagagtt tgataggatg ttttcggatt atttaattta atttttttat tttagaggga 540
gtaaattgtt ttaggaaggt taagtgagtt gtttaagata acgtaattag gaagtagtag 600
atggaggagt taaattaagt ttttttgatt tgaagtttta tgttattagt atattatgaa 660
ttatatggat atattattta gaagagggag gtaaatagat tttaaattat atgaaagttt 720
agatcggtat gattggcgtt tttagtatta tgttaaggag gatttttagg ttggttagag 780
tttggttgag gaaaaggtgt tgggtcgatt aataatgttt gttatgggta tgtggtcggg 840
gtagggtatg gtggtatata tattagttat atgttatttt tgcgttagga attttaaggt 900
aagagttttt ttttgtttaa taatttgtta aatttgtaaa gtttaagtaa agagtttttt 960
tttttgatta tttaatatag agtttaaaat gtttggatta ttatttatgt gaattatttt 1020
attttttagt tttattattt ataaggtgag taagaaaatt agttgatttt aaagaatttt 1080
ttaaatttta atatattgaa aaattttatt tcgtaattga ggagttttgt ttttaatagt 1140

ttagtggaag ggttggcgaa ttttttttgt aaagggttag taagttaata ttttaggttt 1200
tatgggttat gttgtgtttg ttataatttt tttttttatt gtgatagtat gaaagttgtt 1260
atagataata cgtagattgg tgtatgtgtt gtgttttaat aatattttta tgtacggata 1320
ttgatgtttg aattttatat aatttttata ggttacgaaa tgtttcgttt ttgatttttt 1380
tttttcggtt tacgaatgtg aaaattattt ttagtttgtg gtagtagaat aattcgtagt 1440
gggttagatt tagttaattt ttggtttagt tttttgtgga aatattataa tatatgtaat 1500
tttttggaga ttgtaaggag tttttttcga aaagtagtaa attaattttt ttttattaat 1560
tattgttttt taatatattg gggaggtaag atgtgtttta ttaatagtag aaattgacgt 1620
attgaaatat tatagaatga ggtagtggag gttttggagg aaaggttttt gataggagtt 1680
ttattttaaa ttttgttttt atatcgtttg tgcgttaagt atatataatt ttttgtgcgt 1740
tattgtatga atattattat tgtattataa tatagtcgtt atgatttttt gtgatttagg 1800
atgatgaaat ttttgaatgt attattaata tatatgatga ttgggttatt tgtgtggttg 1860
gttaggttat tgtttaatta gaagttattg attgttgtta gttatgggga acgtttttga 1920
aaataagtac gtatttatag ggttattttt aaatgaaatt ttttattttt attttttaat 1980
ttgttttttt tttggagata ataaatgttg agtaagtatt tggatttttg ttttagtgga 2040
ttaatttgtt tttttatttt ttattttttt ttttttattg gttaatattt tttttttatt 2100
tttattttat ttttgtttat ttttttttta ttggaatata aatatatgta ttttgttttt 2160
ttgaatttat aggttgaata gaatattttt aataagatgt taacgattta tgtttttgat 2220
tgagttttat ttttaataat ttagaaaatt tagattttat ggttttgagg attttagggg 2280
ataaagttag tttttttatt ttttataaat atatgttatt tcgttttagt ggtgatgata 2340
tatttatttg ttttatttta tttggttttt atttattata gaatttggat gaggttatta 2400
gagattgttt gtcggaatta ttattataaa tggttggata gaggtattat tttgtttatt 2460
ggcgatgttt ttgtattata gaaggtgaga attttttatt aagagaattt tttttatatc 2520
gtggagggga tttattaatt aaagtatttt attagtagat attaaatttt ataagatatt 2580
agtgattagt tgagaaatat aattttaaaa aaatttatat ggaatttttt tttaggtagt 2640
ttttttacgt gatagttatt tattattatt atttagaata tatttagaag ttagttttat 2700
tagtaattta aaagttgtta taaaggtatt ttatttggaa tttattttga attatagatt 2760
taataatata ttaggaaagt gattagttgt tatattgtta ttatttttta tgttatttaa 2820
ttggaaaaga atattttagt ggagttattt ttttttttta gtgttcgaaa ataagaaaaa 2880
aagaatagtt ttattttttt ttaaaaaaaa aaaagattat ttttaatttt atttttgaaa 2940
gaagaaaaga aattaaacgt gttgagaaat tttgttattt ttttaggata gtttttgaga 3000
tttttatagt tgtgtgatga aatatttttt aggggtaatg aattttagga aatttttgtt 3060
aaaaaaaaaa ataaagtaat aaagtatatt ttgtgatgtt gtagcgttga tttgtttgtg 3120
ttatttattt atgaatttat aaaatttgga atttatatta taaaatagaa aagtgggagg 3180
aatgaaatat atttaattta tgaagatttg aatttaaata atttatagat tatttaggtt 3240
gtaaggggtt ttaaaagtag ttagtgtcgt tgtaatttag aaaaatgttt tggagtaaag 3300
tggggttttt taagatttaa atttttttta agtttaattt attaataatg gtaatttttt 3360
tttaaagggt tgtattttag tgatgaggtt attattgtaa gataattatt tttaatagaa 3420
gttattaaat attaagtatt ttgttattat aaattatatt aggagtttta tatatattat 3480
tttatttaat ttttataatt aattttatta tgtgattatt attattttta ttttatagat 3540
gaggaaatta cgtgttatag aagttaagtt tatttaaggt tatatagtta ataaaatatt 3600
gagagtttta tatatattat tttatttaat ttttataatt aattttatta tgtgattatt 3660
attattttta ttttatagat gaggaaattg agtgttatag aagttaagta atttatttaa 3720
ggttatatag gtaataaaat ataataaaat ttaggatcgt ttgatttaaa attttaatag 3780
taggattatt ttattaaatg gttttatata tttgagtcgt ggggtgtaaa ttatattaat 3840
tttatattta agttgttatt tatattattg tggtttttag agttgatttt ttattgtaat 3900
tatatagaaa ggtttttaaa aatagagatt tttagaattt attttagttt agttagatat 3960
aatgtgaaaa aataatttcg tttttttaag tgatgttaat taacgattag atattttttt 4020
ttattgaatt taatgagtat tttatgaaaa atattcgaag tgttaatttt taaggtattt 4080
ggttggtttt agttgtttag tatttttttt ttagttgata atgaatgaat gagtaggaat 4140
tttatttttg atttttttag gaaagtttta tttaaagaat gatagttgat ggaagtatcg 4200
tgatttagag attggtagcg aatgttatat agatacggat atagatttta gggtagtgag 4260
gagtggagag ttatatagtt agatggtaat aggtttgtaa ttttttaatt attttttttt 4320
ttcgtaaaat tttatcgttt tttggaattt ttttggaatt atttagaaat tttaattatt 4380
ataggaaatg aaagacgaat tggtatatat taaattagta taaatatgtg taaaagagtt 4440
ggtaggttta aaataagttt taatagagtt gatgtattaa ttggatttta ttatatatat 4500
tttttatatt tatatatata tatatatata tatatagtat gttgtttata tgtaatgaga 4560
tagatatgtt ttaatttata tatttttata taattatatt tagtattgga gtttatattg 4620
aaagagatag atgtaagttt taattttaat tcggttattt attcgtgtga tattagttag 4680
gtaaggttag tagatttaaa agttatttta gaagttatag tggttgtttt agtgtgagtt 4740
gtatatatgt ttagttataa agtgttttta gttttttatt gtgagattat gtataataaa 4800
gttttgtatt taaatgttat tgttttagga tagaaaagtt attaagaaaa agattaaaag 4860
ttttataaga ttaagagttt tgtggatatg tgcggttttt tgtgttatta ttttttaatt 4920
tttatgttat gaaaaatttt agatatatgg aaaagtataa agaataatgt tattaatttt 4980
tatgtagtta tgatttaagt ttttttatat cgtaatattt agttatattt atattaggtt 5040
tttttgtttt aataaataaa taaattatat aggaaaataa aaatttgtgg ttagtatttt 5100

ttatttttat gtttttgttt ttttaaataa tatgtagtat tagtttgtgt ttttttaatt 5160
atttaaataa atggtattag atagtaagta ttaatttata attttttttt ttaatattgt 5220
atttttaaaa tgtatttata ttaatataag tgtatttggt ttatttattt taattgttgt 5280
atagaatttt attgtatgga tatagtatat attattttaa tttttttttt tttgaaagga 5340
tagttaaatt gtttttattt ttttattaat ataatatata ggtgaatatt tttataggta 5400
tttttttata tttatatatt ggttttttta ggtatatatt taggagtgga attgttgagt 5460
tttagggtat gtgcgtttgt agttttgtta tttgttgtta aattgttttt taaaataatc 5520
gtattatata gtattttcgt cggagtgtgg agaatgtttg ttgttttatt tttttgttat 5580
taatcgattt tgttttttta ttttgggtta tttagtgagt gtgaaatggt attttatatt 5640
aattcgggtg tttttattta ttagcgaggt tgagtatttt tttatgttta ttggttattg 5700
agattttttt ttttatgaat tgtttgattg tagtttttgt ttattttttt tattgtttta 5760
taagagtttt ttttatattt tgtgatttta ggaaagttat ttagttttta ggattttatt 5820
attattaaag gagatgataa atttatttta tagggatatt ttgaagttga ttaaaaagta 5880
ttgagtttag tgtttattat atgttaggta atttttaaat agtcgttatt attattattg 5940
ttttgtttat gaatatggtt tagggtataa agtattttta gtattttttt taagtttgtg 6000
gttttttttg gataagtatt gtaagattta gaaaaaggag ttttagtaat tttttttgtt 6060
tatgatattt ttttaagata agaagatgta atattagtat tttattgttt ataagttatt 6120
attaggaaat gtgtaaagtt agttttttta gatattgttt aaattaagta tggaagttat 6180
atttttttta atttatacga gattgtgatt ttttttggga aaagtttatt attttgagtt 6240
ttagtgatgg ttaatttatc gagtgaaaag gattttggga aagaaaggtg ggtagtggag 6300
gttggtaggt tattaatatt agggatttgt tagtattagg tattttttag gatagaaata 6360
tagttattat tttatgaaat tttattaggt ttaagattat tttatttttt tatatggtaa 6420
gtaatttttg aataagaaaa attaaaggat aagtattata ttatttatta aatgtatatg 6480
ttggtttttt aaaaaaaaaa aaagattaga aatgatttga tattatttag gtagtagtta 6540
attttatttt atgtatattg tgaaaatatt tatttggtcg ggtgcggtgg tttttagtat 6600
tttgggaggt cgaggtaggt ggattataag gttgggaatt tgagattagt ttggttaata 6660
tggtgaaatt ttatttttat taaaaatata aaaattagtc gggtatggtg gtagacgttt 6720
gtaattttag ttatttggta gggtgcggta ggagaattat ttgaatttag gaggtagagt 6780
ttgtagtgag tcgagattgt attattatat tttagtttta gtgatagagc gagattttgt 6840
tttaaaaata aataaataaa aaaataaaaa atagaaaata tttatttttt tttaatattt 6900
agaagaatat gtttttaagg atatgttggt aagttggaaa gacgcggtaa aaaggaattt 6960
tgtaagcgtg tttgtttgta tatggaacgt agttagtttg gtgttgagat ttttttagtt 7020
tggttttttt ttagtcgttt tttgatttgt gtatggagga aggttgtgtt atttgtgtgt 7080
attttgttag gaggaagttt atagagttgt tttttagatt ttttaattag ttttgaaatt 7140
aattttaatt ggtgattggt aggggttttt tttagatgta tttgaatatt atttaatttt 7200
tagaattttt tttttttgta tttaatttta attggtattt aagaaattag agtttttttt 7260
tagtgattac ggatagaggt tttttttttt ttttttttta gagtaattat atgtaggatt 7320
taattcgagt ttaaatataa ttatatcgtt ttttgatatt tatttaggtg tatggttttt 7380
ttttttaaac gtgtgttaag tttttatttt taagaagtta aattgttata taaaaatgtt 7440
ttggtgatga tgagtaatta gatatggtaa tggaaaatta attgtgtttt tatttagagt 7500
atttatttta ataagatttt ttttttttta attgttatta agttgtaata aaaataaatt 7560
aaattttgag ggtttgtaaa ggttttttgg ttaaaaaata aataaataaa atattttttt 7620
ttttggagag tatttgtgaa tgttattaag tttgtataaa gttttggttt gattttcgtt 7680
attaataaat tttaacgtga attttgattt tgttatttgt gttgtggaaa gaaattatcg 7740
ttttattttt ttaggtagtt aaaatggagg taggtatcga taggtaggtt taaggtgttt 7800
ttttacgttt gtagtttttg tacgaatgtt tttaatgttt tttttttttt cgtcgttatg 7860
ttgtttttaa gagagggtgt tatttgtagt atagtggttt tttgttattt ttttgagtat 7920
ttgtgggttt tagtaagttt tcggggaaaa ggtggttaat ttagttgtga gtgcgttttt 7980
agagttatat tagaaatatt tattttataa gattggagtg taaaatagaa ttgtattttt 8040
aatagaagat gtaaaatgag gtgttaattt atgtatttta tttgagtttt tgtagttttt 8100
taattatatt ttagttgaga gtttgttaat gggtttagtt atagatttat taattttagt 8160
ttgttaatta tagtcgtggg taggacgtgt ggtaaggaag atttttaggt tttatagtat 8220
gtacgtttac gagtttagta taaattaaga ggtttcggtt gggtgtagtg gtttacgttt 8280
gtaattttag tattttagga ggttaaagcg ggtagattat ttgaggttag aagttcgaga 8340
ttagtttgtt taatatggtg aaatttcgtt tttattaaaa atataaaaat tagttaggta 8400
tggtggtata tgtttgtagt tttagttatt agggaggttg aggtgggagg atcgtttgaa 8460
ttcgggaagt agaggttgta gtgagttaag atcgttttat tgtattttag tttgggtaat 8520
agattaagat tttgttttaa aaaaataaaa taaaaataaa aataaatgaa gaggttttag 8580
tttatgtgac gtatttattt ttgtattttt gttgtttttt ttatttgatt ttaaaataaa 8640
gtgtaaataa tatttaaaat tgtttttggt ttaatattta tttagttttt tagagcgaag 8700
gttaaatgtt gtgtagagtt taagttttgg attttaattt tgaagaggtt ttagtacgcg 8760
tttaagtaga attaatttta gttatttagt atttatggag cgatagaatt ttataagtta 8820
gttttgtttt gtttgtatag tttttcggag gaatgttgtg ttatgaggtt gaatttgtgt 8880
agttttaaga attttaaaga aatttatttt tatttttttt tttttttttt tttttttttt 8940
tttaattttt ttttataaat aaaataattt tttgtaattt taattttttt ttatatagat 9000
ttttgggata tgttatattt aattttttta ttttgatttt taggatatgt taaaattaat 9060

aattttatat tatttatgtt tttattataa atttagtttt ggatttagaa atataattag 9120
taaaaaataa atatatttgt aggaaattag ttttataata gagttttatt tgagttgtat 9180
gtagtaaagt tgatttgata gttttgttgg tgataatatt ttgtaggtag agtttaaaga 9240
gaaaatttta tatagtaagt aaaagtatta gatgtttttt tattttaatt tcgagttgtt 9300
ttttatatat attaggaaaa aataataata aataaataaa taaaataaaa taaaataaat 9360
aaagaagggt ttagtaatgg ttttataagt agttgagagg gattttttaa atattttaat 9420
tgttagttaa tagttagatt aagttttaaa ataatagatt taatgataat gttattatta 9480
agtaaatata aatatttgtt gtatgaaatt attgaattaa atgatagtga atagtggata 9540
tttggagggg tattttaatt cgtgaacgaa atttttaatc gtttttttat ttgttttgag 9600
aaaaattaga gttagagaag atatcgttaa tgaattattt atgagttaaa atcgatttta 9660
tagcgggcgc gatggtttat gtttataatt ttagtatttt gggaggtcga ggcgggtgga 9720
ttacgagatt aggagttcga gattagtttg gttaatatgg tgaaattttg tttttattaa 9780
aaatataaaa attagttggg tgtggttatg ggcgtttgta attttagtta tttaggaggt 9840
tgaggtagga gaattatttg aatttagaag gtggaggttg tagtgagtta agatttcgtt 9900
attggaagaa aaaaaaaaaa aattgatttt taaggaattt tttggtattt ttagtaaaag 9960
agtagtgaga taatagttaa ggattttaaa agagtttagt tttaaatata tattgatttt 10020
ggatgagttt tagatttcgt gtattttttt atttatttat ttttataatg ggaataggag 10080
aagtagttat gagtaattgt tattaaaagt ttatgtgttt ttggtagaaa tataaatgtt 10140
tgttgataac gattattttt tttagatttt ggtttatgtt tttttattaa tattttagta 10200
tttaattttt aggtttttaa gattttatga gtttgggttt ttgattgaaa aagattaagt 10260
tgagatttgg agagttatta gagttaattt tatgaagtat tagttagttt tttaattttt 10320
aaacgagata ttatatatat ttgtatcgta tttaattttt tgttgggatg atatttaatg 10380
tttgtgataa aaacgtattt aaggatatta ttttaaagga tttgtttttt ttggatttat 10440
tataggataa aatgtttttg atgttatttt ttaaaagata aatatttttt tttaaatgtt 10500
ttttttaatt ggttggttga ttgttttttg ggtgtgattg tacgggttag tttcgggtgt 10560
gtaattaagt tggaaaagtt tttttgattt ttgagtatta tgataaggag gagagtggta 10620
tatagtggat tgaaattata acgaagtatt gacgtgattt tggtattttg taaaaaatat 10680
tttttgtttg aggatattga tttatgattt tatgagtttt tttagttttg taggtttaat 10740
tattaaagat tagataggta agagatttaa tagatttatt ttgttagaga aggggaaaag 10800
aattagatta taggttagtg gaataattaa aagttttatt ttattcgtag gttgtaaaat 10860
gtttaagtat ttatatttat ttttttataa atgtgtatgt gtaaatgtaa ggtaatttta 10920
ggttttaagt agaaaaaatt gtaataaaag taattagtat tataggttta tttattagta 10980
tttttatgtg aaaattttta gggtaattgt aataattttt attttttaag aataaaatta 11040
attttttata tattaattta attttaagta tattttattt tttattaatg gtttgaatga 11100
tataattttt tttaaatgga ggtatttttt tgggtttagt tgtttaaatt ttagtttaaa 11160
tgtgatgata aatcgttttg tggtgaggtt tatagggaaa ggattttttt ttgtagtgtt 11220
tattaggatg atttacgaga cgtattagga aatcgagaga aatttagaga tattttttta 11280
agattaaaat tattgtgttt ataataagaa agcgtttgtt aaataataat ttttggaagg 11340
tgaggatttt attaaggtta gaatgtcgaa taataaattt ttgttaggtg attatttgtt 11400
tgagatgaaa attattttta ttttttagtt tgtttgaata tatgttattt tgtttgatat 11460
ttggtattat aaattgattt ttaggttatt aaatttatag agtatgaatt gttttattat 11520
gtagtgtttt tttttttttt aatgttgtag tttttttttt attttaattt tatgtattta 11580
ttgtatatgt tggtaatttt ttaattttga ggtttttttt tttttttagt ttttattatt 11640
ttatttgata tagtttattt ttgttatacg tttttaaatt ttaaattagt aaatgaaagg 11700
ttgagaaagt ttagtaattt taattttaat gtatgttttt tatttgaatt ttttatgtat 11760
tgtttttatt attagtgttt aatatttagt tttatttagt aaaaaaaaaa aaaaaaaaaa 11820
gaaaaagaaa agaaaagaag aaaattatag tgttttttgg gatgttagta atttattgtt 11880
gttgttttta attaataaga aaaaagatta tatttattta attaaagatt tttgttttga 11940
ttttaattgt taaaaataaa aatgttttga aagtatggta tttgtaaaat ttttgagaag 12000
atattaatgg gatttttatg atttaatata tgtaaggttt taattgtaat tgtaaattta 12060
ttgaaggttt atgatttgaa agtatgaatt attgtagtta gtttttatgg gtttaatttt 12120
attagtaaat tttagttttt atattttaat ttttaattag agtaaaataa cgatatattt 12180
ttaattaata attcgaaaga agaaagtatt ggaataacgg agttagaaaa ttattgttaa 12240
tagttatttt ttattacgaa ttattagtga atttcgaaaa gtataagatt gagtaatatt 12300
taagtttgtg aaatacgtgt attttattta taatatttat aaaattatat atgaattatt 12360
tttttttttt ttgatttatt ttttatataa ggtttaataa aatacgatat ttatttagtt 12420
atttttgatt gaatgattat tttttgtagt tatttattat tttttttttg ttacgttttt 12480
ttttatgatt taattttgtg atttttataa gttaggaaaa gttgttttta agttaatata 12540
gtataaagtt attgaaaatt ttgtcggtat taggttttat ttaggtgttt agaagttgtt 12600
ttgttttgtt ttgggaatgt tttatacgta atgttaatga ttgaaataga tttttttttt 12660
tttttcgaag ggaattgagt tattataata aaggatgatt tgtgtttaaa ataatattta 12720
atttattatt tttatttagt atgtatgtgt ttaagggttt ataattattt atatataaat 12780
gtaaaaatat tagtttttag gagatttatt ttgtttttaa aattttttat ttgttttttg 12840
ttttatatat tttttggttt taaaaattta gtattataag gtttgttatg ggatattagg 12900
tttaatttta ttttatagat aaattaattt taaaaaggta taattgaata tagtatatat 12960
tttataatta aatatatgta ggaataattt ggttttaaaa gaattttatt gtatgtattt 13020

attatatgtt tattgattgt tatataggga ttattaatgg aattaagaga aaatatagga 13080
ttttacgtat tgagtattaa taatttaaat gttttaaaaa atatttttta aaattgttgg 13140
ttttttattt ttttatttta agtgtaattt aatttatttt atgtttttta tgtaattaat 13200
aatttaaaag gtttttttgg tttggaaata ttttatgttt atttttaatg gaaagtatat 13260
ttaataattt ttttttgata aaaagtgttt ttattttttt ttcgttttat tatatttatg 13320
ttttataatt taaattttaa taatgtcgag atattttgaa atttggattt ttttttaatg 13380
tatttaaaat tttaaaagta ttttggattt attttcgata gaaattttat tacgttttta 13440
atatatgtat taagtttttt ttatttttat tattgttttt atttttcgtg aagagagaaa 13500
attaggtgtg agtaatgttt ttattgttat tattgttggt tttggttatt taagataaac 13560
gagggtgatt ttttcggttt gatttaagat aaacgagggt gattttttcg gtttgtgtta 13620
taaagataat gtattttaga attatttttt tagagggcga ttattgtaag taaagtttag 13680
tgaattcgag attttgttga gaattttagg atattgattt atttgtaaaa tttatagtgg 13740
agatagtagg aggttggata agttaattta aggtgagatg taaggtcgtt aagtttaagt 13800
ttgaaattta tatatattat tattttttga aataacgatt tagacgttga atttgtttaa 13860
gtgtttgaat ttttagtagg aaacgaggaa gcgttttttt tcgagtagga taattaggcg 13920
ttgcgcgagt agtttatttt taaggttttg tcgtttttat ttttattcgt gttatttata 13980
ttttgcgtgg tttttttcgg ttacgtcgta gatgtttttg taatgtttat cgtttgttga 14040
ggcgtaggaa aagaattttt taatttaatt ttttttttcg ttttcggatc gtaaagtata 14100
ggttatttat acggacgtga aagttatcga atttatttta tttttagcg gagcgtttta 14160
ttcgtatagg aatgtagggg tttgggtcgt acgattagcg acgggtatac ggagtagagg 14220
gaaagttttt taaagtaatc gtaggttttt ttggagagga tttcgtcgat tatgaaagtt 14280
ttgtagcgtc gtttggttgt tttgagttgg tcgggcgagt ttagttttag ttcggcgtgg 14340
tagtgtatgg tgagtcggcg cgagcgttcg tcggttcgag cgcggttgcg agtgttcggt 14400
tcgttggggt cgcggggttg gcgcgacggc ggggtttggg tttcgttttc ggtagttggg 14460
gaggcggcgg ggttcgggtg gcgcgcggag gagagcgggt agcgggcgcg tttgtcgcgc 14520
gtttagattt attcgtagat tagtattttc gttgagtttt tttgggataa cgtagttgtt 14580
aattagcgcg agttttttgt agggtcgggt gcggagtgtg tgttgagggg cgttcgcgtt 14640
tatttcgttt tcgttcgttc gtgttatttc ggcgtcgatt tgcgcgttta gtgttcggcg 14700
gtttttagtc gttgtttttc gttttggtgt ttcgcgtttt tttgtttggt tttttatttt 14760
aagacgttcg ttttggtttt tttttttttt tttttcggga cgcgttttag gtgtgtgtgt 14820
aggagtgtgc gcgcggtgcg gggagcgcgt atcgttttag ttcgcggtta aggtttaggt 14880
cggtagtttt tcggggtagt gttttttatg agtattttat tgtttgtttt gttattcgtc 14940
ggttcggttt ttagtgttcg tagtttagcg ggagggtacg tttaggtttt ttattagaaa 15000
gaagtttagt ttttattagg ttttcgggat ttcgggttat agagttagtg ttttcgagga 15060
tttatcgtgt tttttttttt tttcgcgtgg acggtttttc gaagatcgcg gttttttaag 15120
ttttttattc gtagtttcgt agttcgcgtt gagtttgcgc gtttcgagcg taagattttt 15180
gagttgaagt tgtaaatttg attgttttaa gattttaaat tataaatgat tttggtttcg 15240
ttcgttattt ttataaaaaa ttgagaaaaa aaaaaagata agaaaagaaa aattaataaa 15300
agtgtgagtt atcgtcgggc gcggtggttt aagtttgtaa ttttaatatt tgggaggtcg 15360
acgtgagacg atagtttgag gttagaggtt cgagattagt ttggttaata tagggagatt 15420
ttgtttttat aaaagaaaaa ttaaaaatag gtatggtggt gtacgtttgt agttttagtt 15480
attggagagg ttgaggtaag aggaaatttg tagttttagt tattggagag gtttaggtag 15540
gaggaaattt tgagtttagg agttcgcggt tgtagtgagt cgggatttcg ttattgtatt 15600
ttagtttggg cgataagagc gagattttga ttttttaaaa aagtgtgcgg ttttttttaa 15660
aatattgttt ataaaaatat ttttttttcg ttggaaaggg ttagagttgt acgattttag 15720
ggagtcggcg ggtattgggg tagtttgttt tatgcgggtt ttttttagcg ttcgagtttt 15780
tcggtaaagg ggtttttaat attcggattt ttcgcgatac gatcgggtta gagagtcgcg 15840
aagataggcg tttattgggt gatttgtttt tttttttttt aggtttttag tatacgttta 15900
gaggattaat tattaggttg ttagagaaaa tagatttttt tttttttttt tgagatcgag 15960
ttttattttg tcgtttaggt tggagtgtag tggcgtgatt tcgttttatt gtagtttttg 16020
ttttttaggt ttaagcgatt tttttgtttt agtttttcga gaagttggga ttataggcgt 16080
ttgttatcgc gttcggttaa tttttgtatt ttttagtaga gattaggttt tattattttg 16140
gttaggttgg ttttgaattt ttgatttcgt gatttattcg tttcgttttt taaagtgttg 16200
ggattatagg cgtgagaaaa tagatgttaa gaggttaagt tatagtttag tattagataa 16260
ggttcgtaac gcgtttttga ggaattttat tttcgaagta tttgtaaatt ggtttttttt 16320
tttttttttt ttatttagaa ttgttttttt ttattttttt tttttatgta gaggcggttg 16380
tagggtaggc gagaattgtt gttaggtgtt cgggtggcgt cgggggtttt gtcgtcggat 16440
tttggggttt ataggttggt tttaggcgcg ttttttgttt tcgcggcggg attttgaggt 16500
tggttcgcgg ttaaagcgcg ggattcggga tagtttatcg tttagttttg gtatcgtcgt 16560
tcgacgttac gacggagggg gttttttcgg gcgtaagtcg agagggaaat cgttgcgtcg 16620
agtcggggtt gggttttagg ggtttagcgt tcgttttcgg ggtaatgtcg ggcggggagt 16680
tttaggcgtt tcgaggaaag tgagtcgttg gtaggtagtc gttagtaaat tgagtgtttg 16740
cgggcgattt tattcgtagc gtcgattacg aggcgggttt ggagttgggg attagtattt 16800
gagcgatagt attaggattc gacgttcgac gaggggtcga gcgcgtcgcg tttatatttt 16860
ttatttgttc ggcgtgtgtt tatagaatgc ggtattattc ggggtttcga ttttgtattg 16920
tttttttttt ttttaatttt tttttttttt tttggttttt tttttttttt ttatcgttat 16980

tgattttttt tttttttttt tttttatttt tttttttttt cgtattgttt ttcgtttttt 17040
gggttttttt ttttttgtcg aatttttcgt ttttgttttt ttttttttgt tttttttttt 17100
ttttatcgtt tatttttgtt tttttttttt ttttttaatt tttttttttt tttttttttt 17160
tttttttttt tttaaattcg ggtttggatt cgcgttcgcg tgtttttggt cgcggtgttg 17220
tttttcggaa ggttgtaggc ggtttacgcg tagggataga gtttaagttc gggagggatc 17280
ggtttcgttt tggttggtcg cgtcgtttag cgtttttatt ttttttcgtt tttattcggt 17340
tataggtata aaatttataa aatttagata ttttcgtaga cgagtttttt gatttgaaat 17400
ttgtagtgtt ttaataggtg aagaagtaaa aattttaatt tatttttttg gattttttat 17460
tttttttatt tcgcgttata gaggtaagaa attttagaag gggtttgcgg gtataagttt 17520
tttgtgtagt ggagagggag agatgttcga ttttggagtt tagagttttg cgtttttttt 17580
tgttttttaa gttgagtttt agttttttta tttgtaaatg gtgattataa aatttatttt 17640
ataaggatgg gtttttataa tttatgtttt tatataagtt tgtttttttt tattggaatg 17700
ttttttatat tttatttttt ttttttttgt tttaaaattt agagatttat tttaaatgtt 17760
attttttttg tgaagtgttt ttttattttt taaggttgtc gatttttatt tttgtggtgg 17820
tatattttat attgtattgt tttttgtatg tttattttat ttattggata atgattaggt 17880
gggtttacgt tttatgttat attatttatt tgtattatta atatttataa agtatatagt 17940
gggtttttag taaatgagtt atgaataaat gaatgaatgg gaaaataata tgttagatgg 18000
gatattaaat tttaattagt tgtatttagt ttgttttttt ttttaaatga gtttttttta 18060
tttcgggtgt ttagtaatgg tatttagttt aatttttttt ttgtttttta tttttttttt 18120
ttttttttta tttatttttt tttgttttta gattgtaaag ggtttttgtg ggaggaagta 18180
tattttagtt gttaattatg aagtggggtt ttggttagaa tagtgagggg ttttgttttt 18240
aatattaatg aagtggttgg aatagtaaat atttgttaat aggatttaaa ttttttttag 18300
ttgttggaga tttatagata ttttttagtt ttttgtgatt gtatttaggt agcgtttgtt 18360
tttgtatttt ggggtggagg agtggggata tattatatac ggaggaatag gcgggtatgt 18420
tttgagttat atataggtag atatggaaag gataaaaatt tatatttttt atggagtttt 18480
aggggaagtt ttaaattttt agttttttat atttatattt ttattttagt atttgatttt 18540
tagggatata gtttaattag agagaagggg atagggatat ttttgttaag ttaggattgg 18600
aaattttaga tttggttgtt ttgttttttg ttaattggaa attaaagaat ttttttttta 18660
atattttaat ttatataagg tatttttatt tggttagaat ttacgaagtt taataaaata 18720
aaatggtttt taaaaatttt agggtgatat ttaagttgtt tttaggattt aagtgtagat 18780
gattttggtt tgatttttaa tttaattttt tttgtgtaat tttgggttat gtgtcgatag 18840
atatagatat ttagaagtag ttaggatatg ttttataagt atattaattt attttttaat 18900
tatttttttt ttaaagagta gagaggaggt aaaatatttt ttaaagttta gtaaagtgta 18960
tttaataatt tgtattttag tcgttacgtt ttgaaatagt tttagataag taaaagtaaa 19020
atagggaaag ttttgtaagt taatattttt ttgggtttta gggttatgtt aaaattgttg 19080
ttgtggagtt tagtatagta gttttaaata ataatttaaa ggaggtatat atggaggtta 19140
gtttgttttt aatgatgata ttaatttata aaaattgttt ttatatatta taaattattt 19200
ttttaagaga aagtataaat gcgatttatt tgataaatgg gtttggaaag gattgaatag 19260
attttttttt tttttttttt tagatttagt tttattttgt tgtttaggtt ggagtgtagt 19320
ggtataattt cggtttattg taatt 19345

<210> 35
<211> 5518
<212> DNA
<213> Artificial Sequence

<220>
<223> chemically treated genomic DNA (Homo sapiens)

<400> 35

gtataaatta tatttttaag agtggggaga atattgtaaa gtattttttt aagtggggtg 60
gaggggagaa tatattatat tagttagagt ggggtaggaa taaattatta tggtggaatg 120
ttattagtta aggttatttt tatttttttt gtggattttt agttgttttc ggttatttgg 180
atgtgtacgt gtaggttata ggggatatga cggtttagtt tgggtttaga ggtttgatat 240
ttattatatg gtagttggtt ttttttaaag gaagtgattt gaaagggaat aaggtggaag 300
ttgtagtggt ttttttgatt tgttttgtga aattatattt tgttattttt ataatatttt 360
tttggttata ttgtttagtt ttattgagtg tgggtagaga ttatatagta gtatgaatat 420
ttggaggtgt gattttttag gtgttatttt ggatgtgggt ttaatagttt tgatttagta 480
tttttattat tataaagagt ttggttgggt gcggtggttt acgtttgtaa ttttagtatt 540
ttgggaggtt aaggcgggcg gattatgagg gtaagagatc gagattattt tggttaatat 600
ggtaaaagtt tatttttaat aaaaatatta aaattagtag ggcgttgtta tacgtttata 660
gttttagtta tttaggaggt tgaggtagga gaattatttg aattcggagg cggaggttgt 720
agtgaattga gattatatta ttgtatttta gtttggcgat agagtaagat ttcgttttaa 780
aaaataaaat aaagtaaaat aaaataaaat atataaaaaa gagttttagt taggtatagt 840
gacgtatgtt tgtagtttta gtattgtggg aaattgaggt aggtggttta tttgagttta 900

ggggtttgag attagtttgg gtaatatggt aaaatttttt ttttataaaa tatataaaat 960
tagtagtgta tagtggtata tacgtgtagt tttagttatt ttagaagttg aggtgggagg 1020
atttttttgag tttaggaggt cgaggtggta gtgagttata gttgtgttat tgtattttag 1080
tttggacgat agagggagat tttgttttaa aataaataaa taaaaataaa aataaatagt 1140
tttgatttta taattagtta aaaagtgtat ttttttattt agtgattgta ttgtttagag 1200
gtgtatattt gtattaaaag tttttttttt ttttttttgt aattggagta gggaagggtt 1260
attttttgtt atgattggaa gttttttgag gtttttttag aagtagaagt ttgtgttata 1320
tttgttgtat atagtttgta gaattatgag ttaattaaat tttatttttt ttttttttga 1380
gatgaagttt cgttttgtta ttcgaggtgg agtgtaatgg tacgattttt gtttattgta 1440
gtttttgttt tttgggttta agtgattttt tggttttagt tttttaagta gttgggatta 1500
taggagtata ttagtacgtt tagttaattt ttgtattttt agtagagata gggtttattt 1560
atgttggtta ggttggtttt aaatttttga ttttaggtga tttgtttgtt ttggtttttt 1620
aaagtggtgg gattttaggt gtgagttatt acgttggtta aggatatggt tttttataga 1680
gatttgtttt tttaaattta taaattgttg gaaattttat tagtaaaaat gaaatattcg 1740
agttttgttg ataggattta atttatttga tatagagtag tagttttgat ttttaaagta 1800
ggtgtatagt tttagataaa gggtttttgg atgtaatatt ttatatgtat ttttttgttt 1860
ttagcgattt aggatattgg ttttatttta tagttttgat ttaatgttga ttttgttttg 1920
ttttaagtta ttatttggtt gttatttaag ttttattttt tttttttatt ttggtttttt 1980
tttttttttt ttttttttga gataggtttt ttttttgtta tttagtttga ttgtagtgat 2040
gtaattgatt atagtttacg gtagtcggga ttttttaagt ttaaataatt tttttatttt 2100
agttttttaa gtagttggga ttataggtac gtattattat atttagttaa tttttttttt 2160
tttttgtttt tttttttttt tgtttttttt tagatagagg tttgttttgt tgtttaggtt 2220
ggggtgtagt ggtacgattt tggtttattg taatttttgt tttttgggtt taagcgattt 2280
ttttgtttta gttttttaag tagttgggat tgtaggtacg cgttattatt tttagttaat 2340
ttttttgtat ttttagtgga gacggggttt cgttatgttg gttaggttgg ttataaattt 2400
ttgattttag atgatatatt tatttcggtt ttttatatag ttggtattat aggtgtgagt 2460
tattacgttc ggtttttttt tttttttttt gttttttagt tgatataggg ttttattatg 2520
gtatagttta ggttggtttt gaatttttgg ttttaggtga tttttttgtt ttggtttttt 2580
aaagtgttgg gattatagga atgagttatt atatttggtt ttttttttta atttttaaaa 2640
taaattgatt ttttaaggtt aagaggaaat attttttttg agaagttttt tttgaatgtt 2700
agaggtagat aatgtttgat ttttgtatgt tttttaatat ttaattatat agttattgaa 2760
taatatattt tgagagataa ttatgaatta agtaatatgt tggtttttgg gagaattgag 2820
gataaattaa ttttgtggaa attttgggtg gatgaaaaaa attaatatga aattaaaata 2880
ttgtatatat ttatagttgt gagaagtatt atatattttg ggtgttatgc gagttttttt 2940
tttttttttt tttttttttg agttggagtt ttttttttgt tattgaggtt ggagtgtaag 3000
gttacgattt tagtttattg taatttttgt ttttagattt aaacgatttt tttgttttag 3060
ttttcgagta gttgggatta taggtgtttg ttattatatt tggttaattt agaattttta 3120
gtagggatag ggttttatcg tgttggttag gttggtttta aatttttgat tttaggtgat 3180
ttatttattt cggtttttta aagtgttggg attatagaag tgagttattg agtttaatta 3240
ggagtttttt cgagaaagaa ggaagtttaa gagatttttt tgatatttta gtttgatttt 3300
gttttttgtt tgtttaaaat ttttttatgt tttttagcgg ttttttggat atagattaag 3360
tttttttttt gataggttta aattttttat tatttgattt tagtttattt ttttgagttt 3420
tttttagttg ttattatttt ttgtttaaag tgatatgtta taatatttat aaagtatata 3480
agttttatgt gtatagttta atgaattgta aatatgtgta tattcggtcg ggtatagtgg 3540
tttacgtttg taattttagt attttgggag gtcgaggtag gtggattatt tgaggttagg 3600
agtttgagat tagtttgatt aatatagtga aattttattt ttattaaaaa tataaaatta 3660
gttgggcgtg gtgtcgtatg tttgtaattt tagttatttg ggaggttgag gtaggagaat 3720
tgtttgaatt cggaggtaga ggttgtagta agtcgagatc gtgttattgt attttatttt 3780
gggtaataag agtaaaattt cgttttaaaa taataataat aataataata ataataataa 3840
taataatgtg tatatttatg taaatattat ttagataaaa atatggtata tttggggtat 3900
tcggggagtg ttttttgtgg tttttttttt ttatattttg ttgatttatt agtatagatt 3960
agtttttgtt attttttaaa ttttatattt tttttttttt tatataaata taaatattcg 4020
agttatgatt gggtggggtg gtttaagttt gtaattttag tattttggga ggttaaggtg 4080
cgaggatcgt ttgagtttgg gagtttagag attagtttgg gtaatataga gagattttat 4140
ttttatataa aaagttttaa aaattaatta ggggcggtgt agttttagtt atttaggagg 4200
ttgaggtggg aggttttagt tcgggaattt tagattgtag tgagttatga ttgggttatt 4260
gtattttagt ttgggtaata tagtgagatt ttgttttaaa aaaaaaaaaa aaaaaaatag 4320
gaaaaaataa ataaatagaa aagtaggttt ggcgcggtag tttatgtttg taattttagc 4380
gttttggaag gttgagacgg ggttattttt tgggtttata agttagagat aagtttgggc 4440
gtgggttata ttgttagatt taggtttttg taaaaaataa aattatttag tttttagtta 4500
tttataacgt tttgtttgga agtatgttat tttgggtttt tgagtttttg tatttgttaa 4560
ttttttttgc gttggggaga gtttaaattt tgttcgaaat ttttaaaaat ggtgtttgga 4620
taaatgaagg tattagagtt gcgattggac ggacggttgt tggacggcgt tattgttggt 4680
atttatcggg agatgtttat tggatagtta cgtgacggga ttaaattttt cgagggagcg 4740
aggtaggtgc ggttacgtga ttcggcggcg ttgcggggta gcggttattt tgcggggcgg 4800
ttacgtgaag gacgtacgtt tagcggggtt tttacgtgat tcgggcgcgt tgcggtcgtt 4860

cgcgcggatt cggcgagagg cggcggcggg agcggcggtg atggacgggt tcggggagta 4920
gtttagaggc gggggtgagg cgggaggtag acgggcggga ggagggcgag ttttttcgtc 4980
ggttcgttcg ggattttttt tatcggtttg gggttgtgcg atttttaagt attgaggggt 5040
agaaattttc ggatcgggcg ttgttagttt ttagtttttt tcgttttcgg aggtttttgg 5100
ttttttgatt ttcgtgtttc gatttttgtt tttttggcgt tttcggattt tcgagaatta 5160
ggggatttcg gaagttaagt tttcgggtag gttcgggttt gtcgttcgta ttattttttg 5220
tttttggtat tggtgggagg ggcgggtttc gtttttgttt ttttaggtta ggggtttgga 5280
tgtatatagc gtttttttag tttttttttt cggggagaat gtaggatata ggtttagttt 5340
tttggttttt ttttattagg gatttagttg tttgggtttt ttcgttattt tatttgttag 5400
ggttttagaa gtttagggtt ttagttttgt ttttttttag ggtcgtgagt ttttatagtt 5460
ttttgatttt ttagaattta agagtttagg tatttttttt tttttttttt tttttagg 5518

<210> 36
<211> 5518
<212> DNA
<213> Artificial Sequence

<220>
<223> chemically treated genomic DNA (Homo sapiens)

<400> 36

tttagaggag aaaggaaggg aagaggtatt tggatttttg ggttttaggg gattaggaga 60
ttgtggagat ttacggtttt gagggaggat agagttggga ttttggattt ttgggatttt 120
agtagataaa gtgacggggg ggtttagata attgagtttt tgatggagaa aggttaggag 180
gttgggtttg tattttatat ttttttcggg gaaagaggtt aggggaacgt tatatgtatt 240
tagatttttg gtttgagggg gtagaggcga gattcgtttt ttttattagt gttagaggta 300
ggaagtggtg cgggcgataa gttcgggttt gttcgggggt ttggttttcg agattttttg 360
gttttcgagg gttcgagagc gttagagagg tagggatcgg gatacgggga ttagagagtt 420
aggaattttc gaggacggag gggattggag gttggtagcg ttcgattcgg gagtttttgt 480
tttttagtgt ttggagatcg tatagtttta ggtcggtagg aaggatttcg gacgggtcgg 540
cgagggggtt cgtttttttt tcgttcgttt gtttttcgtt ttattttcgt ttttgggttg 600
tttttcggat tcgtttatta tcgtcgtttt cgtcgtcgtt tttcgtcggg ttcgcgcggg 660
cggtcgtagc gcgttcgggt tacgtgagag tttcgttgaa cgtgcgtttt ttacgtggtc 720
gtttcgtaaa atggtcgttg tttcgtagcg tcgtcgggtt acgtgatcgt atttgtttcg 780
ttttttcggg aggtttggtt tcgttacgtg attgtttaat gagtattttt cgataagtgt 840
tagtagtggc gtcgtttaat agtcgttcgt ttaatcgtag ttttaatgtt tttatttatt 900
taggtattat ttttagaagt ttcgggtagg gtttgagttt tttttagcgt agaaggaatt 960
agtaagtgta aaagtttaga ggtttaaaat agtatgtttt taggtaggac gttatagatg 1020
attaaaaatt gagtggtttt gttttttgta gagatttgga tttagtaata tagtttacgt 1080
ttaggtttgt ttttaatttg tgagtttaag agataatttc gttttagttt tttaaagcgt 1140
tgggattata ggtatgagtt atcgcgttag gtttgttttt ttgtttgttt gttttttttt 1200
gttttttttt tttttttttt tgagataggg ttttattgtg ttgtttaggt tggagtgtag 1260
tggtttaatt atggtttatt gtagtttgga attttcgggt tgaagttttt tattttagtt 1320
ttttgagtag ttgggattat atcgtttttg gttaattttt aaaatttttt atgtggagat 1380
gaggtttttt tatgttgttt aggttggttt ttgaattttt agatttaagc gattttcgta 1440
ttttggtttt ttaaagtgtt gagattatag gtttgagtta ttttatttag ttatgattcg 1500
aatgtttgtg tttgtgtaaa aagaaaagga atatgaagtt taaaaagtgg tagaaattaa 1560
tttgtgttga tagattagta gggtatggag gggaggggtt ataagagata tttttcgggt 1620
gttttaaata tgttatattt ttatttgaat ggtgtttata tgggtatata tattattatt 1680
attattatta ttattattat tattattatt ttgagacgga gttttgtttt tgttgtttag 1740
gatggagtgt aatggtacga tttcggttta ttgtaatttt tgttttcggg tttaagtaat 1800
tttttgtttt tagtttttta agtagttgga attgtaggta tgcgatatta cgtttagtta 1860
attttgtatt tttagtaaag atggggtttt attatgttgg ttaggttggt tttaagtttt 1920
tgattttaag tgatttattt gtttcggttt tttaaagtgt tgggattata ggcgtgagtt 1980
attgtgttcg gtcgggtata tatatattta taatttattg agttgtatat ataagatttg 2040
tgtgttttat gaatattatg atatgttatt ttagatagaa aataatagta attaaggaaa 2100
atttagaaaa atgagttagg attagatgat aaagggtttg ggtttgttag agaaggattt 2160
tgatttatgt ttagaaggtc gttgggaagt atggggaaat tttgagtagg taaagggtag 2220
agttagatta gggtgttagg aagatttttt ggattttttt ttttttcgaa aaagtttttg 2280
gttgggttta gtggtttatt tttgtaattt tagtattttg ggaggtcgag atgggtagat 2340
tatttgaggt taggagtttg agattagttt ggttaatacg gtgaaatttt atttttatta 2400
aaaattttaa attagttagg tgtggtggta ggtatttgta gttttagtta ttcgggagtt 2460
gaggtagggg aatcgtttga atttggaggt agaggttgta gtgagttgag atcgtgattt 2520
tgtattttag ttttagtaat aagagggaaa ttttaattta aaaaaaaaaa aaaaaaaaaa 2580
aaaagttcgt atagtattta ggatgtgtaa tgttttttat agttgtaaat gtgtgtagtg 2640

ttttaatttt atgttggttt tttttattta tttaaaattt ttataaggtt aatttgtttt 2700
taattttttt agaaattagt atgttatttg atttatagtt attttttaaa atgtgttatt 2760
taataattgt atgattgaat gttggggagt atgtagaaat tagatattgt ttgtttttga 2820
tatttagaga agatttttta gaggaggtat ttttttttga ttttgaagga ttagtttgtt 2880
ttgaaaattg aagaaagggg ttaggtgtga tggtttattt ttatagtttt agtattttgg 2940
gaggttaagg taggaggatt atttgaagtt aggagtttag gattagtttg ggttgtatta 3000
tggtgagatt ttgtgttaat taaaaaataa aagaaaggag ggggggtcgg gcgtggtagt 3060
ttatatttgt aatattagtt gtgtgggagg tcgaagtggg tgtattattt gaggttagga 3120
gtttgtgatt agtttagtta atatggcgaa atttcgtttt tattaaaaat ataaaaaaat 3180
tagttgggag tggtggcgcg tgtttgtagt tttagttgtt tgggaggttg aggtaggaga 3240
atcgtttgaa tttaagaggt agaggttgta gtgagttaag atcgtgttat tgtattttag 3300
tttgggtaat agagtaagtt tttatttaaa aaaaaataaa aagaaaagga agtagaaaaa 3360
aaagaaaatt agttggatgt ggtggtgcgt gtttgtagtt ttagttattt gggaggttga 3420
ggtgggaaga ttgtttgagt ttgggaggtt tcggttgtcg tgagttgtga ttaattgtat 3480
tattgtaatt agattgggtg atagaggaag agtttgtttt aaaaagaaaa aagaaaagaa 3540
aaagttaaga taaaggggga gggtagagtt taggtgataa ttaaatgata gtttagagta 3600
aagtaaggtt aatattggat taggattgtg aagtgaaatt agtgttttga atcgttggaa 3660
ataagaaggt atatgtgaaa tgttgtattt agaaattttt tatttgaagt tgtgtatttg 3720
ttttggaaat taaggttgtt attttgtatt aagtggatta gattttgtta gtaagattcg 3780
gatgttttat ttttattgat agaattttta ataatttatg aatttagaga ataaagtttt 3840
tgtaagaaat tatgtttttg gttagcgtgg tggtttatat ttgaaatttt attattttgg 3900
gaggttaaga taggtagatt atttgaggtt aggagtttga gattagtttg gttaatatgg 3960
tgaaattttg tttttattaa aaatataaaa attagttggg cgtattggtg tgtttttgta 4020
attttagtta tttgggaggt tgaggttaga gaattatttg aatttaggag gtagaggttg 4080
tagtgagtag agatcgtgtt attgtatttt atttcgggtg atagagcgag atttttattt 4140
aaaaaaaaaa aaaatgaggt ttaattggtt tatggttttg tagattgtgt atagtaagtg 4200
taatataggt ttttgttttt ggggaggttt taggaagttt ttagttatgg tagaaggtgg 4260
ttttttttta ttttagttat agagaaggaa ggaaagggtt tttaatgtag gtatatattt 4320
ttgaatagta tagttattga gtgagggaat atatttttta gttagttgtg aagttaaaat 4380
tgtttatttt tatttttatt tatttatttt gagataggat ttttttttgt cgtttaggtt 4440
ggagtgtaat gatataattg tggtttattg ttatttcgat tttttgggtt taaggatttt 4500
ttttatttta gtttttggag tagttgggat tatacgtgtg tgttattatg tattgttaat 4560
tttgtatgtt ttgtagggag agggttttgt tatgttgttt aggttggttt taaatttttg 4620
ggtttaagtg agttatttgt tttagttttt tatagtgttg ggattatagg tatgcgttat 4680
tgtgtttgat taaaattttt ttttgtgtgt tttgttttgt tttgttttgt tttgtttttt 4740
gagacggagt tttgttttgt cgttaggttg gagtgtagtg gtgtgatttt agtttattgt 4800
aattttcgtt ttcgggttta agtgattttt ttgttttagt tttttgagta gttgggatta 4860
taggcgtgtg ataacgtttt gttaattttg atatttttat tagagatagg tttttgttat 4920
gttggttagg atggtttcga ttttttgttt ttatgattcg ttcgttttgg ttttttaaag 4980
tgttgggatt ataggcgtga gttatcgtat ttagttaaat tttttataat aataaagatg 5040
ttgaattaaa attattaagt ttatatttaa gatggtattt gaggagttat atttttaggt 5100
atttatgttg ttgtgtggtt tttgtttata tttaataggg ttaaatagtg tgattaagag 5160
gatattgtgg aaatgataga gtgtgatttt ataggatagg ttagaaaagt tattatagtt 5220
tttattttgt ttttttttag attatttttt ttaaggaaag ttagttgtta tgtggtgagt 5280
gttaggtttt tgagtttaag ttaagtcgtt atattttttg tgatttgtac gtatatattt 5340
agatggtcgg aagtaattga agatttataa aagaagtgaa aatagtttta attgatgata 5400
ttttattatg gtgatttgtt tttgttttat tttaattgat atgatatatt ttttttttta 5460
ttttatttaa gaaggtattt tgtaatattt tttttatttt tgagaatgta atttgtat 5518

<210> 37
<211> 6561
<212> DNA
<213> Artificial Sequence

<220>
<223> chemically treated genomic DNA (Homo sapiens)

<400> 37

gatggtttta atttgtatgt atatagtaat ataaatttgt aattttttgt tgtttttata 60
tatatttttt tatttatttt ttataatttt tatgagaagt taattgttag tttttataga 120
gtatttatga gggagtcgag gttatgagaa tttgttattg ttatagatta gaggttttta 180
attttcgggt tatgaatagt atttgtttat ggtttgttag gaatttttag gggtaggtga 240
gggagtatta ttgtttgagt tttgtttttt gttagattag tagtagtatt agatttttat 300
aggagtttaa attttattgt gaattgtatg tatgagggat ttaggttgta tgttttttat 360
gagaatttaa ttaatgtttg acgatttgac gtggaatcgt tttattttga aattattatt 420

tttaatttgg tttgtagaaa aatttttttt tataaaattg gtttttggtg ttaaaatggt 480
tggggatttt tgttttagat tgattgattt aattggtggt ttttttgatg aaatatttat 540
tgtttggtaa tataataaaa gttgggagaa gagaaggaga aggaggagaa agaggaggag 600
aagaaaggta tttaaagttt tattttttaa aattaggtta tattgtagat agaaggttaa 660
agaatatatt tgaagatata tagagtgagt agtaattaga aattaagttt atatatgtta 720
tttatttgat ttttattatg ttagtttgaa gtaagtaaat atatatttat ttattttaat 780
taatagatga aatgattatt ttaaagttag ttatttatga tagaattagg aattagaatt 840
taggttattt aattatattt tagagtgtta ttaatatagt ttatagatat aatttaaggt 900
gtatatttta aattgtgtga tagtttaatt aatgtataaa atttattttt tttaattttt 960
aagaagatgt agttggtatt tgttgaaagt agtgattttg atttggttaa tttggtttag 1020
tttttgatat aaattatttt atttttttta ttttttttat tatgtttaat taaatattaa 1080
aaaggaaaat atttgattga ttaaatgttt tttttttgtt ttggttttaa gtttttgtaa 1140
aaaaaatttt atttaggtaa agtaagtagg gatggttatg ggtgttattt ttgtaataaa 1200
gcgatatgta tgttaaaaag taaagagggt tttttagggt agttgttata ggtttttatg 1260
gaatgataat tataaggtag atggattacg taatgtttgt tgtgaatttt ataatatatg 1320
gggattatta ttttaatgtt gatttgtata atgtttggtt tagtaagaat aatattgaat 1380
gttaatagtt atagaatgta ataaaatgga atattatgtg agttgaagtg ttaaaattgt 1440
taaagtatga ttgtaagtag ttaattttat ttcgagtttt gtttatatat aattaaatgt 1500
aaatttttat tattttttga gaaattgtta agagatgatt gaaatggaat agtaataatt 1560
ttttttatgt ggaattatta tttggttatt tttgtttttt tatataattt gtttgtattt 1620
tttgatgtat ttgaatttgt aatttttatt atttttattt taaataattt tttattttta 1680
tttttgtttt ttttatattt gttggtagat gtattaagtt tgataaattt agtaagattg 1740
aattttattt tttgaattga aaaattttga atggattttt gaaaacggta gaattgataa 1800
tggttagttg taagtgatat ttttaaggta aatagatatt tttttaaagt attaaataat 1860
ttagtatttt aagttgtagg tggaaggtag ttattagtga agagagagaa aaaaaaaaga 1920
aatagttcgt ttgtatttag atttattatt tttgattatt gttttttttt ggaaaacggg 1980
taggtatagt tattttgtat ttcgatttta aattagtttt tggagattat ttatttattt 2040
atttatttat ttatggattt ttttttttta agcgttcgaa tttattttta ttataagagg 2100
gtagttattt ttaaaaaaaa aaaaataggg ttaaaattta tgtaagttgt gtttggaata 2160
agtatttagt agttttttag aaattatata ttttatataa aagagatttt gtaatgggta 2220
gtattaatat gaaatagtgt ttagaagtat ttattttttt ttagatttta aattgataag 2280
gtttttattt attaggttat gaagttttaa agttgtaaga tatttttgag gttattatag 2340
gatatttatt tatttttttt tcgggtgtat ttaatagtta ttaatttttt ttttttttta 2400
aaagttattt aaatttattt gaagtttgt tttgttttgt ttttgaaatt taagtaatga 2460
gagaaataat tgttaatttt ttaattaaat ttgataggaa aggaaataat tttagaagtt 2520
ttgtgtttat gagtaggata tgttttattg tttttttgtt tgcggtgtaa tgattttgag 2580
tgataattaa tttttatagt attttttttt ttttttttag gaaataaagt agtatgtttt 2640
tgaataattt ttttattttt ttttattttt ttggtagtta ggtttttttt aaaatatttt 2700
atttgatttt tatattttta gaaatagtaa gtgtttaatt cgtttttgtg ggttgttaat 2760
tcgatttacg tgagcggaat ttagtattat tttagttttt ttatcgaaaa aataatatat 2820
atggataata gttttattat tagtttttgt ttttgatttt tttttttttg tttcgtaggt 2880
tcgatagttt tgggaaagta gaatttggtt ttttttaaaa attttttgtt tttggttttg 2940
gggattattt gggtaagttc gaggtgttgt gtatgggggt ttttggaatt ttgggaaggg 3000
tagaaagttt tggttttaga tttatcgtgt agtagttttg agtagtattt cggttgagga 3060
gtgattttag tgaatattta gttgaggagt gatttggtta cgtgttatag ttttattttt 3120
tgggggtttg gtggaagagg gtggcgtaga aggttttaag gttttaaatt ggaattgttt 3180
tgtatgtttg gtttatatag tgcgttattt tatttttttt tgagttgtta atcgtttgtt 3240
tttgagttgg gtgagataaa tattataagg tataaagtga ttgtataata aaaaaattaa 3300
atttttttta tttatttttt agtttgttat atacgtagtt tacgttatat atatgttacg 3360
taaagtagga tgatatttat gttatatata tagatatatt aatcgaaatg tggtttttcg 3420
gttgtatata tttttatata tgaatatatt tatagaaata tatgtatata tttttgtata 3480
ttggatatat ttatgtaatt ataaatttat atgcgtatgg atatgaaaat aaatgtatat 3540
atatttatgt aaaaaaattt gtatatatgt atttatatat gtaaatatat atatttttat 3600
gtattaatgt ttaaaaatat ttaattttta gtttgttgtt tttttttaat ttttttttta 3660
tttcggggaa atagaagcgt ggattttacg tttatgttat gttaaaatac gttgtaattg 3720
aggtgttttg ttttgttttg ttttttgaaa tcgtatatta tcgaaaaatt ttaaattgaa 3780
agttgaataa cgggtttagc ggggaaataa gaggttagat tttgattttg tatttgtttt 3840
ggatttcgtt tttagagttt tcgcgagggt tcggcgcgtt agttgatttt ttttttgaga 3900
gtagggagtg gaggcgcgag cgtttttttt ggcggtcgcg cgttttcgtt tttcgtttta 3960
tttcgtcgcg gttgttcggg cgcgtcgttt atatttttgc gcgtagtttt cgttcgttcg 4020
gggattttcg gcgagtcgcg tcgcgaaggg ggaggtgttc ggtcgcggtc gggagggagt 4080
cggtaggcgg cgtttttttt aaaagtcgcg agcgtcgcgt tacggcgtcg tcgtcgtcgt 4140
cgtcgtcgtc ggagttttcg tttcgtcgcg ttgcgttcgg ttcgcgttgc gttagtcgtt 4200
tcgtttttta tatttcgtcg gggattggta gtcgtcgtcg tatatttgtc gttatagttt 4260
tcgtcggtta ttcgaacgtt ttcggggtta gcgtcgagtg gattatcggg gatcgcgagg 4320
tattcgcgcg tcgtagattt cgcgcgggtt ggagtattcg gtagagcgcg ttatagcgtc 4380

gtggtttttg ttgttcgggt tgcgttagag tcgcggacgg gcgcgtagag cgtcggggat 4440
ttcggagtcg atttttagcg tcgcgatgcg gagtatttat tgtaggagat cgggggtttg 4500
ggacgcgttg gtcgaggtgt gatcggattt taggttagtt ataaagggta tttggtttta 4560
gggttaggag agcgagggga gagtatagtt attcgtttcg gcggttcggg attcggttcg 4620
attcgtcgga gaatgcgttc gaggacgacg gggcgttaga gtcgcggtgt ttttaattgg 4680
cgagcgcgaa tgggggtgta ttggagtaag gtagagtgat gcgggggggt aattcgtttg 4740
gtatcgagat cgtcgtcgtg tttttttttg gattcggcgt cgtttaggat ggttgtttcg 4800
agttatgggt cgcggcggag ttagcgcgga gcgttcgatt ttcgattttc gagtttcgga 4860
gtcggtttcg cgcggggtta cgcgtttttc gggcgttggt ttttaaggag gacgatagta 4920
ttagtttttt tttttttttt tttttttttt gtttcgtatt tttttttttg ttcgttgttg 4980
ttgtgtgtta gtatttggtt ggggattttt tgaatttgta gggagaataa tttgcgtatt 5040
ttattttgcg tcggtgtttt tgttttagcg gagtttgttt cgttattttc gagttttatc 5100
gtttttttat ttttcggttt tgttcgatat tgagacgttg tttttagcgt gaaaagagag 5160
attgcgcggt cggtattcgg gagaaggagg aggtaaagaa aaggaacgga tattcggttt 5220
ttgcgttagg ttttttgatt agagtttttt tatgtggacg tttttttaat ggacgtgttt 5280
tcgcgtgttt tttagacgga ttgcggtttt ttaaaggtag aggacgcggg ttagggttcg 5340
gggtgggtgg tgggtgggag ggggatttgg gtagttattg cggtagagtt tttttttacg 5400
tttaggttag aagtaaatag attttttttt agtttacgtg taacggagtt ttgtaggggt 5460
ttttattttt agttgtttcg ggcgatcgta agttttattt tttcggttcg tattttttta 5520
tttttttttt tttttttttt tttggaatat ttttggagtt gttaatattt agatgaggtg 5580
ttttatttat ttatttattt atttttaatt tttttaaaaa tttttttggg ttaaagaaat 5640
ttttttgaga gggtagtttt tgggttttat tcgttagttg agaatttgtt cgttttgtta 5700
tggtgatttt tattttttaa gtgttttcgg gagatttggt ttttttgttt agagtcgtgt 5760
tttatttagg aaagtattag gagtttgggg ttttttttat ttgtttttag aaatgcgagg 5820
ggttagtatt gaaggattat ttggtattgt gtttttaata gttgatacgt gtattaatag 5880
atatttatta tttacgtaat ttcgggaaga tatatgtgta ttttgattgt attgtgggga 5940
tgcgggatgg agttgttttt cgagatattt ttgagggtag gggtttggga tataagttat 6000
aagtggtttt agaagttgtg gttttgagtt tatagggttt ggaagttata agggtgtgtg 6060
tgtgtgtgtg tgtgtgtgtg tgtgtgtgtt aggaagtttt atatagtgtt tttaaggaag 6120
ttatatgtat tatttatgtg tgtttatatg ttagatagcg tttagtattt cgtatttggg 6180
tttgtatagg ggacgtaggg tgttagatta agcggtggtt tttttaggtt ttcggtattg 6240
gttgttagcg ttgtgttata tataaaaaag tgatagttat tggcgttggt ttggttgggg 6300
gggagggtaa attttaaatt tgatgttaga cgagttaagc gttggatggg agcgataaat 6360
tatttggttt aggaatttgg gattttttat tattttaaac gtttgagttt cggtcggttt 6420
tatttagatt cgtgagtgtg ttaagttagg agggtatttt ggaggaggta cgttagttaa 6480
atgggagatc gggtcgcggg ggcgcgaggg gggaggattg ggcggggaat tcgggtgatt 6540
tacgtcggtt ttgttcgtag g 6561

<210> 38
<211> 6561
<212> DNA
<213> Artificial Sequence

<220>
<223> chemically treated genomic DNA (Homo sapiens)

<400> 38

tttgcggata ggatcgacgt gagttattcg agtttttcgt ttagtttttt tttttcgcgt 60
tttcgcggtt cggtttttta tttggttggc gtgttttttt taggatgttt ttttggtttg 120
gtatatttac gagtttaggt aagatcgatc gaagtttaaa cgtttgggat aatgaagggt 180
tttaagtttt tgaattagat gatttatcgt ttttatttaa cgtttagttc gtttgatatt 240
agatttggga tttgtttttt tttttaatta aattagcgtt aatgattgtt atttttttgt 300
gtgtgatata gcgttgatag ttaatgtcgg gaatttggga aaattatcgt ttgatttgat 360
attttgcgtt ttttatataa atttaaatgc ggagtgttga gcgttgtttg gtatataaat 420
atatataaat ggtgtatgtg atttttttag aggtattgta tagaattttt tgatatatat 480
atatatatat atatatatat atatatattt ttatagtttt tagattttgt aagtttaagg 540
ttataatttt tgaagttatt tatgatttgt gttttaggtt tttattttta ggggtgtttc 600
gaaaggtagt tttatttcgt atttttatag tgtagttaag atatatatgt attttttcgg 660
gattacgtaa atggtgaata tttattaatg tacgtgttag ttgttaaaaa tatagtatta 720
agtgattttt tagtattgat ttttcgtatt tttggaaata agtagggaga attttaaatt 780
tttagtattt ttttaaatgg gatacggttt tgagtaaaga aattaagttt ttcggaaata 840
tttgaagaat ggagattatt atggtagagc ggataggttt ttagttaacg ggtgaaattt 900
aggggttatt tttttaaagg gatttttttg atttaaaaaa gtttttaaaa aaattaaaaa 960
taaataaata aataaataaa atattttatt taagtgttaa tagttttaaa agtattttag 1020
ggagagggga agaaagaggg gtggaagagt gcgggtcggg agggtgaggt ttacggtcgt 1080

tcggggtagt tggaagtggg agtttttgta gggtttcgtt gtacgtggat tggagagggg 1140
tttgtttatt tttggtttgg acgtaaggaa gggttttatc gtagtggttg tttaaatttt 1200
tttttatttt attatttatt tcgggttttg gttcgcgttt tttattttta ggagatcgta 1260
gttcgtttaa gaagtacgcg gggatacgtt tattgaaaga gcgtttatat ggaaaaattt 1320
tggttaaagg atttggcgta aggatcgaat gttcgttttt tttttttgtt tttttttttt 1380
ttcgggtgtc ggtcgcgtag tttttttttt tacgttggga atagcgtttt agtgtcgggt 1440
aaggtcgagg agtggagggg cggtggggtt cggagatggc gaagtaggtt tcgttggggt 1500
aaaggtatcg gcgtaaagtg gggtgcgtaa gttatttttt ttgtaagttt aagaagtttt 1560
tagttaagtg ttgatatata ataatagcga gtagggggag gagtgcgggg tagggaaggg 1620
aagggagaaa ggagaagttg gtgttgtcgt ttttttaggg aattagcgtt cgagggacgc 1680
gtggtttcgc gcggggtcgg tttcgggatt cgggggtcga gggtcgggcg tttcgcgtta 1740
gtttcgtcgc ggtttatggt tcggggtagt tattttgggc gacgtcgggt ttagggaagg 1800
gtacggcggc gatttcggtg ttaggcgagt tgttttttcg tattattttg ttttatttta 1860
gtgtattttt attcgcgttc gttagttgaa agtatcgcgg ttttggcgtt tcgtcgtttt 1920
cgggcgtatt tttcggcgag tcgagtcgag tttcgggtcg tcgaggcggg tggttgtgtt 1980
tttttttcgt ttttttagtt ttggggttaa gtgttttttg tggttagttt ggggttcgat 2040
tatatttcgg ttagcgcgtt ttaggttttc gattttttgt agtaggtgtt tcgtatcgcg 2100
gcgttaggga tcggtttcgg agttttcggc gttttgcgcg ttcgttcgcg gttttggcgt 2160
agttcgggta gtagaggtta cggcgttgtg gcgcgttttg tcgggtgttt tagttcgcgc 2220
ggggtttgcg gcgcgcgggt gtttcgcggt tttcggtgat ttattcggcg ttggtttcga 2280
gaacgttcgg gtagtcggcg gaggttgtgg cggtagatgt gcggcggcgg ttgttagttt 2340
tcggcggggt gtgggaagcg gagcgattag cgtagcgcga gtcgggcgta gcgcggcggg 2400
gcgaggattt cggcggcggc gacggcggcg gcggcgtcgt ggcgcggcgt tcgcggtttt 2460
taaaggggac gtcgtttgtc ggtttttttt cggtcgcggt cgaatatttt ttttttcgcg 2520
gcgcggttcg tcggggattt tcgagcgggc gggagttgcg cgtaggggtg tggacggcgc 2580
gttcgggtag tcgcggcggg gtggggcgga gggcgggggc gcgcggtcgt taaggggggc 2640
gttcgcgttt ttattttttg tttttaaagg agagattagt tggcgcgtcg gattttcgcg 2700
gggattttgg aggcgaaatt taggataaat gtagggttag ggtttggttt tttatttttt 2760
cgttgggttc gttatttaat ttttagtttg aagtttttcg gtaatatacg attttaaaaa 2820
ataaaataaa ataaaatatt ttaattatag cgtatttggg tatagtatag acgtgggatt 2880
tacgtttttg ttttttcgga ataggaggaa aattaaaaga aaatagtagg ttggaaattg 2940
agtgttttta aatattaata tatagagatg tatgtattta tatatgtaaa tgtatgtgta 3000
taaatttttt tatataaatg tgtatgtatt tatttttata tttatacgta tgtaaattta 3060
tagttatata aatatattta atatataaaa atatgtgtat atatttttat aaatatattt 3120
atgtatgaga atatatgtaa tcgaagggtt atatttcggt taatatgttt atgtatgtga 3180
tatggatgtt attttgtttt acgtgatatg tgtgtaacgt agattgcgtg tgtggtagat 3240
tgaaggatgg atgggaggga tttgattttt ttattgtata attattttgt gttttgtgat 3300
atttatttta tttagtttag aggtaggcga ttagtagttt agaggaaggt aaaataacgt 3360
attgtgtgaa ttaagtatat aggataattt tagtttggga ttttggaatt ttttacgtta 3420
ttttttttta ttaggttttt aagaagtagg gttgtgatac gtggttaagt tattttttag 3480
ttgaatattt attgaagtta ttttttagtc gaaatattgt ttagagttgt tgtacgatga 3540
gtttggggtt aaggtttttt gtttttttta ggattttagg agtttttatg tatagtattt 3600
cgggtttgtt taaatgattt ttaaaattaa gggtagaaaa tttttggaaa aggttaagtt 3660
ttgttttttt agagttatcg ggtttgcgaa atagagagaa aaaagttaga agtaggagtt 3720
ggtaatagaa ttattattta tgtgtattat tttttcggta ggggagttaa aataatatta 3780
ggtttcgttt acgtaaatcg gattagtaat ttatagaggc gaattaggta tttgttgttt 3840
ttaaaggtat aaaggttaaa taaggtattt tggaggaagt ttgattatta ggaaaataaa 3900
agggggtggg ggaattattt aggaatatgt tattttattt tttgaaaaaa aaaaaaaagg 3960
tgttatagaa gttgattgtt atttagagtt attgtatcgt aaataaggag gtaataaaat 4020
atattttatt tatggatata gggtttttga aattattttt ttttttatta agtttaattg 4080
agaagttaat aattgttttt tttattattt agattttaaa aataaaataa aataaaattt 4140
taatgagatt taagtagttt ttaaagagga ggaaaagttg ataattattg gatgtattcg 4200
aagaaaaaat aaataaatat tttgtgatga ttttaaggat gttttgtagt tttagaattt 4260
tataatttgg taagtggaaa ttttgttagt ttagaatttg agggaaaatg ggtatttttg 4320
aatattgttt tatgttagtg ttgtttattg tagaattttt tttatgtagg gtgtatgatt 4380
tttgaggaat tattgaatgt ttgttttaag tataatttat ataaattttg gttttatttt 4440
ttttttttta gagatggttg tttttttgtg gtggaaatga gttcgaacgt ttgaaagaaa 4500
gaagtttata aataaataaa taaataaata agtagttttt agagattgat ttgggatcga 4560
agtataggat gattgtattt attcgttttt tagggaagag taatagttag aaatgataaa 4620
tttaaatata gacgagttat tttttttttt tttttttttt tttattaatg gttatttttt 4680
atttgtaatt tagaatgttg ggttatttaa tattttggga gagtgtttgt ttgttttgaa 4740
aatattattt gtagttaatt attgttaatt ttatcgtttt taaaggttta tttaaagttt 4800
tttagtttag agggtgagat ttagttttgt tgagtttatt agatttagtg tatttgttag 4860
taaatgtgga aggggtagag gtgggagtgg ggagttgttt aagataggga tagtagaagt 4920
tgtaaattta aatgtattag agggtataga taagttatgt aaaaaagtaa agatgattag 4980
ataatgattt tatataaagg aggttgttgt tattttattt tagttatttt ttggtagttt 5040

tttaggagat agtggaaatt tgtatttgat tgtgtgtgga taagattcgg gatgaaatta 5100
attgtttata gttatatttt agtaatttta gtattttagt ttatatgata ttttatttta 5160
ttgtattttg tagttattag tatttagtat tgtttttgtt aggttaaata ttatgtaaat 5220
taatattaaa atagtgattt ttatgtattg taaagtttat agtaggtatt acgtgattta 5280
tttattttgt aattgttatt ttatggaaat ttgtggtaat tgttttgaga aatttttttt 5340
gttttttagt atatatatcg ttttgttata agggtgatat ttataattat ttttatttat 5400
tttatttgga tgaagttttt tttataggaa tttggagtta aggtaaaaga gaagtattta 5460
gttaattaaa tgtttttttt tttggtgttt gattagatat ggtagaaagg atgggagagg 5520
tggagtgatt tatattagaa attaggttaa gttggttaaa ttaggattat tgtttttagt 5580
aagtattaat tatatttttt taaaaattaa aaagaatgaa ttttgtgtat tgattagatt 5640
gttatatagt ttaagatgta tattttaagt tatgtttgta aattatgttg ataatatttt 5700
aagatgtagt tgggtgattt gggttttaat ttttggtttt gttatggatg attgatttta 5760
aaatggttat tttatttgtt aattggggta aataaatata tgtttgtttg ttttagattg 5820
atatgatgaa ggttaaatgg ataatatgtg tgaatttagt ttttgattgt tatttatttt 5880
atgtattttt aaatgtattt tttggttttt tgtttgtaat atggtttggt tttaagaaat 5940
aggattttgg atgttttttt tttttttttt tttttttttt tttttttttt ttttttttag 6000
tttttattat attgttaaat agtggatatt ttattagaaa ggttattagt tgggttagtt 6060
aatttaaagt agaggttttt aattattttg gtattaggga ttagttttgt ggaggaaaat 6120
ttttttatag attaggttgg gggtgatggt tttaggatga aacggtttta cgttagatcg 6180
ttaggtatta gttaggtttt tataaggagt atgtaattta gattttttat atatgtagtt 6240
tatagtaggg tttgagtttt tatgagaatt taatgttgtt gttgatttga taggaggtag 6300
agtttaggta gtaatgtttt tttatttgtt tttgggggtt tttaataggt tatggataag 6360
tattatttat ggttcggggg ttggggattt ttgatttatg gtagtaataa atttttatag 6420
tttcggtttt tttatgaata ttttatagag attaatagtt gattttttat ggaggttgtg 6480
aagaataaat gagaagatat gtgtgaagat aataaaaggt tgtaaatttg tattattata 6540
tatatgtaaa ttaaggttat t 6561

<210> 39
<211> 6636
<212> DNA
<213> Artificial Sequence

<220>
<223> chemically treated genomic DNA (Homo sapiens)

<400> 39
   ttttatttta gatatgtttt ttataaagat ggggtttttt tttggagaaa gaaattagaa 60
   gtattgtatt taatatgtaa aaaattattt gttattgttt ttaatatgga gggggaataa 120
   atagaaaaaa tggtaagtgg tttttagtag ttgataataa gttttggtta aaagttagta 180
   agaatatgga atcgcggttt tataattata tataaggaat ttgattttgt aaaaatttga 240
   ataagtttgg tggaggattt ttatttagag tttttcgata agaatattat ttggttaata 300
   tattgaatgt gatattttga gtagagaacg tggttgagtt ttattgaatt tttaatttat 360
   agaatggtga gataataatt gggtgttgtt ttaagttttt aagtttgtag taatttgtta 420
   tgtagtaata gaaattagat ttatttggag tggggaaaaa ttggaagtta tttaggagat 480
   taaaaatata gtttagatta aaataaatta ataattaaat gaaaggagtt gtaatagaaa 540
   taaagagaat aaatgaaaga aataatagag atagttttta tatgatttag taattaattt 600
   atgttgttta gagagaagga gaaggtgaat agaagtttaa gatttggaac gtggatggtt 660
   aaaaggataa gagtgttaat ataaaggttt ggaaattaag aatataatta agatggatga 720
   ttagttttat tttagatgta ttgaatttat gatattaata ggataaatat agtttataaa 780
   aattggggcg gggggagttg aaatattgta gagatttaag gttagaaata tttgtggagt 840
   tattggtaat taaatataag tatataattt aagtgagtta attaaagaaa taatggagag 900
   aaagattggt atgaatttgg aagaggattt tagaagaaag atttttttta tttaagtgta 960
   gatgatattt tttttttgtt tgaaatagtt tatttaaaat attttcggaa aattatttat 1020
   tttgttttta aattttagtt taattattat tttttttatg tagtattttt tgttttattt 1080
   agttagggga aaaaaaaata tttttaagtt ttttaagtgg ttatttttat tttttaagta 1140
   gattaagttt ttaggggata ggaagtattt tattttattt ttgtattttt aatatgaaat 1200
   tgttaagaat ataatagata ttaaatatgt attgagtgaa tgaattaagg atgggagtaa 1260
   taaaaaagag gaaggaatta aaattttata attgttaata tagttttata taagtattta 1320
   ataataatgg ttgatgggaa gtttttagaa aataaatgta tattatttaa gaatattaat 1380
   aaagataagt ataatatata tatatttata tatatatata tagggttttt atagttaaaa 1440
   gtagagttta atgtaataga atttattaat ttattaattt gttgttgttt tttgttttag 1500
   aaatgttttt ttttaatttt ttttgtgttt gggaaaaaag attgataagt attttttgat 1560
   ttagtagttg gttataatag ttataatttt taatataaaa taggaaagat ttattattta 1620
   aaaattaaaa taatttttaa ttaatgtaat ataatagaga gattaaatat aaggtacgta 1680
   tattattaat atgtttaatt tagttttttt tgaaaagttg taaatttttt atatattaag 1740
   gaaattagat gtttagttag aaatatgaat tttatgattt aaattatttg agtgaaggta 1800
   ttttaagatg ttagtatttt gaaataattt aatttattta gagttgggat tttgtagttt 1860
   ttgataaaag aaatattgtg ttattgtttt gaatattata agatggtatt tttaggtgtg 1920
   ttgatgaggg aattgttttt tttttattta atttgttttt aatttaattt tttaagaagt 1980
   tattttaaga gtttgggagg ggtgttattt atttgttatt gttggatatg ttgttgttgg 2040
   ttttatttat tttatttatt gttttttttt tttggatgta tgttagttgt aaatgagtta 2100
   ttataagttt aaaatttggt tgttattagt gttttttggt ttgttggttt ttatatttat 2160
   attttgtttt agtttggaat tatagttggg aatttttgtt agggattatt ttatttgttt 2220
   attatttttt gttattatga ttattttgaa tgatatggtt ggttattaaa atataaagat 2280
   gggtatgttt tgatttttgt tttagataaa aatagataag cgattttttt aggttttagt 2340
   cgattttttt taagtatttg ttatgtgtta ggtattttgt taattttttg aaatggatta 2400
   tttaattatt ttttattata attttgtgag atggtatttt tattttatgt ttatggttta 2460
   aataatagat tggaggtgta gtaaagggaa gggagttatt gtagatatgt tgttagtttt 2520
   tagtatttat gaatgaagaa ttatgtggag tatagagtgg taaatgttgt gatagaaata 2580
   tgtattgtag ttaaagggag ggaagtttta aagatggttt taaaggggaa gaggatgaat 2640
   ttttttggta aggaggattg gagttgaggg ggatttagaa ttatatattt aaaggttaga 2700
   ggtgattatt aaagtagaag aattgaaaaa gaattttttt ttggatttgg tattttttaa 2760
   gggtagagta aagatgttag gaacgtttga ggtaggttta gaaagaggag gtagttgata 2820
   gaattgagaa agggtagaaa ggaatggaaa aataattaat agtttatagg gaaaaaatta 2880
   aagagagagt ttttagggtt tattgaattt tgtaattaga tttttattat taattataaa 2940
   agttattatg ttttttatgt taagtaaaga ttaaatatat gattttattt aatttttata 3000
   attatttttg aaatatgtgt tattatttgt agaatagttt tcggggtggt taagggtaaa 3060
   atttaaatcg tagtgggtta agaagtgatt gagtggggag aatgtagttt ggatttggga 3120
   taaatttagg atagaaaggg attgagtttt tattaaaggt tagtgagtgc gttagttatt 3180
   ttagtattgt tttttaatgt tatttaaata aaatttaaaa aggaatgtgt tgttatttat 3240
   tattaaagaa gaaatttaga tttggagata tggtgtgatt ttaggagttt attttttttt 3300
   cgattaattt tattgtttta tagaggggaa gagggataaa tgtaatagat attgattgaa 3360
   gattttattg tatatggtta agaattgtat taaatattta tatggattgt atattgaaat 3420
   tttagtttaa atgttaattt tattattaat agtgtggtta tttggtttga ggttagaaat 3480
   tagatttttt gaatttggat tttagttttg ttatttatta tgtatttttt tgttttagtt 3540
   tttttatttg taaaatgggt tttgttgtga aaattatttg gtaatatatt tgaaatataa 3600
   ataataaggt atgtttagag tataagtgtg gagaaaaatg ttagtttata gttgtttttt 3660
   gtttgtttgt ttgttttttg ttttgagata aagtttgttt tgttatttag tttggagtgt 3720
   agtggtataa tttcggttta ttgtaatttt tattttttgg atttaagtta tttttttgtc 3780
   gtagtttttt tagtagttgg gattatatgt gcgtattatt acgtttagtt aatttttgta 3840
   tttttagtag agatgaggtt ttattatgtt ggtaaggttg gtttcgaatt tttgatttcg 3900
   tgatttattc gttttggttt tttaaagtgt tgggattata ggtttgagtt attgcgttag 3960
   gttgtttata gtttaatttt tattgtatag ttatgataag agagaattag gaagataaag 4020
   ttagcgattt ttatattttg tttcggattt cgggattttt tatatatttt tgtttttagt 4080
   tattattgta ttattttgtt gggaaatttt ttggggattt gtaggaagga aatttttggg 4140
   ggaagttgta ggtggggttg tggtgtgttt gtttgttttt ttatttgttt ttaatatgat 4200
   attgattcgt atatattttt tatttcgaga gaagatgatg cgggagagag gggcgttatg 4260
   atgggtatgt ttttagaaag ataggaagtt atgatgacgg gttgtaggtg taggggttcg 4320
   tagtgttcgt tagaagaggt tcgggaaagt aagcgagaat ggtgtttacg ttgtgatttt 4380
   gtttggaatt ataagatcga aaagttagga agaagttttg tttttttttg tttttttgtt 4440
   agttattata tagttattgt ttttggagaa ttaaaaaaaa aaataaataa aaataaaata 4500
   aaataaaata aaaaaatcgt tttttttatt atttttttag ttttgaattt tttttttttt 4560
   tttttttttt agttattttt atttttattt tttttttttt ttttttcgtt gttagttttt 4620
   tggttatttt tttttttttt tgtttttatt ttagtttggt gtaagttaag agggcgagga 4680
   cgagggagtg ggagagattg gttgaggatt ttatagatta gttagagagc gaaagaatta 4740
   agtttttttt ttttatatat atatatatac gtatatacgc gcgcgcgcgc gcgtatatat 4800
   atatatacgt atattaaaaa attcggatta gtcgcgtcgt agttgtttta atttttggaa 4860
   aaggtaatcg agcgtttttc ggatcgttgc gtatagtttc ggtttcgatt tggcgtttaa 4920
   aatagagtta gttttagttt ttcgcgcggt tagtttggtc gggtgttttt aaaaataaag 4980
   cgaggaggga aggtatagat agattttgaa aatattcggg ttatatacgt cgcgatttat 5040
   agtttttttt tagcgttgga gtggagacgg cgttcgtagc gttttgcgcg ggtgaggttc 5100
   gcgtagttgt tggggaagag tttttttgtt aggttgcgtt gggttagcgt agtaagtggg 5160
   gttggtcgtt atttcgttgt attcggtcgc gtttcgggtt tcgtgcgttt tcgttttagt 5220
   tggtttggag tttaattttc ggtttcgtcg tcggtttttt gcgttttcgg agtgtttcgt 5280
   agcgacgtcg ggagtcgacg cgtcgcgcgg gtatttagtt atggttgggg cgagtaggtt 5340
   gttttttttg tggttgggtt gtttttgcgt gagtttggcg tagggagaga gatcgaagtt 5400
   atttttttcg gagtttcgta aagttgtgtt aggtgatcgt acggtaggtg gtggttcgga 5460
   tttcgagttg tagtcgtaag ataaggtttt tgaatatatg ttgcggtttt atgataggta 5520
   tagtacggtt taggcggttc ggatatcggg ttttttggag ggaggttcgt agttttggcg 5580
   ttttcggttt ttgcgcgaag gtaatacggt tcgtagtttt cgggcggtag tagtaggtga 5640
   gtgcgcgagg tgagattttt ttttcgcggt ttcgttttag ttttttttcg ggatttttta 5700
   tagttttttt gtttgttttt tgtttggtgg cgttgtttag ggattttttt tcgttttttt 5760
   tagttgtttt ttggattttt tcgtttagtt atatttcgcg tgtcgttagt tgtatttttt 5820
   gtaagtttag tttttaaatt taaagtgaga gggggaaata gaatgaaggc gcggagtagt 5880
   ggaggggtag gttggaggag tttcgtggaa aaagggaagg ggcgatattt tatgtttgtg 5940
   aggttttttg ttttgagtga ttggagtatt tttagggtat tttattttgt tgatttttaa 6000
   atcggttagc ggaaattttt ttggattggg ttttagttat gtttattaag aggtagatgt 6060
   tattttatgt taatgttagg attcgagaga aattatattt gaataagacg tttttttttt 6120
   tttttttttt tttttttagt cgttttttta gatattgtat tcgtttttaa ttcgcgtttt 6180
   ttgtgttatt gtattatatt tgatatttta tttgatagtt ttttagtggc gtaaaaaaaa 6240
   aaaaaaaaaa aaaaaaattc ggggttgggg ggggttttaa tattaaaaat ttattggggg 6300
   aaataggggg ggttttattc gggggggggg ggagttggga aataaggggg gggggtattt 6360
   ttggatgaaa atatttttgg gaatttgttt aaaaaaattt gtttatttta tttttaatta 6420
   taaattattt tggattgaaa tttatggttt aataaggttt tttttagttt ttgtggtgaa 6480
   gggaataatt tatgttaata gttttttttt tttgtgttaa tagtttgagg gaaagttttt 6540
   tggatattaa ttttgttttt atgttttgag gatttattag gtttttttgt ttattatatt 6600
   tatgataagg ggaaatgtaa ttgtaaagtt tgtttt 6636

<210> 40
<211> 6636
<212> DNA
<213> Artificial Sequence

<220>
<223> chemically treated genomic DNA (Homo sapiens)

<400> 40

aagataggtt ttgtagttat attttttttt attatgaatg tagtgaataa aaaagtttgg 60
tgaattttta aagtataaaa atagagttag tgtttaggaa gttttttttt aagttgttaa 120
tataagggag ggaggttgtt agtataagtt atttttttta ttataggggt taggagaaat 180
tttgttaagt tatgaatttt agtttaaagt gatttgtggt taaaaatggg atgagtagat 240
ttttttgggt agatttttaa aggtgttttt atttaggggt gttttttttt tttgtttttt 300
agtttttttt tttttcggat gggatttttt ttgttttttt taatgggttt ttaatattga 360
aattttttta gttttcgggt tttttttttt tttttttttt ttttgcgtta ttaaggaatt 420
attaagtaaa gtgttaaata tagtatagta atataaaggg cgcgaattag gagcgggtgt 480
aatatttgga gaagcgattg gggaaggaga aaaggagagg ggaaggcgtt ttatttaagt 540
atgatttttt tcgggttttg gtattggtat aaaatggtat ttgtttttta gtaaatatgg 600
ttaaagttta gtttaaagga attttcgttg gtcgatttag gaattaatag aataaggtat 660
tttagaggtg ttttagttat ttagggtagg aggttttata ggtatggggt gtcgtttttt 720
tttttttttt acgaagtttt tttagtttat ttttttattg tttcgcgttt ttattttatt 780
tttttttttt attttggatt tgaaaattgg gtttatagga gatgtagttg gcgatacgcg 840
gggtgtgatt ggacggagga atttagaggg tagttgagga gggcggagaa aggtttttag 900
gtagcgttat taagtaaggg gtagataagg aagttgtggg gggtttcggg agaaagttgg 960
ggcgggatcg cgggaaggga gttttatttc gcgtatttat ttgttgttgt cgttcgaaag 1020
ttgcgaatcg tgttgttttc gcgtaggagt cgagggcgtt agggttgcga gttttttttt 1080
agggagttcg gtgttcgggt cgtttggatc gtgttgtatt tgttatagag tcgtagtatg 1140
tgtttagaga ttttgttttg cggttgtagt tcggagttcg ggttattatt tgtcgtgcgg 1200
ttatttggta tagttttgcg gagtttcggg aaaggtggtt tcggtttttt tttttgcgtt 1260
aggtttacgt agaagtagtt tagttataga aagagtagtt tgttcgtttt agttatggtt 1320
aggtattcgc gcggcgcgtc ggttttcggc gtcgttgcgg gatatttcga aggcgtaagg 1380
agtcggcggc ggagtcgagg gttgaatttt aaattagttg gggcgagggc gtacggagtt 1440
cgggacgcgg tcgggtgtag cgagatagcg gttagtttta tttgttgcgt tgatttagcg 1500
tagtttgata ggggggtttt tttttagtag ttgcgcggat tttattcgcg tagggcgttg 1560
cgggcgtcgt ttttatttta acgttgagaa agagttgtag gtcgcggcgt gtgtggttcg 1620
ggtgttttta agatttgttt gtattttttt ttttcgtttt atttttggga atattcggtt 1680
aaattggtcg cgcgagggat taggattagt tttgttttgg gcgttaggtc ggagtcgggg 1740
ttgtgcgtag cggttcggag ggcgttcgat tgtttttttt agggattgga gtagttgcgg 1800
cgcggttggt tcgagttttt tagtgtacgt gtgtgtgtgt gtgcgcgcgc gcgcgcgcgt 1860
gtgtgcgtgt gtgtgtgtgt atgagagaga gagatttagt tttttcgttt tttagttgat 1920
ttgtagagtt tttagttaat tttttttatt ttttcgtttt cgttttttta gtttgtatta 1980
ggttgggata gaagtaaggg agggggggag taattagaga attggtagcg ggagggagag 2040
aagaggaagt aggggtgggg atggttgggg agaggaaggg aaaaggagat ttaggattaa 2100
agaaatggta ggagggacgg tttttttgtt ttgttttgtt ttgtttttgt ttgttttttt 2160
ttttaatttt ttaagaatag tggttgtgtg gtggttagta agagggtaga ggaaaataga 2220
attttttttt ggtttttcgg ttttatgatt ttaaataaag ttataacgtg aatattattt 2280
tcgtttattt tttcgagttt tttttgacgg atattgcggg tttttgtatt tgtagttcgt 2340

tattatagtt ttttgttttt ttgagagtat atttattata acgttttttt ttttcgtatt 2400
attttttttc gaggtgagaa atatgtgcga gttaatatta tgttaaaaat aaataagaaa 2460
ataagtaaat atattataat tttatttata gtttttttta aaggtttttt ttttataggt 2520
ttttaaaagg ttttttagta ggatggtgta gtggtggtta aaaataaggg tgtgtggaag 2580
atttcggagt tcggaataga atgtaagagt cgttagtttt gtttttttaa tttttttttg 2640
ttatggttgt ataatggaga ttaaattatg agtagtttgg cgtagtggtt taggtttgta 2700
attttagtat tttggaaggt taaagcgggt ggattacgaa gttaggagtt cgagattagt 2760
tttgttaata tggtgaaatt ttatttttat taaaaatata aaaattagtt gggcgtggtg 2820
gtgcgtatat gtaattttag ttattgggaa ggttgcggta agagaatggt ttgaatttag 2880
gagatggagg ttgtagtgag tcgagattgt gttattgtat tttagattgg gtgatagagt 2940
aaattttgtt ttaaaataaa aaataaataa ataaataaaa aataattgtg agttaatatt 3000
tttttttata tttatgtttt agatatgttt tgttatttat attttaagta tattattaaa 3060
taatttttat agtaaaattt attttataga tgaggaaatt gaggtaaaag gatatatagt 3120
aagtgataga attgagattt aaatttaggg agtttgattt ttggttttag gttagataat 3180
tatattatta atagtgaaat tagtatttaa attgaagttt tagtgtgtaa tttatgtaaa 3240
tgtttagtgt agtttttgat tatatgtagt aaaattttta gttaatgttt attgtattta 3300
tttttttttt tttttgtagg gtaatggagt tgatcgaaaa gaagataggt ttttggagtt 3360
atattatgtt tttagatttg agtttttttt ttgatggtga ataataatat atttttttta 3420
gagttttgtt tggatgatat tagaaagtag tgttaaagtg attggcgtat ttattagttt 3480
ttaatagggg tttaattttt ttttatttta aatttatttt aaatttagat tatatttttt 3540
ttatttagtt attttttaat ttattgcggt ttggattttg tttttagtta tttcgggaat 3600
tgttttgtag ataataatat atattttaag agtagttgtg aggattaaat gagattatgt 3660
gtttgatttt tgtttggtat aaagaatata atagtttttg tgattaataa tgaagattta 3720
attgtaaaat ttaataggtt ttggaaattt tttttttgat tttttttttg tgaattattg 3780
gttgtttttt tatttttttt tgtttttttt tagttttgtt agttgttttt tttttttagg 3840
tttgttttaa gcgtttttga tatttttatt ttgtttttgg aaaatgttaa atttagaaga 3900
aaattttttt ttagtttttt tgttttggtg gttatttttg gtttttaaat atatagtttt 3960
gggttttttt tagttttagt tttttttgtt aaagaagttt attttttttt tttttgaaat 4020
tatttttgag attttttttt ttttagttat agtgtatatt tttgttataa tatttgttat 4080
tttgtatttt atatgatttt ttatttatag atattgaaga ttgatagtat gtttataatg 4140
gttttttttt ttttgttgta tttttaattt gttatttaag ttataagtat aaaataagag 4200
tgttatttta tagggttgta gtaaagaata attggatagt ttattttaag agattagtag 4260
agtgtttagt atatagtaaa tgtttaaaaa gagtcggttg agatttaaga gaatcgtttg 4320
tttgtttttg tttaaggtag agattaaggt atatttattt ttatatttta atgattagtt 4380
atgttattta gaataattat agtaataggg gatgatgagt aagtagaata atttttaata 4440
gagattttta gttgtaattt tagattagaa tagaatatgg atatgggagt tagtaaatta 4500
gagaatatta gtagtaatta aattttggat ttataataat ttatttgtaa ttaatatata 4560
tttaggaaga gggagtaatg gatgaaatgg atgaagttag tagtagtatg tttagtaatg 4620
ataaataaat ggtatttttt ttagattttt ggaatgattt tttgggaaat tgaattgaaa 4680
gtaaattaaa tgggaaaaaa ataatttttt tattagtata tttggagatg ttattttgtg 4740
atatttaaga tagtagtata gtgttttttt tattaaaggt tgtagaattt tagttttgga 4800
tggattaaat tgttttagaa tgttggtatt ttgagatgtt tttatttaaa tggtttaaat 4860
tataggattt atatttttgg ttgagtattt ggtttttttg atgtatggag gatttgtagt 4920
tttttaggag gaattgaatt gaatatattg gtagtatacg tgttttgtat ttgatttttt 4980
tgttgtgttg tattaattaa gagttgtttt aattttaaaa taataggttt tttttatttt 5040
atgttagagg ttgtgattgt tatagttaat tattgaatta gaggatattt attaattttt 5100
ttttttaaat ataaggagag ttaaaggaaa gtatttttaa aatagaaaat aatagtagat 5160
tagtggatta gtggattttg ttgtattaaa ttttattttt gattataaaa attttatgtg 5220
tgtatgtatg aatgtatgtg tattatgttt gtttttattg atatttttga gtaatatata 5280
tttatttttt aaaaattttt tattaattat tattattgaa tatttatgta agattatatt 5340
agtagttgtg aagttttggt tttttttttt tttttattat ttttattttt gatttattta 5400
tttaatatat atttagtatt tattgtgttt ttgataattt tatgttgaga atataaagat 5460
aaaatgaaat gttttttgtt ttttgaaaat ttagtttatt tgggagatag gaataattat 5520
ttgaaaaatt taaaggtatt tttttttttt ttggttgagt aaggtagaga gtgttgtatg 5580
ggagagatgg tagttgaatt gagatttaaa agtaaagtag gtggtttttc gggggtattt 5640
tagataggtt attttagata gagaaagaat attatttata tttaggtggg agaagttttt 5700
tttttggggt ttttttttaa atttatatta attttttttt ttattatttt tttagttaat 5760
ttatttgagt tatatgtttg tatttagtta ttaataattt tataagtatt tttaatttta 5820
agtttttata gtattttagt ttttttcgtt ttagttttta taaattgtgt ttattttgtt 5880
gatattataa atttagtgta tttaaaatga aattggttat ttattttaat tatatttttg 5940
gtttttaaat ttttatgttg gtatttttat ttttttagtt atttacgttt taagttttag 6000
atttttattt attttttttt tttttttgag taatataaat taattgttaa attatataga 6060
ggttattttt gttatttttt ttatttattt tttttatttt tattgtaatt ttttttattt 6120
agttattaat ttattttaat ttaggttatg tttttagttt tttaaatagt ttttagtttt 6180
tttttatttt agatgaattt gatttttatt gttgtataat aaattattat aaatttagag 6240
gtttaaaata atatttaatt attattttat tattttgtag gttagaagtt tagtggggtt 6300

tagttacgtt ttttgtttag gatgttatat ttaatgtgtt ggttaggtgg tatttttatc 6360
ggaaggtttt gggtagaaat tttttattaa gtttatttag gtttttgtag aattaaattt 6420
tttatatgta attgtaggat cgcggtttta tatttttatt ggtttttagt tagggtttgt 6480
tattagttat tagaagttat ttgttatttt ttttgtttgt ttttttttta tattaaaagt 6540
agtaataaat aattttttgt atgttgaata tagtattttt aatttttttt tttagaaaga 6600
aattttattt ttgtaaagag tatgtttgaa gtgagg 6636

<210> 41
<211> 8453
<212> DNA
<213> Artificial Sequence

<220>
<223> chemically treated genomic DNA (Homo sapiens)

<400> 41

agaaaaagag aaggagaagg atgtatgagt ataaaataaa gaaattttaa ggtttaattt 60
tttttatatt ttttaaaata atttattttt aaagagtatt tttgttgtag aattaaatga 120
gtgtagtcga ttattttttt aggagaggaa gttgagaata gttaaaattt aaatatttgt 180
tattgtatgt taattttatt ttgattattt tttagaaaag attttttata tttatgtttt 240
gtttgttatt tacgttaaat atgttatgat ataaaaatag tttttttatt tgtttgatat 300
tattgggggt ttatttagaa gttttgtttt attttggatt tttgtttgtt tgttgttgtt 360
tttgttgttg tttttgagat agagttttat tttgtcgttt aggttggagt gtagtggcgt 420
gattttagtt tattgtaagt ttcgtttttt gggtttacgt tatttttttg ttttagtttt 480
tcgagtagtt gggattatag gcgttcgtta ttatgtttgg ttaatttttt atatttttag 540
ttaagatggg tttttattat gttagttagg atggtttcga ttttttgatt tcggtattta 600
tttgttttgg ttttttaaag tgttgggatt ataggtgtga gttatcgtat ttggtttgga 660
tgttaatatt gaagggtttt tatagaaagg attatatttt agaaagtatt aaggtagtaa 720
tttaaaatat agggaattga aatagtattg gatttttttt ttgttatgag aaaatattag 780
tatttagtat tatttatttt gagtgaggta tattaaaggt tggtaataat attaaatgtt 840
tagagaagga ataatttgtg taattgggtt aaaagtttat ttttagatta gaataaagaa 900
aatgagttta tgttttagtt tgagggatag gaaaaaaaat ttttaaaagt tagaggtgtt 960
aggaattcgt aaaatagacg gagaatgttt tttggatatg ttatttagtt ttagtttatt 1020
agtaaaaatt aattgtttag agtaggtatg attttgatag tgagtagtta ttttggtata 1080
gttatgttta tgggttagat ttatatagat aaagtataat gagtggatat tggagattaa 1140
gtaatttgat tgtgatttta attattagga tatattgttt ttgtatttta gagtttgaga 1200
aagatttgtg aggattttgt ttaagtttta tattttataa tgaggaaatt tggatttagt 1260
agagataatg agtttttttt ttaagattat aatttttttt ttaggaaagg ttatataaaa 1320
gttttgtttt aaggcgggtt tatatagtaa cgttaggaag aaataaattt ttaggttttg 1380
attttgtttt gttatttaag aatttgggaa gatattttga aatttttggt aatagtatgt 1440
tgttaggatg gagtagtagt tggtgttttg gtttggggaa agtttttagg ataagtttcg 1500
gggatttatt tataatattt tttatttgtt tagggagagg agatagattg atatggtttg 1560
gatttggggt ttattatttt ttagttttgt gattttgagt aatttatata acgtttttaa 1620
attttagatt tttatttgta ggatagggat tttataaggg ttgttgtaag ggttagagat 1680
atatagggta aatatataag atatgataaa tattaataag aggaaaatta aatttttagt 1740
taattttaga gattattttt aattttaatt cgaattcgaa ttttaatttt aattgattta 1800
ttttattgtt ttttaaaatt tagtagtgat tttgggaatt tttttcgtag ttttattaga 1860
tatttaattg gttaagaagg tttgaagatt tgatgtggtt tttaattggg gatggggaat 1920
taagggttat tgtatttata ggattatttt tttatttgta tagatttatt tggtgtgttt 1980
agggtatagt gatattataa ttgttatatt taatagttta taaagtttaa gtttagtata 2040
ttttttgttt gtttaatgat gttttggtat tagtttttta atggtattta ttagtataga 2100
aaatggaaat aaaataattt ttaaaatagt agtttttaag ttttagtgtg tttagaatga 2160
ttagagaatt ttgtgaaata tatagatttt tgggtttaga tttggggtag gattaggaag 2220
tttgtatttt atttgtattt ttattttatt ttgaggttta tagttttgag aatatgtttt 2280
gaaaaaggtt gttttaaggg ttcgtagata ggttattgat tagttatttt tttttgatgt 2340
tttttaggaa aaatttaata aaggaatgtt ttttattgag tagtagtagt ataggagtaa 2400
tagttgtttt tgaattatgg tgggtttttt ttttttatta atgtgtttta agggtatagt 2460
tttatttggt ttatttatta tgttttataa aaatatgaaa atttataggt aagtttgaga 2520
tatagaaaat aattaaattg atttttttta cgaattttga ttattaggtt gtggttgatt 2580
tagtttttta attaataagt aatttgtttt ttggtatgag taagggggga aaaggaggag 2640
tgggtaaaag tagttgataa tagatggttt gcgtttattt aaaatgtggg gagagaaata 2700
aagttgtttt aagagaatta aagttgagtt ttttcgttat atatttgaag atttatatta 2760
ggggtttaaa tatggtatgt cgggtagttt aattggaaat ttttggattg tgagtgttat 2820
agatttatgg atgggttaat tagtggttat tttagtgttt gggttgtagt aaaatgagat 2880
aatagttgtt atttaaaaat ttttggaatt aaaaaaattt cgaaatgata ttggtgtttt 2940

aaagtaaaat aaagttttgt ttttaagttt agtatattat tgttgttttt gagtttaaat 3000
attaagaatt atatttcgtt aatgattaaa ataatagtga ttgatttagg ggtttagtga 3060
gtatttaatt tgttttgatt ttaggtatta tgttaaagga gtataatgtt tgatgttgta 3120
ggagaaatat taggtaatta tttaatggag ttttaatttt ttgttattat ttttaataat 3180
taattgtgat tttgattatt tggaagttat aggtatattt tgtttttttt ttggggtggt 3240
gttattgttt tgttttgttt taattagtgg ttttagagaa aagtgaattt aggagtgatt 3300
taaaatgaag gaagacggat tttggttaaa attataatta aataattaaa ttatttttaa 3360
atataaaggg agtatgtaga tgatttggtt taattttttt atttgtagaa tgagaaaatt 3420
gagatttata ggaatgaaaa gatttgttta aagttatata gtttgttttt gttgtttggt 3480
gtattaggtt aaaagattta ggtttaatag atggaaaaga tggtaggatg ttttggtttt 3540
gttttgatag ttgttttttt gattttagat attttttatt ttttgtaatt tgtgttttat 3600
ataggaagta gtttttgttt tttaaatatc gaaggtgtat aaacgtaaag tttttataga 3660
tgagttattt agggttaata tttgtttaag taaagattta aatgttttgt agagatagta 3720
aagtgttatg tttgttttag ggaaagaaat ttaggatagg aaatgtttag ttttttagta 3780
tttttttggt tatttggagt ttaggttatg agttttaatt tttttttgaa gtattagttt 3840
tggagtagag gttgtgattt attttagaga tttgggtaag tttttttaat ttggtagttt 3900
tgtttttttt tgtttgtaaa atagagagat gaggttgata gtttttttat agttttatta 3960
gaggtagtgt gtgaaattag tttttgtttg ggaaggttta aaagttatta tattttattt 4020
ttttgttaat atgattatta aaatgttttt atatgaaagg gttaattttt tatttttttt 4080
ttttttttaa aaatagatta aggggtattt ttttttgttt ttttgtggtt taaaaggtta 4140
ttgtttttgt ggttattttt ttggaaagat agagtgttag gatttttagg tatattaaaa 4200
atgaataaaa aaattaataa taattataat attaataaaa ataattgttg tgtcggtttt 4260
taagacggtt tttgagttag aaatagattt ttttaattgt aaaaaacgtg gttttagttt 4320
gtttgtaggt tatttttgtt tttaggtttt ggggggagga gggaagtgag tttatttatt 4380
ggggtttatt ttagggttat tattaaggtg ttttataaaa cgtattttaa gaatgttttg 4440
gaaggaaatt tattttttaa tagtttaaga ggtatttttt tttggtatat agttttgtat 4500
atagtttgtt ttttaacgtt tggaaatttt ttaatagttt atggaaggtt attttttaaa 4560
tcgatttaat agtttttttt tttataattt gattttagag gtgttttatt atttttaatt 4620
atttagggta aatggtgatt atttagtgtt ttaattatta gtttgggtag tagttatatt 4680
aaatttaggg aagtttagat ttttatgggt atttttggaa ggtacggcga ttagtcggtg 4740
tatgtttttt agtattttcg tacgtggttt ttaggtgagt tttagtcgtt ttttagagtt 4800
ggaggtagcg gcgttttagt ttcgacggta gttgcggatt cgggcgttgt ttgggttttc 4860
gggattcggg tttgttaggc gaggtcgggc ggttggaggg gaggatgtgg gcggggtttt 4920
tatttttaga aagggaggcg agcgagggag gagggaagga gggaggggtc gtcggggaag 4980
aggaggagga aggaaagaaa gaaagcgagg gagggaaaga ggaggaagga agatgcgaga 5040
aggtagagga ggagggaggg agggaaggag cgcggagttc ggttcggaag ttaggtgagt 5100
gtggtattcg agttgaggga cgcgagtttg agacgtcgtt gttgtttcgg ttgagtattt 5160
agtttgtttt ttcgatggga ttttcgttta agttatttcg agtttgtagc gttatagttt 5220
tcggttttcg tttaggttta ttgtaatcgt ttagaggttt ttaggagttg ttgttggcga 5280
gttcgttatt gtagggattt atggtgagta aggttatttg gtgaggggag ataggtagag 5340
ggggtttagg agtttttttg gggggaagaa gttggttata ggttgtgatc gaggtaaaag 5400
gtggtttaat tattttttaa tagtggtgtt ggaggtgggg atgttggcgt tgaaagattt 5460
ttaaatatcg gttattgttt ttgtttaggt tttttttgtt tagtagtttt tgggagattt 5520
ttatttttgg gaagtgcggg gtaggagagt agaaataaga gaagtttttg gtaggggggt 5580
cgttgggaaa aattgtgggg ttttgggttg aacgcgttgt ttacgggttg gaggttgcga 5640
ttttcggacg gaaagcgcgg gaggaggaag gagagaatcg gttttgaggt ttagagagag 5700
tgagggggta gagcgacggc gagatgggga gagaatattt agttggagta ggttttgcgg 5760
tagagagcgt agttttgttg gtttttggag agtgcgcgtc gtttcggagg ttgcgtcgag 5820
gggagtgtta tttaatttgg tttttagttg gcggggcgtt ttgagagttt gcgaattgta 5880
gttgtaggac gcgttttttt tacgagttat tttcgtcgat ttgcggaatt taaggaattt 5940
cgtttttatt attttacggt gtagggtttt tagagacgat agttaagatt ttaggggttt 6000
ttaggacgtt tgtttttgcg gtgtcgtgtt ttatggggag tttttggcgg gacgaaaggc 6060
ggacgcgcgg ttttttttgg ttttttaggt tcggaatcga cgggaaaggt tttcgtgatt 6120
ttcgagtttt tgtaggtttt ttttagcggg agttggttcg gggggtttag aggtttttaa 6180
gtattgtttt ggaggatggt ttttaaggat cgcggtttgt gagttgaagg ttttgtgaga 6240
ggttaaattt ttaaaagata tatatttggt aaattgaggt tatttgtaaa tatatttcgg 6300
tattaggaga agattcgagt agggaagtga aggataatta tttcgagtta tatttttttt 6360
ttttaataaa aagttttggg gatgaaagtt tttttggttt ttattttttc gatttaaaaa 6420
tttgagaaga aaaatgtgat tagagatgaa ttttggtgaa ttcgaaattg aaatataatt 6480
tttttttttt ttttttattt tttcggtttt agaatcgcgt tttttcgttt taggagattt 6540
tttggggtcg agggtttttc ggggaattcg ggcgttcgtt ttttttattg tttttttgtt 6600
tcgcgggtat agtttgtttt cgtttgtttt ttttattttt ggattttttt tcgtcgggtt 6660
ttttaaaggg tttttgcgtt ttaaaataaa ataaaaaaat tttttgtttt ttttaatttt 6720
ttcgtagttc gttttagcgg tggcgcggga ttagtaaagg cgaaagtcgc gcggtttttg 6780
tcgggcgcgg acggtcgcgt aggggcgttc gcggttttcg tattcggatt tgaggtgttg 6840
gtcgatttcg ggtatttacg gtcgggaggg agggttgagt tgttcgattt tttatttttt 6900

ttttttaaag tttatttgtt aatgttttta agaagaaaat taagtatgtg cgtggagagt 6960
ggggcggtag gtaattcgag tttttgagtt tcggagcgat ttaaagtagt aattgggaat 7020
agttttagga aagggaggtc gggtggagtg ggttttgggg taggagttat ggggttcggg 7080
tttcggggac gatttggcgt tttcggtttt gttgaacgtt gagttgcgtt tagtcgggtt 7140
ttcgaagagg tttttgcgta gagcgattta cgcgcgcggt agtattttcg attagttagg 7200
atattttagt aattgtttga attgtaggta ggtaaaattt ttgaaggagt atttgttgcg 7260
tgcgattttg ttgttggtgt aacggaggaa gggggtgggg gaaggaagtg gcgggggaag 7320
gagtgtggtg gtggtttaaa aaataaggga agtcgaggcg agagagacgt agacgtagag 7380
gtcgagcgta ggtcgaaagt tgtttatcgt tttttcgatt tcggggaata tggtgggatt 7440
ttttttttgc gtcgggtcgg gagttgtaaa atttcggtta tattaagatt tgaaaattgt 7500
gatgcgtttt ttttgtagcg acgttttttt ttgaatttgt tcggagtttc gagtttcggc 7560
gtttgttttt tagtttggta tggttttttc gggggtttgt tttgtatggg gagaggggtt 7620
acgtagcggc ggattaggtt tggggatttt cggtaatgga ttcggagtaa tgattaatag 7680
tcgttttttt ttattttttt atagcgatta ttttttaata tttttttttt tattttcggt 7740
ttcgcgcgtg ataaggtcgg ttgtttttag tcgggagtta gatcggtggt tcggtttttc 7800
ggagttttag taggcgttcg ttaaggggtg attggttgtt attgggagta atatttggtt 7860
ttgaggagat tttggggagg aagtggcggg gagttcgtgt ttgtttgtgt gtgtgtgggg 7920
gggtgtgtgt gtatacgcgc gtgggtaggg tttttttgcg tttttttttt taagtgtttt 7980
tcggtggtga ggttttgggc gggtgagatt ttttcgattt cgttttcggt tttatttaag 8040
tcgggttcga gttgggagac gtagtttttt tagtgcgttt taaatttttt ggttttaggt 8100
ggttcggcgc gggggtttag tacgacgtat cgcgtcgaga atcgggtttt tcgtgcgttg 8160
cgttagtagt tttgggagcg cggcggtcgc ggggtatcgg tcgagggttt tgtcgagcgt 8220
cgtcgggagt ttttttcgga tcgttgaggt tcgggcggcg ggcgcggagg ttggtttcgt 8280
ttggaggggc gggttcgcga ggggcggggg gttgtggagg aggggagggc gcgtaagttt 8340
tttcgtcgtt tgtcgcggga ggggtttcgg cgtttacgtg atttcgaggg gttggaagaa 8400
aaatagagtt tgtttgcggt ggagttttat tatatttaaa tatttttttt agg 8453

<210> 42
<211> 8453
<212> DNA
<213> Artificial Sequence

<220>
<223> chemically treated genomic DNA (Homo sapiens)

<400> 42

tttaaaagga atatttgaat ataatgagat tttatcgtag ataggttttg tttttttttt 60
agtttttcgg agttacgtga gcgtcgaggt ttttttcgcg gtaggcggcg aaagggtttg 120
cgcgtttttt ttttttttat agtttttcgt ttttcgcggg ttcgtttttt taggcgaggt 180
taattttcgc gttcgtcgtt cgagttttag cggttcggga ggagttttcg gcggcgttcg 240
gtagagtttt cggtcggtgt ttcgcggtcg tcgcgttttt agggttattg gcgtagcgta 300
cggagaattc ggttttcggc gcggtgcgtc gtgttgggtt ttcgcgtcgg gttatttgaa 360
gttagaggat ttggggcgta ttgaagggat tgcgtttttt agttcgaatt cggtttaagt 420
ggggtcggga gcgaggtcgg gaaagtttta ttcgtttaaa gttttattat cgagaggtat 480
ttaaaaagga aagcgtagag ggattttgtt tacgcgcgtg tatatatata tttttttata 540
tatatataag taaatacgag tttttcgtta tttttttttt agggtttttt taaggttaaa 600
tattgttttt aatgatagtt agttattttt tggcgaacgt ttgttaaggt ttcgaagagt 660
cgggttatcg atttagtttt cggttgaaag tagtcgattt tgttacgcgc ggggtcggga 720
atgggaggga gggtgttaga gggtgatcgt tgtgggaaag tgagagggag cggttgttag 780
ttattgtttc gggtttatta tcgagaattt ttaaatttag ttcgtcgttg cgtggttttt 840
ttttttatgt aaagtagatt ttcgaagaag ttatgttagg ttgagggata gacgtcgggg 900
ttcgaagttt cgggtagatt tagaaagagg cgtcgttgta gaaaggacgt attatagttt 960
ttagatttta atgtggtcga ggttttataa ttttcgattc ggcgtagaaa ggaaatttta 1020
ttatgttttt cggagtcgag aaaacggtga atagttttcg gtttgcgttc gatttttgcg 1080
tttgcgtttt tttcgtttcg gtttttttta ttttttaaat tattattata tttttttttt 1140
cgttattttt ttttttattt tttttttttc gttgtattag tagtagagtc gtacgtagta 1200
aatatttttt taagaatttt atttatttat agtttaagta gttattggga tgttttgatt 1260
aatcgaagat gttgtcgcgc gcgtgggtcg ttttgcgtaa gggttttttc gaaaattcga 1320
ttaggcgtaa tttagcgttt agtagggtcg ggagcgttag gtcgttttcg gggttcgggt 1380
tttatgattt ttgttttaaa gtttatttta ttcgattttt tttttttgag gttgttttta 1440
gttgttgttt tgggtcgttt cggagtttaa gaattcgggt tgtttgtcgt tttatttttt 1500
acgtatatat ttggtttttt ttttaggggt attggtaggt agattttgag gaagaaaagt 1560
aaaggatcga atagtttagt tttttttttc gatcgtggat gttcggagtc gattaatatt 1620
ttaggttcgg gtgcggaggt cgcgggcgtt tttgcgcgat cgttcgcgtt cggtaagagt 1680
cgcgcggttt tcgtttttgt tggtttcgcg ttatcgttgg ggcgggttgc gaaagggttg 1740

ggaagagtaa agggtttttt tgttttgttt tgagacgtag aagtttttta aaaagttcgg 1800
cgaggagagg tttagaagta gagaaagtag acggaggtaa gttgtgttcg cggggtaaag 1860
ggatagtaga aggggcgggc gttcgggttt ttcggaaaat tttcggtttt aaggaatttt 1920
ttggggcggg agagcgcggt tttaaaatcg agaggatagg aaggggaagg gggagttgtg 1980
ttttaatttc ggatttatta ggatttattt ttagttatat tttttttttt aaatttttaa 2040
atcgaaaaga taaaagttaa aagaattttt atttttagag ttttttattg ggggaaagga 2100
atgtaattcg gggtggttgt tttttatttt tttattcgaa ttttttttta atgtcgaaat 2160
gtgtttatag gtagttttag tttattaagt atgtattttt tgggggttta attttttata 2220
aagtttttaa tttataaatc gcgatttttg gaaattattt tttaaagtag tgtttggagg 2280
tttttaaggt tttcggatta attttcgttg gaagaagttt gtagggattc gggaattacg 2340
ggaatttttt tcgtcggttt cgggtttgga gggttaggaa gagtcgcgcg ttcgtttttc 2400
gtttcgttag gaatttttta taggatacga tatcgtagga ataagcgttt tgggagtttt 2460
tgggattttg gttgtcgttt ttagggattt tatatcgtga aatgatagag gcgaggtttt 2520
ttgggtttcg taagtcgacg aaaatagttc gtggagaagg cgcgttttgt aattgtagtt 2580
cgtaagtttt tagggcgttt cgttagttgg gggttagatt gggtgatatt tttttcgacg 2640
tagttttcgg agcggcgcgt attttttaga ggttagtagg attgcgtttt ttatcgtaga 2700
atttgtttta gttaggtgtt ttttttttat ttcgtcgtcg ttttgttttt ttattttttt 2760
tggattttag agtcggtttt tttttttttt ttttcgcgtt tttcgttcgg ggatcgtaat 2820
ttttagttcg tgggtaacgc gtttagttta agattttata gtttttttta acgatttttt 2880
tattaagggt tttttttgtt tttgtttttt tgtttcgtat tttttaaagg tgagaatttt 2940
ttagggattg ttggatagag aaggtttggg taggggtagt agtcgatatt taaaggtttt 3000
ttagcgttag tatttttatt tttagtatta ttattggaaa ataattaggt tattttttgt 3060
ttcggttata gtttgtgatt agtttttttt ttttaaggag gtttttagat tttttttgtt 3120
tgtttttttt tattaggtag ttttgtttat tataggtttt tgtagtagcg ggttcgttag 3180
tagtagtttt tggggatttt tgaacggttg tagtgaattt gggcgagggt cggggattgt 3240
ggcgttgtag gttcgagata gtttggacgg gaattttatc ggggagataa gttagatatt 3300
tagtcggagt agtagcggcg ttttaggttc gcgtttttta gttcggatgt tatatttatt 3360
tagttttcgg gtcgggtttc gcgttttttt tttttttttt tttttttttg ttttttcgta 3420
tttttttttt tttttttttt ttttttcgtt tttttttttt tttttttttt tttttttttc 3480
ggcggttttt tttttttttt tttttttttc gttcgttttt ttttttgggg atgggagttt 3540
cgtttatatt ttttttttta gtcgttcgat ttcgtttagt aggttcgggt ttcggaagtt 3600
taggtagcgt tcgagttcgt agttgtcgtc ggagttggga cgtcgttgtt tttagttttg 3660
ggaagcggtt ggggtttatt tggggattac gtgcggaggt attagaaagt atgtatcgat 3720
tagtcgtcgt attttttaaa aatatttatg ggagtttggg tttttttgag tttagtgtaa 3780
ttgttgttta aattgatgat taaaatattg agtaattatt atttattttg agtaattaga 3840
gataatgaaa tatttttaaa attaggttat ggagaaagga gttgttggat cggtttaaaa 3900
ggtggttttt tataaattgt taaaagattt ttaaacgttg agaaataggt tgtgtgtaga 3960
attgtgtgtt agagagagat attttttggg ttgttaaaag gtgaattttt ttttaaaata 4020
tttttaaagt gcgttttgta gaatattttg gtgatgattt tgaggtagat tttagtaaat 4080
gagtttattt tttttttttt tttaaggttt aaagatagag gtggtttgta gataggttgg 4140
ggttacgttt tttatagtta gaaaaatttg tttttagttt agaagtcgtt ttaagaatcg 4200
atatagtagt tatttttgtt ggtgttatgg ttgttgttga tttttttgtt tatttttggt 4260
gtatttaaga gttttgatat tttgtttttt taaggagata attatagaag tagtaatttt 4320
ttaggttata gggagataag aaaagatgtt ttttggtttg tttttaaagg aagaggggag 4380
atgaggaatt ggttttttta tataaaaata ttttagtaat tatattagta gggaggtgga 4440
atgtggtggt ttttaaattt ttttaaatag gaattaattt tatatattgt ttttgatgga 4500
gttgtgaggg agttattagt tttatttttt tgttttataa ataaggaaag gtaggattat 4560
taggttaaag ggatttgttt agatttttga agtaaattat agtttttgtt ttagagttaa 4620
tgttttaggg aggggttgag gtttatagtt tgagttttag gtagttagag gagtgttgga 4680
agattgagta ttttttgttt tggatttttt tttttgggat agatatgata ttttattgtt 4740
tttgtaaagt atttaggttt ttatttaaat agatattggt tttgggtggt ttatttataa 4800
aaattttacg tttatatatt ttcgatattt aaaaaataag aattattttt tgtgtggaat 4860
atagattata aagggtggga aatatttgag attagagaag taattgttag agtaaggtta 4920
agatattttg ttattttttt tatttattag gtttaggttt tttggtttaa tgtattaaat 4980
aatagaaata agttgtatgg ttttgggtaa gtttttttat ttttatgagt tttagttttt 5040
ttatttgtag aatgaaagga ttgggttaga ttatttgtat gtttttttta tatttgaaaa 5100
tgatttgatt atttaattgt aattttagtt aaagttcgtt tttttttatt ttaagttatt 5160
tttgagttta ttttttttaa gaattattga ttaaaatagg gtagggtaat aatattattt 5220
taaaaggagg ataaaatata tttgtagttt ttaaatagtt aaaattataa ttaattatta 5280
aaaataataa tagaaaatta aaatttattt aaatagttat ttaatgtttt ttttgtagta 5340
ttaggtattg tgttttttta gtatggtatt tgaagttagg atagattaaa tgtttattga 5400
gtttttaaat taattattgt tgttttaatt attaacgaaa tgtggttttt aatatttaaa 5460
tttagaaata atagtgatat gttggattta aaggtaggat tttattttat tttaaagtat 5520
taatgttatt tcggggtttt tttaatttta aaggtttttg aatgatagtt attgttttat 5580
tttgttgtag tttagatatt aaagtggtta ttgattggtt tatttatgag tttgtgatat 5640
ttatagttta ggagttttta attaagttat tcgatatatt atgtttagat ttttgatatg 5700

aatttttaga tatatgacga gagaatttag ttttagtttt tttgggatag ttttattttt 5760
ttttttatat tttagatggg cgtaagttat ttgttattag ttgtttttat ttattttttt 5820
tttttttttt ttatttatgt taaagaataa attatttgtt ggttagagag ttaaattaat 5880
tatagtttag tgattagagt tcgtgagaag aattagttta gttatttttt gtattttaaa 5940
tttatttgtg aatttttatg tttttataga atatggtaga tagattaaat gaaattgtat 6000
ttttaaagta tattgatgaa gaggggaaat ttattataat ttaggagtaa ttattgtttt 6060
tatgttgtta ttatttaatg aaaggtattt ttttgttggg ttttttttgg agaatattaa 6120
gaaagagtag ttggttaata gtttgtttgc gagtttttgg gatagttttt tttaaagtat 6180
gtttttagga ttataagttt tagagtaggg tgggggtgta gatgaaatgt agattttttg 6240
gttttgtttt agatttggat ttagaaattt gtatatttta taaggttttt tggttatttt 6300
gagtatatta aagtttgaga gttattgttt taaaagttat tttgttttta ttttttatgt 6360
tgataagtat tattaaaagg ttgatattaa gatattatta aataggtaaa gaatatgttg 6420
ggtttgaatt ttataaattg ttaaatatgg taattatagt attattatgt tttgaatata 6480
ttaaataggt ttatgtaagt gaaaagataa ttttatagat atagtagttt ttaatttttt 6540
atttttaatt aagaattata ttaggttttt aaattttttt ggttaattag gtgtttggtg 6600
aagttacgga aggaattttt agaattattg ttaagttttg agaagtagta agatagatta 6660
attggaattg gaattcgaat tcgaattgga attggaagtg gtttttgaaa ttggttagaa 6720
atttagtttt ttttttatta atatttatta tattttatat atttgtttta tatattttta 6780
gtttttataa taatttttat aaaatttttg ttttatagat gagaatttga agtttagaga 6840
cgttatgtaa gttgtttaag gttataaagt tagaaagtgg tggattttaa gtttaaatta 6900
tgttagtttg tttttttttt ttgggtaagt gaggggtatt gtggatggat tttcgaggtt 6960
tattttggaa gtttttttta ggttaaggta ttagttgttg ttttattttg gtagtatgtt 7020
attattagga attttaggat atttttttaa gtttttagat aataaaataa aattaagatt 7080
tagaagtttg tttttttttg acgttattat ataagttcgt tttagggtaa gatttttatg 7140
tgattttttt tgagaaaaag gttatgattt tgagaaagga gtttattatt tttgttgggt 7200
ttaagttttt ttattgtaaa atgtggagtt tgaatagggt ttttataggt tttttttagg 7260
ttttggaata tagagataat atattttggt agttaaaatt atagttaaat tgtttaattt 7320
ttaatgttta tttattatgt tttgtttata tagatttgat ttatgaatat agttgtgtta 7380
agataattat ttattgttaa aattatattt attttgagta attagttttt attaataggt 7440
tgagattgaa tgatatgttt aaaaagtatt tttcgtttgt tttgcggatt tttaatattt 7500
ttaattttta agaatttttt tttttgtttt ttaagttaaa atataaattt atttttttta 7560
ttttgattta aagatgaatt tttaatttaa ttgtataagt tgtttttttt ttaaatattt 7620
aatattgttg ttaatttttg atatgtttta tttaaagtaa ataatattgg atgttgatat 7680
ttttttatga taaaaagaaa atttaatgtt attttagttt tttatgtttt aaattgttgt 7740
tttgatattt tttggaatat ggtttttttt ataaaaattt tttaatatta atatttaagt 7800
taggtgcggt ggtttatatt tgtaatttta gtattttggg aggttaaggt aggtggatat 7860
cgaggttagg agatcgagat tattttggtt aatatggtga aaatttattt taattaaaaa 7920
tataaaaaat tagttaggta tggtggcggg cgtttgtagt tttagttatt cggaaggttg 7980
aggtaggaga atggcgtgaa tttaggaggc ggagtttgta gtgagttgag attacgttat 8040
tgtattttag tttgggcgat agagtgagat tttgttttaa aaataataat aaaaataata 8100
ataaataaat aaaaatttaa aatggataga gatttttaaa tgaattttta gtgatattag 8160
atagatgaaa agattgtttt tatgttataa tatgtttaac gtggatggta gatagggtat 8220
ggatatgaaa agtttttttt aaggaatgat taggatggga ttggtatgta atgatagata 8280
tttgggtttt gattattttt agtttttttt tttaaaaaaa tagtcgattg tatttattta 8340
attttataat agggatgttt tttgaaaata aattatttta gaggatataa aaaagattaa 8400
attttgaaat ttttttgttt tgtgtttata tatttttttt tttttttttt ttt 8453

<210> 43
<211> 7189
<212> DNA
<213> Artificial Sequence

<220>
<223> chemically treated genomic DNA (Homo sapiens)

<400> 43

ttgttatgat attgaaatat atagtttatt atatgtgtag tgatttatat gttgttaaga 60
ttatttgtta gttattttta attttggtta tatattaatt agtattgaaa aatattaatg 120
tttgggatta tttttaagag ttgtgatata attggttttg ggtgtggttt tatttttagt 180
attttttaaa atgttttttt gttttcggtt tgtgatttta atgtgtagtt tggtttgaga 240
gttttcgtgt tagagggata tttatatgta tagtttgaat attaataatt attgttatgt 300
tttttattat tatatttttt tagggtatta tgttttagga agtagatagt ttatattttt 360
ttatattata gaatattaat ggattttgga tgatgtatat ataagttttt tacgtttttg 420
atggataatt tttgttatat attttgggtt aatttgttag gttgtaaatt tattttaaag 480
gttttgaaga agattttata aaatattatt tgttttttag gaaaaaagaa tgttatggaa 540

tagtagggtt attttattaa agtattatag ataatagtag gtttgttatt aaggttattt 600
tgtattagat aatttttaag ttttttttaa attgttttat tgtgaatata ttagattagt 660
gatatttata gattttattt ttttttgagg attaagttgt tgttaatggg aattttgggg 720
taatttggta gtcgtgtttt ttatgaatta ttacgggttt tgaattggtt aatttattgg 780
taaaatattt gtagttagag ttagttattt ataaaagtat aaatagtgtg ggtttataag 840
tatttatttt gatatttata aatatgtttt tagtgtattt ttttatgttt tagattttat 900
tttattttag ggtttattat gtgttttaag tttgtatgta tttattgagt tttagtaggt 960
gtgttgttaa ttgttaatgt tttttaaaaa tggttatttt tagggttttt tgtattgtaa 1020
tatgggttat aaaaatagta ttaataaaat aaaataaaat aaaatatata ataagtaatt 1080
atagtgtaaa ggaatgaggt gttaattgat aggagtagaa gttgtttatt tgggttaagg 1140
gttattttaa tttaatatgt tgtttttgta atgagagatc gaggtatttt gtaagtttta 1200
atgtaatttt ttatataatg tttttttttt tgagttattt taattgttta gtattgatag 1260
aagtttgtag ttagatttaa aattttgggg taagatgtgt aattggtaag tttttttatg 1320
aaaggaaatt ttagaaggtt aggggggttg tatattaaga gatgtagtaa tttattaatt 1380
tattagttta aggaattttt ataattatta ttttagagag atttgttttt aaaaaatttt 1440
ttggaatatt gtttattttt gttaagaaaa tagattgtta tataaatatt aattgagtta 1500
ttaatatgta ttgttttata tcggtgatta aaataaattt tgtttttatt tttatgtagt 1560
ttgtattgta gttaaaattt taagaaaaaa agtagaaatt ttttttgatt aataattata 1620
ttgaaataat aattttatgt ttttaagttt attgatagga taatatttgt ttttaagaaa 1680
taatataaaa ataattttag gaatatgtaa taatatgaag tatattgaaa aattttttgt 1740
ttatttaaaa ttattcggta atgttattat ttatgaaaaa taatattaaa tattttaaaa 1800
gtttttggtg attgtatttt attatgtatt tttaagattt ttttaataat tagaaattaa 1860
gatggataaa aagtttatgt tgttcgtgat tatagttttt ttttaggtta taataagtaa 1920
atttaagtta gatggtagtg gtgtagtatt gttttgtttt tttttggatt atattatatt 1980
ttttatttat atgtttttta tttaaagaga gtgaattgaa gtaatttaat attatagatg 2040
ttgttttgtt atgtatttgt ttatgtagtt attaaaaaga ttttagaacg tgatattttt 2100
tttaaattat tttattgttt ttagtttttt taatatttta agtggtttta tttttatttt 2160
ttttttttat tttttttaat ataatttaag atagtttttg tttaagattt ataattatat 2220
ttaaaggatt ttgaattttg ttttaatttg agagataatt tagaaagtag atgttattta 2280
tattgggtat ttgtttttcg ttgtagttat atttttatat taggagattt tttttttaat 2340
agagagaaag gagaaatgtt tttaaggaga aaataatata aaaataaaaa gttaaaagtg 2400
aaatagaaag gtttttgtga gtaaaataga tttaaacgtt ttaaattttt ttagagtaag 2460
ttttttttaa aaagttaaaa aaaaaaaaat tggttgattg gatatagttg gtttatatat 2520
taatgaacga aatgtgttag attttaatga gggaaagaga aaaatgatta gaaagaatga 2580
tagtgtagaa atatttttta tatgaattgt attgtttttg ttttttatta gaaggttttt 2640
aatattagga tatatttagg gaaatattat attgtgaaag gtagattttt ataaggtttt 2700
tttttttttt ttttgaaatg tgtttatttt tagatataat ttttgtgatg ttttgggtgt 2760
tttttttgtt tttatgtttt tttttttagt atatgtttat ttcgtatgtt taatgtttta 2820
tataatttta aatatatatt ttgaaatttg gaatgtttat attttttagt tgtaaaatat 2880
tttatttttt aaattgtttt tatatataag tttatttgag aatttaaatg atggagtaaa 2940
atatatatat ttttaattga aaatgaattt gattttttgt ttggcgatta ttattttttt 3000
tttatttttg gaaggttttt tttttatttt ttttttttta ttgtataagt tgattttttt 3060
gaaaaatgag tgtttgttaa atttagtttg agttggaagg gaaggtcggc gggaagggtg 3120
attatttaat attttgatta agaatattag taattgattt ttaatataga ggtaggttat 3180
atgtttggtg aaataattaa attataatta tattgattga tattataaat tatgttttgg 3240
gaaaatgagt ttttaagatt tgtgatgttt agttttattt ttatttttta tatgtatttt 3300
agaataaaaa tttatatata tggaagatag taagagttgt tttttttttt agtgtggtaa 3360
atttttattt tgtaatttta tgttgtgttt tagagataaa tgaagtaaat ttataatttt 3420
gatgatatat tttaaaatat tgagaattgt agtttgtttt atttaggatg ttttgtaaat 3480
gtagagaaat tttttgtttt gaagtaattg atttgatttt gttttttagt ttggtttgga 3540
tgttaaataa gtaatgttaa gaattttagt tatttaaatt attttttttt gaagtggtat 3600
tttttaattt atttttaatt tgtattattg ttattttttg tgatatttat tttataaggg 3660
tgttgtatgt atgttagtat ttgtatttgt tattgtggta ttttatatga ttattaataa 3720
ttaaatttta gtaaagtaaa tattaaatag taatattaag ttttcgtgta ataattttgt 3780
ataagaaaat gatgcgtata ataagatttt gtaaattttt ttagtatttt gtatttatta 3840
ataattgaag atttttaaga aatatgattt ttttttatat atttagagga aatatttaaa 3900
aagaaataag aaattaggcg tttttaggta gagtaaagga aaaatattaa aattttataa 3960
tagttttaga atttgttcga taaatttata ttggtattag tatattattt tagataatga 4020
aagtattgtg agttttaagg ttaagtgaga gtttgtaagg aaaattatag ttgtataaaa 4080
atattgtaga tttttaaatt ttaaaggtat taaatattaa ggataaatat atattttgtt 4140
tatttttttg gattaagtat ggtattttag aaaggtgtgt atttttttta ttttatttat 4200
taaattttaa aaatttaaat tttaatttgt atgtagttat gacgatttga tttgtgttta 4260
tttgtatggg ttaaaataag ttaaagtatt gaatataatt tatgggaaat atttaatttg 4320
taagtttgtg tatattttaa ttgaaataat ttaaaagtta tgaattatga tattatattt 4380
taggatattt gtattttaat tttttgttta aaattttaat atttaaagat atttaaagtt 4440
aggttattta attattgtta ttgaattatt ataattatta ttttggtagt tagaatatat 4500

ttattagtga ttaattgttt gttgtatttt attttaaaag gatttggttg ttattaagaa 4560
atagtgaatt aattttaatt tataatttat ttttttattt tttaagaagt gtattttaat 4620
gatttataaa aattagaaat aattttaaat atagaatcga gatttattaa gttaatttag 4680
gtataatttg agatttaata tagaagtgta ttaagtatat gttattgaaa aaagatttaa 4740
agaagtatta tgtaaaatgg atggtaaaat ttttagtgta ttatttgaaa attgtttttt 4800
atttttttta gaatttgtat gttttatgta ttattgtttt atgttagatg cgtggttata 4860
aaattgtgaa aatggagatt tttttggtag taaaagggtt aagttttttt tgttttatta 4920
gttttaaatt ataatgagaa gttttttttt ttttagtagg gttgtgttta tattattttt 4980
tagatttttt ttgaagaggg ttggtatatt tgtgtttgtt ggaggtggaa ttaatagtaa 5040
gaaggagaaa gggattgaat ggatttatag gaaggatttt aagtaaattt agggaaatat 5100
atttatttga atagtataat ttagagtatt attttatatt aagacgatat aaaagatgtt 5160
aaagttatta ttaagttgtc ggatagatat atattttaat attaaggtgt agattagtat 5220
agatttgtga tttagaaatt aggatttgtt ttggaaagag tttaagggtt gagaagaatt 5280
taaaagtaag tgaagattat tttgggaatt atagtttatt agaagattaa tttttgttaa 5340
tttaaatatt aaaggtttga ttattataaa tttatatagg aatgtatagg ttatttgatt 5400
aaataatatt agtcgttttt tgttatatta atgtagtaaa aatttttaat aattgtggat 5460
aattggaaat ttgagtttta gtttttttag aaataattat ttttgatata ttttaaaata 5520
tttaaaatag gataggaaaa tcggtgagga tgttgtgttt agaaatgtta ttgttatgaa 5580
aaataggtaa atttgttttt ttagttattg ggaaattgta ttttttagaa ttttaggttt 5640
tttttttttt taagaggata agaaggatta aaaatattaa tttttgtttt tggataaaaa 5700
tgtatttgat tgtattttta tttaagggta ttgtgggttt tttttggagt tgttgggttt 5760
tagtgggtta tgtaaaagga ggtttgggag ataattatgt ttattttagt tttatttata 5820
gaagattacg gaattacgaa agacgggaaa tataaaggga aatttttttt attttgggtt 5880
tgttttatag atttagatta tttttatttg gaaaataagc gttttttgta ttttttttta 5940
tgttggattt ttataatgtt atgattaatg aagaaaattt tgaagagtcg gagtatttag 6000
taagggtatt tttggtagaa gagattagag gggtaagaaa gggatattta gtttttttta 6060
atgggtattt tcgtcgtata tagttatttc ggacgatttt tttgattatt tagagttttt 6120
ttttagttag tttttatgat attaattttt tgaatgatgt tgatatggtt atgagttttg 6180
ttaatttagg tatgttgttt atttgtttgt taatttatgt tattataaag tggaagtttt 6240
ttttaatggt aaaggagttg tttggtaggt ggttatgttt atataaagat atttttttta 6300
atttttttgt ttagtttttg tttttttttt tttattgatg ttttaggtaa aaagttatat 6360
atatatattt aatatgaata gttaaattta aatatttata gtgtgggttt ttttagattt 6420
tgttaaaatt ttaatttcgt ttaaatatat gaatgtaaag gtaatagttt aaaaataggg 6480
aaatttatga ggatagttat gttatgggta ataataaaaa aaagaagtat ttatagatgg 6540
gaaagtaaaa gtgatattcg gaagtatttt ttttttaagt gatttaaagt atatgtaagt 6600
atatatttat atattattta aaaatttttt atttagggga ggtaattatt gtaaaaatat 6660
agaggtaaaa ttaattaaga ttgaatagta ttataaaaga ttattattaa ggaattaagg 6720
aagggatttt gaaattttat agtaaatatt ttagaaataa agttttaatt taatagtttt 6780
taattgtaat gagtaaagta tgttatagtt atttgatttt ttttataggt aagaaaaatg 6840
taaaatatgt tttagtggtt tttatttata taaaattttt tttttatatg agataaaata 6900
tttgaaattt tttgtaaatt ataataatat taatgagaaa attttaaaat tatgttttag 6960
ttgtagttgg tattttatgg tggtttttaa atattgataa tgttaaatga taatttagtt 7020
tttaatattt ttttttataa taatattatg gattattaat tttaaattat tttataaggg 7080
ttgttgtttt ttttaaatgt tttagtaaaa attgaaggtg aaatttatta tttttggtag 7140
ttaaaggaaa ataatatcga atatatttta tgtttgaaga ttaaaaatt 7189

<210> 44
<211> 7189
<212> DNA
<213> Artificial Sequence

<220>
<223> chemically treated genomic DNA (Homo sapiens)

<400> 44

agtttttaat ttttaaatat aaaatatatt cgatgttatt tttttttgat tgttagagat 60
agtaaatttt atttttaatt tttattaaaa tatttaaaga aaataatagt ttttataaaa 120
taatttgaaa ttggtaattt ataatattat tgtgagaggg aatattggga attaaattat 180
tatttgatat tattagtatt taaaaattat tatgaagtat tagttataat taaaatataa 240
ttttaaaatt tttttattaa tattgttgtg gtttatagaa aattttaaat attttatttt 300
atatagaagg aaaattttgt atagataaag attattaaag tatgttttat attttttttg 360
tttgtgaaaa aaattaagta gttataatat attttattta ttatagttaa gaattgttag 420
attgaaattt tatttttgaa atatttatta tagaatttta gaattttttt tttgattttt 480
tgatagtaat tttttatggt gttatttaat tttggttaat tttattttta tatttttata 540
gtagttgttt tttttagatg agagattttt aagtagtatg tgaatatata tttatatgtg 600

ttttaaatta tttaaaaaaa aagtgttttc gaatgttatt tttatttttt tatttgtaag 660
tgtttttttt ttttattgtt gtttatagta tagttatttt tataaatttt tttgttttta 720
gattattgtt tttgtattta tatatttaag cgaggttgaa attttaataa gatttgaaaa 780
gatttatatt ataagtgttt aaatttaatt atttatatta aatatatata tatgattttt 840
tgtttaaggt attaatgaaa gaaagaaaat agaagttaaa taggagagtt agagaaaatg 900
tttttatata aatatgatta tttattaagt agttttttta ttattaggga aaatttttat 960
tttgtaataa tatagattaa taaataaatg aataatatat ttaagttgat aaagtttatg 1020
attatgttag tattatttag aaagttggta ttatggaggt tggttagagg aggattttgg 1080
gtggttagag gagtcgttcg agataattgt atgcgacgag gatatttatt gggagaggtt 1140
gggtattttt tttttgtttt tttggttttt tttgttaagg atgtttttat tgagtatttc 1200
gattttttag gatttttttt attggttatg gtattgtaga gatttagtat aaagagaggt 1260
gtagaggacg tttgtttttt aggtgaaaat ggtttgggtt tgtgaggtaa atttaagata 1320
gagagaattt ttttttgtat ttttcgtttt tcgtggtttc gtagtttttt ataaataaaa 1380
ttggagtgaa tatgattgtt ttttaaattt ttttttgtat aatttattag aatttagtag 1440
ttttagagga aatttataat atttttaagt aaaaatatag ttagatgtat ttttgtttaa 1500
aagtaaaagt tgatattttt agtttttttt gtttttttaa aaaaaaaaaa aatttaaggt 1560
tttaggaggt atagtttttt agtagttgaa aaaataaatt tatttatttt ttatgatagt 1620
gatatttttg agtataatat ttttatcgat ttttttgttt tattttaaat attttggaat 1680
atgttaagag tagttatttt taagaaagtt gaaatttaga tttttaatta tttatagttg 1740
ttaagagttt ttgttgtatt gatgtagtag aagacggtta atattatttg attagatgat 1800
ttatgtattt ttatatgaat ttatgataat taggtttttg gtatttgaat tagtaaaagt 1860
tgattttttg ataaattgta gtttttaaag taatttttat ttgtttttga gtttttttta 1920
atttttgagt tttttttaaa ataaattttg atttttgaat tatagattta tgttgatttg 1980
tattttggtg ttggaatata tatttgttcg gtagtttggt gataatttta atattttttg 2040
tgtcgtttta gtgtaaaata atattttagg ttgtattatt taagtaaatg tgtttttttg 2100
aatttatttg aaattttttt tgtaagttta tttaattttt tttttttttt tattgttaat 2160
tttattttta gtaggtataa atatattagt tttttttaag agagatttaa agagtagtgt 2220
aagtataatt ttgttgggaa agaagaggtt ttttattgta atttgaaatt ggtaaagtaa 2280
aaagaattta atttttttgt tattagaaaa gtttttattt ttataatttt ataattacgt 2340
atttaatatg aaatagtgat gtatgggata tataaatttt aggggaaata aaaggtagtt 2400
tttaagtagt atattgaaaa ttttgttatt tattttatat agtatttttt tagatttttt 2460
tttagtgata tgtatttaat atatttttgt attaagtttt aggttgtgtt taagttagtt 2520
tgatgaattt cggttttgta tttgagatta tttttagttt ttgtaagtta ttaaaatgta 2580
ttttttaaaa aataaaagga tgagttgtaa attgaggtta atttattgtt ttttaatgat 2640
agttagattt ttttaaaata aaatatagta gataattaat tattaataaa tatattttag 2700
ttgttaaaat agtagttgta ataatttaat aatagtaatt agataattta gttttaagtg 2760
tttttaagta ttaaaatttt aagtagaaag ttgaaatata aatattttaa gatgtagtgt 2820
tatgatttat aatttttaag ttattttagt tgaaatatat atagatttat aaattagata 2880
ttttttataa attgtattta atattttgat ttgttttaat ttatgtaaat aaatataaat 2940
taaatcgtta tgattatatg tagattgaaa tttgagtttt taaaatttga tgggtaaaat 3000
ggggaaaatg tatatttttt taaaatatta tatttaattt aaggaaatga gtaaagtatg 3060
tgtttatttt taatgtttaa tgtttttaaa atttggaagt ttataatgtt tttgtgtagt 3120
tataattttt tttataggtt tttatttaat ttttagggttt ataatatttt tattgtttgg 3180
gatagtatgt tgatattaat gtaaatttat cgagtaaatt ttagagttgt tatgaaattt 3240
taatgttttt tttttatttt atttagaggc gtttggtttt ttgttttttt ttaaatattt 3300
tttttgaatg tataaagaaa agttatattt tttagaagtt tttagttatt ggtgaatgta 3360
gaatattgaa agggtttgta gaattttatt gtacgtatta tttttttata taaagttatt 3420
gtacgaaaat ttgatgttgt tatttaatat ttgttttatt gaaatttaat tattaatagt 3480
tatgtaggat gttatagtag tagatgtaaa tgttgatata tatgtaatat ttttgtaaaa 3540
tagatattat aaagaatagt aatagtgtag gttgaaagta agttaagaaa tattatttta 3600
aaaagaaata gtttaaatgg ttaaaatttt tgatattgtt tatttaatat ttaaattaaa 3660
ttaagaaata aagttaaatt aattatttta agataaaaag tttttttgta tttataaaat 3720
attttagata aagtaggtta tagtttttaa tattttaaaa tatattatta aagttatgaa 3780
tttgttttat ttgtttttag agtatagtat agagttataa agtaaaaatt tattatatta 3840
gaggaagaaa taatttttat tattttttat atgtgtggat ttttgtttta agatatatgt 3900
gaaaaataaa aatgagatta ggtattataa gttttaaaaa tttatttttt taaagtatga 3960
tttataatat taattagtgt aattataatt taattgtttt attaggtata tagtttattt 4020
ttatattgga aattagttgt tagtattttt agttagaata ttaagtaatt atttttttcg 4080
tcgatttttt tttttaattt agattagatt tggtaaatat ttatttttta aaaagattaa 4140
tttgtgtagt gagagaaaga aaataaagaa aaagtttttt aggaataaag aggaaatagt 4200
aatcgttaaa taaaaagtta gatttatttt taattaaaag tatatgtatt ttattttatt 4260
atttgaattt ttaggtagat ttgtatgtga gagtaatttg aaggataaag tattttgtaa 4320
ttgaaaagta taaatatttt aaattttaga gtatgtattt aaagttgtat aaggtattga 4380
atatacggaa taagtatatg ttgagaaaga agatatgggg ataggaaaaa tatttagggt 4440
attataaaaa ttgtgtttaa agataagtat attttaaaga aaaaaaaaag gagttttgta 4500
aaaatttatt ttttataatg tagtattttt ttgggtatat tttaatgtta ggaatttttt 4560

ggtaaggaat agagataatg taatttatgt aagaagtatt tttgtattgt tatttttttt 4620
gattattttt tttttttttt tattaaaatt tgatatattt cgtttattga tatataaatt 4680
aattatgttt aattaattaa tttttttttt tttggttttt taaaagaaat ttgttttgaa 4740
aaaatttgaa gcgtttaagt ttgttttgtt tataaggatt tttttatttt atttttggtt 4800
ttttgttttt gtgttgtttt ttttttgaaa atattttttt ttttttttta ttaggagaaa 4860
aattttttaa tataaagata tgattgtagc gaaagataaa tatttaatat aaatggtatt 4920
tgttttttaa attgtttttt aaattaaaat aaaatttaaa attttttgaa tgtaattata 4980
aattttaagt aaaagttgtt ttagattgta ttggagggag taagaggaaa gggtggagat 5040
aaggttattt agaatgttga agaggttgga agtagtggga taatttaggg aaggtattac 5100
gttttagaat ttttttggtg attatatgga taaatgtata ataaagtagt atttatagta 5160
ttaaattgtt ttagtttatt ttttttgagt gaaaaatata taaatagaga atatggtgtg 5220
gtttaaagga aggtagagta gtgttatatt attattattt gatttagatt tgtttgttat 5280
aatttgaaag ggaattgtaa ttacgggtag tataggtttt ttgtttattt tgatttttag 5340
ttgttaagga agttttggaa atgtataata aaatatagtt attaaaaatt tttgaaatgt 5400
ttggtattat tttttatgga tggtgatatt gtcgaatagt tttaaatgaa tagaagattt 5460
tttaatatgt tttatattat tatatgtttt taagattgtt tttatattat tttttgagag 5520
taaatgttat tttattaata gatttaagga tataaaatta ttattttaat gtagttatta 5580
attaagggag atttttattt ttttttttga gattttaatt gtaatgtaaa ttgtatgaaa 5640
ataagggtag aatttatttt ggttatcggt gtaaagtagt atatattagt ggtttagtta 5700
atatttgtat aatagtttat ttttttagta aagatgaata atattttagg gaatttttta 5760
agggtaggtt tttttgaagt gatagttata aggatttttt aagttgatga gttgatgaat 5820
tgttgtattt tttagtatgt agttttttta attttttaaa gttttttttt ataaagaaat 5880
ttgttagtta tatattttat tttagagttt tgaatttaat tataaatttt tgttaatatt 5940
aggtaattgg aataatttag aagagaaagt attatataaa gaattatatt agaatttgta 6000
aaatatttcg gtttttattt ataaggatag tatattgaat taaagtagtt tttggtttaa 6060
atgagtaatt tttatttttg ttagttaata ttttattttt ttgtattgta attgtttatt 6120
gtatgttttg ttttgttttg ttttgttggt gttgttttta tgatttatat tataatgtaa 6180
gaagttttga aaatgattat ttttaaggaa tattagtaat taatagtata tttgttaagg 6240
tttagtaggt atatgtagat ttgaaatata tagtagattt tgggatgagg tggggtttgg 6300
agtatggaag aatatattaa aggtatgttt gtgagtatta aagtaaatgt ttataagttt 6360
atattattta tatttttgta gatggttgat tttaattata agtattttat taataagttg 6420
attaatttaa aattcgtgat agtttatgaa aggtacgatt gttaagttat tttaaaattt 6480
ttattaatag taatttgatt tttagaagga agtaaaattt ataaatgtta ttagtttgat 6540
atgtttataa tgagataatt taaagaaaat ttaaaaatta tttaatgtaa aatggttttg 6600
atggtagatt tattattatt tgtaatgttt taatgaaatg gttttattat tttatagtat 6660
tttttttttt taggaagtag ataatatttt ataggttttt ttttaaaatt tttaagataa 6720
atttatagtt taataaattg gtttaagata tatgatagaa attgtttatt aggaacgtaa 6780
agaatttgtg tatatattat ttaagattta ttaatatttt gtgatatgag aggatatgga 6840
ttatttgttt tttaagatat aatattttaa ggaaatataa taatggggaa tataataata 6900
attattaata tttaagttgt atatgtaaat atttttttaa tacgaaggtt tttaaattag 6960
gttgtatatt agaattataa atcggggata ggaggatatt ttaagaaata ttaagagtag 7020
ggttatattt aagattaatt gtattataat ttttgagaat gattttaagt attagtattt 7080
tttagtattg attaatatgt agttaaagtt gagaatgatt aataaataat tttggtaata 7140
tatagattat tgtatatata ataagttata tattttagta ttataataa 7189

<210> 45
<211> 6541
<212> DNA
<213> Artificial Sequence

<220>
<223> chemically treated genomic DNA (Homo sapiens)

<400> 45

gtttttagat tttagaaatt ttgtatggta ttagtttttt ttagagtgtg tgtgtcgttt 60
gtatttttta tttggaattg tggttattgt tatgtgtatt taagattgat ggaagacgtt 120
taatttttta ttttgttgtt tttaaagttg tgtaaagaaa attttagagt gggtaagcgt 180
gaggagttta agtttttcgt gtaatgagag atttgcgtgg gaaataaaat tttattatag 240
gtgtgtttta ataatttttt ttgtagggtt tatttttgta tataacgatt tttaagtttg 300
tgtaggaggt agtttttggt tttttatttt ttgttggggt tggaggttta ggttatttgg 360
tttttgttag gtggtgtgga tagagagaga gtagtttatt ggtttttttg gttgtgtttt 420
ttagggagtt tttaggttag ttgagttttt tttagggtag atggatgaag atgaggtttt 480
tatttcgttg aataatgtgt gtgttagtag aatttttttt ttttttttat tgttttagag 540
agatatgttg atttggttaa aattatttta gtaagatgtg attgatgatg taggaaatga 600
aaatttttgg tttagtattg ggaggttagt aggcgagggg cgcggatatt gggatcgggt 660

tattcgatat aataatattt tttataattt taggtcgttt tttatttagg taaaggcggt 720
tttaggtgtg gagtagattg gggtagatta gattacggtt ttggagttta gtgttgggtg 780
ttttggttag gggaattata attgggggtg tgtgggttgg gggttagttg ttggggaaat 840
gagataggat ttagggaagt ttttgggttt tagttggttt gtagggggtt taggtataat 900
gtgaggattg gaatgtagtg atgatttttt tattttttat gttttttttt attttatttt 960
tttttttgtt ttggtatttt tgtttttgtt tttagagtta ggatatattt gttttttttt 1020
gtttggttaa aaagtagatt attatgaaaa gttttgttgt tattttgtaa tttaggaggt 1080
ttttttgtta ttatattttt ttttttattt agatggtaat ataataatat ttattaaggg 1140
ttttttttgt gttttaggta ttgtgtaaag ttttttttat gaattaagtt ttgtaatttt 1200
atagcgattt tagaagatag gcgttattag ttattttatt ttatagatgg gggaattgag 1260
gtgtagagag gttaagtttt ttgtttatgg tgtatagttg gaagagatag agttggagtg 1320
tgaatgcggt tgggtaggtt ttagtgttta ggttattttt tttatataag atttgttttc 1380
ggtaatttta aagttttttt tggtggtggg tttagttttt aaattggtat cggatgtatt 1440
tttagttaga tattttttgt ttgtcggttt ttatttattt atttatttat ttatttattt 1500
atttattcgg tatttattga gggtttggtg tagttttggg gatgtttttc ggggaggaaa 1560
tagggaaaag aaagattttt ttattaagta tggattatag aaaagataag gttaaatggg 1620
ggtttgtggg attttagagg aaattttatt ttttgaggtt ttggatatga agagtatgtt 1680
gttttttttt tttgtatgag aaaagatggc gttttagagg aagggttgtt ggggtgaggg 1740
atttgggaga tgttttagtt tggcgtttgt atagttagtt tttagttaat cggttttttt 1800
aggttttggt tgtgtttatt attatttatt taataggtat taattgagta tttgttgtgt 1860
gttaggttta gaataggttt aggtgagatg tataaagaag ggtaaattag aatttttgtt 1920
tagatattga cggattagtt gttttatatg taaattgtag tattaagatt tgttgttttt 1980
gtttttagtt ttatttgttt gtgaagattt ttttaaaaga tttgagagta gataaaaagt 2040
agagattatt attgaagaat agggttgttt tggtttttta ttattttaga ttttggaaga 2100
aaatgatttt tttttttttt attggtattg gaggtggtat agtttgtttt tagtaagtta 2160
gcgttggagg gcgtgtgtag ggttggggat cgagtttggt ttttgttttt tgttttgtag 2220
ggtttaagta tttgttgttt ttttatttgg gatgtttttt tttggaaaag tttgtttttt 2280
ttttgttttt taggatttag gttagtggta ttttttttaa aaattttttt ttttattttt 2340
tattatttta ttttgtttat ttgcgttttc gtttttattg tttgttattt attgtaggtt 2400
gaagtgattt aggtttttta gttgtatatt tttagatgga ttttggacga ttgtggtatt 2460
tttgtaattt ttttagtttt tttggggtag gatttttgtt tgttaggatg tttatttttt 2520
tttttttttt ttgtatgttt ttttttgttt ggtttttgaa ttgtttttag agagagtgat 2580
agataagatt tgtttttttt ttagtttttg aattttattt aaggtttttg ttttgttttt 2640
ttggtttgga ggtcggtttt ttgatggagt ttgttatgtg ggttcgttta tggttatgtt 2700
tttttgttta gtatggtgtt tggttttggg attggttata taatatttgg gttaggtgta 2760
aaattagtac ggggtagggg gtattttgtt tataggtgat ttagaattat atatggtgat 2820
tttagagtag gaaattaagt gtggggtttt taagagttgg ggggttttgt acgattgttt 2880
aggttgtagg ttttatagtt cgttttttga tattttgtgt tttatgtttg tttgttgaag 2940
gaaggagtga atggatgaag agtaggtggt gggggtggtt tgaggttttt gtttggtggg 3000
tgggtagagg tttttttttg gtatggggtt taagatttgt tttattttat agtttggggt 3060
ttgtgtgtaa atggttagga tttgtaggtt ggtatttttt tgttttttgt ttggtttttg 3120
gttttttttt tttttattta tgtggttttt taggttgtta tttagtttaa aagtttttaa 3180
gggagattta gagggttatt tggttaaatt atttttgttt tagaaaattg tagaagatta 3240
taattttttt ttttagtttt tttgttttag gaaggatagt tttaaagtga ggtttagtta 3300
gagttttttt tagataagcg ttttcgtttt tttaatttta gtttttttta gtttatttta 3360
aaggtttttt ggggatttat ttttttattt agttttagga ggggaaggag ataggatgaa 3420
tttttatttc gttgttttta ttgttatttt gggtgtagta atttttttga gattttatat 3480
cggtagaggg atcggtgggt tttgagtggt ttggggattt tttgtgatag cgtgtatggt 3540
tcggtattga ttgagggatg aatggatgag gagagatagg agaggaggtc gatggggagg 3600
ttttaggtat agatttttgg aggggaagag gatgtgaaga ttagcggttg gttttttagg 3660
tattgttacg aggagggttg atgggaagtt ttagtggtgg ggttggggtg tttggtttta 3720
ggttgagggg tggttggaaa gatatagggt ttcgaagagg aggaggtggg aagaattttt 3780
ttagtttata cgtagtttat ttatttattt aataaatcgt gattgcgtag ttatagtggt 3840
tattaggcgt tgggtttaag gtattgcggg tattagaggt gcggagaaga tcgttgattc 3900
gggttttagt gttttgggtg tttagcgggg gtaagaaggt aataaagaag gtacggagta 3960
atttaaatag taattttaga tagtaagaga aattatagga aagaaaataa acgtgcgagg 4020
ggcgaggcga ggaaataatt ttagtttggt aggttttgga ggtttttggg aggagaaagt 4080
agcgtttgat gggggcggga ggtggtgagt ggggagaggt ttaggcggag ggaatggcga 4140
gcgtagagat aggttggtaa cggttttagg gaggcgcgga ggggttagcg tggttggttt 4200
aaaaggatat agggattgag gggtaagatc ggtttaaggg ttatcgtttt taggaagttt 4260
tttattttcg cgttattttc gcgttttttt aatttttttt atcgcggttc gtagtttatt 4320
cgttttgttc gggtcgtttt tttggttcgg atcgcgagtg tcgagagggt agggtcggtt 4380
tcgatttttt tagtcgtatt ttcgcgacgt ttcgttaggt tttaggtatt tcgtgggtat 4440
ttagtaaata tttgtcgagc gttttagagg gaatgaatga atttattggg tatagttggg 4500
gggagggcgg ggtcgagggt aggtgggagg tcgtcggcgc gggaggggtt tttcgaagtt 4560
cgtttttttt tttttttttt ttcgtttagg ttttagcgcg tattattttg gcgttttcga 4620

ggcggttttt tgtgcgattt agggcgtata aggttgggag agcgtttcgg ggtttttgtt 4680
attcgcgtcg gaggttggaa gagggtgggt tgtcgtcgtt cgagggcgag agcgttagag 4740
gagcgggaag aaggagcgtt cgttcgttcg tttgtttttt cgttgttttt tcggcgttgg 4800
ttttttggat ttttaggttt gttggttgtt ttttttattc gcgttcgttt ttttattcgt 4860
tttttcgttc gtcggggttg ttttttaagt tttgcggtgc gttcgggcga gtgcggggcg 4920
aggggttcgg ggttagtatc gagtaggggg cgggggttcg ggtagagcgc ggtcggtcgg 4980
ggaggggtta tgtttggcgc gggcgtagcg gggttcgttt gtagtaagtg atcgagcggc 5040
gcggacggtc gtttgttttt tttgttattt ggggcggtgc gggttcggag ttcggagttc 5100
gggtagcgcg tagagtcggc gcgatgtacg tgcgtttatt gcgagttgcg gcgtcgtata 5160
gtttcgtggc gttttgggta tttttgtttt tgttgcgttt cgttttggtc gattttagtt 5220
tggataacga ggtgtattcg agttttattt atcggcgttt tcgtagttag gagcggcggg 5280
agatgtagcg cgagattttt tttattttgg gtttgtttta tcgttcgcgt tcgtattttt 5340
agggtaagta taattcggta tttatgttta tgttggattt gtataacgtt atggcggtgg 5400
aggagggcgg cgggttcggc ggttagggtt ttttttattt ttataaggtc gtttttagta 5460
tttagggttt ttttttggtt agtttgtaag atagttattt ttttatcgac gtcgatatgg 5520
ttatgagttt cgttaatttc ggtgagtaag ggtaggcgag ggtacgcgtt ttttttcggg 5580
ggtattttga gattgggagg gagggagtcg ttttttttat gtagttcgtt tagtttttcg 5640
tttttggttg aaatcgtagt gtttgttcga gggtttttta tttatagttt tatgattttt 5700
aagttgtgtg cgtttttagg tcgggcgcgt tgggttcggt gagtttgtag gggttattgg 5760
gaaggaggga tttttcgaag ttttttttat gttacgtcgt cggtcgtatt tttggggttg 5820
gaggtaaggg tcgtttaaag cgcggggttc ggttatgtga gttgtttcgg gtcggcgcgg 5880
ttcgcgttat ttggatgtaa agggtttttt tcggcgaggt tgttttgtcg ttttttttgg 5940
gtttttttta gttttgtttg gttttggtat cgcggtcgtc gtattttttt attttttttg 6000
ttaagtttta tttgtttttt cgtggtgcgt tcgttttagc gtatcgcgcg tttcgagcgt 6060
tttggggttt tttttcgggt cgtcggatgt tttatttttt tttggttgga gttggggaag 6120
aaacggtgtt attgttaatt ttttttgttt tttttttttg tttatttttt tttttttttt 6180
tttttttttt tttttttttt tttttttttt ttttgagacg gagttttatt tttgtcgttt 6240
agtttggagt gtaatggcgc gatttttgtt tatcgtaatt tttgtttttc gggtttaagc 6300
gattttcgtg ttttagtttt tcgagtagtt gggattatag gtatgcgtta ttatgtttgg 6360
ttaattttgt atttttagta gagatagggt ttttttatgt taggtaggtt ggtttcgaat 6420
tttcgatttt aggtgatttt ttcgttttag ttttttaaag tggtgttggg attataggcg 6480
tgagttattg tgttttgtcg ttagtttttt attttaagta tttagtggta ggtttcgggt 6540
t 6541

<210> 46
<211> 6541
<212> DNA
<213> Artificial Sequence

<220>
<223> chemically treated genomic DNA (Homo sapiens)

<400> 46

ggttcgggat ttattattaa atatttaaaa tagaagatta gcggtagggt atagtggttt 60
acgtttgtaa ttttagtatt attttgggag gttgaggcgg gaggattatt tgagatcggg 120
agttcgagat tagtttgttt aatatggaga aattttgttt ttattaaaaa tataaaatta 180
gttaggtatg gtggcgtatg tttgtaattt tagttattcg ggaggttgag gtacgagaat 240
cgtttgaatt cgggaggtag aggttgcggt gagtagagat cgcgttattg tattttagat 300
tgggcgataa gagtgaaatt tcgttttaaa aaaaaaaaaa aagaaaagaa aagaaaaaaa 360
aagaaaaaag aaaaaaataa ataaagaaag aaaataaaga aaattagtaa tggtatcgtt 420
ttttttttag ttttagttaa gagagaatgg ggtattcggc ggttcggaga ggggttttaa 480
ggcgttcgga gcgcgcggta cgttaaggcg ggcgtattac gaggggatag gtagggtttg 540
atagggaggg taagggggtg cgacggtcgc gatgttagag ttaggtaggg ttgagagggg 600
tttaggaagg gcggtaaggt agtttcgtcg ggaagggttt tttatattta ggtagcgcga 660
gtcgcgtcgg ttcgggatag tttatatgat cgagtttcgc gttttgaacg gtttttgttt 720
ttagttttag agatgcggtc ggcggcgtaa tatggagggg atttcggagg attttttttt 780
tttagtaatt tttataggtt tatcgaattt agcgcgttcg atttgggggc gtatatagtt 840
tgggagttat agggttgtgg gtgggagatt ttcgggtagg tattgcgatt ttagttagga 900
gcggaaagtt gggcgggttg tatagaagaa gcggtttttt tttttttagt tttaaagtgt 960
tttcgaaagg agacgcgtat tttcgtttgt ttttatttat cgaggttgac gaagtttatg 1020
attatgtcgg cgtcggtgag gaaatggtta ttttgtaggt tggttagagg ggggttttgg 1080
gtattgaaga cggttttgta ggggtaggag aagttttggt cgtcgggttc gtcgtttttt 1140
tttatcgtta tggcgttgta taggtttagt atgaatatgg gtgtcgagtt gtgtttgttt 1200
tggaggtgcg ggcgcgggcg gtggggtaag tttaaaatgg agaggatttc gcgttgtatt 1260
tttcgtcgtt tttggttgcg gaggcgtcgg tggatgaagt tcgagtgtat ttcgttgttt 1320

aggttgaagt cggttagggc ggagcgtagt aggaataggg gtgtttagag cgttacgaag 1380
ttgtgcggcg tcgtagttcg tagtgagcgt acgtgtatcg cgtcggtttt acgcgttatt 1440
cgggtttcgg gtttcgggtt cgtatcgttt taggtggtag aggggggtagg cggtcgttcg 1500
cgtcgttcgg ttatttgttg tagacgggtt tcgttgcgtt cgcgttagat atggtttttt 1560
ttcggtcggt cgcgttttgt tcggattttc gttttttgtt cggtgttggt ttcgggtttt 1620
tcgtttcgta ttcgttcggg cgtatcgtag ggtttggaaa gtagtttcgg cgaacgaaaa 1680
ggcgagtgag gaggcgggcg cgggtgggag gagtagttag taagtttagg agtttagaga 1740
gttaacgtcg gggaggtagc gaggaggtag gcgggcgggc gagcgttttt ttttttcgtt 1800
tttttggcgt tttcgttttc gggcggcggt aatttatttt tttttaattt tcggcgcgga 1860
tagtaggggt ttcggggcgt ttttttagtt ttgtgcgttt tggatcgtat aagaggtcgt 1920
ttcgggagcg ttagagtggt acgcgttggg gtttgggcgg aggaggagga ggaggaggac 1980
gggtttcgag ggtttttttt cgcgtcggcg gtttttattt tgttttcggt ttcgtttttt 2040
ttttagttgt gtttagtggg tttatttatt ttttttagag cgttcgataa atgtttattg 2100
agtgtttacg gggtgtttag agtttggcgg gacgtcgcgg ggatgcggtt ggaggaatcg 2160
gagtcggttt tgttttttcg gtattcgcgg ttcggattag gaaggcggtt cgagtaggac 2220
gggtgggttg cggatcgcgg tgggaaaagt tgggggagcg cggaggtggc gcggaaatag 2280
aaggtttttt ggaagcggtg atttttgagt cggttttgtt ttttagtttt tgtatttttt 2340
taagttagtt acgttgattt tttcgcgttt ttttgaagtc gttgttagtt tgtttttgcg 2400
ttcgttattt ttttcgtttg gatttttttt tatttattat ttttcgtttt tattagacgt 2460
tgtttttttt ttttagagat ttttaagatt tgttaagttg aggttgtttt ttcgtttcgt 2520
ttttcgtacg tttgtttttt tttttgtagt tttttttgtt gtttggaatt gttgtttgag 2580
ttatttcgtg ttttttttat tgttttttta ttttcgttag atatttagag tattggggtt 2640
cggattagcg atttttttcg tatttttggt attcgtagtg ttttgaattt agcgtttggt 2700
agttattgta gttgcgtagt tacgatttgt tgagtgaata agtgaattgc gtgtgagttg 2760
gggggggtttt ttttattttt ttttttcgg ggtttgtat tttttagtt atttttagt 2820
ttgagattag atattttagt tttattatta gggtttttta ttagtttttt tcgtggtagt 2880
gtttggggag ttagtcgttg gtttttatat tttttttttt tttaagggtt tgtgtttgag 2940
atttttttat cggttttttt ttttgttttt ttttatttat ttatttttta attaatatcg 3000
agttatgtac gttgttatag ggagttttta gattatttag aatttatcgg tttttttgtc 3060
ggtgtgggat tttaagggaa ttattgtatt tagagtggta gtgagggtag cggggtaaaa 3120
gtttattttg tttttttttt tttttggggt tgggtgagag agtgggtttt tagagggttt 3180
ttgggatgaa ttgggaaggg ttgaggttgg ggaagcgggg gcgtttgttt gggaggggtt 3240
ttggttaagt tttattttgg ggttgttttt tttggagtag gagagttggg gaagagaatt 3300
atggtttttt atagtttttt ggagtagaag tagtttggtt aagtggtttt ttgggttttt 3360
tttggggatt tttggattag atggtagttt gaggggttat atgggtggag aaaggggagg 3420
ttagaggtta ggtaaggagt agaaaaatgt tagtttgtag gttttggtta tttatatata 3480
ggttttaggt tgtgggatgg aataggtttt gagttttatg ttagggaggg gtttttattt 3540
atttattagg taaggttttt aaattatttt tattatttgt tttttattta tttatttttt 3600
tttttaatag ataagtatgg agtataggat attaggaggc gagttgtggg gtttgtaatt 3660
tggatagtcg tatagggttt tttagttttt aagggtttta tatttggttt tttattttga 3720
ggttattata tgtggttttg aattatttat gaataaagta ttttttgttt cgtattaatt 3780
ttgtatttgg tttagatatt atgtggttag ttttagggtt aagtattatg ttgggtagga 3840
agatatggtt atgagcgaat ttatatggta gattttatta aggagtcggt ttttaggtta 3900
gggaagtaaa gtaagagttt taaataagat ttagggattg aaggagaggt agattttgtt 3960
tattattttt tttggaaata atttagaagt taggtagagg aggatatgta ggagggagaa 4020
ggaaaggtgg gtattttggt aagtaggaat tttattttag ggagattggg gaaattgtag 4080
gagtgttata gtcgtttagg gtttatttga gagtgtataa ttggagagtt tgggttattt 4140
tagtttgtaa taagtgatag gtagtggggg cgggggcgta gataaatagg gtgaggtgat 4200
ggagggtggg aaggggagtt tttggaggag atgttattga tttgagtttt gaaagataag 4260
aaagagatag gtttttttag ggaaaggtat tttaggtgga ggaatagtaa gtgtttgagt 4320
tttgtagagt agggaataga aattaggttc ggtttttagt tttgtatacg ttttttagcg 4380
ttggtttgtt agggataggt tatgttattt ttagtgttag tagagaaaaa ggaggttatt 4440
tttttttaaa gtttgaaata ataaggagtt aaagtagttt tgttttttag tagtagtttt 4500
tgttttttat ttatttttaa attttttgga gggattttta taagtaggtg aggttggggg 4560
taggggtagt aggttttggt gttataattt atatatgaaa taattgattc gttagtgttt 4620
aagtaaggat tttagtttat ttttttttgt gtattttatt tgagtttatt ttgagtttgg 4680
tatatagtag gtgtttaatt agtatttgtt gaatgaatag taataagtat agttaaaatt 4740
taaagagatc ggttgattga gggttgattg tgtaggcgtt aagttaaggt attttttaga 4800
ttttttattt tagtaatttt ttttttgaga cgttattttt ttttatgtaa gaggaagaga 4860
taatatgttt tttatattta agattttaag agataaggtt ttttttgaag ttttataaat 4920
ttttatttag ttttattttt tttgtgattt atgtttggtg ggggggtttt tttttttttt 4980
gttttttttt cgagaggtat ttttaagatt gtattaggtt tttagtgaat gtcgaatgaa 5040
tgaatgaatg aatgaatgaa tgaatgaatg aaaatcggta agtaagaaat atttggttgg 5100
gagtgtattc gatgttagtt tgggagttga gtttattatt agaaaaggtt ttgagattgt 5160
cgagggtaag ttttgtgtgg aggaggtagt ttgggtattg gagtttgttt agtcgtattt 5220
atattttagt tttgtttttt ttagttgtgt attatgggta agggatttaa tttttttata 5280

ttttagtttt tttatttgta aaatggggtg attgatagcg tttgtttttt gggatcgttg 5340
tgaggttgta gaatttaatt tatggaaagg gttttgtata gtgtttggaa tatagagaaa 5400
atttttgata aatgttattg tgttattatt tggatgaaaa ggaaaatatg gtaataggaa 5460
agttttttga gttataaaat aataataaaa ttttttatga tggtttgttt tttggttaag 5520
tagagaaaag tagatgtatt ttgattttga gagtaagagt aaaagtatta gagtaaaaga 5580
gagagtgaag tggaagggaa tatgagagat gaaaaggtta ttattgtatt ttaattttta 5640
tattgtgttt aggttttttg taggttagtt ggggtttagg gatttttttg ggttttattt 5700
tattttttta gtagttagtt tttaatttat atatttttag ttgtggtttt tttggttagg 5760
gtatttagta ttgggtttta ggatcgtaat ttgatttgtt ttagtttgtt ttatatttgg 5820
ggtcgttttt gtttggatga aagacggttt ggaattgtaa ggggtgttat tgtgtcgggt 5880
ggttcggttt tagtgttcgc gtttttcgtt tgttggtttt ttagtgttgg gttaggagtt 5940
tttatttttt atattattag ttatattttg ttgaagtgat tttgattaag ttagtatgtt 6000
tttttaaggt agtaaaaaga agaaaagaat tttgttgata tatatattgt ttagcgaggt 6060
ggggatttta tttttattta tttgttttag aggaggttta gttggtttgg aagttttttg 6120
gggagtatag ttaaggaagt tagtgggttg tttttttttt gtttatatta tttggtaaag 6180
gttagatgat ttggattttt aattttaata aggaatagag ggttaagagt tgttttttgt 6240
ataggtttga aaatcgttgt gtgtagaggt ggattttgta gaggaaatta ttagagtata 6300
tttgtggtga aattttgttt tttacgtaga ttttttattg tacgggaggt ttggattttt 6360
tacgtttgtt tattttgaga ttttttttgt ataattttaa aagtagtaga ataagagatt 6420
aaacgttttt tattagtttt aagtatatat ggtagtggtt atagttttaa atggaaaata 6480
tagacgatat atatatttta gagaaagttg atattatgta ggatttttag gatttggaag 6540
t 6541

<210> 47
<211> 5942
<212> DNA
<213> Artificial Sequence

<220>
<223> chemically treated genomic DNA (Homo sapiens)

<220>
<221> unsure
<222> (3085, 3087..3088, 3102, 3104, 3124, 3161, 3297)

<400> 47

ttaatgtagg ttgaaagaag ttagttataa aatatcgtat gttgtattat ttttattttt 60
atgaaaggtt cggaaaaggt aagtttatag agaaagtaga ttagtgataa aaatgtattg 120
gaattagtgt tgatggttgt ataattttgt gaatatgtcg atagttattg aattgtatat 180
tttaaaagag tgaattttat tatacgttaa ttatattttt aaagaatgga ataaagtttt 240
aggggttttt aaagtggtga ttttaaatat tggttttagt gttaagaaaa tgaatggtta 300
tagtttttgg agaataaatt tttttatggt tttttagttt tttattttta ataaaattga 360
aggattaatt ttaatattta taaataataa atgtgttagt tattaaatta tttaaaatat 420
ttttggtgga aaataattat gtaattttta atagataatt tagaagaaat tttaaaagtt 480
atgtgattta attgatagaa ttttttttat ttttattatt tgtttatatg agtaagtttt 540
tttagtgttt gttttttttt tttttttgag acggagtttc gttttgtcgt ttaggttgga 600
gtgtagtggc gtatgttggt ttattgtaag ttttgttttt cgggtttacg ttattttttt 660
gttttagttt tttgagtagt tgggattata ggagttcgtt attacgttta gttaattttt 720
tttatttttt agtagagata gggttttatc gtgttagtta ggatggtttt aattttttga 780
tttcgtgatt ttttcgtttt agttttttaa agtgttggga ttataggcgt gagttattat 840
atttggttga tttggatagt tttgatattg tttttatgta tattaagatt tgtaggtagt 900
ttttttatta gtaattaaaa tgttttgtaa tgtattttaa gagtataggg ttttgagtgt 960
tttaaatttg tatataagaa tgttttagat ttttggttaa aagaagtttt tgttagaaaa 1020
tattaaagta aaggttgtta aattttggtg aggtttttaa tgtttgtatt ttttaagaat 1080
tatttaaaaa tgttatgaaa taaaaatatt ttagtggtaa ttgtttagaa gtattttaaa 1140
tgtatattaa attaatgaaa tataatcgtt gggtgataga ttattttttt tttattttat 1200
aattttttat ttaataaaaa tgtgggggaa aaattgtatg ttgaagttgt acgtgtatat 1260
tttagtaaaa attgttaatt tttttgaagt attgttttgg ttattatttt ttatttgtta 1320
aattattttt ttttgttttt ttatttgttt ataatagaaa atttagaata ttttggattg 1380
gttttttgtt ttggttaagt tagttttttt tgttttgttt ttttttttat tattttatta 1440
gtttaagtta cgtaaatatt cgatttaggt ttgggtttag aatttttaga tttttattgt 1500
ttattataat agatagttat tattatatgt ggttgttgaa gatttgaatt gtggttagtt 1560
taagtgtgaa ttacgtatta gattttgaag atagtattaa aagatgtaaa atattttatt 1620
atgtttttaa aatattggtt atatagtgag atgatattgt tagggatata ttggattaaa 1680
gaaatatatt aaaattgatt ttggtcgggt atggtggttt atgtttgtaa ttttagtatt 1740

gtgggaggat tgtttgagtt tagtagtttg agattagttt gggtaacgtg gtgagatttt 1800
gttttagtta aaaaaataaa attgatttta taggattttt tttatttttt aaatgtggta 1860
attagaaagt ttaaaattat acgttatatt ggatagtatt gtttttgatt tttttttttt 1920
tggttttttg tttattagaa tgttttaaaa tttagtataa atttttggtt aggtgttgtg 1980
gtttacgttt gtaattttag tattttggga ggtcgaggcg ggaggattac gaggttagga 2040
attcgagatt agtttgatta atatggtgaa attttatttt tattaaaaat ataaaaatta 2100
gtcgggtgtg gtggtacgtt tgtaatttta ggtatttagg aggttaagat aggagaatta 2160
tttgaatttg ggaggtggag gttgtagtga gtcgagattg tattattgta ttttagtttg 2220
ggcgatagag taagattttg ttttaaaaaa aaaaaaaaaa aaaaaagaat attttttttt 2280
tttttatgaa gtttttttta gtggtttaag ttttatagtt ggttttttgt tgtattatta 2340
tttgaagata attttttggt atattagatt tttttattat aaattttttt agaattttta 2400
atatgttaat atatatggtg aaattttagt atagtttttt taattacgta ttattataga 2460
attgtgtgtt ttttgttaat agaagttata tatatttgtg gttgaaattt taaataatat 2520
agaaatgaag taatgaaagt tttttgtaat tatgttttat ttgtgtttta agattgttat 2580
tgttgaaagt atagagttag aaaaaatttt tttttttgtt gttttttttt tttttttttg 2640
tttttatttt ttaaaatggg gttatacgat atatttttat ttttaaattt atttttattg 2700
aatatatagg gggatttttt ttatgttagt aaattttaat ttttttattt tttttttttt 2760
ttttgaggga aggttttgtt ttgttgttta ggttgtagtg gtgttgtggt gtaaatagtt 2820
tattgtagta tttaattttt gggtttaagt aattttttta ttttagtttt ttgaatagtt 2880
gggattagag gtgtatgtta ttatgtttag ttaattaaaa aatttttgtg tgtgtggagg 2940
tgggatttta ttatgatgtt taggttggtt tcgaattttt gggttaaagt gattttttta 3000
tttagttttt taaaggttga gattataggt gtgaggtgtt gtgcgtagtt ttaattattt 3060
ttattttttt tattagtagt taagntnnag ttatttttat tngntttttg attttaaatt 3120
taantttttt tttgaaaaat ttttgttaat ttgttttttt nttttttttt aaaaaaaaaa 3180
aaaaaaaaat aaaaaaaaag gggttttttt tttttttttt tcgggatttt ttttttttaa 3240
aaaaaaaaaa gggggggttt tttttttttc gtggttaatg gaaataatta agattanttt 3300
ttattttttt gtttttgtat tgtaaataaa tatttatatt agaatataag ggaatatttt 3360
ggaatttaaa tattttttta ggtaagaatg tgtgttaata taaatgtttg gtatattgtt 3420
tttttttttt tttttttttt atttagttta tttagtttta aaagtgattt tttaaaatgt 3480
atttttaaaa gaatattttt tgttaagtga tttaaatttg atatattttt tggattatta 3540
aggaggttaa agttatattt ggtgaaaaag gggtttattt tttttttttt tatatacggg 3600
ttatcgaata tttttaaaag tattttttat tatgaatgag gggaaatgga agaaaagagg 3660
tggtgataat tatttgtatt ttttaaatgg acggaatgag ttttcggagg gtatacgagg 3720
ttattagcgt gagttcgcgg tggagttgga ggcgtattta tagttggttg attttagttt 3780
tgttgggttt tcgtcgtttt ttattttatg tcgttggaat tcgcgggttc gtaggttata 3840
ggagttcggg cgcgtcgggt ttggcgtttt cgaatcgatg agggaagtcg tttttttcgg 3900
tagatttttt cggtcgggga gtttttatta ttttgtttgc gtttcggtac gttggtaagg 3960
agttcgggaa gagacgtcgg gagcggtggg tgggcgagaa ataaatgtaa tgtgatttat 4020
attttttttt gtcgtaatta ttacgatttg gttatttttt attttggtga tttggtgttt 4080
ttttagcgat tttattttac gttagttgat tggcgtttat cgtagtgtga gtgtcggtcg 4140
gaggaggggt ttacgcgaaa gagttcgcga gagtggggat attcggtttc gaaggtcgga 4200
gcgggcgttt agggcgtcga tggtcgtagc gttcggtagt cgtgtttacg ttttttttcg 4260
tcgcgtttta tcgcgtgtta tttacggcgt ttgcgggaag ttattcgggc ggtagttgtg 4320
gggttggttt gtggggaggg gtcggcgggt tcgcgcggag gagtagtttc gattttcgtt 4380
cgtattagta gttcggcgtt tttttgtcgg cggttggttt tttttttcgg taattgggcg 4440
tcgggtttcg cgaatttttc gggcgttttc gtcgtgcgtt cggttttttt cgtgttcggg 4500
gcggggcggg gtaggcgttc gtggtcgggt gcgcgcggcg cggcgggggg tttttttagt 4560
gcgcgcgcgt cgtgtaaggt tcgcggacgt tcgttttttt tttaggtttt cgtattcgcg 4620
tcgttttttt tttttttggt aagagtcggc ggcggtggcg gcggtcgttg tagagattgg 4680
aattcgtttg tcgggtttgg cgaaggagaa gggaggaggt aggagcgagg agggaggagg 4740
gttaagggcg ggtaggaagg tttaggttcg gcgcgttcgt tcgcgcgcgg cgaagatcgt 4800
acggttcgat cgaggggcga tcgggtcggg gtcgttgtac gttaagggcg aaggtcgatt 4860
cggggtttta tttcgtttcg gcggttcgtc gcgtttattc gtttcgcgtc gagggttgga 4920
ggatgcgttt tttggggttc gggtgagttg ggagtgcgcg tgcggatcgg aggggttggt 4980
cgtagcgggg aggttagagg ggttcgggcg aggttgtaga ggtagttggg cgcgcggtgg 5040
tcggagttag gggttgttcg gttcggtttc ggtagtttcg taggtttcgg cgcgcgttgg 5100
gtcgggatcg tttagttaga gatttacggt atttcgcgtc gcgtttttcg tcggtttttt 5160
ttttgttttt tattcgcggg ttgtaggtcg agtttgggga tgcggagtgg gcgaaagtcg 5220
tttcggggac gtttttggaa tttttaaatg ggaaaggcgt ttattttttc gtttcggagc 5280
ggcgaggttg atatttgtga tttggtttcg gacgttgtgg ggaggaatag gggagaataa 5340
aatgagtttc gggcgggggt cgagtgagag gagtagtcgg ttcgttttcg agttttcgcg 5400
ggaaggggtt ttggttttcg agttttcgga gttgttttat ttgttgtttt gcgcggtttt 5460
cgtttcgtgg tttggtgatt tcgtatttta gttttggaaa attttttgtt ggattgtttg 5520
gatttttttt ttttttttta gatttttagt ggaaagattt ggtaaggaaa aaaaaatgag 5580
tttgcgttag ggaagaaaaa taatatgttg tgtgttaggg tttggcgttt gagcgtggta 5640
tttttttatg tttttagatg ttttaatttg gttaaatttt tgtaggtttt tttttttaag 5700

cggttaataa attgtttaga gtatatagta tgaatttggt atttgtgtta cgttatttaa 5760
tttttataac gattttggtt tattgtattt tttattgttt taggggaaga aatcgagtcg 5820
taaagaggtt aattaatttg tttaagggag ttgggatttg aatttaggaa tatcgtattt 5880
tggtaaacga attaggaaga aaaaaaatta aaattgtttg taaatatttt tacgtttagg 5940
aa 5942

<210> 48
<211> 5942
<212> DNA
<213> Artificial Sequence

<220>
<223> chemically treated genomic DNA (Homo sapiens)

<220>
<221> unsure
<222> (2646, 2782, 2819, 2839, 2841, 2855..2856, 2858)

<400> 48

tttttaaacg taaaagtgtt tgtaaatagt tttggttttt ttttttttta attcgtttgt 60
tagaatacga tgtttttggg tttaaatttt agttttttta gataagttag ttaatttttt 120
tgcgattcgg tttttttttt taaaataatg aggaatataa taaattagaa tcgttgtaag 180
gattaaatga cgtaatatag gtgttaagtt tatattatgt attttgaata gtttgttaat 240
cgtttaaaag agaaaatttg taggagtttg attaaattaa aatatttgag agtatgaaag 300
agtattacgt ttaaacgtta gattttaata tatagtatat tatttttttt ttttgacgta 360
agtttatttt tttttttttg ttaaattttt ttattaggag tttggaaaga gaaaaaaggg 420
tttaggtaat ttagtagaaa gttttttaaa attgaagtac ggaattatta ggttacggag 480
cggaggtcgc gtaggataat aggtgaagta gtttcgggag ttcgggggtt agggtttttt 540
ttcgcgggga ttcgggggcg ggtcggttgt tttttttatt cgattttcgt tcgaaattta 600
ttttgttttt ttttgttttt ttttatagcg ttcggagtta aattataaat attagtttcg 660
tcgtttcggg gcggggaggt gagcgttttt tttatttaag gattttagaa gcgttttcgg 720
agcgattttc gtttatttcg tatttttaaa ttcggtttgt aattcgcgag tgggagataa 780
aagaggggtc ggcgggggac gcggcgcggg gtgtcgtggg tttttggttg ggcggtttcg 840
gtttagcgcg cgtcgaggtt tgcggagttg tcggggtcgg gtcgggtagt ttttggtttc 900
ggttatcgcg cgtttagttg tttttgtagt ttcgttcgag tttttttggt tttttcgttg 960
cggttagttt tttcggttcg tacgcgtatt tttaatttat tcggatttta gggaacgtat 1020
tttttagttt tcggcgcgga gcgggtgggc gcggcgagtc gtcggggcga agtggggttt 1080
cgaatcggtt ttcgtttttg gcgtgtagcg gtttcgattc ggtcgttttt cgatcgggtc 1140
gtgcgatttt cgtcgcgcgc ggacggacgc gtcgagttta agtttttttg ttcgtttttg 1200
gttttttttt tttttcgttt ttgttttttt tttttttttt cgttaagttc ggtaggcgga 1260
ttttaatttt tgtagcggtc gtcgttatcg tcgtcgattt ttgttaggag gggagggggc 1320
ggcgcgggtg cgggggtttg ggggaggggc gggcgttcgc gggttttgta cggcgcgcgc 1380
gtattgggaa ggttttcgtt cgcgtcgcgc gtattcgatt acgggcgttt gtttcgtttc 1440
gtttcggata cgaaaagagt cgagcgtacg gcggaagcgt tcgaagagtt cgcggagttc 1500
ggcgtttagt tgtcggggag gagggttagt cgtcggtaag ggggcgtcga gttgttggtg 1560
cgggcgggag tcggggttgt tttttcgcgc gggttcgtcg gttttttttt ataagttagt 1620
tttatagttg tcgttcgggt agtttttcgt agacgtcgta ggtagtacgc gatggaacgc 1680
ggcggagggg agcgtggata cggttgtcgg gcgttgcggt tatcggcgtt ttggacgttc 1740
gtttcggttt tcggggtcgg atatttttat tttcgcgggt tttttcgcgt ggattttttt 1800
ttcggtcgat atttatattg cggtgaacgt tagttagttg gcgtgaaata aggtcgttgg 1860
gaaggtatta agttattaag gtgggggatg gttaggtcgt ggtagttgcg ataaagaaaa 1920
atataagtta tattgtattt gtttttcgtt tatttatcgt tttcggcgtt ttttttcggg 1980
ttttttgtta gcgtgtcgag gcgtaggtag ggtgatggag gtttttcggt cgggaggatt 2040
tgtcggggag agcggttttt tttatcgatt cgggagcgtt aggttcggcg cgttcgggtt 2100
tttgtgattt gcgagttcgc gaattttagc ggtataagat aaggagcggc gagggtttaa 2160
taggattgag gttagttagt tataaatacg tttttagttt tatcgcgggt ttacgttggt 2220
gatttcgtgt atttttcgga ggtttatttc gtttatttgg aaaatgtaaa taattgttat 2280
tatttttttt tttttatttt tttttattta tggtggaaaa tgtttttaaa agtattcgat 2340
gattcgtatg tgaggaaaga ggagataggt ttttttttta ttaaatgtga ttttaatttt 2400
tttagtaatt taagaaatgt attaagttta agttatttaa taaaagatgt ttttttaaag 2460
gtgtatttta aaaaattatt tttaaaattg ggtaaattgg gtaaaaagaa agggggaagg 2520
aaaataatgt gttaggtatt tgtattgata tatatttttg tttgaagagg tgtttagatt 2580
ttagggtatt tttttatatt ttggtataaa tgtttatttg taatgtaaaa gtaaaaaaat 2640
ggaaantaat tttaattatt tttattaatt acgaaaaaaa aaaagttttt tttttttttt 2700
ttttgggggg ggggggtttc gggggggggg gggggggggt tttttttttt ttgttttttt 2760

tttttttttt ttgggggggg gnagaaaaat aggttaataa aaatttttta aaggggggnt 2820
taaatttaaa attaggaant naatgggaat aattnngntt taattattaa taaaagaaat 2880
gagaatgatt aaggttgcgt ataatatttt atatttgtaa ttttagtttt tgggaggtta 2940
ggtgggagga ttattttagt ttaggagttc gagattagtt taggtattat ggtgagattt 3000
tatttttata tatataaaaa ttttttaatt agttgggtat ggtagtatgt atttttggtt 3060
ttagttattt aggaggttga gatgggagga ttgtttgagt ttaggagttg gatgttgtag 3120
tgagttgttt gtattatagt attattgtag tttgggtaat agaataagat ttttttttaa 3180
aaaaaaaaaa aagaatgagg aaattgaaat ttattgatat aaaaagaatt tttttatata 3240
tttagtgaaa ataaatttaa agataaggat gtatcgtatg attttatttt aggaaatggg 3300
gatagaaaaa aagaaagaag gtaatagaaa agaaaatttt ttttagtttt atatttttaa 3360
tagtggtaat tttggggtat aggtgaagta tgattataga agatttttat tattttattt 3420
ttgtattgtt tgaaatttta attataaata tgtgtaattt ttgttaatag aaaatatata 3480
gttttatggt aatacgtagt tagggaaatt atattagagt tttattatgt gtattagtat 3540
attaaaagtt ttgagaaggt ttgtagtaaa gaaatttaat atattaaaag attattttta 3600
aataatgatg tagtaaagag ttaattatga ggtttggatt attgaggagg gttttatagg 3660
agagagagga atattttttt tttttttttt tttttttgga gatagagttt tattttgtcg 3720
tttaggttgg agtgtaatgg tgtaatttcg gtttattgta atttttattt tttaggttta 3780
agtgattttt ttgttttagt tttttgagta tttgggatta taggcgtgtt attatattcg 3840
gttaattttt gtatttttag tggagatggg gttttattat gttggttagg ttggtttcga 3900
gtttttgatt tcgtgatttt ttcgtttcgg ttttttaaag tgttgggatt ataggcgtga 3960
gttatagtat ttggttaagg atttatatta gattttaaag tattttgatg ggtaaagaat 4020
tagggaaagg aaagttaaag gtagtattgt ttaatatgac gtgtaatttt aaatttttta 4080
gttgttatat ttaaaaagta aaaagaattt tgtgaaatta gttttgtttt tttaattgag 4140
ataaggtttt attacgttat ttaggttggt tttaagttgt tgggtttaag taattttttt 4200
atagtgttgg gattataggt atgagttatt atgttcggtt aaaattagtt ttaatatatt 4260
tttttaattt aatgtatttt taatagtatt attttattat ataattaata ttttaaaagt 4320
ataatgagat attttatatt ttttggtatt atttttaaaa tttggtacgt aatttatatt 4380
tagattagtt atagtttaag tttttaatag ttatatgtgg tagtggttat ttattgtagt 4440
ggatagtaga ggtttaagga ttttgaattt aaatttgaat cggatattta cgtgatttag 4500
gttgataaaa tagtaggaaa ggaagtaaag tagaaaaggt tgatttggtt agagtaggag 4560
attagtttaa gatattttgg atttttattt gtaagtaaat aagaaaatag ggaaaaataa 4620
tttaataaat aagggatgat gattaaagta gtattttaga aagattaata gtttttgtta 4680
aaatatatac gtataatttt aatatgtaat ttttttttta tatttttatt aaataaaaga 4740
ttataaaatg aaggaagaat aatttgttat ttagcgatta tattttattg atttggtata 4800
tatttaagat atttttgaat agttattatt gaaatatttt tattttatga tatttttaaa 4860
tgatttttga aaaatgtaga tattaagagt tttattaaaa tttaatagtt tttattttaa 4920
tattttttaa taagagtttt ttttagttag gaatttaaaa tatttttata tataaattta 4980
aaatatttaa gattttatat ttttgaaata tattataaag tattttaatt attagtaaga 5040
aaattattta taaattttaa tatatataaa gatagtatta aaattattta agttagttag 5100
atgtggtggt ttacgtttgt aattttagta ttttgggagg ttgaggcggg aggattacga 5160
ggttaggaga ttgagattat tttggttaat acggtgaaat tttgttttta ttaaaaaata 5220
gaaaaaatta gttaggcgtg gtggcgggtt tttgtggttt tagttattta ggaggttgag 5280
gtaggagaat ggcgtgaatt cgggaggtag agtttgtagt gagttaatat gcgttattgt 5340
attttagttt gggcgataga gcgagatttc gttttaaaaa aaaaaaaaaa gtaagtattg 5400
agaaggtttg tttatataaa taggtgatgg aaataaaagg aattttgtta gttgggttat 5460
atgattttta aaattttttt tgagttattt gttgaaaatt atatagttgt tttttattag 5520
aaatatttta aatgatttag tagttaatat atttattatt tgtaaatatt ggaattgatt 5580
ttttaatttt gttagaagtg gaaaattgaa aaattatgaa aggatttgtt ttttaaaggt 5640
tatggttatt tattttttta atattaaaat taatatttgg aattattatt ttgggggttt 5700
ttgaggtttt attttatttt ttggagatat aattgacgta taataaaatt tattttttta 5760
aagtgtgtag tttagtagtt atcggtatat ttataaagtt gtgtaattat taatattaat 5820
tttagtatat ttttattatt aatttatttt ttttgtggat ttattttttt cggatttttt 5880
ataaaaataa gaataatata atatacgata ttttgtgatt gatttttttt aatttgtatt 5940
aa 5942

<210> 49
<211> 14287
<212> DNA
<213> Artificial Sequence

<220>
<223> chemically treated genomic DNA (Homo sapiens)

<400> 49

tttttgttat tttatttttt tatggttttg tatttttttt attttagggt aagggttggt 60

aaattttttt gtaaaaagtt agtagatatt ttaggttttg tgattcgtat agtgttataa 120
ttatttaatt tttttgtttt aggtgaaggt agttatagat aatatataaa tgattgatta 180
taggtatatt ttaataaagt tttatttgta gatattgaaa tttgtattgt atattatttt 240
tatgtattat gaatattatt tttttaattt tttaattatt taaaaatgta aaaaatattt 300
ttgagaattg tataaaaata ggtattaggt taaatttgtt ttatgggtta tagtatgacg 360
atttttggtt tagtggtaat gttgtttttt taagggttga gtaatttttt gttaattaga 420
gtttaaacga aaatttttta aagatcgttt tgtttataaa gtttttttat atgagatatt 480
taaagtttta gggattatta atgtgtttat aattttttat tggttttttt attttaatag 540
ttggtttatt gaaaaatatt ttaagatttt atagggattt attttatttt ttattataag 600
tatatataaa ataatgtatg ggggttagaa aaggaaatta ggataaaagg tttatgggtt 660
ttgtttttag ttagtttttt gattggataa ataatttgat tgttttatat tttatttttt 720
ttgtttagaa agttagaatt gttaggtgtt gttattaaat agttatgtta aatgttaaag 780
gatatggtag ggagtggtgg gtgggattat gtttgtattt tttagagttg ttatattttt 840
agaaaaaaag tgtatatttt ttagtgttat tatatataat tgaaaagatg aatatttttg 900
aagagaaata gggtgggtga tttgttttaa atattttgac gtagaaaatt taaatttaaa 960
gatgatatta agtaaattat agtataaaat atatatatgt aaaattttat ggttttttta 1020
tgtgtttacg attaatagga gaggtttggt ttaggggttt gtaaaaatgt tttttttttt 1080
taagtataat gtttagagtt aagatattat tagttttagg tttagttagt tttagtcgaa 1140
agtgtgaatg tttagtaaaa ggatgttttt gaaaggagat tgaaaattta ttgtgttaga 1200
tatttgaaac gttgttaggg tatttagtgg aaaattttat ttttatatta taaataattg 1260
taggatattt taagagattt aagattagta atttttaaga gttgtttata ttagttttat 1320
tgaaatgtga aatggaggaa ggatattgag aaaggttata aagttgagtt attagatatg 1380
attaattaaa ataaataatt ttattattga ttaaattgtt tttaaaattt ttaattattg 1440
aggattgtat agtttgttat ttattaaata aaataaagga taaaatagcg atggagaaaa 1500
aaggaggaaa agtttttaag gtaatttttt aaatttaatt ttagaatttt ttatttggaa 1560
agtttttttg gtggtttata tttgaaatat taattgtttt ttattgtttt taaagtttta 1620
ggaatttttt gttttatgga ttttttggga ggaggataat tttagagaag gtggtatatt 1680
atttattatg gtagttatgg aattttggtg ttatttttat tatagaaata attaattttt 1740
ataattgttt tttttttttg tagtttttat agatttaaaa ataaaaaacg agttagggat 1800
ttttgttaga tagtggtata atatgtttaa agtaaaatta gttttttaaa aagaaaaaaa 1860
aaaggtagga aaaaaaagtt atgattttat gagtgtgtat gtatttatgt gtgtttgcgt 1920
gtggatttgt aagagtgtgt gttcgagtgt atatgagtgg acgtgtaagg taggatagta 1980
atggatgtag tttttttttt ttttttttga ggcgaaagtt tggttttttt tttcggatcg 2040
gtcgtttgtg ggattcgaga gatttgtgta cgtaggggac gcggcggtgt tcggggttag 2100
agtttttttt tgtgatcgaa aagataatta aaaatcgtac gcgtaggagt tagtttagtt 2160
tttggttgcg tttttagttg gttcggtttg gttaaatggg ttttcgtatt ttggcggcgt 2220
tcgggttggg aggttagtag gtcgggcgcg aagagtaggt tggggattag gtgttatagt 2280
cggtggcgta gaatcgtttt tcggagttat cgagggtggt agtggtaggc ggcggttgtg 2340
gggcgggagg ggcgcggttt ggggttagtg tttgtggagg gatttgggga agaggtagga 2400
acgtggagag aaacgtggga aagggaaaga tcgacgaggg aggcgtggag tagtgtatgg 2460
aggaagggta ggtaaaaata aagttttaat taggaaggat agagtagtat tttttgaatt 2520
ttatttttat ttttcgagag cgtatatttg gttatttcgt atttttattt ttgttttcgc 2580
ggtttggtag atttttagtt tttcgcgtat ttttttagcg gggcgatttc gagttacgta 2640
attatagttt tttaagttta ttttgtattc gaaagggttt tagagttcgc gattggtatt 2700
aggtttttat ttttcgtgtt ttttatgttt ttagagtttt tttaggtttt ttttagggcg 2760
tagattcggg cgtgggttta gcgagtggga gtaaagtttt aaaattagat agggagggtc 2820
gtggtaggtc gttacgaggg tgagggaggc gtttgtattg agtaataaaa atatttagta 2880
atgggtatgt gaattgtttt aaataagatg ggagggcgtg agagatgatt taattatttt 2940
ttttttttga tgtttcgagg gggaggtatt ttaatttttt atttatttta taaaattatt 3000
tgttattgtg ttagttattt tattaggaat ttttgaaatt ttggtaattg atgatttttt 3060
tcgtaaatac gtttttaagt tttggaaggt tttaaagagt ttggtaataa gtaagaaacg 3120
tagtagtata taacgtcgag ggtaaaacgg ttttgtgttt tttttttggg gtgtaatagt 3180
tttaagcgga tttttcggtt tttgcgggcg tagtatattg ttttatttgt tttgaaggtt 3240
tagagtttga ggttggtcgt agatatttac gtgttttgat ttttattagc gtaatgatcg 3300
atttagttag agtcgtgttt atataaatta taaaaattta attattagaa attttagttt 3360
ttaaaaatta tattttaggt aaaaagtgtc gttttttttt cgcgtttttt tattttttcg 3420
attataattt tttgggggat ttaaagtcgt tttttaaatt tatattagga aataaatata 3480
taaataaata gttacggaag tattttgaat tttttagttc gtatttttaa ttttttttcg 3540
tgagcgttgg ggaagataga tacgaaattt agttttagat gtagaataga tttaataaaa 3600
tatttaaatt tatggaaaac ggttggtgtt tttttttagt aagggttttg taatttattt 3660
taattataaa gttgatagtt ttttagtttt taaaataaaa ataaataaat aaatatttta 3720
tttttgtttt ttaggaatga taagattgaa tatataagaa aaaagaaatg tttagttatg 3780
tatttgagtt tttaaaaatt attttgatta gatttttttt tatttttttt ttttattttt 3840
tttgtgttat ttaaaataaa aaatatttaa attttgtaaa attttttttt tttgggagtc 3900
gtttagatag aggtgggggt agaggtggga gggtgaagag tgttgagaag ttagtataga 3960
ggtttaagaa tttttttttt tttttttttg aagttataac gaaaaatagc gatagttaga 4020

ttagtcgggg gttttttggt gtttcggttt taattttttt ttataaagag ggggtttttt 4080
gttttagggg tggggagagt aagaggacgc gatttggcga gattgttttt agtttggggt 4140
cgtttttagg tttcggaaat tgtatacggc gcgcgggggg ttttttttcg cggtcggttg 4200
gcggcggtag cggttgcgat tcgtaggttc gagattcgtt gtttcgagat tcgttgttta 4260
cgatttattt tttggtattt tgaagcgcga gagatagggg agttttgtaa attgtaggag 4320
ggagtggatg taaagaggtg gttgtgagag ggcgagaaga aaagaaggga aaggaaggag 4380
tttagtcggg gagtcgggtt ggtagtcgtc gcgggaggaa tttgagcgtg tagttcgcgc 4440
ggggggagcg gcggtcgtcg cggaggcggc ggcgcgggag ggcggaggcg cggtcgtttg 4500
tagttgggta gtagcgcgcg gcgcggcgcg gcggtggcgg gtagcgcgcg gtaatgtcgg 4560
tttcggggtt ggattgttga atttatatcg ttttattatc ggattttaat tgggaggggg 4620
cgttagcgta tttaggacgc ggtgttttaa gggattttat tttaagttgt aaatttaagt 4680
ttttcgtttt tttaggttaa atatattttc gcgttcgttc gtagttgtta tttcgggtgt 4740
tttaaagatt tcggtaaaga tttgcgtttt tcgggttttt ttattcgtta gttcgattgc 4800
gtcgtatcgt tagattttgt tggtttatta gatttcgttc ggtagtcggt tttattaaat 4860
ttgatttttt atttttttag ttttattatt tttatttttt tttttttcgt ttcgttttta 4920
tttttttaat tttttttttg ttttttattt ttattttttt tttttttttt cggttatttc 4980
gttaatttgt ggttgttgtg atgcgtattt tcgtagattc gagtattagt cggcgtttta 5040
gttttttttt tagtagtttg tagttcggta aaatgagcga cgtgagttcg gtggtggttg 5100
cgtaatagta gtagtaatag tagtagtagt aatagtagta gtagtagtag taatagtagt 5160
agtagtagta ggaggcggcg gcggcggttg cggcggcggc ggcggttgcg gcggcggtag 5220
ttgtagtgtt tcggttgcgg tcgttttacg ataatcgtat tatggtggag attatcgtcg 5280
attattcggt cgaattcgtt cgtatcgata gttttaattt tttgtgttcg gtgttgtttt 5340
cgtattggcg ttgtaataag attttgttcg tggtttttaa ggtaagaggt tatatcgttt 5400
ttcgttttcg gtcgggagcg gcggaatttg ttcgtcggtg tttttttgtt tacggggttg 5460
ggtttcggac gttttttaag attttttgtt ttggcggttt taattttaga aattttcggt 5520
cgggtttttt ttcggatgtt ttggcgggtt cggggtttag ttttttcgcg taaatttttt 5580
ttttttttat ttgtgttcgt tgttatttcg attttagttt tagttttttt gattcgttta 5640
gtatttttta tgttttttgt ttggttttga tttttattta ttttttcgcg tcgttttttt 5700
tttttttagg atttttaaag tatttatttt tgtattgcga gcggatttgt tttatttcgt 5760
ttttttttcg gggcgagga tttcggtat tgtttttgg ggttttcgcg tttttttcggg 5820
atatttcggt ttggtcgtat tgcgggttgt tcgcgtagtt tttttttggg gtttcgcgcg 5880
tttttattcg ttttttttat agttaaacgt ttttttttag gtttcggcgt tttttttttc 5940
gattcgtaga gtttagcggt tttggttttt ttcgggtttt cgtgtcggga ttcgtggggt 6000
gtcggtgtcg ggtattagtg ttcggggttg ggcgtggggt gttgattgag gcgcgcgggt 6060
tgcgttatta gtgttcggga gggttttgga taaatttagg gtttttagag gagggggtgt 6120
tagggcgggg gcgtttgttt tgttcgttga ttcgcgttag atttcgttat cgggaggaag 6180
gcggggtcgt tggggcgttc gtaagtttcg aagcgggggc gtttaggggg tttgggcgtt 6240
gaattttcga gtgttgggag gcgcgggtta ggggacgggg cgggagttcg ggtcgttttt 6300
tttagtcggg atcgagtttt gcgtttcgtt tcgttaggga gggatgaggg aaatttgtgg 6360
aataatttta gttcgtttaa tttgaaaacg gaggtgaaat tttgtatttt tgttttatta 6420
ttatttttaa aggatttaaa tttaaaaaag attttttttg tttttgaaag gtggtgagag 6480
gtttttgttg gtggtttgag tacggagtga gttcgaaggt ttgagagcgg tttttgcgcg 6540
tttcgtagat ttttgtgttt gttcggttcg ttagtttgag ggcgggtggt agtcgcggga 6600
gtcgtaggta ggtgtttcgt aggttgaggt tttaggattt ggagtttttt atagtagttc 6660
gatggttttt tataattcga tttagatttt tcggggtagt tttttatagg ggggagacgc 6720
ggagttttta gggtttagcg tatattgggt tgatggggaa acgggttttt aggttgttta 6780
tattcgtagg attgtttata ttcgtaagac gttagggatg taggggtggt aagttagacg 6840
tggagatgag atttttagaa aggtgttttg ggtttttgtt aatttttgaa tttgagtagg 6900
tgggtttggg ttttttgggt tttgaggttt gtggagagta gggtaagtaa ggttttgggg 6960
gaggttaaaa aaaaaagttg ggggaggaat tatgttagtt ttttcgttgg ggattttggg 7020
gttaaatttt tacgggttat tgatagtttt tagttgttga ttatttttat tttacgtatt 7080
aatttagatt tcgtgtgtgg gtgggatgga aagaaagagg agtttaggtt aattagattt 7140
ggttatttcg ttaggattta tattagaata gaaattagaa tgaattgtta tggaaagggg 7200
atgttgtatt ttggggtaag ttttattttg tagttggttt aagaaatttt tttttttaat 7260
cgttatattt ttttttttat aaattattta ttttgtaggt taatgtttgt ttttttgaaa 7320
taggaaaagt ttagaatata tgtgaattgt ataaatttaa atttttatat aatatatatt 7380
ttttataatg tttatcgttt aagggtttag aatttttaaa ttagttttat atgagaatgg 7440
aaatttgttt ttattaacgt tttttaattt tttaaattaa aagtttttaa aaattttaaa 7500
tttggatgaa aatagtaaaa aatataaata agtagatatt ttgtttgtat aatgttataa 7560
attagaatat gtgaaaatat aagaatagat gtttatattt ttaaagtgtt ttatatatta 7620
ttttgttaaa tatatttaag gtttattttg aattgttttt ttgtatttat gcggttgaat 7680
tgatttgaat atgagtattt tggatggaaa ggttgttttt tgtattttag tttgagttag 7740
ttttagatat tttggttgtt atttaagatt ttttattatt attttttaaa aattattttg 7800
aaaataaatt ttgttttttg ttattaatta atttgttaat tatatagagt atatttttgt 7860
attttaagta gtaaatagat cgtaggtaat aagattatat taaattgatt attttaattt 7920
atattaattg aataatgaat tgagttaatt tataatttga ttgttaatgt ttttttatta 7980

atatatttgt gttaaataat ttaaaaagtg ggtgatagtt aagatttatt tagtatagaa 8040
aatattttgt tgagaaaaaa tgtatggatg tttggaatat ttagattgtg ataatttttt 8100
tttattttat ttttggaata aaagtttgag agtattagat tttttatatt tttataatat 8160
aattaaatat atttgtaatg aagaaataat atgaagaagt ttttttttta aaagtggtga 8220
tagtaatttg gttatgatat ttagagttat gtaaaaaata gattatagtg gttgttaggt 8280
taatagtaga taggtaaatg gatttaaata tatatatata tatatatata tataatttat 8340
ttgttgtgaa gtttgatatt taaattaata taatatttat tgagttcgta ttttgtattt 8400
gttatattga gtttgatatc gtagagaatt agaaatgaat ttaatagatt gttttgaaat 8460
gttagtttaa attcgtgttt aaatacgata ttgttaattt attttattaa gaatgtatta 8520
gcgttgttta tttatttttg tttcgagatg atataaaatt tatttatttt tatatgtaaa 8580
aatgtaaaaa taattggtat attttatata aagttgttta ttaatattgt atttttaata 8640
aagttttgta gttttgaaat gttcggtttt tttaggtgtt aaggaaacgg aaataaatat 8700
ttttaatatt tttaggtgta ttttattgaa attttaaaat agttagggta tattattaga 8760
ggattttaga ttttttttat tttagataat tttagtgata aagaagtaat ttgtaaattt 8820
agtttattag ttgatggtta ggtaacggtg gagagagcgt gttaattatg tatttagtag 8880
tattttttta gttaggggtt tgttagtatt tttattgtga agtttggtgt ttagggttcg 8940
taatttagtt gttttaaata gttttaagtt gaaatattat gtagaaattt taagtttaga 9000
tgtatatttt aaaattaaaa gtagtttaag tttattttgt tgttttaagt tagagaatat 9060
tggattatat atatttatta gtattatatg tttttttggt agtgttaaag aaagaaaata 9120
tatttttaga tatataagtt agtatttata gttaattttg tatatttaat aatatttttt 9180
agtgtttgtt aaaaggaggg gttttaaaat gatagagtaa tttttgtttg aaaataaatt 9240
tttggtatta ttagttattt tttttttttc gaaaataaat atataattgg taaatatttg 9300
atattagtga gtagtatttt ttttatatag ttaatgatta gattttatgt ttatattttt 9360
tgaattttat tgtatttaaa ttagggaaaa taggttgtta agtttgttaa atttttatta 9420
atcgtaatat aaattattat gtaaaattat gtaaattaat agattttaag ttgtgtgaag 9480
atagatatat ttttgtgtta tgtttttgaa tttaagaaaa tttatttaaa gttaaatatt 9540
ttgggattga atatataaaa tttttaagta aggaagttgg aataaagaat tttaggtgga 9600
aaataaatat ttataaaatt ttttagaaaa tttttattaa atatttgaaa ataggtatgg 9660
tagatatttt ttattatttt tttttttttg gtattattgt ttgtattttt ttaaaatttt 9720
agaatattta ttgttagatg atttaaatta ttatgatata tggtttataa attgttgttt 9780
tatattgaaa taatttttat taaaatttat ttttgatata tatattttta aaattatgaa 9840
atgatatatt gtgttttttt tttaaaattt aatgggtttt ttttaagttt taggataaat 9900
ttaaaatatt aattgtagta tttgttgaaa tgttttgata ttgtatttga agttattttt 9960
aagtaattgt gataggaata atatttttgt tttttgtaat tagttttatt ttttttttta 10020
ttgtaaagaa agatttgatt agattttatt ttgaaaaata attatatttt aattatatcg 10080
taagtataat attttgaatt attttggttt ataagagtaa ttgattagtt aagattagat 10140
ttttgtttat ggaaatgtta taggtaattt taatttttat ataaaaagat gagaaattgt 10200
tttaagggtt tgagttaatt ttttattttt ttttttttag tgatttatag atttagttat 10260
aaggagtttt atataaaatt ttaatagtat tattatagtt aaatattttt ttattttatt 10320
ttgtgtaagt tatatattat gtgatttaaa tgttagttat ggtgaggtgc gttaaataaa 10380
ggaacgtttt tttttatttt gaaatgttag gatagaaaat tatttttttt ttttttatgg 10440
agtttttga atttgtagat tttatatttt aaaaaaatat agaaatgtaa ttgttaagat 10500
tattataaat tattttttgt agagtagtat ttttgtattg ggtaggatgg aagtaaattt 10560
taatttgtgt ttatttttaa ttagttaaag agcgagtttt gattgtattt tattttttta 10620
aatgtttttt tattgtttgg ttttggtata gatgaaatgt gtaaaaattt tttgattttt 10680
ttggtttttt tttagataaa tatagttgag aatttttaag ggaaattttt tagttttaga 10740
gatttattta aagaaaaaat tttttaaata tataattata tttttgattg atataaagtt 10800
aaatatagta ttttttttaa tttttaattt tttttttttt ttagaaaaat atcgggtaat 10860
ttgatttggt agagtgaata gtagaaaaaa aattaatata tttagttatt aaattagagt 10920
ttagttttaa taggaaagat tgttattatt ttgtattagt attttttttt aggttttgtt 10980
tttagtttta tagaaggttt atataatttg tgattattta tttgttttgt aaaatgtgat 11040
acgtaaaata tattaatatt gaatatggtt attattatat tgtttaagta tagtaaaatg 11100
gatgtatgta ttttattttt agggattttt ttttttatat ttttttatat tgaattaatt 11160
ttttattttt tttaattaaa aaaaaattta ttatgttaaa aatatgtagt aaaataataa 11220
tgttttagag tttttgaaaa tttttatatt ggggtagatt gttttttttt tttttttttc 11280
ggaaatgtag ttagatttga gtgaaattta ttagtagtta ttttgtattt tttaataata 11340
ttaaagaata ttttagaata tgaagtgagg aagtacgtat ttagttgaat tttttaagaa 11400
tgtttataag taaaagttta tgtttaatag atagtagatt tggaatagaa tattgtgtta 11460
ttttttttat tttataaaaa gtatattatt ataattatat tttatattta tatagtattt 11520
tttatatttt gtagattttt taatttatat tggaaaattg tggtttttgg agatgaagtt 11580
attttgtgtg aggttatagt agatatgtag tagaagtggg gaaaaagtgg agttttttga 11640
ttttggtata tttatttttt tttaaatagt ataatttttt ttttgttttt attaatatgt 11700
ttattgggtt tatatttatt ttgatataga aagttacgta gtaggtgtta aaatataata 11760
tgttgagtgt ataaaatgtt aaatatttag atgaattttg gcgaatgtag ttaattattg 11820
tagtattttt aaatgtttat atttttggta tggtgaattt gaaaaattga aaagtatatt 11880
gttttattta ggagtatagt attatttgta tttagtagtc gtttgagata tgagatgacg 11940

tgtatagaga ggaagtttta tttttaaggg attttatttt tgatttagga gattttttat 12000
attgagaata attatatttt ttagggaaag ggaggtagta tggtttcgtt ttttttgaga 12060
tgaaaataat gttagaaaat aagttggggg taagggtata ttgttgttag taaggggtgt 12120
tgatttaata ttaattggtt tttttagagt tttagtgggt agttttatag atttttgttt 12180
ttgcgaagtg gggtttttta tggagttttt ttatttattt taaaaatgtt tatttggttg 12240
ggttcggtgg tttacgtttg taattttagt attttgggag gttgaggcgg gcggattatt 12300
tgaggttagg ggtttaagat tagtttggtt aatatggtga aatttttttt ttattaaaaa 12360
tataaaaatt agttaggtgt ggtggtgtat atttgtaatt ttagttattc gggaggttga 12420
ggtaggagag ttatttgaat ttgggaggtg gaggttgtag tgagtcgaga tcgtgttatt 12480
gtattttagt ttgggtgata gattaagatt ttattttaaa aaaaaaaaaa aattaataaa 12540
atatttatta tgtgttggat atttttggga gtagggttga taagtggtga ataagataga 12600
tgagatgaag gttttttttt cgtgtagttt tttaatttat ataatttgga tttttaggag 12660
tgagtttttt agttttggtt tggtatattt aaagtagtta tagattatta atattaatat 12720
atgtacgttg aaatgaggat tatttgagta tattgggagg ttggggaaag tggggtttgt 12780
aatttaagga aggttttatt gattttgaat tgaataatat agtattaaat ggaaggaatg 12840
gaattgagaa atagtttaag agaaaagtaa gggtgataaa aatattgata agttttttgt 12900
ttgattgttt ttttggttgg aggagaggat atagtgaagg cgtgttgtag tagggtttat 12960
ggatggggga aatttagttt taaaatatag gtatagtgtg tattattata ttgttaagaa 13020
atttattgta atatgttaat tgtattggga atatggaatt aaatgtttag gtttgtttta 13080
tggaaatttt gataaaggag ggagtagtat tagaaggaga aaaagataag tgggaggttt 13140
attagatagg taagtatata ggggtattat tgttagatag ttgtgggtta tttgagttgc 13200
ggttattgtt gagttattat gagggtattt ggaagttgaa tgaggaagat atggaaataa 13260
tttgaatata gagttttgga agttcgttga gagaagggat tgttattaag gggtaggatt 13320
ttggtaatgg attaggagga gtgttttata tatgatagtg aaggatttgt attagtattt 13380
ttttattggg ttttgtgtta attaaaggta tatatttgta tgttagttga tttaggttaa 13440
agtgttattt ttagttttag agtagagaaa atgtttgttt gttttaaaga tggattaatt 13500
tagatttttt tttgattgag ttaatttgtt ttagattaat tgttgaagat taagtagtta 13560
agtatgttat tttaggatgg tagatgggat ataagatata atagaattga ggttaagggt 13620
atatattgtt ttttttttat ttattattaa ttatttttat ttttagtttt ttgatgattt 13680
tggatattat attgggtttt atcgtagtgt ggtttttagt aaatatattt tattaaaagt 13740
tgaagtgtta gtttaaagta aggagataaa atagattata cgatatttag gttttatgta 13800
gtgaggagtt ttagggtttt atttaaaaat ttttttttat aaagttgatt agatttgata 13860
ttttttagtt tgtgatggga taagattata ttttagggtt ttttttgaga gaaagtttgt 13920
agataggtgg tgttttgatt tgtatgatgt aatttgtaga tggggtattt tttttttttt 13980
tgtggtataa tttttacgga tagtttaata tggggattta ataaaaatgt atatattttt 14040
tgtgaagaat ttaataataa atgaaagaag tttttgtttg tttttgaaat gggtggattt 14100
tgaaatattg gtttgtttga agtggtatta taatttatat atagatgttt tatttttgtc 14160
ggttattatt ggattggatt agaatattaa gttttgataa gatatattat ttgtaatgag 14220
aatatatttt gtttgtaagt ttttttgatg tgttattatt gttgttgtgt tttttgtttt 14280
atgtagg 14287

<210> 50
<211> 14287
<212> DNA
<213> Artificial Sequence

<220>
<223> chemically treated genomic DNA (Homo sapiens)

<400> 50

tttatataaa ataggaaata tagtagtaat aatggtatat tagaaaggtt tataaataga 60
atatattttt attgtaaatg atgtgtttta ttaggattta gtgttttagt ttagtttagt 120
aatggtcgat aggaatgaag tatttgtgta tgggttgtga tgttatttta aatagattaa 180
tgttttaggg tttatttatt ttaaaggtag gtaaaaattt tttttatttg ttgttggatt 240
ttttatagaa aatatgtata tttttattaa atttttatgt taagttgttc gtaggaatta 300
tgttatagaa ggaagaggga tgttttattt gtagattgta ttatatagat taaggtatta 360
tttgtttgta ggtttttttt tagggagagt tttgaggtgt ggttttattt tattataggt 420
taaaaaatat taagtttggt taattttatg ggagaagatt tttaagtgaa attttggggt 480
tttttattat atagagttta gatatcgtgt agtttgtttt atttttttat tttgggttag 540
tattttagtt tttaataaag tatatttatt gaaaattata ttgcggtggg atttagtgtg 600
atatttaaag ttattaagaa attgaaggtg aaaataattg atgataaata agaaaaaaat 660
aatatatatt tttagtttta gttttattat gttttgtgtt ttatttgtta ttttggaatg 720
atatatttga ttgtttaatt tttaatagtt aatttgaagt aaattaattt aattaagaaa 780
agatttagat taatttattt ttggaataaa taaatatttt ttttgtttta agattagaga 840
taatatttta atttgagtta gttaatatgt aaatgtgtgt ttttgattaa tatagagttt 900

agtaaggaga tgttggtgta gattttttat tgttatatat gaaatatttt ttttaattta 960
ttgttaagat tttgtttttt ggtaataatt ttttttttta acgaattttt aggattttat 1020
atttagatta tttttatgtt ttttttattt agtttttaga tgtttttatg gtaatttagt 1080
aatgatcgta gtttaagtag tttatagttg tttgataata atatttttgt gtgtttgttt 1140
gtttggtgaa ttttttattt attttttttt ttttttagtg ttattttttt ttttgttaag 1200
atttttataa gataaattta agtatttaat tttatgtttt taatataatt ggtatattat 1260
agtagatttt ttaatagtgt gataatatat attatattta tattttaggg ttgaattttt 1320
tttatttatg aattttattg taatacgttt ttattatgtt ttttttttta gttaggaaaa 1380
taattaaata aaaaatttgt taatattttt gttattttta tttttttttt aggttgtttt 1440
ttagttttat tttttttatt tagtattgtg ttatttagtt taaaattaat aaagtttttt 1500
ttaaattata ggtttatttt tttttagttt tttaatgtat ttaagtgatt tttattttaa 1560
cgtatatatg ttaatattaa tggtttatga ttattttaaa tatattaggt taaagttgag 1620
ggatttattt ttaggagttt agattatatg agttaggaaa ttatacgaga gaaggttttt 1680
tattttattt gttttgttta ttatttatta attttgtttt taaaagtgtt tagtatatgg 1740
taagtgtttt attaattttt tttttttttt gagatggagt tttagtttat tatttaggtt 1800
agagtgtagt ggtacgattt cggtttattg taatttttat tttttaggtt taagtgattt 1860
ttttgtttta gtttttcgag tagttgggat tataggtgtg tattattata tttggttaat 1920
ttttgtattt ttagtagaga aggggtttta ttatgttggt taggttggtt ttgaattttt 1980
gattttaggt gattcgttcg ttttagtttt ttaaagtgtt gggattatag gcgtgagtta 2040
tcgagtttag ttaggtaaat atttttgaaa taaatgaaag agttttataa agagttttat 2100
ttcgtagaga tagaagtttg tggggttatt tattaaaatt ttgagagggt tagttaatgt 2160
tgagttagta ttttttgtta atagtaatgt gtttttgttt ttagtttgtt ttttgatatt 2220
gtttttattt tagaggagac ggaattatgt tgtttttttt tttttggaga atatagttat 2280
ttttaatatg agaagttttt tggattaaaa gtaaaatttt ttgaaaataa gatttttttt 2340
ttgtatacgt tattttatat tttaagcggt tattgaatat aggtaatgtt gtatttttga 2400
tggaggtaat gtgtttttta attttttaaa tttattatgt taaaaatgtg aatatttgaa 2460
aatattgtaa taattgatta tattcgttag gatttatttg aatgtttgat attttgtata 2520
tttagtatat tgtattttaa tatttgttgc gtggtttttt atgttaaagt aaatatggat 2580
ttagtaagta tattaataaa ggtaggagaa aagttgtatt gtttagaaga gaatgaatat 2640
attaaaatta agaggtttta tttttttttt atttttgtta tatatttgtt atgattttat 2700
ataaaataat tttattttta ggaattatag ttttttaata tgaattggaa agtttgtaaa 2760
atgtagaaga tattatataa gtatagagtg taattataat gatgtgtttt ttgtaagatg 2820
aggagggtaa tatagtgttt tgttttaaat ttgttgtttg ttaaatatag gtttttattt 2880
ataagtattt ttaaagggtt taattgagta cgtatttttt tattttatgt tttgagatgt 2940
tttttgatgt tgttgagaga tataggatga ttgttggtgg attttattta agtttggtta 3000
tattttcgaa aaaaaaaaaa aaaaagtaat ttgttttaat ataaaggttt ttaagaattt 3060
tgggatatta ttattttatt gtatattttt aatataatga attttttttt agttagaaga 3120
aatggaaaat tgatttaata tgggaaaata tgaaagaaaa gatttttaag agtgaaatat 3180
atatatttat tttattgtgt ttaagtagta taataatgat tatatttaat attagtgtgt 3240
tttacgtatt atattttata aaataaataa ataattataa gttgtgtgag ttttttatga 3300
ggttgagagt aagatttgaa aaaaaatgtt aatgtaaggt agtgataatt ttttttgtta 3360
aagttgaatt ttagtttaat ggttaaatat attggttttt tttttgttat ttattttatt 3420
aaattagatt gttcgatgtt tttttgaggg ggagaaaagg ttgggagttg ggggaagtat 3480
tgtatttgat tttatattaa ttagaagtat aattatatgt ttgagaggtt ttttttttaa 3540
gtagattttt gaagttaaaa agtttttttt ggagattttt aattgtgttt atttaaaaga 3600
aaattaaaag aattaagaaa tttttatata ttttatttgt attaaaatta agtaatggaa 3660
aaatatttga aggggtaaag tgtagttaaa attcgttttt taattagtta ggaataagta 3720
taaattagaa tttgttttta ttttatttaa tataaaagta ttgttttgta aagaataatt 3780
tgtgatgatt ttaataatta tatttttatg tttttttaaa atataaaatt tataaattta 3840
agaaatttta tgagaaaagg agaagtaatt ttttgttttg atattttaga atagagaaag 3900
acgttttttt atttagcgta ttttattata attagtattt aaattatatg atatgtggtt 3960
tgtataaaat aaagtagaag gatatttaat tgtagtaatg ttattgaaat tttgtatgag 4020
attttttatg attaaatttg tgaattatta aaagaagagg aatggagaat taatttaaat 4080
ttttaaaata gtttttattt tttttatatg gaaattaaaa ttgtttatga tatttttatg 4140
gatagaagtt tagttttaat taattaatta tttttatagg ttaaaataat ttaggatatt 4200
gtatttgcgg tataattgaa atataattat tttttaagat aaaatttgat taaatttttt 4260
tttgtaataa aaagagaagt gagattaatt gtaaaaagta aaaatgttat ttttattata 4320
attatttaaa gataatttta aatatagtat taaaatattt taataaatat tatagttaat 4380
attttgaatt tattttagaa tttaggaaaa gtttattgaa ttttagagaa aaaatataat 4440
atgttatttt ataattttaa aaatatatat gttaaagata aattttgata aaaattattt 4500
taatgtaaag tagtaatttg tgggttatgt attatggtgg tttagattat ttaataatag 4560
atattttgga attttgaaag gatataaata ataatgttaa agaggaaaaa ataatgaaag 4620
atatttatta tatttatttt taaatattta atagaagttt ttttaaaaat tttataagta 4680
tttatttttt atttgaaatt ttttgtttta atttttttat ttagaagttt tatatattta 4740
gttttaaaat atttaatttt aaataagttt ttttgaattt aaaggtataa tataaaaatg 4800
tgtttgtttt tatataattt gaaatttatt gatttatata attttgtata ataatttata 4860

ttacggttaa taaggattta ataggtttaa taatttattt tttttagttt agatataatg 4920
agatttaaaa aatataaata taaaatttga ttattaatta tgtaagaaaa atattattta 4980
ttggtattaa gtatttatta gttgtatatt tgttttcgaa gagagaaaaa tggttagtga 5040
tgttaaaaat ttatttttaa atagaaattg ttttgttatt ttgaaatttt tttttttgat 5100
aagtattgaa aggtattatt aaatatatag aattaattat gagtgttagt ttatatattt 5160
aaaaatgtgt tttttttttt taatattgtt agaaaaatat atgatattga taaatatatg 5220
tagtttagtg ttttttaatt taaaatagta gaatagattt gaattatttt tagttttaaa 5280
atatatattt ggatttaaaa tttttgtata gtgttttagt ttgaagttat ttaaggtaat 5340
taagttacga attttgaata ttaggtttta tagtggaaat gttgatagat ttttaattaa 5400
aagggtgtta ttggatgtat ggttgatacg ttttttttat cgttatttga ttattagtta 5460
atgagttgaa tttgtaaatt atttttttgt tattagaatt atttaggata gaaagggttt 5520
gggatttttt aatagtatgt tttaattatt ttaaggtttt agtgagatat atttgaaaat 5580
gttaaaaata tttattttcg tttttttgat atttgggggg atcgaatatt ttaaaattgt 5640
agaattttgt taaaaatata atgttaataa ataattttat ataaagtata ttagttgttt 5700
ttgtattttt atatgtaaaa ataaataaat tttatattat ttcgaaataa gagtagatga 5760
ataacgttaa tatattttta atgaaataga ttgataatat cgtgtttaaa tacgagttta 5820
aattgatatt ttaaaatagt ttattgaatt tatttttgat tttttgcggt gttaagttta 5880
gtgtgataga tataaaatac gggtttaata aatattgtat tgatttgaat attagatttt 5940
ataataaata agttgtgtgt gtgtgtgtgt atgtgtgtgt ttaaatttat ttatttattt 6000
gttattgatt tgataattat tataatttat tttttatata gttttaaata ttatggttaa 6060
attgttgtta ttatttttaa aaggagattt tttttatatt atttttttat tgtagatgta 6120
tttgattata ttgtaagagt atagaaagtt tagtgttttt aggtttttat tttaaaagta 6180
gggtaaagag aggttgttat aatttggatg ttttaaatat ttatatattt ttttttaata 6240
gagtattttt tatattgagt agattttagt tattatttat tttttgaatt gtttaatata 6300
ggtatgttga tagaaagata ttgatagtta ggttgtaggt taatttaatt tattatttaa 6360
ttgatatgaa ttaaaatagt taatttaata tggttttgtt atttacggtt tgtttattgt 6420
ttaagatata aaaatgtatt ttgtgtgatt aataagttga ttaataataa agagtaaaat 6480
ttatttttaa aataattttt aaagggtggt agtgaaaaat tttagatgat agttaaaata 6540
tttgagatta gtttaagtta aagtgtaaaa aataattttt ttatttaaaa tgtttatgtt 6600
taaattaatt tagtcgtata aatgtaagag aatagtttaa gatagatttt aaatatgttt 6660
agtagaatgg tatatggaat atttaaaaaa tgtaagtatt tgtttttata tttttatata 6720
ttttgatttg tggtattgtg tagataaggt atttatttat ttgtattttt tgttgttttt 6780
atttaggttt ggggtttttg ggggtttttg gtttggggga ttggagggcg ttggtggaaa 6840
taaattttta tttttatata agattaattt aaaaattttg ggtttttgga cgatggatat 6900
tgtaaaagat atatattgta tggaaattta aatttatata atttatatgt attttgagtt 6960
tttttatttt taaggaagta aatattaatt tataaaatgg gtggtttata aaaggagaaa 7020
tgtgacggtt aaaaaaaaaa attttttgag ttaattgtaa aatgaaattt gttttagaat 7080
gtaatatttt ttttttataa taatttattt tgatttttgt tttgatataa gttttagcga 7140
gataattagg tttaattgat ttggattttt tttttttttt atttatttta tatacggaat 7200
ttgaattggt gcgtggggtg gggtgattaa ataattggga attgttagtg gttcgtggga 7260
gtttggtttt agggttttta gcggagaaat tggtatgatt ttttttttaa tttttttttt 7320
taattttttt taaggttttg tttgttttgt tttttataga ttttagaatt taggggattt 7380
aggtttattt atttaagttt agaggttagt aagggtttaa agtatttttt tgagagtttt 7440
atttttacgt ttggtttatt atttttgtat ttttggcgtt ttgcgagtgt ggatagtttt 7500
gcgagtgtgg atagtttggg aattcgtttt tttattaatt tagtgtgcgt tgggttttgg 7560
gggtttcgcg tttttttttt gtagagggtt atttcgggaa atttggatcg ggttatggag 7620
agttatcggg ttattgtgag aggttttaga ttttaaaatt ttaatttgcg gagtatttgt 7680
ttgcgatttt cgcggttgtt attcgttttt aaattaacgg gtcgggtagg tataagagtt 7740
tgcggggcgc gtagaggtcg tttttaagtt ttcgagttta tttcgtgttt aggttattag 7800
taggagtttt ttattatttt ttagaagtag gagggatttt ttttagattt aagtttttta 7860
aaaataataa taaagtaaaa atataaaatt ttattttcgt ttttaagttg ggcgaattaa 7920
agttgtttta taggtttttt ttattttttt ttggcggggc gaggcgtaga gttcggtttc 7980
ggttggaaga agcggttcga gttttcgttt cgttttttgg ttcgcgtttt ttagtattcg 8040
agggtttagc gtttaggttt tttgaacgtt ttcgtttcgg ggtttgcgga cgttttagcg 8100
gtttcgtttt tttttcggta acgggatttg gcgcgggtta gcgaatagag taaacgtttt 8160
cgttttgata tttttttttt tggagatttt aggtttgttt aggatttttt cgggtattgg 8220
tagcgtagtt cgcgcgtttt agttagtatt ttacgtttag tttcgggtat taatgttcgg 8280
tatcggtatt ttacgaattt cggtacggga gttcgggagg gattaggatc gttgaatttt 8340
gcgggtcggg gaaggggacg tcgagatttg aggagaggcg tttggttgtg agggaagcgg 8400
gtgagagcgc gcggagtttt aaagagaggt tgcgcgggta gttcgtaatg cggttaggtc 8460
gggatgtttc ggaaggacgc gggagtttta ggggataatg tcggggattt tcgttttcgg 8520
gaaaggggcg gggtggagta agttcgttcg tagtgtaaga gtgggtattt tggggatttt 8580
gggggaaagg gagacggcgc gaaggagtga gtgagaatta aggttaggta ggaggtatga 8640
ggagtgttga acgggttagg gggattaggg ttgaagtcgg ggtggtagcg ggtataggtg 8700
ggagagggag ggatttgcgc ggagggattg agtttcgagt tcgttagggt attcggaggg 8760
aggttcggtc gggggttttt ggggttagag tcgttaaggt aggaggtttt ggaggacgtt 8820

cggggtttag tttcgtgggt aggaagatat cggcgggtag gtttcgtcgt tttcggtcgg 8880
gggcgggggg cggtgtagtt ttttattttg aaggttacgg gtagggtttt gttgtagcgt 8940
tagtgcgagg gtagtatcga gtataggaag ttggggttgt cggtgcggac gagttcggtc 9000
gggtggtcgg cgatgatttt tattatggtg cggttgtcgt ggggcggtcg taatcggggt 9060
attgtagttg tcgtcgtcgt agtcgtcgtc gtcgtcgtag tcgtcgtcgt cgttttttgt 9120
tgttgttgtt gttgttgttg ttgttgttgt tgttgttgtt gttgttgttg ttgttgttgt 9180
tgttgcgtag ttattatcgg gtttacgtcg tttattttgt cgggttgtag gttgttggag 9240
ggggggttga agcgtcggtt ggtgttcgga tttacgggaa tacgtattat aatagttata 9300
agttagcgaa gtggtcgggg gagggggagg agggtggaaa tgaggggtaa ggaggaaatt 9360
gggggggtga aggcgagacg gggggaaaga ggatggaggt gatggggttg aggaggtgag 9420
aaattaagtt tgatgaggtc gattgtcggg cggagtttga tgggttaata gagtttggcg 9480
atgcggcgta gtcgggttgg cgggtaggga gattcgggag gcgtagattt ttgtcggagt 9540
ttttggaata ttcggggtgg tagttgcgaa cggcgcgggg ggtgtgtttg gtttggggag 9600
gcgggggatt tgagtttgta gtttggaatg gggttttttg gggtatcgcg ttttgggtgc 9660
gttggcgttt ttttttagtt ggagttcggt ggtgaagcgg tgtgggttta gtagtttagt 9720
ttcggggtcg gtattgtcgc gcgttgttcg ttatcgtcgc gtcgcgtcgc gcgttgttat 9780
ttagttgtag gcggtcgcgt tttcgttttt tcgcgtcgtc gttttcgcgg cggtcgtcgt 9840
ttttttcgcg cgggttgtac gtttagattt ttttcgcggc ggttgttagt tcggtttttc 9900
ggttaggttt tttttttttt tttttttttt tttcgttttt ttatagttat ttttttgtat 9960
ttattttttt ttgtaatttg taaagttttt ttgtttttcg cgttttaagg tgttaagagg 10020
taagtcgtgg gtagcggatt tcggggtaac gaatttcgag tttgcgaatc gtagtcgttg 10080
tcgtcgttag tcggtcgcga gaaggagttt ttcgcgcgtc gtgtgtagtt ttcgaagttt 10140
agaggcggtt ttagattagg ggtaatttcg ttaggtcgcg tttttttgtt ttttttattt 10200
ttaggataag gaattttttt tttgtaggaa gagattgaag tcgagatatt agagagtttt 10260
cggttgattt gattgtcgtt atttttcgtt gtaattttag gagaggagga aagaaaattt 10320
ttgagttttt gtgttgattt tttagtattt tttatttttt tatttttatt tttatttttg 10380
tttgagcgat ttttaaggga aaaagatttt gtagggtttg ggtgtttttt gttttaagtg 10440
atataaaaaa aatgaggagg gggggtggag ggagatttag ttaaaatagt ttttaggaat 10500
ttaagtgtat agttggatat tttttttttt ttatatattt aattttgtta tttttagaga 10560
ataagaatga ggtgtttgtt tgtttgtttt tgttttaagg attgaaaaat tgttagtttt 10620
gtgattaaaa taagttgtaa aatttttgtt ggggaaaagt attagtcgtt ttttatgaat 10680
ttaaatattt tattaagttt atttatattt taagattaag tttcgtgttt gtttttttta 10740
gcgtttacgg ggaaaaatta aaaatacgaa ttaaaaaatt taaaatgttt tcgtggttgt 10800
ttatttatgt atttattttt tagtgtgggt ttaaaaagcg attttggatt ttttaaaaag 10860
ttgtggtcga gaggatgaag gagcgcggaa aaaaagcggt attttttatt taaaatgtgg 10920
tttttggagg ttgggatttt taatgattag gtttttgtgg tttatataga tacgattttg 10980
gttaagtcga ttattacgtt gatgagagtt agagtacgtg ggtgtttacg attagtttta 11040
aattttgagt ttttaaaata aataaaataa tgtgttgcgt tcgtagaagt cgggaaattc 11100
gtttgaggtt attatatttt aagaaggggg tatagggtcg ttttattttc ggcgttgtgt 11160
attattacgt tttttgttta ttattaaatt ttttaaagtt ttttaaagtt tgaaaacgtg 11220
tttgcgaaaa aaattattag ttgttaaagt tttaaagatt tttagtggag tagttgatat 11280
agtaataagt gattttataa aataaatgaa gaattagaat gttttttttt cgaagtatta 11340
aggaagaaag atggttgggt tattttttac gtttttttat tttatttaaa gtaatttata 11400
tatttattat tgggtgtttt tattgtttag tgtaaacgtt ttttttattt tcgtagcgat 11460
ttgttacgat tttttttgtt tggttttaga gttttgtttt tattcgttga gtttacgttc 11520
gggtttgcgt tttagaaggg gtttggaagg gttttgagag tatgaggggt acggaagatg 11580
ggggtttggt gttagtcgcg gattttggag ttttttcgag tataagatga gtttgggggg 11640
ttgtggttgc gtgattcggg atcgtttcgt tgaagagatg cgcgggggat tgagggtttg 11700
ttagatcgcg ggagtagagg tgggggtgcg gggtagttag gtgtgcgttt tcgaggggta 11760
aggatggagt ttaggggata ttattttgtt ttttttaatt ggggttttat ttttatttgt 11820
ttttttttta tgtattgttt tacgtttttt tcgtcggttt tttttttttt tacgtttttt 11880
tttacgtttt tgtttttttt ttaaattttt ttatagatat tagttttaag tcgcgttttt 11940
ttcgttttat agtcgtcgtt tgttattgtt attttcggtg atttcgagaa gcgattttgc 12000
gttatcggtt atgatatttg gtttttaatt tatttttcgc gttcggttta ttggtttttt 12060
agttcgggcg tcgttagggt gcgagaattt atttagttag atcggattaa ttggaagcgt 12120
agttaggagt tgagttggtt tttgcgcgtg cgatttttaa ttattttttc ggttataggg 12180
gagagtttta atttcgggta tcgtcgcgtt ttttgcgtgt ataggttttt cgagttttat 12240
aggcggtcga ttcgggaagg gaagttaggt tttcgtttta aaaaagagag agagagattg 12300
tatttattgt tgttttattt tatacgttta tttatgtgta ttcggatata tatttttgta 12360
agtttatacg tagatatata taaatatata tatatttata gagttatggt tttttttttt 12420
tgtttttttt tttttttttt agggggttag ttttattttg ggtatgttgt gttattgttt 12480
aatagagatt tttggttcgt tttttgtttt taaatttgtg ggggttgtaa agggggggaag 12540
tagttatggg aattgattat ttttatggta aaagtgatat taaaatttta taattattat 12600
ggtgggtgat atgttatttt ttttgggatt gttttttttt taggaagttt atagaataga 12660
gaatttttgg aattttgaag ataataagaa ataattaata ttttaaatat ggattattag 12720
aggggttttt taaataaaaa attttagaat tgaatttaag aagttatttt ggggattttt 12780

tttttttttt tttttatcgt tgttttgttt tttgttttat ttggtggata gtaaattatg 12840
tagtttttag tggttgggga ttttaagggt aatttaatta atagtgagat tatttatttt 12900
aattaattat atttagtaat ttagttttat ggtttttttt agtatttttt ttttatttta 12960
tattttagtg gaattaatat aagtagtttt tggaagttat tagttttgga ttttttaaaa 13020
tattttatag ttatttataa tgtggaaatg aaatttttta ttgaatattt taataacgtt 13080
ttaggtattt ggtatagtgg gtttttagtt tttttttaaa aatatttttt tgttgaatat 13140
ttatattttc gattggaatt gattgagttt ggagttgatg atgttttggt tttgagtatt 13200
atgtttaaag aaagaaaata tttttataga tttttaaatt aaattttttt tgttaatcgt 13260
aggtatatag gaaaattatg aggttttata tatgtgtatt ttgtgttata gtttgtttga 13320
tgttattttt gggtttagat tttttacgtt aaaatgtttg aaataaatta tttattttat 13380
ttttttttag aaatatttat ttttttagtt gtatgtgatg gtattaaaaa gtatatattt 13440
tttttttgaa aatgtggtaa ttttggaaga tataaatata attttattta ttatttttta 13500
ttatgttttt taatatttaa tataattgtt tagtaataat atttgataat tttgattttt 13560
tagataaaga aaatgagatg tgaagtagtt aggttattta tttagttaaa gaattgattg 13620
ggaatagaat ttataggttt tttattttgg tttttttttt taatttttat atattatttt 13680
atatatattt atgatgaaaa gtagaataag tttttgtaaa attttaaaat attttttaat 13740
ggattagtta ttaaagtggg aaaattaatg gaaaattatg ggtatattga tgatttttaa 13800
agttttgaat gttttatatg ggaaaatttt ataaatagaa cgatttttgg agaattttcg 13860
tttggatttt gattgataga aggttattta gtttttgaag aagtagtatt gttattagat 13920
taggagtcgt tatattatgg tttatgggat aaatttggtt taatgtttgt ttttgtataa 13980
tttttaaaga tgttttttgt atttttaaat agttgaaaaa ttaaaagaat aatatttata 14040
gtatatgaaa atgatatgta atgtaaattt tagtgtttat aaataaagtt ttattggaat 14100
atatttatgg ttagttattt gtgtattatt tatagttgtt tttatttaaa gtagaagaat 14160
tgggtagttg tgatattgta cgagttataa ggtttaaaat atttattgat tttttataga 14220
aaagtttgtt aatttttgtt ttaaagtgaa agggatgtaa gattatggag gagtgagata 14280
gtagaaa 14287

<210> 51
<211> 7148
<212> DNA
<213> Artificial Sequence

<220>
<223> chemically treated genomic DNA (Homo sapiens)

<400> 51

ggttgtttag taggatttat taaatttagt tttttttttt tattataata gttttttttt 60
ttgtatttag atgttagttt tttttattat ttagttatta ggaggagagt gttgatatgt 120
ttggttaggt gatgcgttta tgttttatat tttgaattag agtagatttt taattcgggt 180
ttggtttttt ttagtttggt tgatttcgga taacgttttt ttttgtttat atgggtataa 240
tatttattat atatgacgtc gtggggattg aatattatga gtaatgttta tgtgcggcgt 300
ttggtataag gtaaagtttt agtattttta gttgttattg ttttttttat tttattttcg 360
gggattaagg tttaggggag cgtggatatt aggtaaaagt tttagatcgt ataggtagta 420
tttgggtttt aatattttta ttattttgtt tttttttgtt tttttttttt tttttttaag 480
tttggaattt tttaaaaagg aagaaagatt tttgtaattt gagaagtata tttgtgtttt 540
tatatatttt gaattttttt agagaggttg tttaggatag atgattttag agttggattt 600
tttggagttg gattttcggt ttataggaga taatttttta atttagatat attgttattt 660
tttattaata tttttcgttt atatatttag agattattat ttgaaattag gatgtagatg 720
agttttagtt tatttagtag tatttttttt ttgttttttt aaataatcga ttattaatta 780
taatgatgtt tttttttttt tagagggaat ttttaatttt tttttcgtga aatggagtgg 840
ttagaatgat ttttattgat agaagaggaa attgaggttt agtgaggggt atgaatgttt 900
tttttgagtt ttagggtatt gggtaggaga taggttttat tacgggtttt tttggatagg 960
ggatttaggg ttaggttggg agaaattttt agttttagaa gagagttttt tgtttttttc 1020
gttggattag gtttttgtgt gattcgggat atggggaata gaggtttttt gggaaggaaa 1080
cggggttttt ggtagtgttc gttgaggttt agttgttatt taggaaaggg tttgtttata 1140
ggagggcggt cggttggggg cgaaagtgag gttttgagag gaatggttag ataggtaatt 1200
atcgagattt agttttggtc gttttgtgat ggttgttgga aatttcgtta tgagttgatt 1260
ttacgtggag gtaggttata gttagttttt atttttttta ggtagtttgt gggcgaattg 1320
agggattagg atagtatgga ttggtttgga gttttagtaa gttttgaata tgagaattgg 1380
atattttata gatggaagtg tgaggtttag agagaggagg ggtgggttaa aggtttttag 1440
taaggattga gaggttttat ttttttatga tagtgatggt tttcggaggt tggagtattt 1500
tttatgtgtt aggtttgtgt taagtatttt atatataatt ttatgaaggg ttattatgag 1560
tgttttattt tataaatgaa agaaatgagg tttagagagg tttaggtaat tttttttaga 1620
gcgttgtggg tgagggaaaa agatacgttt aggtagaggg attgtggatt ttagtttttt 1680
ttatagttta tggatttttt aggcggtttt ttagggtata tttgtttttt gtttggtata 1740

tttggggatt tttgtttagg cgttatagag tttatatgaa ttatatttgt tttgggaagg 1800
cggtattttt attgttaaga acgtatttag agagtatttt taaatgttag agggagacga 1860
attttttttt tggggtaaag gagttggttt tatttgttcg gggatgggtt ttgggtttat 1920
ttagttttaa gttaaggtag ggtaggggag tgttttgatt ttttgttttt tagttttgtt 1980
gtggataatt tagggtgttt ttaggtgggg gttattgggg agaaattggt ttgtttgttt 2040
atcgatttga tggaagaggg agaaaagacg acggtttatg ttaattgggg aagggcgagg 2100
gtgtttgtat gttttaggtg ggggagtcgg agtttttttt tttattaaat agacgataat 2160
ttttgtcggt tcgggatggg gaggaagtag gtggaaattg ggaataagtt agtttgtttt 2220
gtgggttttt ttcgggtgtt tgaaaacgcg aagatagaag gcgtttattt tattagaggt 2280
gaggaaggcg gttttggttt gatataaagt tatcggtttg tttgagattt ggtcgtatgg 2340
atggtaagga aagggtagtt tttcgggggc ggtcgtcgcg tttgtattcg gggtcgtagt 2400
tggcgcgtat ttgtagttcg gtcgggttcg tattttcgcg gttggtaggg cgcgggcgtt 2460
aattagcggc ggttttcgtt attgtgtttg ttaggcggtt tcgcgatttt ggtttgggga 2520
tttaagggtt tgtattcgtt tttttttttt tcgtcgtttt ggttttttgg atttggtgtt 2580
tacggggagt tagcgtttta cggagagtcg gagtcgtgtc gggttttgcg ttaggtgtta 2640
gggtttataa gagtttttta ttttttggaa atttgtttgt gaatttatcg taagcggaga 2700
gggagaaatt aggcggagga ataagtaaag ggtacggtgt aaatcgaaat tattcgataa 2760
atggaattta ttattatttg aaatagcggg ttatggtttc gtaatttgtt cgagggtagt 2820
acgtgttgtt cgttttcggt tagggtttta tttggttacg acgcgttgcg ttttttcgga 2880
gacgttttcg gaggtgggag cgtttaggtt ggattattcg ttttttttta gttttgttgt 2940
tcgtgttagc gcgttcgagg gcgcgcgggt ttgggttcgt aatcgataat tgttaggcgt 3000
aggttttttt aaaaggttta gtaagggatt tttgtcgttt tttttttcga tacggtttga 3060
gggcgtgttg tgaggtttcg aggtgggtag gggttagttt tttcggtggt cggggtgagt 3120
ttagagtttt tcgtttttgg agcgtacgcg gggtttgatt tcgtttggta acgacgaaat 3180
ggcgcgcgtt gagtaggggt tagatttatg atgagatttt gcggttatcg tcggatttta 3240
agtttttttg ttttcgaggt ttggaattta ttgttttgta tttgtcgaga gtcggaggta 3300
acgaaaattt agtttcgttt ttaaagcggc ggggaggttt agtacgcgtt cgttttttag 3360
agtttaggga ggtgtttggg gcgataattc ggaatgattg tggtttgatt tttttcgttg 3420
tttttttaat tgtagtcggt ttttaggttt gttcgataga tttaggaggc gggagaggaa 3480
ggggtgattt gtagtgaagt ttaggagagg ttgggttacg cggttgggag tgggagcggg 3540
gattcggagt cgggcgggta ggtagtgttt tggcgaagtt gttcgcggtt tttgcggcgt 3600
agtcggagcg tacgggttta agaagaagtg gggttggatt cgtagaggtt attttttatt 3660
cgtatggaga aagaaaattt ttttttttga aagcgagggt ttttagtttt gtagttattg 3720
ttgttttttt tttgttatcg acgcgcgtat cgttttacga tgtaggatcg tggttatatg 3780
cgtaaaggaa aaaaagaaaa acgtattttg taggtttcgt cgtgtttttt aaagagttat 3840
aggtcgttat aacgaagaac gacgtcgcga ggtttgtaag attttgaaat ttgttttgag 3900
gggagagtag agaggaaagg ggttgttggt tttaggttat ttagggtttt taggagattt 3960
tttttcgttt gttttcggtt tggtataggg gttatcgagg ttgggattaa agtcgcgtag 4020
ggttgggagt agtaaaggtc gtcggtcggg cgtggacgac gcgtaaaatt tcgtgtgggg 4080
tggaggtttt tgggttagaa taatgtgcgg gacgagggag gtgagtaatt tttttggggc 4140
ggtttttagt gcggcgttat cggttttgag atttcgcggt ttttagttcg gggttgtaga 4200
agttataggt tcgaagtagt aagagttggg gaagttcggt cgcggttagc ggggaggagg 4260
agcgaagggg ttgcgtttta gcgttaggga gttacgattc gagagagggc ggtaagggcg 4320
tttttcgtgg gattcggacg ttttaagtaa atttttagta tttgtttcgg gtttttagag 4380
ttttcggggg ttttgggttg tggtattggg gtttttttcg cggggtggcg tttttcgttt 4440
tttttcgttg ggcggttttc ggtaggtttc gttttttttc gcgaacgtta tcgaggtgtt 4500
cgcgatgggg gtttcgtcga ttggttgtgc gacgcgtcgt ttcgttagtt tcgtttcgcg 4560
ggtttcgggg gtattaattt cgcgcgggcg gtcgcggttt cgttatttga ttttggagga 4620
tttgttttgg ggttgcggtc gcggagtcgg ggcggtcgcg ggcgagtttc ggggcgggag 4680
gcggcggtag cggtatagtt tcgcgcgggt ttcgtcgcgg tttaggtagt cgggatagtt 4740
acgaggggcg gtcgtacgcg gggtcgcgcg tcgaggatgc gggattagtc gggtaggttg 4800
cgggcggtcg tcgggttagc gaggtttcgt agcggcgggg ttttggcgag tagtggtcgg 4860
gcgtcgtttt ttgcgttttg aggttcgggt ttcgtcgttt ttgttttttc gtttttcgcg 4920
gtagcggcgg tcgaggaggc gttcgcgtcg gtcgttttcg ggggaagtcg cgtcgttttc 4980
gtttgttcgg cgttttgacg gtcgttgtta tgcgtatttt cgtagattta agtattagtc 5040
gtcgttttat attttttttt tcggtttttt tttgcggcgg cggcggcggt aagatgggcg 5100
agaatagcgg cgcgttgagc gcgtaggcgg tcgtggggtt cggagggcgc gttcggttcg 5160
aggtgcgttc gatggtggac gtgttggcgg attacgtagg cgagttcgtg cgtatcgata 5220
gttttaattt tttttgtttc gtgttgtttt cgtattggcg ttgtaataag acgttgttcg 5280
tcgtttttaa ggtgagtgcg ggattcgggg cgggagggcg tcggttttgg ggtttcgggc 5340
gtttgagttg cgggagttag agtttcggga gtagtgggga tgggaggtgt tcgagacgtc 5400
gcggcgatat tcgggtattc ggtttagttt ggttgcgcgt tcggtttcgg gatttcggcg 5460
tttcgttttg gatgcgtttt gtaggaatga tttttaacgg ggttgtttcg ttttatttgg 5520
gttttggggc gttttgcgta gagacgttgg tgcggaaatg ggcggatggg gttttgcgtt 5580
tttttaattc ggatcgttta gatatagttt tgcgggtaac ggagaagagg atttcgggat 5640
aggggtaagg atatcgcggg tggggtgcga acggtgaaag ggtttatttt tcgtttttag 5700

tatttttttt ttcgcgtcgt ttcgtaaaag ttttttgttt atattttcgg gttttcgaaa 5760
ttttatcgga cggcgttagg tcgggatcgg ggtattgttt tttagacgtt tgttcgggga 5820
tattatgcgt ttcgaatggc gagttgagat tgggggattt ttaattttgg tagtttttag 5880
ttatttagag gatttttttt tttatgttag ggagtaggcg attgttttat ttttattttt 5940
ttaagggggt agcgagatga aacgaaaacg tttcggtttt tttttaaatt ttttatagtt 6000
gtcgtgagaa aagggtaggg taggaagggt cggcggttgg gggtggtgtt gttcgaggtg 6060
gttggaagtt gaggtcgttt ttgtttttat taagacgatt ttaaatggga aaaaaaggaa 6120
gagtggtttt ttgaggtttt tcgggtgata ttttaaaata ttagtggggg aaatttcgtc 6180
gggaggggcg ggaggttcgt gtgattttaa tcgttagatc ggaggtttta ttgttatttg 6240
gagtttaaaa ggagtttttg aaattaaaag aagataggat gttgatagga aatttggttt 6300
ttttaattag attttaaata tttttttttt gaaattttta ttatatggat ttttagtttt 6360
tgtacgcggc ggttacgttt aaatagttgt gattttgttt aaagtttgat ttttttttaa 6420
aaggtattcg ttttttcgtg tttttggggg tgggggattt gggggagatg gtgtttaggg 6480
ggatatcgtt atttttattt tgtaattttc ggtaggtgta tttttttatt attaaaagtt 6540
ttatttttat tttaaagggg ttttttggtg gtttttttta aaatattagt gagttttgta 6600
ttttgtattt tttttgtata acgaatttag gttttgtaga ggttaataaa ttgggtttgg 6660
tttatatttt agggttaaat tttatgtttt aaatagtttt tgtataaatt gagtgggggt 6720
agtagttttg ttgttattat tgttgttatt tttttttatt tggagatttc gttgttagga 6780
gtatgttgat ttttttttcg ttgtagtcga agataatagg ggaattgtat tttatattat 6840
agtgattgag ttgatttata gttttggttt agattttatt gaaaggatga ttttttggga 6900
tatagtttat gtaagtagtt gatttaggat tttggtgtgg aagttcgaga ttttattttt 6960
ttgttttcgt gatatttggg ggaaggtttt tattgttttt aagttttttt tttattttgt 7020
attggagaaa tatttagtgt gtttgggatt tgggggagtt gtttagttta gttttttttt 7080
tttttttttt ggttattttt ttttttttta agttgttttt ttgtattttt tcgtttcgtg 7140
tttttagg 7148

<210> 52
<211> 7148
<212> DNA
<213> Artificial Sequence

<220>
<223> chemically treated genomic DNA (Homo sapiens)

<400> 52

tttgaagata cggggcgggg ggatgtaggg ggatagttta gaaaggaaga gggtgattag 60
ggaaaggagg ggaggggttg ggttgggtag tttttttagg ttttaggtat attgagtatt 120
tttttaatgt agggtggaga agaggtttaa aaataataaa gatttttttt taaatattac 180
gaaaataaga agatggaatt tcgagttttt atattaaaat tttagattaa ttgtttatat 240
aaattgtgtt ttaagaaatt atttttttaa tgaaatttaa gttagagttg tgaattagtt 300
tagttattat gatgtggggt gtagtttttt tgttgttttc ggttgtagcg aaagaggaat 360
taatatgttt ttagtaacga agtttttaaa tgagaaagag taataataat aataataata 420
gggttgttat ttttatttaa tttatgtaag agttgtttag ggtatgaaat ttggttttga 480
aatgtggatt aggtttagtt tattggtttt tgtagagttt aaattcgtta tgtagagaaa 540
atgtagaatg taaaatttat tggtgttttg aaaaaggtta ttagaaaatt tttttaaagt 600
gagagtgggg tttttgataa tggaaggatg tatttgtcgg gaattgtagg atgggggtgg 660
cgatgttttt ttaaatatta ttttttttaa attttttatt tttaggagta cggagaggcg 720
gatgtttttt gaaaaagaat tagattttaa atagagttat aattatttaa acgtggtcgt 780
cgcgtgtagg gattggggat ttatatggta aaaattttaa ggagaaaatg tttgggattt 840
gattaagaag attagatttt ttgttaatat tttgtttttt tttaatttta aagatttttt 900
ttaagtttta agtgatagta aaattttcga tttgacgatt aaagttatac gggtttttcg 960
tttttttcgg cgagattttt tttattggta ttttaagatg ttattcggga gattttaaag 1020
agttattttt tttttttttt ttatttagag tcgttttaat gggagtaggg acggttttag 1080
tttttagtta tttcgggtag tattattttt agtcgtcggt tttttttgtt ttgttttttt 1140
tttacggtag ttgtgagagg tttaggggaa aatcgaggcg ttttcgtttt atttcgttgt 1200
ttttttaaaa aaatgaaaat gaaatagtcg tttatttttt ggtataaaga aaaaggtttt 1260
ttaaatggtt gggggttgtt agggttaggg gttttttaat tttaattcgt tattcgggac 1320
gtataatatt ttcgagtaaa cgtttggaga gtagtgtttc gatttcggtt tagcgtcgtt 1380
cggtaaaatt tcggaagttc gagggtgtga gtaggaagtt tttgcgaagc ggcgcgggag 1440
gaggggtgtt ggaggcggag ggtaggtttt tttatcgttc gtattttatt cgcggtgttt 1500
ttgtttttgt ttcgggattt tttttttcgt tattcgtagg gttgtatttg agcgattcgg 1560
gttagggggg cgtaaaattt tattcgttta ttttcgtatt aacgttttta cgtaaggcgt 1620
tttaaaattt aggtggagcg gggtaatttc gttaaaagtt atttttgtag ggcgtattta 1680
aaacggaacg tcgaggtttc ggagtcgagc gcgtagttag attgaatcgg gtgttcgggt 1740
gtcgtcgcgg cgtttcgggt atttttttatt tttattgttt tcgaggtttt ggttttcgta 1800

gtttagacgt tcggagtttt agggtcggcg ttttttcgtt tcgggtttcg tatttatttt 1860
gaaggcgacg ggtagcgttt tgttgtagcg ttagtgcgag ggtagtacgg agtagaggaa 1920
gttggggttg tcggtgcgta cgagttcgtt tgcgtggttc gttagtacgt ttattatcga 1980
gcgtatttcg ggtcgggcgc gtttttcggg ttttacggtc gtttgcgcgt ttagcgcgtc 2040
gttgttttcg tttattttgt cgtcgtcgtc gtcgtagggg aaggtcgggg agggaggtgt 2100
gaagcggcgg ttggtgtttg ggtttacggg aatacgtata atagcggtcg ttagggcgtc 2160
gggtaggcgg agacggcgcg gtttttttcg ggggcggtcg gcgcgggcgt tttttcggtc 2220
gtcgttgtcg cgagaagcgg gaaagtagaa gcggcggggt tcgggtttta gggcgtaggg 2280
ggcggcgttc ggttattatt cgttagggtt cgttcgttgc gaggtttcgt tggttcgacg 2340
gtcgttcgta gtttgttcgg ttagtttcgt attttcggcg cgcggtttcg cgtgcggtcg 2400
tttttcgtgg ttgtttcggt tgtttgggtc gcggcggggt tcgcgcgggg ttgtgtcgtt 2460
gtcgtcgttt ttcgtttcga agttcgttcg cggtcgtttc gatttcgcgg tcgtagtttt 2520
agaataaatt ttttagaatt aagtggcggg gtcgcggtcg ttcgcgcggg gttagtattt 2580
tcggggttcg cggggcgggg ttggcggagc gacgcgtcgt atagttaatc ggcggagttt 2640
ttatcgcggg tatttcggtg gcgttcgcgg ggaggaacgg ggtttgtcgg aggtcgttta 2700
acggggaggg gcggaaggcg ttatttcgcg gaggaggttt tagtgttata gtttagggtt 2760
ttcgagagtt ttgggagttc ggggtaaatg ttagaaattt gttagaacgg ttcggttttt 2820
acggaaggcg tttttgtcgt tttttttcgg gtcgtagttt tttgacgttg gggcgtaatt 2880
ttttcgtttt tttttttcgt tggtcgcggt cgggtttttt tagtttttgt tgtttcgggt 2940
ttgtgatttt tgtaatttcg ggttgggggt cgcggggttt tagggtcggt gacgtcgtat 3000
tgggagtcgt tttaaagagg ttatttattt ttttcgtttc gtatattatt ttgatttaag 3060
agtttttatt ttatacggga ttttgcgcgt cgtttacgtt cggtcggcgg tttttgttgt 3120
ttttagtttt gcgcggtttt ggttttagtt tcggtggttt ttgtgttaaa tcggggatag 3180
gcggaaggga gttttttagg gattttaagt agtttggggt taataatttt tttttttttt 3240
gttttttttt taaaataagt tttaggattt tgtaggtttc gcggcgtcgt ttttcgttgt 3300
ggcggtttgt ggttttttga aaaatacgac gaggtttgta aaatgcgttt tttttttttt 3360
tttttacgta tgtaattacg gttttgtatc gtgaaacggt acgcgcgtcg gtggtaaaag 3420
aaaaatagta gtggttgtaa agttaagggt tttcgttttt agaggagaga attttttttt 3480
tttatgcggg tggaaagtgg tttttgcggg tttaatttta tttttttttg ggttcgtgcg 3540
tttcggttgc gtcgtaggga tcgcggatag tttcgttaag gtattgtttg ttcgttcggt 3600
ttcgggtttt cgtttttatt tttagtcgcg tggtttaatt ttttttgggt tttattgtaa 3660
attatttttt tttttttcgt tttttaagtt tgtcgagtag atttaggggt cggttatagt 3720
tgggagggta acgggaaaga ttaagttata attatttcga attatcgttt tagatatttt 3780
tttagatttt ggggaacgaa cgcgtgttga gttttttcgt cgttttggag acggggttag 3840
attttcgttg ttttcggttt tcgataggtg taaaataatg aattttaagt ttcggaagta 3900
aagaagttta ggattcgacg gtggtcgtaa gattttatta tggatttgat ttttgtttag 3960
cgcgcgttat ttcgtcgttg ttaaacgaaa ttaagtttcg cgtgcgtttt aggggcgaag 4020
gattttggat ttatttcgat tatcgggaga gttggttttt atttatttcg ggattttata 4080
gtacgttttt aggtcgtgtc gaaaggaagg acggtaaagg ttttttattg aattttttaa 4140
gagagtttgc gtttggtagt tgtcgattgc ggatttaggt tcgcgcgttt tcggacgcgt 4200
tggtacgagt agtagaatta gaggaaagcg agtgatttag tttgggcgtt tttattttcg 4260
ggaacgtttt cgagaaggcg tagcgcgtcg tggttaggta gggttttggt cgggggcggg 4320
taatacgtgt tgttttcgag taggttgcgg gattatgatt cgttgtttta ggtggtggta 4380
aattttattt gtcgaatggt ttcggtttgt atcgtgtttt ttgtttgttt tttcgtttga 4440
tttttttttt ttcgtttacg atgggtttat agataagttt ttagagaatg agggattttt 4500
gtgggttttg gtatttggcg tagggttcgg tacggtttcg gtttttcgta gggcgttggt 4560
ttttcgtggg tattagattt aagggattag ggcggcgggg ggaggggggg cgggtgtagg 4620
tttttgggtt tttagattaa ggtcgcgggg tcgtttggta ggtatagtgg cgggagtcgt 4680
cgttagttgg cgttcgcgtt ttgttagtcg cggaggtgcg ggttcggtcg ggttatagat 4740
gcgcgttagt tgcggtttcg ggtgtaggcg cggcgatcgt tttcgaggag ttgttttttt 4800
tttgttattt atgcggttag gttttagata aatcgatggt tttgtgttaa attaaggtcg 4860
ttttttttat ttttgataag atggacgttt tttgttttcg cgtttttagg tattcgggga 4920
agatttatag aataggttag tttgttttta atttttattt gttttttttt tatttcggat 4980
cgataaaaat tgtcgtttgt ttgatgggag ggagaatttc gattttttta tttggggtat 5040
gtagatattt tcgttttttt ttagttggta tggatcgtcg tttttttttt tttttttatt 5100
agatcgatgg ataaataggt tagttttttt ttagtggttt ttatttaaga gtattttaag 5160
ttgtttatag tagggttagg aagtagaagg ttaggatatt tttttatttt attttgattt 5220
agagttgggt aaatttagaa tttattttcg ggtaaataga gttagttttt ttgttttagg 5280
aaggggattc gttttttttt ggtatttagg agtgtttttt aagtgcgttt ttggtagtga 5340
gggtgtcgtt tttttagggt aggtgtgatt tatgtggatt ttgtggcgtt tgggtaggga 5400
tttttaggta tattagataa ggggtaggtg tgttttggga aatcgtttaa gaggtttatg 5460
ggttatggaa ggagttgggg tttatagttt ttttgtttga gcgtgttttt tttttttatt 5520
tatagcgttt tagggaaagt tgtttaaatt tttttgagtt ttattttttt tatttgtaaa 5580
gtggggtatt tatagtggtt ttttatagaa ttgtgtgtaa agtgtttagt ataggtttgg 5640
tatatggagg gtgttttagt tttcgggagt tattattgtt atgaaaaaat aagatttttt 5700
aatttttgtt gggggttttt gatttatttt tttttttttt gggttttata tttttatttg 5760

tgaaatgttt agtttttata tttaaagttt attaggattt taagttagtt tatgttgttt 5820
tgatttttta attcgtttat aggttgtttg ggggaggtaa ggattggttg tgatttattt 5880
ttacgtggag ttagtttata gcggggtttt tagtaattat tatagggcgg ttagagttgg 5940
gtttcgatga ttgtttgttt gattattttt tttagaattt tattttcgtt tttagtcggt 6000
cgtttttttg tgggtagatt tttttttgag tagtaattgg gttttagcgg atattgttag 6060
ggatttcgtt ttttttttag gaggtttttg ttttttatat ttcgaattat ataggagttt 6120
agtttagcga agagagtaga ggattttttt ttagaattga aaattttttt tagtttggtt 6180
ttaaattttt tgtttagagg gattcgtggt gaaatttatt ttttgtttag tgttttagaa 6240
tttaaagggg atatttatgt tttttattga gttttaattt ttttttttgt taatggaggt 6300
tattttaatt attttatttt acgggagggg gattaaggat tttttttagg aggggagggg 6360
tattattgtg attgatgatc gattgtttga agaaatagaa agaaaatgtt gttgagtaaa 6420
ttaggattta tttgtatttt gattttagat aatgattttt gaatatataa gcgagaaatg 6480
ttaatgaaaa atggtaatat atttgggttg aggggttgtt ttttgtaggt cggggtttta 6540
gttttagaga gtttagtttt ggggttattt attttgggta gtttttttgg aaggatttag 6600
aatgtgtggg agtataaatg tgttttttaa attatagaga tttttttttt tttttggaaa 6660
gttttagatt tggaggggag ggagaaggag taagggagag tagggtggtg agggtgttag 6720
gatttagatg ttgtttgtgc ggtttgagat ttttgtttgg tgtttacgtt tttttgagtt 6780
ttggtttcg agggtaaaat ggaagaata gtaatagttg gggtgttga ggtttattt 6840
tgtgttaggc gtcgtatatg ggtattgttt atggtattta atttttacgg cgttatatgt 6900
ggtaggtgtt atgtttatgt aagtaaagag gaacgttgtt cgaggttagt taggttagag 6960
agggttagat tcgggttaaa agtttgtttt ggtttaaaat gtggggtatg aacgtattat 7020
ttggttaagt atgttagtat ttttttttta gtggttgagt aatgggaaga gttagtattt 7080
agatatagag gaaagagtta ttgtgatggg gagagggagt tgggtttggt aaattttgtt 7140
aagtagtt 7148

<210> 53
<211> 7187
<212> DNA
<213> Artificial Sequence

<220>
<223> chemically treated genomic DNA (Homo sapiens)

<400> 53

atgatttgtg tatagtagta gatatatttt tagatgtaaa aataaaaata tttagaatat 60
agaaaattat tttttaatgg tgttggaggg tgttttgaat agagggtagt gagattatcg 120
aaattataaa tagaataatt taatttttaa aagataattt aattattagt gttttgaagt 180
ggaaatgggg atttttttgt tggtagttat attatttgat atttgttttt ttttattggt 240
tatatatttt ggtatttttg ggtgatagtt ttaaaagatt gaataaagaa attgataata 300
atggtttttt ttatggtaag gataggtagt ttggggtgga gttagaggaa gattaatttt 360
tcgttgtgta tttttttgtg tgttatttga attttgttat tatgcgtatt tattatttat 420
ttaaaaataa aagaaatgta ttgtattatg tttatagtgt ttttttgaat ttaataaata 480
tttattattg aataaaatta gtataaataa agtatatagt ttattgtatt tttgggattt 540
ggtttatttt gaaagttttt gattagtata ggaatgatgt ttgtttaaat tttgtttttt 600
tttttttttt tttttttttt tttgagatga agtttttttt tgtcgtttag gttagagtgt 660
agtggtatga ttttggttta ttgtaatttt tattttttaa gtttaagtaa tttttttgtt 720
ttaggtttta aagtagttgg gattataggt attagttatt atgtttagtt aatttttgta 780
tttttagtgg agataggatt ttattatgtt agttaggttg gtttcgaatt ttggatttta 840
ggtgatttgt ttattttcgt tttttaaagt gttgggatta taggtatgag ttattatgtt 900
tggtttaaat tttttttgta aaatttattt tgaatatatt aggttgtttt tatagattgt 960
aaaatttatt agatttgaaa ttgaataaag atatttttaa tatttatttt tagtttttag 1020
aatagagttg gtatttaggt gtgcgttgta agaatgaaag aattagttta gttaaaggag 1080
tagttttagg gaatggggtt tttttttacg tgtttttttt atggaaaggg gaggagggag 1140
tgtatatttt gaataattaa gtaaattgtt tatttaaaag tttaagggga aattatatat 1200
tttaattttt tttttttttt gttttttgga aaataaattt aatttgttat gatatgattg 1260
ttattttgta ttttgaaaag agaggtagtg atgaggtagt ggtatgtttt ttagttagat 1320
tttttttttg aattttttaa tttaaatgtt tttttttagt ttttagttgt tttgtttaat 1380
tgtaatgttt attttttatt ttaatattgt tttttatttt ttgttgattt aagataggtt 1440
tttagggaag aatttagttt ttttgttcgt ttttggagag tattgggttt ttttttaatt 1500
gttttttgag agtttgtaag agggatagaa aatgtagttg ttgttgtttt tttataggta 1560
tattggggag gtttgaaaag gagataaatt attttaacgt gttttgagcg gttgggtttt 1620
atgggttttt ttttttgttt attttataaa agtgttatta gtatatgaaa atatttttta 1680
tttaagtatt ttttcgaggt ttttagagtt tttgaagata ttttagtgtt taagaaattt 1740
aagttaagtt gaaaatagtt agattttgtt gaaagtttga attgtaaatg gaaatgaatg 1800
gattttggga ttggtttttt tggtttttat gtatgtttag ggttggttta ggaagtggat 1860

ttaggaggtt tgggtttatt tatatgaatt taggtttatt ttttattgtg gtatttggta 1920
ttaagtgtat taataattgg cgatgttgtt tttttatgaa agttttttat tgggtttgga 1980
gattttttgt agatttattt ttatttttat ttggtttatt taaattggga ggtaattaga 2040
tgtttgtatt aattgaagat taatagaggt agtatatttt tgttgaaagt tataaaattt 2100
ttatttattt ttttaagaga ttataatgta ttatattata tatatattat atgttatata 2160
tatataatta tatatatata attatatata tatagtgtat taatggtttt agaattggtg 2220
aatggatacg atagtgaaag gtgatagtta ttgtttttag ttgaaaaagg tagaggttgg 2280
agataggttt gttttatagt ttttattata gaaggtttgg ttttgttaga tttatttttt 2340
acggatagta tttattaatg ggagtaggga gaatatttta agatatttgt atattttttt 2400
aaaagaagtg atagaatagt aaaaggatat tttttgtgag tgatttttaa atggtattta 2460
taagttttaa attaggatta gaggaaggga agtttaggat taaaggaaag tgaaaaatgg 2520
ataattgata tttttataga tgattttata taataaagtt agatgatgta taagaaattt 2580
tattagatta tttaatttat ttgagggtag ggataaagtt ttatttattt tttaattatt 2640
tattagtatt taatatgatt tttaatatat aaagtgagtt ttttatatat tgtagtttaa 2700
taggaattat tttaattgta ttttatgttt agtaatttta gagttaagta aattgagatt 2760
cgagatagaa tttttgagat tgtttttagt tttgggaaat agtagatatt ttgattgata 2820
ataatggaat tttagttatt gaaaagtaat agttaaaagg atttaggaaa ggtattatag 2880
ttggttttta tattatatta tattaggagg tttagtgatt agttttggaa agcgtatagt 2940
attggatggt tgaggggaat taagaaattt aagtagtagt taattaaaaa aaaaaagatg 3000
ttgatatagt atggtggttt acgtttgtaa ttttagtatt ttgggaggta gaggtaagat 3060
ggtggtttaa ggttaagagt tttagagtag tttgggtaat agagtgaggt tttgttttaa 3120
taataatata aaattttaaa tagttaggtg tggtagtgtg tttgtagttt tagttattta 3180
ggaggtttgt ttgagtttag gagtttgagg ttatagagtg ttatgattgt attattgtat 3240
tttagattgg gtaatagagt agaatttttt ttttaaaata aaaaaaaata atggtaaata 3300
tatatgttta tttattatat attaatagta ttttttatta atattagtat acgttttata 3360
gatatttggt ataagtataa tatattttta tttatggtat tttggtaaag tgatggttta 3420
tgttaaaaat agtttattat tatgggtaaa gggatttatt taaggtttta ttttatagaa 3480
attaggtatg gttattaaga agaatagaaa tgtttggaaa atagatttta gtgggttatt 3540
ttttggaaag aaagtttaat tagtattgat aaaaaagggg gaatatgttt taggattatt 3600
tgtgttattg aaagtaagga atatagtgat tgaattaaaa ggggagaaat aaaagtagat 3660
attgtattgt tttttagtgg aaatattgtt tgaattatgt ggtaggttga tcggatttta 3720
ttttggggtt ttagtttttt ggacgtgttt ttttttcgta ttttaggttg atttatgata 3780
gttttgaatg atagttattt ttttaatttt tttttgattt tcgaagattt ttatttgtgt 3840
taatttttat ttagtttggt tattaattag tgtaaaataa ggggagatga tgtggattta 3900
ataaggtaag aaattgatta gttagcgtta tatataaagt tattaaattt ggtttttaga 3960
atttacgttt ttatgtttgg ttttttgttt aaagggaaaa aaaaattaat aggatggttg 4020
gagggagata tttttttatt tttattgaag attgatgagt tgttagttta ttacgtggta 4080
ttttgttgag gaatatgtga ataattggaa gagtttattt gatgattgat tagttgtgaa 4140
atttagtatt tattaattga tttttaatgt gagaggatta atttgggaat tgtgagtttt 4200
tacgtgtgga aaggaaaata tatttttttt atttagttat tgtgtaaaaa ttaataattt 4260
taatttgtat gtaagtaaga aataatatgt tttttgttta aaaaaattaa tattttttta 4320
gttttttttt tttttttttt ataatttgga tatatttttt cgtgtgtgtg tgtgtgtgtg 4380
tgtgtgtgtg tgtgtgtgtg tgtgtgtgaa tagttttttt aaaggtttgg gtttttttta 4440
aatgtatgtt ttttaatttt atttaaaaat tttttttata gtaatatgtt ttgtttattt 4500
tattttagta taaggtagat gtatagtaaa tgtgagttga ttttagtttt ttttttgaaa 4560
atagttatgg gaaatttagg taaagaatgt gttgtttttg ttaatattgt tttttaagtt 4620
ttagatatag ttaaattttt agttaattat tttttgggtt ttaggttttt attggggttt 4680
tttaaaatat ataaaattaa agtgacggta ggaggttatt agtattatat aatttaagta 4740
aataataaat gtgtttattt tgtttggtta ttgattattt gtttttttat ttcgttaggt 4800
aggtatttat atatacgatt tttttttttt tagttttgta gagaatgagt tttttttttg 4860
ggttttatta tttataaaag aagtttgggt ttttgatagt atgtattttt ttttatttta 4920
gttgttggta tttttgtttt taggaaagat tatacggtat taggatggtc gttagaatta 4980
gagttttttt tttttcgtat ttttgttaag gaattaaaga gagtttttta taggtaatta 5040
tgaggaagtt gaggagaagt tagatttgtt tgtcgtagtg ttatattttg tagttattag 5100
ttttgtaggg gaaggttaga ggtagagaga gaagatgttg tttagatttg gtaggttttg 5160
gaagaagttt gagagagaaa tgtatttatt tagggattta gatagtgagt tttttttatt 5220
tgggatttag ttttttattt agtcgataga tggaatgaaa atggagaaat ttttttttcg 5280
ggaagaagtt aagaaatttt ggtattattt tatgtttaga aaaatttcgg ttttttaggg 5340
ggttattttg tttattaaaa gttatgaagt atattgggag atttgtagga tagtgttttt 5400
tagttaggta tgtgttttgg ggggagagta ggtaagagtt tgtaggtggt agtggatagt 5460
tgggatggat ggagagtagg ggaaaaggtt gttaggagtt tgattttagt ttaattatag 5520
atttggtttt tgggtattta ttataggatt tggtaaagat taagtttttt tttggttttt 5580
ttattttttt ttggtattgt ggaaatgttg taagaatgat atgatgatat tgttaatatt 5640
agtaattatt tatttatatt gaagatatag agttttgttt tatttattta tttttgtatt 5700
ggaggtgatt tatttagaga tataagttag attgtatttt tagttaggaa attgagaatt 5760
tagagtaatt attagaattt ttttgtagtt atttttttgt attttattaa tatgtggatg 5820

agtaggttaa ttttatttgt tgataaagtg gggtgtattg gatttttttt taaatatttt 5880
tatatttatt tacgatggtt ttaattttta tagtttatat ttaattattt tataggttta 5940
tgagggattt ttttaatagt ttgttaaagt tattttataa ttttaaagta cgttgaggtt 6000
tttaggtaaa agttgttata ttatttttag tattatgata gtaaaaaagt gatttttttt 6060
tatttatttt tttatatgag tttttaaaaa aattaatttt gatgtgagat tatatttttt 6120
ttttttagaa gtattttttt tttgatttat gttgtgttgg ttgatttgta gttattatga 6180
ttaattttat gttattaaga taaagggata ttttattgtt tatttttttt ggtaatattt 6240
atattttaaa tgttaaatta aaattgagaa tttgtattag gtttttaaat atgaagatat 6300
tgaattaaaa atatgtaggg tagagtaaaa ttttatagtc gagtaatgtt atttaattaa 6360
gtaagtaata gaatagggta attgattgtt taaggtagtt aagtattttg tttgaaaagg 6420
taaggatatg tagtaatggt aagttaatta ttaaataata atgattattt tgttggttat 6480
gtgtaattag aaaattattt ttaagaatta ggtaattaaa ttttttttga aatttttttt 6540
ttgaatttta ttgttaatta aattaaaatt aagatgtttg atttttatat attttgaaag 6600
gtatataaag ttaggtgttt ggagttatga gaggtaaagg tgatgtaata tataatgatt 6660
tgtaggtata tgtattgtaa ttttgtttgt atataatttt atagatttga atgtattata 6720
ggttttgtag aataggatag aattaaattt agaatgtttt gatttatttt acgattaatg 6780
taataaatat gtattgggag tttattatgt ataaagtttt gtgaggaata aaaaagtaag 6840
gtatattatt tatgtaagat aattattatt agaatttttt aatcgtttat atttaattag 6900
ataaaattgt ttaaggtttt gtacgaataa tgtagagtaa aatatttttt atgttaattt 6960
agggattttt taaaggttgt ttaataattt atttaataaa gaaaatttaa ttgaggtggt 7020
tttgtgtttt tatagatatt attatttgta tattgtaaat tgtatttaaa attggaaagt 7080
tttgaaattt ttaaattatt tttagattta tagtttatag tttttgtatt atgtgtgtta 7140
aagaaataat ttaaaataac gtaatggaaa tgtgtttgtt ttttaga 7187

<210> 54
<211> 7187
<212> DNA
<213> Artificial Sequence

<220>
<223> chemically treated genomic DNA (Homo sapiens)

<400> 54

tttaaaaagt aaatatattt ttattacgtt attttgaatt atttttttaa tatatataat 60
gtagaagtta tggattgtaa atttgaggat aatttaaaaa ttttaaagtt ttttaatttt 120
ggatataatt tgtaatatgt aagtgatggt atttataagg gtatagaatt attttaatta 180
aatttttttt attgagtaaa ttattaaata gtttttaggg aatttttaag ttaatataaa 240
aaatatttta ttttatatta ttcgtgtaaa attttaagta attttgttta attggatgtg 300
agcgattgaa aaattttaat ggtaattatt ttatataagt aatgtgtttt atttttttat 360
tttttatagg gttttgtgta tagtaggttt ttaatatata tttgttatat tgatcgtaga 420
atagattaga atattttagg tttaatttta ttttattttg taagatttgt agtatattta 480
agtttatgaa gttgtatgta agtagagtta taatgtatat gtttgtaaat tattgtatat 540
tatattattt ttatttttta taattttaag tatttagttt tatatgtttt ttaaaatatg 600
taagagttaa atattttagt tttaatttaa ttagtaatag aatttaaaag aagaatttta 660
aaagaaattt gattgtttga tttttagggg taatttttta attgtatatg gttaatagag 720
tagttattat tatttgataa ttgatttgtt attgttatat atttttgttt ttttaggtag 780
aatatttaat tgttttgaat agttaattgt tttattttat tgtttgttta attgggtagt 840
attattcgat tataaaattt tattttattt tgtatgtttt tggtttaata tttttatgtt 900
taaggattta atgtaagttt ttaattttaa tttaatattt ggaatgtaga tattgttaga 960
ggaagtagat agtaggatgt ttttttgttt taatagtatg gaattgatta taataattat 1020
aaattagtta atataatatg aattaggaga aaggtatttt taaggggagg agatatgatt 1080
ttatattaag attgattttt taaaaagttt atatgagaaa ataagtgaaa gaaaattatt 1140
tttttgttat tataatatta gaaatgatat gataattttt gtttgagaat tttaacgtat 1200
tttgaggttg tgggatagtt ttagtaagtt attaaagaag ttttttataa atttataaag 1260
taattaagta tggattatgg ggattaagat tatcgtaaat ggatatgaga gtatttggga 1320
aggagtttaa tgtattttat tttattaata aatggagttg atttgtttat ttatatatta 1380
atagagtgta gaaagatagt tatagaggag ttttggtgat tattttaaat ttttagtttt 1440
ttaattgagg gtatagtttg atttatattt ttgagtagat tatttttaat gtaaaaataa 1500
ataaataaaa tagagttttg tgtttttagt gtgaatgaat gattattgat attgatagta 1560
ttattatatt atttttgtag tatttttata atgttaagaa aaaatggaaa ggttagaagg 1620
aaatttaatt tttgttaaat tttatgatga atgtttaagg attaaatttg tagttaagtt 1680
agagttaggt ttttgatagt tttttttttt attttttatt tattttagtt gtttattatt 1740
atttgtaaat ttttatttgt ttttttttta gaatatatat ttggttgaag ggtattgttt 1800
tgtaggtttt ttaatgtatt ttatggtttt tgatgggtaa gatgattttt tgagaagtcg 1860
gagttttttt gaatatgaag tggtgttaga attttttggt tttttttcga agaggagatt 1920

tttttatttt tattttattt atcggttgga tgagggattg ggttttaggt gggaagggtt 1980
tattatttga gtttttggat ggatgtattt tttttttagg ttttttttag aatttgttaa 2040
atttggatag tatttttttt ttttgttttt ggtttttttt tgtagggttg gtggttataa 2100
ggtgtggtat tgcgataaat agatttggtt tttttttagt ttttttatgg ttgtttgtgg 2160
gaagtttttt ttgatttttt ggtaggagta cgggggaaag agaattttga ttttggcggt 2220
tattttggtg tcgtgtggtt ttttttagag gtaggagtat tagtagttga aataagagga 2280
gatgtatgtt gttaggggtt taagtttttt ttgtaaatga tgaggtttaa aggagaggtt 2340
tattttttgt aggattggga aaaaggaaat cgtatatata gatatttgtt tggcgggatg 2400
ggaaggtagg tggttagtag ttaggtagaa taaatatatt tattgtttgt ttgaattatg 2460
tagtgttaat ggttttttgt cgttattttg gttttgtgta ttttaaaagg ttttagtgaa 2520
agtttgggat ttagggggta attagttaag agtttagtta tgtttggggt ttaaagagta 2580
gtattagtaa agataatata tttttgtttt aaatttttta tggttgtttt tagaagaagg 2640
attagagtta gtttatattt gttgtgtatt tgttttgtgt tggaatagaa tagataagat 2700
atgttgttgt aagagaagtt tttggataaa aattaaaggt atatatttag aaaaagttta 2760
ggtttttagg aaggttattt atatatatat atatatatat atatatatat atatatatat 2820
atatacgaag aagtatatta aagttgtaag gagaagaaag gaagagttag aaggatgtta 2880
atttttttga gtaagggata tattgttttt tatttatatg taaattgggg ttattgattt 2940
ttgtatagta attgagtaaa gggaatatgt tttttttttt atacgtagaa atttataatt 3000
tttaagttaa tttttttata ttaagaatta attaatgggt gttaaatttt ataattggtt 3060
agttattaga taaatttttt taattgttta tatgtttttt aataggatat tacgtaatag 3120
attgatagtt tattagtttt taatgaggat agggaaatgt ttttttttag ttattttatt 3180
gatttttttt tttttttgaa taaagagtta gatatgagaa cgtggatttt aagaattagg 3240
tttgataatt ttgtgtgtga cgttggttag ttagtttttt attttattga atttatatta 3300
tttttttttg ttttatatta attgatggtt aggttaaata aagattgata taaataagag 3360
ttttcgggga ttaaaggaaa aattgaaaaa taattattat ttaaaattat tatgaattag 3420
tttaggatac gaaaaagaaa tacgtttaga aaattgaaat tttagaatag agttcgatta 3480
gtttattata tgatttaaat agtattttta ttgggaagta atgtaatatt tgttttattt 3540
tttttttttt taatttaatt attatatttt ttgtttttaa taatataaat agttttggaa 3600
tatgtttttt tttttttgtt aatattgatt aaattttttt tttaagaagt gatttattgg 3660
agtttgtttt ttaggtattt ttgttttttt tgatagttat gtttgatttt tatgaaataa 3720
agttttaagt aagttttttt atttatgata atgagttatt tttaatatgg attattattt 3780
tattagaatg ttatgagtgg agatatattg tatttgtatt aaatatttat ggaacgtgtg 3840
ttagtattaa tgaaaggtgt tgttgatata taataaataa gtatgtgtat ttgttattgt 3900
ttttttttat tttaggaaaa gggttttgtt ttgttgttta gtttggagtg tagtggtgta 3960
attatagtat tttgtaattt taaattttta ggtttaagta agttttttga gtagttggga 4020
ttataggtat attattatat ttggttattt aaaattttgt gttgttgttg aaatagagtt 4080
ttattttgtt gtttaagttg ttttgaaatt tttagttttg agttattatt ttgtttttgt 4140
tttttaaagt gttgggatta taggcgtgag ttattatgtt atgttaatat tttttttttt 4200
ttaattgatt gttatttgaa ttttttaatt ttttttagtt atttaatgtt atacgttttt 4260
tagagttagt tattaggttt tttggtataa tatggtgtgg aagttagttg tgatattttt 4320
tttgaatttt tttaattgtt attttttagt aattgaaatt ttattgttat taattagagt 4380
atttattatt ttttaaaatt aaagataatt ttagaaattt tatttcgggt tttagtttgt 4440
ttggttttgg aattattgaa tatgagatat agttgagata atttttgttg aattgtaatg 4500
tgtagaagat ttattttgtg tgttaagaat tatattagat attggtagat aattgaaaaa 4560
tgagtaaaat tttgtttttg tttttaagta ggttagatag tttagtggga ttttttatgt 4620
attatttaat tttattgtat aaaattattt atagggatgt tagttattta ttttttattt 4680
ttttttagtt ttaaattttt ttttttttga ttttaatttg gggtttataa gtgttattta 4740
aaaattattt atagagaata tttttttgtt gttttgttat tttttttgag aaaatgtgta 4800
aatattttga aatatttttt ttatttttat tagtaaatat tattcgtgaa aagtaaattt 4860
agtaaagtta agttttttgt gatgagaatt atgaaataag tttattttta atttttgttt 4920
tttttagtta aaggtaatga ttattatttt ttattatcgt gtttatttat taattttaaa 4980
gttattgata tattgtgtgt gtataattat gtgtgtataa ttatgtatgt ataatatgta 5040
atatatgtat aatgtaatat attataattt tttaagaaaa taagtgaaga ttttgtagtt 5100
tttagtaaaa atatgttgtt tttgttggtt tttagttgat ataggtattt agttattttt 5160
taatttggat gaattaaatg gaaatggaag taggtttgta ggaggttttt agatttagta 5220
ggaagttttt atgaggagat agtatcgtta attattagtg tatttagtat taaatgttat 5280
agtgaagggt gagtttgagt ttatgtgagt gagtttaagt tttttaagtt tattttttgg 5340
gttaatttta gatatgtatg agaattagag gggttaattt tagaatttat ttatttttat 5400
ttatagttta ggtttttagt agaatttgat tgtttttaat ttgatttggg ttttttaggt 5460
attaagatgt ttttaggaat tttaaagatt tcgagaagat atttaaataa aaaatgtttt 5520
tatatgttgg tgatattttt ataaaatgga taagaaaaga ggtttatgag atttagtcgt 5580
ttaagatacg ttgaggtggt ttgttttttt tttagatttt tttagtgtgt ttatgagagg 5640
atagtaatag ttatattttt tgtttttttt atagattttt agaagatagt taaaaagaag 5700
tttagtgttt tttaagggcg aatagagaga ttaagttttt ttttaaaaat ttattttaaa 5760
ttaataaaag gtgagaggta gtgttgaggt ggggggtgga tattatagtt aaataggata 5820
attgagagtt ggaaaaggat atttgggttg aaggatttag gagagaaatt tgattgggga 5880

gtatgttatt gttttattat tatttttttt tttaaaatat agaatagtaa ttatgttatg 5940
gtaggttaga tttatttttt aaggaataag gaaaagggaa aattaggata tatgattttt 6000
ttttaaattt ttaaataaat aatttattta attatttaaa atgtatattt tttttttttt 6060
tttttatgga aaagatacgt gaggaagagt tttatttttt gaagttgttt ttttaattgg 6120
attaattttt ttatttttat agcgtatatt tgagtgttag ttttgtttta ggaattggag 6180
ataagtgttg agagtgtttt tgtttagttt taggtttagt ggattttgta atttataggg 6240
atagtttagt atatttaaaa taagttttat agggaaaatt taggttaggt atggtggttt 6300
atgtttgtaa ttttagtatt ttgggaggcg aaggtgggta gattatttga ggtttagagt 6360
tcgagattag tttggttaat atggtgaaat tttgttttta ttaaaaatat aaaaattagt 6420
tgggtatggt ggttggtgtt tgtaatttta gttgttttgg agtttgaggt aggagaattg 6480
tttgaatttg ggaagtggag gttgtagtga gttaagatta tgttattgta ttttagtttg 6540
ggcgataaag ggagatttta ttttaaaaaa aaaaaaagaa agaaagaaag aaataaaatt 6600
taggtaggta ttatttttgt attaattagg gatttttaag tagagttaaa ttttaaagat 6660
gtagtgaatt gtatgtttta tttgtgttaa ttttatttaa taataaatat ttgttgaatt 6720
taaggaagta ttgtagatat ggtgtagtat atttttttta tttttgagta ggtaataaat 6780
acgtatagta ataaaattta aataatatat aaaaaaatgt ataacgaaag gttgattttt 6840
ttttgatttt attttaggtt gtttgttttt gttatggaga gaattattat tattagtttt 6900
tttatttaat tttttaaaat tgttatttaa agatattaaa atgtgtgatt agtggaaaag 6960
gataaatatt aaatggtgta attgttaata aaaaaatttt tatttttatt ttaaggtatt 7020
gatggttaga ttgtttttta aagattagat tattttgttt gtagtttcgg tgattttatt 7080
gttttttgtt taaaatattt tttaatatta ttgagaaata gttttttgta ttttgggtat 7140
tttgtttttt gtatttgaag atgtatttat tattgtgtat aagttat 7187

<210> 55
<211> 11260
<212> DNA
<213> Artificial Sequence

<220>
<223> chemically treated genomic DNA (Homo sapiens)

<400> 55

tgattttatt agaattatat atttaggtga aatttttaag tgttgaagat aaagttttat 60
gttgtattta atttggataa agtgtcgggg gtattttatt aagtttttgt tagtaggaga 120
tagagagaaa gttgttattt taaatttgga aattatgttg agtttttgta ggatgtgaaa 180
tgaagaattt agtttatggt gggataggag gagggaaaaa aattagggtg agttttattg 240
atttaagtaa ttgatagata ttatttatga atggttaaat ggttattagt tttggttttt 300
gattttatgt agttatggta ggaaggttat tttataaatt ttgaagttga tattttaagg 360
atatttgtgt gtgtgtgtgt gtatatatat atatatatat atatatatat atatatatat 420
atatatatat atatagtttt tttttttttt tttttttatt tttttttttt gagatagggt 480
tttgttttat tttttaggtt ggagtgtatt ggtaagagta tagtttattg tagttttaaa 540
attttaggtt taagggattt ttttgtttta gttttttgag tagttaggat tatacgtata 600
tattattatg tttggttaat ttttaagttg tttttttttt tttttgtata gagatagaat 660
tttatgtttt ttagggtggt tttgaatttt tggtttttga tttttttgtt tcggtatttt 720
aaagtgttat tattataggt gtgagttatt gtattttggg taggatttat tattttttaa 780
atagtttaag gtattttttt aaaaatagtt ttttaaggta taattgattt ataataaatt 840
atatatattt aaagtatata gtttgatacg ttttgattta tatgtatata tttataaaat 900
tattattata attaagatga taaatttgtt atttttaaaa gtttttttat gtttttttgt 960
aatttttttt ttttattttt tttatttttg ttttttagta attagtgatt tgttttttgt 1020
cgttgtggtt tttatttttt agaattttat ataaatggaa ttatataata taattttttt 1080
gattgtttgt ttttatttgg tataattatt ttgagattta tttattgttg tagtgttagt 1140
agtttttaag aaaatttttt tattcgttta agaatgttat tgttattttg aattaagtta 1200
ttagtaaaag aagagtaaat ttaagatgta gtttgatttg ggatttttaa tttttggtat 1260
tttcgtttag ttgatttttt ttttaaattt ataaggtatt gtaaatattt tagaatgagg 1320
atattttttt ttatgtgtat ttgtgtagtg attgttttat agttattttt atgaaattaa 1380
agattatttg ttatttaaat ttatttttga ttttttgatg tattttgtta tttttttttt 1440
aaagaaggag ttatattatt ttgtttttta tatatattgg tatggtattt taattttagt 1500
ttaatttttt gggtattttg ttttaatttt tattttattt tatgtttatg agtgagtata 1560
ttttagtaat ttatttaaga tgtttttatt agtattgaag aattggaatg gaagtattta 1620
taatttgttg agaagtggga aagttatgtt ttagattttt tataatattt gaattttagg 1680
ttatttaaat ttattggttt gtttttttaa taaaaattat ttgtttatat tttttttgat 1740
taaaggagtg gtggtattta atgtaattta tttttttttt tttttttttt tttttttttt 1800
tttgagatgg agttttattt tgttgtagta tagtggtacg attttagttt attgtaattt 1860
cggtttttcg ggtttaagta atttttttgt tttagttttt cgagtagttg ggatttatag 1920
gtatgaatta ttatattcga ttaatttttg tattgttagt agagacgggg ttttattatg 1980

ttggttaggt tggttttgaa tttttgattt taggtgattt gttcgtttcg gttttttaaa 2040
gtgttggtat tataggcgtg agttattgcg tttggttatt tattttttta aaattttgga 2100
gcgtagaggt tggtagtttt tttaagatgt agagttatta tttttatgtg aagtaaatat 2160
ggatttattt attttataaa ttatagtttt tttaattttt taaaatgtat ttaattgata 2220
taattttttt taataattta ataattattt tgtgtttgtt gttagtaagt aagatattgt 2280
gtagatataa cgatgaatag agatagtttt ataattttaa taatagtaga aataatagaa 2340
ttgtagttgt tattttattt agtatttatg tgtcgagtat tgtgttatat attttatata 2400
tattatttta aaatgtttat aataattgtg aggtaggtga tattattttt gtattatagt 2460
tggtgaaatt gaagtgtaaa gttgtttaga attttgtata atattgtata atttacgagt 2520
gataaaatta ggttttggag ttagttttga tttaaaagtt tatatttttt tggttgtgtt 2580
atgttttttt aagaagtagt aggtagtaga atggggtggg agaagtatat agattatata 2640
atagatgata ttatagaata taatgtggtt gttgtaagta aagtataaat aatatagttt 2700
gtggaagaaa tttttaattt ggattatggg gaaggtatta taaaggaagt gttatttgaa 2760
gtgggttttg atagtgtaat attaaaggag gtaaatattt atttgggagg gtatatttta 2820
gggagagtta aaataatatg taaattgagg gtgattttta gaggatggta ggtagtttga 2880
tattgttaaa tgggttatgg tgtattggaa gtagtattta gaaggttaga ttagatataa 2940
atgatagtta aatttgtatt ttttaaagta tatttaaaag gttgagtttg taggatttat 3000
taagtggata attagatttg tatttatggt atttagtgat agttttgatt tttaatttga 3060
ggttgtaaga aaaggagttt tgagttggat gtttgattag ttaatgtaag ttttattatt 3120
attttggaaa ttttatgaag gtaggtattt attttgattt gttagtatat ttttagtatt 3180
tagtaagaag tatttatgtt aagtataaaa tggaaattta agagtaagga agataaagaa 3240
atgttagaag aagagttaag agaaattaaa gaagtcgatt tttaaaaaat tttatttaga 3300
atttttttgg tgttttttat gatagttgat ttttttatta gtatagattg tagttatttg 3360
ttagagagta atgtgatata ttaataattg tttatgtgta gttattgatt tttttaataa 3420
tttatagttt agtaaaattt tttttttagg tttttgttat gagtatattt gtaatattgt 3480
aataaaaagg agataagttt ggatttatag gttttagatt tagttagatt aataaagaga 3540
tagtatttaa gtttttttaga attgttatcg tttttgtttt agggtgtttg aattgttaat 3600
atttataatt tttttagtta ggtattagaa aatggtaata agggaatgga gtttttattt 3660
gttggttaag aaatgggttg ttgtgtaggt aagaaatttg ttttgtagag gagggggata 3720
gtatagtgga gagtagaaaa gattaaggag aagggtatta agggttaatt ttttttgaaa 3780
gtttttttag agttattaga ttgatttttt tattattttt ttatttttat aatattttaa 3840
tttttatttt tattaaatta attgaaaatt gtatttttat attttttttt tttttagatt 3900
gaaagttttt taaatgtaag gagagttttt tttatttttg tgtttgtgta tagttgatgt 3960
ttaaatattt tttgtattaa attgagttag gcgttgttgt aggtgttaat ttaaagataa 4020
gttttttgtt tttatggagt ttatatttta tttgtaatga tagaagtatg gttattatag 4080
atttttttgg atttttttag tgtttttttt ttttttttcg tagatagtat ggtaaagttg 4140
aatgtgagtg aagaaatgga atttttttag ataaaaattt tgtatgggat aagggaagaa 4200
attttttttt tttttatttt ttatattaga tttattttta ttttaggatt ttgaattttg 4260
gttagttata tattttttta taagaattat taaatttttt tgagtatgat atttttgaaa 4320
ttttatttat gaataaaatt atgaaaaatg taattttttt ttatgtatta ttagtttatt 4380
tggataatta tatagttatt tttatttatt attggatgag gttttagtga ttataaggtg 4440
aagttattgg tggagtttta aaaatgggtt attaatttga gtagttttag tatattgtat 4500
ttgtttttaa gttgatataa ttggagttgt ttatatagat taattatatt gtgggataaa 4560
taaattttga tgaattaggt tgagaagttg aatattagta agtatatttt ttaaaattaa 4620
aagttttttg aagagaaata tggaattatt aggttgtatt atttttatta gtgtttaaag 4680
taaggatttt tttatttttt taatggttgt tttttaatta tagttttaag ggttgaatta 4740
aatgtttttt ttatttttaa tttttttggt ggagttagat atgttaatat ttatgtttag 4800
gatatttgtg taggtgtgtt gatattttaa gagtagtatt ttattataaa aattgttaat 4860
agtgattagt tttggggtga tagtagtgtt atagagagta atttgagtat tttatttttt 4920
attgttttaa gaagtgtttt agaattattt ttaatagtgt gaaattattt tgttttttag 4980
tatttttgtt atgagaaaaa gaatgtgata agttatttaa tttatgaaat ttaagttata 5040
tgtgaatttt gttaggtatg tgtgtaatat ttggtatttt tagttatttt ttatattttg 5100
ggttgtaaaa atttttatag tttgttttta ttaggtaagt atttgtggtg ttttttttta 5160
tataaaataa gttatttatt tagtatgtag tttagttgat atttttattt tgtttttaaa 5220
taattaaatt aaaagtattt aagtatattt agttatgata gatataaata tgttttaagg 5280
ttttgatttt ttttaaaatg agttttatta gttttatggg atgttttgtg aaaaagtatt 5340
taaatagtaa atagttttgg taaaaatgtt atattttggg tgttttttta gttattttat 5400
attagtatat taaagattgt gaagagtttt gttaaaaaaa aaaaaaaaaa ataaagaaag 5460
aaagaaaagt atattttagt atttttcgga tttgtttggt tatatttttt tttttttagt 5520
gtttattgat attttcgttg aaatgttttg gggagtattg ttattgaagg gttaataagt 5580
tattatttga aaaagaaata gtgttataga attattagtt gagttaaata tatttgttat 5640
atagaatttt tagaaagaaa ataagttgat ggatgggata attaattgag aatttttaaa 5700
tcgaaattta aaagtaggaa agtaattttg atttagagat gtttttttgt atttgttttg 5760
tttaaattag gatttaaaaa ggtttttaat ttggttgtat gtttattaaa ttttttttaa 5820
tggaaaatag taattttttt tttttgtttt tttttttatt tttatgatat ttatttgttg 5880
aggagattag gttatttgtt ttaatttata gtgatttatt ttagtgtttt ttgattttac 5940

gatggtggga aagtaatata gttagtatat tgtttaattt atggtggggt tatgttttaa 6000
taaatttatc gtaaattgaa aatatttgta ttggatttgg atgattgttt tttggtggtg 6060
ttgtttaatt tttttttttg ttttttgaat ttttttgtag ttggagttag ggaggtttga 6120
ttagatttat tttcgatttt tgatattttt tgtttttatt tttaataatt tttggtgtta 6180
atttaagatt aatttgtgaa tttagatgtt gttagtttgg tttaattatt atgaagtttt 6240
tattaatttt ttttttaatg gttttaaaaa ttagatattg atgatagttg tttagattta 6300
ttattttatt agggattaaa gtggtggtat tttttttttt ttatttgttt tttttttttt 6360
ttttttttta tgtttggtta attttttgta tttttagtag aggtggggtt tcgaaagtgt 6420
tagttaggat ggtttttttt tttttttttt tttttttttt aaagagatgg ggttttattt 6480
tgttatttag gttggattgt agtggtataa ttataggtta ttgtagtttg gattttttgg 6540
gtttaagaga tttttttgtt ttagttttta gagtagttgg gattataggt gtatattatt 6600
atgacgttat tacgatatta ttataagtgg ttgttttaaa ttatttgtag agatggggtt 6660
acgtcgtttt ttttaggtta gttttgaatt tttgggttta agttattttt ttattttagt 6720
tttttaaagt gttgggatta tagttatgag ttattgtatt tagttaaagt ggtaattttt 6780
taattatttt atttttttgt atttgttgaa attttataaa agattttttt tttattaatt 6840
ttttggttat tggaaatata ttttatgtat aaaaggtaga ttaaatatat tgattttttt 6900
tttttttttt tttttttttt ttttttaaat tagtttttag atgagtgtat ttttttagtt 6960
gttgggttgt atgatttgtt tgttaagttt ttattggttt gttgtatgtt aggttttttt 7020
tttaattttt tattttattt ttatttagag ttttgtgaaa attagtcgtt ttttttagat 7080
atatagttgt tttttaatat ttgtgggtga ttggatttag gattttttga agatattaaa 7140
atttgaggat gttttagtta tttatataaa atcgtgtaat atttgtgtat tatttatgta 7200
tattttttcg tatattttaa attattttta gattattttt aatatttatt ataatgtata 7260
tgttgtgtga atagttgtta tattatattg tttaggtaat aatgataaga aaaaaagttt 7320
gtatatgttt agtatagatg tagttttttt ttatatattt ttgatttaag attgattgaa 7380
tttatggatg taaaatttat agatatagag ggttgattgt atatattttt ttatttttaa 7440
ttatttttta tagtttgtgt tttttttaaa gtttttgtaa ggttttttgg aatatattgt 7500
ttttggatgt agttatttgt ataatttttg tttttttttt agtattaggt taaataattg 7560
tttgtttttt ttttgttttt tattgaggta taatttaaat agaataaaat gtattatgta 7620
gagtaaaatt tgttttatta attttttttt ttatggattt tttgtgtttt gtttaatttt 7680
tgtttatttt aaaattatga agattttttt ttattttttt tttagaaatt tatgttttgt 7740
tttttatttt taggtttatg atttattgtg atttaatttt tagtataatg tgagatagaa 7800
tttatggggt attttttaaa gttatgaatt tttaattgat ataatattaa ggttacgaaa 7860
agttttgtga aatgaagttt atatatttaa tattttttaa atttatttgg ttaaaaattt 7920
tttttttatg atagttatta tgttttggga aatattgatt taaagtattg ataaattatt 7980
gttgttaagg gaatattata taattattat tagttgagtt aagtattttt gttatataaa 8040
atttttagaa ttaattattg aaaagattat ttttttttta tttaattgta gttgagtatt 8100
tgtataaatt aggtagtttt atgtgtggtt ttatttttgt attttttttt ttttattttt 8160
ttgtatgttt attattggat tgatattata gtgttttaat tattgtaatt ttataatggg 8220
ttatgatatt tggtaataaa agttttttag tttaattata attttttatg attgttttgt 8280
ttatttattg ttttttgtat tttgttatag attttttagt ttttatatat atataaaagt 8340
attgggattt attttattta tttatttttt ttttttttga gatagagttt tattttgttg 8400
tttaggttgg agtgtagtgg tatagtttta gtttattgta atttttattt ttttaggttt 8460
aagtgatttt tatgttttag ttttttttaa gtagttggga ttataggtgt gtgttattat 8520
gtttagttta ttttttaatt tttagtagag atagggtttt attatgttgg ttaggtttgt 8580
ttcgaatttt tgattttaag tgatttattt gttttagttt tttagagtgt tgggattgta 8640
ggtatgagtt attatatttg gttgaagaat aaaattgata taataatttt ggtttgtatt 8700
tatttaaaga tattgaattt attatttaaa attatttaga agagaaattt ttaggtttag 8760
atagttttat tggtgaattt ttttaatatt taaggaagaa ttagtattta ttttatgtag 8820
atttttttag agaatggaaa agaattaata tgatttaatt tttataaagt tattgaattt 8880
agatatttgg taaggttatt ttttatgata tagattgtaa atattttaaa atacgagtaa 8940
aagggaggat ttatttaaga aattttggtt tgtttgttta atatttagtg tgatatatta 9000
tattaaaata ataagagaaa aaaatagtat ttgaatagat gtagaaaaag tatttgataa 9060
agtttaatgt ttatttatta ttaaaattcg taggaaatta gaaattggag gaagtttttt 9120
taatttgata aagagtattc gaaaaaaatt tgtagtattt gttttttttt tgagatttac 9180
gtgtattttt aagattagta tttgtatagt gtaataaagt taagtataat aaaatgagat 9240
atgtttgtat taggaatttt tataatgtga gagcggtttg tgaagttaaa aatattttta 9300
taataatatt aaaatattta tttttttaaa atttttatta ttttataagg atttgatgtt 9360
attttttaga ggatatatga tgatattata ataggttgaa tattagaagt atataggaga 9420
atttagttgt gttatatttt gttaagttag atgttaagga tttgtaaaaa gtgggaaata 9480
atgttttttt tattaaattt tttgttttga aaatatattg ttttaaaaaa tatgtgttaa 9540
tatgtagtga gtttattgtt atttttaaat tataaattaa tatttttaga attttttagt 9600
tttaattttt gatgtggtta gtattgatag tatataaata aaaacgtttt ttgggagttt 9660
aaatttttaa gagtgtagag gattttgttt gagaattgcg gtgttatatt gtttttattt 9720
attatttagt aagttttatt agtggttagt tatagttaat tttgttagaa tattttgttt 9780
ttatttaaaa atttatatat tagtattttt tagtttgtaa atatttaaga agtattagaa 9840
ggataatatg ttatttagat aaatcgtaga tattatgagg aggtgtttta aatttagtta 9900

taaaatagtt tataatgttt atagattgtg taggttaaaa atttgtatag ggtataagaa 9960
aatggtttgt ttttgttttt tgtttttgtt tagtattggt attagtagta ggtatttgtt 10020
attaataaat tgtgattttt agtgtttgtt ttagttttaa ttttttaggg tattgttttt 10080
tgattttatt tttttgatgg atgtaagaag agttgatgat ttttcgtttg tttaatttat 10140
ttttttttat gaggatagga atgatgattt ttaagttttt tatgtgttgg attagaaatt 10200
agaagtttag attagtgttt ttttaagtgt tatttttgta tttattagtg taatattaag 10260
ttgtttatag gttatttgat aattatagta ataaatattt tggataaagt aataaaattt 10320
ttgttaatat attttataaa ttttgtatat gagattaata atggtgattt tttggaaatt 10380
atttgtattt ttagtgatta ttgcgtatat gttttaaata tgaagtagtg attattgtga 10440
attagtatgg gttttttaaa aatgagatat attaaatttt tggtaaaagt tttgatatat 10500
tataaggaaa tagtataaaa tattgataag atttttaaat ttggatttag aagattaatt 10560
tatgattttt agtatattat taataaaaag ttggaaatta aggatttaga aagagattag 10620
agttagagat atagattttg ttatgttaat attattttat tttttttatt gggaggagag 10680
attttttgag gttgggattt attttttttt tatttttgaa tttttttgag ttttttatag 10740
tgtttttaat ataggaaaag gtgtaaattt aagaagggat ttaagagttt gtttttagat 10800
atgttgatgt tttggtagta tatttaggta gaaatgttta agttaataga tataaatatt 10860
ttattagaat ataggattgg agatggagat tttgttatat tatttttata atagtatagt 10920
aggatttttg gaagattgta tagtatttgt ttatttaatt tttttgaata aatgaaatgt 10980
gtttatttta ttataagtat ttttatattg ttgagtagga aaaagtttgt ttgatagagt 11040
agtgttttta attttttttt tatagtgtaa aatttgtatg ttatatgtat tttatttatt 11100
tttataataa gaaagataga attaaaatgt tagaaaagtg ttttattttg tattaaaata 11160
gaaaagttaa taagtttaat gaaaaatttt gagatttata ttttaaatta ttttttattt 11220
taaaatatgt attttaaagt gttttttgtt tgattatagg 11260

<210> 56
<211> 11260
<212> DNA
<213> Artificial Sequence

<220>
<223> chemically treated genomic DNA (Homo sapiens)

<400> 56

tttataatta aatagaagat attttagagt atatatttta aaataaagaa taatttaaaa 60
tataaatttt aagatttttt attaagttta ttaatttttt tattttaata taaaataaaa 120
tatttttttg atattttaat tttatttttt ttattatgag agtagataaa atatatgtaa 180
tatgtagatt ttgtattata ggaaaaaggt taggaatatt gttttgttag gtaaattttt 240
ttttgtttaa tagtgtaaaa gtatttatgg tgaaatgaat atattttatt tatttaagaa 300
aattaaatga gtaaatatta tgtagttttt taaggatttt attgtattgt tatgaaaata 360
atgtggtaaa atttttattt ttagttttat attttagtgg aatatttgta tttattggtt 420
taaatatttt tatttaaata tgttattaaa atattaatat gtttaaaaat aaatttttaa 480
attttttttt aaatttatat ttttttttat attggaagta ttatagaaga tttagagaaa 540
tttaaaaata aaagagaaat ggattttagt tttagggaat tttttttttt agtaaaggag 600
ataaggtagt attaatataa taaaatttgt atttttaatt ttgatttttt tttaaatttt 660
taatttttaa ttttttgtta gtggtatatt gaggattata agttaatttt ttgagtttaa 720
gtttaaaagt tttgttaata ttttatgttg tttttttgta atgtattaaa atttttgtta 780
aagatttgat atattttatt tttaggaaat ttatgttggt ttatagtagt tattgtttta 840
tgtttaaagt atgtacgtaa tgattattga gaatataaat ggtttttagg aagttattat 900
tattaatttt atatataaaa tttgtgaaat gtgttaataa aagttttatt gttttattta 960
aaatatttat tgttataatt gttaaatagt ttatggataa tttgatattg tattggtagg 1020
tgtaaggatg atatttgggg aaatattggt ttagattttt ggtttttagt ttaatatata 1080
aagaatttga aagttattat ttttgttttt ataaagagaa ataagttaaa taaacggaaa 1140
attattaatt ttttttatat ttattagaga aatgaggtta aagggtagtg ttttgaaaag 1200
ttaagattga ggtagatatt gagaattata gtttattaat agtaaatgtt tgttgttggt 1260
gttagtatta ggtagagata aaaagtagag ataagttatt tttttgtatt ttgtataaat 1320
ttttgattta tataatttgt gagtattata agttatttta taattaaatt tggaatattt 1380
ttttatgata tttgcgattt atttaaataa tatattattt ttttagtgtt ttttaaatgt 1440
ttataaatta aggaatattg atatataaat ttttaagtaa gagtaaagta ttttaataag 1500
attggttgta attgattatt agtaaaattt attaaataat gagtgagaat agtatggtat 1560
cgtagttttt aaataggatt ttttatattt ttaaaaattt aggtttttaa agaacgtttt 1620
tgtttatgta ttattaatat tgattatatt agaaattaaa attgagaaat tttaaaaata 1680
ttaatttatg atttaaaaat aataatgaat ttattgtatg ttaatatata ttttttaaaa 1740
taatatattt ttaaaataaa aaatttagtg agaaaggtat tattttttat tttttgtaaa 1800
tttttaatat ttggtttaat agagtataat atagttgaat ttttttatgt gtttttggta 1860
tttaatttgt tgtgatatta ttatgtattt tttagaaaat ggtattaaat ttttgtgaga 1920

taatgagaat tttaaaaagg taaatatttt agtattatta taaaaatgtt tttgatttta 1980
tagatcgttt ttatattatg agaatttttg gtataggtat attttatttt attgtgttta 2040
gttttattgt attgtgtaga tgttagtttt aaaaatatac gtaaatttta gggagaaagt 2100
agatgttgta gatttttttc ggatattttt tattagatta agaaagtttt ttttaatttt 2160
tagttttttg cgagttttaa tgatgagtag atattgagtt ttattaaatg ttttttttgt 2220
atttatttag atattatttt ttttttttat tgttttaatg tggtatatta tattgaatgt 2280
tgaataaata aattagaatt ttttaaataa attttttttt ttgttcgtat tttaagatgt 2340
ttataattta tgttataaga aataattttg ttaggtattt aagtttagtg gttttataag 2400
aattgggtta tgttaatttt tttttatttt ttagaaaaat ttgtataaga taggtattaa 2460
ttttttttta aatgttggaa gaatttatta gtgaagttat ttgagtttgg aggttttttt 2520
tttgggtggt tttaagtaat gaatttagta tttttaaata gatataaatt aaaattatta 2580
tattagtttt gttttttagt taggtgtggt ggtttatgtt tgtaatttta gtattttggg 2640
aggttgaggt aggtggatta tttaaggtta gaagttcgag ataagtttgg ttaatatggt 2700
gaaattttgt ttttattaaa aattaaaaaa taagttgggt atggtggtat atatttgtag 2760
ttttagttat ttgagagagg ttgaggtatg agaattattt gaatttgggg aggtagaggt 2820
tgtagtgagt tgagattgta ttattgtatt ttagtttggg taatagagtg agattttgtt 2880
ttaaaaaaaa aaaaaataaa taaataaaat aaattttagt gtttttatgt atgtgtagaa 2940
attgaaaaat ttgtaataaa atgtaaaaga taatgaataa ataaggtagt tatgaagaat 3000
tgtaattaag ttagaggatt tttattatta gatattatga tttattataa aattataata 3060
attaagatat tgtggtatta gtttaatgat aaatatataa agaaatggaa aagaaggagt 3120
atagaaatgg aattatatat aaggttattt gatttgtata aatgtttaat tgtagttaag 3180
tgggaaaagg atggtttttt taataattgg ttttagaaat tttatatagt aagagtattt 3240
gatttaatta ataataatta tgtaatattt ttttgataat agtagtttat tagtgtttta 3300
gattaatgtt ttttaaagta tggtagttat tatggaaaaa agatttttag ttaaataaat 3360
ttgggaaatg ttgaatgtgt aggttttatt ttataggatt tttcgtgatt ttaatgttat 3420
gttaattaag gatttataat tttaaaaaat gttttatgaa ttttatttta tattatatta 3480
aaaattaagt tatagtagat tatagattta aaagtgaaag gtaaagtata agtttttaga 3540
agaaaaatag gaaagaattt ttatgatttt ggagtaggta aaggttaaat aggatataaa 3600
aggtttataa aagaaaagat tgataaaata gattttattt tatatagtgt attttatttt 3660
gtttaaatta tattttaata aaaagtaaaa aaggagtaaa tagttattta atttaatatt 3720
aggaaaaaga taaaaattat ataggtaatt atatttagag atagtgtgtt ttaggaaatt 3780
ttatagaagt tttgagaagg atataaatta taaaaaataa ttaaaaataa aagaatatgt 3840
atagttagtt ttttgtattt atgagttttg tatttatgga tttaattaat tttggattaa 3900
aaatatgtga gaaaaaattg tatttgtatt aaatatgtat agattttttt ttttgttatt 3960
attatttaaa taatatagtg taataattat ttatatagta tatgtattgt agtaggtatt 4020
aaaagtaatt tagagatgat ttaaagtata cgggaggatg tgtataggtg atatataaat 4080
attatacgat tttatataag taattggagt atttttaaat tttggtattt ttaggagatt 4140
ttggatttaa ttatttatag atattgagga ataattgtat atttaaaaaa aacgattgat 4200
ttttatagga ttttaagtag aagtggagta agagattgaa agaaaagttt ggtatatagt 4260
aaattagtaa gggtttgata gataagttat ataatttaat aattaagaaa atgtatttat 4320
ttgaaaattg gtttaaaaaa aaaaaaaaaa gaaagaaaga aaaaagatta gtgtgtttaa 4380
tttgtttttt gtgtataaaa tgtattttta gtaattaaag agttgatgaa ggaaaagttt 4440
tttatagaat tttaataagt gtagaagaat aaaataatta gaaaattgtt attttagttg 4500
ggtgtagtgg tttatgatta taattttagt attttgggag gttgaggtgg gaggatggtt 4560
tgagtttagg agtttaagat tagtttggga aagacggcgt gattttattt ttataaataa 4620
tttaaaatag ttatttgtgg tagtgtcgtg gtagcgttat ggtggtgtat atttgtagtt 4680
ttagttattt tggaggttga ggtaggagga ttttttgagt ttaggaggtt taggttgtag 4740
taatttgtga ttgtgttatt gtaatttagt ttgggtgata aaataagatt ttattttttt 4800
aaaaaaaaaa aaaaaaaaaa aaaaaggatt attttggtta atattttcga aattttattt 4860
ttattaaaaa tataaaaaat taattaggta tggagaaggg ggaggaggag gaataagtgg 4920
aagaggaaga atattattat tttaattttt aatgaaataa tggatttggg taattattat 4980
taatgtttga tttttaaaat tattaggaga aaagttgata ggaattttat aatggttgga 5040
ttaggttgat agtatttgaa tttatagatt agttttaaat taatattaaa ggttgttaag 5100
ggtggggata gggaatgtta aaagtcgaaa gtgaatttaa ttaagttttt ttagttttaa 5160
ttataaggaa atttaggaga tagaaaaaga agttaaataa tattattagg aggtaattat 5220
ttaaatttaa tatagatatt tttaatttac gatgggttta ttgggatata attttattat 5280
gaattgagta gtatattgat tgtattattt ttttattatc gtaaagttaa aaaatattaa 5340
gatgaattat tgtaagttag aataaatgat ttggtttttt taataagtaa atgttatgga 5400
gatgaaaaga agagtaaagg aagagggttg ttgtttttta ttagaagaaa tttaataaat 5460
atataattaa attaaggatt tttttggatt ttgatttgaa taaaataaat gtaaaaagat 5520
atttttgagt taggattatt tttttatttt taaatttcga tttaaaagtt tttaattaat 5580
tattttattt attaatttgt ttttttttta gaaattttat atagtaaata tatttgattt 5640
aattaataat tttgtaatat tgtttttttt ttaaatagta gtttgttaat tttttaatag 5700
taatgttttt taaaatattt taacggggat gttagtagat attaaagaaa aaaaggtgtg 5760
gttaaataaa ttcgggaaat gttaaaatat attttttttt ttttttttgt tttttttttt 5820
tttttttggt aggatttttt atagttttta atatgttaat gtaaaatgat taggggagta 5880

tttagagtat ggtatttttg ttaaagttat ttgttatttg aatatttttt tatagaatat 5940
tttatgggat tgatagaatt tattttagag gaaattaaaa ttttaaaata tgtttatgtt 6000
tgttatagtt aagtgtattt agatgttttt aatttgatta tttaaaaata aaataggagt 6060
attagttaaa ttatatatta agtaagtgat ttgttttgta tggaaaaaaa tattataaat 6120
atttatttaa tagaaataag ttataaagat ttttataatt taaaatgtaa aaaatggtta 6180
aaaatattaa atattgtata tatatttggt agaatttata tgtaatttga attttataag 6240
ttgaataatt tattatattt ttttttttat ggtaagaata ttgaaaaata aagtggtttt 6300
atattgttaa aaatagtttt gaaatatttt ttgaaatagt agaaaataaa gtgtttaaat 6360
tattttttgt aatattattg ttattttaaa attagttatt attaataatt tttataatag 6420
agtattgttt ttgaaatatt aatatattta tataaatatt ttaaatatag gtattaatat 6480
atttaatttt attaaaggga ttaaaagtgg aagaaatatt taatttagtt tttagggttg 6540
tgattgaagg gtagttatta aaagagtaaa aaaattttta ttttgaatat tgatgagaat 6600
gatgtaattt agtaatttta tatttttttt tagaaagttt ttaattttaa aaaatgtatt 6660
tattgatatt tagtttttta gtttaattta ttaaaattta tttgttttat aatgtagttg 6720
atttatataa atagttttaa ttgtattagt ttaaggataa gtatagtata ttgaaattat 6780
ttagattgat gatttatttt taaaatttta ttaatggttt tattttatgg ttattgaaat 6840
tttatttagt ggtaaataag agtaattatg tggttattta aataagttaa tggtgtatag 6900
aggaaaattg tattttttat aattttgttt ataaatgaag ttttaagaat gttatgttta 6960
gaaaaatttg gtaatttttg tggggaaatg tgtaattagt taaagtttag agttttggag 7020
taaaggtgga tttggtatag gaaataagaa gagagaaagt tttttttttt gttttatgta 7080
aaatttttat ttgagagggt tttatttttt tatttatatt taattttatt atgttgtttg 7140
cgaggagagg aaaaagaata ttagaaaaat ttaaggaaat ttgtaatagt tatgtttttg 7200
ttattataag tagaatatga gttttatgag ggtaaggaat ttatttttgg gttaatattt 7260
ataatagcgt ttagtttagt ttagtataag gaatatttaa atattaatta tatatagata 7320
taaagatgaa gaaaattttt tttgtatttg agaaattttt agtttagagg aaagaggaat 7380
atggaagtat aatttttaat taatttggta ggaataagag ttaaaatatt atgagagtag 7440
agaggtagtg gagggattaa tttgatagtt ttagaaaaat ttttagaggg agttagtttt 7500
tggtattttt ttttttaatt ttttttattt tttattatat tatttttttt ttttataaaa 7560
taagtttttt atttatatag tagtttattt tttagttaat aaataaaaat tttatttttt 7620
tattattatt ttttaatatt tagttaagag agttataaat attaatagtt taagtatttt 7680
gagatagaaa cgataataat tttgggaggt ttaaatgtta tttttttgtt aatttaattg 7740
aatttaaagt ttataaattt aggtttattt tttttttatt gtagtgttgt aaatatgttt 7800
ataatagaag tttggaaggg aggttttgtt gagttataga ttgttggaga agttaatgat 7860
tgtatataga taattgttaa tatattatat tattttttgg taagtaatta taatttatat 7920
tggtaggaaa attaattgtt atgaaaagta ttaggagagt tttaggtaaa attttttaga 7980
agtcggtttt tttgattttt tttaattttt tttttaatat ttttttattt tttttgtttt 8040
tgagttttta ttttgtattt ggtatgagta ttttttgtta gatgttgggg atgtattggt 8100
aaattaagat agatatttgt ttttatggag tttttaagat agtgatgggg tttatattaa 8160
ttaattaagt atttaattta gagttttttt ttttataatt ttaaattaag aattaaaatt 8220
attattgaat attataaata taaatttgat tatttattta gtaaatttta tagatttaat 8280
tttttaggta tattttgaga gatataaatt tagttattat ttatatttaa tttgattttt 8340
taaatgttat ttttaatata ttatggttta tttaatagta ttagattgtt tgttattttt 8400
tgaaaattat ttttagtttg tatattattt tagttttttt taaaatatat ttttttaaat 8460
gaatgtttgt tttttttgat attatattgt taaggtttat tttaaataat attttttttg 8520
tgatattttt tttataattt agattgaaaa ttttttttat aagttatgtt gtttatgttt 8580
tgtttatagt aattatattg tgttttgtaa tgttatttat tatataattt gtgtgttttt 8640
tttattttat tttattgttt attatttttt gaagaaatat aatatagtta aaggaatatg 8700
agtttttgag ttagaattgg ttttaaaatt tggttttgtt attcgtaagt tatataatat 8760
tgtgtaaggt tttaagtaat tttgtatttt agttttatta attataatat aagggtagta 8820
ttatttattt tatagttgtt gtaagtattt taaaatagta tatgtaaagt atatagtata 8880
atgttcggta tatgagtatt ggataaaatg gtagttgtag ttttattatt tttattgtta 8940
ttaaaattat aaggttgttt ttatttatcg ttgtatttgt atagtatttt atttgttaat 9000
agtaggtata gagtaattgt taaattgtta aaagaaatta tattagttgg atatatttta 9060
aaaaattaaa aaaattgtaa tttgtaaaat gggtaagttt atatttattt tatatggaaa 9120
taataatttt gtattttgga gaagttatta atttttacgt tttaaggttt taaagaaata 9180
gatagttagg cgtagtggtt tacgtttgta atgttagtat tttgggaggt cgaggcgggt 9240
aaattatttg aggttaggag tttaagatta gtttggttaa tatggtgaaa tttcgttttt 9300
attaataata taaaaattag tcgggtgtgg tggtttatgt ttgtaaattt tagttattcg 9360
ggaggttgag gtaggagaat tgtttaaatt cgggaggtcg aggttgtagt gagttgagat 9420
cgtgttattg tgttgtaata gagtgagatt ttattttaaa aaaaaaaaaa aaagaaaaga 9480
aagaaagaaa tagattatat taaatattat tattttttta attaggaaaa gtgtaaatag 9540
atgattttta ttaaagagat agattagtaa atttgggtaa tttaaagttt aaatattatg 9600
aaaaatttaa aatatgattt ttttattttt tagtaaattg taaatatttt tattttaatt 9660
ttttaatatt ggtgaaggta ttttaagtaa attgttgaaa tatatttatt tatgaatata 9720
aagtaaaatg aaggttaaag taaaatgttt aaaaagttaa gttaaaatta aagtgttata 9780
ttaatgtgtg taaaaagtag aataatataa tttttttttt gagaaaaaaa taataaaata 9840

tattaggaaa ttaggaataa atttaagtaa taaataattt ttaattttat agaggtaatt 9900
atgaagtagt tattgtatag atatatataa gaaaaagtgt ttttattttg gaatgtttat 9960
aatgttttgt gggtttaaaa aaaaaattag ttaggcgaaa atgttaaaga ttaaaagttt 10020
taaattagat tgtattttgg atttgttttt tttttattaa taatttggtt taaagtgata 10080
atggtatttt taagcgaata aagagatttt tttgaaaatt attgatatta taataatgga 10140
tgaattttaa aataattatg ttaagtgaaa gtagatagtt aaagaaatta tattgtatga 10200
ttttatttat ataaaatttt agaaaatgaa aattatagcg atagaaagta gattattggt 10260
tgttgaggga taggagtaga aggagtggga gggaaggatt ataaaggggt atgagaaaat 10320
ttttgagggt gataaattta ttattttgat tgtggtgatg gttttatggg tgtatatata 10380
taagttaaaa cgtattaagt tgtatatttt gaatatgtat aatttattat aagttaatta 10440
tattttaaaa agttgttttt aaaaggatat tttgaattgt ttgaaaaata ataaattttg 10500
tttaaggtgt aatggtttat atttgtaatg atagtatttt ggggtgtcga ggtagaagga 10560
ttagaggtta ggagtttaag attattttgg ggaatataag gttttgtttt tatataaaaa 10620
aaaaaaaaag taatttaaaa attagttagg tatggtggta tgtgcgtgta gttttagttg 10680
tttaggaggt tgaggtagga gaattttttg agtttaggat tttgagattg tagtgaatta 10740
tgtttttgtt aatatatttt agtttggggg atagagtaag attttatttt aggaaaaaaa 10800
aataaaaaaa aaaaaaaaaa aaaattatat atatatatat atatatgtat atatatatgt 10860
gtgtgtatat atatgtatat atatatatat atataaatat ttttaaaata ttaattttaa 10920
ggtttgtgaa ataatttttt tgttatagtt gtatgaagtt aaagattagg gttaatgatt 10980
atttgattat ttataaatgg tatttgttaa ttatttaagt tagtgaaatt tattttagtt 11040
tttttttttt tttttatttt attatgggtt ggatttttta ttttatattt tataaaaatt 11100
tagtataatt tttaggtttg aaatggtaat tttttttttg ttttttattg gtaggggttt 11160
aataaagtgt tttcggtatt ttatttaaat taaatataat atgaagtttt atttttaata 11220
tttaaaaatt ttatttaggt atgtggtttt ggtagaatta 11260

<210> 57
<211> 5387
<212> DNA
<213> Artificial Sequence

<220>
<223> chemically treated genomic DNA (Homo sapiens)

<400> 57

ttttaaagag tttggtatgg aaggaagttt aggggttttg gaagttatta tttaggaatt 60
gggagagttt taggtaaatt attttatttt tttgggtttt agtatttttt atttatattt 120
ttatggttag tagatgttaa gtatatggtg gatatataga agtattgatt atttttttta 180
ttttttttag gagagttttt gggaattttt atataatatt ttttgaagat tttaatttaa 240
tgagtcgagt aattgattta gtagtaaatt tatagaaagg atattagaag ttaaaaggag 300
gtagagtatg tttagtataa taattagttt ttgattttaa ggaagaaaga atagagttgt 360
aggtgagaag tgtgcgtttt aaattattaa agtgagagtg gaaggagtat ttattagttt 420
ttcggaggcg atttttgaga tcggtgagaa tggtttcgaa aaatgtgatc gtttttaatt 480
ttatagtttt gaatttaata ggagattttg aagtttgaaa gatattattt taggattaat 540
agtagatttg tgatttttta tagtaggtat atagaatttt agagttagtc gaggttggat 600
ataggaaggg tttttaaata tatagataaa tttatggatt ttaaattata aagggaggtt 660
ttttaaaaga agtaagatga ttgtgagatt attattagtt tttttttttt ttttgatata 720
gagttttatt ttgttattta ggttggagtg tagtggtatg atttcggttc gttataattt 780
ttatttttcg ggtttaagta attttttgtt atagcgattt gatgagtagt tcggattata 840
tgcgtttgtt attacgcgta gttaattttt gtatttttag tagagacggg attttattat 900
tttgattagg tttggtttta aatttttgat tttaatgatt tatttatttt ggttttttaa 960
agtggtggga ttataggtgt gagttaacgt attcgattta tttagagttt tttgaggttg 1020
atgttagagg tataaagttt agtttttttt tattggttat gtggaggttt ttgttgggta 1080
gttttttagt ttgtagtagc gagggatttg gtttaagtga tgttttaata gtgggtaatt 1140
ttttagtatt gttaggagaa ttttaagttt aatttaaagt tgatttttta ttagtaatga 1200
atgatagata tggtttttat tgtttaaggg aagatttaga ttagagaaaa ggattattaa 1260
tttttattta tatttgtggg ttttagtttt tttttttggt ttatggttgt tatggtaatt 1320
ttttataaag gtgttttttt aggggttttt aacgtttttt gagttattat aattttgggg 1380
ttaatagaag tggagaggtg agaggagata tttttttttt ttggttggtt ttatatgttt 1440
ttggtagtaa tttcggtatt tttgtgttta tttgtggagt tatgtagtag tttttagtta 1500
gagtttttgt tttttttatt tttaagggag gtttttttta cgaatggtta aaaatttata 1560
tttaagtttt tagaattggt taaaaggttt tgttatgggg gttttttgtt ttagttgata 1620
ttggaggaag gatggtgagg gaggtaggga ttttttttta tttggagttt agggatttat 1680
ttttattttt atttggtttt ttttttttgt tggtgttggg aggagtaggg gaaagtggtg 1740
atattatttt tttttgattt tatatttttt agagttggta ttattgatga tatttttttt 1800
ggatttttat ataggttttt ttggagaata gtttatagta tagtgttttt ttagtaggtg 1860

atgagtttgt ggtgttggtt cggtaatgtt ttaggaatga tggtaaaaaa aggagttttg 1920
tttttcgttt tgaaagtggg gagatggtag ggttttagta tttatatttt aggttatagg 1980
ggtgtgggtt tttaatgttg gttgttaata ttatagttaa ttatttttag aagtatttgt 2040
ttgtttgttt gtttgtgttt ttatagcgtt aagtgttata tgtttttaaa tttagggtgg 2100
gagaagattt tattattata gagggtgttt ttttttgttt ttatttttgg gggtatttga 2160
tgtttgtgat ttttaggtgg tatggaaata gttttaattt tgggtattag gagataggag 2220
gtttagtttt ggtttttttt gtttttattt tgttttgtta cgaggttttt ataaggtatt 2280
ttatgttatt gggttatttt tatttgggaa ttgagaaaga ataatttgtt ttttatggaa 2340
agataaaatt aggtaaaggg ttttttgtgt tgggggtagg atttttgtat ttagagagat 2400
ttagaagttg aaataattgg gttgggggcg gtgatttacg ttagtaattt tagtattttg 2460
ggaggttagg gagggtggat tatttaaagt taagagttcg agattagttt ggttaatata 2520
gtgaaatttc gtttttatta aaaaagtata aaatttagtt cggtatagtg gtgggtattt 2580
gtaattttag ttatttggga ggttgaggcg ggagaattat tggaatttag gaggtggagg 2640
ttgtagtgag gtaagattat gttattgtat tttagtttgg gagatagaat gagattttat 2700
taaaaataag aaagaaagaa agaaggaagg aaggaaagaa ggaaggaagg aaggaagaaa 2760
ggaaggaagg aaagaaagaa agaaagaaaa agaaagaaag aaggaaggaa ggaaagaagg 2820
aaggaaggaa gaaaggaagg aaggaaagaa agaaagaaag aaaaagaaag aaagaaagag 2880
aaagaaaaag gaaagtaaga aagaaaagaa agaaagaaag aaaaagaaag aaggaaagaa 2940
aagaaattaa aataattaaa taattgagta gtattatatt atttgttttg gagaaaggat 3000
ttttgttgtt gttgttgttg ttgtcgttgt tgtggttgtt gttgaaatgg agttttattt 3060
tggtatttag gttggagtgt tatggtttaa ttttggttta cggtaatttt tgcgttttgg 3120
gtttaagcga ttttattttt ttatttttcg gagtaattgg gattgtaggt ttttgtcgat 3180
tatatatagt tatttttgta tttttaagag atggagtttt gttatgttgg ttagtttggt 3240
tttgaattga ttttaagtga tttatttatt ttagtttttt aaagtgttgg gattataggt 3300
atgagttatc gtgttcggtt tggagaaagg atttaaaatg atgtaatgtg ggaagaggta 3360
aggttgtgga gatttgttgt tttggttgag gtagtttatg taattagttt ttttgagttt 3420
tagttttttg atttgtgaaa tgggatgata gttatagttt ttatataaga taagtggtag 3480
gttagacgtg agaatgtata ggtaggtttt tgataattgg ataagtttta tataggtttg 3540
gaaaggagga agagataaaa gaggacgggt ttttatggtt ggatagggtt tttttcggta 3600
tgatgtgttt tgagttttaa agttgggaag agatttaaat tttttttttt tttttttttt 3660
ttttaattat tattattttt ttattttaag ttttagggta tatgtgtata atgtgtaggt 3720
tagttatatg tgtatatttg tgttatgttg gtgtgttgta tttattaatt tgttatttag 3780
tattaggtat attttttaaa gttatttttt tttttttttt tattttataa tagtttttag 3840
agtatgatgt ttttttgaga tttaaatatt taagagtttt tttaattttg agattttttg 3900
atggttttag ggttgtggga ggaagcgttt gtggttcgtg tttgttttcg ggatttttgt 3960
tttttggttt gtgtttttgt tataggttta aggagttgag gtaatatttt gagtttgggt 4020
ttttcgtttt tagtgatttg ggggagtttt atcgttttcg ttttaaggtt tagggaaagg 4080
ggagagtaat gtgtggggtt ggtttttttt agtggttagt gttagtattg tatttagttg 4140
atttaggtgg gtttaggagt tattagtaaa agtggaattt aggatttaat ggtgtttaga 4200
atttttataa tttattggtt gtgtttggtt ttttttttta atatatatat tttgtttggt 4260
gggtggaagt taaatacgcg gggaggagga aaggaatagg atagagagtg gaatggggtc 4320
ggtaggggtt ttaaggattg gttattttga tagttttttt cgcgtttagg ttgattaata 4380
tggtttgtag ttagagggta tttatttgat ttttaaagag aggataagtt gggtggagtt 4440
tgtggttgat attttgtgta taatttttat aatattggtg atggtgagaa gggaaagacg 4500
ataagttagg gggtatgatt ttagtatgtg tgggaggagt ttttaaatta tttattagta 4560
taagttcgtt agtggtttta tgtataaatg tatatagaaa taggtggggt taagtaggga 4620
gagagaattg gttagggtat aaaaagggtt tataagagat cggttttagg attttaaggt 4680
ttaatttttc gaattattta gggttttgtg gatagtttat ttagcggtaa tggttgtagg 4740
taagcgtttt taaaattttt tgggtataac gtgttttgag gggagaggta gcgttttgta 4800
gatgggacgg gggtattaat ttttaggttt ggggtttatg aatgtgagta tcgttattta 4860
aggttagata tttggttaat ttttgaatgt ttttggtttt tggagggatg gagagagaga 4920
aaaaaataaa tagtttttgg agtagggaga gcgttggttt tttttttttc ggtttttttt 4980
attgtttttc ggtttttttt taggttttcg gacgtttttg tttttggttt ttgttttgtt 5040
ttgtttgttt tggttttaag aggttggtgt cgtttaaatc gtttcgttat ttaggttttt 5100
tgattacgtt atgttttaag tttatcgcgc gtattagttg gttattgata tttattagga 5160
gtttgtaagt ttttggggaa tgggtgcggg ttaggggtgg taagaagggg tgattttttt 5220
attggggaag taatgggagg agattaagga gtttagggtt gttttttgaa gcgaaaatgt 5280
aggtagatga gtataggttg agttaggttt ttagaaaagt aataatggga gttggttttt 5340
agtatagaaa ttagtagttt tttttggtgg ggggtttttt ttttagg 5387

<210> 58
<211> 5387
<212> DNA
<213> Artificial Sequence

<220>

<223> chemically treated genomic DNA (Homo sapiens)

<400> 58

tttaggagaa ggatttttta ttaagaagga ttgttggttt ttatgttgga gattagtttt 60
tattgttgtt tttttgggaa tttggtttag tttatgttta tttgtttgta ttttcgtttt 120
agaaaataat tttgagtttt ttagtttttt tttattattt ttttagtggg gaagttattt 180
ttttttgtta tttttgattc gtatttattt tttaagaatt tataaatttt tggtaggtgt 240
taatggttag ttggtgcgcg cgatgggttt ggagtatagc gtggttaaaa agtttggata 300
acggaacggt ttggacggta ttagtttttt gaagttaggg taggtagagt agggtaaaag 360
ttaggagtag ggacgttcgg gagtttgggg agaaatcgga gggtaatgga gggagtcgga 420
gaggaagagg ttagcgtttt ttttgtttta ggagttgttt gttttttttt ttttttttat 480
ttttttagag attaggaata tttagagatt ggttaaatat ttggttttag atggcgatgt 540
ttatatttat aagttttaaa tttgagggtt agtgttttcg ttttatttat agggcgttgt 600
tttttttttt aggatacgtt gtgtttaaag gattttaggg gcgtttattt gtagttattg 660
tcgttaggtg agttgtttat aggattttga gtggttcggg gagttgggtt ttgggatttt 720
agagtcggtt ttttgtgggt tttttttata ttttggttag tttttttttt ttgtttgatt 780
ttatttgttt ttgtgtatat ttatgtatgg ggttattgac gggtttgtgt taatggataa 840
tttggaagtt ttttttatat atgttgggat tatgtttttt ggtttgtcgt tttttttttt 900
ttattattat tagtgttgta aggattgtgt atagagtgtt agttatagat tttatttaat 960
ttgttttttt tttgagggtt aggtgggtgt tttttggttg taggttatgt tggttaattt 1020
gaacgcgggg aaggttgtta ggatagttag tttttgagat ttttatcgat tttattttat 1080
tttttatttt attttttttt tttttttcgc gtgtttaatt tttatttatt agatagaatg 1140
tgtgtgttgg gaaaaggggt taagtatagt taatagattg tgggggtttt gagtattatt 1200
gagttttgaa tttttatttt gttgatgatt tttgggttta tttgagttag ttggatgtag 1260
tgttaatatt gattattaga gagaattaat tttatatatt gttttttttt ttttttgaat 1320
tttggggcgg aggcggtggg gtttttttag gttattgggg acgaagaatt tagatttaag 1380
gtattgtttt agttttttgg atttgtagta gagatataaa ttaagaaata gaaatttcgg 1440
gaatagatac ggattataag cgtttttttt tatagttttg aaattattag agaattttaa 1500
ggttggaaga gtttttgaat atttgagttt taggggaata ttatattttg gagattgttg 1560
tggggtgggg ggagggggag ggatagtttt aggagatata tttaatgtta aatgataagt 1620
taatgggtgt agtatattag tatggtatag gtatatatat gtaattaatt tgtatattgt 1680
gtatatgtat tttaaaattt aaagtaaaag aataataata attaaaaaaa aagaaaaaaa 1740
aaagaagatt tgagtttttt tttagttttg gaatttagaa tatattatgt cgaaagaggt 1800
tttatttagt tatggaagtt cgtttttttt tgtttttttt ttttttttag atttgtgtag 1860
agtttattta gttgttaaag gtttgtttgt gtatttttac gtttgatttg ttatttattt 1920
tgtgtaggag ttatgattat tattttattt tatagattag gagattgagg tttagagaga 1980
ttgattgtat gagttatttt agttagggta gtagattttt ataattttgt ttttttttat 2040
attgtattat ttaaagtttt ttttttaggt cgggtacggt ggtttatgtt tgtaatttta 2100
gtattttgga agattgaggt gggtggatta tttgaggtta gtttaaggtt agattagtta 2160
atatggtaaa attttatttt ttaaaagtat aaaagtagtt gtgtgtggtc ggtagaagtt 2220
tgtaatttta gttatttcgg agggtgagga ggtagaatcg tttgaattta ggacgtagag 2280
gttgtcgtga gttaagattg ggttatggta ttttagtttg ggtgttagag tgagatttta 2340
ttttaataat aattataata acgataataa taataataat aataaaagtt ttttttttag 2400
agtaggtggt gtggtgttat ttagttattt agttatttta gttttttttt tttttttttt 2460
tttttttttt tttttttttt tttttttttt tgtttttttt tttttttttt tttttttttt 2520
tttttttttt tttttttttt tttttttttt tttttttttt tttttttttt tttttttttt 2580
tttttttttt tttttttttt tttttttttt tttttttttt tttttttttt tttttttttt 2640
tttttttttt tttttttttt tttttttttt tttttttttt gtttttgatg gagttttatt 2700
ttgtttttta ggttggagtg tagtggtatg attttgtttt attgtaattt ttattttttg 2760
ggttttagtg attttttcgt tttagttttt taagtagttg ggattatagg tgtttattat 2820
tatgtcgggt taagttttgt atttttttag tagagacggg gttttattat gttggttagg 2880
ttggtttcga atttttgatt ttaggtgatt tatttttttt ggttttttaa agtattgaga 2940
ttattggcgt gagttatcgt ttttaattta gttattttag tttttaaatt tttttgagtg 3000
tagggatttt gtttttaata taaggggttt tttgtttgat tttgtttttt tataaggggt 3060
agattgtttt tttttaattt ttaggtgaga atggtttagt ggtatggggt gttttgtgga 3120
aatttcgtag taggataagg tgaaggtaga gaaggttagg attgagtttt ttgttttttg 3180
gtgtttaggg ttggggttat ttttatatta tttggaggtt ataagtatta gatgttttta 3240
gagatgagag taggaaagaa tattttttgt agtggtgaaa ttttttttta ttttgagttt 3300
aagagtatgt agtatttgac gttgtagaaa tataagtaag taaataggta aatgttttta 3360
gaagtggttg gttgtggtgt tggtaattaa tattgaaaat ttatattttt gtggtttagg 3420
atgtgaatgt tggggttttg ttattttttt atttttagag cggaagatag agtttttttt 3480
tttgttatta tttttgaagt attatcggat tagtattata gatttattat ttgttgggag 3540
ggtattgtgt tgtgggttgt tttttaggga agtttgtgtg aggatttaag aggggtgtta 3600
ttaatgatat tagttttgag aaatatgggg ttaaggaggg gtggtgttat tatttttttt 3660
tgtttttttt agtattagta aagaggagga gttaggtggg aatggaagtg ggtttttgag 3720

ttttaagtag aagagggttt ttgttttttt tattattttt tttttaatgt tagttgggat 3780
agggagtttt tatgatagga ttttttagtt agttttgggg atttagatgt ggatttttgg 3840
ttattcgtaa gggaagtttt ttttaggaat gaggggggta ggggttttag ttggggatta 3900
ttgtataatt ttataggtgg gtataggggt gtcgaggtta ttgttaggaa tatgtgggat 3960
tagttaggga aggggagtgt ttttttttat ttttttattt ttgttgattt tagagttata 4020
gtgatttaga ggacgttagg gatttttggg gaaatatttt tataaggggt tattatggta 4080
attatggatt agagggaaga attgaggttt ataggtgtgg gtagaagttg gtgatttttt 4140
tttttagttt agattttttt ttgagtaatg gagattatgt ttattattta ttgttagtaa 4200
aaaattaatt ttgaattaga tttgggattt ttttgatagt attgggaagt tgtttattgt 4260
tggggtatta tttaggttag atttttcgtt gttgtaggtt gaaagattat ttaataagag 4320
tttttatatg gttagtgggg agaggttggg ttttgtattt ttagtattag ttttaaaagg 4380
ttttggatag gtcgggtgcg ttggtttata tttgtaattt tattattttg ggaggttaag 4440
gtgggtggat tattagggtt aggagtttaa gattaaattt ggttaagatg gtgaaatttc 4500
gtttttatta aaaatataaa aattagttgc gcgtggtggt aggcgtatgt aattcgagtt 4560
atttattagg tcgttgtggt agggaattgt ttgaattcgg gaggtggagg ttgtagcgag 4620
tcgagattat gttattgtat tttagtttgg gtgatagagt aagattttgt attaagagaa 4680
aaaaaaaagt tgatgataat tttataatta ttttgttttt tttaaggaat ttttttttat 4740
aatttaggat ttatgaattt atttgtgtgt ttggaagttt tttttatgtt taatttcgat 4800
taattttagg gttttgtgtg tttgttgtgg aaagttatag atttgttgtt gattttaaag 4860
tggtgttttt taggttttaa gattttttgt taggtttaag attgtggggt tgaggacgat 4920
tatatttttc gaagttattt ttatcggttt taggaatcgt tttcgaggga ttggtgggtg 4980
ttttttttat ttttattttg gtgatttgag gcgtatattt tttatttgta gttttgtttt 5040
tttttttttg agattagagg ttggttattg tattgggtat attttatttt tttttggttt 5100
ttaatgtttt ttttatgagt ttgttattga gttaattatt cggtttattg gattgaggtt 5160
tttagagaat attatgtggg gatttttaag ggttttttta ggggaggtga ggaagatgat 5220
taatattttt gtgtgtttat tatgtgtttg gtatttattg attataggga tgtgagtgag 5280
ggatattgag gtttaaagag ataagatggt ttgtttggag tttttttagt ttttaagtaa 5340
tggtttttaa ggtttttggg ttttttttta tgttaagttt tttagaa 5387

<210> 59
<211> 6172
<212> DNA
<213> Artificial Sequence

<220>
<223> chemically treated genomic DNA (Homo sapiens)

<400> 59

attatattta gagtatttgt aatataatat ttttaaagaa aaaaaagttt ttataatatt 60
ttaaaatttt taataataga ggtttagaaa tagtttgtat tttttttatt ttagtaattt 120
ttgattgaga tagtttggtt aagtagtatt ttttgttttt taaatatcgt tttttttatt 180
aggatagaag aggttttttt tataattttt taagtttgat ttgattttat agttagattt 240
tttatttaga tttttagagg tttagttttt tttattgtat gataaagggt ggttgttttt 300
ttttttgacg attaattaga ttattgtaaa aagtttttta atttatattt aagttttttt 360
tttggttgtt agttaattgt ttaattattt tattttttta aataaaattt tttaggttgt 420
cgggaattta tatttaggat ttaggtttgt attttgggtt ttaggagttt gggggatttg 480
gtttttgttt gtttgtttgt tttttacgtt tttttgtttt ttgtttagat tcgattttgg 540
gttgattgag tttttagttt gtttggggtt gatagatgat tttattggtt ttagggggtt 600
agggtaggat tggaaatagt ttgagggtat ggaaagaatt ttggtttggg agtgggaaga 660
tttgatttta tgggggtatt gattgtggat tatcgattgt gtgtgtttgg ggaagatgtt 720
atttttttga gttttatttt ttcgttttta aaattcgggg ggtaggagta tggtttggat 780
agttaatttt agtgttaagt tttttttgat tttataaatt agtaggtgaa ataaagaggg 840
taagtcgcgt ttggttggat aaattttttt attggtgtta aatgtttttt agttttttta 900
tttaagggag gttttttaat attaggaagg gattttagtt ttttaatcgt ggattatttg 960
tttaggttat tagcggtttt tagggttatt ttgagtatta cgttttagat atttattttt 1020
gtaggtagga tcggttttta gagaggttgg atattgattg tgtagtttta ttttttgttg 1080
agggttttgt tttagttttg gttttgtgtt gaggggtcga tgtagaaata ttagtaagat 1140
atttggggga ggtagggaat taagaaggtg gggggttttt ttagggggag gtagggaatt 1200
aagaaggtgt aggggggttt ttttattttg ttattttttt tggaaatgag gttttaattt 1260
agttggtttg ttattttaga tgtagaagga gtttttgaat ttttggggat ttttagaggt 1320
ttttagtttt gtggtgtttt atagttggaa gagaatttcg aatgggtatt tgtaggttag 1380
ggaagtagta gtagtagggg tttggttttt tttagttttg gaatagtagg tttttgttaa 1440
attagaatag gggtttgtga ggttttattg tgagtgttta gttttagtga gtttttagtg 1500
ggtttatatg ttggggtttt ggggaggggg tagtggtatg taaggatagt ttgggtgtga 1560
tatttttggt attagtgata agttataggg ttttttgtta tttagtagta agggtagtgg 1620

attattgtat atttatgaaa tattagttta gaatagtttt tagagattga ggttcgtagg 1680
tttcggtttg tttgtaaatg tgatattttt gtattttttg gtttgattta agatttaaat 1740
gtttgtatta agtaagtttt ttttttgtta attatttgta aagaaaggta atagtttttt 1800
ttttgaagaa agttattttt gttatagaga gaataggagg ttttgtttgg atcggatata 1860
gtggtgggaa tttatatttt acgggatgtt ttaattatgg gagatgagtg ttaagatggg 1920
atttgaattt ttttttgtga attatataaa ttttacggaa atatagatgg gagagaaaag 1980
gaaagggaaa tagagaagta gttgaggtta tgattttatg attttaaggg tttttttttt 2040
ttatttcggg gttagttgtt aggatttttg tatttttttt tattttggtt tgtggatata 2100
tgtgattaaa tttttgtttt ttttttttag atattttgag ttttagtatt tattatttgt 2160
atgattttgg gttagttaga tagtagtttt atgtttagtt tttttattta taaaaaggaa 2220
ataataatag taattttttt tttaagttat ggtgagaata aagagttaat aataaatata 2280
aaatatttag aatagtgtat ggtttataag tattattagt tattgtggtt attatttatg 2340
tttatttgta gagatggggt tttgttatat tgtttaggtt ggtttcgaat ttttggtttt 2400
aagtgatttt tttgttttgg ttttttcgag tgttgagatt ataggcgtga gttatattat 2460
atttagttta ttacgtttat tattagtgtt atttttattt tttttttttt tttgatgtta 2520
aatatttttt taatatatat attgttaaaa gttttttaga tagtagtatt attttggggt 2580
atgttttttt tagtgagtta gtggattggt ttatgttatt agtatttagt tggagtttag 2640
gttattttta gatatagaat aagggataga aatttagaaa atagaggtta aaattgttta 2700
gaggtatggt ttgtataaag ttataggtat tgggggtaga ttgaggtggg agttttttat 2760
gtttttttaa atatatattg tagattggtt gggtttaaat atgagatttt tatatatata 2820
tatatatgta tttgtatata tatgtggtgg tatgaagttt ttgggttttt atttaaaaga 2880
aatttatttt tcgagtatta gaagattttt tttttttttg tttgaaatgt ttgaggatag 2940
aagtaggggg atgatatgga gagatttgtt agtgatatag tatttttata aaggggttag 3000
agtgttttta tttagaaggt gaagggggat gatatggaga gatttgttag tgatatagta 3060
tttttataaa ggggttagag tgtttttatt tagaaggtga agggggatgt tttttgttcg 3120
gtagtttttt tggtttaggg tttttgtttt tttttattat ttttatttat gcgatagaat 3180
atgggtaagt gaggttgtgg agttttggga agagtttttt tttgtatatt tttaaatttt 3240
ttatgttagt gttagaagga gatgtttata gagatagagg ttttatttag tttttaagtt 3300
ttttattgag gttggcgagt aggttatttt tttaggtagt ggtttgattt tgtatttgta 3360
agatgtattt gaaattgata tttgtttttt aaggttgtgg tgaatatata tttagagttt 3420
agaataatga ttggtatatg tatttaggaa atgtgtgtta ttattgttgt tgagagtttt 3480
ataaaaagtt acgatttggt tcggtacggt ggtttatgtt tgtaatttta gtattttggg 3540
aggttaaggt aggcggatta ttaggttagg ggatcgagat tattttggtt gatacggtga 3600
aattttattt ttattaaaaa tataaaaaat tagtcgggcg tggtatgcgt ttgtagtttt 3660
agttattcgg gaggtcgagg taggagaatt gtttgaattt aggaggtaga ggttgtagtg 3720
agtttagatc gcgttattgt attttagttt gggtgataga gtgaggttac gttaaaaaaa 3780
aaaaaaaaat gtaaaaagtt acgattttgt gaatgtagat tttaggtaga taataatgat 3840
agtaaatttt tatagagtgt ttatttgtta ttagatattg ggtgaaatat tttgtatgta 3900
ttaatgtatt taatttttat attaatttta tgaagaagat tttatgatta ttattttttt 3960
tttgtagatg aggaaattga ggtattgaga agttaattta tttgttagta ggttaaatag 4020
ttagtagatg gtagttttta tatttgaacg tggttagttt aaaatgtgtg tttgaagaga 4080
tatagggggg tttttatttt aatttgtttt tagtatttgt ggttttggat aggttatgtt 4140
tttgggtagt tttggttttt gttttttaag tttttgtttt ttgtttgtgt ttggaaatga 4200
tttgggtttt agttgagtgt tggtggtatg aatcggttag ttagtttatt ggggagagta 4260
tattttagaa taattttgtt gtttgaaggg aggatttttg tgttgtggtt acgtgattgt 4320
aaaggtattt gttgaaattg ttgtttttgg gatttttaaa gatagtagag ttgttagatt 4380
tgtgttttga tcgtgtttag atagaggatg ttatgggggg agggtaagga cggtgcgata 4440
attttgaggg atttttggtg tggggagagg tagggggtgt gtaaggagtg taggttggag 4500
ggagatgatt aaggggtttt tgtgaatgtg gttagaatta gtaggaaggt agaatttttt 4560
tcgttttttt tttatgattt gtagatttgg atttagttta gtttttagaa gagatttaaa 4620
gagatttaag tagttggtta gtaggatttt gttttttata gaatagattt atttttttat 4680
agaatagggt tggggtaagg ttaggaaaat ttaataggta ttaaaaatat tgttaagttg 4740
ttgatagtat agttagaggt tttaggtttg ggtggtattt agatagtttt gtttttttta 4800
gatagttttt ttttttaggt ttagggattt gtttggttgt gagtttttag gaggttttag 4860
gggtgtgatt tttttttttt tttttttttt tttttttttt attttaggtt agtttagggt 4920
tagttataaa gttggtttag tttggttttt agtatattta gttgtttgag attttttttt 4980
aattttttta gaggatagtt ttattttgtt ttttgttttt ttagggtagt attatgtggt 5040
ttttgtggtt ttgttgggta ttttgggtgt tgtttttggt tggtttcggg gcggttttga 5100
tcgaggagta gtttttgggt agtttgttgc ggtagttgta gtttagcgag gtgttcgtat 5160
tggatagggt cgatatggag aagttggtta ttttcgttta cgtgagggtt tagtatgtag 5220
ttttgttgcg gcgtagttac ggggatcgtt ttcgcggaaa gaggtttagt tagagttttc 5280
gaggtgagat tttttttttt tgttggtttc ggttttatgg tcgggggtag gtcggttgtg 5340
tttattttgg aggtgtcgaa gggtttttgg ttttttggga ttttgtggga ggttgtgtga 5400
gggttacggt ttaaggagta gtgaggagga gtcgggtttg ggtttagggt ttttagttgt 5460
gtattgaggt ttttttttag tttaggggag gtgtattagg ttagatttgt tggaaaaatt 5520
aagattttag agaaggggag ggtagaggag agaaagtaga gttgggggag taggtgagtg 5580

tgtgttttaa ggtatttggt gtagttggat ttttagggta tgtttttata ttgttatttt 5640
ggtttgagtt tgattttgag taaatggttt taaaattttt tttttattat gtgtttattt 5700
ggaatagggg tatgattatg ttttcgttat tttttagtat tgtgaacgtt aaagtgagtt 5760
tttgtggata gaagcgtgtt tggataattg aaaaatattt aagtagatta tttgggttat 5820
atttggttgt taggggtgta attgggtttg aatttttttg tagtttttcg tttttttagt 5880
tttttagtgt ttagagttag agttagtgtt aggtatttgg gtgaggattt tgtttttagt 5940
attttagttg aggtaaattt gttagttagg gttttatagg tttttttgtt tgttggatgg 6000
aaattgttat taaataaggt ttagatttaa ggaagatttg taaattgggt ttgttttaga 6060
gatagaatag ggtcggtgtt ttttggtttg gttgttagtt tagtggtttt gttattttta 6120
gagtttcgtt ggagttcggg gaggttttgt tgattttgtt tttgttttta ga 6172

<210> 60
<211> 6172
<212> DNA
<213> Artificial Sequence

<220>
<223> chemically treated genomic DNA (Homo sapiens)

<400> 60

tttggggata agagtagggt tagtagggtt ttttcggatt ttagcggagt tttgaggatg 60
gtagggttat tgagttggta gttaggttag gagatatcgg ttttgtttta tttttggggt 120
aaatttagtt tataaatttt ttttggattt gggttttgtt tagtaataat ttttatttag 180
taggtagggg ggtttgtggg attttggtta gtaagtttgt tttagttgag atgttgggag 240
tagaattttt atttaggtgt ttggtattgg ttttagtttt gggtattgag gagttaggga 300
ggcgagaggt tgtaggagga tttaggttta gttgtatttt tgatagttag gtataattta 360
ggtagtttat ttaggtattt tttagttatt taaatacgtt tttatttata aggatttatt 420
ttgacgttta tagtgttggg agatggcgaa gatatagtta tatttttgtt ttagatgagt 480
atatggtgga gagaggattt taaagttatt tatttaaggt taggtttaga ttagagtggt 540
agtgtgaaaa tatattttgg gagtttagtt gtattaagtg ttttgaggta tatatttatt 600
tgttttttta gttttatttt ttttttttta tttttttttt ttttgaagtt ttggtttttt 660
tagtaggttt agtttggtgt attttttttg ggttggaggg gggttttagt gtatagttgg 720
agattttgga tttaggttcg gttttttttt attgtttttt ggatcgtggt ttttatatag 780
ttttttatag agttttaaaa ggttaggggt ttttcgatat ttttagagtg ggtataatcg 840
gtttgttttc gattatggga tcggggttag tagggaggga gggttttatt tcggaagttt 900
tggttgaatt ttttttcgcg ggagcggttt tcgtggttgc gtcgtagtag gattatatat 960
tgggttttta cgtgggcggg gatgattagt ttttttatgt cggttttgtt tagtacgggt 1020
atttcgttga gttgtagttg tcgtagtagg ttgtttagga gttgtttttc ggttagggtc 1080
gtttcggggt tagttagggg tagtatttag agtgtttagt agagttatag gggttatatg 1140
gtgttgtttt gggggagtag gaggtagagt ggggttgttt tttagggagg ttgaaggagg 1200
gttttaggta gttgggtgtg ttgagagtta ggttgggtta gttttatagt tggttttggg 1260
ttggtttggg gtgaagggaa gagggaggga gggagggagg gaggttatat ttttgggatt 1320
ttttgggagt ttatagttag ataggttttt gagtttggag gagggggttg tttagaaagg 1380
atagggttgt ttggatgtta tttaggtttg gggtttttgg ttgtgttgtt agtagtttga 1440
taatattttt agtgtttgtt gaattttttt ggttttattt tagttttgtt ttatgaaaaa 1500
gtgagtttat tttgtgaaaa ataaagtttt gttagttagt tgtttaaatt tttttaagtt 1560
ttttttggag gttgggttgg gtttaggttt gtaaattata aaggaagggc gggaaggatt 1620
ttgttttttt gttggttttg gttatattta tagaagtttt ttggttattt ttttttaatt 1680
tatatttttt gtatattttt tgtttttttt tatattaggg gttttttagg gttgtcgtat 1740
cgtttttgtt ttttttttat gatatttttt gtttagatac ggttaggata taaatttggt 1800
agttttattg tttttaggag ttttaggaat agtaatttta gtaagtgttt ttgtagttac 1860
gtgattatag tataggagtt ttttttttag atagtagaat tattttgggg tgtgtttttt 1920
ttagtgagtt ggttggtcgg tttatgttat tagtatttag ttggagttta ggttattttt 1980
agatatagat aagggatagg gatttagaaa gtagaggtta gagttgttta aaggtatggt 2040
ttgtttaaag ttataggtat tgggggtaga ttgaggtggg agtttttttg tgttttttta 2100
aatatatatt ttagattggt tacgtttaaa tatgagaatt gttatttgtt aattgtttga 2160
tttgttagta agtgagttaa ttttttagtg ttttagtttt tttatttata gaaagagggt 2220
gatgattata aggttttttt tatagggtta gtgtgaagat taaatgtatt aatatatgta 2280
ggatgtttta tttagtgttt agtgataggt aaatattttg taagggtttg ttattattat 2340
tatttgtttg aaatttgtat ttatagggtc gtggtttttt gtattttttt tttttttttg 2400
acgtagtttt attttgttat ttaggttaga gtgtagtggc gcgatttagg tttattgtaa 2460
tttttgtttt ttgggtttaa gtaatttttt tgtttcggtt tttcgagtag ttgggattat 2520
aggcgtatgt tacgttcggt taattttttg tatttttagt agagatgggg ttttatcgtg 2580
ttagttagaa tggtttcgat tttttgattt ggtgattcgt ttgttttggt tttttagagt 2640
gttgggatta taggtatgag ttatcgtgtc gggttaggtc gtggtttttt ataaaatttt 2700

taataataat aatagtatat attttttgag tgtatgtgtt agttattgtt ttaagttttg 2760
aatgtgtatt tatatagttt ttaggaggta ggtgttagtt ttaggtgtat tttgtagatg 2820
taaggttaga ttattgtttg gggaagtgat ttgttcgtta attttaatga agggtttggg 2880
agttaaatgg agtttttgtt tttgtaggta tttttttttg gtattggtat aggggatttg 2940
aggatgtgta gggggaagtt ttttttaaaa ttttatagtt ttatttgttt atgttttgtc 3000
gtatgagtaa ggatggtgga ggaggataag gattttgggt taagggggtt gtcgggtaag 3060
gggtattttt ttttattttt taaatggggg tattttggtt tttttgtggg aatgttgtgt 3120
tattgatagg tttttttatg ttattttttt ttatttttta aatgggggta ttttggtttt 3180
tttgtgggaa tgttgtgtta ttgataggtt tttttatgtt atttttttgt ttttgttttt 3240
aaatatttta ggtagaggaa gagggaattt tttggtgttc gaaggtggaa ttttttttgg 3300
atgaaagttt agggatttta tgttattata tatatatgta aatgtatatg tatatgtata 3360
tggaagtttt atatttgaat ttagttagtt tatagtgtgt gtttgaggag gtatgggggg 3420
tttttatttt aatttgtttt tagtatttgt ggttttgtat aggttatgtt tttgggtagt 3480
tttggttttt gtttttaggg tttttgtttt ttgttttgtg tttggaaatg atttgggttt 3540
tagttgagtg ttggtggtat gagttagttt attagtttat tggggagagt atattttaga 3600
atgatattgt tgtttgaagg gtttttagta gtgtgtgtgt tgggggagtg tttagtatta 3660
gagaaggggg agggataaga ataatattga taataaacgt aatgggttga gtgtggtgtg 3720
atttacgttt gtaattttag tattcgggga ggttaaggta ggaggattat ttgaggttag 3780
gagttcgagg ttagtttgaa taatgtagta agatttattt tttatagata agtataaata 3840
ataattataa tggttaatag tgtttgtggg ttatgtattg ttttgaatgt tttatattta 3900
ttgttaattt tttattttta ttataatttg aggaggaagt tgttgttatt attttttttt 3960
tatagatgaa gaaattgagt ataaaattgt tgtttgatta gtttaaagtt atatagatag 4020
tagatgttag agtttagggt atttgaaggg aaggaatagg gatttggtta tatgtgttta 4080
taaattaagg taagaggaaa tatagaaatt ttaatagttg atttcggggt gggagaagga 4140
gatttttggg gttatagaat tatgatttta gttgtttttt tgtttttttt tttttttttt 4200
ttttatttat attttcgtgg gatttgtgta atttataagg aggaatttaa attttatttt 4260
ggtatttatt ttttatagtt ggaatatttc gtgaaatata aatttttatt attgtgttcg 4320
gtttaaataa gattttttgt tttttttgtg ataagggtag ttttttttaa aagagaagtt 4380
gttgtttttt tttgtaagtg attaataggg aaaggattta tttgatgtaa atatttgggt 4440
tttaagttag gttaaaaggt gtagaagtgt tatatttata ggtaagtcgg gatttgcggg 4500
ttttagtttt tggaagttgt tttgggttgg tgttttataa atatgtaata gtttattatt 4560
tttgttgttg ggtgatagga agttttgtgg tttattattg gtgttaagga tattatattt 4620
aggttgtttt tgtatgttat tatttttttt ttagaatttt aatatataga tttattggga 4680
gtttattaag gttggatatt tataatgaga ttttataggt ttttgttttg gtttagtagg 4740
gatttgttgt tttagggttg agggaggtta ggtttttgtt gttgttgttt ttttgatttg 4800
tagatgttta ttcgaggttt ttttttagtt atgaaatatt atagggttga gaatttttgg 4860
aagtttttag ggatttagga gtttttttta tatttggggt aatagattag ttaaattgaa 4920
attttatttt tagagaagat agtaaagtgg agggattttt ttatattttt ttgatttttt 4980
gttttttttt ggagggattt tttatttttt tgattttttg ttttttttag atgttttgtt 5040
gatatttttg tatcggtttt ttagtatagg attaaagtta gagtagagtt tttaatagga 5100
agtgaaatta tatagttaat atttagtttt tttaaggatc gatttgtttt gtagggatgg 5160
gtgtttggga cgtagtgttt agggtggttt tgggggtcgt tggtagtttg ggtaagtgat 5220
ttacgattgg gaaattgaga tttttttttg atgttggggg atttttttta ggtggaggag 5280
ttggggaata tttagtatta atggaggggt ttgtttagtt agacgcgatt tatttttttt 5340
attttatttg ttagtttgta gaattagaga gagtttagta ttgaaattgg ttatttaggt 5400
tatgtttttg tttttcgggt tttagaaacg agaaaatgag gtttagagag atgatatttt 5460
ttttaaatat atatagtcga tgatttataa ttaatatttt tataaagtta ggttttttta 5520
tttttagatt aaagtttttt ttatgttttt aaattatttt tagttttgtt ttgatttttt 5580
gaagttagta gggttatttg ttagttttag gtaagttggg gatttagtta atttagggtc 5640
gggtttgggt aggggtaggg gaggcgtgag aagtagataa atagataaag gttaaatttt 5700
ttagattttt gaggtttaag atataggttt gggttttaaa tgtggatttt cggtagtttg 5760
aagagttttg tttggggaaa taaaataatt gggtaattag ttagtagtta aaagaagagt 5820
ttgaatgtgg attgggaaat tttttataat ggtttgatta gtcgttagga gagagggtag 5880
ttattttttg ttatgtagtg gaaggagttg ggtttttgag ggtttgagtg gagagtttgg 5940
ttgtggggtt aggttaggtt tgaaaaattg taaaaaaagt tttttttgtt ttggtgggag 6000
ggacggtgtt tgaagaatag ggaatattat ttaattaagt tgttttaatt aaaaattatt 6060
aaagtggaaa aaatgtagat tgtttttgaa tttttattgt taggggtttt aagatgttgt 6120
gaagattttt ttttttttgg agatgttatg ttgtagatgt tttaggtgta at 6172

<210> 61
<211> 5460
<212> DNA
<213> Artificial Sequence

<220>
<223> chemically treated genomic DNA (Homo sapiens)

<400> 61

atggatgggt gggtagacgg atggatggat gaatggatgg atggatggat taatggatag 60
aaagaaggtg gttgggagga aaggtggatg gacgggtgga tgggtggatt gatggataga 120
tgggtaggta ggtgaataga tggaaggatt attggatagg tatggtatag attgttggtt 180
ttatagtaga taagtataag gggaaaagtt tttgtttagg gttttttgtt tgttgagagt 240
agggaagtgt gttttggaga agagttgttg tgaggcggga gggtttttta agggttgggt 300
gttaggaaag tatattgggg atacgtaggt ttagggtcgg gttttggagt tatattgatt 360
tgttttttaa ttttggtttt gttatttttt agttgtttga tatggggtaa gttatttttt 420
ttgagtttcg tttttttatt tataaaatag ggatttatta tagtaattat tttataggta 480
tgttgtgata gtttacgaag ttaatatatt taaattattt agtatagggt ttagaatata 540
tttaatgttt aataaatgtt tatttatttt ttatttttaa aaggttaagt ggggaatttg 600
gtttttagtt tattagaggt aatttaaagt tttggagttt agagattttt attttagatg 660
ttgagttttg agttagtttt tttattttaa aaagtttagg aggatttata tggtttttta 720
ataagagtgt tttgattgaa ttatgtttgt ttttggttga aattttagta gttttatagg 780
tttggttgat tttgttttga ttatttttag attgataata aagagagtat atgaattatt 840
tttttgaaag tatagtttat gtgggagata taaatttttt taaatgggtt gagatagagg 900
gaagaggatt aagggtgttt tttaagggaa gttatggaga ttaggggagt cggtttcgta 960
ggttgaggtt gtttttgtaa agatttagtt tgaggatagt ggtgtgggta gtggttggtt 1020
ttattggttt agggtgagtt atggttgatg gtaggaagtt tagattattt attagaaaga 1080
ggttttgagg ttgagtggtt ttaggtttga gtgggggtag ttcgatttat agtatttggg 1140
ataagtggtg atgggaatgt ttgagttttt tgagttttta agttttagtt taggcggttt 1200
tagggttaaa ttttttttaa tttttagttt ttgtaagttt ttgttatttt tttcggttag 1260
tttttttttt tagttgttcg ttttgttagg tcgtttgtat agtgtgtgtt tgtagtaaag 1320
atattgtgtt ttttttattg gggttgtaaa tagaattttt taaaattcgg tggggttagg 1380
tgtcgatggg gaaatgattg gttttgtata ggtgaaggag tagaaggttt taagttgtaa 1440
gagttatgtt gttggagttt tttgggtttt tttttgggga gttttaaatt taggttagtt 1500
tgattttgaa tgttataggt agttagaggt gaaagtattt ttcgttatta atgggaaggc 1560
gggatatttg aggttaggga gaggagtttt aggttatatg ttatgaaaag tagagttggg 1620
ataaagtttt ttggtttagg gtttcgattg taattttagg tatttgttgt agtttagttt 1680
tgttgtgtta ggtattatta ggagatttag tttttatttt tatgtaattt ttatttagtt 1740
tagtgtttat agttgttgga tagttgtttg acgggattat atttgttttt agagttggga 1800
ttatagaggg agttttgggg tgtatagaga gatagtttaa ttttatttta atgttttagt 1860
ttttttgtta gtggggtttt ttttgacggt ttttttttaa gggatttagg attttgggtt 1920
tagtttggat ttttgggaga gttttacggg tgtataggaa agggtgaggt aggttaggaa 1980
ttaagagaat atatttagtt ttattagtgt tttgagttag ttgttttttt ttttgtattt 2040
tagttatttt atttattaaa tgggagtgga gacggggatt tttaggggga agtttttagt 2100
ttagtgtttg atttagaaat atatttaatt gttttttttt gttagttttt tttttttata 2160
tatttttttt ttttggattg ggttgttgta agtataaggg aaataaatcg tttagcgagt 2220
aaagtaattt tttttaaatt ttgagagggt gattttcggt ggttttttgt tttttttttt 2280
gtagttttaa taaattcggg taggtgttag ggttagagaa gtggggttag ggggtcgtga 2340
agttaggtta tttagaaagg atagggatag tgaggttagg atagttatag gtggtagggt 2400
tatgtagttc gattagttac gaggggtaga ggtaacggat agaggtttag gttaagcgat 2460
ttagggagat ggggttattt ttaatttagt ttaatttttt ttatttggaa attaattttt 2520
gttagtggag tagcgttgtt tcgggaatgg ttttattttt taagatttgt gttcgtttag 2580
gtttttgggt tagttttttt tattttatag gagttttgtt tgtttagttt aggtagaggg 2640
tagagaagga gagaaatcgg agttcggttt agttcgtagt tggttgtgtt gattttgcgg 2700
ggttcgggta gtagtttttg ttttgtagtt attttttttt tatattcggg agggattttg 2760
tttttgggtt tatttaatgt ttttattatg ttttttcggt tcggagtttt tggtttttta 2820
ttgttttgag taggcgtttt gtatttgtgt aaagattaga cgttttataa tttttttttt 2880
tttttttatg gttttttttt tttatttttt taaatttgag tttttttttt ttggttacgt 2940
attgtttttt tatttttttt tttttttttt gtttgttttt atttgttttt ttgtttgttt 3000
ttttttttat cgtgattttt ttcgtttttt cgttgatttg tttttttttt tttttttttt 3060
ttacggttta gcgatttgaa ttttttattt atttttaagt agtttttagt tttgtttaat 3120
ttcgtattta tatttttgta gaggttgata gaaatgattg tgagtattgt ttattttatt 3180
ttagaaatat ttttttaaat ttttcgagag gtacgacgtg ggagggtaga gagtatttta 3240
cggggagttt tttgtgtttt gtttgataga ttaagttatt tgttcgttgg gtggttttga 3300
ttaggttttg tttttttttg agtttcgtgg gtagttttga gttgattttt ttgtgataat 3360
agttgttttt tggggttgtg gtgaggaaga gtcgatagtg ttttaagtac gtagtaggtg 3420
tttaggaaat atttgtttgt tttttttata ggagttagtt tagtttttag gaaggaagat 3480
tttgttttta ttttttatta gttttgttag tttttttagg attttatata tgtacgtttg 3540
ttttgggtat attttaggaa gatgtaaatt tgtttgtttt tggtggttat ttattgtttt 3600
ttttataaat gtttaaggat aaaggagttg ttgaatatag gaattgagta ttgggtattt 3660
tgggttttgt aagtggaaag ttaggtgtgt attttttgat ttttttgttt ttaggaagta 3720
ttcgtaaggt ttttattgtg tgtagggtag gtgttaggtt ttgttttttt tggggttttg 3780

gattttgaga ttgtagggga gataagtgta atgggatgag ttaggtaggt acgagaggta 3840
taggttttgg gaattttaag aaacgagagg gttttttttg ttgtgtttgg agatagggtt 3900
tttgagaaga ttttagtagg aagtggattt ttagttaggg taggaataga gggtgtttgt 3960
cggttttttg agttgtttag tatagttgtg gtttattttt gttttttttt tttcggtaag 4020
gtttttgggt tagtagaggg gtaggagtag aatgttagtt tttagagaag ttttttggtt 4080
tatttttttt gtagatagta gttttcggtg gaagttaaga tattttgtta tggttaggat 4140
aggagattgt ttaaggggcg ttttgtgtgt gtgtttttag ggatattttt ttggaaaggg 4200
gtttgaagtt atttttttag ttttaaagga gtttgtaagt taagtaggtg aagtattttt 4260
ttgttttggt tttgtttgta aataaggaaa taggtttagg aaggatgttt ttggttatgg 4320
ttgtatagtt gaatagtttt tggaagggat tagaattttt gttttaggtt ttggggtgat 4380
agggtagggg ttgaggggga tattttttgg gtgaggtggg gatgaagtag ttttaggttg 4440
gttagaagtt aggtatgagt tgtgtttttt atagagggag gtaggtttag agagggtttt 4500
gattcgttta aggttatata gttttgcgtg ggttttttat attttatata atagaggggt 4560
atttttagtt tggttggtag agggtaggta ggatagatgg tagagttttt ttcgaggaga 4620
ggggttttgt ttatggaatt ttttttttta tatttatagt tttgggcgag atttgtggag 4680
tagtcgttaa tagagtgagg gagggggttc ggggtagttg ggggtgattt gaggaagttt 4740
agttggattg cgaggggttt ttggggattg ttagggagtt ttaggatttt tagaggtgtt 4800
ttaggtatag agggaggaat gggtttttta tttttttttt ttttttattg tagaggttgg 4860
gtcgggttag gcgttcgggg aggaggcggt gtttttggtt tttagttcgt cggtgtagcg 4920
gggtagggtt ggattagaag gggtggggta tcgtgtttgg tataagaggt agttagggta 4980
tcgaggtaat gagttatttg tttagtttaa tagtaggacg ttggtaatag gcgttttttg 5040
ttttagttta gtttgcgcgt tttatcgtcg ttatggtttt cgtgtttatt atttggtgtt 5100
tcgttgcgtt agttttgttc gtggttttgt atgaaggtga gttgttttgg ttttatttgt 5160
tgtttatagt ttttgggttt gtttattttt gttttttttt agggttgtag tgttttttaa 5220
gttagttggg ggtagatagg ggcgggagtt tggcggtagt ggtggtaggg gaaaggtttt 5280
gtaaatatag aggtgtttag gttttttacg ggttgttggg atttttttgg gttggtttgg 5340
agtttttgag tagtaagagt gttaattttt tcgattatgt aagaggaggg gtatggtggg 5400
ttttttattt tgggatgtgt taggtatttt aatttatttt tttatttttt tttttttttt 5460

<210> 62
<211> 5460
<212> DNA
<213> Artificial Sequence

<220>
<223> chemically treated genomic DNA (Homo sapiens)

<400> 62

aggagggaga gagaggtgga gaggtgggtt ggggtgtttg gtatatttta gagtggggga 60
tttattatgt tttttttttt gtataatcga gggagttggt atttttgttg tttaagagtt 120
ttaggttagt ttagggaagt tttagtagtt cgtggggagt ttgggtattt ttgtatttgt 180
agaatttttt ttttgttatt attgtcgtta ggttttcgtt tttgtttgtt tttagttggt 240
ttgggaggta ttgtagtttt agagggagat agagataggt agatttagga gttgtaggta 300
gtaggtgagg ttagggtagt ttatttttat gtagggttac gagtagggtt agcgtaacgg 360
agtattaggt ggtaggtacg gagattatag cggcggtgga gcgcgtaggt tggattggag 420
tagggagcgt ttgttgttag cgttttgtta ttaagttgag tagatagttt attgtttcgg 480
tgttttggtt gttttttata ttaggtacgg tgttttattt tttttggttt agttttgttt 540
cgttgtatcg gcgggttggg agttagggat atcgtttttt tttcgggcgt ttggttcgat 600
ttagtttttg tagtgaaggg gggagggaga tggaaggttt attttttttt ttgtgtttgg 660
agtatttttg ggagttttga ggttttttgg tagtttttag gggtttttcg tagtttagtt 720
ggattttttt aagttatttt tagttgtttc gagttttttt ttttattttg ttggcggttg 780
ttttataggt ttcgtttagg gttgtgagtg tggaggagga ggttttatga gtaaaatttt 840
ttttttcgga gaagattttg ttatttattt tgtttgtttt ttgttaatta ggttgaggat 900
gtttttttgt tgtgtgggat gtgaaaggtt tacgtaaggt tgtgtggttt tgggcgagtt 960
agagtttttt ttgggtttgt ttttttttgt gaaggatata gtttatgttt gatttttgat 1020
tagtttggaa ttgttttatt tttattttat ttaggaaatg ttttttttaa tttttgtttt 1080
gttattttag ggtttggggt aggagtttta gtttttttta gaagttgttt agttatgtag 1140
ttatggttaa gggtattttt tttgggtttg tttttttgtt tgtaaataag gttaaaataa 1200
gaaggtattt tatttgtttg gtttataggt ttttttgaga ttaagaaggt ggttttagat 1260
ttttttttag aaagatgttt ttgaggatat atatataaag cgttttttga atagtttttt 1320
gttttggtta tgataggatg ttttggtttt tatcgggggt tattgtttgt aaaagaagta 1380
agttagggga tttttttggg gattggtatt ttgtttttgt ttttttgttg gtttagaagt 1440
tttgtcgggg aggggaaagt agggatgaat tatagttgtg ttgagtagtt tagggaatcg 1500
gtaggtattt tttgtttttg ttttggttga gagtttattt tttgttggag tttttttagg 1560
gattttgttt ttaggtataa taggaaaggt tttttcgttt tttggaattt ttagggtttg 1620

tgtttttcgt gtttgtttga tttattttat tgtatttatt ttttttgtaa ttttaaggtt 1680
tagagtttta gaggaggtag ggtttgatat ttattttgta tatagtaggg gttttgcgag 1740
tgttttttga gggtaggggg gttaagagat gtatatttgg ttttttattt atagaattta 1800
ggatgtttaa tgtttagttt ttgtatttag tagttttttt gtttttggat atttatgaag 1860
gggatagtag gtgattatta ggaataggta agtttgtatt tttttagggt gtgtttagag 1920
taagcgtgta tgtgtgaaat tttggagaaa ttggtagaat tgatggaggg tgagagtagg 1980
attttttttt ttggagattg agttggtttt tgtgaaaaaa ataaataaat gttttttgag 2040
tatttattac gtgtttggag tattatcgat ttttttttat tataatttta ggaggtagtt 2100
attattatag gggagttagt ttaggattgt ttacgaggtt tagagaggag tagggtttgg 2160
ttaaggttat ttagcgggta ggtggtttgg tttattaagt aaagtataag ggatttttcg 2220
tgaggtgttt tttgtttttt tacgtcgtgt ttttcgaaga atttagaaga gtatttttgg 2280
aatgaggtga gtaatattta tagttatttt tattagtttt tgtaagaata tgaatacgga 2340
attaagtagg gttgggggtt gtttgggggt ggatagagaa tttaaatcgt tggatcgtga 2400
agagaagaaa gaaggaaaag taggttagcg gggagacgga gggggttacg gtggggagga 2460
aaataaatag ggaggtagat aaagataggt agggagagag ggggagaatg aggaagtagt 2520
gcgtagttag aaagaaagaa tttagatttg agaaagtaaa agagggaaat tatagggaga 2580
agagggaagg ttataaagcg tttaattttt atataaatgt aaaacgtttg tttaagataa 2640
tagggaatta gaggtttcga gtcggggaaa tataatagaa gtattaaatg aatttagaga 2700
taggattttt ttcgggtgtg aaagaagggt gattataagg taggggttgt tgttcgagtt 2760
tcgtagggtt agtatagtta gttgcgagtt gggtcgggtt tcggtttttt tttttttttg 2820
tttttgtttt gagttgaata agtagggttt ttgtgaaatg gagaggattg gtttaggaat 2880
ttaagcgagt atagatttta ggggatgaag ttattttcgg aatagcgttg ttttattgat 2940
aagagttagt ttttaggtag ggaaaattaa gttgagttaa aaatagtttt atttttttgg 3000
gtcgtttagt ttgggttttt gttcgttgtt tttgtttttc gtggttggtc ggattgtatg 3060
attttgttat ttataattgt tttggtttta ttgtttttgt tttttttagg tggtttggtt 3120
ttacgatttt ttggttttat ttttttggtt ttggtatttg ttcgagtttg ttgaggttgt 3180
agggggaagg gtagggagtt atcgaaggtt atttttttag aatttgaaga gaattgtttt 3240
attcgttaag cgatttgttt tttttatgtt tgtagtagtt taatttaaga aagagggatg 3300
tatggggagg gagggttggt agaagagagt agttgagtgt atttttgagt tagatattgg 3360
gttagaaatt tttttttaga aattttcgtt tttattttta tttggtagat gagataattg 3420
aggtgtaaga ggaggagtaa ttggtttaag atattgatgg ggttgggtgt gtttttttgg 3480
tttttaattt gttttatttt tttttgtata ttcgtgaagt ttttttaggg gtttaaattg 3540
agtttaggat tttgggtttt ttggagaaag gtcgttagga aggattttat tggtaggaag 3600
gttggggtat tgggatgagg ttaggttgtt tttttgtgta ttttaaagtt ttttttatag 3660
ttttagtttt aagagtaaat atggtttcgt taagtagttg tttagtagtt gtggatattg 3720
agttgaatgg aggttgtatg ggagtgaaga ttaagttttt tagtgatgtt tgatataata 3780
gggttgggtt gtagtaggtg tttgggattg taatcgaggt tttggattag gaagttttgt 3840
tttagttttg ttttttatga tatgtggttt agggtttttt tttttggttt taggtgtttc 3900
gtttttttat tgatggcgag agatgttttt atttttgatt gtttgtgata tttaggatta 3960
agttgatttg agtttagagt tttttagaag gaggtttagg agattttagt agtatggttt 4020
ttgtagtttg agtttttttg tttttttatt tgtgtaaaat tagttatttt tttatcgata 4080
tttggtttta tcgggttttg ggaagttttg tttgtagttt tagtagaagg gatatagtgt 4140
ttttgttgta ggtatatatt gtgtagacgg tttagtagag cgagtagttg ggaagagggg 4200
ttggtcgggg gagatgatag gagtttgtag ggttgggggg ttggggaggg tttggttttg 4260
gagtcgtttg ggttggggtt tagggattta ggagatttaa atatttttat tattatttgt 4320
tttaaatgtt gtgggtcggg ttgtttttat ttaggtttgg ggttatttag ttttagggtt 4380
tttttttggt gggtggtttg ggttttttgt tattagttat agtttatttt gagttagtag 4440
gattagttat tatttatatt attgttttta aattgggttt ttgtaagggt agttttagtt 4500
tgcggggtcg gtttttttgg tttttatggt tttttttgga ggatattttt ggtttttttt 4560
tttttgtttt agtttatttg gaaagattta tattttttat ataaattgta tttttaaaag 4620
aataatttat gtgttttttt tgttattagt ttaagggtaa ttagagtagg gttagttagg 4680
tttatgaaat tgttggaatt ttagttaaaa gtaaatatgg tttagttaag atatttttgt 4740
taagaggtta tgtaagtttt tttggatttt ttgaaatgag gaaattaatt taggatttag 4800
tatttaggat ggaaattttt ggattttaag gttttgagtt atttttaatg ggttaagggt 4860
tagatttttt atttagtttt ttaggaatag aggataaata agtatttatt gagtattgaa 4920
tatattttga gttttgtgtt aagtgattta ggtatattag tttcgtgaat tgttataata 4980
tatttgtggg gtagttatta taatgagttt ttgttttata aatgaagaaa cgaggtttag 5040
agagggtgat ttgttttatg ttagatagtt aggaagtggt agagttaagg ttagaaagta 5100
agttagtgta gttttaaagt tcggttttga gtttacgtgt ttttagtgtg tttttttgat 5160
atttagtttt tggggagttt tttcgtttta tagtaatttt tttttagggt atattttttt 5220
gtttttagta ggtagagagt tttaggtaag aatttttttt tttatgtttg tttgttatgg 5280
gattaataat ttatattatg tttatttaat gattttttta tttatttatt tatttattta 5340
tttatttatt aatttattta tttattcgtt tatttatttt tttttttaat tatttttttt 5400
ttatttattg atttatttat ttatttattt atttatttat tcgtttattt atttatttat 5460

<210> 63

<211> 5791
<212> DNA
<213> Artificial Sequence

<220>
<223> chemically treated genomic DNA (Homo sapiens)

<400> 63

tgaaaggttt aaggagttat gttgggttta gggtttatga tttagttttg attttttatt 60
tttatttttt attttttatc gattattttg tgttggtatt attttcggag agatttttta 120
ttttatgatg ataagtattg ttggtattag taagttttta tttgattttt ttagcggttt 180
tttagaaaaa tagaagagtt tttttttaaa gttttagaat tgaatttttt gggttttgag 240
tagttaggtt tggtttatgg gtttattttt ggataaatta ttatatttaa agttgtatta 300
attttttatt gttgttgtaa taaattatta tgaatttaat ggtttaaaat aatattaatt 360
gattatttta tagttttgga ggttagtagt ttaaaaatag gttggaaggg ttatgtgttt 420
tttggaggtt gaaggaaaga atttattttt ttgttttttt tattttttag atattgtttg 480
tgtttttttg tttatagttt tgtattagtt taatttttgt ttttattgtt atagtttttt 540
tgacgttgat tttttttttt ttttttaaaa gaatattttt aaaaattata ttgtttatcg 600
ttttttattt tcgataattt agggttattt tttattttaa tgtttttaat tatattttta 660
aaattttttt tgttatgtta gttgatatat ttataggttt tgaggattag gatatggata 720
tttttgggga tattattatt ttttttattt taaagatgat gtatggtttg attagttaat 780
ttatagttat aagtttggtt ttggagcgtt ttttgaaatt atttggatag agaaggggag 840
gggtggtttt ttaggggaag atgtggttta ttattagatt tggagggatg gggatgttgg 900
tagggtaata atagtaaatg tttatagtag aaaatatagg aaaaaatttt aggatttttt 960
tgaataagtt ttgttgtttt tgggattatt cgattttttt taggttttga atatatttta 1020
ttgatagtat taaagtttag aatgtattgt gattatttta ttttacggag aatattatat 1080
atagtgtatt ttatttgtaa agtaagggat ttggagtttg gtattttttt tagtgtattt 1140
tagaatgaat tacgtgttag tgagggaaga tatagttaag agttggataa tatggaaaag 1200
tatttaatta tttggttttt gagtaagttt agagtttttg ttgttgtggg tgggtgggga 1260
gaagaaattt tagtatgatg gaaaattata taatgtatta tgttattttt ttttaaattt 1320
tttaaattaa aggaaaaata atttttaaag aattataaag tatataaaga gataatttga 1380
ggggatgttt atttgagata aattgtaata tttttaatgg ttgggttttg aattaaaatg 1440
taagtggtaa ggatttaaag tattagtcgt atatttgttg atttggaaaa agttgaggaa 1500
aaataaatat aatgattaag tcgtgtattt ggtcgagttt tagtagtgtt ttgatatatg 1560
atttttttta ggggtatatt ggatgttgag gtagtgaagg aggattaagg aatattggta 1620
gacgtttgcg gagaagaatg tttatttatt cgtgtttttt gtagaggtta ttattttttt 1680
atatgatttt ttattagaga ttatggaatg gaaattagat agatggagag gtagtgagtt 1740
gttaatgata tatagaagaa aatgggaatt attaaaattt tgggtatgag ttttagaggt 1800
agcgttgttt aagtattatg aattttgttt ttggaattat gtggagttaa atttgaattt 1860
tgatagattg ttatttaata gatgtatggt tgtaggttgg ttattttgat tttttcgagt 1920
tttagttttt taatttgtga gatggggata atgttagtat aaattttgtt gagttgttat 1980
aagggtaaaa taatgatgga aattattagt ttgttattga gttatatgaa ttgtatattt 2040
tttttgtata ttttttagtt tggaatttag aaggttttgt gattttgtta agtttttaag 2100
tcggttttat aatgaattat tttatattgt aattgaggtt atttttattt gatttaagtg 2160
tattaattta gaaaggaatt ttatgttaat tattattatg tatagtaagt tcgattttat 2220
agtttgataa aggttttaag tttatggttt gttatttttt tgtataagtt tatggtttgt 2280
tatatttttt ttgtattttt ttgtttaaaa atttttttgt atagtttttt ttttgttcga 2340
gagttgttaa agatatatcg agttattgtt ttggttatcg ttagaagtta gaagaatagt 2400
aggaaaggga gttttttcgt ttgagagttg gtgttggcga gtgttattgt aggttgttgt 2460
tttaaattat ggcgttcggt ttgttttagg gattaaggat aatgttgttt tttttgatta 2520
tttagttgaa tatattgttt ttttttttta gttttgtgcg gcgttttatg tttttagtat 2580
tttggatttt tggcgattta tttttgaatt tttttatttt aattagagta gaagatttag 2640
agggtagggt tagttttatt ttggtatttg gttagtattt tttggtgttt gagaggttta 2700
tttagggttt gttgattttt agttttagtg aagataagtg gtggtcggtt ttaagttatt 2760
gaaggttata gatttagaga gtggtttaag tagttatgat attttttgtg tttgaatatg 2820
gtattcggaa attttttttt attttttttt tttttttagt tgattagtat attttttatt 2880
tttttagtta ttttttaatt taagaatttt ttattttgtt gaagatggaa attttaattt 2940
taaagagata gtaattatta tggtttgaat atttgttttt tttagattta tttgttgaaa 3000
ttttatttat gaaggtgatg atgttaggag gtggggtttt tgaggggtga ttaagttatg 3060
agggttgtag tttttatgaa tggggttggt gtttttagaa aagagatttt tagagagttg 3120
ttttgatttt tttataattg aggatgtagt gagaagttat tatttatgat tagaaatcga 3180
gtttttatta aatatcgaat ttattagtat tttgattttg gttttttaga ttttaaaaat 3240
gtgaaaaata aaattttgcg gtttattagt tatttagttt atggcgtttt gttatagtag 3300
ttttaataaa taaagataat attaaaatga tttaaatttt agtttagtag gaatagaaga 3360
tattgtgagt tttgggtttt ttttattttt aaagtattaa gtgtaaaaat aaataaataa 3420

atatttggat ttaagggtta ggtaggatgg gatgggagtg gggggatgga gaattttagt 3480
attagtttta gtgttggaag tttgagtgtt attttagtta ttgtagtttt gtattaattt 3540
atttagagtt tagtgttgta aaataattat ttattatgtt tatggattgt gtgggtggga 3600
agttcggata gggtatagcg ggatagttat tttttagttg gaagttttga gggttgggag 3660
tttaaattat cggaaggtat atttatttag gttagtggtt gatattggtg gtcggttggg 3720
aggtttcggt ttttttttac gtaggttttt ttaggtgatt tttttaggtg ggtttgttag 3780
ggtttttttt tggtatagta gttgtgttta gaggataggt gttttgagtt attaagttag 3840
ggggatgttg tagtattttt ttggattgtt tttagaaatt gttttggtta ttttagttgt 3900
tttttagttt ttagaagttg gtcgttaagg ttagtagagg gtaggattta atttaatttt 3960
attttttaga gatgaggttt cgttatgttg tttaggttgg ttttgaattt ttaggtttaa 4020
gtaatttatt tattttggtt ttttaaagag ttggaattat agatgtgaag tattgttttt 4080
tttttaattt tatttgttat gaaaggaggg ttttttgatt ataagtgttt tgagaagtta 4140
ggaggatttt ttagttttag atatttttat ttgagttgga gatggggata gttttttata 4200
tataggattt ggaaggttat gaaggaagta gggggttttt ttgggaggtt attttttttg 4260
gtagggacgg ttgtgtgaat gaatttggtt tttagggaga ggagttcgat ttttatttga 4320
agttatttaa tttgtgtaga tggttgagga agttttggat attataggat cgttaattta 4380
ggatttttgt tgattttgtt tttaggttat tgttttatac gtaggttttg aatttttggg 4440
agaggtaggg agttagttcg aggcgtgagt taagagtgta aagtatatcg agttttttgg 4500
taagtaggtt ttttaagggg aaggtgttat tcggggaata atgagaaatg tgtttttttt 4560
ttagtttttg atagaaaaga taatattttt tgagtatagt ttttttttat ttgtttttgt 4620
ttttggatgg ggggttgtta tttcggagcg gcgaattggc gggcgggggt gcgtttgtga 4680
gtgtgagcgc gcgtgtgcgt gtgtgcgcgc gttttcgtgg tttttcggta gttggtagtt 4740
gtttcggttt ttattttatt ttattttatt ttatttagtc gcgttacgtt acgtcgtttt 4800
cgtttttcgt tgttttgggt gcgtttcggg tcgttgcgtt tttggttagt tttttttagc 4860
gtttgggagg cgatttttag tgcgttcggg tttaaaggtt cggcgaggta gttttagttt 4920
ttttgggggg cggggaggcg acgggggtgg tgtagaagtt agaaaagttc gagttatagt 4980
cggttagttt cgcgtaggga tgggtagcgc gttttgaaag tttatgatcg tcgtagttaa 5040
tttttggtcg gagttggaga cgtagcgagc gatcggtcgg tttcgaattt ttatagttgg 5100
agggcgaggt tagttgtatt cggttttagt gttttttcgc ggttataagc ggtcgttttt 5160
ttggttcggt gtttcggcgt ttgatttagg tttatggagt cggggttgtg gttttttttc 5220
gggtttatag tgattttcgt cgtaggtaag cgtacggggc ggcgcgtttt ttttggcgcg 5280
agcgtatata aaaggattta gggcggggga ttcgaggcgc ggagaagatg tgcgtcgcgg 5340
ggagtaaatt tttgtttggg ttttgtattc gtgaagacgt ttgggggagg gttggggagt 5400
agaagcggcg cgtaacgatt attcgtacgg gtttcgggag ttgtagttcg cgtattggtt 5460
ggagatttgt tgtttgggtc gatttggtta attttttatg aaattgggga ttttttagaa 5520
agtttgcgaa ttatttagag gttgtgttaa attttggtaa agtttgtaga aagtatgtaa 5580
aatgtatgta agtttttaac gggtgtagat agtttgtaag ttttttatag agtgtgtaga 5640
tagtttggaa attttttaga aattgcgtag agttttggaa attttgtaga tattttgttt 5700
gttttatttt tttttttagg tgtttggggg tggcgggtgt tgaggggttt gtatatttag 5760
tttaggagtt ttttaatttg ttttttgtag g 5791

<210> 64
<211> 5791
<212> DNA
<213> Artificial Sequence

<220>
<223> chemically treated genomic DNA (Homo sapiens)

<400> 64

tttgtagaag gtagattgag gggtttttaa gttgagtgtg tagatttttt aatattcgtt 60
atttttaaat atttgaggag gggggtggag taagtaaaat gtttgtaaag tttttaaagt 120
tttgcgtagt ttttgaaaag tttttaagtt atttgtatat tttgtgaaaa gtttgtaagt 180
tatttgtatt cgttgaaaat ttgtatgtat tttgtatatt ttttgtaaat tttgttaggg 240
tttggtatag tttttgaata gttcgtaaat tttttgaaaa gtttttagtt ttatgaaaaa 300
ttgattaagt cggtttaggt agtaggtttt tagttagtgc gcgggttgta attttcggag 360
ttcgtgcggg tagtcgttgc gcgtcgtttt tgttttttag ttttttttta ggcgttttta 420
cgagtgtaga gtttaggtaa gagtttgttt ttcgcgacgt atattttttt cgcgtttcgg 480
gtttttcgtt ttgggttttt ttgtgtgcgt tcgcgttagg agaggcgcgt cgtttcgtgc 540
gtttatttgc ggcggaggtt attgtgagtt cgaaaaggag ttatagtttc ggttttatga 600
atttagatta ggcgtcggag tatcggatta ggaggacggt cgtttgtggt cgcgaaaggg 660
tattggggtc gggtatagtt ggtttcgttt tttagttgtg ggggttcgag gtcggtcgat 720
cgttcgttgc gtttttagtt tcggttagga gttggttgcg gcggttataa atttttagag 780
cgcgttgttt atttttgcgc ggagttggtc ggttgtggtt cgggtttttt tggtttttgt 840
attatttcg tcgtttttc gtttttaga ggggttggaa ttgtttcgtc gggttttga 900

attcgggcgt attgggggtc gttttttagg cgttgggggg agttggttag gagcgtagcg 960
gttcgaggcg tatttaggat agcgaggggc gggggcggcg tggcgtggcg cggttgggtg 1020
gggtggggta gggtggggtg ggggtcggga tagttgttag ttgtcggaag gttacgagag 1080
cgcgcgtata tacgtatacg cgcgtttata tttataggcg tattttcgtt cgttagttcg 1140
tcgtttcgga gtagtagttt tttatttaag agtaagaata gatgagaaaa ggttgtgttt 1200
aaggggtgtt atttttttta ttaggggttg agggaaagat atatttttta ttgtttttcg 1260
agtgatattt tttttttgaa gagtttgttt gttaaggaat tcggtgtatt ttatattttt 1320
gatttacgtt tcgggttggt tttttatttt ttttaggagt ttagagtttg cgtgtggggt 1380
agtggtttgg gagtaaagtt aataaagatt ttgggttagc ggttttgtgg tgtttagggt 1440
ttttttagtt atttgtatag gttgggtggt tttaggtgag agtcgggttt ttttttttaa 1500
aggttaagtt tatttatata atcgttttta ttaagggaaa tgatttttta agagagtttt 1560
ttattttttt tatagttttt taagttttgt gtgtgggagg ttgtttttat ttttagttta 1620
gataaaaatg tttgagattg ggaagttttt ttgatttttt agggtatttg tggttaaagg 1680
attttttttt tataatagat ggggttgggg gaggggtagt gttttatatt tgtaatttta 1740
gttttttgag aggttaaggt ggatggattg tttgagtttg ggagtttaag attagtttgg 1800
gtaatatagc gagattttat ttttaaaaaa tggggttgaa ttgggttttg ttttttatta 1860
gttttaacga ttagtttttg aaaattagaa agtagttgaa gtgattagag tagtttttga 1920
ggatagttta aaagagtgtt ataatatttt tttggtttga tagtttagga tatttgtttt 1980
ttgaatatag ttgttgtatt aggaggaagt tttaataagt ttatttggag agattatttg 2040
gagaaattta cgtggagaga aatcgaggtt ttttagtcga ttattagtgt tagttattga 2100
tttgagtgaa tgtgtttttc gatgatttga gtttttagtt tttaagattt ttaattgagg 2160
aatagttgtt tcgttgtatt ttgttcgaat tttttattta tataatttat gagtataata 2220
aatggttgtt ttatagtatt aaattttggg taaattggta tagagttgta gtaattagag 2280
tagtatttaa gtttttagta ttgaagttga tgttgaagtt ttttattttt ttatttttat 2340
tttattttgt ttagtttttg aatttaagtg tttatttgtt tgtttttgta tttgatgttt 2400
tgaaaataag aaaaatttaa gatttataat attttttgtt tttgttgggt tagaatttgg 2460
attattttag tattattttt gtttgttggg gttattataa taaaacgtta taagttgggt 2520
ggttgataaa tcgtagagtt ttatttttta tatttttggg gtttggaaaa ttaagattaa 2580
ggtgttggta gattcggtgt ttaataaggg ttcgattttt gattatggat ggtgattttt 2640
tattgtattt ttagttgtgg aagggttaag gtagtttttt gggggttttt tttttaagga 2700
tattaatttt atttatgagg gttgtaattt ttatgattta attatttttt aagggtttta 2760
ttttttgata ttattatttt tatggatagg attttaatag ataaatttgg agaggataaa 2820
tatttagatt ataataatta ttattttttt ggaattaaaa tttttatttt taataaaata 2880
aaaagttttt ggattggggg atggttggag gaatagaaaa tatattaatt agttaaaagg 2940
agaaaaggag taggaggagg ttttcgggta ttatgtttag atataggagg tattatggtt 3000
gtttagatta ttttttgggt ttatggtttt tagtagtttg aagtcgatta ttatttgttt 3060
ttattgggat taaaaattaa taaattttgg atgagttttt taggtattag gaggtgttag 3120
ttaaatatta ggatggagtt ggttttgttt tttgggtttt ttattttagt tgaggtaagg 3180
aagtttagaa atagatcgtt aaggatttag gatgttgaga atataaaacg tcgtatagag 3240
ttaagagaag aagatagtgt gtttagttgg atgattagag gaggtagtat tgtttttggt 3300
ttttaaagta agtcgggcgt tatgatttga ggtagtagtt tgtagtgata ttcgttaata 3360
ttagttttta agcgaaagag tttttttttt tattgttttt ttggtttttg acgatggtta 3420
gggtaatgat tcgatgtgtt tttaatagtt ttcgaataga gagagagttg tataaaggga 3480
tttttgaata aagggatata aaaggaatgt agtaggttat aggtttgtat aaagggatag 3540
taggttatag gtttggagtt tttgttaggt tgtggaatcg ggtttattgt gtatggtagt 3600
gattgatata aaattttttt ttaaattaat atatttaagt taagtaaagg taattttaat 3660
tataatatga agtaatttat tgtaaagtcg gtttggagat ttggtaagat tataaagttt 3720
tttgggtttt aggttaagaa atgtgtaaag gaaatatata atttatgtaa tttaatggta 3780
agttggtggt ttttattatt gttttgtttt tgtgataatt taataaaatt tatgttaata 3840
ttatttttat tttatagatt aaaaaattaa ggttcgaaaa ggttaaagta attagtttat 3900
aattatgtat ttattaagtg gtagtttatt aggatttaaa tttgatttta tatgatttta 3960
gggatagagt ttataatgtt taagtagcgt tgtttttgga atttatattt aaggttttaa 4020
tggtttttat ttttttttgt gtattattga taatttattg tttttttatt tgtttagttt 4080
ttattttata atttttgatg gaaggttatg taaaggaatg atgattttta tagaggatac 4140
gaatgaatag gtattttttt tcgtagacgt ttgttaatat tttttgattt ttttttattg 4200
ttttagtatt taatgtgttt ttaggagaag ttatgtatta ggatattgtt ggggttcggt 4260
taagtgtacg gtttggttat tgtgtttatt tttttttaat tttttttaag ttagtagatg 4320
tgcgattgat attttgagtt tttattattt atattttgat ttaaaattta gttattaaga 4380
atgttgtaat ttattttaga taagtatttt tttaagttgt ttttttatgt gttttatgat 4440
tttttgaaga ttgttttttt tttaatttaa gggatttaag ggagagtaat ataatatatt 4500
atgtagtttt ttattatgtt ggaatttttt ttttttattt atttatagta gtaggaattt 4560
tggatttatt taaaaattaa gtaattaagt gtttttttat gttgtttaat ttttagttgt 4620
gttttttttt attggtacgt ggtttatttt aagatgtatt ggaggaagtg ttaggtttta 4680
aattttttgt tttataaatg aggtatattg tgtataatat ttttcgtgaa ataaaatgat 4740
tataatgtat tttgaatttt agtgttatta ataaaatgtg tttaaagttt aagagaaatc 4800
gggtgatttt aaaagtaata aaatttgttt aaaagagttt tgaagttttt ttttgtgttt 4860

tttgttgtgg atatttgttg ttgttgtttt gttagtattt ttattttttt aggtttggta 4920
atggattata tttttttttg gggaattatt tttttttttt tttgtttaag tggttttagg 4980
ggacgtttta gggttaggtt tgtgattatg gattaattaa ttagattata tattattttt 5040
gagataggaa gaataatggt atttttaaag atgtttatgt tttaattttt agaatttgtg 5100
aatatgttaa ttaatatggt aaaagggatt ttaagggtgt gattaaagat attgagatgg 5160
gagatgattt tggattatcg gggtgggggg gcggtgggta atgtaatttt taaaaatgtt 5220
tttttaagag ggagggggag ggttagcgtt agagaagttg tgataatgga aatagaagtt 5280
gggttggtat aaggttatga gtagaggaat ataggtaatg tttagaaaat ggaaaaggta 5340
agggaatgaa tttttttttt tagtttttag aaggtatata gtttttttaa tttattttta 5400
gattgttgat ttttagaatt ataagataat taattggtat tgttttaaat tattaagttt 5460
atgatgattt gttgtagtag taataagaaa ttaatataat tttaaatata gtgatttgtt 5520
tagagatagg tttatgagtt aagtttggtt gtttaggatt taggagattt agttttggga 5580
ttttgaaaag aggttttttt gtttttttgg ggagtcgttg agaaaattag atgaaagttt 5640
gttggtgtta gtagtatttg ttattatgag gtgaaaagtt ttttcgggaa tggtgttaat 5700
atagagtaat cggtagaaag tagaaagtag aagtagggaa ttaaggttaa gttataagtt 5760
ttggatttaa tatggttttt tggatttttt a 5791

<210> 65
<211> 9543
<212> DNA
<213> Artificial Sequence

<220>
<223> chemically treated genomic DNA (Homo sapiens)

<400> 65

ggttaatatt gttatgtgtg aatttgattt tgttattatg atgttaggtg gttattttgt 60
ttattaatta atgtagtttt tttttagttt tgacggtttt tataatttgg tatgtatttg 120
tagtggttgg tatcggttgt ttttttttat gtgtagtgtt ttttttatgt gtttttttag 180
ggtaggtttg gtggtgataa aatttttcgg tatttgtttg tttgtaaagg attttatttt 240
ttttttattt atgaagttta gtttggttgg atatgaaatt ttgggttgaa aatttttttt 300
tttaagaatg ttgaatattg gtttttattt ttttttgatt tgtagagttt ttgtcgagag 360
attagttgtt agtttgatgg gttttttttt gtgggtaatt tgattttttt ttttggttgt 420
ttttataata gtatttagta tttagtttaa atttaagtat ttttagttgt ttttataaaa 480
aaatgagttt tatagttgtt ttagttaaat taggatttaa tttagaatag tataattgga 540
tgttgcgttt ttttaaattt tttttaattt agaaaaagat aattattttt ttattgtttg 600
gtttgttgaa tagataggtt aattgttttg taaaatgttg taatttttag atttgtttat 660
ttgtattttt atggtatttg tttattttgt ttatttattt ttatttttgt tggaaattgg 720
aagttatagt tgaagatttg attatattta attgaaatat ttttaaatgg atggaatata 780
tgattagtgg atatttatta tttggttatt ttattattag taatattaag attgattgat 840
tttttagttt tttaatgggg ttattagtaa tttaaaaaaa attttgggtt atttgatata 900
ttaaatatat tttaatggga taatttggtt taaattatta tgtgtaatat tgttgtaatg 960
tataagtatt ttagaatttt tagtagtagt ttatttatag attaattttg gagtatagtt 1020
atgtgttttt atgatggaga tatgttttga gaaatgtggt tttatcgttg tgtgaatata 1080
atagagtgta tttatataaa ttagatgata gagtttgtta tatatttaag ttatatggta 1140
tagtttatta tttataggtt ataaatttgt atagtaagtt attgtattaa atattgtagg 1200
tagttgtaat ataatggtat ttgtgtattt aaatatattt aaatatagaa aaggtatggt 1260
aaaaaaaata tagtattata gttttttttt tttttttttt ttaaaagtat attttttaaa 1320
ttttttgtaa tttaggtatt ataattttat gggattacgt gtggtttgtt attgattaaa 1380
atgttatggg gtatatgatt gtgtttattt taaaaataaa ttgtttttag ttagttttaa 1440
ttaaaaatgg atttttaatt aatagggttt gtttttttat ttttagatag gtgtgtttgt 1500
tgttaatagt tttggttata gtattaatta atgagaaggt tgtagtgata gttttaggtt 1560
tttgatatat atttttttaa tttttttgtt tttattttta taattagttt ttgtggtttt 1620
tttttgaaaa ttttaggttt tattgttttt agagatttta attatgaata tatttttagt 1680
aaatattgtg agatttaaaa ttattattgt attttaagtt tgaggatttt gggagttagt 1740
aattgtgaaa tggatttata aatttaaatt aagaaattta aatttagaag aatatttgtt 1800
ttaaggatat atgaaaaaat ggttttttaa gtgttatatt gagtattttt taatttttag 1860
tattttttgt ttatgaaatt aatataatat taatatgatg ttaagataag tttattatag 1920
taatttttta tttagtgagt tatttgaatt agaagttaag tgtgattagg atagtttttt 1980
cgaaatttag aaaatttgta aatttgtatt taatttattt tttaggggaa agaaataatt 2040
tttggaagtt aagttgaaaa gtttagttaa atatattaat tatatgtttt ttgaaagtaa 2100
aaagtatgga atatagagtt ggggtaagta tataaagtat tatttatggg gagaaaggta 2160
attggaggta aagttaattt gattaaaaat tggttcgagt tttttgaagt tatttgtttt 2220
aagtaaagtt gagaaaatga ggatataaat ttttaatagt taaagaatat ttatgttgtt 2280
tttatttaga atgaggtaat tttatttttt tataaaggta ggtttatgtt gattatttat 2340

ataaatatat ttatttatgt ttaaagattt taaaagagat agtaaattta ttttatgaat 2400
ttagagggta gattaatagg taacgtatag aggtagattt tattttgatg ttaggaagaa 2460
ttgattacgg taaaagatat ataatataaa tttattattt taattatttt aaagtgtata 2520
gtttattggt attaagtata tttatattgt tgtgtaattt ttattattat tcgttattgg 2580
aattttttta ttattttaaa ttaaattttt gaaacgtatg aaatagtaag tttttatttt 2640
tttttcggta tttttaagtt tttggtaatt attattttat tttcggtgtt tatgaattta 2700
attattttag gtattttatt taagtgggat aataaaatat ttattttttt ttttatgttt 2760
ggtttatttt atttaatata atattttaaa cgtttattta tgtggtagta tatattattt 2820
ttttttaaag ttgaataatg ttttgtgtta tgtatatgta tttatatata tatatatata 2880
tgtatatatt atattttgtt tatttatttt tttattgatg gatatttgga ttgtttttat 2940
tttttggtta gttttatttt tttggttttt ttgaataatg ttgttatgaa tatgggtgta 3000
taaatatttg tttgagattt tgtttttaat tatttaggta agtattaaaa agtagaattg 3060
ttggattata gggtaatttt atgtttaatt tttggagggt tgttatgttg ttttttataa 3120
ttgttatatt atattttata tttagattgg taatgtataa gggttttaat tttttaatat 3180
ttttgttaat atttgttttt tgttgttttt gtttttgttt tattataatg gttattttaa 3240
taggtgtgaa ggaagaattt ttagtaatta gttttatatt atagtgagat tagttgtttt 3300
aataggtggg ttatgatgaa tgtgttttag taaagattgg atagattgat atatttagat 3360
atgtaggtga tgtattgttt aagtgtgttt ggttatagag tgaataaggg ttgaaattta 3420
gtatatgttt tacgggttaa gatgtgaatt gttttttttg ggaggaagta gtaagttttt 3480
ttttttcgaa aatattgtta gtttgttaag ttatatgttt aaagggttat ttttttttaa 3540
tataaataat ggtatttata aaagttatta gttattttgg ttggttgatt ttttttttta 3600
gagaagttgt aagattagtg aatagggtaa tatttagttt aattttatta gatgattatt 3660
aaggtttttt ttaatggtgg ttttttgtag atttttttga tggttttata aaatggtttt 3720
taaatttttt gatatttttt atattaaggg ttgggtttta tatattttta tttttaattt 3780
gggatttgtg attgtttgat taatagaatt aagtagaaat gatacggtgt tagtttttgg 3840
gtttagtttt taagaaattg gtagttttta ttttttgttt ttggggatat ttatttttgg 3900
attttcgtta ttatgttgtg aggaagttta aattataagt ttttataagt ttttgtggag 3960
agacgtacgt ggagaaaaat taatatttaa tattaagtaa gtgaattgtt ttggaagtgg 4020
attttttagt ttttgttata tggagtaaaa acgagttgtt ttagataggt tttgtttaaa 4080
ttgtagattg ataagtagag taaatgagtg ttgttatttt aagttattac gttttatagt 4140
aatttgttta gttgtagtag atagtcggaa tagtatggga tataatatga ttagtgtttt 4200
aattttatat ttttattatg tgttgatttt agtagtgtat tgaaggattt taaggtaggt 4260
tttttttgag gagattttag tttttattta tatgttatag gtagtggttt ttgtttttta 4320
tttttttttt tatttttaat ttaggataaa gtatttgtcg tgataaagac gttgagattt 4380
attttttgta aggtcggttt ttttattgtt tgaatttttt gaggttttac ggagttacgc 4440
gttggaattt tttatagttt tttttgaggt tttttttttg ttttgggttg gaggtttgta 4500
gtcgtgggat gttggttata agggataaga tagaagtaaa ttatttgatt tagtaaattg 4560
ttgtttattt cggtttttta acggttttta agtttaagag ggagtacgta agttaagtaa 4620
aggcggtagg gaagacggag aagaaattat tcgtgggttt tggttttgtg tttagttgtt 4680
tcgttataga ttaaatttgt ttgtattaag aggatgggtt tttttgtaag gtttttcgga 4740
attttgagtt ttgtttgtta aattttattt tttttttttt atatttttta tttttttttt 4800
taggaaggaa atagttaaaa aagatttttg ttttttaggg tttggaaggg ggcggtagtt 4860
ttgtgttttt tagtggtcgc gttttaggat agttggaagg ttaggacgtt tttgcggttt 4920
tagagtggag tggaaggttt ggagttttgg gaggagacgg ggaggataga ttggaggcgt 4980
gttttttcgg agtttttttt ttcgtgcgag ttttcgcgcg cgcgtatagt tatttcgttg 5040
gtttgacgat tgtggagagg cggtggagag gttttattta ttttattcgg tcgtcgtcgg 5100
ggattggggt tttagcgaga ttttttcggg agaagtagtg tttaggaggt tttttgaagt 5160
cggggaagtt gtgtagtcga agtcgtcgtc gcgtcggagt tcgggatatc ggttattttt 5220
cgcgttattt attttcgtcg gtttcggttt tttttggttt aggcgtcgcg cggattcggt 5280
agttgtttgc gtacgtcgag ttttacggtc ggtgtaagtt ttttttatcg gggattggga 5340
ttggggcggg tgcggggatg gcggagacgt tgtttgggtt tttcggtcgg gagaagacga 5400
gagttgggaa cgttttggtt cgatcgtttt gtagtttggg cgattcgtcg tagtaggggt 5460
tggtatttat ttgttttagg gcgcgcgggg aggcgggcgt ttttcgtgag gggtgcgttg 5520
cggatacgtg tagagttcgg ggaattttat ttttttacgt tagggtttag gattcggagt 5580
tttaggaaga ggaagtttaa tagttttttt tgggtgattt ttttttttta ggtatgtaag 5640
aattttgagg aacgttaagt tttgtggttt gttttagcgt ttttttgtga agttggtttt 5700
atagtttttt tttattggtt tgtttttttt tttttatatt tgggtttggg tatgttaagg 5760
gtagttaagg gtattcggtg tgagggattg aggtgtttgg ggaaattttt tgtgtagtta 5820
gttttggaaa tttgtttgtg gaggtttggg cgttttgttt tgtgttcgtt tttaaaggat 5880
atttgttgtt ttttacgtgt agaaaataag ggttgttttt agataattat tttttgttta 5940
tattatttta atggtttttt tttgatagag ttgggggtgg gaggtatatt aaatttaggc 6000
gttgttttta aggagagttg tttatttttt aattagagag taaaatttta gaaaatttgt 6060
aaaaagtttt tatttttatt tgtttttttt tttatttgta gttgtttatg tgttagttaa 6120
ttgagaaata ttttggtagt tattttatgt aaattgaatg ttttaaattt atgttatttt 6180
gataaggttt gaatttttaa attattaatt ataggtgaat tttgattttg agagttttat 6240
atgttaaatg gttaaagtgt aagcgtaagg atttagtttt tttttttaat aatagttttt 6300

gtatttttta aatttttaga ttttaaatag gaagaatgtt gaatttatag attatgtttt 6360
ttaggtttga atttgagatg ttttattagc gttatgattg attatgagtg atgatttaaa 6420
gtatagtttg atttagattt ttggaaatga gttatagatt tattttggaa gaatttgtag 6480
ttgaggggtt ttttttattt ggttttgtat tttttagtta atgataatat gatatttata 6540
ggtttttgtt aagtattttt tagttttgta gtttaatatg tgtggagagt tgatagagaa 6600
gttattagaa atgatttttg tagtggtgtt attgttggag gaggaaggaa gggggtttta 6660
atgatagttg gtttttatgt tttaagaatt tatagcgttg gttttatttt ttggtataat 6720
tagttaatta agtagattag tatgttaacg tttaaagtat tataggattt ttgatgagta 6780
gagtagtatt tatcgtagtt aggttagttt ataggttagt ttttagtcga ttgtaatcgg 6840
tagagagtta ttttattttt gtaaaggttt tttttttttt atttttaaga aagttgttat 6900
tgaaggtttt tttttatttg ggaagttgga agacgttagt tacgaaatag tataagatat 6960
gaaagtattt tttttttttt ttgtatttgt gatttaaaaa ggaggatgat aagaagtttt 7020
ttttttggag ttttggtgtt ttttttggaa tggaaatggt gttattttat aggtttatta 7080
atttttggtt agatatttat tttagattat aagaagtagg aaaaaattgt aaattgttta 7140
taattaagtt tagttagagt tgataaatat atggtttgtt tttagttttg aaaaattgat 7200
atttttttta tttaattaga gtgagttaag ttttataagt aaaattatgt gttttgttat 7260
tttttggttt tgaaattttg atttaaatat ttttttaaga ttttggtttt tttataagta 7320
aaatggagat attgtttttg tttatttaga gtcgtaaagt ttaaggtaga taatagatat 7380
atatataata agttattata tgtaaatatt attattattt tagagtttag ggggaaattt 7440
ttggggaagg taaaagtatt taatattcgt attttatata tattttatat tttttatgta 7500
atttgaataa attttatttt atttacgtat tggttaaatt taagttttat tgatttttta 7560
agataattgt taaaattttt aaaagtggtt aatattaatt ttgaatatat ttagaaaaag 7620
gatttaattt tattttttat tttttattta tttaattttt ttttttttga gatagggttt 7680
tattttgttg tttaggatgg agtgtagtga ttcgattata gttttttgta gttttaattt 7740
tttggattta agtgattttt ttattttagt tttttaagta gttgagatta taggtatacg 7800
ttattatgtt tgtttaattt tttttatgtt ttgtaaagat agggttttat taagttgttt 7860
aggttagttt taaatttttg ggtttaagtg atttttttgt tttggttttt taaagtgttg 7920
agattataga tatgagttat tatatttaat taggatttaa tttttttaag attaaattat 7980
gatttgggta tatatattat agatacgtat atatattatc gtataaatta gattatgttt 8040
ttatttgaag atttataaaa gtttatagaa aaggaaatat ataaaatatt gaaataggat 8100
gagttatttt taattatttt tgattatttt ttattaaggt tttttagatt ttttggatag 8160
gaaggattat ttgttttttg taagtgattt aaaaataaat aaataaataa ataaattata 8220
ttagatggtt tttgaaaatg tttaattatt tttttgagtt tattgtttta tttaaatttt 8280
ggagattttt ttaaaatatt ttatatataa ataaagtata agaatgagag ataggatggt 8340
ttagtggtta atagtttcgg tttagagttg gattgatttg ttttagaatc gtagtgttgt 8400
tatttattat ttgtggtttt gataagttgt tttatttttt tgggtttttg tttttttatt 8460
tttaaaattg aatcgtggat ttattttata ggattattgt atggattata gggtattatg 8520
tatatatagt attgagtata gtgattggta taaaataaat gtagttatat aggatggtta 8580
aaagatagta tggggatagg tgttttttgg aaattaaaat attatgatta gtttggaaaa 8640
gtttattatt tttattttat ttatcggtaa atatcgtatt gtgatgataa tttttgagaa 8700
atgaaaaagt aaaaaaattt ttgtaaaaaa tttagatttt atatattaat tattaaggtt 8760
tttaaaataa atgtttttat taattatttt atttttaatt gaaattaaaa ttgttagatg 8820
tgttaatggt gttaattaga gtttttatta atattttata atattttaga tggttatttg 8880
tgttgtattt tatttttatg tttattattt attatatgta agatagtata tattttagaa 8940
gatttaattt ttatattcga taattttatt ttatgtttta atattttgta ttttttattt 9000
agtatataat gttttttttt ttttattaaa aaatatgttt gtattttaat ttttgaagat 9060
ttttgtaatt taggttttta ttagtttgaa tgtagagaaa gaagtaatta ttatttaaag 9120
gaaaaattga tatagtttta tttatattat gatgtagata taagtatagt tatgtataga 9180
gatgagtgag agagataaag ttagagagat agggagatgg agatagagtt agagatggaa 9240
atagagatag agatagatag ttatagagac ggaaatggag atagagatag agacggagat 9300
gatagagatg atggagatga tggagatgga gatggagatt gatatagagg agatagagtt 9360
tgttttaata gagttttagt ttttttttgg ttttgttttg ttatagggta atttgatata 9420
tatatatttt atttaattat taaatatatt ttgtatttta attgaattaa taaataatta 9480
ttgattatga tttttttggt aattgggttt tggaataaaa attatttttt tttttgtttt 9540
agg 9543

<210> 66
<211> 9543
<212> DNA
<213> Artificial Sequence

<220>
<223> chemically treated genomic DNA (Homo sapiens)

<400> 66

tttgaaataa aaggaaaaat aatttttgtt ttaaaattta gttgttaaaa agattatagt 60
taatgattat ttattgattt aattaaaatg taggatatat ttggtgatta gataagatat 120
atgtatatta ggttatttta tgatagagta aaattagaaa aaaattgaag ttttattagg 180
gtaagtttta ttttttttat gttagttttt atttttattt ttattatttt tattattttt 240
gttattttcg tttttgtttt tgtttttatt ttcgttttta tagttattta tttttgtttt 300
tatttttatt tttagtttta tttttatttt tttatttttt tagttttgtt ttttttattt 360
atttttatat atagttatat ttatatttat attataatat ggatgaggtt atgttagttt 420
tttttttaag tagtagttat tttttttttt atatttaggt tgataagaat ttgaattata 480
aaaattttta agagttgaga tataaatata ttttttaatg aaagagaaaa ggtattatat 540
gttgagtaaa gggtataggg tattgaggta taaaataaaa ttatcgaatg taaaaattga 600
attttttaaa atgtgtgttg ttttgtatat gataaataat gaatataggg atgaagtgta 660
gtataggtag ttatttaaag tgttatgaaa tgttaatgag aattttggtt aatattatta 720
atatatttaa taattttagt tttaattaag aatgaaataa ttgataaaaa tatttgtttt 780
gaaaattttg atgattaatg tgtaaaattt aaatttttta taggagtttt tttgtttttt 840
tattttttag aaattattat tataatacgg tatttgtcga tgaatgaaat gggagtaatg 900
aattttttta ggttagttat agtattttgg tttttaaggg atatttgttt ttatgttatt 960
ttttaattat tttatatagt tatatttatt ttatgttagt tattgtattt agtattatat 1020
gtgtatgatg ttttgtaatt tatataatgg ttttgtgggg tagatttacg gtttaatttt 1080
gaagataggg aaatagaggt ttaggagggt gaagtagttt gttaaggtta taggtgatgg 1140
gtggtaatat tgcgatttta aggtaggtta gtttaatttt gagtcggagt tgttaattat 1200
taaattattt tgttttttat ttttgtgttt tatttgtatg taaaatattt taaagaagtt 1260
tttagagttt agatgaagta atagatttag gagggtggtt agatattttt aaagattatt 1320
tggtgtggtt tatttattta tttatttatt tttaagttat ttgtagagag taagtggttt 1380
tttttgttta ggaagtttag gagattttaa taaggaataa ttaaagataa ttaaaaatag 1440
tttattttat tttaatattt tatatatttt tttttttgta ggtttttatg aatttttaaa 1500
tgaagatata atttgattta tgcggtggtg tgtgtacgtg tttgtgatgt atatatttag 1560
gttataattt aattttaaaa aaattgagtt ttggttgggt gtggtggttt atgtttataa 1620
ttttagtatt ttgggaggtt aaagtaggag gattatttga gtttaggagt ttgaaattag 1680
tttgggtaat ttagtgagat tttgttttta taaaatataa aaaagattag ataggtatgg 1740
tggcgtgtgt ttgtagtttt agttatttgg gaggttgaga tgggagaatt atttgagttt 1800
aggaggttaa ggttgtaggg agttatgatc gagttattgt attttatttt gagtagtaga 1860
gtgagatttt gttttaaaaa aaaaaaaatt aaataaataa aaagtaaaaa ataaaattga 1920
attttttttt tgagtgtatt taaggttgat gttaattatt tttaggaatt ttgataatta 1980
ttttgggaaa ttagtaaagt ttaggtttgg ttaatacgtg gataaaataa gatttattta 2040
agttgtatag aaagtatagg gtatatataa agtgcgagta ttggatattt ttgttttttt 2100
tagagatttt tttttgaatt ttgaaataat aatagtattt atatatagta atttattata 2160
tatgtatttg ttatttgttt tgagttttac gattttaggt aggtaggagt aatattttta 2220
ttttatttat aaggaaatta gagttttgaa gaagtattta ggttaaggtt ttaaagttaa 2280
gaaatggtag gatatataat tttgtttata ggatttgatt tattttgatt aaatgggaaa 2340
aatattaatt ttttaaggtt agaggtaggt tatgtatttg ttagttttga ttagatttaa 2400
ttataaatag tttgtagttt ttttttgttt tttataattt ggagtgagta tttggttaaa 2460
aattgataga tttatgaaat gatattattt ttattttaaa gggaatatta ggattttaag 2520
agaaaagttt tttgttattt ttttttttgg attatagata taggggggag aagaaatatt 2580
tttatatttt gtgttgtttc gtgattaacg tttttttagtt ttttaaatga aagggaattt 2640
ttaataatag tttttttaaa aatgagagag agaaggtttt tgtagaaatg gaatggtttt 2700
ttgtcggtta tagtcggttg ggaattgatt tgtgaattga tttggttacg gtgggtattg 2760
ttttatttat tagaggtttt gtggtgtttt gaacgttagt atgttggttt gtttggttag 2820
ttgattatgt taggaggtaa ggttaacgtt gtgggttttt aaggtatgag agttagttat 2880
tattaaaatt tttttttttt tttttttaat aatgatatta ttatagaaat tatttttgat 2940
gattttttta ttagtttttt atatatgtta agttgtaaaa ttaagggata tttggtaaag 3000
gtttgtggat gttatattat tattggttga aaagtataaa attaaatgaa agaagttttt 3060
taattgtagg tttttttaag gtgggtttgt agtttatttt taggaatttg agttaggttg 3120
tgttttgaat tattatttat aattagttat agcgttagtg aagtatttta ggtttagatt 3180
tgaaggatat ggtttataga tttagtattt tttttgtttg gaatttgaga gtttggggag 3240
tgtaaaaatt attattaaga aagaggattg aatttttacg tttatatttt aattatttaa 3300
tatatgagat ttttaggatt aaagtttatt tgtaattggt aatttggaaa tttagatttt 3360
attaaaatgg tatgagttta gagtatttaa tttatatgaa atgattgtta aggtgttttt 3420
taattagtta atatatagat aattataggt ggaggaaaaa ataagtaaaa gtaggaattt 3480
tttataagtt ttttgaggtt ttgttttttg gttaagaaat gagtaatttt ttttagagat 3540
aacgtttgag tttaatatgt tttttatttt taattttatt aggggaaagt tattaggatg 3600
atgtaaatag agggtgatta tttagaagta gtttttgttt tttgtacgtg agaagtaata 3660
agtgtttttt aaaggcgagt atagaataaa acgtttagat ttttataggt aggtttttaa 3720
aattagttat atagggggtt tttttaaata ttttagtttt ttatatcgag tgtttttggt 3780
tatttttggt atatttagat ttagatgtgg gggaggaaaa gtaagttagt aagaaaaggt 3840
tgtggagtta attttataag aaggcgttgg agtaagttat agagtttggc gttttttaaa 3900
gtttttgtat gtttggggaa aaggggttat ttagggaaaa ttattgggtt tttttttttt 3960

aaaatttcga attttaaatt ttaacgtgga gaggtgaagt ttttcgaatt ttatacgtgt 4020
tcgtagcgta ttttttacgg aaggcgttcg ttttttcgcg cgttttgggg taagtgggtg 4080
ttagtttttg ttgcgacggg tcgtttaagt tgtagggcgg tcgggttaga acgtttttag 4140
ttttcgtttt ttttcgatcg aggggtttag gtagcgtttt cgttattttc gtattcgttt 4200
tagttttagt tttcgataag aaagatttgt atcgatcgtg gagttcggcg tgcgtagata 4260
gttgtcgggt tcgcgcggcg tttgggttag aggaagtcgg agtcggcgag ggtgggtggc 4320
gcggagggtg gtcggtgttt cgggtttcgg cgcggcggcg gtttcggttg tatagttttt 4380
tcggttttag aaaatttttt gggtattgtt tttttcgggg aggtttcgtt gggattttaa 4440
ttttcggcga cgatcgggtg ggatggatga agttttttta tcgttttttt ataatcgtta 4500
gattagcggg atgattgtac gcgcgcgcga gggttcgtac gaaaaagaaa atttcggagg 4560
aatacgtttt tagtttgttt ttttcgtttt tttttaaagt tttagatttt ttattttatt 4620
ttgggatcgt aagagcgttt taatttttta gttattttgg gacgcggtta ttaaaaagta 4680
taaagttgtc gttttttttt aggttttgaa gggtaggagt tttttttaat tatttttttt 4740
ttgaaagaaa aggtggggga tgtggaggag agagggtaga gtttgataga taagatttag 4800
aatttcgaaa ggttttgtag aggaatttat ttttttagtg taggtagatt tgatttgtga 4860
cggaataatt ggatatagag ttagggttta cgggtggttt ttttttcgtt ttttttgtcg 4920
tttttgtttg gtttgcgtgt ttttttttaa gtttagaggt cgttgaggag tcgaagtgga 4980
tagtagttta ttggattagg tggtttgttt ttattttgtt ttttgtagtt agtattttac 5040
ggttatagat ttttagttta gggtaaagaa ggagttttag gagagattat ggaaggtttt 5100
agcgcgtggt ttcgtgaaat tttaagaaat ttaaataatg aagaagtcga ttttatagaa 5160
agtgggtttt aacgttttta ttacggtaga tattttattt taaattgggg gtgggagagg 5220
agatgaggga taaaggttat tgtttgtgat atgtgagtaa aaattggggt ttttttaggg 5280
aaggtttgtt ttggagtttt ttagtgtatt gttagagtta gtatatggta gaagtgtgag 5340
gttggagtat tggttatgtt atattttatg ttgtttcggt tatttgttgt agttaaataa 5400
attattataa aacgtagtgg tttaaaataa taatatttat ttattttgtt tattagtttg 5460
tagtttgggt agagtttgtt tgggatagtt cgtttttgtt ttatgtagta ggggttggag 5520
gatttatttt taagatagtt tatttgtttg gtgttgagtg ttagtttttt tttacgtgcg 5580
tttttttata aagatttgtg gagatttgtg gtttgggttt ttttatagta tggtggcggg 5640
gatttaagag tgaatgtttt tagagataga aagtggaagt tgttagtttt ttaagggttg 5700
ggtttagaaa ttggtatcgt gttatttttg tttgatttta ttagttaagt agttataaat 5760
tttagattga aggtgagggt atatagagtt taatttttag tgtgaggagt gttaaagaat 5820
ttagggatta ttttgtaaaa ttattaaaga gatttataga aaattattat tgaaagaggt 5880
tttaatagtt atttagtagg gttagattag atattatttt gtttattaat tttatagttt 5940
ttttaggagg gagaattagt taattagaat aattaatagt ttttataagt gttattgttt 6000
atattaaaaa aaggtgattt tttgggtatg tggtttggta agttgataat attttcggga 6060
aagaaaaatt tattgttttt ttttaaaaga ggtagtttat attttggttc gtgaaatatg 6120
tgttggattt tagtttttat ttattttgtg gttagatata tttggatagt gtattatttg 6180
tatatttgaa tatgttaatt tgtttagttt ttgttagaat atatttatta tgatttattt 6240
attgagatag ttgattttat tgtggtgtag gattagttat taaaaatttt ttttttatat 6300
ttattaggat agttattata gtaaaataaa agtaaaaata gtaaagaata agtgttggta 6360
agaatgttga gaaattggaa tttttgtgta ttgttagttt gaatgtaaaa tatagtgtag 6420
tagttgtgga aaatagtatg gtagtttttt aaaaattaaa tataaaatta ttttatgatt 6480
tagtaatttt attttttggt atttatttaa ataattgaaa gtagagtttt aaatagatat 6540
ttgtatattt atatttatag tagtattatt tagaaaagtt aaaaggtgaa aattagttaa 6600
aagatggaaa taatttaaat atttattagt ggaagaatgg ataaataaaa tgtggtatat 6660
atatgtatgt atatgtatat aaatatatgt atataatata gaatattatt tagttttaaa 6720
aaagaatgat atatgttatt atatggatga acgtttaaga tattatgtta agtgaaataa 6780
gttagatata aaaggaaaga tagatatttt attattttat ttagatgagg tatttagaat 6840
agttaaattt atagatatcg aaggtagaat agtgattatt aggggtttgg aggtgtcgag 6900
gggagaatgg ggatttattg ttttatacgt tttagagatt tagtttggga tgatgaaaaa 6960
gttttagtga cggatggtga tgagggttgt ataataatgt gaatgtattt aatgttaatg 7020
aattgtatat tttgaaatgg ttaaaatggt aaatttatgt tatgtatttt ttatcgtaat 7080
taattttttt tgatattaaa atgaaatttg tttttatacg ttatttgttg atttgttttt 7140
taaatttata aaatgagttt attgtttttt ttaagatttt taaatataag taagtgtatt 7200
tatgtgaata attagtatag atttgttttt gtaagagaat aggattgttt tattttagat 7260
gagaataata taaatgtttt ttagttgttg gagatttata tttttatttt tttagtttta 7320
tttggggtaa atgattttag gggattcgaa ttagttttta gttaagttaa ttttattttt 7380
agttattttt ttttttatag ataatatttt atatatttgt tttaatttta tattttatgt 7440
tttttatttt taaagaatat ataattaata tatttgatta agttttttaa tttgattttt 7500
aaaggttatt tttttttttt ggaaagtggg ttggatgtag atttgtaggt tttttgggtt 7560
tcggggaaat tattttgatt atatttgatt tttggtttaa gtagtttatt aagtggagga 7620
ttattgtaat gaatttattt tggtattatg ttagtattgt attgatttta tgaataaaaa 7680
gtgttgaaga ttaagaagta tttaatatag tatttaaaaa attatttttt tatgtgtttt 7740
tggggtaaat gtttttttga atttaagttt tttgatttaa atttgtaaat ttattttata 7800
gttgttaatt tttaagattt ttaaatttga aatgtagtag tggttttgag ttttatagta 7860
tttgttagag atgtgtttat ggttaaggtt tttaggagta atgaaatttg aagtttttag 7920

agaagaatta taaagattgg ttgtgagagt aaaggtagag aaattgggga aatgtatgtt 7980
agagatttga aattgttatt atagtttttt tattggttag tgttgtggtt aagattattg 8040
gtagtaaata tatttgtttg gagatgaaaa gataagtttt attagttgaa gatttatttt 8100
taattaaaat tgattagagg taatttgttt ttgaggtaaa tatagttatg tgttttataa 8160
tattttggtt aatggtagat tatacgtggt tttataggat tataatattt aagttgtaag 8220
agatttgaga aatatgtttt taaaaaaaaa gaagaaaaaa gattgtaata ttatattttt 8280
tttattatat tttttttatg tttagatatg tttggatata taagtattat tgtgttataa 8340
ttgtttataa tatttagtat agtaatttgt tgtataagtt tgtagtttat gagtaatagg 8400
ttatattata tagtttagat gtgtagtagg ttttattatt tagtttgtgt aagtatattt 8460
tattatgttt atataacgat gaaattatat tttttaaaat atatttttat tatgaagata 8520
tatgattgta ttttaaaatt agtttataga tgagttgttg ttggagattt taaagtgttt 8580
atgtattgta ataatgttgt atatggtggt ttagattagg ttattttatt aaaatatatt 8640
taatatatta agtagtttaa agtttttttt aaattgttgg tggttttatt ggagagttga 8700
gaaattaatt aattttagta ttattaatga tagggtaatt agatagtaag tgtttattga 8760
ttatatattt tatttattta aaaatgtttt agttgaatat aattaggttt ttagttgtaa 8820
tttttagttt ttaataaaga tgggaataga tgaataaggt aaataaatat tatgaggatg 8880
taaataaata aatttggaag ttataatatt ttatagggta attaatttgt ttatttaata 8940
agttaagtaa tgaaaagatg attatttttt tttaggttga aagagattta aaggaacgta 9000
atatttagtt gtgttgtttt ggattggatt ttggtttggt taaagtaatt ataaagttta 9060
tttttttgtg gaggtagttg agaatatttg gatttagatt aggtgttaga tgttattata 9120
agggtagtta gagagaaagg ttaggttatt tataaaggga agtttattag attaatagtt 9180
gatttttcgg tagaaatttt ataagttaga agagagtggg ggttaatatt taatattttt 9240
aaagaaaaga atttttaatt tagaatttta tatttagtta aattaagttt tataagtgaa 9300
ggagaaataa aattttttat agataagtaa atgtcgagag attttgttat tattaggttt 9360
gttttaaaag agtatatgaa ggaagtatta tatatggaaa ggaataatcg gtattagtta 9420
ttgtaaatat atgttaaatt gtaaagatcg ttaaggttag gaagaaattg tattaattaa 9480
tgagtaaaat aattatttaa tattataatg ataggattaa atttatatat aataatatta 9540
att 9543

<210> 67
<211> 2865
<212> DNA
<213> Artificial Sequence

<220>
<223> chemically treated genomic DNA (Homo sapiens)

<400> 67

atgatagtta ttaagggatt ggtgggaggg ggaaatagga agttgttgtt taatgtgtat 60
aaatttatag ttatataata tgagtgagtt ttagaaattt gttgaataat ttagtgatta 120
tagttaataa taagattttg tgaatttaaa aatttaaaga gagtagattt tatgtgaagc 180
gttgttatta taaaaataaa ataatgggat ataaggaaaa tttggaggta atggatatgt 240
ttattatttg gtttgtagta atgataacgt gagtgtatgt atatatttgt ttaaatttat 300
taaattgtga atattaatta tatgtagttt tttggatatt agttgtattt taataaagtt 360
gagggggaaa gtgatattat tgtaaataaa taatttggtt ttatgacggg tggagaaatt 420
attttagaga gtgtattagg gaagattttt ttaaggagaa gatttgagaa ggaagtgagg 480
attttaataa agtgagagat taatataggt agataattag gatagattat tttagtttgg 540
gagaggtggg aatgggggaa gaagtttagt aagaagaagt ttttgagaaa aggtttagta 600
tggtatgttt taggatttgt aaagaggttg gtataggtgg atatgagtgg gggtagggta 660
agaaaggaag ttggagagtt tattagaatg gattatttag ggttttgtag gtaatggtaa 720
tgagttagaa atgtatttgg tgtttatttt ttaaattata tttggaaata tatatgaatt 780
ttatttagtt attgggagat attggaaagt tttaagtagg ggaatgattt gaattgattt 840
agatttgtat ttggttttgt tggttagggt gtggagaatt gattatagat atagtaagag 900
tgtagggaga tattttagaa tattttttgt tttttttggt tatgtagatt tggatatttt 960
tcggataagg tacgaatatt ttattttagt agtttattgt ttgttttttt tttatttatt 1020
ttgtttaatt taaatttaag attttgttat ttttttgttg tattaattta agttgttttt 1080
tgttaatgga gagtaattta tatatttata gttgatttat atttattgga gtttatttaa 1140
gggaggatat tattttttat attgttttat gtttaatttt tgtttttgaa gaaagaataa 1200
gtaaaaatta aaaggtagaa atgaaattta ttggtagata gtttggtgtt atattttggg 1260
tttggtagtt aaagatcgat ttttgattta atcggttatg ttatttatag attttagata 1320
ttgtatggaa aagtattgtg aaaatttttg ttttgttttg ttttgatttg attattagtg 1380
tatgtagttt ttagttatgt atttgttgtt tgtttaatta attataattt ttttacgtag 1440
atttttttag agttgtgagt ttttaaaagg gataggaatt gtttatttag agagtttagt 1500
ttttgagata ggagttttgt cgatgttttt ggtcgaataa attttttttt tttttaattc 1560
ggtattcgag gaattttgtt tgtagtttgt tttgttatat tttttggttt tttgattggg 1620

aagtgaggtg attggtggat ggtcgaggta gttttttagg tgatttaagt ttgttttgtg 1680
gaatatttcg gtgggggatt ttggttagtt cgagtaacgt ggattttgag agtattttta 1740
ggtaggtatt tgtttcggtg ggacgttttg ttagagtagt gtgtggtagg ttttcgtgga 1800
ggattaatat agtggttgaa tattgggaag gaattggtat ttggagtttg gatatttgaa 1860
atttggtaag attagttttt ggaatttgtt tattttattt gagtagaagc gtggtttgtt 1920
tatttacggt gtgtttttat tggtattttg gttttggttt tggttttgat ttggtttgaa 1980
ttgtttgata ggattggttt tgggaatttg tttattttat ttgagtggaa gtatggtttg 2040
attatcgatg gtgtgtttgt atcggtattt tggtttttgt ttttgatttg atttggattg 2100
tttgatattt tggttttgtt tttgatttgg tttgaatttt tggatatttt gattttggtt 2160
ttgatttttg tttggtgtaa attgtaaaag tgtgtgtgtg ttttttttat cggttttttg 2220
ttttgtggtg tgtatgtggt gtgagcgtgg tgttttgttt taaagaagta tgggttaggt 2280
ataaataagt ttattttatt aggaattatg ttgaaaaatt ttaaaaaggg atttaaggga 2340
gattatgata ttaggaaaat ttagaatttt atgtaaaata gattggttag tattagaggt 2400
aggttggtta ttagaaggaa gtttggaaag gttttttgtt ttaaaggtat ggtataaggt 2460
aatttgtaaa gttagggtat ttagattagt tttcgtatat agatgtttgg ttattgttgg 2520
ttttagattt ttttatagtg gttgagagaa ttgtagtata agtggttggt agaggtagag 2580
agagagagag agagaggtag agaggagaga gagagataga gaagagaaag atagagatat 2640
ataagtagtt aagaaaaaaa gtgtatttta tttttttaaa agttaaggta aatttgaaat 2700
ttataattga taattgaagg tattttttat aattttgtaa tattttaata ttattttgtt 2760
gttagtgtaa ataagggcgt attttaaaag tattgaggtt tttttattaa aaatttttaa 2820
tttagtagtt cgtggatggt ttaaatgtat ttaatttgta gtagt 2865

<210> 68
<211> 2865
<212> DNA
<213> Artificial Sequence

<220>
<223> chemically treated genomic DNA (Homo sapiens)

<400> 68

gttgttatag attgaatgta tttgggttat ttacgggtta ttgggttaag gatttttgat 60
aggaaggttt tagtgttttt gggatacgtt tttgtttata ttgataataa agtagtattg 120
gagtgttata ggattatgga gaatattttt aattattaat tataggtttt aaatttattt 180
tggtttttaa aggaataggg tatatttttt tttttaatta tttgtatatt tttgtttttt 240
ttttttttgt tttttttttt tttttttgtt tttttttttt tttttttttg tttttgttag 300
ttatttatgt tgtaattttt ttaattattg tgaagggatt taaaattagt agtaattaag 360
tatttatgta cgggaattgg tttgggtgtt ttggttttat aggttatttt gtgttatatt 420
tttgaaataa gggatttttt taggtttttt tttgatggtt aatttatttt taatgttggt 480
tagtttattt tatataaagt tttaagtttt tttggtgtta tagttttttt taaatttttt 540
tttgaaattt tttaatatag tttttagtag ggtgggttta tttgtgtttg atttatgttt 600
ttttgaggta aaatattacg tttatattat atgtatatta taaaataaag aatcggtaaa 660
aagggtatat atatattttt atagtttata ttaaataaaa attaaaatta aaattagagt 720
atttagaaat ttaagttaag ttaaagataa aattaaagta ttaagtaatt taagttaaat 780
taaaaataga aattaaagtg tcggtatagg tatattatcg gtgattaggt tatgttttta 840
tttaaatgga gtaggtaagt ttttaagatt agttttgtta agtaatttaa attaagttaa 900
aattaaaatt aaaattaaag tgttaataaa ggtatatcgt gggtgagtaa gttacgtttt 960
tatttaaatg gagtgggtaa gttttaaaga ttagttttat taagttttag atgtttagat 1020
tttaagtatt agtttttttt tagtgtttag ttattgtgtt gattttttac gggggtttgt 1080
tatatattgt tttggtaagg cgttttatcg gggtaaatgt ttatttggga gtgtttttag 1140
gatttacgtt gttcgggttg gttagagttt tttatcggaa tgttttatag ggtaggttta 1200
agttatttaa ggagttgttt cgattattta ttaattattt tattttttag ttagggaatt 1260
aggaaatgta gtaggataag ttgtagataa aatttttcgg atatcgaatt aaagaaagaa 1320
gaggtttatt cggttagaag tatcggtaag atttttgttt taagagttga gttttttgag 1380
tgagtaattt ttattttttt taagggttta tagttttaag ggggtttgcg tgaaagggtt 1440
gtgattgatt gagtaagtag taggtatata attgggggtt gtatgtattg gtaattagat 1500
tagaatagaa taggatagga atttttatag tgttttttta tataatgttt ggaatttata 1560
gataatataa tcggttaggt taggggtcga tttttaatta ttagatttag ggtgtggtat 1620
tagattgttt gttagtggat tttatttttg ttttttagtt tttatttatt ttttttttag 1680
aggtagaaat tgggtataag ataatatgag gggtggtatt tttttttaaa tggattttaa 1740
taaatgtaag ttagttgtgg gtgtatggat tgttttttat tagtagggag tagtttaggt 1800
tgatgtagta gaaaggtggt agagttttag gtttgagtta ggtaagatag atgaggaaaa 1860
ggtagatagt gaattgttaa gatggaatgt tcgtgtttta ttcgagaaat gtttaagttt 1920
gtataattag gagagataga ggatgttttg agatgttttt ttgtattttt gttatgttta 1980
tagttaattt tttatatttt agttagtaag attaaatgta agtttaagtt aatttaagtt 2040

atttttttgt ttaagatttt ttaatgtttt ttagtagttg aataaagttt atatatattt 2100
ttaaatataa tttggaagat aaatattaaa tatattttta atttattgtt attgtttata 2160
aggttttaaa tgatttattt tggtaagttt tttaattttt ttttttattt tgttttattt 2220
tatgtttatt tatattagtt tttttgtaga ttttgaaata tgttatgttg aatttttttt 2280
tagggatttt tttttgttga attttttttt ttatttttat tttttttaga ttgaaatgat 2340
ttattttagt tgtttgtttg tattgatttt ttattttatt agggttttta tttttttttt 2400
aagttttttt tttggagaag ttttttttag tatatttttt aaaatagttt ttttattcgt 2460
tatgaggtta aattatttgt ttatagtaat attatttttt tttttagttt tattgaggta 2520
tagttagtat ttaaaaagtt gtatgtaatt aatgtttata gtttggtggg tttggataag 2580
tgtatatata tatttacgtt attattatta taagttaggt aataaatata tttattattt 2640
ttaaattttt tttgtgtttt attgttttgt ttttatggta ataacgtttt atatgagatt 2700
tatttttttt aagtttttaa gtttataaaa ttttattgtt aattatagtt attgggttgt 2760
ttagtagatt tttggaattt atttatgttg tataattgta aatttatata tattgaataa 2820
taatttttta tttttttttt ttattaattt tttggtaatt attat 2865

<210> 69
<211> 6412
<212> DNA
<213> Artificial Sequence

<220>
<223> chemically treated genomic DNA (Homo sapiens)

<400> 69

ttttttataa tattgttttg ttaaattcga agcgtttgaa tattgtttgt atttttttgt 60
ttttatgttt ttaggttttt gttgtttttt ttttgttata cgtttatttt tttttatatt 120
tggtaaaata tttatatttt aaagggtatt taaagtcgtt taaaagttat tgtttttgtg 180
atattgggcg aattataata aaattatttt atataatgtt gatttgtata aatttaaatt 240
atatgaatgt ttttttgaag agataaagtt atattattta acggtaagtg tatagttaat 300
aatatagttt tttaaatttt gtttaagtat agtaggtttt taacgttgtt tggttgaacg 360
ttatttcgtt ataatggtga tgagaaaaaa aatcgatttt aggttatggt tattgtttgt 420
gagtagtttg tatatttttt ttatgtttgc gtgggttttt ttcgggtatt ttggtttttt 480
tttataattt atagttgtgt tcgttaggtt gtgtttaagt tgtgtatata tttaaatatt 540
tttagtatgc gtgagtgtgg gtgtgagtgt gagtgtattt taggatagaa gggtgttttg 600
tttagggtta tttttttttt tgagttttga atttcggtta tttgtaattt tgaaatggaa 660
aaattggtta tatgatttta tttgttttta ttaatttttt ttaaatgtat gtttagttta 720
tatttatttt aatgtttaat attagaagtg ttttggtttt tatttggaag tttggtgatg 780
tttttgtgat cggagatatg tataggaatt taatttttgt ttgtattaag tagtttatgg 840
tgaaattgtt ttttttatat atgttatttc gtttaaagtt ataattttta agaatatatt 900
tttgatatta aggatttatt gtatttatta ttttgttgat tagtaatttt tatattaata 960
attatttagg tatataattt tagagttatt tgatttaggt ttatgaattg ttttatatag 1020
atatttgagt tatgttatga atagtcgcga attttaatat tttattttaa ttttatgaat 1080
tattttatgg aagaggataa tgtatatttt tttttttgtt taatatggga ttatttaaat 1140
tttaaatagt agagatattt aaatataagg atatttatat ttttgtgata taattgaatt 1200
tttttatttg gttattgtat gtgttttaaa cgggaaatga gtggatatta tttttttttt 1260
tttatttttt ttttagtttt gatgttatag gaagttagtt tgttagtttt ttgttatttg 1320
atagtttttt gattaaaaag attttttagt gaggattagt attttatttt agataggagt 1380
cgtgaatttt ttttttaaag atatatatat tttttaaaag ggtttagaat attttttgta 1440
aaattgtttt tgttatatga gtatttatta gagttattat aagaaaagtt aaggaaataa 1500
atttattttt tttgtaatag aaaaaatata tattttaaaa tgggttaaga atttgaatat 1560
tagtaagatt atagattagg gaaaaaagta aataatattt taattgaaaa tttgatttga 1620
aaaataaaag tttatttatt ggttttttta tttttttttt tatttttttt ttattgttgg 1680
aattttgtga aatatataga tgatggagga gtatttgtgt cgatattttg aattttaatt 1740
tttaataatt aggtagatgg ttggtttgtg gtaaagttta ttattgtata gattgtaatt 1800
tgtatttagt tataaaatat aagttatttt tttaggacga ttttaggatt tttttagata 1860
ggaaatatta ttgtaattga aaaacgattt aatttttagt taagagttta taatatttag 1920
gtattttcgt gtttttatga attgtttttt tttttttcga gtttttattg gttatagttt 1980
tagatttttt attaaattta attcggtttg agcggtaggt aatgtttttg ttgggtgcgg 2040
attgtaggtt tattattgga gggcgtttgg acgtttttaa atagttttga agcgtttgga 2100
aatttcgttt gaggattgga aagcgtagga gggtgaacgt gagatttgat tttattgggt 2160
taataagtaa aaaggagtgt aggtaggtgt gggagtaaaa gtaaattgtt tttttttttt 2220
tggggatttg ttttttttat taaggtagaa aattttttag ggaatatgag aaaaaatatc 2280
gttgttttcg agttcgcgga ttttttaagg gcgggtttcg ggggtttgtg gtttcgtaga 2340
gtaggcgtcg ggtttatttt ttgggcgttg gacgatagtg gggacgcgtt tcgaggcgtt 2400
gggatagttt cgttgttagg agatcgtttt ttagtggcgg ttgaagatta ggtttgtaat 2460

ttgtttttga agtttaggat cgaggggttt tttttaataa taaaggaatt gttttttttt 2520
ttatagtttt cgttttcggt ttttattttt tttcgtatcg ggttcgataa aggcgtttat 2580
tttttgggga gtgggtagta aattattttt aaggttgaaa tggaagaggt gtgaggttat 2640
ttttgaaggg ttcgtttgtc gtatttattt tttttttgtg gaaggttcgc gttgaattgt 2700
aattatttta aattgtgatt tgtttgattt ttaaaaacgt gtttttagag tattatttta 2760
aagaaaattt gttttttgtt taagaatttt taaattttaa gtgggaagag gagggttgaa 2820
aggtatagga ggggtgttta gatttttaga taatttagcg ttaggtagcg tgcggcgtgg 2880
agagcgggtg gaatggatag gaacgcgtat tttttgaggt ttcgttttag tttattcgtt 2940
atttgcgggg tattagagat agatgtcggt tttttagtta aagggtgggt ataggttttt 3000
ttaaaatcga aagattttat ttttaggtaa aaagatgtta aattattttt tagtttagac 3060
ggaatttagt atttaaaggg ttatacgtgg ttgtcgggtt tcggcgtagg agagggcgtt 3120
tcggtatttt tagtaattta attagttagg tttttagaag gtggggcggg ggagggggta 3180
ggaggcgtgg ggaagggaga ttaggaaaac gttttttttt cgtggagtaa attaattcgg 3240
ttggagtaaa gagagaggaa aaatttttaa tttgtaagta aatagtaggt cgttttttga 3300
ttggttgttt tagttatttt tttatcgcgt ttttattttt tttaagtaaa ttgtttggaa 3360
ggaggaaaaa aaaaaaaaaa aaaaaaaaga tagtttatat gattcgggtc gtagcggtag 3420
tagcgttggt tgggcgcggg ttggcggtat ttttttttgt ttcgttcgga gtttatattc 3480
gcgttcgcgt cgttcgttcg tttcgcgttt cgcgtaatac gttcgggcgg agttttattt 3540
tttcgtttac gtttacgttt acgcgtgcgg tttcgcgttt tgttgaggtt gttgttattg 3600
ggtcgtcgtc gttgtcggtt ttttacgtaa gttattaacg agggggcgtg ttcgaggaga 3660
tttttttttt tataaagtat tttttttttc gttttttttt tttttttttt ttttgtaaaa 3720
ttgattttta taaaattttt tttatttttt gttagtcgtt ttaatttttt ttgggttgtt 3780
cgtgttgttg ttgttgtatt taagtttatt tattttttcg tttgggtagg ggttttagga 3840
ttcggtttgt ggttttattt tttgttgttg ttgtgtttaa ggagggaaag aggagtagtt 3900
gcgggaggat cgggaaggtt ttttgatgta gttatcgggg aggtgttatt cgttcgtgcg 3960
tttttttttt tttttttttt tttattttaa attgattgat gtatttttat aagttgttag 4020
gtttggattc gttggagtcg tagggatatt tttgagagtt tatttcgggt ttttggtatc 4080
gcgtgtgggg gtttagtttt tttttcggaa ggcgatcgga gattgatatc ggtttattta 4140
ggagttggag ggatttattt gaaagtgatt gttttttttt tcgtttttta gtcgaatagt 4200
tgggttggcg tcgttttgta ggtttaggaa ttttagtttt gttaggtcgg ttggttttaa 4260
tttttgtttt ttatttattg tggtgtattt atatttagtt aaggaaagta cgcgcgagaa 4320
attttcgagg ttcggttagt tttcggggtg ttttttttcg ttatcgtcgc gtttcgattt 4380
tgttggggga tttgtcggaa gacgcgaaga gttaaatttt tttttgaggt tttcgaggta 4440
agatcgtttc gaaagtcggc gtagttgtcg tttaattagt tttaggatat cgataaggtt 4500
gggttgcggt ttcggatttt tatttttttc ggcgtatcgt tcggtatcgt agtatttacg 4560
atttttttga gatttttggg ggtcgtttcg ttaaggtata gaggaggttg aggaaagggg 4620
tttttagatt ttcgggatta taagcgcgaa gttttttatt agtacgtcgt tttttttttg 4680
tagtttttta tcggtcgtta aagtaatttg gagcgaagcg tggcgtagtg cgagggtttc 4740
gggcggtggc ggagtcgtgc gtagtttgtt gcgtcgacgt tgagtcgggt ttcggcgggt 4800
cggtgtatgt tttttcgcgg ttgcggcgcg taattttcgg tgattgtgta gcgcgcgtcg 4860
tcgtttgttt cgattcgcgt tgtagtcgtt cggttcggcg cgtatttttt tttttcgggg 4920
tgatcggttt gtcgtcgtcg ttcgagatat agtttggtat tttttttttc gttttcgtcg 4980
tcgttattat atatacgtac gttttttttt attttgtgat tttttttttt ttttgaattt 5040
tttagtgggg gtgcgagttt gtttttatta ttttttattt tatcgttttt tttttttttc 5100
gtttttttat ttttttttag tttggtgggc gttttttttt tttttcgttt ttttttattt 5160
ttatttattt atatttattt ggcgttcgtt ttttttttgt ttttttgttt gttttttttt 5220
tttcggattt tcgttttttt ttcggagtgg cgcgtcgggg gtttcgtcgt tggttaggcg 5280
tgatgttgta cgtggagatg ttgacgttgg tgtttttggt gttttggatg tgtgtgttta 5340
gttaggattc gggttttaag gtcgtcgtcg atcgttacgt tgtttattgg aatagtagta 5400
attttaggta agtcgataat aggcggagag ttagggttat tcgggtttcg gggttttgcg 5460
gaaggagcgg cggcggggat cggcgcgtat taatcgttgg cggcgcgtat tttcggagtt 5520
tgcgtcgttc gggcgttgat ggtttgaggc gtcggcggga agcgttcgcg tcgcgttttt 5580
ttttgttaag tcgtcgcgga aaggttaggt atttttggga gttttcgggg ttgtttagat 5640
taattgtttt tgcggggcgt cgggagattc ggaaaagggc gggagtcggg gagtagggat 5700
ttttttgttt tttagtttcg gtatttggtt ggtattcggt tttcggcgcg gggagttttt 5760
agtgtgcgta gtcgggagtt tttttttgga gcgtgggttc gtagggattg cggagagaat 5820
tcgttaaata ttttttaaag tttttttttt tttttttttt taattttaat tttttttttt 5880
tttttttcgg tgcgcgtaga gagagcgtac gggttggggg aagtgagggg ggttgggaag 5940
cggaagtttt aggtttttat tttttttttt aattgcgagg aaattattcg gtttttgagt 6000
tagtttgggt tcggcgcgtt tggtagtgga gaggattttt ttattttttt tattttttta 6060
tttattttag tgggtttaag gtgttagaaa gtttgattta atgtcgggtt acgttatgtg 6120
tgtagaataa gttgttagag ttggtcgagt taggaggtgg gagtttttcg ttcgcggttt 6180
ggcgatgtgt tgtcggagga acgcgcgcga ttatttggaa tcgatttgta ggcgtttcga 6240
agttcgtcgg gtatgcggga gattagaggt gggcgtcggg ggcggtttta gtgcgcgtgt 6300
tggatatcgg ttagtcgtag acgtttcgtg ttttcggatt ttggaagtgg agtggggagg 6360
ttatttagag tgttcggcgg ggtttttcgg gtttggagta gtttcgaatt tt 6412

<210> 70
<211> 6412
<212> DNA
<213> Artificial Sequence

<220>
<223> chemically treated genomic DNA (Homo sapiens)

<400> 70

aaggttcgga attgttttag attcggagga tttcgtcggg tattttggat aattttttta 60
ttttattttt agggttcgaa aatacgggac gtttgcgatt ggtcggtgtt tagtacgcgt 120
attaggatcg ttttcggcgt ttatttttag tttttcgtat attcggcggg tttcgggacg 180
tttataaatc ggttttaggt aatcgcgcgc gttttttcgg tagtatatcg ttaggtcgcg 240
ggcgaagggt tttatttttt tggttcggtt aattttggta gtttgttttg tatatatgac 300
gtaattcggt attagattaa attttttggt attttgaatt tattggggtg ggtggaggga 360
tggagaagat gggagaattt tttttattgt taggcgcgtc gggtttagat tgatttaggg 420
gtcgaatgat tttttcgtag ttgaagagga aaataaaaat ttaaagtttt cgttttttag 480
ttttttttat tttttttagt tcgtgcgttt tttttgcgcg tatcgggaaa agagaaggga 540
aaattaaaat taaaaaaaaa aaaaaaaaaa aattttgggg gatgtttggc gggttttttt 600
cgtagttttt gcggatttac gttttaggaa ggagttttcg gttgcgtata ttgggggttt 660
ttcgcgtcgg gaatcggatg ttagttagat gtcggggttg ggaagtagag aagtttttat 720
ttttcggttt tcgttttttt tcgagttttt cggcgtttcg tagaagtagt tggtttaggt 780
agtttcgggg atttttaggg gtatttggtt ttttcgcggc ggtttaataa agaggggcgc 840
gacgcgagcg tttttcgtcg acgttttaag ttattagcgt tcgggcgacg taggtttcga 900
gggtgcgcgt cgttagcggt tggtgcgcgt cgattttcgt cgtcgttttt ttcgtagggt 960
ttcgaggttc gggtagtttt ggtttttcgt ttgttatcgg tttatttggg gttgttgttg 1020
ttttagtaga tagcgtagcg gtcggcgacg gttttggagt tcgggttttg gttgaatata 1080
tatatttaga gtattagaaa tattagcgtt aatattttta cgtgtaatat tacgtttggt 1140
tagcggcgga gttttcgacg cgttatttcg gggagagagc ggggattcgg agggagggag 1200
gtaggtaaag ggatagagag agagcgggcg ttaaataaat atgaataaat aaaaatgaaa 1260
gggggcgaga aaggaaagag gcgtttatta agttggggag gggtaggaga gcgagaagaa 1320
aagaaggcgg tgggatgggg ggtgataaag ataaattcgt atttttattg gagggtttag 1380
gaggaaaaag gaattataag atggagagaa gcgtgcgtgt gtgtggtggc ggcggcgagg 1440
gcgggggaag aggtgttaag ttgtgtttcg ggcggcggcg gtaggtcggt tatttcggga 1500
gagggaggtg cgcgtcgggt cgggcggttg tagcgcgggt cggggtaggc ggcggcgcgc 1560
gttgtatagt tatcgggagt tgcgcgtcgt agtcgcgggg agatatatat cggttcgtcg 1620
gggttcggtt tagcgtcggc gtagtaggtt acgtacggtt tcgttatcgt tcggggtttt 1680
cgtattgcgt tacgtttcgt tttaagttat tttggcggtc ggtgggaaat tgtaggagaa 1740
gggcgacgtg ttgatagagg gtttcgcgtt tgtggtttcg ggagtttgag aatttttttt 1800
tttagttttt tttgtgtttt agcggggcga tttttagaaa ttttagagga gtcgtaagtg 1860
ttgcggtatc gggcggtgcg tcgagaaggg tggggattcg gagtcgtaat ttagttttgt 1920
cggtgttttg gaattggtta agcggtagtt gcgtcggttt tcgaagcggt tttgtttcga 1980
aaattttaaa gaaaagtttg gtttttcgcg tttttcggta ggttttttag tagagtcgga 2040
acgcggcggt agcggaagag ggtatttcgg gggttggtcg agtttcgggg atttttcgcg 2100
cgtgtttttt ttggttgggt gtgggtgtat tatagtgaat gaagagtaga ggttaagatt 2160
agtcggtttg gtagagttgg gatttttaaa tttgtaagac gacgttaatt taattgttcg 2220
gttggagagc gggaggggaa ataattattt ttaaatggat ttttttaatt tttggatggg 2280
tcgatgttag ttttcgatcg tttttcgaga gggagattga gtttttatac gcggtgttaa 2340
gagttcgggg tgagttttta gaagtgtttt tgcgatttta gcgggtttaa gtttagtaat 2400
ttgtagagat gtattaatta gtttgaaatg ggaaaagagg aaaaaaaaaa agcgtacgag 2460
cgagtggtat tttttcgatg attatattag aaaatttttt cgattttttc gtagttgttt 2520
tttttttttt ttttaggtat agtagtaata gaggtgaaaa ttataaatcg aattttaaaa 2580
tttttattta ggcgggaaaa taaataaatt tgagtgtagt agtagtagta cgagtagttt 2640
aggaggaatt gaaacgattg gtaagaagtg gaaagggttt tgtaaaagtt agttttgtaa 2700
aagaggggga aaaaaagaga gcgagagaaa aaatgtttta tgggaaaaaa aattttttcg 2760
ggtacgtttt ttcgttaatg atttgcgtgg aaagtcgata gcggcggcga tttagtggta 2820
gtagttttag tagagcgcga ggtcgtacgc gtgaacgtgg acgtgggcgg ggggatgggg 2880
tttcgttcgg gcgtgttgcg cgggacgcgg gacgggcggg cggcgcgagc gcgggtgtga 2940
gtttcggacg aagtaggggg aggtgtcgtt agttcgcgtt tagttagcgt tgttgtcgtt 3000
gcggttcggg ttatgtggat tgtttttttt tttttttttt tttttttttt ttttttaagt 3060
agtttatttg ggaagggtgg gagcgcggtg gggggatagt tggagtaatt agttagggaa 3120
cggtttattg tttgtttata gattgggggt tttttttttt tttttatttt aatcggattg 3180
gtttgtttta cgagaaagag gcgttttttt aatttttttt ttttacgttt tttatttttt 3240
ttttcgtttt attttttaga aatttgatta attgaattat tgggagtgtc gaggcgtttt 3300

tttttgcgtc ggagttcggt agttacgtgt aattttttgg gtgttgaatt tcgtttgggt 3360
taaggaatga tttgatattt ttttatttgg gagtagggtt tttcgatttt aaaggaattt 3420
gtgtttattt tttggttgga gagtcggtat ttgtttttgg tgtttcgtag gtgacggatg 3480
ggttggaacg gagttttagg gagtgcgcgt ttttgtttat tttattcgtt ttttacgtcg 3540
tacgttgttt ggcgttgggt tatttggaag tttgagtatt ttttttgtgt tttttagttt 3600
tttttttttt atttgggatt taaagatttt tgggtaggaa gtaagttttt tttaaagtga 3660
tgttttgaaa atacgttttt aaaaattagg taaattatag tttaaagtga ttataattta 3720
gcgcgaattt tttatagaga ggaaatgaat gcggtagacg agttttttag aaataatttt 3780
atattttttt tattttaatt ttgagggtga tttgttgttt attttttaaa gaataaacgt 3840
ttttatcgaa ttcggtgcgg aaagaaatga gggtcggggg cgggggttgt gggaagaaaa 3900
gtagtttttt tattgttgga aaaggttttt cggttttgga ttttaagggt aggttgtaag 3960
tttggttttt agtcgttatt gaagagcgat tttttggtag cgagattgtt ttagcgtttc 4020
gggacgcgtt tttattgtcg tttagcgttt aggaagtgag ttcgacgttt gttttgcggg 4080
gttataggtt ttcgagattc gtttttaagg ggttcgcggg ttcgggagta gcggtatttt 4140
tttttatgtt ttttagggag ttttttgttt tagtgggaag ggtagatttt taggagaaaa 4200
aaggtagttt atttttgttt ttatatttgt ttgtattttt ttttatttat tagtttaata 4260
aaattaaatt ttacgtttat ttttttgcgt tttttagttt ttagacggga tttttaggcg 4320
ttttagagtt gtttagaagc gtttagacgt tttttagtgg tgggtttgta attcgtattt 4380
agtaggggta ttgtttgtcg tttaggtcgg gttgaatttg atgggaaatt tagggttatg 4440
attaataaag attcgagagg aaaagaaata gtttataggg gtacggaggt atttggatgt 4500
tatgggtttt tggttaaaag ttaagtcgtt ttttaattat agtaatattt tttatttgaa 4560
aagattttga aatcgtttta ggaaagtaat ttatattttg tgattagata taaattgtaa 4620
tttgtataat aatggatttt attataaatt aattatttgt ttaattatta aaaattgaaa 4680
tttagaatgt cgatataaat gtttttttat tatttgtatg ttttataaag ttttagtagt 4740
gagaaagaag tggggaaaga agtgagagaa ttaatggata agtttttatt ttttaaatta 4800
aatttttagt taaaatgtta tttgtttttt tttttggttt gtaattttgt tgatgtttaa 4860
gtttttggtt tattttaggg tgtgtgtttt ttttgttgta ggaaagatgg atttgttttt 4920
ttagtttttt ttatagtggt tttaataggt gtttatatga tagagatagt tttgtaggaa 4980
atattttgga tttttttaag agatatgtgt atttttaaaa agaaaattta cgatttttgt 5040
ttagggtaaa atgttgattt ttattgaaga gtttttttag ttagggaatt attaggtaat 5100
agggagttag taagttggtt ttttgtggta ttaaagttga aaaaaaggtg aagaaaaaga 5160
aatggtattt atttattttt cgtttgaaat atatataata attaaatggg aaaatttaat 5220
tatattataa aaatatggat gtttttatat ttaaatattt ttattattta aagtttgaat 5280
agttttatat taaatagaaa aaaaagtata tattgttttt ttttataaag tgatttataa 5340
gattggaata agatgttgaa attcgcggtt gtttataata taatttaaat gtttatgtga 5400
aataatttat aaatttaaat taaatgattt tgggattatg tgtttaagta attattggta 5460
tggaagttat tagttagtag gatgatagat atagtaagtt tttaatgtta gggatatgtt 5520
tttggaaatt gtgattttaa gcgaagtggt atatataagg aaaataattt tattataggt 5580
tatttgatat aaataagagt taaattttta tgtatatttt cggttataaa aatattatta 5640
aatttttaga taaagattaa aatattttta atgttaaata ttgaaataaa tgtgagttag 5700
atatatattt gagaaagatt aataaaaata agtaagatta tgtaattaat ttttttattt 5760
taggattgta ggtggtcgga gtttagggtt taaggaggaa agtaattttg gataggatat 5820
ttttttattt tggggtgtat ttatatttat atttatattt acgtatattg agaatattta 5880
gatatgtgta tagtttagat ataatttagc gagtatagtt gtgggttgta ggaggaaatt 5940
agagtattcg gagaaaattt acgtagatat gaggagaatg tgtaaattat ttatagatag 6000
tggttatggt ttggaatcga tttttttttt tattattatt atgacgaagt gacgtttaat 6060
taaatagcgt tgaggatttg ttgtatttga atagagtttg aagagttata ttattaattg 6120
tatatttatc gttagataat gtgattttgt ttttttagga aagtatttat gtgatttaaa 6180
tttatataaa ttaatattat ataaaataat tttattataa ttcgtttagt gttatagaag 6240
taatgatttt taaacgattt taaatgtttt ttagaatgta agtattttgt taggtatagg 6300
aggaggtgaa cgtataataa aaagagaata gtaagaattt aaaaatatgg ggataaaaaa 6360
atgtagatag tgtttaaacg tttcgagttt gataagataa tgttgtaggg ag 6412

<210> 71
<211> 7070
<212> DNA
<213> Artificial Sequence

<220>
<223> chemically treated genomic DNA (Homo sapiens)

<400> 71

tttttttttt tttcggcgaa agagaaaggg gttaaattta aattcgggtt tgggtttggg 60
ggtttgggta ggatttagtg taagtataaa aagcgggatg ttagattaaa gtgaagaaaa 120
ataaataggg aattgtatgt agtttttttg tttttcggag tagcgaagtt taaaattttt 180

agttttagag gtttgtttgg ttgtgtgata tagttttagc gaggtttgaa atttcgaggg 240
gggttagtga aggttttttt tttcgtaatt cgagttaagg ggttttgcgt cgggttttcg 300
gatattttcg cgtagttcga aggtcgtttg taaattcgcg agcggcgata gttcgggtgt 360
ttgtattttg gcgttttttg cgttcggcgc gggtttgtgg atagtcggcg gggtttagag 420
aggaaattag ggttttagga gcgttttttg aattttggtt atcgggtgta atgtatagaa 480
attattgata tttttttatc gtttttcgcg gggagttaag aggagaaagg ttttagtttg 540
tttgagtttg agagagagag aggagagaga gaggtattat ttttagaata aaacggttta 600
tgttgtgaat tgttttagat aagagggaga aaattttggg ggaagatcgg atgatgggga 660
tataaattta aatattgaaa tttacgaagg tgataatttt taatagtggg ttttttttcg 720
ggttttttag atatttagat atttttagtt ttattttata tatatagaaa aaaagaaatt 780
tttttttgttt tttatttttt ttaaggaatg ttttgagttt tttataaata aagttattgt 840
tttaatttta ggagttaacg gagtatttta ttttgttagt agagcgtttt atttttaatg 900
gtttgttttt tatgtatttt tcggttatcg gttttttttt tttgttgttt tttttagttt 960
tattttattt tggatagaat tgtttaggat ttttaattaa tatttaaacg aatttttagt 1020
atttatttat ttttatatta ttatgggttt tagatgaaga tcgaaatcgc gagtattaat 1080
tgaattagtt tattgaaaaa gattgttagg tataatgacg tgattcgagg ttttatattt 1140
tttaaggttt ataggttttg gggatttgcg atgtatagtt tgattttgta gtttataata 1200
acgcggtatt tgtagtagaa attttttcgt atttattttt aataaaatcg ttggttattt 1260
agttttcgta ggaaggttgt acgttattgt gttgcgataa tttatttatg tttcgtatta 1320
cgaggtttta tcgttatttg atttatattc gaaatgcgat tattattttg tgtatgtaat 1380
atgtttttat ttttagttta tggcgtatat ttttcgttag gttgtaagaa tttttttcgt 1440
aatttttttt ttggttttta ttttcgaaag agaggtggaa ataagtagga ttttggtatt 1500
tgtttttgtt tatataaaat ttaaaatttt atggatatat aggttggttt ttttttttga 1560
atagtatata ttaaaatttt tagtaaatat atttttttat gtttgattag tggggttgat 1620
ttgggtgtat atattaatat ttatatatat atagggggttt tttgttatat ataattatat 1680
atatatatat atatatatat atatatatat ataatatttt tatagattaa tttttttttt 1740
gggtggtttt tatatattaa gtaaataaat ataaatatat atttgttttt ttatagttac 1800
gggttatttt ttaagatcgt tgagcgtttt ttttaaaatt agaaaaggag agaaagaaag 1860
gtaaaatttt ttagttttta ttttgataaa tatatgttag tttttcgatt atatgatttt 1920
taaatttttt tagttagtag ttttttaaat aagtatgtat tatttttttt tgtttggtta 1980
ggtgtttttt gtttattggg agtttttgtt tatttttaga aaatgtagga agtttaagga 2040
ttggagaagg gaaggacggt tggggttttt ttttgtttta tttatttaat tttcgattat 2100
tgaaggtttc gttgggattt ttttttttta agtgaaaaaa tgtagtattt tttttttaat 2160
tttggtattt gagataaaat agaattttta ttttttttgt cgttttttat ttttatttgg 2220
gttattttta agaaaagggt atttggggat aattaattta ttgtagagac gtagtttagt 2280
ttatggtttt ttgtgcgttt ttagtttagg tgattgtata gtttagaatt ataaggttat 2340
agaaatacgt attgggtatt tttatatttt tttattttcg gtttaagagt agatggtatt 2400
tggagaaagg ggttttattt tatttgagtt aggttttttt atttgttatt tgtaggttta 2460
tttttttgtt atttcggttt gggtatttat taagttgaaa gtaatttttt agtttttaaa 2520
ggtagaaacg agtattttta aaaggatcgt tatataaata tatttattcg tgtatatata 2580
attttttata tatattttaa ttttttattt atatatttgt atatataatt tattttgttc 2640
gtagtttttt atttatacgt ttaatttggg gtttatttag agttttgtga aatttttatt 2700
tatttttttc ggttaatatt tgtttattta gtttttatat attttattcg tatgtttgat 2760
aagacgttaa tgcgtttata tagagttttt aatggtatgt ttatttgtag gagggagaag 2820
agggcgtagg gtttgggtgg aggttatgga gcgtagagcg gcgcggggta tcgagcgttc 2880
ggtttaggtc gggtttcggg gaggtggggg gacgagttag cgcgtagagt gtcgtttaat 2940
ttagttcgag ttcgtgggtc gcgggtgtat ttattatttt attattgtaa taaattcggg 3000
ttttattata tgaaaggtaa aatgaattat cgacgttaag tcgttaataa agttattttt 3060
gtaaatggtg tttttaaagg gtcgtcgtat tatttagttg gttttattag ttggttggaa 3120
attacgtgga ggagtcgggt cgcgcggcgc gcggggtcgt ttattttgat taagcgtttt 3180
gttagcgcga tgtgatagag tttttgatta gggttttatt tataggtttg tgatgaacgt 3240
taagttgatt aggcggaatg aagagtaatt aaaatttata aattattttt cgtagttttt 3300
ttcgaggcgt tttttgtagg cgagataagg cgtcgagatt ttatttatcg cgttgaaagg 3360
gatcgcggcg tataaaagtt acgtagttaa ataggatatt gatagtgttt taattagaat 3420
ttaattaagt atttacgttt gtttgcggga taaagatttt aattttgcga atttaaatat 3480
aaataaaatt ttatttttat ttcggggaga aggggtgggg gcgtttggag atttcgggga 3540
gtttttgggt gtgttattga agaggattcg ggagatttta aggtggggtt ggaggcggtt 3600
tgggaaggta gagaagattg tagcgtagcg ttagggagag aggttttttt ttttaggttg 3660
tagcgtaagt ttagtcggga atttagtttc ggaaatcggg ggattttcgt ttgtttttaa 3720
gatgcggtga gtaatataaa tttttgtttt ttattagttt ttttgagaaa ttttagttat 3780
tgtggtggtg gttcggtttg ttgtgggttt agttgtatgt tttttttagt ttaatttttt 3840
gtgatttttt tggaaaattg gaggtagggg gattttttcg tatgttataa atagataata 3900
tttttaaagg gatttttttt ttattgtttt agttgcggag ggcgtttagg gcgaggaggt 3960
tcgttggagt tttaagatga tattttcgaa ggtttcgttt gttgacgttt ttttcggtta 4020
tcgtaggttt tagtatagtc ggatattggt tttttaaaac gaattcggag ataagtttcg 4080
taggtgttta gagttttagt ttatacgtta ttttcgtttt ttatattttc ggttatgaga 4140

aaattttggg ggtgtttcga ttaggaaata gcgaaaaaga gatcggtaga cgggagaatg 4200
atagtagttg gaggaacggt atttttttta aattagggaa gaacgtatag agttggagtc 4260
ggaggtattt agggagttgg gagtaatttt aggaaattta gttattcggt aattgaggga 4320
aattttttgt ttataatttt agttagttag ggtaaatatt tggggtgtag cgtttttatt 4380
tatttgattt tattttgtag ggaaatttaa tatagaggtt attttgaatt ttaaaagtta 4440
gggtttagcg cgtattcgcg ttttgattcg tcggtttcgg ggtttttttg agattaattt 4500
gcgttgttcg gatttttatt gttagggaag gtttaggtaa ttggttttgg ggttattttt 4560
agttttttgg gtttggtttt atggcgttag ggtagtttta gggtgttgtc gaaagtttgg 4620
atcgggaagg gtatgggtgc ggatttttta taaagggaaa gtaatgtagc gggtcggtag 4680
gagacgtata aacgtaaacg tgcgacgtta gttaggcgta gcgggttttt tagattttgt 4740
tatttgtgaa aaataaattg cgtttttgaa agtaattaat tttaggttta ttttatatta 4800
tcgatttttt tgttttattt tttttttttt tattatatat atttaaattt atatttattt 4860
ataaatatat aaatcggtag tgataataat tatttatttt ttaataatag taattagaga 4920
tagaggagaa aataaaaagt tgagtttttt aggcgtgggg gtgtaaaata gttaggtttt 4980
tgtttattgt ttttgttttc gtagtttata gatttataat tttgtatttt tttaataagt 5040
tgtttatgga gttgtttttt tatttgttta tattttaaaa tttatttttt tcgggttttt 5100
tttgtttttt tttgagtttc ggtttgaagg aggggagggg acgcgggtgc gggcgcgggt 5160
gggggagggc ggattcgacg tatagggtta gcgtcgaggc gttttttttt cgttagcggt 5220
tgacgttttc ggattattta ttcgtaaagt ttcgcgcgcg tttattgggt cgaggttcga 5280
gtgttagcgc gagtttcggt tcgttattgg tttcgtatac gtgcggtttt gatttacgtg 5340
ttttcggttt gaaggtaaaa agtgtgtttg ggtgattttt tttttaagcg agagagtttg 5400
tgtaaagatt cgagttgtta gagatttgaa aaaaaaaaaa aaaaaaaaaa aaaaaaaaat 5460
agttcggcgt tggcggagac gcgttttttt tgtaaaaaaa gtaaaggcga ttaaaggcgt 5520
tgttagtttt acgttttggg tatagttgag cgtgatattc ggggaagtta aattttttat 5580
tattgtttag gaagatggtt agattttaaa tattattttt ttttttttaa gaaaaaaatt 5640
attggagttt ttttttttgt tttttttttt tttttttttt tttttttttt ttttattttt 5700
ttggtcgtgg tttatttttt atttaaatta aattattgaa tttggttgta gaaagaaaaa 5760
agaaatagtt aagtgttttt atatttggat gtttataaat tagagaggga gagatagcga 5820
gatttatttg ttagataaga acgagcgatt taggttagac gtttgagttt tttttttgta 5880
ttcgtttcgt gttttcgttg aggtttcgtt tgtttttttt tttcgcgtat ttttacgggt 5940
cgttggtaaa gtggggtggg gagcgaggcg gtgggggcgg gggcggcgcg gcgtcggggc 6000
ggcggggcgg tcgagtatgg aagaatagta gtcggaattt aaaagttagc gcgattcggt 6060
tttcggcgcg gcggcggcgg cgatttcggg cggttttagt ttgagtttta gttcgggcgt 6120
tagcggtagt agcggtagcg gtagcgatgg agatagcgtg tcggtgtttc gtagtttgat 6180
tttttttgtt tgtcgttttt ggtttattat tcgtattttt ttttacgttt ttcgttttcg 6240
tcgttttagt atttcgcggc gtttgtttat tagtcgtagt tagcgttagt tgtatcgtat 6300
tattaatttt tttatcgata atattttgag gtcggatttc ggttgtaaaa aggagtagtc 6360
gtaacgtagt ttttggtggt tgcggcggtt agaggaggcg taggaggagg aggtcgggtc 6420
gagcgtgata gaggttagat tgtcgtaggt agagattttg tttattcgtt gggtattcgg 6480
cgtttagcgt gtttcgtttt tgtgcgtttc ggacgcgaat tgtggtttat tcgacggttt 6540
ttagttagtc gcgtcgggcg ggcgcgttta aagttgggaa ttcggttgcg gcgcggcggc 6600
ggcgcggcgg tagtggcggc gcggcggcgg tcgtagtagt taagttttcg gatatcggtg 6660
gcggcggtag atggtaggcg gcgcggggag tttcggagcg tagggtatta aatattcgga 6720
gtacggtaat tcggttattt tatttatggg tttagtaacg gcgggttcgt ggttaaaatt 6780
gattcgtagt agtttttcgt atggttcgtt tgggtgtatt gtatacgtta ttcggatcgt 6840
ttatttttcg gtgagtattt aatttcgggt tgcgttatgt tttttggttt cgcggatcgt 6900
tttagaacgt taagttgcgg cgttagggag agtaaattcg gtttcgggta ttttagtttt 6960
ttacgtttgt ttttttattt tagttttagt tataatcgaa tgtttattta gttatgcgat 7020
tttggttttt gcggttatta ttcgagaagt tatagttcgt ggtataggag 7070

<210> 72
<211> 7070
<212> DNA
<213> Artificial Sequence

<220>
<223> chemically treated genomic DNA (Homo sapiens)

<400> 72

tttttgtgtt acgagttgta gtttttcggg tggtggtcgt agaggttagg atcgtatagt 60
tggatgaata ttcggttgtg attggaattg gggtgaggaa gtaggcgtga gagattggag 120
tattcgaggt cgggtttgtt ttttttagcg tcgtagtttg gcgttttggg gcggttcgcg 180
gggttagaag gtatggcgta gttcggagtt gggtatttat cggaggatgg acgattcgaa 240
taacgtgtgt agtatattta ggcgggttat acgagaggtt gttgcgagtt agttttgatt 300
acgggttcgt cgttgttgag tttataagta ggatagtcgg gttgtcgtgt ttcgggtatt 360

tggtgttttg cgtttcgggg tttttcgcgt cgtttgttat ttgtcgtcgt tatcggtgtt 420
cgagggtttg gttgttgcgg tcgtcgtcgc gtcgttattg tcgtcgcgtc gtcgtcgcgt 480
cgtagtcggg tttttagttt tagacgcgtt cgttcggcgc ggttggttgg gagtcgtcgg 540
gtgggttata gttcgcgttc ggggcgtata ggagcgaggt acgttaggcg tcgggtgttt 600
aacgggtgga tagggttttt atttgcggta gtttggtttt tgttacgttc gattcggttt 660
tttttttttg cgtttttttt ggtcgtcgta gttattagaa gttgcgttgc ggttgttttt 720
ttttgtagtc gaagttcggt tttaggatgt tgtcgatgaa aaagttggtg gtgcggtgta 780
gttggcgttg gttgcggttg gtgagtaggc gtcgcgagat gttgaggcgg cgggggcggg 840
gggcgtgggg ggaggtgcgg gtggtgggtt aggggcggta ggtaagggga attaggttgc 900
gggatatcgg tacgttgttt ttatcgttgt cgttgtcgtt gttgtcgttg gcgttcggat 960
tgaggtttag gttgaggtcg ttcggagtcg tcgtcgtcgt cgcgtcgagg gtcgagtcgc 1020
gttgattttt aggtttcggt tgttgttttt ttatgttcgg tcgtttcgtc gtttcggcgt 1080
cgcgtcgttt tcgtttttat cgtttcgttt tttattttat tttgttagcg gttcgtgggg 1140
gtgcgcggag gaaggaggta ggcgaagttt tagcgaaggt acggggcggg tgtaggaaaa 1200
aagtttaggc gtttggtttg gatcgttcgt ttttatttag tagatagatt tcgttgtttt 1260
ttttttttta atttgtagat atttagatat ggagatattt ggttattttt tttttttttt 1320
tgtaattaga tttaatgatt tgatttaaat ggggagtaag ttacggttaa aaaaatgaag 1380
gaaaaaaaga aaaagaaaag gaaaaagaaa gtaagaaaaa aagttttaat aatttttttt 1440
ttaaagggaa aaaaatagta tttaaagttt agttattttt ttaggtagta gtgaggggtt 1500
tgattttttc gagtgttacg tttagttgtg tttagagcgt gaggttggta gcgtttttaa 1560
tcgtttttgt tttttttgta gggagagcgc gttttcgtta gcgtcgggtt gttttttttt 1620
tttttttttt tttttttttt tttaaatttt tgatagttcg gatttttgta taaatttttt 1680
cgtttaaaaa aaaaattatt taggtatatt ttttgttttt aaatcggaag tacgtgagtt 1740
agggtcgtac gtgtgcggag ttaatggcga gtcgggattc gcgttgatat tcgggtttcg 1800
gtttaatggg cgcgcgcggg attttgcgga taaataattc gggggcgtta atcgttggcg 1860
gagagggggc gtttcggcgt tggttttgtg cgtcgggttc gttttttttt attcgcgttc 1920
gtattcgcgt tttttttttt tttttaggtc gggatttagg agggggtaga agaaattcgg 1980
gaggggtgaa ttttgggatg taggtaaatg gagaagtaat tttataaata atttgttaga 2040
gaaatgtagg attatgggtt tgtgagttac gggagtaggg gtagtaggta ggagtttggt 2100
tgttttgtat ttttacgttt aagaaattta gtttttattt tttttttttg tttttggttg 2160
ttgttattga aggatgggtg gttgttgtta ttgtcggttt gtgtgtttgt gggtgggtgt 2220
gggtttgggt gtgtgtagtg gagggagggg gagtgagata gggaggtcgg tgatgtgaaa 2280
tagatttaga gttggttatt tttagaggcg taatttgttt tttataaata gtaaaatttg 2340
aaaggttcgt tgcgtttagt tagcgtcgta cgtttacgtt tgtacgtttt ttgtcggttc 2400
gttgtattgt tttttttttg tggggaattc gtatttatgt ttttttcggt ttagattttc 2460
gataatattt tggagttgtt ttggcgttat agagttaagt ttagagggtt aggaatagtt 2520
ttagagttag ttgtttggat tttttttggt agtgggagtt cgggtaacgt agattaattt 2580
taggagggtt tcggggtcgg cgagttagaa cgcggatgcg cgttaggttt tagtttttag 2640
agtttagggt aatttttgtg ttagattttt ttgtaaagta aaattaaata aatgaaagcg 2700
ttgtatttta agtatttgtt ttggttagtt gaagttgtga atagggagtt ttttttaatt 2760
gtcgggtgat tgaatttttt gggattgttt ttaatttttt gggtgttttc ggttttagtt 2820
ttgtgcgttt ttttttagtt tgagggaaat gtcgtttttt tagttgttat tattttttcg 2880
tttgtcgatt tttttttcgt tattttttgg tcgaggtatt tttaaaattt ttttataatc 2940
gggaatgtga gggacggggg tggcgtgtgg attgaagttt taggtatttg cgggatttgt 3000
tttcggattc gttttggagg attagtattc gattatgttg aagtttgcgg tggtcgagaa 3060
aggcgttagt aaacgaggtt ttcgggggtg ttattttaga gttttagcgg attttttcgt 3120
tttgaacgtt tttcgtaatt gggatagtag agaaaggatt tttttggaaa tattgtttat 3180
ttgtgatatg cgaaagaatt tttttatttt tagtttttta gaaaagttat agagggttag 3240
gttaggagga atatgtaatt aaatttataa taagtcgaat tattattata gtagttgaag 3300
ttttttaaaa gggttggtgg agagtaggaa tttatattgt ttatcgtatt ttgggagtag 3360
gcgagggttt ttcgattttc ggggttgagt tttcggttag atttgcgttg tagtttaggg 3420
gagaaaattt ttttttttaa cgttgcgttg tagttttttt tattttttta agtcgttttt 3480
aattttattt tggagttttt cgagtttttt ttagtagtat atttaagggt ttttcgaaat 3540
ttttaggcgt tttatttttt tttttcgaa atgggggtgg gatttattt atatttaagt 3600
tcgtaaaatt gaaattttta tttcgtaagt aagcgtgggt atttaattaa attttaatta 3660
ggatattatt aatattttat ttagttgcgt agtttttgtg cgtcgcggtt ttttttagcg 3720
cggtaaatag ggtttcgacg ttttatttcg tttgtaggag acgtttcgag aagggttgcg 3780
gaagataatt tataggtttt aattattttt tatttcgttt gattagtttg gcgtttatta 3840
taggtttgtg aataaaattt tggttaaaag ttttgttata tcgcgttggt aagacgttta 3900
attaaagtga gcggtttcgc gcgtcgcgcg gttcggtttt tttacgtaat ttttagttag 3960
ttgataaagt tagttgaata atacggcggt tttttggaga tattatttat agaaatgatt 4020
ttattgacgg tttaacgtcg gtaatttatt ttatttttta tgtagtggag ttcggatttg 4080
ttatagtaat gggatgataa atgtattcgc ggtttacgag ttcgagttgg attaggcggt 4140
attttgcgcg ttggttcgtt tttttatttt ttcgaggttc ggtttgggtc ggacgttcgg 4200
tatttcgcgt cgttttgcgt tttatgattt ttatttagat tttacgtttt tttttttttt 4260
ttgtaaatga atatgttatt gggaattttg tgtgagcgta ttggcgtttt gttaagtatg 4320

cgagtgaagt gtgtgagggt tgagtgaata aatgttggtc gagagggatg ggtgagggtt 4380
ttataggatt ttgagtgagt ttaggattgg gcgtgtgagt gaggaattac gggtagggtg 4440
agttgtgtgt ataagtatgt gagtaaaggg ttagggtgtg tgtaaaggat tgtatatgta 4500
cgagtgggta tatttgtgtg gcggtttttt tgggggtatt cgtttttgtt tttgggagtt 4560
aaggaattat ttttaatttg atagatgttt aggtcggggt ggtagagagg tgggtttgta 4620
ggtagtaggt ggggaagttt gatttaaatg gaatagagtt ttttttttta aatgttattt 4680
gtttttgggt cgaaggtagg gaagtgtggg ggtatttagt gcgtattttt atggttttgt 4740
agttttgagt tatgtagtta tttgagttag aggcgtatag aaggttatgg attgggttgc 4800
gtttttgtaa taagttgatt gtttttaagt attttttttt tggggatagt ttagataaaa 4860
gtggagaacg ataagaaaga taaagatttt attttgtttt aaatgttaaa gttgggggag 4920
gggtgttata tttttttatt tgaaggaagg aggttttaac gaggttttta gtgatcggga 4980
gttgggtaaa taggatagag agagatttta atcgtttttt ttttttttag tttttggatt 5040
ttttgtattt tttgaggata ggtaggggtt tttagtgagt aaaaggtatt tggttaaata 5100
gaggagaatg atgtatattt gtttgaggag ttgttagtta ggggagtttg gggattatat 5160
agtcggaaga ttggtatgtg tttattagag tgaaggttga gaaattttat tttttttttt 5220
tttttttttt aattttggga aaaacgttta gcggttttga agagtggttc gtggttgtgg 5280
ggaatagagt gtatatttgt gtttatttgt ttggtatgtg agagttattt agagagaaaa 5340
ttagtttata aaagtattgt gtgtgtgtgt gtgtgtgtgt gtgtgtgtgt gtgtagttgt 5400
gtgtggtagg aggtttttgt atgtatgtga atgttaatgt gtatatttaa gttagtttta 5460
ttagttaggt atgaagaaat gtatttgttg agggttttgg tgtatgttgt ttagaaaaga 5520
agattagttt gtgtgtttat agaattttgg attttgtgta agtaaagata aatgttaggg 5580
ttttgtttgt ttttattttt ttttcgagga tgaggattag aaggagaatt acgaaagaaa 5640
tttttgtagt ttggcgaggg gtgtgcgtta tgggttggga gtaggagtat gttgtatgta 5700
tagggtggta atcgtatttc gagtgtagat tagatggcgg tggggtttcg tggtgcggga 5760
tatgaatgag ttgtcgtaat ataatagcgt gtagtttttt tacggggatt ggatgattag 5820
cggttttgtt gggagtgaat gcgaggagat ttttgttata agtgtcgcgt tattatgagt 5880
tataaggtta gattatatat cgtaggtttt taaagtttat ggattttggg aaatgtgggg 5940
tttcgagtta cgttattgta tttgatagtt ttttttaata gattaattta attagtgttc 6000
gcggtttcgg tttttatttg gagtttataa taatgtgggg gtgggtgggt gttggggatt 6060
cgtttgggta ttggttaggg attttaagta attttgttta aagtggagtg ggattagggg 6120
aggtagtaaa ggaaaaaaat cggtggtcgg ggggtgtatg gggggtaaat tattgaaaat 6180
aaaacgtttt gttgataaaa taaaatgttt cgttgatttt tgaaattggg gtagtagttt 6240
tgtttgtaga ggatttagag tattttttga agggggtgag ggatagggag gatttttttt 6300
tttttgtata tatgaaataa gattaaaaat gtttaggtat ttagaaagtt cgagaaaaag 6360
tttattgttg gaggttgtta ttttcgtgga ttttagtatt tggatttatg tttttattat 6420
tcggtttttt tttagagttt tttttttttt atttaaggta gtttatagta tggatcgttt 6480
tgttttagaa atagtgtttt tttttttttt tttttttttt taagtttaga taaattgaga 6540
tttttttttt tttgattttt cgcgggaaac ggtggaaaga tattaatgat ttttgtatat 6600
tgtattcggt ggttaggatt tagaaagcgt ttttggggtt ttggtttttt ttttgagttt 6660
cgtcggttgt ttataggttc gcgtcgggcg tagagggcgt tagggtgtaa atattcgaat 6720
tgtcgtcgtt cgcgggtttg taggcggttt tcgggttgcg cggggatgtt cgggagttcg 6780
acgtagggtt ttttggttcg gattacggga agaggagttt ttattggttt ttttcggaat 6840
tttaaatttc gttgggattg tattatatag ttagataggt ttttggagtt gagaatttta 6900
gatttcgttg tttcgggaag taagaggatt atatataatt ttttatttat ttttttttat 6960
tttaatttga tatttcgttt tttatgttta tattgaattt tatttaaatt ttagaattta 7020
gattcgaatt tgggtttaat tttttttttt ttcgtcgaaa aaaagaaaaa 7070

<210> 73
<211> 10652
<212> DNA
<213> Artificial Sequence

<220>
<223> chemically treated genomic DNA (Homo sapiens)

<400> 73

cgttattata gaggttgttg tgttttaggg gattaattga tgtgagatat ataaaatttt 60
gtaattttat aatataaatt atagtatagt ttttttggag agggttggaa tatttgagtg 120
agttttcgag aggaaaagag gagtttttta gaggagaaat agagtatttt tataatgtgt 180
tttaattgag aaattttgtt ttattgagtt tttttttaag tggaattaga agtgttggga 240
tgagagggaa aggatgggag tgcgtttaaa ggtggatagt aggtttttat ttttggtggg 300
agtgagattg gacggtattt ttcggaaagg tggtttgggt tttggataag gttagaggta 360
ggagtttatg atgtagagat gatatagtgt tttttcgcgt gtgagtttac gaaggttatt 420
attgaggttt tgtgtttgta aaaggtcgtt acgttttata taagttttta tatttaatat 480
agggattgat tgggtatagg gattttttta tattatatat gtaagtatgt atgttaatta 540

aagatgttcg tgttaaagaa acggttaatt ttgttgaatt tagaggaatt gattaattat 600
ttaattaagt agaggaaatg tttgaattta attcgtaatt tagttgtttt tttatataaa 660
attatatatt tttatttata tttgatgaat gaaaaaagaa attagtttat gattttaatt 720
taaatatatg tttttaaaaa tatatttttt ttagtttagt tagtatataa attaattgag 780
tttttttggt taagtatgat ttagttgtga tatttaagag cgggagtggt tgtttagata 840
tttttttttt tacgtgaaat ttagattaat gagttattat ttaataagtt gtaggtagtt 900
tggttgggtt ggatttagat ggtttgagtt aaatttagat tgtatttgtt taaaattttg 960
taaattttta gttacgtaaa ttttattttt aaaaatgttg ggaagttatt gtataagagt 1020
ttaagttata ttagtttttt tgtgattatt tgtagttttt ggggaaagaa tagaagaaaa 1080
gaaaatgtta gtttttgtga ggtggggttg gtgttttagg ggtttttttt gaatattttg 1140
tttttttttt aaaggttaaa aggaaggtag tggatatata ttagaatttt ttttatttgt 1200
gaatggtcgt aaggttggag aaggtggtta gtgtatttta aggtttatta tttttttttg 1260
tgtttttttt tttgttttgg taggtttagt tagtttaagt tttgggtgtt attttttaaa 1320
tttttttgtt aaattaattt tatttatttg attggattat ttgagaggtg ttattttttt 1380
ttgggttgtt gtgattttga gggggtattt ttataagagt ttaggtatta ggtggtgaaa 1440
tagtttgtgt ttttaaattt gtttttttta gggttttcgg gagattttag agtgtaggtt 1500
tgtttgggga gttttagggg tgggttttga gtggaagtgg gtttattttt tataggagtt 1560
taaattttat aggaataaga atagtagtaa aataggataa gagagtaggt agggagttgt 1620
taaggaaagg cgatttttgg gaaggtaggt tatttagaat aaggtttttt tggcgttgga 1680
gagtcggaag ggggagcggg tatagaggat ttggtttagg tgtgggggtt attagtataa 1740
gtagagttat tttttagatt tttttttaga agtagttgtt ttttagagaa attaggtgag 1800
ggatagtttt tgtattttta tacgttagtt ttggagattt gtttatttgt ttttagtcgt 1860
tttttttttc ggcgagtttt gggttaagta ttaagttagg atagaagggc ggtttttagt 1920
cggtttttgg tttattttgt ttttttaata tttagggagt ttgggtttag tataggcgtt 1980
tttttagcga tcggggtaga attaggacgt gtaatgcgat tgtttttttt ttttcgtttt 2040
tcggtagagt ttttgttcgc gttaatttat ttattaggtt ttgttcgcgt tacgcgcgtt 2100
tttggtaggt ttggggcggg gaaaggcgaa gcgttgggta cgcgagggtt tgtgtatttt 2160
agtttttacg acgtttttgg ttttttttag gttcgtcgtt gtcgtattta attttttttt 2220
ttcgggggtt attttgaaga agttttagat tttagattta gttttttaga tttcgttttc 2280
gagttcgttc ggtgggtttg taggtcggtt ttttttcgtt tagaggagag cgtagatacg 2340
taatgttcgt tcgttggttt cgttcgtttt attttgtttt ttcgcgtttt ttcgtttcgg 2400
tttttgttta taggtcgggt cggaacgtta gttttaggag tcgacggtgg ttttttgttc 2460
gtttcgggga aggtgttgtt tttttatcgg ttttaatttt tcgttcggcg tttggggttt 2520
ggttgcgggg ttcggttttt agtcgagggc gtagggttgg ttaggtttgt tttggttgag 2580
gtggagattt cgtttttagg gattgttggg cgtttttgtt tcgcgagtaa cgagatcgtc 2640
gcgagcgaac ggtttttatt gagtttattt ttttaagtgt tattacgtta agttagagaa 2700
gcgaggcgag tggaggggac gtagaggggt cggaaaagtt attttttttt ggtttcgttt 2760
ttagataaaa acgggagttt ggttcggtat tcgggcgttc ggtgttttcg gggcgtttta 2820
ttgaggtttt gtttgtaaaa ttagcgttcg tgttttgaag cgtacggcgt ttggaagttg 2880
tttttttgtt cgtttttttc gaggtttttt tttgtgtagc gagtttgaga aatatggagg 2940
atcgtttttc gtaagcgggt ggtcgcgggc gtttttcgat ttttgggtga agttagaggg 3000
aaaacggggt tttcgggtta gtgttttatt ttttatttcg ggagaaatta atgttcggag 3060
agggttcgtt tgattcgtat agaaaggtcg attttgaggg cggcggtcgt tcgggggaga 3120
aagcggaggt tttgggttcg cgggagcgcg gtagtcgggg ttggtatcgt agaggagaga 3180
tacgttattg tttcgtattt ttagaaagcg cgaggcgtcg ttttagttgg ggtaggcggt 3240
cgaggtcggt ttttatgcgc gtttttcgaa gttttttgaa atattttgcg gagtttcgtg 3300
tgtatagaat ttagtatcgt ttagtttttg ggtagtttaa ttttttgtag ttttatttta 3360
gttttttcgg ttttttgaac ggtgattttt tttatcgttt ttttagagtt gtttcgtgtt 3420
tgggtttatt ttggggggtt cggtattttt tagttatttt tttttttatt tatttttttt 3480
tttttatttt ttttttttat tttttatttt tttttttttt aggagagcga tttacgaatt 3540
tttttttttt tagttaattt tagggtttag ttcgtagatt ttgcgagggt aggtttttgt 3600
ttatcggttc gttaggcgtt ttggaggtga cgttttgttt tttagagttt ttgttgtagt 3660
tacgaattgg ggtttgggtc gtaggaaagt atagggttga agtttagcgt tttggggtta 3720
tttatattga ggtagttaga ggtaaagagt tttaagaatt tagaaaatat tttttaggaa 3780
gtcgtttaat tggtttttat gggataggtg gagttattaa tttgggatgg ttttgtagga 3840
attaaagagt ttagggtttt tttttttttt aatattatgt ttaggagatt tagagtcgtt 3900
ggattttttt ttttttgatt ggtgattatt agagttttta gagttgtaga aaattttttt 3960
tttaaaaaat taagtaagcg ttaataagat tttttataaa tttttattag ttttattttt 4020
tcggggggta ggtagattgt ggggtttgat tttttgagat tcggggagga tttttggtag 4080
atgtgtgttt agttagaata tttggtaagg atttttttaa tgaagaaaaa gtggaggaat 4140
ttagttttag cgagaagagg tttttttatt ttgttttaga tatatcggat agagggtata 4200
ttttgattag agttacgttt agtggttagg aggttagttt agtatttttt tttttattat 4260
tttcgttttg ggtggggggg taattttttt gggagtagtt gtgggaattg tcgtttttta 4320
ttttagttta gttagtattt tgaagtttgt aggggaagga tagtacgtgg gatggatatt 4380
ggggaaggag tttcgtaagg ttagggtgta atttttaggt tttaggtggt ttggtaggtt 4440
acgttgtttc ggagatgttt gttagatttt ttaagtttat ttagggtttg gtagtaattt 4500

gttggttgtt tttgcggggg tttgggttgt tgagtacggt gtagttgttt agggttaatt 4560
agtttagggg tgttcgtgtt aggttgcggt tttttcgttt ttttcgtatt gagggtattt 4620
atggcgtgta aatgttttcg tatttttaga gttgttttat cggatgtttt taggaattta 4680
tatattttta taaaaatgta ttttaaatga tggataggcg agtttggggt aataacgggt 4740
gtttggtggg tagataagag taaatgggaa ggagttcgag ggaggagggg gaagagaaga 4800
ggaaatagaa tttttagttg gatattttga taatagttgg aaggaaagtt tagaaaagat 4860
gaagagagag gaggggagaa attaattggg gtttttattt ttgtcgttgg atttttaatt 4920
ttcgttttaa atgggttttg tttttcggta aaattagttt aaaggatttt aaaataaaga 4980
aaacgagatg atcggtttgg gagtttttta attagagtag agaagttaga ggggggcggg 5040
cgatttggtt ttgaagtttt agttgaatag ttattttttt tttttttggt aaaaaggatt 5100
tttttagaat tttcgaggtt tttggatttt ttttttcgta aatggagtcg tatattgtat 5160
tttttcgttt tttcggatcg ttaagtatgt tttatgaggg tcgttgtttt cgggtggaat 5220
gcggtcgtat gtacgcgttt ttttgtatac gtatatatac gtatatttat aataagtgtt 5280
tgtaggagga gtgttttgcg cgttagtttt gcgtttaaga taggaagttg tcgggttatc 5340
gagttaaatg ggagtgatat tatttttttt tattagtaag gaaagcggat tataaaagtt 5400
tttttgtatt tcggtagttt atttaatatt atttatgtat tttgtgtaag gaattgtggg 5460
atttcgtttt acggtaaata atatggaaat tttaaaaata gcgatttttt tgtgcgtgtt 5520
tatttacgcg tttcggggtg atttggcggg gttgtcgtcg ggtgatttat atttttgaat 5580
cgcgaagcga tagggaaagc gcgggcgagc gtaggagacg cggtcggggg ttttttcggg 5640
tttttgggtt ttcgtattcg gagcggggga cgcggtcgtt ttaaggggag gaggggcggc 5700
gggttgtttt tgttatttag cggcggtcgg agcgttacgt gggcgcgcgg cgtcgcggtt 5760
attggttcga ggtacgtgtt taggagatcg gtttgcgacg ttattcgagg gggttttgtt 5820
aaaaataaga ataaaaattt agagtgaaag tgttttaggt tgcgtcgagt ggtttggaaa 5880
ttttcgagtt cgcgcggagg tcgaggcggc gagggcggcg gacggtcggg gagcgcgggc 5940
ggtttagttc ggttcggtcg ggttttggtt tcgcgttttt tatttatgcg attcgggtcg 6000
cggagttttg cggggttcgg cgggggcgcg gtcgtacgtc ggtggggcgt ttcggttcgt 6060
agcggggcgg cggtcgcgag gagggggttt ttatgtgcgt gcgggcggtg gcgggcgcgt 6120
tgatcgcggg cgttcggtat tttcgagggt cggttagggc gtgcgggcgg ggacggtcgg 6180
gcggcggcgg cggtcggagt cggttcgggc gggcgtgagc gtcggggaac gcgttgtttg 6240
tatgcgcgta gttttcgttt cgggcggttt aggcggcggc gtcggagttc gaggcggtcg 6300
gacgcggaga ggagcgggga gttcgggagg cggttcgcgt tttcgtcgga ttattgcgat 6360
tgtttagatt tcggttgcgc ggcgaagtcg aggatttggt tttgttgaat tttttatcgt 6420
ttgggcgagc ggggcggttc gtggtgtttt taatttagtt cgtggattta aaggtggttt 6480
cgcgtcgagc gcggtcggcg atttgtagga ttttagtttt ggtcgcggtc gtcgcgtacg 6540
ttttcggaag attcggcggg gtgggggcgc gggggttttc gtgtgcgtcg cgggagggtc 6600
gaaggttgat ttggaagggc gttttcggag aattagtgtg ggatttattg tgaatagtat 6660
ggaggagaat gattttaagt ttggcgaagt agcggcggcg gtggagggat agcggtagtc 6720
ggaatttagt ttcggcggcg gttcgggcgg cggcggcggt agtagttcgg gcgaagcgga 6780
tatcgggcgt cggcgggttt tgatgttgtt cgcggttttg taggcgttcg gtaattatta 6840
gtattcgtat cgtattatta atttttttat cgataatatt ttgcggttcg agttcggtcg 6900
gcgaaaggac gcggggattt gttgtgcggg cgcgggagga ggaaggggcg gcggagtcgg 6960
cggcgaaggc ggcgcgagcg gtgcggaggg aggcggcggc gcgggcggtt cggagtagtt 7020
tttgggttcg ggttttcgag agtttcggta gaattcgtta tgtgcgttcg gcgcgggcgg 7080
gtcgttttta gtcgtcggta gcgatttttc gggtgacggg gaaggcggtt ttaagacgtt 7140
ttcgttgtac ggtggcgtta agaaaggcgg cgatttcggc ggttttttgg acgggtcgtt 7200
taaggttcgc ggtttgggcg gcggcgattt gtcggtgagt tcggattcgg atagttcgta 7260
agtcggcgtt aatttgggcg cgtagtttat gttttggtcg gcgtgggttt attgtacgcg 7320
ttattcggat cggttttttt taggtgagtt cgcggggatt acgcgtttcg gttcgtcgcg 7380
gggaggttcg cggagttggg gggcggtgtt ggcgcgggaa tttatcggga ggaaaatatt 7440
tcgaattttt ttcgcgtata cgtataaaga tttacgcgat attgtgtgaa gttgacgtcg 7500
gttcgggtag cggttaggag tttagcggta ggattgattc gttagggggt atagattttt 7560
taggatcgta gaagggattt tttttttttt tttgtttttt tttttttttt tttttttttt 7620
gttttttttt tttgtttttt atttcgtttt ggcgtatttt tttttagttt ttagtttatg 7680
ttttttttat tgtagttttt tttggtggga acgtggtggt tggaagatgg gttcggaagt 7740
gtatattttt attttttttt ttacgatttt ttaatttagg ttaggtcggg gacgtatgtt 7800
ttagtttatt ttagatttgt tttattattc ggttatttcg gttgtgttcg gggaagaaaa 7860
ggcgaggttt tttgtcgttt tgttttttgt ttttcgggtt tgcgttgatc ggtgggattt 7920
aggaggatgt atatagggaa ggaggaaaat aaaggcgttt tttttttttg gttttatttt 7980
gtttgttagc gttagttcgt agtggtgggg tttagttttt tttttgtata tagcgaggat 8040
aagggaggta gtcgtttttt tcggtatttg ttatttttaa atagaaagga ttttttttta 8100
gggttttttg ggggttgttg atgggaaaga ggtagtattc gtaggggttt tgtagagatg 8160
ttggatatat ttttttatag atttgcgatt ttaaaaaatt aagtttatgt ttttgtagaa 8220
attattaatt gtattttatg cgggtttgcg gttgggaatc gttattagaa gtggattgtt 8280
tgatttcgag ttggtagcgg attttcgttg tttttaaatt tttaattatt ttgcgggggt 8340
tatttgttta gattatagta ggagtgagtt aatttttggg tcgttatttc gtagaattat 8400
gcgtgtatat ttttgatgaa atttagattt tttagttaga tttgaaattt gttttattgt 8460

tttcgttttt tttttttgtt aatatttaat taatatatag gtttataatg tcgggcgagg 8520
agattcggtc gggttttgtg cggcgcggga gttcgttgag ttagttttta acggttcggg 8580
agttgggtag tatcgttcgg ttcggtttgg ttcggtttag tttagtttag tttaagtcgt 8640
ttatttttat gggttttaaa atatttttgt aagataatgt ttttgttttt tggttttttc 8700
gaaagaaagg ggagagagag tttttttggg gaggtttgat tttgtttttg agatttttaa 8760
gtatttgttt tttgaaagaa aattaagaaa aaaatttaaa aattattatt ttagggaaat 8820
ttattgttat aaaatggtgt ttttttgcgg gttgttttat gagtgtatta ataagagttt 8880
taggattaga agagtttggg ggtagagttt tggggaaggg agtggttgga aatttagata 8940
gagatgggtt ttgggagtag gaggttgggg ttttttttgg agttttgtgt tttatttttt 9000
attatcgttt cggaggttta attttatttt taaatttgta tttattttta ttaaagttag 9060
gtttattggt ttggagtttt gggcgtgagt aagataggta ttgagtgtgt acgtgtgtat 9120
ggggtgggtg tttaagtata gggtgtgtgt ttttatgggt ggtgagtttg tttatgggtt 9180
gttttaaaag ttgtttttgg tgtttttgag gtggtgttta tagatttttt ttttttaggt 9240
ttgttttttg gagagagtat aagatttatt tggttatgag ggagtgtttg gtatttattt 9300
tgggttttta gttcgttttt tattttttgt tgggtatagt tttagtattt tagttgattt 9360
ttttgatttg ggtagggtgt agttttaggg tttttaagga gatttatatt tttttttttt 9420
ttagtgtgtt cggtagtttt tcggttttga agggtggggg gtttttagtt ttttttagtt 9480
atagggattt gtgatgaagt tggggttaga tgttttttaa agtcgattta tatatcgtat 9540
aaattgaaat ttagaggcga ggttattatt ttttgttagt ggttttgttt tttttttttt 9600
ttatagggaa cgttaggggg ttgagttttt tattattaaa aagaaattga tgatattttt 9660
ttttttttgt tttttttttt ttgttttttt tttatggata gtaggtttta gaagttttat 9720
agcgattttg tttaaaattt ggggtaggtt tatagggaga aggttaggtt aggtttataa 9780
gtttgaattt tagttgggag gtatagtggg gagggttaga agtggatttg gataaggtta 9840
gttgggttat tttgttgttt atagtgaagt agttttatgt ttggggaaag ggtggtgtag 9900
ttaatatttt tgtagagtta ggtttttttt tttggatagg aaatttggga gatttttagt 9960
gggtgaagga tttatttatt gtgagtagtt tagtgttttt ttttattaag gagggaagta 10020
tatgtattga ttttttttta aaggaatgaa tttgggttta tagagttttt ggttgggagt 10080
tatagaggag tttgggtgga ggtagatatt ttgggttttt tttgttttta gggtttattt 10140
gtttttgatt tttatagttt ttggtatttt gtggggtatt tttatgaggg tttttattat 10200
agtttttagg gcgttttttg tttttgtgat cgttttgtag ttttttttag tttttttttt 10260
tttttttttt tttagtattt atcgttattt ttgtttttga atagagagtt ttagaaagga 10320
ttaggaaaaa ttaggttaga aagtgtgggg agttttgttt atatttagga gttttatttt 10380
tatttagaga ttttttattt gtggttagtt tgtttattag gtttgtttta tagttttatt 10440
tatattatac gtagtttttt tttattaagt ggtggaggtt cgcgttgagt ttatgtttag 10500
ttcgaagttt agttttatat ttggcggttt agtttcgagt ggttttgggc gagttatttt 10560
ttttttgggg ttttagaatt tttattcggt gtttattggg tggtatattt ttgggtaatt 10620
tgattttttt ttgtttatcg tatttattta gg 10652

<210> 74
<211> 10652
<212> DNA
<213> Artificial Sequence

<220>
<223> chemically treated genomic DNA (Homo sapiens)

<400> 74

tttgggtaga tacggtggat agagagaagt taggttattt agaggtgtgt tatttaatag 60
gtatcgggta aggatttggg ggttttaaga gggaagtgat tcgtttaagg ttattcgagg 120
ttgaatcgtt agatgtgggg ttagatttcg gattgggtat gggtttaacg cggattttta 180
ttatttggtg agggaggatt gcgtgtgatg taagtgggat tatggggtag gtttagtggg 240
taggttgatt ataaatgagg ggtttttaga taaaagtaaa atttttggat gtaagtaaaa 300
ttttttatat tttttggttt ggtttttttt agtttttttt gaggtttttt gtttaggaat 360
agggatggcg gtaggtgttg agagagaggg gaggagagaa gattgggaaa ggttgtaggg 420
cggttataga agtaggaggc gttttgaggg ttatgatggg gatttttatg gggatatttt 480
atagggtgtt aagggttgtg ggaattaggg gtaggtgggt tttgaggata ggaggggttt 540
agagtgtttg tttttattta agttttttta tgatttttag ttaagggttt tgtggattta 600
agtttatttt tttaaggaag agttagtgta tgtatttttt tttttggtgg ggaggggtat 660
tgagttattt atagtgggtg agttttttat ttattggaaa ttttttaggt tttttgttta 720
ggagagggga tttggtttta taagggtgtt gattgtatta tttttttttt agatatggga 780
ttgttttatt gtgggtagta gggtagttta gttgattttg tttaggttta tttttgattt 840
tttttattgt gttttttaat tgggatttag atttatgaat ttgatttggt tttttttttg 900
tggatttgtt ttaggttttg gatagggtcg ttgtaaggtt tttaggattt gttatttatg 960
gggaaagggt agagggagga gagtagaagg agggaagtgt tattagtttt tttttggtga 1020
taagaggttt aattttttgg cgttttttgt gggggaagaa gggggtaagg ttattggtag 1080

ggagtggtga tttcgttttt gggttttaat ttgtgcggtg tatgaatcgg ttttagggag 1140
tatttggttt tagttttatt ataggttttt gtggttggag aaggttgggg gttttttatt 1200
ttttagggtc ggagagttgt cgggtatatt gaggagaaga ggaatgtgga tttttttgga 1260
ggttttggga ttgtattttg tttaggttag gagagttaat tagggtgttg gggttgtgtt 1320
tagtaagagg tgggggacga attggaggtt tagggtgaat gttaagtatt tttttatggt 1380
taagtgagtt ttgtgttttt tttaggagat aggtttggag aaagaggatt tgtgggtatt 1440
attttaggag tattaagggt agtttttaaa gtaatttata ggtaggttta ttatttatga 1500
gagtatatat tttatatttg ggtatttatt ttatgtatac gtatatattt agtgtttgtt 1560
ttgtttacgt ttagggtttt aggttagtgg gtttggtttt ggtaggggta ggtgtagatt 1620
tggagatggg gttggttttt cggaacggtg gtgggggatg gggtataagg ttttaggaga 1680
ggttttagtt ttttgttttt agagtttatt tttgtttggg tttttaatta tttttttttt 1740
tagaatttta tttttaaatt tttttggttt tggggttttt gttaatgtat ttatgaagta 1800
gttcgtaaaa ggatattatt ttatggtaat aaattttttt aaaataataa tttttaggtt 1860
ttttttttga ttttttttta aaagataaat atttaggagt tttagaaata gaattaaatt 1920
tttttaagaa aatttttttt tttttttttt tcggagaaat taaagaatag aaatattatt 1980
ttgtagggat attttaaagt ttatgaagat aggcgatttg ggttgggttg agttgggtcg 2040
ggttaggtcg ggtcgggcgg tgttgtttag ttttcgggtc gttgggggtt ggtttagcga 2100
attttcgcgt cgtataaagt tcggtcgagt tttttcgttc ggtattgtga gtttgtatgt 2160
taattaaata ttaataaagg aggaagacga gaataatgga gtaaatttta gatttagttg 2220
agaaatttga attttattag aaatatgtac gtatagtttt gcgggatggc ggtttaaggg 2280
ttggtttatt tttgttgtga tttgggtaaa tgattttcgt aaaatagttg agggtttggg 2340
ggtagcgggg attcgttgtt agttcggggt taaatagttt atttttaatg gcggttttta 2400
gtcgtagatt cgtatggaat gtagttgatg atttttataa ggatatggat ttagtttttt 2460
aaaatcgtag atttatgaaa aaatatattt agtatttttg tagggttttt gcgaatgttg 2520
tttttttttt attagtagtt tttaggaaat tttgagagaa ggtttttttt atttggggat 2580
ggtaggtgtc gggagggacg gttgtttttt ttgttttcgt tgtgtgtagg aagggggttg 2640
agtttattta ttgcgggttg gcgttggtaa ataaagtgga gttaagggga aaggcgtttt 2700
ttattttttt ttttttttgt gtgtattttt ttgagtttta tcggttagcg taggttcgag 2760
gggtagaagg tagagcggta aagggtttcg tttttttttt ttcgagtata gtcgggataa 2820
tcggatggta ggataagttt ggggtgggtt ggggtatgcg ttttcggttt ggtttgagtt 2880
ggaagatcgt agggaagggg atgagagtgt gtattttcgg gtttattttt tagttattac 2940
gtttttatta aaaaaaatta tagtggggga gatatgggtt aggggttggg aagagatgcg 3000
ttaaggcggg gtggaagata gagaggggag atagggagag aggaaggaga gagagagata 3060
gagaaagaaa gaaggttttt tttgcggttt taagaagttt gtatttttta gcgaattagt 3120
tttgtcgttg gatttttggt cgttgttcgg gtcggcgtta gttttatata atgtcgcgtg 3180
agtttttgtg cgtgtgcgcg ggggaggttc gagatgtttt tttttcggta agttttcgcg 3240
ttagtatcgt tttttagttt cgcgggtttt ttcgcggcga gtcgggacgc gtggttttcg 3300
cgggtttatt tgaagaaggt cggttcgagt agcgcgtata gtagatttac gtcggttaga 3360
gtatgggttg cgcgtttagg ttggcgtcgg tttgcgagtt gttcgagttc gagtttatcg 3420
ataggtcgtc gtcgtttaag tcgcgggttt tgagcgattc gtttaggggg tcgtcggggt 3480
cgtcgttttt tttggcgtta tcgtgtagcg agagcgtttt ggagtcgttt ttttcgttat 3540
tcggagagtc gttgtcggcg gttgggagcg gttcgttcgc gtcgggcgta tatggcgggt 3600
tttgtcgggg ttttcgggag ttcgagttta agagttgttt cgagtcgttc gcgtcgtcgt 3660
tttttttcgt atcgttcgcg tcgttttcgt cgtcggtttc gtcgtttttt ttttttttcg 3720
cgttcgtata gtaggttttc gcgttttttc gtcggtcgaa ttcgggtcgt aggatgttgt 3780
cgatgaagaa gttggtgatg cggtgcgggt gttggtggtt gtcgggcgtt tgtaggatcg 3840
cgggtagtat tagagttcgt cggcgttcgg tgttcgtttc gttcgggttg ttatcgtcgt 3900
cgtcgttcga gtcgtcgtcg gggttggatt tcggttgtcg ttgttttttt atcgtcgtcg 3960
ttgtttcgtt aggtttgggg ttattttttt ttatgttgtt tatagtaaat tttatattgg 4020
tttttcgggg acgttttttt aaattagttt tcggtttttt cgcggcgtat acggagattt 4080
tcgcgttttt atttcgtcga gtttttcgag ggcgtgcgcg gcggtcgcgg ttagggttga 4140
ggttttataa gtcgtcggtc gcgttcggcg cggagttatt tttgaattta cgaattgggt 4200
tagaaatatt acgagtcgtt tcgttcgttt agacgatgag agatttaata gagttaagtt 4260
ttcgatttcg tcgcgtagtc ggggtttaga tagtcgtagt ggttcggcgg ggacgcgggt 4320
cgtttttcgg gtttttcgtt ttttttcgcg ttcggtcgtt tcgggtttcg gcgtcgtcgt 4380
ttgggtcgtt cggggcgaga gttgcgcgta tgtaggtagc gcgtttttcg gcgtttacgt 4440
tcgttcgggt cggtttcggt cgtcgtcgtc gttcggtcgt .tttcgttcgt acgttttagt 4500
cggttttcga gggtgtcggg cgttcgcggt tagcgcgttc gttatcgttc gtacgtatat 4560
ggaggttttt ttttcgcggt cgtcgtttcg ttgcgggtcg gggcgtttta tcggcgtgcg 4620
gtcgcgtttt cgtcgagttt cgtagagttt cgcggttcga gtcgtatggg tgagagacgc 4680
gaggttaggg ttcggtcggg tcgggttggg tcgttcgcgt ttttcggtcg ttcgtcgttt 4740
tcgtcgtttc ggttttcgcg cgggttcgga aatttttagg ttattcggcg taatttgaga 4800
tatttttatt ttggattttt gtttttattt ttaatagagt tttttcgagt gacgtcgtag 4860
gtcggttttt tggatacgtg tttcgggtta atggtcgcgg cgtcgcgcgt ttacgtgacg 4920
tttcggtcgt cgttgggtga taggagtagt tcgtcgtttt tttttttttt aaagcggtcg 4980
cgtttttcgt ttcgggtgcg ggagtttagg aattcggaga gattttcgat cgcgtttttt 5040

gcgttcgttc gcgttttttt tgtcgtttcg cggtttaggg gtgtgagtta ttcggcgata 5100
gtttcgttag gttatttcgg ggcgcgtagg tggatacgta taggaagatc gttattttta 5160
agatttttat attgtttatc gtggggcgaa attttataat tttttgtata aaatgtataa 5220
ataatattaa atgagttgtc gagatataaa gggatttttg tggttcgttt tttttgttga 5280
tggagaggaa tagtgttatt tttatttgat tcggtaattc ggtagttttt tgttttaaac 5340
gtagagttgg cgcgtaggat attttttttg tagatattta ttgtaagtgt gcgtgtgtgt 5400
gcgtgtgtag ggaggcgcgt gtatacggtc gtattttatt cggggatagc gatttttatg 5460
aaatatgttt agcgattcga aagagcgggg gaatgtagta tgcggtttta tttgcgaagg 5520
gagaaattta ggagtttcgg aggttttaaa ggaatttttt ttgttaagga gaggaggggt 5580
gattgtttag ttaagatttt aaaattaagt cgttcgtttt tttttaattt ttttattttg 5640
attgaggagt ttttagatcg gttatttcgt tttttttgtt ttaaaatttt ttaaattaat 5700
tttgtcggag agtagggttt atttgaaacg agaattagga atttaacggt aagggtggga 5760
gttttagttg gttttttttt tttttttttt ttattttttt tagatttttt ttttagttgt 5820
tgttagaatg tttaattgga agttttgttt tttttttttt tttttttttt ttttcgggtt 5880
tttttttatt tgtttttgtt tatttattaa atattcgttg ttattttagg ttcgtttgtt 5940
tattatttaa aatgtatttt tatggaaatg tgtgaatttt tggaaatatt cgatagggta 6000
gttttggggg tgcgggagta tttgtacgtt atgagtattt ttagtgcggg ggaggcgggg 6060
aggtcgtagt ttggtacgga tattttaggg ttgattggtt ttgggtagtt gtatcgtgtt 6120
tagtaattta gattttcgta gaggtagtta gtaggttgtt gttaggtttt gagtgaattt 6180
ggggagtttg gtaagtattt tcgaggtagc gtggtttgtt aggttatttg gggtttgaag 6240
gttgtatttt ggttttgcga agtttttttt ttagtgttta ttttacgtgt tgtttttttt 6300
ttgtaggttt tagagtgttg gttgggttgg gatgagaaac gatagttttt atagttgttt 6360
ttaggaaaat tattttttta tttaggacgg gaatggtggg ggaggaggtg ttgggttggt 6420
tttttggtta ttggacgtgg ttttggttag agtgtgtttt ttgttcggtg tgtttagggt 6480
agggtgggga agtttttttt cgttggggtt ggattttttt attttttttt tattggagga 6540
gtttttatta aatgttttgg ttaggtatat atttgttagg ggtttttttc gagttttaaa 6600
aaattaaatt ttatagttta tttgtttttc gggaaaatag ggttggtgag agtttgtggg 6660
aagttttatt gacgtttatt tggttttttg gggggagaat tttttgtaat tttggaaatt 6720
ttgatgatta ttagttagga aagggagggt ttaacgattt tgggtttttt gaatatgata 6780
ttggggggag gaggggtttt gagttttttg gtttttgtaa aattatttta ggttaatggt 6840
tttatttgtt ttatgaaagt taattaggcg gttttttgag aagtattttt tgaatttttg 6900
ggattttttg tttttgattg ttttagtata aatggtttta agacgttggg ttttagtttt 6960
gtgttttttt gcgatttaga ttttaattcg tggttgtaat agagattttg ggaagtagag 7020
cgttattttt agggcgtttg gcgggtcggt gggtagagat ttgttttcgt agggtttgcg 7080
ggttgggttt tggggttggt tggaggaaga ggaattcgtg ggtcgttttt ttggaaagga 7140
ggaaggtgag aggtggaggg aggaatagga gaaagagaaa taaatgaaga gagagataat 7200
tagagggtat cgggtttttt agagtggatt taaatacgaa ataattttgg gaaagcggta 7260
agaggggtta tcgtttaaag ggtcggggaa gttgggatgg ggttgtagaa agttgagttg 7320
tttaggggtt gggcggtgtt gagttttata tatacggaat ttcgtaaggt gttttaggaa 7380
gtttcgagaa gcgcgtatga ggatcggttt cggtcgtttg ttttagttgg gacggcgttt 7440
cgcgtttttt ggggatgcgg ggtagtggcg tgtttttttt ttgcgatgtt agtttcggtt 7500
gtcgcgtttt cgcgagttta gagttttcgt tttttttttc gaacgatcgt cgtttttaag 7560
gtcggttttt ttgtgcggat tagacgagtt tttttcggat attagttttt ttcgggatgg 7620
aaagtggggt attgattcgg gaatttcgtt ttttttttag ttttatttag gaatcggaga 7680
gcgttcgcga ttattcgttt gcggaggacg gttttttata ttttttaggt tcgttgtata 7740
aagaggggtt tcggagaggg cgagtaggaa agtagttttt agacgtcgtg cgttttagga 7800
tacggacgtt agttttgtaa ataaaatttt agtgaagcgt ttcggagata tcgggcgttc 7860
ggatgtcgag ttaggttttc gtttttattt gaaaacgagg ttagagaagg gtgatttttt 7920
cggttttttt gcgttttttt tattcgtttc gtttttttag tttgacgtgg tgatatttgg 7980
aaaaatggat ttaatgaaag tcgttcgttc gcggcggttt cgttattcgc ggggtagaga 8040
cgtttaataa tttttgagag cgagattttt attttagtta aagtaggttt ggttagtttt 8100
gcgttttcgg ttgggagtcg agtttcgtag ttaagtttta ggcgtcgggc ggggaattgg 8160
ggtcgatagg agagtaatat ttttttcggg gcgagtagag agttatcgtc ggtttttggg 8220
gttgacgttt cgattcggtt tgtagataaa ggtcgaaacg aaggggcgcg gggagtaggg 8280
gtggggcggg cggggttaac gggcggatat tgcgtgtttg cgtttttttt tgggcgaagg 8340
aggatcggtt tataggttta tcgggcgagt tcggggacgg ggtttgggag gttgggttta 8400
gggtttgaga tttttttaga ataattttcg gaaggagggg gttaaatgcg gtagcggcgg 8460
gtttgaaaag ggttaggagc gtcgtaggaa ttggggtgta taggttttcg cgtgtttagc 8520
gtttcgtttt ttttcgtttt aggtttgtta ggggcgcgcg tggcgcgggt agagtttggt 8580
gggtgggttg gcgcggatag aagttttgtc ggggagcggg gagggggggg tagtcgtatt 8640
gtacgttttg gttttgtttc gatcgttgga gaagcgtttg tgttgagttt aagtttttta 8700
ggtgttgggg aagtagagtg ggttaggagt cgattgggga tcgttttttt gttttgattt 8760
gatatttagt ttaggttcgt tcggggaggg gggcggttgg gggtaggtgg gtagattttt 8820
agagttggcg tgtaaagatg tagaaattgt tttttatttg gtttttttgg aaagtagttg 8880
tttttgaagg agaatttgaa ggatggtttt atttgtgtta gtaattttta tatttaaatt 8940
aggttttttg tgttcgtttt ttttttcggt tttttagcgt taggggagtt ttattttggg 9000

tgatttgttt ttttaagaat cgtttttttt tggtagtttt ttgtttgttt ttttgttttg 9060
ttttattgtt gtttttattt ttataaaatt tggatttttg tggaaggtgg gtttattttt 9120
atttaaggtt tatttttgag attttttaga tagatttgta ttttggagtt tttcgggagt 9180
tttgggaggg gtaggtttgg gagtatagat tgttttatta tttaatgttt ggatttttat 9240
gaaaatgttt ttttaaaatt atagtaattt agaagaaagt ggtatttttt aaataattta 9300
gttaaataag taaaattagt ttggtaaagg aatttgaaga atggtattta aggtttgagt 9360
tggttaagtt tattaaaata ggaaggaaaa tatagaaaag gataatgagt tttgaagtat 9420
attagttatt ttttttagtt ttgcgattat ttatagatgg aagaaatttt agtatgtatt 9480
tattgttttt tttttgattt ttggaaagaa aataagatgt ttaaaaggaa tttttgaaat 9540
attagtttta ttttatagag gttgatattt tttttttttt tatttttttt ttaaaggtta 9600
taagtaatta tagagaggtt agtgtagttt aaatttttat atagtgattt tttagtattt 9660
ttggaaatag gatttgcgta gttagaggtt tatagaattt taggtaagta taatttagat 9720
ttaatttaaa ttatttggat ttagtttagt taaattattt atagtttgtt agatgataat 9780
ttattagttt aggttttacg tgaggaagaa aatatttaaa taattatttt cgtttttaaa 9840
tattataatt gaattatgtt taattaggag aatttaatta gtttatatat tggttaagtt 9900
gggggaaata tatttttaaa aatatatgtt taagttaaag ttatgaattg attttttttt 9960
ttatttatta aatataaata aagatatata gttttatata aaaaggtaat taaattgcga 10020
attaaattta aatatttttt ttatttagtt aaatgattga ttagtttttt tgagtttaat 10080
aaagttggtc gtttttttag tacgagtatt tttaattgat atatatgttt atatgtatgg 10140
tatgggggga tttttgtatt taattaattt ttgtgttgag tatagaagtt tgtgtgaagc 10200
gtggcgattt tttataagta taaagtttta gtagtgattt tcgtggattt atacgcggag 10260
gggtattgtg ttatttttgt attatggatt tttgttttta gttttgttta aggtttaaat 10320
tattttttcg ggggatgtcg tttagtttta tttttattag ggatggggat ttgttgttta 10380
tttttggacg tatttttatt tttttttttt tattttagta tttttggttt tatttaaaga 10440
aaagtttagt agaataagat tttttagtta gggtatatta tagaagtgtt ttgttttttt 10500
tttaaaaaat tttttttttt ttttcggaaa tttatttaaa tattttagtt ttttttagaa 10560
aggttgtgtt atagtttatg ttatggagtt gtagggtttt gtgtgtttta tattagttaa 10620
ttttttaaga tataatagtt tttgtgatga cg 10652

<210> 75
<211> 16891
<212> DNA
<213> Artificial Sequence

<220>
<223> chemically treated genomic DNA (Homo sapiens)

<220>
<221> unsure
<222> (8209)

<400> 75

ttaaattttt gattttatgt gatttattta ttttggtttt ttaaagtgtt gggattatag 60
gcgtgagtta ttatattcgg tttttttattt ttttattttt agagataagg ttttgttttg 120
ttatttaggt tggaatatag tgacgtgatt atagtttatt gtagttttga atttttgggt 180
ttaagtaatt tttttgtttt agttttttga gtagtcggga ttgtaggttt ttattattaa 240
gtttggttta aatttgtatt tataagaagt ttttagaaga aataagatta tattttgagt 300
agtaatagtt tagggtatga tattttatgg tcgagattcg ttggtgggta ataaaattaa 360
tagatgagtt tgtgacgagt agtgaaagaa aatgtaatag gttgggggtt attggagggt 420
attatagtcg taaatttatt tataggatta taaaatgcgt ttttattatt tgagtttgag 480
ttttagggtt tgtgttggga cgtgatatta gggtcgtaaa ggtttgcgaa aatttggttt 540
gggagtgagg gtaaataaag aaggaagggg ttagtaatta agtttggggg aataaggagg 600
aattagggag gagatagata aggtgattag ggaggtggga ggaaagttag gatagaatgt 660
tttggaagtt aagggaaaaa ggtttggatt ttaattattt tttgtttttt tttagttttg 720
tggttttgta aatttgagtt ttagtttttt ttatcgtaaa atgttgatat gatagtttta 780
gttattgaag tggttgtaaa gtggtatttg gtatagggtt aggtaatttt atggatggtg 840
gtgtaatttt aatttttgtt ttgttttttg aattttttta gattagtagg ttgttttttt 900
tttgtgtaga tgaagaaatt gaggtttaga aagtggaaag atttggttgg gtgcggtggt 960
ttacgtttgt aattttagta ttttgggagg gtaaggcggg tggattattt gaggttagga 1020
gtttaagatt agtttgggta atatggtgaa atttcgtttt tattaaaagt ataaaaatta 1080
gtcgggtgtg atggtgtatg tttttagtta ttcgggaggt tgaggtagga gaattgtttg 1140
aatttaggaa gcggaagttg tagtgagtta agattatgtt attgtatttt agtttgggtg 1200
atagagtgag attttgtttt aaataaataa agattttgtt tgatgttatt tggttagtgt 1260
agggtagagt ttgggtattt tgtttcgttt tgtgggtttg gttttgttgt tattaatggt 1320
ttttggtttt aggttagtgt tggagatagt tagtcgtttg gtgggttttt gggggtcgtt 1380

tgaaattttt ttagtggtta gtggttaggg tggacgtgtt ttgttttttt ttgtagtttt 1440
ggtttttttg gttagttagg aggaagaaag agtagaaagt gtgtatttgt tttttgtggg 1500
gtttaggtat aggggtttgg ttattagtta cgcgtttttc gggcgtgtgg atagacggta 1560
tttgaatata ttcgtattag ttaaagtttc ggtaggaaat agatggtacg ttttagtagg 1620
attattagag gagagtttgg tgaagggttt gtttatatag gtgtggtcgg ggtgtaggga 1680
agttttaagg gacggtgtag aattttaggg ttggtagcgg cgtgggttgt gattattttt 1740
agtttgaaga ggttagggga ggagttgagt ttatagttgg atagagttgg gtggaggggg 1800
tttttaatag gatcgtggtt tttagtggag ggaggttatt agtatgtagt gattttgtag 1860
ggaaggggtt ggaagacgga tttttttttt tttgtttttt ttgattttgg tagggtgggg 1920
gaagtggatg tgttttataa gagggagagt gtagttgggg gatgtagaag atagttattt 1980
taaggtttgt gtttagtagg gaggttatgt ggttattagg ttttttcggg taggagattt 2040
agtttaggat tggttttttt ttttgggaat taatgataag gtttattttt ttgggttttt 2100
atgattttta tttttgtttt gatttttagg gtttttttatg tttttagtaa atttatttta 2160
agagttgaaa ggttttgggt tttgtgttgg ggatatagta gtgtaaaggg tgttaaagtt 2220
tttgttttta tggggttttt aattaagtag agagatatat gtagatggtt gtatataggt 2280
ttaggtgtga tagggaaggt taggatgttg tgggagttag aggtgatttt cgttttattt 2340
tttattcgcg gtggttttaa tttttttttt tttttatgag gtgtttgggg tcggggtttt 2400
agtttttttt ggagttattt tggagttttt tttatttttt agagaattta ttgggttttg 2460
tttataacgt ggatggggat agattgtgta gcgtggaggg aaggggaggg agggagtttt 2520
gggaagagtt ttttgtgggg tttttgttat tgttttagat gtttttaata ttttgtgata 2580
tttgtagttt ttgttatatt tgttttttat tttaaattta gtttagggga tggtggtagg 2640
gaaggaggtg agtttggatt taggtagttt tggggttatt attttttagt tggggttttt 2700
tttttgtaaa gtggaggtat aacggtattt atttttgggg gtggttgtga ggatttagag 2760
ttgataaggt gaacgtttag ggcgggtttt ggtgtagaga gagcgtttag tttttagggt 2820
tggattaatt gtttttgggg tatagatttc ggtttggggt ttgtggaaat tttagagtta 2880
tttttgaatt tttatcgagt tattttttgt ttcgtagtgt ttatggtttt gttttcgagg 2940
ttataggaaa aggtagagga gatgtttttt ttagggtggt tttttgggag aggatatttt 3000
tttttgattt taaagttacg tttggttgta aattggttag gtagttataa ggttgggtaa 3060
gtaaaattat ttttaatttt tatttaaaga gttatatcga tttttttagt cgttgtgata 3120
gtttttgtag agataaatat acggtttatt tttgttattc gggtcggtta ataagtacgg 3180
agaggtaagg ttttagattt tggatagata tttttttttt agaggtattt agggttttag 3240
tttttttttt tttttttggg ttttaatttt tttatttgtg atttagggta ggtggattta 3300
gggagaagaa ttttttggtt ttattttatc gtgggttttg ttagtatata taaagatttg 3360
gttttttaaa gtttagtttt gttagcgttt ttttgtttaa gaatttttta tgatttttag 3420
tggttttaag gataaagttt tggtatttga ggttttttta atgtagggtt agattttgtt 3480
tttttagttt tttgttttta ttatattttt gttggtttta cggtggttcg attgtttttt 3540
gtttttgtgt ttttgtttag tttggtattt ttgtttggta tgtttttttt attttttttt 3600
ttttttaatt ttaatttatt tagtttttta aagggtaggt ttaaatatta ggttttttag 3660
gtggtttagg attttttttt tgagttttta tgggtttggt tttgggtgtt atttgtgagt 3720
agttttacgg tgggtatata gtaggtgcgt ttattgtttg tagaatgaat atgggatagt 3780
ttggggattg ttatttagtt tagggagtat tgatggggaa gtattttttg tatgttttag 3840
ggtttagtgt tgtagtgttt tgatttttag aaattttata atggtttggt taggaaggta 3900
tcgtgtttta ttttgtaaat agggggtgtt gagaattgag gggttttgtt taaggtttta 3960
tggttaggag taagtagaat cggatttgaa tttagggtta cgtgatttta gaagtgttat 4020
taaagttttt ataatttgga gttgtttttt tttttttttt tttttttttt ttttgagatc 4080
gagttttatt ttgttattta ggttaggagt gtagtggttt gattttagtt tattgtaatt 4140
ttcgtttttt aggtttaagt gattttttag ttttagtttt ttaagtagtt gggattatag 4200
gcgtacgtta ttatgtttag ttaatttttg tatttttagt agagatgggt ttttattatg 4260
ttggttaggt tggtttcgaa tttttgattt taagtgattc gtttgtttcg gttttttaaa 4320
gtgttgggat tataggtttg agttattata ttcggtttgg agttgtgttt tgtcggtgaa 4380
ggatttttta tttatgaagg ggttagacgt gaagtgtgtg gttttgggta gtttttttga 4440
gtttagagac gttagtttta gtcgttttgt tgtgttattt tgggattttt ttttttagtt 4500
tgagttttag tttttttgtt tgttaggtag ttttatgtta attgtattta gtaggtcggg 4560
tttgatgttc gataagacgt gaagtggtgg aggtgggtag gattttagcg ttattatttt 4620
tttgaattag tgattttaat atatcggatt tttgtttttt tatttgtaaa atgggattag 4680
tgagtttagg tgggttataa attttatagg aattatttta gttaagatcg gttttttgaa 4740
agtttttttc ggtgggttgt tagggtgatt gtttttattt gtggggtttt ttgatgcgtt 4800
ttatttatta gttttggaga gggtgggatg ggagggtggg gtgtttgggg agataagttt 4860
agagtttggg gttttttatt ttattgtttt tttttattat agggtttttt atttagtgat 4920
aaagttcgtg gtattttttt tattcggttg gtaggcggtt tggtttagtt ttttttttaa 4980
ggaagcgtat tttttgtttt tttgggtcgg tcgggttgga tgagttagga gttttttgtt 5040
gtcggttata ttatagtttt tatttgcgtt ttggggttag gattgttgtt gttattgtta 5100
tttattggag tttagtattt ttttttcgtt tatttttcgg atagtaattt tagtttagtt 5160
tcgcgttttt gtgtttattt tttttgattt ttcggtcgtt attttagaag gttggagtag 5220
ggacgtcgtc gtttcggtcg tttgtttttt tcgggttttc gtgcgagttt acgtcggttt 5280
cggtgttcgt tcgtagtttt gttattggat ataggataag gtttagcgta taggttttta 5340

cgtggatagt atggatcgcg gtacgttttt tttggttgtt gttttgttgt tggttagttg 5400
tagttttagt tttataagta ggtgtttagg gatttagggt ggggagattc ggttttcggt 5460
gtacggatta ggttttaagt attttcggtt ttttttttgt attttgagtt tacgtttagt 5520
agagttattt ttggggtttt ggagggttat taattttttt agtttgttgg aattaaatgg 5580
ttatgtagga tttttagtgt tgaaagaaag tttcgggtaa attgggttga tttttttatt 5640
ttaattttgg tttttggcgt ttgtttattt agttgcgttt tattattttt cgggaagttt 5700
aggttttaga atgttgtgta gcgacgggag agttttttgg tttttttggg ttttgagttt 5760
ttttatgagg aatggatggg aaggagggtt tatagtttgg gttttgagta tcgtttttgg 5820
gtttaaattt tatataggta tttttttgtg gtgtgatttt gggggattgt tttcgggttt 5880
ttgagcgaag tggggaagag aatagatttt ttttttagag ttgttgagga ggtgggtggt 5940
gagggattat tgtagagagg gttttggtag attggttatt tttgtgttgt tttattttta 6000
gttttaagtt tttttagttt atttttttta gggtttttgg gtagtttttt aaattttttg 6060
aataggttaa ggttgtaggg ttttgttttg tttttttttt atttttttga tttttagaaa 6120
gggtgtagga gtgggtgtag gttaggttag gggttttgtg gggttttttg gtttgggggt 6180
ttttttaagg ggttatggtt tttgttgttt tttgaaattg attagaggag gggcggtgag 6240
gagtaaggga tttttagttt tatgttttat tttttatttt tcggttattt taagtcgttg 6300
gtcggttttg tggatatttt ttgatttatt tgtgtattta ggggtttttt gatagatttg 6360
ggggaagagg ggtagaaatg agttgagagt gtggggagga aggggaagga aaatagcggt 6420
ggggtagtta attttttttt atttttttat ttgatggtat tttagagttt ttgattttgg 6480
gtttttaaag ttttgtttat tatttgattg ataggaaatt gagttttaga tatattaagt 6540
gatttagtag ggatttgatt ttcggtttgc ggttttttaa atgtttgttt ttagtaaggt 6600
gtgatagggc gaattttttt aatgcggttt ataacgattt tgtaagatgg ttgttattat 6660
tatttttatt ttatagaaga gaaaagggag gttttaattt gttaagttta tatggttaag 6720
acggagtttt tatttgagtt ttaagatttt tttttttcgt atttgtcgtt gtagtaatat 6780
tttttttttt ttggtcgtta gagaaaggtt tatgtagata tttagggttt ggttttaaag 6840
gattaaaggt agtgatatgt tgttgaataa ttaataacgg ggtggggagt ttaatttgta 6900
gcgtttgtgg atttttatag cgtaagtatt tttattcggt tgattttaag ttattaattt 6960
attaagtttg gagttgggaa ttagtgttcg gtagtatttg gcggttggtt ttttagagtt 7020
tttgtaggcg tagattatat cgagagtaga gatgatagtt aggggtgtaa aacgattaga 7080
aagaaatgag tttggatatt tatgattttt taaaaatata tagtaatagt tatgtaatag 7140
ttagttggaa aaatttttgg aaacgtattc gtttgttttt gtgagtaggt aggagttggt 7200
tttagtgtat taggagttat aggtttaaag gtataataga ttattaattt aatttattta 7260
tggtttggga agttgaggtt tatggaaggt agaggggagt ttggggttag ggtttggaag 7320
ttagttgggg gtaggggtcg gagtgatttt gattttttag aaatgggttt agaatttggg 7380
ggtattattt gtaagaattt tttaaaattg tattttgtat gtgatttgat atttttataa 7440
attagttttt agtagttagt tttttaaagt tattcgttta attttgtaaa gatgcgtggt 7500
tcgtgggtat ttgtaataaa aattcgataa ttggaaatta tttaagtgtt tatgagtaaa 7560
gattgttaat aaattattat acgacggaat gttatgtagt aggtattaag aaggtagagt 7620
tatatgaata ttgttatgga cggatgttag ggatatattg ttaggtgttt aaataataat 7680
aattttattt tggtaataat aaaatagtaa taatgtataa tatgtataga gaaaattttg 7740
gaatagagtt ttttatttaa tttattatta ttgttttttg gtttggggtt ttttttgtgg 7800
gatttttatt ttttttttta aatttttttg agatagtttt gtttttgtta tttaggttgt 7860
agtgtaatgg tataatttag gttcgttgta attttcgttt ttcgggttta agtagttttt 7920
ttgttttatt ttttcgagta gttgggatta taggcgtcgg ttttatattc gtttaatttt 7980
tgtattttta gtagagacgg ggttttatta tgttggttag gttggtttcg aatttttgat 8040
ttcgtgatac gtttgtttta gttttttaaa gtgttgggat tataggtgtg agttattatg 8100
tttggttggg attttgattt ttttagatat atttttttgt tatgttgggt tttttcggtt 8160
tattttggtt aaagataatc gttttaaaaa ataaataaaa ataaaatana taaagtatgt 8220
ttaaagatta ggaatatttc gttaaataat tataatttag agttttaata gtattaaatt 8280
tagatttttg ttatttttat acgtattttg ataatagtta atagttagtg tgtaattttt 8340
cgtagtgttt gtataatgtg tttaatagtt tatttgttga aatttgtatg taatttagga 8400
ttgtatatcg tatttggatg tgatgtttat tagattttat ttatttattt atttatattt 8460
ttgatgcgat agtttcgttt tgttatttag gttggagtgc ggtggtgtaa ttttagttta 8520
ttgtaatttt tatttagtag gtttaagtga tttatgtgtt ttagtatttt aagtagttgg 8580
gattatagac gcgcgttatt aggtttaatt ttcgtatttt tagtatagac ggggttttat 8640
tatgttgtat aggttgtttt agaatttttg gcgttaggtg atttgtttgc gttggttttt 8700
agaagtgttg ggattatagg tatgagttat tgattaaaaa tataaaaatt agttaggcgt 8760
agtggtgggc gtttgtaatt ttagttattt gggaggttga ggtaggagaa tcgtttgatt 8820
ttgggaggtg gaggttgtga tgagtcgaga ttaagttatc gtattttagt ttaggtgata 8880
gagtaatatt ttgtattaaa aaagaaaaaa aaagaataag tatgtagaga tttttttaaa 8940
gtaattaaaa ttaattatat agttgtttta gtttgttttt taaaaaaaat agagaaaagg 9000
gaaaaaatta tttcggaatt tttttattga tataggtatc gttagtatgg tggtagatgg 9060
tttattagtt ttttttttta attttttaag ttatttttaa tttagatggg tttatatttt 9120
gggagttttt tgtttatttt tttcgaatta ggtttttatg ttgttttgtg gattttatga 9180
ttattttttt gtatttttgg aatattttgt tttattaatt tgttatcgtt tgtttattgt 9240
taaatattta ggtggttttt tttttgtttt agttataaat attattataa tgtatagttt 9300

tggtacgtgt attttttttt gtattttgga ttattttatg tggattgcgt ttcggtagta 9360
aggttgtagc ggttcgtggt ttgtgttggg tggttgggat tgaagagtgt ttttgagtgt 9420
ggaggatttt ttgttggagg atttaaagtt tttatgttgt tggttgtttg tgttggagtt 9480
tgggatttgt tggagaaggg ttagtggcgt aagagcgaga ttataaatat ttgtttttta 9540
gaggttgcgt ttttatttgt taggaggaaa aagagtttgg agtaggagtc gtgtgaatta 9600
ggagagggtg ggtttttcga gatacggggg acgtgattat tagtattttt ttttatttta 9660
gttagataga cgtgatttag agggatagag ggatttgttt gtggttacgg atgtgttggt 9720
gttgggtttt cgggcgatag atttattttt ttgttttagg gttggaggtg gatttgggta 9780
agagtgaggc ggttattttt tttttgtttt tagtatgtta ggtataatgt tagttttttt 9840
ttgtttttat ttttagttta tagatgagaa aattgaggtt tgggataaat gataggttta 9900
gattttaatt ttaggtcgtt tgtttttaaa tttagttttt tttttttata atttttgtga 9960
tttgaatgtt ggagatttaa agaattgttt atttttggtt ttgggatttt gttttttagt 10020
tttagtttag gttttggggt attagttttg gaggttgaag ggatttttag ttagggttta 10080
gttggtttta atttttgtgg taggagtggg ggtgggataa agagataata taataattga 10140
tatttattta gtatttatag tatgttagac gttgttttaa gcgtggtatg tgtattgtta 10200
gatttaacgt ttgtaaaagt tttataaagg agatattttt tttgttttta tttgatagat 10260
ggggagattg aggtattcgt ataatttagt taggtgaaga aatttattta agtaatattg 10320
ttttgaaatg agttattggg gtttaagttt tgggtttaag tattgtagtg tgttgttata 10380
gtggagagga tggggatttt ttttttgttt gatttttttt tcgtaggtat ttagtttttt 10440
ttttggtttt tataacggtt tttttattcg gggtataatt tttgggattt tgtttttatt 10500
tttagtagtt ttatttatgt tttgttttgt tttttttttg tttttatttt taggtgaaat 10560
tttagattgt tagattttta gtagaattta gaatatgttt tatttatttt cgtttttttt 10620
tatttattag ttatagggaa atagagtttt agaatggtgg ggggatttga tgtgggttat 10680
ttagtaggtt agaggtagtt ttgattagtt agttttataa atggatgttt gtggtgttag 10740
aaataaaatg tttgtttttt agtgtcgtaa agaaatagta tttgaatata aatttaattt 10800
ttttagtaag gtaattttta tttttataga agggtgcgat ttaagaatgg agtaatggcg 10860
agagtatatt tgaataaggg aggggaaggg gtttttattt ttgacgtagg tagtttttat 10920
tgttacgtcg tttttttacg ggttagtgtt ggatcgtata gtttaagtta attttagtta 10980
gttattttag agagagtagg ggtatgagtt agagtggcgg ggtgagtagt ttggcgggaa 11040
ggatggttag gaataggtaa ttaaaggtga tttaggttaa agtaggtgat taggggtttg 11100
tgaggttaaa gtatatgatt aggatgagtt aggatggagt aggtgattag gggaatagat 11160
gtgaattatt gattaaaatt ggtggaaaag gttgtttatt gaaattatga ggaagttaaa 11220
ttttaaaatg gaggataaag aattgaatat attgatatat tgattttttg aagagaaatt 11280
tagaatttat tgtatttagt ggaagtggat gtttgttttg agttttggat taattgattg 11340
ggatatggaa tgttgtggat tttttttgtt ttggaaaaat ttattgtttg ttgggagtgt 11400
agatatattg ataaattaaa atagtatatt tgggtatgta tggtttggtt ttttaaggat 11460
atttaaggac ggatgtattt aagaaaggag ggattggttt tagtgagatt tttaggatag 11520
ggttttgaag gatgaataga agtttgttag gggttgggta tggtggttta tgtttgtaat 11580
tttaacgttt tgggaggtta aggtgggagg attatttgcg gttaggtatt ttagattagt 11640
ttgggtaata tagtaagatt ttttttttat aaatattttt aaaatttagt tgggtataat 11700
gatttatatt tgtagttgtt cgggaggttg aggtagagga ttgtttgagg ttaggaggtg 11760
gaggttgtag tgagttatga ttgtattatt gtattttagt ttgggaaata gagtaagatt 11820
tcgatttttt aaaattaaaa aaaaaaggtt aggtggttaa ataaaggatg agtgttggtt 11880
tgggaaaggg tatggtagag gtaggagcga ggtgtgttta ggggaagagt ttatatattt 11940
ttattgattg ggtataaata attgtgatag tagttattag tttttgagta atttttattt 12000
agtaggagtc ggtttagcgt tgtgtaggtg taatttattg gtttgtttgt cgattttatg 12060
ggaagttgtt atgatgattt ttatttttat gttatagatg agaatattga ggatggaaag 12120
gtagttggta ggtggaagaa ttaggatttg aatttttggt atttgttaaa tcgttgtgtt 12180
ttttattttt attgtaggtt gtagtgtagg aatttgattt tgtaggtatc gtagaattag 12240
gggggagttc gggttaaggg tatgttgaga tttttggtg gttgggggta gtggttgggt 12300
tggatgttgg gaaatttgtg aggttgtaat tgggtttgaa aagggtaaag agtggttcgg 12360
gggtattgaa taagacgggg taggtattgt agtaagaagg atttttgttg aaacgtattt 12420
tttttgggag gttgagatgg gtggattgtt tgagtttagg agtttgagat tagtttgcgt 12480
aatacggcga aatttcgttt ttataaaaaa tataaaaatt agttgagtgt ggtggtttat 12540
ttgtagttat ttaggaggtt gaggtatgag aatcgtttga atttgggacg gggaggttat 12600
agtgagtcga gatcgagata ttgtattttg gtttgggtga tagagtgaga ttttatttta 12660
gaaaagaaaa gggggggtgg tagcgggaag aaaagtattt gttttagtat gatgaaataa 12720
tatttatagt ttttgtgttt ggggtattta tttagtatta ggtatttaat aggggttatt 12780
ttattaaatt gatatattta gtttgtgaat gtttgttgtt tgttggagtt agattgtttg 12840
ggtttaaatt taggtcgtga tatttagtag ttgggttatt tcgggtagtt ggtttttagt 12900
ttttttaatc gttttggttt ttagtttttt ggagtgtgta tgggtgtaat agcgtcgttt 12960
attttataag gttgttgtga ggattaaatg agtttattcg tgtaaagttg tttaggtttt 13020
tagtaagtat gatgggagta tttgtagtga ttattggtgg tttgtaaatg ggaaattgga 13080
ggtttagaga ggttaggttt ttgtttaagg tgattagtat tttaagaggt agagttggga 13140
tttgatttag gttatttggt tttagagttg ttttttttga ttatttttta aggtgttgaa 13200
ggtagttttt tggaaatttg ggaggtgttg tgtgtatatg ggtggcgagg gtggagaggg 13260

gatagtggtg ggaggtggtt tttgggtttt ttatttgggg ttttagttat ttaggttttt 13320
agcggttttt tttttgattt agtttcggga ggttttggcg ttttttttgg tgttagggga 13380
ttgtttttaa tttggtttgg gttcgtttta ggtttcggga gataggttta ggcgttatat 13440
ttttttttgt ttttttattt tcgtttgcgg ggtgggggcg gttaggattc gaaaaggttt 13500
taaaataaat ggatttttta tttgattttt tcgttaggtt tgttatttga ttattatata 13560
atggttaggt ttgtggtttt tttcgttggt tgacggtatg gaggaggcgg ttttaggcgg 13620
ttttagtaat tcgggaggaa ttggggtttt tggaagtttg gggagggggt agggaaagtt 13680
ttaaagggaa ggagggaggc gttgcgataa tttgtaggtt agatattggg ttatgttggg 13740
agacgcgggt tcgaggttcg gtttggttaa ttatgtgttg tgagattttg atttgtttcg 13800
tttttttaga ttttagtttt tttatttgag taggaggggt ttaagttagg taatagttaa 13860
ggaatttgcg tttatcgatg aggaaaggag agggtgggga gaatatgggg gttggtgggt 13920
tagagggatt ggagatagga gatggtatta gtttttgatt ttgttggggt gagggagatt 13980
ttagtgattt ttttatttta gaggatcgtt atttggaggt tattttattt cgttatttta 14040
ttttattttt tagaatagtt aattttatag tgattgaagt tagttaaata attgtaatta 14100
agagaaagtt gtcggttagg tatgttggtt tatttatggt ggtttatatt tgtaatttta 14160
gtattttggg aggttgaggc gggaggattg tttgagttta ggagtttgag attagtttga 14220
gtaatatagt gagatttagt ttttattgaa aatttaaaaa attagttagg cgtggtggcg 14280
tataattgta gttttaatta tttgggaggt tgaggtagga ggatcgtttg aatttagtag 14340
tttaaggttg tagtgagttg ttattatatt attgtatttt agtttaggag atagagggta 14400
attttgtttg aaaaataaat aaatagaaat ttaagaaaga attgttatgt gtttagtttt 14460
atggagtttt tttagttatt tttgtggatt atgtgggttt tgttgttatt ttatagatta 14520
ggtattgaag ttttaagagg tgaaattatt tattagagtt atatagttgg taagtattgg 14580
gtttgagagg taaattaggt tagtttggtt ttagggagtt tgtttttaat taaaatgtta 14640
ttatgtttag gtattttaga gtttttggga tatgtaggat agggttagag agacgatata 14700
taaggtttgg gttttcgtgg tagttagaag tttttattta gatttttttt tttttaagat 14760
agagttggga atggcgttgt tatggtaagg ggtgattatt ttattttttt tgttgatttt 14820
tggggtttag agtttaacgt gttgtgggaa ggtgaggaag gaggtcggtg aggttttaga 14880
tagtttaggg tttagaagaa gtttttttgt tttttttttt gagtcggttt tggttggata 14940
tgaagttagt tttttcgcgt ttatttttga tttttaagaa aataggaagt gagcgcgata 15000
agatggagat ataaattttt aaatagtttt tttttttgtt tttttttttt ttttcggagt 15060
tttttttttt cggtatattt tttaggacga ggtgtaggag ttttattttt tttttcgatt 15120
ttgttgggtt agggtgaaat tttatttttt ttttttagga tttgtgtttt cgggttttgc 15180
ggtttgtttt ggtagttttt aaatttgggt cgttttttcg gttcgttttg ttttttgggg 15240
ttagggaatt tttaattttt ttagattatt ttttttatta ttttgaggta agagagggga 15300
agttttgttt tttgagttta gtttttgttt tatttggagg gagatttggg tttatattta 15360
ggttttgtta tgaagttagg gttgttagat atattaaatg aaaattagtc gggtgtggtg 15420
gtttatgttt gtaattttaa tattttggga ggtcgaggtg ggaggatttt ttgagtttag 15480
gagtttaata ttagtttgag taatatggtg aaattttatt tttataaaaa tgtaaaaatt 15540
agttggatgt ggtggtgtat gtttatagtt ttagttattt aggaggtcga ggtgggagga 15600
ttatttgagt ttgggaggtt gaggttgagg ttgtagtgag ttatgatttt gttattgtat 15660
tttagtttga gtaatagagt agagtttgtt ttaaaaaaat aaaaaatagg ttaggcgtag 15720
tggtttatgt ttgtaatttt agtattttgg gaggttaagg taggcggatt atttgaggtt 15780
gggagtttaa gacgagtttg attaatatgg agaagtttta tttttattaa aaatataaaa 15840
aattagttag gtatggtggt gtatgtttgt aattttagtt gttcgggagg ttgaggtagg 15900
agaattattt gaatttagga ggtagaggtt gtagtaagtc gagattatat tgttgtattt 15960
tagtttgggt aataagagta aaatttcgtt ttaaaaaaat aaaataaaat aaataataaa 16020
aaataatttt taaaaataaa taaaaaataa aaatatagga ggttggtcgg gtacggtggt 16080
ttacgtttgt aattttagta ttttgggagg tcgaagtggg tagattattt gaggttagga 16140
gttcgagatt aatttgagta atatggagaa attttatcgt tattaaaaat ataaaattag 16200
ttgggtgtgg tggtgtatgt ttataatttt agttatttgg gaggttgagg taggagaatt 16260
atttgaattt aggaggcgga ggttgcggtg agttaagatt atattattgt attttagttt 16320
gggtaagaag agtaaaattt tattttaaaa aaaaaaaaaa aaaaaaaaaa aaaaaaaaat 16380
atatatatat atatatatgt atgtggatgt gtgggtgtat atatgtatat ttatatatgt 16440
atatatgtat gtatatatgt agatttatgt atatgtgtgt gtatgtatat atatatatat 16500
atatatatat atggggttta gtgaaattcg aattttagat aaataatgaa aaatttagta 16560
taagtacgtt ttatgtaata tttgtaatat attaatattt aaaaaaatgt atttattgtt 16620
tatttgaaat ttaaatttag ttgggtgttt ttttatttat cgattttata cgtagtagcg 16680
ttgtgatttt gggtaggaga cgtttttttt tttagttttg gtttttttta atttatggaa 16740
cgaatataat gatatttatt ttataaggtg gttgtgatga tgtaggaaag tcgttagttt 16800
atgttaagtt tttaggagac gtttggaaag taggagttat tgttattatt ttatttttat 16860
ttggtttttt ttcggatgtt tttttaatag g 16891

<210> 76
<211> 16891
<212> DNA
<213> Artificial Sequence

<220>
<223> chemically treated genomic DNA (Homo sapiens)

<220>
<221> unsure
<222> (8683)

<400> 76

tttgttggag aaatattcgg aaagaggtta ggtgagaata aggtgatgat aatgattttt 60
attttttaaa cgttttttag ggatttgata tgagttaacg gtttttttgt attattatag 120
ttattttatg aggtggatat tattatattc gttttatgga ttaaaaaaaa ttaaggttag 180
gagaggaaac gttttttgtt taaggttata gcgttattac gtatagggtc gatgaataga 240
aggatattta gttaaattta aattttagat aaataatgag tgtatttttt taagtattag 300
tatattgtaa atattgtatg gaacgtattt atattaaatt ttttattgtt tatttgaaat 360
tcggatttta ttgggtttta tgtatatatg tgtgtgtgta tatatatata tatatatata 420
tatatagatt tatatatata tatatatata tatatatata gatatatata tatatattta 480
tatatttata tatatatata tatatatata tatttttttt tttttttttt tttttttttt 540
tttttgagat ggagttttgt tttttttgtt taggttggag tgtaatggtg tgattttggt 600
ttatcgtaat tttcgttttt tgggtttaaa tgattttttt gttttagttt tttaagtagt 660
tgggattata ggtatgtatt attatattta gttaattttg tatttttagt agcgatgggg 720
tttttttatg ttgtttaggt tggtttcgaa tttttggttt taggtgattt gtttatttcg 780
gttttttaaa gtgttgggat tataggcgtg agttatcgtg ttcggttagt tttttatatt 840
tttatttttt atttattttt aaaaattatt ttttattatt tattttattt tatttttttg 900
agacggagtt ttgtttttgt tgtttaggtt ggagtgtaat agtgtgattt cgatttattg 960
taatttttgt tttttgggtt taagtgattt ttttgtttta gtttttcgag tagttaggat 1020
tataggtatg tattattatg tttggttaat tttttgtatt tttagtaaag atggggtttt 1080
tttatgttgg ttaggttcgt tttgaatttt taattttagg tgattcgttt gttttggttt 1140
tttaaagtgt taggattata ggtatgagtt attgcgtttg gtttattttt tatttttttg 1200
agatagattt tgttttgttg tttaggttgg agtgtagtgg taaaattata gtttattgta 1260
gttttagttt taatttttta ggtttaagtg atttttttat ttcggttttt tgagtagttg 1320
gaattatagg tatgtattat tatatttagt taatttttgt atttttatag agatgaggtt 1380
ttattatgtt gtttaagttg gtattgaatt tttggattta agagattttt ttatttcggt 1440
tttttaaagt gttgggatta taggtatgag ttattatatt cggttgattt ttatttgatg 1500
tatttggtaa ttttaatttt atggtagggt ttaggtgtga atttaagttt ttttttaggt 1560
aaggtaggag ttgagtttag gagataaagt tttttttttt ttgttttaag gtggtgggag 1620
gaatggtttg aaggggttga gagtttttta gttttaaggg atagggcgag tcgagaaggc 1680
ggtttaggtt tggaggttgt tagagtagat cgtagagttc ggaaatatag gttttgaggg 1740
aaaggaatgg aattttattt tgatttagta gggtcggaaa aggaaatgag gtttttatat 1800
ttcgttttga ggaatgtgtc gggggaaggg ggtttcggag gagggaagga ggatagaagg 1860
ggagattgtt tggaagtttg tgtttttatt ttgtcgcgtt tattttttgt ttttttggag 1920
attaaagatg ggcgcggaga gattggtttt atgtttagtt aaggtcggtt tagaggaggg 1980
ggtagggagg ttttttttga attttgggtt gtttggagtt ttatcggttt ttttttttat 2040
ttttttatag tacgttgggt tttgggtttt agaagttagt aaaggaagtg agatggttat 2100
tttttgttat ggtaacgtta tttttagttt tgttttggga aggagaagat ttggatgagg 2160
atttttggtt gttacggggg tttaggtttt atatgtcgtt tttttgattt tattttatat 2220
gttttaggga ttttggagtg tttggatata gtggtatttt ggttaaaagt aagttttttg 2280
gagttaggtt gatttggttt gttttttagg tttaatgttt attagttatg tggttttggt 2340
gaatgatttt attttttggg gttttaatat ttggtttata aaatgataat agagtttata 2400
tgatttatag aggtggttgg gaggatttta tgagattggg tatatggtaa ttttttttta 2460
aatttttatt tgtttatttt ttagataagg ttgttttttg ttttttgggt tggagtgtag 2520
tggtgtgata atagtttatt gtagttttga gttgttgggt ttaagcgatt tttttgtttt 2580
agttttttaa gtagttggga ttatagttgt gcgttattac gtttggttaa ttttttaaat 2640
ttttagtaga gattgggttt tattatgttg tttaggttgg ttttaaattt ttgagtttaa 2700
gtagtttttt cgttttagtt ttttaaagtg ttaggattat aggtgtgagt tattatgagt 2760
gagttagtat gtttggtcgg tagttttttt ttaattataa ttatttgatt gattttagtt 2820
attgtagaat tgattgtttt aaggggtggg gtgaagtggc gggatggggt ggtttttagg 2880
tgacggtttt ttgggatgga gaggttattg ggattttttt tattttaata aaattaaggg 2940
ttggtgttat tttttgtttt tagttttttt gatttattag tttttatgtt ttttttattt 3000
tttttttttt ttatcggtga gcgtaggttt tttggttgtt atttagttta agtttttttt 3060
atttagatgg ggagattgag gtttggaggg acgggatagg ttaaagtttt atagtatatg 3120
gttgattagg tcgggtttcg gattcgcgtt ttttaatatg atttagtgtt tggtttgtag 3180
attatcgtag cgtttttttt tttttttttg ggattttttt tgtttttttt ttagattttt 3240
aggaatttta gtttttttcg gattgttggg gtcgtttggg gtcgtttttt ttatgtcgtt 3300
agttagcggg agaggttata agtttggtta ttatgtaatg attagataat aggtttggcg 3360

gggagattag ataagaggtt tatttatttt ggggtttttt cgaattttgg tcgtttttat 3420
ttcgtagacg agaatgggga gataggaggg agtgtgacgt ttgggtttgt ttttcggagt 3480
ttgaggcggg tttagattaa gttgaaaata attttttggt attaagggaa acgttagggt 3540
ttttcgggat tgggttagag gaggagtcgt tgggggtttg ggtgattgga attttaagtg 3600
agagatttag aagttatttt ttattattgt ttttttttta ttttcgttat ttatgtgtat 3660
atagtatttt ttagattttt agagggttat ttttagtatt ttggaggatg attaagaaaa 3720
atagttttgg agttagatgg tttggattaa attttagttt tgttttttaa aatgttggtt 3780
attttgagta gaagtttaat ttttttgagt tttagttttt ttatttgtag attattaata 3840
attattgtag atgtttttat tatgtttatt gggagtttag gtagttttat acgagtgaat 3900
ttatttgatt tttatagtag ttttgtgagg tgggcggcgt tattatattt atgtatattt 3960
taggaaattg aagattaggg cggttaaaga aattgaagat tagttgttcg aggtgattta 4020
gttattaagt gttacggttt ggatttaaat ttagatagtt tggttttagt aagtagtagg 4080
tatttatagg ttgagtgtgt tagtttgatg gggtaatttt tgttgagtgt ttggtattaa 4140
gtaggtgttt tagatataga agttgtgaat attattttat tatattagga tagatatttt 4200
ttttttcgtt gttatttttt tttttttttt ttgagataga gttttatttt gttatttagg 4260
ttagagtata gtgtttcgat ttcggtttat tataattttt tcgttttagg tttaagcgat 4320
ttttatgttt tagttttttg agtaattata agtggattat tatatttagt taatttttgt 4380
attttttgta gagacggggt ttcgtcgtat tgcgtaggtt ggttttaaat ttttgggttt 4440
aagtaattta tttattttag ttttttaagg aagatacgtt ttaataaggg ttttttttgt 4500
tgtaatgttt gtttcgtttt gtttagtatt ttcgggttat tttttatttt ttttaggttt 4560
aattatagtt ttataggttt tttagtattt agtttagtta ttgtttttag ttattaggag 4620
gttttagtat gttttggttt cgggtttttt tttggtttta cgatgtttgt agggttaagt 4680
ttttgtattg tagtttatag tagaggtgaa aagtatagcg atttgataga tgttaaaggt 4740
ttaggttttg atttttttat ttgttagttg tttttttatt tttagtgttt ttatttgtaa 4800
tatggggatg agggttatta taatagtttt ttatggagtc ggtaagtagg ttagtaaatt 4860
atatttgtat agcgttaggt cggtttttgt taagtggggg ttgtttagga attggtgatt 4920
attattataa ttatttatgt ttagttagtg gggatatgtg ggtttttttt ttaaatatat 4980
ttcgtttttg tttttgttat gttttttttt agattaatat ttattttttg tttgattatt 5040
tggttttttt tttttaattt tggagagtcg gggttttgtt ttgtttttta ggttggagtg 5100
tagtggtgta attatagttt attgtaattt ttattttttg gttttaagta attttttgtt 5160
ttagtttttc gagtagttat aggtatgagt tattatgttt aattaaattt taaaaatatt 5220
tgtggagaaa gagttttgtt atgttgttta ggttggtttt gaatgtttgg tcgtaagtga 5280
tttttttatt ttagtttttt aaaacgttgg gattataggt atgagttatt atgtttagtt 5340
tttagtaaat ttttatttat tttttaaaat tttgttttga aaattttatt gaagttagtt 5400
tttttttttt taggtgtatt cgtttttggg tgtttttgga gaattagatt atatatgttt 5460
aggtgtgttg ttttgatttg ttagtgtgtt tgtattttta gtagatagtg agttttttta 5520
ggataaggag agtttatagt attttatgtt ttaattagtt agtttagggt ttagagtaaa 5580
tatttatttt tattggatat agtgagtttt agattttttt ttaaagaatt agtatgttag 5640
tatgtttagt tttttgtttt ttattttaaa gtttaatttt tttatggttt tagtaaataa 5700
ttttttttat tagttttaat tagtagttta tatttgtttt tttggttatt tgttttattt 5760
tgatttattt tggttatgtg ttttgatttt ataaattttt ggttatttgt tttgatttaa 5820
gttattttta gttatttgtt tttaattatt tttttcgtta aattatttat ttcgttattt 5880
tggtttatat ttttgttttt tttaaaatag ttaattggaa ttagtttaga ttgtgcggtt 5940
taatattagt tcgtagggga acgacgtagt agtaggggtt atttgcgtta ggagtaagaa 6000
tttttttttt ttttttgttt agatgtgttt tcgttattat tttatttttg aatcgtattt 6060
ttttgtagaa gtaaaaattg ttttgttgag aagattaaat ttatgtttaa gtgttatttt 6120
tttgcggtat tgaggaataa gtattttgtt tttaatatta taaatattta tttgtggaat 6180
tggttggtta gggttgtttt tgatttgttg ggtgatttat attaagtttt tttattattt 6240
tgggattttg tttttttatg gttagtgggt ggggaagagc gggggtggat aggatatatt 6300
ttaagtttta ttaggggttt gatagtttga gattttattt aaggatgaag gtaggaggaa 6360
gatagagtag ggtatagatg gggttgttga aggtaaaggt agggttttag gggttgtatt 6420
tcggatggga gggtcgttat ggggattaga gaaaaggttg agtgtttgcg gggagaagat 6480
taggtaagag gaagattttt atttttttta ttgtggtagt atattgtagt gtttaggttt 6540
agggtttgag ttttagtggt ttattttagg gtagtgttat ttgggtaaat ttttttattt 6600
gattgagtta tacgagtatt ttagtttttt tatttgttaa atgggggtag aaagagtatt 6660
ttttttgtag ggtttttgta agcgttaagt ttgatagtat atatattacg tttagagtag 6720
cgtttagtat attgtaagtg ttgaataaat gttagttgtt atattatttt tttattttat 6780
ttttattttt gttatagggg ttgaagttag ttgaattttg gttgagggtt tttttagttt 6840
ttaaggttgg tgttttaaag tttgggttgg aattaggaag tagggtttta gagttaaggg 6900
taaatagttt tttaggtttt tagtatttag gttataaaag ttgtggggaa aagaagttga 6960
atttggaagt agacggtttg gagttgaaat ttggatttgt tatttgtttt aagttttaat 7020
ttttttattt ataaattggg gatagaggta aggaggagtt ggtattatgt ttgatatgtt 7080
ggggatagag gagaggtggt cgttttattt ttgtttagat ttatttttag ttttggggta 7140
aaagaatggg tttgtcgttc gagggtttag tattaatata ttcgtgatta taagtaagtt 7200
tttttgtttt tttgggttac gtttgtttgg ttggggtgag ggagaatgtt aatggttacg 7260
tttttcgtgt ttcggggagt ttattttttt ttggtttata cggtttttgt tttaggtttt 7320

tttttttttt agtaaatggg aacgtagttt ttaagagata ggtgtttgtg gtttcgtttt 7380
tgcgttattg attttttttt agtaagtttt agattttaat atagatagtt agtagtatgg 7440
ggattttaag ttttttagta aaggattttt tatatttagg aatatttttt aattttagtt 7500
atttagtata ggttacgagt cgttgtagtt ttgttgtcgg gacgtaattt atatgaaata 7560
atttaaaata taggaaaagg tgtacgtatt aaaattgtgt attgtggtag tatttataat 7620
tagaataaag gggaaattat ttaaatattt aataataagt aaacggtgat aagttgatgg 7680
gatagaatat tttagaagtg taagaggatg gttatggagt ttataggata atatggagat 7740
ttggttcgaa agaaatagat aaaagatttt taaaatgtgg atttatttgg attaaaagta 7800
gtttaaaaag ttagaaaaaa aaattagtaa gttatttatt attatattaa cggtatttgt 7860
attaataaag gagtttcgag gtgatttttt tttttttttt tatttttttt gaggggtaag 7920
ttgaggtagt tatatggtta attttaattg ttttaaaaaa atttttatat gtttgttttt 7980
tttttttttt ttttgatata aagtgttgtt ttgttattta ggttggagtg cggtggtttg 8040
atttcggttt attataattt ttatttttta gggttaagcg atttttttgt tttagttttt 8100
taagtagttg ggattatagg cgtttattat tacgtttggt taatttttgt atttttagtt 8160
aatgatttat gtttgtaatt ttagtatttt tggaggttaa cgtaggtaga ttatttgacg 8220
ttaggagttt tagagtagtt tgtgtaatat gatgaaattt cgtttgtatt gaaaatacga 8280
aaattaggtt tggtggcgcg cgtttgtagt tttagttatt tgggatgttg aggtatatga 8340
attatttgag tttgttaggt ggaagttgta gtgagttgaa attgtattat cgtattttag 8400
tttgggtgat agagcgagat tgtcgtatta gaaatataaa taaataaata aataagattt 8460
gatgagtatt atatttaaat gcgatgtgta gttttggatt gtatgtaagt tttaatagat 8520
gagttattag atatattgtg tagatattgc ggaaaattgt atattggtta ttagttatta 8580
ttagagtgcg tgtgagagtg gtaagagttt gaatttagtg ttattgaaat tttgaattgt 8640
ggttatttag cgaaatgttt ttaatttttg ggtatatttt atntatttta tttttattta 8700
ttttttgaga cggttgtttt taattaaaat aaatcgaaaa aatttaatat gataggaaag 8760
tgtgtttgag aaagttaaag ttttagttag gtatggtggt ttatatttgt aattttagta 8820
ttttgggagg ttgaggtagg cgtattacga ggttaagagt tcgaggttag tttggttaat 8880
atggtgaaat ttcgttttta ttaaaaatat aaaaattagg cgggtgtggg gtcggcgttt 8940
gtagttttag ttattcgaga ggatgaggta ggagaattgt ttgaattcgg gagacggagg 9000
ttgtagcgag tttagattgt gttattgtat tgtagtttgg gtgataagag taagattgtt 9060
ttaaaaaaat ttaaaaaaga aagtgaaagt tttatagaga aaattttaga ttagagaata 9120
gtaatagtgg gttaaataga aaattttatt ttagaatttt ttttgtgtat attgtatatt 9180
attattattt tattgttgtt aaaatggaat tattgttgtt tgggtattta atagtgtgtt 9240
tttgatattc gtttatggta gtgtttatat gattttgttt ttttggtgtt tgttgtatga 9300
tatttcgtcg tgtggtaatt tattaatagt ttttgtttat ggatatttag atgattttta 9360
gttatcgggt ttttgttata ggtatttacg gattacgtat ttttatagag ttggacgaat 9420
aattttagaa ggttgattat tgaaggttga tttgtgaggg tattaagtta tatgtaaggt 9480
atagttttag gagattttta taagtggtgt ttttaaattt tagatttatt tttaggaggt 9540
tagggttatt tcgattttta tttttagttg gtttttaggt tttggtttta ggtttttttt 9600
tgttttttat gggttttagt tttttagatt ataaatgagt tgggttgatg gtttgttgta 9660
tttttgagtt tgtgattttt ggtgtattgg gattagtttt tatttgttta taggaataga 9720
cggatacgtt tttaggagtt tttttaattg gttgttatat agttattatt atatattttt 9780
aaaaggttat gaatgtttaa atttattttt ttttaatcgt tttatatttt tgattgttat 9840
ttttgttttc ggtgtgattt gcgtttgtag agattttgga gaattaatcg ttaggtgtta 9900
tcgggtattg atttttagtt ttaaatttgg tgagttggtg gtttgaagtt agtcgggtgg 9960
gagtatttac gttgtgaaaa tttataaacg ttataaattg agttttttat ttcgttgtta 10020
attatttagt agtatattat tgtttttgat tttttaaaat taggttttaa atgtttgtat 10080
ggattttttt ttaacgatta agaggaagag agtgttattg tagcgataaa tacgagagag 10140
ggagattttg gagtttaggt aggggtttcg ttttagttat gtgaatttgg taggttagag 10200
tttttttttt tttttttgta aaatggggat gatgatggta gttattttgt agggtcgttg 10260
tgagtcgtat taagagggtt cgttttatta tattttatta ggagtaaata tttagggagt 10320
cgtaggtcgg gagttaaatt tttgttaagt tatttaatat gtttgagatt tagttttttg 10380
ttaattaggt ggtgggtaga gttttagaga tttagaatta gggattttaa aatgttatta 10440
ggtggggagg tggggagaag ttggttgttt tatcgttgtt tttttttttt ttttttttta 10500
tatttttagt ttatttttgt tttttttttt ttaaatttgt taggaagttt ttggatgtat 10560
aggtgggtta aaggatgttt ataggatcgg ttagcggttt gaggtggtcg agggataggg 10620
agtgggatat ggggttgggg gttttttgtt ttttatcgtt ttttttttgg ttagttttag 10680
aaagtagtag aggttatgat tttttggaag agtttttagg ttaaggagtt ttatagaatt 10740
tttaatttgg tttgtattta tttttgtatt ttttttgggg attagggagg taggagggag 10800
gtagggtagg gttttgtagt tttggtttgt ttaaagggtt tgaggagttg tttaaaggtt 10860
ttggggaagg tgagttggag gaatttaaag ttaaaaataa aataatatag aagtggttag 10920
tttgttagag ttttttttgt aatgattttt tattatttat ttttttagta gttttgagag 10980
ggaagtttgt tttttttttt atttcgttta gaggttcgaa ggtagttttt taggattata 11040
ttataaggaa gtgtttatgt gagatttgaa tttagagacg gtgtttagag tttaggttgt 11100
gggttttttt ttttatttat tttttatggg gaaatttaag gtttagagag gttaggaaat 11160
tttttcgtcg ttgtatagta ttttgggatt taggtttttc ggggagtaat ggaacgtagt 11220
tgggtgagta gacgttagag attagggtta aagtgaaaga gttagtttag tttgttcgag 11280

gttttttttt aatattgaaa gttttgtata attatttagt tttagtaaat tgggagggtt 11340
ggtgattttt tagagtttta aggatggttt tgttgggcgt gagtttagga tataggaagg 11400
aggtcgggaa tatttggggt ttggttcgtg tatcggaggt cgagtttttt tattttgggt 11460
ttttggatat ttatttgtgg ggttgaggtt gtagttggtt agtagtaggg taatagttag 11520
agggagcgtg tcgcggttta tgttgtttac gtgggggttt gtgcgttggg ttttattttg 11580
tgtttagtgg tagggttgcg ggcgggtatc ggggtcggcg tgggttcgta cggggattcg 11640
aggggagtag gcggtcggag cgacggcgtt tttgttttag ttttttgggg tggcggtcga 11700
ggggttagga gaagtggata tagggacgcg gggttgggtt ggagttgttg ttcgaaggat 11760
gggcggggag ggggtgttgg gttttaatgg atggtagtga tagtagtagt tttggtttta 11820
gggcgtagat gaaggttgtg gtatgatcgg tagtagggag tttttggttt atttagttcg 11880
gtcggtttag ggaggtagga aatgcgtttt tttagagaag gggttgggtt aggtcgtttg 11940
ttaatcgggt agaggaagtg ttacgggttt tgttattggg tggggggttt tgtgatgggg 12000
ggaggtagtg gggtgggagg ttttaggttt taggtttgtt tttttaagta ttttattttt 12060
ttattttatt ttttttaagg ttggtgggtg ggacgtatta gggagtttta tagatgaaaa 12120
taattatttt aatagtttat cgaggggaat ttttaggggg tcggttttgg ttaaagtagt 12180
ttttgtagaa tttgtgattt atttgagttt attgatttta ttttgtagat gaggaaatag 12240
aaattcgatg tgttgagatt attggtttaa gaagatggta gcgttgggat tttgtttatt 12300
tttattattt tacgttttgt cgggtattaa attcggttta ttaagtgtag ttgatatggg 12360
gttgtttaat aggtaggaaa attgaagttt aggttaggga gggaagtttt aaagtggtat 12420
agtaaggcgg ttagggttgg cgtttttggg tttagaggag ttgtttaggg ttatatattt 12480
tacgtttgat ttttttatgg gtggaaaatt ttttatcgat aaaatatagt tttaggtcga 12540
gtgtagtggt ttaagtttat aattttagta ttttgagagg tcgaggtaga cggattattt 12600
gaggttagga gttcgagatt agtttgatta atatggtgaa aatttatttt tattaaaaat 12660
ataaaagtta gttgggtatg atgacgtgcg tttgtagttt tagttatttg ggaggttgag 12720
gttagagaat tatttgaatt taggaggcgg aggttgtagt gagttgagat tagattattg 12780
tatttttggt ttaggtgata gagtgaggtt cggttttaaa aaaaagaaaa gaaaaaaaaa 12840
aagaagatag ttttaaatta tggggatttt aatggtattt ttgaagttac gtggttttgg 12900
gtttaaattc gattttgttt gtttttagtt atgagatttt ggataaggtt ttttaatttt 12960
tagtattttt tgtttgtaaa gtggggtacg atgttttttt gattaagtta ttatgagatt 13020
tttgagggtt aggatattat agtattgagt tttgggatat ataggagatg tttttttatt 13080
agtgtttttt gagttgggtg atagttttta aattgtttta tgtttatttt gtaaatagta 13140
agcgtattta ttatgtattt atcgtgggat tatttatagg tagtatttag ggttaggttt 13200
atgaaagttt agagaaggaa ttttgggtta tttggagggt ttggtattta ggtttgtttt 13260
ttgaaagatt gggtgagttg ggattgggga gaagaagggg tgaggaaagt atgttaggta 13320
ggggtgttag attaagtaaa ggtatagaag taggaaatag tcggattatc gtgagattag 13380
taggggtgtg gtggggatag gaagttggag aggtagagtt tggttttgta ttgggagggt 13440
tttaaatgtt aggattttgt ttttagggtt attgggagtt atggagagtt tttgagtaga 13500
aggacgttgg tagagttagg ttttgagagg ttaaattttt gtgtgtgttg gtaggattta 13560
cggtgagatg gagttagaag gttttttttt ttggatttat ttgttttggg ttataggtgg 13620
agaaattgag gtttagggag ggaggagaag gttgaggttt tgggtgtttt tggagggagg 13680
atgtttgttt agggtttgag gttttgtttt ttcgtgttta ttggtcggtt cgggtgataa 13740
gagtaggtcg tgtgtttgtt tttgtaggag ttgttatagc ggttgggagg gtcggtgtgg 13800
ttttttgggt ggggattagg gatagttttg tttgtttagt tttgtggttg tttggttagt 13860
ttgtagttaa gcgtggtttt gaggttaagg gagagtgttt ttttttagag ggttattttg 13920
agaagggtat tttttttgtt tttttttgta atttcggaga taaggttatg ggtattgcga 13980
ggtaaagggt ggttcggtgg gggtttaggg gtggttttga ggtttttata ggttttagat 14040
cgagatttgt gttttaggga tagttaattt agttttagga gttgagcgtt ttttttgtat 14100
tagggttcgt tttaggcgtt tattttatta gttttgaatt tttatagtta ttttaggagg 14160
tgggtatcgt tatattttta ttttatagag gaggaatttt agttggaggg tggtgatttt 14220
agggttgttt gggtttaagt ttattttttt ttttgttatt attttttgag ttgagtttgg 14280
agtgagggat agatgtggta ggggttgtag gtattatagg gtgttggggg tatttggggt 14340
agtgataggg attttatagg aggttttttt taaaattttt tttttttttt tttttttacg 14400
ttgtatagtt tgtttttatt tacgttgtaa ataagattta atggattttt tagaaagtag 14460
gagagatttt agatggattt taggaagaat tggggtttcg attttagata ttttatggag 14520
gaaggaagag attgggatta tcgcgggtgg ggggtgaagc ggaagttatt tttggttttt 14580
atagtatttt gatttttttt gttatattta agtttgtgtg tagttatttg tatgtatttt 14640
tttatttgat tagaagtttt atgaaggtag agattttgat attttttgta ttgttgtgtt 14700
tttagtatag agtttagggt tttttagttt ttgagatgga tttgttgaaa gtatgaggga 14760
ttttagaagt taaggtaggg gtgggggtta tggaggttta gagaagtggg ttttgttatt 14820
ggtttttagg agaaaaggtt agttttggat tgggtttttt gttcgaggaa gtttggtggt 14880
tatatggttt ttttgttggg tataggtttt ggggtagttg ttttttatat tttttagttg 14940
tatttttttt tttgtgggat atatttattt ttttattttt gtagaggtta gaggaggtag 15000
gaggaggagg gttcgttttt tagttttttt tttgtagggt tattgtatat tggtggtttt 15060
tttttattga aggttacggt tttgttgggg atttttttta tttagttttg tttagttgtg 15120
gatttagttt tttttttggt ttttttaggt tgagggtggt tatagtttac gtcgttgtta 15180
gttttggggt tttgtatcgt tttttggggt ttttttatat ttcgattata tttgtgtaaa 15240

tagatttttt attaaatttt tttttaatga ttttattgga acgtgttatt tgttttttgt 15300
cggggttttg attgatacgg atgtgtttag gtgtcgtttg tttatacgtt cgagagacgc 15360
gtggttgatg gttaggtttt tgtgtttgga ttttatagag ggtagatgta tattttttgt 15420
tttttttttt ttttggttgg ttaggaaggt tagggttgta ggaaaagata gagtacgttt 15480
attttggtta ttggttattg aaggaatttt aggcggtttt tagggattta ttaggcggtt 15540
gattgttttt agtattggtt tggagttagg ggttattgat aatagtaggg ttagatttat 15600
agggcggagt agggtgttta ggttttgttt tatattggtt aggtggtatt aggtaggatt 15660
tttgtttgtt tgagatagag ttttattttg ttatttaggt tggagtgtag tggtatgatt 15720
ttggtttatt gtaattttcg ttttttgggt ttaagtaatt tttttgtttt agtttttcga 15780
gtagttggga atatgtatta ttatattcgg ttaatttttg tatttttagt agagacggga 15840
ttttattatg ttgtttaggt tggttttgaa tttttgattt taagtgattt attcgtttta 15900
tttttttaaa gtgttgggat tataggcgtg agttatcgta tttagttaga tttttttatt 15960
ttttgagttt tagttttttt atttgtataa ggggagggta gtttgttggt ttggaggagt 16020
ttaaagggta agataagaat tggaattgta ttattattta tgaggttgtt tggttttgtg 16080
ttaagtgtta ttttgtagtt attttagtgg ttgaggttgt tatgttagta ttttacgatg 16140
gaagaaattg aggtttaggt ttgtaaggtt atagggttgg gaagaggtag aggataattg 16200
aaatttaggt tttttttttt tggtttttag aatattttgt tttggttttt tttttatttt 16260
tttggttatt ttatttgttt tttttttggt ttttttttgt ttttttaggt ttggttgttg 16320
gttttttttt tttttgttta tttttatttt taagttaggt tttcgtaaat ttttgcgatt 16380
ttggtgttac gttttaatat agattttggg atttagattt aaataatgga aacgtatttt 16440
gtggttttgt agatagattt gcggttgtga tgttttttag taatttttaa tttgttgtat 16500
ttttttttat tattcgttat aagtttattt gttggttttg ttatttatta acgagtttcg 16560
gttataaaat gttatgtttt agattgttgt tatttaaagt gtggttttgt tttttttgga 16620
agttttttat aaatgtagat ttgagttagg tttagtggta aaagtttgta gtttcggtta 16680
tttaggaggt tgaggtagga ggattgtttg agtttaggag tttaaggttg tagtaagttg 16740
tgattacgtt attgtgtttt agtttgggtg atagagtaag attttgtttt taaaaaataa 16800
aaaatgaagg atcgggtgtg gtggtttacg tttgtaattt tagtattttg ggaggttaag 16860
gtgggtggat tatatgaggt taggagtttg a 16891

<210> 77
<211> 6971
<212> DNA
<213> Artificial Sequence

<220>
<223> chemically treated genomic DNA (Homo sapiens)

<220>
<221> unsure
<222> (6637)

<400> 77

ttagttaaac gtttttaaaa tatatttttt tgagagtgtt ttattttttt tgtttaatgt 60
ggtttttttt gttattatat atttttatat atatgatgag gaagaagaaa aagatgatga 120
taatgatgat gataaaatag tagttaaata tagaaattta ttagtagttt aaaaattatt 180
tttataaaaa aggtaagtta tgattttgtg ttgttgttaa agatttttga tttaattttt 240
aggagaagat aatttatttt ttgtaataag taattattag tgtgtttatt tattaatttt 300
tattttagga tttttttatt tttttatatt ttaatagtga attataaagt gtattagtaa 360
tgtagatata tagatttaaa gtaaagataa tagtttaaat tggttatttt tgttgaaaaa 420
aatattatgg gtttaatata atattttacg ttatatatgt gtagttttgg tttggggttt 480
ttttgttttg ttttgttttt gtttttttgt aaagtaagtg gttagtgtat tttagtgtta 540
tggtgaaata taaattttaa aaatttttat aaaagtgaaa tttttggtgg gagtattttg 600
tttttgttag tagaattaat attgtttgtt tttgttaaga tgttttattt tacgggatat 660
ttgtttaaaa aaaaatgaaa tatgaataat ttaacggtga ttaaagtttt tttttttttt 720
ggaaagtata gtagaggtgg tagatttggt tattagagaa taaagggtta aagagtgaat 780
ttgagttggt gaaataaaat gtaatattaa taaagaaagg gttatagata aatagtaaaa 840
ggattaattt attataaaaa atttattgac gtttttttag agtttaggcg aagcgttggt 900
gatcggttaa tgttggaatg tttttttagg gttaaaagga gaatcggtag taagtttgtt 960
tagaatttat aaagtgtttg aataatatgg atagtgtttt gataatacgt ttttagtggg 1020
ggtgaaaata atagtattta ttgggatatt tatatgggtt tttttggttg tggggggtgg 1080
gtggtatatg gggagaggta aattttatat tattgtattt atttataatt tatttttgta 1140
atatatttat atgtgtgtaa atgtattttt tatataataa ttttgtttta attaaatgtt 1200
ttttagtttt gtttttaaaa gattaatgta tggttagtgg ttatttttaa gggggagaaa 1260
gagataaaga ggttatttgg ttaagttggt aataattttg attaagatag tggtatgaag 1320
ttggtattat tttttagaat aatagagcgt gagattttta atgtgtttat atatagattt 1380

ggttttggtt ttttggtagt ttttaaagat ttttgtttgt tttattttag gttgaaaggt 1440
ataagatttg agttaattta gtaggtattt gggtttgggg ttttaagggg tttttatgtt 1500
ttttttttag tgttgatgtt tttgtttaat gtattttaat atattgttag ttgagttata 1560
taaaattagg tattttttaa taaatgtttt atttggattt tatgaagttt ggggtgtaat 1620
tgaaattaaa tagtaaatag gttggaaggt tttgttattt gtttatttag tttaaggagg 1680
gtgttgtttg atatgtttat ttaagttatt gttttggttt ttttttttat ttttattttt 1740
ggaaaggagt gtttggattt taaagtgtgg ttgggatacg tgaaaatatt ttttatgttt 1800
ttttttttgg ttataatatt gagttagtaa aaggaaagtt tattttaagt aggaaattat 1860
agatttgttt atttataatt ttttaggtat tttagattag tatattttaa atgtaataat 1920
aataataata atgtgattag ttaattaata tagaggaatt attaaatgag attttttatt 1980
tttagttaga aaaaaaaaga tttgtagttg aaatagtgtt ttttattatt ttaaagagaa 2040
tttgtaatat atatttataa atgtaatgta atataatatg taatataata ttatgtatat 2100
attttttatg ttttaaaaat tttgggtttt agagtagttt tatagaaatt gaatttttat 2160
tttttttgaa tttgtttttg tttttttata tgtaaaatgt gttgaagttt gggaaaaata 2220
atagatttat tggggaagtg ggtagatatt aattaaaatt ttttgttttt aagaattaat 2280
ataaatttta attatatata tcgtgtgtat atttaataat taggtggtgt ttatattgaa 2340
tttatgcgtt tttaaaagtt tgttttgggg ttggaataaa attaacgttt tttgatgtgg 2400
ttcgggggat agttggttcg cgttattcgc gttagagtgg gtcggaacga tgtttgttgt 2460
ttgcgatggt attttgttac gcgaagcgtt tttttggttg cgttaaaagt attttgaagg 2520
gacgtcgagg ttcgtttttt gttttgttaa agcgtaaaat tgttatgtat ttttggttat 2580
tttagtttta aataacgtga gattaggttt tatattaggg agatttttta tttttatttt 2640
tgttttagat attttgtaat ttaggtaaag gataatgtgg aggattttac gaatagtaat 2700
taggattcgt tagtttaggg atagatacgt cgtttaagtc gtttaagttg tacggttttt 2760
tttttttttt gagttcgcgt attttttacg ggatttttac gttgatagtt atgaggtatt 2820
tatagagtta tattatttga ataattaatg ttttagattt ttttgggtta aaataatatt 2880
aaagtagggg aggaaggtat ggataatgta gtgattaaaa tagtcgtgtt aaatattaat 2940
tatattgatg atataaaacg taggaagcgt agggtagaac gcgtttagta ttagtttttt 3000
ggcgatttgt ttttggttat tttgttttag gatttataag gttatcgttt tagtttatta 3060
gttttaattt gttttttttt ttgtggttgt gttttgattt tgaaaatttt cgcgtatttt 3120
tgggttttat tattatatat acgcgagcgc gcgagcgcgc gaatatatat attaaaaaag 3180
aaaaaaaaaa tcgtgataaa tgttttggag agtttaagtg tattaaggat ttcgttttcg 3240
gagattttag atatgtcgat gttggtgttg ttgtggttgg aaaattgggt tggaaagttt 3300
cgtattgttt tatatttgcg tgtgcgtatt tagtaatttt ttggttattt tatttgttgt 3360
gggcgaagag tttttcgtgt gatcgcgttc ggttggggaa gtagagtttc gatattttag 3420
tcggaaaatg cgttttcgga gcgattattg gcggcgtttg taattgttta ttaatagcga 3480
atatttaggt ttttttttat tcgtaacgaa acgtgttttt cgttttcgta ataatgaaac 3540
gataaaatat gacggtttcg tttttgaatt tatttgagga aacgtagcga agaaataagt 3600
agttggagtt taatttaata ttaaattgat tattttatta attattttat tttttgtatt 3660
tgtcgtagag tagaggcgta gggaatttta attgtggggt tgttagggaa gttttgtttt 3720
ttggtgttag gtttaattga agatggtggt gaagggacgg aattcgggat taggaaaatt 3780
gagtagaaag gattttaaat tgtggggaat aaaagtgatt gttttttcga aagtagttgt 3840
gttggcgtga gtatgaagtt ggtgttagtt cgtttagttt tatttttgtt ttgtttttag 3900
tattttgttt aagtttttga tttagaagtt gagagtttag gttttttagg atggtaatgt 3960
gttttggtcg ttttcggaat tttaaaagtt tagcgcgagt ttttttttta taaatttttt 4020
ttattgggta aaaggatggg aataaataaa aagtggtttt gtgcgttgaa ggttagggag 4080
tttgttttcg ataagaggaa ggtgtgtttt gaagttatag ttttcgacga ggaaagagat 4140
ttttttgggt ttgtttttaa tttgttttag tttttttagt tttcgggacg ggcgtttttt 4200
tgtaagttat tagtttgaag agtttttttt tttttggttt ttaaaatttg ttttagttat 4260
tttatttttt tagcggaggg ttgagatgat taatgcgtta agggtggata tcgttatgga 4320
gattttaaat tttgtttttt taaatttcgg cgaaattata gatattagga ttgattatta 4380
tagcgggacg tagttttaaa aaaaaaaaaa aaaaaaaaaa aaaaaaaagg aaaagaaagt 4440
tataggcgat ttgatttaat tagtaaatgt ttaattgaat tagtgttatt tttattagtt 4500
aataggaggg ttttcgggtt gtgaggtcgg gaagtttttt tttggtcgta aatatataaa 4560
gtagttagtg atagtttata agtttttaag tggttaattt gatcgatgtt ttggtaattg 4620
aaaaagggta gaaaggcgcg ggttagtggg tggatgggga taaagattta agttattttt 4680
ttttagcgtg tgagtttggg aggagggtgg gggttttgag gagtaagagg tacgaggaag 4740
gaaaaggaga gggtttttgg gttagttttt attttttgtt ttttaattta cggcgttttt 4800
ttttcggaaa ggacgttgga tttagggcgc gttagtacgc gtagtagcgg ttcgcgagtc 4860
ggtaggtggg tagtttcggc gcgggaggaa ggggaagtta tttttttttc ggaagagggc 4920
gttggttttt ttattttgtt tttataataa ggttatcgga ggagaggaag tagttagttg 4980
tcgtttgcgt tttgtaaagt atgtagttag gggagtagtt tttggtgagt ttagtgaatt 5040
tgtttggcgc gtatttttat tcgttggaga gtgcgcgagg cggtagcggc gggagcgttg 5100
gttatttttt tagcgcggtt tttttttttt agaagttgga tttagataaa gcgtttaaga 5160
agtttttcgg tagttttttt tgcgaggcgg tgagcgggga gttcgtagtc gttagcgtag 5220
gggttttcgc ggttatgttt agtgatatcg acgtcgggga cgtatttgtt agcgttgcgg 5280
tagtggttaa gtcggggttt tcggacggtc gtaagggttt tttttgcggg gaggaggagt 5340

tgtttttcgt cgttgtagtc gtcgtcgtcg tcgtcgtcgc ggttgcggtt attgcgcgtt 5400
attttatgga tagtttgagt ttcgagcggt attattttta gtttttcggt ttttaggggt 5460
cggagttggt tgcgttttgt ttattttttt cgtattaggc ggcggttggg gcgttttacg 5520
gatttgtgta ttcggttttt aacggggcgc gttattttta cggttttatg ttgtttttcg 5580
gcggtttttt cgcggttgtg tgtttattcg ggagggcgta gttcggttta ggagtcggtg 5640
cgggtagtgg cgcgggcggt agtagcggcg ggggcggcgg ttcgggtatt tattagtata 5700
gttagggggt ttcgttttac gggtcgtatt ttggagtcgt agcggcggga ttttgcggag 5760
gattgggggg tttgggggtt ttaggttttg gttttcgtgt ttacgtttat ttgtgtaatc 5820
ggtttttgtg gtttaaattt tatcgttatt aaattgagat gattattacg aaatagggta 5880
ggtgagcgta gcgtggaggg gtttttgggt tcggggataa agggtgacgt gtggttttgg 5940
ggaaggttcg ggatgaggga gttgaattta gttgtttcgc gcggtgttta gagtgtttta 6000
tcgcgcgtaa taaattttcg tagatatcgt atttttaatg cggtgttttt ttgtgttagt 6060
gtttcggcgg tttttgtagg gtgttaatgt tacggttgta tatcgttttt tttttttatt 6120
atttttattt gaatagattt ggaggaggcg gaaatggaaa ttagggtatg tttataagtt 6180
ggattcgtta gatttttttg ttcggcggta tgtttgtgta tgtttgtatg tcggtaagga 6240
gttacggata gggaaggttt aagagttggt tatttgagcg tagtagatgg gttttcgtag 6300
ttttaggcgt tagggttttt acggggcgtt ttcgggaggt gggtatgaag gttagagcgt 6360
cggtttcgga agttcggtcg gagtaggtgg ttagatttgt tttggagtta gatttggttc 6420
gggttcggtg cgcgagaaag ttttgatata tatttaattg ttaatattgg ttatttttgg 6480
ggaaagggag gggtgggtgt tgagagaggg gtatttaaag gggattttta gtttaaagtt 6540
ttaatatata tttttaataa gaataattta atattgtttt ttggtatttt ttaaaatggt 6600
ggtatttaaa aattagtagt ttttaatatt ttaggaaaaa aattttaaga gcgtttgaaa 6660
gggtcgagaa tatgagtttt cggaagtacg cgcgaatttg gaatatatcg atgttggggt 6720
aagttttttt tttttttttt tttttttttt tttttttttt ttttgaaaag acggaaaggt 6780
tttaagagtt gttaatttaa aaatatttta tatagtttta gattatttga tttattagta 6840
ttttgtttat tgtaaatttt taaatgtgtt tcgattttat tttaggtgtt tggggaattc 6900
gttggggttt gggtttgttt taggatattt taattaaaat cgattttttt gattttattt 6960
ttttgtatag g 6971

<210> 78
<211> 6971
<212> DNA
<213> Artificial Sequence

<220>
<223> chemically treated genomic DNA (Homo sapiens)

<220>
<221> unsure
<222> (335)

<400> 78

tttgtgtaag ggaatagaat tagaaaaatc gattttaatt gggatgtttt agaataggtt 60
taggttttag cgggtttttt agatatttgg gatagggtcg gaatatattt aaaagtttgt 120
agtgaataga atgttggtag attaagtggt ttgaaattat gtaaaatatt tttgagttgg 180
taatttttgg aattttttcg tttttttaaa aaaaaaaaaa aaaaaaaaaa aaaaaaaaaa 240
aaaaaaagtt tgttttaata tcggtatatt ttaaattcgc gcgtgttttc gaaggtttat 300
attttcggtt tttttagacg tttttaaaat ttttntttta gaatgttaaa aattattaat 360
ttttaaatgt tattattttg aaaagtatta gagaataata ttaggttatt tttgttaaaa 420
atatatatta aaattttagg ttaaaagttt tttttgagtg tttttttttt agtatttatt 480
tttttttttt tttagaagta gttagtgtta gtagttggat gtatattaga attttttcgc 540
gtatcgaatt cgagttaggt ttggttttag ggtagattta attatttatt tcgatcgagt 600
tttcgaggtc ggcgttttag tttttatgtt tatttttcgg agacgtttcg tggagatttt 660
ggcgtttaaa attgcgggag tttatttatt gcgtttaggt ggttagtttt tgagtttttt 720
ttattcgtgg ttttttgtcg gtatgtaggt atgtataagt atatcgtcga gtaggaggat 780
ttaacgaatt tagtttgtgg atatgtttta atttttattt tcgttttttt taggtttgtt 840
taggtgagga tgatgggagg gaaaagcggt gtatagtcgt aatattggta ttttgtaaga 900
gtcgtcgggg tattgatata gagggatatc gtattggaga tgcggtgttt acggagattt 960
attgcgcgcg gtgaaatatt ttaagtatcg cgcggaataa ttgggtttag tttttttatt 1020
tcggattttt tttaggatta tacgttattt tttattttcg aatttagggg tttttttacg 1080
ttgcgtttat ttgttttgtt tcgtaatgat tattttagtt tggtggcggt ggaatttgag 1140
ttatagaggt cggttgtata ggtagacgtg ggtacggaag ttagaatttg gaatttttag 1200
gttttttagt ttttcgtaag atttcgtcgt tgcggtttta gggtacggtt cgtagagcgg 1260
agttttttgg ttgtattgat aggtgttcgg gtcgtcgttt tcgtcgttgt tatcgttcgc 1320
gttattgttc gtatcggttt ttgggtcgaa ttgcgttttt tcgggtgggt atatagtcgc 1380

ggggaagtcg tcggggggta gtatggagtc gtaggggtag cgcgtttcgt taggagtcgg 1440
gtatataggt tcgtggggcg ttttagtcgt cgtttggtac gggaagagtg agtagggcgt 1500
agttagtttc gatttttgag gatcggggga ttggaggtag tatcgttcgg agtttaggtt 1560
gtttatggag tagcgcgtag tggtcgtagt cgcggcggcg gcggcggcgg cggttgtagc 1620
ggcggagggt agtttttttt tttcgtaggg ggagtttttg cggtcgttcg ggggtttcgg 1680
tttggttatt gtcgtagcgt tggtaaatgc gttttcggcg tcggtgttat taagtatggt 1740
cgcgggggtt tttgcgttgg cggttgcggg tttttcgttt atcgtttcgt aggagagatt 1800
gtcggaaaat tttttggacg ttttgtttaa gtttaatttt tgaggagagg gggtcgcgtt 1860
ggggaggtgg ttagcgtttt cgtcgttgtc gtttcgcgta ttttttagcg ggtagaagtg 1920
cgcgttaggt aggtttattg agtttattaa gagttgtttt tttaattgta tgttttgtaa 1980
agcgtagacg gtagttggtt gttttttttt tttcggtggt tttattataa aggtaggatg 2040
ggggagttag cgtttttttt cgaggggaag gtaatttttt ttttttttcg cgtcggggtt 2100
atttatttgt cgattcgcgg gtcgttattg cgcgtattgg cgcgttttga atttagcgtt 2160
tttttcggaa ggaaagcgtc gtgagttgga aagtaggagg tggaaattaa tttagaagtt 2220
tttttttttt tttttttcgt attttttgtt ttttaggatt tttatttttt ttttaggttt 2280
atacgttgga agaaggtgat ttagattttt gtttttattt atttattagt tcgcgttttt 2340
ttgttttttt ttaattgtta aagtatcggt taagttgatt atttggaaat ttatgggttg 2400
ttattagttg ttttatatat ttgcggttag ggaggggttt ttcggtttta tagttcggag 2460
atttttttat tggttggtgg aggtgatatt aatttaatta ggtatttatt aattggatta 2520
agtcgtttgt gatttttttt tttttttttt tttttttttt tttttttttt ttttgagatt 2580
gcgtttcgtt gtgataatta attttggtgt ttgtaatttc gtcggaattt agggggataa 2640
agtttggggt ttttatagcg gtgtttattt ttggcgtatt agttatttta gtttttcgtt 2700
ggaggaatga gatggttgga gtagatttta gaggttagag agagaaagat tttttaagtt 2760
gatagtttgt agggaagcgt tcgtttcgga ggttggggaa attggggtaa gttggagata 2820
ggtttaagga agtttttttt ttcgtcggaa gttgtaattt taaagtatat tttttttttg 2880
tcgaaggtaa attttttggt ttttaacgta tagaattatt ttttgtttgt ttttattttt 2940
ttatttagtg ggaaaggttt gtagagagga aattcgcgtt aagtttttgg gatttcgggg 3000
acggttagag tatattgtta ttttggagga tttaagtttt tagtttttaa attagaagtt 3060
tggatagggt attggggata ggataagagt gaaattaagc gagttaatat taattttata 3120
tttacgttaa tataattgtt ttcggggaga tagttatttt tattttttat aatttaaaat 3180
tttttttgtt tagttttttt gatttcggat ttcgtttttt tattattatt tttagttagg 3240
tttgatatta agaaataaag tttttttaat agttttatag ttaggatttt ttgcgttttt 3300
gttttgcggt aaatatagaa ggtagaataa ttagtgaaat aattagtttg atattgaatt 3360
aaattttagt tgtttgtttt ttcgttgcgt ttttttagat agatttaaga gcggggtcgt 3420
tatattttat cgttttatta ttacggaagc ggggggtacg tttcgttgcg gataaggaga 3480
agtttgaata ttcgttgtta ataagtaatt atagacgtcg ttagtaatcg tttcgggagc 3540
gtatttttcg gttgagatgt cgggattttg tttttttaat cgaacgcgat tatacgggaa 3600
atttttcgtt tataatagat gagatggtta aaggattgtt gagtgcgtat acgtaagtgt 3660
agaatagtgc gaagtttttt aatttagttt tttaattata gtagtattaa tatcggtatg 3720
tttggggttt tcgagaacga ggtttttaat gtatttagat tttttagggt atttattacg 3780
gttttttttt ttttttttga tgtgtgtgtt cgcgcgttcg cgcgttcgcg tgtgtgtggt 3840
ggtgaaattt aggagtgcgc gaagattttt agggttaagg tatagttata ggggaggggg 3900
taagttgaga ttagtgggtt ggggcggtgg ttttgtaggt tttgggatag agtggttagg 3960
agtagatcgt tagaaagttg gtattgagcg cgttttgttt tgcgtttttt gcgttttgtg 4020
ttattagtat agttagtgtt tggtacggtt gttttgatta ttatattgtt tatgtttttt 4080
ttttttgttt tagtgttatt ttgatttaag gaagtttaga atattgattg tttaagtagt 4140
atagttttgt gaatatttta tgattgttaa cgtgaagatt tcgtgagggg tacgcgagtt 4200
taaggaaaga aagaggtcgt gtagtttggg cggtttggac gacgtattta tttttgggtt 4260
agcgggtttt gattattatt cgtaggattt tttatattat tttttatttg agttgtaaga 4320
tatttaaggt aaaagtaggg atgagaaatt tttttaatgt gagatttaat tttacgttat 4380
ttggagttag ggtggttaaa ggtatatgat agttttgcgt tttaataaag taagaagcgg 4440
gtttcggcgt ttttttaaaa tgtttttgac gtagttaggg aagcgtttcg cgtggtaaag 4500
tgttatcgta ggtaataggt atcgtttcga tttattttgg cgcgaataac gcgggttagt 4560
tgtttttcga gttatattag aaagcgttga ttttatttta gttttagagt aagtttttga 4620
aagcgtatga gtttagtatg agtattattt aattattaaa tatatatacg atgtatataa 4680
ttagaatttg tattgatttt tggggatagg aaattttggt tgatatttat ttattttttt 4740
agtaaattta ttattttttt taaattttaa tatattttat atatggagga gtaaagataa 4800
atttaaagag aataaaaatt tagtttttat aaaattattt taggatttag gatttttgga 4860
atatggaaaa tatgtatata atattatatt atatattgta ttatattata tttgtaagta 4920
tatgttataa atttttttta aaataatgaa aagtattgtt ttagttatag attttttttt 4980
ttttggttga agataaaaaa ttttatttaa tggttttttt gtgttaatta gttaattata 5040
ttattattat tattattata tttagaatat attagtttag aatgtttgga gagttataga 5100
tgagtaagtt tgtgattttt tgtttgaaat gggttttttt tttgttggtt taatattata 5160
gttaggggaa gagatataaa aaatattttt acgtgtttta gttatatttt aaaatttagg 5220
tatttttttt tagaagtagg aatagaaaag aaagttaaag tagtggttta agtgaatatg 5280
ttaggtagta ttttttttag attgggtggg taaataatag agttttttag tttgtttgtt 5340

gtttgatttt aattatattt taagttttat gaaatttaaa tgagatattt attgaaaggt 5400
gtttggtttt atgtggttta attgatagtg tattggaata tattgaataa aaatattaat 5460
attggggagg aaatatggaa attttttgaa attttagatt taaatatttg ttggattaat 5520
ttaaattttg tattttttag tttgagatgg agtaagtagg aatttttaaa ggttattagg 5580
aagttaaggt taagtttgtg tgtgaatata ttaaagattt tacgttttat tgttttgagg 5640
aataatatta attttatatt attgttttgg ttaaagttat tgttaatttg attagatgat 5700
tttttatttt tttttttttt tttaaaggta attattagtt atgtattgat tttttaaaag 5760
taaaattgag aagtatttgg ttggaatagg attattatat aagaaatata tttatatata 5820
tatgggtatg ttatagaggt gagttataga tgagtgtaat gatataaggt ttattttttt 5880
ttatgtgtta tttatttttt ataattaagg gggtttatat aaatgtttta ataaatatta 5940
ttatttttat ttttattaaa aacgtgttat taaaatattg tttatgttat ttaaatattt 6000
tgtaaatttt gagtagattt gttgtcggtt ttttttttaa ttttaggaaa atattttaat 6060
attaatcggt tattagcgtt tcgtttggat tttgggaaag cgttaatggg ttttttgtgg 6120
tgagttggtt tttttattat ttatttatgg tttttttttt gttgatgtta tattttattt 6180
tattaattta ggtttatttt ttggtttttt gttttttgat ggttagattt gttatttttg 6240
ttgtgttttt taaagaggga gggagtttta attatcgtta ggttgtttat gttttatttt 6300
tttttaagta aatgtttcgt aagataaaat attttggtaa agataagtag tgttgatttt 6360
gttgataaga gtaagatatt tttattaaaa gttttatttt tataagaatt tttagaattt 6420
atattttatt atgatattga aatatattga ttatttgttt tataaaaaaa taaaaataaa 6480
ataaaataaa aaaattttaa attagagtta tatatgtgtg acgtgaagtg ttatattaaa 6540
tttatggtat tttttttaat aaaaatgatt aatttaaatt attatttttg ttttaagttt 6600
atatatttat attgttaatg tattttgtgg tttattgtta aaatatgagg aggtgaagga 6660
attttgaaat ggaagttaat aaataaatat attaatagtt gtttattata aaagataaat 6720
tatttttttt taaagattga attaagagtt tttggtaata gtataaaatt atgatttatt 6780
ttttttatga aagtaatttt taaattgtta atgaattttt gtatttagtt gttgttttat 6840
tattattatt attattattt tttttttttt ttttattatg tatatgggaa tatataataa 6900
taagaaaagt tatattaggt aaagaaagta aaatattttt aagaaagtgt attttgggaa 6960
cgtttggtta a 6971

<210> 79
<211> 9830
<212> DNA
<213> Artificial Sequence

<220>
<223> chemically treated genomic DNA (Homo sapiens)

<400> 79

tgtgtatggt ggtgtgtgtt tgtaatttta gtaattaggg aagttgaggt aggagaattt 60
tttgaattcg ggaggtggag gttgtagtga gtcgaggttg cgttattgta ttttagtttc 120
ggtgatagag taagatttcg tttaaaaaaa aaggaaatga atggaggaaa ttaatttaga 180
atgttattat taaatgtgag tatttaaggt taagtatagt ggtttacgtt tgtaatttta 240
gtattttagg aggtcgagga gggcggatta tgaggttagg agtttaagat tagtttgatt 300
aatatggtga aattttgttt ttattaaaaa tataaaaatt agttaggtgt ggtggcgtgt 360
atttgtaatt ttagatattt aggaggttga ggtaggagaa ttatttgaat tcgggaggtg 420
gaggttgtag tgagttgaga ttgcgttatt gtattttagt ttgggggata gagtaagatt 480
ttgttttaaa aaaaaaaaaa aaaaaaaaat tagtatttat tgagtagaat gtgtggggaa 540
ttgttttatt tgttgttgga tttatatttt gtttttgagg gatttatagt ttagttgaga 600
agatagttta gaggttttga aaaataaaaa agagtttagt tggttgagag tagggtgttg 660
gtaggtatgt agtatggagg tttttggggt tttagggggg gtttgatttt tgggggtttt 720
atttaagagg tagtaattag attgatttta aagattttag ttgcggagag ttggtatatt 780
ttaggacggg gtattgtttt ttaggtttag tggaatttag aggtaatggt tgaggtaggt 840
atttatgaga gtgcgcgttg gatttagttt gtttgggtta gaatgttagt tttaaatttt 900
atttttaaat ttgttttttt gtaaaatgtg ggtaggagta gtggttattt ttttgaaggt 960
gttatgagaa ttaagtggat tagtatagat aagtgtttag aattgggttc ggtatatagt 1020
agtgttttat aagtattggt tatagttttt ttttttgttt tttttttttt aaaaaaattt 1080
tgttttgaaa tagggtttta tttggttgtt taggttggag tgtagtggtg taattacggt 1140
ttattgtagt tttgattttt taggtttaag taattttttt tttttagtcg ttcgagtagt 1200
tgaggttacg ggtgtttatt attatgttta gttaattttt tttttttttt tttttgtaga 1260
gatggggttt tattgtattg tttaggtcgt attatttttt ttttgatgtt tttggttaaa 1320
ggtaggtata gattaaaggt ggggtaagtg aggttatggt tttagtattt atttttgtta 1380
tttttttgtt tttagtattt agttttagtt aagttttagt aaaggtgggt ttttaagtag 1440
gttggtgggt cggaggtagg gattggtagg agaggtagtt atatataaat atatatttat 1500
tcgggtttta tttgtttgta taggagtttt gtttgttgta gtggcgttga ggttttgagg 1560
gtttttgggg gtttttatgg ggtaatttta tttttattag ggttgattat gttattataa 1620

atatcgcgtt ttggtgtcgt tttataagat ttattgaaga attttttttt ttagtattta 1680
gttatttaga gcgggaaaga ttttgagtcg gataggaaaa gagggaaggg tgtgttatta 1740
aatgggtgat taaaagttag ggaaaagtta ggtggaatat tttttgttta ttatttgata 1800
tattattttt ttttataggt ttttgtttac gttatatgtt gtagatgagt aatattttgt 1860
tttatgtttt tagtttttaa tttgtgtata tattgttggg tatacgtttt atagttatta 1920
gggtttgttt gtatttttgt cgtgtgtgtg tttggattta attttttgag gttatatatg 1980
gaaatttata tttgttatta gaaggattaa ttaatattat atatagagat ttttacgtaa 2040
tatagaattc gtaatattgt tatttattta ttagtttatt tatttagcgg aggtttgttg 2100
aatatttagt atgtattagg tattgtttgt tttttttttt tgtttttggt tttcgttttg 2160
ttatttaggt tggagtgtag tgtcgcggtt tcggtttatt gtaattttta ttttttgggt 2220
ttaagcgatt tttttgtttt taattcgagt agttgggatt acgggtatgc gttattacgt 2280
tcggttaatt tttgtatttt tagtagagac ggggttttat tatgatgata agtttggttt 2340
taaatttttg attttaagtg atttatttgt ttcggttttt taaagtgttg ggattatagg 2400
cgtgagttat cgcgtttggt ttaggtattg tttgtttttt ataaatattt ttgttttttt 2460
gtagtgtgtt ttttagtgat atatttgata gatggagttt gtaagtggta tgtaatatat 2520
atgtataagt agtggttttt atataattta tatggagttt tatatacgtt agtgttttta 2580
gtttagaggt tttaatataa tatgtaaaat atttatttaa attacgtatt atatatatgt 2640
ttatatttat ttatatattt tatgtgtttg tttatagagt tttgggtata atatatgttt 2700
tgttttttac ggatgtggtt ttttgttgtt gaatcgtatg taatttagta atagttgaga 2760
ttttatgcgt gttttttgta gttttggttt ttgtggttta tatgtatgat tttatttgat 2820
ttttatagta atttttttaa ataggtttta tattttttta ggagtaaatt gaggtttaga 2880
aaagtgaaat aatttgttta gaaatttatg ggttgatttt atatttattg tgaagtatgg 2940
tatatttata aatatatgta tatataaatt tatatttata tgtatttata tataaatttt 3000
ttatatttat atgtatattt atatatatat ttagatatat ttatataatt tataaaaata 3060
tatatagata tatatttata tttaaatttt tttatgttta tgtatatata tttatatata 3120
tatatattaa tatttttttt tttttttttt tgagatagag ttttgttggt ttaagttgga 3180
gtgtagtgag tggcgtaatt tcggtttatt gtaatttttg tttttggggt ttaagtgatt 3240
tttttgtttt agttttttta gtagatggga ttataggtga gtggtattac gtttagttaa 3300
tttttttgta tttttaggag agacggggtt ttattatgtt ggttaggtat gtttggagtt 3360
tttgatttta aatgatttgt ttgtttcggt tttttaaagt gttggaatta taggtttgag 3420
ttattttgtt tagttatata taatatttat gtttatatat atatttatat atagttatat 3480
atatattaat atttttatgt ttatatatac gaatgtatat ttaataaaat tttatatttt 3540
tatatatatg tatttatttt tataggtatt tgtatattta taggttttta gaggattaag 3600
gttttttttt tatttttttg gttaaaggtt ttaattattt ttaacgtttg taattaattt 3660
tttttttttt tttgtaggag agatgtttgg attcgttttt taagagtagg tattaaggcg 3720
agaggttaag atgtttatat tcggtagaga agatttacgt atttgggtag taggtttcgg 3780
gtattaaagc gaggatgggg ttagagagga agaggtaggt aaagggagtt tggggttttt 3840
taggaaatta ggtttgttat agttgttttt tttggattgg ttaaatggtg attggttaat 3900
aggtagtcgt gggtggttgg gtaggcgggt tagagtaaat ttttcgtttg ttttatttat 3960
attagtcgtt tttgttagga agggagatga gtttatgttt gaggaagttt tgtgaagagg 4020
ttttattaag gggtaggtgg taggtaggtg ttttttaaat tgttaaataa aatttattaa 4080
tttatgttag ggtcgggggt taggggtgtt ttttgttttt ttttttaaaa agaagtagtg 4140
gtttaagtag agattattag gatttaaatt ttgtttttat tattcgtgag ttgtgtattt 4200
tttgggtatg ttgtttttgt ttttggagtt ttagtttttt tttttgtaat atggggatga 4260
tgatttttgt tttttagatg agaattatta ataaataaga tagtgtatgt ttgatttatt 4320
tttagaggaa atgaatttat ttttagatta tagtaggttt ataggttttt gaatagagat 4380
attttttttg attattagta gtttgatata ttttttaagg gttttaggaa ggagggtatt 4440
taggtttatt tttgggttat ttttttaggt ttgttgttta ggaggtaatt gtttattgga 4500
tttttaattt attttagttt agatttttag gttagcgttg agtttagtcg tattgagttt 4560
agttttagag gtgaagggta gaatgggtat ggtttagcgt aaaattatta aacgttttta 4620
tcgaggatat taggtttaga gatttttggg ggttaattcg taggggcgtt ggcgttttga 4680
gacgtttaaa ttttggaagg tttgcgggga gttcgcgagt ttagagggtt ttaaggtttt 4740
tttttaaatt ttttcggatt tttttttttt ttttttcggg tgttagttcg gtttttttaa 4800
agatattttt gagggcgggc gttggtgacg ttacgtaggg tatgaatgaa taggagtcgg 4860
tttttattta atttttatta aggattcggg gtaggagggg tagaagttgc gcgtaggtcg 4920
gcgggcggga gcggatatcg aggtcggcgt gtaggcgtgc gggtgtgcgg gagtcgggtt 4980
cggggggatc ggatcgagag cgagaagcgc ggtatggagt tttaggtagt tcgcgtttgt 5040
ttcgttttgt tgtggggttg tgcgttggtc gcggtcgcgg cggcgtaggg taaggaaggt 5100
gagtgcgcgc ggcgtttgga tttttggttt tcgggtttta gtgtttttta gagttcggcg 5160
ttttttgacg cgcgtaattt tggaatttat ttggtgtcgt ttagtttcgg cgaattggag 5220
ttcggagggc gtacgggttg cgatcgaggc gagttttggt ttttattcgg ggttataggg 5280
gtttcggaga ataagcggtt ttagtttggt cgtacgggtt tcggaggttg cggagaagag 5340
gacgcggcgt aggttacgcg gttgtagttt aggcggcgat ttcgggaacg cgtttcggcg 5400
gggggcgtgg ggagtttttt agagaagggg gtgtttgggt agtttttcgc gggtttagtt 5460
ttttagtgat ttgaatttat taattcgttt gtcgaggttt tgcggagaag gtatattgtg 5520
taggtttcgt atttggggga gggggttcgg gcgggcggga gttttttgtc gaagtttttg 5580

ttttttgaga ttttttttac gttttttttt atttttagtt tcgggggaga gcgtggagat 5640
aggttcgggt taatttttgt ttatatattt tttttttttt tttttaagtc gtattttcgg 5700
gttttcgggg taggttaggg gtcgcgagtg cggacggggt ttttaggtcg agtcgcgtcg 5760
agtgaggggt cgtagatttt gtttatggcg tagcgggtgc gtggtggggg tgttgttttt 5820
tcgttagggt tagggcgagg atgggggttt ttgttttggt cgaagatttt tttgtttatt 5880
tagtttaggt tagttttaat ttttaatagt gagggtaagg tttttttttt tgtttgggta 5940
gagcgaggtt ttcgtaggtt ttataagggg gaagtcggga ggaaatttgg gtcggtgaga 6000
agtttttttt tttttttttt tttttttttt ttttgagacg gagtttcgta tttgttgttt 6060
agattggagt gtaatggcgt aatttcggtt tattgtaatt tttgtttttc gggtttaagt 6120
tatttttttg ttttaagttt ttttagtagt tgggattata ggtgttcgtt attaggcgcg 6180
tttaattttt gtatttttta gtggagacga ggttttatta tgttggttag gttgattttg 6240
aatttttgat tttaggtgat ttattcgtcg cggtttttta aagtgttgcg attataggta 6300
tgagttatta ttttcggttt gttttttttt ttttttgttt tttaaattgg gtttggaggt 6360
tttgttgttt tgtgttggga ggaaaagagg tcggatttgg agtcgttggg tagtggtagg 6420
tgttgggggt ggtattttgt tttagttttt ggagtgatag ggttcgggtt ggggttttaa 6480
tcgggatttt tggttgtagg agtttatggt aaggattttt gttattttta atattcgttt 6540
tattaacgtt tggttttttt tttttttggg ggattgtggt tggagagaag gggagagata 6600
gttggaatta ttataggttt tttttttcgg ttgtttatgg gggggtgggg gattaggcgg 6660
gtgttttgtt gttgtagttt ttaagttggt ttggaagttt ggagttgttt tgtttttgtt 6720
ttttgtgttt attttttttt agtttgtgtg attaaatttt atttttatat tttatgggat 6780
gtagggggaa aggtttgtgt attgtattgg ggtgggggta tatttttgtt tcgattttga 6840
gttttcgtga aagagttttt agagaagtgt gtttaggtgg gggtgggtat gatggtttag 6900
ttggtagttg ttggttttag tttttgggaa ttaggttgta tgtggtgggt ttgtttgggg 6960
gtggtttagg gggtaggtag gaggatatag gtgttttttt tatttatttt taggttgttt 7020
tagatttttt ttttgttttt ttatttttag gttatatttg gttgtttacg tggtttttgt 7080
taggttatta atagttattg aatttgtttt tatttttata aatggggaaa ttgagggagg 7140
ttcgagtttt tgttaattag ggttttttgg atagaggatt agatttgagt atttcgtttt 7200
ttggttgtta gagttaaggt tttgtttgtt ttgtggttgg tgttatggta gatttttttg 7260
ggaagtaggt atagttgggt aggggtttgg gcgggtggta gggggtgttg agttggggtg 7320
ttgttgatgg gaagagggtt ggagagtgtt aggaagtttt tggtttttgg ttttatcggg 7380
gttaagtttg ttttttgttt tttttttttt tttttttatg ttttttttag agtttttatt 7440
tttttgtttt ttttagggat tagttagttt tggttagaga ggaatggggg aagcggggag 7500
ggaattatag tttcggaggt tgcggagaag gatcggagat cgggagtggg tggtagtttt 7560
gtatttgtgg aagaagttgt ggagtaatgt ggatttataa agatggtatt tacggtggaa 7620
tttatggtaa agaaggaggt gttttttgtt tttttttttt ttttttagaa atgggaaggt 7680
aaggggatag agagtttatg gataagtttt gtttagttta agttttttta ggaagtcgtt 7740
tgggttagtc gaggggtttt taggttttta ttttttttag agaagcggga gtttttgttt 7800
ttggaagaat ttggggaaag agtcgcgttc gcgttagggc ggagggattt agcgttgggc 7860
gttttttgtt tttggtggtt ggttcgaggg aatttttttg ggaggggttt cggggttgga 7920
aatttttgta ggggggggtt tttttttgtg tgagggtttt tttttttttt cgattgagtt 7980
ttgtgttatt tttagagtta ggtttaggtt ggaaagggag ggttttttat gttaggttaa 8040
ttttttagaa gtttggtagc ggtgagtttt tttatttggg tatagatttt agttttttgt 8100
taatttaagg gggggttgta ggattgtgag gaatttttgt tttttttattt atttagttga 8160
aattaggagt ttttagattt ttttttgtgt agttggaata ttttatttgt tagtggggtt 8220
agtgagtttt aaagtttcgg ttttgggtat gtttgaataa atttttgttt ttgtttattt 8280
cgttgttttt agttattttt ttttattttt atagtttttg ttatggggtc ggtgttatat 8340
tggatttttt tttgtttgtt tgggttgttt gatttttttt agagaggttt ggtttcggtt 8400
agtagtgggg gtggtagtat tttacgtttt agtttggggg tttttagttt aggtaggagg 8460
gttatgttat tttaggaggc ggtttttaag attattttta gagtttagtt tttattttta 8520
taaattaggt tatttttgtt tttagtttgt ttggagtttt gtttattttt tgagtttttt 8580
ttcggtttg gttttgagga atggggtttt tgaggtagat tttttatgtt ttaggtttta 8640
aaggaagtat tgatttggtt tttttatttt tattttaggg ttttattttt tgaatttatt 8700
ttgtataggt tttatgtttc ggtttttttt atttttttat tttttaggga gggtagtatt 8760
tattggtagt tatttggatt ttatttggaa atagagtggg gatagtattt agggttttag 8820
gttttgttta tggtttttga gtttggtaga gaatttgggg tggattagtg ggaggtgttt 8880
aatggtagga ttaggtttag ggaagatttc gggagtgtat tttttcgtta ggttttagtt 8940
gtttttttgg gaataggatg gttttgatat ttggtttttg ggtttagaat gttttttata 9000
agggttttta tttttgattt tttaggaatt tttggtattt ttggtagggt agtggttagg 9060
atgtgagggg taggggtggt gggagtgatt tttttaggga ggggatttta ttagttgtgt 9120
gtatttttgg gtttttttta ggggttttat gggtttaggg ttttattttg tttgttttgt 9180
ggtttttgtg gtgagaagat cggtttttag atattttttt ttttttttta ttttggagtt 9240
gtttttattt gtttatagta tttttttttt ggagtatcgt aagatgtacg tgtatatata 9300
ggtgtatatg tgtgtgttat atgatttgag tggggttggt gtgttttttg ttggggggaa 9360
tgtggtagaa gtagatatta gtgtttttaa ggtttatatg tacgtgaagt gttgttaaag 9420
ggttttgttt aaggaagtag agggggcggt gtggtgttat ttaatatttt gtttagttta 9480
gttttttgtt tagttgttgt ttttttgtcg ggttgagttt tttggtttgt tttgtttagt 9540

tggggattaa cggaggggga taggatggtt ggaattttgg tttgggagat tggaggttta 9600
gttttgattt tagtttgttg tgtgttttta gttgggtttt attttttttt tgggtttgtt 9660
ttttttatga gtcgagggat gatttttttg gatatggtga ttttgtgtgg tggggttttt 9720
ttcgttttgt ttgagagttg gggtttttgg agggattttt attttttttg ttatggtttg 9780
atttttgaag atatttttag gtttattttt tttttttttt tttttttagt 9830

<210> 80
<211> 9830
<212> DNA
<213> Artificial Sequence

<220>
<223> chemically treated genomic DNA (Homo sapiens)

<400> 80

attgaaaggg agaagggaga gggggtgagt ttgggggtgt ttttaggagt tagattatgg 60
taggaaaggt gaggattttt ttaggaattt taatttttag atagagcgag agggatttta 120
ttatataggg ttattatatt taagaaaatt atttttcgat ttatgaggga aataggttta 180
gaaaaaggat ggaatttagt tgaaggtata tagtaggttg gggttaggat tgggttttta 240
gttttttagg ttaggatttt agttattttg ttttttttcg ttagttttta gttgggtaga 300
gtaggttagg gagtttagtt cggtaggaga atagtagttg ggtagagagt tgggttgagt 360
agggtattaa gtgatattat atcgtttttt ttgttttttt gaataaggtt ttttggtagt 420
attttacgtg tatgtgggtt ttagggatat tggtgtttgt ttttgttata ttttttttag 480
taggaaatat attaatttta tttaggttat gtagtatata tatatgtatt tatatgtata 540
cgtgtatttt gcggtatttt aagaagagag tgttgtgaat aggtgggaat agttttaggg 600
tggaggaggg aagagggtat ttggaaatcg atttttttat tataaaagtt atagagtagg 660
taggataaag ttttggattt atagggtttt taagaggggt ttaaagatat atatagttgg 720
tgaggttttt tttttgggaa agttattttt attatttttg ttttttatat tttgattatt 780
gttttgttag gggtgttaga ggtttttggg ggattaggaa taaaagtttt tgtaggggat 840
attttaggtt tagagattag gtgttaggat tattttgttt ttagagaggt aattggagtt 900
tggcggaggg atatattttc ggggtttttt ttagatttag ttttgttatt gggtattttt 960
tattgattta ttttaggttt tttgttaggt ttagaggtta tagataaaat ttaagatttt 1020
aggtattgtt tttattttat ttttaagtaa agtttaggta gttgttaata agtgttattt 1080
tttttagagg gtaaggagat aggagaaatc ggggtataga atttatgtaa gatggattta 1140
gagggtaaag ttttaaggtg ggggtaaaga aattaagtta atgttttttt tgggttttgg 1200
agtatgaggg atttgtttta gaagttttat tttttaaggt taggatcgga gaaggattta 1260
aagggtggat agagttttag gtaggttggg agtagggatg gtttggtttg tggagatggg 1320
agttgggttt tgagggtggt tttgaagatc gttttttgaa gtgatatggt ttttttattt 1380
gggttgaagg tttttaggtt ggggcgtgaa atgttgttat ttttattatt gatcggggtt 1440
aaattttttt aggaagggtt aggtagttta gataggtaag aggaaattta atgtgatatc 1500
ggttttatga tagaggttgt ggggataggg gagggtggtt gggggtagcg gggtgggtag 1560
gggtagggat ttgtttagat atgtttaggg tcggggtttt gaaatttatt aattttatta 1620
gtaaatggaa tgttttagtt atataggaag gaatttggag gtttttgatt ttagttgggt 1680
gggtagaagg ataaaggttt tttatagttt tgtagttttt ttttagattg ataggaggtt 1740
gaggtttgtg tttaagtgag agggtttatc gttattaggt ttttgaaggg ttaatttggt 1800
atggagggtt tttttttttt agtttaggtt tggttttaga agtagtatag ggtttaatcg 1860
gagaagaaaa gaagttttta tataggaagg agtttttttt tgtaggggtt tttaatttcg 1920
gagttttttt tagggaaatt ttttcgaatt aattattagg aataggaggc gtttagcgtt 1980
gggttttttc gttttgacgc gggcgcggtt ttttttttag gtttttttag ggatagaggt 2040
tttcgttttt ttggaaaggg tgggagtttg ggagtttttc ggttgattta gacggttttt 2100
tggagaggtt taggttaagt agggtttatt tatgagtttt ttattttttt atttttttat 2160
ttttgggggg aaaaaaaaaa agtaaaaaat attttttttt ttgttatgag ttttatcgta 2220
agtgttattt ttgtgggttt atattgtttt atagtttttt ttataaatgt aggattgtta 2280
tttattttcg gttttcggtt ttttttcgta gttttcgagg ttgtggtttt tttttcgttt 2340
tttttatttt tttttaatta agattagtta atttttaaag gaagtaggga ggtggggatt 2400
ttggggaaaa tatgaaagaa aagggaaagg ggatagaggg tagatttggt ttcggtagaa 2460
ttagaaatta aggatttttt ggtatttttt agtttttttt ttattagtag tattttagtt 2520
tagtattttt tgttattcgt ttaggttttt gtttagttat gtttgttttt taggaaaatt 2580
tattatgata ttaattataa agtaggtagg gttttggttt tggtagttaa gaagcgggat 2640
gtttaggttt ggttttttgt ttagaaggtt ttggttggta agggttcggg ttttttttag 2700
tttttttatt tgtaaaaatg agggtagatt tagtggttgt tgatgattta ataggagtta 2760
cgtgggtaat taagtatagt ttaggggtga gggggtaaga aagaagtttg aagtagtttg 2820
ggggtaggtg aaaaaggtat ttgtgttttt ttgtttgttt tttaagttat ttttaggtag 2880
atttattata tatagtttga tttttaggaa ttgaaattag tagttgttag ttaggttatt 2940
atgtttattt ttatttaggt atattttttt agggattttt ttacggggat ttagagtcga 3000

agtaaaaatg tatttttatt ttagtgtaat atataagttt ttttttttgt attttatggg 3060
gtgtgggggt ggggtttaat tatataggtt ggggagaggt ggatataggg gataaggata 3120
aggtagtttt aggtttttag gttagtttgg ggattgtagt agtaaagtat tcgtttaatt 3180
ttttattttt ttatgggtag tcgaggagaa aggtttatag tgattttagt tgtttttttt 3240
ttttttttta attatagttt tttaggaagg gggaggatta aacgttggtg gggcggatat 3300
tgagggtggt agaaattttt gttatgagtt tttgtaatta gaagtttcgg ttgagatttt 3360
aattcgggtt ttgttatttt agagattgag ataggatgtt atttttaata tttgttattg 3420
tttaacggtt ttagattcgg tttttttttt ttttaatata gaatagtaaa atttttaaat 3480
ttagtttggg aggtagaaaa agaaggaaat aggtcggggg tggtggttta tgtttgtaat 3540
cgtagtattt tgggaggtcg cggcgggtgg attatttgag gttaggagtt tagaattagt 3600
ttggttaata tggtgaaatt tcgtttttat taaaagatgt aaaaattaga cgcgtttggt 3660
ggcgggtatt tgtaatttta gttattaggg aggtttgaga taggagaata gtttgaattc 3720
gggaggtaga ggttgtagtg agtcgagatt gcgttattgt attttagttt gggtaataag 3780
tgcgaaattt cgttttaaaa aaaaaaaaaa aaaaaaaaag gaaggaaatt ttttatcggt 3840
ttaagttttt tttcggtttt ttttttgtgg agtttgcgga aatttcgttt tgtttaggta 3900
ggaggggaag ttttgttttt attgttggaa attgaagttg gtttgggttg ggtggatagg 3960
aaggttttcg gttagggtag gagtttttat tttcgttttg attttagcgg agggataata 4020
tttttattac gtattcgttg cgttatgagt aaagtttgcg gttttttatt cggcgcggtt 4080
cgatttggga atttcgttcg tattcgcggt ttttaatttg tttcgggagt tcgggagtgc 4140
ggtttgggaa ggagggaaag gagtgtgtga gtagggattg gttcgaattt atttttacgt 4200
tttttttcgg gattggaggt gaaggggggc gtggagaaaa ttttagggaa taggggtttc 4260
gatagggggt tttcgttcgt tcgagttttt tttttaaaat gcgaggtttg tatagtatgt 4320
ttttttcgta aaatttcggt aagcgggtta gtgaatttag gttattaaga agttgagttc 4380
gcggaaagtt gtttaaatat tttttttttt aggggatttt ttacgttttt cgtcgggacg 4440
cgttttcgaa gtcgtcgttt ggattgtaat cgcgtggttt gcgtcgcgtt ttttttttcg 4500
tagttttcga ggttcgtgcg attaagttga aatcgtttat ttttcggagt ttttatgatt 4560
tcgggtgggg gttagggttc gtttcgatcg tagttcgtgc gtttttcgga ttttagttcg 4620
tcgggattgg gcgatattag gtaggtttta aagttgcgcg cgttaaggag cgtcgggttt 4680
tagggggtat tggggttcgg gggttagggg tttagacgtc gcgcgtattt attttttttg 4740
ttttgcgtcg tcgcggtcgc ggttagcgta tagttttata gtagggcgaa gtaggcgcgg 4800
gttgtttgga gttttatgtc gcgtttttcg ttttcggttc gattttttcg agttcggttt 4860
tcgtatattc gtacgtttgt acgtcggttt cggtgttcgt tttcgttcgt cggtttgcgc 4920
gtaatttttg ttttttttgt ttcgagtttt taatggaagt tgggtgagaa tcgatttttg 4980
tttatttatg ttttgcgtga cgttattaac gttcgttttt aggaatgttt ttaaaggggt 5040
cgggttggta ttcggaggag agggagggga agttcggagg ggtttggagg aggatttggg 5100
ggttttttgg attcgcgggt ttttcgtagg ttttttaaag tttgagcgtt ttaaagcgtt 5160
agcgttttta cggattagtt tttagggatt tttgagtttg gtattttcgg tggaaacgtt 5220
taatggtttt gcgttgagtt atatttattt tgttttttat ttttgagatt gagtttaatg 5280
cgattgagtt taacgttggt ttgaaggttt gagttgaaat ggattggggg tttaataagt 5340
agttgttttt taagtagtag gtttgagaga gtggtttaga aatagatttg gatgtttttt 5400
tttttaaaat ttttgaagag tatattaagt tgttgatggt taaaaagagt atttttgttt 5460
agaagtttat aagtttatta tgatttgaga ataggtttat tttttttggg aatggattag 5520
atatgtatta ttttatttat taataatttt tatttgggag gtaggggtta ttatttttat 5580
gttatagaag aggagattga ggttttaaga gtagaaataa tatgtttaaa gggtgtatag 5640
tttacggtta gtagaagtag gatttgaatt ttggtggttt ttgtttgaat tattgttttt 5700
ttttggggaa gggggtaagg ggtatttttg gttttcgatt ttggtatggg ttgataggtt 5760
ttgtttgata gtttggagga tatttgttta ttatttgttt tttgataagg tttttttata 5820
aaattttttt agatatgagt ttattttttt ttttgataga agcggttagt gtgagtgaaa 5880
taaacgggaa atttgttttg gttcgtttgt ttagttattt acggttgttt gttggttaat 5940
tattatttga ttagtttagg aagagtaatt gtggtaggtt tgattttttg ggaggtttta 6000
gatttttttt gtttattttt ttttttttga ttttattttc gttttgatgt tcgggatttg 6060
ttatttaggt acgtgggttt tttttgtcgg atgtggatat tttggttttt cgttttggtg 6120
tttatttttg ggaagcgggt ttagatattt tttttgtagg aagagaggaa gggttaatta 6180
taggcgttgg ggataattaa agtttttgat taagaggatg ggaggagagt tttggttttt 6240
tgagagtttg tgagtgtgta agtgtttgtg agaataagtg tatgtgtatg agggtgtgag 6300
attttgttga atgtgtattc gtgtgtatag atatgagagt gttgatgtat gtatgattgt 6360
gtgtgagtgt gtgtatggat ataagtgttg tatgtgattg ggtagggtgg tttaggtttg 6420
taattttagt attttgggag gtcgaggtag gtagattatt tgaggttagg agttttagat 6480
atgtttggtt aatatggtga aatttcgttt tttttaaaaa tataaaaaaa ttagttgggc 6540
gtggtgttat ttatttgtaa ttttatttat taaggaggtt aaggtaggag aattatttga 6600
attttagagg tagagattgt agtgagtcga gattgcgtta tttattgtat tttagtttgg 6660
gttagtaaga ttttgtttta aaaaaaaaaa gaaaaaagtg ttggtatatg tgtgtataag 6720
tgtgtgtgta tgggtatgag aggatttggg tatgagtgtg tgtttgtgtg tgtttttgtg 6780
gattgtgtgg atgtgtttag atgtgtgtat gagtgtgtat gtgggtgtga gagatttgtg 6840
tatgagtgta tgtgagtgtg gatttgtgta tgtatgtatt tgtgagtgtg ttatatttta 6900
tagtagatgt ggagttagtt tatgagtttt tgaataagtt attttatttt tttgagtttt 6960

agtttgtttt tggaaagatg taaagtttat ttaggagagt tattgtaaag attaaataaa 7020
gttatgtatg taaattatag gggttaggat tatagaaagt acgtatgaaa ttttagttgt 7080
tattgggtta tatgcggttt agtaatagga agttatattc gtggagagta gggtatgtgt 7140
tgtgtttagg gttttgtgga taggtatatg gggtgtgtgg atgagtgtga gtatgtgtgt 7200
agtacgtggt ttggataagt gttttgtatg ttgtattgga gtttttgggt tagggatatt 7260
ggcgtgtgta agattttatg tgagttgtgt ggaagttatt gtttgtatat gtgtgttata 7320
tgttatttgt aggttttatt tgttagatgt gttattagag gatatattgt aggaggatag 7380
ggatgtttgt agaaaataga tagtgtttgg gttaggcgcg gtggtttacg tttgtaattt 7440
tagtattttg ggaggtcgag gtagatagat tatttgaggt taggagtttg agattaagtt 7500
tgttattatg gtgaaatttc gtttttatta aaaatataaa aattagtcgg gcgtggtggc 7560
gtatgttcgt aattttagtt gttcgagttg gaggtaggaa agtcgtttga atttaggagg 7620
tggaggttgt agtgagtcga gatcgcggta ttgtatttta gtttgggtga tagagcgaga 7680
gttaaaaata aaaaaagaaa atagatagtg tttgatatat attagatatt tagtaagttt 7740
tcgttgaatg aatgaattaa tgagtgaatg atagtgttgc gagttttgtg ttgcgtgaaa 7800
gtttttgtgt gtagtgttgg ttggtttttt tggtggtaag tgtgggtttt tatgtgtggt 7860
tttaaaaggt tgaatttaag tatatatacg gtaggggtgt aggtaggttt tgatggttgt 7920
ggggcgtgtg tttagtagta tgtgtataag ttgggggttg agagtatagg gtagggtgtt 7980
gtttatttgt agtatgtgac gtgaatagag gtttatggaa aggagtggta tgttaggtgg 8040
tggtaagagg gtgttttatt tggttttttt ttggttttta gttatttatt taatgatata 8100
tttttttttt tttttttgtt cgatttagag tttttttcgt tttaggtgat tgggtgttaa 8160
ggaggaaagt tttttagtga gttttataaa gcggtattaa aacgcggtat ttatagtgat 8220
ataattagtt ttaatggaga tggaattatt ttataaaggt tttaaggagt ttttagggtt 8280
ttagcgttat tgtagtagat aaggtttttg tataggtagg tggagttcga gtgagtgtgt 8340
gtttgtgtgt gattgttttt tttgttaatt tttgttttcg gtttattagt ttgtttaggg 8400
gtttattttt gttaggattt agttggggtt gggtattggg ggtaaggaga tgataaaggt 8460
gaatgttgaa gttatggttt tatttatttt atttttgatt tgtgtttgtt tttggttaag 8520
agtattagaa aaaaaataat gcggtttggg taatatagtg agattttatt tttataaaag 8580
aaaaaagaaa aaaaattagt tgggtatggt ggtgagtatt cgtggtttta gttattcggg 8640
cggttgagga aggaggattg tttgagttta ggaggttaag gttgtaatga gtcgtgattg 8700
tattattgta ttttagttta ggtaattaag tgagattttg ttttaaaata aaattttttt 8760
aaaaaaagaa aagtaaagaa aaaaattgta gttaatattt atgaagtatt attatgtgtc 8820
gggtttagtt ttaggtattt atttgtattg atttatttag tttttatgat atttttaggg 8880
aggtggttat tgtttttgtt tatattttgt agaagagtaa atttggaagt aaaatttgga 8940
gttggtattt tggtttagat aggttagatt tagcgcgtat ttttatgggt atttgtttta 9000
attattgttt ttgaatttta ttgaatttgg agagtagtgt ttcgttttgg aatgtgttaa 9060
tttttcgtag ttgggatttt taaggttagt ttaattatta ttttttgggt gaaattttta 9120
gggattaggt tttttttaga gtttaggggg tttttatgtt atatgtttat tagtattttg 9180
tttttaatta attgaatttt tttttgtttt ttaaagtttt tgaattattt ttttaattag 9240
attgtaaatt ttttagaggt agggtatgaa tttagtaata ggtagaatag ttttttatat 9300
atttgtttta atagatgtta attttttttt tttttttttt tttgagatag agttttgttt 9360
tgttttttag gttggagtgt agtggcgtaa ttttagttta ttgtaatttt tatttttcgg 9420
gtttaagtga tttttttgtt ttagtttttt gagtatttgg gattataggt gtacgttatt 9480
atatttggtt aatttttgta tttttagtag agatagggtt ttattatgtt ggttaggttg 9540
gttttgaatt tttgatttta tgattcgttt ttttcggttt tttaaagtgt tgggattata 9600
ggcgtaagtt attgtgtttg gttttagatg tttatatttg atggtgatat tttaaattag 9660
ttttttttat ttattttttt tttttttaga cggagttttg ttttgttatc gaggttggaa 9720
tgtagtggcg taatttcggt ttattgtaat ttttattttt cgggtttaag ggattttttt 9780
gttttagttt ttttagttgt tgggattata ggtatatatt attatgtata 9830

<210> 81
<211> 5975
<212> DNA
<213> Artificial Sequence

<220>
<223> chemically treated genomic DNA (Homo sapiens)

<400> 81

gggtttcgtg gcggatacgt atggaggggt gttgtggggg ataggttggg agggtcggtt 60
tattttggat agggtttatt tggttaggtt gggttttgat tttagagggt ttttcggttt 120
ttgggggttt gaattcgatt ttggaggtat gtgggttacg ttagtagagt tgtgtaaaat 180
aggatttttt ttaggaggag tttgtgtagt tatcgtgagg gggtgtttgt agagtaggta 240
tagttgaagg gtatagggta gagagtagta ggtggggagt gggttgtatt gtataggtag 300
gtttcggggg taaaagtaga tattggttat tattataatt tgtttttttg ttttggtttt 360
tatgtgggta aagttttttt ataaattttt ttagataggg tattttatgg taggtttttt 420

ttaaaaagtt tatagtaaaa aatttaaggt tggtttgggt atagggtttt tcgtttgtaa 480
tcgtcgttat tcgggaggtt aaggtagtag aatatgagtt taggagtata aggtttattt 540
ggttaaatcg ggtaattttt tatttttatt aaaagtataa aaattattgg tcgtggtggt 600
atgcgtttgt agttttagtt atttgggagg ttgaggtagg agaattattt gaattcgaga 660
gacggaggtt gtagtgagtt gagattgtat tattgtattg tagtttggtt atagagtaag 720
attttgtttt aaaaaaaaaa aaaaagatat aaaaattaat tggtatggta gtatgtgttt 780
gtaattttag ttattgggga ggttgaggta ggaaaattat ttgaacgcgg gaggtagagg 840
ttgtagtgag tggagattgt gtaatcgtat tttagtttgg gcgatagagt gagattttat 900
tttaaataaa taaaaaaata gaaagttata tggttagtaa agtttaaaat gtttgttgtt 960
tagtttttta tagtaaaagt tggttaattt ttggtttagt atatttattt ggtgttatta 1020
gttttattat tatcgttatt tttattagtt gttttttttg tttggaaagt ttttttatcg 1080
gattttagga tagtatgttt ttttgtggga ttcggattat attttaagtg taattttttt 1140
agagattttt tgtggttatt ttgttatttt tttattttat tattttgttt taatttttga 1200
tatgttagtt attgtttgtt tttttgttgt attttaagaa tttggttagt ttttgattta 1260
taggtagtat ttagtaagtg tagtgagttg aataagtgaa tagttgttga gtttttggta 1320
taggggtttt agtataggat gtgatatgga aggttttgag tttttggttg agtaagtttg 1380
atgaaaaggt ttttttttag gtgtgcgtgt attagtgtta ggagagtttc ggtatgaaag 1440
aagggtttgg aattgttatt tagtttttat tttggttaat ggatagatgt ggtataagtg 1500
ttagggttag gatacgttcg gggaagagta agggtggagt tgggggtgtt ggtagtttgt 1560
ggggttattt gagattgagg tttttaagtt gtatgtagtg gagagttgta ttaatcggtt 1620
gtttatttag attagaggtt taagaatttt tagaatatag attttaaagt atagttatat 1680
aggaggagtt tttttgttag gtgttagttt aggtaatttt cggggtaaag cggagtttaa 1740
gtgtatttta agtaggtgag ggatgaggtt aggataggtt aattagagat acgagggatg 1800
gttttagtta atagggagga aatttagttc gagtattagg agggggaagg attttttaaa 1860
gttattttta ttttaatttt agttcggggg atttggggta ggggtaggta gttggttttg 1920
tagagttttg agcgtttatt atagtgatag gtatggagtt agtatgtgtg taagtttatt 1980
aggtagtttt tagtttttta ggtaatttag tcgtacgtcg tcggagtttt tttcggcgag 2040
tcgtggtttt gggtttttga attaggtagc gtaattagag gtttttttag gaggtttagg 2100
gtattgtagg taagattttt tttttagaga aattttaata cgtttaggaa gtaattgggg 2160
cgttaagacg tttaggaggg tttattgttg gattagatat attttgagaa aggtagcggt 2220
attttcgttt ttatttttat ttttaggtag gaatttagga agaaaaggaa ggtatagagg 2280
tttggggaag gttaattttg ttttgttttt tttaagttta gaatgatttc gtatacggtt 2340
tttttttttg tatttatgga aattaatttt atcgtgtttc gggttttcgt tttggtatag 2400
ataggggttt atattagttt tggacgtttt tttttaggcg gggtgtttgt ttagttagta 2460
atttttgttt tgattttaat ttttgagggg tggttcggac gtttcgtatt ataagtgagg 2520
ttatttgaat ttttgaaagt tgaaggaatt tgttttagtg agattttatt ttttttttta 2580
gtttagtttt aaggtttata taaagtttaa gtttgggtaa gttttttttt tgcggtttcg 2640
atttttttat ttgaaaatga agataatttc ggagttgttt atttgatcga cgggttgcgg 2700
agaatttata tatttttgtt ttggaataaa acgcgaagcg tttatcggta gttttcggtt 2760
ttttgttttt tttgatcgtt ggtatttgtt gttttgggtt ttagttagta acgtttaagg 2820
cggttcggcg ggatttgtgt tttgggcgtt cgcgtgtcgg tagtcgcgtt tttcgagggt 2880
tttttttcgg tttttttcgt cgttagtttt tttttttttt tcggtttttt ttttttttcg 2940
tttttttcgg tttttttttt tttttttgtt ttattttttt tcggttcggt tttttttttc 3000
ggtgtcgatt tagtgttttt tttttcggg ttcgttattg ttgttttgg tcggggtttt 3060
tttttttcgt ttcgttttcg tcgtaggatt cgggagttcg cgttttattt cgcgtttcgc 3120
gtcgatcgtc gtataagtgg gttcgttggt tttcgcgggt tagtgggttt cgcgtttttt 3180
gaggcgttgc gggggcgggt ttcgcgttcg gttttcgtta tggtaacgtt atattttcgg 3240
gtcgcggtat ttatagtcgg aagtcgcggt gtttcgtggg agtcgggggc ggagttcggg 3300
aggaggaaag gtcgggggcg gggtttgggg agggacggtc gggggtgggg gttaaacgcg 3360
gatagttgcg ggcggtcggg cgagtttttc gcggttggga tttttttggg gcgtgggtgg 3420
gacgggaatg gtttaaagtg ttttaaggtt ttcgggacgg tggttattga tttatgttgg 3480
ataaaatcga gttatcgcgg gtaacgggac gggcgttagc ggaatgagtg gtagttttgg 3540
gggtggtttc gggtttagga tagtgcggtc ggagaagagc ggttagaatt tggggagttt 3600
ttagcgtttt attaaggaag cgttagtgga gaggggggtg ttttaaaatt tacgggagat 3660
cgggcgcggt gttttacgtt tgtaatttta gtattttggg aggtcgaggc gggtagatta 3720
cgaggttagg aaatcgagat tattttagtt aatttgatga aatttcgttt ttattaaaaa 3780
tataaaaaaa ttagtcgggc gtggtggtgg gcgtttgtag ttttagttat tcgggaggtt 3840
gaggtaggag aatggtatga attcgggaag cggagtttgt agtgagtcga gattacgtta 3900
ttgtatttta gtttggcgga tagagttaga tttcgtttta aaaaaaaaaa aaaattatgg 3960
gaaaggttcg gttcggtggt ttacgtttgt gattttagta ttttgggagg aggtcgaggt 4020
tagtgggtta tttgagttcg ggagattaat ttgagtaata taggtaggtt cggtttttat 4080
taaaaattaa gaaattagtt gggttttgtg gcgtacgttt gtagtttcgg gacgttgagt 4140
cgggaggatc gttttagcgg gggaggtgga ggttgtacgg ggtttagatc gtattattgt 4200
attttagttt gggtaataga gtgagatttt gttttaaaat aataataata ataaatattt 4260
gtaggaaaga acgacgtttt gttgatcgaa tggggtgaag tggttataag tattagtttt 4320
atgaggtata ggattgtgta gttataattt agggaatata gtagagaggt ggtggtagga 4380

gttttgaggg aaattacgtg gcgtttgagg ggagagttag agatagaagg aaaaggggta 4440
ttattcgatc gggggtgtgg ggggtaggag tagtgtggta ttttatatat aagatttttt 4500
gtagtttagg tttttttttt ttggatattt gttgtttgtt tttttttttt atattttagg 4560
gtgtgttgga ttttatcgta tgttgtagat ttttagtttt ttgaggttat tatggtggag 4620
gttgtataag aaaagttgaa tttttagagt gaagggtaaa agtatatttt tgggttttcg 4680
tgtgtttggg ggtttttggt ttggtgttag tattaattat cgttgttaat ttttagagtt 4740
gttatttttt tgggtaaatt tgttttttgt gagggaggag aatttgggtt ttggggtagg 4800
atatataata ttagttttgt ttgggtggag gagtagagat ggttagggag gaggaggtga 4860
gcggtttatt tgggtttttg aagtaatttt taggaaatta gttgagttaa tgtgtttttt 4920
ttttgttttt agggtgcggg ttttatggtt ttttttttta ggaagtttta tttgattatg 4980
ttttgggtgg atatttagtt tttatagttt agggtttatt agtagttgtt agattttgaa 5040
ttttaggagc gttttacgtt ttgggagttt ggtatgggtt aaatattttt ttatttgtta 5100
aatggggttt tgaaatttga ttagggaaga cgggataaag tagttatggg ttatcgtagt 5160
ttttttgaag tcgggtttgg ttatttaaag gtaatttagg ggtggagatt gaggttttag 5220
gagaagtttt tattagaatg ttttttgttt ttttttttta gattaattaa aatttgttaa 5280
ttgtggaagt tttcggtatg tttttttttt ttatagtttt tttttttttt tttttttttt 5340
ttttttttat tcgaatgata aaggttttag ttcgtttgtt ttagttcggt tttaggtttc 5400
ggttttgttt tttatattgt tttatcgttt tgtatttttt attcggttag gtttttgttt 5460
acgttgttta tcgtttcgta tggggttttg tagcggtttt cgtttggggt ttttagaggt 5520
agagtcgttt ttttagtttt ttaagaggag gaagaagagg tatttgcgat atgataagtt 5580
tttttatatt tatttggtta tgatcgtttt ggtgatttag gtcgtttttt ttcgtagatt 5640
gaagttggtt taggtgagcg ggtttcggtt tttatttttt ttttttttag attcgttttt 5700
tatttatttt taggtttaag ttttgatttt tatttttttt tagtttattt aggtatgtta 5760
gtgggattag ttttgagagt tgagggtttt aaggtacgat tttttttcgg gagttttttg 5820
ttttttttgt cgcgttttcg cgtagaaatg ttttttttcg gggatagtag gggtcggtat 5880
cgcgcgtttt tgagttcggt agtgggggag gggtagggcg ttattttttt ttcgtttggg 5940
tttacggcgt cggtcggtcg tttggcggtt ttaga 5975

<210> 82
<211> 5975
<212> DNA
<213> Artificial Sequence

<220>
<223> chemically treated genomic DNA (Homo sapiens)

<400> 82

tttgaaatcg ttaggcggtc ggtcggcgtc gtgagtttag acggggaggg ggtagcgttt 60
tatttttttt ttattatcgg gtttaggggc gcgcggtgtc ggtttttgtt gttttcgaag 120
aaggatattt ttgcgcggaa gcgcggtaga gggggtaagg ggttttcgga gaagggtcgt 180
gttttgaggt ttttagtttt taggattggt tttattggta tatttgggtg ggttaggaag 240
gggtgggggt tagggtttga atttggaagt gggtggggaa cgagtttggg gaaagagaga 300
gtgggggtcg gggttcgttt atttgggtta gttttagttt gcgggaggga gcggtttgaa 360
ttattaaggc gattatggtt aagtaggtgt aggggggttt gttatgtcgt aggtattttt 420
tttttttttt tttagggggt tgggagggcg atttgttttt tgggggtttt aggcgggagt 480
cgttgtaggg ttttatgcgg gacggtagat agcgtgggta ggggtttggt cgggtggagg 540
gtgtagggcg gtggggtagt gtagaaggta gggtcgggat ttgaggtcgg gttggggtag 600
gcgggttggg gtttttatta ttcggatgga agggggaggg gagggaaggg aggaagaggt 660
tgtgggggag gggagtatgt cgagggtttt tataattagt aggttttggt taatttggaa 720
gggaggggta gagagtattt tagtgggggt tttttttgag gttttagttt ttatttttga 780
gttgtttttg ggtggttagg ttcggtttta aaggggttgc gatgatttat ggttatttta 840
tttcgttttt tttggttagg ttttaggatt ttatttaata aatgggggag tatttagttt 900
atgttaagtt tttaaggcgt ggggcgtttt tggagtttaa gatttagtag ttgttagtag 960
gttttgaatt atgggggttg gatatttatt taggatatga ttaggtggag ttttttggag 1020
gagaaggtta tgaagttcgt attttaggaa taagaggggg atatattgat ttaattgatt 1080
ttttgggagt tgttttagga gtttaggtgg atcgtttatt tttttttttt tagttatttt 1140
tgttttttta tttaagtaag gttggtattg tgtattttgt tttaaagttt aggttttttt 1200
tttttataag gggtaagttt gtttaagagg atagtagttt tgggaattag tagcggtggt 1260
tggtgttggt attaggttaa gggtttttag atatacgggg gtttagggat atgtttttgt 1320
tttttatttt gagaatttag ttttttttgt gtagttttta ttatgataat tttaaagggt 1380
tgagaatttg tagtatacgg tggggtttag tatattttga ggtgtggagg ggagggatag 1440
gtagtaagtg tttaggagga ggagatttgg gttgtaaggg gttttgtatg tggggtatta 1500
tattgttttt gttttttata ttttcggtcg ggtggtgttt tttttttttt tatttttgat 1560
ttttttttta gacgttacgt ggtttttttt aggatttttg ttattatttt tttgttgtgt 1620
tttttaggtt atggttgtat aattttgtgt tttatgaggt taatatttgt ggttatttta 1680

ttttattcgg ttagtaaggc gtcgtttttt tttataagtg tttgttgttg ttgttgtttt 1740
gagataaggt tttattttgt tgtttaggtt ggagtgtagt ggtgcgattt gggtttcgtg 1800
tagtttttat ttttttcgtt agggcgattt tttcgattta gcgtttcggg attataggcg 1860
tgcgttatag agtttagtta attttttaat ttttggtagg gatcgggttt gtttatgttg 1920
tttaggttgg tttttcgggt ttaagtgatt tattggtttc ggtttttttt taaagtgttg 1980
ggattataga cgtgagttat cgggtcgggt tttttttata attttttttt ttttttgaga 2040
cggagtttgg ttttgtcgtt taggttggag tgtagtggcg tgatttcggt ttattgtaag 2100
tttcgttttt cgggtttatg ttattttttt gttttagttt ttcgagtagt tgggattata 2160
ggcgtttatt attacgttcg gttaattttt ttgtattttt agtggagacg gggttttatt 2220
aggttagtta ggatggtttc gattttttga tttcgtgatt tgttcgtttc ggttttttaa 2280
agtgttggga ttataggcgt gagatatcgc gttcggtttt tcgtaagttt taaggtattt 2340
ttttttttat tggcgttttt ttggtggggc gttggaggtt ttttaagttt tgatcgtttt 2400
ttttcgatcg tattgttttg ggttcgaaat tatttttaaa gttgttattt atttcgttgg 2460
cgttcgtttc gttgttcgcg atgattcggt tttgtttagt ataaattaat gattatcgtt 2520
tcgggagttt tggagtattt tgagttattt tcgttttatt tacgttttaa ggaggtttta 2580
gtcgcggagg attcgttcgg tcgttcgtag ttgttcgcgt ttggttttta ttttcgatcg 2640
ttttttttta ggtttcgttt tcgatttttt tttttttcgg gtttcgtttt cggtttttac 2700
ggggtatcgc ggttttcggt tatgggtatc gcggttcgga agtgtagcgt tgttatggcg 2760
agggtcgggc gcggggttcg ttttcgtagc gttttaggga gcgcggggtt tattgattcg 2820
cggaagttag cggatttatt tgtgcggcga tcggcgcggg gcgcgaggtg aggcgcggat 2880
tttcgggttt tgcggcggga gcggagcgag ggagaggggt ttcggttagg gatagtagtg 2940
gcgggttcgg gaaggagggg tattgggtcg atatcgagag aggaggtcgg gtcggggaag 3000
gatgaggtag aggagaggga ggaggtcggg aagggcggga ggaggaggag atcgaaggga 3060
gagaggaggt tggcgacgga ggaggtcggg gaggggtttt cgggagacgc ggttgtcggt 3120
acgcgggcgt ttagggtata ggtttcgtcg ggtcgttttg ggcgttgttg gttgaggttt 3180
agggtagtag gtgttaacgg ttaggaaagg tagggagtcg aaggttgtcg gtgggcgttt 3240
cgcgttttgt tttaaggtaa aggtgtgtgg atttttcgta gttcgtcggt taggtagata 3300
gtttcggaat tatttttatt tttagatgag gaaatcgagg tcgtagagaa gggatttatt 3360
taaatttaag ttttgtgtgg gttttggagt taggttgaag gagaaagtgg agttttattg 3420
gggtaagttt ttttaatttt tagggattta aatgatttta tttgtagtgc ggggcgttcg 3480
gattattttt taggaattgg gattagggta agaattatta attaagtaag tatttcgttt 3540
gggagggagc gtttaggatt ggtgtgggtt tttgtttgta ttagagcgga ggttcggggt 3600
acggtgggat taatttttat gggtgtagag aggggagtcg tgtacggagt tattttaggt 3660
ttaggggaga tagagtaaag ttagtttttt ttaaattttt gtgttttttt tttttttttg 3720
gatttttatt tgggggtgga ggtggaggcg ggggtatcgt tgtttttttt aaagtgtgtt 3780
tagtttagta gtgagttttt ttgggcgttt tggcgtttta gttgtttttt ggacgtattg 3840
gggttttttt gggagaaggg ttttgtttgt agtatttggg gttttttgaa gaggtttttg 3900
gttgcgttgt ttgatttagg gatttagagt tacgattcgt cgagaggagt ttcggcggcg 3960
tgcggttggg ttgtttgagg ggttgggggt tgtttggtgg atttgtatat atgttggttt 4020
tatatttgtt attgtggtgg acgtttagag ttttataggg ttagttgttt gtttttgttt 4080
taagtttttc gggttggggt tgggatgggg gtggttttag ggggtttttt ttttttttgg 4140
tgttcgagtt ggattttttt tttgttggtt ggggttattt ttcgtgtttt tgattggttt 4200
gttttggttt tattttttat ttgtttggga tgtatttgga tttcgttttg tttcgagggt 4260
tgtttgggtt ggtatttagt aagggggttt tttttgtatg attgtatttt ggggtttgtg 4320
ttttagggat ttttgaattt ttggtttgaa tgaatagtcg attgatatag ttttttattg 4380
tatgtagttt aggaatttta gttttaggtg attttataga ttattagtat ttttagtttt 4440
atttttgttt tttttcgagc gtgttttaat tttagtattt gtgttatatt tatttattgg 4500
ttaaaatgag aattgggtgg tagttttagg tttttttttt atgtcgaggt ttttttggta 4560
ttggtgtacg tatatttaaa gggaggtttt tttattagat ttgtttagtt aggagtttag 4620
agttttttat gttatatttt gtgttgggat ttttgtgtta gaggtttagt agttgtttat 4680
ttgtttagtt tattgtattt attgagtgtt gtttatgagt taggaattgg ttaagttttt 4740
aggatgtaat agggaaataa atagtaattg gtatgttaag aattaaaata aggtgatgag 4800
ataagagaat gataaggtaa ttatagaaga tttttgagga ggttgtattt aagatgtgat 4860
tcgagtttta taaaggagta tgttgtttta agattcggtg gaagaatttt ttagatagaa 4920
ggagtagttg atgaaggtgg cggtgatgat gaagttggta gtattaaata aatgtgttag 4980
attaggggtt ggttagtttt tattgtgaag ggttaggtag taaatatttt aggttttgtt 5040
gattatgtgg ttttttgttt ttttgtttgt ttgagatgga gttttatttt gtcgtttaga 5100
ttggagtgcg gttgtataat ttttatttat tgtaattttt gtttttcgcg tttaaatgat 5160
ttttttgttt tagttttttt agtagttggg attataggta tatgttatta tgttagttaa 5220
tttttatatt tttttttttt tttttgagat agagttttgt tttgtagtta ggttgtagtg 5280
tagtggtgta attttagttt attgtaattt tcgtttttcg aatttaagtg atttttttgt 5340
tttagttttt taagtagttg ggattatagg cgtatgttat tacggttagt aatttttgta 5400
tttttagtag agatgggaat tatttcggtt tggttaaatg ggttttatat ttttggattt 5460
atattttgtt gttttggttt ttcgaatggc ggcgattata ggcggggagt tttgtgttta 5520
agttagtttt ggatttttta ttataaattt tttgaagggg atttattata ggatgtttta 5580
tttaaagggg tttgtgggag ggttttgttt atatggaaat taaaatagag aaataaatta 5640

taatagtaat tagtgtttgt ttttgttttc gaggtttatt tgtgtaatgt agtttatttt 5700
ttatttgttg tttttgtttt tgtgtttttt agttgtgttt gttttgtaga tattttttta 5760
cggtggttat atagattttt tttgagaaaa attttgtttt gtataatttt gttggcgtga 5820
tttatatgtt tttaggatcg agtttaggtt tttaggagtc gaaaggtttt ttggggttag 5880
ggtttagttt ggttagatgg gttttgttta gaatgagtcg gtttttttag tttgtttttt 5940
atagtatttt tttatgcgtg ttcgttacgg gattt 5975

<210> 83
<211> 7041
<212> DNA
<213> Artificial Sequence

<220>
<223> chemically treated genomic DNA (Homo sapiens)

<400> 83

ttttggttat gttttgtttt tttttttttt tttttttttt ttttttttgg ggatggagtt 60
ttattttgtc gtttaggttg aagtgtagtg gtatgatttc ggtttattgt aagtttcgtt 120
tttcgggttt atattatttt tttgttttag tttttcgagt agttggaatt ataggcgttt 180
attattacgt tcggttaatt ttttgtattt ttagtagaga cggggtttta tcgtgttagt 240
taggatggtt ttaatttttt gatttcgtga ttcgttcgtt tcggtttttt aaagtgttgg 300
gattataggc gtgagttatc gcgttcggag ttttttattt tttttttagt ttttgttaat 360
ttttgttttt ttatttaatt tgtaaagttg aagtttttta aagtttaatt atggattttt 420
tttttttttt agttttattt gttttagggt gatttttttt tatttttgat ttttaatgtt 480
atttttatgt tgataatttt aatttatatt tgaaatagat tttttttttg ggatgtatat 540
attattattt atttgatttt tttatttgga tattttaagg gtatgtttag tttagtatat 600
ttaaaattga atttttgatt tttttttgaa aatttgtttt tttttttgtt ttttttattt 660
tagtgagtga ttggtttatt attattagat tttttggagt tttttttttg attggaatgg 720
aattttttga tttttttttt tttttttttt ttttaagttt ttatattaag tttttttaat 780
tttaaatatt ttttaaatta gtttgttttt ttttattttt attgttattt ttttagttag 840
tttattatta tttttggtcg tagtaggttt tttaatttgt ttatttgtaa tttaatttag 900
agtaattaaa gttatttttg taaaacgtta aatatatttt gtgaatttta agtttaaaat 960
tttttaatgt gtattttagt attttgggga tgtgaggtat ttgtaaaaga gatattattt 1020
tttcgggatt tagtttatat acgatataaa ttgaagtatt ttatgaaata tatttatttt 1080
ttttatatga ttttatgata tatttttttt attaattaat ttatttattt gttgtgtatg 1140
tgagatggag ttttgttttg tcgtttaggt tggagtgtag tagtacgatt tcgttttatt 1200
gtaattttta tttttcgggt ttaagttatt tttttgtttt agttattcga gtagttggga 1260
ttataggtac gttttattat attcggttaa tttttgtatt tttagtagac gggattttat 1320
tatattggtt aggttggttt ttaaattttg attttaggtg atttatttat ttcggttttt 1380
taaagtgttg ggattatagg cgtgagttat tatgtttagt ttatttttat attttttatt 1440
tttaaattga ttttagtatt ttttttagtt tgatttatat ttaaaagtat tgttttaata 1500
tttttttatt gtttgtagaa taaaatttat tttttttaat atggtttaaa aagttttgta 1560
taatttagtt tttgtttatt ttttgaattt tatattttat tttttttgtt ttttaagtat 1620
tatgttttag ttatattagt tttttatagt ttttaaatag gttatttttt ttttttttta 1680
gggttttttt atttgttgta ttttttgttt ggagtatttt tgtgtttatt tatttgttaa 1740
tttttatttt tttagggttt agtttaaata gtagtttttg tttgattttt taggttaaat 1800
tatatttttt tatatatata tattcgttat tttttttttt attattttgt tttgtttttt 1860
tataatttta ttataatttg taattatgtg tgtttatgtg ttgtaattat gtgtttattt 1920
tatatttttt ttagtattag ttaatgaatt ttgaggatag tttttttatt attgttttgt 1980
ttatgtttgt tttatttatt gttgtatagt tagtgtatta tatggtgttt ggtttatagt 2040
ttgtgtttaa aagatattcg ttgaatgagt tgagatttga agaataaatg aagtggaatt 2100
ggggagatgt agagaaagtt ttatttagcg agaatagtat atgtaaaaat ttggaggtta 2160
gagagattaa ggaatagttt aagaatttta gtacggaaag gttggtaggg gtatagatta 2220
tgaagggtta tttaattttt tttgttaagg aaggtgggtt ttattttgag ttagtagaga 2280
tttattgaag ggttttaaat atgggagtga tataatttga tttaattttt atttaattat 2340
atatatatat atatatattt tttttttttt tttttttttt gagacgagtt tcgttttgtc 2400
gtttaggttg gaagtagtat gattttggtt tattgtaatt ttcgtttttc gggtttaagt 2460
aattttttgt tttagttttt taagtagttg ggattatagg tattcgtttt tatgttcggt 2520
taatttttgt atttttggta gagatggggt tttattattt tggttaggtt gatttcgaat 2580
ttttgatttg aagtgattta tttatttcgg tttattaaag tgttggtatt ataggtgtga 2640
gttatcgtat tcggttttat ttatgttttt aaaagatata tattgtttat agtgtagaga 2700
atagatttta gagaataaga ttgaaatagg gaggttgtga tagtattgta ggttagaaat 2760
ggttttggtt taaattaggg tagtggtttt agaaatgtag agattatata aatagatttg 2820
gtaggaggtt aaaatgatgg gatttggtga ttgtatgggt gatggaggag agaaaagaat 2880
taagggtgta attcgggttt ttgttttgat tggggttatt tattaaggta agatataaag 2940

aaggataaat aggttggaag gaggatgttt tgagttggga agaagttgag gttttaggtg 3000
tttttgggat atttaggtag agttgtttaa taattagttg aatgtatggg ttaggagaaa 3060
tatttggttt tgaggtatta atttgggagt tattagtaaa gatatgatta ttgaagttag 3120
gggagtaaat gagataaaga aaagagcggt ttgtaattcg aggttttttt ttttttaagt 3180
ggattatttg aggttagtag ttggagattt gtttggttaa tatggtaaaa tttcgttttt 3240
attaaaaata taaaaattat tcgggtttgg tggtgtatgt ttgtaatgtt agttatttgg 3300
gaggttgagg tagaagaata gtttgaattc ggtaggcgga agttgtagtg agtttagatc 3360
gcgttattgt attttagttt aggcgataga gtaagatttt gttttagaaa gaaagaaaga 3420
aaaaaaaatt atatgtagag agaggataag agcgtttagg ttaaagtttt gaagagtatt 3480
agtataaagc gttggataaa gaggagtttg gaaagtaaaa aaagaattat taaggaagta 3540
gaagaaaaat aaggaaagtg tagtgttatg gaagtggttt aaggaggagg gagtggttaa 3600
tagtgttaaa tgttattaag aaattaagta ggataatttt tgaaaagatt ttatgggggt 3660
tgggcgcggt ggttgacgtt tgtaatttta gtattttggg agatagaggt cggcgaatta 3720
taaggttaga agttcgagat aagtttggtt agtatggtga aatttcgttt ttattaaaaa 3780
tataaaaaaa tagttgggcg tagttattcg ggaggttgag gtagaagaat cgtttgaatt 3840
taggaggcgg agattgtagt gagtcgagat cgtgtcgttg tattttagtt tgggcgatag 3900
agtgagattt ttgttttaaa aaaaaaaaaa aagaaaagat tttatgggat ttagtgagat 3960
gttttatggt agattttgat gagagagagt agttttaagt aagtagtgga ggtagattgg 4020
agtgggttga aagataagtg ggaggtggga aaatgaagat agttgggtgt aggtaatttt 4080
tttgagaagt ttgtagaaga gaagagaagg aggtggttgg agaggtagaa tttttggtaa 4140
ttgtttattt gtttttagat ggaagaggtt tatgtttaga tgttgttggg aaggagtaag 4200
gataaatttt taggtttcgt agtaaaaagt taagagtttt ttttacggtg gttttgttta 4260
ttttttgtag taggttaggt tatttgttga gggttatagg gtagatggag tatttggggt 4320
ttgaggggaa aggagaaggt ttgaaatggt tattgtggag aatagaggga gagttgatta 4380
gtagggttgt gggttaaaat tgagggtttg gtagaagttg ggaattatgg gtttatagtg 4440
gtataaattt gtgaagttga atagtttttt ttagtaatgt ttagttgttt gggcgtaggt 4500
atggaaaagg cggatagttg gatcgattta agattgggat gttgttaggc ggatgcgata 4560
aattagggaa ttgagggtat ttaagaaagt gattaagtaa gggattaaga tatttggttt 4620
gagtaaagtg aaggagaatt agagataggt tttttatgag ttaagtttta ggaatattta 4680
ggaatatgaa gaaagggttt tggaaaagag agtgaacggt aagtagaggg gggaagattt 4740
gagatgggat ggaagggtag atatgggggg agggggttga gttcggagat ttgtttgtgg 4800
ggaaagtagg ttgaggggga attttttaag gggtaggagg gttttgagag cggtagtagc 4860
gagaagtgag gcggtttcga gcgagggggc gtggttaggc gtaaatttcg ggcgaggggg 4920
cggggtttcg gtaattgggt tttagtgtcg ttgtggttgc ggttgttttt gcgaggtttt 4980
cgttttttta tttttttttt tagggttttt tttcgttcgt cgtcgtcgag atttaggttg 5040
aagttgggga ggacggtgga gttcggttta ggtcgttttt ttgttttcgc ggttggagta 5100
tcgtttggga ttattggttg aaggcgagtt tcgtagttta agcgtagcgt atacgcgcgt 5160
tttcggttat cgtcgttttt tttttttttt tttttttttt tttttttttt ttttcgtttt 5220
cggtttcggt tgcggcggga ggaagaacgt tttttaatta gttaggagag tttagattta 5280
tttttattcg ggagaaaaga agagatttcg tatttggatt cgatcgattt ttcgtttcgg 5340
ggttacggaa gtggttcgtc gtttatcgga gagttttcgt cgagcgtagt tttttaattt 5400
tcgtttaatc gtagtcgtat agatagcgtt ttttagcggg ttttcggttt cgtcgagttt 5460
atttttcgcg cggggtcggg gtcggggtcg gcgatgtttg gtacggtagc ggcggtggtc 5520
gcggttgttg ttgttgttat tgttatcgtt gttacggttg ttggttcggg ttcgggttgg 5580
gggtttgagt ttttgtagtg gggttgagtt cgtagtttcg tttttcgatt ttgaggtcgt 5640
tgtttcgtcg ggcgtcgggt ttttttcgtt cgcgttttcg ttttaggagt tggagttaag 5700
cggggagttc gggtttcgcg tttaggttcg gattatgtat ggttatcgag tttcgggggg 5760
cgtcgggttt tcggagttcg aatattcggt tacgaattcg tcgggcgtcg tttcgtcggt 5820
ttgtgtcgat tcggattttg gagtttcgga attcggattg ttggcgcgta ggggtttagg 5880
ttcggttttt ggcggtttat tggattttta gtttgtcgga ttttcggata ttagtttggg 5940
cgttgtttta ggttatcggg ttttgttttg cggttttagt ttatagtatt atcgggtttt 6000
gcgttttttt tattatttgg agggttcgta gtttcggttt ggtgagtgag cgattgagag 6060
agtatgggag ttcgggttac gggtttggta ttgggagcgg gtttttgttt tcgagttttt 6120
taagttttat ttcggaatag gttcgtttat ttcggttttt tgggtttttt taaatttttt 6180
ttttttttta cgttttttgg ttttatgttt agttttttta atttttgttt ttattttttt 6240
tttttgtttt ttgtgttttt taattttttt aatttttttt ttttttgttt gttgaggtta 6300
atgggagagg gagtattcgc gagaaaaaga gggaggattg taaggtagta atttttggat 6360
atttggttgg ggaaattagg gtttcgtttg gagaatagaa gaaaaaaagg agttttatat 6420
ttgttaagcg tttatattgt attaggtatt ggattgggtg ttttatatgg atttttattt 6480
tagttttagg aatttttatg aagtagatat tattagtttt attttgtagg gaaggaaatt 6540
gaagtttaga gaagttaagt aatttgttta aggttatata gttagtatgg agttacgatt 6600
taaattagat ttgtttgtgt ttaaagtttg tggttttttt gtttttttaa attgaggttg 6660
gagattatga aagtaatgtt aagttttttg tttatgtgat ttttttttgt attatttagt 6720
agttttggag tatgagcgga aaaaaaaaaa aaaaaaaaaa aaaagaaaga cggtggattg 6780
atttaaggtt gagattttgt taggtgtgtg tggcgggaga ataaggaaag gaaggagtag 6840
aaggtgttga gagagaaagg tggaagaggt gggttttggg agttaggtta gaggagaagg 6900

ttggaggggt tgataggtag ggagatagta gaaaagttaa tagaggtaga agttagttgt 6960
agtaggagtt ggagagttgt aggtttacga ggttggttgt gatgtgaaat taggatggtt 7020
ttttggattt gaaaaggtgg a 7041

<210> 84
<211> 7041
<212> DNA
<213> Artificial Sequence

<220>
<223> chemically treated genomic DNA (Homo sapiens)

<400> 84

tttatttttt taaatttaag aaattatttt gattttatat tataattaat ttcgtggatt 60
tgtagttttt tagtttttgt tataattggt ttttgttttt gttggttttt ttgttgtttt 120
tttatttatt agttttttta gttttttttt ttagtttgat ttttaaggtt tatttttttt 180
attttttttt tttagtattt tttatttttt tttttttttg ttttttcgtt atatatattt 240
ggtaaaattt taattttgga ttaatttatc gttttttttt tttttttttt tttttttttt 300
tttcgtttat attttagggt tgttgaatag tataggagaa aattatataa gtagaaggtt 360
tggtattatt tttatggttt ttagttttag tttggggaag tagaaagatt atagattttg 420
gatataaata gatttggttt gaatcgtggt tttatattag ttgtgtgatt ttgggtaagt 480
tgtttaattt ttttgagttt tagttttttt ttttataaaa tgggattaat ggtatttatt 540
ttatgagggt ttttgaagtt gaaatgaaaa tttatgtaaa gtatttagtt tagtgtttgg 600
tataatgtag gcgtttggta aatgtaaggt tttttttttt tttttgtttt ttaaacgaaa 660
ttttggtttt tttaattaaa tatttaagga ttgttgtttt gtaatttttt tttttttttt 720
cgcgagtgtt ttttttttta ttagttttag tagatagagg agaagggggt tggagaggtt 780
gaggggtata agggatagag agaggagatg gggatagagg ttgaaaggat tggatataag 840
gttaagggac gtggggagga ggagggggttt gagggaattt aaagggtcga ggtaagcgag 900
tttgtttcga ggtggggttt agagggttcg agaataagaa ttcgttttta gtattaggtt 960
cgtggttcgg gtttttatgt ttttttagtc gtttatttat taggtcgagg ttgcgagttt 1020
tttaggtggt aagagaagcg tagggttcgg tggtgttgtg gattgggatc gtagggtaag 1080
attcggtggt ttggagtagc gtttaggttg gtgttcgagg gttcgataaa ttggggattt 1140
agtgggtcgt taagagtcga atttgagttt ttgcgcgtta gtagttcggg tttcgaggtt 1200
ttagggttcg agtcggtata ggtcggcgga gcggcgttcg gcgggttcgt ggtcgggtgt 1260
tcgggtttcg aaggttcggc gtttttcggg gttcggtggt tatgtatggt tcgggtttgg 1320
gcgcggggtt cgagtttttc gtttggtttt agtttttggg gcggaggcgc gggcggagga 1380
ggttcggcgt tcggcggagt agcggtttta ggatcggggg gcgggattgc gggtttagtt 1440
ttattgtaga gatttaagtt tttagttcgg gttcgggtta gtagtcgtgg tagcggtggt 1500
agtagtagta gtagtagtcg cggttatcgt cgttgtcgtg ttaggtatcg tcggtttcgg 1560
tttcggtttc gcgcggggag tgggttcggc ggggtcgggg gttcgttggg gagcgttgtt 1620
tatgcggttg cggttgggcg agggttggag ggttacgttc ggcggggatt tttcggtgga 1680
cggcgagtta ttttcgtggt ttcggggcga gaagtcggtc ggatttaagt gcggggtttt 1740
tttttttttt tcgaatggga atagatttgg gtttttttgg ttggttggga agcgtttttt 1800
ttttcgtcgt agtcgaggtc gggggcggag gagaggggag gggagaggag gggagggaag 1860
agagaacggc ggtggtcggg ggcgcgcgtg tgcgttgcgt ttgggttgcg gggttcgttt 1920
ttagttagta gttttagacg atgttttagt cgcgggggta ggagaacggt ttgggtcggg 1980
ttttatcgtt ttttttagtt ttagtttggg tttcggcggc ggcgggcggg aaggagtttt 2040
gggaaggggg gtggggaggc gggagtttcg tagaggtagt cgtagttata gcggtattgg 2100
gatttagtta tcggggtttc gttttttcgt tcgggatttg cgtttgatta cgttttttcg 2160
ttcggggtcg ttttattttt cgttgttatc gtttttaagg tttttttgtt ttttaaaaaa 2220
ttttttttta gtttgttttt tttataggta agttttcggg tttaattttt tttttttata 2280
tttgtttttt tattttattt taggtttttt tttttttgtt tgtcgtttat tttttttttt 2340
aaaatttttt ttttatgttt ttgagtgttt ttagggtttg gtttatagag gatttgtttt 2400
tgattttttt ttattttgtt tagattagat atttggtttt tttatttagt tatttttttta 2460
aatattttta gttttttgat ttgtcgtatt cgtttggtaa tattttagtt ttgaatcggt 2520
ttaattgttc gtttttttta tgtttgcgtt taggtagtta aatattgttg gggaaaatta 2580
tttaatttta tagatttgtg ttattataaa tttatggttt ttagttttta ttaggttttt 2640
agttttgatt tataatttta ttagttagtt ttttttttgt tttttatagt gattatttta 2700
aatttttttt ttttttttta aattttaagt gttttatttg ttttgtaatt tttagtaaat 2760
aatttgattt gttgtagaga gtaaatagag ttatcgtaga ggaagttttt gattttttgt 2820
tacgaagttt aggaatttgt ttttgttttt ttttaatagt atttaaatat agattttttt 2880
tatttaaaaa taaataaata attattaaga attttgtttt tttaattatt tttttttttt 2940
tttttttata aattttttaa aagagttgtt tatatttaat tgtttttatt tttttatttt 3000
ttatttattt tttaatttat tttaatttgt ttttattatt tgtttgaaat tgtttttttt 3060
tattaggttt tgttatgaaa tattttatta aattttatag aatttttttt tttttttttt 3120

ttttgagata agagttttat tttgtcgttt aggttggaat gtagcggtac gatttcggtt 3180
tattataatt ttcgtttttt gggtttaagc gatttttttg ttttagtttt tcgagtagtt 3240
acgtttagtt atttttttgt atttttagta gagacggggt tttattatat tggttaggtt 3300
tgtttcgaat ttttaatttt gtaattcgtc ggtttttgtt ttttaaagtg ttgggattat 3360
aggcgttagt tatcgcgttt agtttttata gaattttttt agaggttatt ttatttggtt 3420
ttttgatagt atttggtatt gttgattatt tttttttttt taaattattt ttatgatatt 3480
gtattttttt tgtttttttt ttattttttt agtaattttt tttttatttt ttaggttttt 3540
ttttgtttaa cgttttatgt tggtgttttt tagggttttg gtttgagcgt ttttgttttt 3600
tttttatatg tgattttttt tttttttttt ttttttgaga tagagttttg ttttgtcgtt 3660
taggttggag tgtagtggcg cgatttaggt ttattgtaat tttcgtttat cgggtttaag 3720
ttattttttt gttttagttt tttaagtagt tggtattata ggtatgtatt attaagttcg 3780
aataattttt gtatttttag tagagacggg gttttgttat attggttagg taggttttta 3840
attattgatt ttaagtgatt tatttgagga aagagaagtt tcggattata ggtcgttttt 3900
tttttttattt tatttgtttt tttggtttta atgattatat ttttgttgat gatttttaaa 3960
ttgatatttt aaaattagat attttttttg atttatgtat ttaattggtt attaggtagt 4020
tttatttgaa tattttagag atatttaaaa ttttagtttt tttttaattt aaggtatttt 4080
ttttttaatt tgtttgtttt tttttgtgtt ttattttagt gaatgatttt agttaaagta 4140
gaaattcggg ttatattttt aatttttttt ttttttttat tatttatgta gttattaagt 4200
tttattattt tgatttttta ttaaatttat ttatgtagtt tttatatttt tggggttatt 4260
attttggttt agattagaat tatttttaat ttgtaatatt gttatagttt ttttatttta 4320
gttttgtttt ttggaattta ttttttatat tataggtagt gtatattttt tgaaaatata 4380
aataaggtcg ggtgcggtgg tttatatttg taatattagt attttggtaa gtcgaggtgg 4440
gtggattatt ttaggttagg agttcgagat tagtttggtt aaaatggtga aattttattt 4500
ttattaaaaa tataaaaatt agtcgggtat gggggcggat gtttgtaatt ttagttattt 4560
gggaggttga ggtagagaat tgtttgaatt cgggaggcgg aggttgtagt gagttaagat 4620
tatgttgttt ttagtttggg cgatagagcg agattcgttt taaaaaaaaa aaaaaaaaaa 4680
aaaatatata tatatatata tataattaaa taaaagttaa attaaattat attattttta 4740
tgtttaggat tttttagtga gtttttattg gtttaaaatg aaatttattt tttttaatag 4800
gaaaggttaa atggtttttt atgatttgtg tttttgttag ttttttcgtg ttgaaatttt 4860
tagattattt tttagttttt ttagttttta agtttttgta tatgttgttt tcgttgagtg 4920
aaattttttt tgtatttttt taattttatt ttatttgttt tttagatttt agtttattta 4980
acgaatgttt tttgaatata aattatgggt taggtattat gtgatgtatt agttatgtag 5040
tagtgaataa gatagatatg aataagataa tggtgaaaaa attgttttta gagtttattg 5100
gttggtattg gagaggatgt gaaataaata tataattata atatataaat atatataatt 5160
ataaattgtg ataaggttat aaagggatag aatagggtga taggaaagag aataacgggt 5220
atgtgtgtgt gggggggtgt gatttagttt gaggggttag gtaagggttg ttatttaagt 5280
tgagttttga aggagtggga gttggtagat gagtggatat aaaagtgttt taggtaaagg 5340
atatagtaag taggaaggtt ttggggagaa agagaaatgg tttatttagg agttatagaa 5400
ggttaatatg gttaaagtat agtgtttgga aggtaagagg ggtgggatat gagatttaaa 5460
aggtaggtag gagttagatt atgtagggtt ttttaggtta tgttaagaag aatgggtttt 5520
attttataag taatgaaaag gtattgaagt agtgttttta aatgtgggtt aaattggaga 5580
aggtattgaa attaatttaa gaatagaaaa tataagaatg ggttgggtat ggtggtttac 5640
gtttgtaatt ttagtatttt gggaggtcga ggtgggtgga ttatttgagg ttaggagttt 5700
gaggttagtt tgattaatat ggtgaaattt cgtttattaa aaatataaaa attagtcgag 5760
tgtggtggag cgtgtttgta attttagtta ttcgagtggt tgaggtagga gaatggtttg 5820
aattcgggag atggaggttg tagtgaggcg agatcgtgtt attgtatttt agtttgggcg 5880
atagagtaag attttatttt atatatataa taaataaata aattaattaa taaaaagagt 5940
gtattatagg attatgtagg aagggtaaat atgttttatg aaatgtttta gtttgtatcg 6000
tatgtgaatt ggatttcgag gaaatagtat tttttttgta gatattttat atttttaaaa 6060
tgttgaaata tatattgaaa ggttttaagt ttgagattta tagggtatat ttaacgtttt 6120
ataaagatga ttttggttgt tttagattga attgtaggtg gataagatta ggaatttatt 6180
gcggttagag atgatggtga gttgattaag gaggtggtag tggagatgga gagaggtaga 6240
ttgatttgag agatatttag aattgagaag gtttggtgtg aaagtttggg agaaggaggg 6300
ggagggggga attaagggat tttattttaa ttaagggggg aattttaagg gatttggtga 6360
tggtaggtta gttatttatt gagatgagga agatagaaag aggaatagat ttttagggga 6420
agattaagag tttagttttg gatatgttaa gttggatatg tttttgagat atttaagtgg 6480
agaagttaag tagatagtgg tatatatatt ttaggggaga agtttgtttt agatataagt 6540
tagagttgtt agtatagaga tggtattaaa agttaagagt agaaaaaaat tattttgaaa 6600
tagtaaagat tgagaaagga agaggattta tgattgagtt ttgaggaatt ttaattttat 6660
aggttgagtg aaggagtagg agttggtaga gattgaaaaa gaagtaaaga gtttcgggcg 6720
cggtggttta cgtttgtaat tttagtattt tgggaggtcg aggcgggcgg attacgaggt 6780
taggagattg agattatttt ggttaatacg gtgaagtttc gtttttatta aaaatataaa 6840
aaattagtcg ggcgtggtgg tgggcgtttg tagttttagt tattcgggag gttgaggtag 6900
gagaatggtg tgaattcggg aggcggagtt tgtagtgagt cgagattatg ttattgtatt 6960
ttagtttggg cgatagagtg agattttatt tttaaaaaaa aaaaaaaaaa aaaaaaaaaa 7020
gaggtaggga tataattagg a 7041

<210> 85
<211> 16602
<212> DNA
<213> Artificial Sequence

<220>
<223> chemically treated genomic DNA (Homo sapiens)

<400> 85

tatatggttt agaatttaga agaaaagtta tttttttttt ttttgagata gagtttcgtt 60
ttgtcgttta ggttggggtg taacggcgta attttggttt attgtaattg tcgtttttta 120
ggtttatgtt atttttttgt tttagttttt cgagtagttg ggattatagg tatgtattat 180
tagatttggt taatttttgt atttttagta gagatgggtt tttgttatgt tggttagatt 240
ggttttgaac gtttgatttt aggtgattta tttatttcgg ttttttaaag tgttaggatt 300
gtaggtatga gttattatat ttggtttgaa aggttatttt taaaatgaaa ttgttttata 360
attagaggta aatgggattt atattggagg aagtttagag ataaggagaa ataaaattcg 420
gtttatttaa gattgataat ataaatgatt atttgtgtag ttaagtggta attattggta 480
tataattatt gttttttgta ttaaatgaag tatataattt aggaagtgtt tggaaaaaat 540
aaatgaaaga aaaaaattat tattttttag ttgtatttta aggaagggga atttggtgat 600
ggttgagtgt taatagagag gtgagggata aagatattta tattgggaag agttaaattg 660
ttttatatta gaagtggatt tagaagggag tggagtgagg agttagagtg agaggttggg 720
tggagagagg agtgagtgga gagataggaa gagatagagg gatagagagt aagagagtta 780
gaggtggata tagaaggatt tagaggttta tggataagaa agtaaggttt ggaagagata 840
gtgaggatgg agagagattg tattatgtaa ggtaaatttt gtaatttttt ttgtgaaatt 900
gatataaata aaattatata tatgtggtat tatatttatt ttttgtttgt tgttgtatta 960
tgtgaaaaga attaatcgaa gttttattat atgggatgga ggggatattt gggatgattt 1020
gattttaaat atagtttata tggtatttgc gaaatttttt ttgggatttt tggaggtatt 1080
tttaatatta gttgaaatta gggataatgg ttggtttatg tggtggtaga gtgggaaaaa 1140
tgtattagaa atattaaaat gttaggagta gagaaaatag atggaggttt ttaatacgag 1200
tttttatttt aaagttataa ggataaatta ttgtaattgg tgttttgatt tatgatggaa 1260
ttatttttta tgagagtagt ttcgtatagt tgttataggt attgatggat ggttagtgta 1320
gggaaggtag ggtagttgtt ggttttttat gatgtttacg aagttgttgg ggtgggtttt 1380
ttttggagaa ggtggagatg tatggagttt tgttttatag atggtatggg ggtaaaagga 1440
gaggttgttg gtggagatta gttaattttt gggttataaa taaatatatg aatgataagt 1500
taatggtggt agatgtatat gttaggtaat gggtaaaaat tttatttttt ataaaaagat 1560
tagtgtggat aaagttatta aattttttta tggtgattaa gggtaaattg gggttttttg 1620
tttttagatt ttttttgcga tttagtttta tcgtttatta gttgttgttt ttgggtaggt 1680
tattgaatat ttgtaagttt tggttttatt aaaaatgatg ataatattag tgtttatttt 1740
aagggattat ggtgagatta tttgagataa tttgtagttt aaaatacgtt aatgatgagg 1800
gtgatgattt ttattttaag attatttgat aattaatatg tattgatgat tatatttatt 1860
atttatattt ataaagtata gtttttaggt cgggcgtggt ggtttacgtt tgtaatttta 1920
gtattttggg aggtcgaggt aggcggatta cgaggttatg agtttaagat tagtttggtt 1980
aatatggtga aattttattt ttattaaaaa tataaaaatt agtttggtgt ggtggcgtat 2040
ttttgtaatt ttagttattt gggaggttaa ggtaggagaa ttgtttgaat tgggattcgg 2100
gaggttaagg ttgtagtgag ttgagatcgt gttattgtat tttattttgg gttatagaga 2160
tttcgtttta ggagaaaaaa aaaaaagtgt agtttatacg ttagtttttt ggtttttttt 2220
tggttttttt ttttttagtt tttagagtta ttagtatttt ttttttttaa atggtggggt 2280
aagtatatgt tttaattaat tttaatgttt attggttttg ttttttaatt ttttgaggtg 2340
taagtgatgt atttttatat taataatata agtttgtatg tagtggttat atataatgtt 2400
aaaggtatat tatatataat atggtggttt ttgtgtttta ttttatatta gaaaatagtt 2460
aggatgaaaa tagtttgcgt gtaagggata gatttatttt ttttgtgtta tatgaagtta 2520
tgtggaaagt tgtagaatgg gtaggaatta ggtatagttt ttataatgat ttgttttata 2580
atattttttt tgatatggta tttgtttttt aaaaatttta tttattttta ttttgtgttt 2640
ttttttttgg ttttagtttt gtttttttta gtaaatttag aagttttttt taatatatta 2700
attttttatt ttttttgttg tgtttttgaa aggttttttg tgtttttggt tgtaggtttc 2760
gaacgtttag gttatttgtg ttatttgttt cgcggtattt tattaacgta acgtgagggt 2820
ggagggtaga atttggtttt tggtttttta gtttttgtgg gttttagtta gattttaggt 2880
gttattttag tgtaattttg gtgtttaatt tgaggatgtg tgtggattag aaggagggat 2940
taaaatatga tttttttttt tatggttaga tgattaaatt tgaagtttta aaaaatgtag 3000
tttggtttaa agttgtgttt aattgggaag agagaaaaat gttttggaaa ttttttttag 3060
gtttgggatt attttttttt aattattagt tattttatag gttcgcggat tgcgggtatt 3120
atttgggtag gttgtgttta tttattattc gggaattttg tgttttggag ttgttttttt 3180
tttttttaaa gtgtattttg tgtttttgtt ggaagaatcg attataggtt tgtttaattt 3240
tttatagttt tgaaagcgtt ataagtagta gttgttgagt tatggttgaa ggggaaatta 3300

ttatttttat agttttgatc gagaagttta atttgttttt agggaattat aagaagttta 3360
aattttttta ttgtagtaac gggggttatt ttttgaggat tttttcggat ggtatagtgg 3420
atgggataag ggataggagc gattagtata gtaagtttat ttttatggta tttttttttt 3480
ttttttgata ttttttgtag ttaaggtggg aggaaggtgt atatttaagt ataggtattt 3540
gtttttttaa ggttttattt aggtatgata tatttagagg tggagttata taaatgcgta 3600
aaatgtttgg gtttagaaaa tagggatttg tgggggttat tatttattta aacgtgtttt 3660
attttaagtt ttttaaagta ttttttgttg atttaataga acgggttgtc ggtgtttaat 3720
tgttgtatgt ttttttaggt ttttgtaatt agtgaaagat ttggtttcga aggttagtta 3780
ggggtttgta cgttttatga tgggatgaat tagataggta tggtggtaaa ttttgtatag 3840
agatgtgggt ttcgttttaa gttaattgat ttttttggtt atgtttttat tgggtttgat 3900
ttgtgaaatt ttagtttata atcgtagttt tatatattat aaattatgta gttttttttt 3960
tgcgtattac gtagatgtgg ttgtggtaat tagtagtggt aaagttattt tttagatttt 4020
atagaaattg gagagtagga gcgattagcg ttaaatgttg aatatttttt ttaggatttt 4080
ttggtagttc ggtttttaga ggtgttgata aatgaaggta tgattttaga gaaaagaatt 4140
tttttgatta tttaagtata aaaaaatttt ttttttttgt tttggtattt ttggtattgg 4200
tgtgtatgtt tgatattagg tgtttaggtt ttatcgtgat gttatttgag attttttgag 4260
ttttattgta gtattagagg ttataaagaa gtttaaagtt agatttagtt tttgtttaga 4320
gggttagtaa ttttaagagt taaatttgtt ataaattaag agaaggaaaa ttaggagata 4380
tagaagaaga taggtaggaa ggaagttttt gtaggattgg ttttaaaatg taatgtaaaa 4440
tatttatttt gatatgatgg tttatatatt aggtggtttt aattatttat ggattatgta 4500
ttagaggttg taggttttaa gaataaatga ttatttcgtt ggtgtttacg ttgtattttt 4560
agatgtatag gtaatgggga tggacgttgg cgtttatacg aggtgacgtt gttttttttt 4620
ttttatttat tgtatagatt ttggagatga ggagtaggaa ggttgaggtt ggggtttgta 4680
gttgtagaat tagttttttt gggttttgtt ttgatggttt atttttaaat gggtttgttg 4740
aatgttttat atttagtgaa aaataggtgt tagaatgagg taaaaaaaaa agattttagt 4800
ttgttaaata gtatatataa gtaatattta ttgtaaatta gtaaatttag attgtagttt 4860
attgtgttag gtatgatata taatttacgt gatttgtttt atataatacg aataagtttt 4920
aatttttttg ggttttaagt gtttttaata cgttttaatt aattttttga aaaagtaggg 4980
aatgtttttc ggtttttaga atttaagagg aaagataaat tttatgatat gttttttggg 5040
gttaagttag tttttttgga aattttgatt tttggagtaa aaaaaggttt taatattgga 5100
atttttgtag taatggaaaa tggtttaatt gttgttattt tattattagt ataatagatt 5160
gtattttggg tgtgtaatat tggtataatt tttggagttt atagtggatt tggtcgtttg 5220
atttaagatg ttaggagttt ttagaaagta gtataacgta gttttgggag tgtaggtgag 5280
gatttttgta tttaaggaat gttagattat tttttaagtg aatttgggta attgtatttt 5340
ttatttttat tttttttatt tgtataaaaa gaatattaat attttttgtt ttataggttt 5400
tttgggaggg ttaaatgaga taatatagtg agagtttttg gtatagtggt tggtatagag 5460
gaatagtata gtacgtgttg gtttatattg tagttattat tattaagttt tttatataat 5520
tagtttagta tatggtaata tagggtcgta ttgaaggatt ttttagattt ttttttttaa 5580
tagtattgtt atttaggaaa gttaaggggt tagatgtagg tttagattta gtattttttt 5640
gttaatattc ggagttgttg tgtgggttta ttatttgttt attttggaat taagaataag 5700
gattaagagg atagagatta gagtaagtgg ggtgggtggt tagaagttta ttcgtgtatt 5760
tgttagtatt ggaattggat attttattga agagttagta ggttcgagtt taagtttatt 5820
ttgttttttt ttatttggag tagttttgga taattaacgt ttttgttatt atgtattttt 5880
tttgtttgta aattttgcga aagaagtttt aagtttttta gttttgggac gtttttagtt 5940
tttttttgat tagaaattga agtgttttta gtgtttttag ataattttta ggaaaattat 6000
ttagtttcgt tgagatttaa tttttttatt tgtggaatgg gaatggtagt attgtttagt 6060
gtgttttata agggcgaaat ttttatttat tagggttttg taggtgagag gagttttggg 6120
tacggatgta ttttttgaat gtggttttgt tgtttttttt tgtttagtgg tggtattgtt 6180
agagtttttt tgagttttaa gggttattta gaaatagaat tgtttcggtt aggtatgaga 6240
cggatgtttt ttgattaaat gtgatttata cggtttggta aggagatatt tttgattttt 6300
tgattattag tttgagtata attagagatt gaagtgatag agttatacgt taagattttt 6360
tttagaaaga gcgtttttaa aagttaatcg aaaaatttaa ataaattata taagatagaa 6420
tcgtatgttt ttgagtttta taaggtagaa ataatttttt ttacggtttt tttttgtttt 6480
taagttattg attatttttg ggtaaatgta tttttttttt atttttagga gaatgagttt 6540
ttaagtattt aatttttttt atagtggtat aattttaggg tagatgtttg tgtgtgggtt 6600
gttttaatag agatttgagt aaaatgtttt tattggtgga tttttagttt gtagagaagg 6660
tggttttggt ttgttttatt tttatttttt agatcgttgt tgtaggtaat tattaatatg 6720
gattgtgaat attggttacg ttgttatgtt aggaaggtta ggtagaatta gttagggagt 6780
gttgtataga ttttagggtt gatgggtata gtttgtgaga aggtttatgt tgttttttaa 6840
gttttagttt agggggatat ataagtggtg ttattatgag agggggaggt tttggaagaa 6900
gattaggttt aggggaagat ggttagtttt attttggata taagtattga ggagtgtttt 6960
acgtattatg tagttgtttt tagggttata attttttatt tatttgtgcg attatttaat 7020
ggtttttttt tttattggat ttttagagga taaggatttt gttttttatt gtttaatatt 7080
ataaattttg tgtttagtat attgtttgtt atttggtaaa atgtttagta aatattaatt 7140
tgataagtga ttgaattgta ttggtggtag ttaaatttaa agttaataat atatttaata 7200
aatatttatg agttttgata tgtgttaggt attatgttga atttttttat ttttatttaa 7260

ttataatttt gagaagttat taattattat tattttaaga atattgtagt tgataggcgt 7320
gattagtttt taattgggag ggtttggtat taattatatt ttttgttttt aggattaagg 7380
ttttttttgt attttataat tattcgatat taatattatg atagtggttt cgttttataa 7440
attgatttaa taaatatatt tgtgttatgg attagatggg tgtaggtcgg gttttaatat 7500
tgttgtttat tagttgtatt attttgggat agttattttt taatgtgttt tagttttttt 7560
gttttaaaaa taagaatgat aatagtattt atttatagga acgtaggagt tttaatgtga 7620
taatgtttgt aagagtgttt agtatattgt taggtatata agtgtttaaa aaaaatggta 7680
ttgattatta ttttttcggg atttttgagt tttttttatt taattagttg attttttaag 7740
tttgttagat taagtgaatg ttatgtttgt tcgtagaatt gagggtgatt ttttgtttta 7800
gtttttttgg gattatttgg gttgaagttt ttgaggtttg atatagaggt ataatgtgtt 7860
tagatatagg tgtagtaagt ttcgttttat tttatttttt tggtagggta agtttgtttt 7920
gtgattgatt ttgatttttt tggggagttt ttttttaggt gaagggggat tatgatttat 7980
tttttaatgg gattaataat tgtaataata attagagaat aattttttta taagtatttt 8040
aaatttttag ttgtttgtta tatttataat tttttagttt tttaagtacg taagagatta 8100
cgtggggagt agaattatcg gtattattta ttgtataaat ggaaatgttt ttaagtaaag 8160
tgcgtgttaa ttaaatattt ataaattaag ttatatttta aaggcgttag tagagtttat 8220
tggttaaaat aattttattt tgtagttttt tttaaagggt agattttttt ttatgaaatg 8280
ttttttgttt tgttgtttta ggggagttta gatttttatt ttttggttgg gattatttag 8340
tatttagtag ttaaggtatt ttatttttta ttaatataga ttcgaagtta aaatagtttt 8400
ttaaatgatt acgatgggga gagttggatt ttattgttag aagttagaga gaaggggagg 8460
tttttaggta ggaatgtatt tcgcgatagg gtttttttat ttcggttata tgtgatggga 8520
aggatttttt gttttttttt ttataatttt tatttgatta tatagaggat ttttatatat 8580
aaattttaaa atatatatat atatatatat aatatattat atatattata tatatatgat 8640
atatatgtgt atatatatta tatatatatg atatatatgt gtgtgtatat atatatatgt 8700
tatttagttt ttggattgta ttaaaatttt ttagataatt tttttagatt ttaaagaagt 8760
agatattttt taagataagg tttttttttt tatatatatt tatatatatg tgtgtgtgtg 8820
tatatatata tatatatata tatatatata tatatataga tgtgtatata tatttatata 8880
tatgtatatg tatatatata tattttttat tattggaatt attttttttt aaattcgatt 8940
tttttttttt tttgtttttt tttaatgttt tattaatttc ggtttgtggt tatattttaa 9000
ttataaatgt tattgatata aaataatcgt attaattttt gaatgaggtt gattttattt 9060
tgggtaaata cggtaaagga cgggaagttt ttaatttaga ataaattagg tatataattt 9120
tttttggttt ttttttggag agtagaggcg gaaagtaggt ttagaggtta tagatagata 9180
tgttatagaa gtagaatttt tttttttagg tatttttttt attgaatgta ttagttgtgg 9240
ttttatcgtt atatttgttt gggatgggat aggaagatgt taggatagtt tggggtttag 9300
aaatagggtg taagatttta aagttgattt tttttggtat tggtaggatt ataatttttt 9360
tttttttttt ttatttttgg aaagtaaggg attgttattt ttattgtgaa taaattattg 9420
ttaagattta atggaaggtt ttggggagta gaggagaaaa ttaaagttat aaatttagtt 9480
ttttagataa aaggtgtgaa tagtgagttg cgggttagta tagttatgta aaagttttgg 9540
gttttttttt atgtaggttt tttaggtatg tagggattcg tgaagtagtt taaataatga 9600
gtatgggata aggaagatta tgatttataa aggtgagttt gtatatttta gttttattga 9660
tatgtagttt tgataaatag gttgttttta aattaatatt ttttaattta tttttagtaa 9720
aggagtagga ttttggattt tattgcgggt atatatagtt ataaatttat taagtaaata 9780
tttttagttt ttttttcgta tatttttata atgttttatt tgagtttgaa tttttttaag 9840
aaatttttat cgttagagta tttttttaaa agttgttttt tattttttta gttaaattaa 9900
ttgttaatgt ttttatttta gaaggggaaa atgaagtatt ttatagatta tggacgtatt 9960
ttgtttttta aaaagtattt gtttttttat ttttgggata tttgagagat attgattttt 10020
ttgtttatag atagtaattt ttagattttc gtatgggata aagaagagtg tttgtttttt 10080
atttgttttg tgcgttaatt tggtcggggg gtattgaaaa agggagtgga tagagaagtt 10140
agagttttag aggtgtttta tttattcgtt ttgttttttt attttttttg ggtttttgtt 10200
tgtttttaag aagatttatt ttttataagt gtttatgttt tggtttagat gtaatatgga 10260
tttaaattta agttgggtta taaaatattt taggggttac gaggaataaa aagtgggagt 10320
tttggttagt tttaagtgtt ttattgatta tgaatggagt tttaatatat tagttattat 10380
tttaaaatgt atgaaggaga attaggggag attatagtaa atttttgatg aaaattttgt 10440
aaaatatata aaataatatg tatagtttat gtatatatag taaaatagaa aattatgtat 10500
tagatataga ttaaatatta gtaaatatta ggaattttat tatttagagt tttttagtgt 10560
tattagttgg ttgtttttat tttagaattg tttataaaga aatagagtta ggatgaagga 10620
aatatttttt taattttttt tttagtttta gtttttttta ttttttttaa agtagttatt 10680
tgtatgaatt tggttagtat ttggtgtata gttttatatt ttattataga tgtatttatg 10740
agttatatac gtattgtgtt tttttttttt tttttttttg agatggaatt tcgttttgtc 10800
gttaggttgg agtgtagtgg tacgatttcg gtttattgta attttttttt ttcgggttta 10860
agtaattttt tcgttttagt ttttcgagta gttgggatta taggcgttta ttaatatatt 10920
tggttaattt tttttttttg tatttttagt agagacgggg ttttattttg ttggttaggt 10980
tggttttgat tttttgattt cgtgatttat ttgtttcggt tttttgaatg tattgtgttt 11040
tttaatatga tttttattaa ggatttatta tattttttta ttgtaattta gtttttttta 11100
ttgtatgtta tattttattt ttttttgaaa ggggattaaa aaaaaaaatt atatatatat 11160
atatatatat atatatatat atatatatag agagagaaag aatgttgtag attttgtgtt 11220

tttgtttgtt tttagtttaa aaataattgt agtttttgtt attgtgttgt tgtattattt 11280
ttaggtttat ttgtttgtat ttttaattat gtagaagttt taagtgtagg gattatggtt 11340
ggcgttgagc gtaagtttta gtatttagta atgaatatgt gttgattgaa tggttttttg 11400
ttttttaatt ttgagttgtt attcgaggaa aaagttgttg ttgtgtggtt agtttttttt 11460
ttttttttta aggtttattt tttttatgtt tttttttttt ttgtttgttt ttagtagtag 11520
ttgtaagagg atgtaggttt ggaaaattgg aatgaagtta tattagagag ttggatatta 11580
agttagttat acgagtcgtt tagtttgggt gttgtttttt tttgttagtt ttatttgtag 11640
aaggatatat gtttaggaaa tttagttaga aagaaattta gtgaaaatga gttatgaaat 11700
ttggatattt tggagaaata atatttatag aggaaatttt tgagacgaat ttttatcggt 11760
ataataaatt tggtgtcggg atttatggtt gagggttgaa gttatgtgtt aagtaggttt 11820
ttggtttttg tttattttta tgaggttttt ttatggttat gatggaattt tatagatggt 11880
aaagttaatt ttatttagtt ttatggttag ataagtgggt gttattgaag gtagtaaaat 11940
agaaatgtat aggaaatgaa aagtttttta aaaatttttt ttgattggtt gggtacggtg 12000
gtttatgttt gtaatttcgg tattttggga ggtcgagggg ggtggatcgt ttgaagttag 12060
gagtttaaga ttattttggt taatatgatg aaattttgtt tttattaaaa atataaaaaa 12120
aaaaaaaaaa aaattagtta ggtatgatgg tgcgtgtttg taattttagt tatttgggaa 12180
gttgagatag gagaatcgtt tgaattggga ggtggaggtt gtagtgagtt gagattatat 12240
tatagtattt tagtttgggt aatagagtga gattttattt taaaagaaag aaaaaagaaa 12300
taaataaata aaaaaaattt ttttgattat ttaaaaagat tttttttttg gggtttttgt 12360
cgtatttata atgtgtttga ttaaatattt atatatatgg gcgataggtt gaaaagagtt 12420
ttaggtaatt aattttattt tattttatgg ttatagatgg gattttggat ggttatttat 12480
tattttgttt atgtgtgatt ttgaatataa gagggtttaa gtaggagagt atcgttggat 12540
ttggattaat ttatttttat aattggagtt taagagattg tgtgaaagat aggaaaaagt 12600
aggatatttg gagtattcga tttggggttt ggttatgatt tttttttttt ttagaagtat 12660
aatattgcgt aaaatatatt tttattaaat tttagttttt tattaaagga tagagtaagt 12720
attatttttt tttagggaat atttcgagga gaaaaggtta taaaagtata gaggaaatag 12780
ttatacgagt attggttatt ttttatgtat tagtttttat aataattttt attatagata 12840
aggaaataga ggtaaatatt ttgttcgatg attatatagt taataggtag ttaagtagaa 12900
tttgagttta tgtagttgtg ttttggagtt tatatttttt aattttatat tgtttatatt 12960
aagatagaaa ttagtatggt gtttggtata ttgtaggtga ttaataaaag ttagtttttt 13020
tttttgattt tttttaaata tataggatta tgttgagaaa ttgatataaa agtatatgtg 13080
aaggtttttt gaaaattata aagtttgata attatttaaa aaaaaattag ttttattgag 13140
ataatatata tattatataa tttatttatt taagttgtat aatgtaatgg atttttttat 13200
aatttaatag agttgtgtaa ttaatattat aatttatttt agaatatttt ttaaaagaag 13260
ttttatgttt tttaattata atttttttat ttttatttat tttggttttg gatattttga 13320
atttatttgt tgtttttatg gatttgttta ttttggatat tttatttaaa tggaaatata 13380
aaatatatgg ttttttgtga tgtttttttt ttatttagta ttgtttttag atttatttgt 13440
gttgtggtat gttttagaat tttgtttttt tttattgtta aagaatattt tattggtatt 13500
ttatgtattt atttgtttta tagattgtta gattgagaaa taattttatg gagattgagc 13560
gtaaatattt tttattttat tgaaaaagag attgtgttta agaggttatg tagtttagtt 13620
aagattattt agtttagagt ttagttttga gttttataat tttttacgtg gttttatttt 13680
attgtttttt ttagagagtt attggagata taatttaaag tgtatagttt attgataatg 13740
ggttaggaga attatttttg ttaatattag tttaatatta tttatttatt aaataatgta 13800
gattgttttt tgtgtgtagg gtattgtagg aggtgttgag gattttagag ggaataggat 13860
aagatttttg tttttttttt tagagtgtat atttttttga ggaggtagaa atagattagt 13920
agttttttag ataataaaat tattgtatgt tgtattaagt gttatgaaag aaataagggg 13980
tagaggagga ggaggatgtg ggggagggtg gtattggtag ggagaatggg gtggggatga 14040
aaggattttg tgcgtagagt gttatatatt ttggggaggg ttttttttag gatatttagg 14100
ttgaggtttg gaggagaagg attttgtgaa tatttggagg aagaatgttt tagagagaag 14160
gttatagtat gtgtaaagat tttaggaaaa tagggaaagt ttgaagttta ataggagtag 14220
aaagagcgag taagtggtgt gattgaaggt tcgagagagt agggtttagt ttatagtgga 14280
ggtttatagg tttgtttttt tacggttata tttttagagt taattatttt taggaggatt 14340
cgtgagaaag tgtattagtg gtttttagtt tttttttgtg aattaaataa attttagttg 14400
ttattggaag ttagaggaag ttttgagttt ttatagtaaa gatttttaag gaagggagaa 14460
ttgtatttta ggtaaaggtg atgaaattat gaattatttt gggggtgaat taggagataa 14520
ggaaggtaaa gagaaataag aaaatatagg tatattaata gagaaatggt taaataatta 14580
tggtatagtt atataatgga atataattta attagtaaga agaaagaagt ttattttttt 14640
tattttgatg tagaaagatt tataattagt tgttaagtaa aaaaaaaagt aaggtgtaga 14700
attgtgtgtt tagtatatta ttattagtat gagatagtat gttttttgta atatacgtat 14760
ataagttttt tagaaaggat atatatataa ggtgtttagg aggggttttt ttgtttttgt 14820
atttttttag gagatgtgta ttttagaacg aagtttttta ttattttttt tgttgtttgg 14880
gaaaatggaa gtatattttt tatttaaaga aattaaatta aaaatgaaaa taggttaggc 14940
gtagtggttt atatttgtaa ttttagtatt ttgggaggtt gaggtaggta gagtataagg 15000
ttaggaattc gagattagtt tggttaatat ggtgaaattt tatttttatt aaaaatatta 15060
aaattagtcg ggtatggtgg tgtatgtttg taattttagt tattggggag gatgaggtag 15120
gagaattgtt tgaattcggg aggtagagtt tgtagtgagt cgagatcgcg ttattgtatt 15180

ttagtttggg taatagaata agatttcgtt ttaaaagaaa aaaaaaaaaa gaaaagaata 15240
taaagaaaag gtaatgaggg gtcgagattt attaattaaa gttttgtgtg ttttagtttt 15300
ttttgttttt tggtttttgt taagatgaat tttagagatg tttttttatt tttttaggga 15360
attaattatt gggtagttat tggtttttgg tttagatttt atttttagtt gtaatgatgg 15420
ttgtgacggt ttagtgatta aataaattat ttcgaagagt ttagaattaa ttttaatttt 15480
taaatgtgaa ttattaaagt atttttttta agagggtatt ttagggtgaa taaataagtt 15540
aataattatt aaatggatat tggattttaa ttaagtgttt agtattttgg gagtgttaat 15600
tagtgtgaat gaggagagtg tgattttgta atttatagtt tttttttttt atttttaggt 15660
tttttaattt ggacgttgtt gatttttggg ttggaaaatt ttttgttgtt gggggttgtt 15720
ttgtgtattg taggatgtta gtagtatttt tggtttttat ttattagatg ttagtagtat 15780
tcgttttttt tagttgtgat aatagaaatt gtttttagag attgttaaat gttttttttg 15840
gagtaaaatt atttttagtt gagaattatt gtttttatta ttgtattttt aggtaacggt 15900
tttatttttt ttttattaga agttggtgat tttttttttg ttttattttt aggtgggttg 15960
ttatagaggt tgagggattg acggaaattt tattgtgagg gagtttatcg agttttatat 16020
gtggtaatta gttttaattt tttgggaatt ttttttgttt tagggaaaat agtaatttta 16080
gattttataa aataacgatt tgcggatagg aatttttttt atgattagaa ggtttagata 16140
attaaaaggt aattttgtgt aggttgataa tgaaagtaga atatatgtag gttggttaga 16200
ttttatttta tggtatttta agattgtagg gatgagggag ttcgggggag tgagtgttat 16260
ttttttttgt gattattttt ttataagtga tagaaagaaa ggagcgaggt atcgtgaagg 16320
tgtatttagg gttattttgt aattaatagg gtggataggt aggtgagtcg gtcgttgttt 16380
tttttttttt ttttaaggac gtaattttta atggatagtt ttttttggaa ggtaaagaaa 16440
aagggattgt atttttatgt tttgattaat ttgaggttta tttttgaggg tttcgtgaaa 16500
tgaatgagta gaatttttta tggttaattg ttttggttgt cgggttttat cggtaaaagc 16560
gtagtgttta tttatttttg ttcgtgttat ttttatttta gt 16602

<210> 86
<211> 16602
<212> DNA
<213> Artificial Sequence

<220>
<223> chemically treated genomic DNA (Homo sapiens)

<400> 86

attggaataa aaataatacg agtaaaagta aataaatatt acgtttttgt cgataggatt 60
cggtagttag gatagttggt tatggaaaat tttgtttatt tattttacgg agtttttagg 120
gatgagtttt agattaatta aaatatagaa atatagtttt tttttttttg ttttttaaag 180
aaagttgttt attgggaatt acgtttttgg gaaaggaagg agagataacg gtcggtttat 240
ttatttgttt attttgttgg ttataaaatg attttgagtg tatttttacg gtgtttcgtt 300
tttttttttt tgttatttgt aggaaaataa ttataggagg aaatggtatt tattttttcg 360
ggttttttta tttttgtaat tttgggatgt tatagagtag gatttggtta gtttgtatgt 420
gttttgtttt tattattaat ttgtataggg ttgtttttta attgtttggg ttttttagtt 480
atgggagaga tttttgttcg tagatcgtta ttttataaaa tttgagattg ttgttttttt 540
tgagatagaa gaggttttta gggagttagg attggttgtt atatatgaaa ttcggtgaat 600
ttttttataa tgaagttttc gttaattttt tagtttttat ggtaatttat ttggggtaga 660
ggtagagggg agattattag tttttgatgg ggggagaatg aagtcgttat ttggaaatgt 720
agtgatgaga atagtggttt ttaattgggg gtgattttgt tttaaagggg atatttggta 780
atttttgaag atagtttttg ttgttatagt tagggggggc gggtgttatt ggtatttagt 840
gggtagaggt tagagatgtt gttaatattt tataatgtat agggtagttt ttaatagtaa 900
agaatttttt agtttaaaag ttagtagcgt ttaggttgag aaatttggag atggagggaa 960
aggattatga gttatagagt tatatttttt ttatttatat tgattgatat ttttaaggtg 1020
ttggatattt aattggaatt taatgtttat ttgataatta ttaatttgtt tatttatttt 1080
agggtgtttt tttgagaaag gtgttttgat aatttatatt tgggaattgg aattaatttt 1140
agatttttcg ggataattta tttggttatt agatcgttat aattattatt ataattagga 1200
gtgaaattta gattaggaat tagtggttgt ttaatagtta gttttttagg aggatgaggg 1260
ggtatttttg aggtttattt tggtagaggt taaggggtag gagaggttgg aatatataga 1320
gttttggttg gtagatttcg attttttatt gttttttttt tgtgtttttt tttttttttt 1380
tttttttttt gagacggagt tttgttttgt tgtttaggtt gaagtgtagt ggcgcgattt 1440
cggtttattg taagttttgt ttttcgggtt taagtaattt ttttgtttta ttttttttag 1500
tagttgggat tataggtatg tattattatg ttcggttaat tttggtattt ttagtagaga 1560
tggggtttta ttatgttggt taggttggtt tcgaattttt gattttatgt tttgtttgtt 1620
ttagtttttt aaagtgttgg gattataggt gtgagttatt gcgtttggtt tgtttttatt 1680
tttaatttaa tttttttgaa taggaaatat atttttattt ttttaaatag tagaggagat 1740
agtgaaaagt ttcgttttgg agtgtatatt ttttgggggg atgtagaaat aaggaaattt 1800
tttttagata ttttgtgtgt gtattttttt tagagagttt gtgtgcgtat attgtaaaag 1860

atatattatt ttatattaat ggtaatgtat tgagtatata gttttgtatt ttgttttttt 1920
ttttgtttaa taattaattg tagatttttt tatattagga tggaaagagt aaattttttt 1980
ttttttgttg attgaattgt gttttattat gtagttatat tataattgtt taattatttt 2040
tttgttaata tgtttgtatt tttttatttt tttttgtttt ttttattttt tgatttattt 2100
ttaggatgat ttatagtttt attattttta tttaagatgt aatttttttt tttttggaag 2160
tttttgttat agagatttag gatttttttt agtttttagt ggtaattgga gtttatttaa 2220
tttataggaa ggaattggaa gttattgatg tattttttta cgggtttttt taggaatggt 2280
tagttttgag agtgtagtcg tgagggaata ggtttatggg tttttattgt aagttgggtt 2340
ttgttttttc gagtttttag ttatattatt tgttcgtttt ttttgttttt attaaatttt 2400
aaattttttt tgtttttttg ggatttttgt atatgttata gttttttttt tgggatattt 2460
tttttttaga tatttataag gttttttttt tttaggtttt agtttaaata ttttaaaaga 2520
ggtttttttt agagtgtgtg atattttgcg tatagggttt ttttattttt attttatttt 2580
ttttgttaat attatttttt tttatatttt tttttttttt tgttttttgt tttttttatg 2640
gtatttagta taatatgtaa tgattttatt atttagggag ttattgattt atttttgttt 2700
ttttaggaaa gtgtatattt tgagaggaag ggtagagatt ttgttttgtt ttttttgggg 2760
tttttagtat tttttatagt gttttgtata tagggggtag tttatattat ttggtgaatg 2820
aatggtgttg ggttaatatt ggtagagatg atttttttgg tttattatta ataagttgtg 2880
tattttgaat tatgttttta ataatttttt gaggggggta gtaagatggg attacgtgga 2940
gagttgtaag gtttaaggtt gggttttgag ttgagtgatt ttggttaggt tgtatagttt 3000
tttagatata gttttttttt tagtaaaatg aggggtattt gcgtttagtt tttataaagt 3060
tattttttag tttaatagtt tatggagtaa gtagatatat ggaatgttaa tagaatattt 3120
tttggtaata aaaaggaata gagttttgag gtatgttata atatagatga gtttggaaat 3180
agtgttgagt gggaagaaag tattataaag gattatgtgt tttatgtttt tatttaaatg 3240
aaatgtttag aataggtaaa tttataaaga tagtaagtag atttagggta tttagggtta 3300
gggtgggtgg agatggggga attatggtta aaaggtatgg ggtttttttt ggaaaatatt 3360
ttaaaatgga ttgtggtgtt ggttgtataa ttttgttaag ttgtaagaaa atttattgta 3420
ttgtataatt taaataggtg aattgtatgg tatgtgtgtt attttaataa agttggtttt 3480
tttttaaatg gttattagat tttatggttt ttagagaatt tttatatata tttttgtgtt 3540
aattttttag tataatttta tatgtttaag agagattaaa ggaaggggtt aatttttatt 3600
gattatttat aatgtgttag gtattatgtt agtttttatt ttgatataaa tagtataaaa 3660
ttaaagagtg tgggttttag aatataattg tatgaattta agttttgttt ggttgtttat 3720
tagttgtgtg attatcgggt aaagtatttg tttttgtttt tttatttata gtgggagttg 3780
ttgtaagggt taatgtatgg aaagtggtta gtattcgtat gattgttttt tttgtgtttt 3840
tgtggttttt ttttttcgaa gtgttttttg gaggaaggtg gtgtttgttt tgttttttgg 3900
tgaagagttg aagtttggtg agagtgtgtt ttacgtaatg ttatattttt aagaagggga 3960
aaaattatga ttagatttta ggtcgagtat tttaagtatt ttattttttt ttgtttttta 4020
tataattttt tgggttttaa ttatgggaat ggattgattt aaatttagcg atattttttt 4080
gtttggattt ttttgtgttt aagattatat atggataaga tggtgggtgg ttatttagga 4140
ttttatttgt agttatggaa tggagtgagg ttagttgttt gggatttttt ttaatttatc 4200
gtttatatat gtagatattt agttaaatat attataggtg cgatagaaat tttaaagggg 4260
aggttttttt aggtaattaa ggaagttttt tttgtttgtt tgtttttttt tttttttttt 4320
tgagatggag ttttattttg ttgtttaggt tggagtgttg tggtgtgatt ttagtttatt 4380
gtaattttta ttttttagtt taagcgattt ttttgtttta gttttttagg tagttgggat 4440
tataggtacg tattattatg tttggttagt tttttttttt ttttttttgt atttttagta 4500
gagatagggt tttattatgt tggttaggat ggttttaaat ttttgatttt aggcgattta 4560
tttttttcgg ttttttaaaa tgtcgggatt ataggtatga gttatcgtgt ttagttaatt 4620
aaagaagatt tttaaaaagt tttttatttt ttatgtattt ttgttttgtt gtttttaata 4680
gtatttattt gtttggttat ggggttgaat agggttggtt ttgttatttg tggagtttta 4740
ttatggttat gaagaggttt tatagaggta aataagagtt agaggtttgt ttagtatatg 4800
attttagttt ttagttatga gtttcgatat taggtttgtt gtgtcggtga gagttcgttt 4860
taaaagtttt ttttgtaaat attatttttt taggatgttt aagttttata gtttattttt 4920
attagatttt tttttggtta agttttttaa atatatattt ttttgtaagt agggttggta 4980
ggaaggagta atatttaggt tgagcggttc gtgtagttgg tttggtgttt agttttttag 5040
tgtgatttta ttttagtttt ttagatttat atttttttgt agttgttgtt gaggataggt 5100
aaggaaggag agagtatgag aaagtggggt tttggaaaga gagggagggg ttggttatat 5160
agtagtagtt tttttttcgg gtagtagttt agggttgggg gatagaaaat tatttagtta 5220
atatatattt attattaggt gttgggattt acgtttagcg ttagttatgg tttttgtatt 5280
tggggttttt gtatgattag ggatatagat aaatgaattt gggagtgata tagtaatata 5340
ataataaaag ttgtaattgt ttttgagtta aaagtaaata aaagtataaa atttgtaata 5400
tttttttttt ttttgtgtgt gtgtgtgtgt gtgtgtgtgt gtgtgtgtat gtagtttttt 5460
tttttaattt ttttttaaag aaaggtagaa tataatatat agtgaaaaaa attaaattgt 5520
aatgggagaa tatagtaaat ttttgatgaa aattatatta aaaaatataa tgtatttagg 5580
aggtcgaggt aggtagatta cgaggttaga agattaagat tagtttggtt aatagggtga 5640
aatttcgttt ttattaaaaa tataaaaaaa aaaaattagt tagatgtgtt ggtgggcgtt 5700
tgtaatttta gttattcggg aggttgaggc gagagaattg tttgaattcg ggagaaggag 5760
gttgtagtga gtcgagatcg tgttattgta ttttagtttg gcggtagagc gagattttat 5820

tttaagaaaa gaaaagaaaa agaatataat acgtgtgtag tttatgaata tatttatagt 5880
aaaatataaa attgtgtatt agatattgat tagatttata taagtagttg ttttgggaag 5940
gataagagga attaagattg ggagaggaat tgaggaaatg ttttttttat tttaatttta 6000
tttttttata aatagttttg aggtaagaat agttagttgg tgatattgga ggattttgag 6060
tgataggatt tttagtgttt gttggtgttt agtttatatt tagtgtatag ttttttattt 6120
tattatgtat gtatgaatta tgtatattgt tttgtgtatt ttataaaatt tttattaggg 6180
atttgttatg attttttttg attttttttt atatatttta ggatggtagt tgatgtgtta 6240
aagttttatt tatagttaat gggatattta gagttggtta agatttttat tttttgtttt 6300
tcgtgatttt tgagatgttt tatgatttag tttaggtttg agtttatgtt gtatttagat 6360
tagagtatag gtatttataa aaggtgggtt tttttggggg tagataggga tttaggagaa 6420
atggggaggt aggacgggtg gatgaggtat ttttaaagtt ttaatttttt tgtttatttt 6480
tttttttaat atttttcgat taggttagcg tatagagtaa atgaagagta agtatttttt 6540
tttattttat acggggattt aggagttgtt atttatgaat aggaaggtta gtgtttttta 6600
agtattttag ggatgaaggg gtaagtattt tttgaaaaat aagatacgtt tatggtttgt 6660
agggtatttt attttttttt tttgaaatgg ggatattaat aattaattta gttgaagaaa 6720
tggaaagtaa tttttaaaaa ggtattttaa cgatagaaat tttttggaga aatttaaatt 6780
tagataaagt attgtaaaaa tgtacgggaa gagggttgga agtgtttatt tggtggattt 6840
gtgattgtat gtgttcgtaa tagaatttag agttttgttt ttttattaag aatgagttaa 6900
gaagtgttgg tttgaaaata gtttatttgt tagaattata tgttaatgaa attgaaatgt 6960
gtaaatttat ttttgtgggt tatggttttt tttgttttat gtttattatt taaattgttt 7020
tacgagtttt tgtatatttg ggaaatttat ataaagggga gtttagagtt tttgtatggt 7080
tgtgttagtt cgtaatttat tgtttatatt ttttgtttga ggaattaagt ttgtagtttt 7140
ggtttttttt tttgtttttt aaagtttttt attaggtttt agtagtagtt tgtttatagt 7200
aagaatagta attttttatt ttttaaaggt agaaaaaagg aaaaagggtt atgattttgt 7260
tagtgttaag agaagttaat tttgaggttt tgtattttgt ttttgagttt taagttattt 7320
tagtattttt ttgttttatt ttaggtaaat gtaacgataa gattataatt ggtgtattta 7380
atgaggaaaa tgtttggaga gagaggtttt gtttttgtag tatgtttgtt tgtggttttt 7440
aagtttgttt ttcgtttttg ttttttaaag agaggttaag agagattatg tgtttggttt 7500
attttgggtt ggagattttt cgttttttgt cgtgtttatt taaagtgagg ttagttttat 7560
ttagaaattg gtgcggttgt tttatgttag tgatatttat aattaaaatg tagttataag 7620
tcgggattgg taaaatatta gagaaagata gagaagggga aaaatcgaat ttgggggaaa 7680
ataattttag tggtaaaaaa tatatatata tgtatatata tatatataaa tatatatata 7740
tatttatata tatatatata tatatatata tatatatata tatatatata tatatatatg 7800
taaatatata tgaaaaaaaa aattttattt taaaaggtgt ttgttttttt aaggtttgaa 7860
aaagttgttt ggaaaatttt gatatagttt agagattgaa tgatatatat atatatatat 7920
atatatatat attatatata tatagtatat atatatatat atattatata tatatagtat 7980
atgtgatata ttatgtgtgt gtgtgtgtgt attttaaagt ttatgtataa aaatttttta 8040
tgtaattaga tgagggttgt ggggaggagg gtaaggagtt ttttttatta tatatgatcg 8100
aggtgagaga attttgtcgc ggagtatatt tttgtttgga gatttttttt tttttttgat 8160
ttttggtagt ggggtttagt tttttttatc gtggttattt gaaaaattgt tttgatttcg 8220
ggtttatgtt ggtggggaat aaagtgtttt aattgttagg tgttgagtga ttttaattag 8280
aaaatagaag tttggatttt tttaaagtaa tagggtagga ggtattttat gaagaggaat 8340
ttgtttttta gaaagggttg taaggtagga ttgttttgat tagtgaattt tgttagcgtt 8400
tttaggatgt ggtttgattt ataaatattt gattggtacg tattttgttt aagaatattt 8460
ttatttgtgt agtgagtggt gtcggtaatt ttgtttttta cgtagttttt tgcgtgtttg 8520
gggagttgag gagttgtggg tgtggtaggt agttgaaggt ttggaatgtt tgtgaggaaa 8580
ttatttttta attattgttg tagttattaa ttttattgag ggataagtta tggttttttt 8640
ttatttgaga ggaaattttt tagaaagatt agagttaatt atagagtaga tttgttttgt 8700
tagaggagtg agatggggcg gggtttgttg tatttgtgtt tgggtatatt atgtttttgt 8760
gttaggtttt aggggtttta atttagatag ttttagagaa gttgggataa agagttattt 8820
ttagttttgc gagtaagtat agtatttatt taatttaata gatttgaaag gttagttggt 8880
taggtgagga aggtttagga atttcggaag aatgatgatt aatattattt tttttgagta 8940
tttatgtgtt tagtagtgtg ttaaatattt ttataaatat tattatatta aaatttttac 9000
gtttttatga gtgggtgttg ttgttatttt tgtttttaaa ataaggaaat tgaggtatat 9060
tgagaagtaa ttgttttagg atgatataat tggtgaatag tagtgttggg attcgattta 9120
tatttatttg atttataata tagatgtgtt tattgaatta gtttgtgggg cggaattatt 9180
gttatgatgt tggtgtcgga tggttgtggg atatagaagg agttttaatt ttggaagtag 9240
aagatgtggt tggtgttaag tttttttagt taggagttgg ttacgtttgt taattgtagt 9300
gtttttagag tgataataat tagtaatttt ttaaggttgt ggttaggtag aagtgaagga 9360
gtttagtata atgtttagta tatattaggg tttatagatg tttattgaat atattgttga 9420
ttttgaattt agttgttatt aatataattt agttatttat taaattaatg tttattgggt 9480
attttattaa gtggtaggta atgtgttaga tataaaattt gtagtgttga gtaatgagag 9540
ataaagtttt tgttttttag gagtttagta ggagggaaaa ttattaaata atcgtataaa 9600
taaatggaga attataattt tgaggatagt tgtatggtac gtgagatatt ttttaatgtt 9660
tgtgtttaaa atggaattgg ttattttttt ttggatttga ttttttttta ggattttttt 9720
tttttatggt gatattattt atgtgttttt ttgagttgaa gtttagggga taatatgaat 9780

ttttttataa attgtgttta ttagttttgg aatttatgta gtattttttg gttgattttg 9840
tttggttttt ttagtatggt agcgtaatta atgtttatag tttatattgg taattgtttg 9900
tagtagcgat ttaaggaatg gaaatgggat aaattaaaat tatttttttt gtaggttaga 9960
agtttattag tagagatatt ttatttaggt ttttattaga atagtttata tataagtatt 10020
tgttttaggg ttatattatt ataaggaagg ttaggtattt ggaaatttat ttttttagag 10080
gtaggaagaa gatatatttg tttaggaatg attaatgatt tggggataaa gagaagtcgt 10140
gaaaggagtt atttttgttt tgtagagttt aaaggtatac ggttttattt tgtgtgattt 10200
gtttaaattt ttcgattggt ttttagaaac gttttttttg aaggaagttt taacgtgtga 10260
ttttgttatt ttagttttta attatgttta aattagtgat taaggaatta aaaatatttt 10320
tttgttaggt cgtgtggatt atatttagtt agggagtatt cgttttatgt ttggtcgagg 10380
taattttgtt tttgaataat ttttgaaatt tagaagggtt ttggtagtat tattattggg 10440
tagaagaggg taatagaatt atatttaggg agtatattcg tgtttaggat tttttttatt 10500
tgtagaattt tagtaaatag aagtttcgtt tttgtgaggt atattgggta atgttgttat 10560
ttttatttta taggtgagga aattgagttt tagcgagatt aaatgatttt tttgaaaatt 10620
atttgggaat attagagata ttttaatttt tagttaggaa aggattggaa gcgttttagg 10680
gttggggggt ttgaagtttt tttcgtagag tttgtaaata gaaagaatgt ataatggtaa 10740
gaacgttaat tgtttagggt tgttttaggt agaaaggggt agagtaggtt tgaattcgag 10800
tttgttgatt ttttagtgga atatttagtt ttagtattga taggtatacg gatgggtttt 10860
tggttattta ttttatttat tttgattttt gtttttttgg tttttgtttt tagttttaga 10920
gtaagtaaat aataggttta tataataatt tcgagtgttg gtagagagat gttgaattta 10980
agtttgtatt tagttttttg atttttttgg gtggtagtat tgttaaaaag aggagtttgg 11040
aaggtttttt aatacgattt tgtgttatta tatgttaggt tgattatatg gaaagtttga 11100
tgatgataat tgtaatatga attaatacgt gttgtgttgt ttttttgtgt tagttattgt 11160
gttaagaatt tttattatat tattttattt aattttttta agaaatttgt gaggtaagga 11220
atattagtat tttttttgta taaatgagaa aagtgagggt gaaaggtgta gttgtttaag 11280
tttatttgga aaataatttg gtattttttg aatgtagaaa tttttatttg tatttttagg 11340
gttacgttgt gttgtttttt aggggttttt gatattttaa gttaggcggt taaatttatt 11400
gtggatttta gaagttgtgt tagtgttata tatttagagt gtagtttgtt gtgttaatga 11460
tggaatggta atagttaagt tattttttat tgttatagaa attttagtat tggagttttt 11520
ttttatttta gaagttaagg tttttagaag agttagttta attttaagga atatattatg 11580
aagtttgttt ttttttttgg gttttgagga tcggagagta ttttttattt ttttagaaaa 11640
ttggttaaga cgtattagga atatttaaaa tttagaggaa ttaaagtttg ttcgtgttgt 11700
gtggggtaga ttacgtgagt tgtgtattat gtttgatata atggattata atttaagttt 11760
gttaatttgt agtgaatatt gtttatgtat gttatttaat aaattagagt tttttttttt 11820
tgttttattt tgatatttgt tttttattga gtataagata tttagtaagt ttatttgaag 11880
ataagttatt aaaataaagt ttaaaagagt taattttgta gttgtaagtt ttagttttag 11940
tttttttgtt ttttattttt agggtttgtg tagtgggtgg ggaggaagga ataacgttat 12000
ttcgtgtaaa cgttagcgtt tatttttatt atttgtgtat ttgagggtat agcgtgagta 12060
ttagcgaagt ggttatttgt ttttagagtt tgtaattttt ggtgtatggt ttatgaataa 12120
ttaagattat ttgatgtata aattattatg ttaaagtgag tgttttgtat tgtattttga 12180
agttagtttt atagaaattt tttttttgtt tgtttttttt tatgtttttt gatttttttt 12240
tttttgattt gtggtaagtt tgatttttga gattgttgat tttttaagta gggattggat 12300
ttgattttag gtttttttgt aatttttgat gttgtagtgg gatttaggga attttagata 12360
atattacgat aggatttgga tatttaatat taaatatata tattaatgtt agaagtatta 12420
aagtagaagg aagagatttt tttatattta gatagttaag aagatttttt tttttagagt 12480
tatattttta tttgttaata tttttgagaa tcgggttgtt aggaagtttt ggaagagata 12540
tttagtattt ggcgttagtc gtttttattt tttagttttt ataaggtttg gagaatgatt 12600
ttattattgt tggttattat agttatattt gcgtgatacg tagagggaaa gttgtataat 12660
ttatgatgtg taagattacg attatgaatt gggattttat aaattaagtt tagtggaaat 12720
ataattagga gagttagttg atttgaaacg gagtttatat ttttgtgtag gatttattat 12780
tatgtttgtt tggtttattt tattatgaag cgtgtaggtt tttgattagt tttcggggtt 12840
agatttttta ttagttatag aaatttggga aaatatatag taattgagta tcggtagttc 12900
gttttgttgg gttagtagag agtgttttaa aaaatttgaa ataggatacg tttgggtaag 12960
tggtggtttt tataagtttt tattttttaa atttagatat tttacgtatt tatgtgattt 13020
tatttttgaa tgtgttatgt ttgaatagaa ttttggagaa gtaagtattt gtatttaaat 13080
gtgtattttt tttttatttt gattatagaa gatgttagaa agggaagggg ggtgttatag 13140
agatgggttt attgtgttgg tcgtttttgt tttttgtttt atttattgtg ttattcggaa 13200
ggatttttag gaagtggttt tcgttgttat agtagaggag tttgggtttt ttgtaatttt 13260
ttggaggtag attaaatttt tcggttaggg ttgtgaaggt ggtgattttt tttttagtta 13320
tggtttagta gttgttgttt gtggcgtttt taagattgta agaaattgaa taaatttgta 13380
gtcggttttt ttagtaaagg tataaaatgt attttgaaga gaggaaggat agttttaggg 13440
tatagggttt tcgggtagtg agtaagtata gtttgtttag gtgatgttcg tagttcgcgg 13500
gtttgtgagg tggttggtgg ttaaggaagg atggttttag gtttgggagg ggtttttagg 13560
gtattttttt ttttttttaa ttggatatag ttttggatta aattgtattt tttagaattt 13620
taaatttaat tatttgatta tggggaaaag aattatgttt tggttttttt ttttggttta 13680
tatatatttt taaattaaat attaaagttg tattaaaata atatttaggg tttggttgaa 13740

atttataaaa gttgagaggt taggattaag gttttgtttt ttatttttac gttgcgttgg 13800
tgaggtatcg cgaagtagat agtatagatg gtttggacgt tcgggatttg tagttaaagg 13860
tataggaggt tttttaggga tataataggg aaagtaaagg gttagtatgt taagggaagt 13920
ttttggattt gttgggggaa atagaattgg aattagggaa aaaggtatag agtgggagta 13980
agtggggttt ttaaagggta aatattatat taaaggaaat gttatgagat agattattat 14040
gaaggttatg tttggttttt gtttattttg tagtttttta tatagtttta tgtggtatag 14100
aaaaggtggg tttgtttttt gtacgtaggt tgtttttatt ttagttattt tttaatgtga 14160
gatagaatat agaaattatt atattatata taatatattt ttaatattat atgtagttat 14220
tatatataaa tttatattat taatgtgaaa atgtattatt tatattttaa aggattaaaa 14280
aataaaatta atgagtatta gaattggtta aaatatatat ttgttttatt atttaggaga 14340
aggaagtgtt ggtgatttta aaaattgaaa agaaagaggt taaaaagaga ttaggaaatt 14400
aacgtgtgaa ttgtattttt tttttttttt tttgagacgg agtttttgta gtttaggatg 14460
gagtgtagta gtacgatttt agtttattgt aattttggtt tttcgggttt tagtttaagt 14520
aatttttttg ttttagtttt ttaagtagtt gagattatag gaatgcgtta ttatattagg 14580
ttaattttta tatttttagt agagatgggg ttttattatg ttggttaggt tggttttgaa 14640
tttatgattt cgtgattcgt ttgtttcggt tttttaaagt gttgggatta taggcgtaag 14700
ttattacgtt cggtttggga gttgtatttt gtaagtgtga gtagtgaatg tgattattaa 14760
tatatattga ttattaagtg gttttagaat aaaaattatt atttttatta ttaacgtatt 14820
ttgggttata ggttatttta agtaatttta ttataatttt ttgaagtgga tattaatatt 14880
attattattt ttaataaaat taaagtttat agatgtttag tgatttgttt aagggtaata 14940
gttagtaagc gatggagttg aatcgtaaaa ggagtttggg aatagaaagt tttaatttgt 15000
ttttgattat tatgaaaagg tttagtgatt ttatttatat tagttttttt atgaggagtg 15060
gggtttttat ttattgtttg atatgtatat ttgttattat taatttgtta tttatatatt 15120
tatttgtgat ttaaaggttg gttagttttt attaatagtt tttttttttg tttttatgtt 15180
atttgtgaaa taggatttta tgtattttta ttttttttag gagaagttta ttttagtagt 15240
ttcgtgagta ttatggaaga ttagtagtta ttttgttttt tttgtattaa ttatttatta 15300
gtgtttgtag tagttgtgcg gagttgtttt tataggagat ggttttatta taaattaaga 15360
tattaattat aataatttgt ttttatgatt ttaaaatgag ggttcgtgtt ggaagttttt 15420
atttgttttt tttgtttttg gtattttaat gtttttggta tatttttttt attttgttat 15480
tatatgggtt agttattgtt tttggtttta attagtgttg gaggtgtttt taagaatttt 15540
aaagagaatt tcgtaaatat tatataaatt atatttaaag ttagattatt ttaaatgttt 15600
tttttatttt atatggtgaa atttcgatta atttttttta tataatatag taatagatag 15660
aaaataaata taatattata tatatgtaat tttatttatg ttaattttat agagaggatt 15720
gtagagttta ttttgtatag tataattttt ttttattttt attgtttttt ttaaatttta 15780
tttttttatt tataagtttt taagtttttt tgtgtttatt tttggttttt ttattttttg 15840
tttttttgtt tttttttgtt tttttattta tttttttttt tatttagttt tttattttaa 15900
ttttttattt tatttttttt tggatttatt tttaatgtaa agtaatttag ttttttttag 15960
tgtggatatt tttatttttt atttttttat tagtatttaa ttattattag attttttttt 16020
tttaaaatat aattaagagg taatgatttt ttttttttat ttattttttt taagtatttt 16080
ttaagttatg tattttattt ggtataggag ataataatta tatattagtg gttgttattt 16140
aattgtataa atagttattt gtattgttaa ttttaggtgg gtcgaatttt gttttttttt 16200
gtttttggat tttttttagt gtaggtttta tttgtttttg attgtggggt aattttattt 16260
taagaatggt tttttaggtt aggtgtggtg gtttatgttt gtaattttag tattttggga 16320
ggtcgaggtg ggtggattat ttgaggttaa gcgtttaaga ttagtttggt taatatggta 16380
aaaatttatt tttattaaaa atataaaaat tagttaggtt tggtggtgta tgtttgtagt 16440
tttagttatt cgggaggttg aggtaggaga atggtatgaa tttgggagac gatagttgta 16500
gtgagttaag attgcgtcgt tgtattttag tttgggcgat agagcgagat tttgttttaa 16560
aaaaaaaaag aatggttttt tttttagatt ttagattatg tg 16602

<210> 87
<211> 6325
<212> DNA
<213> Artificial Sequence

<220>
<223> chemically treated genomic DNA (Homo sapiens)

<400> 87

aaaaatttgt ttaagtaatt tatgttttga aatattataa tattttatgg aaaattaata 60
ttagaacgta gaaattcgga ttaaattata aatttatttt tttagaaagt tttattagtt 120
ttttagaggt gaatttataa atttagattt tattaaaaaa ttggggtatt ttgaaaatat 180
ggggtattta ttattttttg attgtgttta tttcggaagt attgataaaa tatttttttt 240
tgttaagtat atattaatat ttattttagt taatttagtt ggtatttaat aatttaatat 300
tattttatta tttttttttg gttaatttat tgtagtaaag atataattgt ttttttataa 360
ttaaattttt aatttttttt attattttta ttttgttatt ttagatatat tagaatgatg 420

aatggttaat ggtaagaaag ggagtaggga ggaagaaaaa gaatataaat aggataggtg 480
ttgtaaaaat tagggtaata gtttaaggtt tattatagga gttttaaatt ttggtttatt 540
atggagtgat ttgggaatta gtttatttat ttataaaata tatgtgttgg atttagttat 600
agttaagatt tttattggtt ttgaagtaat gtatattatt atttgtggga taatattttt 660
tttttgttta tttgaagttt ttggtttaaa aataataaga tattattttt ttatttattt 720
aaatgtgagt gattattagt attattttta gttgtagttt gggaagtgta taagttttaa 780
tgatgattta aaattggtat gttttgtgtt taaaagggaa aaatgggttg tgatatatat 840
ttataaggtt tgtattatag ggtgaaaaaa tgaggtttgt atatgtttaa atttttgaat 900
agcgtggttg atttttgatt ttatttagta ttatttgaag tagtttattg acgttgttaa 960
taatattata tataataggg tatatatttt gtaattgaat tataggtagt gttgagtaat 1020
ataaagaatt taggtaatat atatgtaaaa tgtaaattgt ttaaatataa tagttattga 1080
aggatttgtt agtatatata ttgattgttt ttttgaggtt taagagttag gttttaggat 1140
tttttaatgg ttgttgttat ggttttgttt taaaaagttt taattatttt ttaagatatt 1200
atacgtaata aaaggttgta tttatttttt ttaatataga gttagtttga tagtagtaga 1260
tatgtttaag ggtatagaaa taaagtatgt tttttagaag ttagataaaa gagaaaaata 1320
gtttttgaga attagtaggt attattttaa tttgaagttt gtattgtttt ttttaatttt 1380
agtaaagaaa taattaataa aggaatttat taagatttga ttattgtagt taaaaaagga 1440
aggtagaatt tttagttttt gaagtaattg agaatgattt aggttttatt atagagatat 1500
aatttatatt tatatattta tgttagtgat tttaaattat tgggagagat tgaatttcgt 1560
taaatagtgt attttttttg tggaattttt tttaggtttt ttcgggttaa tttaagaggt 1620
gatttaataa tgtaaagtag tcgggtattt agagatttaa ttgtgttgta aaagaaaggt 1680
tgtatagaaa agaaaaagaa gaatgagata taatttaaaa agttatgtta gttttttttt 1740
tgggttgttt tgtaaatgtt tttttgttat tacgttgtat agaagagtag cgaaggggtt 1800
ttagaatttt taaatattgt atttgttgta attagatttt aatatgaaag tttaaagata 1860
tttttttttt tattttagat agaaaaaata aagaaatttt tagttaaaag atttgaatgg 1920
ttttttttat tggaatataa agttggtaga tgtgtgtatg agagagagag ggagaaagag 1980
agattagttt tggattggag gggtatatgt ttagggtgag tttaataaaa atttttaaat 2040
tttaaagtgg tattaatagt gttggttttt attgtataga aattttaaaa taataatttt 2100
ttgtgttttg taatttttta tagtagaatt tttattgtat ggttttatta aatataattt 2160
ttaaaaaatg tagttttaat tagttgtaaa tttaaatatt tggatttttt tggattattg 2220
ggttgaaatt taaatttatg ggaaatatta tattgttttt taggtatttg tttttttagg 2280
tataaaattg tgaattaaaa tattgaaatt cgtattgttg gtttattttt tagtatttta 2340
aagaaaaaga ttcgtttgat gtgtgttttt tatttgttta attttttaat taatatttat 2400
agaaattaga gttaattata ttaaatttta aaaatttatt gtaatgttta ttatattgtt 2460
gaattttttt aaataagtat tgtatgagtt ataattaatg taattaaaat tttgatttta 2520
gtattaaatg attttatttt aaaggatttg aatatataga aaaatataag aatatttatg 2580
taaatttatt ttttaagtta ttaaatattt aattgagtaa ttttttttaa tgtttatttt 2640
taacgttaat tttttttttt ttgtattttt tttggtttat aattaatatg gaggttaaac 2700
gtatattata gggattttag tttgtgtgaa tatagatttt tttaattagt tttttttaaa 2760
ttatagttgt tgttgttaaa tttttagttt attttgaagt gtttttttgt tttatattgg 2820
tagtttaatt ttaggttgaa atatatgaaa ttattatttt tgtaggtata aaattttaaa 2880
atgttggtaa ttttgtgggt tttaataaaa tataaatgtt taattgtttt atgtgatata 2940
gatagtttat tttagtatat gtttaggtat tttttttagt taaacgtttt ttagtttaga 3000
gttattttta aagaagtggt tttttaggag ttgtttttta tttagattgg aatggattat 3060
tatagtattt ttttttttgt gttttttaat ttttggtaaa ggttttttag ttttggattt 3120
agttttgtta atatttgggt tgtgagttag tttttgcgag taaggaggga gttggggtgg 3180
tttgaaagag tgtgaaaagg agaggaggat tagtagtgta gtgatattgt aaaatagagt 3240
tttttaaata ttaaatgtga agatagaaaa aaaaaaaatt ttttttttat gataaagtgt 3300
aaaggaaatt gtataaaaaa gaaaatagat agtagggtat ttagttaaat tatatggtaa 3360
aatttttgcg atcgttataa agtgcgtttg ttttgaagtg tacgtatgat ttagtgaagt 3420
gtttatgtta gtaattagaa atcggttatt tgggtttttg attttatttg cgtcgagaga 3480
ggtaattttt aagggtgacg ataaattttt ttgagtttaa aaggtagaga gggttaaagt 3540
gtgaattttt tattttagta acggaagcga gaaattagtg atttattaaa gaggagattt 3600
ttttggaagt tggtagtaga ggaggaaagg atattaatta tgtttttttt ttttgtgttc 3660
gttttgatta gtttataatt tgttgttttg gacggtttaa aatttttagt attttttgtt 3720
tttttttagt tttttttttt tttgggagtg agaagggggg aaaagttagt taagtattag 3780
gttttaggtg gtttttaagt attttattgt taaaagaaat gggggtgggg ggaggagatt 3840
ttacgttgga aagtaagtga gaaaggggtt ttttagaagg gattggttta ggttggaaga 3900
tagtaaaatt cggaaagcgg gaagatatcg ttagcgtcgt ggttcgcgtt gtaagtatag 3960
gacgttttag tagcgaatta gatagagtta gaggcgtcgg cggaggttgc ggtcgtaata 4020
gtcgcggcga ggaagggatt tggtagtttt cgagttcggt gtaaaggtat cgcgtttttt 4080
tttcgttttt ttgttttttt attttttttt ttgttttttt tatttttttt ttttttggcg 4140
tttttagtag tttagggttt tagatgtttt atcgtcggtt gatttttttt tttttttttt 4200
ttatttttgt aaatgaggtt tgattagtag aggtagagtt tatttttggt ttagaattat 4260
tgatatttag atttaggttt ttaatttgtt tataagcgga tttttttaaag gaagggggtg 4320
ggagataggg ttatataaaa tattagtcgt cgttttttaa aataaaaagt gattttcggt 4380

agtttttaaa tcggtaggta ttattaaaaa tagaaaggaa gagagggaag aaaataacgg 4440
cgattgggta gttgttttta tttttgataa ttttaaaggg atatatttgt agaagtggtt 4500
cgtaggttgg ggtttcgtag agagagatta gaaggtgtta atcgtagagg ggtgtagata 4560
tttttttttt atttttattt ttattttttt tgggttttgt ttatcgtgtt gttatttgtt 4620
ttttagattt ttttggtatt taatatggtg aagaaaggag taaagaagag aataaagtaa 4680
tttttggggg agcgaagagc gttggtgatt aatattatta acgttattat tagtttttgt 4740
tgttgcggtt atttacgttt attatttatc gggaggtttt agaggcgtag gtagcggatt 4800
cgagaaagga gcgaggggag ttagtcggtt ttttcgagga gttatggatg ttggtgtatt 4860
tatttttggt tagattcgcg tttagaggga gttaattagt agttattatt tcgagttttt 4920
tttttgtttt gtatcgggtt ttattttttt agtatgtttt ttttgatgag ataattttta 4980
gtgtcgagag ttttagtata atgtggaaat ggatattgat atattgtgtt ttagtttttt 5040
tttatttgtt cggttgttgt tgttgttgtt ttttgttgtt gtttttggtg tttttcgttt 5100
ttgttatttg ttaagttttt ggttaggata tggtgttatt agaggttatt aatttttttt 5160
tttttttttt tttttttttt tttagcgcgg gaaggtatgt gcggagttat aattattttt 5220
aaggagatgt tcgttggaga aagttatttt tttttattaa gtattttttt aagattgaga 5280
agaacggaaa ggttagcggg attaagaagg agaattgttc gtatagtaag taataaatgt 5340
atgttttttt ttttttaatt atttattttt agtttatacg ggggggaaaa atttgtttta 5400
gatgtggtta gttaaatatt tacgttttta atttttgtaa aatgattaag tggaatattt 5460
ttatattaat ttatttttta attttcgtgt atatattgtg ttgtcgtatt ttttttttta 5520
agcgcgtttt ttttatattt tttatttttt gtcgttttat ttattttttt tttttagttt 5580
gttattagtc ggattacgta gggcgtttaa gagagttgtt gtagtgtaaa gatgtttttt 5640
ttttttttta tcgttttttt tattttattt aaagagtaat tttttttttt ttttggaaat 5700
ggtttaagta aatatatttt gttttttttt tttggtaatt ttgttaagag ttttgggata 5760
ggttttttaa gttgaaaata attattcgtt gttaattata ggggttttgt gggaatttta 5820
tttaagaaag aaaagttaat tttatttttt ttttattttt atttttttat tttattattt 5880
ttagatatga gcgaaaagta tttttttggt tttgattttt gttttgtttt gttttgtttt 5940
gggtttttta tgttagtttg agtgattgta gtttatgaag ttagtttttt aggtattatc 6000
gttgttattt gttttttgtt tgtttttttt tttggtaata tgatgtttat gtttgtattg 6060
ttaatagcgt atgtattgtt ttgtggtgtt ttgttgtatt gttttttttg aaatttttta 6120
tattgtagag ttatattatt atagtattgt aaatatttgg gattggattt atatagattt 6180
tatataaatt ttattgttat ttttttattt ttaaagtaaa agttataagt ttgtttttat 6240
ggttatagtt tattttatag tgtgatattt tttttttagc gtaaattatg tttgggattt 6300
ttatattttg gttttgtttt ttttt 6325

<210> 88
<211> 6325
<212> DNA
<213> Artificial Sequence

<220>
<223> chemically treated genomic DNA (Homo sapiens)

<400> 88

gggaaaaggt aagattaaaa tgtaggattt ttaggtataa tttacgttga ggagaaaatg 60
ttatattgtg ggataaatta tagttatgga aatagattta tgatttttat tttgaaaata 120
aaagaatgat aatgaaattt gtgtgaggtt tgtgtgaatt tagttttaga tgtttataat 180
gttgtagtag tatggttttg taatatgaga ggttttagaa gaaatagtat aataggatat 240
tataaagtag tgtatacgtt attgataatg tagatatgag tattatgtta ttaagaggga 300
aggtaggtag aagataaata gtagcggtgg tgtttgaaaa gttaatttta tggattgtaa 360
ttatttaaat taatataagg gatttaaaat aaaataaaat aaaataaaaa ttaaaattag 420
aaaaatgttt ttcgtttata tttagaggta gtggggtaaa gggatggggg tggggagaga 480
gtggaattga tttttttttt ttaaataaag tttttataaa gtttttataa ttgatagcgg 540
ataattattt ttagtttgag gagtttgttt taaaattttt aataagattg ttagggaaaa 600
gaaataaaat gtgtttattt gagttatttt tagaaggaag gaaaaattgt tttttggatg 660
aggtgggaaa ggcggtggag agagaaaggg gtatttttgt attgtaataa tttttttgag 720
cgttttgcgt ggttcggtta gtgataggtt ggagggaagg ggtgggtaga gcggtaaggg 780
gtgggggatg tgaagggagc gcgtttaggg gaggggatgc ggtagtataa tgtatatacg 840
ggggttgggg ggtgaattag tgtgaaagta ttttatttaa ttattttata ggggttgggg 900
acgtaaatat ttagttggtt atatttggaa tagatttttt tttttcgtgt gggttggggg 960
tggataattg gaaggagggg agtatgtatt tgttatttat tgtacgggta gttttttttt 1020
ttggtttcgt tgattttttc gtttttttta attttgagaa agtatttggt gaaagagaat 1080
agtttttttt agcggatatt tttttgaagg tgattgtagt ttcgtatatg ttttttcgcg 1140
ttggaaggag aggagaagga ggaggaagaa gagttggtgg tttttggtga tattatgttt 1200
tgattaaggg tttggtaggt gatagggacg gaagatatta agaatagtaa taaaaagtag 1260
tagtagtagt agtcgggtag gtggggaaag gttgaggtat aatgtgttag tatttatttt 1320

tatattgtat tgaaattttc ggtattggaa attgttttat tagaaggaat atattggaag 1380
ggtaagattc gatgtaaggt aaggagagag ttcgaggtgg tggttgttgg ttagtttttt 1440
ttgggcgcgg atttggttag aagtgaatgt attaatattt ataatttttc ggaaaagtcg 1500
gttgattttt ttcgtttttt tttcggattc gttgtttacg tttttggagt ttttcggtaa 1560
atggtggacg tgggtggtcg tagtagtagg agttggtggt ggcgttggtg gtgttggtta 1620
ttagcgtttt tcgttttttt aggagttatt ttgttttttt ttttattttt tttttattta 1680
tgttagatgt taaaaaggtt tgaaaaatag atgatagtac ggtgaataaa atttaaggga 1740
ggtggggtgg ggaatagggg ggagatattt gtattttttt gcggttggta ttttttggtt 1800
tttttttgcg gagttttagt ttgcgagtta tttttataag tatatttttt tggagttgtt 1860
agaagtggag gtagttgttt agtcgtcgtt gttttttttt tttttttttt ttttgttttt 1920
agtggtgttt gtcgatttga aggttgtcga aagttatttt ttgttttggg ggacggcggt 1980
tggtgttttg tatggtttta ttttttattt tttttttttg gaaagttcgt ttgtaaatag 2040
gttgaagttt ggagtttaaa tgttagtgat tttaagttag aggtgggttt tgtttttgtt 2100
ggttaaattt tatttgtagg ggtggagaag aggggggagg gggttaatcg gcggtgggat 2160
atttgaaatt ttaggttgtt gggaacgtta gggagaggga gggtgaaagg ggtagagaag 2220
ggggtggggg agtaggggag cgaggagaga gcgcgatgtt tttgtatcga gttcgggggt 2280
tgttaggttt ttttttcgtc gcggttgttg cggtcgtagt tttcgtcggc gtttttagtt 2340
ttgtttaatt cgttgttaaa gcgttttatg tttgtagcgc gggttacggc gttgacggta 2400
ttttttcgtt tttcgaattt tgttgttttt tagtttgaat taattttttt tggggagttt 2460
tttttttatt tgttttttag cgtaggattt ttttttttta tttttatttt ttttggtagt 2520
gaagtatttg agagttattt ggagtttggt gtttggttga tttttttttt ttttttattt 2580
ttagaagagg agagggttgg gagaaagtag aagatgttga ggattttaaa tcgtttaaag 2640
tagtaggtta taaattggtt agaacgagta tagaaaagga agatataatt agtgtttttt 2700
ttttttttgt tgttagtttt taaggaggtt ttttttttaa tgaattattg atttttcgtt 2760
ttcgttgttg aagtaaaaag tttatatttt ggtttttttt gttttttaag tttagggaga 2820
tttatcgtta tttttaaaag ttgttttttt cggcgtaaat gaaattaggg atttaaataa 2880
tcgattttta attgttaata tgagtatttt attgaattat gcgtgtattt taaagtaaac 2940
gtattttatg gcgatcgtaa agattttatt atataatttg gttgaatatt ttgttgtttg 3000
tttttttttt tatgtaattt tttttgtatt ttgttataaa ggggaggttt tttttttttt 3060
tatttttata tttaatgttt aaagggtttt gttttataat attattgtat tattagtttt 3120
tttttttttt ttatattttt ttaggttatt ttaatttttt ttttgttcgt agaaattagt 3180
ttatagttta ggtattgata gaattaagtt taaggttaag aaatttttgt taagagttaa 3240
gaggtataaa aaaggaaata ttgtgataat ttattttaat ttgaataaga aatagttttt 3300
agaggattat ttttttaaga atgattttaa attagggaac gtttaattag aaggaatgtt 3360
taaatatgta ttggaatgaa ttatttatgt tatataggat agttagatat ttgtgttttg 3420
ttgaagttta taaaattgtt aatattttaa ggttttgtat ttataaaagt ggtaatttta 3480
tatgttttaa tttaaaatta ggttgttaat gtaagataga gaaatatttt aaggtgaatt 3540
gggagtttaa taatagtaat tgtaatttgg agggaattaa ttagaagagt ttgtgtttat 3600
ataagttagg atttttatga tgtgcgtttg atttttatat tggttgtgaa ttagaggaaa 3660
tgtaaagaaa aaagaattag cgttagagat gagtattagg aaggattgtt taattaagta 3720
tttagtgatt tgaaaaatga gtttatatga atgtttttat atttttttgt gtatttaaat 3780
tttttgaaat agaattattt aatattagaa ttaggatttt aattgtatta gttataattt 3840
atgtagtgtt tatttagaag agtttaataa tgtggtgaat attataatag gtttttaaag 3900
tttaatataa ttagttttaa tttttgtaga tattaattgg aaagttaagt aaataaagag 3960
tatatattaa gcgagttttt ttttttagga tattgaaaaa tgaattaata gtgcgaattt 4020
taatgtttta gtttatagtt ttatatttga gaggataaat gtttgaaaga tagtgtagtg 4080
ttttttatgg atttgggttt tagtttaatg gtttagaaga atttaaatgt ttgaatttat 4140
aattaattaa aattatattt tttaaaaatt atgtttgatg aaattatata ataaagattt 4200
tgttgtgaaa agttataaaa tataggaagt tattattttg gggtttttgt ataataggaa 4260
ttaatattat tgatgttatt ttggggttta agaatttttg ttaaatttat tttagatatg 4320
tgttttttta atttaaagtt agtttttttt tttttttttt ttttttatat atatatttat 4380
taattttatg ttttagtaga agaagttatt tagatttttt agttaggagt ttttttgttt 4440
tttttgttta aaatgaggga aaagatgttt ttaaattttt atattaaaat ttggttgtag 4500
taggtgtagt gtttgaaagt tttagagttt tttcgttgtt tttttgtata gcgtgatgat 4560
aagagggtat ttataaaata gtttaaagga aaagttgata tagtttttta gattatattt 4620
tatttttttt tttttttttt gtgtagtttt ttttttatag tatagttgaa tttttaaata 4680
ttcgattatt ttgtattgtt aggttatttt ttaggttagt tcgagaaagt ttgagaaaag 4740
ttttatagga aagatgtatt atttaacgga atttaatttt ttttaataga tttgaattat 4800
taatataaat gtatgaatgt agattatatt tttgtaatga ggtttgaatt atttttagtt 4860
attttaaagg attgaaattt tatttttttt ttttagttgt aataattagg ttttaatggg 4920
tttttttatt aattgttttt ttgttaggat tgaaaaggat agtataagtt ttagattaga 4980
gtggtattta ttaattttta aaggttgttt ttttttttta tttaattttt ggaaggtata 5040
ttttattttt atgtttttaa atatatttgt tattattagg ttagttttgt attgagagaa 5100
atagatgtag ttttttgtta cgtgtgatgt tttgagaaat gattgaggtt ttttaaaata 5160
agattatggt agtagttatt gaagaatttt aaaatttggt ttttgaattt tagagaagta 5220
attagtgtat gtattagtag attttttagt agttattgtg tttgagtagt ttatatttta 5280

tatatgtatt gtttggattt tttatgttgt ttagtattat ttatggttta gttataagat 5340
gtgtgtttta ttatgtatga tgttattggt agcgttagta agttgtttta agtaatgtta 5400
agtaaggtta aaagttagtt acgttgttta aaaatttaga tatatgtagg ttttattttt 5460
ttattttgtg gtgtagattt tatgagtatg tgttatagtt tatttttttt ttttggatat 5520
agaatatgtt agttttaagt tattattagg atttgtgtat tttttaggtt gtagttagaa 5580
atgatgttaa taattattta tatttgggtg aatgaaggag tgatatttta ttgtttttaa 5640
attaggagtt ttaagtgaat agaaaagagg tgttatttta tagatagtgg tgtgtattgt 5700
tttagagtta gtgggaattt tagttataat tgaatttaat atatgtattt tatagatgaa 5760
taagttgatt tttaggttat tttatggtag attagaattt ggaatttttg tgatagattt 5820
taggttatta ttttaatttt tgtaatattt gttttgttta tatttttttt tttttttttt 5880
attttttttt ttattattga ttatttatta ttttaatatg tttaagatgg tagagtagaa 5940
atgataaaaa gagttagggg tttagttatg gaaaagtaat tatatttttg ttgtaataga 6000
ttaattagag aaaagtaata aaatagtgtt gaattattaa gtattagttg ggttagttaa 6060
aataaatatt gatatatatt tggtaaaggg ggatatttta ttaatgtttt cgagatgagt 6120
ataattaaaa agtaatgaat attttatatt tttagaatat tttaattttt taatagaatt 6180
tgaatttata agtttatttt tagaaaattg atgagatttt ttagaaaagt agatttataa 6240
tttgattcgg gtttttgcgt tttaatatta attttttata aagtattgtg atattttaaa 6300
gtataggttg tttaaataag ttttt 6325

<210> 89
<211> 5771
<212> DNA
<213> Artificial Sequence

<220>
<223> chemically treated genomic DNA (Homo sapiens)

<400> 89

aaagatataa ttgttatttg atttagtaat tttattattg ggtatgtatt taaaggaata 60
taaattattt tattataaag atatatgtat gtgtatgttt attgtagtat tatttataat 120
agtaaagata tggaattaat ttaaatgttt ttaatggtag attggataaa gaaaatgtgg 180
tatatataga ttatggaata ttttgtagtt attatgtttt ttgtaggaac gtggatggag 240
ttggaggtta ttatttttag taaattaata taggaatagt aaattaaata ttatattttt 300
ttatttataa atagagttaa gtagtgagaa taaatggata taaatagaga ataatagata 360
ttggggttta tttgagggtg gagggaggga agagaaaata taattattag gtattatgtt 420
tagtgtttcg gtgataattt gtatattgaa tttttgtgat atgagtttat ttgtataata 480
aatttgtata tgtatttttg aatttaaaag attaaaaaaa gaaaaaaaaa gaaatatgtg 540
ttggattata attttagata tgtaaagaat aatatataaa tttatattta taaatataaa 600
ttaatttagt tttaattttg agaattttgt ttttttagtt tggtaaaaaa tttaatttta 660
gttattttag ggtttttttt ttttgtttta agatgggata gattttaaga gttttatgta 720
tggttaaagt taaggtattt gggtgttgag ttattcgttt attttgattt tttttttggg 780
gtatttattt attttgttta tatagttttt atagttttga attttttttt atttttagaa 840
ttattttaaa atatgggaat gtttatgagg ggtttttatg gatttatttg tttagatata 900
tgaggtagtt ttgatttttt ggagtattgt tgtttttgtt atgtgcggtg gtgttgtgta 960
tagataaata tataagtatg gttttttaaa tatgggggtt ataaattttt tttatgtttt 1020
taggtttatg ggattttgtg atagtttttt tgggttgttt tttaaatgta ttttagtgtt 1080
ttttttattt ttagtgcgtt tggtatgatt ttttgaaagg gatttatgtt ttattttagt 1140
taggaagtat ttatgatttt ttttttttaa tttttttttt ttaatatttt tttgaaaata 1200
tgaagaatag aaaaaaatgg gataaatgaa tatagtttaa tattttataa gtttattttg 1260
ttaataggaa ttaagttttg attagaatag aggtagagta aaattttgat tatgttattt 1320
ttttgtttat tatattataa tggtttttta ttgtttttgg aatatagatt atatgtttta 1380
ataaaatata tggagatttt gtttgttttt ttaatttttt tatgtattat agtttttttt 1440
tatttttttt attgggatta aatttgtgtt ttattgtgaa aaggataggt ttttttatta 1500
ttattatttt tttatttttt atttttgtat agtatttttt tatttggtta atttttattt 1560
atgttgtaaa gttgtagttt tagtggaatt tttgtaggga aagtttatat attgttattt 1620
attatagata tttatggtat tatgtatttt ttttttgtag tatttgttta tgatggttat 1680
ttttttttat aagaagagaa atgtaaagga taattataat ttaatggtat ttttatttgt 1740
tagattaata aaattttaaa tgtttttata ataaattttt tgaaataaat gagtattttt 1800
atttattgtt ggtgggatta taaaattagt ataatttttg tggaggataa gttggtaata 1860
tttttttttt aatatttttt gttttaggta gtttattttt ggaaattgat tttatagatt 1920
tattttcgta gtataaaatg atatattttg tttttattta ttatagtatt tttttggtaa 1980
tagaaaagta ttgaagaaaa ttaaatgttt tttaatagga ggttgtttaa atatattgtg 2040
atatatttat tcggtggaat attttgtagt tataataaaa ataaaaatgt gagaagtttt 2100
ttaaataatg agataggaag atttttttgt taaaaaaaaa tataaaaaaa tgtatatgac 2160
gtgttatttt ttgtataaaa agggtaaaag taaaaaataa tatgtatgtg tatatatata 2220

tatatataaa tatatgtatt tagtttgttt gtataagtat agcgaaattt tgaaaagata 2280
tataagaaat taataaaaag aattatttat gttttgggga agaatgaaaa ttgataggat 2340
atggaataga ataagatgta agaatgaaaa gtaatgaaaa tatttaatta tgtttttttt 2400
tattgattat attatatatg attatgaacg aggtaaagtt ttgttttttt tttttagatg 2460
gagtttcgtt ttgttattta ggttggagtg tagttgtgcg atttttgttt attgtaagtt 2520
ttgtttttta ggtttatatt atttttttgt tttagttttt cgagtagttg ggattatagg 2580
cgttcgttat tatgttcggt taattttttt gtatttttag tagagacggg gttttattgt 2640
gttagttagg atggtttcga ttttttgatt ttgtgattta ttcgtttcgg ttttttaaag 2700
tgttgggatt ataggcgtga gttattatgt tgggttagtt aattttttga agttagtaat 2760
ggtgtgttaa aaaagaaaat ttaatttata aataataaaa aaaagataat agtttaaagt 2820
atttttgtgt ttaatttatt attataggtt tattaaaata agagtaataa acggttgtta 2880
atgtataatt taaaatattt agagggttat tttatttata gttgtttaaa gttattttta 2940
gtttttggag aatttattta gaagtataag tataataagg gtatagtgaa atttaagttt 3000
ggttttttat tatagttgtg gttttaatga attgtgggtt tattttgaat taataatttt 3060
ttaataaaaa tgataattaa agaatttaaa ttattgatat agtttttttg ttgatattaa 3120
tttgtttatt aatattattt ggattatatt tttaaattat agagtgtaat agtatttaat 3180
gtatttttat agattataat tttatttttt tgttttttat tttatagtta attttatgta 3240
gtagatatta aggaggtatt aagtattttt tgttaataaa ttatttattt tttttattta 3300
agtatttagt atttagttag taaatgtgga ttttttattt attgattttt tggtttaaaa 3360
attgtaggtt tagtatggaa taaatggatt tagtagtttt tgaatatttt tatattgtaa 3420
atttattatt gataattttt atttttttag aagtaatatt taagttttag gaattttgag 3480
tttgtttatt attgttattt tttaaattag cggagtgtga tttagaatat aataatatta 3540
tatttttttt tttttttttt tttttttttt tagagaatat atgataggat gtttttagtt 3600
ttttagtttt taatagatgt aatgattaaa gagtttgttt ttttttgttt taataaatgt 3660
ggaatattta atatttggta tatgaagagt aattgggtat atttgaatta ttttgtgttt 3720
tttattttgt tttttaaagg gatagatata aagagaaatt atataaatta taatttagtt 3780
gagtagattt aatttttttt ttttatttgg tttatatgta ttatttggtg gtaaagtagt 3840
gtattttata tagagagaga gagagagaga gagagagaga gagagtgtgt gtgtgtgtgt 3900
gtgcgcgcgc gcgcgcgtgt acgcgtgtgt gttttgagga gtatataata tgaaaaagat 3960
aattttaata tattttttta atattgattt agatagataa tataattatt ttatattaag 4020
aatttaatat aatatttgag tattatttta tgtataatag aataggaagt aaaggagaga 4080
atttatgttg gaaagtaaaa tagtttttat ttatttattt tagtaatata tattataatt 4140
ttatttttaa attaatattt attgttttaa tatttttatt gaggtaaaaa ttaaatattg 4200
ataattaatg tgaaattatt tttttttttt taaagaataa tagtttttaa aagtaggtgt 4260
tttgtttttt atattagtag tgagtataag ttatagatta tagaattgta aagtattata 4320
gaggtattag attaatatat taattttata aatgtagtag ttgagatttt ggaatagtag 4380
gtaatttgtt cgaagttata ttggttatta gtggtttaat ataaataaga atttagattt 4440
ttttattttt agtttaggaa tataatatat aaaaattgta tttatattta tatttaatga 4500
attgtttttg tatgatgtga taatatttaa tttatttata tagtgtttta aggtatttat 4560
atgttagtta atatatatta gttttgtttt aatattttga gtataaagaa gtaattgaag 4620
aaatgtatgt attataaagt tatggaaaag attttttaag tattaaaaaa ttttatatat 4680
atgtttaaat gaataatttt ttaagtataa tataattgtt atattgtaaa gaaggtttaa 4740
aaaatttttt tttttttttt aattgtttta gaattagaga tttgttttta taaagtaggt 4800
atttatttgt agatttaagt ataaaatatg tttatagttt ttttttatga ttattgaata 4860
ttatataagt ttttttttat tttcgttgtt gtatttaatt aatagtttat tggttaagtt 4920
ttaattgttt ttaatgaggt tagtaaaggt atttatcgaa aagttttgaa taaaaggttt 4980
atatatatat ataagtatat acgcgtttat atatagagag aaaatttttt tgtttgttga 5040
tttatggaaa taattatgat tttgttggag aattttttag ttgagaaata gtttgtagtt 5100
atagtagaaa ggtttaagtt gtattaggta gataatagat atggaatttt tatatattta 5160
gttgttagta ataaaataag taagttattg ttatttgttt tttaaaataa tgttgaatgt 5220
tttttaaaag tttatttttt tttttgattt tagtattgag taggaaagta aagttataag 5280
atttttgagt tatgtaatta tagtaattat gttgtagaat ataaagataa ggttgaagtt 5340
ttttagagta attttgatat ttaatttaaa aaatttgggt agtttaggag aaaataattg 5400
attaattata aatgtaaaag tatataataa agaagtttag ggagaaagta gttatatttt 5460
gttagtagta attgtaaaaa tttaatttaa attaatagtt ttgagtgtag tatggttttt 5520
ataaaatagt aattggaatg aatggtatag ttatattttg ttttgttttt atttatgtat 5580
aaattttata aatttatata taaagtggta aagattttga tattttttgt gaaggttgtt 5640
ttaaatgtat ttttaaagaa taatttatat tgtattttaa tgttattatt tatttattaa 5700
aatttatata tataattttt tatttttttt tttttttttt tttttatttt taatttttta 5760
ttgtaaatag a 5771

<210> 90
<211> 5771
<212> DNA
<213> Artificial Sequence

<220>
<223> chemically treated genomic DNA (Homo sapiens)

<400> 90

tttgtttgta atgagagatt aagaataggg aggggaggag agaggaagat aaaaaattgt 60
atgtgtaaat tttagtgggt aagtagtagt attagaatat agtataggtt attttttgaa 120
gatatattta aaatagtttt tatagaggat gttaaaattt ttattatttt atgtgtagat 180
ttataaaatt tatgtatgag tagagataaa atagaatatg attgtattat ttattttaat 240
tgttattttg tgagaattat attgtattta gaattattga tttaaattaa gtttttataa 300
ttattgttaa taaagtgtaa ttgttttttt tttagatttt tttattatat gtttttatat 360
ttgtggttaa ttaattattt ttttttgaat tgtttaggtt ttttaagtta aatgttagga 420
ttgttttgga gaattttaat tttattttta tgttttatag tataattatt atagttatat 480
ggtttaaaaa ttttgtaatt ttattttttt gtttagtatt aaaattagaa aaagagataa 540
atttttagaa aatatttagt attgttttaa aagataaata atagtaattt atttgttttg 600
ttgttaatag ttggatatat aagaatttta tgtttgttgt ttgtttggtg taatttgagt 660
ttttttattg tagttataaa ttatttttta gttgaaaagt tttttagtag aattataatt 720
gtttttataa attaataggt agaaggattt tttttttgtg tgtgagcgcg tgtgtgtttg 780
tgtgtgtgtg tgagtttttt atttagggtt tttcgataaa tatttttgtt gattttattg 840
gaagtaatta aaatttagtt agtgagttgt tagttgaata taatagcgag aataaaagga 900
ggtttgtata gtatttaatg gttatgagag ggagttatga atatgtttta tgtttaaatt 960
tataagtggg tatttatttt gtagaaatag atttttggtt ttgaagtagt tagaagggga 1020
aagggaattt tttaaatttt ttttgtaata tagtagttgt attatatttg gaaaattatt 1080
tatttgaata tatatatagg gttttttaat atttagaaaa ttttttttat agttttatga 1140
tatatgtatt tttttaatta tttttttgtg tttagaatgt tagaataaaa ttggtatgtg 1200
ttaattaata tatgagtgtt ttaaagtatt gtgtagataa gttgaatatt attatattat 1260
atagagataa tttattgaat atgaatataa gtataatttt tatgtattat atttttaggt 1320
tgaaagtgag aagatttaga tttttatttg tgttggatta ttaatgatta gtatgatttc 1380
gggtaagtta tttgttgttt tagagtttta gttgttatat ttgtaaaatt ggtgtattgg 1440
tttaatgttt ttatggtgtt ttataatttt gtgatttgtg atttatattt attattaata 1500
taaggaatag ggtatttatt tttaaaagtt gttatttttt aaaaaggagg aaatggtttt 1560
atattgatta ttagtgtttg atttttgttt taataagaat attggaataa taaatgttaa 1620
tttagagatg gaattataat atgtattgtt aaggtaaata agtgaaagtt attttatttt 1680
ttagtatgag tttttttttt tattttttgt tttgttatat atgaagtagt gtttaagtgt 1740
tgtattagat ttttggtgta gaataattgt gttgtttatt taaattagta ttaagaaagt 1800
atattaaaat tatttttttt atgttatata ttttttaaaa tatatacgcg tgtacgcgcg 1860
cgcgcgcgta tatatatata tatatatttt tttttttttt tttttttttt tttttttttt 1920
atgtgaaatg tattatttta ttattaggtg gtgtatgtag attaaatagg aaaaggaaat 1980
taaatttatt taattggatt atagtttatg tgattttttt ttgtatttgt ttttttaggg 2040
aatagaatga agagtataga atagtttaga tgtatttagt tattttttat atgttaaatg 2100
ttgaatgttt tatatttatt aaagtagagg gggatagatt ttttgattat tgtatttgtt 2160
gaaagttaga aagttgaaaa tattttatta tgtatttttt aaaaggagga agagaaggag 2220
gaaagaaaat ataatgttat tgtgttttga attatatttc gttagtttgg gaggtaataa 2280
taatgagtag gtttagaatt tttaggattt agatattgtt tttaaagaaa taaaagttat 2340
taatagtaag tttatagtgt aaagatattt aagagttatt aaatttattt gttttatatt 2400
gaatttatag tttttaaatt aagaaattag taagtgagaa gtttatattt gttaattaag 2460
tgttaaatat ttaaatgaaa gaaatggata gtttgttagt aaagagtgtt tagtgttttt 2520
ttaatatttg ttatatagga ttaattgtaa aatgggaaat aaaagaataa aattgtgatt 2580
tatgaaagtg tattagatgt tattatattt tgtaatttgg aaatatgatt taagtaatat 2640
taatgaatag attaatatta ataaggaaat tgtattaatg gtttaaattt tttgattatt 2700
atttttattg gagaattatt aatttaggat gggtttataa tttattagga ttatagttat 2760
aatggagaat taaatttaga ttttattgtg tttttattat gtttatgttt ttaaatgaat 2820
tttttaggaa ttaagaataa ttttaggtaa ttgtagatgg ggtaattttt tggatgtttt 2880
aagttatata ttggtaatcg tttgttgttt ttgttttaat agatttatag tgatagatta 2940
agtataggaa tattttgaat tattgttttt tttttgttat ttgtagattg aatttttttt 3000
tttaatatat tattgttggt tttaaagaat taattggttt agtatggtgg tttacgtttg 3060
taattttagt attttgggag gtcgaggcgg atggattata aagttaggag atcgagatta 3120
ttttggttaa tatagtgaaa tttcgttttt attaaaaata taaaaaaatt agtcgggtat 3180
ggtagcgggc gtttgtagtt ttagttattc gggaggttga ggtaggaaaa tggtgtgaat 3240
ttgggaagta gagtttgtag tgagtagaga tcgtataatt gtattttagt ttaggtgata 3300
aagcgagatt ttatttgaaa aagaaaaata aaattttgtt tcgtttataa ttatgtataa 3360
tataattagt agaagggaat ataattaaat atttttatta ttttttattt ttatatttta 3420
ttttgtttta tattttgtta gtttttattt ttttttaaaa tatagataat tttttttatt 3480
agttttttgt gtattttttt agagtttcgt tatgtttgta taggtaaatt ggatatatat 3540
gtttgtgtgt atatgtatat atatatatat attatttttt atttttattt tttttatata 3600
aaaggtagta cgttatatgt atttttttgt attttttttt aatagaaaaa tttttttatt 3660

ttattattta ggaagttttt tatatttttg tttttgttat agttataggg tattttatcg 3720
aatgggtata ttatagtgta tttgagtagt tttttattga ggaatattta atttttttta 3780
gtattttttt attattaaag aaatgttgta atgaataaag ataaaatatg ttattttata 3840
ttacgaaaat aaatttatag gattaatttt tagaagtaga ttgtttggga taaaaggtat 3900
taaaaaaaag atattgttaa tttatttttt atagaggttg tgttagtttt ataattttat 3960
tagtagtagg taagagtatt tatttatttt aaaaaattta ttgtgaaaat atttggagtt 4020
ttattagttt gataagtgaa aatgttatta aattgtagtt attttttgta tttttttttt 4080
tatgagagaa ggtaattatt atggataagt gttataaaga aaagatatat agtgttatga 4140
gtatttataa taagtggtaa tatgtaagtt ttttttgtaa gaattttatt aaagttgtaa 4200
ttttatagta tgaatgggag ttaattaggt gaagaggtat tatataaaaa taaaaaataa 4260
aaaaataata ataataaaaa aatttgtttt ttttatagtg aaatataggt ttggttttag 4320
tagagagaat gagaagggat tgtggtgtat gaagaggttg gagaggtagg taggattttt 4380
atgtgtttta ttaaggtata tgatttgtat tttaggagta ataggaagtt attataatat 4440
ggtaagtaga agagtgatat gattagaatt ttattttgtt tttgttttgg ttaaaattta 4500
atttttattg gtagaatggg tttatgaaat attaaattgt gtttatttat tttatttttt 4560
tttgtttttt atatttttaa aaagatattg gaaaggaaag gttgagggaa aaaagttata 4620
aatgtttttt gattagggta aaatatgaat tttttttaaa aggttatgtt aagcgtattg 4680
aaaataaagg aagtattaga gtgtatttga gaagtagttt agggaagtta ttatagagtt 4740
ttatggattt gggagtatag agaaaattta tggtttttat atttggggag ttatgtttgt 4800
atatttattt gtatatagta ttatcgtata tagtagaggt agtaatattt tagagaatta 4860
gggttgtttt atgtgtttga ataaataggt ttataaggat tttttataaa tatttttata 4920
ttttaaaata attttggaag taggagggag tttagaattg tagaaattgt gtgaatagag 4980
tgaatgaata ttttagaaga ggaattaggg tagacgaatg atttaatatt tagatgtttt 5040
agttttggtt atatataaaa tttttgaaat ttattttatt ttgaaataga agagggaaat 5100
tttagaataa ttgaagttaa gtttttattt aaattggaag agtagaattt ttaaaattaa 5160
agttaggttg atttatattt ataaatataa atttatatgt tattttttgt atatttgaaa 5220
ttgtggttta atatatattt tttttttttt ttttttttga ttttttaggt ttaggggtat 5280
atgtgtagat ttgttatata ggtaaattta tgttataagg gtttagtgta tagattatta 5340
tcgaagtatt aagtatagta tttgatagtt atattttttt tttttttttt ttatttttaa 5400
gtggatttta gtatttgttg ttttttgttt gtgtttattt gtttttatta tttagtttta 5460
tttataagtg ggagaatatg atatttgatt tgttgttttt gtgttagttt gttaaggata 5520
atggttttta gttttattta cgtttttgta aaggatatga tggttgtaaa gtattttata 5580
gtttatatgt attatatttt ttttatttag tttattattg agggtattta gattgatttt 5640
atatttttgt tattgtgaat agtgttgtaa tgaatatata tatgtatgtg tttttatggt 5700
agaatgattt atattttttt gggtatatat ttagtaatag gattgttggg ttaaatggta 5760
attgtgtttt t 5771

<210> 91
<211> 5127
<212> DNA
<213> Artificial Sequence

<220>
<223> chemically treated genomic DNA (Homo sapiens)

<400> 91

aggttgtttt tgagtagggt tgggttttta gagaaatttg atttcggggg ttagatggag 60
gggattggtt agattgggtg atagggaaat tagaattcgg gtggtttggt atatgggggt 120
agaaaggggt tttagggaat ttgttttttg gattaaggga tttttgtttt ttatatatcg 180
tggggtttgg gggtttttgg tgtttggaat taggtagttt tggtatttgg ttgtttttgt 240
gttaggaaga attgatatat ttggtttgag gatgagtgta tgtgattagt tttggggtta 300
gtagtttagt agttgtgttg gttgtggggg gagggttgtt aatgtttata aataaataaa 360
taaataaaat attgtttttt tagataattt ggggttagat aggttgagga tagatagaga 420
atagggtttg atgggggtta ggttaatttt tatttttagt ttttagttaa gggggagggg 480
tttcgggata tcgcgggttg gtggttttgg ggtaggtgag gttagttttg aagattttgc 540
gttcgtagta tcgattcgta cgttttttag cgtattttta gtgaaggagg ttttttttta 600
gttcgtggtg aggaattagg ggtttagttt ttaatttttt ttttttttaa gttgaggata 660
aagggagatg acgttcgttt gtgttgcggt gatttgggtc gtaggcgttt tttttcgatt 720
taggagggcg ttgttaaaaa tgtttttttg gggagttcgg cggaggggcg gtttggttgg 780
gaattatggg tttcgttgcg gagcgcggtc ggtagggaga gggaagtcgg ggttggcgtt 840
ttttggcggg cgaggttgaa gtcgttcgtg gtcgtatcgt ttttttggtt tttagaggtt 900
tcgggcgggc gtcgtggggt cggggttttc gagtttcgtt attgttttgg gcggtcgaat 960
tttgggcgga aggcggggga atgttattga ggtttgtatg tatgcgtgta tacgtttatt 1020
tatttattcg aacgatttga gttggagtgg ggaagtttgg ttgtggtttt tatttttttt 1080
tttttttttt tttttggttt tagtttttaa agttttcggt acgtgtttgg taggaggagc 1140

gggagattgg ttgagttttt ttttttggga ggttttttta gttgatttat ttattttttg 1200
cgcggtttcg ttttaatatt tattttattt atttttcgcg atgttattga tcgttttttg 1260
tatttttcgt cggtttttcg ggtaggtttt tcggtgggcg cgggtttgcg tcgagtacgt 1320
ttttgttcgt agtgggggtt taatttatat tagtttttcg gtttaagagt ttttcggtcg 1380
gttggggcgt ttcgcggata tagttgggta aggtttattt tttttttttc gttttagttt 1440
tttttcgagt tttttttttt gttttttagc gtatagtagg agcgttgtcg gtttattgtg 1500
gttttagatt tttgggggat gaaggtatac gtcggggttt gtcgattgtt ggaggaaggg 1560
taggttttta tttgggggtc gagggagacg atttttagat agtggtcgaa gtgtttaacg 1620
tagtttcggc gatttgtatt tgaacggtcg gtagtgagtt cgcggcgttt tgaacgttcg 1680
gcgtaggaag cgcgcgtcgg agcgagttgc ggttttcggc gggtgtggat tggaggggtc 1740
gggggatggg ttgggatcga ggggtgcgtt tggaaatagg ttgaagtagt gtgtttgggt 1800
gtaggcgagg gcgtgtttgc ggagatgggt cgtttgtgtt ggcgaggagt gtgtgtttgt 1860
gtttgtaggc gagggagttt gtgtttgtgc gtgtgtgtgt ttgtgtttgc ggagtgtggg 1920
ggcggttagg aaaggtggtc gggttgttat ttagcgatta ggttgatagg cgttttttta 1980
tttgggttag ggagttttga ttgtagattc gaggaaataa aatagtaatt gtaattatcg 2040
ggttttgata agataaagga gggagggagt tggtcgggag gcgggcgagt tagtaaggga 2100
gggagcggtt tgcgggggtt cggcgggata tttgtatttg ttagttacgg ggcgtgttag 2160
ggtagagtta ggtgtttgtt ttcggggtgt ttttatttgt tttttttttt tagagaaacg 2220
gatttttggg gagatgagaa ttttaacgaa ttaggtgagg gttcgttttt taggagggtt 2280
tttaaattta aatgtaaagg aattttggtt tagtataggt tttttggtag aggttgggat 2340
aggattgaaa tttgatttcg gattttttag attttaggac gtttattttt tttggggtag 2400
aggaagaagg gtggttgaag attattgagg gttaatttag ggtttttaga attttttggg 2460
ggttttatag ttggagttga atgttggttt tagttagggg ttttgttttt atttttattt 2520
tagggagggg taggtattta gagtggtttt ggttgtgatt ttaggattat gtaaatattg 2580
ttggatgggc gggttatttt tatttttgtt tggggtttat tttttgtttt gtttatttta 2640
ttgatgttta gagagtttat ggtgggagaa agtttatttt ttataggaag tgtttgtttt 2700
ttattttttt atttaattat ttgtttatcg atatacgtaa ttaaatatgt ttgagggttt 2760
attgtgtata aggatgaggg taagattata agtaaataga tataattttt gtttatttga 2820
agtagtgtag agagggaaat agatatttaa tgaattgtga tttaggtttt atatagaata 2880
tagggaaggt aagggttggt ttgggttgtg gttatttgtg tttatttgat ttgttttcgt 2940
ttgatggatt tattttttta tgggttttag aggagatagt gtttgttaag tttgtggtag 3000
atataagtgt ttattaattg gtggtgtttt tggtgataat ttgttgggag gaggtgaagg 3060
taggtttttt tggggaagtt gtaaataggt tgaagtttat aggtttagtt atttttttta 3120
ttatttattt atttatttat ttaacgaatt tttaattgta gaatagtttt gtttagtttt 3180
gtgttacgag ttgtgatttt tgagtttata agatatggtt tttgtgggta aggtttgtgt 3240
gggtaagagt ttattgaata gatttttgga ggaggatggt aagtgtgttt tggaagttta 3300
tttttttgag gagggataag attttggaac gtatttttta attgtttttt tattttttta 3360
ttgtttagtg tttgtattta tggttgggag ttggaaagat ggggtatagg gaaaggtggg 3420
atatagagta gggagggagg tagggtttgg ttgtgttgtt ggattagggt tgtcgttaat 3480
tttggtttaa tggttttttg ggattatatt aaatttttta gttttatttt taggggaatg 3540
gagggttatt ggggaagggg aggttgtaag tgaagatagt agtttggtag ggtattattt 3600
gttttttata aggtatttgt tttagagtat ttttgtttat ttaggttgtt tttatagaag 3660
tagtatgggg gtttagggtt tttagaaatt ttagggggga agttataagg tggggggtta 3720
ggaaaggttt agataagtgt ttattttttt ttatgtttta agtttagttg attagttttt 3780
gtttagaata tagtaggtgt gtaaatattt gtgaaatgaa aaacgagtgg ggttttgagg 3840
gggaaagggt tgggtggttt ggatggaagt tttttttttt agtaaaagtt attgagagtt 3900
tttttgtttt tgttgtttgt tgaggtacgg ggatagtggg ttttgttagg gattttcgga 3960
tttttttttt tatataggtg gttcggggtg ttttttaagt tttaggttag tgtgtgtgtg 4020
tgtgtgtgtg tgtgtgtgtg tgtgtgtggt gaggggtata ggggaagggg tgatagtgat 4080
aatgggatgg ggggaggggg aggtattttt ggtttttagg gttttttcgg gagagtgaag 4140
gagattggag agatcgatag gacggggtac gcggtcgaaa taggggagcg gggttcgggt 4200
ggtatcggta cgtattgcgc gagggtcggg tgcgtagtta ttcgttgtat ggttagcggt 4260
cgtttttagg ggttcggcgg gatatatagt aggtggttcg gagattattg cgtagtcggc 4320
ggcggaagcg gttgggttcg gaggcggaga tttagattcg ttagattcgg gcgttttagg 4380
cggtcgggga agtttaggtt cggggttttt cggttcgtag agtttgtagt tttaggcgtt 4440
tttattttcg cgcgttcggg atcggggagc gcgcgagggg ataggtcggg gataggggga 4500
ggcgtttaga tcggtcgttt tcgttttttt cgcgggtttt tagatttttt ttttagttta 4560
gatttttaat ttcgaattcg tcggtcgttt tttttcggtc gtttcgtttt cgtttcgggt 4620
tgtatttagg ttttagtttt tagtcggggg ttgcgggttt tcgtcgttcg tttcgcgttt 4680
ttttttcgcg ttcggtgcgg cgtttttcgt ttttattttt tttcgttttt ttattttttt 4740
atttatttcg ttagttcgtg attttttcgt tcgtttcggt tttcgagtag ttagcggtta 4800
tcggtttcgg tagcggcgta tagcgattcg gtgcggcgcg gcgagtacga cgttttacgg 4860
gattcgcgga gtcgcgtcgt gatcgtcgtc ggtttttcgt attcgtattt ttttcgttcg 4920
cgttttgttt agcgcgtttt ttcgcggcgg ttcgcgggac ggcgtgattc gtcgggtttt 4980
cggtgtttcg gggtcgcgcg ttatgggtag ttttcgtttc gcgttgagtt gtttgtgagt 5040
atcgcgtcgt tttgtttcgt tattcgtttt tcgcgttttt cgttttattc gcgttttttt 5100

ttttcgttcg tttttgtttt ttatagg 5127

<210> 92
<211> 5127
<212> DNA
<213> Artificial Sequence

<220>
<223> chemically treated genomic DNA (Homo sapiens)

<400> 92

tttgtgggag ataaaagcgg gcgggagaga gaggcgcggg tgaggcgagg ggcgcggggg 60
gcgggtggcg gggtagggcg gcgcggtatt tataggtagt ttagcgcgga gcgggggttg 120
tttatggcgc gcggtttcgg ggtatcgaga gttcggcggg ttacgtcgtt tcgcgggtcg 180
tcgcgggagg acgcgttgag tagggcgcga gcggagaggg tgcgggtgcg ggaggtcggc 240
ggcgattacg acgcggtttc gcgggtttcg tggaacgtcg tgttcgtcgc gtcgtatcga 300
atcgttgtgc gtcgttgtcg gagtcggtgg tcgttggttg ttcgggagtc ggagcgggcg 360
ggagggttac gggttggcgg ggtgggtggg gaggtgggag ggcggggagg ggtgggggcg 420
gggggcgtcg tatcgggcgc gaggggggag cgcggggcgg gcggcgggag ttcgtagttt 480
tcggttggga attggggttt ggatgtagtt cggggcgggg gcggggcggt cggggagggg 540
cggtcgacgg gttcgggatt gagggtttgg gttaggggag gggtttgggg attcgcggga 600
ggggcggggg cggtcggttt gggcgttttt ttttattttc ggtttgtttt ttcgcgcgtt 660
tttcggtttc ggacgcgcgg ggatgggggc gtttagggtt gtaaattttg cggtcgggga 720
gatttcgagt ttgggttttt tcgatcgttt ggagcgttcg agtttggcgg gtttgggttt 780
tcgttttcgg gtttagtcgt tttcgtcgtc ggttacgtag tggttttcga gttatttatt 840
gtgtgtttcg tcgggttttt gggagcggtc gttaattatg tagcgaatgg ttgcgtattc 900
ggttttcgcg tagtgcgtgt cggtgttatt cggatttcgt tttttgtttt cggtcgcgtg 960
tttcgttttg tcggtttttt tagttttttt tattttttcg aggaggtttt gggagttaga 1020
aatgtttttt ttttttttta ttttattatt attattattt ttttttttgt gttttttatt 1080
atatatatat atatatatat atatatatat atatatatta gtttgaagtt tgaggagtat 1140
ttcgagttat ttgtgtggaa gagagagttc ggaggttttt ggtaggattt attgttttcg 1200
tattttagta ggtagtaggg gtaaaaaaat ttttagtagt ttttattggg ggaagggatt 1260
tttatttagg ttatttagtt tttttttttt taaagtttta ttcgtttttt attttatagg 1320
tatttatata tttattgtat tttaggtagg ggttggttag ttgggtttgg ggtataggag 1380
aaggtaggta tttgtttaag ttttttttag ttttttattt tgtggttttt tttttgaggt 1440
ttttggaaat tttgagtttt tatgttgttt ttgtgggagt agtttgggtg gatagagatg 1500
ttttgggata ggtgttttgt gaggagtagg tgatgtttta ttagattgtt gtttttattt 1560
ataatttttt tttttttagt gattttttat ttttttgggg gtagggttgg agggtttggt 1620
gtggttttag gaagttatta ggttaaaatt agcggtagtt ttagtttagt agtatagtta 1680
ggttttgttt ttttttttgt tttgtgtttt attttttttt gtgttttatt tttttaattt 1740
ttagttataa atgtaaatat tgagtaatgg agagatggga aaatagttag agggtgcgtt 1800
ttaagatttt attttttttt agagaagtag atttttagag tatatttgtt attttttttt 1860
aggagtttgt ttagtgggtt tttgtttata tagattttgt ttataaggat tatgttttat 1920
gagtttagaa attatagttc gtggtataag gttgggtaga gttgttttgt aattaagagt 1980
tcgttgaatg aatgaatgaa tgaatgatgg gggaagtggt tgagtttgta gattttagtt 2040
tatttgtaat ttttttagga agatttgttt ttattttttt ttaatagatt attattagga 2100
atattattag ttagtgagta tttgtatttg ttataggttt ggtaggtatt gtttttttta 2160
aagtttatgg ggaggtgggt ttattaggcg gagataagtt aggtggatat aagtggttat 2220
agtttaggtt agtttttgtt ttttttgtgt tttgtgtaag gtttgagtta taatttattg 2280
agtgtttgtt tttttttttg tattgtttta agtgggtaaa ggttatgttt gtttgtttgt 2340
ggttttattt ttatttttgt atatagtagg tttttaagta tgtttagttg cgtatgtcga 2400
tgaataaatg attgaatgga gagatggaga gtaggtattt tttgtaagaa atgggttttt 2460
ttttattatg gattttttga atattagtga gataagtaga gtagggagta ggttttaggt 2520
aggggtgagg gtgattcgtt tatttaatag tgtttgtatg gttttggggt tatagttaga 2580
gttattttgg gtatttgttt ttttttggaa tgggggtagg ggtagagttt ttggttgaga 2640
ttagtattta gttttagttg tggaattttt agaagatttt gagggttttg ggttgatttt 2700
taatggtttt tagttatttt tttttttttg ttttagagaa gatgggcgtt ttgggatttg 2760
aagggttcga ggttaagttt tagttttgtt ttagtttttg ttagggagtt tgtgttggat 2820
taggattttt ttatatttgg atttggagat ttttttgaga agcggatttt tatttagttc 2880
gttgaaattt ttattttttt aaaagttcgt ttttttgggg gagaggagta gatggggata 2940
tttcgggagt aagtatttgg ttttgttttg gtacgtttcg tgattgatag atgtaaatgt 3000
ttcgtcgggt tttcgtaggt cgtttttttt tttgttggtt cgttcgtttt tcggttagtt 3060
tttttttttt tttattttat taaagttcgg taattataat tgttattttg ttttttcgaa 3120
tttgtaatta gagttttttg gtttagatga gggagcgttt gttaatttga tcgttgagtg 3180
atagttcggt tatttttttt ggtcgttttt atatttcgta aatataaata tatatacgta 3240

tagatataaa ttttttcgtt tgtaaatata aatatatatt tttcgttaat ataaacggtt 3300
tattttcgta gatacgtttt cgtttgtatt tagatatatt attttagttt atttttaaac 3360
gtatttttcg attttagttt atttttcggt ttttttaatt tatattcgtc ggagatcgta 3420
gttcgtttcg acgcgcgttt tttgcgtcgg gcgtttaaag cgtcgcggat ttattgtcga 3480
tcgtttaaat gtaaatcgtc gagattacgt taagtatttc ggttattgtt tgggagtcgt 3540
ttttttcggt ttttaaatag aagtttgttt tttttttagt agtcggtaaa tttcggcgta 3600
tgtttttatt ttttaggggt ttgaggttat agtgagtcgg tagcgttttt attgtgcgtt 3660
gaagggtaaa gaagagggtt cgaggagagg ttggggcgag gagagaggaa taaattttgt 3720
ttagttgtgt tcgcggggcg ttttaatcgg tcgagaaatt tttgggtcga gaggttgatg 3780
tggattgggt ttttattgcg ggtaggagcg tgttcggcgt aggttcgcgt ttatcgggag 3840
atttattcgg aaaatcggcg agaaatgtaa ggagcgatta gtggtatcgc ggggggtggg 3900
tggggtaaat attggggcgg agtcgcgtag ggggtagatg ggttagttag ggaggttttt 3960
taggagagag gatttagtta gtttttcgtt ttttttgtta agtacgtgtc gaggattttg 4020
gaggttgggg ttaagaagag gaagggaaag aggagtgagg gttatagtta gattttttta 4080
ttttaattta agtcgttcga atgaatgaat gaacgtatgt acgtatgtat gtaagtttta 4140
gtggtatttt ttcgtttttc gtttaagatt cggtcgttta gagtagtggc gggattcgga 4200
ggtttcggtt ttacggcgtt cgttcggagt ttttagaggt tagggaagcg gtacgattac 4260
gagcggtttt aatttcgttc gttagagggc gttagtttcg attttttttt ttttgtcgat 4320
cgcgtttcgt agcgagattt atggttttta gttaggtcgt tttttcgtcg ggttttttag 4380
ggaagtattt ttagtagcgt ttttttggat cggagaaagg cgtttgcgat ttagattatc 4440
gtaatataga cgggcgttat ttttttttgt ttttaatttg aggaagaggg aggttggaaa 4500
ttggattttt agttttttat tacgggttgg gggagaattt tttttattag ggatgcgttg 4560
aagagcgtgc gggtcggtat tgcggacgta aggtttttaa agttgatttt atttgtttta 4620
gaattattag ttcgcgatat ttcggggttt tttttttttg attagaggtt gggagtggga 4680
gttggtttga tttttattag gttttgtttt ttgtttattt ttagtttgtt tggttttagg 4740
ttgtttgggg aggtaatgtt ttgtttgttt gtttgtttgt gggtattgat aatttttttt 4800
ttataattaa tatagttgtt gaattgttga ttttagagtt ggttatatat atttattttt 4860
aaattaggtg tgttagtttt ttttggtata aaggtagtta agtgttagaa ttatttgatt 4920
ttaggtatta aagattttta gattttacgg tgtgtggggg ataagaattt tttagtttag 4980
gaggtaaatt ttttggggtt tttttttgtt tttatatgtt aggttattcg gattttggtt 5040
tttttgttat ttaatttggt tagttttttt tatttggttt tcggagttag gtttttttaa 5100
gggtttagtt ttgtttagag atagttt 5127

<210> 93
<211> 6077
<212> DNA
<213> Artificial Sequence

<220>
<223> chemically treated genomic DNA (Homo sapiens)

<220>
<221> unsure
<222> (1153)

<400> 93

tgtaattttt gtttttcggg tttaagtaat ttttttgttt tagttttttg agtagttggg 60
attataggcg cgcgttattg tacgtagtta atttttgtat ttttagtaga gacggggttt 120
tattatgttg gttaggatgg tgtcgatttt ttgatttcgt gatttgttta ttttagtttt 180
ttaaagtgtt gggattatag gcgtgagtta tcgcgtttgg ttaatatttg ttttttaatt 240
aatttgtttg tttagatttt atttaatgta attatatttt taaaatatta ttatttgaat 300
atgtaattaa tatagaaatt attgatgaga tattttatat tttttttata ataagttttt 360
aagatgcggt gtatattttt tatttatagt atattcgtta gtattagtta tattttaagt 420
gtgtagtggt tattgtgtgg gttataggtt tagaatataa gatatgaaga tggagagtga 480
gaaatgtttt tggaaaggtt ggaagttttt gtttttttgt tgttaattat taaatttttt 540
gagagtgtta ttaaggagtg atttaaagta ttatataaag agaattataa atattttaat 600
attatatttg aaatttaaat gtataatttt ttatatttgg ttggtatttt agagaggaga 660
gaatgggtat agttatttat attatttatt tgagttttat aattatttgt gatgtggttt 720
tttttaggtg ggaaatcgag gtttagaacg gttaagtgat tattttaggg tggtaagatt 780
ataagtggaa gggtgtgaat ttatattgtt tttagcggat aagaataaaa agatttaggt 840
tgggtgtggt ggtttatgtt tgtaatttta gtattttggg gaggttattg taggtggatt 900
ttttgagttt aggagtttat tattagtatg ggtaatatgg tgagatttta tttttattaa 960
atatatagaa aattagttta gtttttcggg aggttgaggt gggaggatta tttgagtttg 1020
ggggatggag gttgtagtga gttgaggaac gtgttattgt attttagttt gggtgataga 1080
gtaatatttt gttttagaaa gaataaaaag atttggttat gaattcgtta gttagttttt 1140

tttatataat ttntggataa ggagatttga tatttatagg ttttttttta gaagtgggag 1200
agtttaagg ttacgtggtt cgtttagttt ttgttatttt attagatatt gttatatttg 1260
tatgttggat tagtatttta gtgagaagat agtaggtatt tttatttatg tagtttattt 1320
agtttttata attatttgtg atggaggtaa ggtaagtatt ttagtttttt ttgatgagtg 1380
ggaaattgag atgtgttttt tttttgtttt ttatcgagga tttttgtatg taggtatgaa 1440
ttttaggagt ttagttgata ttggagagac ggggcggggg gaattagttg tagggttttg 1500
gaggaagttg ttgtgtatat ttgtaaggtt gtaggttata tttatttgtt aagtagtgaa 1560
ggaaggaagt tgtttttaag ggattggaaa aatttattaa ttagtagaat gagaaattga 1620
ggtgaagtag gaggtggtag ggttttagat agtatgttgg attagtggtt tgtgttattg 1680
tgttttttgt aggcgggtgg tatggggtgt atgttgattt tttattttag gagttggtgt 1740
taggaggtta ggttttttta atatttttgt tttatagatg ttaaatttga gggttagagg 1800
ggtaggagag gaagagattt tttgtatttt ttttgggaaa gaataagagg gaagttggta 1860
gatgaatttg aagtgtattg attagggagt tgagagaggg cggtttgtag ttagtttata 1920
tttgggagga ggcggtaggt ttttttgggg atagagtggt taagtcgaag taagtttaat 1980
tattttaata tgatattatt tttttttatt ggaatttgag aatttgttta gtattttgat 2040
atttagtaag ggatttgggt ttttttggtt aggtgtgcgt ttttgggtga taggtttgta 2100
ttaggtgtat tttcgggatg tagtaagttg tggaatatgg gtttaggggt attttttggt 2160
aagtattgtt tttattttag ttttgggaat gtgttttatg tagtgtttta gatggtttat 2220
ttgtggtttg tttttaaggg tttttttttt agttagttag ttttgagata gagttttatt 2280
tcgttattta ggttggagtg tagtgatgta atttcggttt attgtaattt ttatttttta 2340
aatttaagta atttttatgc gttagttttt tgagtagttg ggattatagg cgtgtattat 2400
tatatttagt taatttttgt atttttagta gacgaggaat tttattatgt tggttagttt 2460
ggttttaatt ttttatttta ggtgatattt tcgttttagt tttttaaagt ttcgggattg 2520
taggtatgag ttaatatgtt ttggtataag ggtttatttt tgattaatgg aattgtaaat 2580
taagtttttt ttgttattag agttattttg gatttatttt tatttatttg gtttggataa 2640
attgagtttg tattagatgg agttaggttt gattaatttt ttatttattt ttttttattt 2700
tgttttttaa tatttaaaaa ttttgaggta ttattatatg ttagttatat ttttttgagt 2760
aaagtattta atttttttga gtttagtttt ttttattgta taaaaggaat aataaaattt 2820
attttttata agtttatagt gaggaatgaa ttaatttttt attatagttt taaattaatt 2880
ttatttaggg tatttttggt atatagtggg tgtttagtat ttattttatt tttttttttt 2940
tgattttttt cgtaaaagta gaaaaatgaa agagaaatgt tgattttttt tttgatttga 3000
aattattaaa atattttagt aagttttggg agggagttag tggtgtggta tgtgtatttc 3060
gttggttaag tatatgtgaa cgaagttaag aatttagggg tttttttgtt agttagtatt 3120
gatttatttg cgaggggttt tgtttggttc gggggaggta gggttgaagt attatattag 3180
ggtatgtttc ggggaagtag attttttgaa taatttggat ggttttttgg agtatttagg 3240
atagaagtta tttggaaaat agagatggtt atttttacgt agttttgata gtgtttagaa 3300
agttttgtta tttggtaaat gttaatagtt atgattcgtt ttttaagatt ttggggagtt 3360
ttaagtttta ttttattaga tttgagaagg gtaaagggtt gtagattttg tttttttaat 3420
tggggttagt gtgagttatt tttgatttag tgtttgtaat agattttgat tttgtagtgg 3480
gattttttag gtttttttgt ttttcgtaat ttttggattt atattcgtta gatattattt 3540
gttatttttt agtattaggg agaattggat tttttttggt tttatatttt taggtttttt 3600
gtaagtagtt ggtgagggtt tttttttttt tgtaagggag gttggtagaa ttatggatgt 3660
gattcgtata attttagaga taaaaagaaa gtatttagga ggttatttat tttagttgtt 3720
ttattgtata ggtcggggag ttgagtatgg agtttagtag ttattaatta gttatttttg 3780
tatttggttt ttatttattg gtttttagtt tgtttcgtga ttttttattg ttttttattt 3840
tttattagag ggattggttg gttttagatg gagtggtttt tttaaaattt tttttttaaa 3900
ttttttgaga tagagtttta ttttgttatt taggttgtag tgtagtgttg cgatttcggt 3960
ttattgtaat tttcgttttt tgagtttaag taattttttt gttttagttt tttgagtagt 4020
tgggattata ggtgtgtatt attatgttta gttaattttt gtatttttag tagagatggg 4080
attttattat attggttagg ttggttttga atttttgatt ttaaatgatt tgtttatttt 4140
agtttttcga agtgttggga ttgtaggtgt gagttatcgt atttggtttg ggtagagtga 4200
agttttatgt tggggagtta ttagtatgtt taaatttttt gtaattgtag tatattttgt 4260
aaaattgttt tttataaaag ggtagaatta tttgggattt ttatgagatt atttattttg 4320
tagtatatat tattttgaag tttatatttt ggaaaatttt atgatggtat ttttaggttt 4380
gtacgtaatt tgtatttaaa atatagttgt agaattgaat taaagtattt ttttgtttaa 4440
ttaagattta taattttttt ttttgagata gaatttcgtt ttgttattta ggttggagtg 4500
tagtggtgta attttagttt attgtatttt tcgttttttg gatttaagcg atttttttgt 4560
tttagttttc gaagtaattg ggattatagg tgcgcgttat tacgtttggg taatttttgt 4620
atttttagta gagacggggt ttcgttatgg ttaggttggt tttaaatttt tggttttaag 4680
tgattttttc gttttagttt tttaaagtgt tgggattata gggtgtatta ttatatttag 4740
ttaggattta tgatttaatt tattgttggg gtagttttat aatttttttt tggacgtttt 4800
agtaagttta tattttaagt agttattata tggtatattt tattttttgt tttttttttt 4860
ttttttttta tttagatttt tttaattttt ggggtttttt tttttttttt agggttagtt 4920
tgaaaaggag gatcgagttt attgtggagt atttatggag atgtggagtt ttgttattaa 4980
tttttaattg tagaattggg atgtggagtt ggaagtgttt ttttttttgg gttgtgttgg 5040
ttatagttat attttgtatc gttaggtcgt tttcgatttt gtttgaataa ggtaaggtgt 5100

tggttggggg tagtttggat ttttgggttt tgggaatagg atgggttggg agggaggtga 5160
tatggttgta ggtgatgtgg ttaataggga aggttttttt ttaagtggag taaagaattt 5220
tttttttgta ggagaaattt ttaagatatt tgggttggtt ttggtttggt tataaatata 5280
attgatttta ttagaggttg gggttatgaa ggttggtgat atttatttat ttgagatgat 5340
atgtttgtgg ggggtaattt atgttttttt tttttgaggg aaagaatatt tgatgaggat 5400
tttagtgtta tattttaata taggtgatgt ataaaatgta gttggtggtg taatggggag 5460
agaatgattt tagaatttgc gtgtagaatt tgggttagat tataggggat gttgtaggag 5520
gtgagagttt gttaaagatt tttgattttt agaaaattgt tttaaggtta tattttcgtt 5580
aggtatagtg ggttatatat gtaattttag tattttggga ggttaaggta agaggattat 5640
ttgaggttag gagtttaaga ttagtttagg taatatagcg agattttatt tttataaaaa 5700
aaaaggaaat taattgggtg tgatggtgta cgttgttagt tttagttatt tgttatttag 5760
gaggttgaga taggaagatt gtatgatttt aagagttgag gttgtagtga attatgattg 5820
tattattgcg ttttagtttg ggtgaaaaaa aggtatattt tttataaggg ttagtagtta 5880
tggttttacg tttgtatttt tttattttta attgtgtttt ataggttttg tttgggggtt 5940
tgttaagcga tttgttttgg ataggtattg tttgaggggt tttgaagtga ttgagattta 6000
ggaagtataa aggggtatgg agatttatgg cgtgttggaa atttagagtt attatcggta 6060
ataggaagga tttattt 6077

<210> 94
<211> 6077
<212> DNA
<213> Artificial Sequence

<220>
<223> chemically treated genomic DNA (Homo sapiens)

<220>
<221> unsure
<222> (4925)

<400> 94

gggtaagttt tttttgttat cgatgatggt tttgaatttt taatacgtta taggttttta 60
tgttttttta tgttttttgg gttttaatta ttttaaaatt ttttaaatag tatttattta 120
aagtaaatcg tttggtaggt ttttaaatag aatttgtgag atatagttaa ggataggaaa 180
atgtaggcgt gaagttatga ttgttgattt ttatagaaga tgtgtttttt ttttatttag 240
gttggagcgt agtagtgtaa ttataattta ttgtagtttt aatttttagg attatgtaat 300
ttttttgttt tagttttttg agtagtaagt agttgggatt agtagcgtgt attattatat 360
ttagttaatt tttttttttt ttgtagagat ggggtttcgt tatattgttt aggttgattt 420
tgaatttttg gttttaagtg atttttttgt tttagttttt tagagtgttg aggttatatg 480
tgtgatttat tatgtttggc gaagatgtgg ttttgaaata attttttgga agttagaggt 540
ttttagtaag tttttatttt ttataatatt ttttgtagtt tgatttaagt tttatacgta 600
aattttaagg ttattttttt tttattgtat tattagttgt attttatata ttatttatgt 660
taaagtgtga tattagggtt tttattagat attttttttt tagaaagaga aaagtataaa 720
ttatttttta taaatatgtt attttaggta aatggatgtt attagttttt atgattttaa 780
tttttgatga agttagttat gtttataatt agattagaat taatttaggt attttgagag 840
ttttttttat aggaagggag ttttttgttt tatttgggaa ggagtttttt ttgttgatta 900
tattatttgt aattatatta tttttttttt agtttatttt gtttttaagg tttaggagtt 960
taggttgttt ttagttagta ttttattttg tttaggtaag gtcggggacg gtttagcggt 1020
gtagagtgtg gttgtgatta gtatagttta gaagaggagg tatttttagt tttatatttt 1080
agttttgtag ttagaggttg gtgataaggt tttatatttt tatggatatt ttatagtgag 1140
ttcgattttt ttttttaaat tgattttgag gaaaggaaaa aaattttaaa agttaggagg 1200
gtttaggtag gggaggggaa aggaaaaata gaaggtaaag tatgttatgt ggtggttgtt 1260
taaagtgtgg atttattaag gcgtttagaa gaaaattgtg aagttatttt aataatgaat 1320
tagattatag gttttggttg ggtgtggtgg tgtattttgt aattttagta ttttgggagg 1380
ttgaggcggg aggattattt gaggttagga gtttgagatt agtttggtta tggcgaaatt 1440
tcgtttttat taaaaatata aaaattattt aggcgtggtg gcgcgtattt gtagttttag 1500
ttatttcgga ggttaaggta agagaatcgt ttgaatttag gaggcgaagg atgtagtgag 1560
ttgagattgt attattgtat tttaatttgg gtgatagagc gagattttgt tttaaaaaga 1620
gaggttatag gttttaattg gatagaggga tgttttagtt taattttata gttatgtttt 1680
gagtgtagat tacgtgtaag tttgggaata ttattatgag gttttttaag gtataaattt 1740
taaagtagta tgtgttataa agtgaatggt tttatgaaag ttttaaatag ttttgttttt 1800
ttgtgggaaa tagttttata aagtgtgtta taattgtagg aggtttgagt atgttgatgg 1860
ttttttagta taagatttta ttttgtttag gttaggtgcg gtggtttata tttgtaattt 1920
tagtatttcg ggaggttaag gtgggtagat tatttgaggt taggagttta agattaattt 1980
ggttaatatg gtgaaatttt atttttatta aaaatataaa aattagttgg gtatagtggt 2040

atatatttgt aattttagtt atttaggagg ttgagatagg agaattgttt gaatttagga 2100
ggcggaggtt gtagtgagtc gagatcgtag tattgtatta tagtttaggt gatagagtga 2160
gattttgttt taaaaaattt aaaaaaaaaa ttttaaaaag attattttat ttagggttaa 2220
ttagtttttt tggtgagaaa taaggggtaa tgaagaatta cggaataagt taaggattag 2280
taaatggaag ttaggtatag agataattag ttagtagttg ttggatttta tgtttaattt 2340
ttcgatttat gtaatgaagt agttgaaata aatgattttt tgggtatttt ttttttgttt 2400
ttaaaattgt acgaattata tttatagttt tattagtttt ttttgtagag agaagaaaat 2460
ttttattagt tatttataaa gagtttaaga gtgtggagtt aggagggatt tagttttttt 2520
tgatgttgga aggtgataag tgatgtttga cggatatgag tttagaagtt gcggggggta 2580
agggggtttg ggaggtttta ttgtagaatt aaggtttatt gtaagtattg agttaaagat 2640
ggtttatatt ggttttagtt aaaagaatag aatttgtagt tttttatttt ttttaggttt 2700
ggtggggtaa aatttaaaat tttttagggt tttaaagaac ggattatagt tgttaatatt 2760
tattaagtga taagattttt tgggtattgt taaggttacg tgggggtgat tatttttatt 2820
ttttaggtgg tttttgtttt aaatgtttta gggagttatt taagttattt agagaattta 2880
ttttttcgga atatgtttta atgtggtatt ttagttttgt ttttttcgag ttaggtaggg 2940
tttttcgtaa gtgagttagt gttggttggt agaaaagttt ttggattttt ggtttcgttt 3000
atatgtgttt ggttagcggg atatatatgt tatattatta gttttttttt aaggtttatt 3060
aaaatgtttt aatgatttta aattaaaaga gaagttaata tttttttttt atttttttat 3120
ttttacgaaa ggaattagaa aagagaaaat gaaatgaata ttgaatattt attatgtatt 3180
aaggatgttt taagtagaat taatttagaa ttatagtgag gaattaattt atttttattt 3240
ataaatttat gggggataag ttttattatt ttttttatgt aatggagaaa ttgaggttta 3300
aagaggttaa gtattttatt taaggaaatg tagttagtat gtaatagtgt tttaaggttt 3360
ttggatatta gagaataggg tgggaggaag taaataaggg attgattaag tttgatttta 3420
tttgatgtaa atttaattta tttaaattaa gtagataagg gtaaatttaa ggtaattttg 3480
gtaataaaag aggtttgatt tgtagtttta ttggttaaag atagattttt gtgttagggt 3540
atgttggttt atatttataa tttcgggatt ttgggaggtt gaggcgggag tattatttga 3600
ggtggggagt tgagattaga ttggttaata tggtgaaatt tttcgtttat taaaaatata 3660
aaaattagtt gggtgtggtg gtgtacgttt gtaattttag ttatttagga ggttaacgta 3720
tgagaattgt ttgaatttgg gaggtggagg ttgtagtgag tcgagattgt attattgtat 3780
tttagtttgg gtgacggagt gagattttgt tttaaaatta attaattaaa agaaagattt 3840
ttggaagtag attatagatg ggttatttag gatattgtat ggggtatatt tttagagttg 3900
ggatagaagt agtgtttgtt agaggatgtt tttaaattta tattttataa tttattatat 3960
ttcgaaaata tatttgatgt aggtttgtta tttagaaacg tatatttgat taagaaaatt 4020
taggtttttt gttaagtgtt agaatattga ataagttttt aggttttaat gggaaagagt 4080
ggtgttatgt tgagatggtt aagtttgttt cgatttggtt attttgtttt tagaaaagtt 4140
tgtcgttttt ttttaggtgt gggttggttg tagatcgttt ttttttagtt ttttggttaa 4200
tgtattttaa atttatttgt tagttttttt tttgtttttt tttaaaaaag gtataaaaag 4260
tttttttttt ttttattttt ttggttttta agtttgatat ttgtaaaata aggatgttaa 4320
gaaaatttgg tttttttggta ttaattttg gaataagaag ttagtatata ttttatgtta 4380
ttcgtttgta aaaaatatag tgatataggt tattagttta atatgttgtt tgggattttg 4440
ttattttttg ttttatttta gtttttattt ttattaatta atgaattttt ttaatttttt 4500
agaaataatt tttttttttt attgtttgat agatagatgt aatttgtagt tttgtaggtg 4560
tatatagtag ttttttttaa gattttgtag ttggtttttt tcgtttcgtt tttttaatat 4620
tagttaggtt tttgggattt atgtttgtat gtagaggttt tcggtgggga gtagagaggg 4680
ggtatatttt agttttttat ttattagagg gggttgaaat atttgttttg tttttattat 4740
aggtggttat gaagattgaa tgggttgtat gggtgaaagt gtttgttgtt tttttattgg 4800
agtattgatt taatatatag ggtggatagt gtttggtgag atagtagggg ttggacggat 4860
tacgtgattt tgaagttttt ttatttttaa gagaaaattt atgaatgtta gatttttttg 4920
tttanaaatt atgtaaggaa aattagttga cgaatttatg gttaaatttt tttatttttt 4980
ttgagataga gtgttgtttt gttatttaag ttagagtgta gtggtacgtt ttttaattta 5040
ttataatttt tattttttag atttaagtga tttttttatt ttagtttttc gagaagttgg 5100
gttaattttt tgtatattta ataaaaatgg ggttttatta tgttgtttat gttggtaatg 5160
aatttttggg tttaggagat ttatttatag tggttttttt aaagtgttgg gattataggt 5220
atgagttatt atatttagtt tgggtttttt tatttttgtt cgttggagat agtatgaatt 5280
tatatttttt tatttatgat tttgttattt tgggatagtt atttaatcgt tttaagtttc 5340
ggttttttat ttagaggaag ttatattata ggtgattatg aggtttaaat gggtggtgtg 5400
ggtgattgtg tttatttttt tttttttaaa gtattaatta aatgtaaaga attatgtatt 5460
tggattttag atataatatt aagatattta taattttttt tgtgtagtgt tttgagttat 5520
tttttaatag tatttttagg agatttggta attggtagta gaaggatagg aatttttaat 5580
ttttttaaag gtatttttta ttttttattt ttatgtttta tattttagat ttgtggttta 5640
tatagtggtt attgtatatt tgaaatgtgg ttggtgttaa cggatatgtt ataagtaaaa 5700
agtatatatc gtattttaaa aatttgttat gaaaaaaatg taaagtattt tattaataat 5760
ttttatattg attatatgtt taaatgataa tattttggaa atatagttat attaaataaa 5820
atttaaataa ataagttaat taaaaaataa atattggtta ggcgcggtgg tttacgtttg 5880
taattttagt attttgggag gttgaggtgg gtaaattacg aggttaagag gtcgatatta 5940
ttttggttaa tatggtgaaa tttcgttttt attaaaagta taaaaattag ttgcgtgtag 6000

tggcgcgcgt ttgtagtttt agttatttag gaggttgagg taggagaatt gtttgaattc 6060
gggaggtaga ggttgta 6077

<210> 95
<211> 9018
<212> DNA
<213> Artificial Sequence

<220>
<223> chemically treated genomic DNA (Homo sapiens)

<400> 95

tttgggaggt tgaggtagga gaattatttg aatttaggag gtagaggttg tagtgagtta 60
agattacgtt attgtatttt ggatgataga gtaagatttt attttaaaaa aaattaaaaa 120
ataaaaataa aaataaagat aaaaataaaa taattaaaaa aattcgaata aataaaaaaa 180
taaaaataaa gaaaaattaa gtgtttgtta cgttttaatt tattttattt ttttagtgat 240
tttattaaag atttaggaga ggtcgggtgc ggtggtttac gtttgtaatt ttagtatttt 300
gggaggtcga ggcgggtgga ttacgaggtt agtagtttaa gattagtttg gttaagatgg 360
tgaaatttta tttttattaa aaatataaaa aattagttgg gcgcggtggt aggtatttgt 420
agttttagtt atttaggagg ttgaggaagg agaattattt gaattttgag ggtggaggtt 480
gtagtgagtc gagatcgtgt tattgtattt tagtttgggt gatagagtga aattttgttt 540
taaaaaaaaa aaagaaaaaa aaaatttagg aaataagaat tattattttt tttattttat 600
agttgagaaa atggaaaatt tagataattt aagtaattgg tttgaggtta tatagcgttt 660
gtttgtttgt ttgttttttt tgagacggag tttttttttt gttatttagg cgggagtgta 720
atggtacgat ttcggtttat cgtaattttt gttttttagg tttaagtgat tttttcggtt 780
tagttttttg agtagttgag attataggta tgtacgatta tgtttagtta attttgtatt 840
tttagtagag acggggtttt tttatgttgg ttaggttggt tttgaatttt tgattttagg 900
tgatttattt gtttcggttt tttaaagtgt tgggattata ggtgtgagtt attatgttag 960
gttttttttt gttgtttttt gagagggaat tttgttttgt tatttaggtt ggagtgtagt 1020
ggggcgattt tagtttattg taatttttgt ttttcgggtt taagcgattt atatgtttta 1080
gtttttagag tatgtgggat tataggtatg cgttattata agcggttaat ttttgtgttt 1140
ttagtggaga cggtatttta ttatgttggt tagtttggtt tcgatttttt gtttttaagt 1200
gattcgtttg tttcggtttt ttaaagtgtt ggaattatag gcgtgagtta ttgcgttcgg 1260
ttataggtta taaagttatt gagtaaagaa agttgtattt agttttttat tttaagaaag 1320
ttattttttt ttgttttaaa gtgaggttat atttttagaa ggattttgtt ttcggggaag 1380
ttttggtttt ggaatttagg tttagtttta agtttaaaat tattgatgtt gtttagtagt 1440
gggattaatg attttgtttt atttaaatgt tataagtttt ttgaagatgg gttggagttg 1500
ttagggtatt tagttttttt ttttatttag aagtaaatat ttttattata gtttttcgat 1560
tattttatat ttttttattt gagtgttttt tttatgtcga gggtattatt gagatgtagt 1620
ttagagaatg aaatttttgg tgttgtaggg agtttagatt tattcgggaa ggtttatgag 1680
tattttatgt tagtagtttt tattttattt aacgttttta tagagtgaag gaatagttat 1740
ttattataaa ataaattatt aaaagaaaaa ttttaaattt ttaaaggaga gaggtttatg 1800
gtagtgagcg tttttatttt tttttttttt ttttttttat tattaacggg ttagtagtat 1860
aatttgtttt ttgaaatttt tgggggtatc ggagttttgg tgtttttatt tttttttttt 1920
tttattgatg tattattatt agagtgaaaa aagaagggcg tttcgagggt ttttgaaaaa 1980
aaaaaagggt gattatttaa gatattttgt aaagggggga aaattgcgaa gaaaagagat 2040
tttagtaggg ggtggaggaa ttgtgaatgt gtttttgttt ggcggttttt ttagttgaaa 2100
tttggtgttg gcgttcgtag tttagtttaa attgttattt ttagttttta ttggtttttt 2160
tgtttttgtt ttagaatttt aatagattgg ggtttattta tttatttata tttcgtcgtg 2220
gaagtttatt aataggtttt attagtaata ttatttatgg gaggttattg acgaaagggg 2280
ttttagttat ttgaagaggg aagagaaaag aaaaaattgt aaagagatta tttttttatt 2340
tagaagtttt agtggtgtga aggttttttt ggatggtttg aggttatttg aagattaaat 2400
atggggatat aattgatttt ttaaaaataa ttttaaatat attttttttt ttttttaaaa 2460
ttgaagagtt ttttgttgtg gttttgtggg ttttagtttt ttatatatga gtttttttta 2520
gggaataagg agtttttgat agattggtgt ttttttattt ttataaaaaa tatattttgt 2580
aaattgtgtt tggatatgaa agtaggtgtt tttatttgtt atttttagcg gaaagttttt 2640
tttttattta atatgtataa agttattgat gaatggggga tagggatagc ggttttttgt 2700
attattaatt tatgtgagaa tgtgttaata gtatttttaa tggttgatgg atgcggtgta 2760
ggatgggaaa ggaattgttt tgagatattc gagtttttta ttggtgggga atataaagat 2820
gtaagtggta gtttgcgagt aggtagatac gtttagttgt attttttttt tgagaatttg 2880
aaaatatatt gttattaaaa taattcgtgt gggagtgaat ggttaagtgt taatttgggt 2940
tgaggtgttc gttaatgtat aggttttggg gttaatttag gttagaggat ttgattaaaa 3000
atatattttt ttattaagta tatagaggga taattttgtt taagaattag tttttatttt 3060
gttgttatta aatttttaaa atataatgtt atagtttttt gtttgaggta gttaaatgga 3120
ttttttattt gtgtggtttt taaataattt gtataaacgt tttttgtgtt atatttgtga 3180

ataaaaatga ttttttaatt atgtagtatt ttgaaaaata tttttgtaaa tgttatgaag 3240
ggtgtaatgt tagtgatata ggtgtaaata tgtttttaaa tttaggaaaa gtttgttttt 3300
taagttaggt ttgtttttaa tgttttattt atttttaggt tttaaaagga ttttaaagta 3360
tttttgggtt tagtgatttt ttttaaaaaa aaataattaa ggttttaatt tatatggttt 3420
tgaatttaag tacggttgtt taggagttgg atgaataagg agggagaaaa agtatattta 3480
aaataaatta tttcgaagat acggtttagt gaaaaggtaa atggatatag gaattttaat 3540
tagttatagt ttggtgtgta gttgaagaat tagattttgt atttatgatt tttaatttaa 3600
gatttagagg gaggaattat tttgtttaag gtttatttat ggtagagttg tttttagatt 3660
tttttgaatt tttttttatt aatttaaata tatattatta aatatttttt ttttttgatg 3720
taattttggt tttttttgag agagttatat ttaaagttag gttgttttag tagttagttg 3780
tgtggtttta ggtaaattat ttaatttttt tgattttgtt tttttattag aaattgggaa 3840
tagtattttg tagggtggtt gggtagatga aatagaatta tttatgtagt gtgatttttt 3900
tatagtaggg tttttattaa tatttagttt ttttttttta tttttattgt ttagaaggat 3960
agaaatatgt aatgttttgc gaatattttt aagtggtttt ttttttttta aaagttttag 4020
ttgtttggta atttttttcg tagtattaag taggcgtatt ttaaagtttt tttagcggga 4080
gggtagggtt agagttttcg gttatttgtt ttttaatttg atataaaata acgtaacgta 4140
gttatttagc gagtcgttgg ttagtttcgt tcgttcgggg aggaggaggg aggtggcgcg 4200
gggcgcggga ttaggtttta ttcggaggtt gtcgttgtat ttttacggtc gtatcgcggt 4260
cgagttttcg tttttcgggt cggtgttttc ggttgttcga aggcgttcgg agtcgcgcgg 4320
aattcggggc ggttcgggcg cgcggcgttg attggtagag agggcgtcgt cgtttaggaa 4380
acggttcggg ttttagtggg ggcgtgattt atcgcgagaa ggcgggggcg gcggcggcgg 4440
cgcgggcggc ggttggagga gagcgcggtg gagagtcgag cgggcgggcg gcgggtgcgg 4500
agcgggcgag ggagcgcgcg cggtcgttat aaagttcggg cgtcgcgggg ttgtatgcgg 4560
cgtatttggt tcggcgcggc gattgttttt cgggttggcg ggggtcggtc gcgagtttcg 4620
ggggtttcga ggtcgtagtt tgtttgcgcg ttttgagttt tcgtaattcg cgagtaaagt 4680
ttggtggagg taacgttaag tttgagttag ttattttttt cgtttcgcga gttagagggt 4740
aggtttcggg cgttatgttc gcggtttttt atagttttag tgcgtatagc ggtgtagtat 4800
ttcggggaaa taaattcgag gtagttcgcg ggcgttatgt tcgcgttttt tcgtagtttg 4860
gggtacgcgt gaagttcggg aggtttggcg tcggcgaaga tttaaggatt atttttttgc 4920
gtttggagtt gtttttcgta atttcgggtt cgtcgttttt tttatttcga tttacgcggg 4980
gcgcggggat aatataggtc gcggaggagc gttgttattt aaggtaatcg tcgcgtaaga 5040
cgtttcgggg agtttcgtta gtcggggacg tgggcgttac gggagttcgg gacgtcgggt 5100
gtatcgtttt tcgggcgggg ggcgcggaag gattagtatt gtggaggacg tttcgtgttt 5160
tttttttgtg gttgtatagg tgatggggga ggtgggtgcg tgttgacggt cggcgttttg 5220
gaagttttgt ttttgttatt tttatttaga tgttgatatt tgtttttggc gttgacgttt 5280
ttttttttgt taaattttgg cgggcgtatt ttcgttgatt gggcgcgttt tttcgatttt 5340
agagtagacg ggcggaaggt tcgtttgttc gtggtatagt ttcgtaggtc ggttttcggg 5400
gttattttcg aggtgtttta ttcgtgtgtt ttgggaattt tcgtattatt taggttttgc 5460
ggagattttt ttttcgggag ggggtcgttg gggtggggtc ggtgatattt cgtaggggtg 5520
gcgatggtta ggggtggtat tgttggggag gcgacggttt cggtgtttgg gcgtttttcg 5580
ttcgtagtcg tcgtcggcgt tggagggagc gtagtgcgtt tggggttagg gggcgaattg 5640
gatcgatttt ttttagttcg tttgtttgtt ttgtcgtagc ggttgggaga tgtcgaaagc 5700
gtaggcgagt tttaatttgc gcgtttattt ttttcggtta cggggtcgcg ttggggaaaa 5760
aagtcgaggg ttttgcgtgg cgttggtttc gattttcgcg gattacggat ttggcgtagg 5820
aggttcgttc ggggattatt agttttcgtc gtttttttcg tatttcgttt ttttgtagat 5880
cgttttttag attttttttt ttttttttta atttaataaa atttattttt aaaggtaaat 5940
ttgtttttaa atttaggtta tttattatgt gtttggtgtg aaacgttatt aatatttaaa 6000
attttattgt ttttttggtt ttaaattttt gtaaattttt tatcgggttc gatttatttt 6060
tatttgaaaa gtttgtttag tttgaataga aaagtaatta ggcgtttttt tttttttttt 6120
tggaatgtta attaaaatgt agattttttt gagtttttta gcgtttcgag aagggggaga 6180
aaaataggat attttaggta aataaatgaa agaagtgttg ttttgaaagg gggtggtggt 6240
ggggagtatt tttaagttgt tcgtaagttt tgattggacg taagtattaa atcgggaggg 6300
ttttgtggtt ttgggttagt gtgtgttttt tgagatttta atttgttgag gaattttttt 6360
ttagttttga ttttttgata gttttcgttt ttatttagtg ttggggagaa gtagggaggt 6420
tttaagcgaa gagatgggtt tgtattttgg aggagtcgga tattgttgat ttttttgatg 6480
tgaaatttat ttaggaataa aatattaggt gattttagtg gtcgagttag tagtggatag 6540
attatttttt aggtggaagt ttaaagcgga gggtattgta ttggtttgtt tttttggagg 6600
gcgggggttt tgggaatgtt atggtaaatt gaagagttta gtataggttt ggtatgggga 6660
tcgtttcgta gttaagtttt ttttgtttgt tattttgttt gtttttgggg gtaggtttgt 6720
gtcgaggtga aaatggatcg gggggcgggc ggtcgagaag agttatttat tagagggagg 6780
gtgataattt ttttcgcgtg taggttgaga agagagtttt tttttaaggg gaaaaataaa 6840
tacgttgggg tttttattgg ggtttagatt ttaggaagga ttatggtatt gaaggtagga 6900
agtcgggatt gtggtcgtta gcggtatgtt gggtttgtat ttttaatatc gagtttgggg 6960
atttaattat tttgtttagg aggtcgtatt atatttttgt ttattggtgt gaggaattgt 7020
gtagatttgt cgttttaggt tttcgttttt tagagttttg gggagggggt tgtcgtgggg 7080
tttggatttg gaaatggttg aaatgtaaat ttttagtgtt agttagcggg atgatggaac 7140

ggtttgagat ttagcggagc gttttgttta tttttttttg tttggagtta aggaggtttt 7200
tttgggtagg gataggtggg cgagtaggtc ggggaaggag tttggggtga aatttttgcg 7260
aggatttagg attgtttttg aagtcgtgtt agaggtagaa aatttttttg tattttgtaa 7320
atgtttagta aattaatgag aatgtttgtg tatttttttt ttatatgtat gtagaaatag 7380
attcgaaatt attttttgag gtttttgatt aagaggaaag aaaagttaag aggaaaaatt 7440
ttttaagtta tttgaaagta tatgtatata tcgtgtacgt atatattatg tatatatata 7500
tatcgtgtat atatatatat tatgtacgta tatatatcgt gtatatatat atatattgta 7560
tatatatttt ttttgttttt gttgtgaatt atatcgtttt atttgtgtta tttttgaagt 7620
agaggttttt tttatttttt ttttggaaat gatgtggtat attttttttt tttttttatt 7680
tttgttttta gttaaagtta aaaggagtag tttttatgat gagatttggg gaggttttag 7740
tagtgttgaa attttatgat ataatttgaa atatcgagtg ggtatataaa aaatgtagag 7800
tttagagatt tttgtattaa aggttttttt gttattttta gttttagaaa atgtggttta 7860
tttttgtggt ttatagaatt tgaagtttga gattgatgag tttttttttg tgattgtttt 7920
aattattttt taaagtttgt gtattttatt gtatttttag ttatagagag tattgagtga 7980
atttaaagtt gtttggaata tattaagttt ttttggaaaa tttttttttt tttggattaa 8040
atgggtgaat agaatattaa gttgagtgtt tttttttaat ttatattttg aaaatttagt 8100
tagtaggttt gtatatggaa agttattggt ttaattatat attatgatta taatttatat 8160
aatttatatt attattatta ataaattttt gtattattta ttagaatttg tatttgttta 8220
gtttttttat ttttttatag aggttgaaga tatttagtag ttttggattg ttatttagtt 8280
ataggggaag ggaatttagg tttcgaagtt ttttttggat tagttgcgtt gaatgcgttg 8340
tagttgtgtt tatttttcgt aagtggttgt ttggcggaag attgaaagat atttttttat 8400
ttgaaaatga attttgataa gtgtaaattg gtgggaattt tttgtagttt ttttgggatt 8460
tttttgattt tgttggtttt tttttttttt aagagcgata gtgagggtgg gagattgttt 8520
atttttagtt tagtagaaat acggagttgt gagtagattt ttttaatagt ttagtttttt 8580
aaagtttagt tggattaaga gaagggaacg aggttggtta tgttaattcg agtattttta 8640
atttgttttt gtagttgttt ttgaaattaa ggttacgttg tttttgttta ttgagggcgg 8700
tggaggcgat agtggatagt agaagagagt atatattgtt ttattgaagt ggttgtacgt 8760
atttgagttt tgtagttatt gttttatttt tgttttttaa aagtatgttt ttaaaaagat 8820
tagttttata tatttttgtg gatcggtttg gtggtattat ttgggatttt gaggtgagga 8880
tggaaggatt tagtagataa tgaaaaagaa ttttgtttgc gtatattgaa gaggttgaaa 8940
aatggtttta ttttatttgg gttggagtga tttggtattg ggaagatttt tttgattcgt 9000
taattttttt tttttagt 9018

<210> 96
<211> 9018
<212> DNA
<213> Artificial Sequence

<220>
<223> chemically treated genomic DNA (Homo sapiens)

<400> 96

attaaaggaa agagattggc gagttaggga atttttttta atgttaaatt attttagttt 60
aagtgggata aaattatttt ttagtttttt aaatgtgcgt aaatagagtt tttttttatt 120
atttgttaaa tttttttatt tttattttag agttttaggt gatattatta gatcggttta 180
tagaaatgtg tgaggttaat ttttttaaaa gtatattttt aagaaataga gataaaatag 240
tagttatagg atttagatac gtgtagttat tttagtgagg taatgtgtgt ttttttttat 300
tatttattat cgtttttatc gtttttaatg agtagaagta acgtgatttt agttttaagg 360
gtaattataa aagtaagtta aaaatgttcg ggttggtata attaatttcg tttttttttt 420
ttagtttaat tgggttttag aaagttaaat tgttggagga gtttgtttat agtttcgtat 480
ttttgttggg ttggaggtga gtagtttttt atttttattg tcgtttttgg gaagaaagga 540
gattagtaaa attaaaagaa ttttaggaag gttatagaaa atttttatta gtttatattt 600
gttaggattt atttttaaat ggagaagtgt tttttagttt ttcgttaagt agttatttac 660
gaagagtgaa tataattgta gcgtatttaa cgtagttgat ttaaaaagga tttcgaagtt 720
tgaatttttt ttttttgtga ttaaatggta gtttagggtt gttaaatgtt tttaattttt 780
gtagaagggt gaggaaattg agtagatgta aattttgatg aataatgtag gaatttatta 840
ataatgatga tgtaaattat gtaaattata gttatggtat gtgattggat taataatttt 900
ttatatgtaa atttattggt taaattttta gagtataagt tagaagagga tatttaattt 960
aatattttgt ttatttattt gatttaaggg aaagagaatt ttttaagaaa gtttaatata 1020
ttttaagtaa ttttaaattt atttagtatt ttttgtgatt aagggtatag taaaatgtat 1080
aaattttaga gaatgattaa aataattata agaaggggtt tattaatttt aggttttaga 1140
ttttgtgaat tataggaatg aattatattt tttaagattg ggggtggtag ggaggttttt 1200
aatataaaaa tttttaaatt ttatattttt tatgtattta ttcgatgttt taagttatgt 1260
tataggattt taatattgtt ggagtttttt taaattttat tataaaaatt gtttttttta 1320
gttttgattg aggataaaaa tggaggaggg aaaagaatat gttatattat ttttaaagga 1380

ggaataaaga aaatttttgt tttaaagata gtataggtga aacggtgtga tttatagtaa 1440
aagtaagaaa agtatgtgtg tagtgtgtgt gtgtgtatac ggtgtgtgtg cgtgtatggt 1500
gtgtgtgtat gtacggtgtg tgtgtgtata tggtgtgtgc gtgtacggtg tgtgtatgtg 1560
tttttagata gtttgaaaga tttttttttt taattttttt ttttttttag ttaagagttt 1620
tagaagataa tttcggattt atttttatat atatgtggga ggaaagtata taaatatttt 1680
tattgattta ttaagtattt gtagaatgta ggggaatttt ttatttttaa tacgatttta 1740
ggaataattt taagttttcg tagaggtttt attttaggtt tttttttcgg tttgttcgtt 1800
tatttgtttt tgtttagagg agtttttttg gttttaggta gagaaaggtg gatagggcgt 1860
ttcgttgaat tttaagtcgt tttattattt cgttagttgg tattagaaat ttgtatttta 1920
gttattttta ggtttaaatt ttacggtagt ttttttttta aaattttgga aggcggagat 1980
ttaaggcgat aggtttgtat agttttttat attagtggat aaaagtatgg tgcgattttt 2040
taggtaggat aattaggttt ttaggttcgg tgttgggaat ataggtttag tatgtcgttg 2100
gcggttataa tttcggtttt ttgtttttaa tattataatt ttttttggag tttgagtttt 2160
agtgaaagtt ttagcgtgtt tatttttttt tttgaaaggg aatttttttt ttagtttgta 2220
cgcgggagga gttgttattt tttttttgat ggatggtttt tttcggtcgt tcgtttttcg 2280
gtttattttt atttcggtat aagtttgttt ttaaagatag gtagagtgat aagtaggaag 2340
ggtttggttg cggagcggtt tttatgttag gtttgtgttg ggttttttaa tttattatga 2400
tatttttaaa atttcgtttt tttaagaagg tagattaata tagtgttttt cgttttgggt 2460
ttttatttaa ggggtgattt gtttattgtt ggttcggtta ttgggattat ttggtgtttt 2520
gtttttgggt agattttata ttaggaaagt taatagtgtt cggttttttt aaagtgtaga 2580
tttatttttt cgtttaaggt ttttttattt ttttttagta ttgagtagga gcgggagttg 2640
ttaaggggtt aaggttaggg ggaaattttt taataaattg aaattttaaa aaatatatat 2700
tgatttagga ttataaagtt ttttcggttt aatgtttgcg tttaattaga atttgcgagt 2760
agtttggggg tgttttttat tattattttt ttttagggta gtattttttt tatttgtttg 2820
tttgaaatat tttgtttttt tttttttttt cggggcgtta aagagtttag agaaatttgt 2880
attttaattg atattttagg gaaagagaga ggggcgtttg attgtttttt tatttaaatt 2940
aaataggttt tttagatgaa aatgagtcga gttcggtgga agatttatag ggatttggga 3000
ttagggagat aatggagttt taaatgttga tagcgtttta tattaaatat ataatgggtg 3060
ttttgagttt gaaagtaggt ttgtttttaa gggtaggttt tattgaattg ggagaaagga 3120
aggagggttt ggagaacggt ttgtaaggga gcggggtgcg aggagagcga cggaggttgg 3180
tggttttcga gcgggttttt tgcgttaggt tcgtggttcg cgagggtcgg agttagcgtt 3240
acgtagggtt ttcggttttt ttttttagcg cggtttcgtg gtcgaaaaaa atgagcgcgt 3300
aagttagaat tcgtttgcgt tttcgatatt ttttagtcgt tgcggtagag taggtaggcg 3360
aattagaaaa agtcggttta gttcgttttt tagttttagg cgtattgcgt tttttttagc 3420
gtcgacggcg gttgcgggcg aggggcgttt aggtatcgga gtcgtcgttt ttttagtagt 3480
gttatttttg gttatcgtta tttttacggg atgttatcgg ttttatttta gcggtttttt 3540
ttcgaaaaag gggttttcgt agggtttgga tggtacggaa gtttttagga tatacggatg 3600
gggtatttcg gagatgattt cgggaatcgg tttgcggggt tgtgttacgg gtaggcgaat 3660
ttttcgttcg tttgttttgg ggtcggagaa acgcgtttag ttagcgggaa tgcgttcgtt 3720
agggtttgat agggaggggg gcgttaacgt tagaagtaga tgttagtatt tggatggggg 3780
tagtagaggt agaattttta gaacgtcggt cgttagtacg tatttatttt ttttattatt 3840
tatgtagtta tagagggagg atacggagcg ttttttataa tgttagtttt ttcgcgtttt 3900
tcgttcggag gacggtgtat tcggcgtttc gggttttcgt ggcgtttacg ttttcggttg 3960
gcgaagtttt tcgaggcgtt ttgcgcggcg attattttga atggtaacgt tttttcgcga 4020
tttgtgttgt tttcgcgttt cgcgtgggtc gggatggaga aagcgacgag ttcggggttg 4080
cggggagtaa ttttaaacgt agaagagtgg tttttgggtt ttcgtcggcg ttaagttttt 4140
cgggttttac gcgtatttta ggttgcggag gagcgcgggt atgacgttcg cgggttgttt 4200
cggatttgtt ttttcggggt gttgtatcgt tgtacgtatt agggttgtag gggatcgcgg 4260
gtatgacgtt cggggtttgt tttttgattc gcggaacgag aggaataatt gatttaggtt 4320
tggcgttgtt tttattaaat tttgttcgcg agttgcgaag gtttagagcg cgtaggtaag 4380
ttgcggtttc ggggttttcg gggttcgcgg tcggttttcg ttagttcgga gagtagtcgt 4440
cgcgtcgggt taggtacgtc gtatgtagtt tcgcggcgtt cgagttttgt ggcggtcgcg 4500
cgcgtttttt cgttcgtttc gtattcgtcg ttcgttcgtt cggtttttta tcgcgttttt 4560
ttttagtcgt cgttcgcgtc gtcgtcgtcg ttttcgtttt ttcgcggtgg gttacgtttt 4620
tattgaaatt cgagtcgttt tttggacggc ggcgtttttt ttgttaatta gcgtcgcgcg 4680
ttcgggtcgt ttcgagtttc gcgcggtttc ggacgttttc gagtagtcgg agatatcggt 4740
tcgggaggcg agggttcggt cgcgatgcgg tcgtagggat gtagcgatag ttttcgaata 4800
aggtttggtt tcgcgtttcg cgttattttt tttttttttt tcgggcgagc gaggttggtt 4860
aacggttcgt tgagtgattg cgttacgttg ttttatgtta agttagaaaa taagtgatcg 4920
aaggttttaa ttttgttttt tcgttaaaga gattttaaaa tgcgtttgtt tgatattgcg 4980
gggagggttg ttaggtagtt gaagtttttg gaggagagga agttatttgg aaatgttcgt 5040
aggatattgt atgtttttgt ttttttaaat aatagaaatg agaggaaaaa attgaatatt 5100
aatggaggtt ttattatggg agggttatat tatataggtg attttatttt atttgtttaa 5160
ttattttgta gagtattatt tttagttttt gataaggaaa taggattaga gaggttaagt 5220
aatttgttta ggattatata gttggttatt aggatagttt gattttgaat gtggtttttt 5280
tagaaaaagt taaaattgta ttaaaaaaaa aaagtgttta ataatatatg tttggattaa 5340

taaaaagaaa tttaaaggag tttaaagata attttattat aaatagattt tgaataaagt 5400
aatttttttt tttgaatttt agattaaaaa ttataaatgt aaaatttgat tttttaattg 5460
tatattaagt tgtaattggt taaagttttt atgtttattt atttttttat taagtcgtgt 5520
tttcggaata atttatttta aatatgtttt tttttttttt ttatttattt agtttttgag 5580
tagtcgtgtt taagtttaag attatataga ttaaaatttt aattattttt ttttagaaaa 5640
ggttattaaa tttagaaatg ttttagaatt tttttgaggt ttgaagatag atgaagtatt 5700
gagaataaat ttgatttgga aaatagattt tttttggatt tagaaatata tttgtatttg 5760
tgttattaat attgtatttt ttataatatt tataaaaata ttttttaagg tgttatatga 5820
ttagaaaatt atttttattt ataaatatag tatagaaaac gtttatgtaa gttatttaaa 5880
agttatataa gtaagaaatt tatttaattg ttttaggtaa aaggttataa tattgtattt 5940
tggaaatttg atagtaataa aataaaaatt ggtttttaag taaaattatt tttttatatg 6000
tttggtggga gggtatgttt ttggttaagt tttttaattt ggattagttt tagagtttat 6060
atattaacgg atattttagt ttaggttgat atttagttat ttatttttat acgggttgtt 6120
ttgatagtag tatgttttta agtttttaga aaagggatgt agttgaacgt gtttgtttgt 6180
tcgtaggttg ttatttatat ttttatattt tttattaatg ggaaattcga gtgttttagg 6240
atagtttttt ttttatttta tatcgtattt attagttatt gaaaatgttg ttaatatatt 6300
tttatataaa ttaatggtgt aggaagtcgt tgtttttatt ttttatttat taatagtttt 6360
atgtatgttg aatggaaagg aagtttttcg ttgaaaatga taaataagaa tatttatttt 6420
tatgtttagg tatagtttgt agaatgtatt ttttatagaa atgaaaagat attaatttat 6480
taaaagtttt ttatttttta aagagggttt atatataagg gattgaaatt tataagatta 6540
taataggaaa ttttttagtt ttggggaggg ggggaagtat gtttagaatt atttttaaaa 6600
aattaattat gtttttatat ttagttttta agtgatttta gattatttag gaaaattttt 6660
atattattga ggtttttgaa tgagaaaata atttttttat agtttttttt tttttttttt 6720
ttttttaagt ggttggaatt tttttcgtta atggtttttt atgaatggtg ttgttaataa 6780
agtttattaa tgagttttta cggcggagtg tgaataggta aataagtttt aatttgttga 6840
ggttttgggg taagggtaga aaagttagta agaattaaag gtgatagttt gaattgaatt 6900
gcgaacgtta gtattaggtt ttagttgaaa gaatcgttag ataaggatat atttatagtt 6960
tttttatttt ttattgaggt tttttttttt cgtaattttt ttttttttat aaaatgtttt 7020
gagtagttat tttttttttt ttttaaaagt tttcgagacg tttttttttt tttattttag 7080
taatgatata ttaatgggaa ggaagaaaaa tgaggatatt agaatttcga tgtttttaaa 7140
agttttaaga aataaattgt gttgttagtt cgttggtgat gggaagagaa aggagaaggg 7200
ggtgggagcg tttattatta tgggtttttt ttttttgaag atttggaatt ttttttttaa 7260
taatttgttt tgtaatgggt ggttgttttt ttattttatg gaaacgttag atgaaataaa 7320
ggttgttgat atgaagtgtt tatgaatttt ttcgggtgga tttgagtttt ttgtaatatt 7380
aggaatttta ttttttaagt tatattttaa tagtgttttc ggtataaaag aagtatttaa 7440
atggaaggat atagaatagt cggaggatta tgataagagt atttattttt gggtaagggg 7500
aggggttgga tgttttgata gttttagttt atttttaggg agtttgtggt atttaagtga 7560
agtaaaatta ttgattttat tattgaatag tattaatgat tttaaattta gaattgggtt 7620
tgaattttaa agttaaggtt ttttcggaga taaagttttt ttggaagtat agttttattt 7680
tgaggtaaag gggaatggtt tttttaaggt gagaggttgg atgtagtttt ttttatttaa 7740
tagttttgtg atttgtggtc gggcgtagtg gtttacgttt gtaattttag tattttggga 7800
ggtcgaggta ggcggattat ttgaaggtag gaagtcgaga ttaggttgat taatatggtg 7860
aaatgtcgtt tttattaaaa atataaaaat tggtcgtttg tggtggcgta tgtttgtaat 7920
tttatatatt ttggaagtta aggtatgtga atcgtttgaa ttcgggaggt agaggttgta 7980
gtgagttgag atcgttttat tgtattttag tttgggtgat agagtaagat ttttttttaa 8040
aaaataataa aaaaaggttt ggtatggtgg tttatatttg taattttagt attttgggag 8100
gtcgaggtaa gtggattatt tgaggttagg agtttaagat tagtttgatt aatatggaga 8160
aatttcgttt ttattaaaaa tataaaatta gttgggtatg gtcgtatatg tttgtaattt 8220
tagttattta ggaggttgag tcgggagaat tatttgaatt taggaggtag aggttgcggt 8280
gagtcgagat cgtgttattg tattttcgtt tgggtaataa gagagaaatt tcgttttaaa 8340
aaaaataaat aaataaataa acgttgtgtg attttaggtt agttgtttga gttatttgag 8400
ttttttattt ttttagttgt aaaatgggga aaataatggt ttttattttt tggatttttt 8460
tttttttttt tttttttgag atagagtttt attttgttat ttaggttgga atgtagtggt 8520
acgatttcgg tttattgtaa tttttatttt tagggtttaa atgatttttt ttttttagtt 8580
ttttgagtag ttgggattat aggtgtttgt tatcgcgttt agttaatttt ttgtattttt 8640
agtagagatg gggttttatt attttggtta agttggtttt gaattgttga tttcgtgatt 8700
tattcgtttc ggttttttaa agtgttggga ttataggcgt gagttatcgt attcggtttt 8760
ttttagattt ttaataggat tattaagagg atgaaatgag ttaggacgtg ataagtattt 8820
agtttttttt tgtttttgtt tttttatttg ttcgggtttt tttggttgtt ttgtttttgt 8880
ttttgttttt gtttttgttt tttggttttt tttgagatgg agttttgttt tgttatttag 8940
agtgtagtag cgtgattttg gtttattata atttttgttt tttgggttta agtgattttt 9000
ttgttttagt tttttaag 9018

<210> 97
<211> 7662
<212> DNA

<213> Artificial Sequence

<220>
<223> chemically treated genomic DNA (Homo sapiens)

<400> 97

ggagtgcggc ggtcgttttg agttcgttgg ggatttggat ggagagaaat tggggttcgt 60
tcgcgtatat atgtattatt tgtttttttc gattatttat ttttttacga attttatttt 120
tatttcggat tgttaatgta agacgatagt ttgaattttt atttattttt gtttgaatat 180
aagaagtttt gagttttttg aagtcgggta ttaaagttaa agatttattt ttcgtatttt 240
ttatggtttt tttttgtagt ttagttcggt tttgagtttt cgaaacgttt ttcgaggcgg 300
gggttttttt gttaaggggc gataggtggg ttatttcgag gcgggaacga ttaggtaggt 360
gttggttggt ttttttttgg gtgagtagtt aggggaatcg tttttttagg ggatttagat 420
gatattaaat tattttatta tataaatttt tcgtggaggt ttgtttttag gagggtaatt 480
atcggattaa tttaattatg tagataggta atatggatta ttttgttttt aaaataatcg 540
aaaattaaac gtttttttcg tttattgtag aaattttttt attaaggacg gggttgaatt 600
ggagggattg tgttgataaa ttttttattt gaaaattttt tagttaagga gtatttgttt 660
aaggtcgcgg ttatttagat gttcggtttt tatttttttt ttcgttgaaa agtcgtatcg 720
atgattaacg ttgtttgtcg gtttggtggt ttgaaaacga atgtcgggta ttgatgtaag 780
gatatttttt tttatttttg ggattttggt ttaagtaaag gaggtcgaat ggagaattaa 840
gataattatt taagtaattt aaaaagttta aagatatttt taagcggatt ttgttttagt 900
ggtttgtgat ggtagtttag tggatagttt tttagtggtt ttttggggtt aattgtttta 960
attttttatt ttgtttgttt tagtaaaggg aaagatgtta aagtagttgt ggatttgtag 1020
tttttttttt gtgtgtgtag agtttttagg aattattgtt ttagtgtagt aggagaagcg 1080
ttttttttta tgttttaagt ttgttttttt tgttgttttt tagtttttgg gttaggtgga 1140
gttttttttg aagttggttt ttatttgttt tcgttttttt attgtagttt atagttcggt 1200
ttttagggtt gaatttagag tttagaggat ggagaagtta gaaagcgggg aggtaaggga 1260
gagggcgttt aggtatatta tagaggtgat tgggttgtga cggggataat tcgtataata 1320
cgagttaagt ttatattttt agggagtgat tttttcggat tgtttttatt ttaattttta 1380
ttttttttgt tttatttgaa aaagaaaaag aaaaaagaga gttttttttg gtttttggtg 1440
atttatattt aggatattat tttatttttt agtatatata tttttatagt gttatttggt 1500
ttttaattcg agtatttttt ggagtgttcg ttatattttt ttttttggtg tttttttatg 1560
gtggggttag gggaggtttt gttttgaata tcgttgtgac gttatttttt ttttttcgag 1620
ttaagtattt ttcggtgggt tttgggaatt gttgttcggt ttgttgggga ggggcggagt 1680
gtgggtttag ggagggtttt attaaatatt tattatcgtt gtcgtttgcg gggtttttag 1740
gttgtaagta cgtgaggtgt tagaaaagaa gtcgaagggt tttcgtgggg gtttttttgt 1800
taaatgagtt aagagatgtg cggaagcgga tgattgaagt atttcgtgga gtagtagaaa 1860
ggggtattaa aaattgtaat ttgaaaataa aagttttaat ttgtttttta aaagatttta 1920
tgtttgcgtt ttaatgttta aataatatta ttaataataa aaatgaaata agaaaagtgt 1980
ttgaaatata attaagtcgt ttattggagg tttgttttcg agtaagttta taaaatataa 2040
atgtaaatat ttttttttgg cgaatattta gagttaaatt agtggaaagt tgattttgat 2100
aaaattacgt ttttttattt tttttcgtac gttagatatt aattttataa cggcgatttt 2160
tatatttaaa acggagagtt gagaatatcg ttgtaggtcg atttgtaatt tatataataa 2220
gttgtttata ttttattggg tcgatggagg gaggaggtag ggaagagtag gaggcgatag 2280
gttggtgggg gagggggtag cggggtatta ttgtttgttg taaaattcgg ggtcgaaggc 2340
gtttttcgta ttcggaaaat aaagtattcg taatcgttgc gtttattttc gcgtatcgtt 2400
agtagtgtaa aaggtagttt taagatcgaa tttattattt gttgaggtgt ttagggagat 2460
tatttaaatt ttttttaggt tgaagttata gtatttttta aagtttcgat taggcgggtt 2520
gataagattt ttagggtgtc ggttgggggg ttgaatagga attaagtgtt taggttttgg 2580
ggttttatta ttattttcgt ttttgtttag tgtttttggt aattagtaag tgttgagttt 2640
tttgttggtg tttttttaat ttattagtga tttggttcgt aaattatttg gaaaaggagt 2700
ttttagagat ttcggcgtat agtaggttag gaggtagttg tttcgggatt tagagtaatt 2760
tcggggtaat tttttttttt ttaaaggtta attttagaag aggtaggttt tttttttttc 2820
gatgttaatg tttattatga aatagtaatt tttaaatatt taggtgatac ggagatgcgt 2880
ttgttatacg ttagcgtttt ttatatgtaa tagtcgaagt tgtaatttga atttttttgt 2940
taacgtgagg aagtcgggat tcgaatcggg gagataaaag gggtttcgaa tatgtttttg 3000
gatttaatag atggtcggaa attaggggta gcggagttgt taaatgcgtt acgtacgttt 3060
tttcgtagtt ttttgagttc gttatttttt ttagttttta gtgaacgatt tgggatttag 3120
ttttcgggag ggtttttttt taagtcgtta aaatgtagaa tcgtcgtgtc gggggaggga 3180
ggtttggttt tttttcgttt tttcgttttt tttttttatt tttttttttt ttgttttatt 3240
ttaattattt agcgacggcg aattttaggc gtgtgtataa cgagcggggg ttttttcgtg 3300
cgtgtttttt tagaattttt gaatcgtttt ttaggtgcga ttttggacgt ttttttagat 3360
atattcgttg tttttgcgag tcggtagttt cgcgtatgtg gagatggaga ttggtttggg 3420
gaagcgttaa gtttttagat atatttattt gttttttgtt attatatttg ttaatgttat 3480
cgagttcgta gtttgatttt ttatttgagt cgaaacgggt ttgacgggat tagagggggc 3540

ggcggcgcgg cgagtcggtg ggattttcgc gggcgggtag gggagcgcgg ggtagtagtc 3600
gggtttcggt agttttcgtc gttcgtattt ttaggtcgtt aagggtcggt ttttcgggga 3660
ggggggcgag aagtagggcg gttcgtagcg gggcgggttt atttttcgcg ggtgaacggt 3720
cgtcggaaat ttttagtagg gtttcgtatt ttttgttttt atttcggttg gggaggggtt 3780
atggaggggg gttagatggg cgacgaattt ttttttttat cgcgagtagg gtttggttgt 3840
gatcgagttt ttaaaattat gttttttgta gtcgagttta ggataggaga atttttaagt 3900
ggagaaaagt cggagacgtt agtgattcga gtgagattgg cgttggagaa gggagggaag 3960
gggttgcggg aggggagagg cggcggggag gagaggttgg ggtaatttta gcgttggcgt 4020
cggcggggtt ggcgaaggaa tgagagcgag ttagtacgag gcggggcgga gacgttaggg 4080
ttattcggga gtcgtgggcg aaggaagggt ttgtacgggg tttcgggagg atttaggaag 4140
tttgcgcgaa cggcgggcgt gggttcggag ggttcggggt gggaggtttg tcgggcgtcg 4200
gcggtaggta ggtggattgc ggggggcgaa tcgacggcgg gtcgggcgtc ggcgcgggcg 4260
tagggtcggg gttcgggtta gggcggtcgg cgcgcgtgtg gttagtttcg cggtttttcg 4320
gttttcggga gcgggtcggt agggttcggt tttcggttta ttcggaggcg gtttttattt 4380
tttattggtt gtcgtcgttg gcggggcggg gtttcgttgt tcggaaaaaa gtgtaattgc 4440
gtaaaaaagt tttcgtttgg gtgacggatg tttaaaagtt tagaagtttt tttaatgttt 4500
ttttaagcgg ttggcgcgcg agagatcgag aaaaggtgac gcggggttcg ggtaggcggt 4560
cggcgcgcgg tttttttttt tttcgttttg gttatttggt cgttttcgtc ggtagtttag 4620
ggtagagttt tttggaaggc gtaggtagtg tggcgagaag ggcgtttgtt tgtttttttt 4680
ttttgtttgt tttttttcgt ttgcgtttgg aagttgcgtc gcgagttttt gtaaggcggt 4740
ttgtcgcggt cgggttcggt tttttttttt cgtagcgatt tcgtttcgcg gtcgcgcggg 4800
tttcgaggta gttcgaggcg tcggaggagt tagttttagc gagcgtcggg agaggcggta 4860
gcgtagtcgg acgtatagcg tagcgggtcg gtattagttc ggtcgggttc ggattcggat 4920
tcggcggtcg gcgcggcgcg gttcggttcg agcgagggtg gggggcggcg ggcggcgcgg 4980
ggcggcggcg agcgggggtt atgtaggcgc gttatttcgt gtttagtttt aattttttgg 5040
gagtggtgtt ttatttcggc ggcgagtaga gttattatcg cgcggcggtc gcggcggtcg 5100
ggggcggtta tatcgttatg tcggttttta tgagcgtgta ttcgtatttt gcgtacgtcg 5160
agtagtattc gggcggtatg gttcgcgttt acgggtttta tacgtcgtag tcgtagttta 5220
aggatatggt gaagtcgttt tatagttata tcgcgtttat tattatggtt atttagaacg 5280
tttcggataa gaagattatt ttgaacggta tttattagtt tattatggat cgtttttttt 5340
tttatcggga taataagtag ggttggtaga atagtattcg ttataatttt tcgtttaacg 5400
agtgtttcgt taaggtgtcg cgcgacgata agaagtcggg taagggtagt tattggacgt 5460
tggattcgga tttttataat atgttcgaga acggtagttt tttgcggcgg cggcggcgtt 5520
ttaagaagaa ggacgcggtg aaggataagg aggagaagga taggttgtat tttaaggagt 5580
cgttttcgtt cggtcgttag ttttcgttcg cgtcgtcgga gtaggtcgac ggtaacgcgt 5640
tcggttcgta gtcgtcgttc gtgcgtattt aggatattaa gatcgagaac ggtacgtgtt 5700
tttcgtcgtt ttagtttttg ttttcggtcg tcgttttggg tagcggtagc gtcgtcgcgg 5760
tgtttaagat cgagagtttc gatagtagta gtagtagttt gtttagcggg agtagttttt 5820
cgggtagttt gtcgtcggcg cggtcgttta gtttggacgg tgcggatttc gcgtcgtcgt 5880
cgttcgcgtt tttcgtttcg tcgtcgtatt atagttaggg ttttagcgtg gataatatta 5940
tgacgtcgtt gcgggggtcg tcgtagagcg cggtcgcgga gtttagtttc ggttttttgg 6000
tttcggcggt cgcgttttcg cgcgcgggga tcgtattttc gttggcgttc ggcgtttatt 6060
cgttcggtta gagttttttt tatagttttt tttgtagtta gatttttagc gcgggtagtt 6120
cgggcggcgg cggcggcggc gcgggggtcg cggggggcgc gggcggcgtc gggatttatt 6180
attgtaattt gtaagttatg agtttgtacg cggtcggcga gcgcgggggt tatttgtagg 6240
gcgcgttcgg gggcgcgggc ggttcggtcg tggacgattt tttgttcgat tattttttgt 6300
tttcggttat tagtagtagt tcgtcgtttt tgagttacgg cggcggcggc ggcggcggcg 6360
ggggaggtta ggaggtcggt tattattttg cggtttatta aggtcgtttt atttcgtggt 6420
atttgaatta ggcggcggga gatttgggtt atttggcgag cgcggcggcg gcggcggcgg 6480
tcgtaggtta ttcgggttag tagtagaatt tttattcggt gcgggagatg ttcgagttat 6540
agaggatcgg tttgaataat tttttagtga acgggaatag tagttgttaa atggtttttt 6600
tttttagtta gtttttgtat cgtacgttcg gagttttcgt ttacgattgt agtaagtttt 6660
gatatatttt taaagtcgaa ttaaatcgaa ttttaaagta ggaaaagtta aaggaattta 6720
ttaaggtaaa atcgaaatta aaaaaaaaaa atttaattaa aaaaaatttt tgagaatatt 6780
tattatatta gcgaatagaa tattttttta aaaatttagt ttattagtat tagtacgaag 6840
aaaattttat ttttttaatc gattaattta gagttatttt tattttgttt tgtttaaata 6900
aataaattcg taaattgttt tatatagaga tagtaaaatt ttggtttatt aaaggatagt 6960
gttattttag ataatacgta agtttttttt tgttttttag agatttgttt tttttttttt 7020
tcgttttttt ttttttgttt ttttttttgt tttttatttg taagatatta ttttatttta 7080
tgttgaaggg aggggaaaag ttttcgttta tgaaagtcgt ttttttttta tttatggatt 7140
tgttttaaaa tgtaaattgt aatatagtaa tttattttta atttgtagtt ggatgtcgtg 7200
gattaaacgt tagaaagtgt ttttaaaatt tgacgttaaa ttgtttgaaa ttttaaattg 7260
tgtttttttt tttattataa aaagggaaat tgtattaatt ttattttatt tttttttttt 7320
tttttttgtt gaatatattt attgtttgtt tattaataaa ttattattta gtttgaatga 7380
gatttatatg tttggatatt ttaatagagt tttaattatt acgaaaaaag attttagaga 7440
taaaatatta gaagttattt attttttatt taaatttttg aaaaatggag aaattttttg 7500

attagtttat gttaaatttt attaaaagtt tttttgttta gatttatttt tttgtagtat 7560
tttttgtaaa atgtattata tagttagttt gttttgaggt tagtaaaaag atattttttt 7620
aaatagattg gagttggtat ataaataaat acgttttttt at 7662

<210> 98
<211> 7662
<212> DNA
<213> Artificial Sequence

<220>
<223> chemically treated genomic DNA (Homo sapiens)

<400> 98
   gtgagaaaac gtatttgttt atatgttaat tttaatttgt ttagaaaaat atttttttat 60
   tagttttaaa gtaagttgat tatatagtat attttgtaga agatgttgta ggaaaataaa 120
   tttaaataaa aagattttta gtaaaatttg atatggatta gttagagggt ttttttattt 180
   tttagagatt taggtggaga ataggtaatt tttagtgttt tatttttgaa attttttttc 240
   gtaataatta aagttttatt aaagtattta gatatatagg ttttatttaa attgaatggt 300
   aatttattaa taaataaata atgaatatgt ttaataaaaa gaaagaaaag aggatagaat 360
   aagattaata tagttttttt ttttataatg agaaaaaaag tataatttaa agttttaggt 420
   aatttaacgt taggttttgg gaatattttt tggcgtttgg tttacgatat ttaattataa 480
   attaaaaata aattattatg ttgtaattta tattttaaaa taagtttatg aataaaaaga 540
   aagcgatttt tataaacggg gatttttttt ttttttttaa tataggataa aataatattt 600
   tataggtgag aggtaaggaa gaaggtaaga gaggggagac gggaggaggg gaaagtaggt 660
   ttttgaaaag taagaagaaa tttacgtgtt atttggagta atattgtttt ttaataaatt 720
   aagattttgt tgtttttgta taaaatagtt tacgggtttg tttatttaga taaggtaaag 780
   tggaggtggt tttgaattaa tcggttaaga aaatagagtt tttttcgtgt tggtgttggt 840
   gagttgaatt tttggaggga tattttgttc gttggtgtgg tgaatatttt taggggtttt 900
   ttttaattgg attttttttt tttagtttcg attttgtttt gatgggtttt tttagttttt 960
   tttgttttgg ggttcgattt agttcggttt tgagggtgtg ttaaaatttg ttatagtcgt 1020
   agacgaaagt ttcggacgtg cggtatagag attggttgga agggaaggtt atttgatagt 1080
   tattattttc gtttattgga gagttgttta agtcgatttt ttgtgattcg aatatttttc 1140
   gtatcgagtg gaagttttgt tgttggttcg ggtagtttgc ggtcgtcgtc gtcgtcgtcg 1200
   cgttcgttaa gtggtttagg ttttcgttcg tttggtttag gtattacgag gtgaggcggt 1260
   tttggtgggt cgtagggtgg tggtcggttt tttggttttt ttcgtcgtcg tcgtcgtcgt 1320
   cgtcgtgatt tagggacgac gagttgttgt tggtgatcgg aggtagagag tagtcgggta 1380
   gggggtcgtt tacggtcgag tcgttcgcgt tttcgggcgc gttttgtaag tggttttcgc 1440
   gttcgtcggt cgcgtatagg tttatggttt gtaggttgta gtggtaggtt tcggcgtcgt 1500
   tcgcgttttt cgcggttttc gcgtcgtcgt cgtcgtcgtt cgagttgttc gcgttggagg 1560
   tttggttgta gggggagttg tagagggagt tttggtcggg cgagtaggcg tcgagcgtta 1620
   gcgggggtgc gattttcgcg cgcgaggacg cggtcgtcga ggttagaagg tcggagttga 1680
   gtttcgcggt cgcgttttgc ggcgattttc gtagcgacgt tatgatgttg tttacgttga 1740
   agttttggtt atggtgcggc ggcggggcgg agggcgcggg cggcggcggc gcggaattcg 1800
   tatcgtttag gttgagcggt cgcgtcgacg gtaggttgtt cggggggttg ttttcgttgg 1860
   ataggttgtt gttgttgttg tcggggtttt cgattttggg tatcgcggcg gcgttgtcgt 1920
   tgtttagggc ggcggtcggg gataggggtt ggggcggcga ggggtacgta tcgttttcgg 1980
   ttttgatgtt ttggatgcgt acgggcggcg gttgcggatc gggcgcgttg tcgtcggttt 2040
   gtttcggcgg cgcgggcggg ggttggcggt cgggcggggg cggttttttg aggtgtagtt 2100
   tgtttttttt ttttttgttt tttatcgcgt tttttttttt gaagcgtcgt cgtcgtcgta 2160
   ggaagttgtc gttttcgaat atgttgtagg agttcgggtt tagcgtttag tagttgtttt 2220
   tgttcggttt tttgtcgtcg cgcggtattt tgacgaagta ttcgttgagc gagaggttgt 2280
   ggcggatgtt gttttgttag ttttgtttgt tgtttcggta gaaggggaag cggtttatga 2340
   tgaattggta gatgtcgttt agggtgattt ttttgttcgg ggcgttttgg atggttatgg 2400
   tgatgagcgc gatgtagtta tagggcggtt ttattatgtt tttgggttgc ggttgcggcg 2460
   tgtagggttc gtaggcgcgg gttatgtcgt tcgggtattg ttcggcgtgc gtagggtgcg 2520
   agtatacgtt tatgggggtc ggtatggcgg tgtagtcgtt ttcggtcgtc gcggtcgtcg 2580
   cgcggtagta gttttgttcg tcgtcgaggt agggtattat ttttagggag ttggggttgg 2640
   atacggagta gcgcgtttgt atggttttcg ttcgtcgtcg tttcgcgtcg ttcgtcgttt 2700
   tttattttcg ttcgggtcgg gtcgcgtcgc gtcggtcgtc gagttcgagt tcgggttcgg 2760
   tcgagttggt gtcggttcgt tgcgttgtgc gttcggttgc gttgtcgttt ttttcggcgt 2820
   tcgttggggt tggttttttc ggcgtttcgg gttatttcgg ggttcgcgcg gtcgcgaggc 2880
   ggggtcgttg cgaggggaga aggtcgggtt cggtcgcggt agatcgtttt gtaggaattc 2940
   gcggcgtagt ttttaggcgt aaacggggga aagtagataa aaagaaagaa taagtaggcg 3000
   tttttttcgt tatattgttt gcgtttttta ggagattttg ttttgagttg tcggcgaagg 3060
   cggttaaata attagggcgg gggggggggg ggtcgcgcgt cggtcgtttg ttcgggtttc 3120
   gcgttatttt ttttcggttt ttcgcgcgtt agtcgtttaa ggaagtattg ggaaaatttt 3180
   tgaatttttg agtattcgtt atttaggcga ggattttttt acgtagttat attttttttc 3240
   gggtagcgga gtttcgtttc gttagcggcg gtagttaatg ggaggtgaga atcgttttcg 3300
   aatgagtcgg aggtcgagtt ttgtcggttc gttttcggag gtcggaggat cgcggggttg 3360
   attatacgcg cgtcggtcgt tttggttcgg gtttcggttt tgcgttcgcg tcggcgttcg 3420
   gttcgtcgtc ggttcgtttt tcgtagttta tttgtttgtc gtcggcgttc ggtagatttt 3480
   ttatttcggg tttttcggat ttacgttcgt cgttcgcgta gattttttgg gttttttcgg 3540
   gatttcgtgt agattttttt ttcgtttacg gttttcgagt ggttttggcg ttttcgtttc 3600
   gtttcgtgtt ggttcgtttt tattttttcg ttagtttcgt cgacgttagc gttggggttg 3660
   ttttagtttt tttttttcgt cgtttttttt ttttcgtagt tttttttttt ttttttttaa 3720
   cgttagtttt attcgggtta ttgacgtttt cggttttttt ttatttggag gtttttttgt 3780
   tttgaattcg gttataaagg atatgatttt gagggttcgg ttatagttag gttttgttcg 3840
   cggtggggaa gaaggttcgt cgtttatttg gttttttttt atgatttttt tttagtcgaa 3900
   atgaaggtaa ggggtgcggg gttttgttgg gagttttcga cggtcgttta ttcgcgaagg 3960
   atgagttcgt ttcgttgcgg gtcgttttgt ttttcgtttt ttttttcggg gggtcggttt 4020
   ttggcggttt gagggtgcgg gcggcgaggg ttgtcggagt tcggttgttg tttcgcgttt 4080
   ttttgttcgt tcgcgagggt tttatcggtt cgtcgcgtcg tcgttttttt tgatttcgtt 4140
   aggttcgttt cggtttagat gaggggttag gttgcgggtt cggtgatatt agtaggtgtg 4200
   atgataagaa gtaaataaat atgtttaaga gtttaacgtt tttttaggtt agtttttatt 4260
   tttatatgcg cggggttgtc ggttcgtaga gataacgggt gtgtttgggg gaacgtttag 4320
   ggtcgtattt agagggcggt ttaaggattt tggggggata cgtacggaag ggttttcgtt 4380
   cgttgtgtat acgtttgaga ttcgtcgtcg ttgggtggtt ggaatgggat aggaagaaga 4440
   gaaatgaggg aaagaaacga aggagcggga aagagttagg tttttttttt tcggtacggc 4500
   gattttgtat tttagcgatt tgaggagaga ttttttcgga ggttgaattt taagtcgttt 4560
   attagaagtt gggggaagtg gcgagtttag gagattgcga agaagcgtgc gtgacgtatt 4620
   taatagtttc gttgtttttg attttcgatt atttattaaa tttaaggata tgttcgggat 4680
   tttttttgtt ttttcggttc gaatttcggt ttttttacgt tggtaggaaa atttaaattg 4740
   tagtttcggt tgttatatgt gaaggacgtt gacgtgtagt aggcgtattt tcgtgttatt 4800
   tgaatattta aaaattattg ttttataatg gatattgata tcgaaggaaa gaggatttgt 4860
   tttttttaga attagttttt ggaggaggga gggttgtttc gaggttgttt tgaatttcgg 4920
   ggtagttgtt ttttggtttg ttgtgcgtcg gggtttttgg agattttttt tttaggtggt 4980
   ttgcgggtta agttattggt gggttggaga agtattaata ggaggtttag tatttgttgg 5040
   ttgttaagga tattagatag gggcgagggt ggtggtgaag ttttaggatt tgaatattta 5100
   atttttgttt agtttttagt tcggtatttt gaaggtttta ttagttcgtt tggtcgaggt 5160
   tttaaggagt gttgtggttt tagtttggag gagatttaag tggttttttt gaatatttta 5220
   gtaagtggtg agttcggttt tgaagttgtt ttttatattg ttagcggtgc gcggagatga 5280
   acgtagcgat tacggatgtt ttgtttttcg aatgcgggaa acgttttcgg tttcgagttt 5340
   tgtaataggt aatggtgttt cgttgttttt ttttttatta gtttgtcgtt ttttgttttt 5400
   ttttgttttt tttttttatc ggtttagtga agtgtaaata gtttgttatg taaattgtaa 5460
   atcgatttgt agcggtgttt ttagtttttc gttttgggta taaaagtcgt cgttgtgaaa 5520
   ttagtgtttg gcgtgcgaga aaaaatggag ggacgtagtt ttattaagat taatttttta 5580
   ttggtttagt tttggatatt cgttaggaaa agatatttgt atttgtgttt tataaattta 5640
   ttcggaagta agtttttagt aagcggtttg attgtatttt aagtattttt tttattttat 5700
   ttttgttgtt agtggtgtta tttgaatatt aagacgtagg tatgaagttt tttggggaat 5760
   aggttaaagt ttttattttt aggttatagt ttttgatgtt tttttttgtt attttacggg 5820
   gtattttaat tattcgtttt cgtatatttt ttgatttatt tggtagaaga gtttttacgg 5880
   gaatttttcg gttttttttt tgatatttta cgtatttgta gtttgaaggt ttcgtaggcg 5940
   atagcggtaa tggatgtttg gtggaatttt ttttgggttt atatttcgtt tttttttagt 6000
   aggtcgggta ataattttta gggtttatcg aggggtgttt agttcggagg agggggagtg 6060
   acgttatagc gatgtttaaa ataaggtttt ttttggtttt attatagaaa agtattagga 6120
   aggggaatgt ggcgagtatt ttaggaaatg ttcggattgg ggattaggtg atattgtagg 6180
   aatgtgtgtg ttgggggatg ggatggtgtt ttaaatatgg gttattagga gttagaagga 6240
   gttttttttt tttttttttt tttttaagta aagtagagga ggtggagatt gggatggggg 6300
   tagttcggaa aaattatttt ttaggggtgt gagtttagtt cgtgttgtgc ggattgtttt 6360
   cgttatagtt taattatttt tgtggtgtgt ttagacgttt ttttttttat tttttcgttt 6420
   tttggttttt ttattttttg ggttttgggt ttagttttga aagtcgaatt gtgggttgta 6480
   gtggggaggc gagggtaggt agaaattagt tttagggggg gttttatttg gtttaggagt 6540
   tgggggataa tagaaaaggt aggtttaggg tatgagggaa ggcgtttttt ttgttgtatt 6600
   ggggtagtga tttttggggg ttttgtatat ataggaggga ggttgtaagt ttatagttgt 6660
   tttagtattt ttttttttgt tggggtagat agagtggaag attaaggtaa ttaattttag 6720
   agggttatta gaaaattgtt tattaggttg ttattataga ttattggagt aaggttcgtt 6780
   tggggatgtt tttgagtttt ttagattatt tagataattg ttttagtttt ttattcggtt 6840
   ttttttgttt agattagaat tttaggggta ggggaggata tttttgtatt aatgttcggt 6900
   attcgttttt aaattattag gtcggtaggt aacgttagtt atcggtgcga ttttttagcg 6960
   gaggaaggag tggggatcga atatttgggt ggtcgcgatt ttgggtagat attttttagt 7020
   tgggggattt ttaagtagag gatttgttag tatagttttt ttaatttagt ttcgttttta 7080
   gtagaaaaat ttttataata aacgaaaaga gcgtttggtt ttcgattgtt ttaaagataa 7140
   agtgatttat attgtttgtt tatatggttg gattaattcg atagttgttt ttttgggggt 7200
   agatttttac gggaagtttg tgtgatggga taatttaatg ttatttaagt tttttggagg 7260
   ggcggttttt ttaattattt atttaggaga aagttagtta atatttattt gatcgttttc 7320
   gtttcgggat ggtttattta tcgttttttg gtaggaaagt tttcgtttcg aagggcgttt 7380
   cgggggttta gagtcgagtt aagttgtaag agggggttat ggggaatgcg ggaagtgaat 7440
   ttttaatttt ggtgttcggt tttaaaagat ttagggtttt ttgtgtttag gtagaggtaa 7500
   atggagattt aaattgtcgt tttgtattaa taattcggaa taaaaataaa attcgtaaag 7560
   ggatagatga tcggaaagaa taaatggtgt atgtgtgcgc gaacgagttt tagttttttt 7620
   ttatttaggt ttttagcgga tttagagcgg tcgtcgtatt tt 7662

<210> 99
<211> 10250
<212> DNA
<213> Artificial Sequence

<220>
<223> chemically treated genomic DNA (Homo sapiens)

<400> 99

gtttttttaa tattatttgt gaaataagga attttttttt tattgtttgt ttttgttagg 60
tttgttaaag atcggatggt tgtagatgtg tggtattatt tttgaggttt ttgttttgtt 120
ttattggttt atatatttgt tttggtatta gtattaagtt gttttggtta ttgtattttt 180
gtggtatagt ttgaagttag gtagtatgat ttttagtttc gttttttttg tttaggattg 240
ttttggttat ataggttttt tttttgtttt atatgaaatt taaagtagtt ttttttaatt 300
ttatgaagaa agttaatggt agtttgatgg ggatagtatt gaatttataa attattttgg 360
gtagtatggt tatttttatt atatttattt ttttatttat gagtatggaa tattttttta 420
tttgtttgtg ttttttttta tttttttaag tagtggtttg tagttttttt tgaagaggtt 480
ttttatattt tttgtaagtt gtatttttag gtattttatt ttttttgtag cgattatgaa 540
tgggagtttt tttatgattt agttttttgt ttgtttatta ttggtgtata ggaatgtttg 600
tgatttttgt atattgattt tgtattttga gattttgttg aagttgttta ttagtttaag 660
gaaattttag gttgagacga tggggttttt taaatatata attatgttat ttgtaaatag 720
agataattta attttttttt tttttgttta aatacgtttt attttttttt tttgtttgat 780
ttttttggtt agaattttta atattatgtt gaataggagt ggtgagagag ggtatttttg 840
ttttgtgttg gtttttaaag ggaatgtttt tagtttttgt ttatttagta tgatattggt 900
tgtgggtttg ttataaatag tttttattat tttgagatat gttttatcga tatttggttt 960
attgaggttt ttagtatgaa ggggtgttga attttatcga aggttttttt tgtatttaat 1020
gagttaatta tgtggttttg ttattggttt tgtttgtgtg atggattatg tttattgatt 1080
tgcgtatgtt gaattagttt tgtattttag ggatgaagtt aatttgatta tggtgggtaa 1140
tttttttgat gcgttattga gtttgttttg ttaatatttt attgaggatt tttatgttga 1200
tgtttattag ggatattggt ttgaaatttt ttttttgttg tgtttttttt aggttttggt 1260
attaggatga tgttggtttt ataaaacgag ttagggagga gttttttttt ttttattgtt 1320
tggaatattt ttagaaggaa tggtattagt aattttttgt atttttggta gaattcggtt 1380
gtgaatttgt ttggttttgg gttttttttg gttgatagtt tattaattat tgttttagtt 1440
ttaaaatttt ttattggttt atttagggat ttaattattt tttggtttag ttttgggagg 1500
gtgtgtgtgt ttaggaattt atttattttt tttagatttt ttagattatt tgtgtagagg 1560
tgtttatagt attttttaaa ggtagtttgt atttttttgg ggttagtggt gatatttttt 1620
ttattagttt ttattttgtt tatttgattt tttttttttt tttattagtt tggttagtgg 1680
tttatttatt ttgttaattt ttttaaaaaa ttagtttttg gatttattga ttttttgaag 1740
ggttttttat gtttttattt tttttagtat tgttttgatt ttagttattt tttgtttttt 1800
gttagttttt gaatgtgttt gtttttgttt ttttagtttt tttaattgtg atgttagagt 1860
gttagtttta gatttttttt gttttttttt gtgggtattt agtgttatga attttttttt 1920
aaatattgtt ttagttgtgt tttagagatt ttggtatatt gtgtttttgt ttttattggt 1980
tttaaggaat ttatttattt ttgttttaat tttgttattt atttagtagt tatttaggag 2040
taggttgttt agtttttatg tagttgtgtg gttttgagtg aattttttta ttttgagttt 2100
tagtttgatt gtattgtggt ttgagagatt gtttgttatg aattgtatta ttttgtattt 2160
gttggggagt gtgttatttt tagttatgtg gttaatttta gaataagtgt gatgtggtgt 2220
tgagaagaat gtatatattt tgttgatttg gggtggaaag ttttttaaat atttattaga 2280
tttatttggt ttagagttga gtttatgttt tgaatatttt tgttaatttt ttgttttatt 2340
aatttaatat tgatagtggg gtgttaaagt tttttattat tattatgtgg gagtttgagt 2400
ttttttgtag gtttttaaga atttttttta tgaatttgga tgttttggta ttgagtgtat 2460
atatatttag gatagtattt tttgttgtat tgattttttt attattatgt aatgtttttt 2520
tttatttttt ttgatttttg ttggtttaaa gtttgtttta ttagagatta ggattgtaat 2580
ttttgttatt tttttgtttt ttatttgttt ggtaaatatt tttttattag atggtttgat 2640
aaagttttta aatatgggat attattagta gagggaatga gtattagagt ggaaaggtta 2700

gagaaaaagt gtttttggga gtttgggggt taggatgtta gagtgaaaat gaatatttat 2760
tgaacggttt aaaggatttt agagagttgt gttttttaat ttttttttta atattaaaat 2820
gtgttataaa ttatagttaa tatacgtata tatgtttata taattgtgta tttagtattt 2880
gttgttttaa aaaaatgtta gtaattaaat attttaaagt aaaaatgtat tttatgaagg 2940
tattgtttat ataaatattt gaaaaataat aatatatgta tttgagataa atagatgtta 3000
ttttgtttta gtaggtaaag atatgattgt atttttttta tagatattaa tatgttgggt 3060
agtgaaattt ttagagattt gttagtatgt agtataagta atatttattt aagtagtaat 3120
ttttatgtgt agatgaatgt taaagttttt taatgatagt ttgttttagg tgtttttttt 3180
tagttatttt ggaattattt tttgtttatg tggggaaagt gatttttaat agttttttat 3240
tttttgtata atttggttta aacgttttag tttggattaa aatttttttt tttgtttttt 3300
tttaaagtgt tttagttata tttatttttt tggttttttt tagtaattta agtgaatttg 3360
agttttaatt ttttgtattt tgtatttatt ttttcgttag aattttattt tatttgagtt 3420
ggttgttttt tatgtgtttt tttggttttg ggatggatat tgtaataagt gatttgtatt 3480
tttgtgtttt attgtattat gttttttttt aaggttggta taggtttttt ttattttata 3540
ttttacgatt taagagtgtt tatagtataa tgggtggttg gtatttatta aataaatgaa 3600
tgtgtaaatg aatgatagaa agtaattggt gaaaagtacg ttgagataga gtttgattat 3660
aggtaaaaaa aaaaaaaaaa tggaagaatt aatagtttaa tggtattttt tttataaaat 3720
tgaattatat agttaaatta ttatttaaaa taattttata aggaaaataa tattaatgtg 3780
cgtttgttga gacggattat gttgttttgg aattaggaag agaatgtgta gttcgttgtt 3840
tgtttttaga ttgtttgttt gttttgttta ttaaattggc gtttttagtt ttgtagttta 3900
gtttgtggaa tttaaatggt ttaggtttta ggagttttta tcggagtcgt gggcgttttt 3960
agggtttaga aaggtttttt aggggtttaa gttgtcggcg agtgtagttt tcgtcggcgt 4020
tatttttgcg gtttggagcg gcgaggacga ttttagagag gcgtttttat tgtttcggtc 4080
gtcgggtttt taagtttttt tttttaaaaa tttaatggat agtttttttt tgtttttgtt 4140
tttgatttga ttaaagaggc ggaaaaaaag gagtttgatt ttaagaagaa tgatagtagt 4200
agaaggcgta tttggtttta ttttttaaat tttattaaag tttgtgtttt gttttatttg 4260
atagcgattt cgtgaacgtt ttttttgggg aaatggcgcg attaaagttc gttaagttta 4320
gtttgcgaat tggaggattt attaatgtaa tatgttaaat aaaatttcga tagatataaa 4380
tttaaaggaa atcgtgcgtt ttttaagtta aaggtatttg ttttggaagt agaattcggt 4440
ttgttgttgg taattagcgt taagagtagg atttttaaat atgataatgg ataaaataaa 4500
agaaaagcga agtttcgcgg ttttttgatt tttatttatt atttttagta gtatatggga 4560
ttattttttt acgtattttc gttttttttt tttgaaaaag taaaagtaat taagtttgtt 4620
tcgttttaga gtaattttat tttttgtgtt tgagtagtgt tattattatt attttttttt 4680
tttttcgttt ttttttattt cgtttttttg tcgttggttt tcgttttggc gaggaggcgg 4740
tcggtttttt ttgcgtcggg aagtcggagt cgcgattggg cgacggtttt tgagttttta 4800
gttttgcgtc ggttttaagg tttttttttt ttggtgaaag atagattacg gggcgtttgg 4860
aaatcggttc gagggcgcgc gaggtagagg agagggagcg agttgaggga ttgatataaa 4920
tggttaggcg gcggcggcgg agaaggaggc ggaggcgtag gggggagtcg agttcgttgg 4980
gttgcggaga gttgcgtttt ttacggggtc gcggttatta gcgcggcgtc gttagtcggg 5040
agttagcgag tcgagggtta ggaaggcggg atacgatttc ggcgcgtttt agttattcgg 5100
gtttttttcg tcgttcgcgt tttatgaatc gtaagttttc gcggcggcgg cggttgcggt 5160
acgtagaata ggagtcgggg gagcgggtcg aaagcggttt gggttcgacg gagggtattc 5220
gcgtagaggt ttttttggtc gtagggggag tcgtcgtcgg tcgtgttttt ggtagtttta 5280
gcggagcggc gttaagagag gagtcgagaa agtatggttg aggaggaggc gtttaagaag 5340
tttcgggtcg tcggcggtgg cgcgagttgg gaattttgtg tcggggcgtt ttcggttcgg 5400
ttggcggagg agggtagcgg ggacgtcggt ggtcgtcgtc gttcgttagt tgattttcgg 5460
cgattggcgc gttagttgtt gttgttgttt tggttgttgg aggtttcgtt gttgttgggg 5520
gttcgggttt aggcggcggg ttaggggtta ggttaggggt tcgggtcggg gtagtaatcg 5580
tcgtcgtcgt tttagtagta atagagcggg tagtagtata acggcgagcg gggtattttc 5640
gtttcggatt acggttattg ttagtttatt tttatttcgt tgtgtacgga tatcgcgtat 5700
aattagatta ttatgtttaa tttgttgggt tatacgaatt aggaggacgc gggtttggag 5760
gtgtattagt tttatttttt agtgaaagtg tagtgtttcg ttgagtttaa gttttttttg 5820
tgttttatgt acgcgttcgt gtgtatcgtg ttagagtagg cgttgtcgtt ttgtcgtttt 5880
ttgtgcgagc gcgcgcgtta gggttgcgag gcgtttatga ataagttcgg tttttagtgg 5940
ttagatacgt ttaagtgtga gaagttttcg gtgtacggcg tcggcgagtt gtgcgtgggt 6000
taaaatacgt tcgataaggg tatttcgacg ttttcgttgt ttttagagtt ttggattagt 6060
aatttttagt acggcggcgg agggtatcgt ggcggttttt cggggggcgt cggcgcgtcg 6120
gagcgaggta agtttttttg ttcgcgcgtt tttaaggtgt ttttttattt taattattat 6180
tttttggggg agaaggattg cggcgtattt tgtgagtcga ttaaggtgta tgggtttatg 6240
tatttcgggt tcgaggagtt gcgtttttcg cgtatttgga ttggtatttg gttagtgttg 6300
tgttgcgttt ttacgttttt tacggtgttt acgtatttgg tggatatgcg gcgttttagt 6360
tattcggagc ggtttattat ttttttgttc ggttgttata cggtcgtggt cgtggtttat 6420
atcgtcggtt tttttttgga agatcgagtg gtgtgtaatg ataagttcgt cgaggacggg 6480
gtacgtattg tggcgtaggg tattaagaag gagggttgta ttattttttt tatgatgttt 6540
tattttttta gtatggttag ttttatttgg tgggtgattt tgtcgtttat ttggtttttg 6600
gcggttggta tgaagtgggg ttacgaggtt atcgaagtta atttatagta tttttatttg 6660

gtcgtttggg ttgtgtcggt tattaagatt attattattt tggcgttggg ttaggtggac 6720
ggcgatgtgt tgagcggagt gtgtttcgtg gggtttaata acgtggacgc gttgcgtggt 6780
ttcgtgttgg cgtttttttt cgtgtatttg tttatcggta cgtttttttt gttggtcggt 6840
tttgtgtcgt tttttcgtat tcgtattatt atgaagtacg atggtattaa gatcgagaag 6900
ttggagaagt ttatggtgcg tattggcgtt tttagcgtgt tgtatattgt gttagttatt 6960
atcgttatcg tttgttattt ttacgagtag gtttttcggg attagtggga acgtagttgg 7020
gtggtttaga gttgtaagag ttacgttatt ttttgttttt atttttaggc gggcggaggc 7080
gtttcgtcgt attcgtttat gagttcggat tttacggttt ttatgattaa gtattttatg 7140
acgttgatcg tgggtattac gtcgggtttt tggatttggt tcggtaagat ttttaatttt 7200
tggaggaagt tttatacgag gtttattaat agtaaataag gggagattat agtttgagat 7260
tcggggttta gtttatgttt aggtttcggt cggggcgtag cgatttttta aagttagcgt 7320
cgtggagttc gtgttaattt tgatatttcg aggttttttt attagataat tttttttcgt 7380
aggttttttt gaataattta gtttttgtaa aagttttcgt ttttgaggta aaaggatacg 7440
agggttcgat tgttagaggg aggatggata gattttttgt ttttatattt tggtattagg 7500
attgttcgtt tttatgattg taaatagttt gtgtaagatt tttgtaagta tatttgtatt 7560
taaatgacga tcgattacgc gttttttttt tttaaaagtt tttaattatt tagggcggtt 7620
taattatttg aggttttttt tttttgtttt tttcggagta ttgtaaagga gttaaaattg 7680
gtgtgtaatc gtatagcgtt tttggtcgtt ttcgcgcgtt tttttttatt acgggtgttc 7740
gggacggttg ggcgttagtt tcggggcgag tttagtattg cggggtgcga ttagggttgc 7800
gttgttaggg ttatttttcg tttttttttt ttgttttttt tttttttttt tgtttttttt 7860
tttttttttt tggtttgagg taggggtttt taaggtatag aattttataa attttttaaa 7920
tttggaggag ggtttttata tattataatt ttttttttgt tcggcggtgg attgcgaagg 7980
ttcgtttttt cgattttttg aagttggatt tttaattgtt tagaattttt ttttaatttt 8040
atgggggttt acgggtgtgg gcgttggtag ttttagtttt tttttacggt tatttttaac 8100
gtttagatat tttttttttt tattttagtt ggttataggg tgagtgagat aattaatgtt 8160
aaattttttg aagtttaatt tttgaggggt gagtttattt tattttttag tgtttaaaat 8220
ttggtatggg tttggttagc gttatggaaa gatgtggtta ttgagatttg ggaagaagta 8280
tgaagttttg tgtgggttgg aagagattga agatatgggt tataaaatgt taattttaat 8340
tgtatacgga tgtttggtaa ttttgttttt gagaatgaga tagtttgcgt ttagatttta 8400
tcggtttgta aaatggaaat gttgaggtta tttggaaagt tttgttaagg agttgatgtt 8460
tgtttttttt aataagatag taaaacgtaa atagaaattg aaaatttgaa ggatatttta 8520
gtgttatgga tttttttaaa atgaagtgtt atttttttat ttttaattaa ataattagat 8580
atatattaga aattttaaaa tgtaaaagtt gtatattttt aatattttat tacgattatt 8640
atttagtagt atattttgag gggggaataa tttatattat taataataat ttggtaagat 8700
tttaggaggt aaagaaggtg gaataattga cggggagata gcgtttgaaa taaataaaat 8760
atgggtatgt atgttaaagg gaaaatgtgt gtaggtttat tgtattaaat tttgtgtgtt 8820
ttttttttgg atttatagaa atgtgttaaa tgtaaatttt ttaaagttat ttaaaaatat 8880
ttattttagt tttttgtgaa gaagaggaga aaagtaattt ttttgattgt attgttttaa 8940
attttaagaa tttattaaaa tgtcggtatt taggatttaa atttatttat gtttgttata 9000
cgttaaaatg atattggttt ttgaatttgg tatatattta ttttgtttat tattataaaa 9060
ttatttatat ttatagagga atagaagttt atatatatat aatattatat ttttaatttt 9120
ataaataaaa aatttaaagt tttgtataaa attatatgga ttttgtgttt gaaaataata 9180
gagtttgagt tgtttgaatt attttatatt ttatggtgtt ttatagttaa ttttatagtg 9240
taaaaattta ggaatttaat gaaaaaagtt tatttttaaa tttttagatt agttttttta 9300
aagaattatt ttgtttgtat tgttgtgatt gatatttgtg aagttttaag aaaagatttg 9360
tttttatgat agtagaaaat agaagtttgt aaattatttt tttatttaaa gaggattaaa 9420
agagaatttt aattttaata ttaaagtttt tttttttttt agggaataaa tttatatgat 9480
tttttatatt atggaggttt atttttaaat tattattttt tttatatttt ttagaggtat 9540
tgttttattt tttttataat tttggatatt ataaaatttt aaataggtaa ttaataaatg 9600
gttaattggt tatgtgttta taaatatttt aaatagtaat tgggtttgta aggttgtttt 9660
gttgtttgtt tttgaagatt ttttttttta atcgtttaag gaattttttt tagataaatt 9720
atttagttgg tttattttta gttagaattt atgtatgggt gtatatatgt gtatatatag 9780
ataagtatat atataagtat atatgtatta atttttataa aatagagatg taaagtttat 9840
ttatatttta taatatggaa atatggttat gagaaatgga aaatgttaga tggaaaattt 9900
taaatatttt tataggttaa aaatattttt attaaatttt tttatgaaaa tattttgtga 9960
taaaatgata agggattaaa atgaagatta ttgatataat agagttattt tttttgttat 10020
aatgtaattg ttttataagt tattatttat tttaaggaaa attaaaaaaa ttagatttta 10080
ttaaaaatgt agtatttgat tttaagtagt aagatgtaat tttgtgtttt tttttttttt 10140
gttatattaa tataatatag tagatagagg gtttattgta tagatatata taaaaaaata 10200
gtatttttag gttaaattta tatatatttt tttattaagt agtttaatta 10250

<210> 100
<211> 10250
<212> DNA
<213> Artificial Sequence

<220>
<223> chemically treated genomic DNA (Homo sapiens)

<400> 100

taattgaatt atttagtgag gagatatgtg tgaatttggt ttaagaatat tatttttttg 60
tgtatgttta tataataaat tttttattta ttatattatg ttaatgtaat aaagaaaaag 120
ggaatataaa attgtatttt gttatttaaa attaaatgtt atatttttag taagatttaa 180
tttttttgat tttttttaaa atgaataatg atttataaga taattgtatt gtaataagga 240
aagtaatttt attgtattaa tgatttttat tttagttttt tgttatttta ttataaaatg 300
tttttatggg aaaatttagt aaagatattt ttaatttgta aaggtatttg aggtttttta 360
tttagtattt tttatttttt ataattatat ttttatatta tgaaatgtaa gtaaatttta 420
tatttttatt ttataaaaat taatgtatat atatttatgt gtatatttat ttatatatat 480
atatatatat atttatgtat aaattttgat taagagtaga ttaattagat aatttatttg 540
gagaaggttt tttgaacgat taaaaaaagg ggtttttaga agtaaataat aaaatagttt 600
tataaattta attgttgttt aaaatgttta tgaatatata gttagttaat tatttattga 660
ttgtttattt agggttttgt aatatttaag attatggaag agataagata atatttttaa 720
aaaatatgag aaaggtgatg atttaaaaat aaatttttat aatataaaaa gttatgtaaa 780
tttatttttt gaaagaaaag aaagttttaa tattaaaatt agagtttttt tttaattttt 840
tttgagtaaa gaaataattt gtaaattttt attttttatt gttatgaaaa tagatttttt 900
tttgggattt tataaatgtt aattataata atgtaaatag agtaattttt tggaaagatt 960
gatttgaggg tttaagggta gatttttttt attgaatttt tgaattttta tattgtggga 1020
ttggttatga gatattataa aatgtaaaat agtttagata gtttaagttt tattattttt 1080
aggtataaaa tttatataat tttgtataaa attttgaatt ttttatttgt gaaattaaaa 1140
atatggtatt atatatatat aaatttttat ttttttataa atatagatga ttttgtgata 1200
gtgaatagaa taaatgtata ttaaatttaa agattaatat tattttagcg tatgatagat 1260
atagataaat ttaggtttta agtatcggta ttttgataaa tttttaaagt ttaaaataat 1320
ataattagga ggattgtttt tttttttttt tttatagaga attaaagtga atatttttaa 1380
atggttttga aagatttata tttgatatat ttttgtaaat ttaaaagagg agtatatagg 1440
atttaatgta gtagatttgt atatattttt tttttagtat gtatgtttat attttgttta 1500
ttttaggcgt tattttttcg ttaattattt tatttttttt attttttgaa attttattag 1560
gttattattg gtggtgtgaa ttgttttttt tttagaatgt gttgttgaat aataatcgta 1620
ataaaatgtt gaaagtgtat aatttttata ttttaaagtt tttgatatat gtttagttat 1680
ttgattaaaa ataagaaaat agtattttat tttgaggaag tttatgatat tgaaatattt 1740
tttaagtttt taatttttgt ttacgttttg ttgttttgtt aaggaaagta aatattaatt 1800
ttttaataaa gttttttagg tgattttaat atttttattt tatagatcgg taaaatttaa 1860
gcgtaggttg ttttattttt aaaggtaagg ttgttaggta ttcgtatgta attagaatta 1920
atattttata atttatattt ttagtttttt ttaatttata taaagtttta tgtttttttt 1980
taaattttag taattatatt tttttatgac gttggttaaa tttatattag gttttagata 2040
ttagagaatg aaatgagttt attttttaaa aattagattt taaaaagttt ggtattggtt 2100
atttatttta ttttgtaatt aattaaggtg ggagaaggga gtgtttgggc gttgaaggtg 2160
atcgtggagg gaggttgaga ttgttagcgt ttatattcgt gggtttttat gaagttggag 2220
gaaagttttg gatagttaaa aatttagttt taggaagtcg aagggacggg ttttcgtaat 2280
ttatcgtcga gtaagggagg aattgtaatg tatgggggtt tttttttaga tttggaaggt 2340
ttgtggagtt ttgtatttta agagttttta ttttaagtta ggaaagaaag ggaggggata 2400
gaaggagggg gagggggtaa aaggaggagg cgggaagtga ttttggtagc gtagttttag 2460
tcgtatttcg tagtgttgaa ttcgtttcgg agttggcgtt tagtcgtttc gagtattcgt 2520
ggtagggaga ggcgcgcgag gacgattagg agcgttgtgc ggttgtatat tagttttagt 2580
ttttttgtaa tatttcgaaa agggtaagaa ggaaaagttt taaatggtta aatcgtttta 2640
aataattaaa aatttttgaa aaagaaaaac gcgtgatcgg tcgttattta aatataaata 2700
tatttataaa aattttatat aggttattta taattataaa agcgaatagt tttggtatta 2760
gagtgtgagg gtaagaggtt tgtttatttt ttttttggta gtcgggtttt cgtgtttttt 2820
tgttttaggg acggaagttt ttgtaggagt tgagttgttt aaaggagttt gcgaaagaga 2880
gttgtttagt gaggaaattt cgagatgtta ggattggtac gaattttacg gcgttggttt 2940
tgggggatcg ttgcgtttcg gtcgaggttt gggtatgggt tgagtttcgg gttttagatt 3000
gtagtttttt tttgtttgtt gttggtgagt ttcgtgtaga atttttttta ggagttgagg 3060
gttttgtcgg attagattta gaagttcgac gtgatgttta cgattagcgt tataaggtat 3120
ttaattatga agatcgtgaa gttcgggttt atgggcgggt gcggcggggc gttttcgttc 3180
gtttggaggt gagggtaggg gatagcgtag tttttgtagt tttgggttat ttagttgcgt 3240
ttttattggt ttcggaaggt ttgttcgtag aagtagtagg cgatgacgat ggtggttggt 3300
atagtgtata gtacgttgaa gacgttaatg cgtattatga gtttttttag tttttcggtt 3360
ttggtgttat cgtgttttat gatggtgcgg atgcggaaga gcgatataaa gtcggttagt 3420
agaaaggacg tgtcgataaa taggtatacg aagaggggcg ttagtacgaa gttacgtagc 3480
gcgtttacgt tgttaagttt tacgaagtat atttcgttta gtatatcgtc gtttatttgg 3540
tttagcgtta ggatggtgat ggttttgatg gtcggtatag tttaggcggt taggtgaaaa 3600
tattgtgagt tggtttcgat ggtttcgtgg ttttatttta tgttagtcgt taggaattag 3660

gtgagcgata ggattattta ttagatggag ttggttatgt tgaagaagta gagtattatg 3720
aagaggatgg tgtagttttt ttttttggtg ttttgcgtta tagtgcgtgt ttcgttttcg 3780
gcgaatttgt tattatatat tattcggttt tttaggagga agtcggcgat gtaggttacg 3840
gttacggtcg tgtaatagtc ggataagaag atgatgggtc gtttcgggta gttgaagcgt 3900
cgtatgttta ttaggtacgt aagtatcgtg aagagcgtgg aggcgtagta tagtattgat 3960
taaatgttaa tttaggtgcg cgagaagcgt agtttttcgg gttcgaagta tatgagttta 4020
tatattttgg tcggtttata aggtgcgtcg tagttttttt tttttaggaa gtggtagttg 4080
aggtaggagg gtattttgag ggcgcgcggg taggagaatt tgtttcgttt cgacgcgtcg 4140
gcgtttttcg ggaagtcgtt acggtgtttt tcgtcgtcgt gttgagggtt gttggtttag 4200
aattttggaa gtagcgaggg cgtcggggtg tttttgtcgg acgtgttttg gtttacgtat 4260
agttcgtcgg cgtcgtgtat cgggaatttt ttatatttga gcgtgtttgg ttattggaag 4320
tcgaatttgt ttatgagcgt ttcgtagttt tggcgcgcgc gttcgtatag ggagcggtag 4380
ggcggtagcg tttgttttag tacggtgtat acgggcgcgt atatggagta taggaagaat 4440
ttgagtttag cggaatattg tatttttatt agagggtaga attggtgtat ttttaggttc 4500
gcgttttttt ggttcgtgtg gtttagtagg ttgggtatga tggtttggtt gtacgcgatg 4560
ttcgtgtata gcgggatgga gatgggttgg taatagtcgt ggttcgggac ggagatgttt 4620
cgttcgtcgt tgtattgttg ttcgttttgt tgttgttgag gcggcggcgg cggttgttgt 4680
ttcggttcgg gtttttggtt tggtttttgg ttcgtcgttt gggttcggat ttttagtagt 4740
agcggagttt ttagtagtta aagtagtagt agtagttggc gcgttaatcg tcgggggtta 4800
attggcgggc ggcggcggtt atcggcgttt tcgttgtttt ttttcgttag tcgggtcgag 4860
agcgtttcgg tataaagttt ttagttcgcg ttatcgtcgg cggttcggga ttttttaggc 4920
gttttttttt tagttatatt ttttcggttt tttttttggc gtcgtttcgt tggggttgtt 4980
aggggtacgg tcggcggcgg ttttttttgc ggttagggag atttttgcgc gggtgttttt 5040
cgtcgagttt aagtcgtttt cggttcgttt tttcggtttt tgttttgcgt atcgtagtcg 5100
tcgtcgtcgc ggaaatttgc gatttatgaa gcgcgggcgg cggggagaat tcgggtggtt 5160
agggcgcgtc ggggtcgtgt ttcgtttttt tggttttcgg ttcgttggtt ttcggttggc 5220
ggcgtcgcgt tagtggtcgc ggtttcgtag agagcgtaat ttttcgtagt ttagcgggtt 5280
cggttttttt ttgcgttttc gttttttttt tcgtcgtcgt cgtttgatta tttgtgttaa 5340
ttttttaatt cgtttttttt tttttgtttc gcgcgttttc ggatcggttt ttaggcgttt 5400
cgtagtttgt tttttattag gagggaggag ttttgaaatc gacgtagaat tggaggttta 5460
gggatcgtcg tttaatcgcg gtttcggttt ttcggcgtaa agggagtcgg tcgttttttc 5520
gttagagcga aagttagcgg taggagggcg gggtaggagg aggcgggagg aggaggaagt 5580
agtggtggtg gtattattta ggtataaaga gtaggattgt tttgaagcgg gataggtttg 5640
gttgtttttg tttttttaga agaaaaaggc gaggatacgt aggagaatgg ttttatatgt 5700
tgttggagat aatgaatagg agttagagga tcgcgggatt tcgttttttt tttattttat 5760
ttattattat gtttaaaaat tttgttttta acgttggtta ttaatagtaa atcgggtttt 5820
gtttttagaa tagatgtttt tgatttaagg agcgtacggt tttttttgag tttgtgtttg 5880
tcgaggtttt gtttaatata ttatattaat aaatttttta gttcgtaggt tgggtttggc 5940
gggttttggt cgcgttattt ttttagagga gacgtttacg ggatcgttgt taaatgaaat 6000
aaaatataaa ttttgatgga atttggaaag taaagttaga tgcgtttttt gttattatta 6060
ttttttttgg agttaaattt tttttttttc gtttttttaa ttaagttaga aatagaagta 6120
aaagaaaatt atttattaaa tttttggaga gaaaaattta agaattcgac gatcgaggta 6180
gtgggggcgt ttttttggga tcgttttcgt cgttttagat cgtagaggtg gcgtcggcgg 6240
gagttgtatt cgtcggtagt ttgggttttt ggggagtttt tttaaatttt ggggacgttt 6300
acggtttcga tgggaatttt tgggatttgg gttatttagg ttttataggt tgggttgtag 6360
agttggagac gttagtttaa taaataaaat aaataaatag tttgaaagta aatagcgaat 6420
tgtatatttt ttttttaatt ttaaaatagt ataattcgtt ttaatagacg tatattaatg 6480
ttgttttttt tgtaagatta ttttaaatga tggtttgatt atgtaattta gttttataag 6540
aaaagtgtta ttggattatt aattttttta tttttttttt ttttttgttt gtgattagat 6600
tttattttag cgtatttttt attaattatt ttttattatt tatttatata tttatttgtt 6660
taatagatat taattattta ttgtgttata agtattttta ggtcgtagag tataaaatga 6720
aggagatttg tgttagtttt agagaggggt atagtatagt gaggtataga aatgtaaatt 6780
atttgttata gtgtttattt taaagttaaa aaggtatata gaaagtaatt agtttagatg 6840
aaatggggtt ttgacggaga ggtaggtgta aggtgtaaaa gattaaagtt taaatttatt 6900
tgaattgttg aagggggtta gagaggtgag tgtagttgag gtattttgag gggaggtagg 6960
agaaaaggtt ttaatttaga ttaaaacgtt tgggttaaat tatatagaaa atagaaaatt 7020
attgaaggtt atttttttta tataagtagg gagtgatttt agggtgattg aaagagggta 7080
tttgaggtag gttgttatta ggaagtttta atatttattt atatataaag gttattattt 7140
agatgggtgt tatttgtatt atatgttaat aagtttttga gaattttatt atttagtatg 7200
ttggtgttta taaagaaggt gtagttatat ttttatttgt taaagtaaaa tgatatttgt 7260
ttgttttaga tatatatgtt attgtttttt aaatgtttat ataagtagta tttttataaa 7320
atgtattttt gttttaaagt atttgattgt tagtattttt ttaaagtaat aggtattaaa 7380
tgtataatta tgtgagtatg tgtacgtgtg ttgattgtaa tttatgatat attttgatat 7440
tgaaaaggag gttgggaagt atagtttttt gaagtttttt gaatcgttta ataagtgttt 7500
atttttattt tagtattttg gtttttaggt ttttagggat attttttttt tagttttttt 7560
attttgatgt ttattttttt tgttgatagt attttatgtt taggaatttt attaagttat 7620

ttgatggaag aatatttatt aagtaaatgg aaagtaaaaa aatagtaaga gttgtaattt 7680
tagtttttga taaaatagat tttaaattaa taaagattaa aaaagataaa gaagagtatt 7740
atataatggt aaagggatta atataataag aagtgttatt ttaaatatat atgtatttaa 7800
tattagagta tttagattta taaagaaagt ttttagagat ttataaagag atttagattt 7860
ttatataata atagtgggag attttaatat tttattgtta atattagatt aatgagatag 7920
aaaattagta aggatattta ggatatgaat ttagttttgg attaagtgga tttaatagat 7980
atttagagaa ttttttattt tagattaata gaatatatat atttttttta gtattatatt 8040
atatttattt taaaattgat tatataattg gaagtaatat attttttagt aaatgtaaaa 8100
taatgtaatt tataataaat agttttttag attatagtgt aattaaatta gaatttagga 8160
tgaagaaatt tatttaaaat tatataatta tatagaaatt gaataatttg tttttgaatg 8220
attgttgggt aaataataaa attaaggtag aaataaataa gttttttgaa attaatgaga 8280
ataaagatat aatgtattag aatttttggg atatagttaa agtagtgttt agagggaaat 8340
ttatagtatt aaatgtttat aggagaaagt aggaaagatt taaaattgat attttaatat 8400
tataattaaa agaattagag aagtaagagt aaatatattt aaaagttagt agaagataag 8460
aaataattaa aattagagta gtattgaagg agatagagat atgaaaaatt ttttaaaaaa 8520
ttagtgaatt taggagttgg ttttttgaaa agattaataa aatggataga ttattagtta 8580
gattaataag agagagagaa gaattaaata gataaaataa aaattgataa aggagatatt 8640
attattggtt ttagagaaat ataaattatt tttagagaat attataaata tttttatata 8700
aataatttag aaaatttaga agaaatggat aaatttttgg atatatatat ttttttaaga 8760
ttaaattagg aggtagttga atttttgaat aggttagtaa gaagttttga ggttgaggta 8820
gtaattaata gattattaat taaaaaaagt ttaggattag atagatttat agtcgaattt 8880
tattagaggt ataaagagtt gttggtatta ttttttttga aaatatttta aataatagaa 8940
aaagagggat ttttttttaa ttcgttttat gaggttagta ttattttgat attaaaattt 9000
aggagagata taataaaaaa aaaattttag gttaatattt ttgataaata ttaatatgaa 9060
aatttttaat aaaatattgg taaagtaaat ttagtagcgt attaaaaagg ttatttatta 9120
tgattaagtt ggttttattt ttgggatgta aggttggttt agtatacgta aattaataaa 9180
tataatttat tatataaata gaattaatga taaaattata tgattaattt attagatgta 9240
gaaaaggttt tcggtaaaat ttaatatttt tttatgttaa aaattttaat aaattaggta 9300
tcgatagaat atattttaaa ataataagag ttatttatga taaatttata gttaatatta 9360
tattgaatgg gtaaaagttg gaagtatttt ttttgaaaat tagtataaga taaggatgtt 9420
tttttttatt atttttattt aatatagtat tggaagtttt ggttagggaa attaggtaag 9480
agaaagaaat aaagcgtatt taaataggaa gagaggaagt taaattgttt ttgtttgtag 9540
atgatatgat tgtatattta gaaaatttta tcgttttagt ttgaaatttt tttaagttaa 9600
taagtaattt tagtaaagtt ttaagatata aaattaatat gtaaaaatta taagtatttt 9660
tatatattaa taatagataa atagagagtt gaattatgag agaattttta tttataatcg 9720
ttataaagag agtaaaatat ttaggaatat aatttataaa ggatgtaaag gattttttta 9780
aggagaatta taaattatta tttaaggaaa taagagagga tataaataaa tggaaaaata 9840
ttttatgttt atggatagaa gaatgaatat agtgaaaatg gttatattgt ttaaagtaat 9900
ttatagattt aatgttattt ttattaagtt attattgatt ttttttataa aattagaaaa 9960
aattatttta aattttatat ggaataaaaa aagagtttgt gtagttaaga taattttaag 10020
taaaaagaac gaagttagag attatgttat ttgattttaa attatattat aagggtatag 10080
taattaaaat agtttggtat tggtattaaa atagatatat agattaatgg aatagaatag 10140
aggttttaga aataatgtta tatatttata attattcgat ttttgataaa tttgataaaa 10200
ataggtaatg gggaaaggat tttttatttt ataaatggtg ttgggaaaat 10250

<210> 101
<211> 10682
<212> DNA
<213> Artificial Sequence

<220>
<223> chemically treated genomic DNA (Homo sapiens)

<400> 101

tgtttgagtt tgggaggtag aggttgtagt gagttaagat tatgttattg tattttagtt 60
tgggggatag agtgcgattt tattttgaaa aataaaaaaa tattaatatt attattatta 120
taatttgtag aaatgttgat taagtgtggt ggtttacgtt tataattttc gtattttggg 180
aggttgaggt aggaggattt tttgagttta ggagttgaag tttaggaatt ttagattagt 240
ttgggtaata tagtaaaatt ttatttttat aaaaaaaaaa aaaaaaaaaa ttgttaggtg 300
tgggtgtggc ggtgggtttt tatagtttta gttatttagg aggttgagat ggaaggattt 360
tttgagttta ggagtttaag gttgtagtga gcgatgattg tattattgta gtttagtttg 420
ggtaatacgg taagattttg ttttaaaaaa taaaaattaa taaaaaaaaa aaaatttttg 480
tagaattgta gaaatgtaga gtgaaattta taatatgata ttgggttttg gggttatatg 540
tagagtttat tgagtataat aggattgggt ttttttgtta atagaattta tttatatatt 600
gtaatattat tagataaggg aattttaaat tattattagg aaattattaa gaaatagcgt 660

atgggtagat tttaatacga atttattttt cgtttaatta gtttttaaaa agtaagaatt 720
ataatataac gtaaaaagtt aatgtttggt aggtatataa ttatttgtgg gttgttaatt 780
ttataagtat gaatatttgg cggttgtatt gtagtttaaa tgagagggta taatgaaatt 840
tttaagagta ttgataaagt ttatgggagg ttttggagtt ttttaaagag agagttttta 900
ttttttttat gtattgttta tagtttttat tttttttagt ttgttttttt ttttaagttt 960
taagcgttga cgttgttata gttttattag gttagttttt ttttcgattt tttttagttt 1020
tttatttttt ttagttttta gtagtttaat tatcgtttat aaatttcgtc gttggtattc 1080
ggggtttttt aggtaataat gtgtgggttt ttgattggtc gcgagattta ggagggttgg 1140
ttataaaatt gggtacgggg ataaaattaa taagtgtggc ggttaagagc gtgggaggga 1200
ttgtaatgtt ttaatataat tttagaatga agacgcgtcg aattttgaaa taagtagcgt 1260
tgaaaatttt tatttaagac gtagtgtgtt ttaaattagt tttatttacg gagtacgttt 1320
agttatattg ttttggttat ttcggagttg cgaaatgtag gttttattgt gttaggtttt 1380
aagagtggtt aataggtaaa tagagggttg gaggaaaaag aattcgagat tggtttaatg 1440
aaagaatagt atttgtaata ggaagtggaa ggatggggtt aggaggaatt gtttaatata 1500
gagatatata aatgttttaa tgtaaatttt aatttattgg attatagaat ttaggttgat 1560
tgatagatat attagggaat tagtagatgg tatgttttaa taagaataat taaatttagg 1620
aatttttaga atttcgttta atttattaaa tttattgagc gtttgttttg taatacgaag 1680
taaaatgaga atataaagat aaaaaagaaa tttaaagagg aagattggaa ggtgtaatat 1740
ataaatattt aatggaaggt ttatacgggt aatagttgtt atagagttat gaataagtgt 1800
tggaggatat taaaaagttt aaaatttcgg aatagaggtt ttttggttaa tgtaaagtag 1860
gttttatagg tagtagttaa taataataaa aagtaaaata atagtaaaaa gagatttttt 1920
ttattatttt ttttttttaa aataaataag aatgtatttt ttttttattt aaatgtataa 1980
atttgtgttt tttgttgtgt tttataggtt ttatttttat ttttgggggg aacggagttt 2040
tgtttttatt gtttaggttg gagtgtattg gtgcgatttt aggttattgt aattttcgtt 2100
tttcgggttt aagcgatttt tttgttttag ttttttgagt agcggggatt ataagtatgc 2160
gttattatgt tcggttaatt tttttgtatt tttagtagag acggggtttt attatgttgg 2220
ttaggttggt ttcgaatttt tgattttagg tgatttgttc gtttcggtat tttggagtgt 2280
tgggattata ggcgtgagtt atcgtgttcg atttgtttta tagtttttaa ataggtagta 2340
tttgtatatt tttagtttaa aaagaaaaaa atgaagttta tagaagttgt tgtttttttt 2400
gggttaaaat ggattagtaa gtgggtaagt tgaaattaga atttaggttg tttgaatttt 2460
agtttaggga ttttttttat atgatcgggt tgtatttaaa tgaataaata aagttttggg 2520
ggtagaggcg gtgagggggt ggtttggagg aggtatttag atgtaatatt aaattattat 2580
gtattattag ttagttttta tggttttaaa acgggtttat ttagaaagag attagtttta 2640
ataatgttgg gagtgttgtg tttttttatg aagttttcgt ttattaaaga tttcgtattg 2700
gtggttataa tatggaatga aagagagatg ggattataga gagtattttt tttagttttg 2760
ttggatttga atatttttat ttttgaaaag aaggtttttt tttttttgtt ttttaattaa 2820
tattttttgg gttttttttt gttttgtatt ttattattag attattgttt ttttaaaaga 2880
atttgagtta tattatttat attgtttgtg tttatagtga gtatatagag ttttatgttt 2940
gatattgtaa tattattttt tttttttttt taagagatag ggatggtttt gttgtttagg 3000
ttgtagtgta gtggtttatt atagttttga atttttgggt ttaagtaatt tttttaggtt 3060
aattttttag tttttatttt tgtagagata ggatttgttt atgtttttta gattggtttt 3120
taatttttgg tttaagtgat tttttttttt cggtttttta aaatgttggg attataggtt 3180
tgaattattt tatttagttt taaatattaa ttatttttaa gagggatatt ttaaatataa 3240
agaaagattt ggatattttt ataagaaata tttttgattt tttacgaaga ttttttttaa 3300
atagtatatt tgtatgtatt ttatagatat agtagaagtt ttttttgttt tatttttagg 3360
ttttattttt ttttttgttt gtttagaggt agttattgtt ataaaattgg tgtgtagcgt 3420
ttttgtttat ttttatgttt ttattatatg tacgtttgtt tttataaata ttgtatagta 3480
ttgttagttt ttttttttta aagaggtatt attatttata tattttgtgg tttgtttttt 3540
tttttttatt taatgttttt tttttgtaat ttagttatgt tgatatagtg gcgattattt 3600
ttacgttatt tttatttttc gatgttgagt tattatgatg tgttttattt ttgatagttt 3660
ttataggatg gtgtgtaaat tgaataaaaa tgtatttcgt agtatatttt tagaggtttt 3720
ttagttataa gggatttgat atatatagat tttttttacg ttatttgttt ttttatttat 3780
tttttataga ggtatcgttg gttttagagg atatattgtt ttgtttagaa ttaatttaag 3840
aattaatatt attttaaata ttgtttattg tataagtatt tatttattag agaaattgta 3900
attgttattt tagattttat ttattaggtt tgtttatata gtttattttt tgatgtgaaa 3960
ttttaggttt aagtttaata ttcgttaata tttatttaat acgtattaag cgttttttta 4020
aaaaaaattg taaaatatat ataatataaa tgtattattg taaatatttt taagtgtata 4080
attttaatag tttatttata tcgtttaatt tttttaagtt ttatgtgtta tcgatagaat 4140
aaaatgatgt ttatgaaatt taggttgacg aggaacgaaa aagaagagaa agaaaagata 4200
ggttaaacgg tgtagtgaaa agaaagatag ttttaggttg ggacgagttt aggttttgtt 4260
gttaaaacgt tgtatggttt taggtaaatt atttaatttt tgggggcgtt ttttttttcg 4320
tttgaaaaat gagaggttcg gtttagatta tttttaagaa tttttttagt tttaaagtag 4380
tttatgtaga aaaggagagg agaaatattt tttaaggagt aaaaattggg gcgcgtatta 4440
gtaacgatta gcgttatttg ttttttaagg ttttggttaa gaggttatga attatttcgg 4500
gcgagtagtt tattttgttc gttacgttgt ttcgcgtgga gttttgtagt aggttagagt 4560
ttttgtattt tattttattt ttagtattcg gggatttgtt ttttgagtat tttaaatttt 4620

taggttcgga ggtcggtatt tttggttttt agattaggtg taggtggttt tgtaattata 4680
ttttcgcgtt gtgttattag gggacggagt gtattgcgag gagggtttcg agtgtatttt 4740
agatgagcgt ttttagggtt gggaacgagt acgtcggagt aagggagttt taggcgttat 4800
ttagggcgag aggtagagaa taacgaggcg cgtcgggtta ggttcgtaag tttaggattc 4860
ggtagggagt tttcggtcga gcgtatttag tgcgtttgtt tcgtgcgtgc gcgcgcgttc 4920
gtgagttcgt ttttcgggtc gcgcgtttcg gtgttcgcga aagttcgttg tcgttgggag 4980
gcgcgcgtcg gcgtttttcg gtcgtttcgg gtatttgagg gacgcgcggt cgttcgcggt 5040
aggcggtgta gtttttttat tttttggagt taggcgtcgg ggtttgaggt gagtttcgcg 5100
aagcgtgtgg gtttttgggt cggggtagcg ggagaaggag cggcgagtgt ggagttttaa 5160
gggaggcgtc gcgcggttgt ttgttgtttc gttttagttc gcgtcgagga gttagtagta 5220
gttcggcgtt ttttttttag ggatcgtttt tttcgtcgcg gttataataa aagttttggg 5280
cgtcggattg tggcgggttg cggagcgggg gttcgggttc gcggtggcgg ttgggggttt 5340
cggggacgta tagtttgagg ttgggggttg agggttgagg gttaaggggg gaagttgttt 5400
tcgggagatt gttaattttg ttcgttttga gttcgcggtt cggtaatttg tgcgagtgtg 5460
gatcgcggtt cgttcgtttt tatttcggtt tagattttgc gcggcggaga gaggggttcg 5520
gtcggagtcg gcggtagcgc ggggtgtcgg gggtcgtagg cggaagataa tgtttcgggg 5580
gttttcggtc gttcgaagcg cgcgtcgtta ggttttcggt ttcggttcgg gttagtttcg 5640
ggtttatttt tgtttgttta cgtttgaaag tttttttttt cgtttattag tttgtaaagt 5700
ttttgttttt tttcgcgagt ttttttcggt tgtttttttc gcgggttgtg ggtttgtcgt 5760
ggggtggttg ttgttggatt ttcgagggtt cgtatttgaa atgaagttgt tgtgaaatag 5820
ttagtaaggt gaaggagttt tgtgtttttt taagatatgt aaatattttt ttagcggtgt 5880
tttatatttt aggagttttg agttcgattt ttttgttgta agagaagtta agggtgtttt 5940
ttttggtttt gtttttaaga ataaaagtac gtgttatgaa ttgaggtaga ggagtagatt 6000
ttagatttag agagattggt tttatgaagg aggttcggcg gtttgtttag tgtagaatga 6060
ttttttgaat tttggttttc gtttgggttg agggagatgt agtagtagta tagtagagta 6120
gtcgtattag tgattgttac ggggaagtgt ttagatatag ttttatttta gagaagtagt 6180
tttgatcggg aatgtttatt gatgggaatt gagttatttt gtaaagttaa tatgatatga 6240
gtaaggaaat aaagatttga ttatttttgg ggatgttatt tttttttttt attttttttt 6300
agtttttggt ggtgtgttgg tgttattttt gtattttagt ttggaaaaag aatagaagtt 6360
tttagtttta ttattaagaa gtggttattt ttgaagatga tgtttttcgt ttttattttt 6420
aaataaatta atattatttt ttttttttag aaaaaaggat aaaaatttat aaagataaaa 6480
aaaatttaga gtcggagagt ttaggaaaga ggtgtaagag tttgattggg tggggttgga 6540
aggataaaag agttgaagat aaaatataat taagatgtag tttttttttt attatatttt 6600
tttattttat tgtttgaaat tattaggaaa ataagtaaat tattggtaaa gtttattatt 6660
ttattttttt ttttttttta tttttttttt tttatttatt attaaaattt tttattttta 6720
ttttttttaa atcgttttat ttataaaagg agaaggataa tgagttgttt ttttatttta 6780
gttgttttat gtttttgagt tttattttgt gttttgagta tttaggtggt tattttttgg 6840
gagtttcgtg attgagatat ttgaagtaga gagggttagg ggaatttaat agtaaagtgt 6900
atattttttt tttgtataga ataaagtgaa agttttatta ttggggaagg agagaggagt 6960
ttttcgttta taataagtta tttgtttttt aggaaataga tttaaatttt atgttagttt 7020
gatagtgaaa ttttaagatt ttatagttaa aatttttttt gagtatgtga gtttgttgat 7080
gttaagttgt tgttgatttt tttttttttt agagatagat agtaggaaat aatttttatt 7140
atttttttga gcggttttgg ttttaagttt tgtttttgtt tttgtttttt ttggtttttt 7200
ttgttttttt atttttagaa ttttagaatt atgtatgatt aaaattgtag ttgtgcgtaa 7260
aaataaaaag aatgatttta aaaatatgta ttggataaat tatttagtat agattggagg 7320
aaaatgtgtg gtagagtttc gaggaaagat attagatttt tagtagtgat atttttataa 7380
tgttaggaat aaatgaagtg taatagtttt ttattggtta ggaaagagtt ttatttatta 7440
tgttaaaaaa tgagtttttt ttttttgttt tttaattcgt tatgttggga attattgata 7500
gtttggtata tttaaggagt gtattgtacg tgaagtttga gatgaatgta attaggttat 7560
tgggtttgaa aattaaattt tggtttttag attttatttt attaaaataa ttttgtagtt 7620
aaagtatgta aattttataa atttgttaag aggggtggaa agtaggggtt tggtattaga 7680
gaattttttt gatagtagat ttaagagaag ttgggttgaa attttagttt attttaaagt 7740
ttagaattta tttgtttttt aattttttag ttttattttt aggttaattt ttaaagattt 7800
tttgtttttt ttagttttag taattaataa tttttaaatt gaattgaatt ttgatgttta 7860
ttattcgttt aaattttgaa tttagatgga tttatttttt ttttgtgtgt gtgttttaag 7920
ttttaattat ttaatatatt ggaagattaa gttatttgtt gtaattagta gatgatgata 7980
aatggatttt aaatattaga tgaagtagga aagtttagaa aagagatatt ttgagattaa 8040
taggaattta ggttttgtga attaaatatg aagttatata aatttttttg tagtaattag 8100
taggtaaagg gttaaagatt atatagattt taagagaagg aaagtaatat ttgagtaatg 8160
agtgtatatt gagtgtatat tttgtttaat aagataataa tttttttaag aagtttttat 8220
ttagagatag ttattgttaa tttttgtaat gcgtttttga aaacgtgttt gaaaaaagat 8280
aaagaatatt ttttttgttc gttttgtagg tattgtgtaa ttagtattta ttttgaaata 8340
atttttagat gatttttttt aattggaaat gtaaaatgtt atttgtattt attaggtgtt 8400
aagaaaatga aattttaata gaatgatgga atggagtttt ttttgtagag aaagtgtgaa 8460
ttggaataga gttgttgaat ataatttttt gttgtagaat ttttttgata agtttttagg 8520
gttttttttt tttattattt ttttttttgg gatttagttt gttttttttt ttatagttgt 8580

tattttttgc gttttttatc gagttttttg ggtttgattt tgtaggtagt tcgggaagat 8640
agttttttaa gattggaatt tttttttttt attgttgttt tttaggataa atatttagtt 8700
gattttattt taaagatttt tattttttgt tattaaaaga aatttgataa ataatggttg 8760
gaaatataaa atttttgatg ttatatattt ataaaaatag aaatgggtaa gttttaaagt 8820
atttagtttt ttttgttttt tgatggaatg ttaattaagt aatttagttc gaaattagtt 8880
ttttagatat tttgtattgt tttatggaga agtgaatgtg tttttttttt tatataaagg 8940
gcgtgagtaa ttgttgtata gtatttattt tttgtagggt tttggttttt gaagttgttt 9000
atttcgatta tgttaattta ataggttttt tttttttttt tttttagttg atatattata 9060
gttttttata ataaaaaaaa aattagtaag aacgttttaa atgattgtta atttttattt 9120
aatgatttgt tatattttga taggattttt ttgggtttag aatttttgtt atgttttgta 9180
agtttggaat tttaatttat agttttttta tggatgattt tattttagga aaaatggtat 9240
atttatataa ttgtgattag aagtagataa tttattatgt tattgaagat atattatttg 9300
gaatgtttat taaaagtttt tattttttta ttttatttaa tttttttgag atagttttat 9360
tttgttattt aggttggatg tagtagtata gtttttattt atcgtaattt ttatttttta 9420
tgttgaagta atttttaagt tttttttgaa tagttgggtt tataggcgta tgttagttaa 9480
tttttgtatt tttagtagag atgggttttt gtcgtgttgg ttaggttggt tttaaatttt 9540
tggttttaaa tgatttgtat gttttcgttt tttaaaatgt ttggattata ggcgtgagtt 9600
attgtgttta gttaggaatg tttattaaaa gtttttaaat tgtgtatatt atttgattta 9660
gttgattggt tatttgattt ttttagtatt gaaaattaga ttttgatttt tattaaattg 9720
attattattg atttgttttt tttattaggt atagtagagt agtagtgtat tttttggttt 9780
gttgtttaat tagtagttaa taaattagtt attttattag tatttttttt tgttagttag 9840
ttagtatata tttatagatt tagtagagtt aattatttta tatataaaat aatgtattat 9900
aggttttttt ataaaatttt ataaagtatt taatatttta tttttaaata atcgaaatag 9960
ttttattgtg ttatatgtat atatatattt tttgataaaa tgagagatat atatatatat 10020
tgttttgtaa attgttttta aattttattt tttatttatt gttattattt tattgtattt 10080
ataaatatag gtttatgttt attattttta gtaattatat aatattttat tatgtggtta 10140
tattataatt tatttaatta atttttattt ggtagatttt ttagttgtta ttagttttta 10200
gggttttttt tgttataaat agtgttatat taatattttg gttgtgtatt atgtgtattt 10260
acgtgtttat ttagaataat aagatttgtt ggatgaaatt gtgtatattt taagggtttt 10320
tgatataagt agttattttt ttttttaagt attaattagt ttattaataa tatttgaaaa 10380
tgttgaaata ttgaaggtgt tttttatttt gtatttttat tttatattgt gttttattat 10440
tattgttttt ttttatttgg ttggtaaaaa attttatttt atatttgtat tttttattat 10500
tagtgaagtt gaatattttt ttatatgttt tttggttgtt tgtatttttt ttgtaaattt 10560
atgttttttg tttatttttt tattaaaatg ttatttttta tttgttttta tttttaaaat 10620
ttatttattg tatataatta tataaataat atttttattt tttttaatta ttttgtttta 10680
gg 10682

<210> 102
<211> 10682
<212> DNA
<213> Artificial Sequence

<220>
<223> chemically treated genomic DNA (Homo sapiens)

<400> 102

tttagaatag aataattaaa aagaataaag gtattgttta tgtaattata tatagtaaat 60
gaattttaaa aataaaagta gataggggat aatattttaa tagaaaaata agtaaaggat 120
ataaatttat aaaagaaata taaatagtta agaaatatat gaaaagatat ttaattttat 180
taataataaa aaatgtaaat gtgagataag attttttgtt aattagatag gaaaaaataa 240
taatgataga atatagtata gggtgggagt atagggtaaa gaatattttt aatattttaa 300
tatttttaaa tgttattagt aaattagttg gtatttaaaa aaaaaagtgg ttatttatat 360
taaaagtttt taaaatgtat ataattttat ttagtaaatt ttattatttt aagtaaatac 420
gtaagtatat atgatgtata attaaaatat tagtgtaata ttgtttataa taaaaaaaat 480
tttaaaagtt ggtaataatt gaaaagttta ttaataagaa attggttaaa taaattatgg 540
tataattata tgatggaata ttatatagtt attaaaaatg atgagtataa atttgtattt 600
gtggatatag taagatagta ataataaata aaaaataaaa tttaaaggta gtttataaaa 660
tagtatatgt atatattttt tattttgtta gaaaatatat atgtatatgt aatatagtaa 720
aattatttcg attgtttaaa aataaaatat taaatgtttt atggaatttt atgaaagaat 780
ttatagtata ttattttgtg tatgaagtaa ttaattttgt taaatttgta aatatgtgtt 840
gattggttga taagaaaaaa tattagtgaa atgattggtt tattgattat taattaagta 900
ataagttaag gaatatatta ttgttttatt gtatttagta agaaaagtaa attagtgatg 960
attaatttag taagagttaa aatttgattt ttaatattaa aaaaattaga taattaatta 1020
attgaattaa atggtatgta taatttaaaa atttttgata aatatttttg gttgggtata 1080
gtggtttacg tttgtaattt aagtattttg ggagacgaag gtatgtagat tatttgaggt 1140

taggagtttg agattagttt ggttaatacg gtaaaaattt atttttatta aaaatataaa 1200
aattagttgg tatgcgtttg taggtttagt tatttaggag gggtttgaga attgttttaa 1260
tatgagaggt ggaggttgcg gtgagtggag attgtattat tgtatttagt ttgggtgata 1320
gagtgagatt gttttaaaaa aattaaataa aataaaaaaa taaaaatttt tgataaatat 1380
tttaaataat atgtttttaa taatataatg aattatttgt ttttaattat aattatgtga 1440
gtatgttatt ttttttaagg taaggttatt tatggaaaag ttgtaagttg aagttttaga 1500
tttgtaggat atagtaaaga ttttggattt aaagagattt tattaaaatg tagtaagtta 1560
ttaaataaaa gttaatagtt atttggggcg tttttattga tttttttttt attataagaa 1620
attgtagtat attaattgga aaaaaaaagg ggggggattt attagattaa tataatcgga 1680
gtgagtagtt ttaggggtta gggttttgta aaaagtgaat gttatgtaat agttgtttac 1740
gttttttgta tgggaaaaga gatatattta tttttttata gagtaatata gaatatttgg 1800
aaagttgatt tcggattaag ttatttggtt aatattttat tagaaaataa aaaggattag 1860
atattttaaa gtttatttat ttttattttt atgaatgtat aatattagga attttatatt 1920
tttaattatt atttattagg ttttttttga tgataaaaag taaaagtttt taaaataaaa 1980
ttaattaagt atttgttttg aaagatagta ataaagggaa gggattttaa ttttggaaga 2040
ttgttttttc gggttgtttg taaaattaga tttaaagaat tcggtgggaa acgtaggagg 2100
tggtaattgt gaagaggaaa gtaggttgaa ttttagaaga aaggatgatg agaaaagagg 2160
gttttaaggg tttgttaaag gggttttgta ataaaagatt atgtttagta attttatttt 2220
aatttatatt ttttttatag aaagggtttt atttattatt tttgttgaga ttttattttt 2280
ttgatatttg ataaatgtag gtaatatttt gtatttttaa ttgagaaaga ttatttaaag 2340
gttgttttaa agtaaatgtt ggttatatag tgtttataga gcgaatagga gggatatttt 2400
ttgttttttt ttagatacgt ttttaagaac gtattgtaag gattagtaat aattattttt 2460
aaataagagt tttttaagga aattattgtt ttgttagata aaatatatat ttagtgtata 2520
tttattgttt aaatgttatt tttttttttt taaagtttat atagtttttg gttttttgtt 2580
tattggttgt tataagaaag tttatatagt tttatattta atttatagga tttagatttt 2640
tattaatttt agagtatttt ttttttgaat ttttttgttt tatttaatgt ttaaagttta 2700
tttgttatta tttattgatt ataataaata atttaatttt ttaatgtgtt aagtgattaa 2760
ggtttaaaat atatatatag aggaaaagta aatttattta ggtttaaaat ttaaacgaat 2820
ggtaggtatt aaaatttagt ttaatttaga aattattaat tattaagatt aaagaagata 2880
gaagattttt aaaaattaat ttgaaaataa aattggaaag ttaagagata gatagatttt 2940
ggatttaaaa atgggttgaa attttagttt agtttttttt aaatttgtta ttaaaaaaat 3000
tttttaatat taagtttttg ttttttattt tttttaataa gtttgtaagg tttgtatgtt 3060
ttgattatag aattatttta atgagataaa atttggggat taggatttgg tttttaggtt 3120
taatagttta gttatattta ttttaaattt tacgtgtaat atattttttg aatatattaa 3180
attgttaata gtttttaata tgacgaattg gaaagtaaaa agaggaaatt tattttttga 3240
tatagtaggt aaaatttttt tttagttagt aagaagttgt tatattttat ttgtttttaa 3300
tattatagaa atattattat tgaaaattta atgttttttt tcgagatttt attatatatt 3360
tttttttaat ttatattgaa taatttattt agtatatatt tttaaaatta ttttttttat 3420
ttttgcgtat agttgtagtt ttaattatgt ataattttag agttttgggg ataggaaaat 3480
aaaaaaaatt aaaaagaata aaaataaaaa taaaatttga ggttaaaatc gtttaaagaa 3540
gtggtaagaa ttgtttttta ttatttgttt ttgaaggaaa agaaagttaa tagtagttta 3600
atattaataa atttatatgt ttagaaaaag ttttgattat aaaattttag agttttatta 3660
ttaaattaat atgaaattta agtttgtttt ttgggaaata gataatttat tgtgagcgaa 3720
aaattttttt tttttttttt agtgataaag tttttatttt gttttatata aagaaagaat 3780
gtgtattttg ttattaaatt tttttagttt tttttatttt aaatatttta attacggaat 3840
ttttaagaaa tagttattta aatgtttaaa gtataaaata aagtttagaa gtatagagta 3900
gttaggatga ggaaatagtt tattgttttt ttttttttat aaatgggacg atttaggaga 3960
gatggagata gagaatttta gtagtggatg agggaaaagg ggtaggagga aagagagggt 4020
agaatgatag gttttattag taatttattt atttttttaa tagttttaaa taatggggtg 4080
ggggggtatg gtagaagaaa ggttgtattt tagttatatt ttatttttag tttttttatt 4140
tttttagttt tatttagtta agtttttata tttttttttt aggtttttcg attttgaatt 4200
ttttttattt ttgtgggttt ttgttttttt ttttggagga aaaagatagt attgatttgt 4260
ttaaaagtaa agacggaaag tattattttt aggagtaatt attttttaat aatagaatta 4320
aggattttta tttttttttt agattaaaat gtaggaatgg tattagtata ttattaagga 4380
ttaaaggagg gtgagggaag gaggtaatat ttttaggggt agttaggttt ttgttttttt 4440
gtttatatta tattggtttt ataaagtaat ttaattttta ttaataagta ttttcgatta 4500
gaattatttt tttaagatga ggttgtattt aaatattttt tcgtaatagt tattgatgcg 4560
gttattttgt tgtgttgttg ttgtattttt tttaatttag acgggaatta aaatttaaag 4620
agttatttta tattgggtaa atcgtcggat tttttttatg gagttagttt ttttaaattt 4680
gaaatttatt tttttgtttt aatttatggt acgtgttttt gtttttaaaa ataaagttaa 4740
gaaaagtatt tttggttttt tttataatag agggatcgaa tttaagattt ttaagatgta 4800
aaatatcgtt aggaagatgt ttatatgttt tgaaaggata taaggttttt ttattttgtt 4860
ggttatttta tagtagtttt attttagatg cggattttcg gggatttagt agtagttatt 4920
ttacggtaaa tttatagttc gcggaggagg tagtcggggg aagttcgcgg aagggagtaa 4980
ggattttgta aattgatgga cgggagggga aatttttagg cgtgggtagg taaaggtggg 5040
ttcgaagttg attcgagtcg gggtcgggag tttggcggcg cgcgtttcgg gcggtcggga 5100

gttttcggag tattgttttt cgtttacggt tttcggtatt tcgcgttgtc gtcggtttcg 5160
gtcgggtttt ttttttcgtc gcgtagggtt tgagtcgggg tagaggcgag cgaatcgcgg 5220
tttatattcg tataggttgt cgagtcgcgg gtttagggcg gatagggtta atagtttttc 5280
gggggtagtt ttttttttta gtttttagtt tttagttttt agttttaaat tatgcgtttt 5340
cgaggttttt agtcgttatc gcgggttcgg gttttcgttt cgtagttcgt tatagttcgg 5400
cgtttaaggt ttttgttgtg gtcgcgacga gaggaacgat ttttgaggag gaggcgtcgg 5460
gttgttgttg gtttttcggc gcgggttgag gcggagtagt aggtagtcgc gcggcgtttt 5520
ttttggggtt ttatattcgt cgtttttttt ttcgttgttt cggtttaggg gtttatacgt 5580
ttcgcggggt ttattttaga tttcggcgtt tggttttaag ggtggggggg gttgtatcgt 5640
ttgtcgcggg cggtcgcgcg ttttttaggt attcggagcg gtcgaggaac gtcggcgcgc 5700
gttttttagc gatagcgagt tttcgcgagt atcggggcgc gcggttcgga gagcgagttt 5760
acgagcgcgc gcgtacgtac ggggtaggcg tattgagtac gttcggtcga aagttttttg 5820
tcggattttg ggtttgcggg tttggttcgg cgcgtttcgt tgttttttgt ttttcgtttt 5880
gagtgacgtt tggagttttt ttgtttcggc gtgttcgttt ttagttttgg aggcgtttat 5940
ttgagatgta ttcggggttt ttttcgtaat gtatttcgtt ttttgatgat atagcgcgga 6000
ggtgtgattg taggattatt tatatttggt ttgagaatta gaggtgtcga ttttcggatt 6060
tggggatttg gggtgtttaa gaggtagatt ttcggatgtt gagggtggaa tggggtgtag 6120
gagttttggt ttgttgtaga gttttacgcg aaatagcgta gcggatagaa taggttattc 6180
gttcgggata gtttatagtt ttttggttaa gattttagga aataggtggc gttggtcgtt 6240
attggtgcgc gttttaattt ttgtttttta ggaaatgttt tttttttttt tttttatata 6300
agttgtttta gagttgaaag aatttttaaa gataatttaa atcgaatttt ttatttttta 6360
gacgaggaaa agaacgtttt taaaggttag atgatttatt taaagttatg tagcgtttta 6420
ataatagggt ttggattcgt tttaatttag ggttgttttt ttttttattg tatcgtttaa 6480
tttgtttttt tttttttttt tttttcgttt ttcgttaatt tggattttat gggtattatt 6540
ttgttttgtc ggtagtatat agaatttaaa gaaattgaac gatgtgaatg gattattaga 6600
attgtatatt taaaaatgtt tatagtggtg tatttatgtt atgtgtgttt tataattttt 6660
tttagaaagg cgtttgatac gtgttaagta aatgttgacg aatgttgaat ttagatttag 6720
agttttatat tagaaggtga attgtgtgaa taggtttgat aaatgagatt tgaaatgata 6780
gttatagttt ttttaataag taaatatttg tgtagtaaat aatgtttaaa ataatattga 6840
tttttaggtt gattttaggt agggtagtgt attttttgga gttagcggtg tttttgtgga 6900
gagtgagtga gggggtagat agcgtggggg aaatttgtgt gtgttagatt ttttgtagtt 6960
ggggaatttt tggagatgtg ttgcgaggtg tatttttatt taatttatat attattttgt 7020
aaagattgtt aggaatgaga tatattataa tagtttaata tcgggaggtg ggaatgacgt 7080
agaaatgatc gttattgtat tagtatgatt aaattgtaga aaaagaatat tgagtgaaaa 7140
aaaaaaaata aattataaga tatgtaagtg atgatgtttt tttagaaaaa aaagattagt 7200
aatattatat aatgtttatg agggtaaacg tatatataat aaaagtatga aaatggatag 7260
aaacgttgta tattaatttt gtgataatga ttgtttttag gtaggtaggg agggggatga 7320
aatttgggaa tgagataaaa gaagttttta ttgtatttgt gaaatatata taaatgtatt 7380
atttaaaaaa aattttcgtg gagaattaag ggtgtttttt atgaaagtgt ttaaattttt 7440
ttttatgttt gaaatatttt ttttaaaagt aattaatatt tggggttgag tgaggtggtt 7500
tagatttgta attttagtat tttggaaagt cgaggagaga ggattatttg agttaggagt 7560
tggagattag tttgggggat atagtaagat tttgttttta taaaaataaa aattaaaaaa 7620
ttagtttggg aggattgttt gagtttagga gtttaaagtt gtagtgagtt attatattgt 7680
agtttgggta ataaagttat ttttgttttt taaaaaaaaa aaaaaagtaa tgttgtagtg 7740
ttagatatgg agttttatgt gtttattgtg aatatagata gtgtggatgg tgtgatttag 7800
gtttttttgg gaggatagta gtttgatgat gaggtgtagg ataagaggag gtttagaaga 7860
tgttggttga gaggtaagaa gaaagagatt ttttttttag ggatgaaaat atttaaattt 7920
aatagggtta aaggagatgt tttttgtgat tttatttttt ttttatttta tgttgtaatt 7980
attaatacga agtttttaat gagcgggaat tttatagaga gatatagtat ttttaatatt 8040
gttaaaattg gttttttttt ggatgaattc gttttgaaat tataaaagtt aattggtaat 8100
gtatggtaat ttagtattgt atttaagtat tttttttagg ttattttttt atcgtttttg 8160
tttttaaggt tttgtttatt tatttaggta taattcggtt atgtggaaaa aatttttagg 8220
ttggaattta aataatttag gttttaattt tagtttgttt atttattgat ttattttaat 8280
ttaaggaaag taataatttt tatgagtttt attttttttt ttttaaatta gagatatata 8340
aatgttgttt atttaaaaat tataaaatag gtcgggtacg gtggtttacg tttgtaattt 8400
tagtatttta ggatgtcgag gcgggtagat tatttgaggt taggagttcg aaattagttt 8460
ggttaatatg gtgaaatttc gtttttatta aaaatataaa aaaattagtc gggtatggtg 8520
acgtatgttt gtaattttcg ttatttagga ggttgaggta ggagaatcgt ttgaattcga 8580
gaggcggagg ttgtagtaat ttgagatcgt attaatgtat tttagtttgg gtaataagag 8640
taaaatttcg ttttttttaa aaataaaaat aaaatttgta aaatataata gaaatatagg 8700
gtttgtatat ttaagtggaa ggggagtgta tttttattta ttttgagaaa aaaagatagt 8760
aaggaaagtt tttttttgtt gttgttttgt tttttgttgt tgttgattgt tgtttataag 8820
atttgtttta tattgattaa aggatttttg tttcggaatt ttgaattttt tggtattttt 8880
tagtatttgt ttataatttt ataatagttg ttattcgtgt gagtttttta ttaaatattt 8940
gtatattgta tttttagtt tttttttta gatttttttt ttattttgt attttattt 9000
tgtttcgtat tataagatag gcgtttaata aatttaatga attggacggg attttaagag 9060

tttttaaatt tagttatttt tattagggta tgttatttgt tggttttttg gtgtatttgt 9120
tagttagttt gaattttata atttaataaa ttgaaatttg tattgaagta tttgtgtgtt 9180
tttgtgttgg gtaatttttt ttggttttat ttttttattt tttgttgtag gtgttatttt 9240
tttattgaat tagtttcgaa tttttttttt tttaattttt tgtttatttg ttggttattt 9300
ttaaaatttg gtatagtgga gtttgtattt cgtagtttcg gagtgattag gataatgtga 9360
ttgggcgtgt ttcgtaggta gggttgattt gggatatatt acgttttgaa tgaaggtttt 9420
taacgttatt tgttttagga ttcgacgcgt ttttatttta gggttatatt gagatattgt 9480
agtttttttt acgtttttgg tcgttatatt tgttaatttt attttcgtgt ttaattttgt 9540
agttagtttt tttggatttc gcggttaatt aggagtttat atattgttgt ttaagagatt 9600
tcggatgtta gcgacgggat ttatagacgg tgattgggtt gttggaagtt gggaggggtg 9660
agggattgga gggagtcgga gaaaaagttg atttaatgaa attgtggtaa cgttagcgtt 9720
tgaggtttga agagggagat aagttaaaag aggtgagagt tgtgggtagt gtatgaaaaa 9780
gatggagatt tttttttttgg aaagtttag ggtttttat gagtttatt agtgttttg 9840
gggattttat tgtgtttttt tatttgggtt atagtgtaat cgttagatat ttatatttgt 9900
gaaattaata atttatagat aattatatgt ttattaagta ttagtttttt gcgttgtgtt 9960
atagtttttg ttttttaaaa attagttaag cggaagatga attcgtattg aaatttattt 10020
atacgttatt ttttgataat tttttgataa taatttgaga tttttttgtt tgatgatatt 10080
ataatgtatg ggtaaatttt gttagtagag agatttagtt ttattatgtt tagtaaattt 10140
tgtatataat tttagagttt agtattatgt tatgagtttt attttatatt tttataattt 10200
tataagggtt tttttttttt tgttggtttt tgttttttga gatagggttt tgtcgtgttg 10260
tttaggttga attgtagtgg tgtaattatc gtttattgta gttttgaatt tttgggttta 10320
aggaattttt ttattttagt tttttgagta gttgggatta taggagttta tcgttatatt 10380
tatatttggt aatttttttt tttttttttt ttgtagagat agggttttgt tgtgttgttt 10440
aggttggttt ggaatttttg ggttttaatt tttgggttta agagattttt ttgttttagt 10500
tttttaaaat gcggggatta taggcgtgag ttattatatt tggttaatat ttttataaat 10560
tgtggtggtg gtggtgttgg tgtttttttg ttttttaaga tggagtcgta ttttgttttt 10620
taggttggag tgtagtggta tgattttggt ttattgtaat ttttgttttt taggtttaag 10680
ta 10682

<210> 103
<211> 7922
<212> DNA
<213> Artificial Sequence

<220>
<223> chemically treated genomic DNA (Homo sapiens)

<220>
<221> unsure
<222> (4850)

<400> 103

ttgtgagtta agattatatt attgtatttt agtttgagcg atagagtgag attttatttt 60
aaagaaaaaa aaaagatatt tgtttattgt aaaagattta agaatagata tagttaaata 120
aaaaggaaga taaataattg gtaaaatatt tgatttgaga agaaagttga aagtgtgaat 180
tatgtttgat ttggaggagc ggtttataat tttgatttaa ttattatttt ggaattattt 240
aggtagtgtt ttggcggttg tttatattcg gtttataatg taggtttttt ttttttgaat 300
aattagatta tttataggta gaattattta tttatgtatt aaataaataa ttattgagtg 360
ttttttatgg tttaggtaat attgttttat gttgtttaag agtatggtag tgaaaaagat 420
atagtttttg attttagagg agagtgaaag agataaaagt aaaatagttt aataatttta 480
taatatagaa ttttatgtaa aataataagg ggagattgga aataagaaag tttaataaaa 540
tgtatttaat tataaaaaat ttgttttgtt tttggtttta tgttttaatt ttttttaatg 600
tgattgtaag ttttttgaag gtagaaatta tttttaatat gtttgtatat tttttagtat 660
ttagtttaat gatatgttta taataattta ttattaaata tttgttaata gtttgattga 720
ttaattttat tgaaacgaaa cgaggttatg gaggtaaaag tgtgtttgta ttattttttg 780
ttttttatgg aggtttttgg ttgtttattt ttgcgttaat ttattattat taataaataa 840
ggatgtttga tgtattattt aatgtttgtt aaatttatgg gtaggaaaaa ttttgagttt 900
ttattttttg atatgtaaga gttttgtggt gataattttt ttttgttagt tagtggtgta 960
atggattaag aagggagttt ggttttgtta tttaataggt attaattttt tgggaatttt 1020
gattttttag ttcgaagtaa gattagtgtt tttttttatt ttatttagta ggcgtatttt 1080
tgttttataa ttagtttggg tgtaggggga gatgtaatat tttatttata aatggatgga 1140
atgtggtatg gtattttttt gttttgtttt tgttgtttta tagtaaattt ttaatattta 1200
tggggtttta gttttagtga attatttttt ttaaaaatta ggtagataat aatgaattat 1260
ttttttgtat tttttattaa gatagttttt tgtgagtttt aaatgtttta tttagattta 1320
aagaagttgg tttttttatg agtggttgga ataagatttt ttttggaagt tattgaatat 1380

ggattagttg gattgtttta taggaaataa aaagtaggtt ttagggtaat tattttagta 1440
tttaattggg ttagtaaaga gtcgtaaatg agagattttt tcgtaggagg gaaaggatgt 1500
ttatagtttt tattttttta ggtgatgtat tttaatttaa atattatttt tttaatgtag 1560
tttttaattt agtaagtatt ataagaattt attagagtag aaaacgttag gttgaaaaat 1620
taatatatta tcggagttaa taagaattta gtttttattt agatataaat ggagagtttt 1680
ttaattgttt aatagattgt tgaagttatg tttgttgtag ttttaaagta gttgaatttt 1740
tattgtttag aggttggatt aaggaaattg tttaggtatt taagttagag ttagtagggg 1800
tttatggttt atattttatt taggtgtttt tatttttttt ttttagaaat gttgtgtatg 1860
ttatagggtg gatatttttt tgtttgggat tttttcggga agtgtttttt gtaataaaga 1920
taaatatgtt atgtttgtta ttaggggtag attgtggatt gtgtaaatta ttttaggaga 1980
gggaggttga ggggttttag gttatttaga tttagtttta agttatggtt tttgttgttt 2040
ttttatattt agattgaaat gtgatttttt tagttttttt ttttggaaaa tattggtgat 2100
gagttttacg attagaaaga aaagttgttg atagaaaaag tttttgatag ggaaggggtt 2160
tttttatgtg tttttaaagg agataattgt tttaagtaag ttgaggtagg atgttaggtt 2220
taggaataag aggtttagat tataaaatgg ttatttttaa ttatggatta ggttgggcgt 2280
gaagtagttt tttttgtaag agtagtatta tgtatataga gtttagggtt aggtagatat 2340
gtatgagttt ttttttacga tttattgtgt aattttgggt aaattgtttg gttttttaga 2400
gtattagttt ttgtaagttg tgagaaataa ataaaattaa gaatgtaagg tatgtaatat 2460
agtgtttgaa agtaagagtt ttatatgagt ggtttgttat tgatttgaaa atagtatttt 2520
tttatttaga ataaatgatg ttttttgtgt gttaggtttt gtatgaggga taggtttttt 2580
tttttgattt tagggagttt attgtttaat aaattatgta attatatttt tagtataaag 2640
tatattttat tattattatt tttttttaga agaaggttaa attgtatatt aattatattt 2700
tgttgagtag ttacgagttt tgtaggttat ttttttttga gtagtttttt agatttagtt 2760
atttttattt ttaatttatt tataattttt tatttgtgta tttagtaaat taagttattg 2820
ttgtttgtta attttttgtt tttttttgtg agatttaaaa ggatgaggat tatttattta 2880
tttgatttgt ttatttgatt atatatttat ttttgtaatt taaatgatta atagattatt 2940
tgatttttat atttgatcgt ttatttattt ttgtaggtta agggttttat gtattattta 3000
gtataggtat atagagattt aattttattg ttgagtatat tgatttttag ttgggaattt 3060
tttttgggaa aatagttttg ttttttattt ttgaagagtt ttttttgggt tttggtttgg 3120
ttggttttgt tattttttat tattgtggtt ttagttttag ttttttaatt tttataagga 3180
gttaacggaa ttttttttat tgggagttta tgatgatgat tggagataat gtagtttttt 3240
cgtgtttaat atagttttag gtatagagtt aaatattaaa aaaaagttga ttttattttt 3300
gtttttagtt aagtttcgtt gttaatataa gaagattaaa aatataagat agaatatgag 3360
tgttatatga gtggtataga tattttttta atttgtattt agtttttgtt atatgttata 3420
gaattggttg aaatgggagg ataaaaatat gtataaaata gtggtaagtg ttggataaaa 3480
agattttagg aagagggtga aaagtttttt atttagattt ttatttcggg gttgttggag 3540
ttgtttttag tattaggttg gaaagtagga ggttgtttta taattgattt tttttagttg 3600
ttttggaaat atatatatat atatatatat atatatatat atatatatat attttaatta 3660
ttaagttttt tagtatttat tatgtgttag atattgttaa atgatttata ggtattattt 3720
tattgaattt ttagaataat tgtgtgaggt ggatatgaat ttttttattt tatagtcggt 3780
tattttgagg tttagagagg tattatagtt ttaatatagt taagtaataa gtaaacggtg 3840
gaggaattta tttgttggga gtttattggt cgattggaaa gtagagagag ggtggattag 3900
tataaagtcg cggggggttg gtaatgagat aggaaagttg ggtttaaatt tgggttatat 3960
gatttttaga tttttaggag tcgaagtaga agtaaagaaa aaaaggggtg tagtttggtg 4020
gggagggggt gttcgggagg tcgggagaga ggagttacgg tggttgtgcg tttttttcgt 4080
ttttagtgta taggttaagt tttttttttt tcgttttttt atttttttaa ttttatttag 4140
aaatcgtgtt ttcgagatag gaagggcggc gggtcgagaa gtacggagag tttcggtttt 4200
attttttttc gaattttttt gtggaatttt attttatacg gtgaggaaat tagagcgcga 4260
gatcgtcggc gggtttgtag tttcgtagtt ttggggcgtt ttttcgttgg cggttggagg 4320
tgggcggttt tttgcgtagg tcgggcgagt ttgggggcgg ggagaggtcg ggtttcgatc 4380
gtcgttggtt cggagtgaaa atagttgtgg atttacgtag tattaggtat tatgttaagt 4440
tttttatatt ttatttttaa tttttttaag tgattttata gaggagattg acgtcgaggg 4500
gttgagtaat ttgtttaagg ttatataatc gggattgaaa gttataatcg gtgtaaattt 4560
aaatttttaa ttttttgtgt acgttttttt gtttgacggg gaggtggtta tcggtttagg 4620
ttatagttga aaggagatag gaaggaaagg gggtagatgg aatagggcgt attttttatt 4680
tgtagaaggg ggtaggacgg cgtggttgga gttattgggt tgcgtttttg tttggttttt 4740
gcgcgtttag attcgggtgg gaggaaagta agagtcggga ggcgggcggg gcggggcgtt 4800
aggggaggga gggagggagg ggcggtcgga ggagggggtc ggagggcggn ggtttgattt 4860
tttttgcggg agagcggttt cgtttcgtcg tttcgttttt agttgtttat cgcgttcggt 4920
tattggagag gttcgttgtt cgtttcgttt cgtcgtcgcg tcgcgagttt ttagcgtcgc 4980
gtagggtttc ggcggtgaga gttgcgtagc gttggttgtt ggttggtttc gcggagacgt 5040
cgaacggacg cggtcggcgt cggtttgtgg gttcgtcgtt tgtagttatg attttcgtag 5100
tttgtttttc ggtttcggtt cgggacgttt aaaattttat atagtcgcgc gtagttgttg 5160
gagagtcggt cgttgttttt tcgtcgtcgt attatatttt cgtttcggga gttgggagta 5220
gcgcgggtag tcggcgtttt cgtgtaaatt gggggtgttt gttagagtag ttttagtcgt 5280
tgtcgttgtt attttcgatg ttggttatgg tttggcgggg cgtagggtcg agcgtttcgg 5340

gggcgttcgg gggcgtcggt tttagtttgg ggttgttttt gtagttgttg ttgtttttgg 5400
ggtcggcgcg gggtttcggg gacgaggaag agcggcgttg cgatttattt cgtattttta 5460
tgtgttagaa tttcggttat aacgtgatta agatgtttaa tttggttggg tacgagttgt 5520
agacggacgt cgagttgtag ttgataattt ttatatcgtt tatttagtac ggttgtttta 5580
gttagttgta ggtgggcgtt tttattttta tttttggcgg gattttttgg ggagggacgt 5640
tttaaattaa ttttgtagag tttatgttaa gttgattatt ttagttcgaa ggagatagtt 5700
tttttggaaa agtgattttt atttttattt ttattttttt agggattgga agttaaaacg 5760
ttgtgtaagt tatttggttt gggaaagaat ttatttttat atttcgtttt ttcgtttttt 5820
tttttttgttt atttatgttt ggtttagatt tttaatttta gtggagttga gggttatttt 5880
tgttaaggat tttggtcgtt attgttcggt gggcgagtgg gagggagtta gttgtagtta 5940
agatttggag ggagtgataa ggagattatt ttttatcgaa tttttttttt tttattattt 6000
ttggggtgtt attaaatgaa atattattgt attttttttt gttgaatagt ttttttttac 6060
gacgtttcgt agcgttttag aggttatgta tgaaggagtg gttgggttgg tttgagtttt 6120
tttttatttt tatagttgaa ggtacgttta atgtttggag ggtgagaaga agttgtagga 6180
aggtggttgg tatattggaa gaaatttttt tgtagttatt aaaaaatgta aagtatattt 6240
aatgatagag aaaatgttta ttttatagta ataagaattt gtatatatag ggtgattata 6300
attttgtaaa ataaaaaatt tattgggata ataaaagatt gataggaaaa tttttaaagt 6360
gttaatattg gttatgtttg ggttggtgag gttgggtgat ttttagaaat ttagaaaaga 6420
gagaagttgt gggaaaaagt agacgttagt tgtttaggtt ttagaaattt ggtttttggt 6480
cgggcgtagt ggtttacgtt tgtaatttta gtattttggg aggtcgaggt aggtggttta 6540
cgaggttagg agttcgagat tagtttgatt aatatggcga aattttgttt ttattaaaag 6600
tataaaaatt agtcgggcgt ggtggcgtgc gtttgtaatt ttagttattt aggaggttga 6660
ggtaggagaa tcgtttgaat ttaggaggta gaggttgtag tgagcgggga ttgtattatt 6720
gtattttagt ttgggttata ggagcgaaat ttcgtattaa aaaaaaaaag aaaagaaaag 6780
aaatttgtgg ttttggttgt aatttttgag agataataaa tagatatata gtttatttat 6840
taggtgtatg tttttatata ggttatttta aaatttggtt tgtggttttg gaaagatggt 6900
ggggattaga tttgttgttg ttaaaatatt tttttggaag ttttagaatt ttagaattag 6960
gttataaaag tttaagaaat gattttttag tttttagttt ggaaatagta tatattttga 7020
tattagtggt aatttgttgg agaaatagtt tttgtaggga tatatatttt taattatatg 7080
tattttgggg aaagcggtta agttgttttg aaggataaga ttggattttt ttatatgttg 7140
gaaaatagtt atttgtaatt ttagatttta tagataatat ttgtaaggag ttaagtagat 7200
tgtttattaa tgttggagga attgaggata gtaattggat ttttttttgt tggtattgta 7260
gttttagcga tttaatattt atattttttt attgtgtttt attattattt attgaggaaa 7320
tttggttaaa taaattttta gtgattagag atttaatttt gtattttatt tttgagttta 7380
atagtgtttt atttgggtta tttttttgttt tagtatggga ttagtttaaa taaattttta 7440
agggataaat tagggagaag agaatattaa taggatttat attttttttt aatttttatt 7500
tttaaaatga taaagaaacg ggataaaaaa taaattattt aaatgagtta tattagtttt 7560
tttagttttt ttaaatagtt tttaaaatgg aaggagattg aaagttagtt ttttaagatt 7620
tattttaagt gttgtgtaaa gagggtttat gtaatttttt agattttatt tattttattt 7680
atttatttat gtatattttt ttggaaagga ttattaaagt taaattaaga aaagtatgta 7740
agtgatggat tattaggcgt tttgtttgga ggttagagta aatagataga ttttcgatag 7800
gttggtttaa aagtagatat ttagattgtt tggaagtatt taatttagtt ttgtgggagt 7860
atttggttaa atttttaagt tattgttttt tttgtgtgtg tttattttgt tattttttta 7920
gt 7922

<210> 104
<211> 7922
<212> DNA
<213> Artificial Sequence

<220>
<223> chemically treated genomic DNA (Homo sapiens)

<220>
<221> unsure
<222> (3073)

<400> 104

attggaaaag taataaaatg aatatatata aaaaaaataa tgatttggaa gtttgattaa 60
atgtttttat aaagttgagt tgaatgtttt taggtaattt aggtatttat ttttaaatta 120
atttatcggg agtttgtttg tttattttga ttttaaataa gggcgtttga taatttatta 180
tttatatatt ttttttagtt tggttttagt aatttttttt agaagaatgt atataaataa 240
ataaataaaa taaatagggt ttgaagaatt gtataagttt tttttgtata atatttgaaa 300
taaattttaa aaaattggtt tttaattttt ttttatttta gaaattattt gaaaaagtta 360
aagaaattaa tatgatttat ttaggtggtt tatttttatt ttcgtttttt tattatttta 420

aaaataaaag ttaaaaaaaa atgtagattt tgttagtatt tttttttttt taatttattt 480
tttaaaaatt tgtttaaatt agttttatat tgaaataaaa aatggtttaa atgagatatt 540
gttagattta aggatgggat gtagattgag gtttttggtt attagggatt tgtttggtta 600
ggttttttta atgaataata atgaagtata ataggaaaat gtaaatgtta agtcgttgaa 660
attgtagtat tagtagggga gagtttagtt gttgttttta atttttttag tattgataaa 720
tagtttgttt ggttttttat agatgttgtt tatgaagttt agagttataa gtaattattt 780
tttagtatgt aaagggattt agttttgttt tttaaaatag tttggtcgtt ttttttagga 840
tatatgtaat taaaaatata tatttttgta aaagttgttt ttttaataaa ttgttattga 900
tgttaggata tgtgttgttt ttaaattgag agttggagga ttatttttta agtttttata 960
atttagtttt aaagttttga aatttttaaa gggatatttt agtagtaata ggtttaattt 1020
ttattatttt tttaaaatta taaattaagt tttaaaatga tttatgtaaa aatatgtatt 1080
tagtaagtaa gttgtatgtt tgtttattat tttttagaag ttatagttag aattatagat 1140
tttttttttt tttttttttt ttttgatacg gagtttcgtt tttgtggttt aggttagagt 1200
gtaatggtgt aattttcgtt tattgtaatt tttgtttttt gggtttaagc gatttttttg 1260
ttttagtttt ttgagtagtt gggattatag gcgtacgtta ttacgttcgg ttaatttttg 1320
tatttttagt agagatagga tttcgttatg ttggttaggt tggtttcgaa tttttaattt 1380
cgtgagttat ttgtttcggt tttttaaagt gttgggatta taggcgtgag ttattgcgtt 1440
cggttagaaa ttagattttt gaagtttgga taattaacgt ttattttttt ttataatttt 1500
tttttttttt aaatttttaa aaattattta gttttattaa tttaaatata attaatgtta 1560
atattttaaa aatttttttg ttagtttttt attattttaa taaatttttt attttgtagg 1620
attataatta ttttgtatat gtaaattttt attattgtgg aataaatatt ttttttatta 1680
ttagatatgt tttatatttt ttaatgattg taaaaaaatt ttttttaata tattaattat 1740
ttttttgtag ttttttttta ttttttaagt attaaacgtg tttttaattg tgaggataag 1800
aaagaattta agttaattta attatttttt tatgtatgat ttttaaaacg ttgcgggacg 1860
tcgtgagaag ggttatttta gtagaaaaag gtgtagtagt gttttattta ataatatttt 1920
aaaaatagtg ggagaaggaa agttcggtga aggatggttt ttttattatt ttttttaagt 1980
tttagttgta gttggttttt ttttattcgt ttatcgagta gtggcggtta gaatttttgg 2040
tagagataat ttttagtttt attggggtta ggggtttggg ttagatatgg gtgggtaaga 2100
gagaaaaacg gaagggcggg gtgtaagagt gggttttttt ttaggttaga tgatttatat 2160
aacgttttgg tttttagttt ttgagaaagt gggggtgggg atggaaatta tttttttagg 2220
agagttgttt ttttcgggtt aggatgatta atttggtatg ggttttgtaa agttagtttg 2280
gagcgttttt ttttaagggg tttcgttagg ggtgggggtg ggggcgttta tttgtagttg 2340
gttggagtag tcgtattgga tgagcggtgt gaaagttgtt agttgtagtt cggcgttcgt 2400
ttgtagttcg tgtttaatta ggttgggtat tttggttacg ttgtagtcga ggttttggta 2460
tatggagatg cggatggggt cgtagcgtcg ttttttttcg ttttcgaagt ttcgcgtcgg 2520
ttttaggagt agtagtaatt gtaggagtaa ttttagattg agatcgacgt tttcgggcgt 2580
tttcgggacg ttcggttttg cgtttcgtta ggttatggtt agtatcgggg gtagtagcgg 2640
tagcggttgg ggttgttttg gtagatattt ttagtttgta cgggggcgtc ggttgttcgc 2700
gttgttttta gttttcggga cgggagtgtg atgcggcgac gagggggtag cggtcggttt 2760
tttagtagtt gcgcgcgatt gtgtgggatt ttagacgttt cgggtcgagg tcgagggata 2820
ggttgcgagg gttatggttg taggcggcga gtttataagt cggcgtcggt cgcgttcgtt 2880
cggcgttttc gcgaggttag ttagtagtta gcgttgcgta gtttttatcg tcggagtttt 2940
gcgcggcgtt ggaggttcgc ggcgcggcgg cggggcggga cggatagcgg gtttttttaa 3000
tggtcgggcg cggtgggtag ttaggggcga ggcggcgggg cggggtcgtt ttttcgtagg 3060
aggagttagg ttntcgtttt tcggtttttt ttttcggtcg tttttttttt tttttttttt 3120
ttagcgtttc gtttcgttcg tttttcggtt tttgtttttt ttttattcgg atttaggcgc 3180
gtaggggtta agtagggacg tagtttagtg gttttagtta cgtcgttttg tttttttttg 3240
taggtgagaa gtgcgttttg ttttatttgt tttttttttt ttttgttttt ttttagttgt 3300
aatttaggtc ggtggttatt ttttcgttag gtagagaaac gtatataaag aattggggat 3360
ttggatttat atcgattgtg gtttttagtt tcggttgtgt gattttgagt aaattgttta 3420
atttttcggc gttagttttt tttgtaaagt tatttgggag gattagagat aaagtgtaga 3480
aggtttagta tggtgtttgg tattgcgtga gtttatagtt atttttattt cggattagcg 3540
gcgatcgggg ttcggttttt tttcgttttt agattcgttc gatttgcgta gggagtcgtt 3600
tatttttagt cgttagcggg gaggcgtttt agagttgcgg agttgtagat tcgtcgacgg 3660
tttcgcgttt tgattttttt atcgtatgaa atgaaatttt atagggggat tcgaaaggaa 3720
atgagatcgg gatttttcgt gtttttcggt tcgtcgtttt ttttgtttcg gggatacggt 3780
ttttaaataa ggttgggagg gtgagagggc gggaggggag aggtttgatt tgtgtattgg 3840
ggacgaggaa ggcgtataat tatcgtgatt tttttttttc ggtttttcgg gtattttttt 3900
tttattaggt tgtatttttt tttttttttg tttttgtttc ggtttttagg gatttgggga 3960
ttatgtgatt taggtttgaa tttaattttt ttgttttatt attagttttt cgcggttttg 4020
tgttggttta tttttttttt gttttttagt cggttagtaa atttttagta ggtggatttt 4080
tttatcgttt atttgttgtt tgattgtgtt gaagttatgg tgtttttttg agttttaaag 4140
tggtcggttg taaagtgggg aagtttatat ttattttata tagttgtttt aagagtttag 4200
tgagataata tttgtaaatt atttagtagt gtttgatata taataaatgt tgaagaattt 4260
ggtgattggg gtgtgtgtgt gtgtgtgtgt gtgtgtgtgt gtgtgtgtat gtgtttttaa 4320
aatagttagg ggagattaat tatgggatag ttttttgttt tttagtttgg tgttaaagat 4380

agttttagta gtttcggaat gaggatttgg gtaaaaggtt ttttattttt tttttgagat 4440
ttttttgttt aatatttgtt attgttttat gtatgttttt atttttttat tttagttaat 4500
tttgtggtat atggtagagg ttagatgtaa gttggaagaa tgtttgtgtt atttatgtga 4560
tatttatatt ttgttttgta tttttagttt ttttatattg gtaacgagat ttgattgagg 4620
gtaaaaatgg aattaatttt tttttggtat ttggttttgt gtttagaatt gtgttggata 4680
cggagaagtt gtattgtttt tagttattat tataaatttt tagtgaagga ggtttcgtta 4740
gttttttata gagattagga aattgaggtt agaattatag taatagaaaa tgataagatt 4800
aattaaatta gggtttagaa gaggtttttt aaagatggaa ggtagggtta tttttttaga 4860
aaaaattttt agttggaaat taatgtattt agtaatagga ttgaattttt gtgtgtttgt 4920
gttgagtaat gtataaaatt tttggtttat agagatgaat aaacgattag atgtagagat 4980
taaatgattt attaattatt tgggttatag agatgaatat ataattagat gaatagatta 5040
aatgaataga taatttttat tttttaggat tttataggag gagataaagg attaataaat 5100
aataataatt tagtttgtta agtgtataga tagagaattg tgggtgaatt gaaggtagaa 5160
gtggttgagt ttggagggtt atttaaaggg ggatgatttg taagattcgt agttatttaa 5220
taaagtgtag ttagtatgta attggttttt tttttaggga agagtggtgg tggtggggtg 5280
tgttttgtgt tgaagatatg gttatatggt ttattaaata gtgaattttt tgaggttaag 5340
gaggaggatt tgttttttat atagggtttg gtatataagg aatattattt gttttgggtg 5400
aaggaatatt gtttttaaat taataataaa ttatttatat agaattttta tttttagata 5460
ttgtgttata tgttttatat ttttaatttt atttattttt tataatttgt agaaattgat 5520
gttttgagag gttaagtagt ttgtttaaga ttatatagta agtcgtagag aagaatttat 5580
gtatgtttgt ttggttttaa attttgtgtg tatggtattg tttttgtaga ggaaattgtt 5640
ttacgtttag tttaatttat agttaaagat gattattttg tgatttggat tttttatttt 5700
tagatttggt attttgtttt agtttgtttg aagtaattgt tttttttgag aatatatgaa 5760
gaagtttttt ttttattaga agtttttttt gttaatagtt ttttttttta gtcgtggaat 5820
ttattattag tattttttaa aaaagaaagt taggggaatt atattttagt ttgaatgtgg 5880
aaagataata aagattataa tttagagtta aatttgaatg gtttggagtt ttttagtttt 5940
ttttttttgg agtggtttat atagtttata atttgttttt ggtgataggt atggtatgtt 6000
tgtttttgtt atagaaaata tttttcgagg gagttttagg tagagggata tttattttgt 6060
gatatgtata gtatttttga gaggaaaaga tagggatatt tgaataaagt gtaggttatg 6120
ggtttttgtt aattttggtt tgagtgtttg ggtagttttt ttggtttagt ttttgagtag 6180
taaagattta attattttgg agttgtaata aatatgattt taatagtttg ttgggtagtt 6240
ggagggtttt ttatttgtat ttaataagag gttgggtttt tattaatttc ggtgatgtgt 6300
taatttttta atttgacgtt ttttgtttta atagattttt gtaatgttta ttaaattaaa 6360
ggttgtattg gaagagtaat gtttaggtta aaatgtatta tttaaggggg tggaagttat 6420
aaatattttt ttttttttac ggaaaaattt tttatttgcg gttttttgtt aatttaatta 6480
agtgttgaag tagttatttt agagtttgtt ttttattttt tatgaaataa tttaattaat 6540
ttatatttag tagtttttaa gaaaagtttt attttaatta tttatgaaga aattagtttt 6600
tttgaattta gatagaatat ttagaattta taaagagttg ttttgatgga aagtgtaaga 6660
aagtggttta ttattatttg tttggttttt gagggaaata atttattggg attaaaattt 6720
tataagtatt gggaatttat tgtggaataa taaaagtaaa gtaagaagat gttatgttat 6780
atttatttta tttataaata gggtgttata tttttttttg tatttaaatt agttataaaa 6840
taaaagtgcg tttgttgagt aaggtggaag ggaatattga ttttatttcg agttgaggaa 6900
ttaaagtttt taagagatta atatttatta agtggtagag ttaaattttt tttttagttt 6960
attgtattat tagttagtag agaagaattg ttattataga gtttttgtat gttaagaaat 7020
agaagtttaa aatttttttt atttataagt ttagtaagta ttaaatggta tattagatat 7080
ttttatttat tggtggtgat gagttaacgt aaagatgaat aattaaaaat ttttatggaa 7140
ggtaaaaaat aatgtaaata tatttttatt tttatagttt cgtttcgttt tagtgaaatt 7200
gattagttaa gttgttaata aatatttagt gatgggttat tgtggatatg ttattgagtt 7260
gggtattagg gagtatgtag atatgttaaa ggtggttttt gtttttaagg agtttatagt 7320
tatattgggg aagattaaga tatgagatta agggtaaaat aagtttttta tggttaagtg 7380
tattttgttg agttttttta tttttagttt ttttttgttg ttttatataa ggttttgtat 7440
tataaagtta ttgggttgtt ttgtttttgt tttttttatt tttttttgaa gttagaggtt 7500
gtgttttttt tattattatg tttttaaata atatagaata atattatttg agttatagaa 7560
ggtatttagt agttatttgt ttaatatata aatgaatggt tttgtttata agtgatttga 7620
ttatttagaa aaagaaagtt tgtattatga gtcgggtata ggtaatcgtt aaaatattat 7680
ttaagtggtt ttagaatgat ggttaagtta agattataaa tcgttttttt aagttaggta 7740
tggtttatat ttttagtttt ttttttaagt tagatgtttt gttaattatt tatttttttt 7800
tttgtttgat tgtgtttatt tttaaatttt ttatagtaga taaatatttt tttttttttt 7860
ttgagatagg gttttatttt gtcgtttagg ttggagtgta gtggtatgat tttggtttat 7920
aa 7922

<210> 105
<211> 6067
<212> DNA
<213> Artificial Sequence

<220>
<223> chemically treated genomic DNA (Homo sapiens)

<400> 105

aattagtttt tagagtaggg agtgataggg aatgtatttt aagtgaaagt tagtttttaa 60
atagaaatta ttttttacgt tatgcgtata aattttttta aattaagtta gtgtatgatt 120
aaatttataa tgttatatgg agggagtgaa gttatggtag agtttttaag ggttagggaa 180
agaagtatta gggagagaat tttatgaggg aaggggttta tattattgga agttttaatt 240
gttttttttt aagttttttt ttgtttttgt atttttttat gtttttgttt agtttagttt 300
tttttagttg gggttttttt attgttgttt gttttttaaa ttgttaatgt tttagtatta 360
ggttagtggt tataagtcgg tttatttttt agtatgtgta tagggtttag gtaggagtat 420
aaaggaggat attatgttat atgtttatat agttaaaatt tatatattaa attaataaat 480
tgttatttga aatatgtttt atttttttat tttgaaaaaa tgtattttta taaagataga 540
aatgttttta atgtgtaaag ttatgtttta atgagtaggt aaaatattag aagtagataa 600
atttaattat tatggtacgt gtttgtatag ttcggtgata ggttggagat gtttgggtaa 660
gtaattaaat aaaaatttat atagtttata agttatatat ttatttttgt gaaatttatt 720
tttttttttt tattttagta aaatttttta attatattgt tataataaaa atttttatat 780
agttagtttt ttatgaatat taatgttaaa ttatataatt tttttgaatt attgtagttt 840
ttgggatttt ttaattcgat tttaatttgg agaaattttg ttttgttaag gtagtagtaa 900
ttggaattat taataaaatt tttaaagtta tatttatttt taaaaatgag aaattaaggt 960
ttgtagtgtg gtatgtaagg agtttagttg ttattttgtt ttgataaaaa gtagaaagtt 1020
gaataaattg aaaattaata atttttttta gatttagtag agaagtaagg atatagagta 1080
agttattgtt tttaaagttg gagagataaa taggtagata tatagaatta taatttatta 1140
gagtagaaat tcgtcggtag aaatttttag gggtatgagt atagggtagg aagattagaa 1200
ttgtaattga tgaattgtta gaggtttagg gtggttaaga ttaagaattt aaaattttag 1260
ggggttagtt gggtacggtg gtttacgttt gtaattttag tattttggga ggtttaggta 1320
gggagattac gaggttagga gatagagatt attttggtta atacggtgaa atttcgtttt 1380
tattaaaaat ataaaaatta gttgggtatg gtggtgtgta tttataattt tagttattcg 1440
ggagggtgag gtgggagaat tatttgaatt taggaggcgg aggttgtagt gagttaagat 1500
tacgttattg tattttagtt tggtaataga gtgagatata tatatatata aatatatata 1560
tatatatata tatatataaa gggggagggg gtttagtttt tggagggtat atttatattt 1620
ttgtaagttt tatttttagg agttttttta ggttttttta gtgaaattta aagaaaaatt 1680
attttttata tttaataggg agaggtgaaa agaattattt tgaaatatgt taaaatattt 1740
tgtttttttt aataaggttt gtttttaaga gaaattatta gagtttaatt tattatagtt 1800
ttagtagagt ttaattaagt tgatagatgg gaaatattta atttagtttt tttttagata 1860
ttttgtttta tttaagaagg gggataaaaa ttaagaagta tttgtgaagt ttttagttta 1920
gggatataag tttattaaga ttgaaattta attataggtt tatagaatgt tttttttttt 1980
tttatatttt aatatcgtat tattaaagtt tatttatttt agtgtttttt atttagtgta 2040
ttatgtttag tttttaatta aaaattataa ggtatattat agagtaaaaa atatagtttg 2100
aagaggtaaa gtaataatta gaattagatt tatatatggt agagatgttg gaattattag 2160
attaggaatt ttaaataatt gtgattaata tattaagggt tgtaaaggta aacgtagata 2220
atttgaaaga atagacggtt aatgtaagta gaaatgaaaa ttttaaggaa gaataagaat 2280
tgttagagat ataaaatttt ataatagaaa tgaagaatgt ttttgatgga tttattaata 2340
gattggatat ggttgagaaa agaatttttg agtttgagga tatgttaatg gaaattttta 2400
aaattgaaaa ataaagagaa aaaggattta aaaaaaagaa ataatattta agaattgtga 2460
aataattata aaagtataat atatgtgtaa taggaatatt aggagaagaa ttagagaaag 2520
gaatagaaga aatatttgaa gtaatagtga ttgaaaattt ttttaaatta atgttagtta 2580
ttaataatgt tagattaatt ttttaaaatt ttttttagaa gatagaagta gggggaatat 2640
tttttagtag tattgtttta atattaaaat tagataaaaa tattattagt aaagaaaatt 2700
ataaattaat atattttttg aataaagatg taaaaatttt taataaaata ttagttagta 2760
gaatttagta atgtataaaa agaattgtat agtataatta agatttattt taggtatgta 2820
aggttggttt aatatttgaa aatttattta tgtaatttat tatattaata gattaaagaa 2880
gaaaaattat atgattatat taatagatgt agaaaatgta tttgataaaa tttagtatat 2940
ttttattata aaaattttta gtaaattagg gatagagggg gaattttttt atttgttaaa 3000
gaatatttat aaaaatttta aggtaataat atatttaatg atgaggagtt tatttataga 3060
ggaataaaaa taaagaatga tatttaattt tttagaaatt acgtagataa gaagagaggt 3120
ggagtaaaag tgtttagaga aaaaaattat taatttataa ttttgtattt tgtaaaatta 3180
gtttttaaaa gtaaaagttt gtttttttta tttttggtta ttattgtttt ttagtattag 3240
tttagaggaa taatgtaaag agaaagagaa ggaaaatgag gaaaatcgtt agatagtttt 3300
atatttatag atgaagaaat taaagaggat ttgaataatt tgtttaagtt tatatagtaa 3360
gtaaatgttg gagttaggtt ttgaatttag ggattttgaa ttttaaagtt tgtgttttta 3420
atttcgtatt attgggatga aattgtatag tgaaagataa gcgatgatat agatgattgt 3480
ttaagtattt taaatttatt ttgataaaaa aaaaattatt ttagtttatt attgtttatt 3540
atataattaa atataatgaa aaataaaata ggttttattt atttttgtat aaattaattt 3600
attataattt ttttttttaa ataatggaat aagtatttat ttgaatttta aaattatcgt 3660

ttttaaatga tattttaaaa gtatcgtttt taaacgatat tttaaaaagt atcgttttta 3720
aatgatattt ttagtgatat tttattgttt ggtagttatt agagtttgat tataaaaaga 3780
ttttattaag ttcgtagaaa ttggagagaa atagtttttt aatgtagaaa attagaaatt 3840
taggggaaaa tatttttttt atatagaaat ttattttgta atatattaat tatttagttt 3900
ttaatgatgt taatattttt ttattattat ttgtttttta atattagtaa gtttttgata 3960
aaattgggga ggaatttttt attttaaatt cgtttttttt tatagatttt aaaattttta 4020
ttttggttgt ttagggttgt ttttttaggt atggaaatat gatatttttt ttttgtaata 4080
ggatgattta tttgataaga atagttttta tttataagtt attttgtatt ttttaaagta 4140
tttttataag tttatttgtt ttttatatta ttttgtttgt tttataggag ggttttatag 4200
gttaggtgta aatatttgta acgaatttgg tattatttga tttatagtta gtgtttaatt 4260
aacgttaatt attattataa ttattgttat ttttatattg tgaggaaatt atgtttttta 4320
aagattaggg taatttattt aatatttatg atagagttgg tattttaaat taggttgatt 4380
tttatagtag tttaattttt tattagggtt tgtattaata aaattgtggg attcgttagg 4440
gttggaattt tattgttatt tgagtattta gttggtgatt aggtttttcg aattaataaa 4500
agtatttgtt gttgttttta tagtagttgt ttaggaagta ttttttttcg aatttaagtt 4560
atttgggtaa ttggttttat aaagtaaatt ggtttcgttt aaggattttt gatttttgaa 4620
attcgtaagg aagtgggttt ttttagagtt agcgttaaga gttttaaaaa atttttttaa 4680
ggtgaatttt ttcggattcg gatagcgaga tggggtttag ttttgcgttt cgggggtcgt 4740
aatagacggt aaggtggagg tggtttttcg gtttcgtagt cggatttcgg tatttcgagt 4800
tcgtttatcg gggtcgggtt ttggcgggtc gcggagtgag gtcgggcggc gtagcgtagg 4860
cggtgttcgg gcgtagggtt agtttcgtcg tcgtcgtcgt cgtcgtcgtc gtcgtcgtcg 4920
cgagttcgga tttgttcgtt cgatttggcg gcgtagtttc gcgggatcgt ttagttttcg 4980
tttttcgtgc ggttttcgag gtagttttag tttcggacgt aatttcggag tcgttttagg 5040
tttttggggg gaacggtggg ttagacgggg acgggaaggg atagcggttt tcgatcgttt 5100
ttcgaggtga ggtttttacg gttgggattg ggtaggggtc gggcgggtgt cgggggaagc 5160
ggggagggtt tcgaggggcg aggggtcgcg gttgggatga ggcgcgtcgg gagtcgtagg 5220
tgttggggag gtaacggcgg gacggttcgt atttgagttt tttcggaagc gggaatcggt 5280
tttgaaaggc gatgtcgggg tttcgcggcg tcgcgtaggt taggaacgtt gttgtttggt 5340
tttaggtttg gggtaggttt tcgggtgtgg ggcgttggcg gatcgggtat tagtgtgtgt 5400
tggggcgcgg gtaattcgta attcggggtt tatagttttt ttttcgtaag gggattgttt 5460
tcgttttttt ttcgggatag atttgcgtat ttttttttcg acggtagtat tatatttttt 5520
tagttagtga aaattgtaat gttgtttttt tattggagga attttaaaag ttttttttta 5580
gttggtttta cgaaaatcga gaaaattata tatttagaag attgttataa aaattgattt 5640
tttagagttt tttataagtt taagaatttt ttgggtttta aggaattttt tagggtttta 5700
gtttttttgt atttaataag ttttttaata ggggttttta gattgtatgt ttatattaat 5760
tttttataat ttatttcgaa ttattagttt tttttttttt ttttatttta tttttgttat 5820
tttttttttt taattttgtt ttttatatag tattaatttt ttagtatcgg ttttttagag 5880
gtgtagtttt tttagtaaat gatatatttt atgttaaggt aggtggaaga gtatttcgtt 5940
aggatagaat ttttcgttgt ttttttagtg tttgttattt ggggtgaaaa ttttaatttt 6000
gtattttgat tttatatttg agtttatgat aaatgttgtt gatatatttt ttttttgttt 6060
ttatagt 6067

<210> 106
<211> 6067
<212> DNA
<213> Artificial Sequence

<220>
<223> chemically treated genomic DNA (Homo sapiens)

<400> 106

attgtaaaaa taaaaggagg gtgtattagt aatatttgtt ataaatttaa atgtggagtt 60
aaagtgtaga gttaagattt ttattttagg taataaatat tggaaaaata acggaaaatt 120
ttgttttaac gagatatttt tttatttgtt ttagtatgga atatattatt tgttaaggaa 180
gttgtatttt taggaaatcg atgttaggag gttggtgttg tatggaagat agggttaaaa 240
agagggaatg gtaagagtaa gatggaggaa aaaaggaaag ttggtgattc ggagtaaatt 300
ataaaggatt aatataagta tataatttaa gggtttttgt tggaaagttt attaagtata 360
agaaaattaa ggttttggga aattttttaa gatttaaaga gtttttaaat ttgtaaaaga 420
ttttgaagag ttagtttttg tggtagtttt ttgggtatgt agttttttcg attttcgtaa 480
ggttaattag agggagattt ttaggatttt tttagtggag gagtaatatt gtagttttta 540
ttggttagga gggtgtagtg ttgtcgtcga aaggaaagtg cgtaaatttg tttcggggga 600
agggcgaagg tagttttttt gcgggaggaa ggttgtaggt ttcggattac gaattattcg 660
cgttttaata tatattgatg ttcggttcgt tagcgtttta tattcgggaa tttgttttag 720
gtttagagtt agatagtagc gtttttggtt tgcgcggcgt cgcggggttt cggtatcgtt 780
ttttagagtc ggttttcgtt ttcgaggaaa tttaggtgcg ggtcgtttcg tcgttgtttt 840

tttagtattt gcggttttcg gcgcgtttta ttttagtcgc ggtttttcgt ttttcgaggt 900
ttttttcgtt tttttcggta ttcgttcggt ttttgtttag ttttagtcgt ggaagtttta 960
tttcgggggg cggtcgaagg tcgttgtttt ttttcgtttt cgtttaattt atcgtttttt 1020
ttagggattt gagacggttt cggggttgcg ttcgggattg gagttgtttc gagagtcgta 1080
cggggagcga gagttgagcg atttcgcgag attgcgtcgt taggtcgggc gggtaggttc 1140
gggttcgcgg cggcggcggc ggcggcggcg gcggcggcgg cgggattggt tttgcgttcg 1200
ggtatcgttt gcgttgcgtc gttcggtttt atttcgcggt tcgttaggat tcggtttcgg 1260
tgaacgggtt cggggtgtcg aggttcggtt gcggggtcgg gaagttattt ttattttgtc 1320
gtttgttacg attttcgagg cgtaaggttg agttttattt cgttattcgg gttcggaggg 1380
gtttatttta gaaggatttt ttgaagtttt tggcgttggt tttaaaagaa tttatttttt 1440
tgcggatttt aggagttaag aatttttaaa cggagttaat ttgttttgta aagttaattg 1500
tttaagtgat ttgagttcga aaggagatat tttttggata attgttataa aaataataat 1560
aaatattttt attaattcgg ggagtttggt tattaattgg atgtttaggt agtagtggga 1620
ttttagtttt ggcgggtttt atagttttat tgatataaat tttaatagga agttgaattg 1680
ttgtagaaat taatttagtt tggggtatta attttgttat agatattaga taaattattt 1740
taatttttaa ggggtatagt ttttttataa tataggaatg ataatagttg taatgatggt 1800
taacgttaat tgagtattgg ttgtaagtta aatagtgtta agttcgttat aagtgtttat 1860
atttaatttg taaggttttt ttatgaaata ggtagaatag tatgagaagt aaatgaattt 1920
gtagaagtat tttgaaaaat gtaaagtaat ttgtagatgg aggttatttt tgttagataa 1980
attattttgt tgtagaaaag gaatgttata tttttatatt tagaaggata attttgaata 2040
attagagtaa gagttttgga atttgtgggg aaagacgaat ttggagtggg gaattttttt 2100
ttagttttat taaaagttta ttgatgttgg gagataggta gtgatgaaga aatattaata 2160
ttattaagag ttggatagtt gatgtattgt agaataaatt tttatgtagg aagaatattt 2220
ttttttaaat ttttaatttt ttatattgga agattatttt tttttagttt ttacgaattt 2280
aatgagattt ttttgtgatt aggttttagt gattattagg tagtgaagta ttattaaaag 2340
tattatttaa aaacgatatt ttttaaaata tcgtttgaaa acgatatttt taaagtatta 2400
tttgaaaacg atagttttga aatttaagtg aatatttgtt ttattatttg gaaggaaaaa 2460
ttgtagtaag ttgatttata tagaagtaaa taaagtttgt tttatttttt attgtattta 2520
attatatagt gagtagtgat gaattagaat gatttttttt ttgttaggat ggatttggaa 2580
tatttaggta attatttata ttatcgtttg ttttttattg tataatttta ttttaatggt 2640
acgagattaa gaatataaat tttggagttt aaaatttttg ggtttaaaat ttagttttag 2700
tatttatttg ttgtgtgaat ttgagtaaat tatttagatt ttttttagtt tttttatttg 2760
taaatatgga attatttgac ggtttttttt attttttttt tttttttttt tgtattgttt 2820
ttttgggttg atgttagggg atagtggtga ttaaaagtag aagaaataga tttttatttt 2880
taaaggttaa ttttgtaagg tatagaatta taggttggta gttttttttt ttgaatattt 2940
ttattttatt tttttttttg tttacgtggt ttttgagaag ttggatgtta ttttttattt 3000
ttgttttttt ataggtaagt tttttattat taagtatgtt gttattttag ggtttttgta 3060
gatgtttttt agtaagtgag aaagtttttt ttttattttt agtttgttga aagtttttat 3120
gatgaaaatg tgttggattt tgttaaatgt attttttgta tttattgatg tggttatgtg 3180
attttttttt tttagtttat tggtatggta gattatatga atgaattttt aaatattgga 3240
ttagttttgt atatttggga taaattttgg ttatgttgta taattttttt tatgtattgt 3300
tggattttat tagttaatat tttgttgagg atttttatat ttttgtttaa gagatatatt 3360
ggtttatagt tttttttgtt agtaatgttt ttgtttggtt ttggtattag ggtaatgttg 3420
ttaggaagta ttttttttgt ttttattttt tggaagagat tttagagagt tggtttaata 3480
ttgttggtga ttgatattaa tttggggaaa tttttagtta ttattgtttt aaatattttt 3540
tttgtttttt tttttaattt tttttttggt atttttgtta tatatatgtt atatttttgt 3600
agttgtttta tagtttttgg atattgtttt ttttttttaa gttttttttt tttttatttt 3660
ttaattttgg aagtttttat tgatatattt ttaaatttag agattttttt tttaattatg 3720
tttagtttat taatgaattt attaaaggta ttttttattt ttgttataga attttatatt 3780
tttagtaatt tttatttttt tttaaaattt ttatttttgt ttatattaat cgtttgtttt 3840
tttaagttgt ttacgtttat ttttataatt tttagtatat taattatagt tatttgaagt 3900
ttttggtttg ataattttaa tatttttgtt atatgtgagt ttagttttga ttattgtttt 3960
gtttttttaa attgtgtttt ttgttttgta gtgtgttttg taatttttag ttgaaagttg 4020
gatatgatgt attgggtgaa aggtattgga gtaaatgggt tttagtgatg cggtgttaag 4080
atgtgggagg aggggaaata ttttatagat ttatgattag attttagttt tagtgagttt 4140
gtgtttttag gttggaaatt ttataagtgt tttttagttt ttattttttt ttttgggtgg 4200
gataggatgt ttagaggggg ttggggttgg atatttttta tttattagtt tggttaggtt 4260
ttgttaaaat tgtaataggt taggttttgg tagttttttt tgagggtagg ttttgttaaa 4320
aagaatagaa tattttgata tattttaaaa tggttttttt tatttttttt tgttggatgt 4380
aggaggtgat ttttttttga tttttattga gagaatttgg aagaattttt ggaggtaaaa 4440
tttataaaag tgtgagtgtg tttttaaaag attaggtttt tttttttttt gtgtgtgtgt 4500
gtgtgtgtgt gtgtgtttgt gtgtgtgtgt gttttatttt gttgttaggt tggagtgtag 4560
tggcgtgatt ttggtttatt gtaattttcg ttttttgggt ttaaatggtt tttttatttt 4620
attttttcga gtagttggga ttataggtgt atattattat gtttagttaa tttttgtatt 4680
tttagtagag acggggtttt atcgtattgg ttaggatggt ttttgttttt tgatttcgtg 4740
atttttttgt ttaggttttt taaagtgttg ggattatagg cgtgagttat cgtgtttagt 4800

tgattttttg gagttttaaa tttttagttt tgattatttt gagtttttag taatttatta 4860
gttatagttt tggttttttt attttgtatt tatgtttttg gaggtttttg tcggcgggtt 4920
tttgttttag taaattgtga ttttgtgtat ttgtttgttt gtttttttaa ttttgggggt 4980
agtggtttgt tttgtgtttt tatttttttg ttagatttag gaagagttgt tgatttttag 5040
tttgtttaat tttttatttt ttgttaggat agagtaatag ttaagttttt tatatgttat 5100
attataaatt ttaatttttt atttttaaaa ataggtatga ttttaagaat tttattgata 5160
attttagttg ttattgtttt agtagagtaa agttttttta agttaaaatc gagttgaaaa 5220
attttaagag ttgtaataat ttagaaaagt tatgtaattt ggtattagta tttatagaag 5280
attgattata tgaaaatttt tattatgata atataattaa gggattttgt taaaatgaag 5340
ggaagaaagt aaattttata ggggtaaata tgtaatttgt aaattgtgta agtttttatt 5400
taattattta tttaaatatt tttagtttat tatcggatta tgtaaatacg tgttataata 5460
attgaattta tttatttttg atattttgtt tatttattaa aatatagttt tatatattaa 5520
aaatattttt gtttttataa aggtatattt ttttaaggta ggaggataga atatatttta 5580
agtagtagtt tgttagtttg atatatgaat tttagttatg tagatatata gtatggtgtt 5640
tttttttgta tttttgtttg ggttttgtat atatattggg ggataggtcg gtttgtggtt 5700
attgatttga tgttgagata ttagtagttt gaggaataaa tagtagtaga gggattttag 5760
ttggaggagg ttgggttgag tagaggtata gaagagtata gaagtaaaga ggaatttggg 5820
aggaagtagt tagaattttt agtggtatga attttttttt ttatgaagtt ttttttttaa 5880
tatttttttt tttaattttt gaaagtttta ttatggtttt atttttttta tgtaatattg 5940
tgaatttgat tatgtattga tttaatttag ggaagtttat gcgtatgacg tagaggatag 6000
tttttattta aaaattaatt tttatttgaa atatattttt tgttattttt tattttgaaa 6060
gttaatt 6067

<210> 107
<211> 8186
<212> DNA
<213> Artificial Sequence

<220>
<223> chemically treated genomic DNA (Homo sapiens)

<400> 107
   gttattttgg aggaggtagg aaaggaaggg gagggtgtat tttttagggg ttgatatgtg 60
   ttcggggagg ggagggttgg ggatgtaggg atataggtta ggagggcgtt tttttttata 120
   tatgtaggat aattattggt tttagttagg ggttttagga gggacggttt ttttttaggg 180
   ggaggaggag ttaggggggt ttttcggttt agggaggggt gtcggagatg ggtattgata 240
   gtgatttttt ttttttcggt tttttcggtt tttcggtttt tcgtatagtt ttttttttag 300
   tgagttttag gagtattgtt ttatttttgt aaagagggag atcgggtgat aaagtcgggt 360
   gggtgggtag gaggttagag gttttttttt ttttaggtcg tatttgcgtg ggggcggagt 420
   ttatttattt tttttttttg gttttttagg cgcggtcgtt gttatggtga taggtttggt 480
   attttacggt ttttggggcg ttttgtttat cgaagttgtt agaggaaggg tttttcgacg 540
   tttttttatt ttggtaagat gggggcgggg gtttataaaa tttggttttt aggttagttt 600
   tagtattggg aggtttttag tagtttgaat tttttaagtt tgtgggaggg gagttttatt 660
   ttttggtatt ttgatagttg ggggtttagg agttggggtt atatattatt attatttgtg 720
   tatttaggtt tttttggggt tagatataga gaaataatat ggttgttttt ttgtgggttt 780
   ttttgtggag agagggggtt ttgttgtgtt gtttaggttg gttttgaatt ttgggtttaa 840
   gtaatttttt tattttggtt ttttaaagta ttaggattgt aggtgtgagt tattgtattc 900
   ggttaatatg ttattttttg atgttatttt atggtttttg aatagtggtt ggtatggggt 960
   cgagggaggg tacggatgta gaggttatag tgtgttggat agatgggttg gtttggattt 1020
   tggatttata gagtagagtt tttagtttta gtttagtttt gttgggttgg ttttgggagg 1080
   tgatgtttta atttggggtg ttagatggaa tagaagagga ggaagatttt tagagttggg 1140
   gaaataagtt tggtaaaggt ttggaagtat ggttttgtag ggtgtttttg gggaggagta 1200
   agagtggggt gtcgggtgtg ataggaaatg aagaggtgag gcggggtttt tgtttggagg 1260
   aatttttatt gttatttttg agagtatgta ttttattttg ggtagttgtt gttgaagttt 1320
   tgggattttt tattattaat gtttaggtga ttaaagttgg gttttttttt ttttttgtag 1380
   tgttttgttt tgttttgttt tttttttttt ttcgtttttt tttttttttt tttttttttt 1440
   tttaattttt tttttttttt tttttttttt tttttttttt ttattttttt tttttttttt 1500
   tttttttttt tttttttttt tttttttttt tttttttttt tgtttttttt tttttttttt 1560
   ttgttttttt ttttattttt tttttagaga tagggtttta ttttgttgtt taggttggag 1620
   tgtagtggtg tgattatggt ttattgtagt tttttaggtt taaggatttt tttagtttta 1680
   gttttttgag tagttgagat tataggcgta tattattatt tttgattttt ttgtttattt 1740
   ttgttttaga gatggtgggg ttttgttatt ttgtttaggt tggtttcgaa tttttgggtt 1800
   taagtgattt ttttattttg gttttttaaa gtgttaggat tataggtttg agttacggtg 1860
   ttttatttgg gttttttagg gttataggta ttatattttt gttttatttt tttgggaatt 1920
   ttaggacgtt ttatggttta tatgtggttg agtattattt tgtgttggat atagggttgg 1980
   ttgcgttttt ttggtgtttt atttttcgat ttttttgagg agcgaggggt tgagtgtatg 2040
   tacgagatgg tatgtttttt gtttggtttt ttttttgttt tagtatagtt ttaagggtag 2100
   tagagtatta attataggga tgtgtgggaa atgatggttt tattttttta ttattgttat 2160
   tattattgtt gatttttttt taagttttta ttataggtta ggttttagtg taggtaggtg 2220
   tggttgagtt gggtcggttg ggttggtgtt ttgtttttgg tggtgttaat aggtagtttt 2280
   ttgtaatgga ggagtttgga tgttaatttt gatttttacg agttgcgtaa ttttgggtaa 2340
   atatttgagt ttgttaagtt ttaatttttt atttgtatat gggtacggga ttttttgtag 2400
   tattgtgatg agggtgaata aatgattatt gtaaaatttt tggtttagag tttaggaggt 2460
   ttataatagg ttttcggtta gtttttgtgt ttttttgaag ttttaggtta tttatttatt 2520
   tatttattta ttatttttgg gtttagttag ggtgtttagt tttgtattaa gtgttcgtat 2580
   gaaatagtgt taaatgttta cgtgtaatag tattaagtgt ttacgttaaa tagtattaag 2640
   tgtttatgtg aaatagtgtt aagtgtttac gtgaaatagt attaagtgtt tatatgaaac 2700
   gatgttaagt gtttacgtga aatgatgtta agtgtttacg tgaaatagta ttaagtgttt 2760
   atatgaaacg atgttaagtg tttatatgaa atagtattaa gtgtttatgt gaaatatttt 2820
   gagtttttta ggtgttttta ggttaagaga ataggaatag ttgggtgaaa gggggatggt 2880
   tttttttaaa gagttttaga ttagggtttt ttgttttttg tgtggtttgt cggtggtatt 2940
   tggattttat ttatagagtt atttaggaat tatggtagtt tttaagcggg ttttaggagt 3000
   tttaaggggg tagtttagag agaggtgaga gggtgatggc gatatttttt agaggagtac 3060
   ggtggttttg tgaaatgggt atgggtttcg gaatagataa ttataaatgg aatatttagt 3120
   agttttatgt ttttgggtat gttatttttt tttgagattt agttttttta tttgggaaat 3180
   aaggataacg tagtattttt ggattgttga gaagatataa taagggtaag tatgaagtgt 3240
   gatcgattag atttaggtgt ttaatacgtg ttgttgattt tttttttttt tttttttttt 3300
   tttttttttt taggttaggt atcgtggttt atgtttgtaa ttttaatatt ttgggaggtc 3360
   gaggtggatg gattatttga ggttaggagt tcgagattag tttggttaat atggtgaaat 3420
   ttttgttttt attaaaagta taaaaattat tcgggagtga tggtgggcgt ttgtaatttt 3480
   agttatttgg aaggttgagg taggagaatt gtttgaattc gggaggcgga ggttgtagtg 3540
   agttaagatc gtattattgt atttttgggc gatgagatga gtttgggcga taagagcgaa 3600
   attttatttt aaaaaaaaaa aagttatttt ttttttttta ttagagacga ggttttatta 3660
   tgttgtttag gttgatgggg taagatgggg gtgggggtga gttattgcgt ttggtagaat 3720
   tgttgatttt aatagtagga ttatttgttt gtattataga aaagtaagtt gaggtttaga 3780
   gaggagtttt aagttatata tattgagggg agaggtagac gttgtatttg agttgttatt 3840
   gggtataagg tagattttta tacgtgggaa gttttagaaa tggttttttt tggtcgggta 3900
   cggtggagta cgtttgtaat ttcggtattt tgggaggtag aagcgggagt attttttgag 3960
   tttaggagtt taagattagt ttgggtaata tagtgagatt ttgtttttat aaaaatattt 4020
   taaaattagt cgggagtggt ggtgtttatt tgtaatttta gttattttgg tggttgaggc 4080
   gggaaggtcg tttgagttcg ggagatggag gttgtagtga attatggtcg ttttattgga 4140
   ttttagtttg ggcgatatag agagattttg tttttaaaaa gaaataataa ttaaaaataa 4200
   aaataaaaag ttttttagtt gggcgcggtg gtttaagttt ttaattttag tattttggga 4260
   ggtcgaggta ggtgaattat ttgaggttag gagttcgaga ttagtttggt taaaatggtg 4320
   aaatttcgtt tttattaaaa atataaaaaa ttagaggggc gtggtggtag gagtttgtaa 4380
   ttttagttat tcgggaggtt gaggtaggat aatcgttgga attcgggagg cggaggttgt 4440
   agtgagtcga tatcgcgtta ttgtatttta gtttgggcga taagagtaaa atttcgtttt 4500
   aaaaaaaaaa aaaaaaaaaa aaaagttttt tttttagagt aaaatgaggc gatttagttt 4560
   gagagtgtgg gtacggggat ttttcgtagt tttttttagg tagtaaggga agggaagaaa 4620
   tcgagtgtaa gcggagggta agcgggcgga tatttattag gttcggtttt atttcgtgtt 4680
   tcgcgtggtc gggagacgcg gcgtttttaa agtttagtgg gaggcgtgtt tttgggcgtg 4740
   ttttttcgga gtttcgtttc gggggttggt agtttcggtt tcgggaggtt gcgtcggttt 4800
   tggtttagcg gcggtttcgg tggtagcggc ggtggcggtt aggcgggttc ggcggttttt 4860
   gtttttcggg cggttgttcg ttcgttgttt agggcgttcg gatatacgtg ggcggtttag 4920
   cgatggttcg cgtttcgcga cgtggcgggt aggtatcggg gcgcgggatc gttgagttcg 4980
   agtgagtcgg ggtcgcgttt tcgttttcgg ttcgggtttt tcgggatggt cgtggcgttt 5040
   ttgcgggggg cgttgttgtt gtggtagttg ttggcggcgg gcggcgcggt attggagatc 5100
   ggtcgtttcg attcggagcg cgggcgcggg gttgcgtcgt gttaggcggt ggagattttt 5160
   atgtgtcgcg gtatcggtta taatttgatt cgtatgttta atttgttggg ttatacgtcg 5220
   tagggcgagg cggttgtcga gttagcggag ttcgcgtcgt tggtgtagta cggttgttat 5280
   agttatttgc gttttttttt gtgttcgttt tacgcgttta tgtgtatcga ttaggtttcg 5340
   acgtttattt tcgtttgtcg gtttatgtgc gagtaggcgc gtttgcgttg cgcgtttatt 5400
   atggagtagt ttaatttcgg ttggtcggat tcgttcgatt gcgttcggtt gtttacgcgt 5460
   aacgattcgt acgcgttgtg tatggaggcg ttcgagaacg ttacggtcgg tttcgcggag 5520
   ttttataagg gtttgggtat gttgttcgtg gcgtcgcggt tcgcgcgttt tttcggagat 5580
   ttgggttcgg gcgcgggcgg tagtggtatt tgcgagaatt tcgagaagtt ttagtacgtg 5640
   gagaagagtc gttcgtgcgt atcgcgttgc gggttcggcg tcgaggtgtt ttggtttcgg 5700
   cgcgataagg atttcgcgtt ggtttggatg gtcgtgtggt cggcgttgtg tttttttttt 5760
   atcgttttta ttgtgtttat ttttttgttg gagttttatc gtttttagta tttcgagcgt 5820
   tttattattt ttttttttat gtgttataac gtttattcgt tggttttttt gattcgtgcg 5880
   gtggtcggag cgtagagcgt ggtttgtgat taggaggcgg gcgcgtttta cgtgatttag 5940
   gagggtttgg agaatacggg ttgtacgttg gtttttttat tgttttatta tttcggtatg 6000
   gttagttcgt tttggtgggt ggttttgacg tttatttggt ttttggttgt cgggaagaaa 6060
   tggggttacg aggttatcga ggtttacggt agttattttt atatggttgt ttggggtttg 6120
   ttcgcgttta agattatcgt tattttgatt ttgcgtaagg tggcgggtga tgagttgatt 6180
   gggttttgtt acgtggttag tacggatgta gtagcgttta cgggtttcgt gttggtgttt 6240
   ttttttggtt atttggtgtt gggtagtagt ttttttttga tcggtttcgt ggtttttttt 6300
   tatattcgta agattatgaa gacgggcggt attaatatag agaagttgga gaagtttatg 6360
   gttaagatcg gggttttttt tattttttat acggtgttcg ttatttgcgt tatcgtttgt 6420
   tatgtttacg aacgttttaa tatggatttt tggcgttttc gggttataga gtagttatgc 6480
   gtagcggtcg cggggttcgg aggtcggagg gattgttcgt tgttaggggg ttcggtgttt 6540
   atcgtggcgg tttttatgtt taaaattttt atgttattgg tggtggggat tattagcggc 6600
   gtttgggtgt ggagttttaa gattttttag atttggtaga gtttgtgtta tcgtaagata 6660
   gtagttggtc gggttcgggt taaggtttgt cgcgttttcg ggagttacgg acgtggtacg 6720
   tattgttatt ataaggtttt tatcgtggtt ttgtatatga ttaagacgga tttttttttg 6780
   gagaatttta tatattttta gttatatagg tttggcgcgg ggtggttgtt gttttttttt 6840
   tgttttttac gttttgtttt ttgtattttt tagagatagt tgattagtag ttgtttagtt 6900
   gttaaggtta ggtaagtgag tatcggggat tgaggattag ggcgggattt cgtgaggttt 6960
   attaggggag atgggggttt tttttaatgc gggggttgga ttaggttgag tttttatagg 7020
   gttttagtgg aggatgtgga ggggcggggt agagggtttt agtcggagtt tatttaatga 7080
   tgtaatttat tgttgcgttt ttttggaagt tgtgattgga ataaattttc gcgtggtatt 7140
   gttgagtttt ttttggttgg gaagggggga aggtaggagg gtgagggttt tgtaggagtt 7200
   ttttttgtaa gtagagttgg tagtgggtgt tggtattttt taattaggag gaagtttttt 7260
   cggaatagtt gtgtattgta ggagttttag atattatgtg tgcgtgtttt tttttttcgt 7320
   tcgtttttgt gtttttaaat ggagataggg acgtttgcgg atgtttaggg aattttttag 7380
   ggtgatattg ttatttttgg ttttagggag tttagtttga ggttatggat gagttttttt 7440
   tttttgggaa tttgtttttt ttattggcgt ggaaagtaaa ggtgttagtt tgtgttaatg 7500
   ttgttacgag gatcgttttt ttggggtttt ggaaaattgt tggtgcgttt gtttggtttt 7560
   tcggggtttt tattagggta gtttttatta gggtagtttt agtttgtttt ttggcgtttt 7620
   taggtggggt gaagaggttg ttttggtttg tgggtttttg ggtagtatag atatgtacgt 7680
   ggagttggga agtttttgag tagagtggga attgggtttt tagtttttaa gtttgttaga 7740
   tttgagttgt taatggtagc ggtgggaatg aggtagttta ggtggttgtg tagagtttta 7800
   tattggggta ggtatgggga aaggttttat gtttatttgt gtgttggggt agtttagggt 7860
   tgttagggtt tgttttgttt ttgcggattg ggtagttggg tacgtgtggt tatttggaga 7920
   tttttattgg tttggggtta gattttaggg ttttattttg gttgttgtga ttttagttag 7980
   gtgagagtat tagttattag tgtttttggg tgtttattat atacggtcgg tatttggtgt 8040
   tagtgttagg agattttagc ggtttagttt tgttgttagt tttattttag agttacgggg 8100
   aggttttagg aggggagacg tttaggtgtt gaggatgaat ggtgaggcgg agttttaaat 8160
   ttagggtttt tcgggttgga gttttg 8186

<210> 108
<211> 8186
<212> DNA
<213> Artificial Sequence

<220>
<223> chemically treated genomic DNA (Homo sapiens)

<400> 108
   tagggtttta gttcgagagg ttttaggttt ggggtttcgt tttattattt atttttagta 60
   tttgagcgtt tttttttttg gagttttttc gtggttttaa agtgaggttg gtagtagggt 120
   tggatcgttg ggattttttg gtattgatat taaatgtcgg tcgtgtatag taggtattta 180
   gggatattag taattaatat ttttatttgg ttggagttat agtagttaag atggggtttt 240
   ggggtttgat tttaagttaa taggggtttt tagatggtta tacgtgttta gttatttagt 300
   tcgtagaaat aggataggtt ttagtagttt taagttattt tagtatatag atggatatgg 360
   gatttttttt tatatttgtt ttagtgtgga gttttgtata gttatttgag ttgttttatt 420
   tttatcgttg ttattagtaa tttaaatttg ataagtttag gagttagggg tttaattttt 480
   attttattta agggtttttt agttttacgt gtatgtttgt gttatttaga ggtttataga 540
   ttagaataat ttttttattt tatttggaga cgttagggag taggttgggg ttgttttggt 600
   gggggttgtt ttggtggggg tttcgaggag ttaggtagac gtattagtaa ttttttaagg 660
   ttttagggag gcggttttcg tgataatatt gatataggtt gatattttta ttttttacgt 720
   taataaggag agtaagtttt tagagaagga aaatttattt atagttttaa attggatttt 780
   ttggggttag aggtggtagt gttattttgg ggaatttttt gaatattcgt aggcgttttt 840
   gtttttattt ggaagtatag agacgggcgg gaggggaaaa tacgtatata tggtgtttgg 900
   gatttttgta atgtatagtt gtttcggggg gatttttttt tgattggggg gtgttagtat 960
   ttattgttag ttttgtttat aagaaagatt tttgtagggt ttttattttt ttattttttt 1020
   ttttttttag ttagagagga tttagtagtg ttacgcgggg gtttatttta gttatagttt 1080
   ttagaggaac gtaataataa attatattat taaataaatt tcggttggat ttttttgttt 1140
   cgttttttta tattttttat taggattttg tggggattta gtttggttta gttttcgtat 1200
   taggggagat ttttattttt tttaatgagt tttacggggt ttcgttttga tttttagttt 1260
   tcggtgttta tttgtttgat tttgatagtt gggtagttgt tagttagttg tttttagggg 1320
   atgtaggggg tagggcgtgg agggtaagga gggggtagta gttatttcgc gttaggtttg 1380
   tgtggttaga ggtgtgtggg gttttttaaa gaggggttcg ttttagttat gtgtaagatt 1440
   acggtgggag ttttatagtg gtagtgcgtg ttacgttcgt agttttcggg ggcgcggtag 1500
   gttttggttc gggttcggtt agttgttatt ttgcggtagt ataggttttg ttaggtttgg 1560
   aaagttttgg agttttatat ttagacgtcg ttggtgattt ttattattag tgatatgaaa 1620
   attttgagta tgaagatcgt tacggtgggt atcgagtttt ttggtagcga gtagtttttt 1680
   cggttttcgg gtttcgcggt cgttgcgtat ggttgttttg tggttcgaag gcgttagaag 1740
   tttatgttga ggcgttcgta gatatagtaa acgatgacgt aggtggcggg tatcgtgtag 1800
   aggatggaga agatttcgat tttgattatg agttttttta gtttttttgt gttggtgtcg 1860
   ttcgttttta tgattttgcg gatgtggaag agggttacga agtcggttag gaggaaatta 1920
   ttgtttagta ttaggtagtt agagaggggt attagtacga agttcgtgag cgttgttgta 1980
   ttcgtgttgg ttacgtagta aagtttagtt agtttattat tcgttatttt gcgtagggtt 2040
   aggatgacga tggttttgag cgcgggtagg ttttaggtag ttatgtggaa atagttgtcg 2100
   tgggtttcga tggtttcgtg gttttatttt ttttcggtag ttaggaatta ggtgagcgtt 2160
   aggattattt attagagcga gttggttatg tcgaagtagt agagtagtag gaagattagc 2220
   gtgtagttcg tgttttttag gtttttttgg attacgtaga gcgcgttcgt tttttggtta 2280
   taggttacgt tttgcgtttc ggttatcgta cggattagga aggttagcga gtagacgttg 2340
   tagtatatgg agaggaagat gatggggcgt tcggggtatt ggaagcggtg gggttttagt 2400
   aagaaggtga gtatagtgaa ggcggtggag aagaagtata gcgtcgatta tacggttatt 2460
   tagattagcg cgaagttttt gtcgcgtcgg gattagaata tttcgacgtc gggttcgtag 2520
   cgcggtgcgt acgagcggtt tttttttacg tattggaatt tttcggggtt ttcgtaggtg 2580
   ttattgtcgt tcgcgttcgg gtttaggttt tcgggagggc gcgcgggtcg cggcgttacg 2640
   ggtagtatgt ttaggttttt gtggggtttc gcggggtcgg tcgtggcgtt ttcgggcgtt 2700
   tttatgtata gcgcgtgcgg gtcgttgcgc gtgggtagtc gggcgtagtc gagcgagttc 2760
   ggttagtcga agttgaattg ttttatgatg ggcgcgtagc gtaggcgcgt ttgttcgtat 2820
   atgggtcggt aggcgggaat gggcgtcgag atttggtcgg tgtatatggg cgcgtagagc 2880
   gagtatagga agaagcgtag gtggttgtgg tagtcgtatt gtattagcgg cgcgaatttc 2940
   gttagttcgg tagtcgtttc gttttgcgac gtgtggttta gtaggttggg tatgcgggtt 3000
   aggttgtagt cgatgtcgcg gtatatgggg atttttatcg tttggtacgg cgtagtttcg 3060
   cgttcgcgtt tcgggtcgaa gcggtcgatt tttagtgtcg cgtcgttcgt cgttagtagt 3120
   tgttatagta gtagcgtttt tcgtagaggc gttacggtta tttcgggagg ttcgggtcga 3180
   aggcgggagc gcggtttcgg tttattcggg tttagcggtt tcgcgtttcg gtgtttgttc 3240
   gttacgtcgc ggggcgcggg ttatcgttga gtcgtttacg tgtgttcggg cgttttgggt 3300
   agcgagcggg tagtcgttcg agggatagga gtcgtcgagt tcgtttagtc gttatcgtcg 3360
   ttgttatcgg agtcgtcgtt gagttagagt cggcgtaatt tttcggggtc gaggttatta 3420
   gttttcgggg cggggtttcg ggaggatacg tttagggata cgttttttat tgggttttaa 3480
   aggcgtcgcg tttttcggtt acgcggagta cggggtgggg tcgggtttgg tgggtgttcg 3540
   ttcgtttgtt tttcgtttat attcggtttt tttttttttt ttgttgtttg ggagaggttg 3600
   cgggaagttt tcgtatttat atttttagat tgggtcgttt tattttattt taggagggag 3660
   attttttttt tttttttttt ttttttgaga cggagttttg tttttgtcgt ttaggttgga 3720
   gtgtagtggc gcgatatcgg tttattgtaa ttttcgtttt tcgggtttta gcgattattt 3780
   tgttttagtt tttcgagtag ttgggattat aggtttttgt tattacgttt ttttaatttt 3840
   ttgtattttt agtaaagacg gggtttattt attttggtta ggttggtttc gaatttttga 3900
   ttttaggtga tttatttgtt tcggtttttt aaagtgttgg gattagaggt ttgagttatc 3960
   gcgtttagtt ggaagatttt ttatttttat ttttaattgt tatttttttt tagagatagg 4020
   gtttttttgt gtcgtttagg ttggagttta gtggaacgat tatagtttat tgtagttttt 4080
   atttttcggg tttaagcgat tttttcgttt tagttattag agtagttggg attataggtg 4140
   ggtattatta ttttcggtta attttaaaat gtttttgtag agatagggtt ttattatgtt 4200
   atttaggttg gttttgaatt tttgggttta agggatgttt tcgtttttgt tttttaaagt 4260
   gtcgggatta taggcgtgtt ttatcgtgtt cggttagggg agattatttt taaggttttt 4320
   tacgtgtgga agtttgtttt gtatttagtg gtagtttaaa tatagcgttt gttttttttt 4380
   ttagtgtatg tgatttgggg tttttttttg agttttagtt tgtttttttg taatgtaggt 4440
   agataatttt attgttaaaa ttaatagttt tgttaggcgt agtggtttat ttttattttt 4500
   attttgtttt attagttggg gtaatatagt gagatttcgt ttttaataaa gaaaaaaaaa 4560
   tagttttttt ttttttgaga tggagtttcg tttttgtcgt ttaggtttat tttatcgttt 4620
   aggagtgtaa tggtgcgatt ttggtttatt gtaattttcg tttttcgggt ttaagtaatt 4680
   tttttgtttt aattttttaa gtagttggga ttataggcgt ttattattat tttcgggtaa 4740
   tttttgtatt tttagtagag ataggggttt tattatgttg gttaggttgg tttcgaattt 4800
   ttgattttag gtgatttatt tatttcggtt ttttaaagtg ttgggattat aggtatgagt 4860
   tacggtgttt ggtttgaaaa aaaaaaaaaa gaaaaaaaaa aaaagaaaat taataatacg 4920
   tgttgggtat ttgaatttga tcgattatat tttatatttg tttttattgt atttttttag 4980
   taatttaaga atgttacgtt atttttattt tttaggtggg gaaattgagt tttagagagg 5040
   aatgatatat ttaagggtat agagttatta agtgttttat ttgtgattgt ttgtttcgga 5100
   atttatattt attttataag gttatcgtgt ttttttgggg agtgtcgtta ttattttttt 5160
   attttttttt aggttgtttt tttgagattt ttgggattcg tttagaaatt gttatggttt 5220
   ttgggtagtt ttgtgggtga aatttaagtg ttatcggtag gttatataag gggtaagggg 5280
   ttttgatttg ggatttttta gagaaggtta tttttttttt atttaattgt ttttattttt 5340
   ttggtttgga aatatttgga ggatttagaa tgttttatat gagtatttag tattgtttta 5400
   tgtgagtatt tagtatcgtt ttatgtgagt atttagtatt gttttacgtg agtatttagt 5460
   attattttac gtgagtattt agtatcgttt tatgtgagta tttagtattg ttttacgtga 5520
   gtatttagta ttgttttata tgagtattta gtattgtttg acgtgagtat ttagtattgt 5580
   tgtacgtgag tatttagtat tgttttatac gagtatttag tatagggtta ggtattttgg 5640
   ttgagtttag ggatggtgag tgaatgaatg agtgagtaat ttagaatttt agggaggtat 5700
   agaaattgat cgaggatttg ttgtgagttt tttgggtttt gggttaggga ttttatagtg 5760
   attatttgtt tatttttatt atagtgttat aaggaatttc gtatttatat atagatgaga 5820
   aattgaggtt tagtaaattt aaatatttgt ttaggattac gtagttcgta agggttaaga 5880
   ttggtattta ggttttttta ttgtaagagg ttgtttgtta atattattag aggtaggata 5940
   ttagtttagt cggtttagtt tagttatatt tgtttgtatt agagtttggt ttgtggtagg 6000
   gatttgggaa ggagttagta gtggtggtgg taatggtgag aaaatggggt tattattttt 6060
   tatatatttt tgtaattagt attttgttat ttttagagtt gtgttggagt agggagggga 6120
   ttaggtaggg gatatgttat ttcgtgtata tatttagttt ttcgtttttt aagaggatcg 6180
   ggagatgggg tattaaggag acgtagttag ttttgtgttt agtataggat agtatttagt 6240
   tatatgtggg ttatgagacg ttttgaagtt tttaaagagg taaggtagga gtatggtatt 6300
   tgtgattttg ggggatttag gtgaggtatc gtggtttaag tttgtaattt tagtattttg 6360
   ggaggttaag gtgggagaat tatttgagtt taggagttcg agattagttt gggtaaaata 6420
   gtaagatttt attattttta aaataaaaat aaataaaaaa gttagggatg gtggtatgcg 6480
   tttgtagttt tagttattta agaggttgag gttggagaat tttttgagtt tgggaggttg 6540
   tagtgagtta tgattatatt attgtatttt agtttgggta atagagtgag attttatttt 6600
   tagaaaaaaa ataaaaagaa agatagggag agggggaggg gaggtagggg gagggggagg 6660
   ggggaggagg ggaaggggag ggagggggag gaggggaagg ggatggaggg ggaggagggg 6720
   aaggggaggg agggggaggg gttagaggga gggaggggga gggggagggg agcggggggg 6780
   aagggagggt agggtagggt agggtattgt agggaaggga agggatttag ttttgattat 6840
   ttgggtattg ataatggagg gttttaaaat tttagtagta gttgtttaga ataaagtgta 6900
   tgtttttagg gatggtagtg gaagtttttt taagtagaga tttcgtttta tttttttatt 6960
   ttttgttata ttcgatattt tatttttatt tttttttaga aatattttgt aaagttatgt 7020
   ttttaggttt ttgttaggtt tgttttttta gttttgaagg tttttttttt tttttgtttt 7080
   atttggtatt ttagattgaa gtattatttt ttagggttag tttagtagag ttgggttggg 7140
   gttggaggtt ttgttttgtg gatttagggt ttaggttagt ttatttgttt agtatattgt 7200
   ggtttttgta ttcgtgtttt ttttcggttt tatgttagtt attgtttaag ggttatggaa 7260
   taatattaag aaataatata ttggtcgggt gtaatgattt atatttgtaa ttttagtatt 7320
   ttgggaggtt aaggtgggag gattgtttga gtttagagtt taagattagt ttgggtaata 7380
   tagtaagatt tttttttttt ataaaaaaat ttataaaaaa ataattatat tatttttttg 7440
   tatttggttt taaggaggtt taggtgtata gatgatagtg atgtgtggtt ttaatttttg 7500
   agtttttagt tgttagggtg ttaggaggtg gggttttttt tttatagatt tgggaggttt 7560
   aggttgttag aaatttttta gtgttggggt tagtttaaga gttaggtttt gtgggttttc 7620
   gtttttattt tgttagggtg gagaagcgtc gggggttttt ttttttggta gtttcggtgg 7680
   gtagggcgtt ttaggggtcg tgggatgtta ggtttgttat tatggtaacg gtcgcgtttg 7740
   ggaagttaga ggaggggagt ggatgagttt cgtttttacg tagatgcggt ttgggagaga 7800
   agaggttttt ggttttttgt ttatttattc ggttttgtta ttcgattttt ttttttataa 7860
   aggtggaata atgtttttgg agtttattga aagggagatt atgcggagga tcggaggatc 7920
   ggaggggtcg ggaggaggga ggttattatt aatgttattt ttcggtattt ttttttgggt 7980
   cgaggggttt ttttaatttt tttttttttt ggggagaagt cgtttttttt gggatttttg 8040
   gttggggtta gtagttgttt tgtatatgtg ggaagggacg tttttttgat ttgtgttttt 8100
   gtatttttaa tttttttttt ttcgaatata tgttaatttt tagagggtat attttttttt 8160
   tttttttttg ttttttttag gatgat 8186

<210> 109
<211> 8447
<212> DNA
<213> Artificial Sequence

<220>
<223> chemically treated genomic DNA (Homo sapiens)

<400> 109

agagggaatt aaagtgatta gttattttta tagtgagagt taggtaggaa ttttgggttt 60

ttttttggag agggtttttt aattaagttt agagtttttt ttttttgttt ttagtttatt 120
tttgggagtt ttttgttttt taaataatag ttttggtttc gagatttttt tatgttttta 180
aagttttagt ttattgggtt agacgttgat tgaaaggaat attgtaattt tttttagtgt 240
tttattattt ttagtgtttt taggggttag attaggtttt ttgggtataa aatgtgttga 300
agttaaaatt agatttaaaa aataatttaa tttttttgaa tttaaattta tttattttta 360
aaatgagagt attgggttag gtatggtggt ttatgtttgt aattttagta ttttgggagg 420
tcgaggtggg tagattattt gaggttagga gttcgagatt agtttggtta atatggtgaa 480
atttcgtttt ttataaaaat ataaaaaatt agtcgggcgt tttggtgcgt atcggtagtt 540
ttagttattt gggaggttga ggtaggagaa ttatttaaat tgggaggtag aggttgtaat 600
gagttgagat tgagttattg tattttttat tttagtttaa gtgatagagt aagattttgt 660
tttaaaaaaa aaaaaaaaaa aaagacgggt attaagagtt attttcgggt tgggtacggc 720
ggttgatgtt tgtaatttta gtattagggg aggttaagtt atatatagag atattgtggt 780
tgtttaattc gtttatttat ttattttttg agtttataaa atgttttttt gtggattatt 840
tgaggttagg agttgggatt agtttggtta atatggtgaa attttatttt tattaaaaat 900
ataaaaatta gttgggcgtg gtggtatata tttgtaattt tagttattta ggaggttgaa 960
gtaggaaaat tttttgaatt taggaggcgg gggttgtagt gagttgagat tgcgttattg 1020
tattttagat tgggtgagag agcgaggttt tattttaaaa aaataaaaaa agttattttc 1080
ggttatatgt ggtgatttat atttgtaatt ttagtatttt gggaagttaa ggcgggtaga 1140
ttatttgagt ttagaaattt gagattagtt tgggtaatat ggtgagattt tgtttttata 1200
aagaatataa aaattagttg ggtatcgtga tgtgtatttg tagttttagt tatttgggag 1260
gttgaggtgg gaggattatt tgagttttat gattacgtta ttgtatttta atttgggtaa 1320
taggtaagat tttgttttaa aaaataataa tgaaaattat tatttttata gtattgttat 1380
gaagataaat aatatataaa tgtaatggag tataaaattt agagtttagt tgattggaat 1440
aagtgtttta aaaaaaatag ttattattgg tcgggtatgg tggtttatat ttgtaatttt 1500
aggattttgg gaggtcgagg taggtagatt ataaggttag gagatcgaga ttattttggt 1560
tattatagtg aaattttatt tttattaaaa atataaaaat tagtcgggcg tggtggtacg 1620
tatttgtagt tttagttatt tgggaggttg aggtaggaga attatttgaa ttcggggggt 1680
ggaggttgta gtgagttaag attatgttat tgtattttag tttgggtgat agagtgagat 1740
tttatttaaa aaaaaaaaaa aaaaaagtta ttattaataa ttgttattaa tagttgatta 1800
ttttatgtaa tttaatttta tttatttttt tagtagagat agggttttat tatgttagtt 1860
aggatggttt tgattttttg atttcgtgat ttatttgttt cggtttttta aagtgttggg 1920
attataggcg tgagttattg cgtttggtcg taatttattt tattgaattt ttaataagag 1980
ttttgtgagg taagtattat cgttattatt gtttagagtt ttagaggggt ttagaggttt 2040
tttaagatta tataatatat aaatagtaga attaagtttt aaaattaggt ttgtttgttt 2100
ttagaatttt tgttttttat taagttacgt atagagatat tgtggttgtt taatttattt 2160
atttatttat tttttgagtt tataaaatgt ttaagaagtg gtaagataat tttttttttt 2220
aagaaattta gagtttaatg ggaaaggtag gttatgttta taaataatta tattttaaag 2280
tagaaaatga tgatttttgt taataagggt tagttagaga ttaatttatt tatataatac 2340
gtattgagag ttgttgtggg ttgggttttt tgttaagtat tgggtataat ttaaagataa 2400
atatggtata gtttagttat aatttaatgg gagagatagg tatgtaaaga aattattata 2460
gttataagga attaagttat gattttaaaa tatgataaaa tatagagtgg aagaaataaa 2520
ttgtgttggg atagggggta gaggtagaga ttgaataaat ttttagttgt taaaaaatgt 2580
aaaagagtat agaaaatgtg tgatggtagt ttagggggag tgtgtatttt ttggaggatg 2640
taagaaggtt ttatggagga ggtggtacgt gggttggtat ttgtttgatg ggtagttttt 2700
agtagagggt tgaattagag agagtttgga ggtttagaag ttagttatag gggagaatta 2760
tagcgaggta agtaatgagg aggatagggt agtgatagag gtggtgagga gggagtgaag 2820
ggagggatgt tagagaggat tggtttagtg ggaataagtt tgtttttaat ttttttattt 2880
tattaatatt atttatttat ttattttgtg tgtgtgtgat ggattttagt tttgttattt 2940
aggttggagt gtagtggtgc gatttcggtt tattgtaatt tttgttttta tattcggtta 3000
attttttgta tttttggtag agatgtttgt aattttagta ttttgagagg ttaaggtggg 3060
tggattattt gaggttagga gttcgagatt agattggtta atatggtgaa atttcgtttt 3120
tattaaaaat ataaaaaata gataggtatg gtggtgtgta tttgtagttt tagttattcg 3180
agaggttgag gtaggagaat cgtttgaatt taggaggtgg agattatagt gagttaagat 3240
cgtattattg tattttagtt taggtgatag agtgagattt tgttttaaaa aaaaaaaaaa 3300
aaaaaaaaaa aaagaaggaa gtttatttga ttattgtttt tttttcgtta ttttttttat 3360
agcgtgaaaa gtgttgttgt agagaaatgt atagaagggg taattggttt tgtttgggga 3420
cgaggaaagg tttttcgaag gaggtaaatt tttagttaaa ttttttagga tgtagagtgt 3480
tttttttagt tgtgggagag agagaggagg atattttagg tgaagcgaat agtttttttc 3540
gttgattttg tgttaagttg tttaggggtt ttacggcgaa ggttttttag aggaagggat 3600
tttttatatt ttaatgaggt attttaagaa atttttgagt ttagttggaa tttagatttt 3660
ttagagttat tagaaaaata ttatgtggga aattgtgttc ggcgcggtgg tttacgtttg 3720
taattttagt attttgggag gtcgaggcgg gcggattacg aggttaggag atcgagatta 3780
ttttggttaa tatggtgaaa ttttattttt attataaacg taaaaaatta gttaggtgtg 3840
gtggtagggg tttgtagttt tagttattgg ggaggttgag gtaggggaat ggcgtgaatt 3900
tgggaggtag agattgtagt gagttgagat tatgttattg tatttaatag agcgagattt 3960
cgttttaaaa aaaaaaaaaa aaaaagcgtt atgtgggaaa tggttttttt agttttttgt 4020

aggggtcgtt gttgttttag atttagttag ttttgtttaa gaaaatttag aggacgtttt 4080
tgttggggtg gtggtggggt atattttgga tttgtcgtta tttaaggagt gaatttaagg 4140
gcgatagtgt ttttaagttt gaagaatatg tatttagtta atggtatttg ggggtaggga 4200
gggggtgtag tgggtattga tattttttag tttttgggtt attataagtt tattaaaata 4260
aaagaaaatg tgggtaaatg ttgttatttg tgttgttttt atttttaata tatatatata 4320
ggtatatata tatataggta tatatatata taggtatata taaatatata tatatatata 4380
tatatatata tatatatata tatatatata cgtataataa attaggtttt agattagatg 4440
taggagttga aattgtttta aaaatatttt aagaatttaa ttataaattt atatttttta 4500
tgtttttggg gtttaagatt tttgagttta ttttttagta ttttttttcg ggaaagtggg 4560
gtgggttgtt ttatgaggtg gaggtgaaga tttttgagtt tgtttcgtgg agggggaggt 4620
tttttaagtt tagagtttcg ttgtagggtt ttgtgttagg agttttcgtg agttatggtt 4680
tcgaaagggt agcggtgatt ttttttatat aaatatatcg tatttaaatg agtttagata 4740
gtatgatatt agagagtaat taaattggtt tgggttggaa tttcgttttt aatttttgag 4800
tttaggtttg taaaagattt ttttgagtat ttgtaggttt gtgagtgtgt gtgtgtgtgt 4860
gtgtgtgtgt gtgtgtgtgt gtgtgtgtga agtattttta ttggaaagga tttaaaatta 4920
gggggaaaaa aaaattggag tatataggta gtattacgtt attttttttt tttggaaaaa 4980
ttttttagtt ttatataagt tttttttaag tttttagtta gttgtgtagg agaaaggggg 5040
cggttggttt ttttttttta agaacgagtt atttttagtt gttgattgga gacggtgtac 5100
gtttggatac gagagtattt ttattatggg attggatata aatatatatt cggtagattt 5160
taagagtttt agattgagga gaaagttttt ttttttgttg ttattgttgt tgtcgttgtt 5220
tttgaaagtt tatttttttt atggtttttt ttgttaaatt agaggtattt ttgttgttgt 5280
cgttgttttt tttggtgtta tttagcggtt ggttagagga tgagattttt taaatttttt 5340
atttttttgt tttggtattt ggtttggttg gatttggaat ttatttgtat tgtgttgggt 5400
gtttttgatt tgggttagag attttagggg attaggttag gattggttaa agtagaggtt 5460
aaggagaggt tttttttggt tcggaacgtt tttaggttag ggggttatag ttatggtggg 5520
ggggttatta atgttaatgt tagggtaaag ggaggtatcg ggtagatagg aggtttgata 5580
tagtttaaga aggatgaatt taaaaagttg ttttttagat cgggcggttt tgaatttaag 5640
ttaggatatt ttttttaaat aaggtaggtt atagttcgga ttgtgatttt aaaaggatag 5700
tttttcggag gtaaggtatt tttaaaagta ggatttgttt ttagtttttt tttgttgaag 5760
aaggttaggg agttcgggtt tttacgagag tttaaggagt cgtttcgttt attttttatt 5820
atattttacg agtatatgtt ttcgttgtat aggacgttgt tcgatgttga tagaaaggga 5880
ggtaatagta gcgtgaagtt ggaggttggt ttggttaata ttattattag ttttattgat 5940
aaagggtaag gtgagggggc ggggtggtag gggtacggtt tagagggagg ggtatttgta 6000
tgaatggagg ggtttttaaa gttttggtat tgttttggtg ggagatattg tgtgtatttg 6060
gttcggggtg ggtgtttggg gatattgata tatattttat atatagtaga ttttaggttg 6120
tttatatatt agggttggag ggtatttagg aagtattagg tttaacgttt gtaaggtgta 6180
gagtgggtat taaagtttag agaagagata agatttgtta gttcgtggta gagttggaga 6240
ttagatttcg agatttgatt gtttagttgt gtatttgtat ttgttgggtt ggtcgaattt 6300
tttaagtttt ataggtttgt aggcggtttt agacggtaga gaaagaaatg aattttgagg 6360
tttttatgtt tttgtaattt tgtttttagg taaggggata gaggaggtaa gagaggtgta 6420
tatagtaaat ggggtatttt ggagttatgg aatgatgttt agatatatgg ggagggagta 6480
gatttagggt ttaattcgta gtatgatttt gggaaagttt ttttttattt ggggttttcg 6540
tttttttatt tgtatattta tatggggttg gatagggtgg tttttgagag tttttttaat 6600
atgaaaattt tagatgttgt tttgaatatg ttttattttt tttattaatt ttagagatta 6660
tttgtttttt tttgtttttt tcggtttatt taatgttatt gttgtagttt ttttttttta 6720
tttgttgtgt gatttaagta agtttttttt tttttagggg ttttagtttt tttttagtgt 6780
gaagagggag gtgggttgtt ggtcgtttaa gttgtttgta gtttatttgt tttggtttta 6840
ggttggtagg gtgggggagg ggttagaggg tgggagaggt tttttattag agttggtttg 6900
gagggggttg ttagggggcg ggggaggtat agaggtagta ggtttggttg aggtggggga 6960
ggtggtttgg ttgtttgttt agttagggaa agttgtgatt ttttgggggt ttataaaata 7020
agttcgtgtg ttttttagta ttaagggagt ttggagaggg ggagacgggg agtttaaagg 7080
tttaattagt tattttttag agtaatatat gaaagttttt taatggtaaa ttgtaattta 7140
ggaaggaggg tatgttgatt cggagatatt attgagtttt ttatatatat atatatagtt 7200
ttatgtttat atatatatgt tgaattatat atgtatatgt atgttgattt atatatatac 7260
gtgttgaatt atatacgtat atatatgtcg aattatatat atatgtatgt gtatatatat 7320
ttttttatat atatatataa gattaattat gtatatatag atatatatgt gttttttaaa 7380
tagttatata agtttaatta tttatatata taatgcgtta ttaacgtata tattttttta 7440
tatttatata tgaatttaat tatatatatg tatataattg taatatatat atatgtttag 7500
gagagtaaga tatataggta ataaaatttt tgtattggaa agttaggaaa attgggttgt 7560
aggttagtgt ggattagttt tgttattaat ttgttgtgtg attttagtta agttattttt 7620
tatttttgtg ttttatttgt ataataagag tattaaggtt ttttttaagg gatggtggaa 7680
gtagttgaga atttgagtaa gttatttaat ttttttgaat tttgggattt ttttttggaa 7740
aagcgtgtat tttttttttt tttttttttt ttttttttta gttagggttt ttttttgttg 7800
tttaggtcgg agtgtaatag cgaatttacg gtttattgaa gttttgattt ttttggggtt 7860
aattaatttt tttattttag ttttttgagt agttgggatt atatgtgtac gttattatgt 7920
ttggttaatt tttgtatttt ttgtagagat agagtgttat tatgttgtgt aggttggttt 7980

cgaatttcgg agtttaagta atttatttat tttagttttt taaagtgttg ggattgtagg 8040
tgtgagttat tgtgtttagt taataggagt aattttaatg tttattttat ttattttata 8100
aatgagatat tacgttagtt tatggttggg agagagatag gtagaaggta gttataaagt 8160
tgataaattt ttgataatac gtatattatg atataaatat tagaattacg taaattattt 8220
ttagttatat ttttaggtga atatttttat ttggtcggtt atgtaggaaa tgtttttatt 8280
tttaaaaagg tttttagtgg tttggttttt ggtttttgtt agaatggggt agaggtgaaa 8340
gaaagttttt tggacgggga gagagttggg gttttttttc gtagtttcga agtgattggt 8400
ttttttggtg aggttgtagg gaatgatttt tgggtgtttt ttttaga 8447

<210> 110
<211> 8447
<212> DNA
<213> Artificial Sequence

<220>
<223> chemically treated genomic DNA (Homo sapiens)

<400> 110

tttagagaga atatttagaa gttatttttt gtaattttat taagggagtt agttatttcg 60
aggttgcggg ggaaggtttt agtttttttt tcgtttagag agtttttttt tatttttgtt 120
ttattttgat agaagttaag agttaaatta ttgagagttt ttttgaaaat gagagtattt 180
tttatatagt cgattaagta ggggtgttta tttgaagatg tggttgggga tgatttgcgt 240
gattttggtg tttgtgttat gatatacgtg ttattagaga tttattagtt ttgtaattgt 300
tttttattta tttttttttt agttatgagt taacgtgatg ttttatttgt gagataggtg 360
gagtgagtat taaaattatt tttgttgatt gggtatagtg gtttatattt gtaattttag 420
tattttggga ggttgaggtg ggtggattgt ttgagtttcg gagttcgaaa ttagtttgta 480
taatatggtg atattttgtt tttataaaaa atataaaaat taattaggta tggtggcgtg 540
tatatgtagt tttagttatt taggaggttg aggtgggagg attgattgat tttagggagg 600
ttaaggtttt agtgagtcgt gaattcgtta ttgtatttcg gtttgggtaa tagagaaaga 660
ttttggttaa aaaaaaaaaa aaaaaaaaaa aaaaaaaata tacgtttttt tagaggagaa 720
ttttaaggtt tagagaagtt agatgatttg tttaaatttt tagttgtttt tattattttt 780
taaaggagat tttggtgttt ttgttatgta gatgaggtat agagatgggg agtgatttga 840
ttaaggttat atagtaagtt aatgataaag ttggtttata ttgatttata gtttaatttt 900
tttggttttt tagtgtaagg attttgttgt ttatgtgttt tatttttttg agtatgtgtg 960
tgtgttatag ttgtatgtat gtgtgtgatt gggtttatat gtgaatatga aagagtgtat 1020
acgttaatga cgtattatat gtataaatga ttgggtttgt gtgattattt gaagagtata 1080
tatatgtttg tatatgtatg attgattttg tgtgtgtatg taagaaggtg tgtgtgtata 1140
tgtatatgtg tgtgattcgg tatgtgtgta cgtgtgtgat ttagtacgtg tgtgtgtgag 1200
ttagtatgta tgtatatgtg tgatttagta tgtgtgtata aatataaggt tatgtatgtg 1260
tatgtgagaa gtttagtagt attttcgaat tagtatgttt ttttttttaa attgtaattt 1320
gttattgggg agtttttata tattgtttta gaaggtgatt aattaggttt ttaaattttt 1380
cgtttttttt tttttaagtt tttttagtgt tggggggtat acggatttat tttatgagtt 1440
tttaaggggt tatagttttt tttggttggg taggtagtta gattattttt ttttatttag 1500
ttagatttgt tgtttttgtg tttttttcgt tttttggtag ttttttttag gttagtttta 1560
atgaggagtt ttttttattt tttgattttt tttttatttt gttagtttag gattagagta 1620
aatgagttgt aggtaatttg agcgattaat agtttatttt tttttttata ttaggaagaa 1680
attgaggttt tgagaggggg agggatttgt ttagattata tagtaagtgg agaggaaaag 1740
ttgtaatagt aatattaggt gggtcgagag agatagaggg gagtaggtag tttttggagt 1800
taatgaggag aatgaggtat gtttaggata gtatttaaag tttttatgtt ggaagggttt 1860
ttagaaatta ttttgtttaa ttttatatag gtgtatagat gggaaaacgg agattttaag 1920
tgagaaggag tttttttaag gttatattgc gagttaggtt ttgggtttgt ttttttttta 1980
tgtatttgaa tattatttta tgattttaag atgttttatt tattgtgtgt attttttttg 2040
ttttttttat ttttttgttt gagggtaaag ttgtagaagt atggggattt taagatttat 2100
tttttttttt gtcgtttgag atcgtttata aatttgtgga gtttggaaag ttcggttagt 2160
ttagtaggta tagatgtata attgagtaat taagtttcgg aatttggttt ttaattttgt 2220
tacgaattgg taggttttgt ttttttttta ggttttagtg tttattttgt attttgtagg 2280
cgttggattt ggtgtttttt aggtgttttt tagttttgat gtgtgggtag tttaggattt 2340
gttatgtgtg agatatgtgt tagtgttttt agatatttat ttcgggttag atgtatatag 2400
tgttttttat tagggtagtg ttagggtttt gaaagttttt ttatttatgt agatgttttt 2460
ttttttgagt cgtgtttttg ttatttcgtt tttttatttt gttttttgtt aataaagttg 2520
gtgatggtgt tggttaggtt agtttttaat tttacgttgt tgttgttttt ttttttgtta 2580
gtatcggata gcgttttgta tagcgagagt atgtattcgt ggggtgtgat ggggggtggg 2640
cgaaacggtt ttttgggttt tcgtgggggt tcgggttttt tggttttttt tagtaggaag 2700
gagttgggga tagattttgt ttttgggggt gttttgtttt cgggaagttg tttttttggg 2760
gttatagttc gggttgtagt ttgttttgtt tggggagggt gttttggttt gggtttaggg 2820

tcgttcggtt tggggggtag ttttttgggt ttattttttt tgggttgtgt taggtttttt 2880
gtttgttcgg tgtttttttt tgttttggta ttggtattgg tggttttttt attatagttg 2940
tgattttttg gtttgaagac gtttcgggtt agggggggtt tttttttggt ttttgttttg 3000
gttaattttg gtttggtttt ttggggtttt tggtttaagt taggggtatt taatatagtg 3060
tagatgaatt ttaggtttag ttaagttagg tattaaagta agaaagtgag gagtttgggg 3120
agttttattt tttggttagt cgttgaatga tattaaagag aatagcggta gtagtaaagg 3180
tgtttttggt ttggtaggaa aaattatgaa aggagtggat ttttaaaagt agcggtagta 3240
gtagtagtag tagaaggaaa ggtttttttt ttagtttgag atttttgaag tttgtcgggt 3300
gtgtgtttgt atttagtttt atagtggaaa tgttttcgta tttagacgtg tatcgttttt 3360
agttagtagt tgaaaataat tcgtttttga aaggagaaag ttaatcgttt tttttttttt 3420
gtataattga ttgagggttt gaaggaggtt tgtataaggt tgagggattt ttttaagaag 3480
gaagaatggc gtaatgttgt ttgtgtgttt tagttttttt ttttttttag ttttgaattt 3540
tttttagtga aaatatttta tatatatata tatatatata tatatatata tatatatata 3600
tatttatagg tttgtaggtg tttagaaaaa ttttttataa atttgaattt aggaattgga 3660
aacggaattt taatttaaat taatttaatt attttttgat gttatgttgt ttaaatttat 3720
ttaagtgcga tatatttatg tgaaaaaaat tatcgttgtt ttttcgaggt tatggtttac 3780
gggggttttt ggtatagagt tttgtagcgg gattttaggt ttagggggtt ttttttttta 3840
cggggtagat ttaggggttt ttatttttat tttatggagt agtttatttt attttttcga 3900
ggagagatgt tgaagaatga gtttaaagat tttaagtttt agaagtatgg ggagtgtggg 3960
tttgtagtta agtttttaga gtgtttttaa agtagtttta atttttgtat ttagtttggg 4020
atttgatttg ttatgcgtgt gtgtgtgtgt gtgtgtgtgt gtgtgtgtgt gtgtgtgtgt 4080
gtgtttatgt gtgtttgtgt gtgtgtgtgt ttgtgtgtgt gtgtgtttgt gtgtgtgtat 4140
tgggagtagg gatagtatag atggtagtat ttatttatat ttttttttat tttaataaat 4200
ttgtggtgat ttagagattg aaaaatatta gtgtttatta tatttttttt ttatttttaa 4260
atgttattga ttgagtgtat attttttagg tttgggggta ttgtcgtttt taagtttatt 4320
ttttggatgg cggtaggttt agggtgtatt ttattattat tttagtagag acgttttttg 4380
agttttttta gataagattg gttgagtttg gggtagtagc ggtttttata ggaggttgga 4440
aaagttattt tttatatgac gttttttttt tttttttttt tgagacggag tttcgttttg 4500
ttgagtgtag tggtatgatt ttagtttatt gtaatttttg ttttttaggt ttacgttatt 4560
tttttgtttt agttttttta gtagttggga ttataggttt ttgttattat atttggttga 4620
ttttttacgt ttgtagtaga gatggggttt tattatgtta gttaggatgg tttcgatttt 4680
ttgatttcgt gattcgttcg tttcggtttt ttaaagtgtt gggattatag gcgtgagtta 4740
tcgcgtcggg tataattttt tatatgatgt ttttttggta gttttggaag gtttgaattt 4800
tagttgaatt taagggtttt ttaaagtgtt ttattgaagt atggaagatt tttttttttg 4860
gggaattttc gtcgtagagt ttttgggtaa tttaatatag ggttagcggg gaaagttgtt 4920
cgttttattt gaaatatttt tttttttttt ttttataatt ggaggaagta ttttatattt 4980
tgagaagttt agttgaaaat ttattttttt cgggaagttt tttttcgttt ttagatagag 5040
ttagttattt tttttgtata tttttttata ataatatttt ttacgttgta agaaggatgg 5100
cgggggagaa gtaatagtta gataggtttt tttttttttt tttttttttt tttttttttt 5160
tgagatagag ttttattttg ttatttaggt tggagtgtag tggtgcgatt ttggtttatt 5220
gtaattttta ttttttgggt ttaaacgatt tttttgtttt agtttttcga gtagttggga 5280
ttataggtgt atattattat gtttatttat tttttgtatt tttagtagag acggagtttt 5340
attatgttgg ttagtttggt ttcgagtttt tgattttaag tgatttattt attttggttt 5400
tttaaagtgt tgggattgta ggtattttta ttaaaaatat aaaaaattag tcgggtgtgg 5460
aggtaaaggt tgtagtgagt cgagatcgta ttattgtatt ttagtttagg tgatagagtt 5520
agagtttatt atatatatat aaaataagta aataaataat attaataaaa taaaaaaatt 5580
aaaaatagat ttgtttttat tgaattagtt ttttttggta tttttttttt tatttttttt 5640
ttattatttt tgttattgtt ttgttttttt tattgtttgt ttcgttatga ttttttttta 5700
taattggttt ttaggttttt aggttttttt tggtttaatt ttttattaaa agttatttat 5760
taaataagtg ttaatttacg tgttattttt tttatgaagt ttttttgtat tttttagaaa 5820
atgtatattt tttttgagtt gttattatat attttttgtg tttttttata ttttttggta 5880
gttgaaagtt tatttagttt ttgtttttgt tttttatttt aatatagttt gtttttttta 5940
ttttgtattt tgttatattt taaaattata atttaatttt ttataattat aataattttt 6000
ttatatattt gtttttttta ttagattatg attagattgt gttatattta tttttgaatt 6060
atatttagtg tttggtagag ggtttagttt atagtagttt ttagtacgtg ttatatgaat 6120
aaattaattt ttaattggtt tttattgata aaaattatta ttttttattt tgaggtgtag 6180
ttatttgtgg atataatttg ttttttttat tagattttaa gttttttgaa gggaagaatt 6240
gttttgttat tttttaagta ttttgtaagt ttagagagtg aataaatgaa tgagttgaat 6300
aattataatg tttttgtacg tggtttgata aagagtaagg attttggaga taaatagatt 6360
tggttttgaa gtttggtttt gttatttatg tattgtgtga ttttgggaag tttttaaatt 6420
tttttgaaat tttaaatagt gataacgata gtgtttattt tataggattt ttgttggaga 6480
tttaatgaag taaattgcgg ttaggcgtag tggtttacgt ttgtaatttt agtattttgg 6540
gaggtcgagg taggtggatt acgaggttag gagattaaga ttattttggt taatatggtg 6600
aaattttgtt tttattaaaa aaataaataa aattaaattg tataaagtga ttaattatta 6660
atggtaattg ttaatgatag tttttttttt tttttttttt agatggagtt ttattttgtt 6720
atttaggttg gagtatagtg gtatgatttt ggtttattgt aatttttatt tttcgggttt 6780

aagtgatttt tttgttttag ttttttaagt agttgggatt ataggtgcgt gttattacgt 6840
tcggttaatt tttgtatttt tagtagagat ggggttttat tgtggtagtt aggatggttt 6900
cgattttttg attttgtgat ttgtttgttt cggtttttta aagttttggg attataggtg 6960
taagttatta tattcggtta ataatagtta ttttttttaa agtatttatt ttagttaatt 7020
agattttggg ttttgtattt tattgtattt atgtgttatt tatttttatg ataatgttat 7080
gaaggtagtg gtttttattg ttgttttttg agatagggtt ttatttgttg tttaggttgg 7140
agtgtaatgg cgtgattatg gggtttaggt gattttttta ttttagtttt ttaagtagtt 7200
aggattatag gtgtatatta cgatgtttag ttaatttttg tattttttgt agagataggg 7260
ttttattatg ttgtttaggt tggttttaaa tttttgggtt taagtgattt gttcgttttg 7320
gttttttaaa gtgttgagat tataggtgtg aattattata tgtggtcgaa agtaattttt 7380
tttatttttt tgagatggag tttcgttttt ttatttagtt tggagtgtag tggcgtaatt 7440
ttagtttatt gtaattttcg ttttttgggt ttaaggggtt tttttgtttt agttttttga 7500
gtagttggga ttatagatgt gtattattac gtttagttaa tttttgtatt tttagtagag 7560
atggggtttt attatgttgg ttaggttggt tttaattttt gattttaggt gatttataaa 7620
aaagtatttt gtaagtttag agagtgaata aatgaacgag ttgaataatt ataatgtttt 7680
tgtatgtggt ttggtttttt ttagtgttgg gattataggt attagtcgtc gtgtttagtt 7740
cgaaggtaat ttttgatatt cgtttttttt tttttttttt ttttgagata gagttttgtt 7800
ttgttattta ggttagagtg aagagtgtag tggtttaatt ttagtttatt ataatttttg 7860
ttttttagtt taagtgattt ttttgtttta gttttttaag tagttgggat tatcgatgcg 7920
tattaagacg ttcggttaat tttttgtatt tttgtggaga cgggggtttt attatgttgg 7980
ttaggttggt ttcgaatttt tgattttaag tgatttgttt atttcggttt tttaaagtgt 8040
taggattata ggtatgagtt attatgtttg gtttaatatt tttattttaa agataaataa 8100
atttaggttt agaaaggtta aattattttt taaatttaat tttggtttta atatatttta 8160
tatttaaaaa gtttaatttg gtttttagaa atattagaag tgatagggta ttggggaaag 8220
ttatagtatt ttttttagtt aacgtttaat ttagtgggtt gggattttgg ggatatggag 8280
gagtttcgag gttagggtta ttgtttgagg aatagggaat ttttagaaat gggttgggag 8340
tagggaaagg ggattttgag tttggttaga aggttttttt tagagaggag tttagggttt 8400
ttgtttggtt tttattataa aggtgattaa ttattttggt ttttttt 8447

<210> 111
<211> 8513
<212> DNA
<213> Artificial Sequence

<220>
<223> chemically treated genomic DNA (Homo sapiens)

<400> 111
   tagatggtga aatttgagtt gatggggagg aggattttta ttttttgtag gggtttgatg 60
   ggtagtatag ttggttaatt ttgggattta gagggtaggt cggttggttg attattaggt 120
   ttggaagatt taggtagttg gttttaaaga ggttttaggt tagtagttag atatgagttg 180
   tgagggttaa gtatagttat ttattagatg atttattttt agtttttttg agttttagat 240
   aatagaagat aggtggttgt atttttggtt aagggtaggt gtggtagtgg tgtttgttgt 300
   tattgtgttt ttattggttt ttagtaatta gatttaatag acggagtaat tgttattcga 360
   ggtttttgaa ttagggttat ttattaggag tatgcggttt tttgatgttt agttttggtt 420
   ggtgttgttg tgggtattgg tgcgagtata ggggataggg tttgtgtgtt ttttttgtgg 480
   gggttttaaa ttggtatttt aagtagaacg agttttggtg ttggagttag ttaagtagta 540
   aattttggat gggttgtatt tgattagtcg ttttagaata atttattttt tattttaggt 600
   agcgttgatt agagtttttc ggagattata gttagggagt gtggttttag ggaatgggga 660
   ggaggttatt agttttgtta ttgttatagg tgggtgaggg agagagtaat aggtaaagag 720
   tagataggga aagggaggta gaaggggagt ttggtaggag tagtagaggg agtggggtgt 780
   ggtaggagaa ggaggagttg gggtagggat tggttgtaga ggatataaag tagtttttat 840
   ttttttagag gtaggttcga gggagagtag tgggtagggt ttggggagtt ttagaggaga 900
   gttttatttt tatttatatt tttttttttt tttttgtttt tcgggtagat tttattttag 960
   tttatagttt tttgtttatt ttttatttgt ttatttttcg gttttattat ttgtattatg 1020
   ttcgtttgtg gttgtacgtg ttttttattt tttttggtat tttttgtttg aggatttttc 1080
   gatggggatt aaggaggagg cgttaagggt ttcgtatttt tttggttgag tattatatta 1140
   ttaatttggg ttggtatatt ttaattttgt tttttagtgg tttgaggggt gagaagtttg 1200
   gtgttttgaa attgtaatta gattgtagat ttttagaagg taatagtata gttattggat 1260
   aatcgaggcg gtttttggat atagtaggat attagtagtt ttttttagag tttaagattc 1320
   gagttaatga gtttggagta ggtcgggtta ggaggaggat ttttatttgt gagtttgcga 1380
   tttttttatg ttgtaggcga gattattacg tagattttta ggaattggga tggcgggatt 1440
   ggatattgta gttcgagggg tattagttga attattgtag tgggtagtgt tttttttatt 1500
   tggttggtag tttaggtatt gttgtttttt tttattttgt cgtttttagt ttttttaaag 1560
   ttaataattt ttggtttgtt agtatttttt gttgtgtttt tattgttcga aggttttttt 1620
   ttttttttta tttggattat aatggtaatg tggttaagac ggatgtgtta gatatggtgg 1680
   tggaggtttg tggttgtagt tagtaagagg atttggggtt ttggagtgaa gagattaaga 1740
   tgaagttttt taggtatagg gtatttgtga ttggaggtat tagatttttg atttatattt 1800
   taatttaata attatttggt aatatgattt atttgatttt tatgggattt aaatgggtat 1860
   tttttgtttt gagattttgg tttattttag gttggttgat gtgttgggag atgggtaaag 1920
   cgtttttttt aaaggggttt atttagaaag tatgattttt tgttttaagt tttgtgagaa 1980
   gatgttaggg attagggagg gagggaggga aggtagagaa aaattattta gtttttttta 2040
   agatgagaaa gtttttaagt gaggggagga ggaagtagat agatggttta gtaggtttga 2100
   agtagggtaa gtaggttggt ttagggtaag ggttgttgag gtattttaag ggaaggttaa 2160
   gagggagatg ggtaaggcgt tgagggagga tgtttagggg atttttagaa ataggagtta 2220
   ggaaaatgag gtattaagtt taagaagttt tttggttttt tttaggggat aggatttatt 2280
   gggagataag tatttatatt tttttttttt ttttttattt ttttgagatc gagtttcgtt 2340
   ttgttattag gttggagtgt agtgatacga ttttggttta ttgtaatttt cgttttttgg 2400
   gtttaagtga tttttttgtt ttagtttttc gagtagttgg gattataggc gtttattaat 2460
   ttttgtattt ttagtagaaa cgaggtttta atatgttggt taggatggtt ttaatttttt 2520
   gattttttga tttattcgat ttggtttttc gaagtgatga gattataggc gtgagttatc 2580
   gcgtttggtt tatatttttt taataaaaag gagaaagaaa attaataaat gtgagttata 2640
   aagaagggtt agggtgatgg tttagagtaa tagtttttta agtgtatttt gtaggttttt 2700
   gggaggtttt tttttagggg tgtttataaa gttaaagtta tttttataat aatattaata 2760
   tgttatttgt tttttgaatt tttattattt taaaattgta ttgtggagtt ttttagaggt 2820
   cgtgtgatat gtgattatat tatttttttg atattattgt taatggaatg tgtgtttgta 2880
   tggttttgtg ttatagtttt ttttagtttt aatttttaat gtggtgaatt gatagatgta 2940
   aattatatga ataaaagttt aatgattttt ttttttttga gacggagttt cgttttgtcg 3000
   tttaggttgg agtgtagtgg tataatttcg gtttattgta atttttattt tttaggttta 3060
   agcgattttt ttgttttagt tttttgagta gttgggatta taggtttgcg ttattatgtt 3120
   tagttatttt ttgtattttt agtagagatg gggtttattt atgttggtta ggttggtttc 3180
   ggatttttga tttgaggtga ttcgtttgtt ttggtttttt aaagtgttgg gattataggt 3240
   ttgagttatt gtgtttaatt taatgatttt taagagtata gaggaaatta aaaagtttga 3300
   gatttattgg tttatagaat tgggtgggga gaagaaagta aggtgtttta agagagtttt 3360
   ttttgttggg tatcggtggt tttagttatt taggaggtta aggtcggaag atcgtttaaa 3420
   tttaggagtt cgaggttatg attatatttg tgaatagttt ttgtatttta gtttgggtaa 3480
   atagtgaggt ttcgttttaa ataaaaataa ataaatatat aaatatataa atagtttttt 3540
   tggaagaagg ggttcgaggt tgggatagga gtatgtgtgg ggtgtttttt ttttagtgtt 3600
   tattagtttg gtttgattga gttgggtttt tgattttttt ggggataatt agtttgggtt 3660
   aaagttttag attttttttt atttttattt tttttttttt tttttgattt ttagattgaa 3720
   tagttaattt attgattttt tatatttaag ggggtggttt ttggaagtag agttaggatg 3780
   tgggaggttt gtttgtgggg ttgaaaaaaa ggggagaggg tgtttttttt ttttgttatg 3840
   tttttttttt ttttttataa aaattagaga ttgaaatgtt gttttttttt tatattcggt 3900
   tacgatggta atgtaaattt aaagaggtag ggtatttttt tgttaggtag tatcgttggg 3960
   tatagtaatt tttgtttttt cgttttttag agtttatata tttatttttt gggtttttaa 4020
   gtgggtttta gtgaggggtt ttttttttaa ttgggttttt tttttagttt tttagttttt 4080
   ttgtttcgat ttcgagcggg tgttatgtgt gagaacggtt agtagaggga gtagaaagtt 4140
   tggaagagta gttagagttt gtagtgacgt ggtgcggagg ggcggtattt tttagaattt 4200
   cgagacgtag agtcggggtt ttagggaaag gggttttagt tttagggttt tttggtgatt 4260
   tcgtgaatta tattttgtat ttagagtttt agtcgttgtt ttttgttttt atgttttata 4320
   gattttttat ttttttttag agtttttaat ttaatttgat ttgttttaaa tcgtaatttt 4380
   gtttcggtcg ttgtaagttt tatttaaagg gtgaggtttg tagataaatt ataggatggt 4440
   agaatgttta gttagtatta attaaaggcg attattttat ttttattatt atcgttttcg 4500
   gttgaatttt tttttttgtt tcgtaatatt ttttttaatt tggttgtcgg ggtttttttg 4560
   gggtagtcga tttagaaatt taagtcggga tttagtattt attaatagta gcgtgtttag 4620
   tcggggcgcg ggggggggcg taacgagtat agggtttttt aagggagggg gaggattttg 4680
   ggggttttgg gggtgtaata agttcggtat tttttttttt gtttttagtt atttcgtttt 4740
   attttttaga acggtaagag ggagggaaat agaagggagg tgaggggcga gcgggaagag 4800
   cggcggcgcg ttagcgttgg agagagaaaa agtttttgta aaagggaaaa aaaaagtttg 4860
   cgtttttcgc gggtggttcg ggtttgcggc gcggttgggt cggcgggagg gttgggggtt 4920
   aggttggggg ggtgggggtg gtatcgaggt tgcgttgtcg tggttttttt cgtttttttt 4980
   ttttatcgta tattttttag tttagatttt agttttggat cgcgtatttc gagtttagcg 5040
   tttagataga ggtgagaagg gggggtaggc gggggattat ttgggagtag tgggggaggg 5100
   ggtttgaggg gatgtttagt tttttaggga tttattttag attcgggata tagaggtaaa 5160
   ataggggtgg gagagtttgg ggtgagatat tagaaatttt agatttttta tttgtgtttt 5220
   tttttgtatt tttttttttt tttttttttt ttttttgtta gtttgtgtat ttttgtttta 5280
   gggaatcgtg ggtttttgtt ttcgtttttt ttatatatta gaaatatttt atttttatgc 5340
   ggttaagttt aagaggttgg agggatggtt agttggaggt ttgcgttgta gagaggtaat 5400
   tttaggtgtg tgtttgcgcg tggggtagga agatgttagt gtttttgaaa ggtggggatt 5460
   gtaaaggagg gagttttagg tggggtgggg acgggtgtgt gggaggtaat agagttatta 5520
   ggggttagtt aggtttgaat ttttgatttg ttttgtgata gatgtgttag tggatgtttg 5580
   tgttttaggg gaaaggagtg ttttttggat ttggaagggg gttggggcgg gggggggttg 5640
   tttaaggttt agtgaaggtt ttagaatgat tttatgtaat ttggattttt gagttttaag 5700
   ggttgtgggt aaggagttta ggaggagtcg ggagattttt gttttgattt tttgatttta 5760
   ggattatcgg ggtagcggga gttagtcgta ggagattttt atcggggttg ggcgggatta 5820
   ttggttattg ttagtttgtg tattttcgtt ggtatttcgt ttaaacggga gttggggatc 5880
   gaggtttttt ttttggttta gattattttg tttgtttttg tttttcgttt tgaatttttt 5940
   tttaggtttt atgatttcga aataattagt attgggtagt tagttttcgg gataggatta 6000
   cggaaagggg atttcgtaga gtttggggat tgagggtttt aggggtttgg agagttgggg 6060
   ttttttagga taggttttta cgttttagta gagggagaag gcgggcggtt tttcggggga 6120
   tatgtaaggg tcggaataag tgtggttttt tttagggggc ggggtttaga gatttttttt 6180
   ttttattatt tatttttttg gtattttcgt ttttgacgtt attagggggt tttcgggggt 6240
   ttggaggggt taatttttgg gaagtcggtt gttataatta gtaatttaag gtgttcgggt 6300
   tttttttttt ttttatatat atttaatttt attgatttag ttattttttt tgtttttttt 6360
   tttttatatt ttttggattt tttttttttt ttaattttat taacggggat ttttattttt 6420
   tagtagtgtt tttttgggcg ttattgtttg tttttcggat gtgttgagag gaggagggaa 6480
   gaatgtggtt cgttttttag tttttatttt tatggttcgt tttattgggt tttcggggcg 6540
   ggtgggggga gcggagtcgt ttgtagttag tgtttgcggc ggcgatttgg gtgggtcgag 6600
   gaggtacggg ggcggggaag gcgggatcgg gagtaggggg gcggagtcga tttttgggag 6660
   ttggggagcg ggggtgcgag ggggacgttg gaaaataggg agatatcgaa gatggtttag 6720
   ggtttcggta ggggaatttt agagtttttt gttttatttc gaatttttat attttagaaa 6780
   aattttatcg aagttaaaaa agttggcgat attttttttt tattagtttg aaggggagat 6840
   atgggtgggt tgtggaacgc gtgggcgagg aggggtgtag ttttattttt ttggaggttt 6900
   ttcggtagtt aggtagtggg gtagaaagat gattgagtag gtaagtttgg gggagttttt 6960
   agtgtttggg ggattatttg taaatttttt tttttttttt gggtgagtta tagagggaag 7020
   gaggcgggag aattgttttt ttatttagtt gttaattagt tgtgtttttc gttttttata 7080
   ttagttttaa ggtcgagttg ggaggttttg gatgcgggat tcgtatttcg gttggaaaag 7140
   gaggagttag aaaagggtat agtttaggtt gtcgggtcgg gttgggtcgc ggggttcggg 7200
   tttcgttttt tattttattt cgtttttatt ttaaatttta gtatttcggg attatttatc 7260
   gcgtcggtcg gttcggttcg ttttcggtgg ttttttattt tatttcgttt tattttattt 7320
   ttattttcgg gagattggaa aattcgggat ggagttaaaa ttggagtttg aaaagaaaaa 7380
   cggatagtag tatttttttt ttcgttacgt ttttataagg attatattgt tttttttatt 7440
   tttttagagt ttttttttat attttttttt ttgttttttt aaaatagaga ttaaaaagat 7500
   taggtgggtt tgatttaaat tattttagaa taatattagt ttttattttt gtgtttaata 7560
   gatttagaag atttttttta ggaattttat tttttttttt atattgtttt ttgtttaaga 7620
   gggtagatgg tttgtgtttt tttttggttt tatttggttt tgtgtcggga ttgtggttag 7680
   ggtttttttt gttggatatt gtagagattg tatacgggtt gtttttgggg aggggtacga 7740
   gaaggaaggg ggaacgggtt ttagtagagt cgtttagtgt ttgaagttag atttattata 7800
   cgagtttttt tattttattt ttaaagtcgg tttagggtta tagtaagaag aattagaggg 7860
   tttatgttat taaaattatt tgaagttaat ataggtataa ttagtttaaa atatgttttt 7920
   tatatataga ttatataggt aaagttttta tttaggtaaa gtttttattt agtttttaag 7980
   aagattttta gagtatatag attataagat tgtttttgtt tttttgggat attatttttt 8040
   tttatttttt tttttattta gcgagtgatg ttttttttgt tttgagacgg agtttagttt 8100
   tgttatttag gttggagtgt agtggtatta tttcggttta ttgaaatttt cgttttttag 8160
   gtttaagcga tttttttgtt ttagtttttc gagtagttgg gattataggt attcgttatt 8220
   atgattggtt aatttttgtt tttttgtaga gacgggggtt ttattatgtt ggttaggttg 8280
   gtttttaatt gattttaggt gattttttcg ttttagtttt ttaaagcgtt ggaattatag 8340
   gcgtgagtta ttgtgttcgg tttagtgatg ttttttttaa ttcgaatttc gtggtagatg 8400
   ttttagaggg gtgggggata ttagggatgt tttgtttagg attttgtgtt tgagattgtt 8460
   gtttgatagt ttttattttt tttattttta tatttatttt ttttttttgt agt 8513

<210> 112
<211> 8513
<212> DNA
<213> Artificial Sequence

<220>
<223> chemically treated genomic DNA (Homo sapiens)

<400> 112

attgtagaaa gggaaggtag gtataaaggt ggaggaaata gagattgtta gatagtagtt 60
ttaggtatag aattttgggt agaatatttt tggtattttt tattttttta agatatttgt 120
tacggaattc gagttgaaaa gagtattatt gagtcgggta tagtggttta cgtttgtaat 180
tttaacgttt tgagaggttg aggcgggagg attatttgag gttagttgaa gattagtttg 240
gttaatatgg tgaaattttc gtttttataa aaaaataaaa attagttagt tatgatggcg 300
ggtgtttgta atttagttta ttcggaaggt tgaggtagaa gaatcgtttg aatttgggag 360

gcggaggttt tagtgagtcg agatggtgtt attgtatttt agtttgggtg atagagttag 420
atttcgtttt aaaataaaaa aagtattatt cgttgagtga ggggaggggt aaggggaaat 480
gatgttttag agaggtagag gtagttttgt agtttgtgta ttttggggat ttttttggga 540
attgggtggg agttttgttt gggtgggagt tttgtttgtg tggtttgtgt gtagaaggta 600
tgttttggat taattgtgtt tatattaatt ttaagtgatt ttagtaatat ggatttttta 660
attttttttg ttgtaatttt gggtcggttt tgagggtggg atgggagaat tcgtgtgatg 720
gatttgattt taagtattgg gcggttttgt tagagttcgt tttttttttt ttttcgtgtt 780
tttttttagg gatagttcgt gtataatttt tgtagtgttt agtagaggga gttttagtta 840
tagtttcgat atagagttag gtggggttag gagggagtat aagttatttg tttttttaga 900
tagaaaatag tgtggaggag gaggtaaggt tttgaggggg agtttttttgg gtttgttggg 960
tataaaggtg aaagttggtg ttattttggg gtagtttaaa ttaggtttat ttaatttttt 1020
tggtttttgt tttgggggaa taggaaagga agtatgaggg gagattttga gagaatgaaa 1080
aaaataatgt gatttttata aaagcgtagc gaggaagaag gtgttgttgt tcgttttttt 1140
ttttaaattt tagttttgat tttatttcgg gtttttagt ttttcgggaa tgaggtgggg 1200
gtggggcggg atggggtgga gaattatcgg gagcgggtcg ggtcggtcgg cgcggtgggt 1260
gatttcggga tgttgggatt tagggtgagg gcggggtgag atggggagcg gaattcggat 1320
ttcgcgattt agttcggttc ggtagtttgg gttgtatttt tttttaattt tttttttttt 1380
aatcgaggtg cggatttcgt atttaagatt ttttaattcg attttggaat taatgtgggg 1440
ggcggggggt atagttggtt ggtagttgga tggggggata attttttcgt tttttttttt 1500
ttatgattta tttaggggaa ggagggaagt ttgtaaatga ttttttaaat attagagatt 1560
tttttaaatt tgtttattta gttatttttt tgttttattg tttagttgtc gaagagtttt 1620
taaggaaatg gggttatatt ttttttcgtt tacgcgtttt atagtttatt tatatttttt 1680
ttttaggtta gtaaaaggaa aatgtcgtta atttttttag tttcggtaaa gtttttttgg 1740
ggtgtgagag ttcgggatgg gatagggagt tttagggttt ttttgtcgga gttttaaatt 1800
attttcggtg tttttttgtt ttttagcgtt tttttcgtat tttcgttttt taatttttag 1860
gagtcggttt cgttttttta ttttcggttt cgtttttttc gttttcgtgt tttttcggtt 1920
tatttaagtc gtcgtcgtag gtattggttg tagacggttt cgtttttttt attcgtttcg 1980
ggagtttagt agagcgagtt ataggggtgg gggttgaggg gcgggttata tttttttttt 2040
ttttttttag tatattcgga aagtaggtag tggcgtttag gggggtatta ttggagagtg 2100
ggggttttcg ttggtggggt tagaaaggga gaagaattta gaaagtatag gggagagaaa 2160
gtagagaaag tgattgggtt aataaaatta ggtgtgtatg ggaggaggga ggggttcgga 2220
tattttaggt tgttggttat agtagtcggt tttttaaggg ttaatttttt tagattttcg 2280
ggaatttttt ggtgacgtta agggcgggga tgttagggag ataatggtgg agggggagag 2340
gtttttggat ttcgtttttt agggaaaatt atatttattt cgatttttat atatttttcg 2400
ggaaatcgtt cgtttttttt ttttattgga acgtaaagat ttattttaga agattttagt 2460
tttttagatt tttaaaattt ttagttttta ggttttacgg ggtttttttt tcgtaatttt 2520
gtttcgaaag ttagttgttt agtgttgatt gtttcggggt tatgggattt gaaagaggat 2580
ttagagcggg gagtaagggt aggtagggtg gtttgagtta gaggaggggt ttcgattttt 2640
agttttcgtt tgggcggggt gttaacgggg atatataggt tggtagtggt tagtggtttc 2700
gtttagtttc ggtaggggtt ttttgcggtt ggttttcgtt gtttcggtgg ttttgaggtt 2760
agagggttaa gataaggttt tttcggtttt ttttgagttt tttgtttata gtttttggga 2820
tttaggagtt taaattgtat ggggttattt tagggttttt attagatttt ggatagtttt 2880
ttttcgtttt agtttttttt taagtttaga agatattttt tttttttaaa gtataagtat 2940
ttattggtat atttgttata agataggtta aaggtttaag tttggttggt ttttagtggt 3000
tttgttgttt tttatatatt cgtttttatt ttatttggag tttttttttt tgtagttttt 3060
attttttaga aatattgata tttttttatt ttacgcgtag atatatattt ggggttattt 3120
ttttataacg tagattttta gttagttatt tttttagttt tttaaattta atcgtatgaa 3180
agtaagatat ttttaatata tgggagaggc ggagataaag atttacggtt ttttgagata 3240
gagatatata gattgataga agaaaaaaag ggaagaaaaa gaagatatag agaaagatat 3300
aagtgaaaaa tttggagttt ttaatgtttt attttaggtt tttttatttt tattttgttt 3360
ttatgtttcg ggtttgggat gagtttttaa gaagttgagt atttttttag gttttttttt 3420
ttattgtttt taggtggttt ttcgtttgtt tttttttttt atttttgttt gggcgttggg 3480
ttcgggatgc gcggtttagg gttggagttt gggttggggg gtgtgcggtg gggagggggg 3540
gcggagaggg ttacggtagc gtagtttcga tgttattttt atttttttaa tttggttttt 3600
agttttttcg tcggtttagt cgcgtcgtaa gttcggatta ttcgcgagaa gcgtaaattt 3660
tttttttttt ttttgtaaaa attttttttt tttttagcgt tggcgcgtcg tcgttttttt 3720
cgttcgtttt ttattttttt tttatttttt ttttttttgt cgttttggag gatgaggcgg 3780
ggtagttgga agtaagagaa ggggtgtcgg gtttattgta tttttaggat ttttaggatt 3840
tttttttttt tttgagggat tttatattcg ttacgttttt tttcgcgttt cggttgaata 3900
cgttgttgtt ggtgagtatt aaatttcggt ttggattttt ggatcggttg ttttaaagag 3960
gtttcgataa ttagattgag gaaaatattg cggggtaggg ggagaagttt aatcgagaac 4020
gataatagtg gaggtagggt agtcgttttt ggttggtgtt aattgagtat tttgttattt 4080
tgtggtttat ttgtaggttt tattttttgg atggaatttg tagcggtcgg gatagagttg 4140
cggtttgggg taaattaagt tgggttgggg gttttggaag aaggtgagga gtttatggag 4200
tataaaagta aggagtagcg gttgaggttt tgggtgtagg gtgtggttta cgaggttatt 4260
aaggagtttt gggattgaag tttttttttt tagaatttcg gttttacgtt tcgaagtttt 4320

ggagggtgtc gttttttcgt attacgttat tgtaggtttt agttgttttt ttaggttttt 4380
tgtttttttt gttggtcgtt tttatatatg atattcgttc ggagtcgaag tagaagagtt 4440
ggggaattga aggaggagtt tagttgaagg aggagttttt tattaaagtt tatttaaaag 4500
tttaggaggt ggatatgtga gttttgagaa acggagaggt agaggttgtt atgtttagcg 4560
gtattgtttg atagggaagt gttttgtttt tttagatttg tattgttatc gtgatcggat 4620
ataagggagg ggtagtattt tagtttttga tttttatgag aaagaggagg aagtatgata 4680
aggaaaaagg gtattttttt tttttttttt taattttata ggtagatttt ttatatttta 4740
gttttgtttt taagaattat ttttttgggt gtggaaggtt agtgggttaa ttgtttagtt 4800
tgagggttaa gggggaaaag aaaaggtgaa ggtaggggga gatttgggat tttgatttag 4860
gttagttatt tttagagggg ttagagattt agtttagtta gattagatta atgggtattg 4920
aaaaaaaggt atttatatat tgtttttgtt ttagtttcga gttttttttt ttagaggagt 4980
tatttatgta tttatgtatt tatttatttt tatttgggac ggggttttat tatttgttta 5040
ggttggagtg tagaggttat ttataagtgt gattatagtt tcgaattttt gggtttaagc 5100
ggtttttcgg ttttagtttt ttaagtagtt gggattatcg gtgtttagta agaagagttt 5160
ttttagaata ttttattttt ttttttttat ttagttttat agattagtga gttttaaatt 5220
ttttggtttt ttttatattt ttaaaagtta ttaggttagg tatagtggtt tagatttgta 5280
attttagtat tttgggaggt taaggtaggc ggattatttt aagttaggag ttcgagatta 5340
gtttggttaa tatggtgaaa ttttattttt attaaaaata taaaaaatag ttgggtatgg 5400
tggcgtaggt ttgtagtttt agttatttag gaggttgagg taggagaatc gtttgaattt 5460
gggaggtgga ggttgtagtg agtcgagatt gtgttattgt attttagttt gggcgataga 5520
gcgagatttc gttttaaaaa aaaaaaaatt attgagtttt tgtttatatg atttatattt 5580
attaatttat tatattagaa attaaaattg aaaaagattg taatataaga ttatataagt 5640
atatatttta ttaataatga tgttagaaag atgatgtaat tatatgttat acggtttttg 5700
gaaaatttta taatataatt ttaagataat gagaatttaa aaggtaaata atatgttagt 5760
attattatga aaatagtttt gattttgtgg atatttttga aaagggattt tttagaagtt 5820
tatagagtat atttgaagaa ttgttgtttt ggattattat tttaattttt ttttatgatt 5880
tatatttgtt gatttttttt ttttttttta ttaagaaagt ataagttagg cgcggtggtt 5940
tacgtttata attttattat ttcgggaggt taagtcgggt ggattaagag gttaagagat 6000
tgagattatt ttggttaata tgttgaaatt tcgtttttat taagaatata aaaattagtg 6060
ggcgtttgta attttagttg ttcgggaggt tgaggtagaa gaattatttg aatttaggag 6120
acggaggttg tagtgagtta agatcgtgtt attgtatttt agtttggtga tagagcgaga 6180
ttcgatttta aaaaaataaa aaagaagaaa gaaagtataa atgtttgttt tttagtgggt 6240
tttgtttttt gggaaaaatt agggaatttt ttaggtttag tgttttattt ttttgatttt 6300
tgtttttggg ggttttttaa gtattttttt ttagcgtttt gtttattttt tttttgattt 6360
ttttttaagg tattttaata gtttttattt tgggttagtt tgtttatttt gttttaagtt 6420
tgttggatta tttatttgtt tttttttttt tttatttgag gattttttta ttttgggaga 6480
ggttaagtaa tttttttttg tttttttttt tttttttttt agtttttgat atttttttat 6540
aggatttagg gtaggaaatt atgttttttg ggtagatttt tttagaagaa acgttttatt 6600
tattttttaa tatattagtt aatttggaat aagttagagt tttagataag aaagtgttta 6660
tttgggtttt ataggggtta agtgagttat attgttaggt ggttgttggg ttggggtgtg 6720
gattaggaat ttgatgtttt tagttataga tgttttgtgt ttgggaaatt ttattttggt 6780
ttttttattt taaagtttta ggtttttttg ttagttgtag ttataggttt ttattattat 6840
atttggtata ttcgttttga ttatattgtt attatgattt aggtagagga gagagagggg 6900
ttttcgggta gtagggatat aataggaggt attggtaggt taaggattgt tggttttgag 6960
gaggttgaag acggtagaat ggaaagaggt agtaatgttt gggttgttag ttaggtgggg 7020
agggtattgt ttattgtagt aatttagttg gtatttttcg ggttgtagta tttagtttcg 7080
ttattttagt ttttggaagt ttacgtaatg gtttcgtttg taatataagg gggtcgtagg 7140
tttataggtg ggggtttttt ttttggttcg gtttgtttta ggtttattgg ttcggatttt 7200
aagttttagg aagggttgtt ggtgttttgt tgtgtttaag agtcgtttcg gttgtttagt 7260
aattgtgttg ttgtttttta ggggtttgta gtttagttgt agttttagga tattagattt 7320
tttatttttt aagttattag agggtagagt taaggtatgt tagtttaggt tggtgatgtg 7380
gtgtttagtt aggagagtgc gggatttttg gcgttttttt tttggttttt atcggaagat 7440
ttttaagtaa agagtgttag gaagggtggg gagtacgtgt agttataggc gggtatggta 7500
taggtggtgg gatcgaggag tggataggtg aaaagtgagt agggagttgt aggttgaagt 7560
ggagtttgtt cgaaagatag aaaagggaaa gaaaatgtga gtagagatga agtttttttt 7620
tgagattttt taagttttgt ttattgtttt ttttcgaatt tatttttaga aaagtagaga 7680
ttgttttgtg ttttttgtaa ttagtttttg ttttagtttt tttttttttt gttatatttt 7740
attttttttg ttgtttttgt taggtttttt ttttgttttt ttttttttgt ttgttttttg 7800
tttgttgttt ttttttttat ttatttgtga tagtagtaaa gttgatgatt ttttttttat 7860
tttttggagt tatatttttt ggttgtagtt ttcggagggt tttggttagc gttgtttggg 7920
gtggaggatg agttattttg ggacgattgg ttaggtgtaa tttatttagg atttgttgtt 7980
tggttagttt tagtattaga gttcgttttg tttggggtgt tagtttggag tttttatagg 8040
agggatatat agattttgtt ttttgtgttc gtattagtgt ttatagtagt attagttaga 8100
gttggatatt agggagtcgt atgtttttgg tgaatggttt tggtttagga gtttcggatg 8160
gtagttgttt cgtttgttga gtttgattgt tgggggttaa tgagggtata gtgatagtag 8220
atattattgt tatatttatt tttggttaag ggtatagtta tttgtttttt attgtttggg 8280

atttaggaag gttgaaagta gattatttga tggatagttg tgtttgattt ttatagttta 8340
tgtttggtta ttgatttggg gtttttttag agttagttgt ttgggttttt taaatttagt 8400
ggttagttag tcgatttatt ttttgagttt taggattggt tagttgtgtt gtttattagg 8460
tttttgtagg gggtggaggt tttttttttt attagtttag gttttattat ttg 8513

<210> 113
<211> 6169
<212> DNA
<213> Artificial Sequence

<220>
<223> chemically treated genomic DNA (Homo sapiens)

<400> 113

ggtttgggag tatttttttt gtttttagat atttagagtc gtttttgagt aatttagtat 60
gttttgtttg ttggcgcgtt gattggggcg cgtcggggtc gggagaattc ggcgtggatt 120
ttttcgcgtt gatgtaatga tgatggatgc ggcgacgcgg ttgtagatag cgttgcgcgg 180
gttcggcgcg ttcgttaatg aatttatcgc gtcgtcgtcg cggtttggga tttcgggtag 240
gggacgtagg cgcgggtaaa agtggtgaaa ggcgtgaaaa atattttttg ttatcgtcgg 300
aggaaattag aatttgaagg gaattcgata ggaaagcgta gagaaagagc gagtttttaa 360
ataaaattgg ttatattcgt ataatttgag taaaataatt attataatta atggggattc 420
gtttttgtaa tggtgaaagt tttgtttggg gtttttattg gtttttcgtt ttttgtaggt 480
ttaagtgacg atttaaaaga ggacgatttg aggcgtttgt gtgtaagtgt tgtttaatat 540
gtaggtttcg gtaagaattc gttaaggtgg taaaggttgc ggtttttttg ttcgtttttt 600
gtttttttgt ttatttttaa atgtagacgt gtattatagc gttattaggt tagtggtttt 660
aggaagtttt aaatgttttg tgttgtaaga ggttgacgta ggtttagatt tataatttag 720
aaagaaaaga tttatttgta ggtaggttgt aaataggaga cggattagat ttaaaatagg 780
gtttggaatt aattgtgttt tgggtttagg tttcggggaa attataaaag gtttagggat 840
agttgagtgg gaaaaagttt tgattgtttg tttttaggaa ggaatgttta tttttttttt 900
tatttatgaa attttaagtt ttaggaagga tatattatta gagtaatttt ttttgcgttg 960
tttttttttt ttttttttat cgagtttttg ggatttaaat attagttata aatatttttt 1020
tttttaagta tgaaaagtta aggggtatgt tagtaaattt tgtatgtaat gttttagtgg 1080
tgaaagggat tgaaggagaa tagtgtggag tcgtaagtaa ggaaattatg tcgggggaaa 1140
aagtattatt ttatttatat ttgaaaaata gtggtatgtt ttttttaggg aaaagttttt 1200
gaagtgaagt agaatttaac gttttttttt gatgtttaag ttttaaataa attatatttt 1260
attatttttt aaattttagg ttttaaaagt gtcgtggagt tattgtttta gtggtgtttt 1320
gagattttat gaggaatttt tattgattat ttttatttcg aatgtttgaa tggattagtt 1380
taggagtgat tttttaagtt tttgattatt ttttgaaaat tttattatta taatttaaag 1440
cggttatttt ttgtcgttta ttgatttttt atttatagtt tgatgaggtg tgggcgttta 1500
gaatgttaaa tttttttttt tataattgat agggataaag ttgtaaaata agttgatata 1560
atttatagga gtgtatagga agtagatgtt tgatttggta tattgttatt tttatagttt 1620
agttttaagt ataaattata tttttaaata attaggtatt aattaatgag atttaaaaat 1680
aagttgttaa aatagaaggg ggtagattgt tgagtgatta agggtttatt tttaaaaaaa 1740
tttattttag acgggggtag gaattagtat tttttaagat tattaattag tgtgatttta 1800
gttagtgttt ggagttttat ttttttttat ttgagttttt ttttatggtg atgagtattg 1860
ggagggtttt attgttttgt taaaataaag ttttttgttt tggggaaaaa attaataatt 1920
ttataaaaat taggtttgtt aaggaatttt gggaatttgt tttttgattt ttattaatta 1980
gtattttttg ttaaggaaga tatttagttt ttattaggaa ttttattttt gaatgggttt 2040
ttttttggtg tatttgtatg ttttagtgtg tagttttaac gttgtagatt ttatttaaat 2100
atagttaata aaattgttat gtgtgtgtcg aagggaataa ttatagtata gtaaattttt 2160
gtttatttta atggttaaga atggagtgtt tggattaatt aaatattttt gttaaagttt 2220
ttagttttta tagaatttgt tagtttttaa cgaaatattg aatatgaaaa gaaaatgtat 2280
gtagttaaat tagtggtgat tgaaatttaa gacgttgtag agttttgtaa gttatagggt 2340
tattgatgcg attatttatg tatttagtaa atatttttac gtgaattaag tgttgggaaa 2400
gtagaagttt tgtttttatg gagtatatag tttggaaagg ggaggtggat gtttagtata 2460
gattttttta gatgaaggag agtttattat tgtgatgggt gttaggatag gtttgtgtag 2520
ggtgttagaa tttaggattt tagagtgtag tttatttatt agtttattag gttagtaaag 2580
attttaatga gtatatattg tattgggtat atagtagtga gtaaaaataa acgtgtagaa 2640
tagttgttta aggtttacgt ttgtattttt agtattttgg gaggtcgagg cgggtggatt 2700
atttgaggtt aggagttcga gattagtttg gttaatatga taaaatttta tttttagtaa 2760
aaatataaaa aattagttgg gtatggtggt gggcgtttgt aattttagtt atttgggagg 2820
ttgaggtagg agaatcgttt gaagttggga ggtagaggtt gtagtgagtt gagattacgt 2880
tattgtattt tagtttgggt aatagagtga gattttgttt taaaagaaat aaaatcgaaa 2940
aaataaaaat aaataaataa aaaaataagt tttagtgaat ttgataatta gtaacgtttg 3000
ttaatattga ggtttcgggt gttatttttt aaggtaaatg tattgtagga atttgaatgt 3060

ttagtaagta ttgtagaaat attgatataa atggtttttg tattaagtga gaaataatga 3120
tgttaattgt attggattat ttttgtggta tattttaaag atgatattta aattaatttt 3180
taaggattat tataaaaagt aattattttg agattgttag agttagaagg gattattaaa 3240
gatttttttt ttaagggttt tttgtttaga tttttggaaa agtttggaag gtttgtgatt 3300
ttttggaaat taaatgtata tttttaagtt taagggtatt ttttggggag aagtttttta 3360
atttttaata tttttttaag atgttatttt ttttcgttta aagtaaaggt tagattttat 3420
tgttcgattt tgttatttga tagatgagat tattgaggtt tagtaatagg ttgttggcgg 3480
ttgtgtattg gttagttagt tttagggggc gttatgtagg tagttttgtt tttgggattt 3540
acgtacggtg gttgtattcg gaagggaaat tttaagtttt tttttttgtt gtataatatt 3600
ttgttagtgt tttttgaggg gtgtttttga atttggagtg gattttataa ttgatatgta 3660
aatttaattt agtgtttttt tttttttttt tattttataa aagttgtttt taaataattt 3720
gtgtgtttta taatgaatga tattggataa ttttagaaaa gagatggaag tgagtggaag 3780
agtcggtgtt atagtgtgtt atgtggtttt tggtttttaa gtattttagt tatttgaatt 3840
ttagattaag gaggaattta ttatataatg aagagtttgt agtttttttg taaatatgtg 3900
ttaaagtaga ttgaaaatat tttattttgt attttaattt ttagttggtt aagtagttgt 3960
ttattttatt ttattaatta ttgttgtgat tgaggatgtc gatatttatt aaatgtagaa 4020
aaataagtaa gatggttaga tttaagaaat gttttttatg aatgttgtaa tttagataat 4080
ttgaagaaaa tatgttagag agggtatttt tttttttttt ttgtattttt tgtaggtgta 4140
gaatatgatt gtaaaaagga atatgaatta gagtgaaaat tgatgaatga tgagttaggg 4200
cgattattat atgaatgaag ttagtgaaag atagatttat tgaatttgtt tttagttgag 4260
ttttgatatt ttttaagtga gtttagtttt tgttaaggta ttttttaatt tttttatgtg 4320
gaattaagag cgtgttgttt tattattttt gttttaaaag tgtaatttaa atgaattgaa 4380
atatttttat gttttaaaga ggttaaagag tatagtttat tagaatgaat taggattaag 4440
aggtatattg taatttagat atttttatga aagaggtaaa aattattgat tatttggttt 4500
gaattatttt ttgatttaga atttggagta ggttttcgat gttagaaatt gtcggatttt 4560
ttttaaaata tttaattaat aattaaaaat tttttttttt tttggaattt gtttaaatgt 4620
ttttttttgt aaagttggtt atttgaagag atatttgtag acgtggtttt aaaaagtaat 4680
gtgatatttg ttttatatga tagatatagt tgtaaattga gatttttata tcgtttgata 4740
ttaaacgttt agtagggatt tgggaaaagt attatgttat gggtattttt ttcggttttt 4800
ttttttgttt gtgtagagtt ttattggttt gttaaatttt gagatttttt tttttgttag 4860
aaaatttgtg gagaagtgtt atttatattt tgaatttttt aaagttttag gtttttttta 4920
gatgataggt gaagtggatt tttgaaagtt tttttttttt ttttattatt ttggaaagtt 4980
gatggttttg ttgttttttt tagggtattt ttggtcgaag agagttgaag taatgagaag 5040
atattatgga ggtttagttt tatagtttta cgattattga aaagaaaaaa gttgagaatt 5100
ttatagtgaa gtgttttatt cgaatagatg tgagcgagaa agtcgttgtt tttagtatta 5160
tttttaatgg tgagtagtat agggattatt agtagtttag tttggagtta tttttgtgtt 5220
ttttgtattt tttgtgaatt gagcgtttgt atttggatta gtgtttgttt taggagacgt 5280
taaagaaagt aaatggagta ggggcggggg gacggcgggg ggatatggaa tgtagtaatt 5340
tttattttgg tatttaattt atacgttgat ttttaaattt tttaatttga gatgtaaatg 5400
tgataggtta tttattatta gaagtttttg ttttggtatt ttggaaatta ttttgattag 5460
tttggtaaga gtttaaaatg ttttgtgttg tatatggatt atttatatta ttcggaataa 5520
ttagtataaa agatataaaa gttgttttgt taaaattagt gataggatag tagttttttt 5580
taattttaat tttaagtagt ttattttaaa ggattttaag aattgttaat gattgaattt 5640
taaggtaatg ttagtatttg agatgtaggt tttgttgtta gtttataaaa atttaattta 5700
tttatttgtg tttagagata atatagagtt attgtttttg tattttgtgt ttttaaagtt 5760
taagtaaagt gttgagaggt attttagttt aagtatggag tgttgaaaat aaattttaga 5820
gtatttttgg tataaataga ttgaatatgt taatggtaat ttttattttt tgtttttgtt 5880
gtttttttat tgtgggttat taatgttttt tttttttgta gattttatag ttatttttgg 5940
tttttttgaa tttttttgtt tttttatggg tttttaggaa gaatgagttg gttcgttttt 6000
ttcgtttttg gatattgttt atttgggtat attgatttgt atgagttaat ttgttatttt 6060
tatatttttt atattttaag ataggtttta tttgattgat ttataagttt ttatatttat 6120
ttagagagtt agaatttttt ttggaaggta attgtagttt agaatatta 6169

<210> 114
<211> 6169
<212> DNA
<213> Artificial Sequence

<220>
<223> chemically treated genomic DNA (Homo sapiens)

<400> 114

taatgttttg gattgtagtt attttttaaa ggagattttg attttttagg tggatgtaaa 60
aatttgtaaa ttaattaagt aaaatttgtt ttggaatata gaaaatataa gaatagtagg 120
ttgatttata taaattagtg tgtttagata ggtagtgttt aggaacgagg aaaacgggtt 180

agtttatttt ttttaaaggt ttatggaaaa gtagagaaat ttagggaggt taagaatggt 240
tatgaaattt ataaaaagga aaggtattag tggtttataa tggagaaata gtagggatag 300
gaagtgaagg ttattattaa tatatttagt ttgtttgtat tagggatgtt ttggaattta 360
tttttaatat tttatgtttg aattgaaatg ttttttaata ttttgtttaa attttaaaaa 420
tatagaatat agaaatagtg gttttgtgtt atttttgaat ataaataagt aaattaaatt 480
tttataagtt aataataaaa tttgtatttt agatattaat attattttga ggtttagtta 540
ttaataattt ttaaaatttt ttaaagtaga ttatttaaag ttggggttgg aggagattgt 600
tgttttgtta ttaattttga taagataatt tttgtgtttt ttgtattaat tatttcgagt 660
agtgtagatg atttatatgt aatataaagt attttgaatt tttgttagat tagttaagat 720
gatttttaaa atgttaaaat aggaattttt gataataaat aatttattat atttatattt 780
taaattagaa agtttggaag ttaacgtgta ggttgagtgt taaaatagag attattatat 840
tttatgtttt ttcgtcgttt tttcgttttt gtttatttgg ttttttttag cgttttttag 900
aataaatatt ggtttaggtg taaacgttta atttataagg aatgtagaga atatagaaat 960
gattttaggt tgggttgttg gtaatttttg tgttgtttat tattagaagt ggtgttggag 1020
gtaacggttt tttcgtttat atttgttcga gtggagtatt ttattatgga atttttaatt 1080
tttttttttt tagtggtcgt ggagttgtgg gattgggttt ttatgatgtt tttttattat 1140
tttagttttt ttcgattaaa aatgttttaa agaaaataat agagttatta attttttaaa 1200
atggtggaaa aaaaaaaaaa atttttaaaa gtttatttta tttgttattt gagaaaagtt 1260
tagagtttta ggaaatttaa aatgtggatg atattttttt ataggttttt tggtaaaaaa 1320
aaaagtttta aaatttaata aattagtgaa attttatata agtaaaaaag agaatcgagg 1380
gaaatgttta taatatggta ttttttttaa atttttattg agcgtttggt attaaacggt 1440
ataaaaattt taatttgtaa ttgtgtttat tatgtgaaat aagtgttata ttatttttta 1500
aagttacgtt tgtaaatgtt tttttaggtg gttaatttta taggaagggg tatttgaata 1560
aattttaaag ggagggaagg tttttaatta ttaattagat gttttaaaga gagttcgata 1620
atttttaata tcgaaggttt attttaaatt ttgaattaga ggataattta ggttaagtag 1680
ttagtgattt ttgttttttt tataaagata tttgaattgt aatatatttt ttaattttaa 1740
tttattttgg taagttatat tttttaattt ttttaaagta tgagaatatt ttagtttatt 1800
taagttatat ttttaagata aaggtaatag aataatacgt ttttgatttt atatggaaaa 1860
gttgaaaggt gtttagtaaa agattgggtt tatttaggaa atattaaaat ttagttaaag 1920
gtaggtttag taggtttgtt ttttattggt tttatttatg tgataatcgt tttaatttat 1980
tatttattaa tttttatttt ggtttatgtt tttttttgta gttatgtttt atatttataa 2040
agaatataag aaaagaggaa aagtattttt tttagtatgt tttttttaag ttatttagat 2100
tgtaatattt atggaaagta ttttttaggt ttggttattt tgtttatttt tttatattta 2160
gtaaatgtcg atatttttaa ttatagtagt ggttagtgaa atgaaataag taattgtttg 2220
attaattaaa agttaaaata taagataaaa tatttttagt ttattttaat atatatttgt 2280
aagggaattg taagtttttt attgtataat aaattttttt ttgatttgga atttaagtaa 2340
ttagaatgtt taagaattag aggttatatg gtatattgta gtatcggttt ttttatttat 2400
ttttattttt tttttgagat tatttagtgt tatttattgt aaaatatata agttatttaa 2460
gaatagtttt tgtagaataa aaaagaaaaa agaagtatta agttaaattt atatattaat 2520
tgtaagattt attttaaatt tagagatatt ttttaaaggg tattgataaa gtattatata 2580
atagaaaagg gggtttgaga tttttttttc gagtgtagtt atcgtgcgtg agttttaagg 2640
gtagggttat ttgtatggcg ttttttgggg ttagttggtt agtgtataat cgttaataat 2700
ttgttgttga gttttagtga ttttatttgt taaatgatag aatcgggtag tgagatttga 2760
tttttatttt gggcgggagg aagtaatatt ttaagaaggt gttaaaagtt aaagagtttt 2820
tttttagaaa atatttttaa atttaaaagt atatatttaa tttttaaggg attatagatt 2880
ttttaagttt ttttaagaat ttggatagag aatttttaaa ggagaaattt ttgatggttt 2940
tttttagttt taataatttt agaataatta ttttttgtaa tggtttttga aaattgattt 3000
aaatattatt tttaaagtat gttataagaa taatttaata taattggtat tattgttttt 3060
tatttggtat aagaattatt tgtattagtg tttttatagt gtttgttaag tatttagatt 3120
tttgtaatgt atttgttttg gaggatggta ttcggaattt taatgttagt aagcgttatt 3180
aattgttaga tttattaaag tttgtttttt tgtttgtttg tttttgtttt ttcggttttg 3240
ttttttttga gatagagttt tattttgttg tttaggttgg agtgtaatgg cgtgatttta 3300
gtttattgta atttttgttt tttagtttta agcgattttt ttgttttagt tttttaagta 3360
gttgggatta taggcgttta ttattatgtt tagttaattt tttgtatttt tattagagat 3420
gaggttttgt tatgttggtt aggttggttt cgaatttttg attttaggtg atttattcgt 3480
ttcggttttt taaagtgttg ggagtatagg cgtgagtttt gaataattgt tttatacgtt 3540
tatttttgtt tattgttgta tatttagtgt agtatatatt tattgaagtt tttgttgatt 3600
tgatgaatta atgaatgaat tgtattttag aattttgaat tttaatattt tatataaatt 3660
tattttagta tttattataa tgataaattt ttttttattt ggaaaagttt gtgttgaata 3720
tttatttttt ttttttaggt tgtatgtttt atgaggataa ggtttttatt tttttagtat 3780
ttagtttacg taagaatgtt tattgagtat ataaatgatc gtattaatga ttttgtgatt 3840
tataggattt tgtagcgttt tgaattttaa ttattattgg tttagttata tatatttttt 3900
ttttatattt agtgtttcgt tgaaaattgg taaattttat aaaaattaaa aattttagta 3960
gaaatattta attgatttaa atattttatt tttagttatt gggataagta agaatttatt 4020
atgttataat tgtttttttc gatatatata taatagtttt attggttgta tttgagtaga 4080
gtttataacg ttaaaattgt atattgaaat atgtaaatgt attagaaaaa aatttattta 4140

gagatgggat ttttagtaag aattaagtat tttttttagt agagggtgtt aattaatagg 4200
aattaggaga taagttttta aaattttttg ataaatttgg tttttgtgga gttgttggtt 4260
ttttttttaa ggtaaaggat tttattttaa taaagtaata aaattttttt aatgtttatt 4320
attatagaga gaggtttaag tgagaagagg taaagtttta ggtattgatt gaaattatat 4380
taattaatgg ttttaaagaa tattaatttt tattttcgtt tgaaatggat ttttttaaaa 4440
ataggttttt ggttatttaa taatttattt ttttttattt tgataattta tttttggatt 4500
ttattagtta gtatttaatt atttaaaagt gtggtttatg tttggaattg ggttataaag 4560
atggtaatgt gttaaattag atatttgttt tttgtatatt tttataaatt atattagttt 4620
attttataat tttgtttttg ttagttataa agaagaaagt ttaatatttt agacgtttat 4680
attttattaa attgtgggtg aagagttagt gagcggtaaa aggtggtcgt tttgagttgt 4740
ggtgatggaa tttttaggga atagttagga gtttggaaag ttatttttga gttggtttat 4800
ttaagtattc gggatagaag tggttagtag aaatttttta tggaatttta aaatattatt 4860
aggatagtag ttttacgata tttttaaaat ttggagttta aaaaataatg aagtataatt 4920
tatttagaat ttaagtatta agggaaaacg ttaagttttg ttttatttta aagatttttt 4980
tttgaggaaa atatgttatt gttttttaga tgtgaatgaa atgatatttt ttttttcgat 5040
atagtttttt tgtttacgat tttatattgt ttttttttaa ttttttttat tattgaaata 5100
ttatatgtaa gatttgttgg tatgtttttt gattttttat gtttaagggg aaaaatgttt 5160
gtagttgatg tttgagtttt aggagttcgg tgaagaaaaa aaaaaaaagt aacgtaagga 5220
aaattgtttt aatggtatat tttttttaaa gtttgaagtt ttataggtaa aaggagggat 5280
aaatattttt ttttgaaagt aaatagttaa ggtttttttt tatttaatta tttttgaatt 5340
ttttgtaatt ttttcgaggt ttaaatttag ggtataatta attttagatt ttattttaaa 5400
tttaattcgt tttttatttg taatttgttt gtaggtgggt tttttttttt taaattataa 5460
atttgaattt gcgttaattt tttataatat aaggtattta aagttttttg gagttattga 5520
tttggtgacg ttgtagtgta cgtttgtatt tgaaagtaag taagagaata gaaagcgagt 5580
aagagaatcg taatttttat tattttggcg aatttttgtc gggatttata tattaaatag 5640
tatttgtata tagacgtttt aagtcgtttt ttttagagtc gttatttaga tttgtaaggg 5700
gcgggggatt agtggaaatt taaatagaaa tttttattat tataaaggcg agtttttatt 5760
gattatggta attgttttgt ttaggttgta cgggtatggt taattttgtt tgaaagttcg 5820
tttttttttt gcgttttttt gtcgagtttt ttttaggttt tggttttttt cgacggtggt 5880
aagggatgtt ttttacgttt tttattattt ttgttcgcgt ttgcgttttt tgttcgaggt 5940
tttaggtcgc ggcggcggcg cggtgggttt attagcgggc gcgtcgggtt cgcgtagcgt 6000
tgtttgtagt cgcgtcgtcg tatttattat tattatatta gcgcggggaa gtttacgtcg 6060
agttttttcg gtttcggcgc gttttagtta gcgcgttagt aaatagaata tgttggattg 6120
tttagagacg gttttgggtg tttgggggta gaaggggtgt ttttaggtt 6169

<210> 115
<211> 5368
<212> DNA
<213> Artificial Sequence

<220>
<223> chemically treated genomic DNA (Homo sapiens)

<400> 115

agtttttagt aagtttagag gatatacgta agttgtgaaa gtaggtgatt gttattttat 60
gtggttttat ttatgatatt tgttttatag tttatttagg tagttttgaa cggtagtgga 120
gtatgagggt aaagattttt aaacggtaga gtttttgtga gtattgtgtt atttattttg 180
taaaggtaga atttgtaaga tttattagtt gtagtaaata gttgagttgg ttggtttagg 240
gatttggata gagtaagatt agaagttaag agatgagggt ttggtgtagt agtttatttt 300
tgtaatttta gtattttggg aggttgagat gggtagatta tttgaggtta ggagttcgag 360
attaggttgg gtaatatggt gaaattttat ttttattaaa aatataaaaa attagtcggg 420
tatgttagtg tatgtttgga atttagttat ttaggtggtt gaggtataag aattgtttga 480
atttggaagg tagaggtagg ttgtagtgag ttaagattat attgttgtat tttagtttgg 540
gtgatagagt gagattttgt tttggaaaat aaaaaaaaaa agaaaagaaa agaaaagaaa 600
agaagaagga gaagaagaag gaggaggagg gggaggggaa ggggaggggg agggtgaggg 660
ggaggtgagg gggaggggag ggggagggga agggggaggg gagggggagg aagaggaagg 720
atgttaggag atgaggatat taagagaagt atttgaagtt ttagggtgat gagttaaggt 780
ggtttacgtt tgtaatttta gtattttggg aggttgaggc ggtcggatta tttgaggtta 840
cgagtttaag attagtttgg ttaataggga gaaatttcgt ttttattaaa aatataaaaa 900
aattagttgg gtatggtggt atatatttgt aattttagtt attcgggagg ttgaggtacg 960
aaaattgttt gaatttagga ggcggaggtt gtagtgagta gagattatat tattgtattt 1020
tagtttgggt aatagagtaa gattttattt taaagaaaaa attttttttt atagagatga 1080
gggttttgtt atgttgttta ggttggtttt gaatttttgg ttttaagtga tttttttatt 1140
ttagtttttt aaagtattgg gataatagga gtaagttttt atgttcggtt ttaaattttg 1200
ttttttgaat tatatttttt ttttttttat tattggtagt tagaagaagt taagtagtat 1260

tttgaatttt ttttttgggt atgtttttag attattttat ttttgggaat attatttagt 1320
ttttattttt tatttggtta taggtgataa tgttgttaaa ttttttgtta ttgtataata 1380
atggtttttt tttttttatt ttttagtaat aattttttta ttttttttta agtttttatt 1440
gttagttttt gtaaggtttt tttagttttt ggatattgtt ttattttaaa gttaatgtta 1500
tatgatttag gttttgtatt agtagtattt attttatttt tagtattaga tttcgttttg 1560
tttatttata gttatataat aaattatatt aaatttggtg tagtaaaaat atttagtatt 1620
atttatttta ttatatttta tagttatggg gtttgattgg gtttagggag gggattttta 1680
gttttggtgt tttttgtggt tgtagtttaa tggtggtcgg ggttagagtt atttggaagg 1740
tttttttatt tatttgtagg tgattcgtgt agggggtgga tgggtttttt agttggtgtt 1800
gttggtagga atacgtttat tttttttcga ggttgtttgt gttttttata gtatggtggt 1860
tgggtgtaaa gattaagtat tttaagatgt gtgagagaga gagaaggagt tgggttgttg 1920
atttgatttg tgtgattttg tgtgtgattt ggttttagaa attatacgat gttagttttg 1980
ttgtatttta tggattataa gtaagtcgtg aaggttagtt tatgttttag gaagaaattt 2040
aattaaattt ttaaagggag tagtattaga gaatttgtta gtatgtttta gaggcgttag 2100
agtggttagg tacggtggtt tatatttgta attttagagt tttgggaggt tgaggtaggt 2160
ggattgtttg aggttagaag ttttagatta gtttgggtaa tatagtgaga ttttatttat 2220
aatttttttt aaattagtta ggtatggtgg cgtgtgtttg tgtttttaat tatttgggag 2280
gtcgaggtgg gagaattttt taagtttagg tgtttagagg ttgtagtgag ttatggttat 2340
ttattgtatt ttagtttggg tgaaagagcg agattttgtt ttaaaagtaa aaaaataaat 2400
aaaaaaggtc gggcgtgtaa ttttagtatt ttgggaggtc gaggcgggta gattataagg 2460
ttaggagatc gagattattt tggttaatat ggtgaaattt cgtttttaat aaaaatataa 2520
aaaaaaaaaa tagttgggta tggtggtagg cgtttgtagt tttagttatt cgggaggttg 2580
aggtaggaga atggcgtgaa ttcgggaggt ggagtttgta gtgagttgag attatgttat 2640
tgtattttag tttggacgat agagcgagat ttcgttttaa aaaaaaaaaa aaaaaaaaaa 2700
aagggagggg ggtaggttag gtaggtacgg tggtttatgt ttgtaatttt aatattttgg 2760
gaggttaagg taggcggatt gtttgagttc ggtagtttga gattagttta ggtaacgtgg 2820
taaaattttt ttaaaataga aatattagtt gagtatggtg gtgtatttgt agttttagtt 2880
atttgggagg ttgaggtagg aggattattt gagtttagga tgttaaggtt gtagtgagtt 2940
atgattatat tattgtattt tagtttgggt gatagagtaa gattttgttt taaaaaataa 3000
tgtaaaatag aaaataaatt ttttgaaaaa gtgttatagt gggttattta ttaaggtgga 3060
ttggtaaaga ggtagggtat acgtagaggt aaataggtag aagagaatga gtattagaga 3120
tggataggtg ggtagtggtt tatttgtaaa agtggagaat atagggtaag aaagggattt 3180
tgagaagatt ttgagttttt ttttggtatg gtgattggga gaattttatt ggtagtggag 3240
atggagatgt ggagtaggtt ttagaaataa gagcgtggag tagagagaga ggttaggttg 3300
gaggtgtatt tgtgagttat tgttataaag atggtgtttg gagttttaag gttggaatag 3360
gtattgaggg tgagaatggg aaaggatgaa ggaatggtta gggttaggag gaagagggtt 3420
tagtaaatgt tgggttggtt tttttgatta atgattttgt tttttttatt atagtagtat 3480
tttgattttg gaggatggtt tagatgtagt tttgtgaggg aggtttgttg agggaggagg 3540
gatttatggt tatttaaagg gttatgggag gagatgaaaa gtgtggggtt gtggtagtta 3600
ttttattatt aagaggtggg tggaggaggg gttgatagag ttatttatgg aggtggtagt 3660
ttattattga gtttttagat tatttttggg aaatttatgt gggagttttt gtgatagaaa 3720
ttaaattagt aggttcggtt atagtgatgt ttatagtatt tttattgata gtagagtggg 3780
gatggtagag ttttgtagag gggttggcgg cgggaggagc gtaggaatac gtattgtttt 3840
ggggaggcgg ttaggtttta atcgttttat atttagttat tatattttat agattgggaa 3900
agttaagttt agaggttgaa gacgtttatg gaggttatac ggttagaaat tgataacgga 3960
gtatgagtta ggtcgtttgt ttttttgtgt tatgagtttt atattgtatt tttaaaggtt 4020
aagaggaaag ggttttaagt tgaagtgacg gtttcgttgt taggagtcgt tttattgagg 4080
gtgaaataat gtagttaagt gcgggttatt tgcgttgtgg tagaagggat cgaggacggt 4140
gtggttagag gattggtttg ggcggagagc gaggaggggg agtttatagt ttttgggggg 4200
tagtgatagt gcggggaagg gcgaggtgta gtgttggatg ttttcgcggg gattttggga 4260
ttagtgggaa agttggagga taggcggttt tgaagtatcg aggaggggtt tcggaagggg 4320
cggggaggtt tttttgatat aggtgaggag acggagaatt gggggtattc ggtagtttag 4380
gttttttgtc gcgtttttac gttttttagg gttatgagat tttggtttcg aggtggcggg 4440
taggttcggg attcgagcgc ggcgggatcg tgcggttgtc ggtggtgttg ggttttatgg 4500
aggttgcggt ttaggagttt cgggtgggcg gttaggtcga gtttttgtag gcgtgttgag 4560
tcgtcgtagt agggttttag aggggacgag gaattaggtt tagtcgaacg agggggagtt 4620
attagaggtt gggtcgggag gtatcgtcgc gttttgggga gttgttaggg tcggggcgtt 4680
tttcgagcgg ttgttaggtt tcgcgggcgg cggcgaggcg gtttttcgtt tttttttcgt 4740
aggtcgtttt gttagtttga gcggcgggcg cgggtggggt aggaggacgc ggggcggggt 4800
ggggggtata gggggttttt tttgtagcgt tttcggtttt cgcgcgcgga ggtagggtcg 4860
tcgtagtcga ggattagcgc gttcgcggtc ggcgttgcgg gattaattcg cgtggattgg 4920
acgttcggtt cggggattat tgcgcgtttt tttttcgacg tatatatttt cgcggcggcg 4980
gcgtttcggt cgggtcgggt atggcggtag cggttggggg cgcggcgagt cgtcggggtg 5040
tcgggcggtt ttgttttttt tttatcgagt atattttttt tagtttgttc gagcggagtt 5100
tttcggttcg ggtcgtttgt ttatcgtagt tcgtcggtcg ttagagtttc gcgaagttag 5160
aggagttcgg ggcgttcgag gttgcgtttt gcgtttgttg ttgttgttgc ggttttcgcg 5220

cggcgttttg cgggttttta gaggcggtcg tcgggttggg tgagtgggcg cggagcgggg 5280
cgcggggttc ggcggagttc ggggcgcggc gtagtgggtg tcgagtttgg ttttagtttc 5340
gcgttttatc gcgttttcgt ttcgtagg 5368

<210> 116
<211> 5368
<212> DNA
<213> Artificial Sequence

<220>
<223> chemically treated genomic DNA (Homo sapiens)

<400> 116

tttgcggagc gagggcgcgg tgaggcgcgg ggttggggtt aagttcggta tttattgcgt 60
cgcgtttcgg gtttcgtcgg gtttcgcgtt tcgtttcgcg tttatttatt tagttcggcg 120
gtcgtttttg ggggttcgta gggcgtcgcg cgggggtcgt agtagtagta gtaggcgtag 180
ggcgtagttt cgggcgtttc gggttttttt ggtttcgcgg ggttttggcg atcggcgggt 240
tgcggtgggt aggcggttcg ggtcgggagg tttcgttcgg gtaggttgga gaggatgtgt 300
tcgatggaga aggggtaggg tcgttcggta tttcggcggt tcgtcgcgtt tttagtcgtt 360
gtcgttatgt tcggttcggt cggggcgtcg tcgtcgcggg aatatatacg tcggggaggg 420
agcgcgtagt aattttcggg tcgggcgttt agtttacgcg ggttaatttc gtagcgtcgg 480
tcgcgaacgc gttaattttc gattgcggcg gttttgtttt cgcgcgcggg ggtcgggggc 540
gttgtagaga gggttttttg tgttttttat ttcgtttcgc gtttttttgt tttattcgcg 600
ttcgtcgttt aagttggtag ggcgatttgc ggggaagggg cggagggtcg tttcgtcgtc 660
gttcgcgggg tttggtagtc gttcggaggg cgtttcgatt ttggtagttt tttagagcgc 720
ggcggtgttt ttcggtttag tttttggtgg ttttttttcg ttcggttggg tttaattttt 780
cgtttttttt ggggttttat tgcggcggtt tagtacgttt gtagaagttc ggtttagtcg 840
tttattcgaa atttttggat cgtagttttt atggggttta gtattatcgg taatcgtacg 900
atttcgtcgc gttcgggttt cgaatttgtt cgttatttcg gagttagggt tttatggttt 960
tggagggcgt gagagcgcgg taggaagttt gggttgtcgg gtgtttttag tttttcgttt 1020
ttttatttgt gttaaggaga ttttttcgtt tttttcgagg ttttttttcg gtgttttaga 1080
gtcgtttgtt ttttagtttt tttattggtt ttagaatttt cgcgaaaata tttaatattg 1140
tatttcgttt tttttcgtat tgttattgtt ttttagaagt tgtgggtttt tttttttcgt 1200
ttttcgttta ggttagtttt ttggttatat cgttttcggt tttttttgtt atagcgtagg 1260
tgattcgtat ttggttatat tgttttattt ttagtgaagc ggtttttggt agcgggatcg 1320
ttattttagt ttaagatttt ttttttttga tttttgaaaa tatagtatgg gatttatagt 1380
atagagaaat agacggtttg gtttatattt cgttgttaat ttttggtcgt gtgattttta 1440
taggcgtttt tagtttttgg gtttggtttt tttaatttgt aaagtgtagt gattaggtgt 1500
gagacggtta gaatttggtc gtttttttag ggtaatgcgt atttttgcgt ttttttcgtc 1560
gttagttttt ttgtaagatt ttgttatttt tattttgtta ttagtgaaag tgttgtggat 1620
attattgtga tcgaatttgt tgatttaatt tttgttataa gagtttttat ataggttttt 1680
taggggtagt ttggaggttt agtgatgagt tgttattttt atgagtggtt ttgttaattt 1740
tttttttatt tattttttgg tggtagaatg gttgttatag ttttatattt tttatttttt 1800
tttatggttt tttggatggt tatggatttt ttttttttta gtagattttt tttatagaat 1860
tgtatttgga ttatttttta aaattagagt gttgttatga tgggggaggt agggttattg 1920
gttaggagag ttagtttaat atttgttgag tttttttttt tttagttttg gttatttttt 1980
tatttttttt tatttttatt tttaatattt attttagttt tggagtttta gatattattt 2040
ttgtgatagt gatttataag tgtattttta gtttggtttt tttttttgtt ttacgttttt 2100
gtttttggag tttgttttat atttttattt ttattattaa taaagttttt ttagttatta 2160
tgttagaaag gaatttaagg tttttttaaa attttttttt tattttatgt tttttatttt 2220
tataaatggg ttattgttta tttgtttatt tttagtgttt attttttttt atttatttgt 2280
ttttgcgtgt gttttgtttt tttattagtt tattttggta aatggtttat tgtggtattt 2340
ttttaaaaag tttatttttt attttgtatt attttttgag atagggtttt gttttgttat 2400
ttaggttgga gtgtagtggt gtgattatgg tttattgtag ttttggtatt ttgagtttaa 2460
gtgatttttt tgttttagtt ttttaagtag ttgggattat aggtgtatta ttatgtttag 2520
ttaatgtttt tgttttgggg gggttttgtt acgttgttta ggttggtttt aaattgtcga 2580
gtttaagtaa ttcgtttgtt ttagtttttt aaagtgttgg gattataggt atgagttatc 2640
gtgtttgttt ggtttgtttt tttttttttt tttttttttt tttttttttt gagacggagt 2700
ttcgttttgt cgtttaggtt ggagtgtagt ggtatgattt tagtttattg taagttttat 2760
ttttcgggtt tacgttattt ttttgtttta gtttttcgag tagttgggat tataggcgtt 2820
tgttattatg tttagttatt tttttttttt gtatttttat tagagacggg gttttattat 2880
gttagttagg atggtttcga ttttttgatt ttgtgatttg ttcgtttcgg ttttttaaag 2940
tgttgggatt atacgttcgg ttttttttgt ttgttttttt atttttgaga tagggtttcg 3000
tttttttatt taggttggag tgtagtgggt gattatagtt tattgtagtt tttgaatatt 3060
tgggtttaag aggttttttt atttcggttt tttaagtagt tgggagtata ggtatacgtt 3120

attatgtttg gttaatttaa aaaaaattat agatagggtt ttattatgtt gtttaggttg 3180
gtttagaatt tttgatttta agtaatttat ttgttttagt tttttaaagt tttgaggtta 3240
taggtgtgag ttatcgtgtt tggttatttt ggcgtttttg aaatatgttg gtaggttttt 3300
tgatgttgtt ttttttggga gtttaattgg gttttttttt gaaatatggg ttggttttta 3360
cgatttgttt gtaatttata gaatgtagta gaattgatat cgtgtggttt ttgaggttaa 3420
gttatatata gagttatata gattagatta gtaatttaat tttttttttt ttttttatat 3480
attttgggat gtttgatttt tatatttagt tattatgttg tgaggggtat aggtagtttc 3540
gggagaggat aggcgtgttt ttgttagtag tattagttga aggatttatt tattttttgt 3600
acgggttatt tataagtgag tgaggaagtt ttttagatgg ttttggtttc ggttattatt 3660
ggattgtaat tataggagat attaaaattg agaatttttt ttttgagttt agttaagttt 3720
tatgattatg aaatataata aaatagatga tgttgggtgt ttttattgta ttaagtttga 3780
tgtggtttgt tatataatta tagataagta gaacgggatt tggtattgga agtggggtgg 3840
gtgttgttga tataaaattt aaattatgtg atattggttt tgggatgggg tagtgtttag 3900
gagttgggag gattttgtag aggttgatag tggggattta gaggaaagtg aagaaattgt 3960
tattggaaga tggaggaaag aagattattg ttatgtagtg gtaggaagtt tagtaatatt 4020
gttatttgta gttaaatagg aaatagaagt tagatagtat ttttaagaat aaaatgattt 4080
aaggatatat ttagagagaa agtttaaggt gttatttggt tttttttagt tattaataat 4140
aaaaagaaag aagatatgat ttaaaaagta agatttaggg tcgggtatgg aggtttattt 4200
ttgttatttt aatgttttgg gaagttgagg tgggagaatt atttgaggtt aggagtttaa 4260
gattagtttg ggtaatatag taagattttt atttttataa agaaaagttt tttttttaag 4320
atggagtttt gttttgttgt ttaggttgga gtgtagtggt gtaatttttg tttattgtaa 4380
ttttcgtttt ttgggtttaa gtaattttcg tgttttagtt tttcgagtag ttgggattat 4440
aggtgtgtgt tattatgttt aattaatttt tttgtatttt tagtagagac ggagtttttt 4500
tttgttggtt aggttggttt tgaattcgtg attttaggtg attcgatcgt tttagttttt 4560
taaagtgttg ggattatagg cgtgagttat tttgatttat tattttggag ttttaggtat 4620
ttttttgat gttttattt tttaatattt ttttttttt ttttttttt ttttttttt 4680
ttttttttt ttttttttt ttattttttt tttatttttt ttttttttt ttttttttt 4740
ttttttttt ttttttttt ttttttttt ttttttttt ttttttttt tttttttat 4800
tttttaagat agagttttat tttgttattt aggttggagt gtagtagtgt gattttggtt 4860
tattgtagtt tgtttttgtt ttttaggttt aagtaatttt tgtgttttag ttatttgagt 4920
agttggattt taggtatgta ttgatatatt cggttaattt tttgtatttt taatagagat 4980
ggggttttat tatgttgttt agtttggttt cgaatttttg attttaggtg atttgtttat 5040
tttagttttt taaagtgttg ggattatagg agtgagttat tgtattaggt ttttattttt 5100
taatttttaa ttttgttttg tttaggtttt tgaattaatt agtttaatta tttgttatag 5160
ttggtgggtt ttgtaggttt tgtttttgta gagtggatgg tatagtgttt atagaaattt 5220
tgtcgtttgg gagtttttgt ttttatgttt tattgtcgtt tagggttgtt tgagtgagtt 5280
gtggggtaga tgttataagt gaggttatat gggatggtag ttatttgttt ttatagttta 5340
cgtgtgtttt ttggatttgt tgagagtt 5368

<210> 117
<211> 6301
<212> DNA
<213> Artificial Sequence

<220>
<223> chemically treated genomic DNA (Homo sapiens)

<220>
<221> unsure
<222> (3683)

<400> 117

gttttgtaga gttggggttt tattgtgtta gttaggttga ttttgaattt ttggtattaa 60
gtgatttttt gtttttagtt ttttaaaatg ttgggattat aggtatgagt tattatgttt 120
agttaggagt tattttttga gtatagttgt gatatgaatt gatttatgtt tttaaatgat 180
ttttttggtt gttgtgttta tagaaagtta tggtgggttt agggtggaag tagggattat 240
tagagatgtt attgtaatta tttttttttt tgattttttt tttttttttg attgtatttt 300
tgtattttta gatttttttt ttgttttgtt agttgttttt tttttagtat tatttttgtt 360
tttattttta aatgttggag aattttagat ttagttttga gatttttttt gtatttagta 420
ttatttattg agtaattttt tttttttttt ttgagataag gttttattta tatatttagg 480
ttgaagtgta gtggtatgaa tatagtttat tatagttttt attttttggg tttaagggat 540
ttttttattt tagtttttta aagttttggg attataggta tgagttattg tgtttagtta 600
ttggatgatt ttatgtaaat ttatagtttt taaaattatt tttttgttgg tgatgtataa 660
atttagtatt ttagtattga gtttatagta atttgttatt taaattggag gtgtttatta 720
ttttttaaat tttttttttt ttagtttagg tttttatatt ttagtaaatg gtattgtatt 780

tgtttaagtt aaaaaattta gttattttta atgttttttt tttttttatt tttttattta 840
atttattagt aagatatgtt ttttaatttt tgaattaaat aggtattttt tttaaaatgt 900
tatgtatata ataattttaa aaattttaat ggagttagtg taatggttgg tatttgttgt 960
taatagttat tttggaggtt gaagtagaaa gattgtttga gttttagagt ttaagattag 1020
tttggataat atagtgagat tttattttta aaaaaatata aaataaaata aaaagaaaag 1080
aaaaaatttt agtggtttaa ataatgaata tgttttttat aaaattgtag gttagttggt 1140
atttggttga gattggttgg atttggttga tttggttgta ggttgtaaga tggattagga 1200
tttgatttat gtgtgtttat tttgggtttt aggttgtagg gtagtagtta ttttagggat 1260
gattttatag tgaagttaaa agtataagag ggggttgggt atttgtaatt ttagtatttt 1320
gggaggacga gttaggtaga ttatttgagg ttaggagttt gagattagtt tgattaatat 1380
ggtgaaattt cgtttttatt gaaaatataa attatacgtt tgaattttag tattttggga 1440
ggttgaggta ggtagattat ttgaggttag gagtttaaga ttagtttgat taatatggtg 1500
aaaatttatt tttattaaaa atataaaaat tagttaggtg tgatgatatg tatttgtagt 1560
tttagttatt cgggaggttg aagtaggaga attatttgaa tttgggtggt agaggttgta 1620
gtgagtagag gttgcgttat tgtattttag tttgggtgat agagtgagat tttgttttaa 1680
aaaaaaataa aaataaaatt tttaaatggg tatattgagt ttttgtttat attatttttt 1740
ataaataagt ttaaaattag gaggtaggag gtatattgta tttattatga aattatagta 1800
aggggatggt tgtataagat attatgggga ttggagaatt gggattaagt atttaattat 1860
tttatttttt ataatttttt aaagaattgt attttttttt aaatttattt ttttttttta 1920
cgtttttttt aaaggattat tattttttgt cgttaaataa ttgtttttgt ttttattatt 1980
gtatttttta tatttagaat aatgtttagt aagtagtata tgtttagtaa attatgtgtt 2040
gaataaataa tttttaattt gatttttttg gttgtattta ttttttttta attttttttt 2100
atagagtagt tagagtaagg tttaaaaata aattttatta ttttggtttt agtttaaaat 2160
tttattttgg tttttttttg tattggaatt ttttattatg ttttttataa aggttattta 2220
ttatttagtt tagtttggtt ttttaaatgt atttggtatt attgatttta ttttaattat 2280
tgtttttttt ttttttaaat attttaagtg atttttaatt gtaagatttt tgtatttgtt 2340
attgtttttg agtgtagtgt ttttttttag gtttttggta tgttagttta gttttagtga 2400
aaatgttatt tttttaggtt tttttttatt atcgattatc gatttttgta ttttaagtta 2460
ttttttgtta ttatattgta tttttattat tttgtttatt ttttttaatg tatttattat 2520
gttatgaaat tagtatgttt tgttcgttat tgatttttag tatttataat aatgtttggt 2580
atatattaag tgttttgtaa aagtttgttg aggttaagta tggtggtggt ttatatttgt 2640
aattttatta ttttaggagg tcgaggtagg tggattattt gaggttaaga gtttgagatt 2700
agtttggtta atatggtgaa atttttattt ttattaaaaa tataaaaatt agttaggttt 2760
ggtggtgtat acgtgtaatt ttagttattt aggaggttaa ggtaggagaa ttgtttgaat 2820
ttaggaggtg gaggttgtag tgagtcgaga ttacgttatc gtattttagt ttgggtaata 2880
gtgagatttc gttttaaata aattagtaaa taaataaata aaaatttgtt gagtgagtat 2940
atgagaaaat gtttttttta tatttttaga taattttttt tgttttttat ttttaattat 3000
ttatttatat ataaaatgat atgtgtaaat ttagtcgggc gtggtggttg aagtttgtaa 3060
ttttagtatt ttgggaggtc gaagcgggcg gattacgagg ttaggatatc gagattattt 3120
tggttaatac ggtgaaattt cgtttttatt aaaaatataa aaaaattagt taggcgtggt 3180
ggcgggcgtt tgtagtttta gttatttagg aggttgaggt aggagaatgg cgtgaattcg 3240
ggaggcggag tttgtcgtga gtcgagatta tattattgta ttttagtttg ggcgatagag 3300
taagattttg ttttaaaaaa atagatagat agatagatag atagatagat agatagatag 3360
atagatagat agatatagat agatagatat agatatgtaa atttaaggaa aattaattta 3420
agttgaataa aaatattgga atatttttgt taataaagaa gaagttgaaa attggagttg 3480
ttttggtatg cgttgtggta tttttttgtt ttgtgttttt taggtattta tttgcggggt 3540
ttaggggttt agtgaggttt atattgatat ttattttttt attttttgta ttaatttatt 3600
ttatttggtt tatttgtttt ttttttttat ttttattttg gttaatatat tagataattt 3660
atttataatt attatttgtt ttnttgtttt ttatttttgt attattgtga gttttatgaa 3720
ggtaaggata gtattttgtt tttttttggt tttgagaagg tgtttaatta attgtggaag 3780
gaaataatga atgttttaaa ggagagaaag aaagttgaaa aggttttgta ttttttcgtt 3840
tttttttagt cgttttagat taagatattt tttatttttg tataatttat attttagtat 3900
tatatataaa atttttatga ggtataaatg tttatcggtt tatttattcg tagaatggtt 3960
ttatacgtat atttataaat ttatatgttt gttgatgttt tgtatgtgag attttttatt 4020
tattcgagag tttttattag gaggaattta gtttaatatt tgtgagatta taataggata 4080
aaagttgatg aagatgagtt ttattttata gaggttattt tttttatgag tggagattgt 4140
tttttttttt tttgaaggaa agaatagtag ttgtttaaat aatgaataaa gttaagtatt 4200
taaatttatt tttaggaagg tattggaaga agaggaggag tagagtataa aaattataag 4260
tatatttttg cggggggaat agttagttta aaattttttt aattagtagt tatattttaa 4320
ttttaatttt aagttttttt aggattatga aattaaaata ttttgttttt ggttattttt 4380
ataagtttta ttattttgaa agattttatt ttttgtttat tttttaaaag attaatagac 4440
ggaaattttt ttttagaaag taaattatgt tttttttttt gaaataattg tgatagagaa 4500
attatttatt ttaagtagtt atgtttttaa gttgtttgaa ggtgattata tatgaatttt 4560
tatataaata tgtatttttt ttatttaaaa gaaaagagga aggaaaggaa ttagtttatt 4620
aattatttaa aatatttggt gtaattgttt ttatgaatta atttaggtat ttggaagata 4680
attatatttt atgttaataa tattgttaat tttttttatt atgttgtatt ttaaatatta 4740

ttgtgttttt attgataata ttttttatta tatatgtata tgaaaaatag gtatttttat 4800
tagaattatt gtaatagtat taagagatta ttattttttt ttattttttt ggaagaaaat 4860
ttatttaaat tagattattt attattcgat tgttttcgta ttttgtttgt atgtttaggt 4920
aattggagtt tatgaaagta taaattttag aataaaaagt ttgattttat atattaatga 4980
ttttaaataa aagataagtg ttatatttgt gtattatttt tttagagaaa gttaagtttg 5040
tgttttgttt aggagagata atattttttg tttttgtagg tggttttttt ggtgtagtta 5100
ttagttgtta attatttgta aataaataaa taattaattt tttaagttgt tggttgggta 5160
agtgtttatt gatatgttaa aattttttaa gataggattt taattagtga cgttttaaat 5220
ttagtttttt tgttagcgga gttataaggt gaattgtagg aagattttag ttttatatac 5280
gtggggtaga gttagtagag gttagagttg ttgttttgtg tagattacga gaggatgttt 5340
tttagttttt aggaaggcgt ttagttcggg gaaaataaat ttttaatttg ttttttttta 5400
attgagagaa ttttaggatt ggattagaag aaagattgtg ttttattaat gaaattttat 5460
aggttttggg tagatatttg tagtttatta ggtatgttat agtttaattg ttttaatttt 5520
ttgttatttt atttatagtg tttaatttgt tttttatttt atattatata gagttagagt 5580
ttaggatttt aatttaattt attattatta tttattattt tttttttttt tttgagatag 5640
agtgttggtt ttgttgttaa gattggagtg taatggtata attttggttt attgtagttt 5700
tcgttttttg ggtttaaacg atttttttgt tttagtttgt taagtagttg ggattatagg 5760
tatgtattat tatggtttgt taatttttgt attttttagt agaggggggt tttattttgt 5820
tggttaggtt agtttcgaat ttttgatttt aggtgattcg tttgttttag ttttttaaag 5880
tgttaggatt ataggtatga tttattacgt ttagttagag tttaggattt ttaatgtttt 5940
taattgagtt ttatgggaaa ttaaaagaat attagttttg gattgagtaa agtggataaa 6000
tttatttata ggaaatttta agaatttgta tttattttag aagatttttt tttgtttgat 6060
atattagata tttgaaattt ttttttattg aaaaataaac gatatttaga gattaaagag 6120
gtaggattat taagatagga tttttatttt ttattggttt ttggttaacg ggtgtaatt 6180
tttttgtgaa ttttagaatt ttattaggaa atgaaagatg agtttttgaa attattttta 6240
tagaattttg tggaatataa gattgattat ttaagatagg gtttattttt ttgtttttag 6300
g 6301

<210> 118
<211> 6301
<212> DNA
<213> Artificial Sequence

<220>
<223> chemically treated genomic DNA (Homo sapiens)

<220>
<221> unsure
<222> (2619)

<400> 118

tttaaaaata aaaaaataag ttttgtttta gatggttaat tttatgtttt ataaagtttt 60
atagaggtag ttttaggaat ttatttttta ttttttagtg gaattttggg gtttataaga 120
gaattgtatt tcgttaatta aaaattaata aaaaatgaag attttgtttt ggtaattttg 180
tttttttggt ttttgaatgt cgtttatttt ttagtgaaaa gagattttaa atatttgatg 240
tgttaaatag ggagagattt tttgaagtaa atgtaggttt ttgggatttt ttgtgagtga 300
atttatttat tttgtttaat ttaaagttga tattttttta attttttata ggatttagtt 360
ggaggtatta aaagttttag attttggttg ggcgtggtgg gttatgtttg taattttaat 420
attttgggag gttgaggtag gcggattatt tgaggttagg agttcgagat tagtttgatt 480
aatagggtga aatttttttt tattaaaaaa tataaaaatt agtaggttat ggtggtgtat 540
gtttgtaatt ttagttattt ggtaggttga ggtaggagaa tcgtttgaat ttaggaggcg 600
gaggttgtag tgagttaaga ttatgttatt gtattttagt tttggtaata agattaatat 660
tttgttttag aaaaaaaaaa aaaataataa ataataataa taaattaaat taaagtttta 720
aattttagtt ttatgtagta tgaaataaag ggtaaattaa atattgtaaa tgaaataata 780
aagaattgaa ataattaagt tgtggtatat ttgatgagtt gtaggtgttt gtttagggtt 840
tgtggggttt tattaatgga atatagtttt ttttttggtt tagttttaag atttttttaa 900
ttgaaaagga gtaagttgag ggtttgtttt tttcgagttg agcgtttttt tgaaggttgg 960
gagatatttt ttcgtggttt gtatagagta atagttttgg tttttgttgg ttttgtttta 1020
cgtgtatagg gttgggattt ttttgtagtt tattttatag tttcgttgat aagggggttg 1080
gatttagaac gttattaatt aaaattttgt tttagaaagt tttagtatgt taatgaatat 1140
ttgtttagtt agtagtttaa ggagttaatt gtttatttgt ttgtaagtaa ttagtaatta 1200
atggttatat taggggggtt atttataggg ataaaaagtg ttattttttt taagtagaat 1260
atagatttaa ttttttttaa agaagtgatg tataaatgtg gtatttattt tttatttgaa 1320
attattaatg tataaaatta agttttttat tttgaagttt atatttttat gggttttagt 1380
tatttaaata tataggtaag atacgagagt agtcgagtaa taaataattt aatttaagta 1440

aatttttttt taaggaaatg ggaaaaaata ataatttttt aatgttatta tagtaatttt 1500
gatgaaaatg tttgtttttt atatgtatat gtaataggga atgttgttaa tgaaaatata 1560
atagtatttg gagtgtagta tagtggaaaa ggttggtaat gttattaata tggagtgtaa 1620
ttgtttttta agtgtttgga ttaatttata ggaatagttg tattaagtat tttaaataat 1680
taatgggtta attttttttt tttttttttt tttttaaatg agagagatat atgtttatat 1740
gaaggtttat atgtaattat ttttaagtaa tttagaagta tgattattta aagtgaataa 1800
tttttttatt atagttattt taaagaaaag aatatagttt gttttttaaa gaaaaatttt 1860
cgtttgttga ttttttgagg gatgggtaga ggatggagtt ttttaagatg gtaaggttta 1920
tgaaaataat taagaataga atgttttaat tttataattt taagaaaatt tgaaattaaa 1980
attaaagtat aattgttgat tgggaagatt ttgaattggt tgtttttttc gtaaaggtat 2040
atttgtagtt tttatatttt gttttttttt tttttttagt atttttttga agataaattt 2100
aagtgtttag ttttatttat tatttaaata attgttgttt ttttttttag ggaagggaaa 2160
aatagttttt atttatagag gaaatagttt ttataggatg gagtttattt ttattagttt 2220
ttattttatt atgattttat aaatattgag ttaaattttt tttgatgaaa gttttcgggt 2280
aaataaagga ttttatatgt aaaatattaa taggtatatg gatttataaa tgtacgtgta 2340
aaattatttt gcggatgaat aaatcggtga atatttatat tttatggaga ttttgtatat 2400
agtgttgaga tgtagattgt gtaaaggtgg ggggtatttt aatttgggac gattaggagg 2460
aagcgagaga atataggatt tttttagttt tttttttttt ttttgagata tttattattt 2520
ttttttataa ttggttgagt atttttttag aattagaaaa aaataaaata ttatttttgt 2580
ttttatagag tttatagtgg tgtaggggtg gggggtagng aaataaataa taattatagg 2640
taaattattt agtatgttag ttagggtaag aatgagaaaa ggaaataggt gagttaggta 2700
agatgaatta gtgtaggaag tggaggggta gatgttagtg taggttttat tgagtttttg 2760
agtttcgtag atgagtgttt aagaggtata aggtagaagg atgttatagc gtatgttaga 2820
gtaattttag tttttaattt tttttttatt aataaagatg ttttaatatt tttatttagt 2880
ttaaattgat tttttttgga tttatatatt tatatttatt tatttatatt tatttattta 2940
tttatttatt tatttattta tttatttatt tatttattta tttttttgag atagagtttt 3000
gttttgtcgt ttaggttgga gtgtagtggt gtgatttcgg tttacggtaa gtttcgtttt 3060
tcgggtttac gttatttttt tgttttagtt ttttgagtag ttgggattat aggcgttcgt 3120
tattacgttt ggttaatttt tttgtatttt tagtagagac gaagttttat cgtgttagtt 3180
aggatggttt cgatattttg atttcgtgat tcgttcgttt cggtttttta aagtgttggg 3240
attataggtt ttagttatta cgttcggttg gatttatata tattatttta tatatggatg 3300
agtagttgaa aataaaaggt aggaaaaatt atttaaaaat gtgagggaag tattttttta 3360
tatatttatt taatagattt ttgtttgttt gtttgttggt ttgtttgaga cggaatttta 3420
ttgttattta ggttggagtg cggtggcgtg atttcggttt attgtaattt ttattttttg 3480
ggtttaagta gtttttttgt tttagttttt tgagtagttg ggattatacg tgtgtattat 3540
taagtttggt taatttttgt atttttagta gagatggggg ttttattatg ttggttaggt 3600
tggttttaaa tttttgattt taagtgattt atttgtttcg gttttttaaa gtggtgggat 3660
tataggtgtg agttattatt atgtttggtt ttaatagatt tttatagggt atttaatatg 3720
tgttagatat tattataggt gttagagatt agtgacgaat aaaatatgtt aattttatgg 3780
tatgataaat atattgaaag gagtgaataa ggtgatggag atgtaatata atggtagagg 3840
gtgatttaaa atatagaggt cgatggtcga tggtggagga gggtttaagg aagtgatatt 3900
tttattgaag ttgagttagt atgttaagag tttgagaaaa agtattgtat ttagagataa 3960
tggtaagtat aaaggttttg tagttgaaaa ttatttggaa tgtttgagga ggaagaaggt 4020
agtagttgga gtgaagttag tggtattagg tgtatttgga gagttaggtt aggttggata 4080
atggatggtt tttataaagg atatggtaag gagttttagt gtaaagagaa gttagggtag 4140
ggttttaagt tgggattaaa atgatagaat ttgtttttaa attttatttt ggttgttttg 4200
tggaaaggga ttagagagaa gtgagtgtaa ttagggagat tagttgggga gttatttatt 4260
taatatataa tttgttaagt atatattatt tgttaggtat tgttttaggt gtgagggata 4320
tagtggtgaa aataaagata attgtttagc ggtaaaagat gatgattttt tgaaaaaagc 4380
gtgaggagag agggtagatt tggagagaag tgtaattttt tagaaggtta tgggagatag 4440
aataattgaa tatttggttt taatttttta atttttataa tattttatat aattattttt 4500
ttgttgtggt tttatggtga atatagtata ttttttattt tttgattttg ggtttgtttg 4560
tgggaagtga tgtgagtaga agtttagtgt gtttatttag gggttttgtt tttgtttttt 4620
tttgagatag agttttattt tgttatttaa gttggagtgt agtggcgtaa tttttgttta 4680
ttgtaatttt tattatttag gtttaagtga tttttttgtt ttagtttttc gagtagttgg 4740
gattataggt atatgttatt atatttggtt aatttttgta tttttagtag agatgggttt 4800
ttattatgtt ggttaggttg gttttgaatt tttgatttta agtggtttgt ttgttttagt 4860
tttttaaagt gttgggattt aggcgtgtaa tttgtatttt tagtagagac ggggttttat 4920
tatgttggtt aggttggttt taaatttttg attttaagtg atttgtttga ttcgtttttt 4980
taaagtgttg gaattatagg tgtttagttt ttttttgtgt ttttgatttt attgtgagat 5040
tatttttgag atagttgttg ttttatagtt tgagttttag aatgaatata tatggattag 5100
attttaattt attttgtagt ttgtagttaa gttagttaag tttagttaat tttagttaaa 5160
tattaattga tttgtagttt tgtgagaaat atatttattg tttaagttat tgaagttttt 5220
tttttttttt ttattttatt ttatgttttt ttagagatag gattttatta tgttgtttag 5280
gttgattttg aattttggag tttaagtaat ttttttgttt tagtttttag agtagttgtt 5340
gataataggt gttagttatt atattggttt tattgaagtt tttgaggtta ttatatatat 5400

agtattttag aggaaatatt tatttgattt agaggttaaa gaatatattt tgttgatgga 5460
ttggataggg aggtgaggga aaggaaggta ttaaagatga ttaggttttt tggtttgagt 5520
aaatataatg ttatttattg ggatgtgaaa gtttgggtta gggaaggaaa ggtttgggga 5580
gtaatgaata tttttagttt ggatagtaag ttgttataag tttagtgtta gagtgttaag 5640
tttatgtatt attagtaaag agatggtttt agaagttata ggtttgtata agattattta 5700
gtggttgggt atagtggttt atgtttgtaa ttttagaatt ttgggaggtt gaggtaggag 5760
gattttttga gtttaggagg tagaggttat agtgagttgt atttatgtta ttgtatttta 5820
gtttggatat atgagtgaaa ttttgtttta aaaaaaaaaa aaaaaattat ttagtgaatg 5880
gtgttagata taagaagggt tttaggattg agtttaaggt tttttaatat ttggagatag 5940
ggatagaagt ggtattggaa aaggagtagt tagtaagata ggagaaaaat ttggagatat 6000
aagaatatag ttaagagagg gaaaagggtt aaggaaaaga atggttataa tagtattttt 6060
agtggttttt gtttttattt tggatttatt atggtttttt atgaatatag tagttagagg 6120
gattatttaa aaatataagt tagtttatgt tataattgtg tttaaaaaat ggtttttggt 6180
tgggtatggt ggtttatgtt tataatttta gtattttggg aggttgaggg tagaggatta 6240
tttggtgtta ggagtttaaa attagtttgg ttaatatagt gagattttaa ttttataaaa 6300
t 6301

<210> 119
<211> 8712
<212> DNA
<213> Artificial Sequence

<220>
<223> chemically treated genomic DNA (Homo sapiens)

<400> 119

cggggtgagg gtgtttttta aaatggtaat aggaaattta ggttgtaggg agaggttgag 60
gaggaggggt tgagggagaa aatttataga gcggagtata tagtagattt gggtttattt 120
taggtttcgt ttcggtttga gggtttgagt tgtaggtttt tgggttgtta attttgagtt 180
tatttttttt agattttttt aaggtttagt ttttaggggt tatagaggag gttagtagat 240
taaaggatag tatgaatgat ttgatgtttt tggtttttat tgttttgaag gggttggggt 300
tggtgggggg atttgagtag atgtttttag tttcggtgag tttaggtttt ttttatagat 360
tttttttttt tttgagtttt aggaaggagg tcggagtatt ttttagtagt ttttgttttg 420
attttttggg tttttatttt agtttaatta agttattttt tgtgttaggg gtaagtttta 480
ttttaaattc gtgtttaggt tttttcggtt agttatatag tatttttatg tttgtttttt 540
gaagaaagtt ttgatagttt atattgaaac ggaattgtat gagttttcga agattatatg 600
gttttagtag gataggatta atgtgtttgg gtttttttgt agttaagttt tttttgttgg 660
ttttgggttt tagttttgga aatgtaagat ttgttttttg ttttagttat ttagtgtttt 720
tttaggtaat tgttttattt tatatggaaa aaaagtaata tttaaatgat aaataaatgt 780
atgatatttg tttcgtattt tgtgaatatg tgaatgtata ggtttttttt ttagttttta 840
cgggttttta gttgtgtgtg taagtttaga tgtaaataga tatagattat aacggcgttt 900
ttttttgtga tggtttgtag aaattattat ttttttttta agtagtggaa ggaagggtag 960
ttgtaggtat tttttagttt tttaatagga ggtttgtaga aagtaggttt tcgcgttggg 1020
tagggaggag ttgtggtggg ggttagttga ttagggtgta ttaggttttt tttgtttgtt 1080
ttgagaaatt aagagtttta gggagaattt tttagggttg agttttgttt ttggggtcgt 1140
tggagagaaa atttatttgg agatttaggt ttggtagtat tattaggatt agatttttga 1200
tttttttttg tggttttttt taagatttaa gtttagtttt agattgtttt aaagttagtt 1260
gggttggatt aatatagata ttgggaatag gaagagattt agtaggtttc gttttatata 1320
ttgtaaatta tataaagatt tatatttttt agatagtttt tttttttttt tatatttgtt 1380
tttttttatg tagttttata gagaattttt tttttatatt aatatatttt taaatataag 1440
tagaatattt gattatttta aggttttata gttttttaga gtttatttgg tttaattttt 1500
gtttttgtag ataaagaaat aaatttatcg agataaggag ttttagattt ttgaaatggt 1560
atttagttta gaggggcggt tcggatagga cgtagggttg ttaggttagg tttttttcgga 1620
tagttttgta tagttttata ggtggtatag atatttgaag gatttgtata cgtgttgttt 1680
tgtttgtgtt tttagggagg tgtttttagt aggttttagg tagaatagaa atatgagata 1740
gggtgtgaag ggttttttgga ttatggttat tatagaataa aaaggggtga aattaatttt 1800
tttaggagtt aaaaaaaaaa gttagaaaat atttattgtg attttgttga aggagatggt 1860
tgatgttatg agtttagatt atatatttta ttattaatta aaattatttt ggtagtgtgt 1920
atttttggtt ggagagagga atagtatttt ttttaaggtt tattttggga gatttttttt 1980
gtagttattt ttttttttta gttttttaaa ttattgattt taggattaag taatttgttt 2040
ttagtagatg ggggtgatga gaagagaaag gtaagttagt tttggtgtta aatttttgga 2100
ggaggaggtt aaggttgtgt tttttatttt ggggaggtag agttatagtt agggtttttt 2160
gtttgggata ggtggttaag aatttttggt tttttttata attttttttt ttttaaaaag 2220
aaatagatgg gatggggatt tagggttagg tgtatttgaa agtttgaggg gtagtagggt 2280
gggaatagtg tggtttttgt aattagattt ggttttattt ttagttttaa ggttaaattg 2340

tttgttggga ataaggattt taaatttttt gaatttgtat tttagaaaat ggagatgata 2400
gttcgggtac ggtcgtttat gtttgtaatt ttagtatttt gggaggttga agtgggtgga 2460
ttatttgagg tcgggagttc gagattagtt tgattaatat ggtgaaattt tatttttatt 2520
aaaaatataa aaattagttt ggtttggtgg tatatgtttg taattttagt tattggggag 2580
gttgaggtag aagaatcgtt tgaatttggg agatggaggt tgtagtgagt tatgattatg 2640
gtattgtatt ttagttcggg taataagaat aaaatttatt tttaaaaaaa aaaaaaaaaa 2700
gtggggggcg tttggcgcgg tggttgattt ttgtaatttt agtattttgg gaggtcgagg 2760
cgcgcggatt aggaggttag gcgattaaga ttattttcgt taatacggtg aaatttcgtt 2820
tttattaaaa atataaaaaa aaaaaagaaa gaaaagaaaa tggagatacg gagatgataa 2880
aaattttttt tttagtgtta tagtgaagat tacggatgag aattgtagaa agtttatagt 2940
atatagtagg tgtttaagaa atgcgcgtgt tttttttttt ttagtgagag gtagaattgt 3000
atttgtgtta ttataaagat atttaaaggt acgataaatt ttatgtgaat gtttgggatt 3060
tttttttatt agtggtcggt tttgtaaagt tagattatat ttattttaag ttgtagttat 3120
ttatttattg ttattttttt aattttttaa ttagttttgt gtatttggga attattttgt 3180
gttataatga aaacgatatt atttatggta atagagttat tgttattaat tttcgtttag 3240
gttaaaagtt ttttgaggag aagtggtaag gtttttatat ttgttttttt aaaaattttt 3300
tttgtagtaa tttggcgtgt taattttgaa gtttatttgg tattgagttt ttttttggtt 3360
tttttttttt tttatagttt ttttcggttt tttatttatt ttttatttgt atagtaagtt 3420
ttggaatttg gaattttttt ttagttttta cgtttgttgg ggaagataat tgggtttaat 3480
ggcgttattg ataaagtttg gtgttgatgt cgtttgattt ttcgattttt gtgtggattt 3540
ttttagtttt aaaaacgttt ttggaagtgg ttttattgta aatatttgaa gtgtgcgtgt 3600
gggggagggg tgagatttgg atgtttattt cgtgtttcgt ttatttacgg gaagtttttt 3660
tttttttatt ttttaggagg tttcggtagt acgagagata tagttttttt tcgtttttgt 3720
tcggggattg gtcgggggtc gtagcggtag ggttttcgaa ggatgtcggg ttttacgggt 3780
tttttagggt cgaggtgtgt gggttttgat tttcgtaagt ttatgggtat ttttcggtag 3840
cgaggaagag ttttgttatc gaggattcgt tttattgagt aggtacgtgg gcgacgtttg 3900
taagtcgatt tgggttttgt aggttagagc gtttagattt atcgttttcg ttttgattgg 3960
ggtaggggtt tttagagagg attcgtggag gttaaattta tatttatatg cgcgtttata 4020
tttttatatt ttcgtatata tatttgaaaa tgcggggttt ttttatagta gataaggttc 4080
gatttgtaga gttttggtta cggggataaa attttttttt gttgcgtttt ggaggttcga 4140
gagtagggag ggtttgggaa atattacggt ttttagtgcg ttgtcgtcgt ttcgtagagc 4200
ggttaggttt gcgtagcgtg ggcgtttagg agcgtggaat tatcgcgttt cgtaaggtag 4260
acgtggattt attttagcgt cgataaggag gtcggagagg acgcgcgtag ttggttttag 4320
ggattaatta agttcggagg ggtttacgtg tttcgggagc gggttttggg tcgtcgcggg 4380
gttttttagg ggttcggggg ttgttagtgt tcgtcgttcg gtttcgtatt taggggaggg 4440
cgggtagagg ttttaggttg agtttcggag tatttatttt tcgttggtgt tttcgttcgt 4500
tagcgtcgcg taatagttcg ggttgtttag gatttcgcgg cgcgggggcg gggcggcggg 4560
cggtcgggac ggtgataggt gcgtttttcg gttaatggcg gagttcgcgg tttttttcgg 4620
gttttcgttg cgcgcgttaa tcggaagtcg cgttgagttt taagtgcgcg gaggggcgcg 4680
ggtttttatt acggcggtag cggttttcgc gcggtcgttt gagggttgta gcgtaaagaa 4740
tcgggttttc gggttcggac gcggggaagt attcgcgtgg gagtttgtgc gagcgcggtt 4800
cggggcgggg ttcgtacgga ggaggcgtcg ggattatggt tattttcggt tgagttttcg 4860
gcggcgattt tgattattgg taaataatta ttataataat atcgagtttt cgggtttgta 4920
tcgtacgtat tgatttcgcg agttcgtata cggtcgcgtc gttcgttatc gggttttgag 4980
cgttagtttt aaacgagtcg atggaaaaat ttaaaaattt tcgtatcgac gttttgttgg 5040
cggtggattt tttacgagtc gtttttgcgt agagcgcgtc gttggttttg gttacgtcgt 5100
tcgtcgtcgt cgtatttggt atcggaggtg gcggcggcgg cggcggggcg agcggcggga 5160
ttagcggtag ttgtagtttc gcgttttcgg agtcgtcggt tgcgttcgtc gatcgtttgc 5220
gcgtcgagag ttcgtcgtcg tcgcgtttgt tggtcgcgta ttgcgcgttg ttgtttaagt 5280
cgggtttttt gggcgcgggc ggcggcggcg gcggtacggg cggcgggtac ggggggtttt 5340
attattacgc gtattcgggc gtagcggtcg ttgtcgtcgt cgtcgtcgtc gtcgtcgtcg 5400
ttgggggttt ggcgttgggg ttgtattttg ggggcgcgta gggcggcgcg ggtttttcgg 5460
cgtaggcggc gttttacggt tattcggttt acggttattt cgcggcggcg gcggcggttg 5520
cgttggcggg ttagtattcg gcgttttttt attcgtattc gtaggtgtaa ggcgcgtatt 5580
tcgcgtattt cgtcgatttt attaagttgg gcgtcggtat tttttagttg gattagtggt 5640
tgcgcgcgtt tatcgcgggt atgattttgt ttaagatgtt cgattttaat tgtgagtatt 5700
ttgcggtttt cgtggatgcg cgtggcggga gggtttcgcg tattttttat cggtatcgtt 5760
tgtttttttt tgcgtttggt ttttagcgag cgggggaaga gttgcgaagt cgtttcggtt 5820
ttgttcgggg tttgcgagga ttcgttcgga tttttaggta tgagacgggt agcgaattat 5880
taaaatggtt atgcgtaggt tttgtttttt tttttttttt taacgggaag gggtagttgg 5940
aggtgtgaga ttgagattta atgattaatt cgaaggttgt aggagaagga agtcgtcggg 6000
ggaaggcgag tcgagagcgt tttgaattag gtaggttcgg gaatgggggt tagggtttat 6060
tatcggggaa aggaatttgg gttaggtttt ttattggggc gttagagaga ttcgcggttt 6120
ttttagggag agggagtgtt ttaggagtag taaataagag aaatgggttt cggaggttcg 6180
gggaggtaag gttaaggttt agggaatttt cggtttttat tagggcgttg tttttttttg 6240
gaagggcgtt ggattcgggg tgcgatggtc gaggtaggtg gtttggtttg aattagttat 6300

ggggggattt ttaatagtag gtgtttgttt tggaattttg ggcgtttaat aaggtaacga 6360
atgttttttt aaggtggggt cgagggagtt tttgtttttt ttattttaga gtcgttgggt 6420
ttcgggtagt ttttttttta ttcgggcggg atatttcgtt agaggtttgg gttttttagg 6480
tttttttggt atacgcggat ttttcgggtt ggggtttcgg ggttttggtt ttttggggtt 6540
ttggtttttt tcgttcgttt tatcggggtt agttatttat gagtcgggtc gagtagatgt 6600
atatggttta acgtttttag tcgtcgaata tttaaataaa gagattttat tgtttttgtt 6660
tgagtggatt tgtttagatt ggggagatgt aggtagagta ggggaaaagt aaaaaagaaa 6720
atagaaataa atattttaga atttagattt ttttgttgga agttgtggtt gtgattaaat 6780
agagaaaatt taggatttta gaggttggag aagttataag agggtgagaa gaatttttgt 6840
agaaggttgt ttgtttatag tcgttttagg tttttttagt ttagttttaa gaaggtttta 6900
gtggttattt atttttttatt tgggagagtt agttttattg attggttgga agagggaaag 6960
ttgtttttta ttttagatac gtttgcgttt gcgaattttt tgttagtttg ggtttaaatt 7020
tagggtgtcg atttagggtt tatttttttt gatagaaagt tattaatatg atttgtattt 7080
tttagagtta aggacgttaa gttgtttacg gagggggttg gttagtttcg ggggttcgtg 7140
gttttaggtt ttttaggggt gtttttttgga gtttcgtgga gtttagggtt atttagaaaa 7200
tttattaaag tgagatattt tgtttatttt tgattttaaa tgttgattta gagttggtta 7260
aattttaaat ataattagtt ttaagtttag aagaggtatt tgtatttggt aatttaaaag 7320
ggtaggtata atcgtgttaa agacggcgga atgtgttttg agaagttttt ttcgttggtt 7380
cggggaaaat ttgtttggtt tttttaattg gttgtttttg tttgttggat ggtatttttt 7440
aaatggtttt ttcggagtta gtggtttaat taaagtttta atgtattgtt aagttgggaa 7500
ttcggggttt tgtttgatta aagtaggggg cgtgggttat tttttaaagt aaattgttta 7560
tttcggggga tttgagttta tttaggtttg gggttggttt tttttttttt ttttgttttt 7620
ttttttttat tgtttaatat ttgttaggtt tttgggaaat taagggaagt ttcgttttga 7680
ggttataaaa ttttttgtga tgtaatcgtg cgaaattatg taaataaagt cgggtaggtt 7740
tttttgaata tatagagaat cggattttgt ttgcgttttt gtttgtaatt tgcgtaatga 7800
gaattgtttt gaaaaggttt ttagtttttt taggttttgt ttttgtcgtt cgtattttga 7860
ggagggggta tgttaaggaa aatatttgga aggtgggagg gtttttggtg aatatataga 7920
tttggatttt gggtttggtt ttgttttatt tgtttttttt tttttttttt tttttttttt 7980
tttttttttt tttttttttt tttttttttt tttttttttt tttttttttt tttttttttt 8040
tttttttttt tttttttttt tttttttttt tttttttttt tgttttaagg aggtagtgta 8100
gggagatttg gaatagtagg attagttttt tttagatttg tttggatttg agtttgaatt 8160
tttaggaagg cgtagtgagg agagggggag agatttggtt tttattattt attgtttttt 8220
tattttatta ttttattatt ttttatttat tgagagatgg ggttttgttg tgttatttag 8280
ggtatagtgt aggggtgtag tttcgtttat tgtagttttt aatttttggg tttttcggaa 8340
tttttttttt tggttttcga ttggttttag tttttttttt gtttttgggt taatttattg 8400
ttattaaagt cggtttttgt ggagaggaag ttttttgcga tgggttttgt ttaataggga 8460
tatttagacg ttttttagat aaaaaaaaaa aaaaaaattg gttttattaa ttttaaatta 8520
tttattttta tttggtggtt ttgagttcgt attttttggg gagtgggtat tgatagtgag 8580
ggaatagttt cgttttttgt agggttaggt tttggcgttg gggttttgag tgtagtggta 8640
taagtagtaa cgggaggggt tggggcgagg atcggagtcg gttgtgggtt ttattgtttt 8700
ttggttttta gt 8712

<210> 120
<211> 8712
<212> DNA
<213> Artificial Sequence

<220>
<223> chemically treated genomic DNA (Homo sapiens)

<400> 120

gttggggatt aaagggtagt gaggtttata gtcggtttcg gttttcgttt tagttttttt 60
cgttgttgtt tgtattatta tatttaaggt tttagcgtta aggtttggtt ttgtagaggg 120
cggggttgtt tttttattat tagtgtttat tttttaagga atgcggattt aggattatta 180
agtggaaatg gatagtttgg agttaatgag attaattttt tttttttttt ttgtttagga 240
gacgtttgag tgtttttgtt aaatagggtt tatcgtagga ggtttttttt ttataggagt 300
cggttttggt agtagtggat tgatttagag gtaagaggag aattgaggtt agtcgggggt 360
taggaaggga ggtttcggga agtttaggag ttgaaggttg tagtgagcga gattgtattt 420
ttgtattgta ttttgggtga tatagtaaga ttttattttt taataaataa aaaataataa 480
aataataaaa taaaggagta gtgagtgatg agaattaaat tttttttttt ttttttattg 540
cgttttttta gagatttagg tttagattta aataggtttg ggaagagttg gttttgttgt 600
tttaggtttt tttgtattgt ttttttaaga tagaaaagga ggaggaggag gagaaaaaga 660
ggaaggaagg aagggagaga gggaaagagg gagggaggaa ggaaagaagg aaagaaggaa 720
ggaaggaagg aaggaaggga ggaagggaag ggagagagga taggtggaat aggattaaat 780
ttagggttta agtttgtgta tttattaggg atttttttat tttttaaatg ttttttttgg 840

tatgtttttt ttttagggtg cgggcggtag gggtaaggtt tgaggagatt agaagttttt 900
ttaggataat ttttattacg taaattatag gtagaggcgt aggtaggatt cggttttttg 960
tgtgtttaag gaggtttgtt cggttttatt tatataattt cgtacggtta tattatagag 1020
aattttatga ttttaaaacg agattttttt tgatttttta ggagtttgat aagtgttgga 1080
taataaaaga aagaaagtaa aggaaagaaa ggaggttagt tttaggtttg gatgagttta 1140
ggtttttcgg gatgggtagt ttgttttaga ggatggttta cgttttttgt tttagttaaa 1200
taaggtttcg aatttttaat ttgataatat attagaattt taattaaatt attggtttcg 1260
gagggattat ttgggaaatg ttatttaata ggtagagata attaattaaa gaaattaaat 1320
aggttttttt cggattagcg aggaggattt tttagaatat atttcgtcgt ttttggtacg 1380
gttatgtttg tttttttgga ttgttaggtg tagatatttt ttttgaattt aagattggtt 1440
gtgtttaaaa tttggttaat tttaggttaa tatttggaat tagaaatagg taaggtgttt 1500
tattttaata aattttttga gtgattttgg gttttacgaa gttttagaaa gtatttttag 1560
gaggtttagg attacgggtt ttcggggttg gttagttttt ttcgtgggta atttagcgtt 1620
tttggttttg ggaaatgtag gttatattgg tggtttttta ttagaaggga tgggttttgg 1680
gtcggtattt tgggtttaag tttaggttgg taggggattc gtagacgtag acgtgtttga 1740
ggtgaggggt agtttttttt tttttagtta gttagtaggg ttgatttttt taggtgaggg 1800
atgggtgatt attggggttt ttttggagtt gagttaggaa agtttggggc ggttgtgggt 1860
aaatagtttt ttgtagggat tttttttatt tttttgtgat ttttttagtt tttggggttt 1920
tagatttttt ttgtttagtt ataattatag tttttagtaa aggaatttga attttggagt 1980
gtttattttt gttttttttt ttgttttttt tttgttttgt ttgtattttt ttagtttaaa 2040
taaatttatt tagatagaaa taatggggtt tttttgtttg ggtgttcggc ggttgaaagc 2100
gttgagttat atatatttgt tcggttcgat ttataagtag ttgatttcga tggggcgagc 2160
ggggagagtt agagttttag agagttagag tttcggagtt ttagttcgga gagttcgcgt 2220
gtgttaggaa agtttgaggg gtttaggttt ttggcgaggt gtttcgttcg gatggagagg 2280
aagttgttcg aagtttagcg gttttggggt ggggagaata ggggtttttt cggttttatt 2340
ttggaggggt attcgttatt ttgttgggcg tttaggattt tagggtaggt atttattgtt 2400
gagagttttt ttatagttgg tttaagttag gttatttgtt tcggttatcg tatttcggat 2460
ttaacgtttt tttagaggag ggtagcgttt tggtaagagt cgggggtttt ttgggttttg 2520
gttttgtttt ttcgggtttt cgaagtttat tttttttgtt tgttgttttt gaagtatttt 2580
tttttttttgg aggggtcgcg ggtttttttta acgtttagt ggggggatttg gtttaagttt 2640
tttttttcgg tgataagttt tgatttttat tttcgggttt gtttaattta gggcgttttc 2700
ggttcgtttt ttttcggcga tttttttttt ttgtagtttt cgggttaatt attagatttt 2760
agttttatat ttttaattgt tttttttcgt taagagagga agagaaggta aggtttgcgt 2820
atggttattt taataattcg ttgttcgttt tatgtttggg ggttcgggcg agttttcgta 2880
ggtttcgggt agagtcgagg cggtttcgta gttttttttt cgttcgttgg gagttaagcg 2940
tagagagggg taggcggtgt cggtggagga tgcgcgaggt tttttcgtta cgcgtattta 3000
cgggggtcgt agggtattta tagttgaagt cgggtatttt aggtaggatt atgttcgcgg 3060
tggacgcgcg tagttattgg tttagttgga aggtgtcggc gtttagtttg atggggtcgg 3120
cggggtgcgc ggggtgcgcg ttttgtattt gcgggtacga gtaggagagc gtcgggtgtt 3180
ggttcgttag cgtagtcgtc gtcgtcgtcg cggagtagtc gtagatcggg tggtcgtaga 3240
gcgtcgtttg cgtcgggagg ttcgcgtcgt tttgcgcgtt tttagggtgt agttttagcg 3300
ttaggttttt agcggcggcg gcggcggcgg cggcggcggt agcggtcgtt gcgttcggat 3360
gcgcgtggtg gtggggtttt tcgtgttcgt cgttcgtgtc gtcgtcgtcg tcgttcgcgt 3420
ttaggaagtt cggtttgggt agtagcgcgt agtgcgcggt tagtaggcgc ggcggcgacg 3480
ggttttcggc gcgtaggcgg tcggcgggcg tagtcggcgg tttcgaggac gcggggttgt 3540
agttgtcgtt agtttcgtcg ttcgtttcgt cgtcgtcgtc gttattttcg gtgttagatg 3600
cggcggcggc gagcgacgtg attaaggtta gcggcgcgtt ttgcgtagag gcggttcgtg 3660
gggggtttat cgttagtagg gcgtcgatgc ggaaattttt ggattttttt atcggttcgt 3720
ttggggttgg cgtttagggt tcggtggcgg gcgacgcggt cgtgtgcggg ttcgcggagt 3780
tagtgcgtgc ggtgtaagtt cgggggttcg gtattgttat ggtggttatt tgttaataat 3840
taaagtcgtc gtcggaaatt tagtcgaggg tgattatggt ttcggcgttt ttttcgtgcg 3900
ggtttcgttt cgggtcgcgt tcgtataaat ttttacgcga gtgttttttc gcgttcgggt 3960
tcgggagttc ggttttttgc gttgtagttt ttaggcgatc gcgcggaggt cgttgtcgtc 4020
gtggtgggga ttcgcgtttt ttcgcgtatt tgaagtttag cgcggttttc gattggcgcg 4080
cgtagcgggg gttcggggag ggtcgcgggt ttcgttattg gtcgaggggc gtatttgtta 4140
tcgtttcggt cgttcgtcgt ttcgttttcg cgtcgcgagg ttttaagtag ttcgggttgt 4200
tgcgcggcgt tggcgggcgg ggatattaac ggggagtgga tatttcgagg tttaatttgg 4260
ggtttttgtt cgtttttttt tgggtgcgga gtcggacggc gggtattggt aattttcggg 4320
tttttggaga gtttcgcggc ggtttaagat tcgttttcga gatacgtggg ttttttcgga 4380
tttggttggt ttttggagtt agttgcgcgc gtttttttcg gtttttttgt cggcgttgga 4440
gtgggtttac gtttgttttg cggagcgcgg tggttttacg tttttgagcg tttacgttgc 4500
gtagatttgg tcgttttgcg aggcgacggt agcgtattga agatcgtggt gttttttaag 4560
ttttttttgt tttcgggttt ttagggcgta gtaggggagg gttttgtttt cgtggttaaa 4620
gttttgtagg tcgaatttta tttgttatgg gaaggtttcg tatttttagg tgtgtgtgcg 4680
ggggtgtggg ggtgtaaacg cgtatgtgag tgtgagtttg gttttacgaa atttttttttg 4740
ggagtttttg ttttagttaa ggcggagacg gtagatttgg gcgttttggt ttgtagaatt 4800

taagtcggtt tgtagacgtc gtttacgtgt ttgtttagtg gagcgggttt tcggtgataa 4860
ggtttttttt cgttgtcgag ggatgtttat gggtttgcgg gagttaaggt ttatatattt 4920
cggttttgaa aaattcgtgg ggttcggtat ttttcggaag ttttgtcgtt gcgattttcg 4980
gttagttttc gggtaggagc ggggaaaggt tatgtttttc gtgttgtcgg ggttttttgg 5040
gaagtggaga ggaaagggtt tttcgtgagt gagcgaggta cggagtgagt atttaggttt 5100
tatttttttt ttatacgtat attttaaata tttatagtaa aattattttt aagggcgttt 5160
ttgaagttga aagaatttat ataggggtcg ggaagttaag cggtattaat attaggtttt 5220
gttaatagcg ttattagatt tagttatttt ttttagtaag cgtgagaatt gggagggagt 5280
tttaggtttt agggtttgtt atgtaggtga gaggtgaata aaggatcgga aggggttgtg 5340
gagaggggag agagttaaag agaaatttag tgttaggtaa attttaaaat tagtacgtta 5400
ggttgttata agagggattt ttaaaaaggt aaatatagag attttgttat ttttttttaa 5460
aaggtttttg atttaggcga gagttgatga tagtgatttt gttgttatga gtggtatcgt 5520
ttttattata atatagggta gtttttagat gtataggatt ggttggaagg ttaggaggat 5580
ggtaatggat gagtaattgt aatttggagt aaatgtgatt tggttttgta gagtcgatta 5640
ttggtaaaaa gaaattttag gtatttatat ggagtttgtc gtgtttttag atgtttttgt 5700
ggtgatataa atgtagtttt attttttatt ggagagaggg aaatacgcgt attttttgaa 5760
tatttattat gtgttatgag ttttttgtaa tttttattcg taatttttat tatgatatta 5820
gaaggaagat ttttattatt ttcgtatttt tatttttttt tttttttttt ttttttttgt 5880
atttttggta gagacggggt tttatcgtgt tagcgaggat ggttttgatc gtttgatttt 5940
ttgattcgcg cgtttcggtt ttttaaagtg ttgggattat aggagttagt tatcgcgtta 6000
ggcgtttttt attttttttt tttttttttg agatggagtt ttgtttttgt tgttcgggtt 6060
ggagtgtaat gttatgatta tggtttattg taatttttat tttttaggtt taagcgattt 6120
ttttgtttta gtttttttaa tagttgagat tataggtatg tgttattagg ttaggttaat 6180
ttttgtgttt ttagtagaga tggggtttta ttatgttggt taggttggtt tcgaattttc 6240
gattttaggt gatttattta ttttagtttt ttaaagtgtt gggattatag gtatgagcga 6300
tcgtgttcgg gttattattt ttatttttta gagtataagt ttagagagtt tgggattttt 6360
gtttttagta gatagtttgg ttttagagtt gggagtggga ttaggtttga ttgtagaggt 6420
tatattgttt ttattttgtt gtttttaaag tttttaggta tatttagttt tgagttttta 6480
ttttatttgt ttttttttgg ggagggaagg attgtaaagg agattagaga tttttagtta 6540
tttgttttag gtaggaagtt ttagttgtga ttttgttttt ttagggtaga gaatatagtt 6600
ttggtttttt tttttagggg tttggtatta aggttgattt gttttttttt ttttattatt 6660
tttatttgtt gggggtagat tgtttggttt tgaggttaat gatttaggga gttgggggaa 6720
gggagtggtt gtagaagaaa ttttttagga tggattttgg agaagatgtt gttttttttt 6780
ttaattaaaa atatatattg ttagggtgat tttagttggt gatggggtgt gtagtttaga 6840
tttatgatat tagttatttt ttttaataaa attatagtgg gtgttttttg gttttttttt 6900
ttaattttta gaggaattaa ttttattttt ttttgttttg taataattat ggtttaaaat 6960
ttttttatat tttgttttat gtttttattt tgtttgagat ttgttgagga tatttttttg 7020
aaaatataga taggataata cgtgtgtagg ttttttaggt gtttgtatta tttgtggggt 7080
tgtgtaaggt tgttcgagga ggtttagttt agtagttttg cgttttgttc ggatcgtttt 7140
tttggttgag gtgttatttt aggaatttga agttttttat ttcgatggat ttgttttttt 7200
gtttatagaa gtaggggttg gattaaatgg attttaagga gttatagggt tttaaaatga 7260
ttagatgttt tgtttatatt taagaatatg ttaatataaa aaggaggttt tttgtaaagt 7320
tgtatggaga gaggtaggtg tgggggagag agggagttat ttgggaagtg tggatttttg 7380
tgtggtttat aatgtgtgag gcggagtttg ttgggttttt ttttgttttt agtatttgta 7440
ttgatttagt ttagttggtt ttggggtagt ttggagttgg gtttgggttt tgggaagggt 7500
tataaagaga gattaaaggt ttgattttag tagtgttatt aaatttgagt ttttaggtga 7560
gttttttttt tagcggtttt aggggtagaa tttagttttg gagagttttt tttggagttt 7620
ttggtttttt aagataggta gagaggtttt ggtgtatttt aattagttgg tttttattat 7680
agtttttttt tgtttagcgc gggagtttgt tttttatagg ttttttgtta aggaattgaa 7740
gagtgtttgt agttgttttt ttttttatta tttgggaagg agatggtgat ttttataaat 7800
tattataaaa gaaaacgtcg ttgtagtttg tgtttgttta tatttggatt tatatatata 7860
attgggaatt cgtgaggatt ggggaaaaaa tttgtatatt tatatattta taagatgcga 7920
ggtaggtatt atgtatttat ttattattta gatattgttt tttttttatg tgaaatggag 7980
tagttgtttg gaagggtatt gggtggttga agtagggggt agattttgta tttttaaaat 8040
tgaagtttag ggttaataga gaggatttgg ttgtagaggg gtttaggtat attagttttg 8100
ttttgttgaa gttatgtggt tttcggggat ttatgtagtt tcgttttaat gtaaattgtt 8160
aaggtttttt ttaggaaata ggtatgaaga tgttgtgtaa ttggtcggag ggatttaagt 8220
acgagtttga gatgggattt gtttttggta taggaagtag tttagttagg ttggagtggg 8280
agtttagagg gttagagtag gaattgttgg gagatgtttc gatttttttt ttggagttta 8340
gagggaagga gaatttgtgg agagggttta ggtttatcgg agttggggat atttgtttag 8400
gtttttttat taattttagt ttttttagag taatgggggt taaaggtatt aagttattta 8460
tgttgttttt tgatttgttg atttttttta tggtttttgg gagttgggtt ttaaaggagt 8520
ttggaaaggg taggtttagg attagtaatt taaagatttg tagtttaaat ttttaaatcg 8580
aggcggggtt tgaggtgggt ttaggtttgt tgtgtgtttc gttttatggg tttttttttt 8640
tagttttttt tttttagttt ttttttgtaa tttggatttt ttgttgttat tttagagggt 8700
atttttattt cg 8712

<210> 121
<211> 6107
<212> DNA
<213> Artificial Sequence

<220>
<223> chemically treated genomic DNA (Homo sapiens)

<400> 121

tttaaaggta ttttatttag aggatagagt ttgttttttt tttttaaata atatgtagaa 60
attattttgg taagttaata tttgtggttg atatgaatat tttttttttt ttttttttaa 120
tttagggaaa attatagttt attttttgat attagtaaga taaaataata atgttttgtt 180
ttttagaatt taaatttgtc gttagtttgt atggtggttt ttttgatttt tttttaagga 240
tgagaattga atgaatattt gtataagtta ttttgttaaa gtttttttaa tattagattt 300
aatattttta ttatatattt tattataaaa tagtaaaaga gaatgtaata ataaagtagt 360
gatataaaat gatattattt atatttttaa aaatatagat tgaagatata attttattat 420
ttaaatataa ttaaggttat taatatcgta tttatttaat ttattttatt tttttatttt 480
aaaaatgtat gttttatttt tttttgtgga ttttgcggtg gagagataaa ttgtatgtag 540
gtgtatatag tattaaattt aaaatgttgt attttgtttt tttttttatt agtatttttt 600
tgagtttttg ttgaaggttg taatatgttg aattttttta tttttttata taaatatttt 660
ttgataaggt gtttttagtt aatgattttt ttaattgttt ttttcgtaaa taattggttt 720
tttgattatt taagaatttt gaaagttaat ataagtgttt gaaggtgtgt ttagtgttaa 780
gtagataaat tttgatggtt taattttttt tttttttttt tttttgagat tgagttttgt 840
ttttgttgtt taggttggag tgtaatggtg cgattttagt ttattgtaat ttttgttttt 900
cgggtttaag cgattttttt gttttagttt tcgaagtagt tgggattata ggtatgggtt 960
attatatttt gttaatttcg tatttttaat aaagatgggt ttttattatg tttgttaggt 1020
tggttttgaa tttttgattt aaggtgattc gttcgtttcg gttttttaaa gtgttgggat 1080
tataggtatg agttattgcg tttggttgat tttttagtat attttagaaa taataattta 1140
tttaaagaaa gtgaaatgtt gattataatt tttgatatac gatatgtttt ttttttattg 1200
agatttgaga tttatttaga gaatgaataa ggggtgtttg gatatttgtt tttttattaa 1260
ttaaaaagtt tataagaaag taaaaatgta tattttttta agattgtgtt gttatttttt 1320
tatttttttt gtttttattt tgtttatata tttgttttat gttgttttaa atacgttggg 1380
gaattttgag taaggttttg tttgggtatt tattttttga aaggttttag gaattggagt 1440
attaatggga tttttattgt tttttttttt aggtatttta tgggttttta tttaggaaaa 1500
ttgagttttg tttaatgatg ttttttttgt attggttggt tttatgtgat aaagagagtt 1560
ttagggagga tttttgttag gggaacgtag attgttttag taagatgaag gtttaggaga 1620
tttggatggt ttgggtgttt tttttttagg gagagtagag gaggtttgga tttgaattcg 1680
agttttatga ttttgttatt gaggtaatat ttaaaataaa aatagtatgt taattttaag 1740
tatagttttt agttataaag tagttttatt atagaagtcg gcggtgattt aataaaatgg 1800
aaagggaagg gttttgtagg tttcggtttt atatgagggt agattaaaga gggattggga 1860
gaaatatttg tagttcgagg ttttcgcgaa tttttttttg gagagaaatt tcgggagtaa 1920
tttttagtat ttaagtttga gttagaattt tggatagttt tttagaaatt agaatttcgg 1980
gaaggaggag ttttaaagat cgttgagttg gaagggaacg cgaattttag gttgtttgaa 2040
cgtttttgtt tttttcgtag ttcgttggtg tttcgttttt agttatttaa agttaatcgt 2100
agatttcgtt cgtattttta tgggtaggcg tgtcgggagg gtgcgggagc ggttgtttcg 2160
agattttagg aggcgttatt tagttttatt ttatcggagt ttttagaatt tcgttagggt 2220
gtaggaggtc gttttgggtt cgcgtagaat ttaggggttg gggttttaaa aaggtcgagt 2280
tttttcgaag gcgttttcgg agagattggg agagcggggg agaaggtgtt tcggtgtcgg 2340
agttgcgggg ttgggatcgc gggggtaaat gaggattcgg gtcggtttcg cgggtttggc 2400
ggtcgatagg gggcgttttg ttattgtgga agttttcgtc gagcgttttt tggtgttttt 2460
gatttcggtt attaatagtg ttgtggtgga ggtgggggtt tttaaagttc gttttttgga 2520
agtgtagcgt tttttttttt tgcggttttt tagttcgttt tttttgtttt tggaataatt 2580
tttgggatta aatttttatt taggagtaaa atagaaaggg aagtgggtat ttatataagt 2640
ttataaatat ttttatatag tatacgtata gattaggtta attataatat ttttttgagt 2700
tttgggggtg cggtgtgtgt gtgtgtgcgt gtgtgtttgt ttaggtttgc ggagaaagtt 2760
ttgaaaaatt gtttattttt tgagttttaa tgtttatttt tttttttttg ttttcggtgg 2820
agtgatgaag ttgtttttat ttacgcgtta ggtgggattt cgtgggaaat gttggggatt 2880
tagttttttt gatttcgttg cgtatatttt tacgtttatt acgtagtcgt atatattatt 2940
aagttttttt tttggcgtat cgggttggtt tgtttttcgt gggagttttt agtattcggg 3000
ggatcgatag gtacggtttt cgcggagata tagattttga atttgtggga gaatttcgtt 3060
ttggcgttta ttcggcgggt ttattaatgt tttttttatg aagtgtttaa attatttgta 3120
ttttagtagt aaaagatgtt gtcggcggtt tttttttgtg ttttaaatcg tgtagatttg 3180
agagttttgg ggttagggaa gtggtttttt tgtgtttatt gttatttttt cgtttgtttt 3240
ttagttttat ttttatttaa gttttaatgc gaagtttcgt gtttgttatt tcgtttgttt 3300

tttgttatta tcgtttttaa ttttggatag gtgggttgga tgtttattag tttttatgta 3360
ttttttgtgt ttgagggggt gggtataggg ttggattttt aaggtttagt taggttttta 3420
gaattaagaa agagttttta tatttaaata tttgtaatat ttttttattt taaatttggg 3480
tttataggtt aatttagaat agaagataat ttttgaattt aagagttgtt ggggaaataa 3540
aagtatacga ttgttggagt ttttaatttt tattaagtag tttttttgga agatagagag 3600
gatagagacg tttttgaagt taattttata tgttttatta ttgatttttg gatttttttt 3660
tttttatttt ttttttttta ttttttgttt tgtttgtttt agttacgaaa tgttgtgggt 3720
atttcggttg tgattgaaaa gtaattttga aatacgtcgg tttgaatatt agagataaat 3780
tttagttttt taatcgtcgg tcgttgttag aggggttgtt tgcgttaggc gtcggtcgtt 3840
ttattgcggg tttttggcgg tcggtgtttg aggagtcgga gagtcgaggc ggttagatcg 3900
tgcgtttcgc gtttttttcg aggtcgtttc gggtttgaat tgtaataggg aggggtttcg 3960
taggagtagt agcgggcgag ttaaaggtgt gtacgtagtt tttttaaata ggatagcgtt 4020
ttgtaaattg gttgttatgg atttgttgtt ttgatagaga atgagtattg agagcgaaga 4080
aattcggtat aaataaattc ggtttttata gggatttgtc gggtattgag gttggacgcg 4140
tttttcgata ggcgtatcgg gcgttcggcg cgatgagacg gggcgggggt tggaggtcgg 4200
agtcgtttta gagggtggtt tttttttagg tttttttttt tttaggggat tcgttgtgga 4260
ttcgggagga aagggggttc gattagggat ttcgcgtgtt cgatcgttcg tttagttcgt 4320
tttttttggtt cgtttaggag tgcggggcgt cgcggcggtg ggcggacggt taggcgttga 4380
ggggtaggtg cggagcgtcg gttgcggtta tttttttatt aggtgtgagt tgattattta 4440
aatgggttgt ttcgggtttg gggattggag gttcgcgtta gcgcgtcgcg ttatattatt 4500
agttttttac gttattgcgg tatttgttcg tttcgcggtt tatatttttt tttttttttt 4560
tttttttttt tttttttttt ttttttttat tattattatc gttttttttt tttttttttt 4620
tttttttttt tttatttttt tattagtttt cgcgtcgcgc gtttttcgtc gtttcggggt 4680
agttcggtat tgttaatttt agtcgttatt cggtagttcg tgagcgcggc ggcggcgggt 4740
gttttttatc gcggggcgtt ggtttgggtt tttttcgttt ttcgtttttt tttatttttc 4800
gtttattttt aattatcgtt ttcggtttgt ttagggagag agagggagtg gagtttaggg 4860
agagggagcg cgagagaggg agggaggagg ggacggtgtt ttggttgatt tttttttaaa 4920
agagggtggg ggtggggggt gattgttggt cgtttgttgt ggttgttaaa ttttaaattg 4980
ttatgtattc ggtttttagt atgttgggag cggtgaagat ggaagggtac gagtcgttcg 5040
attggagtag ttattatgta gagttcgagg taagcgttcg ttttttttag ggggaagtgg 5100
agcggcggcg ttcgtttttt ttttaatttt aggatcgttt ttttcgttgg tattttttta 5160
tttcgtttta taattttagt ttttttgggt agtcgttttt ttcgtttttt ttggaattaa 5220
atgggttttt tttttatacg atatattgtt agttttgaga taatattaat tttgtgatta 5280
aatattgatt tgcggcgttt ttaaattttt ttcgtggtcg agttacgtat tattttaata 5340
gagttatgtt tggtagcgcg cggttgggga ggggtgggag gtcggtcggc gagggggtgg 5400
ggagttgagg aggagaagat gggtaggagt tgggggttga agtggaggta gagttaaaga 5460
ttagtggata aagagaaaga aagtttttgt ttgtaaagtt tagtcgcgtg tggtttaggt 5520
ttcggttttt tttttattgt ttcgtttagt gtttttagtc gttttcgtat ttcgtatagg 5580
tattgttttg tagttttttt gggataaggg agggggtttt tggagggaga tcggatagag 5640
ggggttcggg atagttttcg ttggaggtag ttcgaggcgt ttcgcgcgtt gtttcgtagg 5700
ttgtttgttg cgatcggagg tggcgttcgg ggatttcggg tttcggcgtt agtttagtat 5760
gttggtggga gagtttgggg agattttggg gtaggggaat gtatttgggg tgttggtttg 5820
ggagtatttt atgggatttt tgttttagaa tttgggcgag gtggagaagg gttaggaacg 5880
ggttatatgt ttaagtagta attcgttcgt tattttttta agggcgatcg attatgattt 5940
tttttgaatg aaatagattc ggagttcgga gattgggtta tcggggacga aagggttttt 6000
cggaggtttt ttcggacgcg gaggtttggg cggtgggtgg gaggtttggg tattttttag 6060
tcgcggtgtt aacgttgtcg tttttttttc ggggttttgt tcgtagg 6107

<210> 122
<211> 6107
<212> DNA
<213> Artificial Sequence

<220>
<223> chemically treated genomic DNA (Homo sapiens)

<400> 122

tttgcggata gagtttcggg agggaggcga tagcgttagt atcgcggttg gagggtgttt 60
agatttttta tttatcgttt aggttttcgc gttcggggag gttttcgggg agttttttcg 120
ttttcgatgg tttagttttc ggatttcgag tttgttttat ttagggagag ttatgatcga 180
tcgtttttgg gaaggtggcg agcggattgt tgtttgggta tgtggttcgt ttttagtttt 240
tttttatttc gtttagattt tagaataggg gttttatgga gtatttttaa gttagtattt 300
taaatatatt tttttatttt agagtttttt taaatttttt tattagtata ttgagttggc 360
gtcgaggttc gaagttttcg gacgttattt tcggtcgtag taagtaattt gcgggataac 420
gcgcggggcg tttcgggttg tttttagcga agattgtttc gggttttttt tgttcggttt 480

ttttttaggg attttttttt ttgttttagg gaaattgtaa ggtagtgttt gtgcggagtg 540
cgggggcggt tggaggtatt gaacggggta atagggaaag aatcgagatt tgaattatac 600
gcggttgagt tttgtaggta aaaatttttt ttttttttgt ttattagttt ttgattttgt 660
ttttatttta gtttttaatt tttatttatt tttttttttt tagtttttta ttttttcgtc 720
ggtcgatttt ttattttttt ttagtcgcgc gttgttaaat ataattttgt taggatagtg 780
cgtggttcgg ttacgaagag gatttggagg cgtcgtaagt taatatttga ttataaagtt 840
aatattattt taaggttaat agtgtgtcgt ataaaaaaga gatttatttg attttaagga 900
gggcggaaaa ggcggttgtt tagaaaggtt ggggttgtgg ggcggggtgg gggggtgtta 960
gcgagggaag cggttttgag gttggggaag gagcgagcgt cgtcgtttta tttttttttg 1020
gaaaagacga gcgtttattt cgggttttgt atagtagttg ttttagtcgg acggttcgtg 1080
tttttttatt tttatcgttt ttagtatatt ggaagtcgag tgtatggtag tttaaaattt 1140
aatagttata ataaacgatt agtaattatt ttttattttt attttttttt aaaaaaaagt 1200
tagttaaagt atcgtttttt tttttttttt tttttcgcgt tttttttttt tgggttttat 1260
tttttttttt tttttgggta ggtcggaggc ggtagttgga agtggcgggg aggtgggggg 1320
gggcgaggag cggaggaggt ttaggttagc gtttcgcggt agggagtatt cgtcgtcgtc 1380
gcgtttacgg gttgtcgggt ggcggttgag gttggtagtg tcgagttgtt tcgaggcggc 1440
gggaagcgcg cggcgcgggg gttagtgggg gggtggggag gaggaggagg aaggaggagg 1500
aggaggcggt ggtggtggtg gaggaggagg aggaggagga ggaggaggag gaggaggagg 1560
aggtgtggat cgcggagcgg ataagtgtcg tagtgacgtg ggaggttggt gatatagcgc 1620
ggcgcgttgg cgcgggtttt taatttttag attcggggta gtttatttga ataattagtt 1680
tatatttagg tgagaggtag tcgtagtcgg cgtttcgtat ttgtttttta gcgtttgtcg 1740
ttcgttttat cgtcgcggcg tttcgtattt ttgggcgggt taggggagcg ggttgggcgg 1800
gcgatcgggt acgcgggatt tttggtcgag tttttttttt tttcgggttt atagcgagtt 1860
ttttgaggaa ggagggattt gggaggaaat tattttttgg ggcggtttcg gtttttagtt 1920
ttcgtttcgt tttatcgcgt cgggcgttcg gtgcgtttgt cggagggcgc gtttagtttt 1980
agtgttcggt agatttttat gaaagtcgga tttatttatg tcgggttttt tcgtttttag 2040
tgtttatttt ttgttaaaat agtaagttta tgatagttaa tttgtaaagc gttgttttat 2100
ttagaaaaat tacgtatata tttttaattc gttcgttgtt gtttttgcga ggtttttttt 2160
tgttatagtt tagattcgga acggtttcgg gagaagcgcg gggcgtacgg tttggtcgtt 2220
tcggtttttc gattttttag atatcggtcg ttagggattc gtagtggggc ggtcggcgtt 2280
tggcgtaagt agttttttta gtagcggtcg acggttggga ggttgagatt tgtttttgat 2340
atttaggtcg gcgtgtttta aggttatttt ttagttataa tcgaggtgtt tatagtattt 2400
cgtaattaaa ataaataggg taggaggtgg ggagggaggg gtgaggagag aagaatttag 2460
gaattaatga tggggtatat ggagttgatt ttaagagcgt ttttgttttt tttgtttttt 2520
agagggattg tttaatagaa attagaaatt ttagtaatcg tatattttta tttttttagt 2580
agtttttggg tttaaaaatt gttttttgtt ttgggttagt ttgtgagttt agatttggag 2640
tggggggata ttgtaggtgt ttggatgtgg aggttttttt ttggttttga aaatttggtt 2700
ggattttggg gatttagttt tgtatttatt tttttagata tagagaatgt ataggaatta 2760
gtgggtattt agtttatttg tttaaaattg ggggcgatgg tggtagaaga taggcgagat 2820
ggtagatacg gagtttcgta ttgaagtttg agtaagggtg aggttggaag gtaagcgagg 2880
aggtagtagt aggtataggg gaattatttt tttaatttta ggatttttag gtttgtacgg 2940
tttgggatat agaaggaagt cgtcggtagt attttttgtt gttgaaatgt agatggtttg 3000
gatattttat aaagagggta ttaataagtt cgtcgagtga gcgttagggc gggatttttt 3060
tataaattta aggtttgtat tttcgcggaa gtcgtgttta tcggtttttc gagtgttgaa 3120
ggtttttacg aggggtaggt tagttcggtg cgttagggaa ggagtttgat ggtgtgtacg 3180
gttacgtagt gaacgtggag gtgtgcgtag cggagttaga ggggttagat ttttagtatt 3240
ttttacgggg ttttatttag cgcgtggatg ggagtaattt tattatttta tcgagggtag 3300
ggaaagagga gtaagtattg gagtttaaag aatgagtaat tttttaaagt ttttttcgta 3360
ggtttgggta gatatatacg tatatatata tatatcgtat ttttaaaatt taaaagaatg 3420
ttatggttga tttagtttgt gcgtgtgttg tatgagagta tttgtgagtt tatgtgggtg 3480
tttatttttt tttttatttt gtttttgggt ggaaatttgg ttttagaaat tattttagga 3540
atagaaggag cgagttggag ggtcgtagga gaagggggcg ttatattttt aaggagcgga 3600
ttttggaagt ttttattttt attatagtat tattggtggt cggggttagg gatattagag 3660
agcgttcggc gggggttttt atagtgatag ggcgtttttt gtcggtcgtt aggttcgcgg 3720
gatcgattcg agtttttatt tattttcgcg attttaattt cgtagtttcg gtatcggagt 3780
attttttttt tcgttttttt agttttttcg aaaacgtttt cggggggatt cggttttttt 3840
ggagttttaa tttttaggtt ttgcgcgaat ttaaaacggt tttttgtatt ttggcgaggt 3900
tttgggagtt tcggtagggt agggttggat gacgtttttt agagtttcgg agtagtcgtt 3960
ttcgtatttt ttcggtacgt ttgtttatgg gaatgcgagc ggggtttacg gttagtttta 4020
ggtagttggg agcgggatat taacgggttg cgaaggaagt agggacgttt agatagtttg 4080
gggttcgcgt ttttttttag tttagcggtt tttggagttt ttttttttcg gagttttgat 4140
ttttaaaggg ttatttagag ttttggttta ggtttgaatg ttgaagattg ttttcggagt 4200
ttttttttag gaaaaagttc gcggggattt cgggttgtag atgttttttt taattttttt 4260
ttagtttatt tttatgtagg gtcgagattt atagagtttt ttttttttta ttttgttagg 4320
ttatcgtcgg tttttataat aaagttgttt tataattgag gattgtgttt ggggttagta 4380
tgttattttt attttggatg ttgttttaat ggtaaagtta taagattcga gtttaaattt 4440

aggttttttt tgtttttttt gggaagaaaa tatttaggtt atttaggttt tttaggtttt 4500
tattttgttg gaataatttg cgttttttta gtaaaagttt tttttagagt tttttttgtt 4560
atatgaaatt aattagtgta gaaggggtat tattgaatag aatttaattt ttttaagtga 4620
gaatttatga aatatttaag gagaaaaata ataaaagttt tattaatatt ttaattttta 4680
aaatttttta gagaataaat atttaggtaa ggttttgttt aaagtttttt agcgtgttta 4740
gggtaatata aggtagatat gtaggtaaga tgaaggtaga ggggatggaa aagtgatagt 4800
atagttttgg gaaagtatgt atttttattt ttttataaat tttttaattg gtaaaaaagt 4860
agatgtttaa atatttttta tttatttttt ggatgaattt taagttttaa tgaagaaaag 4920
gtatatcgtg tgttaaagat tataattaat attttatttt ttttgggtgg gttattattt 4980
ttgaaatgta ttgaaaaatt agttaggcgt aatggtttat gtttgtaatt ttagtatttt 5040
gggaggtcgg ggcgggcgga ttattttagg ttaggagttt aagattagtt tgataaatat 5100
ggtgaaaatt tatttttatt aaaaatacga aattaatagg atgtggtggt ttatgtttat 5160
aattttagtt atttcggggg ttgaggtagg agaatcgttt gaattcggga ggtagaggtt 5220
gtagtaagtt gagatcgtat tattgtattt tagtttgggt aataagagta aaatttaatt 5280
ttaaaaaaaa aaaaaaaaaa aaaattagat tattaaaatt tgtttatttg gtattgagta 5340
tatttttaaa tatttatgtt ggtttttaaa gtttttggat ggttaaaaga ttagttgttt 5400
acggagaggg taattgaaaa ggttattagt taagaatatt ttattagagg gtatttatgt 5460
aaaagaataa aaaaatttag tatgttgtag tttttagtaa aggtttagga aaatgttgat 5520
aaagggggag gtaaggtgta atattttaag tttaatattg tgtgtatttg tatgtaattt 5580
atttttttat cgtaagattt ataggaaaag atgaaatata tatttttaaa atgagaaaat 5640
aaaatgaatt aaataaatac gatgttagta attttgatta tgtttgggtg gtagaattat 5700
atttttaatt tatgttttta gaagtatggg tggtattatt ttatattatt attttattat 5760
tatatttttt tttattattt tatggtgaaa tatgtaataa ggatattgga tttgatgtta 5820
aggaaatttt ggtaaaataa tttgtgtaaa tatttattta gtttttattt ttgaaaggag 5880
attagagaag ttattatata aattgacgat aggtttgagt tttgaaaaat aaggtattat 5940
tattttgttt tattggtatt aggggatgaa ttgtgatttt ttttgagttg agagaggaag 6000
agaagaaata tttatgttaa ttataggtat tgatttatta gggtggtttt tgtatattgt 6060
ttaggggaag gagatagatt ttgttttttg ggtggggtgt ttttaag 6107

<210> 123
<211> 12639
<212> DNA
<213> Artificial Sequence

<220>
<223> chemically treated genomic DNA (Homo sapiens)

<400> 123

gggtatggag atggatgttt gtaattttag ttatttggga ggttgaggta tgagaattat 60
ttgagtttgg gaggtagacg ttgtagcgag tcgagattgc gttattgtat tttagtttgg 120
atgatagagt aagattttgt tttaaaaata aaataaaata aaaataaaga aaggaaagaa 180
aataaaaata ggacgggtat agtggtttat aattgtaaat ttagtatttt ggtaggttta 240
ggtgggagga tggtttgagg ttaggagttt aggattagtt taggtttaaa aattaaaaaa 300
gaaggttagg tgtaggggtt tatgtttata attttaatat tttgggaggt tgaagtagga 360
agagtatttg aggttaggag tttgaaatta gtttgggtaa tatagcgata tattattttt 420
agatatatgt ataattagtt gggtatcgat attatgttat tgttttttag tttgggagat 480
acggtaagat tttatttaaa aaaaaaaaaa aaaaaaaaaa aaaattagtt aggtatggtg 540
ttatgaattt ttagttttag ttattttgga ggttaaggtg ggaggattgt ttgagtttgg 600
ttgtagtgaa gtatgattgg gttatcgtag tttaatttgg gtaatagagt atgaatttga 660
tgaatttgtt tttaaaaaaa aaaaagaaaa aataattagt taatatatat tgaatacggt 720
cgggtacggt ggtttaagtt tataatgtta gtattttggg aggttaaggc gggtggatta 780
ttcgaggtta gtagtttgag attagtttgg ttaatatggt gaaattttat ttttattaaa 840
aatataaaaa aattagttag gtatggtggt aggtatatgt aattttagtt attcgggagg 900
ttgaggtagg agaatcgttt gaatttggga ggaggaggtt gtagtgagtc gaggttatat 960
tattgtattt tagcgtgggt aataagagtg aaattgcgtt ttaaattaaa aaaaaaaaga 1020
aaaaaaattt atatatatag agagagaaag agaaagagag agagagaata tttaatatgt 1080
gttaagtata attttaagat atatatatta atttatttaa attttagata ttaaggttta 1140
aatgaattaa tatgtatata ttagaattaa tatatatgag ttaggtttat attttttgtt 1200
tttttgagat agggtgttat tttgttgttt aagttggagt gtagtgtcgt aattttagtt 1260
tatcgtaatt tttgtttttc gggtttaggc gattttttta ttttattttt ttaagtagtt 1320
gagattatag gcgaattttt atttaatttt tgtatttttt gtagagaagg gtttcgttat 1380
gttgtttagg ttgggagtta gtatttttaa tttttatttt atagatgagg aaattgaaga 1440
ttagggaggt aaagtgattt gattaaaggt atatagtttg taagaggtag agttagtagt 1500
atggttgtat atttttttta ttattatgtt atagtattgt ttatttgaaa ggaattgttt 1560
tttattttta ggtttgttta cgtaagttaa ggattttggt tattagataa tcggtatggt 1620

atgtcgtttt tagttaaaat ataaaaaaaa taggtcgggt atggtggttt atatttgtaa 1680
ttttaatatt attgtatttt agtttgggcg atagaatgag attttgtttt aaaaaataaa 1740
ataaaataaa ataaaatttt ataataaata tttttatttt ttttatgata atatatttag 1800
attaattaaa atattttatt ttgttttatt ttttttttgt tttttggttt atgtttttag 1860
ttttgttttg ttttttttta aatatatata tataaaattt ttttgtagga gttagatttt 1920
atgtaaaaaa aggaaaaaaa atgaaaataa aaaaatttat atatatatat ttttttttta 1980
tttataaaaa gagtatatat agggtcggga gcggtgattt acgtttgtaa ttttagtatt 2040
tcgggaggcg gaggtaggtg gattacgagg ttaggagttt aagattagtt tgattaatat 2100
ggtgaaattt tatttttatt aaaaatataa aaattagtta ggcgtggtgg tgtgtttgta 2160
attttagtta tttgggaggt tgaggtaggc gaattgtttg aatttggaag gtagaggttg 2220
tagtgagtcg agattacgtt attgtatttt agtttgggtg atagagtgag attttgtttt 2280
aaaaaaaaaa aaaaggagtt tatataggtc gagtttggtg gtttaggttt gtaatttttg 2340
tattttggaa ggttgaggtg ggtagattat ttgaggttag gagtttaaga ttagtttggt 2400
taatatagag aaatttcgtt tttattaaaa atataaaaat tatttgggtg tggtggggta 2460
cgtttgtaat tttagttatt cgagaggttg gggtgggaga attatttgaa ttagggaggt 2520
ggaggttgta gtgaattggg atcgtgttag tttgggcgat agagtgagat tttgttttaa 2580
aaaaaaaaaa aaagaaaaag tatatataat aaatattaat ttttatttta tttgtttttt 2640
tttaatatat gttggaggtt attatattag tgtatagaga tttttattat ttttttttat 2700
ttattttatt tttttttaga gttgtatagt attttttatg tggatgtatt atagtttttt 2760
ttagttaatt tttttattta agaatatttg ggatgcgtgt agttttttgt tatgatgaat 2820
agtattattg tttttttttt tttttcgatg tattcgttaa gaagtggtat tttgttttat 2880
tgttatttag gaatttgatg attattttgt ttttttaaat tggttttagg agtatttttt 2940
atttttttaa cgaatattag ttgggcgttt tcggcgtgat tggcgttgtg ttgagaatat 3000
agtggcgata gagataaatt tagtttttat ttttatggaa tatacggtta tagatattat 3060
aaaatagtat atagataaga gtcggataat aaattagatt atacgtgata tgaaggaata 3120
cggatcgata aaagtattta ataggcggat ttttggatgg agggtaggga aggagggttt 3180
cgtagaggaa gtgacgttgg tgtcgagatt cgaaggatga aatgggagtt agttaagtcg 3240
aatgggtaaa aggatgttgt aggtagaagg gatagtttgt gtaaaatttt cgaagcggga 3300
aagcgtatgg taagtttaag gaatcgaaag gttagggcgg ttgttagagg tgggggaaaa 3360
agtaaagagg tcgagtcgaa gtaagcgagg tttgcggggc ggtggcgttc ggggggttac 3420
gttggaattt tgtttcgagg gtattaggga gttaaggaag ggttttgagt aagggaagaa 3480
atatgggtcg cgaaagttat tgtttttttt tttttacggc gatgtttttt tcgttcgagt 3540
ttttgttttt ttaggagttt ttttttttcg ttttcgggtt taaacgtcgt ttttcgttta 3600
tcgtagtttt ggttttcgtt tttttcgcgt ttttttagta gtgttttttt ttatatttcg 3660
tcgtcgtcgt cgtcgttatt tttcgttcgt cgtcgggaac ggtgcgtacg cgttatatat 3720
ttagggtcgt cgggaaatgg agtttagagt atagttcgat ttcgtttatg tttcgtttcg 3780
tgcgagcggt attttgttcg tttttatatt acggcgagta gattttttat tttcggtttt 3840
gggttcggtt aggttttttg ggaaatgtag ttttttcgga ggtatcgggc ggaaagggag 3900
ttagagtatt ttgggaaatg gagtttttag tggtgttgag tttttggacg gttggttggt 3960
tttggagagt gcggagttcg ggtagcgaag tttttgggtt ttggagggga ggtggtttgt 4020
tgttttagtg gggtttgcgg agaggtttcg atttttttta tttttgtttt tgattttttt 4080
aatagtttga agtatttttt tttttttttt tttttttttt tttttttttt tggaaacgga 4140
gtttcgtttt tttttttagg ttggattgta gtggcgcgat ttcggtttat tgtaattttc 4200
gttttttagg tttaaacgat ttttttgttt tagtttttcg aatagttggg ataataggta 4260
cgtattatta cgtttagtta gttgttgtat ttttaataga gatggggttt tattttgttg 4320
gttaggttgg ttttaaattt ttgattttaa gtgattcgtt cgttcgtttc ggttttttgt 4380
agtgttggaa ttataggcgt gagttatcgc gttcggttaa gttaagtatt tttagtcgtc 4440
ggaagtttta gtttttttgt ttgtttaatg gggatgtgat ttcggggatt ttttacgggt 4500
ttttgtagtc gtatagtggt tttagcgttt tgattttttt ttttttcgtt acggttatgt 4560
agggttttgg tttttcgttt ttttttgtcg agatgatgaa gttttttgag gtagaaaggt 4620
tttagagttt tgatttcggg gtttttggta tttgtagcgt cgttgcgggt agttttttta 4680
tttttgaatt atttttagtt gtcgcgtttt ggggttgcgg ttttcggcgt ttgaggtttg 4740
ttttttggcg atttcgtagg gggcgtttta gttcgagcgc gtcgttttcg gggaggcggg 4800
aggggagttc ggggcgggcg agggcggggg tgtttcggtt ataaagcgtg gtcgtttttc 4860
gcggcgttcg ggatagtcgt atttcgggcg gtcggacggg cgggcgtcgg tgggagttcg 4920
ggtcgtgttc gttgagagat ttagagcgtt tcgttttttc ggggtcggag cgggggcggg 4980
tgggggcgta agttcggggg atgttgggtt tagtgaagat ggaggtttat gatttggtcg 5040
agtggagtta ttattcggag gcgggcgagg tgtgttttcg gggatggcgg agcgggaagt 5100
tgggggtagg tatcggggtg tgggtttata tggagtaggg attggttgtg ggtttaaatg 5160
gaggtaaagg cgtttatttg ggtagttgag gaattgggaa tacggagatt aggtgtggaa 5220
gtcggggatc ggggacgagg tatggatggg agcgttgttt gggtagtttc gtcgcggtta 5280
gggtttaaaa atggaagtta ggaaaattta gttaagggtc gggttatatt ttaaatttgg 5340
gagtcgggtg ttggagtttt aaaaagtaag ggatttatat gtataaatta aagataggaa 5400
ttagagtttt agggtagggg atagattagg gggttttagg atgtgaggtt taaagttgga 5460
gggaaagaaa gattaaaaga tggaggagga gttattatta aggggaagat tgaggtggga 5520
gtttagggtt agtatatagt gttaaagaat cggggttcgg ggttaaggat tagttggtaa 5580

tttaaggtag ggttagatat agggattggt gtttaggggt tagagatggg ggtgtttaga 5640
gatgaagttt cgtttttgga gtttgaaata ataggtggtt tagagttggg tatttgggag 5700
tagaagatgt agttattagt attggtagga gaggggtcga tttttatttt tttattttta 5760
tttatgattt ttcgtaggga ttttttaagt cggaagatgg agagttttag attgtgtatt 5820
tttttttgtt tttcgttagt ttagtaattt ttagttattg ttaaaatttt tatttagttt 5880
tattttagat aaggtttttt tggagtgggg ggtggcggta tattttaatt tttttatttg 5940
gatagataga aagttttttt ttaagtttga tttttattcg ttttgttgta acgattttta 6000
tattttagtt attttatttt gtaattaacg ttgtgaggtt gttggggttg ggggttgggt 6060
ttttggtttg agaattatgg gggtttggat atttggattt ttaatttgga aggggattgt 6120
gggtttgagg tggggtgtgg ggtaggaatg gttaggtttt ttaaggagtt aagatttggt 6180
gattgcgggg gttttagtgt gtttgagttt tgggtttggg gttcgaaatt gtagggtggg 6240
atttttggat ttttaggttt ttagggttgg gtaaggtgtt tgggaagggt tagttttggg 6300
tttggggagg tgtgaggtgt acgtggggtg gggtagggtt aatagggaaa gggattggga 6360
gtgttgagag gtggaggtag ggtggttgga ggtattagtt gtgagatgcg gaaggtagag 6420
atttaggtgg gaaatggagg gttttaggta gtgggggtga ttttagttgg atttttgtag 6480
aatttagttt ggggatttga tttaagggaa ttatttatag attaattgta taaattaatt 6540
atagttgttt tgggggtata ttttaagtaa atgatggggg tttaggtagg aggagttgat 6600
atttggggta tagtttgaat agatttaagt ttaggggata atttgtatgg aaattgtgtt 6660
agaaattgaa ggaaagtcgg tatagtggtt tatatttgta attttagtat tttgggaggt 6720
cgaagtggga ggattttttg agtttaggag tttgaggtta gtttgggtaa tatagggaga 6780
ttttgttttt aaaaaaaaaa ttttttttta attagttagg tatgtttgta gtttaagtta 6840
tttggttggg aggttgaggt gggaggatta tttgaggttg ggagttcgag ttgtcgggag 6900
ttgtgatcgt attacggtat tttagtttgg gtgatagaat gagattttat tttaaaaaat 6960
aataatagta gagggatagt ttggaggtaa ttagtatttt agggtatatt ttgggagagt 7020
tagtatttgg aggaatggaa atggttgggt gatattgttt gaggtttttt ttagtttttt 7080
tattagataa gtttattttt tttttttggg ttagaataat ggggagagaa atatttatat 7140
ttagttagta attgagagtt gagagggttt ttaaattatt ttttgtttat agtatttttt 7200
cgtttggggt gacgttaata tttagggtag tttgttgttt ggaaagaatt tggaaagtta 7260
ttattttggg atatttttgt tggttttagg tttttgtaga ttagttggtt tagttgtttt 7320
tttgttttag aggtggttaa ttttgaagtt tcgtacgtag aatattgggg ttttgggttg 7380
ggtttgggat tagaaatttg gtttttggtt ttatattgtt tttttttgtt ttttgttata 7440
ttttttttta gagataataa ggaaatgaaa gaggagaaat tttagttttt tggtgtgggg 7500
gtgcgggttt tttaaggttt tttttttaat tttagtttta ttttgttagg ggagattcgg 7560
tttagattcg gatattaggg gtttatttta gtttcggggc gtgggaaagt ttagggtgga 7620
ttagggtagt tggagtgttt gaggtcggat tttaaggagg attttcgggt gggtggggag 7680
ttagaaagga gggttttggg gtttttgttt gttttatttt gtaatattag agagtttgat 7740
tgggttgtag aaatgggggg tggaggtata gggggatttg gaaattttgt ggattttttt 7800
aatttattat ttttttattt tatttttttt ttttttttgg ggaggggatt cgaagttatt 7860
aattagcggg agagtttgta tgggggaagg gagtaattgt ggttgtaatt ttattgtaag 7920
atttggggtg agggggaatg gatttgttga agggagggag atagatagat agatagatag 7980
atagatagat agatagatag atgtatggat ggatggatag atagattgat aggaagatgg 8040
tggttcgtta tggtagttta tgattgtaat tttagtattt tgggaggtcg aagagggtgg 8100
attatttgag gttaggagtt taagattagg ttggttaata tggtgaaatt ttatttttat 8160
taaaaatata aaatttagtt gggtatggtg gtgtatgttt ataattttag ttatttggga 8220
ggttgagata ggagaattgt ttgaatttag gaggcggagg ttgtagtgag tcgagatgga 8280
gttattgtat tttagtttgg gtgatagagt gagattttat tttaaaaaaa aaaaaaaaaa 8340
aagataagaa gatgggttga aggaggaaga aaggtgggag ttggggttgg gaggggagtg 8400
gggtagagtc ggatagtgag agagagtgtt taggaaggga gaaagagatg gaaggatgga 8460
gagagagata ggggtcgaga tggaggggaa agaaagatgg gagaggattc gggaggggtc 8520
gagggtgggt aggaagagta aaagaggtta gggtggagat agaagggaaa ataatgcggg 8580
ggtggggagg gagatgggag aggggaatat gggtattagt attgagacgg gtaatataac 8640
ggtaaaaata gttaatagag atttaaagag atatgatgaa agagtttttt ggagagatat 8700
gaaaaggaaa gagtagagga tttttcgatt ggaatttaga tttagggttg agttggttgt 8760
ttttattttt tgatttttta agttattttg gttagattcg ttttttcgga ttttgtgatt 8820
tgtagaagtt tatagtggta gggttagata tggtggttta tggttataat tttaatattt 8880
tgggaggtcg aggtaggagg attatttgag gttaggagtt cgagattagt ttgattaata 8940
tggtgaaatt ttgtttttat taaaaatata aaaattagtt gagtgttgtg gtgcgtgttc 9000
gtaattttag ttgttcggga ggttgaggta ggagaatcgt ttgaatttgg gaggtagaag 9060
ttgtagtgag ttgagattgt attattgtat tttagtttgg gcgatatagt gagatttttt 9120
taaaaaaaaa aaaaaaaaaa agtttatagt ggtaggagat taggcggtat ttagttaatt 9180
tatatttggt ggttttagtt agtgttgtat atagtagtat ttttttattt acgtagtgtt 9240
atgagatttt aggtttcgga ggaggtagtg gggaaaggtt taggtggaga atggggtaga 9300
gaggatggga gatagtggtt gagaagggat aagtagatat aaagaaagaa ataatttttt 9360
agtatgttta tgtttttttg gcgtgttttg tttattgttg tgtttttgtt agattgttgg 9420
tattacgagg gtaggtattt ttgtttgttt tagttattgt ttttatttta ttttatgatt 9480
attatttttt gagatggagt tttgttttgt tgtttaggtt ggaggtagtg gtacgatttt 9540

ggtttattat aatttttgtt ttttgggttt aagtaatttt tttgttttag tttttttagt 9600
agttgggatt gtaggtatgt gttattatgt tcggttaatt ttgtattttt agtggagacg 9660
gggttttttt ttgttggtta ggttggtttc gaattttcga ttttaagcga ttcgtatttt 9720
tatttttttt attttagttt tttaaagtgt tgggattata ggcgtgagtt attgatgttt 9780
gagtttatta tgtagttgat tatattgaac gataggtacg tatatatgtt atattatgta 9840
tcgtgttatt acgagagtta tatttatata tttttgtatg tatgtatgtt tgtaattttt 9900
gttttatatc ggtttttgtt atagacgtta acgttgtgcg gttaattttg ttcgtttttt 9960
ttataagggg gggtggtaag attaaaggag attatttttg tgaagtgttt ggagtagtta 10020
attgataaat attggttgaa tgaatgaaag aatatataga gggttattta ggaggtattg 10080
taagatttat cgtgttttat tgatttagga tgtatttttt ttttttattt gaatattttt 10140
aaaaatggga tgtattttat agttaatagt gttttatgat tttaattgag tgtttttttt 10200
atttgtatta atatataaag taatgttgta gtttttagag aagttttaga tttgaagata 10260
tatagtaaat ggataatatt attaggttga aaagtgattt ttgatattta aatattttta 10320
tgtataaata tagtaattta tttttattta tttatttttt ttttttgaga tagagttttg 10380
ttttgtcgtt taggttggag ggtagtggtg taatttcggt ttattgtaat ttttattttt 10440
tgggtgtaag taattttttt gttttagttt tttgaatagt tgggattata ggcgttcgtt 10500
attatatttg gttatttttt gtgtttttag tagagatagg gttttgttat gttggttagg 10560
ttggtttcga atttttgatt ttaagtgatt tatttgtttc ggttttttaa agtgttggga 10620
ttataggtgt gagttattgt gtttagttta aatgtagtaa ttttataagt taaaagattg 10680
aagagagtta tatatatatg ttagatatgt tagattatta tagagttata ataatttttt 10740
tttttttttt ttgagataga attttattgt cgtttaggtt ggagtgtagt ggtatgattt 10800
tagtttattg taagttttat ttttcgggtt tacgttattt ttttgtttta gttttttaag 10860
tagttgggat tataggcgtt cgttattata ttcggttaat tttgtttttg tatttttagt 10920
agagataggg ttttatcgtg ttagttaaga tggtttcgat tttttgattt tgtgatttat 10980
ttgtttcggt tttttaaagt gttgggatta taggtgtgag ttatcgtgtt cggtttataa 11040
taatttttta tgattattaa aattgagaat tgttgattta tttaataaat tagtagttat 11100
taaatgtttt ggttttagga ttattttttt tttttttttt ttttttttga gatggagttt 11160
tgttttgttg ttttggttgg agtgtgtagt ggtatgattt tagtttatcg tagttttaga 11220
tttttgggtt taagcgattt ttttgtttta atttttcgag tagttgggat tatagttttg 11280
tgttacgatg tcggttgatt tttgtatttt tggtagataa tagggttttg ttacgttgtt 11340
aggttggttt tgaatttttg attttaagtg atttattttt tttggttttt aaagttttgg 11400
gattattttg ggaggttgag gtaggaggat tattcgaggt taggagtttg aaaatagttt 11460
gggtaatatg gtgaattttc gtttttatta aaaatataaa aaaattagtt gggtgtgcgg 11520
tgtatgttta taattttagt tatttgggag gttgaggtag gaggattatt tgagttttag 11580
agtttgaggt tatagtgagt tgcgattgta ttattgtatt ttagtttgag tgatagagtg 11640
agattttgta gaaaaaaaaa attatttagg agtttaaata gtttttgttg atgtgggtta 11700
tgtttattta cgtttatagt atttataatt aaaattgaga aatattataa atatgtattt 11760
atttaaaaat agtaattatt tgttagtgta aaagattttt attaaaaaaa ttaaatttta 11820
aaaaatttag ttaaaagagc gatattgttt ttttttattt ttgtaaatta agtttggttt 11880
agtggaagat agttggattt ttttggttgt ttttttattt tttttgttgg aatattaata 11940
ttttttggga aaattttatt gtatatttta tgagaaagag agtgaaaagg gttaataata 12000
atttaggatt gcgggtgtgg tggtttatgt ttgtaatttt agtattttgg gaggtcgagg 12060
cgggtagatt acgaggttag gagtttaaga ttagttcggt taatatggag aaattttgtt 12120
tttattaaaa atataaaaaa aaaaaaaaaa aagttggcgt ggtggtatat atttgtaatt 12180
ttagttgttt tggaggttga tgtaggagaa ttggttgagt ttaggaggcg gaggttgtat 12240
tgaggtgaga ttacgttatt gtattttagt ttgggcgata gagtaagatt ttgtttcgaa 12300
aaaaaaaata gtattatttt gaaaatagtt ttgatttcgt ggatattttt aaaggttttt 12360
agagattgtt ttgggagttt cgaattttgg agaattattt aaaggatgat gttgagtgta 12420
tgggagtatg gtgggtggga aagtaggggg tagaaagggc gtgtttaggg agtgaggaga 12480
gattttgaga gattgttttt tatagataga ggtaatgatg gaagggattt gtagagaagg 12540
taagatggat agatagacgg atatttagtg ggttttggga ttttttgttg attagtagag 12600
tggagtttta ttgatttttt tttttatttt tttttttagg 12639

<210> 124
<211> 12639
<212> DNA
<213> Artificial Sequence

<220>
<223> chemically treated genomic DNA (Homo sapiens)

<400> 124

tttaggggaa agatgaaagg aggggttagt ggggttttat tttgttggtt agtaaaggat 60
tttaggattt attgagtgtt cgtttgtttg tttattttgt tttttttata aatttttttt 120
attattattt ttatttgtag aaagtagttt tttaaggttt ttttttattt tttggatacg 180

ttttttttgt tttttgtttt tttatttatt atgtttttat atatttaata ttatttttta 240
agtgattttt tagggttcgg gatttttagg gtagtttttg aaattttttg aggatgttta 300
cgaagttaaa attattttta aaataatgtt attttttttt tcgaggtaga gttttgtttt 360
gtcgtttagg ttggagtgta gtggcgtgat tttattttaa tgtaattttc gttttttggg 420
tttaattaat ttttttgtat tagtttttag agtagttggg attataggtg tgtgttatta 480
cgttagtttt tttttttttt tttttgtatt tttagtagag atagggtttt tttatgttgg 540
tcgggttggt tttgaatttt tgatttcgtg atttgttcgt ttcggttttt taaagtgttg 600
agattatagg tatgagttat tatattcgta attttaagtt attattagtt ttttttattt 660
ttttttttat gaagtgtata gtggagtttt tttagggggt gttgatattt taatagagag 720
aatgaagaag taattaggag aatttagttg ttttttattg ggttagattt gatttgtaaa 780
aatggaaaaa aatagtgtcg tttttttgat taaatttttt gaaatttggt ttttttaata 840
aaaatttttt atattaataa atgattgtta tttttaaatg aatatatgtt tgtaatattt 900
tttagtttta attgtaaata ttgtaaacgt agatagatat aatttatatt aatagaaatt 960
gtttggattt ttaaataatt tttttttttt atagggtttt attttgttat ttaggttgaa 1020
gtgtagtggt gtaatcgtag tttattgtag ttttaaattt tggggtttaa gtgatttttt 1080
tgttttagtt ttttaagtag ttgggattat aggtatgtat cgtatattta gttaattttt 1140
ttgtattttt agtagagacg gggatttatt atgttgttta ggttgttttt aaatttttgg 1200
tttcgagtaa tttttttgtt ttagtttttt aaagtaattt tagaattttg ggagttaagg 1260
agggtgaatt atttgaggtt agaagtttaa gattagtttg gtaacgtggt aaaattttgt 1320
tatttattaa aaatataaaa attagtcggt atcgtggtat aggattgtaa ttttagttat 1380
tcgggaggtt gaggtaggag gatcgtttga gtttaggagt ttgaggttgc ggtgagttga 1440
gattatgtta ttgtatattt tagttaggat aatagagtaa gattttattt taaaaaaaaa 1500
aaaaaaagaa ggggggtggt tttgagatta aaatatttaa taattgttgg tttattaggt 1560
aaattagtag tttttaattt tagtaattat aaagagttat tataggtcgg gtacggtggt 1620
ttatatttgt aattttagta ttttgggagg tcgaggtagg tggattataa ggttaggaga 1680
tcgagattat tttggttaat acggtgaaat tttgttttta ttaaaaatat aaaaataaaa 1740
ttagtcgggt gtggtggcga acgtttgtag ttttagttat ttgggaggtt gaggtaggag 1800
aatggcgtga attcggaagg tggagtttgt agtgagttga gattatgtta ttgtatttta 1860
gtttgggcga tagtgagatt ttgttttaaa aaaaaaaaaa aaaagttatt ataattttgt 1920
aataatttaa tatatttaat atatatgtgt agtttttttt aattttttag tttataaaat 1980
tattgtattt aggttgggta tagtggttta tatttgtaat tttagtattt tgggaggtcg 2040
aggtaggtgg attatttgag gttaggagtt cgagattagt ttggttaata tggtaaaatt 2100
ttgtttttat taaaaatata aaaaatagtt aggtatggtg gcgggcgttt gtagttttag 2160
ttatttagga ggttgaggta ggagaattgt ttgtatttag aaggtggagg ttgtagtgag 2220
tcgagattgt attattgttt tttagtttgg gcgatagagt aagattttgt tttaaaaaaa 2280
aaaaataaat aaataaaaat aaattattgt atttatatat aaaagtgttt gaatattaga 2340
aattattttt taatttaata atattattta tttattatat atttttaaat ttaagatttt 2400
tttggaagtt gtagtattat tttatatatt aatataaatg ggaaaaatat ttagttaaaa 2460
ttataggata ttgttgattg taaaatgtat tttatttttg gaaatgttta aatgggggga 2520
aaaggtgtat tttaggttaa tggaatacgg tagattttgt agtgtttttt aagtaatttt 2580
ttgtatattt ttttatttat ttaattaata tttattagtt agttgtttta agtattttat 2640
aagaatggtt ttttttaatt ttgttatttt tttttgtaag gggagcgaat agggttaatc 2700
gtatagcgtt gacgtttgta ataaagatcg atgtaaaata aaggttgtag gtatgtatgt 2760
atataaaagt gtgtaaatat agttttcgtg ataatacgat atatagtgta gtatatatgc 2820
gtatttgtcg tttaatataa ttagttgtat aataggttta ggtattagtg gtttacgttt 2880
gtaattttag tattttggga ggttgaggtg gggggggtgg ggatgcggat cgtttgaggt 2940
cgggagttcg agattagttt gattaatagg gagaaatttc gtttttatta aaaatataaa 3000
attagtcggg tatggtggta tatgtttgta attttagtta ttagggaggt tgaggtagga 3060
gaattgtttg aatttaggag gtagaggttg tggtgagtta agatcgtgtt attgttttta 3120
gtttgggtaa tagagtaaga ttttatttta aaaaataata attataaaat aaaataaaag 3180
tagtgattaa gatagataga aatgtttgtt ttcgtggtgt taatagttta gtaggaatat 3240
aatagtgaat agaatacgtt aggaaaatat aggtatgttg gaaggttgtt tttttttttg 3300
tatttattta ttttttttta attattgttt tttatttttt ttgttttatt ttttatttgg 3360
attttttttt attgtttttt tcgaggtttg gggttttatg gtattgcgtg ggtgggggaa 3420
tgttgttata tatagtattg gttagagtta ttaaatgtga gttaattgga tatcgtttag 3480
ttttttgtta ttgtaagttt tttttttttt ttttttttaa gagagtttta ttgtatcgtt 3540
taggttggag tgtagtggtg taattttagt ttattgtaat ttttgttttt taggtttaag 3600
cgattttttt gttttagttt ttcgagtagt tgggattacg ggtacgtatt ataatattta 3660
gttagttttt gtatttttag taaagatagg gttttattat gttggttagg ttagtttcga 3720
atttttgatt ttaggtgatt tttttgtttc ggttttttaa agtgttggga ttatagttat 3780
gagttattat gtttggtttt attattgtaa gtttttgtag gttataaggt tcggagaaac 3840
gagtttggtt agggtggttt agggaattag agagtggagg taattaattt aattttaagt 3900
ttgagtttta gtcgagaaat tttttgtttt ttttttttta tgttttttta aggaattttt 3960
ttattatgtt tttttgaatt tttgttggtt atttttgtcg ttgtgttatt cgttttagta 4020
ttagtattta tatttttttt ttttattttt ttttttattt tcgtattgtt tttttttttg 4080
tttttatttt aatttttttt gttttttttg tttattttcg atttttttcg agtttttttt 4140

tatttttttt ttttttttat ttcggttttt gttttttttt ttattttttt attttttttt 4200
ttttttttgg gtattttttt ttattattcg attttatttt attttttttt taattttaat 4260
ttttattttt tttttttttt tagtttattt ttttgttttt tttttttttt ttttttgaga 4320
tagagtttta ttttgttatt taggttggag tgtagtggtt ttatttcggt ttattgtaat 4380
tttcgttttt tgggtttaag taattttttt gttttagttt tttaagtaat tgggattata 4440
ggtatgtatt attatgttta gttaagtttt gtatttttag tagagatggg gttttattat 4500
gttggttagt ttggttttga atttttgatt ttaagtgatt tatttttttc ggttttttaa 4560
agtgttggga ttatagttat gagttattat gacgagttat tattttttta ttaatttatt 4620
tatttattta tttatgtatt tatttattta tttatttatt tatttattta tttatttatt 4680
tttttttttt taataaattt attttttttt attttaagtt ttatagtgaa attgtagtta 4740
tagttatttt ttttttttat gtaggttttt tcgttggttg gtggtttcgg gttttttttt 4800
tagaaaaaga aaagggatga ggtgagaagg tgatggatta gaggggttta tagagttttt 4860
aaattttttt atatttttat tttttatttt tgtaatttag ttaggttttt tggtattgta 4920
gagtaagata gatagggatt ttagggtttt tttttttggt tttttattta ttcggaagtt 4980
ttttttgagg ttcgatttta agtattttag ttgttttaat ttattttagg tttttttacg 5040
tttcggggtt gggatgaatt tttggtgttc gggtttgagt cggatttttt ttgatagggt 5100
ggggttgaga ttggggggaa gtttttgggg gattcgtatt tttatattag gagattggaa 5160
tttttttttt tttatttttt tattgttttt ggagaagggt gtgataggga gtagaggaag 5220
gtagtgtgaa gttagaagtt aagtttttag ttttagattt aatttaggat tttagtgttt 5280
tgcgtgcggg gttttagagt taattatttt taaaataggg gagtagttgg attagttgat 5340
ttgtaagaat ttgaggttaa tagggatatt ttaggatggt aattttttaa gtttttttta 5400
gatagtaagt tgttttgagt gttaacgtta ttttaagcgg gagggtgtta tgggtaagaa 5460
gtggtttggg gattttttta gtttttagtt gttggttgag tgtaggtatt ttttttttta 5520
ttgttttgat ttaaggggaa gaggtgggtt tatttgatgg gaaggttggg gagagtttta 5580
gatagtatta tttaattatt tttatttttt taaatattga tttttttaaa atatgttttg 5640
aaatgttgat tatttttaga ttgttttttt attattatta ttttttgaga tagggtttta 5700
ttttgttatt taggttggag tgtcgtgata cgattatagt tttcggtagt tcgaattttt 5760
agttttaagt gattttttta ttttagtttt ttaattaagt agtttagatt ataggtatgt 5820
ttgattaatt aaaaaaaaat ttttttttta aagataaggt ttttttatgt tgtttaggtt 5880
ggttttaaat ttttgggttt aagagatttt tttatttcga ttttttaaag tgttgggatt 5940
ataagtgtga gttattgtgt cggttttttt ttaatttttg atataatttt tatataaatt 6000
gttttttaaa tttggattta tttagattgt gttttaaata ttaatttttt ttgtttagat 6060
ttttattatt tatttaagat gtgtttttaa aataattgta gttggtttgt ataattggtt 6120
tgtaggtggt ttttttaaat taggttttta gattgagttt tgtagaggtt tagttgggat 6180
tatttttatt atttggaatt ttttattttt tatttgggtt tttgtttttc gtattttata 6240
attgatattt ttagttattt tgtttttatt ttttaatatt tttagttttt ttttttgttg 6300
gttttgtttt attttacgtg tattttatat ttttttaaat ttaggattgg ttttttttag 6360
gtattttgtt tagttttgaa ggtttgggga tttaggagtt ttattttata gtttcgagtt 6420
ttagatttaa gatttaggta tattggggtt ttcgtagtta ttagattttg attttttgga 6480
gaatttagtt atttttgttt tatattttat tttaagttta tagttttttt ttaggttaga 6540
agtttaagtg tttagatttt tataattttt agattaagaa tttaattttt agttttaata 6600
attttataac gttgattata gagtgaggta gttaaggtgt aggggtcgtt gtagtaagac 6660
gaatgggggt tagatttgag gaaggatttt ttgtttgttt aggtgagggg attagaatat 6720
gtcgttattt tttattttag ggaagttttg tttgaggtga gattggatgg gaattttagt 6780
aatagttgga ggttgttgga ttggcgggga gtaggggaag gtgtataatt taaaattttt 6840
tatttttcga tttggggagt ttttgcggag ggttatgggt agggatgggg gggtggagat 6900
cggttttttt tttgttaatg ttgatggtta tattttttgt ttttagatgt ttaattttaa 6960
gttatttgtt attttagatt ttaaaaacga agttttattt ttgagtattt ttatttttga 7020
tttttgagta ttaattttta tgtttgattt tattttagat tattagttga tttttagttt 7080
cgaatttcgg ttttttaata ttgtgtgttg gttttgagtt tttattttag tttttttttt 7140
aatggtgatt ttttttttat tttttagttt tttttttttt ttaattttga attttatatt 7200
ttgaaatttt ttagtttgtt ttttattttg gaattttgat ttttattttt ggtttgtata 7260
tataaatttt ttgttttttg gggttttaat attcggtttt taagtttaag gtgtgattcg 7320
atttttggtt aaattttttt gatttttatt tttaagtttt ggtcgcggcg aggttgttta 7380
aatagcgttt ttatttatgt ttcgttttcg gttttcgatt tttatatttg gttttcgtgt 7440
ttttagtttt ttagttgttt aagtagacgt ttttgttttt atttgaattt ataattagtt 7500
tttgttttat gtgagtttat atttcgatat ttgtttttaa tttttcgttt cgttattttc 7560
gaggatatat ttcgttcgtt ttcgggtagt agttttattc ggttaggtta tgggttttta 7620
tttttattga gtttagtatt tttcgggttt acgtttttat tcgttttcgt ttcggtttcg 7680
ggggaacgga gcgttttgga ttttttagcg ggtacggttc gagtttttat cggcgttcgt 7740
tcgttcgatc gttcggggta cggttgtttc gagcgtcgcg ggaggcggtt acgttttata 7800
gtcgggatat tttcgttttc gttcgtttcg ggtttttttt tcgttttttc gagggcggcg 7860
cgttcggatt ggggcgtttt ttgcgggatc gttaggagat agattttagg cgtcgggggt 7920
cgtagtttta ggacgcgata gttgggggtg atttaggagt gagggagttg ttcgtagcga 7980
cgttgtagat attaagggtt tcgaggttag aattttggag tttttttatt ttagggggtt 8040
ttattatttc ggtaggagag gacgaagagt tagagttttg tatgatcgtg gcggaagaaa 8100

ggggaattag aacgttaggg ttattatgcg attataagga ttcgtaaaag gttttcgaaa 8160
ttatattttt attggataga taggaagatt gaggttttcg acggttagaa atgtttaatt 8220
tggtcgggcg cggtggttta cgtttgtaat tttagtattg taggaggtcg aggcgggcgg 8280
acgaattatt tgaggttagg agtttgagat tagtttggtt aatagggtga aattttattt 8340
ttattaaaaa tataataatt agttaggcgt gatggtgcgt gtttgttatt ttagttattc 8400
gggaggttga ggtaggagaa tcgtttgaat ttaggaagcg gaggttgtag tgagtcgaga 8460
tcgcgttatt gtagtttagt ttgggagaga gagcgggatt tcgtttttaa aaaaaaagaa 8520
agaaagaaaa gaaaagaaaa agaaatgttt tagattgttg aaagggttaa gaataaagat 8580
ggaggggatc ggggtttttt cgtagatttt attagggtaa tagattattt tttttttaag 8640
atttaggagt ttcgttgttc gaatttcgta ttttttaaga ttagttagtc gtttagggat 8700
ttagtattat tgagaatttt attttttaga atgttttagt ttttttttcg ttcggtgttt 8760
tcgaagggat tatatttttt agaaggttta gtcggattta gagtcggaaa tgaagggttt 8820
gttcgtcgta gtatggaggc gggtagaatg tcgttcgtac gggacgaggt atgggcgggg 8880
tcgggttgtg ttttggattt tatttttcgg cggttttggg tgtgtggcgc gtgcgtatcg 8940
ttttcggcga cgaacggaag atggcggcgg cggcggcggc ggagtgtagg aagaggtatt 9000
gttgaggggg cgcgagggga acggaggtta gagttgcggt gagcgggagg cggcgtttgg 9060
gttcggagac gaaaggagga ggtttttaaa gaagtagggg ttcgggcggg gagggtatcg 9120
tcgtggggag aggggaatag tggttttcgc gatttatgtt tttttttttg tttaggattt 9180
ttttttggtt ttttagtgtt ttcgaggtaa agttttaacg tggtttttcg ggcgttatcg 9240
tttcgtagat ttcgtttgtt tcgattcggt ttttttgttt ttttttttat ttttaatagt 9300
cgttttggtt tttcggtttt ttaaatttgt tatgcgtttt ttcgtttcgg ggattttgta 9360
taggttgttt tttttgtttg taatattttt ttatttattc ggtttggtta atttttattt 9420
tatttttcgg gtttcggtat taacgttatt ttttttgcga agtttttttt ttttgttttt 9480
tatttagaaa ttcgtttgtt aaatgttttt gtcggttcgt gtttttttat gttacgtgtg 9540
gtttaatttg ttattcgatt tttatttgtg tgttgtttta tgatgtttat gatcgtgtgt 9600
tttatgaagg tagggattag gtttgttttt gtcgttattg tgtttttagt atagcgttaa 9660
ttacgtcgga gacgtttagt taatgttcgt tgaaggaata aaagatattt ttagagttag 9720
tttggggaaa tagggtagtt attagatttt taggtagtag tggggtaggg tattattttt 9780
taacgagtat atcgagaggg aaggggaaat agtgatattg tttattatag taaaaggtta 9840
tacgtatttt aaatgttttt aaataagaag attggttgaa agaaattata atatatttat 9900
ataaaaaata ttatgtaatt ttaaaaagga atgaggtaga taaaaggaaa tgatgaaaat 9960
ttttatatat tgatatagtg gtttttaata tatattaagg gaaaataagt gaagtggagg 10020
ttggtgttta ttgtatatgt tttttttttt tttttttttt tgagataggg ttttattttg 10080
tcgtttaggt tggtacgatt ttagtttatt gtaattttta tttttttggt ttaagtgatt 10140
tttttatttt agtttttcga gtagttggga ttataaacgt gttttattat atttaggtaa 10200
tttttatatt tttagtagag acgggttttt tttgtgttgg ttaggttggt tttgaatttt 10260
tgattttaag tgatttgttt attttagttt tttaaagtgt agggattata ggtttgagtt 10320
attaaattcg gtttgtgtag gttttttttt tttttttttg agatagagtt ttattttgtt 10380
atttaggttg gagtgtagtg gcgtgatttc ggtttattgt aatttttgtt ttttaggttt 10440
aagtaattcg tttgttttag ttttttaggt agttgggatt ataggtatat tattacgttt 10500
ggttaatttt tgtattttta gtagagatgg ggttttatta tgttggttag gttggttttg 10560
aatttttgat ttcgtgattt atttgttttc gtttttcgaa gtgttgggat tataggcgtg 10620
agttatcgtt ttcggttttg tgtatgtttt ttttgtaaat gaaagggaaa tatgtatata 10680
tgggtttttt tatttttatt tttttttttt tttttgtata aaatttagtt tttgtaggag 10740
aattttgtgt atgtgtgttt aaaaaagaat aaaataaaat tgaaagtata aattaaaaag 10800
tagaagaaga atgaaatagg gtaaaatatt ttggttgatt tgaatgtatt attatgaaaa 10860
aagtggaaat gtttattatg gggttttatt ttattttatt ttattttttg aggtagagtt 10920
ttattttgtc gtttaggttg gagtgtagtg gtgttgggat tataggtgtg agttattatg 10980
ttcggtttgt tttttttatg ttttgattgg ggacggtatg ttatgtcggt tgtttggtgg 11040
ttaggatttt tggtttacgt aaatagattt gggggtaaaa aataattttt tttagataaa 11100
tagtattgta gtatagtggt aaggaagata tgtagttatg ttgttgattt tgttttttat 11160
aagttatatg tttttggtta ggttatttta tttttttggt ttttagtttt tttatttgta 11220
aaatgaaaat taaaaatatt aatttttagt ttgggtaata tggcgaaatt tttttttata 11280
gaaagtataa aaattaaatg gggattcgtt tgtagtttta gttatttggg aaaatgaggt 11340
aggaggatcg tttgagttcg ggaggtagag attgcggtga gttgagattg cggtattgta 11400
ttttagtttg ggtaatagag tgatattttg ttttaaaaaa ataaaaaatg taaatttaat 11460
ttatgtatat taattttaat atatatatat taatttattt aaattttggt gtttgaggtt 11520
taaatgagtt aatatgtata ttttagaatt gtgtttagta tatattaagt gttttttttt 11580
tttttttttt tttttttttt tgtgtgtgtg ggtttttttt tttttttttt ttaatttgaa 11640
acgtagtttt atttttgttg tttacgttgg agtgtaatga tgtgatttcg gtttattgta 11700
attttttttt tttaaattta agcgattttt ttgttttagt ttttcgagta gttaggatta 11760
tatgtgtttg ttattatgtt tggttaattt ttttgtattt ttagtagaga tgaggtttta 11820
ttatgttggt tagattggtt ttaaattgtt aatttcgggt gatttattcg ttttggtttt 11880
ttaaagtgtt ggtattatag gtttgagtta tcgtgttcgg tcgtgtttaa tatatattaa 11940
ttgattattt tttttttttt ttttttggaa ataggtttat taggtttatg ttttgttgtt 12000
taggttggat tacggtggtt taattatatt ttattataat taggtttaaa taattttttt 12060

attttagttt ttagagtagt tgggattaaa ggtttatggt attatgtttg gttaattttt 12120
tttttttttt tttttttttt ttagatggag ttttatcgtg ttttttaggt tggagagtag 12180
tggtatgata tcgatgttta gttaattgtg tatatgttta gagatggtgt gtcgttgtgt 12240
tgtttaggtt ggttttaaat ttttggtttt aagtattttt tttgttttag ttttttaaaa 12300
tgttgggatt ataggtatga atttttgtat ttggtttttt tttttagttt ttaggtttag 12360
gttagttttg aatttttggt tttaagttat ttttttattt agatttatta aaatgttggg 12420
tttataattg tgagttatta tattcgtttt attttgtttt tttttttttt ttttgttttt 12480
gttttgtttt atttttgaga tagagttttg ttttgttatt taggttggag tgtagtggcg 12540
taatttcggt tcgttgtaac gtttgttttt taggtttagg taatttttat gttttagttt 12600
tttaagtagt tgggattata ggtatttatt tttatgttt 12639

<210> 125
<211> 6380
<212> DNA
<213> Artificial Sequence

<220>
<223> chemically treated genomic DNA (Homo sapiens)

<220>
<221> unsure
<222> (3793)

<400> 125

gggtcggcgc ggtggtttac gtttgtaatt ttagtatttt ggaggtcgag gtaggcggat 60
tatttgatgt taggagttta agattagttt ggttaatatg gcgaaatttt atttttaata 120
aaagtataaa aattagtcgg gtgtggtggt atatgtttgt aattttagta tacgggaggt 180
tgaggtagga gaaaaaataa ttttggaggc ggaggtgtag tgagttaaga ttatattacg 240
taaatagttt gtggaggagt agatattttt aaaattggtt tattattttt ttttttttag 300
gttttttttt agagaaggaa gtttagatga agagaaattt gtatttaaat ggtatttttg 360
tttttggatt ttatttggtg attttttttg tgaattttaa gtgtttttta aagatttttc 420
gtaggttatt ttttttgata tttttgtttg gtatattagt tttattttga ttttgattat 480
agattttttt ttatattgag ttttgtttat ttattttaag gatatttggt ttgttttttt 540
gtttggggat tttatttttt ttttttgtta gttaaatgtt ggtttagaat tttttaatta 600
agtaattgtt ttgtagtttt taggttgttt tgttatattt ttttttggta tagttttttt 660
tacggtgtaa gtgtgtagta agattgagat ttaagttata tttattaggt tgggagggtt 720
attttgtaat taattgttat tattttgagt atttggtgag agttaatagt atgttgggta 780
ttaaattaag gattttatat gtattatttt agtgtatgtt tatattttta agagatagat 840
tgtgtagtgg tgcgatttcg gtttattcgt aatttttttt ttttaggttt aagttatttt 900
tttgtttagt tttttgagaa gttgggatta taggtatatt ttattattgt ttggttaatt 960
tttgtatttt taatagagat ggggtttcgt tatgttggtt aggatgattt taaatttttg 1020
attttaaatg atttatttgt tttagttttt taaagtgttg ggattttagg cgtgagttat 1080
tgtgattggt taggtttttt tatttttatt ttatagatga agaaataggt aaagatgtta 1140
aataatttgt ttaaggttat ttagttagga attgggattg gagtttcgtg tatttttatt 1200
attgaattgt attgtttgtt atagttttta ttagtaatta aggtttagtt aagttttttg 1260
tttaggatta gttttattgg atttttttta gagaaaatgt taggttaggg ttaaggttta 1320
tatttagaaa gttgagggag ggagtagatg gtaattttag ggggattttg ttatagtgga 1380
gttagggata ggaggttaaa aggattgtag ttgagttttg ttttgttttt tgttatttga 1440
gtgaacgtgg ttaagtttga tttttttgaa ttttagtttt tttatttgtg atatggggat 1500
ggtagtagaa gttatttttt agaggttatg aggaataaat gaggaaatgt gtaaggtagg 1560
attgtattaa ttgtaaagag ttgtttgatg taagtgattg ttagtgttag tagttattta 1620
atatttatta tgaattatta ggatcgaatt taggtgttgt gagtatagtt atgagttttt 1680
gggtttggat ttgtttttaa gatgataaat tgtaagttaa ataagaatat agttattttg 1740
ttatttttcg ttttttatgg gttttttgtg atttttttga agttatgatt tgggttttgg 1800
ttattttttt ttatttgtgt aaattgaagg tttttagttt tttaatagtt tagatagttt 1860
ttaattttaa tattttaggg gtggaatggg aggcgggttt ttgtgatatt tgttaaagta 1920
gaattgttgt taaaaagggg tttttttttt tatgtaatga ataaaaattt tagtgtttcg 1980
tgtttgtgta tgttaagatt tattgttgaa atttttgtta atttatttat ttaaatgaat 2040
tatatttatt taaatttatt ttaaatttta aaagtaaatt aaaaatgtat ttttgttgta 2100
agaaatatag atgaaataat ttttttaatt ttagaattat ttgtagaggt tattgttgtg 2160
gttaatttat tgtgtatttt tttagattat tttttacgta tttatttata tgtatatatt 2220
atttaaggag aaatatacga tttttttgtt gggtttttat ttttgtattt gagtattttt 2280
gtttttttgt aatttgtttt tttagtgagc ggtggtttac gtttgtaatt ttagtatttt 2340
gggaggtcga cgtaggcgga ttatggggtt tggagtttga gattagtttg gttaatatag 2400
tgaaatatcg tttttattaa aaatataaaa attagttagg tatggtagcg ggtgtttgta 2460

attttagtta ttcgggaggt tgaggtagga gaattatttg aatttgggag gtggaggttg 2520
tagtgagttg agatcgtgtt attgcgtttt agtttgggtg atagagttag attttatttt 2580
aaaaaaaaat gtatttattt atttatatat atggtttgga atgaaaatat ggtatgtata 2640
tttttttata ttagaatatg gaggtttatt ttattatttt taattgtttt attttaagta 2700
ttttatagat gggggtgtta ttgtttattt atttattttt attggtggat tgttaggttg 2760
tttttgattt tttattgttg taaatttgtt tagtgaattt tttggtatgt ggttttgtgg 2820
gtagatatgg ttattttgtt aggataatgg atatttagaa atagagttgg ttaaatgaag 2880
atgggtattg ttaattttaa tagaaattgt taaaatattt attaagaaga ttgaattaat 2940
ttagggtatt tttaagagag gatgagaaga ttaatttacg tatatatttt tttttatgtt 3000
ggatattgtt agtacgttga atttttgtta atttaatgga tgaagttaaa ataaatttta 3060
tgtttatttt aatttgtagt ttttaaatta tgattgtatt ttttttatgt tgagtatatt 3120
tttttaagtt tattggatat tgtgttttta tttttgtaag ttattttttt atatattttg 3180
tttattttat ttttattttt atttttggag ataaagtttt attttgttat gtatgttaga 3240
atgtagtggg cgattcgggt ttattgtaat ttttgttttt taagtttaag agtttttttt 3300
gtttagtttt ttcgagtagt ttgggttaaa ggtgtttgtt attatattta gttaattttt 3360
gtatttttag tagagatagg gtttcgttat gttggttagg ttggttttga atttttgatt 3420
ttaggtgatt tatttgtttt agttttttaa agtgttggga ttttaggggt gagttatcgt 3480
gtttagttgt ttattttaaa aattggggtt ttaaaaatat tttttttatt aatgtgttgg 3540
aaatttttaa ttttggttat taatatgtgt tgtattttgg ttgagtgtag tgttttatgt 3600
ttgtaatttt agtattttgg gaggtcgagg aaggaggatt gtttgaggtt aggagtttag 3660
attagtttgg gtaatatgta agatttttat ttttattaat ataaaataaa taatttagtt 3720
aagtatttgt atgtggaatg tttttggttg aatttattaa atattttttg aattgttttt 3780
ttttaatttt gtnagatttt attttttgac gtatttgttt atttaaaatt aaaggggtat 3840
gttgaggtat gggtattttg gtagtagatt ttaaattaat ttttttgatt tgtgtttgtt 3900
ttttaggata tgtttttttt ttgaatttgt ttaagaaggt tgagttgggg ggtggttgtt 3960
gatttttata atatatagtt agttgtttat aggtttgtgt tttaaagaag ggtaagtttg 4020
aatttatcgt tttgttttag ggtttgggtt ttatatttgg ggagtagata gttttttatt 4080
ttttaaaaag ttggatttga aatttggagt aaattttttg gttgagtgat aggtgtttcg 4140
tagttgagtt tttgaggaga ttttttagtt gggtagagat attttttttt tagacgtttt 4200
gtgggttgga tttttttggt ttagtttaaa gtgaggggag ggtttttaat aggtcgggaa 4260
gatagttgat tttatttttt ttgggtttgt ttgtttgttc gtttttggga atggtcgttt 4320
tttgtttttt tttttttttt taagtttcgt tttgtttttt tttttttttt ttttatgaat 4380
tatttcgagt tttggttttc gtttttttat ttttggtttt tgtatttgtt tcggtttttg 4440
gttttttttt tttttttttt tttttttttt cgcgttgtta tttatttcgt tttttttcgc 4500
gtatagttaa tggagagatt tagtcgaaat cgcgaagttt ttttgtatcg ggttttttcg 4560
tttggtgatt gatgttttag agttaatagc gagcgagtag tcggagcggg gaagtagaag 4620
ttagagaggg gaagaatacg gcgttttttt tttttttttt tttttttttt tattttagtt 4680
tttttgcgta tttcgttttt aagttttcgc gtagttagga gtcgttgttg ttttttagtt 4740
tttgttagtt gtttttcgag tcgagcgtag cgagcgtcgt cgttcgggtt tttcggtggg 4800
gttagggtta gtatggagta tttgggttcg tattattttt attcgggtta cgtagagttt 4860
attagtttcg gtatcgatta gatttttaat agttcggatt agggtggttg tatgggattc 4920
gtttcgcgtt tttaggacgg agaatacggt tttggttgtt tggtcggagg cgtttatatt 4980
tacggcggcg ggggtttcgc ggtcgcgacg ggggttggag gagcgggggt ttatggtatt 5040
ggaggtttcg gcggtttcgg aggttcggta ggcggcggcg gcgtttgtag tatgggtttt 5100
ttgatcggtt tttataacgt gaatatggtt ttggtaggcg gtttcggttt tggcggcggc 5160
ggcggtagta gcggcggtgt cggggtattt agcgttgcgg gggtaattcg ggtgtcggta 5220
tataggtcgt tcgtcggagt cgtggtttat ttttagtttt tggttatcgg tttgtttatc 5280
gtgttttttg tgtttgttat gtcgggcgtt aataatttta ttggttttat ttttttttgg 5340
atggagagta atcgtagata tataaaggat aggtttatag gtgagttcgg ttcgcgcgtt 5400
tttcgtttgg tcgcggttcg ggtttcgtgt tatttttgtt tcgtcgggtg gttttttaaa 5460
gtcggttttg cgttttaggt cgtttagttt tttttggtgt tttttttaag ttgagtcgtt 5520
cgttcgattt tataacgtag attcgttatg tttgaagagt ttttttagtt tggatttttt 5580
tttgtttttc gaaggatttt tgttcggagg agtttttttt ggtttttttt tcggatttat 5640
tagtcgggtt aggtagagag aaggaggtta tagatttagg attatttttt gtagttattt 5700
ttggtttggg aattttagag aaaggggtgt aacgtgaata gtttttttcg ttttggttgt 5760
tgttttagga tttaggaagc gagttttagg attagatata agaaaagaat aggttttttt 5820
atttttagtt ttttatatat atgtatttcg cgtttttagt ttcggtgttc gtggtgttgc 5880
ggggttagtg gggggtttga gtttcggtaa attagtagaa ttagtggttt tttttttttt 5940
tttcggagag taggttttat ttgggaggat ggtagcgggg tttagtcgtt taggatagtt 6000
agttttttat tttcgttttt tttcggttgg gtgttttatt tcgggtttag ttttgttatt 6060
ttgggtacga ataatgtatc ggtaggttgg tgatcggtgg cgggaagatt attttcggga 6120
tattgggtat taagtggtgg tcgttgtcgt tttttttgtt gttcgttttc gttttttttt 6180
gttggttttg gttttttttg ttttcgtttt tattttgggt atttaatttt ttttttggcg 6240
tggatttttt ttttcgtttt tattttttgt tttttttagg gttttttgga ttttagatta 6300
cgattttttc gttcgggttg ggaagtagtt ataaattgag tttaggttag ttagtcgatt 6360
ttagagagag gaggtatgag 6380

<210> 126
<211> 6380
<212> DNA
<213> Artificial Sequence

<220>
<223> chemically treated genomic DNA (Homo sapiens)

<220>
<221> unsure
<222> (2588)

<400> 126

tttatgtttt ttttttttaa gatcggttgg ttaatttggg tttaatttgt ggttgttttt 60
tagttcggac ggaaggatcg tggtttagga tttagggaat tttaagggga gtagaaagtg 120
agaacggggg aagggattta cgttagaggg agagttgagt gtttagagtg gaaacgggag 180
tagagggagt tagggttagt agaggaaggc gggagcgggt agtagagaag gcggtagcgg 240
ttattattta gtatttaatg tttcgggagt agttttttcg ttatcgatta ttagtttatc 300
ggtgtattgt tcgtgtttaa gataataggg ttgggttcgg ggtaggatat ttagtcggga 360
gagaacgaaa atgaaaagtt agttgttttg ggcggttaag tttcgttgtt atttttttag 420
atgaagtttg tttttcggaa aaaaaaaaaa aggttattgg ttttgttgat ttatcggggt 480
ttagagtttt tattggtttc gtagtattac gggtatcgga gttaggggcg cgaagtgtat 540
gtgtgtaggg gattggaggt gggggaattt gttttttttt tgtgtttgat tttggggttc 600
gtttttgggg ttttagaata gtagttagga cggaagaaat tgtttacgtt gtattttttt 660
ttttaagatt tttaggttaa gagtagttgt agaaggtggt tttgaattta tggttttttt 720
tttttgtttt gattcggtta gtggattcgg agaggggatt agggagagtt ttttcgagta 780
ggggtttttc gggagataga gaggggttta ggttgagaga attttttaag tatggcgagt 840
ttgcgttata gaatcgggcg ggcggtttaa tttgggggaa gtattaagaa gagttgggcg 900
atttggagcg tagaatcggt tttggggagt tattcggcgg ggtaggggta gtacggagtt 960
cgggtcgcgg ttaggcgggg agcgcgcggg tcggatttat ttgtgaattt gttttttgtg 1020
tatttgcggt tattttttat ttaggggaag gtgaggttag tgaggttgtt gacgttcggt 1080
atggtaggta tagagggtac ggtgggtaag tcggtggtta ggggttgggg gtgggttacg 1140
gtttcggcga gcggtttgtg tgtcggtatt cggattattt tcgtagcgtt gagtgtttcg 1200
gtatcgtcgt tgttgtcgtc gtcgtcgtta ggatcggggt cgtttgttaa ggttatgttt 1260
acgttgtagg agtcggttag aggatttatg ttgtaggcgt cgtcgtcgtt tgtcgggttt 1320
tcggggtcgt cgggattttt agtattatag gttttcgttt ttttagtttt cgtcgcggtc 1380
gcggagtttt cgtcgtcgta agtgtaggcg ttttcgatta agtagttaag gtcgtatttt 1440
tcgttttgga ggcgcgaggc gggttttatg tagttatttt ggttcgggtt gttgaggatt 1500
tggtcgatgt cgaagttaat gggttttgcg tgattcgggt ggaggtggtg cggatttagg 1560
tgttttatgt tggttttggt tttatcgggg ggttcgggcg gcggcgttcg ttgcgttcgg 1620
ttcggggggt agttagtagg ggttgggagg taatagcggt ttttggttgc gcggagattt 1680
ggaagcgaag tgcgtagaaa ggttaaagta gaagggggag gggagggaga gagggggcgt 1740
cgtatttttt ttttttttgg tttttgtttt ttcgtttcgg ttgttcgttc gttgttgatt 1800
ttgggatatt aattattagg cgaaaaagtt cggtgtaaga gagtttcgcg gtttcgattg 1860
ggttttttta ttggttgtgc gcggagagga gcggggtgaa tgatagcgcg agggagggga 1920
ggggaggggg agaggaaggt tagaagtcga gatagatgta ggagttagag atagagggac 1980
ggagattaag attcggagta gtttatgaag agagagaaga ggggaataag gcgaggttta 2040
aaaggaaaag ggaaaatagg aagcgattat ttttagagac ggatagatag ataaatttaa 2100
ggagggtgaa gttaattgtt ttttcggttt gttgggagtt ttttttttat tttgaattgg 2160
gttaaaggag tttagtttat aaggcgtttg aggaagggat gtttttgttt aattggagaa 2220
tttttttaag ggtttagttg cgaaatattt gttatttaat taggagattt attttaaatt 2280
ttaagtttag ttttttagaa agtagaagat tgtttatttt ttaggtatgg agtttaggtt 2340
ttgaggtagg acggtgagtt taggtttgtt tttttttaaa atataggttt atgaataatt 2400
ggttatatgt tataaaaatt agtaattatt ttttaattta gtttttttag ataaatttag 2460
gaagggaata tattttgaaa ggtaggtata agttaagagg gttggtttgg ggtttgttgt 2520
tagggtgttt atgttttaat atattttttt ggttttaaat aaataaatgc gttaagaagt 2580
aaaatttnat agggttagaa agaggtaatt taaaaagtgt ttgatgaatt taattagaaa 2640
tattttatat ataaatgttt ggttaaattg tttattttat attggtaaag atgggagttt 2700
tgtatgttgt ttaggttggt ttgaattttt ggttttaagt aatttttttt tttcggtttt 2760
ttaaagtatt aggattataa gtatgagata ttgtatttag ttaaaatata atatatatta 2820
ataattagaa ttaagaattt ttaatatatt aataagaaaa atatttttga aattttagtt 2880
tttaaaatag gtaattgggt acggtggttt atttttgaaa ttttagtatt ttgggaagtt 2940
gaggtaggtg gattatttaa agttaggagt ttaagattag tttggttaat atggcgaaat 3000
tttgttttta ttaaaaatat aaaaattagt tgggtgtggt ggtaggtatt tttaatttaa 3060

gttattcggg aggttgaggt aggagaagtt tttgaatttg ggaggtaggg gttgtagtga 3120
gttcggatcg tttattgtat tttagtatgt atgatagagt gagattttgt ttttaaagat 3180
aaaaataaaa ataaaatggg taaagtatat gaagaggtga tttatagaaa taaaaatata 3240
atgtttaatg aatttaggaa aatatgttta atatgagaga aatgtaatta taatttgaga 3300
attataaatt aaaatagata taagatttgt tttagtttta tttattggat tagtaaaaat 3360
ttaacgtatt gatagtattt agtatgggag agagtgtgta cgtaagttgg tttttttatt 3420
tttttttgga agtgttttga attggtttag tttttttgat aggtattttg gtagttttta 3480
ttaaaattaa taatatttat ttttatttga ttaattttat ttttagatat ttattatttt 3540
aataaaatag ttatgtttgt ttatagaatt atatattaga gagtttatta agtaaattta 3600
taataatgaa aaattagaag taatttaata gtttattagt gggaatgggt gaataaatag 3660
tggtattttt atttatggaa tatttaaggt gaagtaatta aaaataatga ggtagatttt 3720
tatgttttga tatgggaaaa tgtgtatgtt atatttttat tttaagttat atatatagat 3780
agatagatat attttttttt gagatggagt ttagttttgt tatttaggtt ggagcgtagt 3840
ggtacgattt tagtttattg taatttttat tttttaggtt taagtgattt ttttgtttta 3900
gtttttcgag tagttaggat tataggtatt cgttattata tttagttaat ttttgtattt 3960
ttagtagaga cggtgtttta ttgtgttggt taggttggtt ttaaatttta gattttatga 4020
ttcgtttgcg tcggtttttt aaagtgttgg gattataagc gtgagttatc gtttattgga 4080
aaagtaaatt gtagaaaaat aaaaatattt aggtataaag gtaaaaattt aataaaaaaa 4140
tcgtatgttt ttttttgaat aatatatata tgtaggtaaa tgcgtaggaa atgatttaga 4200
aggatatata gtgaattgat tatagtagtg atttttgtag atgattttgg aattggaagg 4260
attattttat ttgtattttt tataataaga atgtattttt agtttatttt tgaaatttaa 4320
agtaaattta aataaatgtg gtttatttga ataaatgaat taataaaagt tttagtagtg 4380
agttttgata tatataaata cgaggtatta agatttttat ttattgtatg aagaaagagg 4440
tttttttttg gtagtaattt tgttttagta aatgttataa ggattcgttt tttattttat 4500
ttttggaatg ttaaaattgg gggttgttta gattgttaga gggttaaaga tttttagttt 4560
atataggtaa ggaaagatgg ttagggttta agttatagtt ttagaaagat tataaaaggt 4620
ttatggaagg cggagagtgg taaggtgatt gtatttttgt ttgatttgta atttgttatt 4680
ttagggataa gtttaggttt aggggtttat ggttgtattt atagtatttg gattcggttt 4740
tagtaattta tgataaatat tgagtgattg ttggtattaa tagttattta tattaggtag 4800
ttttttgtag ttgatataat tttgttttgt atattttttt atttgttttt tataattttt 4860
gagaggtagt ttttattgtt atttttatgt tatagatgag gaaattgaag tttagagagg 4920
ttagatttgg ttacgtttat ttagataata agaggtagag taagatttaa ttgtagtttt 4980
tttgattttt tatttttaat tttattgtgg taaaattttt ttggggttat tatttgtttt 5040
tttttttagt tttttgggta tgagttttag ttttggtttg atattttttt tgggaaaaat 5100
ttagtgaggt tgattttagg tagaggattt aattgagttt tggttgttga tgaggattat 5160
gataggtagt gtagtttagt ggtgaagatg tacgaggttt tagttttaat ttttagttga 5220
gtgattttgg gtaagttatt taatattttt gtttattttt ttatttgtaa aataagaata 5280
ggagaatttg gttagttata gtggtttacg tttggaattt tagtattttg ggaggttggg 5340
gtaggtagat tatttgaggt taggagtttg agattatttt ggttaatata gcgaaatttt 5400
atttttatta aaaatataaa aattagttag gtagtagtgg agtgtgtttg taattttagt 5460
tttttaggag gttgagtagg agaatggttt gagtttggga gggggaggtt gcgggtgagt 5520
cgagatcgta ttattgtata atttattttt tggagatgtg aatatatatt gagataatat 5580
atataaagtt tttagtttgg tgtttaatat attattagtt tttattagat gtttaagatg 5640
atggtaattg attatagaat ggttttttta gtttagtgag tgtgatttga attttagttt 5700
tattgtatat ttgtatcgta gaaagagtta tattagagag agatgtgata gagtagtttg 5760
ggaattgtaa agtagttatt tggttagggg attttgagtt aatatttagt tgataaaagg 5820
gaaaaataag gtttttaaat aggagaatag gttaagtgtt tttggagtgg gtagatagag 5880
tttaatgtgg ggaggggttt gtggttaagg ttaggatggg attggtgtgt taagtagaga 5940
tgttaggaga aatggtttgc gggaggtttt tagggggtat ttagggttta taaggagagt 6000
tattagatgg gatttaagag taagggtgtt atttggatgt agattttttt ttatttgggt 6060
tttttttttt ggaagggagt ttgagaggaa aaaaatggtg agttaatttt gagagtattt 6120
gtttttttat aggttgtttg cgtggtgtga ttttggttta ttgtattttc gtttttaggg 6180
ttattttttt ttttgtttta gtttttcgtg tgttgggatt ataggtatgt gttattatat 6240
tcggttaatt tttgtatttt tattagagat ggggtttcgt tatgttggtt aggttggttt 6300
tggatttttg atattaggtg attcgtttgt ttcggttttt aaaatattgg gattataggc 6360
gtgagttatc gcgtcggttt 6380

<210> 127
<211> 6118
<212> DNA
<213> Artificial Sequence

<220>
<223> chemically treated genomic DNA (Homo sapiens)

<400> 127
   ggtttttgag gttttttttt tgtaggattt tagcgttttt tttatagatt tttgtttgta 60
   gtttttagat gtagttttat ttagtttgtt aggttttttc gatagtttcg tagatatttt 120
   agttgatagt ttagattttt ttatagatat atttattata atcgatttgt agtatttgaa 180
   ttattaaaaa tattaaagta aaataaagta ttattaaata aaaatttttt tgattaggtg 240
   gttttgtttt tttttatttg ggagtttatt ttttattttt tttttgattt attttttttt 300
   ttttttaagt gttgaggatt ttttgtttag tgattttttg atttagtttt aaatagagtt 360
   attttttata gattattttg gagttttagt tgttttaaat ttaatttaat aattttttat 420
   gtgatttttg agagtagtat gaggtttgta agaaagtgat tatataattg tatttttatt 480
   ttttttttat ttttgtatta tattgggatg tattgttatg tatatttttt gtagaataaa 540
   tttttttttg ttataagtag tttatttttt tttttttttt tttttaaggt ttataacggg 600
   tttttttttt ttttttagtt taatttcgta aataaaattc gttataattt attttttttt 660
   tttaatttta atatatttat taaggcggta tgtttaaagt ttttaatgga tagtaataaa 720
   atagaaagtt aaaaaatgat tagggttttt aataagtgtt tttttttttt tttttgtttt 780
   tttttttttt tttttttttt aaaggatttg atttttagtg tataggttta agttatagat 840
   ttaaattggt aatagaggta gaattggata gtttaagatt tgggttaagt ttaagttttt 900
   gaaagtggag aaataaagtg tttatagaaa tttttaattt taggaatgag taatacggag 960
   tttttttaat aagttattag tttttaggtt aataattaag ttattaataa tttatttgtt 1020
   tatatatttt tgttttaaat atttatattt taattttttt ttttataaat ttttttatat 1080
   ttttagtgtg gttgattaat tttttagttg ttgatgggtt taggtaaaat agatttaaaa 1140
   gatagagaaa aataaaaata aaataaaata gtgattatta tttggaaaat gtattttaga 1200
   aaatgttttt tttaattttt tggtttagtt attttaggat tttttttttt tttttagggt 1260
   tagaaaaatt aatattttat ttttaaattt gtttatattg ttaataatag atcgttaatg 1320
   tttcgaagtt aggttagtaa gttgagattt aaaatggtgt gtttttatat tttaggttat 1380
   aaattaacgt aggttttaga gagagagaaa agtattttaa atgaaatatt aataaaatgt 1440
   gatttagggt gtgttattgg gttgttgttt tttttttttt ttttgttagg tttttttatg 1500
   tttatttttt tttttttgag tttttttttg atgtttattt atttatcgtg agaaaacgta 1560
   gtatgtaagt aatatgtgtg tggggcgggt tgatttaaga atttggtttt taatagtaaa 1620
   ggtttggaag ttttaatgat aacgcgatgt ttttttatag gtttgttttt tatttttttt 1680
   cgtttttttt ttcgttaaat tttagttgcg gcggcggagt gtggggatta tggttggttt 1740
   ttggttttaa gtttagcgcg gagtagggcg tttcggtagc ggttagggtt gcgtagtttt 1800
   tggatgcgat tcgttttacg tttataaatt aaacgttttt tatgaatgag aatgttatta 1860
   aagagattaa ttgtaggaat atatgtataa ataaaaattt ttttacgtta tttgtcgggg 1920
   attttcgttc gaaagtatta agttagaagg cgtttagtta taatttattt ttattgtttt 1980
   tttaaaataa tagtttggtt gagtcgcgga tgttatgaat agtttagggt ttcggcgtta 2040
   ttttattttt tttttatttt tttttttttt agtataagag ttttgaaaga ggcgttttga 2100
   gttaagagaa tcgtttgtag gcgattattt taagcgtaat taagttcgtt ttttatttag 2160
   ttttttaagg cgttgaaaga gattagtttt agttttgggg cgttgattta tttgtttaga 2220
   gggttaagga ttttttattc ggtggaacgg tttttagaat tttttggggg aggggtgtgg 2280
   ggaaagaatt gagttataga agtttaaggg ttagaggtta ggggaggttg tttttgtgtg 2340
   ttgttttcgg ggagcggcgt tttgggttgt taggcgaggt atagttagtt cggttgcgag 2400
   tattcggttt gcggggagtc gggaatagta agcggagaag ggcggtcgtc gggtttgtgt 2460
   gtttgcgggt ttgtgtgcga gtgtaagttc gtgattttaa gaaaaatatt ttatttagga 2520
   tgttttagaa aagaggttag gagagaagaa gagaggttga gggagagtag ggtcgaggta 2580
   gcgaattagt agagagagtg gtttttgtcg ggtcggtttt tgtggtaggc gttttgtagt 2640
   cgagggggtt ttgtaaattt aggttgagga gggtaattaa ggtataggaa tgaggtagcg 2700
   ggggaaaatg tatcgatttg tttaggtttg gagggaggat tgttcgtata gttagtatcg 2760
   atttatttat attttgtttt tttttttata ggagtttttt tttttttcgt cgttggaagt 2820
   tggtgttttt aaggaagtcg tttttaggag gattgttttg tttttttttt tttttagtcg 2880
   gaggattttt aggagtcgaa attggggtag gggtggttgt ttaattttag aaagtgggtt 2940
   ttttttgttc gtgttttttt ttaattatgt ttgggatgga ttaaagttat aaatattatt 3000
   ttgaaaggaa atgtttgggg agaatgtttt attgttattg agggagttta gagttagtat 3060
   gggttttttt ttttgagagg ataggtttgg gggaatttcg gtacgtggag aatagaatat 3120
   agaagattga aagggtgatt aatatatttg aaatgtattt tttttgaaaa aaaaaaaatt 3180
   ttttttgttt taacgggagg aaaggttgaa gaggagtgga aattttaaat ttatatatat 3240
   ttatttatta taaattttat ttgattttaa tattgtatat atggttggag taaaatttat 3300
   atagtattgt ttataattta tatagtaaag ttttttggat aagttttcga ttttttttat 3360
   ttaagattgt attaattttt ggttttcgtt tggttttttt tttttataag tttagggttg 3420
   acgtgaaatt taatagtaaa taataattta acgattttag tagttttagg gaagtagggt 3480
   gatgaggaat aggatttttg gtttgggatg aggtttagat ttgtatgtgt ttttttaggg 3540
   aaggatttga aggtttttat aagtaattat atagttagtt ggaaagggga ggaggtgtcg 3600
   ttggggtaaa gtgtttcgtt tattattagt tatttgttta tcgttttttt tatttgttag 3660
   tttttatatt tattttagac gtgtagtaga atgaggaagg ttataaaagg aagttttttt 3720
   ttttttttaa tatttttttt tagaaaaaga ggaacgtaac gaagggagtt tagttattat 3780
   gttggagttg tggatcggtt ttgtgaggtt tacgcgggag cgcggaggtt tttgggtttt 3840
   agggcgtcgg tagatggttt gtagcgtatc gttcggtttt cgcgttggtt acgttcgtta 3900
   gcgttatttt tttttttcgt tttttatttt taaaaattcg gcggtcgagt agtttcgtat 3960
   ttcggagggt tttcggtgta aaattgagtg ggaaagagaa aggggcggag agcgacgaga 4020
   gagggaaggt ggggaagaag agttcggtcg ttttcggttt ttttcggcga gttttgagtt 4080
   tttagggttt attcgttttt ttaagtcgaa ttatagaaat tttttattgt tatgttaatg 4140
   acgagagggg ttgtggggag agggtttcga agattttttt ggaagaaggg aagttttttt 4200
   ttttgttttc gtttttttta cgtttattcg agtttttttt agttagtttt ttgcgttttt 4260
   ttttttttat ttcgagtttg aagagtcggt ggtttagttt cgttttttat tacggtttta 4320
   tgcgtttttt atagttttac ggttttttag tttagtttta gaatagagga ggtggtttgg 4380
   gaattcgggg cgcggtgtta gtcggatttt tcgtttttcg gttgggcgcg ttgcgggttg 4440
   ggtcggcgta tttgcggtta ttcggcgagt tttggtttat tgattttcgt ttcgagagag 4500
   tcgggagttt tttttttttt ttatttttcg ttagtttatt ttttattgaa cggtggggag 4560
   ggggcgggag cgggggacgt tttttagttt ttatttttcg gaggtttggc ggggcgggta 4620
   gcggagtcgg gcgcgcgtag aacgtagttt ttgttttttt tcgttttagt attttaaatc 4680
   ggtttttgta gtcgtcgtag tagttgtcgt cgtttaggtt gtttgggggg gcgattgcgc 4740
   gttatttaga cggcggagcg tcggggattt aaatgttatt aaaacggtga tttattattg 4800
   cggaagtcgg taaatttttg taggaggttt agttattggt tgatatcgtt acgtgttttt 4860
   tttttagcgt ttttcgtttt ttcgtttttt tttttgcgta ttgtatattt atattatttt 4920
   tttttttcgg ttttatggag gaagtgagaa agttggtata gttacgtcgg gtttcgtagg 4980
   attaggttat ttagtgatag atggataatg taagaatgaa tttttttttg gaatatttta 5040
   tatttagtag tggcgattcg gggatttgtt tagttcgagt ttatttttcg gattatagga 5100
   ttataatttt ttagtcgtgc gcggttagcg ttaatagttg cggcggcgac gatcgttttt 5160
   tagtgggtag gggggtgtag atcggttcgt tttattatta ttattattat tattatcgtt 5220
   atttttagtc ggttatttat tagattttcg ggaatttggg ggtgttttat ttttatttaa 5280
   gttgtggttt aagttatggt ttatagaatt ttagtgcgtt ttatagtttt tacgcgttaa 5340
   attaggaagt agacgtaagt ggtgggtatt tttagtgcgt tttcgttgtt tattttggaa 5400
   attttttatt ttttatggtt tagtattatt attattatta gggttatgtt gggggcgcgg 5460
   tgggttcgtt ttaatatatt tattatttat atggatagga gtattagagt ttggttttgg 5520
   ttacgtataa taattttttg tttttttttt acgttagtta ttaagaagtt tgtcgttttt 5580
   tcgtatcgga gatatttttt ttagcgtaga tttttgattg gatgaaagtt aaaagaaatt 5640
   tttttaaaat aggttagttt tgttggttgg tggatgtttt ttgattattt tggaaggagt 5700
   tggtatgttt aatttttaag gaaattaata atgatttttt ttttaaaagg tttttgtatt 5760
   agattagtgt tgtagtgtat ggagagggtg tttagaggtg ttggggtaaa gaagttttag 5820
   ggattggtgt gtttttaagg attttatata tttgggaaat aggaagtatg tgggggtggt 5880
   tattgtgacg gtagtgtttt gttaatattt tgaggtttta ttaattatcg gtttgattat 5940
   gttaatggtt tatattaggt taatatttta tattttatta tttatttttt atagttcgat 6000
   ttgtgttgag tttttgattt tttttttatt tgagtgttgt tatgaagcgt gtggggaatt 6060
   ttaagtcgat ttaaaaattt ttatattttc gttttatggt tttttatttt gttttagg 6118

<210> 128
<211> 6118
<212> DNA
<213> Artificial Sequence

<220>
<223> chemically treated genomic DNA (Homo sapiens)

<400> 128

tttggggtaa agtgggaagt tatgagacgg aaatgtaaaa atttttaaat cgatttgaga 60
ttttttatac gttttatggt aatatttagg taaagaaaag attaagaatt tagtataaat 120
cgggttgtgg agggtgagtg atgaggtgta aagtgttaat ttgatgtaaa ttattagtat 180
ggttagatcg gtgattaatg gagttttaag atattaatag aatattatcg ttataataat 240
tatttttata tattttttat tttttaaatg tataaaattt ttgaaaatat attaattttt 300
gagatttttt tgttttaata tttttgggta ttttttttat gtattataat attagtttga 360
tataaaagtt ttttaaaaaa aagattatta ttaatttttt tggaaattaa gtatattagt 420
tttttttaga ataattaagg agtatttatt aattagtagg attgatttgt tttgggaggg 480
ttttttttga tttttattta gttaaaagtt tgcgttggag aagatgtttt cgatgcgggg 540
gagcgatagg ttttttggtg gttggcgtgg agaggggata aggagttatt atacgtagtt 600
agggttaggt tttggtgttt ttgtttatat gagtggtgaa tgtattgagg cgagtttatc 660
gcgtttttag tataattttg gtggtggtgg tgatgttgga ttatgggaga tgagagattt 720
ttagagtaaa tagcgggagc gtattggggg tatttattat ttacgtttgt tttttgattt 780
aacgcgtagg ggttgtaagg cgtattgaag ttttgtgagt tatagtttgg attataattt 840
gagtgggagt aggatatttt taggttttcg gaagtttggt aggtagtcgg ttgggggtgg 900
cgatggtggt ggtggtggtg gtggtggggc gaatcgattt gtattttttt gtttattagg 960
aagcggtcgt cgtcgtcgta attgttggcg ttgatcgcgt acgattggaa agttgtaatt 1020
ttatggttcg aggggtaggt tcgggttgag taggttttcg agtcgttatt gttaagtatg 1080

gggtatttta ggaaggagtt tatttttgta ttgtttattt gttattgagt gatttggttt 1140
tgcgaagttc ggcgtgattg tgttaatttt tttatttttt ttatggggtc ggagaagaaa 1200
aatgatatga atgtatagtg cgtaagaggg ggggcgggag ggcggaggac gttggaggag 1260
gggtacgtga cggtgttagt taatggttga gttttttgta aaagtttgtc ggttttcgta 1320
gtgatggatt atcgttttag tggtatttaa attttcggcg tttcgtcgtt taggtgacgc 1380
gtagtcgttt ttttaggtag tttaggcggc ggtagttgtt gcggcgattg taaaggtcga 1440
tttggagtgt tggagcgaag aagagtaaaa gttgcgtttt gcgcgcgttc gatttcgttg 1500
ttcgtttcgt taggttttcg ggaggtgggg gttgggaggc gtttttcgtt ttcgtttttt 1560
ttttatcgtt taatgaaaga tgaattggcg agaggtgaga agggaagagg gttttcggtt 1620
ttttcggggc gggaattagt gggttagagt tcgtcgggtg gtcgtaagta cgtcggttta 1680
gttcgtagcg cgtttagtcg gaaggcgggg aattcggttg atatcgcgtt tcgggttttt 1740
aggttatttt ttttgttttg aggttgggtt gggagatcgt ggggttgtga ggagcgtata 1800
gaatcgtggt ggagggcgag gttgggttat cggtttttta agttcggaat ggagggggaa 1860
gagcgtagag ggttggttgg gaggaattcg ggtgggcgtg aaggagacga gggtaagaaa 1920
agaaattttt ttttttttag gagggttttc gaaatttttt ttttatagtt tttttcgtta 1980
ttagtatggt aatgaggagt ttttgtaatt cgatttggag gggcggatga gttttggaaa 2040
tttagagttc gtcggaaaag gtcgggggcg gtcgggtttt ttttttttat tttttttttt 2100
tcgtcgtttt tcgttttttt ttttttttta tttagttttg tatcgggagt ttttcgggat 2160
gcggagttat tcgatcgtcg gatttttagg ggtaggaggc gggggagaga gatgacgttg 2220
gcggacgtgg ttagcgcggg ggtcgggcgg tgcgttgtag gttatttgtc ggcgttttga 2280
gatttaggag ttttcgcgtt ttcgcgtggg ttttataggg tcggtttata gttttaatat 2340
agtagttgaa tttttttcgt tgcgtttttt tttttttgga ggggaatgtt agaagagaga 2400
gagagttttt ttttataatt ttttttattt tgttgtacgt ttagagtggg tgtgggggtt 2460
ggtaggtggg aggggcggtg gataaatggt tgatggtgga cgggatattt tattttaacg 2520
atattttttt ttttttttaa ttggttgtgt agttgtttat gagaattttt aagttttttt 2580
ttagagagat atatgtaaat ttgagtttta ttttaggtta ggggttttgt tttttattat 2640
tttatttttt tgaggttgtt gaggtcgtta aattgttgtt tattattagg ttttacgtta 2700
attttgggtt tgtagagaga aaaagttaaa cggagattaa gaattgatgt agttttgggt 2760
aggagaaatc gagagtttgt ttaggaagtt ttgttgtata aattataagt aatgttgtat 2820
aaattttatt ttaattatgt gtataatgtt ggaattagat gaaatttata gtgaatgaat 2880
gtatgtgggt ttggggtttt tatttttttt tagttttttt tttcgttaga ataaggaagg 2940
tttttttttt tttaaggaag gtatatttta aatatgttag ttattttttt agttttttgt 3000
attttgtttt ttacgtatcg aagttttttt aaatttgttt ttttaagaga aaaaatttat 3060
gttgattttg gattttttta gtaataatga aatatttttt ttaaatattt ttttttagaa 3120
tagtatttgt gattttgatt tattttaagt atggttaaga gggagtacgg gtaggaaagg 3180
tttatttttt ggggttgggt agttattttt gttttagttt cggtttttgg gaatttttcg 3240
attggagaag gggaaaggta aggtagtttt tttggaggcg gtttttttgg gagtattagt 3300
ttttagcggc ggggagagaa ggagtttttg tgggagaggg ggtaggatgt gagtaggtcg 3360
gtgttggtta tgcgagtaat ttttttttta agtttgagta agtcggtata ttttttttcg 3420
ttgttttatt tttgtatttt ggttgttttt tttagtttgg gtttgtagga ttttttcggt 3480
tgtagggcgt ttgttataaa gtcgatttcg gtaggagtta ttttttttgt tagttcgttg 3540
tttcggtttt gttttttttt agtttttttt tttttttttt ggtttttttt ttggggtatt 3600
ttgggtggag tgtttttttt gggattacga gtttgtattc gtatataggt tcgtagatat 3660
ataggttcgg cggtcgtttt ttttcgttta ttgttttcgg tttttcgtag gtcgggtgtt 3720
cgtagtcggg ttggttatgt ttcgtttggt agtttagagc gtcgtttttc gggaatagta 3780
tataaaggta gttttttttg gtttttagtt tttaggtttt tgtagtttag tttttttttt 3840
atattttttt tttaagaaat tttgggggtc gttttatcga gtaggagatt tttggttttt 3900
taggtaagta ggttagcgtt ttaagattgg agttggtttt ttttaacgtt ttgggagatt 3960
gggtgaaagg cgagtttggt tacgtttaaa atgatcgttt ataagcggtt tttttggttt 4020
aaaacgtttt ttttagggtt tttatgttag aaaggaaagg aataaggagg agataaaatg 4080
acgtcgaggt tttgaattgt ttatggtatt cgcggtttag ttaagttgtt gttttaaaag 4140
agtaataaaa atgaattatg attaaacgtt ttttaattta atgttttcgg acggggattt 4200
tcggtaaata acgtaagagg atttttattt gtgtatgtgt ttttgtaatt gatttttttg 4260
atgatatttt tatttataga aagcgtttga tttatgagcg taggacgaat cgtatttagg 4320
agttgcgtag ttttggtcgt tgtcgggacg ttttgtttcg cgttgagttt ggggttagaa 4380
attagttata gtttttatat ttcgtcgtcg tagttgagat ttagcggagg aaggggcgag 4440
ggaaggtagg gagtaaattt atgaagaaat atcgcgttgt tattggaatt tttaagtttt 4500
tgttgttaag agttaggttt ttaaattaat tcgttttata tatatgttgt ttatatgttg 4560
cgttttttta cggtaagtaa gtaggtatta gaagagagtt tagaaaggga aaaataaata 4620
tggggagatt taataaggga ggggggaaaa gtaataattt agtgatatat tttagattat 4680
attttattga tattttattt aaagtgtttt tttttttttt tagaatttgc gttaatttat 4740
aatttagggt gtagaaatat attattttaa attttaattt gttaatttga tttcgaagta 4800
ttaacgattt attattagta atgtaaatag gtttaagaat gaaatattga tttttttgat 4860
tttgaaaaaa aaaaaaaaat tttagagtga ttgaattaga ggattggaaa gaatattttt 4920
tagggtgtat tttttagata gtaattattg ttttgtttta tttttgtttt tttttatttt 4980
ttaggtttgt tttgtttgaa tttattaata gttgggagat taattaatta tattgaaaat 5040

gtggagggat ttatggggga gggggttgaa atgtgggtgt ttgaaataaa agtgtataaa 5100
taaatgaatt gttgataatt tagttattga tttggagatt ggtagtttat taaagaaatt 5160
tcgtgttatt tatttttgga gttgggggtt tttgtaggta ttttattttt ttatttttaa 5220
gagtttgggt ttggtttaaa ttttagattg tttaattttg tttttattat taatttaaat 5280
ttatggtttg aatttgtgta ttgaaaatta aattttttaa aaagaaagag gagaagaaga 5340
agtaaaaaag aaagaaaaaa tatttattag aagttttagt tattttttgg ttttttgttt 5400
tgttgttgtt tattgaagat tttgaatatg tcgttttaat aaatgtatta aaattgaaaa 5460
aagaggataa attgtgacga attttattta cgaagttaga ttaaaagaag aaaaagaatt 5520
cgttatgagt tttgaaaaaa gagggggaga aaaataagtt atttatagta aaggagaatt 5580
tattttataa aaaatatgta tgataatgta ttttaatgta atataaaaat aaaaagaaag 5640
tgaagatata attatatgat tatttttttg taggttttat attgttttta ggaattatat 5700
aaagggttgt tgagttaggt ttaaaataat taaaatttta aaataatttg taaaagatgg 5760
ttttgtttga aattgggtta gggaattatt aaatagaaaa tttttaatat ttaaaggagg 5820
gaaggggtag gttaagaaga aaataaaaaa taaattttta aataaaagaa ggtaaaatta 5880
tttggttaaa ggagtttttg tttggtgatg ttttgttttg ttttaatgtt tttagtaatt 5940
tagatgttgt aagtcgattg tggtgagtgt gtttgtaaaa aagtttaagt tgttagttga 6000
aatatttacg ggattgtcga gggaatttgg taaattgggt gaaattgtat ttgaaagttg 6060
taggtaggaa tttgtggaga aaacgttaaa gttttgtaaa gaggggattt taagggtt 6118

<210> 129
<211> 7117
<212> DNA
<213> Artificial Sequence

<220>
<223> chemically treated genomic DNA (Homo sapiens)

<400> 129

agagaaggag gaagtgttgg ggaagaaagg agggtagaga agggagggga gaggaaggga 60
gataagttag atttggtggg gttttgaggt ggagtatttt taagaaggag gtgggagatt 120
tagtttaggt tttggtggaa gttcgaagtt gggggagagt tagggattta atagtttttt 180
ttttttgcgt ttgagtattg gatgtttttt agtggtattt tagttgttgt ttagtttagt 240
agaggtatta aaagaggtag ggaagagata ggaggtttta gtaggtgaga atggttggaa 300
ggaggtaggg taggagatag gaattgtagg ggggatagga gtaggattag gaattgtaga 360
gggataggat ttttttgaga aggatagtgg tgattggggt agtttggtat tgatattgag 420
attgtggata aatgttgggt atttaaaggg ttgtatgtgt aagtattggg gatttcgaat 480
ttttagattt ttgggataag gagtagttgt tgttttttaa gttagtttta gggagagaag 540
agggtggttt agtagttttg ttagttggag ttgggaagtt tggagtgtgg aggtgttttc 600
ggttatttag ttgtattttt ttttggagtg agagggtgtt aagtcgtttg ttaagtgacg 660
atttttgtag tttacgtttt ttgaatgatt gtgttggaaa cgtttttagt ataataattt 720
cggtaataag gtttcgtatt gttcggtgtt tatcgacggt ttcgaggcga tttcgtttgg 780
gtattgttcg tcggcgtttt ttcgttgttt ttcggattat ttaggttgta agaagaagcg 840
gaggcggtag aggcgtttgg atttatttta tttcgatatt tcgtttgtaa aggttgtgga 900
aagaagcgta aggtttaggg atggtagttt ttagagagga gggtttttga ggttttgcgt 960
aagttttttt tttttaattt ttcgggttaa gttagacgaa gtttcggaaa ggttgggttt 1020
gttttgttga ttaaattttt ttgttagtcg ttttcgtttg ataagtttgg cgtaggcgtt 1080
ggtttttttt ttttcgggcg gttattttta atgtcgcgcg gggagtaatg gggttcggtt 1140
ttgtagaggg ttgtttggat tttgaagttt ggaaaaataa gaagagattg agtcggtttt 1200
tttttttttt tgtagatata tacgtatcgt taggtcgtcg ttattttttg tttttgtttt 1260
ttttttttta aatatttttt ggttttttgt attagtcgcg gtggggtttt gggttgacga 1320
taaaaggaaa tatattttga agatagtttt ttggagatgg ttcgcgttgt ggagtttttg 1380
agaaagaata gtttttggga agttagtcgt tagggttaat gttatagttt tacgcgcgtc 1440
ggttttttta agaaatggaa ttcggatttg gtttttcggg gcggttttag ttttagtcgg 1500
gtttttttta gagttcggtg ggttaagttc ggttgttgga ttttttgtaa agcggaagag 1560
ttaattatag gtttagagta aagttaaaat taaggattcg aaggtttgtt tttttttttt 1620
tttttagatt tggtttgatt tttagagttg cgtttatttt tcgttttagt cgtttgtttg 1680
gagaaatttt tatatttata tcgttagtat atttcgaata cgagggtagg agcgggtaaa 1740
gttgggatat tgcgtagttt tggggagttg agtattcgtt ttttattttt gttgggtaat 1800
taaaagtgtt cggttatttt aggcgttttt ttttttgttg atattaaaaa tttttttttt 1860
tatttatttt tacggtagcg tttttatata ttttttttcg ggcggtaagt tttttcgggt 1920
attcggtttc ggtgtagttt gtcgaattgt tttgttagtc gttttcggag cgtttttttt 1980
agttttagtt tttttttcgg aattcgtttt tgttttttgc ggatattcgt gggatttagt 2040
tttgttagta ttttttcggt tcggttttcg aggaggatat gggaggtagt ggttatgcgg 2100
aatagcgtat atttttatat cgattttttt gtcgggatcg ttttttcgga tattagggga 2160
aaatcgattt aattaagaga ggggttgcgt gtcgttgggg gaggtggttt ggtatttttc 2220

gttggcgggt tattataatt tcgtttgttt aggtataagt tgaagttgat tgttaaaatt 2280
gttataaaat cgtaggtttg tttagaattg ttttcggttt ttttagacgt tttcgggttt 2340
aattatggtt ttaaaagttt taatataagt gtaagaattt gtttgttttt atttaggggc 2400
ggggcgagtt tcgtaatggt ggcgtttttg ttatggtaaa gttattgaaa ttttttatgt 2460
attttttttg ttcgaaggta ggggtggcgt ggattgggtg ggttgtagag agagagtttg 2520
ggatagttaa aattaagtta gggaatagtt cgaatatcgt tgtttgaaat tttttttttt 2580
gtttttttata tttttgtttt aaaaaagaga tgttgtggag aaataaatat tttgttttgt 2640
tggttattgt tggaaataga agttttttcg ttgtagggta attttgtaaa tgtgtttttt 2700
agttttagga ggtttgaggt tatttttttt taataatttt tttttttatt ggattttttt 2760
atttatagta ttgttgtttt ttggataagt tatagtggat aaatatgtta agttgaagat 2820
gtataaataa ttttaagttt agtttttagg gatttaaagg atatgaaaat ttttgtagta 2880
aattttgaat aaagagttta ttaaattaat gtgtttaagt agtttgattt gtgataagta 2940
atttttgtaa aggtggtggt ggggggaggt ggagagttat ataaatatta atgttaataa 3000
attagtttat tcgattatag agtaattatt ttatattatt gatatttata gtaggtattt 3060
aaatgtaatg gtaggttatt atttggagaa aatatataat aagaatattt tttttttagt 3120
gtaatatacg attagatttg ttattgagtt agttatagtt agtttagtaa taaataaata 3180
aatcgatgtt gatatttaaa tattaaagat tttagagttt atattttaaa tttttttaag 3240
atatgtaaat aattttggtt attttttgga gagggaaaaa taaaaggtta tttttttata 3300
tattttttta ttttttttta gtaatatttt agattttttt aagaagagag ttgttgggag 3360
gttttaggtt tgggtttttt ttagtttttg gttttgtttg gttgttttgt gttttgtgtt 3420
tttttttttt tttcgttttt tttaaatatt gtttttttaa ttttgtagga tggggagtat 3480
attttgtttt tttaatttat tttttttttt ttttttaaga aagttagatt tagagtattg 3540
ttatggtttt tttttatttt tagtataaat ttagtttttt aaagatattt ttgttttttt 3600
aggtgtaggg agttcgggaa gtattttttt tttttttggt attgtatttt ttttgtggaa 3660
tgagtagtag gaaaggtata gagttttttg agtttttgtt ttgtatattt tttgttttta 3720
tttttatata ttgtaaggaa ggagagtagg agagtaggtg ggttattttt tttttagtta 3780
tttttttttg gtttttagtt cgtttttttt atttttattt tttttatatt tttggagttt 3840
tgtaagtagt ttgatgggtt ttttacgaag atgtagtata tttaggagaa gttttttcgg 3900
atgttagcgt ttttaaagta gtttaaggtt tgttttaatt gtatggtttt tcgagttttt 3960
agttttagaa gattaggtag atgggtggat cggtgagtag tagggtagtt tttgtgtttt 4020
tgtttttgtc gagttatttc gaagttcggt aggtagcgag gaggagggag tttttttaag 4080
gatagaaggt gggatgaaga ggtaggtagg gaagatgagg ggagaggtgg atttcgggta 4140
agacgaaggt tttttcgggt tttgcggatt agtgataaat cgcggggaga agtcgttttg 4200
gttgttggcg gtttagggac ggaaggtatt aaagcgtttc ggaagtgatt atgaatgaga 4260
gagtgtaatt aagttatcgt gtaaatcgga taagttatta ggtaggtata tttacggttt 4320
taaattttgg tttcgaaggg gtttcggtta gggtcggagg tagaggcgtt ttttagagta 4380
agtttatggg agggggattg cgaagaaggg ggtgtaaatg cgagatttta ggagaataga 4440
tttcgagatt ataggttata tagcgacgga tttttatttg gttattttta gtttagggta 4500
ttttttattt attcggggag ttgcgggtgg gaggtgggga cgagagttga gtttttatcg 4560
ttttttgtat attcgagaac gaggattttg taattgagta taagtatgtt gtatgggggc 4620
gtattttagt tttttcgcgc gcgtcgggag gttttttagt taatatgagt tatatcggcg 4680
attacgtgtt ttcggtgaga atatcgagtg acgatttgtt gttttttttg aggtggttat 4740
aaagaaagga agtcgggagg gaggggagag gaggaaaaaa aaaaaaggaa aggggggggg 4800
aaaaggttcg gattagttag tagtttgtta attttaatat cgggttatat gattagtatt 4860
tttttgttaa ggatggggat agatttttac gttagtttac gtttttaaat ttttatttta 4920
cggattcgtt ttaaagaggt agttgtagtg gagaattatg ttaagttcgg ttattgcgga 4980
gagtttaagg tagtttaata atggattttg atgagcgtgg tttttgtttt tttaatatgt 5040
atttgttaag ttgtatttat tacgtttcgg gtttagattt ttttagtttt tttttttttt 5100
tgttttagat ttcgttttcg cgtttaatga tatattttta tttttttaat ttgttttagg 5160
tttaattcgt gcgcgaagtg atttttagag agtacgttat tgagttcgtt attaaatggt 5220
attttcgcgg taatttggtt tattgttatt tcgcggagga gttcgtgtat agagattgtt 5280
tgtaggcgtt tagcgcggtc ggcgtgtttg gcgacgtgtt ggttaagagt tcggttaacg 5340
tttattatta ttttattttc gtagtttcgt ttaattttta tagtatcgtg ggtaggaacg 5400
gcgttttgtt ataggttttc gattagtttt tcgagatagt ttacggtatt tcggaaaatt 5460
tcgttttttt cgattatttc ggggataaga gcgtcgagaa ggggttttcg gcggttacgg 5520
cgattttcgc ggcggcggcg gcggttgtaa cgggcgcgtc ggtaatttta agttcggata 5580
gcggcggcgg cggcggttgt cgggagacgg cggcggtagt agaggagaaa gagcggcggc 5640
ggcgtttcga gagtagtagt agtttcgagt cgtttttcgg ttatattgag gataaggtcg 5700
gcggttttag tacgtataaa ggtagcgttt tgcgttttat gaaaggggag ttggaaattt 5760
agcgtagtat cgttgttaaa tttttatatg ttgttttata agtatataag gtttatgaag 5820
ggtttttagg ttttttttaa taaaattttg ttataaaagg cgggattacg tggcgagtgt 5880
tgaaattggt cgtgaaatat ttatcggtta aggtatgttt gtaaaaaaaa ataatttttt 5940
tttttttttt tttttttttt tagtttttaa gtttagtagt tcgatttcgt tcggggttag 6000
ttttaggttg gggagtcgga ggtgggcgtt tgtaaatttt ttttgtaagg gtttcgggag 6060
tttttgatag cgaggtaatt cggagatttt tataaaagtt atgtgttgcg tcggattttt 6120
agtttcggtc gggatttaaa ggcgtcgttg tttaacgacg gcgttaggag cgcgtttaat 6180

agataaagta gttcgtttag ttttagtaat atattaaaag aataaattaa tttaaagttg 6240
gtttttttgt ttaaaggaag ttattttttt ttggcgtggt ggtaattacg tgtttttttt 6300
tgtttaattg tatgttattt ataattgtta tttaatttgt taaaagcgat tttttttttg 6360
ttttgtgggt tttttttttt tttttttttt tttttttaaa gggggagtaa gtagagtttg 6420
ttcgtagttt cgggaagttt tttttttttt tattttattt aagatagagt cgttgttttg 6480
tcgtgtgtag gatggtcgtg tggtcgtggt gtttagcgaa ggtttagggt agggggtttt 6540
cgtcgttatt tttttatttc ggcggagtcg gaaggttggt cggggaggta gaagttggtg 6600
gttaggattt aggttgataa agattcggta gtcgttttag atgttaagga ttgtttagtt 6660
ttggtcgtag tcgggaaaga ttgggattgg agtcgttttt attgttttgg gaaggggtag 6720
tttaggtgtt ggaaggtgaa atgttcgtag gcggttgttt gtttagtttg gtttgcgaga 6780
ggtagtcggt aatttttatt tggttgtttt taaagaggtt taggtagcgt aaattcgttt 6840
gttgttgtgc gaagtttttt tttttgtttt gggggtagcg ggagtagttt tttcgggggt 6900
ttatttaggg agttggtttt tatttgtggt tgagttttta attgcggtcg ttttaagtat 6960
gtagtcggag cgttaaaggt aggggtttgg gtagattttg acggggtttg ggttggatta 7020
ttagttttta tggtagtttt tttttttagt tatgggtttt ggggtggatt gtagttttta 7080
tatttggttt attttatgtt tttttttttt ttatagg 7117

<210> 130
<211> 7117
<212> DNA
<213> Artificial Sequence

<220>
<223> chemically treated genomic DNA (Homo sapiens)

<400> 130

tttgtggagg gagaaaaggt atggggtgag ttaggtgtgg gggttgtaat ttattttaga 60
gtttatagtt gaggagaagg gttgttatgg ggattggtgg tttagtttaa gtttcgttaa 120
agtttgttta ggtttttgtt tttaacgttt cgattgtatg tttggaacgg tcgtagttgg 180
aggtttagtt atagataaaa gttagttttt taaataggtt ttcggggaag ttgttttcgt 240
tgtttttaga atagaaagga gagtttcgta tagtaatagg cgagtttgcg ttgtttaagt 300
ttttttgaaa gtagttaagt gggggttgtc ggttgttttt cgtaggttag attaaataaa 360
tagtcgtttg cggatatttt attttttagt atttgggttg ttttttttta aggtagtaaa 420
ggcggtttta gttttagttt ttttcggttg cggttaagat tgggtagttt ttaatatttg 480
gggcggttgt cgggtttttg ttagtttgag ttttggttat tagtttttgt tttttcggtt 540
agtttttcgg tttcgtcgag gtggggaggt ggcggcggaa gttttttatt ttaggttttc 600
gttgagtatt acggttatac ggttattttg tatacggtag agtagcggtt ttattttagg 660
tagggtgaag ggaaaggggg tttttcgaag ttgcgggtag gttttatttg tttttttttt 720
aaaaaaaaaa aaaaaaaaaa aaaaaagatt tataagataa aaaaaaaatc gtttttgata 780
gattgaataa taattgtgaa taatatgtag ttgggtagaa ggaggtacgt aattgttatt 840
acgttagagg aaaatggttt tttttggata aaaaggttaa ttttgggtta atttgttttt 900
ttaatatatt gttagagtta agcgggttat tttatttgtt aaacgcgttt ttagcgtcgt 960
cgttaaatag cgacgttttt gaatttcggt cgggattgga gtttcggcgt aatatatggt 1020
ttttataaaa attttcggat tatttcgtta ttaaaagttt tcgaagtttt tgtaggggga 1080
atttataggc gtttattttc ggttttttag tttggagttg gtttcgagcg gggtcgagtt 1140
gttgggtttg ggagttgaga agggaaaaaa gggaaaaggg gagttgtttt tttttgtagg 1200
tatgttttgg tcggtgggta ttttacggtt aattttagta ttcgttacgt gatttcgttt 1260
tttataataa agttttgttg ggggaaattt aaaggttttt tataaatttt atatgtttat 1320
aaaatagtat ataaaaattt aatagcggtg ttgcgttaga tttttaattt tttttttata 1380
aagcgtaggg cgttgttttt atacgtattg gagtcgtcgg ttttgttttt agtgtggtcg 1440
gaagacgatt cggggttgtt gttgttttcg gggcgtcgtc gtcgtttttt tttttttgtt 1500
gtcgtcgtcg tttttcggta gtcgtcgtcg tcgtcgttgt tcgaatttga agttgtcggc 1560
gcgttcgttg tagtcgtcgt cgtcgtcgcg gaggtcgtcg tggtcgtcgg gggttttttt 1620
tcggcgtttt tgttttcggg gtagtcggag gaggcgaggt ttttcggggt gtcgtaggtt 1680
gtttcgaaaa attggtcgaa agtttgtggt aggacgtcgt ttttgtttac ggtgttatag 1740
aaattggacg agattgcggg ggtggggtgg tggtagacgt tggtcgagtt tttggttagt 1800
acgtcgttag gtacgtcggt cgcgttgggc gtttgtaggt agtttttgtg tacgagtttt 1860
ttcgcggagt agtagtgggt tagattgtcg cgggggtgtt atttagtggc gggtttaatg 1920
gcgtattttt tgaaggttat ttcgcgtacg ggttggattt ggggtaggtt ggaggagtag 1980
gagtatgtta ttgggcgcga agacggggtt tggggtagaa aagaagggag gttggagaaa 2040
tttggattcg agacgtagta agtataattt ggtaaatata tgttagagga gtagggatta 2100
cgtttattaa aatttattat tgggttattt tgggtttttc gtagtagtcg agtttaatat 2160
gattttttat tgtagttgtt tttttgaagc ggattcgtga agtagaaatt tggagacgta 2220
agttgacgtg gaaatttatt tttattttta gtagggaggt gttggttatg tgattcgatg 2280
ttgaaattga taagttgtta gttagttcgg gttttttttt tttttttttt tttttttttt 2340

tttttttttt tttttttttt ttcggttttt tttttttgta gttattttag gggaagtaat 2400
agatcgttat tcggtgtttt tatcgaaagt acgtaatcgt cggtgtaatt tatgttggtt 2460
gggggggttt tcggcgcgcg cggagaggtt ggggtgcgtt tttatgtagt atgtttgtgt 2520
ttaattgtag ggttttcgtt ttcgagtgtg tagagggcgg tgagagttta attttcgttt 2580
ttatttttta ttcgtagttt ttcgggtggg tgagggatgt tttggattgg ggatagttag 2640
gtgggagttc gtcgttgtgt ggtttgtggt ttcggagttt gtttttttgg agtttcgtat 2700
ttgtattttt tttttcgtag tttttttttt atagatttgt tttgggaagc gtttttgttt 2760
tcgattttag tcggaatttt ttcggggtta gagtttgaag tcgtggatgt gtttgtttgg 2820
tggtttgttc gatttgtacg gtgatttgat tatatttttt tatttatggt tattttcgaa 2880
gcgttttagt gtttttcgtt tttaaatcgt taatagttag aacggttttt tttcgcggtt 2940
tgttattgat tcgtagggtt cggaagggtt ttcgttttat tcgggattta tttttttttt 3000
tatttttttt gtttattttt ttattttatt ttttgttttt ggagaaattt tttttttttc 3060
gttgtttgtc gggtttcgga gtgattcggt agagatagag gtataggggt tgttttgttg 3120
tttatcggtt tatttatttg tttggttttt tggagttgag gattcgggaa attatgtaat 3180
tgaggtaagt tttgggttgt tttagaggcg ttgatattcg aggagatttt ttttgggtat 3240
gttgtatttt cgtggggggt ttattaggtt gtttatagag ttttaggggt gtggggagaa 3300
tggaggtgga aaggacgggt tgagggttaa ggagaggtgg ttgagaaagg ggtaatttat 3360
ttattttttt attttttttt tttgtaatgt gtgagagtga aagtaaggga tgtgtagggt 3420
aaaaatttag agagttttgt gtttttttta ttgtttattt tataggagga atataatatt 3480
agagaaagga gaggtgtttt tcgaattttt tgtatttggg gaaataggga tgtttttaaa 3540
aagttaggtt tgtgttaagg atagagagag gttatagtaa tattttgagt ttagtttttt 3600
tgagaagagg aaaaaaaata aattaagaag gtaaaatatg ttttttattt tgtaggattg 3660
aaggagtaat gtttggagga agcgaaagaa aggagaggat atagagtata gagtagttag 3720
gtagagttag gagttgagaa gggtttagat ttgaggtttt ttaataattt tttttttgga 3780
aggatttggg atgttgttga aggaaaataa aaaaatatgt aaaaagataa ttttttgttt 3840
tttttttttt aggaaatagt taaagttatt tatatatttt ggggagattt agagtataaa 3900
ttttaagatt tttggtattt aagtgttaat atcgatttat ttatttattg ttgagttgat 3960
tgtaattgat ttaataataa atttaatcgt gtattgtatt ggaaaagaaa tatttttatt 4020
atgtattttt tttaaataat ggtttattat tgtatttgaa tatttgttgt aaatattaat 4080
aatatgaagt aattattttg tagtcgagta aattaattta ttagtattaa tgtttatgtg 4140
gttttttatt tttttttatt attattttta taaggattgt ttattataaa ttaagttatt 4200
tggatatatt ggtttaatga attttttatt taagatttgt tataagaatt tttatgtttt 4260
ttgaattttt gagaattgaa tttgaaatta tttgtgtatt tttagtttga tatatttgtt 4320
tattgtggtt tgtttagagg gtagtagtgt tgtgagtgag aaggtttagt gggaaggaag 4380
gttattggag aagagtagtt ttagattttt taaagttggg aagtatattt atagaattgt 4440
tttgtagcga aaaaattttt atttttagta gtgattaata aggtaaaatg tttgtttttt 4500
tatagtattt tttttttaga gatagaatat aaaagataga aggagaggtt ttaaatagcg 4560
gtattcgagt tgttttttgg tttgattttg gttattttaa gttttttttt tgtagtttat 4620
ttagtttacg ttatttttat tttcgaataa aaggaatgta tgaagggttt tagtgatttt 4680
gttataataa aggcgttatt attgcggggt tcgtttcgtt tttgggtgaa ggtaaataaa 4740
tttttgtatt tgtattaggg tttttaagat tataattgaa ttcgggggcg tttaggaaaa 4800
tcgaaaatag ttttagatag atttgcggtt ttatagtagt tttggtagtt aattttagtt 4860
tgtgtttgag tagacggggt tgtggtggtt cgttagcggg ggatgttagg ttattttttt 4920
tagcggtacg tagttttttt tttaattaga tcggtttttt tttggtgttc gggagagcgg 4980
tttcggtaga aaggtcggta tgggggtgtg cgttgtttcg tataattatt gttttttatg 5040
tttttttcga gggtcgaatc gagagggtgt tggtagggtt ggattttacg ggtgttcgta 5100
ggagataaag gcgaatttcg gagaaaaggt tggggttgag aaaggcgttt cgggagcggt 5160
tggtagggta attcggtagg ttgtatcgaa gtcgagtgtt cggagggatt tgtcgttcgg 5220
aagggggtgt gtgggggcgt tgtcgtgaag atggatgagg gaaaaggttt ttgatattag 5280
tagaagggaa aacgtttgga gtggtcgaat atttttagtt gtttagtagg aataggagac 5340
gggtatttag ttttttaagg ttgcgtaata ttttagtttt gttcgttttt gttttcgtgt 5400
tcggaatatg ttggcggtgt gaatgtgaag gtttttttag gtaggcggtt ggggcgaggg 5460
gtgggcgtag ttttgaggat tagattaaat ttaggggaaa aaggggaggt agattttcgg 5520
gtttttggtt ttgattttgt tttgagttta tgattgattt tttcgttttg taggaggttt 5580
aatagtcgag tttagtttat cgggttttgg gaaagattcg attgaggtta aagtcgtttc 5640
ggaaggttaa gttcgagttt tattttttga agaggtcggc gcgcgtaagg ttgtgatatt 5700
ggttttggcg attggttttt taggagttgt ttttttttag gagttttata gcgcgggtta 5760
tttttagaaa attgttttta gagtgtattt ttttttatcg ttaatttaga gttttatcgc 5820
ggttaatgta agaggttaaa aaatgtttgg aggaagaaaa ataaaggtag gaagtggcgg 5880
cggtttgacg gtgcgtgtgt gtttgtagag aagggaggga gtcggtttag tttttttttg 5940
tttttttaaa ttttaaggtt taggtagttt tttgtagggt cgggttttat tgtttttcgc 6000
gcggtattgg aggtggtcgt tcggagagga gaaggttaac gtttgcgtta ggtttgttag 6060
gcggaaacgg ttaataagga gatttggtta gtaaaataga tttagttttt tcgaggtttc 6120
gtttgatttg gttcgaaagg ttggggaggg ggggtttgcg tagagtttta gggatttttt 6180
tttttgggga ttattatttt tgagttttac gttttttttt atagtttttg taggcggaat 6240
atcggaataa agtgggttta ggcgtttttg tcgttttcgt ttttttttgt agtttgaatg 6300

gttcgggagg tagcgggagg gcgtcggcgg gtagtgttta ggcggggtcg tttcgaggtc 6360
gtcggtgagt atcgggtaat gcgaggtttt gttatcgagg ttgttgtgtt gggggcgttt 6420
ttagtatagt tatttaaggg acgtgagttg taaggatcgt tatttgatag gcggtttaat 6480
atttttttat tttaagggag gatatagttg ggtggtcggg agtattttta tattttaggt 6540
tttttagttt taattggtaa agttgttggg ttattttttt ttttttttgg ggttggtttg 6600
ggaggtagta gttgtttttt gttttagaaa tttgagaatt cgaagttttt agtatttgta 6660
tatgtagttt tttaaatgtt tagtatttgt ttatagtttt aatgttaatg ttaagttgtt 6720
ttagttatta ttgttttttt taaggggttt ttgttttttt atagtttttg atttgttttt 6780
tgtttttttt atagtttttg ttttttgttt tgtttttttt tagttatttt tatttgttgg 6840
gatttttgt tttttttta tttttttga tgttttgtt agattgagta atagttgagg 6900
tgttattgga gggtatttaa tatttaggcg tagagaagag gggttgttag gtttttgatt 6960
tttttttaat ttcgggtttt tattagagtt tgaattgagt tttttatttt ttttttagga 7020
atattttatt ttaaggtttt attaagtttg gtttattttt tttttttttt tttttttttt 7080
ttattttttt ttttttttta atattttttt ttttttt 7117

<210> 131
<211> 6636
<212> DNA
<213> Artificial Sequence

<220>
<223> chemically treated genomic DNA (Homo sapiens)

<400> 131

tgtttttagt tttagtgaag agcgattagt ggtattgtcg gggaaaagag tttttttatt 60
attacggtcg ttgtcgcggt tgtcgtcggt ttcggagtta tttaggtttt ttagagtcga 120
gttttggagg acggagtttg cgttagtttt gaggagttcg ggcgcggttg gttaaggttt 180
tgtagtggga gatgtttttt atgggagttt ggaatttggt tgttttgaaa tggtgtgtgt 240
ggggtggagg tttttttgtt tagggatttt ttgggggaaa taaggtaggg ttgtatttaa 300
attggagttt tttttgggtt tttgtcgttt tttttgagtt tttgtgtatt agtggttttg 360
tggtatttga aaaaataaaa taaaataaaa aattttatag gtagtaggag agttgttttt 420
ttaaaaattt gttgtttttg agatttattt tatgaaggga ttttaaaggt aaagtggggg 480
tttttgtaga tttttttgga ttttgtattt ttatttttat tatgttaata tttggttatt 540
atagtgataa tagtttaaaa ttattttata gttttaagtt ttttattgtt ttattattgt 600
tttaaatgta taaaaatttg gaataaaagg ttgagtgagt gattcgatgt attatttttt 660
tgttcgttgg ttaattttag tttagatttt aggatttatt aatttttttt ttttaagaaa 720
gagggtatag tttttatttg atggggatat ttaaaatttt ttttataagt agagtagttt 780
tttatttgtt ttttttttaa atgatttgtt taagtttggt tttagaggtg ttatattgtt 840
tgacgatttc gttttttttt tttttttttt tttagttttt taattagttt tttttttgga 900
tttttttttt tttgttatta tttttgtgtt ttagtttatt tagggtggga gagagagggt 960
taagatgttt tgtaaaagtg gtttgtaaag ttgtttggat atattaaaag tatatgaata 1020
ttttgaaata taaagtgttt ttagaattaa ttaaggaatg tttattttag tgttggatgg 1080
atgtaaagaa atagtgttag ggagtagtgt tgatagtttt tttagagtta aagtgagagt 1140
aggtaaattt tatttgttaa tgaaaattaa gttcgattaa aatttggttt tttttttttt 1200
tttttttttt taatgttggg agtttagagt cgcgtggatt aggttggtat ttagtttttt 1260
atttagaaaa atttagttgc ggtttttttt tttagtgaga ggtttttggg taattagttt 1320
ttgtagggta ttttggttta ttttaggaag taagaagttt tgttagttaa ggtattttgg 1380
aatattgaga aaggttttgg tgatttttga aataaaggtc gtgttgtaga aattattttt 1440
cggttttgtt tgtattgttt ttttagggtt ttttcgggga aattagtgta ttagatagta 1500
tttgggaagt gattgtagtt ttttttattt tttttatttt tatttatgta gttttttgtt 1560
tggttgttat gtttaaggtg ttatcgttgt cggtcgattt tagtgggtgt tttggttttt 1620
ttagtttaat ttaggaggag tttgagaata taaaattatt aggttgaagg tgtttttgta 1680
attttatagg aatttttgag tttggagaat aaaaaattag agggatagat agtttagaag 1740
attaggattt attattttgg taggtaagta aattttgatt ttgtcgttgg gaaaagagga 1800
aaaatgttag gagggtttgt gtttgttttt tagagtatat tcgttttaat cgttttttaa 1860
attttttttt gttatagtaa gttagtttgt tggttgtttg gtagggaaag aaattatatt 1920
gggaaatatt aataaatatt ttattttttt tagtagaaga tagggaagga agattatttt 1980
tgtttaaaag tgagtagaaa ttttgaaatt tagatttaaa ttatttagag ttcgtttttt 2040
ttttataaac gatttttatt gattggtggt tataatttaa tattgatagg atatgtagat 2100
atttgaaaat agtggggtat gttaggacgt tatggatatg tttggtagaa aaataagata 2160
tatgttgaga tttaagataa gaagagaata agtagggtgt ttttttttaa ataagtatgt 2220
tttgagaggg gttttttttt tttttttttt tttttaatga tagggatata tcggggaatt 2280
aaaaaaagtt tagataaatt tgattaataa ggtgtattat aaattttaag aatttagttt 2340
ttaagttttt ttttggcgag agcgttatat ttaaggagta aagtttaatt tgagatgtaa 2400
tttaaagttt ttttttagta ttattttttt tagataaatt tgtttgtggt ttttcggtcg 2460

atagatgaaa ttttgtttta ttatttttgg tgaggaggga tttagtcgtt tgtggagttg 2520
agattttttt tttgcgcgta gggttcgggg ttacggtgaa gtgggagggg gagggaagaa 2580
tgtggagttg gttttggtcg tcgtgtgtat cggtagttgc gttatggtta gaggtgagtt 2640
ggtataggcg tcggttagtg ggggtttttt aagttgtgtt ttcgtttttt cgggtttggt 2700
cggaaaatcg aagaggttta aagttttggt tgagtagggc ggaggtagag aagagtcggt 2760
aaatatcgtt ttaaaaattt tacgaaggta ttaaaaaatt ttgggtagtc gaatagagtt 2820
agaggcgaga atttaaaaaa gagagagaaa attaagagtg aggggatgtg ggtaatgggt 2880
tttttgttgt tgtttgtgtt tttacgttcg tatggtagtt tttatgttgg ttggattgta 2940
atttttaatt tgattttggt ttttagtcgt gtttgattag tatgtaaaat cgtttttaag 3000
tttttaggaa taggggttta gaattttatt ttgttttttt gttattgtga gttttataat 3060
gttatttgga gggaggaagg ggggtggtga aacggttaga aagtaataga aaaattaagt 3120
ttttatagag gttagttttt gagtttgtaa atggttagtt agggagaagg taaattgggg 3180
gtaaattaat atttatttag aattgttaaa aagttaagaa ggaaagggta tatttggtgg 3240
gggggtggtt ggtgtggggg ttatagaagg ttttattggg cgatatttat attggttagg 3300
ataaatttag gattaggata gataaaagtt taagtttagt tatattggtt tggattttgt 3360
aggaagttta ggggttttag atagtagatg gtggggtgtg tgtgaggggt ggtatgcgtg 3420
ttgttttgag tttagggttg agtgtgtaag ttaggagtag aggataggtt taagtttggt 3480
tttttattag ttcgtttggg gtggaaaaga atttagtatt ttttttagta ttatttcggt 3540
aatgtgttta tttttatttt gaacgtttgt tttttttttta ggtatttgat tagtttatag 3600
tagtgaatta ggaagtggaa gtatgtggtt ttaaagtagt aggattttta gaatttttcg 3660
ataaaatatg gtagttataa gacgggtcgt tgtttggttt tttagtatta atattttatt 3720
atgtttttaa gaaaaggttg tattttttag gtatagagga aaggtttttt ggattggttt 3780
ttgggtaaag aaggattagg tattttttgt aggttggaga ttttatttga ggggaaggta 3840
ggaagtggtt ttaaagagtt tttttttttt gttttaaaat agagtgaaat agcgggtttt 3900
tagatttttg gagagttagg ttcggtttat aaagaagcgt ggttttaggg tttagaaggg 3960
gggtttaagt tttagttaga agtatagttg ggtttgtttt tttttaagtt tttttttagaa 4020
ttaagttagt tatattggcg aggtgggagt atggatgaat ggtgtatagg gatcgttcgt 4080
tttattttaa agtttttgga ggaggaagcg ttgttattag gttagagtaa ggattaggtt 4140
gtatatttaa gtgtgcgaat gtgagtgtgt tagtgtatat tgtgtgtgtg tgtgatgagt 4200
cgttttgagt ttatgggttt aaatgtaaat atatgaaatt gtttattaat agtgtggata 4260
gggaagggat aggggttttt aaatgtagtt taagttttta gtggaatgtt ttgggattta 4320
ggtatattcg taggaatcga taagtagtaa atattttgtt attgtttttt tggaaagagt 4380
aaaatcgtaa ttttatcgaa gtcgaaggcg tcgatgttcg ttttgtggtt ttattgtaga 4440
aatacgcgtc ggtttttgga aagaaaatag ttttagaggt tttaagaaat atttattttg 4500
agttaggttg gtttcgtttt ttttattttt ttatttttga tcgtcggtag aattatttga 4560
tttgttttta ttgttttaga tcgtaattta atcgcgcgtt tatttttttt tttcgtttgg 4620
ttgcgcgggt ttgggtttta ttgggataaa ggaagggaag gggggcggag atggggtcgt 4680
tttttttttt tgttttattc gtacgttttt tcgttttttt ttcgggtgaa tggtgcgcgg 4740
tcgcgcggtt ggcgttgggg acggcgcggg tcgcggcggg gacgacgggt gtatcgtgta 4800
gagttggagt cggagtcgtt cgttttgtat acgtcgtggg cggagcgggg gggtcgggtt 4860
aatgggcggt cgtttggcgc gatttcgcgg ggcgcgattc gtttttttcg ttcgggtttc 4920
ggtttcgcgt cgcgcgtttt ttattttttg gggggttttt aaaatagcgt tattggggtt 4980
ttttttatgc ggttcgggtt atgatagttt tcgtgttttt ttattttcgt tggatcgagt 5040
ttatcgttat gtttttttac gataacggcg gcggtttggt ggtcgacgag tttaataaga 5100
atatggaagg ggcggcggcg gttgtagtag cggttgtagc ggcggcggtt gtcggggtcg 5160
ggggcggggg ttttttttat tcggcggttg cggcggtagg gggtaatttt tcggtggcgg 5220
cggcggtcgc ggttgcggcg gcggtcgcgg ttaattagtg tcgtaatttg atggcgtatt 5280
cggcgttttt ggcgttagga gtcgcgttcg tttatagtag cgttttcggg gaggcgtttt 5340
cgtcggttgt cgtcgttgtt gtcgcggttg tcgttgtagt cgtcgtcgtc gtcgtcgcgt 5400
cgttttcggg aggtttcggt tcggcgggtt cggcgggcgt agaggtcgtt aagtaatgta 5460
gtttttgttc ggtagcggcg tagagttcgt cggggttcgc ggcgttgttt tatggttatt 5520
tcggtagcgg ttattattcg tgcgttcgta tgggttcgta ttttaacgtt attaagtcgt 5580
gcgcgtagtt cgtttcggtc gtcgtcgtcg tcgttttcgc ggataagtat atggatatcg 5640
tcggtttagt tgtcgaggag tttagttttc gcgttaagga gttcgttttt tattattagg 5700
gttacgtagt cgggttttat tattattatt agtttatgtt tggttatttg gatatgttag 5760
tggtgtcggg tttcgggggt ttcggcgagt cgcgttacga atttttgggt ttttttatgg 5820
aaagttatta gttttgggcg ttgtttaacg gttggaacgg ttaaatgtat tgttttaaag 5880
agtaggcgta gtttttttat ttttggaagt ttattttgtt cggtaaatga cgatttattt 5940
ttagttttgg tttttcggtt ttgttttagt ttttttttcg ttcgtattcg ggcgatttcg 6000
ggtgcgtttt tgtttttttt ttggtttgtt ttagcggttt tgtatttttg ggagttcgag 6060
tatggttggt tgggtttgtt tgtattgttt cgagttgagt tggtttttgg ttttttttgg 6120
gtgaggggtg gtttgtggag atttcggtta gttttttttt ttttttgcgt ttcgtttttt 6180
ttagtttttg atattaattt aaggatgaga aattgattag aaaatagttt tttaaattgt 6240
ttttttttat ttattttttt aaaaatggtt ttagtgtaga agtttcgagt attgggacgg 6300
gtatttagaa aggaggtagg tatagaagtg ttgtattttg agtttggcgt taaggtgtgg 6360
gtcgttggat taggttatta ttcgaggttg tttacgcgtt gtttttgtag gatggtcggg 6420

ttggggaagt tattggagtt ttgggtgatt ttattttagt ttagaattaa ttattttttt 6480
tattgatttt ttaggtttta gtagaagata ggattgtata gcgggtggta aagagtagtc 6540
gggcgttgta aggcgggtgg tttagatcga gttgtcgttt atgttttggt tggggttcga 6600
tttttgtgta atttgttttt ttttttgttt tttaga 6636

<210> 132
<211> 6636
<212> DNA
<213> Artificial Sequence

<220>
<223> chemically treated genomic DNA (Homo sapiens)

<400> 132

tttagaagat aagggagaag gtaagttata tagggatcgg attttagtta gggtataggc 60
gatagttcga tttgagttat tcgttttgta gcgttcggtt gttttttgtt attcgttgta 120
taattttgtt ttttgttaaa gtttagaggg ttagtgggga aggtagttag ttttgaattg 180
aaatgaaatt atttagggtt ttagtgattt ttttaattcg gttattttgt aggagtagcg 240
cgtaggtagt ttcgagtgat agtttggttt aacggtttat attttagcgt taggtttaag 300
gtataatatt tttgtgtttg tttttttttt gggtgttcgt tttaatattc gaagttttta 360
tattgaagtt atttttgaga gaatgaatag ggaggggtaa tttggggagt tgttttttgg 420
ttaatttttt atttttaaat tggtgttagg ggttggggag ggcggggcgt agagggagag 480
ggaagttagt cgaggttttt ataagttatt ttttatttag ggagagttag ggattagttt 540
aattcgaggt agtgtaggta gatttagtta gttatgttcg ggtttttagg ggtgtagagt 600
cgttagggta gattaggaag agaatagaaa cgtattcggg atcgttcggg tgcgagcgga 660
gaagaagttg gagtagagtc ggaagattag ggttgggaat aggtcgttat ttatcgggta 720
gagtggattt ttagaggtgg ggaggttgcg tttgtttttt ggggtagtat atttggtcgt 780
tttagtcgtt gggtagcgtt tagggttggt agttttttat gggaagattt aagggttcgt 840
ggcgcgattc gtcggggttt tcgaggttcg gtattattgg tatatttagg tagttaggta 900
tgggttgatg gtggtggtaa ggttcggttg cgtagttttg gtggtagaag gcgaattttt 960
tagcgcggga gttgaatttt tcggtagttg ggtcggcggt atttatgtat ttgttcgcga 1020
aggcggcggc ggcggcggtc gaggcgggtt gcgcgtacga tttgatggcg ttggggtgcg 1080
ggtttatgcg ggcgtacggg tagtagtcgt tgtcgaagta gttatagggt agcgtcgcgg 1140
gtttcgacga gttttgcgtc gttgtcgagt aggggttgta ttgtttggcg gtttttgcgt 1200
tcgtcgggtt cgtcgggtcg ggatttttcg aggacgacgc ggcggcggcg gcggcggttg 1260
tagcggtagt cgcggtagta gcggcggtag tcgacggggg cgttttttcg ggggcgttgt 1320
tgtaggcgga cgcggttttt ggcgttaagg gcgtcgggtg cgttattagg ttgcggtatt 1380
ggttggtcgc ggtcgtcgtc gtagtcgcgg tcgtcgtcgt tatcgagaag ttgttttttg 1440
tcgtcgtagt cgtcgggtgg gggaagtttt cgttttcggt ttcggtagtc gtcgtcgttg 1500
tagtcgttgt tgtagtcgtc gtcgtttttt ttatgttttt gttgagttcg tcggttatta 1560
ggtcgtcgtc gttgtcgtag agaaatatga cggtgggttc gatttagcgg gggtggagga 1620
gtacggaggt tgttatagtt cgagtcgtat ggagaagatt ttagtggcgt tgttttaaaa 1680
agtttttaag aagtgaagag cgcgcggcgc ggggtcgggg ttcgagcgag gggggcggat 1740
cgcgtttcgc ggggtcgcgt taggcggtcg tttattggtt cggttttttc gtttcgttta 1800
cggcgtatgt aaagcgggcg gtttcgattt tagttttgta cggtgtattc gtcgttttcg 1860
tcgcggttcg cgtcgttttt agcgttagtc gcgcggtcgc gtattattta ttcgggggag 1920
aggcggagga acgtgcgggt ggggtaggga aggaaggcgg ttttattttc gttttttttt 1980
ttttttttta ttttagtggg gtttagattc gcgtaattag gcggggaggg gaggtgggcg 2040
cgcgattggg ttgcgatttg gagtagtggg gataggttag gtaattttgt cggcggttag 2100
ggatgggaga gtggggagga cgggattagt ttagtttaga atgggtgttt tttggagttt 2160
ttgggattgt ttttttttta ggaatcggcg cgtatttttg tagtgagatt ataggacgga 2220
tatcggcgtt ttcggtttcg atggagttgc gattttgttt tttttaggga aatagtggta 2280
gggtgtttgt tgtttatcgg tttttgcgga tatgtttggg ttttaggata ttttattgga 2340
ggtttggatt gtatttagga gtttttattt tttttttgtt tatattgtta gtgagtaatt 2400
ttatatgttt gtatttagat ttatagattt agaacgattt attatatata tatatagtgt 2460
atattgatat atttatattc gtatatttag gtatatagtt tgatttttgt tttgatttgg 2520
taataacgtt ttttttttta gagattttga gatagagcga gcgatttttg tgtattattt 2580
atttatgttt ttatttcgtt agtatggttg gtttagtttt ggaaggggtt taagaggaat 2640
aagttttagt tgtgtttttg gttgggattt aaattttttt tttgggtttt aaagttacgt 2700
ttttttgtgg atcggatttg attttttagg aatttgggaa ttcgttattt tattttattt 2760
tgggataaga aaaaggggtt ttttggggtt attttttgtt ttttttttaa gtaggatttt 2820
tagtttgtag agggtgttta gttttttttt gtttaagaat tagtttaaga agtttttttt 2880
ttgtgtttgg gaaatgtaat ttttttttgg gagtatggta gggtgttggt gttgaagaat 2940
taagtagcga ttcgttttgt agttgttatg ttttgtcgag ggttttgggg ggttttgttg 3000
ttttagagtt atatattttt attttttgat ttattattgt gagttggtta gatgtttaga 3060

agaggaataa gcgtttaaag tgaaagtggg tatattatcg gaatagtgtt ggggagagtg 3120
ttggattttt ttttatttta ggcggattgg tgagaagtta ggtttggatt tgttttttgt 3180
ttttagtttg tatatttagt tttaaattta gagtagtacg tatattattt tttatatata 3240
ttttattatt tgttgtttaa ggtttttggg ttttttgtag gatttagatt aatgtggttg 3300
ggtttgggtt tttatttgtt ttgattttgg atttgttttg attaatgtaa gtgtcgttta 3360
ataaaatttt ttatgatttt tatattagtt atttttttat taagtgtgtt tttttttttt 3420
tgatttttta gtagttttgg gtaaatattg atttgttttt agtttatttt ttttttgatt 3480
ggttatttgt agatttagga attagttttt gtagggattt gatttttttg ttattttttg 3540
gtcgttttat tatttttttt ttttttttta agtggtattg taaaatttat agtgataaag 3600
agatagagta gggttttagg tttttgtttt tggggatttg aaggcggttt tatatattgg 3660
ttagatacgg ttggaggtta aggttaagtt gaaagttgta gtttagttag tatgagaatt 3720
gttatgcgag cgtagagata taggtagtag taaaaggttt attgtttata tttttttatt 3780
tttaattttt tttttttttt taaaattttc gtttttgatt ttgttcggtt gtttagaatt 3840
ttttggtgtt ttcgtggggt ttttggggcg gtgtttatcg attttttttt gttttcgttt 3900
tgtttagtta gggttttgag ttttttcggt ttttcggtta gattcggaaa aacgaaaata 3960
tagtttgggg agtttttatt agtcggcgtt tgtgttagtt tatttttggt tatggcgtag 4020
ttgtcggtgt atacggcggt taaggttagt tttatatttt tttttttttt ttttatttta 4080
tcgtagtttc gaattttgcg cgtagagaaa gggttttagt tttatagacg attgggtttt 4140
tttttattaa aaatggtgag ataagatttt atttgtcggt cgaggagtta taagtaggtt 4200
tgtttgagag ggatggtgtt gggggaaggt tttggattgt attttaaatt aagttttgtt 4260
ttttaaatgt ggcgttttcg ttaagaaaaa gtttggggat tgaatttttg agatttatgg 4320
tgtattttat tgattaaatt tatttggatt ttttttagtt tttcgatgtg tttttattat 4380
taaaaaaaaa aaaaaaaaaa aaaaattttt tttaagatat gtttatttag aggaaggtat 4440
tttgtttgtt ttttttttgt tttggatttt aatatgtatt ttattttttt attagatatg 4500
tttatggcgt tttggtatgt tttattgttt ttaaatgttt gtatgttttg ttagtattga 4560
gttgtagtta ttaattagtg gaggtcgttt atagaaggag aacgagtttt gaataatttg 4620
ggtttgagtt ttagaatttt tatttatttt taagtaagaa taattttttt tttttgtttt 4680
ttgttaaggg agataagata tttattaatg ttttttaata tgattttttt ttttgttagg 4740
tagttaataa attgatttgt tgtggtaaaa gaaagtttgg aggacgattg agacgggtgt 4800
gttttgggaa gtaagtatag atttttttag tatttttttt ttttttttaa cggtaaggtt 4860
aagatttgtt tgtttgttaa ggtagtgggt tttgattttt tgagttgttt gtttttttga 4920
tttttttgttt tttaggttta ggaattttta taggattgta gaggtatttt taatttgata 4980
attttatatt tttaggtttt ttttgggtta gattggggaa gttagggtat ttattaggat 5040
cggtcggtag cggtggtatt ttaggtatga tagttaagta gagggttgta tgagtagagg 5100
tgggagaggt gggagaggtt gtagttattt tttaggtatt gtttggtgta ttgatttttt 5160
cggaggagtt ttgagagggt agtgtaggta ggatcggagg gtagtttttg tagtacggtt 5220
tttattttag gaattattag agtttttttt agtgttttaa aatgttttgg ttgataaggt 5280
tttttatttt ttggggtagg ttagggtgtt ttgtagggat tggttgttta aaaatttttt 5340
attgaagaaa aaaatcgtag ttaggttttt ttgggtgggg aattggatgt tagtttggtt 5400
tacgcggttt tgggttttta gtattggaga agaaaaggaa aaagggaaat taggttttaa 5460
tcgagtttga tttttattag taaatgaaat ttatttgttt ttattttaat tttgaggagg 5520
ttgttagtat tgttttttgg tattattttt ttgtatttat ttagtattag gataaatatt 5580
ttttggttga ttttaaagat attttgtatt ttaggatatt tatgtgtttt taatgtattt 5640
aagtagtttt ataagttatt tttgtagaat attttaattt tttttttttt attttaggtg 5700
ggttggggta tagagataat ggtaaaggaa gaaagattta gaggaaaaat tggttgaggg 5760
gttgaggaga gagaaagaga aaagacgaaa tcgttaaata gtgtagtatt tttgaagtta 5820
aatttgagta gattatttgg gaaaggagta agtgggggat tgttttgttt atggagggaa 5880
ttttgagtgt ttttattagg tagaagttgt attttttttt ttaaggggaa agggttggta 5940
ggttttgaga tttgggttga agttggttag cggataggga aatggtgtat cgagttattt 6000
atttaatttt ttgttttaga tttttgtgta tttgaaataa taataaggta gtaaaaggtt 6060
tgaggttgtg ggatagtttt gggttattgt tattgtggtg attaaatgtt aatatggtga 6120
aagtggaagt gtaaggttta ggaagattta tagggatttt tattttgttt ttaagatttt 6180
tttatggggt ggattttaag gatagtaggt ttttggagga gtagtttttt tgttgtttgt 6240
agagtttttt gttttgtttt gtttttttag gtattataaa gttattagtg tatagggatt 6300
tagaaaagac ggtaggagtt taaggaaaat tttaatttga gtatagtttt gttttgtttt 6360
ttttagagag tttttgagta aggagatttt tattttatat atattatttt agaataatta 6420
ggttttagat ttttatgagg agtatttttt attgtagagt tttggttagt cgcgttcgga 6480
ttttttagag ttggcgtaaa tttcgttttt taaaattcgg ttttgggagg tttaagtgat 6540
ttcgaagtcg gcggtagtcg cggtagcggt cgtggtggtg gaagagtttt ttttttcgat 6600
agtgttattg atcgtttttt attggagttg gaaata 6636

<210> 133
<211> 6036
<212> DNA
<213> Artificial Sequence

<220>
<223> chemically treated genomic DNA (Homo sapiens)

<400> 133

attttttgta aagagtgaga aaaaaaatta aaggcgtttt gaaatagggg ttttttgtgt 60
aaggatgatt aagtgtacgt tttttcgtgt gtgtatgttg gtgaatagtt agatttattt 120
atattttttt gtaagtattg aataatttaa gttttaaata ttatttattt ttattcgttc 180
gtatttatat taaagaattt tgtattttga tggtttagaa gggtttttgg gttttttgtt 240
taattgtgta ttggcgtatt tagaattttt tttatttgaa agagaaatat aattttttta 300
aacggtaacg atataataaa attagagaag atttagtttt tgaaaatagt gatttaggtt 360
tgtaatattc ggtaaaattg aaaaaaaaaa tttgtaaacg cgaaaaatat tagatttgtt 420
ttgagagttt tttatttttt gttgtttata ttttgagaaa taaaaagaaa taaagttttt 480
attttgaata atattcgtgt taagaagggg ttttttggtc gaagacgtgg gtttgcgtgg 540
aatttaggtc gaggcgagtc ggtagagtag gtcggacgta gtagtttttt ggttttagta 600
tggggtttgg ttaggttatt cgtttggaag ttcggcgaat ttttaggatg gcggttgggg 660
ttttaggcgg ttgcggtagt tttggtaacg tcgtgcggcg ggttagttgg gttgttcggt 720
ttttagttgt tgcggaggta ggttgagggc gtaggggttg tcgagtgttg tgtacggaag 780
aaataaagat atttcggttt aaggcgtagc gggagcgtat aggtgtttcg cggtttagtc 840
gggggataac gtagggcggt tttttgtttt atgttttttt tcgggttaaa gcggattaat 900
taacgtttaa atttcggtat tagttagtcg cgtagaggat gtcgagtatt tttcgggagt 960
aatcggattt ttggtttttt tcggggatgt ttcgcggttt gttatcgcgt taggaggaaa 1020
gaattgtttt aaaaatttgt acgcggagcg aaatagtttg aaagggattg aggtttattt 1080
aggtttttag taaacggagg attgaattgg ggagagttat tttgagttag tttttttttg 1140
gattgtcgga attttagtat tagttttttg ttgaatgtag tatttggtat tttttgaatt 1200
tatttttttt ttttttttta tttagttttt tttttatggt tttaggggga gggggagaga 1260
gtaaggagat cggtattttt gtaataaaat tgtaatttta taaatttttt atttgatgtt 1320
gtaggagtta aatattatat ttttattttt ggatttaaga tagggttgtt tattgattgg 1380
ttagaagagg taaaagaagg tatagtaaag ggaaatattt ttatttttag atttaaggat 1440
ttttcgagaa aggaattaaa tttagaaaat gtagatttta gatttgtatt tttattatta 1500
ttttttttag agttagaagc gaggaattgt tttcgtttgg ggattaggag tatcggatgt 1560
gagaaataaa tttttatttt ttttggattg tattttttag agatagaagt ggaaaatgtc 1620
gttattttgt ttgtaattta aaatatttat tattatggtt tttatttttt ttttgtgaag 1680
tttttttaga ttatttttag aagtaggtat tggagttatt tttgggtatt tgttgggttt 1740
taacgagttg tttttagaga aattttttat ggtttaagga aaaggttcgg aggatgttaa 1800
gttaggttgt tgtggagagt ttagtgtttt ttttatattt agaggtgttt tttttatatt 1860
tgttttaagg ttgttgatat atatattttt ttttaaaggt ttgggttatg ggagaaatag 1920
attttttttt cgtattttaa tttttgcgtt aaaatatggc gtattttaga aatgtagagg 1980
ttgtaaaatt aggttttgtg aagattagag tttttatttt ttcgttacgt taagtagata 2040
taggagtttt tttttaagta attagtttag agatacgtgt aaaattattt ggaaaaataa 2100
ggaatagtta ggggagagat ttatttaagg atgaagattt tttagggata ttattatttg 2160
aatacgtttt tgaaatttat tttttagttg agggaggggt ggtttggagg tttttttttt 2220
tttttagggt taagatttta aaatgagatt ttaatgtgat ttttcgtatt ttattagtta 2280
agatatttat tttggttttt aaagaaaaaa gtattgattt tattttttag ttttttagat 2340
attaaaaaga aatttttatt aaaatatttt ataagttaaa gtaatagatt tggggaagat 2400
tttagtgttg agtggttaaa gaaaagggtt tttttttaga tggtattgga attaaggcgg 2460
ttgaggtagt agaaagtttt tatttacgta gtttttgata aagttttaga gatatggggg 2520
tagcgttgtt ttttttttgt atttttcgtt tacgtttttt taagtttgta atattttttg 2580
gtaaaagtag atgttaaaag ttgttatttt agggttcgta aagtattttt tttaaaagga 2640
gttttataat tttttttatt gttaaaatat attttttaaa agattagttt tttttattta 2700
tttgtatttt ttgttttttt ttttgttcgg ttatatttta ttttggaagg agttggaagt 2760
tgaaatgtgt ttttaagggt tagaagttgt taaggttttt ggtgagtaag attgatcgcg 2820
tttagatttt ttttggagtt ttgtttggaa taaaagtaag aaaattaaaa aattttatat 2880
tttttaaaat agtattttta tttgtaaggt aatgttttgg gttggttaat gatataggtt 2940
atttatgttt aatttttatt ttaaattttt tttgacgttg gttttagaga tggggttttg 3000
tttttgtttt ttggttggtt ttttttgttt tttacgtaga tttttttttt tttttaattt 3060
tttttttttt gattattaga atgatttatt tttttgttat tgatgtttgt cggtttgggg 3120
gagtttgatt agttagttta ttttaaagga agtaaaagcg aattattatt tttagggttt 3180
gggggggaaa gaggtatttt tagatttatt ttttagtttt tttatttttt tttggtttgg 3240
tggatttttt aaaaattatt ttttggtatt taaaggtgtt attttttgaa ttttttagta 3300
ttgtttaaga tgcgtttagt tttaagtttt cgaattggaa aaaagataat gaaggagaac 3360
gaaaattttt ataatttttt tttgggtcgt tttttttttt tcgtagtgtt gttttttata 3420
atagaattcg gaagtaaata ttttcggtgt ttaaggatta ggaaggtgtg ggaggtagtt 3480
taggttgtta gggagtattc gttgtattta aattaattag tttttaatta gagcgataag 3540
gataagaaga agggtagaaa aaaaagagag agggagggaa agagaggagt aagtattttt 3600
tttttttttg ttggtaattt ttaatagatt ttttggaaat cggaaatatt tatcgtattc 3660

gagttttacg ggtatgaaat ttagaatgtt aggagtcgta cgcgtttgtt cggggcggta 3720
tttttttggt tggtcgtcgt tttggttacg gggatttgga tatatggatt tggggttgtt 3780
gtttcgtttg gtatatgtat ttttttttag ttatttttga gaagttgatg gcgtaggaag 3840
gtcgggaagt ttcgagagtc gtttttcgtt tttcgttttt cgtcggagtt agatgtaaag 3900
ttgtcgtgga atagtttgtt aataatgggt tcggggcgta gattttgggt tggatattgg 3960
gaggggggcg agaggttgag gggagaaggg gaggcggata gaagagagag ggagggagaa 4020
agggggagaa gaggaaaaag agggaaaggg atagatagga aggaaaatag atcgagagag 4080
attagttttg agatttagga attgttttta ggaaaagtga aggaggaaaa gggaaagaaa 4140
aggaagattt ttttttaatt aaaatttttt tttttttttt ttttttttgt tttttttttt 4200
tttatttttt aaattttttt tttttttttt tttttttatt tttttttttt tatttttttt 4260
ttttttttgt tttttttttt tgttttaata gaatttatgt ggttgggacg tagggttttc 4320
gggtgttaaa attttgaaga ttaatggatt attttgttaa tgattgtagg cgttagattg 4380
aggtgtttaa atgatttgtg aggtgcgagg cgttttttcg atagttttaa ataatgcgcg 4440
gagtgtgcgg gggaggtaga gggtagttat cggcgggatc gatagtaggg tttatattcg 4500
cgtatattta tatatatata tatatatttt taggtatata tattagatag atttttgtag 4560
attaggaggt acgtaggtat tttcgttttt acgtatttcg ggatattttt atttatatta 4620
atatatatgt atttttgttt tgaaaaaagt gtaaataaag tttcgttggt ttttaatgag 4680
gcgttttttt tcgatttttt tggattaatt aaatagatag tggttttttt tgattaaagt 4740
ttaaattgtt attgggtaga agtaattatg tgatagttaa ttcggtttaa ttttaatttt 4800
gtttttatga atttaatagt ttaatagtag cgcggttttt atacggttgt aattagtgaa 4860
ttagaaaaaa aatattttag tagcgatatt ttatgataga tttttttttt tttgcgttcg 4920
tttttttttt aggttttgtt tttttaaagt ttttttaaaa gagggaattt tttttttgag 4980
ggggttttaa ggagaaggtt atgaattacg aatttgagcg agagattggt tttattaata 5040
gttagtcgtc gttcgttgag tgtttgatat tttttttttt tgtcgttgat atattttaaa 5100
gtttattaat taagatttcg acgtttttat attcgatatt gatttttttt ttttttgagt 5160
agattatttt tagtttgaat ttcggtagtt attttcgtta cggcgttggc ggtcgtttta 5220
agtcgagttt cgcgggtagt cgcggtagtt cggtgttcgt cggcgttttg tagtcgttcg 5280
agtatttttg gatgaaggag aagaaggcgg ttaagaaaat cgtatttttg tcggtcgtcg 5340
tcgtcgtcgt tatcgtcgta gttatcggtt ttgtttgttt tagttataaa ggttagttta 5400
aagatttggt tttaggtttt tgggattttt tttttttttc gggttgtttc gagagtggtt 5460
gtggggggag gggagagtgg gttgggagag aaggaggaga gggagagatg tttggttgtt 5520
ttgtttttgt ttttgtgggt ttaggagtgg gtttgatgga gaggaaaggg gattttgcgt 5580
tttataatga gatatatata tttttaggaa gtggggggaa attttttcga atttgtgtaa 5640
tgtgggatga tttatttgag ttggaattga tttttttttg tttagttgtt ttagagtttg 5700
gttttttgat agtaatgaag agtgatagat cgtttttgtt tagttaagta gttgacgtat 5760
taattataaa ttgtgttgtg gttaatataa agtttgtttt cggatgaaga ggttgggggg 5820
agttataggt atgaattcga tagagatgga ggggacgggg tttgtttagg ttgggttttt 5880
aggaaaggta gtataatagt tttattgtat ttaggggcgg ttattttgtt gtagttgatt 5940
ttttttgtta tatttatttt ttagtggaat aatttcgttc ggattttttt atttttaatt 6000
gtgcgtgtgt tttttgttgg tttttttttt cgtaga 6036

<210> 134
<211> 6036
<212> DNA
<213> Artificial Sequence

<220>
<223> chemically treated genomic DNA (Homo sapiens)

<400> 134

tttgcggaaa gggaaattaa taagagatat acgtatagtt ggaggtggag gggttcgagc 60
ggggttattt tattggagaa taaatatagt agaaaagatt aattgtaata aaatggtcgt 120
ttttggatgt agtagagtta ttgtgttgtt ttttttggga gtttagtttg ggtagatttc 180
gtttttttta tttttatcga atttatgttt gtggtttttt ttaatttttt tattcgggag 240
taaattttat attagttata atataattta taattaatgc gttagttgtt tagttgagta 300
agagcgattt attatttttt attattgtta aaaagttaaa ttttaggata attagatagg 360
aggaggttag ttttaattta aataaattat tttatattat ataagttcgg gaaagttttt 420
ttttattttt taaaaatata tatgttttat tgtagagcgt aggatttttt ttttttttta 480
ttaaatttat ttttgaattt ataggggtag ggataaggta attaagtatt tttttttttt 540
tttttttttt tttagtttat tttttttttt ttttatagtt attttcgggg tagttcggag 600
aaggaagagg gtttagaga tttgggtta agttttgga ttgattttg tggttgaggt 660
aagtagggtc ggtggttgcg gcggtggcgg cggcggcggc ggtcggtaga agtgcggttt 720
ttttggtcgt tttttttttt tttatttagg ggtattcggg cggttgtagg gcgtcggcgg 780
gtatcgggtt gtcgcggttg ttcgcggggt tcggtttggg gcggtcgtta gcgtcgtggc 840
gagggtgatt gtcggggttt aggttgggaa tggtttgttt aaaaggagga ggaattagtg 900

tcgagtgtga aagcgtcgag gttttgattg atgaattttg aaatgtatta gcgatagggg 960
gaaaagatgt taggtattta gcgagcgacg gttggttatt gataaaatta atttttcgtt 1020
taaattcgta atttatggtt ttttttttgg agttttttta gagaaaaagt tttttttttt 1080
ggaggggttt tgggggggta aggtttagga aaaaggcgag cgtagaggaa aaaaaattta 1140
ttatagaata tcgttgttag ggtgtttttt ttttaattta ttgattatag tcgtatgggg 1200
atcgcgttat tattaaatta ttgaatttat ggagataagg ttgaaattgg atcgaattgg 1260
ttgttatatg attgtttttg tttaatgata atttgggttt taattaaaag aagttattgt 1320
ttgtttgatt gatttaaaaa agtcgggaag gaacgtttta ttgggggtta gcgaggtttt 1380
atttatattt tttttagagt aaaaatatat atatgttggt gtgggtgggg atgtttcgga 1440
gtacgtgggg gcgagggtgt ttgcgtgttt tttgatttgt aaggatttat ttagtgtgtg 1500
tgtttgggag tgtgtgtgtg tgtgtgtgaa tgtgcgcgag tgtaagtttt gttgtcggtt 1560
tcgtcggtgg ttgttttttg tttttttcgt atatttcgcg tattgtttgg gattgtcggg 1620
aagacgtttc gtattttata aattatttaa gtattttagt ttgacgtttg tagttattaa 1680
taaagtaatt tattaatttt taaagttttg atattcgagg gttttgcgtt ttagttatat 1740
aagttttgtt aaagtaggag aaaggagtag aggaagagag gagatgagag agggagaata 1800
aagagagagg gaagaagaga gagtttgaga gatggagaaa gagaagatag aaaagagaga 1860
agaaaggaaa gattttggtt gggaaggggt tttttttttt tttttttttt ttttttttat 1920
tttttttaaa agtagttttt ggattttaaa attgattttt ttcggtttgt tttttttttt 1980
gtttgttttt tttttttttt tttttttttt tttttttttt tttttttttt tttttttgtt 2040
cgtttttttt ttttttttta gtttttcgtt ttttttttag tgtttagttt agagtttgcg 2100
tttcgggttt attgttagta ggttatttta cggtagtttt gtatttggtt tcggcgggaa 2160
gcggaaaacg ggaggcggtt ttcgaagttt ttcgattttt ttgcgttatt aattttttag 2220
gagtggttgg agaaagatgt atgtgttaag cgagataata attttaggtt tatgtgttta 2280
aattttcgta gttagggcgg cggttagtta aagaaatgtc gtttcgagta ggcgcgtgcg 2340
gtttttggta ttttgggttt tatattcgta gggttcgggt gcggtgagta ttttcgattt 2400
ttaggaagtt tgttggaagt tattagtagg gaaagagaag atgtttgttt tttttttttt 2460
tttttttttt tttttttttt tatttttttt tttgtttttg tcgttttggt tgggggttgg 2520
ttggtttaga tatagcgagt gttttttggt agtttgaatt gttttttata tttttttgat 2580
ttttgggtat cggggatgtt tgttttcggg ttttgttgtg ggaaatagta ttgcggggga 2640
gaggaggcga tttaaggagg aattataaag gttttcgttt ttttttatta tttttttttt 2700
aattcggaga tttgagattg agcgtatttt ggatagtgtt agagggttta aaagataata 2760
tttttaggta ttaaaaaata atttttaaaa aatttattaa gttagaaaga ggtgaggagg 2820
ttgagaggta gatttggagg tatttttttt ttttttaaat tttgaggatg gtgattcgtt 2880
tttgtttttt ttgaaataga ttaattgatt agattttttt aggtcggtag gtattaatgg 2940
taggaaaata aattattttg atggttaggg agagagaggt tggaggggga gaaaaattta 3000
cgtagaaaat aagaaaggtt aattaaggag taaaggtagg attttatttt tgaggttagc 3060
gttaaagagg gtttggggtg gaaattagat ataagtggtt tatattattg attagtttag 3120
agtattgttt tgtagataga gatgttattt tggaaaatgt gaggtttttt agtttttttg 3180
tttttatttt aaataaagtt ttaaaggaag tttgggcgcg attaattttg tttattaaaa 3240
gttttgatag tttttagttt ttaagaatat attttagttt ttagtttttt ttaagataaa 3300
atgtggtcga gtaaagagga gagtaggaaa tgtaagtaaa taaggaaaat tagtttttta 3360
aaaaatatat tttggtagtg aaaagggtta tagggttttt tttaggaagg gtgttttgcg 3420
aattttggga tggtagtttt tggtatttgt ttttattaga aaatattata ggtttaaggg 3480
aacgtgggcg gggggtgtag agagggagta gcgttgtttt tatatttttg aggttttgtt 3540
aggggttgcg tgggtgagag ttttttgttg ttttagtcgt tttaatttaa atattatttg 3600
gaagaaaatt tttttttttg attatttaat attgaaattt tttttaaatt tattatttta 3660
atttatgaga tattttaata aggatttttt tttaatattt aggaaattgg ggggtgaagt 3720
taatattttt ttttttgaaa gttaaaatga atgttttgat tggtaagatg cgggaaatta 3780
tattagaatt ttattttaaa attttagttt tgggggaagg aggaagtttt taaattattt 3840
tttttttagt taaagagtag attttagaaa cgtgtttaaa tagtgatatt tttgaaggat 3900
ttttattttt aggtgaattt tttttttagt tgttttttat ttttttaggt aattttatac 3960
gtgtttttaa gttgattatt taaaagaaga tttttgtatt tgtttagcgt gacgaagagg 4020
tggaagtttt agtttttata aagtttggtt ttgtagtttt tgtatttttg aggtgcgtta 4080
tattttggcg tagaggttga ggtgcggaga ggaaatttat tttttttata gtttaaattt 4140
tttaaaaagg atatatatat tagtagtttt aggatagata tggggaaagt atttttgggt 4200
gtgagagaaa tattgagttt tttatagtag tttggtttgg tatttttcga gttttttttt 4260
taaattatga ggagtttttt taaaagtagt tcgttgggat ttagtaggta tttagaaatg 4320
gttttagtgt ttgtttttaa ggataatttg agggagtttt atagggggaa gataaaggtt 4380
atgatgatgg atattttgga ttgtaaataa aatggcgata ttttttattt ttgtttttga 4440
aagatataat ttagggagaa tagggattta ttttttatat tcgatgtttt tgatttttag 4500
acgaaagtag tttttcgttt ttagttttga agggggtgat gatggggatg taggtttgag 4560
gtttgtattt tttgaatttg attttttttt cgaggaattt ttaaatttga agatgaaaat 4620
attttttttt attgtgtttt tttttgtttt ttttagttag ttagtaagta gttttatttt 4680
aaatttagga gtggaaatgt aatatttagt ttttataata ttaaatgaaa aatttataaa 4740
attgtagttt tattataaag atatcgattt ttttgttttt tttttttttt tttggggtta 4800
taaaaagaaa gttgggtggg ggaaggagag gaaataaatt tagagaatgt taaatattat 4860

atttagtagg aagttaatgt tgggatttcg gtagtttagg gaagggttgg tttagggtga 4920
ttttttttag tttagttttt cgtttgttgg agatttgggt gagttttagt tttttttaaa 4980
ttgtttcgtt tcgcgtgtag atttttggag taattttttt tttttgacgc gataatagat 5040
cgcgaagtat tttcggagga gattaggagt tcgattgttt tcggaaagtg ttcggtattt 5100
tttgcgcggt tggttaatat cgaggtttag gcgttagttg gttcgttttg attcgaggaa 5160
gagtatggag tagaagatcg ttttgcgtta tttttcggtt gggtcgcggg gtatttgtgc 5220
gttttcgttg cgttttgggt cgggatgttt ttgttttttt cgtgtatagt attcggtagt 5280
ttttgcgttt ttagtttgtt ttcgtagtag ttgggaatcg ggtagtttag ttggttcgtc 5340
gtacggcgtt attagagttg tcgtagtcgt ttggagtttt agtcgttatt ttgagaattc 5400
gtcgagtttt taggcgaata gtttggttag gttttatgtt ggagttagag ggttgttgcg 5460
ttcggtttgt tttgtcggtt cgtttcggtt tgaattttac gtagatttac gttttcggtt 5520
aaagaatttt tttttaatac ggatattatt tagaataaaa attttatttt tttttgtttt 5580
ttagaatgtg agtagtaagg aatgaagaat ttttaaaata aatttagtat ttttcgcgtt 5640
tatagatttt tttttttagt tttgtcggat gttataaatt taaattattg tttttaaaag 5700
ttggattttt tttggtttta ttgtatcgtt atcgtttaaa ggaattatat ttttttttta 5760
aataaaaaaa attttaagta cgttaatata taattgaata aaaggtttaa gaattttttt 5820
gaattattag ggtatagaat tttttaatat aaatacgaac ggatggggat aaataatatt 5880
taaaatttag attatttaat gtttgtaaaa aaatataaat aaatttgatt gtttattagt 5940
atatatatac ggaaagacgt atatttagtt atttttgtat agagagtttt tgttttaaag 6000
cgtttttgat tttttttttt attttttata agaagt 6036

<210> 135
<211> 6163
<212> DNA
<213> Artificial Sequence

<220>
<223> chemically treated genomic DNA (Homo sapiens)

<400> 135

tgtttttaaa ggtaatagaa tggtgtagga aatttttttt atttgaaaag agggattttg 60
attggtcggt tttttgtttt tttagtatat atgatgattt ttaaagtgtg taagaataaa 120
tgtaaatata gatatttttt gtaaaatata atttttgtag tttttaaata tagaatatgt 180
tttttttgaa attatagttc gttgaatgta aagcgttttg agatttgaga aggggaagga 240
aggaagtgga aatttttttg aaagatatag aatttttttt ttattttttt tattattatt 300
gttagtgata aaatgtttat tggtgttatg aaaatgtttt tgtttggaaa tttttttaat 360
gtttattatt aaattataat tgaatgtaag agaaatgtta taagttataa gtgaataatt 420
agattgtagg gaatgtagaa aggtgaaatt tataaataat aggttatttt gtaagattat 480
taggatagaa tagaatattt tttatttttt agaatgtatt ttttaatgat atacgtggaa 540
ttaaaaaaaa aagttaagga gaagataaag taatttaaga agaagggtgg gagagtagaa 600
aagaaagaga atagtgtatt aaggaagtcg attagaatta tgttttatgt gtgagttgta 660
attgaagagt taaataatat tttatttttt atgtatttag aatggtttta agtgttggtg 720
ttgtaagaat ttatttttta gaggatatat tttttttttt tatgaattga tttttaaggg 780
attagtatag gaaaatagag aagagaaaag tgaaatattt tttttaggtt tttttttttt 840
aattttggaa ataatttatt tatttagaat gtggattttt gtaggaatgt ttaagattat 900
tttaatattt tggttgattg gatttaatgg tgttaggtat gtttagtggt aaagtaaata 960
ttttttaaat attgttttta tggaagtaat tagggtgttt ttgtgtaatg tgtaaagttt 1020
ttattttggt taagagtttg aagttaaatg ggggtttgtt agatgaaata gaagaaagga 1080
aataattgtg gtgattgttg ttgtagtagt gaatatttta ttatatttgg gttagtgaaa 1140
tgaggaaaat tgaaattttt cgatgagtta ataagataat tacgtgtttt tttgggaaaa 1200
atttttttat tgtgtttttt ggttaaaaat atgtgatata ttttatagat atgttagttt 1260
taaaaattat agatattttt tagttagttt ttaaagtaaa agaaaagagt atttgtagaa 1320
tttattgttt ttagtgtgtt tgttttattt ggaggatttt gaattttagg ttttttaaat 1380
aattttagtt aaaatttatg tgtaatttta atatgaatat tagtttttat ttttgcgttg 1440
tgtgtattat tttaaattat tataagaaaa ttatttttaa agaattaatt ggttaaatgg 1500
ttggtaaagt gttgtttttt ttaaattttg tgtttagtgt aggtggtggg gaagttttta 1560
aatgttatta ttaggcgttt tgaaattttg ttattttatt gtttagtttt agagatatgt 1620
attagataga gaatagtttg ggggattttc gttttaaaga ataaaggata aatatagtta 1680
atatatttta taacgtttat attttttttg gttttttttt ttggtttttt ttgagaagga 1740
gtgatttggg taaagtattt tgttttttaa aagatattgt atagattttt tagaagcgta 1800
aagtttaagg tcgaggtgaa ttttaggtta gtgcgtttaa taaatatgaa aatgtcggtt 1860
agtgaagggc gcgttttgtg atttaataaa gattgttaat gtgtaagatt aataagaaat 1920
aaaatgtata cggtgttatt gttcggttat tttgaaattt gggatagggt tgtatttaag 1980
agggaatgtt gttataaata tatttaaaat gatttaaatt agatttaaat taagttgtaa 2040
aatattgttt tttttgtatt tttttttttt tttcgatttt ttttattttt ttttatttgg 2100

gattcgagga tatgatgaaa agttatttta tttaatggga ttttaagaaa gtttttgttg 2160
ggaatatatg ttatttttgt acgtggaaat gagtaaacgt gtttattagt tttttaagga 2220
aaggataacg tttttatttc gtttgggatt ggagttagtt ggtgtgtgag gattagtatg 2280
tttttttttt gttatatttt agggtatttt attttttttt aggcgagatg ttgttggtaa 2340
attttagttt gtttattaat tgagttagtt tttttttggt tttattttgg tgttttggat 2400
attgtgtaaa ggtaagaggt atttttttta aatgtttttt aaaagattat ttgatttaat 2460
ggaatgatgt tatggtttgt aaagggagat tgaggaaggt ttagaaaagg tattgagatt 2520
gtttattata gttataaatt ttgtagtaaa atgttaaaat gtcggtgttg agataatatt 2580
tggtggtttt ggtttcgttt gtgtttatga tacgaattat aaagataagt tataggtttt 2640
gggggatttt gtgtaaggtt atttttttaa agtaatagaa ttatataaaa tataggtata 2700
gttaattcgt ttgggttaat cgtatataaa atagtgttta tgtttcgtta atatttaaaa 2760
atagaaattt ggtataaggt tttgaggtta aataatagaa ttttgtgttt tagtgtttgt 2820
aaatattaaa tagatatggt taagatttta gtatttaagg ttgatgatat gagaaggagg 2880
gaagattttt ttgttatgtt ttttgttttt ttgaaattta tagggtttag atttgtaata 2940
cgtatgttta tatattattt atattatatt ttatataatt tatgtgtata ttattttttt 3000
ttgttaggga gagatattgg tttaggtaga tgtattatat tttaaatttt ttttttagtt 3060
tagagaggat ggttaaagat tatatttagt gttgattttt gtttgtggtt taaatttttt 3120
taaaaataaa tttttagggt gttttatgtg aatggatagt attttttata tttatatata 3180
tttaacgtgg aaaatttttt tgtatttttt tagggtgatt ttagttagaa agttaatatg 3240
attttttttt taattttttt ttttaaatta gtttaaagat tagttttata taataggttt 3300
tatgtggtta agtattaagt gattatttat taaatggata tattttagta tttagtaatt 3360
tatatgagta attagtatga atgaagatag aattgatatt tttttttaag tgtttttagg 3420
tttaggaaat aatttttttt gtttgcgttt tagatttagt tgtgagaagg ttgtttatat 3480
tgtttttttga aaaattaatt aaggtgtatt agttgaaaaa ttttttgtttt tgtatgttaa 3540
tatgaaaata tttttagaat ggtgattatg ttagtttata ttgttttaat ttaggttgaa 3600
aagtagagga tttagatatg tttattattt agaaaagaaa gggagaaagt atatagtata 3660
aataaggtat tagtatttat tttaaagatt aatattaggt ggaattaata agtaatttat 3720
ttgttaataa tggcgtaatt agtgtattta ttaaatttta attattaaat agtaggtaat 3780
agtttttttt tttttttttt cggagttttt gatatcgtta atacgttaat ttttagtaat 3840
ttagaaatta tagattgttt tgtaaatata attttagttt tagatattgt ttgagaaagt 3900
aggatttaaa agagaaaata tgaaattttt agttggaaaa gatttattag cgattagatt 3960
cggtaaggtg tttattttat ttttagtttg tttttgtttg gggatagttt tgaaatagga 4020
agttattgtt tttaaagttc ggttttgtgg aggtgttgtt tttatttatt tttatttttg 4080
ggttattttt ttttttataa tcgtatttag taaatgcggg tatatttagt ttttatatcg 4140
ataattttcg ttaaattaga tgttattacg aaggttttaa ttttttttag gaaaatacga 4200
aatagattgt ttttgaaaga aggaaaaaat gaaaagtgtt tatttagtta taatggaatg 4260
gtttcgattt atatttttgg gtcgattttt tttagttgtt cgtttatttt ttgtagtttt 4320
tgttaagttt tttaaagtaa gggtttttag gttgatacgt aaggagtagg gtgtggggaa 4380
gggggatttg cggttgagaa aatgtgtttg gtggtttttt agtggcgtat ataaaaatta 4440
ggatgttttt cggttttaac gttttttttt tcgtttgtta gtttagaatt tttgaaggat 4500
acgaagtttg attgggttgc gttcgaggtt agcgttgagt cgcggtcgtt agatagttcg 4560
aggtttaggt tagtagtagg gtaggtagag ggttttcgag aggatttata gtttgatagg 4620
gtttgaattt ttagcgggaa ttaaagttgg ggggttgggg tttaaagttt ttgttttttt 4680
ttttagttta ggggtcggat agtagtattt attacgtatt cgcgcgtttt ttgattcggg 4740
ttcgcgcgcg cggtgcgttt attggtgggg gcgttgtacg tggggtacgg ggcggggcgc 4800
gcgttgggtt ggggaggagc ggttcgaatt ttggtggcgg cgtttgcggg agggggagga 4860
gtgggtattt gatagcgggg ggaggggaag ggcgtagagg aaagggggat gggaagcgag 4920
gtaggagggg gaggggtttc ggtaagttta gaaaaaacga taacgcgaga aaaattagta 4980
tttttgtatt ttataaatta atgattatga gttcgttttt gataaatttt aattatatcg 5040
agtttaagtt tttttttttc gaggagtacg cgtagtatag cggttcgggc ggcgtagacg 5100
gcggttcggg cgggggtttc ggttattagt agtttttagc gttttcgatt tagtatttgt 5160
cgttgtagta gttttagttt ttttacgcgg gcggcggtcg agagtttatt gttttttatt 5220
acgcgtcgcg gatcgttcgc gagttcgttt attttgttgt cgcgttgtat ttcgcgtatg 5280
gggtcgcgga tatcgtttat ttttatggtt atcgcggcgg cgttagtttc gggcggtcgt 5340
tttagttcga gtagttttcg gcgtaagtta agggtttagc gtacggtttg tatgcgagtt 5400
acgttttgta gttttagttg tcgtcgtttt tgtagtttcg cgtcgtgttt ttagcggttt 5460
cgcggcgttg cgaggcggtt ttcgttattt taggcgtttc ggtagggggt agcgttttcg 5520
cgtgttcgtt gtttttggtc gataagagtt cgttgggttt gaagggtaag gagttcgtgg 5580
tgtatttttg gatgaagaag atttatgtta gcgtcggtac gtggcgtcgt ttgggaggcg 5640
cgggaggtgg gtcggggttg ggtttgggtt cgagggtcgg ggttaggagg gagaggttta 5700
ggggaagggg gtttcgggag gggagttttt atttgattcg ggttgcggtg agatgtgggt 5760
atgtgcgcgt tagttagtcg tttgatttta atatgagaat ttttttgtat ttagggtttt 5820
tttattagga gatttacgag acgttaaatt gttagggtag taattatagt ttttataaat 5880
ttaattgttt tggtagaggt tttaggttat gtgtttttga agtttttttt tagttttaat 5940
gtttagtatt attttttatg gttttcgggt gtttttcgtt gttaatagtt tgtgaaatat 6000
tttttttgtc gttttatgta ttttgcgtga atttggtgtt aggttattat ataatgatga 6060

tgtttaattg ttttttttta ttttattttt tttttttttt tttttttttt ttttttcgtt 6120
tttttttttt tggtttttta tttcggggtt ttgggttttt agt 6163

<210> 136
<211> 6163
<212> DNA
<213> Artificial Sequence

<220>
<223> chemically treated genomic DNA (Homo sapiens)

<400> 136

attgaaaatt tagaatttcg aaatagaagg ttaaggagga gggagcggaa gagagggaaa 60
ggaggaggag agagaaggtg gggtggggaa gagtaattgg atattattat tatataataa 120
tttgatatta agtttacgta agatatataa aacggtaaag aaaatgtttt ataggttatt 180
gataacggga aatattcgag agttataaag aatggtgttg ggtattgggg ttgaagaaaa 240
gttttaaaga tatatggttt gaagtttttg ttagggtaat taaatttatg ggggttataa 300
ttattgtttt aatagtttgg cgtttcgtaa attttttgat aaagggattt tgggtataaa 360
agggttttta tgttgggatt aggcggttgg ttggcgcgta tatatttata ttttatcgta 420
gttcgggtta gatgggggtt tttttttcga ggtttttttt ttttgagttt tttttttttg 480
atttcgattt tcgaatttag gtttagtttc ggtttatttt tcgcgttttt taagcggcgt 540
tacgtatcgg cgttgatatg gatttttttt atttaggggt atattacggg ttttttgttt 600
tttaggttta gcgggttttt gtcggttaag agtagcgggt acgcgggggc gttgtttttt 660
gtcgggacgt ttggggtggc gggggtcgtt tcgtagcgtc gcggggtcgt tgggggtacg 720
gcgcgaggtt gtaggggcgg cggtagttgg ggttgtagga cgtggttcgt atgtaggtcg 780
tgcgttgggt ttttggtttg cgtcgggggt tgttcgggtt ggggcggtcg ttcggggttg 840
gcgtcgtcgc ggtagttata ggggtaggcg gtgttcgcgg ttttatgcgc ggggtatagc 900
gcggtagtag ggtaggcggg ttcgcgggcg gttcgcggcg cgtagtagga ggtagtgggt 960
tttcggtcgt cgttcgcgtg agggagttgg ggttgttgta gcggtaggtg ttgggtcggg 1020
ggcgttgggg gttgttggta gtcggggttt tcgttcgggt cgtcgtttgc gtcgttcgag 1080
tcgttgtgtt gcgcgtattt ttcgaaggga gggaatttgg gttcgatgta gttggagttt 1140
attaaaaacg agtttatggt tattaatttg tgaagtgtaa aaatattaat ttttttcgcg 1200
ttgtcgtttt ttttgggttt gtcgaggttt tttttttttt tgtttcgttt tttatttttt 1260
ttttttttgc gttttttttt ttttttcgtt gttaagtgtt tatttttttt tttttcgtag 1320
acgtcgttat taaagttcga gtcgtttttt tttagtttag cgcgcgtttc gtttcgtgtt 1380
ttacgtgtag cgtttttatt aatgggcgta tcgcgcgcgc ggattcggat taggaaacgc 1440
gcgggtgcgt gatggatgtt gttgttcggt ttttgggttg ggggagggag taggagtttt 1500
ggattttagt tttttagttt tggttttcgt tgggaattta ggttttgtta ggttgtaggt 1560
tttttcggga gttttttgtt tgttttattg ttggtttagg tttcgggttg tttggcggtc 1620
gcgatttagc gttgatttcg ggcgtaattt agttaggttt cgtgtttttt aggggtttta 1680
ggttaatagg cgaaaggaag ggcgttggga tcgaggggta ttttggtttt tatgtacgtt 1740
attgagaggt tattagatat atttttttaa tcgtagattt ttttttttta tattttgttt 1800
tttgcgtgtt agtttgagag tttttgtttt gagaagtttg gtagaagttg taaagggtgg 1860
gcgggtagtt aagagaaatc gatttaagga tgtaaatcga ggttatttta ttataattgg 1920
atggatattt tttatttttt ttttttttta gagataattt gtttcgtgtt tttttaagaa 1980
aaattggaat tttcgtaata gtatttaatt tgacgggggt tgtcgatgtg agagttaaat 2040
atgttcgtat ttattaggtg cgattgtgag agagaaggtg gtttaaggat gggaatggat 2100
agaagtaata tttttataga atcgagtttt gaaaataata attttttatt ttagaattat 2160
ttttaaataa aaataagtta agggtagaat aaatattttg tcgggtttga tcgttgatgg 2220
gtttttttta gttaagaatt ttatgttttt ttttttagat tttgtttttt taggtagtat 2280
ttgaggttag agttatattt gtaggatagt ttataatttt tgaattgttg aaaattagcg 2340
tattaacgat attagaagtt tcggaaagga gggagaggag attgttgttt gttatttggt 2400
aattgaaatt tgatgggtat attaattacg ttattattaa taaataaatt atttattaat 2460
tttatttaat gttgattttt gaagtaaata ttgatgtttt atttgtgttg tgtgtttttt 2520
tttttttttt tttgagtagt agatatattt agatttttta ttttttagtt taaattaaag 2580
tagtgtaaat tagtatagtt attattttaa aaatattttt atattggtat gtaaaagtaa 2640
ggatttttta gttggtgtat tttagttgat tttttaaaga gtagtataaa tagttttttt 2700
ataattgagt ttggaacgta gataaggaaa attatttttt aagtttggag atatttgaaa 2760
aggaatgtta attttatttt tatttatatt ggttatttat atgagttatt aaatgttgga 2820
atatatttat ttgatggata gttatttaat gtttagttat ataaagttta ttatatggga 2880
ttaattttta aattaattta ggaaaagagg ttaaaaaggg gattatatta gttttttaat 2940
tggaattatt ttgaagaggt ataaagagat tttttacgtt aggtgtatat gagtgtgaag 3000
agtgttgttt atttatatga ggtattttga aaatttgttt ttaaagaaat ttgagttata 3060
gatagaaatt aatattgagt gtaattttta gttatttttt ttagattgga ggaaaaattt 3120
agaatgtgat atatttattt gaattaatat ttttttttag taagaaaaaa taatatatat 3180

ataggttata taaaatgtaa tataaataat atatagatat acgtattata aatttgaatt 3240
ttataagttt tagggggata aaaagtatga taaggaaatt tttttttttt tttatgttat 3300
tagttttgag tattagggtt ttaattatat ttgtttaata tttatagata ttaaaatata 3360
aaattttgtt gtttagtttt agaattttgt attaagtttt tatttttaag tattaacgag 3420
atataaatat tgttttgtat acggttaatt taaacgagtt agttgtgttt gtgttttgtg 3480
tgattttatt attttaggaa gatggtttta tatagaattt tttaaggttt gtaatttgtt 3540
tttgtggttc gtattataaa tataaacgga gttaggatta ttaagtgtta ttttaatatc 3600
gatattttga tattttattg taagatttat ggttgtaata aataatttta gtattttttt 3660
tgaatttttt ttaatttttt tttgtaaatt atagtattat tttattgaat taaataattt 3720
tttgaaaaat atttaaaaaa aatatttttt gtttttatat aatatttaag atattaaagt 3780
aaagttagga agaaattaat ttaattaata aataaattga agtttattag tagtatttcg 3840
tttgagaaaa gatgggatgt tttgaaatgt agtagagagg gagtatgtta atttttatat 3900
attaattggt tttagtttta agcggggtga aagcgttatt ttttttttag gaaattggtg 3960
agtacgtttg tttattttta cgtgtaggga taatatatat ttttaataaa agttttttta 4020
aaattttatt aggtgaaata attttttatt atgttttcga attttagatg gagaagagtg 4080
aagggagtcg gagggagagg gagggtgtaa gggaggtaat gttttgtagt ttggtttgaa 4140
tttgatttga attattttga atatatttgt aatagtattt ttttttgaat gtaattttgt 4200
tttaagtttt aaagtgatcg aatagtgata tcgtgtgtat tttgtttttt attaatttta 4260
tatattgata gtttttgtta aattataagg cgcgtttttt attagtcgat atttttatat 4320
ttgttagacg tattgatttg aagtttattt cggttttgga ttttgcgttt ttaaaaggtt 4380
tatatagtgt tttttagaga gtagggtgtt ttgtttaggt tatttttttt taggaaaaat 4440
taaggggaaa agttaaagga aatgtaaacg ttatggaatg tattgattgt atttgttttt 4500
tgttttttag agcgagagtt ttttagattg ttttttattt gatgtatgtt tttagagttg 4560
aatagtggaa tggtagaatt ttaaaacgtt tgatggtggt atttgaaggt ttttttatta 4620
tttatattag atataagatt tgagaggaat aatattttat tagttatttg attaattaat 4680
tttttgggga taattttttt gtagtagttt aaaataatgt atataacgta gggatgagga 4740
ttgatattta tattgggatt atatatgaat tttaattggg attgtttgag aggtttgagg 4800
tttaaaattt tttagataaa gtaagtatat taaaagtaat aaattttgta agtatttttt 4860
ttttttattt tgaagattag ttaagaggta tttatggttt ttgaagttga tatgtttata 4920
aggtgtgtta tatgttttta attaaaaagt ataataaaaa ggtttttttt aaagagatac 4980
gtaattgttt tgttgattta tcgaggggtt ttagtttttt ttattttatt agtttaaatg 5040
tggtgaaatg tttattgttg taatagtaat tattatagtt gttttttttt ttttgtttta 5100
tttggtaaat ttttatttgg ttttaagttt ttggttagag tgaaaatttt atatattgta 5160
tagaagtatt ttgattattt ttatgaaggt agtgtttgga aaatatttat tttattattg 5220
aatatatttg gtattattaa atttaattaa ttaaaatatt gggatgattt taaatatttt 5280
tgtaagagtt tatattttga gtagatgaat tatttttaaa gttaaaaaag aaaaatttag 5340
ggaaaatatt ttattttttt tttttttgtt tttttatatt gattttttga aggttaattt 5400
atagaaaagg gaaatatgtt ttttggaaaa tagattttta tagtattaat atttaaagtt 5460
attttagatg tatgaaaaat aaaatattgt ttagtttttt agttgtaatt tatatatgag 5520
gtatggtttt agtcggtttt tttaatatat tatttttttt tttttttgtt tttttatttt 5580
tttttttagg ttgttttatt ttttttttgg tttttttttt taattttacg tgtattatta 5640
aaaagtatat tttgaagaat agaaaatatt ttattttgtt ttggtggttt tatagagtag 5700
tttgttattt gtggatttta tttttttgta ttttttatag tttagttatt tatttatagt 5760
ttgtagtatt ttttttatat ttaattgtgg tttaataata aatattaaaa aaatttttaa 5820
ataggaatat ttttatggta ttagtaagta ttttgttatt ggtagtggtg gtggaagggg 5880
tgaagggaga atttgtgttt ttttaggagg gtttttattt tttttttttt tttttttaga 5940
ttttagaacg ttttgtattt agcggattgt agttttagaa aaagtatatt ttgtgtttga 6000
aaattgtaaa gattatattt tgtaagaagt gtttgtgttt gtatttattt ttatatattt 6060
taggggttat tatgtgtatt aaaaagataa aaaatcggtt aattagaatt ttttttttta 6120
aataaaggag gttttttgta ttattttgtt gtttttgaag gta 6163

<210> 137
<211> 6523
<212> DNA
<213> Artificial Sequence

<220>
<223> chemically treated genomic DNA (Homo sapiens)

<400> 137

gttggaagga gtgttttgat attaggtagg cgcgggttat tgagtttttt gggatttaaa 60
taggatgtgt atgtgggagg agggaatggg tgggaagaat ttacgtttgg ttgagttttg 120
gttagttcga ggtgattttt tagttttggg ttgtaaggtt taggtttttg gtgttagcgg 180
agaaggagag aatgagggag tttatcgatt tttaaggcgt acgagtgaag agtatagagt 240
gaaggtttgg ttttttatgg tttttgtttt agtttaagaa ttcgtattta cgaatagggg 300

agcgttaggg gaggggtaat tttgggtcgt gtttggagtg taggtcggta gggagtaaag 360
ttattttgt tattgtttt tttggagtag gattttagg tttttgttt ggaggaggag 420
gggcggggaa ttcgtaaaat ttaataaagt aaaaagggga ggaggaagga ggggatttgg 480
cggggttttt gaggcggttt aagtattagt gtttttattt tggtttgatt tgttcgttat 540
tgaaagggtc gtgttcgtcg ttggcgttcg ggaagagagt cgggtcggtt agttgtgcgc 600
gtttattaat ttgtcgggcg ggcggcgggc gggcgggttg ggggttgttt gtaattttgt 660
tgtttcggtc gtcgcggttt tcggggagcg ggtttcggcg ttcgttttta ttggtcgtcg 720
tcgcgttagt tggtgcgatt tgttgttgtc gtttttggcg ttcggttatt tagaaataaa 780
ttagttcgat gattattaat agttatagtt agatgtatta atatataata aattatagtt 840
ttgtagaggg gcgcgtaaat gagtttttat tttgtgaatt ttattttttt cgggagtttt 900
tttagcggtt aggatttttt tttgggttag ttgttttttt attaggttgg ttatgacgcg 960
ttgaggtttt tttcggtttc gtacggggcg tcgagttttt cggataagac gtatatttta 1020
ttttgttttt attaatagtt taattcggtt ttggtttgta atcgggcgtt ttacgagtac 1080
ggggtttcgt gtttttattt tgataaggat tttagtggcg tttcgttttc gggtagtggt 1140
aagtagaggg gtttcgggga ttatttgtat ttttttttcg agtagtagta taaattcgat 1200
agtagtagcg ggtagggtaa agtattttat gacgaaggcg tcgatcggaa gtatacgagt 1260
tcggtttatt tttggatgta gcggatgaat ttttgcgcgg gtaagatata ttttaagata 1320
gtggagggag aggggaaagg aggtaggaaa agaaagaaag aaggaggaaa tagagtagaa 1380
agaggaatag agagaaagaa aggatgaaag aattagtaaa gtttttttgg ttttttaaaa 1440
gagaagaaaa aattagtttt taatggggtt attaggttat ggtagaggaa gggtaaggtt 1500
ttagtgttta tgtaagttga tattttaggg gaggatgggg gtattggtac gtatatgaga 1560
tttgtgatag ggatagtttg aagagagaga gaggttggtg gggagggata gtttgtttat 1620
gtattttgag tattttgttt agttttttaa gttagagggt tttttgtatt gcgttattga 1680
taaatatttt ttgtttttta attttttttt ttttttgttt tttagaaatt cgtatagaga 1740
ggtggttatt tggttttttt tagtgttttt ttaaagtttt ggagggttat agttttagat 1800
ttaggatttt tttttttttt tttttttatt tttttcggtt tttatttaat tgtttatttt 1860
tagttaattt ttgttttttt ttgagtttat gtttgagttt tatatatttg ttttggaggg 1920
atttttattt atttttgggg aattttggta ttagattaga tagtttaata taattttata 1980
ttgttagaaa ttttagatta tttatagttt tattttttag tttttggttt ttaaggattt 2040
tatagggaat tttaaagatt tttaggaatt ttttaggata gagtttaatg cggattgtta 2100
aaattataat gtttttttatt aatatttatt ttatttttgg gggagttttg tatttttttag 2160
aagggttata taatttggga gaatgtgtaa agatttaaga gggttgatta tttttaattt 2220
tttaaaaaat gtgttgagtt tcgatatggt tggttatagt agtattttta gagagattat 2280
tatgaattta gagtgtgtat attatttata tggttttata ttatttattt tagatatata 2340
tatatatata tatataaata tatatatata ttttgtattt ttataaaaat acgatttggg 2400
tattttaaat taagaatgtt tattttgtta ttttgtttgt aatggttttt ttatgaggta 2460
gattttttaa aaacgataat gtttatttta agtcgttata ggtggaaaat atttcgtttg 2520
gtatgaaata attttttatt tagtatgttt tgggttttag atatagtggg agaggttgga 2580
gtggaggcgg ggtggttttt agtttgggtg ttttttgtgg gaggtattgg gttgattggg 2640
ttaatttttt aggttttggt ttatcggcgt tcggtcgttt ttgttcgtag gtgttgtgta 2700
tgggagttat gggcgtcgag gtcgttagat ttatacgcgt tattagatat tggagttgga 2760
gaaggagttt tattttaatc gttatttgat acggcgtcgt cgtatcgaga tcgttaacgc 2820
gttttgtttt atcgagcgtt agattaagat ttggttttag aatcgtcgta tgaagtggaa 2880
aaaggaaaat aagtttatta attttacgta gtttagcggg gaggatttag aggtaaaggc 2940
gggcgagtag atgtttgggt agggattagg ttagcgttgt aatttttttc ggttttgttt 3000
ttttgttttc gtttgttttt taattttttt tttcgtttgt ttttatttgg gggttttcgt 3060
agttttaggg gagttcggag ttttgtaagc gtttgtgtat ttatttttta taaaataaaa 3120
taaaataaaa taaaataaaa taaaataaaa taaaataaaa ttttatatat agttaatatt 3180
agcggtggag cggggcgtat tgtatatatt tagtttcgtt ttataaggat gtttaaatcg 3240
gtttcggagt tttggggtgc gttttgaatg cgtttggggt ttttgtgtta gtgtttttga 3300
gtttttcgat ttcgggacgc ggggttgttg gggaggaaag agttttgggt cgagtttcgt 3360
ttcgtgtttg ggcgtttttg tttaagtttt tcgttagtaa gtttcgaagg gtattattaa 3420
attatttttg gttagtggaa agagggggtt taagagttgg agttcggttt tgtcgcggtt 3480
ttttaagtcg ttttttagaa taggaaggaa gttcgaggaa taaagggggt tttaatagag 3540
tttagttttt cggtttcgcg tgtgtaatcg ttagtggaag agaagtagtt ttagtcgagg 3600
cgagttaatt tgggcgcggg tggaatatta tagttcgggg taggttttcg gtttcggatt 3660
ttcgttttta gttttcgata gcgttcgcgt cgtcgttata gtagcgttta ggatttaacg 3720
aggggtttag ggttatggga gtcgttagag ttttggtttt tagacgtgtt aggggcgttt 3780
gtaacgtcgg gtttttagcg gggagatttt atttgttttt tagttatatt tgtgatttat 3840
ttgagttttt cggagtcgtg gaaaataatt atagagatat taggtattgt taaggaatgt 3900
ttttattatc gtttttattt tatttttgga ggtttaagta agattttagt aattcgtttt 3960
acggattttt agattttagg tagtttttgt ggttgtaggg agtttgggtt tattcgttgg 4020
gtttaaggag aatttttcga tttggggttc ggttggttcg aggaatcgga ttttgtgatg 4080
aagatttttt aggttggata cgtaagtaaa ttaaatcgta aattaatgat acgaaaatta 4140
tatttatacg aaagaaaaat cgattacggt tataaaagtg tatggaattt gatttcgttt 4200
tggagaaata ttatataaaa gttattttta atagacgttt gttatttaag agcggtaggg 4260

atattgtttt ttatgcgttc gtaaaaatag agtcgtaatt gtagttgttg ttgttgcggg 4320
taggatttat ttttttaatt ggttaaatgg tttttttttt ttttcgaagg tgatatttgt 4380
atttttaaaa tttagagttg ttggtaggac ggttagtatc gattttaatt tcgatataaa 4440
aataagaggg gttgtaaaac gggggaaata aagttgttgt aaataaaatg taagttatta 4500
ttttttttta attttttgta ttttcgtcgg gcgcgcgatc ggtagttgac ggtttaataa 4560
ttggtatatt ttaatggaat tgcgagggaa atgtaataat tttgttataa tgggttgtaa 4620
ttttaattcg atttcggttt ttgtagtttt cggtcggaag ttgggcgatg agttttgttt 4680
ttagcgggtg gcgttcgagt tcggttgaac ggcggtaatt ggcggcgggt acgcgttcgg 4740
ggcgcgcgcg ttattttttt cgtttttatt taattttttt attagtgtac gagtttattt 4800
ttagaggtta ttaggtagga tttacgattg gataataaaa gtacgtgatt cgaagtcgta 4860
ttttatattt gggtgtttac gtaggaggga attaagtata tgttttagtt atttttataa 4920
tttattataa attgtgtaag ggtgttatag acgtataaac gatcgcgagt tataaattaa 4980
gtatatatat taaaaaataa atgagttttt attttgtaaa tttattttgc ggtcgttatt 5040
taaatggttc ggattattag ttgtataatt atggagatta tagtttcgtg agcgagtaat 5100
ttagggattc ggcgagtatg tatttcggta ggtacggtta cggttataat ggtatggatt 5160
ttagcgtcgg tcgttcgggt ttcggttatt ttggtttcgg agagcgcgtt cgtagttacg 5220
ttgttagcgt tagcgcggcg ttcgtcgagt ttaggtatag ttagtcggtt acgtttacgt 5280
atttttttta gttcgattcg ttgttttgtt tcgtcgtggt tttttcgttc ggtagcgata 5340
gttattacgg cgggaaaaat tttttaagta attttagcgg cgtttcggtc gacgtcggta 5400
gtatttatat tagtagtaga gagggggttg gtacggcgtt cggagtcgag gaggacgttt 5460
ttgttagtag cgagtaggcg agtgcgtaga gcgagtcgag ttcggcgtcg ttcgtttaat 5520
tttagattta tttttggatg cgtaagttgt atataagtta tggtaaagtt agtttttttt 5580
taaatttacg cgatcgcggg gacgcggttt tcggtttttt tttttttttt tgtcgttttt 5640
ttttagtttt ttttggtttt atttttttta gttttgcgga gttttttttg tcgttttttt 5700
ttttttgttt tttttttttt tttttttttt tgcgagtatt ttgcgtttat aaatagtggg 5760
ggaaatgggc gtttttgggg atagtttaga cgttggaagg gggaggaagt aaaaaatttt 5820
tttggaattt tacgttttgg gacgcgtttt cgggttaggt tagtcgagta aggcgtagag 5880
aggtagagga tggttgtagt agtcgtgaat cgggtttgtt acggcggata atttatgagg 5940
agggttacgt tggggaaata gcgttattaa ttatagtttt cgaaaagggg tttgggggaa 6000
agaatcgagg cgagagtttg taggattttg aattttgggg gtaggaggga gagagaagga 6060
aagggaagaa aaagaaaata ggttttttaa ttttgtaggt tggaaacggg aggcggtttt 6120
cggggttgga attttgaggg agggtgattc gaaggttatt tgggcgttta ggaaagggtt 6180
ttgttttttg ggtttttgtg cggtgggtag tttgggaggg ttgtgttttt cgatcggggc 6240
gttcggggta gggcggaggg ggtaggagag gggttaggga aagtcggagt tcgtcgggat 6300
acggttttag ttttagatgg gtagattgtt tttagggttt aaatcgtatt gttttttttt 6360
tagaaaggaa gagagaagga aattcgggag gggtgtgcgg gttggtaggt agaatttgtt 6420
gagtttttcg tttgggtttt ttgtttatga tttaagtttg tttttttggc ggattttgga 6480
agataggagt tggtggttaa atcgttgatt tttttattgt aga 6523

<210> 138
<211> 6523
<212> DNA
<213> Artificial Sequence

<220>
<223> chemically treated genomic DNA (Homo sapiens)

<400> 138

tttgtaatag aaaagttagc ggtttagtta ttaatttttg ttttttaaag ttcgttaggg 60
ggataagttt gggttatgag tagggaattt aggcgaaaag tttaataagt tttgtttatt 120
agttcgtata tttttttcga attttttttt tttttttttt ttagaaagaa aataatacga 180
tttggatttt gggaataatt tgtttatttg aggttggggt cgtgtttcgg cggatttcgg 240
ttttttttgg tttttttttt gtttttttcg ttttgtttcg ggcgtttcga tcgggaggta 300
tagttttttt aggttgttta tcgtatagaa atttaggaag taaggttttt ttttgagcgt 360
ttaagtggtt ttcgggttat ttttttttaa agttttagtt tcgagagtcg tttttcgttt 420
ttagtttgta gggttgggga gtttgttttt tttttttttt tttttttttt tttttttttt 480
tgtttttaaa atttagaatt ttgtaggttt tcgtttcgat tttttttttt aagttttttt 540
tcgggggttg taattagtaa cgttgttttt ttagcgtagt tttttttata aattattcgt 600
cgtgataagt tcgatttacg gttgttatag ttatttttta tttttttgcg ttttgttcgg 660
ttggtttgat tcgggagcgc gttttaaggc gtggggtttt agaggggttt tttgtttttt 720
ttttttttta acgtttaaat tgttttagag aacgtttatt ttttttatta tttgtgagcg 780
tagggtgttc gtaaagaaga ggaggaagga ggaaggtagg ggagggagaa cggtaaggag 840
agtttcgtag ggttgggaga aatgagatta agagagattg ggagagggcg gtagagaaga 900
gaggggggat cgagagtcgc gttttcgcgg tcgcgtggat ttagaaaaag gttggtttta 960
ttatgattta tgtgtagttt gcgtatttag gggtagattt ggggttgggc gggcggcgtc 1020

gggttcggtt cgttttgcgt attcgtttgt tcgttgttgg taggggcgtt tttttcggtt 1080
tcggacgtcg tgttaatttt ttttttgttg ttgatgtggg tgttgtcggc gtcggtcgag 1140
gcgtcgttgg agttgtttag ggagtttttt tcgtcgtggt ggttgtcgtt gtcgggcgag 1200
ggggttacgg cggagtaggg tagcggatcg ggttgaggag agtgcgtgga cgtggtcggt 1260
tggttgtatt tgggttcggc gggcgtcgcg ttggcgttgg tagcgtagtt gcgggcgcgt 1320
ttttcggagt taaagtggtc ggagttcgag cggtcgacgt tgagatttat gttattgtag 1380
tcgtagtcgt atttgtcgga gtgtatgttc gtcgagtttt tgaattgttc gtttacggaa 1440
ttatgatttt tataattatg taattggtag ttcgggttat ttggatagcg atcgtaaaat 1500
gagtttataa aataagagtt tatttgtttt ttgatatgtg tgtttgattt gtggttcgcg 1560
gtcgtttgtg cgtttatagt atttttgtat aatttatgat gaattatgga aatgattggg 1620
atatgtattt ggtttttttt tacgtaggta tttaaatatg gggtacgatt tcgaattacg 1680
tgtttttgtt gtttagtcgt aaattttgtt tgatgatttt tagaggtaaa ttcgtgtatt 1740
aataggggag ttgggtggag gcgagggggg tggcgcgcgc gtttcgggcg cgtgttcgtc 1800
gttagttgtc gtcgtttagt cggattcgag cgttattcgt tggaggtagg gtttatcgtt 1860
tagttttcga tcgggggttg taagggtcgg ggtcgaattg aggttatagt ttattatggt 1920
aaaattattg tatttttttc gtagttttat taggatgtat taattgttag gtcgttagtt 1980
gtcgatcgcg cgttcggcga ggatgtagag gattgggggg aggtggtgat ttgtatttta 2040
tttataataa ttttattttt ttcgttttgt agtttttttt atttttgtgt cgaggttggg 2100
gtcggtattg atcgttttgt tagtagtttt gaattttgaa aatatagata ttattttcgg 2160
ggaaggggga aagttattta gttaattgga gaaataaatt ttgttcgtag tagtagtagt 2220
tataattacg gttttgtttt tgcgagcgta tgagggatag tgtttttgtc gtttttaaat 2280
gataggcgtt tattaaagat agtttttgtg tagtgttttt ttaaggcgag gttaaatttt 2340
atatattttt ataatcgtag tcgatttttt tttcgtgtga atatggtttt cgtgttatta 2400
gtttgcgatt tgatttgttt acgtatttag tttggaaaat ttttattata gggttcggtt 2460
tttcgagtta gtcgggtttt aagtcggagg gttttttttg aatttagcga gtgggtttag 2520
gttttttgta gttatagagg ttgtttgggg tttggggatt cgtggggcgg gttattgggg 2580
ttttgtttag atttttagga gtaaaatgag ggcgataatg gaagtatttt ttggtagtgt 2640
ttagtatttt tgtagttatt ttttacggtt tcgaaagatt taagtaaatt ataaatatag 2700
ttgagaggta agtggagttt tttcgttgga ggttcggcgt tgtaggcgtt tttggtacgt 2760
ttggaagtta ggattttggc ggtttttatg gttttgggtt tttcgttggg ttttgaacgt 2820
tgttgtggcg gcgacgcggg cgttatcgga ggttgggagc gggaattcgg agtcgggagt 2880
ttatttcggg ttgtaatgtt ttattcgcgt ttaggttaat tcgtttcggt tgaggttgtt 2940
ttttttttat tgacggttgt atacgcggga tcgagagatt gggttttgtt ggggtttttt 3000
ttgtttttcg agtttttttt ttgttttggg aggcggtttg ggaggtcgcg ataaggtcgg 3060
gttttagttt ttagattttt tttttttatt ggttagagat gatttgatga tgtttttcgg 3120
gatttattgg cgagggattt aggtagagac gtttagatac gaaacggggt tcggtttagg 3180
gttttttttt ttttagtagt ttcgcgtttc gaggtcgggg agtttagaga tattagtata 3240
ggagttttag acgtatttag ggcgtatttt agaatttcgg agtcggtttg ggtatttttg 3300
tggagcggga ttgggtgtgt gtagtgcgtt tcgttttatc gttggtattg gttgtgtgtg 3360
aggttttgtt ttgttttgtt ttgttttgtt ttgttttgtt ttgttttgtt ttgtaagaaa 3420
taaatgtata gacgtttgta aagtttcggg tttttttgaa gttgcggaag tttttagatg 3480
ggagtaggcg gggagaaaag ttggggaata ggcgagggta agggggtaaa gtcgaaggag 3540
gttgtagcgt tggtttggtt tttgtttagg tatttattcg ttcgtttttg tttttgagtt 3600
tttttcgttg ggttgcgtgg aattgatgag tttgtttttt tttttttatt ttatgcggcg 3660
gttttggaat tagattttga tttggcgttc ggtgaggtag agcgcgttgg cgatttcgat 3720
gcggcggcgt cgtgttaggt agcggttgaa gtggaatttt ttttttagtt ttagtgtttg 3780
gtagcgcgtg taggtttggc ggtttcggcg tttatggttt ttatatatag tatttacgag 3840
tagaaacggt cgggcgtcgg taagttaggg tttggagggt tgatttagtt agtttagtgt 3900
tttttataag aggtatttag attagaaatt atttcgtttt tattttagtt ttttttatta 3960
tgtttggggt ttaaagtata ttgaatggga gattatttta tattaagcga gatgtttttt 4020
atttataacg atttggggtg gatattatcg tttttgaaaa atttgtttta taggaaaatt 4080
attataagta aaataataga atgagtattt ttaatttggg atgtttaggt cgtgtttttg 4140
tggggatgta gggtgtgtgt gtgtgtttgt gtgtgtgtgt gtgtgtgtgt ttgggtgggg 4200
tggtgtggaa ttatgtaagt agtgtatata ttttgaattt atagtggttt ttttgaggat 4260
gttattgtga ttagttatgt cggaatttaa tatatttttt gaagagttaa aaatggttag 4320
tttttttgaa tttttatata tttttttaaa ttatatgatt tttttgagaa atgtagaatt 4380
tttttaagga taaagtggat gttaatggaa aatattataa ttttaataat tcgtattagg 4440
ttttgtttta gggaattttt aggagttttt gggatttttt gtggagtttt tggaaattag 4500
aggttaagag atggagttat aggtagttta ggatttttgg taatgtagga ttgtgttggg 4560
ttgtttgatt tggtgttaaa gttttttaag ggtggtgggg ggttttttta agataggtgt 4620
atgaagttta ggtatgggtt tagaagaggg tagaagttgg ttaagagtgg gtagttgagt 4680
agaagtcggg ggagatgagg gaggagagag aaaaaaattt tgagtttggg gttgtggttt 4740
tttaaggttt tggggagata ttggaagagg ttaaatggtt atttttttgt acgggttttt 4800
gaagggtaga aaggagaggg ggttgggaag taaagggtat ttattagtga cgtagtgtaa 4860
aaggtttttt ggtttgggaa gttgagtagg gtatttaggg tgtatggata ggttgttttt 4920
ttttattagt tttttttttt ttttaggttg tttttgttat aggttttata tacgtgttag 4980

tgtttttatt tttttttaag gtgttagttt atatggatat tggggttttg tttttttttt 5040
gttatggttt gatagtttta ttgggaattg attttttttt tttttttaag aagttaaaga 5100
aattttgttg gtttttttat tttttttttt tttttatttt tttttttatt ttgttttttt 5160
tttttttttt tttttttttg tttttttttt tttttttttt tattgttttg ggatatgttt 5220
tattcgcgta ggagtttatt cgttgtattt aagggtaaat cgggttcgtg tattttcggt 5280
cggcgttttc gttatggagt gttttgtttt gttcgttgtt gttgtcgggt ttgtattgtt 5340
gttcgggaga aaagtgtagg tagttttcgg ggtttttttg tttgttattg ttcgagggcg 5400
aggcgttatt gaggttttta ttagaataga aatacgaggt ttcgtattcg taggacgttc 5460
ggttgtaggt taggatcgag ttggattgtt ggtagaaata aggtgaggtg tacgttttgt 5520
tcgggagatt cgacgtttcg tacgaggtcg ggaagggttt tagcgcgtta tagttagttt 5580
ggtagagggg tagttggttt aagaaggagt tttggtcgtt gggaaggttt tcggggaaag 5640
tgggatttat aaaataggaa tttatttgcg cgtttttttg taggattgtg atttgttgtg 5700
tattagtata tttggttata attattagta gttatcgaat tggtttgttt ttggatggtc 5760
gaacgttaaa agcgatagta gtaaatcgta ttagttgacg cggcggcggt taatgggagc 5820
gaacgtcgga gttcgttttt cggggatcgc ggcgatcgag gtagtaaagt tataaatagt 5880
ttttagttcg ttcgttcgtc gttcgttcgg tagattaatg ggcgcgtata gttagtcggt 5940
tcggtttttt tttcgaacgt tagcggcggg tacggttttt ttagtggcga gtagattagg 6000
ttagagtagg gatattaatg tttgggtcgt tttaaaggtt tcgttaggtt tttttttttt 6060
tttttttttt tttgttttat tgaattttgc gggtttttcg tttttttttt tttaaatagg 6120
aaatttgaag gttttgtttt aggaggggta gtgataaagg tggttttgtt ttttgtcggt 6180
ttgtatttta ggtacggttt agagttgttt tttttttagc gtttttttat tcgtgggtgc 6240
gagtttttga gttggagtaa gggttatgag aaattaggtt tttattttat gtttttattt 6300
cgtgcgtttt aggaatcggt gaattttttt attttttttt ttttcgttag tattaggggt 6360
ttgggttttg tagtttagag ttgaaggatt atttcgagtt gattaggatt tagttaaacg 6420
taggtttttt ttatttattt ttttttttta tatatatatt ttgtttgagt tttaaggggt 6480
ttaatggttc gcgtttgttt ggtgttaaag tatttttttt agt 6523

<210> 139
<211> 6430
<212> DNA
<213> Artificial Sequence

<220>
<223> chemically treated genomic DNA (Homo sapiens)

<400> 139

tttttttcgt tcgatttttt ttttttattt tacgattttt gtaaagttgg atgtgtttta 60
agtttaaaag gggaattagg gcgcgggtgg atgaggcgtt ttttaatttt tatttttatt 120
tcgtcgttag gttgggacgt tttttgttgt tgtagataga aggaatttta aagaataggt 180
agtaagagtg tgttataaag gtcgggtttg cgaattgttt ggaattcgtt ttttaatgag 240
tttataattg tttagtttta attaggatat tttattaaag tttttttatt tgtttatttg 300
ttttgtttgt cgtcgataaa gcggttgcgg aaatattttt tttattggta ttgttttttt 360
tgtagtagat ttcgggtttt acgtagattt ttcgggattt aggtttacgt ttttcgtttg 420
ggagtggggt gcggtgggtt agtcggcggt ttttaaagtt taggtcggcg gaggtagttg 480
aggttgtagt attaaacggc ggttagtaga tggtagtgtg gttttatgta agaggttttc 540
gcgtcgttat ttttcgtttt cgcgattttt gtgaagtttt taagatgtcg atttgatacg 600
agtttttttg gaatgggagt tttagatttt agtgaatagt cgttttatat tttttgggtt 660
gtttaagttt ggttagaagt ttcgttggga aataaggaat cggcgggggt ttgttcgatt 720
tttatcgttc gcgttttttt gattttattt cggggtttta gtgttgtcgg gttgtagttg 780
ttcgggcggt cgcggggttg tagttttagg cgcgggcgcg gttttagggt tatcgttttt 840
agttgggata ggtttgatat agttaggcgt tagagagttt cggttgattg gcggatatag 900
gaggaacgtt tgtagtttta gaatttgatg atcgtttaga gggtgtgtac ggtgcgtgtc 960
gagggtttat atcgtgatcg cggggttcgc ggtcgtttag gaggttttcg aaatttgcga 1020
tttgggttag ggttcggcga gttagaattt aagatagagt taggatttcg ggattcggtt 1080
ggtttgaatc gagataataa aattaggcgg tgaaaattgg gcgtaaaggt aatttcggtg 1140
tttgattatt gtaggtatag gggtcgggtc gagttggttg ggaggagaat tttgggggat 1200
aaattaggga ttgttttggt tattttttta atgtgatgtt taatataata attatatcgt 1260
agtattaaaa gtgtcgtata tagtgtggaa gtcgttcggg ttgtttttgg tcgcggtatg 1320
tttttcgttg tataggaatc gaggttgttt tggcgtttat ttattaggag gttagaaggg 1380
aggggatttt aatttaggtt tagttttttt tagattttag gcggggaata cgtttcggtt 1440
ttttttcggg tgcgcgtatt agttacggat gtcgaatggt tttagaggta aaacgtaaaa 1500
gtaaatagag aatttttcga aaggtttcgg agttttgtga atacgttata tagtgtattt 1560
atttgggtcg taatagtttt cgttaagttt ataaaatgtt tacgattcgt attttagtgt 1620
ttttacgttg taaaattggg gattggattt tggttgatgt ttttttcggg tgtttgtttt 1680
tttttttttt ttatatatat ataatatttt tttttttttt tttttttttt ttgtttttta 1740

ttttatatat atatatatat atatatatgg atagatagat agatagatat agatattata 1800
gatatatatt tttattatat tttcgtatat aattttataa cgtttgattg taagaagggt 1860
tttatagttt ttttgggagt taatcgtggt tttgtattta agtcgtagat agagagttta 1920
atttatgtta attgtaaata aatagtgaag tcgtagattc gtggatgtag agagaaagtt 1980
cgatttgtgt aattataaat aaatgaagtt atagatcggt ggcgattgcg tttttttatt 2040
gattacggag gttttaggta ggtgtaggtc gtttagggtt ttttaggttt gggtggattg 2100
tttaaaggtt ttttttttgt ttttggtagg ttggtggttg ggagtttgtt ttttttttgg 2160
gaagtttggg agtggttttt tcgttgcgga tttttattta ggtaagattt cgtattaatt 2220
taattttatt tgtatacgat aggtacgagt ttgtttttgt atttaggtta gggattattt 2280
atttattgga tggggaatag atgtagttgg tgagagaggt aagggtggaa atttttagga 2340
agtaatttgt ttgttattta aatttttgga aggcgttgtt ttttgtttag agttggtttt 2400
atttttataa ttgaatttaa atattggtat ttggtaataa gaagtagagt gaatacgttt 2460
tgttagtagg taggaaggaa gtttagggac ggtagtggaa aatgtttatt taaatatagt 2520
tttattgatt agaaattcgt ttatatgtgt ttgttttagg ttgagggtta atgagtattt 2580
ttgtgaaaat tattttatat aatttagtgt atttgtgttt ttttagttaa ataagtagag 2640
aattaatgaa tagagaatat atagttaaag aagttttatt gaaagtttga ttttattcga 2700
ttttttcgtt ttaaattaga ttatagaggg taagagggga gaaggaagtt tggaaacgaa 2760
agggaggttt cggtgttcgt aggattcggt ttagttggtt gatttgggtt tataaaggtc 2820
gaagttttgc gggtagtatt cgtatttttt ttggtcgggg tagaggggta gtgcgttttg 2880
ttagaatttt ttggacgcgt atcgggtaaa atcggagtag gaatgagtcg gtggcgaggg 2940
tattcggttt ttttttggcg aagtgcgttt tttttcgcga ttttcgaaat cgttttttgt 3000
tcgggttgtt ggattgggta ggttttattt taaatattag ttttgttggt ttttttagtc 3060
gtttgagttt agatagacgg cggcggcggg ggttttggtt tttttttttt gcgttttgtt 3120
gtgtgttatt ttaggaaaaa tttattggtt taaaacggtt taattttgtt tttttgattt 3180
tttagttttt ttgtttgttt tatttttttt taattttttt gggtttttat ttttttttag 3240
aaggcgatgc gattgggatt atttaggttt tttttttttg ttttattttt tttatttttt 3300
atcggagata aggtattatt tgattcgtta taaaggcgtt ttcggatgtt gttttttatt 3360
ttatattttt atttttaggt tttcgtggtt cgggaggaga tagtttagcg tttatttgtt 3420
ttattagttt tcgttttttt atttaagttt cggtggttta ttgcgtagga attggggttt 3480
cgtggcgttt cgatttgaat ttttttttta ttttttgtat ttttggggtt ttttgagagg 3540
gtgcggtaaa cggggacggt ttggattttt tgaaagtgat gggaagtgtt tagtatataa 3600
ggaattaaat tttataattt ataggagaat tttcggggta attttattgt gaggtcgggt 3660
ttatggtgtg taatcgagtt gagtagtaaa aggtggtcgg gtttttagcg tagtcgggta 3720
gtttatgagg cgtttagggg aagggttttt tttattattt tttatcgagc ggtgggcggt 3780
aggggtttta gttggtgtaa tatggtgtag ggtcggaggg tttggaaggt aggaattgga 3840
ggtttggatg ggaggaagtg agattattgt gcgtttagtt ttatgtgagt tggtatttgg 3900
ataggtgaag tttttatagt tgggtggttt ttagggggtg gtttttttta tttgttttcg 3960
gtgtttttaa aagaaaagga agtttatagt gggttgtagt ttttagacgt tagagttttt 4020
tttttaaatt cgttttaaag tatgtattgt ttttaggtat ttcggtttat tttagaaatt 4080
ttttagtatt gggagtgttt ggaagggtgt aggttcgaga tttttttaag ttgggggaaa 4140
tagttttggt tttttatgcg ttagagggga attagaagtt gtggaaagga taaaggaatt 4200
tttagtaatt tatcggattg ttggagttag ttaggtgttt aggagcgaag gtatagagtt 4260
tgtagtatta tagcgttcgg gaggagggag gggaatttta cgttaataaa tttgttggta 4320
aatgagtttt attaatagat aaatatttta ttgttttttt tagggcggat aaatagtgtg 4380
ttttcgatta aggaaaggaa gttgggagac gttgatttta ttcgaattat atggttttag 4440
tttgcggtgg taattttttt gtagttgtaa gagatgttgc gtttttttcg tttggattcg 4500
agtttaagtt cggtttgtcg tttattggat ttgggtgaga gaagatttgg gtagagtcga 4560
tttgtttata gttgagtttt gtttataagg ttatcgcggg gtaggttgtt gcgggggata 4620
gagatttttt tagggtttgg gtaggcggat aggagaggga tggggaggat tttaagtttg 4680
gtttagggtt tattagtagg agtcggcggg ggggcggggt ggggggtgtt gcgtggggtc 4740
gggtcgtttg gcgttcgtag attttagtgc ggaggttggt cgttagttgg gcgttttcgc 4800
ggagttttta ggtttttttt cgcgggacgc gttaggttcg tcgggcgcgg gcggattttt 4860
tggtcgtata tttgagtttt ttgttttttt attttaggcg gttgcgggtt ttgcggagcg 4920
agattatttg tgaggattgt tgagattggc ggaggcggtt atgtgggcgg ttacgtgttg 4980
cggcgagttt cgtttaaaag aaaatggggt ttggtgtaaa tttgggggtg taatgttatt 5040
atatattatt ttatttcgta aaatcgatat tgaaagttgt cggataataa attataggtt 5100
aaaattatga gtttttcgta ttatgtgaac gcgtttttta gtaaatatac ggcgggggtt 5160
tttttgtttt aaaatgtcga gtcgattttt tgtttttttg tttttaattt atagagaagc 5220
ggttacgggg cgggcgtcgg cgttttcgtt tcgatcgttt cgggtttata taatgttaat 5280
agtttttttt attagagttt ttttgcgttc ggttacggtt tgggcgtcga cgtttacggt 5340
aatttgtttt gcgtttttta cgattaaaat attttcgggt tttgtagtga tttcgttaaa 5400
ggcgtttgcg ataagacgga cgagggcgcg ttgtatggcg cggttgaggt taattttcgt 5460
atttattttt ggatgcggtt tttaggtagg cgtagtcgtt aggcgggtta ggttggcgga 5520
gcgggatcgg gagcggggag cgtagcgttg gggagcgcgg agcgcggggc gcggggtcgg 5580
aagagcggag ttaggttgtt gcgagtcggt agtttcgtga ttttcggcgt agtttttttg 5640
gttttaattg gagcggaggc gttattgcgt ggagtttata gtattgtatg taaatgaggt 5700

ttataaaatt ttttatggtt taattaatgg gttcgatttt cgtaaattta tttaatttta 5760
tttgttttgg aattttaata ttaagtttgt tcgttatttt gttttttttt tttttttttt 5820
tgttattttt tttttttttt tttttttttt ttttttattt ttttttgttg tttgatattt 5880
tttttttaaa agttaggaga gttaagtttt ttagggtttt ttattcgatt ttgtgttagg 5940
gtttatttta agatgggggc gagggaatga ggatagaggg tcggggtgtt ttaggaaggt 6000
tttggggatg agggtttagg ttgttgggaa gagtcgttta tttgtttagt aaggtagggg 6060
aatggtcggg tttatttttg aatggtaaaa gtgtagcgtt ttttatttgt ttatatttta 6120
tatttatagt ttttttttgt tagaggaagt aggatattgt ggaagtttag tcggttggaa 6180
aggtcggttg taaagttgag attttgtgtt tttgtttatt atagagttgt ttgcgcgttt 6240
ggtattgtag ttggggtttt tttttaatat tttttttttt tttttagttt agttcggggt 6300
ttggggtaga gggggatgtt cgttggggat taggttagga ggaaggtgcg ggttttggga 6360
tagtagttta acgagtgttt tgtttatttg tttgtttttg tgtttatgtg tttgtatgtt 6420
tgtttttagg 6430

<210> 140
<211> 6430
<212> DNA
<213> Artificial Sequence

<220>
<223> chemically treated genomic DNA (Homo sapiens)

<400> 140

tttgagaata gatatgtaga tatatgaata taaggataga taagtagata gggtattcgt 60
taggttgttg ttttagagtt cgtatttttt ttttggttta gtttttagcg agtatttttt 120
tttgttttag gtttcgaatt gagttagggg aggaggggga gtgttaggga aagattttaa 180
ttgtagtgtt agacgcgtag gtagttttgt aatgagtaaa ggtatagaat tttaatttta 240
taatcgattt ttttagtcgg ttaagttttt ataatgtttt gttttttttg ataaaggaaa 300
attgtaaata tagagtgtga gtaagtggga aacgttgtat ttttgttatt taaagatgag 360
ttcggttatt tttttgtttt gttaggtaag tgggcgattt tttttagtag tttgagtttt 420
tatttttagg atttttttag ggtatttcga ttttttgttt ttattttttc gtttttattt 480
tgaaatggat tttggtatag ggtcggtgga gaggttttgg agggtttggt ttttttagtt 540
tttgagaaag aaatgttagg tagtaaggaa aatgaggaga gagagaagaa gaaagggagg 600
gagggtgata gaggagggag aaagagagat agaatagcga ataaatttaa tgttaaaatt 660
ttaagataaa tggagttaaa taaatttacg aggatcgaat ttattaattg ggttataaaa 720
agttttatga gttttattta tatataatgt tatgggtttt acgtaatggc gttttcgttt 780
taattaaaat tagaaaggtt gcgtcgggag ttacggggtt atcggttcgt aatagtttgg 840
tttcgttttt tcggtttcgc gtttcgcgtt tcgcgttttt tagcgttgcg tttttcgttt 900
tcggtttcgt ttcgttagtt tggttcgttt agcgattgcg tttatttgaa gatcgtattt 960
aggggtagat gcggaaattg gttttagtcg cgttatgtag cgcgttttcg ttcgttttgt 1020
cgtaggcgtt tttggcgagg ttattgtaga gttcggggat gttttggtcg taggaggcgt 1080
agggtaggtt gtcgtaggcg tcggcgttta ggtcgtagtc ggacgtaaag gggttttgat 1140
aaagggggtt gttgatattg tataagttcg gaacggtcga ggcgaaggcg tcggcgttcg 1200
tttcgtagtc gtttttttgt gagttgggag taaaggagta agaagtcggt tcggtatttt 1260
ggaatagaga agttttcgtc gtatatttgt taaaaagcgc gtttatataa tacgaagaat 1320
ttataatttt gatttgtgat ttgttgttcg gtagttttta gtgtcggttt tacgaggtag 1380
agtgatatat gataatatta tatttttaga tttatattaa attttatttt tttttggacg 1440
gagttcgtcg tagtacgtga tcgtttatat gatcgttttc gttaatttta gtagttttta 1500
taggtggttt cgtttcgtag ggttcgtagt cgtttagaat ggaagggtaa gaggtttaaa 1560
tatgcggtta aagaattcgt tcgcgttcgg cgggtttggc gcgtttcgcg gaaaaagatt 1620
tggaggtttc gcgggagcgt ttagttggcg gttaattttc gtattggggt ttgcggacgt 1680
taggcggttc ggttttacgt agtatttttt atttcgtttt ttcgtcgatt tttgttagtg 1740
agttttggat taagtttggg atttttttta tttttttttt gttcgtttgt ttagattttg 1800
gaagggtttt tgtttttcgt aatagtttgt ttcgcggtgg ttttgtgggt aggatttagt 1860
tatgagtaga tcgattttgt ttaagttttt ttttatttag gtttagtggg cgataggtcg 1920
gatttagatt cggatttaga cggggaaggc gtagtatttt ttgtagttgt agagagattg 1980
ttatcgtaaa ttggagttat gtggttcgaa taaagttaac gttttttagt tttttttttt 2040
taatcggagg tatattgttt attcgtttta aaggaagtag tgaaatattt atttattaat 2100
gagatttatt tgttaataga tttattaacg tggggttttt tttttttttt tcggacgttg 2160
tagtgttgta ggttttgtgt tttcgttttt gggtatttgg ttggttttag tagttcgata 2220
aattgttaaa gatttttttg ttttttttat aatttttggt ttttttttgg cgtatgggga 2280
gttagggttg ttttttttag tttggaaaaa tttcgggttt gtattttttt aggtattttt 2340
aatattggaa ggtttttggg gtaggtcggg gtgtttggga ataatatatg ttttagagcg 2400
gatttggaga gggggttttg gcgtttaggg attgtaattt attgtggatt tttttttttt 2460
ttgaagatat cgaaaataaa taggggaaat tattttttaa aggttattta gttgtggagg 2520

ttttatttat ttaagtatta gtttatatgg agttgagcgt atagtggttt tatttttttt 2580
tatttaggtt tttaattttt attttttagg ttttcgattt ttgtattatg ttgtattagt 2640
tgaagttttt gtcgtttatc gttcggtgga ggatggtggg gagaattttt tttttaaacg 2700
ttttataaat tgttcggttg cgttggaagt tcggttattt tttattgttt agttcgatta 2760
tatattataa attcgatttt ataatggaat tgtttcggaa atttttttgt aaattgtaga 2820
gtttagtttt ttgtgtattg agtatttttt attattttta gagggtttag gtcgttttcg 2880
tttatcgtat ttttttaggg ggttttagga atataaaagg tggagggagg gtttagatcg 2940
ggacgttacg gagttttagt ttttgcgtag tgaattatcg gggtttgggt aggaaggcgg 3000
gggttggtgg ggtaggtggg cgttgggttg ttttttttcg ggttacggaa gtttgggggt 3060
aggggtgtgg agtgagggat aatattcggg gacgttttta tggcggatta gatgatattt 3120
tgttttcgat ggagaataga gggagtaaga taaaaagaaa agatttaaat aattttagtc 3180
gtatcgtttt ttggaaggaa ataaaaattt aagaaagtta aaggaagatg aagtaaataa 3240
gaaggttagg agattaaaga ggtagaatta gatcgtttta gattaataaa ttttttttgg 3300
ggtgatatat agtaagacgt aaagagaaaa gattaaggtt ttcgtcgtcg tcgtttgttt 3360
agatttaagc gattgaaggg gttaatagag ttggtgttta aagtagaatt tgtttagttt 3420
aatagttcga gtagggagcg atttcgggga tcgcggaagg gaacgtattt cgttaaggga 3480
gggtcgggtg ttttcgttat cggtttattt ttgtttcggt tttgttcgat gcgcgtttag 3540
gaggttttgg taggacgtat tgtttttttg tttcggttaa ggaggatgcg gatattgttc 3600
gtaaggtttc ggtttttatg gatttaagtt agttaattgg gtcgagtttt gcggatatcg 3660
aaattttttt ttcgttttta ggtttttttt tttttttttg ttttttgtgg tttgatttaa 3720
aacgaaaagg tcggataaaa ttaggttttt aataaggttt ttttaattgt gtgtttttta 3780
tttattggtt ttttatttat ttgattgaaa agatataaat gtattaggtt atgtgagata 3840
atttttatag aaatatttat tgatttttag tttgaagtag gtatatgtag gcgggttttt 3900
aattagtaga gttatgttta gatagatatt ttttattgtc gtttttgagt ttttttttta 3960
tttattggta gagcgtgttt attttgtttt ttgttattaa atattaatat ttaaatttaa 4020
ttatgaaggt aaagttagtt ttaggtaggg aatagcgttt tttagagatt tgggtggtag 4080
gtaaattgtt ttttaaaggt ttttattttt gtttttttta ttagttgtat ttgtttttta 4140
tttagtgagt gagtgatttt tagtttgggt gtaaagataa attcgtattt gtcgtgtata 4200
aatgagattg ggttggtgcg gggttttgtt tgggtgaagg ttcgtagcgg ggaagttatt 4260
tttaggtttt ttagaggaaa agtagatttt tagttattag tttgttaagg gtaagggagg 4320
agttttggga tagtttattt aggtttggga gattttgggc ggtttgtatt tatttgaggt 4380
tttcgtgatt agtggagaga cgtagtcgtt atcgatttgt gattttattt atttgtagtt 4440
atataaatcg agtttttttt ttgtatttac ggatttgcga ttttattatt tatttgtagt 4500
taatataaat tgagtttttt atttgcggtt tgaatataaa gttacggttg atttttaggg 4560
aagttataaa atttttttta taattagacg ttgtgaaatt atatacggaa atgtaatgaa 4620
ggtgtgtgtt tgtaatattt gtatttattt atttatttat ttatatatat atgtatatgt 4680
atatatgaga tgagagatag agagggagag agagaaagga gagatattgt gtgtgtgtga 4740
aaggaaagag agaataaata ttcgggagag atattaatta aaatttagtt tttagtttta 4800
tagcgtgaaa gtattgggat gcgggtcgta aatattttgt gggtttggcg gagattatta 4860
cgatttaaat aaatgtattg tgtagcgtgt ttatagggtt tcggggtttt tcgaaaggtt 4920
ttttgtttgt ttttgcgttt tgtttttgga attattcgat attcgtggtt gatgcgcgta 4980
ttcgagagga ggtcgaagcg tgtttttcgt ttagggtttg ggagaggttg ggtttggatt 5040
ggggtttttt tttttttggt tttttgatgg gtgaacgtta gagtagtttc ggtttttgta 5100
tagcggaggg tatgtcgcgg ttagagatag ttcgggcggt ttttatattg tgtgcgatat 5160
ttttggtgtt acggtgtgat tgttatatta aatattatat taaaaaaata attaaagtag 5220
tttttggttt gttttttagg attttttttt tagttaattc ggttcggttt ttatatttat 5280
agtggttaga tatcgagatt gtttttacgt ttaattttta tcgtttaatt ttattgtttc 5340
gatttaagtt agtcgagttt cggggttttg gttttgtttt gggttttggt tcgtcgggtt 5400
ttgatttagg tcgtaggttt cgggggtttt ttgggcggtc gcggatttcg cggttacggt 5460
gtgagttttc ggtacgtatc gtgtatattt tttgggcggt tattaagttt tggggttgta 5520
ggcgtttttt ttgtgttcgt tagttagtcg gggttttttg gcgtttggtt gtgttaggtt 5580
tgttttagtt gaggacggtg attttggagt cgcgttcgcg tttggagttg tagtttcgcg 5640
gtcgttcgga tagttgtagt tcggtagtat tgggttttcg ggatggggtt agggaggcgc 5700
gagcggtgag ggtcgggtaa gttttcgtcg attttttgtt ttttagcgag gtttttagtt 5760
aggtttgagt agtttagaaa atatagggcg gttgtttatt aaaatttggg gtttttattt 5820
tagaaaggtt cgtgttaagt cgatatttta ggaattttat aagggtcgcg gaggcgggag 5880
atggcggcgc ggaagttttt tgtatggagt tatattgtta tttgttggtc gtcgtttggt 5940
attgtagttt tagttatttt cgtcggtttg agttttggga gtcgtcggtt agtttatcgt 6000
attttatttt taaacgaggg gcgtgggttt aggtttcggg gagtttgcgt ggagttcgga 6060
atttattgta gaggaggtaa tgttaataaa agaggtgttt tcgtagtcgt tttatcggcg 6120
gtagatagaa taaatgaata aatgaaaggg ttttggtgga atattttaat tggggttgga 6180
tagttgtaaa tttattaagg gacgaatttt agatagttcg tagattcggt ttttatggta 6240
tatttttatt gtttattttt tgaagttttt tttgtttgta ataatagagg gcgttttaat 6300
ttggcggcgg ggtgggggtg ggggttaggg gacgttttat ttattcgcgt tttggttttt 6360
tttttaagtt tagaatatat ttaattttgt agaagtcgtg gagtggggga agggggtcgg 6420
gcgagggaag 6430

<210> 141
<211> 6036
<212> DNA
<213> Artificial Sequence

<220>
<223> chemically treated genomic DNA (Homo sapiens)

<400> 141
   agttttttgt ggtttgtatt tttttgtttg ttttaatttt attaataagg tagtaagata 60
   agtagtatta aatttatttt tttttttcga ggttgagatt aatagaaatt aagaaatttg 120
   tttagggtta taatgttaag aagtggttaa gtaaaggtta ggtatagtga ttttcgtttg 180
   taattttata ttttgggagg ttgaggcggg tgaattgttt aagtttggga gttcgagatt 240
   agtttgtgtt atatggtaaa attttatttt tattaaaaat ataaaaaatt agttgggtat 300
   gttggtatat atttgtgatt ttagttattt gggaggttga ggtgggagga ttgtttgggt 360
   ttgggaggta gaggttgtag cgggtcgaga ttgcgttatt gtattttagt ttgggtgata 420
   aagtgagatt ttgtattaaa aaaaaaaaaa aaaaatggtt aagtaagggt ttgaatttaa 480
   ttattttggt ttatatagtt agtattaatt ttattataat atgtattata tatagtttaa 540
   ggtttttaat tagatttatg atagtttaat tagtttgtat taaaagaaat attttttttt 600
   taatagtaat aaaaataatt tattttatag tgaaaaattt gtggtggtgt atgtttgtag 660
   ttttatttat ttaggaggtt gaggtgggag gatcgtttga gtttaggagg ttaaagttgt 720
   agggagtcgt gattatatta ttgtatttta gtttggggta atagagcgaa attttgtttg 780
   aaaaaaaaag aaagaaagaa ggaaaaaatt atgaatattt tttatttttt aagttttaat 840
   tttttttttt ttttttgagt tttgttttgt cgtttaggtt ggagtgtaat ggtgcgattt 900
   tagtttattg taattttcgt ttttcgggtt taagcgattt ttttgtttta gttttttgag 960
   tagttgggat tataggtgtt tgttattata ttcggttaat ttttgtattt tttagtagag 1020
   atggggtttt attatgttgg ttaggttggt tttgaatttt cgattttagg tgatttgttt 1080
   tttttggttt tttaaagtgt tgggattata ggtatgagtt attttattgg tttagtttta 1140
   atttttgatt ttgggtttgg tttttttaga tttgggttgg ggagagtaga tagagatatt 1200
   taaggtttaa tgagagaatt tagattgttt ttgttaagtt tttagttgaa gtagtttttt 1260
   ttatagaaag gattagaatt tgaggttaaa tttttaatat atttttattt tggttagata 1320
   gtttttttgt ttgtattttt agtgtgataa ggagtgaaaa ttgttaaaga taattttagt 1380
   tttaaatatg tttatgtttt ttttgttttt ttttatgtat aagtagatta tataaattag 1440
   gatattaatt ttattttatt ttggtgttgg gtttatttag attttttttg gtgagtttaa 1500
   tttggtttat taaaaatttt atgggttata ggttatttta gttagcgaag ttttttttag 1560
   ggaggaggag gagattggga atttagggag taaagttgtt tggttttttt agtttttttt 1620
   gttggggttg ggagagttag taggaatttg ggtaagtttt aatgtttttg ttaatataag 1680
   agaaggtggt atgatttagg gatgaattgg ggagtatagt gaatttggaa agttgaggta 1740
   attattgtaa ttgatagtat aatttgaggt attttttttt ttttttttta aatagagata 1800
   ggttttttgt tatttaggtt agagtgtagt ggtgtggtta tagtttattg tagttttgaa 1860
   tttttgggtt taagttattt ttttttttga gtagttggga ttttagggag gtattattat 1920
   atttagttaa tttttaaatt tatttatttt tgtagagatg tggttttatt attttgttta 1980
   ggttggtttc gaagttttgg gtttaagtga tttttttgtt ttggtttttt aaagtgttgg 2040
   gattgtattt agttaattta gggtattttt gttttcgttt ttgtttgttg ttatgaataa 2100
   tagattgttg gtgattttag ttgttataaa gggaatgtat tttttaattt ttagatttgt 2160
   gttgttttat taaattgatt tgaaatttat tttttttttt agattttaaa ttgttttttt 2220
   aattatgaag gtaaaggatg gggtgagaag atttttttcg ggatgaggaa aaggtggaag 2280
   gtgaataagt tttaagtttt ttagaagtag ggttgttaga ttagatatag gtttagttta 2340
   aatttggggt tagaagataa gttttagatt tttttagata agttattttt agcgtttgtg 2400
   gaagatattt taaaatgaaa ggatgaagta tttgtaaatt tttttttcga aaaagtaaaa 2460
   tagaaagaaa taagtttgtt tttattttaa taattttttt ttttttaatt tttttgtagg 2520
   ttattttttt ttattttaat tttttttttt tttgtttgtt tttatttttt tggtaagtga 2580
   atattttttt ttatttttat tgtatttatt aaagaagtta ttagttggtg tttttttttt 2640
   ttagtttgaa gtagggtttg aagtagagta tatatgatta ggaaaaggag gtaggggttt 2700
   tgtgaaatgt tttgagtttt tttagagttg ggggtgatgg ttaggtgtgg tagtttaggt 2760
   ttgtaatttt agtattttgg gaggtcgaag taggtagatc gttgaggtta ggggttcgag 2820
   attagtttgg ttaatatggt gaaattttgt ttttattaaa aatataaaaa aaattagtta 2880
   ggtttagtgg tgtttgattg tggttttagt tatttaggag gttgaggtga gaggattgtt 2940
   tgaatttggg aggtagaggt tgtagtgagt tgagattatg ttatcgtatt ttaatttgga 3000
   tgatagagta agattttatt ttaaaaaata aaaataaaat aaaatttttt tttttaaaaa 3060
   aatagagttg ggttaggttt ataattatta gagaagagaa aggtagtgtg atgttggtgg 3120
   tatagagtag aaagtaggag aggtagttac ggtaaggtta agtaaaataa gagggaatgg 3180
   gagataaaga tggaggggta agatatagat tgtaggggat aattttgggg ttttttgtag 3240
   aggaaggagt attcgttttg ttttttttgg ggtgtggaaa tttgagaagt tttgagggtt 3300
   ttttttaaat ttgttttatt tgtttttttt tttttttttg agtttcgttg tttatagaga 3360
   ttattttttg aatttttgtt tttttggatt tgttttagtt tcggttttag gttttcggtt 3420
   aggtagatat tttgataggt tataaatgag cgtgggtgtt ggattgtttt aagtttttgt 3480
   ttttaagttg ttcggaggag gagagttaat gggtttattt taagggtttt ttttttagtt 3540
   ttatttgggt ttggagtttg agagagtgaa gaagatatat tttgtgtttt ttcgtaatag 3600
   ttatatagaa taaaaagggg cgagatttta ttttttattt tggagtttta gaatttagag 3660
   ttttagggtt tttatttcga tttttttatt ttttgattaa atttttttta ttaattttag 3720
   aaaaattatt gaaatttgga ggaattttta agatttaatt ggtattaaat ttatgtattt 3780
   gaggtttgga aatttttttt ttttggaagg agatggaggg tggtgaagga gagataggta 3840
   ggagggaatt aggagaatgt aggtttttat tagttttaaa tattttaaga agagaataaa 3900
   aaattttttt tttttttatt tcgttgtaaa tattatttgg ttgtgtttat tgtggttttt 3960
   tagagagata tagggagtta tttaagtttg agtgagtgag ttagttatag ttatggatgg 4020
   ttagagagag gataaatata tttagagaga tgagaggtag ggaacgaata taaatatgga 4080
   gaaagatgga cgaatagaga gatggattga gatagagaga atataaagag aggttgaggg 4140
   agagaaatgg atgtgtgaat gaggagagag tatttatgta aagagagaga gagggaagat 4200
   ggggtatttt ttattttttt ttttatattt ttgtagtttt gttttgtttt ttgaaatgtt 4260
   aattttttat ttttggtttt atgaattttt tttttagatt ttagattttg ttttatattt 4320
   ttaggtgttt ttgtattatt tttttttatt tttttattat atagttattt atttggttta 4380
   agataatata ttttattcgg ttgttttagt ttattatttt aagataaatt ggaagaaagg 4440
   aatatggttt gggagttttg ggtaaagtat ggaagttgga ttttttattt ttaggtttat 4500
   agatttaggg ggaaggtttt tttgaggggt gggaagagtg aggggtagtt ggtttgaatt 4560
   tgtttaaatt taataattta ggagtttatt gaaggttttg ggggttggga gggaaggaaa 4620
   gttttgaata tttttttaat tttttttttt tttttttttg gttttttttt tttaaaagtt 4680
   tttgaagaaa gatgtttttg acgtttttat gtcgttttta gatggatggg ttgcgggtga 4740
   ttgaagtgtt tttgttatgt taatgtttgg ggggtgatgg atgggcgttg gggttgttaa 4800
   attttggttc gtttagttta ttggtttggg agagattata tgtgtttttt tatttttatt 4860
   ttttattttt tttttaggga agcgtttgtt taggcgagcg ggttaggtta tggattcgag 4920
   ttttagcgtt gtgatatatt gtcgaaaggt tgtagggtaa gagggtgttt tttttaaacg 4980
   ggtcgatttt tttgcggttt tgacgtatgg attataatag gatgaatttt tttttagagt 5040
   atttattttg taatcgggga tttagcgttt atagcgttta tagcgtttta attttttttt 5100
   ttttaagttc ggtttaggcg gttgatagtt atgtaagcga gggtcgttac ggtggggggt 5160
   tgtttagttt tgcgttttag tagaatttcg gttatttcgt ttagtagtcg ttttcgattt 5220
   tgggggtgtt tttttttagt ttcgcgtttt cggggtatgt ttttgtcgtt tgtagtttta 5280
   gttacgggtt tttttagtat tattttttgg gttaattaga aggagacgga ggttattttt 5340
   atttttcgag ttacggggtt tagttagggg gtttgttcga tggttacgga gtaggtggag 5400
   tcggttcggg gttatatttt tcgtagtatt ttttttatgg gaacgagtag atcgcgagtt 5460
   ttgtatcggt ttatgttgat ttttttttcg aggataagga aatattttgt tttttagaat 5520
   ttaatatttt tacggttcgg attttcgatt ggatgaaggt taagagaaat ttatttaaga 5580
   taggtagggt ttagatgtgg ttattttttt ttcggggttt aaagtatttt tgtttttttt 5640
   gtagtgatat gttttgggtt tgggttgggt tttttttttt tagcggtttt ggtagatata 5700
   ggtaagagta taatttacgt tttaggttag gtgaggtttt tttatttagt atgtaatcgg 5760
   ttattttagg agtaagtttt tatatatatt ttattttttg atttgtgtat ttagtataga 5820
   tttaggattt ggtgtgtatg ggggatggaa gtatggggaa ggttgtttta gatttggtga 5880
   ttattatggg ggtatttggt ggtttttggg aattttgata attgtatttg ggtagaatgg 5940
   taaatggagg tttagagtta gtttcgggag gagggaagcg gaaggagaaa gaggtgagag 6000
   aattgatttg gttttttttt ttgtttcgtt tttagt 6036

<210> 142
<211> 6036
<212> DNA
<213> Artificial Sequence

<220>
<223> chemically treated genomic DNA (Homo sapiens)

<400> 142

gttaggggcg gggtagggag aaaggttagg ttaatttttt tatttttttt tttttcgtt 60
tttttttttt cggggttggt tttggatttt tatttgttat tttgtttaag tatagttgtt 120
aaagttttta gggattatta ggtattttta tggtgattat taagtttgga gtagtttttt 180
ttatattttt attttttatg tatattaggt tttgggtttg tgttgggtgt atagattagg 240
aagtggggtg tgtatggaaa tttatttttg gggtgatcgg ttatatattg ggtggggaga 300
ttttatttga tttgagacgt aggttgtgtt tttatttgtg tttattagag tcgttgaaag 360
agaagaattt agtttagatt taggatatgt tattgtaggg gaagtagaga tgttttgggt 420
ttcggagaag gggtggttat atttgagttt tatttgtttt gggtgggttt tttttaattt 480
ttatttagtc gaaggttcgg gtcgtggggg tgttaggttt tgaagggtag ggtgtttttt 540

tgttttcgga gaggagatta gtataggtcg gtgtaaagtt cgcggtttgt tcgtttttat 600
aagggggatg ttgcggagga tatggtttcg gatcggtttt atttgtttcg tagttatcgg 660
ataagttttt tagttgggtt tcgtagttcg agggatgaaa atagttttcg tttttttttg 720
attgatttag agggtagtat tgagaaggtt cgtagttggg gttgtaggcg gtaggagtat 780
atttcgaggg cgcggagttg gggaagggta tttttagggt cgaaggcggt tgttgggcgg 840
gatagtcgga gttttgttga aacgtagggt tggatagttt tttatcgtag cggttttcgt 900
ttgtatagtt gttaatcgtt tgagtcgagt ttgggggaaa ggaggttggg gcgttgtggg 960
cgttgtaggc gttgggtttt cggttataga gtgggtattt taagaaggag tttattttat 1020
tatagtttat gcgttaaggt cgtaggaggg tcggttcgtt tgggggagat atttttttgt 1080
tttataattt ttcggtagta tgttatagcg ttggggttcg aatttatggt ttgattcgtt 1140
cgtttgggta agcgtttttt taggaagggg gtaggagtgg ggggtgaggg ggtatatgtg 1200
atttttttta ggttaatggg ttaagcgggt tagagtttgg tagttttagc gtttatttat 1260
tattttttaa gtattagtat gataaagata ttttaattat tcgtagttta tttatttgag 1320
agcgatatgg aagcgttaaa aatatttttt tttaaagatt tttggaaaga aggattagaa 1380
gggagagggg gaaaaagtta ggagaatatt taagattttt tttttttttt aatttttaag 1440
gtttttaata ggtttttggg ttattagatt tggataaatt taagttagtt gttttttatt 1500
ttttttattt tttagagaaa tttttttttt aagtttgtgg atttggaggt ggggaattta 1560
gtttttatat tttgtttaga atttttaaat tatgtttttt ttttttaatt tgttttaggg 1620
tggtgggtta ggatagtcgg gtgaggtatg ttattttggg ttaaatggat gattgtgtgg 1680
tggggaggtg gggggaaggtg gtgtaaaaat atttaagggt gtgggtaggg atttagaatt 1740
tagaggaaga atttatgaag ttaggggtgg gaaattggta ttttagggag taaggtagga 1800
ttatagaaat gtggagaaaa ggatgagaaa tgttttattt tttttttttt tttttttgta 1860
taaatgtttt ttttttattt atatatttat tttttttttt tagttttttt ttatattttt 1920
tttgttttaa tttatttttt tgttcgttta ttttttttta tatttatatt cgttttttat 1980
tttttatttt tttagatgta tttatttttt ttttagttat ttatagttgt agttggttta 2040
tttatttaga tttaaataat tttttatatt tttttagaga gttataatga gtatagttag 2100
gtgatgttta tagcggggtg aagaggaggg gaatttttta tttttttttt gaagtgttta 2160
aaattagtgg agatttgtat ttttttggtt tttttttatt tgtttttttt ttattatttt 2220
ttattttttt ttaaaaaaaa aaaattttta aattttaaat gtataaattt aatattagtt 2280
aaattttaaa gattttttta gattttagta atttttttag gattgataaa gggagtttgg 2340
ttagagagtg ggagaatcgg ggtggaggtt ttgaggtttt gaattttaag attttagagt 2400
gaggagtgga gtttcgtttt tttttgtttt gtgtggttgt tgcggaggga tataggatgt 2460
gtttttttta tttttttaga ttttaaattt agatgggatt agaagagaag tttttaggta 2520
gagtttattg attttttttt ttcggataat ttgagggtaa gagtttgggg taatttaata 2580
tttacgttta tttgtaattt gttagggtgt ttgtttggtc ggaggtttgg ggtcgaagtt 2640
agggtaagtt taggagggta agagtttagg gagtgatttt tataaataac ggagtttagg 2700
agaggaaagg gaagtaggtg ggataggttt aaggaggatt tttaaaattt tttaggtttt 2760
tatattttaa aaagggtagg gcgaatattt ttttttttgt aagggatttt aaagttgttt 2820
tttgtagttt gtgttttgtt tttttatttt tgttttttat ttttttttgt tttgtttggt 2880
tttatcgtag ttgttttttt tgttttttgt tttatattat tagtattata ttgttttttt 2940
tttttttaat aattgtgggt ttagtttaat tttgtttttt tgagggggag ggttttgttt 3000
tgtttttgtt ttttgagata gggttttatt ttgttattta gattggagtg cggtggtatg 3060
attttagttt attgtaattt ttgtttttta ggtttaagta atttttttat tttagttttt 3120
tgagtagttg ggattatagt taggtattat tgggtttggt taattttttt tgtattttta 3180
gtagagatag ggttttatta tgttggttag gttggtttcg aatttttggt tttaacgatt 3240
tgtttgtttc ggttttttaa agtgttagga ttgtaggttt gagttgttat atttggttat 3300
tatttttaat tttgaaagag tttaaagtat tttatagaat ttttgttttt tttttttgat 3360
tatgtgtatt ttgttttaga ttttgtttta agttgagggg gaggggtatt agttggtaat 3420
ttttttaata gatgtaatgg gggtgagaga gaatgtttat ttgttaaggg aatgagggta 3480
gataagagaa aggaaaattg agatggaggg aggtgatttg tagaggaatt gaagagaaag 3540
aaattattga ggtaagagta agtttatttt tttttatttt attttttcgg ggagaagatt 3600
tgtagatgtt ttattttttt attttaagat gttttttata gacgttaaga ataatttgtt 3660
tggaggaatt tagggtttgt tttttaattt taaatttgaa ttaggtttgt atttgatttg 3720
gtagttttgt ttttgagaga tttgagattt gtttattttt tatttttttt ttatttcggg 3780
gagaattttt ttattttatt ttttgttttt ataattaagg gaataatttg aggtttaaag 3840
gaagaagtga attttaaatt agtttaataa aatagtataa atttgggaat tgaggaatgt 3900
atttttttta taatagttga agttattaat aatttattat ttatgatagt aagtaaagac 3960
ggaggtaaaa gtgttttaag ttggttgggt gtagttttag tattttggaa ggttaaggta 4020
agaggattat ttgagtttag gatttcgaga ttagtttggg taagatggtg agattatatt 4080
tttataaaaa taaataaatt taaaaattag ttgggtgtgg tggtgttttt ttgaagtttt 4140
agttgtttag aagggaggat ggtttgagtt taggagttta aggttgtaat gagttatgat 4200
tatattattg tattttagtt tgggtgatag agaatttgtt tttatttaaa aaaaaaaaaa 4260
aaaagtgttt taagttatat tgttaattgt agtgattgtt ttagtttttt aaatttatta 4320
tgttttttaa tttatttttg agttatgtta tttttttttg tgttagtagg gatattaaaa 4380
tttgtttaaa tttttgttgg tttttttagt tttagtagaa aagattggga gaattaagta 4440
attttgtttt ttaggttttt aatttttttt ttttttttaa aagaagtttc gttgattgga 4500

ataatttatg gtttatagga tttttggtgg attaaattaa atttattaga gagggtttgg 4560
gtggatttaa tattaaaatg aaataaagtt gatgttttag tttgtatgat ttgtttgtat 4620
ataaaagaga gtaaggagaa tatagatatg tttggaattg aagttatttt tgatagtttt 4680
tattttttat tatattgaga atgtaggtaa ggaaattatt taattagaat ggggatatat 4740
tagaagttta attttagatt ttggtttttt ttgtgaagga agttgtttta gttgggggtt 4800
tggtaggagt aatttaaatt tttttattag attttaaatg tttttatttg ttttttttag 4860
tttaaattta gggaggttaa atttaagatt aaaggttggg gttaggttag tgaggtggtt 4920
tatgtttgta attttagtat tttgggaggt taaggagggt agattatttg aggtcgggag 4980
tttaagatta gtttgattaa tatggtgaaa ttttattttt attaaaaaat ataaaaatta 5040
gtcgggtgtg gtggtaggta tttataattt tagttattta agaggttgaa gtaggagaat 5100
cgtttgaatt cgggaggcgg aggttgtagt gagttgagat cgtattattg tattttagtt 5160
tgggcgatag agtaagattt aaaaaaaaaa aaaaaagttg gggtttagga agtaaagaat 5220
atttataatt tttttttttt tttttttttt tttttttagg tagggtttcg ttttgttgtt 5280
ttaggttaga gtgtaatggt gtaattacgg ttttttatag ttttgatttt ttgggtttaa 5340
gcgatttttt tattttagtt ttttgagtag gtaggattat aagtatgtat tattataagt 5400
tttttattat gagatgaatt gtttttgttg ttgttagaga aaggatattt tttttaatgt 5460
aagttaatta aattgttata aatttggttg agaattttag attgtatgta gtatatattg 5520
taatgggatt agtgttagtt atgtgggtta gaatagttgg gtttaagttt ttgtttggtt 5580
attttttttt ttttttttta atatagggtt ttattttgtt atttaggttg gagtgtagtg 5640
gcgtaatttc ggttcgttgt aatttttgtt ttttaggttt aagtaatttt tttattttaa 5700
ttttttaagt agttgggatt ataggtgtgt gttaatatat ttagttaatt ttttgtattt 5760
ttggtagaga tggggtttta ttatgtggta taggttggtt tcgaattttt aagtttgggt 5820
aatttattcg ttttagtttt ttaaagtgtg ggattatagg cgggagttat tgtgtttggt 5880
ttttgtttgg ttatttttta gtattgtggt tttggataag ttttttgatt tttattagtt 5940
ttagtttcgg agaaaaagaa tgggtttaat attatttatt ttgttgtttt gttagtagga 6000
ttaaagtaaa taagaaaata taaattatag ggaatt 6036

<210> 143
<211> 6233
<212> DNA
<213> Artificial Sequence

<220>
<223> chemically treated genomic DNA (Homo sapiens)

<400> 143

atggttaggt tattattaat taacggattt attttatatt tttgtaggta atttttttta 60
aattttgatt tttttttttt ttaattattt ttttaaaaat aagaaaatat attttaaatt 120
taaaaggtat aaaatacgtt tttttttaat ttttttaaaa ttttaaattt gtttttattt 180
gaggtttatt gaggtatatt tttagttttc ggtttttttg ttttagaata tttatattta 240
tatattttat ttttattaat tatagttttt agagggttta gggatggtga gagattttga 300
aagagttgtt tatattataa attatatata ttttttttta aggaaaagtg tggaggttag 360
ggtaggtagg ttgttaggat tgaaggtttg tttattttgt tgtttttatt ttagttttag 420
ttttattttt ttttttatag aaagtagttg gtgatacgag gttttatatt ttttttttgt 480
tgtttttttg atttaacgtg aaaatagggt atatttgaat aaattgtttg ttttaggtag 540
gggttgggta gggtttgtgt gttttgttta gttttttgat aggatatttt tgttgtattt 600
agaatttata ttttaatgga tgtaaaaata attgtgagag atgtttgggt ttgtagaagt 660
ttagtattgt ttaaaaaagc gtgtgtttta gtgaatattt ttatatatat ttattggtta 720
tagtttgtta aaatattttt ttttttgtat tatttatttt tttatattat gtatttatat 780
gagggaaaaa aaggaaaaaa ttgtattttt ttagtattta tttgttataa aggatattgt 840
gttttttgtt atgtaaaatt agttatttta gttattattg tattttagaa aagagttgta 900
gatttatgtt aaattcgtat ttacgaataa ttgtaattag ttttaaaagg tatgaattta 960
gtttttaatc gttattgtat agttttgaat ttgtagaatt agagttaatt tttttttgga 1020
attttttttg ttttttagta gttatttttt tttttattta aaagggttgt ttgttaaata 1080
atttttgaat aaattttttg ttattaattt tattttgttt tggtggttta atttagatag 1140
tagagtaagg ttgttgattg ttaaattttt tttaagggaa gaaggataag gataaggata 1200
ttgaagattt ttttttggtt tttttgttta ttgtttaggt ttttgttttt tttttgatat 1260
gttttaagtt taaatattat ttttttattt ataagagttt gtaatatttt gttaattttt 1320
ttagttgtgg tttaagtagt aagttgggag gtaacggggg atttttggat gttttttgta 1380
ggtttttttg tttggtttgt atgtttttag gtttttattt tattgatttt tcgtttgtta 1440
gtttttttgg ttatagcgtt tatgtatttt ttatttttta ttttttttta ttattattaa 1500
gaaaatttgg tcgattacgg ttttttttac gaaatttaaa ttttataatt ttttttcgga 1560
gttataaaga aggtgttacg tgattcgaag tttaattatt attgggttta aaatgaaaat 1620
aaagaaaaat gattaattta aaaaaaaaaa aaataaaatt gaggtttaga ttttttaggt 1680
taaattttaa aattaagtaa aattaaatta ttaaataaaa ttcgaggcgg agggagggga 1740

agtacgttcg attttttttg ttagagaaag gggagaggag ggtgggagaa gttgttggat 1800
ttaagcgtaa ataaaacggt ttttaggtta atgtgcggtt ttgtttttat tatttttggg 1860
aaaattttat tttttagttg tggttttata aaagttttat tatattggag tgaaaataga 1920
gaaggtttgt taaagatgaa atgttatcgt cgagtcgttg gagtcgataa gagcgagaag 1980
atagggcggt tgaaatatgg agttaattcg ttgaaagaga agcgatggtt gtttgttgaa 2040
gagttgtata attttaattg taagcgatgt gttcgtattg tttaattagg agtatatatt 2100
tggtggtagt gatgttgggg gagtgagaat cgttttaggt tttgttttaa agagagggtt 2160
gatttttatt tttatgttgt attttggtag tttaaggtta gtaattatag tttttatgta 2220
ataataaata ataataaaaa agtaatttga attttacgtt tttttttttt tttattatta 2280
agtaggattt taattggatt tatttaagta gtaagtaatt tagtattggg ttaggttata 2340
atttaaaggt ataagagcgt gtaaagtttg attgggatta aataacgttt aagggttttt 2400
tttttttttt tttttttttt tttttttgaa ataatatttt ttgaatgata taattagatg 2460
aagtattatg tttggttttg gggttgggtt gggttgttat ttatttagta ggaaagaggt 2520
taagaaagag aatttagttt aagtttgaaa aatagagttt tggttcgaag ttggaattgt 2580
aaataagaaa aataagaatt aatttggttt ggtgtttttt attttttttt ttggaagatt 2640
ttaggcgttt agttaaagta gttaaagacg tgggtaaaat attcgtgttg ttaaatttat 2700
agtaggatag gggggcgggg gagggatgtt tttgaaaagg agggtttttt gggttttttt 2760
taatatttaa gtttttcgtt gtttgttaat ttggttataa aatttgttag tgagggttag 2820
agagagttgt attttttgta gggatagttt gttcgagttg tttgagataa gttttggatt 2880
ttttagttag ttatagaagt tgttgttaaa tttttagagg tgtttaaggg ggtggttaga 2940
gggagaattt atttttaggt ttttttgtat gatttttaga tagttattat ttggattatt 3000
ttatgtttat ttttatttat atttttaaat ggtaaaaatt gttttttttg gagtttagtt 3060
gtatattatt gtcgttggtt attttgttta ggttagagta gttttttttg ttttgagttt 3120
tttagagtat gttttttcgg tttttgtagt ttttattttt aatttttttt tttttatttt 3180
tttttttttt atttttatgt ttttatttag atttttttgt tttttttttt aagtatttac 3240
gtagatgtta tattgataga aagtttttag ttttagtttc gggtttagtt gttatttttt 3300
tgttaattag tttttttttt ttggttgggt agtagagaga gaggaggggg aggggaggtt 3360
aaagttatgg gagtttttta ggtaaatttt ttatttaatt tagcgttttt tttttgtttt 3420
tattttttat ttgtttttta gtttttttag aattattttt tttttttgta tttttatttt 3480
tatttttagg tttttttaag ggatgtgaaa ttttggttgt tcgttttgtt tttttaaaga 3540
gttaaatttt ttgatgtaat cggagggagt tgttaggggg ttaagattga tcgttttatt 3600
ttttttttat tcgtttgatt tatttattta aaaatttttt tttagatcga ttttttattt 3660
tttgttttga ggtttagttt tttattaatt gtttggggtt tagagttttg tgtttttttt 3720
ttatagtaaa ttataatatt ttaaatttta gattattttg gtttttttaa aataaaataa 3780
atttaaaaaa tatttttttt tagtagttag aaatggaata ttattttagt ggttattatt 3840
tttattttta ttatttttag ggaatttttt tttgttaggg attttttttt tttttttttt 3900
tttttttttt ttttaattag tataatggta aaaatgggag ggaaagtttt tttgttttag 3960
gggaaaattt ttttattata tttatgtata tatataacgt tgatttgaat tttgagagtg 4020
ttttaagtta aattaatggt tattttagtt tttgtttatg tttttggata tggatataga 4080
tataagtttg aggtttgagt gggtatatgt atattttaga ttttgtttgt ttttggggga 4140
tgttatagag aagtatggaa gtttgaaagt gaggaaagta ggggtatata aggggtagag 4200
gtagtgttgt aaatattggg tagagaagag aatttagatt atatagcgat ggaggttaga 4260
ggtttaaaaa tgttttttat gttttatttt atttttatgt gtttgtttgg gagtttttgt 4320
ttatttttta agatttaatt gttgtttttg gagggtaata gggatagtgg tgtgatgatt 4380
gtaaattttt tttttatttt tattttaata ttgatttttt ttaaagagtg gaagtttttt 4440
tcgatttttt ttttttttaa cgcggagatt tttatttatt tttttttggg gaaggggaga 4500
agggaagggg atgttgttgg ttacgtaaag aaggttttaa tttattttat taaaatattt 4560
ggaaaagagg aaggtttttt ttttagagtt tttttttttt taggtttttg aaatttttta 4620
attttcgaag gggatgagga aaaggttttt ttagtttttt tggagaaacg gaggtattgg 4680
ggagagagta tgtgaggtat tggtgtagga atgggggttt atggtgtttt ggtcgtggtt 4740
ttttggtgtt tttgaattaa ttaaattaaa taatgttttt tgttttttat taggtttaga 4800
cgagcgattg gcggaggtcg gtttcgtgat tacgaatttt ttgtaatttc gttggagttt 4860
tgggtttaat agagagagtt tttatacgtt tgtatttatt agtaatatat aattataaag 4920
gtttaaaatt aaaaaaaaga gagatcgaaa tttttttttt ttaaaatcgt tttattatat 4980
aaatcggggg gggtgtagga gggggggttt ttttcgattt ttttttttga cgtttttttt 5040
agtagttttt ttttttatta ttgaaagtta tgaattttga atttgagagg gagattgggt 5100
ttataaatag ttagtcgtcg ttcgtcgagt gtttgatttt ttttttcgtt gttttggaga 5160
tattttaaat tttattaatt aaggagtcga tattaatttt tttttttttt tttttcgagt 5220
aaattttttt tagtttttag ttcggcgttt ttatttttta gagatttagg agttaaaagc 5280
gagtcgaaga tgggtttgtt ttgtcgtcgt tatcgtcgtc gttatttttc gttgtttttt 5340
cggttttcga gtttttttgg atgaaagaga agaaattcgt taagaaattt agttaattcg 5400
ttacgttttt tttttcggtc gttttcgtcg tttcggtttt cggggtcgga tcgtttgtag 5460
gtcggtaagc gtggtggggg tggggtgagg ggtagttaga agagggggag aaaaagtagt 5520
ggaaggaggt ttaaaaaaaa tggtggcgag tttgttgggg ttttttattt tttttatttt 5580
tgggatgggg aagtagaata ttttattggg gtttaggttt ggtcgttaat agcgtttttt 5640
ttttttttgt tttttttttt tttttttttt ttatggttga ttttggaagt tggttgggga 5700

aacgtttttt tttttgttag aagaaaatga tttgattgag gtttttattc gtatttgtgt 5760
aggggaatga gtatgttgtt gttgggatag attttagaag tagaaaagat tcggtttttt 5820
ttgttttaat tttaggagaa gaggaagaat ttagagttag ggaggaagag gggtgaagaa 5880
taattttttt gattttcgtt tcggttgagt tggaaagttt cgaattgagg ggtttattgg 5940
gagggtttgg aagggggatt tgaggtttcg gcgtcgaagg gagtcgtttt tcgttttttg 6000
cgtggttcgt gggcggtttt gaatgttttg ttttgattcg ggtcggggtt gtagattaga 6060
gagggagaga agaaaattta aaagtagtaa agcgagcggt tttggggtgg gggcgggggt 6120
ggggtggcga ggaaggggag aggtgatatt ggttttggag gatggggtag ttcgaattga 6180
ggttgggttg agtatacggt tcgttatgac gtcgagtttg ttttttttgt aga 6233

<210> 144
<211> 6233
<212> DNA
<213> Artificial Sequence

<220>
<223> chemically treated genomic DNA (Homo sapiens)

<400> 144

tttgtaggga aaataggttc ggcgttatag cgggtcgtgt atttagttta attttagttc 60
ggattatttt attttttaaa attagtatta tttttttttt ttttcgttat tttattttcg 120
tttttatttt aaagtcgttc gttttattgt tttgggtttt tttttttttt tttttagtt 180
tatagtttcg gttcgggtta gggtaaagta tttagagtcg tttacgggtt acgtaggggg 240
cggagagcgg tttttttcgg cgtcggaatt ttagattttt tttttaagtt tttttagtgg 300
attttttaat tcggaatttt ttaatttaat cggagcgagg gttagaaaga ttgtttttta 360
tttttttttt tttttagttt tgaatttttt tttttttttt agagttgggg tagaaaggat 420
cgaatttttt ttgtttttag ggtttatttt agtaataata tatttatttt tttatataga 480
tacgggtgaa aattttaatt aaattatttt tttttagtag aaaaaaaggc gtttttttaa 540
ttagttttta aaattagtta tagggagaga aagagagaga gagatagaaa aaaaaaagac 600
gttgttagcg gttaggtttg aattttagtg ggatatttta tttttttatt ttaggaatgg 660
agggggtaag gaattttaat aggttcgtta ttattttttt taaatttttt tttattgttt 720
tttttttttt ttttttagtt gttttttatt ttatttttat tacgtttatc gatttgtagg 780
cgattcgatt tcggaggtcg gaacggcgga ggcggtcgga gaaggagacg tggcggattg 840
gttgggtttt ttggcggatt tttttttttt tatttaaggg aattcggggg tcgggggggt 900
agcggggagt ggcggcggcg gtggcggcgg tagagtaggt ttattttcgg ttcgtttttg 960
gtttttgggt ttttgaaggg tggaggcgtc gggttggagg ttggggaagg tttgttcgaa 1020
aggaggagga ggaggaatta atgtcgattt tttgattgat gaagtttgaa atgtttttaa 1080
gatagcgggg aaggaagtta gatattcggc gagcgacggt tggttgttta taaatttaat 1140
ttttttttta aatttaaaat ttatggtttt taatggtggg ggagggggtt gttggggggg 1200
gcgttaggag ggaggatcgg aagggatttt tttttttgta tttttttcga tttatgtaat 1260
ggagcgattt tgggaggggg agatttcggt tttttttttt tttaattttg ggtttttata 1320
attgtatatt gttgataaat ataagcgtat ggggattttt tttattaaat ttaggatttt 1380
agcgaaatta tagggaattc gtggttacgg gatcggtttt cgttaatcgt tcgtttgggt 1440
ttggtggaaa atagagagta ttatttggtt taattgattt aaaaatatta gaggattacg 1500
attaggatat tatgagtttt tatttttata ttagtgtttt atatgttttt tttttaatgt 1560
tttcgttttt ttagaaaagt tagaggaatt ttttttttat ttttttcgaa ggttagaagg 1620
ttttagaagt ttggaaaaga agagatttta agggagaaat tttttttttt tttaagtatt 1680
ttaatagagt aaattaaggt tttttttacg tggttagtag tatttttttt tttttttttt 1740
ttttttagga aaagatgagt gaggattttc gcgttggagg aagagggagt cgaaagaggt 1800
ttttattttt tgaaaaaggt tagtattaag gtgaaaataa gaaagagatt tataattatt 1860
atattattat ttttgttatt ttttaaaaat aatagttaaa ttttgggaaa tggataggaa 1920
tttttaggta gatatatagg agtgagatgg agtataaggg gtatttttgg gtttttggtt 1980
tttatcgttg tatggtttgg gttttttttt ttgtttagtg tttgtagtat tgtttttgtt 2040
ttttatgtat ttttgttttt tttattttta ggtttttatg tttttttgtg atatttttta 2100
aaaataggta gagtttaaga tgtgtatgta tttatttaag ttttaagttt gtatttgtat 2160
ttatatttag ggatatgaat aagaattgag gtagttattg gtttaattta gagtattttt 2220
aaagtttaga ttagcgttat gtgtgtgtat gagtgtggtg gggagatttt tttttaggat 2280
aaagagattt ttttttttat ttttgttatt gtgttagtta aaaaagaaaa aagaaaaaaa 2340
aaaaaaaaag aatttttgat aaggaagaat tttttgaaga tggtaggggt ggaaataatg 2400
attattgaga tgatatttta tttttggttg ttgggggaaa atgtttttaa aatttatttt 2460
attttgagga gattaagata gtttgaagtt tagagtgttg taatttatta tggggagagg 2520
gtataagatt ttgagtttta agtaattggt gggaaattag gttttaaagt agaaaatgaa 2580
aagtcgattt gggggaagat ttttaaataa gtgggttaga cggatggaga ggagatgagg 2640
cgattaattt tagttttttg atagtttttt tcgattgtat taaagaattt ggttttttgg 2700
gaaagtagag cgaatagtta aaattttata ttttttaaaa aggtttgggg gtgggggtgg 2760

gggtgtaaaa gagaagggtg attttgggag agttgggaaa tagatgggaa atggggatag 2820
agggaaagcg ttaagttggg tggaaagttt gtttggagaa tttttatggt tttggttttt 2880
tttttttttt tttttttttt gttgtttagt taggaggaga aggttggtta gtagagaaat 2940
ggtaattgag ttcggagttg gaattagaaa ttttttgtta gtgtaatatt tgcgtgagta 3000
tttaaaaaga aggatagaga agtttgagta gaagtatgag ggtgggggag aggaggatgg 3060
agaaagggga gttggaggtg ggggttgtag ggatcgagga ggtatgtttt ggaggtttta 3120
gaatagagag agttgttttg atttgggtaa agtggttagc gatagtagtg tgtaattagg 3180
ttttaaagaa aataattttt gttatttaga ggtgtggatg ggggtgggta tagaataatt 3240
taaatagtag ttgtttggaa gttatgtagg gaggtttgag agtgggtttt ttttttggtt 3300
atttttttga atatttttga aagtttagta atagtttttg tagttggttg aaggatttag 3360
agtttatttt aggtaattcg ggtagattgt ttttgtagga aatatagttt tttttaattt 3420
ttattgatag attttatggt tagattagta aatagcggaa aatttgggtg ttaaaagggg 3480
tttagagaat tttttttttt agagatattt ttttttcgtt tttttgtttt gttgtgaatt 3540
taatagtacg gatattttat ttacgttttt ggttgtttta gttgagcgtt tggaattttt 3600
taaagagaag gatagggaat attaagttaa gttggttttt attttttttg tttgtaattt 3660
tagtttcgag ttaaagtttt gttttttaaa tttaagttaa attttttttt ttggtttttt 3720
ttttgttaga taaataataa tttaatttag ttttagagtt aaatatggtg ttttatttag 3780
ttatattatt tagaaaatgt tatttaagga agaagagaga aagagaaaga aagaaaaatt 3840
tttgagcgtt atttgatttt aattaaattt tgtacgtttt tatgttttta aattatagtt 3900
tgatttaatg ttaaattgtt tgttgtttgg atgagtttag ttaaaatttt atttggtggt 3960
ggggaagggg gagggcgtag agtttaggtt gtttttttat tgttatttat tgttgtataa 4020
agattataat tattaatttt ggattgttag gatgtaatat aaaaatgaaa attagttttt 4080
tttttaaggt agggtttgga acgattttta tttttttaat attattatta ttaaatatat 4140
gtttttaatt aagtaatgcg ggtatatcgt ttataattag aattatgtag ttttttagta 4200
agtagttatc gttttttttt taacgaatta gttttatatt ttagtcgttt tgttttttcg 4260
tttttatcgg ttttagcgat tcggcggtga tattttattt ttgatagatt ttttttattt 4320
ttattttaat gtgataaaat ttttatggaa ttataattaa gaaatgaagt ttttttaagg 4380
atgatggaaa tagagtcgta tattagtttg aggatcgttt tatttacgtt tggatttaat 4440
agtttttttt attttttttt tttttttttt tggtagggga aatcgaacgt gttttttttt 4500
ttttcgtttc gagttttatt tagtgattta attttattta gttttaaagt ttgatttgag 4560
aagtttaggt tttagttttg tttttttttt ttttagatta attatttttt tttgttttta 4620
ttttagattt aatggtggtt gggtttcggg ttacgtgata ttttttttgt ggtttcggaa 4680
gaggattgtg aagtttaggt ttcgtggaag aaatcgtaat cggttagatt tttttggtgg 4740
tggtgggaag ggtggggggg tggggggtgt atggacgttg tggttaaagg agttaataga 4800
cgaaaggtta atgaatgaaa aatttagagg tatataggtt aaatagaaaa atttatagag 4860
agtatttaag agtttttcgt tattttttaa tttgttattt aaattatagt tgaagaagtt 4920
ggtaaaatgt tataggtttt tatgagtgag aaaatggtat ttaaatttaa gatatattaa 4980
gggggagata ggaatttggg tagtggataa aaaggttagg gaagggtttt tagtgttttt 5040
gtttttgttt tttttttttt gggggaaatt tggtagttag tagttttgtt ttgttattta 5100
aattggatta ttaggataag ataaaattga taatagaaag tttatttaag aattgtttga 5160
tagataattt ttttaggtaa ggaaaaaagt aattattgaa gaataaagaa agttttaaga 5220
gggaattaat tttagtttta taaatttagg attatatagt gacgattagg agttgagttt 5280
atgtttttta gaattaatta taattgttcg taagtacgag tttaatataa atttatagtt 5340
tttttttaga gtataatagt aattaaaata gttggtttta tatgatagga aatataatgt 5400
tttttgtaat aggtaaatat taaaaaagta taattttttt tttttttttt tttatataaa 5460
tatataatgt agggggataa ataatataaa aaagaaaata ttttaatagg ttataattaa 5520
taaatatata tgaaaatgtt tattagaata tacgtttttt tgagtaatgt tggatttttg 5580
taggtttaga tattttttat agttgttttt atatttatta aaatgtaaat tttaagtgta 5640
ataaaagtgt tttgttagga ggttgagtaa ggtatatagg ttttgtttag tttttgtttg 5700
ggatagatag tttgtttaaa tatattttgt ttttacgtta agttaagaga gtaatagaag 5760
aaaagtatag aatttcgtgt tattaattgt tttttgtgga gagggggatg gagttggagt 5820
tgagatggag gtagtagaat gggtaaattt ttagttttaa taatttgttt gttttagttt 5880
ttatattttt ttttaaaaaa aaatgtatat aatttataat ataggtagtt tttttaggat 5940
tttttattat ttttgagttt tttaaaaatt gtaattgatg ggggtgggat gtatagatat 6000
gaatgtttta gagtagaaaa atcgggggtt agaagtgtat tttaataaat tttaaatggg 6060
aataaatttg aggttttggg aaagttggaa gggaacgtgt tttgtgtttt ttgggtttga 6120
aatgtgtttt tttatttttt aaaggataat tgaggaaaaa aaaaattaag atttgggggg 6180
aattatttat aaagatgtaa ggtaagttcg ttggttggtg atggtttagt tat 6233

<210> 145
<211> 6116
<212> DNA
<213> Artificial Sequence

<220>
<223> chemically treated genomic DNA (Homo sapiens)

<400> 145

ttttattttt ttttatttag ttgtagagaa aagttttaag ttttgtgttt tggttttcgt 60
atgggttttt ttttcgagac ggagttttta attcgttagt aatttaattt tttatagaga 120
cgtagggtgt tagcgttttt tttcgtcgag gggaggagat tggggttttt ggaaggtata 180
gaatgttggg tagaggtgag gtagaatagg agggttatat cgggagtttt aattttttaa 240
gggatttagg gtttgcgttt tgtttagcgt tttaataggt tttttagtcg taatttgcgc 300
gcgttttttt tttcgttttt tttttgattt cggagagagc gaggtaagga gggggtcgtt 360
tcgtaggagt tttatgtaaa ttttggtgtt gggtgggtgg gtggggaggg ggaagggaag 420
aaggggaaat aaattttttt ggttggagtg gggttcgggt gagtagattt ttttattcgg 480
gaatcgtagg cggggcggtt attggttcgg aggattacgt gggcgtttaa ttttgtttat 540
ttgatagtaa gtaggagggt tttcggaaat aggaaaacga gttaggggtc ggaataaatt 600
ttagtatatt ttgtgggtaa tttttagaaa ttaatggtta tgagtttttt tttgattaat 660
ttaaattatg tcgattttaa gtttttttta tgcgaggaat atttatagag cgattattta 720
tttagcgatt attcgttcgg gtattacgtc ggcggttaga ggcgagagag tagtttttag 780
tcggaggcgg gtttcgggcg gcgcgcggcg tgtatcgtgt agcgttacgc ggtttgtcgg 840
gattttgggt tttcgtcgtt ttcgttatta ttttcgtcgt tttcgttatc gttcggtttg 900
ttttttcggg tttttgcgtc gttattcgtc ggggtttttt tttcggagtt cggttagcgt 960
tgcgaggcgg ttagtagtag tttttcgtcg tttttttgcg tttagaattt tttgtatttt 1020
agttcgtttt atttcgcgtg taaagagttc gtcgtttatt tttggatgcg taaagtttac 1080
gtgagtacgg gtgagtgcgt gggtattttt tttttttttt atttttggag ttttatatta 1140
aagggatttc ggaggtatgg gttggggggg ggttgagttg gggaagttaa ttgttttcgt 1200
tataaattcg ttattgtcgg agatttacgg tcgtttgttt ttagagttat ataattatat 1260
tttttataaa tttttatggt ttggtgggtt ttgcgttttt gaagtaggtt ttttattttt 1320
tttattattt ttattagcga ttttttcgtt agggagatat agttttagtg aagttggaag 1380
ttggggaggg gtggaggagg tggaggagga gaaggaggag gaggtaattc gtatttaagt 1440
agagagttga gttaattttt agtatttatt tttttttttt ttttagtttg attttttggt 1500
tggagaagtg agtaaaagtt tttagtaggg gtaggtattg tttggaattt gagtagtaag 1560
ggtgggaatt aaatagagga aaggtttagg gaaatttttt tttttttttt tattttttta 1620
tttttttatt ttgggggttt taagttgagg gggagagagg agtaattaaa atttgggggg 1680
agtttttata cgtttttttt ttttattata tatttaaata aggatatagt atagtttttt 1740
aaaatttgga ggtttttttt ttatttttgg gttttgtatt tttttttttt cgtttttttt 1800
tttttttttt agaaggaaga agaggtggcg ggagagggag ggagggaaac gttgtgtttt 1860
ttggaagaaa ggattttttt tttcgggggt aataaaattt tttttttttt ttttttttcg 1920
tgtgtgttta gtaaatttta attacgtcgg cggggagttt aagcgttttc ggatcgttta 1980
tacgcgttag taggttttgg agttggagaa ggaattttat tataatcgtt atttgatacg 2040
gcgtcggagg gtggagatcg tttacgcgtt ttgttttttc gagcgttaga ttaagatttg 2100
gttttagaat cggcgtatga agtggaaaaa agattataag ttgtttaata ttaagattcg 2160
ttcgggtggt gcggtaggtt tagtcggagg gttttttggt cggtttaatg gaggttttcg 2220
cgcgttttag tgttttcgta cgcgggagtt acgaatttcg gggtgggggt gggtagtgag 2280
tgtaggggat ggggtggggg gataggaggg ggttttgggg tttgggtttc ggaaaaattt 2340
atttgttttt ttttatattt tatatacgaa taaacgtaga agagggggag gggaagtttt 2400
atttatagaa atgataatag agggttacgg ggaggttttt tttagaagta agatttaaat 2460
tttttgtttt tttttttaaa aaaaagaaaa agaaaaagta agaagaagga agaaagaaaa 2520
agatagaaag agaaatagga ggaggttgta gtttttcgtt tttagttttg gcgaagatgg 2580
atttacgttt tatttttaat tacgttaggt ttaggtttat ttgttttgtt ttttttgtcg 2640
aggagaagac gggtttcggt ggcgattatt atttcgatat tcgttaataa atgaggttcg 2700
gttcggtcgt tttcgttttt gttattgtcg ttgttggaag atagtttgga tttttttttt 2760
ttgtttttta ttttcgatat ttagcgaaag tattttttga ttgttagata gtgtagtgtt 2820
ttggttacgg taatatatat atattttttt ttatttttcg tgtttattta ttgagggtta 2880
gaatgattgt ttatttattt ttatcgtggg gttgggggtg ggtaatagag gaggggagta 2940
agtagggaag gggtggtttt tgataattta ggagtgagta gggaaattga gtttaaggaa 3000
aaagagagat ttagagattc gggaggtttt ttttttgaaa ggttaagtta agttatgttt 3060
ggtagggtga ggggttagtt gagttttggg agttgggtat tattttgtta gtttagagtt 3120
gtatagtaga agtttttttt ttagattgaa aatgaatgtg aaattaggaa ataaaatgtg 3180
ttttttttag tttgggagga ggatgttgta gagttttttt ttatagttta ttatgttgta 3240
tcgtttatta ttattattga taatattatt attattattt ttttgtgtta tgtgagtttt 3300
tttttttttt ttttttttga tattttaaaa ttaggttttt ttttattttt ggggttgttt 3360
gagtttagaa tttttcgtat gtgtgaatat ttgtgtgttg tatagagtga taatagaaat 3420
aaatgtttgg ttttttgtga ttagtatagc gtgtttttgt tgaaatattg gcgtaaagat 3480
atggataaat gttatttagt gtatatttta agtattttat tgggtttagt tttatattgt 3540
gtgtggttga agattttttt taaagagttt gatgggaatt tttattttag aggattttaa 3600
attgtggtgg ttggtttgtt ttttttttag ggtgggtgtg ttttgttttg gggtttttaa 3660
tttttttttt ttagtttaaa aatggattga aaggattggg aaggttgatt ttagttttta 3720
atgggggaaa atgttttatt gtaggatata agaggtttaa gtacggtaaa gttttttata 3780

tcgtgattta tttgttataa gtaaataaaa tgcggttaag ttgttttttt ttagatagaa 3840
ggttttttgg ttattaaagt ttcgagagag gattttatga ggttggtttt ttttttcgta 3900
gtataagaag gtatcgtttt tttttgaggt gtttaaggtt ttaatgagta taattttttg 3960
tatagatgtt tttgggtgtt tgtaggtttt tagagtattt ttatattata aagaagttag 4020
ttagtgttat agtttaaatt ttgataagta aatagataat taagaagata aatggttttt 4080
tttgtgaagt tttgttttag tattaatttt tatttttttt tttatagaaa gtatcgaaag 4140
tatgattggg gattaaatag tttttgtttt ttgaggtttt agttattttt agaatggagt 4200
gggggtaggg gaaaggggtg taggtgattt ttagaagttt tgttaggttt agattgtttt 4260
tgtgtagtat tgtttggaag ttttaaggtt tgggggtttt agttttaggg tttttggttt 4320
attggttatt tagggtgtag ttattatatt tttaaaaagt tcggggttcg ggagagtata 4380
tgtaattaaa agtcgttttt ttagttttat ttttttttag aaaataaaaa gaattaattt 4440
tattcgcgcg ttttattgtt atttattttg tattttttta gtttgtagtt tcggtagttg 4500
gaggttgggt agggtagggg gaatcgatag gtcggtgttt ttagtcgtaa aagagttgtt 4560
gaattgttcg tttaaatgtt gttgggagat tcgtaaaaaa attatcgtgg atttggagga 4620
tgagaggggc gagttttatt tcggtcggat tgcggtgtgg tggtttagtt gtaaggggat 4680
gtcgtagttt tagttgaggg ggaaaatagt ttttaaaaag tatatgtttt tttaaggaat 4740
gtttttaaag aattaaatta aagttgtttt ttggaaggta tgaatagaat ttaaaaaaaa 4800
aagattttta tggagtttaa agtttatagt tattttgtgt agataagagt taagaaaaat 4860
gtgagaatta tatagaaaat tattaattat tttttttttt taaatacgta tttttttttt 4920
tttgttatta tttaatagta aatttttttg gatcggttgt tgggggaaaa aagtgttagt 4980
cgtttttttc ggatttgtaa gggggaaaaa atttggaatt ataaagttga aaattttttt 5040
tttttagttt ggaagaagtt tttcgttatg aatggatttt gtagagttcg ggcgagagga 5100
ggcgagaggc gtaaaggtaa taaagttcgg gttcgggttg gcgtgttttg atcgttttat 5160
gagttttttt tataattgaa agaagaaata ttttttcgtt tttttttttt ttttttttaa 5220
tttttttggt tgggttttta tggtggatgt ttcggtaggt ttgttttttt ttatgatgat 5280
tgatgtttgg gttagaggga gagaaagagg ggtttcgata tttggagagt gtttgaattg 5340
gttgggattg aggtgttagg gtttatgaaa aattttagag aagacggttg ttttgtgagg 5400
tagtttagaa gcgttttttt ggttttggaa aatgtaggta ggtatagcga gttttagtat 5460
gtgtgtatgg gtgtgtttag tgaggtttat atgtggtcgg tgtgtttttg tgattttgtt 5520
tgtttatatt gggtattggt gttttttatg tttgtttatt agtgagacgt tgttattgtt 5580
gttttttgga aaaattaaat ttagtgtttg gatggaatgt tttagttttg gagcgggtat 5640
aagtggatat gttttttttt ttttttttgt gtttagtttg ggattttaga taaatatgtg 5700
ttattttttg gagtaggaaa ttgtttagtt tatagataag gtggattgat gtagaaatat 5760
tttttttttg ttttgttttt ttagtttttt ttttggagga gtaatgttgg tttggtttgg 5820
tttggttttt ttttgttttt ttaatttgag gtttttgttt ttttttttgt agttttgttt 5880
tttaggttga gggagttagt gggttttgta gtcgatttga atgtgttggt tttttttttt 5940
tgaggtttta tttagggttt ggaggcggga ggttggtagg agagagagtt tatggagagg 6000
ttacgttttt taggcgatta gtttagtatt agaattataa aatgatttcg gaagtttttt 6060
gatttcgttg agattggata ttgtgttggt atttgttttt tgtttaggag ggagag 6116

<210> 146
<211> 6116
<212> DNA
<213> Artificial Sequence

<220>
<223> chemically treated genomic DNA (Homo sapiens)

<400> 146

tttttttttt tgggtaggag gtaaatgtta atataatgtt tagttttagc gaggttaagg 60
ggttttcgag gttattttgt ggttttaata ttgagttaat cgtttagaaa acgtggtttt 120
tttatgaatt tttttttttg ttagtttttc gtttttaaat tttaagtgag gttttaggga 180
aaaagggtta atatatttag gtcggttgta gagtttattg atttttttag tttaagaaat 240
agggttgtag aggagagaat agagatttta aattaggaga gtaaagggaa attaaattaa 300
attaaattaa tattgttttt ttaggaagaa ggttgggggg atagagtaga agagaaatat 360
ttttgtatta gtttattttg tttgtgaatt gagtagtttt ttgttttaga gagtagtata 420
tatttgttta gaattttaag ttagatataa gaagaaaaaa aagaatatat ttatttatat 480
tcgttttaaa gttggaatat tttatttaag tattggattt gattttttta agaggtagta 540
gtgataacgt tttattggta aataggtata ggggatatta gtatttagtg tagataagta 600
gagttatagg aatatatcga ttatatgtgg attttattag atatatttat gtatatatgt 660
tggggttcgt tgtgtttgtt tgtatttttt aaaattagaa aagcgttttt gaattgtttt 720
ataaaataat cgtttttttt ggggtttttt atgggttttg atattttagt tttaattagt 780
ttaaatattt tttaggtgtc gaggtttttt tttttttttt tttgatttaa gtattagtta 840
ttataaaagg gagtaaattt gtcgaagtat ttattataaa aatttagtta agaaagttaa 900
aaaagaaaaa ggaagaggcg agagagtgtt ttttttttta gttgtgaaaa gagtttataa 960

aacgattaaa atacgttagt tcgggttcgg gttttgttat ttttgcgttt ttcgtttttt 1020
ttcgttcgaa ttttgtagat tttatttatg acgaagggtt ttttttaaat tgagagaaaa 1080
aagtttttaa ttttatggtt ttaaattttt ttttttttgt agattcggga gagacggtta 1140
atattttttt tttttaatag tcggtttaag gagatttgtt gttgaataat aataaaggag 1200
agaggatacg tatttaaaga aaagaagtga ttaatggttt tttgtataat ttttatattt 1260
tttttagttt ttgtttatat agaatggttg tgaattttaa gttttataga aatttttttt 1320
ttttaaattt tatttatatt ttttaaagag tagttttgat ttggtttttt agagatattt 1380
tttagggggg tatatgtttt ttaagaatta tttttttttt taattggggt tgcggtattt 1440
ttttgtagtt aaattattat atcgtaattc gatcgaaata aagttcgttt tttttatttt 1500
ttaggtttac gatgattttt ttacgagttt tttagtagta tttaaacgga tagtttagta 1560
gtttttttgc ggttggggat atcggtttgt cggttttttt tgttttattt agtttttaat 1620
tatcggagtt gtaagttgga ggaatgtaaa ataaataata ataaaacgcg cgggtgggat 1680
tggttttttt tattttttga aaggagatgg gattgaaaaa acgattttta attgtatatg 1740
ttttttcgag tttcgagttt tttggagatg tggtggttgt attttggatg attaatagat 1800
tagagatttt gaggttggag tttttagatt ttagaatttt taagtagtgt tgtataaaag 1860
taatttgagt ttgatagagt ttttgagggt tatttgtatt tttttttttt atttttattt 1920
tattttaggg gtgattggga ttttagagat agaaagttat ttggttttta gttatatttt 1980
cgatattttt tatgagaaag aaaatgaaaa ttaatattgg agtaaggttt tataaaagag 2040
gttatttgtt tttttgatta tttatttgtt tgttagagtt tgagttatgg tattggttag 2100
tttttttgta atataaaaat attttgaaaa tttgtaaata tttagaaata tttgtgtaaa 2160
aaattatatt tattaaagtt ttaaatattt tagaggaaag cgatattttt ttatgttgcg 2220
aggaaagagg ttagttttat ggaatttttt ttcggaattt taataattaa agagtttttt 2280
gtttaaaagg agatagttta atcgtatttt atttgtttat gataaataaa ttacgatatg 2340
aaaggttttg tcgtatttaa gttttttgtg ttttataatg aaatattttt ttttattagg 2400
gattggaatt agttttttta gtttttttaa tttatttttg gattgaaggg aaaaaattaa 2460
aagttttaaa ataaaatata tttattttaa aaggaaaata aattagttat tataatttgg 2520
agttttttga gataaaaatt tttattagat tttttgaaaa aaatttttaa ttatatataa 2580
tgtggggttg aatttagtag ggtgtttaaa atatatattg ggtggtattt gtttatattt 2640
ttacgttaat gttttaataa aaatacgttg tgttggttat aagaaattaa atatttattt 2700
ttattgttat tttgtatagt atatagatat ttatatatac gaagggtttt agatttaagt 2760
agttttagag gtaggaaggg gtttggtttt ggaatgttag aaagagaaaa ggagagagga 2820
tttatatgat ataaaaaaat agtaataata atattattaa taataataat aaacgatgta 2880
atataataaa ttatggagaa gggttttgta atattttttt tttagattgg gagggtata 2940
ttttattttt tagttttata tttattttta gtttaggaga gaggtttttg ttgtataatt 3000
ttggattggt agagtagtgt ttagttttta gaatttaatt ggttttttat tttgttaagt 3060
atggtttggt ttggtttttt agaggaaggt tttttcgggt ttttgagttt tttttttttt 3120
ttggatttaa tttttttgtt tatttttgag ttgttaaggt tatttttttt tttatttgtt 3180
tttttttttt gttgtttatt tttaatttta cggtggaagt gggtgagtag ttattttggt 3240
ttttagtgaa tgggtacgaa agatgaggga gagtgtgtgt gtgttatcgt gattaaaata 3300
ttgtattatt tggtagttag agggtgtttt cgttgggtat cgggagtggg ggataaagaa 3360
aggaaattta ggttgttttt tagtagcggt agtagtagag gcggaggcgg tcgagtcggg 3420
ttttatttgt tagcgggtgt cgaggtaatg gtcgttatcg aggttcgttt ttttttcggt 3480
agaggaaata agatagatgg gtttggattt ggcgtgatta aagatgaaac gtggatttat 3540
tttcgttaaa gttgaaaacg aggagttgta gttttttttt attttttttt ttgttttttt 3600
tttttttttt tttttttgtt tttttttttt ttttttttta agaaagaaag taagagattt 3660
gaattttgtt tttggggggg gttttttcgt ggttttttat tgttattttt ataaataaag 3720
tttttttttt tttttttttg cgtttattcg tatataaagt gtgggggagg gtagatagat 3780
tttttcgggg tttaggtttt agggtttttt tttgtttttt tattttattt tttgtattta 3840
ttgtttattt ttatttcgag gttcgtggtt ttcgcgtgcg ggggtattag agcgcgcggg 3900
ggtttttatt gggtcggtta gggggttttt cggttgagtt tgtcgtatta ttcgagcgga 3960
ttttggtgtt gggtaatttg tggttttttt tttattttat gcgtcggttt tggaattaga 4020
ttttgatttg gcgttcggag aggtagagcg cgtgggcgat ttttattttt cggcgtcgtg 4080
ttaggtagcg gttgtagtga aatttttttt ttagttttaa gatttgttgg cgcgtgtagg 4140
cggttcgaga gcgtttgggt ttttcgtcgg cgtaattggg gtttattgga tatatacgga 4200
gagagggaga aagagaaagt tttattgttt tcgaaagaga gagttttttt ttttagggaa 4260
tatagcgttt tttttttttt ttttttcgtt attttttttt ttttttgagg gagaggggga 4320
aaaacgaaaa gggaagaatg taagatttaa gaatgagaaa gagattttta agttttggag 4380
ggttgtgttg tgtttttgtt tgggtgtgtg gtggggaagg ggggcgtgtg aggatttttt 4440
taaggtttta attgtttttt tttttttttt agtttggggt ttttagaatg ggagaatgag 4500
ggggtggaag agggagggga ggttttttta aatttttttt ttgtttagtt tttatttttg 4560
ttgtttagat tttaagtagt gtttattttt attgaaaatt tttgtttatt tttttagtta 4620
aggagttaga ttggaagaaa ggaaaaaata aatattggga gttgatttaa ttttttgttt 4680
aaatacggat tatttttttt tttttttttt tttttatttt ttttattttt ttttaatttt 4740
taattttatt aagattgtat ttttttggcg aaaaagtcgt tggtgagaat ggtgaagagg 4800
atggggaatt tattttaaag gcgtagggtt tattaggtta taaaaattta tgggggatgt 4860
aattatgtgg ttttgaaaat aggcgatcgt aaattttcgg taatggcgag tttatagcgg 4920

ggataattgg tttttttagt ttaatttttt tttaatttat gttttcgaag tttttttggt 4980
gtaaagtttt aggggtggga gggggaaggg gtgtttacgt atttattcgt gtttacgtga 5040
attttgcgta tttaggggta gacgacgggt tttttgtacg cggagtggga cgggttgggg 5100
tgtagggggt tttgggcgta gggaggcggc ggggggttgt tgttgatcgt ttcgtagcgt 5160
tggtcgggtt tcgggaggag ggtttcggcg ggtggcggcg taggagttcg aggggataga 5220
tcgggcggtg gcgggggcgg cgggggtggt ggcggaggcg gcgggggttt agggtttcgg 5280
taggtcgcgt agcgttgtac ggtgtacgtc gcgcgtcgtt cgaagttcgt tttcggttgg 5340
aagttgtttt ttcgtttttg gtcgtcggcg tagtattcgg gcgagtggtc gttgggtagg 5400
taatcgtttt gtgaatattt ttcgtatgga gggaatttgg ggtcgatata gtttgagttg 5460
attaaaaaag aatttatagt tattaatttt tgggaattgt ttataaaata tattaaaatt 5520
tatttcgatt tttgattcgt tttttgtttt tcgaaagttt ttttatttat tgttaagtga 5580
ataaagttag gcgtttacgt gatttttcga gttaatggtc gtttcgtttg cgattttcgg 5640
ataaggaaat ttgtttattc ggattttatt ttagttaaag aggtttattt tttttttttt 5700
tttttttttt ttttatttat ttatttaata ttaggattta tatagggttt ttgcggggcg 5760
attttttttt tgtttcgttt ttttcgggat tagagagaga gcgagagaga gagcgcgcgt 5820
aggttgcgat tggagggttt gttggggcgt taggtagagc gtaaatttta gattttttaa 5880
gaagttgggg ttttcggtgt agtttttttg ttttgtttta tttttgttta gtattttgta 5940
ttttttaaag gtttagtttt ttttttttcg gcgggaggga gcgttagtat tttgcgtttt 6000
tatggggagt taggttattg gcgggttggg ggtttcgttt cgaaagggga atttatacgg 6060
gggttagagt atagagtttg gggttttttt ttgtagttgg atggggaagg gtaggg 6116

<210> 147
<211> 7925
<212> DNA
<213> Artificial Sequence

<220>
<223> chemically treated genomic DNA (Homo sapiens)

<400> 147

cgttattttt atcgtgttgt agtcgggcgg cgggggcgat cgaagttgta gttttatagt 60
tgtagaggaa agaattcgta aaatgttaat agtttttatg cgttgcggta taaataataa 120
tagatttcgg ttttattgcg ttttatagtt ttttaaagag tttatagtcg ttttttgggg 180
ggtcggttat ttagttaatt ttttttatac gatagttaaa tttttattaa taataaggaa 240
attgattatt aaagttttaa atatgattat ttcgtttgtt tgaaaatacg tttaggagag 300
gaagttgtat gatttgataa tttgtattaa aatattaatt atatttatag gattttttaa 360
aattcggcgt aattaaatcg tgttttttta tattttttcg aagcgatttt gatatttaga 420
aagattttaa ttgtttcgtt aaaatcgcga aattaatagc gtgtttacgt ttggcgcgtt 480
gtgtatgtgt gtgtgcgtgt tttttttcgt gattttttta tattagtttt tgggggtggt 540
ttgcgaaggt atttgatttg ttgtgaggcg agagatattt tattaatgta ggtagttaaa 600
tagatttaat tcgtatttat ttttttttat tttttttttt tttttttttt tttttttttt 660
tttttttttt tttttttttg ggtgtttttt tttttttttt tttttttttt tttcgttttt 720
tttttatttt ttggcgtttt ttcgttttag tttttttttt cgttcgtttt ttttcgtttt 780
tattttcgtt ttgcgttttg ttcgggagga gtacgaagaa gttaatagga tgcggggttt 840
ttaatggatg taaatgatta tgaaaagata gtgtaaaata tgaaataatt tatttgtagg 900
gaagtaaatt atcgaaaatt gtttatgaat tggtattttt ttttcgaaat gtaaagcgag 960
gattttttta agtggtggta tatttttgtt tggggggtgg ggggtggggg gagggcgagc 1020
gagtcgcggt tgttatgtaa gtttagattt ttggaggttt ttttgttttt ttttattttt 1080
ggaaattata aaaagtgtgt cggtttcgag atttttttcg tttttttttt tttttttttt 1140
tttttttttt tttttttttt tttttttttt ggcgagggtg attaggagtc ggcgaattcg 1200
cgtttttttt tttttttttt tttttttttt tttttttatt ttttttttaa tagtttttaa 1260
ttatagtttt cgtcgtcgtc gtcgttttaa aatttaataa aatgagtttt tatttcgtta 1320
atttattgtt ttttaaatat aaaatcgggg agtttttgcg ttttaattat tatgattgcg 1380
gtttcgttta ggatttgggc ggtcgattta tcgtggtgta cggttttagt agcggcggta 1440
gtttttagta ttcgtcgtaa atttaggagt tttattacgg gtcgtcgtcg ttgtttacgg 1500
ttttttatta gtagaattcg tgcgtcgtgg cgtgttacgg ggatttcggt aatttttacg 1560
gttacgattc gttgtaacgt tagagtttat tcggtgcgta ggatttagat ttggtgtagt 1620
acgtagattg taagtttgtc gtcgttagcg gtttgggcga ggaggtcgag ggtttcgagt 1680
agagttcgtc gtttatatag tttttttttt ggatgcgttc gtaaggtgag ttcgtttcgg 1740
ggtcgataag ggtaggaggg ggtcggaagg gtttaaggtc gggtaggggg tttggtaggt 1800
cggggcgtat tttttttagt ttttggaaga tttgtcggtt tcgtttcgat cgtttgtcgt 1860
tttttttttt ttttttttat tttttttttt tttttttttt tttttttttt tgaagggggt 1920
cgtttagaaa tattttttga atttataaat tttttatttt gttttatttt tttttttttt 1980
tttttttttt tttttttttt ttattgtttt atggggggta gtgaagagag ggtggtggga 2040
gtgggggcgt ttaatttttg tattttttaa ttgttttata gtttaattat tttgaagaaa 2100

tttttttttt ggggtagagg ttttgggggt tttttaaggt gggtttattc gtttttattc 2160
ggttttttta tttttatttt tttttttttt ttatattttt ttttttaaag tttatggtat 2220
ttttaataga tttgaattat tttttatata attttggtgt tggttatcgg gggagggggt 2280
tttttttttt ggttttttta gatttatttg tgtttaattt ttttcgtttt tttttcgtcg 2340
tcgatcgcgg tttttttgcg tttttttcgt tttcgtacgg ggtagaaggt tttttgtttt 2400
tattataatg gttttttagg ttcgtttacg aattttttaa tttttttcgt ttttgttttt 2460
ttttttattt tttttttttt tttgtttcgt tttttttttt atttttagta gtcgtcggac 2520
gtaggcgagg tcgatagatt tatagtcgtt attagatttt ggagttggag aaggagtttt 2580
tatttaattt ttatttgatt cgtaagcggc gaatcgaggt atcgtacgtt ttgggattga 2640
tagagagata ggttaaaatt tggttttaga atcggaggat gaagtggaaa aaagagaata 2700
ataaagataa gttttttagt agtaaatgcg agtaggagga gttggagaaa tagaagttgg 2760
agcgggtttt agaggcggcg gacgagggcg acgcgtagaa gggcgataag aagtaggttt 2820
tagttgggat tgttagggtc gcggtcgttc gtacgttcgc gggtttcggt cgcgtcgtcg 2880
tcgcgcgttt ttgttcgaga gagttttggt ttcgttagcg gggttaggag tcgggttttt 2940
tatcgtagcg tttttcgtcg cgttagtttt cgttagtggt agtatttcgt aatagttttt 3000
gtgtgtgagt tatcgtggat tttttttttt ttttttgggg gtcgggggga aagaaaagga 3060
tttaagtaaa ggttttttcg ttttgtgagg gcgagcggta aaggttcggt tgagtttttt 3120
atgtttttt tttttcgtg taaaaagttt ttttgtgtaa ttgtttttt tttttttga 3180
acgtgttttt ttgtaatgat taaggtatcg atttttgtta agttttttta ataatatgaa 3240
attgtttatt tacgtcgtaa ttttttttgt tttttttttt tttttttttt tcgttcgttc 3300
gttttcgttt tcgttttttt tcgttgcgtt tttatttttt tttttaattt tttttttttt 3360
ttgtaatttt ttagttcgtt ggtttttttt ttggtttttt tttttttttt ttttatttat 3420
tttttttggt ttgataattt tgttttaagt gttttttaaa agaggttatt ttagttagta 3480
tgcgcgttgt gggtaattgt tataagtgtt tttaggttta ttgtgaagag aatgtatttt 3540
gtattcgtga attgttttat gggggggagg gagggttaat tatatatttt gttgtttttt 3600
tatattttgt tttgttgttt gcgtttgaaa agggcggaag agttataata aagtttataa 3660
gcgagaattc gagattggtt cggttagcgt tttttatttg ttttcgggtg ttttgtagag 3720
tcggtgggga gtttgttatc gggtttttgc gtttgttgag gttaggttgt agagaggagg 3780
gggcgggggt tggaggtagg tggtgcgagt ttttcggttt aggggcgttg ggggtgaggg 3840
aggcggttga gtttgattgg aggagagaaa aacgagggag gggagttaag agaaattttt 3900
ttttttcgcg tttcgaggtt gttcggttag ggaggttagg gcgtttaggt tacgttggag 3960
atttgcggtt gggttggtgg ttgtgttttt cgttattttt ttttttgttt tgttgtgtgt 4020
ttttttaaga tgtgtgtcgg gaggtggggt ggggggtagg tataaaggtg gagtgtaggt 4080
ttgttgagta tatttttttt ttttcggatt gttgttgttg ggggagtagg aggagaaggg 4140
agatcgtttt tttttgtttt ttttttttag ggatttggtt attttcgttt tgttttgagg 4200
ttatggtttt ttgttttttt tttttcgttg tttttgtttt tttggtagtt acgttttatt 4260
gaggtattcg ttttcgggaa tcgggagagt agcgcgttcg ggttaatttt tatttttttt 4320
tttttttttt atttttttaa tttttttttt tttttatttt taaatttttt tttttttttt 4380
tttttttttt tttttttttt tttttgttat tagaaacggt agcgtttaag gttcgtgggt 4440
tggattttgt gttttaggag tatttaggaa gatttgtttt attttcgttc gtatatttgg 4500
ggtattaaga gagatagagt cgtgagttag gttcgtttta tttttcgttt tgggagaatc 4560
ggattaattt ttttaaaagg ttttgtcgtg tagaattggt ttggtttaat aaagaataga 4620
tttgttttaa gtatttattt atttagttgc gtgtatttaa tttttgcgtt tgtttttcgt 4680
tcggtaacgg gttgaagagg atgggtggag ggtgatacgg atcgtttcga ggttatttat 4740
tttttttttt atttaatttt ttttttttta aatttcgttt gtaagcggtt tttagttcgc 4800
gggagtcggt agcggttttt gagtttttag ttttaattta tagggttcgg tttttttatt 4860
tattattgat tatattttat aaatttaacg ttatataatt ttttttatat tatcgggagt 4920
cgtggggaga cggttcggtt attggcggag gggacgttac gtgggcgggg ttacgtggtt 4980
cggagaggaa aaagggggtt ttttttggtg taaatttgga ttttaatttt gtaatatatt 5040
aaggaatttc gtaaaatcga tattaaaacg tttttgttta taaattattc ggttaaatta 5100
tgagtttatt gtattatgcg aatgttttat tttttaaata tttagtttta agttcggttt 5160
tcgttatcgg agttttttta gaataaattt tttgtgcgtt tgtttttaat ttttagcgtt 5220
cgggttatgg agcgggttcg ggcgtttttt tcgtcgtttc gatgtagggt ttgtatttcg 5280
gcgggggggg tatggcgggt tagagcgcgg tcggcgttta cgcggtcggt tatgggttcg 5340
agtcgagttt ttttaatatg tattgcgcgt tttttgagta gaattttttc ggggtgtgtt 5400
tcggcgattt cgttaaggcg gcgggcgtta aggagtagag ggattcggat ttggcggtcg 5460
agagtaattt tcggatttat ttttggatgc gaagtttagg taacgtcgcg tatcgagttg 5520
ttttgagttt taggggtcgg ggtatattat tttgaaggcg tttttttttc ggttgggcgt 5580
tttttttcgc gtttagagtg tttttagtcg gaggtcggtt ttggaaggtt gtatttattg 5640
ggtcgcgatt tagtaggtta ttttcgtttt tatatttgtt cggtgttttt aggttcgttt 5700
tgtttttcgg cggatttttt ttcgtcggta ttacggcgtt gcgagttttt agttcgtttt 5760
tatttttttt ttcggttttt tagttttaaa tatttattag tagcgtcggt acgggttgga 5820
gacgaggtcg tttgttttgc ggaggaaggt cggtgtttgt agagaatagt tattagggtt 5880
tttttttttt tttttttttt tagttaagga ttttagtttt gggtgtgttt ttttgtttta 5940
gttcggataa ggtatggggg agggggttat agcgttaagt aatttttttt tttttttttt 6000
tttttagtta atttcgtttt tttattattt ttattaggat aattagaggt gggttttaga 6060

ggtttggcgg gaggagaaaa ggatttagtt agggatatag agttagagag gtaggtggag 6120
ggagggggtt gaggttaaag gagatgattt ttttgatttt agtgaatttg ggtgggggag 6180
tggaatagga gaattagaaa ggggtagagg ttttatggga gtgtagttgg ttttttgagg 6240
tgtttttttt agggttttta ggaattttta aagaattggt taagtttgga ggtgttattt 6300
tagataagtg attttttttt tgggggaggg gttaggattt tttggagtta atttaaaaat 6360
tagagagtgt agtttattta gttagttgtt gtagaatttt aagaggttga ggttttatgg 6420
ggtttttgtc gagtttgatt tagatatttt tttttgtatt gttttaaagt agacgaattt 6480
aggggttagt tttaaagttt agaagattta agatttatgg ttagaggtat ttaatttagc 6540
gatggttagg ggttagaagt agtttagttt ttatattatt agtattagtg gttataggtt 6600
ttattatttg ttttatagtt taattgtttt tttagttgat ttattaaagt tttatttttt 6660
ttgtttatta gtattttagt ttggttttta tttgatttga aattagggtt tttagttttt 6720
ttttttttgt ttttttttta ggagggttga tattagtatt tttttaaagt ttttttaggt 6780
tttggtttta atgtagaagt ttttttttat tttttagttt ttattattta gatattttat 6840
gttttatttt attttaggtt gttgtttttt tttttttttt gtgtattttt ggaggtttaa 6900
ggattgtttt agagagttgg gttgtttagg taagtaggta agtaggatag tttggttttg 6960
gttgaagttt agttatttat tggaaaagaa ttattagagg gttattgttg ttagattaat 7020
tttttgtttt aaggaaattt gtaaattttt atttttttat tttataatat gttggggatg 7080
agttgggggg attgggtttt tgaggttttt ttgttttttt ttttttttgg gaaacgtttt 7140
taagttaggt ttggcgtttt attttagggt tatttgtatg gttttttttg gtttttattg 7200
gaaagattat tagattagta tttagaaaag ggggtttagg gtttttgatt ttagagagtg 7260
aaaggaggat taattttttt ttattttatt tgtatttttt aggtgtagag tttttaagag 7320
ttagtagatt atttttttgg gtttagaagt tttaggttaa aaaagatttt ttggatgaaa 7380
aatgggaatg tttgttttga gaagtttgta gaaagtttag taattagggg agtttgtagt 7440
tggagtaagg gttggggtat gaaaagttag aatttggttt agtgggaaaa tgggtttttt 7500
tgtttatcgt ttattgattt taaattatat atacggtata agagggaggt ggttattttt 7560
ttggcgtttt tttttttttt tttttttttt ttttttgaga cggagtttta ttttgtcgtt 7620
tagattggag tgtagtggcg taatttcggt ttattgtaag ttttgttttt cgggtttacg 7680
ttattttttt gttttagttt tttgagtagt tgggattata ggcgttcgtt attacgttta 7740
gttaattttt ttgtattttt agtagagacg gggtttattt gtgttagtta ggttggtttc 7800
gattttttga tttcgtgatt cgttcgtttc ggttttttaa agtgttggga ttataggttt 7860
gagtttatcg cgttcggtcg gcgttttttt tatttattga tttttttttg aatattgtgt 7920
gtagg 7925

<210> 148
<211> 7925
<212> DNA
<213> Artificial Sequence

<220>
<223> chemically treated genomic DNA (Homo sapiens)

<400> 148

tttgtatata gtatttagag gaaaattagt gagtaaagaa aacgtcggtc gggcgcggtg 60
ggtttaaatt tgtaatttta gtattttggg agatcgaggc gggcggatta cgaggttagg 120
agatcgagat taatttggtt aatatagtga aatttcgttt ttattaaaaa tataaaaaaa 180
ttagttgggc gtggtggcgg gcgtttgtag ttttaattat ttaggaggtt gaggtagaag 240
aatggcgtga attcgggagg tagagtttgt agtgagtcga gattgcgtta ttgtatttta 300
gtttgggcga taaagtgaga tttcgtttta aaaaaaaaaa aaaaaaaaaa aaaaggaaac 360
gttaagagag tggttatttt ttttttatat cgtgtatgtg atttgaagtt aatgggcgat 420
gggtaaagaa gtttattttt ttattaaatt aaattttggt tttttatatt ttagttttta 480
ttttagttgt aaattttttt aattgttgaa ttttttatag gttttttaaa gtaaatattt 540
ttatttttta tttaagaagt tttttttagt ttggagtttt tgaatttagg aaagtaattt 600
gttggttttt aaaggttttg tatttaaggg gtgtaagtgg gatgagggga ggttaatttt 660
ttttttattt tttggggtta aggattttaa attttttttt ttgaatgttg atttgatggt 720
ttttttagta gagattaaga gaaattatgt aagtgatttt ggagtggggc gttaggttta 780
gtttaagaac gttttttaag aagaaaagga gataaagggg ttttaaaggt ttagtttttt 840
taatttattt ttagtatgtt gtggaatgag aaggtaagaa tttgtagatt tttttggagt 900
aaagagttgg tttgatagta gtgatttttt gatgattttt ttttagtgag tggttaagtt 960
ttagttaggg ttaggttgtt ttgtttgttt gtttgtttag atagtttagt tttttaaagt 1020
aatttttggg tttttaggaa tgtatagggg aaaaggagag atagtagttt gggataggat 1080
ggaatatgga gtatttgggt ggtggggatt gaagggtaga ggggaatttt tgtattagga 1140
ttagaatttg aggagatttt aaagaggtgt tgatgttagt ttttttggga aggagataag 1200
ggaggagggg ttaggggttt tagttttaag ttagataaaa gttaagttga aatgttgatg 1260
agtagaaaaa atggggtttt gataaattaa ttggaaaggt agttaaatta tggagtaggt 1320
aatgggattt ataattatta atattgatgg tatgagagtt gggttgtttt tggtttttga 1380

ttatcgttgg gttgggtgtt tttgattatg agttttgggt tttttggatt ttggggttag 1440
tttttaagtt cgtttgtttt ggaatagtgt agaaaaggat gtttgggtta gattcggtag 1500
ggattttatg gaattttagt tttttggggt tttgtagtag ttggttaggt gaattgtatt 1560
ttttaatttt tgaattggtt ttagagagtt ttagtttttt ttttaagaaa agggttattt 1620
atttggaatg atatttttaa atttggttag ttttttaggg gtttttagga gttttaggaa 1680
gagtatttta ggaagttagt tgtattttta tgaagttttt gttttttttt agtttttttg 1740
ttttattttt ttatttaggt ttattgggat taggaggatt attttttttg gttttagttt 1800
ttttttttta tttgtttttt tagttttgtg tttttggttg ggtttttttt ttttttcgtt 1860
aggtttttag agtttatttt taattgtttt gatgggaata atgggggagc ggggttggtt 1920
ggagaggaag ggggagggga gaattgttta gcgttatggt tttttttttt atattttatt 1980
cgagttgggg tagggggata tatttagggt tgagattttt agttgggaag gggggagaag 2040
ggggagattt taatggttat tttttgtaaa tatcggtttt ttttcgtaag ataaacggtt 2100
tcgtttttag ttcgtgtcgg cgttgttgat aaatatttaa agttaaaagg tcgagaggaa 2160
gggtgggggc ggttgggggg ttcgtagcgt cgtggtgtcg gcgaagagag attcgtcggg 2220
aagtagagcg agtttgagag tatcgagtaa atatgggggc ggggatggtt tgttgggtcg 2280
cggtttagtg ggtgtagttt tttagggtcg attttcggtt gggagtattt tggacgcgga 2340
aaggggcgtt tagtcgggaa ggaggcgttt ttagggtaat gtgtttcggt ttttggggtt 2400
taggatagtt cggtgcgcgg cgttatttga gtttcgtatt taggggtaga ttcggaagtt 2460
attttcggtc gttaagttcg agtttttttg ttttttggcg ttcgtcgttt tggcggagtc 2520
gtcgggatat atttcggaga ggttttgttt aaagggcgcg tagtgtatgt tgaaggaatt 2580
cggttcgagt ttatagtcgg tcgcgtagac gtcggtcgcg ttttggttcg ttatgttttt 2640
ttcgtcgggg tataagtttt gtatcgaggc ggcgaaggaa gcgttcgaat tcgttttata 2700
gttcgggcgt tgggggttgg aagtaaacgt ataagaagtt tgttttggga aggtttcggt 2760
agcgaaaatc gaatttgagg ttggatattt agaaaataaa gtattcgtat aatataatga 2820
atttataatt tggtcggatg atttgtaggt agggacgttt tagtgtcggt tttacgagat 2880
tttttgatat attatagaat tagagtttag atttatatta aaaaggattt tttttttttt 2940
tttcggatta cgtgatttcg tttacgtgac gtttttttcg ttaatggtcg ggtcgttttt 3000
ttacggtttt cggtaatgtg gaaaaaattg tgtggcgttg gatttataaa atatgattaa 3060
taatgaatgg gaaagtcgag ttttgtggat tggggttggg ggtttaaggg tcgttgtcga 3120
ttttcgcggg ttggaggtcg tttgtaggcg agatttgggg aggaagagat tgagtggaag 3180
ggaaaataaa taatttcggg gcgattcgtg ttatttttta tttatttttt ttagttcgtt 3240
atcgagcgag agataaacgt agggattgaa tgtacgtaat tgggtgggtg ggtgtttggg 3300
gtaggtttgt tttttattaa attaggttaa ttttgtacgg taaggttttt tggggaagtt 3360
ggttcggttt ttttaagacg aggaatgagg cgagtttggt ttacgatttt gttttttttg 3420
gtgttttaga tatgcgggcg gagatggagt aagttttttt aggtgttttt agggtatagg 3480
atttagttta cgggttttgg acgttgtcgt ttttggtggt aaaaaaaaaa agaaagaaag 3540
aaagaaaaag aaaaaaaaag gtttgaaagt aaaagaaaaa gggggttggg agggtgggga 3600
gagaaaggaa gggtggaggt tggttcgggc gcgttgtttt ttcggttttc ggaggcggat 3660
gttttagtgg agcgtgatta ttagagaagt agaggtagcg aaggaaggaa ggtagagagt 3720
tatggtttta gaatagagcg aaggtggtta agtttttgag agggagaggt agggaagagc 3780
ggtttttttt ttttttttgt ttttttaata ataatagttc gagggaaaaa aagtatgttt 3840
agtaggtttg tattttattt ttatgtttgt tttttatttt atttttcgat atatattttg 3900
ggaggatata tagtaaaata aagagggagg tggcggggga tatagttatt agtttagtcg 3960
taggttttta gcgtggtttg ggcgttttgg tttttttggt cgggtagttt cggaacgcga 4020
gaggaggggg ttttttttgg tttttttttt tcgttttttt tttttttaat taggtttagt 4080
cgtttttttt atttttagcg tttttgggtc gagggattcg tattatttgt ttttagtttt 4140
cgtttttttt tttttgtagt ttagttttag taggcgtagg agttcggtga taggtttttt 4200
atcggttttg taaggtattc gggagtaaat gaggagcgtt ggtcgggtta gtttcgggtt 4260
ttcgtttgta aattttattg taattttttc gtttttttta ggcgtagata atagaataaa 4320
gtatagagga ataataaaat atataattag tttttttttt tttttataaa gtaatttacg 4380
gatatagaat atattttttt tatagtaaat ttaagaatat ttgtaataat tgtttatagc 4440
gcgtatgtta attaaagtaa ttttttttga gaaatattta agataaaatt gttaaattaa 4500
agggggtggg tggaggagga aaagagagaa gttagagaga aagttagcga gttggggggt 4560
tgtagagggg agagaggatt gggaggggaa atgaggacgt agcgagagag agcgagagcg 4620
agagcgagcg agcgagagag agagagagaa gggagataga aagaattacg gcgtgaatag 4680
gtagttttat gttgttggga gaatttagta gaaatcggta ttttggttat tataaagaag 4740
tacgtttaaa ggaaaaaaaa gataattgta taaggaggtt ttttatacgg ggaggggagg 4800
ggtatggggg gtttagtcgg gtttttgtcg ttcgttttta tagggcgagg gagtttttgt 4860
ttaaattttt tttttttttt tcggttttta agagaaggga aggagattta cggtagttta 4920
tatatagaag ttattacgag atattattat tagcggggat tggcgcggcg ggggacgttg 4980
cggtgggagg ttcggttttt ggtttcgtta gcggggttag agttttttcg ggtaggggcg 5040
cgcggcggcg gcgcggtcgg gattcgcgga cgtgcgggcg gtcgcggttt tggtagtttt 5100
agttgaagtt tattttttgt cgtttttttg cgcgtcgttt tcgttcgtcg tttttggggt 5160
tcgttttagt ttttgttttt ttagtttttt ttgttcgtat ttgttgttgg ggaatttgtt 5220
tttgttgttt tttttttttt attttatttt tcggttttgg aattagattt tgatttgttt 5280
ttttgttagt tttagggcgt gcgatatttc gattcgtcgt ttacgagtta gatagggatt 5340

aaataggaat ttttttttta gttttagggt ttggtagcgg ttgtaggttt gtcggtttcg 5400
tttgcgttcg gcggttgttg ggaatggggg aaagggcgag atagagggga ggggagggtg 5460
ggggaggggg tagggacgga gaaggttgga agattcgtga acgagtttgg gagattatta 5520
taataagggt aggggatttt ttatttcgtg cgggggcggg gggggcgtaa gaaggtcgcg 5580
gtcggcggcg gaggaggggc gagggggatt agatataagt gaatttaagg gagttagaaa 5640
ggggagtttt ttttttcggt aattagtatt agagttgtgt aggaggtggt ttagatttat 5700
tagaagtgtt ataaatttta ggaggaagga tgtgaaggag gggggaaaat aagaataaaa 5760
aagtcgggtg gggacgaatg gatttatttt gaaaagtttt tagagttttt gttttagaag 5820
aaaagttttt ttagggtaat taaattataa agtaattgag aagtgtaaaa gttgagcgtt 5880
tttattttta ttattttttt tttattattt tttataaaat agtaaaaggg gaaaaaaagg 5940
aggagaagaa gaagaaaagt aaggtagggt gaggggttta tagatttagg aaatgttttt 6000
aagcgatttt ttttaaggga aagaaaaaag aaagaagaaa gaaagataag aaagaaaggg 6060
gaaaagcggt aggcgatcgg aacggagtcg gtaagttttt tagaagttgg aggaaatgcg 6120
tttcggtttg ttaggttttt tgttcggttt tgggtttttt cggttttttt ttgtttttgt 6180
cggtttcgag gcgagtttat tttgcgggcg tatttagggg aagagttgtg tgggcgacgg 6240
gttttgttcg gagttttcgg ttttttcgtt taggtcgttg gcggcggtaa gtttgtagtt 6300
tgcgtattgt attaggtttg gattttgcgt atcgaatagg ttttggcgtt gtagcgggtc 6360
gtagtcgtag aaattgtcgg ggttttcgtg gtacgttacg gcgtacgggt tttgttggta 6420
gggagtcgtg gatagcgacg acggttcgtg gtagaatttt tggatttgcg acgggtgttg 6480
gaagttgtcg tcgttgttgg gatcgtatat tacggtgggt cggtcgttta ggttttgggc 6540
gaagtcgtag ttataataat tggggcgtag ggatttttcg gttttgtatt tggagaatag 6600
tgagttgacg aaataagagt ttattttatt gaattttgag gcggcggcgg cggcggaggt 6660
tatagttggg ggttgttggg gagggggtgg ggagggggaa agggagggag agagagaaaa 6720
aaacgcggat tcgtcggttt ttagttattt tcgttaggaa aggagaggga aagagaggag 6780
ggaaaaaaag aaaagaaaga aaaggcgaag aagatttcga agtcgatata ttttttgtgg 6840
tttttaggaa tagaaaagga tagagaagtt tttaaaagtt taagtttgta tggtagtcgc 6900
ggttcgttcg tttttttttt atttttattt ttttaaataa aatatattta ttatttaaag 6960
aggttttcgt tttatatttc gaagaaggga tgttagttta taaatagttt tcggtgattt 7020
atttttttgt aaatgagttg ttttatattt tgtattgttt ttttatgatt atttgtattt 7080
attagagatt tcgtatttta ttggtttttt cgtatttttt tcggatagaa cgtagagcga 7140
gggtgagagc gagagagagc gagcgagaga gagagttaga gcgagagagc gttagggagt 7200
gaggagagag cggaaaaaaa aaaaggaggg gaagaaaaaa atatttaggg agagagaggg 7260
ggagaggggg agagagaggg ggagagggag agggggtgag agagaatgaa tacggattaa 7320
atttgtttaa ttatttatat taatgagata tttttcgttt tataataaat taaatatttt 7380
cgtagattat ttttaaagat tgatgtgggg gagttacgga aaaaaatacg tatatatata 7440
tatataacgc gttaggcgta ggtacgttgt taatttcgcg attttaacga ggtaattgga 7500
gtttttttaa atgttagggt cgtttcggaa aaatgtaaaa gaatacggtt taattgcgtc 7560
gagttttaaa aggttttata aatgtaattg gtattttaat ataagttatt aaattatata 7620
gttttttttt ttaaacgtat ttttaaataa acgaggtagt tatatttaaa gttttaatga 7680
ttaatttttt tattattgat aaaggtttaa ttatcgtgtg gagggaattg gttggataat 7740
cggtttttta aaaaacggtt gtaaattttt taagaaatta taaaacgtaa taaagtcgga 7800
gtttgttgtt gtttatgtcg tagcgtataa aagttgttag tattttacga gttttttttt 7860
ttatagttat aaaattgtag tttcgatcgt tttcgtcgtt cgattgtagt acggtggggg 7920
tgacg 7925

<210> 149
<211> 8910
<212> DNA
<213> Artificial Sequence

<220>
<223> chemically treated genomic DNA (Homo sapiens)

<400> 149

ttttttttta atggcgattg ttggggtttg gttgttgatg attggtagtt ttatatttcg 60
ttttgtgagg aggagttgag ttaaggttgt aggcgggaga aggttaattt taagttttag 120
tagttgggtt agggaggtat gcgattgagt ttggtgttgt tttttttatt aagtttagta 180
tattagagaa aaataagaaa ggttaggaaa gaaagttagg gggaaggaaa taattggaga 240
gagaaaagag taaagaatta attgtataat aaagaaagaa ggaaataagg aaatgggaga 300
aataaggaag ataagaaaga gaaaggattg gaaaaagtag gaaaaaataa attataggaa 360
gaaaaaagaa aaagaaaaat agaaagaagt aaataaagag aaaagagaag gatgaaataa 420
gaaggaaaga aagaagagaa gaaaaaaaaa gaaaaataag aaaggaaagg aaggtaaata 480
gagatttttg aaaaggaatt taatcggtaa gaaaggttcg gtgttttttt tcggagtaag 540
ttagtttaat tatagtttcg atttagttta gaaatttgat ttattggggg taatttagaa 600
gtaaattatt aaagtggggg gtagggtttg tttttaagat atttttttat ttttttttta 660

aagttttttt tcgaggtgtt ttcggtttgg ttttaattta ggggataaaa ttaaataatt 720
ggttagtttc ggtagggtta ggaagtagtt agattggcgc gggttattag aattcgggat 780
tcggagattt tagatagtat tttgggtaaa agtgaatatt tgtggttgtg tatatatatt 840
taagtgtaag ttagatacgt gtatagatgt tgaatttcgt agtatttgtg tgtaagtgtg 900
tgtaatgtta agacgtgaga gattttgttg gtcgagttgt ttggggggtt gtgaggttta 960
tagtagatta gaggttttgt taagtgtttt tagtttttta ttttttttta gtatttgttt 1020
gaaaattttt tttttttttg ggtttttggg tatttcgaat tttgtttgga atttttttgt 1080
ttttaattta gttggggtag agtttggtgt tagtgaagaa gaaaagcgaa tttttttttt 1140
aagttttggg aagggaagga tttatttcgg tttttaaagt aagaggaaaa aatttgtaat 1200
ttttggtgtg atttgttttt tttttgttt aaaaagattt taatatatat attttatttt 1260
tattttatta tttaatttaa aagggaaaga tatttttttt tttttatttt atttttagaa 1320
ttttttagtt gattttttta atttgttggg gaaattttaa ttttttgttg gagaaattag 1380
ggtgtttttg gggtgtgttg agggttatag ttaagaaagt aataaaggtt tttttttgtt 1440
tattgagtat attttttttt ttttgttttt acgtggggat ttttagcgtt ttagtttttg 1500
ggttgttttg tggaattcgt tggatttttt tagagtattt atagagtttt tgaatttttt 1560
ttttttgtat tgtttatagt tttatagata gaaggaggtt tagtttcgtt tttgttgttt 1620
ttttaggatt tgggtattcg ggaagggaga aaggggttag ggtattggtt ttgggtggga 1680
tttgttaggt cgtatgtata gtttgagttt tattgtggtt tgtagttttt ggatttttat 1740
gtttttaagt tgaggtaaga ggggggagtt ttagacgtag gtataggtat aggtgtttta 1800
gcgttttcgg aaagttgggg aaatggatta ataaaagttt atatgtgggt gattttagta 1860
atataggttt tttttggatt ttaatttagt tgttaagggt ataggttgaa ggattttggt 1920
tttgtggggt agggcggtgg gtgtttggta gtggcggtag gcggggtcgt tgtggcggtt 1980
cgtatgtttt tgaacgttag gttcggataa taggatcgaa atagtttttg ttcgtggttt 2040
cgggaaagat cgagtttaag ggggtagggt tgggatttag gttcgtataa atgttttttt 2100
cgttaggata aaatatttat tttttttttt tttttatttt tattgaagta gaattttaaa 2160
gtttgtgaaa atttaataag ttatttaggt ttgcgttcga gcgggtttta gtttagtagt 2220
atcgggtttt cgtttatttt gcgtaattag aggtaaatat ttgttttatc gggggtttta 2280
tttaggttga tgaatttgat tttttttttt attaatcggt cgtgtttatt atatttttaa 2340
ttattttaat tttaaatatt attttgtttt gtgaggaggt gggagtcgag gcgggaggtt 2400
tttttttgga gtattatttt tattaatgat ggcgataatt tcgaagcgat tttgtttttt 2460
tttttggtta aggtgagagg tgggtttgga aagaggttgg gtggtgggat ttttgaggtc 2520
gatgggtttg ttttttgttt gttggttttt taggttacgt tttggggttt gtttgaagtt 2580
tatttttttt attttttttt gtcgattttt ttattttttt ttaggttggg tttaaatttt 2640
ttgcggtttt tttttggaaa tttttttttg attttagttt gttttggttg gtttgggtag 2700
aggttcgggt agtattgttt tggtttttag ttattaatat ttagagaaaa aaaaaaagaa 2760
aaggttgagg tggttaaggg ttatttggtc gtggggaaaa ggatttatta gattttaaag 2820
tggggggtaa gggtagggaa ttgggacgtt aaatttttgg gaaaagtata ttaggtagga 2880
aaataaagga gacgtgaaag aggggggagg gaatttttaa agttatataa tttttaattg 2940
ttttggtatt taattttatc gttttataaa tgaggaaata ttagttttta tattaaattt 3000
ttatttttgg ttgataaata tgacgggaaa ttttcgttgg gttttcgtaa aagggaacgg 3060
tttcgattaa attggtgggg aaagattaat gtagataata taaatagagt ggttataaag 3120
tttcgttcgt ttttcgcggg atgggcggtg ggataaatgg ttttttggat tgattgttgt 3180
tcggattgtt ttaagtttgg ggtttaaata gtttaatttt gttttggata atttttatgt 3240
tgaggttacg gtgattttgt atatgaaaat ataaaattat tttaaatttt ttttggtcgg 3300
ttttgtaagt aaattatata ttttatattt ttttttagtt tttcggaggt ttaggaattt 3360
ttgcgttttt tttttttatt tttggatggg gaaattcggc ggattttttt ttgcggtttt 3420
ttagtcgtag cgggtttggg tttttttaga aacggtttag tttttttatt tttttatttt 3480
agggcgttgg gttttagttt ttttttcgtt aaattttagt ttttcggaaa gtttgaattt 3540
taggttttgt ttaaggaaaa atgtggggcg ataggaaaga taagttgttt tttagatttc 3600
gttttttgag gaaggggtgg agagtttagt tcgttttgga agaaggcgat ggagaaacgg 3660
aaataattgt ttgaaatata cggttttttt tatttttttt ttttatttaa aaataattat 3720
aaaaatagat tggtttgttt ttttttaaat atgtttttag ttcgtgcgtt ttgtaaagga 3780
cgtttttttg ggtcgggttt tgttgcgttt tgtgtgttgg tttttcgtgt gatacgtgat 3840
ttttttaaat tgatcgaggt ttcgtaggtt tttttttttt ttttcggaat attttttgtt 3900
ttagcggggg taaggttaag cggaaagcga ggatcgttag ggtttgaagt aaagtagtag 3960
aatcggtttt aaggttaaag ttttgtgggt ttttgagatg gtgatttagg agtcgtttat 4020
tttgttaggt ttaggttata gttttcgggt cggggatcgg gtgtttttgg tttttggttt 4080
tttttttttt ttatttcgcg tttattcgga attttttttt tcgagtagtt atgaagtgat 4140
cgtgattttt tttttggttt attttagagg ttggagattt ttagttaatt tttataggaa 4200
atttgtaatt ttagcgtcgc ggtttttaag tttttagttt cgagggttaa gttcggggaa 4260
aagaggaggc gggagagtag aaattttagt tttgtttggt attttttaat tttttttttt 4320
ttttttttgt ttattagatt tttttaaatt ttttttttta taaattttat tttattttta 4380
tttttaaggt aagaagtggg agtcgggttc ggggatatgg aaaggggaga tgaagagtgg 4440
ggagaagtat ttgattcgtg tttcgatttt ttaatttgaa tttgattgtt ttgagtttcg 4500
ggttgtttcg tttttttttt ttttttcgta tttttttttt tttcgttttt ttttttttag 4560
gagttatagg ttggttattg gataaataaa ttttattttt tttagaattt ttatttttat 4620

ttttattttt ttttattttt ttattttttt tgtcgttcgg ggggcgtttt ttttgttcgc 4680
ggggaagggt ttcggtgttt ttatttcgag gtagttgtta gatggcgttg ttattttatt 4740
ttgcgcgttt cgtttgtcga taatttgatt tcgttgacgt tacggtcgtt tgaattatta 4800
aggttatttt taaattttat tggtttagtc gttatatggt gagaatcgaa tgcgcggata 4860
attacggagt tgatattttt tttttttttt tttttttttt tttttttttt ttaatcgcgt 4920
tttttttttc ggatggggaa aaaaaaagat gttagttttt tcgttgtagt attgtttttt 4980
aaaaattttt ttttttgaaa atgatatgtt ttcgtaatgt aatttcgaat tcgtacgcgg 5040
agtttttggt tgcgttcggc ggaggagagc gttatagtcg gagcgtaggt atgtatatgt 5100
agtttgggag tgattttaat tgcggggtga tgaggggttg cgggttcgcg ttttcgtttt 5160
ttaagaggga cgagggtagt agttttagtt tcgtttttaa tatttattcg ttttattttt 5220
cgtagttgga tttttgggc gattttaaag tcgtttatcg tttggaataa tttgttggta 5280
ggtcgttgtt tttttgtttt tatttattta gtgttaagga ggagaatgtt tgttgtatgt 5340
atagcgtaga gaagcgggcg aaaagtggtt tcgaggtagt tttttatttt tattttttgt 5400
cggagttttg ttttggggag tacgaggtat tcgtgtttag ttattatcgc gttagttcga 5460
gttatttcgc gttggataag acgttttatt gttttggggt taacgatttc gaagtttttt 5520
tcgagtagcg ggttagtttt aattcgcgcg tcgaatattt ggaatcgttt tagttggggg 5580
gtaaagtgag tttttttgag atttttaagt tcgatagtta gatttttagt tttaatgaaa 5640
ttaagacgga gtagagtttg gcgggtttta aagggagttt ttcggagagc gaaaaggaga 5700
gggttaaagt tgtcgatttt agtttagata tttcggataa cgaagcgaaa ggtaaggtcg 5760
tttgggtcgc gggcgttatt gggacgtttc ggtatttggt tttcgcggtc ggggaggggg 5820
gtaggggaga gggttgggtt taggaggttt tagattattt cgggaatgcg attttggttt 5880
tcgattagcg ttcgttgagt tttaggttgg tggcgcgtta tttagttggg gaaggtaagc 5940
ggcgacgtgg aggtgtcggt tttgtttcgg ttatgggtgt taaggcgggg gcggtgcgta 6000
tcgcgggtcg tttgtaggcg tagtgtgttg tatcggacgg gtggttgttt ttaggcgcgt 6060
gttggttgtg gtttgttttt ttaatgttgg tgttttgggg agttgacgtt ttttagttta 6120
ggttaggggt ttgtaaaaag tttaaaatgg cgattttggg ttagggatta gggaaggttg 6180
gggagatggg gggagttttt tttattgcgt ttttttagtt gaaaattgtt ttttgcgaaa 6240
cgcgatttgt tgtttgtggg tttgatttgt gcgtgcggtt tgggtttttg cggtttttgg 6300
ttcggtcggg ggttttgggt agcgaggttg gagtcggaag aggtggaggt gaagggttgt 6360
tcgttacgtt tttttttttt ttagatgttt ggtttggatg gcgttggaat agggtatttg 6420
gaatgttgta agttttttgt tttttttttt aatttttttt tttttatttt tttttagtta 6480
aattattttt ttttattggt ttttaaaagt agtttataaa tgagtggttg ggaaattttt 6540
gtgtgcgtgg taagaggttt ttttttgaaa gggaatgtgg ttttggttgg attttattgt 6600
aattttggtt gagagaaagt ttagttagag tttaagttta gggtttaaga tttatttttg 6660
ttgtaggagt tgggggtttt cgggattttt ttttgatatt tggggaaaga atgtggagga 6720
aacgggagta ggggaggaaa agttgagggg aacgattttt tttgaaaagt tgtagtaaga 6780
gttttagaaa aaggtttaat taacggggaa gaataaattt gttttttata atatcggtta 6840
ttggtgatag tatataaggg gtttattggg tgtagaggaa gatgaaatat tgagagcgga 6900
gtaggttggg ggcggtttgg attttttggt gttttgtata tggatgcgac gtatacgtat 6960
gtttacgggc gatttgtgtt atatttacgt atatttatat tgtatgattt ttgtatattt 7020
acgtatgttc gtttatatat gtataggtat ttttttggcg ggtaagggta gagttttatt 7080
tttttgggtt tgtaaaggtg gttttggtaa attattttta tagagatatt gattggaaga 7140
tgagcgttgg tgtgtttatg aggataaggg ggagagtgag gcgtttatag ttttaaagtt 7200
tttcgtttgg tacgtgtgta gtgtgaaggt ttaattttat atttttttta tagattttga 7260
gtttggttag ggagtagatt tggggtagga aaatttgtat tttagttttt attaaaagta 7320
aagttaaggt taataagttt attttttaat tcgatttgta tttattttta taggtttttt 7380
ttttttttta gttgttttta tagtaattta atttttttta ttttaaggaa ttttttttat 7440
tttgtatttt ttttgtgttt tttttttttt tttttttgtt ttgatgtttt tattaggtta 7500
gaggtaagag agtttttgat taaatttttt tatttataat ttgttttgtt tttttatttt 7560
attaaattta ataattttag taagaaaata aataggacgt agttagatgt ggagagtttg 7620
taaataattt ggtaagaagt tgtaggtgta gagataggag agaaaattcg ggatatttag 7680
atttttttag attgtaattt taagtttgtt ttttttgtgt gttttttggt tttttttagg 7740
ttttcgtttt atttcgtttt taataagata agatttagga atttggtagt tttattggaa 7800
tagggaggag agagagttta gagttattag tagagttttt tgttgggtgt ttttttaggt 7860
ttttggattt tgttttttgt ttttaaaaag atggttaggt ttttttttgt tatttgagaa 7920
gtaaggttgg tttattttag taagaatgtg tgttagggat agaggttgtt aataagttta 7980
tgggaaatat atagtatttt atagtatttt tttataaaaa aagatttatg tatatattta 8040
tgtgtgtgta tttatgtgta taggtgtgtg tatttttgta ggtatatatg tgtatttgta 8100
ggtatatata tttacgtatg tatagattgg gtagggggtt tttaggaggt tgtatttttt 8160
taggatatgt tttttttttg attttaggtt ttttggttag gaaagttgtt tgaggatttt 8220
ttaggttgtt ttagttttgt tgtttttgat ttttgttgtt tttataagtt ttttttagtt 8280
gtatatattt gggagaaatg attaagagag gaagagatgg taatgggaga tttaagggag 8340
tagagttatt ttgtggtagt ttaggttggg gtaagagttg ggggtttagg ataggattta 8400
ttgtagtggt ttttggttat agattttgga ataaagtttt tttattgagt tgaaataggt 8460
taaagttttt tgggaatttt tttggtttgt gggtggattt tgttttggtt tggggtttgg 8520
aaaatgtttt tttatttggt tggatttttt tagagttgga atggaataga gagaaggggg 8580

gatttggtaa ggtttaggga atttgttagg gttagttagt tggggaaaag ttagggtatt 8640
taggattttt tagattaagg tggatttggg taatttaaat ttaataaatg tttggaggta 8700
agaatttttt tttttatttt ttttaagttt aaatttaaaa gaaattgtaa gaattttgag 8760
tttaagagga gtttatcgtt aggtttttgg ggtaagagta gtttttttgt gtttttttag 8820
ttattttata gtaggttggt aaaggttgta gcgttgagag ggttttcgat ttcgagggat 8880
atttatgtaa ataatgttat ttttaataga 8910

<210> 150
<211> 8910
<212> DNA
<213> Artificial Sequence

<220>
<223> chemically treated genomic DNA (Homo sapiens)

<400> 150

tttgttaaaa ataatattat ttatataagt atttttcgaa gtcgaaaatt tttttagcgt 60
tgtagttttt gttagtttgt tgtggggtgg ttgagggagt ataggggagt tgtttttgtt 120
ttaggggttt ggcggtgggt tttttttagg tttaaggttt ttataatttt tttggggttt 180
agatttaggg agataaaggg aggaagtttt tgtttttaag tatttattaa atttaaatta 240
tttaagttta ttttaattta aaagattttg agtattttgg ttttttttta gttggttgat 300
tttggtagat tttttaaatt ttgttagatt tttttttttt tttgttttat tttagttttg 360
gaaaaattta attagatgga aaagtatttt ttaggtttta gattagagta gagtttattt 420
atagattaga gaaattttta gagagttttg atttgtttta atttagtgga gggattttgt 480
tttaggattt gtggttagag gttattgtag tggtttttgt tttgggtttt tagtttttat 540
tttaatttga gttgttatag agtgattttg tttttttagg ttttttattg ttattttttt 600
tttttttaat tatttttttt aggtatgtgt agttgagggg agtttgtaag agtagtagag 660
gttaggggta atagagttgg ggtaatttaa ggggttttta aataattttt ttggttaaag 720
gatttaaggt taagggagag atatattttg aggaaatgta gttttttaga aattttttgt 780
ttagtttgtg tatacgtaga tgtgtatgtt tgtaagtata tatgtatatt tgtagagata 840
tatatattta tatatataag tatatatata tagatatgta tatgagtttt tttttgtaag 900
gagatattat agaatattgt gtatttttta tgagtttatt gatagttttt atttttgata 960
tatatttttg ttgaaatgga ttagttttgt tttttaagta gtagaaggga atttggttat 1020
ttttttaagg gtaaaaaata gagtttaaag atttgagagg atatttagta aaggattttg 1080
ttgatggttt tgagtttttt ttttttttta ttttaatgag gttgttaggt ttttggattt 1140
tattttgttg ggggcggggt gaggcggggg tttaagagga attagaaaat atataaaaaa 1200
ggtaaatttg aggttgtagt ttaaaaaaat ttgaatgttt cgaatttttt tttttgtttt 1260
tgtatttgta gttttttgtt agattgttta taagtttttt atatttaatt acgttttgtt 1320
tattttttta ttggagttgt taggtttggt ggggtggggg gataagataa attataaatg 1380
ggaagtttta attaaaagtt tttttgtttt tggtttggtg gagatattaa aataggagga 1440
gaggaaagaa gggtatagag agggtatagg gtaagaggga ttttttgggg tgggagggat 1500
taggttgttg tggggatagt tagaggaaag ggagagttta tgggaatggg tgtaggtcga 1560
gttaagaggt gaatttgtta attttgattt tgtttttggt gggggttaga atgtaggttt 1620
ttttgtttta aatttgtttt ttaattaagt ttagggtttg tagagagggt ataagattaa 1680
atttttatat tgtatacgtg ttaaacgaaa ggttttgagg ttgtaggcgt tttatttttt 1740
tttttatttt tatgaatata ttagcgttta ttttttagtt agtgttttta tggggatgat 1800
ttattaagat tatttttgta gatttaaggg gatgaggttt tgtttttgtt cgttaaaaga 1860
gtgtttgtgt atgtgtgagc gggtatgcgt gagtgtgtaa ggattatgta atatggatgt 1920
gcgtgagtgt ggtataggtc gttcgtaggt atgcgtgtgc gtcgtattta tatataaagt 1980
attaggagat ttaggtcgtt tttagtttgt ttcgttttta gtattttatt tttttttata 2040
tttaatgagt tttttatata ttgttattag tagtcggtgt tgtggggagt aggtttattt 2100
tttttcgttg gttgggtttt tttttgaggt ttttgttata attttttagg aaaagtcgtt 2160
ttttttagtt tttttttttt tgttttcgtt ttttttatat ttttttttta agtattagag 2220
aaagatttcg aaggttttta gtttttatag tagaaatgaa ttttgaattt tgagtttgaa 2280
ttttggttgg gttttttttt aattaaaatt atagtgaaat ttaattaaag ttatattttt 2340
ttttaggaga aaattttttg ttacgtatat agaaattttt taattattta tttgtaagtt 2400
gtttttggaa attaatgaaa aaagataatt taattagaaa aaaatggaaa agaaaaaatt 2460
aaaagaggaa atagaaaatt tataatattt taaatgtttt gttttaacgt tatttaagtt 2520
aggtatttgg aggagggagg gacgtggcgg gtagtttttt atttttattt ttttcggttt 2580
tagtttcgtt gtttaaggtt ttcggtcgag ttaaaagtcg taggagttta agtcgtacgt 2640
ataaattaga tttataaata ataaatcgcg tttcgtagga aataattttt aattgggaaa 2700
acgtagtaaa gagaattttt tttatttttt tagttttttt tagtttttgg tttaagatcg 2760
ttattttagg ttttttgtaa gtttttgatt tgagttgggg gacgttagtt ttttaggata 2820
ttagtattga gaaagtaaat tatagttagt acgcgtttgg gagtagttat tcgttcggtg 2880
tagtatattg cgtttgtagg cggttcgcgg tgcgtatcgt tttcgtttta gtatttatgg 2940

tcggggtagg gtcgatattt ttacgtcgtc gtttattttt tttagttaag tgacgcgtta 3000
ttagtttgga gtttagcgga cgttagtcga aagttagggt cgtattttcg aaatggtttg 3060
gggttttttg ggtttaattt ttttttttgt tttttttttc ggtcgcgaag attaagtgtc 3120
ggaacgtttt agtggcgttc gcggtttagg cggttttatt tttcgtttcg ttattcgagg 3180
tgtttgggtt ggagtcggta gttttggttt tttttttttc gtttttcgag gggttttttt 3240
tagggttcgt taggttttgt ttcgttttga ttttattggg gttgggggtt tggttgtcgg 3300
atttgggggt tttagggaaa tttattttgt tttttagttg aggcgatttt agatgttcgg 3360
cgcgcgggtt gagattggtt cgttgttcga aaggggtttc gaagtcgttg gttttagaat 3420
agtggggcgt tttgtttagc gcggagtagt tcgggttggc gcggtagtag ttgggtacgg 3480
gtatttcgtg ttttttaagg taggatttcg gtaaggggtg ggagtagaga gttgtttcgg 3540
ggttattttt cgttcgtttt tttgcgttgt atatgtagta gatatttttt tttttgatat 3600
taggtgggta ggagtaggag gatagcggtt tgttaatagg ttgttttagg cgataggcgg 3660
ttttggggtc gttttaggag tttagttgcg agaggtagga cggataggtg ttgagggcga 3720
ggttggggtt gttgttttcg ttttttttgg agagcgaggg cgcgagttcg tagtttttta 3780
ttatttcgta attgaagtta tttttagatt gtatatatat gtttgcgttt cggttatagc 3840
gttttttttc gtcgggcgta gttaagggtt tcgcgtacga gttcggagtt atattgcgag 3900
ggtatgttat ttttagagag aggggttttt aaggagtaat attatagcgg aggagttgat 3960
attttttttt ttttttattc gggagggggg gcgcggttgg aggggaggga ggaaaaagaa 4020
ggggaggggg ggaaatatta gtttcgtaat tattcgcgta ttcggttttt attatgtgac 4080
ggttagatta atgggatttg aaaatggttt tgatgattta gacggtcgtg acgttagcgg 4140
ggttaagttg tcggtaggcg gagcgcgtag agtggagtaa tagcgttatt tagtagttgt 4200
ttcggggtag gggtatcgga gttttttttc gcgaataaag gaagcgtttt tcgggcggta 4260
ggggaggtag aggggtggag gggggtgggg gtgggggtgg gggttttagg aagagtgggg 4320
tttgtttatt tagtaattag tttgtaattt ttggggagaa gggggcggag aggggggaaa 4380
tgcgggagag agggagagag cgaggtaatt cggggtttaa aatagttaaa tttaaattaa 4440
aaggtcgaga tacgaattag gtattttttt ttatttttta tttttttttt ttatattttc 4500
ggattcgatt tttatttttt attttgggga tgggagtggg gtgaggtttg tggggggggg 4560
ggtttggggg gatttgatga gtagagaaag gggaaaaaaa gttaaggaat attaaatagg 4620
attaaagttt ttgttttttc gttttttttt tttttcgggt ttggttttcg gggttggaag 4680
tttgggggtc gcggcgttgg agttgtaagt tttttgtggg ggttgattgg gaatttttag 4740
tttttagggt gggttaggga gggggttacg gttattttat ggttgttcgg aaaaggaggt 4800
ttcgaatgga cgcggggtag gggaaagagg aagttagaga ttagaggtat tcgattttcg 4860
attcgagagt tgtggtttgg gtttggtagg gtgggcggtt tttgggttat tattttaggg 4920
gtttataagg ttttggtttt ggagtcggtt ttgttgtttt attttaggtt ttggcggttt 4980
tcgtttttcg tttgattttg ttttcgttaa aatagggaat atttcgggaa agaaagaggg 5040
aatttgcgga gtttcggtta gtttggagga gttacgtatt atacgggaaa ttagtatata 5100
agacgtaata aaattcgatt tagagaagcg ttttttatag agcgtacggg ttgggagtat 5160
gtttgaaaga agatagatta gtttattttt gtggttgttt ttaaataaaa ggaagaaata 5220
aggggaatcg tatgttttaa atagttattt tcgttttttt atcgtttttt tttaaagcga 5280
attaaatttt ttattttttt tttaggaaac ggaatttgga gaataatttg ttttttttgt 5340
cgttttatat ttttttttgg atagagttta gggtttagat ttttcgaagg gttggagttt 5400
ggcggggaaa ggattggagt ttagcgtttt ggagtggagg agtgaggagg ttagatcgtt 5460
tttaaggagg tttaagttcg ttgcggttag aagatcgtag aaagaaattc gtcgaatttt 5520
tttatttagg ggtggagaga gaagacgtag gggtttttga attttcggaa aattggaagg 5580
aaatgtggag tgtatgattt gtttgtagga tcggttaggg gagatttgaa atggttttgt 5640
gtttttatat gtagggttat cgtggtttta gtatgggggt tatttagggt aaggttaggt 5700
tgtttaggtt ttagatttgg gatagttcgg gtagtagtta atttagggag ttatttgttt 5760
tatcgtttat ttcgcgggaa acgggcgaga ttttatggtt attttgtttg tattatttgt 5820
attagttttt ttttattagt ttgatcgaaa tcgttttttt ttacgaggat ttaacgaaaa 5880
tttttcgtta tatttattag ttagggataa aaatttagta tgggaattga tattttttta 5940
tttatgggac gatgaaatta aatattaaag taattgagaa ttatatggtt ttgggggttt 6000
tttttttttt tttttacgtt ttttttattt ttttgtttgg tgtgtttttt ttaggaattt 6060
agcgttttag ttttttattt ttatttttta ttttggagtt tggtgggttt ttttttttac 6120
ggttaagtgg tttttggtta ttttagtttt tttttttttt ttttttttgg gtgttagtag 6180
ttgggggtta ggatagtgtt attcgagttt ttgtttaggt tagttaggat aggttgggat 6240
tagagaaagg tttttaggga agagtcgtaa agggtttgag tttagtttgg gagggggtga 6300
aggagtcggt aggaaagggt gaaggagata aattttagat aagttttaag gcgtagtttg 6360
gggaattagt agatagggag tagatttatc ggttttaggg gttttattat ttaatttttt 6420
tttaagttta ttttttattt tagttaaggg agaggataga gtcgtttcgg aattatcgtt 6480
attattaatg aaaataatat tttagggagg agtttttcgt ttcgattttt atttttttat 6540
aagatagaat agtgtttaaa gttgaaataa ttagaagtat gataaatacg gtcgattaat 6600
gaaaaaagaa attaaattta ttagtttaag tgggattttc ggtgggataa atgtttgttt 6660
ttaattacgt aagataaacg aaaattcggt gttgttaaat tagagttcgt tcggacgtag 6720
atttgaataa tttgttgggt ttttatagat tttgagattt tgttttagtg gaggtaaaag 6780
gaaggaaaaa gtgagtgttt tattttggcg aagggagtat ttgtgcgggt ttgggtttta 6840
gttttgtttt tttgggttcg gtttttttcg aggttacgag taaaggttgt ttcggtttta 6900

ttgttcgggt ttggcgttta aaggtatgcg ggtcgttata gcgatttcgt ttgtcgttat 6960
tgttaggtat ttatcgtttt gttttatagg gttaagattt tttagtttgt gtttttggta 7020
attggattag gatttagggg aggtttgtgt tgttagaatt atttatatat ggatttttat 7080
taatttattt ttttaatttt tcgagggcgt tgagatattt atgtttatgt ttacgtttgg 7140
ggtttttttt ttttatttta atttgggaat atgaaaattt aggggttgta agttatagtg 7200
gggtttaagt tgtgtatacg atttgataaa ttttatttag ggttaatgtt ttggtttttt 7260
tttttttttt cgggtattta gattttggaa aggtaataag gacggggttg ggtttttttt 7320
tgtttgtggg gttgtgagta atgtagagga ggagggttta ggagttttgt gaatattttg 7380
ggaggattta gcgggtttta tagggtagtt taagggttgg ggcgttaggg gtttttacgt 7440
gaaagtagaa gaaggaaaat atgtttaatg aataaaaaag gatttttgtt gtttttttgg 7500
ttgtagtttt taatatattt tagaagtatt ttggtttttt tagtaaaaaa ttggagtttt 7560
tttaataggt taagaaagtt aattgggaaa ttttgaagat gaagtgaaaa aggaagaatg 7620
tttttttttt ttgggttaaa taataaaata aaaataaagt gtgtgtattg aaattttttt 7680
aagtaaagag gaaatagaat tatattagag attgtaggtt tttttttttt gttttggagg 7740
tcgagataaa tttttttttt tttaaaattt gaagaaaaga ttcgtttttt tttttattta 7800
gtattaggtt ttgttttagt tgaattggga gtagggaaat tttaggtaaa attcggaatg 7860
tttagagatt tagaaaagaa aaagattttt aaataggtat tagagggagg taggaggttg 7920
aggatattta gtagggtttt tggtttattg tggattttat agttttttag gtagttcggt 7980
tagtagaatt ttttacgttt tgatattgta tatatttgta tatagatgtt acggagttta 8040
gtatttatat acgtgtttaa tttgtattta aatatgtgta tatagttata gatatttatt 8100
tttatttaaa gtgttgttta agattttcga atttcgaatt ttaataattc gcgttagttt 8160
gattgttttt tggttttgtc gaagttggtt agttatttaa ttttgttttt tgaattgaaa 8220
ttagatcgaa gatatttcgg agagaagttt tgggaggagg gtgggaggat gttttagggg 8280
taggttttgt tttttatttt agtggtttgt ttttaagttg tttttagtaa gttaggtttt 8340
tagttagagt cggggttgtg gttaggttgg tttgtttcgg agaaaaatat cgaatttttt 8400
ttatcggtta agtttttttt taaagatttt tgtttgtttt tttttttttt tttatttttt 8460
tttttttttt tttttttttt tttttttttt ttgtttattt tttttttttt tttttattt 8520
gttttttttt gttttttttt tttttttttt tttttgtaat ttattttttt ttattttttt 8580
taattttttt ttttttttgt tttttttatt ttttttattt ttttgttttt tttttttttt 8640
attatataat taattttttg tttttttttt tttttaatta tttttttttt tttgattttt 8700
ttttttagtt ttttttattt ttttttggtg tgttgggttt agtgaggggg atagtattag 8760
gtttagtcgt atgttttttt agtttagttg ttaaggtttg gagttgattt tttttcgttt 8820
atagttttag tttagttttt ttttataagg cgaagtgtag ggttgttagt tattagtagt 8880
taggttttag tagtcgttat tagagggagg 8910

<210> 151
<211> 6984
<212> DNA
<213> Artificial Sequence

<220>
<223> chemically treated genomic DNA (Homo sapiens)

<400> 151

tattttgggt ttttattcgt gaatatttag gcgtatttgt ttagttttaa gagtgtatat 60
aaatgtgtgt atttttataa aatgatttaa agaaattttt ggggtttgcg ggtaggtatc 120
ggttgtataa atattttata gagttagttt tatgtttttt gagtttcgtt aatgtacgat 180
ttatattcgg agataaaagt ttagataata gatggttgtt tatgataacg aggtttttta 240
agtttggggt taggcgataa atttttggcg aatttttttg taatttaatt tttgattgta 300
gtgaggagaa aattattgag ttcgtcgaaa ggaaagtagt aaaatggggg agatttgtaa 360
attttgagta gaaggtattt tttttgttgg ggtaaggagg tataggttat aatatttatt 420
ttacggagta gtttttattg tggaagtttt cggattaagt tttagagtat aggcgagtta 480
gagtttttta ttgtttgttt aggtttttag gtttcggaaa ttagggtaga atgtgtaggt 540
agaaggtagg gtttggaggt tggaatagat tttaaataaa tgatttttgt tgggggtaaa 600
agattttttt ttgtttttgt ttttggtgat tggaattttt ttgttttgtg ttatttgaag 660
ttttttattt tatgtttttt tatttttttt tttttttttt ttttttggat tgattagttg 720
tttgtttttt tttgtatttt cgatttaggt atttgatcgt taataaagag ttgttgaagt 780
tgattttata aaaatgtcgc gagtttattt tgattaggag gattaggttc gagttttaat 840
gtaggagtta aagttagaga attagggttt cggttaattc gtttttgttt tgtggtgagg 900
cgggagattt gggtggttta atagggtaat tttatttaga aaaggtattt tttgtgttat 960
tataattggt ttaggaaatt gaagtttaat agtagcggtt ttatttttag tttgttttta 1020
agatattttt tttattttgg agtatttggt gagggggtta gtgaaatcgg ttcgtaaaat 1080
agggttttta tgggaagaaa tagggtcggg ttgttagtat tttgggataa tttttttata 1140
gaggaagttg ggtggggttt gtgggggtaa aggagttgtt tttttttttt tttattattt 1200
tttttatagg ttttaggaat ttttgagtag cgtttattag tttaggtggt ttgatttggg 1260

gatttaggtt tggggtaatg gttaagtttg agaagaggtg gaagttagga attagtagtt 1320
ttattcgttt tttgtttttt ggttggggtt ggggtgtttc gggtgtaaga taaattattg 1380
atttgaaaat tttaaagtat attaaaggga ttattgtagg tagtgatatt attgtaaggg 1440
agagaaatat aggtaagaag gtgaaagtta atttttaatg aagttttttt gttggtttag 1500
tttgtgaata aacgagagcg tttatgtgtg ggattttttt cgtttatttt tttattgatt 1560
ttatataggt tgaatttata atttaagttt attggagtat atgtgggttt tatttttttt 1620
tttagtattt ttttttttag tttatggttt gtgggatggg tttttttttt tattacgatt 1680
tgttttttag aatttagagt agtggaggag gtagataagt taataggagg ttttaacgtt 1740
gtgtgcggga gtttgttttg ggagtcgttt agggtagaat ttaggagata ttgttttttt 1800
tttaagttgt ttttgttgtt cgtgtgttgg agggaagggt gttggagttt gttttaattt 1860
tggtttagtt atttggatag ttgtttagat cgtagtcgtt tgttagtagt taggttggtt 1920
gatggcgttg attgaggata ttaagagtta gtggggaggg tcggatggtt ttgttgtaga 1980
gaattttagg tgaaggttag gatttaaagt agatggaggg gttataggta tttattagat 2040
aagatataaa taagttattt ggagcgggtt tgataaggaa gggattgggg gttagggatg 2100
gtttttgtat agggaggtat tgatgttgtg gttagaattt ataggttgag agggtgtagt 2160
ttagggtaaa tattttagtt gaagcgttta ggtagtttgg atttagggtt gggtagggag 2220
gttattttag attatgggta tttttgttag taaatgttta gttgatttta gtggttaggg 2280
tttttttttt ttgatcgtag tagtaattga tagttgttag aggttttttg atttgttgaa 2340
aaattaagtt atattaatat tagagaatat tttaaatttt atgttttatt tttaagggga 2400
ggaatattat tttttagaag tttttgttgg gagattttag cgttttttag atattaattt 2460
tttgatattt ttttttgtat tttttttttg ttttaaggaa atagggtcgt tagtagtttt 2520
ttgttgtcgt ttggtttatt tttatggtgg tttaggaata aattttggtg gttaaattta 2580
agtgtttagt ttagtagttg tttttttata ggatatagtt tttattttta ttttagtttt 2640
ggttttgttg aatttgtttt gggggattgg gttttatatt attttggagg gttaggaggg 2700
ggaaggaagt agagttttgg gtggaagtgg agaaagcggg atttgagatt cgagttttta 2760
tttatatttt tgtaaggata ggggtatttt tcgttttttt tttgttggtt tttattattg 2820
tttgggtagt attttgtttt taataaattc gagtttaatg aggttagtgt gaattttatt 2880
aaaagttata gtttatagtt ttttattatt ataattaaaa atgttggttt tattttattt 2940
tataaaaagg gttagaggaa aaaggaaata tagtagattt ttttttttat ggttttttat 3000
taatagaagt tttatatttt agtaaagttt taatttagtt tttttttgtt ttagtttaag 3060
atttgattag aattaaagga gtagcgttta gtattgagtg cgtagagggg tatttgagat 3120
ttatttttga gtattttaga attattttga gaatgggatt tatggtggag gtagatttta 3180
tttaggtgtt atttaaatgt tgagtaagtt tgaggaggta ggagtattga ggaggaatgg 3240
attttagggg ttatatatgg agataagtta taattttttg tgtttttttt atttggtttg 3300
ggagtcgtag tattttatta tgttttatta agataaattt attttaattt ttttaagtgg 3360
aaattaattt taattgaggg ttatggatgt aaagagagtt cgggttattt tttgtttttt 3420
tttgagtgtg tattgggttg agttttgggg ggtttcggtt tttttttttt tttttaaata 3480
aattgtgttt tggtagtttt tttttggatt taaggttgtt tttagtttat gttggttgtt 3540
tttggttttt tggatttagt ttagaatttt ttaataaagt gatttttaga tgtttatatg 3600
tgttttgtgg tgtttagagg gatggaaggt aaggggttgg ggatgtagaa tgattgagtt 3660
tattttttgg gttatagtta tttaggaggt tgatagattt gtaggagggt aggatagggt 3720
tgagaaatag gaaggaggta ttaagatttg gttatagttt tagggataga ggttgggggt 3780
tttgattagg gagtggtcgt tgggtttttt ttgatgttat agtatttatc gttttttttt 3840
attgtttggt tagttttttt tggagattta tatttggttt tattatttat tttatagttt 3900
ttaaaattat ttaaggataa gaattttaaa tttagttttt tttattatat tttttttgga 3960
tttttatatt aggttttttt ttagcgtatt agttgaggtt gagttattaa gttaaagggt 4020
tttgatatag aagatatatg gtttttattt taattttttt ttttggggtt tgggatttga 4080
aaatttatag tggatgtagt tttttttata ttgattgata ataaaggtag gtatgaaata 4140
tttatttagg gttatttttt tatattaaat atttaatata atattagaga aggttttttt 4200
tagatttcgt taggtatcgg ttgggcggtt ggcgtattgt ttattattcg tttatgatta 4260
atttgatttt ttagttaggg gtagtatagt aagttagata aagttgggat attggtttgt 4320
agttgggttg ttttattttt gggaaatttt agatattttt tcgggagttt ttttaaggat 4380
tttagaaagg tagttttgat atttagtttt ggttaggaaa agagtggatt tttttaaatt 4440
tttgtgaata ggcggtgttt ttgcgggttg gaagggtaag agtttttgag atttaaggga 4500
aaggatttat ttttttgggg aaattaggta gtggggaaag aagtttttat ttttattttt 4560
ttggtgttgt ttatagagag gaggtaagat ttttatatat gagagttttt tttttttttt 4620
taattttggg ggaaaaaaga gtagtagtta gaaaaaaata taattatttt tttttagtta 4680
ttattttcgt ttattttatc gtttatttta ttttaatttt tttttttttt ttttgttcgt 4740
gttgtggtat ggagagaagg ggagtgacga gagaaaaacg aatataaatt tgtaattgat 4800
ttttataatg tttttgcggt gtttgtaaat taatcgtttg aacgttttcg atttatttta 4860
agagagaatt ttttttacgt ttgcgaagtg tttcgaattg atatatgata atatttattt 4920
tggattacgt gtttagagag agagagatta agacggataa cgcgttattt cgttttttta 4980
aatttttttt tttcgttaga tcgggtttaa aatttttatt cggagtcggt aggagaggag 5040
aacgatgttt aattcggtta atttgggtaa tttttgtttt tcgtcgcgta aggagagggg 5100
cgtagatttc ggcgagcgag ggagttgcgt ttttaatttt tatttgttta gttgtattta 5160
ttatatgttc gagtttttta cggttttttt ttttttgttt taggtttttt ttcgttagat 5220

tttttatttt tattcggttt aagtgttttc ggttcgggag gttttttacg gtttggagtt 5280
attcggtaag tggtattatc ggaatagtta ttttttttgt tatgcggcgg tcgacgagtt 5340
tatgtatcgg gagtgtttgt ttttttttat cgttatcgag atttttatga aaaacgaagg 5400
tttttacggc ggttattatt attttagcgt ttcgtacgta attttcgtcg gtttttattt 5460
tttagttaat aagaatagcg ttttgtttta agttttcgat cgttttttcg ataacgttta 5520
ttgcggtggc ggcgattcgt tcgtcgagtt tttttgtttc ggtaagggcg aggttaaggg 5580
ggagttcgag gtattttcgg tttcgggatt ggcgtttcgg gttgaggcgg gtgtcgaggc 5640
ggaggttgag gaggagaata taaattttag ttcgttcggt ttagtttatt tcgtggttaa 5700
ggagtcggtt aaaggagtcg tttttagtag gtagtagcgg tcggggaacg ggcgggtagc 5760
gagggaggga gcgagagagg gagggcgaga gaagggggga ggtaagggga gcggggacgg 5820
tttcgtgttt tgggttagtt cgattttatg tggagtttta taagtattta aaggatttta 5880
ttataattta taaagttttt ttttttaacg attcgatttt ttacgatgga gaaggggtgg 5940
ggagggaggg aaaagggttt tttggaaaag tcgggtgaat tttttttttt cgttattttt 6000
ttcggtttgt gaaattttta aaagcgtaat tattgcgggc gatattaatt ttgatcgtga 6060
atttagagga gtatttaagg aagtatgggg agtcgggttg tcgaggagtg gggaggaggg 6120
gaggggtgga gggggagaaa ggggagggcg agggaaagat agggagagat ttagagaggg 6180
ggagaggtgg gggagaggag taatggagta gaatttgaga tcggagaggt aagttaggat 6240
cgttatgatt ttttttaaaa ttagttttga aaatgtttaa attatgtgtt cgcgggtttt 6300
cgagtagaaa tttaagtaat tttgggttta gcggcgatag gggaatgggg cgaggcggcg 6360
taggatttta ttgcgtttta ggcgggggtt tgggcgtttt tttcgggttc gcggttttag 6420
tgtttgttta gaattgcggt gtggaaggcg ttgtttcgcg ggtttttcgg ggcgttaggg 6480
tttcggaagc ggttttttgg tgtttcggtg tttgttggtc ggtttttttt tcgtttttcg 6540
attgttttcg ttagatttta ttgtttcgcg tttgttttta gttttttttt tagatttttg 6600
ggggaggggt tgggtttttc gaattattag gagggcggtt taggaagggt tcgagggcgg 6660
agggggagta gatagggggt tcgaggggac gtacgtgtat ttggagggtt ttttttttgt 6720
tttagatttt gttagtcgcg gttttcgttt gagaattggg gacggggtgg cgtagtggcg 6780
ggggggtggg gattcgttag atttggtagg gggtcgaggt ttgttcgggt gttgcggttg 6840
taggagagtt agtcgtttgg cgggagggtt gttcgcggtg ggttaggaga agggtcgttg 6900
agcgtttagc gggttggggt cgttcgggtt ttacgtgttt tttttttttt ttttttcgta 6960
tttgtttttt tttttttttt taga 6984

<210> 152
<211> 6984
<212> DNA
<213> Artificial Sequence

<220>
<223> chemically treated genomic DNA (Homo sapiens)

<400> 152

tttggaggga ggggaggggg taagtgcgag gagagggggg agagagatac gtgagattcg 60
ggcgatttta gttcgttgag cgtttagcgg tttttttttt agtttatcgc gggtagtttt 120
ttcgttaggc ggttggtttt tttgtagtcg tagtattcgg gtaagtttcg attttttgtt 180
aggtttagcg ggtttttatt ttttcgttat tgcgttattt cgtttttagt ttttaggcga 240
gagtcgcggt tgatagggtt tgggatagaa ggaaagtttt ttaggtatac gtgcgttttt 300
tcgggttttt tgtttgtttt tttttcgttt tcgggttttt tttgggtcgt ttttttagtg 360
gttcggaaag tttaattttt tttttagaaa tttgggggga aagttgagaa taggcgcgaa 420
gtagtgaaat ttggcgagag tagtcgggag gcggggagga agtcggttag taggtatcga 480
gatattagag agtcgttttc ggggttttgg cgtttcggga agttcgcggg gtagcgtttt 540
ttatatcgta gttttaggta agtattaagg tcgcggattc gggagaaacg tttagatttt 600
cgtttggaac gtagtggagt tttgcgtcgt ttcgttttat ttttttgtcg tcgttgattt 660
tagagttgtt tgggtttttg ttcgaggatt cgcgaatata tagtttgaat atttttaaaa 720
ttagttttaa gaaggattat agcggttttg gtttgttttt tcggttttag gttttgtttt 780
attgtttttt ttttttattt tttttttttt ttggattttt ttttgttttt ttttcgtttt 840
tttttttttt tttttttatt tttttttttt tttttatttt tcggtagttc ggttttttat 900
atttttttaa atgttttttt aagtttacga ttaaagttaa tatcgttcgt aatggttgcg 960
tttttaaaaa ttttatagat cgagggagat aacgggggag gggggtttat tcggtttttt 1020
taaagagttt tttttttttt tttttatttt ttttttatc gtaaaaagtc gagtcgttgg 1080
gagagagggt tttgtaggtt ataataaaat tttttgaatg tttataaaat tttatataaa 1140
atcggattga tttaaaatac gaggtcgttt tcgttttttt tgtttttttt tttttttcgt 1200
tttttttttt tcgttttttt tttcgttgtt cgttcgtttt tcggtcgttg ttatttattg 1260
ggggcggttt ttttggtcgg ttttttggtt acggagtggg ttgaatcgga cgagttggga 1320
tttgtgtttt tttttttagt tttcgtttcg gtattcgttt tagttcggga cgttagtttc 1380
gaggtcgggg gtgtttcggg tttttttttg gtttcgtttt tgtcggagta ggggggttcg 1440
gcgggcgggt cgtcgttatc gtagtaggcg ttgtcgaaga aacggtcgaa ggtttgaggt 1500

aggacgttgt ttttgttgat tgaggagtag aagtcggcgg gggttgcgtg cggggcgttg 1560
gggtggtggt ggtcgtcgta ggagttttcg ttttttatga ggatttcggt gacggtggaa 1620
ggaggtaggt attttcggtg tataagttcg tcggtcgtcg tatagtagga ggagtagttg 1680
tttcgatggt gttatttgtc ggatggtttt aggtcgtagg agatttttcg gatcgggggt 1740
atttgggtcg agtagggata ggagatttga cgagaggggg tttggggtag gaaggaggag 1800
atcgtggaga attcgggtat gtagtaagtg taattgggta gatagaggtt ggaggcgtag 1860
ttttttcgtt cgtcgaaatt tgcgtttttt tttttgcgcg acggagagta gaagttgttt 1920
aggttgatcg agttaaatat cgtttttttt ttttgtcggt ttcggatgga ggttttggat 1980
tcggtttagc gaggggggaa aatttgggaa ggcgagatga cgcgttattc gttttagttt 2040
tttttttttt gagtacgtga tttaaagtag atattgttat atattagttc ggagtatttc 2100
gtagacgtag gaggggtttt tttttaggta agatcgggga cgtttaggcg attggtttgt 2160
aaatatcgta gaaatattat gaaaattaat tgtagatttg tattcgtttt tttttcgtta 2220
tttttttttt ttttatatta tagtacgagt aaaggaggag agagagggtt ggggtggggt 2280
gggcggtggg atgggcgagg gtggtggttg ggaaggggta attgtatttt tttttagttg 2340
ttgttttttt tttttttagg attgggggaa ggaggagggt ttttatgtat ggggggtttg 2400
tttttttttt gtgagtagta ttaggagggt gggagtgagg attttttttt ttattgtttg 2460
atttttttag agaagtgaat tttttttttt aaattttagg gatttttatt tttttaattc 2520
gtagagatat cgtttgttta taagagtttg aagaagttta tttttttttt ggttagagtt 2580
gggtgttaag attatttttt tggagttttt agagagattt tcggaagagt atttgaagtt 2640
ttttaaaggt gaggtagttt agttgtaagt tagtgtttta attttgtttg gtttgttgtg 2700
ttgtttttgg ttagggagtt agattagtta taaacgagtg gtgagtagtg cgttagtcgt 2760
ttagtcggtg tttggcgaag tttgggaaaa atttttttta atgttgtgtt aaatatttag 2820
tatgagagag tggttttggg tgaatatttt atgtttgttt ttattgttaa ttaatgtgaa 2880
gaaagttata tttattgtgg atttttaaat tttaaatttt aaagaaaagg attggaatga 2940
gagttatgta ttttttatgt tagggttttt tggtttagtg gtttagtttt agttagtgcg 3000
ttgaaaagaa gtttagtgta ggggtttagg aagaatatgg tgggggggat taggtttggg 3060
gtttttattt ttggatgatt ttgaggattg taaggtgggt ggtggggtta ggtatgggtt 3120
tttagaagag attaattaga tagtggaaag gagcggtgga tgttgtggta ttaggggagg 3180
tttagcggtt attttttagt taaagttttt agtttttgtt tttggggttg tgattagatt 3240
ttagtgtttt ttttttattt tttagttttg ttttgttttt ttataagttt gttagttttt 3300
tggatagttg tagtttaaga agtaggttta gttattttgt atttttaatt ttttgttttt 3360
tatttttttg agtattatag gatatatgtg ggtatttgaa aattatttta ttgaaaggtt 3420
ttggattggg tttaaggggt taaaagtaat tagtatgagt tgaaagtaat tttggattta 3480
aagaggagtt gttagggtat aatttgtttg ggaaggaggg gaagaatcga agtttttaag 3540
ggtttagttt aatgtatatt taggggaaaa taaaaaatag ttcgaatttt ttttatattt 3600
ataattttta gttaaaatta gtttttattt aaagaaatta gaatggattt gttttgatga 3660
aatatgataa ggtgttgcgg tttttaaatt agataaggag gatataaaaa attgtagttt 3720
gtttttatat gtaattttta aaatttattt ttttttaatg tttttgtttt tttaaatttg 3780
tttagtattt aagtgatatt tgagtggagt ttgtttttat tatgggtttt atttttaggg 3840
tggttttgga atatttaggg ataggtttta ggtgtttttt tacgtattta atgttgggcg 3900
ttattttttt agttttggtt agattttaaa ttgaagtaaa gaagaattag attggggttt 3960
tgttgaagtg tgagattttt gttggtaagg ggttatgaaa agaaaaattt gttgtgtttt 4020
tttttttttt tagttttttt tatagagtga gatggggtta gtatttttaa ttatagtggt 4080
ggggaattat ggattatggt ttttaatggg gtttatattg attttattaa attcgagttt 4140
attgaaggta aaatgttgtt taaatagtga taggaattag taagagggaa gcgagaggta 4200
tttttatttt tgtagaggtg tggatgaggg ttcgagtttt agatttcgtt ttttttattt 4260
ttatttaagg ttttgttttt tttttttttt tggtttttta gggtgatgtg gggtttaatt 4320
ttttaaggta gatttagtaa agttaggatt gagatggggg taggggttgt attttgtgag 4380
gagatagttg ttggattgag tatttgaatt tgattattaa agtttatttt tgggttatta 4440
taaaagtgag ttaggcggta ataaggagtt gttggcggtt ttattttttt gaaataaaaa 4500
agaaatgtaa gaaagaatgt taaagagttg atgtttagga aacgttgggg ttttttaata 4560
gagatttttg gaaagtaata tttttttttt taaaagtaag gtataaagtt taaaatgttt 4620
tttagtgttg atatggttta gttttttaat aaattaaaaa atttttagta attgttagtt 4680
gttgttacgg ttaaggaaag aaggttttgg ttattgaggt tagttagata tttattggta 4740
aaaatgttta tggtttggag tagttttttt gtttagtttt gggtttaggt tatttggacg 4800
ttttagttga gatgtttgtt ttaggttgta tttttttagt ttgtggattt tggttatagt 4860
attaatattt ttttgtatag gagttatttt tggtttttaa tttttttttt gttaagttcg 4920
ttttaggtgg tttatttgta ttttatttgg tgggtgtttg tgattttttt atttgttttg 4980
agttttggtt tttatttgaa attttttgta gtagggttat tcggtttttt ttattggttt 5040
ttggtgtttt tagttagcgt tattagttag tttggttgtt agtaggcggt tgcggtttgg 5100
gtagttgttt aggtagttgg attaaagtta aggtagattt tagtattttt ttttttaata 5160
tacggataat aggggtagtt taagaagggg atagtgtttt ttaaatttta ttttgggcgg 5220
tttttagagt aaattttcgt atatagcgtt ggggtttttt gttagtttgt ttattttttt 5280
tattgttttg ggttttagag agtagatcgt ggtagggaaa ggagtttatt ttatagatta 5340
tgaattgggg aggaaaatgt tgaaagaaga aatggggttt atatatgttt tagtgggttt 5400
ggattgtaaa tttagtttgt gtggagttaa taaaaagata agcggagaga gttttatata 5460

tggacgtttt cgtttgttta taggttgggt tagtaaggag attttattag aaattggttt 5520
ttattttttt gtttatattt ttttttttta tagtaatatt attgtttgta ataatttttt 5580
tagtatgttt tgggattttt aggttagtgg tttattttgt attcgggata ttttagtttt 5640
agttagggaa taagaagcgg gtgggattgt tagtttttgg tttttatttt tttttagatt 5700
tggttattgt tttaagtttg agtttttaag ttaggttatt taagttggtg gacgttgttt 5760
agaggttttt gggatttatg gaggaggtag tggggaggga gaaggatagt ttttttgttt 5820
ttataggttt tatttagttt tttttgtgga gagattgttt taggatgtta gtaattcgat 5880
tttatttttt tttataaaag ttttatttta cgagtcggtt ttattggttt ttttattaaa 5940
tgttttaaaa tgaaggaaat gttttaaaag taggttggaa atggagtcgt tgttattggg 6000
ttttagtttt ttaagttagt tgtagtggta tagaaaatat tttttttggg tgggattgtt 6060
ttgttgggtt atttagattt ttcgttttat tatagagtag aggcgggttg gtcggagttt 6120
tgattttttg gttttagttt ttatattaag gttcggattt agtttttttg attaaagtga 6180
gttcgcggta tttttatgag attaatttta gtaatttttt attagcgatt agatgtttgg 6240
gtcgaaaatg taaaagggaa taaatagtta attaatttaa aaggaaaaag aaaagagagg 6300
ggtgggaagg tatggggtga aaaattttag gtgatataaa gtaaaaagat tttagttatt 6360
aaaaataaaa ataaaaaaaa attttttgtt tttagtaaga attatttgtt tgggatttgt 6420
tttagttttt aggttttgtt ttttgtttgt atattttgtt ttgattttcg gaatttggaa 6480
gtttaggtag gtagtgggga attttgattc gtttgtgttt tggagtttga ttcgaaagtt 6540
tttatagtga ggattgtttc gtggggtgag tgttgtggtt tatgtttttt tgttttagta 6600
aggaaagtgt tttttgttta ggatttataa gtttttttta ttttgttgtt tttttttcgg 6660
cgggtttagt ggtttttttt ttattatagt tagaagttga gttataggaa gattcgttag 6720
agatttatcg tttggtttta aatttggaga gtttcgttgt tataaatagt tatttattgt 6780
ttagattttt attttcgggt atggatcgtg tattagcgag gtttaggaga tatggggttg 6840
gtttgtgggg gtgtttgtgt agtcggtgtt tgttcgtagg ttttaggggt tttttgggt 6900
tattttatga gagtgtatat atttgtgtat atttttggag ttgggtaggt acgtttgagt 6960
gtttacgagt ggaggtttaa ggta 6984

<210> 153
<211> 11209
<212> DNA
<213> Artificial Sequence

<220>
<223> chemically treated genomic DNA (Homo sapiens)

<400> 153

gagcgcgggg aagttgtgaa agaaagagga ttggggttag ggattttaga agtttttaga 60
ggaggtattt tgatagtttt ttatggaagg ggttatttga cgtttttcga ttaaatttag 120
tatattatta agtagagttt gtagttgggg gtattttgga ggagaaatta ggagaagatt 180
tgtgatagtt taaatttgga ttttttgtat ggatttttgt agagttagag aggaaaaggt 240
ttttcgggtg attttaaatt ttagttagta ttgtaagttt taattaggta gtagagagga 300
atggattttt ttgatattta ttataggaaa ataagtgggg tttggaggta ggtaagtttg 360
ggtttaagag cggaaaattt tgaaataagg agtaaagtga tgttttagtt attttggttt 420
tatttttttt taagttgggg gtttagggag ggagaatatt aaaggtggaa gatgttagga 480
tggtagttgt tgtttaggag gtgagttttt aagttagtag aagaggttta gttgttattt 540
ttagaggttg ggttgtggga gtagagagat taaagtagag taggttttag agggatttag 600
gagtttggga ggaggtattt atgtttgtag ggtttatggt tgtagggacg aaggattttt 660
ttggagttta gagaagtgga gggaacggaa aaaggagggg agggtattgg gtaggtttag 720
ggagttgtag gattagttag tttttttttt tttttattta atattatttt ttgtagttta 780
ttaggaattt tttttaagta tgtgggaggg tttttttata ttgatatatt tttaagggta 840
tattttttta tatgtttttt ttgaattttt aatttttttt tataagtggt tttttttaaa 900
tatttttttt ttggttagtt taggttttgt ttttttttat tttttataaa tttttttttt 960
tatcggtagt ttgaattttg gataggaaag ggtttttaaa ttttttgttt gttttataag 1020
gttttaagtt tgggttagaa tttagatttt atggtggtga ggttttttgt ttttttatag 1080
ttttaattta tttttatttt ttttaaaatt ggaatttttt ttattaaaag cgggttgggg 1140
gagtaggagt tttagcgttt taagtttttt ttcgtttttc gtttatatgg aggaaataga 1200
gaaaagggtt gtagggagag aaattatttg atatttaatt tttagaggag aaaatttggg 1260
tattgaataa gggatataga aacgttattt tttttatttt tttttttagt ttagtattga 1320
agttatattt agtttttaaa tgtgtgtatt aggtttgtat ttttcgtttg taaatgttgt 1380
taagttatat ttttgatttc gttttagata tagtggggta tgaattttat ttttcgattt 1440
taaaaagata tagatatttg tttttttatt tttttatatt tttttggggg atgtttttgt 1500
aagtagcgta ggaagaatat gtaaattaaa agtgtatata gatagataag tagagatttt 1560
tatttgtata tagaggattt aggtagagag ttgggtattt ttaggaattt atttgggttg 1620
tgtttataag gtcgtgtgtt gaaacggtgt aggtagtttg tatttatttt taaatttata 1680
ggtacgtaag cgtacgcgta tacgtatata tatagacgtt tttcgtgaaa aagtatatta 1740

aatatttttt ttttgtgttg gattatttaa aagaaaatat tttagagttg aatttagtcg 1800
agagtttggt ttatgagttg tagttgaggg tagagtggtt tttgtcgttt cgcggttgta 1860
gaagtttttt tgaaaaagta gtcgagagta gagtcggggt ttttcgacga ggttgaggag 1920
tcgtttttta tttttttttc gtataattaa atgtttcgtt ttttttcgta tattagtagt 1980
tggatttttt ttaaatttta tttatagtta aattttattg ttttattttc gaaggtcggc 2040
ggggcgcgtt cgggtgagta tcgtagtcga gggttttagc gttaggtttt ggaaaggttt 2100
tttagggcga gatcgttttg gagatgtaat tattacgttt ttagtggtgt atattttttt 2160
ggagtgttta atttgatttt atttgtgttt tagcgagatt tatatttttt tttcggggga 2220
gggggaagaa attattttag gtttaaagtt aattaattag taaggtttta ttttttatcg 2280
tttttttgta ggtttttgtt tcggcgtttt ttttttcgat ttcggttcgt tttagatttg 2340
gagattgcgt ttgtaggcgc gaaaggtttt ggggaattaa gtaaaagaag agggaattgt 2400
ttagagtaag cggatttttt tagtcgtttt tagcgttttt agtttaaatt ttttttttta 2460
gtttttttat ttttttaagt cgttttaggg gtttataatt tcggttggtt tttttgagga 2520
agaggattat ggtttttttt tttttttttc gttattaaat tttggtcgta tagtgttttt 2580
aataaatgga attgtaaagt tgtcggatta attaaattta tattttattg taataggaat 2640
agttaatgtt tttatataaa tttggaaaag attatattat taattttatt tattgtttgg 2700
tcgttttgta tttagatttt ttacgtcgtt ttggttttta ttttcgttat tttggttttt 2760
atttttaaag gttggggacg tgtttttgag ttttggttcg cggaaaacgg gttcggcgtt 2820
gggtttgatc ggggagggag ttttaagaat ggggtgggcg ttttaggttt tttgtttttt 2880
ttggtagttt tgtagaattt tgtttatagc gttttattat ttttatttgg gagtagtatt 2940
ttatagaagg ggaggttagg atttgggttt tatttttatt attcgcggtt ggaaaaaggt 3000
aggcgttttt atttatttat ttttattagt taggttttag gtaagggggt taacgttata 3060
taatttagtt tagatttggt taggttttgt ttgttttttt cgttttattt tgattttgga 3120
ttttagtagt aggataagta gttttttagt agagtcggtt tttagtaaga ttttattttt 3180
tattgggttt attttttttc gttttttttt tattgttttt aatgtatatt cgagttggtt 3240
tttttatgtt ttaatttttt ttttgttgta tttttttttt taatgttagt ttagttcgta 3300
gtttgttttt tgaattttga gttgattttt ttggatgaag attacgtaga taagaattat 3360
tattttaatt tatcgatgtt cgggtgtttt taatcgttaa atattatttt ttttttataa 3420
agaggaggga ggtggttcga aggggttttt gtagatttgt ttatttgttt tttatttgag 3480
gggtgtagta ggtttggttg agcgaggcgt tgttttgtgg ttatggtttg tgcgttggtt 3540
gtatacgagt atttagtatt taaaatttgt ggtagaggga ggaaagaagg aaacgttgta 3600
gttttttttt ttttttattt tttttttttt attacgattt tttagggagg ttttgtgttg 3660
aaaggaattt tgatttaatt ttacggagat gtttttttag aaaggggtga ttttattgag 3720
gtttttgata agttgggaga ttgggaggaa agaagggaga gtttttgaag aattagtaag 3780
ttggtgttaa tttttttttt gaaatagggg gtagttttag attatgaaat tattttttat 3840
taaaagaggg tgatttaaga aagtcgtggg agttttattg cgggtattta aattggggat 3900
tgtgttatgt ggtttaattt aggtttttga gggtagaggt gggttttgta gggtttttta 3960
gagtggcgtt agagagaggt ttttttaaag gtgtagtatt gaattttttt tattttttac 4020
gttttttttt gtatatttat agaaagcgcg ttttttttcg tatttagggt ttttagtttt 4080
tttaggttga attttgcgtt tttattattc gtattttttt gaaaaatttt attttgagat 4140
tttttatttt tattatatat tgaaatatta gtttatttta ttgtttttat ttttttcgtt 4200
ttattttttt tttatttatt gtcgagtata ttttattgtt tttcgatttt ttgttttagg 4260
tgatgaattt tgtatttggg ggttgttata ttttttttcg tttgagaaaa atttagcgag 4320
gtaaagtaga gtgttttttt tagatattta gagttttggg gaatgtggtt gaaggtagga 4380
aatggaaata taatttcggt attaaaggtt cgttacgtag tatcgggttt tttttagtcg 4440
cgagcggagg cgagttgtta gggttagagc gtttttgatt gttttttatt atttttattt 4500
attatttttt aaaatttggt ttttttttta tttttagttt taagttttaa tttagagatt 4560
ttagtagtta tagtgatcgg tttggcggta gggggtttgt tttttcggtg tatattttat 4620
gtttaggcgg aggcgtttag ttatagcgtt ttttttttta tatttttttg cgtcggttaa 4680
atttagtttt tttttttttt tttttttttt ttggggtttg tcgggggcgt ttttttgttt 4740
ttttaaattt tttagttttc gttttttttg gtttatttga gatttaatta gtatagattt 4800
tttttttttc gtttcgtttt atttcgaggg gagggaattt taggagattt ggtcggggta 4860
ttacgtgcgt agagcgggtt acgtgggcgc gggaggggtg ttgagaggtg gagagcggga 4920
gtcggcggat tttttaatga agtgttttcg tatgcgtaga gggaatgtag ggagggaggt 4980
ggaagtatta ggaggagggg aggggtggag agaaaagagg ggagggatgg ggggagggga 5040
aataggagcg aggtgttttt ttagttcgtt gtttttggta agtggagttt ttaaaaagtt 5100
ttagtagatt atgttatgac gatttcgttg tttttgtatt tacgttggtc ggagagtttt 5160
atgtacgttt atgaggatag cgcggcggag agcggtatcg gcggcggcgg cggaggagga 5220
ggcggcggta cgggcggagc ggggggtggt tgtagcggag cgagtttcgg taaagtttcg 5280
agtatggatg gtttgggtag tagttgttcg gttagttatt gtcgcgattt gttttcgtat 5340
ttcgtgttgg gtcgttcgtc ggtttttttg ggcgtttttt agggcgtcgt ttatacggat 5400
atttcggttt cggaggcggc gcgttagtgt gtttcgtcgt tcgtattttt tatttcgttt 5460
agcgttattt tgggttacgg ttattttttc gggggtagtt attacggttg tcgtttgtcg 5520
tataacgtga atttgtagta gaagttttgc gtttattatt cgggcgataa atattcggag 5580
tcgtcgggcg ttttgttcgg tgacgatttg ttttttaggg ttaaggagtt cgttttttat 5640
tttagtttcg ttagttttta ttaggcgatg ttcggttatt tggacgtgtc ggtggtgttc 5700

gggattagcg ggtattcgga gtcgcgttac gacgttttta ttttcgtcga aggttattag 5760
tattgggttt tttttaatgg ttgggatagt taggtgtatt gttttaagga gtagtcgtag 5820
ttcgtttatt tttggaagtt ttttttttta ggtaaggaag ggattcgagc gtcgtcgtcg 5880
tcggggattt tttttcgttt tgtttgtttc ggggtttcgc gttttaatta ttttcgtcgt 5940
ttggtttcgg cgcgttcgtt cggttgggtt gtttatggag tcggtcgggc gagttgtatt 6000
gaggaatgcg tcggggaaga aatttgtttc gatacgtttt ttgtagttgt tcggtcgaga 6060
atgaagtaat tataggcgtt cgaaagtcgg gtcgtcggtt ttgttttgtt cggtagttcg 6120
ttttcgtttt tttttgtagg ttttagtttt tcgtcgggtt tttggttttt aaatttgttt 6180
tcggtaaggg atgggggcgg ggtgggttta tagtgttttg gaatttagga taaaattttt 6240
aatttatcga ataattgggg agggcggaga ataagaattt ttattttttt tgatagaatt 6300
cgtaggatcg tttaggtatt agatagtatt ttttaaatag gggattaatt tgttggggtt 6360
tatagaaatg tgagatttat tttttttttt ttttgattta ttttaaaaat ttggttacga 6420
attttttgat tgttgaggga atagaagatt taaaattttt ggagagggag tggagaggag 6480
gttagaattt ttttttagta ttgtaaagtt ttttttgttt ttttggtata ttgagtttaa 6540
atttatagat atttttattg ttaatttttt tatatgtggt cgagaaagag agttagttat 6600
tggtgtttta gatatagaaa gaagggcgtg gggatgagaa atgggagagg aagatttgtt 6660
taagatttgt ttaggggttt ttggagttta gatttaggtt ggggattttt tggttttgtt 6720
tggttaagga tgggaaggga gaatttattt tttttttgtt tcgtttttag ttgaagagag 6780
aaaggttttt gtaaatttta tcgttttttt tgcggataag attagtttta ggttttttaa 6840
aattatatta agagttttgg atttgttttt gtgttgagaa attaggattt cggtttaggg 6900
agtttagtat ttgagtttgt tttttttgta agtagagttt agtgggagag cgtttttttt 6960
tttgatattt atagaggttt ttgggggtgg ggggtttagg gtagttatag atgtttttcg 7020
atagtgttta gtttttcgat tagttttacg tgttaagttt agggatgttt tttttggaaa 7080
gtatagattt aaagattttt taagggtggg tttgaaattt ttttgtatac ggaattggaa 7140
gaagaggtag gtggtttaaa aaaaaatatt gagtgatatt agagagtgta gtaaataata 7200
tttttgtgaa tattatgaaa ttatttagta taatataatt taatattagg gtatgattta 7260
tattaggtat tttgattttt gattgttaga attaaggatg gtgtaatttt agtatatggt 7320
attatgtatt atttattttg tattggttgt aatattttta tataatatga ttttttttat 7380
taattattta atatgaaata tataataata gatttttaga aagataattt atatgtttat 7440
aatatagtaa tgtattttag agagatggtt ttttaatttt ttattttatt aaaatataaa 7500
gttttttagg agattggggt ttagtaggaa tttttggggt agggaagtta atgggttatg 7560
ttaaggggtt aaggggtaag ggattaggtg tttattaaat ttattgtgga ttgggggtat 7620
aggtttagag taatgtgtaa tgggtttagt gtgaagaggt agaagagaag gagttgtttt 7680
atgtatgaat gtggtgtgga gtaggggtga aggattggag agaagttgag aaatattttt 7740
gagtaaaata aatattttaa agatttttag gatataaaat aaaataaaat aaaataaaat 7800
aaaattttat taaaaataaa tatttttttg attatagttt tttagagaaa aatatttttg 7860
aaataaaaaa agtatatttt aattgagatt taatatattt gtaaattatg gtatggaaat 7920
ggatataaaa tttagtatgt tttagtagtt atgtgttttt ataattttcg tgtggaaata 7980
gaattgggtt gatgtatttt agtgagtgta gaggtataga taagaggtag acgaataaga 8040
aattattttt agatattttt tattttttta tttatttaga gattttttag aagtagtgag 8100
attttaagat aatttgtttt gtgtaattgt ggtttgttcg gtaatatgta ggtataaata 8160
ttgttgtttt tttatagtgt ttttggtttt gagaattttt atttgtggta gtttgggttg 8220
gtgtgggaga tggaggtaat tgtttagtaa gatttagata attagggtta agatgtgtcg 8280
tggttttttt ttttttttaa ttatatagat ttttgtttta ttgttttggt tttttttttt 8340
ttttttttac ggtgagtgta gatttttttt tttttttttt tttttttttt tttttttttt 8400
gagagagaga gagaagtaga aattttagga gagaattaga aagggagaaa aagagagatg 8460
gggtttagga gagagaaaga gggagaaaga aatagtgggt tatttggatt atattagaag 8520
tttttattga taagtttttg gatttgggtt gggaagagta gtaagttgga tttttagttg 8580
gataaggtat tttttagagt ttagagtttg tttgtggggg ggcgggtggt taagggtggg 8640
tgggttgtgg ggagttgaat agaggggttt cgaagtttaa aaagaaagtt atggttgagt 8700
attgagatga tttttattta ttttttaaag gtgtttatta ggtttttagg tatgaagtta 8760
ttcgtgtatg tgatttggag ggagattagg gaaggggtga ttgtaagatt aataattaag 8820
tttttattta ttttaggtag tgtacgtata tttttaggtt taagttatta gatgtttttt 8880
tatttttata tattattttt tttttagata aggtagtaga taaagaaagg gggttttttt 8940
gagaaggtaa gattggagtt atttttttag gaggttaaat taatttgtag ttggtttgat 9000
ttaggataaa ttggttaaat taatttttgg tattttatag agaaaaataa ttagtttaaa 9060
ttagttgttt attttttaaa ttgatttttt ttagggttga ttaaatttta ttggaaattt 9120
agagttagaa tttaggaaat taaaattgga taggtaattg tgagagttta aagagagtaa 9180
agtaaaaaat tagggataat tatttggtta atttagaaat aaaaatttat ttttatttta 9240
ttgtattttt ttttagggaa aaggaggtgt tttaaagttg gattgtatta gttggtttat 9300
ttttatttta aaatttattg tattatttta agagttagaa agtttggttt taaaatttgg 9360
ttttgttatt taagttattt tatttatttt gagttttaga tttttaattt tgtaaaatga 9420
gtttaataaa ataattttga aaaaatgttt tgaggattaa aaataataat aataataata 9480
aaagtaataa atttatggta ttattatata ttttttttat tttatttttg ttaataaatc 9540
gttaattttt taaattatgg ttatttttgt aagagtaaaa taaatttgag aattgagaga 9600
tttaaagata atggtcgtgt ttaaaaataa atttttttta atttaaagga tttatattgt 9660

ttaaataaag ttattttgtt ttttagattt ttttaaatta tagtttattt ttatagaggt 9720
agagaaaaat gttaggttta atagtttttt tgagaaattg ttattagaag atagttaaat 9780
gaggttattg gatttcgttt tttatttttt ttttgagtat tttgtttttt tttgtgtttt 9840
attgtgatta ttattttttt tattgtgtat agagtttatg agagaaagta gatttaatgg 9900
ggatgggtat agggaaagtg ttttgtgagg gtgggatggg aataaatatt ttagaaagaa 9960
ttttgtgttt tttttttggg ttgtgtttat agttttaaaa tgtggtagga atgaagggga 10020
aattttaaat atttagtttt ttttattagt ttttatttaa agtttaataa ttatatggaa 10080
aaaattgagg gttgagttat ttagataatt tttttagttt ttttattttt ttttgaaagt 10140
gttgcgtgat ttttattgat agggatgagg gtggagtaga aaatggagtt agaagtgata 10200
tatattggtt ttagtttttg tgaaattttt agagtttaaa gaaatagaat tggtaatatt 10260
ggtttggatg ataggtattt agggtttagg gttatggttg gtgtaaggga tgtagatagt 10320
tttttttagt tgtgaaggaa tggagaggga aggcggttgg aatagaattt gttttgtttt 10380
ggtgggattg tagtgtggat tttttatttt tagagatatt tgatttttta tattttaggt 10440
ttaggatttt ttattttggg tgttaggtgg tttataatat tggattttaa tttttagagt 10500
gtggattttt aagtaggttg gttttatttg gatatttatt ttttatttgt gttttatatt 10560
tttaacgtag ggttagtgtt taaaggcgtg aagatttatt tggtggtttg ggtttattga 10620
agttgatttt ttgtttgtta tgtttttttt gaaatatata atagaatgtt tttttttggt 10680
aacggggtag aatataaatt aataaggatt taagcgattt aattgtagga aggagggatt 10740
tttttgaata tttgaaattt tgtaagtatg gtaggttagg ttggaatttg gagggttttt 10800
tttaaatata taaggatttt aatattgagt gttgtgaaga gttatattgg ggtttgaaat 10860
aaaagaaaat tagtaatatc gaatttatgt ttattttttt aattgatatt ttgtttatta 10920
tggatgtttt tgtattcgtt ttgttttttt aaatattgta ttaaaatatc gttttgatta 10980
ttgagttttt ggcgtttttt tttttaaatt tcgagtttag tggttttatt atagttgtgg 11040
ttttgttaaa ataatagaaa tgaaaagtat tagggagttt tattatgtag tgtaattttt 11100
tggtttcgtt gagtttttgt tagaattttg tgtttaggta gtttcgggtt ttttatttta 11160
gtttagtcgt ttgttttatt ttttttcgtt tgttttattt tttcgtaga 11209

<210> 154
<211> 11209
<212> DNA
<213> Artificial Sequence

<220>
<223> chemically treated genomic DNA (Homo sapiens)

<400> 154

tttgcgggga gataaggtaa gcgggaagaa gtgaggtaag cggttggatt gagatagagg 60
attcgaggtt gtttaggtat agagttttgg taggggttta acggggttag aggattgtat 120
tgtatagtga agttttttaa tgttttttat ttttgttatt ttagtaagat tataattatg 180
atgaggttat tgggttcgaa gtttaaggga gggggcgtta aaaatttagt aattaagacg 240
atattttaat gtaatgttta aaaaagtaaa acgaatataa aaatatttat gatgaataaa 300
atattaatta aaaaaataaa tatgggttcg gtattattga tttttttttg ttttaggttt 360
taatatggtt ttttatagta tttaatatta ggatttttgt atatttagaa gggatttttt 420
aaattttagt ttggtttgtt atgtttgtag aattttagat atttagaaaa attttttttt 480
tttgtagtta gatcgtttgg gttttattta gtttatattt tgtttcgttg ttagaggaag 540
atattttgtt gtatatttta gggagggtat ggtaggtagg gggttagttt tagtgggttt 600
agattattag atgagttttt acgtttttgg atattggttt tacgttaggg atgtgaggta 660
tagatagaag ataagtgttt aggtgaagtt agtttgtttg gaggtttata ttttaggaat 720
tggggtttaa tgttatgaat tatttaatat ttaaggtggg gggttttgaa tttgagatat 780
agggaattag gtgtttttgg aagtaggggg tttatattgt agttttatta aaatagaata 840
aattttgttt taatcgtttt ttttttttat ttttttatag ttgaaggaaa ttgtttatat 900
tttttgtatt aattataatt ttaaatttta gatgtttgtt atttaaatta atattattag 960
ttttgttttt ttaggttttg agaattttat agaaattaaa attaatatat attattttta 1020
attttatttt ttattttatt tttatttttg ttaataaagg ttacgtaata tttttaaggg 1080
gaagtggggg aattagggag attatttgga tagtttagtt tttaattttt tttatgtaat 1140
tattagattt taggtgaaag ttaatgagga gggttgaata tttggggttt tttttttatt 1200
tttattatat tttgaaatta tagatatagt ttagagggaa gatataaagt tttttttgaa 1260
atgtttgttt ttattttatt tttatagaat attttttttg tatttatttt tattgggttt 1320
gttttttttt atggatttta tatataatgg gaagagtagt ggttatagtg agatataggg 1380
gaaaataaaa tgtttaggaa gaaaatggga gacggggttt aatggtttta tttgattgtt 1440
ttttggtgat aattttttag gaaaattgtt ggatttggta tttttttttg tttttgtgag 1500
aataaattat gatttaaaaa aatttaaaaa ataaaataat tttatttaaa tagtatggat 1560
tttttaggtt aaaaaggatt tgtttttgaa tacgattatt atttttaaat tttttagttt 1620
ttaaatttgt tttattttta tagaggtaat tatgatttgg gaaattgacg atttgttgat 1680
aagaatagaa tgaagaggat atgtagtgat attatgagtt tgttgttttt gttgttgtta 1740

ttgttgtttt taatttttag aatatttttt taggattatt ttgttaaatt tattttataa 1800
gattaggaat ttgaggttta gagtgggtaa aataatttaa gtggtagaat tagattttaa 1860
agttagattt tttgattttt aaaatgatat agtaagtttt aaaatggaaa taggttagtt 1920
aatataattt agttttaaaa tatttttttt tttttggaga agaatatagt agggtaaagg 1980
tgggtttttg tttttgaatt agttagataa ttgtttttaa ttttttattt tatttttttt 2040
aaatttttat aattatttat ttaattttgg ttttttaaat tttagtttta aatttttaat 2100
gagatttaat taattttaaa aaaaattagt ttaaaaagta gataattggt ttaaattggt 2160
tatttttttt tataggatgt taaggattga tttagttaat ttgttttgga ttagattaat 2220
tgtaggttgg tttggttttt tggggaaata attttagttt tgttttttta aagaagtttt 2280
ttttttttgt ttgttgtttt atttggaaag gaaatggtat gtagagatga agaaatattt 2340
ggtagtttgg atttggagat gtgcgtatat tgtttaaagt gagtgggggt ttagttatta 2400
attttataat tatttttttt ttagtttttt tttagattat atatacgaat gattttatgt 2460
ttggaaattt aatagatatt tttgaggagt aaataaaaat tattttaatg tttagttatg 2520
gttttttttt tgggtttcgg ggtttttttg tttagttttt tatagtttat ttatttttga 2580
ttattcgttt ttttataggt aagttttggg ttttgaagag tgttttgttt agttagaaat 2640
ttagtttgtt atttttttta atttaaattt aaaggtttgt tagtaaagat ttttagtgtg 2700
gtttaaatag tttattgttt tttttttttt tttttttttt tttaaatttt attttttttt 2760
tttttttttt ttgatttttt tttggaattt ttgttttttt ttttttttta aaaaaaaaaa 2820
aaaaaaaaaa aaaaaaaaaa aaaaaagttt atatttatcg tagggagaaa aaaaaaaaaa 2880
ttagagtaat aaagtaagag tttgtataat tgggagaagg aagaagttac gatatatttt 2940
ggttttaatt atttggattt tgttgaataa ttgtttttat tttttatatt agtttagatt 3000
attatagatg aggattttta gagttagagg tattgtagag agatagtagt gtttatgttt 3060
atatgttgtc gaataaatta taattgtata gatagagtta ttttgaaatt ttattatttt 3120
taaaagattt ttgagtgggt agaaagatgg agagtgtttg aagataattt tttattcgtt 3180
tgttttttgt ttgtattttt gtatttattg agatatatta gtttaatttt atttttatac 3240
ggggattgtg ggagtatata gttgttgagg tatgttaggt tttatattta tttttatgtt 3300
ataatttgta agtgtattaa attttaattg ggatatattt tttttatttt aaagatattt 3360
ttttttagga ggttgtgatt aaaaaagtat ttatttttag tggagttttg ttttgttttg 3420
ttttgttttg ttttgtgttt taggaatttt tgagatgttt attttgttta aagatatttt 3480
ttagtttttt tttaattttt tatttttatt ttatattata tttatatata gaatagtttt 3540
ttttttttta tttttttata ttaaatttat tgtatattgt tttgggtttg tgtttttaat 3600
ttatagtggg tttggtgggt atttgatttt ttatttttta gttttttggt atagtttatt 3660
ggtttttttg ttttaggaat ttttgttgga ttttaatttt ttaagaagtt ttgtgttttg 3720
gtgagataga gaattaaagg gttatttttt taaaatatat tgttgtatta tgaatatgtg 3780
aattgttttt ttagaaattt attattgtat attttatatt aggtagttaa taaaagaaat 3840
tatattatat aaaaatatta tagttaatat agggtaggtg atgtataata ttatgtatta 3900
aaattgtatt atttttaatt ttggtaatta gaagttaggg tgtttagtgt aggttatatt 3960
ttggtattga gttgtgttat attaagtaat tttataatat ttataagaat gttatttgtt 4020
atatttttta atattattta gtattttttt ttaaattatt tatttttttt tttaatttcg 4080
tatgtagagg aattttaggt ttatttttag gaggtttttg ggtttatgtt ttttaaggaa 4140
aatatttttg agtttggtac gtggagttgg tcggggagtt gggtattgtc gggagatatt 4200
tgtggttgtt ttgggttttt tatttttaaa gatttttgta ggtattagaa gggagaacgt 4260
ttttttattg agttttgttt atagggagaa tagatttaaa tgttggattt tttagatcgg 4320
aattttgatt ttttagtata aggtaggatt taaagttttt ggtgtggttt tgggaaattt 4380
gggattggtt ttgttcgtaa ggagagcggt gggatttata ggagtttttt tttttttaat 4440
tggagacgga ataagagggg aatgaatttt ttttttttat ttttagttag ataagattaa 4500
ggggttttta gtttgggttt ggattttaga agtttttgaa taggttttga ataggttttt 4560
tttttttatt ttttattttt acgttttttt ttttgtgttt ggggtattaa taattaattt 4620
tttttttcga ttatatatgg gggagttggt agtggaaatg tttataggtt tgagtttaat 4680
atattaaaga ggtaaggaaa attttataat gttggggagg gattttagtt ttttttttat 4740
ttttttttta gagattttgg attttttgtt tttttaataa ttaggaaatt cgtggttaga 4800
tttttaaaat aagttaggaa aggaaaaaaa taaattttat atttttgtag attttagtaa 4860
attagttttt tatttaaaaa atattgttta gtgtttgaac gattttgcga attttgttaa 4920
agaaagtggg ggtttttatt tttcgttttt tttagttatt cggtgggttg ggggttttgt 4980
tttggatttt aaggtattat aggtttattt cgtttttatt ttttgtcgga agtaggttta 5040
ggggttaaga gttcggcggg aggttggagt ttgtaagggg aggcggaggc gagttatcgg 5100
atagagtaga gtcggcggtt cggttttcgg gcgtttgtga ttgttttatt ttcggtcggg 5160
tagttataga gaacgtgtcg gagtagattt ttttttcggc gtatttttta gtgtagttcg 5220
ttcggtcggt tttataggta gtttagtcga gcgggcgcgt cggggttaga cggcgggggt 5280
ggttggggcg cggagtttcg gggtaggtag ggcggggagg ggttttcggc ggcggcggcg 5340
ttcgggtttt ttttttattt gggaagggag atttttagag gtgggcggat tgcgattgtt 5400
ttttggagta gtatatttga ttgttttagt tattggagag agtttagtgt tggtagtttt 5460
cgacggggat gagggcgtcg tgacgcggtt tcgggtgttc gttgatttcg ggtattatcg 5520
atacgtttag gtagtcgggt atcgtttggt aggagttggc gaagttgggg tagaaggcga 5580
attttttggt tttagaggat aggtcgttat cgggtagggc gttcgacggt ttcgggtatt 5640
tatcgttcgg gtggtaggcg taaggttttt gttgtaggtt tacgttgtgc gataggcggt 5700

agtcgtagta gttgttttcg aaggggtagt cgtagtttag ggtggcgttg gacgaggtgg 5760
ggggtgcggg cggcggggta tattggcgcg tcgttttcgg ggtcgggatg ttcgtataga 5820
cggcgttttg aggggcgttt aggggagtcg gcgggcggtt tagtacgggg tgcggaagta 5880
ggtcgcggta gtggttggtc gggtagttgt tgtttagatt atttatgttc ggggttttgt 5940
cggggttcgt ttcgttgtag ttatttttcg tttcgttcgt gtcgtcgttt tttttttcgt 6000
cgtcgtcgtc gatgtcgttt ttcgtcgcgt tgtttttata gacgtatata aggtttttcg 6060
gttagcgtgg atgtaggagt agcgaagtcg ttatgatatg atttgttgga gttttttaaa 6120
aattttattt gttagaggta gcgagttagg gagatatttc gtttttgttt tttttttttt 6180
tatttttttt tttttttttt tttatttttt tttttttttt agtgttttta tttttttttt 6240
tatatttttt ttacgtatgc ggagatattt tattaagaaa ttcgtcggtt ttcgtttttt 6300
attttttagt atttttttcg cgtttacgtg attcgttttg cgtacgtgat gtttcggtta 6360
ggttttttgg ggtttttttt tttcgaggtg gggcggggcg aaaggaaaaa agtttgtgtt 6420
gattggattt taggtaggtt aaaggaggcg ggagttggga gatttaaagg gatagggaga 6480
cgttttcggt aggttttagg gagggagagg gagggggggg aggttgaatt tggtcgacgt 6540
aaaggggtgt agggaggaga gcgttgtaat tgggcgtttt cgtttaggta taggatgtgt 6600
atcggagaga tagatttttt atcgttaagt cgattattgt gattgttgaa gtttttggat 6660
tggggtttga agttaagggt ggggaagaaa ttaggtttta aaaaataata aatgaagata 6720
atagaaaata gttaggagcg ttttggtttt ggtagttcgt tttcgttcgc ggttggaaag 6780
aattcgatgt tgcgtagcga gtttttgatg tcgggattgt gtttttattt tttattttta 6840
gttatatttt ttaaaatttt agatgtttga aaaggatatt ttgttttatt tcgttggatt 6900
ttttttaagc ggagaggggt gtgataattt ttaaatatag aatttattat ttggggtagg 6960
ggatcgagag gtaatggaat atattcggta ataaatggag aaagaataaa acgagggaaa 7020
taaaagtaat agaatagatt gatattttaa tatgtaatag aaataaaaaa ttttagagtg 7080
aggtttttta aagaaatgcg ggtggtgggg gcgtaagatt tagtttggga aagttaaaag 7140
ttttggatgc gaaggagagc gcgtttttta taggtatata aaagaaaacg tggaaaatgg 7200
gggagattta gtgttatatt tttggaggga tttttttttg gcgttatttt gggggatttt 7260
gtagaattta tttttgtttt taggggtttg ggttaagtta tatgatatag tttttagttt 7320
gaatattcgt aatggagttt ttacggtttt tttaggttat ttttttttag tgaggaatag 7380
ttttatgatt taaaattgtt ttttatttta aagaaaaaat taatattaat ttattgattt 7440
tttaaaagtt tttttttttt tttttttaat tttttaattt gttaggagtt ttaatagagt 7500
tatttttttt taagggagta ttttcgtgga attgagttaa gttttttttt agtataggat 7560
ttttttgaga aatcgtaatg aagagagaaa aataagaggg gaaaggggtt gtagcgtttt 7620
tttttttttt ttttttgtta taggttttaa atgttgagtg ttcgtgtgta gttagcgtat 7680
agattatggt tatagagtag cgtttcgttt agttagattt attgtatttt ttaagtggag 7740
agtaaatgga taggtttgta gaaatttttt cgggttattt tttttttttt tgtggggaga 7800
aggtggtgtt tgacggttga gagtattcgg atatcggtga gttaggatag taatttttat 7860
ttgcgtgatt tttatttaga agggttagtt tagagtttag aaagtaggtt gcgggttggg 7920
ttggtattag gaggaaaaat ataatagaaa gagagttaag atataaggag attagttcga 7980
gtgtgtattg agggtaatga gggaggggcg gggagagatg agtttagtgg aagatagggt 8040
tttgttggga gtcggttttg ttaggaaatt gtttattttg ttgttggagt ttaaggttaa 8100
ggtaaaacga ggagagtaaa tagggtttgg ttaagtttag attgggttgt atgacgttgg 8160
tttttttgtt taaggtttgg ttggtggggg tgggtgggtg gagacgtttg ttttttttta 8220
gtcgcgggta gtagaagtgg aatttagatt ttggtttttt tttttgtgaa gtgttgtttt 8280
taggtggaag tagtggggcg ttgtggatag ggttttgtag ggttgttagg agaaataaag 8340
ggtttgaaac gtttatttta tttttggggt ttttttttcg gttaggttta acgtcggatt 8400
cgtttttcgc gggttaggat ttaaagatac gtttttagtt tttgggagta ggaattagaa 8460
tggcgggagt gggaattaga acggcgtgaa ggatttgggt gtagggcggt taagtaataa 8520
ataaaattaa tagtatggtt ttttttaggt ttatatgagg gtattaatta tttttattgt 8580
aatgagatat agatttaatt aattcggtaa ttttataatt ttatttatta ggagtattat 8640
gcggttaaaa tttagtggcg agaagaaaga aagggaatta tagttttttt ttttaagaag 8700
attagtcgaa gttgtaggtt tttaaagcga tttggaggag tgggaaggtt ggaggggaaa 8760
gtttgggtta agggcgttaa aggcggttgg ggaagttcgt ttgttttgaa taattttttt 8820
tttttttgtt tggtttttta agatttttcg cgtttgtaaa cgtagttttt aagtttgggg 8880
cgagtcggga tcgggaaggg aggcgtcgag gtaagagttt gtaggagggc ggtggaaagt 8940
aaagttttgt tggttaatta attttaagtt tggggtggtt tttttttttt ttttcgaaaa 9000
aaaaatgtgg atttcgttga gatataagta aaattaaatt aaatatttta ggagaatatg 9060
tattattaga aacgtagtaa ttatattttt aaggcgattt cgttttagaa agttttttta 9120
gggtttggcg ttgaaatttt cggttgcggt gtttattcgg gcgcgtttcg tcggttttcg 9180
gggatgaggt agtgaggttt gattgtggat gaagtttaga gaaaatttag ttgttggtat 9240
gcggagaaag acggagtatt taattatacg aaaggaaaat ggaaaacggt tttttagttt 9300
cgtcgggaag tttcggtttt gttttcggtt gtttttttaa agggattttt ataatcgcgg 9360
agcggtagaa gttattttgt ttttagttat agtttatgaa ttaagttttc ggttaagttt 9420
aattttgaaa tgtttttttt taaatgattt aatatagaaa ggagatattt aatatgtttt 9480
tttacggaaa gcgtttgtgt atgtgcgtgt gcgcgtgcgt ttgcgtgttt gtgagtttag 9540
ggatgggtgt aggttgtttg tatcgtttta gtatacggtt ttgtggatat agtttaaatg 9600
agtttttaaa gatatttagt tttttgttta aatttttttgt atgtaaataa aggtttttgt 9660

ttgtttgttt gtatatattt ttaatttata tgtttttttt acgttattta taaagatatt 9720
ttttaggaaa atatagaaaa atgaagaagt agatgtttgt gtttttttga agtcgagggg 9780
tggaatttat gttttattgt atttaagacg aagttagagg tgtaatttag taatatttgt 9840
aagcggaggg tgtaagtttg atgtatatat ttgagggttg ggtgtagttt taatgttagg 9900
ttagggaaaa ggatgaaaga aatggcgttt ttgtgttttt tatttaatgt ttagattttt 9960
ttttttgaaa attaggtgtt agatgatttt tttttttgta gttttttttt ttgttttttt 10020
tatatgggcg gggggcgggg gggagtttaa agcgttgaag tttttgtttt tttaattcgt 10080
ttttaatggg agaaatttta gttttaaaaa aggtgggggt gggttgaggt tgtaggagaa 10140
taaagagttt tattattatg gggtttggat tttggtttag atttggggtt ttatagggta 10200
ggtaaagggt ttgggggttt ttttttgttt aaggtttagg ttgtcgatgg aagaggagat 10260
ttgtggagga tagaggaaga tagggtttga gttagttaag aaagggatgt ttgagagagg 10320
ttatttatag gaagggattg ggggtttaga aagggtatat gagggggtat atttttgagg 10380
gtgtgttagt gtgggaagat ttttttatat gtttggagaa aatttttgat gggttgtaga 10440
gggtgatatt ggtagagaag gaaagaaaat tggttgattt tgtagttttt tggatttgtt 10500
taatattttt tttttttttt ttcgtttttt ttattttttt gggttttagg aggttttttc 10560
gtttttgtag ttatggattt tgtagatatg gatgtttttt tttaggtttt tggatttttt 10620
tggaatttgt tttattttga tttttttatt tttatagttt agtttttgaa aatggtaatt 10680
aggttttttt tattagtttg ggaatttatt ttttggatag tagttgttat tttggtattt 10740
tttattttta gtgttttttt tttttaggtt tttagtttga gggaagatgg gattagggta 10800
gttgaagtat tattttgttt tttgttttag agtttttcgt ttttaaattt aaatttgttt 10860
atttttaggt tttatttatt tttttgtgat agatattagg gaaatttatt tttttttgtt 10920
gtttagttag gatttgtagt gttggttaag gtttggggtt attcggaaga tttttttttt 10980
tttggttttg tagggattta tatagggggt ttaagtttag gttattataa attttttttt 11040
ggtttttttt ttaggatgtt tttagttgta gattttgttt ggtggtgtat tgggtttggt 11100
cgggggacgt taagtggttt tttttatgga gaattgttag aatgtttttt ttggaggttt 11160
ttggagtttt taattttagt tttttttttt ttatagtttt ttcgcgttt 11209

<210> 155
<211> 6484
<212> DNA
<213> Artificial Sequence

<220>
<223> chemically treated genomic DNA (Homo sapiens)

<400> 155
   tttttttttt tttttttttt ttttttttag ggttttttgt ttagaaaaat ttttttgatt 60
   ttttttagga atttgatgga aatttgaagg agatgtgggt tttttttttt tttttatttt 120
   tagaagggta gataggagtt tgtagtcgtt tttaaaattt tatttaatta ttttatggtt 180
   attcgggttt atgttttttt ttttttcgtt gtttgatttt tattttgttg ggttcgtttt 240
   tttttgagtt gtattagtgt tagtgtttag aaattattat aattacgaaa ataataataa 300
   taaattttta atatattatt taaagggaat ttgtaataat tttgaaaaag aaaaagagaa 360
   aaattttaaa attttgttat aggagaaaaa aagagaaaaa aataaaaatt aaaaaaaaaa 420
   aaaagaaaga aagaaatttt tagcgtattt tattattatt tatagaaaga aattttgttt 480
   tgagagtatt tgtaatgcgg ttttgttgtc gtttgttgtt gtttatttta ttaagaaaat 540
   ttaataattg agattgttta gttcgtcggt tttgtgcgtt tttattgtgt ttttaatttt 600
   agtagagtag aattaaattg tattgaatgt atagttaatt ttgttttgaa ttttttgttt 660
   atgtaatgtg ttcgaaagaa aaaaatgtta aaaatatatt tataataata attttttgtt 720
   atttgttttt atgtttagtt atgaatgtag attttgtgtt tcgatagttt tgtttttggt 780
   ttaagtattt tgtattgtat acgtgagtta taataataaa aagaagaaga aaaataatag 840
   aagttggaat gatttgtttt ttttttatag ttttttttta ttttttattt tttttttttt 900
   tatttttttg tttaattttt tggaaaaggt ttgttttatt gttttggttt tggggtttag 960
   aaatttacgt ggagagaagt ttgggaattg agaaagtatg ttggtttggg agggtaggtg 1020
   aaaggggtga ggggttgaga ggaggaagaa aaggagtagt ttattggttg agagagtaag 1080
   gagtttatag gtatgttttt gggggaattt tggttttaaa taaagtgggt ttttagacgt 1140
   taggatttta atgggaagaa agattttttt atttatttat tttttttttt tttagttttg 1200
   ggttgggttt agataaagaa tttgttttat gtaattatga tttttttttt tagggtttgg 1260
   ggttgaggat gagatagtgg gatttttttt gattttcgta tgttttttgt ggttagattt 1320
   tagtatttag tatttagtat tagaataatg aatcgggtgg ggatgtgtga ggtttagggt 1380
   aattggttgg tttgtttgta gggtttgttt gaagatgggg gatgttaaga atggagggtg 1440
   aggaagttta agtttgaagg agaggagaaa ggaaggggaa agtaagtgag ttcgtaggta 1500
   attagtttga gtttatattt tgaaatttat atttaatttt ttgtatagga agattgttat 1560
   tattgttatt tagttcgttt gtatttattt taatttttgt ttagacggtt taaagtttat 1620
   tttttatttt attttggtaa ttggttagtt tatttagttt ttatagtgtg ggttattggt 1680
   ttggaggatg ggtttgttga gtggatattt tgttatttta gttatagcgg ggtaagaggg 1740
   cgtttttatt ggtattttta ttttttaaaa tgaagaattg aaggtttagg gatttagggg 1800
   gtagaagggt tttttatttt tttttggaga gttttggatt taaaaatttt gtttatgtta 1860
   ataagatttt ggttaaataa ttttaaattt tagagatata agttttttga tttagttagg 1920
   agaggggaaa agggagggga gggatttttt ggttttaaaa attagatttt cgaaggttga 1980
   aatatttttg aggttagatt tttttttttt tttttttttt tttttttttt tttttttttt 2040
   tttttttttt tttttttttt tttttttttt tttttttttt tttttttttt tttttttttt 2100
   tttttttttt tttttttttt tttttgtttt ttttattttt tatttgttta gtttttggtt 2160
   aatgattttt ttagatttag gtttaggtta atttttgttt tgtggaattt tatttaggtt 2220
   ttagttattg gtttttacgt aaatcgggtt tgaaaatcgg gtttgagtag agaattgagt 2280
   cgggtttttt ttataggggg atttttgtag ttcgagaaag gttgtgaatt ggaagtatag 2340
   gttgtaggta gttggggtag ttttgagtag gagtttcggg aaattttttt taatttgggg 2400
   tttttgttgt gagtagcggt attagttagg taatttgttt ttagtgggag atatcggtta 2460
   tttattaggg aagttagaaa agaggttatt tcgtttagat tttaggtttt tttataggcg 2520
   tgttttttat atttcgaggg atggttgagg tttcgtttcg tttttaaacg tggaattatg 2580
   attgttatta ataaaaatat ttggtttagt agtttagcgg aatgaattta gtattaaatg 2640
   gtttcgaagg gtttcgtcgt ttttttttta tattaaatgt gttatttatt ttttgttttg 2700
   tttagtaatg ttcgtggttt taagttcggc gcgtcggagg gcggggtgcg tttggcgttc 2760
   ggttttattc gagatgttag gtatggagag ggttaagtcg agggagtttt agttattttt 2820
   ttatgtagta ttcgtttttt tatttttttt gtttatcgtt tttgtttttt cgtttggaaa 2880
   gaaatggagg taaaaattcg ttttatgggg gaacgtaatt gtgagcggga tgcgtttttt 2940
   ttagaatcgc gtttttttaa atgttttcgt cgttttatta tcggaatggg gattattcgg 3000
   ttgttgtaga taggattttt ttttttattt tttatttttt tattagttaa ttaaagtggt 3060
   tttcgaaagt tatttgtgat ttgtgaaaaa tagtattttt taaaatgttg agttgaagta 3120
   tttcgtttat tgttttttga atttattatt ttaattttaa tcgtagagtt ggaataatgt 3180
   agttattaag ttggggggta aattcgtttt tttggattaa attttttttt ttttttattt 3240
   ttttattgta ttagttaagg aaggtgcgga tttgtaagag atttgggggt gggtgagaga 3300
   ggtagtgagc gttttgaagg gttttttgag ggatttttgg ataggcgagt ggtttggttt 3360
   ttttagtttt atttttttta gataagatgt tttaatgatt gtttgttggt tgggaaagtt 3420
   atttcgttta attagggaga atttaggata ggttgtgtta tagtttagaa gtaaaatttt 3480
   cggtgagttg ttcgtagcgt cgttttattt ttttcgttgt ttattgtgaa aagttgggtt 3540
   agcgtttttt tgtcgtaatt tttaattatt tgttataaaa tttatgggtg ggtttgtgaa 3600
   aagaaacgag gtttttgtat tcggtttagg gaattgtttt ttttattatg gaggaagttt 3660
   ggggaaggta tgaggtattt ttttttggtt agggttgagg tatttaatta ttgagatttt 3720
   tatgttagtt tgatgcggga ttaagtggag agatgtcggt ttttaaattt cgagttcgtg 3780
   gggagagtga tttgtttgta attttttttt cggtaaatag tttttatggg ggaatttatt 3840
   ttggatttat ttttaaaatt aggggtttgg aaaaataggt tgttcgaaat tttagtatag 3900
   aaggtggaat ttagggtagt aaaaggttat ttttttaggt ttcggtagta tattttagtt 3960
   gtttagatat tgaattagga aatttttttg agaaaggtag gtgggtatgg gtaatttgat 4020
   acgatttaaa aggtttattg ttttgtattt tttttcgtaa aaaggaagta gagattaaga 4080
   ttttgggttt taattttatt tttaattgta aaaagatttt aaataagtta ttatttgaat 4140
   tgggaaggga aattagggat ggttttgggg gaaaatgtta ttttttttaa gaaagggatt 4200
   tcggggatgt gggtcgtttt gagggtttta gaagttgatt ttaattttta ttttttttgt 4260
   tgtattgtag gattgtttag ggtaggttta gttttttagg ttttttagat tattttaggt 4320
   taggtttggt tgggtggttg ttagtggttt tttattattg tttttgttta gggtggagta 4380
   ggagttttta ggggtacggt atagagggga tgttgttttt ttattaaaaa aaaaaaaaaa 4440
   aaaaaaaggt aggggtaggg aggtattttt ttaatttttg taatttatta gttattattg 4500
   ttttaaggtt ataaattttt gtaaagtttt gaatttcgtt tgatttaatt ttaatgtttt 4560
   tatttattaa ggataatatt taaaattcgg taagttagga gaggtaatcg atttttagtg 4620
   tttgaagttt tttttcgttt gggcgttcgt ttttagtttt ttttgcgttc gtttgtagta 4680
   gtttggttcg tggggagtgg gcggtgggtg ggggtagggg tggggtgggg gttcggggat 4740
   gtttttttta tgagagaatt gggtaaatat ggcgatcgcg gcgtttattt gtatacgtta 4800
   tataaatgta tcgtattttc ggttgtttgt agaaaattta tagttgagta ataaaagttt 4860
   acgatcgatt tataagttgg attggttata agaagttatg tggattttat ttatgaacgt 4920
   gaatttttta ttgtggtttg ttcgtttcga gcgtttcgta gaatagtttt ttttgtaaga 4980
   gtttaattat tgttagggaa atttgttttg ggcgtttttt ttattagtag tatttttttt 5040
   aaattaattt gattaataat tatttttttt ttatttaatt tttttttttt ttaggtggag 5100
   ttgtcgaagt tgggggtagt tggggagggt ggggatggga ggggagagat agaagttgag 5160
   ggtatttttt tttttttttt cgattttttg gtttttaagg ggtaggagga atgtaggagt 5220
   aggagttgag tttgggagtt gtagatgttt tcgttttttt ttttttttag gttttttttt 5280
   ttgttttttt tttgtaattt tttttaattt tgtttggttt ttggatgatt ataattattt 5340
   ttatttttga atttatataa agtatatgtg tgtgtgtgtg gagttgagat aggttcggta 5400
   gcggtataga atgagggaag acgagaaaga gagtgggaga gagagaggta gagagggaga 5460
   gagggagagt gatagtagcg ttcggtaagt gtttttttat tggtttaaat atgtaattaa 5520
   tggttcgtgg cggttttaag aataggatag tagcggtttt ttttggttag ggagaagagg 5580
   gggttggcga gagggtgggc gcgtagttaa ttatggggag gaaaattggc ggggggagag 5640
   aaacggggtt aaattggaat atcggtaatt ggtagtgggg cgattgtgtt gttttgatgt 5700
   tattgtgtgt taagttgggg gtggggaggt aagggaatag tatttgtggg gtttaagtcg 5760
   gcggtttagg gcgttagttt tttgttgttt attgtgttag tatttgttta ttatagattt 5820
   tcggtttttt ggttaatgga attgggtcgg agcggaattg gaagtttaag ttttaggtag 5880
   gagagagaat ttagaggttg tgtttattgt tttttaagta aagttagttt tttaattttt 5940
   aaataaattt tttagttttt tttttttgtt tggtagtttt ttttttttta gagagcgcga 6000
   ttgttagagt ttatatatgc gtagtgtggg tttagggtcg ggtcgtcgag taggaagtcg 6060
   gcgtagttag gcggtcggcg gggtttgtta attggtaatt aggggggagg ttggtggttg 6120
   gtgcgcgtta gtcgagagga gagcgtttgt ttattttttg ttttcgtttt tattcgggcg 6180
   gatggaaggg tgggaggtgt tttgcgttgg gtggagggtg gaggttgtag ggtgggggtg 6240
   ggggatgttg tatttaaaag ttattttgtg tttagagaaa agaggtttat cggttttttt 6300
   tttcggggtt cggcgttttt tatttttagt cgcggttatt ttagtcggga tgtttattgg 6360
   atcgggatgt tcgttcgtta cgtatggttg ttttgggtta ggatttgttt cgtttcgggg 6420
   ggaggtggaa atggggaagg ttttaggttg ggcggggata ggtttagttt ttttggtaga 6480
   gttt 6484

<210> 156
<211> 6484
<212> DNA
<213> Artificial Sequence

<220>
<223> chemically treated genomic DNA (Homo sapiens)

<400> 156

agattttatt aggaggattg ggtttgtttt cgtttagttt agggtttttt ttatttttat 60
tttttttcga ggcgaggtag gttttagttt agagtagtta tgcgtggcga gcgggtattt 120
cggtttagtg ggtatttcgg ttgggatggt cgcggttggg ggtgaggggc gtcggatttc 180
ggaagggaaa gtcggtaggt tttttttttt tgagtataag atggtttttg agtatagtat 240
tttttatttt tattttataa tttttatttt ttatttaacg tagggtattt tttatttttt 300
tattcgttcg agtgggggcg ggagtagggg gtgggtagac gttttttttt cggttgacgc 360
gtattagtta ttagtttttt ttttaattgt taattaatag gtttcgtcgg tcgtttagtt 420
gcgtcggttt tttgttcggc ggttcggttt tgggtttata ttgcgtatgt gtgagtttta 480
gtagtcgcgt tttttgggag ggagggggtt gttagatagg gaggggaagt tggaaagttt 540
gtttgggggt tggggggtta attttgttta gggggtaatg ggtatagttt ttaagttttt 600
ttttttgttt ggagtttgag tttttagttt cgtttcgatt tagttttatt agttaagaga 660
tcgaagattt ataataagta ggtattgata tagtgggtag tagagagttg gcgttttaga 720
tcgtcggttt gagttttata ggtgttgttt ttttgttttt ttatttttaa tttggtatat 780
agtgatatta ggataatata atcgttttat tattagttat cggtatttta gtttaatttc 840
gttttttttt tttcgttaat tttttttttt ataattaatt acgcgtttat tttttcgtta 900
gttttttttt ttttttaatt aagagaagtc gttgttgttt tatttttgaa atcgttacgg 960
attattagtt atatatttga attaataagg aaatatttat cgagcgttgt tgttattttt 1020
tttttttttt tttttgtttt tttttttttt attttttttt tcgttttttt ttattttgtg 1080
tcgttgtcga gtttgtttta gttttatata tatatatata tattttatat aaatttaaaa 1140
ataaaaataa ttataattat ttaaaagtta aataaaatta aggagagttg taagaagggg 1200
gtaggaggaa gagtttggga gagaggaggg gcggaggtat ttgtagtttt taagtttaat 1260
ttttgttttt gtattttttt tgttttttgg gggttagagg gtcgggaagg aagagagaga 1320
tgtttttaat ttttgttttt ttttttttat ttttattttt tttagttgtt tttagtttcg 1380
gtaattttat ttgggaggaa aaaaaattaa atgggggaaa aataattatt aattagatta 1440
atttaaaaaa aatattgtta atgaagggag cgtttagggt aggttttttt ggtaatggtt 1500
aggtttttat agggaggatt gttttgcgga gcgttcggaa cggataaatt ataataaaaa 1560
gtttacgttt atggatggaa tttatatgat tttttgtggt taatttaatt tgtgagtcga 1620
tcgtaaattt ttattattta gttgtaaatt ttttgtaaat aatcgggaat gcgatgtatt 1680
tgtgtagcgt gtgtaaatgg gcgtcgcggt cgttatgttt atttaatttt tttatagagg 1740
gagtattttc ggatttttat tttattttta tttttattta tcgtttattt tttacgggtt 1800
aggttgttgt aggcgagcgt agaaagagtt gagggcggac gtttaggcga aagaaagttt 1860
taagtattga gggtcggttg ttttttttgg tttgtcgagt tttaaatatt gtttttaata 1920
aatgggggta ttaaggttgg attaggcgga atttaaagtt ttgtaaagat ttgtggtttt 1980
gaggtagtga tggttggtga attgtaggga ttgaaagggt gtttttttat ttttattttt 2040
tttttttttt ttttttttta gtaagagaat aatatttttt ttatgtcgta tttttgaaag 2100
tttttatttt attttagata ggaatagtag tggaagatta ttggtagtta tttaattaaa 2160
tttgatttgg agtggtttga agaatttagg ggattagatt tattttgggt aattttgtag 2220
tgtagtagga gaggtgagaa ttggggttaa tttttaggat ttttaaaacg atttatattt 2280
tcggggtttt ttttttagag agaatgatat ttttttttaa gattattttt agtttttttt 2340
tttagtttaa atggtggttt atttgaaatt tttttataat taaaaataaa attaaagttt 2400
aagattttgg tttttatttt ttttttgcgg ggaggggtgt aaggtagtaa gttttttgga 2460

tcgtgttaga ttgtttatat ttatttgttt tttttagaaa aattttttga tttaatgttt 2520
gggtagttgg gatgtattat cgaggtttga aaaaataatt ttttattgtt ttgaatttta 2580
ttttttgtgt taaaatttcg agtagtttgt ttttttagat ttttaatttt gaaggtgagt 2640
ttagaataaa tttttttata gagattattt gtcgggggag gggttgtagg taggttattt 2700
tttttacggg ttcgagattt gaaggtcggt atttttttat ttggtttcgt attaagttgg 2760
tatgggaatt ttaatggttg ggtgttttaa ttttgattaa gaggggatat tttatgtttt 2820
ttttagattt tttttatagt aagaaaagta gttttttagg tcggatgtag ggatttcgtt 2880
tttttttata aatttattta taaattttat aataggtagt taaaaattac gataaggaag 2940
cgttggttta gttttttata atgggtagcg gggagaatgg gacggcgttg cgggtagttt 3000
atcggaaatt ttatttttag attgtggtat aatttgtttt gaattttttt taattgggcg 3060
gggtaatttt tttagttagt aggtagttat tagggtattt tatttgggga agataagatt 3120
ggggagatta aattattcgt ttatttaagg gttttttaag gagtttttta ggacgtttat 3180
tatttttttt atttattttt agattttttg taggttcgta ttttttttgg ttgatgtaat 3240
gagggagtgg ggaggaaaaa aaatttaatt tagagaaacg aatttgtttt ttaatttaat 3300
agttgtatta ttttagtttt acggttgaga ttagaataat aaatttaagg agtaatgagc 3360
gagatgtttt aatttagtat tttgaaagat attatttttt ataaattata aatggttttc 3420
ggaagttatt ttgattggtt aataaaaaag taaagaatgg gagagaaagt tttatttgta 3480
gtagtcgaat ggttttattt tcggtaatgg gacggcggga gtatttggga ggacgcgatt 3540
ttaaagagag cgtatttcgt ttataattac gtttttttat aaaacgggtt tttattttta 3600
ttttttttta aacgagaaaa tagaggcggt gggtaggagg ggtgggggag cgggtattgt 3660
atgagggggt ggttggggtt ttttcggttt agtttttttt atgtttggta tttcgagtaa 3720
agtcgggcgt tagacgtatt tcgtttttcg acgcgtcggg tttggggtta cggatattgt 3780
tgaataggat aagggataaa tggtatattt ggtgtgggga aaaggcggcg aggtttttcg 3840
gagttattta atgttgggtt tatttcgtta ggttattggg ttagatattt ttattaatag 3900
taattataat tttacgttta aaaacgaaac gggattttag ttatttttcg gagtatggag 3960
gatacgtttg tgggaaggtt tgaggtttga gcgaaatggt tttttttttg gtttttttgg 4020
tgggtagtcg gtgtttttta ttgagaatag gttatttggt tggtgtcgtt gtttatagta 4080
gggattttag gttagagaag atttttcgga gtttttgttt aaagttgttt tagttgtttg 4140
tagtttgtgt ttttaattta tagttttttt cggattgtag gagttttttt gtgaaggaag 4200
ttcgatttaa ttttttattt aggttcgatt tttaggttcg gtttgcgtgg ggattagtga 4260
ttgggatttg ggtgaaattt tataaaataa gagttggttt gggtttaggt ttgggaaaat 4320
tattgattag gagttagata gatgaggggt ggggaggata gagagagaga gagagagaga 4380
gagagagaga gagagagaga gagagagaga aagaagagaa gagaagagaa gagaagagaa 4440
gagaagagaa gagaagagaa gagaagagaa aaagaaagaa agaaaatttg gttttaaaaa 4500
tattttagtt ttcgggggtt tggtttttaa gattagaaga tttttttttt tttttttttt 4560
tttttttgat tgagttagaa gatttgtatt tttggaattt gggattattt gattagaatt 4620
ttattggtat aaatagaatt tttaagttta ggatttttta gagagaagtg agagattttt 4680
ttattttttg ggtttttgag tttttaattt tttattttgg ggaatgaaga tattaataaa 4740
gacgtttttt tatttcgttg tgattggggt ggtaggatgt ttatttagta ggtttatttt 4800
ttaggttaat ggtttatatt atgggggttg ggtaagttga ttaattgtta aggtgaagtg 4860
gggagtggat tttaaatcgt ttgaatagaa gttggagtgg atgtaagcgg gttgaatgat 4920
agtgatgata atttttttgt gtagaggatt gaatgtgaat tttaggatgt gggtttaggt 4980
tggttgttta cgagtttatt tatttttttt tttttttttt ttttttttta ggtttaagtt 5040
tttttatttt ttatttttag tattttttat ttttaggtaa gttttgtaga taggttagtt 5100
agttgttttg agttttatat atttttattc ggtttattat tttagtgttg ggtgttgggt 5160
gttaaggttt ggttataagg ggtatgcgga gattagaagg aattttatta ttttattttt 5220
agttttagat tttggaagaa aaagttatag ttgtataggg tagatttttt gtttgggttt 5280
agtttaaggt tgaggaggag gagggtggat gagtgaggaa attttttttt ttattgggat 5340
tttggcgttt ggaaatttat tttgtttgga gttaagattt ttttaagggt atatttatag 5400
attttttatt tttttaatta atggattgtt tttttttttt ttttttttaa ttttttattt 5460
tttttatttg tttttttagg ttagtatgtt tttttaattt ttaggttttt ttttacgtga 5520
gtttttgaat tttaaagtta gagtagtgaa gtagattttt tttagagagt tggatagggg 5580
gataaggaga gagaaggtag agaataggga agagttgtgg agaaaaagta agttatttta 5640
gtttttatta tttttttttt tttttttatt attatgattt acgtatataa tataaagtat 5700
ttggattagg aatagggttg tcgggatata aaatttatat ttatagttgg atataaagat 5760
aaatgataaa aaattattat tatagatata tttttaatat tttttttttt cgagtatatt 5820
gtataaatag agaatttaag atagagttaa ttatatattt agtgtaattt agttttattt 5880
tattggggtt agaagtataa taaaagcgta taggatcggc gggttaggta gttttagttg 5940
ttgggttttt ttagtgaaat aagtagtaat aaacgataat aaaatcgtat tataaatatt 6000
tttaaagtag gatttttttt tataggtagt gataaaatac gttggaggtt tttttttttt 6060
tttttttttt ttttgatttt tatttttttt tttttttttt ttttatagta ggattttaaa 6120
attttttttt tttttttttt taagattatt gtaggttttt tttaggtagt atgttaaaga 6180
tttattatta ttattttcgt gattatggtg atttttgagt attaatatta atgtagttta 6240
gaggaaggcg ggtttaatag aatagaaatt aaatagcgaa ggagaggaag gtatgggttc 6300
gagtgattat gggatggtta ggtaggattt tagaggcgat tgtaggtttt tatttatttt 6360
tttggaaatg gggggagggg aaagatttat atttttttta ggtttttatt aggtttttag 6420

agaaagttaa gggggttttt ttaagtaggg agttttgggg agggggaggg ggagggggag 6480
ggga 6484

<210> 157
<211> 6552
<212> DNA
<213> Artificial Sequence

<220>
<223> chemically treated genomic DNA (Homo sapiens)

<400> 157
   attttatttt ttaggttatt taatagttat tatgtatgtt ttgttttgat tttgttaaaa 60
   atagtagttt gtatatatta gatataatat atataaatgt tttgagtgaa aataaatatt 120
   taggtttgag ttaagttgtt aaggagttga agttaatttt ttaaaatgtt ttggatttta 180
   ttttaatgtt ttttaggtta ttaaattcgt tttttttttt tggttattgt tttatcgcga 240
   gtgttgattg ttcgttaaat gagaaaatgg ttttcgaggt agttaggaaa tggtgtgtaa 300
   aagtttataa attaaatatg tttggaatat ttaaaaggga aggattatgg tagatttgag 360
   tgtgttttat atattaaggg gatttttagt ttgttgtaag ttgggatttt tatatttagt 420
   ttaaattttt aaattttaaa gtttgttggt attcggggtt tgtaaagatt tgaggttttt 480
   tagatgttaa aataaaacgt ttgtttttat cgaatttggg tttgattttt ttggaatcgg 540
   ttatgttttg ttaaattttt gatattagta ggtttattag gcgtttagta gatttttttg 600
   ggaaatgggg ttttaaaatt ttttagttta tttgaatttt tagtttagtt taggaaattt 660
   agggtttgtt tgttatttat ttttttgtaa aaataagttt ttagttggtg gaggtggaaa 720
   ttagttttta gttagaggaa gaattagtaa aattattaaa ttattaatat tgaatattta 780
   atattttaat tttttttttt agaagtaaat ttggtgtata attgaaattt ttaattgttt 840
   tttaattgta tatatatata taattatata tatttatatg tatatatatt taataggaga 900
   gaaagaagta agagttgtta aggttttatg gtttaaaagg attttttatt tggttgggta 960
   tatagaggcg atggtattta gagtaggatt tttaagtttg agtttgtatt tagaggtttg 1020
   tttttttaaa aataattgaa taaaggttga agtcgtattg agcgttcgga aggttgggga 1080
   aaggaggagg aaagtatatg aatttagatt taggttttaa gatagaaaat acggaggtcg 1140
   ttttttttta aaatatatta ttaaattatc gtgttttttt tttttagttt gggatgagtg 1200
   gggggagggg atatttatgg agattatttt ttaaagaaaa ttaaattatt ttaaatataa 1260
   aagcgtattt tttggtttag tagttttttt taaatttatt tcgtttgtta ttttatataa 1320
   atataaagat tttggtgttt ttttttatag ttcgggtttt cgtagagatt tggaattatt 1380
   aggaaatttg ttaaaaaaaa aaaaaaaaaa aaaaagtcga agagaaagga cgttaaaatt 1440
   tgagtgttta atattttttt tagaaagata aatgtgtttt ttgaaatgtt gtagtttaag 1500
   cgggtttttt gattaagaaa atagttttaa aacgagaaaa aaatagtttt atttggtatt 1560
   taattagttg ggaatttaga gggcggcggg gaagggggga cgtttttaag gtttgggggt 1620
   ttttttttag ggaggcgggt tgaggaggtt ttcgggatgt cggcgggtgg aagagaaatt 1680
   tggaatcgtt gtaaatattt atgtgggggg atgttttttt attttttatt cgtttttttt 1740
   agttgtattt tttttttagt gatagtgggg gagggtaagg cgattgtttt attgttttta 1800
   gataaaggtg atttttttat attttgtttt tttagtcgtt tgatttaaga agtttatttc 1860
   gaaggttttg tagggaagga attggttttg ggtgtttttt ttgtattttt tttttttcgt 1920
   tttttaaaag agtataaata attaaagttt ggtagggagg aaaagttttt ttgtagaatt 1980
   gtagtggtaa taaatgaaat gatttagaat ttttttttta gttagttttt acgagagttg 2040
   ttagatagtg tttgtttacg ttttttagat attaggggcg ttttgataaa gttaaggtta 2100
   agttagtgtt ttattttggg tgatttaaaa ttatcgtatc gcgttttggc gttgtgcgga 2160
   aagttttttt tcgcggagcg gttggttgcg tttcgaaggt ttggtgcgtt ttttgcgttt 2220
   cgggtggggt cgcgtaggta aggataaggt tatagagtcg ggttttttac ggtaggtttt 2280
   cgagcgggac gcgcgggttc ggaggttgcg gttttttttg ggtttgggga atgggggtag 2340
   gaattaggag ggagttgggg ttaaagagtt aagcgggttg ggattggaat gaaagcgttt 2400
   tgggttgtgg agtgggtcgg ggggtaaggg ttcgcgttaa ggagtcgaaa ggggtcggtc 2460
   gttttttttt tttatgtatc ggcgcgttat tgtagatggt ttattttttt tcgttaaatc 2520
   gttgttttcg tcggagttgt cgtcgttatg tcgttgaatt tgaatggttc gttttttttt 2580
   tttttttttt cggcggttag taggttttat attgggggcg tcgggttgtt tgtattttat 2640
   ttcgtgtcgt tagtagagag aaggtgcgag aattttgcga atttttagat atataaagat 2700
   tcgggttgaa gttttcgtag ttgttttggg ttttgagtta tcgtagggtt agtgtgtagc 2760
   gtttagggag ttattttagg agagagattt aggggcgtcg attacgagtt cgaaagggag 2820
   aggtttgggt ttgggatgga gatggggagg tagataagtg tgttgggggt ttgggttgag 2880
   gagaagaatg ttagttggaa tttgggcggg ggtgttagtt gcggggaggg agtatataag 2940
   atgggtagtt ttagttgggg gaggttcgat gttttttagt cgttatagat ttatttgcgt 3000
   agtgttgggg gagggaagaa gatatgggaa ggggcggagg aaggagagag gaagggagaa 3060
   agggagggag gagaaaaaga ggaagggggg gtatattagt tagaaaatag atattagttt 3120
   agattattcg tttttttttt gtttgttcgt acggtgggta gagcgtatag tatcggtatt 3180
   gtagtagttc gggttaggga gggaattagt attatagtta gcgtcgtttg ttttgggtat 3240
   aggattgagg tttaggtttt agcgttggga ttgtttgggc ggaggaattt tggggaaagg 3300
   ggtagttttt tgttttcggt tttgggattt gggtttggga agagacggtt tatttttgaa 3360
   aagcgtttgt ttattcggag gtgggatttt aggttttgtt tggatttatg ggttaggaga 3420
   gggtcgcggt aggttgggag gagagtgagg ttttggggga gtggatgggg tgttttacga 3480
   agagtttgtt ttagagttag gtgatataga ggggttttag gagggagtta ggtttcgttg 3540
   agagttttta gagagataat ttattttggg ggaggggttg aagtgattat gtagatttaa 3600
   gggtattaga agagtttcgg ggaaacgcgg cgtggattcg taggttttgg cggggtttcg 3660
   aagtagtgaa gggtttaggt ataaggcgga tatttaggtt aggtttgaaa gtagaggggt 3720
   gaggaggacg aaggtgcgag tgtgtggggg ttgttttttg tggggagtta gcgggaagtt 3780
   aagtatttta agagtaggat tttagatttt tttgtttatt agatttttta gattttaggg 3840
   aggttgggga ttacgggttg agtttttttt tggttttagt gttgaaggag aaggtagaga 3900
   ggtaacgggg agagagatag agatagtgat atatagttta gaggaaagga ggggagagga 3960
   tgagaggata gagagagaga ggagaaggga gataggaaag taagaggaag gaagaaggaa 4020
   agcggaaaag aagaggaagg agagaagtga agataggaga gagaagggag aagattgaag 4080
   agaaacgagg taaggagaaa gagagataag aatagagagg gaaattagag agaagggaag 4140
   agagtcgggt gggtacgaag gtttcggttt gggtgagtgt ttaggtggtg aatggtttgg 4200
   gttttttcga ggttagtatt cgtatttttg gggttagaat cggaaagagt taagttattt 4260
   tattttgaac gcgttgggta gaaaaatatg taaattaggg ttttttgcgt tttggagagt 4320
   ggcgaaatta ttttcgttcg gtgaataaga agtaataagt ttttaggcgg gtgagttgta 4380
   gagggtggta gagtcgcggg ggagggagcg gttgtagtaa ggggggtggg gttcggaatt 4440
   ttatattttt atatttagtt tttagttttt agggagtttg gagtcggggt tttttttttt 4500
   ttgttcgttg tttttttttt tttttttttt tttttttttt tttttttttt tttttttttt 4560
   cgtcggtttc ggttcgcgta tttaaagcgg cgcgggaggg cggacgcggg cgggcggttc 4620
   gttcgggcgg tggtagatgc gtttagcggt gatagcggtt agcggcgcgt aggtgatcgg 4680
   tttgaggcgt agtttggtta gggagcgttc ggggagagtt ggcggtagag ggtagtcgat 4740
   tcgtttttag cgcgcgcgtt tcggcgttag gagtcgtttc ggggcgtgtt ggcgagcgtt 4800
   gatatagata taaggatatt tttttttatg gcgttacgtg atataattat tattagaatt 4860
   aattaagatg aattgtacgt tagcgttcgg tggggatttt tgtttagttg attttggttt 4920
   aagttttttg tgtaatcgat ggtttaggtt ggttgcgcgg ggagcggtta gaggttcgtt 4980
   ggcgcgtacg tcgcggagtt atgaacgatt ttgacgagtg cggttagagc gtagttagta 5040
   tgtatttgtc gggttgcgtt tattatgtgg tttcgtttga tttcgttagt aagttttcgt 5100
   ttttttttta atcgtcgttt tgttagatga ttttttttta tttttttaat ttggtttcgt 5160
   acgtttagtt cgtgcgcgaa gtggtttttc gcgattacgg tttggagcgc gttaagtggt 5220
   cgtatcgcgg cggcggcggc ggcggtagcg cggggggcgg tagtagcggg ggcggtttcg 5280
   gcgggggcgg cggcggcgcg gggggttacg ttttttatta cgcggcggcg gcggcggcgg 5340
   ttgcggcggt cgcggcggtc gaggaggcgg ttatgtaacg cgagtttttt tcgttcgcgg 5400
   gtcgtcggtc ggacgtgttt tttaaggcgt ttgagtcggt gtgcgttgcg tcggggtcgt 5460
   cgtacggttt cgcgggcgtc gtttttaatt tttatagcgc ggtgggtcgt aatggtattt 5520
   tgttataggg tttcgattag ttttacgagg tagcgttcgg gttttcgttc gtcgggtcgt 5580
   agttttcgtc gttattcgcg tcgttatagt tcgagggcgt agtcgataag ggcgatttta 5640
   ggatcggggt tggtggcggc gggggtagtt tttgtattaa ggcgattttt ggttcggagt 5700
   ttaagggggt agtagaaggt agcggtggcg acggcgaggg tttttcggga gaggcggggg 5760
   tcgagaagag tagtagcgta ggtaggtatc gggtattggg taagcggtgg gttcgggggt 5820
   cgcgggggag gggggggggg cggaggtttt tttttgtttg cgttttttta tgtgttattt 5880
   tataagtatt cgaagaggtt tatatatttt ataagatttt ttgggtcgtt gtaaagtgtg 5940
   tttatggtag gaaattaatt taggtgggtt taaggtagat ttcgaggagg atattaatag 6000
   ttgtcgagag ttttttttta gttttgggta gggggttgtt agatattatg gagggaggga 6060
   gggagggaga ttttaagtta gtgtgagtgt atatatcgga gttttttgtt tttaagggag 6120
   agaggcgggg ggtggggaag tggggggagt ttgatttttg tattggattt tatttaagtc 6180
   gttagttgac gcgcgtcgtt ggagttaagt agatgtttcg gcgggtttgt ttttgttttt 6240
   gtttttttag gttttagttt agagtttgtt ttgttttttg tttttttatt ttcgtgttag 6300
   gttttgtgtg tgtgtgtgtg tgtgtgtgtg tgtgtgtgtg tgttcgggcg tgaatatatg 6360
   tttacgttcg tatttttttt tgtgttcgtt tatatattat tttttacgac gtaggtaggg 6420
   tttttcggtc gcggtagaga acgtttttaa cggggttagg gtttggtttt cgtttagtgg 6480
   ttcgggcggg cgggcgggtg ggggttgtta ggtagcggtt ttttttattt tttggttttt 6540
   ttgttttgta gt 6552

<210> 158
<211> 6552
<212> DNA
<213> Artificial Sequence

<220>
<223> chemically treated genomic DNA (Homo sapiens)

<400> 158

attgtaaggt aaagagatta gggggtgaga gaggtcgttg tttgatagtt tttattcgtt 60
cgttcgttcg ggttattgag cgagggttag gttttggttt cgttggggac gttttttgtc 120
gcggtcgaaa ggttttgttt gcgtcgtggg ggatgatata tgggcgggta taggagagag 180
tgcgggcgtg gatatgtgtt tacgttcgga tatatatata tatatatata tatatatata 240
tatatataga gtttggtacg gaggtgggga agtagagggt aaggtaggtt ttaaattgga 300
atttgaggga atagagataa gagtaggttc gtcggggtat ttgtttgatt ttagcggcgc 360
gcgttagtta gcggtttgga taaagtttag tgtaaggatt aaattttttt tattttttta 420
tttttcgttt ttttttttta aaagtaggag gtttcggtgt atgtatttat attggtttga 480
aatttttttt tttttttttt tttatggtat ttagtaattt tttgtttaaa attgggaaaa 540
ggttttcgat agttattaat gtttttttcg aagtttattt taagtttatt taaattggtt 600
ttttattata aatatatttt ataacggttt aggaaatttt ataggatgta taaatttttt 660
cgaatgttta taaagtagta tataaaaagg cgtaagtaga gggaggtttt cgtttttttt 720
ttttttttcg cggttttcgg gtttatcgtt tgtttagtat tcggtgttta tttgcgttgt 780
tgtttttttc ggttttcgtt tttttcgggg ggttttcgtc gtcgttatcg ttgttttttg 840
ttgttttttt gggtttcgag ttaggggtcg ttttggtgta gggattgttt tcgtcgttat 900
tagtttcggt tttggggtcg tttttgtcgg ttgcgttttc gggttgtggc ggcgcgggtg 960
gcggcggggg ttgcggttcg gcgaacgggg gttcgggcgt tgtttcgtag aattggtcga 1020
agttttgtgg taagatgtta ttgcggttta tcgcgttgta gaagttggag gcggcgttcg 1080
cggggtcgtg cggcggtttc ggcgtagcgt atatcggttt aggcgttttg aagagtacgt 1140
tcggtcggcg gttcgcgggc gggagaagtt cgcgttgtat ggtcgttttt tcggtcgtcg 1200
cggtcgtcgt agtcgtcgtc gtcgtcgtcg cgtagtaggg agcgtagttt ttcgcgtcgt 1260
cgtcgttttc gtcggggtcg ttttcgttgt tgtcgttttt cgcgttgtcg tcgtcgtcgt 1320
cgtcgcggta cggttatttg gcgcgtttta ggtcgtagtc gcggaaggtt atttcgcgta 1380
cgggttggac gtgcggagtt aggttggaag agtaggggaa agttatttgg taggacgacg 1440
gttgggaaag gaacgaaggt ttgttagcga agttagacgg ggttatatag taggcgtagt 1500
tcggtaggta tatgttggtt gcgttttggt cgtattcgtt aaagtcgttt atgatttcgc 1560
ggcgtgcgcg ttagcgagtt tttggtcgtt tttcgcgtag ttaatttgag ttatcgattg 1620
tataagaggt ttgggttagg attaattaag taaaaatttt tatcgggcgt tgacgtgtaa 1680
tttattttga ttgatttggg tggtaattat gttacgtgac gttatgaaga gaaatgtttt 1740
tatatttata ttaacgttcg ttaatacgtt tcgggacggt ttttggcgtc gagacgcgcg 1800
cgttgggggc ggatcggttg ttttttgtcg ttagtttttt tcgggcgttt tttgattagg 1860
ttgcgtttta ggtcggttat ttgcgcgtcg ttggtcgttg ttatcgttgg gcgtatttgt 1920
tatcgttcga acgggtcgtt cgttcgcgtt cgttttttcg cgtcgtttta agtacgcgga 1980
tcgaggtcgg cggggaggag gaaggagggg gaagaggggg aggaaggggg aagggagaga 2040
agtagcgagt agaggggggg aagtttcggt tttaggtttt ttagaagtta ggaattaggt 2100
gtgagggtgt gaggtttcga attttatttt ttttattgta gtcgtttttt ttttcgcgat 2160
tttgttattt tttgtagttt attcgtttgg gagtttgttg ttttttgttt atcgggcggg 2220
agtaatttcg ttatttttta gggcgtaggg agttttgatt tatatatttt tttgtttagc 2280
gcgtttagag tggagtgatt tggttttttt cggttttgat tttaagggtg cgagtgttgg 2340
tttcggaaaa atttaggtta tttattattt aggtatttat ttaggtcggg gttttcgtgt 2400
ttattcggtt tttttttttt ttttttggtt tttttttttg tttttgtttt tttttttttt 2460
tgtttcgttt ttttttagtt tttttttttt ttttttttgt ttttattttt tttttttttt 2520
ttttttttcg tttttttttt tttttttttt tgtttttttg tttttttttt tttttttttt 2580
tttgtttttt tatttttttt tttttttttt tttaagttgt gtgttattgt ttttgttttt 2640
tttttcgttg tttttttgtt ttttttttta gtattgaagt taggaggggg tttagttcgt 2700
ggtttttagt ttttttagaa tttggggagt ttggtgaata aaaggatttg gagttttatt 2760
tttagggtgt ttggtttttc gttggttttt tataagagat aatttttata tattcgtatt 2820
ttcgtttttt ttattttttt gtttttaggt ttagtttggg tgttcgtttt atatttagat 2880
tttttattgt ttcggagttt cgttaaggtt tgcgaattta cgtcgcgttt tttcgaggtt 2940
tttttggtat ttttgggttt gtatggttat tttagttttt tttttaagat aggttgtttt 3000
tttaaggttt tttagcggag tttgattttt ttttggagtt tttttgtgtt atttggtttt 3060
gaggtagatt tttcgtgggg tattttattt atttttttaa ggttttattt tttttttagt 3120
ttgtcgcgat ttttttttag tttatgggtt taggtaaggt ttgggatttt attttcgagt 3180
gagtagacgt tttttaaagg taaatcgttt ttttttaaat ttaaatttta gaatcgaggg 3240
taggaggttg tttttttttt taagattttt tcgtttaggt aattttagcg ttggggtttg 3300
ggttttagtt ttatatttag ggtaggcggc gttggttgtg atgttggttt tttttttggt 3360
tcgggttgtt gtagtgtcgg tgttgtgcgt tttgtttatc gtgcggatag atagagaaaa 3420
ggcgggtggt ttgagttaat atttattttt tggttgatgt attttttttt tttttttttt 3480
tttttttttt tttttttttt tttttttttt ttttttcgtt tttttttatg tttttttttt 3540
ttttttaata ttgcgtaaat aggtttgtgg cgattggagg gtatcgagtt ttttttaatt 3600
agaattgttt attttgtgtg tttttttttc gtagttggta ttttcgttta gattttagtt 3660
gatatttttt tttttaattt agatttttag tatatttatt tgttttttta tttttatttt 3720
aggtttaagt tttttttttt cgggttcgtg gtcgacgttt ttggattttt tttttaaaat 3780
ggttttttgg acgttgtata ttggttttgc ggtagtttag gatttagggt agttgcgagg 3840

attttaattc gggtttttat gtgtttgggg attcgtaaaa ttttcgtatt ttttttttat 3900
tggcggtacg gggtggggtg taggtagttc ggcgttttta gtatggagtt tgttgatcgt 3960
cgaagaggga aaaggggagg acgaattatt taagtttaac gatatggcga cggtagtttc 4020
ggcgggagta gcgatttggc gggggagggt gagttatttg tagtggcgcg tcggtgtata 4080
ggggaagggg gcggtcggtt tttttcggtt ttttagcgcg gatttttgtt tttcgattta 4140
ttttataatt tagagcgttt ttattttagt tttagttcgt ttggtttttt ggttttagtt 4200
tttttttagt ttttattttt attttttaaa tttagggaaa atcgtagttt tcggattcgc 4260
gcgtttcgtt cgagaattta tcgtgaagat ttcgattttg tggttttgtt tttgtttgcg 4320
cggttttatt cgaaacgtag ggagcgtatt aggttttcgg ggcgtagtta gtcgtttcgc 4380
gagaaagagt ttttcgtata gcgttaggac gcgatgcggt gattttgagt tatttaagat 4440
aagatattaa tttgatttta attttgttag ggcgtttttg gtatttggag aacgtgaata 4500
gatattgttt ggtagttttc gtaaaagttg attggggaag ggattttgag ttattttatt 4560
tattgttatt atagttttgt aagaaagttt tttttttttg ttaaatttta attatttatg 4620
tttttttaag aaacgaaaga agagaaatgt agggaaaata tttagagtta gttttttttt 4680
tatagagttt tcggaatgga ttttttaagt taagcgattg gagaataaaa atatggggag 4740
attatttttg tttaaggata gtggggtagt cgttttgttt ttttttatta ttattgaggg 4800
gggaatgtag ttgggagagg cgggtgaagg gtgaaggggt atttttttat atgaatattt 4860
ataacggttt tagatttttt ttttattcgt cggtatttcg gaagtttttt tagttcgttt 4920
ttttggggga gaatttttag gttttaaaag cgtttttttt ttttcgtcgt tttttgagtt 4980
tttaattgat tgaatgttag atgaagttgt ttttttttcg ttttaaaatt gtttttttgg 5040
ttaagaggtt cgtttggatt gtagtatttt agaggatata tttgtttttt tgaagggaat 5100
gttgagtatt tagattttag cgtttttttt tttcggtttt tttttttttt tttttttttt 5160
aataaatttt ttagtggttt taaatttttg cgggaattcg ggttatggaa ggagatatta 5220
gagtttttgt atttgtgtga aatagtaaac gggatagatt tggaaaagat tgttgaatta 5280
agagatacgt ttttatattt agaataattt aatttttttt gaggagtgat ttttatgaat 5340
attttttttt tttatttatt ttaggttaaa gggaaagaat acgatgattt aatgatatat 5400
tttggaaaga aacggttttc gtgttttttg ttttaaggtt tggatttgag tttatatgtt 5460
tttttttttt tttttttagt ttttcgagcg tttagtgcgg ttttagtttt tgtttagttg 5520
tttttgagga ggtaaatttt tgaatgtaga tttagatttg gagattttgt tttgggtatt 5580
atcgttttta tgtgtttagt taggtggaga atttttttga gttatagggt tttggtagtt 5640
tttgtttttt ttttttttat tgaatatata tatatatagg tatatatagt tgtgtgtgtg 5700
tatataatta aagaataatt aaaaatttta attatatatt aaatttgttt ttgggaaagg 5760
gagttgaaat gttgaatatt tagtgttagt gatttagtgg ttttgttaat ttttttttta 5820
gttaaaagtt agtttttatt tttattagtt aggagtttgt ttttgtaaga aaatgagtag 5880
taagtaggtt ttgaattttt tggattaagt tagaggttta gatggattag ggagttttga 5940
ggttttattt tttaagggag tttgttgggc gtttggtaaa tttgttggta ttaaggattt 6000
agtaggatat agtcggtttt agagaagtta gattaaaatt cggtggagat agacgttttg 6060
ttttgatatt tgaagaattt taaattttta tagatttcga atattagtaa gttttaaaat 6120
ttgagaattt aggttaagtg tggaagtttt agtttgtaat aagttgagag tttttttaat 6180
gtgtgagata tatttagatt tgttatggtt tttttttttt ggatatttta aatatatttg 6240
gtttgtaggt ttttatatat tattttttga ttgtttcgga agttattttt ttatttaacg 6300
aatagttaat attcgcggtg aaataataat tagaaaagaa aaacgaattt ggtggtttga 6360
aaaatattaa ggtggaattt aaaatatttt gaaaaattga ttttagtttt ttgatagttt 6420
ggtttagatt tggatattta tttttattta aaatatttgt atatattgtg tttgatatat 6480
ataagttatt gtttttgata aaattaaaat aaaatatgta tgataattat tagatagttt 6540
agaaaatgaa at 6552

<210> 159
<211> 5721
<212> DNA
<213> Artificial Sequence

<220>
<223> chemically treated genomic DNA (Homo sapiens)

<400> 159

aatatgtatt taattttttt tattttttgt taatggtggt aaattttgtg aattgttttt 60
tttttttttt tttatttggt tttaaaattt tttgttgttt aatttgattt tgtagttttg 120
atttttattt gtttattatt ggttttgttt ttgtttaggg tttttaaaat atttgttttt 180
aatgttttgt tttttttttt aggttagtat aaagggattt gaagtatttt ttaataattc 240
gttttttaat gaattttaga agtgttattt tgatttaagg gtttttttaa aaaattattt 300
tatttgttta tttttagtat tgattatttt tataatttat taggatagaa tggtttttag 360
tcgtttatat tttgtaatta ggtaattgaa ttattagttt ttagtagttg ttttgaggta 420
agtggaaagg taggtagtgt tttggggtta tcgagaaagt ttaaaaatag gaggttgaag 480
gtattgtgat ggttttaaaa atggttattt tattaaatag ggattgtatt aatattgaaa 540

tgaagataat ttttttaatt ttgggtgttt tatttgttgt tttaattgtt tgtttttaat 600
attttgtagg tttgtgtttt tataattttt aatttgaaat ttatgtgttt ttatggatcg 660
tggatgtttt tattttttga gtttttaatg tagatattta aatggttgta attagtagag 720
tgattcgcgg atggtataaa tgtatttttt ttttttggtt ttggatttat gggtttggga 780
ttgtggttat ttttttaatt tttaaaaaga ggttgaatta atgtggtcgt gggtgggaat 840
ttatatatag aatttaatga agaatttgat tgtttaaata agggggtttc gtatggagtt 900
gtaaagtatt taataaatat gaaaaatgtg aagttttaag gtttaagaag aaaaataatg 960
atgtttttga aagtgatgat aaataattat ttttaaagtg ttgtatattt atataattga 1020
gagattattt ttgtaaatgt aatgtttgtg agttgggata tatgggtagt gttttagata 1080
tttaaaaatt atttttattt tttagggaga tgttaataaa tagaattttt ttgttttttt 1140
tgagttttta tatatttgtt ttattttttt aagtttgtgt ttttttttga aacgattttt 1200
atttattata aaattttggt attttgaata taattatgtt ttttatagat gaaggaaatt 1260
taatattaat tttggttttt ggaattttta tgttttttat ttttgttttt tttttttttt 1320
tttgtttatg attttaatat aagtagtgat gttttaaaat gaggatatta tttatagtgt 1380
ttaaaatgat attttatatt aggttagttt aaaagataat aaaattttat ttttttttta 1440
ttattgttaa tttatttata tagttttatt tattttttaa tttatatgta tttgtatgtt 1500
tgtgtataga aaaatttatt tatttgaaaa tttttaattt ggaaagatta ttataaatat 1560
ttggattagg aaggttttga aattagaaag atatatttat atttggtaat cgaaaaagaa 1620
atattttaag tttattttat atcgtttttt tcgggtagtt tgtatagagt agttgataat 1680
tgaaaatata aaatttaaat ttttagacgt aatttttagg tattagaatt ttttgtttga 1740
ggtttttttg ttatttaggg aaatggtcga tgaggaattg gatgattttt tatggttagt 1800
tttttatata attaatttag tggttgttaa agttttagtg tgtaagttag ttatatagtg 1860
atatttaaaa ttttatttag tgtgaaaatt tttttggtgg gagggttttt ttatgatttt 1920
gagagagaaa acgtagagat ttaagatttg gagttatttt cgtttttttt gttttagttt 1980
gatttttttt atattttatt ggcgatcgtg gatagttaat ttcgattttg agggggaggg 2040
atagagatag agaaatagag agagatatga tcgcgattta atttttgtag aagaaataat 2100
tcgtgtataa ggggttatat tattttttat tttatatttc gggaagggtc gttatcgttt 2160
ttgtggtttt ttttaaaaga tgtgttttta ttagaaacgg gtatattttt tttttttttt 2220
aatcgttaat ttttttttta ttttttgtga gttttatatt tcggagtttt attttatttt 2280
ttttgtttat ttttcgggtt ttttatcgtg ggtaggggag attgtttcga ggtttttttt 2340
ttatttttag tttgtatgtt attttttttt tttttttcgt tttggagaaa tggtaggttt 2400
gttgaagatt aaaattgtgt ttaaacgttt tttggaggaa tttggattga gggttaaatt 2460
aataatagaa gaaggttttg ttatttatat gtattttttt tatattttaa attgtagagg 2520
gaggagagga attggtaagt ggtttaggta ttgggaggga ggttttcggg gaattcggaa 2580
ggttaagggg gtttggagag aggttttttt ttcggttgga gataggcggt aggagagttt 2640
tttagaaatt cggtaggttt ttagcgtcgc gggtggggga acgaggtttt cgtgtcgcgt 2700
gatttaagag gcgaaggaag tattggagtc gggatttacg gttagataaa ggggcgattg 2760
tagtgttttt tcgtttttgg tagttttttg ggtttttttg gtttcgttgt taggggcggt 2820
tttgtcgtta agattttcgg ttttcggagg cgttaggttg cgtttgaaag cgggttggcg 2880
atttagggga ggtcgtatcg ttttttattg ttgattttga tcgtgcggtt gcgtttcgat 2940
aggtatggag cgcgttcgtt attttttggg tattgggcgg tattggtaga gttttttatt 3000
cgttttttag ttaaattttg ttttattttt ttaattatta gttaagttat tttttcgtta 3060
ttatttttta tttcgatttt ttttcgatta ggttttttag ttcgtataag attttgtttg 3120
ttagatttcg aaagttataa attcggggga tgaagtggga agattgttta aggagtaatg 3180
gagggaattt agttgttttt gttttttttg ttttttattg agttttttta tgtttttagt 3240
tttgaggcgt aaatagttgt tgtagggttg tttaaaggtt cgaaagagat atttttcggt 3300
ttggaaataa cgaggaatcg tcgtgtattg aaacgtgagg agagattgta gggatttttg 3360
gagttggagt tttcgggagt aacgaagttg tagtttttcg aaaataggat gggtttttta 3420
aaataagagt ttttagggat ttgaggtttg tatttttttg agtatttagg aattgggatt 3480
gtagtttgtg tttgttttta ttttttaaga gaggacgaga gcgagtgagt ttttatgttt 3540
gggtttttaa ggaaattttt tggtagagtt agaggagttt tagatatttt tattttttcg 3600
tttttatttt tttttttttt tttattagag taggaggaag aggtcgattg cgttttttag 3660
agttatttaa ttggtcgtta taatttatat aataaagtgg tagcgcggcg gttagttttg 3720
tttttcggcg gcgtttgtgt ggttttagtg taggagttcg cgatttaatt aattttagtt 3780
tggttcggag atcgagggtt ttgttgtagg ttgagattgg cgtcggttat cgggtttttt 3840
agggttttgg gattagaggt aggaggggag gatttttagt tttttggtcg gattaggtaa 3900
gagggttagc gacggggttt gagtatatcg ttttcggtta agaattgttg ttttttttat 3960
ttttttgttt gttagattcg tgaatttttt attttcggta tttttggggg cgttgaggta 4020
aggaattttg ttagttagtt gttttcgaac gttcgttgtt tttcggttag gtggttttgg 4080
tttttttgtt tttttatatt tagtcggggg agttcgtttt gttttgtttt ttatttggtg 4140
gatggggata tttcggagtt ttggtttgta gtgtgtagtt ttggagagga tttttgaaga 4200
taaaaggaat gtagtttttg gttacgattg gtggatcgag ttgatgggtt ttttggcgga 4260
tattttttat ggatttgtat ggtaattttt ttatgattag agtttttagt tatggggata 4320
taaattagaa atagtgtggt tttttttttg gattttaaaa ttgggtagtt tgtgatgttt 4380
tttttgtgtt tgcggtttgg gattgagggt ttaagtagcg aatgggtttt tgttgttaga 4440
tttttaggta gaggtttttg gagaaatttt ttttgtggga gtgattgggg taaaagtgat 4500

attgtttagg ttaagggata gagtttttgg tgtcgtcgga tcgcgttttt ttcggataag 4560
tcgagaggcg tcggttaatg gaaaatgttt tcgttgtaat ttaaagtcgg tagaagtaag 4620
tcgggtttag aaagtttgcg gaaaacgaat cgtaaagtta attacgatta agaagagttt 4680
taggggatat ttgggtagag ttattttttt gttcgatgtt tttagttgtt gaagtcgggt 4740
ttggaaattc gtagatagtt agttttcgtt taatttgatt tggttttgtt ggtagtttcg 4800
tttttcgtta tcgaatattg cgggtgttat tataatattt tgaagggggg gaaaacgggt 4860
cggggggatg taggcggtgt tgaaatgatc ggttttgaag aatttgtagg taaagtttcg 4920
tttaatcgtt tgagtttgtt tttttatttt cggttgtaat taaatattgt tgcgagcgta 4980
gtcgaagttt tttgttggag atgtgtgagc gtagttttta tagagcgggt tatgtgggtt 5040
cgtttttgaa tttgtagtcg ttagattttt tttatttttt taatttgagg tcgaatggcg 5100
gttagttggt cgcgtttttt tttatttttt attcgcgcgg cgcgttgttt tgggtcgtta 5160
cgttcgtttt ttgcgttttc gcgtagtttg cgggcgttat tgttttcggc ggtttttcgt 5220
agttttattt ggttggtttc gggtttttcg gtttgtagtt tttaatagtt aaagacggat 5280
tcgaagagta ggttaagttt tatgcgttcg aagcggtcgt tgggttagag gagcgcggtc 5340
gtattcggtc gtttttcgtt ttcgagttta gtttggtttt tgtagtggtt gtttttaaag 5400
cggttaagta tgattacgtt ggtgtgggtc gtgttacgtc gggttttacg attttgtttt 5460
agggggtttt ttgcgttttt ggttttaagg acgatattaa gggttcgttt aatttgaata 5520
tgatagtgta ggcggcgggc gttgtttttt gtttgcgatt tttattgttc gacggtaaac 5580
ggtgtttatg tttttcgggt cggtttgggt cgggatggga ggtggggttt aagggagagt 5640
gtaaggggag gtgaatcgtt tgggggcggg taatagatag agtacgggtt gggttgacgt 5700
gggttggggt tgtgttgtag g 5721

<210> 160
<211> 5721
<212> DNA
<213> Artificial Sequence

<220>
<223> chemically treated genomic DNA (Homo sapiens)

<400> 160

tttgtaatat agttttaatt tacgttaatt tagttcgtat tttgtttatt gttcgttttt 60
aggcggttta ttttttttta tatttttttt tgaattttat tttttatttc ggtttaaatc 120
ggttcgggga gtatgggtat cgtttatcgt cgggtagtga aggtcgtagg taagaggtaa 180
cgttcgtcgt ttgtattgtt atgtttaagt tgagcgggtt tttggtgtcg tttttgaagt 240
taggggcgta gggagttttt tggagtaggg tcgtggagtt cggcgtggta cgatttatat 300
tagcgtagtt atatttggtc gttttgagag tagttattgt aggagttagg ttagattcgg 360
gggcgaagga cggtcgggta cgatcgcgtt tttttggttt agcggtcgtt tcgggcgtat 420
agaatttagt ttgtttttcg ggttcgtttt tggttgttgg gggttgtagg tcgagaggtt 480
cggagttagt taggtagggt tgcgagaagt cgtcgaaggt agtggcgttc gtaggttgcg 540
cgggggcgta ggaggcgggc gtggcggttt agggtagcgc gtcgcgcggg taggagatag 600
ggggaagcgc ggttaattgg tcgttattcg gttttaggtt ggagaagtag aaagagtttg 660
gcgattgtag atttagaagc gagtttatat agttcgtttt gtagagattg cgtttatata 720
tttttaataa agggtttcgg ttgcgttcgt aatagtattt agttataatc gggaataaga 780
ggataggttt agacgattgg acgaaatttt gtttgtaggt tttttaaagt cggttatttt 840
agtatcgttt atattttttc gattcgtttt tttttttttt agagtattat gataatattc 900
gtaatgttcg atggcgaagg acgaggttgt tagtagagtt aaattaggtt gagcgaagat 960
taattgtttg cgggttttta ggttcggttt tagtagttgg ggatatcggg taagagggtg 1020
atttgtttta agtgtttttt gggatttttt ttggtcgtga ttggttttgc gattcgtttt 1080
tcgtaggttt tttgggttcg gtttgttttt atcggtttta agttgtagcg gaggtatttt 1140
ttattaatcg gcgtttttcg atttattcgg agagggcgcg gttcggcggt attaggagtt 1200
ttgttttttg gtttgagtaa tgttattttt attttaatta tttttataga gagggttttt 1260
ttagagattt ttgtttgagg atttggtagt aaagatttat tcgttatttg agtttttagt 1320
tttaggtcgt agatataaaa agggtattat aaattattta attttgggat ttaggaagga 1380
ggttatatta tttttggttt gtgtttttat ggttaagaat tttgattata gggaaattgt 1440
tatataggtt tatggagaat gttcgttagg gaatttatta gttcgattta ttaatcgtgg 1500
ttaaaggtta tatttttttt atttttagga gtttttttta ggattatata ttgtaaatta 1560
gaatttcgag gtgtttttat ttattaagtg gagggtaaag taggacgaat tttttcgatt 1620
gggtgtggaa aggtagaaga gttagaatta tttggtcggg gaatagcggg cgttcggagg 1680
tagttggtta gtagagtttt ttgttttagc gtttttaggg gtgtcgggga tagggagttt 1740
acgggtttga taaataggga agtgggaaag gtagtagttt ttagtcggga gcggtgtgtt 1800
tagatttcgt cgttggtttt tttgtttagt tcggttagag gattaggggt tttttttttt 1860
tgtttttgat tttaagattt tgggagattc ggtgatcggc gttagtttta gtttgtagta 1920
ggattttcgg ttttcgagtt aagttggaat tggttagatc gcgggttttt gtattgagat 1980
tatataggcg tcgtcggagg gtaagattga tcgtcgcgtt gttattttat tgtgtgagtt 2040

atggcgatta attgggtggt tttggggaac gtaatcggtt tttttttttt gttttggtgg 2100
gggggagaga gggagtgagg acgggggggt ggggatgttt ggggtttttt tggttttgtt 2160
agagagtttt tttaggagtt taggtataga gatttattcg ttttcgtttt tttttgggag 2220
gtgggggtaa gtataaattg taattttagt ttttagatat ttaagggaat ataagtttta 2280
gatttttaag agtttttatt ttggggaatt tattttattt tcgaggagtt gtagtttcgt 2340
tattttcgaa agttttagtt ttaggggttt ttgtagtttt tttttacgtt ttagtgtacg 2400
acgatttttc gttattttta agtcgagggg tgtttttttc ggatttttag gtagttttat 2460
aatagttgtt tgcgttttag gattaaaggt ataaaagaat ttagtgaaaa ataggaaaaa 2520
taagaataat taggtttttt ttattgtttt ttggatagtt tttttatttt atttttcggg 2580
tttatgattt tcgaggtttg gtaggtaaag ttttatgcga gttggggaat ttgatcgagg 2640
gaggatcggg gtggaaagta gtggcgggag agtggtttgg ttggtgatta agagaatgga 2700
ataggatttg gttgaggagc gaatagagag ttttgttaat gtcgtttaat gtttaggaga 2760
tggcgagcgc gttttatgtt tatcggggcg tagtcgtacg gttaagatta atagtgaagg 2820
gcggtgcggt ttttttgggg tcgttagttc gtttttagac gtagtttagc gttttcgaaa 2880
gtcggagatt ttggcggtaa ggtcgttttt gatagcggag ttagagggat ttagggggtt 2940
gttagaggcg ggaagtattt gtagtcgttt ttttgtttga tcgtgagttt cggttttaat 3000
gtttttttcg ttttttgaat tacgcgatac ggggatttcg tttttttatt cgcggcgtta 3060
aaaatttgtc gggtttttga ggagtttttt tgtcgtttgt ttttaatcga aggaaaggtt 3120
tttttttaag tttttttagt ttttcgggtt tttcgagggt ttttttttta gtgtttgggt 3180
tatttattag tttttttttt ttttttgtaa tttggaatat aaagggaatg tatatgaata 3240
ataaagtttt tttttgttat tgatttagtt tttaatttaa atttttttag agagcgtttg 3300
agtatagttt taatttttag taaatttatt atttttttaa ggcgggggga gggggggaag 3360
tgatatgtaa attagagatg aaaggaaagt ttcgaggtaa ttttttttat ttacgatggg 3420
aaattcgaga aatagatagg ggaaatggag tgaagtttcg gagtgtggga tttataaaaa 3480
atggagggga agttggcgat taggagaaag gaaaaatgtg ttcgtttttg atgaaaatat 3540
attttttgag aaaaattata aaaacggtaa cgattttttt cgaagtgtaa ggtgggggat 3600
ggtgtggttt tttgtgtacg ggttattttt tttgtaaaga ttgaatcgcg attatatttt 3660
tttttgtttt tttgtttttg tttttttttt ttaaaatcga agttagttgt ttacggtcgt 3720
tagtaagata tgaaagaaat taagttggga tagggaaaac gaaagtagtt ttaagtttta 3780
ggtttttgcg tttttttttt taaagttatg agggagtttt tttattagga gagtttttat 3840
attaagtgag gttttaaata ttattatgta attaatttat atattaaaat tttagtaatt 3900
attgagttga ttgtgtgggg agttaattat gaggagttat ttagtttttt atcggttatt 3960
tttttgagtg gtaaaaaggt tttaaataga aaattttagt gtttaaaaat tgcgtttgaa 4020
agtttaggtt ttatgttttt agttgttaat tgttttgtgt agattgttcg gagaaggcga 4080
tatgggataa gtttgaaatg tttttttttc gattgttaaa tatgggtatg tttttttaat 4140
tttaagattt ttttagttta gatgtttata atggtttttt taggttgaaa atttttaagt 4200
gagtaagttt ttttatgtat aggtatataa atatatatgg attaaaaaat aaatagaatt 4260
atataaataa attgataata gtgaaagaaa agtaaaattt tattgttttt tgagttggtt 4320
tggtataaaa tattatttta aatattgtaa atgatatttt tattttaaaa tattattatt 4380
tatgttaaag ttatgggtaa aagggaggga ggggggtaaa agtggagagt atgaaggttt 4440
tagaaattag aattagtgtt gagttttttt tatttataga gaatataatt gtgtttaaga 4500
tattaagatt ttgtaataaa taagaatcgt tttaagaaga aatataaatt tgaaaaaata 4560
aaataaatgt ataaggattt agaagaaata aaagagtttt gtttattggt atttttttag 4620
gagtaaaaag tgatttttaa atgtttgaag tattgtttat gtattttagt ttatagatat 4680
tgtatttata aaaataattt tttaattgta taaatatgta gtattttaaa agtaattatt 4740
tattattatt tttagaaata ttattatttt tttttttgga ttttggaatt ttatattttt 4800
tatatttgtt agatgtttta tagttttatg cgaggttttt ttgtttagat agttaagttt 4860
tttattgggt tttgtatgta agtttttatt tacggttata ttgatttagt ttttttttga 4920
ggattgagga gatgattata attttagatt tatagattta aggttaagaa aaggaggtgt 4980
atttatgtta ttcgcgggtt attttattga ttgtagttat ttaaatgttt gtattgagag 5040
tttaagaaat gaaggtattt acgatttatg aggatatatg agttttaaat taaagattat 5100
gaaaatataa atttataaag tgttaaaaat aaataattaa aatagtaagt gaaatattta 5160
aagttggaaa gattgttttt attttaatat tgatataatt tttatttaat aaggtggtta 5220
tttttaaagt tattatagta tttttagttt tttgttttta gattttttcg gtgattttag 5280
agtattgttt gtttttttat ttgttttagg gtaattgttg agagttaatg atttaattat 5340
ttaattatag gatatgaacg attgaaaatt attttgtttt aatggattat gaagataatt 5400
agtgttggga atgaataaat aaagtaattt ttttaaaaaa tttttaaatt agaatggtat 5460
ttttgaagtt tattgggggg cggattatta aaaaatattt taagtttttt tatattggtt 5520
tggagggaga aataaaatat taaaaataga tattttaaaa attttaggta agaataaaat 5580
taataataaa taagtaaaaa ttagaattat aaagttaggt tgggtagtag aaggttttag 5640
agttagataa ggggaagaga gaaaaataat ttataaaatt tgttattatt ggtagggggt 5700
gggggggaatt aaatatatat t 5721

<210> 161
<211> 6677
<212> DNA

<213> Artificial Sequence

<220>
<223> chemically treated genomic DNA (Homo sapiens)

<400> 161

ggattgaaaa gttttttttg ttttaaagga gtaatgattt tgaagaggtt taattttttg 60
gttaggtttt tagattaaat atttgttttt tgagtagtag cgggtttttt gaggtgaagt 120
ggtaaatatt tttggtttat gattgtaggt aataaaaatt tgttcgggtt gttcgagggg 180
aggggcgtcg gtaaagtcgt agttgtagcg aagtttcgcg tttgtcgttg tcggagtttg 240
gttttttttt tacgcggggg gttgcgggta attggggtcg ataatttgcg gtttcgtttt 300
gggacgtatg tttgttcgag agttggggtt gtagggtgta gtgggatagt aggatcgcga 360
aagtttgggt ttcgagtttc gggttttttg tttttaggcg aagtgcggtt tttacgggtg 420
taggtttcgt tgtataaggg aagatttaga tttcgagttt tcggtaggtt agtttttagt 480
tttttgtttt tttcgggttt tttcggttgt ttttgttttt ttttttttta ttttcgtcgt 540
aatttcgcgt tttacggagg aaggtcgtgg tgcgggagtt gggggatatt ttttttttgg 600
gttcggttta cgtagattta tcgtttgttt cgagtttttg tgtttttaga cgttacggga 660
aaatttgtag ggatatttag cgttaggcgt ggggaattgc gcgtcgtcgg gtcgggaagt 720
ggagtcgaat attgtttttt cgtttttttt tcggtttttt ttttcgtgtt tgggtatttt 780
ttttattttt atttttttgt tttgtcggta aggataatgt ggatttttta tagagataat 840
ggattttgcg gaatggagat aggtaggtag atagatattt ttttttattt taagttatcg 900
atagagtgag aatttattgg attttttagt agttttgtta gggaagtgta ttttaaggat 960
aattttttat tttgaaatgt cggataaaat tttagtaaat tagggtttta gtttttattt 1020
tttaaaaaaa ggtaataagt tattttgggt ttttggtttt tgttttattt ttttgcgttt 1080
tttttatagt gagattagtg ttttttttta aaggaaagaa tttttattgt gtttaatttt 1140
ttttaaggtt ggagaagttt tttttatttt aaagaagggt tcgttgtttt gtatgaggta 1200
aatttggttt tattaaataa tgtttttgat ttggaggggg tttggaattg ttgttgttgt 1260
aaacgatttt tatttgataa agtttaatcg tgttgtgaaa tttaaacgga atatgtttta 1320
aaggtagaaa gtatttaaat gtggttttta ttgtttagtt gttgttaggg tttttttttt 1380
ttataatagg tttggtttgt tgttgttgtt gttgttgttg ttttttttga tatttttagg 1440
ttgagtaaaa ttgggtgttg tttggagagg gaaaattagt tttaaagttg gtagaatata 1500
gatttggtta agaatattgt ttagttattt ttgttagatt taagatttta ttttggaatt 1560
aagattttat ttttatatta aatatttggt tttttataag ttaattagga gggtgttttg 1620
tattttttga ttttggttat taagataagt gtttttaaag gagtttttag gattttattt 1680
tgatagaatt ttgtgatttt ggttttaatg gttattgatt atgtggttaa ttgagtttat 1740
tgttgttttg gtaagtagag taattttaat aaatttaaat tatagtttgt atttgtttat 1800
ttttttaaat taggataaga gtaaaatttg aaagattatt tattaggttg aaaggagaga 1860
atatgatttt tagaatagta ttgatgttta aaaaggatgt ttttggaaga aaaggaggaa 1920
gaggagtaag tgatgggaga atatagtggg attttgggta ttatagggtt attttgagtt 1980
ttttattaaa attaggaata gcggtaaaat tggtttattt gaagaagata tacgtttgga 2040
gatatgtgta gtttttaagg atttttattt aataaagttt atttttgttg ttaaaaattt 2100
ttgtataatg tatttatata taaatataag gtttggtttg tgtttttggt tatttaaaga 2160
aattgatttt tagtaagttt aaatttgaat gaaatgtttt tgtaaattta gttttaaaat 2220
ttttttttta tttggtattt ttggtttgta aattatgtgt tttattagtt tataaataaa 2280
gtagttttga ttttgttttg tatttaatta tagttttagg agttagtaga atttgtttag 2340
aggtgttggg ttttggagtt taagtggata aagttagatt tttttttttt agggtaaagt 2400
ttttttatag ggttgggatt ttaaaggtta tgttggaagt aggtttagta gtaggatatt 2460
aaggggtaaa gtttttaatt taaaattttt ttggtttttg aataattatt aggatggata 2520
gagaaaattt ttgttttgtt ttgagagggt tttatagggt ttttggaagt agagttatta 2580
ttgagaaatt ttttttaatt tgagtagtaa tttagatttt ttttttattt ttgtataatt 2640
taatttgttg aatggaaaat ttagttagaa gtgatgggtt gtttgaaatt aataaaattt 2700
gatatatttt ttttattttt attttatttt attgttaaat atataattga ttattaaaat 2760
gtatttatta gtgagaaaat gaagtagaag tttaaggtat aaaattatag ggatatttaa 2820
ttttaagaga ttgaaagttt gttggtttta tttatatttt agaaatattt taacgaattt 2880
gttttttgga gttataaaaa gttaaaatta tttttatgtt ttttttgttt gaggaagaaa 2940
aaatgagata aagggaaaat aaaagtattt tatggtggtt tatggttggt ggtttggtat 3000
taaaattgtt ttataaagat ttagttaaat tttttttaaa agttagtaat tttgtcgttt 3060
cgttatagtt ggttttttta aaatgttggt tgaattttta ttgttgattt ttgttttttt 3120
tttttaaata gtgaaaaaaa tttttttatt agtaaatttt tattttttat gattttttgt 3180
aaatttaggt tttttttatt ttaaatttga ttatgaatat gtttagaatt ttaaaacgat 3240
gaaacgaatt tttttgtttt ttttttttaa tatttttttt ataatttttt tttttatttg 3300
agttttttat ggttattgcg ttttgtatgt taatattttt aatattagta gtttattttg 3360
aaatgttagg ttaggttaag gagggttttt ttggtttttt tttttatttt atttagggta 3420
tttttggtcg gggaatagtg gtgttgggtg ataagttttt attaattata ggtttagggg 3480
agtgattttt ttggagatgg gttggtttgg ggagtgtatg ttatagaatt aaatgggttt 3540

tgaaggattt tatatttatt tttagaattt tgggtttatt ggatttaata ttgtttttaa 3600
ggtagagttt taaagttata gtaggatttt tattggtgtt ttattagttg ttgagattga 3660
gggatggtgg gttggagttt tgaggttgag gagtaataga aagggatgat gagggatagg 3720
gttgtaattt ttaatatttt ggtagtttgg ttttagagtt ggatttagtt ttattagtat 3780
tgttttgagt ttatttaatt ttttgaagtt agatcgtttt tttgggttgt ttttattata 3840
aaattcgaat ttgtttggaa taagcgtttt tttaatttgt tttttttagt tttttgtagt 3900
tgatagtttg gaaaaggaag cgaaataaag gtttttggga aagtgaagtt tttaattaat 3960
ttggtgtgag aaacgggcgg gagtgggtgg tgattgtaaa atgcgaggtc ggtcggttgt 4020
tggagagata tagaagtttt acggtgggag gttgagtggt tttttttttc ggcgtcgttt 4080
ttagggtttt tttgcgggtc gaagaaggat tcgcgggagt tgagaggttt aggtcggaag 4140
tattttcggt tggtttaaga gtagaggcga agagcgttga gtaggtattt atggattttt 4200
ttttttggga tagattgtta ggtttattgc gtcgatgttt gttttttatt tgtttaagtt 4260
tttgtcgtta gttttaaggg cgttttatgg gtatttttcg aacggatagc gtgtgtgtat 4320
aggaagggtt tgttttaatg ttttttattt gtgtgaaatg tttttgtcgg gtattagtgt 4380
ataaggtagg gtaaattagt ttattcggat ttggggttta gaagtcgatt aattgaggga 4440
tttagtaata ggataaaaaa atgggtttgt ttttatatta ttttgattat ttttgttttt 4500
cgtttttatt ttgttgtgta attcggggag tcgaggagag gtggtaaaaa tagcggttgt 4560
cgagataagg cgtaggtttt ggcgttcgtt ttagtcgtag atagggtttg ggatgggtcg 4620
tcgcgtaatt aattcgtggg ggtggttgta gcgcgtacgt ttgggtcggg ggggagggcg 4680
ggaatgggag gtggattttg taaggggtag gagaggggtg ggggtcggag tgggtgggtt 4740
tagttaggtt tgggtcggga gttaggtttt ttcgcgtttt tatttttacg tggtcgcgcg 4800
tagttaatgg tacgttttcg gcgggggttt tcggggcggg aggcggtttt tcgatcggtt 4860
taggtttttt tttaatttga atttcgtttt tataaacgtt tcgcgatgag ttaatttgtt 4920
ggagggtagg cgggtcggag gcgggaggtt tatagaggga gagagggtta gaggaagagg 4980
gcgggagcga gcgaattaga gagaaaggag aggagggagg aggcgcgtcg cgttatggtg 5040
ttttgcgcgg ggttagggtt agggtcgggg tcgggttagg tcgggttatg agtcgcgtcg 5100
ggagttggga tatggacggg ttgcgggtag acggcggggg cgtcggtggc gtttcggttt 5160
tttttttttt tttatcggtg gcggcggcgg cggcgttagg ttagtgtcgc ggtttttttt 5220
tcgcgtttgt gttcgtcggg acgtattcgg ggcgggcggc ggcggcggta gcggcggttg 5280
cggcggcggc ggcggtagtt ttcggttttg cgtatttcgg gatttttgag cgtacgggtt 5340
ttttttcgtc gtcgtttttt tttgtcgttg tagcggcgcg ttcggaggtt tttttagtta 5400
aagagtgttt agtatttacg tttgtagcgg tcgttgtagc gttttcgagc gttttagcgt 5460
tgggttacgg ttattatttc ggtaacggtt attatagttg tcgtatgtcg tacggcgtgg 5520
gtttatagta gaatgcgttt aagttatcgt cgtacgtttc gttgggaggt tttttcgtgg 5580
agaagtatat ggacgtgtta ggtttggcga gtagtagcgt atcggttaac gaggtgttag 5640
cgcgagttaa ggaggtattt ttttattagg gttatacgag tttttattag tacgtgttcg 5700
gttatatcga tatggtgttt attttcggtt tcggggagtt tcggtacgag gtttatattt 5760
ttatggaggg gtattagttt tggacgttgg ttaacgggtg gaatagttag gtgtattgta 5820
ttaaggatta gttatagggg ttttattttt ggaaattttt ttttttaggt aggggcgatg 5880
gagaaaaggg atcgatacga gggaggggga gagagaagga gaaaagaaag gattaagagt 5940
atgcgttttt atttgggagt gggagttgtg tttgtgtttt atacgtgatt agggttaatt 6000
tagtggaaat ttgtttagtt cgtgtgaaat ttgtaaattt tttttttgat tttaattgtt 6060
ttcgttattg tttttttttt tttgtgtgta gcgtagatac gttttttttt taaagttgag 6120
agtttttgcg tgtttttttt ttgggttttt tttttttttt ttaaagagaa ttttttttta 6180
agaaatttga tagagttggg tttttttatt tttaaagata ttattgtttt aagatgatta 6240
ttaaagtttg ttagattaag tttacgaatt tttaatatag gaacgcggga aattaaaatt 6300
tgagtaaaag atttgagttg gtatgtgttt atgtatgtaa gtttgttttt atataatcga 6360
tatttgtaga aattgttttt atgaacgtgt ttaataatta tattataaaa ttgtaagttt 6420
gttaagatgt cggtttgaga aattatgata attaattaaa ttaattattg ttgttaagtg 6480
taaataattt ttttttaata ataggtaagt gttatgttta tgtatataat aaagattaaa 6540
tagtatggta ttttttaaaa attttttgta tttttgtaaa cgtatatata tatatatata 6600
tatatatata tataattttt agttaggtgt ttcgaatatt ttagtttaat tttttttgtg 6660
tttttgtttg tattagg 6677

<210> 162
<211> 6677
<212> DNA
<213> Artificial Sequence

<220>
<223> chemically treated genomic DNA (Homo sapiens)

<400> 162

tttgatataa ataaaagtat aagaaaaatt aggttgggat attcggagta tttagttgag 60
gattgtgtgt gtgtgtgtgt gtgtgtgtgt gtgtgcgttt gtaggggtgt aggaaatttt 120

taaaaaatgt tatgttgttt ggtttttatt atgtatatag atataatatt tatttgttat 180
taaagaagga ttatttgtat ttaataataa tggttaattt gattgattat tatagttttt 240
taaatcggta ttttaataag tttgtaattt tatggtgtgg ttattgggta cgtttatgag 300
gatagttttt gtaaatgtcg gttgtataaa aataaattta tatatataga tatatattaa 360
tttaggtttt ttgtttaaat tttgattttt cgcgttttta tgttaggagt tcgtgagttt 420
gatttaataa gttttagtag ttattttggg gtagtgatgt ttttgaaaat ggggggattt 480
aattttgtta ggttttttag gaaaaagttt tttttaggag aaaagggaga ggtttaagga 540
aagggtacgt agagattttt aattttagga gaaaaacgtg tttgcgttgt atataggaaa 600
gaaagggtag tgacggaggt agttgaaatt agaggaggaa tttatagatt ttatacgaat 660
tggataaatt tttattgggt taattttggt tacgtgtgga gtataggtat aatttttatt 720
tttaagtagg ggcgtatatt tttagttttt tttttttttt tttttttttt ttttttttcg 780
tgtcggtttt ttttttttat cgtttttatt tggaaaggaa gatttttaaa agtgggattt 840
ttgtggttgg tttttggtgt agtatatttg gttgttttat tcgttagtta gcgtttagga 900
ttggtatttt tttatggaga tgtaggtttc gtgtcgaggt ttttcggagt cgaaagtgga 960
tattatgtcg atatagtcgg gtacgtgttg gtaagggttc gtatagtttt ggtagaagga 1020
tatttttttg gttcgcgttg gtatttcgtt ggtcggtacg ttgttgttcg ttaggtttga 1080
tacgtttatg tattttttta cgggaaagtt ttttagcgag gcgtgcggcg atgatttgag 1140
cgtattttgt tgtaagttta cgtcgtgcga tatacggtag ttgtagtagt cgttgtcgaa 1200
gtggtagtcg tagtttagcg ttggagcgtt cgggggcgtt gtagcggtcg ttgtaggcgt 1260
gggtgttggg tattttttgg ttgggggagt tttcgggcgc gtcgttataa cggtagaaga 1320
ggacgacgac gaggaagagt tcgtgcgttt agaggtttcg gggtacgtaa agtcggaggt 1380
tgtcgtcgtc gtcgtcgtag tcgtcgttgt cgtcgtcgtc gttcgtttcg aatgcgtttc 1440
ggcgaatata ggcgcggaga gaaagtcgcg gtattggttt gacgtcgtcg tcgtcgttat 1500
cgatgaggag gaggaagagg tcggggcgtt atcggcgttt tcgtcgtttg ttcgtagttc 1560
gtttatgttt tagttttcgg cgcggtttat ggttcggttt ggttcggttt cggttttggt 1620
tttggtttcg cgtaggatat tatggcgcgg cgcgtttttt tttttttttt tttttttttt 1680
ggttcgttcg ttttcgtttt ttttttttag tttttttttt ttttgtgagt ttttcgtttt 1740
cggttcgttt gttttttaat aggttagttt atcgcgggac gtttataaaa acgaagttta 1800
ggttgggagg gggtttgggt cggtcggggg gtcgtttttc gtttcgaggg ttttcgtcgg 1860
gggcgtgtta ttggttgcgc gcggttacgt gggggtagga acgcggggga gtttggtttt 1920
cggtttaggt ttggttggat ttatttattt cggtttttat tttttttttg ttttttgtag 1980
ggtttatttt ttattttcgt tttttttttc gatttaggcg tacgcgttgt agttattttt 2040
acgagttgat tgcgcgacgg tttattttag gttttgtttg cgattgaggc gggcgttaag 2100
gtttgcgttt tgtttcggta atcgttgttt ttgttatttt ttttcggttt ttcgagttgt 2160
atagtaaaat gaagacgaag gatagagatg attagaatga tgtgaaaata ggtttatttt 2220
tttattttgt tgttgaattt tttagttagt cgatttttag attttaaatt cgagtgagtt 2280
aatttgtttt attttgtgta ttggtattcg gtaaaggtat tttatatagg taagaggtat 2340
tggagtagat tttttttata tatatacgtt gttcgttcgg aaaatgttta tgaagcgttt 2400
ttggggttgg cggtagagat ttaaatagat gaaagatagg tatcggcgta gtgagtttga 2460
taatttgttt tagaaagaaa agtttatgga tgtttattta acgtttttcg tttttatttt 2520
tgggttagtc gggagtgttt tcgatttggg ttttttagtt ttcgcgggtt ttttttcgat 2580
tcgtagaaag attttgagaa cggcgtcggg ggagaaagtt atttagtttt ttatcgtgaa 2640
atttttgtgt ttttttagta gtcgatcggt ttcgtatttt gtagttatta tttattttcg 2700
ttcgtttttt atattaaatt aattgaaaat tttatttttt tagggatttt tgtttcgttt 2760
ttttttttaa gttgttaatt gtagagaatt gagaggggta aattaagagg gcgtttgttt 2820
taagtaaatt cggattttgt gataggagta gtttagagaa acggtttgat tttaaggggt 2880
taggtgggtt tagggtagtg ttggtggggt taggtttagt tttggaatta agttgttagg 2940
gtgttaggga ttatagtttt gttttttatt attttttttt gttgtttttt agttttagag 3000
ttttagttta ttatttttta attttagtaa ttaatggggt attaatgaga gttttgttgt 3060
gattttgggg ttttgtttta ggaataatat tagatttaat gagtttaggg ttttgggggt 3120
ggatatggga ttttttagag tttatttggt tttgtggtat gtatttttta agttagttta 3180
tttttaagaa aattattttt ttaagtttgt gattggtgaa agtttgttat ttagtattat 3240
tgtttttcga ttaaagatgt tttgagtaag atgaggaaga gagttaggaa ggtttttttt 3300
ggtttggttt ggtattttag ggtaggttgt tggtgttagg agtgttgata tgtaaaacgt 3360
agtaattata aaaggtttaa ataaagggga aagttatgag aggggtgttg gaaaggaaaa 3420
gtaagagaat tcgttttatc gttttgaagt tttagatatg tttatgatta aatttgagat 3480
gggaggggtt tgaatttata ggaaattata gggaatagaa gtttgttgat gagaaaattt 3540
tttttattat ttgggaaaag aaggtaaaag ttagtaatga aagtttaatt agtattttaa 3600
aaaggttaat tgtggcgagg cgataggatt gttaattttt aaagagggtt tggttgggtt 3660
tttatgaaat aattttggta ttaaattatt aattatgggt tattatgaaa tgtttttatt 3720
ttttttttgt tttatttttt tttttttaga tagagaaaat atgaagatag ttttaatttt 3780
ttatgatttt aaaaaataaa ttcgttgaaa tatttttaag atataagtag aattaataaa 3840
tttttagttt tttaaaatta aatatttttg tggttttgtg ttttgggttt ttgttttatt 3900
tttttattga taagtatatt ttaatgatta attatgtgtt taataataaa gtaaaatgaa 3960
aatgggaaga atgtgttaag ttttgttgat tttaagtagt ttattatttt tggttgaatt 4020
ttttatttag taaattaagt tgtgtaggag tgggagaaaa atttgaatta ttatttaggt 4080

tgaaagggat tttttaatgg tggttttgtt tttaaaagtt ttgtgggatt tttttagagt 4140
agggtaaaag ttttttttgt ttattttaat ggttgtttag aagttaggaa gattttgaat 4200
taggagtttt gttttttgat attttgttgt tgaatttgtt tttagtatag tttttggggt 4260
tttagttttg tgggagggtt ttgttttgag gaaagggggt ttgattttgt ttatttgggt 4320
tttaaagttt agtatttttg gataaatttt attggttttt agggttgtgg ttgagtataa 4380
ggtaaagtta gggttgtttt gtttatgaat taatgagata tatagtttat aagttaggga 4440
tgttaggtag aggaggaatt ttgaggttga atttgtagaa atattttatt taggtttaaa 4500
tttattggga attagttttt ttaggtggtt aggaatatag attaagtttt gtgtttgtgt 4560
gtgggtgtat tatgtagggg tttttaataa tagaaatagg ttttgttaag tgagaatttt 4620
tggagattat atatgttttt aaacgtgtgt tttttttagt gaaattagtt ttgtcgttgt 4680
ttttgatttt ggtgaaaaat ttaggatgat tttatggtgt ttaaagtttt attgtatttt 4740
tttattattt gttttttttt tttttttttt tttagaggta ttttttttaa gtattagtgt 4800
tgttttggaa gttatatttt ttttttttaa tttagtagat gattttttaa attttgtttt 4860
tgttttggtt tgggggaatg ggtagatata ggttgtgatt tagatttatt gaagttgttt 4920
tgtttgttaa gatagtaatg agtttaatta attatataat tagtggttat taaggttagg 4980
attataggat tttattagag tggggttttg gggatttttt tgaaagtatt tgttttgatg 5040
gttaagatta ggaagtgtag gatatttttt taattaattt gtaagaagtt aaatatttgg 5100
tgtagaaata gagttttaat tttaaagtaa aattttaagt ttagtagaag tggttggata 5160
gtgtttttaa ttaaatttgt gttttgttaa ttttggagtt aatttttttt ttttaggtaa 5220
tatttagttt tatttagttt ggagatatta aaagaaataa taataataat aataataata 5280
aattaaattt attgtgaaaa aaggggattt tagtaatagt tagatagtgg aagttatatt 5340
tgagtatttt ttatttttga gatatatttc gtttgggttt tataatacga ttaggtttta 5400
ttaaatgaaa atcgtttgta gtaataatag ttttaaattt tttttaaatt agaaatatta 5460
tttggtggaa ttaggtttgt tttatgtagg atagcgggtt tttttttggg atggagagaa 5520
tttttttagt tttggaaaaa attaaatata gtgaggattt ttttttttga agagaagtat 5580
taattttatt gtgaaaggga cgtaggaggg tggggtagga gttagaggtt tagagtaatt 5640
tgttgttttt ttttggaaaa taaaagttgg gattttaatt tgttgaagtt ttattcgata 5700
ttttaaggtg aggggttgtt tttggaatat atttttttag taaaattatt gggaagttta 5760
gtaaattttt attttgtcgg tagtttgaga taaaagaaaa tgtttgttta tttatttatt 5820
tttatttcgt agagtttatt gtttttatgg ggaatttata ttgtttttat cggtagggta 5880
aagggatgaa ggtgaggaaa gtgtttaagt acgaaggggg gagtcgaaag ggaggcggag 5940
gggtagtgtt cggttttatt tttcggttcg gcggcgcgta gttttttacg tttggcgtta 6000
gatgtttttg taagtttttt cgtggcgttt gagggtatag gaattcgagg taggcgatgg 6060
gtttgcgtag atcgagttta ggaagaaagt attttttaat tttcgtatta cgattttttt 6120
tcgtggggcg cgggattgcg acgggagtgg gaggaggaaa gatagaagta atcggagaga 6180
ttcggaaggg atagagagtt agaagttggt ttgtcgaggg ttcgaaattt gagttttttt 6240
ttatgtagcg aaatttgtat tcgtggaaat cgtatttcgt ttgaagatag ggagttcgag 6300
gttcgaagtt taggttttcg cggttttatt gttttattgt attttgtagt tttagttttc 6360
gagtaagtat gcgttttaag gcgggatcgt aagttgtcgg ttttaattgt tcgtagtttt 6420
tcgcgtaaga ggagagttag gtttcggtag cggtagacgc ggggtttcgt tgtagttacg 6480
gttttatcgg cgtttttttt ttcgggtagt tcggatagat ttttattatt tgtagttata 6540
ggttaaaagt gtttattatt ttattttaga ggattcgttg ttgtttaaga aataaatatt 6600
tagtttggaa atttaattaa aaaattaggt ttttttaaag ttattatttt tttagaataa 6660
aaagggtttt ttagttt 6677

<210> 163
<211> 14920
<212> DNA
<213> Artificial Sequence

<220>
<223> chemically treated genomic DNA (Homo sapiens)

<400> 163

gggggaaaaa attaagagtt gtttttaaaa gaagttgttt ttgttggtgt ttagggtaaa 60
aatagaggtt gtcgtttaga tcggtttggt tttggtttta ggtattttgc gagttgggtt 120
cgagtagcgg tcgcgtttcg gcgtgatttt tggaagttgt tagtaggtgt tgtttaaggt 180
atagtagtag ggtcgagaag tcgggattcg aagggtttgg ggtgcgggga agtttcgggg 240
gagggggaag tgaaagagaa attaaattaa ggaaggagaa ggagattttg gttaggattg 300
ggttgattag tttgtgtttc gggtttttga gtttcgtagg gaaaagttga tttttagttt 360
agaatatttt atagaagtta ttttaatttg tttgtttttt ttattatttt tattttgatt 420
tgtatcgagg gaggaggtat tgttattgtg gtgtaggtgg ttgtagaggg gttgtagtac 480
ggttggtagg ttggtttggt aggtgggtat tcggtggtat tagatggtta gcggagtttt 540
agagttatat ttgtgttgtg gtttttttgg ttagagagtt gtagattttt gtgatattta 600
tgtttaagaa tagttattag gttttttaga aatagtatta tttattatta tttgatagtt 660

gtatttgatt tttataggtt tatattatgg attttatttt atagatggga atattgaggt 720
ttgagtttat acggtgagtg agtagaggaa ttgggatttg atttgggttt gttttgtgtt 780
ttttattttt attttacgtt atttatatat atatatatat atatatatat atatatatat 840
atatatatat tattttgtgt ggaggcggag gggatgtttt taggatatta gggtttatgt 900
gtgggttagg ggtgagttta gatgttttga aggttattga ttttattaat ttttagtaaa 960
aagggtggag aagttttttt tttataaagg gtttgagttt atttagtgtt aagttgaggg 1020
gtaggtttgg agtttattat tattaatttt attttgtttt tcgttattta gatttatttt 1080
ttttttttat aatttaatga tttttttttt attttatttt tgtatatgta gtttttagaa 1140
tatttttttt ttttattttt atgtagttta gtatttatat tttttttgta tttagttaag 1200
agagattttt ttatatagaa aatttttggt gttttaatgg aatttgttta agttatatat 1260
taggaggtta gagtttttat attttgtttg tgggtatagt gaaaagttta ttttatgtat 1320
gttaaatttt gtatataaaa taatatattt ggttatatgg tttttattag tttttaattt 1380
tgtatatttt atttaatttg ttatgagaat tgggtttatg ttttaattgt agttttttaa 1440
ataatttttt atcgtttttg ttagaggatt tttttttttt gtgttattta ttagatttgt 1500
taatattttt tttttataaa aaaagtttga ttagttttat ttttatttta aggagtgttt 1560
attttttttt attaatttgt tgttatattt gtaataattt agaaattata ggagttgaag 1620
ttttggtaaa aaagaatgat gtatgagggt ttttgttgtt gttgttgttg tgtgtgtgtg 1680
tgtgtgtgtg tgtgtttggt tagttagaat gagattttaa agatttggga aggatattta 1740
tgaagtttat ttaaagagaa ttattatttt taaagttttt gatttatgaa gtaggtattt 1800
aattttaaga tgtaaaataa tagtgtaagt tggttggggt cgggggagat tattattata 1860
tgattatata gattttttta attttttttt ttaatgaaat agtttatttt aaaaatataa 1920
aattagaatt tttataaaga atattattaa tttttttttt tgaaagaaat aataaaatat 1980
tttgtaaata gaagtaattt tatgaagtaa tagatgtttt atgtaaagat ataaaattga 2040
atttgaatat aacgagacgt gtgtgcgagt agtaaaagtt gaaagttgga tatatttatt 2100
gagatatatt tatttgattt taatgttttt ttaaaaggta gagtaatttt agttagaggt 2160
tttattggtt tagtgtatta tttagtagtg ttttagaagt taggaagggt tttttattag 2220
ataatgaatt atgaaatgtt ttatattgga aaaattagtt attcgttgat gttatgttga 2280
ttttaattaa ttttaaataa agttttagtt tttttttgtt ttagtggtat taatgtgtgg 2340
tgtataaata agtagtatag tataaaattt tatagtgtta atattatgaa gaggagttta 2400
gatagttttt attatatgat ataagagtcg gttggtggaa gagtggggat tagaaaggta 2460
atgtttttta atttttattt ttgagttttt tatatattta aagataggaa agttaggagg 2520
aattttataa ttaattggta tttttaatgt gtattgtgat gtgtattttt ttatattatt 2580
taggtaggtt aatatagttt ttaatagttt tagagtatgt aaatagatta tatgtttata 2640
taagttattt agtatatata tataagtata tatgttaaag agaaagttat ttttaagagt 2700
tatattcgta aatagtaaat ttagggaata tatatatatt tagatgtaga gagaatttaa 2760
atattgataa gttgtattta tttaaatgtt gttattagga tttttgagtt gtttagagtt 2820
attaaatttt tggttgtatt ttagattttt ttgtaaaatt taattgaatt gtaaaatatt 2880
ttgggtattg tatatgtgat tttaaatagg tggatgatgt taagtattat agtataaaga 2940
tttttttataa aattattgta atataaatgt tttttgtttt ttttattttt ttttattatt 3000
ttcgtaaaaa atatgaggtt ttttaggtaa tgttgataaa gttttaataa taatggtgga 3060
gtaattgatg tttttggagt tgaaatttta aaggtgttag gttatttgta atagaaaaag 3120
ttttataaat agttgttttg gaaaagggga tagtatatat aatttagaaa gtattgttaa 3180
tttgtgtaaa ttgtaattat aatttagtgt tataattttt ttgttttttt ttttttgtat 3240
ttagatttta ttttgtagta atgatattta ttaatttttt ttatttaaat tgtattgttt 3300
gattgtaatg ttatgaatat attaggtgtt agatataagt tgagtgtatt tttagaaatt 3360
aagagggttt atgtgtggga aagaaatcga gagggaagga acgttttaat agatggattt 3420
tttaaagatt tttttgtaag ataaaagtaa taagatagaa aatgaaaaag aggggagggg 3480
gaagaatttt ttttaagttt tagaaaggta ttgttaaaaa atttatattt tttttttttt 3540
gtgtatatta aaatttatga tgattttatt tgtattgttt ttttgaggga aaaagaagta 3600
gttaaggagg ttgtttatga atgtattggt cgggataggt ttggggtaag ttgaaaaaat 3660
tattatatga tagagaaaaa taatttgtta atatatttta gagagttttt ttttatagga 3720
ttagttattt aagttatacg agtgtatttt ttttataaaa ggatgtggga aaggttaaga 3780
gaattttgta ttttatttgt gaagttcggc gagtggtggt aggttgtaat gtgtgagagt 3840
gagtgggttt tcggtagaag ggggtagttg aacgcgacgg ggagaaagcg tttttggaat 3900
ttgggtttgt gataggttgt ttgttttttg gttttttagt gttttgttgt attttatagt 3960
tgggaagagt ggagtgtatt atatgatttt aaataaaagt tttattgcgt tttttttaga 4020
ttgtttgtta gtgttgatga tttgaatatt taaatatgaa tgaattgggg gaaaggatta 4080
ttttttttgg attttattag gttagaataa ttttttgtta tttttgagtt tttttttttt 4140
gatttttatt atttgtttat tggatttttt tgtatttttt gttttattac gtggttaggt 4200
gggcgtttta ttatttaggg ttgtggtttg gttgggtgga ggggcggtgg gaatattttt 4260
ttaattaatt tatttttatt gattgagata ttgtgtttgt ttggggtttt tttttttttt 4320
tttaaaaaag gaagaaggtg aaattaggag attgggtaga gaaagaaaaa aaaatagtga 4380
ataaaattag gataatttat tttagaagag aaagtagaat tttcgaaaat ggtgatattt 4440
gaagagaggt gtttgtgagg aagttaagag tagaaggaga gtagtttgtt agaaaacggg 4500
ttgttttttt ttttttaatt atagttttat ttatagtaaa tttttttttt ttttatttat 4560
ttatttattt agggttaatt tttttttttt tttttttttt tttttttttt tttttttttt 4620

tttttttttt tttttttttt tttttttttt tttttttttt tttttttttt tttttttttt 4680
tttttttttt tttttttttt tttttttttt tttttttttt gttttgtttt ttttttattt 4740
tttttttttt gttttgtttt gtttttcgtt tattttgttt tttttttttt ttttgttttt 4800
tttttttttt tttttttttt ttttttttta ttcggagata gttagaattt ttttttttga 4860
tagttataaa tttatagtat tgattgtatt tagagaggaa tttgtaaata aaattttata 4920
gaaaattttt tgttttgttt ttagagaatt tgttgaagag gagaaggaaa aaaaaaatat 4980
taaaaaaaaa aataaaaaaa tttatatata taaaaaaatt tgcgcgtgag gggggaggaa 5040
aagtagggtt ttttaaaaag gtaattataa taatttttgt tgttaggatg tttttgtttt 5100
ggttgagagg atttttgttg gtaagttgtt ggattatagt gaggagtttt tttattttag 5160
gattcgaggg gtatagcgcg gttttcgatt gttcgttttg tgcgttggtc gtttttttaa 5220
aggatgtatt taatttttag ttagagatgg tggaggtcgt taagaagtat atttaaaata 5280
tgttgtattt gaagaagaga ttcgatgtta tttagtcggt atttaaggcg gcgtttttga 5340
acgcgattag aaagttttat gtgggtaaag tcggggagaa cgggtatgtg gagatagagg 5400
atgatattgg aaggaggtta gaaatgaatg aatttatgga gtagatttcg gagattatta 5460
cgtttgtcga gttaggttgg tgttggtatt ggtagggggt ggggaggggt ggggggtggg 5520
agggtaaaat atattttttt gatagtttta ggaggaattt tttttttttt tagttggaaa 5580
ttgtttggga aggttattag ttattaggtg atggtagcgg attagtcgac ggagggtagg 5640
taggggaggg ggagaggatt ttatagaaaa ggaattttcg gtcgagtttt gtttggagat 5700
gattggttta tatttattaa atttagcggg ttatatagag aggaagttcg gggttaatgt 5760
tgagttggaa ggtagattgt gaggggttgt tttgttttgt ttgtgaaatt agatttgagt 5820
agtcggagga aagtcgcggt attttcgggt gttaggggag tagaggaggt tttcggattt 5880
tatttaagtt tttattgagg gtagaggggt gaatgtatta ggattggagt ggaatggtat 5940
agatgaagtt atttttttaa aataaatttt tttttttaaa agtttaattt ggggttatat 6000
tggagaagta gggtatattt atgagtgata gttattttta tttttagaaa atgtttataa 6060
gtgtataggt attatattta agatagggaa gagttatttt gggggatagt tgttattgaa 6120
ttagtagtta tttttgggat attgattttt gttttttgaa agaaaaaaaa ataaataaaa 6180
taattagttt tgtttttttt aaagttatta agagtttttt gttaaggaac gtaattttga 6240
taaagtattt ttagatatta cgttgaattt atttaatttt taagaggaag aattttaata 6300
aatagatttt aatcgttggt ttaggattat attaatttgt tgttgaatag taatatttgt 6360
taaaagtatg ggtttatgga tttggagtta ggttattttt atagttaagt gatatagatt 6420
ttttgtatgt ttgtaagttt atgtacgtta ggtatataga tgtgttaata tcgtgttatt 6480
tttatggtaa tgttaggtta aagaattgta ttttttgtta attttatttg aaaggaatgt 6540
tattgttttg tttggtagag tatatggtta gtaaagtatg tgtagggatt tttagtattt 6600
ttgtttaaaa gtttgtcgta gtagtataaa taattaaggt aatatttaga atttttaatt 6660
tgaaaatttg ttgatttaga tgttttgttt ttaagaaaat tatgaaagat tagggtattt 6720
tttcgggggt gtagatagtt tgttggttgt gtggtttttt tttgtaggag aagttttggt 6780
gttttataag ttttttagtt tggttttttt attagaagtt ttttttattt agggaagatt 6840
ttattttttt tttttaaagt ttttttttaa tttagtttta aaaggtatga ttattaggat 6900
tttatttttt ggagtttttt gaggaagaaa gttaggttgg aattttttaa gttgatattt 6960
attttttttt tttaattttt tatggtggga aaaggtaaaa aagaaaatat ttataatatt 7020
tggttgtgta tgtgaaatag attttagtgg tttagagggt tgttttttgt ttttgtatat 7080
taatttttat atatgatgga tttaatgata gttcgtattt agttgttttt aattatgtta 7140
tatgattaag tatatttatg tttttgatta tttttaatcg tattatcgtt attagtaata 7200
tgtttttttt tttgatttat tggatttgta tagtaaatta tttttttagt atttagagat 7260
ttggtagtga aaaaatttga ttatttatat ttttttttat gtgtggtttt tttttttgtg 7320
atagtttttt tttagttgta tgagattttg ttattttttt attttttagt taggtgttta 7380
gtttgaaaaa aaatgagcga tttttgaaaa agcgattcgt tggacgttta tattgagtta 7440
agtttaagat ttgtaatggg aaatttttgg ttgattttat aatttgattt tggattttta 7500
gaaaatattt attataattg tttatttttt tttgagggat tttatatatt aattttaagt 7560
tgatttaatg taattttatg tatttaatga ttgttgtatg gattgtttat tttttaagta 7620
gaaaattacg tgagtatttt gttgttgatt ttgttagttt agaaaagata tttattattt 7680
aaatgattgt agtaaatata tgaatatgaa tagaaaagat ttatgttttg ttgtttttgg 7740
aataatttaa ttttaataat attttaatta tgtggaatgt taagagtttt tggagaatag 7800
aaaaaggaat gtattttttt tttttttata attattttgt gtagtataga atttagatat 7860
tgttatttat gagtatgtta tagatgtaga tttatagtag ttttttttaa ttttaagatt 7920
ttttaatttg aggttacgtt taatttttgt tttttttttt tttgattgtt ggataaagtt 7980
aaggtttggt tatagaagta gagagttttt aaaagttgaa gtgttgagga tttttttaat 8040
tgagtttgtt ttattttgtt taggtagaaa gtgtttaatt tttgtaaagt tgatgcgagt 8100
tgttattgat taaatagtag ggaattattt taaggttttt tgaaatgtat tttaataaat 8160
aaaaattatt ttatttttat atatgttata ttatttaaga ttttttatta aggttagtgt 8220
gttttttgta tggttggtat aaaattttta atgttttatt ttaaggttta tagagttttt 8280
tataaggaaa gatagaaggg atgggtaaag ttaagttatt attataaatg ttatgaagtt 8340
aaatttagtg gtttttttag ttgatgtagt ttatttttat tttattttga tttatgatag 8400
taggtggtta attaatgtat aataataatg tatttttaag tttttatatg ttgttaaatt 8460
tggatgtggg ttttggtaat tagtttagta attgatagta tttgttgtag aaagaatagg 8520
gaaagaaaga taaatgatat attaagaaat ggatgtgttt acgtagttta ggtgttgtta 8580

agcgtaatat ataagtttat tttttggagg atattatgta tggaagattt gagttagaag 8640
tttgtattgt ttagataggg atttaagaag ttttgtttgg atgattattt tttttatttt 8700
aagaaagtta ttttaaaatg aagttgattt ttattatttt aattttattt ttcgaatttt 8760
tttgtgaatt attttttttt tagggatgtt tttaggggtt atacgttttt tttcgtgaat 8820
aatggggaag agcggttata agagggcgcg ttttaaagat ttttgttttt tgttagtgta 8880
tttagggaaa ggtagttgcg cgtattggaa gtttttgttt gtagcgtttt aattttggtg 8940
attattagtt atcgtttgtt tttttttatt ttgtagaaaa agttaggaat ttatgagatg 9000
ggagaagtat gattatttgt aatatattaa atgagataga aggaaaggaa atggaaattt 9060
tagtgatata tgtgaatatt tcggagtttt atggaaggta gacgagtgat aaattttaaa 9120
agtaaataaa gcggagtagg gagaaatgtg attatgaatt attagcgata ttgatttgtt 9180
tataagtttt tttttaatac ggtttttcgg gtcgtaataa aaatattttt tatttttttt 9240
gattttgttg tttagttttt ttgttttatt aatagttttt attagcgata aatgataagt 9300
gattttaaat agggaggaaa aaggtttttt ttgtaatgtt ttgtgggaat atggtgttcg 9360
gtttttgagg atgcgtttgt tttcgaattg agggtgtgga gagaaatggt gggaaagatg 9420
ggaaaagaga ttaatagtgg tttgcggttt gtgtagtgga tggattgggt tgggggtggt 9480
ggagatgtta gggtggaaag ttcgttgtaa tatttaaaga attgtagtaa tattttttta 9540
ggtatttttt ttgaaaatat atatttgttt tggttttttt tttgttaaaa aatatttgta 9600
cgtgattttt tagttgtagt tttgaatttt tcgtattcga ttggttttta agtttaaaat 9660
gagaagtgtt aaagttttta ggaattttgg aagaagtttt tgaaattttg tgggttaaat 9720
tttaagaatt tttgttattt gtaggaattt aaaatttttt ttttttaaaa tatttaattt 9780
tttttattat tattttaatg gaagaggtat ggatgatgga cgtttgtgtt agggtattta 9840
taaattaata atggatttta aggagggaaa ttaaagttaa aaaattaatt aagataattt 9900
atggttaaat ttatagtttt attgtaggaa agatttttta ttgtaggtta aaggaggaat 9960
aattttaaga ttttatttgt tgttttttgt agaaattttt ggaagtagtt taaatgtaaa 10020
gttagggttt tagagaatgt attttgtaaa tggtagttgt gtatattttt attattgatt 10080
atattatatt taggtatagt gaaattttga tgaagaatgg aaaattaaag gaatagacgt 10140
tttttatagg gaggtttttt ttagttttta ttttatggta agtttaaatg tgttttataa 10200
ataacgaggt tttttatatt tatattagag atataattat tgtttatatg taaaagagta 10260
atatatttta agtaggattt tggtgaggaa aataaaatta aatcgaaata ttgtatgtgt 10320
gtgttgttta tttaattgaa aatgtttata gatttataaa tttgagatgt tggatttttg 10380
gatgagttta gtattttgta ttttagtgta gggggaggga ataataaggg gtttagttgt 10440
ttaatttttg ttatgttttt atgattgatt tatttaaaat atttttaaaa ttttgaaagt 10500
aaaggaaaat gtgttgtaaa tattttttta gttatttttt tttttagttt atatagtttt 10560
ttttttgttt aatttttttt atttagtagt attttagagg tattttttgt gaggtataaa 10620
attttttttt gtttattttt ttgtattttt tttttttttt ttttggtatg tgtttattaa 10680
gtttatagag gtttaagtga tgaaatatta ttatttatat gggtttgcgt gggatttttt 10740
tagtgaatta tttaggtaga gtaggttgtg atatagtttt gtttataagt ttcgggaggg 10800
aaaaaatagt atagaggatg aaaagaatat tattagagag taggtaatga aaggttcgta 10860
aagtaattag ttatatttgg ttagttaggt aatagtgtag gaatttaggt cgtatttgta 10920
taaattaatg tagaatttgt ttacggcgta ttatgggaag atatttattt taagagttta 10980
tagtatgaga tatttgagta aaatttgaaa ttgattagaa attgattttg aatattttag 11040
ttttagttaa tttttttttt tttaaattgt tatgtatgga gattggagga ttggggtatt 11100
ttgaaagtgt tattgtagta tagtaatttt ttttaggtaa tatagagata aagggaatat 11160
aataaattga tttgttattt attatttttt ttatatttag gatatttcga ggtttaatat 11220
atttttgaag tttatagttt tgataaatat taattttacg ttagttttta gtagttttat 11280
ttttttatat ttttttgtaa tgaaagttaa tgtaaaagaa agaaatatgt attgtttaat 11340
aagttagaat ataatagaat ttgtaaaatg tatttgtata taaggtagat attaggaaga 11400
tttgaatttt tttttttttt ttgttttttt gagtattatg gtaatagtat ttttatgaaa 11460
ttagatttgt tttatatttt tttttgataa aatattatgt aatatataaa tagtagtttt 11520
agaaatggat attaggagaa ggaggcgata ggtatgttaa ttagtagggg gaaaatttaa 11580
atgttagaaa tatgggggag agagaaagta cgtaggtagt ggatgtaaat atagggaata 11640
gagtttttta atttagataa ttaaacggat gtataaatta gtatttttgt ttttttataa 11700
tacgtaggcg atgttatgta gttaattatt agttaagatt agtatgaaag ttattttatg 11760
atgatttttt gattttaata taatagtatt tgatagattg agtttttttt aatgagtatt 11820
ttttttgtat aatttttttt taggaatatt aaattatttg tttttaagta tagttgtttg 11880
tgaagaaggg gtggggttag attatgtatt agaaatttgt ttttgtgaaa ttaggggttg 11940
ttttgtaggt tgtgtgattg gaagttagta gttttagttt ttttggtttt aaaaaagtaa 12000
tttgtatacg gttgttttta attttagtta gttaagtaat tttttttcgg tatttttatt 12060
taaatatttg tagttagttt tgggtgttat ttatggaaag tttattgtat gcgggttagt 12120
attgttgttt atcggtttta ttttgatttt ttttggtagg tgtggatgag agtagagtag 12180
agtttatggg gatgagttat tttaaattta ttaagttttt ttattcgggg tattagttag 12240
atattttagt tttatatttt tttttagaaa aataaaattt aattttgtgt tttttgggtt 12300
ttttaattta gtagttatta tttgtttgtt tttggagtga tattttgtgt agtttaggat 12360
tttagaagtt gttttgtata gaggtagaag ggaaaatagt aggtattttg ttaatatttt 12420
ttatggggta aggtagatgt gtttaagttg gaggaaattt gtatattttt taggttagtt 12480
atttaaggtt tgggaggttt ttttcggttt ttttttaagg ttttggtagg attttgttat 12540

tttaaaatta attgttgatt gtttttttag atttaaatga ttaagttttg tttatgggaa 12600
tttttttttt ttttattttg ttaagaaata aataataata ataaaattta tattaatttt 12660
tagtaaaggg gaatatcgaa tttagatttt gaaaaaataa gttattatgt tttttaaaat 12720
aagatagttt ttttttttaa ttgttttttt tgttttggta ttttatttaa ttataaattt 12780
agttttatta tttattttaa tttttgttaa agatatgagg tcggagttag aggaattttt 12840
tttattatcg ttattattat tatggttagt attttcgtaa gtttatagaa agatgttaga 12900
gaattttttt tatttatttg tagttttgtt tggtttttgt tttagtgaat ttattattat 12960
tataattata gtgtgtagag tgtgttaaat taagaataga atttatagta cgtaatgtat 13020
agaaaagttt gtttttttgtt gtagagaatt tgaaattgtg tttaaaattt atagtatcgt 13080
aaatatttta gggtttcgag tgataagtat ggttggattt tcgttaatta tagaaattta 13140
ggtagggaga tttttttagg gagttgtaag ttttaagatg aattttttga agattgtttt 13200
tagatttgtg tatgggatat atgtatagta gatagttgta aggaggttta cggttatgga 13260
aaagaggata tgtttttata tgttattaag tgggaaaaat ggaagtgagg gaaaatattt 13320
gattaattaa aaaattttag tttggatatt tgtttttata gtaattgttg ttgtgttatt 13380
aaatatatag ttgtttagga aaagagaagg aaataggtga ttttttaatt ttagagttag 13440
gttggaattg agtggttgtt aaatttaatt gttttgataa tgtggggatt ttttgtttat 13500
tagtagcgat tgttatttga tatatgtttt tggtgtttat taatttgttt tgtaatgggt 13560
tagaggtaaa aatgggggat ttagaggtat taggtggttt ttttaattta atagaattta 13620
atgttaagga cgtttttatt tgttaataag taattaagtt attggttatt ttttgttaaa 13680
tattagatta tgatagtgat tttagatttc gggtatttta aattaggttg gtaggagggt 13740
ggtggtggag gtgatgtgag gtaagttttt tagaggaatt gttatttaaa taatttgaaa 13800
attgaaatat tattaaaggt agtattaagg gtggtatgtt ggagttgggg taggagtatt 13860
taaggtagaa ggaatagtag gtgttattgt ttgtagttga gaaagggggt ggaattttta 13920
aggttttaga ttttagagaa taggattggg taagagttga tatttaaggg aggaatagta 13980
tgtatagagt ttaaagtttt agttattttt tgttggatat tgagtaatag gaatttagta 14040
aaggattttt aagaagaagt gatgtgatta gatttatttg tgggaaagat gtttttagtt 14100
gttttatgga gaaatgattg gagaggaggt tttttttagt gttgtagagg taaagtaaag 14160
aaaatgttgt tgtagtgatt tagtaaagag gtgatggcgg aggaattagg ttgtgataag 14220
ggaagaagag gttggtttaa gtgtgtttcg attgtataga tttatatagc gaggtggggt 14280
taggggttgt atttttgttt gagaatagta tttttttagt tttttagagt ttatagtgtt 14340
tttttgttta tgttatttta tttgattttt ttaaatttgt gagataatag gttaaatgtt 14400
tttatgttat ttttattttt atagaagatg aaattgaggt tgagtaatta gttaatagat 14460
atagattcgt aggttttttt tattttaaag taagggttta aaattttttt tttataaagg 14520
gttagagagt aaataattta ggttttgtga gttaggtagt ttgttgtagt tacgtagttt 14580
ttggttatta tagtgtaaaa atagttatag gtagtatgta tagaaatgag tataattatg 14640
ttttaataaa atttatttta taggtattaa tgtttaaatt ttaggtaatt tttatatgtt 14700
ataaaatatt attttttttt aattatttaa aagtataaaa gttattttta gtttgtaggt 14760
agtatagaaa tagttttagt ttatgggttg ttatttgttg atttttattt aagagggttt 14820
gttatagaag atttttgttt gtttgaaatt tacgagtgta tgtaaatgtt ggaattaata 14880
ggtgtgtttg ttttttttta tgttgttttt tatttttagg 14920

<210> 164
<211> 14920
<212> DNA
<213> Artificial Sequence

<220>
<223> chemically treated genomic DNA (Homo sapiens)

<400> 164

tttgaagatg aaagatagta taagagaaaa taggtatatt tgttaatttt aatatttata 60
tgtattcgta aattttaggt aagtaggagt tttttgtgat agattttttt gaataggggt 120
taataaatga tagtttatgg gttgaaattg tttttgtatt gtttgtaaat taagaatggt 180
ttttatattt ttaagtggtt aaagaaaagt gatattttgt gatatgtgaa aattatttga 240
aatttaaata ttagtgtttg taaataaagt tttgttggag tatggttatg tttatttttg 300
tatatgttgt ttatggttgt ttttgtatta taataattaa ggattgcgta gttgtaatag 360
attgtttggt ttataaagtt taaattattt attttttggt tttttataga aaaaaggttt 420
tggatttttg ttttagaatg aagagggttt gcgagtttgt gtttgttgat taattgttta 480
attttagttt tattttttat aaaaatggga atgatataag gatatttgat ttgttatttt 540
ataggtttag gaaggttaaa tgagataata taaataaaaa gatattgtaa gttttagaaa 600
gttaaggaaa tattattttt agataaaaat gtagtttttg gttttatttc gttgtgtaag 660
tttgtgtagt cgagatatat ttgagttagt tttttttttt tttgttatag tttgattttt 720
tcgttattat ttttttgttg ggttattgta gtagtatttt ttttattttg tttttgtaat 780
attaaggaag gttttttttt tagttatttt tttatgaagt agttaaaggt atttttttta 840
taggtggatt tgattatatt attttttttt gaaagttttt tattgaattt ttattgttta 900

gtatttaata gaaagtggtt gggattttgg gttttgtata tgttgttttt tttttgaatg 960
ttagtttttg tttagttttg ttttttgggg tttaaggttt tgagaatttt attttttttt 1020
ttaattgtag gtagtgatat ttgttgtttt ttttgtttta agtgtttttg ttttaatttt 1080
aatatattat ttttagtgtt gtttttaata atgttttaat ttttagattg tttggatgat 1140
agttttttta ggaggtttat tttatattat ttttattatt atttttttgt tagtttggtt 1200
tagaatgttc ggggtttaag gttattatta tagtttagtg tttagtagaa agtagttaat 1260
gatttgatta tttattaata aatagaggcg tttttagtat tgagttttat taaattaaga 1320
gagttattta gtatttttga attttttatt tttgtttttg atttattata gggtagatta 1380
atgggtatta gaaatatatg ttaaatagta atcgttgtta atggatagga aatttttata 1440
ttgttagggt agttgaattt agtaattatt tagttttagt ttaattttaa aattagaaag 1500
ttatttattt tttttttttt ttttaagtaa ttgtatgttt aatgatatag taataattat 1560
tatgaaagta ggtatttaaa ttaaaatttt ttgattaatt aaatattttt ttttattttt 1620
atttttttta tttggtggta tatagaaata tatttttttt tttatgatcg tggatttttt 1680
tataattgtt tgttgtatat gtattttatg tataaatttg ggaataattt ttaagaagtt 1740
tattttagaa tttgtaattt tttgggagga tttttttgtt tggattttta tagttaacgg 1800
aaatttagtt atatttatta ttcgaggttt tgagatgttt acggtattat aaattttagg 1860
tatagtttta agttttttgt agtagaaggt aggttttttt gtgtattgcg tattgtggat 1920
tttgttttta atttaatata ttttgtatat tgtgattatg atagtggtaa atttattgag 1980
gtaagggtta agtagggttg tagatggata ggaaaggttt tttggtattt ttttataaat 2040
ttacgaggat gttggttatg atgatggtgg cggtggtaag gggagttttt ttgatttcga 2100
ttttatgttt ttggtaggag ttgaaatgaa taataaagtt gggtttatga ttagatagaa 2160
tattagagta gagagagtag ttagagggag aagttgtttt attttaggaa gtatgatgat 2220
ttattttttt aaaatttgaa ttcgatattt ttttttgtta agaattggtg tgggttttgt 2280
tgttgttgtt tgttttttaa taaaataaaa gagaagaaga tttttataag tagagtttaa 2340
ttatttaaat ttaaaaaggt aattagtagt tgattttagg gtggtagggt tttgttaagg 2400
ttttaggaga aggtcgaggg aaatttttta agttttggat aattagtttg gagggtgtgt 2460
aggttttttt tagtttgggt atatttgttt tgttttatag agaatgttaa taagatattt 2520
gttgtttttt tttttgtttt tatgtaggat agtttttgaa attttaaatt gtatagggtg 2580
ttattttaaa aatagataga tagtagttgt taaattagag aatttagagg gtataaggtt 2640
gggttttgtt tttttaggaa agagtgtaaa attggagtgt ttgattagtg tttcggatag 2700
ggaggtttaa taggtttggg gtaatttatt tttatgagtt ttgttttatt tttatttata 2760
tttgttagaa ggggttaggg tgaagtcgat ggatagtagt gttgattcgt atgtagtgga 2820
ttttttatga ataatattta aggttgatta tagatgttta ggtgggggtg tcgagggagg 2880
gttatttggt tgattagaat tagggatagt cgtgtgtaaa ttgttttttt agggttagag 2940
aggttgaggt tgttggtttt tagttatata gtttatagga tagtttttga ttttataaga 3000
gtaggttttt gatgtatgat ttagttttat ttttttttta taggtagttg tatttggaag 3060
taaatgattt agtgtttttg agagaggatt gtataaagag agtatttatt agagggggtt 3120
taatttgtta ggtgttgtta tattaggatt agagagttat tataaaatga tttttatgtt 3180
ggttttggtt gataattaat tgtatggtat cgtttgcgta ttgtagagag gtaagggtgt 3240
tgatttatgt attcgtttaa ttatttgaat tgaaagattt tgttttttgt atttgtattt 3300
attgtttgcg tgtttttttt tttttttata tttttgatat ttggattttt tttttattaa 3360
ttgatatgtt tgtcgttttt tttttttggt gtttattttt ggaattgtta tttgtatatt 3420
gtatgatatt ttattaggaa aaaatataaa atagatttaa ttttatgaaa atgttgttgt 3480
tataatgttt aggagaatag ggggaagaga gagatttaag tttttttaat gtttatttta 3540
tatgtaggta tattttataa gttttattgt attttgattt attaagtagt gtatattttt 3600
tttttttata ttagttttta ttgtagaagg gtgtggggag atgagattgt tggaaattga 3660
cgtgggattg atgtttatta gggttgtagg ttttagaaat atattaagtt tcggaatgtt 3720
ttaggtatga aaaagataat aaataatagg ttaatttatt atattttttt tatttttata 3780
ttatttgaga gagattgtta tattgtagtg atatttttaa gatgttttaa ttttttaatt 3840
tttatgtata ataatttgaa aaagggaagg ttgattaagg ttgggatgtt taggattaat 3900
ttttaattag ttttaggttt tatttaaata ttttatgttg taaatttttg aaatgaatgt 3960
ttttttatag tgcgtcgtga gtaggttttg tattagtttg tataggtgcg atttaggttt 4020
ttgtattatt atttagttaa ttagatatag ttggttgttt tgcgaatttt ttattatttg 4080
ttttttaata atattttttt tattttttgt gttatttttt ttttttcgag gtttgtgagt 4140
aaggttgtat tatagtttgt tttgtttgag taatttatta agaaagtttt acgtaaattt 4200
atgtgggtga taatatttta ttatttaaat ttttatgagt ttggtggata tatattaaag 4260
agagagaaaa agaggtgtag aaaaatgggt aaagagaaat tttgtatttt atagggaata 4320
tttttgaaat gttgttgggt gggaaggatt ggatagagga gagattgtgt aagttggaag 4380
gaaagatagt tggaagggtg tttgtagtat attttttttt gtttttagaa ttttaaaaat 4440
gttttgaata agttaattat gaaggtatgg taagggttaa gtaattaagt tttttgttgt 4500
tttttttttt tatattaaga tgtaaaatgt tggatttatt taaaagttta gtattttaga 4560
tttgtgggtt tatgaatatt tttagttaaa taagtaatat atatatgtag tgtttcgatt 4620
tggttttgtt ttttttatta gggttttgtt taaaatatgt tgttttttta tatatgggta 4680
atagttatgt ttttaatgtg aatgtggaaa gtttcgttgt ttatagaata tatttaaatt 4740
tattatggga tgggggttgg gggaagtttt tttgtaaaaa gcgtttgttt ttttaatttt 4800
ttatttttta ttaagatttt attgtgttta gatatggtat agttaatggt aagggtatat 4860

ataattatta tttatagggt gtattttttg aagttttaat tttgtatttg gattgttttt 4920
aggggttttt gtagaaagta gtaagtgagg ttttaaggtt attttttttt tgatttatag 4980
tagaggattt tttttgtagt agaattatag gtttggttat aaattatttt gattaatttt 5040
ttagttttaa tttttttttt tagggtttat tattgattta taaatgtttt ggtataaacg 5100
tttattattt atgttttttt tattaaagtg gtgatgaaaa aggttaaata ttttgggaga 5160
aaggggtttt aggtttttat aagtagtaaa aatttttaag gtttaattta taaaatttta 5220
gagatttttt ttaaagtttt tgagaatttt ggtatttttt attttggatt taggaattag 5280
tcgagtacga agaatttaga attatagtta ggagattacg tgtaagtatt ttttagtaga 5340
agaggggtta agataaatat gtgtttttaa gaaaagtgtt tgggaaggta ttattgtagt 5400
tttttaaata ttataacgga ttttttattt tggtattttt attattttta gtttagttta 5460
tttattgtat aggtcgtaga ttattattaa tttttttttt tatttttttt attatttttt 5520
tttatatttt tagttcggaa ataaacgtat ttttagaagt cgaatattat gtttttataa 5580
ggtattatag aaagaatttt tttttttttt gtttgaagtt atttgttatt tgtcgttggt 5640
ggaaattgtt gataaggtag gaaaattagg taataaaatt aggggagatg gaagatgttt 5700
ttgttacgat tcgggagatc gtattaggga gagatttgtg agtaaattag tatcgttgat 5760
gatttatagt tatatttttt tttgtttcgt tttgtttgtt tttgaaattt attattcgtt 5820
tgttttttat agagtttcgg aatgtttata tgtattatta gaatttttat tttttttttt 5880
tttgttttat ttaatgtatt ataggtagtt atgttttttt tattttataa atttttaatt 5940
ttttttgtag gatgagaggg agtaggcggt aattgataat tattaagatt agggcgttat 6000
agataggggt ttttagtacg cgtaattgtt tttttttgga tatattgata aggagtaaaa 6060
gtttttggaa cgcgtttttt tgtggtcgtt tttttttatt atttacggaa gagagcgtgt 6120
aatttttaaa ggtattttta aaggaggggt gatttataga aaagttcgga aggtgggatt 6180
ggaatggtgg gagttagttt tattttgaga tggttttttt ggggtgggga aaatggttat 6240
ttaagtaaag ttttttggat ttttgtttgg gtagtgtaaa tttttaattt aaatttttta 6300
tatatagtat tttttaaggg gtagatttat gtgttgcgtt taataatatt taggttacgt 6360
gagtatattt attttttggt gtgttatttg tttttttttt tttatttttt ttataataag 6420
tgttattaat tgttaagtta attattagaa tttatattta ggtttgatag tatatggaga 6480
tttagaggta tattattatt atgtattgat tgattattta ttgttatgag ttaaaataaa 6540
ataaaaataa gttgtattag ttgaaaaggt tattgaattt agttttatga tatttatagt 6600
gatagtttgg ttttatttat tttttttatt tttttttgtg aagaattttg taggttttaa 6660
ggtaaaatat tgagggtttt gtgttagtta tgtaaagaat atattaattt tggtgagaaa 6720
ttttgggtga tgtgatatat ataaaaataa aataattttt gtttgttggg atatatttta 6780
gagagtttta gagtgatttt ttgttgttta gttaataata attcgtatta attttataaa 6840
aattgagtat tttttattta aataggatgg gataaattta gttaaaagag tttttagtat 6900
tttagttttt ggaggttttt tatttttgta gttaggtttt ggttttattt aataattaag 6960
gagagaaaag gtaaaaatta aacgtagttt taaattgaag aattttgagg ttgaaagggg 7020
ttgttatggg tttgtatttg tggtatgttt atgggtgata atgtttggat tttatgttgt 7080
ataaaatggt tataagggaa agaaaaatgt attttttttt ttatttttta aaaatttttg 7140
atattttata tagttggaat gttattaaag ttgaattatt ttaaaagtaa taagatatag 7200
gtttttttta tttatgttta tatatttgtt ataattattt ggatgataaa tgtttttttt 7260
ggattagtaa aattagtagt aaaatgttta cgtggttttt tgtttgaaga gtagatagtt 7320
tatatagtaa ttattaaata tatgaaatta tattaaatta atttgaagtt gatgtgtgaa 7380
attttttaga gaaaaataag taattgtaat aagtgttttt taagaattta aagttaagtt 7440
gtaaagttaa ttaggagttt tttattataa attttaaatt tgatttaatg tgaacgttta 7500
acgaatcgtt tttttaaaag tcgtttattt ttttttaaat taaatattta attgagaagt 7560
aaaaaaatga taagatttta tataattgag agaagattgt tatagaaagg aagattatat 7620
ataagaaaga atgtaaatgg ttaaattttt ttattgttaa atttttaaat gttaagagag 7680
tgatttgtta tataagttta gtaagttaga aagaggggta tattgttagt aacggtagtg 7740
cggttaaaag tggttaaagg tataggtata tttagttata tgatatggtt aaaggtaatt 7800
ggatacgagt tgttattgaa tttattatgt gtggaggttg gtatataaag ataggaagta 7860
attttttaga ttattgaaat ttgttttata tgtatagtta aatattgtaa atattttttt 7920
ttttattttt ttttattatg aaaagttgag aaaaaaaaat gggtgttaat ttgaaaagtt 7980
ttaatttagt tttttttttt aaaaagtttt agaagataaa gttttaatga ttatgttttt 8040
tggggttggg ttaagaaaga gttttaggga aagaaagtag gatttttttt aagtgagaga 8100
ggtttttggt gagagagtta aattgggaag tttgtaggat attagggttt tttttgtaag 8160
gggaagttat atagttagta ggttatttgt attttcggaa agatgttttg gttttttatg 8220
gtttttttga aaatagaata tttgagttaa taggttttta gattggaaat tttgagtgtt 8280
gttttaattg tttatattgt tgcggtaaat ttttaaataa aaatgttggg agtttttgta 8340
tatattttat tggttatatg ttttattaaa taaaataatg gtattttttt taaatggagt 8400
tggtaaagga tgtaattttt tagtttggta ttattatgaa agtaatacgg tattaatata 8460
tttgtgtgtt tgacgtgtat gggtttgtag gtatgtaaag gatttgtgtt atttaattat 8520
aaaaatagtt tggttttaag tttatgggtt tatatttttg ataggtattg ttatttagta 8580
ataggttagt gtggttttgg gttaacgatt agaatttatt tattaaagtt ttttttttta 8640
aggattgagt gagtttagcg tagtatttga gagtattttg ttaaggttgc gttttttggt 8700
agagagtttt tagtaatttt agaaagaata aaattggttg ttttatttat tttttttttt 8760
tttagagagt aaaagttagt gttttaaaag taattgttgg tttaatgata attgtttttt 8820

aaagtagttt ttttttgttt tgaatgtggt gtttgtgtat ttatagatat tttttaaagg 8880
taaaaatgat tgttatttat aaatatgttt tgttttttta atgtggtttt agattggatt 8940
tttaaagggg aaggtttgtt ttaagagagt gattttattt gtgttatttt attttaattt 9000
tggtatattt atttttttat ttttaataaa aatttggatg gggttcggaa gttttttttg 9060
tttttttagt attcgaaaat gtcgcggttt tttttcggtt gtttagattt ggttttatag 9120
gtagggtaag gtagtttttt atagtttgtt ttttagttta atattggttt cgagtttttt 9180
ttttgtgtga ttcgttgggt ttagtaagtg taagttagtt atttttaggt agagttcgat 9240
cgagaatttt ttttttgtaa agtttttttt tttttttttg tttgtttttc gtcggttagt 9300
tcgttattat tatttaataa ttaataattt ttttaggtag tttttagttg aagggaaaag 9360
aagttttttt tgggattgtt aaagaaatat attttatttt tttatttttt attttttttt 9420
attttttgtt aatgttagta ttaatttgat tcggtaaacg tgatgatttt cgaggtttgt 9480
tttataagtt tatttatttt tgtttttttt ttaatgttat tttttatttt tatatattcg 9540
tttttttcga ttttgtttat atgaagtttt ttgatcgcgt ttagaagcgt cgttttgggt 9600
atcggttggg tgatatcggg tttttttttt aagtgtagta tgtttaaaat gtgttttttg 9660
acggttttta ttatttttgg ttgagagttg ggtatatttt ttgggagggc ggttagcgta 9720
taggacggat agtcgggggt cgcgttgtgt ttttcggatt ttggggtggg ggaatttttt 9780
attataattt agtaatttgt taatagaaat ttttttagtt aaagtaaggg tattttggta 9840
gtaaaagttg ttgtgattgt ttttttaaaa ggttttgttt tttttttttt tttacgcgta 9900
ggtttttttg tgtgtgtgga tttttttatt tttttttttg gtgttttttt tttttttttt 9960
ttttttagta aattttttgg aataagaata aaaagttttt tgtgaagttt tgtttgtagg 10020
tttttttttg aatgtagtta gtgttgtagg tttgtggttg ttagggagga gagttttgat 10080
tgttttcgag taagagagaa ggaaggaggg agggagggag ggagataggg agggagggag 10140
agatagggtg gcggggagat agatagagat agagagagag gatgggaggg agatagagat 10200
agagagagag gagagagggg gagagaagag agagggaaag aagggggaga gagagggaga 10260
gagggaggga agggaggggg agagggagag agggaaagga agagagagag agagagagag 10320
agagagagag agagagaaag gattggtttt aagtaagtga gtgaatggag agggagggag 10380
tttgttgtga gtagggttgt gattagggag ggagggatag ttcgtttttt gataggttgt 10440
tttttttttg tttttagttt ttttatagat attttttttt aaatattatt attttcgggg 10500
gttttatttt tttttttaaa ataaattatt ttaattttgt ttattatttt tttttttttt 10560
tttgtttagt tttttggttt tatttttttt tttttttgga ggggagaaaa gaaattttaa 10620
ataaatatag tgttttaatt aatagggatg aattgattga aaaagtgttt ttatcgtttt 10680
tttatttagt taagttatag ttttaggtgg tagaacgttt atttggttac gtggtggggt 10740
agagggtgta gggaggttta gtggataggt agtggaggtt agggaagagg gatttagggg 10800
taatagagga ttgttttggt ttgatgggat ttaggggaga tggttttttt ttttaattta 10860
tttatattta aatgtttagg ttattaatat tggtaggtaa tttgaggaga gcgtaatgga 10920
atttttgttt ggggttatat aatatatttt atttttttta attgtgaaat gtagtagggt 10980
attgaaggat tagagggtag gtagtttgtt ataagtttaa gttttagaag cgtttttttt 11040
tcgtcgcgtt tagttgtttt tttttgtcgg ggatttattt atttttatat attatagttt 11100
attattattc gtcggatttt atagataaaa tgtaaaattt ttttggtttt ttttatattt 11160
ttttataaaa agagtgtatt cgtatgattt gaataattgg ttttatggga aaagattttt 11220
taaaatatat tggtaaatta tttttttttg ttatgtggta gtttttttag tttgttttaa 11280
gtttgtttcg attagtgtat ttatagatag tttttttgat tgtttttttt ttttttaaag 11340
gaatagtgta gatgaaatta ttatggattt tagtgtgtat agaaaaagaa aaatgtgaat 11400
tttttaataa tgttttttta aggtttaaaa aaaatttttt tttttttttt tttttttatt 11460
ttttgtttta ttgtttttat tttgtagaag aatttttaag gggtttattt gttaaagcgt 11520
tttttttttt ttcggttttt ttttatatat aagttttttt ggtttttgaa gatatattta 11580
gtttatattt gatatttagt gtgtttatag tattatagtt aagtaatgta gtttaggtgg 11640
aaagagttaa taaatattat tattataaga taagatttga gtgtaagggg aagagggtaa 11700
gaaaattgtg atattaagtt ataattatag tttgtatagg ttaataatgt tttttgaatt 11760
atatatattg tttttttttt taaaataatt atttgtaaga ttttttttgt tataagtaat 11820
ttaatatttt tggggtttta gttttagaaa tattaattat tttattatta ttgttaaaat 11880
tttgttaata ttgtttaaaa agttttatat tttttacggg gatggtgaaa gagaatgggg 11940
gaggtagggg atatttgtgt tataatggtt ttataaaaaa tttttgtgtt ataatattta 12000
atattattta tttatttgga gttatatata tagtatttaa aatgttttgt agtttaatta 12060
agttttataa gaaagtttga aatataatta aaagtttaat ggttttaaat aatttaggag 12120
ttttaatagt agtatttaga taagtgtaat ttattaatat ttggattttt tttgtatttg 12180
agtatgtgtg tgttttttga atttattgtt tgcgaatgta atttttaaaa atagtttttt 12240
ttttggtata tgtatttgta tatatgtgtt gagtggtttg tataaatata taatttattt 12300
gtatgtttta ggattattaa aagttgtatt aatttgtttg aataatataa aaaggtatat 12360
attataatgt atattggaaa tgttaattag ttgtaaaatt ttttttagtt tttttatttt 12420
tgaatgtgta aagagtttag aagtaagagt taaaaagtat tatttttttg gtttttattt 12480
ttttattagt cggtttttgt attatgtggt aagagttgtt tgagtttttt tttatggtat 12540
tggtattgtg aagttttata ttgtattatt tatttgtata ttatatattg gtattattga 12600
aatagaggag ggttggggtt ttgtttggga ttggttggaa ttagtatgat attagcggat 12660
gattggtttt tttagtgtga gatattttat aatttattat ttaatggaaa gttttttttg 12720
gtttttgaga tattattggg tgatgtattg agttagtgaa atttttggtt aggattattt 12780

tattttttag aaaaatattg gaattaaata gatatgtttt aatgagtgtg tttaattttt 12840
aatttttatt attcgtatat acgtttcgtt atatttaggt ttagttttat gtttttatat 12900
aaaatatttg ttattttatg aagttgtttt tatttataaa gtattttatt gtttttttta 12960
ggaaagaaaa ttggtagtgt tttttatagg gattttaatt ttatattttt aaaataagtt 13020
attttattga ggaaaaaaat tgggaaaatt tgtataatta tatgatgatg gtttttttcg 13080
attttaatta atttatatta ttattttata ttttgaaatt gaatgtttgt tttataaatt 13140
aagaattttg gaaatagtaa ttttttttaa atgaatttta taagtatttt ttttaggttt 13200
ttagaatttt attttaatta attaaatata tatatatata tatatatata taataataat 13260
aataataaaa atttttatgt attatttttt tttattagaa ttttaatttt tataattttt 13320
aaattgttat aaatataata gtagattaat gaaaagaagt gaatattttt tgaaataggg 13380
gtaaagttgg ttaggttttt tttgtggggg aggggtgttg gtaagtttgg taaataatat 13440
aggaggaagg ggttttttga taagaacggt gaagggttgt ttgaaggatt atagttgaag 13500
tataagttta gtttttatga taagttggat aaaatgtgta aaattgggag ttaatgagag 13560
ttatatgatt agatatgttg ttttgtatgt aggatttaat atgtataaaa taaatttttt 13620
attgtgttta taagtaaaat atagaggttt tggttttttg atgtgtggtt taagtaggtt 13680
ttattaggat attagaagtt ttttgtgtaa aagggttttt tttggttaga tatagagaaa 13740
gtgtgggtgt tgagttgtat gggggtgggg ggaaggagta ttttaagaat tgtatatgta 13800
gaaatgaagt aggaaaaaaa ttattaggtt gtagaggaag agagtgaatt tgagtggcga 13860
agatagaaat aggattaatg atggtgaatt ttaaatttgt tttttagttt agtattgggt 13920
gaatttaaat tttttataag aaagaaattt ttttattttt tttgttgaga attagtggag 13980
ttaatgattt ttagggtatt taaatttatt tttgatttat atatgaattt tagtgttttg 14040
aaaatatttt tttcgttttt atataaagta gtgtgtgtgt gtgtgtgtgt gtgtgtgtgt 14100
gtgtgtgtgt gtgtgtaggt gacgtggggt gggggtgggg ggtataaaat aaatttagat 14160
tagattttag tttttttgtt tatttatcgt gtaaatttag gttttagtgt ttttatttat 14220
aaaatgggat ttatgatatg aatttgtggg ggttaagtgt agttgttaga taataataag 14280
tgatattatt tttgagaagt ttggtggttg tttttaaata tgggtgttat aggaatttgt 14340
agttttttga ttagaggagt tataatataa gtgtgatttt gaaatttcgt tggttatttg 14400
atgttatcga gtgtttattt gttaggttag tttattagtc gtgttgtaat ttttttgtaa 14460
ttatttgtat tatagtggta atgttttttt tttcggtgta aattaaggtg ggggtggtgg 14520
aggaagtagg taggttaaaa tagtttttgt ggaatatttt agattgggaa ttagtttttt 14580
tttgcggaat ttaagggttc ggggtataag ttggttagtt tagttttgat tagaattttt 14640
tttttttttt ttggtttggt ttttttttta tttttttttt tttcggagtt ttttcgtatt 14700
ttaggttttt cgggtttcgg tttttcggtt ttgttattgt attttgagta gtatttgttg 14760
gtagttttta gggattacgt cggaacgcgg tcgttgttcg agtttagttc gtaggatgtt 14820
tggagttagg gttaagtcgg tttaagcggt agtttttatt tttattttga gtattagtaa 14880
gggtaatttt ttttaagagt agtttttgat tttttttttt 14920

<210> 165
<211> 6109
<212> DNA
<213> Artificial Sequence

<220>
<223> chemically treated genomic DNA (Homo sapiens)

<400> 165

tttattcgag gatttttttt taggatattt tatgaggttg tttttcgagg gttgaatttt 60
gagatttaaa ggaaggttat ttgaggtatt ttggtatatt ttcgggtttt taattttttt 120
ttttgtttgg ttggtaattt tttgttgttt tttttttttt tttatttgat ttttttcgag 180
gtattttcgt tttttttttt tcgttcgttt ttagtaaatt tttagcgggt gggggaaatt 240
aattttttgg cgttaaagtt aaatttagag ttgttcgttg gataggattg tagtggggta 300
aatggtgttc gtttttttag aaagaatgat taattatatg gaggtaattt gttttgggtt 360
ttttgagatt tcgggcggat tcgttcgtgt tttgttagat ttagtgtaaa tgtagatttt 420
agttgaatcg gttaggattt gagagatagt tgttttattt ttcggattgt aattattttc 480
gcgagaattt gatatattta atgaaagttt tgttaaatgt acgggttagc gcgtttttgg 540
gttattatta tttgggttag tatattgtat ttattagcgt taatttgggt tttacgtagt 600
agaattcggt taaaaatcgt ttgtttttgg ttatttagga gttttagggg gtagggggaa 660
gtaagaaaaa ggtttatttt gatattgtta gtcgtttttg ataagttata gtaaatattt 720
ttagaagtat aattttaaag ggagaatata aattttcgtt ttcgtttggt cgaacgtttt 780
tttgtaatta attgcggatt ttcgtatgtt taggattgaa ttgaggtttg tggtttcgta 840
gttgtttcga tgtcgggagt atgaaatttt atttcggttg agaatcgagt aatagatcgt 900
ataagaacgg tgttttttgg tttgtcgttt gttaagaaat attaggaaag ttggtgttag 960
aaataattat ttgaatttag aaagtaaggt taggaagttt tgcgtgtaga gaaaattttt 1020
gttaatttat ttagattgtt ttatttggtt aataaagtaa ttgatattta ataaaattat 1080
aatcgtagta ggagttttag gtattagaat aagaattata attaaaggtt tgttgttgtt 1140

aataattgtt tttattatta ttgtttagta tttttttaag taaaaggaaa gaaaaagaga 1200
attattttat atttattggt attttttttt tttttttgaa attagagtat agattttttt 1260
gattgttttt taataagtta ttattattaa cgggaagaga cgtaaattgg ttttaaatat 1320
ttatatttcg ttcgcggtgg aatataaatg gttaatcgta atttttttta aacgtatata 1380
atttatattg attttggat atgattaaag atattgagga tttatatagt tttattttat 1440
attattagag attatataga aaagataaaa acgggaaagg ggatatatac gtatttattt 1500
aatatttatt tttagaggta aataaaattt ttttgtttta ttttggtttt taaattatat 1560
gatgtggata ttagaaaata taaatttttt agttatatat atttttttat ttgaatattt 1620
tatttagtaa aattggtagt gtaatttatt ttattttttt aaatttgatt ttgatgtgag 1680
ttaaatatat agtattattt ttttgggaat aaatttttaa atttatattt ttattggaaa 1740
gttgattaaa gttttttttt tttttttagt tttggttaga tgagaaggaa taagttgttt 1800
tattgtttgt gattttttaa agaaataatt taaaatttat ttatatttgt tagaggttat 1860
tgggagataa ataaatgtta aatgtgtaat tttatttttt aattgtaatt aaaatttatt 1920
tagatttttg aaatgtagtt tgagagtatg atgtttgtgt aggaatttgt atttgatatt 1980
aagtttgatg tggaatgttt aaatggtaat taaagtagag aaagtaatag tatataaaaa 2040
tataagttta agagtttatt aggaggttgg gtgtaatagt tatattttgg aaggtgtata 2100
taagggtata tgggtagatg ttatagattt tagaggaatt taaattttag tatttttttt 2160
atattgaaag atattgttag atatattgag gtagtttgga ggtgatgggg attatattta 2220
ataattattt atatttttta tttttttagt aaaggaatta aattagagat ttatattatt 2280
gtcgttttta aggatatatt tttgatttaa taatatataa aggttaaaac gagttttttt 2340
ttgagaggag agggtttaat tttcgttttg aatataagag gtcgtttttg tgattaagta 2400
ttacggataa attgtttatt agttttatgt atataaaatt ttatagatat atttgatgta 2460
ttaaatttat atatagtaat tagttgagga aattataata atttatgatt aatttagaat 2520
tttaaggtaa agtaaaatta taaagtttgt tattttaatt tggattttaa tgtaaaatgt 2580
gttatgatat atgtattgaa ttttagagta tgtttattat ggtggggttt tttaaacgtt 2640
tttttttaat gttggtattt ttattatgcg ttttgttttt attagtttag gaatttatta 2700
agaaaatatt tgtttataat aatggtgtag aatgaaaaga ggaaaatgtt gtttttatta 2760
ttaaggtgaa attgtttatt attaaaggtt ttttttaagt cgtatgtaag tttttttatt 2820
tggaagtgtt attatttgta ataatttttt gtaatagttt attggtttag ggagttaaaa 2880
tattatattg tgggtgggag gtgggggttg gtttgaagga ggatggatag tgagggtgtt 2940
ttgtggattt taaatgtaag ttattaggtt tttggtggtt tttggtgttt aattttattt 3000
tataaaagtg gtatgatgga atatgattag gtaaaatttt attttttggt attgtggtgg 3060
tatttttttt tttttttttt tttattaggg ttaatatgtt tatttttatg ggaaatatat 3120
ataaatatta ggaagatttg tttcgtattg agattaatag tattagtttt gtaaaagtag 3180
tttgataaaa ggttttatat atttaatatt agagattaga aatagattaa aaaaaaaaaa 3240
aaaaaaaaat atatattata taattggtaa atttattaat ttaggtttat ttaattttcg 3300
tttttatata cgcgttttaa aataatttat aataaaaatg gaagggaaga tagatttaat 3360
ttgaaatttt ttttgagaaa aaataaatta aaattagttt ttggggaaga aagttttagt 3420
taggttagga ggaaatttac gtatttttga tttttttttc gttttggaga ggtattattc 3480
gtttaagttt agttaatgcg gtcggttagg atttatagtt tttattaagg gttagatttt 3540
gcgaaagata taaagaaagg agttttttaa tatatcgggt ttttttatag taataagata 3600
tcgaaattaa gttttttatg gtttgtgttt gtaggatttt ttagataaaa ttggtagttt 3660
tattagataa gaaatggttt tttagaagtt ttgggggaag cgcgggggtt tttcgtggtt 3720
tttttgtgat tgtttttggg agatcgtaat agatgatggg aatatgtaag aatgattgaa 3780
gaaggttatc ggcgagttgt atttaatagt tttttttttt taagtagttt ttcgagagaa 3840
aattatatat taacgtttgg ggacgtgaaa taggtttttt attgttttgt aataaatttt 3900
tttttattcg cgtttttaga ttaatgtatt tttaaaaaat ttttataaag ttatggagat 3960
ttgtttttat aattattaaa atattatagt tagttgtata agtttgttta aaaataaagg 4020
gtaggagatt agtagtgaga agaattgaat agtatataaa aattttataa attttagatt 4080
attgaaagat tatttcgttt tttggtttcg ggttagtgag ttttaatttt agagttaaat 4140
tgagaagtag atttcgcggg tttgaaagat aaaaagttag ttcgcggagt taatttcggt 4200
ttatcggtgc gttttaattt gattgtttta atagtaagaa gcgttttttt ttttttattt 4260
aacgttttta gatatttgag gttgggggag acggagtaat ggtgtagtag ttaggaaata 4320
gtttttttgg gtttaattat tttattttcg attttttttt ttttagcgcg tcgagcgtag 4380
ttagttttgg aggaattagt ttttcggagt agttaaggta ggttaggttt cggggatgtg 4440
tattacggcg tggggattaa attaagttga acggtttgtt ttaagttttt tttttttttt 4500
agagttcggg agggatttcg ttaataggta gatttattta ttagttattc gttatcggcg 4560
gtagaaagtg agtttcgcgc gtaacgcggt tagtcggatt gcggggattt taggagcgta 4620
gggcggagga gtagcgttat aggaggcgag ggcgtaggcg gcgcggtcgg gaaggaacgc 4680
gggaggggat agaaggaaga ggaagaggag gagagggagg ttagagttag aatagttcgg 4740
tagttcgagt ttcgggggag aacggtttga gtttcgagta agttgtttcg ggagttttaa 4800
ttttttttcg ttggttcgtc gagcggttag tggcgtttag cggcggcgag gttgaaatat 4860
gataattaga atagttgcgt cgcgcgtttt gtagttaatg ggcgcggcgt tcgtttgacg 4920
ttttcgcgcg ttgcgttaga ttaatggcga tggagttgag ttggagtaga gaagtttgag 4980
taagagataa ggaagagagg tgttcgagtc gcgtcgagtt tgtcgtcgtc gtagcgtttt 5040
cgtttcgtta atttcgtcgg tttaaattgg atttttagat tcgcgagggc gcggcgtagt 5100

cgagtagcgg tttttttagt attggtaatt ttaggggtta atatttttta tttagttata 5160
gttttagtat tttttttgtg ggttgtttat taattgtata attattattt tattgtggat 5220
attatttttt tttatagata tgggagatat gggagattta ttaaaaagta agaggttatt 5280
ttattttgtg gggttcggtg tgttgttttt gtgcggggtt tttttttagg tataggttga 5340
ggtgttaagg gttttttgga gttggagtta ttgtttggag aaagagaaaa ggtggttttt 5400
ttttgttgtc gttacgtttg tatgtttatt gtcggttttt attttcggga aattgattgt 5460
attttgtgtg tgaattcgtt tgtgtgtttt ttaaagtttt agttttttgg tgttaagcgg 5520
tgattttttt ttggggaatt ttgagttttt cgagaaggtt attatgttgt aaaggtttgt 5580
ttgtatagtt aatgtttaga gatgtgaatt agtattagat ttgtaaaaga gaacgagtga 5640
taattgtatt tatgtttgtt tttgttaata atatttagtt ttgtgtgtta tttaagagcg 5700
cgtttacgg aaaatataga tattttgcg tttatttaac gttttagtt aaaattttt 5760
ttttgatttg atttatttat aatttttttt aatgattata gtaaagagga aggggggggg 5820
gagaaatata aaatgagcgg gtttgattgc gtgttaggcg tataaatgta gattatttta 5880
atttgtattt tatatatatt ttattttttt ttaaaaatga gttaaggttt tgatggtata 5940
ttttaattat tattttaaag tgtaatgttt taaaaaaaaa aaaaaagaaa gaaagaaaga 6000
aagaaaaaaa ttttttagag tacgttttat aagagaacga tattaaaagt gtgtttattt 6060
ttgtaggaag taaacggtta gttaattatg tatttatttt tattttaga 6109

<210> 166
<211> 6109
<212> DNA
<213> Artificial Sequence

<220>
<223> chemically treated genomic DNA (Homo sapiens)

<400> 166

tttgaaatga aaataaatat atgattgatt aatcgtttat tttttataga gataaatata 60
tttttagtgt cgttttttta tagggcgtat tttgggaggt tttttttttt tttttttttt 120
tttttttttt ttttttttag agtattgtat tttgggatgg taattgaaat gtgttattaa 180
aattttgatt tatttttaaa aggaggtgga atatatgtaa aatgtagatt ggaatagttt 240
atatttgtac gtttagtacg taattaaatt cgtttatttt gtattttttt tttttttttt 300
tttttttgtt ataattattg ggaaagatta tggataagtt aagttaaaga aaaggttttg 360
attagaagcg ttaagtgaac gtagggatgt ttatattttt cgtgaagcgc gtttttaaat 420
agtatatagg attggatatt gttagtaaga gtaggtataa atatagttgt tattcgtttt 480
tttttgtaag tttaatgtta atttatattt ttgggtattg attgtgtagg tagatttttg 540
taatataata attttttcgg agagtttagg attttttagg aggaaattat cgtttagtat 600
tagaaagtta gagttttgga gggtatatag gcgaatttat atataaggta taattagttt 660
ttcgaagata agaatcgata gtaagtatgt aggcgtggcg gtaataagaa aaagttattt 720
tttttttttt tttaggtaat gattttaatt ttaaagagtt tttggtattt tagtttgtgt 780
ttgagagaga atttcgtata agaatagtat atcgagtttt ataaggtaaa atagtttttt 840
attttttggt ggatttttta tgttttttat atttgtaaga gggagtaatg tttatagtga 900
aatggtggtt gtatagttgg tgaatagttt atagagagga tgttggagtt gtggttaagt 960
gggaaatatt ggtttttggg gttgttaatg ttgaaagagt cgttgttcgg ttgcgtcgcg 1020
ttttcgcgga tttaggagtt taatttaagt cggcggagtt ggcggagcgg aggcgttgcg 1080
gcggcggtag attcggcgcg gttcgggtat tttttttttt tattttttat ttaaattttt 1140
ttgttttaat ttagttttat cgttattggt ttgacgtagc gcgcggggac gttaggcgag 1200
cgtcgcgttt attggttgta gggcgcgcgg cgtagttgtt ttgattatta tattttagtt 1260
tcgtcgtcgt tgagcgttat tgatcgttcg gcgagttagc gggagaggat tagggttttc 1320
gaggtaattt gttcggggtt taggtcgttt tttttcgaag ttcgggttgt cgggttgttt 1380
tggttttggt tttttttttt tttttttttt tttttttttg ttttttttcg cgtttttttt 1440
cggtcgcgtc gtttgcgttt tcgttttttg tggcgttgtt ttttcgtttt gcgtttttgg 1500
ggttttcgta gttcggttgg tcgcgttgcg cgcggggttt attttttgtc gtcggtggcg 1560
agtggttggt gggtaggttt atttgttgac gaggtttttt tcgggtttta ggaaaagggg 1620
ggaatttgga gtaggtcgtt tagtttggtt tggtttttac gtcgtggtgt atattttcgg 1680
ggtttggttt gttttggttg tttcggaggg ttagtttttt tagaattggt tgcgttcggc 1740
gcgttagggg aagaagaatc gggaatggga tgattaaatt tagaggaatt gttttttgat 1800
tgttatatta ttatttcgtt ttttttaatt ttagatattt aaaaacgttg ggtgggagga 1860
aaggggcgtt ttttgttgtt gggataatta ggttggaacg tatcggtaaa tcgaagttga 1920
tttcgcggat tgattttttg ttttttagat tcgcgaggtt tgttttttag tttaatttta 1980
gaattgaagt ttattaattc gaagttaaag agcgaaatga ttttttagta gtttaaagtt 2040
tgtaagattt ttatatattg tttaattttt tttattgttg gttttttgtt ttttgttttt 2100
aagtaaattt gtgtaattag ttgtggtatt ttggtaatta taaggataag tttttatgat 2160
tttgtggaga ttttttaaaa atgtattagt ttaagggcgc gagtagggag ggatttatta 2220
tagaatagtg aaagatttat tttacgtttt taggcgttaa tatataattt tttttcgaga 2280

aattatttgg agagagaaga ttgttaggtg taattcgtcg gtaatttttt ttaattattt 2340
ttatatgttt ttattatttg ttacggtttt ttaaagatag ttataggaag gttacgaaga 2400
attttcgcgt tttttttaaa gtttttaaaa agttattttt tatttaatgg ggttgttagt 2460
tttatttaaa aagttttata gatataaatt atagaaggtt taatttcggt gttttgttgt 2520
tgtgagaaga ttcggtgtgt taggaaattt ttttttttat atttttcgta aaatttaatt 2580
tttgataaga gttgtaaatt ttggtcggtc gtattggttg ggtttgaacg ggtaatgttt 2640
ttttaaagcg gaaagggaat taaagatgcg taagtttttt tttgatttag ttgaggtttt 2700
tttttttagg aattagtttt gatttgtttt tttttaagga ggattttaaa ttggatttgt 2760
tttttttttt atttttatta taagttgttt tagagcgcgt gtatgaaagc gaaagttgga 2820
tagatttagg ttgataagtt tattagttgt gtggtgtgtg tttttttttt tttttttttt 2880
tggtttgttt ttagtttttg gtattagatg tatggagttt tttgttaggt tgtttttgta 2940
aagttgatgt tgttaatttt aatacgaagt aaattttttt agtatttgtg tgtgtttttt 3000
atagagataa atatgttgat tttagtaaaa aaaaaaaaaa aaaaagatat tattataatg 3060
ttagaaaata gagttttatt tagttatatt ttattatgtt atttttataa gatggaatta 3120
aatattagaa gttattagaa gtttgatgat ttatatttga gatttataaa gtatttttat 3180
tatttatttt tttttaaatt agttttattt ttttatttat agtgtggtat tttaattttt 3240
taaattaata agttattata aagaattatt gtagatgata gtatttttaa gtagaaaagt 3300
ttgtatgcgg tttgggggaa atttttagta atagataatt ttattttaat ggtgaagata 3360
atattttttt ttttttattt tgtattattg ttgtaaataa gtattttttt aataagtttt 3420
taaattgata gaagtaagac gtatggtgaa gatattagta ttaaaaggag acgtttgagg 3480
gattttatta tgatggatat gttttgaagt ttagtgtatg tattatgata tattttgtat 3540
taaaatttaa gttagaatag taggttttgt agttttattt tattttgaag ttttaaattg 3600
attatggatt attatgattt ttttagttga ttgttgtatg tggatttagt gtattaaata 3660
tatttgtaaa attttgtata tataaaattg atgagtaatt tattcgtaat atttgattat 3720
aggagcgatt ttttgtgttt aaagcgggga ttagattttt tttttttaaa aaaggattcg 3780
ttttgatttt tgtatattgt taggttaaaa atatattttt gaaaacggta atagtgtgaa 3840
tttttggttt gattttttta ttgagaaaat gaggagtatg agtagttatt aagtgtagtt 3900
tttattattt ttaggttgtt ttaatatatt tgatagtgtt ttttaatata aaaaggatat 3960
tgaaatttaa gtttttttgg agtttgtggt atttgtttat gtgtttttgt atatattttt 4020
taaaatgtgg ttgttatatt taattttttg atgggttttt agatttgtgt ttttgtgtat 4080
tgttgttttt tttgttttag ttattattta aatattttat attaaattta atgttagata 4140
tagatttttg tataaatatt atgtttttaa gttatatttt aaaaatttaa ataggtttta 4200
attgtaattg aaaggtaaaa ttatatattt agtatttatt tattttttaa taatttttgg 4260
tagatataaa tgaattttaa gttgtttttt tgggaaatta taaatagtaa gatagtttat 4320
ttttttttat ttaattaaga ttgggaaggg aagaggggtt ttgattagtt ttttaatgga 4380
ggtataaatt tgagaattta tttttaggga gatgatgtta tatgtttagt ttatattaga 4440
attaggtttg aaagagtaga ataagttata ttgttagttt tattgagtaa ggtatttaga 4500
tgaaaaaata tgtataattg aaggatttat attttttgat gtttatatta tataatttgg 4560
gggttaaaat gaaataggaa gattttattt atttttaaaa atggatattg agtagatgcg 4620
tatgtatttt ttttttcgtt tttatttttt ttgtgtagtt tttaatgatg tagaataagg 4680
ttatataaat ttttagtatt tttaattatg tttaagggtt agataaaatt atgtgcgttt 4740
aaagggagtt acgattaatt atttatattt tatcgcgaac ggggtgtggg tgtttagaat 4800
taatttgcgt tttttttcgt tggtggtggt ggtttattga ggaataatta ggagaatttg 4860
tgttttaatt ttagagaaaa ggaagagata ttagtgggta tgaaatgatt tttttttttt 4920
ttttttttat ttaaaaaaat attaagtaat gataataaag gtagttgtta ataataataa 4980
atttttagtt gtgattttta ttttgatgtt taggattttt gttgcgattg tggttttgtt 5040
gagtgttaat tgttttatta attagataaa atagtttggg tgaattagta agaatttttt 5100
ttgtacgtag agttttttag ttttattttt tgagtttagg tagttgtttt tgatattagt 5160
ttttttggta ttttttggta gacgataggt tagaaggtat cgtttttgtg cggtttgttg 5220
ttcggttttt agtcgagata gaattttatg ttttcggtat cggggtagtt gcgaaattat 5280
aaattttagt ttagttttga gtatgcgaag gttcgtagtt gattgtaggg aggcgttcgg 5340
ttaagcgagg acggaaattt gtattttttt tttaaaattg tgtttttgaa agtatttatt 5400
atgatttatt aaaagcggtt ggtagtgtta agtgaaattt tttttttgtt tttttttgtt 5460
ttttagaatt tttaagtgat taggaataga cgatttttaa tcgagttttg ttgcgtggag 5520
tttaggttgg cgttagtgag tgtagtgtgt tgatttaagt ggtggtggtt taaggacgcg 5580
ttgattcgta tatttggtaa agtttttatt aaatgtatta gattttcgcg gaagtggttg 5640
tagttcggag agtgaaatag ttatttttta agttttgatc ggtttagttg aaatttatat 5700
ttgtattgaa tttggtaaaa tacggacggg ttcgttcgag attttaggaa atttagagta 5760
agttattttt atgtagttga ttattttttt tgaaaggacg gatattattt attttattgt 5820
aattttgttt aacgggtagt tttgagttta gttttagcgt taaaggattg atttttttta 5880
ttcgttggag atttattggg gacgagcggg ggaggggagg cgaaggtatt tcggaggagg 5940
ttaagtgggg gaggaggagg agtagtagaa aattattagt taagtagggg agggagttgg 6000
aagttcggaa atatattagg atgttttaag taattttttt ttaagtttta gaatttagtt 6060
ttcggaaaat agttttataa ggtgttttgg gaaggagttt tcgggtggg 6109

<210> 167

<211> 5937
<212> DNA
<213> Artificial Sequence

<220>
<223> chemically treated genomic DNA (Homo sapiens)

<400> 167

ttggatttat ttaatttatg tttgaatgag taaatttgtg gtttggttgt ggttgttata 60
attatatttt gatttttagt gtttgttatt tataggttgg tggtgggttt ttttttatgt 120
agaatatttt gtgatgtggg agaatgatgt tgtgtttttt tagggagttg gttggatagg 180
tttatgggag gtagtaattt ggcgggatat gtgagtttta ttgggttttg ttggaaaggg 240
gtagaaagat aatatattcg tggaatagga tttgtgaatt tgtgttaata tttttgtaag 300
tttaaataag gaataaggaa gattttaatg gatattttat atgtgttttt tttataattt 360
gaatttaatt ggaatgaaga atgggaagaa agaattattt ttttttcgtt tatttttttt 420
tttttttttt tttttttttt ttgagatgga gtttcgtttt tgttgtttag gttggagtgt 480
aatggtgtta ttttatttcg gtttatcgta atttttgttt tttaggttta agtaattttt 540
ttgttttagt tttttgagta gttgggatta taggtatgta ttattacgtt tggttaattt 600
tgtattttta gtagagacgg ggttttttta tgttgaggtt gttttcgaat ttttgatttt 660
aggtaattcg ttcgttttgg ttttttaaag tgttgggatt agaggcgtta gttatcgttt 720
ttagttttat ttatttttaa aatttatagt attattattt ttttgaaata tattttttat 780
ttagtagtgt tgaagattag tattgtggtg ttgtttatta tatttttggt atttataaaa 840
tatttggtaa attaataatt atttttttta agatttaaga aggaaaattt ttaaatatag 900
aaaaatgtcg aagaattttg tagtgaatat tattgtaata tttaaatttt attattaaga 960
ttttattggt tgggcgttgt ggtttatgtt tgtaatttta gtattttggg tggttaaggt 1020
aggaggatta tttgaggtta ggagttttaa agtagttatg gttaatatgg tgaaatttta 1080
tttttattaa aaaatataaa aattagtcgt gtatgatggt atgtatttaa ttttagttgt 1140
tcgggaggtt gaaataggag aattatttga atttgggagg cggagattgt agtgagttaa 1200
gattgtgtta ttgcgtttta gtttggataa taggttttgt tttaaaaaaa taaaaaaaga 1260
ttttattatt ttgttttagc gtgtaattat ttatttagtt attttttttg atttttttaa 1320
ttttgtatgt attttagtgt tagttgtaga tattatttta ttttttcgtt aaatgtttta 1380
gtatttatat aattggaggt gaatatttgt ttataatttt tttttttttt tttgaatatt 1440
taatgaaata taattttaaa tgtattattt aataaatttt gataaatgta tatatttgtg 1500
tagtttaaat atttattaaa aatatgaaat attattttat aatgttatta gaagagtttt 1560
aagtttattt tagtgaagtt ttgtttttat ttatttattt ttaaaattat ttttgtgatt 1620
tttttttatt agtttttttt gttttggaat ttcgtataaa taaatttata taggatgtag 1680
ttttttgtgt ttggtttatt ttatttaata taatattttt tagattatcg atgttattgt 1740
ttataatagt agtttttaat aaatattgat tagtttattt taattatagt gttgattaat 1800
taaggattat agttgattag atgtatttaa atatagttaa tatagtttga atgtttttta 1860
taaagttttt gtaggtttag aattgttata tgttgttgat tagataaaag tggggtaagt 1920
tttttgggaa aagaaaaaat gaggtattta ttattttttt agaattaaag gaaaagatat 1980
ttaaggatga tgtttgagta tacgagatag gttagatagt aattgattat aagttttttt 2040
ttttttttta ggtgtaggta tgatagttaa agatggtatt ttttatagga aggttgtttt 2100
ggaatattta tttatatttg gaggttatat tttaaagtag gataagtttt gttaaggggg 2160
aatttttgta ttttagtttt ttcggtagta gttttttatt ttttaaggag ggggtttgat 2220
aatcggtttt aaggaagtag aggatagagt ttttatttag tgtttgttgt ggtaaacgtg 2280
attggttttt tgggggagtt ttaattattt ggaagagttt taattgaaaa tttttaaaag 2340
aatttttttt agtttataat tgtttttttt aaaagtgggt ttttaggttt aggaagggtg 2400
tgggattttg tgttatgtga tataaaaatt ttgtgtttat tagtgatttg atatttttgt 2460
taggtgtaat taggtagttt ttatttttta gagtttatat tttttttttt attagttaaa 2520
tgggttaaat ttattggtgg attggaggtg atattagttt gtatgtggtt tagattgtaa 2580
ttggtggtat ttgtaatttt aggtagtttt tttaggtgaa gttaggtgga gatttaaata 2640
ttttaattaa aggtttggtt tcgtagtttt ttttttgaga gttgtggggg gtataaggta 2700
tagaattaaa atagggttcg atatagggaa ttttaaggag tgtgtttttt aagattatat 2760
tagttttttt tagtattatt ttgttttttt tttttttttt tttttttttg agatggagtt 2820
tcgttttgtc gttaggttgg agtgtagtgg tatgatttta gtttattgta attttcgttt 2880
tttaggttga agttattttt tttttttagt ttttcgagta gttgggatta taggtatgtg 2940
ttattacgtt tagttaattt ttgtatttgt agtagagatc gggttttatt atgttgatta 3000
ggatggtttt aattttttga tttcgtgatt tattcgtttc ggttttttag agtgttggga 3060
ttgtaggcgt gatttatagt tcgcggttta ttttattttt aaatcgttat ttagtattta 3120
aatttaggga tatttgggag tagaaattag aacgggatag ataagtgggg agattggtgt 3180
tgatggaaat tttagtgaga aaagtgtaga tgggattata gttgataatt ggttgttttg 3240
atttaggata attggggaaa gggaggttgg ggtatattga aatatttagg ttgtcgttgt 3300
ttattttagt tgttaatatg attagttgta aatttgtttt gtttttagaa attttttttt 3360
taaaatatgg attttttcgt tagtgtttaa gatgggatat attattatta agtaagtatt 3420

ttttttagga gtatttttta ggttgttaaa atgttaagat ttttaattat ttttggtagt 3480
tttgaattta tgtaaagagg ttagttagtt gttttagttt attttttaga atgatgagat 3540
ttggtattga aaatattgtt tatattgagt tttttattaa atgtaataag tagataatta 3600
taaatattta tttttttatg tttataattt agtatgttgg aaattatttt tttttgattt 3660
attgttagtt tattacgttt tatatttagg agaatttaag ttttaaatcg gtcgttgaat 3720
agttacgttt ttttgtagga tatatttatt attagggtta aaattttgtt tattttttaa 3780
aggaagtcga tgtttttata taaaggtttt tttaaatgta atagtaagtt cgtgggagag 3840
gggttagtag aggttacggt tagtttacgg gtttttttcg attttgatta tgattaggag 3900
tgtagttgtt aattgcgagg tttgttttaa ggtggaaaat agtatcggtt tttattgtta 3960
ttttagagaa ggtaagtttc gcggttggcg gtgttgggga tacggttttt tttaggttta 4020
ttttttgtta tttagagagt tgttcggagg tcgtttatag gtgtaatttc ggtattgcgg 4080
tcggggcgtc gggtcgggga gggcgtttaa gtttattagt attttcgtcg gtttttttta 4140
aagtttgaat agggtttcgg cgtgttcgtc gttttttatt ttcggttttt tcgcgttttt 4200
gtttcggcgc ggtttggata ggaagttggg agttttttta ggtttcgtag attcggattt 4260
gggagggggt gggacgcggt cgaggttttt tttcgaattt gcggtaagtt tggttttagg 4320
aaagtttttt aaagttttta gtagcgtttg tttaggtcgt tttcgcgggg cgagtagacg 4380
gcggtaagcg cgttagtttc gtcgtcgttt ttgtcgttag tagagcgttt tgggcggttc 4440
gttcgcggga agcgggtcga attttcggcg ggtaggtagg tttttttttc ggggcgaaag 4500
tcgtagttga cgtaggcggt tcggaaggcg gaagttgttt cgtttcgatc gtttagttag 4560
cgtcgcggcg tttatatttg gggtttcgac gcgcgggtaa aggcgtacgg ttcggggcgt 4620
tcgagggggc ggttttcgtt gggggttttt aggcgttttt gagtaacgat tttttttaag 4680
tattttttcg tatttttttt ttttttttgg ttcgaagttt tcgagggcgg gggttggtgt 4740
ggggttttgg tttttttacg tcgttttgag tatttcgttg tttttaattt tttttaagtt 4800
ttttttcgta ttattttttt tttagtaacg tgaaggggag gggcgtgttt agggtgagta 4860
cgttttttta tgaatattaa taagcgcgta tgcgttttgt tcggcgtgtt gggtagaggt 4920
ggttagtttc ggtcgttgtt gttagacggg tttttcgggt tttttttcga gagtcgggcg 4980
ggtacgcgtt attgtgttat ttgcggtcgg ttcgcgagtt aggttggttt tttttttttt 5040
tttttttttt tttttttttt ttatgtagtt gatttaaaag ggaataaaag gttgcgtata 5100
attataataa taaaagaagg ggagcgcgag agaaggaaag aaagtcggga ggtggaagag 5160
gagggggagc gttttaaaga agcgattaga ataataaaag gaggtcgggt tttttgtttt 5220
ttggaacggg tcgtttttga aagggttttt gaaaagtggt gttgtttttt agtcgtgtat 5280
gttttaatcg gcggagtata ttagagtcgg gacgcggcgg tcgtaggggt agcggcgacg 5340
gtagtatcgg cggtagtatt agcgcgaata gtagcggcgg cgtttcgagt gttcgcggcg 5400
cgcggcgtag cgatgcgttt tttacggacg cgcggtcggt tcgggcgttt tttaagtttt 5460
ttgttcgttt tgttttgtgt tttgcgagtt aaggtaggag ttttttttcg ggtttttttt 5520
ttagtcgttt tttttttttt ttcgcggcgt tttgggcggt tgagtttcgc gagtaggttc 5580
ggtagttttc gagcgttcga tttttcgttt ttgcgttcga gcggttgtat ttgggttgag 5640
cgcgttaatt tttttacggg agtttttgtt ttttgagggt gattttttta ggtcggatag 5700
gggcgcgttc gtttcgttgt tgtttttcgg gaagtcgtcg gatttttttc gtattcgtgt 5760
attttttttt ttttttgttt cggaattatg ttgagttttt gaacgtagtt tcgtaaaatt 5820
atttttattc ggcgaggagg gttcgtcggg gcggggagtg ggagggagtc gttattttta 5880
tattcgaaga aaatagtttt ttttcgcgtt gatttatttt tttttttcgt cggtagg 5937

<210> 168
<211> 5937
<212> DNA
<213> Artificial Sequence

<220>
<223> chemically treated genomic DNA (Homo sapiens)

<400> 168

tttgtcggcg agggaaggag gtaggttagc gcgggagaaa gttgtttttt tcgagtatag 60
aggtggcgat ttttttttat ttttcgtttc gacgagtttt tttcgtcgag tgaaaataat 120
tttgcgaaat tacgtttaag gatttaatat gatttcgggg taaaaaaaaa aaaaaatgta 180
cgagtgcgga agaaattcga cgatttttcg gggggtaata gcggagcgaa cgcgtttttg 240
ttcggtttgg aggggttatt tttaggaggt aggggttttc gtgggggagt tggcgcgttt 300
agtttaggtg tagtcgttcg ggcgtagggg cgaggagtcg ggcgttcgag ggttgtcgag 360
tttgttcgcg gggtttaatc gtttagggcg tcgcgagggg agggagagga cggttgggag 420
ggaggttcgg agaagggttt ttattttggt tcgtagggta tagagtaggg cgagtaggag 480
gtttaggggg cgttcggatc ggtcgcgcgt tcgtggggaa cgtatcgttg cgtcgcgcgt 540
cgcgggtatt cgggacgtcg tcgttgttgt tcgcgttggt gttgtcgtcg gtgttgtcgt 600
cgtcgttgtt tttgcggtcg tcgcgtttcg gttttaatat atttcgtcga ttggagtatg 660
tacgattgga aaataatatt attttttaaa agttttttta agagcggttc gttttagaag 720
gtaaagagtt cggttttttt ttattatttt gatcgttttt ttgagacgtt tttttttttt 780

ttttattttt cggttttttt tttttttttc gcgttttttt ttttttatta ttatgattat 840
gcgtagtttt ttattttttt ttagattagt tgtatggaaa aaggggggag ggaggggaga 900
aaaaaaaaaa ttagtttagt tcgcgggtcg gtcgtaggta atataatgac gcgtgttcgt 960
tcggttttcg gagaaggatt cggagagttc gtttggtagt agcggtcggg gttggttatt 1020
tttatttagt acgtcgggta gggcgtatgc gcgtttatta atatttatga gagggcgtgt 1080
ttattttggg tacgtttttt ttttttacgt tgttggggag ggggtagtgc gaggaggaat 1140
ttggaagggg ttgggggtag cgggatgttt agggcggcgt agaagaatta gggttttata 1200
ttaattttcg ttttcgggag tttcgggtta ggagggaagg gagagtgcgg ggaggtattt 1260
ggaagggatc gttgtttagg gacgtttgga ggtttttagc ggggatcgtt ttttcgggcg 1320
tttcgggtcg tgcgtttttg ttcgcgcgtc ggggttttag gtgtaggcgt cgcggcgttg 1380
attgagcggt cggagcgggg tagttttcgt ttttcgaatc gtttgcgtta gttgcggttt 1440
tcgtttcggg aggagggttt gtttgttcgt cgggagttcg gttcgttttt cgcgagcgag 1500
tcgtttagag cgttttgttg gcggtagagg cggcggcgag gttggcgcgt ttgtcgtcgt 1560
ttgttcgttt cgcggaggcg atttgggtag acgttgttgg gaattttgaa aaattttttt 1620
ggagttaggt ttgtcgtaga ttcgagggga agtttcggtc gcgttttatt tttttttaaa 1680
ttcgagtttg cggagtttgg gagggttttt agttttttat ttaaatcgcg tcggggtaga 1740
ggcgcgggga aatcgggggt agaaggcggc gggtacgtcg gggttttgtt taggttttgg 1800
gaagggtcgg cggagatgtt ggtgggtttg gacgtttttt tcggttcgac gtttcggtcg 1860
tagtgtcggg attgtatttg taggcggttt tcgagtagtt ttttgggtgg taagagatgg 1920
gtttgggagg gatcgtgttt ttagtatcgt tagtcgcgga atttgttttt tttggggtgg 1980
tagtggaaat cgatattgtt ttttattttg aggtaagttt cgtaattaat agttatattt 2040
ttggttataa ttaaggtcga agaaagttcg tgggttggtc gtagtttttg ttaatttttt 2100
ttttacgggt ttgttgttgt atttgagggg atttttatat ggaaatatcg attttttttg 2160
aagggtggat aaagttttgg ttttaatagt aggtatgttt tgtagaaaag cgtgattatt 2220
tagcggtcgg tttggggttt gagttttttt gggtgtgggg cgtgatgggt tggtaatagg 2280
ttaagggaaa atagttttta atatgttgag ttatgagtat aaaagggtgg gtatttgtgg 2340
ttatttattt attgtatttg atgggaggtt tagtatgaat aatattttta gtgttaaatt 2400
ttattatttt gaggaataaa ttagggtagt taattgattt ttttatatag gtttaaagtt 2460
attaaggatg gttaggagtt ttggtatttt aatagtttaa gaaatgtttt taagaaaagt 2520
gtttatttag tggtaatgtg ttttattttg agtattggcg ggagaattta tgttttggaa 2580
gggaggtttt taagaataga gtaagtttat agttggttat gttagtagtt ggggtaggta 2640
gcgataattt gggtgtttta atgtgtttta gttttttttt ttttaattat tttaaattaa 2700
ggtaattagt tgttaattgt aattttattt atattttttt tattgaggtt tttattaata 2760
ttagtttttt tatttatttg tttcgtttta atttttattt ttaagtattt ttgagtttgg 2820
gtgttaaata acggtttaaa agtaaaatgg gtcgcgagtt gtgggttacg tttgtaattt 2880
tagtattttg ggaggtcgag gcgggtggat tacgaggtta agagattgag attattttgg 2940
ttaatatggt gaaattcggt ttttattata aatataaaaa ttagttgggc gtggtggtat 3000
atatttgtag ttttagttat tcgggaggtt gagagaggag aatagtttta atttgggagg 3060
cggaggttgt agtgagttga gattatgtta ttgtatttta gtttggcgat agagcgagat 3120
tttattttaa aaaaaaaaaa aaaaaaaaaa aaggtaaaat ggtgttgaaa aaggttggtg 3180
tgattttaaa gagtatattt tttggagttt tttatgtcgg attttgtttt aattttgtgt 3240
tttgtgtttt ttatagtttt taggaaagag gttgcggagt tagattttta gttaaagtgt 3300
ttagattttt atttggtttt atttagaagg gttatttgag attgtaggtg ttattagttg 3360
taatttgggt tatatgtaag ttggtgttat ttttagttta ttagtgaatt tagtttattt 3420
gattgatagg aaagaaaata taaattttga gaggtgaaaa ttatttgatt atatttaata 3480
agaatgttaa attattgata aatataaaat ttttatgtta tatagtatag agttttatat 3540
tttttttgag tttgggaatt tatttttaga gaaggtagtt gtgggttaga gggaattttt 3600
ttgaaaattt ttagttgggg tttttttaaa taattgggat ttttttaaaa agttagttac 3660
gtttattata atagatattg agtggaagtt ttattttttg tttttttgaa gtcggttgtt 3720
aggttttttt tttagagaat gaggaattgt tgtcgagaga attgaaatat agagattttt 3780
ttttggtagg gtttgttttg ttttggggta tagtttttaa gtgtaaatga atattttaga 3840
ataatttttt tgtggaaaat gttattttta gttgttatat ttatatttga gagaagggga 3900
gaggtttgta gttagttatt atttgatttg tttcgtgtgt ttaaatatta tttttggata 3960
tttttttttt taattttaaa aagatgatgg atgtttattt tttttttttt ttagaaaatt 4020
tgttttattt ttatttagtt aataatatat agtagttttg aatttgtaaa agttttataa 4080
gagatattta agttatatta gttgtattta agtatattta attaattgta atttttggtt 4140
aattaatatt gtggttaaaa taagttaatt aatatttatt gagaattgtt attataagta 4200
gtaatatcga tgatttaaaa aatattatgt taagtgaaat aagttagata taaaagatta 4260
tattttgtat gaatttattt atacggagtt ttagaatagg aaaaattggt gaaaaaagat 4320
tataagagtg attttgaggg tgggtgggtg ggagtagagt tttattggga tgggtttgag 4380
gtttttttgg tggtattgtg gggtaatgtt ttatattttt gataggtgtt tgggttatat 4440
aggtgtatgt atttgttaaa atttattaaa tagtatattt aagattgtat tttattaggt 4500
gtttaaaaga ggaaaaaaga attgtgaata gatatttatt tttagttata tgagtgttga 4560
agtatttagc ggggaagtgg aatgatattt gtaattgata ttgaaatgta tataaaatta 4620
gagggattag agaggatggt tgggtgggtg gttatacgtt gaagtaaaat agtaaagttt 4680
ttttttgttt ttttgagata gagtttgttg tttaggttgg agcgtagtgg tatagttttg 4740

gtttattgta atttcgtttt tttaggttta ggtgattttt ttgttttagt ttttcgagta 4800
gttgggatta gatgtatgtt attatatacg gttaattttt gtatttttta gtagagatgg 4860
ggtttattta tgttgattat ggttgtttta aaatttttga ttttagatga tttttttgtt 4920
ttggttattt aaagtgttgg gattataggt ataagttata gcgtttagtt agtaaagttt 4980
taatggtaga atttaggtgt tatagtggtg tttattgtaa aatttttcga tattttttta 5040
tgtttgaaaa tttttttttt taaattttgg ggaagatagt tattgatttg ttaggtattt 5100
tgtgggtgtt aagaatatgg tgggtaatat tataatattg gtttttagta ttattgagtg 5160
gagagtgtgt tttaaaggaa tggtgatatt gtagatttta agagtgaatg aggttgggag 5220
cggtggttga cgtttttaat tttagtattt tgggaggtta aggcgggcgg attatttgag 5280
gttaggagtt cgagaatagt tttaatatgg agaaatttcg tttttattaa aaatataaaa 5340
ttagttaggc gtggtggtgt atgtttgtaa ttttagttat ttaggaggtt gaggtaggag 5400
aattgtttga atttgggagg tagaggttgc ggtgagtcga gatgagatgg tattattgta 5460
ttttagtttg ggtaataaga gcgaaatttt attttaaaaa aaaaaaaaaa aaaaaaaaaa 5520
aagagtgaac gaaaggaaag tagttttttt tttttatttt ttattttagt taggtttaaa 5580
ttgtagagaa ggtatatgta aaatatttat taagattttt tttatttttt gtttgaattt 5640
gtagagatgt taatataagt ttataaattt tgttttacgg atatgttatt tttttgtttt 5700
tttttagtag agtttagtga agtttatatg tttcgttagg ttattgtttt ttatgaattt 5760
gtttagttag ttttttaagg aagtataata ttattttttt atattataag atattttata 5820
tggaaaaaaa tttattatta atttatagat aatagatatt gaaaattagg gtatagttgt 5880
agtaattata attaggttat aagtttgttt atttaagtat aagttgagta gatttag 5937

<210> 169
<211> 5367
<212> DNA
<213> Artificial Sequence

<220>
<223> chemically treated genomic DNA (Homo sapiens)

<400> 169

taacgtgatt agggaaggga tgttttgttg cgggaggggt tacgggcgtg tatattaggt 60
gggaattcgg gtgggagagg gtattttaga gttttgttta ttggagggtt ggagggagta 120
gagtggagag ggtgggttgg agggtggtag aagggttagg gggttagcgg ggttagcgtg 180
ggtgatttag tagattttgt tttaattcgt aggtttggag tgggattgat tggttttaga 240
tagattatgt tttatataga ggtgatagat gtttagcgta gcggtttagg tgggggtttg 300
gtggaggagg gtgagtttat tgaggtggtg tatttgtttt tggaaggcgt ttaggttttt 360
gtagacgatt cggatatttt taagattttc ggcgttattt ttggtgtttt atggtttttt 420
agttaggtgg tttttaattt ggatttttag tgagatttta gggttttaga tagaggttag 480
ttttggttat ttattttata tttcgcggat tttaataaag gtttgtatag ttttagtttt 540
tgtttgttgg ttttgagttt tgtgattagg gttttggaga ggattgggga aggggcggga 600
agtagtgatt ttgatttgag atggttttcg ggatacggcg agtttttttg taggaggagt 660
agttggtggt aggttatgta ggtggttgtt gtggggttga ggataggttt ggggatgtta 720
tgggtagggt taggttgtgt ttttttttgg agttgggagg ggtggggttc gggatttatg 780
gagggtggta tggtaggttt gggggtgggt ttgttggtta cggggttgta gtatgagttg 840
tgtttacgag agtggtggtt gagttagttt ttaggagtta ggatggggtg ggtagtattg 900
taggttgtac gttgtaggga aagtatgttt tttgtttttg gggtaagtga ggggttttta 960
ttttgattgg ggttgggtta gagttatttt ataggagttc ggagttaaag ttgttttgtg 1020
tcgggggttt tagtagttga ttcgtgtatt tttagttttg gaagtttttt aagggatttt 1080
tttcggtttg tggggtatgt agaaatgttt gtaggtttgt tttaagagga gtaggtggcg 1140
gtatagggtg gggatataag gttagtggtt ttgatatggg ttaggttttt ggtgttagtt 1200
cggttttggt ttcggggtac ggttttagtt tgggtcgttt agtattaggt tttattggtt 1260
tttgagtatt tttggtttgt atgttttggt tttgtttttt gatttgtttt gagggtgggg 1320
ttgtgggaga tgcgtgtagt ttagagttgt tttttaggtg tttagaatgt ttgtgaggta 1380
ggaggggatt tagttcgttt ggtttgtttt ttgggttata ggtgtatagt aagggttttg 1440
taggtgaatg ggtgtgttgg tattggttgt gggtattcga gggtattgtt gggttattag 1500
tttgcggtag gatcgattcg tttttggata ttattaggtt taggttgtgg tttttaggag 1560
gaaagtagag aaggggatag tgaagtgaag ggagttttag tattttgtag ggtggaattt 1620
tgggtaggga ggtggagatt gattttttgt tgttatttag tatagtgttg gatagtttgg 1680
tattggaggt tgttttgagg aggttgtgtt ttaggggttt ataaaagtag tgttatttgt 1740
atgtaaatgt tggttgtggt ttaggtgggg ttttgtgggt attagtaagg ttagcgatag 1800
ggttgtcggg gaggggttgt taggtatggg gtgtgtgtta gggaggggtt gttaggtatg 1860
gggtgtgcgg gttttggtta gttttagttt tattttattt agatgaggtt ttagtaggat 1920
atttatagga taggagtttt gtttgattta tagtagtttt gttgtgttgt ttttattagt 1980
agtttgtttg gtgatattac gagagtgcga tgggtttttc gggttgatta gtagaggttt 2040
tttattattt tagagggatt tttcgtaggt attaggtggg tttggttttt gaagtattga 2100

ggggataggt gatagggtat tcggatttgg gtgtagttta gtttttattt ttgtttcgta 2160
gttcgatggg gtgggttagt aggtagtgtt gtcgggttta atggagttgg tattgatttt 2220
tcggtattgg agggggggtt gtaatcggaa attttttttt tcgtttgtta tatttagtat 2280
tgtaggattt agcggaagta tagtttattt tggagtttcg tcggttttgt ttaaattttt 2340
ggtagttcgt ttttagtttg gtttttagga ttgttttatg ggtttagagt ttttggttag 2400
gttgagtagt tgtatttttg ttttttgtat ataagttggg tatcgtttag cgtttagagt 2460
gggagttttt taggttgggg gacgggtggt tttgggaggt tggagggtgt tgtggtttag 2520
tgggttttag aataatgacg aggtttttta ttagaagaga ttaggtagtt tggtttggtg 2580
agggttgggg ttggagtttt ggtcgttatt gattttagtt ttttatttta gtaaggtgta 2640
gtagggggta aatgtgtttt gtgaaatatg tatgaggggc gagggttttt attgttgttt 2700
tttggaaatt taaattaggg tttagttgtt tggtattttt ggtttaaggg ttgttgaaat 2760
ttttgtaggg aagggatttt tttttcgatg ttttttatgt tttttttttt tttaagggta 2820
ggtgcggtta tttttttgat ttgggtgttt ttatggatat cgtgaggttt ggttgattta 2880
tgttcgttta cgttttggcg gttttttatg ttagtttttg tatgatttgt ttaagttttt 2940
tgtttatttt cgggttgggg gtttttgagg gtgtgtaggg gcggagtttt ttttgtttat 3000
tatttggtta agtgtttagt agaggtattt agtatttttt tttttattat agtggggtag 3060
tgggcgtagg tggggatgga gttttgggtt tttggagtcg ggtagggggt ttttttgttt 3120
tggggttttt ttttttcgag tattcgagta tttgttttta ggttaaagta taggagaggt 3180
ggtattttta tagggagttt tttttttcgt aggcggtaat tatcgtttat atatcggaag 3240
gcggtaattg gaaggttttg aatatagttt ggattttttt gtttggtggg ggtttttgtg 3300
cgggttttat tttggtttgt gttttaattt ttacgaagtt tatttattat taagtttggg 3360
gtgggtattt tgagtagagt tagttgaggt gggtatgggg ggtttttatt tattttttta 3420
gggattttta gttgagtttg cggtttggat tttggaggag ttgttaaata gggatggagt 3480
aggtagattt tgcgtatggg gtgtttatag gttttgtgtt tttaaagttt ttttttttta 3540
gtttttagtt ttagtagtat ggtggtagtg atttgtttta ggttgggggg ttgtatagtt 3600
atttagtttc gggtataggg taaatttgcg agaggagtta gggggagaga agatagttta 3660
gttattatgg ggatgggttg aggatttttt tggattatat ggggattttt aattattaag 3720
gtgattttta gttttggaaa ggatcgtgtt tattggagga gaggaaggtg ttattggttt 3780
tgattttgtg gaggagttgc gaggttattt agggagaggg taaggaggtg atcgtagagg 3840
atggggtgtg gaagtttggt gattagggta gtagtgggag gtttttttcg gggtagtttt 3900
tagggatagg tattgtcgat ttttgttttt tatttttcgt ttttcgtttt ttaagtttag 3960
atttgagttt ggggggcgag aggcgggaaa cggggaatgt ggtttgagta ttttttgagg 4020
gtatggtttg gttatttcga cgttagcgtc gagttgagta gtttgtattt tggagtattt 4080
gttgattggt tgtttgatta gcgcgtttcg tagaggggaa ggtaggggcg tcggaggggc 4140
gtagcgtttt ttgtagtcgg cgtggaggcg gtaggagcgg cgcggagaag gggagatttt 4200
cggaggaggt ggggggcgcg tagtaggggt tgggttcggt tttggtttta gggtcgcgtt 4260
atttcgcgtg ggggtcgagt tttgattaga gtaggaggcg gtattttttt tgggattgcg 4320
aggagcgcgg cggtggcgta ttgatgggag gggattatac ggtaatttcg gggcgtttta 4380
ttttcggttt ttgatattcg gtaggagttt aggcggagga ggggaggtag ttttgcggcg 4440
tcggcgtacg tttcgtcgat ttacgcggag gtgtgagcgg ggttttcgcg gttcgcgttg 4500
atttcgaggt ttcgtgtttt cgtcgttcgg gcgttttggg gggcgcgcgt cgggtcgggg 4560
cgttggtagg cgacgttttt tatcgttttt aaagtttggg gcgttttcgg attttttttc 4620
ggttttattt cgcggcgcgg tttcgttttt ttatgtatat gtaggtgcgc gggtgacgaa 4680
tgggcgagcg agttgttagt ttcgtttcga atttgttggt tgcggtgtcg ggagcgcggg 4740
cgcgtagagt cgaggtcggg attcgttgtt tttatcgtcg tcgtcgtcgt cgtcgggtgg 4800
gagtcgggtc gggtagtcgg agcgcggtcg ttagcgagtc ggagttgtcg tcgtttttgt 4860
acgttcgtcg tttaggttcg cgcgtcgcgg cgttgcgttc gatttcgttc gcgtcgtcgt 4920
cgtcgtcgtt tttgtcgttg tcgttgtttt tgcgggcgtt cggagggcgg gcgggcgttg 4980
ggaggtcggc gcggcggttg ggagtcgggc gcgggcggcg gcggcggggt cgggcgggcg 5040
ggtcgcgggg gtaatgcggg cgtagggtcg ggggcgtttt tttcggcggt tgttgttgtt 5100
gttggcgttt tgggtgtagg tgagcggggc ggcgggggcg gcgggggtcg cggacggggt 5160
atatcgggtc gtttttaggg gtcgggcggg tattgtttgg ggtcgtcgtg gttcggaagt 5220
tttcgaggtt gcgcgcggcg gttggggttt cgggcgggcg cggttgggtg ggggcggggc 5280
ggcggggttt gtttttttat ttttggcgtt cggttcgtcg atttcggttc gcgttttttt 5340
tcgtttttc gttgttttat ttttagg 5367

<210> 170
<211> 5367
<212> DNA
<213> Artificial Sequence

<220>
<223> chemically treated genomic DNA (Homo sapiens)

<400> 170

tttaaaaata aggtagcggg agagcggagg gaggcgcggg tcggggtcgg cgggtcgggc 60
gttaggggtg ggggaatagg tttcgtcgtt tcgtttttat ttagtcgcgt tcgttcggag 120
ttttagtcgt cgcgcgtagt ttcgagggtt ttcgaattac ggcggtttta ggtagtgttc 180
gttcggtttt taggggcggt tcggtgtgtt tcgttcgcga ttttcgtcgt tttcgtcgtt 240
tcgtttattt gtatttagag cgttagtagt agtagtagtc gtcggggaag gcgttttcgg 300
ttttgcgttc gtattgtttt cgcgattcgt tcgttcggtt tcgtcgtcgt cgttcgcgtt 360
cggtttttag tcgtcgcgtc ggttttttag cgttcgttcg tttttcgagc gttcgtagag 420
gtagcggtag cggtagaggc ggcggcggcg gcggcgcggg cggggtcgag cgtagcgtcg 480
cggcgcgcgg gtttgggcgg cgggcgtgta ggggcggcgg tagtttcggt tcgttggcgg 540
tcgcgtttcg gttgttcggt tcggttttta ttcggcggcg acggcggcgg cggtgaaggt 600
agcgggtttc ggtttcggtt ttgcgcgttc gcgttttcgg tatcgtagtt aataagttcg 660
gaacgagatt gatagttcgt tcgtttattc gttattcgcg tatttgtata tgtatgaggg 720
ggcggggtcg cgtcgcgggg tggggtcggg ggggggttcg ggggcgtttt aggttttaaa 780
ggcggtggag ggcgtcgttt gttagcgttt cggttcggcg cgcgtttttt agggcgttcg 840
ggcggcgggg gtacggggtt tcggggttag cgcgggtcgc gggggtttcg tttatatttt 900
cgcgtgagtc ggcgaggcgt gcgtcggcgt cgtaaagttg tttttttttt ttcgtttggg 960
tttttgtcgg gtgttagaaa tcgggggtgg ggcgtttcga ggttgtcgtg tggttttttt 1020
ttattagtgc gttatcgtcg cgtttttcgt agttttagag gagatgtcgt tttttatttt 1080
gattagggtt cggtttttac gcggggtggc gcggttttgg ggttagagtc gggtttagtt 1140
tttattgcgc gttttttatt tttttcgaga attttttttt tttcgcgtcg tttttatcgt 1200
ttttacgtcg gttgtagggg gcgttgcgtt ttttcgacgt ttttgttttt ttttttgcga 1260
ggcgcgttgg ttaagtagtt agttaataaa tgttttaggg tgtagattgt ttagttcggc 1320
gttggcgtcg aggtagttag gttatgtttt taggaaatgt ttaggttata tttttcgttt 1380
ttcgtttttc gttttttaaa tttaggtttg ggtttggggg gcgagaggcg ggaaatgggg 1440
aataaaagtc ggtagtgttt gtttttgaag gttatttcga gagaggtttt ttattgttgt 1500
tttggttatt aggtttttat attttatttt ttgcggttat ttttttgttt tttttttggg 1560
tgatttcgta gtttttttat agggttaaaa ttaatggtat tttttttttt tttagtgagt 1620
acggtttttt ttagagttag gggttatttt ggtggttaaa gatttttata tggtttagag 1680
gggtttttaa tttattttta tgataattga gttgtttttt ttttttttag tttttttcgt 1740
aaatttgttt tgtgttcgga gttgggtggt tgtgtagttt tttagtttgg agtaagttat 1800
tgttattatg ttgttggggt tgagggttga aggaagagag ttttggaggt atagggtttg 1860
tgaatatttt atgcgtaggg tttgtttgtt ttatttttgt ttagtagttt ttttaggatt 1920
taggtcgtag gtttagttga aggtttttaa aggggtgggt gggagttttt tatgtttatt 1980
ttagttggtt ttgtttagag tgtttatttt aaatttggtg gtgggtaggt ttcgtgggga 2040
ttaagatata agttaggatg aggttcgtat agagattttt attaaataag agaatttaag 2100
ttgtgtttag agttttttag ttgtcgtttt tcggtgtgtg ggcggtgatt atcgtttacg 2160
gggggagggg ttttttgtgg agatgttatt ttttttgtgt tttggtttgg gggtaggtat 2220
tcgggtattc gagagaggga agttttagga tagagaagtt ttttgttcgg ttttaaaggt 2280
ttagggtttt atttttattt gcgtttattg ttttattatg gtgggggagg gggtgttggg 2340
tgtttttgtt gggtatttgg ttaagtggtg aataagggag atttcgtttt tgtatatttt 2400
taggagtttt tagttcgggg gtggatagag gatttagata agttatatag aagttggtat 2460
agggggtcgt tagagcgtgg acgggtatgg gttagttaga ttttacgatg tttatgagag 2520
tatttaggtt aggaaggtgg tcgtatttgt ttttaggaag gagggggata taggaggtat 2580
cggagagggg gttttttttt tgtaggaatt ttagtagttt ttgagttaga aatgttaggt 2640
agttggattt tggtttgagt ttttagaagg tagtagtggg aattttcgtt ttttatatat 2700
attttataaa atatatttgt tttttgttgt attttgttgg ggtgaggggt tgagattagt 2760
ggcggttagg gttttagttt taatttttat tagattaggt tatttggttt tttttagtaa 2820
gggatttcgt tattgttttg gggtttattg gattatagta ttttttagtt ttttagagtt 2880
attcgttttt tagtttggag ggtttttatt ttgagcgttg ggcggtgttt agtttgtatg 2940
tagagggtaa gggtgtagtt gtttagtttg gttaggggtt ttgggtttat agggtagttt 3000
tggggattag gttgaggacg ggttgttagg ggtttggata gagtcggcga ggttttagag 3060
taagttgtgt tttcgttggg ttttgtagtg ttaaatatag tagacgggga ggggagtttt 3120
cggttataat ttttttttta gtgtcgggaa attaatgtta gttttattag gttcggtagt 3180
attgtttgtt gatttatttt atcgggttgc ggagtaaagt gaggggttgg attatattta 3240
ggttcgggtg ttttgttatt tgttttttta gtgttttagg ggttagattt atttggtgtt 3300
tgcgagaggt ttttttgagg tagtgggagg tttttgttgg ttaattcgga gggtttatcg 3360
tattttcgtg gtgttattag gtaggttgtt ggtaagggta gtatagtaag gttgttgtgg 3420
gttaggtaga gtttttgttt tgtgaatgtt ttgttggggt tttatttggg tagagtgaag 3480
ttgaggttgg ttaagattcg tatattttat atttggtagt ttttttttgg tatatatttt 3540
atatttgata gttttttttc ggtagttttg tcgttggttt tgttgatgtt tatagggttt 3600
tatttgggtt atagttagta tttgtatata ggtggtattg tttttgtggg tttttgaggt 3660
atagtttttt taggatagtt tttaatgtta ggttgtttag tattgtgttg gatgataata 3720
gggggttagt ttttattttt ttgtttaggg ttttattttg tagggtattg aggttttttt 3780
tattttattg tttttttttt tatttttttt ttgggagtta tagtttggat ttaatgatgt 3840
ttaggagcgg atcgattttg tcgtagattg atggtttagt agtgttttcg aatgtttata 3900
attagtgtta atatatttat ttatttgtag aatttttatt gtgtatttgt ggtttaggaa 3960

gtagattagg cgagttgggt ttttttttgt tttataagta ttttgggtat ttggggagta 4020
gttttgagtt gtacgtattt tttatagttt tatttttagg ataggttaga aggtaaggtt 4080
agggtatata ggttagagat gtttaggggt tagtagagtt tggtgttggg cggtttaggt 4140
tgaggtcgtg tttcgggatt aggatcgggt tggtattagg aatttggttt atgttaagat 4200
tattggtttt gtgtttttat tttgtgtcgt tatttgtttt ttttggagta agtttgtaga 4260
tatttttgta tattttatag gtcgaggaag gttttttgga aaatttttaa ggttgggggt 4320
gtacggatta gttattgggg ttttcgatat agggtagttt tggtttcggg tttttgtgga 4380
gtggttttag tttagtttta gttagggtgg ggatttttta tttgttttag gagtagggaa 4440
tatatttttt ttgtagcgtg tagtttgtag tgttgtttat tttattttaa tttttgagga 4500
ttggtttagt tattattttc gtgagtatag tttatgttgt agtttcgtga ttagtaggtt 4560
tatttttagg tttgttatgt tattttttat gagtttcgga ttttattttt tttagtttta 4620
agaggaggta tagtttggtt ttgtttatgg tatttttagg tttgttttta gttttatagt 4680
aattatttgt atggtttgtt attagttatt ttttttatag gagagttcgt cgtgtttcga 4740
gggttatttt aggttagggt tattgttttt cgtttttttt ttagtttttt ttaaggtttt 4800
ggttatagag tttagaatta gtaggtagaa gttggagtta tgtaagtttt tattggggtt 4860
cgcggggtgt ggggtgagtg gttaagattg gtttttgttt agaattttgg agttttattg 4920
gagatttagg ttgggggtta tttggttgag gaattatgag atattaaaga tgacgtcgag 4980
ggttttgggg atgttcgggt cgtttgtaaa ggtttgggcg ttttttaggg gtaggtgtat 5040
tattttaatg agtttatttt tttttattag gtttttattt ggatcgttac gttgggtatt 5100
tgttattttt gtgtagaata tggtttgttt ggagttagtt agttttattt tagatttacg 5160
ggttgagata gggtttgttg agttatttac gttggtttcg ttggtttttt ggttttttta 5220
ttatttttta atttattttt tttattttgt tttttttagt tttttagtag gtaggatttt 5280
gggatgtttt tttttattcg gatttttatt tggtgtgtac gttcgtggtt tttttcgtag 5340
tagggtattt tttttttgat tacgttg 5367

<210> 171
<211> 7534
<212> DNA
<213> Artificial Sequence

<220>
<223> chemically treated genomic DNA (Homo sapiens)

<400> 171

ttgtcggatg ttgttttttt ttggttttgc ggtttttgtt tttttttaat tagtttaagt 60
tttttttgtt tataaaatat ggttaggcgt aaagtgtata ttggagtaag gtgttaagtt 120
ttttatttaa ttttttattt taaatagtgt tttgatttag ggatttcgga taagtttttt 180
aatttgggtt ttttggtcgg gaaagtggag atagtatttt ttttttttat tttttattat 240
ttttttgtta tacggtttag gggagatcgt gatataagtt gtgaagtatt gtatacgagt 300
agattatttt tatgagagag tgatgggtga gattaaggta atttgagttc ggagagaatt 360
agacgggaag gagaggcggg agattttggc gtttagattc gagcgatttt atttacgtag 420
ttttttgcgt ggtattattg cggttttatc gagtttgggc ggagtttgcg tggtttttgg 480
gatcggtaga gtttaggggt gtttcgggtt ggttattttt tttttttttc gggatgggtg 540
tagtagggtg tgtatttcgt atggggtaat tttttagagt cggttacgtt ttaggcgttt 600
ttttttacgg ttttgttatt tttttttgtt ttttgggttg ttttggaatc gaagagagag 660
gagggaaatg gttaggggcg gttattattt tcgggattaa tagtagtcga gagtgattgg 720
gtaggttttg tttatgggtc gagcgttatt ttggaatttg gagggggcgg gggcggggga 780
aggaagggaa gtggaagttt gagaggggta tttttaggtt ttttaaattg tatggtagtt 840
ggagagggtt gatagaattt tttttaattc gggttttgat tagggttttt ttggggttga 900
tcgttaagga gttttttaag aaaagaaatt tggattgtga aagttatttt ttaggttttt 960
tggggtcgta ttcgggggtg tttgggagga gggtggaaag gattttgtag ttatcgagtt 1020
atgggcgaaa atgattttcg cggattttgg tttagaatgt ataggttttg ggcggtgtaa 1080
gtggttgtgg gtttgtggag gatattgggt aggaaaacgt ttgcgtagtt gaggatgtag 1140
ataataaatg tggtcgggaa ggtgaggagg gaaggttggt gtttttcgtt gaacggaatt 1200
ggtttaaatg tagagttgag ttgagcggtt tcggagaagg cgaatttaga aagtttggtt 1260
aatttttttt ttatgttttt gcgatcgatt atgttttaaa ataaaagttt aatggaaacg 1320
aaaataaaat aaaatttatt atttgtttaa tgtgttgttt tcgtggagga gttaaatttg 1380
gattttaaat ttagaatttg gagttaagag gcgtattttg agttgcgttt ttggtttttt 1440
ttgtagagac gttagggttg gtttttgcgg atttgtttat tgagttattt ggatattagg 1500
acgagcggag tttgacgttt tagagcgagc gttttgtttt tatttttaat ttttttgttt 1560
cgttggttcg tttgtgggga ggggtttttt tttttttata tttaggtttt aaggttttta 1620
tatttttaaa tggatggagt aatttttttt tgtttattag agggaggtga taggttttgg 1680
gttggggaag ggtcgatagt taggtttagt tgtcgttcgg taggttttat tgatcgtttt 1740
atattttatc gtgatttttt tgttttgtat gagaaggagt ttagttggga cgggggcgga 1800
ggtaagttat tcgggattgg ggttattata gattttagaa aggggcggtt gggtcgaggg 1860

cgttggcggc gagaatcggg atagttgcgg ggaggggagg agtgtgagga tagtagggtt 1920
ggattggttt ttttttcgta ggttttacgt agcgtagtag gttgggaggc gtttgggggt 1980
tcgggtggaa gaagtgggga ttttatgtta tacggttttt ggcgtttgcg ggggtgttag 2040
aagtttatcg agtatcggtc gtttttcgtc gggaagtgtc gggttcgtag gaggagcgcg 2100
aagtcgagtt cgagttcgcg gagttcgttt tttagatcgt agaatcgttt tttttatttt 2160
ttgttttgtt tcgcgtcggg gtttgggagc gatcggcgga ggtcggcggc gcgtcggagt 2220
tgattagttt tttacgtttt ttcggttcgt agcggggagt ttatatgtgt atgtgtaaga 2280
aggtgtggag aaggcgttag ggtttcggta tttacgggtt agggtagaac gttaacgagt 2340
cgtattgcgg gttttcgcgg tttcggtttt tatgatatgg tgacgtcggc ggtaaggcgg 2400
ggggttgttt gtgggttttg aggttgttgg gcggtgcggg cggagttagt aaaggggcgt 2460
tggtaaagtt ttaaaatttt acggttttac ggagcgtaag ttagattttt tgaagtagtt 2520
ttttcgttcg ttcgaagagg gtattttatt tgtatagcgg gattttttgt tagtttaggt 2580
ttattgcgat ttcgtttttt cgagtatttt tttcgtttta gttttcgttt ggtttgcgta 2640
tcgcgagtgg ggtttaaagg ttaattttat aatattagtt agtttcggag ggacgtttat 2700
tattattgtt ttttatgttc gggttaaatg agttttttta gtattttttt tttcgaagta 2760
taaaaacggg gagtaaaagt gggatcgttt agtttaatta tttttagatt ttattttagg 2820
agaaattttg gttaagttta tttagaatag gtgtggtcga aacggaattt agggtttgat 2880
ttgagtgttt cgaaaaatta tagtaatttt tttaggcgaa ggtattttag attaggattt 2940
gttttttacg tgtgggaaaa taggtttttg ggggcggcgg tttcgagttt ttagttagag 3000
cgggattagg gagttacggg ttttttgagg agtgaattat ggagggtgtg gtttgttgtt 3060
gtttggggaa gtcggtgtta attcgaagtt tggatagggg aggattggaa tttcggtata 3120
cgttgtttat gtattgtacg tttatttatt gtagtttttt cgtggcgttt cggtattttt 3180
tttttttttt tttttttttt aacgttacga ttattatttt tagtcgtcgt tggagttttc 3240
ggtttttcgt ttagtcgttt gtttttgtac gtaaaaggtg ttttcggatt atagtttttt 3300
tataattttt tcgttagggt tgtaattttt tattcgaggc gtttttcggt ttattttttt 3360
tttcgcgtcg gcgtcgcgtt ttcggttttt tcgtttttat taggaaatcg ttcgaattaa 3420
ttttgtaaga gtgttttgcg ggagatagat atttagttcg tttaggattc ggattttgtg 3480
gattttggat ggattggggg agggggtata gaaagaggga cgggaggagg gagattttgt 3540
tgttaagagt tttttttttt tttaaagatg tgcgttgcgt ttgggataaa tttttaggtt 3600
aagaaagtac ggggggataa tggtaatagt cgttgaattg taaagtttag tagtgtaggg 3660
gagtcgtttg ggatttcgga ttcggttgta ttttaatttt ttataaggtt cggttttgga 3720
ttatttttat tttatagtag aattttggtt ttcgtttgcg tttattttcg tttttttttt 3780
gtttttttta tcgaaattta cgttttatcg gagtaggagg gggtttaggt acggtgttgt 3840
tcgtcgttcg cgtcgcgcgg ggtcgcgggt gtcgtcgtta ttgatttttt aaacgcggtt 3900
ttttcgtatt ttttcgggtc gggggatgta gaatggttta tagagggatc gcgaggcgtt 3960
gattggtcgt aggtcgtagt gacgtcggcg ggcgttgtat taaggcgagg gggtttttag 4020
agtttagtta atagttagga gtagtgatta agtcgtcgga gttggggaga gacgtatcgg 4080
ggcggcgatt gggttaggag attagggatt gagggacgcg ttcggggagg gttagtaata 4140
agtcgcggtt cgggcgcgat agcggcggtt cgcgtcgagt cgttttagtc gtcggtttat 4200
tgtagtcgtt gcgttaggat attttttttt aaagtttttt aagttgtttt tttttttttc 4260
gatttttttc gttgtaaaag aaaaataaaa aaaaaaaagg aaggaggtta gaaaggaaat 4320
ttagatttgt tattataacg aaaaaaagaa ggggaaaagg aaaaggagag aaagtcgagc 4380
gattgtgggg ttagacggag gtagatagag cgtgggtcga gcgatgcggg gttgtgcgtt 4440
taggcggtcg cgagttgtga ttggagttac gatgtacggt taggcgcggt gagaagttag 4500
ttcgtagtgt ttttcgaagg agttcgggcg tagggagggt cgttttgagg atacggaggt 4560
cgttaggtag gttaagggtc gaggtgattg ggttggggcg gtagggaagg agcgagtgcg 4620
tttggttgtt ttcgtacgga gttgtttttt tttgttttcg attgatataa atattttttt 4680
aaaagcgggg aaatttaagt aataatagta attaatatta agattttttt tttatttttt 4740
tttttttttt ttttttcgcg gtcggttttc gttttttttt ttaggtttag cgcgggcgtt 4800
cggcgcgttt agattcgcgg cgcgatgtcg gtagtttagg atttaaagtt tttttgtttt 4860
ttttgttttt tttttttttt ttttaaaaaa agagggggga aattttagtg gtgggtagtt 4920
tggtacgtat atagtcgttt ttatatttcg ataaaagtag atgtattttg atttttgata 4980
gttttatttt aagtttcgga gaatttagcg gcgttttttt cgtaattcga gttcggcgag 5040
tcgtcgtttt tttttttttc gtttttattt atttattttc gttttcgtcg tcgttttcgt 5100
tgatttttat tttttcgcgg gttttgagta gaagggtcgt attttttttc gtttgagatt 5160
tttttttttc gtttcgggag tttaggcggc gtcgttttag ttcggggttt cgggattttt 5220
cggttgtata ttttattgag acgttttttt aggtttcgat tagtttcgtt ttttttattt 5280
tattttgatt ttttggtgcg agttttggtt tgtacggtcg agtgtgtgtt ttttttttgg 5340
agagattggg gagttcgtgt cgattgtttt taggagttat tttttgggtt ttattttgtt 5400
tttttttttt tttgggtttt attagattaa attaaagatt atggtttatt gtgtcggttg 5460
taaaaggttt attttggatc gttttttttt gaacgtgttg gatagggttt ggtacgttaa 5520
gtgcgtttag tgttgtgaat gtaaatgtaa tttgatcgag aagtgttttt ttagggaagg 5580
taaattttat tgtaagaacg atttttttcg gtgagtattt tttttttacg tttttgttgt 5640
tattttttcg cgggtttttt tcggttagtt tcggattaga ggttagcgtg gtttcgttgt 5700
ttagttagga attgttttta gagagggtag ttaagtttcg cgggcgttcg ttttttcgtt 5760
agttggcgcg ttgggagttc gggacgcggt tttgtttgta ttttggaaat tatgggtttg 5820

ggtttggttg tatgtgtttg ggaagaaagg gtttgagttt gattcggttt agaagttttt 5880
ttaagtttgg aagagatagg gtagtttcgg gcgttgtagt tcggattgtt gtttttttcg 5940
gagttaagtt ttgtagtaat tgtatttttt aggttttcgg ggttgtttgt tttttaggta 6000
gagtttaggt tttttttcgg ttattatttt tttcgatttc gagcggtttt ggcggtaagt 6060
ttcgagtttt tttgcgtaga gtgtttgggt taattgtttt gttagcggta ggaaagaatt 6120
cggttttaaa gtcgattttg tacgttcggg ggtttttttt aaattcggat atgttttcga 6180
gtgtagattt ggataaattg ttgtattgta gtttgcgaag tgttcggagt ttttttttgt 6240
ttttttggtt gtcgaagttg tttggtggtt cggttttagt tttaggtgat ttcgattttt 6300
acgggtttcg tttagtagtt tttttaggaa attgttagtt atagttagta atttttttga 6360
tgaaatttgg gggaaagtcg agatttttag tttgttggtg ggagattttt tttttatttt 6420
ttttattcgt ttagtatttt aattatatta tcgtatattt ataattttgt cgttttattt 6480
atataaatat ataagttaat agagaagaac gggtataaat gcgaattgta attttatttt 6540
acggatgtga aatatcgtaa tttattcggt tgatttggtt tttgtttttt tgagtttgtg 6600
gttatagttt aaattaggga gtaggaggaa gagggattta aagttattta gagggttaag 6660
acgcgttgtt tttagtgtcg ttttaggatt cgtggttggg gttgggttgc gtggaattat 6720
gaagtaaagt ttcgtgttcg ttggtatttc gggagaacgc gtaggttgag tttttgagtt 6780
ttcgcgggtt gttcggtttt ttttaaggat tggattatag cgggagggtg gtaaatcgta 6840
ttcgtattcg ttgttagtgg gtttttgagt ttggttttta taattcgcgg tagagcgttt 6900
agagttagtt ttcgtagttt tcgtttttgt taatttgttt cgttttaggt aagagcgtgg 6960
cggcggtttc gttaaggtta cggagggggg tttttcgcgg ttatgtttgt ttattaatag 7020
cggggttaga tatcgttaat tttatgttta tgtgcgggtt tttttgcgat gaaggagaga 7080
aaagcggcgt ttcgcgtacg tttatataga gttttaattg taattcgatt tttttgaatt 7140
taatcggaaa ataagtgttt cgtttttttc gtttatatag gttattgttt tgttattttt 7200
tttagacgat gtattttttt tttgggataa cgttgggagt gaggttgggg tttggcgggt 7260
agtttgggtt tttggtttta atcgcgtgta tttttttttt aatttttttt tggtgtcgcg 7320
tttttttttt tgttgttttt ttatttgttt aatcggaatt ttgttttttt ttattttttt 7380
tttttttttt ttttgtgttt tatttttttt tttttatttt ttttattttt tatatttttt 7440
ttttttttcg ttgttatttt ttttttcgtt tgtttttttt tgtttttggt tgatttattt 7500
tcgttttcgt tttttatttt tattttttcg tagg 7534

<210> 172
<211> 7534
<212> DNA
<213> Artificial Sequence

<220>
<223> chemically treated genomic DNA (Homo sapiens)

<400> 172

tttgcggggg ggtgggggtg gggggcgggg gcggggatgg gttagttagg ggtaagggga 60
gataggcgga gagaaagatg atagcgagaa agagggaaga tgtaaaaagt ggaggaagta 120
ggagaggagg aatggagtat agaaagaagg gaagagagag ataaagagag gtagaatttc 180
ggttaagtag gtgaggaggt agtaggagag agagcgcggt attagggaag agttagggag 240
gggatatacg cggttagagt taggggttta agttattcgt taagttttag ttttattttt 300
agcgttattt tagaggaaag atgtatcgtt taaagaaggt ggtaaggtag tgatttgtgt 360
gggcgggggg aacggggtat ttatttttcg attagattta gaaaagtcga attataatta 420
gaattttata taaacgtgcg cggagcgtcg tttttttttt ttttatcgta aaaggattcg 480
tatataggta tggggttggc gatgtttagt ttcgttgttg gtaaataaat atagtcgcgg 540
agagtttttt ttcgtagttt tagcggggtc gtcgttacgt ttttgtttaa agcgggatag 600
gttggtaaaa gcgggagttg cgggagttgg ttttgggcgt tttgtcgcgg gttgtggaag 660
ttaggtttag ggatttattg gtagcgggta cgagtacggt ttgttatttt ttcgttatga 720
tttagttttt gaggaaaatc gggtagttcg cggaggttta gaggtttagt ttgcgcgttt 780
tttcgaaata ttagcgagta cggagttttg ttttatggtt ttacgtaatt taattttagt 840
tacgagtttt ggagcggtat tggggatagc gcgttttggt tttttgagtg gttttgaatt 900
tttttttttt ttgttttttg gtttgaatta tgattataaa tttagaaaag tagagattaa 960
gttagtcgag taaattgcga tattttatat tcgtagggtg gaattataat tcgtatttgt 1020
gttcgttttt ttttgttgat ttgtgtgttt gtgtgaatgg agcggtaaaa ttgtgagtgt 1080
acgataatat aattaaaatg ttaaacgaat aaggggggta gggagaagtt tttttattag 1140
taagttggag gtttcggttt ttttttaggt tttattaagg aggttgttgg ttgtggttgg 1200
tagtttttta ggaggattat tgagcggagt tcgtgggagt cggagttatt tggggttgaa 1260
gtcgaattat tagataattt cggtagttag ggaagtagga aaaagtttcg ggtatttcgt 1320
aggttatagt gtagtagttt gtttaggttt gtattcggag gtatgttcgg gtttggggaa 1380
gattttcggg cgtgtaggat cggttttaag gtcgagtttt tttttgtcgt tgatagggta 1440
gttggtttag atattttacg taaaagggtt cggagtttat cgttaagatc gttcggggtc 1500
ggaaaaggta gtggtcggga gggagtttgg gttttgtttg gagggtaaat aatttcgaag 1560

gtttggagaa tgtagttgtt gtagagtttg gtttcgggaa agatagtagt tcgggttata 1620
acgttcgagg ttgttttgtt ttttttaagt ttgaaggggt ttttgggtcg agttaggttt 1680
agattttttt tttttaggta tatgtagtta aatttagatt tatagttttt aggatgtaag 1740
tagggtcgcg tttcgggttt ttagcgcgtt agttggcgga gaggcgggcg ttcgcgggat 1800
ttggttgttt tttttagaag tagtttttaa ttgggtagcg aggttacgtt aatttttgat 1860
tcgaagttgg tcgggaaggg ttcgcgggga ggtagtagta gaggcgtggg aggaaagtat 1920
ttatcggaag aagtcgtttt tgtagtagag tttgtttttt ttggagaagt atttttcggt 1980
taggttgtat ttatatttat agtattggac gtatttgacg tgttaggttt tgtttagtac 2040
gtttaagaga aagcggttta ggatgggttt tttgtagtcg gtatagtgaa ttatggtttt 2100
tggtttggtt tgatgaggtt tagagagaga gaggggtaaa gtagagttta ggggatgatt 2160
tttgaagata atcggtacga gttttttagt ttttttaaaa agaggatata tattcggtcg 2220
tgtaagttaa aattcgtatt aggaaattaa agtagggtgg aggaaacgag gttgatcggg 2280
gtttgggggg gcgttttagt gaagtgtgta gtcggggagt ttcggggttt cgggttggag 2340
cggcgtcgtt tgagttttcg gggcgaggaa aagaagtttt aggcgggaga gaatgcgatt 2400
tttttgttta gagttcgcgg aggagtgaag gttagcgaga acggcggcgg gaacggaaat 2460
aaataaataa aaacggagaa gaagaagacg acgattcgtc gagttcgggt tgcgaggaaa 2520
gcgtcgttga gtttttcggg gtttgaggta gagttgttag aagttaaagt gtatttgttt 2580
ttgtcggggt atgaggcgga ttgtgtgcgt gttaggttgt ttattattgg gatttttttt 2640
tttttttttt aaaaaaggga aggaaagaat aaaaggagta gagaagtttt ggattttaaa 2700
ttgtcggtat cgcgtcgcgg gtttggacgc gtcgagcgtt cgcgttgggt ttgggggagg 2760
gggcgagggt cgatcgcggg agaaggaaaa gaggggaggg taggaggaag attttggtgt 2820
tgattgttgt tgttgtttag gttttttcgt ttttggagaa gtgtttgtgt taatcgaaaa 2880
tagagaggga taatttcgtg cggaggtagt taggcgtatt cgtttttttt ttatcgtttt 2940
agtttagtta tttcggtttt tggtttgttt ggcggttttc gtgtttttag ggcgattttt 3000
tttgcgttcg ggttttttcg ggaggtatta cgggttggtt ttttatcgcg tttggtcgtg 3060
tatcgtggtt ttagttataa ttcgcggtcg tttgggcgta tagtttcgta tcgttcggtt 3120
tacgttttgt ttgttttcgt ttaattttat agtcgttcga tttttttttt tttttttttt 3180
tttttttttt ttttcgttgt ggtggtaaat ttgggttttt tttttagttt tttttttttt 3240
ttttttttgt tttttttttg tagcgggagg agtcggggag gaggggggta gtttggagag 3300
ttttaaaaaa aaatgttttg gcgtagcggt tatagtaggt cggcggttgg aacggttcgg 3360
cgcggatcgt cgttgtcgcg ttcgggtcgc ggtttgttgt tggttttttt cgggcgcgtt 3420
ttttagtttt tggttttttg gtttagtcgt cgtttcggtg cgtttttttt tagtttcggc 3480
ggtttggtta ttgtttttgg ttgttggttg agttttggaa gttttttcgt tttaatgtag 3540
cgttcgtcga cgttattgcg gtttgcgatt aattagcgtt tcgcggtttt tttgtaaatt 3600
attttgtatt tttcggttcg ggagagtgcg gggagatcgc gtttagagga ttagtggcgg 3660
cggtattcgc ggtttcgcgc ggcgcggacg gcgggtagta tcgtgtttgg gttttttttt 3720
gtttcggtaa ggcgtgggtt tcgatgggag ggatagagag agagcgggaa taggcgtagg 3780
cggaggttag agttttgttg tagagtgggg gtagtttaag atcgagtttt gtggaaggtt 3840
ggggtgtagt cgggttcgga attttaggcg gtttttttgt attgttgggt tttgtaattt 3900
agcggttgtt gttattgttt tttcgtattt ttttgatttg gaagtttgtt ttaagcgtag 3960
cgtatatttt tggaggggga ggaggttttt agtagtagaa tttttttttt ttcgtttttt 4020
tttttgtgtt ttttttttta atttatttaa agtttatagg gttcgggttt tgggcgagtt 4080
gagtgtttgt ttttcgtaga gtatttttgt aaagttgatt cggcggtttt tttgatgggg 4140
gcggggagat cggaggcgcg gcgtcggcgc gggggaagga gtggatcggg aagcgtttcg 4200
ggtgaggagt tgtaattttg gcgagaaagt tgtgagaaaa ttgtgattcg gagatatttt 4260
ttacgtataa aaataaacga ttaggcggga aatcgggggt tttagcggcg gttgggggtg 4320
gtggtcgtgg cgttgggaag agggaaggag gagggggggt gtcgggacgt tacgaagagg 4380
ttgtagtaga tgggcgtgta gtgtatgggt agcgtgtgtc gggattttag tttttttttg 4440
tttaggtttc gggttggtat cggttttttt agataatagt aaattatatt ttttatagtt 4500
tattttttag ggaattcgta attttttaat ttcgttttgg ttaggaattc gggatcgtcg 4560
tttttagggg tttatttttt tatacgtggg ggatagattt tggtttagag tgttttcgtt 4620
tggaagagtt attgtagttt ttcggagtat ttaagttaaa ttttaaattt cgtttcgatt 4680
atatttgttt tgggtgggtt taattaggat tttttttgaa gtggagtttg aaggtggttg 4740
ggttgggcgg ttttattttt gtttttcgtt tttgtgtttc gggggagggg gtgttaggaa 4800
ggtttatttg gttcgagtat ggaaggtagt gatagtgggc gttttttcgg agttggttgg 4860
tattgtggaa ttggtttttg gattttattc gcgatgcgta gattagacgg aagttgaaac 4920
gggggaaatg ttcggggaag cggagtcgta gtgaatttgg gttggtaggg aatttcgttg 4980
tgtagatgga atattttttt cggacgaacg gggaagttgt tttagagaat ttggtttgcg 5040
tttcgtggaa tcgtggggtt ttagggtttt gttaacgttt ttttgttgat ttcgttcgta 5100
tcgtttagta attttaaggt ttataggtag tttttcgttt tgtcgtcggc gttattatgt 5160
tatggaaatc gaagtcgcgg agattcgtag tgcgattcgt tggcgttttg ttttgattcg 5220
tgggtgtcgg gattttaacg ttttttttat atttttttgt atatatatat gtgggttttt 5280
cgttgcgggt cggaggagcg taggggatta gttagtttcg gcgcgtcgtc ggttttcgtc 5340
ggtcgttttt agatttcgac gcggggtagg gtagaggatg gagaaggcgg ttttgcgatt 5400
tagggggcgg gtttcgcggg ttcggattcg gtttcgcgtt ttttttgcgg attcggtatt 5460
tttcggcgga agacgatcgg tattcggtag gtttttggta ttttcgtaag cgttaaaagt 5520

cgtgtggtat ggggttttta ttttttttat tcggattttt aggcgttttt tagtttgttg 5580
cgttgcgtga ggtttgcggg gagggggtta gtttagtttt gttgttttta tatttttttt 5640
tttttcgtag ttgtttcggt tttcgtcgtt agcgttttcg atttaatcgt ttttttttgg 5700
aatttatagt ggttttagtt tcgggtggtt tgttttcgtt ttcgttttag ttgggttttt 5760
ttttatgtaa agtagagggg ttacggtgga gtgtggggcg gttagtggga tttatcggac 5820
gatagttgga tttagttgtc ggtttttttt taatttaggg tttgttattt ttttttggtg 5880
ggtaggagga ggttgtttta tttatttagg ggtgtaagaa ttttgggatt taggtatagg 5940
ggaaagaagg ttttttttta tagacggatt agcgaggtag gagagttgag gatgaggata 6000
ggacgttcgt tttagggcgt taggtttcgt tcgttttggt gtttagatgg tttagtaaat 6060
aggttcgtag gagttaattt tggcgttttt gtaagggggg ttaggagcgt aatttaaagt 6120
gcgttttttg gttttagatt ttaggtttgg agtttagatt taattttttt acggggatag 6180
tatattagat aaatggtggg ttttgttttg ttttcgtttt tattgagttt ttgttttaaa 6240
atataatcgg tcgtagggat atggagagaa aattgattag gttttttaaa ttcgtttttt 6300
tcgaagtcgt ttagtttagt tttgtattta agttagtttc gtttagcgaa gagtattaat 6360
tttttttttt tattttttcg gttatattta ttgtttgtat ttttaattac gtaagcgttt 6420
ttttatttaa tgttttttat agatttatag ttatttatat cgtttaggat ttgtatattt 6480
tggattaggg ttcgcgggga ttattttcgt ttatagttcg gtgattgtag aatttttttt 6540
attttttttt taggtatttt cggatacggt tttagagggt ttaggaggtg atttttatag 6600
tttaggtttt tttttttgag aagttttttg acgattagtt ttagagggat tttggttaga 6660
attcgggttg gagagggttt tgttagtttt ttttagttgt tatgtagttt ggggagtttg 6720
aaggtgtttt ttttaaattt ttattttttt tttttttttc gttttcgttt tttttaggtt 6780
ttagaatgac gttcggttta tggatagagt ttgtttagtt attttcggtt gttattgatt 6840
tcgaggatga tggtcgtttt tagttatttt tttttttttt tttcggtttt agaatagttt 6900
aaggagtaga aaagagtgat aaggtcgtga aaggaggcgt ttggggcgta gtcggtttta 6960
ggaggttatt ttatgcggag tatatatttt gttatattta tttcggagga gggaggaaat 7020
agttagttcg gagtattttt ggattttgtc ggttttagag gttacgtaaa tttcgtttag 7080
attcggtgag gtcgtagtaa tgttacgtag ggaattgcgt ggataaggtc gttcggattt 7140
gagcgttaag gtttttcgtt tttttttttc gtttagtttt tttcggattt aaattgtttt 7200
aattttattt attatttttt tatggaaatg atttattcgt gtgtagtatt ttatagtttg 7260
tattacggtt ttttttgaat cgtatagtaa gagggtggtg gggggtggaa gagaagggta 7320
ttatttttat tttttcggtt aggaggttta agttaggagg tttgttcgag atttttaggt 7380
tagagtatta tttaaggtga gaagttgggt ggggagtttg gtattttgtt ttagtgtgta 7440
ttttgcgttt ggttatgttt tataagtaag gggggtttgg gttggttaag ggggagtagg 7500
gatcgtaggg ttaggaaagg gtagtattcg gtag 7534

<210> 173
<211> 7406
<212> DNA
<213> Artificial Sequence

<220>
<223> chemically treated genomic DNA (Homo sapiens)

<400> 173

tttagggatt attttgtgtt tgtagttgtt tttttagggt aggtttttga gggttttttt 60
tgttttttcg ggtgttcgat attagacgtg gggattttaa taggggagga gttaaggaat 120
tttgtggttg tgttgcgttt tagaaaataa tttttagagg ttttgggttg tggatttggg 180
ggttggaagg atgggggttt atttaatttt tagaggtagc gtttttgttt gtttatttgg 240
tgataagaga gagataagta aatgggggtc gttgggacgg cgggtgtttg gagggtagtt 300
ttgggtttag cgggtagtgt ttagagtata ggtttttttg ttgggggatg gggaggagag 360
tagtttgttt ttgggagcgt aggttttagg gagattttta aagttttttt tgtcgtttgt 420
tttttattta gtattataga ggtatttgtt gtatatataa gtattttatt cggtttacgg 480
agggggttag gttttttttg tttgaagttg ttttgggaag ggtttttata taggtattga 540
ttttttaagt ttttgttatg atgtttttta ttatttgata attttaaata ttgtttttaa 600
atttaaaata tttagtggtt cgttgttttt gaagatgtaa ataaataaat atattatttt 660
tgggaatatt tatattgaga ttttttgtgg ttattggtgt gttttatagg taaaatttga 720
tttggttaaa ataggtttag atgtatttgt gtgttcgtgt gtgtgtgtgt gtgtgtgtgt 780
gtgtgtgtgt gtgtatgaga gagagagaga ttttgacggt tgtagatatt tttttcgttt 840
tgtttatttt atgttgtata attatgtgtt tattaataag ttgatgatat ggatgtattt 900
ataagattat gtaatattga tgtgattgtt gtcgtttgag aaaaaaaggt aatagttgat 960
ttttttaata attgttatag aatggttggg ttgagaacgt tgtttagggt tttgtagttg 1020
gcgggagagg tgtttggtgg gagcggtgtt tggtgcgtta gtttgttgtt tcgtgtttta 1080
ttcgagagtt gtttttggtt ttatattttt taattttttt gtgttttagt agtcgtgatt 1140
ttgtttttat tgttttattt agtgtaggtt tgggttattg aagatattaa agtgtttttt 1200
ttttagtttg aaaattaggt aggtttatac gtgggtttta gtgtatttgt ttttgaattt 1260

tttaaattaa attttagttt tttggttggt gttgaatgtt tttagttggg gtgatttgga 1320
gtgatttggg acggtgttgt ggtttatcgt ttttagtttt agttttttta gaaatttttg 1380
tggagggtag tgttggttgt aggttttatt atattgtagt ttgaggttat tattatttgg 1440
ggaataagtt tgcgttttgt tgaggggtag gatttttagt agagcgtcgg tggggttggg 1500
tcgtcgtgga ggtggtttta ggagttatat ggttatattt agatatattt tgtgtggttt 1560
gtttagagtt ggatgttatt tttaggtaat gtttagagtt tattttttaa agttttaagt 1620
attttaagag gtattgagat taatgaatat aatagtttag taatttaaat gtttatttat 1680
ttttagtgga aggattttta ttaaaaagaa gttaattgat atggaaatgt tagtgaaatt 1740
ttttatttgt aaggaaagtg aatattttgt atttaagtaa attataatgt gtatatttta 1800
ataaagaaat ttgatatttg aaaaaatggt gatttttttg aaatgtgaat tagttttatt 1860
agtgggagtt gtggtagagg cggtgggtgt agaatggatt ttttttttgt ttacgtattg 1920
gatatttgaa attttagagt tttttttatc gaattattgg tttttgggat atagtttaag 1980
ggtcggatgg aaagtagaag atggaaagta gtgatagatt atttttttgg agttgatttg 2040
gacgagattt ataggataag atttttgatt ttatataggt taaagggtag ttgagatgta 2100
tgttagttat atattttaaa gagtacgcgc ggtagtttag tttgagggta ttttagagga 2160
tttttagttt gtttgagtgg ttattttttt gtttttgggg tgtgagtgtt ttgttaaggt 2220
tgtttgtggt gttttgtggg gtgtttttga tttcgtgttg ttttttgttt tttttttatt 2280
tttgttgtgt tagtgggggt agggtagtgg ttaatagttt tgtttgtggt tgtggaggaa 2340
gtcggttggt atttttttgg ttaggttgtg tagaggttgg tagtaattta gttattcgtt 2400
aggtatcgag tagatattag tttgggtgtg tattgggcga tttttttagg ttttgtttat 2460
gtttaaggtt ttgagttttt agtagtgttt ttgttatagg ggattgtagg tgtttttttt 2520
tttggtagtt cggaggggtg cgggttgagt tttttttaga agttgggggt tgagtttaag 2580
gaggttaaga gtggaaagtt ttttgattag taaaatattt gtaggagagt gttttagtga 2640
gttttgggtt ggagttgatt acgtatgggg ttgtttcggg gtacggtttg ggtattgttt 2700
tttagaggtt gtacgttaga agggtagggt tagtgttttt ttgttttgcg gttggacgat 2760
tcgtttgttg cgaaattcgg cgaattttag cgttttagta gggtttggag tttagggagg 2820
agcgggtacg gttattttgg gttttagagg ggtgagggcg ggggttgttt tgagtgcggt 2880
ttaatttagt tagggaggga ggcgcgttcg atacggggta gaggcggggg tggggatcgg 2940
ggcgacgaga ggggtttcgg gaggtgggcg ggcgcgcggg cggggcgggt agcgggcgtt 3000
ttttttagta tcgcggatag cgttaggttt agtggttttc gggttttttg tttcgtacga 3060
cgcggcggga tttgggagtt tcgaattttt taggggacgt tgatttagga ggagcgcgtt 3120
tcgcgttatt cggtttggtg gtcgcgatgt tgttggaaac ggggttcgag cgcgatcgag 3180
cgaggttcgg ggtcgtcgtc gtttgtattt tgggcgggat tcggggtaag ttttagtagg 3240
atattgagga tagaaacggt aagggcggta gaggcgcgag gaagggggtg cgtgtagggt 3300
tagcggttag gtaaagaaag tttcgtcgtt ttgtaggcgg gatatagaga tggaaattgt 3360
agagagtgag tttttagatt ttagggtggc ggggagggtt tgacgttggt ttgtaagagt 3420
gcgggatagc ggttggagtg gaggttttta gaaaaaaagg gggtatcgta ggtatagttg 3480
gggggcgatg gggtcgatta aggggtgtta ggttttttcg gtgattagtg ttcgttagtt 3540
tttgtatata gttcggttta ggtttttgga ttttatagta ggggatttaa gtttgtgttt 3600
ttcgtttgaa ttattttttt taagggttat tttattatta cggacgttgg gaaataatgg 3660
aggtattgtt ggagggttgg ttagatgtta gtagggtggg tcgttttttt aatttgtcgt 3720
cgtttttttt ttagttttgt tatatacgat ttttgatcgt ttcggtagta gttgatattt 3780
agttatttgt atttagtata gttcgtatat attcggtttt gtattcgcgt gtttttgttt 3840
tggttttttt tgggtaataa gtgttgtgta aagtgaaggg gtagaaagtt ggttgtatgg 3900
gttattgttt aaaacggata tatcggattt gttgggagtt aggagggagg gattgtggtt 3960
ttttgtgttt atttttttgg gtttgggttt ttaaagttta tagtttagaa gttataggta 4020
gagtggatag agtattgttg tgagatttat agggagaggg atttgtagga tggtattagt 4080
ttttggtttt ttaatttttt ttgtgtgttt ttgcgtattg ttagggtatt tttgtttttg 4140
ttaagttttg tgttgtcgag ggttatttat tgttgtgttt tttttcgggt ggttgtttag 4200
ggttggtgtt ggtttagggt tttttgggta ggggtgggtg cgtttttttg tttgtaagga 4260
taggtgggtt ttggagagtt tatttgtgtg gattggggta agaggttgaa atattaggta 4320
agggatcgtg ttttaatgga gtcggagtgt tgggggttgg aaatggaagg tgtgttttgg 4380
ggttttttta gttcggtttt ttacgattcg aggttttggt tgcgtgttta ggatatagag 4440
tttgtttttt tttaaggatt gttttttttt tttgagtcgt tcggggtcgt agttttaggg 4500
gtggagttta gaagtttgtt agtatttggg atcggttcgg tattttgcgg tttcggtacg 4560
tagttttcgg cgttatattt attttttttg cgtttcgggt tgagttttcg tcgattattt 4620
gttgttttcg aggttaaatt ttagcggcgg gaggattttg ttgttttttc gatttttttt 4680
tcgggaattt tagttttttt ggcgtgtgtt ttagtttagg tttttgtttt tgggtcgttt 4740
cggcgtagga agttgcgggg tcgggacgaa cgttggcggg aagttttgat ttgggttttt 4800
ttttatcggt gttcgttttc gggtcgggta cgcggggcgg tttttgggta tcgtaggttt 4860
cggcgtaaag gcgtttagag ttcgtagtgg ttcgggttgg ttttcgcgat tttcggtttc 4920
gtttcgtttc gcggtttcgt ttttcggtcg tttcgttttt cgttcggtta gaggtttcgg 4980
gttttagggc ggttcgcggg cgtagcgttt agtagtattc ggagtcgttt ggacgtcggt 5040
tcggggttat tgcggggtgg cgtcgttggg ttcgggaaag ttcgggattg gagagtggcg 5100
acgtcgggcg gcgggattta tggaggcgcg cggggagttg ggttcggttc gggagtcggc 5160
gggaggcgat ttgttgttag tattgttggc gcggagggcg gatttgcgtc gaggtgggtg 5220

ttcgggtcga gtggttgtat cggggagatt aggagatttt taggtttttt cgggtttggt 5280
cgcggaggtt ggtaggagtt agaggatttg ggcgggagtg ggcgcgagga tttcggaacg 5340
ttcgcgtttg ggcgttttag tttgtatttg ttgtagggtt tttggtttgg gttgttaggg 5400
agtgagtgag gttgtggtat tgcgttgttt cggttaggga gttttcgggg gtttagggtg 5460
ggtttaggag atttttgtag ttcggagtta gttttttggg ttggaggagg cgtagggagt 5520
agtggcgggg tagtgattac gggataggag ggtttttaag aaggtcgttt tagtcggatt 5580
ttttttacgt tttagcggaa tagagttaga tgtaggggtt aaggtcgagt tgaatttcga 5640
tcgtcggttt tttcgaagtt aggttttagc gtttgcgttt atagatattc gttcggttta 5700
ttttcgttta gggttcgttt gtaggaaaaa gttttttttt tttaggtttt ttagtttttt 5760
ggtggtatta ttttagtttt tagtattttg gtttttaggg aatttgggta gtttttttcg 5820
attttcgggt tagttggagt cgtaggatgg ggaaggaggt ttcggagtta gtggggagtg 5880
agagggatcg gtcggcggga agggggttat atttaggttg tgggggttcg cggtttttta 5940
tttatttatt tatttttcga cggtttttgg gaggggttgg tcgcgggggt tggggggcgg 6000
agagagggag ggaggaagga ggattgcgcg ttcgcgttcg ggagagttgg tcggagcggg 6060
cgggttggcg tttaggtttc gtcgatttcg ttatttttga tataggagtt ttcgttaggg 6120
ttggagtcgt tatgtagcgt aaggttgggg tcgcgggcgc ggcgcggggt gggttggggc 6180
ggtttttgtt cgacgcgggc gtcggcggcg agttgcggtc gaggcgttgt tcggttcgcg 6240
gtgttgaatt cgcgttgtgt cggtttgtcg ggtcgtttcg tttcgagtcg ggttttcgtt 6300
tgcgatcgtt gtttacgatg gggatttcgg gcgcgtagcg ggttttcgac gtttcgtatt 6360
cggagttgcg gttttgtcgg atgcggggcg tatttatcgt cggtttttcg ttattgcgtg 6420
gggaggcgta gtttagtttt tttcgtcgga ggtcgaggga gttttttttt ggtgtttttt 6480
atttattggg agatgggttg gagtcggcgg ggtttatagt tagggaggcg ggtgtaatat 6540
gttagtaaat ttcggttttt ttagcgggta tttttttttt ttagagattt tttttaagtg 6600
aagagggagt ttttagtttt tgatatttgg aaaattcgtt tatagattcg aggtttttat 6660
agggtatttt tggatttttt tagtgtggtt tttttagggg gtttgtagta tcgaagtggg 6720
gtgtgggggg tagaagtagc gggaagttag acgtttgagt aattttgacg gtgttggggg 6780
tatttacgtt tgatataaag gtagtggatc ggtttgttta gtttatttat aagtaatttt 6840
gcggttgttt aatgatgggg aaggtattga tatttatttc ggaggtttgt agatagggtt 6900
attaggtagg gtagttatat ttgcgaggag tttttagaaa gagtggttat gacgtttaat 6960
atgaaggaga ttcgatgggg tgttttagtt ttaggtgatg gtgaagattc gtttttttat 7020
gttttttgtc gggttttaga gagtagattt ttttggggtg ggttttttat ttgagtttta 7080
tatgtatttt ttttggtatt ggtaatattt tgtaattgag tatttagttt cgtgaaatgg 7140
tttgggttgt ttttttgttt atatatattt ttagatcgat gttagttttt ttttagtttt 7200
tgatatagga tgcggtgttt gtatattttc ggaatttgcg gggttattta agttggttgg 7260
ggaagagggt gtgtagggaa aggaggattt ttcggtagtt ttgagttttg tgggtcggag 7320
gggagattgg ttcgcgttgg ggtgggaagg tggttttatt gttttttggt ttacgaggtg 7380
atttagaatt ttttcgtgtt tataga 7406

<210> 174
<211> 7406
<212> DNA
<213> Artificial Sequence

<220>
<223> chemically treated genomic DNA (Homo sapiens)

<400> 174
   tttgtgggta cgaggaagtt ttgggttatt tcgtagatta ggaggtagtg aagttatttt 60
   tttattttaa cgcgagttag tttttttttc ggtttatagg atttagggtt gtcgaggggt 120
   tttttttttt ttatatattt ttttttttag ttagtttaag tggtttcgta agtttcggga 180
   gtgtgtaggt atcgtatttt atgttaggag ttaaggggaa attgatatcg gtttaaaaat 240
   gtgtgtgagt aagaaagtag tttaggttat tttacgaggt tgaatattta attataaaat 300
   gttgttagtg ttaagaaggg tgtatgtgga gtttaaatga aaattttatt ttagggggat 360
   ttgttttttg aagttcggta ggaaatatag ggaaacgggt ttttattatt atttggagtt 420
   ggggtatttt atcgggtttt ttttatgtta agcgttatgg ttattttttt tagggatttt 480
   tcgtaaatgt ggttgttttg tttggtggtt ttgtttgtag gttttcgggg taaatattaa 540
   tgtttttttt attattgggt agtcgtaaaa ttatttatga gtggattgga taagtcgatt 600
   tattgttttt gtgttaggcg tgggtgtttt taatatcgtt agagttattt aaacgtttgg 660
   tttttcgttg tttttgtttt ttatatttta tttcggtatt gtaggttttt tggaggaatt 720
   atattgggga ggtttaaggg tgttttgtgg gagtttcgag tttgtgagcg ggttttttag 780
   gtgttaaggg ttgaggattt tttttttatt tagaaaaagt ttttggaaga gaggagtgtt 840
   cgttgaaggg atcgggattt attgatatat tgtattcgtt tttttggttg tggatttcgt 900
   cggttttagt ttatttttta gtgggtgaga gatattagga aggggttttt tcgattttcg 960
   gcggaaaaaa ttgggttgcg tttttttacg tagtggcggg aaatcggcga tgaatgcgtt 1020
   tcgtattcgg taaaatcgta gtttcgggtg cggagcgtcg aggattcgtt gcgcgttcgg 1080
   ggtttttatc gtgggtagcg atcgtaggcg aggattcggt tcggagcggg gcggttcgat 1140
   aggtcgatat agcgcggatt tagtatcgcg gatcggatag cgtttcggtc gtagttcgtc 1200
   gtcggcgttc gcgtcgggta aaagtcgttt tagtttattt cgcgtcgcgt tcgcgatttt 1260
   agttttacgt tgtatggcga ttttagtttt ggcgggggtt tttgtgttag ggatggcggg 1320
   gtcggcggag tttggacgtt agttcgttcg tttcggttag ttttttcgag cgcgggcgcg 1380
   tagttttttt tttttttttt tttttttcgt tttttagttt tcgcggttaa ttttttttag 1440
   gaatcgtcga aaaataaata aataagtagg gaatcgcgag tttttatagt ttggatgtaa 1500
   tttttttttc gtcggtcggt tttttttatt ttttattggt ttcggggttt ttttttttat 1560
   tttgcggttt tagttggttc gggagtcgag gagaattgtt taggtttttt aagaattaaa 1620
   gtgttgggag ttagagtggt gttattagga agttgggggg tttggagaag agaggttttt 1680
   ttttatagac gggttttgag cggggataaa tcgagcgggt gtttgtgggc gtagacgttg 1740
   aaatttgatt tcggggagat cgacggtcgg agtttagttc gattttggtt tttgtatttg 1800
   attttgtttc gttggaacgt ggaaagagtt cggttggggc ggtttttttg gggatttttt 1860
   tgtttcgtgg ttattgtttc gttattgttt tttgcgtttt ttttagttta gggagttggt 1920
   ttcgggttgt agaggttttt taagtttatt ttggattttc gagagttttt tggtcggagt 1980
   agcgtagtgt tataatttta tttatttttt gataatttag gttaggggtt ttgtaataaa 2040
   tataggttga ggcgtttagg cgcggacgtt tcggggtttt cgcgtttatt ttcgtttaga 2100
   ttttttagtt tttgttagtt ttcgcggtta ggttcggaaa ggtttgagga ttttttggtt 2160
   ttttcggtgt agttattcgg ttcgggtatt tatttcggcg taggttcgtt tttcgcgtta 2220
   gtagtgttag tagtaggtcg tttttcgtcg attttcgggt cgggtttagt ttttcgcgcg 2280
   tttttatggg tttcgtcgtt cggcgtcgtt attttttagt ttcgaatttt ttcgggttta 2340
   gcgacgttat ttcgtaatag tttcgaatcg gcgtttaagc gatttcgggt gttgttgggc 2400
   gttgcgttcg cgggtcgttt tggggttcgg agtttttggt cgagcggagg gcggagcggt 2460
   cggggggcgg ggtcgcgggg cggggcgggg tcgggggtcg cggagattag ttcgggttat 2520
   tgcggatttt gagcgttttt gcgtcgggat ttgcggtgtt tagaggtcgt ttcgcgtgtt 2580
   cggttcgagg gcgggtatcg gtaagggagg gtttaggtta gggtttttcg ttagcgttcg 2640
   tttcggtttc gtagtttttt gcgtcggagc ggtttagagg tagaggtttg agttggagta 2700
   tacgttagga gggttaaggt tttcgggaga ggggtcgaga aggtagtagg gttttttcgt 2760
   cgttgaggtt tggtttcggg agtagtagat ggtcggcggg agtttaattc gaaacgtaga 2820
   aggggtgagt gtggcgtcga gggttgcgta tcggaatcgt aaggtgtcgg atcgatttta 2880
   gatgttaata ggtttttggg ttttattttt ggaattgcgg tttcggacgg tttagggagg 2940
   aagggtaatt tttgagagag aataggtttt gtgttttgga tacgtagtta agatttcggg 3000
   tcgtgagggg tcgagttggg gaagttttag ggtatatttt ttatttttag tttttagtat 3060
   ttcggtttta ttggaatacg gttttttatt tgatatttta gttttttgtt ttagtttata 3120
   taggtgagtt ttttagaatt tatttgtttt tgtaggtaga gggacgtatt tatttttgtt 3180
   tagagggttt tgggttagta ttagttttgg gtagttattc ggggaagaat atagtagtgg 3240
   gtggttttcg gtagtatagg atttggtaaa ggtaggggtg ttttggtagt gcgtagaaat 3300
   atataggaag ggttggggga ttaggggttg atgttatttt gtaggttttt ttttttgtgg 3360
   gttttatagt aatattttgt ttattttgtt tatggttttt ggattgtgag ttttaagggt 3420
   ttaggtttag aaggatgggt ataagaaatt atagtttttt ttttttagtt tttagtaggt 3480
   tcgatgtgtt cgttttgagt agtggtttat gtagttagtt ttttgttttt ttattttgta 3540
   tagtatttgt tatttaagaa gggttagggt aaggatacgc gggtgtaaag tcgagtgtgt 3600
   gcgggttgtg ttgggtgtag gtggttgagt gttagttgtt gtcgaagcga ttaggggtcg 3660
   tgtgtggtag gattggggaa ggggcggcgg taggttaagg aggcggttta ttttgttggt 3720
   atttggttag ttttttaata atgtttttat tattttttag cgttcgtggt gatggaatgg 3780
   tttttgggga gggtggttta ggcgggagat ataggtttgg gttttttgtt gtggggttta 3840
   gagatttggg tcgggttgtg tgtaagagtt gacgggtatt ggttatcggg ggaatttagt 3900
   attttttggt cggttttatc gttttttagt tgtgtttgcg atgttttttt tttttttagg 3960
   ggtttttatt ttaatcgttg tttcgtattt ttgtaggtta gcgttaggtt tttttcgtta 4020
   ttttgggatt tggaaattta ttttttgtag tttttatttt tgtgtttcgt ttgtagagcg 4080
   gcgggatttt ttttgtttgg tcgttggttt tgtacgtatt ttttttttcg cgtttttgtc 4140
   gtttttgtcg tttttgtttt tagtgttttg ttggggttta tttcgagttt cgtttaaggt 4200
   gtagacggcg gcggtttcgg gtttcgttcg gtcgcgttcg agtttcgttt ttagtagtat 4260
   cgcggttatt aggtcgagtg gcgcgagacg cgtttttttt aggttagcgt tttttggagg 4320
   gttcggggtt tttaagtttc gtcgcgtcgt gcggggtagg gagttcggga gttattgggt 4380
   ttggcgttgt tcgcggtgtt gaaggaggcg ttcgttgttc gtttcgttcg cgcgttcgtt 4440
   tatttttcgg ggtttttttc gtcgtttcgg tttttatttt cgtttttgtt tcgtgtcggg 4500
   cgcgtttttt tttttggttg ggttgggtcg tatttaaggt agttttcgtt tttatttttt 4560
   tgagatttag ggtggtcgtg ttcgtttttt tttaagtttt aggttttgtt gaggcgttgg 4620
   gattcgtcga gtttcgtagt aagcgggtcg tttagtcgta gggtaggagg atattgattt 4680
   tatttttttg gcgtgtagtt tttggaaggt agtgtttagg tcgtgtttcg gggtagtttt 4740
   atgcgtgatt aattttagtt tagggtttat tggggtattt ttttgtaggt gttttgttgg 4800
   ttagggaatt ttttattttt aatttttttg aatttaattt ttaatttttg ggaggagttt 4860
   agttcgtatt ttttcgggtt gttaggagga gaggtatttg tagttttttg taataaaagt 4920
   attgttgagg atttaagatt ttgggtatag gtagggtttg aaggaatcgt ttagtatata 4980
   tttaggttgg tgtttgttcg gtgtttgacg agtgattgag ttattgttag tttttgtata 5040
   gtttggttag ggaggtgtta atcggttttt tttatagtta taggtaagat tgttgattat 5100
   tgttttgttt ttattggtat agtaaaagta ggggaggggt agaagataat acggggttaa 5160
   gggtatttta tagaatatta taaatagttt tagtaggata tttatattta ggaagtagag 5220
   ggatggttat ttaagtaagt tgggggtttt ttgaggtgtt tttaggttgg gttgtcgcgc 5280
   gtgttttttg aagtgtgtag ttgatatgta ttttagttgt tttttaattt gtgtaaaatt 5340
   aagggttttg ttttgtgagt ttcgtttaga ttagttttag ggagatgatt tattattgtt 5400
   ttttattttt tattttttat tcgatttttg ggttgtgttt taaaagttaa tgattcgata 5460
   ggagaaattt taggatttta gatgtttagt acgtgaataa aagaggggtt tattttgtat 5520
   ttatcgtttt tattataatt tttattggta gagttggttt atattttaaa agaattatta 5580
   tttttttaaa tgttagattt ttttattaaa atgtgtatat tatagtttat ttaaatataa 5640
   aatgtttatt ttttttgtag gtaagaaatt ttattgatat ttttatgtta attagttttt 5700
   ttttaataaa aattttttta ttgaaaataa ataagtattt aaattattga attattatat 5760
   ttattagttt taatattttt taaaatattt aaaattttag aaaatagatt ttaaatattg 5820
   tttaaaggtg gtatttagtt ttgagtaggt tatataaggt gtgtttgagt atggttatat 5880
   gatttttggg gttattttta cgacggttta gttttatcga cgttttgttg aaaattttgt 5940
   tttttagtag gacgtaagtt tgttttttaa atagtggtga ttttaaattg taatatgatg 6000
   aaatttgtag ttaatattgt tttttataag ggtttttgga aaggttgaag ttggagacgg 6060
   taaattataa tatcgtttta ggttatttta ggttatttta gttaaagata tttaatatta 6120
   gttaaaaggt taaagtttag tttgaagggt ttaaaggtaa atatattgaa atttacgtgt 6180
   aaatttgttt ggtttttaaa ttggaagaga aatattttgg tgtttttaat aatttaggtt 6240
   tgtattggat gaagtaatga aggtaaggtt acggttgtta aagtatagag gggttaaaaa 6300
   gtgtgaaatt agaagtaatt ttcgagtgag atacgaagta gtaggttgac gtattagata 6360
   tcgtttttat tagatatttt tttcgttagt tgtagggttt tgggtagcgt ttttagttta 6420
   gttattttgt gatagttgtt gaaagaatta gttgttgttt ttttttttta agcgataata 6480
   attatattaa tattatatgg ttttatgaat atatttatgt tattaatttg ttagtaaata 6540
   tatgattata taatataaag taggtaaagc ggaaaaaata tttataatcg ttaaagtttt 6600
   tttttttttt atatatatat atatatatat atatatatat atatatatac gggtatataa 6660
   atatatttga gtttatttta gttaaattaa attttgtttg tgagatatat taatagttat 6720
   aaagaatttt aatataaatg tttttagaaa tagtgtattt gtttgtttat atttttagag 6780
   gtaacggatt attaaatgtt ttgggtttaa aaataatgtt taaaattatt aaatggtaaa 6840
   agatattatg ataaaagttt gggagattag tgtttgtgtg gagatttttt ttaaaatagt 6900
   tttaggtaaa ggaagtttgg tttttttcgt gggtcgagtg agatgtttgt gtgtgtagta 6960
   ggtgtttttg tggtgttggg tgaagagtag acgatagggg gggtttagaa agtttttttg 7020
   gggtttacgt ttttaagggt agattgtttt tttttttatt ttttaataga ggggtttgtg 7080
   ttttaagtat tgttcgttga gtttagagtt gttttttagg tattcgtcgt tttaacggtt 7140
   tttatttatt tgtttttttt ttgttattag atagatagat aaaggcgttg tttttgaggg 7200
   ttaaatgagt ttttattttt ttaattttta ggtttatagt ttaaggtttt tgggggttat 7260
   tttttgaaac gtagtatagt tatagaattt tttggttttt tttttgttga aatttttacg 7320
   tttggtgtcg aatattcgga ggaataagga gagtttttag gaatttgttt tagggaagta 7380
   gttataaata taggatagtt tttgga 7406

<210> 175
<211> 6249
<212> DNA
<213> Artificial Sequence

<220>
<223> chemically treated genomic DNA (Homo sapiens)

<400> 175

atttttgcgt ttttattttt gtaagtaagt attagtttgt gtgtatttgg gtttatatat 60
tttgtttttt ttttagttat tagttttttt tatattattg tattgttttt aattttgttt 120
tttagttttt tagtggatgt gtaaagtaga tttagttttt aggtttttat atttgttttt 180
ttttttgttt gggaggttgg gaatttattt tttagagtgt gtattgtgtt tttttttttt 240
tttttaggat tttttgtagt gaggtttttt ttgttttttt agaaatagtt atttttttta 300
tttttaattt tttttttttt gtttttttat tatttgtatt attatttagt aaatttgcgt 360
atttatttat ttatttattt attttttttt ttgagatgga gtttcgtttt gtcgtttagg 420
ttggagtgta gcggtacgat ttcggtttat tgtaattttt gtttttcggg tttaagtgat 480
ttttttgttt tagtttttta agtagcgggg attataggta tatgttatta tgttcggtta 540
attttttttt ttttttgtat ttttagtaga gatggagttt tattatgttg gttagattgg 600
ttttgaattt ttgatttcgt gatttattta tttcggtttt ttaaagtgtt gggattatag 660
gtgtgagtta ttacgtttgg tcgtgtattt atttattatt tgtttttttt aatgagggta 720
gggatttttt tttgtttgtt attatatttt ttgttagaat agtgttttat ttatttattt 780
atttaatagg tatttaatat gtttttgtta aatattaggt attgtttagg tgttgagatt 840
atagtaatga atatagaaga ttaaaaattt tgtttttttg gttaaataat acggagatat 900

ttgagtgaag atttgaggga gatgaaggag tgagttatgt ggatatttgt gggaagagtt 960
gtttagggag tgttatggtt tgaatgtatt ttttaaagtt tatatattgt aaatttaatt 1020
tttagcgtaa tagtgttgag aggtgagaat tttaagaggt gattatgtta tgaggattgt 1080
taaattaagt ttaggttaaa gttgttttta tatatatata tatacgtata tatatatatg 1140
tatatatgta tatatatata tatatatata tatatatata tatatatata tatttttttt 1200
tttttttttt tgagacggag ttttgttttt gttgtttagg ttggagtata atggtatgat 1260
attggtttat cgtaattttc gtttgtcggg tttaattgat ttttttgttt tagtttttcg 1320
agtagttggg attataggcg tgagttatta cgttcggttt atatatattt taagttaggt 1380
ttaaaggttt ttttgtatat agtaaattgt aatttaattg gatatgtaaa tagattgtag 1440
ttaatttttg tattaattat agagttttgg ttaattatag gtagttaatt gtttaaattg 1500
tgttaaaata aagtaaatat ttagttttaa ttaatttagt tttttttgta ttttatttta 1560
ttttttgtta tttttttttt tttgtttata aatattattt gattttgtgg tagttttaga 1620
gttgttttga aattattttg gttttggggg ttgtttagtt tttaaattgt tttttgttta 1680
attaaatttt gttaaattta atttgtttaa agtttttttt ttaataggga tttgttttta 1740
tgaatggatt aatggtatta tgggagtgcg gtttggtatt ttaggagggg attgttgata 1800
taagtatgag tttggttttt ttttattttt ttttttatgt tttttttttt gttatgtgat 1860
gttttttgtt atgttgtgat gaaggaagaa ggtttttatt agatgtagtt ttttgatttt 1920
ggatttttta gtttttagaa ttatgagtta aattaatttt tattgtgtat aaattgttta 1980
gttggtggtt gtattggttt atttttatat tgttatgaag aaatatttaa gattgggtaa 2040
tttataaaga aaacgtggtt tagggttggg tacggtggtt tacgtttgta attttagtat 2100
tttgagaggt taaggtaggt ggattatttg agtttaggag tttgagatta gtttggttaa 2160
tattataaaa tttcgttttt attaaaaaat ataaaaaaag attagttagg cgtggtggtg 2220
tatgtttgta gttttagtta tttgggaggt taaggtagga gaattgtttg aattttggag 2280
gtagaggttg tagtgagttg aggttgtatt attgtatttt agtttgggtg atagagtgag 2340
attttgtttt aaaaaaaaaa ggtttaatgg atttatagtt ttatacggtt ggggaagttt 2400
tataattatg gtagaaggtg aaggaggagt aaaggtatat tttacgtggt agtaggtaag 2460
agggcgtgtg tagggaaatt gttttttata aaatcgttag atttttatga gatttattta 2520
taattacgag aatagtatgg gaagaattta tttttatgat tcgattattt tttattaggt 2580
tttttttatg atatatggag gttatgggag ttataattta agatgagatt tgggtgggga 2640
tatatagtta aattttatta gtggtatttt attatagtaa tataaaatga atttagaaag 2700
ggagggaata gttagtatat agatattgag gtgagaatgt gtttagattg ttttagaaga 2760
gtgaggatgt tagtgtgatt tggggaagtg agtaagagga agagtagaag tgtaggttag 2820
agtgttaaag gaggggaagg aaagattatg tagagttttg tgtttattat gggaattttt 2880
attttgagtg aaatgaggat ttaagggagg attttttaaa aatttaataa aatgatatta 2940
atatatagtt tatatttagg tttttataat ggttttatta atgttttttt aaaaagaata 3000
tttaaatttt tattattatt taaggaaata atttgtataa tgggtaaaat taaattttat 3060
tgtatatata ataatattaa tagttattat ttaagattat taggattgta ttaagaaagg 3120
ttatttaggt tgggcgcggt gggttatgtt tgtaatttta gaattttgag aggttgagac 3180
gggtggatta tttgaggtta ggagtttgag attagtttgg ttaaaatggt gaaatttcgt 3240
ttttatttag atgggcgtgg tggtgtgtat ttgtagtttt agttattcga gaggttgagg 3300
taagagaatt gtttgaattc gggaggtaga ggttgtagta agtcgagatc gtgttattgt 3360
attttagttt gggcgatcga gcgagatttc gttttaaaat taaataaatg ttatttttga 3420
tttttttaaa aattaacgat tttaaaatga tagtttatag tttttttttt ttatttagga 3480
tttaattaag gttatatatt gtattttatt gttatgtttt ttttttaata gtttgaggta 3540
aaatttatat attaaatgat tgatttattt aaagtgtgta atttaatggt ttttggtata 3600
tgtatagagt tgtgtaatta ttattagaaa ttagttttag aataattttg ttattttata 3660
atttttagtt gttatttttt aggtttttta tttttttaat tttatgtaaa ttattaattt 3720
attttttgtt tttatagttt tgtttatttt gagtatttta tatatgtgga agaatataat 3780
gtgtggtttg tttttttttt ttttacgttt tgaaaggtta tttatgtagt atgtattagt 3840
attagtattt tatttttttt tatggttgaa taatgttttt ttgtatggga tattttatat 3900
tatttattaa gggagggttt ttaatagagg agtggtttga ttcgagtggt atttaataag 3960
gtttattttt ttgttgtttt aaggagtaga gaatgattaa ttgcgttaaa cgttgttttt 4020
agggaaaata agatgaggtt tacgaattga ttagtaagtg tttaaatagg ttaagggaaa 4080
aaggaattta attagaagat atattttgat atatattgtt gggagtgatt tttttttttt 4140
tttttttgtt ttatgtgaaa tttatttttt aaaaaaaaat tatttttttt aaacgtaatt 4200
taatattata gtaattatgt aagattttat gtagttttaa gagttagtta agagaaaagt 4260
tgatttttgt aagaagatat ttttaatgtt tatgtagatt aggtaggtaa gagttaagtg 4320
cgggggagtt ggtattattt aaaagattgt agaaattgga ttaaaaatgt ggagttttaa 4380
agtagtattt ttttatggta tgttattatt tggagtaata aggttattta ttcgaattat 4440
tggattttga gttgatataa aacgtatttg ttgatttagg gtttgtataa ttacgtgtag 4500
tatttattag gttatttaat tttgggtacg tataattttt ggggtttgta aattattgga 4560
gagtgagtga ggggtaacga aagatagata taaaagggcg cgtttcgaaa ggtaattgtt 4620
aatatgtatt ttacgtttat agaacgattt agtgggcgcg gtggaagcgt taatgaatat 4680
tttagaaaat aaatatatgg aatatcgaaa tttatttgta gataatggat taagttcgtt 4740
ttatcgggat taaaaaggta gggttaggaa agtttgaacg tcgttatatt gttttttttt 4800
taggaagaaa cggggcgtgg ggttgtcggt agaagggggt ggtttcggtc gttttttcgc 4860

gtcgtttttc ggtttcggtc gcgttgcgtt tacgtcggtc gggtcgggaa tttggagagg 4920
atttttggtc gcgcggtagc ggcggcggcg cgcgggtgag cgggtgagcg tgtgagcggg 4980
cgggcgggta ggcgagtgcg tcgggtattg gtagttgagg agtggaggtt gggtagtttc 5040
gattttttga cgttagcgcg attagattaa tttaggtttt aggagaaagt aggcgggcgg 5100
gcggagaaag gagaggtcga gcggtttaat tcgggtcgag gttcggggag cggagagtgg 5160
cgtagcgttc ggtcgttcgg attcgggtcg cgagatttcg ttcgttcggt tattcgtgtt 5220
tttatacgga cgggcgcgtc gttaattcgg taaaacgaaa tagattcgaa tcgaattttt 5280
tttttatgtc gcgttttagt ttttttttcg gcgtttcggg ttttttagcg tcgtcgtcgt 5340
cgtttttgta cgcgtggggt cgtcgcggtc gtgttgggtt gggggcgcgg gtagcggcgg 5400
gaagggggtt ttttttgcgc ggggcgggtc gcgatttttt ttttttatga tggcgttttt 5460
cgtttgttcg gaggagcgaa tttgttatta cgttttttcg cgtttagttc gtcgggtttt 5520
tttggtgttt ttgttgggtt ttttgttgtg ggaagtttag ttttcgggtt ttcgagtttc 5580
ggttttcggg tttgatcgcg ttgggatttt ttcggtcgcg tttttttttc gcgcgttttt 5640
tatatttttt tcgtgttgtc gtcgggtcga ggttcgttcg cgtggttcgc ggatttattg 5700
tgtttttcgc gtcggcgggg cgatttttgc gggagttgga ggacgatcgt tggcgttgtt 5760
ttttaaggcg tttggtggag cgggtttcgc gggcggggga tttcggcgtt tcgggttttt 5820
ttatatttcg tacgggtttt tttttcggtt ttagtaagtt tttttggggt cgaggttaag 5880
gaaagttcgt atcgagattt tttttaattt atttaaaggt ttggcggcgg cgcgtaattt 5940
tttttttttt tttcgtttat attcgttgcg ttttttggtg tttcgttttt ttttttttat 6000
cggagtcgat tgttttattg tatgtgtatt cgtgagttcg cggttgaata attgtttttt 6060
tattttgttt tttgtttttg ttagtgtttt tcggggttgt ttttgtagga aggtgggggt 6120
ggtgggcgtg agagatagat gtgggtttgt ttttttagtt gttgaaattg tatgaaggtt 6180
ttaaagggag aacgtttttt tgatgtgttt taggagggga ggaggaataa atgtttgtta 6240
gattttata 6249

<210> 176
<211> 6249
<212> DNA
<213> Artificial Sequence

<220>
<223> chemically treated genomic DNA (Homo sapiens)

<400> 176

tgtgagattt ggtaggtatt tgtttttttt ttttttttaa agtatattaa gaaaacgttt 60
tttttttaaa gtttttatat agttttagta attagaaaaa taagtttata tttgtttttt 120
acgtttatta tttttatttt tttatagaaa taatttcgag aaatattaat aaagataggg 180
agtagagtaa aggaatagtt gtttaatcgc gaatttacga atatatatgt agtgaggtaa 240
tcgatttcgg taaagggaaa ggaacgagat attaggagac gtagcgggtg taggcggaag 300
agggggaaaa aattacgcgt cgtcgttaaa tttttgaata aattagaggg gatttcggtg 360
cgaatttttt ttgatttcga ttttaaagag gtttgttggg gtcgagaaga aaattcgtgc 420
gggatgtgga ggggttcggg gcgtcggggt ttttcgttcg cgagattcgt tttattaggc 480
gttttggaga gtagcgttag cggtcgtttt ttagttttcg taggggtcgt ttcgtcggcg 540
cgggggatat aatgggttcg cgggttacgc gaacgggttt cggttcggcg gtagtacggg 600
agagatgtga ggagcgcgcg gaaggggagc gcggtcgggg agattttagc gcggttaggt 660
tcgggagtcg aggttcgggg gttcgggaat tgggtttttt atagtagaag atttaataaa 720
gatattaggg gagttcggcg ggttgggcgc gagaagacgt ggtagtaggt tcgttttttc 780
gagtagacgg gaggcgttat tatggggggg gggggtcgcg gttcgtttcg cgtagaaaga 840
gttttttttt cgtcgttgtt cgcgttttta gtttagtacg atcgcggcgg ttttacgcgt 900
gtagagacgg cggcggcggc gttggaaagt tcggggcgtc gaggggaggg ttgggacgcg 960
gtatgaaggg aaagttcggt tcgggtttgt ttcgttttat cgggttggcg gcgcgttcgt 1020
tcgtgtggga gtacgagtgg tcgggcgagc ggggtttcgc ggttcgggtt cggacggtcg 1080
ggcgttgcgt tatttttcgt ttttcgagtt tcggttcggg ttgagtcgtt cggttttttt 1140
ttttttcgtt cgttcgtttg tttttttttg gagtttggat tgatttggtc gcgttggcgt 1200
tagggagtcg gagttgttta gtttttattt tttagttgtt aatattcggc gtattcgttt 1260
gttcgttcgt tcgtttatac gtttattcgt ttattcgcgc gtcgtcgtcg ttgtcgcgcg 1320
attagggatt ttttttaaat tttcggttcg gtcggcgtga gcgtagcgcg gtcggggtcg 1380
gaggacggcg cgggggagcg gtcggagtta ttttttttta tcggtagttt tacgtttcgt 1440
ttttttttgg agaaaaaata gtgtggcggc gtttaggttt ttttaatttt gtttttttag 1500
tttcggtgga gcggatttag tttattattt gtaagtgaat ttcgatgttt tatatgttta 1560
ttttttgaaa tgtttattaa cgtttttatc gcgtttattg aatcgtttta taaacgtgga 1620
gtgtatgttg atagttattt ttcgagacgc gtttttttat gtttattttt cgttgttttt 1680
tatttatttt ttaataattt gtaggtttta gaagttgtac gtatttagaa ttggatagtt 1740
tagtaaatat tgtacgtggt tatataaatt ttgagttaat agatgcgttt tgtgttaatt 1800
taggatttag tgattcgagt aggtaatttt gttgttttaa ataatggtat gttataagga 1860

gatattgttt tagaatttta tatttttggt ttaattttta tagtttttta gatgatgtta 1920
attttttcgt atttaatttt tatttatttg atttgtatag atattgaaaa tatttttttg 1980
taagagttaa tttttttttt ggttaatttt tgaagttgta taaaattttg tataattatt 2040
ataatgttga gttacgttta gaaaaaatga tttttttttg aggaataaat tttatatgaa 2100
gtaaaaaaaa aaaaaaaaaa attattttta gtaatgtata ttagaatatg ttttttaatt 2160
aaattttttt tttttttaat ttatttgagt atttattggt taattcgtgg gttttatttt 2220
atttttttta aaagtagcgt ttgacgtagt tgattatttt ttgtttttta gaataatagg 2280
aaagtgaatt ttgttaaatg ttattcggat taggttattt ttttgttgaa aatttttttt 2340
tgataaatag tataaagtat tttatataag agaatattat ttagttataa aaaggaatga 2400
agtattgata ttgatgtatg ttatatggat aattttttaa aacgtgaagg gaaagaagat 2460
agattatata ttgtattttt ttatatatgt gaaatgttta gagtaggtaa ggttatggag 2520
atagaaagta gattggtggt ttgtataggg ttggggaaat gaggaatttg gggagtgata 2580
gttaaaggtt atggggtgat aaaattgttt taaaattagt ttttggtgat ggttgtataa 2640
ttttgtgtat atattaaaag ttattaaatt gtatatttta aatgggttaa ttatttggta 2700
tgtgaatttt attttaagtt attaaaaaaa aagtatgata atgaaatgta gtatatgatt 2760
ttgattggat tttgaatggg gaggaggagt tataaattgt tattttaaag tcgttaattt 2820
ttaaaaaaat taaaaatggt atttatttaa ttttgagacg gagtttcgtt cggtcgttta 2880
ggttggagtg taatggtacg atttcggttt attgtaattt ttgtttttcg ggtttaagta 2940
atttttttgt tttaattttt cgagtagttg ggattataga tatatattat tacgtttatt 3000
taagtagaga cggggtttta ttattttggt taggttggtt ttaaattttt ggttttaagt 3060
gatttattcg ttttagtttt ttaaagtttt gggattatag gtatgattta tcgcgtttag 3120
tttagatggt tttttttagt gtagttttag tagttttaaa tgataattat taatattatt 3180
atgtatgtaa taaaatttga ttttgtttat tgtgtaaatt atttttttaa atgataataa 3240
aaatttggat atttttttta aaaggatatt gatgagatta ttataaaaat ttaaatatgg 3300
attgtatatt aatattattt tattaaattt ttaaaaaatt tttttttggg tttttatttt 3360
atttagaata aaagttttta taataagtat aagattttat ataatttttt tttttttttt 3420
tttagtattt tgatttgtat ttttattttt ttttttgttt atttttttaa gttatattgg 3480
tatttttatt tttttggaat aatttgggta tatttttatt ttagtattta tgtgttagtt 3540
gttttttttt tttttgagtt tattttgtgt tgttgtaata gaatattatt gatagagttt 3600
ggttatgtat ttttatttaa attttatttt gaattgtagt ttttataatt tttatgtgtt 3660
atagaaggga tttggtggga ggtaatcgaa ttatgggggt gggttttttt tatgttgttt 3720
tcgtgattgt gaataagttt tatgagaatt tgacggtttt ataaagggta gtttttttgt 3780
atacgttttt ttgtttgttg ttacgtaaga tgtgtttttg tttttttttt attttttgtt 3840
atgattgtga ggttttttta gtcgtgtaaa attgtgagtt tattaaattt tttttttttg 3900
agatagaatt ttattttgtt atttaggttg gaatgtagtg atgtaatttt agtttattgt 3960
aatttttgtt tttagggttt aagtaatttt tttgttttgg ttttttaagt agttaggatt 4020
ataggtatgt attattacgt ttggttaatt tttttttgta ttttttagta gagacggggt 4080
tttgtggtgt tagttaggtt ggttttaaat ttttgagttt aggtgattta tttgttttgg 4140
ttttttaaag tgttgggatt ataggcgtga gttatcgtat ttagttttaa attacgtttt 4200
ttttataaat tatttagttt tgggtatttt tttatagtag tatgaaaatg gattaatata 4260
attattagtt gggtagttta tatataataa aagttaattt agtttatggt tttgggggtt 4320
gagaaattta aaattaaggg gttgtatttg gtgagggttt ttttttttta ttataatata 4380
gtagaaggta ttatatagta agagagagaa tatgagagga agggtagaaa agggttaaat 4440
ttatgtttat attagtaatt tttttttgag atattaaatc gtatttttat aatgttatta 4500
atttatttat gaggatagat ttttgttaaa agaaaaattt taggtaaatt aaatttaata 4560
gagtttaatt gagtaaagag taatttgaga attgagtagt ttttagaatt agaatagttt 4620
tagagtaatt ttggggttgt tatagggtta gatgatgttt atgggtagag aaagaaaagt 4680
gatagaaaat ggagtgaggt atagaaaaaa ttggattggt tagagttaaa tatttgtttt 4740
attttaatat agtttgaata gttggttgtt tgtgattgat taaaattttg tgattggtat 4800
aagagttggt tatagtttgt ttgtatattt agttaggtta tagtttgttg tgtatagaga 4860
aatttttagg tttaatttaa aatatgtatg ggtcgggcgt ggtagtttac gtttgtaatt 4920
ttagttattc gggagattga ggtaggagaa ttagttgaat tcggtaagcg gaggttgcgg 4980
tgagttaata ttatgttatt gtattttagt ttgggtaata agagtaaaat ttcgttttaa 5040
aaaaaaaaaa aaaaaaatat atatatatat atatatatgt gtgtgtatat atatgtatat 5100
atatatgtat atatgtatat atacgtatat atatatatat ggaggtagtt ttagtttaaa 5160
tttaatttaa tagtttttat gatataatta ttttttgaag tttttatttt ttaatattgt 5220
tgcgttgggg attaagttta taatatataa attttgggga atgtatttaa attatagtat 5280
tttttggata gtttttttta tagatgttta tatggtttat ttttttattt tttttaagtt 5340
tttatttaaa tgttttcgta ttatttagtt aaaagagtag gatttttggt tttttgtgtt 5400
tattgttata attttagtat ttgagtagtg tttggtattt aataggagta tattaaatat 5460
ttgttgaatg aatgagtaag tgaagtattg ttttagtagg ggatatggtg gtaagtagaa 5520
aaaaattttt gtttttatta aggaagatag ataataaata aatatacggt tagacgtggt 5580
ggtttatatt tgtaatttta gtattttggg agatcgaggt ggatggatta cgaggttagg 5640
agtttaaaat tagtttggtt agtatggtga aattttattt ttattaaaaa tataaaaaaa 5700
aaaaaaaatt agtcgggtat ggtggtatgt gtttgtagtt ttcgttattt gggaggttga 5760
ggtaggagaa ttatttgaat tcgggaggta gaggttgtag tgagtcgaga tcgtgtcgtt 5820

gtattttagt ttgggcgata gagcgagatt ttattttaaa aaaaaaaata aataaataaa 5880
taaataagtg cgtagatttg ttaggtagtg gtataagtga tggaaaaata ggggaagagg 5940
gattgagaat gggagagatg attattttta aaggggtaga ggaaatttta ttgtaagagg 6000
ttttgaagga agggaaggaa gtatagtata tattttgagg gatgagtttt taatttttta 6060
agtaaaagga agagtaagtg taaagattta gaagttgagt ttattttata tatttattga 6120
aaagttgggg ggtagggttg gggatagtgt agtgatgtag gaaaagttag tgattggagg 6180
gaaggtagag tgtgtggatt tagatgtata taagttggtg tttgtttgta gaggtgagaa 6240
cgtagggat 6249

<210> 177
<211> 6191
<212> DNA
<213> Artificial Sequence

<220>
<223> chemically treated genomic DNA (Homo sapiens)

<400> 177

atttaggtat ttagaagata acgatttgtt agtttttgaa atgaaggttt tgtagagttt 60
tattttagga atagggataa attagaggta gtttttttta tagtaaaatt gttatttagt 120
agtgttttat gattttggta ttatagagga agtagtaaaa gtatatgtat gaaaattgtg 180
ttaagtgaag gatgaataag aaaatattta gggtaattat taaaagaaaa gaatgtaata 240
aatgaatata gaaaaaaatt ggatatagaa tatgtgataa atttagaaga agataagaaa 300
gaaaatataa aataagtagg taattaaatt agtaattatg ttatatggaa atgggttaaa 360
tattttaagt aaatggtaaa aattattaga ttggataaat aataattatt taaatataag 420
tatttagaaa gattgagaga aaatgatgcg aaaaggtata ttatttaaat gttaattgaa 480
ggaaataagt gtggttatat taattaggta aaggagattt taatataata aatagtagta 540
gaaaaagaga attatatttt atagtgtaag gggattgatt tattagagtt attataattt 600
tttatgtgta tttagtaaaa tagtttaaat atttataaat taaaaatttt attttgaatt 660
aaaagaaata gataaatatg ttattataat ggatgtatat atataggata tatatatatt 720
ataattagta ggtttgattt aattgttata tgtagaatat tgaaattatt aatggtggta 780
ttaattattt ttttttttgt atatggaaat tgtattagaa ttgattataa gttagggtta 840
tgaataaagt tttagtaaat attattgatt ttaataattg aatatgttgt ttgtattata 900
gtaaataaaa ataattagaa aatttttagt tgtttagaaa taaaatatta tgtttttgaa 960
taatatatga tttaaaataa gaatttatga tggttattag aaaatatttt aaattgattg 1020
aaaatgaagg tgaattataa tgatttagat tgttgttgtt gaaatgttaa gatgaaattt 1080
atagttttaa gtttatgtat tggaaaagaa agttttttta aggaggtaga aaaggaatag 1140
gaaattaaat ttaaagatag tagaagtaaa gaaataatga agataagagt agaaattagt 1200
gaatagaaag taattttata tagagaaaat tgataaagtt aagaaaaagt tggttttttg 1260
aaaagattaa tgtaattgat tatttagtaa aattgataaa aggaggatgg ggagggggag 1320
agagaatata aatttaaata ataggaattg aaaaatgtta tattattacg gattttatag 1380
tcgttaaata gaggtattta tgaatagttt atggagaata tgggataggt agagtaggta 1440
gaaattttga gtagttaggg ttggattatt taatatgggt aggtagtatt tttgtgtagt 1500
tattgagtat ttgaaatgtg gttagtttta gttgagttgt tttgtaagtg taaagtattt 1560
aatagatttt gtagatttag tatgaaaatt ttttttatat tgattgtagg ttgaaatgat 1620
ggtattttgg atatattggg ttaaatatat tattaaaatg ttgtttattt tttttttttt 1680
tttaaaatgt tattagagaa tttaaagtat atatgtagtt tatattatat ttttatagag 1740
gttgttatat tggttttaag ataggtattg ttatttttat tttatagttg agaaaattgg 1800
ggtgtataga ggttgttatg tagttagtaa gtggtatttt tgggatttga atttggttaa 1860
ttggttttag aatttgtttt ttgaagtatt atattgttta tgggttttta aaggatatta 1920
agttatttta ttgttatttt ttgtaaatat tgaaggattt ttagtagtag ggtgatatgt 1980
agtatgtatt ttagaaagtt gtttgtatta aagaaggtag aaggtggtga ttgaaagatt 2040
agttaaatga tttgtagatt gagaaaggag ttggagttaa atgtgttatt ttaattttat 2100
ttaatggatt aatttgttag gtatagatag ttttgttgat tgtataaaga gtttagtttt 2160
taatggtttt ttttaggttt ttagattagt tttagattgt tatgtgtaat ataatgtatt 2220
gatataatta agtatttgtt ttttggtatt atgttgtttg atataaattt tagtgttggt 2280
atttaaaagt tgtgttatta tgttgtgttg ttataaattg ttttttgtat ttatgatatt 2340
ttttttttta attatttatt ttttttttat atttgataat ttttttatat gtatttttta 2400
gaattttttt tttattgaat ttggcgtttt ttgagttttt ttatatatta gtattgcgtt 2460
ttattattat atgatagttt gatttttttg ttaagtttta aatttttgtt tttagttgtt 2520
tatcgggtat ttttttttgg aattttatag ttatagtaaa tttagtttga ttaatataat 2580
atttatgttt tttaaatatt tcgtattttg gcgaattgta tttttattta attggttttt 2640
taagtaagaa tttgtaattt gtaatttttt tttaattaat tttgtttttt ttatgttttt 2700
aatagttatt tttttttttg tgtattgtaa ttttttgttg gttgattttt ttgtttttaa 2760
aaatttattt tttttttatt ttttattgtt aatattttgt tataaaggat tttttttgtt 2820

ttattcgttt tagtttaata gtgtagtttg tagatgaata atatagattg gatttgagaa 2880
taaataggta aatataggta taatagatag gtaaaaaaaa aaaattgttg aatgtaaaaa 2940
ggaatgaata aatggtaagt tgttaaggaa gtgtaaatat ttttttagga aaattttttt 3000
tgacgtttaa agtaaaatta gatggtgttt ttttgtgtat ttgtaatttt ttttgtaatt 3060
ttttttaatt gttatgtgta ttttaattat ggtagttttt tttattagtt attgattatt 3120
ttgagaagag ggaatattta aaaaaaaaaa ttattttttt atgtggtgta aattaaaata 3180
atggaattat ggagaggtta gagtaggaat gtagtttgtg tatattggaa atgaatttta 3240
gtatataatt tatgaaaatt agttatgttt tttgatggga aataaatttg tattttgtgt 3300
taggattttt tttaacgtta agaatatgtg taaagtgtgt ttaaaaattg tttttttttt 3360
ttaaaaatgt tttatttagt tttttgtata tatatatata aaaatatata taatggttat 3420
attatttagt tttttttatt tttttatttt tttttttgta tattggtata tttgtaattt 3480
atattaatat tttagatttt tgtttatata cgtataattt agttattagt tgtaatacgt 3540
ggtatattgt tagattattt agaataaagt tgttatattt gattttttta ttagtaatgt 3600
ggaatgataa taattttttt atattttttt tagtagtagg tattttaatt aatttaattt 3660
ttgttagtta gattgggtat gaaatgatgt tttattatta ttttaatttg tatttttttt 3720
tttacgttta aaaattattg tttgattatt atgataatta aggaataaat taatgaaaat 3780
gtaagtatta ttgttttttt aaagatatat tttgaaaatt ataattttag tatttggggt 3840
gtgtgatgta aagattatag atttttttat tttttaaaaa ggatgttttt tgggaatttt 3900
attaaatatg gtaaattgaa tatatttatt tttttttttt ttatatttta ttaaaattga 3960
aggtaaagga aagaaaagcg taagagttaa gatattaaga gtttttggag cgaaataatt 4020
tttaaatttt aattaaagga ttaggaataa aagttatgtg ggatttatta gtattagtag 4080
tggagtttag aaaattgagg ggatagtgtt ttttaaattt tgtgtgaatt gataaggaat 4140
gatttatagg gaattaagta aaaaaataaa aataaaaaaa taattttaag atatataaaa 4200
taaggaatat tatgttattt ttgtatagta ataaggtggg aatgtggtta gttgtatgta 4260
tatatgttta tatttgtaaa atgacgttga atagataaaa tagtttaata aaaatgattt 4320
ttatggaaga gaaaagataa ggtataattt tttaaattgg gaagagaaat tatttgattt 4380
tatttattat gtaaattgtt atttagatga aaatgaaaaa aatatgtgtt aaaaaatttt 4440
ttttaagagt aggtgaaatc ggattatagt ttttttttac ggattttgtt tgttatgttt 4500
atttttttat ttttgattat tttatatttt acgtattttt tttttttgta attgtagatt 4560
ttgtttttta ttagttatga aagtttaaaa ttcgggggag agtgggttta taaattttta 4620
atgtattttt gtatagtttt ttgagattag agtgaagttt taaaaagtaa gagaattttt 4680
tgtgaaaata ttttaattga tattattaag agtttaaaat agtattggtg gtgaaaaatc 4740
gttaaaagta tttgatttta tttgtaaaat tattgtattt ttttataaga ggagttttta 4800
taatattttt gtttttagaa gggtttgttt ttattattta tttaaattta taaagataac 4860
gtttttatgg gtggagaagt ttattgggaa agttataatt cgaattttta ttttgttttg 4920
tagtttgttt tttatgataa gttgaaatta ttatttgatg tttaaggtag tttttatatt 4980
atttttttaa tatttttgtt aggttcgata taagaggttg tttttttagc gtggtttgtt 5040
gtttttggta agaatatgtc gtttattttg ttatttacgt cgttagttgt gaagagattg 5100
ttgggatgga agaagttagt tggtgggttt ggaggagtag gcggaggaga gtagaatggg 5160
taggaagaaa agtggtgtga gaaagtagtg aaaagtttgg tgaagaagtt aaagaaaata 5220
ggacgattag atgagtttga gaaagttatt attatttaaa attgtaatat taaatgtgtt 5280
attatattaa ggtaagtttt gttagatttt aggtttgatt aattatgtta aggaattttt 5340
attaaaaaaa aattttttaa aaaaaggggt tttggcgaat tttttttttt tttttttttt 5400
ttttttgaaa tagttttatt ttattgttta ggttggagtg tagtggtgtg attttggttt 5460
attgtaattt ttgttttttg ggtttaagtg attttcgtgt tttagatttt cgagtagttg 5520
ggattagagt gggttattat atttggttaa tttttgtatt tttagtagag ataaggtttt 5580
attatgttag tcgggttggt tttaaatttt taattttaag tgattttttt atttagtttt 5640
tttaaagtgt tgggattata ggtgtgagtt attatattta gttttttttg gtatttttta 5700
ataggttata aaatgttatt ggagatatag ttgttttatt agtttagtga aatatttttt 5760
atattgataa taattataat aataggaagg ttgaattttt gttaggtaaa ttttaggttt 5820
ttaataatta ttaataatta ataataaaaa gtttagaaga attttgtaaa ttattggaga 5880
gtttattgag agataagagt attagagggg tggaggaatt tttgaaataa agttatgatt 5940
taaaaagtag tagagaaata tatgtgaatg tttatagtag tattatttat aagagataaa 6000
aatggaaata agtaaatgtt taatatggta tatttatatt atagaatatt atttagttat 6060
aagaagaaat gaaatattga tatatgttat aatatagttg gattttgaaa atatgtttag 6120
tgaatgaagt taggttataa aaaattatat attatatgat tttattttta ggaaatattt 6180
agaataggta a 6191

<210> 178
<211> 6191
<212> DNA
<213> Artificial Sequence

<220>
<223> chemically treated genomic DNA (Homo sapiens)

<400> 178

ttgtttattt tgaatgtttt ttagaaatgg aattatatag tatgtggttt tttgtgattt 60
ggttttattt attaagtatg tttttaaggt ttagttatgt tatagtatgt attagtattt 120
tatttttttt tatagttgaa taatatttta tgatatggat atattatatt gggtatttgt 180
ttgtttttat ttttgttttt tatgaataat gttgttgtaa atatttatat atattttttt 240
attatttttt aaattataat tttgttttaa aaattttttt atttttttga tgtttttgtt 300
ttttagtagg ttttttagta atttataagg tttttttagg ttttttatta ttgattatta 360
atggttattg aaggtttaaa gtttatttgg taaagattta gtttttttgt tattgtaatt 420
attattaata tagaaaatat tttattgaat taatgaaata attgtatttt taatgatatt 480
ttgtgatttg ttaaaagatg ttaaggaagg ttgggtgtgg tggtttatat ttgtaatttt 540
agtattttgg gaggttaagg tgggagaatt atttgaggtt aggagtttga gattagttcg 600
gttaatatag tgaaattttg tttttattaa aaatataaaa attagttagg tatggtggtt 660
tattttaatt ttagttattc gggagtttaa gatacgagaa ttatttgaat ttaggaggta 720
gaggttgtag taagttaaga ttatattatt gtattttagt ttgggtaatg gagtgagatt 780
gttttaaaaa aaaaaaaaaa aaaaaaaaaa aattcgttag ggtttttttt tttgggaaat 840
ttttttttgg taagggtttt ttgatataat tgattaaatt tgggatttaa taaaatttat 900
tttggtatgg taatatattt agtattatag ttttgagtgg tgatggtttt tttaagttta 960
tttaatcgtt ttgttttttt tagttttttt attagatttt ttattgtttt tttatattat 1020
tttttttttt gtttattttg ttttttttcg tttgtttttt tagatttatt agttgatttt 1080
ttttatttta gtagtttttt tataattggc ggcgtgaatg gtaagatgga cgatatgttt 1140
ttattaaagg tagtaagtta cgttaggaaa atagtttttt gtatcgaatt tagtagaaat 1200
attgaaagga tgatgtagag attattttga atattaagta ataattttaa tttattatag 1260
aaagtaaatt gtaaagtaag atagaaattc gaattatgat ttttttaata gattttttta 1320
tttatgaaaa cgttattttt atgaatttaa atgaatgatg gaaataaatt tttttaaaaa 1380
taagaatgtt gtaaggattt tttttgtggg aaaatatagt aattttgtaa gtaaaattag 1440
atatttttaa cgatttttta ttattaatat tattttaaat ttttagtagt gttaattaaa 1500
atatttttat aaaaggtttt tttatttttt agaattttat tttaatttta aaagattata 1560
taaaaatata ttgaaaattt gtaagtttat tttttttcgg gttttaaatt tttataatta 1620
ataggaggta aaatttatag ttataagaag ggaaaatgcg taaagtgtga agtaattaag 1680
aataaagaaa taaatatggt aaatagagtt cgtggagaaa agttgtagtt cggttttatt 1740
tgtttttgag agaaattttt taatatatgt tttttttatt tttatttaaa taataatttg 1800
tatagtgagt aaagttagat agtttttttt tttaatttaa ggaattgtat tttgtttttt 1860
tttttttata ggaattattt ttattaagtt gttttgttta tttagcgtta ttttataaat 1920
ataaatatgt atatatataa ttaattatat ttttatttta ttgttgtata aaagtagtat 1980
aatatttttt attttgtata ttttgaaatt atttttttat ttttattttt ttgtttaatt 2040
ttttgtaggt tattttttat tagtttatat agaatttaga aagtattgtt tttttaattt 2100
tttagatttt attattgata ttggtaagtt ttatataatt tttatttttg attttttaat 2160
tggggtttaa aaattgtttc gttttagaag tttttaatat tttagttttt acgttttttt 2220
tttttttgtt tttaatttta gtgagatata ggaaaaagga aaatgaatgt gtttaatttg 2280
ttatatttaa taaagttttt aaaaggtatt ttttttagaa agtaaagagg tttgtagttt 2340
ttatattata tattttaaat attgaaattg tagtttttaa agtgtatttt taagaaaata 2400
atgatgttta tatttttatt agtttgtttt ttaattatta taatgattag ataatagttt 2460
ttaaacgtag aggaggaagt ataagttaaa ataataataa ggtattattt tatgtttaat 2520
ttagttggta aaaattgaat taattaaaat atttattgtt ggagaagatg tgaagaaatt 2580
gttattattt tatattgttg gtgaaaaggt taaatgtgat agttttattt taagtaattt 2640
ggtaatgtgt tacgtgttat aattagtaat tggattatac gtgtataaat aaaaatttag 2700
gatattaata tgggttgtag atatattaat atgtagaaaa gggggtgaaa agatgaaaaa 2760
ggttgaatga tatggttatt gtgtatgttt ttgtgtatgt gtgtataaaa aattggataa 2820
aatattttta aaggaaaaaa atagttttta agtatatttt gtatatattt ttaacgttaa 2880
agaagatttt aatataagat gtaaatttat tttttattaa agaatatggt taatttttat 2940
agattatatg ttaagattta tttttaatgt atataaattg tatttttgtt ttaatttttt 3000
tataatttta ttattttggt ttgtattata tggaagaata attttttttt ttaaatattt 3060
ttttttttta ggatggttaa tgattagtga agaagattgt tatgattaaa atatatatga 3120
taattaagag aaattgtaag ggggattata ggtatataaa gaaatattat ttgattttgt 3180
tttaggcgtt aggaaagatt tttttgagga aatatttgta tttttttggt agtttgttat 3240
ttatttattt ttttttgtat ttaataattt tttttttttg tttgtttatt gtgtttgtat 3300
ttatttgttt atttttaaat ttagtttgtg ttatttattt gtaaattata ttgttgagtt 3360
gggacgggta ggataaaaga aattttttgt agtaaggtat tggtagtggg gaatggagaa 3420
ggggtaagtt tttaagggta gagagattaa ttagtagaga attgtaatat ataaaaggaa 3480
gaatagttat tagggatata gaaggaatag agttgattaa gaaaaagtta taaattatag 3540
gtttttgttt gaaaaattag ttgaatgaaa atataattcg ttaaaatacg aagtatttgg 3600
ggaatataag tgttgtgtta gttaaattaa atttgttgtg attatgaaat tttagaagga 3660
ggtgttcgat aagtagttag aaataaaggt ttggagttta gtagagagat taaattgtta 3720
tgtggtaatg aaacgtaatg ttggtatatg ggagagttta gaaagcgtta agtttagtga 3780
aaaaaaaatt ttaagaaata tatataaagg agttattaaa tgtggaaaaa aagtggatga 3840

ttagggagaa aggtgttatg aatataaagg ataatttata gtaatataat atggtaatat 3900
aatttttaag tgttaatatt aggatttata ttaagtagta tgatgttaga gaataagtat 3960
ttaattgtgt taatgtattg tgttgtatat aataatttga agttaatttg gagatttaaa 4020
aagagttatt aaaggttgag ttttttatat aattaataga gttatttgtg tttgataaat 4080
tagtttatta gataggatta gaatgatata tttagtttta attttttttt taatttataa 4140
attatttaat tagtttttta gttattattt tttatttttt ttgatgtaaa tagtttttta 4200
aaatgtatat tgtatgttat tttattatta aaaatttttt aatatttgta aaaagtaata 4260
gtaaaatgat ttaatatttt ttaagaattt ataaatagtg taatgtttta gagaatagat 4320
tttgaagtta attggttagg tttaaatttt aaggatgtta tttattagtt gtatggtaat 4380
ttttatatat tttagttttt ttagttgtaa aataaggata atagtattta ttttaaagtt 4440
aatataataa tttttataaa aatgtaatat gagttatata tgtattttaa gttttttaat 4500
gatattttaa aaagggaaag aaaataggta atattttaat gatgtattta atttaatata 4560
tttaaaatgt tattatttta atttgtaatt aatataaaaa aaatttttat attaagttta 4620
taaaatttgt tgagtatttt atatttatag gataatttaa ttaggattag ttatatttta 4680
agtatttaat agttatataa aggtgttgtt tatttatatt gaatgattta gttttaatta 4740
tttaaaattt ttgtttattt tgtttatttt atatttttta taaattgttt ataaatgttt 4800
ttatttaacg gttgtaggat tcgtagtgat gtgatatttt ttaattttta ttatttaaat 4860
ttatgttttt tttttttttt tttatttttt ttttattagt tttgttaagt ggttaattat 4920
attaattttt ttaaagaatt aatttttttt tagttttgtt aatttttttt atgtaagatt 4980
atttttattt tattaatttt tgtttttgtt tttattattt ttttattttt attgttttta 5040
ggtttgattt tttatttttt ttttattttt ttaaaaaagt tttttttttt aatgtataga 5100
tttaaggtta taaattttat tttagtattt tagtagtagt aatttagatt attgtagttt 5160
atttttattt ttaattagtt taaaatattt tttaatagtt attataaatt tttattttaa 5220
gttatatgtt atttaaaagt atagtgtttt atttttaggt agttaggaat tttttagtta 5280
tttttattta ttataatata aataatatat ttaattatta aaattaatga tatttgttga 5340
ggttttgttt atggttttag tttatggtta attttagtat aatttttatg tgtaagggaa 5400
aagatggtta atattattat taatagtttt aatgttttat atatgataat tagattaagt 5460
ttgttaattg tgatgtatgt atgttttata tatgtgtatt tattgtgatg gtatatttgt 5520
ttgttttttt tagtttaaaa taaaattttt ggtttataga tgtttgggtt attttattgg 5580
gtatatataa aggattgtgg taattttggt ggattaattt ttttatatta taaaatgtag 5640
tttttttttt ttattattgt ttattgtatt aaagtttttt ttgtttgatt ggtatagtta 5700
tatttatttt ttttagttag tatttggatg gtatattttt tcgtattatt tttttttaat 5760
ttttttaggt atttatattt aggtaattat tatttgttta gtttgatgat ttttgttatt 5820
tatttgaaat atttagttta tttttatgta atataattat taatttagtt gtttgtttgt 5880
tttatatttt tttttttgtt tttttttgaa tttattatat attttatatt taattttttt 5940
ttgtatttat ttgttatatt tttttttttt agtggttatt ttagatattt ttttatttat 6000
tttttattta atataatttt tatatatata tttttattat tttttttata atgttagaat 6060
tatagaatat tgttgagtag tagttttgtt atgaaggaga ttatttttgg tttgtttttg 6120
tttttaggat gagattttgt agagttttta ttttaaaagt tagtagatcg ttatttttta 6180
ggtatttaga t 6191

<210> 179
<211> 6668
<212> DNA
<213> Artificial Sequence

<220>
<223> chemically treated genomic DNA (Homo sapiens)

<400> 179

gatttgtttg tttcggtttt ttagagtgtt gggattgtag gcgtgagtta ttgcgtttga 60
tttaattttt tattttttgt agagataggg ttttattatg ttgtttaggt tggttttgaa 120
tttttaagtt taattgattt ttttattttg gtattttaaa atgttgggat tataggtatg 180
agttattgta tttggtttat aatgtttttt tttttttttt tttttttttt ttttgggaag 240
ggggtttttt tttttataaa gggggggggg ggggggggga aaaaggtttt tttttttttt 300
tttttttttt tttttttttt tttttttttt ttttgttttt tttttttttt tttttttttt 360
ttttgttttt tttttttatt tttaggtttg ttatttaggt tggagtgtag tggcgtgatt 420
ttggtttatt gtaatttttg ttttttaggt ttaagtcgtt tttttatttt agttttttga 480
gtagttggga ttataggtat gtgttattat gttttattaa ttttttgtat tttttgtaga 540
gaggaggttt tattttgttg ttaggttggt tttggtaatc gttttttgtt gttaggttgg 600
ttttgagttt aagtgattta attatgtcgg ttttttaaag tgttgggatt ataggtatga 660
gttattgtat tcggatttgg ttgttttgtt ttttattttt gagattaaat tttattttgt 720
tatttgggtt ggagtgtagt ggtttagtta tagtttatcg tagttttggt tttttgggtt 780
taagtagttt ttttgtttta gttttttgtt aatttatttt tttagtttaa gttgtttaag 840
agtttattat ttgggaaaat ttatgttatt gattgtagtg ttattgtgtt atttgagttg 900

ttagtataat acgtttgttt tggaaggttt gaatttgtag ttgttatatt tgtaatgatt 960
ttgtatataa gtgttagtat ttaatttttt tattatatat tttaatttag ttggtatgtt 1020
tgagtattta taggtggaag tatatattat atattaagtt tttttttttt tttgttatag 1080
ttagggtatt gttaaatttt taaaatatgt ttaagtagtt tagaattttg ttttagtaaa 1140
gttaaggtgg tgatataaat tatttgttgg aaaaaaaatg ggatgttggt ttgagagagt 1200
ttagatttat ttttttatta tgggtttttt gtttttgtat ttgtagtgtt ttgtatatat 1260
agtgtttgtt gaattggtta atttatgttt tttatatttt ttttatgtac gttgttgtat 1320
ttaattatta taataaattt gtgaagttgt taggggtagg tgtttttatt ttatagatga 1380
gagaattgag agagatgtta atatttagtt atgcggttag atttttaatt agaatatata 1440
attttattta tatcgatatt atgtttttgt ttattatatt tttataattt agaattattt 1500
aatgtaggaa tatatttttt tatttttgtt agtattattt tttatatgta taatttttaa 1560
ttatgtttta ggtatttgtg gttataggaa tataataaag agagagatga gaaagttgtt 1620
tgtttaagaa agcgaggtta ttattatttg tatttaaagt aatttggtat taaaataaat 1680
tttgagatat cgttaaggtt ttagtatagt ataaatttta gaaatgatga tatttgatgg 1740
tgagattgtg gtgaaatagt taatatttaa atattttaat tgtattgtag atttatatat 1800
attttttgga atataatatt agaaaatgat tttaaaagtg tttgtatttt tttaatttgt 1860
tgtaagataa ttttatagaa taaaaagtta tggttagttt agaaatattt gtttggtata 1920
tgttaggaat tgtgtatggt gttgggaatt tttaaaatga gagtgttgtt gttgtttttt 1980
tggattttta atttgtggta ataatgtgag atagatatgt aattaaataa ttaatgtgtt 2040
atattgtaat ttatatatgt aatttataat taaattgtaa tcgtaattgt taaattgtaa 2100
tttaacgtat tttttttttt tttttttttt cgattttttt tttttttatt gagttttatt 2160
ttgttgttta ggttggggtg tagtggtata attataattt atagtagttt ttaatttttg 2220
agtttaagta gtttttttgt tttagttttt tgaggagttg ggattataag tttatattat 2280
tatgtttggc gaatttttta tattttttta gatagatttt aatagaaaat gtagaaaaat 2340
gcgtaatgta ttttaggaaa aatttttttg taaaattatt attatttttt tgagataggg 2400
ttttattgtg ttatttaggt tgtagtgtag tggtataatt atagtttatt aaggttttga 2460
ttttttaagg tttaggtgat tttttagttt tagtttttcg cgtagttagg ataataggcg 2520
tgtgttatta tatttagtta atttttttat tttgagtaaa gattaggttt tgtcgtgttg 2580
tttaggttgg ttttaaattt ttgggtttaa gcgatttatt tgcgtcggtt ttttaaagtg 2640
ttgggattat aggtgtgagt tatcgtgttt ggttataatt aagtatgtta atattttaat 2700
agtagttttt ttattgaata ttatgtatta gatacgttat gtagaatttt ttttttgagt 2760
ttttgtatat ttttttttta atttagattt agagtaattt tattttttga ttattatgaa 2820
aattggaatt ttaatttaaa ttttttgatt ttaaaatttt ttatgtaggt tatttttggt 2880
tatttggaaa gtgtattttt agtatgtgta ttaatgtggg taagtgtttt aagatatatg 2940
aaaaattgtt tttttgtatg gggtttatga gttgagatag atatttatgt gaaagagata 3000
ataatagagg gaggagtaag tttttaatat taggtgattg atgtatattt tgaagaggtt 3060
taaaattata atagtggtag ggagaatggg aggagagata gaaaagattg aggtaaagaa 3120
ttttgtaatt gtaacggaat gtagggatag tgaaggaaag aggtacgtta gagatttttt 3180
gtaaagtttt ttaaggttag gtaattgagt ggaattgata ttttaataaa ggtttttaga 3240
agaggaagag attttggtag aatgatgagt tttattaatt ttgtgggagg atattttaag 3300
gatttaagta tgtagtaaaa gaaaatgaaa gtaagataag tttggaatgt aattttagtg 3360
aataaataaa atataaataa gtatataaag tataggtttt agttttgttt tatttttttt 3420
atttttaagt taatggtttt tttttatttt ttgtggggtt ttgggattag ataagttagt 3480
tttttatttg ttgaatggat tttttattaa tttttagttt ttgttatttt aatttttttg 3540
aaaataattt attttgttgt ttttataggt aaaatttttt gaggaattta gtgatttttt 3600
taaagaatag ttgtattgag atataatttt tatattataa aatttattat ttgaaagtgt 3660
ataatttagt ggtttttatt atagttagag ttgtgtaatt attattatta ttaattttta 3720
ggattttttt attataataa agaaatatta tatttattag tagttatttt ttattttttt 3780
tttttttagg ttttggtaat tattgattta ttttttgttt ttgtggattt gtttattttg 3840
tatattttat ataaatggga ttatataatg tgtgattttt tgttattgat tttttttatt 3900
tagtatgtgt tttcgaggtt tatttatgtt ataatttgtt agtattttat ttttttttat 3960
ggttgaataa tatttcgttg tatggaattt tatttatgta tttattagtt gatggatatt 4020
tgagttgtat ttattatgaa taacgttgtt atgaatattt atgtataggt ttgtgtggat 4080
atgggttttt atttttttta agtagaagtg tgaagagtag aattgttggg ttatatttat 4140
tttgtttaat attttgtatt aatattagat tgttttttaa agtatttgtg ttattttgta 4200
attttagtat taaggagtga gggtttaatt tttcggtttt tttaatattt attattgttt 4260
ttttgatata tttattttag taggtatgaa gtagtagttt attgtggttt tgatttttat 4320
ttttataatg tttaatgtcg agtatttttt atgtgtttat tggttatcgt ttttggagaa 4380
gtgtttttta gaattttttt gtttattttt aaattggggt tttttttttt ttttaattgg 4440
ttttttttaa taaaggtgag tgttaattgt taggtagaat tttatattag gcgatggtaa 4500
attttttata aatattagta atagattttt aggtttaatt agtgttgatt tttataagta 4560
attttttaaa aaagtgttgg taagattttt ttttagggtg attagtaaag ttagtaagat 4620
ttgattgagt atgttattat tataatgaga agatttaatt ttaatatttt tggaatgtta 4680
aaaatagtga tattttaatt aagtaatttt atttttaaaa attattttta atggttaata 4740
ttttgtaaga attttaagta atttttaatt ttgagtatta aattttaatt atgtgtattg 4800
aatttttgat ttaattagag taattttatt tttttttaag tagttagatt tatttcgtaa 4860

aatgtgtttt gatgtgtgtt tttttttttt ttttttttta ggttattttg aatatatgtt 4920
taataatttt ttttgtaacg ttagttgttg ttttttattg tttttaaagg attaaaattg 4980
ttttagaaat tggagatata tttgatttaa aaggaaaaat ttgaataaat ggataatatg 5040
tttattacga atatattaat aagtaatgat gtttgtttga gtattgtgta tagtttgatg 5100
tgttatagat aaggtggaga gagtgaaata tttgtaaaaa gagtaattga aagtttggta 5160
aagaagttga aggagaaaaa agatgaattg gattttttaa taatagttat aattataaat 5220
ggagtttatt ttagtaaatg tgttattata tagagaatat tggatgggag gttttaggtt 5280
agttttataa gagtttttt atattttta tggtggtaga tttaaaaatt tgttacgttt 5340
ttttttaagt tattataggg taatttaatt tgtgttttat ttttttagat attgtgtatt 5400
ttgtataaat atgtattatg ggaatttttg gaagaatatg taaaagataa aattttaaag 5460
ttttttaatg ttttatttag aaaaatgttt aatggagaaa atttggaaaa taaaaataaa 5520
aagtagaaaa tttatatttc gtaagaatta tttttagtat tttggtattt atttttgtag 5580
tttgttgttt tttttttttt ttttttttta gacggagttt tgttttgtta tttaggttgg 5640
agtatagtgg cgtgattttg gtttattgta atttttattt tttgggttta agtaattttt 5700
ttgttttagt tttttaagta gttgggatta taggtaagtg taattatgtt tggttaattt 5760
ttgtattttt agtagagatg gggttttatt atgttggtta ggttggtttt gaatcgttga 5820
ttttaggtga tttattcgtt ttagtttttt agaatttggg tattataggc gtgagttatt 5880
gtgtttagtc gtgttgtttt tttttttgat attgtatgta tatttaaata atcggattat 5940
atttagtttt tttataattt agaaatttga tatgttttat tgttagtaaa tgttttattg 6000
gttatatttt ttaggtagaa ttttataata tagttttttt gtttaaataa attttataga 6060
aattttgtcg tagattttaa agttttattt taagatttta tatatagttt ttgtaatata 6120
gtttttttgt atatttattt atttttattt atttttttga gatatggttt tatttttgtt 6180
taagttggag tgtagtggtg taaaatatgg tttattgtag ttttgattta ttgggtttat 6240
gtagtttttt tgttttagtt ttttgagtag ttaggattat aggtatatat tattatgttt 6300
gggtaatttt ttattatttg tggagataag ggttttgtta tgttgtttag gttggttttg 6360
aattttgggg tttaagcgat ttttttgttt tggtttttta aagtgatagg attataggtg 6420
tgagttatta tgtttagtta ttttttatat ttttaaaatt tgagtatagg ttttgaaatt 6480
atattaaggt ttaaatttta ggtttgatgt atgatatggt taagttagtt attgtgaatt 6540
ttagtttttt tatttatgaa atggggattg taatattgag ttggtaggat tgtgaggatt 6600
aaattagaat atataaaagt gttttgtata atgtgatata tgaataaatg gtcgtttatt 6660
tttttagg 6668

<210> 180
<211> 6668
<212> DNA
<213> Artificial Sequence

<220>
<223> chemically treated genomic DNA (Homo sapiens)

<400> 180

tttagaaaaa taaacgatta tttatttatg tgttatatta tgtaagatat ttttatatat 60
tttgatttaa tttttataat tttattaatt tagtattata atttttattt tataaataag 120
aaaattaaaa tttataataa ttaatttggt tatgttatat attagattta gaatttaaat 180
tttggtataa ttttaaggtt tgtgtttagg ttttaaaaat gtaaaaagtg attgggtatg 240
gtggtttata tttgtaattt tattattttg ggaggttaag gtaggaggat cgtttgagtt 300
taggagttta agattagttt ggataatatg gtaagatttt tgtttttata aataataaaa 360
aattatttag gtatggtggt atgtgtttgt agttttagtt atttaggagg ttaaggtagg 420
agaattgtat aagtttagta ggttaaggtt gtagtgagtt atgttttgta ttattgtatt 480
ttagtttggg taaaagtaag attatgtttt aaaaaaataa aataaaataa ataaatatgt 540
aaaaagattg tgttgtagaa attatgtgta aagttttgaa ataaaatttt aaaatttacg 600
ataaaatttt tgtaagattt atttaagtaa gggagttgta ttatgaagtt ttatttgaaa 660
aatgtgatta atgaaatatt tgttaatagt gaaatatatt agatttttaa attataaaag 720
gattaagtgt aattcggttg tttaaatgta tatataatat taaaaaagaa aataatacga 780
ttgggtatag tggtttacgt ttgtaatgtt agaatttggg aaggttgagg cgggtggatt 840
atttgaggtt agcggtttaa gattagtttg attaatatgg tgaaatttta tttttattaa 900
aaatataaaa attagttagg tatggttgta tttgtttgta gttttagtta tttaggaggt 960
tgaggtagga ggattgtttg aatttaagag gtggaggttg tagtgggtta agattacgtt 1020
attgtatttt agtttgggtg atagagtaag atttcgttta gggaaaaaaa aaaaaagaaa 1080
ataataggtt ataaaaatga atattaaagt gttaaaggta gtttttgcga agtgtgggtt 1140
ttttgttttt tatttttatt ttttaaattt tttttattga atattttttt gagtagaata 1200
ttaaaaaatt ttaagatttt attttttgta tattttttta gaaattttta taatgtatat 1260
ttgtatagga tgtatagtat ttgaagagat ggagtataaa ttaaattatt ttgtagtagt 1320
ttgaaaggaa acgtagtaag tttttaaatt tgttattata gagggtatag aaaaattttt 1380
ataagattaa tttgaagttt tttatttaat gttttttgta tggtaatata tttattagga 1440

tgagttttat ttgtagttat agttgttatt aaagaattta atttattttt tttttttttt 1500
agttttttta ttaaattttt aattgttttt tttgtaaatg ttttattttt tttattttgt 1560
ttatggtata ttaaattatg tataatgttt agataggtat tattatttgt tggtgtattc 1620
gtaatagata tattgtttat ttgtttaagt tttttttttt aaattaaata tgtttttaat 1680
ttttgaagta attttgattt tttggaaata gtgaaaaata atagttaacg ttgtaaggaa 1740
aattgttaga tatgtattta ggataattta aaaaagaaaa aaaaaaaaga tatatattag 1800
aatatatttt acgaagtaga tttgattatt tgggaaaaga tgaaattgtt ttggttaagt 1860
tagagattta atgtatataa ttaaaatttg atgtttagag ttgaaaatta tttaaaattt 1920
ttataaaata ttagttatta aaaataattt ttaaaaataa aattatttaa ttaaaatgtt 1980
attgtttttg atattttaaa aatattaaag ttaggttttt ttattatgat agtggtatgt 2040
ttaattaagt tttattgatt ttattagtta ttttaaagga aaatttattt agtatttttt 2100
taaaggatta tttatgagga ttagtattag ttaaatttaa aagtttatta ttaatattta 2160
tgaaaaattt attatcgttt ggtatgaaat tttatttagt agttaatatt tatttttgtt 2220
aaaagagatt aattaaaaaa aaaaaaaaat tttaatttag aaatgaataa aaggatttta 2280
aaggatattt ttttaaagac gatggttaat aagtatatga aagatgttcg atattagata 2340
ttatggaaat gaaaattaaa attataatga gttgttattt tatatttatt gagatggata 2400
tattaaaaag ataatagtaa gtattgaaga agtcgagaaa ttggattttt attttttggt 2460
gttgggatta taaaatggta taggtatttt ggaaaatagt ttggtattaa tataaaatgt 2520
taaatagagt aaatatgatt tagtaatttt atttttttata tttttatttg agggaaatga 2580
aaatttatgt ttatataaat ttgtatatga atgtttatag tagcgttgtt tataatggat 2640
ataatttaaa tgtttattaa ttgatgaatg tatagataaa attttatata acgaaatatt 2700
atttaattat aaaaaagaat aaagtattaa taaattataa tatggataag tttcgaaaat 2760
atatgttaag tgaaagaagt tagtgataaa aggttatata ttatataatt ttatttatat 2820
gaaatatata gaataggtaa atttatagag atagaaagta aattagtggt tgttagggtt 2880
tgggaggaag gggaatgagg aatgattgtt aatgggtatg gtgttttttt gttataatga 2940
aaaagtttta aaaattgata atggtgatgg ttgtataatt ttgattatag taaaaattat 3000
tgaattgtat atttttaaat agtgaatttt atggtatgga aattatattt taatatagtt 3060
gttttttaaa gagattatta aattttttaa aagattttat ttgtaaaagt agtagggtaa 3120
attattttta aaaaaattaa aataataaaa attgaagatt aataaagaat ttatttaata 3180
ggtagaaaat taatttattt gattttaaga ttttatagag aataggaaaa ggttattagt 3240
ttaagaataa agaagataaa gtaggattgg agtttgtatt ttgtgtgttt gtttgtattt 3300
tatttgttta ttgaagttgt attttagatt tattttgttt ttattttttt ttattgtata 3360
tttgaatttt taaagtattt ttttataaga ttaataaaat ttattatttt attaaaattt 3420
tttttttttt tgaagatttt tgttaaagta ttaattttat ttaattattt agttttggga 3480
agttttgtaa aagatttttg acgtgttttt tttttttatt atttttatat ttcgttatag 3540
ttataagatt ttttatttta gtttttttta tttttttttt tatttttttt gttattgttg 3600
tagttttgag tttttttaga atgtatatta attatttgat attgaagatt tgtttttttt 3660
tttgttgtta ttttttttat ataaatgttt gttttaattt ataaatttta tgtagagaag 3720
tagtttttta tatgttttag agtatttgtt tatattaata tatatgttga aggtatattt 3780
tttaaataat taaagataat ttgtatggga gattttggag ttaagagatt tgaattagaa 3840
ttttagtttt tatagtagtt agaaaatgaa gttattttga atttggatta aaaggagggt 3900
atatagaagt ttaaaggaaa aattttgtat aacgtgtttg atatatagtg tttagtaaaa 3960
aagttattgt tgaaatatta atatgtttaa ttgtggttag gtacggtggt ttatatttgt 4020
aattttagta ttttgggagg tcgacgtagg tggatcgttt gagtttagga gtttgagatt 4080
agtttgggta atacggtaaa atttgatttt tatttaaaat aaaaaaatta attgggtgtg 4140
gtggtatacg tttgttgttt tagttacgcg gggagttgag attggaagat tatttgagtt 4200
ttgggaggtt aaggttttgg tgagttgtga ttgtgttatt gtattatagt ttgggtgata 4260
tagtgagatt ttattttaaa aaaataataa tagttttata agaagatttt ttttaaaatg 4320
tattacgtat ttttttgtat tttttattaa aatttgttta aaaaaatgta ggaggttcgt 4380
taggtatggt agtgtgggtt tgtagtttta gttttttagg aggttgaggt aggaggattg 4440
tttgagttta ggagttgaag gttgttgtga gttatgattg tgttattgta ttttagtttg 4500
ggtaatagag tgagatttag tagaaaggaa aggaatcggg aaagagggaa aaggaaagat 4560
acgttaaatt ataatttaat aattacgatt ataatttaat tgtaaattat atgtgtaaat 4620
tataatgtaa tatattagtt atttgattat atatttgttt tatattgttg ttataaatta 4680
gaaatttaag aaaatagtaa taatattttt attttagaaa tttttagtat tatgtatagt 4740
ttttgatata tattaggtaa gtgtttttgg attgattata gttttttatt ttgtggaatt 4800
gttttataat aaattagaag agtgtagata tttttgaagt tattttttga tattatattt 4860
taaaagatgt gtatgaattt ataatgtaat taaaatgttt aagtgttaat tattttatta 4920
taattttatt attaaatgtt attattttta aaatttatgt tatgttgggg ttttaacggt 4980
attttaaagt ttgttttaat attaagttgt tttgaatata aatgataatg atttcgtttt 5040
tttaaataaa tagttttttt attttttttt ttattgtatt tttatgatta taagtgtttg 5100
aaatataatt aaaagttatg tatataaaaa ataatattag taggaataaa gagatgtatt 5160
tttatattaa atggttttag attatggaag tatggtaggt aagagtataa tatcgatata 5220
aataaaatta tatattttaa ttaaaggttt agtcgtatga ttgaatgtta atattttttt 5280
taattttttt atttataaaa tggaaatatt tgtttttgat aattttataa atttattgtg 5340
gtaattagat gtaataacgt atataaaaga aatgtgggaa atataaattg attaatttaa 5400

taaatattgt atatataaag tattatagat ataaaaatag gaagtttata gtaagaaagt 5460
gggtttggat ttttttagat taatatttta tttttttttt aataaataat ttatattatt 5520
attttagttt tattgaaata aaattttaaa ttatttaaat atattttgaa aatttaataa 5580
tgttttaatt ataataaaaa ggaggaaaag tttaatatat aatgtgtatt tttatttata 5640
aatatttagg tatgttaatt aagttagaat gtataataaa gaagttagat gttagtattt 5700
atatatagaa ttattataaa tgtgatagtt ataaatttag attttttaag ataaacgtgt 5760
tatattggta atttaaatag tataatggta ttgtagttag taatatggat ttttttaaat 5820
ggtgaatttt taaataattt gaattaagaa aatgaattaa taagaggttg gggtaggagg 5880
attgtttgag tttaggagat taaggttacg gtgagttatg attgagttat tgtattttag 5940
tttaagtgat agagtaagat ttggttttaa aaataaaaaa taaaataatt aagttcgggt 6000
atagtggttt atgtttgtaa ttttagtatt ttaagaggtc gatatggttg gattatttaa 6060
gtttaggatt agtttggtaa taaaggacgg ttgttaggat tagtttggta ataaagtgag 6120
attttttttt tataaaaaat ataaaaaatt agtggggtat ggtggtatat gtttatagtt 6180
ttagttattt agaaggttga ggtgggagga cggtttgagt ttgggaggta gaggttgtag 6240
tgagttaaga ttacgttatt gtattttagt ttgggtaata gagtttgaag taagggaggg 6300
aggtagggag ggaggaagga aaaagaaaga aaatagagag aaagagagag ggagagaggg 6360
agggagggag ggagggaggg agtttttttt tttttttttt tttttttttt tgtgaaaagg 6420
gggatttttt ttttaaaaaa aaaaaaaaaa aaaaaaaaaa aaaatattgt aggttaggtg 6480
tagtggttta tgtttgtaat tttagtattt tggaatgtta aagtgggagg attaattgag 6540
tttagaagtt tagggttagt ttgggtaata taataagatt ttgtttttat aaaaaataaa 6600
aaattaggtt aggcgtagtg gtttacgttt gtaattttag tattttggga ggtcgaggta 6660
ggtaggtt 6668

<210> 181
<211> 8077
<212> DNA
<213> Artificial Sequence

<220>
<223> chemically treated genomic DNA (Homo sapiens)

<400> 181

ggttcgggtt ggttgttcga gagtgattgc gagacggtga tttgttgttt tttttcggag 60
cgggacgtcg tcggcgcgtt tcgggacgtt agcgattttt tggtcggggc ggttttggag 120
tcggcgggcg gcgggcggag tcgcgaagcg cgttcgcggt tgttgttgtt ggagtaggaa 180
tttaaaatcg ttacgtattc gttgttgaag cggtttaagg agcgttcgtt ggatacgttg 240
ttggaggcgg tggagtttcg cggcggcgtg tcgggcggtt gcgtgttggt gtcgcgcgtc 300
gattttcgtt tgggcggtta gttcgcgtcg tcgtagttgt tgttcggtcg tttttttcgt 360
tggttcgatt tgtagtacgt cgtggagttg aagtttttgt gcggttgtta tagtttcgtc 420
gtcgtcgtcg acggttttat cgtgtgttgt aatttttatt attttagtcg gttttgcggg 480
ttcggtgagc gcgttgcgtc ggtcgggggg gtttcgggtt ttcgttttta ttttttttcg 540
tgtttttttt tttgtgatat tgcgggtcgg cgtagttgcg ggtgtttttt cgggtgtttt 600
tggagcgtgg gagttattag gggaggcgtt ttagatcggt cgaggggagt gggtgtttta 660
gtatatatat taagtggtaa ttttatttta aggtttcggt aaatttaaaa gggtatatgt 720
cgtagttttt tttgtgtagt tttagggata tgatgtaggt tttaatttta taggtagtga 780
aaggggaggg taggttaagg agagattaaa gaagtaggtg ttattttttt ttattttaat 840
ttcggaattt atttagtttt gtgtttaggt ttttagtatt tgtatgtacg tgtttttatt 900
tatattgtgt ttttttatgt ttaagtagtt gtaagttgtg aaattgggtg tatataaatt 960
atagttagaa ttaaaagtgt tcgttaaatg tttaatttgt gtttttgtat attttcgata 1020
aagttttgta gggtaatttt tttttggagt aaagagtatt ttttttattt tttatataat 1080
attatttttt atattttttg ggtgttaagt gttagggttt tttatagttt tttttaagtt 1140
ttgtgtttag tttttgtata gagtttaggg ttttggaggt attcgtgagt agatgaagaa 1200
gttagatatt ggattatttt tgagaattgt tttttttttt tgtttttttt ttgggatttt 1260
ttgtatacgt tttattttta gttttgttta tttggttagg ttttggtgtt taggtgtaga 1320
taggtagagg ttttagtagg ttaggaggtt ttagtaggtt aggagtttgt agttgttttg 1380
ttttgatttt tgttttgtgg ggaatatata ttttagtttg tagttagttt ttgagggatg 1440
tatgggaatt tattagttgg tattggggtt ttgtaggggt gggattggta gggagttata 1500
ggtttttgag tttgggaggg ttttgaattt ttttattttg gtttttttgg ggagagttag 1560
tatttaggtt gggagtatag agagggttag gttgtaggtt ttttgatttt gtgtggaagg 1620
ggaaagtggg tttaggatgg tgtatttgtt ttaggtgttt tgtttgagtg tggagtagtg 1680
tttggagagg aaaggaagtt tttttttagt gatgattttt gtatagaggg tattgatggg 1740
ggtaggtggg gaggggaagt gttgatgagg tattttattt tttttatggt tattttagtt 1800
ttaatttagg agttatgagt agtttttgat ttgtggttta tgttaggttt tttatgtatt 1860
tttttttttt tattgatagt tttttttgga agtgattttt ttttttttgt taattgattt 1920
tttattgttt ttggggaagg agattagtat ttatggttgt tttgtttttg gtgatttttt 1980

ttttttaaa gtaggtttt tttatttcg tagaagtttt tggtaggtgt atttttttc 2040
gttattttag tgtgtagtag atgtttgttg agtaattatt ttgggttagg tattgtgttg 2100
ggtgttgtgg ttaatagagt ggaaatatga gtaggattta tgtttttaag aaatataatt 2160
ttattaggga tttatggggg agagatgggg gaagaaggga agtgtgtagg gttaggttat 2220
tgggtgtgaa ttattagaag gttgtgggtg tgtatgtaat aggatggtat gtaggagttt 2280
tatagtttta atttggagtc ggattttgga ggatgggtta gaggagaggg agttagtcgg 2340
gttagaggag agggagttag ttagtttaga gttaaggtgt taggtacgtt taggtggttg 2400
ggtagggagg ggaggagtga aaaagttgga gacgatggat aggattattt ttgttattat 2460
ttttgggatt aggagttggt ttgggtgttg gtttgtattt ggtaggtttg gagaggtata 2520
ggttaagaga tatttggtaa gttgtagtag aatagttttt gattatggat ttggttttag 2580
gttttgttta gttagtaacg tgtagtttga ttttggatga gttatttttt tttttagtta 2640
tcgtgttggt tagtgggggt ggttttagga atattttttt tatttaaatt tggattaagt 2700
tttgggattt gatttttttt ttttttttag aaggaggcgt tgttgttagg gttgtagtat 2760
tttaagtttt tttttttttt tttgttagtt gttagtttgt ggggttttta tttagttatt 2820
ttggggatag ttgggaggat cgatggttcg ggaagagttt ttagtatgtg ttaggttgat 2880
ttttttgttt ttggtttgag agttggggga gttttgggtt gtttattcgt ttttatcgtt 2940
tggtcgttag tttggttttc gtaattgggc gggcgtatag ttggggttta gcggtgcgcg 3000
ttttgataag gagattgcgt tttttagggt tttgggaaga gggtcggggt atcggtagaa 3060
tagatgcgcg tatatatata gcgtaggtat atcgttagcg agtaaagtat attggaatat 3120
cggtattgag ggatttttat ttggtagtag ttattttcgt atttttatcg attattgtag 3180
ggagtttcgg tgatagaaga tttttttttg ggtagtgtgg atttttgttg tttagagttt 3240
attttttatt aatttgagtt gtggttttgg ggttagtttt ttttttgggt tttttttttt 3300
atatttaaag taaagggttg gagggaattt gtataatttg agggttgatg tgtagatttt 3360
aggttatata ggttttcgtt ttttttttta ttttgtttta ggtttgtttc ggaaattggg 3420
gtttttagga ggggatttat tttttttggg tatgggttag gggttttttg gttttttagt 3480
tcgtgggagt taggtttaat tttgtttttt ttttgggtgt ttttttgttt taggaaaagt 3540
tagttcgttg agttcggtgg gtgtttatgt ggacggaaat gtggagaaat attttaatta 3600
tagttttaat ttgtgtttat agagtttttt ttttttgaaa gtatttatta atttcggaat 3660
aaattttttg ttttttgttt ttttattttt tttttttttg taattatatt tagtaaatta 3720
ttattattta aattgcgttc ggggaagaga gatagagcga gagagagaga atttttaaac 3780
gattggagga ggtcgtttag gaaaatatag ggtttttagg ttaggattat aggaattaga 3840
atgggttaga aagttttttt tttatttggt ttggtattat ttttttgaaa tgagattaat 3900
tttaagttgg gtttttaagt tttcgttttg ttcggtttag cggtttttag ttttgtttgt 3960
ttgttttttt tagttttttt tggaggtgag gaaggggtga taatgtgtag tttgtttttt 4020
gttggcgtta gtcggtcgcg ttgtttattt ggttgttagg ggaatttggg taattaggtt 4080
taggcggttt taaagtgtta ggagtttttt ttttgcgttt tttattatgg ggtttagggt 4140
tgagttttta ggttttgggg gtaggaagga cgggtattta ggaggttttt tttttattta 4200
tagttttttt ttgggagggg ggaaatttgg taattcggga ggtatgtgga ttttttttta 4260
agtaagatgt tgagttggaa agatgggggt gtaaggtaat gttttaaatt gaaattttgt 4320
taggtattgg gagaggttgt gaattttttt ttggttttag aatttaggtt tagattttaa 4380
aaggttaagt atttttgggg ggttaattag aggtatgttt gggttgagtt gaattttttg 4440
gtgtattggt ttttggttga ttgttttttt gtaggaagtt ggaggagatt tttgaagttg 4500
attttttagg ttggatgttt aagggggttg gagtttttga tgtttttttg tttttttttt 4560
tttttttttt ttttttatta ggtttatttt ttttagaggg gtttgcggtg ttttaaaagt 4620
ttttttgtta aagtttagag taaattggtt attattttaa aattaataaa atttttaaaa 4680
gtattaagat aatttttaag aggggagtgg atagagggtt tggtggtagt ttatagtttt 4740
ttttttgatt tttggtttta tttattaagt gttttatttg agtgtttatt ttgtttattt 4800
gaggtaatgt tttggggttt tattagttta gatttatagg gcgtagttat gtgggagtgg 4860
cggttgattg ttatttagta gtgttgatag tatattattt ataatagtta aagagaggaa 4920
gtaatttaaa tgtttattag ttgataaatg gataaatgaa atatggtacg ttcgaagaat 4980
ggaatattat ttatttatga aaaagaacga agtttagtat taaaacgtgt tataatatgg 5040
atgaatttcg atgattttgt gttatatgaa agaagaagtt agttataaaa ggttatatat 5100
tgtatgaaat gaaatgttta gaatgggtaa atttatagag atataaagta agttagtggt 5160
tgtttagggt tttggggttg gggtttagaa agtgattgtt aatggggatg gtgttttttt 5220
ttgcgataaa aaaaatgttt tgtgtttaga tggtggttga tggttgtata atattgtgaa 5280
tatatttaaa aattattgaa tggtatattt ttaaaaagga tgaattttag ggtatgtgaa 5340
ttatatttta atttttggaa agggttattt gatattggtt tgtaattggt tttttttgta 5400
atatttgtag ttatttttat gtgtggtttt gggtaggttt tttagtaagt gggattttag 5460
ttttatatta gtgaaaagaa ggaattgagt tgggtgaatt ttaaggtttt ttttgttttt 5520
gatattttaa atttttgtgt tgtggggtgg agtttaaagt ttttatattt ttttagttgt 5580
ttataatgga atttagtgat tagtttttag tttttgtagt tgttaaatag ttgtgtgtat 5640
attattttgt tagagttaat gggagtttgg tgaggttttt tttgaacggt ttgtagtgtt 5700
ttattagttg gtatgtttta tggtttaggt ttatagattt tttttttatt ttataatatt 5760
ggaattttta atttagttat ggttatgggt agtaggtgtt ataatttaag gtttaggtag 5820
tttttttagg tgggatttta tgagtttttt ttgtttttta ttttttgagt tttaggtaag 5880
tagtttttgg aagagatgtt ggtattggtt atagaggttt gattttagtt attattgttt 5940

atagttgtta agatttattt ttggggataa attttggggg tagatgttta gagttttatg 6000
ttatgtgtgg tttagatttt agttagttag atttttgtaa aagtaatttt aaggaattgt 6060
aggttttttg ataagtttat gggggtggtg gaaagggtat ggaggttaga gatttggttt 6120
tggttttggt tcgatattta gtttttgagg gagttatttt tatttattag gttatagttt 6180
ttttttttgt atagtggaaa tattggtttt aatttttttt ttttgtggaa ttttagtttt 6240
ttttatgttt tgttttttat tttgagaggt tggtttttat ttaaggttgg tttttaggtt 6300
tgagagtttt ttagattttt tttttgtata tttttggtta tagatgtatt taattttatt 6360
attattttta ttttagttgt ttgtgtttat atttagtgag cgcgttttgt gtaggtggtg 6420
ttttagattt agcggggatg aaggggtgta agatagggta ttttttgatt ttattttatt 6480
gggtggtaga gtagagttag gtttggattg gaattttagt tttattatga ttggttcggg 6540
gaatatgcgt aagttagttt ttttatttgt aaaatgggga taataatgtt atttatttta 6600
atttttgtat tttgagaaag gattaaataa aaataggtgt ttgatgagta taattaatta 6660
ttttgtattt gaggtttaaa atagtaaaat tgaaagtgat tggtatggag tgagatataa 6720
cgtagatatc gagtaaatgt tcgcgttttt tttagtggtt attttgtgat ttattttttt 6780
ttttttttaa tttttgttta tagaaagttt ttgtttttaa attttagagt tttgattttg 6840
ttggaagtaa ttttgagttg gaaaagtcgg ggtggggaag aggtgaggtt ttattgggtg 6900
tatggagtaa aataaatgtt tttgtaatta attgtggttt attataattg tgaaaaatat 6960
ttattatttt ttgtgttatt attgatattc gtttaataat agttattatt attgagagtt 7020
tgttttagtt tgcgatttga ttgtgtttag ttttatttat cgaaaatgaa gggttatttt 7080
atttatgtaa attgttattt agtttagtaa attgttattt tttataaagt aagaagttaa 7140
ggaatttttt tgaaagtaaa gtatatgttt ttaaggagaa gtattttagg taaatgaaat 7200
ttaaagaaaa atattataga taataaggaa ttatagtatt tgagttgtag agagtttgtg 7260
ttttattttt gttttttata aaaggggaga ttgaggttaa tagttggaag tgattggttt 7320
aaggtttatt gagatagttg ggtttggttt tttgggtttt aatttttttt attgtttttt 7380
tttgtattta ggaatgttag ttttaatgta gtttgtttgg atacgagttt ttgtgtttgg 7440
gtttttgtat ttatattagg tttgttaaat ttagtaaata aaatgtagga tgtttagtta 7500
aattcgagta ttagataaat aatatattat attttagtat aagtatattt ttaatattga 7560
aagggatata tttatattat aaaatgatgt tatgtttatt taaaatttaa atgtaattga 7620
gtattcggta ttttatttag taattttggt tttgattttt tttgtattta cgtttttttt 7680
gttaaagtag taatagttat tgtttatgga ggtgtttttg gatagggtta agagttgtat 7740
taagtatttt atatatatta ttttagtaaa tttgggaggt ttagatgatt attattttta 7800
ttttagagtt gaggaaatag gtttagagag gtgaagtaag ttgtttgagg ttatatagtt 7860
aaaggtggta gagttgggat tgagtttata tttgtttgtg ttttaagttt gcggttttaa 7920
ttgttgaggg gatttgggta gtagaggagg tgggataaag ggagggtttt ttggaggttg 7980
agtttattat ttgggaaatt agtaattaaa agtatgtaga atttgaggtg tgagtttttt 8040
aattttggta gtgatatgtt gttttttgtt ttttaga 8077

<210> 182
<211> 8077
<212> DNA
<213> Artificial Sequence

<220>
<223> chemically treated genomic DNA (Homo sapiens)

<400> 182

tttggaagat aggagatagt atgttattgt tagggttggg gagtttatat tttaggtttt 60
atatgttttt aattattggt tttttaaata ataaatttag tttttaggaa gttttttttt 120
tgttttattt tttttgttgt ttaaattttt ttaatagtta aagtcgtagg tttggggtat 180
agatagatat gggtttaatt ttagttttgt tatttttggt tgtgtgattt taagtaattt 240
attttatttt tttgagtttg tttttttagt tttaaaatga ggataataat tatttagatt 300
ttttaggttt gttgggataa tgtatgtaaa atgtttagtg tagtttttgg ttttgtttaa 360
aaatattttt ataaatagta attgttatta ttttaataag agaaacgtga gtgtagggaa 420
agttagagtt agggttgttg gataaaatat cggatgttta gttatatttg agttttggat 480
aaatatggta ttattttgta gtataagtgt gtttttttta atattggggg tgtatttata 540
ttaaaatatg gtgtgttgtt tatttgatat tcgaatttaa ttgggtattt tgtattttat 600
ttgttaaatt tggtaagttt agtgtgagtg taaaggttta gatatagaaa ttcgtgttta 660
aataagttgt attagagtta gtatttttag gtgtagggga ggatagtaga aagggttagg 720
atttaaaagg ttaaatttag ttattttaat aggttttaag ttagttattt ttagttgttg 780
gttttagttt ttttttttgt aaaggatagg ggtggaatat aagtttttta tagtttagat 840
gttatgattt tttattgttt atgatatttt tttttgggtt ttatttgttt aaaatatttt 900
tttttaaaaa tatatatttt gtttttagaa gaatttttta gttttttgtt ttatgggaag 960
taataatttg ttaaattaag taataatttg tatgaatgaa atgatttttt attttcggta 1020
aatgaaatta aatataatta aatcgtaaat taaaataaat ttttagtggt aataattatt 1080
attaagcggg tattagtaat gatataaggg gtaataagtg ttttttataa ttgtaataag 1140

ttataattag ttataggagt atttgttttg ttttatatat ttaatgaggt tttatttttt 1200
ttttatttcg gtttttttag tttaaagtta tttttaataa agttaaagtt ttgaggttta 1260
gaaatagaga ttttttgtgg atagaaatta gggagaagga aaagtgaatt atagagtagt 1320
tattgagaga gacgcgaata tttgttcggt gtttacgttg tgttttattt tatgttaatt 1380
atttttaatt ttattatttt agattttagg tatagagtag ttaattatgt ttattaggta 1440
tttattttta tttaattttt ttttaaaata taaaaattaa atgagatgat attattattt 1500
ttattttata gatgagaaaa ttgatttgcg tatgtttttc gagttagtta tggtggagtt 1560
gggattttag tttaggtttg attttgtttt gttatttaat aaggtggaat taagagatgt 1620
tttgttttat atttttttat tttcgttggg tttgggatat tatttgtata gaacgcgttt 1680
attaaatgtg gatatagata attgggatga ggataatggt aaagttgaat gtatttgtgg 1740
ttaaagatgt gtagaaagag gatttagggg gtttttaggt ttagaggtta gttttgagtg 1800
aggattagtt ttttaggata agaggtaggg tataggaagg gttggagttt tataggaaaa 1860
gagaattgag gttagtgttt ttattgtata gagaaggaga ttgtggtttg gtgagtggaa 1920
gtgatttttt taggagttag gtatcgagtt agaattaaag ttaggttttt gatttttatg 1980
ttttttttat tatttttatg aatttattag aaagtttgta attttttagg gttatttttg 2040
taaaaatttg attggttgag gtttaggtta tatatggtat ggagttttga atatttgttt 2100
ttaagatttg tttttaaagg tgaattttga taattatggg tagtagtggt tggagttaag 2160
tttttgtaat taatgttagt atttttttta ggggttgttt atttgggatt taaaaggtgg 2220
aaggtaggaa aggtttatga agttttattt ggaagagtta tttgggtttt gagttgtgat 2280
atttgttatt tatggttata gttgagttgg gagttttaat gttgtgagat agaaagaaaa 2340
tttatgaatt tgaattatga gatatgttag ttagtggagt attataaatc gtttaggagg 2400
gattttatta ggtttttatt agttttggta aaatagtatg tatataattg tttaataatt 2460
gtagggattg aaagttaatt attaaatttt attgtaggta attaaaaaag tatgaaggtt 2520
ttagatttta ttttataata taaggattta gagtgttaaa gatagaagag gttttggggt 2580
ttatttaatt taattttttt tttttattga tatgaaatta aggtttattt tgttgaagga 2640
tttgtttaag gttatatatg aaggtagttg taggtgttat aggggaggtt agttatagat 2700
taatattagg tgattttttt taaaaattga gatataattt atatatttta agatttattt 2760
tttttaaaaa tatattattt agtggttttt gagtatattt atagtgttgt gtaattatta 2820
attattattt aaatataaga tatttttttt atcgtaaaaa gaaatattat ttttattggt 2880
agttattttt tagattttaa ttttaaaatt ttgggtaatt attaatttat tttgtgtttt 2940
tatgggtttg tttattttgg atattttatt ttatataata tatggttttt tgtggttggt 3000
tttttttttt atgtggtata aagttatcga agtttattta tgttgtagta cgttttggtg 3060
ttggatttcg ttttttttta tgggtgaatg atattttatt tttcggacgt attatatttt 3120
atttatttat ttattagtta atggatattt gggttgtttt ttttttttgg ttgttatgaa 3180
taatgtgtta ttaatattat tgggtaataa ttagtcgtta tttttatatg gttgcgtttt 3240
gtggatttgg attggtggag ttttagggta ttattttagg tgggtaaggt gggtatttag 3300
gtgggatatt tggtgggtga gattaaaggt tagaaaagaa attgtgagtt gttattaggt 3360
tttttgttta tttttttttt agaagttgtt ttagtatttt taaaagtttt attgatttta 3420
aaataataat taatttgttt taaattttaa taggagaatt tttagagtat cgtaggtttt 3480
tttgaaagaa gtggatttgg tagggagaga aagaaaagag agggagatag aaagatatta 3540
gaaattttaa tttttttgga tatttagttt gaggaattaa ttttaggaat tttttttaat 3600
tttttgtaga agagtagtta gttaggggtt agtgtattag aaggtttagt ttagtttagg 3660
tatgtttttg gttagttttt agggggtatt tagttttttg ggatttagat ttaaatttta 3720
aagttagaaa agagtttata gtttttttta gtgtttggta aagttttagt ttgggatatt 3780
attttatatt tttatttttt tagtttagta ttttgtttag gaaaagattt atatgttttt 3840
cgggttgtta agtttttttt tttttaaaga ggggttgtgg atggggaggg ggttttttga 3900
gtgttcgttt tttttgtttt tagaatttga agatttaatt ttaagtttta tgatgggaga 3960
cgtagaaaag gagtttttgg tattttaagg tcgtttgagt ttaattgttt agattttttt 4020
ggtagttaga taaatagcgc ggtcggttgg cgttagtaag aggtaaattg tatattatta 4080
tttttttttt atttttagga ggagttagga ggagtagata agtagggtta ggggtcgttg 4140
agtcgggtag ggcgggggtt tggaagttta atttgaaatt gattttattt taaagggata 4200
atgttaggtt aaataaaagg aaaatttttt agtttatttt aatttttata gttttggttt 4260
gggaattttg tgttttttta ggcggttttt tttagtcgtt tagaagtttt ttttttttcg 4320
ttttgttttt ttttttcggg cgtaatttgg ataatagtaa tttattgaat ataattatag 4380
aaaaaaaaaa agtggggaag taaagagtag gagatttatt tcgaagttaa tggatatttt 4440
taggagaaag gagttttata aatataggtt agagttgtga ttaaagtatt tttttatatt 4500
ttcgtttata taggtattta tcgagtttag cgaattggtt ttttttggga tagaaagata 4560
tttaaggggg aggtagagtt gggtttgatt tttacgggtt gggaggttag gaggttttta 4620
gtttatgttt agaagaggtg ggtttttttt tgaggatttt agttttcgag ataagtttgg 4680
ggtagagtgg gggaggggac ggaggtttgt gtggtttgga atttgtatat tagtttttag 4740
attgtgtaga ttttttttaa ttttttattt tagatatagg aaaagaagtt taaagaggaa 4800
attagtttta aggttataat ttaagttgat ggagagtggg ttttgaatag tagggtttta 4860
tattgtttag gaaggggttt tttgttatcg ggtttttttg tagtggtcgg tgaaggtgcg 4920
gaggtgattg ttgttaggtg ggagtttttt agtgtcggtg ttttagtgta ttttgttcgt 4980
tagcggtgtg tttgcgttat gtgtgtgcgc gtatttgttt gttcggtgtt tcggtttttt 5040
ttttagagtt ttggaaggcg tagttttttt attaggacgc gtatcgttga gttttagtta 5100

tgcgttcgtt tagttacggg agttaagttg gcggttaagc ggtggaggcg ggtgggtagt 5160
ttagggtttt tttagttttt aggttagggg taagaggatt aatttggtat atattggaaa 5220
tttttttcgg attatcggtt tttttagttg tttttagggt ggttgaatgg gggttttata 5280
ggttagtagt tagtaaagaa aagggaggag atttggggtg ttgtagtttt ggtaatagcg 5340
tttttttttg ggagaaggag gggaattaag ttttaaggtt tagtttagat ttagatgggg 5400
agaatatttt tagagttatt tttattgatt agtacggtgg ttgagaaggg aggtaattta 5460
tttaaagtta agttgtacgt tattagttgg gtagagtttg ggattaggtt tatggttaag 5520
aattgtttta ttgtagtttg ttaggtattt tttgatttgt atttttttaa atttattaaa 5580
tgtaaattaa tatttaagtt aatttttagt tttaggagta gtagtaggag tggttttgtt 5640
tatcgttttt aattttttta tttttttttt ttttgtttaa ttatttgaac gtgtttggta 5700
ttttgatttt gagttggttg gttttttttt ttttgattcg gttggttttt ttttttttga 5760
tttatttttt aaggttcggt tttaggttgg agttgtggag tttttgtatg ttattttatt 5820
atatatatat ttatagtttt ttggtagttt atatttaatg atttggtttt gtatattttt 5880
tttttttttt tatttttttt ttataaattt ttggtgggat tatgtttttt gagggtatag 5940
atttatttta tatttttatt ttgttagtta tagtatttag tatagtgttt ggtttaaagt 6000
aattgtttaa taaatatttg ttgtatattg aagtggcgaa agagaatata tttgttaggg 6060
gtttttacga gagtggaagg gatttatttt aaaaaaaaaa aaattattag gaataagata 6120
attataaata ttggtttttt tttttagagg tagtggggag ttagttggta gaggggagaa 6180
agttattttt agggaaagtt gttagtaagg ggaaaagagt atatagaaag tttggtatga 6240
gttatagatt aggggttgtt tatagttttt aaattgggat tggagtgatt atgaggggag 6300
tagggtgttt tattaatatt tttttttttt atttgttttt attaatgttt tttatgtagg 6360
aattattatt gaggggaaat tttttttttt ttttagatat tattttatat ttaaataaaa 6420
tatttaggat aaatgtatta ttttagattt attttttttt tttatataga gttaggagat 6480
ttataatttg attttttttg tgtttttagt ttgggtgttg atttttttta gggaagttag 6540
aatggaggga tttagagttt ttttaggttt aagagtttgt gattttttgt tagttttatt 6600
tttgtaaaat tttagtgtta gttaatggat ttttatatat tttttagagg ttagttatag 6660
gttaaagtgt gtgtttttta taagatagag gttagagtaa ggtagttgta agtttttggt 6720
ttgttggagt tttttgattt gttggggttt ttgtttgttt gtatttgagt attagggttt 6780
ggttaggtaa gtaaggttga gggtggggcg tgtgtaggaa attttaagag gagaataaag 6840
aaggggggta gtttttagag gtaatttagt atttggtttt tttatttatt tacggatgtt 6900
tttaagattt taaattttat ataaaagttg aatataaaat ttgagaagga ttatggaaga 6960
ttttggtatt tgatatttaa gggatgtggg gaatgatatt atgtggggaa taaagagggt 7020
gttttttatt ttagagaggg gttattttgt aaagttttgt cgaaagtatg taaaggtata 7080
aattggatat ttgacgaata tttttgattt tagttatgat ttgtgtatat ttaattttat 7140
aatttatagt tatttggata tagaaaagta tagtgtgggt aagggtacgt gtatataaat 7200
gttagaagtt tgggtataga ttggggtaga tttcggaatt gaggtaggag gaaatagtat 7260
ttattttttt ggtttttttt tggtttgttt ttttttttta ttgtttatga agttggagtt 7320
tatattatgt ttttgaaatt gtatagagaa aattacgata tgtatttttt taagtttatc 7380
ggagttttga gataaagttg ttatttgatg tgtgtgttgg gatatttatt tttttcggtc 7440
ggtttgaggc gttttttttg gtggttttta cgttttaagg gtattcgggg gagtattcgt 7500
agttgcgtcg attcgtagtg ttatagagag aagggtacgg aaggggatgg ggacggggat 7560
tcggggtttt ttcggtcggc gtagcgcgtt tatcgggttc gtagagtcgg ttgaagtggt 7620
aggggttgta gtatacggta gggtcgtcgg cggcggcggc gaagttgtgg tagtcgtata 7680
ggggttttag ttttacggcg tgttgtaggt cgggttagcg aaagaggcgg tcgagtagta 7740
gttgcggcgg cgcgggttgg tcgtttaggc ggaggtcggc gcgcggtatt agtacgtagt 7800
cgttcggtac gtcgtcgcgg gattttatcg tttttagtag cgtgtttagc gagcgttttt 7860
tgagtcgttt tagtagcgag tacgtgacgg ttttgagttt ttgttttagt agtagtagtc 7920
gcgagcgcgt ttcgcgattt cgttcgtcgt tcgtcggttt tagggtcgtt tcggttaggg 7980
ggtcgttggc gtttcggggc gcgtcggcgg cgtttcgttt cgaaaagaga tagtaggtta 8040
tcgtttcgta gttattttcg ggtagttagt tcgggtt 8077

<210> 183
<211> 7183
<212> DNA
<213> Artificial Sequence

<220>
<223> chemically treated genomic DNA (Homo sapiens)

<220>
<221> unsure
<222> (3724, 3733)

<400> 183

tttaatgatt ttaggtggat ttgtttattt tttttagatt ttagcggttt aggatatttt 60

tatttggttt tttttttttg gtttttattt tttttttttt tttttttttt tttttttttt 120
tttttttttt tttttttttg agatagagtt ttgttttgtt attttggttg gagtgtagtg 180
gtatagtttc gatttattgt ttttttattt ttttgggttt aattaatttt tttgttttag 240
tttttggagt agttgggatt ataggcgcgt gttattatat ttgttaattt tttgtatttt 300
agtagagatg aggttttatt atgttggtta ggtcggtttt aaatttttga ttttaagtga 360
tttgtttatt ttagtttttt aaagttttga gattataggt gtgagttatc gtgtttggtt 420
tagtttttaa tttttaggtt ttatgtgttt ttagttgtaa gggtttattt agattttgtt 480
attttttttt ttatattttt ttatttagtt ttttttaata aaatttttgt atgtatgatt 540
ttattttgtg ggggtatttg ggtattttgg ttaatacgta atattttttt gagttttttt 600
tgaggttttg gaattttagg atttttgggt tttttatgtt gagttttgat tggttttaga 660
attaggattg agtaaattaa ggagtgttgg agttaggagg atatttatta tggataaaag 720
agtattttat ttatttacgt ttttttgcgg ttgttcgggg tttgggattt tatcgggttt 780
tttttttttt gttttgttgg gagttttagt tttgtttttg tttggtttgt tttttagggt 840
atgtgaaggg ggtgagtatt ttttgaaggt tttataatat tttttgagta aagtattttt 900
ttttgaattt tattcgtttt atagatgagg tttagggagg agtagtgggt tgtttttatg 960
tagttttatt gttggtggga tttggggttt ttgttttttt tgttagtttt ttttttttat 1020
tttattttgt ttttattttt tatagggacg atgagaggtg gtaagggaag gtggtggtag 1080
gtaaggaaag tggggaggtg tcggggttag gaggcgtttt tttaggttta ttttgggagg 1140
ttgggggttg ttatggtatt tttggttcgt tttagttttt ttgattaagt tcggttgttt 1200
ttttttataa aagttaaatt tattttttta ataagtttag tattgtatta aaatataatg 1260
gaatgtttgg gaaagggaat gttttgttat ttagggttag tgagttagga ggttatgttt 1320
agtttttgtg gggtttagta gtgtttttgg agttaggtag aaggtttttt cgtttcgtta 1380
tgtttttttt tttttgggga ggtagggttg agagttttta ttggaaggta ggaggtttgg 1440
gtgttgtgtt ttggggcgga tttttttgag tttgttgtag aatttaagtt tttttttggt 1500
ttcggtggtt tttaaggttt tgtggttcgt ttttatggtg ttgggtgttt attcgaggcg 1560
ttttttcgag gatagtgttt tttttgaaat ttttttagtg gaggttggtt ttttttattt 1620
tgtatttatt ttagaattaa tacgtggttg ggtgttattt ttgtttttat tggtattttt 1680
agtattttt tttttttttt ttttggaatt tttagtttt ttcgaagttt attattattt 1740
ttgttttagg ttatatattt tttttgtttt ttttttgttg ttatttttta attttttggt 1800
tatttttttt tattttttgg gatgttggtt ttgttattag tattttggga ttttttatat 1860
aggggattat tgtttatgtt ttagtttttt ggcgatttta tttttggtta ttttattttg 1920
ttatatagtt ttagggttat atcgtatttt tattattatt agaaattgta ttatttttga 1980
attttgatgt taagtttttt tttttttgat tatttttttt ttttttttag gtttttaatt 2040
ttagatattg atttttagtc gtgatagttt tttagtttta ttatttttgg tttttttttt 2100
tttttttttt tgtgtttatt tggggaattt taattttagt tgaatttagt tatttttttt 2160
aagagttgaa tttgttggga gaaattttg tggtcgggtt gatcgggttt tttttgtgt 2220
atgattataa tttcgaagtg gggtttttgt tgtttggtaa ggttggtgtg cgtggtaggg 2280
atttgttgtt ttttaaggtt ttatattttt ttgaaatttt ttattttttg ttatttattg 2340
ttggttaggt ttttgatttt atttaggaaa gggaagtata gttttttttt ttggttttta 2400
gaatattttt ataatttttt ggagtttttg tttttttatt atttaagtgg aagtttgtag 2460
atttttattt ttttttttag gtttattttt ggtatttatt aaaggatgag agtggtttag 2520
tgggtttatt tatttgttta ttattattag aaaattatta ttttggtatt tggtattaag 2580
taggtattta atatatattt tttcgtgtat aaatgaatat atattaggga tagttagttt 2640
tattaagtaa tattttaaaa ttataacgtt aataaggtat gtatttaata taaaaatttt 2700
tgataatgga aaagtataaa gaaagaaaag gtagaaatta attataattt tattatttgt 2760
taataattgt tgttaatttt tgatgtgtat gtatttgaag ttttttatat atatattttt 2820
ttttttcgaa atgatatttg gatagatata gattattatt taataattgg tgatgaatat 2880
ttttttatat ttttagatat ttttttaaat ttttttaata attgtatagt gttttattgt 2940
atggatgatt ttaatttatt taattatttt tttaatgttg gatatttgta ttttttttat 3000
atttttattt tttttattt agttaatatt tttatatata aatatatgtg tatattgttt 3060
tttttgtggt tgaattttta agtggatttg taggttaaag agtatatata ttttaaggtt 3120
tttgatttat attattaaat tataagagat aatttgtttt ggaaattaaa ttaatataaa 3180
tataattttt tattttttta agtataataa attttttttt gaagttttag aaggtttttt 3240
ttttagaaat ttttatggtt aggattattt tttttattaa tattttgttt aaatgttttt 3300
ttataggtaa ggttttgatg gtttttttat ataaagtggt attttttttt tttttttttt 3360
tttttttttg agatggagtt ttgttttgtt atttaggttg gagtgtagtg gtatgatttc 3420
ggtttatttg taattttcgt tttttgggtt taagcgattt ttttgtatcg gttttttgag 3480
tagttgggat tataggcgtt cgttattata tttggttaat tttttgtatt tttagtagat 3540
atgggggttt tattatgttg gttaggttgg tttcgaattt ttgattttaa gtgatttatt 3600
tattttgggt tttttagagt gttgggatta tagggcgtga gttattatat ttggtttttt 3660
tttttttttt attttttata ttttttttgt agttgtttag ttgtatttag tattttttga 3720
tagnttaatg gtngttatgt ttgtagcgtt cggtttttga gtttttgagt gcggcgcggc 3780
gagtttttgg cggcggtaga aggatcggag cgttaggaga gggcggatcg gggataagga 3840
ggttttcggg cgcgacgagg agagtttcgg aggaggaggc gtcgagagga tatcggggtt 3900
ttttgtcgtc gtagttgggt cgaggcgagt agtttatgcg gggagtttcg gcggttagtc 3960
gcggttaggg gaagggatat cggcggtttt tgtattagta agtagtttgt gttcgggggg 4020

ggcgagaacg cgcgattcgt ttggggtttt tcgtcgtcgt cgcgttattt tttttcgcgt 4080
tgttagtttc gtcgggttta gtcgtcgtcg tagtattttt tgtttttgcg ttcgcgttag 4140
tttcgaggaa gttttcgtcg aggatttggg tttttaggag cgtaggggga aagattagag 4200
atttttttaa attatttaga tgcgtaggat tgaagcggtt tagttaaagt tttttgtgga 4260
ttaaaaaaat atacgatttt tttttttttg gtagaagaaa aggagaggaa gattagcggg 4320
gttttgtaag gaaacggggg gatgtaattc gtggatatat ttttttattt attaaatatt 4380
ttgttatatt ttgtaaatat tttttttttt taaattttag ttttagtcgg acgtttttag 4440
atttttgagg ttcgaggagg tggtggtttt tatttggggt tttgtatatt tggttgttag 4500
gttttgcgag agttttttta ttttgttaga cgttttatgc ggggtgaagt ttattcggtt 4560
tttttttttg agttttcgtg tgtacggaat tcgaggagat tcggttatta aggatataga 4620
ggaaaaaaat aaatcgtttg tttttttttt tttaattgtt tgtttttttt tatttttaaa 4680
ttaagttgtt tagtaagggg gaaagaggtt tttttttttt tttaatagtt tagtcgaacg 4740
tttttcgttt tttgttttcg cggatttttt atgtaggaag tcgaggttgg cgagttcgat 4800
attcgggagt tattgcgggg gggttttttt tgggggaggc gtcgacgggg gtaggttcgg 4860
tcgtttttag ggaagcggcg gtcgggtttt ttcggggcgc gtcggggtcg gagagtcgcg 4920
tagggcgcgg gtcgcgcggg gtggggtagt cggagcgtag gttttcgatt ttcggcgggc 4980
gttttcgggt tttcgcgcgc gtttcggttt tcgggagatt ggcgtatgtt acggagcgtt 5040
tttcgggtcg tcgtcgtttt tgttcgggtt tttgttgttg ttgttgtcgt ttgcgtttgt 5100
tgttttaatt cggcgttcga tttttttatg gtgtgcggag gttatgttcg ttttttagta 5160
ggtaaacgat tttttttttc gttttttcgt ttcgtatgtt taggattaaa cgatttgcgt 5220
tcgttcggcg tttttggagg agtcgtgcgt tcggcggcga ggacgaggag gagggcgtag 5280
ggggaggtgg aggaggaggc gagttgcggg gagaaggggc gacggatagt cgagcgtatg 5340
gggtcggtgg cggcggttcg ggtagggttg gatgttgttt gggtaaggcg gtgcgaggtg 5400
ttaaaggtta ttattatttt tattcgttag tcgcgggcgt cggcgcggtc gggggcgtcg 5460
aggcggattt gaaggcgttt acgtattcgg tgtttaagaa attgaaggag cggtagttgg 5520
agttgttgtt ttaggtcgtg gagtttcgcg gcgggacgcg tatcgcgtgt tttttgttgt 5580
tcggtcgttt ggattgtagg ttgggttcgg gggcgttcgt cggcgcgtag tttgcgtagt 5640
cgttttcgtt ttattcgttt ttttttttgt tgtgtaaagt gtttaggtgg tcggatttta 5700
ggtatttttc ggaagttaag aggttgtgtt gttgtgaatt ttacgggaag attaatttcg 5760
agttggtgtg ttgtaatttt tattatttta gtcgattttg cgaattaggt gagaagttgt 5820
ttttttattt ttaatatttt tcggagcgtt agtgttggaa gaaaagtttt tagggtagtg 5880
cgtttgtgta gttatttttt tagggggagt gtgtatattt ttttgggaga gtgtgtatat 5940
ttttttggga gagtgtgtat atttttttgg gaagagattg ggggttgaaa gtagagaagg 6000
gataagggga aatttaaagg gtattttttg ggggtttttt tttaggtttt tttttgtgtt 6060
gtttcgaagg ggtttacggg tattgttttt gtagtttgcg gttaaaaatt agacgttttt 6120
attttcggat taataagggg gtgtagaggg aggtgggggg gttacgtttt ttcgtattcg 6180
gaaagacgtt gaggtgtgtg gtcgagttgg atttggttta ggttcgttaa ggaaggttgg 6240
gagaggcgtg ttataaaggt tggaaggttt tcgtatttat ttttttagta aatacggaga 6300
attacgtcga atattagtgg tttagatatt gtcgtggtcg cgtatttttg gagttttggg 6360
gtaaagagag ttggatggaa ggtcgaattg ggtatttgtt tttttttaat tttgttagtt 6420
ttagggtgta gaggaataaa taggtaggag aagttgggtg ttttttattt tttttttttt 6480
ttcgtataga taattttgta gattagtggg gtttcggcgt tttagtgacg ttgtcgtttt 6540
ttattgcgtt ggggtttggg tttttcggtt gagattttga aatttttttt tttttatttt 6600
tttagttttt tttatttttt tttttatttt acgtttttgt atatagagtg tgatgtgagg 6660
gtttatttcg ggcgttcgta gattttttac gttttttagt acgtttggga tgtatttttc 6720
gagtgcgtta ggaatgggta gtttcgtgtt tttcgcgcga gtgtcgagcg gatcggcggg 6780
ggtagaggtg aattgtacgt ttggggggcg ggtcggggcg gggagtcgag gttgttggga 6840
gttgggtttg gagttcgagg acggggaggg ggtatggtcg tttttcgttt tgtgaattgt 6900
gaagtgcgcg gtttcggtcg agtcggtggt ttttagtcgg gttagtcgag cgcggcggtt 6960
gcggtcgtag acgtggagga gcggagcgtc gaggttggcg ttaggcggtt ttttttgttt 7020
atttgatagt taaatattaa ggtaattatc gtttcgcggt tttgttttta atttttatag 7080
ttaagataaa tagttgggtt aaaagaatgt ttttgttatt ataagttttt attttttttt 7140
ttatggttgg gtttttattt ttttttgttt ttttaatttt aga 7183

<210> 184
<211> 7183
<212> DNA
<213> Artificial Sequence

<220>
<223> chemically treated genomic DNA (Homo sapiens)

<220>
<221> unsure
<222> (3451, 3460)

<400> 184

tttgaaatta aaaaagtaag agaaaataaa ggtttagtta tgagaagaga gatagaaatt 60
tatgataata gaggtatttt tttagtttaa ttgtttgttt tagttgtggg ggttggaggt 120
agggtcgcga ggcggtgatt gttttgatat ttagttgtta gataaataaa agaggtcgtt 180
tggcgttagt ttcggcgttt cgttttttta cgtttgcggt cgtagtcgtc gcgttcggtt 240
ggttcggttg gaaattatcg gttcggtcgg agtcgcgtat tttatagttt atagggcggg 300
gggcgattat gttttttttt cgttttcgga ttttagattt agtttttagt aatttcggtt 360
tttcgtttcg gttcgttttt taggcgtgta atttattttt gttttcgtcg gttcgttcgg 420
tattcgcgcg ggggatacgg ggttgtttat ttttagcgta ttcgggagat gtattttaag 480
cgtgttgggg aacgtggggg atttgcgggc gttcggggtg aatttttata ttatattttg 540
tgtatagaga cgtagagtaa aggaaggaat ggagaaagtt gagaggataa aaggaaagga 600
gttttagggt tttagtcgag aagtttaggt tttagcgtag taagaggcga taacgttatt 660
gaggcgtcgg ggttttatta atttatagag ttatttgtgc ggaaggaagg aaaaagtagg 720
ggatatttaa ttttttttgt ttgtttgttt ttttgtattt tggggttggt agagttgaaa 780
ggagatagat atttagttcg gtttttattt taattttttt tgttttaaaa ttttaaaggt 840
gcgcggttac gatagtattt gggttattgg tgttcgacgt gatttttcgt atttattaaa 900
ggagtaaata cggagatttt ttagttttta tagtacgttt tttttagttt tttttggcgg 960
gtttggatta aatttaattc ggttatatat tttaacgttt tttcgggtgc ggggaggcgt 1020
gattttttta tttttttttg tatttttttg ttgattcgga aatgaaggcg tttaattttt 1080
aatcgtagat tgtaggagta gtgttcgtag attttttcgg ggtaatatag gagaaaattt 1140
ggaagggaat ttttagggaa tgttttttga gttttttttt gttttttttt tatttttagt 1200
ttttagtttt tttttaggag ggtatgtata tttttttagg agggtatgta tattttttta 1260
ggagggtatg tatatttttt ttggagggat ggttgtataa acgtattgtt ttaggggttt 1320
tttttttagt attggcgttt cggaaaatgt tgggggtagg gggataattt tttatttagt 1380
tcgtagagtc ggttaaggtg atgggggttg tagtatatta gttcggggtt gattttttcg 1440
taagatttat agtaatatag ttttttgatt ttcgaggaat gtttgagatt cggttatttg 1500
aatattttgt atagtaggag ggggagcgag taggacgagg gcggttgcgt aggttgcgcg 1560
tcggcgggcg ttttcgggtt tagtttgtag tttaggcggt cgggtagtag gaggtacgcg 1620
gtgcgcgttt cgtcgcggga ttttacggtt tggagtagta gttttagttg tcgttttttt 1680
agttttttga gtatcgagtg cgtgagcgtt tttagattcg tttcggcgtt ttcggtcgcg 1740
tcggcgttcg cggttggcgg gtggggatgg tggtgatttt tggtatttcg tatcgttttg 1800
tttaggtagt atttagtttt gttcgggtcg tcgttatcgg ttttatgcgt tcggttgttc 1860
gtcgtttttt tttttcgtag ttcgtttttt tttttatttt tttttgcgtt ttttttttcg 1920
ttttcgtcgt cgggcgtacg gtttttttag agacgtcgga cgagcgtaga tcgtttggtt 1980
ttgaatatgc ggggcgagga ggcgaggaga aaagtcgttt gtttgttaag gagcgaatat 2040
gattttcgta tattatgaag aagtcgggcg tcgagttggg gtagtaggcg taggcgatag 2100
tagtagtagt aggggttcgg gtaggagcgg cggcggttcg aggggcgttt cgtggtatgc 2160
gttagttttt cggaggtcgg ggcgcgcgcg ggggttcggg ggcgttcgtc ggggatcggg 2220
ggtttgcgtt tcggttgttt tatttcgcgc ggttcgcgtt ttgcgcggtt tttcggtttc 2280
ggcgcgtttc ggaggaattc ggtcgtcgtt tttttgggga cggtcgagtt tgttttcgtc 2340
ggcgtttttt ttaaaagagg ttttttcgta gtggttttcg aatgtcgggt tcgttagttt 2400
cggtttttta tatggaaggt tcgcgggggt aaaaaacgaa aggcgttcgg ttgggttgtt 2460
ggaagaagga aaaagttttt tttttttttg ttaagtaatt taatttgggg gtggggagaa 2520
gtaggtaatt aaaaaaaaaa aagtaagcga tttatttttt ttttttatat ttttagtaat 2580
cggatttttt cgaatttcgt gtatacgaag atttagggga gggggtcgag tggattttat 2640
ttcgtatgag acgtttggta aaataagaag gttttcgtaa aatttaataa ttaaatatgt 2700
aaagttttaa atgaaaatta ttattttttc gaattttaga ggtttggggg cgttcggttg 2760
gaattggggt ttaaaaaaag aaaatgttta taaagtataa taagatgttt gatgggtgga 2820
aaaatgtatt tacgagttat attttttcgt ttttttgtaa agtttcgttg gttttttttt 2880
tttttttttt tgttaaaaaa aaaaaaatcg tgtatttttt taatttatag aaagttttgg 2940
ttagatcgtt ttaattttgc gtatttgggt ggtttagggg agtttttggt tttttttttt 3000
gcgtttttgg gggtttaggt tttcggcggg gattttttcg aggttggcgc gggcgtaggg 3060
gtagaagatg ttgcggcggc ggttgagttc ggcggggttg atagcgcggg ggagggtggc 3120
gcggcggcgg cggagggttt tagacgggtc gcgcgttttc gtttttttcg ggtataagtt 3180
gtttgttagt gtaggggtcg tcgatgtttt tttttttggt cgcggttggt cgtcgaggtt 3240
tttcgtatgg gttgttcgtt tcgatttagt tgcggcggta ggaggtttcg gtgttttttc 3300
ggcgtttttt ttttcgagat ttttttcgtc gcgttcggga gtttttttgt tttcggttcg 3360
tttttttttg gcgtttcggt ttttttgtcg tcgttagggg ttcgtcgcgt cgtatttagg 3420
ggtttagggg tcgggcgtta tagatataat nattattagn ttgttagggg gtgttgagtg 3480
tagttaggta attgtaaaaa ggatgtagag gatgaaaaag aaggagaggt taggtgtggt 3540
ggtttacgtt ttgtaatttt agtattttgg gaggtttaag gtgggtggat tatttgaagt 3600
taggagttcg agattagttt ggttaatatg gtgaaatttt tatatttatt aaaaatgtaa 3660
aaaattagtt aggtgtggtg gcgggcgttt atagttttag ttatttagga ggtcgatata 3720
ggagaatcgt ttgaatttag gaggcggagg ttgtaagtga gtcgagatta tgttattgta 3780
ttttagtttg ggtgatagag taaaatttta ttttaaaaaa aaaaaaaaaa aaaaaggaga 3840

ggtgttattt tatatggaag agttattaag attttgttta taagggggta tttaagtaaa 3900
atgttgatgg aaaggataat tttagttatg aggatttttg agggaggagt tttttggagt 3960
tttagaaggg gatttgttat gtttggaaaa gtaggagatt gtgtttgtgt tgatttggtt 4020
tttaaaataa attgtttttt gtgatttaat aatataaatt aaaagtttta aaatatgtgt 4080
attttttgat ttataagttt atttaggaat ttagttatag ggaaaataat gtatatatat 4140
atttatgtat aaggatattg attaaatagg gaagataaaa aatatggaaa gaatatagat 4200
gtttaatatt aaggaagtgg ttaagtaaat taagattatt tatataatgg aatattgtgt 4260
aattattaaa aagatttaga agaatatttg aagatgtagg aaaatgttta ttattaattg 4320
ttagatagtg atttgtattt atttaagtat tatttcgaga agaaaaaata tatgtgtagg 4380
aaattttaaa tgtatatata ttaaaagttg atagtagtta ttaataagtg atagaattat 4440
ggttaatttt tgtttttttt tttttatgt tttttattg ttagaagttt ttgtattgag 4500
tgtatatttt gttaacgttg taattttaaa atattattta gtaaagttaa ttgtttttaa 4560
tatatattta tttatgtacg aagaggtatg tattgagtgt ttatttggtg ttaggtgtta 4620
ggataataat tttttagtga taatgaataa gtaaataaat ttattaagtt atttttattt 4680
tttgataagt gttaggaatg ggtttggggg aaggggtgaa ggtttgtagg tttttatttg 4740
gatggtggaa agatagaggt tttagggagt tgtgagggta ttttggaggt tagagaagga 4800
agttgtgttt tttttttttg ggtgaagtta gaggtttggt taatagtgag tggtaggggg 4860
tgagaggttt taggaaagtg tgaagttttg agggatagta gatttttgtt acgtatatta 4920
gttttgttag gtagtaagga ttttatttcg aggttgtgat tatgtataga aggggattcg 4980
attagttcgg ttataggggt ttttttagta aagtttagtt tttgggggaa atagttgagt 5040
ttaattaagg ttgagatttt ttaaatagat ataggagaag gaggaaagag gagttaaaaa 5100
tggtgaggtt ggaaaattgt tacgattgaa agttaatgtt tagagttaga gatttgggaa 5160
ggaaggaggg gtggttagag aaagagaagt ttgatattag ggtttagagg tggtgtagtt 5220
tttggtggtg atgagggtac ggtatgattt tgggattgtg tgataggatg aggtggttag 5280
aagtgaggtc gttaagggat tgagatatgg atagtagttt tttatgtgga aagttttaag 5340
atgttagtga tagagttagt attttaagag atggaggaga gtggttagga agttaggaag 5400
taatagtaag gaggggatag agagagtgtg tggtttgggg tagggtggtg ggtgagtttc 5460
ggaaagagtt gggggtttta aaaaaagagg gaggagagat gttggaagta ttagtgggag 5520
tagaaatggt atttaattac gtgttggttt tgagatagat gtagagtaga gaaaattaat 5580
ttttattggg aaggttttag ggaaagtatt gttttcgagg gagcgtttcg ggtgaatatt 5640
tagtattatg gaggcgagtt atagggtttt gggggttatc gagattaaag aagaatttaa 5700
gttttgtaat agatttagag agattcgttt tagggtatag tatttaggtt ttttgttttt 5760
tagtaggggt ttttagtttt gttttttagg aggagaagag gtatggcgga gcgggggggt 5820
tttttgtttg attttagagg tattgttgga ttttatagag gttgagtatg gttttttgat 5880
ttattggttt tagatgatag aatatttttt tttttaaata ttttattgtg ttttaatgta 5940
gtgttggatt tgttaaaaga gtaaatttag tttttatggg aagaggtagt cgggtttggt 6000
tagggaggtt aaggcgggtt aggggtgtta tggtaatttt tagtttttta agatgggttt 6060
gagaagacgt tttttggttt cggtattttt ttattttttt tatttgttat tatttttttt 6120
tgttattttt tatcgttttt gtgagaggtg aaggtaggat ggggtgaggg agaggggttg 6180
gtagggagaa taggaatttt aggttttatt agtagtgaga ttgtatgagg atagtttatt 6240
gttttttttt gggttttatt tgtgaagcgg gtaaaattta gggaagaata ttttgtttag 6300
agggtgttgt gaggttttta agaaatgttt atttttttta tatattttaa agggtagatt 6360
agataaaagt agggttgaga tttttaatag agtagggaga agaggattcg atgaagtttt 6420
aggtttcggg tagtcgtagg ggagcgtggg taggtaggat gtttttttgt ttatggtgag 6480
tgtttttttg attttagtat tttttggttt gtttagtttt gattttaggg ttagttaggg 6540
tttagtatga ggggtttaag agttttagga ttttagagtt ttagagggag tttagaggag 6600
tgttacgtgt tagttagggt gtttagatgt ttttataaga tgggattatg tatgtaagga 6660
ttttgttagg ggaaattagg taaaagaata tggggaagga gatggtaaag tttgggtgag 6720
tttttatagt tgggggtata tggggtttga aagttaaaga ttgggttagg tacggtggtt 6780
tatatttgta attttagaat tttgggaggt tgaggtgggt agattatttg aggttaggag 6840
tttgagatcg gtttggttaa tatggtgaaa ttttattttt attaaaatat aaaaaattag 6900
taggtgtggt ggtacgcgtt tgtaatttta gttgttttag aggttgaggt aggagaattg 6960
gttgaattta ggaggtggag gaggtagtga gtcgagattg tgttattgta ttttagttag 7020
ggtaatagag taagattttg ttttaaaaaa aaaaaaaaga aagaaagaaa gaaagaaaga 7080
agaaaagaaa gaaagtgaaa gattaaggag gaaaggttag gtgaaagtat tttagatcgt 7140
tgagatttaa ggaaggtggg taagtttatt tagagttatt gag 7183

<210> 185
<211> 6467
<212> DNA
<213> Artificial Sequence

<220>
<223> chemically treated genomic DNA (Homo sapiens)

<400> 185

ttttttaagg agattaattg agtggaaatt atttttagta attgttaatg ggagttggat 60
ttaaaatcgt taatttaaag taaaaaagtt tatgtttttt gtattgttat ttagtcgggt 120
aaattgtttt ttagtgtgga attatgtata tttaaattat ggtgtatgta tgtggttagc 180
gttgtttttt ttttaagtgg gtttgtttat ttaagagatt cgaaaatgag aagttgtttt 240
tttatttagt tggggtttat tgtaaaattt aatgttagaa aaagaaaaaa gtattgtttt 300
tatgttgtaa gtgtgtgaag tgtgtttttt ttaaagaaag gatagttatg gtgttttttt 360
tttttgttta aagaaggaaa gttgtaaggg ttagtaggag gttttgtggg ggatttgggt 420
ttatgaacgt taattacgta ttgattaatg gttttaaata taggaacgtt tagtatatgt 480
ttttattttt atttttttgt agaaattaat attaaaatga aataagtagt tggagagtaa 540
agtgggaatt ggtttatgtt ggtagatgat atttgttatt aatagggtta gtttgatagc 600
ggtgttagag ttgttgtttt gttttgtttt gtgttgtgaa gtgttatgag tgaaggtcgc 660
gtttagatta ttataagtag ttttagtagt attaaaatag ttgagtaagt tttgagattt 720
tatgtgatat atttagttaa tagttatgga gttttggatt tatcgtttag aaatattttt 780
ttttggagat tgagttttgt tttgatttgt aaatgaggag ggggtgaagt ttagagagat 840
agtatgacgt gttttaagag tatatagttg atttgtgata gggatatacg atttttttat 900
aaaagtgaga ttatattata ttaaaaaatt ttggaaaatt attttaattt attatagttt 960
atatttttat tttttgttat ttttattatt ttttgagata gggttttttt ttgttgttta 1020
ggttggagtg tagtggtata attatgattt attgtaattt tcgatttata ggtttaagtc 1080
gtttttttat tttagttttt taagaagttg ggattatatg tattcgttat tacgtttaat 1140
taatttttgt attttttgta gagatggtgt tttattatgt tgtttaggtt ggttttaaat 1200
ttttgagttt aagttatttg tttgttaaag ttttttaaag tgttgggatt ataggcgaga 1260
gttattgtgt ttggttagtt tatttattta tttttaattg tgatttaaat ttttttaagt 1320
gtagtttagt tattttttta ggaatttttt tagatttttc gtttttttag gttgggttta 1380
tttattgttt gttttatttt tttttgtatt ttttggaaat gtagtaattg agagaaatat 1440
taatcgtttt ttttgttttt gtaattagtt ttttttttag tgaatgtttt gagggtgttt 1500
tgttttgtta tggtgaattt gggtcgtgga gttattagga gatgagagta ggttagagtt 1560
tagagtttgt gggtatttta gttttttgat aatggaaagt tgttcggagt taggttgata 1620
taattgaata aatttatatt gttaatgtta gggcgatgat tatttggaga taaataggga 1680
ggttttttgt tttattagtg gttaaaatat tataggaatg agaatgaata atttttatga 1740
attttaaagt gtattgtttg gataaataga aataatttag ataaggaaat ggggtttttg 1800
ttatttaggt cgtagtgtag tggtgtagtt atagtttatt gtagagttaa atttttgggt 1860
ttaagtgatt tttttatttt agttttttga gtagttggaa tttaggttgt aagttattat 1920
atttggtatg atttattttt tataattaat ttttttatta aaaatatgtt aagtatataa 1980
ataagtatag aaaataatat tttgaatatt tgtatagttt ttagttagtt taattaaaat 2040
tttttcgatt tatttttagt ttatttggag gttattgtta tgaattgggt gtttattatt 2100
tttgaagttt taagtttaat tttttggggt tttttttaat gtaagggttt gtgtttaatg 2160
ggataggagt ggtattttgt agtagtaaaa ttatagtttt ggaattgtcg tgagattatt 2220
gtaatgattt gaaatgtgtt agtgagatcg gtttgtagag attcgagatg ttttagattt 2280
tagatttatg attagttggg aattttttaa gggatgagaa agtgtggtgg ttattgaagg 2340
ttatttatta agggaaattt tagatagata gtttgaagaa aaatgtatgg tttaggagta 2400
gtgtttttta aattttttta ttgtaattta atttaataag taaatttttt attgtaattt 2460
tggatatata tatcgtatat atatatatat atatatatat atatatatat atatatagta 2520
tatatttgtg aatgaaaatt ttttatgaaa tgattattat tatatttatt tttcgttagt 2580
tgttttgtaa taaagtttgt tttgttttgt tgtttgtgta aaagttgttt tgttttaatt 2640
tattgatttt atatgttatt ggtaaataga aatttatagt ttaaaaaaac gaattaaata 2700
aaatttggtt tagggaattg ttgttttatg gagtaaaagt ttttaaaagg tgtatttggt 2760
ttaattcgtt gagtgttttt ataaaaagtt tttttttatg attgatttta gtttaaagat 2820
ttatattata atttattaat aatgagtttt atgtaaaatt aagtagaatt tataattttt 2880
ttggggttta ggttatttta ttatgagatt tttttttaaa gtttttaatt ttttttagtt 2940
attgttttaa ggtagtttga ttagattaag taaatagaat ttgaatataa tgatttaatt 3000
atattttata aatttatgat tgttaagtta taaaattaat ttttttaaat aaaattgagt 3060
gtaagttaaa ttgttaagcg aaaagagtag ttatggagtt tttaattttt ttttttgtta 3120
tagtttattt ttgtataatg ttagataaag tttagatgag gaataaagtg atgtgattat 3180
tgttttatat ttattaaatt ttattcgtta ttgtgggatt atgttgttat tttttttatt 3240
tgaggtgttc gataatgatt attttagcgg ggggttataa ggtatttttg ttcgaaagaa 3300
gtttatagaa ttggatttag aaaggtatag tttgtttttt agggaagtaa aggagaaaat 3360
tatatttaat gtatattaaa taaataattg ttggagatta taagttatat cgattttaag 3420
attttaaaat atttgagtta gtttatttta aaatgttatt agttagttat tttaaatatt 3480
aatgaggtag ttggttttta aaatttggaa ggaggaggag gttttgttta agttaattat 3540
tttatagaag aggaaatata gttcgatttt aggaaattta aatgagattg ttgaataatt 3600
gggaaagaat attttttgtg gatgataggg taaagaagaa ttaagttgat tttaggttaa 3660
ataataaatg tgatagttgt gtttttttgt gtatgtattg gtgtgtgata gtgtataatt 3720
tttagaaaag tagtttttat gtatttgggt tttgaaaatt tttattttta aaaattattg 3780
agaattttaa agaatttttg tttttgtagg ttgtatttat taatttttat tattttaaaa 3840
attgaaatta agaaattaaa aaaatattga ttgaattatt aaaaaaattg tattaatata 3900

aaatattttt atttaaaata ttgtttttaa aaaagttatt gagaagagta ttattgtttt 3960
atattattgt aaatttattt aatatttggt ttaataaatg gtatttagat tgttattttt 4020
tttttatata ttttattgtg ttatgttgtt ttggttgaag tatgtgaagc gaagttagtt 4080
tatataaata agtaattgga aaagagagaa ttttatagtt tttttaaaag gattttaggg 4140
gatttttagg gatttttggg gtatattttg agagttattg ttttgaaaag tagtttggaa 4200
gattatgtta agaatggttg tttaggagtg gtgagattat ttacgatttt tttttttttt 4260
tttttttatt agtatttatt ttttataaga agatggattg tttgtgtaaa ttaaaaatga 4320
gaaagaaaga atatatttat gttgagggta gattatggag gttttggaaa aatttaggta 4380
gaggagttta tatttgggag attaaggagt tattgttatt ttttaaatgg gaaaatgata 4440
agagttgtgt ttgggagtaa gtgaattcga attgaaagag agaaagagag atgttatttt 4500
agagaagtgg tgtggtgagt tagattttag gtgttggtag aaatggagag aaaggataaa 4560
ttttaaagat tttgagggta gaatgatttg agatttggat ataggaaata taggagaggg 4620
tagagttaaa gataatagtt ttaaaattaa aatatttgaa gaatgttttt ttttttttat 4680
ttaattttgg ggtttggttt tgttgtttag gttggagtgt agtggcgtag ttttgatttt 4740
ttgggttcga gttatttatt tatttgagta gttgggattg taggtgtaga ttgttttttt 4800
ttatttaatt ttaatttttt ttgaatgtta tttcgattaa gttaattgaa gttttgtcgt 4860
gttttttaga gttggttatt ttgtttaagg gtttaggaga aagttaatat atttcgtaat 4920
ttgaattggt tttagttgtt tttagaagaa cgggcgggtt ggtttttatg ttatttttgg 4980
agagttattc gtcgtttatt ttgtcgtgaa gggttgtgcg gttttcgtgc gcgtcggagt 5040
ttgttgtggt tttttatgta ttttattatt tttattagtt tttttttttt ttttattagt 5100
ttcgtagtga agagattgtt aggttggaag taaggagatg aagaggaaaa gtgggtagag 5160
aaggtagtgg attttttagt gaagaagtta aagaagaaga agggagttat ggacgagttg 5220
gagagggttt ttagttgttc ggggtagttt agtaaatgcg ttacgatttt tcgttttttg 5280
gacgggcggt tgtaggtgtt ttatcgtaag ggtttgtttt atgtgattta ttgtcgcgtg 5340
tggcgttggt cggatttgta gttttattac gagttgaagt cgttggagtg ttgtgagttt 5400
ttatttggtt ttaagtagaa agaagtgtgt attaattttt attattatcg tcgggtggag 5460
attttaggtg ggtcgttttt acgacgttaa ggtttatttt tagagtgaag gtcgtgtgtt 5520
ttattgatgg agaatgatgg ggtattagta gatagggatt ttttttaaaa gagagagaga 5580
gaagagagga gagggaggtt tagttttgag ggattcgttt aaaattttgt ttttcggaat 5640
gaggtagttg tttaattaaa gaaagttttt atgaaaacga tatttttgtt tggaatgtta 5700
taggaatatt taatttaaag agaatcgtag taaaattttg ttatagttag tgtgaaggaa 5760
tgaagataaa ttagttagga aaatttagaa agaagatatt tttatttgat tttattatgt 5820
tggtaagttt tgtgtgattt tgaaaatgag gtagatggtt aatcggagaa aattgttata 5880
aaagttttat tttgaggtat ttgggataag ttaatataat tttttattag tttttggggt 5940
tttgaaaggt tttaaaatgg ttttgataat gatgagttgt aaaaattaaa tatttttttt 6000
ttttcgtttt aaataattga attgaattgt attaaattga attaaggtag ggtttttggt 6060
tgttattttg tttaagttta ggtaagtaga agatatttta tgtttaggtt tgaaaatagt 6120
taatgaaaga gttgttggaa attattttag gtagttattt ttaaaaatgg ttttaagtat 6180
agataggtat tatttaatga tggaaatata ttttgagaaa tgtattagta agtaattttt 6240
tttttgtgtg aatattttgg agtgtattta tataaattat gagtttgtta tatcgttagg 6300
ttatatggtg tagtttattg tttttaggtt attaatttgt atagtatgtt attgtattaa 6360
atattgtagg taattttaat tataatggta ttgaatgtat atgaatagat ttaaatatag 6420
aagaggtata gtaaaagtat agtattataa ttttggggga ttattgt 6467

<210> 186
<211> 6467
<212> DNA
<213> Artificial Sequence

<220>
<223> chemically treated genomic DNA (Homo sapiens)

<400> 186
   atagtggttt tttaagatta tgatattgta tttttgttgt atttttttta tgtttagatt 60
   tgtttatata tatttaatat tattgtggtt aggattgttt atagtattta gtatagtagt 120
   atgttgtgta ggttagtagt ttaggagtaa taggttatat tatatagttt aacggtgtag 180
   taggtttatg gtttgtgtaa gtatatttta gggtgtttat ataaggaaga aattatttat 240
   tgatgtattt tttagaatgt atttttatta ttaagtgata tttgtttgta tttaaaatta 300
   tttttaaaaa taattgtttg gaatgatttt tagtaatttt tttattggtt gtttttagat 360
   ttagatatga gatatttttt atttatttga atttgaataa agtagtagtt aaaaattttg 420
   ttttagttta gtttagtata gtttagttta gttgtttggg gcggggggag gaggatgttt 480
   aatttttata gtttattatt attaaggtta ttttaaaatt ttttaggatt ttaagaattg 540
   gtaaaagatt atgttgattt gttttagata ttttaaaata aaatttttat ggtagttttt 600
   ttcgattagt tatttgtttt atttttaaaa ttatataaag tttattagta taatgaaatt 660
   aaatgaaaat attttttttt tagatttttt tgattagttt atttttattt ttttatattg 720
   gttataatag ggttttattg cgattttttt tggattgagt gtttttgtgg tattttaggt 780
   aggaatatcg tttttatggg aatttttttt gattggatag ttgttttatt tcgggaaata 840
   aaattttgga cgagtttttt aaaattgaat tttttttttt tttttttttt tttttttttt 900
   tgggagaggt ttttgtttgt tggtgtttta ttatttttta ttaatggggt atacgatttt 960
   tattttgaaa ataaattttg acgtcgtaaa gacgatttat ttggagtttt tattcggcgg 1020
   tagtggtaag ggttaatgta tatttttttt tgtttggagt taaatgggaa tttatagtat 1080
   tttagcggtt ttagttcgtg gtgggattgt agattcggtt agcgttatac gcgatagtaa 1140
   attatatggg gtaggttttt gcggtgggat atttgtagtc gttcgtttag ggagcgggga 1200
   atcgtgacgt atttgttggg ttgtttcggg tagttgagag ttttttttag ttcgtttatg 1260
   gttttttttt ttttttttaa tttttttatt agagagttta ttgttttttt tgtttatttt 1320
   ttttttttat ttttttgttt ttagtttagt agttttttta ttgcggggtt ggtgaaggag 1380
   aagagggagt tgatgggggt ggtggagtgt ataagaggtt atagtaggtt tcggcgcgta 1440
   cgggaatcgt atagtttttt acggtaaagt gggcggcgag tagtttttta ggggtggtat 1500
   agggattaat tcgttcgttt ttttgggagt agttgggatt aatttaagtt gcgaagtgtg 1560
   ttgatttttt tttaagtttt tgaatagggt ggttaatttt ggaaaatacg ataagatttt 1620
   aattagttta atcggagtaa tatttagaaa aagttgaagt tggatagaaa gaggtaattt 1680
   atatttatag ttttagttat ttaagtgggt gaatggttcg agtttaggag gttaaggttg 1740
   cgttattgta ttttagtttg agtaatagaa ttaggtttta aagttggata gaagggaaaa 1800
   aatatttttt aaatgtttta attttaaaat tgttgttttt gattttgttt ttttttgtat 1860
   tttttatatt taggttttag gttattttgt ttttaaagtt tttggagttt gttttttttt 1920
   tttatttttg ttaatatttg aggtttgatt tattatatta tttttttgaa gtagtatttt 1980
   tttttttttt ttttaattcg aatttattta tttttaaata tagtttttat tattttttta 2040
   tttaagaaat gatagtggtt ttttggtttt ttaagtgtaa atttttttgt ttgggttttt 2100
   ttagggtttt tataatttgt ttttaatatg agtatgtttt ttttttttta tttttagttt 2160
   gtataagtaa tttatttttt tgtagaaaat aaatattaat gaaaagaaaa gaaaaaaaga 2220
   atcgtgaata attttattat ttttggatag ttatttttaa tatagttttt taaattattt 2280
   tttaggatag tggtttttaa agtgtgtttt aaggattttt gggagttttt tgagattttt 2340
   ttaaggggat tatgaggttt tttttttttt aattatttat ttgtgtgaat taatttcgtt 2400
   ttatatattt taattaaaat aatataatat aatagagtat atggagaaaa gatgataatt 2460
   taggtgttat ttattaagtt aagtattaaa tagatttgta gtaatataaa ataataatat 2520
   tttttttaat aatttttttg aaaataatgt tttaaataaa aatattttat gttaatatag 2580
   tttttttaat gatttaatta atattttttt aattttttag ttttaatttt taaaatggta 2640
   aaaattgata gatatagttt atagaagtaa gagttttttg gagtttttaa taatttttaa 2700
   aagtggaaat ttttaagatt taaatgtatg agaattgttt ttttagaaat tatatattat 2760
   tatatattaa tatatatata aaaaagtata gttgttatat ttgttatttg gtttaaaatt 2820
   agtttaattt ttttttgttt tgttatttat aggagatgtt tttttttagt tatttagtag 2880
   ttttatttaa attttttgag gtcgaattgt attttttttt ttgtgaaata gttggtttaa 2940
   ataaaatttt tttttttttt tagattttag aaattagtta ttttattaat gtttaaagtg 3000
   gttggttggt aatattttgg agtaagttga tttaagtatt ttgggatttt ggaatcgatg 3060
   tagtttataa tttttaataa ttgtttgttt aatatgtatt aaatataatt tttttttttg 3120
   tttttttgga gggtaagttg tatttttttg agtttaattt tataaatttt tttcgaataa 3180
   aggtgtttta taatttttcg ttagggtgat tattatcggg tattttaggt gaggaggatg 3240
   gtagtatggt tttataataa cggatgagat ttaataagta taaagtaata gttatattat 3300
   tttatttttt atttgggttt tatttggtat tgtatagaga taaattatga taaaagggag 3360
   agttgagagt tttatagttg tttttttcgt ttggtagttt gatttatatt tagttttatt 3420
   tggaaaggtt gattttgtgg tttaataatt atgaatttat aaagtatagt taagttattg 3480
   tgtttaagtt ttgtttattt aatttagtta ggttgtttta aagtaatggt tgaagagggt 3540
   tagaagtttt aaggagaagt tttatgatag aataatttga attttaaaag agttgtgaat 3600
   tttgtttagt tttatatgga atttattatt agtaagttgt ggtatagatt tttaagttga 3660
   ggttagttat agagaaaagt tttttataaa aatatttagc gagttaaatt aaatatattt 3720
   tttagaaatt tttgttttat gaaataatag ttttttaaat taggttttgt ttggttcgtt 3780
   tttttaaatt gtgggttttt atttattaat ggtatgtgaa attaatgaat tggaataaaa 3840
   tagtttttgt ataaataata aaataaaata gattttattg tagagtagtt gacgaaaaat 3900
   aagtataata ataattattt tatgaaaaat ttttatttat aggtatgtgt tgtgtgtgtg 3960
   tgtgtgtgtg tgtgtgtgtg tgtatgtgtg tgcggtgtat gtgtttaggg ttataatgga 4020
   aaatttattt gttaagttgg gttataataa aaaagtttga aaagtattat ttttaaatta 4080
   tatatttttt tttaaattgt ttgtttgagg ttttttttga tgagtgattt ttagtggtta 4140
   ttatattttt ttattttttg gggaattttt aattagttat gaatttaggg tttgaaatat 4200
   ttcgggtttt tgtagatcga ttttattgat atattttaaa ttattatagt ggttttacga 4260
   tagttttaaa attgtgattt tattgttgta agatattatt tttgttttat taaatatagg 4320
   tttttgtatt gagggaggtt ttaaagagtt aaatttagaa ttttaagagt gataaatatt 4380
   taatttataa tagtggtttt taggtaggtt agaaatagat cgaaaagatt ttaattaggt 4440
   tgattggagg ttatatagat gtttaaaata ttatttttta tgtttgtttg tatgtttggt 4500
   atatttttaa tagaaaaatt aattgtaaaa aatgaattat gttaggtgtg gtggtttata 4560
   gtttgggttt tagttattta ggaggttgag gtgggaggat tatttgagtt tagaggtttg 4620
   attttgtagt gagttatgat tgtattatta tattacggtt tggatggtag agattttatt 4680
   tttttgttta ggttgttttt atttgtttag gtagtgtatt ttagggttta taaaggttat 4740
   ttatttttat ttttataatg ttttagttat tagtagaata aagggttttt ttgtttgttt 4800
   ttaggtggtt atcgttttga tattaatagt gtaagtttat ttagttgtgt taatttggtt 4860
   tcgaatagtt ttttattgtt agagaattga aatgtttata gattttaagt tttaatttat 4920
   ttttattttt tgataatttt acggtttagg tttattataa taaaatagag tatttttaga 4980
   gtatttattg aaaaaaaaat tggttgtaaa aataggaggg gcgattaata ttttttttag 5040
   ttattatatt tttaaaaagt gtaaagaaga atgaaataaa taataagtga gtttaatttg 5100
   agggggcgga aagtttggaa agatttttga gaggataatt gggttgtatt taaaaggatt 5160
   taaattatag ttaaaaataa ataaataaat tggttaggta tagtggtttt cgtttgtaat 5220
   tttagtattt tgggaggttt tggtaggtag atggtttgag tttaggaatt tgagattagt 5280
   ttggataata tggtgaaata ttatttttat aaaaaatata aaagttaatt gggcgtggtg 5340
   gcgggtatat gtagttttag ttttttgaaa ggttgaggta ggaggacggt ttgagtttgt 5400
   gaatcggaag ttgtagtgag ttatgattgt gttattgtat tttagtttgg gtaatagaga 5460
   gagattttgt tttaaaaaat aataaaaata ataaaaaata aaaatataaa ttgtggtgga 5520
   ttaggataat tttttaagat tttttggtgt ggtatagttt tatttttgta agaaaatcgt 5580
   gtatttttgt tatagattaa ttgtgtgttt ttagggtacg ttatgttgtt tttttggatt 5640
   ttattttttt tttatttgta gattaagata aggtttagtt tttagggaag ggtgtttttg 5700
   agcgatgggt ttaggatttt atggttgttg gttggatgtg ttatatggga ttttaagatt 5760
   tgtttagttg ttttgatgtt attgggatta tttgtagtag tttgagcgcg atttttattt 5820
   atgatatttt atagtataga gtagggtaaa ataataattt tgatatcgtt attaaattgg 5880
   ttttgttaat aataaatatt atttattaat atgaattaat ttttattttg ttttttagtt 5940
   atttgtttta ttttagtatt aatttttgta aaggaataag agtaaaagta tatattgggc 6000
   gtttttgtgt ttgaaattat tggttagtgc gtgattagcg tttataagtt tagatttttt 6060
   ataaagtttt ttgttggttt ttatagtttt ttttttttgg gtagagagaa aaaatattat 6120
   gattgttttt tttttggaaa ggatatattt tatatatttg tagtataaaa atagtatttt 6180
   tttttttttt tggtattgag ttttataata aattttaatt gagtaagaaa ataatttttt 6240
   attttcgagt tttttaaata aataagttta tttaaaagaa aagtagcgtt gattatatgt 6300
   atatattatg atttaagtgt atatgatttt atattaaaaa ataatttatt cggttgggta 6360
   atagtatagg agatataagt ttttttattt taagttgacg gttttaaatt tagtttttat 6420
   tagtagttat tgaagatgat ttttatttag ttggtttttt tggaagg 6467

<210> 187
<211> 5964
<212> DNA
<213> Artificial Sequence

<220>
<223> chemically treated genomic DNA (Homo sapiens)

<400> 187

taaatgttaa tttgaggttt taatttttta gggaatggtt attttttatt tttttgtgtg 60
taatataaat agaagtatgt gtaataatta gtgagattag aattaattta gttgatttag 120
agtgatatgt tttagttgtg aggttgattt gtagtatata ataagagata taagtttatt 180
gtagtaaaat atggttattt tgtttttata ttaatatatg atcgtttaat taagagtata 240
aaataaattt gaattttttt atgttttttt aaatgtttag ggtaatattt gtttttttatt 300
gggatatata taaaatataa tttgaaaaga agtaaattat tgaaaaagta ttggtgaatt 360
tatttaaaga gtaaagtttt ttgaagtttg atttttttta gtttaatatt ttataatttt 420
taagcgtggt ttattgtata tgtattttat aaatgtatat aaatatatgt gtttattttt 480
atatatatat atatgtttgt tttattttta tagttttttt atgaattgtt tttattttga 540
tattttgatt gtattagttt tatttttatt attaagttag aatattagta tttttgtttt 600
ttttgagggt attttttgaa gattattatt tgtaagtttt ttaggaggtg ttatcggtat 660
tttttttgat attttaataa ataggaaaat aaatgttgtt tattaaattt tttaatgtta 720
atatttattt tatttaaaat gaatgagtta gtttttatag attttacggt atgtgttgag 780
aatttatatt tttttttata ttaatgtgtt taattttttt tttttttagg ttttagtttt 840
aggaattata atttattttg tttttttgtg ttttagtttt tttatttgta atatgaggag 900
ggtgggttta atgattgtta aattgttatt tagttataag tttttatgat ttaagaattt 960
ttgtaagttt tttagttttg ggaattgttt tcgtttttat tagaaagttt atgagtaatt 1020
tattttttgt ttagtatata attaagaagt aataataagt ataattagtt gagcggtata 1080
agttgttgtt ttgtttatgg agtagttatt ttttattttt ttattttttt aataaatttg 1140
tttttgtttt gaaaagaaaa aaaaagaatt tttagtttta gtttttttat ttgtaaaaga 1200
agaaggtgga attatgtgtg ggttttttgt ttttttttaa ttattatgaa agtaatattt 1260
gtttgttgaa aaaaattaaa tagtatatga ttttataaaa taatatggta gagttttttt 1320
tttttttttt ttattttatt tattttttta aattatgttt tagcgataat tattgtttag 1380
atttttgtta taattttaaa tatatatagg tttatttaaa tagttttgat tatattatat 1440
ttttaaatta tacgtttaaa tttaaattaa attatgtagt atattttttt gatttttttt 1500

ttaaaataaa atatgataaa tattttttta tgacgatgta tattaggtat attttttttt 1560
ttttttttt ttttttttt ttttttttt ttttttgaaa aggggtttg tttttttatt 1620
taggttaaag ggtagggggg ggatttcggt tttgaaagtt ttttttttcg ggttaaaatt 1680
atttttttgg tttaattttt taagtagttg ggattataaa tgtttttttt tttttttggg 1740
taattttttg ggtatatttt ttttttaata gttttttagg ggtttattga aggggtattt 1800
ttaatttatt tggtattttt tattgaaaat atttaaattg gttattttta tttgaaaaag 1860
ggaatttttt tttttttggg gggttttttt tttattggta ttaaatttta aattttaggg 1920
ggtatgggta taatatgtat gtttgttata tgtatatata tgggttatgt tggggggggt 1980
ggatttatta attcggtatt tatattaggg atatttttta aaggtttttt tttcggtttt 2040
ttttatttta aaaaagggtt tgggggggga tgttttttat tttgggttta agtgttttta 2100
ttgtttaatt tttatttatg aatgaaaata tgggggggtt gggtttttgt ttttgcgagg 2160
agattacgga cgttcggtgg aattttattt tagatttatg tttttatgtt cgttttgtgt 2220
tttttgtttt tcggaagttt attttataga atttttagtt ttatttcgta gacggacgta 2280
aattattttt tattttgcgt ttaagagttg gttttaatat attttagagt ttttaatttt 2340
ttttaaataa ttttcggatg aagggatatt tttaattttg tttaaaattt tttttttatt 2400
tatatttttt tgagggtgag ggtttagttc gtagttggcg agtgggacgt gggtgggggc 2460
gatggaggcg cgggcgggtt gtttggaggg agttagtttt ttggttattt ttagggcggg 2520
aatagggatt ggtagttcgt atttttcgta gatcgaagtt ttttgttgat cgattatttg 2580
ataattgatt tttttcgtag ttaatgcgcg gcggtttggg cgggggcgtt ttttgattgg 2640
tcgagtattt ttttacggtt gtttatcgag atttgggtga gtttattttt tagtcgatcg 2700
tagggggagt tttttgcgag gttttagttg gagggagggg gaaaaatttt ttcggagtcg 2760
agcgagttgc gagttttgtt tgtggatgtg aggtttttag ttattggtag agttgttgcg 2820
gttgagttat ttcggtagat tagagtttag cggatagaaa tataaatacg aaaataacgt 2880
tattaaatat aattataagg gagaagttta gagcggagaa ataaagagaa ggggtgaggg 2940
ggaattaaag tttaggtaat aggtgttaaa gttaaaaata aaataaaata aaaaatagtg 3000
gttggttgga gttttcgagt tgtaggtata ggtagttttt gtttgaaatt ttagttttcg 3060
aagcggattt tttttttttt ggtattggga ggtttcgtcg agtcgggttg gggacgggaa 3120
agaggagcgc ggggaaggat gtttggggtg gtgagggtag ggtttcgttg gagaaagagt 3180
tagtggggcg cgaggttttt ataggttcgg gagaggtcga ggttggagtt tttcggcgtt 3240
tttaagataa aggtagcggt ggttgtagtc ggagtttagt atttcggtag cgtcgcgtcg 3300
cgtcgcgtcg cgttttgggc gtacggtcgt tttatttcga gcgggtcgga gaaagagttt 3360
tttttttagc ggtttttcgg tttcggtttc gttcgcgagg tttgggttgt tgcgagttcg 3420
cgtcgggttt cgtttttcga cgattataaa tagtttggtg tttgtaaata ggcgttgggg 3480
gtatattttt cgcgtttagt ttattgtttt tttttttttt ttttatttcg cgtaggacgt 3540
ttgggttggg gttgggagtc gtcggggtta ggaggtgggg gagtttagat tcgaagtgat 3600
aaaatgttag tatttttttt ttttcggtcg ggcgtttatt tttaatagta aagaaatttt 3660
taagttgggt ttgtagaaag ttcgagttat tttaatttta tgtttttagg attttttagt 3720
agaggttttt ttaatttggt tttttttttt ttttttttta cgatttcgtt ttgatttttt 3780
tttttgtagt gtttagtttt gagaatttag tattttgcgt attatttttt gtttcgaaaa 3840
atattggtag attttaataa tttcgaggaa agttatgaag tttatgcgcg gagttttgtg 3900
taaaataatt ttcgttgttg tttgttcggc gttgattttt aatttatttt aagagagtcg 3960
gttttgggga gagagtttgt agggggaggg agagaaaaga atattgaaaa taaagttggc 4020
ggtcgcgggt tattgtttgc gtttgtgtgc gtgtgttttg ggtgtgtggt gtttgtgttt 4080
gggcgtgcga tttaatggag cgtttttttg tttttttagt gcggttagag ttcgtttcgc 4140
gtatttattt ttgtcgagga gtttatttgt tgtagtttaa tgtattgtgt aagacgcgat 4200
ttgttatggt tattattatt ttcgggtttt agtattttgg tcggaattta ttttttcgtt 4260
tgtgtaatat atattttata tacgtacggg gattgtaagc gggtagtatc gatcgtggtt 4320
tttttaagat aaatttagat agatattttt tttaattttt tttttttaat ttttttttag 4380
tgtagtattt gtaaagaggg agagagaggg agagaaagaa agagagagag agaagagaga 4440
aattgattag gaattaggat tgatttaagg gaagcgagcg ttagggtttt gtgtatttga 4500
atattaatat ttgaggtgtt ttgattagaa gaagatagag cggatgatta tttatttatt 4560
acgttgataa tttcgtttgt gattgatagt tggagtggta gaaagttatg agatttggta 4620
gttgggtttg aggggcgttt ttttttttt ttttttttt ttttttttt ttttttttt 4680
aaattgattt ttgggggaga gaagatttgt ttttttttgt tttcgttgtt gttttggaaa 4740
cggagcgttt ttatgtttag tgattcgggc gttttgtttt aggtttcgta gatcgaagat 4800
ttgggattag tagtttacgt tgttggagac gttaagggat ttttcgtcgg gttttttttt 4860
tttttttttt tttttttcgg gggagtttga atatttgttt ttttttatat tggttttaaa 4920
gaggatatat tagaagttga agtaggaagg gagttagaga ggtcgatggc gtaaagggta 4980
cgtattaaaa aataattgtg gaatttaaat gtgagattaa attgaaacgt ggtcgaaaga 5040
tattgttaaa aagttttttt tttttttttt ttttttttaa aaaatgaggt ttttaaagtc 5100
gtggtttaag cgagaggatt tatttaatgt atttgtagat ttatgtatgt tatttggagt 5160
tgttaattaa aggaatttat ttatttatag atttttaatt agtagttata taaataaaaa 5220
tgtaagttta aataattatt tgggtttttt tttaaaaaag ttagatattt aaagttgtaa 5280
tttgaatatt tagagattag aagtttaaag ttatgtttaa attaaaatat gtagttggaa 5340
atttttgata tgttattgat tatgtttatt tttaatatta ttagaagtat ttagtttttt 5400
aaaggaaata attttgttat agtatagtta attttttggt tagaaaatat ttattttttt 5460

atagtaattt taatttgtat agatttgtat ataaggtaat attttgtaag ttttttttag 5520
ataagttttt tattaattga atattaagta tatagttatt tattttaaaa atgtaatatt 5580
aattttttta tatttttttt ttaatggaaa tgtagtgaga gtaaagtgag ataatgataa 5640
tttaattgag gtagtttttt aaaagaaagg gggtttataa aaagtttttt ttgtataatt 5700
tgtgttttgg tttttaaagt tttgtttgta aaagaaagtt attaggttga tatagttttt 5760
tttatttttt tggtttaggt aaatgttagc gtttttttaa atttttttta tatttttggt 5820
tttaaggaga tgaaagtggt aatttttttt ttttttgaaa attattttat ttttagcgtt 5880
tttaaatttt gggaaaataa ttgtttggaa tgaattaatt agaaaaattt aaatagttaa 5940
attttatata tataaaataa tgat 5964

<210> 188
<211> 5964
<212> DNA
<213> Artificial Sequence

<220>
<223> chemically treated genomic DNA (Homo sapiens)

<400> 188

gttattattt tgtgtgtgtg aaatttagtt atttaggttt ttttaattgg tttattttag 60
gtagttattt ttttaagatt tggaggcgtt gagagtgaaa tgatttttaa agaaaggagg 120
aattgttatt tttatttttt taaagttaga ggtgtgaaaa gagtttggaa gaacgttgat 180
atttgtttga attaaaggag taaagaaaat tgtgttagtt tagtaatttt tttttgtaaa 240
tagggttttg agggttagga tataaattat gtaaggaagg ttttttgtag attttttttt 300
ttttaagaaa ttattttagt tgggttgtta ttgttttatt ttatttttat tgtattttta 360
ttgaaaaagg aatatgggga aattagtatt gtatttttaa agtgagtaat tgtgtgttta 420
gtatttaatt agtagagggt ttatttaaga gaaatttgta aaatattgtt ttgtatataa 480
gtttatataa attgaaatta ttataaaaag atgaatgttt tttgattaaa aaattggtta 540
tattatgata aagttatttt ttttaaaaag ttgaatattt ttagtgatat taaagatgag 600
tataattaat aatatattag aagtttttag ttgtatattt tagtttaaat ataattttaa 660
atttttgatt tttgaatgtt taaattatag ttttaggtat ttagtttttt taaaaaaaaa 720
tttagatgat tgtttgagtt tgtattttta tttatatagt tgttggttag gaatttatag 780
ataaatagat ttttttaatt aataatttta agtaatatat ataagtttat aaatgtattg 840
aataaatttt ttcgtttagg ttacggtttt aggagtttta ttttttaaaa gagagagaga 900
aaaaaaggaa attttttagt agtgtttttc ggttacgttt taatttaatt ttatatttga 960
gttttataat tgttttttaa tacgtatttt ttgcgttatc ggtttttttg gttttttttt 1020
tattttaatt tttaatatat tttttttaag gttagtgtga aaagaaataa atatttaaat 1080
tttttcggaa aaaaaaaaaa aaaaaaaaaa aggttcgacg aaaaattttt taacgttttt 1140
agtaacgtga gttattggtt ttaaattttc ggtttacggg atttgaagta aagcgttcga 1200
gttattgagt ataaaagcgt ttcgttttta agatagtagc gggggtaaaa aaaagtagat 1260
tttttttttt ttaaaaatta gtttaaaaaa aaaaaaagaa agaaagaaag aaagggaaaa 1320
aaaagagcgt tttttagatt taattattaa attttatggt tttttgttat tttagttgtt 1380
aattataggc gaggttgtta acgtggtgaa tgaatgatta ttcgttttgt ttttttttgg 1440
ttagaatatt ttaaatgtta atatttaaat gtataaagtt ttagcgttcg ttttttttga 1500
attagtttta atttttaatt agtttttttt tttttttttt tttttttttt tttttttttt 1560
tttttttttt ttgtaagtgt tgtattggaa ggagattaga gaaggagggt taaaaaaaat 1620
gtttgtttga gtttgtttta aaggagttac gatcgatgtt gttcgtttgt agttttcgtg 1680
cgtgtgtaaa gtgtgtgttg tataggcgga gaggtggatt tcgattagaa tgttagaatt 1740
cggaagtagt ggtggttata ataggtcgcg ttttatataa tgtattgggt tgtagtaagt 1800
aggtttttcg gtaggggtgg gtgcgcgaag cgagttttgg tcgtattgga gaggtagaga 1860
ggcgttttat taaatcgtac gtttaggtat aaatattata tatttagggt atacgtatat 1920
aaacgtaggt agtagttcgc ggtcgttagt tttattttta gtattttttt tttttttttt 1980
ttttgtagat ttttttttta aagtcgattt ttttgaaata aattgggaat taacgtcggg 2040
taggtagtag cgggagttat tttgtatagg gtttcgcgta tagattttat gatttttttc 2100
gaaattattg gggtttgtta gtgtttttcg gggtaggggg tgatgcgtaa agtattgaat 2160
ttttagaatt aaatattgta aggagaaaag ttaaagcggg atcgtggagg aaaaggaggg 2220
agaagttagg ttgggaaagt ttttgttaag gagttttgag aatatggggt tgaggtggtt 2280
cgggtttttt gtagatttag tttagaggtt tttttgttgt tagaggtgga cgttcggtcg 2340
ggggaaagaa aatgttagta ttttgttatt tcgggtttgg atttttttat tttttagttt 2400
cggcggtttt tagttttagt ttaggcgttt tgcgcgaagt agagaggaag ggagggaata 2460
atgagttgag cgcggggaat gtgtttttag cgtttgttta taaatattaa gttatttatg 2520
atcgtcggaa agcgaaattc ggcgcgggtt cgtagtagtt tagatttcgc gggcggagtc 2580
ggggtcgggg agtcgttggg agggaggttt ttttttcgat tcgttcgggg tgaggcggtc 2640
gtgcgtttag ggcgcggcgc ggcgcggcgc gacgttgtcg gagtgttagg tttcggttgt 2700
agttatcgtt gtttttgttt tagaggcgtc gaggggtttt agtttcgatt tttttcgggt 2760

ttgtaagaat ttcgcgtttt attagttttt tttttagcga agttttgttt ttattatttt 2820
agatattttt tttcgcgttt ttttttttcg tttttaattc ggttcggcga aattttttag 2880
tgttagagaa aaagaaattc gtttcggaag ttgagatttt agataaaagt tgtttgtatt 2940
tataattcgg aggttttaat taattattgt tttttgtttt gttttgtttt taattttaat 3000
atttattgtt tgggttttaa ttttttttta tttttttttt ttattttttc gttttaagtt 3060
ttttttttgt gattatattt agtggcgtta ttttcgtatt tatatttttg ttcgttgggt 3120
tttgatttgt cgagatggtt tagtcgtagt agttttatta gtggttggaa attttatatt 3180
tataagtagg gttcgtagtt cgttcggttt cgagaggatt tttttttttt tttttagttg 3240
aaatttcgta gaaaattttt tttgcggtcg attggaaaat gagtttattt aaatttcggt 3300
aggtagtcgt ggaggagtgt tcggttaatt aggaggcgtt ttcgtttagg tcgtcgcgta 3360
ttggttgcgg ggaaaattaa ttattaaata atcgattagt agggagtttc gatttgcggg 3420
gaatgcgaat tgttagtttt tattttcgtt ttgaaggtgg ttaaaaggtt gatttttttt 3480
aagtagttcg ttcgcgtttt tatcgttttt atttacgttt tattcgttag ttgcggattg 3540
aatttttatt ttttaagggg tgtaggtagg gaaagagttt tgagtaagat tgggggtatt 3600
tttttattcg aagattgttt gggaaaggtt aggagtttta aaatgtattg aagttaattt 3660
ttaagcgtaa agtaaaagat aatttgcgtt cgtttgcgag gtaggattga agattttata 3720
gagtgggttt tcgaggagta ggagatatag ggcgagtatg agaatatagg tttggaatga 3780
ggttttatcg agcgttcgta gttttttcgt aaggatagaa atttaatttt tttatgtttt 3840
tatttatagg tgggaattga ataatgagaa tatttggatt tagggtgggg aatatttttt 3900
tttaggtttt tttttggggt gggggaagtc ggaaggaaaa tttttaggaa atatttttaa 3960
tgtaaatgtc gagttaatgg gtttattttt tttaatatgg tttatgtata tatatgtaat 4020
aaatatgtat gttgtgttta tgttttttag aatttaaagt ttaatattaa taaaaaagaa 4080
attttttaaa aaaaaagaaa tttttttttt taagtaaaaa taattaattt aaatgttttt 4140
aataaaaggt attaaataaa ttaagggtat ttttttaatg gatttttaaa aagttgttaa 4200
aaaggaggta tgtttaaaaa attatttagg gggggggggg ggtatttgta gttttagtta 4260
tttgggaggt taagttagaa aaatggtttt aattcgggag ggggagtttt taaaatcgaa 4320
attttttttt tgttttttgg tttgggtgaa aaaataaaat ttttttttaa aaaaaaaaaa 4380
aaaaaaaaaa aaaaaaaaaa aaaaaaaaag aggtatgttt aatatgtatc gttatgggaa 4440
ggtgtttatt atattttgtt ttaaaaaaga aattagggaa gtgtgttata taatttaatt 4500
tgaatttaaa cgtataattt aaaaatataa tataattaaa attgtttgaa taagtttata 4560
tatgtttgga attataataa aaatttgaat agtagttatc gttgggatat agtttgagga 4620
ggtgaataaa ataagagaaa agagggggaa attttattat attattttat agaattatgt 4680
gttgtttgat tttttttaat aagtaaatat tatttttata ataattaaaa ggaaataaaa 4740
aatttatata tagttttatt tttttttttt atagatgaag aaattaaaat tagaaatttt 4800
tttttttttt ttttaaagta aaagtaagtt tattaagaaa gtgaaggaat aaagaatggt 4860
tattttatag gtagagtagt aatttatgtc gtttagttga ttatatttat tgttattttt 4920
tgattatatg ttaaataagg ggtggattat ttatgagttt tttgataaag acgagggtaa 4980
tttttagaat tggaaaattt atagaaattt ttgaattata gaaatttgta attgaatgat 5040
aatttagtag ttattaagtt tatttttttt atattataga taaggaaatt gaggtataga 5100
gaagtaaagt gagttatagt ttttaagatt aaagtttgaa gagaaaagaa gttgggtata 5160
ttgatgtggg gaaaaatgta ggtttttagt atatgtcgtg agatttatga aaattgattt 5220
atttatttta gatgaggtaa gtgttgatat tagggaattt ggtgggtagt atttattttt 5280
ttatttatta aaatgttaga aagagtatcg atggtatttt ttaaaaagtt tgtaggtggt 5340
gatttttaga aaatattttt aaagaggata agaatgttaa tattttagtt tgatgatgaa 5400
aataaggtta gtataattaa gatattaaag taggaatagt ttataaaaaa attatgaaag 5460
taagatagat atatatatgt atgtaaagat agatatatat atttatatat atttataaaa 5520
tatatgtata ataaattacg tttaagagtt gtagaatatt aaattagaaa aagttagatt 5580
ttaaagaatt ttattttttg aataaattta ttaatgtttt tttaataatt tatttttttt 5640
taagttatat tttgtgtatg ttttagtgga agataatatt gtttttggat atttaaagga 5700
gtatagagaa atttaaattt attttatatt tttggttaag cggttatatg ttaatatgag 5760
aatagagtag ttatattttg ttgtagtgag tttgtgtttt ttattatata ttgtaaatta 5820
attttatagt taaaatatgt tattttgggt tagttgagtt ggttttggtt ttattggttg 5880
ttgtatatat ttttgtttgt gttatatata gagaagtgga aaatgattat tttttgaaaa 5940
gttggagttt taaattaata tttg 5964

<210> 189
<211> 6027
<212> DNA
<213> Artificial Sequence

<220>
<223> chemically treated genomic DNA (Homo sapiens)

<220>
<221> unsure
<222> (2550, 4472..)

<400> 189

gttggatagt ttttttcgga tattgagaat tttaatttta aaatgatttt aatttgatat 60
cgtaagatga aattttatcg tttgttaaaa ttatttttag tttgggtaga aggagttgat 120
tgttgacgga ggtgtgtgtt attttttgcg atttttattt cgtgtttgtt tttcgggttt 180
gttttattta tttttttttt attttaaata tgggagagta gatgagtttg gaagtttggg 240
tgggagtgtt aacgttgata atggggtatt agtaaaatgt tattaggatg gaaattagta 300
agtgcgggta ttttattttt ttggaatcgt ttttattgga ttggaaaatt ggggatgagg 360
gaaattagga aatttttata tgttagatga gtttttttta gttttattaa taaaatattc 420
gtaagttgtt gattaaaggt aatagtgttt tgaatttagt agattgatta agaattaata 480
ttttgaatcg atttaatttt gatttttta tgtgtaattt attatattag gattgttatt 540
ttaaattaat agttttataa gtgatatgta attagattag taagattttt ttcgtttaaa 600
gaaattttgg gttttttttt gagataattg gtaaatttta aaaagatata gatttttttt 660
agtaggaatg tatttttaat atttttatat ttttttaatt aaaagtcgtt gaagttaaat 720
atgatttttt gataatattt taaaatgtta tatttttttt ttttttttat atttaattat 780
attgtttttg ttgtttgttt taatgtattt aataaaatgt aaaagaagtt aagaatatga 840
aagagagaga tagagaaagg cgttaaggtt aaaaatgttt tttgtatttg ataaaaatat 900
atgaaaataa agtttaaatt tttttttttt tgtaaaattt aagattatta ttagataatt 960
aatttattat tagtttataa ttgtttgaaa atatagaaat cggtcgggtg cgatggttta 1020
cgtttgtaat tcgagtattt tgagaggtcg cggcgggtgg attaggaggt taggagttcg 1080
agattagttt ggttaatatg gtgaaatttt cgtttttatt aaagatataa aaaattagtc 1140
gggcgtggtg gcgcgttttt gtaattttag ttatttaaga ggttgaggta ggagaatcgt 1200
ttgaattcgg gaggcggagg ttgtagtgag tcgagatcgc gttattgtat tttagtttgg 1260
gcgataaagc gagatttcgt tttaaaaaaa aagaaaaaaa aagaaaagaa aagaaaaaaa 1320
gaaaaagaag ataaagaaag aaaagaaaat atagaaatta ttaatttgtt tgagttgttt 1380
ttatttatat tgtaagggaa ggaatatttt tttaaaaatg cgatttaagt ttttttaaat 1440
gaaagttata gagatgttaa tttattttat agtattatta taaaaataga aaaagaaata 1500
gaaggtagta atgttgaatg tttaaatttt gtaaggtaga ggtttcgtat gaagttgatt 1560
tttataaaaa aaggtttatt aaggatattg aggaattttt tttaaaaaga aacgtaattg 1620
gtatttattg aataagatat ttaaaataat aataatttta ttggcgacga taaatttaaa 1680
tttttaaatt gaatttaaga ataattagaa gagtaaaaat tgagtataaa ttttttagtt 1740
agcggcgttg tatcgggaat ttggatttat taatattaat agttttgtaa agtcgaaaac 1800
gtttcgtagc gtttttgttt tttttttttt tttatttcgt cgtattttta aatttttcgg 1860
tttcggttaa attgaattcg cgtaattgtc gattgaaata aaattttgga gattaatttt 1920
tttttttttt ttttattttt tttttttttt tatttatttt tttttaaatg tcgacgagta 1980
atttagtaat aaataaggat atttttaatt ttggagtttt tttaggaaaa aaaagaggac 2040
gtggaggaag tatttttttt ttaattttta gattttttat tcgtcggttt tagtaggttt 2100
gaagttcggt aaaaaagagt ggttgaaaat ttgggttata tttttttttt tgaagtttcg 2160
tgagtttggt cgtttttgtt cgggttattg ggtagaaatt tcgcggttcg tagttttttt 2220
ttttggcgtc gcgttattcg cgtttttgtt agttttttcg tattattttt ttttatatgt 2280
gagggtcgat ttttcgtaga tattattgtt atgatggttt atttaattaa attgtttaat 2340
tgttttggta attattatta ttattatagg tgtttaatta tttattattt ttggagggtt 2400
ttattgattt tcggagttta ttttttagtt ttattaaaac gaggttaaat tttttcgacg 2460
tcgtttggag atttgtaggt tgtaattttt agtttagttt ttgttgttcg aaagtttata 2520
gtgaagtagg gaatatggaa cgggggttan aaaaaagagt ttttttattc gttttttttt 2580
tcgttttttt tcgttttttt tttttttttt ttttttatat ataatatata tatagagtat 2640
tttatttttt ttaattaatt ttttttattt tatttttttt tcgcgggaaa gtcggttaga 2700
cgcggttcgc ggaagatatt atttttattt ttttagaatg ggtttaaagt tggggaggga 2760
tgcggttgtt ggagaataat agttcgtttt ttattttttg ggttagggat tgggagggcg 2820
agagcgagaa gttggggaga ggacgcgttt ttagcgtttg ttcgttcggt cgcgttataa 2880
ggggtcgagg ggtatggggg ttgttgggcg gtaaggtttg cgcggggtga tcgtggtttc 2940
gtttttaatt tcgggttcgt agtgattgac gcgcggtcgt cgggaaatta gtcgcgtaga 3000
ggtgaagttt ggtgtagttc gggttaggga ggataaggta tcggttataa ttagttttta 3060
tttaaacggt gtggggtagt ttttttcgtt tttttttttt ttttggaaaa gttttttcgg 3120
agttggtttt atttttttga tttcgcgaaa tcgtttttgt gtaattaata tagttattag 3180
taggcgttat cgcgggtcgt tcgtgcgttt cgggcgatta taataaagtt gggttgtgag 3240
cgtcggttat tttttttcgt tcggtaattt tttttttttt tatttttttc gttttttttt 3300
tttgtgtttt ttagttttga gtgagattta ttttggagac gtatatataa tgttcgcgcg 3360
tacggcgcgg gcgcgcgcgt atatatatat atatatatat atatatatat atatacgttt 3420
ttggttatat cggatttaga tgattgtttt tttatttttt ttttttcgat ttcgtcgcgg 3480
tttttaaaga ttttcggggt ggttttttgt tcgtatcgtt tggagggagt gtgttttgag 3540
ttaagttggt tttttttggt tttggaaaaa aatgagtatt gataaggttg ttggatttgc 3600
gtagaaaaga aagtgttatt taataaaaaa tttagttcgg tagtggtatc gtttgtagag 3660
tttgttgttt ttggacgtta gtaggaagtt ttcggggtgt tgtaatcggc gggtagagga 3720
gagggaggtc gcggaattaa aaggaataaa agttagagcg ttatgttaaa cgttttcggt 3780

aagatttagt taggtaggag tcgggagtga tgggaaaatg aattagaagt aagttttttt 3840
ttttttttaa atgaaaagat ttgaggcggt atcgtatttt ttttgtggtt ttttgtagtt 3900
tttttttttg cgtttttttt gttttttttt tttttttttt tttttttttt ttttgttcgc 3960
gcgcgcgtta atatatacgc gcgtacgtat atatatatat atatatttat atatatagat 4020
atattttttt tttcgttttt ttcgtcgatt tttattttcg tggaggagta ggtttgttga 4080
tttgttcggg ttcgttcggt tgtttttatt gttttttttg aataatattt taaagagaga 4140
ggatcgtatt atagatatcg cgggggtaaa gttaaaaaag cgggcggagg ggcggaggaa 4200
aaaatttaag aagtagaaat ttttacgcgg agtttttatt gcgaagaaaa aaacgcggtt 4260
tgaaagattt ttttttttta ttattattaa ttattattta tttattattt tgatatttcg 4320
ggttttgatt gatagttgga gtggtaaaag tatgaaatac gatagttcgg ttatatgtgg 4380
gttgttgacg ggtcgttcgt aatttttagt tcgggggttt gataattttt tttttttttt 4440
tttttttttt tttttttttt tttttttttt tnnttttcgg cgggggggga ggaggaggag 4500
cgggaggagg aggaggagga agattaggag gaggaggaag aagaggaaaa aagagaaaaa 4560
gaagaaatat tatagaaaaa aaaatttttc gttgtttaga ttgggttttt tttttttttt 4620
aaaaaatagt atattggaga attgggagaa gtttttttgg ttggaaaaaa aaaaaaggaa 4680
tttttagttt agattatttt tttattcgga ttgggatatt aaatatacga tatatttagg 4740
agtttattgg agcgtagatt gatggcgtaa agggtaggtt taaattttta atatttattt 4800
agatataaat ttttttttaa gaggggtttg tttcgatgtt tttttttttt ttaacgtttt 4860
tgttgttgtt cgttgtttga tgatggcggt tagtatttta aaaaatagta aaattattta 4920
tcggtttttt tttttttcgt gtttttgcgt gtgtgcgagt gtgtgtgcgt gcgtgtgtgc 4980
gtgtttgggc gcgcgtgttt tagcgttgtg cgagcgggcg ggcggcgagt gcgtgtgagt 5040
ggtgtgtgtg tgtgcgtgtg taatcgttat acgttttcgt ttcgttttag tttttatttt 5100
agtagcgcga ttttttcggt ttttttgttg gtcgttgtgc gtcgtgcgtc gtggttcgcg 5160
cggttcgggg cgggtacggg ttgcgtagga gtcgtcgtcg gggtcgtcgt tggggtcgtt 5220
tggtcgtagt cgtgcgtcgt cgtttagatt attagtttgg gttgtttgtt ttgtttttga 5280
aattgataag gacgtaggga attcgttgtt aaataatgtt tttggtaatt tttacgttat 5340
tcgtttttt attttcgtt ttttgttttc ggggaggtat tggtttagt ttttttagt 5400
tttcggcgtt ttcgtgttga ttattagcgt tttttttttt tttattttgt tttatttcgt 5460
tttttgattt attggatttt tagaggaggc gaaggggggt taggggtggg attcggggtt 5520
ttttttttat attaatttta tttttttttt cgttggttcg ttacggtttt gttgttttcg 5580
gcgggagggg gtttttcggc gtttttttaa attttcggtt tcgtcgtttt tttttttttt 5640
tttaaacgtt gtttttggtt tatgtttttt tatttttaaa tttaataata tttttttttt 5700
ataattttta atttcgttgt tttggttggt ttggtttttg tgtgtttaat ttgtgttcgg 5760
tattttttt tttttgatt ttgttttat ttttatttt tttttttttt tttttttttt 5820
tttttttttt tttttttttt tttttttttt tttttttttt tttttttttt tttttttttt 5880
ttttttattt tttttttttt tttttatttt tatttttttt tttttttttt gttcgttttt 5940
ttttttttgt tttttttttt tttttatttt ttttttttgt ttttttttat tttttgcgtt 6000
tttttttttt tttcgggttt tgatagt 6027

<210> 190
<211> 6027
<212> DNA
<213> Artificial Sequence

<220>
<223> chemically treated genomic DNA (Homo sapiens)

<220>
<221> unsure
<222> (1555..1556, 3478)

<400> 190

attgttagaa ttcgggaagg gggagggggc gtaggaggtg agggagaata gaggaggggg 60
gtggaaaggg aaaaagagta gggagaagag ggcgaataga aaagagagga agggataaag 120
atgagagaga gggagggagg taagagagaa gagaggagga ggaggaaaag gaggagaggg 180
ggagagagag agagagagag agagagagag agagagagag agagagagaa aataaaaata 240
aaaataaagt tagaaaggag aaaagtatcg agtataagtt gagtatatag aaattaaatt 300
agttaaaata acggagttaa aagttgtgag gaaaaaatgt tattaaattt aaaaatgaaa 360
aagtatgaat taagagtaac gtttagagaa ggaagaggag gcggcgaggt cggggatttg 420
agggggcgtc gggaggtttt ttttcgtcgg aggtaataga atcgtagcga gttaacggag 480
ggagaagtgg agttagtgtg aggaggaagt ttcggatttt atttttaatt ttttttcgtt 540
ttttttgagg atttaataaa ttaaaaggcg aaatgaaata aaataaagag aagaaaaacg 600
ttaataatta gtacgagaac gtcgaggatt aggaaagatt agggttagtg ttttttcggg 660
ggtagggagc ggagggtggg ggggcgaata acgtgaaagt tattagaaat attatttagt 720
agcggatttt ttgcgttttt gttaatttta gagataaaat aagtagttta ggttagtagt 780

ttaggcggcg gcgtacggtt gcggttaggc ggttttagcg gcggtttcgg cggcggtttt 840
tacgtagttc gtgttcgttt cgagtcgcgc gagttacggc gtacggcgta tagcgattag 900
taggagaatc gggggaatcg cgttgttggg gtaaaagttg gagcgaggcg aaagcgtgta 960
acgattgtat acgtatatat atatattatt tatacgtatt cgtcgttcgt tcgttcgtat 1020
agcgttggaa tacgcgcgtt tagatacgta tatacgtacg tatatatatt cgtatatacg 1080
tagaggtacg ggagggaaag aagtcgatga ataattttgt tattttttaa aatattggtc 1140
gttattatta agtagcgagt agtagtagaa gcgttgaagg gagaggaggt atcgggatag 1200
gtttttttta ggagaggatt tatatttagg taagtgttgg agatttaaat ttattttttg 1260
cgttattagt ttgcgtttta ataaattttt ggatgtgtcg tatatttaat attttagttc 1320
ggataagaaa gtgatttagg ttgaagattt tttttttttt tttttaatta aagagatttt 1380
ttttaatttt ttaatatgtt atttttaggg gggaaaaaa agtttagttt agataacgaa 1440
gaattttttt ttttgtgata tttttttttt tttttttttt tttttttttt tttttttttt 1500
ttgatttttt tttttttttt tttttcgttt tttttttttt tttttcgtcg ggagnnggaa 1560
aaaaaaagaa agaaaagaaa aagaagaaaa agaagaaaaa aattgttaag ttttcgaatt 1620
gaaggttacg agcggttcgt tagtagttta tatgtaatcg aattgtcgtg ttttatgttt 1680
ttgttatttt agttgttaat taaagttcgg aatgttaagg tagtgaatga atgatggttg 1740
atgatgatga agaggaggaa ttttttagat cgcgtttttt ttttcgtagt aaagatttcg 1800
cgtaggggtt tttgtttttt gaattttttt tttcgttttt tcgttcgttt ttttagtttt 1860
gttttcgcgg tatttataat acgatttttt ttttttaaag tattgtttaa agaaagtagt 1920
gagaatagtc gggcggattc gagtaaatta gtaagtttgt ttttttacgg ggatgggggt 1980
cggcgggggg gacgaaaagg agagtgtgtt tgtgtgtgtg agtgtgtgtg tatgtgtgtg 2040
cgtgcgcgcg tgtgtgttgg cgcgcgcgcg aatagggaga gagggagagg gggagagaga 2100
agaggatagg gagagcgtag agaggaaaat tgtagaaaat tatagggaaa gtacggtatc 2160
gttttagatt tttttattta aaaaaaaaaa aaaatttatt tttagtttat ttttttatta 2220
ttttcggttt ttgtttaatt gggttttgtc ggggacgttt ggtatggcgt tttagttttt 2280
gtttttttta atttcgcggt tttttttttt tttgttcgtc gattatagta tttcgaaggt 2340
tttttgttaa cgtttaaggg tagtaagttt tgtagacggt attattgtcg ggttaaattt 2400
tttattaagt ggtatttttt tttttgcgta gatttagtaa ttttgttaat atttattttt 2460
ttttaggatt agaagagatt agtttaattt agagtatatt ttttttaagc ggtgcggata 2520
aggggttatt tcgagagttt ttgggggtcg cggcggaatc gggagggagg aagtgaggag 2580
gtagttattt gggttcgatg tagttaaggg cgtgtgtgtg tgtgtgtgtg tgtgtgtgtg 2640
tgtgtgtgcg cgcgcgttcg cgtcgtgcgc gcgaatatta tgtgtgcgtt tttaaagtgg 2700
atttatttta gagttgggaa gtataggagg aaaagacgga gagagtgggg gagaaagaag 2760
ttgtcgggcg aggaggagtg gtcggcgttt atagtttagt tttgttgtgg tcgttcgagg 2820
cgtacgggcg gttcgcggta acgtttgtta atggttgtgt taattatata ggggcgattt 2880
cgcggggtta gggagatggg gttagtttcg aaagaatttt tttaaaaagg aaaaggaaac 2940
gaagggaatt attttatatc gtttgggtgg aaattagttg tggtcggtgt tttgtttttt 3000
ttggttcgag ttgtattagg ttttattttt gcgcggttgg tttttcggcg gtcgcgcgtt 3060
aattattacg agttcggagt tgggggcgga gttacggtta tttcgcgtaa gttttgtcgt 3120
ttagtagttt ttatgttttt cggttttttg tggcgcggtc gggcggatag gcgttggaag 3180
cgcgtttttt ttttaatttt tcgttttcgt ttttttagtt tttggtttag agagtggaaa 3240
gcggattgtt gttttttaat agtcgtattt ttttttaatt ttgaatttat tttgagaagt 3300
ggagagtagt gtttttcgcg agtcgcgttt aatcgatttt ttcgcggagg gagggtggga 3360
tgggggaggt tgattagaga gaataaagta ttttgtgtgt gtgttgtgtg tgagagagag 3420
agagagagag agagcgagag agagcgggga ggagggcggg tgagagggtt tttttttntg 3480
gttttcgttt tatgtttttt gttttattgt aagttttcga gtaatagaaa ttaggttaag 3540
attgtagttt gtagagtttt taagcggcgt cggagaaatt tggtttcgtt ttaatagggt 3600
tgaggaatga atttcggagg ttaataaagt tttttaaaaa tgatgaatga ttgagtattt 3660
gtgatgatga tagtgattat tagagtaatt aaatagtttg attaaatgag ttattataat 3720
aatgatgttt gcgggaaatc ggtttttata tataaagaaa gatagtgcga aggggttggt 3780
agaggcgcgg atagcgcgac gttagggaga ggggttgcgg gtcgcgaggt ttttgtttaa 3840
tggttcgagt agggacggtt agatttacgg agttttaggg aggagagtgt agtttaggtt 3900
tttagttatt tttttttgtc gggttttaag tttgttagag tcgacgggta ggaaatttga 3960
aggttggggg gagggtattt tttttacgtt tttttttttt ttttagaaga attttaagat 4020
taaaagtatt tttatttgtt gttgagttgt tcgtcgatat ttaagagaag gtgagtaaaa 4080
aagaaaagaa gatgaaagag aaaagaaaaa gttggttttt agaattttat tttaatcgat 4140
agttacgcga atttagttta gtcgggatcg ggaggtttga gagtgcggcg gggtggggag 4200
gggaaaaaaa taaaaacgtt gcggagcgtt ttcgattttg taaagttgtt gatgttggtg 4260
ggtttaagtt ttcggtgtag cgtcgttggt tgagaaattt gtatttagtt tttatttttt 4320
taattatttt taaatttaat ttaagaattt aagtttatcg tcgttaatag gattattgtt 4380
gttttaaatg ttttgtttaa taagtgttag ttacgttttt ttttaaaaga aattttttag 4440
tgtttttgat aaattttttt ttgtaaagat taattttatg cggaattttt gttttgtagg 4500
gtttaaatat ttaatattgt tattttttgt tttttttttt gtttttatag tgatattgta 4560
aagtgagtta gtatttttat agtttttatt tagaaaaatt taagtcgtat ttttagaaaa 4620
atattttttt ttttgtagtg tgagtaaaaa taatttagat agattgatga tttttatatt 4680
tttttttttt tttttatttt tttttttttt tttttttttt tttttttttt tttttttttt 4740

ttttgagacg gagtttcgtt ttgtcgttta ggttggagtg taatggcgcg atttcggttt 4800
attgtaattt tcgtttttcg ggtttaagcg atttttttgt tttagttttt tgagtagttg 4860
ggattatagg ggcgcgttat tacgttcggt taattttttg tgtttttaat agagacgggg 4920
gttttattat attggttagg ttggtttcga atttttgatt ttttgattta ttcgtcgcgg 4980
ttttttaaag tgttcgaatt ataggcgtga gttatcgtat tcggtcgatt tttatgtttt 5040
taaataattg taagttaata atgagttgat tatttggtga taattttggg ttttataaaa 5100
aaagaaaagt ttaaatttta tttttatgtg ttttttattag atgtagaagg tatttttaat 5160
tttaacgttt ttttttgttt ttttttttta tatttttaat tttttttgta ttttattgaa 5220
tatattaaag taaataatag gaataatatg gttaggtgta ggagggagag agaaatatga 5280
tattttaaga tattgttaaa aagttatatt taattttaac gatttttagt taaaaaagtg 5340
tgagagtgtt gaaagtgtat ttttattgga ggaaatttat gtttttttaa aatttgttag 5400
ttgttttagg ggagaattta gaattttttt gaacgaaaga gattttattg atttagttgt 5460
atgttattta tgaaattgtt agtttaaaat agtagtttta gtgtagtgga ttatatatga 5520
ggagattaag ttggagtcga tttaaggtat tggtttttgg ttagtttgtt gaatttaaaa 5580
tattattatt tttaattagt aatttgcggg tgttttatta atagagttga aaaaggttta 5640
tttaatatat aagaattttt tggttttttt tatttttaat tttttagttt aatagaagcg 5700
attttaaagg agtagggtat tcgtatttat tgatttttat tttggtggta ttttgttaat 5760
gttttattgt tagcgttggt atttttattt aggtttttag gtttatttgt ttttttatat 5820
ttggggtgaa aggaaagtag gtggggtagg ttcgggagat aggtacgaaa tgggggtcgt 5880
aggaggtggt atatattttc gttaatagtt agtttttttt gtttaggttg gaaatggttt 5940
taataggcgg taaaatttta ttttgcggtg ttaaattggg gttattttgg ggttagagtt 6000
tttagtgttc gagaggagtt gtttagt 6027

<210> 191
<211> 6809
<212> DNA
<213> Artificial Sequence

<220>
<223> chemically treated genomic DNA (Homo sapiens)

<400> 191

taggtaagag aaagaaataa aaagtattta aataagaaaa ggagttagat aatttttttt 60
ttttgaagat aagaattttt tttttttttt tttttttttg agatagaatt ttattttgtt 120
atttaggttg gagtgtagtg gtacgatttc ggttttttgt aatttttatt ttaagtagtt 180
gggattatag attatatgtt tagttaattt gttttttagt atttttagta gagatggggt 240
tttattgtgt tggttaggtt ggttttaaat ttttggtttt aagtgatata ttcgttttgg 300
ttttttaaag ttttgggatt ataggtatga gttattgcgt tttatttttt gaagataaga 360
ttttatattt agattttatt aaatggtttt tagaattgat aaataatgtc ggtatagttt 420
taggatataa aattaatgta taaaaattag tagtattttt atatattaat aacgtttaag 480
ttgagagata agttaagaat atagttttat ttataatagt tatatatata tatatatata 540
cgtatataat ttaggaatat atttaattaa ggaagtgaaa gatttttata aggagaatta 600
taaaatattg ttgaaggaaa ttatagatga tataaataaa tagaaaaata ttttacgttg 660
atggattgga agaattaatt ttgtttaaat ggttatattg tttaaagtaa tttatagatt 720
taatggtatt tttaagaaat tattaatatt attttttata gaattagaaa aattatttta 780
taatttatat ggaattaaaa aggagtttgt agaatagtag gaaaaaataa aataaaataa 840
aataaaattt taaaagagtt tgaatagtta aagttaaagt tagaggtatt atattattta 900
attttaaatt gtattataag gtaatagtaa tttaaatagt acggtattgg tataaaaata 960
gatatttaga ttgatggaac ggagtagaga atttagaaat aaagtcgtat atttatcgtt 1020
atttgatttt taataaagtc gataaaaata agtaatgggg aaggattttt tgtttaataa 1080
gtggtgttgg ttagttttat gtagaataaa attggatttt tatttattat tatatataaa 1140
aatttattta agatgggtta aagatttaaa tgtaagattt taaattttag gaattataga 1200
agaaaattta ggaaatatta ttttagatat cggttttggg aaagaattta tgattaagtt 1260
ttgaaaagta atttgtagta aaacgaaaaa ttgatagacg ggatttaatt taaatagttt 1320
ttgtataatt aaagaaatta tgaatagagt gtggtgttac gatatatatt ggtttttatt 1380
tgtggttttt ggtttgtaat ttttataatt ttcgttatag ttttttgtta tgttgagtgt 1440
gttaggtttt agaggtaggt ttttaatttt tttttgtttt ttttttattt gttaataatt 1500
ttttattttt ttgattgtgg gttttaagat ttttttagga gagggtttta ttggatatat 1560
atggatataa agatggtaat aatagatatt gcggacggga gggaaggagg gagtaagggt 1620
tgaaaaatta attgttgggt gtttttttta ttatttgagt gataagaata atcgtatttt 1680
aaatttgagt attatgtaat atatttatgt aatttgtata tgtattttcg aatttaaaat 1740
aaaatttgaa attattttaa aaaaagtatt attttgtata gtttagtgat ttttaggaat 1800
ttatgtgtgt ttttatagtg ggagtgattg tgtttttttt agagtatttt ttttatgtat 1860
tttttttaaat atttttttata ttttagaaat tttttttagat tttttttata tttttttaatt 1920
aaaataatat gttgtaatag tatgaatgta gaagtagaaa tgagaattta ttgttattta 1980

ttaagttaga tattaaaaag atttgtgaaa atgtataacg gtgttattat tttttttgtt 2040
ttgttttgta aaatatcgtt atttttattt aaaaaaattt attttagttt tttagttgtt 2100
taatttagat tttataaaat tttatagtta aagggaattt tgattgtaga tttatttttt 2160
ggatttttgt tttttttttt agaattttgt tagttagttt tttattattt tataatttta 2220
tgttatggaa tataaattat ttaaatcgtg ttttttaggt agagggagaa tttttggatt 2280
agttagaagt agaattttaa tattgtattg gggtttgggt gtttaatata aaaaatatat 2340
ttaaagttaa taaattaaaa aagttagtaa agtaaaataa aaaataaaaa ttgtgtatga 2400
aaatggtttt gaaaggattt ggaaggaaag tataaagtga gagggtataa aaagttatag 2460
ttttggaaaa tagtttgata tataatttta gattttgtat ttttatagta aaatgttatt 2520
tgagtattta ggcgttttag ttttttatat atgaatttta tcgattttta gagtttgtgg 2580
tttttggtga ggatagcggg gaggattatt ttatttttat aaggttttgg aggtttgata 2640
agttgttgaa aatattaaaa ggtatataat tattattagt ttagtaatat tttgttttag 2700
tataattatt gatttattat ttattttatt attgatatta attttaaaag ttagtgtggg 2760
ttatatagat tttattttat atgaagaata taaagttttt ttattagaaa tttattttta 2820
ttattttata tatttttaga aaaatttaat gttatattga tttattgata tagttgtgta 2880
gcggttaggt gttttttttt atgattattt tttttttttt tttaattatt attttttttt 2940
aagatggggg ttttattatg ttgtttaggt tgattttgaa tttttgggtt taagtaattt 3000
ttttattttg gagaaaatta aagaattttt ttttttaatt tttttttttt tttttttttt 3060
agatggagtt tcgttttgtc gtttagttta gagtgtagtg tcgttatttc ggtttattgt 3120
aatttttatt tttcgggttt aaacgatttt tttgttttag tttttcgagt agttgggatt 3180
ataggcggtt gttattacgt ttagttaatt tttgtatttt tagtagagac gggggtttta 3240
ttatgttggt taggttggtt ttgaattttt gattttaagt gatttgttcg tttcggtttt 3300
ttaaagtgtt gggattatag gcgtgagtta ttgcgtttag attttttttt aattttttat 3360
tatttagtta ttttattttt tttttttatt tagtttgaaa tgtaatttat ggttgggttg 3420
ggtggtttac gtttgtaatt ttagtatttt gggaggtcga cgcgggtata ttatttgagg 3480
ttaggagtta gagattagtt tggttaatat ggtgaatttt atattttttt ttttttcgtt 3540
tttattaaaa atataaaatt agtttggtgt ggtggcgtat gtttgtaatt ttagttattc 3600
gaaaggttga ggtaggggaa tcgtttgaat tcgggaggta gaggtcgtag ggagtcgaaa 3660
tcgcgttatt gtattttagt tcgggtaaga agagcgaaat ttcgttttaa aaaaaaaaaa 3720
aagtaattta tatttttttt tgtttttttt ataggattta ttagttttag tttttggaga 3780
tgggaagtat aaaatttgtt tttttattta gttgtaattt gtattggttt ttttaataat 3840
tatagtattt tggggttttt tgggaattta tatatttgtt ttttgttttt taaataagta 3900
ggtatttttt ttgattgttt ttaaataatt tgggtatttt tttttcggtt tcgcgtgatg 3960
tagtttaaaa aatagaatag gttacgttat ttttttgttt tattttgttg ataatttgtg 4020
gagtcgggta gagattttat tttttatttt aagtatttgg cgtttgtggg ggaattaaga 4080
ttttaaatga gaatattaag tttttcggta gttggtatta attaattatt tattattgat 4140
aacggaatga gttattggaa attttaaaag gaaaagtata tgggagaatt tgattaattt 4200
agttagttat ttttggagtg ttatagttat taatgaagta atttttagtg taggttttgg 4260
ttttagggcg ttatgtataa ttttttgttt cgcgtaacgt gggtttttcg gagtttattt 4320
tgttagcgtc gcgtacggtt ttagtttatt aggtcgttaa ggttcggagg ggggcgtttt 4380
agtcgcgggg aatattggaa atgttttagg ggcggatttc gaagatcgag tattgtttcg 4440
agggattttt tgcgttattt cgtaatcgtt tgcgttttag gttttcgtag gtgggggtcg 4500
ggtgggtgaa gattgggatt ttatgttacg cgtagtattt agaggagggt tggggtttta 4560
gggtcgtagc gcggttagtt tagttcgggt tcgagttttt tagtagcgtt ttttttgggg 4620
gttggttttg attttcggtg tcgttacgag tgggtggtta gaagttggtg gtggagaggg 4680
agagcgtttg taaggaacga cgaggttggc gtcgggatta cgcggcggag gcgatcgaaa 4740
gagaaacgag gttaggtaag gaggaggaaa tttattcgat gaaggcgagt tgtcgtttaa 4800
gagggaggag ggttttttcg gttgtcgttg ttgttgtttc gggacgtcgt ttggggagtt 4860
agttcgttcg taaggtgcgc gttaggtagt ttcgtattta gggggcgttt tttttggttt 4920
tttttagagc ggtcgggacg gaaggagttg cgcggcgcgg cgcggcgcgg cgcggcgggt 4980
ggcgagggcg ggttgtattt cgagcgcggc gtttttgagt tgtatcgcgg cgtaggtttg 5040
cgagtcgatt tgttagtcgg ttaagaaaag gaagtttcgt tttttttcgt ttattcggtt 5100
tttttatttt ttgtatttta aattttgtag agggcgagcg gcgcggttac ggaggcgtcg 5160
aggaggagcg agtcgtcgtc gggtagcggc gtgttttcgg gggagagggc gtcggagagg 5220
aggcggcggc gcggcggcga gggcgcggcg cgcgatggta gttgtttagt tcggcgggcg 5280
cggagtagtt tcgagttgtg gttggttagg cggtgcggtt gggcggggga cgtcgtcgtc 5340
gttgttgttc ggttcggaga gatgagtacg gaggcggacg agggtattat tttttttgtg 5400
ttatttttcg ttttttcggg tttttgtatt attttcgagg gcggtatttg taggagggga 5460
ggagcggcgg cggtgggcga gggcgaggag tattagttgt tatcgtcgtc gtcgggtagt 5520
ttttggaacg tggagagcgt cgttgttttt ggtatcggtt gttcggcggt tattttttcg 5580
agtagtgtta ttcgaggtcg gggtagtttt gttggcgggg gtagtcgacg gattacggtg 5640
gtatatgtga ttaacgaagc gagttaaggg taattggtgg tggtcgagag cgaggttttg 5700
tagagtttgc gggaggcgtg cgagatagtg ggcgttattt tggaaatttt gtattttggg 5760
aaattcgatt ttggagaaat tatcgtgttg gatcgttttt ataatgtagg tgcgtgtgtg 5820
attttttttt tatttcgcgc ggtcgggatt cgcgtttaat ttggcggttt tcggggtatt 5880
ttattttttt gggcgggaag tatttatttt ttgttttatt ttgggggtag cgagcgtcgt 5940

tgcgtgattg tttttgtcgt tcgtcgtttc ggagagagcg tgtgtgtttg tgtaagagtt 6000
tgtgtgtatt tgtgcgatag atttatggat ttatttatcg tttagcgttg gagttttggg 6060
tttatgcgat cgatttgaat tgttttttag tagtgtgaga tatttgtttt agttagtttt 6120
ttttttttta gtgtttcggt ttcgtttttg tgaggtttgg tttttgtttt ttttttgtat 6180
atatttgtga tcgttttaga tgaggttttt tttttttttg tgtagttttg agagtacggg 6240
aacgatgata tattatgtac gtagagtgta attatattaa tttgtatgtt atgtgatgat 6300
ttttttaatt tattttattt tcgataagtt ttagtagttt gaattttttt ttattaattt 6360
agattttgaa gtaattatag tgtttttaaa ggtaataata ttataattta tattgaattt 6420
gtgtgttatt tttgtaagga ttttggtgaa agaattaggg tttgtttttt attattttat 6480
aaattatggt gggttagaaa gtttgtaatt taatattgtt taaaatattt gtatgttatt 6540
ttaataataa gattagtttc gttattaaag tgattgttag tataatttat gaaagaaatg 6600
tattttttaa tttattttaa attttagaaa atgttaaggt agttttagta attgaagtat 6660
ttagttgtta gtaaatgtta ggaataatat tatattatgg aaattaattt ttagtttttt 6720
gtacgtttag tgttgagaat atatatatat ttatgtttat atttgtgggt aaaaatttgg 6780
tttagtagtt ttaaaataat atattttttt 6809

<210> 192
<211> 6809
<212> DNA
<213> Artificial Sequence

<220>
<223> chemically treated genomic DNA (Homo sapiens)

<400> 192

aagaagtgta ttgttttgaa attattaaat taagttttta tttataaatg taaatataaa 60
tatgtatata tttttaatat tggacgtata ggggattaaa ggttaatttt tataatatgg 120
tattattttt aatatttgtt aatagttaag tgttttagtt attagaattg ttttaatatt 180
ttttgaaatt tgagataggt tgaaagatat atttttttta tggattatgt tagtaattat 240
tttaatggcg aaattaattt tattattagg atagtatata ggtgttttag ataatgttaa 300
attataaatt ttttagttta ttatggttta tggggtaata aaaaataggt tttggttttt 360
ttattaaaat ttttataaag ataatatata agtttagtgt aagttgtaat gttattgttt 420
ttggaagtat tgtaattatt ttaaagtttg ggttggtgag aggaaattta agttattaaa 480
atttatcgaa ggtaaggtga gttgggaaga ttattatatg gtatgtaaat taatatagtt 540
atattttgcg tgtatggtat gttatcgttt tcgtattttt aaaattatat agagaaaagg 600
agaattttat ttaaaacggt tataggtata tgtagaaaga aaatagggat taaattttat 660
agaagcgaaa tcgagatatt gaaaggagaa agattgattg gggtaaatat tttatattgt 720
tggagaatag tttaagtcga tcgtatggat ttagggtttt agcgttgggc ggtgggtgga 780
tttataggtt tatcgtataa gtatatatag atttttgtat aagtatatac gtttttttcg 840
gagcggcgag cgataggagt agttacgtag cggcgttcgt tgtttttaaa gtggggtaag 900
aagtaaatgt ttttcgttta gaaaaatgaa atgtttcgaa gatcgttaga ttaaacgcgg 960
gtttcgatcg cgcgggatgg gaaaggggtt atatacgtat ttgtattgta aaagcggttt 1020
agtacggtgg tttttttaaa gtcgagtttt ttaaaatgta gggtttttag ggtggcgttt 1080
attgtttcgt acgtttttcg taagttttgt agggtttcgt tttcggttat tattagttgt 1140
ttttggttcg tttcgttgat tatatatgtt atcgtggttc gtcggttgtt ttcgttaata 1200
gagttgtttc ggtttcgggt ggtattgttc gaggaggtgg tcgtcggata atcgatgtta 1260
ggggtagcgg cgttttttac gttttagaag ttgttcggcg gcggcggtgg tagttggtgt 1320
ttttcgtttt cgtttatcgt cgtcgttttt ttttttttgt agatgtcgtt ttcggggatg 1380
gtgtagaagt tcgagggggc gaagggtggt atagagaaag tgatgttttc gttcgttttc 1440
gtgtttattt tttcgggtcg ggtagtaacg gcggcggcgt ttttcgttta gtcgtatcgt 1500
ttggttagtt atagttcggg gttgtttcgc gttcgtcggg ttaagtagtt gttatcgcgc 1560
gtcgcgtttt cgtcgtcgcg tcgtcgtttt tttttcggcg tttttttttt cgagggtacg 1620
tcgttgttcg gcggcggttc gttttttttc ggcgttttcg tggtcgcgtc gttcgttttt 1680
tgtagagttt agagtataag gggtggggaa gtcgggtgag cgggaaggga cggagttttt 1740
tttttttggt cggttgataa gtcggttcgt aaatttgcgt cgcggtgtag tttaagggcg 1800
tcgcgttcgg ggtgtagttc gttttcgtta ttcgtcgcgt cgcgtcgcgt cgcgtcgcgt 1860
agtttttttc gtttcggtcg ttttgggagg ggttaggaag ggcgtttttt gggtgcggag 1920
ttatttggcg cgtattttac gagcgggttg gttttttagg cggcgtttcg gaatagtagt 1980
agcggtagtc gaaaggattt tttttttttt taggcggtag ttcgttttta tcgggtggat 2040
tttttttttt ttgtttggtt tcgttttttt ttcgatcgtt ttcgtcgcgt ggtttcggcg 2100
ttagtttcgt cgttttttgt agacgttttt tttttttatt attagttttt ggttatttat 2160
tcgtagcggt atcgagggtt agggttagtt tttaaggagg gcgttgttag gaagttcgag 2220
ttcggattga attgatcgcg ttgcggtttt gggattttag tttttttttg agtgttgcgc 2280
gtggtatagg gttttagttt ttatttattc ggtttttatt tacgaagatt tggagcgtaa 2340
gcgattacgg ggtggcgtag aaagtttttc gaggtaatat tcggttttcg gagttcgttt 2400

ttgaagtatt tttagtgttt ttcgcgatta gggcgttttt tttcgggttt tggcggtttg 2460
gtggattggg atcgtgcgcg gcgttggtag gataggtttc gggagattta cgttgcgcga 2520
ggtaggaggt tgtatatagc gttttggagt tagggtttgt attggaggtt gttttattag 2580
tgattgtggt attttagaag tggttggtta aattgattag gtttttttat gtattttttt 2640
ttttaaaatt tttagtggtt tatttcgtta ttagtaatga gtaattgatt agtgttaatt 2700
gtcgaaggat ttagtatttt tatttaggat tttaattttt ttataagcgt tagatattta 2760
agatagaaga tggagttttt attcgatttt ataaattatt aataagatga aataggggaa 2820
taacgtgatt tattttattt tttaaattgt attacgcgag gtcgggaggg aaatatttag 2880
attgtttaag agtaattaaa aaaagtattt gtttatttgg ggagtaaaga atagatgtgt 2940
aagtttttag agaattttag aatgttgtaa ttgttaggaa agttagtgta agttataatt 3000
gagtaaagaa ataggttttg tattttttat ttttaggaat tgagattagt gagttttgtg 3060
aaaaaggtag aagaaaatgt aaattatttt tttttttttt tgagacggag tttcgttttt 3120
tttgttcggg ttggagtgta atggcgcgat ttcgattttt tgcgattttt gtttttcggg 3180
tttaagcgat tttttgtttt tagtttttcg aatagttggg attataggta tgcgttatta 3240
tattaggtta attttgtatt tttagtagag acgggagggg gaggggtgtg gggtttatta 3300
tgttggttag gttggttttt aatttttgat tttaggtgat atgttcgcgt cggtttttta 3360
aagtgttggg attataggcg tgaattattt agtttagtta tgaattatat tttaaattgg 3420
gtaagaagga aggataaaat agttgaatag taaagaatta aaggaaagtt tgggcgtagt 3480
ggtttacgtt tgtaatttta atattttggg aggtcgaggc gggtagatta tttgaggtta 3540
ggagtttaag attagtttgg ttaatatggt gaaattttcg tttttattaa aaatataaaa 3600
attagttgga cgtggtggta gtcgtttgta attttagtta ttcgagaggt tgagatagga 3660
gaatcgtttg aattcgggag gtggaggttg tagtgagtcg agatggcgat attgtatttt 3720
agattgggcg atagagcgag attttattta aaaaaagaaa aagaaaaaga attaaaggaa 3780
agagtttttt aatttttttt aaggtgggag gattgtttga gtttaagagt ttaagattag 3840
tttggataat atagtgagat ttttatttta aaaaaaaata ataattaaag gaaggaagga 3900
aatagttatg aggaaaaata tttagtcgtt atataattgt attaatgaat taatgtgata 3960
ttagattttt ttaaaggtgt ataaaataat agaagtaaat ttttagtaag gaaattttat 4020
attttttatg taaaatggaa tttatgtgat ttatattagt ttttggaatt aatattaata 4080
gtaaaatagg tagtgaatta gtgattatat tgaaataaaa tgttgttgag ttagtaatag 4140
ttatgtgttt tttaatattt ttaataattt gttaaatttt taaaattttg taggagtaag 4200
ataatttttt tcgttatttt tattaagaat tataaatttt ggaaatcgat gaggtttatg 4260
tgtggaaaat taaaacgttt aaatgtttaa gtagtatttt attgtagaaa tataaaattt 4320
gaaattatat attaagttat tttttaaagt tgtaattttt tatatttttt tattttgtgt 4380
ttttttttta gattttttta gagttatttt tatatataat ttttattttt tgttttattt 4440
tgttggtttt tttgatttat taattttaaa tatgtttttt atgttaggta tttaaatttt 4500
aatataatgt tgaaattttg tttttagttg gtttagagat ttttttttta tttaaaaggt 4560
acggtttagg taatttatgt tttatagtat agggttatga gataatgagg aattagttgg 4620
taagattttg gaagaaagga taggaattta gagggtgagt ttatagttag ggtttttttt 4680
ggttgtggga tttttgtgga tttgaattag gtagttgaaa ggttgaagtg aattttttta 4740
atgaaaaata acggtatttt gtaaaataaa ataaaaagaa tggtgatatc gttgtgtatt 4800
tttataaatt tttttaatgt ttggtttaat agatgatagt ggatttttat ttttgttttt 4860
atatttatgt tgttgtagta tgttgttttg gttgaagggt ataaagaaaa tttggagaag 4920
tttttgaagt gtaggaggta tttgaagaag tatatgaaga aaatattttg ggaggagtat 4980
agttattttt attgtgaagg tatatatgaa tttttaaaaa ttattgagtt gtgtaaaata 5040
gtgttttttt taaagtaatt ttaagtttta ttttagattc gggggtatat gtgtaggtta 5100
tatggatata ttgtatgatg tttaagttta gggtacgatt gttttgttta tttaggtagt 5160
agaaagagta tttaatagtt ggttttttaa tttttgtttt tttttttttt ttcgttcgta 5220
gtgtttgttg ttattatttt tatgtttatg tgtatttaat gggatttttt tttgggaagg 5280
ttttaagatt tatagttaga aaggtggggg gttgttagta ggtaaaagga gagtaggaaa 5340
aaattagagg tttgtttttg aggtttaata tatttaatat aatagaagat tataacgagg 5400
gttatgggag ttataagtta gaaattatag atgaaaatta atatatatcg taatattata 5460
ttttgtttat agtttttttg gttgtgtaga agttgtttag attaggtttc gtttgttagt 5520
ttttcgtttt gttgtaaatt gttttttagg atttagttat aaattttttt ttaaggtcga 5580
tgtttagaat ggtatttttt aggttttttt ttatgatttt tagggtttga ggttttatat 5640
ttaaattttt aatttatttt gagtgaattt ttgtatatgg tgatgggtag gggtttagtt 5700
ttattttgta taaggttagt tagtattatt tattgaatag ggagtttttt tttattgttt 5760
atttttatcg atttgttgga agattagatg gcggtaggtg tacggtttta tttttgagtt 5820
ttttatttcg ttttattagt ttgagtgttt gtttttgtat tagtatcgtg ttgtttgggt 5880
tattgttgtt ttatagtata gtttgaagtt aggtaatatg atgtttttgg ttttggtttt 5940
agttatttag gtttttttgg ggttttattt tattttattt tatttttttt tgttgtttta 6000
taggtttttt tttggtttta tatgaattgt agaatagttt ttttaatttt gtggaaaatg 6060
atgttggtag ttttttagga atattattga atttgtagat tgttttggat agtgtggtta 6120
tttaaataag attgattttt ttaatttatt agcgtggaat atttttttgt ttgtttgtgt 6180
tatttatgat tttttttaat agtgttttgt aatttttttt gtagggattt tttatttttt 6240
tgattggatg tatttttaga ttgtgtgcgt gtgtgtgtgt gtgtgtgtgg ttattataaa 6300
tggaattgtg tttttgattt gttttttagt ttgaacgtta ttggtatatg aaaatgttat 6360

tgatttttat atattgattt tgtattttga aattatatcg atattgttta ttaattttag 6420
gagttatttg gtggagttta ggtatagaat tttgttttta ggggatgagg cgtagtggtt 6480
tatgtttgta attttaggat tttggaaggt taaggcgggt atattatttg aggttaggag 6540
tttgagatta gtttggttaa tatagtgaag ttttattttt attaaaaata ttgaaaaata 6600
aattagttga gtatgtagtt tgtagtttta gttatttgag atggaggttg tagagagtcg 6660
agatcgtgtt attgtatttt agtttgggta atagagtgag attttgtttt aaaaaaaaaa 6720
aaaaaaaaaa aagattttta tttttaggga agagagatta tttgattttt tttttatttt 6780
ggatgttttt tatttttttt ttttgtttg 6809

<210> 193
<211> 13453
<212> DNA
<213> Artificial Sequence

<220>
<223> chemically treated genomic DNA (Homo sapiens)

<400> 193

tagagagaaa tttattgagt gtttttttat cgttttgtta ttgtgttttg ggttagaggg 60
acgaggatag atggagttag aagaggggtg gaggtgttgg tttttagttt tagtgtgtta 120
gtagttaggg gattaggatt cggtgtattt gttagtgaga ggtttttgtt ttttgggtgg 180
gtagtttttc gttttagagg attgttatag aggggtattt gttttttttt attttttata 240
ttttaattta gtggttatta ttgagggttt cgattgggtt tggcgggaga agatttcgta 300
gggggtttta ttgggtttta tgttgtcgtc gttttatggg atcgtttaag tttcgttatt 360
tttttttgtt tgtgtatgtg tggtttatat acgtgtacgt gtttagttta ggaggatttt 420
aaaattaatt taggttgaag gggaaaacga ttttttagtg tttttttttt ttgggatggg 480
aggggatcgt tgttttcgag ggaattagat gtagtagtcg aattttaggt gtaattttat 540
tattgggcga agtttaagtt tttagaggaa gggtatttat tgagaatttt tattagaaga 600
ttaggggaaa tttttagatg aattggttgt tgaagatttg tttttagggt ttgtaggaag 660
tggggtatat tggttgaagg aattgtgtga agttgttgga ataaggttgg ttcgcggttt 720
tttgttaggc ggcggtcgtt gtagtttata tttggggaga atcgtacgtt gcgttgagaa 780
agtttcgggg aagttgtttt cgagaggttg tttggtttgt ttgatttggg attttggttt 840
tttggtttta agtgtttttt gttagttggt ttggagagag ggtagtgttt tagttgaggt 900
tttttgttta agtttaattg tttttagatg gtttttggag cgttcgtgtt tattttttcg 960
ttcgttgttt tttagatggt tttttagaag tgagacggat agtttttttc gggatagttt 1020
tggaaggttt ttttttattt tgggtttttg ttggggagtg ggtgttaggg tggttttaga 1080
ggtgtaggga agtggaaatt tttgagatta tagtttttcg gtttattttt agtcgtttat 1140
tttagaggtt ttaaaaaagt ggttttttag tagtaggtat ttttggtgtt ttgaaggaat 1200
ttgttgggat aaagttgggg gagagttatt gattaattag attatttatt ttaagatatt 1260
ttggagagtt tgttatttaa tttttgtagt taggtatttt ttttagagag atagttaatt 1320
gattagtttt aattttttgg ttttaatggt taaggaaaga gtttaggtgg tgtgggggaa 1380
ggggggtggt tatttttggt tatggaattg gtttagttgt gttaataaat tattatgatt 1440
tgataagtta aacgcgttgg tcggggtttt attttttgtt tttcgttgac gtggtttgag 1500
cgttttattg aatttgtgat ttttttttta ttataatagt agagattttg ttattttatt 1560
ttttcgggga gtgttttttt tagcgtttta tagttttttg ggtgtagtag gtcgggtatt 1620
cgaatttcgt atttgttggt taagtttggg gtgtgttttt aatagtgggt gttgaggttt 1680
aagttggcgt tagtttttag ggtattagtg tcgtttgggt agtattttgg gttcggtttg 1740
tcggttgtaa agattattta tcgttgtttc ggaatttttt tgtttatttt ggttattgcg 1800
gtggagtttt ggtgttttta tttttttttt ttttagtgga atcgcggttt tttatttttt 1860
tataataatt gttttgtcgt ttgcgtgttt tgggtaggaa ttttttgtta ttagaaatgt 1920
cgggaagtcg gtcgaggttt gagtggtcgg gttagtgaat aagcgatttc gaggagatat 1980
tgttattgtg tgggagttag ttttatagaa gtaggggtcg ttttttcgta ttttggtgtt 2040
gtttttatga tgtatatgta tatttagttt ttatttgtaa tgtttttagc gggatagggg 2100
ttgttttttt gttttttagc gaaaaaggta gaggcgtagg cgtgtaagta tagagggtgg 2160
ttatggtcgt tcggggttaa tgttagtgtt gttttgggtt tgttggaatg cggtggtgtt 2220
taggtagata ttcgtgtttg gtttgagtat tgttatgtcg gtgtttagtt tttatttcgt 2280
gtgtttggtg ggggtatttt tatttgagat ttttatgaat tcgtagcgtc gggggtttat 2340
gagtgcgttt gatttttcgt ttatttttat ttttttttta tattggggtc gtgaatgttt 2400
ttgttgttcg gattttgggg ttatttgaat gttttttatt ttcgggtttt gttagttttg 2460
tggttttggg ttgtgtttat tttttttttt agttattttt tttggttgtt tttttatggg 2520
ttggggttgt tgttagtttg ttattcgttt ttttttgttt gtattttagg ttttatatag 2580
agggtcgggg gtttttgatt tcgagatcgt tttttatatt tgttagttat ttatgacgac 2640
gggttatttt ttttgttttc ggtttgattt taggtatttt ttgtagtgtg tttggttttt 2700
agggagtttt tgtttttttt ttttagggaa ttttagaggg atagtcgttt tgagttaagg 2760
gaattttttt tgtttttttt ttttttaaaa attaattttt agttgataaa taaaaattgt 2820

atatttgtat tatgtatatg ttttagttta ttttttaagt tgttaaatag aaattgtacg 2880
ttttcgtttt attttttata tttggtttgt tgtgcgtata gatttttagt ttttttggtt 2940
agttttttta tatgttggaa ttggtttgtt tttatttgtt attatggttt tgtcgttttt 3000
aaaagtagta tttagaattt tttgatttga gaggtttttt ggtttaatta atttagtatc 3060
gtttaggttt tttgtttcgt ggttttaaga tagaggttaa gaggacgaat aagatgtagt 3120
ttttgttttt agagagttta tagggggtttt gggggttgcg ttagtaattt aggagcgggg 3180
gatgggatga aggggttttc gtggagttgt ttataggttt ttgtggggat ttaaggttgg 3240
tttttagcgt ggttattaga aagggttttg gatagggtag cgtgtgagtt aggatttgag 3300
atgagtaggg ttttgaaacg agtttagggt atttaggttg cggtattaga agagatgaag 3360
agagataggg tggttgtttt tggtgtgttt ggggttggtt gtaggattga gttggaatgt 3420
gcgcgttttt tgtatttttt tagttttggg attttgagat attaggatag cgttattaat 3480
ttaggagtag tttttgtgtt tgtgtgtgta cgtgtgcgtt tgtgtgtagg ttggattttg 3540
attttaaaag ggttaggata tgaaaagaga cgagcgtttt agatttgagg aaatatacgc 3600
gtggaagacg tgcgcggggt tcgggttttt ttttgaggga gtgataagtc gtggaaagtt 3660
ttaatatggc gggcgggggt gggggcgata ggatttgttt gttttagcgg ttttatcgcg 3720
tgataattga gagtttagag ggggacggag agggagggaa gtcggttagg cgtcggggta 3780
tttagttagg tggcggggtt tgaacggggt ttcgaaatgt taaagaagag ataggtattg 3840
gagggattgt agttattggg atatatttgg ttagttgtcg agcgatggcg tggaaaggcg 3900
tagatgggag ttgtttggtt tggggttcgg gagggcggtt ttgttagggg agggtgtggg 3960
atttaggtta tggtttggtt ggttttcggt tattttagta tttagaagta agtgtgcgtt 4020
tggttgttgt tttattttgt aatatttttg tatttgatag cgggtgttag ggtcgtattt 4080
gtgaaaagtt tggggaggtt tttataaagt agggtggata tttattgtag gttttttgtt 4140
ggtggttttg gttaatttag gtatgaagag tgttttgaag gtatgtaatt ttgtgtttag 4200
tggtatggaa ttggtttgag ttgttttgta ttttagagta aagtgttaga tacgatatgt 4260
gtttagcgga gggagttggg ttgtttaggt tacgggttta tttgggatcg gttattaggg 4320
ggtcgtaggt tggtttttgt tttttttttg ggaatttttt attttgtttt tgaaattgaa 4380
agttagtgtt gtagttgatg atttagggaa aggagagggg ttttcgggag gcgttgtgat 4440
gcgtgtttgg gaatgaaaat gttggagtta ataagtttag gtggtagaga tatcgatagt 4500
tattagtaag atgatttttg atagtgaaag tgttcggaag agtatttttt atgatagagt 4560
gatgggagcg gtggtggatg tttttggatc ggtggttagg ggaggttttt ttgtagaggg 4620
atagttaagt tgagagttta gtgataagag gcgttagtgt gggaagtttt aggagtagag 4680
gattttaggt tagtgtggtg gtttcgaggt aggagtgagt tgggttgagt tggggatcgg 4740
tgaggaggtt gtgcgggtgg aattgaggat gaggcggtgg atgagaggag atacggggtt 4800
tgtgtgggag tacggagggg agaaggtacg ggacgtttgc ggtaaggtgt tggcgcgata 4860
ggaagtcgtt tgtgtgttgt ttattgggtt gagagcgtat tgtattgttg gggggtttta 4920
tcgaaagttt tttgtgggta aggggatagt tttttgtttg gaggcgtgtg ttgattttat 4980
ttttttattt tttttttttt tagtgtatcg tttaggtgag gttagagttg gggtatcgcg 5040
tttaattgcg taagaagttt attacggagg ggtttattta cgattggatg gtgtttgttc 5100
gcggtttcga gtaatgtgat atttagtatt tcgtggagaa ggtggttttt tggttgtacg 5160
atagtttttt taagtttaga cgcggtgagt ggttttaagt tttgagtgtt tatagtacgg 5220
atttatttgt ttttattttt tttaaggtta ggttttattt tttttttagg agtagtagga 5280
tttttttaat tatgaggttt tagtttgcgt ttttcggcgt gggggtattg gagtcgtgtt 5340
atttttagtt gttgagttag tcgtaggaag aatcgaagga ggtggtggcg gtttgttgtg 5400
tcgtgattta gattcgggtt ttgggaagta gttagggagt atagttaggt tggtatgggt 5460
gggataggtg aagttagtcg gttggtgagt gattaggaag ttgtcgatcg ggtttatgtt 5520
tgtttgtgtg ttatgtgaag aatttttaat atttggggta gaaagaatgt cggagtttat 5580
gttattattg tttttttcgt gttttttttt tttttttaag ttttaggtcg ttattgattt 5640
agtacgagtg ttttagtcgt gtattttgtt agcgttttta tttgtggcgt gggggttttg 5700
gtttttttgg agggtgtagt gtgacggggt ataggtagga agttatttag tttatgatta 5760
gggatgtttt tttggggaag tgagttttag ggaaaggttg tggtggggaa tcggagagtg 5820
aggtggtggt ttggtgagcg ttattagggt tggggtcgcg tttatttttt ttatcgttga 5880
gtcgttagtg tgaagttcgg agttcggtat attatagggg ttcgataaat gttttttggg 5940
tgatgttttg ataaaggaat gagggatttt ttgtaggaag tattttaggg tgggaagagt 6000
tttggttggg ttggtgattt ttttattttg taatacgggg gtttcgggag ttttaggggt 6060
tacgggaggt gagtcgtgta taagcgtggg tgtgtttagg ttgaagtaag agtttttcgg 6120
gattcgggat ttaggatttt ggttttgagt ttgtaagtgg tttgtagttt agtggggaag 6180
tgatgggcgt tgatagtagt agaggtttgt tattttgaag ggatgtttat ttgcgatttt 6240
tttagtttgc ggtttttttt tttttttgta tttttttttt tttgagtttt tatttttttt 6300
tttttttttt tttttttttt taagtttttt agaggatggt cgcggaggtt tttgatagcg 6360
gtagatgttt ataaatcgtt ttttaatcgt ggggatatat gttatttatc gagtattttt 6420
tggttagcgt ttcgtgcgta ggatcgcgtt tttttttcgt tatagttttt ttttgtagac 6480
ggggagacgg gtttaggggg taggtcgtta tttgtgttta gagtagggtg ggtaggttgg 6540
tttgtgggtt gggtttggtt attgttagtg tagtttgttt ttattggtgt acggttatgt 6600
tcgttcgtat aggtgttgtt tgtggttgtt tttgtgttac gttggtagag taggagtttg 6660
gttgtttttt ttgtattagg tttttttttt tttttttggt ggtatttatt ttattttttt 6720
attaattttt ttattttttt tttttttttt gtttttggag tatagggatg ggtttcggac 6780

gttgattttt atttatttg aggttgttga ttttattttt gtttttattt acgttgatat 6840
ggtatttttt ttaagtattt ggaaggtggg gtagtgagtg gggtttattt ttaagggtta 6900
gtttttagtt ttttgttttg ttttgttttt tgtttgtcgt gttgtttagg tttgttttga 6960
gtttttggta ggtatgtgtt attatgttag gttttagtgt tagtttgtag gcggttttta 7020
aattagtagt tgagttttgt ttgttgttgt ttgtttgtgg ggtttggagg tagatagcgg 7080
ttttattaaa tgtattagaa agtaaatttt tagtttatat tttgtggtat tgaataaaat 7140
tttagagagg gagtgaattt tttagagtat tttagttttt ggattgatat ttgaggtatt 7200
taaaaagtat tttttatttt cgttttagtt tatattatta taaattaaat agggtagaaa 7260
tatgtaaggt ataaagttgt tgtttttggt ttttttagtt tttttttttc ggggttgttt 7320
ttttagtttt ttttggattt tttttaatat tagtttattt gtttttattt ttttgatttt 7380
ttaaatgtag ataatattta ttgatttatt agtttgaagg gttagaattt attttaaagt 7440
atttttttta tttattgttt tttttaagtt tttaatagtt tagtgattcg tttttgcgtt 7500
ggttgttttt gtgattttga atgatgatgt gagtttgttt atttttggga ttttttatag 7560
aagagtttta cgttattttt tttagggagt tttttggttg tttgggttta atcgcggttt 7620
cgttgtttaa tatatttata tagttatatg ttttttagtt tttttgtttt ttgttttggt 7680
ttttttttta gtattatttt aatatttcgt atatatgatt tttttatttt gtttattttt 7740
tttttattt taagttagag gtttttgtta gttttgtttg ttgttatatg ttcggtgttt 7800
gtagggatat agtaggtgtt tattaagtgt ttgatgattg gatggatttt tgtatttggt 7860
tagtttttgt tagtgttttt tgatttttta ttgaggaggg taaggttatt agtattttgt 7920
ttttttttga ttttgttgta tttttttttt tgaggttata atggacgttg ttaagtttat 7980
tgacgaagat taagattttc gtttttcgat tatagattga ttgtaagagt tgggaattag 8040
tagtaggcgg cgttgatatg tttgacgttg ttattattag gttggttggg acggtaggat 8100
tttatttagt ttttgttttt tatgttttga attttgggtt attttagaga gaaggtttaa 8160
agttaggttt gtgtgaaacg ggtttttttt ggttattttt ggttaaaatt tggttttatt 8220
ttagtttgtt ttatatttgg tttgtggttt ttggaatatt tttttatttt ttttttttgt 8280
attgtgtttt tagtatattt ttattttttt tttatttttt tagttatttt atttattttg 8340
tggaattttt tttttttttt aattttttgg tcgggtgcgg tggtttatgt tagtattttg 8400
ggaggtcgag acgggtggat tatttgaggt taagaattta agattagttt ggttaatatg 8460
ttgaaattcg gtttttatta aaaatataaa aaaattagtc ggttgtggtg gcgtatgttt 8520
ttagttttag ttattcggga agttgaggta ggagaatcgt ttgaatttag gatgtagagg 8580
ttgtagtgag tcgagattat gttagtgtat ttcggtttgg gcgatagagc gaaattatgt 8640
tttaaaaaaa aaaatttttt tagagatggg ggtgtcgttg tgttgtttag attgaatttt 8700
aatttttggg gtttaagtag ttttttcgtt tttgttttag gagtagttga gtttataggt 8760
gttagtcgtt ttatttggtt ttttggtttg tttgtaaagt tttttgagtg ttttatgttg 8820
gttttttgtg tttttttttt ttaattataa cggattttta tttagttatt tttttagtta 8880
ttattttggg tttttttttt tttgtttttt tgaggtttgt ttatcggttt gtgttttggt 8940
ttattttttt gttttggtag agtagtgttt taagtaatat tttaagagat tattttagat 9000
atttttggtt gggcgtagtg gtttatgttt gtaattttag aattttggga ggttgaggta 9060
ggcggattat ttgagattag gagtttaaga ttagtttggt taatatggtg aaattttatt 9120
tttattaaaa ttataaaaag ttagttaggg gtagtgagga tgtttgtaat tttagttatt 9180
cgggaggtcg aggtaggaaa atcgtttgaa tttgggaggt agaggttgta gtgagttgag 9240
gtcgtattat tgtattttag tttgggtgat agagagaaat tttgttttaa aaaaataaat 9300
aaataaaaaa aatttataag acgtgggtag taaatttttt gagttttagt atgtttgaaa 9360
atgttattat ttttttttta tagtagaatg gttggttggg tgtagttttg tggtttggga 9420
attataggtt aagaattttt aagttgaaaa ttcgaaattt aaaatttttt tttttttttt 9480
ttttttggag atagaatttt attttgttat ttagattgga gtgtagtgtt gtgattatag 9540
tttattgtag ttttgatttt tgggttgaag tagttttttt gttttagttt tttaagtagt 9600
tgagattatt ggttcgttat tatatttagt taattttttt attttttgta gagacgggat 9660
tttattatgt tgtttaagtt ggttttgaat ttttgggttt aaaatttttt cgttttggtt 9720
ttttaaaatg ttgggattat cggcgtgagt tattatgttc ggtttaagat ttaaaatttt 9780
ttttgaaata gagttttatt ttgtcgttta ggttggagtg tagtggcgcg ttttcggttt 9840
attgtaattt tcgtttttcg ggtttaagcg atttttttgt tttagttttt tgagtagtta 9900
ggattataag tttttattat tatttttggt taatttttgt atttttagta gagatagggt 9960
tttattatgt tggttaggat ggttttaatt tgttaatttc gtgattcgtt tgttgtagtt 10020
ttttaaagtg ttgagattat aggtatgagt tattgtattt ggttaagatt taaaattttt 10080
gttgttgttg ttgaaataga gttttatttt tgttgtttag gttggagtgt aatggcgcga 10140
ttttggttta ttgtaatttt cgttttttag gtttaagtta tttttttgtt ttagtttttc 10200
gagtagttgg gattataggc gtgtattatt ttattttgtt aattttgtat ttttattaga 10260
gatggggttt ttttatgttg gttaggttgg ttttgaattt ttgattttag gtgattcgtt 10320
ttttttagtt ttttaaagta ttgggattat aggcgggagt tattgtattt agtcgattta 10380
aaattttttg agtgttgata tgatgtttag aaaatgttta ttggagtatt ttagattttg 10440
gatttttggg atgttgaatt agaggaatgc gaatatttta aagtttgaaa aattttaaat 10500
ttataatatt tttgttttta aatattttgg gagtatatat tatttagtgt tatgaaggcg 10560
ttgtgttagt attttttgga aagtttgatg ttattcggtt tttattttat tatatgtgat 10620
ttgttttttt ttgtggaaat atttaggatg tttttatgtt tttaggacgt ttgttttatg 10680
atattatgtt tttttttgtt tttttgtttt tttgtttttt ttttgttgtt tattatgttt 10740

tgtgatattt gagtgttttt tattttagtt ttgaaagttt ttttttattt atttgatagt 10800
ttttttttat ttgtttattt tttttttttt tttttttttg ggatttttat tttttttttt 10860
tttttttttt aatagagttt cgtttagtta tttaggttgg agggtagtgg tattattttg 10920
gtttattgta atttttatcg tttgggtgta agtgattttc gtgttttagt tttttaagta 10980
gttgggatta taggtgcgta ttattacgtt tggttaattt tttgtatttt tagtagagaa 11040
tgttatttag gttggttttg aatttttggt gttaagtgat ttttttattt cggtttttta 11100
gagtgtttag gattataggt gtgagttgtg gtgtttggtt gtaatttttt tttttagaag 11160
tattttattt ttatttgttg gatatatatt tttttgaatt tgaggatatt gatttttggt 11220
ttttattttt tttttttttt tttgttaaat tttttgtggt ttttaaatta tttgtttgtt 11280
cgggtttttt ttttttggtg ggagtttttg agcgttatgt gatgggaggg tgggttttgt 11340
ttatttatag gtttgaaggt ttacgggttt tttgattttt taatgagtaa ggtttttttc 11400
ggggtttggt ttttacgttg taagttttag tttttttttg atagttggtt ttttgagata 11460
tagaattatt ttaatttttt tagagggaaa tttgaaattt tttggggtgg ggaggggtgg 11520
agggggaatg ggcgggaagg aggatgggta ggagggaggg attttagggt ttttgtgttt 11580
aatatagttt tttagtttgt ttttttgttt ttttttaata atcgttttat ggagatgtaa 11640
tttatatatt ataaaattta tttttgtaat gtgtatagtt agcgttttta gtatatttat 11700
atagttgtgc ggttatgatt attgatttta ttattgttgt ttaattttag aatattgttg 11760
ttattttagg gagaagttta ggtttcgtag tggtagtcgt ttttttgttt ttattttttt 11820
tttttatcgt gttttttttt ttttttttag tttttatttt tatttttgtt tttcgtttag 11880
ttagtttagg atttttagtt ttttggtatt agtggttgtt tttgttttgc ggcgttttta 11940
tggtagattt tgagtcgtcg tttttttgtt ttgttttttg tttatcgtgt tttcgttcga 12000
tttttgtttt ttagaaattg gtaggtggtt tgaatttttt tttttttgtt tttattttta 12060
cggttttatt tttgtttttt tgttttttgt gggtttttag gtaggagagt gggtcggtgc 12120
gtttttttgt ttttttttgg ttgaattgaa gtattcggtt aattgttatt ttttttggcg 12180
ttgtttgggg ggatagaagt tttttaggtt tggagtattc gttataggat aggatttttt 12240
tttgaaggcg tggggattat gagataagat gtgatagtcg atttgggaag tacgtgattt 12300
ggaggttaga ggtagaggga tatggggttt gtttttcgaa ggaggtggtt ttgttgtggg 12360
tttttgtggt tgggtgggat ttagattatg ggcgagtggg gagaagacga ggtatgggtg 12420
gagtttgaga gtaaatttta ggttattgtt tatgttcgtg ttttttttgt tggggatagg 12480
ttattttttt tttgttttgt tttcgttttt ttttttaggt ttagattaag tatttaggtg 12540
tttggtttta tgttgtattt tgtttattta ttaatttttt aaatggagtt tcgttttgtt 12600
atttaggttg gagtgtagtg gtataatttt cgtttattgt aatttttgtt tttggggttt 12660
aagcgatttt tttgttttag ttttttgagt agttgggatt ataggtgtgt gttattacgt 12720
ttagttaatt tttgtgtttt tattagagac ggagttttat tatgttggtt aggttggttt 12780
cgaatttttg atttgaagtg attcgtttgt tttggttttt taaagtgttg ggattatagg 12840
tgtgagttat cgtgttcggt tttttttttt ttttttttaa tttttggggt taatggggtt 12900
ttgtttgcgt tatttgggaa tgttttattt aggtgttaaa ttagaattta ggaggagggt 12960
tttagttatt tggggttttt ttgtttttta gtgtatgtgt tcgtggtgta gtaggtgttt 13020
agttttcgtg ttggaggtgg tgatagtaga gtgtgtggtt ttttttgttg ggttttatat 13080
tttatttagg ttacgtttat cgatatttat ttgagtcggg taaatttgtg tagttttttt 13140
tggttttagt ttttttattt tttgtaggag taagtatttc gatgataggt taacgttttt 13200
ttttattttt tgttgattat gtcggtggtt aagttttttt tttttttttt ttttttgttg 13260
ttttattttt aagataattt tgtcgtggaa gttaattttg tcgggggttt aattttttga 13320
aaagttattt tcgagaggtt agttaagttt ttgtgaggag gtggtggggt tgaggggtgc 13380
ggtattgtcg ttggtagttg ggtttgaaat aggtaggagg ttgatgggtg tgtttttttt 13440
tttttttttt agt 13453

<210> 194
<211> 13453
<212> DNA
<213> Artificial Sequence

<220>
<223> chemically treated genomic DNA (Homo sapiens)

<400> 194

attaaaaaga aaaggaagaa atatatttat tagttttttg tttgttttag gtttagttgt 60
tagcggtagt atcgtatttt ttaattttat tattttttta taggggtttg gttggttttt 120
cgggggtggt tttttaggag gttaagtttt cgataggatt gatttttacg gtaaagttat 180
tttaggaata aaatagtagg gaagaagagg agaaaggaga tttggttatc ggtatggtta 240
gtagagagtg agaaggaacg ttagtttgtt atcgggatat ttgtttttgt agaggatgag 300
gaggttgagg ttagagaagg ttgtataagt ttgttcgatt taggtggatg tcggtaagcg 360
tgatttgggt gaggtgtgaa atttagtagg gagggttata tattttattg ttattatttt 420
tagtacgggg gttgagtatt tgttgtatta cggatatatg tattgggaga taggggagtt 480
ttaggtggtt gggatttttt ttttgggttt tagtttagta tttaataaga gtatttttaa 540

gtgacgtaaa taaaatttta ttgattttag agattaaaaa agaaaaaaaa aaggtcggat 600
acggtggttt atatttgtaa ttttagtatt ttgggaggtt aaggtaggcg gattatttta 660
agttaggagt tcgagattag tttggttaat atgatgaaat ttcgttttta ataaaaatat 720
aaaaattagt tgggcgtggt ggtatatatt tgtaatttta gttatttagg aagttgaagt 780
aggagaatcg tttgaatttt agaggtagag gttgtagtaa gcggagattg tgttattgta 840
ttttagtttg ggtaatagag cgagatttta tttaaaaaat taataaataa ataaagtgta 900
gtatgaggtt aggtatttga atatttaatt tgaatttggg gaggaaagcg gaggtagaat 960
agaggaaggg tggtttgttt ttagtagagg aggtacgggt atgggtagtg gtttgggatt 1020
tgtttttaga ttttatttat gtttcgtttt ttttttattc gtttatagtt tgaattttat 1080
ttagttataa aggtttatag tagggttatt tttttcgaga aatagatttt atgttttttt 1140
gtttttgatt tttaggttac gtgtttttta aatcgattgt tatattttat tttatggttt 1200
ttacgttttt aagaaggaat tttgttttgt gacgggtatt ttaagtttga agaattttta 1260
tttttttaga tagcgttaag agggatggta gttggtcgag tgttttaatt taattagggg 1320
agaataggga gacgtatcgg tttatttttt tgtttgagag tttataggaa gtaagaggat 1380
aggaataagg tcgtgagagt gaaggtagga gggaggagat ttaagttatt tgttagtttt 1440
tgaaaggtag aagtcggacg aggatacgat gggtaagggg taggataagg aggcgacggt 1500
ttagagtttg ttatgggggc gtcgtagggt agggatagtt attggtgtta gaaggttgaa 1560
ggttttggat tggttgagcg gagggtagga gtgaggatga aagttggggg agggaaaggg 1620
agtacggtgg gggaggaggg tgggagtagg ggagcgattg ttattgcggg gtttgagttt 1680
ttttttgggg tgatagtaat gttttggaat tagatagtag tgatggaatt agtggttatg 1740
gtcgtataat tatgtgaata tattgaaaac gttaattgta tatattataa gagtgaattt 1800
tatgatgtgt aaattatatt tttataaagc ggttattaaa aaaaaataaa aggtaaattt 1860
gaaaggttgt gttgggtata ggagttttgg gatttttttt ttttgtttat tttttttttc 1920
gtttattttt tttttatttt tttttatttt aggaagtttt aggttttttt ttggagaaat 1980
tgagatggtt ttgtgtttta aggagttaat tattaggggg aagttgaaat ttgtagcgtg 2040
gaaattagat ttcgaggaag gttttattta ttaggaaatt aggaggttcg tgagttttta 2100
ggtttgtgga taggtagaat ttattttttt attatataac gtttaggagt ttttattagg 2160
aggaaagagt tcgagtaaat aaataatttg gagattatag agagtttgat aaaaagaaag 2220
aaaaaaaata aaaattaaga gttaatgttt ttagatttaa ggagatatat atttaataaa 2280
taaaaataag atatttttaa aaggggaaat tatagttagg tattatagtt tatatttgta 2340
attttaggta ttttgggagg tcgaggtggg aggattattt gatattagga gtttaaggtt 2400
aatttggatg atatttttta ttaaaaatat aaaaaattag ttaggcgtgg tggtgcgtat 2460
ttgtaatttt agttatttgg gaggttgagg tacgagaatt atttgtattt aggcggtgga 2520
ggttgtagtg agttaagata gtgttattgt tttttagttt aggtgattga gcgaaatttt 2580
gttaaaaaaa aaaaaaaaaa aaaatgagga ttttagaaag aaaaaaagag aggaaataaa 2640
tagatggaag gaaattgtta aataaataga aggaaatttt tagagttaaa atgaaagata 2700
tttaaatgtt ataaaatata gtgaatagta aaaaaaaaat aaaaaaataa aaaaataaaa 2760
aaaaatatga tgttataaag taagcgtttt aaaaatataa aaatatttta aatgttttta 2820
tagaaaaaaa taggttatat ataatgggat gaggatcgaa tggtattaga ttttttagaa 2880
gatattaata taacgttttt atagtattaa gtgatatata tttttaaaat gtttgggagt 2940
aggagtgttg tgaatttggg attttttaga ttttggaata ttcgtatttt tttggtttag 3000
tattttaaaa atttaaaatt taaaatgttt tagtgagtat tttttgagta ttatgttagt 3060
atttaaaaag ttttggatcg gttgggtgta gtggttttcg tttgtaattt tagtattttg 3120
ggaggttgag gaaggcgaat tatttgaagt taggagttta agattagttt gattaatatg 3180
gagaaatttt atttttaata aaaatataaa attagtaagg tgaggtggtg tacgtttgta 3240
attttagtta ttcgggaggt tgaggtaggg gaatagtttg aatttgggag gcggaggttg 3300
tagtgagtta agatcgcgtt attgtatttt agtttgggta ataagagtga aattttgttt 3360
taataataat aataaaagtt ttggattttg gttaggtgta atggtttatg tttgtaattt 3420
tagtattttg ggaggttgta gtaggcggat tacgaggtta atagattgag gttattttga 3480
ttaatatggt gaaattttgt ttttattaaa aatataaaaa ttagttaagg gtggtggtgg 3540
gggtttgtag ttttagttat ttaggaggtt gaggtaggag aatcgtttga attcgggagg 3600
cggaggttgt agtgagtcga gaacgcgtta ttgtatttta gtttgggcga tagagtgaga 3660
ttttgtttta aaaaaagttt tggattttaa gtcgggtatg gtggtttacg tcggtaattt 3720
tagtattttg ggaggttaag gcgggaagat tttaagttta agagtttaag attaatttgg 3780
gtaatatggt gaaatttcgt ttttgtaaaa aataaaaaag ttagttgggt atggtggcgg 3840
gttagtagtt ttagttattt gggaggttga ggtaggagga ttgttttagt ttaaaggtta 3900
aggttgtagt aagttgtgat tataatattg tattttagtt tgggtggtag aatgagattt 3960
tgtttttaaa aaaaaaaaaa aaaaaaaagt tttggatttc ggatttttag tttagggatt 4020
tttgatttgt aatttttaaa ttataaaatt atatttaatt aattatttta ttgtgagaag 4080
agaataatgg tatttttaaa tatgttagga tttaaagggt ttattgttta cgttttataa 4140
attttttttg tttgtttgtt tttttgagat agagtttttt tttgttattt aggttggagt 4200
gtagtggtac gattttagtt tattgtaatt tttgtttttt aggtttaagc gatttttttg 4260
tttcggtttt tcgagtagtt gggattatag gtatttttat tgtttttggt taattttttg 4320
taattttagt agagatgggg ttttattatg ttggttagat tggttttgaa tttttgattt 4380
taggtgattc gtttgtttta gttttttaaa gttttgggat tataggtatg agttattgcg 4440
tttagttaga aatgtttgaa gtaatttttt ggaatattat ttgagatatt gttttattaa 4500

aataagaaaa taaattaaaa tataggtcga taaataaatt ttaggagaat aggagagaaa 4560
aggtttaggg tagtggttaa aggggtggtt ggataagagt tcgttgtgat tagagaagga 4620
aggtatagag aattagtatg aaatatttag aggattttat aaataagtta gaaagttagg 4680
taaggcggtt gatatttgta aatttagtta tttttgaagt agaggcggga ggattgtttg 4740
agtttaagga gttggagttt agtttgggta atatagcgat atttttattt ttaaaagaat 4800
tttttttttt gagatatagt ttcgttttgt cgtttaggtc ggagtgtatt ggtatgattt 4860
cggtttattg taatttttgt attttgggtt taagcgattt ttttgtttta gtttttcgag 4920
tagttgggat taaaggtatg cgttattata gtcggttaat ttttttgtat ttttagtaga 4980
gatcgagttt tagtatattg gttaggttgg ttttgaattt ttgattttag gtgatttatt 5040
cgtttcggtt ttttaaagtg ttggtatgaa ttatcgtatt cggttaaaaa attaaaaaaa 5100
aaaaaaaatt ttatagaata aatgaaatga ttgagagaat ggggggaaaa tgaaggtatg 5160
ttaaagatat aatgtaaaaa ggagaagtaa gggggtattt taaaagttat agattaaatg 5220
tgaagtaagt taaaataggg ttaaatttta attaaaaatg attaaaaaga attcgtttta 5280
tatagatttg attttgagtt ttttttttgg agtggtttag agtttaggat atgggaaata 5340
gaagttgaat gaagttttgt cgttttaatt agtttgatgg tgatagcgtt aaatatgtta 5400
acgtcgttta ttgttagttt ttaattttta tagttaattt ataatcgggg agcgaaggtt 5460
ttggttttcg ttaataagtt taataacgtt tattgtagtt ttaaaaagag gggtgtagta 5520
gggttaaaaa gagatagggt attggtggtt ttgttttttt tagtggagag ttagaaaata 5580
ttaataaaag ttgattaggt gtaagggttt atttagttat taaatattta gtaagtattt 5640
attgtatttt tgtaggtatc gaatatgtag tagtaaataa aattgataga aatttttgat 5700
ttggggtggg gagagggatg gataagatga agaagttata tgtacgagat gttaagatgg 5760
tgttaaggag aaaattaaag tagaaaataa aaaggttaag aagtatatgg ttgtgtgggt 5820
gtattgggta acgaaatcgc gattgagttt aggtagttag aggatttttt aaaaagggtg 5880
acgtgggatt tttttgtgaa ggattttaaa ggtaaatagg tttatattat tatttaaagt 5940
tataaagata attaacgtaa aagcgagtta ttggattatt aaaaatttag agaagatagt 6000
ggataaaagg ggtattttgg gataaatttt aattttttaa gttggtaaat tagtaagtat 6060
tgtttatatt taaaaaatta agaaataaga agtaaatgag ttggtattaa gaggagttta 6120
gagagggtta ggaagatagt ttcggggagg gaggattaag gaggttagga atagtaattt 6180
tgtattttat atgtttttat tttatttgat ttgtgataat gtaagttggg gcggaagtaa 6240
aagatatttt ttaagtattt taagtattaa tttagaggtt ggagtgtttt aagaggttta 6300
tttttttttt aaagttttat ttaatgttat agagtgtgag ttggaaattt attttttgat 6360
atatttgatg gggtcgttat ttgtttttag gttttataga tagatagtaa taaatagggt 6420
ttagttatta atttaaaggt cgtttgtaaa ttgatattga agtttggtat ggtggtatat 6480
gtttgttagg agtttaagat aagtttgggt aatacgataa ataaaaaata aaataaaata 6540
aaaaattaaa aattaatttt taagagtgag ttttatttat tattttattt tttaggtatt 6600
tggagaaggt gttatgttaa cgtggtgggg aataaaggtg aaattaatag ttttaaagtg 6660
gatggaaatt agcgttcggg gtttattttt gtgttttagg gatagagaaa aggggagggg 6720
tgagggggtt ggtgaagaag tggagtggat gttattagag aaaggaaaga gggtttggtg 6780
taggaggggt agttaggttt ttgttttgtt aacgtggtat aaaagtagtt ataggtaata 6840
tttatgcggg cgggtatggt cgtgtattaa taaaaatagg ttgtattggt agtggttaga 6900
tttagtttat aggttagttt gtttattttg ttttaagtat aggtagcggt ttgttttttg 6960
gattcgtttt ttcgtttgta gaagagggtt gtggcgagaa gaaaacgcga ttttgcgtac 7020
gaagcgttgg ttaggaagtg ttcgatagat ggtatgtgtt tttacgatta ggaagcggtt 7080
tataaatatt tgtcgttgtt aggagttttc gcggttattt tttaagaggt ttagagaggg 7140
aggaaggaaa aggggaaaag gtgaagattt aggaaaggag aaatgtagag ggaaagagaa 7200
gtcgtaggtt gagggaatcg taggtgggta tttttttaga atgataagtt tttattgttg 7260
ttaacgttta ttattttttt attaggttgt aaattattta taggtttagg gttagggttt 7320
tgggtttcgg gtttcgggaa gtttttgttt taatttaagt atatttacgt ttgtgtacgg 7380
tttatttttc gtggtttttg gagttttcgg gattttcgta ttgtagggta gaggggttat 7440
tagtttagtt agggtttttt ttattttgaa gtgtttttta tagaaagttt tttatttttt 7500
tattaagata ttatttaaga agtatttatc gaatttttgt gatgtgtcgg gtttcgggtt 7560
ttatattggc gatttagcgg taagaaaagt agacgcggtt ttagttttga tggcgtttat 7620
tagattatta ttttattttt cgatttttta ttatagtttt tttttagggt ttattttttt 7680
aggagggtat ttttgattat aaattgggtg attttttgtt tgtgtttcgt tatattgtat 7740
tttttaggaa ggttagggtt tttacgttat aggtggagac gttgatagaa tatacgattg 7800
aggtattcgt gttgaattaa tgacggtttg aggtttgaag ggagagggaa gatacggaaa 7860
agatagtggt gatatgagtt tcggtatttt ttttgtttta aatattggga attttttata 7920
tggtatataa ataaatatag gttcggtcgg taattttttg attatttatt agtcggttga 7980
ttttatttgt tttatttatg ttaatttggt tgtgtttttt ggttgttttt tagagttcgg 8040
gtttaaatta cgatataata gatcgttatt atttttttcg gtttttttta cgattaattt 8100
aatagttgga ggtgatacgg ttttagtgtt tttacgtcga gggacgtaag ttggaatttt 8160
atggttggag gggttttgtt gtttttgagg gaggggtgga gtttggtttt gggaggagtg 8220
aagatagatg ggttcgtgtt gtgggtattt agggtttgag gttatttatc gcgtttgggt 8280
ttggggaagt tgtcgtgtag ttagaagatt atttttttta cgaagtgttg gatgttatat 8340
tgttcggggt cgcggataaa tattatttag tcgtgagtga attttttcgt ggtgggtttt 8400
ttgcgtagtt gggcgcgatg ttttagtttt aattttattt ggacggtgta ttggaggaga 8460

gagaggtggg gagatgaagt tagtatacgt ttttaagtag gggattgttt ttttatttat 8520
agagaatttt cgataaagat ttttagtagt gtaatacgtt tttaatttag tgagtagtat 8580
atagacggtt ttttatcgcg ttagtatttt gtcgtagacg tttcgtgttt tttttttttc 8640
gtatttttat atagatttcg tgtttttttt tatttatcgt tttattttta attttattcg 8700
tatagttttt ttatcggttt ttagtttagt ttagtttatt tttgtttcgg ggttattata 8760
ttggtttagg attttttgtt tttgagattt tttatattgg cgttttttgt tattgggttt 8820
ttagtttgat tgtttttttg tagggaagtt ttttttgatt atcgatttaa aagtatttat 8880
tatcgttttt attattttat tatagaggat gttttttcga gtatttttat tattaaaagt 8940
tattttgttg atggttgtcg gtgtttttgt tatttaagtt tgttgatttt agtattttta 9000
tttttaagta cgtattatag cgtttttcgg aggttttttt ttttttttta agttattagt 9060
tataatattg gtttttagtt ttaaaagtag aatgggaggt ttttaagaag aaggtagagg 9120
ttagtttgcg gttttttggt gatcggtttt aggtggattc gtggtttggg tagtttagtt 9180
tttttcgttg agtatatgtc gtgtttgata ttttgttttg aaatataagg tagtttaggt 9240
tagttttatg ttattgaata taaagttgta tgtttttaaa gtatttttta tatttaggtt 9300
ggttaaaatt attaataggg aatttatagt aaatatttat tttgttttgt aaaaattttt 9360
ttagattttt tatagatacg gttttgatat tcgttattaa atataagaat gttataaaat 9420
aaagtagtag ttaagcgtat atttgttttt aagtgttgag atagtcggga gttagttaga 9480
ttatggtttg agttttatat ttttttttga tagagtcgtt ttttcgggtt ttagattaga 9540
tagtttttat ttgcgttttt ttacgttatc gttcggtaat tggttaggtg tgttttagtg 9600
gttgtaattt ttttagtatt tgtttttttt ttggtatttc gaggtttcgt ttagatttcg 9660
ttatttggtt gagtgtttcg gcgtttggtc ggtttttttt ttttttcgtt tttttttgag 9720
tttttagttg ttacgcgatg gagtcgttgg ggtaggtaga ttttatcgtt tttattttcg 9780
ttcgttatgt taaagttttt tacggtttgt tattttttta gaaggaagtt cgagtttcgc 9840
gtacgttttt tacgcgtgta tttttttaag tttgagacgt tcgttttttt ttatgtttta 9900
atttttttga agttagagtt tagtttgtat ataggcgtat acgtgtatat atagatatag 9960
aagttgtttt tgagttggtg gcgttgtttt ggtattttag agttttagaa ttgaggaaat 10020
gtaggaaacg cgtatatttt agtttaattt tatagttagt tttagatata ttaggaatag 10080
ttattttatt tttttttatt ttttttagtg tcgtagtttg ggtgttttaa gttcgtttta 10140
aaattttatt tattttaagt tttggtttat acgttgtttt gtttagggtt ttttttggta 10200
gttacgttgg agattaattt tgagttttta tagaagtttg tggatagttt tacgggagtt 10260
tttttatttt atttttcgtt tttgggttgt tgacgtagtt tttagggttt ttgtgaattt 10320
tttgagagta gggattatat tttgttcgtt tttttagttt ttattttgaa attacgggat 10380
aggaggtttg gacgatgttg gattaattaa attagagagt tttttaggtt agggagtttt 10440
aaatgttgtt tttggagacg gtagggttat ggtggtagat aaggataggt tagttttagt 10500
atgtggaaga attggttaga gaggttgagg gtttgtgcgt atagtaagtt aggtataaga 10560
aatgaggcgg aggcgtgtaa tttttatttg gtaatttaaa aagtaaattg aaatatgtat 10620
atgatataga tgtataattt ttatttgtta attaaaaatt aatttttaaa aggaaagggg 10680
gtagagggga tttttttggt ttaggacgat tgtttttttg gagttttttg ggaaaagagg 10740
gtagggattt tttagggatt aggtatattg tagggggtgt ttgggattag gtcggagata 10800
aaagaaataa ttcgtcgtta taagtggttg atagatatgg gaaacggttt cggagttaaa 10860
agttttcggt tttttgtgta aggtttgggg tgtaggtaga aaagggcgaa tggtaaatta 10920
atagtaattt tagtttatga ggaggtaatt agaaggagtg gttggaaaaa ggggtgaata 10980
tagtttaaag ttatagaatt gataggattc gagggtggag gatatttagg taattttaag 11040
attcgggtag tagggatatt tacgatttta gtgtgggaag aaagtgggga tggacggaag 11100
attaggcgta tttataaatt ttcggcgttg cggatttatg aggattttag gtaaaagtat 11160
ttttattaaa tatacgaggt gagggttgag tatcggtatg gtaatattta ggttaagtac 11220
gagtgtttgt ttgaatatta tcgtatttta ataaatttaa aataatattg atattagttt 11280
cgagcggtta tgattatttt ttatgtttat acgtttgcgt ttttgttttt ttcgttagaa 11340
aataggaagg taatttttgt ttcgttggga gtattatagg tgaaaattaa atgtatatat 11400
atattatggg aatagtatta ggatgcggga gaacgatttt tgtttttgtg gagttgattt 11460
ttatataatg atagtgtttt ttcggaatcg tttatttatt gattcggtta tttagatttc 11520
ggtcgatttt tcggtatttt tgatgatagg gagtttttat ttagggtacg taggcggtag 11580
agtagttatt gtgaaagagt gaaagatcgc ggttttattg gagaggggga ggatggaagt 11640
attagaattt tatcgtagtg gttaaagtaa ataggaaggt ttcgaggtag cgatgggtaa 11700
tttttgtagt cggtaggtcg ggtttaaagt gttgtttagg cgatattgat gttttggggg 11760
ttggcgttaa tttgagtttt aatatttatt gttaaggata tattttagat ttggttaata 11820
gatgcgaagt tcgggtgttc ggtttgttgt atttagggga ttgtggagcg ttgagggggg 11880
tatttttcgg aagggtggaa taatagagtt tttgttgttg tagtaaaaaa ggaattatag 11940
atttagtaag acgtttaagt tacgttaacg ggaggtaaga agtggagttt cggttaacgc 12000
gtttgatttg ttaggttatg ataatttatt agtatagtta agttaatttt atagttaagg 12060
gtaattattt tttttttttt atattatttg gatttttttt ttggttatta ggattaaaga 12120
attgaagtta attaattagt tgttttttta ggaagggtgt ttggttgtag aggttaagtg 12180
ataggttttt tagaatgttt tagagtgaat aatttgattg gttagtggtt tttttttaat 12240
tttattttag taagtttttt taaggtatta ggaatgtttg ttgttgggag gttatttttt 12300
tggaattttt ggaatagacg gttggaaatg agtcgggggg ttgtggtttt agggattttt 12360
atttttttgt atttttggga ttattttgat atttattttt taataaaagt ttaggataaa 12420

ggggaatttt ttaggattgt ttcggaaaga gttgttcgtt ttatttttgg gaaattattt 12480
gaaagatagc ggacggaggg atgggtacga gcgttttaag ggttatttgg ggatagttag 12540
gtttgggtag agggttttag ttgggatatt gttttttttt taggttagtt ggtagagggt 12600
atttggaatt aaagggttaa aattttaaat taggtaaatt aggtagtttt tcgagggtag 12660
ttttttcggg gtttttttaa cgtagcgtgc ggtttttttt aggtgtgggt tatagcggtc 12720
gtcgtttggt aaagagtcgc gagttagttt tgttttaata gttttatata atttttttag 12780
ttaatgtatt ttattttttg taagttttga aggtaggttt ttaataatta gtttatttga 12840
agattttttt tggttttttg gtgggaattt ttaatagatg tttttttttt gggaatttgg 12900
atttcgttta gtggtggaat tatatttgga gttcggttat tgtatttaat tttttcggag 12960
gtaacgattt ttttttattt taggagagga gaatattgag gagtcgtttt tttttttagt 13020
ttaaattgat tttgaggttt ttttgggttg aatacgtgta cgtgtgtgag ttatatatgt 13080
ataaatagga agaggtgacg aggtttgggc gattttatgg agcgacggta atatgagatt 13140
taataaaatt ttttgcggaa ttttttttcg ttaagtttag tcggggtttt tagtggtaat 13200
tattgaatta gaatgtaaga gatggggaaa gataggtgtt tttttgtgat agttttttga 13260
agcggagagt tgtttattta gaaggtaggg gttttttatt aataggtata tcgggttttg 13320
gttttttgat tgttggtata ttggggttag gggttagtat ttttattttt tttttggttt 13380
tatttgtttt cgtttttttg atttaaggta taatgataaa gcgatgagaa ggtatttaat 13440
gaattttttt ttg 13453

<210> 195
<211> 7906
<212> DNA
<213> Artificial Sequence

<220>
<223> chemically treated genomic DNA (Homo sapiens)

<400> 195

agtaattata atttttaata agcgtatttt aagagcggaa gtatagggtg tattttataa 60
attgtaaagg tcgatgcgtg tattttttgg gatttttgag aaggtttttt tttttttatt 120
ttttaatttt tttatttagt taggacgttt tttttttcgg atttttattg ttattttttt 180
ttttattggt gttttttttt agatataggt ttttgttttt tgaattttag tttttttaaa 240
tataaaaaga agaaaattaa attttttttt ttatggagga ttatgaggat ttaaatatat 300
gaattaatat agagaatgag gttggtatcg agttttgtgg gtttttagga gtgtttagtt 360
attattatta ttattattat tattattatt attattatta ttattattat taatagttta 420
gaaaatagtg agtaaaagaa gaaagaaaaa ttatttttga aaattttttt ttagtagtaa 480
ttattattaa taattggaga ataatttttt tggtttttaa ataaaatgta tgaagtgaat 540
ttttaggaat ttattatatt gtgtaaattg tttggagatt tatttttgtt atataataat 600
atatcgtgag taatttgata tgttattatg ttattttgtg tattattgtt ttagtggttt 660
ttaaattatt tatagtatta gtatattatt gtttaatgta atagtgattt tatggattat 720
tgttaaagtt ggtttttaaa tttttttttt aagtaatttt tattttttag ggttttattt 780
ttggtttagg tttttttttt tttagtttcg tttatagatt tttattaagg taaaatttag 840
tattaaatat attatttatt cgtttaaata ttttttatgg ttttttagtg tttaatagtt 900
gaaattgtat ttttattgtt tggtagttaa agttttttat aaattattgt tttaattttg 960
tttagttttt atattttttt tgttatattt tatatgttat tttaaggata ttttttattt 1020
ttgttgtttt tagtatttat aaggaatttt tttttttatt tttgttttag ttttatttat 1080
tttttaaatt ttaatttaag tttatttttt tttttttagt tgatttattg tattatttag 1140
gattatattt aattggtaag aaataagatt tagtattata gtgacgttaa tataatacga 1200
gtttatttat ttttgtatag aagatgatta gaaatagaag atttaggggt aggtatggta 1260
gttttttagt tattagggat cgagtttttt tttggttttt ttttttttta tttttggggt 1320
gtatatttat ttttgtggtt tgatatggtt gttagagttt tagatattat atttatattt 1380
ttgttagaag gtttttgagg aaaatgggag aagagtatat tcgttttttt ttagaaagtt 1440
ttttagaagt attatataag atttttatgt atattttatt agttagaaag tgtttatttg 1500
gttattttgt tgtaagagag gttggggaac gtagtaacgt gtttattatt aagaaagaag 1560
ggagcgtgga tacgggagat attttagttt tttataatga gatagaaaag tttttttttt 1620
taagtataat gttgagtttt tattttaggt agtagtagtg tgtgttggaa gatataggtt 1680
ttggagttag atagatttgg ggtagatttt ggtttagtta ttgaatttta gttagatgat 1740
ttagaaaagt tgtttagatt ttttgaattt ttgtatttaa aaaatagaaa aggcggggtg 1800
gggggagtag ttgtgagaag gtaatcgatt agaatttgtg tatagagaat ataggagggt 1860
gtttagtata agtttgatgg ttatgaattg ttatttttat tagtattgtg ttatttatat 1920
ttatgtattt tatattttat aatattgtgt tatttaaatt tttttattat aataatattt 1980
ttattttatt ttattttaaa gataagattt tgttttgtta tttaggttga attgtagtgg 2040
tgtgattatg gtttagtgta gttttaaatt tttgggttta agtgattttt aagatttagt 2100
tttttgagta gttgagggaa gtttaggtat ttggtatgta gttagggttg ttagtaatat 2160
ttgttgtatt aaatggaatt atattgtttt gatttttttt ttgggtttgg ttttattttg 2220

ttttcgttat tgggatattg tgtataatgt ggttttagta ggtttgggat ggggtttgga 2280
aatttatatt ttttattttt ttagtattgt aaattttttt tttagaaggt tttttaggag 2340
tattatataa gatttttgtg taggtcgggt atagtggttt atgtttataa ttttagtatt 2400
ttgggaggtt aaggtagggg gattatttgg ggttgggagt tcgagattag tttggttaat 2460
atggtgaaat tttgttttta ttaaaaatat aaaattagtt aggtatgttg gggtatgttt 2520
gtagttttag ttatttggga ggttgaggta tgagaatcgt ttgaattcgg gaggtggagg 2580
ttatagtgag ttgagatcgt gttattgtat tttagtttat taagaataaa taaataaata 2640
aataaaaaaa tttttgttta ttattgtagt tttaattttt tgggtttaag taattttttt 2700
atttagtttt tttaaatagt tggaattata ggtacgtatt attatgttta gttaattttt 2760
tttttttttt gtagagatag agttcgtcgt tttggaatcg tttgaatttg ggaggcggag 2820
gttggagtga attgagatcg cgttattgta ttttagtttg ggcgataaga gtaaaatttc 2880
gttttaaaga agaggaagag gaagaagaag gaggaggagg aggaggagat gaggaggaga 2940
ggaggaagag gaggatgagg aggaggagaa aagaggaaga aaaagaggaa gaagaagaag 3000
aagaggaaga agaggaagat gttgttgttc gttgttttgg ttgggttatt agtaaggtat 3060
ttgttttata gttttgttta gttttagcga gttgagtttt aaaattgggt tttaaggttt 3120
tttatttttg tattttttcg ggtaaatttt cggaggtttc ggtgtatcgc gcgtttagtc 3180
ggtttaattc gagttagaag ttttaattat tgtttagtgt ttgagttgta gttttgggtt 3240
ttttagaaat ttggagatgt gcgtaaaatt tagatgtcgg tattttcgaa tttttttagg 3300
ttttagtata tttcggcggt ttgtggatag atgggaggtt attaatcgtt tcggcgttcg 3360
tagttcgatt tttgtcgtta gatttcggac gtttttcgga taataaagtt ttcgttttaa 3420
tttatttttt ttaatttgga cgtttttaat ttatagtttt tattgcgttt agttaaaagt 3480
cgagggaatt cgttgttttt tcgcgttcgg ataattattt ttaaatggtt acggtagttt 3540
tttgtgtttt ttggagatta gaatttcgta gttattaatg gtagggtcga gtgagtcgtt 3600
aattattttc gtttattttt tgagagtcgt tggtttgggt cgcgggagga gaggttataa 3660
agcgataggc gtagaaaatg gttaagtttc gatttcgttt taggtagatt ttgtattttt 3720
tttgatttta atttattttt tttgttttta ttattattat tattattaat agtaaatatt 3780
aaattacgtt tttttcgaaa ttggtaaaag aagagttttt aagttttata ttagagttta 3840
tttttaggaa tttttttttt tttttagttt ttttttttaa atgtgttttt tttttttagg 3900
attagcggtt tagagatagg gatatgttat tgttttcgag gacgcgttgt ttgaggttat 3960
tttaaggtta gatcggtgtt ttattgacga ttttgttggt tttattttag taagttttat 4020
attaaaagga tttggtagtt ttttttttcg ttcgggcgtt ataggtgttg tgatttttgg 4080
gatagaagtt tagagcgagg gtgggggggg gggaatatat atcgtgtatg aaaatcgatt 4140
gtagatttta gataatttta cgtattttta tatttttggt attataggaa gtagtttttt 4200
ttcgtaaggt ttattttagt tttaagttag agataaaggt ttatttttat ttcgaggtaa 4260
agtttataag attttagaat aggaagaaaa tgaaggtttg gttttgtttc gttttttgaa 4320
aagaagttaa tagtatgttt tttttttagg ataaatagtt atgcgtattt taaaaattat 4380
atataaaagg aatgtgtaag aaataatttt aatttaaatt attgtggtag aagaagaggg 4440
ggggttagat agtggagggg ggtataggga aatttagtta tagattaaag agaaaggtaa 4500
aagaagtagt agaggagaga aataaggacg gggaaaaaaa gaggagcgga aaagagggtt 4560
gaggagggga ggggaggagg gtagaagaga aggaacgaga ataagggaaa attttttcgg 4620
gaatatagaa agatagagat ttaggggatt ttcgtagaga gggttttttg ggttttagcg 4680
tagaggaaag tttttcgggt attttttttt tttttttgtt tttcgtcgtt tgggttttgt 4740
tttgcgtgtt tttaggttta gcgcgttttc gggcgagttt ttaggagcgc ggtttaatgg 4800
atcgtttcgg gttcgttttt tcgcgcgttg attggtcgtc gtttcgttgg tttcgtttta 4860
ttagtaagtt ttttggggag tcgcggtagg gttcggagtc ggcgagtgtt ttcgggaatt 4920
ttgtttgcgc ggcggtagcg atcggaggtt aggtttagta cgtcggagtt ggtttgttgg 4980
ggaggggcgg gaggcgcgcg cgggagggtt cgttcggtta gggtttcggg cgttcgtaga 5040
ggtcggtcgc gtttttagtt cgttcggagt ttatgttcgg cggttggtta gtgttgcggt 5100
agaagggggg gttcggtttt gtatggtttc ggttgttgat atgatttttt tgttattcgg 5160
tgttaaagtg gaggatttcg ttttcggtaa gtcggcgggg ggaggcgcgg gttaggtttt 5220
tagcgtcgtc gcggttacgg tagtcgttat gggcgcggac gaggaggggg ttaagtttaa 5280
agtgtttttt tcgtttttgt tttttagcgt ggaggcgttt atggtcgatt ataggaagtc 5340
gggggttaag gagagcgttt tggcgttttt cgagggcgtg taggcggcgg gtggttcggc 5400
gtagttattg ggcgtttcgt cggggtcgtt gggagtttcg gacgcgtttt tttcgtcgcg 5460
gtcgttcggt tatttttcgg tggggggatt ttttaagttg ttagaagatg cgttcgttaa 5520
agtcgagagt ttcgagaagt tcgagaggat ttcgtggatg tagagttttc gttttttttc 5580
gtcgtcggtt agtgagtagt tagaatttag gcgtagaggg agggggtcgg gtggggtgtg 5640
ggattcgagg gtttttggtg gttttcggcg tttgcgtatt tgtagtcggt gttagggagt 5700
cgtgggttgt aaggtcgggt tttgcgtttt tttttatttt tatttaggaa gaaggtttta 5760
gattttttcg ttttggttta gagacgttgc gggtgggagt taacggatag gtcgtcgatg 5820
tttgggtatt ttgttttttt gtttttatta aacgatttta ggggtttatg attttttatt 5880
tgattttaaa ttttgtttta tcggttttat tttagcggat gaatgtgtgt ggtgcggtat 5940
tttttttgta ttcggagttt tattttttcg tagtaggagt tggtgttttt tagttttttt 6000
ttttttttaa gtattttttt gtttagaggt ttcgaagttt ttatagaatt ttattttttt 6060
atgttttttg agtttgaggt agatagttgg tgttttgggc ggatggatga tttaggggtg 6120
gggatattta ggttttagtg gagggggcgg ggtattaagt taattagggt aaggcgtttt 6180

cgttaatttt atgggaagtt tttaaacgtt taggattgag ttattaaagt ggattttagg 6240
tgtttaaggc ggttcgtttt aaggttttac ggttttttgg ttgttttatt tagagaatac 6300
gttcggagat attttaggag tacgggaaat ttttaagttt ttttcgtttt tttcgattat 6360
tttgttcggt ataatagtag ttagatttta atggcgtgat gttgaggaat gatttttatt 6420
tggggattaa acgtttttga aaggttagtt tttttttaag tttaatggtc ggagaaggtg 6480
gtttcgtttt gggttgtcgt cgttgaaggg agtgacgttt ttttcggcgt tcgtttttcg 6540
ggcggttcgg cggaaagtta gttgggggtt aagcgttttt cggattttcg gtggttttta 6600
gtagggaaga agcggggtgt taatacgaga tttcgtttga tttatatttt ggtggtttga 6660
aagtttaaag gatcgttgtg ttttttttgt tttgttttgt tttttttgtt tgtttgtggt 6720
tgttttttag agaggtgtga aaaaatgtat atttaggtaa aattcgcgtg gtgaaatatc 6780
gtcgatttga atttattttt tgtcgggaaa gttgttgtat aggtaaagtg ttttttttaa 6840
cgtttagggt tttgggtttt aagatttcga agttaaagcg atttcggttg tgttgggata 6900
atttgtttta tattttattc gggggtagtt tttagtagat tttattttcg atttgtatta 6960
gttttgcgtt ttgatgtttt tttattgttt atttttgagg tttattttga agttaaaaga 7020
aaaagatagt tgggtatgtt gatgtttgtt gattatgtta taggttgagg ggagaggcga 7080
ttttaatatt tttttcgtaa taatattaat atatatatat atatataaac gtttgagtgg 7140
ggttagaggg ttttggcgtt aggggtgaac gcgatttaat agaggattga gataatttaa 7200
agaaaaagtt tattagaata aagtagtttt tcgtttttcg ttttagatga tattttttgt 7260
ttttaagagg gttggttata gtgtattttt tatgatggtt tgcgttgttt tatttttggt 7320
ttgcgggaat tatttttaga atttcgtagg agcgaagtgt ttcggggaaa gtgtagaatt 7380
tgatttggat tttatgttat aaaattgttt agttttatat tgaagtattt cgtgggtatt 7440
gataaatgtt tggttaacgc gtaaaattaa atgtgttttt gggttgggcg tagggttttt 7500
ttttgtatgt tcgttaattg tattaatatt tatttttttt ttggatggtt ttgggaggag 7560
gttcgttatg aaggtttttt taagtcgtcg ggtagtataa aggtgatttt atattttttt 7620
agttgtttag gtttaagatg tggatatcga gtttttaacg tgggtatttt ttttttggaa 7680
ttttagtttt tttatttgta aaaagtagat aggaattttt tttttgcggg gttgtaatgg 7740
gaattggaga aaatatattt taagtgtttt gcgcgatgta cggtatcgag gtatttggcg 7800
gtatttaata tttggtattg tttggttatt attattattt tttgggttga ttatgtttcg 7860
atgttttttt tttaattttt tgtttttttt tttttcggtt tttagg 7906

<210> 196
<211> 7906
<212> DNA
<213> Artificial Sequence

<220>
<223> chemically treated genomic DNA (Homo sapiens)

<400> 196

tttgagggtc gaaagaaaaa aaagtaaggg gttaagagag aagtatcgga gtatgattag 60
tttaagaagt agtaataata gttaaataat attagatatt gagtgtcgtt aagtgtttcg 120
gtgtcgtgta tcgcgtaagg tatttgaaat atattttttt taatttttat tgtaatttcg 180
taggggagaa gtttttgttt attttttgta aatgaagaaa ttaagatttt aggagaaaaa 240
tatttacgtt gaaggttcga tgtttatatt ttaggtttaa atagttggga agatgtgaaa 300
ttatttttgt gttgttcggc ggtttaggaa ggtttttatg gcgggttttt ttttagggtt 360
atttagagga aaggtggatg ttaatatagt tgacgaatat gtagaaagag gttttgcgtt 420
tagtttaagg gtatatttag ttttacgcgt tggttaaata tttattagtg tttacggagt 480
gttttagtgt ggggttaggt agttttgtgg tatagaattt aaattaaatt ttatattttt 540
ttcggaatat ttcgttttta cgggatttta ggagtagttt tcgtagatta gagatggagt 600
agcgtagatt attatggagg gtgtattgtg gttagttttt ttagaaatag aaagtgttat 660
ttggagcgga gaacgagggg ttgttttatt ttaatgggtt ttttttttag attgttttag 720
ttttttgttg gatcgcgttt atttttggcg ttagggtttt ttggttttat ttaaacgttt 780
gtgtgtgtgt gtgtgtgttg atgttgttgc ggggggagtg ttgagatcgt tttttttttt 840
aatttgtggt atagttagta gatattaata tgtttagttg tttttttttt ttggttttaa 900
aataaatttt aagggtggat agtgaagaag tattagagcg taggattagt gtaggtcggg 960
gatggggttt gttggggatt gttttcggat aaaatgtgga ataaattatt ttaatatagt 1020
cgggatcgtt ttgatttcgg ggttttgggg tttaaggttt taagcgttgg aaaggatatt 1080
ttgtttatgt agtagttttt tcggtagaaa gtgaatttaa atcgacgatg ttttattacg 1140
cgggttttgt ttaagtatgt atttttttat atttttttaa aaaataatta taaataaata 1200
gaaaaaataa aataaaataa agaaaatata acgatttttt ggatttttag attattagga 1260
tgtgagttaa acgaaatttc gtgttaatat ttcgtttttt ttttgttgga ggttatcggg 1320
agttcgggaa gcgtttggtt tttaattagt ttttcgtcgg gtcgttcgag gggcgggcgt 1380
cgagagaaac gttatttttt ttagcggcga taatttagag cggggttatt tttttcggtt 1440
attaggttta gggagggatt ggttttttaa agacgtttaa tttttagata aaaattattt 1500
tttagtatta cgttattgaa atttggttgt tattatgtcg agtaaaataa tcggaggaaa 1560

cgaggaaaat ttgggaattt ttcgtgtttt tgaaatattt tcgagcgtgt tttttgagta 1620
gagtagttag ggggtcgtga ggttttggag cgaatcgttt tgggtatttg gagtttattt 1680
taatggttta gttttagacg tttgggagtt ttttatagga ttagcggggg cgttttgttt 1740
taattgattt ggtgtttcgt tttttttatt ggaatttgaa tgtttttatt tttgaattat 1800
ttattcgttt aaagtattaa ttatttgttt taaatttaaa gggtatgggg aggtagaatt 1860
ttgtaggagt ttcggaattt ttaggtaaag aggtatttga aagggaagag gggttggggg 1920
atattagttt ttattgcgag aaagtgaaat ttcgggtgta gggaagatat cgtattatat 1980
atatttattc gttggggtga agtcgatgaa atagagtttg ggattagatg agggattatg 2040
gatttttgag gtcgtttggt gggggtagga ggatagggtg tttagatatc ggcgatttat 2100
tcgttaattt ttattcgtag cgtttttggg ttaaggcgag gaggtttgga attttttttt 2160
tgggtgggag tggagggagg cgtaagattc ggttttgtag tttacggttt tttagtatcg 2220
gttgtaggta cgtaggcgtc ggaggttatt aggagttttc gggttttata ttttattcgg 2280
tttttttttt ttgcgtttgg gttttggtta tttattggtc ggcggcgggg agaagcgggg 2340
gttttgtatt tacggggttt tttcgggttt ttcggggttt tcggttttga cgagcgtatt 2400
ttttggtagt ttgaggagtt tttttatcga gaaatggtcg agcggtcgcg gcgaagaggg 2460
cgcgttcggg gtttttagcg atttcggcgg gacgtttagt ggttgcgtcg agttattcgt 2520
cgtttgtacg ttttcggagg gcgttagggc gttttttttg gttttcggtt ttttgtggtc 2580
ggttatgagc gtttttacgt tgaagggtag gagcgaaggg gatattttgg gtttggtttt 2640
tttttcgttc gcgtttatgg cggttgtcgt ggtcgcggcg gcgttggggg tttggttcgc 2700
gttttttttc gtcggtttgt cgaaggcgga gttttttatt ttgatatcga gtggtaaaga 2760
agttatgtta gtagtcgggg ttatgtagag tcgggttttt ttttttgtcg tagtattggt 2820
tagtcgtcgg gtatgggttt cgggcgggtt gggagcgcgg tcggtttttg cgagcgttcg 2880
gggttttggt cgggcggatt ttttcgcgcg cgtttttcgt ttttttttag taggttagtt 2940
tcggcgtgtt gggtttggtt ttcggtcgtt gtcgtcgcgt aggtagagtt ttcgggagta 3000
ttcgtcggtt tcgggtttta tcgcggtttt ttagagaatt tgttaataag gcgaggttag 3060
cggggcggcg gttaattagc gcgcgagggg gcgggttcgg agcgatttat tgggtcgcgt 3120
ttttggggat tcgttcggag gcgcgttggg tttgggggta cgtagggtag ggtttaggcg 3180
gcggagggta ggggaggaga gggggtgttc gggaaatttt tttttgcgtt gaagtttaag 3240
aggttttttt tgcgggagtt ttttgggttt ttattttttt gtgttttcgg ggggattttt 3300
ttttgttttc gttttttttt ttttgttttt tttttttttt ttttttagtt ttttttttcg 3360
tttttttttt ttttttcgtt tttgtttttt ttttttattg ttttttttat tttttttttt 3420
agtttgtggt tgggtttttt tgtgtttttt tttattgttt gatttttttt ttttttttat 3480
tataataatt tgagttgagg ttatttttta tatatttttt ttatatatag tttttaaaat 3540
acgtatggtt atttatttta ggagaaagat atattattaa ttttttttta agaaacgaaa 3600
taaaattaaa tttttatttt ttttttgttt tgaaattttg taaattttat ttcgaggtaa 3660
aagtgggttt ttgtttttaa tttagagttg gagtaaattt tgcgggaaga agttattttt 3720
tgtagtgtta aggatgtgag aatacgtaag attatttaga atttgtagtc ggtttttata 3780
tacgatgtgt attttttttt ttttattttc gttttgaatt tttgttttaa gggttataat 3840
atttatagcg ttcgggcggg gaaaggagtt gttagatttt tttgatatag aatttattga 3900
aataaaatta atagaatcgt taatgaaata tcgatttggt tttaaggtga ttttaaataa 3960
cgcgttttcg gaaataatga tatgttttta tttttgggtc gttgattttg gaggggagag 4020
gtatatttgg gggagggggt tgggaaaaga ggaaagtttt tggaggtgga ttttagtgtg 4080
ggatttgaga attttttttt tgttaatttc ggagaaaacg tgatttaatg tttgttgtta 4140
ataataataa taataataaa gatagaaaaa atgagttggg gttaggggag atgtaagatt 4200
tgtttgaagc ggggtcgggg tttggttatt ttttgcgttt gtcgttttat ggtttttttt 4260
ttcgcggttt aggttagcgg tttttaggga gtgagcggag gtgattggcg gtttattcgg 4320
ttttgttatt gatgattgcg gggttttaat ttttaggaaa tataaggggt tgtcgtggtt 4380
atttaggggt aattattcga gcgcggaggg atagcgaatt ttttcgattt ttaattgggc 4440
gtaataaaag ttgtagatta ggggcgttta gattagggaa aatgaattag agcgggaatt 4500
ttattattcg gaagacgttc ggggtttggc ggtaggggtc gggttgcgga cgtcggagcg 4560
attggtagtt ttttatttgt ttataggtcg tcgagatatg ttgaggtttg gggaagttcg 4620
ggaatatcgg tatttgaatt ttacgtatat ttttaggttt ttaaaggatt taagattgta 4680
atttaggtat tgggtagtgg ttggggtttt tagttcgagt tgggtcggtt ggacgcgcgg 4740
tgtatcgggg ttttcggagg tttgttcgag agggtataag gatagaagat tttaagattt 4800
aattttgaag tttagttcgt tgaggttgag taaggttgta gggtaaatat tttgttggta 4860
gtttagttag gatagcgaat aatagtattt tttttttttt tttttttttt tttttttttt 4920
tttttttttt tttttttttt tttttttttt attttttttt tttttttttt ttttttattt 4980
tttttttttt tttttttttt tttttttttt tttttttttt tgagacggag ttttgttttt 5040
gtcgtttagg ttggagtgta atggcgcgat tttagtttat tttaattttc gttttttagg 5100
tttaagcgat tttaggacgg cgaattttgt ttttataaaa aaaaaaaaaa attagttgga 5160
tatggtggtg cgtgtttgta gttttagtta tttgagaggt tgaagtagga ggattgtttg 5220
agtttaggag gttgaggtta tagtgataga tagaagtttt tttgtttgtt tgtttgtttg 5280
tttttggtag gttggaatgt aatggtacga ttttagttta ttgtagtttt tatttttcgg 5340
gtttaagcga tttttatgtt ttagtttttt aagtagttgg gattataggt atgttttaat 5400
atgtttggtt aattttgtat ttttagtaaa gataggtttt tattatgttg gttaggttag 5460
tttcgaattt ttaattttaa gtgatttttt tgttttggtt ttttaaagtg ttgggattat 5520

aggtatgagt tattgtgttc ggtttatata gaagttttgt gtagtatttt tgggaagttt 5580
tttgaaagag agatttataa tattggagag atgaaaaatg taggttttta ggtttatttt 5640
taaatttgtt gagattatat tatgtatagt attttaatgg cgaaggtagg gtaagattaa 5700
gtttaggaga agggttagga taatgtgatt ttatttaatg tagtaaatat tattgatagt 5760
tttgattata tgttaggtgt ttggattttt tttagttatt taggaggtta agttttgagg 5820
attatttgag tttagaagtt tgaggttgta ttgaattatg attatattat tgtaatttag 5880
tttgggtgat agagtaagat tttgttttta aaataaaata aaataaaaat attattataa 5940
tgaaagaatt taaataatat agtgttgtag aatatagaat atatggatat agatgatata 6000
atgttgatgg ggatgatagt ttatgattat taggtttgta ttgggtattt ttttgtgttt 6060
tttatatata gattttagtc gattattttt ttataattgt tttttttatt tcgttttttt 6120
tattttttag atgtagagat ttagagagtt taagtaattt ttttaaatta tttagttgga 6180
atttagtaat tgagttagga tttattttag gtttgtttga ttttaaagtt tgtgtttttt 6240
agtatatatt gttgttgttt ggggtaagag tttaatatta tgtttggaaa aaggagtttt 6300
tttattttat tgtggagggt tgggatgttt ttcgtgttta cgtttttttt ttttttagta 6360
atgggtacgt tattgcgttt tttagttttt tttgtagtaa ggtgattaag tgaatatttt 6420
ttggttaatg agatgtatat agaagttttg tgtaatattt ttgggaagtt ttttgaaaga 6480
gagcgaatat attttttttt tatttttttt aaaaattttt tagtaggaat gtggatatga 6540
tgtttggagt tttagtaatt atattagatt ataaggatga atgtatattt taggaatgga 6600
agagaggaaa gttagaaaaa gattcggttt ttgatggttg aagagttatt atatttgttt 6660
ttagattttt tatttttgat tattttttat gtaagggtga gtgaattcgt attgtgttaa 6720
cgttattgtg atattgggtt ttgttttttg ttagttgaat gtgattttaa gtggtataat 6780
gggttaattg agaggaagag aatgaatttg ggttgggatt tgaaagatgg gtaggattga 6840
gataggaatg ggggaagaag ttttttatag gtgttgggaa tagtaagagt gagaagtgtt 6900
tttgaggtgg tatgtggagt ataataagga agatgtagga gttaagtagg gttagagtag 6960
tggtttgtaa agagttttgg ttgttaggta gtgaagatat aattttaatt gttaagtatt 7020
ggggagttat agaaggtgtt tgagcggata agtgatgtgt ttggtattgg attttgtttt 7080
gatggagatt tgtaggcgga attgaagagg aggaagtttg ggttagaaat ggggttttgg 7140
ggggtgggaa ttgtttggaa aggaggtttg gagattagtt ttggtaatgg tttatggaat 7200
tattattgta ttaaatagtg gtgtgttaat attgtgggtg atttggaagt tattgaaata 7260
atgatgtata agatagtatg ataatatgtt aagttgttta cggtatattg ttatatgata 7320
aaaataggtt tttaaatagt ttgtataata tgatgagttt ttgaaaattt attttatgta 7380
ttttatttaa aagttaagaa ggttattttt taattgttaa taatagttat tgttaggggg 7440
aagtttttag ggatgatttt tttttttttt tttgtttatt attttttaag ttgttagtag 7500
tagtagtagt agtaataata ataataataa taataataat aataatagtt aagtattttt 7560
gagggtttat aaagttcggt gttagtttta ttttttgtgt taatttatgt atttaaattt 7620
ttatagtttt ttatgaggga gggagtttaa tttttttttt tttatatttg aggaaattga 7680
gatttagaaa gtaggagttt atgtttaggg gagagtatta gtggaaagag aggtgataat 7740
gaggattcga gagaggggac gttttagttg gatggaggag ttagggagtg aggagggaaa 7800
ggttttttta gaaattttag gggatgtacg tatcggtttt tatagtttat aaagtatatt 7860
ttgtattttc gtttttaaaa tgcgtttgtt aggaattatg attgtt 7906

<210> 197
<211> 9725
<212> DNA
<213> Artificial Sequence

<220>
<223> chemically treated genomic DNA (Homo sapiens)

<400> 197

ttttttgtag ataaattagt ttcggttttg ttcgtggtaa ttttttttta attaggtagg 60
ggttgagaag agattagaga gttgtattta gggattggga gataggagat attttttgtt 120
gtttgtttaa atttaaagtt tttttttttt ttaattttta ttttttatta gatagatttg 180
ttaatttgtt ttttttttaa aaggtaggta gttattttta ttttatagaa gaaaaaaaaa 240
tgaggtttta gaaagttgta tgttttaaat tatttaatga gttttatgtt tcgaattatt 300
taatattaga ttattcgatt ttaaatgtgt acgatgttta ggaagattta ttggaaaggg 360
aggtaaaaag taatatcgat aatgatttaa gaaatgagaa taatatttta tatttattaa 420
atgttaggta tttttttatg ttatagttgt tgaatttgta gtgtagaaaa gtgaagtgat 480
ttatttagtt ttataggaaa tatatggttt aattttattt gaagttagtt tagttttaga 540
ttttgaagtt agtttagttt taggagattc gaagtagagt tgttttttgg gttagtgttt 600
gtttttagta tatttagcgt tattcggtat tttgtaaggt ttgttattta gtttttatta 660
gtaattttgt ggatgggtat tagtatgttt attttttgaa aggggaaatt aagatttagt 720
aaggtgacgg cggaaaacgg tgagaagtag ggtttggatt tgaatataga attatagaag 780
gtcgatttat agtttttttt tttatatttg ttgtttttag aattagatgt ttttatgaat 840
tcgttatttt ttttttaggt tgtgggattt gtaaggagat gtagtttttt ttatttttat 900

ttttttcgtt tttatttttg tgatggtttt tttttaggga tatcgtgttg aattaggttt 960
tattatagtt gaagttaggt taattttttt tttttttgcg tttggacgat tttatttgtg 1020
gaatggggag aaagtttagg ttttaggggg tttttagtaa tttgtatcgt gtttttggta 1080
aaagtttttt ttttttagtt tcggtttaaa ttcgggttcg gttttgattt cggattttag 1140
ggggcgggaa taggagaata ggaaagaata agtttgggtt tgtagagttt cgtaagtaag 1200
acgtttgtat ttttaaaatt tttagttgtg attttgaata tttatttatt cgattttttg 1260
ggttgtgtaa ttcggaaagt gattttttta gatattagag gatcgaattt tttttatttt 1320
tattttgtaa taagttagta agaaaaatga gtttagagac gagtagtttt ttgttcgaag 1380
ttatatagtt aatgttttgg ggggtgggtg agcggtgttg tggaattttt ggcggatgtt 1440
ggcgttcgcg ggtttgtttg tttttgatat tcgggttcgg tagaataggt cgttaacgag 1500
tttttattag gaggaattgt tatttttttg ggagcgagtt tgttttatta gggcgaattg 1560
ttattttttt tggagcgagg ttgtttcggt ttcgcgtagg ttgagtgttg gggtcgagag 1620
taattaacgc ggtttcggcg cgggtagtcg tttttgtttc gggtagcggg ggcggggtag 1680
ggcgggggcg gggcgttcgg cgcggttgga gtcggttatt cggcgtagtt ttttttttcg 1740
gagttcgttt tttatttttt cgcgtttggc gtttgttcga agtttaggtt cgtttatagt 1800
ttttttcgtt gttttttttt cgtttttttt tagtcgttgg ggttataaat tagttttcgg 1860
ttttcggggt tataaacgtt gcgttttgcg cgtagtgttt tatagtttgt aaggagtgtt 1920
ttttttttgg gattcgtagc gcgcgtacgc ggttgtttta agaaggggtt tggcgggtgt 1980
aacgcgtttc ggggagcgcg ggaaggtgta gggttgagga tttacgttta gaggttttag 2040
tcgcgcggga gggatttttg tgttttttat ttttatttgc gcgattttgg gtagagtatt 2100
agcgtttttt agttttaggt atttatttgt gaaatgggaa aaataatttg ttagtagcgg 2160
tgtgttggaa gaaattaata agattttgga taggtttagg tattaaagta ttttttagag 2220
ttgatgtttt ttggtggttg agtttttatt ggaatttcgt ttttttgtta gtaaatattg 2280
tagttacgta tatatttttg ggaattaata tttattgagt gttttttacg tgataaagtt 2340
agtttttcgt tagattattg tttttaaatt tggatatttt ttagatcgag ttaaattttt 2400
agtttcggaa attttataaa gaatttatcg taagttttgg gatgtatttt atttttcgat 2460
ttttggtagg gagaaagtat tcgatatatt gtgattgttt ttgatatata ataatgtttt 2520
aatttggatt ttttatgttt tttagttatt ttaggtagtt tattgaattt aattattatt 2580
agagttattt taggtaagga ttattatttt ttattttgga gataaaaaaa aaaacgttgg 2640
ttggaggaag taatttagag tgtttaaggt tatttcgtta ggaaaaggtg aataaaacgt 2700
tttgaaaacg tagttaaagt tggataaaat gtagatttgg ggttttttat aaagggttag 2760
cgttgtgttg cggttttagt ttttttttag tagaagtgag tttttttttc gatttgtttt 2820
tattttagta gagagttggg gagggaattg gtaaagattt taggagtagg ttggtttttt 2880
ttttagtatt ttttttattt atggttagag tttggtattt tgattttgaa tatcggtaat 2940
ttttttattt ttgttttttt atttttggtt ttgtaattta tttgtttttg tttggatttt 3000
agtattttag agtagtaaag gggatttggt tttttttttt ttagagaagt ttattaaaat 3060
aaaggagttt tagtggtatt gatttttgtt tcgaattttt taggaatgag tttgtgtgtt 3120
ttatagttga tagatattaa taaggagttt ttaggaataa ttataaaata ttggttgcgt 3180
ttagtttaat tgaaagttga atttttttgt atattttggt agttgagggt taaacgaatt 3240
ttagatttgt ttttaaaata tatagtttgt attttgtttt gtatatttag aaggtataga 3300
agttaatttg gatttatttg tattgtattt gagatgtata ttaaattaat tattattagg 3360
tatgatttaa ggggaaaacg tatatatttt taggtagttt ttatagtttt ttttttttta 3420
atttttttga gtttttagtt ggttaagaag aaatgtgtat attttttata gtgagagaga 3480
aatttagaag ttttagaaga ttgtgtttag gatttattta tttttttatt tttttaggat 3540
tatttttaat taaattgaat taattggttt gtgtagattg tttttatttt ttaagattta 3600
ggtaatattt ttttttagtt tttttcggtt agtatagttt tattttaggg aaaggtttgg 3660
ggtgaagtaa atagtgtttt agagaggtat attgggaagg gtatataagg gtttgtaggt 3720
ttaggggtta agtttttaat atttttgaat tgggaggtag tttttaagtt ttggttattt 3780
aaataattaa aaataatgtt tttttttttt aatgttttag ggaatgttta gtaagtattt 3840
gttgtaggcg aggttttgaa ttgggtgatt ttttatttat ttcgttagtc gtaggataat 3900
cgtttagata taggaaaaga tagtcgtagt ttgaaaaaag ttttttgaag ttaatttttt 3960
aggttaggta ttttgaggat tagcggagag attcgaggtt tttaattttc gaagttcgtt 4020
tttttttttt ttaggttgag atagttatta ttgaggtttt ggaaaggaat tatcgagggt 4080
ataatttggg gtggggtgga gatatttatg tttttataaa aaattaggtg ttatgaataa 4140
aaatgtgttt tttaaaatga tatgtaggtt tggggtgtgt aaatttggaa gtcggtattt 4200
ttgagatttg taaacgagag ggaggtatta gatattggcg attttttttt ttttttttta 4260
tttttattat ttttcgaggt aaatattggt atttaaatac gtacgataaa tataggttta 4320
aataaattta tgaatgtata tttgtattgt tttgagaaaa aggtcggatt cggatcgcgt 4380
ttagaaagga ggtttaggag ataagtatag agattttaat tttttaaaag tgcgttggat 4440
ttgtttttta gtgagagaga tggggataat tgatttaaat tttttatttc gaattatttg 4500
ggagagaggt atttttgatt atttttcggg tttttttcgg tttttttttt ttaggggaaa 4560
gagttgtaat aataatagtg ggcgtttttg agcgttggtt agtggcgagt tattttttta 4620
agtgtatata ttttttttat agggatattg gtttgtgagt tttagtatcg attttttgga 4680
attgtaaagt aagttaattt tgacgtatag taggggtttc gtaaatgttt ttgtaatgtt 4740
gtttgcgaat tttgaattta ttttggcggt aaagggagag tgggatgggc gtagggtttt 4800
ttttaaagaa gggttaaaag agagaagaaa tgggattcgg cgaattttcg ttttaaataa 4860

tcgtttttaa ttaggtttcg ggttttttag tttcggcgtg attataattt tttagggatc 4920
gtttaataat aattttgttg attgtttttg taattaattt ttaatttatt ttaaatagga 4980
aaaaaaaaaa aaaaaaaaaa aggggggggg ggtagttttt tcgggaaaag ttaattaggg 5040
gcgagcgttt ttggcgttag cgttaggttc ggcgcggatt ggcggcgcgc gttttttatt 5100
ttttttcgga ggaaaggttt agtttttagc gcgttttttt cgttttcgta gtaaaaaagt 5160
ttgagtcgtc gttgtcgggt tgttagcgga gtcgcgcgtc gggagttacg tagggtagag 5220
aagttatggt tttttcgttt aaaggtaatg atttgttttc gttcgacgag gagggtttag 5280
tagtggtggt cggattaggt tcggggtttg ggggcgtcga gggggtcgcg gaggagcgtc 5340
gcgttaaggt ttttagtttg ttttttagcg tggaggcgtt tatgttcgat aagaagtcgt 5400
ttaaggaggc gttttcgttg tcggtcgaaa gcgtttcggt cggggttatt ttgcggttat 5460
tgttgttgtc ggggtacggc gttcgggaag cgtatagttt cgggtcgttg gtgaagtttt 5520
tcgagatcgt ttcggttaag tcggaaaatt tagaagatgg agcggcgtgg atgtaggaat 5580
tcggtcgata ttcgtcgtcg ttaagtgagt gcgcgtcggg gtaggagtag gaggtagcgc 5640
ggggtattgg agggagcggg gggcgggtgt tttagggttg agggtatcga gatttttttg 5700
cgtgcgttat attttcggga aagtaagttt agggtttttg gatttttggg tgttcggggc 5760
gttcggcgcg tttagtgcgt cgttcgtatt taagatattt tttgggtaat aatcgcgttg 5820
tgtgtgtttg agattttttc gatatataga ttgttttttt aagagcgaat tttagttggg 5880
tttacggggt cgttcggtgg ttcgttttta tagttttcgg ttttgcgttt tggttatttg 5940
gtttgttttt gttttattgt ttttcggagt ttttgtttgg ggagtgaagg agttggattt 6000
atgtttagtg agtatttgga tgtattagga acgagatagg tttaaaattt aattttgaga 6060
gttgtgtttt tattttaaag atgagttaat tgggtttagt tagatattga ggagaaatta 6120
ttttttttcg tgagtttcgc gggtgtcgaa gtcggatttt acgaagggat ggtggtatta 6180
gggatggtgg tattagagtt tggtgcgggg aggaaatgtt tttttgtggt tttcgtagtt 6240
agtatttttt cggtaaggtt ttgagtttcg tcgggagcgg gttggggtta gaagcgtacg 6300
ggtttttttt ggtttatagt aataattaat cgtattttag tagggtttga gagtttgtag 6360
agtgatgttt attagtttat tagatcgaga tttttattag aatttttaag tgtagatatt 6420
ggatttgtga ttattttatt ttttagacgt ggacgttgag gtttttaaag aagacggttt 6480
ttgtttgttt taggttatta ataattattg attggttagg ggtatttgtt gtttacgttt 6540
tgtttttttg tttgatatgt tttgattatt tttgcgtttt ttttttagag agattaggta 6600
gagattcgta tttgtatttt atttgagaat tgaggttgta gagaattttt cgttttagta 6660
tttattttg gttttgagt tttttataa tttggagacg ttattttta tagtttttt 6720
tatttttatt ttttggtttt tatggttttt ttatttttta attttttatt acgttatttt 6780
ttatttgtag gaaaggtgag ggtgggggtt gtggagggat tttttttaaa gttgagagag 6840
atttttttat tatttatatt atttagtttt taagattaga cgtattgtat tttgttaata 6900
aaataaggtt atatatttag gcgttagtgg ttgttttttt tggtagtttt tttttatttt 6960
gttagaaatt ttgaaagatt taaagggcga ggagattaag ggatttaaag aatttaaatt 7020
taagtttgga tagtgtttta tagtattttt tatcgtatta ttttattttt tttgtagggt 7080
agtagtcggt tttttaatag atgaggatat tttagtaaag tttagagagg aatcggtagt 7140
ttgggtttga ttaaaaaaaa aaaaaaaaaa aagttgggga ttatttaatg attatttatt 7200
aggtggtaga tagatagggt tttgggtatt ttttaaattt tagtattgtg gattatattg 7260
agggattagt gttatttatt tattttatcg atagtgaggt agaggtttag aaaattaaaa 7320
gatttaattt gtttaataaa tattgagtat atagtttacg tttatgttta gttaatgata 7380
taggttttaa atggagtagt gtttagaggt taggaatttt gttatttttt aggtttgtgg 7440
gtttggaaag gtaatgatta ttattttttt aaatattttt gttttgtgtt taagtttttt 7500
tgttttgggt aaggtaattt cgggtttagg ttgtgatttt tttatgaaaa gaattagaaa 7560
ttttgagaat cgagtttata atttgtatat tttgtatatg tatttcggaa tttaaagtaa 7620
aaaaaaataa aaaaataaaa aaaaaatatt atgaagaatt gttggtgttt tgggtttgga 7680
ggtggtagcg ggaattttgt ggttagagta gtgggttttg agaatgttag gcgacggagt 7740
ttggggcgag agggagttat tcgttcgggt ttgattttgt tatttggagg aagtaggata 7800
aacgttgttt tgttttatat aatcgtattt tttttttggt tggtttaaaa ttaggtgggg 7860
gtggagggat ggggagtgtt gtattgggag gaagttttat agttttatgg ttttgatata 7920
tatgtgcgat ttcggttatt tggattttat aatttaaatg taattcgaag gattagttgg 7980
aattattttg tttaggcgta tagtgtttta aaattttacg tagagttagg agtaagagta 8040
tatatacgtt aggaatttgg tatttgagtt gagatatttg agtttttttg agggttcggt 8100
ttagtttaga aagggcgaga attttgtagg ttttttgaga gtttttgata gtgttgttaa 8160
tttgtaaatt aagaagttgg tttttcggtt gtttagtaat ttagggatta gtaaatgtaa 8220
aagtatagat ttttatggta gttttttatt tttttttttt ttagattttt gttttttggt 8280
tggttataaa gttttttatt tttttttgat ttagtaaagt gatgggtaat aatgcgatat 8340
tggaataaat ttattttttg ttggaaatgg aagttgggtt gggatttgtg ttgtgttatt 8400
tttaagtatg aagtgtaaat ttttttttgg gttattaaag taattaaata agaaatgtaa 8460
tattttttta tgaatatatt agagttaata ttgtggttat atttggaaaa ttagtatagt 8520
tcgtaaaagt taaatgtaga aaattaattg ttaagtattt tattattata gttttatttt 8580
ttttcgttta tttatttttg aggaagataa gtttaaagtt agttgatgtt atggaattat 8640
aaggatgttt tagttcgtga tttgttgtaa ttgaatttat tttaaatgtg gagtttggat 8700
gttgttggta tatttttatt ttgtttgttt ttttttttga taaataaatt atttatatat 8760
ttgttcgaag ttatagaatg ggtagagggg atgattaaga tgttattacg tgtttttaat 8820

ttggaggtag tatgattttt tttgatattt atatttatgg gttagttaat tttttttttc 8880
gttttagtaa agtttatatt atagaagcgt ttagttaaga attttatttt atatttagat 8940
ttgtaaaatg atgtttagat tttataattt gtggattgga tgtaatgata gtttgtgatt 9000
gtttattgcg tgggattgtt tttaaattta aagatgttat taatttatta tagattttat 9060
ttgtacgatt attttttata aatatgttga cgtaatttat tttttttggt ttagagagga 9120
gattaaggga aatagttaat tcggttgtta aaattttttt tatttttttt tttatatgtg 9180
attgttttta tgtttaaggg aggttgggtt aattatgaat ttggtttttt atttttaaag 9240
agaagataaa ttatgtttag aagagatagc ggtttatttt ttatcgatta tttgattttt 9300
ttgttttata ttaaatgttt tatttttgtt atttttaatg ttgtttgttt tttatataga 9360
aggtttagtt atattattat atttttaaaa ttttttttaa aaaatttatt tttaaaaaac 9420
gaagtaattt atttatttat atatggtttt ttttttttta agaaatatat atagttttga 9480
taaaggtatt ttttttaaag atgaaataaa gtatgttttt ttatatatag atatgtgtgt 9540
gtgtgtgtgt gtgtgtgtgt gtatatgtat gtatatatag atttttttaa agttaagtgt 9600
ttttagttga gaggggaggt tcgaaaggaa aaaatttaga gagatgacgg gggagatggg 9660
ttttttttag tattatttta atgtaatttt tttttgttaa ttcgtttttt tttttgtttt 9720
ttagg 9725

<210> 198
<211> 9725
<212> DNA
<213> Artificial Sequence

<220>
<223> chemically treated genomic DNA (Homo sapiens)

<400> 198
   tttagagaat agagagagga gcggattagt aaaagaaagt tatattaaaa taatattaaa 60
   aaaaatttat ttttttcgtt atttttttag gttttttttt ttcgggtttt tttttttaat 120
   tgaaagtatt tagttttaaa aaaatttata tatatatata tatatatata tatatatata 180
   tatatatata tatatttatg tatggaaaga tatattttgt tttatttttg aaaaaggtat 240
   ttttgttaaa attatgtgtg ttttttgggg ggagggggat tatatataaa tgggtaagtt 300
   gtttcgtttt ttaaaaatga gttttttaag aaaagttttg gaaatgtaat ggtgtaattg 360
   aattttttat atggaaaata aatagtatta gagataatag aaatagaata tttaatgtaa 420
   gataagggaa ttaagtggtc ggtgagaaat gaatcgttat tttttttgag tataatttat 480
   ttttttttta aaaatgggag attaggttta tgattagttt agtttttttt ggatatggag 540
   ataattatat atgggaagga aaatgaaaaa ggttttagta atcgggttag ttgttttttt 600
   tggttttttt tttgaattag aaaaagtgga ttgcgttagt atatttatga aggataatcg 660
   tgtagataag gtttgtggta aattaatagt atttttgaat ttgggggtag ttttacgtaa 720
   taaatagtta taagttgtta ttgtatttag tttatagatt ataaaatttg aatattattt 780
   tatagatttg gatgtgggat aaggtttttg gttgggcgtt tttgtaatat aaattttatt 840
   aagacgaagg gaaaaattag ttagtttata aatgtaaatg ttaggggaaa ttatgttgtt 900
   tttaaattga gagtacgtga tgatatttta gttatttttt ttatttattt tatggtttcg 960
   ggtaaatgtg taaatgattt gtttgttagg gggaaaaata agtaaaataa aggtatgtta 1020
   atagtattta gattttatat ttaaaatgga tttaattata gtaaattacg gattgggata 1080
   tttttgtgat tttatagtat taattaattt tgggtttgtt ttttttaagg gtgggtgggc 1140
   ggaggaggat agagttgtgg tgatagaata tttaataatt aattttttgt atttggtttt 1200
   tacgagttat gttaattttt taaatgtggt tatagtatta attttaatgt atttatggga 1260
   gaatgttgta ttttttgttt agttattttg gtggtttaag aaagagttta tattttatat 1320
   ttgagggtga tatagtataa gttttaattt agtttttatt tttaataaga gatggatttg 1380
   ttttagtgtc gtattattat ttattatttt attgaattaa aaagagataa gaggttttgt 1440
   aattaattaa agaatagaaa tttgggaaag ggaaggataa gaaattatta taaaagttta 1500
   tatttttgta tttgttaatt tttaagttat tagatagtcg gggagttagt tttttaattt 1560
   ataaattaat aatattgtta gagattttta agagatttgt aaaattttcg ttttttttaa 1620
   attaggtcgg gtttttagga ggatttaggt gttttagttt agatattaag tttttagcgt 1680
   gtatatattt ttgtttttgg ttttacgtgg ggttttgaag tattgtgcgt ttgggtaggg 1740
   taattttagt tggtttttcg ggttatattt gaattatgaa atttaggtga tcgggatcgt 1800
   atatgtgtat taaaattatg aagttatgag gttttttttt aatgtaatat tttttatttt 1860
   tttattttta tttaatttta aattagttag aggaaggatg cgattatata aggtaaaata 1920
   gcgtttattt tatttttttt aaataataaa attaggttcg ggcgggtagt tttttttcgt 1980
   tttaggtttc gtcgtttggt atttttagga tttattattt tgattatagg gttttcgttg 2040
   ttatttttag gtttaaagta ttaataattt tttatggtgt tttttttttg tttttttgtt 2100
   tttttttatt ttaagtttcg ggatatatgt gtagaatgtg taggttataa attcggtttt 2160
   tagaattttt ggtttttttt atgggaaggt tatagtttgg gttcgaggtt gttttattta 2220
   gagtagggaa gtttgagtat agggtagaga tgtttagagg ggtagtagtt attgtttttt 2280
   tagatttata ggtttaaagg atggtaggat ttttaatttt tggatattat tttatttaag 2340
   gtttgtatta ttggttgggt ataggcgtag gttatgtatt taatatttgt taaataaatt 2400
   aagtttttta attttttgag tttttatttt attgtcgata aagtgggtaa gtaatattaa 2460
   ttttttaatg tggtttatag tattaaaatt ttaagagtat ttagagtttt gtttgtttgt 2520
   tatttggtag gtgattatta aatggttttt agtttttttt tttttttttt tttggttagg 2580
   tttaaattgt cgattttttt ttgaattttg ttgaaatgtt tttatttgtt aagaggtcgg 2640
   ttgttgtttt atagaggaga taagataata cggtgaaaag tgttataaag tattatttaa 2700
   atttgagttt aaattttttg agttttttaa ttttttcgtt ttttgaattt tttagagttt 2760
   ttaatagggt ggagagagat tgttagaggg gatagttatt gacgtttggg tatatggttt 2820
   tgttttgtta ataaaatata gtgcgtttag ttttgagggt tgaatgatgt aaatagtgaa 2880
   aggatttttt ttagttttgg agaaagtttt tttatagttt ttatttttat tttttttata 2940
   ggtaggggat ggcgtggtgg gaaattaggg ggtagagaaa ttataaaagt taagagataa 3000
   aagtaaaaga ggttgtaaag gatgacgttt ttaaattgta aaaggattta gggattaaaa 3060
   atggatgttg aagcggagaa ttttttgtag ttttaatttt taagtggggt gtaggtgcgg 3120
   atttttattt agtttttttg gggagaggac gtaaaagtgg ttagggtata ttaaatagag 3180
   gagtagggcg tgggtaatag gtgtttttgg ttaattagtg gttgttggta atttgggata 3240
   agtaaaggtc gtttttttta aaagttttag cgtttacgtt tgaaaaatag ggtagttata 3300
   agtttagtgt ttatatttgg gggttttagt gaggatttcg atttggtgag ttagtgaata 3360
   ttattttgta aatttttaag ttttgttgga atgcggttaa ttattattgt gagttaagaa 3420
   gagttcgtgc gtttttggtt ttagttcgtt ttcggcgaag tttagaattt tgtcggggag 3480
   gtgttggttg cggagattat agagagatat ttttttttcg tattagattt tgatgttatt 3540
   atttttggtg ttattatttt ttcgtgggat tcggtttcga tattcgcggg atttacgaaa 3600
   gaaaatggtt tttttttaat gtttggttaa gtttagttag tttattttta aaatgggggt 3660
   ataattttta gggttgagtt ttgaatttgt ttcgtttttg gtatatttaa gtgtttattg 3720
   aatatggatt taattttttt attttttagg tagagatttc ggggaatagt gaaataaaaa 3780
   taagttaagt ggttagagcg tagggtcgag ggttgtagaa acggattatc gagcgatttc 3840
   gtaaatttag ttggggttcg tttttgaaaa aataatttgt atgtcgaggg agttttagat 3900
   atatatagcg cggttattat ttagagggtg ttttggatgc ggacggcgta ttgggcgcgt 3960
   cgaacgtttc gggtatttag gagtttagag gttttgggtt tgttttttcg ggagtgtagc 4020
   gtacgtagaa gggtttcggt atttttagtt ttggaatatt cgtttttcgt tttttttagt 4080
   atttcgcgtt attttttatt tttgtttcgg cgcgtattta tttggcggcg gcgaatatcg 4140
   gtcgggtttt tgtatttacg tcgttttatt ttttgaattt ttcgatttga tcgaggcggt 4200
   ttcgaagggt tttattagcg gttcggggtt gtgcgttttt cgagcgtcgt gtttcgatag 4260
   tagtagtggt cgtagggtgg tttcggtcga ggcgttttcg gtcggtagcg gggacgtttt 4320
   tttgggcggt tttttgtcgg atatgagcgt ttttacgttg aagggtaggt tggagatttt 4380
   gacgcggcgt tttttcgcgg ttttttcggc gtttttaggt ttcgggtttg gttcggttat 4440
   tattgttggg tttttttcgt cgggcgaaaa taagttattg tttttggacg gagaagttat 4500
   gatttttttg ttttacgtag ttttcgacgc gcgatttcgt tggtaattcg gtagcggcga 4560
   tttaaatttt tttgttgcgg aaacgggagg ggcgcgttgg gagttgagtt tttttttcga 4620
   ggggaagtgg gagacgcgcg tcgttaattc gcgtcgggtt tggcgttgac gttaaaaacg 4680
   ttcgtttttg attggttttt ttcgaagggg ttgttttttt tttttttttt tttttttttt 4740
   tttttttttg tttgaaataa attaggagtt aattatagga gtagttagta aagttgttat 4800
   taggcgattt ttgaagggtt ataattacgt cgaaattgaa aagttcgaga tttaattaag 4860
   agcgattatt taaagcggaa gttcgtcgag ttttattttt tttttttttt gatttttttt 4920
   taaaagggat tttgcgttta ttttattttt tttttatcgt tagaataagt ttaagattcg 4980
   taaatagtat tgtagaaata tttacgaagt ttttattatg cgttaaaatt ggtttatttt 5040
   atagttttag aaagtcggtg ttgaaattta taggttagta tttttgtgaa agaaatgtat 5100
   gtatttagaa gagtaattcg ttattagtta gcgtttaaaa gcgtttattg ttattattat 5160
   aatttttttt tttggaaaag aggggtcgga aggagttcga gaagtggtta ggagtgtttt 5220
   ttttttaaat ggttcggagt gaagaattta aattagttgt ttttattttt tttattagaa 5280
   agtaaattta gcgtattttt agaaagttga aatttttgta tttgtttttt gaattttttt 5340
   tttgagcgcg attcggattc gatttttttt ttaagatagt gtaagtgtgt atttatgagt 5400
   ttgtttgagt ttgtgtttgt cgtgcgtgtt tgaatgttag tgtttgtttc ggagggtaat 5460
   gggggtgggg gggaggagga aaagtcgtta atatttagtg tttttttttc gtttgtaagt 5520
   tttagaaata tcggttttta agtttgtata ttttaggttt atatattatt ttgggaagta 5580
   tatttttatt tatggtattt ggttttttat aaaaatatag atgtttttat tttattttaa 5640
   attatatttt cggtaatttt tttttaaagt tttagtgatg attgttttag tttggggaag 5700
   gggagagcga gtttcgagga ttagaggttt cggatttttt cgttggtttt taaaatgttt 5760
   gatttagaag gttagtttta aaagattttt tttaggttgc ggttgttttt ttttatattt 5820
   gaacgattat tttgcgattg acgaggtaaa taggaagtta tttagtttag gatttcgttt 5880
   atagtagatg tttgttaaat attttttaga gtattggaag ggagagatat tgtttttaat 5940
   tatttaaatg attaaggttt ggaggttatt ttttagttta ggagtattag aaatttgatt 6000
   tttagattta tagattttta tgtatttttt ttaatgtatt tttttgaggt attgtttgtt 6060
   ttattttaaa tttttttttg aagtgaaatt gtgttggtcg gagagaattg gaaaaaagta 6120
   ttatttgaat tttagaggat aaagatagtt tgtataggtt agttaattta gtttgattaa 6180
   agatggtttt gaaaaagtaa aagagtaagt aagttttaaa tatagttttt taaaattttt 6240
   gagttttttt tttattataa agaatatgta tatttttttt taattaattg gaggtttaaa 6300
   aaagttaaaa aaaaaaaaat tatgaaaatt atttggagat gtatgcgttt tttttttgag 6360
   ttatatttgg tgatagttga tttgatatgt attttaaata tagtataggt aaatttagat 6420
   taatttttgt attttttaga tgtataaggt aaggtgtagg ttatatattt tggaagtaaa 6480
   tttgaaattc gtttagtttt tagttgttaa agtgtataaa agggtttaat ttttagttag 6540
   gttaaacgta gttaatgttt tataattatt tttgaaaatt ttttgttaat atttgttagt 6600
   tataaggtat ataggtttat ttttgagaaa ttcgaaataa aaattaatat tattggaatt 6660
   tttttatttt aatgaatttt tttgggaaag gaaaagttag gtttttttta ttattttgga 6720
   atattgaagt ttaggtagag ataaataaat tataaagtta ggaataggaa aatagagata 6780
   aaaaaattat cggtgtttaa aattagagtg ttaagttttg attatgaata ggaaagatgt 6840
   tgagagagga attagtttgt ttttggggtt tttgttagtt ttttttttag ttttttgttg 6900
   aggtggagat aaatcgggaa aggggtttat ttttgttggg aaagggttgg aatcgtaata 6960
   taacgttagt tttttatgga aagttttaag tttgtatttt atttaatttt ggttgcgttt 7020
   ttagaacgtt ttatttattt ttttttggcg gagtgatttt gggtatttta aattgttttt 7080
   tttagttagc gttttttttt ttatttttaa aatgagagat aataattttt atttgagatg 7140
   attttggtgg tgattaaatt taataggtta tttaaagtaa ttagaaggta taggaggttt 7200
   aggttaaaat attattgtat attaaaagta attataatgt atcgaatgtt ttttttttgt 7260
   taaggatcga aagataaggt gtattttaga gtttacggta ggttttttgt aaaattttcg 7320
   gggttgagaa tttggttcgg tttgagaagt gtttaggttt ggggatagtg atttagcgga 7380
   gaattggttt tgttacgtag aaggtattta gtaaatgtta gtttttaggg atgtatgcgt 7440
   agttgtaata tttgttagta aaaaagcggg gttttaatag gaatttagtt attaggaagt 7500
   attaattttg aaggatgttt tagtgtttgg atttatttag gattttattg gtttttttta 7560
   atatatcgtt gttagtagat tatttttttt attttataga tgaatgttta aggttgggaa 7620
   gcgttaatat tttgtttaag gtcgcgtagg tagaagtggg agatatagga atttttttcg 7680
   cgcggttgag gtttttgagc gtagattttt aattttgtat tttttcgcgt ttttcggggc 7740
   gcgttatatt cgttaggttt ttttttgaag taatcgcgta cgcgcgttgc gagttttaag 7800
   gagaaaatat tttttgtaaa ttgtggagta ttgcgcgtag aacgtagcgt ttgtaatttc 7860
   gagggtcgaa ggttagtttg taattttagc ggttagaagg gggcggggga ggggtagcga 7920
   agggggttgt agacgggttt gggtttcggg taggcgttag gcgcggggag atgaaaggcg 7980
   ggtttcgggg gaaggggttg cgtcgggtga tcggttttag tcgcgtcggg cgtttcgttt 8040
   tcgttttgtt tcgttttcgt tgttcggggt agaggcggtt gttcgcgtcg gagtcgcgtt 8100
   aattgttttc ggttttagta tttagtttgc gcggagtcgg gataatttcg ttttaggagg 8160
   aatgataatt cgttttaatg ggataagttc gtttttagag gagtgataat tttttttaat 8220
   gggagttcgt tagcggttta ttttatcgag ttcgggtatt agaagtaggt agattcgcgg 8280
   gcgttaatat tcgttaggaa ttttataata tcgtttattt attttttaaa atattggttg 8340
   tgtgatttcg ggtaaaaggt tattcgtttt tgagtttatt ttttttattg gtttgttgta 8400
   gagtgagaat ggggaaggtt cggttttttg gtgtttagag agattatttt tcgagttata 8460
   tagtttagga agtcgggtag atagatattt agggttatag ttggaggttt tgggggtata 8520
   aacgttttgt ttgcgaagtt ttgtaagttt aagtttgttt ttttttgttt ttttgttttc 8580
   gttttttaag gttcggagtt aggatcgagt tcgggtttga gtcggggttg gagagaagga 8640
   gtttttgtta aaagtacgat gtagattatt gaaggttttt taaagtttga gttttttttt 8700
   tattttatag atgaagtcgt ttaggcgtag agaggggaga ggttggtttg attttagttg 8760
   tggtaaaatt taatttaata cggtgttttt ggaagaagat tattataagg atgggggcgg 8820
   gggaggtaga ggtaaaagaa gttgtatttt tttgtaggtt ttatagtttg gagaggggat 8880
   ggcgagttta tggggatatt tagttttgga ggtagtaggt gtgaggggag gagttgtggg 8940
   tcggtttttt gtagttttat atttaaattt aggttttatt ttttatcgtt tttcgtcgtt 9000
   attttgttgg gttttggttt ttttttttag aaaataggta tgttaatgtt tatttatagg 9060
   gttgttggtg agaattaggt gataagtttt ataaaatatc gagtagcgtt gagtgtgttg 9120
   gaagtaggta ttgatttagg agatagtttt gtttcggatt ttttagagtt aaattggttt 9180
   taagatttag agttgaattg gttttaagta aggttgaatt atgtattttt tgtgagatta 9240
   gataaattat tttatttttt tgtattgtag atttagtagt tgtaatatga gagagtgttt 9300
   agtatttagt aaatgtggga tattattttt attttttaag ttattatcga tgttattttt 9360
   tatttttttt tttaatagat ttttttgggt atcgtgtata tttgaaatcg aatgatttaa 9420
   tattggatga ttcgaggtat ggaatttatt gaatgatttg aggtatgtaa ttttttggag 9480
   ttttattttt tttttttttg taaaatggga ataattattt atttttgggg aaggaagtag 9540
   attggtagat ttatttagtg agaggtggag attaagggaa gaagaaattt taggtttaga 9600
   taaatagtag ggagtatttt ttgtttttta atttttgggt atagtttttt ggtttttttt 9660
   taatttttgt ttgattggag gaaagttgtt acgagtaggg tcgaggttga tttgtttgta 9720
   gagaa 9725

<210> 199
<211> 6699
<212> DNA
<213> Artificial Sequence

<220>
<223> chemically treated genomic DNA (Homo sapiens)

<400> 199

gtatttttta aggacgtttt atggtttttg aaaggtagag tgttttattt aaggtcgtat 60
agttggtaga ttgtaagttt tttgatttta agttttatgt tttttaggcg ttatttatag 120
ttgttttagg tttatgtttt tagggttaaa gaagtaagaa gattgttaag ttgtttttcg 180
aattatgata gaaataagag aagatatata aaagtatttt taggtttttt ttgtttttga 240
tttggagttg ttttagcgat tgtttaaagg agtgttttta tttattttgt ttagagaatt 300
ttgtagggat agaaatttgg gatggggtta tttagggata ggtttttatt tatgttgttt 360
attaattttt tttatgtagt tttttgaggt ttaagttttt aattaagttt tattatagtt 420
taagtatcgt ttatagcgag agttaagttg atagtcgtat ttttattggt ttatttttcg 480
tttatagttt tttttgtaag aaatataatt ttttttgttt agttttgtta agtttttttt 540
ttaatagaat tatagaagta ttaaaatggg ttgattatag aattttttaa gaaattatag 600
tttttttttt tttaaatttt tgaagtttgg tagtttttaa gttgtataga tggagtggat 660
agaaagtaag tttaaaggtg ttggagttga ttttagtagt atttttattt ttttaatttt 720
tttttgttta aatttgagat ggttggttag ttgagggtag tggggttttt tgaagaagta 780
acgtgttgta aaattattgt tcgatatagg ttttgtttag gtattttatt aggttaggta 840
ttttttattt tttttacgtg gtatgatttt gagaggtgtg tattattttt attttataga 900
tgagagaatg gaaacggggt ggttaagatg attgttttaa aaaggtaata tagtcggtga 960
ggggtatagt tagaatttta atttaaattt atttggttta aaagtcgtgg tgtttttttg 1020
tttgggatat tttttttttt attttttata tttttagatt ttttggttgt tttttatata 1080
agtgtggttt ttttttttat gtagatgtat attttatttt ttaaattttt ttttttttat 1140
atttagataa atatattttt ttttatgaag tttagttaaa atttagtttt attattaagg 1200
ttttagtatt ttttagtttt tgatattaat atttttaggt ttatttagta tttaagcgta 1260
tgggttattt tagtgtggta tagagttagg agttgtaagt tttttaaatg tggtatttta 1320
gaattttata ttgaagaggg gtagatatgg tttgaaaata gtatagttaa tataattgtt 1380
tgggtttttt tttggttata ataatgtatt ttaaaaattt taaataatat ttaaagaaga 1440
gtatgatatt gttatattta ttaaaagggg atatggattt taaaaagaaa aggttataaa 1500
aaattagttt atattgttat gggaatttat aaattttttt gatgtttttt tattattttt 1560
ttttgtggtt gttatatttt ttttaattgt attataaaag ttttgtagga tggagattga 1620
ttatgaattt tttttttttt gtattttttt tgatggtttt tatttagtag atattttata 1680
aataggttat ttaatttgtt tttaagtatg atttgttttt ggtttttgag ggattttttt 1740
tgttgtttag ttaaatttgt ttagtttaat taaataaagg atttgggttg gtttttaagt 1800
aaaaataggt gatttgaagg agagaagagg gggattaaaa gaatagaaat tagtatgata 1860
taatgttatt aattggaatt tattatttta ggagggttga gttagagttt agtttttgaa 1920
ttttattaaa taattttttt agttaattta tattagtgat aaaattgttt tggaatatgt 1980
aatttttata aggaaataaa ttagtgttga atttttataa agtatataaa aaagaaagag 2040
gtatgtttag gagtttatta tgtgttaggt tttttttttg gagggggagg agttggggag 2100
gtgggaatag agttttattt tgttatttag gttggagtat agtggtgtaa ttttaattta 2160
ttgtaatttt tgttttttag gtttaagtga ttttcgtgtt tcggtttttt aagtatttgg 2220
gattatagga gtttgttatt acgtttagtt aatttttttt tttttttttt ttttttgtat 2280
ttttagtaga gatagggttt tagtatgttg ggtaggttgg tttttaattt ttgattttaa 2340
gtgattcgtt tgttttagtt ttttaaagtg ttggtattat aggtatgagt tatcgtgttc 2400
ggtttgtatt aggttttaat tatatattat tttaaagtta gtttaattta aataattaat 2460
gggttgttgt taattttagt atttattaaa gtaagatttt tttttgagta gttttttgat 2520
agtttaattt tagttgttta gattttaaaa ttgagggaaa cgtttttagc ggaaattgtt 2580
tatttttttt aatagaggtt ttgtagaaag attgtagtat gtaataaagt gttttttttt 2640
ttagatagat gataaatttt gggagttggg gtggggtagg ttgaaaggaa aaattagatt 2700
tttggggaaa gaaagttaag cgatttaggt tgttataatg ggatttttta atttttattt 2760
tatggaagat tggtaaatat ttgagttatt tattttaagt tttttttttt tagttttttt 2820
tagattttaa ttatatttat tataaagaaa attgagtttg tattttaaag ttgagattga 2880
gtttttcgtt taaggggaaa ttaatcggag gtgttttttt agtatttatt ttttattaaa 2940
gagaaattgt ttttgaataa gagaaagtat cgtggaggtt tatttaaaat tatagaaagg 3000
tagggttgga agagaaattt attaatagtt acgttattaa tgcgaaaagt gagaaaaatg 3060
atttgttcga gttatattag ggtggatttc gggtaagatt taggtgtttt tgttgttagt 3120
ttatttttta agtttttggt ttttattcgt atttattgag attagagatc gatttatttg 3180
aggtagaagg tgttgtacgt atttttgtga aatttgaggg gatcgagaag gaattttagg 3240
aagtttgttt atattggggg tttggagatt ttcgggaaaa tagttagaat taaaacggat 3300
tagttagtag acgtagatat atataaagtg atagggatat agaagtagaa gttgaattgg 3360
gtagagcggg tagcggtcga tttatttcgt acggtcgttt tcgttagcgt taggtgtagg 3420
gtttatagcg ggttagtaga gggcgcgtcg tttttaggga gtcgagcggc ggtcgcgcgg 3480
agtcgggcgg gttaggggag tcgtaggcga gttagtcgcg tttttgaggt taggttattt 3540
agggtgtagg attcggttaa ggaggaggag gatggtgagg agcggaattt tttcgtgggt 3600
tcgaggggta gatagatagg agtgtttttt aagaattata ttacgttgcg taacgttgcg 3660
gttttcggat tttttggtgt aaaggggggt ggggtagggg ttgtgtgtgt gtgtgtgtgt 3720
gtgtgtgtat atatgtgtgt tggggggagg ggatttagag taagagttcg ggagagaaga 3780

agacgcgttc ggggaaaacg ttttatgtat atggagaaat tttttgtttc gaattgtatg 3840
tatcgagaaa cgagttttta gtattttttc gttgtttttt ttttagtgta aagtatttag 3900
ttagtgagaa atttttagt tttattttaa gtatcgtttt tttttttaa atatcggttt 3960
tgtatttttt agttcggttt tagcgtcgtc gcgagttttt tttaggattg gttttggcgg 4020
ggcgtttaga gttttagttg tattgtttta ggagaaagga ggggggcggt gtattttgta 4080
ggaggaggag aaggggggtg ggtggtgggg gagagagaaa attgcgcgga gatttgttaa 4140
gcgttatcgt cgtcgttatc gtcgttgtcg tcgtcgtcgt cgtcgtttgt gagtttcggc 4200
gggtagtcgg attgtcggtt tttcggggta tttgggttcg gcggggtata gttttgggcg 4260
ttgtcgaagt cgtcgtcgtc gttttcgcgg cgagtatagg cggttttttt cggagtttgt 4320
gtagttttag tttttcgggg gtggagaagt ggggggtggg gttgttgttt ggggggaaga 4380
agggggaggg ggtaacgtcg agagagttag tggtttttat ggtgatggag ttgaaagtgt 4440
aggaaattta aaggtttgga ttttgcgaga tagataaatc ggtgttaacg tgcgcggacg 4500
tcgtcgtcgt cgtcgtcgtt ggagttcgtc gggtagagtc ggtcgcggag ttcggagtag 4560
gcggagggaa gtgtttttag aattagttta gttagcggcg tttgtataga gcggtcggac 4620
gaagagtagc gagaggagga ggggagagcg gttcgtttac gcgttttgcg tcgtcgtcgg 4680
ttcgggaagg tagcgaggag tcggcgtttt tcgcgtttcg cggtcgtttt ggagtaattt 4740
cggatgttta gtcgcggtcg ttttttttag tagattcggg agaggagttg cggttaattt 4800
gtgtgttttt ttttcgtttc ggtgggagtc ggcgttgcgc gaagggtttt ttcggcggtt 4860
tatgttgtcg gttttgcgtt tgtttagttt cgggtgagtc gttttcggag agacggggga 4920
gcgcggcggc gtcgcgggtt cggcgtgttt tttttcgggg acgcgggacg aagtagtagt 4980
ttcgggcgcg cgttagaggt atggagcgtt gttttagttt aggggttatt ttttacgttt 5040
tggtggtggt tttggggttg cgggcgatat cggtcggcgg ttagtattat ttttatattc 5100
gttcggtatt tagcgataat ttgtttttgg tggattttat cgaatattta gattttattt 5160
ttgattttaa ggaaaaggat ttgaacgaga cgttgttgcg ttcgttgttc gggggttatt 5220
acgatttagg ttttatggtt atttcgtttt tcgaggatcg gttcggcggg ggcgggggtg 5280
tagttggggg cgcggaggat ttggcggagt tggattagtt gttgcggtag cggtcgtcgg 5340
gggttatgtc gagcgagatt aaagggttag agtttttcga gggtttggtt tagggtaaga 5400
agtagcgttt aagtaagaag ttgcggagga agttatagat gtggttgtgg tcgtagatat 5460
tttgtttcgt gttgtacgcg tggaacgatt tgggtagtcg tttttggtcg cgttacgtga 5520
aggtgggtag ttgttttagt aagcgttcgt gtttcgtgtt cgagggtatg gtgtgtaagt 5580
cgtttaagtt cgtgtatttt acggtgttgc ggtggcgttg ttagcggcgc gggggttagc 5640
gttgcggttg gattttattt tagtatttta ttattttcga gtgtaagtgt tcgtgttaga 5700
attcgggggt tttttgttcg tattcggata tttgatcgat ttttatcgac gttttttgta 5760
tcgtttttaa ttagttttat tattttttag cgagggtttt taatgaattt tttttttttt 5820
tttttttttt ttttttgggt tatagagatt tagttttttg gtttttgtaa tgtattgttt 5880
aattgtgtag gaatgtatat gtgtgtgtat atacggtttt agttttaatt tatttattaa 5940
aaggttagta ttatacgtta aaagttatcg gtttttattg tatttttaaa aaaaagtaag 6000
taaaagaaaa aaaaaagaat agagaaaaga gagatttatt ttggttgttg ttaataatgt 6060
taatttgtta tttatatttt agtgtttttc gtatggcgaa gtagggggga aaagttattt 6120
tttttttgaa gtataaagag acgggggaat ttttgtagag gattttttaa aagttatttt 6180
ttatttttcg gaaagtgttt tggttttttt tggatttcga agaagttata gagtttaatg 6240
ttattttata gttattgtaa atatagagaa taaatggaat gattaattat tgtaaattaa 6300
gagtatttgt tatttatttt ttataatatt tcgtgtagta aatgagaaag aagtgtagag 6360
taggattaaa gaaaattatt aaaatcgatt gcgttttata ttgatttttt gtgttggtga 6420
ttgatgggg tgggagggcg tgggaggggg cgggtagggg atgttgttgt gtttttattt 6480
tcgttgggga aggggtttat tttttcggat tttttttttg tggtatgatg gaggtgattt 6540
agtttttga gggcgttggg gggggggcg atattttta atttttatt aaaatattac 6600
gtggttttcg tttagttttt tagttttttt ttttatgtta ttaaagttcg ggtttgtagt 6660
gatttcgata tttagtcggt tttttggagt tgattttag 6699

<210> 200
<211> 6699
<212> DNA
<213> Artificial Sequence

<220>
<223> chemically treated genomic DNA (Homo sapiens)

<400> 200
   ttggggttag ttttaggggg tcggttgggt gtcgaggtta ttataggttc gagttttggt 60
   gatatagggg gaaaagttag gagattaggc ggaggttacg tgatgtttta gtgaggggtt 120
   agagaatatc gttttttttt ttaacgtttt taaaaaatta agttattttt attatattat 180
   aggaggggag ttcgagggga tgagtttttt ttttaacggg gtaggaggta tagtagtatt 240
   ttttattcgt ttttttttac gtttttttat ttttattaat tattaatata gaaaattagt 300
   gtggagcgta gtcggtttta gtggtttttt ttaattttgt tttgtatttt ttttttattt 360
   attatacggg atattataaa gaataaatag tagatatttt taatttataa tgattagtta 420
   ttttatttgt tttttatatt tataataatt gtaaaataat attgaatttt atagtttttt 480
   cgaggtttaa ggaaaattaa aatatttttc gaagaatgga aatagttttt taaaaagttt 540
   tttataaaag ttttttcgtt tttttgtatt ttaagaaaaa aataattttt tttttttgtt 600
   tcgttatgcg aagggtattg gaatataaat agtaggttaa tattattagt aataattaga 660
   ataagttttt tttttttttg tttttttttt ttttttttgt ttattttttt ttaaaaatat 720
   agtagaagtc ggtaattttt aacgtataat attgattttt taataagtaa attaaaattg 780
   ggatcgtata tatatatata tatatatttt tatatagtta aatagtgtat tataggaatt 840
   agaaagttag gtttttgtag tttagaaaaa aaaaaaaaaa aaaaaaaaaa agtttattga 900
   aaattttcgt tagagggtgg tggaattggt tggaggcggt gtagggggcg tcggtgggga 960
   tcgattaagt gttcgggtgc gggtaggggg ttttcgagtt ttagtacgag tatttgtatt 1020
   cggaaatgat ggggtattgg atgggaattt agtcgtagcg ttggttttcg cgtcgttgat 1080
   agcgttatcg tagtatcgtg aggtgtacgg atttggacgg tttgtatatt atgttttcgg 1140
   gtacggagta cgagcgttta ttgaagtagt tgtttatttt tacgtagcgc ggttaaaagc 1200
   ggttgtttag gtcgttttac gcgtatagta cggggtagaa tgtttgcgat tatagttata 1260
   tttgtaattt ttttcgtagt tttttgttta ggcgttgttt tttgttttgg gttaagtttt 1320
   cggagaattt tagttttttg atttcgttcg gtatggtttt cgacggtcgt tgtcgtagta 1380
   gttggtttag tttcgttagg tttttcgcgt ttttagttgt attttcgttt tcgtcgggtc 1440
   ggttttcggg gggcgaggtg gttatgaagt ttgggtcgta gtggttttcg agtagcgagc 1500
   gtagtagcgt ttcgtttaga tttttttttt tggggttaaa gatagggttt gggtgttcga 1560
   tgaggtttat taggggtagg ttgtcgttgg gtgtcgggcg gatgtggaga tagtgttggt 1620
   cgtcggtcgg tgtcgttcgt agttttagga ttattattag ggcgtagagg gtgattttta 1680
   ggttggggta gcgttttatg tttttggcgc gcgttcgggg ttgttgtttc gtttcgcgtt 1740
   ttcggaggag agtacgtcga gttcgcggcg tcgtcgcgtt ttttcgtttt ttcggaggcg 1800
   gtttattcga ggttgggtag gcgtagggtc ggtagtatga gtcgtcggga gagtttttcg 1860
   cgtagcgtcg gtttttatcg gggcggaaga aaggtatata agttggtcgt aatttttttt 1920
   tcgggtttat tggggaaggc ggtcgcggtt gggtattcga aattatttta gggcgatcgc 1980
   ggggcgcggg aggcgtcggt ttttcgttgt tttttcgggt cggcggcggc gtagggcgcg 2040
   tggacgagtc gttttttttt ttttttttcg ttgtttttcg ttcggtcgtt ttgtgtaagc 2100
   gtcgttggtt gagttggttt taggggtatt ttttttcgtt tgtttcgggt ttcgcggtcg 2160
   gttttgttcg gcggatttta gcggcggcgg cggcggcggc gttcgcgtac gttggtatcg 2220
   gtttgtttgt ttcgtagggt ttaagttttt aaattttttg tatttttagt tttattatta 2280
   tggaaattat tgattttttc ggcgttgttt tttttttttt ttttttttta aataataatt 2340
   ttatttttta tttttttatt ttcgaggagt tggagttgta taggtttcgg gggaagtcgt 2400
   ttgtattcgt cgcggaggcg gcggcggcgg tttcggtagc gtttagggtt gtgtttcgtc 2460
   ggatttagat gtttcgggaa gtcgatagtt cggttgttcg tcggagttta taggcggcgg 2520
   cggcggcggc ggtagcggcg gtggcggcgg cggtggcgtt tggtaggttt tcgcgtaatt 2580
   tttttttttt tttattattt attttttttt tttttttttt gtaaaatgta tcgttttttt 2640
   tttttttttt ggagtaatgt aattggggtt ttaggcgttt cgttagggtt agttttgggg 2700
   aaagttcgcg gcggcgttgg agtcgggttg gagggtgtag agtcggtgtt tggggagggg 2760
   gggcggtgtt tgaggtgggg ttggagggtt ttttattagt tgggtgtttt gtattgggag 2820
   gggagtagcg ggaagtgttt gggggttcgt ttttcggtgt atgtagttcg gagtaagaaa 2880
   tttttttatg tgtatggagc gtttttttcg agcgcgtttt tttttttttc gggtttttgt 2940
   tttaagtttt ttttttttaa tatatatata tatatatata tatatatata tatatatagt 3000
   ttttatttta tttttttttg tattagggag ttcgagagtc gtagcgttgc gtagcgtgat 3060
   gtaattttta ggaagtattt ttgtttgttt gtttttcggg tttacgaggg ggtttcgttt 3120
   tttattattt tttttttttt tggtcgggtt ttgtattttg agtggtttag ttttaggggc 3180
   gcgattggtt cgtttgcggt ttttttagtt cgttcggttt cgcgcggtcg tcgttcggtt 3240
   ttttgggggc ggcgcgtttt ttgttggttc gttatgagtt ttgtatttgg cgttggcggg 3300
   ggcggtcgtg cgaggtgggt cggtcgttgt tcgttttgtt tagtttagtt tttgtttttg 3360
   tgtttttgtt attttgtgtg tgtttgcgtt tgttgattgg ttcgttttgg ttttggttat 3420
   tttttcgaag gtttttaggt ttttagtgta aataaatttt ttgagatttt ttttcggttt 3480
   ttttaaattt tataagaatg cgtgtagtat tttttgtttt aggtaaatcg gtttttggtt 3540
   ttagtgggtg cgagtgggga ttagaaattt gaaaggtgag ttgatagtag aggtatttga 3600
   gttttgttcg ggtttatttt tgatataatt cggataagtt atttttttta tttttcgtat 3660
   tagtgacgta attattaata gatttttttt ttagttttat ttttttgtga ttttaaataa 3720
   atttttacga tgtttttttt tgtttagaag tagttttttt ttgatgggag atgggtattg 3780
   aagggatatt ttcggttaat ttttttttgg acgaggaatt taattttagt tttaaaatgt 3840
   aaatttagtt ttttttgtgg tgaatgtgat tggaatttgg aaaaagttgg agaagagaga 3900
   tttggagtgg atgatttaag tatttgttag ttttttatgg agtgggagtt aagaggtttt 3960
   attgtgataa tttaaatcgt ttaatttttt ttttttaaag atttggtttt tttttttaat 4020
   ttgttttatt ttaattttta aagtttatta tttgtttaag gaagaaaata ttttgttgta 4080
   tattatagtt tttttgtaaa atttttatta aaaggggtgg gtagttttcg ttgaaagcgt 4140
   tttttttaat tttaaagttt agataattag aattagatta ttaagggatt gtttaaaagg 4200
   agattttgtt ttgatggata ttggagttaa taataattta ttggttgttt gaattgggtt 4260
   gattttgaaa taatgtgtga ttagaatttg gtataggtcg ggtacggtgg tttatgtttg 4320
   taatattagt attttgggag gttgaggtag gcggattatt tgaggttagg agttggaaat 4380
   tagtttgttt aatatgttga aattttgttt ttattaaaaa tataaaaaaa aaaaaaaaaa 4440
   aaaaaaatta gttgggcgtg gtggtaggtt tttgtaattt taagtatttg ggaggtcgag 4500
   gtacgagaat tatttgaatt tgggaggtaa aggttgtagt gagttgagat tgtattattg 4560
   tattttagtt tgaataatag agtgagattt tgtttttatt tttttaattt tttttttttt 4620
   aaaaaaagaa tttagtatat agtaggtttt taagtatatt tttttttttt ttatgtgttt 4680
   tatggggatt tagtattagt ttgttttttt atgaagatta tatgttttag agtaattttg 4740
   ttattgatgt gaattaatta agaaaattat ttagtggagt ttaagggtta aattttagtt 4800
   tagttttttt gagatggtag gttttaatta atgatattat attatgttgg tttttgtttt 4860
   tttaattttt tttttttttt ttttaaatta tttatttttg tttgaaaatt aatttagatt 4920
   ttttatttag ttgggttgag tagatttgat tgaatagtaa aaaaggtttt ttaggaatta 4980
   gaaataaatt atgtttgaga atagattggg tgatttgttt atggagtatt tattgggtga 5040
   agattattag gaaaaatata aagaagaaaa aatttatagt tagtttttat tttgtaagat 5100
   ttttgtagta tagttagggg agatatgata attataagaa gaaatagtaa aaggatatta 5160
   gaaaaattta taaattttta taatagtgtg gattagtttt ttataatttt ttttttttaa 5220
   aatttatgtt tttttttgat agatataata atattatgtt tttttttgaa tgttgtttaa 5280
   agtttttaaa atatattatt ataattaaaa agaagtttaa gtaattatgt tagttatatt 5340
   gtttttaaat tatatttatt tttttttaat gtgaagtttt gaaatgttat atttgggaag 5400
   tttgtaattt ttaattttgt gttatattgg agtagtttat gcgtttaggt attgagtgaa 5460
   tttggggatg ttggtattag agattaggaa gtattaagat tttaatggta gagttgaatt 5520
   ttagttgagt tttatgaaga aggatgtatt tatttgagta tggagaggaa gagatttgag 5580
   agataggata tatatttata tgaagaaggg aattatattt gtatgaggga tagttagaaa 5640
   atttggggat atgagaggtg gaagaggaaa tgttttagat agggaagtat tacgattttt 5700
   ggattaggtg gatttaagtt ggaattttgg ttgtgttttt tatcggttgt gttatttttt 5760
   taaaatagtt attttaatta tttcgttttt atttttttat ttgtaagatg gggatgatat 5820
   atatttttta gaattatgtt acgtaaaaga ggtaaggaat gtttagttta gtaaggtgtt 5880
   tgggtagagt ttatatcggg taatggtttt ataatacgtt atttttttag agaattttat 5940
   tgtttttaat tggttaatta ttttaggttt gagtagaaga gggttgaagg ggtgaggatg 6000
   ttgttgaaat taattttaat atttttggat ttgttttttg tttattttat ttgtgtaatt 6060
   tgagggttgt taggttttag gagtttgagg gagggaggat tgtagttttt taggagattt 6120
   tgtagttaat ttattttgat gtttttgtgg ttttgttgaa gagggagttt gataaaattg 6180
   gataaagagg gttgtatttt ttgtaaagga agttgtgggc ggaagatggg ttaatggaag 6240
   tacggttatt agtttgattt tcgttgtggg cggtgtttgg attgtagtgg ggtttggttg 6300
   gggatttagg ttttaggagg ttatatagga agagttaatg aataatataa atggaggttt 6360
   gtttttgggt agttttattt taggtttttg tttttgtaaa attttttggg taaggtaaat 6420
   ggaagtattt ttttaaatag tcgttggggt aattttaggt taaaggtaag aggagtttga 6480
   gggtgttttt atgtattttt ttttattttt attatgattc gaagaataat ttgataattt 6540
   ttttgttttt ttggttttgg aaatatagat ttaaggtaat tgtgggtgac gtttaaaggg 6600
   tatggggttt gaggttagaa aatttgtaat ttattagttg tgcgattttg ggtgagatat 6660
   tttatttttt aaggattatg agacgttttt ggagaatgt 6699

<210> 201
<211> 6534
<212> DNA
<213> Artificial Sequence

<220>
<223> chemically treated genomic DNA (Homo sapiens)

<400> 201

tttgattcga gcgaaagaaa gatttgttag tttttttttt ttagtatttt ttttttttaa 60
gagtaggata tatagtttta gttttttatg tatcggcgtt cggaggtagg agtttttgga 120
gtggttattc gtttattttt ttgttttacg agttaaggaa attaacggat tttaaggttt 180
tttttattta ttttttttag ttttgttttc gtttttattt tatttatagt ttttatttgt 240
tttcgtatag attagatttt ttggtttttt taaggtttag gttgttcgtt tttaatataa 300
gacggttttt tagttaaata aggtttttta atttttattt tttttatata tattagattt 360
ttttatatat atatatatat atatttcgaa tatatatatt tcgaatatat atatttcgaa 420
tatatatatt aaagagtagt tatcgaagaa tttgtttttt aaatattatt tttattttat 480
tatgaatttt tatttaattg agtagaatat tttttggaag attagatagg atttatttat 540
tttttataag atattttatt tttttcgttt tggagaatcg ggcggcggaa ttttgttttt 600
tttgtttgtt taaaaataat ttttttagat ttaaatgaat taggatatta gttgttgtga 660
gatttatttt tttttttttt ttttattgtt tttaaaaaaa aaagtatttt tttttttttt 720
tttttttttt tttttttttt tttatttttt tttttttttt ttattttttt ataaattata 780
tgttttttat tgtaaattcg tttatacgtt ttttggggtt tttttagttg aaggagtaaa 840
tttatttcgg ttggtttata ttatgtttta atgggatgtt tatattttta tttagttttt 900

taattcgaat ttatttcgat tgatttttcg atggggagaa gagcggcgtt aatgtttttg 960
ataaataatt gcggggggtt taatgattgt tattgaggta gcggttgcga aggattacgg 1020
gtttaattcg ggtatttatt aattttgtat tattatattc ggatcggaag tttatgatta 1080
cgaatttggc gttttgatgt ttttttcgtg agttttcggt agtttttttg tgggattgag 1140
ggtttttcgg cgtttgttta atttttattg tattttgaga attacgtttg gggtcgggcg 1200
ggtggttagg aatttttttg ttcgatcgtg ttagtttgtt agagttcggt tttttagacg 1260
ggaggaaaaa ggtttgggtg gtagatatta ttttcgtttt ttattaggtt agggatatta 1320
ttttgtatag attataattt ttttttaaga tttgtgtttg gttgagtatg aagttaattt 1380
ttagaggttt tgagaagttt acgggttatg aaatttttgt gttagtgttt ttaagtgcgg 1440
aggttgttcg ttaaagggtg ttttatagtt taggtcggtt attaaagtgc gattgggttg 1500
tagacgtacg tatttaaacg ggttgcgagg gagaattttt aagttaataa gggtacgttt 1560
tatttaggtt tggatagtta gttcgttgtt atttttattt gtagtgtttt tgaaattggg 1620
ggtagaggcg tagaggggat aatagaagtt atttttttcg tttatttttt tttttttttt 1680
gttaaattat ttaaatatcg tttttaaggt tttcgtttta tttcgaacgt gtatagagcg 1740
tgagtggatt ttatattcgt ttataatatc gttgtggaga gttttattga tgtttttggg 1800
gtaatttgtt agtttaggta aaagttattg ttttttaaat tagggtttaa ggatgtaggg 1860
cgcgggtttt cgttttttaa gttttatcgt tgggttattt ttttattaat tagcgtttgt 1920
atcgcggtta aatttggtat taggattttt tggtttatcg gttgcggata gggaagtggt 1980
ttggattttt atttagttgc ggggtttggg tgtggggtgg tttgggtttc ggagagattt 2040
tagaaagaaa taaaggggag gtttaagatt cggagataga gaaagagtgg ttggaattat 2100
tcggtatttt tgagatttgt tgcgttttgt tttgcggagt tacggttttg agtcgggtta 2160
ggtttggcgg cgtgcgggcg ttttaggtta ggtagtttta gttaattttg ttgttttggt 2220
tattttaggt cggatttcgg ggatataggt tataagaaat ttagatttat taagtttttg 2280
gatgtggttt agttgtttag agtttaaggg tgttttttta ttttttttcg ggtgtttagg 2340
gatttttggt tattggtcgg tgagaagggt cgggttgatt atttgggtat atatgaattt 2400
tagagcgttc ggtagaaaag aaagaggtcg gaaaggagag aaaatgagtc gttttttgat 2460
ttcggagttt taggttttta gtatttttat atttttgtgg tttttatttg tatatgttta 2520
tagtaagtga tagttagttt atgaatttat gggtatttaa gatttagttt agtcggtagt 2580
attttcgttt aacggtttcg cggattttgt aatttacggg atcgggagaa ggaattttgt 2640
agtaagcgtt tttttttagt atttgtcggt ttttaggaga gaaaattttc ggtaggagag 2700
agacgggtgg acgttgggag ggtttggaga ttaagttttc ggtttaaaaa taaatgagat 2760
ttagttttag atgtttttta attgttcgcg gggtttaaga gtttatttta gttgagagtt 2820
gagtttattt ttgttttttt gaaattatat ttttggtatt ttttttatat agtaattatc 2880
gttgatttta gaaagtgttt tgtcgatttt gtttaatttt tgggaagaag aaaatttgga 2940
aaaagtttag tttttaaaat gttatcgttg agaagtggtt taaaacgtac ggatataggt 3000
tttggtggtt ttaacgtttt aaaaagaaaa tgagaaaatt ttttttttga aaaattgaat 3060
tatttaatgt aaaattaggt taaataaggt atatgttttt tttatgggtt tagatatttt 3120
tgggagtttt ttgagatttg ttttcgtggt tttgagggtt aattaagttt tttggtatta 3180
ggtaggttga aagtttgttt ggttttttta atggtgagtg ggtttttaaa gggttaattt 3240
gtggattgtt gagcggttat attttatatt tttttatatt ttgttttttg agtttgtttt 3300
aattagggaa tggggaagta gggttggggg ttcgagttta cgttaaaatt gttaaaggag 3360
gtgggggagg gggatagaga gagaggagtg gagaattttt tttaggggta ggaaaaattt 3420
aaaaattata aaaggaaaag ggggaatttg aagtttagaa gttattgttt tttttatttt 3480
aaagggaaaa atttgttcga tttttatttt tttgattgat taaggttttg aatatttcgc 3540
ggtttcggag tgatagtttt tgaataggtt cgagcgaata aagcgtagtg tagagcgcgg 3600
ggttggtatt cgggggtgta aaggaggcga gttcgttggt atttattaag ttataaataa 3660
aaggttatgt ataatggtat tttttttaag gatagatagt ttttataata tttttggcgt 3720
tatattttgt tggggatatt ttagttttta gttaagattt cgtttttttt atttttttag 3780
tagtttagtt tgaatgttat aataaatttt tgagaataaa cgttgaattc gggtaaaatt 3840
ttaatatata gatatatttt tgtcgacgta tcggggattt atatgtagag atttagaatc 3900
gtagtttgtt agagcggttt ttatattaag aagaggagtt aggttttttt ttgtattttt 3960
ttttatattt ttagttttta attaacgagt gtttgggttt agcgacggtt gtttgtgttt 4020
tatattagtt tcgtttgcgg acggagaaga taaaagaata ttagcgtatt ttggattttt 4080
tttaggagga gttttatcgg gaggattttt taataagttt aggttaaggg gtattgatta 4140
taggaaggaa agttaaatat gtttggggtt ttagatgttt ttttattgga attgtgtttt 4200
ttttagtggt agtaagttaa gagaaatgag agcgagattt ataggtagaa aaaatgagat 4260
atagagagag atataggaaa ttataagagg aatagaggtt gagcgagata tatatataga 4320
ggtatagaaa gagatagaga gggaaataga aagttaagga aagagtgatt agagaaagat 4380
atatatatat atatatatat atatatatat atatatatat atatatatag agtgatatag 4440
atagagagat agagatagag agataggaat tttttcgttt ttagtaattg ttattttttt 4500
ggggttgttt tttttagttt ttatcgggtt aatttttttt tttgggtaag gggcgtagcg 4560
cgggtttttt tcggggttag tagaggtttc ggtattatta gagatgggaa gagaaagtgg 4620
tcgttgttgt ttaattagcg cgtgttttcg ttattcggga cggtttattc gtcggttaat 4680
cgtagtttag ggtttttgat taagttttgg gtaaaagaat taataaatgt tttcgagttc 4740
ggattttcgt attcggtgtt attataggag gagatttagg taggtcgcgt tttagtttta 4800
ttaggttttt cggttcgtcg tggttttttg agtttttttt ttagggattt tagtcgttgg 4860

aatatttgtt tagattcgta ttaaattttt tcgttttggg ttcgggattt tggatttcgg 4920
ggttttttcg tttttttttt tttcgggttt tagtttcggt ttttggatta ggaatcgata 4980
gttttttttt tcgcgttttt tttttttttt ttagtcgttt tggggagggg ttttttacgt 5040
ttcggatagt tttcgagggt tattcgcgtc gtattcgttt tttcgtttcg tttttatttg 5100
gatataattt tcgagcgaag ttgtttttag gatgattacg ttggtcggcg ttgtgtttag 5160
gatgatgcgg tcgggttcgg ggtagaatta ttcgcgtagc gggttttcgt tggaaggtaa 5220
gggagggttt tagcgcgtcg tttggatttt agggtttggg atcggttgtt ttattttatt 5280
tttaggtttc gtaggttttt ttggtgtttt taggaagttt attttttggg tattttatat 5340
tttaagaagt attagtcggg ggcgaggatt tattcgattt tttttttgta aatggagcgc 5400
gttgtttttt gtaaattttg gcggagttgg gcggttaggt ttgcggcgag tcggggagat 5460
ttgtttttgt tgttttggaa atgtcgcggt ggagaaggag gtgaatcggt ttttgagcgg 5520
tagaagtaat tttttcgtaa tttgtttatt cgaaattgtt tcgaagttgt ttcgaaattg 5580
tttcggttgc gtttatattt gtgaaatata tgttagttta ggaagtattt tttttttttt 5640
gttttgtaaa gttgtggtgg ttaacgtcgc gttattgtga ttggaatttt tagaataatt 5700
aaataaatta gaattttatg tattttaaat agtatttaga agtttttata atttgtttag 5760
gaatcgtttt ttcgtttatt tgaagatttt gggaatttcg gggaggtagg aagttttgta 5820
agtttgagtc gtttttaatg gttttttagg ttagggcgtt ttagtagaga tgtttatcgt 5880
tttcgttatt tatttttttt atatttagtt tagagtagaa tgtaatattt agagtaagaa 5940
tgggggttgg gggtttagga atagttttgt cgttgttgtg gtttttgttt taagatgtaa 6000
taggtgatat aggtattgtt gtttgggttt tttttttgga ttttttttga tttttggttg 6060
ttgtattttt ggttagaaat tttaagggtt attagttggt ttaagttttt tagaaaggga 6120
gagattttaa ttggataggg atcggtggga gtagagttta ttaaggttag tttttgggtg 6180
aatttttttt cgttaggagg ttgtttttag agtaagttag attttttatt agttgtaggg 6240
ttttttaagt tgttattttt cgggttcgaa ggggaagacg ttttttttgg ggagaattat 6300
tttgttttat ttgttgtttt tatataagat tatatgcgtt atattgttga ataatttgaa 6360
taaatgtgat tgaaaggagg tttgggtttg ggagttcgat gtcgagtagt tttagcgtcg 6420
gtgttgtaat atgaggatag gatagaagat tgcgaaatta cgtgttgttg ttttttgttt 6480
tttatttttt tttagtgatt ttttttttgt tttttttttt tattttttta tagt 6534

<210> 202
<211> 6534
<212> DNA
<213> Artificial Sequence

<220>
<223> chemically treated genomic DNA (Homo sapiens)

<400> 202

attgtgggaa ggtgaaaaag agaagtaagg gaaaagttat tggaggaaaa taaaaagtaa 60
agaatagtag tacgtaattt cgtagttttt tgttttattt ttatgttata gtatcgacgt 120
tgaaattgtt cgatatcgga tttttagatt tagatttttt tttaattata tttgtttaaa 180
ttatttagta atatagcgta tgtaattttg tgtaaagata gtaaatgggg taaaataatt 240
ttttttagag aaagcgtttt ttttttcggg ttcggggagt ggtaatttag gagattttgt 300
agttggtaga gagtttagtt tgttttagaa atagtttttt ggcgaagaga agtttattta 360
ggagttggtt ttgatgaatt ttgtttttat cgatttttgt ttaattggga tttttttttt 420
tttaagggat ttgggttagt tgatagtttt tggggttttt agttaaaagt gtagtagtta 480
agggttaaag agggtttaga aaggaggttt aagtagtagt gtttgtgtta tttgttatat 540
tttgggatag agattatagt agcggtaggg ttgtttttgg gtttttagtt tttatttttg 600
ttttgggtgt tatattttat tttaggttgg gtgtgaaggg agtgggtggc gggggcgatg 660
agtatttttg ttggggcgtt ttaatttgga aaattattga aaacggttta ggtttgtaag 720
attttttgtt ttttcgggat ttttagaatt tttaagtgga cggaaaggcg atttttaaat 780
aagttataga aatttttaga tgttatttga gatatataga attttaattt atttaattat 840
tttaaaaatt ttaattataa tggcgcggcg ttagttatta taattttgta gggtaaaaaa 900
aaaaaaatat tttttgaatt aatatatgtt ttataagtgt gggcgtagtc gggataattt 960
cgagataatt tcgagataat ttcgaatgga taaattgcgg agaagttgtt tttgtcgttt 1020
agaagtcggt ttattttttt ttttatcgcg gtatttttaa aataataggg ataagttttt 1080
tcggttcgtc gtaggtttga tcgtttagtt tcgttaggat ttgtagagag tagcgcgttt 1140
tatttgtaga aaggaaatcg agtaggtttt cgttttcgat tggtgttttt tggggtgtgg 1200
ggtgtttagg gaatgggttt tttggaagta ttaaaggagt ttgcggagtt tggggatggg 1260
gtgaggtagt cggttttagg ttttgggatt taggcggcgc gttgaggttt ttttttattt 1320
tttagcggga attcgttacg cgggtagttt tgtttcgggt tcggtcgtat tattttgggt 1380
atagcgtcgg ttagcgtggt tattttgggg gtagtttcgt tcggaaatta tatttaggtg 1440
aaggcgaaac ggaaaggcga gtgcggcgcg gatgattttc gggaattatt cggagcgtgg 1500
agagtttttt tttaaaacgg ttggagagag agggagggac gcggggaggg gggttgtcgg 1560
tttttagttt agaggtcgga gttggaattc gggaaagggg aggacgggga ggtttcggag 1620

tttaggattt cgagtttagg gcggaaaagt ttggtacgag tttgggtaaa tgttttagcg 1680
attggggttt ttgaaaaggg ggtttagaga gttacggcga gtcggggagt ttggtgaggt 1740
tggagcgcgg tttgtttgag tttttttttg tggtgatatc gagtgcgggg attcgggttc 1800
gggagtattt attagttttt ttatttaaag tttggttagg agttttgagt tgcgattggt 1860
cgacgggtag atcgtttcgg gtggcggaga tacgcgttga ttgggtaata gcgattattt 1920
ttttttttta tttttggtgg tgtcgaggtt tttgttggtt tcgaggggga ttcgcgttgc 1980
gttttttgtt taggagagaa ggttggttcg gtggaaattg agagagatag ttttagggag 2040
atggtagttg ttgagggcgg agaagttttt gtttttttgt ttttgttttt ttgtttgtgt 2100
tattttgtgt gtgtgtgtgt gtgtgtgtgt gtgtgtgtgt gtgtgtgtgt gtgtgttttt 2160
ttttgattat tttttttttg attttttatt tttttttttg tttttttttg tgtttttgtg 2220
tgtgtgtttc gtttagtttt tatttttttt gtgatttttt gtgttttttt ttatgtttta 2280
ttttttttat ttgtaggttt cgtttttatt ttttttggtt tattgttatt ggagggggta 2340
taattttaat aagaaggtat ttggggtttt agatatattt agtttttttt tttgtggtta 2400
gtgttttttg gtttaggttt gttaaggggt tttttcgatg gggttttttt tgggaggagt 2460
ttagggtgcg ttgatgtttt tttgtttttt tcgttcgtaa gcggggttaa tgtgaagtat 2520
aggtagtcgt cgttaggttt aagtattcgt tagttgaagg ttgggggtat gggggagggt 2580
gtagaaaaaa atttggtttt tttttttggt gtaaaaatcg ttttggtaag ttgcggtttt 2640
aaatttttat atatagattt tcgatgcgtc gatagagatg tgtttgtatg ttaaagtttt 2700
gttcgggttt agcgtttgtt tttaggaatt tattatggta tttagattaa attgttggaa 2760
aaataaaagg aacgaagttt tggttaggag ttgaagtgtt tttagtagga tatgacgtta 2820
ggagtgttgt aaagattgtt tgtttttaga gaaggtatta ttgtgtatag ttttttattt 2880
ataatttggt aagtgttagc gaattcgttt tttttatatt ttcgagtgtt agtttcgcgt 2940
tttgtattgc gttttattcg ttcgagttta tttagggatt gttatttcgg ggtcgcgagg 3000
tatttagggt tttaattaat taaaaggatg agaatcgggt aggttttttt ttttgaaata 3060
aaggaaataa tgattttggg gttttaagtt tttttttttt tttttatgat ttttgaattt 3120
tttttgtttt tggagggagt tttttatttt tttttttttg tttttttttt ttattttttt 3180
tgatagtttt gacgtgaatt cgggttttta attttatttt tttatttttt aattaaggta 3240
aatttagaag gtaagatgta ggggggtgtg gggtatagtc gtttagtagt ttataagttg 3300
gttttttaaa aatttattta ttattgggga aattaggtaa gtttttagtt tgtttgatgt 3360
tagaaggttt ggttgatttt tagagttacg gagataggtt ttaggagatt tttagaaatg 3420
tttggattta tgaaggaggt atgtgttttg tttaatttgg ttttatatta aatgatttaa 3480
ttttttaagg ggaaaatttt tttatttttt ttttaaggcg ttggggttat taaagtttgt 3540
gttcgtacgt tttaagttat tttttagcga tagtattttg agagttagat tttttttaag 3600
tttttttttt tttaaaggtt aagtagagtc gatagagtat tttttggggt tagcggtaat 3660
tgttgtgtga aggaggtatt agaagtgtaa ttttaagaag gtaaaagtag gtttaatttt 3720
tagttaaaat aaattttggg atttcgcgag tagttaaaga atatttagag ttgagtttta 3780
tttgtttttg agtcggaggt ttggttttta agttttttta gcgtttattc gttttttttt 3840
tgtcgggagt tttttttttt aagagtcggt agatgttgga gggaagcgtt tgttgtaaga 3900
tttttttttt cggtttcgta gattgtagag ttcgcgggat cgttgaacgg aggtattgtc 3960
ggttgagttg ggttttaagt gtttatgggt ttataaatta gttgttattt gttataaata 4020
tgtataaatg gaggttataa ggatgtgggg gtgttgggga tttaaagttt cggaattagg 4080
aggcgattta tttttttttt ttttcggttt tttttttttt tgtcgagcgt tttgagattt 4140
atatgtattt agatgattag ttcggttttt tttatcggtt agtggttaga ggtttttgga 4200
tattcggaga ggggtgggag ggtattttta ggttttggat agttgggtta tatttaaaga 4260
tttaatgaat ttaaattttt tgtggtttgt gttttcgaag ttcgatttgg gatggttagg 4320
atagtaaagt tggttgaggt tgtttggttt agggcgttcg tacgtcgtta aatttaattc 4380
ggtttagagt cgtgatttcg tagaataggg cgtaataggt tttaggaatg tcgggtgatt 4440
ttagttattt tttttttgtt ttcgagtttt gggttttttt tttatttttt tttgaagttt 4500
tttcggagtt taagttattt tatatttaaa tttcgtagtt ggatgggagt ttaggttatt 4560
tttttattcg tagtcggtgg gttaaggagt tttggtgtta aatttggtcg cggtataggc 4620
gttaattggt aaaggatgaa tttagcggtg gggtttggaa ggcgagggtt cgcgttttgt 4680
atttttgaat tttggtttgg gaggtagtga tttttatttg ggttaataaa ttatttagaa 4740
agtattagtg ggatttttta taacggtgtt gtgaacgagt gtagggttta tttacgtttt 4800
gtgtacgttc gaaatgaaac gggagttttg aagacgatat ttggataatt tgataaaaaa 4860
aaaaaaaagg tgggcggggg agatagtttt tattgttttt tttgcgtttt tgtttttagt 4920
tttagagata ttataggtga aaataatagc gggttggttg tttaggtttg agtggagcgt 4980
gtttttgtta gtttgaaagt tttttttcgt agttcgtttg gatgcgtgcg tttatagttt 5040
agtcgtattt tggtgatcgg tttgggttgt gaagtatttt ttagcgaata gttttcgtat 5100
ttggggatat tggtataagg gttttatggt tcgtggattt tttagggttt ttgagggttg 5160
attttatatt tagttagata taggttttaa gaggaaatta tgatttatgt aaaataatgt 5220
ttttgattta gtggggagcg ggggtggtgt ttgttattta gatttttttt ttttcgtttg 5280
aagggtcgag ttttggtagg ttgatacggt cgagtagaga agtttttggt tattcgttcg 5340
gttttaggcg tgatttttag gatgtagtaa aagttgggta aacgtcgaga aatttttaat 5400
tttatagaaa ggttgtcggg ggtttacggg gaggatatta aaacgttagg ttcgtgatta 5460
tgagttttcg gttcggatgt gatgatatag gattggtgaa tgttcgggtt aaattcgtgg 5520
tttttcgtag tcgttgtttt agtgatagtt attaggtttt tcgtagttat ttgttaaaag 5580

tattggcgtc gttttttttt ttatcgaaaa attagtcggg atgagttcgg attaagggat 5640
tagatgagga tgtggatatt ttattgaaat atggtgtagg ttagtcgaag tgggtttgtt 5700
tttttagttg ggggagtttt agggaacgtg tgagcgggtt tataataagg aatatatggt 5760
ttgtgggaag ataggaagag agaaggaaaa taggaaagga aaaagaaaag aggaaaagaa 5820
gagagaggta tttttttttt taaaggtagt gagggaggga gagaagaata aattttatag 5880
tagttggtgt tttagtttat ttgggtttga gaaaattatt tttaggtaaa taaggaagat 5940
aggatttcgt cgttcgattt tttagggcgg gaagaatagg atgttttgta aaaaataagt 6000
aagttttgtt taatttttta aaaaatgttt tgtttaatta ggtaaaagtt tatggtgggg 6060
tgggggtgat gtttagaaga tagatttttc ggtaattgtt ttttggtgtg tgtattcggg 6120
gtgtgtgtat tcggggtgtg tgtattcggg gtgtgtgtat gtgtgtgtgt gaaagaattt 6180
ggtgtgtgtg aaaggggtaa gggttgaagg atttatttta gttgggggat cgttttgtat 6240
tgggagcggg tagtttaggt tttggaaaga ttagaaaatt tgatttgtgc ggaggtaggt 6300
gaaggttgtg agtgaggtgg gggcgggaat agagttggag gaagtgaata aaagaaattt 6360
tggggttcgt tggttttttt gattcgtgga gtaaaaaagt aggcgagtga ttattttagg 6420
aatttttgtt ttcgggcgtc ggtatataaa gagttggggt tgtgtgtttt gtttttgggg 6480
gaggagggta ttgggaaggg ggaattaata ggtttttttt tcgttcggat tagg 6534

<210> 203
<211> 5632
<212> DNA
<213> Artificial Sequence

<220>
<223> chemically treated genomic DNA (Homo sapiens)

<400> 203
   tgtagggatt tgtagggagg gggttttggt gttagagtag gggttgggtt gggagagtag 60
   ggtaattgta taaagttttg cgggggtagc gtaattttgg cgtgagggtt ttaagtttag 120
   aatttttagg gaattcgaat agttttcgtg tttttgtagt aaggaaatta ttttgatttt 180
   aaagtgggag gtataggtcg ttagagaacg gttttggata ggtggcgcgt gcgttttgga 240
   aagttgaggg ttggtaggag atgggttttt tcggagaaag agaagaaggg gtttaggatt 300
   ttatagttta cgaaaatgta ttatagggag gggtggaaat agtttagagt tggtgttatt 360
   ttttatagta ggtgttaaga gtttaagacg aggtgttaga ttaacgttag cgggtttttg 420
   cgttggtcgc gtttagtttt ttttagtttt acgtacgggt ttggttcgtt ttttggtttt 480
   ttgaggaagg taggggaatt ttagtcgttt tttttgagga taagattttt ttatagatac 540
   gggttttttt attagggatt tttaagggta gaagaagtcg gagattaggg ttttatatta 600
   gggcgaggga taggttggaa tggttatggt tggtttcgga gaggggcgta tagtttgttg 660
   gcgggtggta agatttttga gttaatgtat aattgtgagt agatggcggg ggttggcggt 720
   agttcggggt ttgggtttag ttaatgaggg ataattagtt ttaaattcgg tgggggtggt 780
   tgagagagtt agatagtgaa ggtagatatt tgttttttaa atagtttagc gcgttgggta 840
   gagggttgag tggtagagtt tttgggtttt ttaggttttt ttgttagggt tttttattat 900
   tttttttttt tttattattt ttgggtcgaa agtttttgtt tgtttagatt taagagcgcg 960
   ttataaaggc ggagggagag ggtagtttat ggggtaaatc gttgttatta ttatttgtta 1020
   gggtttgttt ttgttttttt tgatattttt atttttattt ttatgtagtt gtttatagga 1080
   atagttggtt aggatataag ttttgagttt ttagacgtag gggtagattt agcggggagg 1140
   gatgaggtag gaatggggga ttaggagtag ggttattata ggttttattg gggatttttt 1200
   tttatttaag tatggttaag tagagtgatt agtagttttt agttgtttcg gggatggaat 1260
   gggttgtaag gatatttggg taaatgttat tttgttcgag gttttgaaat tattttttgt 1320
   agtttttttt gtattttaag attagtttga ggatgttgga ggagggtgga agaggaagag 1380
   agtatgtatt agggtaaagt ggaaaattta tttatgtgat atttggtttt tgagagtaag 1440
   agtttttaat tttaggatta agttgaatgt ataataatta tttttatttt taaaatttta 1500
   aaatatttat taatgatata cgttttttat tttttgattt ttgggattag ttttattgtt 1560
   ttatattttg tttattttta gtttaatttt tgtaatggtt ttatagattt tgaggtgtgg 1620
   aatggattta aagattattt gtttaagatt ttgatgattg gtaataaaat ttatatgtat 1680
   tagaggttgt gttttatttt tagtatttta ttttataggt attatttgtt tagttattga 1740
   tatttttgat ttcgttaatg atttagataa tttgtggtgt tttttgtgga aatagtagtg 1800
   ttattattaa tttagagttt aaaagtatta aatttttaat agaatggagt ttgttaatat 1860
   ttaaagatgg aagaaggaaa attttgttgt aagttttttt ataaatattt atatgtattt 1920
   ttgattattt tgaggtttat ttttattatt attattattg ttagtgtttt gaagtttagg 1980
   atttttttat ttttaggtaa gaatttattt ttggtgttat tttagttaag gagttaattg 2040
   gagaattggt taggatattt tttggggata gttggtgtta ggtgatatat attttttata 2100
   ttttttttta tttattttta ttattttaga gtgttaggag ttaatttagt tagtatttag 2160
   gtaatagttt gtatatttat ttatttttta tagtaatata tacgatttag gagttgattt 2220
   taaagtttat gtatgtgtat tttggttggg aagttgtgtt ggtggttcgg gaaggtattt 2280
   ttttgttttt gtgtatttaa agttttaaga atataatttg aagtttttta ttttttatgt 2340
   ttagtatgtt ttttaggagt attggtgttt gtgtgggtat atttttgttt gtttgagaat 2400
   ggttttgttt ttgttttcgg gtggtaggtg gatataaagt gggttttgag gattttttat 2460
   ggaaggatat agtgggttat tggttgtggt ttgttttgtt ttagggttgg tgttagttag 2520
   tttatgtata tttttttttt tttttttttt ttgtttatgg tttgtgatat tattggttat 2580
   ttttagaagg ttgttttttg tttttgagta cgggtttttt atagttttgg attttttggt 2640
   aggattgaag taggagtaga gtggaggttg ttattaagat tagattatta ttatttttgg 2700
   agtttgtagg ggttttagaa gttgagattt tttattgaag tttttatttt atttttttta 2760
   tataggtagt ttatttgtag gtttattgtt tgttttgttt tttatgttgt ttttatgttt 2820
   ttgtgtggtt aaatttaatt ttttttttat tttgtttttg tagttataat ttttttgttt 2880
   tttttatttt tttttttgtt tttttatttt gtaagtatta gttatttttt tagatatgtt 2940
   aatatgaatg ttgggaatgt gtgttgtggt tattgggaat ggggtttttg tttgtgagtt 3000
   ggggtataat gatttaggtt tggagattat ttaggtttgg atattgaatt tgtattttta 3060
   ttgttttttt gaattttggt tatttaggag aattgagttt tattaaggat ttagggtttg 3120
   gttgatattt tgagtttttt tattttgttt tggtatagtt ttttatagtt tttgtttgat 3180
   tttttttttt ttattgtttt tgtggtaatt atataaatat gaaatagggg tgggagtaga 3240
   gaaagagagt ttaggattag gagttagaaa ttgtagtttt tgatgaggaa ttggttttta 3300
   aattttgaat tttatttttt ggatagtttt tgatttgtgg taatgttaat ttgtgtttta 3360
   gttttttttt tggaaaattt ttgttttttt tttcgatttt ttggaaagat gagaatttga 3420
   aggggtttgt gtttttattt atttagaaat aagtgagtaa aagttaattt agcgattgaa 3480
   tttaataagt agattttgga ttttaaattt tttgtaattt tttgttttat tggtgttagt 3540
   tttttttttt tagttgtttt gtaatgtgtt ggagagtaaa gattttattt ttttttgttt 3600
   attgttttta ttttgaataa ttagtaatgt gtttatgttt aatattttat ttagtatata 3660
   attgtagatg aattaattaa gtttagatgg tacgtttatt ataagaaagt tttgtgtata 3720
   agttgttttt tggggtgatt tttagtatta ttagggtttt aggtatgaaa ggaggtattt 3780
   ttaattttta ttgattatta atgaaagtag aagaggtatg gtggtttgtg ggatttgtgt 3840
   tttttgttgt ttagagattc gtttgggttt atgacggttt ttatgttgtt tagcggatag 3900
   tttttttttg ttttttattt tttttaatat gtttttgata aatgttagtt gaatgaatga 3960
   gtgtttgata ttagaaatat cgtttttgaa attgaagttt tattgtgaga tttagaattt 4020
   tttagagaat gaatagaatg aataatttta gtaggttaga gatgttaaga gatgcgtttt 4080
   ttaggagaag tcggtttttt aggcgtgttt atttttatgt ttagtgtgta ggattagagt 4140
   tttaatgttt ggtgggttag agttgagttt tagttttttt agagtttttt ggagagtata 4200
   aaaaaatagg agtaagtttt tttgaagtgt agtatttggg tggtagtttg gagagtttaa 4260
   tcgaggattg tttaatagtg aaggtggatt tttgaatttg aggattttag aatttttggt 4320
   tcgggtgtgt tttttagggt tgagaaggtt taggagtagt tggagataat agttgtttat 4380
   ttggaaaggg gaaagtttga gtatttgaaa gtattatttt tttttatttt tttaaatttt 4440
   tttaatgaat gtgtaggaaa ggagaatttt tttaattttt ttagtttttt attaggattt 4500
   taagaaagat ttagttttta gttaatttgt agtgttttag tttttattat ataaaggttt 4560
   aaataaaagg aattagaatt ttttgatttt agttaattta ttggtaaatt ttaggaattt 4620
   gattttttat tataaaatat tattttttag gattatttta agtaaaagat tttatttaaa 4680
   taatttttta ttattgtaga gttagttttt aagttggaaa aggagttttt agaaggaatt 4740
   ttttttttta gtttttttag ttggagtttt atttttttga ggagggttag tttggagtat 4800
   gtattaggac ggtgaggagt ttgggggaag tgggaagata gggaggataa tagagatagg 4860
   ggaagatttt agggttttgt ttttattaat tgttttattt gttattggtt aatgttttgg 4920
   ggaagttttt tttagttttt ttttttttgt tttgttttgt tttttttaga ttaggagagt 4980
   tggcgggtat gggtaaggaa taaagattta ttcgtgagtt agtttttaaa gaaagtagtt 5040
   tgcgttgata gtttgggggt agtaaggatg tagtttttta ggagaggatg tattcggtgg 5100
   tgggaagtta ggttggaggg gtttgagtga ttttttttat aggcgggtag ggtagtggga 5160
   gaggtggtgt gtggatattt ttgttttacg tttagggatt agtagtatga attagtttgg 5220
   ggggtttttt gtgaatggtc ggtttttgtt tttggatatt cggtagtaga ttgtgcggtt 5280
   agtagttagt ggaatgcggt tttgtgatat tttacggatt tttaaggtaa tgggttagta 5340
   tttttattta gtgatgggga taggaagtag ggagaaaggg tttttttgaa ggtaagagtt 5400
   tggggttgtt gtaggttttg agggtttttg ggatttgggt tattttttgg gagatttttt 5460
   cggaggttga aaaggggagt tttaggtttt taaaggtgag gttggatttt cgattttatg 5520
   gtttggttta gtaagttttg gttttgtttt atagtttttt tttgttttag ggatattttt 5580
   tttttttttg tttattatgt tttatttgtt tttgtttttt atttgattta gg 5632

<210> 204
<211> 5632
<212> DNA
<213> Artificial Sequence

<220>
<223> chemically treated genomic DNA (Homo sapiens)

<400> 204

tttgagttag gtgagaagta gggataggtg aggtatgatg ggtagaagga aggagagtgt 60
ttttgggata ggaggaggtt ataagataaa gttaagattt attggattag gttatgaagt 120
cgggagttta gttttatttt tgagggtttg aggttttttt ttttaatttt cgagaggatt 180
ttttaggaag tgatttaagt tttagaagtt tttagagttt gtaatagttt taggtttttg 240
tttttagagg agtttttttt ttttgttttt tgtttttatt attgggtaaa ggtgttggtt 300
tattatttta aggattcgtg agatgttata gggtcgtatt ttattgattg ttagtcgtat 360
aatttgttgt cgggtattta gaggtagggg tcggttattt ataaagagtt ttttaagttg 420
gtttatgttg ttgatttttg ggcgtgagat agaggtattt atatattatt ttttttattg 480
ttttgttcgt ttgtggagag ggttatttag gtttttttag tttggttttt tattatcgag 540
tgtatttttt tttgggagat tgtatttttg ttgtttttag gttgttaacg taagttgttt 600
tttttgagag ttggtttacg ggtgagtttt tgttttttgt ttatattcgt taattttttt 660
gatttaaggg aagtaggata gggtaggaag ggagggattg gaaaaagttt ttttagaata 720
ttggttaata gtagatgaaa tagttgatgg aagtaaagtt ttgaggtttt tttttgtttt 780
tgttgttttt tttgtttttt tatttttttt aggtttttta tcgttttgat gtatgtttta 840
ggttgatttt ttttagaagg atgagatttt agttgggaag gttgggaagg gaagtttttt 900
ttaggagttt tttttttagt ttgggggttg gttttgtaat aatggggggt tatttgggtg 960
aggttttttg tttgaaatgg ttttgggagg tagtgttttg tgatagaggg ttaggttttt 1020
aaaatttgtt agtgggttgg ttaaaattag aaaattttaa ttttttttat ttaaattttt 1080
gtgtgataaa aattaaaata ttgtaggttg gttgaggatt ggattttttt tgaagttttg 1140
ataaggggtt gggaggattg agagggtttt ttttttttat atatttatta gaaaagtttg 1200
gggaggtaga aagggataat gtttttaaat gtttaagttt tttttttttt aaatggatag 1260
ttgttgtttt tagttgtttt taagtttttt tagttttgga agatatattc gagttagaga 1320
ttttggggtt tttaagttta ggggtttatt tttattatta ggtagttttc ggttgggttt 1380
tttaaattgt tatttaaatg ttgtatttta gaaaggtttg tttttgtttt tttatatttt 1440
ttaggaagtt ttagggaaat tgggatttag ttttaattta ttaggtattg aggttttggt 1500
tttatatatt gggtatagag atgaatacgt ttgaagagtc ggtttttttt aggagacgta 1560
ttttttagta tttttaattt gttgagattg tttattttgt ttattttttg aagagtttta 1620
gattttatag tgaagtttta attttagaga cgatgttttt ggtattagat atttatttat 1680
ttaattaata tttattaaag gtatattgaa gggaatgaga agtaggagaa gattgttcgt 1740
tgagtaatat agaaatcgtt atgagtttaa gcgagttttt gagtagtaag ggatataggt 1800
tttataggtt attatatttt ttttattttt attagtaatt aatgggagtt gaagatgttt 1860
ttttttatgt ttggaatttt ggtagtgttg gagattattt taaggaatag tttatgtata 1920
gaattttttt ataataagcg tgttatttgg gtttagttaa tttatttgta gttgtatgtt 1980
gaataagata ttaagtatga gtatattgtt ggttgtttag gatgaaggta gtaaatagaa 2040
aagggtaaag tttttgtttt ttaatatatt atagagtaat tggagaaaag agattagtat 2100
tagtgaaata aagaattata aagagtttgg aatttagagt ttatttatta agtttagtcg 2160
ttaaattggt ttttatttat ttgtttttag gtaggtagag gtatagattt ttttaaattt 2220
ttattttttt aaaaagtcgg gaaagaaggt agaggttttt taaagagaaa attgaggtat 2280
agattggtat tgttataagt taggggttgt ttagagagta aaatttaagg tttagaagtt 2340
agttttttat tagaagttat aatttttggt ttttaatttt gggttttttt tttttgtttt 2400
tatttttgtt ttatgtttgt gtaattgtta taaaaataat ggagagagga gaattaagta 2460
gggattgtaa agggttgtgt taagataggg tgaaggagtt taaaatatta gttaaatttt 2520
aaatttttga tgggatttag tttttttaag tgattagaat ttaaaagagt agtagggatg 2580
taggtttagt atttaaattt aaataatttt taaatttaaa ttattatgtt ttaatttata 2640
aatagaaatt ttatttttaa tagttataat atatattttt aatatttata ttaatatgtt 2700
tgaaaaaata attggtgttt gtaagatgga aaaataggaa aggggtggga aggggtagga 2760
gggttgtagt tgtaaaggta gagtgggaga gaagttgggt ttggttatat agaggtatgg 2820
agatagtatg agaggtaggg tagataatga gtttgtaggt gagttatttg tgtggaggaa 2880
atgaggtggg agttttagtg ggaggtttta gtttttgaga tttttgtagg ttttaggggt 2940
ggtgatgatt tggttttggt aatagttttt atttgttttt tgttttagtt ttgttagggg 3000
gtttagagtt atagagagtt cgtgtttaag agtagagggt agttttttgg gagtagttag 3060
tgatgttata ggttatgagt agaggagaga ggaggaggga ggtgtgtatg ggttgattga 3120
tattagtttt gaggtagggt aaattatagt taatgattta ttgtgttttt ttatggggaa 3180
tttttagagt ttattttgtg tttatttatt attcggaagt agggatagag ttatttttag 3240
ataggtaaaa gtgtatttat ataagtatta gtatttttga agggtatgtt gagtatggaa 3300
gatggggaat tttaggttgt gtttttaagg ttttgaatat atagaagtaa aaagatgttt 3360
tttcgaatta ttaatatagt tttttagtta gaatgtatat atatgggttt tggggttaat 3420
ttttgagtcg tgtgtgttgt tataaaagat aaatgagtat atagattgtt gtttaaatat 3480
tgattgagtt ggtttttagt attttgagat aatgagagtg aatggagaag agtgtgaaga 3540
gtgtgtgtta tttagtatta gttgttttta gagagtgttt taattaattt tttaattgat 3600
tttttgattg aggtgatatt aagggtgaat ttttgtttgg aaatggaagg attttggatt 3660
ttagaatatt gataatggta gtggtggtgg aaatgagttt taggatagtt agagatgtat 3720
gtgggtgttt ataaggaggt ttgtagtagg attttttttt ttttattttt agatgttagt 3780
aggttttatt ttgttaggga tttgatgttt ttagatttta agttagtaat ggtattgttg 3840
tttttataaa agatattata ggttatttgg gttattggcg aggttagagg tattagtagt 3900
tgggtaggtg gtgtttgtgg agtgggatgt tgaaagtgaa atatagtttt tagtgtatgt 3960

aaattttgtt gttagttatt agggttttgg gtagatggtt tttgggttta ttttatattt 4020
tagagtttgt gaaattattg taaaagttag attggaagta agtagggtgt agggtaataa 4080
gattagtttt agaaattaga gaatggaaga cgtatgttat tggtaaatgt tttgaggttt 4140
tgggggtgaa agtggttgtt gtgtatttag tttgatttta gggttgggaa tttttgtttt 4200
taaaggttaa atattatata agtgagtttt ttattttatt ttgatatata tttttttttt 4260
tttttatttt tttttagtat ttttagattg gttttggggt ataggggaga ttgtagaggg 4320
tgattttagg atttcgggta ggatagtatt tgtttaagtg tttttatagt ttattttatt 4380
ttcggggtag ttggggattg ttggttattt tatttagtta tgtttgggtg ggaggaggtt 4440
tttagtaagg tttgtggtag ttttgttttt agtttttatt ttttgtttta ttttttttcg 4500
ttagatttgt ttttgcgttt aggggtttaa ggtttatgtt ttgattagtt atttttatag 4560
atagttgtat gagggtggag ataggggtat tagggggagt agggataagt tttagtagat 4620
ggtggtggta gcgatttatt ttataaatta tttttttttt tcgtttttat gacgcgtttt 4680
tgggtttgaa taaatagagg ttttcgattt agaggtgatg ggggagggaa aagtggtaaa 4740
gggttttggt aggggggttt agagagttta aaggttttgt tatttagttt tttgtttagc 4800
gcgttagatt gtttgaggag taaatatttg tttttattgt ttgatttttt tagttatttt 4860
tatcgggttt ggggttaatt gtttttattt agttgggttt aggtttcggg ttgtcgttag 4920
ttttcgttat ttgtttataa ttatatatta atttaaaggt tttgttattc gttaataagt 4980
tgtgcgtttt ttttcggggt tagttatggt tattttagtt tgtttttcgt tttgatgtag 5040
agttttgatt ttcggttttt tttgttttta ggagtttttg gtagaaaggt tcgtgtttgt 5100
agggaagttt tgtttttagg aaaggcggtt ggagtttttt tgtttttttt aagggattag 5160
agggcgagtt aagttcgtgc gtaaagttgg agaggattgg gcgcggttag cgtagagatt 5220
cgttggcgtt ggtttgatat ttcgttttgg gtttttgata tttgttgtgg gaggtaatat 5280
tagttttagg ttgtttttat ttttttttat ggtgtatttt cgtaggttgt ggggttttgg 5340
gttttttttt tttttttttc ggaaagtttt attttttgtt agtttttagt tttttagagc 5400
gtacgcgtta tttgtttaaa atcgtttttt ggcggtttgt gttttttatt ttaaagttaa 5460
agtgattttt ttgttataaa aatacggagg ttattcgagt tttttgaaaa ttttgaattt 5520
gagattttta cgttaaagtt gcgttatttt cgtaaagttt tatgtagtta ttttattttt 5580
ttagtttagt ttttgttttg atattaaaat tttttttttg taaatttttg ta 5632

<210> 205
<211> 5203
<212> DNA
<213> Artificial Sequence

<220>
<223> chemically treated genomic DNA (Homo sapiens)

<400> 205

agggaattta acgttttttc ggggtagaag ggggtagcgc gcgcgaggcg gaaggatagt 60
tattttcgtt gtataaattt tttcgcgggt cgcgtcgcgc gtgtaggggt tggacgttat 120
tcggcgttag agtcgggttt gaggagcggg gtttggtcgg gaacgagggt tgttagtttg 180
ggtggtgtta tttgttgttt tattttttat tttttttttt ttcgtgagga attagaggta 240
tcgggtcgcg cgggttgaga atttttaaat ttttttcggt cgtattcggt tgagttaatt 300
cgtagttttt gttagttttt ttgtttttcg ggtagcggtt tttttcgtta gttaggtttg 360
gaagttattt tttgtgagat tgggttagga agtgacgaaa agcgtcgaat tgtttttaaa 420
ttgaaaatat tttttttttt ttttttggag atagcgttga taaatatatg ggatttcggt 480
ttttgatttt tggttgtcgt ttttgttttt ttgtcgttaa taaaattcgt attgagtttt 540
ttagtgtttt gattaatagg tagattaatt tttattgggg ttgggaatag tttgttttaa 600
ttaatgttat tttaaaagtt cgttaggatg cgtttagttt cggttttttg gatgtttgag 660
cgagttagtt atgatatttg tttttttttt ttttttagtt ttaaattttt tagtaaagtt 720
tttagttttc gtttgtttgt ttttaggttt ataacgggat aaatcgtagg atcgggtcgt 780
tggtttttag aataggtagc ggtttagaga ggggatattt agggagaagg gattgaggtg 840
gttttgattt gtttgttttt tttttgaagt tgaatttttg tttttggggg gtagaggtta 900
tagggaaaag agagatgggg ggatcgttat agggtgatga aatggaaagt aaattttttt 960
aggaatttta tttgtaaaaa tcggagagtg tcgttgattt tagttgtttt atatgttagg 1020
agttttaaat ttaattttta tttggataat agtatggagg gattgggagg taaagttttg 1080
ggagtttgat tcgttgggtt gttaggaatt tttttgttgg gagattttat tggataatgt 1140
tattttaaag gtattttggg ggtgggtagg gagaagatag agagagaaag agagagagag 1200
agagagagag agagagagag agagagatag gttggttttt tagagcgaac ggaagaggat 1260
tgaggattga atttagggag tttttagtat tggttatttg gtaggaggcg aggaagggcg 1320
agggtatcgc ggtttttttt taagtcgagt agtggttata gtttgaaggt gttacggggc 1380
ggttttggga tttttttggt ttttttaggt tttgggttcg gcgaggttgt cgggttagcg 1440
ggaatcggta ggttgttgga gaaaggagag ggataaaggt ttggaagtgg gggaggtatt 1500
agggattggg cggtcgggat gggagttttt gtttttaggg tttattggat tcgtttgtta 1560
gtttcgcgga taagggattt tattttttta gggtttgtaa aaagagagac gtttggggta 1620

ttataaagtg acgtaggtgt ttgttaagat tttttttttt taaaaaatgt tagtttttat 1680
tttttttagc gcgtcggcgt tgcgtttttg tttgatcggg gaaagagaga gtttggaggt 1740
gttagaattg ttagaatttc ggatgggagg tttttggttt tgaagttggg agttggaggt 1800
attttttttt agaagtgggt atttttttgt tttttcgaag gtttgatggg gtgggtatag 1860
gggaatgtta gggatcggtg cgggtgagat gtttgtttta ttttatgttg acgtattaat 1920
attggttaga agttttgggt aggttttggc gagttacggt tttttagcgg gatttttttt 1980
acgtagatag tttcgttatc gggtgggacg atttcggcgg ttttcgcggg aacgtattcg 2040
tcggtttgag aggttttttt tttggttata gggggcggag ggtagggggg aataggggtt 2100
tgtggggagg cggggaaagg agatagaatt taaagcgtaa ttttcgtcgt tttttcgcgt 2160
tttggagggt agtaagtggg tttcgggcgt gttggtagtc gtaattaaaa tggcgatttg 2220
cgagttgttt cggttttgta tatatttttg gtaatcgcgt tcgtatttta gtttcggttt 2280
ttatattttt aggtcgtttc gtttttagag tcggtatttg gtagagggag tgaggttttt 2340
tacgaggtag aatagtattt ttattttttt tgagattagt ttagagagaa cgaggaaatt 2400
ttatttttat tttttttttt ttaaattttg aaaattcggt tttattttgg ggttgaattt 2460
ttttttttaa atattgatat atgggaagta gttaagtttt ttttgttttt tgtttgtgcg 2520
gaggagtttt taggagtttt tttcggagtt ttttttttta tttaatgacg ttagtggtat 2580
ttatttttcg ttttttgttt tttttgattt tttgttaaaa ttttttttcg tagcgagatg 2640
ggggttggag gggtgggaga aggagttgat ttaaagagtt agtgttttgg ggggaaatag 2700
taataagatt acggttgttt tttttcgagg taaagtttaa gaaggtgtta atttattcgg 2760
tcgttaattg agaggttgta aaaatgatat tcgtttttag ttcggataga attatttttt 2820
tttttttcgt tttttcgtaa gtttttcgtt tatgtatagc ggatttgagt tttgagttta 2880
agttttttta atgttcgttt ggaaagtaaa aggagagaga aagagaaagt cgattttcgt 2940
tggtttggga tttagggtcg cggttttatt tgtcggtatt tttcgaaagt cgcgtttttg 3000
cgaaatgaaa tttcgtttag gaggtcgcgg atttggatat tcggcgttat tttttttatt 3060
tttgatttag gttttttttc ggcgttgcga gttttcgggg aagggttaga gtcggtagtt 3120
ttttttagtt cggggagggg agagggttat gcgattttat ttttggttag ggtcggggag 3180
gtttttgttt ttcgggagtt ttgttcgggt tttttggtcg tagggttgtt gggttttagg 3240
taggaacgag agggtgaggt ttatatgtgg ttcggcggtt tagggcggtt tgtagcgttt 3300
ttattgtttc ggttgttagg ggttgcggcg acgcggttag ttagtagcga gtttaggtcg 3360
cgtagatttt attgatgagt tttgattttt agtatttttt ttaagttaag aagagtttag 3420
cgtatttttc ggttgtttta ttttagtttt tttgttttag ttttttagtt ttattttttt 3480
tcgttttgtt ttggggtgtg tatagtagtt taggtttttt tttttttttt cggtttcgtg 3540
gttcgaagtc gtcgagagag ttcgggatag cgtaggatta ggtagtcgtt cgtttttttg 3600
ttattttaat tgtaggtttc gaggggcgtt tttggagtgt attgaggtgt gttttaatcg 3660
tgcggtattt aataaatgga tttttggtgt gtggttagaa gagaaaagtt atttatttat 3720
tttttttttc ggttttttgg taatagttga aggggagttg ttttcgtgga ttgagtagat 3780
ttaggagagg gagtcgtggt gcggagatat acgtattata tatagatgat cggtggtata 3840
tacgatatac gttgatatat cgatatcgtt agtgggatat atatatatat atatatatat 3900
atatatatat atatagagag agagagagaa ttttttttag tattggttat tcgttttttt 3960
atttaggttt ttattttttt ttttttttta ttttttttgg tttttttttt tttcgggcgt 4020
tgcgaaaagt agtcgtattt agttaataaa tggtacgtgg gagaagttgg tgagtgtttg 4080
gtgaggattt tttagggttt tttataagaa ttttttgtat ataaagtaag tggcgtgttt 4140
attcgggttt ttttagttag agttgtgttt ttgtttcgtt gcgtatcgcg gttttcgaaa 4200
ggagaaagga gagaagaaag ggcggggaga gcggggtgga ggatttggat aggttttgga 4260
ggtttgggtt ggggaggttt ttggtttcgt ttagttttcg gttcggtaat tttttttcgg 4320
tttaggtttc gtcgcggttt tcgtagttgg aatggagttg ttaggattta gtgacgtttt 4380
cgtttttttt tttttttttt aaggggttag gtgggttggg gtgcggtcgt cgttgtgttt 4440
tgtgttttgg ggtttcggtt gggatggggt gggggcgggc gggggcgggg cggtaggtta 4500
cgttgttttg gagttggtaa gaaaggatag tatagaaatt tgtatttttc gaggattggg 4560
agtttcgagt ttagtttagg gggagtgggg gcgcgatttt taatttagaa atttttattt 4620
gatcgtttaa gttcgcggta gtagggcggg tcgcgtcgaa tttcggcgtg cgcggagcgg 4680
ggagatgtag gcgagcgtta gagttcgggt tcgggggttt tgcgtcgggg agaggagtcg 4740
ggatttatcg gcggagtcga aaataagtgt atttatattt aaataaacgg attaattgta 4800
ttaggcgggg agagggagta tttaatcggt tggcgcgagg tttcggcgtt gttttgtata 4860
aagtaatatt ttgtgtgaga gcgagcggtg tatttgtatg ttgcggagtg attagtgggt 4920
ttgaaaaggg aatcgtggtt cggttttatt tttcgttttg gtttaggcgt aggaggaagt 4980
gttttgttgg aggatgatga tagaggttag gtttcgttaa tgggttagtg aggagcggtg 5040
gaggcgaggt cgggcgtcgg tatatatata ttaatatatt tgagttatta ttaattagta 5100
taggtgtgtt ggttgtagtt atttttttta tttatatttt ttatttttta ttttttagtc 5160
gttgatagtt tattttattg ttaatttttg tttttttttt agg 5203

<210> 206
<211> 5203
<212> DNA
<213> Artificial Sequence

<220>
<223> chemically treated genomic DNA (Homo sapiens)

<400> 206

tttgggaagg agatagagat tgataataaa atgggttgtt agcggttgga gagtgagaga 60
taaagagtgt gggtgaggga agtggttgta gttagtatat ttatgttgat tggtgatggt 120
ttaagtgtgt taatgtgtgt gtgtcggcgt tcggtttcgt ttttatcgtt ttttattggt 180
ttattagcga agtttgattt ttgttattat tttttagtaa aatatttttt tttgcgtttg 240
aattagagcg ggaaatgagg tcgagttacg gttttttttt taaatttatt aattatttcg 300
taatatgtaa atgtatcgtt cgtttttata taaaatattg ttttatgtaa agtagcgtcg 360
gggtttcgcg ttagtcgatt ggatgttttt ttttttcgtt tggtgtaatt ggttcgtttg 420
tttgaatatg aatatatttg ttttcggttt cgtcggtggg tttcggtttt ttttttcggc 480
gtagggtttt cgagttcggg ttttggcgtt cgtttgtatt ttttcgtttc gcgtacgtcg 540
agattcggcg cggttcgttt tgttgtcgcg aatttgagcg gttaagtgaa ggtttttggg 600
ttgggggtcg cgtttttatt ttttttaagt tggattcggg atttttagtt ttcggagggt 660
gtaagttttt gtgttgtttt tttttgttaa ttttaggata gcgtggtttg tcgtttcgtt 720
ttcgttcgtt tttattttat tttagtcggg gttttaagat atagagtata gcggcggtcg 780
tattttagtt tatttggttt tttggaagaa gaggaaaggg gcgggagcgt tattgggttt 840
tggtagtttt attttagttg cggaggtcgc ggcgaagttt aggtcgagag gaggttgtcg 900
ggtcgagaat taaacgaggt tagaggtttt tttagtttaa gtttttaggg tttgtttaaa 960
ttttttattt cgtttttttc gttttttttt tttttttttt ttttttcgga agtcgcggtg 1020
cgtagcggag tagaggtata gttttggttg gagaggttcg agtaaatacg ttatttattt 1080
tgtgtataga gggtttttgt gaaaagtttt gaagagtttt tattaaatat ttattaattt 1140
tttttacgtg ttatttgttg attaagtgcg gttgtttttc gtagcgttcg agaggagggg 1200
aagttagggg agataagagg ggagggggag tggaagtttg ggtggggggg cggatgatta 1260
atgttgggag ggattttttt tttttttttg tgtgtgtgtg tgtgtgtgtg tgtgtgtgtg 1320
tgtgtgtttt attggcgatg tcggtatgtt agcgtgtgtc gtgtgtgtta tcggttattt 1380
gtgtgtggtg cgtgtgtttt cgtattacga tttttttttt tgggtttgtt tagtttacgg 1440
aggtagtttt tttttagttg ttgttagaaa atcggggagg aaagtaagta aatggttttt 1500
tttttttgat tatatattag aagtttattt gttgaatgtc gtacgattag gatatatttt 1560
agtatatttt aaaggcgttt ttcggagttt gtagttaagg tgataggaga gcgagcggtt 1620
gtttggtttt gcgttgtttc gagttttttc ggcggtttcg ggttacggag tcgggaagga 1680
gaaggaaggt ttgggttgtt gtatatattt taggataggg cgagggggaa tagggttgaa 1740
gagttgaggt agggaggttg aaatgaagta gtcgaagggt acgttagatt ttttttgatt 1800
tagggaaagt gttgaaagtt agagtttatt aataaaattt gcgcgatttg aattcgttgt 1860
tgattggtcg cgtcgtcgta gtttttggta gtcgggatag tgaggacgtt gtaagtcgtt 1920
ttgggtcgtc gggttatatg tgggttttat tttttcgttt ttgtttggga tttagtagtt 1980
ttgcgattaa ggagttcggg tagggttttc gggaagtaaa ggttttttcg gttttagtta 2040
gaggtggggt cgtataattt tttttttttt tcgggttggg gagggttgtc ggttttaatt 2100
ttttttcggg agttcgtagc gtcgggagga aatttgggtt agaggtgaag gaggtggcgt 2160
cgggtgttta ggttcgcggt tttttgggcg aggttttatt tcgtaggagc gcgattttcg 2220
gaggatgtcg gtaagtgggg tcgcggtttt gagttttaga ttagcgaaag tcggtttttt 2280
tttttttttt ttttttattt tttaagcgag tattaaagaa gtttggattt aagatttaag 2340
ttcgttgtgt atgagcgagg gatttgcggg ggggcggggg ggggggaagt gattttgttc 2400
gaattggagg cgagtattat ttttataatt ttttaattag cgatcgagtg gattagtatt 2460
tttttaagtt ttgtttcgga aagaagtagt cgtaattttg ttgttatttt tttttagggt 2520
attggttttt tgaattagtt ttttttttta tttttttaat ttttatttcg ttgcggagaa 2580
aagttttaat aaaaaattag aaaaggtaag gagcgaggag tgaatgttat tgacgttatt 2640
gggtggggaa gggggtttcg ggaaagattt ttggaaattt tttcgtataa ataaaaaata 2700
aaaaggattt ggttgttttt tatgtgttaa tatttgggga ggggagttta attttagaat 2760
gaggtcgagt ttttaaagtt taaggagaga ggggtgagag tgagattttt tcgttttttt 2820
tggattggtt ttaaggaagg tgggggtgtt gttttatttc gtggggagtt ttattttttt 2880
tgttaagtat cggttttggg ggcgaagcgg tttaggggtg tgggggtcga gattgaggtg 2940
cgggcgcggt tgttagaggt gtgtataaaa tcgagataat tcgtaggtcg ttattttaat 3000
tgcggttatt aatacgttcg aggtttattt gttgtttttt aagacgcggg ggggcggcgg 3060
ggattgcgtt ttaggttttg tttttttttt tcgttttttt ataggttttt gttttttttt 3120
atttttcgtt ttttgtggtt agagggagga ttttttaggt cggcgggtgc gttttcgcgg 3180
gggtcgtcgg ggtcgtttta ttcggtagcg gggttgtttg cgtggaggga gtttcgttgg 3240
gggatcgtgg ttcgttagag tttgtttaga gtttttagtt agtattgatg cgttaatatg 3300
aggtgaggta agtattttat tcgtatcgat ttttggtatt tttttgtgtt tattttatta 3360
ggttttcggg gaggtaaaga agtgtttatt tttgggggga agtgttttta gtttttagtt 3420
ttagaattaa gagtttttta ttcggagttt tgatagtttt gatattttta aatttttttt 3480
tttttcggtt aagtagaggc gtagcgtcgg cgcgttggaa agggtgaaga ttggtatttt 3540
ttggagaagg aaagtttggg tagatatttg cgttattttg tagtgtttta aacgtttttt 3600
tttttgtaga ttttgggaag gtgggatttt ttgttcgcgg agttggtaaa cgggtttagt 3660

gagttttaaa ggtaaaggtt tttatttcgg tcgtttagtt tttggtgttt tttttatttt 3720
taggtttttg tttttttttt tttttagtta atttgtcggt tttcgttaat tcggtagttt 3780
cgtcgagttt agagtttaga aaagttaaga agattttaga gtcgtttcgt ggtattttta 3840
ggttgtggtt attgttcggt ttggggggaa gtcgcggtgt tttcgttttt tttcgttttt 3900
tgttaaatgg ttagtgttag gggttttttg ggtttagttt ttagtttttt ttcgttcgtt 3960
ttgggaggtt agtttgtttt tttttttttt tttttttttt tttttttttt tttttttttt 4020
tttttgtttt ttttttgttt atttttaaaa tatttttaaa ataatattgt ttagtggagt 4080
tttttagtaa agggattttt agtagtttag cgggttagat ttttaaggtt ttgtttttta 4140
gtttttttat gttgttgttt agataggagt taggtttaaa atttttgata tgtaggataa 4200
ttgaaattag cggtattttt cgatttttgt aaatagggtt tttagaaaag tttgtttttt 4260
attttattat tttgtgacga tttttttatt tttttttttt ttgtaatttt tgtttttaag 4320
aggtaagagt ttagttttaa aggaaaagta agtaagttaa ggttatttta gttttttttt 4380
tttggatatt ttttttttaa atcgttgttt attttaaagg ttaacgattc ggttttgcga 4440
tttgtttcgt tgtagatttg ggaataggta ggcgggaatt gggggtttta ttgggggatt 4500
tgaggttggg gagggggagg gagtaaatgt tatggttggt tcgtttaagt atttagggaa 4560
tcgaagttaa gcgtattttg acgggttttt aaaatgatat tgattaggat aagttgtttt 4620
taattttagt aagagttaat ttgtttgtta attaaggtat taaggggttt aatgcgagtt 4680
ttattagcga taggagaata gaggcggtag ttagggatta aaagtcggga ttttatatat 4740
ttgttagcgt tatttttaaa aaaaaaaaaa aaagtatttt taatttgaaa ataattcggc 4800
gtttttcgtt attttttaat ttagttttat agagggtgat ttttaaattt ggttagcggg 4860
gaaaatcgtt gttcggggga tagaggggtt gataggaatt gcgggttggt ttagtcgaat 4920
gcggtcgggg agaatttaag aatttttagt tcgcgcggtt cgatgttttt gattttttac 4980
gagaggaaag ggaatgaaaa atgaagtaat aaatgatatt atttaggttg gtagttttcg 5040
ttttcggtta gatttcgttt tttaggttcg gttttggcgt cgggtggcgt ttagtttttg 5100
tacgcgcggc gcggttcgcg ggaaagtttg tgtagcgaga gtgattgttt tttcgtttcg 5160
cgcgcgttgt ttttttttgt ttcggagggg cgttgggttt ttt 5203

<210> 207
<211> 8298
<212> DNA
<213> Artificial Sequence

<220>
<223> chemically treated genomic DNA (Homo sapiens)

<400> 207
   agaaggaaga gaagtgggag aagtattttg gttcgttttg ggtttttttt taagttttag 60
   ttttgtttgt ttttattttt agaattttga gaggtaaaag gtttttagga ttagtttgag 120
   aaggagatga agggagaaat ggaggaaaag aaagtagaga ggaggagaaa aggaggaagg 180
   agaagaagag aaggagaaaa agaggagata taagaaatag ggaggagaag gaagaatgga 240
   ggaaggagaa agagggagag ggagaagagg aggatgagga ggggaggaag gagaatataa 300
   ggaagagaag gaaaagaggt gaagagtgtt ttatgtttgt aattttagta atttgggagt 360
   ttgaggtggt tggattattt gaggttagga gttcgagatt agtttggtta ataatggtga 420
   aatattattt ttattaaaaa tttaaaaatt agttaggtgt ggtggggggt ttttgtaatt 480
   ttagttattc gggaggttga ggtgggaaaa ttgtttgaat ttggaaggta gaggttgcgt 540
   tgagttaaga ttatattatt gtattttaat ttgggtgata gagtgagatt ttgttttaaa 600
   aaaaaaaaaa aaaaaaaaaa aaaaaagagg tgtagaggag gaggaggcga aggaagagga 660
   agaaaataga ggagaaagag aaaatagaaa aggaagggga ggaagaggaa gaaaatagag 720
   gagaaagaga aaatagaaaa ggaagggaag gaagaggaag agaaagggtg aaggaagaga 780
   ggaagaggag gagaaggaga ggaaggaaga agattaggaa gaaaaaaggg aggaggttta 840
   ttcgagagtt tatttagtag taggtttgag agaaatttgg ttattattat tgttgttgta 900
   tttttagtga tgttgttttt gtttagtttt tgagaattgg aatttagtgt atattatttt 960
   atttttttgg ggaaaatgaa aatgttaagt aagttattaa tttttttttt tgtttttatt 1020
   taggtttggg tagattattt taagtttatt tttagaagag tgttattttt taaaagaaaa 1080
   gtataataat aaaaggtcgg tgttttttgg aaatgtagcg ttgaggaatt ttagttttcg 1140
   aaatttggaa ggagattttt gagagttttt tgtttttgga tgtttagttt ttttttttat 1200
   ttttacgttt attttttttt tttttattat taaatatttg aagattgagt aattttattt 1260
   gttgatgttt ttggtttttt ttagtagagt aggttagagt ttttgaaaat gtaaaggttt 1320
   ttaatgaaat aaaaattttt ggtttttaag gtggatttat tattttttaa aggaatattt 1380
   attgggtaat tattttttaa aagtgtttat gatatagggg attgaaataa agagcgtttt 1440
   ttttttcggg ttgagggaag aggttgttgg ttatttggat gtagggagga gggagttttt 1500
   ttgtggttta tgttttggga gggagtagtt agagataggt taagtttttt tagttaaatt 1560
   taagttgggg ttttgttttt gtattttggg tttatttatt tttttttcgt ttttaaattt 1620
   aaattgggtt tggaagatgt tatttggaaa tttgggaaga agaattttta ttagtattgt 1680
   tgttgttttt aagaaaaggg aaaaggagga atgtagagat tttagattag acggggtgag 1740
   ttagagatag gagataatta tttaagttag attatattga gacgggaatg atttttggcg 1800
   gattagtggg ttgggttggg tcgggtcggg agatgtattt taagattaag tagagaagga 1860
   ggattttttt tttaaggttt ttagttaatt ttatttggat taaaggattt ttttagggat 1920
   ttaggtttga gttatagttt agagagtaat ggaggtttat atatatttta ggaaattatg 1980
   gagatggggg aggcggtgat aagatagata gttttagaga aaagtcgttg gggtgaagtt 2040
   agaaaggtaa ggaggaaaag agaaagagag attaaaagaa taaaatagat ggaggagatt 2100
   ataagataga ttgaggggga aagagagata ggaaaaatgt ttgtgtgtga gagagagata 2160
   gagagaaaga gggtgaaaga gggtgaggag agatagggat agaaagaggg ggttaagggt 2220
   taaaggaaaa ttttttggaa taagtttggt ttagtttata tatttagaag tttttatatt 2280
   tttttagtag taattggagg ttttaggagt tgattaagtt tgtatggggg tgattttttt 2340
   tagataaggg ggtaagtaaa gggaaataag aggaggggac gcggtttttt tttattttta 2400
   gttatagttt ttaggtgaat tttttttttt tttgttggat gtgaaatcgt tattttattt 2460
   tattatattt tttaattgtt gtaaaaatta tttgtgaaat tggtttttaa taggtaagaa 2520
   cggttacgtt tattttaata gataatttta ttttaatttt tttttttgta agggtttatg 2580
   gaatttcgga gttgtagaaa ttttgggggt ttgtgtcgtt attgtattta ttattgtgag 2640
   ttttttgtag ggagggtgag ggattatatt gtgggtaagg tggttttagt gtaggatttt 2700
   tgaggttttg agggtttttg gtagtagaag tgtttagtta tgtattcgtt ttatttagtt 2760
   taagatgtta tatgagtatt ggttagaaga gagggagttt tttatttttg gtatattttg 2820
   gaggtttttt tttatttgag aattgattta aatatatatt tatatatttt tgacgcgcgt 2880
   ttagagttat attaaggtaa tttggaggtg attttaaagt agaaatatag atatggattt 2940
   ttatttagaa atatatattt atttaaattg attttaaata gatttatata taaaatttat 3000
   ttgtattttg atatatagat tcgggtatag gtttatattt atacgtaagt atattggttt 3060
   attttataaa tatagggaag aagtgttttg aaatttttta ggtagaggat agttagtgac 3120
   gaatgaatgg atgaatatgt aatatattta atatatatgt gtgtgtgtta tacgtgtgta 3180
   tgtataagtg tatatgatat aaatatatgt atatatatat atagagaata tatatttatt 3240
   tatggaggtt aaagtaggtt aggtttttag taaggttggt gatgtaaggg ggttgttgtt 3300
   ttttttgggt atatatgtta tggtttaaag tttgattttt taattttttt cggggagggg 3360
   ttttataatt tgggttaggt aaagtatttc ggtgtttttt ttttaattcg aaagttattt 3420
   ggtatttgtt tttttagtta gagagagtat gagtttaata agttttttat tagggttttt 3480
   atttttagaa ggttttttta aaatttgatg agttttttgt gttaggagag tggggagagg 3540
   agggagattt gttatatgtt gtaaggggta aaggttatag gtgaggcgaa ggggagggat 3600
   ggggatattt tggggttagt tagcggtgag cgttttaggt taagataagg ttgattttat 3660
   tgtcgagaga gggcgggtag ggggtgagat tgagggttgg gtttatttta ttgtattttg 3720
   tttttagcgt ggggttagag acgttttttt tttcgggaga taaaaggttt gtagttgtgg 3780
   cgttttgttt cgttgggagg gcgtttcgtt tttaggattc ggtgtttcga ggttgaggtt 3840
   attggttatt ttggttttag aaagcgaaga atttaggaat ttttgattat tttattttga 3900
   tttttgatta tttagttggg gcgttttttt aaatttatta gtaaggagtt tagagttggt 3960
   gagtttagag ttgtattaag ttttgacggg tttttttttt tttatttttt atttttttgg 4020
   gtttttagat ttttatatta gtattaattt tttgtaaatt taattatttg ttggaaagga 4080
   gttataggtt cggtttgggg gtttgaggag gacggcggcg gttggagacg tttgtacggg 4140
   gcggaattgg aaggggagga agaaatttgt gtttagtatt tggtagattt attttgttaa 4200
   gtttcggaat tagatacggt tattgggggt tttttagagc ggtaggaagg tagagtaaga 4260
   atcggatatc ggggttttga tattatatag aaaaggttag aatttttttt tttaagttat 4320
   tttaggtcgg ttttcgggga gttttattgg aggaatttta gagggtgtaa gacgagggag 4380
   aagatgagaa ataggcggga ttttggtttt cggaggttag agaaaaggcg agattttaga 4440
   gaggcgtcgg attcgaggta gatattttta tatagtcggg atttcgagag tagtcgtggg 4500
   cgatttgggg gtcggggatt cggcggattc ggtttattta ttaaaggaag gtattgggat 4560
   aaagttggtt tttttttttt tttttttagt ttttaattcg tcgggatcgg tttttttttt 4620
   ttttacggcg gtttaaattt ttttttaaaa cgaggtcgag tcggggatta gaaaagaggg 4680
   aaaaaaatat tcgttggcga ttttttttcg ttattggttt ttcgtttttt tgcgttttgg 4740
   tttttttttt tttttatgaa taaaagaaaa gttcgaattt attagatcgc gtttattgtt 4800
   tttttttttt tttttttttt ttttttttcg tatttcggtg aatatttttg tataatttat 4860
   ttagttgatt attcgcgttt tttcgttttt ttattttttt ttttttaatt tcgttatttt 4920
   ttgttttttt cgtttagttt tgaaattcga ggaggttttt tttttcgttt gttttcgggt 4980
   tttttagggg acggggttgt gaaagttggt gtggagggag aagcgagtgt ggttcggaga 5040
   aagaaggcgt ggagaagagg gagggagcga gagcgagaga ataaatatat aaataaatac 5100
   gagaacgaaa tttatttcgt agttttcggg ttcggaaatt ttggtttcga gcgttagagc 5160
   gttagagcgc gagagcgcgg cgttcgttat tttgaggttg gcggtttcga tttcggtcgc 5220
   gttttttcgg ttttttttcg tcgcggggtt tggttttcgg gttttgttag gcgtattagt 5280
   tcgtataatt tttggtcgag gttagtcggt agaggcggat ttggggttgg agtgtttgtt 5340
   tgtttgaatt ttttcgtcgt cgttattttt ttttttttta atttttattt tattttattt 5400
   ttttttttag tttttggacg cgtttgattg tagttagggg tggggggtgg gggtagggag 5460
   tgtgtgtgga ggggagggag aagaggttaa aaaaaagaag acgaagaaga cggaaagaaa 5520
   gagatcgtag taggggtgaa gggagcggac gggaagcgat ttttgtcgat tttggattcg 5580
   ttttcggcgt gcgtaagaat ggcggttttt ttcggtacgg tatcgagaat gatgcggtcg 5640
   gtttcggggt agaattattt tcgtacggga ttttttttgg aaggtaagaa cgtttaggtt 5700
   ggtttcgtcg cgatttcgtc gttcggaatt cggggttttt ggagaggttg cggtttttag 5760
   gggacggtgg cggcgtcggc gatagtagag ggatttcgtt ttttttgggg ttttagttcg 5820
   ggcgcggaat ttagggagtt tttgggattt atatttgttc gtttttgcgt gggttttatt 5880
   gttcggcggt tttcgtttcg taggtcgtta gttgtcggtt tttattcgta gggttgaaat 5940
   tttggtagat ttggattttt agtaaatatt aggttttggt gaattttagt tttttttatt 6000
   atttttagat attttttttt tgatagtttt tcggtttttt tggttcgttg gttgttagtt 6060
   ttttattttt gttttgaatt atttttttta ttttcgggat tggtttttta gttttttatt 6120
   tcgaattatt tattatgggt gatattttat atgttttaat tattacggtt atttattttt 6180
   ttcgaagtta tagttaaata tttttgtttt ttcgtttgga aataaattaa ttggaaataa 6240
   taattagtaa tgattttttt tttaaaattt tttattcggg ttttcgtatt ttggaaattt 6300
   ttcgggagag aatttttaaa cggcgaggtt tagagaattt ttaaacggcg agaggggtac 6360
   ggtaatgttt aaacgagaag aaagagttga ttttgtaaag cgtttttttt aaatatttag 6420
   cgttcgttcg gggagattcg ataggacggg cggcggcgaa tcgggttttg gttggagaaa 6480
   aggttgggag cgtatagcgg agagattcgt ttttgtgttt ttgttttgtt ttttttttaa 6540
   attaatggaa ggttttaatt tgttattgag ggattatttg gtttttgagg gtaggtagtt 6600
   tagggtgtta tagagaagtt attagaaata tgtgcgattt ttttttttta aattattttt 6660
   tttgtgatcg tttgagtttt tggcgcgtga ggaaggggag aggaggtagg gagggggtgg 6720
   gtgggttggt agtttgcgat tcgggaggag tttttttggg tagcgagacg gcgtcgggtt 6780
   tttgaattag atagaggcga agagagcggt ttcgtgatta agtgcgcgtc ggtagttacg 6840
   taggcgggag agcgcgtaaa ttcggggatt tgtagtggta tttttttgta gcgaatgtaa 6900
   gtaaaatagg cggttgagga cgcgcggcgg attagaataa tatttgttta atatgacgta 6960
   gaatattgag gagagtcgag tgtcggtcgt taaagaggtt tttgaatata aagttgggcg 7020
   ttcgagagtt tttgagcgtt aatggtatat ttcgttacgg agatcgagtg atttcgattt 7080
   tgcgacgtac gatttttgat taaattcgag agtggtttta gatatcgatt tttataaaga 7140
   gaggtggggg gatcgggaga gcgagggagg gggcggtagg aacggtttag tagcggagtt 7200
   ttttttcgtt tttttttttt taattatagt tagttttttt tattttcgcg gttgtgaaat 7260
   cgttttaatt ttttagattt tcgtagtcgt tgagttttta aattgggggg atcgaggtcg 7320
   gattagtgcg ttgggtgttt ttttaatttt ttgtgaggaa attgtgtaga ttttgtatta 7380
   aggtaattag gcgttttttt ttttttagta tatattagtg atgtttgttt tttcggtttt 7440
   taaagttatt tttattcggg gggattgtgt tttatttgtt tattagtttc gtagagtttg 7500
   taggtatagt ttatggattt gtgaggttaa gtcgattatt taatttagga gttttggggt 7560
   agtagtgttt taaaattgtt ttaaaacgaa attaggattt cgagggtggt tagaagtaag 7620
   gagagttatt taagggataa ttaggtgttt ttggaggtag tagttaatag tatatgttat 7680
   tcgtttttcg gttgtgtttt agttgttttt tgggatagtt ttacggtttt agttagagtt 7740
   gtaaatgtag taattagtta ggttttttta gtttttaata aattggatta tttatgatta 7800
   ttaggagcgt agaattgagt agatgagtta tattatattc gttattggtt tttttaagac 7860
   gtaggtaggt ttgtagttta agtttcggtt tgagggatag atagttattg gttttaagat 7920
   ttttaaggtt ttattagttt ggtggttttg tttgtagttt tatttttatt tttttttttt 7980
   ttttttttag tagtattcga attttttagg tgtgttttta tttttcgtag tgtggtagga 8040
   gttgggggat atagtatttg ggagattttt tgtagttgtg atttttttat ttatttttgt 8100
   aggagatttt ttagatttat gatagttgtt ttttaaatta tttatttatc gaagttttag 8160
   tgtgaggatt atcgggattg ttgtttgagg ttttggggtt taaataaata aaattggagt 8220
   tagggagtgt atttagtgtt tgttttttat ttttattttg tatttttttt tttttgtatt 8280
   tttttttttt tttttagt 8298

<210> 208
<211> 8298
<212> DNA
<213> Artificial Sequence

<220>
<223> chemically treated genomic DNA (Homo sapiens)

<400> 208

attggagaag ggaggggaga tgtagaagga gagaggtgta gggtgaggat ggagagtaga 60
tattgagtat attttttgat tttagttttg tttgtttgag ttttaagatt ttagatagta 120
atttcggtgg tttttatatt ggggtttcgg taggtaggta gtttgagaaa taattgttat 180
aaatttagga aattttttgt agagatggat agaggagtta tagttgtaag agttttttta 240
gatgttgtgt tttttaattt ttgttatatt acgagggatg aggatatatt tgggggattc 300
gaatgttgtt aagaagggga gggagagaga tgaagatgag attgtagata gagttattag 360
gttggtagag ttttagagat tttgggatta atgattattt gttttttagg tcgaagtttg 420
ggttgtaggt ttgtttgcgt tttggagagg ttaataacgg gtgtgatgtg gtttatttgt 480
ttagttttac gtttttggta gttatgaatg atttagtttg ttgggggtta gaaaggtttg 540
gttgattatt atatttatag ttttggttag agtcgtggaa ttgttttagg gaatagttga 600
ggtatagtcg agggacgggt gatatatgtt gttggttgtt gtttttaagg gtatttggtt 660

gttttttggg tgattttttt tgtttttaat tattttcgaa gttttaattt cgttttggga 720
taattttagg atattattgt tttaaagttt ttaaattaaa tagtcgattt ggttttataa 780
atttatgaat tgtgtttgta ggttttgcga ggttggtagg tagatgggat atagtttttt 840
cgggtggggg tgattttggg aatcgggaag ataggtatta ttggtgtgtg ttggggaggg 900
gagaacgttt ggttgtttta atgtaaagtt tatataattt ttttatagaa gattgggggg 960
atatttagcg tattaattcg gtttcgattt ttttaatttg gggatttagc ggttgcggag 1020
atttggaaga ttagaacgat tttatagtcg cgggagtgga gggagttgat tgtaattgaa 1080
agggaggagg cgagaagagg tttcgttgtt gggtcgtttt tatcgttttt tttttcgttt 1140
tttcggtttt tttatttttt tttgtaaaag tcggtgtttg aagttatttt cggatttaat 1200
tagaaatcgt gcgtcgtaaa gtcggagtta ttcggttttc gtggcgggat atgttattag 1260
cgtttaagag ttttcgagcg tttaatttta tatttaagag tttttttagc gatcggtatt 1320
cggttttttt tagtattttg cgttatgttg ggtaaatatt gttttaattc gtcgcgcgtt 1380
tttagtcgtt tgttttattt gtattcgtta taggagagtg ttattgtaaa ttttcgggtt 1440
tgcgcgtttt ttcgtttgcg tggttgtcgg cgcgtatttg attacggagt cgtttttttc 1500
gtttttgttt aatttaggga ttcggcgtcg tttcgttgtt taagggagtt tttttcggat 1560
cgtaggttgt tagtttattt attttttttt tgtttttttt tttttttttt acgcgttagg 1620
gatttaaacg attataggaa gagtaattta aaaaaaaaaa gtcgtatata tttttaatga 1680
tttttttgtg atatttaggg ttatttattt ttagaagtta aatgattttt tagtgatagg 1740
ttgaggtttt ttattagttt aaaaagaaaa tagaataaaa atataggagc gggttttttc 1800
gttgtgcgtt tttagttttt tttttagtta ggattcggtt cgtcgtcgtt cgttttatcg 1860
ggttttttcg ggcgggcgtt ggatgtttag gaaggacgtt ttataaaatt agtttttttt 1920
tttcgtttgg atattgtcgt gtttttttcg tcgtttagag gttttttaaa tttcgtcgtt 1980
tagaggtttt ttttcgggag atttttagag tgcggaagtt cgagtaaaaa gttttagggg 2040
gaaaattatt attaattatt atttttaatt agtttgtttt taggcggaaa agtaggagtg 2100
tttgattgtg gtttcggaag aaatggatag tcgtggtagt tgaggtatat aaagtattat 2160
ttatggtagg tggttcgaaa taaaggattg gaagattaat ttcgggggtg gaaaaaataa 2220
tttaaaataa gagtagaaag ttggtagtta gcgagttagg agagtcgaga aattgttagg 2280
gaagaagtgt ttggaaatag tggaaagagt tgaaatttat taggatttga tatttattag 2340
gagtttaaat ttattagaat tttagttttg cgggtaaaga tcggtagtta gcgatttgcg 2400
aaacgaaggt cgtcgggtag tgaaatttac gtagaagcgg ataagtatgg gttttagaaa 2460
ttttttgggt ttcgcgttcg gattgggatt tagaagagaa cgggattttt ttgttatcgt 2520
cggcgtcgtt atcgtttttt ggagatcgta gtttttttaa ggatttcgag tttcgggcgg 2580
cggagtcgcg gcgaggttag tttgggcgtt tttatttttt aaagggaatt tcgtgcgggg 2640
gtagttttgt ttcggagtcg gtcgtattat tttcggtatc gtgtcgggaa gggtcgttat 2700
ttttgcgtac gtcggggacg aatttaaagt cggtaaaaat cgtttttcgt tcgttttttt 2760
tatttttgtt gcgatttttt ttttttcgtt tttttcgttt tttttttttt aatttttttt 2820
tttttttttt tatatatatt ttttattttt attttttatt tttggttgta gttaaacgcg 2880
tttagaagtt ggggaggggg gtgaggtggg gtggaggttg gggggaggga aggtggcgac 2940
gacgaggaag tttaaataaa taaatatttt aattttaagt tcgtttttgt cggttggttt 3000
cggttagaag ttgtgcgggt tgatgcgttt ggtagaattc ggagattagg tttcgcggcg 3060
gaggggggtc gggggaacgc ggtcggaatc gaggtcgtta gttttagagt ggcgagcgtc 3120
gcgttttcgc gttttggcgt tttggcgttc ggggttaaag ttttcgagtt cggagattgc 3180
ggagtggatt tcgttttcgt atttatttat atatttattt tttcgttttc gttttttttt 3240
ttttttttac gttttttttt ttcggattat attcgttttt ttttttatat tagtttttat 3300
agtttcgttt tttaggagat tcggggatag acggaagaag gagttttttc gggttttagg 3360
gttggacgga ggagataggg ggtgacgggg ttggggaaag agaaatggaa aagcgagggg 3420
gcgcgagtga ttagttgggt aagttgtgta aaagtgttta tcgaagtacg aggaggagga 3480
ggaggaggag gaggaggagg tagtgagcgc gatttgatag gttcggattt tttttttatt 3540
tatgaggagg gaagggggtt agggcgtaag ggagcggagg attaataacg aaaagaaatc 3600
gttaacgaat attttttttt ttttttttta attttcggtt cgatttcgtt ttgggggaga 3660
atttgaatcg tcgtgaaaag agagggagtc gatttcggcg agttgggggt tgggggagaa 3720
ggggaggggg ttaattttgt tttagtgttt ttttttgatg aatgggtcgg attcgtcgag 3780
ttttcggttt ttaggtcgtt tacggttgtt ttcggagttt cggttgtatg ggaatgtttg 3840
tttcgggttc ggcgtttttt tggagtttcg tttttttttt ggttttcgag ggttagagtt 3900
cgttttattt tttatttttt ttttcgtttt atattttttg aaattttttt agtgaggttt 3960
ttcgggaatc ggtttagggt gatttgaagg gaggaatttt gatttttttt gtgtaatgtt 4020
agagtttcgg tgttcggttt ttattttatt tttttgtcgt tttgagaagt ttttagtaat 4080
cgtatttggt ttcgaggttt ggtaaagtgg gtttgttaag tgttaggtat aggttttttt 4140
tttttttttt agtttcgttt cgtgtagacg tttttagtcg tcgtcgtttt ttttaggttt 4200
ttaggtcggg tttataattt ttttttagta ggtagttggg tttataggga gttggtgttg 4260
gtgtgaggat ttgggggttt aaagaggtgg gaaatgggag ggagggagtt cgttaagatt 4320
tgatatagtt ttgaatttat taattttgag ttttttgttg gtgggtttga ggggacgttt 4380
taattgaatg attaggagtt agggtggggt gattagaggt ttttgaattt ttcgtttttt 4440
gaagttaggg tgattagtag ttttaatttc gggatatcga gttttggggg cgaaacgttt 4500
ttttagcggg gtaggacgtt ataattgtag gttttttgtt tttcggggga aagagcgttt 4560
ttggttttac gttggaggta aaatataata aaatgaattt agtttttaat tttatttttt 4620

gttcgttttt tttcggtagt ggggttagtt ttgttttagt ttggggcgtt tatcgttggt 4680
tggttttagg gtgtttttat tttttttttt cgttttattt gtgatttttg ttttttgtag 4740
tatgtgatag attttttttt tttttttatt tttttagtat aaggggttta ttagattttg 4800
gggggggtttt ttgggaatgg gggttttgat gaaaaatttg ttgagtttat atttttttta 4860
attgagaaga tagatgttaa gtagttttcg aattggaagg gggatatcgg ggtattttgt 4920
ttggtttaag ttatggggtt ttttttcggg agagattggg agattaagtt ttaagttata 4980
gtatgtgtgt ttagggaaga tagtaatttt tttatattat tagttttgtt ggggatttgg 5040
tttgttttga tttttatagg tggatatata ttttttgtgt gtatatatat atgtatttgt 5100
attatatata tttatgtata tatacgtata atatatatat atatatatta ggtatattgt 5160
atatttattt atttattcgt tattagttgt tttttgtttg gaaggtttta aggtattttt 5220
tttttgtatt tgtggaatgg gttagtgtgt ttgcgtgtga atataggttt gtgttcgggt 5280
ttgtgtgtta aggtgtaggt gggttttgtg tgtaggtttg tttggggtta gtttgagtaa 5340
atgtgtgttt ttaagtgagg gtttatgttt gtatttttgt tttgaggtta tttttaggtt 5400
gttttggtat ggttttaagc gcgcgttagg aatgtgtgag tgtgtgttta ggttaatttt 5460
taagtgagaa aagattttta gggtgtgtta agggtgggag gttttttttt ttttggttag 5520
tgtttatgta gtattttgag ttgagtgggg cgagtgtata gttggatatt tttgttgtta 5580
gggattttta gaattttagg agttttgtat tggggttatt ttgtttatag tgtggttttt 5640
tatttttttt gtaaaagatt tatagtagtg ggtgtagtag cgatataggt ttttagggtt 5700
tttgtagttt cgggatttta tgggtttttg tagagggaag ggttgaggtg gggttgttta 5760
ttggaatgag cgtggtcgtt tttgtttgtt ggagattaat tttataaatg atttttgtaa 5820
taattaagga gtgtggtggg gtgaaatggc gattttatat ttagtagggg aaaggggggt 5880
ttatttggga attgtggttg gggatggggg agagtcgcgt tttttttttt tgtttttttt 5940
tgtttatttt tttgtttaga aaaagttatt tttatgtagg tttaattaat ttttaagatt 6000
tttagttgtt gttgggaggg tgtgagggtt tttgagtgtg tgggttgggt tagatttgtt 6060
ttagaaagtt tttttttagt ttttgatttt ttttttttat ttttgttttt ttttattttt 6120
ttttattttt tttttttttg tttttttttt atatataaat attttttttg tttttttttt 6180
ttttttagtt tattttatgg ttttttttat ttgttttgtt tttttggttt tttttttttt 6240
tttttttttt gtttttttgg ttttatttta gcggtttttt tttgggattg tttgttttgt 6300
tatcgttttt tttattttta tggttttttg aggtatgtat gggtttttat tgttttttgg 6360
gttgtaattt agatttgaat ttttgggaag attttttggt ttagatgagg ttggttaaag 6420
attttgggga agagattttt tttttttgtt tggttttggg gtgtattttt cggttcggtt 6480
tagtttagtt tattaattcg ttaggaatta ttttcgtttt agtgtggttt ggtttggatg 6540
attgtttttt atttttagtt tatttcgttt ggtttgaaat ttttgtattt tttttttttt 6600
ttttttttaa aggtaataat aatgttaata ggaatttttt tttttaagtt tttaagtggt 6660
attttttaaa tttaatttgg gtttggaaac gggagagaaa taagtaggtt taaggtatag 6720
aagtaagatt ttagtttgag tttagttggg gaaatttggt ttgtttttgg ttgttttttt 6780
ttaagatata ggttataaag aggttttttt ttttttgtat ttaagtggtt aatagttttt 6840
ttttttagtt cgagggagag gacgtttttt attttagttt tttgtgttat gagtattttt 6900
ggaaagtgat tatttaataa atattttttt aaagggtgat aaatttattt tgaaaattag 6960
gggtttttat tttattgggg gtttttgtat ttttagaggt tttggtttgt tttgttagag 7020
gaggttaaag atattaataa atggaattat ttagttttta aatgtttgat aatgagagag 7080
gagggggtgg gcgtgggggt ggggagggaa attgaatatt taggaataaa ggatttttag 7140
gagttttttt ttaggtttcg gaggttggaa ttttttagcg ttatattttt aagaggtatc 7200
gattttttat tattatgttt tttttttaag aagtggtatt tttttagggg tgagtttgaa 7260
ataatttgtt taaatttgaa tagaaataaa aaggaaagtt gatagtttgt ttgatatttt 7320
tatttttttt agaaaaatgg aatagtatgt attggatttt agtttttaag aattgggtaa 7380
gaataatatt attaaagata taataatagt aataataatt aggttttttt taggtttatt 7440
gttaagtggg ttttcgagtg agtttttttt tttttttttt tttggttttt tttttttttt 7500
tttttttttt tttttttttt ttttttttta tttttttttt tttttttttt tttttttttt 7560
ttttgttttt tttttttttt ttgttttttt tttttttttt tttttttttt tttgtttttt 7620
tttttttttt tgtttttttt tttttttttc gttttttttt tttttatatt tttttttttt 7680
tttttttttt tttttttttt tgagataggg ttttattttg ttatttaggt tgaagtgtag 7740
tggtgtgatt ttggtttagc gtagtttttg ttttttaggt ttaagtaatt tttttatttt 7800
agtttttcga gtagttggga ttatagggat tttttattat atttggttaa tttttggatt 7860
tttagtagag atggtgtttt attattgttg gttaggttgg tttcgaattt ttgattttag 7920
gtgatttaat tattttagat ttttaaattg ttgggattat aggtatgggg tattttttat 7980
tttttttttt tttttttttt gtgttttttt tttttttttt tttatttttt tttttttttt 8040
tttttttttt tttttttttt tatttttttt tttttttttt gttttttatg tttttttttt 8100
tttttttttt tttttttttt tttttttttt tttttttttt tttgtttttt ttttttttat 8160
tttttttttt attttttttt taggttggtt ttgggagttt tttatttttt aaggttttgg 8220
aaataaagat aagtaaggtt aaagtttaga gagagattta agacgggtta aggtgttttt 8280
tttatttttt tttttttt 8298

<210> 209
<211> 6081
<212> DNA

<213> Artificial Sequence

<220>
<223> chemically treated genomic DNA (Homo sapiens)

<400> 209
   ttgggtttta gaaatttttt ttttgagggg aaggggtagg tgtggagagt agtaaaaatt 60
   aggttgaagt gagtagaatt tataatattt aaggtaaagt ttgagttatt ggtgttttat 120
   ttggttattt agttggttgt ttgttagttg ggtggttttg ttttttattt atttttttta 180
   gaatttagtt tttttatttg taagatgggt tgttaaaagg gtttagttta ttttttttaa 240
   tttaaaaaaa aaaaaaaaaa aaattatgat tattagagaa aaaaattgag ttgtgtacgt 300
   taattttttt attttttttt gttatttttt tttttgtgtt tttaggaagt ttttttttta 360
   tttatttgta agtattaggg ttttttttta gaagtttagg taattgttgt taaaggttgt 420
   ggggtgggta aagagtttaa gggataagtg tttaggatat atttttattt aaattaattg 480
   gataaatgtt attagtggtt ttttttaaat tgttttcggt agtttaatta gtggtttttt 540
   tttttttatt tgttaggatt tatattttta tttttgagtt tttaaagttt agggaggtgg 600
   agaatttgga ataattaatg attgattttt aggtattgtt attaggaggt tttagggtag 660
   gttgtcggtt attttttgag tgtagggata gggttgaaat tttacgtggt ttcggtagga 720
   ttgggtaagt ttgtgagggg agagggtgga gcgaaaattg ttagtgtttt gaatattttt 780
   gttttttgta aaatgatgaa gtcgttgttg gaagttttgt tggggtgttg gttttttgtt 840
   attgattttt tttttttttt gggttaacga atgttaattg ttgtttttat agatattttt 900
   gttttattgt tatttttgta tttacgtttg tatttatagg tacgggttta ttggtttttt 960
   tttttttaag gtttttttgg gaaaggatta aaggaatcga tttttatagt aaaattttag 1020
   ggatatttta attttgtttt ttttggatgt atttttttgt ttttgttgtt taataataat 1080
   taatttttgt atagaggtgt agtttttaaa gggtttttat atttataatt ttattcgttt 1140
   ttataataaa gttacgaggt gggtttgatt ttttttagtt tttcgtttat atttatatta 1200
   tgtagaaggg gaaattgagg ttcgggtaga agaaattatt ttattttata taattagggt 1260
   tggaatattt atttttgatg taaagtagag agttgttgtt ttttttttga agggatgtat 1320
   tttagtttta ataatggttc gttacgtagg ggaagtggta tggtggtaag taagttagga 1380
   ggattgttgg gagtagtggt tttttttcga ggttggtttt tgggtattgt gtttagagaa 1440
   attagggtat ataggagtaa gaggaagagt ttatggattt attggagttt aagggtttta 1500
   gttatttgag ttagtaggta gaatgattta agtgtgtaaa taattattta tttaatagtt 1560
   agttgaaata cggtagttta taaaagattt ttaaattatt ttatttttat attttaatta 1620
   gttaggatga ttaatttatt ttgatttgtt cgggaaagtt ttagttttaa ttcgtattta 1680
   gaaaatattg ttggttttcg tttagggtaa attagggtat tttattattt tattattagt 1740
   ttagtgtatt atagttgagt ttagaaaaga aagaatattt tttgaaggtt tagtagatta 1800
   gtatagaatt aaataaattg tgggatatta tgtaaattta aaaattattg tagttaggta 1860
   attatttagg attgttggaa tgttaaattt gaaagaatgt ttatttttat aaagaaacgt 1920
   ttagatttgc ggttagattt tttgtatttt taaaggtaag gtagagttag tttttcgttt 1980
   ttatttttgt attttgatat tggtaatgaa ttatattttt ttaaaaaaat attgagttta 2040
   aaaaaattat aattagttgt tagttgataa tattgttata aaagttttat gggttttaag 2100
   tttgagaaga aaagttggta aatgaggtat ttattttaaa aagtgagttt taaggtttgg 2160
   ttaggtatag tggtttatgt ttgtgatttt agtattttgg gaggttgagg taggcggatt 2220
   atttgaggtt tggagttcga gattagtttg gttaatgtgg tgaaatttcg tttttattaa 2280
   aatataaaaa attagttagg tatgatgatg tatatttgta gttttagtta gtcgggaggt 2340
   tgaggtagga gaattatttg aatttaggag gtggaggttg tagtgagtcg agattatgtt 2400
   atcgtatttt agtttgggtg atagagtgag attaagttta gaagaaaaaa aaaagagttt 2460
   taaaagtttt attgaaagtt tattagttat agtgtaaatt tattttaaaa ttttaaatat 2520
   tattatagga aggtatatga tttttatttt taattaaaag tattatggat tttaagtttg 2580
   aaaaaatatt gtgataatag gtgattttaa aaatatatgt attttaaatt tttcgttttg 2640
   tagaggagga gattgatgtt aaagagatag agttaaagta ttagttaatg atattttttt 2700
   ggttttggtt ttagagaatt aggaagtatt aaggaattta ggtttgattt ttgattttaa 2760
   ttagaagtag ggttagttta ggaggtttgt gtttattttt gtagtagaag tttattgtat 2820
   ttttattttt tgttcgtttt agattttttt tgttgagttt tttggttatt tgttatgtta 2880
   tttgtagttt ggagaagagg tattgttaag gtgtggggtg tgtttttttt tgaggagtaa 2940
   ttttgaatag tgtatattgt ttttattttg aaatgaaatt ttaaagataa atttatatat 3000
   gggataggaa tgttttgata atttgttgga tttgtttttt aagaaatttt gtttaaaaag 3060
   gggataggaa agggagaggg tttattgttt gggtagaaaa gatgtgtata ggtaagaatg 3120
   attgtatgag agttaatggt tattggtacg tattagaatt ttagattggg tagaattttt 3180
   aaggttgtta gagttgtttg aaatgagggc gtattagatt tattttggag tattgttttt 3240
   ttggtgggag ggaattttgg gtggataaag agagtttttg ttgtggttgt tatattttat 3300
   attttttttg tagtaggata taatttaggg tagtaaattg gtttatgggg ttcggaggat 3360
   ttatgttaag atttattttt attttaatat ttttggattg tttttttttg ggagggattg 3420
   ttagagaagt tagggatttg gagttttgaa aattatagag atatttaaaa ttataatttt 3480
   agagaaagat ttaaaatgat gaagtattaa ttatttttgt ttatagtttt tttttgtaaa 3540
   tgtggaggaa atatgtaggg atatatattt tttttttttt ttttttataa gatgatttat 3600
   tttttttaaa ttgagaaatg agaaagattg ggtggaagga ggttgtggat tgatatgaaa 3660
   gagaaaattt ttttaattta gaaaaatagt ttatgtgttt ttatatacgt gtatatatgt 3720
   gtatatatat atatatatat atatatatat atatatttaa tttttttgaa tattttagtt 3780
   ttttttgtat tttttttgtt ttttttttat tggtttttta gttttttgtt ttagaataat 3840
   tttattaggt ttgtagagtt attattgaaa agggtatggt tgttttttag attgtaattt 3900
   tgagtttaga ggtttggtta aagattggag gagtatttag gttttgattt ttaggtttgt 3960
   tagtgtttta ggatatttgt ttatttatat ttatttataa ttttacgtgt atttcggttt 4020
   ttcgattgag agaattatga ggaaggaggg ggtagttttt gttattatta aggtaaagag 4080
   gtgttaggta gttttgttcg gttttagggt ttattaattt tgttattatt tttattagtg 4140
   gatttttttt taattatatt taggttttta tattgttgag gttattgatt tttatagatt 4200
   aggttagagg ttagtttagt tttgtttttt tttaaggttt ttttatatag agatgaagtt 4260
   taggttttta atgttttttt tttgaatttt ttttttaagt ttaaaatttt taaggagtaa 4320
   gaaagagggt aggtggttgc gtgtgtgttt tagaggagag aggcgaggag ttgttgtgga 4380
   atgggttatg tgataatttt gttagtttga taatgtaagg gtgtgaacgt tatttgtgta 4440
   gaatagaggg aatggtttta ggtgttgttt aggatttttt tttggaagag gttatagttg 4500
   tggtgagaag gtaatagtat tttgtttata gtttcgaaag taggttttat ttaaagaatt 4560
   aggtataaaa tagaagtttt tttttttttt ttataaaaga aaaggttgtt ttttggtgag 4620
   taaattaggg gagatagggg gtaaggaggg tattttatag agattatttt ttttattttt 4680
   ggtggttagt gatttaggag gatttagaga attttatttt taggttttta ggtttttgtt 4740
   ttttattttt ttaatatttt taggtttgtt gagttagggt ttagttgttt tttttttttt 4800
   ttcgttggaa atagaagttt ttagcgaatg tttttaattt ttgagtttat ttagttatgt 4860
   tttttttgta aaattttttt tttttcgtat ttttttgtat ttagttagtt aattcgcggt 4920
   ttacgagttt cgttagattt attttttagg gttttagcgg gataaaatcg gtcggtgatg 4980
   tcgggtggat gggaataaat tttagaagga ggagagatat cgggtttagg gtattttcgc 5040
   gggcggattt aattagtgag ggtttgtagt cggtcggtta ggtatgttat ttaggggtta 5100
   gttggaagtt ggttagtagt gaggacgggg gatggaagaa aggagagggt tttaggatgt 5160
   ttggtagttt tttttttttt aagttaaacg ggataagatt gggatagcgg agggagtggg 5220
   tacggaggtt ggagtttgga gtttttttag cgtattttta atttttgatt ttttcgggaa 5280
   gtttgttagt taggttaggt tgaggttggt tttatttcgt ttaaaaattt ttttattttt 5340
   ggtagacgat gtaggtgtaa aggatgaggt ttggggaggg ggtttgagga tgtaggtatc 5400
   gaattttatc gtattttatg tttttttttt gggtttgtgt agggtagcgg taggcgcggt 5460
   tcggatttac gggaggaagt ttcgagtttt cggcgggttg cgagcgattt ttcggcgatg 5520
   ttttataatt ttattagatt tggtaagaac gcggtgtggt taggattttc gagttttcgc 5580
   gtcgttgagc gttcgtgcgt gcgttcgggt ggttggattt cgagagcgtt agtacgggat 5640
   tcgcgggatt tattcgcgtg gggatttagt tggggcgttt ggggtttggt ttttcgttgg 5700
   agtcgtttcg ttgttttcgg gtattggggt tttattcggt tagttgagtc gtttttgttc 5760
   ggtttcgagg gattatatgg atcggtgagg ggatttttgt ggagttagag gggttttttt 5820
   ttgagtttta tttttagttt tgtatggcgt attagtgtgg atatttttcg tgttttgttt 5880
   ttgggttgta tcggtgttgg atttgtcgtt ttttcgtttt cgatttttaa gttatttttt 5940
   tcgggtgcgc gtagtttttt ttagtgattt ttttggttgg attgtgagtt ttcgtatttt 6000
   taagttacgg taatggagtc ggtggtttcg ggttcggagt cggtagcgat ttaggtgttt 6060
   ttcgattttt tttggtttag t 6081

<210> 210
<211> 6081
<212> DNA
<213> Artificial Sequence

<220>
<223> chemically treated genomic DNA (Homo sapiens)

<400> 210

gttgggttag gaggagtcgg agagtatttg agtcgttgtc ggtttcgggt tcgggattat 60
cggttttatt gtcgtggttt gaggatgcgg gagtttatag tttagttagg ggagttattg 120
ggaaggattg cgcgtattcg aggaagatga tttagaggtc ggggacgggg aaacggtaag 180
tttagtatcg gtgtagttta gggatagagt acgaagaatg tttatattaa tgcgttatgt 240
aaggttggga atggagttta aaggaaattt tttttaattt tatagagatt tttttatcga 300
tttatgtggt ttttcgaaat cgggtaaggg cggtttagtt ggtcgggtgg aattttagtg 360
ttcgagaata gcgaggcggt tttagcggag ggttaggttt taggcgtttt agttgggttt 420
ttacgcgggt gggtttcgcg aatttcgtgt tgacgttttc gagatttaat tattcgagcg 480
tacgtacgga cgtttagcgg cgcgggggtt cggggatttt gattatatcg cgtttttatt 540
agatttgatg gagttgtgag gtatcgtcgg ggagtcgttc gtagttcgtc gagggttcgg 600
ggtttttttt cgtaggttcg ggtcgcgttt gtcgttgttt tgtataaatt taggagaagg 660
gtatgagatg cgatgagatt cgatgtttgt atttttaggt ttttttttta ggttttattt 720

tttatatttg tatcgtttgt taggagtagg aggattttta ggcgaagtag ggttaatttt 780
agtttagttt agttaatagg tttttcggga ggattaagga ttgggagtgc gttgaagaag 840
ttttagattt taattttcgt gtttattttt ttcgttgttt tagttttatt tcgtttaatt 900
tgggagggaa aaggttgtta ggtattttgg gatttttttt ttttttttat ttttcgtttt 960
tattgttagt tagtttttag ttaatttttg ggtgatatat ttggtcggtc ggttgtaggt 1020
ttttattggt tgggttcgtt cgcgagggtg ttttgggttc ggtgtttttt ttttttttga 1080
agtttgtttt tatttattcg gtattatcga tcggttttat ttcgttgagg ttttgggaga 1140
tgggtttggc gaggttcgta ggtcgcggat tggttggttg ggtgtagggg ggtgcgggaa 1200
ggggaggatt tttgtaaggg gatatggttg agtggattta ggagttagaa atattcgttg 1260
ggagtttttg tttttagcgg agggaagagg gaggtagttg agttttaatt tagtaggttt 1320
ggaaatattg gaggagtggg gaatagaggt ttaggagttt gaggatgagg ttttttgggt 1380
ttttttgggt tattgattat tagaagtgag agggatggtt tttgtaggat gttttttttg 1440
ttttttgttt tttttggttt gtttattagg aagtagtttt tttttttgta aaaggaagga 1500
gggaattttt gttttatgtt tgatttttta ggtagagttt gttttcgggg ttgtaggtaa 1560
gatgttatta tttttttatt atagttatga ttttttttag ggaagaattt tgagtagtat 1620
ttgaagttat tttttttgtt ttatatagat agcgtttata tttttgtatt gttaggttaa 1680
taaaattgtt atataattta ttttatagta atttttcgtt tttttttttt ggaatatata 1740
cgtagttatt tgtttttttt tttgtttttt gaggattttg aatttggaag aagagtttag 1800
gggaagggta ttgagggttt agatttattt tttgtgtgga aaggttttag aaaggaatag 1860
gattgagttg atttttggtt tggtttgtag gaattaatag ttttagtaat atgggggttt 1920
gggtgtggtt aagaaggggt ttattggtgg gaatgatggt agggttggtg agttttggga 1980
tcgagtagag ttgtttggta tttttttatt ttagtgatgg taaaagttgt tttttttttt 2040
tttatagttt ttttagtcgg ggaatcgggg tatacgtaag attgtgagtg ggtatgagtg 2100
agtaaatgtt ttggaatatt ggtaggtttg ggggttaagg tttggatgtt tttttaattt 2160
ttggttagat ttttggattt aggattatag tttagaggat agttatgttt tttttagtga 2220
tggttttgta ggtttggtgg ggttgttttg ggataggagg ttaagaagtt agtgggaggg 2280
gggtaaggaa ggtgtagggg aaattgggat atttaggggg attggatgtg tgtgtgtgtg 2340
tgtgtgtgtg tgtgtgtatg tatatatgta tacgtgtgtg aaaatatatg aattattttt 2400
ttgaattaaa aaagtttttt tttttatgtt aatttataat ttttttttat ttagtttttt 2460
ttatttttta atttaggaga aatgaattat tttgtgaaaa aagagagaag aagatgtgta 2520
tttttatatg ttttttttat atttgtaaag aggggttgta aataaaaata gttggtgttt 2580
tattatttta aatttttttt taaaattgta attttaaata tttttgtggt ttttaaagtt 2640
ttagattttt aatttttttg gtagtttttt ttagagagag ataatttaag ggtgttggga 2700
tggaaatgga ttttggtata ggtttttcgg attttatggg ttagtttgtt gttttggatt 2760
gtgttttgtt gtaggggagg tgtggagtgt ggtagttata gtagaaattt tttttgttta 2820
tttaggattt ttttttatta gggaggtaat gttttagggt gaatttaata cgtttttatt 2880
ttaaatagtt ttgataattt tagagatttt atttaattta aggttttgat acgtattaat 2940
aattattgat ttttatgtaa ttatttttgt ttgtgtatat tttttttgtt taagtaatga 3000
attttttttt ttttttattt tttttttggg taaaattttt tagaggataa atttagtaag 3060
ttattagaat atttttgttt tatgtataaa tttattttta aaattttatt ttaaggtaaa 3120
aataatgtgt attgtttaag gttatttttt agggaaaaat atattttata ttttgatagt 3180
attttttttt tagattgtag gtgatatagt aggtgattag aagatttagt agagaagatt 3240
taggacgagt aagaagtgag aatgtagtga atttttgttg tagagataaa tataggtttt 3300
ttggattgat tttgttttta attagaatta gaaattaggt ttgggttttt tggtattttt 3360
taattttttg agattaaggt taaggaaatg ttattaattg gtgttttaat tttgtttttt 3420
tggtattagt tttttttttt gtaaaacgaa aggtttgaaa tatatatatt tttaaaatta 3480
tttattatta tagtattttt ttaaatttaa aatttatgat gtttttagtt aagaataaaa 3540
attatatgtt tttttatgat gatatttgaa attttaaaat aaatttgtat tgtgattaat 3600
agatttttaa tgggattttt agaatttttt tttttttttt ttagatttag ttttattttg 3660
ttatttaggt tggagtgcgg tggtatgatt tcggtttatt ataattttta ttttttgggt 3720
ttaagtgatt tttttgtttt agtttttcga ttagttgggg ttataggtat gtattattat 3780
gtttgattaa ttttttatat tttggtagag acggggtttt attatattgg ttaggttggt 3840
ttcgaatttt agattttaag tgattcgttt gttttagttt tttaaagtgt tgggattata 3900
ggtatgagtt attgtgtttg gttaggtttt agaatttatt ttttaaaatg aatattttat 3960
ttgttaattt ttttttttag gtttgaggtt tataaggttt ttgtggtaat attattaatt 4020
ggtaattagt tataattttt ttaaatttag tgtttttttg agaaaatgta atttattgtt 4080
aatattaaaa tatagaagta gagacgagag attaattttg ttttgttttt gaaaatgtaa 4140
gagatttggt cgtagatttg agcgtttttt tgtggaaatg aatatttttt taggtttaat 4200
attttaataa ttttaggtaa ttgtttaatt atagtggttt ttgaatttgt ataatatttt 4260
ataatttgtt taattttatg ttgatttgtt gagtttttaa aagatgtttt tttttttttg 4320
gatttagttg taatgtattg ggttggtggt agggtaataa agtgttttgg tttgttttgg 4380
acgaaaatta atagtgtttt ttagatgcga gttaaaattg ggattttttc gggtaaatta 4440
ggatgaattg gttattttag ttgattagag tgtgggagtg ggatggtttg ggggtttttt 4500
ataagttgtc gtgttttaat tgattattgg gtgggtgatt gtttgtatat ttgggttatt 4560
ttatttgttg gtttaggtga ttgagatttt tgaattttag taggtttatg agtttttttt 4620
tttattttta tgtattttgg tttttttggg tataatgttt agagattagt ttcgagggaa 4680

gattattgtt tttaataatt tttttggttt atttgttatt atgttatttt ttttacgtaa 4740
cgaattattg ttgggattaa agtatatttt tttaggaaga aaatagtagt tttttatttt 4800
gtattagagg tgagtgtttt agttttaatt gtgtagaatg ggatgatttt ttttgttcga 4860
atttagtttt tttttttttgt atgatgtgga tatagacggg gggttgggga gggttagatt 4920
tatttcgtag ttttgttgtg aggacgaata aaattatgaa tgtgaaggtt ttttgaaaat 4980
tatatttttg tataaaagtt aattattatt agataataaa ggtagaggaa tatatttaaa 5040
gagggtaaag ttaaaatgtt tttaaagttt tgttgtggaa gtcggttttt ttaatttttt 5100
tttaaaggag ttttggagag gaaagggtta gtgagttcgt gtttgtgggt gtaggcgtgg 5160
gtgtaagggt agtagtgggg taggggtgtt tgtgagggta gtaattaata ttcgttgatt 5220
tagagagagg ggagagttaa tggtagaagg ttagtatttt agtagaattt ttagtagcga 5280
ttttattatt ttgtagaggg tagaagtgtt tagggtattg gtagttttcg ttttattttt 5340
tttttttata gatttgttta attttgtcga agttacgtgg gattttaatt ttgtttttgt 5400
atttaggaaa tggtcgatag tttgttttgg ggttttttgg tggtagtgtt tgaaaattaa 5460
ttattaatta ttttaggttt tttatttttt taggttttga aaatttagaa atagaaatat 5520
aggttttggt aggtgaaagg aaaagagtta ttggttgggt tgtcgagggt agtttggaag 5580
aaattattgg tggtatttgt ttagttggtt tgggtaggga tgtgttttgg gtatttgttt 5640
tttgggtttt ttgtttattt tatagttttt ggtagtagtt gtttgggttt ttggaaggag 5700
attttggtat ttataaatgg gtggaaaaaa aattttttgg ggatataaaa aggaaggtga 5760
tagagggaga taaaaggatt gacgtatata gtttaatttt tttttttagt aattatagtt 5820
tttttttttt ttttttttaa gttagaaaaa atgagttaga tttttttggt aatttatttt 5880
gtagatgagg aaattggatt ttagggaagg taagtagaga gtaaggttat ttagttagta 5940
gatagttaat taggtggtta aataaagtat tagtgattta ggttttgttt taaatattat 6000
aaattttatt tattttagtt tagtttttgt tattttttat atttgttttt tttttttagg 6060
aaggaagttt ttggaattta g 6081

<210> 211
<211> 9243
<212> DNA
<213> Artificial Sequence

<220>
<223> chemically treated genomic DNA (Homo sapiens)

<400> 211

tgttattttt aaatttgaaa agattgagag attttagagg gtttagatgt gttaaaggtt 60
agagggatta atatataggt tttattacgg aaaggcgggg aaaaggttcg aatagaaaat 120
tgttgtagaa gggaagttat tgagaggtaa gggagttttt gaataattaa aaagttaaga 180
ataagtaaaa ggaaggaggt cgggtggggg ataaaaaaag tagttgatgt ggtaattaga 240
tttggtggga gttgggtagg ttattttttt tttagattag agtttattag aaattttttt 300
agtgtttttt tgcgtcgtta aacgtggggt gtggtaatgc gttacgtttg tgcgaggagt 360
gtgcgtaaga tatagtagag ttgttataag ttagatagta aggaagagtc ggtgggataa 420
aaaagtagta tgtgtattag aattggtgga gttgggtagt tatttttttt tcgatcgagt 480
tttatcgaaa tttttttagt gtttttcgcg tcgttaaaga tgataatagt aaattaataa 540
gtgtttgaaa tgaaagggga tgttgattag tttttaggtt atagattttt cgtcgttagt 600
tttttttgaa tttttatagc gtgtttttgt atcgtttttt ttaagaagag ttatttttta 660
tttttatttt taggacgaag gtaagtgttt agttagtata tttattaaat gttagttttg 720
gttttagttt ttcgtttgcg cggaaagttt attgttatag cgcgtttagt ttgtcggagg 780
gtagatagaa aaagtaagtt tggtttggcg atttgcgggg ttacgtattt ttagggttgg 840
ttcggagttt tttagagttt aacgtttttg ggttagaatt gtaaggtttc ggttcgagta 900
aagggtttga gttatcgtag tcgtgggagc gtttttttta tttgaatgta tttatttata 960
aataagtata aaattttttt aatagtagag gagaaagatt tttgttttaa aattaaagtt 1020
gggaattatc ggaaatttcg ttttgagtgc gagatattgg tttgttattt ttttaatttt 1080
tatatttttt ttgtaatatg ttttgattta ataatttttt tagcgtttag tattgttcgg 1140
acgttttaat tgggtaattt attagtgagt taatataggt atttatatat ttttttagtt 1200
taagtggtta agtattaatt tcgaaatgat tataaaatat cggaatcggt aacgtatagt 1260
aattttaatt tatatgtaaa ttaattggtt tattttaaat gtttttttta aaaaaataat 1320
tattgtattg tagtatcgga ggtatggatt aaatttttag aatagataat tcggaaataa 1380
gatttggatt aggaagataa tttagaatag ttaataaatt aataatgttt gaggtagtta 1440
aatatcgtta gttattggta tttatatatt cgtttgttgt tagataagga gttggggaaa 1500
ttgtttgtta gggttgagat tataatttag agtgaagaaa gtaaatggta gtatatagtt 1560
cgttatagcg ggttttgaaa taatattgta ttttttttaa attttgattt ttgggtgata 1620
gggagttggc ggaggttatt ttatatttgt ttacggtttt gttttaattt gattacgaaa 1680
ttgttgtttt ttttgagttt tttaatttga ttattatttt aacggttttg ttatttgttt 1740
taatatattg gggggaggag tgtaattgag atttttatta aaaattattt gaatttattt 1800
agttagtatt gttttattta agtttagttt tatgggttgt atttaatttt ttgtgttttt 1860

tatatattaa aattagatta ttaaaatgtc ggtaggaaag ggtgaaggaa atggtttaat 1920
gttttagttt attggaagat tattattttt agatatagtt taaaattttg aggaaataaa 1980
aaggatatac gttttggggg gaaaatgttt taatatttta gaatgggggt attatttttt 2040
ttattttaga gaattcgtat tggagttgtt tatgtaaaaa tgtaatattt ttgaaattta 2100
tagatacgta aggttagtgt tttttttttt ttaggttttt agtttaggcg atttagtttt 2160
aaaggagtta gtatttttga tgttataatt ttgtttatat ttgtagggta gagaattgtt 2220
tgttttgttt ggacgttttt tttatttttt tttaatttga agtaatcgga atttaaatat 2280
agtcgttaag gttcgttttt tttttattgt tttgataagg gaaaaatttg aaatttacgt 2340
tttaaattag ttcggtggtt tgtagttttt tagtattttg tttttacgat tgtatgttta 2400
atgtattttt tggtgatttt gggtattaat tagttgttta ataggagtat gattaaaaat 2460
gtaaaagaag gattaggagc gtgaaacgta tgtttagttt tttttatata ttcgaggagg 2520
gaatgagaat tattttgtat tttttatttt tttaggagtt atttgtattt tttattagtt 2580
gtttatttta gttgtattgg cgttgggtaa ggcgaggatt taaaagttta gcgtagtgtt 2640
tgcggcggtc gggattgggg ttaattagtt tttggcgggc gagattttag atagaagggg 2700
ggcgagagga acgtgagttt ttcgagtttt ttttttttag ttttggtttg taaatttttg 2760
aaatttgaaa ggggagggag ttgtacgcgc gtatttttgc gtttttttag cgtaattttt 2820
tttttttttt ttgtgttttt tcgcggattt ttgaattttt ttgtttttgg tttttttatt 2880
tttttttaat ttttttatga gattgtttat tttcgttatt agttgaaggt aaggtcgttt 2940
tgttacgagc gttttttaat ttttataaaa tgaaaagaaa aaaagggagg attattagtt 3000
tattatttag aggaatgggg aggttgtaaa aatcgtcgat gggtagaggt gaagatgttt 3060
ttttcggatt gtatttttcg gtgttttgta attagagttt agttgtggga tttgttgaag 3120
aaatttgatt tttttgtttc ggcgagattt taaaaattag aaatagaaat ttttagagtt 3180
agagaggaaa tataattaaa tagtacgtgg gtattttttt ttttattttt ttttttttaa 3240
ataatattgt tttgagtttt tattgggtaa agagagaaag tttgagtttt tacggatgtt 3300
acgtggaggt tagaaatggt ttaaaatgta gatttttaat tagttttttt cgtggttgaa 3360
gaggttaatt ttttttataa aatgagttta tttgtcgatt gttagttatt ttaaagtgaa 3420
gggatttagt atttaaaata aattgagtaa gtttgtttgt ttgtttttat tgttaattta 3480
aatgaattta aaatacggag taatttaaga aaatatataa tatgttttag atagttttta 3540
aaagtaggga aagtttagta tttatatagt gattagggtt agttttaagc gttaagtttt 3600
tttaaacgta tttattttat gtatattttt tcgagttatt atatattttt aaaattgcga 3660
gtattggtat attgatttag gaagagtaat ataattttta gagggaattt tatttttaat 3720
tagggattaa agagatgttt ttttaatagc gggtttgagt tttgttttta agtaggaatt 3780
aatattggtg ggaaaattcg aatttaggag taatggttgt gtttcggtat tttttaaaaa 3840
tatatattaa taggatgttt ttgagattga aaaaatattg ttttatatgt ttggtagaag 3900
tttttatatt tggtttttta ggcgaattat atttatagtt tttttattta gaggtaggat 3960
agagttaaaa tattttgttt attattaaaa tatatatttt tgtttaagtt aagaaattag 4020
aaaattaggg tttagaagta aggtatattt ttcgagtgag aatatgtttt gtaattttat 4080
atattttttg ttttgtagga gtaaatgtgg atttgaggga aatttttttt tttattttta 4140
tttttatttc gtgtaattta atattatttt cgttaggaat tttaatttcg ttattttaaa 4200
aaatgagata ttcgtgattt agggtgaatt tgttgaatgt aggtatagta gaggaaattt 4260
tagattttat gagcgtttga gttttgttta gtgtaaattt ttcgtgaata ttgggttagt 4320
gcgtggtcgt gtttatttgt gcgtcgatat ttttagtatg tttggtttat tcgttttgat 4380
ttcgggcgcg gtgttttagt taagttgggt ttagcgtttc ggtttttttt agttgataag 4440
tttagttcgt tcgttttcgg ttgtggtttt tttatttttt tttattagtt tattttattt 4500
ttttagattt ttttttattt attttttttt atttttatcg cgtttatttt tattttcgtt 4560
ttttatcggt tttttatttt ttttttttcg tagttttttt ttgttgtgat tttttttttt 4620
aattttgtag gtttgaaaga aggttatata cgtacgttta tatttatatt ttatacgttt 4680
cgttttaaat aattttatga atattgtttt ttgtttcgtt ttttgggtta ttttttttgt 4740
cgtttttttt tagttcgttt tgatttgttt tttaaaagta cgtttttgtt ttttcgttgt 4800
tttggcgttt tttttttgat ttattagggt tgtcggttgg gcgtagattg tttttttttt 4860
ttttttattt tatttttttt tttggttttt ttttttatag tgggagttcg tgtttttgtt 4920
tttcggttgg tttttaagtg tttcgttagg tttttttttt tttcgttttt tcggtttcgg 4980
ttttcgattt ttcggttcgt tggtatttgt tttttttttt tgtttcgttt ttcgtcgttt 5040
ttgttcgttt ttttcggcgt tcgttcgggc gttgtgttcg tttttggatc gttagtcgcg 5100
tagcgggttc gtcggcgttc gcgcgttatt gtgtagtgga gtttggtgga atttttgttg 5160
acgttacgtt attttttata cggagtagga gtagagggaa gagagaggga tgagagggag 5220
ggagaggaga gagagtgcga gatcgagcga gaaagttgga gaggagtaga aagaaattgt 5280
tagtggcggt tagatttcgg aggttttagt gtattcgtgg attttttcgg aatttggtat 5340
ttttaggagt tttgtagttt ttttaggttc ggttttcggg cgtttgtcgt gtagtcggag 5400
gttcggttcg ttggaaatcg tttcgggaag tagtgggacg cggagatagt agtttttttt 5460
cggtagtcgg taagtggagg ttatttattt cgtagggatg tgagataatg cgagtttgga 5520
aatttgtttt atttcggaga atttttatcg taggtgattt gtggtttttg gggttaagtt 5580
tcgtttaagg taacgtagtc ggtaaataga ttttgtaaag ttttgttttt ttcgtttttc 5640
gttatagata ttaataattt atagggtgtt gaagtcgaga gggaagttag atcgtggttg 5700
gtatttaaaa cgaggtattt ttttttaaat ttcggtgtta atattgtagg aataaatttt 5760
cgggttaagg attagtattt ttaagataaa gggttgggta taaagtttta gttattggaa 5820

gattagtttt ttttttattg ttatttattg ggaaaaaaaa gaaaagaaaa agattttatt 5880
ttaattggta gttagtgatt ttttaggttt aagcgaatta tttgggagtt aggtttggat 5940
gttaagtttt tattattttt ttggattgta atttttttaa attgattatt agttaatttt 6000
aatttggtat tttaggagat atattttaaa tggatgtaga gaattatttt ttagttggag 6060
attaagaaaa aaattttcga ttttaaattt tcgaaatatg tttttttttt ttagtttaat 6120
tattttattt ttttaagtaa tttagaaatt aaattattat aaggtggtgt gatttttttt 6180
tatttttttg tgtgagtatt gttttattaa attaaacgga aaaaattttt attattataa 6240
atgtaaatat tagaatttat atattttaaa atatttttat gaaaaattaa tttgatttaa 6300
agaaattttt ttgtatttgt tttagtttat taattaaaat taaagatgtt tttattatat 6360
aaaatattat tttggtagaa atttatttaa aatttaaata ttaataatat taagaaaata 6420
aagtatataa gtaaaataaa ttgaagattt ttgttgatgt aatatgagta tataatattt 6480
taataattaa atttttttta aaaaattaaa tagttatttt atttgtggaa tgttttattt 6540
taatttagta aaattatatt taaattattt aggtgttttg ttttttaagt taagcgtgtt 6600
tgtttttaaa tgtttttaaa gtatttatat taattggttg taaagaacgt atatatatgg 6660
taaaatatag aattgaattg agtagtattt taattttttt aaataattat ttattataaa 6720
ttaatttatt ggttaatttt ataatttagt ttatttaaaa tatatgtttt tgtgttgttt 6780
atttttaaat tttttattaa agattttgtt atggggtaat aaagtgtatg aaaagggggg 6840
aaatgtgaaa ggatttggga ttattcgaat tgtatttttt ttgtattttt agttttgcgg 6900
tagttattag aaattatttt ttagtaaatt gttttatttt ttagggtttg tttgtttgtt 6960
ttgttatggt ttttcgtttt tcgttagtcg tgtagtgttt tttgtgtgtt tataatataa 7020
aatttaagtt ggttaaaata agagtttttg gtatatatat tttaattaga atatgaattt 7080
tgggggtgag aattattttt tattaggaaa agttttttat tttaatttgt gagattagtt 7140
attgaagtta gtttcgaagt ttggtagtta aattttttat agaagatttg ttttgatagg 7200
gtaagtttaa ggattagtag gcgggaattg gaggtttttt tttaaaaaat tatttttttt 7260
agttatttag atttagtttt tttagtaggt ttggttatta aatgaagtat aaaaatgtaa 7320
gttttaaggt ttattttgat tgtaaaataa atttttaagt tataaggata tgtaggagtg 7380
agttaaggaa tacgttttga tttttttttt agtttttaga gtggagtttt atgagttttt 7440
gaagatttgt tttgtattgt tttgtttggt ttttagtatt gaagtacggg gaagtggggg 7500
gaagaatgtg taataattga ttgattttat attaagtaac gtaatttttt ttttttgtat 7560
attttatttt ttaaaaaaaa taaataaata aaaattattt gtagttatta tttgtagtgt 7620
ttcggttatt agttaataat gtagttagtt tagatatata aaaaaaaaag attatcgaaa 7680
tgatgatgat atgtaaattt ttttcgaaat tattataagt aaatatttga agtttggatt 7740
aataaaattt tatttgtgtt attttatatc gagttagtag aaagttgtga taatgaattt 7800
tgtaatattt tacgaataga tattttaatt agggattaat tttgtgattt tattgtagaa 7860
ttattaaatt tggagtcgtt aaattgttat ttttgggttt acgggtttat aaggatcgaa 7920
tcggtagagt tttcgttcgc gttttcgtta gcgggtgggg gaatcgtttg gtcgttttta 7980
ttttggattt ttacgttata gcgtcgggta gttttttttg taggtagcga ttttggttag 8040
aggtttttta gggtttagtt ttttttagga gaggtcgaga cgtagggaaa cggtatttag 8100
gttagaggta ggttcgtagt tttttgtttc gtttttgtgt tttcgttaat tcgataacgt 8160
ttgtttttat ttcgattttc gtattcgcgc gaagtgggtt tttcggtcgt cggcgtattt 8220
tggttagcgt ggagagaggt aggcgttgag atcgaagggg tttagggagt tttggatttt 8280
ttttttttgt ttttaaagta atcgcggttt ttttatttat tcggtggagt ttttcgagat 8340
ttattttttt cggtttgttt gtggtagaga agggggagcg cgttaaatgt ttggttcgtt 8400
gcgttgtggt tgaaaacgtg aaaaagattt ggttcgttcg ggagagaaag ggggagaatt 8460
gggtagtagt tatattagag ttattttttc gtttttggcg ggtagtaaat tttttaagaa 8520
cgtttgtttt gtttttttta gtttcgttta gtttatttag tgtttttttt ttgcgatttt 8580
aaattatatt ttagggtaat tatttgtagt aagtaaataa atggtcgggt tagtattttt 8640
aggagaaagt gtggttaaat atggaaaagt ggtttttgat ggatgagagg ttcgaattta 8700
gttcgttttt gaaatatttt aggttaagag ttcgttcgtt ttagaattat agaaaatcga 8760
gggaaattgt tgtttaggat aggggtacgt tggcgttgat gttttataaa tgtttatcga 8820
gttttaatta atggataagt attgaagggt ggtttttgta tatagttttt taaagagaaa 8880
agtttttttt atttatttat tttcgttgtt attgcgttta gatgagtttt taatttcggt 8940
atcgagattt ttgaaagtag gtttatagtt tttttagtat attgtggttt tatagttttt 9000
taattttggg gtatttttgc ggtaattttg gagggagatt tttttttgat aaataaatgt 9060
tttgggttcg aggttaggtt ggagatgttg ttgtatatgt tagaggttgt taggtcggaa 9120
aaatacgttt gaagtttagt atatagtagg cgtttaatag ttagtgtaac gtagttttat 9180
ttgagttttg tttattcgac ggtcgtcgtt ttttatagtt tttttttttt tttgttttgt 9240
aga 9243

<210> 212
<211> 9243
<212> DNA
<213> Artificial Sequence

<220>
<223> chemically treated genomic DNA (Homo sapiens)

<400> 212

tttgtaaaat agaagagaag gaaggttgta agaagcggcg gtcgtcgagt gagtagggtt 60
tagatgagat tacgttatat tagttgttag gcgtttattg tgtgttaggt tttaggcgtg 120
ttttttcgat ttgatagttt ttggtatgtg tagtagtatt tttagtttag tttcgggttt 180
aggatattta tttattaaga ggggattttt ttttagagtt gtcgtaaaag tgtttagagg 240
ttagaggatt ataaagttat agtgtgttgg ggaggttgtg gatttatttt taagaatttc 300
ggtgtcgggg ttaagaattt atttgaacgt aatggtagcg ggagtgggtg ggtggagagg 360
attttttttt ttgggaagtt gtatgtaaag attatttttt agtgtttgtt tattagttgg 420
agttcggtaa atatttgtag aatattagcg ttaacgtgtt tttgttttag atagtagttt 480
ttttcggttt tttgtaattt tgaaacgaac gggttttggg tttagggtgt tttaggagcg 540
agttgagttc gggtttttta tttattagga gttatttttt tatatttagt tatatttttt 600
tttagagata ttaattcggt tatttattta tttattataa ataattattt taaagtatga 660
tttaagatcg tagaggagag atattgggtg gattgagcga gattgaggag agtagggtaa 720
acgtttttgg agggtttatt gttcgttaag gacggagaaa tagttttggt ataattgtta 780
tttagttttt tttttttttt tttcgggcga gttaaatttt ttttacgttt ttaattataa 840
cgtagcgagt taagtattta acgcgttttt ttttttttgt tataggtaag tcgggagagg 900
tgggtttcga ggggttttat cgggtgggta gaagagtcgc ggttgtttta aagataagaa 960
aagaaggttt agggtttttt aggttttttc gattttagcg tttgtttttt tttacgttaa 1020
ttagggtacg tcgacgatcg gagggtttat ttcgcgcggg tgcggggatc ggggtgggag 1080
taagcgttgt cgggttggcg gaggtataga ggcggggtag ggagttgcgg gtttgttttt 1140
ggtttgagta tcgttttttt gcgtttcggt tttttttgaa gggagttggg ttttggggag 1200
tttttggtta aggtcgttgt ttataggagg ggttgttcgg cgttgtggcg tggggattta 1260
gggtggggac ggttaggcgg tttttttatt cgttagcgag aacgcgggcg gggattttgt 1320
cgattcgatt tttgtgggtt cgtgggttta gaagtagtag tttggcggtt ttagatttag 1380
tgattttgta gtaaaattat aggattagtt tttgattgag atgtttgttc gtgagatatt 1440
ataaaattta ttattatagt tttttattaa ttcgatatga agtaatatag atgggatttt 1500
attagtttag attttaaatg tttatttatg ataatttcgg aggaaatttg tatgttatta 1560
ttatttcgat aatttttttt ttttatatgt ttgaattggt tgtattatta gttggtagtc 1620
ggagtattgt agatggtaat tgtaaatagt ttttatttat ttattttttt taaagaatga 1680
aatatataaa agaaaaagat tgcgttgttt ggtgtaaagt tagttaatta ttatatattt 1740
ttttttttat tttttcgtgt tttagtgttg aagattaaat aaagtaatat aaaataaatt 1800
tttaagaatt tatagagttt tattttaagg attgaaaaga aggttaaggc gtgtttttta 1860
gtttattttt atatgttttt gtgatttgga gatttatttt gtagttaaaa tgagttttga 1920
gatttgtatt tttatgtttt atttaatgat taggtttatt agaagaattg agtttaaata 1980
attggggaag ataatttttt aaaaagagat ttttaatttt cgtttgttga tttttaaatt 2040
tgttttatta agataagttt tttgtgagaa atttggttgt tagatttcgg aattggtttt 2100
aatggttaat tttataaatt gagatgggag attttttttg atgggaggta gtttttattt 2160
ttaaagttta tgttttagtt ggaatgtata tgttaaggat ttttgttttg gttaatttgg 2220
gttttatatt gtgagtatat aaaaagtatt atacggttaa cggaggacga ggaattatgg 2280
taaagtaggt aggtaagttt taagaaataa aataatttgt taaaaaataa tttttgatga 2340
ttatcgtaag attgaaagtg taggaaaaat atagttcgaa taattttaga tttttttata 2400
tttttttttt ttttatatat tttgttattt tataataaaa tttttaatgg aaagtttaaa 2460
aataaatagt ataggaatat gtgttttaaa tgaattaaat tgtgaaatta gttagtaaat 2520
taatttgtag taagtaatta tttaaggaaa ttaaaatatt gtttagttta gttttgtatt 2580
ttattatgtg tatgcgtttt ttataattaa ttaatataag tgttttagga atatttgaag 2640
ataaatacgt ttaatttaag gaataaagta tttaaataat ttaagtgtaa ttttgttgag 2700
ttaaagtaaa atattttata aatgaagtgg ttatttaatt ttttagggaa agtttggtta 2760
ttgaaatgtt gtatgtttat gttatattaa taaaaatttt taatttattt tgtttatgtg 2820
ttttgttttt ttgatattat tggtatttga attttagatg gatttttgtt aaaatgatat 2880
tttgtgtgat aaaagtattt ttagttttga ttgatagatt aaaataaatg taaggaaatt 2940
tttttaaatt agattaattt tttataaaaa tattttagaa tgtatgaatt ttgatattta 3000
tatttataat ggtaaaagtt tttttcgttt agtttagtaa gataatattt atataaaaga 3060
gtaaaaaaaa attatattat tttatgatag tttgattttt aaattgttta agaaagtaaa 3120
gtggttaaat tggaaaagag gaatatattt cggaggttta gaatcgaaaa tttttttttt 3180
aatttttagt tggaaaataa tttttttgtat ttatttaaag tgtatttttt gaagtgttag 3240
attggagttg attggtgatt aatttaaagg agttataatt taaagaaatg gtgagagttt 3300
ggtatttagg tttggttttt aggtaattcg tttgggtttg agaggttatt aattgttagt 3360
taagatggaa tttttttttt tttttttttt ttttaatgga taataatggg aagggggtta 3420
attttttagt agttgaaatt ttgtatttag ttttttattt tgagaatgtt aatttttggt 3480
tcgaggattt gtttttgtag tgttggtatc gagatttaag ggaagatatt tcgttttaaa 3540
tgttagttac ggtttggttt ttttttcgat tttagtattt tgtagattgt tagtgtttgt 3600
ggcgggggac gaaaggaata gggttttgta aggtttgttt gtcgattgcg ttattttggg 3660
cgaaatttag ttttaaaagt tataaattat ttacggtgaa gatttttcga agtggaataa 3720
atttttagat tcgtattatt ttatattttt gcgggataga tggtttttat ttatcggtta 3780

tcgggagaga gttgttgttt tcgcgtttta ttgtttttcg gggcgatttt tagcgagtcg 3840
agttttcggt tgtacggtaa gcgttcgaaa gtcgggtttg agaggattgt agggtttttg 3900
agggtgttaa gtttcgaagg agtttacggg tgtattgggg ttttcgaaat ttagtcgtta 3960
ttggtagttt ttttttgttt ttttttagtt ttttcgttcg gtttcgtatt tttttttttt 4020
tttttttttt ttattttttt tttttttttt tgtttttatt tcgtgtgggg agtgacgtga 4080
cgttagtaga gattttatta aattttattg tatagtggcg cgcgggcgtc ggcgagttcg 4140
ttgcgcggtt ggcgatttag gagcgagtat agcgttcggg cgagcgtcgg ggggagcgag 4200
taggggcgac gagaaacgag gtaggggagg gaagtagatg ttagcgggtc gaagagtcgg 4260
gagtcggagt cgggagagcg aaaggagagg ggatttggcg gggtatttag gagttaatcg 4320
aggagtagga gtacggattt ttattgtgga aaggaggatt agaagggagg atgggatgga 4380
agagaagaaa aagtaatttg cgttaattcg gtagttttaa taaattaaag ggggagcgtt 4440
agggtagcgg ggagatagaa acgtattttt ggggagtaaa ttaggacggg ttgggaggaa 4500
gcgataggga aagtggttta agagacggaa taaaggataa tgtttatggg gttgtttggg 4560
acgaggcgtg tggagtgtgg gtgtgagcgt gcgtgtgtga ttttttttta ggtttgtaga 4620
gttgaggaaa gaggttatag taaagaggga ttgcggaggg aggaaagtga gagatcggta 4680
gagggcggga gtggaggtgg gcgcggtggg gatgggagag gatgagtgaa gagaaattta 4740
gaagaatgga gtgagttagt gggagagggt gggagggtta tagtcgggag cgaacgagtt 4800
aggtttgtta gttggggaag gtcgggacgt tgggtttagt ttagttggga tatcgcgttc 4860
gaggttaagg cgggtggatt aggtatgttg agagtgtcgg cgtataggtg ggtacggtta 4920
cgtattgatt tagtgtttac gaagggtttg tattggataa ggtttagacg tttatagagt 4980
ttagaatttt ttttgttgta tttatattta ataagtttat tttgggttac ggatatttta 5040
ttttttaaaa tgacgaggtt aaggtttttg gcgaggatgg tattaaattg tacgggatag 5100
aagtgggggt gggggagaga gtttttttta agtttatatt tgtttttgta aagtaaagag 5160
tatgtgaaat tatagggtat atttttattc gaaaagtgtg ttttattttt gaattttgat 5220
tttttgattt tttgatttga gtaaagatgt gtattttggt agtgagtaga atattttggt 5280
tttgttttgt ttttgagtgg aaggattata aatataattc gtttggagga ttaggtgtga 5340
aggtttttgt taggtatatg ggataatgtt tttttaattt taagggtatt ttgttaatgt 5400
atgtttttgg aaagtgtcgg aatatagtta ttgtttttgg attcggattt ttttattaat 5460
attaattttt gtttgagagt aaaatttagg ttcgttatta aaaagatatt tttttggttt 5520
ttaattgaga ataaagtttt ttttaaaagt tgtattgttt tttttaaatt aatatattaa 5580
tattcgtaat tttagaaata tatagtgatt cgggagaatg tgtataaaat agatacgttt 5640
aaaaaagttt ggcgtttaaa attaatttta gttattatat aggtgttggg ttttttttat 5700
ttttgggggt tgtttggaat atgttatgtg tttttttgaa ttatttcgtg ttttgaattt 5760
atttgagtta gtagtaaaaa taggtaaata aatttgttta atttgttttg agtgttaaat 5820
ttttttattt tgaaatagtt aatagtcgat agatggattt attttatgga aagggttagt 5880
ttttttagtt acgaagaaaa ttgattagag atttatattt taagttattt ttaattttta 5940
cgtaatattc gtgaaaattt aaattttttt tttttattta gtggaaattt aaagtagtgt 6000
tatttaaggg gagagaaatg agggggaaaa tgtttacgtg ttgtttaatt gtattttttt 6060
tttgattttg agaattttta tttttggttt ttgaaatttc gtcgaggtaa gaaaattaaa 6120
tttttttaat aagttttata attgaatttt agttatagga tatcggaaag tgtagttcga 6180
gaaagatatt tttatttttg tttatcgacg atttttgtag tttttttatt tttttgagta 6240
atgggttaat aatttttttt tttttttttt ttattttgta gagattaaga ggcgttcgta 6300
gtagaacggt tttgttttta gttggtggcg aggataggta attttatgga aaagttggaa 6360
gagaatgaga aaattaaaga tagaaagatt tagagattcg cggagagata tagggagagg 6420
gaagggagtt gcgttgaaaa gacgtaaaga tacgcgcgtg taattttttt tttttttagg 6480
ttttagaggt ttgtaaatta gggttgagag gaaggggttc gggaagttta cgtttttttc 6540
gttttttttt tgtttggagt ttcgttcgtt agaggttggt taattttagt ttcggtcgtc 6600
gtagatattg cgttgagttt ttgggttttc gttttgttta gcgttagtgt agttgaagtg 6660
agtagttggt gggaaatgta aatggttttt ggagaaatag aagatataga atgattttta 6720
tttttttttc gagtgtgtgg aaggagttgg atatacgttt tacgttttta attttttttt 6780
tatattttta gttatatttt tattaaataa ttaattaatg tttagaatta ttagggaata 6840
tattaggtat gtaatcgtag aagtagggtg ttggggggtt ataaattatc gagttgattt 6900
aagacgtgga ttttaggttt tttttttgtt aaagtagtaa aggaagagcg ggttttggcg 6960
attgtattta gatttcgatt attttaaatt agaagggggt ggagggagcg tttaagtaaa 7020
gtaagtaatt ttttgttttg tagatgtaaa taagattgta gtattaaagg tattagtttt 7080
tttagggtta gatcgtttgg attgggagtt tggggaaggg gagatattaa ttttacgtat 7140
ttgtgaattt taaggatgtt atatttttat ataaataatt ttagtgcgga ttttttggaa 7200
tggggggagt aatattttta ttttagaata ttaaaatatt ttttttttaa agcgtatatt 7260
ttttttattt ttttaaaatt ttgaattatg tttaaagata atagtttttt agtaaattgg 7320
agtattggat tatttttttt attttttttt atcgatattt tgatgatttg attttaatgt 7380
gtggggggta tagggaatta aatatagttt ataaaattaa gtttagatga aatagtgttg 7440
gttaagtggg tttagataat ttttaatgag aattttaatt atattttttt ttttaatatg 7500
ttgagataag tgatagaatc gttagaatgg taattaaatt ggaaagttta gggagaataa 7560
taatttcgtg attaaattgg ggtaaaatcg tggataaatg tggggtgatt ttcgttaatt 7620
ttttgttatt taagagttag gatttgggaa aggtatagta ttattttaga gttcgttgtg 7680
acgggttgtg tgttattatt tatttttttt attttggatt atgattttaa ttttggtaag 7740

taattttttt agttttttat ttgataataa gcgagtatgt aaatattaat ggttagcgat 7800
gtttaattgt tttaaatatt attgatttgt tggttgtttt aaattgtttt tttagtttag 7860
gttttgtttt cgaattgttt attttagagg tttgatttat gttttcgatg ttataatata 7920
ataattgttt ttttaaaaaa ggtatttaag atgaattaat tgatttgtat ataaattaaa 7980
attattatgc gttgtcgatt tcggtgtttt ataattattt cgaaattagt atttaattat 8040
ttgagttaaa agaatatata aatgtttgta ttgatttatt aatgaattat ttaattaaaa 8100
cgttcgggta atgttgggcg ttggaaagat tgttaaatta agatatatta taggagggat 8160
atgaagatta gaaaggtaat agattaatat ttcgtattta aaacggagtt ttcggtgatt 8220
tttagtttta attttggagt aggggttttt tttttttgtt gttaaaaaga ttttgtgttt 8280
gtttgtgagt gagtgtattt aagtggaagg aacgttttta cggttacggt ggtttaggtt 8340
ttttgttcgg atcgggattt tatagtttta atttaggagc gttaaatttt ggaagatttc 8400
gggttagttt tggaggtgcg tggtttcgta agtcgttagg ttaagtttgt tttttttgtt 8460
tgtttttcgg taggttgggc gcgttatggt agtgagtttt tcgcgtaaac ggagagttgg 8520
aattaaagtt gatatttaat agatatgtta attgagtatt tattttcgtt ttgagaatag 8580
gaataaaagg tagttttttt taagagaggc ggtgtaaagg tacgttatag gagtttagaa 8640
aaggttggcg gcgggaaatt tgtagtttgg gggttagtta atattttttt ttattttaag 8700
tatttattga tttgttgttg ttatttttgg cgacgcgaag gatattgaaa gaatttcgat 8760
ggggttcgat cgagaaggag gtgattgttt agttttatta attttaatat atatattgtt 8820
tttttatttt atcgattttt ttttgttatt taatttatag taattttatt atgttttacg 8880
tatatttttc gtataaacgt gacgtattgt tatattttac gtttggcgac gtagaaggat 8940
attgaaagaa tttttgatgg gttttgattt gagaaagagg tgatttgttt agtttttatt 9000
aaatttaatt attatattaa ttgttttttt tatttttat tcgatttttt ttttttttgtt 9060
tatttttaat tttttaatta tttagaaatt tttttatttt ttagtggttt tttttttgta 9120
gtagtttttt attcgaattt ttttttcgtt ttttcgtggt agggtttgta tattgatttt 9180
tttgattttt ggtatatttg ggttttttga aattttttaa tttttttaga tttgaggatg 9240
gta 9243

<210> 213
<211> 6763
<212> DNA
<213> Artificial Sequence

<220>
<223> chemically treated genomic DNA (Homo sapiens)

<400> 213

taaaattata aatggtttta gggttaaatt attggaatat attaattttt ttatttattt 60
atattttgta aaatttgtta aattatattg gattttaaag atttatttgg tttgatttga 120
gaaattagtg aaaagttaaa ttttattgtg gttttttata attttattaa gtttggtttt 180
tataattgtt tattattgga atattttaat ataaagggta gatgttttat tttattgtta 240
ttatttggat aatttgatgt tatttttgaa atgtgtgtgg tgtggttatg gatatttgta 300
gtattggaat agagaaggga ggagatgtga gggaattcgg agagtttaag gtatttgttt 360
tgaaaattat atattttaat gagaagtttt ggattatgat tgggagatta aaatgataat 420
ttatgtattg agatgaattt tatttggaaa ggggaaatgg aatatttagt tgttgtttta 480
taaaggttgg taatttttgt tggttgataa ggttttaagt ttttatttat ttttatttgg 540
taattgttat aaggggtgtt agaggtagaa gggttattta aagggattgt tttttcggag 600
gatattttag taggtttggt tgttttagtt tgtattttag atttgtggaa tttttgtggt 660
tagttgaagt taataaattc ggtgtggtat agtagttttt tgtatagggt atgtatatta 720
aagtcgataa tgtttataaa tgtaaataag ggtagattga ttaggaataa tattatcgtt 780
gggaatgtcg tattcgtatg tattagtaat gaggtaaatt gggggtagtt ttagttttga 840
ttatttaagt cgagtaggtt tttatattgg tttttttttt tttttttaaa acgttataga 900
aataaggtta attagatttt taaagtaatt tatttttaag tatgaaaaaa gtagattata 960
ggggaagcgg cgggtggggg gtggggagat taagatagcg ttcgttagag tcgaggtttc 1020
ggagtgcgtt ttggaattta cgggtagaat ttataggtcg ggattattgt ttgagatgtt 1080
gaaaagttaa tttattaaat agcgggcgag ggggtttagg gtattgaaag tttcggggat 1140
taatgtttaa aacgtttagg aattaagtta aattaaggga tatgggtttg agtggaagag 1200
gttttgttat tgaaatgacg tgtatgtggt agtcgtcgga attagaggat atgtagtcgc 1260
gtgcggagag tgaggcggat ttattacggg tatatagttt attatttttt tttagaattt 1320
ttttttttaa ataagttata ttgaaatttt tttagtaaaa gattgagacg aaatattaat 1380
agttttgggt taaaacgtgg tggagaagtt tgaaaagata aatatagtaa cgtgtttttg 1440
gttatatggt tttttgtttt ttagtaagtt tgaaatataa aaaggttaat atgtaaaatt 1500
aggggtatta tttttttttt ttagttttta aaaagtaatt tttagaagta aatatataat 1560
ggttcgattt gaagtgtcgg tataattatt tagtaagaaa agttagaata gtaaattttt 1620
tttttgtttt tgtgttattt attaaattta ataaatatta ggatcgtatt taaagatgaa 1680
attggtaatg tgtggagtcg tttgagtttt ttattaattt agtgttattt ttttcgtatg 1740

ttgttcgttt gtttttcgag ttttcgtagt ttttttttga ggtatttata aaattacgtg 1800
ttttttaagc ggtttgagtt ttgaattttc gcgttggttc ggtgtttagg ttttttttcg 1860
gatttcgcga acgtttttaa atatttttat ttcgtttttt tttttttttc gggatatttc 1920
gttttgattt cggtatcgta tacggtttgc gggttagggg gtcgggggaa gggttttttt 1980
ttgaagtcgg ggaggaggag aggttggagg gaaggtggtt aggttggtgg gggaggggag 2040
attgagggag ggagcggggc gagggaggaa ggagaaggcg taggtcgaag gaaggaagcg 2100
gggaggggaa atcgtcgggg aggaaggaag ggggcgggga gagtcggcgg acgggttttt 2160
tgcgggagtc gcgtggatcg tttcggtggt cggcgcgttt cgtcgtagtt tttagcgcgt 2220
tcgggtagtt agcgcggggt cgttgaatgg gtagggattc gtgagcgcga ttattggtcg 2280
gtcgcgggga ggcggcgcgg gtattgggta gcgtaagggg gtcggttcgt ttttaggaat 2340
gtattacggc gcgttcggta cgttcgttcg cgtcggttgg gcgcgcgttt cgtttgggtt 2400
ttcgggagat cgcgttggag gttcggagtc ggttcggcgt attcgttttt ttatttattt 2460
tgtttcggtc gcgcgggcgc ggggagttcg ggagtcgggc gcgcgcgcgt atcgggtttt 2520
ttggcgcggg gttacgtggt tatcggagtt tcgttttttt tttgtttttt tttttttttt 2580
tttatttggg tttttttttt tttttttttt tttttttttt ttttttttta atgaatatat 2640
atcgtgcgta ttttaaagtt atatcgtttt attttacgat tttattagat ttttcgttat 2700
tgttagtttt tttgtagttt aatttcgttt aaaaaaaaaa ataaagtgaa gtaaattaag 2760
ggtttgcggg tcgggttttt atcgtaggga ggaacgtaat aatttggaaa cgtaagaaaa 2820
atatatttta ttttttcgaa aataaaaatt tttttaaata aaaagtttag ttgtgtagac 2880
gtgtttggtt ttagaattta ttttaaaggg tcgcgtgttt ttttttttcg cgggaagcga 2940
attttcggga tgttttggtg atattggacg cggtgtcggg acgaattttc gcgattcgcg 3000
tatagtttgt ttttaatgaa tttagtatcg gggtatttat aattttttac gttatattat 3060
tattaaatat aatatgtata tcgagttttg taaaaaaaaa tattgattat ataaatattt 3120
ggttaataaa aaggggtgta taaatgaatt taaaataatt aggaaaattt tattttttta 3180
aggaaataat ttcgtttttt taagttgtaa aaaaagattt tgttgttttt tttttattta 3240
tgttatattc gagatttaaa taaaaacgtt tttgttttta taagattgtt tcggtgtttg 3300
gattttacgt ttatagaata ttgatttttt taaattaaga ttgtgttttt ttttttttta 3360
agttgggggg agaaagcggt tggggggtag acggatttta agaaagatgg ttttttttaa 3420
cgtttttgtt ttttttttta attttttatt ttgtttattt taatgcgagg cgttgttttt 3480
tgtaagagtt taagtttttt ttttagttat aaaagaaaag agaagaggaa aaaaaaattt 3540
ggtatatatt ggcgtattat ttagtacgtt gttattattg gtgtaggttt gggaatgcga 3600
gtcggatttt ttagtatttg ttgtagtttg aggttaaggt gtttttgaaa ggtttttttt 3660
tgaggaattt tagaaattgt ttaagcgttt aaaaaaatat tatatttttt ttcgtgttta 3720
tcgcgaggta aatgttcgtg tgtgtgcgtg cgcgcgcgtg tgtgcgcgta aaggtatttt 3780
ataaaaagaa gatattgttg aaaagaaaag gaataagaga gaaaggaggg ggagtatttt 3840
ttaatggaag tattgtatgc gagaaggttg gcgtttttag aggttgtttc gaagtcgagg 3900
atgtatatat tgggttgttt atttgggtgg tttttttatt ttggtgagtt gttgttaagt 3960
agttaataat agttatgttt gttgagtaat ttttgttttt cgcgagttaa tcgcgtcgta 4020
gaaatttaag ttatttgata gttcgggggg cgggagtttt agtttttttt tttttcgttc 4080
gttttttttt tttttatttt tttttttttt tttttttttt ttttttttta aatggagaga 4140
gttttttttt tttttttttt atttatttta ttgaattaag gagttgttgc ggtcgttgtt 4200
cgttgtatat atatagagcg gagtttttag gtttcgggag cgagagaggc gcgttgtacg 4260
gggatagaat ggtttagcgg gttgtcgagg agtataagaa agtagagttc gggatcgagt 4320
agttatcgcg aatttagtag ttagagttcg agtagttcga gtagtagttt ttggagtcgt 4380
tatcgttagt agtagttatc gcgggagtag cggtagttgc ggttgttcgg gttcggtagc 4440
gttgagattc gtcgggtatt ttcggatttc gcggcggcgg cggttttgcg tttgtttttc 4500
gcggtcgttc gggcgattcg ggaggcgtcg agagagttag cgcggtcggc gggagtagcg 4560
gggattcgtt ggtttttttt gtagtgaagg ggtcgcggcg cggggcgggg ggcgggtagg 4620
gggagttggg gatcgtaagg agtgtcgcgg aagcgttcga aggataggtt cgttcggcgc 4680
gtcggttttc gttttttcgc gaattggatg tgggtagcgg cggtcgtaga gatttcggga 4740
ttttcgcgta atgtggtaat ggaaggcgta gggtttgatt tttcggtagc ggtcgcggtc 4800
gtagcggtag tagcgttcgt cgtgtgagta gtagtagcgg ttggtttgtt aatcggagtt 4860
cgagttcgag tagtttgcgg ttagtagcgt tttcgtaagt cgagcgttta ggcgcgttag 4920
gagttcgtag tagcggtagt agcgcgtcgg gtcgttcggg aagttttcgt tttcgcggcg 4980
gcggcggcgg cggcggtaat atggtttcgg ttggtaacgt cgtcgagttt taggatcgcg 5040
gcggcggcgg tagcggttgt atcggtgttt cgggacggtc ggttggaggc gggaggcgta 5100
gacggacggg ggggttgcgt cgtgttgtcg cgtcggatcg ggattatttg tatcggttta 5160
gttattgcga cgtcgttttc gttttggagt agatttttaa ggtgtatttt agattttttt 5220
tttttatttt tttttttttt ttttaatttt ttgggatcgt tttcgttata tataaatata 5280
tatatttttt tttttttttt tttatatata tatatatatt tatttatatt tttttaggaa 5340
aagtagtaga taaatgggga ttgaaaaatt taaatttttt ttttggtttt gggaggaaag 5400
ggttgtttga ggttcgtagg gggtggaggt gtgtgtgtgt gcgtgtgtgt gtgtgtatat 5460
atacgttttt tttggtgtgt ttttttcgga gtattggaaa gtcgtttacg gcggattatt 5520
ttaagggcgg tcgcggtatt gttttgtttc gtgttttttg ttttgaattt tttttttttg 5580
cgtttttgtt tttatttgta gtttaaattt ttgtacggtt gttatatttt ttaatatttt 5640
ggagggggag gcggagtgga gagaggcgga gagaggaagg ggggagggag tcgaaataaa 5700

ggtggttttt ttttttggta gttagttttg gttttgttga gtatgaaatt tttgtttttt 5760
taaaaaatta ttttcggaaa aagatatttt tttcgttttt taggttttga gtcgtttttt 5820
tttagggttt ggtcggggga ggaaaagttg taaataaatt gttattttaa attcgcggtg 5880
cgagtttgcg gagttgtcgg gtttattgtg tttacgaggt tcgttgaaat gtgtggaatt 5940
tagggaaggc gagtatttag acgggggttc gtcggggtcg cggttagcgt cggggaaatg 6000
tcgcgtcggg gagtagtttg cgtcggtttg agtttttttt tttgtattcg gttgtttttt 6060
acgtttaatt agaaaggaag ggagaggagg gaaagattta tgtggttgtt ttttttcgat 6120
tataaatatt gtcgtaagtt gtagttggtt gttttatttt ttaatttagt ttatatagtt 6180
tttttttacg ttatggaaat gcgtcgggag tgaatttcgg cggtcgcgtt tattacgtgg 6240
atttttattt attattattt tcggcggggg tttagttggg ggaattcgta atatgttgtt 6300
ttaaagagcg ttcgttttta gcgttcgttt tcgagggtga tggatagagt aggattggtt 6360
tgttggtttt tgaatttggg gttttatcgt tgggattacg tagttttttt ttttttagtt 6420
ttggggtgaa tattgtttgt gtcgagtggt tttttttttt tttatttcgg tttttttttt 6480
tgtattgtag ttttagtgtt ttaagtcgtt tagggatttc gcgggtgggg gcgttttgat 6540
gttttcgagg tgtagttttt acgttgcggt tcggttttat gatcgagttt tttatgacgt 6600
ttagattcgt cggggttgta gtttcgaggg cgagttcgtg tgtcgcggtt attcgcgttt 6660
ttacggttgt tgggggttta tcgggcgggt gcgcgtaagc gggcgtgggc gcgcgggtcg 6720
cgatttttcg gcgtttattt tttttttttg tttcgttttt agg 6763

<210> 214
<211> 6763
<212> DNA
<213> Artificial Sequence

<220>
<223> chemically treated genomic DNA (Homo sapiens)

<400> 214

tttggggacg aagtagaagg gaggagtgag cgtcggggag tcgcggttcg cgcgtttacg 60
ttcgtttgcg cgtattcgtt cggtgagttt ttaatagtcg tgggggcgcg ggtggtcgcg 120
gtatacggat tcgttttcga gattgtagtt tcggcggatt tgagcgttat ggggggttcg 180
gttataaagt cgggtcgtag cgtgggagtt gtatttcggg gatattaggg cgtttttatt 240
cgcgggattt ttgagcgatt tggggtattg gggttgtaat atagaagagg aagtcgaggt 300
agagaggaga gaaattattc gatatagata atatttattt tagagttgag aagggagggg 360
ttgcgtaatt ttagcgatgg agtttaaggt ttaggagtta gtaaattagt tttgttttgt 420
ttattatttt cggggacggg cgttaggggc gagcgttttt tggaataata tattgcgggt 480
ttttttaatt ggattttcgt cgagaatggt agtaagtggg gatttacgtg gtgagcgcgg 540
tcgtcggagt ttattttcga cgtattttta tagcgtgggg agaggttgtg tgagttgaat 600
taggaagtgg ggtagttagt tgtaatttac gataatattt gtgatcggaa gagggtagtt 660
atatggattt tttttttttt tttttttttt tttggttaaa cgtaaaaaat agtcgagtgt 720
aaagggaagg gtttaggtcg gcgtaggttg tttttcggcg cggtattttt tcggcgttgg 780
tcgcggtttc ggcgggtttt cgtttgggtg ttcgtttttt ttggatttta tatattttag 840
cgagtttcgt aaatataatg aattcggtag tttcgtagat tcgtatcgcg aatttaaggt 900
ggtaatttgt ttataatttt ttttttttcg attaggtttt aaggagaggc ggtttaaaat 960
ttggaaaacg ggggggatat tttttttcga gaataatttt ttaagggagt agagatttta 1020
tgtttaataa aattaaaatt ggttgttaaa aaaggaaatt atttttattt cggttttttt 1080
tttttttttt tttttcgttt ttttttattt cgtttttttt tttaagatgt taagaaatgt 1140
ggtagtcgta taggagttta ggttgtaaat aggggtaggg gcgtaggagg aagaagttta 1200
gggtaggggg tacggggtag gatagtgtcg cggtcgtttt tgaggtggtt cgtcgtggac 1260
ggttttttag tgtttcggaa aaggtatatt agggagggcg tgtgtatata tatatatata 1320
cgtatatata tatattttta ttttttgcgg attttagata gttttttttt tttaggatta 1380
gagggagggt ttgaattttt taatttttat ttgtttgttg ttttttttgg agaggtgtga 1440
gtgagtgtgt gtgtgtgtgt gagagagaga ggaagagagt gtgtgtgttt gtgtgtggcg 1500
ggggcgattt taaagagtta gaggagggaa gagaaagtgg gaggagagag tttgaaatgt 1560
attttggaaa tttgttttag agcgaaggcg gcgtcgtagt agttgggtcg gtgtagatag 1620
tttcggttcg gcgcggtagt acggcgtagt tttttcgttc gtttgcgttt ttcgttttta 1680
gtcggtcgtt tcggggtatc gatatagtcg ttgtcgtcgt cgtcgcggtt ttggggttcg 1740
gcggcgttat tagtcgaggt tatgttgtcg tcgtcgtcgt cgtcgtcgcg gggacggagg 1800
tttttcgggc ggttcggcgc gttgttgtcg ttgttgcggg tttttggcgc gtttgggcgt 1860
tcggtttgcg aggacgttgt tggtcgtagg ttgttcgggt tcgggtttcg gttgatagat 1920
tagtcgttgt tgttgtttat acggcgggcg ttgttgtcgt tgcggtcgcg gtcgttgtcg 1980
gggagttaga ttttgcgttt tttattgtta tattgcgcgg gggtttcgag gtttttgcgg 2040
tcgtcgttgt ttatatttag ttcgcggaag agcgagagtc ggcgcgtcga gcgagtttgt 2100
ttttcgggcg ttttcgcggt attttttgcg gtttttaatt ttttttattc gtttttcgtt 2160
tcgcgtcgcg atttttttat tgtagaaaga gttagcgaat tttcgttgtt ttcgtcggtc 2220

gcgttggttt tttcggcgtt tttcgggtcg ttcgagcggt cgcggagggt aagcgtagag 2280
tcgtcgtcgt cgcggggttc gagggtgttc ggcgggtttt agcgttgtcg ggttcgggta 2340
gtcgtagttg tcgttgtttt cgcggtggtt gttgttggcg gtggcggttt taggagttgt 2400
tgttcgggtt gttcgggttt tggttgttgg gttcgcggtg gttgttcggt ttcggatttt 2460
gtttttttgt gtttttcggt aattcgttgg attattttgt tttcgtgtag cgcgtttttt 2520
tcgttttcgg gatttgggga tttcgttttg tgtgtgtgta gcgggtagcg gtcgtagtag 2580
ttttttgatt taatagatga gatggaagaa gaaaaaaaag aatttttttt atttggagaa 2640
agaagaggag gaggggaggg gagggggtgg agggggagag agcgagcgaa agagaaaaag 2700
gttggagttt tcgttttcgg ggttgttaga tggtttgggt ttttgcgacg cgattggttc 2760
gcggagggta gaaattattt agtaaatatg attattatta gttgtttagt aatagtttat 2820
taaagtagag agattattta ggtaggtaat ttagtgtgtg tattttcggt ttcggggtag 2880
tttttgagag cgttaatttt ttcgtatgta atatttttat taaggaatgt tttttttttt 2940
ttttttttta tttttttttt ttttaatagt gttttttttt tgtgggatgt ttttgcgcgt 3000
atatacgcgc gcgtacgtat atatacgaat atttgtttcg cggtagatac ggggggaaat 3060
gtaatatttt tttaagcgtt taaataattt ttgaaatttt ttaaagaaaa gttttttaga 3120
ggtattttgg ttttaagttg tagtaaatat tgggaggttc ggttcgtatt tttaggtttg 3180
tattaataat gatagcgtgt tggatagtgc gttagtgtgt gttagatttt tttttttttt 3240
tttttttttt tttataatta aagggaagat ttaggttttt gtagggaata acgtttcgta 3300
ttaagataaa tagaatggaa agttaaagag gaaagtaagg acgttgggaa aagttatttt 3360
ttttaaaatt cgtttgtttt ttagtcgttt ttttttttta atttaaaaaa aaaaaaaata 3420
tagttttggt ttgaaggagt tagtgtttta taaacgtgga atttagatat cgaagtagtt 3480
ttgtagaggt agaggcgttt ttatttgaat ttcgaatgtg atataagtaa aagagaaata 3540
atagaatttt tttttataat ttagagaaac ggaattgttt ttttgagaga gtaaagtttt 3600
tttgattatt ttgggtttat ttgtatattt ttttttatta attagatgtt tatatagtta 3660
gtattttttt ttataaaatt cggtatgtat gttatattta ataatagtat gacgtaggag 3720
attataaatg tttcgatgtt ggatttattg aaaataaatt gtgcgcggat cgcgaggatt 3780
cgtttcgata tcgcgtttag tgttattaag gtatttcggg gattcgtttt tcgcggaggg 3840
ggaaaatacg cgattttttg aggtggattt tggagttaga tacgtttata taattaggtt 3900
ttttatttaa aaaaattttt gttttcggaa aagtggaatg tgtttttttt acgtttttaa 3960
gttattacgt ttttttttgc ggtaggaatt cgattcgtaa atttttgatt tgttttattt 4020
tatttttttt tttaaacggg gttgaattgt agaggattgg gtagtagcga gaagtttagt 4080
gaagtcgtga aatggggcgg tgtggtttta agatgcgtac ggtgtatgtt tattgaaaaa 4140
aaaagaagaa agaaaggaaa ggaaagaaag aaatttagat ggagggaaag ggggaggagt 4200
agggagaagg cggagtttcg gtggttacgt gatttcgcgt taggaggttc ggtgcgcgcg 4260
cgcgttcggt tttcggattt ttcgcgttcg cgcggtcggg gtagagtagg tagggaggcg 4320
gatgcgtcgg gtcggtttcg ggtttttagc gcggtttttc gagagtttag gcgaggcgcg 4380
cgtttagtcg gcgcggacgg acgtgtcgga cgcgtcgtag tatatttttg aaggcggatc 4440
ggttttttta cgttgtttaa tgttcgcgtc gttttttcgc gatcggttaa tggtcgcgtt 4500
tacgagtttt tgtttattta gcggtttcgc gttggttgtt cgggcgcgtt gggggttgcg 4560
gcggggcgcg tcggttatcg ggcggtttta cgcggtttc gtaggggggtt cgttcgtcgg 4620
tttttttcgt tttttttttt ttttttcggc ggtttttttt tttcgttttt ttttttcggt 4680
ttgcgttttt tttttttttt ttcgtttcgt tttttttttt aatttttttt tttttattag 4740
tttggttatt ttttttttag tttttttttt ttttcggttt tagggggagg tttttttttc 4800
gattttttaa ttcgtaaatc gtatgcggtg tcggggttag ggcgggatgt ttcgaggaag 4860
agagggagac ggggtggagg tgtttggggg cgttcgcggg attcggaggg ggatttgagt 4920
atcgggttag cgcggaaatt taaaatttag atcgtttaag gagtacgtga ttttgtgagt 4980
gttttaggga agggttgcgg aaattcggaa agtaggcggg tagtatgcga ggaaaatgat 5040
attgagttga taaagagttt agacggtttt atatattgtt agttttattt ttgagtacga 5100
ttttgatgtt tattaggttt gataaatagt ataaaatagg aaaagaggtt tattatttta 5160
gtttttttta ttgaatggtt atgtcgatat tttaagtcga attattgtgt gtttgttttt 5220
ggaaattgtt ttttaaaaat tgggggaggg gggtagtatt tttagttttg tatgttagtt 5280
tttttatatt ttaagtttgt tggagggtaa ggaattatat agttaaagat acgttgttat 5340
gtttgttttt ttaaattttt ttattacgtt ttggtttaaa attattagtg tttcgtttta 5400
attttttatt agagggattt tagtatagtt tgtttggaag aaggaatttt ggaaggaaat 5460
ggtaagttgt gtgttcgtaa tggattcgtt ttatttttcg tacgcggttg tatgtttttt 5520
ggtttcggcg gttgttatat gtacgttatt ttagtggtaa agtttttttt atttaggttt 5580
atatttttta gtttgattta atttttaaac gttttgaata ttgattttcg gagtttttag 5640
tgttttgagt tttttcgttc gttgtttgat gggttaattt tttagtattt tagataatgg 5700
tttcggtttg taaattttat tcgtgggttt taggacgtat ttcggagttt cggttttaac 5760
gagcgttatt ttaatttttt tattttttat tcgtcgtttt ttttgtggtt tgtttttttt 5820
atatttagag atgaattatt ttgaggattt aattggtttt gtttttgtaa cgttttaaga 5880
aagggaagag gagttagtgt aaaagtttgt tcgatttggg tggttagggt tgaggttgtt 5940
tttaatttgt tttattgttg atgtatgcga gtgcgatatt tttagcgata atgttgtttt 6000
taattaattt gtttttattt atatttgtaa gtattgtcgg ttttaatatg tatgttttgt 6060
gtaggggatt gttgtgttat atcgggtttg ttaattttaa ttaattataa ggattttata 6120
gatttgagat gtagattggg gtagttagat ttgttaaggt gtttttcggg aagatagttt 6180

ttttaaatgg tttttttgtt tttggtattt tttgtgatag ttattaaata aaaataagtg 6240
agaatttggg gttttattag ttagtaggag ttgttagttt ttatgaagta atagttgagt 6300
gttttatttt ttttttttaa gtaaagttta ttttaatgta taaattgtta ttttggtttt 6360
ttagttatga tttaagattt tttattggag tgtgtagttt ttaaagtaga tgttttaagt 6420
ttttcggatt tttttatatt tttttttttt tttgttttaa tattataaat atttatggtt 6480
atattatata tattttaaaa atgatattag attatttaaa tgatgataat gaaataaaat 6540
atttgttttt tgtgttggga tgttttaatg gtaagtaatt gtggaagtta gatttggtgg 6600
agttatgaaa aattatagtg agatttaatt ttttattggt tttttagatt aaattaaatg 6660
agtttttaaa atttaatgta atttggtaag ttttgtagag tataaatgaa taaaggggtt 6720
aatgtatttt agtaatttga ttttggggtt atttataatt ttg 6763

<210> 215
<211> 13508
<212> DNA
<213> Artificial Sequence

<220>
<223> chemically treated genomic DNA (Homo sapiens)

<220>
<221> unsure
<222> (7911)

<400> 215

gaggggtatt aaggtggtgg taagaggtat ggggatggag ggttttagtt gttcgggttt 60
tttttgtgtt aagttttggt ttgggtgttt tttaggattt tttgtttgaa atcgtatagt 120
tcgttgttga ggtaggtagt ttataggcga gtaaatagat gtttagagag gttaagtgat 180
ttgcgtaagg ttatttagag tggtagagtt gagatttgat tttttcgatt tttggttttt 240
tttttatttt tcgtggttta gaaagttttt attttttttt ttaggtgtgt gattttgggg 300
tgtaacgcgt gggtagttag ggatgttgat gttggagaag ggattttggt attttttgag 360
ggtgagagcg gttttagtgg gtaggagtgt ggggttggtt gtaggtgttt tagtttttta 420
tttttttttt tgttgtgttt aggttaggtt ttatatattg gaggtgtggg tttgtggttt 480
tttttttttt aggggagggt gttcgtgggt agtggaggtg gttggtatag ggtggaggtt 540
ggatgtgggt agtttgtttt tttttttttt ggtattggtt tcgatttttt gtcgaggttt 600
tttttatttt tggggtgtag gggggttggg gtttaggtgt ttttttgagg gttatagttt 660
ttgatggatt ttttgaggta gagtttgtgt ttttttgggt ttattttggt tatttttatt 720
tttgtttatt tttgtttttt ggtttttttg gtttttggtt tatttttgtg atttttagtt 780
tttttgcggt ttaaggtttt ttttagggag ttgagtaaat ttgtattgaa atttgtaggt 840
agagttgggt taggtttttt ggaggttttg ataggggttt ggggaggttt tttagttttg 900
ttttgtagtg tgtgtgggtg gtgggttggg ggtgaggtgg gggtagtggg gattgaagtt 960
tgatttaatt ttaaatttta ttggggtaga gttgaggtgg ttgtggtttt tttttttttg 1020
ggagtaaggg tgggttttag agggtttagg ttggattgat taaattggtt tttcgatttt 1080
taaaggttgg gttaggatgt ttgagatttg gagtgatggt tttttgggtt ttagcgtggg 1140
ttatagaggt tttattttgg gttagttgtt ttttgggttt tattttgaga gaggtaggtt 1200
tttgtggatg tgaatttatt tgagtgtttg tggatttggt gtatgggttg ggtttatagg 1260
gaggatttta gagtattttt tggtagaagt agtaggtata agggttattt tggttgtgtg 1320
aggttcggag aatgtatagt ttgggagatt gtggggttgt ggttgtgttt ttggggagtt 1380
gtgtattggg gtttatgtat gtagtttttg agttgtgttt attgtgtggt ggagtttagg 1440
tgtggagaga tggtattttt aggttttttt tttttatatg tatgtttgta tatatatagg 1500
ttggggttgt aggcgtgttt ttgtttattt agatgttttt tgtagatttg tatgtatgag 1560
tttgtatgtg tatttgtgtg tgagggttta attgtaggaa ttgatgaggt tttgttagtt 1620
tttttttttt ttgtatggtt tttttagttt tagttttcgt tgtgtggggt tttataggag 1680
ttgggagggg gttagggcgg taattggttt agtgggtggg gtttttgttt ttttgatatt 1740
agttttttta gtttaggagg gaattggtag gggtttgggg taggaaggga gttttttatt 1800
taggagtgag gagagaggtt ttatgattgt tataatgagt aatagtaaaa atcgtggtta 1860
ttagtaaggt ttagatttta tttttggggt agtttttttt ttttagttta gtatttgttt 1920
ttttttattt tttttttttt agttttatat aggttggttg gtttagttgg gttagaattt 1980
ttggtttttt ttatagtggt tttggtagga attttttttt tttaggtttg gtttttaatt 2040
aaatgttggt tatgtgtttg ttttttggat tttagatttg aggttagagt ttggagttta 2100
gagtttggaa tttagtgtag tttgagtttg gggtggagtg gtaaggtata gggggtgttg 2160
gaaatggata gttttttattt tgagtttttg tggtttgttt tttttttttt tttttttttt 2220
ttttttaaaa tagttttatt gagatataat ttatatatta taaaatttat ttattaaagt 2280
gtataattta gtggttttag tatatttaga gttgtgtaat tattattata attaatttta 2340
gaatattttt attattttag aaagagtttt tatatttagt aatagttatt ttttatattt 2400
tttaaattta cgttttagtt tttggtaatt ataagtttgt tttttgtttt tataaatgtg 2460

tttattttgg atattttata taaatggaat tttataatat atggtttttg taatcggtat 2520
aatgttttta aggaatattt atattatagt attagtattt tatttttttt ttggtagaat 2580
aatgttttat tgtagggata tataatattt tatttattcg ttagttgatg gatttttggg 2640
ttgattttat tttttgatta ttaggaataa cgttgttgtg aatgtttatg tataagtttt 2700
tttgtggatt tacgttgtta tattttttgg gtagatattt aggagtagaa ttgttgggtt 2760
atatgttatc gttgtgttta attttttgag gaattgttaa attgtttttt aaaatagtta 2820
ttttatttta tatttttatt agtaatatat gaggatttta atttttttat attttcgtta 2880
gtatttgtta ttatttgttt tttttattag agttagttta gtgggtatga agtggtattt 2940
tattgtgttt tttttttttt tgagatggag ttttattttg ttgtttaggt tgaagtgtag 3000
tgatataatt tcggtttatt gttttttagg tttaagtaat tttcgtgtat tagttttttg 3060
agtagttggg attataggta tgtgttatta cgtttggata atttttgtat ttgtgtgtgt 3120
gttttttttt ttttggagac ggagttttgt tttgttattt atgttggagt gtaatggtgt 3180
gattttagtt tattgtaatt tttatttttt tggtttaagc gatttttttg ttttagtttt 3240
ttaagtagtt gggattatag gtatttgtta ttatgcgtag ttaatttttt agtagatttt 3300
tgtatttttt agtagagatg gggtttcggt atgttggtta ggttggtttt aaatttttga 3360
ttttaggtga tttatttgtt ttagtttttt aaagtgttgg gattataggt gtgagttatc 3420
gtatttaatt gattttcgta tttttagtag agataggatt ttgttatgtt ggttaggttg 3480
gttttgaatt tttgatttag gtgatttatt tgttttagtt ttttaaagtg gtgggattat 3540
aggcgtgagt tatcgttttt agttttattg tgtttttgat tggttatgtt tttttttgtt 3600
tgtttttttt tttttgtttt ttatatatgt atttttttata tataaatata tgtatatgta 3660
tttatatata atttgtattt aaaatggatt tgaaatattt ttttgttttc gtatggatat 3720
gtttttcgtg tttttttttt ttgtttttgt tgttgtttat ttgttttatt gtgttatgga 3780
atttttgggt ttatttttgt ttttgttttt attttatggt attatttgtg tatatttttt 3840
ggtgttagtt gtttaaatag ggtgtggatt tgtatttata ttggtgaagg gggtatttgg 3900
ttttttattt tttgtttatt ttttatttta gtttatgtgt tttgtttttg tagatagaag 3960
tatgtgggtg aattttgaga ttaatttttt ttttattgtt tgggggtagg aggttttttg 4020
ggcggttttt gtttttttat atatcgttta tttttggatt atttatgttg ttaagtattg 4080
gaggtgatat ttaggagttt ttggttttta tttttttatt agtgtgtacg taaatgggtt 4140
tttttttagg gtttgtggga taaattacgt ttgggtgttg gggtttggat ttggggggga 4200
agtggtgtaa gaggggtttt tggtttagga gttatttttt tgagaagtgt taatgtttta 4260
gggaagcggt tttttttttt tttttttttt tttttttagt atacgttttt tgtattttga 4320
ttttaggtaa tttttattgg gggaaggagg gttagttgtt cgtttttgtt gtagtgattt 4380
tttgaggtgg tattgtggat tttttttttt taggattgtg ggtgagaggt tttttaaggt 4440
tttgggtttt gtttttgttt ttttagtttg gttgtgttat ttgttatttt tagtagtgtt 4500
tgggttttgt ttttaggtta tttttgttag ttaggtatgg tgttagttgt ttgtttagtt 4560
gggtgttacg ggaaaatttt cggtagttta ggttggggag ttttacgggg aatgtttgtg 4620
tgtttgtttt ttagtgttta tttatttgtt tttttttttt ttgtagtatt ataggatatg 4680
gtttttttag ttatttagtt ggggtttatt taggagtggt attttttttg gtgttttgaa 4740
ggttagtttt ggattttttt aggaaaagtg ttagtttata gaattgtttg attaaaggat 4800
cggtttttga gatatttttt aatttatttg gtttttagtt agggtggggg ttttaggaga 4860
ttgagattag tttgtttttt ttggatagta gttttaggat agggcgggtg ggttgattat 4920
ttaaatttta tttgggttta ggttttatgt ttcgaggagg ggtggtttga agtttattag 4980
agttttttgt tagattgttt gttttttttt tgattgtggt cgtttggtat ggttagtaat 5040
agtagttttt gttcgatatt tgggggcggg tattttaatg ggtattcggt gtttttttac 5100
gttttttttt ttttttttat gttgggtgga tttttttcgt taggcgtttt gattattttt 5160
tagtattagt ttttagttag tggatatagt atattatttt tagttagtaa gtttgggtgt 5220
gggggttggg gtgggaaggg attgtggagg gtggtaggtt ttagagtttg ggttttgggt 5280
acgtttgttt tgttgtgagt ggaaggtacg gtgagcgata aggttttttt tagttggggt 5340
gattattatt tgaggtttta aaaatgaaag ttttatggtt tataggttga ttatggtggt 5400
tgtgtttgaa gttgggggtg gtagaggggg agatgaaatt atgttgtttt ggtttgtagt 5460
ggttttcgta tttttttttt gtgttttgtt gttggggttt tataaatagt tttaaggatt 5520
ggatatatag gttggaggtg ggtaaataaa ttttttgata tttgatggaa atggtagttt 5580
tatttagagt gagagtagta tattatttac gttttatttt tatttagtgt ttttgaggga 5640
aagggtttga aggagtagat cgaggaaggt tggttggaat tttatttttt tttttgttag 5700
ttttattttc ggttatgtta ggatgtgttt ttttttgtgg ggaggatgag agggaggggt 5760
ttacggttta gttgggtgga tagggtgagt gttttaggag gggatatttg gtttggattt 5820
tggtttcgga cgtatgtatg gttttggtag ggtttgtgtt ttttttgttt ttattttttg 5880
ttatggaggt tatttgttgg ttgggtaggg gttttagtat ttttcgtcgg ttggtcgagt 5940
taggtagtgg gaggtggcgg tagttttggt tagttggtag agtttagggt tggggtttgt 6000
taggtcgttt atagtaattt tggtttttag ggtttagttg ggcgttaggt tagggttggg 6060
gggaggaggg agtcggggtt tggtttggga cgggagaggg tggaagtagt agaatgggtt 6120
atgggttgtt ggttggtcgg ttggttagtt gttgatttgg gaaacgtgtt tttatttttt 6180
ttggttagta gggattgtat attttgtttt gtgtatttat tatttttttt acggttgttg 6240
gtaagttgtt tggtattatt gttttatgtg tttggttggg ttgggttagt gttttttttt 6300
tagtgtgggg gtagcgtttt tttttcggag ttaggagttt tttttgtttt tggggtaggt 6360
tttggttggg cgttttattg ttcgtattgg ttgggtgttt attattatta ttgttttttg 6420

gatcgttttt tttggtttag ttgtggtttt ttttttttta ggttttatta gttattagga 6480
gtagggattt tatgtttgtg tttttttgtg tttttcgttt tttatgtttt ggggttaggg 6540
ggtaggtagg tgttgtttta gttttttttg ttatttttta ttaaattttt attttttgtt 6600
tttgttgttt ttttttttta ttttatatag gagtatggga tggatggagt ttggggattt 6660
tgattttggt ttggttttat agtttgtgtt agtgatgggg agaggttagt ttttggggtt 6720
tggggttatt agggaatgta ttggtgtatt tttgtagaat atttattgta tttaaaggga 6780
ttgtgattta tattttggat ttggattatt ggaattagaa gattgtttgt tttttgatag 6840
ggtagtttag cgagagttta gtcgtattgt attaggtaaa tgaggttcgg tttgggtggg 6900
ggtgtgtgtt aaggggagga tatcgggatt attttttttt ttttcgtttt attatttttt 6960
ttattacggt ttcgttcggt tagggattga ttaaattttg ggggagtttg ggagtcggaa 7020
ttggtataag ggaggacgtt cgtttttttt tcgtttttgt tttttcgtag tttttttttt 7080
tttttgtatt cggcgttttt ttgtattttg tgtatttttt atttggagtt tttttttttt 7140
tttgtttttt tttttttttt ttttttttta ggttgttttt atttgtttgt ttatatgtcg 7200
tttttttttt tcggtttttt gcgttttttt cgttgtagtc ggacgcgtcg ggaatgggtt 7260
aagttagggg cggtgtttgg acggggcggg gcggtggaaa gggggtggtg ttcggagggg 7320
agggggcgcg tagagttggg gtgggggggt cgtggcgcgt attattagag atcgagcgag 7380
tcgttagttg tttttggttt ggcgggggcg gaatcgcgcg ggatttttat ttttattcgg 7440
aattttcgtt acggagaatt tttggagaag tttcggattt tcggttggga ggaggaagtg 7500
ttcgttgatt tttagtttcg cgttgatttc gttttcggtt tgcggatttg gggagtcgtt 7560
gtattttgtt tcggacgtta cgagatttta gacgggagtt ttttcgaggt gaagtcgttg 7620
agttttcggg tttcgttagg tttttttggg agagtcgacg gatttttttt tttagtatat 7680
ataatttttt tgttttttat cgggattggc ggagcggtcg gcggatttag acgcggggat 7740
tttagggtag gggcgttttt ttgttcgggt tattagtcgg ggcgagggga cgtttttttt 7800
tttttagttg ttttgttcgg atggcgtcgt cggttgagtg acgggggcgg cgcgtaggat 7860
tttttagttc ggattttttg ttttcgaggg gaaagatgta cgagagtgta naaatggggg 7920
gttttatttt taattttttt ttagtggtgg atttttataa ttagaatcgg gtttgtttgt 7980
ttttagagaa ggggtttttc gtttcgggtt cgtattttat ttcgtttcgg atttcgtttt 8040
ggaatggttt aaattattgt acgtatcggt tttttagttt gttgttgtag ggggtgggag 8100
tgggcggtag ggtttttatt attattcggg ggtgagagtt tcggggtgta gtggaggttt 8160
tgtttttatt ttttatttaa ttcgtgttgt ttttgttgga taattgaatt ttttcggtcg 8220
tatttttttt ttagtaattt taagtgtaat ttgtgttaga ttagggttga ggaattttga 8280
gagttttttt ttttaagtaa tattttttta ttttttgttt tatttttttt aggagaaatg 8340
aagtttaagt tttttgtttt tagtttatat ttttttgttt ggaatttaat agttttatag 8400
taggttggag ggtggggtaa aggttttgtt tgtgtgggtt tgtttgtttt cggggaaagg 8460
ttttttcgtt agattttatt tgttaaagtt cgagggagag tttttttttg tgcgatagtt 8520
gttgttttta gtttttgttt ttgcgttatt agagtgggtg ttaggggagt atttagtgtt 8580
tttggtattg atagtggggt gtggtagaaa ggggtaattt tattagatat tcgtttgtta 8640
ttttaatttt ttattttggt aaggggtatt gggtttttat ggggttgttg ttttggtttt 8700
agatatttgg ttgttatttt tgaggttttt atttttagga gtggagggga gaagttgttt 8760
tggtgagaag aatttttttt tttttaattt taattcggtt tgtttttggg ggaatgagtt 8820
tgtttttgtg ggtttggggt tgttttggaa atgaggattg tatttttttt tagtttcgtt 8880
atcgagtttt tttgttattt tgattttatt tggggatagg gttaggtttt gggttgtgtt 8940
tttttaagag gggtattgat gagaatttta gtattttggt agtttggtta tttttttttt 9000
ttgttgtttt ttgtttgggt gtaggttttt ttgggaggaa gggttttgtg tatatttagg 9060
agttcggagt attagggtgt atatttgggt atttttttgc gtagttgtga ggtagtgtat 9120
attgggtggg cgggagtagg cgtaaggggg ttattgttag atggtttagg tttttttttt 9180
ttttgggttt tgggtttttt gttggagggg aagttttttc gtggaggatt ttttattttt 9240
ttggatttgt tgtttttttt ttgtttgtta gggaggaggg gtggagtggg tttcgggggg 9300
gttttggtag attggagaag gttgaagggt aaaggattta ttttattttt tttgttggga 9360
gaagagagat ttgagatgga tagatagttt atttttgttt ttttagagtt atttttattt 9420
tagttttttt tattgttttg ttttcgatta ttttttttag ggtcgaattt gttgtgtggt 9480
tgtagatata agagggaagg tattattttt gattttggaa gaagagagag tgagaggatg 9540
attttaggat tttttttttt atttttttag tgttggagta agattttttt tttttagggg 9600
gatagttgga gtagggttgt tagagttatt ttttttattg ttttggttat tttttttaga 9660
gggttggaga gaagttggga tttgagattt tggtttttag tttttgtttt tttttagttt 9720
tatggggata gtttagtttt ttttggttta gaattggaga gggaggagga ttatagagag 9780
taggataggt agtgtattgg tgagtttttt ttttaaatta ttggatatgg ggaagtggag 9840
atttgttttt atatttattt tggggtgggg tagtagattt agaggtttgg gtttttagtt 9900
ttcgtagttt gagcgtgggt gtgtatatat aagtgagtga ggtgttagtg gattcgggtt 9960
ttgaggttgt gaggttggga gtgatggggg tttgggggtt tgttttgagg tataggaagg 10020
attcggagtt tgagggtggt aattagattt agtttagaat atgtggggtt aatgttatta 10080
ttttggaagg gtttttttgg gtgtgtggaa gttcgttggt gattggagtt gtttttagtt 10140
tttttttggg gaatttttta tttttttttt tattgttatt gaaggtggga agagtaggtt 10200
ggttttggga ggaggtggtt tgggttttgt agggttttag ggatattgtt ttttttttta 10260
gagtttttat ttcggtgtat tagaggataa ggggggtgta taggatgtgg ttttttattt 10320
gttttttatt aattttcgtt atttatattt tcgttcgttt ttagacgttt aagaatgtga 10380

agtacgtgga tgttcgtagt tgggggaggg ggagacgttt attaggcggt cgttgggtta 10440
ggggtttttt ttcgttgtcg cggtatattt agagttattt tcgttttttt tcgggaggag 10500
gaaggacggt atagagggtt ttacgttttt tttttaatta tttttagggg ttgcgagggg 10560
agttgcggag gagcgggcgt tagttggatt gggaggggag tcgttggtcg ggggttcggt 10620
tgattttttg ttgatttttt ttaggaaatc ggttttttgt gcgagtttgc gaacggttcg 10680
ggggcgtggg gaattcggag tggagcgttt tgcgtcgttc gttttgttag gatggggagc 10740
gagggagggg tattttggta gcgtcggcgg gaggggacgt ttggtttttt gggttagttt 10800
tagttttttg ttgggcgttg gaattttgag ttgagaaggt gtggtttttt tttagttcga 10860
gtttttttgt aggaagagga gagattggtg ggttgggttt ttggggaggg aggttagtag 10920
ggatgggtta ggttcgggta gttttttttt cgttggtgtt tttttttatt ttatttgtgt 10980
gtgtaaggag ttatggtcgt gttttaattt ttgtagaggt tgtgaggatt tcggagttgt 11040
ttatattttc ggttttggtt tttgtatttt atttttttgg attgttggtt ggaggtttgg 11100
ggaggtgggg tatcgagttt tgggtttaaa gggtagagta gggaaatttt agagttgggt 11160
tatttgggtg ataggtgggg atgtgttgga ggtagggggt aggttatgtt atagttttta 11220
aggtagttaa gttgtcgttg ggtgggttaa aaggaggttt tgtttagttt aaattgtagt 11280
ttttgttttt ggttattttt tttattttgt taaaaaataa ttttaaaaag tatatttttt 11340
tagtttcgta aatatttttt gttggatttt gttttagttt tatgttttta tggttttttg 11400
ttttttagtt tgttttaggt tttagagttg tttatttttt attttggtat tttttatgat 11460
tttttatatt ttagtttttt tcgtgatatt tcgttttgta tttttaaagt ttttttagtt 11520
tttttttttt ttttagtttg gtttatttta gaagtggggt tttgggagag gcggggttta 11580
gggtaaacgg tggattagga ggggtgggga ggttagtgtt tttttttttt gtttgtcgga 11640
atgttgatta agattttagg ttatggtttt tttaattttt tatatatttt tttgtttttg 11700
attttttttt tttttattag tttggattgt ttattgttat tgtttttacg ttttggaaaa 11760
agttagtata ataaagggtt gttttgtggt ttattttttt tgttttttgg ttttatttag 11820
gttttttaat ttcgtttttt tagtagttgt ttttaggttt tttagtttgt ttgatttgtt 11880
tgtttggttt ggggagaatg aggtgggaga aaattaggtt agggtagttg gtgttggagt 11940
gaagagtaga cggcggtggg gaggttagga gagaatttgt tgggttgggg atggtgtggg 12000
tattaattgt tttattgttg taggttggtt ttggggtagg gaaggggatg gggggttata 12060
gtagtgttgg ttagttaggt tggtttggga agtggtgttt aggtattatt aagagttagg 12120
aaagttttgt taaggttgtt ggtttagttt tttgttatta gtcgtttagt agtttttatt 12180
gtgtttgtag gtaagggggg agggtggtag tatatagtta gtttttggtg tttttatgtt 12240
tttttttttt gtgttttaat tttagggtta tgtgatttgg ggttatgtga tttatgtttg 12300
taaggtgttt gggttaggag ttgtgggtat ttttaaatgt tagttagttt tatgtgtcgg 12360
agtttggggt agggttaggt aggattgtgg aatatgggag gaggtaggga tttgtttatt 12420
tagggaggta tttttattta tttttggttt tggataagag aatttgaacg ttggtagggg 12480
ttttaggacg atgttgcgtg gttttttggg aatttgggat tgttttggtt atagttttta 12540
ttttgtattt aatattttta gttgtttagg ttttggatat ggatagtttt tatttaattt 12600
agttttgttt tgtttatagt gatgggtatg gagttagata ttggggagga tttggttagt 12660
gagggttgtt tttgttgttt gggttatttt ttttggttgt ttttttggag ttgaataata 12720
gaaggggagg ggttagtaat tcggatatag tattgaggtt agatagatag agtattgatg 12780
ggaatagatt ttttttgtta tgttattttt ttttagatgg ggggtattta gaataatggg 12840
tttttggggt tttggggatt tttttttttg tatttagggt attttttttt attttaggga 12900
gatatttttt attgtgttta gtatttgtga tttttttttg tattttttgt tttgggtttt 12960
tggttttggg attgtttggg tggaggaggg gtagtggttg ttggtagaat ggggtggagg 13020
ggggagcgga agtagagggg gcgggggagt ggtcggtttt gaatattttg ttgattttag 13080
ttttttttgt ttttagttta tgtttttttt tttttttttt ttttaagcgt taattttttt 13140
ttaattcggg gggagaacgg ggttaggtcg tttaggggta ggagttttag aattagggtg 13200
atttttattt ttatttttta agtatagtta cggtatatat ataaatgtga tggtttatta 13260
ttgtattttt gtggttttga aggtgggggt tttaggagtt tagaggagtg atgggtgttt 13320
ggaggtttta ttggtagttt tttgttttga gtttggttgg ggagttttag tttttttaag 13380
atttgaattt tgttagtagt ggtgaggttg ggagaggttt ttaggaggga cggtgaggta 13440
gggtggagtt tggtattaag gatggcgatt taggttttta attgtttttt tttttttttt 13500
tttttagt 13508

<210> 216
<211> 13508
<212> DNA
<213> Artificial Sequence

<220>
<223> chemically treated genomic DNA (Homo sapiens)

<220>
<221> unsure
<222> (5598)

<400> 216
   gttagagaga gaagagggga ggggtagtta gagatttagg tcgttatttt tagtattaag 60
   ttttattttg ttttatcgtt ttttttaaga gtttttttta gttttattat tgttggtagg 120
   atttaagttt taagaaaatt gggatttttt agttagattt agggtaggag gttgttaatg 180
   aagtttttag tattttatta tttttttgga tttttaggat ttttattttt aaaattataa 240
   agatataatg ataaattatt atatttgtat gtgtgtcgtg attgtgtttg gggagtaggg 300
   gtgggggtta ttttgatttt aaagtttttg tttttgggcg gtttggtttc gttttttttt 360
   cgagttagga aggagttaac gtttgaagag gagggaggga agaggatata agttgggggt 420
   agaggaaatt ggggttaata ggatatttaa agtcggttat tttttcgttt tttttgtttt 480
   cgtttttttt tttattttat tttgttagta gttattgttt tttttttatt taaataattt 540
   tagggttagg gatttaaggt agggggtgta aagaagagtt ataaatgttg ggtatagtag 600
   gaggtgtttt tttgagatag ggggagatat tttgaatata gggagaagag tttttagggt 660
   tttaaaagtt tattattttg ggtatttttt atttggggag agatggtatg ataaaggggg 720
   tttgttttta ttaatgtttt gtttgtttgg ttttaatatt atgttcgggt tattaatttt 780
   tttttttttg ttatttagtt ttaagagggt agttaggagg ggtgatttag atagtagggg 840
   tagtttttat tggttaaatt ttttttagtg tttggtttta tgtttattat tgtaggtaga 900
   atagggttgg gttgggtagg ggttatttat gtttaaagtt tgggtagtta agggtgttgg 960
   gtgtaagata agaattatga ttaggataat tttagatttt taaggggtta cgtagtatcg 1020
   ttttgaggtt tttattaacg tttaagtttt tttgtttagg gttaaggatg gatgaggatg 1080
   tttttttagg tagatagatt tttgtttttt tttatatttt ataattttat ttagttttat 1140
   tttaaatttc ggtatatgag attggttggt atttaaaggt gtttatagtt tttggtttaa 1200
   gtattttata gatatgagtt atatagtttt aaattatatg gttttaaaat tggggtatag 1260
   aggaaggaag tatgggaata ttaggggttg attatgtgtt attatttttt ttttttattt 1320
   gtagatatag tgggggttgt taggcggttg atgataggga attaggttaa taattttggt 1380
   agggtttttt tggtttttag tagtgtttgg gtattatttt ttaggttagt ttggttgatt 1440
   agtattgtta tggtttttta tttttttttt tgttttaaga ttagtttgta gtaatgggat 1500
   agttgatgtt tatattattt ttagtttagt agattttttt ttgatttttt tatcgtcgtt 1560
   tgttttttat tttaatatta attgttttgg tttggttttt ttttatttta ttttttttag 1620
   gttagataag taaattagat aggttgagag gtttgagaat agttgttggg ggggcggggt 1680
   tggggggttt gggtgaggtt aggaggtaga gggggtgagt tataaagtag ttttttgttg 1740
   tgttaatttt ttttaagacg taaaagtagt aataatagat aatttagatt ggtgggggga 1800
   ggggagatta agggtaaggg ggtatgtgga gggttggggg gattatggtt tagaattttg 1860
   gttagtattt cgataagtag aggaagaagg tattgatttt tttatttttt ttaatttatc 1920
   gtttgttttg ggtttcgttt tttttaaggt tttattttta aaatgaatta gattggagag 1980
   ggagaaagga ttgagggagt tttgggggta tagagcggga tgttacgagg ggagttaggg 2040
   tatggggagt tatgggggat attaggatga gaggtaaata gttttaaggt ttgggataga 2100
   ttagagggta aaaagttata aaaatatgga gttaaagtaa agtttaatag agggtgttta 2160
   cggaattgag agaatgtgtt ttttaaaatt attttttggt agaatgggag agatgattag 2220
   aggtaaggat tatagtttag gttgggtaag gttttttttt agtttattta acggtagttt 2280
   gattgttttg gaggttgtaa tatagtttgt tttttatttt tagtatattt ttatttgtta 2340
   tttaggtaat ttagttttga ggtttttttg ttttgttttt tgaatttaga gttcgatgtt 2400
   ttattttttt aggtttttag ttagtagttt aaggaggtga ggtataagga ttaaggtcgg 2460
   aggtgtgggt aatttcggaa tttttatagt ttttgtaaga gttaggatac ggttataatt 2520
   ttttgtatat ataggtggag tggggaggga tattaacggg ggagggattg ttcgggtttg 2580
   gtttattttt gttaattttt ttttttagag gtttagttta ttaatttttt ttttttttgt 2640
   agaaggattc gggttagaga aggattatat ttttttagtt taaagtttta gcgtttaata 2700
   gaggattgga attgatttag gaagttaggc gttttttttc gtcgacgttg ttagggtgtt 2760
   tttttttcgt tttttatttt ggtagggcgg gcggcgtaga gcgttttatt tcggattttt 2820
   tacgttttcg agtcgttcgt aggttcgtat aaggggtcgg ttttttggag gagattagta 2880
   ggaaattagt cgggttttcg gttagcggtt ttttttttaa tttagttggc gttcgttttt 2940
   tcgtagtttt tttcgtagtt tttggggatg gttggggagg gggcgtaggg ttttttgtat 3000
   cgtttttttt tttttcgggg agaggcgggg gtagttttgg gtgtatcgcg gtagcggaag 3060
   aaggttttta gtttagcggt cgtttgataa gcgttttttt tttttttaat tacgggtatt 3120
   tacgtgtttt atatttttgg acgtttggga gcgggcggag gtgtgagtgg cggagattgg 3180
   tgggagatag atggggagtt atattttgtg tatttttttt gttttttaat gtatcgaaat 3240
   gagggttttg gggagggagg taatgttttt agggttttgt agaatttagg ttattttttt 3300
   ttagagttaa tttgtttttt ttatttttag tggtagtaaa ggggagagtg gagaattttt 3360
   taagaggggg ttggaggtag ttttagttat tagcggattt ttatatattt aaggggattt 3420
   ttttaaggtg gtggtattgg ttttatatat tttagattga gtttagttgt tatttttaga 3480
   tttcgggttt tttttgtgtt ttaaggtaag tttttagatt tttattattt ttaattttat 3540
   agttttagga ttcgagttta ttgatatttt atttatttat atatgtatat ttacgtttag 3600
   attacgggaa ttggaaattt aggtttttag gtttattatt ttattttaga atggatgtgg 3660
   ggataggttt ttattttttt atgtttagtg gtttagggga agggtttatt aatatattgt 3720
   ttgttttatt ttttgtgatt tttttttttt tttagttttg ggttaggaag agttgagttg 3780
   tttttatgga gttaagaaga gataggggtt ggagattaag gttttagatt ttagtttttt 3840
   tttagttttt tggagaaggt ggttaaggta gtggaagggg tgattttggt agttttgttt 3900
   taattatttt tttaggggag gggggttttg ttttagtatt gggagaatga gaaaaagggt 3960
   tttagagtta tttttttatt tttttttttt ttaaagttaa gggtgatatt tttttttttg 4020
   tgtttatagt tatatagtag attcggtttt agaggaaatg gtcgggggta ggataatagg 4080
   aaaagttggg atagaagtgg ttttgggagg gtagaggtgg gttgtttgtt tattttaggt 4140
   tttttttttt ttagtaagag gggtggggta agttttttgt tttttaattt tttttaattt 4200
   gttagggttt tttcgagatt tattttattt tttttttttg gtaggtagga gggaggtagt 4260
   aggtttagga aggtgggaga ttttttacgg aagagttttt tttttagtaa agagtttaaa 4320
   gtttaggagg ggaagaaatt tgagttattt aataataatt tttttgcgtt tgttttcgtt 4380
   tatttagtgt atattgtttt atagttgcgt aggaaaatgt ttaggtgtat attttggtgt 4440
   ttcgagtttt tgagtgtgta tagagttttt ttttttagag gagtttgtat ttaggtaggg 4500
   ggtaataagg agagaaagtg attaggttgt taggatgtta ggatttttat tagtgttttt 4560
   tttaggaagg tatagtttag gatttggttt tatttttaag tgaagttagg gtaataaaag 4620
   gattcggtgg cggagttggg aagggatgta gtttttattt ttagagtagt tttagattta 4680
   taaaaataaa tttatttttt agagaataaa tcggattagg attaggggga gaggattttt 4740
   ttttattaga gtagtttttt ttttttattt ttggggatga aagttttaaa gatgatagtt 4800
   aagtgtttgg ggttaggata ataattttat aaggatttag tgttttttgt taagatggaa 4860
   ggttaggatg gtagacgggt gtttgatgaa gttgtttttt tttgttatat tttattgtta 4920
   atattaaaga tattgagtgt ttttttgata tttattttaa tgacgtaaga gtaaggttgg 4980
   ggggtagtag ttgtcgtata ggaaggggtt ttttttcggg ttttggtaag tgggatttgg 5040
   cgggggggtt ttttttcggg agtagataaa tttatataga taggattttt attttatttt 5100
   ttagtttgtt gtgaagttgt tggattttag gtaagaagat ataaattggg ggtaaggggt 5160
   ttgggtttta ttttttttgg aagaagtgaa ataagagatg aaggaatgtt gtttgaaaga 5220
   aggaattttt aaagtttttt agttttaatt taatataaat tgtatttagg gttattgggg 5280
   ggagggtgcg gtcgggaggg tttaattgtt tagtaagggt aatacgggtt aagtgaaagg 5340
   tagagatagg attttattta tatttcggga tttttatttt cgagtagtag tggaagtttt 5400
   atcgtttatt tttatttttt ataatagtag attgagaggt cggtacgtat agtggtttga 5460
   gttattttaa agcggagttc ggagcggggt ggagtacgga ttcggggcgg ggagtttttt 5520
   ttttgggagt aaataggttc ggttttggtt ataaaaattt attattagga agggattagg 5580
   ggtgggattt tttatttnta tattttcgta tatttttttt ttcgaaggta agaggttcga 5640
   gttgggaagt tttgcgcgtc gttttcgtta tttagtcggc ggcgttattc gagtagagta 5700
   gttgggggag aggagacgtt ttttcgtttc gattggtgat tcgggtaggg ggcgtttttt 5760
   gttttgaagt tttcgcgttt aagttcgtcg gtcgtttcgt tagtttcggt gaaaagtagg 5820
   gaagttgtgt gtgttgggag ggggggttcg tcggtttttt tagggaagtt tggcggggtt 5880
   cgggaattta gcggttttat ttcgagggga ttttcgttta gagtttcgtg gcgttcgagg 5940
   tagagtatag cggtttttta agttcgtagg tcgggggcgg gattagcgcg gggttggggg 6000
   ttaacgagta tttttttttt ttagtcgggg attcggggtt tttttaggga tttttcgtgg 6060
   cgaggatttc gggtgggggt ggggatttcg cgcggtttcg ttttcgttag gttaggggta 6120
   gttggcgatt cgttcggttt ttggtggtac gcgttacggt ttttttattt tagttttgcg 6180
   cgtttttttt ttttcgggta ttattttttt tttatcgttt cgtttcgttt aggtatcgtt 6240
   tttggtttaa tttattttcg gcgcgttcgg ttgtagcggg agaaacgtag ggaatcgaga 6300
   gggagaggcg gtatgtggat aggtaagtgg gggtagtttg ggaaggggga ggagaggaga 6360
   gaagtaggaa gggggaaagg ttttagatga ggagtatata gagtatagag ggacgtcgag 6420
   tatagggaga ggagggggtt gcgaggggat aaggacggaa gaggggcggg cgtttttttt 6480
   tgtattagtt tcggttttta ggttttttta aggtttggtt agtttttggt cgagcgaagt 6540
   cgtggtggag gaggtggtgg ggcggggaag aggggggtgg tttcggtgtt ttttttttaa 6600
   tatatatttt tatttaggtc gggttttatt tgtttaatgt aatgcggttg aattttcgtt 6660
   gaattatttt gttaggaagt agatagtttt ttagttttag tgatttaagt ttagaatgta 6720
   aattatagtt tttttaaatg tagtgaatat tttataaaga tatattaatg tattttttag 6780
   tagttttaaa ttttagaggt tggttttttt ttattattaa tatagattat aggattagat 6840
   tagaattaga gtttttagat tttatttatt ttatattttt atatgaggtg gagagaagag 6900
   atagtagaaa tagaagatga aggtttgata aggggtggta ggagaaattg aggtagtatt 6960
   tgtttgtttt ttggttttag ggtataaagg gcggggaata taaagaaata tagatataga 7020
   gtttttgttt ttggtggttg gtggagtttg ggagggaggg ggttatagtt aagttagagg 7080
   aggcgattta ggaagtagtg gtggtggtgg gtatttagtt agtgcgggta gtgaggcgtt 7140
   tagttagggt ttgttttagg gataaagaga ttttttggtt tcggggagag agcgttgttt 7200
   ttatattgag aagggggtat tgatttagtt tagttaggta tatagggtag tggtgttagg 7260
   tagtttgtta gtagtcgtga gaggggtggt gggtgtatag ggtaaggtgt gtagtttttg 7320
   ttggttagga gaggtgggga tacgtttttt aggttagtag ttgattagtc ggttagttag 7380
   tagtttatgg tttattttgt tgtttttatt ttttttcgtt ttaggttagg tttcggtttt 7440
   tttttttttt tagttttggt ttggcgttta gttgggtttt gggggttaga gttattgtaa 7500
   gcggtttggt aggttttagt tttggatttt gttagttggt tagagttgtc gttatttttt 7560
   attatttggt tcggttagtc ggcgaagggt attgagattt ttgtttagtt aataagtgat 7620
   ttttatggta gagggtggag gtaaggaaaa tataggtttt gttaagatta tgtatgcgtt 7680
   cgaggttaga gtttaggtta agtgtttttt tttgggatat ttattttatt tatttagtta 7740
   gatcgtgggt tttttttttt tatttttttt ataaagggaa gtatatttta gtatggtcgg 7800
   gggtggagtt ggtaggggga gaggtggggt tttagttagt ttttttcggt ttgttttttt 7860
   aagttttttt ttttagaggt attgagtggg ggtggggcgt aggtggtgtg ttgtttttat 7920
   tttaggtaga gttgttattt ttattaaatg ttaagaggtt tgtttattta tttttaattt 7980
   gtgtgtttag tttttggagt tatttataag gttttaatag tagagtatag aagaggggtg 8040
   cgagggttat tgtaggttag aatagtatgg ttttattttt ttttttgtta tttttaattt 8100
   taaatataat tattatgatt agtttatagg ttatagggtt tttattttta agattttaaa 8160
   tgatggttat tttaattgga gaagattttg tcgtttatcg tgttttttat ttatagtaga 8220
   atagacgtgt ttagagttta agttttgagg tttgttattt tttatagttt ttttttattt 8280
   tagtttttat atttagattt attggttggg gatggtgtgt tatatttatt aattggaagt 8340
   tggtgttgga gagtggttag agcgtttggc ggggagagtt tatttagtat aggggggaag 8400
   aagaaggcgt agggaggtat cgggtattta ttgaggtgtt cgtttttagg tgtcgggtag 8460
   gagttgttgt tgttggttat gttaagcggt tatagttaga agggaggtag atagtttggt 8520
   agggggtttt ggtgggtttt agattatttt ttttcggggt atggggtttg ggtttagatg 8580
   gggtttgggt ggttagttta ttcgttttgt tttggagttg ttgtttaaag agggtaggtt 8640
   aattttagtt ttttggagtt tttattttag ttgggggtta ggtgggttgg gggatgtttt 8700
   aagagtcggt tttttggtta agtagttttg tgagttggta ttttttttgg gaaggtttag 8760
   agttggtttt tagggtatta aaaaagatgt tatttttaga tgggttttag ttaggtggtt 8820
   gagggggtta tgttttgtga tgttgtagaa aaaagaaaga taggtgagtg ggtattgaag 8880
   aataggtata taggtatttt tcgtggagtt ttttaatttg ggttgtcgga gattttttcg 8940
   tggtatttag ttgagtagat agttggtatt atgtttggtt ggtagagatg atttggaagt 9000
   agggtttaga tattgttggg ggtggtagat agtatagtta ggttggggag atagggatag 9060
   agtttagaat tttggagggt tttttattta tagttttgag gggaaagggt ttatagtgtt 9120
   attttaaggg attattgtag tagaaacggg tagttggttt tttttttttt agtgagggtt 9180
   atttgaggtt aagatgtaga gaacgtgtgt tgggggggaa gagggagggg gaggaaaagt 9240
   cgttttttta aagtattggt attttttaga aaaatgattt ttaagttagg ggtttttttt 9300
   atattatttt ttttttagat ttaagtttta gtatttagac gtggtttgtt ttataggttt 9360
   tgggaggagg tttatttacg tatatattaa taggaaaatg ggaattaagg atttttaggt 9420
   attattttta atgtttaata gtatggatgg tttaggggta ggcgatgtgt aggagggtaa 9480
   ggatcgttta ggaagttttt tatttttagg taatgaagga gagattgatt ttaaggttta 9540
   tttatatatt tttgtttgta gaagtaaaat atatgaattg gggtgggagg tgggtagaag 9600
   atgaaaggtt agatgttttt tttattagtg tgaatataga tttatatttt gtttgaatag 9660
   ttggtattag aggatatgta tagataatat tatggggtag agatagagat agagatgaat 9720
   ttagagattt tataatatag tgagatagat aggtagtaat aagagtaaag agagagaata 9780
   cgaagaatat atttatacga ggataagagg atgttttaaa tttattttgg gtataggtta 9840
   tatataaata tatatgtatg tatttatata tgaaaaatat atatatgaaa gataaaagaa 9900
   aagagataga taaaaagaaa tatggttaat taaaagtata atgaggttgg gggcggtggt 9960
   ttacgtttgt aattttatta ttttgggagg ttaaggtagg tagattattt aagttaggag 10020
   tttaagatta gtttggttaa tatggtaaaa ttttgttttt attaaaaata cgaaagttag 10080
   ttgggtgcgg tggtttatat ttataatttt agtattttgg gaggttgagg taggtggatt 10140
   atttgaggtt aggagtttga gattagtttg attaatatgt cgaaatttta tttttattaa 10200
   aaaatataaa aatttattaa aaaattagtt gcgtatggtg gtaggtgttt gtaattttag 10260
   ttatttggga ggttgaggta ggagaatcgt ttgaattagg gaggtggagg ttgtagtgag 10320
   ttgagattat attattgtat tttagtatgg gtaatagagt aagatttcgt ttttaaaaaa 10380
   aaaaaaatat atatataaat ataaaagtta tttaggcgtg gtggtatatg tttgtaattt 10440
   tagttattta ggaggttgat gtacgagaat tgtttgaatt tgggaggtag tgagtcgaga 10500
   ttgtgttatt gtattttagt ttgggtaata gagtgagatt ttattttaaa aaaaaaaaaa 10560
   gtataatgag atattatttt atatttatta ggttagtttt aataagaaag atagataata 10620
   ataagtattg gcgaggatgt ggagaaattg gaatttttat atattgttgg tgggaatgta 10680
   aaatagaata gttgttttgg aaaatagttt ggtagttttt taaaaggtta aatatagcga 10740
   tggtatatga tttagtaatt ttatttttag gtatttattt aagagatatg ataacgtaag 10800
   tttatagaaa gatttatata tgaatattta tagtagcgtt atttttaata gttaaaaagt 10860
   ggaattaatt taaaagttta ttaattgacg aataaataaa atgttgtatg tttttataat 10920
   ggaatattat tttgttaaaa aaagaatgaa gtattgatat tatgatatgg atattttttg 10980
   aaaatattat atcggttata aagattatgt attgtaggat tttatttata tgaaatattt 11040
   aggataggta tatttataga gatagaaagt agatttgtgg ttattaggag ttggggcgtg 11100
   gatttgagga atatgggaag tgattgttgt tgggtatggg ggtttttttt ggggtgatga 11160
   aagtgtttta aaattgattg tggtggtagt tgtataattt tgaatatatt aaaattattg 11220
   aattgtatat tttaatgggt aaattttatg gtatgtgaat tatattttaa taaagttgtt 11280
   ttaaaaagaa gaggaaagaa agaaaagaaa tagggttata gagatttaaa ataaggattg 11340
   tttattttta atatttttta tattttgtta ttttatttta agtttaggtt atattaggtt 11400
   ttaagttttg aattttagat tttagtttta aatttggagt ttaggggata agtatatggt 11460
   tagtatttgg ttgaagatta ggtttgggaa gggggagttt ttgttaaggt tattgtggaa 11520
   gaggttagga atttggtttt agttgagtta gttagtttgt gtggaattga gggagagagg 11580
   gtgggagggg ataggtattg ggttagggag gggaggttgt tttaggagta aaatttggat 11640
   tttgttaata attacgattt ttattgttat ttattatgat aattataggg tttttttttt 11700
   tatttttgaa taaagagttt tttttttatt ttaggttttt gttaattttt ttttaggttg 11760
   agagggttga tgttagagga ataggggttt tatttattgg attagttgtc gttttggttt 11820
   ttttttaatt tttgtggggt tttatataac gggaattgga gttgggggag ttatgtaagg 11880
   gaagaagagg ttggtagaat tttattagtt tttatagttg gatttttata tataaatata 11940
   tatataaatt tatgtatgta ggtttataga aaatatttgg gtgggtaaga gtacgtttgt 12000
   agttttagtt tgtgtgtgtg taggtatgta tgtgaggaga aggaatttgg agatattatt 12060
   tttttatatt tgggttttat tatataatga atatagttta gaaattgtat gtatgggttt 12120
   tagtatatag ttttttaaag atataattat agttttatag ttttttaaat tgtatatttt 12180
   tcgaatttta tatagttagg gtggtttttg tatttgttgt ttttgttaga agatgttttg 12240
   aggttttttt tgtgggttta gtttatgtat taggtttata aatatttagg tgaatttata 12300
   tttatagagg tttgtttttt ttaaggtgag gtttaagggg tagttaattt aaagtgaggt 12360
   ttttgtaatt tacgttgagg tttagaaagt tattatttta gattttaggt attttgattt 12420
   agtttttggg ggtcgggagg ttagtttagt tagtttagtt tggatttttt ggaatttatt 12480
   tttgttttta gagaagagaa aattatagtt attttaattt tgttttagtg gagtttgaaa 12540
   ttgaattaaa ttttaatttt tattattttt attttatttt tagtttatta tttatatata 12600
   ttatagggta aggttggaag gtttttttag atttttatta gagtttttaa aaagtttaat 12660
   ttagttttgt ttgtaaattt tagtgtaaat ttatttagtt ttttggggag ggttttgagt 12720
   cgtaggaggg ttaaaggtta tagaaatagg ttaaaggtta gaaagattag agaataggga 12780
   tgggtaaggg tagggatgat tagggtgggt ttaaaagggt atagattttg ttttagaagg 12840
   tttattagaa gttatgattt ttagagagat atttgggttt tagttttttt atattttagg 12900
   ggtggagaag gtttcggtag agaatcgaaa ttagtattaa agagagggaa aataaattat 12960
   ttatatttaa tttttatttt atgttagtta tttttattgt ttacgggtat ttttttttga 13020
   gagagagaaa attataggtt tatattttta atatatggag tttagtttaa gtatagtaga 13080
   aaaaggggta gggggttaga gtatttgtaa ttagttttat atttttgttt attggggtcg 13140
   tttttatttt tagaaggtgt taggattttt tttttagtat tagtattttt agttgtttac 13200
   gcgttatatt ttagagttat atatttagag aaaaaggtga gagttttttg agttacgaag 13260
   atgggagaaa gaattagaag tcggaagggt taaattttag ttttgttatt ttgggtgatt 13320
   ttacgtaagt tatttaattt ttttaagtat ttgtttattc gtttgtgaat tgtttatttt 13380
   aataacgagt tgtgcgattt tagataggaa gttttggaaa gtatttagat tagagtttgg 13440
   tataggggag gttcgggtaa ttggagtttt ttatttttat gttttttgtt attattttgg 13500
   tgtttttt 13508

<210> 217
<211> 6478
<212> DNA
<213> Artificial Sequence

<220>
<223> chemically treated genomic DNA (Homo sapiens)

<400> 217

tttattttga agggatgttt aagaaatatt gttaaattta aaagtgttgt agagtaatat 60
gtattataag attatattgg tataatagta ataaatagta tttttttata taattttaaa 120
aaaatatata gatttatgtt aagtattagt taagatgttt ggtatatggt aggtatttaa 180
tatttaatga atatatgtag aggattttta ttttttattt tatatttttt ggataagatt 240
ttgaattttt atgagtatgt attatttaaa tttagattta tagggagtga tggaaaatgt 300
gaaaataaaa ttttaaaaag gaaaagaaag agtaaagatt tagatttaaa aaaggaaatg 360
taaataagat gaaatagtta aagataggtt tttttaaaat taatgttgag ataatgttat 420
tagtagtgat agttagttaa gagatggttc gtagtttatt gtttagtatt gaggtattta 480
gtaggggttg tatttaattt ttaagttgga attaatcgaa attgttgggt ttgaagagtt 540
gtttttatga gttatcgacg gtagaaagta tagaagtatt aaagaaaata atttagaaat 600
gtggaaagag aaaagtatgt ttttgttttg ggagaataat atttgtgtgt aattaagtag 660
gaagattttg tggaggaaag tttgaaaaag tagagagttg aagagaaaag agttaaatga 720
aggatacggt ggttaatatt taaagtaaaa agatattttt gaagaagatg tgtttttagg 780
gtttttaaga agtcgttata tttatgttta gagttatttt gtaagtttat ttggaaagtg 840
tattggtgtt ttttggtata agatttacga atattaatag ttttgttatg tgagtaggtg 900
tttatattga agggttataa gtaaagtttt tatgtgttat tattgttaga atttttttcg 960
ttttttttta atataaaagt agttttgttt gttagtatgg aagttttggg aagagtttta 1020
tatttgtttc gtgttgttaa tatttttgtt ttggtttttt tgttggaata tataagttat 1080
ttagtattag ttatttattt ttttggtaga attgtggtaa gaggggatta ataggaaaaa 1140
aaaattaaaa aataaataaa aagttaaaat aatatgaaag agttgggtat gtttttaaaa 1200
taattagtgt ttattttttt tagttttgtg tttgtgtttg tttgtatttt gtatgtttgt 1260
ttttgtggtg gggggggata tgtttgcgtt tttgaaaatt tttttattta gatattataa 1320
ggtattagtt taattttgaa tatagtcggt tgtagtacgg tgtatagata cgattgtttt 1380
ttttttagaa aattttttag tttaaatatg tttgtagagt atgatttatg gtgttttttt 1440
ttggattgtt tagtgggtaa ttgttgtacg aaaattagag aaatagatgt agattttgaa 1500

ttttggttat aaatttttgt tttatgatta tgggttttaa atgtgttgtg ttatattgat 1560
aaatatatag ttattttgaa ataaaatgga ttttattttt aaagttttgt tttttgtttt 1620
ttattttttg tttgtttgtt ggttggaaat atatttatta gattattgat taaatataaa 1680
gaaaatagag atgatatata ttagaatgtt attttatgtt ataataagaa gatatttggg 1740
aaagtttttt ttaattttgt ggttaatgtt taaaatgggt gttagtagtt tatattttat 1800
tacggataaa tcggtttatt ttttattttt tattttattt attaattttt atagtgtacg 1860
gatttgtatt taaagaatta tggtttattt atgtattttt tttttatttt atttaaaatg 1920
aagaatttgg agatagaatt aagtttttaa gaagtgtaag tatttattga tgaagaaata 1980
attagtatgt ggttaaatta tgggtttgtt ttataaatta atatttggat taagtaatag 2040
ttttaattat tgttattagt ataattaatt tatgatgagg aatgattaat tgattagcga 2100
atattgttat attataagaa atttatatta ttttttttat ggggatttaa atattgatta 2160
tttattttaa ggtttaagtt tattttttag ttgagaattt taaggtaatt atatatttta 2220
tatttttttg tataaagaaa gaaaatatcg agtcgtttaa tttttattat atatagagag 2280
agatatatta atgttgttat ttttgtgtaa tgatatagta tagaggtaag aaattcggaa 2340
gtagtggttt cgtttaatga ggttgatttt atagtttaag aaatgagaat gaatagaatt 2400
ttgaggatat ttatttttat ttttattttt ttgggattag tgtattttta ttttttggag 2460
agatgttaag taagaatgtg tagtagtttt ttttggataa tagatttttg tatatatata 2520
tttgttagtg atgttatgtt ttttattttt aatttaagtt ttttttgttt tatttttaat 2580
atagttgttt tttttttttt tgtttaataa ttttttattt atttttattg ttcggtaaaa 2640
atgaatttag tttttaagga attttaagga ttttaaataa gtatttggag ttataagttt 2700
tatggattaa gtagaaattg gtatataatg tagtgttttg tgtattttta taattatttt 2760
aatgatagtg tgttaaatgt tttgtaaatt tttttgtgtt tttttgttta aaatgtgtgt 2820
tttttttgta gttaaagggt tttttataat aagtttttgt tataaaatga tggttatata 2880
aaagagagaa gagaggcgta aaaataataa atatagattt agtaaaaatt ttattttttt 2940
attgttttga attttttaat agtaagaatt gagtttgttg ggttgaaaat tgttttaatg 3000
tatttttata aaaggatttt tgatttggta agtgtgtgcg atgtatattt tttatgttat 3060
attataagtg ttatttagta attttattag atagggtagt gttttagtat ggggtggggt 3120
gttttttgat aggtttttaa aaggttcgga tgttaatgta tattttaata ttatttataa 3180
aaaggagatt ggagtagtgt ttttttttgt attgggtaag gagatttttt ttttttgttt 3240
aattatttgt ttgttttgtt ttgtgggaga attataagta aatgttatag aggtagtgga 3300
gaaaaaaggg tgttttaatt tttttttaga gtttttttta tttgatgtat gttgtatttt 3360
ttaaataagt tgtgtaaaat ggttgtaggg tagattggtt ttttttttta aagtttttta 3420
ttcggttggg tttatttgta aatattgtat ttattttttt tagtgtttta ggattggttg 3480
gaaagatttt tttttttgta ggttgggtta gtgtgagaga tttaaaaaat tatttttttt 3540
taaaattatt gtattttaat aaaaggattg ggtaggggtt ggaatgagag aaaattggtt 3600
ttttaaaatg taaaattgtt atatttaaat tagtttataa aatatgcgtt taattatgtg 3660
gtgggatgtg tgtaggtgta tgatgagaga ggtaattaat atggttattt ggggtgtaag 3720
gatgtgggaa taggtaagta atttttatat tggattttta ttttagggag ttgggtttta 3780
gttatagttt tgagttgtgt gattttgggt aggttttatt ttttcggggt tttgttttat 3840
tagttgaata gtatgaggat gagttatagt taatatttgt tttatgatat ttatttagta 3900
ttatataagt gttatttttg tttttttagt taatattgac gtgggtagta ttatatgttt 3960
attttataga tgaggaaatt gaagtttgaa gaagttaaat atttatttta gaatatatag 4020
ttggtaagtg gtagatttgg aattggaatt tagtttagtt tgatttttta atttatgttt 4080
ttgattatta tattgttttt ttaagtttag atttgaaatt ttattttttg tgtggttgtg 4140
tgtttgggat aggggtaatt aatttttgat tattttatat gttgtataga atttggagag 4200
gattttttaa agtaaatgaa tttcgaaagt tggattgtag agtaaacgag tgtagttaat 4260
ttagttaggg gtttgtaaga gggagaaaga gaaaaagatt gtggaatgga aagtttttta 4320
atttaagttt tttttaaggg gtagttattt tttgttttat agtttagggt ttgtatgtgt 4380
tttttttgga gtggaaaaat atataagtta taaggaattt aatagataga aaggcgtata 4440
gaggaattta aagtgtgggt tgggggggcga ggcggtgggc gggaggcgag cggcgtaggg 4500
cggaatatcg ttttttaagt taagtcgtcg taaataaaaa ggcgtaaagg gagagaagtt 4560
ggtgtttaac gtgagttagg agtagcgttt cggttttttt tttgttattt ttaaaagtat 4620
tttttgtatt gtttttaagg tgagaaatag gaaagaaaac gtcggtttgt gcgttcgttg 4680
tttgtttttt tggttgtttg tttttgtagg gttgttggga gtttttaagt tttgtgagaa 4740
ttttgggagt tggtgatgtt agattagttg ggttatttga aggttagtag ttcgggtagg 4800
gtttatcgaa agtttattcg tatatattag gtaatttaat tttttatttt gtgtgataga 4860
agtagtagga agtgagttgt ttagaggtag gagggtttat tttttgttaa aggggggatt 4920
agaatttttt tatgcgagtt gtttgaggat tgggatgtcg agaacgcgag cgattcgagt 4980
agggtttgtt tgggtatcgt cggggtagga ttcggaacgt attcggaagg ttttttgtaa 5040
gtatttattt ggaaggagaa tttgggattt ttttgggaat ttttcgtttc ggttggattg 5100
gtcgagtaag tttggaaaat ggtaaatgat tatttggatt aattataggt ttttagttgg 5160
tttgtttgtt ataatttatg attcggggtt gggaaaaaga ttaatagttt acgtgttaaa 5220
aaaggggtag agtttgatgg agttgggtgg atttttttat gttatttgtt tttatattta 5280
gaggataagt atttttgtag atatttagtg taagggagat tatgtttgat tgtatggatg 5340
ttttgttagt gagttttggg taaattttgg atttttatat tgcgagttcg tttttttgta 5400
tgttttagga gaaagttttt aaagtatgtt ttagtggatt gatttaaatc gaatggtagt 5460

atcggtatat tgtttaatgt aggtttattt tttttttttt ttttattaag aaaaaaaaaa 5520
ttgttttttt tgtatgtaat aaagacgttg gaaataaatt gtattggtag taagataaag 5580
gatttaatga tttaatgttg aaggggtgtg atatgttggt atgtatattg attttttttg 5640
ttgaaaattt tttgttagat gttttttttt taaataattt ttttcgtttt tatgtttttt 5700
tttttattaa ttttatagtt ttttgaaaat agtttttaaa atatgtataa aaatgtgggg 5760
ggtggggagt agagggtgat tataagattt taaaatattt tttaagtagg gtaatttgat 5820
atattttgta gttttattta aaatgtatgt agagtggaat ttaattttgt tgtttttttgg 5880
attagttatt agttgtatag gtttttttat tttttgtgta ttttaatagt ttaaagtaat 5940
taaaatatga tatttttttt gaaaggaatt tagtttaatt aaggtggttg ttgttttcga 6000
agtttataaa ttattagttt aagttgtcgt ttttaatgtt ttttttgtta aggaaaggta 6060
atatttttta gtattgattt tgttgttatt tttttaagtt attttgttgg tttaagaagt 6120
tgttgttttg ttagaaaatt attggtaatg aatatttttt ggattgagtt atttattatt 6180
tttatatttt gtagtttagt tcggaacgtg gatattagtt aaaggaaagg aaatataagt 6240
ggggtagttt aatttaaaaa aaaaatattg attattttgt tatttgatgt tgatatagtg 6300
tcgaggtacg tatttggtta atagtagttt ggttgtaggt ttgagaaatg attaggagtt 6360
ttagaaaatt ttattttcgg gtggttgtag tgggagtttg taggatgttt cgtcgttttg 6420
atagtgattt agataaatgg tttagttatt tattaaaatg tttttagtat tttatttg 6478

<210> 218
<211> 6478
<212> DNA
<213> Artificial Sequence

<220>
<223> chemically treated genomic DNA (Homo sapiens)

<400> 218

taaataaagt attaaaaata ttttgataga tagttgagtt atttatttaa attattgtta 60
gggcggcgaa gtattttgta ggtttttatt atagttattc gaaggtggga ttttttggga 120
tttttgatta ttttttaagt ttgtagttag gttattgttg attaggtgcg tatttcggta 180
ttgtgttagt attagataat aaaatagtta atattttttt tttaaattgg gttattttat 240
ttatattttt ttttttttaa ttgatgttta cgtttcgggt tgaattgtaa gatatggaaa 300
taatggatgg tttagtttaa ggggtgttta ttgttagtag ttttttagta aaatagtaat 360
tttttagatt aataaagtga tttggaaagg tggtagtagg gttaatgtta agaaatattg 420
ttttttttta gtaaggaaaa tattggaagc ggtagtttga attggtggtt tatgaatttc 480
gggagtagta gttattttaa ttaaattaaa ttttttttaa ggaaaatgtt atattttaat 540
tattttggat tgttggaata tataaggggt ggggggattt atataattag tagttaattt 600
aaagaatagt aaagttaaat tttattttgt atatatttta gtaaaaattg taaaatgtgt 660
taaattgttt tatttgaaga atgttttaaa gttttgtggt tattttttat tttttatttt 720
ttatattttt atgtatattt taaaaattgt ttttaagaga ttataaggtt ggtaaggaga 780
ggagtatggg ggcgggaggg gttatttggg aagaaaatat ttaataggaa atttttagta 840
aagggaatta atatgtatgt tagtatatta tattttttta gtattaaatt attaaatttt 900
ttgttttgtt attaatgtag tttattttta acgtttttat tgtatgtaag agagataatt 960
tttttttttt tggtagaaga agggaaaaaa ataaatttat attgagtagt gtgtcgatgt 1020
tgttattcgg tttgggttaa tttattgaag tatgttttga gagttttttt ttggagtatg 1080
taggaagacg gattcgtagt gtagaaattt aggatttgtt taggatttat tgatagaata 1140
tttatatagt taaatatgat tttttttgta ttgaatgttt gtaaaagtgt ttatttttta 1200
ggtgtggagg taaatggtat agaaaagttt atttaatttt attaaatttt gttttttttt 1260
tggtacgtag gttgttggtt ttttttttag tttcgaatta tgaattatga tagataagtt 1320
agttaaaagt ttgtaattga tttaaatgat tatttattat tttttaggtt tgttcggtta 1380
atttagtcgg ggcggggggt ttttagaaag attttaagtt ttttttttaa gtaaatgttt 1440
gtaaaaagtt tttcgaatgc gtttcggatt ttatttcgac ggtgtttaga taaattttgt 1500
tcggatcgtt cgcgttttcg gtattttagt ttttaaatag ttcgtatggg ggaattttgg 1560
tttttttttt ggtaaagaat agattttttt gtttttgaat agtttatttt ttattatttt 1620
tgttatatag aatgaaagat tgaattgttt aatatatgcg agtgaatttt cggtgaattt 1680
tattcgggtt gttaattttt aaatgattta attagtttga tattattaat ttttaggatt 1740
tttatagagt ttaaaaattt ttagtagttt tgtaaaagta gatagttaga gaggtaggta 1800
gcgagcgtat aagtcggcgt tttttttttt atttttattt ttaaaaataa tataagaagt 1860
gtttttaaaa tgagtagggg aggagtcggg acgttgtttt tggtttacgt tgagtattaa 1920
tttttttttt tttacgtttt tttatttgcg gcggtttagt ttggaaaacg gtgtttcgtt 1980
tgcgttcgtt cgtttttcgt ttatcgtttc gttttttagt ttatatttta aatttttttg 2040
tgcgtttttt tgtttgttaa attttttata atttatgtat ttttttattt tagaaaaagt 2100
atatgtaagt tttaaattgt agaatagaga atggttattt tttgggaaag gtttgggttg 2160
ggaaattttt tattttatag tttttttttt tttttttttt ttgtaagttt ttggttgaat 2220
tgattgtatt cgtttgtttt gtaatttagt tttcgagatt tatttatttt gaaaaatttt 2280

ttttaggttt tatgtagtat atagagtagt taggaattgg ttatttttgt tttaaatata 2340
tagttatata gaaaatgaga ttttagattt ggatttgaaa aaatagtata gtggttaaga 2400
gtatgggttg gggaattaaa ttgaattaga ttttaatttt aggtttgtta tttattagtt 2460
gtgtgttttg ggataagtat ttaatttttt taggttttag tttttttatt tgtaaaatgg 2520
gtatataata ttatttacgt tagtgttaat tgggaggata gaaataatat ttgtatggtg 2580
ttgggtaaat attatggaat aaatgttagt tgtgatttat ttttatattg tttaattggt 2640
gaaataaaat ttcggggaga tgagatttat ttaaggttat atagtttaga gttgtgatta 2700
gaatttagtt ttttaggatg aaagtttaat gtgaaaatta tttgtttgtt tttatatttt 2760
tgtattttaa atagttatgt tggttgtttt ttttattata tatttatata tattttatta 2820
tataattaaa cgtatatttt gtaaattggt ttaagtatga tagttttata ttttgaagga 2880
ttagtttttt tttattttag tttttgttta atttttttat taaaatatag taattttaag 2940
ggaaaatgat tttttagatt ttttatattg atttaattta taggaagaag agttttttta 3000
gttagtttta aaatattaag aaggatagat gtagtattta taaataaatt tagtcggatg 3060
gagagtttta aaagggagag ttaatttatt ttgtagttat tttgtataat ttgtttaaag 3120
gatgtaatat atattaaata aaggaaattt tggagaggaa ttaaaatatt tttttttttt 3180
tattgttttt gtagtattta tttgtaattt ttttataaaa tagggtaggt aagtggttag 3240
gtagggaggg agagtttttt tgtttaatgt agggaagagt attgttttag tttttttttt 3300
gtggatagtg ttggaatgtg tattggtatt cgggtttttt aaaagtttgt tagggggtat 3360
tttattttat gttgaaatat tgttttgttt agtggagttg ttaagtggta tttgtggtgt 3420
aatatgaaaa gtatgtatcg tatatattta ttaagttaaa gattttttta tgaaggtata 3480
ttaaaataat ttttagttta gtaagtttag tttttgttat tgaaaagttt agggtaataa 3540
aggggtgaga tttttgttga atttgtgttt gttatttttg cgtttttttt ttttttttta 3600
tatggttatt attttgtaat agagatttat tgtaagaaat tttttaatta taaagaaaat 3660
atatatttta agtaagagaa tatagaggaa tttataaaat atttaatata ttgttattaa 3720
ggtagttgta aagatatata aagtattata ttatgtgtta atttttattt ggtttatgag 3780
gtttatggtt ttaaatgttt atttaaggtt tttagagttt tttggaaatt gagtttattt 3840
ttatcggata gtggaggtgg atgaaaagtt gttagataag agaagaggaa atagttatat 3900
tggaagtaga gtaggagaaa tttaagttag agatgagaaa tatggtatta ttgataggta 3960
tgtgtgtata gggatttgtt atttagggga ggttgttgta tatttttgtt taatattttt 4020
ttaaagagta gagatgtatt ggttttaggg aagtgaagat ggggatgggt gtttttaaaa 4080
ttttgtttat ttttattttt taagttatgg aattagtttt attgagcgag attattgttt 4140
tcgaattttt tatttttata ttgtattatt gtataaaaat aataatatta gtgtgttttt 4200
ttttgtgtgt ggtaaaggtt gggcgattcg atgttttttt tttttgtgta agaaaatgta 4260
aaatatataa ttattttgaa gtttttaatt gagaaatgaa tttgggtttt gaaatgaata 4320
attagtattt aaatttttat gaaaggaata gtatgggttt tttataatat gataatattc 4380
gttagttaat taattatttt ttattatgaa ttaattatgt tgatagtaat aattagagtt 4440
attatttaat ttaagtatta atttataaaa tagatttatg atttagttat atgttaatta 4500
tttttttatt agtaaatgtt tatatttttt gaaaatttga ttttgttttt aaatttttta 4560
ttttaagtaa aatgaaggaa agatatataa ataagttata attttttaaa tatagattcg 4620
tgtattataa agattaatga gtagggtaga aagtgaaaaa tagatcgatt tgttcgtaat 4680
gaaatgtaaa ttattgatat ttattttaaa tattagttat agggttggaa gaaatttttt 4740
taaatatttt tttgttatgg tataaaataa tattttgata tgtgttattt ttattttttt 4800
tgtgtttgat taatgattta ataaatgtat ttttaattaa taagtaaata aaaagtgaaa 4860
aataaaaagt aaaatttgga gagtaaaatt tattttgttt tagagtaatt gtatatttgt 4920
tagtgtgata tagtatattt gaaatttata attataaaat aaaaatttat agttagggtt 4980
tagggtttat atttgttttt ttggttttcg tatagtaatt atttattgga taatttagaa 5040
agaaatatta tgaattatgt tttgtaaata tgtttgaatt ggagagtttt ttgaagaaag 5100
agtagtcgtg tttgtgtatc gtattgtagt cggttgtgtt taaaattggg ttaatatttt 5160
gtgatgttta aataagaaga tttttagagg cgtagatata ttttttttta ttataggggt 5220
agatatgtaa agtgtaggta gatataggta tagggttggg ggaggtggat attgattgtt 5280
ttaaagatat gtttagtttt tttatgttgt tttggttttt tgtttgtttt ttaatttttt 5340
ttttttgtta attttttttt gttatagttt tgttagaaga ataaatggtt agtattgagt 5400
gatttgtgtg ttttagtagg gaagttaagg tagagatgtt ggtagtacga ggtaagtatg 5460
aggttttttt tagggttttt atattagtag gtagagttgt ttttgtgttg gaagggagcg 5520
ggggaagttt taatagtaat aatatatgga aattttgttt gtggtttttt agtatgaata 5580
tttgtttata taataggatt gttgatgttc gtggatttta tattaaaaga tattaatgta 5640
ttttttaaat gaatttgtaa ggtggttttg ggtatgagta tagcgatttt ttagaaattt 5700
tagaaatata ttttttttaa aaatgttttt ttattttgag tgttggttat cgtgtttttt 5760
atttggtttt ttttttttta gttttttgtt tttttagatt tttttttata aagttttttt 5820
atttggttat atataaatat tgttttttta agataagggt atattttttt ttttttatat 5880
ttttgaatta ttttttttag tgtttttgtg ttttttgtcg tcgatgattt ataaagatag 5940
ttttttaagt ttaatagttt cggttggttt taatttgaaa gttaaatata atttttgttg 6000
aatgttttag tattgagtag tgagttgcga attatttttt agttgattgt tattgttggt 6060
ggtattgttt taatattagt tttggggaga tttatttttg gttattttat tttgtttata 6120
tttttttttt taaatttaga tttttatttt tttttttttt ttttaaaatt ttatttttat 6180
attttttatt attttttata aatttagatt taagtaatat atgtttataa aaatttaaag 6240

ttttgtttaa aagatataaa gtgaaaagta aaagtttttt atatgtattt attaaatgtt 6300
gaatatttat tatgtgttag gtattttaat tggtgtttgg tatagatttg tatatttttt 6360
tgggattgtg tgggggggtg ttgtttgttg ttattatatt agtatggttt tatgatatat 6420
attgttttgt aatattttta aatttagtaa tattttttga atattttttt aaaataaa 6478

<210> 219
<211> 6236
<212> DNA
<213> Artificial Sequence

<220>
<223> chemically treated genomic DNA (Homo sapiens)

<400> 219

tacgtttttt atttataggt gagttagaga tgtggaatga gttaggagtt ttaaggaggg 60
gttttttttt ttatattatt tggttggttg atttcgttta atttaatttg atttttttta 120
cgatgttatt gatgttgatg atggaattac gataattatt ggatattagg gtttgtgttg 180
tagtttgagt gattaggaag gtgttttggc gggaaagggt aataagggag aaaagattag 240
ttggggtgat agtttttttt taattttttt tttaggttaa gggggttaaa cgtcgtaggt 300
ttagagatcg ttattttgag gttgatagtt atgattttgt tttagttatt tttagatatg 360
tgtagtagaa atttattttg ggtgatgttc gtataggata taacgataga tggtgggcga 420
gaaaagtagg tttatgggag ggggaggtta cgtattaaaa attttttata aaaagtcggg 480
gtagtggagg taatattaga gttttagagg gggaaagtgg tttagcgttt atttgtattt 540
gttttagtag gtatttggcg gttttggttt tagatacgtt agtttggaag gtagtatggt 600
tttttcgggg cggttttttt ttgttttttg gttcgtttag tagtcgtatc gttttttgtg 660
tcgttgttcg gtttaggtcg taggtagtta cggtggtttt tttttcggtt aggcgtatat 720
tgatcgttcg gtcgatgtcg tttttcgtat ttgaggtagg atagaatacg tcggatcggg 780
tacggggagg agagggtagg gattagaggt tatagggtag aaagtaaaat gtgggtaagg 840
ggttagaggt tatagcggtc gcgtatttta agggtacgtt tttgatttta ttttaatatt 900
ttaaatcgtt cggggaaaga atttttttaa tttgtgatta aggttagtgc ggagcggagt 960
cggttttgga gttgagacgt tatggcgggt tgtgggtggg tgggaatttt agtattaagt 1020
taattttagc gtagaggggc gtggtaaggg taaagtcggg gttaattaaa ggttcgttag 1080
ttttaggtta tttgcggtta ttagcgtagg tcgatttttt atcgaggggg cgtggttcga 1140
tattgttgtt taggggtttc gggtgtggag aagataaggg gttttatttt ttttaaaaag 1200
ataaagattg tatgtggggc ggaagatcgg aggtttttat tttcggttat tatttttttt 1260
tttcgattta aatatagtga tataaaagga atagaaataa aaaaaattag gttatgagaa 1320
tattaattaa ttttttttta ttttattttt ttaattattt ttaaattagg atatacgatt 1380
gatgtttgat gtttattttc gatttttttt gttatttttt ttgtttagga ttttggtttt 1440
ttttgaagtt tttattaatt ggtgggagaa aggaaattgg attttggaga attttttgtg 1500
tagtaatttg gtttttcgag aataggtggg gtaggggata ttaggtaggt tgttattatt 1560
ttttgttttt tttagttttt tttatggttt tatttagttt tttaaatgta atatggttag 1620
atagtgttta atattatggt tttttggttt ttttattttt atcgttttag tttttgtaag 1680
attgttagtt tttgaaggtt ttaatgttaa agtaattatt tttttaggtt atagtttttt 1740
tttttggtag atgttttggg ttagggtttt taatttttac gtatttttga tttggagtta 1800
ggggtagagg tttggggtag gggtagttta ttttattttt atttaaggtg ggtaattagt 1860
attattatga taattttttt tagtagtttt agtattttta gaagtgattg taatggtgat 1920
gtttttttta gtagttcggg taatatagat attgttgtta cgtagataaa gttatttgta 1980
gtaaatggta ggatttagtt aggatttgta ttatttgtaa ttttattgtt tattgttttt 2040
tttttagtga gaagggtata ggttgtgttt gttagtgttt ttattgttgt tagtagttgt 2100
agacgtatcg tttgatgggt agaaaagaga aaaaggtatt tattaatagt taaagattat 2160
tagtggttta gaagtggttg gtggggtata ggtatagggg atatggattt ggaaattaaa 2220
tattaaaata aaaagagaag agggagtagt ttagaataga taatttttga aaaattttaa 2280
gagattagag gtttttttta tagatttttt gattgagtag gcgtgtgtgt gtaagatttt 2340
attaaattat ttagatggtt tatagattag tttaagaaat attgagttaa aggataaagt 2400
aattagaatt tttttttttt tatttttaga ggatttgtta atgatagtat gaattgtttg 2460
gaattaataa gaggttgttt gggtttaggg tgagtgagga gagataaagg gtttgaggta 2520
gttgtgtttg gtagatatta ttaatttgtt ttttgttgta gttagattgg attaagatgg 2580
taaagttttc gatttcgtgg ggtattttat gtagtaggat agttattgtg gttaggattt 2640
ttagttttcg gttttttcga aaggaagttt taatggttag attattagtg aagttgtgtg 2700
ttaagttagt agttagattt aggtatttcg atatacgtag gttttgtata ggataagaag 2760
agaaaatgat tagaggtttt tattattatt tgttttagtt ttggttagtt atttttatat 2820
ttttttttag agattatttt atatttagtt ttttagtgtt ttttagatat ttgttattat 2880
ttatagtata tattttatgt gatttgtgat gatgtttttt gtttaaattt attattaatt 2940
ttggttttta tttaagtttt tttttttttt tttagcgttt tgaggtttta ttggtttatt 3000
tttgggtatt gtgttttttt ttcgtttttt ttgaattttt tttgtttttt tttttttttt 3060

ttttgagttt tgtttttttt tggtagaacg ttttgaggga agaaaagagt tggttagagt 3120
ggagaataga ttttttaata tgtttttttt tttttgaggg ttataagatg aggagatttt 3180
gaggtaggta agttagtttt taggaaagtt gtttttgggt ttattttgtt ttttatgttt 3240
atgattttta cgtgtatgat tgtgagcgtg tttatgttta tgattgtgat tatgtttttt 3300
ttttatatgt tttataaatt tttttacgat aagaaaggta ataattttat tgagaattta 3360
tagttttata gatagaatgg ggttttggtt tgggaatgga ggtattgaga tattaaggga 3420
gatgtgtatt ttagattttt tttttttaag tgcgggaggt gaggaatagg gagagacgga 3480
ttttttttag tattttaatt tttattttta tttgtttttt ttttattatt gtgggagtgt 3540
ttatgttcgg gttgttttag agtgtggtga gaatgaggtt ttggaagaag gggggaaaat 3600
tagatgaggg aatatttgat ataatgagtg tgtatttggg ggaatatcga aaggttaggg 3660
attataaagg agaaagaatt tttttttttt aaagaacgaa ataggttaga tttgaggtag 3720
aagttagagg ttatttatta agagtatgag gaatgaggtg taggaaagta ttttttagga 3780
gtttatcgga agtaaaattg agtaagattt gaagtagaga gcgatgtcgg ggagagttcg 3840
attttacggg gataaggaag aggataaaaa atggagttgt tgagattagt attgtggttt 3900
ttagtgtttg gtgagggaga gttaaggtta agtattgtgg agttttttaa taatttagaa 3960
ttagtttttt ttttttttat tttttggaga tttatataag tttagagagt gatagtattt 4020
aggttttgtt tgatgtttgg agttttagat tttttatagt ttttatggtt atgtttatgg 4080
ttatgtttat gttttttgtg gtagaggttt ttatgggagt agttatggtt atggttatgg 4140
tgtaaatttt tatgtgaatg tttatggtcg tgatcgtggg tatgtttatg ttagatgttt 4200
tcgtgagtgt ggttatggtt atgggtatgg ttgtggttat ggtggaaatt tttatgtgag 4260
tgtttgtggt tgtggttatg gaagtttttt tgtagatcgt cgtgtaggtc gttatgattt 4320
tcgagtttag ttattagaag ttttaaggtc gtttaggtta gtagttttac ggttatttag 4380
tggggggttt ttaggttttt ggttattacg ttgattagag ggatagaggt agtgatttta 4440
tttgatttaa ttttatattt atatagggtt cgttttattt taatcgcgtt tttcgtttat 4500
ttttatgggt cgttttttta tttttttaag tttttattta tattgttttt aagattagtt 4560
ttttttattt cgaattattt ttcggatgta gggttttggg atattaattt tttattagtt 4620
attttataga ttttttgggt tttcgttttt acgatttatt aggtgaggaa aaggtttgga 4680
aaggatggta gagaaaaaat agagaatttt tatcggtttc gggatttttt tttttttcgg 4740
tttgtttgga cgtggtagtt tcgtttttag atttcgcgag aataggaagt ttcgttttat 4800
tttcgtttta atgtttttat taatatggcg gtatttcggt agcgacgaat ttattttgta 4860
ttgatacgta tgcgcgagtg tggaggtgat ttgaggggag ggtgggcgag gcgggttaat 4920
aggtcggagg agatcgtttt ttcggttttt aattagacga gggggcggga attcgcgtcg 4980
agatttttcg tttgtataaa gatggttgtt atattggcgt tgttattttg gtattgagta 5040
gagcgacggg tttaattcga tttaatttag gttagagttt tttttttagg ggttttttcg 5100
tgtttagtta atttttcgat taatttttgg gaatttttgg gattttttcg gtatttttat 5160
ttttagttag ggattatgtt ttgggtcgtt cgttcgtttt ttttttttta gcggtatgtc 5220
gtagggtagt gtgggtcggt gggggtgcga aaagaaatgt attgtggggt cgcgtttcgg 5280
tggcggcggc gacggttttg gttggatttc gtagcggcgg cggcggcggc ggtggtaggc 5340
ggagaataat aaatttcgga gtcggagtta ggggaggttg gacgggacgg gatgggcgat 5400
agcgggcggg gtgagtgttt tagtagaagg gtaggcgagt tagtgatcgt tttatttgcg 5460
attttttttt tatatttttt ttcggtgttt ttttttttgc gttttcgttt ttttgtttgt 5520
aaatacgcgt tttttttttt tcgagggttt tagtattttt tttttcgggg cggggcgggg 5580
aaggggagtt ttcgtttttt tagttgtgat cgtttgaggt tttgggtatc ggaggtcgcg 5640
ggtttgggaa gggtacgatt ttttttaaat tttaggattt tgggcgttta taggtagatt 5700
ttgcgtttta ggaggggttt tttttgtttg tttttttttt ttgtacgttt tggttatttg 5760
gttttacgtt gggaatagag gggttcgatg gtttatttta cgtgttgttt tatttacgag 5820
ataggtgtgg ttttgggatg atttggtatt tatttaaatg gattggttgg aaaatttttt 5880
ttatttgaat tatttttttt tttttgttaa tttttttttt ttattataat gaggagttac 5940
gttgtttttg ggttttttta gttagttaaa tatagtagtg ggaggttgta tattaatttt 6000
agtgatataa atgttaataa gttttgataa tttattatta tcggattagt cggagttata 6060
ttgttgtttg atagtagggt gtttttgaat ttggggatgg gtggagtggg ttgggttcgg 6120
tacggtgggg tattagagaa ttaaagtttt tagttggatg tttgaagatt tatttttttt 6180
ttttgttttt tttgtttttt gtgtgtttgt gtgtttgtat atattttttt tttaga 6236

<210> 220
<211> 6236
<212> DNA
<213> Artificial Sequence

<220>
<223> chemically treated genomic DNA (Homo sapiens)

<400> 220

tttgagggag gggtatgtat aggtatatag atatataaga gatagaagag ataaaaaaag 60
aaaatgagtt tttaaatatt taattagaga ttttaatttt ttaatatttt atcgtgtcgg 120

atttagttta ttttatttat ttttaagttt agagatattt tgttgttaaa taatagtgta 180
atttcggttg gttcgatggt agtgggttat tagaatttat taatatttgt gttattaaaa 240
ttggtatata atttttattt gttatatttg attggttaaa aaatttaaa aatagcgtaa 300
ttttttattg tggtgagagg agggagttga taaggagagg aggatagttt aggtgaggaa 360
aattttttaa ttaatttatt tgaatgaata ttaggttatt ttaaagttat atttgtttcg 420
tgggtggggt agtacgtggg gtagattatc gagttttttt atttttagcg taaagttagg 480
tagttagagc gtgtaaggga aagagatagg taggagagat tttttttaag acgtaggatt 540
tgtttgtaaa cgtttaaagt tttgaggttt aagaggaatc gtgttttttt taggttcgcg 600
attttcggtg tttaaggttt taagcggtta tagttaggag ggcggaagtt tttttttttc 660
gtttcgtttc gggggggagg gtgttaaggt tttcgggagg gaggggacgc gtgtttataa 720
ataagggggc gggagcgtaa ggaaaagagt atcgggggag ggtgtggggg aggggtcgta 780
gataaagcgg ttattggttc gtttgttttt ttgttggggt atttatttcg ttcgttgtcg 840
tttatttcgt ttcgtttagt tttttttggt ttcggtttcg gggtttgttg tttttcgttt 900
gttatcgtcg tcgtcgtcgt cgttgcggga tttagttagg gtcgtcgtcg tcgttatcgg 960
gacgcgattt tataatgtat tttttttcgt atttttatcg gtttatattg ttttgcggta 1020
tgtcgttgag ggaggaaggg cgggcgagcg gtttaagata tgatttttgg ttgagagtag 1080
ggatatcgaa gaggttttag ggatttttaa ggattgatcg gaggattagt tgagtacgag 1140
gaagtttttg agagaaagat tttggtttgg attgggtcga attaagttcg tcgttttgtt 1200
tagtattaaa atgatagcgt taatgtggta gttatttttg tatagacggg aagtttcggc 1260
gcgagttttc gttttttcgt ttagttggaa atcgaggagg cggttttttt cggtttgtta 1320
gttcgtttcg tttatttttt ttttaaatta tttttatatt cgcgtatgcg tgttagtgta 1380
ggatggattc gtcgttatcg gagtgtcgtt atattggtaa aggtattagg gcgaaggtgg 1440
aacggaattt tttgttttcg cgggatttaa aggcgggatt gttacgttta agtaaatcgg 1500
gaaaggagag gatttcggag tcggtgagaa ttttttgttt tttttttatt atttttttta 1560
ggtttttttt ttatttaatg agtcgtagag acgagggttt agagagtttg taaagtggtt 1620
ggtgaaagat tagtgtttta gggttttata ttcgggaggt ggttcgggat aaagagaatt 1680
agttttggga ataatgtagg tgggaattta agggaatggg agagcggttt atagaggtgg 1740
acggagggcg cgattggagt aaagcggatt ttgtgtaggt atagagttga gttaagtgga 1800
gttattgttt ttgttttttt ggttagcgtg atggttagag gtttgggggt tttttattgg 1860
gtggtcgtgg gattgttgat ttgggcgatt ttggggtttt tggtggttgg attcgggggt 1920
tatgacgatt tgtacgacga tttgtaagag gatttttatg gttatagtta taggtattta 1980
tatgaagatt tttattatgg ttatagttat gtttatggtt atggttatat ttacgagagt 2040
atttggtatg gatatattta cgattacgat tatggatatt tatatgagga tttatattat 2100
ggttatagtt atggttattt ttatgagagt ttttattata gaggatatgg atatgattat 2160
gagtatagtt atggaggtta tggggagttt ggggttttag gtattaagta ggatttggat 2220
gttgttattt tttgggttta tgtgagtttt taggggatgg gagagagaag ggttggtttt 2280
ggattgttgg gaaattttat agtatttgat tttgattttt ttttattagg tattgggggt 2340
tatagtgttg attttagtag ttttattttt tgtttttttt tttattttcg tggagtcgaa 2400
tttttttcgg tatcgttttt tattttagat tttgtttagt tttgttttcg gtgggttttt 2460
gggagatgtt tttttgtatt ttatttttta tgtttttggt aagtaatttt tgatttttat 2520
tttaaattta atttatttcg ttttttggag gaaaagggtt tttttttttt tatgattttt 2580
gatttttcga tattttttta aatatatatt tattgtgtta gatatttttt tatttggttt 2640
tttttttttt ttttagaatt ttatttttat tatattttgg agtaattcgg atatggatat 2700
ttttatagtg gtgaggaaga gatagatggg gatgggagtt ggggtgttgg ggaaggttcg 2760
ttttttttta ttttttattt ttcgtatttg aggaggagga gtttggaatg tatatttttt 2820
ttaatgtttt aatgttttta tttttaggtt agggttttat tttgtttgtg ggattgtggg 2880
tttttagtgg aattgttgtt ttttttgtcg tggagaaatt tgtgagatat gtgaaaggag 2940
gatatggtta tagttatgga tatggatacg tttatagtta tatacgtgga agttatggat 3000
atggaagata aggtgagttt aggaataatt tttttgaaag ttgatttgtt tgttttagaa 3060
tttttttatt ttatggtttt taggagggag aggatatgtt ggaagatttg ttttttattt 3120
tgattaattt tttttttttt ttagagcgtt ttattaagga gaagtagagt ttagaggaag 3180
aagaaaagga aataagaggg gtttagaaga ggcgaggagg gagtatagta tttaaagatg 3240
ggttagtgag attttagaac gttgaagaag aaaaaagagg tttaggtaag ggttagagtt 3300
ggtgataaat ttgggtaagg gatattatta taaattatat ggaatatgtg ttgtgggtaa 3360
tggtaggtat ttgagaaata ttaaaggatt gggtgtaaag tggtttttga ggggaggtgt 3420
gagaatagtt gattaagatt ggaataagtg gtgatggaag tttttgatta tttttttttt 3480
ttgttttgta taagatttgc gtgtgtcggg gtatttgaat ttggttgttg atttggtata 3540
taattttatt gatggtttgg ttattggggt tttttttcga gggggtcggg gattagggat 3600
tttgattata atgattgttt tgttatatga agtgttttac gaggtcggag attttgttat 3660
tttggtttag tttggttgta gtaaaaagta ggttggtgat gtttgttaaa tatagttgtt 3720
ttaaattttt tatttttttt tatttatttt aaatttaaat agttttttat tagttttaaa 3780
taatttatat tgttattgat aagtttttta gaaatgaggg ggaagaagtt ttggttattt 3840
tgttttttag tttagtattt tttaaattgg tttataaatt atttgaatgg tttagtggag 3900
ttttatatat atacgtttat ttaattagaa agtttgtgga aaggattttt gattttttaa 3960
gattttttag aaattgttta ttttagattg tttttttttt tttttttatt ttgatgttta 4020
gtttttaaat ttatgttttt tatatttata ttttattagt tatttttaaa ttattgataa 4080

tttttagtta ttggtgagtg tttttttttt ttttttgttt attaggcgat gcgtttgtaa 4140
ttattgatag tagtaggggt attggtaggt atagtttgtg ttttttttat tgaaggagga 4200
gtagtgggta gtgaaattgt aggtggtgta ggttttggtt gggttttgtt atttattgta 4260
ggtggtttta tttacgtagt aatagtgttt gtgttgttcg agttgttgag ggaggtatta 4320
ttattgtaat tatttttgga ggtgttgggg ttgttggggg gagttattat gatggtgttg 4380
attgtttatt ttgagtgagg ggtggataaa ttatttttgt tttaaatttt tatttttaat 4440
tttaggttag gggtgcgtag aggttggggg ttttggttag ggatatttgt taaaggaagg 4500
aattgtagtt tgggagaatg gttattttgg tattagggtt tttaagggtt ggtagtttta 4560
tagaggttgg agcggtgaga atgagaggtt agagggatta tagtgttggg tattgtttga 4620
ttatgttgta tttggaaggt taaatggggt tatgaagaag gttggaaggg atagggggtg 4680
atggtagttt atttggtgtt ttttatttta tttgttttcg gagaattaag ttgttatata 4740
ggaagttttt taaggtttag tttttttttt tttattagtt ggtggaggtt ttagggaaga 4800
ttagagtttt ggatagagag ggtaatagga ggagtcgggg ataaatatta aatattaatc 4860
gtgtgttttg atttgggagt gattgggggg atggggtggg agagggttag ttggtatttt 4920
tatggtttga ttttttttgt ttttattttt tttatattat tgtgtttgaa tcgaggggga 4980
ggggtggtaa tcggaaataa agattttcga tttttcgttt tatatgtagt ttttgttttt 5040
ttggggggaa tggggttttt tgtttttttt atattcgggg tttttaagta gtagtgtcgg 5100
gttacgtttt ttcggtggga ggtcggtttg cgttggtggt cgtagatggt ttaaggttgg 5160
cgggtttttg attggtttcg gttttgtttt tgttacgttt ttttgcgttg ggattggttt 5220
agtgttggga tttttattta tttatagttc gttatggcgt tttagtttta gaatcgattt 5280
cgtttcgtat tggttttggt tataggttga gggggttttt ttttcgggcg gtttggggta 5340
ttggagtgag gttaggggcg tgtttttgga gtgcgcggtc gttgtgattt ttggtttttt 5400
atttatattt tatttttgt tttgtgattt ttgatttttg tttttttttt ttcgtgttcg 5460
gttcggcgtg ttttgtttta ttttaggtgc ggggagcggt atcggtcgag cggttagtgt 5520
acgtttggtc ggagaggggg ttatcgtagt tgtttgcgat ttggatcggg tagcggtata 5580
ggagacggtg cggttgttgg gcgggttagg gagtaaggag gggtcgtttc gagggaatta 5640
tgttgttttt taggttgacg tgtttgaggt tagggtcgtt aggtgtttgt tggaataagt 5700
gtaggtgaac gttaggttat tttttttttt taaagttttg atattgtttt tattgtttcg 5760
gttttttgtg gggggttttt gatgcgtaat tttttttttt tataggtttg tttttttcgt 5820
ttattatttg tcgttgtgtt ttgtgcgggt attatttagg atgagttttt gttgtatatg 5880
tttgaggatg attgggataa agttatagtt gttaatttta aggtggcgat ttttgaattt 5940
gcgacgtttg gtttttttag tttggggagg gagttggagg agggttgtta ttttagttga 6000
tttttttttt tttgttattt tttttcgtta gggtattttt ttagttattt aggttgtagt 6060
ataagttttg gtgtttaatg gttgtcgtgg ttttattatt aatattagta gtatcgtagg 6120
aaaggttagg ttgagttgga cgaggttagt tagttaagtg gtatagagag gagaattttt 6180
ttttgagatt tttgatttat tttatatttt tgatttattt ataggtgggg aacgtg 6236

<210> 221
<211> 6068
<212> DNA
<213> Artificial Sequence

<220>
<223> chemically treated genomic DNA (Homo sapiens)

<400> 221

aaattgttta tttgtaaaat tggggtaata aagtttgttt tatttaagtt tagtattggg 60
gatagtatat ggaaatttaa tgtattatta tgtaggtaag gtattataaa aatataaaaa 120
tattaatata taatattagt atggagatat ttgtatttaa attttatagt taaagtttat 180
tggttattgt taaatgaatg agtttggttt tttatttgta tttgttgtaa aatattattt 240
tatattatta cgggttaaaa gagaaaaaaa gagtagacgt aaagttatat tttttgatta 300
ttagggtgaa tattagatat tattggtcga atttgtgtta aatagttaaa tatattagtt 360
aaaatttatg ttttttttat ttatttaaag gtattaatgg ttttagtaaa agtagaatat 420
attatttaag aggattagtt aattagtttt gataagaagt aggttaagaa tttttgtgaa 480
tgaattagtt aatttttatt gtagattttg agtatttggt tttttatttg atattttaat 540
gagaatttag taatagttgg agatatagtt gtgattgaag ttagtggagt tgtttgggaa 600
gggtttatgt tagaaatatt agttatttag gataacgttg ttatattaat aatggtttta 660
gggaagatat aatggtgtta agaaaaaaaa aagtgggtta aaaagaaaaa attatattta 720
tatttgttga tttttaggta gagtttatgg tttgttatgg ggtattaagt tatatggaag 780
gtattataag aaaagtatat tggggtatcg tttggtatat tgtttttgat gtatttgatt 840
atgttaagta tttttgatat agaaatattt gttgagtatt tattgtgtgt tttagtattt 900
aggtgagaaa ataaagagat ataatatatt attattatat ttgaagagtt aataattatg 960
ttatgaagat aagatatatt taaaggaaag ataaataatt attttttgtt attttattgg 1020
ggatgttata aggttatata tattaagtgt tatttgaata tattggataa taagttttat 1080
tagatttagg ttagggagat atttttttgg ataagtgttt ttatggtggg aaatgagttg 1140

gaatttgaat gataggtaag aattttagtg atgagaaaaa gtaaatgggt atgaataatg 1200
aattacgtgt tagttttttt agtgttgtta taaagaaata tttgaggttg ggtaatttat 1260
agagagaaaa ggtttaattg gttgatagtt ttgtaagttg ttaaaaaaaa gtatggtgtt 1320
agtgtttgtt tagtttttgg tgaagtttat gaggttttta attatggtgg aaggtaaagg 1380
gagagttggt gtgttatatg gtgagagagg ggttaagaga gtggaggggg aggttttaaa 1440
tttttaaata attagatttt ttgagagtta attgagtaag aatttacgta ttattaaaag 1500
gatggtatta agatatttat gagggattta ttttatgatt taatattttt tattaggttt 1560
tatttttatt attagggatt atattttaat atgagatttg gagaggaaaa atatttaaat 1620
tatattaaat cggaagttgg aatgaatata tcgtattttt atagtgggta aaatagttag 1680
attagaagga atttttagag aaagggagga aagaaaatta ggaaaatatt aattttttgt 1740
atttgtttta taaagttatt gtatagaatt tcgtagtgta aaaatgagta cgtgtttaat 1800
ttttaatata tttgatttat aaaattaaaa ttaaatttag aaattttagt gaggttggta 1860
gtatggatgt tttttttaga gagtgtgtat tttaagtttt gtgattattg ttttaaagta 1920
ttttttttta tatagagttt atatgatagg tcgagatatt ttataaatgg tgagtttttt 1980
ttatgttagg ttaggatttg gttttttttt tgtgaggaaa agaaatagat aatgtattga 2040
atttagaaaa aggtgaagaa agagtttatt tgttggtggt ttaattaata gattattttg 2100
ataatgattt tggataagtt tgtagattgt gatttttaaa gaatggtttt tgagtatttg 2160
agtatttgga aaaatttttg aaatatgggt ttatatatat ataaaaaaat agtttaagta 2220
atttattttt ttttttgata tgcgtataat agaaaattgt agattgtaag atatttgttt 2280
tagatattat tatggagaaa atggataatt atggaatggg tgatgtatta gtttgcgttt 2340
agttttaaag ttgaagatga tattggaatt tatgtagttt tgttttatta ttttataaat 2400
gagaaaaagg agaggagtta taaagtgatt tgtttaaggt tatatttttt ttttttttga 2460
tttttttttt tttttattta tttgttttaa attagattat atgtatcgag tatttagtat 2520
ttatttggcg tattttaagt gtttaatatg tattgattta tttaattttt atggtaattt 2580
tataaggtaa ttgaggttta gggagattta tttatttgtt agtattatga ggttgtaagt 2640
agcggaatag ggatttgtat ttagtaatgt tggtttgtga gttggtgagt tggtgttttt 2700
attatatata tagtttttta aggttatata attagtgagt agtagagtaa agtgtttaag 2760
gtattaatga atggattttt tagtgatata agatgttgtt tttgttatgt ttatgttaat 2820
atttttatta atgagtcgaa taagatatga aaaagtagtt tattagattt ttagaagtta 2880
tgaaataagg gtatgaataa gaaatatttt ggataataga agtaggattt taaaagattt 2940
gggatgatga atgaagttaa aaagttttaa ttttagagga atataggaag tattttatgt 3000
aggttttaaa attaattgtt taaggatggg atgaagaata tgtataagag aatataattt 3060
aggggaggta ggggtgggga gatttttatc gagttagttt tatatgaatt aatatgtaga 3120
gttgtttatt taatgtaagt gagtaaatgg tttttttgta ttttatatta tttatgttta 3180
ttggattttt attttagtaa tagttaaggt agttttgggt tttttaaagt tttttatagt 3240
gaaggtttgg atattttttt tttttttgtg atatgttttt gttttgattt tttattttgg 3300
tttttttttt tgttattatg attttgtttt tgattttggt tttttatttt acgtttttgt 3360
tttggttttt tttagtattt gaattttgat tttttatttt gggattttga tttgatttat 3420
ttttttggtt ttttatgttt tatgttgtat taggagaaaa ttttttattt tttttttagt 3480
ttaatagagt ttagtttatg aaacggattt ttaatgggta tgttgatagt attttattat 3540
cgtgttaata tatatattat gaggaaaagg tttggattat atttggagta ttgtatttaa 3600
gtttggttaa tttttttttt tattgtgagg gtatttatgt aaattgaagt atattattag 3660
aaaaaggata ggttgggaat agatatgaat ttttggtgat atatagaatt attgaaagag 3720
ttagagatgt ttagggaaag ggggattatg ggtatagtag attaatttta ggatttgtag 3780
aggattttat tgtgaaagat ttttttgttt tttaggtttt ttttgtgtgg tttttgggag 3840
tagtttagta tttgtgggta gaagttgtag ggaaaaggag tagattttat ttttataaga 3900
aatttttttg tggtgaaaat tatttagaaa tggaatgaga aagtggtgag ttttttatta 3960
ttggaggtgt ttaagtatag gttgagcggt gatttgttac gaaaattgta tgatagattt 4020
aaatattaac ggggtgttta gattttttga tttcggttta gttttatata agataggttt 4080
tgtgttagtg aggatttagt ggtgtttttg aataggcggt aggataataa tggtatataa 4140
aataaatgta gagaaagatt tttatattaa gttaattttt tttttttatt tgtaagtttt 4200
ttatttgagt gagttttggt tttagttgta tttgttaaat ttttattgtt agagtataga 4260
taaatgtttt cggttattta agtaggagag gtttatttta atttaagggt tattttattt 4320
tagtttttcg tagttggaga ttgagtatta cgttttttta attttgtgtt tttgaaaggg 4380
ttttgttttt ttttgaattt tatttttttt tttttttata gattaaatat taagtatatt 4440
ggatagatag aagttcgaat gagaaaatgt attttaataa aattttattg atttttgtgg 4500
atgtaagtta aggaataggt atttaaagag ttttttattt tttgtcgttt ttcgtttttt 4560
ttttgattta tatcgggcgg ttatatatcg attggttttt tagataatag atcgtgttat 4620
tcggtaggga tttttgggga cgcgtcggga gttggaagag tcgtacgtag tagtttaatt 4680
ttgagttaat taaattagta ataggatttt aagtagtagc gacggcggtg gtaagagtag 4740
cggtgacggc ggcggcggcg gcggcggtag tattatgcgt gattattgat aggtattagt 4800
tgttgtcgtt atagtcgttt taaacgtatt atgtggattt ttcgatttag aggtagattt 4860
ttgattaatt ttagagggtt ggtttagttt gtgtttttcg ggttgttagg aagcgatgat 4920
tatttttgtt agtttaagtt gaagaaagtc gggttgtgtt tgggagtcga gagaggcggt 4980
aatatttaga agttgtatag gagaggaata tgaattgacg agtaaatatg tatggaaatt 5040
atttttattt tatgaagttt ttcgtaggtt atggaggtaa gtattttttt tgggttttat 5100

tgcgtggatt tgtatgagag attgagaaga aattgggaaa tacgaatgat tggattagta 5160
ttgaattaaa aggagaatat atatgtttat atgtatatat agtgtgtata tagcgtttta 5220
gcgtgtgtgt gtacgcgtgt atgagagagg agaggaggaa agaatggttt gtaggtgagg 5280
atacgtttgt atattagaat gaggatacgg ggatatttta aggagagagt aaattaatat 5340
gtttgagaat gtgtttgaga taaagataga ataagagtat tgttataatt tagtgtgttt 5400
gtgcgggtgt gaaaagtgga agagttaacg gggaattatt atgatgtttt tattagttaa 5460
tttgtaatga gaaaaaatgt gattaaaaag gttttgttta ttttgagggt ttttgggata 5520
ttttgtaata agggtatgat tgttttattt aaaagtagat ttttttttaa ttggattttt 5580
aaaaagaaaa tatagtgttt taatttgtta ttgtgttttt ttttttttgt tttttttttt 5640
ttgatttgtt aatagttttt tttttttttt tttgttgttg tttttaattt tattgtaaga 5700
gttgttgtaa ttatttttat tttgggggta gagatttggg gatggggggt tggggttttt 5760
agagaattgg taaattgaag atttaagtta tcgtgttagg tagggtagtt tagttatatt 5820
ttatagaagt tttcggagat taggttttta ggttgtttta acgtttaagt atatttgttt 5880
taaggatttt ttgatttagt gatagaaata gtatagtcgt ttagttttga gtaaatggaa 5940
gtaatagttg attaattggt ttgtcgtttg ttagttggag gtatgtagtt tatgttgttg 6000
ggagttaaag aaagattttg gtaggtaaga tagtagaaat ttttataagg ttttgaaaat 6060
tgaaaagt 6068

<210> 222
<211> 6068
<212> DNA
<213> Artificial Sequence

<220>
<223> chemically treated genomic DNA (Homo sapiens)

<400> 222

attttttaat ttttaaaatt ttataagaat ttttgttgtt ttgtttgtta gaattttttt 60
ttggttttta gtaatataag ttgtatgttt ttaattgata agcgataggt tagttggtta 120
gttgttgttt ttatttattt agagttgggc ggttgtattg tttttgttat taaattaagg 180
gattttgggg atagatgtgt ttgggcgtta aggtagtttg aggatttagt tttcggaagt 240
ttttgtgaag tgtgattggg ttgttttgtt tggtacggtg atttgaattt ttagtttgtt 300
aattttttaa gagttttaat tttttatttt tagattttta tttttagagt ggaagtgatt 360
gtagtaattt ttgtagtaga attgggggta gtagtagggg aggagagaag aggttattag 420
taaattaaag gaaagaaaat aggggaagga aaatatagta gtaaattaaa gtattatgtt 480
ttttttttaa aaatttaatt aaagggaaat ttgtttttga gtaaaataat tatgtttttg 540
ttataaagta ttttaggaat ttttaagata ggtaaaattt ttttaattat attttttttt 600
attataagtt ggttaatagg aatattatag taatttttcg ttaatttttt tatttttttat 660
attcgtataa atatattggg ttataataat gtttttattt tatttttgtt ttagatatat 720
ttttaagtat gttggtttat ttttttttta gaatattttc gtatttttat tttgatatgt 780
aaacgtattt ttatttatag attatttttt tttttttttt ttttttatgt acgcgtgtat 840
atatacgttg aaacgttata tatatattat atatgtatat aaatatatat gttttttttt 900
taatttaata ttaatttagt tattcgtatt ttttaatttt tttttagttt tttatatagg 960
tttacgtagt gaagtttaag ggagatattt atttttatag tttgcgggaa attttatgaa 1020
gtgagaataa tttttatata tgtttattcg ttagtttatg tttttttttt gtgtagtttt 1080
taaatattat cgtttttttc ggtttttagg tatagttcgg ttttttttaa tttgggttaa 1140
taagagtggt tatcgttttt tagtagttcg gggagtatag gttgggttag ttttttggga 1200
ttagttagga atttgttttt agatcggaga gtttatatag tgcgtttgag acggttgtgg 1260
cggtagtagt tggtgtttgt tagtaattac gtataatgtt gtcgtcgtcg tcgtcgtcgt 1320
cgttatcgtt atttttgtta tcgtcgtcgt tgttgtttga gattttgttg ttagtttgat 1380
taatttaggg ttgggttgtt gcgtgcgatt tttttagttt tcggcgcgtt tttagaggtt 1440
tttatcgggt ggtacgattt attatttagg aagttaatcg atgtatgatc gttcggtgta 1500
ggttaggaga gaggcgggag acggtagggg atggaagatt ttttaggtgt ttgttttttg 1560
atttatattt ataaaaatta atgggatttt attaaaatgt atttttttat tcgaattttt 1620
atttgtttaa tgtatttggt atttgatttg tgaaaggagg agggggtggg gtttaagaga 1680
gaatagggtt tttttagaag tataagatta gaaaagcgtg atatttaatt tttagttacg 1740
gaaaattaaa atagagtagt ttttaagttg aggtgggttt tttttgtttg ggtggtcgga 1800
agtatttatt tgtgttttaa taatgggagt ttgataagtg tagttggagt tagagtttat 1860
ttaggtggaa aatttatagg tagggaggag gggttggttt gatgtggagg tttttttttg 1920
tatttatttt atatattatt gttattttgt cgtttgttta aaaatattat tgggttttta 1980
ttaatataga gtttgttttg tgtggaattg attcgagatt aaagaattta agtatttcgt 2040
tgatgtttgg gtttgttata tagttttcgt gataagttat cgtttagttt atatttgaat 2100
atttttaata atggggagtt tattattttt ttattttatt tttagatagt ttttattata 2160
agaaagtttt ttatagagat aaaatttgtt tttttttttt atagtttttg tttataggtg 2220
ttagattgtt tttaggggtt atataagaga agtttgaaag ataaaaaagt tttttatagt 2280

gggatttttt gtaaattttg agattgattt gttgtgttta taattttttt ttttttaggt 2340
atttttggtt tttttagtaa ttttatgtat tattaagggt ttatgtttat ttttaattta 2400
tttttttttt aataatgtat tttagtttat atagatattt ttatagtgag gaagggggtt 2460
ggttagattt ggatgtaata ttttagatat ggtttaaatt ttttttttat ggtatatgtg 2520
ttggtacggt gatggggtgt tgttaatata tttattgaag gttcgtttta tgggttgagt 2580
tttgttgggt tggaaaaaag ataagagatt tttttttgat gtagtataag atatagagag 2640
ttagagaggt aagttaaatt agggttttag agtaaaaaat taagatttaa atgttaggag 2700
aagttaaaat aaaaacgtag aataaaagat tagagttagg aataaagtta tgatgataaa 2760
ggaggaggtt aaaatgggga attaaagtaa aagtatgtta taaaggagga agaaatattt 2820
aggtttttat tgtaaagagt tttggagagt ttagagttgt tttgattgtt gttgggatga 2880
gagtttagtg gatataagta atatagaata taggaggatt atttgtttat ttgtattagg 2940
tgggtagttt tgtatgttaa tttatgtaga attaattcga taaaaatttt tttattttta 3000
ttttttttaa gttgtgtttt tttatgtata ttttttattt tatttttggg taattggttt 3060
tgggatttat atgaaatgtt ttttatattt ttttgaagtt aaaatttttt ggttttattt 3120
attattttaa attttttagg attttgtttt tgttgtttaa aatatttttt atttatattt 3180
ttattttatg atttttgaaa atttgatgaa ttgttttttt atattttatt cgatttattg 3240
ataaaaatgt tgatatagat atagtaaaga taatatttta tattattaaa aagtttattt 3300
attagtgttt tagatatttt gttttgttat ttattagtta tatggttttg aaaggttata 3360
tgtatagtaa aagtattaat ttattaattt ataagttaat attgttgggt ataaattttt 3420
gtttcgttgt ttatagtttt atgatattgg taaatgagta aatttttttg agttttagtt 3480
attttatagg gttattatga ggattaaatg agttaatata tgttaagtat ttgaaatacg 3540
ttagataaat attaaatgtt cgatatatgt aatttaattt gaggtaaatg atggggagga 3600
ggaggaggtt aaagaggaag aggatgtaat tttaagtaag ttattttatg gttttttttt 3660
ttttttttat ttgtaaaatg atggagtaga gttatatggg ttttaatatt atttttagtt 3720
ttaaaattga acgtagatta atgtattatt tattttataa ttgtttattt tttttatagt 3780
ggtatttgaa gtaagtgttt tataatttat agttttttat tgtacgtatg ttagagaagg 3840
aaatgagtta tttaaattgt ttttttgtgt gtgtgtgaat ttatgtttta agggtttttt 3900
taagtattta agtatttaaa aattattttt taaaaattat agtttataga tttgtttaag 3960
gttattgtta agatgatttg ttggttaagt tattaataga tagatttttt ttttattttt 4020
ttttaagttt agtatattgt ttattttttt tttttataga agaaaaatta aattttaatt 4080
tggtatggaa aaggtttatt atttgtgggg tgtttcggtt tattatgtga gttttatgta 4140
gaggaaagtg ttttggggta gtaattataa ggtttgggat atatattttt tgggaaaaat 4200
atttatgttg ttagttttat taaggttttt ggatttagtt ttgattttgt ggattagatg 4260
tgttgagagt tgggtacgtg tttattttta tattgcgaag ttttatataa tggttttgta 4320
aaataaatgt aaagaattaa tatttttttg attttttttt tttttttttt ttgagagttt 4380
tttttgattt ggttgttttg tttattgtag gggtacggta tgtttatttt aattttcggt 4440
ttgatatagt ttggatgttt ttttttttta aattttatgt tgaaatgtga tttttaatgg 4500
tggaggtgga gtttagtggg aggtattgga ttatggggtg gattttttat gaatgtttta 4560
gtgttatttt tttggtgatg cgtgagtttt tgtttagtta gtttttaggg gatttggttg 4620
tttaagagtt tgggattttt ttttttattt ttttgatttt ttttttatta tgtgatatat 4680
tagtttttt tttgtttttt attatgattg aaagtttat gggtttattt aggagttgaa 4740
tagatattga tattatgttt ttttttaata gtttgtagaa ttgttagtta attaaatttt 4800
ttttttttat aaattattta gttttaggta tttttttata ataatattaa aaggattaat 4860
acgtagttta ttgtttatgt ttatttattt ttttttatta ttaaaatttt tatttattat 4920
ttaagtttta gtttattttt tattatgaaa atatttattt aaaaagatgt ttttttaatt 4980
taggtttgat aaaatttatt gtttagtgtg tttaggtgat atttagtatg tatggttttg 5040
tggtattttt agtgaaatgg taaggaatgg ttatttattt tttttttgaa tatgttttat 5100
ttttataata tgattgttaa ttttttaaat atagtgataa tgtattatgt ttttttgttt 5160
ttttatttaa gtgttaaaat atatagtagg tgtttaataa atatttttgt attaaaaatg 5220
tttgatatag ttaaatatat taaaaataat atattaaacg gtattttagt atgttttttt 5280
tatgatattt tttatatggt ttaatatttt ataataaatt ataagttttg tttaaagatt 5340
aataaatatg aatgtggttt ttttttttta atttattttt tttttttttg gtattattgt 5400
gtttttttta agattattat tggtataata acgttatttt aagtggttgg tatttttaat 5460
ataagttttt tttaaatagt tttattgatt ttagttataa ttgtgttttt agttgttatt 5520
gaatttttat taggatgtta agtgaaagat taaatgttta aagtttatag taggaattag 5580
ttaatttatt tataagaatt tttggtttat tttttgttaa ggttagttaa ttagtttttt 5640
taaataatat gttttatttt tattaaagtt attagtgttt ttgaataagt ggaaagagta 5700
tggattttga ttagtatgtt tggttgtttg atataaattc ggttaataat atttaatatt 5760
tattttggta attaaaggat atagttttac gtttgttttt tttttttttt ttgattcgtg 5820
gtgatataaa atagtatttt gtagtaaata taaatgaaaa attaaattta tttatttgat 5880
aatggttaat aaattttagt tatagaattt agatgtaaat atttttatat taatattatg 5940
tattaatatt tttatatttt tataatattt tatttatata atagtatatt aagtttttat 6000
gtgttatttt taatattaga tttaaatagg gtaggttttg ttattttagt tttataaatg 6060
ggtaattt 6068

<210> 223

<211> 10825
<212> DNA
<213> Artificial Sequence

<220>
<223> chemically treated genomic DNA (Homo sapiens)

<220>
<221> unsure
<222> (5015)

<400> 223

tattaggaga gtaattattt aatattgtta tttttatttg tttgtatttt aggttttttt 60
tcgtataagg atttttttat ttttaaaatt aataattatt tacgtatgaa tttttttttt 120
tatgaagttt ttatttaaaa gatttttttg tttggttttt tttttttttt tttttttgtt 180
tttgtgaatt tattaaattt ttttttataa tagaaatcga tttagaaaaa aaaaaattag 240
gttggcgtgg tggtttattt ttgtaatttt agtatttggg aagttgagtt gggtggatta 300
tttgagttta ggagtttgag attagtttgg gtaatatggt gaaattttgt ttttattaaa 360
aatgtaaaaa ttagttaggc gtggtggtgt atgtttatag ttttagttat ttaggaggtt 420
gaggtgggag gattgttgga gtttgggaag tggaggttgt agtgaattga gattttatta 480
ttgtatttta gtttgggtga tagagtgaga ttttgtttta aaaaaagaaa gaaagaaaaa 540
aaatttttta agtgtgaata aatggattta aaattgttta gtttcgtttt ttagaatttc 600
gttttgagag tgggtaggtg ttattttttt agtggagggg cggggagcgt tgtttttggt 660
tttttcgttt gggggttcgt tgagggtgtt tttttggttg ttttcgatag gaattgattt 720
taggatggtt tagtaagaaa atatagggag tagagttttt ttttaaggaa ggaagaatat 780
tttagtggat tgtttgaatt tgggggttat atttttaatt aaaaaaaggg atggttcgtt 840
agtttttgtt taaaaagggg taaaggattg ggtgcggtgg ttttttttcg taatttttat 900
tttttgggag ggttaaatag gaggattgtt tggggttagg agtttaaaat tagtttggat 960
aataaagtaa aatttttttg gtttaaaaaa aaaaaaaaaa aaaaaaaaaa aaaaagttag 1020
gtgtggtggt ttgtatttgg agttttagtt atttgggagg ttgaggtggt aggattgttt 1080
gagtttagga ggttaaggtt gtagtgagtt aaggttttgt tattgcgttt tagtttgggg 1140
aatagagtga gattttgtat taaaaaaaaa aaaaaaaaaa agatgaagag gtaggggtgt 1200
ttattttaag aagagatgaa gagatggggt tggaatttgt ttttaatttt aaatttttaa 1260
gatgtttttt agatattttt agattttagt tagtaggtta ggttagtttt ttaaagagtt 1320
agaaaggatt aggattatat atttatagag gtagaatgta gaagaagttt gtttaagtaa 1380
gagtatgatt aggttttaag gtatttgata acgaatatta ttttggtttt tttagatttg 1440
tttggaagtt taaggatgtg tttcgagtga ttttatatgt tgagaattta tatttttgag 1500
ttagtagggt agtcgggtag gtattaatag ttttgtttta tagatgagaa tattaagatt 1560
agagagagga agtgcgattt ttatgagtcg tttagttagt taggagtaga gttgaggttg 1620
tagtttgtgt ttattgattg tgggttagta tgtttttttt ggtattgttt ttgatttata 1680
gatatttagt tttaggcggg agtttttttt ttagtgttgt tggttgaggg agagcgagtt 1740
gaatgtattt ttggagagga tggtagatat tattgatgat atgatagtgg aatataatgg 1800
gagttgtggt tgggaaggtt ttttttttag tttttttagg agaagttagg aaaaagtttt 1860
gcgtgtggga agaggttatt tgggaggtat ttgggggggt tttttgtgat tttttgtgga 1920
ggttgaagaa ttgagaatgt tttttgaagt tggtgttttg aagtaataga ggacgaattt 1980
ttaggtttta tgtatgggta tttagtagtg tgggaaaagt agaatttttg ggaaatgaga 2040
aatataagtt aaagttgtat tttgtttttg tttatttaag ttatttaaaa aggagggagg 2100
atatgttttt tggtttatag tttttatatt taggaatttt tatatttggg agttttgtta 2160
tgttagttgt atgtttaaag ggtttttgtt ttagaatttt ttgttgtata tataggagtg 2220
cgtgtatata taaatatata tatatttgtt attaggaaaa gggggattgg attttgaagt 2280
gtaaagagtt gataataagt aaaaggataa aagtattgta tgtatttttg taatataaag 2340
tttttaggtt gttttgtttg gtgttgttat gtagtatgtg aatttttcga gaggacgggg 2400
ggcgggtggt ttttttttta tattttagga atggattaaa gtttttcggt gggattgagg 2460
ttttagggtt tttttttttt agttgggttt gggaggagtt aggagtgtat tggatttagg 2520
atttggggtt tttttttatt attttttgtt ttacggagag ataattgtgt ttttagtagg 2580
ggttagtaaa gtagtaggat tattaggggt gagtggggga ggtggtgatt ggaggtgatt 2640
aaagtttatt cggagtgatt agatataggc gaggtttttt tttgaatgga tttgtgagat 2700
ttatttttga tttgggattt ttagggtagt tatttggacg aatttttagt atttaatcgt 2760
ggatagatat aagttttttt gttattcggg aaaattgaaa acgtgagtga gcggttagtt 2820
aagtttgggt ttagaggtga gaggatgtta tttgtaaata tttatttttg tagtgagtaa 2880
atagacgtat ttggtttgag gtggagggtt tggttttagg agataaaaag attttttatt 2940
aaatagtatt tttttttaaa tgttttttga attgagatat agttgtcggg ttttttgcgg 3000
ttttattttt tgtgaggaat gtttttttta gttttatagg tcggttttat taattgtttt 3060
gggattaatt tttggttatt tcgattttgt tttttttttt ttgtgttttt tttttagttt 3120
ttatgttatt ttttgagtgt taaggtattt tttattggtt acgaaatgat agagaagtta 3180

tttatttgta gaagtttttt gtgtgtatcg tggaattatc gtgtttgtac gggtacgaat 3240
gttttaagaa aatagttttg tttttttata aattttagtt attttatttg gaatttgttt 3300
attaagaaat taggtttgta gagtttaaag taagtttttt agagttttat aaataagttg 3360
gcgggtaagg tagcgcgttt tatagtgttt ttgtggagcg ttaagatttg tgattttttt 3420
gttatatggg gtgtgcgttt agattgggtt atgaatatat tagttttttt ttattcgtat 3480
ttagggatag aagatatgga gattttagga attttatgaa ttatttgttg tggttttttg 3540
tttcgggata gttcgggttt tatttttagt tttttagagt ttaatttttt tttttagggg 3600
aatgtttgaa atttacgaag aatttttagg aattcgaggt agaaggtggt agagagattt 3660
ttgtttttgg gggtcgagtt gttgtgttga tatcgttttt ttggcgtttg ttttagtggg 3720
gatttttgat tttaatttga agtttttgga ttgggtttaa ttttagtatg tgtgtttgag 3780
tgatggattt ggtatttata ttagttacgt tgataagtgt atatgtgttt ttaatgtttt 3840
ggttttttat attataaata tatagatgtg ttgtcgttcg ggttaggatt tgagtagggt 3900
tttttttattt aaatatatat tatatattta aaaaagtgtt tttttagagt taatatcgtt 3960
gttagtagtt ttttttgtcg ttatatcggg taaagtttat ttattgtttt agtgttgagg 4020
gttattatgg gcggttttat ttggagaggt gttgtttata gtaaatagtt ttaattgcgt 4080
tttcgtttcg ttttttaggg agtttagtta ggtttattgg gtatttataa gtagattttt 4140
ttcgttttag tttttttgaa tttttgttag ttgggaaatt atttgtttgt atcgtagtta 4200
gagaatcgag gagaggagtc gttagtttaa agggttgttg gttgaaatta ggaagtagtg 4260
taaagaaaaa gaaaaaaaaa gtttgggagg ttaaggtagg agtattattt gaggttagga 4320
gttcgagatt agtttggtta atatggtgaa atttcgtttt tattaaaaat ataaaaaatt 4380
agcgggggta tggtggtacg cggttgtaat tttagttatt cgggaggttg aggtaggaga 4440
atcgtttgaa ttcgggaggc ggaggttgta gttagcggag atagcgttat tgtattttag 4500
tttaggtaat aagagcgaaa ttgttttaaa aaaaaaaagt ttttataatt ttatgggttt 4560
gtaagtatga tttaggtttt tcgttttttt gtaagttaat gcgagttaat tatagcgttc 4620
gttttggttt ttttttattt tacgtcgtga tttatttttt tttttttttt ttttttgttt 4680
tttttttttt ttttttttta tttatgtatt tttggttttg ttttttaagg ggtgttgagt 4740
cgcgtttggt tttagtaaat cgaattcgtt cgcgagggag gcgattggtt ttcgcgtcgg 4800
tgacggacgt ggtaacgagt gcggttcgtt tcgtcgggag ttgattggtt gcgcggggcg 4860
gtttcgaggg ttcggtcgta ggagtttcgc gtattttagt tttgtagttt tcggagttag 4920
tgtcgcgcgt tcgtcgtttc gcgttttttt gttcgtcgta ttttcgggag tcggggcgta 4980
tttagttcgt agcgtcgttt tttcgttcgc gtcgntttcg atcgtaggtc gagggtcgtt 5040
attggtcggg gggatcgggt agtagtttgc ggtcgcggag tcgggtaacg ttggggattg 5100
cgttttttgt tttcggaggt ttttggaagt ttgcggtagg acgcgcgcgg ggaggcggcg 5160
gaggtagttt cgacgtcgcg gagaataggg cgtagagtcg gtatgggtat cgggcgtagc 5220
gaggggggtc gtcgcggggt agttttgggc gtgttgttgg cgttgggcgc ggcgtttttg 5280
gtcgtgggtt cggttagcga gtacgattac gtgagttttt agtcggatat cggttcgtat 5340
tagagcgggc gtttttatat taagttattt tagtgcgtgg atattttcgc ggatttgcgg 5400
ttgtgttata acgtgggtta taagaagatg gtgttgttta atttgttgga gtacgagatt 5460
atggcggagg tgaagtagta ggttagtagt tgggtgtttt tgtttaataa gaattgttac 5520
gtcggtattt aggttttttt ttgttcgttt ttcgcgttcg tttgtttgga tcggtttatt 5580
tattcgtgtc gttggttttg cgaggtcgtg cgcgattcgt gcgagtcggt tatgtagttt 5640
ttcggttttt attggttcga gatgtttaag tgtgataagt ttttcgaggg ggacgtttgt 5700
atcgttatga cgtcgtttaa tgttatcgaa gtttttaagt tttaaggtaa ggttgttttt 5760
ttttacgtgc gcgagtcgtt ttttttattt ttcggtcgga gttgtaggag aattcgcgtt 5820
attttacgtt cgttcgtcgt tgggtttcgg tgttggggat tgagggttcg aggttgttcg 5880
tatgttgtgt acgtgatttt taagcgcgtt tggtttgttt taggtatcgt ttagcgtttt 5940
tcgcgattta ggtgtacggc ggggttggtt cgggagcggt agttttgcgt tttggtttcg 6000
tagggattcg ttgtggtttt cggagtcgtt ttttttggga tttaatttcg ttttttttat 6060
atttgaaata aagttttgcg ttttaaataa taggggagtt tttttttttt ttaggaggat 6120
ttcgagtttt tatcgaagtt tttaattttt gagacgggag gaaaaaataa aaatttaaag 6180
cgtagaaagt ttagaaaggt cgagagaatg tttggagtag tttttgttta tttttatagg 6240
ttcgataaag atttaaaaaa taaaattaag tttttttttt tattggttat ttagttaagt 6300
agttttggga ttgttgtaaa attggttgag aaagtttagg aatcgtgggg atttttgttt 6360
ttttattttt tttaggtatt agttttgttt tcggagggtt gggggtggaa agtttttttt 6420
tttttttttt agcgtatttt ttgttttttt tatttatata aatttttagt ttattagaaa 6480
gataaaaatg taattagttt attgtttatt ttttttcggg gagagtagtt ttgtaaaatg 6540
tagatttttt taatagtttg agggatcggt tcgaggtttt ttttaaaatt tttttatgtt 6600
taaagagtaa tttttgaggg ggaagaaaaa aaaattttgg gaagttaaat aaaatgaaat 6660
agatataaag ataaaatata tatattatag atttgatttt tatagaagta aatttaattt 6720
agaatttttt agaagcgaag tttaggtttt gaggtgtgga gagggttagg gattggggag 6780
ttgtatttta gttgcgttta gtatagacgt aggttttttg agggtttttt gaggagggtt 6840
tttagggtgg gggagaaaga ttgtttatat ttagtttatt ttaaagtgtt tttaattttt 6900
gttattgatg ttaaaagagg ggagtgtagt tatttggttt tttagagtga ggatttgggt 6960
aggtggggga aagggatatt ttaaatttta gatgtttttg tttattttag ggagggtgga 7020
gatgggttta tagtgattgt cgttggatgg ttggatggga acggtattga ttgttcgtag 7080
tggttaggtt tgggtatcgt tgtttttaga gttaattttg gtgttgggag tggagtgagg 7140

gtagcggatg tatttgtatt ttgtgagcga gaattgagta gttatttgga gtaaataaag 7200
cgttttgtgt tttgttggtt tgtatttatt ttttgtagtt tgcgggaatt tttttttttt 7260
tttttttagt tagcggggat tagtttagtt ttgttttgtg aggttgtgtt tagttttgat 7320
ttggagttta tttttaatgg ttttcgatgg tttttttttg ggatcgtttg tagatatgag 7380
tggatatttt agggggttcg gttgcggaaa aggtgttgta gtttataggg aatatggggg 7440
gattggacgg gttgtttgaa gaagagagga aagaattttt tattttagtt tttaaaaggt 7500
tgtgatggga tggggaaatt ttataatcgt tgtttttcgg atattttttg ttttttggtt 7560
gtagttttat tggtcggcgt tttttttagt ttggtttgga atcgttttta tttattttta 7620
ttttttttag tagcgtttag gtagttattt ttttaagata ggtttttgag gagtaaagag 7680
aaaagtgggg agagggtcgg tcgggagtgg ggtttgggat tggttttttt tttagtggtt 7740
ttggtggggg gttttgtttt gagatggtat attatttttt ttaggattgt aatttttggt 7800
gtaagggagt tttacgtgat tttttgcgag gtttttgtgg aggggagggt ggggcgtgtg 7860
tgtgtgtgtg tgtgtgtgtg taggtgtgta gagggtaggt taagataaat tagattattg 7920
atttttataa ttttatggtt tgtggttgtg gggtcgtaat tggtattttt taatgagaat 7980
aagtatgtat gtattatttt tttattcgtt tattttttat gtttttattt tttaaaatta 8040
aaatgtattt ttaaagtagt gttatatttt tgtatattgg tttttgttta tatatttggg 8100
taaatttagt tttggattat tggttagttg tttgtggtta tgtgttatta aggagaaatt 8160
tgataatgag aaatgatttg atattttttt tttgtaaagg ttgatattta agttatagaa 8220
gtttttgtta gggaagaggt aattgttttt tttttaggga agttattttt gaatttggta 8280
tttttattag aattgtgaat ttttaggttg atgtattgtt gttttttttt ttgtttggtt 8340
ttattgggtg aagttgtatg agttagtata ttgggtttga ttttgttgtt gtttgggtat 8400
gttgtgtatt tagttagtgt ggtagaggtt atagagtttt ttgtttttta gaaagttttt 8460
tttagggttt ttttttggtt tttttaggaa ttggttaggg aagattgtgg gggttgtgtg 8520
tgagaaaggg aaagagagaa gaaagagaga gagattgaga aagtatttgt gtgtgtgtgg 8580
attgatgtat tataaaaatt atttaatttt gatttagttg ttgggttgtt ttttttatat 8640
atttttaaat attaataaat ataaaatttt tgtttttttt gtttatttgt tttaaatatt 8700
attttaaata taattgaaaa tagcgattta gttttatttt ttagaaggat gattgggtgg 8760
attttttttg tagaggtagt ttgtagagtt ttaagacgta ttaatgaggc gtaggttaat 8820
atcgtattat atattttttt tttttttttt ggattaggtt gttttagagt tagtcgttag 8880
aaaagttgag ataggggaaa gtcgtggtaa aaatatatag agtttatttt aacgagtttt 8940
tgttattttg agaggtattg aagtttaaat gtaattgacg ggattgagga aaatgttttt 9000
gagaagtgta gagttttttt tggtcgattg gtttaggcga attttaagtg tttagaggag 9060
atgtagtgag tgtgtggagc gggtagagat gtgtttttta tgtttggatt attttgattt 9120
tattatttgg ttaagattgt atttatagaa cgttgttatg tgtgtttgtg taatttttat 9180
tttttttttt ttaaaataat ttaaataagg aaaatttaaa agtatagttt attttttttg 9240
tgtaagtaat ttggtttttt ttatattgtt atattttttt ttttttatgt ggaagtaaaa 9300
attataatgt aaaatttgtg gtaagttgtt ttttttgtga gaattattat ttttagaatt 9360
tagttttaag ttttgggtgt aaagtaaatt tttagtattt gggttaagga tagttatgtt 9420
atcgtttgtt gggagcgtgg tttttaattg tattgagaat ttggaaggtg tttgtttttg 9480
ggtgtagagt aaaggtggga gaggaatgtg ttaataaaaa ttaaggatgg ggtttttgtt 9540
gggttttgta gggattttgt ttggaagtag ggagtggttt atgggttatt tgtgttttga 9600
gggtagtttt gatttggggg tgttttgtag gttttttttt tttttttatt tagggagttt 9660
ttgtttattt taatgtttga gttataaggg gggttgatgg ggagggagat gttgttacgg 9720
aagttatggt ggaggaggat gaagagtagt gggttttaag tatattaagt tttatttgta 9780
tttttcgagt gtagggttag cggtgatttt tttgtgtttt ttagttttag tttggtttgg 9840
ttttttgtgt tgtgttcggg ggtggggtgt ttttagaatg tgttcgtgat gtttttttag 9900
atagtcgata agttttaggt tgtttgatag gtgaaggtga tgataatgat gataagttat 9960
tttagttttt ggaggaaagc gtcgtttcgt tttttttttg ttttgggaag ttggagtttt 10020
ttatttttat tggatttttt gattttagta gtatattggt ttggaggagg tttttgtttt 10080
ggttttagat attttttttt aggtagtgga gttgtttttt aacgttatgt acgtttgttt 10140
ttataaattt tttttttaaa gtattttatt agttgtttag gaaagagttt ttgaaggatt 10200
tttttttatt tagttttgtt tttttatttg gtagcgtttg ttagtgtttt atttcgttcg 10260
tttgttttgg ttttttgttg cggtggtttt tttggagagg aggtgtttag gggtcgagag 10320
ttggagtttg tgagtttggt tgagtttttt ttttttttgg tttttggaga tttggttaga 10380
gggattagta ggttagttgg gagtgtggaa atgggaaggg ggtaggaatc ggaggatggt 10440
attgaaagga gaagggagag gatgggaaat aaatattagg aagagatttt tgtgggtttg 10500
taagttagtt tttttcggta tttatagcgt tttataagtt attatttatg tgatttgtat 10560
ttatttagtt ttaggttatg ttggtttaaa taattgtgtt atatttagta gttttgggtt 10620
aaaatgtttt aagaaatttg cgtttgtttt ggttgtagat tttgggttta aggttttttg 10680
tttttaattt ttaggttttt tttagaagtt ttagaagatt tttagttatt tttagtttgg 10740
atttagggtc gatttttttt ttaggggttg tttttttttt gttttgtaat ttatagtttc 10800
gttttttttt attttttttt ttagg 10825

<210> 224
<211> 10825
<212> DNA

<213> Artificial Sequence

<220>
<223> chemically treated genomic DNA (Homo sapiens)

<220>
<221> unsure
<222> (5811)

<400> 224

tttgggaaag gaagtaggga gaaacggaat tgtaagttat agagtaggaa gggagtagtt 60
tttggggaaa agatcggttt tgggtttagg ttgaaagtgg ttaaaggttt tttggaattt 120
ttgaagggga tttgagaatt gaaaataaga agttttgagt ttagggttta taattagagt 180
aggcgtaggt tttttgaaat attttggttt agagttgtta aatgtgatat agttatttaa 240
gttagtataa tttgaggttg ggtgggtgta ggttatatga atgatgattt gtggggcgtt 300
gtgagtgtcg ggaaaagttg gtttataaat ttatagagat ttttttttgg tgtttgtttt 360
ttattttttt tttttttttt ttaatattat ttttcgattt ttgttttttt tttattttta 420
tatttttagt tagtttgttg atttttttga ttaggttttt aggagttaag agagaaagaa 480
atttagttag atttataaat tttagttttc ggtttttgaa tatttttttt ttaaaagggt 540
tatcgtagta ggaggttagg gtaggcgggc ggggtggggt attaataggc gttgttaaat 600
gggaggatag ggttggatag gaaggggttt tttagaagtt tttttttggg taattggtga 660
agtgttttga agaggaggtt tgtgggggta gacgtgtata acgttggaag gtagttttat 720
tgtttgggag gagatgtttg aggttagggt agaagttttt tttaagttaa tatattgttg 780
aagttagaaa atttagtgga ggtaaaggat tttagttttt taggatagag ggagagcggg 840
acggcgtttt tttttaagag ttgaagtggt ttattattat tgttattatt tttatttatt 900
aagtagtttg gagtttatcg attgtttagg aaaatattac gagtatattt taggggtatt 960
ttattttcgg gtatagtata ggaggttaag ttaggttgga gttgaagagt ataggaaagt 1020
tatcgttgat tttgtattcg gggagtgtag gtggggtttg gtgtgtttgg agtttattgt 1080
tttttatttt tttttattat ggttttcgtg gtagtatttt tttttttatt agtttttttt 1140
atggtttaga tattgaaata ggtaaaggtt ttttaagtgg aagagaagag gggatttgta 1200
gagtattttt agattaaggt tgtttttagg atataagtgg tttatagatt attttttatt 1260
tttaaatagg gtttttgtag ggtttagtag agattttatt tttagttttt attgatatat 1320
ttttttttta tttttatttt gtatttagga gtaaatattt tttagatttt tagtgtagtt 1380
aagaattacg tttttagtag acgatggtat gattgttttt agtttagatg ttggaaattt 1440
gttttgtatt tagaatttga gattgggttt tagaggtggt aatttttata aaggagataa 1500
tttattataa attttgtatt atggtttttg tttttatatg gggaaaagga agtgtgataa 1560
tatggaaagg attaggttat ttgtataagg agagtgaatt gtgtttttgg gttttttttg 1620
tttaggttat tttgaaaaag gaaaagtggg agttatataa atatatatag taacgttttg 1680
tgaatataat tttgattaaa tagtgaagtt agggtgattt aaatataaag ggtatatttt 1740
tgttcgtttt atatatttat tgtatttttt ttggatattt gaagttcgtt tgaattaatc 1800
ggttaaggag ggttttgtat tttttaggga tatttttttt agtttcgtta attatatttg 1860
gattttagtg ttttttagaa tggtaggagt tcgttggggt gggttttgtg tgtttttatt 1920
acggtttttt tttattttag tttttttaac ggttgatttt ggagtagttt gatttagaga 1980
gagaaagaga aatgtgtgat gcggtattaa tttgcgtttt attggtgcgt tttggggttt 2040
tataggttgt ttttgtagga gaaatttatt tagttatttt tttgagaggt ggaattaaat 2100
cgttgttttt agttgtattt aaaataatgt ttaaagtaaa tgaataaaaa aagtaaaggt 2160
tttgtgttta ttaatattta agggtgtgtg gaggagatag tttaatagtt gaattaggat 2220
taagtgattt ttgtgatata ttaatttata tatatataag tgttttttta gttttttttt 2280
tttttttttt tttttttttt ttttatatat aatttttata attttttttg gttagttttt 2340
ggggggatta aaagggagtt ttaaggaagg tttttggggg aatagagggt tttgtggttt 2400
ttgttatatt gattaaatgt ataatatgtt taagtaataa taaaattaaa tttaatgtgt 2460
tggtttatat aattttattt agtaaaatta ggtagagagg gaaataataa tgtattagtt 2520
tgggagttta tagttttaat ggaagtgtta aatttaaaaa tgattttttt agaaaaagag 2580
taattgtttt ttttttaata ggagttttta tggtttgggt attagttttt gtaggggaaa 2640
aatgttaagt tattttttat tattaggttt ttttttaata atatatgatt atagatagtt 2700
ggttaataat ttaaaattga atttgtttaa gtgtgtgaat agaaattagt atgtaaagat 2760
atgatattgt tttgaaaatg tattttaatt ttaaagaata aaagtataaa gaataaacga 2820
ataaaagagt agtgtatgta tatttatttt tattggaaag tgttagttac gattttatag 2880
ttataagtta taaggttatg aggattagta gtttggtttg ttttgatttg ttttttatat 2940
atttatatat atatatatat atatatatac gttttatttt tttttttata gggatttcgt 3000
aggaaattac gtggggtttt tttatattag ggattgtagt tttggggagg gtggtgtgtt 3060
attttagggt agagtttttt attaaaatta ttgaggggaa aattagtttt aagttttatt 3120
ttcgatcggt ttttttttta tttttttttt tgttttttaa agatttgttt tgggagggtg 3180
attatttgag cgttgttggg aggagtggag gtgagtgagg acggttttaa gttaggttga 3240
gaagggcgtc gattagtgga attgtagtta aggggtaaaa ggtgttcgga agatagcgat 3300

tatggggttt ttttatttta ttatagtttt ttgggggttg gggtgaggga tttttttttt 3360
ttttttttag gtaattcgtt taattttttt atgttttttg tggattgtag tatttttttc 3420
gtagtcgagt tttttaaggt gtttatttat gtttgtagac ggttttagaa agagattatc 3480
ggaagttatt gggaatggat tttaggttag ggttgggtat agttttatag ggtagggttg 3540
ggttaatttt cgttggttga ggaaggggag aaggaaattt tcgtaggttg taggggatgg 3600
gtgtaagtta atagaatata aggcgttttg tttgttttag gtaattgttt agttttcgtt 3660
tataaagtgt agatgtattc gttattttta ttttattttt aatattagag ttgattttga 3720
gagtagcggt gtttagattt ggttattgcg ggtaattagt gtcgttttta tttagttatt 3780
taacgatagt tattgtggat ttatttttat tttttttaga ataagtaaaa gtatttaaaa 3840
tttggggtat tttttttttt tatttgttta aatttttatt ttgaggagtt aggtgattgt 3900
attttttttt tttagtatta ataatagagg ttaaagatat tttaaggtga gttaagtgta 3960
ggtagttttt tttttttatt ttaaaggttt tttttaaaaa gtttttagga agtttgcgtt 4020
tgtgttaagc gtagttgggg tatagttttt taatttttga ttttttttat attttagagt 4080
ttaaatttcg tttttaaaga attttgggtt gaatttattt ttatagaagt taggtttgtg 4140
atgtgtatat tttattttta tatttatttt attttatttg attttttagg attttttttt 4200
tttttttttt aagaattgtt ttttaaatat aagggggttt tgagaggaat ttcgggtcgg 4260
ttttttaggt tgttaggaaa gtttgtattt tgtagagttg tttttttcgg gaggagatga 4320
ataatgagtt aattatattt ttattttttt aatgagttga ggatttgtgt aaatgagaaa 4380
agtagggggt acgttagagg gagggagaaa agatttttta tttttagttt ttcgagggta 4440
gagttggtat ttggaagggg tggagaaata gaagttttta cggtttttaa atttttttag 4500
ttagttttgt agtaatttta aggttatttg attgaatgat tagtgaagga aagggtttgg 4560
ttttattttt taaatttttg tcgaatttat gaaaataaat aaaagttgtt ttaagtattt 4620
tttcggtttt tttgaatttt ttacgttttg ggtttttgtt tttttttttc gttttagagg 4680
ttaaaaattt cgatagggat tcggagtttt tttagaggag agggaagttt ttttgttatt 4740
taaagcgtaa ggttttgttt taggtatggg aaaggcgaag ttggatttta ggaagagcgg 4800
tttcggggat tatagcgagt ttttgcgagg ttaaggcgta gagttgtcgt tttcgggtta 4860
gtttcgtcgt gtatttgggt cgcgaggggc gttgagcgat atttgggata gattagacgc 4920
gtttaggaat tacgtgtata gtatgcgagt aatttcgggt ttttagtttt tagtatcggg 4980
atttagcggc gggcgggcgt agggtggcgc gggttttttt gtagtttcgg tcgggggatg 5040
gagggggcgg ttcgcgtacg tgggaggagg tagttttatt ttggggtttg gaggtttcgg 5100
tggtattggg cggcgttatg gcgatgtaga cgtttttttc ggggaatttg ttatatttaa 5160
gtatttcggg ttagtagaag tcgaagaatt gtatgatcgg ttcgtacgag tcgcgtacgg 5220
tttcgtagag ttagcgatac gggtagatgg gtcggtttag gtagacgggc gcgaagagcg 5280
agtagaggaa gatttgggtg tcggcgtggt agtttttgtt gagtaggggt atttagttgt 5340
tggtttgttg ttttattttc gttatggttt cgtgttttag taggttgggt agtattattt 5400
ttttgtagtt tacgttgtgg tatagtcgta ggttcgcggg gatgtttacg tattgaggtg 5460
gtttggtgta gaagcgttcg ttttggtacg ggtcgatgtt cgattggaag tttacgtagt 5520
cgtattcgtt ggtcgagttt acggttagaa gcgtcgcgtt tagcgttagt agtacgttta 5580
gggttgtttc gcggcggttt ttttcgttgc gttcgatgtt tatgtcggtt ttgcgttttg 5640
tttttcgcga cgtcggggtt gttttcgtcg ttttttcgcg cgcgttttgt cgtaaatttt 5700
tagggatttt cggggataaa aggcgtagtt tttagcgttg ttcggtttcg cggtcgtaag 5760
ttgttgttcg gttttttcgg ttagtggcgg ttttcggttt gcggtcgaag ncggcgcggg 5820
cggggaggcg gcgttgcggg ttgggtgcgt ttcggttttc ggaggtgcgg cgagtaggaa 5880
ggcgcggggc ggcgggcgcg cggtattgat ttcggaggtt gtagggttgg agtgcgcggg 5940
gtttttacgg tcgagttttc ggagtcgttt cgcgtagtta attagttttc ggcggggcga 6000
gtcgtattcg ttattacgtt cgttatcggc gcgggagtta atcgtttttt tcgcgagcgg 6060
gttcggttta ttagaattag acgcggttta atatttttta aaaaataaaa ttaaaaatat 6120
ataaataaaa gggggaggag gaaagagata aggggagaaa aagaagggga atggattacg 6180
gcgtggggtg gagagagatt agggcggacg ttgtaattaa ttcgtattgg tttgtagaga 6240
agcggggagt ttggattata tttgtaaatt tatgaaatta tgaagatttt ttttttttga 6300
gatagtttcg tttttgttgt ttaagttgga gtgtaatggc gttattttcg ttgattgtaa 6360
ttttcgtttt tcgggtttaa gcgatttttt tgttttagtt tttcgagtag ttgggattat 6420
agtcgcgtgt tattatgttt tcgttaattt tttgtatttt tagtagagac ggggttttat 6480
tatgttagtt aggttggttt cgaatttttg attttaggtg atgtttttgt tttggttttt 6540
taaatttttt tttttttttt ttttatattg ttttttaatt ttaattaata gttttttaga 6600
ttagcggttt tttttttcgg ttttttagtt gcggtgtaga taggtggttt tttaattaat 6660
aggggtttag gaaggttgaa gcgagggagg tttgtttgta aatatttagt gggtttggtt 6720
gggttttttg gaaggcgagg cgaaggcgta gttggagttg tttgttgtga gtagtatttt 6780
tttaggtggg gtcgtttatg gtggttttta atattggggt agtgggtgga ttttgttcgg 6840
tgtggcggta ggaagagttg ttagtaacgg tattagtttt gggaggatat ttttttaaat 6900
atataatata tatttaaata gaaaaatttt atttaagttt tagttcgggc gatagtatat 6960
ttgtgtgttt gtggtgtgga aagttaaaat attaaaaata tatgtgtatt tattagcgtg 7020
gttggtgtaa atattaagtt tattatttag gtatatatgt taaggttaga tttagtttag 7080
gaattttaag ttaaaattaa gggtttttat tagggtagac gttaagagga cggtattagt 7140
ataatagttc ggtttttagg aatagaagtt tttttgttat tttttatttc gaatttttga 7200
gaatttttcg taggttttaa atattttttt gagagggagg gttaagtttt ggagagttgg 7260

gagtgagatt cggattattt cgggatagaa gattatagta aatagtttat gaagtttttg 7320
gagtttttat attttttgtt tttaagtgcg ggtggagaga ggttgatata tttatagttt 7380
agtttgagcg tatattttat gtggtaaagg ggttatagat tttagcgttt tatagagata 7440
ttatagggcg cgttgttttg ttcgttagtt tgtttgtaaa gttttaggga gtttgttttg 7500
gattttgtaa atttaatttt ttgatggata gattttaagt gaaatgatta gagtttgtga 7560
agaagtaggg ttgttttttt aaagtattcg tgttcgtata gatacggtga ttttacggtg 7620
tatataggag gtttttgtag atggatgatt tttttgttat ttcgtggtta atggaagatg 7680
ttttgatatt taaaaggtga tatggaggtt ggaaggaaaa tatagagaag agagagtaag 7740
atcgggatgg ttaagggtta attttaagat aattggtgag gtcggtttgt ggagttgagg 7800
aagatatttt ttatagagaa taagatcgta ggagattcgg taattgtgtt ttaatttaag 7860
gggtatttag agaaaaatgt tgtttggtag gggggttttt tgttttttag ggttaggttt 7920
tttattttaa gttaaatgcg tttgtttgtt tattgtaaag atggatgttt gtagataata 7980
tttttttatt tttgaattta ggtttagttg atcgtttatt tacgttttta gttttttcga 8040
atggtaggag gatttgtgtt tgtttacggt taaatgttgg aaattcgttt aggtgattgt 8100
tttggaagtt ttaagttagg agtaaatttt ataaatttat ttaggggaag atttcgttta 8160
tgtttggtta tttcgagtag attttgatta tttttagtta ttattttttt tatttatttt 8220
tagtggtttt attgttttat tgatttttgt tgggaatata gttgtttttt cgtggaatag 8280
ggagtgatgg gagaagattt taaattttaa atttagtgta tttttgattt tttttagatt 8340
tagttggaaa ggaaggattt taagatttta gttttatcga gagattttaa tttatttttg 8400
ggatgtggga gagggattat tcgtttttcg ttttttcggg aaatttatat gttgtataat 8460
agtattaggt agaatagttt gaagattttg tattgtagag gtatatatag tatttttgtt 8520
tttttattta ttgttaattt tttgtatttt aaaatttagt tttttttttt ttaatggtaa 8580
gtgtgtgtgt gtttgtgtgt gtacgtattt ttgtgtgtat agtagaaggt tttagaataa 8640
aggtttttta aatatgtaat tgatatgata gagtttttag atatgagggt ttttgagtat 8700
gagaattgtg ggttaggaga tatatttttt tttttttttg aatggtttaa ataaatagag 8760
gtaaaatgta gttttaattt atgtttttta ttttttagaa gttttgtttt ttttatattg 8820
ttagatgttt atgtatgaag tttgaaagtt cgttttttgt tgttttaaaa tattagtttt 8880
aggaggtatt tttagttttt tagtttttat agagggttat agagggtttt tttaggtgtt 8940
ttttaggtgg ttttttttta tacgtagagt ttttttttgg ttttttttgg aaaaattggg 9000
aggggagttt ttttagttat agtttttatt gtgttttatt attatattat tagtgatgtt 9060
tgttattttt tttaaagata tatttagttc gttttttttt agttagtagt attagggagg 9120
gagttttcgt ttgaagttgg gtatttgtgg gttaggggta atgttaagga aagtatgttg 9180
gtttatagtt agtggatata ggttatagtt ttagttttgt ttttaattgg ttgggcgatt 9240
tatgggaatc gtattttttt tttttggttt tagtgttttt atttgtgaaa tagagttatt 9300
aatgtttgtt cggttgtttt gttagtttag aagtgtgaat ttttagtatg tagggttatt 9360
cgaggtatat ttttagattt ttaggtaagt ttgaaggggt taaagtggta ttcgttgtta 9420
gatgttttga aatttggtta tgtttttatt tggataagtt ttttttgtat tttgtttttg 9480
tagatgtgta attttgattt tttttaattt tttaaagaat tggtttgatt tgttaattga 9540
agtttgggaa tgtttgggaa gtattttaaa aatttggagt tgaaggtaaa ttttagtttt 9600
atttttttat ttttttttag agtaagtatt tttatttttt tatttttttt tttttttttt 9660
ttttgatata gagttttatt ttgtttttta ggttggagcg tagtggtagg attttggttt 9720
attgtagttt tgattttttg ggtttaggta attttgttat tttagttttt taagtagttg 9780
ggattttaga tgtaggttat tatatttagt tttttttttt tttttttttt tttttttttt 9840
tggattaagg ggattttgtt ttgttattta ggttggtttt gaatttttag ttttaagtaa 9900
tttttttgtt taattttttt aaagggtggg gattacgggg gggagttatc gtatttagtt 9960
ttttgttttt ttttggataa aagttaacgg attatttttt tttttggttg gggatatagt 10020
ttttaagttt aaataattta ttaaaatgtt tttttttttt tgaagggaaa ttttgttttt 10080
tgtatttttt tattaggtta ttttaggatt agtttttatc gaaagtagtt aggaagatat 10140
ttttagcgga tttttaaacg gggaggttaa ggataacgtt tttcgttttt ttattaaaaa 10200
agtaatattt gtttattttt aaggcgaggt tttggaaaac ggggttggat aattttgggt 10260
ttatttattt atatttaaag ggtttttttt tttttttttt ttttttgaga tagggtttta 10320
ttttgttatt tagattggag tgtagtggtg agattttagt ttattgtaat ttttattttt 10380
taggttttag taattttttt attttagttt tttgagtagt taggattata ggtatgtatt 10440
attacgtttg gttaattttt gtatttttag tagagataga gttttattat gttgtttagg 10500
ttggttttaa atttttgagt ttaagtgatt tatttagttt aattttttaa gtgttgggat 10560
tataggaatg agttattacg ttagtttgat tttttttttt ttgagtcggt ttttattata 10620
aagaaagatt taataggttt atagagatag gaaaggagaa aaaaaaaaag ttaaataaaa 10680
aagtttttta aataaggatt ttataaaaga gagaatttat acgtaagtag ttattaattt 10740
tagaaataag ggaattttta tgcgagagaa ggtttagagt gtagatagat aagaatggta 10800
gtattggatg attgtttttt taatg 10825

<210> 225
<211> 7323
<212> DNA
<213> Artificial Sequence

<220>
<223> chemically treated genomic DNA (Homo sapiens)

<400> 225

atttaaaagg agtagattaa aattttaatg ttgtaataat ttgttaagtt tgtgggtgaa 60
attttttttt tattattttt ttatatcgtt tgtaattttg ttatattttt ttaaaatata 120
aaaagtattt tttagaaaat atatattttg cgtgtttttt tagtagatgt ttttagaaag 180
aaatgagata gcgtagaaga tacgttttag attgtagggg gttttatgag tttagagttt 240
gatttagtag cgtgagaata ggggcgaatt tgggtatggg gtattttgta gtttgatttc 300
gttttggatg ggtttttttt ttatttgtta tcgtttcgtt gggtgttatt gagtttttcg 360
ttagtaggtc gttttcggtg ggcgtagcgt tttttttagt ttcggattat gttattttta 420
agagattttt ttcgcgaaag atttttttgg gagaagggag gagttgtaag gggtcggaaa 480
gagttatagg gcgtaggttt ttgcgatttc gtttttttta tgttcgcgcg gttttttatt 540
ttcgtttcgc gggtcgatga tttgtatttt ttgtagggga ttagcgtttt tttttcgttt 600
agtattcgta cgcgttggtt gagttcgtcg taggagagta gggcgcggtt gttgcgttcg 660
ttattttgtt ggtatataat ttggcgtttt ttttattgtt gcggcgtttg gtacgggcgc 720
ggggttttta tcgtttttag gttgtatagg tcgtataggg tttaggttta gaggtcgttt 780
agtagttagg tatttagggc gttcgggacg gtgcggaggg gagcgcgttt tggtatcgcg 840
gttagttttt ttcggttcga gggtaagttt aagtttaagg gggcgttgag gcgttcggga 900
ttagagtggt tttcgtcggt ttttttcgta ttgtgtcggg agtgtggttt ggattaaagt 960
gttttttgtt ttttcggtgg tttttgtcgt ttttgagatt cggacgcgcg agtttcggta 1020
gtcgggatta gggttcgggg aaggttttag tgaatcgcgc gggagttatt tttgtttggg 1080
ttattgtttg ttgttattgt ttgttgttat tgttttttgt cgtttcggtt tttattgttt 1140
attgttgtta ataggaaatg tagtggtagt ggtgttatta gtattttggg aggtatcgtg 1200
ttttaagagg ggggatgggg ggagattgtg ttgagaagtg tgttttttta ttaattttag 1260
tgtttaatag gtaattatat agggaggggt aagtttaagg ttaagatagg aaaagtagag 1320
ttgttgcgtt tgttgtgtaa acgtttgttt cgtttatatt gtttttcgat aaaattgtaa 1380
attttttttt ttaaattaaa agtggaaaga cgagtttttt tttgaagaaa tgatattagt 1440
tatttattat ggtcgttttt tagtttttat ttttagtgaa gagtttagga gattagtaga 1500
tagttttttg tattgggttt aggatttatg gatatagttt taaggttata ttgtagtgag 1560
attgtttttt ttatcgtttg gtaatattat tagtcgagtg gtttgggtgt ttggggggtt 1620
tcgttttttt aagaataagg ttaattgggt ttatagttat gtaatgttag gatttataat 1680
atggaatgag cgaggtttat atatttgtag ttttagttat ttttgaggtt gaagtgggag 1740
gatcgtttga gtttaggagt tagaggttgt agtgagttat gattatgtta ttgtatttta 1800
tttagtttgg gtgatatggc gagattttgt ttttaaaaat atagtgataa agtatgaaat 1860
taagaaaaaa gaaaggaatt aaaaaaatgt atagcgtttt tttatttgaa ttgtatttta 1920
aggatgatta agattgagtt aggtgaaagt gggagataat atttttgaat gaagggtata 1980
aggataagaa aagaataatg ttggggttta aggataaggt agaggagtat tttgaagtta 2040
tagtatttat tttaaggaat ttgttattga tattgaggat tgtgggaatt attaattaat 2100
gtaaagttag ggaggagtat tattaggtat atgttttaga aagtttattt agataataga 2160
aagggtaggg ttagagggaa ggtagggaga ggaggaagtt attgttatta ttttggaaag 2220
agatgatttt gatttagtta aggtagcggg agtggttatg gaaagggtgg aagataggtg 2280
ggagttgtag agaagtattt taatttagag agagaggttt ttggtttggg ttattgggaa 2340
tttagttttg agtgaggaga gatgattaat ttattttagg attggttgat tttgagttga 2400
ttgtggagta ttaaggtgga aatatttaag ggatagttgg ataaatattt ttggaaatta 2460
gtagagtttt tggattagga agttataggt atataggtgg taaatgaagt cgaagagtag 2520
aatagtggtt tttggggaaa ttttatattt ttttttttag taaagaaata tttggtaata 2580
tttggagata tttttgatga ttataatgta gatggggagg aggttatcgt tgatattttg 2640
tgggtagagg ttaaggatgt tattaaattt tttataatgt gtagtatagt tatttttttt 2700
tttttaaatg aagagaaaat tatttggttt taaatgtttt aagtgttggg ggtgagaaaa 2760
tttggagttt aggggagtgt tgagtgtgag aagaaggatt aggatggagt tttagagagt 2820
tttagatatt taaaggatgg gtaaagaata ggtatttgag gagattaaaa ggagttatta 2880
gagagaagtt attgaatgag gtaatttttc gagttttgtt aaataaatgt aaggtatgga 2940
aagaatggat tgaatgttga agtagggtgt tttagaatta tagttttttt ggaagtttcg 3000
aagaggaggg atagttgata ggtttttttg atgtattttt ttttgttgtt ttttgtttgt 3060
cgaattatta gagatattat taatgggtcg aatggggtag gtataaagtt ttaggaagaa 3120
gggtttttta ttttttttta ttttttttta aagggtatgt tttagagaat attagggaag 3180
tttataagat tttgtgattt ggtatttata tagttaaata gatgtagtag gtttaaattg 3240
gttgtagaaa tttgagtttt tttcgagttt gttttgttta gatttatagg aaaagtgtag 3300
ggtagtttag tttttttttt tgttttttag gtaggaattt tagttgaaaa ttttttttat 3360
ttagtgaagg gtttagattt attggagttt attgttagaa atggtttgag atgttatttg 3420
ggtttttttt tttatgtgat attttattag tttggtttgg tatttttgga ggtggagatt 3480
ggttttatag gaattagatt atttttttag agtggtttgg tagtgtaaga atattttggg 3540
ttttttttaa ataagaatta gattttttgg atgaattttg aaatgtattt gttttattta 3600
ttttttttat ttattatgtt taatttatgt tttttaaaaa ataagataat gtatttaaag 3660

tttttagttt tatattttga ttattgtaaa tttttaatag atgttatttg tgtttgttgt 3720
ttttttagtt gttttaattt ttagttttta gttttaattt ttagttaaaa ttaaattaat 3780
tgtgtaggtt taagttttgg agagaaaaat tatgtgtttt aaatttaagt taaattatgt 3840
ttttaaagat taatatttag agaatatgtt tttatattgt tgatgtttta ataaatagta 3900
tatattagaa gttttattat ttgaagattg atatatatta attttatata aataaatttt 3960
tttttttttg tagatagttg agttagtaat tggattattt ttttttaatg taagtaagat 4020
aaaagagaaa gtaaaatata tatatttgta ttttatagta tatagttttt tatttaattt 4080
aagtaatagt ttttgagtat attaaaaatt aagattttag gttgttgagg tggaaatttt 4140
ttggtatgaa ggattagagt aggggagatt ttttagattg tggtgtttaa tgtttttttt 4200
tgggttttat taatgttgta gaggggaata gggagttaga aggaagagga aatgttagaa 4260
gatatttttg aagttggaag atgttagtat attttttttt agttttgtta attttttgga 4320
gtagaaataa attaagtgtt atattggaaa aatttcggtt taaattgttt attagtaata 4380
ataataataa attatatttt atataattaa gggcggagtg ggagtgattt atataacgtg 4440
atttttttgt tttgatattt attgtatttt cgtttgtttt gtaggatatt gttttaattg 4500
tttttagcgt tttttatgtt ttttatcgga gaaagtaaat atattttaga attagagggt 4560
agtagaagtt tttatttttt tttaatttta tatttggaaa aattttaagg tagagggagt 4620
aaagtttagt ttttagttaa agagtgtttt tttttttttt gaaatttttt ttcgtatttt 4680
tttgtattcg gtgtcgaaaa taattgttag aggttttttt ttttggttcg aagaaaaaag 4740
attggttaga ttaaaaagga gggattttag ggggaaagta ggtttttagt tttgggttgc 4800
ggtttagagg gggtgatgtt atcgtttttg tatcgacggg tttgggggtg gggcggtcga 4860
gggggagttc gcgtcgcggt tgtagttgtt aagggagcgt ttcgagttta cgttagggga 4920
ggtgtcggga taaatagggt ttcgtaatgg tcgtggttgg ttgcgtttcg agttgcggag 4980
ttcgggattg gagttgttcg ggcgggttcg cgtttcgaag gttgagagtt ggcgttgttc 5040
gtgttttgtg tgttagacgg cggagtttcg cggtcggatt tcgcggtttc gttttgttgt 5100
cgattggagt ttgggggaag aaattttttt gcgttttaga ggattttttt ttcggcgaag 5160
ggatagcgaa agatgagggt ggtaggaaga gaagggcgtt ttttgtttgt cggggtcgta 5220
gcgcgagagg gtagtgttat gttttttttt attttagtgg cgttgtgttt gtggttgtat 5280
ttggcgttgg gcgtgcgcgg cgcgttttgc gaggcggtgc gtatttttat gtgtcggtat 5340
atgttttgga atattacgcg gatgtttaat tatttgtatt atagtacgta ggagaacgtt 5400
attttggtta tcgagtagta cgaggagttg gtggacgtga attgtagcgt cgtgttgcgt 5460
tttttttttt gtgttatgta cgcgtttatt tgtatttggg agtttttgta cgattttatt 5520
aagtcgtgta agtcggtgtg ttaacgcgcg cgcgacgatt gcgagttttt tatgaagatg 5580
tataattata gttggttcga aagtttggtt tgcgacgagt tgtttgttta tgatcgtggc 5640
gtgtgtattt cgtttgaagt tatcgttacg gatttttcgg agggtgaggt cgtaggaagg 5700
atggagggag gcgaggaggc gttcggggtt gtggttgtta atcgagaatt gggcggttat 5760
atttaaggtt gttaggggcg tttgaggttt gtaggaggtg ggaaagaatg ggggaaatat 5820
tttttttaga aaaagtttgg ttttttttat aggaatttta ggagttagcg gagagtattg 5880
tattttataa agaaaaaaaa aaagattttg tttataaagg gagttttcga tgatgtttgt 5940
atttatagta ataatagaaa tgataatagt agtaaatagg tgttgttagg tgtttgagta 6000
agtgttaaat atattatacg ttattgattt atttaatgtt taaaataatt ttattaagta 6060
ggtgttaata ttcgttttat ttagcgttat tttttaaaaa gttttttttt ttttgaaaaa 6120
gtagtaacgg ttattgtttt ttattaggag tgagatttgt aattgtagtt tttttggaaa 6180
attgtgaggt ttgttttata gttattggtt cggagttaat tatagtggta ttttagttaa 6240
ttttattttt gttttatttt tttttttttt tttttggtta tttgattgtt tttttaaaaa 6300
gaaggagagg tttaaattta ttttttgaaa tgaaatttag gaatattttt gtattttttt 6360
atttaggttt tatttggttt aggttatttt atatttaaat agattatgat attgttgtgt 6420
tggaattttt gaagttatat tttagagtat tgttttaaat atgaaggagg agtggttaag 6480
tgaatagggt aaatggaaat ttttttttaa tttagagttt taggaaagaa aatatattta 6540
ttgtttaaat tatataaata atagagggtt atgtttggtt ttggttatat atttgtgaat 6600
tgtttttgta ttgtaagata tgaggtaggg atttggaaat attttggtta ttttgattaa 6660
tataaattgt taatttggaa aattaatatt taaatatgat tttgggagag agtgatgtga 6720
tttatttagt ttggttgtat atattagtga ggttaaatgg atatatattt atatcgattt 6780
gtgtttggat aggtaaatat ttttttagag gatttttttt ttaagttttg gatattatta 6840
tgaaaggaga agttaataat tatatgtcgt ttttatttat ataaatattt tttgttttgt 6900
tttttttttt tgagatggag ttttattttg ttgtttaggt tggagtgtaa tggtataatt 6960
ttagtttacg gtaattttcg tttttcgggt ttaagcgatt tttttgtttt agtttttaga 7020
gtagttggga ttataggtat ttattattat gtttggttaa tttttgtatt tttagtagag 7080
atggggtttt attatattgg ttagattggt tttaaatttt tgattttagg tgatttgttt 7140
tttttggttt tttaaattgt tgggattata ggtgtgagtt atttcgtttg gtttattttt 7200
attattaaat attattattt tgagatattg ttttagttta tatttttaga gggtttttta 7260
tattagttgg gtgaatagat taaattatta attacgttta tgtggttttg tttgtgtttt 7320
aga 7323

<210> 226
<211> 7323
<212> DNA

<213> Artificial Sequence

<220>
<223> chemically treated genomic DNA (Homo sapiens)

<400> 226

tttgaaatat aaatagggtt atatgggcgt ggttggtgat ttggtttgtt tatttagttg 60
gtataaagag ttttttgaag atataaatta aaatagtatt ttaaaataat agtatttaat 120
ggtaaaaatg ggttaggcga agtggtttat atttgtaatt ttagtagttt gggaagttaa 180
ggagggtaga ttatttgagg ttaggagttt gagattagtt tggttaatat ggtgaaattt 240
tatttttatt aaaaatataa aaattagtta ggtatggtgg tggatgtttg taattttagt 300
tattttagag gttgaggtag gagaatcgtt tgaattcggg aggcgaaggt tgtcgtgagt 360
tgagattgtg ttattgtatt ttagtttggg taatagagtg aggttttatt ttaaaaaaag 420
aaaataagat aaaaaatatt tatataagta aaaacggtat gtgattgttg attttttttt 480
ttataatgat atttagggtt taggaaagaa attttttggg ggaatatttg tttatttaag 540
tataaatcga tgtgaatgta tatttattta attttattgg tatatataat taaattggat 600
aaattatatt attttttttt aaagttatat ttaaatgttg attttttaag ttaatagttt 660
atgttaatta aaataattaa aatattttta aatttttatt ttatattttg tagtgtagaa 720
atagtttata ggtatataat taaaattaag tataattttt tattatttat ataatttaaa 780
taataaatat gttttttttt ttaaaatttt aaattagaga ggagttttta tttattttgt 840
ttatttaatt attttttttt tatgtttaga gtaatgtttt ggggtataat tttagagatt 900
ttagtatagt aatattatag tttgtttaaa tgtggggtaa tttgggttag ataaagtttg 960
gataaaagaa tgtaaaaata tttttaggtt ttattttaag aagtgaattt aaattttttt 1020
ttttttttag aaaagtagtt aaatgattaa agaaaaaaga aggaagtgga agtagagatg 1080
gggttaatta aaatgttatt gtagttggtt tcgggttaat gattatgaaa tagattttat 1140
aattttttag ggaaattgta attataaatt ttatttttgg tgggaagtaa tgatcgttat 1200
tgttttttta gaaaaaaaaa aattttttaa gaagtagcgt taaatggaac gagtattggt 1260
atttgtttgg tgagattatt ttgggtatta aatgagttaa taacgtgtaa tgtgtttaat 1320
atttgtttag atatttagta atatttgttt attgttatta ttatttttgt tattattata 1380
aatgtaaata ttatcgaagg ttttttttgt aagtaaaatt tttttttttt tttttatggg 1440
gtgtagtgtt tttcgttagt ttttaaaatt tttatgggag aaattagatt ttttttgaag 1500
aaaatgtttt ttttattttt ttttattttt tataagtttt agacgttttt ggtagtttta 1560
ggtgtggtcg tttagttttc gattggtagt tatagtttcg ggcgtttttt cgtttttttt 1620
tatttttttt acggttttat ttttcgggag gttcgtgacg atggttttag gcgagatgta 1680
tacgttacgg ttatagatag gtagttcgtc gtaggttagg ttttcgggtt agttgtggtt 1740
gtatattttt atgaggggtt cgtagtcgtc gcgcgcgcgt tggtatatcg atttgtacgg 1800
tttgataggg tcgtgtagga attttagggt gtaaatgggc gcgtatatgg tatagaggaa 1860
gaagcgtagt acggcgttgt agtttacgtt tattagtttt tcgtattgtt cgatggttag 1920
gatggcgttt ttttgcgtgt tgtggtgtag gtggttgggt attcgcgtga tgttttaggg 1980
tatgtgtcgg tatataggga tgcgtatcgt ttcgtagggc gcgtcgcgta cgtttagcgt 2040
taggtgtagt tataggtata gcgttattag gatggagagg aatatggtat tgttttttcg 2100
cgttgcgatt tcggtagata gaaagcgttt tttttttttt gttattttta tttttcgttg 2160
ttttttcgtc gaggaagaaa ttttttgggg cgtaggagag tttttttttt taaattttag 2220
tcggtagtaa agcggggtcg cggggttcgg tcgcggagtt tcgtcgtttg gtatataggg 2280
tacgagtagc gttagttttt agttttcggg gcgcgaattc gttcgggtag ttttagtttc 2340
ggatttcgta gttcggagcg tagttagtta cggttattgc gggattttat ttatttcgat 2400
attttttttg acgtgggttc ggaacgtttt tttggtagtt gtagtcgcgg cgcgggtttt 2460
ttttcggtcg ttttattttt aggttcgtcg gtgtagaagc ggtgatatta ttttttttgg 2520
gtcgtagttt agggttggaa gtttgttttt tttttaaagt tttttttttt tagtttggtt 2580
agtttttttt tttcggatta agaaaaagaa tttttggtag ttgttttcga tatcggatat 2640
aagagggtgc gggagagaat tttaagagag agaaagatat tttttggttg aaaattgaat 2700
tttgtttttt ttgttttaga gtttttttaa gtatagagtt gggggaaaat aggagttttt 2760
gttgtttttt aattttgaaa tatatttgtt tttttcggta gggggtatga ggagcgttga 2820
ggatagttgg gataatgttt tgtaaggtag gcgagagtgt agtgaatgtt agaataggag 2880
ggttacgttg tataaattat ttttatttcg tttttagttg tgtggagtgt ggtttgttgt 2940
tgttgttgtt agtagatagt ttgggtcgaa atttttttag tataatattt ggtttgtttt 3000
tgttttagga agttaataga attgggggaa ggtgtgttag tattttttag ttttagaaat 3060
gttttttaat attttttttt ttttttaatt ttttgttttt ttttgtagta ttagtggaat 3120
ttaggagaaa atattgggta ttataatttg aaaggttttt tttgttttga ttttttatgt 3180
taaaggattt ttattttagt agtttaaaat tttgattttt gatgtgttta gagattgttg 3240
tttgagttga gtaagaagtt gtgtattatg ggatgtaagt atgtatgttt tgtttttttt 3300
tttattttat ttgtattagg aaaaggtagt ttaattgttg gtttagttat ttatagaaaa 3360
gggaggattt atttatatga gattgatgta tattaatttt taggtggtaa gatttttggt 3420
atatgttatt tattagggta ttagtaatat ggaaatatgt tttttgaatg ttaattttta 3480
aaaatatagt ttaatttgaa tttaaggtat atggtttttt tttttaaagt ttaaatttat 3540

ataattagtt tagttttgat tgaggattaa aattaaagat taaggattaa aatagttgag 3600
gaaataataa gtataagtga tatttattgg gaatttataa tggttaaggt atgaagttaa 3660
gaattttaaa tgtattattt tattttttag ggaatatgaa ttaaatatag taagtgaaaa 3720
ggataaatga gataaatata ttttaggatt tatttaaaga gtttggtttt tatttagggg 3780
aagtttaggg tgtttttata ttgttaagtt attttagaaa ggtaatttag ttttatagaa 3840
attagttttt atttttaaag atgttaagtt aggttagtaa aatattatat gaggaggaaa 3900
gtttagatga tattttaagt tatttttagt agtaaatttt aatgagttta aattttttat 3960
tgggtgaaaa agatttttag ttggagtttt tatttgggga gtaagagaaa gagttgaatt 4020
attttatatt ttttttatga atttgagtag ggtaaattcg gagagggttt aaatttttgt 4080
agttagtttg aatttgttgt atttgtttaa ttatgtaagt attaagttat aaagttttat 4140
gggttttttt gatgtttttt gaggtatgtt ttttaagaaa aggtaagagg aaatgaagag 4200
tttttttttt taaagttttg tgtttgtttt attcggttta ttggtagtgt ttttggtggt 4260
tcgatagata gggggtagta gagaaggatg tattaggaaa atttgttaat tgtttttttt 4320
tttcgggatt tttagggagg ttgtgatttt agaatatttt attttagtat ttagtttatt 4380
ttttttatgt tttatattta tttaataagg ttcgggaagt tgttttattt agtggttttt 4440
ttttggtgat tttttttggt ttttttaagt gtttgttttt tgtttatttt ttaaatgttt 4500
ggagtttttt ggggttttat tttaattttt ttttttatat ttaatatttt tttgaatttt 4560
aggttttttt atttttaata tttaggatat ttggggttag ataatttttt ttttatttgg 4620
gagaggggag gtggttgtat tgtatattgt aggaagttta gtagtatttt tggtttttat 4680
ttataaaatg ttagcggtag tttttttttt atttatattg tgattattaa aaatgttttt 4740
agatattgtt aaatgttttt ttgttgggga gggggatgtg aaattttttt aggaattatt 4800
gttttatttt tcggttttat ttattattta tgtatttatg gttttttaat ttaaaagttt 4860
tgttggtttt tagaagtatt tatttagttg ttttttggat atttttattt taatatttta 4920
tagttagttt aagattaatt aattttaaaa tgaattgatt attttttttt atttaggatt 4980
ggatttttag tggtttaaat tagaaatttt tttttttgag ttgaaatgtt tttttatagt 5040
ttttatttgt tttttatttt ttttatgatt attttcgttg ttttagttga gttaggatta 5100
ttttttttta ggatgataat agtagttttt tttttttttt gttttttttt tggttttatt 5160
ttttttgttg tttaagtggg ttttttaaag tatatgtttg atgatgtttt tttttagttt 5220
tatattgatt gatagttttt atagttttta atattaatga taaatttttt aaaatgagtg 5280
ttatggtttt agagtatttt tttattttgt ttttgaattt tagtattatt ttttttttgt 5340
ttttatgttt tttatttagg aatgttattt tttattttta tttggtttaa ttttaattat 5400
ttttgaaatg tagtttaaat ggaaagacgt tatatatttt tttagttttt tttttttttt 5460
ttgattttat attttattat tatattttta gagatagggt ttcgttatgt tatttaggtt 5520
ggatggagtg tagtggtatg attatagttt attgtaattt ttaatttttg ggtttaagcg 5580
atttttttat tttagtttta ggagtagttg ggattatagg tgtgtgagtt tcgtttattt 5640
tatattatag attttaatat tatataatta taaatttaat taattttatt tttgaagaag 5700
cgaaattttt tagatattta aattattcgg ttggtggtat tgttaggcga tgggaggagt 5760
agttttattg taatgtgatt ttgaggttat gtttatgagt tttaggttta atgtaagggg 5820
ttgtttatta gttttttaga ttttttattg ggaatgggag ttgaggagcg gttatgatga 5880
atagttagta ttattttttt aggaaaagat tcgttttttt atttttaatt tggaggaaga 5940
agtttgtaat tttgtcgagg ggtaatgtag gcggggtaaa cgtttgtata gtaaacgtag 6000
tagttttgtt ttttttattt tgattttgaa tttgtttttt tttgtgtgat tatttgttga 6060
atattgaagt tagtggagaa atatattttt tagtatagtt tttttttatt ttttttttta 6120
aaatacgatg ttttttagga tgttagtggt attattgtta ttgtattttt tgttggtagt 6180
agtgagtagt gaaaatcgaa gcggtagaag gtagtggtag taggtagtgg tagtaggtag 6240
tggtttaggt agaaatagtt ttcgcgcgat ttattggagt ttttttcggg ttttggtttc 6300
ggttatcggg attcgcgcgt tcggatttta aaagcggtag aggttatcga agggatagga 6360
agtattttgg tttagattat attttcggta tagtgcggaa agagtcggcg ggagttattt 6420
tgatttcgga cgttttagcg tttttttggg tttgggtttg ttttcgggtc ggggaaggtt 6480
gatcgcgatg ttaggacgcg ttttttttcg tatcgtttcg ggcgttttgg gtgtttggtt 6540
gttgggcggt ttttgggttt ggattttgtg cggtttgtgt agtttggggg cggtgggagt 6600
ttcgcgttcg tgttaggcgt cgtagtagtg ggaggggcgt taggttatgt attagtaaag 6660
tagcgggcgt aatagtcgcg ttttgttttt ttacgacggg tttaattagc gcgtgcgggt 6720
gttggacgag aggaaggcgt tgattttttg taagaggtat aggttatcgg ttcgcggggc 6780
gggagtaggg agtcgcgcgg gtatggggag ggcgaggtcg tagaggtttg cgttttgtaa 6840
tttttttcgg ttttttgtag tttttttttt tttttagaga gatttttcgc gaggagggtt 6900
ttttggggat agtatggttc gggattggga ggggcgttgc gtttatcgag ggcggtttgt 6960
tggcggggga tttagtaata tttagcgagg cggtggtagg taaagaaggg atttatttag 7020
ggcgggatta agttgtagag tgttttatgt ttaggttcgt ttttgttttt acgttgttga 7080
gttaggtttt gggtttatgg agttttttgt agtttagggc gtgttttttg cgttatttta 7140
ttttttttta gagatatttg ttagagaggt acgtagaata tgtatttttt aagaaatatt 7200
ttttgtattt taaaagaata tggtaagatt ataaacggta tgggaaagtg ataaaaagaa 7260
aattttattt ataaatttag taaattgtta tagtattaaa attttggttt atttttttta 7320
agt 7323

<210> 227

<211> 10717
<212> DNA
<213> Artificial Sequence

<220>
<223> chemically treated genomic DNA (Homo sapiens)

<400> 227

agttttatgt taggtttagt cgggtagacg ggatttaagg tatataggta ttaagtagga 60
ttggggagcg tttaggggtt ggggggttaa gtagacggag tattcggagg ttggggagtg 120
ttcgcgggtt gggggagtag tagggggttt ggagttggta gttgtattta tatttttttt 180
ttttacgttc gtttttgggt tttgggattt ttattaggga agttagtaaa ggagaaattt 240
tgatttgagt gttcgggagt gaggttttag cgcggtttgt cggttttttt ttagttttta 300
ggtgaggttt atgtgcgtaa gggtttaatg tattaattgt ttggggtatt tgttttttaa 360
ttaggtttta agatggaagt aatgatggtt ttgtggaagg atttttaggt agttggggtg 420
taaggagtag tatgtagacg tagggttttt ttttttttgg gtacgaggtt atttgtttat 480
attgtgtttt tttttttaat ttttaaatgt agttttaggt ttttttgata acggtgttag 540
agtattttga aattgtatag aggtatatat ttgaggtatt atttagattt aagaagaggt 600
gtttattatt taaaatgaag acgaagtgat ttaataaagg taggtaatgg tttaaagaga 660
gaaaattagt ttaaaggtat atagtaatat agtttaggtt gtacgtttat attcgagttg 720
atcgtttgtt ttaggatgtt tatttcgata gagttggttg tgtgattttt ttagcgtttt 780
tagaaattgt agggttttta ttttttagag tttgtagttt ttatgtatgt gagtcgggtt 840
tttacggggt tttacggggt tttacggggt tttacggggt ttggtgtgta ggcggatagg 900
tatgagggta ggaattttag ttattaaagt gttttttgta ttttgtttta tagagtggtt 960
taggtcggga agaagttagg ttgtatttat agttatgggg tttgtggtag tttgggggtg 1020
gtttcgtgtt cgttgtatag tattattgag aaacgttgta tagtgggtat ttagcgtagt 1080
tggttttata tgttagttat ggtttttcga ggttttgtgt gttttggtag ttgtttttta 1140
gcgggtttgt ttttagcgtt ggtgttatga ggatgtggtt gtaggagttt agagagtttt 1200
gtagttattt taaagttttt gggttttggt acggagagga tagtattgtt ttaatttatt 1260
tgtagttagg ttataattag ggtgttgtgt agagtgtggt tggatttttt gggttattta 1320
gagtttgagt tttttaggaa gttggagatt tatattttta gggttcgggg atggggagga 1380
ttatggagtt agggaaaggg ggttaaggtg tagaattttt tttaaattta aaaggttttg 1440
gatgtaatta taagagaatt ggagtgtaga agttttatcg gttaaaatag ggaaaattta 1500
gaaagaagtg gagattggat ggttggatgg aaagagattg cgtggaatta ttataaggaa 1560
gaaaaattaa aggggagaag gatatgaaag attagaggtt aattttagag attagaaata 1620
gaggtagtat ttggagatgt taggtaggcg ggttttataa ttgtagagtt tagcgggtat 1680
aaatttggga aaggaaagag gggatgaggg gggcgtagaa attgttgacg ggattagggg 1740
aggatgaacg aaggaggttg tttggttttc gggtttttcg atttgggatt gcggggattt 1800
tttaaatcgg ttaagttcga gtcgaaggta gagtgaggag gttatttagc ggtaggggtc 1860
ggaaggaggg gtaggggtag tgtttaggag ggagtttggg ggttggggta gaagaggagg 1920
tagtttttgt ttttagtagg gatgttgggt aaggggtgga ggtgggggag ggtgggggag 1980
ggaaagttgg aggaaggagc gagggaggga tgcgcgtgat tttaaggaag agaggtagag 2040
tttaggtaga tgaataacgg agttttagta tgttcggatg tttggttttg ttagggaggt 2100
tggtgtttgg agtttttgtt ggtgcgggga gggggttaaa tggtttgggg ggggggggga 2160
aggtaaagag atagagttat ttggtttttg gaggggaagg tagtgttggg tcgtggggag 2220
taggtgggat ggaaggatag ggagtgaagg gcgggagggt agagtagtag tttttttggg 2280
gggtttagtt agtagtttgg tttataattg ggattgtttt ttgcgtcgag ttttttttaa 2340
tttatgtagg gggtaggttt tttgaattta ggcgtggggg taggtttttt ggtttattat 2400
tattttattt tggaggatcg gttagtttat aaggggggcg gtttttgagg tgttttttag 2460
ggtgtttagg gttgtttgga gatggagggg agggaggggg ttttacgtta ttttttaggg 2520
tagggaggtt ttaggaaatg ttttgagtta tttaagttta atgttaagtt acgttttttt 2580
tttgcgtttt gttaagaggg agttgatcgt atttgggggt attttacgtt tagttgtgtt 2640
tataaggaat tttgtgtagt ttaggttttt gtttttaaaa gtatatttta tttattttgt 2700
tttttgtaga ggtgagttgt gtgtatatgt tatttttcgg tagggttgta tatttaattt 2760
tttaggtttt agggtggtaa ttcgtgtggg gggttttttg tttgtatttg gaagggagta 2820
agtagcgggg aaagataggg ggaggaggag ggattggggt aagaggagat gttaagggtg 2880
gaaggtaggg tagttaaaag tttgtaaatg ttttattggg ttaggtcgtt ggtttagttt 2940
aggtattatt tagtgatgtg atatttatat gttgtgtagg tagatatatt aaataggtcg 3000
gttttttttg gagagaatac gggttttgtt ttgtgtttgt ttttattggg aacgggatag 3060
gtagcgtaga tatatcgttt ggagaatttt tgggtttagt tgagttttag tttcgtttag 3120
tggaggttgt cggggtttgg ttaagtggac gttgtttttt tacggtgttt ttttttaggg 3180
attacgattc gttataaatt atggaaagga ataagaaaga gaggcgggaa ggggagttcg 3240
ggttaatttt tttaaaggta tttaggttat cgagttttgt taacgtttag ttttttatgt 3300
ttaagataga tgtgttttaa gatgtttttt tttttttttt ttttattata tatataaaaa 3360
gttgatttgt aaagggaatt tttggaaatt tttatcgttt gagggttgag ttttataggg 3420

gttttttttt tttttatttt aagggttcgg ggttaaggga gagtgtttaa ttttataggg 3480
ttatatttgt agtatttgat gattttttag ggttaggatt ttttttttac gtaaataatt 3540
tttatttttt aattttatgt tagaggaatt attgtatatg agtgggatgg cgattgagaa 3600
ggggaagggt atgatttgtt tttttttttt taaaaaaaaa aagaaaagta gggttgagag 3660
ggagttttaa attttatttt tttggtttat ttaggatttt tgagtttttt tttttttttt 3720
gtaaaaagtg aggtgattat agatttaaag attgttattt tttgggtatt gtgttttttt 3780
tttaggaagt ttttcgttaa ttagaatatt gtttttttag ggtttttgat tttttgggtt 3840
gtagttttta ggattggtgg tttttgtgtt tgaagattgt tggtttgttg agtttttata 3900
taaggttagt tttgtaagga gtttgaattt tatatttagt ttatttttgg ttttgtattt 3960
tgttttgatc gatcgtgggg gttgtgtgag tatagtttcg tttattcgtt tgtttttttt 4020
ttaatatttt gtaatggttt ttatatattt agcgtatata ttggttttta tttttttttt 4080
tgtgttgggg tgtagttttt agatttgttt tgtttattta ttttgacgtt cgtagtttat 4140
tttgtaattg tttttgagta cgtttttatt ttatttgtag agttttataa aaaaaaaaat 4200
gtattaattg aagtataata aaagagtagt atttttatat ttttttaagg tatttaaagt 4260
attttaaata ttagtgtttt aagttatggt tatttataat tattttcggt ttatagaggt 4320
tgagttttta ggaggggttt agtttttttt ttttaaaatg tagggagagt tagtttgtat 4380
tataaagttt ttggggaaga aaaattaaat aatagttggg tagttgtgtt ttttaaatgt 4440
gtgagcgatt ttgtgtttga tgattgaagt ttgcgggggt ttaaattgtt ttttaaaaat 4500
ttaagttggt tttaattgaa gtatttatta ttttagggaa agttaggggg aaacgtaagg 4560
atgtatataa agtcgggagt aattgttgtt attataagat atgcgttttt ttttttcgtt 4620
tatttttatt ttaggaaaga tttttttttt tttaattcga attttttagg tgaaaataaa 4680
gtaatagtag taatagaaaa aaaaaacgta gtttttttta gggttaatat tagaagataa 4740
gtttgtgcgg ttttggttaa ttagatgcgt ttttggtagt tataatatag tgttaaaggt 4800
agtttttttt ataagttttt taggtttgtt attatttaaa attagatttt ttttgttttt 4860
tgattgttgt ttcgcgattt aatttcgatg tgtttcgtta ttagcggtcg gtagtttgtt 4920
attttagttt ttgtttgggt ggggagcggt ggagagtttt tcgtagtcgc ggcgggtaag 4980
gttatatagg aagagaaaga gcgaggtagt tagcgaggga gagagcgagc gggcgagtcg 5040
gagcgaggaa gggaaagcgt aagagagagc gtatacgtat atattcgtcg cgcgtattcg 5100
cgtacggatt cgtacgggga tagttcggaa gttattagtt ttatgggcga gatgttgttg 5160
ttggcgagat gtttgttgtt agttttcgtt ttttcgttgt tggtatgttc gggattggcg 5220
tgcggatcgg gtagggggtt cgggaagagg aggtatttta aaaagttgat ttttttagtt 5280
tataagtagt ttatttttaa tgtggtcgag aagattttag gcgttagcgg aaggtatgaa 5340
gggaagattt ttagaaattt cgagcgattt aaggaattta tttttaatta taatttcgat 5400
attatattta aggatgaaga aaatatcgga gcggataggt tgatgattta ggtaggaatt 5460
tagcgtcggg gcgtggaatg tgtggttttt tagggggtta cgagaagtcg aatattttta 5520
gatttaattt tgtttgtttt tcgggtagat aggaaggtga ttttattcgt ttttttttta 5580
tttatttgtt cgttttttat tttttttttt ttttggagga gaatggaggt taagggttta 5640
gttggagaag tttagggtgt ggtgggggtg aggacggtaa tagacgtggt ttattatggt 5700
tttgatttga tgagttttgt tataatggtt ttttttattt tatttttgtt ttggtttgta 5760
atttggggag atttttattt tgggggcgtc ggtttttttt aagttaggag tggaaatgga 5820
aggagaggtt gggaattttt tttttataaa tatgaagtgg ttttttggta ttgtacgaac 5880
gaacgaacgt agttttgggt attggagttt agagttttta cgtttttcgt tgtttttgtg 5940
gttttttttt ttattattat ttttattttg tattttttta ttaaagaatt tttaattgga 6000
aaagttagga ggcggttttg ataaaaggta ggggttttag gggagatttc gttcgttttt 6060
gggtggttgg ttgtatcgta gagttggttt tgcgattgcg tgttcgtaat tgtgtttatt 6120
agagtgtgaa tgtattgata tttttttaag gatgtttttt cgttttttta agttcgaggt 6180
attttagggg agggatttag aatttattgg tattgtatta ttttagtttt taatttgttt 6240
gtataagaat taagagcgaa taaatattag tgtgggggga ggggaagtta agtaaaatat 6300
gaattttttt tttttttttt attttttttg agatttttga gttgttaatt ttttagttaa 6360
ttttagattt tttgaaattt tatgtacgta taattgaagt tagaaatggg tttttttgta 6420
aatataggtt aatatttttt ttattgtttt attaaaatat ttaagtttta tttttagggt 6480
taggtgcgta tagcggttga tggagtggcg ttggtggggc gtaagtgtag ggggagggta 6540
ttgacggtag agagagagga gttattttcg tgtcgttttg tttttcgatt cgatttttag 6600
gtttgtttga ggtcgagaaa ggcgaggggt aggtaaggta gtttgtttta gttgtcggaa 6660
gggagaggaa tgggaaatgg ttttgatttt tttgtttttt tttatttgtt ttcgattatt 6720
ttaaatttgg atcgcgagtg tggacgcgcg cgttagtgtt agatagtagc gcgatttata 6780
attaattttg tacgggttat ggggtgttcg ttgcgtgtag ttggttggag ggagtttttc 6840
ggttagttcg aggcgtttat tttttcgtta tttttatttt tcgcggagga ggggttttgt 6900
tagggttttt cggaattcga gaggagggag gtattgcgga gagagcggcg ggggcgtgga 6960
tattcgaggt tttagagtta gagtgggtta gtttttgttt tgttttgcgg gaggttaata 7020
tcgtagaagg ggttttgggt tcgtatattt ttttagggtt tgggttttgt agttttgttg 7080
taaagttgta agcgtataga gtcgcgtagc gaggtagacg tttgtagttt tatttatttt 7140
cgggttatcg atttttcgcg gttagggttt tagtgcgcga ggggttgggt tggagtcgtc 7200
gggttttgtt gttttacgcg cgggagcgta ggtatcgtag gagttaatag tagcgtcggg 7260
ttcgttgtag tgttttcggc ggcgggggcg cggagatggg ggcgttcgcg taggggtcgg 7320
ggcgtatcgc gggtttcgtc ggtttgtttt gggacgcgtt tttattttta gttcgtcgtt 7380

tgtttggttt ttaggttttc gcgttttagt cggagttttt agttcggggg ttttttttcg 7440
attttcgttc gttttgtcgg gggacgtagg gtttagcggt ttcgcgttcg gttattttcg 7500
tcgtcgcgcg tataggtagt atttgaattt ttgatttttt gttaattttt tttagacgaa 7560
cgaaacgaaa ataaatattt ttttttttgg gtagtggtta ttttcgtttt taatataaaa 7620
ggagggggaa ggacggttta agtgggggtt gggtgaggag agttaggtcg ggattattag 7680
gtagatttta taaaggtttt ttaaaatcga ggggggtagg ggttggtagt ttgtgaggta 7740
ttttcggttg attttttttt tatttttttt ttttcgattt taggagttaa gggtggggga 7800
ggaagggatg gggaaggcgg aggttcgggt gttgagggta ggggcggggt gtaggaggcg 7860
gtaggggagt tttaggtcgg cgggaggttt ggggagtttg ttcggtcgtt tttattttaa 7920
ataattattt aggttttgtt ttaggtgcgt gatttttttt ttttgttttt tttttgtttt 7980
ttgggttttt aatgtgattg tcgtttaagt tttttaatta tggcgagatc gtttttagtg 8040
gaattttcgg agtagtttcg gaacgtagga gttgtcggtt aatattaatt cgggagagga 8100
aagcgtagat agatacgttt ttttcgcgcg ggtttaggtg ttaggcgagg gtgttggcgg 8160
ttagggggtt tttaaggggt aggaggttag agggtcggat ttgaagtttg gagtggggtt 8220
tcgagtcgtt atattaaata gatttaatgt gcgttttggg gtcgttagga aagacgttta 8280
ggtatggggt tggggagggg ttgttttatt aagtttgggg gaaaggatag tggagagagg 8340
tgcgtttagg ggttggggtt gttttgaagt tgggacgttt tcgttttcgc gttgggggaa 8400
gtttatcggt tgttggcggt gatattcgtc ggcgcggttc gtagattagg gaggtaggcg 8460
ggagtttagg cgtggggaat aatttggttt tcgtcgatat aaagttcggt ttcggcggtt 8520
ttgttgggtt ttacggtggt tgtatagagt cgggtttgat tcgcggtata cgatttaatg 8580
aattaataat cggtttgggt ttttcggttt tgtttgcgat aatttcgttt agcgcgggcg 8640
gaggagaggt cgttagtcga ggtcgcgcgg agttcgggtc ggaggagggc gtaaggggcg 8700
ggggcgttaa ttttagtaat tttcggtttt cgttttttat tcgcgtagtt atttttcgtc 8760
gcggttcggt tggattcggg tttttttgtt cggggttttt tatgtttgtt taagtggcgt 8820
agtttataga gttgggggtt aggttatttt tattttgtcg tttttttttt ggttgttttt 8880
ttgggaagtt gtttaaagtt ttttcggtat agttttaggg gagggagttg cggtggggtg 8940
gggggtttgt atgggggttt ttgtgcgtgt tggtggtgtg cgtttgcgcg taacgggttt 9000
tatattatag ttttatattg attttggttt attgattgat ttttatgtaa aacgcgttta 9060
atttttaaga tgattttatt taaattttgt tttttcgatt ttttttttta attgttggta 9120
ggtttataaa tatgtaggta ttgatttgtt attagggcgg tttttagttt tattttattt 9180
ttagttgttg tatgttgaat tttataatta tattattggg ttttattgtt gttagatata 9240
taggattttt tttgttgcgt aatttgttac gtttttttaa agcgtcgtag tagtggggtt 9300
agcgttttcg ttttattttt tcgaaagagt ttttttaatt tacgttatag ttagggtttt 9360
ggatagaagt tgttttttta tggcgataat tagattttaa gtagagtatt tttttagatt 9420
ggaggaggtt agaggttagt ttcgtttttt gtagaagtta ttttgaaatt gtttttcggt 9480
ttttatttgg cgtagttttt tgggggatgt tattatcgtt atttgtgtta gttttttttt 9540
tttaaatttt atgttttatt agtagtagta gggtaaatat ttaggaagta agttagtgtt 9600
tttataaata tatatagtgg atttaattgt tttttgtcgt atttttattt gagggtgatt 9660
ttagaatttt aggggaattt ttataatttg aattttaggt aatttcgttt gtatttgttt 9720
agttagtgtt ttttgttttt ttttaggtaa ttttttggga aattttttag gcggaggatt 9780
tcgagatttt aggttttttt gttttttttt tttttttttt taaatttttt tttttttttt 9840
tattttttta gtttgttttt taaatttgtt ggacgttatt ttatgttggg gttaagaata 9900
taagagcgga ggaagggaat aggttaaaga aaataagaaa tataattaga ttatagaaaa 9960
gttaggtaga aaagggttcg acgggtaaaa agaatgtggt tgtttagata aagaatgttt 10020
gtttcggttt cggtttgtgt tgtaagtggt aatttattta gtcgtttgtt atttaggttt 10080
tcgtttatcg cgtagtttcg ttagcggttt ttcgtttttt ttttgtttcg gatagggtta 10140
gggtttgagg taaataaatg taaggttttt aattttttaa gtagtgcgta gtgtattttt 10200
ttttattttt gggaatttgc gtttaggttt ttgttaggtt tgttgtgagg gattttacgc 10260
ggggagaagg tggaggttgc gtaggtggag aaaggggttt tagaaggggg gttagaagtg 10320
gagggtaacg tgggggcggg gcgggtattt tagagggtgt ttttggaggg ttttgtagtt 10380
gatgttttaa atatgtaggt tatttgtttt agagaaattt tatttgtttt ttaggtttcg 10440
ttaggagtat cggttgtttt aggatttgga gaaaggtttt tagttttatt ttgagaggac 10500
gtgttttttt acgttttttc gtaaatgttg tttttttttt ttagtttagg gttcggtttt 10560
tcggtgtgtt tgggttattt taattttcgt ttttttattt tttcgtatgg ttttcgcgtt 10620
ttgagattgg gtagggtagg ttgatggagg ggtcgggagg ggtggcggtt gtttaggtta 10680
acgtgttcgt cggtgggggt ttttttgttt tcgtaga 10717

<210> 228
<211> 10717
<212> DNA
<213> Artificial Sequence

<220>
<223> chemically treated genomic DNA (Homo sapiens)

<400> 228

tttgcgaaga taaggggatt tttatcgacg gatacgttag tttgggtaat cgttattttt 60
ttcggttttt ttattagttt gttttgttta gttttaaggc gcgagggtta tgcggagagg 120
tggggagacg ggggttggaa tggtttagat atatcgaaga gtcgggtttt gggttgggga 180
agagggatag tatttgcgga ggagcgtgga ggagtacgtt tttttagggt agagttgggg 240
gttttttttt aggttttgaa atagtcgatg tttttagcga ggtttaagaa gtaaataaag 300
tttttttgaa ataagtgatt tgtatgttta agatattaat tataggattt tttaggggta 360
tttttttgga tattcgtttc gtttttacgt tgttttttat ttttagtttt ttttttgggg 420
tttttttttt tatttgcgta gtttttattt ttttttcgcg tagaattttt tatagtaggt 480
ttgatagaga tttgggcgta agtttttaga aataaagaaa aatgtattgc gtattgtttg 540
gagaattgaa ggttttgtat ttatttattt taggttttaa ttttattcga ggtagagggg 600
aggcgagaag tcgttggcgg ggttgcgcgg tgggcgggag tttgggtggt aggcggttag 660
gtgagttgtt atttgtagta taggtcgggg tcgggataga tattttttat ttggatagtt 720
atattttttt tgttcgtcga attttttttt gtttggtttt tttgtggttt gattgtgttt 780
tttgtttttt ttaatttgtt tttttttttc gtttttgtgt ttttggtttt agtatagaat 840
ggcgtttagt aagtttgaag agtaaattga agaggtggag ggagggggag gggtttgaga 900
ggagggggag gggagatagg aaggtttgag gtttcggagt ttttcgtttg gggagttttt 960
taggaagttg tttgggagga ggtaggaaat attgattagg tagatgtaga cggggttatt 1020
tgggatttag attgtggggg tttttttgga attttggggt tatttttaga taaggatgcg 1080
atagaaagta gttgaattta ttgtgtgtgt ttgtgggggt attgatttgt tttttggatg 1140
tttattttgt tgttgttggt gggatatggg atttaaagag ggggaattgg tatagatgac 1200
gatggtggta ttttttaaga agttgcgtta agtggaggtc gaggggtaat tttaaggtga 1260
tttttgtaga gggcggggtt gatttttaat tttttttagt ttggaaggat gttttgtttg 1320
gagtttggtt gtcgttatgg agggatagtt tttatttagg gttttaattg tagcgtgggt 1380
tggggggatt ttttcgagag ggtggggcga ggacgttggt tttattgttg cggcgtttta 1440
aggggacgtg ataaattgcg taataggaaa agttttgtgt atttggtagt aataaaattt 1500
agtaatatgg ttgtagagtt taatatgtaa taattggggg tggggtaggg ttgggggtcg 1560
ttttggtaat aggttagtgt ttgtatgttt gtgggtttgt tagtagttaa aaaaaaaagt 1620
cggaagggta gagtttgagt gaggttattt tgaggattga acgcgtttta tatgaaaatt 1680
aattagtaaa ttagggttag tgtagagtta tgatgtgagg ttcgttgcgc gtaggcgtat 1740
attattaata cgtataggga tttttatgta agttttttat tttatcgtag tttttttttt 1800
tggggttgtg tcgaagaagt tttaaatagt tttttaggag ggtagttagg gagagggcgg 1860
tagggtgagg atgatttggt ttttagtttt gtgagttgcg ttatttgggt aggtatggag 1920
gatttcgggt agggagattc gggtttagtc gggtcgcgac gggaggtggt tgcgcgagtg 1980
aggggcggag gtcgagggtt gttggagttg gcgttttcgt tttttgcgtt ttttttcggt 2040
tcgggtttcg cgcggtttcg gttggcggtt tttttttcgt tcgcgttggg cgggattgtc 2100
gtaggtaaag tcgggaagtt taggtcggtt attaatttat tgggtcgtgt gtcgcgaatt 2160
aagttcgatt ttgtgtagtt atcgtgaagt ttagtagggt cgtcggggtc gggttttgtg 2220
tcggcggagg ttaagttgtt ttttacgttt gagttttcgt ttattttttt gatttgcgag 2280
tcgcgtcggc gagtgttatc gttagtagtc ggtgggtttt ttttagcgcg gggcgggggg 2340
cgttttagtt ttaagatagt tttagttttt aaacgtattt ttttttattg tttttttttt 2400
taagtttggt ggaatagttt ttttttaatt ttatatttga gcgttttttt tggcggtttt 2460
agagcgtata ttaaatttat ttagtgtagc ggttcgggat tttattttag gttttagatt 2520
cggttttttg gttttttgtt ttttaggagt tttttggtcg ttagtatttt cgtttggtat 2580
ttaggttcgc gcggggagag cgtgtttgtt tgcgtttttt tttttcgggt taatattaat 2640
cggtagtttt tgcgtttcgg aattgtttcg aaagttttat tggggacgat ttcgttatgg 2700
ttgagggatt tgggcggtag ttatattaga gatttagaga tagggaggga gatagaagaa 2760
gagggttacg tatttggggt agagtttagg tggttattta aaatgagggc ggtcgagtag 2820
gttttttaaa tttttcgtcg gtttggggtt tttttgtcgt tttttgtatt tcgtttttgt 2880
ttttagtatt cgagttttcg ttttttttat tttttttttt tttattttta atttttggaa 2940
tcgggagaag gaaggtaggg gagggattaa tcggggatat tttatagatt gttagttttt 3000
atttttttcg gttttagggg atttttgtgg ggtttgtttg ataatttcgg tttggttttt 3060
tttatttaat ttttatttgg gtcgtttttt tttttttttt tgtgttggga acgggaatag 3120
ttattgttta aggaaaaaag tatttgtttt cgtttcgttc gtttgaggga agttgataga 3180
aggttagaag tttaaatgtt gtttgtgcgc gcggcggcga gagtggtcgg gcgcggggtc 3240
gttgggtttt gcgtttttcg gtagggcgga cgggggtcgg ggggaggttt tcgggttggg 3300
ggtttcggtt gggacgcgga ggtttagagg ttaggtaggc ggcggattgg ggatgggggc 3360
gcgttttagg gtaggtcggc ggagttcgcg atgcgtttcg gtttttgcgc gggcgttttt 3420
attttcgcgt tttcgtcgtc ggggatatta tagcgagttc ggcgttgttg ttagtttttg 3480
cggtgtttgc gttttcgcgc gtggagtagt agagttcggc ggttttagtt tagtttttcg 3540
cgtattgaaa ttttagtcgc gggggatcga taattcggga gtaagtgggg ttgtaggcgt 3600
ttgtttcgtt gcgcggtttt gtgcgtttgt agttttgtag tagggttgta aggtttaagt 3660
tttgggaagg tgtgcgagtt taggattttt tttgcggtat tggtttttcg tagagtaggg 3720
tagaagttga tttattttgg ttttgggatt tcgggtattt acgttttcgt cgtttttttc 3780
gtagtgtttt ttttttttcg ggtttcgagg gattttggta aggttttttt ttcgcgggga 3840
gtgagggtga cgagaggatg ggcgtttcgg gttagtcgga gaattttttt tagttagttg 3900

tacgtaacgg gtattttatg gttcgtgtag agttaattgt ggatcgcgtt gttgtttggt 3960
attggcgcgc gcgtttatat tcgcggttta gatttaaggt ggtcgggagt agatgaggga 4020
gagtaaggaa attaggatta ttttttattt tttttttttt cgatagttgg agtaggttat 4080
tttgtttgtt tttcgttttt ttcggtttta agtaagtttg ggaatcgggt cgggaagtag 4140
ggcggtacgg aggtagtttt tttttttttg tcgttagtat tttttttttg tatttgcgtt 4200
ttattagcgt tattttatta gtcgttgtac gtatttagtt ttaaaggtag gatttgaata 4260
ttttaatagg gtaataaaaa ggatgttgat ttatatttgt aaggaaattt atttttggtt 4320
ttagttatac gtgtatggag ttttagaaag tttagaattg gttgggagat tggtagttta 4380
gaaattttaa aggaggtggg gagagagaga ggaatttata ttttgtttag tttttttttt 4440
ttttatatta atatttattc gtttttaatt tttatgtaag taggttgaaa attaaagtga 4500
tgtaatgtta ataagtttta agtttttttt ttaaggtatt tcgggtttgg aagaacgaaa 4560
gagtattttt aaagaaatat taatatattt atattttgat gggtataatt gcggatacgt 4620
aatcgtaaag ttagtttgc gatatagtta gttatttagg gacggcggga gtttttttg 4680
gagtttttgt tttttgttag aatcgttttt tggttttttt agttgagaat tttttggtgg 4740
ggaggtgtag ggtgggggta gtggtgggaa agaaaattat agaggtaacg ggaaacgtgg 4800
gggttttgag ttttaatgtt taaggttacg ttcgttcgtt cgtatagtat taggagatta 4860
ttttatgttt gtgggagggg gatttttagt tttttttttt atttttattt ttgatttgga 4920
aagggtcgac gtttttaaag tgaaggtttt tttaagttat aagttagggt agaggtagga 4980
tggggaaggt tattgtagta agatttatta aattagggtt ataatgaatt acgtttgtta 5040
tcgtttttat ttttattata ttttaaattt ttttagttgg atttttgatt tttatttttt 5100
tttaggaaga ggaagagatg gggggcgggt aggtgggtgg ggaaggagcg ggtgaaatta 5160
tttttttatt tgttcgaaga gtaaatagag ttaagtttgg aagtgttcgg tttttcgtaa 5220
ttttttggaa agttatatat tttacgtttc ggcgttgggt ttttatttga gttattagtt 5280
tgttcgtttc ggtgtttttt ttatttttaa atatgatgtc ggggttgtaa ttgggggtga 5340
gttttttaaa tcgttcggag tttttggaga tttttttttt atatttttcg ttggcgttta 5400
gggttttttc ggttatattg gggataaatt gtttgtaggt taaaggggtt agttttttgg 5460
ggtgtttttt tttttcgaat tttttgttcg gttcgtacgt tagtttcgag tatattagta 5520
gcgaggagac gaggattagt agtagatatt tcgttagtag tagtatttcg tttatggaat 5580
tgatgatttt cgagttgttt tcgtgcgggt tcgtgcgcga gtgcgcgcgg cgggtgtgtg 5640
cgtgtgcgtt tttttttgcg tttttttttt ttcgtttcgg ttcgttcgtt cgtttttttt 5700
ttcgttggtt gtttcgtttt tttttttttt atataatttt gttcgtcgcg gttgcggggg 5760
atttttatc gtttttattt tagatagggg ttggaatggt aggttgtcgg tcgttgataa 5820
cggaatatat cggagttggg tcgcgagata gtaattaaaa gataaaaaga gtttgatttt 5880
aaatggtagt aagtttggag agtttgtgag agaggttgtt tttagtatta tattatagtt 5940
gttaggggcg tatttgattg gttaaagtcg tataagtttg ttttttgatg ttaattttga 6000
agaagattac gttttttttt tttgttgttg ttgttgtttt atttttattt gaaggattcg 6060
agttaaaaaa aaaaaaattt tttttaagat aaaggtgggc gggagggagg agcgtatgtt 6120
ttgtggtaat agtagttatt ttcggttttg tgtatatttt tgcgtttttt tttgattttt 6180
tttggaatgg taggtgtttt agttaaaatt aatttaagtt tttaaaggat agtttaaatt 6240
ttcgtaagtt ttagttatta agtatagagt cgtttatata tttgaggggt atagttgttt 6300
agttgttatt taattttttt tttttaggga ttttatagtg taggttggtt ttttttgtat 6360
tttgaagaaa gaggattgag ttttttttga agatttagtt tttgtggatc gaggataatt 6420
atgagtgatt ataatttaaa gtattggtat ttgaaatgtt ttaagtgttt tagaaaaatg 6480
taaaaatatt gtttttttat tgtgttttaa ttaatatatt tttttttttg tagagttttg 6540
taaatgggat ggaggcgtgt ttaagggtag ttatagagtg aattgcgggc gttagggtgg 6600
atgggtaaag taggtttggg ggttgtattt tagtataggg gagagagtgg gggttagtgt 6660
gtgcgttggg tgtgtggagg ttattgtagg gtgttggggg gggggtaggc gagtgggcga 6720
agttgtgttt atataatttt tacgatcgat tagagtaaag tgtagagtta ggggtgagtt 6780
gaatgtggaa tttaggtttt ttgtaaagtt ggttttgtgt gggggtttag taggttagta 6840
gtttttaggt ataggagtta ttagttttag gggttgtagt ttaaggggtt aggggttttg 6900
ggggaatagt gttttggtta acgaggggtt ttttagaaag agggtatagt gtttaggagg 6960
tggtaatttt tgagtttata attattttat tttttgtaga aaaaggaaaa aaatttaaaa 7020
gttttaagta aattaggaaa ataaaatttg aagttttttt ttaattttgt tttttttttt 7080
ttttttaaaa aaaaaaaaat agattatgtt tttttttttt ttaatcgtta ttttatttat 7140
gtgtaatgat ttttttagta taggattaga gagtgagggt tatttacgtg gaggggaaat 7200
tttgattttg aggagttatt agatgttgta aatgtggttt tgtgaagtta aatatttttt 7260
tttgatttcg ggtttttgga gtggaggggg agaggatttt tgtgaagttt agtttttaaa 7320
cgatggaagt ttttagaagt tttttttgta gattaatttt ttgtgtgtgt ggtgggggag 7380
agaaggagag gagtatttta ggatatattt gttttgggta tgagaggttg agcgttaata 7440
agattcggtg gtttgggtgt ttttggggag gttgattcgg gttttttttt tcgttttttt 7500
tttttgtttt tttttatggt ttgtgacgaa tcgtgatttt tggaggagag tatcgtgggg 7560
gagtagcgtt tatttgatta ggtttcggta gtttttattg agcggagttg gagtttagtt 7620
gagtttaggg attttttagg cggtgtgttt gcgttgtttg tttcgttttt agtaaaaata 7680
gatataaaat aggattcgtg ttttttttag aggaggtcgg tttgtttgat gtgtttattt 7740
gtatagtatg tggatgttat attattgggt ggtgtttggg ttggattagc gatttggttt 7800
agtgggatat ttgtaaattt ttgattgttt tgttttttat ttttggtatt tttttttgtt 7860

ttagtttttt tttttttttt tgtttttttt cgttatttat ttttttttaa atgtaggtag 7920
aaggtttttt atacgggttg ttattttggg gtttgagaga ttgggtgtgt agttttgtcg 7980
ggaggtggta tgtgtatata gtttattttt gtaaggggta gggtgggtgg ggtgtgtttt 8040
tggaagtaaa agtttgagtt gtatagagtt ttttgtgggt atagttggac gtggggtgtt 8100
tttaggtgcg gttagttttt ttttagtaga gcgtagggag aagacgtggt ttggtattga 8160
gtttgggtga tttagggtat tttttgaaat ttttttgttt taagggatgg cgtgaggttt 8220
tttttttttt ttttattttt agatagtttt agatattttg gagagtattt taggggtcgt 8280
tttttttgta gattggtcga ttttttagag tgaaataatg atgggttaga aagtttgttt 8340
ttacgtttaa gtttagaggg tttgtttttt gtatgggttg aggggagttc ggcgtagaaa 8400
gtaattttag ttatagatta ggttgttgat tgagtttttt agagaagttg ttgttttgtt 8460
ttttcgtttt ttattttttg tttttttatt ttatttgttt tttacggttt agtattgttt 8520
ttttttttag aggttaggtg gttttatttt tttgtttttt tttttttttt tagattattt 8580
ggtttttttt tcgtattagt agaggtttta aatattagtt tttttgataa ggttagatat 8640
tcggatatgt tggggtttcg ttatttattt atttgggttt tgtttttttt ttttggggtt 8700
acgcgtattt ttttttcgtt ttttttttta gttttttttt ttttattttt ttttattttt 8760
attttttgtt tagtattttt gttgaggata gaagttgttt ttttttttgt tttagttttt 8820
aggttttttt ttgggtattg tttttgtttt ttttttcggt ttttgtcgtt agatggtttt 8880
tttattttgt tttcggttcg ggtttggtcg gtttgggaaa ttttcgtagt tttaggtcgg 8940
gaggttcgga agttaggtag tttttttcgt ttattttttt ttggtttcgt tagtagtttt 9000
tgcgtttttt ttattttttt tttttttttt tagatttgta ttcgttgggt tttgtagtta 9060
tggagttcgt ttgtttggta tttttaggtg ttgtttttgt ttttagtttt tggggttggt 9120
ttttaatttt ttatgttttt tttttttttg attttttttt tttatagtaa ttttacgtaa 9180
tttttttttta tttagttatt tagttttttat tttttttttgg atttttttttg ttttggtcgg 9240
tggaattttt gtattttagt ttttttgtga ttgtatttag ggttttttga atttgggagg 9300
gattttgtat tttagttttt tttttttaat tttatggttt tttttatttt cgggttttgg 9360
ggatgtgagt ttttagtttt ttggagggtt taggttttgg atagtttagg aaatttagtt 9420
atattttgta tagtattttg attgtggttt ggttgtaggt ggattgaggt aatgttgttt 9480
ttttcgtgtt aaagtttagg agttttgggg tggttgtagg gttttttgga tttttgtagt 9540
tatattttta tggtattagc gttgagggta ggttcgttgg gaggtagttg ttaagatata 9600
tagggtttcg ggggattatg gttggtatgt ggagttagtt acgttgggtg tttattgtgt 9660
agcgtttttt agtagtgttg tgtagcgggt acggggttat ttttaagttg ttataggttt 9720
tatgattgtg ggtgtagttt ggtttttttt cggtttgggt tattttgtga agtagggtgt 9780
aggagatatt ttggtgattg ggatttttgt ttttatgttt gttcgtttgt atattaagtt 9840
tcgtggagtt tcgtggagtt tcgtggagtt tcgtggagat tcggtttata tgtatgaagg 9900
ttgtaggttt tgagagatga ggattttgta gtttttgggg acgttggaaa gattatatag 9960
ttagttttgt cgaagtagat attttaaggt aggcgattag ttcggatgtg aacgtgtagt 10020
ttgaattatg ttgttgtgtg tttttgagtt gatttttttt ttttagatta ttgtttgttt 10080
ttgttgagtt atttcgtttt tattttaagt aatggatatt tttttttagg tttaaataat 10140
gttttaggtg tgtgtttttg tatagtttta aagtgtttta gtatcgttgt taggggagtt 10200
tgaagttata tttgggagtt ggaaagggag gtatagtgtg ggtaggtggt ttcgtgttta 10260
gggaggaagg gattttgcgt ttgtatgttg ttttttgtat tttagttatt tagagatttt 10320
tttataagat tattattgtt tttattttgg agtttagtta agaggtaggt gttttagata 10380
gttaatgtat taaatttttg cgtatatggg ttttatttgg gggttgagaa aaagtcgata 10440
ggtcgcgttg aggttttatt ttcgagtatt taaattagaa tttttttttt gttgattttt 10500
ttgatgggga ttttaaaatt taggagcgaa cgtggagggg ggagatgtgg gtgtaattgt 10560
tagttttaag ttttttgttg tttttttagt tcgcggatat tttttagttt tcggatgttt 10620
cgtttgtttg gttttttagt ttttggacgt tttttagttt tgtttgatgt ttgtgtgttt 10680
tgggtttcgt ttgttcggtt gggtttggta tggagtt 10717

<210> 229
<211> 9656
<212> DNA
<213> Artificial Sequence

<220>
<223> chemically treated genomic DNA (Homo sapiens)

<400> 229

ttgtatgttt tattgatgag gaaattgagg ttgggagagt ttgtggtagg ggttaagtag 60
gtttatagtg gcggggtatg gtagtggtgg ttgggtaggt ttttgtagtt tttttttttc 120
ggtaggtgtt tagtattggg tgtacgacgg tgaaaagtta gttttgggtt tcgtattttt 180
tatcgagttg ggtttggtga ggttttcggt ttatgttgtt ttggtttggg gtttcgagaa 240
gaataagatt tatttttttc gaggtaggga ttattggcgt ttttatttta gtattcggcg 300
tgtagatagt ttcgtgtttc gtagggttat tgattggaga ggggtgtttt ttgagatcga 360
cgttgttttt taggatgttg atggtgcgtt gggggtgagg tagttggtgg gaggtgggta 420

tagtagtcgt tttttttatt tggtggtggt tgggttttta tatgtttgtt ataggaagtt 480
tggtttttta ttataggttt tttgtttaga gttatttgtt tttttttcgg tggtcggtta 540
gtgttatatt ttatattgta gatgaggaaa ttgagggtta gagaagtgta aggttttatt 600
ttggtttttt agttatagtt agtagaataa taaattgttg tatattgagg gttaataatg 660
ttttaagttt tttattggtt ttatttagtt tttagaatag ttttttgatg tgatatttgt 720
tgtgaattta gtttttagag gagtaaatag aggtttaggt aatgaggttt ttaatttgga 780
ttattttggt ggtttttgtt tttaattatt gatttattta gttttttata attatagggg 840
gttagagtta gtttagtgtt tttttttttg ttaggttttt tttttgtgtt ttttttttta 900
tattaggatt tattgggaga gttattttag ggtagttttt agttttagga ttttagggtg 960
ttcgttaata gtttggttaa tttaatagat gtaggagaga gtgatgtgga gggtggtggg 1020
ggtaacggga tttgtttttt tgagaggtgg gatttaggtt tttgaggttt tgggtatttg 1080
ttaggttggg tattagttta gtttagattt cggggtaggt agaagggttt tttagaggga 1140
agagaggttt tgaaaggtcg gttttggatt ttgtaggatt cgaggaattt agtagtggtt 1200
aaggtttttt tatttagttt ttttttagtg tttatttttg ggtatagttt gataggtagg 1260
agtagggttt agtgttcgtt cgtttaggtt tgattatttt ttttttttag gttatgttta 1320
ttttttgcgc ggtcgttttt attggaagtt tgattttgtg aaggtgaagg ttttggaagg 1380
tttttttcgt ttcgtgggtt ttgatttttt tggttgtgtc gagtttgtta atattttttt 1440
ttgattatgg tttggatgtt tttaggggtg ttgatttttg ttaggttacg aatattaggt 1500
tagagattta tggttatttt tgtggttgtg ggtattaggt atgggattga gtttatgttt 1560
ttttaggggg atggggtggg gtataattat tatgataatt gtcgggaggg ttacgtaggt 1620
cgtggttatt tgttagcgat tgttttagat tgggtaggga ggttttggta tgatttaaga 1680
ggaagggtag ttttgggttc gttatgtagg ttttggtaaa tttggttgtt ttgtttttat 1740
ttttgttttt tagggtagta ttatggtagg attgggggaa ttggagtgtt tttgttgtat 1800
ttttgttgtg aggttttttt taggggttgg tattgaagta agggtgttgg ggttttatgg 1860
tttttagttt tggttgagta attgggttgt agggtagggt tattttttga ggttaggttt 1920
tggtaggtgt ttgtatttgt ttgttttttg gttgataatt ttggaaattt gttttttaga 1980
atttaggtta aaaagtttat agttaaatgg ggaggggtat tttttatgta ggagatttta 2040
ggttttggag gttgtaatat attttaattt tgttttaggt cggatttttt tgaagttttt 2100
ttcgtagtat tgttattttt taaagttatt gtaaatgtgt gtatagtgtg tataaatttt 2160
tttttttttt tttttttttt aaattgagga ttgttattaa atatagttgt tttttatttg 2220
tttgtttggt ttttttttgt gaatgtttag ttaggatggg gaggtttggt tattgtttag 2280
ttttattttg ggagttaggg tcgaagttta ttatttttaa gtttttggtt tttggggttg 2340
ggtatagtgt ttagggtttt ttttgtttta tttttatatt atattttgtt atagtggttt 2400
tttaattggt tttttatttt tttatttgtt ttttttgttt attttttaga gtggtggttt 2460
atttgtttat gttatagttt agtttgttat atatagttgt ggtttttgta ttcgtaggtt 2520
taattaattt cgggtagaaa aaataatgtt taaaaaaagg atgcggacgg gcgtagtggt 2580
tcgtgtttgt aattttagtt ttttgggagg tcgaggtgtg tggattattt gaggttagaa 2640
gtttgagatt agtttggtta atatggtgaa aatttatttt tattaaatat ataaaaatta 2700
gttgggtgtg gtggtttatg tttgtaattt tagttatttg ggaggttgag gtaggagaat 2760
tatttgaacg tggaggtaga ggttatagtg agttgagata gtgttattgt attttagttt 2820
gggtgataag agtaaaattt cgtttaaaaa aataataaag taaataaata ggttgggtgt 2880
agtggtttat gtttgtaatt ttagtatttt gggaggttga ggttagtaga ttacgaggtc 2940
gggagatcga gattattttg gttaatatta tgaaatttcg tttttattaa aaatataaaa 3000
aattagttag gcgtggtggc gggcgtttgt agttttagtt attcggaagg ttgaggtagg 3060
agaatggcgt gaatttagga ggcggtgttt gtagtgagtt tagattatgt tattgtattt 3120
tagtttggac gatagagtaa gattttgttt taataaaata agtaaataaa taaataaata 3180
aataaataaa taagggatgg ttatttttgt attgaatatg tacggatttt tttttttttatt 3240
attgtttttt aaataatagt ataataatta tttatataat atttgtattg tattaggtag 3300
tataaggaat ttagagatga tttaaggtat ataggaaagt gtgtgtaggt tatatgtaag 3360
tatgatatta ttttatataa tggatttgag tatttgtgga ttttattatt tgtagggggt 3420
tttggaatta atttttatgg atattgaggg atgattgtat tttggttttt tattattgtg 3480
aggatataga ataaagtaat tttagttatt gtttttttta attttttagt gtttttttgt 3540
tttatttttt agttttatgt gtgttttcgt aatagtatag ttgtattttt ttttttttta 3600
agagaggtag ggtttttttt tgttgtttag gttgtagtgt agtggtgtaa ttagtttatt 3660
ataattttga atttttgggt ttaagggatt tttttttttt tattttttaa gtagttgggg 3720
ttataggtgt atatcgttat gttatattta gtttatttat tttagaattt ttgtagagat 3780
agggttttgt tgttttgttt agtttggttt taaatttttg gttttaagtg attttttttg 3840
ttttggtttt ttaaagagtt gggattataa ggtcgggcgt agtggtttat atttgtaatt 3900
ttagtatttt gggaggtcga ggggggcgga ttacgaggtt aggagatcga gattattttg 3960
gttaatatgg tgaaattttg tttttattaa aaatataaaa aattagttgg gcgtggtggc 4020
gggtgtttgt agttttagtt atttgggagg ttgaggtagg agattttagt tttaaaaaaa 4080
aaaaaaagag ttgggattat aggtatgagt cgttgagttt agttatagtt gtaattttat 4140
tagattgaac ggtgtgaaat tattagtagt gggttattgt tttatttttt gtattattta 4200
attatgattg tttttagatt tttattagtt ttattagttt cgcgttgtaa gagttaagag 4260
gtagagttat agggttgggt tgtttggttt tattttttat tgtagggttc ggttaagtag 4320
agtgtaggtt aagagaggaa tggagaaggt ggaaggtgtt tttttttttt aacgtttatt 4380

tgatgtatta gttaaattaa atattatttg ggtttttgaa tattgttaag ttttattcgt 4440
tgttgttatt ttgtttattt ttatagttgt agggatagtc gtaggttcgg aattgggata 4500
ttttttttta ggggtttgtt tatttgttta gttgtattgc gttttagggg tggggaagtt 4560
aaggtacgag attggggttt taaagtatgt ttagattatt taggttttta gatattaggt 4620
tttttttttt taggttttat ttcgttgttt tttttttggg tttagtttta gttttggttg 4680
taagattggg aaagttattg tttgagttag gattttattg gttatttttg ttcgtgagaa 4740
gttttttata ttacgatttg ggaggtagtt tgggagaaag agaaattagt cgtggttttt 4800
ttaaggggtt cgcggaggcg cgttcggttt tttgtttgag cggcggcgcg cgcgttagcg 4860
ttaacgttag cgtttgcgta ttgagggcgg tttggtcgtc gtttgcggcg gcggcggcgg 4920
ttgaggagtt cggttgaggc gttagtattc ggttcggttc gtatttcgtt tttcggtttc 4980
ggtttttttc ggtttagtac gtttcggttt cgttttagtt tttttgattt ttcgtagttc 5040
ggtttcggtc gttcgttttt gtcgtcgtaa tgatgatgat ggcgttgagt aagattttcg 5100
ggtagaagtt cgtgaagttt tagttggagg acgacggcga gttttatatg atcggtttcg 5160
aggtagttcg ggcgcgtttt tcgtttttt cgggttcggt ttcgcgggag tttcgggcg 5220
ggtttatgcg tcgagagcgc gcgtttttat ttatcggggc gggcgggcgc gcgcgcgcgc 5280
gttcggggtt gtggggcgtg gtttagtggg cgtgtcgggt gtggttttcg tttttttatc 5340
gatttgggat ttttttattt atttcgtcga ggttcgtcgt ttttagtgtt gttaagattt 5400
tggcgtcgcg aggggtttta gtggtttcgc gttgggttgg tttttagtta gacgtaaaga 5460
aacgggatat tgagttagtt tttgcgacgt ttgggggatt gtattgttgt ttttgatttt 5520
ttttagatgt cgtaaatttt acgtgtattt tttttcgtat tgttatttgt gggaatttta 5580
aatggtaggt tggaaatttt ggatttagtg agggtttatt tttattgtgt ttgttgtttt 5640
ttaggtgagt agtggaggtt ttttgatttt tgtttatcgg tgaagaaata gattggagga 5700
agatgtttgt ttttgggtga ataagggttt tatgtttaag ttagattttt tagtagtttt 5760
gaagcgattg atatttttta tatttgtgaa ttgattttga attattatag gggaaagagt 5820
ttaaggatgt ttaattttta attagagtgg ttgtattttg ttgtattaat tggatgattg 5880
attttaggaa aattttattg gtttttgaat gggtgtttgt tttaagttta ttaggttttt 5940
gttttttgtt ttttatagag atagggtttg gttatgttgc gtaggtaggt ttttaatttt 6000
tgggtttaag tagtttgttc gtttgggttt tttaaagtgt taggattata ggtatgagtt 6060
attatattcg gttatatatt aggtttttat agattgtttt aaattagttt tttttgtttt 6120
aaaggtagtt tttagttagt atatgttatt tttttaaaaa aattatggat atatagttta 6180
gatgtataaa tgttaagtag tagaatttta tgtaaggaag taagtgttaa ttttggagaa 6240
ttaaagttat tatgaaatgt taagagtttt ttttttttat agtagttttt agtattatat 6300
tttagtattt tattcgtatt ttattttttt ttatttttga aaaaagattt tattttgtgt 6360
agtggtgtga tttcggttta ttgtagtttt gattttttgg gtttaagcga tttttttgtt 6420
ttagtttttt aagtatttgg gattataggt gtgtattatc gtgttcgatt aatttttgta 6480
ttttttgtag agatagggtt ttgttatgtt gtttaggttg gttttgaatt tttgggttta 6540
agtgattttt gtattttagt tttttaaagt gttggaatga taggtgtgag ttattgtatt 6600
tatatttagc gtatttatat tttttaaatt tgtgggtttt attttttata attgagtata 6660
gtatgaatga atgaagtttt aagatggtta tattgagttt tatggatgag attaaggtta 6720
atagtattag aattttagtg ttagtatttt ttattatttt ttttttttgg acgaggtttg 6780
attttattat ttaggttgga gtgtagtggt gtaatttcgg tttattgtaa ttttcgtttt 6840
ttaggtttaa gtcgtttttt tattttagtt ttttaagtag ttgggattat acgtatatat 6900
tattatattc ggttaatttt tatattttta gtaaagatgg ggttttatta tattgtttag 6960
gttggtttta aatttgtgag tttaagtgat ttgttcgttt cggtttttta aagtgttcga 7020
ttataggcgt gagttattgt gtttggtttt tattaatttt tataaaataa aaaatatagg 7080
atttaataaa atttttacgt tgttatttaa attataattg tttaaagatg ttagtgttgg 7140
ggaggtagag ttagtgtttg tttatttgtt atagtttaga atggttttta ttggttagta 7200
ggtatttgtt taattgtaga atgtaaaagg tgttaaattt tagtttgtga tattaggcga 7260
gttgtttatt tgatttaatg ggtgtaaaag taattagttt ggttttaagt aggtgatatt 7320
ttaaaagaaa gtttttattt attagttgga gtaaattgag aagttgtgcg tggttaagtg 7380
ggaagaattt tgggaggttt ggttttagtt ttgttttgtt ttgttgtcgt gttttttatg 7440
tgtataatgt gatgtttaga ttagatggtt tttaagattt tttttaaaat atattttagt 7500
ttttagaaag ttaattatag ttttgttttt tagttttttg atttttgggt tttttagtaa 7560
gaataatttt tttgagattt aggttgtaag agatttagtt ggagaggtag tttgtttttt 7620
tgggtttgat ttaattatta gattttcgga gtttagggaa gagtttaagg ttagagtttt 7680
attgaaattt agtagtttta gtgatagtag tggatggacg gttttagttt attgttttgt 7740
ggaaaatagg tattgtattt agagaggtgg gttagttata gtaatatatt tagtgttttt 7800
atgcgttatt tgtggagttg ttttgggaag gtatttgtat ttattaagag tgtgtgttat 7860
tgttttgtta ttaatagata gttgtattta ttaagtcgga gttatggagt tttgaggatt 7920
ttaatagatt tttaagttta gtattttgat tttatattta ggaaatttgg gttttaagag 7980
ggtgaaggtt ggtttagagg aaagttagga aggtaagttt agttttttaa gttttgaagg 8040
ggttttgggg ggtatttagg agatttagag ttttgtaggg tttgtaaggt agttataggg 8100
gggagtattg ttaatttttt ttaaaaaatt taatggaaat tttatattta ttatgatatt 8160
ggatgatatt gatatataag ttaaataata tagaggggtt tgtgtttttt atgtggaaag 8220
cgagtgattt gttgtgtttg tttttttttt agcgtttagt agttttggtt gttgattata 8280
gttattgttg aaaatggaga aatattttat ttaatattat gtttgggttt tatatttata 8340

attttagtat tttgggaggt ttaggtagga gaattatttg aatttaggag tttaagatta 8400
gtttgggtaa tatagtgaga tttttgtttt tatagatttt tatttttatt ttttttaatt 8460
aggtcgggta tgtggtttat attcgtaatt ttagtatttt gggaggtcga ggtgggtaga 8520
tcgtttgagt ttaggagttt gagattagtt tgggtaatat ggtgaaattt cgtttttata 8580
aaaaatataa aaattagtcg gttgtggtgg taagtaatta tagttttagt tatttaggag 8640
gttgaggtag gaggatcgtt tgagtcgagg agttcgaggt tgtagtgagt tatgatcgat 8700
cgtattattg taatcgagtt tgggtgatag agcgagattt tgttttaaaa ataataataa 8760
aaattaaaaa taaatttaaa aatgtaaaat atatattatg ttgcgtagat aaaatgggaa 8820
atataataga agtggttttt tatgggggtg aagtttagag ttgttgagta tttggtagat 8880
gagtaaaggg tatttagaaa atgggaagtg attttaggtt atagatggag ttagtatttg 8940
gtatttagtt tattggttat tttttgtaat gtgatttcga agtgattata tttgaaaagt 9000
taacgagata ataggtattt tttataaaaa ttttaggttt agatttattt tttttaagga 9060
agttatattt ttagtatttg ttataagtaa attgaagtta ttgtttgagt tagtgatgat 9120
ttatatagta gtttggtgat tgttttgtaa atttttggga aaattttttt ggtatttttg 9180
gtttgggttt tgttattaaa taaagtaata taggttatgg gggaaattat ttggtatttg 9240
tttaaaatta aaagattttt ttggtgttta ttatattgta aagatattaa agttttgttt 9300
ttaagataaa agaaggttaa ggaggttttg tatttttgga aagtgttaga gatttttagt 9360
ttaaaattaa aattattacg aattttttgt taagttttgg gggtttttta tttaggtatt 9420
tgtttaggtt tttcggaatt tggttttgtt gttaggatgt atatatgtag gttagatgtg 9480
tattgttggt gaatttttta tattagtaga atgtgtttta atgttgtcga aagcgtggcg 9540
tttgggggtt attttaaggt ttgtttgttt gttgtttgat gtagtttgcg ttaggatttt 9600
tttttttttt gtggtgttgc gatttttata atgagttttt ttgttttatt tatagg 9656

<210> 230
<211> 9656
<212> DNA
<213> Artificial Sequence

<220>
<223> chemically treated genomic DNA (Homo sapiens)

<400> 230
   tttatgagta aagtaagaag gtttattata agggtcgtag tattataggg aaggggaggg 60
   ttttggcgta gattgtatta agtaatagat aaataggttt tgaggtggtt tttaggcgtt 120
   acgttttcgg tagtattaaa atatattttg ttgatatgaa gggtttatta gtagtgtata 180
   tttggtttgt atatgtgtat tttgataata gaattaagtt tcgaaaggtt tgggtagatg 240
   tttgagtggg aggtttttag agtttagtaa gaagttcgtg gtgattttga ttttggatta 300
   aggatttttg gtatttttta gaaatgtaag gtttttttga ttttttttta ttttaaggat 360
   aagattttga tatttttata atgtaataaa tattaaaaag gttttttaat tttagataga 420
   tgttagataa ttttttttat ggtttgtgtt gttttgttta gtaataaaat ttaaattagg 480
   gatattaaag gaatttttta aaaggtttat aaggtaatta ttagattgtt atataaatta 540
   ttattgattt aaataatggt tttagtttgt ttgtagtaaa tattgaaagt gtgatttttt 600
   taaggaaaat aaatttagat ttaaagtttt tatgagggta ttttattgtt tcgttgattt 660
   tttaagtatg attatttcgg agttatattg taggggatgg ttaatagatt gggtgttagg 720
   tgttggtttt atttgtaatt tgggattatt ttttattttt tgggtatttt ttatttattt 780
   gttaaatgtt tagtaatttt gaattttatt tttatgaagg attattttta ttatgttttt 840
   tattttattt acgtaatata gtatatattt tgtattttta aatttatttt taatttttat 900
   tattattttt gagatagggt ttcgttttgt tatttaggtt cgattgtagt ggtgcgatcg 960
   attatagttt attgtagttt cgaatttttc ggtttaagcg atttttttgt tttagttttt 1020
   tgagtagttg ggattatagt tgtttgttat tataatcggt taatttttgt attttttgta 1080
   gagacggagt tttattatgt tgtttaggtt ggttttaaat ttttgggttt aagcgatttg 1140
   tttatttcgg ttttttaaag tgttgggatt acgggtgtga gttatatatt cggtttaatt 1200
   aaaaaaaata aaaataaaaa tttgtagaga taagagtttt attatgttgt ttaggttggt 1260
   tttaaatttt tgggtttaag tgattttttt gtttgggttt tttaaagtgt taggattata 1320
   ggtgtgagat ttaggtatag tattaaataa aatgtttttt tatttttagt agtgattgta 1380
   attaataatt agggttattg aacgttgaga gaagagtaga tatagtaagt tattcgtttt 1440
   ttatatagag ggtatagatt tttttatgtt atttggtttg tgtgttagta ttatttagta 1500
   ttatggtaaa tgtagagttt ttattaggtt ttttaaaaga aattgataat attttttttt 1560
   atgattattt tgtaggtttt gtagggtttt gagtttttta gatgtttttt aaagtttttt 1620
   tagaatttag gaaattgggt ttgttttttt ggtttttttt tgaattagtt tttatttttt 1680
   tgggatttag gttttttgaa tataaaatta aggtgttgga tttggggatt tgttagggtt 1740
   tttaaagttt tatgatttcg gtttggtaaa tatagttgtt tgttaatggt agaatagtga 1800
   tatatatttt tagtaaatgt agatgttttt ttaagatagt tttatagatg gcgtatggag 1860
   gtattgggta tgttgttatg gttggtttat ttttttgaat atagtgtttg ttttttatag 1920
   ggtagtgggt tgagatcgtt tatttattat tattattgga gttattgaat tttaatagag 1980
   ttttgatttt gaattttttt ttaaatttcg aaggtttgat gattaagtta ggtttaaaag 2040
   aataaattgt ttttttaatt gagtttttta tagtttgggt tttaggaaga ttatttttat 2100
   tgggaggttt aggagttaag gggttgaaag ataagattgt aattagtttt ttagaaatta 2160
   aaatgtgttt tggggaagat tttggagatt atttagttta agtattatat tatatatatg 2220
   agaaatacga tagtaaggta gagtagggtt ggaattaggt tttttaaggt tttttttatt 2280
   tggttacgta tagtttttta atttgtttta attaataaat gaaagttttt ttttgaagtg 2340
   ttatttattt agagttaaat tgattatttt tatatttatt agattaggta aataattcgt 2400
   ttagtattat aggttgaaat ttgatatttt ttgtattttg tagttaggta ggtgtttatt 2460
   ggttaatgag gattattttg aattgtggta gatggatagg tattggtttt atttttttag 2520
   tattggtatt tttgaatagt tgtaatttaa gtgataacgt gaaaatttta ttgaatttta 2580
   tattttttgt tttataaaaa ttagtggagg ttaggtatag tggtttacgt ttataatcga 2640
   gtattttggg aggtcgaggc gggtagatta tttgagttta taagtttaag attagtttgg 2700
   ataatatggt aaaattttat ttttattaaa aatataaaaa ttagtcgggt gtggtggtgt 2760
   gtgcgtgtag ttttagttat ttgggagatt gaggtgggag gacggtttga gtttgggagg 2820
   cggaggttgt agtgagtcga gattatatta ttatatttta gtttgggtga tagagttaaa 2880
   tttcgtttag aaaaaaaaaa tggtggaaaa tgttaatatt aaagttttga tgttattgat 2940
   tttagtttta tttatgggat ttagtgtgat tattttgagg ttttatttat ttatgttatg 3000
   tttaattgtg aaggataaaa tttataagtt taaaaagtat gaatgcgttg agtgtgagtg 3060
   tagtagttta tatttgttat tttagtattt tgagaggttg aggtgtaagg attatttgag 3120
   tttaggagtt taagattagt ttgggtaata tggtaaaatt ttgtttttat aaaaaatata 3180
   aaaattagtc gggtacggtg gtgtatattt gtagttttag gtatttggga ggttgaggta 3240
   ggaggatcgt ttgagtttag gaggttaagg ttgtagtgag tcgagattat attattgtat 3300
   aaagtgagat ttttttttaa aaataaaaga aaataaagta cgaataaaat gttggaatat 3360
   gatattaaaa attgttataa ggagaagaaa tttttggtat tttatgatag ttttaatttt 3420
   ttaggattgg tatttatttt tttatatgag attttattgt ttgatatttg tatatttgaa 3480
   ttgtatattt ataatttttt taaaaaggta atatatatta gttggggatt gtttttggag 3540
   taaaaaaggt tagtttgaga taatttgtaa aaatttaata tatggtcggg tgtggtggtt 3600
   tatgtttgta attttagtat tttgggagat ttagacgggt agattgtttg agtttaggag 3660
   ttggagattt gtttgcgtaa tatagttaga ttttgttttt ataaaaaata aaaaataaga 3720
   atttaataga tttggaataa atatttattt aaaggttagt agagtttttt tgaagttagt 3780
   tatttagttg atgtaataaa atgtagttat tttagttaag ggttaaatat ttttaggttt 3840
   ttttttttgt aataatttag aattagttta tagatatgga aagtgttaat cgttttagaa 3900
   ttgttgaaag atttagttta ggtatggggt ttttatttat ttaaaagtaa atattttttt 3960
   ttaatttgtt tttttatcgg taaataaagg ttaaaagatt tttattgttt atttaagagg 4020
   taataaatat aatggggatg agtttttatt aagtttaggg tttttagttt gttatttgag 4080
   gtttttataa gtagtaatac ggggaggggt gtacgtgaaa tttacgatat ttaaaaaggg 4140
   ttaaaggtag tagtgtagtt ttttaagcgt cgtaggggtt aatttaatat ttcgtttttt 4200
   tgcgtttgat tggaaattaa tttagcgcgg aattattggg gtttttcgcg gcgttaaaat 4260
   tttggtagta ttgaaggcgg cggatttcgg cgaaataagt aaggaaattt taggtcgatg 4320
   agggggcggg ggttatattc gatacgttta ttaggttacg ttttatagtt tcgagcgcgc 4380
   gcgcgcgcgt tcgttcgttt cgatgaatgg agacgcgcgt tttcggcgta tgggttcgtt 4440
   tcggggtttt cgcggggtcg agttcgggga gggcgagaac gcgtttcggg ttatttcgga 4500
   gtcgattatg tagaattcgt cgtcgttttt tagttggaat tttacgggtt tttgttcgaa 4560
   ggttttgttt agcgttatta ttattattgc ggcggtagag gcgggcggtc ggagtcgggt 4620
   tgcgagggat taggagggtt ggggcggggt cggggcgtgt tgggtcgagg gaaatcgaag 4680
   tcggaaggcg aaatgcggat cgggtcgggt attggcgttt tagtcgggtt ttttagtcgt 4740
   cgtcgtcgtc gtagacgacg attaggtcgt ttttagtgcg taggcgttgg cgttgacgtt 4800
   gacgcgcgcg tcgtcgttta aataaaaggt cgggcgcgtt ttcgcggatt ttttaaagga 4860
   gttacgatta attttttttt tttttaggtt gttttttaag tcgtggtgta gaggattttt 4920
   tacggataaa aatagttaat gaggttttga tttaagtagt ggttttttta gttttatagt 4980
   taggattgga attgagttta ggggaaaagt aacgaagtga ggtttgaagg aggaggattt 5040
   agtatttgga agtttgggtg gtttgggtat gttttgggat tttaatttcg tgttttagtt 5100
   tttttatttt taaaacgtag tgtagttgag tagataggta ggtttttagg aagagatatt 5160
   ttagtttcgg gtttacggtt gtttttgtag ttgtggaggt gggtaaaatg ataatagcgg 5220
   atgaggtttg gtaatgttta aaggtttagg tggtatttgg tttggttggt gtattagatg 5280
   agcgttggaa gggagagata ttttttattt tttttatttt ttttttagtt tatattttgt 5340
   ttaatcgagt tttgtagtgg ggagtgaaat taggtagttt agttttgtgg ttttgttttt 5400
   tggtttttat agcgcgaagt tagtgaaatt aatgaaagtt tgaaagtaat tatgattgaa 5460
   taatgtaggg agtaaaatag tggtttatta ttgatgattt tatatcgttt aatttaatga 5520
   aattgtagtt gtggttgggt ttagcggttt atgtttgtaa ttttaatttt tttttttttt 5580
   tttgaggttg gagttttttg ttttagtttt ttaagtagtt gggattatag gtattcgtta 5640
   ttacgtttag ttaatttttt gtatttttag tagagatagg gttttattat gttagttagg 5700
   atggtttcga ttttttgatt tcgtgattcg tttttttcgg ttttttaaag tgttgggatt 5760
   ataggtgtga gttattgcgt tcggttttgt aattttaatt ttttaggagg ttaaggtagg 5820
   gaggattatt tgaagttagg agtttgagat tagattggat aagatagtaa gattttgttt 5880
   ttataaaaat tttaaaataa ataagttggg tgtggtatgg cggtgtgtat ttgtagtttt 5940
   agttatttgg gaagtggaga ggggaagatt ttttgagttt aggagtttaa ggttatagtg 6000
   agttgattgt attattgtat tgtagtttgg gtaataaagg gagattttgt tttttttaaa 6060
   aaaaaaaaaa atgtaattat gttattacga agatatatat ggggttggaa ggtagggtaa 6120
   ggagatattg gagagttggg aagggtagtg attgaagttg ttttgttttg tatttttata 6180
   gtggtgggaa attaaagtat agttattttt tagtatttat gggaattggt tttaggattt 6240
   tttgtagata ataaagttta tagatgttta agtttattat ataaaatggt gttatatttg 6300
   tatataattt atatatattt ttttgtatat tttaaattat ttttagattt tttatattat 6360
   ttaatataat gtaaatgtta tataaatagt tgttatatta ttgtttaggg aataatgata 6420
   gggaaaaaag ttcgtatatg tttagtatag aggtaattat tttttattta tttatttatt 6480
   tatttattta tttatttatt ttattgagat agagttttgt tttgtcgttt aggttggagt 6540
   gtagtggtat gatttgggtt tattgtaagt atcgtttttt gggtttacgt tatttttttg 6600
   ttttagtttt tcgagtagtt gggattatag gcgttcgtta ttacgtttgg ttaatttttt 6660
   gtatttttag tagagacggg gttttatggt gttagttagg atggtttcga tttttcgatt 6720
   tcgtgatttg ttggttttag ttttttaaag tgttgggatt ataggtatga gttattgtat 6780
   ttagtttatt tatttatttt attatttttt tagacggaat tttgtttttg ttatttaggt 6840
   tggagtgtaa tggtattatt ttagtttatt gtaatttttg tttttacgtt taagtgattt 6900
   ttttgtttta gttttttaag tagttgggat tataggtatg agttattata tttaattaat 6960
   ttttgtatat ttagtagaga tgggttttta ttatgttggt tagattggtt ttaaattttt 7020
   gattttaggt gatttatata tttcggtttt ttaaagaatt gggattatag gtacgagtta 7080
   ttgcgttcgt tcgtattttt tttttaaata ttgttttttt tattcgaggt tagttgagtt 7140
   tacggatgta gaaattatag ttatgtatgg taggttgggt tgtggtatga gtagataaat 7200
   tattattttg gaggatgaat agaggggata agtaggaaag tggggagtta gttaagaagt 7260
   tattgtagta aaatgtgatg tagaaatgga gtagaaggag ttttgagtat tgtgtttagt 7320
   tttaagggtt agaggtttgg gggtggtgga tttcgatttt ggtttttagg atagggttgg 7380
   atagtggtta ggttttttta ttttggttga atatttataa aaggagatta agtaggtagg 7440
   tagaaaataa ttgtgtttaa tgataatttt tagtttaaaa aaaaaaaaag aagaagaagg 7500
   tttatatata ttgtatatat atttataatg gttttggagg atagtagtgt tgcgaaaagg 7560
   gttttaggag gattcggttt gggataggat tgaggtatgt tgtagttttt agggtttggg 7620
   gttttttgta tgaagaatat ttttttttat ttgattgtga attttttggt ttggattttg 7680
   gagaatagat ttttaggatt gttagttaga aggtagatag atgtaggtat ttattaagat 7740
   ttgattttag gaagtggttt tgttttatag tttagttgtt tagttagggt tgaaggttat 7800
   ggggttttag tatttttgtt ttagtgttag tttttggaag gaattttata atagggatat 7860
   agtaaggata ttttagtttt tttagttttg ttatggtgtt attttgaggg atagggatgg 7920
   agatagggta gttaggtttg ttaggatttg tatagcgggt ttaagattgt tttttttttt 7980
   aagttatgtt aaagtttttt tgtttagttt gagatagtcg ttggtaggtg attacgattt 8040
   gcgtggtttt ttcggtagtt gttatggtgg ttgtatttta ttttattttt ttgaggagat 8100
   atgggtttag ttttatgttt ggtgtttata gttataaaga tggttatggg tttttagttt 8160
   gatattcgtg gtttggtagg ggttagtatt tttgagggta tttaagttat ggttagagga 8220
   aagtgttggt aggttcggta tagttaaaga agttaggatt tacgagacgg gggaagtttt 8280
   ttagagtttt tatttttata gggttaaatt tttagtagag gcggtcgcgt aggaagtagg 8340
   tatagtttga gaagagaagg tggttaagtt tgggcgagcg gatattgggt tttatttttg 8400
   tttgttaggt tgtgtttagg gatgggtatt aagggagggt tgagtgggaa gtttttggtt 8460
   attgttgagt ttttcgagtt ttgtaggatt tagggtcggt tttttagaat tttttttttt 8520
   tttagggagt ttttttgttt gtttcggaat ttgggttggg ttaatattta gtttgatagg 8580
   tatttagagt tttagaggtt tgagttttat tttttaggaa agtaagtttc gttgttttta 8640
   ttatttttta tattattttt ttttgtattt attaaattag ttaggttatt gacgggtatt 8700
   ttggagtttt ggagttggag gttattttag gatagttttt ttagtgggtt ttgatgtggg 8760
   agaaagagta tagaagagga gtttagtagg ggagggagta ttggattggt tttagttttt 8820
   tgtggttgtg ggaggttggg tgggttagtg gttaggagta gggattatta gggtagttta 8880
   ggttaggggt tttattgttt gagtttttgt ttgttttttt ggaaattggg tttataatag 8940
   gtgttatatt agagggttgt tttgagaatt ggatgagatt agtaaggagt ttagagtatt 9000
   gttggttttt agtgtatagt agtttattgt tttattggtt gtggttgaaa aattagggta 9060
   agattttgta tttttttgat ttttagtttt tttatttgta atatggaatg tagtattagt 9120
   cggttatcga gaggagggta gatggttttg gataaaggat ttgtgatgaa gagttagatt 9180
   ttttgtggta ggtatgtggg agtttagtta ttattaggtg ggagaagcgg ttgttgtgtt 9240
   tattttttat tagttgtttt atttttaacg tattattagt attttggaag gtagcgtcga 9300
   ttttagaggg tatttttttt tagttagtgg ttttgcgggg tacgggattg tttatacgtc 9360
   gggtgttggg gtggaaacgt tagtagtttt tgtttcggaa gaagtagatt ttgttttttt 9420
   cgggatttta gattaaggta gtatggatcg ggaattttat taggtttagt tcggtgaggg 9480
   gtgcggggtt taggattggt tttttatcgt cgtatattta gtattgagta tttgtcggag 9540
   agggaaggtt gtaaggattt gtttagttat tattgttatg tttcgttatt gtggatttgt 9600
   ttgattttta ttatagattt ttttagtttt agttttttta ttagtaaaat atgtag 9656

<210> 231
<211> 6250
<212> DNA

<213> Artificial Sequence

<220>
<223> chemically treated genomic DNA (Homo sapiens)

<400> 231

tcgttttaaa aaagaaaaag aaaaagaaaa taaaaggatt atttgtagtt gattgtggtt 60
attatattat gtatttagat tttaatagat ttattttttg gatatgtttt tttttgttat 120
ttaagttgga gtgtagtggc gttattatgg tttattgtag gttggagttt ttaggtttaa 180
gtagtttttt taagtatttg ggattatagg tatgtgttat tatatttggt ggttaatttt 240
tgtatttttt gtagagatgg gtttttagta tgatgtttat gttggtttgg agttgttggg 300
tttaaaggat ttatttattt taatttttat aaatgttggg attatagata taagtcgtag 360
cgtatggtta agagtgattt tttttgtttt ttgatgtgaa gttttgtttt gttatttagg 420
ttggagtgta atggtacgat tttggtttat tgtaattttt gtttttcggg tttaagtaat 480
ttttcggttt tagtttttcg agtagttgga attataggta tttattatta tgttcggtta 540
atttttgtat tttttatttt tattttttga gacggagttt tattttgttt tttaggttgg 600
agtgtagtgg tatgatggcg gtttattgta attttcgttt tttgggttta agtgattttt 660
ttgttttagt tttttaagta gttgggatta tagatatgta ttattatgtt tggttaattt 720
ttgtattttt agttgagatg ggtttttatt attttggtta tgttggtttt aaatttttga 780
tttcgtgatt tgtttatttt tgttttttaa agtgttggga ttataggtgt gagttattat 840
gttcggttaa tttttgtatt tttagagatg gggttttatt atgtgggtta ggttggtttt 900
gaatttttga ttttaaggga attatttgtt tcggtttttt aaagtgttgg gattatagga 960
gtgagttatt gtatttagtt aatagtgata tttttatttt ttttaatttt ttattatttt 1020
tttttaattt agttttaaag ttattagatt gtagagagta aggtatgttt aagtatatta 1080
agggtggttt agtatgggat gttgggaagg ttggttggag agtgtttata taggagggta 1140
atttagtatg gagattgggt atatttggag aattaataaa ataaataaat atattgagaa 1200
taatggtagt taaatttttt attgtgagag aaaaatttat aaatatgtat ttagttaagt 1260
ttggaatggt ttgagtttgt gtgataaaaa taagtggaaa gtttgatgag gaaatgaatt 1320
atttatatat tttttttttt gagtgggagt ttcgttttgt tgtttaggtt agagtgtagt 1380
ggtttgattt cggtttattg tagtttttgt tttttgggtt taagtaattt tttgattttt 1440
ttgttttagt tttttaagta gttgggatta taggtgtatg ttattatgtt tgggtaattt 1500
ttatatattt tttgagatgg agttttgttg tgttgtttag gttggagtgt agtggcgtaa 1560
ttttagttta ttgtaatttt tattttttag atttaagtaa ttttttttgt tttattttat 1620
taagtagttg ggattatagg tgtttgttat tatgtttggt taattttttt gtatttttta 1680
gtagagatgg ggtttcgtta tgttggttag gatggttttg aatttttgat tttgggtgat 1740
tagttcgttt cggtttttta aaatgttggg attataggtt tgagttattg cgtttggttt 1800
aatttttgta tttttgtaga gagagggttt tattatgttg gttaggttgg ttttgaattt 1860
ttgattttag gtgattaatt tgttttagtg ttttaaagtg ttgggattat aggtgtgagt 1920
tatagtgttt ggttattttt ttaaaaaaat atattttttt ttttttgaga tggagttttt 1980
gttttttgtt ggttaggttg gagtgtaatg gcgtgatttt ggtttattgt aatttttatt 2040
ttttaggttt tagttatttt tttgttttag ttttcgagta gttaggatta tagggatgta 2100
ttattatatt tggttaattt tgtattttta gtagagatgg ggttttttta tgttggttag 2160
gttggttttt aatttttgat tttaggtaat tcgtttgttt tagtttttta aagtgttggg 2220
attataggcg tgggttatta tattaggttt taatatattt tttttttttt tttttttttt 2280
tttttgagat agagtttcgt tttgttattt aggttatagt gtagtggtgt gagtttagtt 2340
tattgtaagt ttcgtttttt aggtttacgt tatttttttg ttttagattt tcgagtagtt 2400
gggattatag gtgttcgtta ttacgttcgg ttaatttttt gtatttttag tagagatagg 2460
gttttatcgt gttagttagg atggtttcga ttttttgatt ttatgatttg tttgttttgg 2520
ttttttaaag tgttgggatt acgggtgtga gttattgcgt tcggttaata tatttttaaa 2580
atattttttat aaaggattta attgagttta ggaagtggag gttgtagtga attatgatta 2640
tattattgta ttttagtttg ggtgataaag taagatttta ttttaaaaaa ttttcgaaaa 2700
gtaaaaaaat aaaatatttt tatagaaaat agtgattaat tataaaggta aaaagaataa 2760
tttcgtagta gagaaatttg gatgatattt tgttattaat gtaataggag aaatgaaatt 2820
atgtattatt tgatagaatg taatgtaaag aatataaaga tataaatatt agataaattt 2880
aaattgaggg ttattttata aaataaatgt tttgtaattt ttataggtat taatgttgtt 2940
aaggtttaag gattaaggaa ttgttttata ttgaaggaga tgaaatttta ttaaatgtaa 3000
agtgtgattt tgaattggaa atttttgtta taaaggattt tatggtgtaa taggaaaaat 3060
ttgaatgggg tttggggatg gtattagtgt taattttttg gtataaatga ttgtattgtg 3120
attatgtaag agaatatata gattatattt gtttgtagaa aatatatatt aaattttttg 3180
atagtgatgg gtattatgtt agtaatttat ttttagatga tttaggaaaa gttttttgta 3240
ttatatttgt aatttttttg ttaaatttga gaaagtttta aattaaaaat tttttttttt 3300
agacgagttt ggttttgtcg tttaggttgg agtgtagtgg cgtgatttta gtttattgcg 3360
atttttattt ttttttgggt ttaagtaatt tttttgtttt agtttttcga gtagttggga 3420
ttataggtat gtattattat atttggttaa tttttgtagt tttagtagag acggggtttt 3480
attatgttgg ttagggtggt ttggaatttt tgatttcgtg attagtttgt tttggttttt 3540

taaagtgttg ggattatagg cgtgagttat ttcgtttggt ttttttgatt atattttatt 3600
aattttttta tttaatatat tttattgttc gtgatatagt ttttaggaga ttttgaaaat 3660
atgtatatat ttttattttt aaaagatttt agaaatttat tagtagttag ttacgttttt 3720
ataagtaatt agttatttgt ttttttgcga ttatatagta ttatgtttat aattttatta 3780
tggttagtta tattttttat gtttttgaaa tagggtttcg ttttgtcgtt taagttgaag 3840
tgtagtgagt ggcgcgatta tagtttattg tagtttttat ttttttagtt taagggattt 3900
ttttatttta gttttttaag aggttaggat tataggtttg taatataacg ttggttgatt 3960
tttgtatttt ttgtagagac ggggatgggt gtggtggttt agagtttttg ggtttaattg 4020
attttttcgt tttagttttt taaagtgttt cgtattcggt taatatattt tgttaataat 4080
ttaaaaattt tcggttttta tgtgggttat ttattaattt attaagtatt tattgtatat 4140
ttgttttttg ttttaggtgt ttaggatata tttgtgaata agttaagatt aggtttttat 4200
gtaatttatg tggaaggtaa atgagaaatt agaaacgtcg ggtcgtggtg agcgttatgt 4260
agaaagtgta tgggtgtgaa tagttgttta agattgttaa gatttgataa ttattttttg 4320
aagttttttg aagtagtgaa tttgaacgcg tttcgtaagt gtaaattaga agggaagatt 4380
tgttgaggtt tcgaaagagt tagtgtaagg gtttttgggg taggaatgag tcgttgtggt 4440
tggatttttg agggagtgga aggtttgtga agtcgaaaag tttaggcgtg ggagttttag 4500
atggttgttt ataaatgtcg tcgggtcgtt gggggtggag ggtttgaggg aaagcggggt 4560
tcggcgtagt tttagttcgg gtgtagggtt ttcggttagt attcgatatt ttagataggt 4620
ttttttttgg gtatttttcg tattaggttt ttttttttta gcggaggcgg gttatgatat 4680
ataatttatt tgcggttgtt gatcgtataa tgattcgacg tcggtagcga gggaagagta 4740
aggtttaaaa atcggcgttt attagggtag aatgtagttt acggaggatg gcggaagagg 4800
cggttgaggt cgtaggacgt gttattttaa ggtacggtcg tacgagtgtt acgtatgcgc 4860
gtgcgttggt tcgcgttaga tatagtgtta gtttttagat ggggcggggt cgggcgttcg 4920
cgtcggaaga gggttggttg ggcggggtcg ggaagcggta gtggcggtta cgcgcgcggg 4980
ggtgcgcgcg ggaacgatcg ggaaatatcg cgagggtcgg ggtgggttag gttgtgggga 5040
cgacgggttg cgacgatggt cgtagcggcg ggcggcggcg ggtcggggat agcggtaggc 5100
gttacgggtt cggggattgc ggcggtagtc gtaggtttag ttgtttatcg acggaaggat 5160
gggggttcgg ttattaagtt ttgggagagt tcggagacgg tgttttagtt ggattcggtg 5220
cgggtttggt tgggtaagta ttataagaag gtgggttcgc gcgttcgtgg gggtcgggag 5280
attacgcgat cggggtcgga ggcggtagtt ggtttcgcgg gtttcgggtc gttttttttt 5340
ttcgtacgtt acgagggata ataaaggtag gcgtcgggtt cgttacgttt tcgtcgttcg 5400
ttttgggcgt tcgcgatttc ggggtttgat tggggttcgg ggtttttttt tttgagttta 5460
cgtatttatg gtgaaagttt ttgttagttt tcgtgtgtta gagtaggtcg agtttagtta 5520
ttaagcgagt gtgatttttt tttttttgtt agttgtttta ggtgtgtgaa taatgatttt 5580
taaagatcga gtaagcgtgg taaaagtagt tttaagtttt gtttagagcg tatttaaagt 5640
ttcgaggggg tttgttattt gttgttgaat tgttaaacga ttgggatgat tgtattcgtt 5700
aattaataat aggtaatatg aattttagga tggatgagaa ttattaacga cggttttatt 5760
ttatcgtgtt tttggttttg tgtggcgtga gagttgatgg tcgtttgtac gtaaagtgtt 5820
ttaaagggag gtttttttta agaatttagt ttttattaga gtattttttt tgtgttattt 5880
ttattttttt cgtagtgttt attatatttt gaaattattt atttattgat tttttttatt 5940
tttggaacgg aagtttcgtc gagttagtag tttttaaagt tttgatttaa ttttttgttt 6000
ataataaatg tttttttttt ttttaatttt ttttttgtgt aaatatttag gaggattttt 6060
ttaaagggtg taatgaagta ggtagtcgaa aaggtttttt agtttttgaa gtttttttat 6120
tatttttttt agttttgtat ttttttttat tgagaattat agtattgaag taagggtttt 6180
gtttattgta gtgtattcgg gaacgtggtt gatatttatt gttttatttt tagtggttgt 6240
ttagtaaata 6250

<210> 232
<211> 6250
<212> DNA
<213> Artificial Sequence

<220>
<223> chemically treated genomic DNA (Homo sapiens)

<400> 232
   tatttattaa gtagttatta agggtaaggt aataagtatt agttacgttt tcggatatat 60
   tgtagtgaat aaagtttttg ttttagtgtt atgattttta ataggagaaa gtatagaatt 120
   aaagagggtg ataggaaaat tttagaggtt ggggagtttt ttcggttatt tgttttatta 180
   tattttttaa gggagttttt ttgaatattt atataaaagg gaagttggaa aaaaaaaaaa 240
   tatttgttat aaataaagga ttaagttaaa attttgaaaa ttattgattc ggcggaattt 300
   tcgttttaaa ggtaggagaa attagtaaat gaataatttt aaaatgtggt aaatattacg 360
   aagaaaataa aggtgatata agaaaagtgt tttaataaaa attgggtttt tggggaaggt 420
   ttttttttag aatattttac gtataagcga ttattaattt ttacgttata tagaattaga 480
   agtacgatga ggtagaatcg tcgttaatag tttttattta ttttagaatt tatattgttt 540
   gttattaatt aacgaatata attattttag tcgtttagta atttaataat aggtagtaaa 600
   ttttttcgag attttgaata cgttttaaat aaaatttgaa gttgttttta ttacgtttgt 660
   tcggttttta gggattattg tttatatatt taaggtagtt gataaaagag agagagttat 720
   attcgtttag tggttgagtt cgatttgttt tggtatacga gattgagtag gggtttttat 780
   tatgggtgcg taggtttaga ggagagagtt tcgaatttta gttaggtttc gggatcgcgg 840
   gcgtttagag cgggcggcgg gggcgtggcg ggttcggcgt ttgtttttgt tgtttttcgt 900
   ggcgtgcggg ggggaggggc ggttcgaggt tcgcgaggtt agttgtcgtt ttcggtttcg 960
   gtcgcgtggt ttttcggttt ttacgggcgc gcgaatttat ttttttgtag tgtttgttta 1020
   gttagattcg tatcgaattt agttgggata tcgttttcgg gtttttttaa aatttggtgg 1080
   tcgggttttt attttttcgt cgataaatag ttaggtttgc ggttgtcgtc gtaattttcg 1140
   agttcgtggc gtttatcgtt gttttcggtt cgtcgtcgtt cgtcgttgcg gttatcgtcg 1200
   tagttcgtcg tttttatagt ttggtttatt tcggttttcg cggtgttttt cggtcgtttt 1260
   cgcgcgtatt ttcgcgcgcg tagtcgttat tgtcgttttt cggtttcgtt tagttaattt 1320
   tttttcggcg cggacgttcg gtttcgtttt atttgggaat tggtattgta tttgacgcga 1380
   gttagcgtac gcgtatgcgt agtattcgtg cggtcgtgtt ttggaatagt acgttttgcg 1440
   gttttagtcg tttttttcgt tatttttcgt aggttgtatt ttgttttagt gagcgtcggt 1500
   ttttgagttt tgtttttttt tcgttatcga cgtcgaatta ttatgcggtt aataatcgta 1560
   ggtgggttat gtgttatagt tcgttttcgt tggagggaaa aggtttggtg cgagaaatgt 1620
   ttaagggaaa gtttgtttag ggtatcgagt gttggtcgag ggttttgtat tcggattggg 1680
   attgcgtcgg gtttcgtttt tttttaggtt ttttattttt agcggttcgg cggtatttgt 1740
   gaatagttat ttgggatttt tacgtttagg tttttcggtt ttatagattt tttatttttt 1800
   taggggttta gttatagcgg tttatttttg ttttaggggt ttttgtattg gttttttcga 1860
   gattttagta ggtttttttt tttgatttgt atttacgaga cgcgtttaga tttattgttt 1920
   taaaggattt tagaaagtag ttgttagatt ttaataattt tgaataattg tttatattta 1980
   tatatttttt gtatagcgtt tattacgatt cggcgttttt agttttttat ttatttttta 2040
   tataaattat atgagaattt aattttaatt tgtttatagg tgtattttga atatttgaga 2100
   taaggaatag atgtatagta aatatttgat gagttaatga atggtttata tagaagtcga 2160
   aaatttttaa attattaata aggtatattg gtcgggtgcg gagtattttg ggaggttgag 2220
   gcgggaggat taattgagtt taggagtttt agattattat atttattttc gtttttataa 2280
   aaaatataaa aattagttag cgttgtgttg taagtttgtg gttttagttt tttggagggt 2340
   tgaggtggga ggattttttg agttggggag atggaggttg tagtgagtta taatcgcgtt 2400
   atttattata ttttagtttg ggcgatagag cgaaattttg ttttagaaat ataaaaagta 2460
   tgattggtta taatggagtt gtgaatataa tgttatatga tcgtagagaa gtaagtaatt 2520
   aattgtttgt gagaacgtga ttaattgttg gtaagttttt gagatttttt aaaaatgagg 2580
   gtgtgtatat gtttttagga ttttttgagg gttgtgttac ggatagtaaa atatattaaa 2640
   taaaaaagtt aatagaatgt gattaagagg gttaggcgag atagtttacg tttgtaattt 2700
   taatattttg ggaggttaag gtagattgat tacgaggtta ggagttttag attattttgg 2760
   ttaatatggt gaaatttcgt ttttattaaa attataaaaa ttggttaggt gtggtggtgt 2820
   atgtttgtaa ttttagttat tcgggaggtt gaggtaggag aattgtttga atttaggagg 2880
   aggtggagat cgtagtgagt tgagattacg ttattgtatt ttagtttagg cgatagagtt 2940
   aaattcgttt gaaaaaaaaa gtttttaatt tgaaattttt ttagatttaa tagaaaagtt 3000
   gtaaatataa tataaagaat tttttttgaa ttatttaaga gtaagttgtt gatataatat 3060
   ttattattat taaaaagttt aatgtgtatt ttttatagat aagtatagtt tatatatttt 3120
   tttatataat tataatataa ttatttatat taggaaatta atattgatgt tatttttaga 3180
   ttttatttag gtttttttta ttgtattatg aagtttttta tagtaaaagt ttttagttta 3240
   aaattatatt ttgtatttaa taaagtttta ttttttttaa tatggaatag ttttttagtt 3300
   tttaaatttt aataatattg atatttatga agattataag gtatttattt tgtagaatga 3360
   tttttaattt aagtttgttt gatgtttgta tttttgtgtt ttttgtattg tattttatta 3420
   ggtggtgtat aattttattt tttttgttat attgatgata gagtattatt taggtttttt 3480
   tattgcgaag ttattttttt tgtttttgta attaattatt attttttatg gaggtatttt 3540
   gtttttttgt ttttcggggg ttttttgaga taaagttttg ttttgttatt taagttggag 3600
   tgtagtggtg tgattatggt ttattgtaat ttttattttt taggtttaat tgattttttt 3660
   gtggaggtat tttgaaagta tattggtcgg gcgtagtggt ttatattcgt aattttaata 3720
   ttttgggagg ttaaggtagg tagattatga ggttaggaga tcgagattat tttggttaat 3780
   acggtgaaat tttgttttta ttaaaaatat aaaaaattag tcgggcgtgg tggcgggtat 3840
   ttgtagtttt agttattcgg gagtttgagg taggagaatg gcgtgaattt gggaggcgga 3900
   gtttgtaatg agttgagttt atattattgt attgtagttt gggtgataga gcgagatttt 3960
   gttttaaaaa aaaaaaaaaa aaaaaaaaga aagtatatta aggtttggtg tggtggttta 4020
   cgtttgtaat tttagtattt tgggaggttg aggtaggcgg attatttgag gttaggagtt 4080
   aaagattagt ttgattaata tggagaaatt ttatttttat taaaaatata aaattaatta 4140
   ggtgtggtgg tgtattttta taattttagt tattcggagg ttgaggtagg agaatggttg 4200
   aaatttggaa ggtggaggtt gtagtgagtt aagattacgt tattgtattt tagtttggtt 4260
   aataaagagt aaaaatttta ttttaaaaga aagaaagtat atttttttaa aaaagtggtt 4320
   aggtattgtg gtttatattt gtaattttag tattttgaga tattgaggta ggttgattat 4380
   ttgaggttag gagtttaaga ttagtttgat taatatggtg aaattttttt tttataaaaa 4440
   tataaaaatt aggttaagcg tagtggttta ggtttgtaat tttagtattt tgggaggtcg 4500
   aggcgggttg attatttaag attaggagtt taagattatt ttggttaata tggcgaaatt 4560
   ttatttttat taaaaaatat aaaaaaatta gttaggtatg gtgataggta tttgtaattt 4620
   tagttatttg gtagggtgag gtaggaagag ttgtttgaat ttgggaggtg gaggttgtag 4680
   tgagttgaga ttgcgttatt gtattttagt ttgggtaata tagtaagatt ttattttaaa 4740
   aaatatataa aaattattta ggtatggtgg tatgtatttg taattttagt tatttgggag 4800
   gttgagatag gagaattaaa gaattgtttg aatttaggag gtaaaggttg tagtaagtcg 4860
   agattaggtt attgtatttt agtttgggta atagagcgag atttttattt aaaaaaaaaa 4920
   gtatataaat aatttatttt tttattaaat tttttattta tttttgttat ataaatttag 4980
   attattttaa atttggttaa gtatatattt gtaaattttt tttttatagt gagagatttg 5040
   gttgttatta tttttaatat gtttatttgt tttgttagtt ttttaaatat atttagtttt 5100
   tatgttgagt tattttttta tgtaggtatt ttttaattaa tttttttaat attttatgtt 5160
   aggttatttt taatatgttt aaatatgttt tgttttttgt aatttaatga ttttaggatt 5220
   gaattgagaa gggatgatag gggattagga aagataaaaa tgttattgtt ggttgggtgt 5280
   agtggtttat ttttgtaatt ttagtatttt gggaggtcga ggtaggtggt ttttttgagg 5340
   ttaggagttt aagattagtt tggtttatat ggtgaaattt tatttttaaa aatataaaaa 5400
   ttggtcgggt atggtggttt atatttgtaa ttttaatatt ttgggaagta gaggtaggta 5460
   gattacgagg ttaggagttt gagattagta tggttaaaat ggtgaaaatt tattttaatt 5520
   aaaaatataa aaattagtta ggtatagtgg tatatgttta taattttagt tatttgggag 5580
   gttgaggtag gagaattatt tgaatttagg agacggaggt tgtagtgaat cgttattatg 5640
   ttattgtatt ttagtttggg agatagagtg agatttcgtt ttaaaaaata gaaataaaaa 5700
   atataaaaat tagtcgggta tggtggtggg tgtttataat tttagttatt cgggaagttg 5760
   aggtcggaga attgtttgaa ttcgggaggt aaaggttgta gtgagttaag atcgtgttat 5820
   tgtattttag tttgggtgat agagtaagat tttatattaa aaaataaaaa aagttatttt 5880
   tggttatgcg ttgcggttta tgtttgtaat tttagtattt atggaggttg agatgggtgg 5940
   attttttgag tttagtagtt ttagattaat atgggtatta tattgaaaat ttatttttat 6000
   aaaaaatata aaaattagtt attaggtgtg gtggtatatg tttgtggttt tagatatttg 6060
   ggaggattgt ttgagtttgg gagttttagt ttgtagtgag ttatgatagc gttattgtat 6120
   tttagtttgg gtaataaagg gagatatatt taaaaaataa atttattgaa gtttaaatat 6180
   ataatatggt ggttataatt agttgtaggt agttttttta tttttttttt tttttttttt 6240
   tttgaaacgg 6250

<210> 233
<211> 7171
<212> DNA
<213> Artificial Sequence

<220>
<223> chemically treated genomic DNA (Homo sapiens)

<400> 233

aaagaaaata ttattagtcg gtgcggtggt ttacgtttgt aattttagta ttttgggagg 60
ttaaggtagg tggattacga ggttaagaga gtgagattat tttggttaat atggtaaaat 120
tttattttta ttaaaaatat aaaaattagt tgggcgtggt agtatgtgtt tatagtttta 180
gttatttcgg aggttgagat aggagaatta tttgaattta ggaggcggag gttgtagtga 240
gtcgagatgg cgttacggta ttttagtttg gagatagagt gagattttgt tttaaaaaaa 300
aaaaaaaaag ttagttatat aaaaataatt ttaagtttag gtttagtggt ttatgtttgt 360
aattttagta ttttgggaag tcgaggtgag aggattattt gaggttagga gtttatgatt 420
agtttggtag tatggtaaaa ttttgttttt ataatgtaaa aataagtcgg tgtggtggcg 480
tgcgtttata atttagttta ttcgggaggt tgaggtagga gaattgtttg aatttgggag 540
gtggtggttg tagtgagtcg agtttgtgtt attgtatttt agtttaggtg agagagtaag 600
attttgtttt aaaaaaaaat aaaaagtaaa aaagtaaaaa agaaaaaaaa attaaaatat 660
tttaggaggt agaaattaga tgatagggta tttgatagta tggaattgga ggtagaaagg 720
tatatatttt attcgaaaaa taaaaggaag tagaaaataa tgtggaaatt tgaaagtgtt 780
aaatgaaggt ggggttttgt ttgtattgaa tttatgaagg attgatttag tggagtagag 840
gaattttaag gtgttatgga aggatattta tggaaggttt tttaggaggt ttaaatgagt 900
gggattatag gtttaagtgg agaaggttat tttagggaaa atatggaagg ttatatattg 960
tagtaggaat taaagaatgt tagtgggtga tatgtaaata tgtaagaatg gtgagagaga 1020
gggggttaga ttatgtaagg taaagaaagg taaaagaata gttaggattt gttggagtgg 1080
aagaggaagg gtgaaggaat tggggagatg gggtgggtat atggggatgg tagaaattta 1140
aaatgattta ttgtatatga aggtttgaag tttcggtttt ttatttataa aagttataag 1200
agaagtattt gagagtaaaa gggatgaatt ttaagtagta aattattttt gttatatttt 1260
tttttttggt ttagattttt tattttgtaa attatggagt tttttttttt ttataatata 1320
gttgttggtt gttttttgtt ggttaatttt ggttttggtt ttttagatat ttaaatatga 1380
gaatttgtgt aaattgggat ttggtaggaa ggagaattat gagtttatga gattttataa 1440
atttatttaa tatatagttg attatattta taaggttttg tagagaagat ataaataaat 1500

aaaatatata gtttaatttt ttaagaagtt tatatattag tttaaagatt aaaatatgta 1560
tatataatta ttgtaaaaaa tatttattta tgtaaaaaaa ttttaaaaat tgatttttta 1620
tttgtgttta atgtgtttat atgttatagg ggaagaaaga ttttatataa taagttgaga 1680
gtataggatg tggtaggtaa taagtggtta gagaaaattt tataggagaa ttaatattta 1740
ggtggagaat gagtagaaag tgtggaagag aaagattatt ttaggtagag aatattatga 1800
gtaaggttaa ataattgtta ggaagaatta aaagaatgtt agtggataga atgtagtgaa 1860
tatggaagag tggtgagaga aaaggggtta tattttgtaa tatagaaagt agttatagaa 1920
gggtttaagg tttcggattg tagtgtggat gttttagtgt agttttttat atattatttc 1980
gtatggtgtt tataaatatt attatttttt agatgaaggt tttagtttag tttgtattta 2040
tttttgagaa aagtgagtat atttgagata tatttagaag gtaaaataaa tggaatttgt 2100
ttttatttaa ttttaatttt attttatttg agatagagtt ttgttgtatc gtttaagttg 2160
gagtgtagtg gtacgatttt ggtttattgt aatttttgtt ttttgggttt aagtgatttt 2220
ttttatttta gttttttaag tagttgggat tataggcgtt cgttaattat atttagttaa 2280
tttttgtatt tttagtagag atgggatttt attatattgg ttaggttggt tttgaatttt 2340
tgatttcgtg attcgtttgt tttagttttt taaagtgttg ggattatagg cgtgagttat 2400
tatatttatt tttgttttta ttttattttt atgttagtat attaagggag aagtttttgt 2460
atgtaattta gtgaatggtg gtggtggtag attgataggt agttggagtg attaagagta 2520
ttaaagagga ggggtttaaa gaattttgga aagtgttaat ggtgagggat agggaggtaa 2580
tgtgagggtt atgttggaaa taatagtttg agtagtggta tgaaggtggg aaattttaag 2640
gtataaatgt gtagaaagtt ttatgtatta ttttagagtt ttttgttttt tgggattttt 2700
tttggataaa aggttgtgta agaaatataa aaagaatatt tttggttagg tatagtggtt 2760
tatatttgta attttattat tttgggaggt tgaggtaggc ggattgtttg agtttaggag 2820
tttgagatta gtttgggtaa tatggcgaaa ttttagaacg tcgtttttat taaaaatata 2880
aaaagttagt tgggcgtggt ggtgtatgtt tgtagtttta gttattgagg agggaggatt 2940
gtttgggttt gggagtttga ggttgtagtg aattgagatc gtattaatgt attttagttt 3000
gggcgataga gcgggatttt gttataaaaa taatttttta aaataaataa taaaattttt 3060
aataagtagt ttagattttt tttataatta gaaattaggg gataggaaaa atggggtgag 3120
atgagtatag gagggttaaa ggttaaagat gagattaatt tttttgtttg gttaatatat 3180
atagataatt taaaatagta tttggtatat aataagtatt taataaaaag tgagaattat 3240
ttttttatat tttggtttta attggatttg taatgatgtt ttaatagatt attttttttt 3300
agttttgtat attttaagga aatttagatg agagaatttt gttttttggg ttttcggtgt 3360
aattagattt ataaggtgtg atttttggtt atgaaattta gaggaaattt taagtttgat 3420
taatattgta aaatagttat ttatattttt aaaatgtaat tttttaatta aatttttttt 3480
tatgttttta aaagaatgag atatgtgtta aagttttttt aggaagtttg aagttataaa 3540
attttgaaaa tgttttatag tttagtagtt aatatagtcg tgtataatta aattttttaa 3600
gattattttt atttttttaa atttaggggt taagattttt tggcggtttt ttttttggag 3660
agagaaagtg tacgaaagaa acgtgtgaga attggggtta tttttatgtg ttgtgaaagg 3720
ggttaaaaaa gtcgacgttt gtaattttag tattttggga ggtcgaggcg ggtagataat 3780
ttgaggttag gagtttgaga ttagtttggt taatatggtg aatttgtttt tattaaaaat 3840
ataaaaatta gttaggtatg gtgacgtatg tttgtggttt tagttattcg ggaagttaag 3900
gtaggagaat tgtttgaatt cgggaagcgg aggttgcggt gagtcgagat cgcgttattg 3960
tattttagtt tgagtaatag agcgagattt ttttttaata ataagaagaa gaaagaaaaa 4020
aaaaaaaaat tcgttacgaa taggattcga atttgtgcgg ggaaatttta ttggatttcg 4080
agtttaacgt tttaattatt cggttatcgt gatttttaaa atcgcgtttt atcgcgcgtt 4140
tatagaaggg gttcgtattg taattcggga atgcgtgcgg gaatcggaag tagggtatcg 4200
gtttcgatat atatatattt gaggattttt tatttttcgg ttttttattt tttatttacg 4260
ttatttaaag aaaaagagtc ggatggattg ggtgatttag ggagggaggg ttttttgttt 4320
agcgaagagt cggggtttcg agtaggagtt tatatttatt taggaggcga tagcgaggtt 4380
tgcggaggaa gtagttgagg gaaggtattg ggtcgttagg gtggcgatta ggggatattt 4440
gggttaataa gtgaggggag tgttgggtgg ataaggcgcg aggagtgagg gagattttga 4500
aaagtttaaa tggggagttg ttgtgaatgg atggaaggaa ggaatttttt ggggaaaggg 4560
gtttggggtt ttgggggacg aggggtttcg tcgggttagg aatgcgtaag gtgaggatta 4620
ttcgggttta cgggttgggg ttgttttagc gattggcggt cgtttggagt gggatagcga 4680
ggtgatattt tcgagttgag gtaaaaagag ggaaagggtt tcgcgtttgg taagagcgat 4740
tttaggagaa tggcgttttt tttaggaagc gttttttttt tcggaggttt tcgttttcgt 4800
tgtcggggag gtcgttaggg tcggttcgcg tttggagttt cgtcgtcgcg ttcggtttgt 4860
cggtaggggg cgtagcgcgg atcggggtcg gtttaggcgg cgggaggcgg cggtcgcggc 4920
ggcgggaggc ggcgggaggc gggcggagga ggaggcggag gaggcgggag ttgagttgag 4980
tggggcgggc ggcggcgggg ttcgagtcgg agaagatggc ggtgcggaag aaggacggcg 5040
gttttaacgt gaagtattac gaggtcgcgg atatcgtgat ttagttcgat aacgtgcggt 5100
tgtggttcgg taagaattat aagaaggtac gcgggtcggg tcgggtcgcg cggggatcgg 5160
gggcgggata aagggatcgc ggttttgtag tttcggattt ttgtttattt tttaggcgtt 5220
ttagtttttt tttcgcggtt ttttaagtag tttttcgcgc gtttttttgc gttttcgcgt 5280
ttttttttta gttttttttt tttcgtattt ttagtttttg agtttagttt tcgtttttat 5340
tgttgcgttt tttgtttttt gttttttttt attgttcggc gggtttcggt gttattagtt 5400
tatttttttt ttagttatcg tttttttggt ttttttggga gaggcggtcg gtcgggtgtg 5460

gatcgggtgt ttaatgggtt ttttcgtaat tcggagcgtg ttcgttagag ttttttacgg 5520
agttaggtat tagcggggtt gaggtttttt cggtgttttg tgtatgttta gagagagaga 5580
atggaaagta ggagggtaaa tagttataga gtaagtcgtt ggttgtttat tagtttgaaa 5640
aaggttttta tttagggatt atttgttttc gtcgtttatt ttttattttt atttaagggg 5700
taatagaatt ttttagggag tcggggtata ggagtttttg ggtagcggtt tttttcgaaa 5760
ttttttttcg attttataat gaagttattt tgtaatttag ttttgtagta ttttgggttt 5820
ggggtcgaga ggttagggtg agttttttat ttttagtttt ttatatttaa attttataat 5880
tggagattat aaggtggtga ataaaaggtt taagaatttt tatttgtttt ttttattaaa 5940
tttttttttg gtgtcgtttt tgtttttagg tttggttttt tttggaaatg gtttgttttt 6000
ttttttttta ttatttttag agagggattt tttttatggt ttttttttag ttttttagtt 6060
ttttttgtcg gttttgtagt tcgttagaag tgtgtttagt ggtttaggag aattttttat 6120
tgatgttggt tgtgagtttg gttttgtttt tggggttttt ttgggtgtga gaattttgat 6180
aaatagttgt tatttttatg gtatgttttt acgtttgtga gaggttttta tttttaggtt 6240
tttatttttg attttttgta gggagattag gttaggggtt tagttttcgg ttttggggga 6300
attgatagtt gtgatttggt ttagttttta tgtttttagt tttttgttat ttattttttt 6360
ttttatagat ttattttttt ttatttttta ttgagaggta ttgttttttt tgtgatgagt 6420
ggggagaaaa cgggttaaaa agtaaaaaga aagttttttg gtttaggttt aaattagaat 6480
ggaaggttag gatgttatta ggtagtagag tgtggaaata tagaagtagg gttagggtta 6540
tttgttttag atatagtttt ttcggatagg gtagatgttg ttttgttttt ttttattagg 6600
ttgggtagtg gatggttttt tttgtttagg agggtttatg aagatattta tatttagttt 6660
ttagaagtaa aatgtataag tttatttagt taatttgttt ttttttattt ttttgtattt 6720
ttttttgtta ggttatatta tagggggaat tatggtttta tagagttttt ttaagaaatg 6780
ttttttttag agttttagag gtattgtttt tatgatttga attttataaa tttttagaat 6840
tttataattt ttagtgttgt tggttaaaga tgaggtgtta agataatagg gttttgtaat 6900
tttaatttta gagtataatt gaggaagagt agtaagtttt tttgtttttt ttaatattgg 6960
ttatttgggt tggtggtagt agaattttag ttttttggtt ggggtgttta gggttttttt 7020
tttgttattt tttttataga gattaaatgg ggagaagggt attatttttt ttgttttata 7080
tgtttttaga gtttttgggg gagaaatatg ggagatagtg gataatttat ttgagattta 7140
tttttttttt tttttttttt ttttttttag t 7171

<210> 234
<211> 7171
<212> DNA
<213> Artificial Sequence

<220>
<223> chemically treated genomic DNA (Homo sapiens)

<400> 234

attaaggaaa aagagggaaa ggaaagaaaa atagatttta gataaattgt ttattatttt 60
ttatattttt tttttagggg ttttggaagt atgtggagta aagaaaatag tgtttttttt 120
tttatttagt ttttataaag aaaatggtaa aagggaggtt ttggatattt taattaggaa 180
gttgaaattt tgttgttatt agtttaggtg gttagtgttg gggggaatag ggaagtttat 240
tatttttttt tagttatatt ttgaaattgg agttgtagga ttttattatt ttggtatttt 300
atttttaatt aatagtattg agggttgtaa aattttggag gtttataaag tttaggttat 360
ggaagtaata tttttggggt tttaagagag atatttttta ggggaatttt gtaaagttat 420
gatttttttt ataatgtagt ttaataagaa aaggtataag gaagtgaagg agggtaagtt 480
gattgagtga gtttgtgtat tttgtttttg aagattgggt ataaatgttt ttataggttt 540
ttttagatag gaagggttat ttattattta gtttgatgga gaagagtaag ataatattta 600
ttttgttcga ggaaattgta tttgagataa gtaattttgg ttttattttt atgtttttat 660
attttgttat ttagtagtat tttagttttt tattttaatt tgaatttgag ttaggagatt 720
ttttttttat tttttgattc gttttttttt tatttattat aaagaagata gtgtttttta 780
gtgagaggtg aaaagagata gatttgtgaa gggaaaggta aatgataaag agttaagagt 840
atggaagttg aattaggtta taattgttaa tttttttaag gtcggaggtt aagtttttag 900
tttgattttt ttgtaaaagg ttaaaggtag aagtttggga ataagagttt tttataggcg 960
tagagatatg ttatagaaat agtagttatt tgttaggatt tttatattta gagagatttt 1020
aagggtaggg ttaggtttat aattagtatt agtaggaagt ttttttaagt tattaagtat 1080
atttttgacg agttgtaaag tcggtaggaa gaattgggga gttgaaagaa agttataaag 1140
agaatttttt tttgagggta gtgagaaaaa gagaggtaga ttatttttag agaaaattag 1200
gtttagagat aaagacgata ttaggaagag gtttggtaga aaagataggt aaggattttt 1260
gaattttttg tttattattt tatgattttt agttatgggg tttgggtgtg aggggttaga 1320
gataggaagt ttattttaat ttttcgattt taaatttagg gtattgtaaa gttaggttgt 1380
aaggtgattt tattgtaaaa tcgaggaaag gtttcggaag gggtcgttat ttaggaattt 1440
ttatatttcg gttttttgag agattttgtt gttttttaga tgaaggtggg ggatgagcgg 1500
cggagatagg tgatttttgg gtggagattt tttttaggtt gatgagtagt taacggtttg 1560

ttttgtgatt gtttattttt ttgtttttta tttttttttt ttgaatatat ataaagtatc 1620
ggagaggttt tagtttcgtt ggtatttggt ttcgtgggga gttttggcgg gtacgtttcg 1680
ggttgcgggg gagtttattg ggtattcggt ttatattcgg tcggtcgttt tttttagaaa 1740
agttaggagg gcgatggttg aggggaaggt gggttaatgg tatcggggtt cgtcgggtag 1800
tagagaaaaa taaggggtag agggcgtagt agtgggggcg ggggttgggt ttagaggttg 1860
ggggtgcggg gagagggagg ttgggggagg ggcgcgggag cgtaggaggg cgcgcggggg 1920
gttgtttggg aggtcgcggg ggaggggttg gggcgtttgg agggtaggta ggatttcggg 1980
gttgtagggt cgcggttttt ttgtttcgtt ttcggttttc gcgcggttcg gttcggttcg 2040
cgtatttttt tgtagttttt gtcgagttat agtcgtacgt tgtcgaattg ggttacggtg 2100
ttcgcggttt cgtagtattt tacgttgggg tcgtcgtttt tttttcgtat cgttattttt 2160
ttcggttcgg gtttcgtcgt cgttcgtttt atttagttta gttttcgttt ttttcgtttt 2220
ttttttcgtt cgtttttcgt cgtttttcgt cgtcgcggtc gtcgtttttc gtcgtttggg 2280
tcggtttcgg ttcgcgttgc gttttttgtc ggtaggtcgg gcgcggcggc ggagttttag 2340
gcgcgggtcg atttggcgg tttttttcggt agcggaggcg ggagttttcg gggagaagga 2400
cgttttttgg agggggcgtt atttttttga gatcgttttt attagacgcg aagttttttt 2460
tttttttttg ttttagttcg ggagtgttat ttcgttattt tattttaggc ggtcgttagt 2520
cgttggagta gttttaattc gtagattcga gtggttttta ttttgcgtat ttttaattcg 2580
acgaagtttt tcgtttttta aaattttaaa tttttttttt taagaagttt ttttttttta 2640
tttatttata gtagtttttt atttaaattt tttagagttt tttttatttt tcgcgttttg 2700
tttatttagt atttttttta tttgttaatt taaatgtttt ttagtcgtta ttttggcggt 2760
ttagtgtttt tttttaattg tttttttcgt aggtttcgtt gtcgtttttt aagtaggtgt 2820
gagtttttat tcgagatttc gatttttcgt taggtaggag attttttttt tttgggttat 2880
ttagtttatt cgattttttt tttttgggtg gcgtagatag gaggtaagga atcgaggggt 2940
ggggagtttt taggtgtgtg tgtgtcgggg tcggtatttt gttttcggtt ttcgtacgta 3000
ttttcggatt gtagtgcgga ttttttttgt aagcgcgcga taaagcgcgg ttttggaagt 3060
tacggtggtc gagtggttaa ggcgttggat tcgaaattta atggggtttt ttcgtatagg 3120
ttcgaatttt gttcgtgacg ggtttttttt tttttttttt ttttttttta ttattaaaag 3180
ggagtttcgt tttgttgttt aggttggagt gtagtggcgc gatttcggtt tatcgtaatt 3240
ttcgtttttc gggtttaagt aatttttttg ttttagtttt tcgagtagtt gggattatag 3300
gtatgcgtta ttatatttgg ttaatttttg tatttttagt agagataggt ttattatatt 3360
ggttaggttg gttttaaatt tttgatttta ggttatttgt tcgtttcggt tttttaaaat 3420
gttgggatta taggcgtcga tttttttaat tttttttata gtatataaag gtaattttag 3480
tttttatacg tttttttcgt gtattttttt tttttaaaag ggaagtcgtt aaaaggtttt 3540
aatttttgga tttgggagga tgggagtgat tttgggaagt ttaattatgt acgattgtat 3600
tggttgttag attgtgaggt atttttagaa ttttgtaatt ttaggttttt tgggagagtt 3660
ttggtatatg ttttattttt ttgagggtat agggaaggat ttggttgaag ggttatattt 3720
tgggaatgtg agtggttgtt ttgtaatatt agttaaattt agagtttttt ttaaatttta 3780
tgattagaag ttatattttg taggtttgat tatatcgaag atttaggaaa taggattttt 3840
ttatttggat ttttttgagg tgtatagggt tagaagggag tagtttattg aaatattatt 3900
gtagatttag ttagagttaa ggtatgggaa gataattttt attttttatt gagtgtttat 3960
tatgtgttag atattgtttt aaattattta tgtgtattaa ttaagtaagg aaattaattt 4020
tatttttaat ttttaatttt tttatattta ttttatttta ttttttttat tttttaattt 4080
ttgattgtgg ggaggattta ggttgtttgt tgaggatttt gttgtttgtt ttggggggtt 4140
gtttttgtga tagggtttcg ttttgtcgtt taggttggag tgtattggtg cgattttagt 4200
ttattgtaat tttaaatttt taggtttaag taattttttt tttttagtag ttgggattat 4260
aggtatgtat tattacgttt agttaatttt ttgtattttt agtagagacg gcgttttggg 4320
atttcgttat gttgtttaga ttggttttaa atttttggat ttaagtaatt cgtttgtttt 4380
agttttttaa agtggtggga ttataggtgt gagttattgt gtttggttaa ggatgttttt 4440
tttgtatttt ttgtatagtt ttttgtttaa aaggaatttt aaagggtagg gggttttagg 4500
gtaatatata gagtttttta tatatttata ttttagagtt ttttattttt atattattgt 4560
ttaggttgtt atttttagta tgatttttat attgtttttt tgttttttat tattaatatt 4620
ttttaaaatt ttttaggttt tttttttttg atgtttttga ttattttagt tgtttgttag 4680
tttattatta ttattattta ttaaattgta tgtaaggatt ttttttttga tatgttaata 4740
taaaaataaa ataaaaatag gggtgggtgt ggtggtttac gtttgtaatt ttagtatttt 4800
gggaggttga ggtaggcgga ttacgaggtt aggagtttaa gattagtttg gttaatgtag 4860
tgaaatttta tttttattaa aaatgtaaaa attagttggg tgtggttggc gggcgtttgt 4920
aattttagtt atttgggaag ttgaggtagg agaaattatt tgaatttaag aggtagaggt 4980
tgtagtgagt taagatcgtg ttattgtatt ttagtttggg cgatgtagta agattttgtt 5040
ttaaataaaa taaaattaaa attaaataga aataggtttt atttatttta ttttttaaat 5100
atgttttaaa tatgtttatt ttttttaggg gtgagtgtag gttgagttag agtttttatt 5160
tggaaggtag tggtatttgt gagtattata cgaggtaata tatagaaagt tgtattaaag 5220
tatttatatt gtagttcggg attttaaatt tttttatggt tgttttttgt gttgtaggat 5280
atgatttttt tttttttatt atttttttat atttattata ttttgtttat tggtattttt 5340
ttagtttttt ttggtagtta tttagttttg tttatggtgt tttttgtttg gaataatttt 5400
ttttttttat attttttatt tattttttat ttaaatgttg gtttttttgt ggagtttttt 5460
ttgattattt gttgtttatt atattttatg tttttaattt attatgtgaa gttttttttt 5520

ttttgtggta tataaatata ttaaatatag ataaggagtt agtttttaaa atttttttgt 5580
ataaatgggt gttttttata gtagttatgt gtatatgttt taatttttag attggtatat 5640
aagttttttg agagttaggg ttgtgtattt tatttatttg tatttttttt ataaggtttt 5700
atagatgtga ttaattatgt gttgaatgaa tttataaaat tttataggtt tatgattttt 5760
ttttttatta aattttaatt tgtataggtt tttatattta aatatttgga gagttaagat 5820
tagaattgat tagtagaggg taattaataa ttatgttgtg aagggaagaa ggttttatgg 5880
tttataaaat ggaggatttg gattaagaag ggaaatatgg tagaggtagt ttgttatttg 5940
aagtttattt tttttatttt taaatgtttt ttttgtaatt tttgtgggtg agaggtcgag 6000
gttttaaatt tttatatata atagattatt ttaaattttt attattttta tgtatttatt 6060
ttattttttt aattttttta tttttttttt tttattttaa taaattttaa ttattttttt 6120
atttttttt atttgtatg atttggtttt tttttttta ttattttat atatttatat 6180
attatttatt ggtattttt agttttgtt atagtatgtg gtttttata tttttttga 6240
agtaattttt tttatttaag tttgtagttt tatttattta aattttttaa ggaatttttt 6300
ataaatattt ttttataata ttttaaaatt tttttatttt attggattaa ttttttatga 6360
atttagtata aataaaattt tatttttatt tgatattttt aaatttttat attgtttttt 6420
attttttttt atttttcgaa taaaatgtat atttttttgt ttttagtttt atattattag 6480
atattttgtt atttggtttt tgttttttga agtgttttgg tttttttttt ttttttattt 6540
ttttattttt tatttttttt tgagatagag ttttgttttt ttatttaggt tggagtgtag 6600
tggtataagt tcggtttatt gtaattatta ttttttaggt ttaagtaatt tttttgtttt 6660
agtttttcga gtagttagga ttataggcgt acgttattat atcgatttat ttttgtattg 6720
tagagatagg attttattat gttgttaggt tggttatgaa tttttgattt taagtgattt 6780
ttttatttcg gttttttaaa gtgttgggat tataggtatg agttattggg tttggattta 6840
aaattatttt tatgtaatta attttttttt ttttttttga gatagagttt tattttgttt 6900
ttaggttgga gtgtcgtggc gttatttcgg tttattgtaa ttttcgtttt ttggatttaa 6960
gtgatttttt tgttttagtt ttcggagtag ttgggattat aggtatatgt tattacgttt 7020
agttaatttt tgtattttta gtagagatga ggttttatta tgttggttag gatggtttta 7080
tttttttaat ttcgtgattt atttgttttg gttttttaaa gtgttgggat tataggcgtg 7140
agttatcgta tcggttggta atgttttttt t 7171

<210> 235
<211> 5938
<212> DNA
<213> Artificial Sequence

<220>
<223> chemically treated genomic DNA (Homo sapiens)

<400> 235

tagaataagt agggaagtat tttttttttt ttttttttta tttttattgt tgtaatcgga 60
gttattttat atttgatgta tattgtatat ggtatagaat atttttttat atatgttatt 120
ttatttaatt ttaaaaggta ggtattttta ttagtatttt attttatgga tatggaaatt 180
gagatttaga gaggttgttt tgttaaggtt atttggttag taagagatag agatatgatt 240
tgaatttagg tttttttttt taatttttgt gtagggagaa tattttggtg ttggatattt 300
tttttgaagt ttttaattat gagattattg aatagaagaa ggtttatgag attgtagggt 360
ttttgggtga gttgttgatg atatttgttt tttttttatg ttatgggtat ggcgtggttt 420
tttattattt aaaagtaggg tgtttatttt tgttttatga gggtttttta ttttagaggt 480
ttttttttaa attttgttgt tttggtaagt ggtgagggaa gggaattgag ttttttttta 540
gggtaaagtg gaaaagagaa aagataggtt tgtgggtatg agggaggggt aggtatggaa 600
gtggagatta ttttatatgt ttttaaatta agagggttgt ggtattttgt aaacgattat 660
tttttttgaa aaatatttgt ttgttttaga aaggtagaaa aacgtagaaa agtatagaga 720
agaaaataaa aattttattt gaatttggtt ttgcgatttt tttgtgcgaa ggttattaat 780
ttttttgaga atataatttg tttgttttag ttttagtaaa ttttgttgtt tttacgatgt 840
tttgatttta ttgatttttt tggcgtttgt tttcgtaggt gatatcgggg gttagatggg 900
gttgtttatc ggggttagta tttttacggt gttggagttt tttgattacg tttacgaggt 960
aagcgggggc gaggttcggt acggggttac gtgggggcgg ggtttagttc gtttatttgt 1020
ttcgttttcg ttttaggtta ttaagtataa gttgtgtcga cgaggaaaat gttagaagga 1080
ggttaaaagg agtagtgcgg ataagggcgt ggtttttagt ttggacgacg ttaaaagata 1140
cgtgagggag cgagcgaggg cgttttttag ttcgtttgtg tttttatcgt tttaggaatt 1200
tcgtttattt tcgtttattc gtttttgtta ttattttttt ttttttgttt ttcgaatgtt 1260
ttagtattat ttgaagtttt ttcgagggat aattcgtttt tgttttgttt ttttttttta 1320
gaattcgtgc gagagttttc ggggttattt tgtcgggatg atatacgttg ttaatatttt 1380
atttattat tcggttcgag gtacgttcga ggatttattt tgttgagttt cgtaggtcgt 1440
tgaattaaag gtttagatgg ggaggattag gagagcgagg gggtttttag ttgttttttt 1500
atatttgttt tggggatttt ttatatttcg gggtagattt ttttttttgt ttgtggtaag 1560
gaaggagttt tgattataga gttttttttt tgtttttatt ttattttttt tatatttaat 1620

aaaattaatt taaaaaagaa ttaaaaaggg agaacggggt aagggatttt aggttgtttt 1680
tttttttttt atgttgtttt ttttagtttt tagtttgaat tttgtttatt tagttgtttg 1740
ttatttgagt gtttatttat attttgttgt tattagttat taaaggtttt ttttagtgag 1800
gggtggaagg gatttttggg gtttggaatt tggttttaaa ttagagaatg tattttaagg 1860
gggagggtta gtgtggggga gggaggtttt tttagtttta agagattttt ttagtttagt 1920
gattgttttt aaatttaagt tttttggtag gaattaaaat tttagtttta tttttttata 1980
ttatgtggaa tttcgtgggg gtcggggatt tttttaagaa gtggtaatgg ggataagatg 2040
cggttttggt gttgtaggtt atattttgat atttataagt ttatttttat tttatagttg 2100
ttggagagaa attttaagag gtagtttttt tttattattt tattaaagat cgggttggtt 2160
agcgtttagt ttagggagaa gggcgttagt gtttaatttt attggttttt ttttcggagg 2220
tttttgtaga gggttacgtt tataaatttt tttatggaat ttttttatat tttttttttt 2280
aattttattt gtttttttta ataattttat ttgtattttt tatttttata tgatatagat 2340
tttatattgt tatatttttg tatatatttt tttagttttg tttgttttta ttttattttt 2400
ttttgttttt gagaattatt tttttatttt aagtttattt ttttatgttt ttattttttt 2460
tttggttttt gaatgttttc gtttgtataa agagttggat tttttttttt ggtgtttgta 2520
ttgtgtatat atattttttt gagaagtata aggagacgat acgcgtattg taatttttgt 2580
attgttttag tggcgataaa ggaagttgtg aattataagt tttgtttttt tttggtttta 2640
tttttttttt taattcgggt attttcggtt ttttttgtag ttttaatatt tttttttttt 2700
gtttttttta ttttattgtt ttttgtttag ttgatagtgg tatttttagg gaaggggttg 2760
ttgtagagat agtttttatt taggggatgg aggtttattt tggatattaa gttaagtgtg 2820
ttagagatag aagggagttg gggattggcg atttttgaag ttggggtagt gggatgttga 2880
taggtagaag ttgaggtttt tagttagtgg tttttttttt ttttgggtgt ttagtttttt 2940
ttttttattt gatatttaag tttattattt ttattttttg gtgaggtggg tttgggagga 3000
aagagaggtt tagaggagga gttgaaagtt ttgttgttgt tttattttat tttaatgaga 3060
gataagtgag gtggagggtt tgtttttttt ttttttatta gatatttttt ttaggtttga 3120
gttttaattt ttttttaggt tttttttttt tagttgtgtt ttggttttta attttagaat 3180
aggatttgtg agtagttgta ttggtttgga gttggagagt aaggttgtag gatttttgga 3240
attttttggt ttttaagagt ttttttagag attatatttt tttagaggga agtaggaatg 3300
aggttaaaaa gtgtgtattg gataggggaa tagtaggtag ggttttgggt gacgtatgtt 3360
tttggtttaa taaattgggt tttaattatt ttttttttag tgttgttttt ttatttgaaa 3420
aagtatttag atatttttat taagtgttta aagttgagtt tggtttgggg ttaattaggg 3480
gatgagtaag gagaaggttt gagtagggat attgtggttt ttttgggatg ggataggttg 3540
attattatat taggaatagt ttgaattatt gggtagggaa agagggagaa gttaaaggta 3600
ataaagtttt aaaatagaat ttttaatata aaggatgttt tagagagaga gagtagagtt 3660
aaaatttaag ataaattgga atttattata aaatttggaa gtaggttggt acggtggttt 3720
atgttcgtaa ttttaatatt ttgggaggtt gagataggtg gattatttgc ggttaggagt 3780
tcgagattag tttatgtaat atagtgaaat ttttattttt attttttttt ttttttttga 3840
gatggagttt cgttttgtcg tttaggttgg agtgtagtgg cgcgatttcg gtttattgta 3900
agtttcgttt ttcgggttta tattattttt ttgttttagt ttttcgagta gttgggatta 3960
taggcgttcg ttattacgtt cggttaattt ttttgtgttt ttagtagaga cggggtttta 4020
tcgtgttagt taggatggtt tcgatttttt gattttatga tttattcgtt tgggtttttt 4080
aagtgttggg attataggta tgagttatcg tgtttagttt tttattttta ttttttaata 4140
tttaaaaaaa aaaaaatttg aggttaggta taatggttta tatttgtaat tttagtattt 4200
tgggaggttg aggagtatag atcgtttgag tttaggagtt cgagattaat ttgggtaata 4260
tggcgaaatt ttatttttat taaaaatata aaatagtttg gtatggtggt gtatgtttgt 4320
ggttttagtt atttggtagg ttgagttggg aggattattt gagtttggga ggcggaggtt 4380
gtagtgagtt gagatggtgt tattgtattt tagtttgggc gagagagaga tagattttat 4440
ttaaaaaaaa ataaaaaata aaaaataata attcggaagt aggttaggtt agagagaaag 4500
ttaggatgaa gtttttacgt gaggtattta ttgaggtatt ttatttgtgg aatgagtttc 4560
gggttatgag taagggtatt tggaattttg gtttcggttt tatggttaat ttgttggttg 4620
atttttgtta agttatttat ttgtgagttt agttttttta tttgtaaaat ggaataaatt 4680
gtgttttttt ttttagtttt tgatagttat agtagtaaga aaacgaaacg aaaagtagtt 4740
aaagcgtttt ggaaagttaa cgttatgcgt taagatgaag ttattatttt aagttttttt 4800
ttattagagg gttcgagttg gggttgagat tggggttgag gttgtggttc gttttttttt 4860
ttaggttttt gttttttttt cgtcggtttg ggggcgttcg tttacgcggt ttttttattg 4920
tagagttttt ttttatggtt ttttttttcg gatcgtgaag gcgggagggc gtggttagta 4980
cgtttttttc gttagtcgtt tcgttttatt ttatagtttc gttgttttga gtttttcgtg 5040
tcggtcggtc ggtcggggga ataggcgggc gttcgggggg cgttcggggg gcggggggag 5100
tttcggtttc ggttttttgt gcggttgtat cggcggtttc gggaagatgg cggttcgggc 5160
gggtttttag tttgtggttt taagcggcgg cgtcggagtt ttaggagggg cgggcgcggt 5220
tgttgttttg ggttcgggcg gaatttcggg gttttttgtg cgaatgggtt cggtttcggg 5280
ttaagggttg tatcgttttt cgatgttcgg agcggtttat tcggtgagtg gggtaggagg 5340
aggggcgcgc gggtcggggg cggggtcgag cggggatatg ggagtgatag gggtgggggg 5400
agaagtagga gggatttttt tgggaggggt tcgggttttg ggtgggggtt gggggaggta 5460
gtgcgtagaa agagaggggg aggtagttat atatttgttt ttaggggtat tgtttttttg 5520
gggagtcggt agagaatttg ggtgggtgaa gtgggtttag ggatatttgt atgagggtcg 5580

gagtgtaaag gggtttgagt agttttagtg attagaggaa tatagaacgt gtagttgata 5640
aattgaggtt agtttttaaa gagatagtag ttagtagagt aaatgtggaa ttggcgatgt 5700
tcgatgtgtt ttatttaaaa tagattgaga atttgagtat aatttggttt tcgtttagtt 5760
tttggatttt gtttttttat ggagttatta ttggagtaaa gggttttttt tttattgttt 5820
tttattttat ttttaaattt tattttattt aaagatttta ggcgtttttt gttttttttt 5880
tttgatgggg tttagtaggt gaaattatga tatttttggg tttttttttt tttgtaga 5938

<210> 236
<211> 5938
<212> DNA
<213> Artificial Sequence

<220>
<223> chemically treated genomic DNA (Homo sapiens)

<400> 236
   tttatagagg ggagaggatt tagagatgtt atggttttat ttgttaaatt ttattaagaa 60
   agaaaagtag ggaacgtttg agatttttga atgaagtaag gtttagggat ggaatggagg 120
   ataatgagag gagaattttt tgttttagta gtgattttat agaaaaatag agtttaaaag 180
   ttaagcgaga gttagattat atttaaattt ttagtttatt ttggatggga tatatcggat 240
   atcgttagtt ttatatttat tttgttagtt attatttttt tagaagttga ttttaatttg 300
   ttagttatac gttttgtgtt tttttgatta ttggagttgt ttaagttttt ttgtatttcg 360
   gtttttatat aggtattttt ggatttattt tatttattta ggttttttat cgatttttta 420
   gggaaataat gtttttagga gtaggtgtgt aattgttttt tttttttttt ttgcgtattg 480
   ttttttttaa tttttattta gagttcgaat tttttttaag ggagtttttt ttattttttt 540
   ttttattttt gttattttta tgttttcgtt cggtttcgtt ttcggttcgc gcgttttttt 600
   ttttgtttta tttatcggat aggtcgtttc gggtatcggg gagcggtata gtttttgatt 660
   cggagtcggg tttattcgta taggaggttt cggagtttcg ttcgggttta aggtagtagt 720
   cgcgttcgtt ttttttgagg tttcggcgtc gtcgtttgga gttatagatt ggaaattcgt 780
   tcgggtcgtt attttttcgg agtcgtcgat gtagtcgtat aaagaatcgg aatcggaatt 840
   tttttcgttt ttcgagcgtt tttcgagcgt tcgtttgttt tttcggtcgg tcggtcggta 900
   cgggaggttt agggtagcga gattatggga tagagcgggg cgattagcgg aaaaggcgtg 960
   ttggttacgt tttttcgttt ttacggttcg ggagggaaaa ttatagagaa gagttttgta 1020
   atagagaggt cgcgtgagcg ggcgttttta ggtcgacggg aggaagataa ggatttggaa 1080
   agggggacgg attatagttt tagttttagt tttaatttta attcgagttt tttgatgaag 1140
   aggggtttgg agtaatagtt ttattttggc gtatggcgtt aattttttaa aacgttttga 1200
   ttgtttttcg tttcgttttt ttattgttat aattattagg ggttgaggag ggaaatatag 1260
   tttattttat tttataaatg ggaaagttga atttataggt gaatgatttg gtaaaggtta 1320
   attagtaagt tagttataaa gtcgaagtta agattttaga tatttttgtt tatagttcgg 1380
   gatttatttt atagataaag tgttttaata agtattttac gtgggaattt tattttaatt 1440
   ttttttttga tttaatttgt tttcggatta ttatttttta ttttttattt ttttttagat 1500
   gaggtttgtt tttttttcgt ttaagttgga gtgtagtggt attattttag tttattgtaa 1560
   ttttcgtttt ttaggtttaa gtgatttttt taatttagtt tattaagtag ttgggattat 1620
   aggtatgtat tattatgtta gattgttttg tatttttggt agggatgggg tttcgttatg 1680
   ttgtttaggt tggtttcgaa tttttgagtt taagcgattt gtgtttttta gttttttaaa 1740
   atgttgggat tataggtgtg agttattgtg tttggtttta gatttttttt tttttaaata 1800
   ttaaaaaata gggatggggg gttgggtacg gtggtttatg tttgtaattt tagtatttgg 1860
   gaggtttagg cgggtggatt atgaggttag gagatcgaga ttattttggt taatacggtg 1920
   aaatttcgtt tttattaaaa atataaaaaa attagtcggg cgtggtggcg ggcgtttgta 1980
   gttttagtta ttcgggaggt tgaggtaggg gaatggtgtg aattcgggag gcggagtttg 2040
   tagtgagtcg agatcgcgtt attgtatttt agtttgggcg atagagcgag attttatttt 2100
   aaaaaagaaa aaaaaaatag ggatgggggt tttattatgt tgtataggtt gatttcgaat 2160
   ttttggtcgt aagtgattta tttgttttag ttttttaaag tgttgggatt acgggtatga 2220
   gttatcgtgt tagtttgttt ttagatttta tgataagttt tagtttattt tgaattttga 2280
   ttttgttttt tttttttaaa gtattttttg tattggggat tttgttttgg aattttgttg 2340
   tttttgattt tttttttttt ttttgtttag tggtttaaat tatttttgat ataatggtta 2400
   gtttgtttta ttttaggggg attatagtgt ttttatttaa attttttttt tatttatttt 2460
   ttaattggtt ttaaattaag tttagtttta ggtatttagt gagaatattt aaatattttt 2520
   ttaaataaga gaataatatt gaagaggaga tggttgaaat ttagtttatt agattagagg 2580
   tatgcgttat ttagagtttt gtttgttgtt tttttattta atgtatattt tttggtttta 2640
   tttttgtttt tttttgggga ggtatgattt ttgaggaaat ttttaggaat taagagattt 2700
   taaagatttt atagttttat tttttagttt taggttaatg tagttgttta taggttttgt 2760
   tttgggattg aagattaaga tatagttagg gaaggaaggt ttgaagaggg gttggggttt 2820
   aggtttggaa ggagtgtttg gtggagggag gggggtaggg ttttttattt tatttgtttt 2880
   ttattaagat agggtgagat aatagtagag tttttaattt tttttttagg tttttttttt 2940
   tttttaaatt tattttatta gaaaataaaa gtggtgggtt tgggtattag gtgaggaaag 3000
   ggggttgggt atttagaagg aggaaaggtt attgattgag gattttagtt tttgtttgtt 3060
   agtattttat tgttttaatt ttaggagtcg ttaattttta gttttttttt gtttttgata 3120
   tatttggttt agtgtttagg gtagattttt attttttggg tgggggttat ttttatagta 3180
   attttttttt tggggatgtt attgttagtt gggtagaggg taatgggata ggaggagtag 3240
   gggaagagaa tgttaaggtt gtagggaggg tcgagggtgt tcgggttggg ggagagggtg 3300
   aggttagaaa gaggtagagt ttgtgattta tagttttttt tgtcgttatt gagatagtgt 3360
   aaaggttata atgcgcgtgt cgtttttttg tgttttttag agggatgtgt gtatatagta 3420
   tagatattag gggagagagt ttaatttttt atataggcga aggtatttag agattaggga 3480
   gggagtagaa atatagaagg tggaatttgg ggtgggagaa tggtttttag agataagagg 3540
   ggatggggtg gagatagata gggttgggaa agtatatata gagatatagt aatatagagt 3600
   ttgtattata tagaaataga aaatgtagat gaggttgttg agagaagtaa atgaagttgg 3660
   ggaagaggat gtgggagagt tttataagaa aatttatgga cgtggttttt tataagggtt 3720
   ttcgggagag ggattagtga ggttaggtat tagcgttttt ttttttgagt tggacgttaa 3780
   ttagttcggt ttttaatggg atggtgagga agggttgttt tttgggattt ttttttagta 3840
   gttgtggggt gggggtgaat ttataggtat taggatgtag tttatagtat tagggtcgta 3900
   ttttgttttt attattattt tttaagggga ttttcgattt ttacgagatt ttatatggtg 3960
   tgagagagtg gggttgaggt tttagttttt gttaggagat ttgggtttgg ggatagttat 4020
   tgggttgaga gggtttttta aggttgggga agtttttttt ttttatatta gttttttttt 4080
   ttaaggtata ttttttggtt tggggttaaa ttttagattt tagagatttt ttttattttt 4140
   tattgggaag ggtttttggt gattggtggt agtagaatgt agatggatat ttagatggta 4200
   gatagttaga tagatagaat ttaggttggg agttagggga ggtagtatgg aggagagagg 4260
   ggtagtttga ggttttttgt ttcgtttttt ttttttagtt tttttttaga ttagttttgt 4320
   taaatgtaaa aagaatggga tagaggtaga gagaggattt tatggttaag attttttttt 4380
   tattatagat aagaggaaag atttgtttcg gagtgtgggg agtttttagg gtagatgtga 4440
   ggaggtagtt gggggttttt tcgttttttt agtttttttt atttaggttt ttggtttagc 4500
   ggtttgcggg gtttagtagg taaagttttc gaacgtgttt cgggtcggat ggtgaggtag 4560
   gatgttggta gcgtatgtta tttcggtagg gtggtttcga aggttttcgt acgggttttg 4620
   ggggaagggg ataggatagg gacgggttat ttttcggaag ggttttaggt agtgttgggg 4680
   tattcgggag gtaaaggaga gaaggtggtg gtagaggcgg gtgggcgggg gtggacgggg 4740
   tttttggggc ggtggaggta taggcgggtt ggagggcgtt ttcgttcgtt tttttacgtg 4800
   ttttttgacg tcgtttaggt tgagggttac gtttttgttc gtattgtttt ttttggtttt 4860
   tttttggtat ttttttcgtc ggtatagttt gtgtttaatg atttaggacg gggacggggt 4920
   aggtgggcgg gttggatttc gtttttacgt ggtttcgtgt cgggtttcgt tttcgtttat 4980
   ttcgtaggcg tagttaaaga gttttagtat cgtgaggatg ttggtttcga tgaatagttt 5040
   tatttggttt tcgatgttat ttgcgggggt aggcgttagg ggggttagtg gggttaggat 5100
   atcgtggggg tagtagggtt tattgggatt ggggtaggta agttgtattt ttaaagggat 5160
   tggtagtttt cgtatagaag gatcgtaaag ttagatttag gtggaatttt tgtttttttt 5220
   tttatgtttt tttgcgtttt tttatttttt taaaataagt aggtgttttt taagaaaaat 5280
   aatcgtttat aaaatattat aattttttta gtttaggggt atatgaaata gttttatttt 5340
   ttatgtttat ttttttttta tgtttatagg tttgtttttt ttttttttta ttttgtttta 5400
   agagaaggtt tagttttttt tttttattat ttattaagat aatagggttt ggggaagggt 5460
   ttttggggtg gaggattttt atgggataga agtgagtatt ttgtttttgg atgataggga 5520
   gttacgttat gtttatggta tgagaagggg ataggtgtta ttagtagttt atttaggagt 5580
   tttgtaattt tataggtttt tttttgttta atggttttat agttgaggat tttaaagaaa 5640
   atgtttagta ttaggatgtt ttttttgtat agggattggg ggaggagatt taggtttaag 5700
   ttatattttt attttttatt aattaggtga ttttggtaag gtagtttttt tgaattttag 5760
   tttttatatt tataaaatga gatgttgatg gagatatttg ttttttagga ttaaatgaga 5820
   tggtatatat gaaaaaatgt tttgtattat atgtagtatg tattaagtat aaagtgattt 5880
   cggttataat aatgaggatg aagaaggagg agaaaggggt gtttttttgt ttattttg 5938

<210> 237
<211> 8182
<212> DNA
<213> Artificial Sequence

<220>
<223> chemically treated genomic DNA (Homo sapiens)

<400> 237

gaggagtaat ttttaatgag gatggagttt ttttttgggg ttattatttt ggattttaaa 60
attttggatt taggtagagg aggtgttgta taatattgtg aatgtaatat atattattga 120
attgtgtaat tttaaatagt taattttatt acgtaaattt tattttaatt taaaaaaata 180
gttaggtgtg gtgtatattt atttagttat ttaggaatta ttatttgagt ttaggggttt 240
gaggttagtt tgggtaattt agtaaattat tgttttaaaa aaatataaat tttaaaataa 300

aaagataaat tgagagagaa aaagattttg ttttcgtaga gtttatattt tagtgggtga 360
gttatattag atagtaaatt agtatatatt taagtaatga tgtattgtga taattatttg 420
gtgggaagta gggtgtgaga gaatagagag gttttgagat gggaagggga tgggtagttt 480
gaaaaattgt aaggagatta tttgggttaa tgtttggaga gttaaaggta gtgatttaaa 540
atgaatttaa ggaggtaagt agggattagg ttttgtagag ttttaaatgt tataggaggg 600
agtgtgatat ttaatttaag agtaatgggg agttggggag ggttttaaat aagggattga 660
taggaattga gttatattta atcgtttgat ttgttttatt attattgaga tagtgtttta 720
ttttgttatt taggttggag tgtagtggta cgatttttgt ttattgtaat tattgttttt 780
tgggtttaag taattttcgt gttttagttt tttgagtagt tgggattata ggtgtgtatt 840
attacgttta gttaattttt atatttttag tagagatatg gttttattat tttggttaga 900
ttggtttcga atttttgatt ttaagtgatt tatttatttt ggttttttaa agtgttggga 960
ttataggtgt gagttatttt atttagttga tttgttttta aatgtattta ttagtttgtt 1020
tttttttttag ggtagaaaaa atttgtgttt ttttgatgcg atttattggg tcgtattaga 1080
attatttagg aagtttattt tttaaatata atattgagag ttattggatt ggaggggtgg 1140
ttggcggagg tattttggag tagtttttta gaggattttg attttttatt gtttttatta 1200
ttttgagaat tattgttgtt ttagaagttt aaaaggattg aaatggatat tagttgtttt 1260
attatgaatt tttaatttat tttataggga tgggaggggt tatgatttag tgattttaat 1320
aattttagtg gttagttttt agattgatag gttatcgatt tttggggtaa tagagtggga 1380
tttttaagtt taggaggtaa gaaaatagta gtagcggtgt ttattattag aggtatgagg 1440
agaattaatt tttaaaaagt attggagaat tatattgtaa aaaagatttg gggtgttatg 1500
atgtaaggaa ggaagtatta tttggttggg gttggaaaaa taaagtaatt atagtaaaaa 1560
aattaaaaaa aaaaaaaagt ttagatttcg gttttttgat agtgtggttt gagaagtaat 1620
agtatcgttg gtagtttgtt ataaatgtag agttttgggt tttgttataa tttgtatttt 1680
taataagagt ttcgagtgtt ttttatgtgt ttattaaagt ttaagtagta ttggtttata 1740
ttacggcgag gagatttaga ttattagagg gtaggttttt tggtatagtt gacgtatttt 1800
tgaatatttt gatatagtat agtgtatttg aaaagatatt gtatttttga aaattaagtg 1860
ttataaatat tggagaaaga tttttttttt taaattgcgg ggaggttttt tataagttaa 1920
attttttttg attagaagta ttttttgggg agtaaagtat taggtttttt taaagtgagg 1980
cggagttacg ttaaaaatgt gcggtagaat ttagttttgg ttaagtcgat cgtttattag 2040
ttgggttaag tttggagttt tttaggtttt ttggtagttt cgagttatta gtatgagttt 2100
ttataggaag ttgagttggt tgttgttttg ttttatagag tagttgagtt tttttttttt 2160
tttttttttt ttttgatgag ttttattttt gtcgtttagg ttggagtgta gtggggcgat 2220
ttcggtttat tgtaattttt gtttttaagg tttaagcgat ttttttgttt tagtttgtcg 2280
agtagtttgg attataggcg ttcgttatta tattcggtta atttttgtat ttttagtaga 2340
gacggggttt ttattatgtt ggttaggttg gtttcgaatt tttgatttta ggggattcgt 2400
tcgttttagt tttttaaagc gttgggatta taggcgtgag ttatagttat cgattttgag 2460
ttgagttttt aacgtcgaag atgtagaaag gagatttgat aaaattattt tcggggagtg 2520
tggtcgcgga attcggtgag ggtgttatat cgggagagag tagtagtaac ggtttattta 2580
ttgtgaagtt tttaagtgat tttagatttt tgatttcgcg ttttattcgg cgttttcgcg 2640
cgtagttttc gttttcgttt tttcgacgaa aggttgggtt aaaggtgggg gggtggggtt 2700
ttgaggtcgg tagttagggg cggtgttcgt tgggggcggg tttcgcgagg ggtaggattc 2760
gaggaggtat tcgagtattg ggtattaggt agaggcgggg tttagtgttg ggttgggttg 2820
ggcggggtag ggagttcgta gtcgtttttg ggtaattcga ttcgggcggt taaattttcg 2880
gaggtcgggg acggaaggcg ggttcgtagt agattttgga ttcggaattt ttcgggtagg 2940
agcggaattt gtttcgaatc gggtttgtga ggaattcgcg aatttggatt aggaaatttc 3000
ggagttcgga tcgataaatt tcggaattcg gaattaagat cgttaagttt cggatcgcgg 3060
agtatagagt acggagtgga ttcgacgcgg agttcggagt tcggatcgcg gtatcgcggg 3120
acgggacgga gcgatgtcgg gtcgaggcgc gggcggtttt tcgttgtttt cgttaagttt 3180
tggcggcggc gtcgtggttg cggttttggg agcgtcgttt tttttcgcgg gattcggtat 3240
gttgttcgga tcggcgtttc ggggatcggg ttcggtagga ggcgtggggg gtttcggggt 3300
cgtcgttttt cgttttatgg gtttcgcggg tttcgcggcg tagtattagg tgagtaagga 3360
ggacgcgagc ggacgggggc gagaggcgtt gcgagggcgt tcgggtcggc ggttgaaggg 3420
gtttcgagtg agggttttgg ggaatgtatg aggaatttcg ggagacggtg gagggcgggt 3480
cgggattttc gggttgcggc gggtcgcggt ggagaagtgg gaaggggaag taggttttgt 3540
agcggagggt tggaagtagt tgggaggttt gggggttttc gcggtatagg gatttcgagt 3600
gattgggaga tggaggagtt tggggagtta gtgaattgtg gcgggtgggt tgagaagttt 3660
agggtaggag ggttggaaag tgatgagaaa gtttgggaaa gggttttcgt tgagaaatcg 3720
gggttatttt gtatcggttt tggttttagt ttaagtttgt tttgtttttt ttgattggga 3780
attggcggag ttttagcgtg tttgagatgg ttttcggttg ttaataagga ggaaggagtt 3840
tggtttttta cgggtacggg acgttgaggt tagggttagg ggattttagt ttgtgtacgg 3900
gcggatttta tttttgaggt agggttaggc gttttcgttg ggaggatttt agtttagatt 3960
agagacggtg gtggtttggt ttgaggggag gtttagttta gttataggtc ggtttttttt 4020
tgggtttttt gttttttgtt atcgtgttcg ggagttgatt agtttggacg tgtagagtgg 4080
agttttgttg gtttggggtc ggaggagttt gcgagagttg ttttaaagag tgttgttttg 4140
atttttttgg ggttttgaga gttgtttaga ttgtagtttt aaaagttttt gtaaagtagc 4200
gatttttgtt tagtgtgtga ggattttagt ttgattttgt tagtgtttta gtttgttgtt 4260

taggttgttt tagaggtttt ggaaattgtg tttatttgtt ttgttatttt ttttgtattt 4320
ttatttggtt tttaggtttt aattttatta cgttcgaagt tatgtgttta agtatttgat 4380
attggttttt tgtttattta cgggtttgtt gtgggttttt tttgttttcg atttatgttt 4440
ttaggtagag gtttttaggg attttcgtgg agtatatata gtttatttgt tttattgggt 4500
ttttattttt aaggagatgg tttaattagg gatagtgatt ggattttttt ttttttttta 4560
ttttttattt tttttttttt tttaagagaa aagagagttt gggtatgggt agagatagat 4620
ttggttttag ggattttagt tggggtttat tttttttatt ttgttgtttt cgttggggat 4680
ttatgggttt agggtgttgt tgagggtttt ttgtattatt tattgtagtt tttgggtttg 4740
tttattgaga tttggtttgg gtattatttg tgggtttttg gttttggaag gatttggaat 4800
agataagtcg ttttagtttt tttggtatgt aagttatagg aaatgtgttt ttgggatagt 4860
ttttgagagg atggatttta tatgttttgg tggggggtgg ttttgggttt gtggtaaggt 4920
taagttggtt ggttggttag gtttgggttg ttagttgtgc ggaagtagga gatggttttg 4980
agtttgggtt ttttatttta ttagtttttt tttttttggt aggagaggta gatgggaagg 5040
agaatttagt tagttaattt tggttgagtt tgaagaggat tttatttgtt ttttttttaa 5100
gtttagttgt atgattgtta ggagaaggga tttgagattt gggaaataaa ggggtttttg 5160
tttgttaggg tttatgagat gtatttgaaa agtgagaatt tgaggtttaa gttgagtaaa 5220
tagttgaaag aagggaagtg atgatttttt tttttttaga gggtggtttt agttgtggat 5280
tatagttaat agaggaattt aaaaaaagaa aaagaaaaga aaaaaatttt tagggtagga 5340
tatggtgggt ttatatttgt aattttagta ttttgggagg tttagataga aggattattt 5400
gagattagga gtttaagatt agtttaagta atatagtgag attattattt tttaaaaaaa 5460
aaaaaaaaaa tttagttaag tatggtggtg tgcgtttatg gttttagtta tttaggatgt 5520
tgtggtagga ggattgtttg agtttaggag tttaaggttg tattgagtta tgattttgtt 5580
attgtatttt agtttgggta ataaaataat aaaaatttta gattgttggg tttattttag 5640
agagtttaaa aagtttaaaa aaaaaatttt ttttttttga tacggagttt tattttgtcg 5700
tttaggttgg agtgtagtga tatgatttta gtttattgta attttcgttt tttaggttta 5760
aataattttt tgttttaagt tttttgagta gttgggatta taggcgttta ttattacgtt 5820
cggttaattt ttttgtattt ttagtagaga cgaggtttta ttattttggt taggatggtt 5880
tcgaattttt gattttgtga tttattcgtt tcggtttttt aaagtgttgg gattataggc 5940
gtgagttatt gtattcggtt ttaaaatttt ttttatttat gggagagtgg gtggtatgtt 6000
ttttaattga ggtttttagg tttaggtttt aggagtatta gggttatatt tggggtaggg 6060
taaagtacgt ttatcggagt tttggatttt ttatttgggg tttaggggat tgatttaggt 6120
ttatttggtt ttgttgtagg tagggagtta gggaggttta gagtgttgga ttgttttttt 6180
tttttttttt aatttttgag ttgttttttt ttgtttgttt tgttttgttt tgttttttga 6240
agtgggagga gttgttagcg tttataagtt ttatggagtt tggtaaggtt tagttgggat 6300
ttgaattata agtagttttt ttttggggtt ttgagtgagt taggggtttt gggtttagga 6360
aatttggagt agatgtttgg gtggagttta ttttagattt tgtttatgag gttttgtttt 6420
gggatttttt gttatcggag tatttgttta ggagggatgg gtggtgttgt aggttttgtg 6480
aatattgtag tagggagttt tttggggatt atagggtttt cgtttatttt tgtaggattt 6540
attgttttag tttttttttg tttttgtttt aggtattagg aaaaattagt ttgggatgtt 6600
tgggtttggg tttatagttg tagttatatt tagttttttt ttataatttt aggttagaga 6660
aagaataatt tttaaggtag agaaagaggt tgttttttgt tttttaatag ttttgattaa 6720
tttttttttg atgtagagag attttggtag gatttttaaa gttttagagt tttttatttt 6780
gaatttttta tttttttaaa tattttcgtt ttattttgtt tttttttttt ttatttttac 6840
gtttgtgggt tatgtttttt tttattttcg tatttggtta agaatttgat tttttgtgtt 6900
gggaatatgg gtttagtgcg ggatgaagag gttggggaag aattttattt ttatgatgtt 6960
ggggtggggg aggggttttt gatttggtat aagtttttat tttgtcgggt ttggttggtt 7020
ggttgtgttc gttgtataat ttgggttttg gggttagttt tgtgaagttg ttagggatag 7080
tgtgtgtaag gtagagttgt taaataggtt ttgtaggtag tagttatggg gaggcgggtg 7140
ggggtggagg tgatttttag atgggtttta tagaaatgtt agggagtaag gttttaggtg 7200
ggaaattttt ttgtaggtat ttaaagaagt tatttttgtt ttttattttt ttgttgttta 7260
gtttgggttt ttatttttgt tttttaggat aattttagtt ttttttttta gtttgggatt 7320
gttagggtta tacggagtta gagttagttt tggttgttag gtggtgtttg gattattggt 7380
agagtttagg tgttagttgt tttttttttt ataggttatt taggagttat agtttttttt 7440
ttggggtttt ttgttatagg ggtagtagtt gggtagtagg gaggtaagag gagggtttag 7500
ttttgggttg tgggtggggg ttatgtgata ttagattata gggtataggg tttttgtatt 7560
ttgttcgagg ttatttttat tagttggtat acgttttaag ttattttgtt gtagttttgt 7620
ttatttgggg tgttgttgtt tattagattt tttttttttt cggttataga ggatggggat 7680
tggggttttg cgttattggt ttatttatat tatagtatat ttttttgttt ggtagtgata 7740
ttgtttttgt gttttttagt tatgtttttt ttttatgatg ggagaaagag aaggagggta 7800
gggtttggta ggattgtttg agagaatagg gattagtttg tttagggaat agggtatagt 7860
atgttggggt tggaaggtag taatgggttt tgaaaagttt tttaaggttt ttttttttgt 7920
aggtaggatt ttttttttag tttaggtttt ggtaggtttt attcgttggt atggttgtta 7980
gggcggttgt agggggtagg gtaggtgtgg tttatatttt gtgtttaggc gtttattttt 8040
ttttgttttt ggggttttag tagggttttg gtgtgttgtt taggttttat ttggttgacg 8100
agagggatgg agggtatggg ttatgggttt cggagggttt tgtttttgat ttatgtattt 8160
tcgttggttt atttttttta gt 8182

<210> 238
<211> 8182
<212> DNA
<213> Artificial Sequence

<220>
<223> chemically treated genomic DNA (Homo sapiens)

<400> 238

gttgggggaa gtgagttaac gggggtgtat aggttagggg taaggttttt cgggatttat 60
agtttatgtt ttttattttt ttcgttagtt aggtagggtt tgagtagtat attagagttt 120
tgttggagtt ttaggaataa aggggagtgg gcgtttgagt atagagtgta aattatattt 180
gttttgtttt ttgtaatcgt tttggtagtt atgttagcga atgaggtttg ttagaatttg 240
ggttgggagg agggttttgt ttgtagaggg aagggttttg gggagttttt tagggtttat 300
tgttattttt tagttttagt atgttgtgtt ttattttttg ggtaagttgg tttttatttt 360
tttagatagt tttgttaagt tttgtttttt tttttttttt tttattatga agaagaagta 420
tggttgagag gtataggggt agtgttattg ttaggtagag gagtgtatta tagtatgagt 480
aggttaatgg cgtagagttt taatttttat tttttgtggt cgagggagaa aggggtttgg 540
taggtagtag tattttaggt gagtagggtt gtaataggat ggtttggaac gtgtattagt 600
tggtgggggt ggtttcgggt agggtatagg gattttgtat tttgtagttt ggtattatat 660
agtttttatt tatagtttag aattggattt tttttttatt tttttattgt ttaattatta 720
tttttatgat agaagatttt agaagggaag ttatggtttt tgggtggttt gtgggaaaga 780
gagtagttag tatttggatt ttgttagtgg tttaggtatt atttggtagt taggattggt 840
tttggtttcg tgtagttttg gtaattttaa gttggagaag ggggttaaga ttattttggg 900
aggtaaggat agaggtttag gttgagtagt aggaaagtgg gagatagaaa tagttttttt 960
agatgtttgt agaaggattt tttatttgag gttttgtttt ttgatatttt tgtggagttt 1020
atttgggagt tatttttatt tttattcgtt tttttatggt tgttgtttgt aaggtttgtt 1080
tggtagtttt gttttgtata tattgttttt ggtagtttta tagggttggt tttagggttt 1140
agattatgta acgagtataa ttagttaatt aagttcggta gggtggagat ttgtattaaa 1200
ttaaaggatt tttttttatt ttagtattat ggagatgaga ttttttttta atttttttat 1260
ttcgtattaa atttatgttt ttaatataga gggttagatt tttgattaga tgcggagatg 1320
gaaagaaata tgatttatag acgtgaggat ggggagagga ggagtagggt gggacggaag 1380
tgtttgggag aataaaaagt ttaaagtggg gggttttggg gttttgggag ttttgttaaa 1440
gtttttttgt attaggaaga gattaattag agttgttgaa aggtaaaaga tagttttttt 1500
ttttgtttta aagattgttt tttttttggt ttgagattat agaaaaaaat taagtgtggt 1560
tatagttgta agtttaggtt tagatatttt agattagttt tttttagtgt ttgagataaa 1620
gatagggaag aattgaagta gtgggttttg tagaggtgag cgagggtttt gtggttttta 1680
ggaagttttt tgttgtagtg tttataaaat ttgtagtatt atttattttt tttgggtagg 1740
tgtttcggta ataggaagtt ttaagataag gttttatagg tagggtttgg aataggtttt 1800
atttagatat ttgttttagg ttttttgggt ttagagtttt tggtttattt agggttttag 1860
gggaaggttg tttatagttt aagttttagt tgagttttgt taagttttat agggtttata 1920
ggcgttagta atttttttta ttttaaaaaa taaaataaaa taaaataaat aaaaaaaaat 1980
aatttaggag ttaaagaaaa aaaaaaaagt agtttagtat tttgagtttt tttaattttt 2040
tgtttgtagt aggattaggt aggtttggat taattttttg aattttagat gaggaattta 2100
gggtttcggt gagcgtgttt tgttttgttt taaatgtggt tttgatgttt ttggaatttg 2160
ggtttgaggg ttttagttgg ggaatatatt atttattttt ttatgagtaa aaaggatttt 2220
gaggtcgggt gtagtggttt acgtttataa ttttagtatt ttgggaggtc gaggcgggtg 2280
gattataagg ttaggagttc gagattattt tggttaagat ggtgaaattt cgtttttatt 2340
aaaaatataa aaaaattagt cgggcgtggt ggtgggcgtt tgtaatttta gttatttagg 2400
aggtttgagg tagaggattg tttgaatttg ggaggcggag gttgtagtga gttgagatta 2460
tgttattgta ttttagtttg ggcgatagag tgagatttcg tattagaaaa aaaaaatttt 2520
ttttttgaat tttttgaatt ttttggagtg aatttagtaa tttgaggttt ttgttatttt 2580
gttgtttagg ttggagtgta gtggtaggat tatagtttaa tgtagttttg aatttttgga 2640
tttaagtaat ttttttgtta tagtattttg agtagttggg attataagcg tatattatta 2700
tgtttggtta aatttttttt ttttttttta agagatgatg gttttattat gttgtttagg 2760
ttggttttga atttttggtt ttaagtaatt tttttgtttg ggttttttaa agtgttagga 2820
ttataggtgt gagtttatta tattttgttt tggaggtttt tttttttttt tttttttttt 2880
ttaggttttt ttgttaattg tgatttatag ttagaattat tttttggagg aagggaggtt 2940
attatttttt tttttttaat tatttgttta gtttgggttt taaattttta ttttttaagt 3000
gtattttatg agttttaata gataggaatt tttttgtttt ttagatttta agtttttttt 3060
tttggtagtt atataattgg atttggaaag aaggtaaata ggattttttt taaatttagt 3120
taaagttagt tggttgggtt ttttttttta tttatttttt ttgttaggag gaggaaagtt 3180
agtgggatgg gaaatttaga tttaggttta ttttttattt tcgtatagtt agtagtttag 3240
gtttaattag ttagttagtt tggttttgtt ataggtttaa ggttattttt tattaaaata 3300

tgtggaattt atttttttag gggttgtttt aagggtatat tttttatggt ttgtatgtta 3360
aggaaattgg agcggtttgt ttgttttagg tttttttaag gttaaaggtt tatagatggt 3420
gtttaggtta ggttttagtg ggtaggttta agggttataa tgaatgatgt agggagtttt 3480
tagtaatatt ttgggtttat gaatttttaa cggggatagt aaggtgggag agatgagttt 3540
tagttggaat ttttgggatt aggtttgttt ttgtttatat ttagattttt tttttttttg 3600
agaaaggagg aagaatgagg agtaagaagg aaaaaaaaat ttagttattg tttttagtta 3660
agttattttt ttagaagtga gggtttagtg gggtaggtag gttgtgtata ttttacgagg 3720
atttttaagg atttttgttt ggagatatga gtcgaaaata gaggaagttt atagtaaatt 3780
cgtagatgag taagaagtta gtgttaaatg tttgggtata tggtttcgga cgtggtggag 3840
ttagaatttg aaggttaaat aaaaatgtag agagggtgat aaggtagatg ggtatagttt 3900
ttaagttttt tggggtaatt tgaatagtag attagagtat tggtagggtt aggttgaggt 3960
ttttatatat tgagtagaag tcgttgtttt gtaagggttt ttgaagttgt aatttgagta 4020
gtttttaaaa ttttaaaggg gttaggatag tattttttga agtaattttc gtagattttt 4080
tcggttttag attagtaggg ttttattttg tacgtttaga ttaattaatt ttcggatacg 4140
atgataggag gtagaaagtt tagaagagag tcggtttgtg attgggttgg gttttttttt 4200
aggttaggtt attatcgttt ttggtttaag ttggagtttt tttagcgaag gcgtttggtt 4260
ttgttttagg agtaaagttc gttcgtgtat aagttggaat tttttggttt tagttttagc 4320
gtttcgtgtt cgtgggaagt taggtttttt tttttttatt agtagtcggg agttatttta 4380
gatacgttag agtttcgtta gtttttaatt agaaaaaata gggtagattt ggattgggat 4440
taaggtcgat gtagggtaat ttcggttttt tagcgagggt ttttttttaa atttttttat 4500
tattttttag tttttttatt ttagattttt taatttattc gttataattt attggttttt 4560
taaatttttt tattttttag ttattcggaa tttttgtgtc gcggaagttt ttaggttttt 4620
tagttgtttt tagtttttcg ttgtagaatt tgtttttttt ttttattttt ttatcgcgat 4680
tcgtcgtagt tcgagggttt cggttcgttt tttatcgttt ttcggagttt tttatgtatt 4740
ttttagggtt tttattcgag gtttttttag tcgtcggttc gggcgttttc gtagcgtttt 4800
tcgttttcgt tcgttcgcgt tttttttgtt tatttggtat tgcgtcgcgg ggttcgcggg 4860
gtttatgggg cggaaggcgg cggtttcggg gttttttacg ttttttgtcg gattcggttt 4920
tcggagcgtc ggttcgggta gtatgtcggg tttcgcgggg ggaggcggcg tttttagggt 4980
cgtagttacg gcgtcgtcgt tagggtttag cgggggtagc gggaattcgt tcgcgtttcg 5040
gttcgatatc gtttcgtttc gtttcgcggt gtcgcgattc ggatttcggg tttcgcgtcg 5100
agtttatttc gtgttttgtg tttcgcgatt cgggatttgg cgattttaat ttcgggtttc 5160
gggatttgtc gattcgggtt tcgggatttt ttaatttaag ttcgcgagtt ttttatagat 5220
tcggttcggg atagatttcg tttttgttcg ggagatttcg gatttaggat ttgttgcggg 5280
ttcgtttttc gttttcggtt ttcggaggtt tggtcgttcg agtcgagtta tttagaggcg 5340
gttacgaatt ttttgtttcg tttaatttag tttagtatta ggtttcgttt ttatttggtg 5400
tttaatgttc gagtgttttt tcgaattttg tttttcgcga gattcgtttt tagcgggtat 5460
cgtttttaat tgtcgatttt agagttttat ttttttattt ttggtttagt ttttcgtcgg 5520
aggggcggga gcggaagttg cgcgcggaag cgtcgagtga ggcgcggggt tagaggttta 5580
aggttattta ggggttttat agtgaatgag tcgttgttat tatttttttt cggtatagta 5640
tttttatcga atttcgcggt tatatttttc gaaaatagtt ttgttaagtt ttttttttgt 5700
attttcggcg ttaaagattt aatttagagt cggtagttgt ggtttacgtt tgtaatttta 5760
gcgttttggg aggttgaggc gggcggattt tttgaggtta gaagttcgag attagtttgg 5820
ttaatatggt gaaaatttcg tttttattaa aaatataaaa attagtcgag tgtggtgacg 5880
ggcgtttgta atttaagtta ttcggtaggt tgaggtagga gaatcgtttg aattttggag 5940
gtagaggttg taatgagtcg agatcgtttt attgtatttt agtttggcgg gtaagagtga 6000
aatttattaa aaaaaaaaaa aaaaaaaaaa aagatttagt tgttttgtgg ggtagaatag 6060
tagttaattt aattttttgt aaaagtttat gttggtagtt cggaattgtt aagggattta 6120
ggaggtttta gatttgattt agttggtgaa cggtcgattt ggttaaggtt gagttttatc 6180
gtatattttt aacgtggttt cgttttattt taaggaagtt tggtatttta ttttttagaa 6240
aatgttttta gttaaaagaa atttggttta tgagaaattt tttcgtagtt taaaaaaaga 6300
ggtttttttt tagtatttat agtatttaat ttttaaaaat gtagtatttt tttaggtata 6360
ttgtgttgta ttaaagtatt taaaaatgcg ttagttgtgt tagggagttt attttttgat 6420
ggtttggatt ttttcgtcgt gatatggatt agtgttgttt aaattttaat gagtatataa 6480
aaaatattcg aggtttttat taaaaatgta gattgtggta gggtttagga ttttgtattt 6540
gtaataagtt gttagcgatg ttgttgtttt ttagattata ttgttaagaa gtcgggattt 6600
agattttttt ttttttttag tttttttatt ataattgttt tattttttta gttttaatta 6660
agtaatattt ttttttttgt attatagtat tttagatttt ttttatagta tagtttttta 6720
gtgtttttta gaaattagtt ttttttatgt ttttagtgat aaatatcgtt gttgttgttt 6780
ttttattttt tgaatttaga gattttattt tattgtttta agagtcgata atttgttagt 6840
ttggggattg gttattggaa ttgttaaagt tattgaatta tggttttttt tatttttgta 6900
aagtgggtta agagtttatg ataaggtagt taatatttat tttaattttt ttaagttttt 6960
agaataatag taatttttaa agtgataggg gtagtggaga attagagttt tttgggaagt 7020
tattttaaaa tattttcgtt agttattttt ttaatttaat ggtttttagt attgtatttg 7080
aaaaataagt tttttaggta attttgatgc gatttagtgg gtcgtattag aaaatataag 7140
atttttttta ttttagaaaa ggggtaagtt aataagtgta tttaaaagta gattagttgg 7200
gtggagtggt ttatatttgt aattttagta ttttgggagg ttaaggtgag tggattattt 7260

gaagttagga gttcgagatt agtttggtta agatggtgaa attatgtttt tattaaaaat 7320
ataaaaatta gttgggcgtg gtggtgtata tttgtaattt tagttattta ggaggttgag 7380
gtacgagaat tgtttgaatt taagaggtag tggttgtagt gagtagagat cgtgttattg 7440
tattttagtt tgggtgatag agtaagatat tgttttaata ataataaagt agattaaacg 7500
gttaaatata atttagtttt tgttaatttt ttgtttaaaa ttttttttag ttttttattg 7560
tttttagatt gaatgttata tttttttttg tgatatttaa ggttttgtag gatttggttt 7620
ttgtttattt ttttgggttt attttaaatt attgtttttg gttttttaag tattggttta 7680
gatggttttt ttgtagtttt ttaaattatt tatttttttt ttattttaga gtttttttgt 7740
ttttttatat tttatttttt attaagtgat tattatagta tattattatt taagtgtgtg 7800
ttgatttatt gtttgatgtg gtttatttat taaaatataa attttacgaa ggtaggattt 7860
tttttttttt taatttattt ttttatttta aaatttgtat ttttttaaga tagtgatttg 7920
ttaggttgtt taggttggtt ttaaattttt gggtttaagt gatagttttt gaatagttga 7980
gtaggtatat attatatttg gttatttttt taaattgaag tgagatttac gtaataaaat 8040
taattgtttg aaattgtata atttagtggt atatattata tttataatgt tgtgtaatat 8100
tttttttatt taggtttaaa attttggggt ttaaaataat gattttaaaa gaaaatttta 8160
tttttattag aagttgtttt tt 8182

<210> 239
<211> 6898
<212> DNA
<213> Artificial Sequence

<220>
<223> chemically treated genomic DNA (Homo sapiens)

<400> 239

aaggttgttt tttttgtgaa atgtttttat tttttagttt ttattttttt ttttagaggt 60
tgttagttga gggggaagaa gtttgatata tgtggatttg aattttagtt ttgttatttt 120
ttggttatgt ggtcgtaaat atgttataga tttttttggg ttttgtgttt tgtcgtttgt 180
aaatgtgaat aataatgttt attttgtagg gttattggaa gattaaatat ttgttataga 240
gtttaatagg tggtgggtga ataaatattt tttttatttt tttagtatgt tttttttttt 300
tttttttttt tttgagatgg agttttatat tgtcgtttag gttggagtgt agtggtatat 360
ggttgatcgt aattttattt tttcggattt aagtgattgt tttgttttag tttttcgagt 420
ggttgggatt ataggtatgt gttattatgt tttattaatt tttgtatttt tagtagagga 480
agggtttcgt tatgttggtc gggttggttt taaacgtttg attttaggtg atttgttcgt 540
tttagttttt tatagtgttg ggattatagg tatgaattat tgcgtttagt ttttatttat 600
tatttttgta aaatttggta gaatatatat aatatataat ttattatttt tattgtttta 660
agtgtataga ttagtggtat tgaagaaatt cgtagtgttg tgtagttatt attgttattt 720
atttatagaa tgttttttat tttgtgaaat tgaagttcgg tgtttttatt gtattgttat 780
tttttttttt ttagtttttg gtatgtttta ttttattatt ttttttttgg tatttggttt 840
ggtttttgtg acggtattag ttttttttag ttggtgttgt tagttgttta gaagttttaa 900
ttttttcgta ggttaagtgg tattttattt atttttattt ttattatcga ggtatatgtt 960
gataatagag gtttgagagg aaaaatgtaa aatgaatgag agaatgagta gagagagggt 1020
tacgtgtggg tttatggtta aaggtatata gttgtgcgtt tgattttatg tagtaggtgt 1080
ttagaatata ttgaaagttt aaatgatgat acgagtggtt tttgtaaatg tttagttatt 1140
ttttgaatag gtgaaatatt tgaaatttaa aaatgtataa aagaatatat agtggaaagt 1200
tagttttttt ttaatttagt tttttagttt tattggaggt aattattttg gttagttttt 1260
tttgtatttt tttggaggag tgtatatata ttatatgtgt atatatttat acgtgtgtat 1320
gtatatgttt atatagtata tgttcgtata tttatatgtg gaagtatgtt ttatgtattg 1380
tttgtatatt ttgttttttt ttatttgata gatttttggg atttttttaa gattagtata 1440
tggatagttt ttttattgtt tgatagtatt tattgaatga taaatttatt ttgttattat 1500
ttatagttgt tataggattt atgttatttg ttatttgttg atgattatgt aggttatttt 1560
tgaattttta ttatataagt cgtgttgtag tgagtagttt cggacgttta cgaatgattg 1620
aatgattgaa tgtttgaacg tgatttttaa ttttatagtt tagtttaatt tttgtttggt 1680
ttttttggtt tttaaagtat tttatttttt taggattttt atttgtattt attacggttt 1740
gtgatttttt ttttatttcg tgttaggtat tgtgttaggt tttagagtgt ttaggttgat 1800
gggttttagt tttgttttta tagaggtata gtttagaggg agagataaat gaggaaagag 1860
atatttagaa attaggtttg agtgttggga tagatttgtc gttttaaatt tatgtttttt 1920
ttttgatatt tggggaggta tagatttatt tttgataatt ttatttttag tttaggtttt 1980
tagggttaag gaggagaaag ggaggttatg gggtgtgggg ttagggttag aggggatcga 2040
atgtttattt ggttgttttg ttttgtatta ttcgggttag gtttgtttgt ttgatgttgt 2100
ttgtgtttat cgatttttta ttgtgttttg gttgtttgtt ttttttttgg gttcgttttg 2160
ttaattttta tgataggatt ttgttatttt attgtttttt ttatttttga aattaattat 2220
ttgttttatt ggaagggttt ttttattttt attttgtgaa gttaatagtt ggtgatagtg 2280
ggaggggaag gtagtattaa ggttatttag atggtgggga gagggtagat attgtttata 2340

tataggttat tttcggttta agggagtgaa gttagcgttg attttcgggt attagggttt 2400
gggttttagg aagtttttta agttgtatta aaatttatta tttagaagtt tttagttttt 2460
tttagtttat tggataatgt tttttttatt taaaatattt attttatttt tttttttttt 2520
gttatatttg ttgtttaagt attgttttta ggtttgtttt ttggggttat aagatattgt 2580
atttttaaaa atttttcgtt taaagggaaa tgtttttagg aaggatagta gggatttttt 2640
tttattatgg atttaggttt ttttatgaaa acgggtagaa gaattagttt tgatgaaggg 2700
tattaagtaa gtaaatgtgg ttaaaatgtt tatagcgggt tttttgagta gttagaggtg 2760
taagagtgtt tggggtaagg gttttgaagg taggaatata ggcgcggggg aaggggcgtg 2820
ggaaggttga gaacgttttt tttgggttaa gttagggtta gttttttttt tgtttgatgt 2880
tagcgtgttt taaaagggga gtaaaagtat aaatcgtaat agttagatga tggaagtatt 2940
ttaagtgttt tatagtaaat gaatggataa ataacggtgt agtggtttta gtttgtaaat 3000
ttagtatttc gggaggtcga gttgggtgga tcgtttgagt ttaagagttc gagattagat 3060
ttggtaatat ggcgaaagtt tattattata aaaaatataa aaattagttt ggtgtggtgg 3120
tgtgtatttg tagttttatt atttaggagg ttgaggttag aggattgttt tagtttagga 3180
gtttaaggtt gtagtgagtt tagatcgtgt tattgtattt tagtatgggc gatagagtaa 3240
gattttattt ttaatataat aataataata ggggtgtata tatatagtgg aatatatttc 3300
ggttttaaaa aggaagaaaa ttttgattcg tgttataacg tggatgaatt ttgaagatat 3360
tgtgtgaagt gaaataagtt agttataaag gtattatatg aggtatatgg agtagttaaa 3420
tttatggata gaaagtagaa tgtggttgtt aggggatagg aagggggaag ggggagttgt 3480
tgttagatgg atatagagtt ttagtttggg atgatggaaa gagttttgga gattggttgt 3540
ataataatgt gaaatatttt ataaaattta atatacgttg taaaatggtt aagatggtaa 3600
attttatatt atgtttattt tattataatt aaaaatttaa aatatgtcgg gtgtggtggt 3660
ttatatttgt aattttagta ttttgggagg tcgaggcggg tagattattt gaggttagga 3720
gttcgagatt agtttagtta atatggtgaa atttcgtttt tattaaaaat ataaaaatta 3780
gttaggtatg gtggtaggtg ttcgtaattt tagttatttg ggaggttgag gtaagagaat 3840
tttttgaatt taggaggtag aggttgtagt gagtcgagat cgcgttatcg tattttagtt 3900
tgggggacga gagtaagatt ttattttaaa aaaaaaaaat taaaagaata aaatagaaat 3960
atttttagat gaagggagga taggaagata ttagaaaggg aaggggtgga aaagttttgt 4020
ttttgttttg ggtttgttta taatagagat aaatttaggt agtgtagagt ttagatttta 4080
aaatattttt agtgttgaag gtgtgatttg gtttggggga gaggttggat atcggtaggg 4140
gtagagacgg gaatttgagg gagaaggatt tgattttgat tttagtttta gattatgggt 4200
ttttgttgtt tgtttgtttt ttattggggg tttacgaggt ttatttggtt ttaaatgttt 4260
ggtaggtttt ttgttcgttg gttgtttagg gaggtgtttt tatttttttc gtcgttgttt 4320
gagtttttta gggttttttt attttgtagg taggttgggg tttttttttg tgttgggtat 4380
tgtggtagtt tagtcgtgtg aggcggggtt ttggaagagt ttatgagatg gttagagttg 4440
ggtttgtttg gttggaagtt gggaataggt ttttttaagt tgttaaattt aagtattgag 4500
tatataaaag gaggagattg gtattagtga gaatatggtt ttgaggtttg atggatttaa 4560
agttttttag ggtatgtgcg ttttgagggg gtggggggtg tatatgggtg ttcgggattt 4620
cgtttaggta atgtttgtag ttatatagga tgatttgatt gaatttgtgt gaggataatc 4680
gtttgtgtgt ttgtgtgtgc gcgtttacgt gtatgcgcgt gtgtggtttt agtgtaggtg 4740
atgtttgcgg ttatatagga tgttgattgc gtgtgtgatt cgtgggcgtg tagatgtaag 4800
tataggttta cgatatattt gtgtataggt tgaagattat aacgggtttg gttagggttc 4860
gtcgagggtt ttcgagttgg cgagtatatc gacggttgga tggggcgacg ttttttgttt 4920
cggtttttaa tttattatta agtttttcgt tttttgtaga cgttttttgc gtttttttcg 4980
tttcgggttg tagcggcgta ggcgtcgggt cgagtcgagc gtcgagtagg gagcgggcgg 5040
tcgcgtttcg ggtcggggtt tcgggggagt aggtgagcga ttcgcgtcgc gcggttttta 5100
cggtttttgg cgaaggcgtt tttcggtcgc gttgcgtcgg cgttttcgtt ttttttgttt 5160
ggtagttttt gttcgttttt gttggtattt tttttttttt tttttttttt gtttggtatt 5220
tttttttttt ttttgtttgg tatttttttt tttttttgtt tggtattttt tttttttttg 5280
tttggttttt tttttttttt tttgtttggt tttttttttt ttattgttag gtattttttt 5340
tttttttttt gtttggtatt ttttttttat ttggtatttt tttttttttt tatttggtat 5400
tttttttttt ttttttggta tttttttttt tttttttttt ttaggtattt ttttttattt 5460
ttgatttttt tcgttttttc gtttggtatt tttttttatt tttattttaa tttcgtattt 5520
ttttttttta tttttttttt gtattttttt tttgattggt agatttttat ttagtttttt 5580
tgtgatatta tttttttttt atcgttgttt ggtatttttt tttttttttt tttttagtat 5640
ttttttattt tttttttatt tgtatttttt tttttttttt cgtttggtat ttttttgttt 5700
ggtatttttt tttttttttt ttgtttggta ttattttttt ttattgtttg ttattttttt 5760
tttgtcgagt atttttttaa tttgtttttg tttggtatgt tttgtttttt attttttttg 5820
tttggtattt tttttttttt ttttgtttgg tatttttttt tttttttttt tatttagtat 5880
ttttttatta tttttttgtt tggtattttt tttgttttta tttggttatc gttttttttg 5940
gatatttttt ttttatttag gttgtttttt tgttttttat ttttttgtag ggtatttttt 6000
tttcgatttt ggattggtag tttttttttt atcgtatttt ttttttttta tttttttttt 6060
ttattttgtt tttcggattt tattagtttt tttattttta gtattttttg tttttagttt 6120
tttttttttt tatttttttt ttttttaatt ttgtttagga ttgatttatg ggtagtagtt 6180
ttttttgttt atatggttta attagatttt gagttatttt taagttttat ttttgttatt 6240
tatatttcgt ttttatagta ttgttttttt tttagttttt atgttttatc gtttgtttag 6300

ggattttatt ttataagttg ggttttgatt taacgagttg gaggtttttt tgttttttta 6360
gtaaggaagg aagtagggta ggataggata gagtaggtat tagtagggat taggagggag 6420
gcgtttaggt tagttggtag ttatggagta ggttgattat ttgtttgtta tttttggagt 6480
tttttttatgt ttttgggttt tagggttatt ttagttatta ttttgatttg tttttttggtt 6540
tttgttttta atgtttagtt tttttttttt aattattgtt atatttttaa aattggtttt 6600
tttagttagg gtttgggttt tgtttggggt ttttgttttg ttttttaagt tttttgtttc 6660
gtaggatttt ggggtcggtt tttgtttgtt ttttatatat attgttttta tttgcgtttt 6720
gttttgtgtt attttagttt tagttggttt atgttggttg tttttatttt aggttttttt 6780
ttttgtatag tgttttggtt tttttagatt tttatttcgg ggagtttggt tttttcgtag 6840
tttttcgagg tttttttgaa tttacgttgg ggtttagggt ttgatttttt tttttaga 6898

<210> 240
<211> 6898
<212> DNA
<213> Artificial Sequence

<220>
<223> chemically treated genomic DNA (Homo sapiens)

<400> 240

tttgaaagga gaggttagat tttgagtttt agcgtagatt taaggaggtt tcgggaagtt 60
gcgaagaagt taaatttttc ggagtggagg tttgaggaag ttaggatatt gtgtagaaaa 120
aagggtttag gatggaggta attagtatgg gttagttggg gttggggtgg tataaggtag 180
ggcgtaggtg ggagtagtat gtgtgggaag tagatagaga tcggttttag ggttttgcgg 240
gataaagaat ttgaaagata gagtagggat tttaggtaga gtttaagttt tgattggaga 300
ggttagtttt gggggtgtgg taatggttgg agggagagag ttgggtattg gaggtaaagg 360
ttagagggta ggttaggatg gtaattggga tggttttgga gtttaggggt atggagggat 420
tttagaggtg gtaggtaggt agttagtttg ttttatgatt attagttggt ttaggcgttt 480
tttttttggt ttttgttagt gtttgttttg ttttgttttg ttttgttttt ttttttgttg 540
gaagagtagg aaggttttta gttcgttggg ttaggattta atttatgaaa tgaagttttt 600
gagtagacgg tggggtatgg gagttaagaa gagagtagtg ttgtaggagc ggggtgtggg 660
tgatagaggt ggggtttggg agtggtttag ggtttggtta agttatgtga gtaaaagagg 720
ttgttgttta tggattaatt ttgagtaggg ttgggaggaa aggggatgag gaaggaggag 780
gttggagata gggagtgttg gggatgggag agttggtggg gttcgggaga tagagtggga 840
ggggaggatg aggagaggaa gatgcggtag gggaggggtt attagtttag ggtcgggggg 900
gaggtgtttt gtaagaaggt gggggataga gggataattt gaataggaga ggggtattta 960
gggaagacgg tggttaagtg gagatagaga aggtgttagg taggggaatg atgggaaggt 1020
gttaggtagg ggaaggaggg agaggatgtt aggtagggga aggagggaag gtgttagata 1080
gggggagtag gaagtagagt atgttaggta ggagtaggtt gagaaagtgt tcggtagggg 1140
ggaggtgata ggtaatggga ggaggtggtg ttagataggg ggaggagggg aaggtgttag 1200
gtaggagggt gttagacgag ggaaggaggg gagatgtagg tggagggagg ataggaaggt 1260
attggagagg gaaggagggg aagatgttag gtagcgatgg ggaggaggtg gtgttataga 1320
ggggttgaat gggagtttgt tagttaggga agaggtgtaa ggaaggggta ggaggaagag 1380
gtacgaggtt ggggtggggg tggagggagg tgttaggcgg ggggacggaa agagttagga 1440
gtggggggag atatttggaa aggaggaggg gaggaagatg ttaggaaggg agaaggggat 1500
gttaggtagg gaggagggga agtgttaggt gaggagaagg tgttaggtag gagaaggagg 1560
ggggatgttt ggtagtggga gaagggaagt taggtagggg gaggaggggg aagttaggta 1620
gggggagaag ggatgttagg tagggaggag aagggatgtt aggtaggggg aggaggggga 1680
tgttaggtag gggggaggag gaggaggagg atgttaatag aggcgagtag agattgttag 1740
gtagggaagg cgggggcgtc ggcgtagcgc ggtcgggagg cgttttcgtt aagggtcgtg 1800
ggggtcgcgc ggcgcgggtc gtttatttgt tttttcggga tttcggttcg gagcgcggtc 1860
gttcgttttt tgttcggcgt tcggttcggt tcggcgtttg cgtcgttgta gttcggagcg 1920
ggggaggcgt agggagcgtt tgtaaagagc gaggagttta ataatgagtt agggatcgga 1980
gtaggaagcg tcgttttatt tagtcgtcgg tgtgttcgtt agttcggagg ttttcggcgg 2040
gttttggtta gattcgttgt gatttttagt ttgtatatag atgtgtcgta ggtttgtgtt 2100
tgtatttgta cgtttacgag ttatatacgt agttagtatt ttgtgtggtc gtagatatta 2160
tttatattag gattatatac gcgtatgtac gtgggcgcgt atatataggt atataaacgg 2220
ttgtttttat atagatttag ttagattatt ttatgtggtt gtagatatta tttagacgag 2280
gtttcggata tttatgtgta ttttttattt ttttaaaacg tatatatttt gggaggtttt 2340
gggtttatta agttttaggg ttatgttttt attaatgtta attttttttt tttatgtatt 2400
taatatttaa atttggtagt ttggagaagt ttatttttag tttttagtta gataagttta 2460
gttttggtta ttttatgggt ttttttaggg tttcgtttta tacggttgag ttattatagt 2520
gtttagtata gagggggatt ttagtttatt tgtagagtga aggagtttta gagagtttag 2580
gtaacggcgg gggaagtggg agtatttttt taagtagtta gcgggtaggg agtttgttaa 2640
gtatttgggg ttaggtgggt ttcgtaggtt tttagtagga ggtaggtagg tagtagggat 2700

ttatggtttg aggttgaggt taaggttagg tttttttttt ttaggttttc gtttttgttt 2760
ttgtcgatat ttagtttttt ttttaggtta ggttatattt ttaatattag gggtattttg 2820
gggtttgggt tttgtattgt ttgggtttgt ttttgttatg ggtaggttta aggtagagat 2880
agagtttttt tatttttttt ttttttggta tttttttatt ttttttttat ttgaaaatat 2940
ttttgtttta tttttttaat tttttttttt tgagatgaag ttttgttttc gttttttagg 3000
ttggaatgcg atggcgcgat ttcggtttat tgtaattttt gttttttggg tttaagagat 3060
ttttttgttt tagtttttta agtagttggg attacgggta tttgttatta tgtttggtta 3120
atttttgtat ttttagtaga gacggggttt tattatgttg gttaggttgg tttcgaattt 3180
ttgattttag gtgatttgtt cgtttcgatt ttttaaagtg ttgggattat aggtgtgaat 3240
tattatattc ggtatgtttt aaatttttaa ttgtgataaa ataaatataa tgtaaaattt 3300
attattttag ttattttgta gcgtatgtta ggttttgtaa agtattttat attgttgtgt 3360
aattagtttt tagaattttt tttattattt taaattggag ttttgtattt atttaataat 3420
aatttttttt tttttttttt tgttttttgg taattatatt ttattttttg tttataaatt 3480
tgattatttt atgtatttta tataatgttt ttgtggttgg tttattttat tttatataat 3540
gtttttaagg tttatttacg ttgtaatacg agttaggatt tttttttttt ttaaggtcga 3600
aatgtatttt attgtgtgta tatatttttg ttgttgttat tgtattagag atgggatttt 3660
attttgtcgt ttatgttgga gtgtagtggt acgatttagg tttattgtag ttttggattt 3720
ttgggttaaa gtaatttttt agttttagtt ttttaagtag tgggattata ggtgtatatt 3780
attatattag gttagttttt gtattttttg tagtgatggg ttttcgttat gttgttaagt 3840
ttggtttcga atttttgagt ttaggcgatt tatttagttc ggtttttcga agtgttgggt 3900
ttataggttg gagttattgt atcgttgttt atttatttat ttattgtggg atatttggga 3960
tgtttttatt atttggttgt tgcgatttgt gtttttattt tttttttaaa atacgttgat 4020
attaaatagg agggaaattg attttggttt ggtttaagga agacgttttt agttttttta 4080
cgtttttttt ttcgcgtttg tgtttttatt tttaggattt ttattttagg tatttttata 4140
tttttggtta tttaaaggat tcgttatgga tattttaatt atatttgttt atttaatgtt 4200
ttttattaaa attgattttt ttattcgttt ttatgaaggg gtttgagttt atggtaagga 4260
ggagtttttg ttgttttttt tggggatatt tttttttgaa cgaggggttt ttggaggtgt 4320
agtgttttat aattttagga aataaatttg gaggtagtgt ttggatagta ggtgtggtag 4380
ggaagggaga ggtggggtgg gtattttgag tggggagagt attgtttagt gaattgggag 4440
aggttgagag tttttgaatg gtgggttttg gtgtagtttg aaaggttttt tgggatttag 4500
attttggtgt tcggaggtta gcgttggttt tatttttttg ggtcgaggat aatttgtgta 4560
taggtagtgt ttgttttttt tttattattt gaatggtttt aatgttgttt ttttttttta 4620
ttgttattag ttattaattt tatagaataa gggtggaaaa gtttttttaa tgagataggt 4680
agttagtttt aaaagtgagg agggtagtga ggtggtaggg ttttgttata gggattggta 4740
gagcgggttt agagggagag taggtagtta agatatagtg gggagtcgat gggtatagat 4800
aatattagat agataggttt ggttcgggtg gtgtagggta gagtagttag atgggtattc 4860
ggtttttttt gattttggtt ttatatttta tagttttttt tttttttttt tggttttgag 4920
agtttggatt ggggataagg ttattaagga taagtttgtg tttttttagg tgttagggga 4980
gggatataaa tttagggcgg taggtttatt ttagtattta aatttgattt ttaaatattt 5040
ttttttttat ttattttttt ttttgagttg tgtttttgtg aggataggat tggggtttat 5100
tagtttgaat attttagggt ttagtatagt gtttggtacg gagtaagagg aagattataa 5160
gtcgtggtgg gtgtaggtgg gagttttggg agggtggggt gttttgggga ttagggagat 5220
taggtagggg ttagattggg ttatagggtt aggggttacg tttaggtatt tagttattta 5280
gttattcgtg agcgttcgag gttgtttatt gtagtacggt ttgtatagta agaatttaga 5340
aatggtttgt atggttatta ataggtgata gatgatataa attttgtgat agttgtaaat 5400
ggtgatagga taaatttatt atttaatgaa tgttattaga taatggggaa attgtttata 5460
tattgatttt agaaggattt taaagatttg ttaaatgaga aaaagtaaga tgtgtaaata 5520
gtatataaag tatgttttta tatgtgagtg tgcgagtatg tgttgtgtga atatgtgtat 5580
gtatacgtgt gagtgtgtgt atatgtggtg tgtgtatatt tttttagaaa aatgtagaag 5640
aaattggtta gagtggttgt ttttagtgag gttggaggat tgggttgggg gaagattgat 5700
tttttattgt gtattttttt gtatattttt gaattttaaa tattttattt atttaaaaaa 5760
taattaaata tttataaaaa ttattcgtat tattatttag atttttaata tattttgagt 5820
atttgttata taaaattaga cgtatagtta tgtgttttta attatgaatt tatacgtgat 5880
tttttttttg tttatttttt tatttatttt gtattttttt ttttaaattt ttattattaa 5940
tatgtgtttc ggtaataggg gtgaggatga atgagatgtt atttgattta cgggggaatt 6000
aagattttta aataattggt aatattagtt agaaggggtt agtgtcgtta tagaggttag 6060
gttaaatgtt agaggagaga tggtagaatg ggatatgtta ggggttgggg gagggggggt 6120
aatagtgtaa tgaggatatc gagttttagt tttataggat gaggagtatt ttgtgggtag 6180
atggtagtgg tggttgtata gtattgcgaa tttttttagt gttattgatt tgtatattta 6240
gaatagtgaa gatggtaaat tgtatgttat gtgtatttta ttaagtttta taaaaataat 6300
aagtgaaggt tgggcgtagt ggtttatgtt tgtaatttta gtattgtggg aggttgaggc 6360
gggtagatta tttgaggtta ggcgtttgaa attagttcgg ttaatatggc gaaatttttt 6420
ttttattaaa aatataaaaa ttagtggggt atggtggtat atgtttgtaa ttttagttat 6480
tcgggaggtt gaggtaggat aattatttga attcgggagg tggaggttgc ggttagttat 6540
gtgttattgt attttagttt gggcgatagt gtaagatttt attttaaaga aaaaaaaaaa 6600
aagggaaaat atgttagaag gatggaaaga gtgtttattt atttattatt tgttgggttt 6660

tgtggtaagt gtttgatttt ttaatggttt tgtagggtgg atattattat ttatatttat 6720
agacgatagg atatagagtt tagggagatt tgtgatatgt ttgcggttat ataattagaa 6780
agtggtagag ttgggattta aatttatatg tgttagattt tttttttttt aattggtagt 6840
ttttggggaa gggagtggga gttggagggt ggggatattt tatagaagag gtagtttt 6898

<210> 241
<211> 8067
<212> DNA
<213> Artificial Sequence

<220>
<223> chemically treated genomic DNA (Homo sapiens)

<400> 241

ggtgtttttt aattttttaa atttttggta gagaaagaat taagcgaaga aaatgagtta 60
tttatttatt tattttataa gtattgagta tttaaatgtg ttgtgtattg ttttagatgt 120
taaaattttt attttgatgg gaaaaaaaag acggttgggt gcggtggttt atgtttgtaa 180
tttagtattt tgggaggtcg aggcggtgga ttatttgagg ttaggagttt aagattagtt 240
tgattaatat ggtgaaattt cgtttttatt aaaaatataa aattagtagg gtgtggtggt 300
atatgtcgta attttagtta tttgggaggt tgaggtagga gaattatttg aattcgggag 360
acggaggtcg tagtgagtta agatcgcgtt attgtatttt agtttgggta ataagagcga 420
aatttcgttt taaaaaaaaa aagaaaagaa gaagaaaaag ataaaataaa ataaatttat 480
aagaagtata gtatgttagg tagcgataag tgttatagaa aaagtaaagt ataggttatg 540
aatagatatc gggggttggg gaatgagatg taattttaaa aaggttagga aatatttatt 600
aagaaggtga tatttgagta gaaatttagt gagaaagtgt attagataat gttattgttt 660
tatttaggtt tttttttttt tttttttgtc gtttttgggt tatttttttt ttattatagg 720
gtgttttatt tttaatattt aaaatttgta gttttttttt agaggattgt ttagagataa 780
tatatagcgt ttttttagta ttgtattcgt ttaatttttt tattataatt ttaaaggtag 840
ggaagaaatt aggttggagt tgttggacga agttagaaaa agaatgtatt gaaatttttt 900
ttgaggtttt tgtgtatcgt attttaattt tttatatgtt tttgatggat atatatatat 960
atatatatat aatttttttt tttttgagat agagtttcgt tttttcgttt aggttggagt 1020
gtagtggcgt tattttcgtt tattgtaagt ttcgtttttc gggtttacgt tatttttttg 1080
ttttagtttt tttaatagtt gggattatag gcgttcgtcg ttacgttcgg ttaattttgt 1140
ttttgtattt ttagtaggga cggggtttta tcgtgttagt taggatggtt tcgatttttt 1200
gatttagtaa tttgttcgtt tcggtttttt aaaagagttg ggattatagg cgtgaatatt 1260
ttttaataag taattttatg tatatttttt taagtattat ttaagttgtt tgtttacggg 1320
gtagacgatt ttttttttag tttttttttt gaatatgttt tttaatttta ttaattttta 1380
tttatgagta taataatatt ggaaaaattg gatagagtat tatttgtttt tcgttttgta 1440
gattagagat gtttttagtt tttgttggta tatttaggat gggggttacg ggaagtttaa 1500
gaatttagtt tttttttaaa tgttaattgt tttttagtaa ttataggtta gggattttat 1560
acgtttattt tttttttata gtgatttata atatttatcg ggtgtttatg tatttaaaga 1620
agaggttatt tattttattt ggtaaattaa tatatttatg atttagtgga aagagttttg 1680
gagtggtata gagggttgtt ttaatatata tttattgagc gtttaataat gaatgggtta 1740
agtttttcgt aggtattggt aatgggaaga tgattaatat agacggttga attttgtttt 1800
taaggttttt ataaagtatt ggagatgata aatacggttc gatataaagg gtcgggttta 1860
ggattttaat tattattatt aaagagttac gggaatataa aggaaggatt attagggggg 1920
aagttttagg agggtattat aaagagattt ttgagttgga tttagaagga tagatgagtt 1980
taagttgaaa gtggggacgg tcgttttcgt tggtgggaat cgaaggagtc gaggtagcgt 2040
tgaggcggag attaggtggt attcgcggcg ttgtaaggat atataagttg ttgcgagggg 2100
gttaattttt cgggttttgg tttagaaatt tgggacgatt atattttaaa agcgtttgga 2160
aaaattgtag aattcgtgtt gattttcgtt aaagttattt tttcgtcgtg agggtaggaa 2220
gttttgcgag gcgtttgcga ggacgtaaac gtcgcgaagt aagtttttta cgtttttatt 2280
ttaggtttag tgaagtgtag attttgattt atagtttaaa ggaaagtaag atcggagtcg 2340
gggagggttt ttttaggagc gggtttagcg ggagtttggt cgaattgttt gttgttatag 2400
tggtttcgcg gtttcgcgtt gagcgttatt cgtcgttttt tatcgcggtt tttatcgttt 2460
tttcgtaagg ttcggtattt ttcgttattt tttcggtcgt tgcggcggtt tcggtttttt 2520
atagtcgtta aatttaggtt tattttgtgt taggtcgggt tttttcggtt tttttcgtcg 2580
tgacgaatta gcgttcggtt gggaaagtat taaaattcgg ggagttttga ggatttaatg 2640
ggttttgaaa aaaatttttg agtgtgttaa gtattatcgt atttttatta gaaggttagt 2700
ttttttaaag agggtcgggt ggggttcgga aaaaaaaaaa aattaagaag agtgtggaaa 2760
gtgcgagtgc ggaagtttcg gacgcgaggg gcggggcgag cgcgggataa agggaagcga 2820
agtcggagtt gcgggcgttt tttttgttcg cgggtgagtg tttttaagtg ggacggatcg 2880
ggtcggtggg tgtaagtcgt ggtgttttcg tttggttaga tttcgagttt tcgggggttg 2940
ttggggttta gttttggtcg tggggttaga gttaggagtc ggttgaggtt tttttgaggt 3000
ttagtagagc gagggttggg agggagtttt atttggggtt ttttttttgg aggtaaggtt 3060

agtttttcga aattggttaa agtatttgtg aatatttcgt tttaggggag ttgttagtaa 3120
agtatatttt ttaagataaa gagagcgatt ttaggaagtc gtgggtaagg taggttcgtg 3180
gtttattttt tattcggtta gaaggttttt ttattggttt aggtagcgat ttgaggacga 3240
gtagagtttt gaaatagttt ggtttgggtt tgaaatttaa ttttggtatt ttttggttgt 3300
atatggtttt agataagttg tttaaatttt tcgagatttt tttgttttat ttgtaaaatg 3360
gggatagttg tatatataaa gtatttgtga gtgttaagcg aaatagtgtt ataagcgtat 3420
ttcgtataaa gtattttttg aataaatatt tatcgggtat ttatttttat gttaggtatt 3480
aaagatggat ttaacggtgg ataaatgtat aagtttattt ttaaattttg tattttagta 3540
aaagagagta ataattttaa agtattaata atgaagtata acgtataggg agtttatgtt 3600
agttgttagg gttttttgta gcgaggtttg ttggatgttg atagtttttt taatgtgagt 3660
agaaaggaaa tttatattag agttaaaggt ttttggttgt tttttaagag ggaaagattt 3720
aggttggatt attgagaaaa tgagttgttt ttttttatat taggatttat tttttatgtt 3780
ttagggaatt tttttaaaat taataataga agcgaaaaat tagatttatt ttatatgttt 3840
ggttgtaaat atttttaata gttatagaag tttatttgtt ttttttttaa agatgaattt 3900
ttgttttttt gtattttatt atttgtgatg tatgttgatg taaagaataa gtttatatgt 3960
gtttggttat gttgtgagtt ggtaatatta ggaaaaaatt ttttaagaat tgagttaggt 4020
tttgatatgg tttttttttt taagtagtat ataatttagt tatattgata agttaaaaaa 4080
aagttataat aatatagatt tgtgtaaatt gtataggaat atagatgggg gagtagttaa 4140
ttttgtttag gtaatgtatt aagaaagttt ttttgaggag aaaaattttg aaatgggttg 4200
tgaatgatga ataaaagttt tttaggtaga tttttaaaat ttatttttag tatttatgaa 4260
gttatttatt attataagag aatgtgaagt ttattgttat attaattatt ttttgtattg 4320
tttttaggaa taaaatgttg tttaatttta ataaaaattg ttattttaaa agtatgatat 4380
agttgttgat tagggagttg ttagtatttg gtaggttttg ttgcgttgga ttttgataat 4440
atattatttt ttatttttat ttttgtatta gtttgatgaa atgtttatat ggcgttagta 4500
aaatgttttt tatataagag gtagtttagt attataagaa aaaatttgaa agtatatttt 4560
taaatatttt tagtatttta ttttagttat ttaattttaa ttaattaaat gatttataag 4620
aattagttaa aatatataaa aggtttaatt attaggaatt aaggtgaagt aattttattt 4680
agaaatatta attaaattat tttatttatt aggaaaatat tttcgtatgt ttgttgagtt 4740
ttgatttttt tttttttttt tttttttttt tattttagtg ttttagattt atttttaagg 4800
aattgagaat ttaatttttt aaaatgttaa gtaagtttta atttttatat ttattaattt 4860
atgttaataa gagattaatg ttttagtaag tgggaatttt aaaatatttt tatttttttt 4920
tttttaaaat aggattattg gtagttttgt tgtatagtat tatattttag ttgtttataa 4980
gaagttattt attttttttt aaatagaaag attattttat gttttatttt ttattttagt 5040
ttttgtaata gtaagtttta atttttttgt taatgtaata gttaattttg gttttgatat 5100
aaaaatttag atgtttttta gtgatagtta tttaattatt atagtaatag gatgaaattt 5160
agtttaaaag aaaaaaaaaa aagtattttt tattaaataa aataattatt tgtatttttt 5220
tttgaattaa ttttaatatt taaagaaata gtattgtaaa attttaattt tttgtgttta 5280
gtttgttttg tttttttttt gtaaatggga aataagtgaa agttttatta tttttttttt 5340
attaattttt attattaaga gtttttaagt tgttattgag tgaatgttta ttttggttat 5400
tttgttaaat ttaattttta attggttatg tttttttttt tttttttgtt tttttttaaa 5460
atttttatat taaataataa agtttttgaa attatttatt gtaaatattt ttaagttgtt 5520
taagtaaaga ttttattatt tatggggtag attttgttat tgaattttat tttattttat 5580
aaattatatt ttattggttt ggaagtatat atgttttaag tagttgtgaa cgtgtttttt 5640
tatttaggaa gttttggttt agtttggtta gtatgtaaaa taatttttta atttataaaa 5700
tagatttagt agtaattgtt taggttttgt tgaaatggtt tttttgttgg ttttttggga 5760
gggggggttt tgtttgtttt taaaaaaata ttgttagtat gattaaattt tataaaatta 5820
gttttttaat attaattttt ttttttatat ttttattatt taaattttag gttagtttat 5880
tttttattat taattaggtt tttgtgtaat ttagtttttt tcgttaaaaa attatttata 5940
tttattaatt tttagatata tattttagtt atatttatta ataatttgtt tttttttagt 6000
gagatatttt agaaaagaga tatcgtatta tatgttttta ttttaattta tgttatatat 6060
aggttttaaa gttaatttta gtagattata aagttttatg tttgttttgt tttttttttt 6120
aaatcgaaga atatttgtta tgattatttt tatggtattt tgttagtggt tatataggtg 6180
gagttgaata attgagattt tgattttaaa gagttaatta tgtattttag ttttggaaat 6240
ggtataatta taatgttttg ttttttattt tttttttttt agttttttta tttttataat 6300
attattgttt tataatgttg aatagtgatt tttattattt atgttatttt tgaaggtgtt 6360
attttgataa aaatattgtt tttgataatg tattattttg ataaatttga ttttttgaga 6420
ttaaaatatt ttaaaaatta agtgtttttt tataatttat tatgtataaa aaggaatttt 6480
ttttgttttt ttttagttat atttttgtgt tattatagtt ttatattaat atataaaata 6540
tagaagatta gggattaaaa tatttaatag aatggatatt aaatgaatta tttttttttt 6600
tttttttttt ttattatttt tattattatt attattatta ttattattat taatattatt 6660
attattatta tttttgttat ttaaatgtat cgttgtaagt aatatatatt tttttttatgt 6720
ttttgatatt ttaattgtgt tttgtttatt taaatatgtt ttatttgcgt tttttttaat 6780
tgtatagatt agaaattgat tagtcgtttt tttttttttt aaagagtatt aaatgtaata 6840
tttgtttaaa tgtttttttt ttgggtatta aggatatgtg tagcggtatg ttttaggaaa 6900
ttgtgtttat tttgtaatag ttatagttta gttggaatgt gttaagtgta ttttatatta 6960
tataattaag gatcgatatt gttaattgta tgtaaaaatg aaattgttat atattttgat 7020

tgaattttat aaggtaaaat attttaattt tggttttttt ttattatatt ttttttatgt 7080
attatgtttt ggtttttatg aatattgagt gttattgtat tagttttaag gttattgtta 7140
tataagagtt tatttgtgat tatattaata tataatttat aaaaattaag ggttttgttg 7200
ttgaaatgag tagttgtatt ttgaaagggt gagagttatt aggaaattag atgaaaaaag 7260
aagtttataa attagaaaag tagtttatat tgagataaaa tgtagaaatg tttttatgat 7320
tgattagtgg aagatggatt aaagaagaaa tggaagagaa attttataaa agaatgattg 7380
ggttaaaaag attagatagt attttagaat gaaatttatg tattttttat aagtttttgt 7440
tttaaattat gtatgtgata tttattaaat ttaatatatg ttttttttaa ttgataaatt 7500
ttggtttttc gatttgagtt tttatttatt ttttatatta tataaaatga tttaaaattg 7560
tcgttgaaat tagatttatt tttttttttt gtttttagta gataaatata ttggtggtat 7620
ttatatgtta aataaattgt attttgtttt gtatgtagtg tttttagagt tttagttgat 7680
tatattttaa gtttttttat tttagttttt atatattgtt aagttgtttt attttattgt 7740
tgattataaa gtaattttga atgttttaaa tattttagtg tgttgtttat tttaaaaaat 7800
aaaatatata tttatagtta tataaatatt tttagttttt gtaaaaaaga aagagaaaaa 7860
taattaaaag atattgtaga taggtttttt aatgaaagaa aaatatttaa tttttttagt 7920
ggaaaaaaag aataattata atatattatt gtttgtttaa atttaggttg tttttgtatg 7980
ttatatgtag attatttttt atttttgtgt ttttttcgtt tgagtttatt ttttattcgt 8040
gtttttgttt tatttgtttt tattagt 8067

<210> 242
<211> 8067
<212> DNA
<213> Artificial Sequence

<220>
<223> chemically treated genomic DNA (Homo sapiens)

<400> 242
   gttgatggaa ataaatgaga taaaaatacg aatgagaaat gagtttaaac gaggaaaata 60
   tagaaatgaa aaatgatttg tatatggtat gtaaaagtaa tttgaattta aataagtaat 120
   gatgtgttgt aattgttttt tttttttatt gaggaaatta aatatttttt ttttattgag 180
   aaatttgttt gtagtgtttt ttggttgttt tttttttttt tttttgtagg ggttgggggt 240
   gtttgtgtga ttgtgaatgt gtgttttgtt ttttgaggta agtaatatat tgaagtgttt 300
   aaaatattta ggattatttt atggttaata ataggataaa ataatttagt aatatatgaa 360
   gattgaaata ggaaggtttg aagtgtgatt aattagaatt ttgaaaatat tatatgtaaa 420
   ataaaatata atttatttga tatgtggatg ttattaatgt atttatttat taaaaataaa 480
   aaaaagaaat ggatttgatt ttaacgatag ttttaaatta ttttatataa tgtgagaaat 540
   gagtagagat ttaaatcggg aagttaggat ttgttaattg aaaagaatat atgttggatt 600
   tgatggatgt tatatatatg atttaaaata aagatttgta agaaatgtat ggattttatt 660
   ttggggtatt atttgatttt tttaatttag ttattttttt ataggatttt ttttttattt 720
   tttttttgat ttatttttta ttggttaatt atgaaaatat ttttgtattt tgttttaatg 780
   tgaattattt ttttagttta tagatttttt tttttattta gttttttgat agtttttatt 840
   tttttaaaat ataattgttt attttagtaa tagaattttt ggtttttgtg aattatgtgt 900
   tgatgtaatt ataaatgggt ttttatatag tagtagtttt ggggttaata taatgatatt 960
   taatgtttat gagaattaag gtataatgta tagaaagaat atagtagaga gagattaaaa 1020
   ttagggtatt ttattttgta aaatttaatt aaaatatgtg ataattttat ttttatatgt 1080
   aattaataat atcggttttt aattgtataa tgtaaaatat atttaatata ttttaattaa 1140
   gttgtagttg ttataaaata aatataattt tttaaaatat gtcgttgtat atatttttag 1200
   tatttaaaag aaagatattt aagtagatat tatatttgat attttttagg agaaaagaga 1260
   acggttgatt aatttttagt ttatatagtt agaaaagacg taaataaagt atatttaagt 1320
   aggtaaaata tagttgaagt attaaaggta tgaaagaagt gtatattatt tgtagcggta 1380
   tatttgggtg gtagaggtga tagtggtgat agtgttggta gtggtggtga tggtggtggt 1440
   ggtggtagaa gtaatggaaa gaaaaaagga agaagaataa tttatttagt atttattttg 1500
   ttggatgttt taatttttag ttttttatat tttgtgtgtt aatgtgaaat tataatgata 1560
   taaaaatata attaaagaaa ggtaaaaaaa attttttttt atgtataata aattataagg 1620
   ggatatttgg tttttgaaat attttaattt taaaaaatta agtttgttaa gatgatgtat 1680
   tgttagaggt agtatttttg ttaaaatgat atttttaaag gtagtataaa taataagaat 1740
   tattgtttaa tattatgagg taataatgtt ataaaaataa gggaattaaa aaaaaaaaaa 1800
   tggaaagtaa aatattataa ttatattatt tttaaagttg aagtatatgg ttagtttttt 1860
   gaggttagaa ttttaattgt ttagttttat ttgtatagtt attagtagaa tgttatggag 1920
   atggttataa taaatgtttt tcgatttaag aaagaaaata aagtaagtat aggattttat 1980
   agtttattgg aattaatttt agagtttgta tatgatataa attgaaatga gaatatatgg 2040
   tacggtattt tttttttaaa gtattttatt aaagaaaaat aaattgttga taaatgtaat 2100
   taaaatatgt atttgagggt tagtaaatat gagtagtttt ttagcggaga aaattaagtt 2160
   atataaggat ttaattagta gtgaaaagta gattagtttg aaatttaggt ggtgggggta 2220
   tggaaggaag gattaatatt aaagaattaa ttttgtagaa tttggttatg ttaatagtgt 2280
   ttttttaaaa ataaataaaa tttttttttt taaaaaatta ataaggaagt tattttagta 2340
   agatttgaat agttattgtt aaatttgttt tgtgggttgg aaaattattt tatatgttga 2400
   ttaagttggg ttagagtttt ttgggtgaaa aaatacgttt ataattattt aaaatatata 2460
   tatttttaaa ttaataaaat ataatttatg gagtggaata agatttaata gtaagatttg 2520
   ttttatagat aatgaaattt ttatttgaat agtttgaaaa tatttataat gagtagtttt 2580
   agaagtttta ttgtttaata tgaaaatttt agagagagat aagaaaggag agaaaaatat 2640
   aattagttga aggttaggtt taataaaata gttagaatga gtatttattt agtaataatt 2700
   taaagatttt taatagtgaa gattaataag aaagaaatgg taaaattttt atttattttt 2760
   tatttataaa gaaaaaataa aataaattga gtataggagg ttggagtttt atagtattgt 2820
   ttttttaaat gttggggttg gtttaaggaa agatgtaaat aattatttta tttagtagga 2880
   agtgtttttt tttttttttt ttaagttgaa ttttattttg ttgttgtgat gattgagtga 2940
   ttgttattgg aagatatttg ggtttttgta ttaaggttag agttggttat tatattaata 3000
   agaaaattaa aatttattgt tgtaggagtt ggggtgggag gtggggtata agatggtttt 3060
   tttgtttgaa agaaaataaa taattttttg taaataattg agatataata ttatgtaata 3120
   aaattattaa tggttttgtt ttgaagaaaa aaaaatgaga gtattttgga atttttattt 3180
   gttagggtat tagtttttta ttaatataga ttgataaata taaaaattga aatttatttg 3240
   atattttgga agattaagtt tttagttttt taagaatgaa tttgagatat taaaataaaa 3300
   aaaaaaggaa aaaaaaaaga gaattaaaat ttagtaagta tacgagaatg tttttttaat 3360
   gaatagggta atttaattgg tatttttgag taaaattatt ttattttaat ttttagtaat 3420
   taagtttttt gtgtatttta attagttttt ataggttatt tggttgatta gaattaaatg 3480
   attaaaataa agtattgggg atatttagaa atgtattttt aggttttttt ttgtgatatt 3540
   aagttatttt ttgtatggaa aatattttgt tgacgttatg taaatatttt attagattga 3600
   tataaggatg agaatagaaa gtgatgtatt attagggttt aacgtaataa ggtttattag 3660
   atattgatag ttttttgatt agtaattgta ttatgttttt gggataataa tttttattaa 3720
   aattaggtag tattttattt ttggaagtag tgtaaaaggt aattggtatg ataatgaatt 3780
   ttatattttt ttataatggt aaatagtttt ataaatgttg ggaataaatt ttgagagttt 3840
   atttgaaaaa tttttattta ttatttatag tttattttaa aatttttttt tttaagggaa 3900
   tttttttgat atattatttg ggtagaatta attgtttttt tatttgtatt tttatatagt 3960
   ttatatagat ttatattatt ataatttttt tttaatttat taatatggtt agattgtatg 4020
   ttatttggaa agagggatta tgttaggatt tagtttaatt tttaggaaat ttttttttag 4080
   tattattagt ttataatata attaggtata tgtgagtttg ttttttatat taatatatat 4140
   tataaatagt gaggtataaa aagataaaaa tttatttttg gaaagaaggt aaataaattt 4200
   ttatgattat tggaggtatt tgtaattaaa tatgtggaat agatttgatt tttcgttttt 4260
   attattaatt ttaaggaaat tttttggagt ataaggaatg agttttggtg tggaaagaaa 4320
   tagtttattt ttttaatggt ttaatttgag tttttttttt ttaaagaata gttagaaatt 4380
   tttgattttg gtgtagattt tttttttgtt tatattaagg gagttgttaa tatttagtag 4440
   gtttcgttgt aggggatttt gataattaat ataagttttt tgtacgttat attttattgt 4500
   taatatttta aaattattgt ttttttttgt tagaatataa aatttaagag tgggtttgta 4560
   tatttgttta tcgttggatt tatttttggt gtttggtata aaaataaata ttcgataaat 4620
   atttgtttaa gaaatatttt atacggggta cgtttatgat attatttcgt ttaatattta 4680
   taagtatttt atatgtatag ttatttttat tttatagatg aaataaggga gtttcggaaa 4740
   gtttaaataa tttgtttgaa gttatatgta gttaggaagt gttaaagttg ggttttaaat 4800
   ttaggttaaa ttattttaga gttttgttcg tttttaagtc gttgtttgaa ttaatggggg 4860
   aattttttgt tcgggtgggg ggtgaattac ggatttgttt tgtttacggt tttttgaggt 4920
   cgtttttttt gttttggaga atgtatttta ttagtaattt ttttaaaacg gggtatttat 4980
   aggtgttttg attaatttcg ggggattggt tttgttttta aggagggggt tttagatggg 5040
   attttttttt aattttcgtt ttgttaggtt ttaaggaggt tttaatcggt ttttggtttt 5100
   ggttttacgg ttaaggttga attttagtaa ttttcgggaa ttcggggttt agttaagcgg 5160
   ggatattacg atttgtattt atcgattcgg ttcgttttat ttggaaatat ttattcgcgg 5220
   gtagaaaaag cgttcgtagt ttcggtttcg tttttttttg tttcgcgttc gtttcgtttt 5280
   tcgcgttcgg agttttcgta ttcgtatttt ttatattttt tttaattttt tttttttttc 5340
   gggttttatt cgattttttt tagaaaaatt aattttttag tgaagatgcg gtgatgttta 5400
   gtatatttag ggattttttt taggatttat tggattttta aaatttttcg aattttgata 5460
   tttttttagt cgggcgttga ttcgttacgg cgaagagggt cgaggaggtt cgatttaata 5520
   tagaataggt ttgggtttag cggttgtgag gagtcggagt cgtcgtagcg atcgaggaag 5580
   tggcgggagg tgtcgagttt tgcggagggg cggtgggggt cgcggtggga gacggcggat 5640
   ggcgtttagc gcgaggtcgc ggaattattg tggtagtaag tagttcggtt agattttcgt 5700
   tgagttcgtt tttggagggg tttttttcga tttcggtttt gttttttttt aggttgtggg 5760
   ttagagttta tattttatta aatttgggat ggaggcgtgg aggatttgtt tcgcggcgtt 5820
   tgcgttttcg taggcgtttc gtagggtttt ttgtttttac ggcgggggag tagttttggc 5880
   gaaaattagt acgagttttg tagttttttt aagcgttttt agggtgtaat cgttttaggt 5940
   ttttaagtta aggttcggag agttgatttt ttcgtaatag tttgtgtgtt tttgtagcgt 6000
   cgcgggtgtt atttggtttt cgttttagcg ttgtttcggt tttttcggtt tttattagcg 6060
   ggaacgatcg tttttatttt tagtttaaat ttatttgttt ttttggattt agtttagagg 6120
   tttttttgtg atgttttttt ggaatttttt ttttagtaat tttttttttg tattttcgtg 6180
   gttttttgat aataatggtt ggagttttga attcgatttt ttgtatcgga tcgtgtttgt 6240
   tatttttagt gttttatgaa ggttttgaaa gtaaggttta gtcgtttgta ttggttattt 6300
   ttttattgtt agtgtttacg aagggtttga tttatttatt gttagacgtt tagtaagtgt 6360
   gtattgaggt agttttttgt gttattttag ggtttttttt attaaattat gagtatgttg 6420
   gtttattaga tggaatgagt agtttttttt ttggatatat aaatattcga tggatattgt 6480
   gggttattgt ggagggagaa tgaacgtatg ggatttttga tttgtggtta ttggaaaata 6540
   gttgatattt agagagaggt tgaatttttg ggtttttcgt agtttttatt ttgagtgtgt 6600
   taatagggat tagaagtatt tttagtttgt aaagcgagga ataagtgata ttttgtttaa 6660
   tttttttaat attgttatat ttatgaatag agattaatga aattgaagag tatatttaag 6720
   agaaagatta aaggagaagt cgtttgtttc gtggataggt agtttgagtg gtgtttgaga 6780
   aaatgtatat gaaattgttt gttaaaaaat atttacgttt gtaattttag tttttttggg 6840
   aggtcgaggc gggtagatta ttaggttagg agatcgagat tattttggtt aatacggtga 6900
   aatttcgttt ttattaaaaa tataaaaata aaattagtcg ggcgtggcgg cgggcgtttg 6960
   tggttttagt tattggggag gttgaggtag gagaatggcg tgaattcggg aggcggagtt 7020
   tgtagtgaac ggagatggcg ttattgtatt ttagtttggg cgagagagcg agattttgtt 7080
   ttaaaaaaaa aaaaattata tatatatata tgtatatatt tattagagat atgtaaagga 7140
   ttaaagtacg atatataaag gttttagaag ggattttagt atattttttt tttggtttcg 7200
   tttagtaatt ttagtttggt tttttttttg tttttgggat tatggtggga ggattgggcg 7260
   agtgtagtgt tggggaggcg ttgtgtgttg tttttaggta gttttttgaa gaaaaattgt 7320
   aggttttgga tgttaagaat aaagtatttt atagtaggaa aggggtggtt taaaaacggt 7380
   aaaaagggaa aggaggggat ttaagtggag tagtgatatt gtttgatata tttttttatt 7440
   gggtttttgt ttagatgtta tttttttagt aggtgttttt tgattttttt aaaattgtat 7500
   tttatttttt aattttcggt gtttgtttat ggtttgtgtt ttattttttt tgtagtattt 7560
   gtcgttattt aatatattat attttttatg aatttatttt gttttgtttt tttttttttt 7620
   tttttttttt ttttttgaga cggagtttcg tttttattgt ttaggttgga gtgtaatggc 7680
   gcgattttgg tttattacga ttttcgtttt tcgagtttaa gtgatttttt tgttttagtt 7740
   ttttaagtag ttgggattac ggtatgtgtt attatatttt gttaattttg tatttttagt 7800
   agagacgagg ttttattatg ttggttaggt tggttttgga tttttgattt taagtgattt 7860
   atcgtttcgg ttttttaaag tgttggatta taggtatgag ttatcgtatt tagtcgtttt 7920
   ttttttttta ttagggtgga gattttgata tttggaatag tatatagtat atttagatgt 7980
   ttaatatttg tggagtaaat aaatgaatga tttatttttt tcgtttggtt ttttttttat 8040
   taaagatttg aaaaattaag aaatatt 8067

<210> 243
<211> 19236
<212> DNA
<213> Artificial Sequence

<220>
<223> chemically treated genomic DNA (Homo sapiens)

<400> 243

ttaattttta tatttttagt agagacgggt ttttattatg ttggttaggt tggtttcgaa 60
tttttgattt tgtgatttat ttattttggt tttttaaagt gttgggatta taggtataag 120
ttatcgtatt tagtcgtatt taattatttt agatgttatt gtaaatggta ttattgtttt 180
aatttttttt ttaggttatt tattattggt atatagaaat ataacggata atttttttat 240
ttttgggagg gaagaaattg aggtttagaa taagttgttt aaaggtgtat ggttagtaag 300
gtttaattta gggtttttaa ttttgaattt atcgtgttgt ttttttgaag aggttgttta 360
ttgggttttt ttattatttt gttttttttt tttgatttaa tgtttttaat tgtaaaatag 420
gaagtattgg gaaaagaaag aattggagta tagtcgggtg tggtggttta tatttgtaat 480
tttagtattt tgggaggtcg aggtgggtag attatttgag gtcgggagtt ggagagtagt 540
ttggttaata tggtgaaatt ttatttttat tgaaaatata aagattagtt gggtatggtg 600
gtagaagttt gtaattttag ttatttcgga ggttgaggta ggagaatcga ttgaatttag 660
gaggcggagt ttgtagtgag ttgagatcgt attattgtat tttagtttgg gtaatagagt 720
aagattttat ttaaaaaaaa aaaaaattgg agtattgtaa tagttatgtt tcgaattttg 780
ttagtgtaat aattgaatta taggaggtag ttatggtttt agtgatattt atggatacgt 840
ttatattaat tttttttgat tggagggttt ataggtaaat gatagttaat attgaatttg 900
tgattttaaa atgttaaaat attttatgag aatgatttag aaattaagaa gttatttgta 960
ataaattaag tattgaaatg tattgttaat ataggatagt tttttatttt tgagaaaagg 1020
attgtgttag gatatgtagt tgaggttaag atttttgagg aattgggtgg ggtgtggttt 1080
aggtttgttt gggagagaag tattttgtag tttgttatta ggttggagat ttgagtgtgt 1140
taagttattt tgcgtagttt aatagatatt ggtagttttg tatgtttata ggaggggatg 1200
tatgaatttt ttttaggaat ttgaatatta tttacgaacg attgggaaat tttttttttt 1260
tagtttttat tgaatagttt ttttttttta aatgatagga tttttgaatt tttttttatt 1320
tttttttata tttaggagat agtatatcgt tgtagttgtt attaatggga acgtggtttg 1380

atagttgtgg atagtattag tagtattttt ttagtttgtt tttttgttat tttttgttag 1440
gttagtagga tttttataag ggaatattga agtttgatga ataggagtat atgtgtgtat 1500
gagtgtatat atatatatat ataaatatat atatatatag aattgagaat ttatggtata 1560
tttttttttt aagaagggaa gagtattttg gttggaagat agtgttttag gatagtttta 1620
atttattttt gttattttta atgtgaaggt attatatttt tataatgcga atttttttta 1680
tattttttga gaaattttat ataagaaatg ggagagttta tatttttgaa gaattattta 1740
ttgatatttg gagtagagaa ataagggttt gtttgagtat atgtgtaagt ttttttttaa 1800
tatatgaaat attaggaaaa ttttataggg tttcgagatt gttataatta gatattttat 1860
ttttgtttat ttaggaaggt aaagagaaaa gtaaaaataa ataaaataat atatatatat 1920
aattaaatag aaaataagtg tagatagttt ataaagtaaa gtatcgggtg tgttattagg 1980
ttttaaggta gtttttttta tagtttggat ttagatatgt tataatagtt attttattat 2040
gttatagaaa gaaataattg aagtaggtta taatatttta aattattttt ttagtaggaa 2100
tattatgttt tgatgatata gtttagtttt atttttttta taagttatta gagatttttt 2160
ttaaaatggt tttttgtttt tagttttttg tattttatgt tatttttatg ttgttttatg 2220
tattagtttg ttttcgtatt gttataaaga aatatttgag attggttagg tgtagtggtt 2280
tatatttgta attatagtat tttggaaggt tgaggtgggt ggattatttg aggttaggaa 2340
tttgagatta gtcggggtaa tatggtgaaa tttcgttttt attaaaaata taaaaattag 2400
tttggcgtgg tggtatgtgt ttgtaatttt agttatttag gaggttgagg taggataatt 2460
atttgaattt gggaggtgga ggttgaggtg agtcgagatt atattgttgt gttttgagat 2520
tatattattg tgttttagtt tgggcgatag agcgtgattt cgtttgaaaa aaaaaaaaaa 2580
tattttagat tgggtaattt ataaagaaaa gaagtttaat tgggttatag tttagtaggt 2640
tatataggaa ttatagtagt ttttgttttt ggagaagttt taggaaattt ttaattatgg 2700
tggaaggtaa agggggagcg agatatttta tatggtagga gtaggaggaa gaagcgggga 2760
ggtgttaaat tttttaaata attttcgtga gaatttattt attatcgtga gagtagtacg 2820
aggaggatgg tgttaaatta tttatgagaa attgttttcg tgatttagtt attttttatt 2880
aggttttatt tttaatattg aggattataa tttaatatga gattgggtgg ggatgtagat 2940
ttaaattata ttatttaata ttaattagta aagattaata ttattttttt agttaaaatt 3000
ttgagatttt tttattatta agagaattga tttttaattt tgtgtgttat ttgaggtttt 3060
ttattaatta gtttttttat ttatttatgt ttttatttta atttttttta tttatatttt 3120
agttttggat ttaaaattta ttttttttat ttttatttat attgattttt gttttttatt 3180
tttatagtat atttacggta tattttattt tggtagttta tttattaagt gattattatg 3240
tatgtttaag tgaattagat gttttgtgaa tattaagaga atgagataag gtttttgatt 3300
tgaaggaggg aacgttgttg ttttgttatg ttatgttttt tgagagtaag ttatgtattt 3360
ataggaaaat ggaagtttat aggttttttt aggatgtttt tgtgttatgt atatgttgga 3420
attttagaaa gatttgttaa attgaattaa attttttttt tttttttaat tgatatagtt 3480
gaagtgtaat aattaatatg tatttaaatt gtttaaagta atagaattag tttattatcg 3540
ttaggttatg agtaagtttt ttaggggtaa gaaaggaaaa tttattttgg tgtgtagttc 3600
gattagtaat aaggaaaaag ttgtttagtg taagtttatt tttttgtttg gagttttgtg 3660
gttatgttgg gttatatttg gttgtgtagg tgtaaattgt tgaagtggga aattaagtgt 3720
ggtgattaaa agtgtgatgt ttaaaggagt gaggaaaggt aaaatgagta gagtggggaa 3780
gaggagtagt agaaattttt tagagtagat gagtagtagg gttattaaag aaggattggt 3840
ttttttttta tataattaag gttagttttt gggaattata gtttagattt tatatatttg 3900
tagtagtaaa ggtttgatgt tggtaagttt attttttaga tatttgtttt cgtaggtaat 3960
agtagttaga gtagttgtcg tgagtgattt tgaggtcgtt ttttttaaag gtttcgagtt 4020
ggaaggattt ggagaagcgt aaattttaaa tttaggtttt tttttatggg gtttcgggat 4080
acgttggaaa atttaattaa agtaggaaaa attggcgggt ttggagaacg gggtagagat 4140
ggaatagaag ttttcgttga ttttaggtat tttttgtttt ttttattttg agttaggaga 4200
aaagttcgtt ttttgtagac gattttagat taagtaaggt gttcgtcgag tttttttttg 4260
taggttcggt ttacgatttt tatttatttt tttttttcgg gcgcggggcg gcggtaggcg 4320
gggttattag atttttttag ttcgggtttt tttaggcgtt agggacgttg acgttcgcgt 4380
tttttttttt tcgttttttt tttttatttt ttcgtttttt cgtttagttt tttttttagt 4440
ttcggcgtag gggggtcggg tttggtttcg cgaggttcgt gtattttaga gagtttagcg 4500
agttagagta ataaaggagt cgggtcgtcg gcggggagag ttcggggggt tgcggcgcgt 4560
tggggcgaag gtggttgttg ggtcgcgggt tggcgcgggg gcggagtcgg agttgtattc 4620
gtattttcgg ttcgcgtttg gtttttttcg cggggcgggg aggtggtttt aatggtggga 4680
gagggaatgg ggttggggat tgggggttta ggggtttttt agggttgaag ataatttgga 4740
ttgcgaggtt agggtttggg gagtcgtgta tttcgtttcg ggttttcgta gtttaatatg 4800
ggtttcgggt tttaaagttt gcgaagttgg gcgtcgaggg gtcggggcgc gcggagcgtt 4860
cgggggggtt cgggttcgga ttttttgggc gtataggtcg tttgagtcgt tttcgcggtc 4920
gtcgaggtcg tgcgtgtggt cggggggttt cgaggagtag gcgggggcgt cggggttttt 4980
tttgagtggc ggggttggtt atggttattg ttcgtttagg cgcgggggtt cggagttttc 5040
gtttttgggg ttgttgttgt tgttgttgtt gggggattcg gttcgggggg gcggtttcga 5100
gcgggaacgc gatcgggttt gggtttcgga gcgcggacgg gagcgcgagt agtagcgcgg 5160
cggtgattgg tttttcgtcg tcgttgagtt attgcggtcg ggttgttttt tgcgagtcgt 5220
tgcgttataa cgtgtgtttg ggttcggtgt tgttttacgg ggttattttt atattgttgg 5280
tcggagattc ggatttttag gaggaagcgt acggtaagtt cgtgttttgg tcgggtaagt 5340

gcggcggagt cgggtttggg gggcgggagg tgtcgcggta agatgggggt atttttggaa 5400
agaataggtt taggtttgag ttttggagag ggcggggata gtattcggga gagttggagg 5460
gatagatttc gaaattttgg gggtaggtta cgtgtaggat gggattttcg gttatgggaa 5520
aaggagggta aggaagttga cgaagcgggt tataaagggt ttgagttggt gggtgagtaa 5580
aggatggcgg ggatgagggg ttcgtgagga ggtggtttta agttacggga aaagatattt 5640
tgtaagaaga ggagatgttt tatttatttt ggaagtaggg tgtcggggga tttaaggagg 5700
tgttgaagat tttggggaaa ttggagttgg agaggaaaag gggagggatg acggaggaag 5760
aggtttagat atttggaggg aagggtaggg ggatatgatc gagtgaagtt ttgaaggaaa 5820
agaaattttt ttagtgatga attattaaaa ggtaattaga aagttttgga aattttgatt 5880
tgggagatag gaatgaagtt tagagaattg tgattttgtt tggtttttac gggagatttt 5940
atttagtttg tgtatttgaa aaatcggttt tttgacgtat gtaggaggtt atgaaggaat 6000
aagatttttt taatagttat ttgtgttttt tttggaaatt tggttatcga ggttggttag 6060
ggttgtggaa ggacgtttaa gtttggagtg ttgagagttt gtggatattt ttatggtggt 6120
ttgaagtttt aaagtttttt tattggagtg gtagagtttg aaaaattgta tattgaattt 6180
aaatgttttt ttttagtagt gtggagtata ttttttttta tttgttattt ttttagatga 6240
ggtttggagg gtgttgagga agggagttgg ttttattagt tttcgttttt agttagtatg 6300
attttaaaat tgttttggga aaaagaaaag aaaattagga gttaggagta gaattgggag 6360
tttttttttt ttgatagggt tggggtattt tattttttag ttttgtttag ggggttggtg 6420
aggattttaa tgtagggagt gtatgtaggt cgtttgttat tgataagttt tcggtttttg 6480
ttagatgttg gataaggtta taaggttttt ggtttggtgt agatgttgga ggaggaattt 6540
ggaggatggt atttatggta tttatgagga agagttgtat tttttttggg gattaggttt 6600
taaagttagt atagtatatt agatagaaat tatgtgtaaa taatagtggt tttgaaaatt 6660
ttgtagatta ttatagtgta aggtggatgt ggttttgtat atatattttt tttacgatgt 6720
ttgtatatat gtgttatatg tatatttata tataagaatg ggtatatata gaagataaaa 6780
taattgtaat tttaaagtta tatttatgta aagattgaaa taataataat aataataaaa 6840
agaatattaa agtgagatta agtaagattg ttttgagtat tatttaaaat gagatttgag 6900
tttaggagtt agttgtgtgg aaagtttaaa ttttttagta ttagaatttt atttagtttt 6960
gagttgttta tgttgttaga ggataatttt tttgtttttt tttttattga ttgtgtaagg 7020
atttatggtt taattttttt aagttaaatg tatgtttgat gttgtttttg tgtagttatt 7080
attagattaa ggaggaaggg tatgaatttt tttttagttt atatttttga gagtagttgt 7140
tgagtagata gaagtagttt aagttggaat atgtattaat tagtattatt tgtaggttgt 7200
gtaggttgaa attttattat ttgtgttttt tggtagggta gggttttata gagatgttag 7260
tattgagttt agtatcgtta gttttattag tttattatga gttttttttt gttattaatt 7320
attatttttt agtttatttt tgtttttata tgtgttttgg ttgtattttt tttgtttggg 7380
atttgtaagt agggtagaag taggtgttgg tttgagttgg gtaatgagta ggtagagtga 7440
atttaagttt cgtgtgagaa ttatagttag gttagttggt agtttttggt aagtgattta 7500
ttatgtttcg agttaatgat tttgattttg tattagatag ggttagtgtt atttggtttt 7560
taggggtgtt tttgtgggtg aaggatgaag gtgatttagg ttgtttttat cggtaaattt 7620
ttgaatgtag gaattgggtt tttatttttt tttaaagttt gtgagtattg agtatatagt 7680
tatgttttta tttatgtatt tagttatgta ttgaatgttt gttgtatgtt atatgttgcg 7740
tatgtaagga tgaagaatat agatatgatt ttttgttatt aagaagatag gttggatatg 7800
gtgtttatat ttgtgatttt agtattttgg gaggtcgagg cgggaagatt gtttaatatt 7860
aggagtttaa gattagtttg agattttgtt tttaaaaaaa aaaaaggaag atagataatt 7920
taataggttt tggaaatatt tgtgaaaggt attgtggtat tttagggtat tgaaaaagta 7980
taaaggattt aaggaatatt ttagaagaat tggtatttaa tttataattt gatatataag 8040
taggaattgt ttggtcgagg gagagttgag gtttgaaggg ataggaagag gaaatagtat 8100
gtataaaggt gtagaggtag gagggtttgg tgttttgaaa gaaagtataa gagttgtagt 8160
agaattggat taaagcgtgt gaggggtgga attgtgagag ataagattta aagaattaga 8220
aaataaaggg atttatattt tatattaagt ttggatgtta tttaggggat gataaggagt 8280
tattgaaaga ttttaagtaa gggagagatg taattgtttt tgtattttag ttggattttt 8340
ttgggtatgg tgtgaagaag ttataggagt agggaaagtt tggagtttgg ttttttgatt 8400
aggggatgtt gaagtagttt aagtaagaga cgatggcggt tgggattggg tagtaggata 8460
gagggagtgg gtagatttta ttaattttta gaggtagtgt ttatagggta ttgatgagtt 8520
agatgtggag ttttagtgag taggaggttt cgggttttag gtttggatgg aaggtggtgt 8580
tatttttttt tattaagata gagaatgtta gaggaagaag aagtttaggt aggaggtgtg 8640
gttttgattt tggtcgtgtg aattagagat gtttattgga tatttatgtg gagattttta 8700
gtagatttgg aaataggaat tgttggagag aggtggttat tgtgtgtttt tttggttttg 8760
attattgagg ttttggaagg atgttattga ttgaggaagt ttatagttta ggttggttta 8820
atttttaggt ttgtatagat tttaggtttt ttttttgttt tttatttttt tggttttagg 8880
tttttttagt tatttagtat tttttttttt tatagttgga aatgtttttt ttcgggtatt 8940
ttaagttgat tagataaata taatgtttag gtcgtggttt attttaggtt aaatttggtt 9000
tagtaagtgg ggtaaaaaag tagagaggtt tgtagttggt agtggcgata ggtttttgag 9060
agttagtacg tagttttttt aattattgtt gtgttttgtg tggtatataa gtgtgtggtt 9120
ttgttatttt gtttatttgt aggtaagtag gagagagata aaggttggga ggaatttttt 9180
gtttgttttt tatatattga attgagggtt gagaggagga atgtttttgg tgatggtggg 9240
ttatttttag ggacggatta ggaagaattt ttttagagtt atttttaaat ttggttggta 9300

attagagtta gttaaaaaga tatatatggt cgggcgcggt ggtttacgtt tgtaatttta 9360
gtattttggg aggtcgaggt gggcggatta cgaggttagg agatcgagat tatttcggta 9420
aatagggtga aatttcgttt ttattaaaaa tataaaaaaa ttagttgggt atggtggcgg 9480
gtgtttgtag ttttagttgt ttgggaggtt gaggtaggag aatggcggga attcgggagg 9540
tagagttttg tagtaagtcg agattacgtt attgtatttt agtttgggcg atagatcgag 9600
attttatttt aaaaaaaaaa aaaaaaaaaa aaaaaaaata tatatatata tatatatata 9660
tatatatatg tataggttgg gtgtagtggt ttatatttgt aatttagtta ttttgggagg 9720
ttgaggtagg aggatttttt gaagttagga gttcgagatt agtttgggta ataaagtgag 9780
attttatttt taaaaaaaaa aattagtcgg gtatgttggt atatatttgt ggttttagtt 9840
atattggagg ttgaggtaaa aggatcgttt gaatttaaga ggttgaggtt gtagtgagtt 9900
atttttatat tattgttttt tagattgggt aatagagtaa gattttgttt taaaaatata 9960
tatatatata tagatatgtg tgtatgtata tatatatata tatatatgta tagtgaaaat 10020
gtaaaatata gaaaaatata tatggattta agtttttttt aaaaaaaaat tatttttaat 10080
taattatttg agaaatgggg ttttgttgtg ttgtttaggt tggagtgtag tggttattta 10140
gaggtttatt tattgtgcgt atggtgttta tggtgtttgg aatagttggt attataggta 10200
tgtgttttgt gttagtttaa gttttaattt ttgttttaga tttttaattt tttaattgtg 10260
aaatataatt gtatgtagaa aattgtataa aatataaagg tattgaataa taaataatta 10320
taaagtgata tttgggttaa taattagatt atagttagat tttgaagttt tttttgtgt 10380
tttgtttagt tatatttttt tttttttgtt taagaggttt ttagtaatta tatttttttt 10440
ttttttatgg ttttattttt tagttttgga atttataagt agtatatttt agttttgtat 10500
gtttttgaat tttgtttaaa tggaatttgt ataaaataat atgttgtgta tattattttt 10560
tgtttttttt attttatatt attatgtaag atttatttat gttattgtag tttatttttt 10620
ttttgtttgt ttatttgttt gttttgtttt ttttgagata gttttatttt gttatttagg 10680
ttagagtgta gtggcgcgat ttcggtttat tatattttta ttttttgggt ttaaacgatt 10740
tttttgtttt cgttttttag gtagttggga ttataggtgt acgttattag gtttggttaa 10800
tttttgtatt ttttagtaga gataggtttt tgttatgttg gttaggttgg ttttaaattt 10860
ttgattttag gtgagttatt cgttttatat tgtagtttat ttttttattg ttgaataata 10920
ttttattata tgagtatatt tttagttgtt tatttatttt attgttattt taagtttttg 10980
tttattgtaa ataatattgt tgtgagtagt tttttatgta tattttagta taattggtat 11040
aagttttttt aggtatatgt tgaagaagta gaattgttag ataataaggt acgtatattt 11100
taggtttttg ttaaattatt ttttaaagtg gttgtattaa tttatatttt tattagttag 11160
tagaaagtta atattggttt atatgatttt taatatttgg tattattagt tttttatatt 11220
tttgttaatt tagtggttat attgtggtat atatttgtgg ttttaatttg tatttttttg 11280
attattagta aagttaaatg tttttttaaa tgtttattgg tttttagggt ttttgttttt 11340
gggaagtgtt tgtttaaatt tttgatatat ttttttatag gggttatttg ttatttttgt 11400
aagatttgta ggagtttttt atatatgtta gatatgagtt tataggatta taaaggaaat 11460
taattatatt gatataatag ttattaaata taaaaatgta agtatgtggg tttatttatt 11520
tatataataa ataagatttg gtgtaggtgt aataataatt ataatattaa agtgattata 11580
agtattgata ttattttaag atatttgtaa taaaaatatt tggtttttat ttgtgataac 11640
gttataggta ttattaatat tatgtattgt gggttaggcg tggtggttta tatttgtaat 11700
tttagtattt tgggaggtta aggtgggtag attacgaggt taggaagtcg agattatttt 11760
ggttaatacg gtgaaatttt atttttatta aaaatataaa aaattagtcg ggcgcggtgg 11820
tgggcgtttg tagttttagt tattcgggag gttgaggtag gaaaatggtg tgaatttagg 11880
aggcggagtt tgtagtgagt cgagatcgtg ttattgtatt ttagtttggg tgatagagtg 11940
agattttttt ttaaaaaaaa aaaaaagaaa agaaaaaaaa aatatttaaa aattatgtat 12000
tgcgatgtgt tgtttatatt tataattgga ggaagggtta aattttagtt aggtgtaatt 12060
ttttttttat tgaaatttat ggatttttga atttttttaa gaattttttg tgaattatag 12120
ggtaagaatg ttatttatag aatattatta tttagtagag agtttgagtt atttttgatt 12180
gtgtgtgtgt gtgtgtgtgt gtgtgtgttt taatagagat agggtttgat tttattgttt 12240
cggttggttt tgaatttttg ggtttaagtg atttttttgt tttggttttt taaaatgttt 12300
ttttgtgttt taatataaaa atatttggtg attttttatt gggattgtat tgattatata 12360
gattaatttg gaaaaaaaaa gagatatttt tataatattg agttttttaa atattgaata 12420
tttatatatt tttatttaga ttttttattt ttttaatatt ttgtatagat ttaaatattt 12480
attatttgtt agatattttt aggtatttga tatttttgat gttgttgtaa atgatgtttt 12540
taatatttat tttattttgt ttgttgttga tatatagaaa taaaattggt tgggtacggt 12600
ggtttatatt tgtaatttta gtatttcggg aggttaaggt agggggatta cgaggttagg 12660
agattgagat cgttttggtt aatatggtga aattttattt ttattaaaaa tataaaaagt 12720
tagtcgggta tggtggcggg cgtttgtagt tttagttatt taggaggttg aggtaggaga 12780
atggcgtgaa tttgggaggc ggagtttgta atgagtttag attgtgttat tgtattttag 12840
tttgggtaat atagtgagat atcgttttaa aaaaaaaaaa aaaaaagaaa taaaattgat 12900
gtttttaata tttattttat tttgttgttg atatatagaa ataaaattga ttgtgaattt 12960
agtaaatttg ttaaatttat tttatatata tagttatata atttgtgagt aatgagtttt 13020
gttttttttt ttttaagttt tatatttttt atttattttt attgttttat tgaaggtttt 13080
tagtatattg ttaaatagat aaaatatttt aatttttgat ttttttggat tttaaaaaga 13140
aagtttttt tatttttat tattaaattt gatgtttggt ataggttttt tatagttaat 13200
tatattttat tagattaaga acgttgtttt ttattttaaa tttgttaatt atttttttga 13260

ttatagatgg atgttgaatt ttattaattt ttttttttgg agacgaagtg ttattgttat 13320
cgaggttgga gtgtagtgat gcgattttag tttattgtaa tttttgtttt ttgggtttaa 13380
gcgatttttt tgttttagtt ttttaagtat ttgggattat aggcgtatgt tattacgttc 13440
ggttaatttt ttgtattttt agtagagacg gggttttatc gtgttagtta ggttaatttt 13500
ttgatcgtgt gattcgttcg ttttagtttt ttaaagtgtt aggattatag gtgtgagtta 13560
ttgcgttcgg ttgaatttta ttaatttttt tttgtattta ttgagatgat tatatgattt 13620
tttttttttt taatttgtta atgtagtaaa ttgtattgat tgaatttttt aatattaaat 13680
tagttttaga atagtgatat ttaatttgat tagttattag aattatataa ataaaaagtt 13740
tttttaaaag tttagattag ttaggcgcgg taatattcgt atttttgttt ttttttttgt 13800
tttatatttt attaaaataa aagaagtaaa ataatattga taaagttgat attttttttt 13860
tttttcgttt tagttttata tttttttttt ttttttggaa agttttttta ttttagtaat 13920
ttgtgtattt ttttattttg tttttaagtt taagttttgt ttttgttttt gtttttgttt 13980
ttttgtagag atggggtttg gttatattgt ttaggttggt ttcgaatttt tgaatttaag 14040
tgatttattt attttgggtt ttttaaaatg ttaggattat aggcgtgagt tattatgttt 14100
agttttaagt ttaattattt ttaaaaaata ggttatatat gtatatggta tagaatttaa 14160
aaggaataag ttaagtttta tttttgtatt tagttattta tttttttttt gtagaagtaa 14220
ttattaatat tggtttttgt gtttttttgg aaatatttaa tatttgttta gatgtagatg 14280
tgtttttagt ttttttttta tcgtttaatt agtattatgt tttatagatt gttttttatt 14340
taattgagtt tatgtaatat gataatattg gatattattt tattttaatg tatattttgt 14400
tatgtatttt agaaggagat tttggttaat aatggattat atgtgatggt gtttttttaa 14460
gattataata ttatattttt attgtagttt tttaatgttt agatatgttt agatatattg 14520
atatttatcg ttgtgttata gttgtttgta gtatttagta tagtaatatg ttgtataggt 14580
ttgtagttta gaagttatag gttatattgt attagttagg tgtgtagtag gttatattat 14640
ttaggtttgt gtaagtatat tttgatgttt atataaagat gatattgttt gatgatatat 14700
tttatagaag atatttttat tgttaagtaa tatgtgatta tattttaatt ttgaatagtg 14760
ttttattata atggtgtata ttattttatt tggttggttt tttattgatg gatatggatt 14820
taagttgttt ttattttttt gttattgtaa aatagtgttg taatgaataa tttatattat 14880
ttttgtgaat atgtgaagta gaagtattag tataaaattt tggtagataa tgttaaagtg 14940
atttttataa gtgtttatta atttttattt ttattaataa atataaaagt gttagtttat 15000
ttttatattt tttatagtac ggtatttttt tttttttttt tttttttttt tgagatagag 15060
ttttgttttg ttatttagat tggagtgtaa tggcgatttc ggtttattgt aatattcgtt 15120
ttttggattt aagtaatttt tttgttttag ttttttaaga agttgggatt ataggcgttt 15180
attattatat ttagttaatg tttgtagttt tttagtagag acgggttttt attatgttgg 15240
ttaggtttgt gtagaatttt tgattttagg tggtttattt gtttcggttt tttaaattgt 15300
tgggattata ggcgtgagtt attattatgt ttagtttttt ttatgttttt aaaggttatt 15360
aaatggtttt gagtttagga gtttaagatt agtttgagta atatggtgaa attttgtttt 15420
tatagaaaaa tataaaaatt tattaggtgt ggtggtgtga gtttgtagtt tttgttattt 15480
aggaggttga ggtgggagga ttaattgaat ttggggagtt gaaggttgta gtgagtcgtg 15540
attatgttat tgtattttag tttgggtaat agtgtgagat tttattttaa aaaataaaaa 15600
aataaaaaaa atataaagag atgttcgatt ttatttttaa taatataaat gtggaataaa 15660
ttataacgaa atattttttt tttaaatttt tatatttttt attatagtga taaagattaa 15720
aatatttgaa aatgtgattt gttatttgtt tttcgtttgt gtgtttatag aattatgtaa 15780
tttttattga tttggaggag ttttgtgtat ataaggaaat tagtttttgt gataagtatt 15840
gaaaatatat ttttttagta ttttattttt tttatgattt ttgtatcgat ttttttgaag 15900
ttttttgttt aaatttttta ttttatgatg tgtaggtttt gtgttatttt agaaggtttt 15960
ttttatttta aggtttaaaa aattattttt tggttaggcg tggtggttta cgtcgtaatt 16020
ttattttggg agatcgaggc gggtggatta cgaggttagg agatcgagat tattttggtt 16080
aatacggtga attttcgttt ttattaaaaa tataaaaaaa aaattagtcg ggtgtggtgg 16140
cgggcgtttg taattttagt tattcgggag gttgaggtag gagaatggcg tgaatttagg 16200
aggcggagtt cgtagtgagt cgagattata ttattgtatt ttagtttggg cgatagagta 16260
agattttatt ttaaaaaaaa aaattatttt ttattttttt ttttaaaatt ttaattttta 16320
gaaaaataat gtagttattt ttttatttat ttggattttt atgtaagaaa tggggtaaag 16380
ttttatttta tttttttttt agataatttt ttagttgttt ttgaagatgt atttaataat 16440
tttttttttt tttggggagt tttattttta aggtatgaaa taatatttat aatgagggaa 16500
aaaaaggaaa ataaattaga tatttttaaa atttgattgt tattatagat ataaaaagat 16560
tgagaaaaat tatagttttt tttttgttgt tgtttgtttg tttgttttaa tgagttttgt 16620
tgtattattt aggttggagt atattgtcgt gattttgatt tattgtaatt tttatttttt 16680
gtgtttaagt aatttttttg ttttagtttt ttaagtagtt gggattatag gcgtggatta 16740
ttacgtttgg ttaattttta tattttttag tagagatagg gttttattat gttggttagg 16800
ttggttttaa attttcgatt ttaagtgatt tgtttgtttt agttttttaa agtgttggga 16860
ttataggtat gagttatcgt atttggttga tataattttt atttttttta tttttcgaga 16920
cggggttttg ttttgtcgtt taggttggag tgtagtggtg ttattttcgt ttattgtaat 16980
ttttgttttt tgggtttaag tgattttttt gttttagttt tttgagtagt tgggattata 17040
ggtatgtgtt attatattta gttaattttt gtatttttag tagagatagg gttttattat 17100
gttggttagg ttggtttcgg atttttgatt ttgtgattta ttcgtttcgg ttttttaagt 17160
tttgggatta taggtttgag ttattatgtt tagtcgatat aattttttta ttaattgttt 17220

gatgtttttt taaagtattt tattttagag aggttttttt attttaaata tattgtgtaa 17280
taaaagtaaa tatttagtaa aattgatttt tttttgtata gttttaagtt tagataaata 17340
tatagattta tgtaattagt tattatattg aatttaagat agagaatcgt tttttaattt 17400
ttatatattt ttttatgtta ttattttgta ggtaaatttt gtttttattt taatttttgg 17460
tatttattgt tttgtttttt gtaagtattt atgtatttag aatttttggt gattgttttt 17520
gggttttttg ttttttattt ttttgttttt gaggtagagt tttatttttt tgtttaggtt 17580
ggagtgtagt ggcgttattt tagtttatcg taatttttat tttttaggtt taagtaattt 17640
tgttgtttta gtttttcgag tagttgggat tataggtatg tattattacg atcggttaat 17700
ttttgtattt ttagtagaga cggggtttta ttttgttggt taggttggtt ttgaattttt 17760
agttttaggt gatttgttcg tttttgtttt ttaaagtgtt gggattatag gtgtgagtta 17820
ttgcgtttgg ttaattggtg attgaattga gttaagtggg aattgttgtt tgaggatatt 17880
gttttttaat tggtaggaat ttgttttgta ttttggaata tattttgaag gttgggagta 17940
gttagaggtt ttagataaat aattataaga gttaatattt tttgagcgtt tattatatga 18000
taggtattgt gttaagggta tttaaagtat tatattattt tttatagtag ttttatgagg 18060
taaatttatt atttttattt tatagatgag gtaattgagg ttaaagataa agtattttgt 18120
ttaagattat atagttagga aagagtggag ttaagatttg gatttaggtt gggtagttgt 18180
tatgtatatg ttttttattt ttttattatg ttgttttttt tcgaaaagag tggttatttg 18240
tttttattat ttttagtttg gaaatgtgat cggtatttat gttggttttt atatttttat 18300
attttttttt aatgtaattt tttgtttggg aaatagatat tgttgaagta gggttgattt 18360
aggaagggtt tttgtgatgg gtagggcgga ggattaggtt ggggacgggt gttgggagat 18420
tgtcgtttgt ttttgttttg ttgataatat tttttttttt ttcgtattgg atagagtttt 18480
ttttgttttt ttttatttga ttttttgttt ttttttttgg ttttggagtg tttggttttt 18540
ttgattattt ttttttaagt ttttattttt ttttttttat ttcgtttggt aagaggaata 18600
agggttttgg ggtttgggta aagggaaata ttttttggtt ttagtaaatg ggtttcgtga 18660
gagttgggtg tatttttgaa aaggtaaatt tatttcgggg agttttatta gaggtattat 18720
tattttttag tagggtattt ggaagtggta gttatttttt taaaatttaa atgatagtaa 18780
gtatatagaa tagcgtatag attatgagtt tggtttttat aaggtgaata tatttatgta 18840
attagtatta gattaggaga tagaatattt ttaatatttt atgtaattag tattagatta 18900
agaagtaaaa tatttttagt agtttagagg ttttttttta ggtttttttt tagttatcgt 18960
ttttagagta gagggttggg gtatttttgt ttgttataat tatttttggg ttttattggt 19020
attgagttgg tagagtttag tgtttgtaat gtatgggata gttttatggt ataaagaatt 19080
gttttgttta gatgtattgt tgagaaatat tgtatttgtt aggtttgatt agtgatgggt 19140
tgtagtgtgg taaaggtttg gaggataggg gtgaagttgc gtttgtgggt tagagtgagg 19200
aggggttttt attgtaatgt tgttgttatt tttagg 19236

<210> 244
<211> 19236
<212> DNA
<213> Artificial Sequence

<220>
<223> chemically treated genomic DNA (Homo sapiens)

<400> 244

tttgggggtg gtaatagtat tgtagtgaag gttttttttt attttgattt atagacgtag 60
ttttattttt gttttttagg tttttgttat attgtagttt attattggtt agatttggta 120
ggtatagtgt tttttaatag tgtatttggg taggatagtt ttttatatta taggattgtt 180
ttatgtattg tagatattag attttgttaa tttaatgtta gtagaattta gaagtgattg 240
tgataaataa aaatgtttta gttttttatt ttagggacgg tgattagaag ggagtttgga 300
gaggaatttt tgggttgttg ggaatgtttt attttttgat ttggtattgg ttatatggga 360
tgttgggaat attttatttt ttgatttgat attggttata tgagtgtatt tattttgtgg 420
aaattaagtt tatgatttgt gcgttatttt gtatgtttat tattatttaa gttttaagaa 480
aataattgtt atttttagat gttttgttag ggagtggtaa tgtttttggt agagtttttc 540
ggggtggatt tattttttta ggggtgtatt taatttttac ggggtttatt tgttagagtt 600
aggagatgtt tttttttgtt tagattttag aatttttgtt ttttttatta agcggagtga 660
gagagaagag atgggggttt aagaagaggt agttagagga gttaggtatt ttaaagttag 720
aggaggaagt aggaagttag atggagagga atagaagagg ttttgtttag tgcggagagg 780
ggaaaaatgt tgttagtaag atagaaatag gcggtaattt tttagtattc gtttttagtt 840
tagtttttcg ttttgtttat tatagaagtt ttttttgaat taattttgtt ttagtaatat 900
ttatttttta agtaagaagt tgtattagga gagaatatga ggatgtgaga gttaatatgg 960
gtgtcggtta tatttttagg ttgggggtgg tagagatagg taattatttt tttcggggga 1020
aggtaatata gtagagaggt aaggggtatg tgtatggtaa ttgtttagtt tgggtttaaa 1080
ttttggtttt attttttttt ggttgtatga ttttgggtaa agtattttat ttttagtttt 1140
agttgtttta tttgtaaaat ggggatgata gatttatttt atggaattgt tgtgaaaagt 1200
gatataatgt tttaaatgtt tttggtatag tatttgttat gtagtaagcg tttaggaaat 1260

gttaattttt atgattattt gtttgaggtt tttgattgtt tttagttttt agagtatatt 1320
ttagggtgta gagtaagttt ttgttagtta agaagtaatg tttttaggta atagttttta 1380
tttgatttaa tttagttatt agttggttag gcgtagtggt ttatatttgt aattttagta 1440
ttttgggagg taaaggcggg tagattattt gaggttagga gtttaagatt agtttggtta 1500
ataaggtgaa atttcgtttt tattaaaaat ataaaaatta gtcggtcgtg gtggtatatg 1560
tttgtaattt tagttattcg ggaggttgag gtaatagaat tgtttgaatt taggaggtgg 1620
aggttgcggt gggttgagat ggcgttattg tattttagtt tgggtaaaag aatgagattt 1680
tgttttaaaa ataaaaaaat aaaaaataaa aaatttaaaa atagttatta gaaattttga 1740
gtatataggt atttgtagaa aatagagtag tgggtgttag ggattggggt gagggtagga 1800
tttgtttata aaatagtagt atgagagaat atatgggggt taaaggacga ttttttattt 1860
tgaatttaat gtggtggttg gttatataag tttatgtgtt tgtttaaatt tagaattgta 1920
taagaaaaag ttaattttat tggatgttta tttttattgt ataatatatt taaaatgggg 1980
ggattttttt ggagtaaaat gttttagaga aatattaggt agttaataaa aaggttgtgt 2040
cggttgggta tggtggttta aatttgtaat tttagaattt gggaggtcga ggcgggtgga 2100
ttataaggtt aggagttcga gattagtttg gttaatatgg tgaaattttg tttttattaa 2160
aaatataaaa attagttgga tgtggtggta tatgtttgta attttagtta tttaggaggt 2220
tgaggtagga gaattatttg aatttaggag gtagaggttg tagtaagcgg agatggtatt 2280
attgtatttt agtttaggcg atagagtaag gtttcgtttc gaaaaataaa aaaaataaag 2340
gttgtgttag ttaggtgcgg tagtttatgt ttgtaatttt agtattttga gaggttgagg 2400
taggtagatt atttgaggtc gggagtttga gattagtttg gttaatatgg tgaaattttg 2460
tttttattaa aaaatataaa aattaattag gcgtggtggt ttacgtttgt agttttagtt 2520
atttgggagg ttgaggtaga agaattgttt gaatatagga ggtggaggtt gtagtgagtt 2580
aagattacga tagtgtattt tagtttgggt gatatagtaa gatttattaa aataaataaa 2640
taaataataa taaaaaaaaa attgtaattt tttttaattt ttttatattt gtggtaataa 2700
ttaggtttta aaaatgttta atttgttttt tttttttttt tttattgtga atattgtttt 2760
atattttaaa aataaaattt tttaggaaaa agagagatta ttaaatatat ttttaaggat 2820
aattaagaaa ttatttgggg aaaaaataag gtggagtttt attttatttt ttatataaaa 2880
gtttagatgg ataaaaaaat aattatattg tttttttaaa aattaaagtt ttagaagaaa 2940
agatgggaaa tgattttttt ttttgagatg gagttttgtt ttgtcgttta ggttggagtg 3000
tagtaatgtg atttcggttt attgcgagtt tcgttttttg ggtttacgtt atttttttgt 3060
tttagttttt cgagtagttg ggattatagg cgttcgttat tatattcggt taattttttt 3120
tttgtatttt tagtagagac gggggtttat cgtgttagtt aggatggttt cgattttttg 3180
atttcgtgat ttattcgttt cggtttttta aagtgggatt acggcgtgag ttattacgtt 3240
tggttaggaa atgatttttt aaattttgga gtggagaagg ttttttgaaa tgatataaag 3300
tttatatatt atgaaataaa agatttaaat aaaagatttt agaaaaatcg atataaaaat 3360
tataagaaaa gtaagatatt gaaaaaatgt atttttaata tttattataa gagttaattt 3420
ttttatatgt ataaagtttt tttaaattag taaaaattgt ataattttat agatatatag 3480
gcgaaagata aataatagat tatattttta aatgttttga tttttattat tgtgataaaa 3540
agtataaaag tttaaaaaaa gaatatttcg ttgtagttta ttttatattt gtattattaa 3600
gagtgaggtc gaatattttt ttatgttttt tttatttttt tattttttga gatagggttt 3660
tatattgttg tttaggttgg agtatagtgg tataattacg gtttattgta gtttttagtt 3720
ttttaggttt agttaatttt tttattttaa ttttttgagt agtaaagatt ataggtttat 3780
attattatat ttggtgaatt tttgtatttt tttgtagaga taaggtttta ttatgttgtt 3840
taggttggtt ttgaattttt ggatttaagg ttatttaatg gtttttaaaa atataaagaa 3900
ggttgagtat ggtggtggtt tacgtttgta attttaatag tttgggaggt cgaggtaggt 3960
ggattatttg aggttaggag ttttatataa gtttggttaa tatggtgaaa attcgttttt 4020
attaaaaaat tataaatatt agttaggtgt ggtggtgggc gtttgtaatt ttagtttttt 4080
gggaggttga ggtaggagaa ttgtttgaat ttaggaggcg gatgttgtag tgagtcgaga 4140
tcgttattgt attttagttt gggtgataga ataaaatttt attttaaaaa aaaaaaaaaa 4200
aagaaaagaa aatatcgtat tataaagagt gtagggataa attggtattt ttatatttgt 4260
tggtaggaat aaaaattagt aaatatttgt ggagattatt ttggtattat ttattaaaat 4320
tttatattag tatttttatt ttatatgttt ataaagataa tatagattat ttattgtagt 4380
attgttttgt aatagtaaaa aagtagaaat agtttgaatt tatatttatt aataggagat 4440
taattaagta aaataatgta tattattata atagaatatt atttagggtt gagatatagt 4500
tatatgttgt ttaataatga ggatattttt tgtgaaatgt gttattaggt aatgttattt 4560
ttgtgtgaat attagagtgt atttatataa atttggatgg tatagtttat tgtatattta 4620
gttgatatag tataatttat ggtttttagg ttgtaaattt gtatagtatg ttgttatatt 4680
gaatattgta ggtaattgta atataacggt gagtattagt gtatttaaat atatttaaat 4740
attgaaaggt tatagtaaaa atgtggtatt ataattttaa gggaatatta ttatatgtgg 4800
tttattgtta attaaaattt tttttggggg tgtatgatag gatgtgtatt gaaatggaat 4860
gatgtttaat gttgttatgt tatatggatt tagttgagtg gagagtagtt tatagagtat 4920
ggtattggtt gggcggtaga ggagagattg agagtatatt tatatttgga tagatgttgg 4980
atatttttaa aaggatataa aaattagtgt tggtggttat ttttgtagag gaaaaatggg 5040
tagttagatg tagaagtagg atttaatttg ttttttttga attttgtgtt atgtatatgt 5100
ataatttatt ttttaaaagt aattaagttt aaggttggat atggtggttt acgtttataa 5160
ttttagtatt ttgggaggtt taaggtgggt ggattattta agtttaggag ttcgagatta 5220

atttgggtaa tatggttaaa ttttattttt ataaaaaaat aaaaataaaa ataaaaataa 5280
aatttgagtt taaaaataaa ataaagaaat atataaatta ttaggatggg gaagtttttt 5340
aaagagaaaa gaaggatatg aaattgaaac gagaaagaga ggggatatta gttttattag 5400
tattatttta ttttttttat tttaataaaa tatgaaataa aaagagaaat aggaatacgg 5460
gtgttatcgc gtttggttga tttagatttt taaaaagatt ttttatttgt gtgattttga 5520
tgattagtta agttgagtat tattgtttta aggttggttt aatattagaa aatttaatta 5580
atgtaattta ttatattaat aggttaaagg agaaaaaaat tatatgatta ttttaatgga 5640
tgtaaaaaaa agttgataaa atttagtcgg gcgtagtggt ttatatttgt aattttagta 5700
ttttgggagg ttgaggcggg cggattatac ggttaggaaa ttgatttggt taatacggtg 5760
aaatttcgtt tttattaaaa atataaaaaa ttagtcgggc gtggtggtat gcgtttgtag 5820
ttttagatat ttgggaggtt gaggtaggag aatcgtttga atttaggagg tagaggttgt 5880
agtgagttga gatcgtatta ttgtatttta gtttcggtga tagtgatatt tcgtttttaa 5940
aaaaaaaagt tgataaaatt taatatttat ttatgattaa aagaataatt ggtaaatttg 6000
aaataagaga taacgttttt aatttgataa aatgtaatta attataaaaa atttatatta 6060
aatattaaat ttaatggtga agggtagaaa aagttttttt tttagaattt agaaaagtta 6120
agaattaaga tgttttgttt atttaataat gtattggaag tttttagtaa agtaataagg 6180
ataaataaaa ggtataagat ttggaaagaa agaaataaaa tttattattt ataaattata 6240
tgattatgtg tatagaataa atttagtaag tttattgaat ttataattaa ttttattttt 6300
atatattagt aataaagtga aataaatgtt aaaggtatta attttatttt tttttttttt 6360
ttttttttga gacggtgttt tattgtattg tttaggttgg agtgtagtgg tataatttgg 6420
gtttattgta agtttcgttt tttaggttta cgttattttt ttgttttagt tttttgagta 6480
gttgggatta taggcgttcg ttattatgtt cgattaattt tttgtatttt tagtagagat 6540
ggagttttat tatgttagtt aggacggttt taattttttg atttcgtgat ttttttgttt 6600
tggtttttcg aagtgttggg attataggtg tgagttatcg tgtttagtta gttttatttt 6660
tatatgttag taataaataa agtgaaataa atgttaaagg tattatttat aatagtatta 6720
aaaatattaa gtatttagga atgtttaata aataataagt atttaaattt gtgtagaata 6780
ttgagaaaat gaaagattta aatagaaata tataaatgtt taatgtttgg aagatttagt 6840
attgtaaaga tgtttttttt tttttttaag ttaatttata tagttaatgt aattttaata 6900
aaaaattatt aagtattttt atattaaaat ataaaggagt attttgggag gttaaggtag 6960
gaggattatt tgagtttagg agtttaagat tagtcgggat aataggttaa aattttgttt 7020
ttattaaaat atatatatat atatatatat atatataatt aagaatagtt tagatttttt 7080
attagatagt gatattttgt aagtaatatt tttattttat ggtttataaa ggatttttgg 7140
aaaaatttag aggtttatga attttaatgg gaaaaaaatt atatttaatt aaaatttggt 7200
ttttttttta attatgaatg taagtaatat atcgtagtat ataattttta aatgtttttt 7260
tttttttttt tttttttttt ttttgagagg gagttttatt ttgttattta ggttggagtg 7320
tagtggtacg atttcggttt attgtaagtt tcgttttttg ggtttatatt atttttttgt 7380
tttagttttt cgagtagttg ggattatagg cgtttattat cgcgttcggt taattttttg 7440
tatttttagt agagatgggg ttttatcgtg ttagttagga tggtttcgat tttttgattt 7500
cgtgatttgt ttattttgat tttttaaagt gttgggatta taggtgtgag ttattacgtt 7560
tggtttatag tatataatgt taatagtatt tgtggcgttg ttataagtgg aaattagata 7620
tttttgttgt agatattttg aaataatgtt aatgtttata attattttga tattatagtt 7680
gttattatat ttgtattaga ttttatttat tgtataaata aataagttta tatatttgta 7740
tttttatatt tgataattat tatgttaata taattgattt tttttgtaat tttataaatt 7800
tatatttagt atatataaag aatttttata aattttataa aaatgataga tagtttttat 7860
ggaaaaatat gttaaagatt tgaatagata ttttttaaaa gtagaaattt taaaggttag 7920
taagtattta aaaaggtatt taattttatt agtaattagg gaagtgtaaa ttaaaattat 7980
aaatatatgt tataatatag ttattagatt ggtaaaaatg taaaaggttg ataatattaa 8040
atgttgggaa ttatgtgaat tagtgttgat tttttattag ttagtgggag tataagttag 8100
tataattatt ttgaaaaatg gtttggtaaa agtttaaagt atgcgtgttt tattatttag 8160
taattttatt tttttagtat atatttgaag gaatttgtgt tagttgtatt aggatatata 8220
taaaagatta tttatagtaa tgttatttgt aatgggtaaa aatttgagat gataataaaa 8280
tgggtaaata attgggggta tatttatata atggaatatt atttagtaat gaagaaataa 8340
gttgtagtat gaggcgggtg gtttatttga agttaggagt ttgaggttag tttgattaat 8400
atggtaaaat tttgttttta ttaaaaaata taaaaattag ttaggtttgg tggcgtgtat 8460
ttatggtttt agttatttgg gaggcggagg taggagaatc gtttgaattt aggaggtgga 8520
ggtgtagtga gtcgagatcg cgttattgta ttttagttta ggtgatagag tgagattgtt 8580
ttaaaaaaat aaaaataaat aaataaataa gtaaaaaaga aataagttgt aataatatgg 8640
atgaatttta tataatgata taaagtaaaa gaagtaagaa ataatatgta tagtatatta 8700
ttttgtataa attttatttg aatagagttt aaaaatatgt aaagttgaaa tatattgttt 8760
atggatttta aaattaggag gtaaaattat agagaaaaaa aggatgtgat tattgaaaat 8820
tttttgagta aggggagggg gatgtgatta gataagatat agagggagtt ttaggggttt 8880
gattgtggtt tagttattga tttagatgtt attttataat tatttgttat ttagtatttt 8940
tgtgttttat gtagtttttt gtatatagtt atattttata attaaaaagt taaagatttg 9000
aagtaaaatt gaagatttga gttggtatag aatatatgtt tgtagtgtta gttattttag 9060
gtattataag tattatgcgt ataatgagtg agtttttgaa tagttattgt attttagttt 9120
gggtaatata gtaagatttt attttttaaa taattaatta aagataattt ttttttaaaa 9180

aagatttagg tttatgtatg tttttttgta ttttatattt ttattatata tatatgtgtg 9240
tgtgtgtata tatatatata tatttatatg tatgtatgta tttttgagat agggttttgt 9300
tttgttgttt agtttggagg gtagtggtgt gaggatggtt tattgtagtt ttaatttttt 9360
gggtttaagc gatttttttg ttttagtttt tagtgtagtt gggattatag gtgtgtgtta 9420
atatgttcgg ttaatttttt tttttagaga tggggtttta ttttgttgtt taggttggtt 9480
tcgaattttt ggttttaaga aatttttttg ttttaatttt ttaaagtgtt aagattatag 9540
gtgtgagtta ttgtatttag tttatatata tatatatata tatatatata tatatatatt 9600
tttttttttt tttttttttt ttttttgaga tggagtttcg gtttgtcgtt taggttggag 9660
tgtagtggcg tgatttcggt ttattgtaag gttttgtttt tcgggttttc gttatttttt 9720
tgttttagtt ttttaagtag ttgggattat aggtattcgt tattatgttt aattaatttt 9780
tttgtatttt tagtagagac ggggttttat tttgtttgtc gggatggttt cgattttttg 9840
atttcgtgat tcgtttattt cggtttttta aagtgttggg attgtaggcg tgagttatcg 9900
cgttcggtta tatatatttt tttaattgat tttgattatt agttaagttt ggaaatagtt 9960
ttaaggaaat tttttttgat tcgtttttga aaataattta ttattattaa aaatattttt 10020
ttttttagtt tttaatttaa tgtgtgagag gtaaataaga aatttttttt agtttttgtt 10080
ttttttttat ttatttgtaa gtgggtaagg tgatagggtt atatatttat gtattatata 10140
gagtatagta gtggttaagg aggttacgtg ttggttttta gggatttatc gttattgtta 10200
gttgtaggtt tttttgtttt tttgttttat ttattaaatt aggtttggtt taaggtggat 10260
tacggtttgg atattatgtt tgtttggtta gtttggaatg ttcgggagga ggtattttta 10320
gttgtaaagg ggagagatgt taagtgattg aagaagtttg aggttagaga gatgaaagat 10380
agaggaaaga tttgaggttt atatagattt agaaattgga ttaatttgag ttataaattt 10440
ttttagttaa tggtattttt ttaaagtttt agtagttagg gttaaggaag tatatagtgg 10500
ttattttttt ttagtaattt ttatttttag atttgttgag aatttttata tagatgttta 10560
atgagtattt ttaatttata cggttaaaat taaagttata ttttttgttt aaattttttt 10620
tttttttagt attttttatt ttagtgaaag ggaatggtat tattttttat ttaagtttga 10680
aattcgaggt tttttgttta ttgaggtttt atatttaatt tattagtgtt ttatgagtat 10740
tatttttgaa agttagtggg atttgtttat tttttttgtt ttgttattta gttttaatcg 10800
ttatcgtttt ttatttgggt tattttaata ttttttggtt aagaagttag gttttaggtt 10860
ttttttgttt ttatggtttt tttatattat atttaaagag atttagttaa aatgtaaaga 10920
taattatatt ttttttttgt ttaaaatttt ttaatggttt tttattattt tttggataat 10980
atttaaattt aatatgaggt ataaattttt ttatttttta attttttagg ttttattttt 11040
tataatttta ttttttatac gttttgattt agttttattg taatttttat attttttttt 11100
aaagtattaa gtttttttat ttttatattt ttgtatatgt tatttttttt ttttattttt 11160
ttaaatttta atttttttttc ggttaggtaa tttttgttta tgtgttaagt tatgggttaa 11220
atgttagttt ttttgggatg ttttttgggt tttttatgtt tttttagtat tttaaaatgt 11280
tatagtattt tttataggta tttttaaagt ttgttgaatt atttgttttt tttttttttt 11340
tttagaggta aggttttagg ttggttttaa atttttggta ttgagtaatt ttttcgtttc 11400
ggttttttaa agtgttgaga ttataggtgt gagtattatg tttagtttgt ttttttgatg 11460
atagaggatt atatttgtat tttttatttt tatatgcgta gtatgtgata tatagtaaat 11520
atttaatata taattgaatg tatgaataga agtatggttg tgtatttagt atttataggt 11580
tttgaaaaaa agtgaagatt tagtttttat atttaagagt ttatcgatga agataattta 11640
gattattttt attttttatt tataaaaata tttttaagag ttaggtaata ttgattttgt 11700
ttaatgtaaa attaagatta ttaattcggg atatgataaa ttatttgtta gaggttatta 11760
gttgatttgg ttgtgatttt tatacgaggt ttgggtttat tttatttatt tattatttag 11820
tttaagttaa tatttgtttt tgttttattt gtaagtttta agtagaaaga gtgtaattag 11880
aatatatatg gaaataggaa tgagttgagg gatggtggtt ggtggtagag aagaatttat 11940
agtgaattaa tggaattaac ggtgttgggt ttagtgttgg tatttttgta ggattttgtt 12000
ttgttaaaag gtatagatag tgagatttta atttgtataa tttgtagatg gtattaattg 12060
atatatgttt tagtttaaat tatttttgtt tgtttagtag ttgtttttaa gagtatgggt 12120
tggaaagagg tttatatttt ttttttttga tttgatggta attatataga ggtaatatta 12180
aatatgtatt taatttggga aaattaaatt atgggttttt gtataattaa taaagagagg 12240
gatagaaaga ttattttttg atagtatgag taatttagaa ttgggtgaga ttttagtgtt 12300
gagagatttg gattttttat atagttggtt tttaaattta agttttattt tgggtggtgt 12360
ttaaagtaat tttgtttaat tttattttgg tgtttttttt attattatta ttattatttt 12420
agtttttgta tgaatgtaat tttaaaatta taattatttt attttttatg tatatttatt 12480
tttatatatg agtatatatg taatatatgt atgtaaatat cgtaaaagaa gtgtatatgt 12540
aaaattatat ttattttata ttatggtaat ttgtagagtt tttaaagtta ttgttgttta 12600
tatatgattt ttgtttaatg tgttgtgttg attttagaat ttgattttta aaaaggatgt 12660
agtttttttt tatgggtatt atggatatta ttttttaagt ttttttttta gtatttgtat 12720
taagttagga attttatggt tttgtttaat atttgataaa ggtcgagaat ttgttaatga 12780
taggcggttt atatatattt tttatattag ggtttttatt agttttttgg gtagagttaa 12840
gagatggggt attttagttt tgttaaaaga gggaggtttt tagttttgtt tttggttttt 12900
ggtttttttt tttttttttt aggatagttt tagaattata ttggttagaa gcgagggttg 12960
ataaggttag tttttttttt tagtattttt taagttttat ttagaaaagt gataggtggg 13020
ggagagtata ttttatattg ttaaggaagg atatttgggt ttagtgtgta gttttttaga 13080
ttttgttatt ttagtgaggg ggttttggga ttttaaatta ttatggaagt gtttataggt 13140

ttttaatatt ttagatttaa gcgttttttt atagttttga ttagtttcgg taattaaatt 13200
tttaaagaaa atataaataa ttgttagaaa ggtttatttt tttataatt ttttgtatgc 13260
gttagagggt cggtttttta gatgtataaa ttgaatgaaa tttttcgtga aagttagata 13320
agattatagt tttttaggtt ttatttttgt tttttaaatt aagattttta gaattttttg 13380
gttatttttt aataatttat tattagggag gttttttttt ttttaaaatt ttattcgatt 13440
atgttttttt attttttttt ttaaatgttt aaattttttt tttcgttatt tttttttttt 13500
ttttttttaa ttttaatttt tttagggttt ttaatatttt tttgaatttt tcggtatttt 13560
gtttttagaa taggtggagt attttttttt tttataaaat gttttttttc gtggtttaga 13620
gttatttttt tacgggtttt ttattttcgt tattttttgt ttatttatta gtttaggttt 13680
tttgtgattc gtttcgttaa tttttttgtt tttttttttt tatgatcgag ggtttatttt 13740
tgtacgtaat ttgtttttaa agtttcggga tttgtttttt taattttttc gggtgttgtt 13800
ttcgtttttt ttaaaattta ggtttgagtt tgtttttttt aagggtgttt ttattttatc 13860
gcggtatttt tcgtttttta gattcggttt cgtcgtattt attcgattag agtacgagtt 13920
tgtcgtgcgt ttttttttgg gagttcgagt tttcggttag tagtgtggag gtggtttcgt 13980
agggtagtat cgagtttagg tatacgttgt agcgtagcgg ttcgtagggg gtagttcggt 14040
cgtagtggtt tagcggcggc ggagggttag ttatcgtcgc gttgttgttc gcgttttcgt 14100
tcgcgtttcg aggtttaggt tcggtcgcgt tttcgttcga ggtcgttttt tcgggtcggg 14160
ttttttagta gtagtagtag tagtagtttt aggagcggga gtttcgggtt ttcgcgtttg 14220
ggcgggtagt ggttatggtt aatttcgtta tttaggaaag gtttcggcgt tttcgtttgt 14280
ttttcggagt ttttcggtta tacgtacgat ttcggcggtc gcggaggcgg tttaggcgat 14340
ttgtgcgttt agagaattcg ggttcgggtt ttttcggacg tttcgcgcgt ttcggttttt 14400
cggcgtttaa tttcgtaaat tttggaattc ggggtttatg ttgggttgcg gaggttcgga 14460
acgggatgta cgatttttta agttttagtt tcgtaattta agttgttttt agttttagga 14520
gatttttggg tttttaattt ttagttttat tttttttttt attattaaag ttattttttc 14580
gtttcgcgag ggaggttaga cgcgggtcgg gggtgcgagt gtagtttcgg tttcgttttc 14640
gcgttagttc gcggtttagt agttattttc gttttagcgc gtcgtagttt ttcgaatttt 14700
tttcgtcggc gattcggttt ttttgttgtt ttagttcgtt gggttttttg gaatgtacgg 14760
gtttcgcgga gttaagttcg gtttttttgc gtcgaggttg ggggagggat tggacgaagg 14820
agcggagagg tggggagggg aggcgagaga ggaagagcgc gagcgttagc gtttttggcg 14880
tttggagggg ttcgggttag ggggatttgg tgatttcgtt tgtcgtcgtt tcgcgttcgg 14940
gggaaagaga tgagtggaaa tcgtgggtcg gatttgtaag gagagattcg gcgggtattt 15000
tgtttggttt gaggtcgttt gtaggaagcg gatttttttt ttggtttagg atgggaaaga 15060
taggggatgt ttgaagttaa cggggatttt tgttttattt ttgtttcgtt ttttaggttc 15120
gttagttttt tttgttttgg ttagattttt taacgtattt cggggtttta tggagaagaa 15180
tttgggtttg gggtttgcgt ttttttaaat ttttttagtt cgggattttt gagagagacg 15240
attttagagt tatttacggt agttgttttg gttgttgtta tttgcgggaa taggtgtttg 15300
ggaaataaat ttgttaatat taagttttta ttgttataaa tatgtgaagt ttgaattgtg 15360
gtttttaggg attggttttg gttatgtgag aaggaaatta gttttttttt gatggttttg 15420
ttgtttattt gttttgggag gtttttgttg tttttttttt ttattttgtt tattttgttt 15480
ttttttattt ttttgagtat tatatttttg gttattatat ttaatttttt attttagtag 15540
tttatatttg tataattaag tataatttag tataattata aggttttaaa taggagagta 15600
gatttgtatt gagtaatttt ttttttgttg ttgatcgggt tgtatattag ggtgggtttt 15660
ttttttttat ttttggaaga tttgtttatg gtttagcggt agtggattag ttttgttatt 15720
ttggatagtt taagtgtata ttagttattg tattttaatt atattaattg gagagagaga 15780
gagaatttaa tttaatttag taagtttttt tgaaatttta gtatgtatat agtatagaga 15840
tattttggag gaatttgtag atttttattt ttttgtggat atatggtttg tttttaggaa 15900
atatagtatg atagaatagt aacgtttttt tttttaggtt agggatttta ttttattttt 15960
ttagtgttta taaaatattt agtttatttg agtatatata gtaattattt gataaataag 16020
ttgttaaaat gagatgtatc gtgagtatgt tataggaata ggggatagag gttagtgtgg 16080
atagaagtgg aggaggtggg ttttgaattt aggattaaag tgtgagtagg aggagttaga 16140
ataaggatat agataagtag ggaaattgat tggtaagaag ttttaaataa tatatagaat 16200
taagggttaa tttttttgat agtaaggaaa ttttaaagtt ttgattaggg gaatgatatt 16260
aatttttatt ggttaatatt aggtgatatg gtttggattt gtatttttat ttaattttat 16320
gttgaattgt aatttttagt gttggaggtg aggtttggtg ggaggtaatt ggattacgga 16380
ggtagttttt tatgaatggt ttagtattat ttttttcgtg ttgtttttac gataatgagt 16440
gagtttttac gaggattgtt taaagggttt ggtatttttt cgtttttttt ttttgttttt 16500
gttatgtaag atgtttcgtt ttttttttgt tttttattat gattagaagt tttttgaggt 16560
ttttttagga gtagaagttg ttatgatttt tgtatagttt gttgaattgt gatttaatta 16620
aatttttttt ttttataaat tatttagttt gaggtatttt tttttttttt tagacggagt 16680
tacgttttgt cgtttaggtt ggagtatagt ggtgtgattt tagagtatag tagtgtgatt 16740
tcggtttatt ttaattttta ttttttaggt ttaaatgatt attttgtttt agttttttga 16800
gtagttggga ttataggtat atgttattac gttaggttaa tttttgtatt tttagtagag 16860
acgaggtttt attatgttgt ttcggttggt tttaaatttt tgattttaag tgatttattt 16920
attttagttt tttaaagtgt tgtgattata ggtgtgagtt attgtatttg attagtttta 16980
ggtatttttt tatagtaatg cgagaataga ttaatatata aggtaatatg aggatggtat 17040
gaagtgtaag aggttggaga taaggattta ttttaaggaa agtttttggt gatttatgga 17100

agaagtgaaa ttagattatg ttattagggt ataatatttt tattggaaaa ataatttgaa 17160
atgttatgat ttattttagt tatttttttt tgtgatataa taaaatggtt gttataatat 17220
gtttaagttt aagttgtaga gaaagttgtt ttggagtttg atagtatatt cgatgttttg 17280
ttttgtgaat tatttgtatt tattttttat ttgattgtgt gtgtgtgttg ttttgtttgt 17340
ttttgttttt tttttatttt ttttgagtga gtaagagtaa aatatttggt tgtggtagtt 17400
tcgaagtttt gtgggatttt tttaatattt tatgtattag ggaagaattt atatatgtgt 17460
ttaggtaggt ttttattttt ttattttaaa tgttagtagg taatttttta aggatatagg 17520
tttttttatt ttttgtgtga ggttttttag gaagtatgag gaagattcgt attgtgggaa 17580
tataatattt ttatattgga gatagtagag gtaaattgag gttattttaa gatattattt 17640
tttagttaga atgttttttt ttttttgagg ggagaatata ttataagttt ttaattttgt 17700
gtgtgtgtgt gtttgtgtgt gtgtgtgtgt atatttatgt atatatgtgt ttttatttat 17760
tagattttag tatttttttg tgggaatttt gttggtttag taaagggtag taggaaggta 17820
gattgagaaa atgttgttag tattgtttat agttattagg ttacgttttt attagtgata 17880
gttgtagcga tgtgttattt tttagatata gaaggaagta gaaagagatt taggaatttt 17940
gttatttgag agaaggaaat tgtttagtgg ggattgggga gagggaattt tttagtcgtt 18000
cgtgaatggt atttaggttt ttggagaaag tttatgtatt tttttttgta gatatataag 18060
attgttagtg tttgttagat tacgtagagt gatttggtat atttaggttt ttagtttggt 18120
ggtaggttgt aaagtgtttt ttttttaagt aagtttgagt tatattttat ttagtttttt 18180
aagagtttta gttttaattg tatgttttaa tataattttt tttttaggga tgagaggttg 18240
ttttgtgttg ataatgtatt ttagtatttg atttgttata agtagttttt tgatttttga 18300
gttattttta taaagtattt tggtattttg aggttataaa tttaatgttg attgttattt 18360
gtttgtgggt tttttagtta aagagggttg atgtgaacgt atttatgggt attattgaga 18420
ttatggttgt tttttgtggt ttagttgtta tattggtaaa attcggaata taattgttgt 18480
agtgttttaa tttttttttt ttttagatgg agttttgttt tgttgtttag gttggagtgt 18540
agtggtgcga ttttaattta ttgtaaattt cgttttttgg gtttaatcga tttttttatt 18600
ttagttttcg gaatagttgg gattatagat ttttgttatt atgtttagtt aatttttgta 18660
tttttagtag agatggggtt ttattatgtt ggttaggttg ttttttaatt ttcgatttta 18720
ggtgatttgt ttatttcggt tttttaaagt gttgggatta taggtgtgaa ttattatatt 18780
cggttgtgtt ttagtttttt ttttttttag tgttttttat tttgtaatta agagtattga 18840
gttaaaggga aagggtaaaa tggtaaaggg gtttaataag taattttttt agagagatag 18900
tacgatagat ttagaattaa gagttttgag ttgggtttta ttagttatgt atttttgagt 18960
aatttatttt gagttttaat tttttttttt ttaaaaatga gaaaattatt cgttgtgttt 19020
ttatatatta gtgatgaata atttgaaagg gaaattaaaa tagtaatatt atttataata 19080
gtatttaaaa taattaaata cggttgggtg cggtggttta tgtttgtaat tttagtattt 19140
tggaaggtta agatgggtgg attataaggt taggagttcg agattagttt gattaatatg 19200
gtgaaagttc gtttttatta aaaatataaa aattag 19236

<210> 245
<211> 5506
<212> DNA
<213> Artificial Sequence

<220>
<223> chemically treated genomic DNA (Homo sapiens)

<400> 245

tttttatatt ttttagtgtt ggtttaatag ttaaatgatt ttttttgatt tatatttatt 60
gagtagttat tatgttaggt aggggagatg tgtgtgtgta tttttattta atttttttat 120
tatttgggga tattaagggt gtataagatt attaatatcg tatatatgta aaagtcgagt 180
tttgagaggt taatagcgtg atttgtttaa ggttttttgg gtattagtgg taggattttt 240
tgattttaag ttatttttta ttatattcga gattgaggat gatatagttt tatttaaagg 300
tggaggtggt ttagaaaatg tttaggggag tttttatagt tattttagcg tatttagatt 360
cggttgaaga tttatagggt agtttttgtt tgttattatg ttgttttcga ttattaattt 420
gtgttcgtcg gttttttatt tttttagtgt ataggttttt ttttgttata ttttaaagtt 480
tttcggatat tttagtataa tttgtttggt tggtgggttt agtatttaag attttttttt 540
tatttttggt gattttggtt ttggaagggg gttatttata gttttaggga tttagagatt 600
ttgtgaagat tttttaggga attttatgtt tgtattttag ttttagaaat ttagaattaa 660
ttagtttggg gtgggattta ggttttagta tgtttttttg ttgttgttgt tgtttgtttt 720
tatgagatag agttttattt tgttatttag gttgtagtgt agtggtgcga tttcggttta 780
ttgtaatttt tgttttttga gtttaagtga tttttttgtt ttaggttttt acgtagttgg 840
gattataggt gcgtgttatt atgtttagtt aatttttgta tttttagtag atatggggtt 900
ttattatgtt ggttaggttg gtttcgaatt tttgttttta ggtgatttgt tcgtttcggt 960
tttttaaagt gttgggttta taggaatgag ttattacgtt tggtttagta tgtttttttt 1020
tttttttttt tttttttttt tttgagatag agtttcgttt tttttgttta ggttggagtg 1080
taatggtgta atttagtttt attgtaattt ttatttttcg ggtttaagtg atttttttgt 1140

tttagttttt taagtagttg ggattatagg cgtttgttat tatgtttagt taattttttt 1200
tttttttttt ttttagtaga gataaggttt tattatgttg gttaggatgg ttttaatttt 1260
tcgattttat gatttgttta ttttggtttt ttaaagtgtt gggattatag gtgtgagtta 1320
ttatatttaa ttaaggttta gtatgttttt tttaatttta taagtgattg taaatttaat 1380
aagaattaga gttagtttta ggtaattgat tagaaatgta gaattttagg ttttatttta 1440
atttgaagaa ttttagtgcg tttttttatt tgagaagtat tgttttacgt taaatttcgt 1500
attcgtaagt taggttttgt gaaaggtttt cgtagggaaa ttggaggtag gagggtatat 1560
agtttagata gaattaaaga gtggaggtta ggatttttta ggtcgtattt atatagtatt 1620
tagaggtgtt aaacgttttt tttgtgtttt tttattttat tttagttttg ttttttgaaa 1680
gttaggaaga tagttttatg tttgtgtggg gaaattgagg ttggggtaga gttttaattt 1740
gattagatta ttttttattt ttgtatggtt tttggttttg tagttagtag attttttgat 1800
ttttatttta gtgtattttt tagggtaggt agggagggag ggtgattatc gttttacgtg 1860
ataggtgagg gattagggat ttagtggggg aatataggcg gagtttttta taaagtcgga 1920
ttagtttttt taggaagagg atttgggagt tgggggtgga aggttattgg ttgatcggag 1980
gtagttttgt taggtagtgt tattagtggg ttttacgttt gattgatagg tatttttttt 2040
tttttttttt ttttggggtt tattttttga gtaaattcgg gatttgtttt aataaaagtt 2100
aggttgagtt ttaggaaaat tttgttgttt cggaaaggtt tttttgggtt atggaaggaa 2160
gtttggattt ttattattat atttagatcg tagatttttt tttgttgtag aataaatatt 2220
ttgagagaag gtaggttata gagtttatta gatattatat tttgttatgg acggtgataa 2280
atttttattt taggatagag aggtgatggg gggtggggta aggaggttat aagtattttt 2340
ggagaagggt taagaatgag ggggtaaagg gatttttgtt atttttagta gggaaaggag 2400
gagaaggtga tattggtttt tttgttgttt tgttggggta gaggtttagg tgtttttagt 2460
taggattttt ttttttttta agttttttgt tttttaggaa tgattatggg ttttttattt 2520
ttgtttgttg gagagtattg ggtttttagg gtttgggatg ggatggaggg gattttagtt 2580
gttagtatgg tgggtaggga tgtgttcgta gtaggggttt taattatata tatatagtat 2640
tggttatttg atagtttttt ttattcgttt tgtaggttta tttgttttat ttaatattgt 2700
ttagtaaaaa gtttagtata gttttgagag gaaggtagtg tttgtgcggg gataagggag 2760
agagagaggg agtggttttt tttgtttagc gtggtttttt gtagatagag cgtgtttatt 2820
gttattttta tttttatttt ggtatagttg gattaaggag ggtaaaggag gaagtaggga 2880
gttagagagg gggtagttgt gggattttat attttatttt ttaattttgg gttttttttt 2940
tttataatta ttttttttgt tttaggcggg ttttaggtta gttatagata tagtagagag 3000
cggggaggtt tttttttttaa agaggaggaa ttgagggtgg gaaggatgag tggggattta 3060
tcgggagaga gagatgttag gtaggtaggt agcggcgggg tagtagcggg tgttggaggc 3120
gattgtaggg tagtcgggat acgacggtag atgttcgttt ttcgtttttt tgtttattta 3180
gggcggtttt ttttcgggcg gttttgaggt cgagcgggcg ttgttaaaaa gttttttatg 3240
tgtgtgtgtg ttaattatat gattgtcgtt tatttgtatt tcgaataaag atagtttatt 3300
gttttaaggt ttcgaataag agtttgggta tagggaagag aagggaggcg gggggaggag 3360
ggttggtagc gtcgggggtg gcgtcgagga aaattggggg gagattgttt aaggttagta 3420
agcgagagaa agagaaaatt tttattaaat ttttaattta ttgggtttcg aggttttaga 3480
tatattggtt cgattgtttg gttggttgtg tgtgtgagta tgtgtgtgtg tgtgtgtgtg 3540
tgtgtgtgtg tgtgttttag tgtttgtttt ttttattttt aatatgtttt tgtttcgtgt 3600
gattatttta ttgtgtttta ttttttattt tttatatttt tttggatttt tttaagtatg 3660
aggttttgtt tttttagatt ttttgtagtt tattttagat aggatttttt ggggtggttt 3720
tggggttttg ttttgttgtt gaggagttta gaacgttata tggttgttaa tgcgatttta 3780
gttttattat ataatttata tatattttat ttcggtttta atatttcggc ggggttgttt 3840
ttagttaaga gaatagtatt gcgatttttg gaggagaggt taaggaaaga gagagaaaaa 3900
ggtgttaacg gaggatgggt aggaaagaaa aggggaggga gggagaggtg ggtagaggag 3960
ggaagaggta gggtaagttt ttattttttg ttggggtggg ggagtagagg atttcgtgat 4020
aattaggatt aggatttttt tatttttttt taagataggg tgagtttagt gtggaaggag 4080
ttgggtttta gtaggtagta atttaaaatg tttttaagat ttattgagag agggttttta 4140
atttgtttta ttttggtttt ttggtagttt tagttttgtg tttttatttt gaattagatt 4200
agaacgcgcg tgtgttgggt gtttgtagag aagggattag gggttaagtt ggggttttgt 4260
attttttaga gggatttttg agaagttttt tttattgtgt aggatggggt agaaagggtt 4320
gtttgggaaa aggtaggagg tagggtgagg agttgggtat tagtgtttgg tatatattag 4380
gtatttaata ggatttcgta gacgaagtag ttagggttag ttatttttac gggtttttag 4440
gatttttatc gtttttacgt ttttttttag ttttttgtta agtgtttatt ttgttatttg 4500
aggtagacgt taggagtttt ggttgggttt aatttttttt tatttaatag gttttgattt 4560
tttagtttat attttttttt ttttcggttt aatttttttt ttatttttta gattaagttt 4620
aagataagtt ttagtaagat gggtttgggt agtagggtag ggagttgggt gggggggttt 4680
gggtttcggg tggaggagtt ttgagtgttg gggatgaagg aaagtttgga ggttttattt 4740
tttgtttttt tttttttttt tttttttttt tttttttttt tttttttttt ttttagtttt 4800
tcgtagcggt ttaggtttag tcgtttagtt agtttcgggt tgttcggtta gggtggttgg 4860
tggtaaggat ttaggtttcg ttttaacgag gtcgtggttt gaggtttagg gtttttcgtt 4920
tttttttttt agtttattag cgttattttt tagtttcgag ttggatcgta tattttggga 4980
tacggttttt tattttttaa ggacgagttt tagattggag gagaggttcg aggaggtgag 5040
tgcgtagttt gttttttttt ggttcgttta ggtttgggat tttgttgtga ggtggagaga 5100

ggcgatattt cgtttttgtt ttttatttgg ttttttttta aatttcgttg gggtaggttg 5160
agggaagaag gaaaggtgag agaaaaggtg ggaaatttta ggggtcggga ttagagtaaa 5220
ataaaggttt cgtttttttt tttttattta ttaataaatt tttatttaaa aaaggaggtt 5280
aaaaaaaaag ttatttatat taagagacgg ttgggattag gtggtgggaa gatatagagg 5340
cgggacgtat ttatttagag tttggtatgt gcgtgggttt taggggttag atgggtttag 5400
ttggagtagg aagggtcgtt atttcggggt tgtgtttacg ttttgtttcg ggtcgttttc 5460
ggggttgcgc gcgcggtcgt tttatcgcgt ttttttttta tttagg 5506

<210> 246
<211> 5506
<212> DNA
<213> Artificial Sequence

<220>
<223> chemically treated genomic DNA (Homo sapiens)

<400> 246

tttggatgga aggagggcgc gatggagcgg tcgcgcgcgt agtttcgagg gcggttcgag 60
ataggacgtg ggtatagttt cgaggtggcg gttttttttg ttttagttaa atttatttgg 120
tttttggggt ttacgtatat gttagatttt aggtgggtgc gtttcgtttt tgtgtttttt 180
tattatttgg ttttaatcgt tttttggtgt gggtggtttt ttttttagtt tttttttttg 240
ggtggaggtt tgttgatgat aaggaagaaa aaaacggagt ttttattttg ttttaatttc 300
ggtttttgga atttttttatt ttttttttta tttttttttt tttttttagt ttgttttaac 360
ggggtttaga ggagagttag gtggggggta agggcggggt gtcgtttttt tttattttat 420
agtagggttt taggtttggg cgggttaggg aggagtaggt tgcgtattta ttttttcgga 480
tttttttttt agtttggggt tcgtttttag gaagtggaaa atcgtgtttt aaggtgtgcg 540
gtttagttcg gggttgggag gtgacgttgg tgggttggga gggaggggcg gggggttttg 600
agttttaggt tacggtttcg ttaggacgga gtttgaattt ttattattaa ttattttggt 660
cggatagttc gagattgatt gagcgattga gtttgagtcg ttgcggaggg ttggggaagg 720
aaggaggggg agagggggga ggggagagga gggagggaag ggtagaaggt ggagttttta 780
ggtttttttt tatttttaat atttagggtt tttttattcg gagtttaggt ttttttattt 840
agttttttgt tttattgttt aggtttattt tgttgaggtt tattttgggt ttagtttggg 900
aggtggaggg gaggttaggt cgaggaagga agagatgtag gttggagggt tagaatttgt 960
tggatggaag aaaattgggt ttaattagga tttttaacgt ttgttttagg tggtaggatg 1020
ggtatttggt aggggattag ggagagacgt gaaggcggtg agggttttgg gggttcgtga 1080
gggtggttgg ttttagttgt ttcgtttgcg aaattttgtt gagtgtttag tgtgtgttag 1140
gtattgatgt ttagtttttt attttgtttt ttattttttt ttaggtagtt ttttttgttt 1200
tattttgtat agtaagagag attttttagg gatttttttg agaagtgtag agttttagtt 1260
tgatttttgg tttttttttt gtagatattt agtatacgcg cgttttggtt tggtttaagg 1320
tggaaatata agattaaagt tgttaggagg ttaaggtggg ataggttggg ggtttttttt 1380
tagtaggttt tagaggtatt ttgggttgtt atttattggg atttagtttt ttttatattg 1440
ggtttatttt gttttgggag agggtagggg agttttagtt ttgattgtta cgaagttttt 1500
tattttttta ttttaataag gggtggggat ttgttttgtt tttttttttt tttgtttatt 1560
tttttttttt tttttttttt ttttttgttt atttttcgtt gatatttttt tttttttttt 1620
tttttggttt tttttttaaa ggtcgtagtg ttgttttttt aattgagggt agtttcgtcg 1680
gggtgttgag gtcgaggtgg ggtgtatgtg ggttatgtaa tggagttggg gtcgtattgg 1740
taattatgtg acgttttgag ttttttaata gtagggtaaa gttttaagat tattttaagg 1800
ggttttgttt ggagtgggtt gtaaagggtt tggggaggta aggttttatg tttgggaaag 1860
tttaaagggg tgtagggggt gggaaatggg atataatgag gtagttatac gaggtagagg 1920
tatgttgggg gtggggggag tagatattgg aatatatata tatatatata tatatatata 1980
tatatatgtt tatatatata attagttaga taatcgggtt agtgtgtttg gggtttcgga 2040
gtttagtgaa ttaggagttt gataagggtt tttttttttt ttcgtttgtt ggttttggat 2100
aatttttttt tagttttttt cgacgttatt ttcggcgttg ttaatttttt ttttttcgtt 2160
tttttttttt tttttgtgtt tagatttttg ttcggggttt tgaaatagtg agttgttttt 2220
gttcgaaata taggtgaacg gtaattatgt gattaatata tatatatata aaaagttttt 2280
taatagcgtt cgttcggttt tagaatcgtt cggagaggag tcgttttgga tggatagagg 2340
gacgagggac gagtatttgt cgtcgtgttt cggttgtttt gtagtcgttt ttaatattcg 2400
ttgttgttcg ttcgttgttt gtttgtttgg tatttttttt tttcggtggg tttttattta 2460
tttttttatt ttttagtttt ttttttttga ggaaaggatt ttttcgtttt ttgttgtgtt 2520
tgtgattggt ttgagattcg tttggggtag gaggggtggt tgtggaagga aagagtttag 2580
agttaggagg tggaatgtgg ggttttatag ttgttttttt tttaattttt tgtttttttt 2640
tttgtttttt ttggtttagt tgtgttagga tgggggtggg ggtagtagtg ggtacgtttt 2700
gtttgtagga agttacgtta gatagaaggg gttatttttt tttttttttt tttgttttcg 2760
tatagatatt gttttttttt tagggttgtg ttgggttttt tgttgagtag tgttgggtga 2820
ggtaggtggg tttgtagggc gggtgggaag ggttgttaga tgattagtgt tgtgtgtgtg 2880

tggttggggt ttttgttgcg aatatatttt tgtttattat gttggtaatt gggatttttt 2940
ttattttatt ttaggttttg ggaatttagt gttttttagt aggtaagggt gaaggattta 3000
tagttatttt tgaagagtag ggagtttgga gaggggaggg attttggttg aagatatttg 3060
ggtttttgtt ttagtaggat aataggaggg ttagtgttat tttttttttt tttttttgtt 3120
gggggtggta ggggtttttt tattttttta tttttgattt ttttttaggg atgtttgtga 3180
tttttttatt ttatttttta ttattttttt gttttgaagt gagggtttgt tatcgtttat 3240
ggtaaggtgt ggtgtttggt aggttttatg atttgttttt ttttagggtg tttattttgt 3300
agtagagagg ggtttgcggt ttgagtgtaa tggtggaaat ttaggttttt ttttatggtt 3360
taaggaggtt ttttcggagt aatagggttt ttttgggatt tagtttggtt tttgttaagg 3420
taggtttcgg atttatttag aagatgggtt ttagagagga ggagaggaaa ggaatatttg 3480
ttagttaaac gtgaagttta ttgatgatat tgtttggtag agttgttttc ggttagtagt 3540
gattttttat tttttagttt ttaagttttt tttttgaggg agttggttcg gttttatgga 3600
aggtttcgtt tgtgtttttt tattgggttt ttggtttttt atttgttacg tggggcgatg 3660
gttatttttt ttttttgttt gttttgaagg atatattgag atgaaggtta ggaaatttgt 3720
tggttgtaga attaggggtt atgtaggagt gagggatggt ttggttaagt tgaggttttg 3780
ttttagtttt agttttttta tataggtatg agattgtttt tttgattttt agaggatagg 3840
gttgagatgg gatgagggaa tatagggaaa gcgtttagta tttttgagtg ttgtgtggat 3900
gcgatttggg gggttttggt ttttattttt taattttatt tggattgtgt gtttttttat 3960
ttttagtttt tttgcggggg ttttttatag ggtttggttt gcggatacgg ggtttggcgt 4020
agggtagtgt tttttaaatg ggaaggcgta ttgggatttt ttaggttggg gtggggtttg 4080
aggttttgta tttttaatta gttgtttagg attggtttta atttttgttg aatttataat 4140
tatttgtgga gttaagaaaa atatattggg ttttggttgg gtgtggtggt ttatatttgt 4200
aattttagta ttttgggagg ttaaggtggg tagattatga ggtcgagaga ttgagattat 4260
tttggttaat atggtgaaat tttgttttta ttaaaaaaaa aaaaaaaaaa aaattagttg 4320
ggtatggtgg taggcgtttg taattttagt tatttgggag gttgaggtag gagaattatt 4380
tgaattcggg aggtggaggt tgtagtgagt tgagattgta ttattgtatt ttagtttggg 4440
taaaaagagc gaaattttgt tttaaaaaaa aaaaaaaaaa gaaaaaaaga aaaatatatt 4500
gagttaggcg tggtgattta tttttgtaag tttagtattt tgggaggtcg aggcgggtag 4560
attatttgag ggtaggagtt cgagattagt ttggttaata tggtgaaatt ttatgtttat 4620
taaaaatata aaaattaatt gggtatggtg gtacgtattt gtaattttag ttacgtggga 4680
gtttgaggta ggagaattat ttgaatttaa gaggtagagg ttgtagtgag tcgagatcgt 4740
attattgtat tgtagtttgg gtgatagagt aagattttgt tttataaaaa taaataataa 4800
taataataaa aaaatatatt ggagtttaga ttttatttta gattggttga ttttgaattt 4860
ttggggttga ggtataagta taaagttttt tgggaggttt ttatagagtt tttggatttt 4920
tgaggttgtg aatgattttt ttttagagtt aaaattatta gggatggagg aggggttttg 4980
ggtattggat ttattagtta ggtaggttat attggggtat tcggagagtt ttggggtgtg 5040
gtaggggagg gtttatgtat tgggaaggtg gagggtcgac gggtataggt tagtggtcga 5100
gggtagtata gtgatagata gaagttattt tgtgggtttt tagtcgagtt tgagtgcgtt 5160
ggggtggttg tgggagtttt tttgagtatt ttttaggtta tttttatttt tggataaggt 5220
tgtgttattt ttagtttcgg atgtagtgga aagtgatttg aagttaagaa attttgttat 5280
tagtatttaa ggggttttag gtagattacg ttattaattt tttaggattc ggtttttgta 5340
tatgtacgat gttgataatt ttatgtattt ttgatatttt taagtgatga gagaattaaa 5400
taagaatata tatatatatt ttttttattt agtatagtga ttgtttaata aatgtgagtt 5460
agaggaaatt atttagttat taggttagta ttgggaggtg tggagg 5506

<210> 247
<211> 6874
<212> DNA
<213> Artificial Sequence

<220>
<223> chemically treated genomic DNA (Homo sapiens)

<400> 247

gtttattgta atttttattt tttgagttta agcgattttt ttgttttaga ttttttagaa 60
gttgggatta taggtgttcg ttattatatt tagttaattt tttttgtatt tttagtagag 120
atagggtttt attatattgg ttagtgtggt tttaaatttt tgattttaag cgatttattc 180
gttttaattt tttaaagtgt tgggattata ggtatgagtt atcgtattcg gtttattttt 240
gtttaatttt aaaagaaaaa tataaaagtt atattgattg ttaaagaagt tatatggtat 300
aaatttatat aaagtaaaaa aatatatttt ttttatttta aattttattt tttataggta 360
agtattatta gtgtttggag tgtggttttt tagatttttt ttgggtataa tgatttataa 420
attttttttt attattgtta atgttgttat tataattgat attttttttg tttttgtgta 480
aaattatgaa tttttgagta aaggttaatt ttaaatgaaa atagatttag ggtattttga 540
tagtaatttt tttttttttt ttgagatagg gttttttttt tatttagatt gagtgtagtg 600
gtgttattat ggtttattgt agtttgggtt taagtaattt ttttatttta gtttttttag 660

tagttgggat tacgattata gatgcgtgtt attgtgtttt agtatttttt tttttttttg 720
agatgaagtt ttattttttt atttaggttg gaatgaagtg gtacgatttt ggtttattgt 780
aatttttttt ttttagattt aagtgatttt tttgttttag ttttttgagt agttgggatt 840
ataggtatat attattaggt tcggttaatt tttgtatttt taggagaggt gggatttttg 900
ttatgttggt taggttggtt ttgaattttt gattttaggt gatttatttt tttaggtttt 960
ttaaagtgtt aggattatag gtatgattta ttttgtttgt ttgtgttttt tttttttaag 1020
agatggggtt ttattgtgtt gtttaggttg gtgttaaaat tttcggattt aaattatttt 1080
tttattttga ttttaaaaag tgttgggatt ttaggtgtga attattgtat ttagttatga 1140
tagtaatttt taaaagtaat ttttaatttt tgtttgttat tagttaggtg ataaaaatta 1200
gaagaaagaa agaaaggtta gggtggaatt tgtgtggtgt gagggatttg gttgaaattt 1260
ttatgtattt atagaggttt tttatggttt ggggtggata tgagttttta tttgttgtaa 1320
ggtgttattt tgattaggtt ttttcggagg aatattttaa ttttatattt tagagtagtt 1380
aggtaggagt ttatttaggt ttagtaggtt gcgtggagtt tatggtaaag tggagagttt 1440
tttttatggt tattattagt tttaggttat aatgtttttg tgggtattta ggtttagtgt 1500
tattaaaaat tttgattttt ttgagagagg ttagatattt agattttttt ttttttttga 1560
aaaggagttt tatttcgtag tttaggtcgg agtatagtgg ttttatttag gtttattgta 1620
atttttgttt tttaggttta agtgattttt ttgttttagt ttttcgtgta gttgggatta 1680
tagatgtgtg ttattatgtt cggttatttt tttatttttt tagtagagat ggcgttttat 1740
tatgttggtt aggttggttt tgaatttttg attttaagtg atttatttat tttagttttt 1800
taaagtgttg ggattataga tatgagttat tacgttcggt tagatattta gatttttatg 1860
taaattttta tgatttttaa atgttataag gtttaaataa aatattttta tgagatagtt 1920
tttgattttg attttatttt tagtaatatt tttggttttg gaatttgtag taatttaata 1980
gtgtgggtag aggttttttt ttgatttttt gtggtgataa aggatatgaa gatgggttat 2040
tttaagtaga tagagaagtt gtgatattaa tattggtgtg taatgattta ggagttggga 2100
gaagttgaag agagggtagt ttatttgaag ggaagttaag gattgcgttt tttttagata 2160
ttttttttta tagatgtgat agagagtagt tgtttttgtt gagggatgtt atttttgtat 2220
gtgtttaaat tagggggcgt aaatgaaagg gtttttagag ggtaggtggg aatgaaattt 2280
tagagttttg gttgtaatat tatggtgttt agataaaata tgattttagg tattatttgt 2340
ggtttttaaa atttttttgg aatttattta tttgtttgat ttgttttttt ttttgggtta 2400
atacgtttta tatgtttaag tatttgttgt aaagagtaaa ttagtggagg gaggggagat 2460
ttttttaaaa ttattggggg ttgggtgtag tggtttaggt ttttaatttt agtattttgg 2520
gaggtcgagg tgagagaatc gtttgagttt agaagtttga gattagtttg ggtaatatag 2580
ggagatttta tttttatatg ataaaaaata tatattatat tttatttatt atatatataa 2640
aatatatatt atattttatt gattatttgt atatatttta ttttagtatt ttttaagttt 2700
ttggattgtg atttatagta agaaatgtag ttaatattgt tatttattat atatatatat 2760
tgaattatga gaaattgata atattttatt tttttgattt ataaaatggt aattttgtag 2820
ggtttaattt atatataatg taaataaata ttttatgtaa taatatttgt ttttgttttt 2880
tgtgatattt attaatttat ttttttaaat gttgattgta gtatattgaa ttgatattat 2940
tattattatt agtaatgatt aggttataat taattgtttt atttaatttt tataattttt 3000
cgggtaggaa taattattgt tttttatttt tttaaatatg ttaagatgtt ttatttttag 3060
gatgaggtga tttattttag gtaattttta ttgtttaatt attgattaat tattttgttt 3120
tttagttttt atgttattaa atttgtatta agaattttat ggaataggtt cggtatggtg 3180
gtttatgttt gtaattttag tattttggga gatcgaggtg ggaggattat ttgaggttag 3240
tagttcgaga ttagtttgga taatatggcg aaattttatt tttattaaaa atataaaata 3300
attagttagg tgtggtggtg ggtatttgta attttagtta tttgggaagt tgaggtagta 3360
ggagaatcgt ttgaattggg gaggtagagg ttgtagtgag tcgagatcgt gttagtgtat 3420
tttagtttgg gcgatagagc gagattttgt tttaaaaaaa aaaaaaaaaa aaatttaggg 3480
aatggatagt agtattgatg aatattgcgt ttggagagat tagattattt gttatttgtt 3540
tttaggtata gggtttatta agaggtagat tttagattta aataattttg taatagtaaa 3600
gtttaagtta tttttattgt tttggagaaa agatttagat ttagagtttg aattttattt 3660
tgtagtattt tagttttaat tttttaagtt tttttttttt tttttttttt tttttgttgg 3720
gtacggtggt ttatgtttat aattttagta ttttgggaag tcgaggggga aggatcgttt 3780
gaggttagga gttcgaaatt agtttgggta atatggtaaa attttatttt tataaaaaat 3840
ataaaaatta ggttagagtg gtggcgcgta tttgtagttt tagttacgtg agaggcggag 3900
gtgggagaat cgtttgaatt cgggaggtag aggttgtaat gagtttagat ttcgttattg 3960
tattttaggt tgggcgatag agcgatattt tgtgtgaaat tttttttttt ttttttaacg 4020
ggttttttag agaagtgtgt gtatgtgcgt gtgtgtgcgc gagcgtgttt gtttgggttt 4080
aaattttttg tcgggttata tttttttaag tttttgtatt ggttgtaggg tgggttttat 4140
tttcgggacg tttttttttt taagtttagt ttgtagttgg aagtttttat tgatttttat 4200
tttttttttt tgattttcgt tttttttttt tgttcgtttt aggattggga ggtcgatttt 4260
tttttttcgt tttttttttt attagttttt tttagatgta tgtttgttaa agatttttta 4320
gtgtagagga tgatgaatga agattttcga gttagttcgg tgggaaagtt tcgtcgttta 4380
taaaagcgag ggaaagggaa gggaagttgg gggtagggga aaagttagag ttgagaggtt 4440
ggggcgcgac gagtttggat atcgggcggg gatttaagtt tttttcgttt agttaataat 4500
tgtgtttttt ttaggaaggc gtgaggaaat gttttaatta atttttgtat tttttttttg 4560
gaatttgggt tgtatttttt tatttattgt aaattttata atttatttag gggttttttt 4620

agtgtttgtt tttagcggtt tcggtgttta tttattagtg ttgttttttt tttttcgtaa 4680
gattgcgttt tagttttagt tttttttttc gcgggtgttt tttaaatcgt tttattattt 4740
tcgggtttag ggaggcggaa tcgtgtttgt ttttcggttt ttttaagagg cgtcggtttt 4800
atttttttta gagtcgcggt ttgacgcgag atgatagtaa cgagttcggt atgtttatgt 4860
aaataagcgt ttttttgtgg gttaatgggg agcggaggtg tcggaattac ggattaatgg 4920
ggcgggggcg ttggggttta ttatataagg agcggtttcg ttataaaagg aaatattgta 4980
ttttttttata tgggggaag ggtcgggga tttttcgtc gttagcgcgt ggtttcggtt 5040
ttttttattc gtcgtttcgg tcgcggttag tagtttttgt ttttcggggg acgttgacgg 5100
tcgttcggcg cgtcgtttta gtagacggat agggggcgtt gcgcgcggtt tggggtaatt 5160
cgggttatag gggtaggaaa gtgagggttt aggtcggttc gggcgtgtag gggtttcggg 5220
ttcgtagcgg cggtcgcggt agcgatagcg gtattagtag tagcgggagt gtcgggttga 5280
gtcgggaagt cgatggcggc ggttgcggcg gtttcgattt ttcgttgatt gttcgttcgt 5340
ttttttgtat cgagcgttat gttatcgatt taagttgggg tcgcggcggt tttgggtcgg 5400
ggttcggttt tggggggtag tttgaatcgg atttcgacgg ggcggtcggg cggcggcggc 5460
gggatacgcg gggttaacgg gggtcgggtt ttcgggaatg gcgcggggtt cgggttcggt 5520
cgtttggagc gggaggttgc ggtagcggcg gtaattattt cggcgtttat cgcgggggtt 5580
ttttatagcg gtagcgaggg cgattcggag tcgggcgagg aggaggagtt gggcgtcgag 5640
cggcgcggtt tgaagcggag tttgagcgag atggagatcg gtatggtggt cggtgggttc 5700
gaggcgtcgg tagcggttat cgggggttac gggtcggtga gcggcgcggt gagcggggtt 5760
aagtcgggta agaagattcg gggtcgcgtg aagattaaga tggagtttat cgataataag 5820
ttgcggcgtt atacgatttt tagtaagagg aagacgggta ttatgaagaa ggtattaagt 5880
cggggggttg gtcggtttcg gggttcggtt ggggtgggga tgtgtaaagg gagttcggga 5940
ggatcgtaga gtcgaggcgg aggtgagagg ttgcgagttc gtaggaggtg tgtgggaggg 6000
gatggttttt gttcggggag ggtcgaaggg gaggggtcgt cggggaaatg tggggagagg 6060
ggagatttcg cgaacgttcg taatcgtggg gaaaggcgta gaggtgggag tgatcggcgt 6120
tagagaggaa gagggttttt gttgagtgaa ggggtgagga gcggtgatgg gaggttacga 6180
ggttgtcggg gaggtggata atgagaattc gggattagta ggtttagtgt atttcggtgt 6240
tcggacgagg gcgtcggaaa agtagggagt aaacgagaag gtatggaggt gaggaggttg 6300
gagttgtatg ataataatga gcgaatatgt gtgggaggaa gtgggtatta cgagagtagt 6360
gttggttgga aggataggta gggttaggga ttaggtaagg gagcggggta ggagaattgg 6420
tgttgttttg agtagtagga atttagtttt gcgtcggtcg tgagtttttt atggtattta 6480
ttgggaatta tatgtttttg gtaggattcg ttgtagaatt ttttgtcgtt aggataagtg 6540
gggtttttag ttttagggag aatttatgtt tggatggggt ttgggggaag ttagggaggt 6600
ttgtagaggt atttaggaat tttttttttg ttagttagtg aaaagtgttg aggaaaggag 6660
ttattttggg gtgtagacga taagggaaga gatgaaagta gaggaattgg gagataagga 6720
gttggatttt tgggaatatt ggttggtgtt tatttttgtt ttatgtagaa attagagttg 6780
tttttaaggt ggtttgtaga attttgttgg gagagtatgg tatttatggg gttatttttg 6840
aaggtttttt ttttgggttt atggtaggat gttt 6874

<210> 248
<211> 6874
<212> DNA
<213> Artificial Sequence

<220>
<223> chemically treated genomic DNA (Homo sapiens)

<400> 248

aaatattttg ttatgagttt aggaagggga tttttaagga tggttttata gatgttatat 60
ttttttaata aagttttgta aattattttg gaagtagttt tggtttttat atgggataga 120
aataaatatt agttagtgtt tttaaggatt tagttttttg ttttttagtt tttttgtttt 180
tatttttttt tttatcgttt atattttaaa atgatttttt tttttaatat tttttattgg 240
ttggtagaag aggaattttt aggtattttt gtaagttttt ttgatttttt ttaaatttta 300
tttaagtata gatttttttt agggttggaa attttatttg ttttaacggt aggagatttt 360
gtagcgggtt ttgttaggag tatgtggttt ttagtggatg ttatggaaga tttacggtcg 420
gcgtaggatt aggtttttgt tgtttagggt agtattagtt tttttgtttc gtttttttat 480
ttggttttta attttgtttg tttttttaat tagtattatt ttcgtggtgt ttattttttt 540
ttatatatgt tcgtttattg ttgttatgta gttttagttt ttttattttt atattttttc 600
gtttgttttt tgttttttcg gcgttttcgt tcgaatatcg gagtgtattg gatttattga 660
tttcgggttt ttattattta ttttttcggt agtttcgtag ttttttatta tcgtttttta 720
tttttttatt tagtaagggt tttttttttt tttggcgtcg gttattttta tttttgcgtt 780
tttttttacg gttgcgagcg ttcgcgggat tttttttttt tttatatttt ttcggcggtt 840
tttttttttc ggtttttttc gggtaggagt tatttttttt tatatatttt ttgcggattc 900
gtagtttttt attttcgttt cggttttgcg gttttttcgg gttttttttg tatattttta 960
ttttaatcgg gtttcggggt cggttagttt ttcggtttgg tatttttttt atgatgttcg 1020

tttttttttt gttgaaggtc gtgtagcgtc gtagtttgtt gtcgatgaat tttattttga 1080
tttttacgcg gtttcgggtt tttttattcg gtttggtttc gtttatcgcg tcgtttatcg 1140
gttcgtagtt ttcggtggtc gttgtcgacg tttcgggttt atcgattatt atatcgattt 1200
ttatttcgtt taggtttcgt tttaggtcgc gtcgttcggc gtttagtttt tttttttcgt 1260
tcgatttcga gtcgttttcg ttgtcgttgt agagggtttt cgcggtgggc gtcggggtgg 1320
ttgtcgtcgt tgtcgtagtt tttcgtttta ggcggtcggg ttcgagtttc gcgttatttt 1380
cggggattcg gttttcgtta gtttcgcgtg tttcgtcgtc gtcgttcggt cgtttcgtcg 1440
gggttcggtt taggttgttt tttagggtcg agtttcggtt tagagtcgtc gcggttttag 1500
tttgggtcgg taatatggcg ttcgatgtag gagggcggac gggtagttag cgaggaatcg 1560
gagtcgtcgt agtcgtcgtt atcggttttt cggtttaatt cggtattttc gttgttgtta 1620
gtgtcgttat cgttgtcgcg gtcgtcgttg cgaattcggg gtttttgtac gttcgggtcg 1680
atttgggttt ttattttttt gtttttgtgg ttcgggttgt tttaggtcgc gcgtagcgtt 1740
ttttgttcgt ttgttagggc ggcgcgtcgg gcggtcgtta gcgtttttcg gggggtaggg 1800
gttgttggtc gcggtcgaga cggcgggtgg agggggtcgg gattacgcgt tggcggcgga 1860
gggatttttc gatttttttt tttatataaa gagatataat gttttttttt atggcgaggt 1920
cgttttttat atggtgagtt ttagcgtttt cgttttattg gttcgtggtt tcggtatttt 1980
cgttttttat tggtttataa gagggcgttt atttgtatag atatatcgaa ttcgttgttg 2040
ttatttcgcg ttagatcgcg attttgagag gggtggagtc gacgtttttt aaaggaatcg 2100
gagagtaggt acgatttcgt ttttttgaat tcgagaatga tagaacggtt tgggaggtat 2160
tcgcggagaa ggaggttggg attggagcgt agttttgcgg aagagaggga gtagtattag 2220
taaatgggta tcgggatcgt tggaggtaaa tattggggaa atttttgggt ggattgtggg 2280
gtttgtagta aataaaaaaa tatagtttaa attttaaggg aggagtgtag ggattgattg 2340
gagtattttt ttacgttttt ttaagggagg tatagttatt ggttgagcgg agagagtttg 2400
ggttttcgtt cggtgtttag attcgtcgcg ttttagtttt ttagttttaa tttttttttt 2460
atttttaatt tttttttttt ttttttcgtt tttgtaggcg acgaaatttt tttatcgggt 2520
tggttcgagg atttttattt attatttttt gtattggggg gtttttggta gatatatatt 2580
tggaaaaggt tggtggaggg gagggcgaga gagagagatc ggttttttag ttttgaggcg 2640
ggtagggagg agagacggag attagggagg agagatggag attagtgaag atttttagtt 2700
gtaggttgga tttggggagg aggacgtttc gaggataaag tttattttat agttagtgta 2760
aagatttggg aaagtgtggt tcgatagaaa gtttaagttt aagtaagtac gttcgcgtat 2820
atatacgtat atatatatat ttttttggaa agttcgttgg agaaaaaaaa aaaagtttta 2880
tatagggtat cgttttgtcg tttaatttgg agtgtagtgg cgggatttga gtttattgta 2940
atttttgttt ttcgggttta agcgattttt ttattttcgt tttttacgta gttggaatta 3000
taggtgcgcg ttattatttt ggtttaattt ttgtattttt tgtagagatg gggttttgtt 3060
atgttgttta gattggtttc gaattttggg ttttaagcga tttttttttt tcggtttttt 3120
aaagtgttgg gattataggt atgaattatc gtgtttagta gaaaaaaaaa aaaaaaaaaa 3180
aaaaaattta aaagattaga attggggtgt tgtaaagtga ggtttaagtt ttgggtttgg 3240
gttttttttt taaagtagtg aaaatagttt ggattttgtt gttatagaat tatttaaatt 3300
tggaatttgt tttttggtaa gttttgtgtt tggaaataag tgataagtga tttgattttt 3360
ttagacgtaa tatttattaa tgttgttatt tattttttga gttttttttt tttttttttt 3420
tgagatagag tttcgttttg tcgtttaggt tggagtgtat tggtacgatt tcgatttatt 3480
gtaatttttg tttttttagt ttaagcgatt tttttgttgt tttagttttt taaatagttg 3540
ggattatagg tgtttattat tatatttggt tagttatttt gtgtttttag tagagatggg 3600
gtttcgttat gttgtttagg ttggtttcga attgttgatt ttaagtgatt tttttatttc 3660
ggttttttaa ggtgttggga ttataggtat gagttattat gtcggtttta ttttatgaaa 3720
tttttaatgt agatttaatg atataaagat taaagggtag agtaattggt tagtggttag 3780
gtaataggag ttatttggag taagttattt tattttaggg ataaagtatt ttggtatgtt 3840
taaggggatg ggaggtagtg attatttttg ttcgaaaaat tatggagatt aaataaagta 3900
gttgattgta atttagttat tattggtagt ggtagtgata ttaatttagt atattataat 3960
taatatttaa aagaataaat taatgggtat tataaaaagt aagggtaaat attgttgtat 4020
aaaatgtttg tttgtattat atataggttg aattttataa aattgttatt ttataggtta 4080
agaaggtggg atattgttaa ttttttataa tttagtgtat gtgtgtaatg gatgataata 4140
ttaattgtat tttttattgt gagttataat ttaaaagttt gaaaaatatt gaaataaaat 4200
atgtgtaagt gattagtaaa atatagtgtg tattttgtgt gtatagtgaa taaaatatag 4260
tgtgtatttt ttgttatata gagataaggt ttttttatgt tgtttaggtt ggttttaaat 4320
ttttgggttt aagcgatttt tttatttcgg ttttttaaag tgttgggatt aaaggtttga 4380
gttattgtat ttagttttta atagttttaa agaggttttt ttttttttta ttaatttatt 4440
ttttataata ggtatttaaa tatatggaac gtgttagttt aagaggaaga ataggttaaa 4500
tagataaata ggttttagaa gggttttagg gattataaat gatgtttggg gttatgtttt 4560
gtttgagtat tataatatta tagttaaagt tttgggattt tatttttatt tgttttttaa 4620
agattttttt atttgcgttt tttagtttag atatatgtag ggtgatattt ttttagtagg 4680
gataattgtt ttttgttata tttatagggg agggtgtttg ggaagaacgt agtttttagt 4740
ttttttttaa ataggttgtt ttttttttaa ttttttttaa tttttgggtt attgtatatt 4800
agtgttagtg ttatagtttt tttgtttgtt tgggatggtt tatttttatg ttttttgtta 4860
ttatagggag ttaggaggag atttttattt atattattgg attattgtag gttttagagt 4920
taaaagtatt gttagggata aagttaaaat taaaaattgt tttatagagg tgttttattt 4980

ggattttata atatttaaaa attataagag tttatataaa aatttgaatg tttggtcggg 5040
cgtggtggtt tatgtttgta attttagtat tttgggaagt taaggtggat ggattatttg 5100
aggttaggag tttaagatta gtttggttaa tatggtgaaa cgttattttt attaaaaaaa 5160
taaaaaagta gtcgggtata gtggtatata tttgtaattt tagttatacg agaggttgag 5220
gtaggagaat tatttgaatt tgggaggtag aggttgtagt gagtttaaat ggggttattg 5280
tatttcggtt tgagttacgg agtgagattt ttttttaaaa aaaaaaaaaa atttgaatat 5340
ttgatttttt ttaaaaaaat tagaattttt ggtaatattg ggtttaggta tttataaaag 5400
tattataatt tggagttgat ggtggttata gaagaggttt tttattttat tatagatttt 5460
acgtagttta ttgagtttag gtaagttttt gtttggttgt tttgaaatgt gaagttgaaa 5520
tgttttttcg aaaaggtttg attagagtag tattttgtaa tagatgggag tttatgttta 5580
ttttagatta tgaagggttt ttgtaagtgt atgggaattt tagttagatt ttttatatta 5640
tatagatttt attttgattt tttttttttt tttttgattt ttattattta attaataata 5700
aataaaaatt aaggattgtt tttaaaaatt attgttatgg ttgggtgtag tgatttatat 5760
ttaaaatttt agtatttttt gaggttaagg tgggaggata atttgagttc gggagttttg 5820
atattagttt ggataatata atgagatttt attttttaaa aaaaaaaaat ataggtaggt 5880
aaggtgggtt atgtttgtaa ttttagtatt ttgggaggtt taggagggtg gattatttga 5940
ggttaggagt ttaagattag tttggttaat atggtaaaaa ttttattttt tttaaaaata 6000
taaaaattag tcgggtttgg tggtgtatgt ttataatttt agttatttag gaggttgagg 6060
taggagaatt atttgaattt ggaggggaga gattgtagtg agttaagatc gtgttatttt 6120
attttagttt gggtgaaaga gtgaaatttt attttaaaaa aaaaaaaaaa tgttggggta 6180
tagtggtacg tatttgtagt cgtagtttta gttattaggg aggttgaggt gggaagattg 6240
tttgagttta ggttgtagtg agttatgatg gtattattgt atttaattta ggtaagagga 6300
aaattttgtt ttaaaaaaga aaaaaaaatt attgttaaaa tattttaaat ttgtttttat 6360
ttggaattaa ttttatttta ggatttataa atttgtatat gaggtaaaga gggtattaat 6420
tgtgatggta gtattagtag taatggaaaa gaatttatga gttattgtgt ttaggaaaag 6480
tttggaaagt tatattttaa atattaatag tgtttattta tggagagtga ggtttgggat 6540
gagaggggtg tgtttttttg ttttatataa gtttgtatta tgtgattttt ttgataatta 6600
atatgatttt tatatttttt ttttaaaatt aagtaaaaat aggtcgggtg cggtggttta 6660
tgtttgtaat tttagtattt tgggaggttg aggcggatgg atcgtttaag gttaggagtt 6720
tgagattata ttggttaata tggtgaaatt ttgtttttat tgaaaatata aaaaaaatta 6780
gttgggtgtg gtggcgggta tttataattt tagtttttgg ggagtttgag gtaggagaat 6840
cgtttgaatt taggaggtgg aggttgtagt gagt 6874

<210> 249
<211> 5795
<212> DNA
<213> Artificial Sequence

<220>
<223> chemically treated genomic DNA (Homo sapiens)

<400> 249

ttttatgttt agatatttat ttaagagaaa tgaaaatttt attatataga aatattggta 60
tataaatgtt taaaaaggtt ttgttatagt tataattgaa aatagtttaa atgtttttta 120
gtaggtaaat gggtaagtat tttttggtat atttatataa tggaatattg tttagtaata 180
aaaagaaata aattattgat atatgttatt atattgaata attttataat aattatgtta 240
agtataagaa gttagataat aaaaagtata gtgtattatt ataattatat aaaagtatgt 300
aaaatataaa ttaatattta gttaagaatt tacgtttgta gttatttgta ggatgagagg 360
ttgggaggga gagatgtatt gaggggtaag aggaaatgtt aggtgtgatg ggtgtgttta 420
ttttttggat ttgtgatgat aattttatag gagtacgtat atattaaatt aaaattgtgt 480
aatttaaata tatttatttt gtgttgttat ttatattata ataaagtttt tattaaaaat 540
tttttaaaaa gttttattaa aaatttatta aaaagtattt attatatata tatatatata 600
aatttattta tatatgtata tatgtataat atatattgat atatttatat atatatattt 660
acgatttata tatatattat atatatttaa gtatatatgt ttatatatat ttttaatttt 720
tattataatt ttgttaaggg ttttttaaat tattttgtta atgaggaaaa tatttttttt 780
agaatttgtt tattagagag gtataattaa tattaattgg tagattatta atatttgtta 840
ttttgatttt ttatttttat aagtttagag ttttttttta gaagatattg atgtagtagc 900
gtgtttgagc gttttggcga tataagggtt tggtttttga gtttttagga gtgtagtttg 960
gtatagtttt ttatgttggt agattttgga gatttacgaa ggtttttttt tgaagggttt 1020
ttgaggttga gagttcggtt tgttttttgg ttgttttgtt ttgtggtttt tttttttttt 1080
ttttagattt ggtaggtgtt acgttttatt tatttttagt taggatttag ttgtttgatt 1140
tttagtttg tgtttttttgg tgtttttttgg aattttgggg aaaatttgt cggtttatgt 1200
tatgtttttt ttttgtaata tttttttttt tatagatagt ttaaggagtt gttttatgga 1260
gatattatgt aaataaattt ttatgtttta tttttaaatg gtttattttt ttttattttt 1320
attagaaata tgagggatta gatatgaagt tatgagaaga ttgatttgat ttagttgttt 1380

taggaaggtg ttattttttt tatagttgtg attttggtag gattttttat ttaaggggag 1440
gaggtggaaa gtggatggaa gtaattagag aagtaattgt agtatgggtt cggaggtttg 1500
ttgttttgag taaagtattg gatgtgttga gtttagggat aatataggtt tatggttttt 1560
ggaattttta tttgttttag gaattgtttt tggttttttt agtagttttc gggtatgaag 1620
ttttttgtat tttttattgt aaatgtgtaa atgtttcgta gtttaggggt agttattttt 1680
ttttagtttt agttattttt gttgttatag tgatttggag gtatcgttgt tatggaggta 1740
gttgtagttt tttgtttttt ttggtaaggt gatattggcg gttttatttt taattgaatt 1800
ttttgtttag gatgtttatt tttatatata ttgtgggtga ttgtgttata tatatatgaa 1860
aatatatata tatataatat ttttatacgg agtatatata taattagaaa agaaatttat 1920
ttgttttttt tgtataaaga gagggattat tagtgtgggg taagagatac gcgaggttgt 1980
tttttatttt tttttgagtt tgtgatatat ttattgttgt ttgtttttaa agataaatgg 2040
ttttgttttt ttagaaggga tagggtttac gaaaattgtt tttttgggag tcgagtgggg 2100
tgtgtgtgtg tgttttttta taaaatgttt taagtatgtt ttcggtggga tagttgtatt 2160
ttgaggttta gttataaggt tttgttttgt ttttttttga atggttgggt ttgttaagga 2220
gagatagatt ttgtgagcga aatatttggc ggtgttttag tttttttttt tttttaagga 2280
agcgtattgt tattaattgg gaatttttta tagtcgggtt ggaggaagtt ttggttgtaa 2340
attgttatgt attgtagttt tcgttgaaaa ggcggaggga gtgggtttgg ttttgggaat 2400
cgaggaataa agattagaaa attagttata gaaaggggag gaaaaataaa cgttagaaag 2460
tgaagatagg tgatattata taagtgttgg ttaaagtcgg tgatttttaa tttttatttt 2520
tttcgacgtg ggtggtttaa tttttgggtc gtatttttta atgttttaga tatttttttt 2580
ggagagtaga aattttatta cgcgtgttag aaacggaata tttttttttg ttgaagttgt 2640
atttttattt ggtcgtgttt tttttgttcg gaatagtttt agagcgattt gttaaatttt 2700
ttagtttttt ttggcgtttt tcgattgtgg gaaaagcggt cgcgacgtcg ttcgagcgga 2760
ggggagggat ttagttttcg ggattttttt gttttgaagt cgtaggagag gtttcgtttt 2820
cgtgtttagg gtttcgaggt ttttaattgt ttgggattta ttttcgtttt ttttttattt 2880
tttttttatt tttttttttt attttatttt cgggaggttt tgggttaaag cgatgatttt 2940
ttagatattt taatattcgg agtaaggaga gtaatacgta ttacgttttt ttttaaagtt 3000
taggattcga tgagttagtg aaggcgtgtt aggagggttc ggcgttagga gtaaatgagg 3060
tttttttggt gttttttttt agaaggaaat ttttttattt cgggttagtt ttttgggaat 3120
atttatgtat tttagatatt aaaagatatt gagaaatgcg gatagggatt agacgtttcg 3180
gttttttgat tcgtcggtgt aagttggaga agggagagaa ggagttttgt tttttacggg 3240
cggtaggtat tttttttcgg gattggtttt tggtagtttt tcgtatatcg cgaggcgggt 3300
cgatttttcg agtttcgggc ggggattttt ttttcggttt ttttagtaat tttcgatttc 3360
ggagcgagtt cggttgggcg aggggcgagg ggtagggggt agggggtagg ggagatttag 3420
cgcggggcgt agatattatg ttcgcgggaa agtttttttg ttagggcgta agattttttt 3480
gaattcgttg ttttattcga tgcgtaggtt ttttttagag gggttggggt tggggtgttc 3540
gtttaggaga tcgggaaata gaggttgtta ttcgaggtag gttttcgttt agcgaatggg 3600
cgaggtgtgt agaagcgtaa agttaggttt tgaaaggggg agtttgtgtt tttttttttt 3660
tttcgtgggt tttcgtttta ggaggaatgt tattgtttaa agagatttta ttgaattatt 3720
tcgtgtttag tgttattttt ttttcgtttt tttcgcgtaa gtttttatcg aaaggtaata 3780
aaataatcgt tgtcgatatc gtttggcgtt gggtcgggcg gggaaagcgt ttcgagtttt 3840
attagttcgg attattcggt tagtttcgat ttttttttat ttttcgtgaa agtaatgata 3900
tagtagaaat tacgtatacg tttggtatat tcgatgcgcg cgttgatttc ggtaataagt 3960
tttgttttta taagagagcg aggaggatat tttttagaag gggttgttgt gtttgtgtat 4020
attttcgttt attttttttt gtgcgcgcgt attttttttt tttagatggt gagagtcgcg 4080
gggatattcg acgtcggggt aggttgattt acgattttgg gtgtgcgtaa cgtcgtttgg 4140
ggtttcgtgg gcgagggacg tgtggggata ggtgtatcgg aaattgttag attggagagt 4200
tgaggtatcg gaggcgcgag aatagtatta ttattgcggc gagacgagcg cggcgtattt 4260
taaagttcgg ttaaatgcgt tcgtttttgg gaggggaggg aggcgcgttt ggagcgggga 4320
taggtgaggt cgcgcgaacg ttttagtttg gcgcggttgc gggattcggg cgcgttggga 4380
aagcgagtag gatttcgcgc gggaattagg gagtatcgta ggttgtcggg tagtttttcg 4440
cggaagtgcg ggttggggcg ggttggggcg ggttggggta ggtcggggcg tttagtttat 4500
tttttttcgg ggtttgtttc gtcgttttga tggaggtgta aatatttgga gaaggcgggg 4560
ggtgtagggg ttgggtcggg gttcgtaggg ttggggtttc ggatttatat gaaataggcg 4620
tgcgggagtt tattgttggt tttcggtttt ttagattcgt tcggttgggt ttgatatggt 4680
cgttttcggt taatgggagg ttgttcggta ttttaaagcg tgcgcggttt aattcgtcga 4740
gtattttagc gttgtcgtcg cggttttatt tatggggagg gtattgaatt tcggttttta 4800
gagatttata ttagtagagt tttggggagt ttaagtggaa taattttttt ttattttttt 4860
gttttcgttt ttttttttta agttttggtt cgcgtttttt tttcggtttt gtgtcgggat 4920
tcgggattcg ggagtcgtcg taggtttcgg tttaaggggt tttttttttc ggaagggcgg 4980
cggttaagag cggggaaggt ggattttagg tagcgagttt gggtttcggg gacggcgggg 5040
aggggagtcg gacggaggga tgagtttttt tggtatcgag agcgcgggaa agagtttgta 5100
gtatcgagtg gattatttgt tgagcgtcgt ggagaatgag ttgtaggcgg gtagcgagaa 5160
gggcgatttt atagagcgcg aattgcgcgt gggtttggag gagagcgagt tgtggttgcg 5220
ttttaaggag tttattaatg agatgatcgt gattaagaac ggtaggtggg tgcgcgttcg 5280
gagttcgcgc gcgtcgcgtt ttttagcgtt tgggtagttt gggggatttg gtaagttttc 5340

ggaggtcgaa tttttttttt tttggaggag ggttttgtgt ttgttttcgt tttggttaat 5400
tttttcgagt gtatttttat aagttttttt tttggggttt ttttcgcgga agtttgggga 5460
ggaggaacgc gtatttcggt gtttcgattt cggaggtatt tcgggattcg cgaggcgcgt 5520
tttggttttc gtttgttttc gcgggatgtg gttttttgta ttgaaatttt tcgttaggtt 5580
tttttttttt ttttttttcg aggtcgtgga gatggtgagg aggttatttg gatttggtag 5640
ggttcgggga agggggagtt tgggagttat tttttgacgt tttttagggg gtttttttgt 5700
gtttcgaaat aattgaggtt tttgtgttta gtttttgttt tcggtttatt tgggtagagt 5760
gggggttttt ttgcgttcgt ttcgtgtttt ttagg 5795

<210> 250
<211> 5795
<212> DNA
<213> Artificial Sequence

<220>
<223> chemically treated genomic DNA (Homo sapiens)

<400> 250
   tttggaaaat acggggcggg cgtaggagga tttttatttt gtttaggtag gtcggaggta 60
   gaagttgggt atagaggttt tagttatttc ggggtataga ggagtttttt ggggaacgtt 120
   agagggtgat ttttaagttt tttttttttc gagttttgtt aggtttaggt ggttttttta 180
   ttatttttac ggtttcggga gggaggggag gagaggattt ggcgaagggt tttagtgtag 240
   gaggttatat ttcgcgggga taggcgggga ttagggcgcg tttcgcgggt ttcgggatgt 300
   tttcgaggtc gggatatcga agtgcgcgtt tttttttttt aagttttcgc ggagaaagtt 360
   ttagaagagg ggtttgtaga aatgtattcg agggagttaa ttagagcggg aataaatata 420
   aagttttttt ttaggagaaa aagggttcga ttttcgggaa tttgttaggt tttttaggtt 480
   gtttaggcgt tggagagcgc ggcgcgcgcg ggtttcggac gcgtatttat ttgtcgtttt 540
   tggttacgat tattttattg gtgagttttt tgaagcgtag ttatagttcg ttttttttta 600
   ggtttacgcg tagttcgcgt tttgtggggt cgtttttttc gttgttcgtt tgtagtttat 660
   tttttacggc gtttagtagg tggtttattc ggtattgtag gttttttttc gcgttttcgg 720
   tgttagggga gtttattttt tcgttcggtt ttttttttcg tcgttttcga agtttagatt 780
   cgttatttga gatttatttt tttcgttttt ggtcgtcgtt ttttcgagaa aaggggtttt 840
   ttggatcgag atttgcgacg gttttcgggt ttcgggtttc ggtatagatt cgggaggagg 900
   gcgcggatta agatttgggg ggaggggacg ggggtagagg ggtggggaga agttatttta 960
   tttgaatttt ttaaggtttt attagtgtag gtttttgggg atcgaaattt agtgtttttt 1020
   ttataaatag agtcgcggcg gtagcgttgg ggtgttcggc ggattgggtc gcgtacgttt 1080
   tgaagtgtcg ggtagttttt tattggtcga gagcggttat attagattta gtcgggcggg 1140
   tttgggaggt cgggggttaa taatgggttt tcgtacgttt gttttatgta aattcggggt 1200
   tttagttttg cgagtttcgg tttagttttt atattttcgt ttttttttaa atgtttgtat 1260
   ttttattaaa gcggcggggt aagtttcgga ggaaggtaag ttggacgttt cggtttgttt 1320
   tagttcgttt tagttcgttt tagttcgtat tttcgcgagg ggttgttcgg tagtttgcga 1380
   tgttttttga ttttcgcgcg gggttttgtt cgttttttta gcgcgttcgg atttcgtagt 1440
   cgcgttaggt tggaacgttc gcgcggtttt atttgttttc gttttaggcg cgtttttttt 1500
   tttttttagg gacgagcgta tttggtcggg ttttgggatg cgtcgcgttc gtttcgtcgt 1560
   agtagtagtg ttgttttcgc gttttcgatg ttttaatttt ttagtttggt agttttcggt 1620
   gtatttgttt ttatacgttt ttcgtttacg gagttttagg cggcgttacg tatatttagg 1680
   atcgtggatt agtttgtttc ggcgtcgggt gttttcgcgg tttttattat ttggaaaagg 1740
   aaggtgcgcg cgtatagagg gaaatggacg gaaatatgta taagtataat aatttttttt 1800
   gagaagtgtt tttttcgttt ttttataaaa ataggatttg ttgtcgaggt tagcgcgcgt 1860
   atcgagtgtg ttaggcgtgt gcgtggtttt tgttgtgtta ttgtttttac ggaaggtggg 1920
   agaaggtcgg ggttggtcgg gtggttcgga ttagtgggat tcggggcgtt tttttcgttc 1980
   ggtttagcgt taagcggtgt cggtagcggt tgttttatta tttttcggtg agaatttacg 2040
   cgaggagagc gaaggagagg tgatattgag tacgaaatag tttagtgggg tttttttaaa 2100
   tagtaatatt ttttttaaaa cgggagttta cgaagggggg aaggaggtat aaattttttt 2160
   ttttaaggtt tggttttgcg tttttgtata tttcgtttat tcgttggacg agggtttgtt 2220
   tcgggtagta gtttttgttt ttcggttttt tgagcgggta ttttagtttt aattttttta 2280
   gagaaagttt gcgtatcggg tggggtagcg agtttagagg agttttgcgt tttagtaagg 2340
   gggttttttc gcggatatgg tatttgcgtt tcgcgttaag ttttttttgt tttttgtttt 2400
   ttgtttttcg tttttcgttt agtcgggttc gtttcggggt cgggaattgt tggggaagtc 2460
   gaaggaggga ttttcgttcg ggattcgagg gatcgattcg tttcgcggta tgcggagggt 2520
   tgttaggagt tagtttcggg gaagggtgtt tgtcgttcgt gggggatagg gttttttttt 2580
   tttttttttt aatttatatc gacgagttag gaagtcggag cgtttagttt ttgttcgtat 2640
   tttttagtgt tttttgatgt ttgggatgta tggatatttt tagggggttg attcgaggtg 2700
   ggaagttttt tttttagaaa gaggtattaa aaggatttta tttgtttttg acgtcggatt 2760
   tttttgatac gtttttattg gtttatcggg ttttgggttt tgggggagag cgtggtgcgt 2820
   gttatttttt ttatttcggg tattaaaatg tttaagaggt tatcgttttg gtttaaggtt 2880
   tttcgaggat aagataaaag ggaaaagtgg aggggaggtg aaggaggaac gagggtgggt 2940
   tttaggtaat tgagggtttc ggaattttag gtacgggagc gaaatttttt ttgcggtttt 3000
   agggtaaagg agtttcgaag gttggatttt tttttttcgt tcggacggcg tcgcggtcgt 3060
   tttttttata gtcgggaagc gttaaagaag attggagggt ttaatagatc gttttaaaat 3120
   tgtttcgaat aggaggggta cggttaaata agaatataat tttagtagga aagaatattt 3180
   cgtttttaat acgcgtaata aaatttttat tttttagaga gaatgtttga agtattgaag 3240
   agtacgattt aggaattgaa ttatttacgt cggaggaggt agaggttgga agttatcgat 3300
   tttggttagt atttgtgtag tgttatttgt ttttattttt taacgtttat tttttttttt 3360
   ttttttgtgg ttgatttttt gatttttgtt tttcggtttt taggattagg tttatttttt 3420
   tcgttttttt agcgaaggtt gtagtgtatg atagtttata gttaaaattt tttttagttc 3480
   ggttataaag aatttttagt taataataat gcgttttttt gggaggaaag aggggttgag 3540
   gtatcgttag gtgtttcgtt tatagggttt attttttttt ggtaagttta gttatttaga 3600
   gaaaaataag ataaagtttt atggttgggt tttaggatgt aattgtttta tcgaaaatat 3660
   gtttgaaata ttttataaga aaatatatat atatatttta ttcgattttt agagaggtag 3720
   ttttcgtggg ttttattttt tttgaaaaag taaagttatt tatttttaga agtaggtaat 3780
   aatgaatgtg ttataggttt aaggaggggt ggaggatagt ttcgcgtgtt ttttatttta 3840
   tattagtagt tttttttttt gtgtaagaaa aataaatgaa tttttttttt gattatatat 3900
   gtatttcgtg tagaaatatt atatatgtat gtgtttttat atatatgtaa tatagttatt 3960
   tatagtatgt gtgggggtga gtattttgaa taaagagttt agttggagat gggatcgtta 4020
   atgttatttt attaaggaga atagagagtt gtagttgttt ttatggtagc gatattttta 4080
   ggttattgtg gtagtaggag tgattggggt tggagagaaa tgattgtttt tgggttgcgg 4140
   gatatttgta tatttgtagt aggaagtgta gagggtttta tgttcgagag ttgttgaggg 4200
   agttaggggt aattttgggg gtaagtgggg attttaggag ttatggattt gtgttgtttt 4260
   tgagtttagt atatttagtg ttttgtttaa ggtagtaggt tttcgagttt atgttgtagt 4320
   tgttttttta attattttta tttatttttt attttttttt tttaggtagg aagttttgtt 4380
   agagttatag ttgtgagaaa agtggtattt ttttgaaata gttgagttaa gttagttttt 4440
   ttatgatttt atgtttaatt ttttatgttt ttaataggaa tgaaggaggg taaattattt 4500
   agaaataaaa tataaagatt tatttgtatg atgtttttat gaaatagttt tttggattgt 4560
   ttgtgggagg aagaatgttg taggaagagg atatggtatg aatcggtaag gtttttttta 4620
   aaattttaag ggatattagg gggtatagaa ttggaagtta ggtagttgag ttttggttag 4680
   gaatgagtgg gacgtgatat ttgttaagtt tgaagggaga gaaaggaggt tatagggtag 4740
   ggtagttaag gagtaggtcg gatttttagt tttaggagtt ttttaggagg gggttttcgt 4800
   gggtttttag ggtttgttag tatgaggggt tgtgttaggt tgtattttta ggggtttagg 4860
   aattaagttt ttgtgtcgtt agaacgttta gatacgttgt tatattaata ttttttgaag 4920
   gggagtttta gatttataga agtgggagat taggatggta gatattaatg atttgttaat 4980
   taatattaat tatgtttttt tagtgagtaa attttgagaa aggtgttttt tttattggta 5040
   aaatggttta ggaagttttt gataaggttg taataggagt taaaaatatg tgtgaatata 5100
   tgtatttgaa tatgtatagt atgtatgtga atcgtgggta tatatatata aatatgttaa 5160
   tgtatattgt gtatgtatat atatataaat gagtttatgt atatatgtat ataataaata 5220
   ttttttaata aatttttaat aaaatttttt aagaaatttt taataaaagt tttattgtgg 5280
   tgtaagtaat agtataaagt gaatatattt aaattgtata attttagttt gatgtatgcg 5340
   tatttttgtg aagttgttat tataaattta gaaaatgaat atatttatta tatttagtat 5400
   ttttttttat tttttaatgt attttttttt tttaattttt tattttgtaa gtgattgtag 5460
   acgtgggttt ttaattgaat attagtttgt attttatata tttttatata attataataa 5520
   tatattgtat tttttattgt ttggtttttt atatttagta taattattgt gagattattt 5580
   aatatggtga tatgtattaa tagtttgttt tttttattta ttgagtagta ttttattgtg 5640
   tggatatatt aaaagatatt tatttattta tttgttgaga aatatttggg ttatttttag 5700
   ttgtggttat gataaagttt ttttgaatat ttgtgtgtta atatttttgt gtaataaaat 5760
   ttttattttt tttgagtaaa tatttaggta tggaa 5795

<210> 251
<211> 7856
<212> DNA
<213> Artificial Sequence

<220>
<223> chemically treated genomic DNA (Homo sapiens)

<400> 251

ttatatgtgt tggttttatt tatttatggg gtttgtagta ttttttttat tttttagata 60
gtattattga ggtttgtaga ggttagggcg tgtttttaga tatagagttt tgaagggtga 120
agttcgtttt tggatttagt ttttgagatt gggcgatatt attacgttgg tttttttttg 180
tgtagaaggg tttagagtat aggggttttg tcgaagtagt tttgtttttt tttgaaatga 240
aatattgtta gtttttttat tagttttttt ttttgggaag gcgggagatt agatagagtt 300

ggtagttttt agttttgtat cgttatttcg attagggaaa gttgtttgtg taattaattt 360
ttttaggttt cggttttttt ttgtttacgg gtttggggat tttttatagg ttttagtaaa 420
ggggttttcg tgttagagtt ttgtttatta gggatatttt ttaaaatttg ttttagtttg 480
ggtagtttat ttgtttttgg ttttaggtta gtgagggcgt tttaggttag tttttataag 540
tataggggga gatatgagta gggtttgttt agagtagtgg gtagggaggt ttaaatgtta 600
gtttgggtat ttgggtatag ttttggttta ggagggataa tttagtagtt ttgggggttt 660
ggttagggag tagagtggat cgagaggtat agggtgtggg gttagttttt ttttttattg 720
agataggatt tttatatagt tttttttagt agttatattg ggttgttata gtgatagtat 780
ggattatttg aattaattga gtatttaatg aaaattgtaa gtgtgatata tttgattatt 840
ttaaatgttt taagtatttt taaagagaga tataatatat ttaatgttta cgggggtatg 900
gtttcgttag gatttattaa taaaggtgtt gaggttattg gtttagttgt tattttttat 960
tgtttttggt tttgttttta gagttttttt ttttagagtt ttttagggtt tattcgttat 1020
taagtaagat ttagggttgg ggtttgagta tggaaagtgg ttttttaagg agttttattt 1080
agaaggtttt ttgtagatcg attttgtttt gagagttttt tttagtatta atgtttaagg 1140
aagtaggttg gttagtcgga tgttattatt cgagtgtatt ttttttagaa atgatttagt 1200
aggagaggat tgaaatattg tatttttttt tttttatata gtagtaagtt tttatagtgt 1260
ttcgtttaag ttaagttaat ttaaggtata tgtgagtatc gttattattg tagaagggtt 1320
atagtggtta ttgtgagtga taagtttgta tatgtgtgcg tttgtatgta tatttgttat 1380
atgtgttgtg tgtgtatatt tttttatatt ggtgtttgtg ttggtgtttg ttatgtgtat 1440
gtgtgtgttt atgtttacgt gtgtttatgt atagaggtgt atgtatgttc gtgtttacgt 1500
ttgtgtatat gtagagtgtt tatgtttgcg tgtttgtagg tatatagtgt gtatgtgtgc 1560
gtttgggttt agttcgtgtg tttgtatgta gtgtgtttgt gtgtgtttat gtttacgttt 1620
gtgtgtttga tatgtgtatg tggatatatg tgtttatggt tataatattg gtatgtttat 1680
atgtgtaggt atatgtgttt atatttttat gtatgtgtgt ttgagtatga atatggtgtg 1740
aatatatata tatatttatg tttatgttta tgagtatgtg tgtacgtggt ggtgtgggtg 1800
tttgaatatg ggtaaatggg agttattgtt tagttagggt tttttgttgt ttagaggaat 1860
tggaggggtt tgtttgtacg tgtttatttt ttggttattt atttttagtt aatatttttt 1920
tagggttttt tgggataagt tttttttttt ggtaagtgtg gttttttttg ttggggttat 1980
agaagggaag ggggttagta gggattggtt ttttagttgg ttcgttaggt tgagatttag 2040
ttattttatt agtttgtata taggtttttt agggttttag agttttattt ttgtttggtt 2100
gtatttgggg tggaggggtt agtgtggtta ggagatggat agatagatgg atagttagtt 2160
ttttatgtgg ttgggttttt ttattaagtg ggtagttaaa ttaatagttt tagaaattgg 2220
agaggggttt agggttgggt tgtttattag tttgtttgtt tgtttgtttg ttttggagtt 2280
gtgggaattt ttagtttgta gggagttttg gtgtttggcg aggtcggggg gtagagggtg 2340
ttattattta ttcggttagt tatttttttt attatttttt tatatttgtt tttttgggaa 2400
tttggtttag aatatcgtat ttatagttgc gggggatagt taggtcgggt gataggaggt 2460
tggggattag ttgtatgcgg ttggttgtgt agattttggt cggttttttt tcggttggga 2520
ggaggttggt gttatacggt tgtgtgtttt tttgtttagt ttttttttta tttattattt 2580
tattttatgt ttgttttttt tagggtttcg ttgttttttt tgttaatttt gtagtttttt 2640
gttttttagg gtttaggtat taggattttt aggcggttta gattttgttt tgggattagg 2700
gagtagatgg tgttggggtt taggaagagt tttaggggat gttttgggtc gggtgagggg 2760
gttatatttt ttgttatttt gtttagtgtt tttttagtta taaggtgtgt atattaggtt 2820
gtgtttaatt tttaggtttt tgttagttat gtggttagtt tggttttgat tagatttata 2880
gggatatttt ttttaagttt tgggtttttt tttgggaacg tggttgtggg tttggagttt 2940
ttgggtaggg tttttgtttg ttgtttgata tatttttttag agtttggaat tagttagagt 3000
atttacgatt ttagtagttt gagtaaaggt ttaatattaa ggagaatacg tgggggtggg 3060
tttggtgagg attttcgtgt taggaggcgg gcgttaggga ggagagaggt ttagggaggg 3120
taaggttaag gagtagtggg attatttgag ttataggttg gggaggattt ttattgagat 3180
tttaggttgg gtaataggaa cggttttttt ttttatcgta ggtagtgttt gcggtggtat 3240
aggagattcg gcggtggatt taaatgcgtt ttgttttggt aggtagggaa gtagatcgtt 3300
tttgttttta gttttttttg gaggcggatt tggtttttgt tttggttttg gtttgggttt 3360
ttttatttta ggagttttta gtttttagtt ttaatttagt attcgtttta gaggttgtaa 3420
atttattgtt tatgtataga gggagatagt attttcgtta acgttttcgt atgtaggatt 3480
taatgttcga ttcggttttg atgtagattt tagttcgttt tcgtgtcggt tttcgttttc 3540
gttttttttt cgttattttc gttcgcggtt tttttttcgg tttggcggtt ggggttcgac 3600
gaaggggtag aggttataat ttagcggttt cgcgggtagt ttcggtacgc ggttttattc 3660
gttcgttttt taggttcggg gtttcggttc gtttacggtt acgagggtcg ttcggaatgt 3720
cgcgttcgtc ggtgttgtgg gttaagtatt ttcgtgggat ggagggacga ttggggagcg 3780
ttcgaggttg ttgtgttggc gttcgttttc gagggttggg agttaagagg tgggagtgga 3840
gaggggagga gagggtagag tttttgggcg cgcggttttg ttcgggttta ggcgaggttt 3900
gggtttttag tcgttcgttt ttttttgggg ttcggggttg agttgttgat ggcgggaggg 3960
aggacggtga gggtagtttt tggcggcggg tttttttcga gatttcgagg tcgcgtttaa 4020
atagggcgtt gtaagtaatt ttaggcggtt tattttaaaa cgaattagta gggttaagta 4080
aagttcggtt ttttagtgtt tttttttgta cggtgcgcgg cggagttcga ttttgttttt 4140
tttatatttt gaggtttaga ggaggagttt ttcgtgtatt tcgaattttc ggtcggtttt 4200
ttttgtgtgt atatttgttt taagtagttt gggattaatg ttgggggcgt aatttgtttg 4260

ggaaatatag tttagtttaa agattttggt atattgtttg tttattaggg acgtacggga 4320
ggtaaaggag ggtgcgcggt aatttagcgg tgtttagaat tttgagtagt ggggaggtgt 4380
tgtgttttta tggataggag gggaaggggg ttttgttggg tgggcgtgag ggtgtttgtt 4440
tgaaggtttt tttgagggag ttacgtgtgt gtaggggagg cggaattggg tagatagatg 4500
ggcggttata attttttagc ggtagtttat ttgcgttagg gaaaaaaatt tttggaatta 4560
gggtttgttt tatttataat tacgaagatt ggggtggggg gcgagtttta gtatattgga 4620
tttttttggg gtagggttga ggtgtagttt agatagtaaa ggtcgtcgcg ggttttcggt 4680
cgtatttttt tttttagttt tttgtattta gttaagtttt tgttttttat tttttattta 4740
ttcgagtgga agttaagttt cggtttttcg gtttgtaaaa tggtattata gtttttattt 4800
cgttttttgc gttgggagaa atgggcgttt tgttttcgtt gcgtattgta tgcggaggag 4860
tagatgtttt agtttaggtt ttagttttgt atcgagtttt gaggaattag attttgcgat 4920
ttttagtcgc gttttatttt tttgatttac gtaagggagt ttttagttag gttttttatc 4980
gcgcgggtgt ggaggtgcgg tcggtagggg gagcgaggag gaagggaatc gcggtcgggt 5040
taggtgaggg agggaggaat atttgtcgcg ggtcgtgttg aggcgttgcg ttcgcgtgcg 5100
gaattcgcgt gtgtcgggtt agggttattc gacggcgcgg tttttttcgg gttgcgggag 5160
ggtacgacgg gtggggtggc ggtaggtgaa gaggagtgtg ggcgggtggc ggggtggcgg 5220
ggcggcggag cgcggcgggg cggtgtttta ggcgggaggg agggtcggag gagttttatt 5280
ttgttgcgcg atggaggcgg tggaggcggc ggtttcggtt ggaggttggg acggtttcgc 5340
gtttcgaggg ttttcgggtt tttttagtcg agggtttttg gagggtttta gcgttgagta 5400
acggggttcg ggggttcgaa attaagcgag tgagtatagg tagtcggtgg ggtagggtcg 5460
gttaatagag tcggttgggt ggggtttttt attttttttc gtcgtttttt tggttgatta 5520
gggttttttt tattttgacg gttttttttt tgttgtggta agttggtcgt ttttgtttgt 5580
ttgtaggttt tgtttaggta cgaaggggtt aatgggggtt ggaagggttt tgtttttttt 5640
tataaatttt aggtatagag gtttggggat ttttggatcg atttttgagc ggttgattta 5700
gagtttttag gaggttttag ggttttttat ttttattttt ttttttttaa tgttggtttt 5760
atttataggt tttatatttg gttagtgttt tgaggtgtgt gtagatgtat ataggatata 5820
agtttgggta tatttttagg ttggggtata tgtaggagtt ttatttttat gtttttatcg 5880
attggtttag gtttttttgg gtgtagattg ttaatatagg tacgtttttt gtaatattat 5940
taaatttgcg gttattagta tttttattat tcgtttttac gggattgttt tttttgtagg 6000
agtagttttt ggggataatg gttggttttg tttagagagg aaggaaaggg gtttggaatt 6060
agttggttta tttttatata gtatttttta ttggatttgt ttttgggttt ttttagtgat 6120
attgagagat ataagttatt gttattgtta tggtttagaa tgattattta gggtaagaaa 6180
aatgtattaa ggatagtttt ttttttttga tatggtttat aaagttttag ttgtaggcgg 6240
taggggttgt ggggggtagg atatgaggta ttttatatag gtagtaatat atattatatt 6300
ttagataggt agtattattc gtatatttat ttattgcggt tttggattta tatgcgtttt 6360
tgaattttag gtttagggtt agttttttta ttggcgttta gttttttagt tcgttattgg 6420
gtttttttag tttttagtta gtattttttt tagtttaggg tttgaggttt tgagatttta 6480
aaaattttgg gagaggttgt ttgtttttta tttttatttt gagtatgtat aagcgaaggt 6540
tgtaatgagg ttgtatatag agaggttttg taaatagtgg ttttgattat tagtattagg 6600
gaggttttag taaggtatag aggttttagt aaggtataga ggtatagagt tgaggttgat 6660
atttagttgg agaggttttg tataggaaag ttatgggtgc gttgaattgg ttagaggaga 6720
ttttggagat agtaggtggg gaaacgtata ggttaagtta ggttttggcg tttgggatgt 6780
ttgggtttag aaattaggta gagcggcggg tttcggtttt attttgttta gtttggtcgg 6840
gttatagagt ttttttcggt aggtaggttg gttgttgggt gtcggggggt agtatacgat 6900
ttagtggttt gaagtttttt aatatgtttt cgtgttttcg gttttttcgg ttggttaggt 6960
ttcgttttcg gttttttttg cgttcggtcg gtgcgtaggg aggtcggagg agcgggtatt 7020
gtttattttt cggatgtata agcgttttgg ttagaggcgg gcggtgcgcg taagtgcgcg 7080
tgacgttcgg gttcgcgggt tgcgtagttt gggtagcgtt tttttgttgg ggtttttagc 7140
gacgatagtg agttaatttt agggggttgt tgggtattta ttgggttggg ttttgttttg 7200
ttgcgttaag gaatagagga cgttgcggtt ttggggtttt tgttagtggt cggtgcgaga 7260
gtttaagttg tttttttttt atttaggtgt aggggtagtt agggaggagg tggaggcggg 7320
aggggttaag ggtaggggta gggttgggtt tcggtagtta ttcgttgttt tatgggtgtt 7380
ttgttatttc gggttagggt tgggtgttgt tggtatagat attttttttc ggggggttgt 7440
agaagttgac ggtggtttcg ttgtatagag aaaggtcgtg ttttggtagg agtaacggga 7500
tgtttaaggg gcgttggtta tggaggtttt tgggaatgga ggaagggggt agggagtttg 7560
gaaagtaaga gggttttgta tttttagggt gttttattcg tttgggtttt tcggttgtta 7620
ttagttttaa gtggggggtt tatgacgtta taattttttg ggttaggggt ttttggtagt 7680
gagtggtatt ataggttttc gttttcggat ttcgtgggtt gggttgggtt gggcgcggtg 7740
cggttgggta tacgtagtcg gaggcggcgt cggttaggtc gtcgggcgtt tatggacgcg 7800
cggagttcgt tgtttttttcg agttagtgcg tttagtatcg ttttttttggt tgtagt 7856

<210> 252
<211> 7856
<212> DNA
<213> Artificial Sequence

<220>
<223> chemically treated genomic DNA (Homo sapiens)

<400> 252
   gttgtaatta gagaggcgat gttgaacgta ttggttcggg gagatagcgg gtttcgcgcg 60
   tttataggcg ttcggcggtt tggtcggcgt cgttttcgat tgcgtgtgtt tagtcgtatc 120
   gcgtttagtt tagtttagtt tacggagttc gggggcgggg gtttgtggtg ttatttattg 180
   ttaggggttt ttggtttaga ggattatggc gttatgggtt ttttatttgg ggttggtagt 240
   agtcgagggg tttagacggg tggagtattt tggaagtgta gggttttttt gttttttaag 300
   ttttttgttt ttttttttta tttttaggga tttttatgat taacgttttt tgaatatttc 360
   gttgtttttg ttaaggtacg gttttttttt gtgtagcgaa gttatcgtta gtttttatag 420
   tttttcgagg agaggtgttt atgttagtag tatttagttt tagttcgagg tggtagggta 480
   tttatgaagt agcgagtggt tgtcggagtt tagttttgtt tttgtttttg gtttttttcg 540
   tttttatttt ttttttggtt gtttttgtat ttgggtgagg agagggtagt ttgggttttc 600
   gtatcggtta ttgataggag ttttagggtc gtagcgtttt ttgttttttg gcgtagtagg 660
   ataggattta gtttagtaaa tgtttaatag ttttttgaag ttggtttatt atcgtcgttg 720
   gagattttag tagagggacg ttgtttaggt tgcgtagttc gcgagttcga gcgttacgcg 780
   tatttgcgcg tatcgttcgt ttttggttag gacgtttata tattcggagg gtgggtagtg 840
   ttcgtttttt cgattttttt gcgtatcggt cgggcgtagg gagggtcggg ggcggagttt 900
   ggttagtcga gggagtcgag gatacgagga tatgttggag aattttaggt tattgggtcg 960
   tatgttgttt ttcggtattt agtagttagt ttgtttgtcg ggagaagttt tatggttcga 1020
   ttaggttggg tagagtgagg tcgaggttcg tcgttttgtt tggtttttag gtttaggtat 1080
   tttagacgtt aaggtttgat ttggtttgtg cgttttttta tttattgttt ttagggtttt 1140
   ttttaattag tttagcgtat ttatggtttt tttgtatagg gtttttttag ttgggtgtta 1200
   attttagttt tgtgtttttg tgttttgttg gagtttttgt gttttgttgg agtttttttg 1260
   gtgttggtgg ttagggttat tgtttgtaag gtttttttgt atataatttt attgtagttt 1320
   tcgtttgtgt atgtttagga tgggagtggg aggtaggtaa ttttttttag ggtttttagg 1380
   attttaggat tttagatttt gggttggaga ggatgttggt tgggaattgg gaaggtttag 1440
   taacgggttg gggggttggg cgttagtggg gaggttggtt ttgggtttga ggtttaggaa 1500
   cgtatgtgag tttagggtcg tagtgggtga gtatacgaat agtgttgttt gtttgggatg 1560
   tggtgtgtgt tgttgtttgt atggggtgtt ttatgttttg ttttttataa tttttgtcgt 1620
   ttgtagttgg agttttgtgg gttatgttag aaaagggaag ttgtttttag tgtatttttt 1680
   ttgttttgag tagttatttt gggttatgat agtgataatg atttatgttt tttagtgtta 1740
   ttgaggggat ttagggataa gtttagtgag gagtgttgtg tggggatgga ttagttggtt 1800
   ttaggttttt tttttttttt tttgagtagg attagttatt gtttttagga gttgtttttg 1860
   taggaaggat agtttcgtgg aggcgggtgg tagggatgtt ggtggtcgta gatttggtag 1920
   tgttgtagga gacgtgtttg tgttggtagt ttgtatttag agaggtttgg gttagtcggt 1980
   gggggtatgg gggtggggtt tttgtatgtg ttttagtttg agggtatgtt taagtttgtg 2040
   ttttgtgtat atttgtatat attttagggt attggttaga tgtggggttt gtgggtgagg 2100
   ttaatattga ggagggagaa gtgaaagtga aaaatttggg ggttttttgg agattttggg 2160
   ttagtcgttt aaggatcggt ttagaggttt ttagattttt gtgtttgagg tttgtaggga 2220
   ggagtagagt ttttttagtt tttattaatt ttttcgtgtt tgagtaggat ttgtaggtag 2280
   gtaggggcgg ttagtttgtt atagtagaga agaggtcgtt agggtggagg aaattttggt 2340
   taattagaag ggcgacggga agaggtgagg ggttttattt agtcggtttt gttggtcgat 2400
   tttattttat cggttgttta tatttattcg tttgatttcg gattttcgga tttcgttgtt 2460
   taacgttggg attttttagg agttttcggt taaaggggtt cggaagtttt cgaagcgcga 2520
   ggtcgtttta gtttttagtc ggagtcgtcg tttttatcgt ttttatcgcg tagtaggata 2580
   gggttttttc gatttttttt ttcgtttagg gtatcgtttc gtcgcgtttc gtcgtttcgt 2640
   tatttcgtta ttcgtttata ttttttttta tttgtcgtta ttttattcgt cgtgtttttt 2700
   cgtagttcgg agggagcgcg ttcgtcggat ggttttagtt cggtatacgc gggtttcgta 2760
   cgcgaacgta gcgttttagt acggttcgcg gtaagtgttt tttttttttt tatttggttc 2820
   ggtcgcggtt tttttttttt tcgttttttt tgtcggtcgt atttttatat tcgcgcgata 2880
   ggaggtttga ttgggagttt ttttgcgtgg attaggaagg tgaggcgcgg ttaggggtcg 2940
   tagggtttga ttttttaggg ttcgatgtaa ggttagggtt tgggttgaga tatttgtttt 3000
   ttcgtatgta atgcgtagcg aagataagac gtttattttt tttagcgtag ggggcgagat 3060
   gggagttatg atattatttt ataggtcgga aaatcgaagt ttggttttta ttcgggtagg 3120
   taaggggtgg ggagtagagg tttggttgag tgtaggaggt tgaagggagg gatgcgatcg 3180
   aggattcgcg gcggtttttg ttgtttgggt tgtattttag ttttgtttta agggagttta 3240
   gtgtgttgag attcgttttt tattttagtt ttcgtagttg tgggtgaagt aaattttaat 3300
   tttagaagtt ttttttttta gcgtaggtag gttgtcgttg gagaattgtg gtcgtttatt 3360
   tgtttgttta gtttcgtttt ttttgtatat acgtggtttt tttaggggag tttttagata 3420
   ggtattttta cgtttattta gtaggatttt tttttttttt tatttataaa ggtatagtat 3480
   ttttttattg tttagaattt tgagtatcgt tgggttgtcg cgtatttttt tttatttttc 3540
   gtgcgttttt ggtgggtaga tagtatatta ggatttttgg gttgggttgt gttttttagg 3600
   taagttgcgt tttagtattt ggttttaggt tatttggggt aaatatatat atagaaggga 3660
   tcggtcggag gttcgaggtg tacgggggat ttttttttta ggttttagag tatggagaga 3720
   gtagaatcgg gtttcgtcgc gtatcgtgta aagggaagta ttgaggagtc gggttttatt 3780
   tgattttgtt aattcgtttt aggatggatc gtttggagtt gtttatagcg ttttatttgg 3840
   acgcggtttc ggggtttcgg ggaaggttcg tcgttaggag ttgtttttat cgtttttttt 3900
   ttcgttatta gtagtttagt ttcgggtttt agagaagggc gggcggttgg gggtttaaat 3960
   ttcgtttgaa ttcggatagg atcgcgcgtt taggagtttt gttttttttt ttttttttta 4020
   tttttatttt ttgattttta gttttcgggg gcgggcgtta atatagtagt ttcgggcgtt 4080
   ttttaatcgt ttttttattt tacgaaagtg tttggtttat agtatcggcg aacgcgatat 4140
   ttcgggcgat tttcgtggtc gtggacgagt cgagatttcg agtttgggag gcgggcgggt 4200
   ggggtcgcgt gtcggggttg ttcgcggggt cgttgagttg tggtttttgt tttttcgtcg 4260
   gatttagtcg gttaagtcga gagaagaatc gcgggcgggg gtggcggggg aggagcggga 4320
   gcgggagtcg gtacgggagc gagttgggat ttgtattaaa gtcgggtcgg atattaggtt 4380
   ttgtatgcgg aggcgttgac gggagtgttg tttttttttg tgtataggta gtgggtttgt 4440
   agtttttggg gcgggtgttg agttagaatt gggggttggg gatttttgga gtggagggat 4500
   ttaagttaga gttagaatag gggttaggtt cgtttttagg gagggttgga gatagaagcg 4560
   gtttattttt ttgtttgtta gggtagggcg tatttgggtt tatcgtcgga ttttttgtgt 4620
   tatcgtaaat attgtttgcg atgaagaagg aggtcgtttt tgttatttag tttggggttt 4680
   taatgggagt tttttttaat ttgtggttta ggtggtttta ttgttttttg attttgtttt 4740
   ttttggattt tttttttttt tggcgttcgt tttttgatac ggaagttttt attaagttta 4800
   tttttacgtg ttttttttgg tgttgggttt ttatttaaat tgttggggtc gtgggtattt 4860
   tggttggttt taagttttgg ggggtgtgtt agatagtagg taggaatttt gtttaggagt 4920
   tttaggttta tagttacgtt tttaggaagg ggtttagggt ttggaggagg tgtttttgtg 4980
   agtttggtta ggattaggtt gattatatag ttggtaaagg tttgggagtt agatatagtt 5040
   tggtgtgtat attttgtggt tgggaggata ttggatagga tgataggagg tgtggttttt 5100
   ttattcggtt tagagtattt tttgggattt tttttaggtt ttagtattat ttgttttttg 5160
   gttttagggt agggtttgat tcgtttgaga gttttagtgt ttggattttg ggggataaaa 5220
   gattgtagga ttgataaaga gaatagcgag attttgagag aggtagatat gggatggggt 5280
   aatgggtgga gagaagattg gatagggaag tatatagtcg tgtgatatta gttttttttt 5340
   agtcgggagg gggtcggtta gggtttgtat agttagtcgt atatagttgg tttttaattt 5400
   tttgttattc ggtttagttg tttttcgtaa ttatgggtgc gatgttttgg gttaagtttt 5460
   taggaggata gatgtgggga gatgatgaga gggatggttg gtcggatggg taatggtatt 5520
   ttttgttttt cgatttcgtt aggtattagg attttttgta gattggaagt ttttataatt 5580
   ttagaataaa taaataaata gatagattga tggatagttt agttttggat ttttttttaa 5640
   tttttgaggt tgttgattta gttgtttatt tggtgggagg atttagttat atggggagtt 5700
   ggttgtttat ttgtttgttt attttttggt tatattagtt tttttatttt aggtgtagtt 5760
   aggtagaggt gaggttttga gattttggaa gatttatgtg taggttggtg aggtggttgg 5820
   attttagttt ggcgggttag ttgaggaatt agtttttgtt agtttttttt ttttttgtgg 5880
   ttttagtagg gggagttata tttgttaggg gaagggattt gttttaaaag attttggagg 5940
   agtgttggtt gagggtgagt ggttagaaga tggatacgtg tagataggtt tttttagttt 6000
   ttttgggtag tagggaattt tgattggata gtagttttta tttgtttatg tttaagtatt 6060
   tatattatta cgtgtatata tatttatgag tatgagtatg agtgtatgtg tgtgtttata 6120
   ttatgtttat atttaggtat atatatatgg gagtgtgggt atatatgttt atatatgtga 6180
   gtatattaat attgtgatta tgggtatatg tgtttatatg tatatattag atatatagac 6240
   gtgagtatgg gtatatatag gtatattgta tgtagatata cggattgggt ttagacgtat 6300
   atatgtatat tgtatgtttg tagatacgta agtatgggta ttttatatgt atatagacgt 6360
   gagtacgggt atgtatgtat ttttgtatat agatatacgt gagtatgggt atatatatgt 6420
   atatagtaga tattaatata gatattagtg tgagagagtg tgtatatata gtatatgtag 6480
   tagatatata tataggcgta tatatgtgta ggtttattat ttatagtggt tattgtaatt 6540
   tttttataat aatgacgatg tttatatgtg ttttggattg atttgatttg ggcgaagtat 6600
   tgtggaagtt tgttgttgtg tgaggaagga agaatgtaat attttagttt ttttttgttg 6660
   ggttattttt gaaggggatg tattcggatg atggtattcg attggttagt ttgttttttt 6720
   ggatattggt attggaagag atttttaaga taagatcggt ttatagagaa ttttttgggt 6780
   ggaatttttt gggaggttat tttttatgtt tagattttaa ttttgaattt tgtttggtgg 6840
   cgaatgagtt ttgggaagtt ttgagaagga aggttttggg gatagggtta gaggtaatga 6900
   ggaatgataa ttaagttagt gattttagta tttttgttaa tgggttttaa cgaggttatg 6960
   ttttcgtaaa tattggatat gttgtatttt tttttaaaga tgtttaaagt atttaaagta 7020
   gttaaatata ttatatttgt agtttttatt aaatgtttaa ttgatttaag tagtttatat 7080
   tgttattgtg atagtttagt gtgattgttg ggagagattg tgtggaaatt ttgttttagt 7140
   ggaggggagg attggtttta tattttgtgt ttttcggttt attttgtttt ttggttaagt 7200
   ttttaaggtt gttggattgt tttttttagg ttaggattgt gtttaggtgt ttaggttggt 7260
   atttgagttt ttttgtttat tgttttgggt agattttgtt tatatttttt tttgtgtttg 7320
   tgggggttga tttgaggcgt ttttattgat ttggggttaa aggtaggtgg gttgtttagg 7380
   ttggggtagg ttttggggag tgtttttgat gggtaaggtt ttagtacggg aatttttttg 7440
   ttgagatttg tggaggattt ttaagttcgt gggtagaggg ggatcggggt ttggagagat 7500
   tagttgtata ggtagttttt tttggtcggg gtggcgatgt agggttgggg gttgttaatt 7560
   ttgtttggtt tttcgttttt ttaggggaag gagttggtga gaaggttggt aatattttat 7620
   tttaaaggag aataggattg tttcggtagg atttttgtat tttgggtttt tttgtatagg 7680
   gaggaattag cgtggtgatg tcgtttagtt ttaggagttg ggtttaggag cgagttttat 7740
   tttttagggt tttgtgtttg ggaatacgtt ttggtttttg taagttttag tgatgttatt 7800
   tgagaagtgg ggagaatgtt gtaggtttta tgggtggatg gagttagtat atgtgg 7856

<210> 253
<211> 6159
<212> DNA
<213> Artificial Sequence

<220>
<223> chemically treated genomic DNA (Homo sapiens)

<400> 253

gttggtttcg aatttttgat tttgtgattt gtttatttcg gttttttaaa gtgttgggat 60
tataagtgtg agttatcgtg ttcggttata atgtttttta ttgttttttt ttttttagtt 120
ttgtttattt atttggtttt atttatttta tatatatgat atgatatgat atatatatag 180
atgtatatat atacgtatgc gtatatatat atatatatat acgaacgtat ataattatat 240
atatttttta atgggatata tatatataat tttatagatg gggtattgtt ttattgttta 300
ggttggagtg tagtggtaaa attagagttt ttgggtttaa gtaatttttt tgttttagtt 360
ttttgaatag ttgagattat aggtatgaat tattatgttt agttaattta aaaaaattta 420
ttaggttagg tatggtggtt tatgtttgta attttagtat tttaggaggt tgaggtaggt 480
ggattatttg agtttaggag tttgagatta gtttgagtaa tatgtcgaaa tttcgttttt 540
tttttttttc gcgaggttgt agaaatttta gggtttttgt acgtagtttt tagggttgtt 600
gaggacgtgg ttatggtttg ggttttatat tttttttagg tatttattaa gagtttattt 660
tggttaggtg tcgtttattt ggtattttag tagatgttat gttatttggg ggttttcgtt 720
attcgtttgt ttgttagttt atttattttt ttaatattta gtattttttt ttgttttagg 780
tattttatag ttttatagtc gtgtaaatat tttaaagtta ttcgggtggg gatatagttg 840
ggaataaaat tttgtttttt gatttttagt tattgtggtt tttatggttt ttttagtttt 900
ttagtttttt atgggtgtat agtaaaataa tagttttgtt ttttaggatt tttttttatt 960
taaaatatat aattgtaaaa tatatataat ataatttatt attttaattt ttttatttat 1020
ttatatttat ttatttattt atttatttat ttattttttg agatagagtt ttattttgtt 1080
atttaggttg gagtgtagtg gtgtgaatat gttttattgt aatttttatt tttttgggtt 1140
taagcgatat ttgtgtttta gtttttcgaa tagttgggat tataggtgtt cgttagtatt 1200
tttggttaat ttttgtattt ttagtagaga tagggtttgt cgtgatggtt aggttggttt 1260
taaatttttg attttaagtg atttattcgt ttcggttttt taaagtgttg ggattatagg 1320
tatgagttat cgcgttcggt ttattttaat tatttttaaa tatatagttt tgtgttatta 1380
attatattta tattgtaaaa agtaaagtag aggttttttt ttatagattt ttttgttatt 1440
taattagaat aaataataat tttttttaga agtaaaattt atttaaagat ttgtattaat 1500
atttttaaat atttgttaat aataataaaa aaaaattaat atattttatg tttttagttt 1560
ttataattta gtttaaatat ttgttttagt atgtttatat tggtttaagt aaatattagg 1620
ttatagtttg tttttttttt ttatttgaag gtgtttttat tttttttagt attttataag 1680
ttattttttt ttttttttgt ttttttttgt ttttgttagt tttaaaagtt ttaagttatt 1740
agttaattag gataaatata gaatgtgagg tttcgtttcg gttaatggga attagatata 1800
gtagtagggt ggatacgtta ggttataaat gattttgttt tttttgttcg gtgtattttt 1860
atggtaaagt tgttggcgag tgtatttttt ttgtaaaaag taaaaaaaat ggttttgttg 1920
aggaaattaa atttatgttt aagtgttatt tttttacggt atcggaaaat aagtattttt 1980
aataatattg ttgtgtaatt attattagta tttattttta gaatattttg tattttgtag 2040
aattgatatt ttgtttttat gaaataataa ttttttattt tttttttttt agtttttggt 2100
aattattatt ttattttttg tttttatgat ttggttttat tacgtacggt ttatgagtag 2160
aattatatgg tatttgtttt ttggtgtttg gtttatttcg tgtaatagta tttttaggat 2220
ttatttatgt tgtagtatgt gttagaatgt ttttttttgt taaggttgga tattattatt 2280
ttgtacggat gattttattt tgtttattta tttatttttt aatggatatt gggttgtttt 2340
tattttttag ttattgtgaa taatgtttat aagaatatgg gtgtgtaaat atttgtttga 2400
gttttttttt ttaatttttt ttggtataga tttagaagtg gaattgttgg attatatggt 2460
aattttaggt tgggtatgat ggtttacgtt tgtaatttta gtattttggg aggttgaggt 2520
gggtaaatta tttgaggtta ggagttcgag ataagtttag ataatatggt gaaatttcgt 2580
ttttaataaa aatataaaaa ttagttaggt atgatgatgt acgtttgtaa ttttagttat 2640
tcgggaggtt gaggtaggag aattttttga attagggagg tagaggttgt agtgagtcga 2700
gatttattcg gtttgggtga tagagtaaga ttttggaaaa aaataaataa ataaataaat 2760
aaataatata tatatatata gtaattttag ttttaatttt ttgaggagtt attatgttgt 2820
ttttttagta gttgtatttt ttatattttt attagtagtg tataagagtt ttaatttttt 2880
gaggttaggt atagtcgtta cgtttgtaat tttaatattt taggaggtcg aggtgggagg 2940
attatttgag tttaggaatt taaaattagt ttgggtaata taggatattt tttttttat 3000
aaaaaaaaaa aaaaagtagt cgtgtgtggt ggtgtgtatt tatagtttta gttatttgta 3060

aggttgatat gggaggatta tttgaggtta ggaggtcgag gttgtagtga gttatgatag 3120
acgtttcgtt gtattttagt ttgggaaata gagtgagatt ttgttttaaa aaaaaaaaaa 3180
aaaaaaaaaa ggagttttaa tttttttatt tttttattaa tattttttat tttttggttt 3240
tttttttttt ttaatagtta ttttaatggg tataaaatgg tattttattt agttaagatt 3300
ttttttaatt ggaggggtga agggtgataa agtatttggt taagatttgt gtcgggattg 3360
tagattaaaa gataagatgt ttagaaaaga gtgtagagat tagagaaaaa gacggtagga 3420
tgggtttttg gaggtaggtt ttgggcgggt tgttaaggtt agggatgggt aattggtgtt 3480
gaatgggggg ttttttttgg tttagttttt ttttagtagt atcgggaggt agggttttat 3540
agagttgggg aagatttttg tttttttttt cgttttatat atatattatt tagaataggt 3600
gttgtttgtg aagggttttt tgtgtattag tttttgtgta tggttattaa tttttatatt 3660
ggttttgtga ggtaggtatt aagagttttt tttgatagat gtggaaattg aggttttagg 3720
aggtgaaata atttgtttga ggttatatag atataagatg gtttaaatat tttttgggga 3780
gtaagatagg ttttgatatt atcgttaagg agtttgttgg taaaataaat tttaagtgag 3840
tatttatttt atagagtgat gttgagtggg ggattttgtg agaatatagt tttggtagtt 3900
agggagggtt ttttggagga agaggtatat aggtgtagtt tgaaggataa gagttggttg 3960
ggtgatgcgt ggggtttata ttttcgtttt ttttttatag tttttttatt tgttggtttc 4020
ggtatatttg tatttttaga gatttatttt ttatttataa ttagttttgg ttgggagttt 4080
cggttggggt tattattttt attaggtttt tgtattagtt ttattggggt ttaagttgtg 4140
gtcgagttta ttgggtaggg ttttaagaat ttacgtgtgt ttagagtgat agtttagttg 4200
gagatgaagt ttttgtggga ggaatttaat tagttgggta ttgagatgat cgttattaag 4260
gtaggtaggt gtgagtagcg ggggcggggt gaggtagggg ttagggttgg agtcgtattc 4320
ggttgttgag tatttttttt tcgtaggagg atgttttttt ttttttaggt gaagattttg 4380
ggtatggatt ttttggtcga ttacgttttg tttatggatt ttattttttt ggacgataag 4440
agatataggt gcggggatcg gtttggggta aaggtttagg ttttaggtgt tgagattagg 4500
aattagattt tttttgtatt ttaaggtgag gtaggggttt gtttaaataa tgttagggag 4560
gtttagttta agggtaggac gatagatacg taataatagt ttgagggtag gaggtagttt 4620
aatttagggt atttagtaga tttttatggg tagtgggtta gttttggtag gtagagaggt 4680
ttagtttagg gtagtagggg ttattttgtt atttattagt taggtgtagt tatagggaat 4740
ttaggtttta ataggatttt tttgagtttt gggtttttta gtaaggtgtt tattaggtgg 4800
ggtgagtagt gttggagtcg gtagtagttt ttttatttcg tttgtattgt ttaggcgtga 4860
ttttggttgg ggaagttttt attatttcgt tgtgtttttt aggtatgttt tttatagttc 4920
ggtttggttg gtggcgggta aggtagattt agttatattt ggtcgcgtgt atttttattt 4980
cgattcgtta gttaagggtg tttagtggat gcgttagatt gtgttttttg ataagtttaa 5040
gttgattaat aatttgttgg atgataatgg ttacgtgagg tcggggttac ggtggggacg 5100
gaagggtttg gggttagggt tggtagggat tttggtgttt aatggttttg gtgtttagta 5160
tttttgtttg ttgttgatgg ttaaggtagg gcggggagtc ggggtggcga gtttatttgg 5220
ttgggggtgg tatagtagga agggttgttt aggtaggtgg aaaagggggt gggggcgggg 5280
gaatgtttta gtatacgttg tttttcgtaa ggagagggag gataaggtat ggaacgttta 5340
gtttaggtcg ggttcgtttt atgtgtggat atagtattat taatatttgg ttaaatagtt 5400
aacgattttt gtttttatag aggttatttt ttggggtaaa ggtagggttt agaagatatt 5460
gtttttagcg ttttagatag ggataaatgt taaagaggaa gtagaggtac ggaagggggg 5520
cggagaaagg gtgttgttgt ttttaggggg cgtagggagt tgaagatttg aaggaggtgg 5580
aggatgtagc ggtttatgtt cgttagaggg gatagtaggt gtaaaggttt tgaggttaga 5640
gaattttggg tcggtagaat tgtaagacgt atttatgggg tcggagtgga gttaggaggg 5700
gaagttaagg aggcgaatgt tgaggggtcg ggttgatttc ggggggtttt ggtttttatc 5760
gtgtggggtg gaagtcgttg gacggttagg agtagggggt atatgatgtg gtttttcggg 5820
tagttgtgtg gagaaggaat agaggggtaa ggcggggata ggggataggt taggaggtaa 5880
tcgaggaaat tggtgaacgc gatagtgacg ggttagtata taggtagtgg gtggtagttg 5940
ggatgtgggg tgtgagagga tatggaggta aggatttatg atttaaaggg gtttttgata 6000
cggaattgta gtttttttag tttagatttt gtgttgttat gaaaggaata gggtggtagg 6060
tttagggtag tttttcggtt tggagggaag gattttagtt ttagggagtt cgttgtattt 6120
cgttttattt ttgtattttt gttttgtttt ttttataga 6159

<210> 254
<211> 6159
<212> DNA
<213> Artificial Sequence

<220>
<223> chemically treated genomic DNA (Homo sapiens)

<400> 254

tttgtggaag ggatagagta ggggtatagg ggtgaggcgg agtatagcgg attttttgag 60
gttggagttt ttttttttaa gtcgggagat tgttttgggt ttgttatttt gtttttttta 120
tggtagtata gggtttgagt tggagaggtt gtagtttcgt gttaaaggtt tttttggatt 180

atggattttt gtttttatgt ttttttatat tttatatttt agttgttatt tattgtttgt 240
gtgttggttc gttattgtcg cgtttattag ttttttcggt tgttttttgg tttgtttttt 300
gttttcgttt tatttttttg tttttttttt atatagttgt tcgggaagtt atattatatg 360
ttttttgttt ttaatcgttt agcgattttt attttatacg gtaaaagtta gagtttttcg 420
aagttagttc gattttttag tattcgtttt tttggttttt ttttttggtt ttatttcggt 480
tttatggatg cgttttgtaa ttttgtcggt ttaggatttt ttggttttag ggtttttgta 540
tttattgttt tttttgacgg gtatgggtcg ttgtattttt tatttttttt aggtttttag 600
ttttttgcgt tttttgagag tagtagtatt tttttttcgt ttttttttcg tatttttgtt 660
ttttttttgg tatttgtttt tatttgagac gttggggata gtgttttttg gattttgttt 720
ttgttttagg aagtgatttt tgtgagggta gggatcgttg gttgtttgat taagtgttaa 780
tgatgttgtg tttatatata aagcgggttc ggtttaggtt gggcgtttta tattttgttt 840
tttttttttt tgcgggggat aacgtatgtt ggaatatttt ttcgttttta tttttttttt 900
tatttgtttg gatagttttt tttgttgtgt tatttttagt taggtgagtt cgttatttcg 960
gtttttcgtt ttgttttgat tattagtagt aggtagaggt gttgggtatt aaggttattg 1020
gatattaaag tttttattag ttttgatttt aggttttttc gtttttatcg tggtttcggt 1080
tttacgtggt tattgttatt tagtaggttg ttggttagtt tgagtttgtt aaaggatata 1140
atttggcgta tttattgggt attttggttt ggcgagtcgg ggtggaagtg tacgcggtta 1200
ggtgtggttg ggtttgtttt gttcgttatt agttaggtcg agttgtggaa ggtatatttg 1260
gggagtatag cgaggtggtg agggtttttt taattagagt tacgtttgaa taatataggc 1320
ggggtgaagg ggttgttgtc gattttaata ttgtttattt tatttggtgg gtattttgtt 1380
aaggaattta aggtttaaga gaattttgtt gagatttgag ttttttgtgg ttgtatttgg 1440
ttggtggatg ataggataat ttttgttgtt ttgggttaga tttttttgtt tgttaaggtt 1500
agtttattat ttatggaagt ttgttgggta ttttgagttg ggttgttttt tgtttttagg 1560
ttgttgttgc gtgtttgtcg ttttgttttt aggttggatt tttttggtat tgtttggata 1620
ggtttttgtt ttattttggg gtgtaggaag ggtttaattt ttggttttag tatttgagat 1680
ttgagttttt gttttaggtc ggttttcgta tttgtatttt ttgtcgttta gggggatgaa 1740
gtttatgagt agggcgtagt cggttaggga gtttatgttt aggattttta tttggaaggg 1800
ggggaatatt tttttgcggg agggaggtgt ttagtagtcg gatgcggttt taattttggt 1860
ttttgtttta tttcgttttc gttgtttata tttgtttgtt ttggtgacga ttattttagt 1920
gtttagttgg ttgaattttt tttatagagg ttttattttt agttgaattg ttattttgga 1980
tatacgtggg tttttggggt tttgtttagt gggttcggtt atagtttggg ttttagtaga 2040
gttggtgtaa gggtttgatg ggaatggtga ttttagtcgg ggtttttagt tagagttggt 2100
tgtaggtagg gggtaggttt ttgagggtgt aagtatgtcg aggttagtag ataggaaggt 2160
tgtggagaga ggacggaagt gtgggtttta cgtattattt agttagtttt tattttttag 2220
gttgtattta tatgtttttt tttttaggaa gttttttttg attgttaggg ttgtgttttt 2280
ataggatttt ttatttagta ttattttgtg aggtaaatgt ttatttaaag tttattttgt 2340
tagtaagttt tttgacggta gtgttagggt ttgttttgtt ttttagagaa tgtttgggtt 2400
attttgtgtt tatgtggttt taagtaaatt gttttatttt ttggagtttt agtttttata 2460
tttgttagaa ggggttttta atatttgttt tataaggtta atgtgaggat tagtgattat 2520
gtataggggt tggtgtatag gggttttttt ataaatagta tttgttttag atgatatgtg 2580
tgtggggcgg gggagggggt aggggttttt tttagttttg tgggattttg tttttcggtg 2640
ttgttgaagg agagttgagt taagaagagt tttttattta gtattaatta tttatttttg 2700
attttgataa ttcgtttaga gtttgttttt agaaatttat tttatcgttt ttttttttgg 2760
tttttgtatt tttttttaag tattttgttt tttgatttgt aatttcggta taagttttgg 2820
ttaaatgttt tgttattttt tattttttta attaaaaaaa attttagtta aataagatat 2880
tattttatat ttattaagat ggttattaga aaagaaaaga aattagaaaa taaaaagtgt 2940
tggtaaggag atggaaaaat tagaattttt tttttttttt tttttttttt ttgaggtagg 3000
gttttatttt gttttttagg ttggagtgta gcgggacgtt tgttatagtt tattgtagtt 3060
tcgatttttt ggttttaagt gattttttta tattagtttt ataagtagtt gggattatag 3120
gtgtatatta ttatatacgg ttattttttt tttttttttg tagagagggg atatttttat 3180
gttgtttagg ttggttttga atttttgagt ttaagtgatt tttttatttc ggttttttaa 3240
agtgttggga ttataggcgt gacgattatg tttggtttta agaaattgga atttttgtgt 3300
attgttggtg ggaatgtaaa aggtgtagtt gttggggaaa tagtatggta gttttttaaa 3360
aaattaaaat tagaattatt atatatgtat atattattta tttatttatt tatttatttt 3420
tttttagagt tttgttttgt tatttaggtc ggatggattt cggtttattg taatttttgt 3480
ttttttggtt taaaagattt ttttgtttta gtttttcgag tagttgggat tataggcgtg 3540
tattattatg tttggttaat ttttgtattt ttattagaga cgggatttta ttatgttgtt 3600
taggtttgtt tcgaattttt gattttaggt gatttgttta ttttagtttt ttaaagtgtt 3660
ggggttatag gcgtgagtta ttatgtttag tttagaatta ttatatgatt tagtaatttt 3720
atttttgggt ttatattaga aagaattgaa agaaggaatt taaatagata tttgtatatt 3780
tatgttttta tgagtattat ttataatagt tgagaagtgg aagtaattta gtgtttattg 3840
gaaaatgaat ggatgaataa aatggggtta ttcgtgtaag gtaatagtat ttagttttaa 3900
tagggaagaa tattttgata tatgttgtaa tatggatgag ttttgaagat gttgttatac 3960
gaaatgagtt agatattaaa ggataaatat tatatgattt tatttatagg tcgtacgtgg 4020
tgaggttaaa ttatagaaat agaaagtaga atggtggttg ttaggggttg gggaggagga 4080
gatggagagt tgttgtttta taaggataga atattagttt tgtaagatgt aaaatgtttt 4140

agagatggat gttaatgatg gttgtataat agtgttgtta gaaatgtttg tttttcggtg 4200
tcgtaaagaa atagtatttg aatataaatt taattttttt agtaaggtta ttttttttat 4260
tttttgtaga aagggtatat tcgttagtag ttttgttatg agagtatatc gaataaagga 4320
gatagggtta tttataattt gacgtgttta ttttattgtt gtgtttggtt tttattggtc 4380
ggaacgggat tttatatttt gtatttgttt tgattggtta gtaatttaga atttttaaaa 4440
ttggtaaagg tagaggagaa taaaggaagg aggaagtaat ttatagaatg ttgagaaagg 4500
taaaaatatt tttaaataag gaagaggaat aggttatgat ttaatgtttg tttggattaa 4560
tataagtatg ttagggtaaa tatttaggtt aaattgtggg agttaagaat ataaaatata 4620
ttgatttttt tttattattg ttagtagata tttaagaatg ttagtatagg tttttgaata 4680
aattttgttt ttaagagaag ttattattta ttttaattag atggtaggaa agtttatgaa 4740
gaggaatttt tattttattt tttataatgt gaatgtaatt aatgatataa aattgtgtat 4800
ttaaaaatgg ttaaggtggg tcgggcgcgg tggtttatgt ttgtaatttt agtattttgg 4860
gaggtcgagg cgggtaggtt atttgaggtt aggagtttga gattagtttg gttattacgg 4920
taaattttat ttttattaaa aatataaaaa ttagttagaa gtgttggcgg gtatttgtaa 4980
ttttagttat tcgggaggtt gagatataag tgtcgtttga atttaagggg gtggaggttg 5040
tagtgagata tgtttatatt attgtatttt agtttgggtg atagagtgag attttgtttt 5100
aaaaaataaa taaataaata aataaataaa taaatataaa taaataaaaa ggttaagatg 5160
gtaaattgtg ttatgtatat tttataatta tatgttttaa atggaaaaaa attttgagaa 5220
atagaattat tatttattta tgtatttata aggggttgag aagttggaag ggttatggaa 5280
gttatagtgg ttgggagtta ggggataagg ttttattttt agttgtgttt ttattcgagt 5340
ggttttggga tatttatacg attgtaagat tatgggatgt ttggagtaga ggggggtgtt 5400
gggtgttgga gagatggatg aattggtaga taggcgggtg acggaggttt ttaggtggta 5460
tagtatttgt tgaggtatta ggtggacggt atttggttag ggtgagtttt tagtaggtgt 5520
ttggggagaa tgtggagttt aggttatggt tacgttttta gtagttttga gagttgcgtg 5580
taaaggtttt gggatttttg tagtttcgcg ggggaggggg gaaacgaggt ttcggtatgt 5640
tgtttaggtt ggttttaaat ttttgagttt aagtgattta tttgttttag ttttttgaag 5700
tgttgggatt ataggtatga gttattatgt ttggtttgat aaattttttt aaattagttg 5760
ggtatggtgg tttatgtttg tagttttagt tatttaggag gttgaggtag gaggattgtt 5820
tgagtttagg agttttgatt ttgttattgt attttagttt ggataataga gtaatatttt 5880
atttgtaaaa ttatatatat atattttatt aagaaatata tatgattata tacgttcgtg 5940
tatgtgtgtg tatatatacg tatacgtata tatatgtatt tgtgtgtgta ttatattata 6000
ttatatatat gagatagata aagttaaatg aataaataaa gttggagaaa gagagataat 6060
gaaaaatatt gtggtcggat acggtggttt atatttgtaa ttttagtatt ttgggaggtc 6120
gaggtgggta gattataagg ttaggagttc gaaattagt 6159

<210> 255
<211> 15121
<212> DNA
<213> Artificial Sequence

<220>
<223> chemically treated genomic DNA (Homo sapiens)

<400> 255

aagtagtgtt ttgtaatttt tgttgtgatt atttatattt ttggttagtt atatttttag 60
gtgttttagt ttttgttttt tgtggttatt gtgaatggta ttgtatttat tatttagttt 120
ttaatttgga tattgttggt gtatagtaat gttagtgatt tttgtatatc gattttgtgt 180
tttgaaattt tgttgaattt gtttattata tttaggagtt tttgggtaga gattgtaggg 240
ttttttaggt atagaattat atcgtttgta aagaaggata gtttgatttt ttgttttttt 300
atttggatat tttttatttt tttttttgttt gattgtttta gttagaattt ttagtattgt 360
gttgaatagg agtggtgaga gtgggtattt ttgttttgtt ttgtttttaa gggaaatgtt 420
tttagttttt gtttttttag tatgatgttg gttgtgggtt tgttatagat ggttattatt 480
ttgagatgtg tttttttttg atgtttagtt tgttgaggat ttttaatatg aagtaaggtc 540
gaatttaatt aagagttttt tttgtattta ttgagatgat tatgtagatt ttgtttttag 600
ttttatttat ttgatgaatt atatttattg atttatgtat gttgaattaa ttttgtattt 660
taggaataaa gtttttttga ttgtggtgaa ttggatttgg tttgttagta ttttgttaag 720
gatttttgta tgtatgttta ttagggatat tggtttgaag attttttttt ttgttaagtt 780
ttggtattag aatgatgttg gttttataga atgagttagg gagagttttt ttttttttat 840
tttttgaaat agttttagta ggattggtat tagtttttta tatgtttggt agaaattaat 900
tgtaaattta tttggtttag ggtttttttt ggttggtaat ttttttatta ttaatttaat 960
tttgaaattt gttattgatt tgtttaggtt tttaattttt ttttggttta gttttgggag 1020
gttgtatgtt tttaggaatt tggttatttt ttttaggttt attagttcgt gttagtatta 1080
tttattgaag agattgtttt ttttttaatg tatgtttttg gtatttttgt cggaaatgaa 1140
tttattgtag gatttatttt tgggtttttt attttgtttt attggtttat gtgtttgttt 1200
ttatgttagt attatgttgt tttggttatt atattttttt agtataattt gaagttaggt 1260

aatatgattt ttttagtttt tttttttttt gtttaggatg gttttggtta ttttgtattt 1320
tttatggttt tatatgaatt tttaggattt ttttttttat ttttgtgaag aatgttattg 1380
atattttgat tgggtttgta ttgaatttgt agagtgtttt gtgtagtatg gatattttaa 1440
taatattgat ttttttagtt tatgaatatg gtttattttt ttatttttta atgtattttt 1500
ttatttttta tattaatgtt ttatagtttt tattgtagag attttttatt tttttggtta 1560
agattatttt tagatatttt attttatttg tagttgttgt aaatgggatt atttttttga 1620
tatttttttt agattgttta ttgttggttt atagaagtgt tatattgatt tttgtatgtt 1680
gattttgtat tttgtaattt tattgaattt gtttatatat tttaatagtt ttttggtaga 1740
gtttttagtt ttttttaaat ataagattat attatttgta aataaggata atttgatttt 1800
ttgtttttta gtttggatgt tttttatttt ttttttttgt ttgattgttg tagttagaat 1860
ttttagtatt atgttgaata atagtagtgt aagtggttat ttttgttata ttttagtttt 1920
agttttgttt tttttttatt ttttttttat tggtgtttta gaatatttta tatttttgta 1980
ttggaatttt ataagcgtgt ttttttgttt tttttagatt gtaaaattat ttgaaggaaa 2040
gatttttatt ttattggtag attgtttggg gagatattgt ttattgtatt ttatagcgtt 2100
ttttttataa agatttatgg gggaaatttt ttataggtta aggtttgttt ttattttagg 2160
gatatagttt aatgtatgtt agattatatt tttttatttt gtattggttg tttggaagtt 2220
tagagttata attgtgttat agttttaagt agcgtttttt taaaattttt atttatatat 2280
ttaatagttt ttgtattagt tttttattgt tgttgtatta aattattata aatttagtgg 2340
tttaaaataa tataaattta ttattttata gtttggggaa ttagaagttt aaaattagtt 2400
ttattggatt aaagttaagg tgttagtagg attatgtttt ttttggaggt tttagaggat 2460
aatttatttt tttatttttt ttagttttta gagatttttt atatttcgtg ttttttggtt 2520
ttttttttta tttttaaagt taggagtgta gtattttttt ttttttttga tttttgtatt 2580
tgtttttata tttttttttt ttgattttga tttttttgtt ttttttttat ttttgcgatt 2640
gtattgggtt tattagataa tttaggttaa tttttttatt ttaagatttt taattgaatt 2700
atatttgaaa agtttttttt ttttatgtga ggtaatatat ttataggttt tggggattcg 2760
gaaatgggtt ttttttgtag ggggttttta tttagtttgt tatatttttt ttagtgttag 2820
ttgtatgaaa ttgaaatgtt tgagtttttt aattaaattg tatttttgtt tattttttta 2880
ttttttttat tttttatatt ttagtaaatg gttttgttat ttaataattg ttttgattag 2940
gtatatggtt ttttgttttt ttttattttt atatttagtt ttttatgttg ttttgttata 3000
tagattttta aagtgtattt agaatttgta tattttattt tttggttatt gttattattt 3060
taggttaagt tattattagt ttttgtttga attgttgtta tagtttttaa tttgtttttt 3120
attttagttt ttgttttttt ttagtttatt ttttataaat cgattagatt ttttgtaaat 3180
ataaagttaa ttatgttgtt tttttattta atattttta attgtttttt atttatgtt 3240
gaagaatatt taaatttttt attatgattt ttaaggtttt atggtttggt ttggtttttt 3300
tttaaagaat attttatttt gtttttttta ttattatatt ttggttatat tggttttttt 3360
ggagattttt atttggttta agttatgttt tttatatgta atttttttgt ttggtatttt 3420
gttaattttt tttcgattat atttttgttg agtttttggt tttatttgaa atgttttttt 3480
tttaaagaag ttgttttata taattttatt taattggagt tttttatttt gttgtgtata 3540
aatttttttt gattatttta tttttttaag tagtttttat agatttatta ttattgataa 3600
ttatttagtt gttgtttatt ttatattatt tatttttttt attatattgt taattttgta 3660
aaggcgtagg ttatttttgt ttgttttttg ttgtatgtga tgttttatag gtatgtagta 3720
taggagattt tgaatatatt ttttgttatt tttattttga ttttatttta tttttttggg 3780
tttgtttttt ttatgttttt gtttttttat tttgtattga aatgagagaa aaatttataa 3840
tttttaggtt ttatttaagg ttgggggtta gaggaggtag agtttttttt gtttttgttt 3900
tttttattcg ttatttattt attcgttatt agtattgtgt gttgtgggta aaagaaaagt 3960
tttttattag tgtttttttt tagggaatgt gggatttatt ttggaatgtt aaaataagga 4020
tgaattgttt aaagagaaaa ttagaatgtt tttttggatt aaagattgaa attgttttgt 4080
ttaataatgt aaatagttta tttttattga ttgggggtag agtgtttttt agcgagatta 4140
atatgatatt agttgttttt ttgttttttt ttggtaaagg ttagagataa taaaaattgg 4200
ggttttagat tttagggagg aaggataata tttatagttt tatttgtagg aggtgaggag 4260
ggtttttgag aaaagtgggg tgtgagcgag tataggatat ttaggagaga gatgattagt 4320
aggggatatt ttgggagaaa gagattttga gttttttttt ttttttcgga gttttgattt 4380
ttgagtagtg gtatatagag tgtttttttt tattttttgg tatagtagtt atgttttttg 4440
gtatagtgtt ttggattttt tatatatgtt tttttttttt tagagttttc gtttaagatg 4500
ttggagagta gtttatagtt tgggtattaa gtttgtcgat tttttttagg attttttttt 4560
ttataggtgt tatatgttat gatttatgta taaatatgat ttgtttgata gtgtagtttg 4620
ggtttggaat aattttaatt aatgggaaaa tggtttttaa tatatcgatt agattttttt 4680
aggtataata agataaaagt ttgataattt ggatggagta ttttttttaa atatttattt 4740
tgtgtacggt agttaatggg aaagttaggt ttttttttat ttgatttaat gagtaattaa 4800
gagagggttt tttttttttt attttttttt tgtttttatt tcgtttttta attaaagttt 4860
aataggttgg tggtataatg tggagatttt taaggggtgt tattttagga gtttaggtaa 4920
gagttagggt attttttttt cgttttttaa gtattgttta agtgggtttg aaaagtaggt 4980
aagtgaggga aggaagaagt tagaagtagg taagttgggt aacggttttc ggtaaagttc 5040
gagaagtgtt tatggttatg agtgagttgg gtattcggaa gtttagcgat ggtattgttt 5100
tttatttgtt taatgtggtg gagagtgagt tttaggtagg gagggaaaaa ggcgatttta 5160
cggagaagta attttagatt attttggagg atgtattttt ttggtagaga tttaaggaag 5220

ttattaatga gatgattgtg attaagaatg gtaggtgagt ttatttgtcg tttcgcgtgg 5280
gttggtaggg tttggtagga gagatgagat tttttgaata ttgaagaggt tgtcgttttt 5340
tatttagagg cggcgggatt tgattaaata tgtttatagg aacgattgtt tttaatggtt 5400
tttttttagt taatttaatt aatttatttg agattattag aggatgaagt tatttgtttt 5460
atgtgtattt taaatggaga aattttgggg aaattattat aaagtggggg gtatgtttta 5520
ttgggatggg aaaatggatt taaatgttag aaaataatat taatttgtga ttagttgtat 5580
attttatcgt agatttgagt tttaatttcg tttagataag gtgaaagata attttatttg 5640
gaagttggat gatttttttt taagtatagt aaatttgtta gaaatgtttg gttagttgaa 5700
gattggaagt aaaagggtaa atgtaaattt attgtttatt attttatttg ttttttcgtg 5760
tgtgtttgtg gtgagtgttt atttgagatt gtgtgtgttt gtgatgatat gaaagttgtt 5820
gtagtaatag tggatgaaaa tatattattt tgttttagag aaaagaagat gttttttttt 5880
ttattagtgt aataaattta gggattaatt gaaattgatt tttggtggta ttttttagat 5940
tttattaaat ttagtttgtt aaggggttgt ttaggtaata ttagaagatt ttatttcgta 6000
gaggagacgt aaatattttg aaataaaatt ttatcgttcg tagtttggtt aaattggttt 6060
taatatttag ttggtgttta tatagttttt tatatatatg gttataaaag tataaaatgt 6120
tgaaaggtat ttttagtagg tttagttttg gttgaatttt aattatgata ttttcgagag 6180
aattttgata aaagagaaga gtcgaggtta tttgaatttt tttttgttag gtttttgttt 6240
ttgtgtaaag tgttttcgtt tttgatgttt cggtttttta tattttaata aagtatagta 6300
tagaaaatga attatttttt gttaattttg aagagttatg aattcgagag ttgttatatg 6360
gtattaaaat gttttttaaa atgaaagtta tatttgaatt agggtatttt aagaaattgc 6420
gattatatta gtagtaggaa tattaatttt gtttgatttt tatatttttt tttttaaaat 6480
gggttttata tttattttat atagatttat tgtttttaat attgttattg taattatagt 6540
tttttttttt gatttgtatt tatatattat ttttagtttt ttttatagaa gaaaatataa 6600
aatatagttt taattttata gaattatatt aaatttttaa ttggatttag tttttgattt 6660
tttaaaaatg ggtaaagatg taattgtaaa aattttatta tatttgatta taggtagaat 6720
attttgttta tttttttttg tattattgga tattaattat agtaatattt agttttatgt 6780
tattgattgg gttgttaaat tggttattta ttaaagtaaa aataagtata attattaagt 6840
taatatatgg tttatttatt ttaataagga ataaataata aaagtttttg aaattatgat 6900
aaattttaat ttaataatat gttatattgg aatgtatttt tattatttaa gaaaagtttt 6960
attttgtttt tttattgttt atgtaatttt tttttatatt taaaatgtaa tattttttat 7020
attagtttag tgtggtggtg tgtttttgtt gttttagtta cgtaggaggt tgaggtaagg 7080
ggtttttgag ttcgggatta gtttgggtaa tacggtgaga ttttgttttt aaaaagtaaa 7140
atgaaatttt ttatttttaa cgttatttaa tattagttat atgaataata ttttttgtag 7200
aaaaagtttg attgcggtga atttggttgt ttttatttat ggatgttttg ttgtgtttgt 7260
cgttttttta tagatgaaat tttttttttta aatttttttta agtttcgga gtttttttagg 7320
gttaagattt tgttaggaga tagaaagttt tttttatatt ttgattaaag aagggaagat 7380
aagtttataa gaaattgatg tatttaataa tgaatgaatt tggtttgaaa ttatttttaa 7440
ttttttagtt ttttagatta aattattagt tatattttat ttttgttaat atagatattt 7500
tttttgtttt atttttttta tttttttttt tttttttttt ttttttttta attatttatt 7560
tttttatgtt ttttttttat ttattttttt atgtttatag attttttgtg ttttttttaa 7620
atttttgttt tttatattaa ttttcgtatt tggtgtaggt agatttgtgg tttttttttg 7680
tggagaagaa tgtttttggt atgattattt agttttagtt ttttttattt ataatgttat 7740
ttgggttttt ttttattgat attgttattt tgatttttta aaaaagtatt atttggggtg 7800
gtaattttta tatatttgag gttttattgt tttgtattaa aatagtaaat tttgaaatag 7860
cgtaagatgg gtcgatgaaa gagtgaaaag atttattgtt aagtggagat ttatttttat 7920
atatataaat aggaagtttg tttttgatat tgattagaag ttgtaaagag aaaggttttt 7980
ttttgaaaaa tacggaatta tttttagaaa ttgggatttt gatgttggtt tttttttttt 8040
cgtttttttt tttttttttt tttttttttt tttttttttt tttttttttt tttttttatt 8100
ttattatttt attttttttt tagttttttt tttttttttt tttttttaaa attgaaaatt 8160
tttagttatt tatagttaat agttttttag atataatagt aataagtata atattttttt 8220
agagtttatt tgagttattt ttagtgtatt tagttagtat ttttttttat gtttattttt 8280
taagttggaa atttatattt tagtttgtag aaagaattta ttttagattt tagaggttaa 8340
taaattaaat tgtttgttag tatggtagtt tatgtttgtg attttagaat tttgggaggt 8400
tgaagtggga ggattgtttg agtttaggag tttgaattta gattgggtag tatagggaga 8460
tttttttttt ataaaaaatt taaataatta gttaggaggt ttaggtaggt tgattatttg 8520
agtttagaag ttttagaata gtttgggtaa tatggagaaa ttttgttttt ttaaaaaata 8580
taaaaaatta gttgggtatg gtgatattta tttgtagttt tagttatttg ggaggttgag 8640
gtgggagatt atttgatttt ggggaggtta aggttgtagt gattatatta ttgtatacga 8700
gtttgggtaa tagagtgaga tcgtgtttta aaaaaaaaaa aaaaaaaaaa aaaattaaat 8760
tttttgtttt tgtttttttt atttgtaata tttttattta aggggaaaat gttatatttt 8820
ttgttattat aaaataagag gtgggaatta ttaagtttaa atttgtcgat tagtttcgat 8880
gatttattag tgtttgatga ttaaggggta ttggtgtagt gttattgagt tgttgtttat 8940
agtttttagt aagggtagta taagagagga gaaaagtaaa gttgtatatt aggttaatat 9000
atttttatgt ttttatagtt tatgggtatt tttttttgaa gtttaaaatt atagtttaag 9060
aagattatat gtatttatgt aatttgtttt taattaggtt attttgtttt tttaatgttg 9120
ttgttttttt tttttcgttt atttattttt aattgatatt tgttgttatt tttatgtttg 9180

tttattttta tataaatgtt agtatttatg tattatgtat gttatatata tatatatata 9240
tatatatata tatatatata tttttttaaa agttttgatg agtatttgat aaatagtaga 9300
gttttgagga gagatggagg aaagtgttta taagtttaat tttttgaatt tgtttttaat 9360
tttttgttgt ttttttattt gtaaaatttg ttttattttt gttttttttt ttttttgtaa 9420
attttatttt ttatagtttt ttttagtagt attgattttt ggagattttt tgttagtgaa 9480
ataaaattgg aaagttggtt ttataataaa agtttaatag tttatgggta aagtttaatt 9540
atttgtggtt ttttaggtgt ggtttttttg aggagtgttt atttattttg ttatattttt 9600
tttttttttt ttttaaggag gttttttttt tagggtggat taagtgaaat tatgttgtta 9660
tttagggatt gatttatata ttttttattt tttatatttt gggttttttt atgttagtta 9720
tatttaggaa aaaaatgggg aaaaaaatta ttttgattgg aagtgtagtt aatttttgaa 9780
aataaagttt gattacgagt ggtaattata gattaattag ttattggatt tttagataat 9840
gtatttttgt tattgtgaga taaaagaggg gaaagtgggg tgggaggagg ggaatttggt 9900
ttggaggtag ggtgagtttt atttttagtt ataagatttt aggtaagttg attgattttt 9960
atttattttt tattgttata atagggaatg tattaaaaaa tgtattaaaa tagggaatgg 10020
attaaaaaat agttattttt tagtttattt acgtagtgat aaaaataaaa aataattata 10080
aagtttttgg taaatgtaat attaaagaaa atagagtaat taattatttt attatttttt 10140
agatatgttt tttttttaag agataagata ttgtgatttt tttgttttta ttaatagttt 10200
tgtgatgata taatttatat gttatatagt ttatttattt gaagtgtata gtttagtggt 10260
ttttagtaat tcgtagagtt gtgtatttat ttttataatt aattttagaa tatttttatt 10320
gttttaaaag gaagttttgc gtttttagta gttatttttt ttggtaatta ttaatttatt 10380
ttttgttttt atagagttat ttattttata ttatggatat ttgatatttt gggaattata 10440
taatatgtgg ttttttgtta ataatttttt ttatttagcg taatgtggtt aaggtttatt 10500
aatgttgtag tatgtattag tattttattt tttttattga taaatatttt attgtatgga 10560
tataatatat tttatttatt ttttattagt ttatggatat ttgtattttt ttatatttta 10620
gttattatgt ataatgttgt tatgtatttt tgtgtataaa tgtttgtatg gagttatgtt 10680
tttatttttt ttgtgtatat atttaggagt ggaattgttg gattattgag aaattgtata 10740
tttaattaag tgaggaattg ttagagtatt ttttaaagtg gttgtattat tttatatttt 10800
tgttagtaat gtatgaggat tttaattttt ttatattttt attaatgttt tttattgttt 10860
gttttttata ttgtagttat tttagtggtt atgaagtggt attttattgt ggtttggatt 10920
ttgtattttt ttgatggttg atgaggttaa gtattttttg attgattatg gtttttttga 10980
aatattaggt atagttttga ttatagtatg aaagatggaa agttattata tattttttta 11040
ttttggaacg aaaagttatg ttatattttt tatgagattt tatataatat agttgagaga 11100
gttaagagga gggtatttta atttattagt agtgaggata ataattggaa tttttttttt 11160
attttgtttt agttattttg gttttttttt ttagtttttg aatagagtgg ggttatttaa 11220
atttaagggt tattgtattt gttatttttt ttgtttttaa gtgtattttt tttaatattt 11280
tgtatgagtt attttttttt attatttagg ttttagttta aatgttattt tttgtttaga 11340
tatggttaaa gtagtttttt attttttagt ttttttttgt gatattatgt attttagttt 11400
ttttatgtta tttttgtaat ttgaaatgat ttttttttta atagagatgg ggtttcgtta 11460
aattgattag attggtttta aattttgggt tttaagtgat ttttttattt tggtttttta 11520
aagtgttggg attattggta tgagttatta tatttagttt taaatgattt tatatgttaa 11580
gaaataataa agatttattg atatgtttgt tgtttgattt ttttgttaga atgtaagttt 11640
tatgaataag aagatattgg gtttgtaatt tttgttattg aatttttatt tagaatagtt 11700
tttgggatag agtaggtatt aaaaaattat ttgttaaatg ggtaaataaa tttttaagta 11760
ggtattaaga tttgggaaaa ttttgagggg gatttttttt tttattgtgg tttaattatg 11820
ttagttttga gaagttaata agaaaggaaa agagagtaaa aaaaaaaaaa aggagtagtg 11880
aatgaaatgt aatgtgtgtt ttttgagttt tagttttttt atttataaaa ttggtataat 11940
aatttttttg tgggtgaagt aagatttaag ttgtatttta tttaagtgaa atatttattt 12000
acgtaaagtt ttaggtgtag agtaaaattt agtatagtta ttttttggta ttcgtagagg 12060
attgttttta ggtttttgta aatattaaaa tttgaggatg ttgaagtttt ttatataaaa 12120
tggattagaa tattttgtat ataatttatg tatatttttt tatatattta agttattttt 12180
ggattattta taatacgtaa tataatgtta atgttatgta aatagttatg ttatattttt 12240
tttttgtatt attttttatt gttgtattat ttttttttaa attttttaat ttgtatttgg 12300
ttgaatttat agatgtagat tttgggaatg tggaggtttg attgtatatg ataattatta 12360
ttattagaaa aataattttg cggttaggta ttgtggtttg tatttgtagt tttagtattt 12420
tgggaggttg aggtaggagt tgagtttagg aatttgaaag tagtttgggt aatatagtta 12480
tatattttta aataaaaata aaataaataa aagtgtttcg ttttaagtta ttttttaaat 12540
tataagttag aattttatga aatattttga tattttttat tatgaagata ttagttatgt 12600
ttttatgttt gaataattgt attattattt tggtaattta gtgtatgagt aatatattat 12660
atgttttatg agtttttgtt tttattttta tgtgtattat atagttttat tgagaaaagt 12720
tttggtattt tttttttttg agatatagtt ttattttatt atttagtttg gagtatagtg 12780
gtgtgataat agtttattat agtttcgatt ttttatgttt aagtgatttt tttgttttag 12840
tttttttaat agttggaatt ataggtgtgt attattatgt ttggttaaat tttgtatttt 12900
ttatggagat ggggttttgt tatgttgttt aggttggatt taaatttttt tggtattttt 12960
taattttagt atgtaaaatg ataattatat atatgttatt tttgtgtttt ttttaaaata 13020
atataaaaag taaattttag tattttagaa gaaatattgg attagaatat agagattatg 13080
tttttatata tttcgtataa acgggtttga tttttggttg ttagaataat tttatatgat 13140

ttttagaatt atttttgatg tttattttta tgattttttg taaacggtat ttgtgttttt 13200
aggatttttt tgttttttat atttggttaa tattattaat ataattgaaa atattaaaat 13260
atatcgtaaa agggaatcgt ttaatttgat agtaggagaa aataaaagat aataattttt 13320
ttattgaaga aagaataaaa gaaaatgtaa tatgaagatt ttatattata taggtagtcg 13380
ttttagagga ggtgtattat tttagttaat gtaaaagtgt aattatttta ttaagggacg 13440
aggagtgttg aggtggataa tgtagttttt agcgaagatt tggatttgga atgtatcgtt 13500
tgatgtattt tttttttag aaattttatt taatttatat atttgtttta gtttttattt 13560
taaattaatt tttttttttt gaatatatta atatagtttt ataaagttgt tgtgaagaat 13620
ttattgagtt agtttatgta aagtgtttat ttttatgttt ggtatttagt gagtgttttg 13680
taagtgttgg tcgtattatt attatattta agtttttcgg ttggtttggt tttgttttgg 13740
ttttaaaatt ttattttagg tagtgatttt ggagttggaa ttataaagga tattaatttt 13800
gttttttttt tgtttagtag gtagaaatta tttttttgtg ttgttttttt ggtgatatga 13860
agtttaaata gaggttaagt atggtggttt atatttgtaa ttttagtatt ttgggagttt 13920
gaggtaggtg gatcgtttga ggttgagagt ttgagattag tttggttaat atggtgaaat 13980
tttgttttta ttaaaaattt aaaaattagt taggcgtggt ggtatatatt tgtaatttta 14040
gttatttttt tggaggttga ggtaggagaa ttatttgaat ttaggaggta gagattttat 14100
ttgaaaaaaa aagaagttta aatagaatag ggagtaattg ttaatggtcg tggggttttt 14160
tttcgggatg atgaaaatgt tttagattta taagttagtt atttggatag tttttttaaa 14220
tatttgtgag agtaaatttg ttgttattat tttagtatgt agaagaagga tcgatgaatt 14280
tagatttgta tgttttaaag tgtgtttttt ggatataaat ttttataaaa tgtttagtta 14340
aatgataatt ttattgttat atatatttag ggtgtgtacg tttttacgtt ttttttaaag 14400
agttttatgg tgtatatggg tatgttaaag gttttgtttg aggtattttg gaggaaagaa 14460
ttggttttat tttgtatagt taagagtttt ttttattgat ttaatatgta attttgtttt 14520
tgtttttttg agatagggtt ttattttgtt atttaggttg gagtgtagtg gtattattat 14580
agtttattgt agtttttaat ttttgtgttt aagtaatttt tttattttag tttttcgaat 14640
aattgggatt attatgttta gttaattttt tttttttttg agatgggggt gttttattat 14700
gttgtttagg ttggtatgtt ttgttataaa tgttattatt attatttaga aatagtgttt 14760
tttgtgaggt tgggaagggt tggttagtgg tattagtgtg ttagtgggaa atttttgaag 14820
agagtttttt gtggggatgt ggtatttgat atattttggg ttttaggttt ttgttgtgtt 14880
aaatgatttt attagtggtt tttagatttt aaaacggagt atgttttaaa gtgaaatttt 14940
agtattaaat ttatttataa aaagaagttt tagtagaatt ggggttgtgg atttgggttt 15000
tgtttatttg gtttttttat agggtatttc gtggaaggtt ttagtattga gaggtttttg 15060
gataaggtga gagtttttt aacgtttttt ttgtggttaa gttttgtttt tttttttag 15120
a 15121

<210> 256
<211> 15121
<212> DNA
<213> Artificial Sequence

<220>
<223> chemically treated genomic DNA (Homo sapiens)

<400> 256

tttaaaagga aaggtagaaa tttagttata ggagggacgt tagaggaatt tttattttgt 60
ttaggggttt tttaatattg agatttttta cggaatgttt tgtgaggagg ttaaataaat 120
agggtttaag tttataattt taattttatt ggagtttttt tttataaatg aatttaatat 180
tagggtttta ttttaaaata tgtttcgttt taaagtttga aaattattga tggagttatt 240
tgatataata ggaatttgaa gtttaagatg tattaaatgt tatattttta tagaaagttt 300
tttttaaaga ttttttattg gtatattgat gttattgatt agtttttttt aattttataa 360
agaatattat ttttgaataa tagtaatagt atttatagta aaatatgtta gtttgggtaa 420
tatagtgaga tatttttatt ttaaaaaaaa aagaaattag ttgggtatgg taattttagt 480
tattcgggag gttgaggtgg gaggattgtt tgagtatagg agttagaggt tgtagtgagt 540
tgtgatggtg ttattgtatt ttagtttggg tgatagagtg agattttgtt ttaaaaaaat 600
aagaataggg ttatatgtta agttagtgag aggaattttt aattgtataa agtgagatta 660
gttttttttt ttaggatgtt ttaggtaggg tttttaatat atttatgtat attatgggat 720
tttttgaaga aagcgtgggg acgtgtatat tttaaatgta tgtgatagtg ggattattat 780
ttaattaagt attttatgag aatttgtgtt taaagaatat attttgagat atgtagattt 840
agatttatcg attttttttt tatatgttag aataataata ataagtttat ttttataaat 900
atttggagaa attatttaag tagttggttt ataagtttag agtattttta ttatttcgaa 960
aagaaatttt acggttatta gtagttattt tttattttat ttagattttt ttttttttta 1020
gatggagttt ttgttttttg ggtttaagtg atttttttgt tttagttttt aggagagtag 1080
ttgggattat agatgtgtgt tattacgttt ggttaatttt tggattttta gtagagatag 1140
ggtttattta tgttggttag gttggtttta aatttttaat tttaggcgat ttatttgttt 1200
tagattttta aagtgttgaa attataggtg tgagttatta tgtttggttt ttatttagat 1260

tttatattat tagaaaaata gtatagagaa gtagtttttg tttgttaggt aggagagagg 1320
tagagttgat gttttttgtg attttaattt taaagttatt gtttaaggta agattttgga 1380
gttaaagtaa ggttaggtta gtcgaagggt ttgggtgtaa tggtaatacg gttaatattt 1440
atagagtatt tattaggtgt taggtatggg aataagtatt ttgtatgaat taatttagtg 1500
agttttttat aataatttta tgaggttgtg ttggtgtgtt taaggagaga aaattaattt 1560
aaatgggagg ttaggataag tatataaatt aagtaaaatt tttagggaga aaaatgtatt 1620
agacgatgta ttttaaattt aggttttcgt tgagaattgt attatttatt ttaatatttt 1680
tcgtttttta gtgagatgat tgtattttta tattaattaa aatgatatat tttttttagg 1740
gcggttgttt gtgtgatgta aaatttttat gttgtatttt tttttgtttt tttttaata 1800
gaaggattat tgttttttat ttttttttat tattagatta aacgattttt ttttacgata 1860
tattttaatg tttttaatta tgttaataat attggttaag tgtagaaaat agaaggattt 1920
tgagaatata aatgtcgttt gtaaagaatt ataaaagtgg gtattagaaa tgattttagg 1980
gattatatag ggttattttg atagttagga gttagattcg tttatacgag gtatatggag 2040
atatggtttt tgtgttttgg tttagtgttt tttttgaagt attaaagttt attttttata 2100
ttattttaaa gaaaatataa gagtggtatg tgtataattg ttattttgta tattaaagtt 2160
aaaaagtatt aaaggagttt aagtttagtt tgggtaatat ggtaaaattt tatttttata 2220
aaaaatataa aatttagtta ggtatggtgg tgtatatttg tagttttagt tattggggag 2280
gttgaagtag gaggattatt tgagtatgga aggtcggggt tgtagtgagt tattattata 2340
ttattgtatt ttaggttggg tgatagagtg agattgtgtt ttaaaaaaaa aaagtattaa 2400
aatttttttt aataagatta tatagtatat ataaaagtaa gaataaaaat ttataaaata 2460
tataatatat tatttatgta ttaaattgtt agagtaatga tgtaattgtt taagtatgag 2520
agtatggttg gtgtttttat gataagaaat gttaggatgt tttataaagt tttgatttat 2580
gatttaagaa atgatttaag gcgagatatt tttgtttgtt ttatttttat ttaaaggtgt 2640
gtgattgtgt tgtttaggtt gtttttaaat ttttgggttt aatttttatt ttagtttttt 2700
aaagtgttgg gattataggt gtaagttata gtgtttaatc gtaagattgt ttttttaatg 2760
ataatagtta ttatgtatag ttaggttttt atatttttag ggtttgtatt tgtagattta 2820
attaaatgta aattgaaaaa tttgaaaaga aataatataa taataaaaaa taatataaaa 2880
aaaaaatatg gtataattat ttatatagta ttaatattgt attacgtatt gtaaataatt 2940
tagaaataat ttaagtatat gggaggatgt gtataggtta tatgtaaaat attttagttt 3000
attttatata aggaatttta gtatttttag attttgatat ttgtaggggt ttggaaataa 3060
ttttttgcgg atgttaaggg atggttgtat tgagttttat tttgtattta ggattttgcg 3120
taggtgaata ttttatttaa gtgaaatata atttaagttt tattttattt ataaggaggt 3180
tattatgtta attttataga tgaggaaatt gggatttaga aagtatatat tatattttat 3240
ttattatttt tttttttttt ttttattttt tttttttttt ttattagttt tttaaagtta 3300
atatggttga gttatagtga aaggaaaagt tttttttaaa gtttttttaa attttaatat 3360
ttatttagag atttatttgt ttatttgata aatggttttt tagtgtttgt tttattttag 3420
gaattgtttt aggtgggaat ttaatgataa aaattgtaga tttagtattt ttttgtttat 3480
gaagtttata ttttaataaa gaagttaggt aataaatatg ttaataaatt tttgttgttt 3540
tttaatatgt aagattattt ggggttgggt gtggtggttt atgttaataa ttttagtatt 3600
ttgagaggtt aagatgggag gattatttga ggtttagagt ttgagattag tttggttaat 3660
ttagcgagat tttattttta ttaaaaaaaa aattatttta aattgtaaga gtgatatgaa 3720
ggaattaaaa tgtatgatgt tatagaagga gattaaagga tgagaagtta ttttagttat 3780
atttgaatag gaagtgatat ttgagttaag atttaaataa tgagaaggaa tgatttatgt 3840
aaaatgttag aggaggtgta tttagaggta aaggaaatag taagtataat ggtttttaga 3900
tttaaatagt tttattttat ttagaaatta aaagagaaga ttaaagtggt tgaggtaaag 3960
taaggggaaa attttaatta ttatttttat tgttaatagg ttagagtgtt ttttttttaa 4020
tttttttaat tatattatgt gaggttttat aaagagtgta gtatgatttt tcgttttaga 4080
ataagaaaat gtgtaatggt tttttatttt ttatgttatg gttagagttg tatttgatgt 4140
tttaaagaag ttatgattaa ttaaaagatg tttaatttta ttagttatta gagaaatgta 4200
aaatttaaat tatagtgaga tgttatttta tggttattag gatggttgta atatgaaaag 4260
tagataataa aaagtattgg tgaagatgta gagaaattgg aatttttata tattgttggt 4320
agggatgtaa aatgatgtag ttattttgga aaatattttg gtagtttttt atttggttaa 4380
atatatagtt ttttaataat ttagtaattt tatttttagg tatatatata agaaaaatga 4440
aaatatagtt ttatgtaaat atttgtatat agaggtatat agtaatatta tgtataatag 4500
ttaaaatgta gaaaaatgta aatgtttatg aattgatgaa ggataaataa aatgtgttat 4560
atttatataa tggagtattt gttaataaaa aagatgaagt gttgatatat gttataatat 4620
tgatgaattt tgattatatt acgttaagta aaagaagttg ttgataaagg attatatatt 4680
gtatgatttt taggatatta aatgtttatg atatgaaata ggtaatttta tagaaataga 4740
aagtagatta gtggttgtta gaggaggtga ttgttaaggg cgtagggttt ttttttgaag 4800
taataaaaat gttttaaaat taattgtgga gatggatgta taattttgcg aattattgag 4860
aattattgaa ttatatattt taaatggatg agttgtatgg tatgtgaatt atattattat 4920
aaagttgtta ataaaaataa agaagttata gtgttttgtt ttttagaagg aggatatatt 4980
tgggaaatgg tagaatgatt aattgtttta ttttttttag tattgtattt gttaagaatt 5040
ttataattat tttttgtttt tattattacg taaataaatt gagaaatgat tattttttaa 5100
tttatttttt attttaatat attttttaat atatttttta ttatggtaat gaggaataaa 5160
taaaagttag ttaatttgtt taaggttttg taattgggaa tggaatttat tttgttttta 5220

agttaagttt tttttttttt attttatttt tttttttttt gttttataat gatagggatg 5280
tattatttag ggatttagta attaattgat ttgtgattat tattcgtgat taggttttat 5340
ttttaggaat taattgtatt tttaattaag gtgatttttt tttttatttt ttttttagat 5400
gtagttaata tagagaaatt tagagtgtga gggataagaa atatgtaaat tagtttttaa 5460
gtaatagtat aattttattt aatttatttt ggagagaaag tttttttgga gagaaagaga 5520
ggaaaatgtg gtagggtaaa taagtatttt ttaagaaaat tatatttgaa gaattatagg 5580
tggttgggtt ttgtttataa attgttgggt ttttattatg agattagttt tttagtttta 5640
ttttattgat agggaatttt tagaagttag tgttgttgga ggaggttatg agaagtgagg 5700
tttgtaagaa gaaagagggg tagagatgag gtagatttta taggtgaggg aatagtagag 5760
agttaaaagt aaatttaaaa agttaaattt gtaaatattt ttttttattt ttttttaaaa 5820
ttttattatt tattaaatat ttattagaat ttttagaggg gtgtgtgtgt gtgtgtgtgt 5880
gtgtgtgtgt gtgtgtgtga tatatatggt gtatggatgt tgatatttat gtgaaggtga 5940
gtaggtataa gaatagtaat aggtgttaat taaagataaa taaacgaagg gaaaaaaata 6000
atagtattag ggaagtaagg tggtttggtt aaaggtagat tatataaata tatatgattt 6060
ttttgagttg taattttaaa ttttagagaa aaatatttat gggttgtagg gatatggaaa 6120
tgtattggtt tgatgtgtaa ttttattttt tttttttttt gtattgtttt tattggagat 6180
tatgaatagt aatttagtgg tattgtatta atatttttta attattaagt attggtgaat 6240
tatcgaaatt aatcggtaaa tttaaattta gtaattttta ttttttattt tatgatgata 6300
agaaatatgg tatttttttt ttaggtgaaa atgttataag taaagaaaat aaaagtaaga 6360
gatttgattt tttttttttt tttttttttt ttgagatacg gttttatttt gttgtttagg 6420
ttcgtgtgta gtggtgtgat tattgtaatt ttgatttttt tagggttagg tgatttttta 6480
ttttagtttt ttaagtagtt gggattatag gtaggtgtta ttatgtttag ttaatttttt 6540
gtattttttg gagagatagg gtttttttat gttgtttagg ttgttttaaa atttttgagt 6600
ttaagtgatt aatttatttg ggttttttgg ttagttgttt aaattttttg tagagaagaa 6660
gtttttttat attgtttagt ttggttttaa atttttgggt ttaagtaatt tttttatttt 6720
agttttttaa agttttggga ttatagatat gagttgttat gttagtaagt aatttaattt 6780
attaattttt gaagtttgaa atgaattttt tttgtaaatt aaagtataag tttttagttt 6840
aaaggatgga tatgaaaggg aatattggtt ggatatatta gagatgattt aaatgagttt 6900
taaggaggta ttatatttat tattgttata tttaaaggat tattaattat gaataattga 6960
aagtttttaa ttttaaaaag agaggaggaa agaaaaggtt gaaagggaaa tgaagtagta 7020
gaatggggga ggggagagaa agaaagaaaa ggaggaagag agagagagaa aggaaggaac 7080
gaaagaagag aggttaatat taggatttta gtttttggag ataatttcgt gttttttaga 7140
gaaaagtttt tttttttata gtttttgatt aatgttagag gtaagttttt tatttatata 7200
tgtgaaagta gatttttatt tgataatgag tttttttatt tttttatcgg tttattttac 7260
gttattttaa gatttgttat tttagtgtag aataatgaga ttttaagtgt ataggagtta 7320
ttattttaaa tgatgttttt ttaaaaagtt aaggtgataa tgttaatgaa aaagggttta 7380
ggtgatatta tgaatgaaaa aggttaggat tgggtaatta tattaggaat attttttttt 7440
atagggaagg gttataaatt tatttgtatt aggtgcggga gttgatgtgg agggtaagga 7500
tttgaaggag atataaagag tttatagata taggagaatg ggtagaaagg aggtatagag 7560
gaataaatag ttagggagaa aagagagaag gaagaaggga gtaaagaaga tgaagtaaga 7620
aagatgttta tattaataaa ggtaaagtgt gattaatggt ttaatttgag aaattaagag 7680
gttgggggta attttagatt aagtttattt attattaagt atattaattt tttatggatt 7740
tgtttttttt tttttaatta aagtataaag gaaatttttt attttttggt aaaattttga 7800
ttttaaaggg tttcgagagt ttaggagaat ttaagaaaaa gattttattt ataggaagac 7860
ggtaaatata gtaggatatt tatagataag aatagttaag tttatcgtaa ttagattttt 7920
tttataaaga atattgttta tgtagttaat attaaatgac gttaaagatg aaggatttta 7980
ttttgttttt taaagatagg gttttatcgt gttgtttaga ttgatttcgg gtttaaggat 8040
tttttgtttt agttttttgc gtagttggga taataggagt atattattat attgggttga 8100
tgtgaagaat attatatttt aaatataggg gaaaattata taggtaataa aaaggtaaga 8160
taaaattttt tttaaatggt aaaagtatat tttaatgtaa tatattatta aattggaatt 8220
tattatggtt ttagaaattt ttattattta ttttttattg aaatagatga attatatatt 8280
gatttggtaa ttgtatttat ttttatttta ataaatggtt aatttgatag tttaattaat 8340
agtataaggt taggtattat tgtaattagt atttaataat ataaagaaaa ataagtagaa 8400
tgttttattt ataattagat ataataaaat ttttatagtt gtatttttgt ttatttttaa 8460
agggttagag attaaattta attaagaatt tggtgtaatt ttgtgaaatt aaaattatgt 8520
tttatgtttt tttttatgaa agaagttaaa aataatgtgt aaatataggt taagagaagg 8580
aattataatt atagtaataa tattaaaagt aataaattta tatgaaataa gtgtggaatt 8640
tattttgaga aagaaaatat aaaaattaag taaggttgat atttttgtta ttggtataat 8700
cgtaattttt tgaaatgttt taatttaggt atggttttta ttttagaaag tattttaatg 8760
ttatgtaata gttttcgagt ttataatttt ttaagattga taagaaatag tttatttttt 8820
atattgtgtt ttattaaaat atagaaaatc gagatattag gaacggaagt attttgtata 8880
aggataaaag tttgatagaa agaagtttag atggtttcgg tttttttttt ttattagaat 8940
tttttcgagg gtgttataat tagagtttag ttaaaattaa gtttattgaa aatgtttttt 9000
agtattttgt atttttgtga ttatgtgtat gaaaagttat gtgaatatta gttgaatgtt 9060
ggagttagtt tggttaagtt gcgagcggtg agattttgtt ttaaaatatt tgcgtttttt 9120
ttacgaagtg aaatttttta atattatttg gatagttttt tagtagatta ggtttaatag 9180

aatttgagaa atgttattag agattagttt tagttaattt ttaaatttgt tgtattgata 9240
agaaagaagg tatttttttt tttttaaagt agaataatat gttttatttt attattattg 9300
taatagtttt tatattatta taaatatata taattttaaa tgaatattta ttataaatat 9360
atacggggaa gtagatgaag tgataggtaa tgagtttata tttgtttttt tgtttttaat 9420
ttttaattag ttaaatattt ttggtaagtt tgttatattt aggaggagat tatttagttt 9480
ttaaataaag ttgtttttta ttttatttgg acggagttgg ggtttaggtt tgcgataggg 9540
tatgtagtta gttataaatt aatattattt tttgatattt aagtttattt ttttatttta 9600
atggggtatg ttttttattt tatggtaatt tttttagaat ttttttattt aaaatgtata 9660
taagataggt ggttttattt tttgatgatt ttagataaat taattaagtt ggttggagga 9720
aagttattgg agatagtcgt ttttgtaagt atgtttaatt agatttcgtc gtttttgagt 9780
gaagagcggt agttttttta atatttaagg ggttttattt tttttgttaa gttttgttag 9840
tttacgcggg gcggtagata aatttatttg ttatttttgg ttataattat tttattagtg 9900
atttttttga atttttgtta gagaggtgta ttttttagga tgatttgaag ttgttttttc 9960
gtagggtcgt tttttttttt ttttgtttga agtttatttt ttattatatt gagtagatga 10020
gaaatagtgt tatcgttggg ttttcgagtg tttagtttat ttatggttat aggtattttt 10080
cgaattttgt cgagagtcgt tatttaattt gtttgttttt agtttttttt tttttttatt 10140
tgtttgtttt ttaaatttat ttgaatagtg tttggggagc ggagagaaga tattttggtt 10200
tttgtttaag tttttaggat gatatttttt aaaagttttt atattatgtt attaatttgt 10260
taagttttag ttggggagcg gggtggaggt aggggagagg tagggaagaa aaagtttttt 10320
tttaattatt tattggatta ggtgggagag agtttggttt ttttattggt tgtcgtgtat 10380
agagtagata tttaagaaga gtattttatt taaattatta ggtttttatt ttgttgtatt 10440
tgaagaggtt taatcggtgt attgaggatt atttttttat taattaaaat tattttaggt 10500
ttagattgta ttgttaggta aattatgttt gtgtataaat tatgatatgt ggtatttata 10560
aagaaaaggg ttttggggag aatcgataaa tttgatgttt aggttataag ttatttttta 10620
gtattttaga cgaaggtttt ggagggaaga aagtatgtat ggaaggttta aggtattgtg 10680
ttaagaaata tgattattgt attagagaat aaaggaagat attttgtgtg ttattgttta 10740
agggttaggg tttcggagaa aagaaaagag tttagaattt ttttttttta gggtgttttt 10800
tattgattat tttttttttg aatgttttat attcgtttat attttatttt ttttaagggt 10860
tttttttatt ttttataaat ggagttgtga atgttgtttt ttttttttag agtttagaat 10920
tttaattttt gttattttta gtttttgtta gaggaagata aaaaagtagt tagtattata 10980
ttaatttcgt taaggaatat tttgttttta gttagtgaaa ataaattgtt tatattatta 11040
ggtagagtaa ttttaatttt tggtttagaa aggtattttg gttttttttt tagataattt 11100
atttttattt tggtatttta ggatgaattt tatatttttt gggaagaaat attagtagaa 11160
agtttttttt ttgtttataa tatatagtat taatggcgag tgagtggatg gcgggtggaa 11220
gagataggag tagaggaagt tttatttttt ttggttttta attttgagta gagtttgggg 11280
gttgtgagtt ttttttttat tttaatataa agtaagaaaa taagaatata aggaaaataa 11340
atttaaaaag gtaagataaa gttagggtaa ggatgataga aaatgtattt aggatttttt 11400
atgttatata tttatgaagt attatatata gtaaggaata aatagaaatg gtttgcgttt 11460
ttatagaatt aataatatga tggagaaaat aaatgatata aagtaaataa taattaagta 11520
attattaata gtgataagtt tatgaaggtt atttaaaagg gtgaagtgat taagaaaaat 11580
ttgtgtatag tagggtaggg aattttagtt agatggagtt gtatagaata gtttttttga 11640
ggaggagata ttttagatga gattaaaagt ttagtaaaga tgtagtcggg gaagagttgg 11700
tagagtgtta agtagaagaa ttatatgtga gaagtatggt ttgagttagg tagaaatttt 11760
tagggaggtt agtgtgatta gaatgtggtg gtaaagagag tagaatgaga tgttttttga 11820
gagagggtta ggttagatta tgaggtttta gaagttatga tgaagaattt gaatgttttt 11880
tagtatgaga taggaaataa ttggagagta ttaagtagga aaatagtatg attggttttg 11940
tgtttgtaaa aaatttaatc gatttgtgaa gaatagatta ggaaagggta agagttgaag 12000
taaagaataa attagaggtt atgatagtaa tttaggtaag agttggtgat ggtttagttt 12060
agggtagtgg tagtggttaa aaaatgaagt gtataggttt tagatatatt ttagaagttt 12120
atatgataga ataatatgag agattggata tgggggtgag ggagagtaga gaattatgtg 12180
tttgattaga ataattatta gatggtaggg ttatttattg agatatggaa ggtgagggag 12240
gtggaagggt aggtagaggt atagtttggt taggagattt aaatatttta gttttatata 12300
gttaatattg aaaagaatgt ggtaggttga ataaggattt tttgtaaaag agatttattt 12360
tcgaattttt agaatttgtg agtgtattat tttatatggg aaaaagggat tttttaaatg 12420
tgatttagtt aagggttttg agatggggag attaatttgg gttatttggt gagtttagtg 12480
taatcgtaaa aataagaggg aggtaggagg attaaagtta gaggaagaag atgtaaggat 12540
agatgtagaa gttagagggg agagaagatg ttatattttt ggttttgaag atggaaaaag 12600
gggttagaag atacggaatg taagaagttt ttagaagttg ggaaagataa ggaaatagat 12660
tattttttaa agtttttaga aggaatatag ttttgttgat attttggttt tagtttagtg 12720
aaattaattt tgggtttttg attttttaaa ttgtaagata ataggtttat gttgttttaa 12780
attattaagt ttgtggtaat ttggtatagt agtaatagga gattaatgta aagattatta 12840
ggtatataag tggagatttt gagaaggcgt tgtttagaat tgtgatataa ttgtggtttt 12900
gagtttttaa gtagttaatg taaagtgggg gaatataatt tggtatatat taaattgtgt 12960
ttttaaaata aaagtaagtt ttgatttgtg agaaattttt tttataaatt tttatgagga 13020
aaacgttgtg agatatagtg aataatgttt ttttaaatag tttattagta agataaaaat 13080
ttttttttta aatagtttta taatttagaa gggatagaaa gatacgtttg tggaatttta 13140

atatagaaat gtgaagtgtt ttgaagtatt agtaagggag aaatgaagaa aaaatagaat 13200
taagattgga atatgataag gatgattatt tatattattg ttatttaata taatattaga 13260
agttttagtt atagtaatta gataagagaa agaagtaaaa gatatttaaa ttggaaagta 13320
ggaggttaaa ttatttttgt ttgtagatga tatgatttta tatttggaaa aaattaaaga 13380
ttttgttaaa aaattattag aatatataaa taaatttaat aaagttgtag gatataaaat 13440
taatatataa aaattaatat agtattttta taggttaata gtgaataatt tgaaaaagat 13500
attaaaaaag taattttatt tataatagtt ataaataaaa taaaatattt aggaataatt 13560
ttaattaaag aagtgaaaga tttttataat gaaaattata aaatattgat gtaagaaata 13620
gaagagtata ttaaaaagtg gggagataaa ttatgtttat ggattggaag aattaatatt 13680
gttaaaatgt ttatattata taaagtattt tatagattta atgtaagttt aattaaaata 13740
ttaatgatat tttttataga aatagaaaaa aaaattttaa aaatttatat ggaattataa 13800
aagatgtaga atagttaaag ttattttgag taagaagaaa aaaaattgaa ggaattatat 13860
tatttgattt taaattatat taaagagata tagtaattaa aatagtatgg tattggtata 13920
aaaatagata tatagattaa tggaatagaa tagagaattt agaaataaat tttatagtga 13980
atttattttc gatagaggtg ttaagaatat atattggaga aaggatagtt tttttaatag 14040
atggtgttga tacgaattgg taaatttaaa agaaatgatt aaatttttga aaatatataa 14100
ttttttaaga ttgaattagg aagaaattga aagtttgaat agattaataa taagttttaa 14160
aattgaatta gtaataaaaa aattattaat tagaaaaagt tttgggttag atggatttat 14220
agttgatttt tattagatat ataaagagtt ggtattaatt ttattgaaat tattttaaaa 14280
aatagaggag gaggaatttt ttttaattta ttttatgagg ttagtattat tttgatatta 14340
aaatttggta gaaaaaaaaa tttttaggtt aatatttttg atgaatatat atgtaaaaat 14400
ttttaataaa atattagtaa attaaattta atttattata attaagaaag ttttattttt 14460
gggatgtaag gttggtttaa tatatataaa ttaataaatg tgatttatta aataaataga 14520
attaaaaata aaatttgtat gattatttta atagatgtag aaaaggtttt tgattaaatt 14580
cgattttgtt ttatgttaaa aatttttaat aaattaggta ttaaaaagga atatatttta 14640
aaataatagt tatttatgat aaatttatag ttaatattat attgaagagg taaaagttga 14700
aagtattttt tttgagaata gaataagata aagatgttta tttttattat ttttatttaa 14760
tatagtattg gaagttttag ttggggtaat taggtaaaag aaaaataaaa ggtatttaaa 14820
taggaagata ggaagttaaa ttattttttt ttgtagacga tatgatttta tatttagaaa 14880
attttatagt ttttgtttaa aggtttttag atatgataaa taaatttagt aaagttttag 14940
gatataaaat cggtgtataa aagttattag tattgttata tattaataat gtttaagttg 15000
agagttaaat aatgaatgta atattattta taatagttat aaaaaataaa aattaaaata 15060
tttaggaata tggttaatta gggatgtgaa tgattataat aagaattata aaatattgtt 15120
t 15121

<210> 257
<211> 7316
<212> DNA
<213> Artificial Sequence

<220>
<223> chemically treated genomic DNA (Homo sapiens)

<400> 257

atttgtagga ggattataaa tagtaaatta gagagttatt tttttgggat tcgtgataag 60
aaaattattt ttttaggtgg gagaattgag ttcgaaagag aatgggagtt tttggggggt 120
aggtatttgg ttagttttaa agttcgttaa tattaaaaga ggttgggatt ttgagattaa 180
atgggttggg gtattgggta gaaacgagga gttattgtta aattgttagg atgattagaa 240
cgtttttttt ttaggaaaag tttatatatg aatttatttt tgtatgaaat tttttaatta 300
ggttttatat tttcggtgaa ttttggatgt ttttttgtta atagaatttt taatttagtg 360
atatttcgga ttgaaaagag ttttgcggta aacgggggtg aaagtttaag agggaataag 420
tatataatat ttttttgtta gattttatat atgttgaagg gaatatttat agtaaattgg 480
ttaaagtaaa cgatttcgtt tatttattat ttttttattt tttttttcgt tttttttgta 540
aattttagat aaatattatt tgattataaa agggtcggtt tgtttttttt atagtttgag 600
gtaggtagtg cggtagggaa aagtggcgtg ggttaagttt tcgtttcggg taaggttagt 660
ttttttgttg gtatcgtggt ttgggttaag gatagttgat ttggttttgt ttttttcgat 720
ttttatttcg attttatttt tataaaggaa tattattttt agggtttttt ttttatttag 780
ttttaatatt cgtttatttg tattcgtatt attttaaatg ttgcgggtat tgtagataga 840
gaggtttttt agttaattta tttttttaat tttagagtta taattaagtg aaaatttttt 900
ttgcgaaaag gtggaggaat attttagaga cgttagaaat tatttgggtt ttttttgatt 960
cggaatttgt tttttttttt tttttttttt tttttaagtt attttttttt gggattttgt 1020
tgaagggtag gtttttttaa cgtttttagt ttgttttttg ttgagtgtga gatcgaagga 1080
aagaggatcg aggggtttgt agagagaaaa agatcgtaga tagtcggtag ttggcgttta 1140
atgtcggggt tcggggagcg ttggtttcgt gggttttttt ggaggttagg tttagtataa 1200
gttttcggaa tacgttggtt aatgtttaat tcgaatgtag tggttattag gtcgtttttg 1260

tttgttcgta aattaattat ttagcgtatg gtcggcgtta gagtgggttt attatttatc 1320
gggagggggg cgcgtcgggt acgtagagat aaagaggagt tcgtattttt tcgtttttga 1380
ttttagaatt acggcggttt ttttgtttaa tacgagtttt ttggggtcga gtttgggagg 1440
ttagttataa ttggcggaag tttgtagatt attagtaaga tgtcgatatt ttcgattcga 1500
atttcgtaaa tttttttttc gtttttgttt tcgcgcgttg gaggttggtg tgggagagga 1560
gatgggggtt agaagtagcg atttggggtg attatagggt taagttagag ttatgggtaa 1620
aaaataggta attgagggag gaggatagtg tgaggggtag aatttttttt tagtttattc 1680
gcggagttaa aaataaattt agatattttt aagtaaaatt cgtaagtttt ttttttattt 1740
ttaaagttga tagttggtta gaatgtagag gaatgttttt ttgttgtgcg tgggacgttt 1800
gggggtatcg agtgggtgag gaggaggtcg gttatagtgt ggttgtagaa ttattttgtt 1860
tttattttaa gtagtggggt agagattggt ttgcgagggt aggtaggtaa aattagatcg 1920
ttgggatttg gggtcgtttt tgaaagagta gcgaaggggt tttaggtttc ggaggcgagt 1980
agtttggggg agggggtgta tttttttttt ttattttttt tttttttttt tttttttttt 2040
tttttggggc gggggttttt agagatttat gaaattttgt agtgattttc gtgttttgtg 2100
taaggcggga aatggtttgg tttttcgtat tttttaggtg ggaggagggg gatgcgggag 2160
ggggtgttat gagttaatat tttggggtat tattatttcg taattttttt tttttttttt 2220
ttttttattt taattattgg tagagataga gaggacgtta gagaaagata gattgaaagg 2280
gaaagaaagg ggcgagatgg cgagttagac ggagttcgta gaattatatt attttttttg 2340
gtgattttag ggaattttta acgttggcgt taagtttttt taattatgtg cgttaaaaat 2400
gcgaggttgg agaagtttgt cgttttaaaa gatttttttt tattttagcg tggttggttt 2460
ataagagtat tttattttta tttttttttt ggtgttacgt tggggtttcg gggttgttgg 2520
gggttgcgcg ggtgtataag gtaaaatgcg agagaggttt gtgttggttt atagagatat 2580
atatatttaa agttttgagt tttttaaatt tttaagtttt tagattagtt tttttttcgt 2640
ttttttttta tcgaagaagt ttttatttta ggaaagataa aagaatattg tttttaagat 2700
ttttttttat ttaagtaagg atggtttaag atattggttt ttagaattaa gaattgtggg 2760
tttaggcgaa ttttttgatt tcgatcgggc gttgcggtaa tagagttggt aattcggcga 2820
ttggtaagat tcggtcgttt ttttttcgtt cgtttaagag gaggttttta ttttattcgt 2880
attaaaaata gttaattcgt ttttgaggtg ggggcgtttt acggtttgtt ttataaattt 2940
attttttttt ttcgattttt ggttagatta agttttcggg gtggagaaag aattgaggta 3000
tcgagagata agtgcgatgt ttagagaaga tattaggttg gcgcgttttt tttaatttaa 3060
tcgtttattt tttattttgt gttgtgtatt tagtcgggtt tcgaggtgag ggtagcggtt 3120
tggaggggat aggtttagaa tttagttttt cgttgtgttc gttttgttta gattttttat 3180
tttttttttt atatttatat ttatatttag gtggaatatg ggggttcgta tgtaaatgaa 3240
agacgagatt taaaagggtt ggtaaatgta ttttataaaa attttaaatt tatttttttt 3300
aggagtttag gtagggttag tcgcgtaggt tgtgtacgtg tttgtgtgta cgtgtttttc 3360
ggtgtgtgtt ttagttttag tgtgttggcg cgtgttcgag tatagatata tcgggggtgt 3420
ttgggtattc gtatatggtt gcgggtgggg cgtagtggag aggaagttta tatatgcgtg 3480
tgttgagata tggtcgtatt tttgtgtttt tttagtttag acgtagggga gattagtatc 3540
gagatattcg agttcgggag ttttttagcg gcggtcgggc ggagtttggt tttacgtggg 3600
gttggagagt acgtaagttt ggagtttcgg cgttcgtttt tcggttgtcg tcggtttttg 3660
tagaatcgag tggtcggatg gtagttcgcg gggaggttcg gttattcgtt cggttcgttc 3720
ggggcgggga gaagaaggag agttggagag agaatcggtc gcggcggtcg gagaggcgag 3780
cggagtgtaa gagaggcgag cgtttttgtt cggcgttcgg gcgcgttttt cgtttttttc 3840
gttcggttcg tttgttcgtt ggtttttttt tttttttttt tttttttttt ttttggtttt 3900
gttttttttt tcgattttcg gttggatgat tgaggtattt tagacgtggg ttgaattaga 3960
gcgagcgagc gagtttaggg gttgtagcga tttttcgata agttatttag aggcgatttt 4020
gtgcgcgcgc gttttttagt ggttttcgtt cgttttttga ttatgttgat atatttatag 4080
gataggtagt agtatcgatg cggcgttgcg acgttatagt tttcgatatt ttttttttat 4140
aatttagttt tattttttag tattcgattt gtgaaaatta cgtttatgta gtaatataat 4200
tggttcgaaa gcgttaaaga gttaattaag aggttttcgg tttttcgtag tttatagcgt 4260
agttcgattt tttagagtcg tcgagtagac gttcggtgaa ttttagaggc ggcggagggt 4320
ggcgaggagt tttcgttttt tttcgttttt ttttttttcg atttcgtttt tttttttttt 4380
tttttttttt tttttttttt tttttttttt gttttatttt tttttttttt aaattttttt 4440
tgttttgcgc gtttggatat agatttagga agcgaattcg tttacgtttt aggataagga 4500
agagagagag gtacgggaga agagtttagt aagatttgga ttgaaatcga gatatttttc 4560
ggaggttcgg agtagaggaa ggaggaggag ggcggcgaac ggaagttagt ttgtaattta 4620
agttttgata gcgttggtag aagggggttt aaattagatt tttttttttt taaaggagag 4680
agattttttt cgttttttcg ttttttgtta aagtcgggtt tagtatagtt gtagacgtta 4740
ttagcgagaa agagggagag gaagatagat agggggcggg ggaagaagaa aaagaaaggt 4800
aaaaagtttt ttaggagaat tttttatatt tgtaataaaa gatttagggg ttggagagag 4860
atttttggga cgtagggttg gagtgtttat ttcgagttta gcggtagggt tcgggcgcga 4920
gtcgagattt tgttcgtttt tttcgttttt gaaatcgacg tttaggagcg gttttttaaa 4980
aacgtaaggt ataaggacgg ttattcgcgc gattatgttt gttgattttt cgttttgttt 5040
tttttaaaag cggtttttta ttttttaaaa gatatttttt tttttttttt tgaagtgtat 5100
tagttgtgat ttttgttttt tttttttttt tttttttttt ttgttttgtt tttttttttt 5160
ttttgaatta tgtgttgttg ttaaataata ataaaaaaat aataaaatat agtagttgcg 5220

gatttgtttt cggttggagt ttagcgtttc gtttggagtg gatgagtttt tttatgagag 5280
attcggttat ttttgggata agtatggttt attattcgtt tttattttat cgggcgtcgg 5340
atttcgttat gagcgcggtg ttgggttatt agtcgtcgtt ttttttcgcg ttgacgttgt 5400
tttttaacgg cgcggcggcg ttttcgttgt cgggcgtttt ggttaagtcg attatggatt 5460
aattggtggg ggcggtcgag atcggtattt cgtttttttt tttggggttt taggcgtatt 5520
tgaggttttt gaagattatg gagttcgaag aagaggtgga ggacgatttt aaggtgtatt 5580
tggaggttaa agaattttgg gattagtttt ataagcgggg tatcgagatg gttattatta 5640
agtcgggaag gtaagtagtg ggggtttttt tttttaagtt gttggagagt tttttttttt 5700
ttttattttt ttgtttttag aatagtcggt tggtcggtta ttacggtttg gacgaaaagt 5760
tagttttttt agaaatgtat gtatagattt ttaggttttg tttcggtggt aggaaatttt 5820
agtttatttg ggtatttgta tgggttttgt atttggtttg tattttgggt tttttttcga 5880
atagatagaa ttttttagtg gagtatagat atttttgtag ggagtaggaa agaaaaaaaa 5940
aaaaggtatt ttattgtaag aaatttattt tttaaatttt tttggaatat ttttattttt 6000
ttgttgatgt tgtgttgttt gttttatttt gtttttagag agaaaaattt aaagtttttt 6060
ttttttgtgt gggtattggg aggtttgata ttatttttcg gttttttttg ttttttagtt 6120
tagtttttgg gtttaaaggg gtttgttgtt gttttggtta gagagaaatc gaagggtatt 6180
ttggtttgtt tgtttatatt attttacgtg tttgtaattt aagggcgagt ttagtaggta 6240
attttgtata atttaagatt atgtttgtag atttaaggag ttagtgagga gatatatatt 6300
ttttttttaa tgtgtgaata ttattaatta tattttatat aatgtttaaa ggtttttgtt 6360
tgattaaaat ttgtttggaa gagaagattt tgtaatagtt atttaaaatt attgattttt 6420
tttttgtaat agtattgaag tttgtaaagg tataaagttg atataaaaat aaaatttttt 6480
ttatgatatt ttaagcgttt tgtttttttt taagttaaat gaggttaaag tttggtataa 6540
aatttttttt aaatttataa gatatttagt tagtttttta gtaaagtgtt tttttgtttt 6600
tttttaagtt aagattatag aatgttaatt tttttgtgaa attaatagta atgtggtggt 6660
atagttttgc ggttttggat tggtttaaga agtgggggaa tgtgttagta gttttacggg 6720
tagatcggtt attaggttta ggagtgtatc gaagtttgta aaattcgttt tgggaattta 6780
ttgaagttta gttttatttc gtttatagcg ggattgttat tttgtagtag ggaggggtgt 6840
aatttgacgt ttattttttt gataagttta atattttttt attaatgggt ggtggaaaat 6900
tttagtttta attgatcgcg ttttataaaa attttatttt aatttgttta gatttagata 6960
tttatagaaa aagatatggg taagaatttg tttttaggag ggtaatttgt aaagttaagt 7020
aaggataaaa aaaatattga agaaattgtt agataatgta gagaattgta gtgttataat 7080
gtatttgttt tgaattttgg gacgtttaaa tatggcgaaa ttgagaatat ttaatacgtt 7140
agttgtggaa gaaaacgatt ttgtaattag ttgttttatt ttgaaatatg taagtttatt 7200
agttataata taaagtttta gatttggttt taatattatg tgatatatag gaaattaaat 7260
ttaagattac gggtggttta tttttttttt ttttttttat tttttcgttt tatagg 7316

<210> 258
<211> 7316
<212> DNA
<213> Artificial Sequence

<220>
<223> chemically treated genomic DNA (Homo sapiens)

<400> 258

tttataaaac gaaagaatag aaaagaaaaa gaaagataaa ttattcgtaa ttttgggttt 60
gattttttat gtattatata atattgaaat taagtttaag attttatatt gtgattgata 120
agtttatatg ttttagagtg aggtaattgg ttgtaaaatc gttttttttt ataattaacg 180
tattaaatat ttttagtttc gttatattta gacgttttaa ggtttaaaat aaatgtattg 240
tggtattgta attttttata ttgtttaata atttttttaa tatttttttt gtttttgttt 300
gattttatag attgtttttt tgagagtaaa tttttgttta tgtttttttt tgtgggtatt 360
tagatttaaa taggttgggg taagattttt gtaaagcgcg gttagttaag attagaattt 420
tttattattt attgatgaga aaatgttaaa tttattaagg aaatgaacgt taagttgtat 480
ttttttttgt tgtagaatag taatttcgtt gtgggcgaag tgaaattgga ttttagtgag 540
tttttagaac gaattttgta gatttcggtg tatttttggg tttgataatc gatttgttcg 600
tggagttgtt aatatatttt tttatttttt aggttagttt aaaatcgtaa gattgtgtta 660
ttatattgtt gttaatttta tagaagggtt ggtattttgt agttttgatt taaaaggaag 720
taaggaagta ttttgttgga aaattgatta aatgttttgt gagtttgagg aggattttat 780
gttaaatttt ggttttattt agtttggaag gagatagaac gtttaagata ttatgaaggg 840
gattttattt ttgtattaat tttatgtttt tataggtttt aatgttatta taaaaaaaaa 900
attagtgatt ttaaatgatt attataggat tttttttttt aggtaggttt tggttaggta 960
aggattttta aatattatgt aaaatatggt tgataatatt tatatattaa aaaaaaggtg 1020
tgtgtttttt tattggtttt ttaagtttgt aaatataatt ttaaattatg taaaattgtt 1080
tgttgaattc gtttttgggt tatagatacg tgaagtagtg tgggtaaata aattaaaatg 1140
tttttcgatt tttttttgat tagggtagta gtaggttttt ttagatttag aggttagatt 1200

ggagggtaga aagggtcggg gaatggtgtt aggtttttta atatttatat aaaaggaaag 1260
ggttttaagt tttttttttt gagaataaaa taaaatagat aatataatat taataaagaa 1320
ataaggatgt tttaggaggg tttgaagagt gagttttttg tagtagagtg tttttttttt 1380
tttttttttt tgttttttgt agggatgttt gtgttttatt gaaaaatttt gtttgttcgg 1440
gagggaattt aggatgtaga ttaaatgtaa gatttatgta gatgtttagg taagttgaaa 1500
ttttttgtta tcggggtagg gtttggaagt ttgtgtatat atttttaggg gaattaattt 1560
ttcgtttaag tcgtaataat cgattaatcg attgttttgg gagtagagaa ataaagggag 1620
gaaaaaattt tttaatagtt taggggagga ggtttttatt gtttattttt tcgatttggt 1680
aatgattatt tcggtgtttc gtttgtgaaa ttgattttaa agttttttag tttttaggtg 1740
tattttgggg tcgtttttta tttttttttc gggttttatg gtttttaaag gttttagatg 1800
cgtttggggt tttagggagg agaacgggat gtcggtttcg gtcgttttta ttaattgatt 1860
tatgatcggt ttggttaggg cgttcggtag cgagagcgtc gtcgcgtcgt tgggaggtag 1920
cgttagcgcg gggaagaacg gcggttggtg atttagtatc gcgtttatgg cgaagttcgg 1980
cgttcggtga ggtaggaacg gatggtaggt tatgtttgtt ttaggaatga tcggattttt 2040
tatggagagg tttatttatt ttaggcgggg cgttgggttt tagtcgggga taagttcgta 2100
gttgttgtgt tttgttgttt ttttgttgtt gtttaatagt agtatataat ttaaaagggg 2160
ggaaaaagta aaataaaaaa gaaagaaaaa agaaaaggag gtagaaatta taattaatgt 2220
attttaaagg gaggagggga agtgtttttt ggagaatggg aggtcgtttt taaagagggt 2280
aaggcgaaaa attagtaaat atagtcgcgc gggtgatcgt ttttgtgttt tgcgttttta 2340
aaaagtcgtt tttgaacgtc ggttttagaa gcgagaggag cgagtagggt ttcgattcgc 2400
gttcgagttt tgtcgttgag ttcgaaatag atattttagt tttgcgtttt aggaattttt 2460
ttttagtttt taggtttttt gttgtaaatg tgaaaggttt ttttagaaga ttttttattt 2520
tttttttttt ttttttttcg ttttttattt gttttttttt tttttttttt cgttggtggc 2580
gtttgtagtt gtgttagatt cggttttaat agggagcgag agagcggaaa aagttttttt 2640
tttttaaaaa aaaaaaaatt tgatttaaat ttttttttat tagcgttatt aaaatttgaa 2700
ttgtaaattg gttttcgttc gtcgtttttt tttttttttt tttgtttcga gttttcggag 2760
ggtgtttcgg ttttaattta aattttgttg ggtttttttt tcgtgttttt tttttttttt 2820
tgttttaaaa cgtgagcgaa ttcgtttttt aaatttgtgt ttaggcgcgt agggtaggga 2880
ggatttagag gggaaaaaaa tggaatagag ggaaagagag ggaggggaga gagagagaga 2940
gagagagaga cggagtcgga gagggaggga gcgagagaaa gcgagagttt ttcgttattt 3000
ttcgtcgttt ttagaattta tcgggcgttt gttcggcggt tttagaaggt cgggttgcgt 3060
tgtgggttgc ggggagtcgg aggttttttg attggttttt tgacgttttc ggattaattg 3120
tgttgttgta taggcgtggt ttttataggt cgagtgttgg gggatagagt tgagttataa 3180
aaagaaggtg tcggaaattg taacgtcgta gcgtcgtatc ggtattattg tttgttttgt 3240
gaatatgtta atatgattag agggcgggcg ggagttattg gggagcgcgc gcgtatagag 3300
tcgtttttag gtggtttatc gagagatcgt tgtagttttt gagttcgttc gttcgttttg 3360
gtttagttta cgtttgaaat gttttagtta tttagtcggg gatcgaggga ggaggtaggg 3420
ttaaggagga agggggaggg agagagggag ggagttagcg agtaggcgag tcgggcgggg 3480
aaggcggaga gcgcgttcgg gcgtcgggta ggggcgttcg ttttttttgt atttcgttcg 3540
ttttttcgat cgtcgcggtc ggtttttttt ttagtttttt tttttttttt cgtttcgggc 3600
gagtcgggcg ggtggtcgag ttttttcgcg agttgttatt cggttattcg gttttataaa 3660
agtcggcggt agtcgagaag cgagcgtcga gattttaggt ttgcgtgttt tttagtttta 3720
cgtggagtta agtttcgttc ggtcgtcgtt gaagggtttt cgagttcggg tgtttcggtg 3780
ttggtttttt ttgcgtttgg gttgggggag tataaggatg cggttatatt ttagtatacg 3840
tatgtgtggg tttttttttt attgcgtttt attcgtagtt atgtgcgggt atttaaatat 3900
tttcggtgta tttgtattcg aatacgcgtt aatatattgg gattgaaata tatatcgaaa 3960
aatacgtata tataaatacg tatatagttt gcgcggttgg ttttgtttga gtttttgggg 4020
aagatggatt tgggattttt atgaaatgta tttattagtt tttttggatt tcgtttttta 4080
tttgtatgcg ggtttttata ttttatttgg atgtgggtgt gggtgtagag aagagaatgg 4140
aggatttgga taaagcgagt atagcgagag attgggtttt gagtttgttt tttttaagtc 4200
gttgttttta tttcgaggtt cggttgggtg tatagtatag ggtaaggggt gggcgattgg 4260
gttgggggag gcgcgttagt ttggtatttt ttttaggtat cgtatttatt tttcggtgtt 4320
ttagtttttt ttttatttcg gggatttaat ttggttagaa gtcggggagg gagggtgaat 4380
ttgtaaaata aatcgtgaaa cgtttttatt ttagaggcga gttgattgtt tttagtacgg 4440
gtagggtggg ggtttttttt taggcggacg ggagggaaac gatcggattt tattaatcgt 4500
cgaattatta attttgttat cgtagcgttc ggtcggggtt agaggattcg tttaagttta 4560
tagtttttga ttttgggggt taatgttttg gattattttt gtttagatgg agggaaattt 4620
tgaaaataat gtttttttat tttttttgag atgaaagttt tttcgatgga aagggaacgg 4680
agaaagggtt gatttggggg tttaggggtt taagagattt agggttttgg atgtgtgtgt 4740
ttttgtaggt tagtataggt ttttttcgta ttttgttttg tgtattcgcg taatttttag 4800
taatttcgaa attttaacgt ggtattaagg gaagggtgaa aatgaagtgt ttttgtgggt 4860
taattacgtt gagatagggg aggatttttt gaggcgatag gtttttttag tttcgtattt 4920
ttgacgtata tggttaagag agtttggcgt tagcgttgag aattttttga agttattaga 4980
gagaatagtg tggttttgcg aatttcgttt ggttcgttat ttcgtttttt tttttttttt 5040
ttagtttgtt tttttttggc gttttttttg tttttgttag tggttaaaat agagaaagga 5100
gagagaggga aaattacgag gtggtggtgt tttagagtgt tggtttataa tatttttttt 5160

cgtatttttt tttttttatt tgaagggtgc gaaaggttag gttatttttc gttttatata 5220
gaatacggaa gttattgtag ggttttatga gtttttgggg attttcgttt taaaaaaaag 5280
aaaaagaaaa gaaaagaaag aaatagaaaa aaaaagtgta tttttttttt tagattgttc 5340
gttttcgggg tttggggttt tttcgttgtt tttttaagag cggttttaaa ttttagcgat 5400
ttggttttgt ttgtttgttt tcgtaggtta atttttgttt tattatttgg ggtggaagta 5460
aagtagtttt ataattatat tgtgatcgat ttttttttta tttattcggt gtttttaagc 5520
gttttacgta tagtagagag atattttttt gtattttggt tagttgttag ttttggaaat 5580
gggaggggag tttgcggatt ttatttaaaa atgtttagat ttgtttttgg tttcgcgggt 5640
ggattgagag ggagttttgt tttttatatt gttttttttt tttaattgtt tattttttgt 5700
ttatagtttt aatttaattt tgtgattatt ttagatcgtt atttttgatt tttatttttt 5760
tttttatatt aatttttagc gcgcgaagta gagaacgaga ggaaagtttg cggggttcga 5820
atcgaaaatg tcgatatttt gttaatggtt tgtaaatttt cgttaattat gattgatttt 5880
ttagattcgg ttttaggagg ttcgtattag gtagggaggt cgtcgtaatt ttgggattaa 5940
aagcgggaag gtgcgaattt ttttttgttt ttgcgtgttc ggcgcgtttt tttttcggtg 6000
ggtgataaat ttattttggc gtcggttatg cgttgggtga ttaatttgcg aataaataaa 6060
agcggtttgg tggttattgt attcgggtta aatattggtt agcgtgtttc gaaggtttgt 6120
gttgggtttg gtttttagga gaatttacga ggttagcgtt tttcggattt cggtattagg 6180
cgttagttgt cggttatttg cggttttttt ttttttgtag atttttcgat tttttttttt 6240
tcggttttat atttaataaa agatagatta gagacgttga aagagtttgt tttttaatag 6300
agttttagaa aagggtgatt taaggggagg agaagggaga aagagggtag atttcgggtt 6360
agaaaagatt tagataattt ttggcgtttt tgaaatattt ttttattttt tcgtaaaaag 6420
agtttttatt taattgtagt tttagaatta aaaaagtaaa ttaattgaaa aattttttta 6480
tttgtaatgt tcgtagtatt taaagtagtg cgggtgtaag taggcgagtg ttaaagttgg 6540
gtggggggag gattttgaaa ataatgtttt tttgtggggg tggggtcggg gtgggggtcg 6600
ggggaaataa aattaaatta attgttttta gtttaggtta cggtgttagt aaagaagttg 6660
gttttgttcg agacggaagt ttagtttacg ttattttttt ttgtcgtatt gtttgtttta 6720
aattataaag gggataaatc gatttttttg tgattaaatg gtgtttattt ggagtttata 6780
aaaagggcgg ggaggagggt aaaaggatgg tgggtaggcg gggtcgtttg ttttggttag 6840
tttgttgtaa atattttttt tagtatgtgt gaggtttggt aagggagtat tatatgttta 6900
ttttttttta aatttttatt ttcgtttgtc gtagagtttt ttttagttcg aggtgttatt 6960
aaattgggaa ttttgttgat agaagggtat ttaagattta tcgggggtat gagatttaat 7020
taagaagttt tatatagggg tgggtttata tatgaatttt ttttggggga ggggcgtttt 7080
ggttattttg gtagtttggt aatggttttt cgtttttgtt tagtgtttta gtttatttga 7140
ttttaggatt ttagtttttt ttgatattga cgagttttga aattgattag gtgtttgttt 7200
tttaagggtt tttatttttt ttcgggttta atttttttat ttgaggaagt gattttttta 7260
ttacggattt tagggaagta gttttttagt ttattgtttg tggttttttt gtaaat 7316

<210> 259
<211> 9507
<212> DNA
<213> Artificial Sequence

<220>
<223> chemically treated genomic DNA (Homo sapiens)

<400> 259
   ggtttttttt tggtttgtta taaatgttgg ttattattgt attttaaatg ttaatagatt 60
   tgtgagttta aaatatttat attgtttttt tggtggttgt gtagatataa gtaggatttt 120
   tttaaaatat taatgtaaaa tgttggggag ggaaggaaat aggaggcgtt gggttagaag 180
   cgggagtttt tgtaggttgt ttgggttttt agaagagaaa ggaatgggtg gaggtattat 240
   agagttagtt agtagttttg agggatatta gatgttttgt tggaaatagg gggagagaag 300
   ttaaattttg ttttttttga gtagttagga gaaagatggg gttggtagta gaatggaata 360
   aagttgagag aggatttaga ggaggagtag ttttcgtttt atcgttaaag atttttgtcg 420
   gtatttgagg tttggacgtt ttttttttgt tatttttttt ttttttgttg tttgtatcgt 480
   tgttaaattt tgtaggcgta gtgttttcga atcggatata agaaaattag tttgtattat 540
   ttattgttta ttttaaaatg aaaagattta tttttttttt ttttttgata aatatttaag 600
   aagaaagtaa tgtaattaga aatattattt taatggtatt tttattatat ttataattga 660
   aatttttttt taagaagaaa aggagaataa tgtatttttt ttattttatt agtttttgta 720
   gatattaatg ttataatttt taatgtttag attttttttt ttttatttag aagaaatggt 780
   tttaggtaaa gaaaaaagtt gtttggattg atgtggggag ttttttatgt agataggtgt 840
   agttaatagg tgtgaagaga ttaagtgata tttaaagttt taggtttttg gttgagatgt 900
   ttgggttacg ggtttcgatt aagatatatt ataggtttta tacgatcggt tttggggtta 960
   gtattttgaa tttaaagatg gggtttttgt ttaggatttg aatttatttg gtattaaatt 1020
   ttaatttttt tttttgttta aaaaaggggg tagttttaaa aatttttgtt tcgttgaatt 1080
   tattaaggag gttttagaaa gttaagaacg attggtagaa acgtttttta ggttttagat 1140
   tgcgtagtta attatacgaa tgttttcggg gtattttatt gttgagagta ttcggtggga 1200
   tttttaggtc gttatggtga ttaggaaatt taaggttttt gcggaaataa tcgttaggaa 1260
   tttttttatt ggaaatttta gttagttgtt tattatagag attttttgtt agttgtttta 1320
   ttttagtatg ttttgggtgt gatttgggag agggagtaag gtgtttgggg tttttgtttt 1380
   tagatttttt tgttaggatg ttattaaagg ttagaaattt agtattaggg agttatagga 1440
   gattggttgg agaggaagaa aaattagagg tttcgattaa gtaattgttt tagtgataga 1500
   tttttttaga ttggggtttg gtttttttta ttatttatat ttttatttat ttttttttat 1560
   ttttttaaga ttattattag agtttttatt tatagtttta ggtggggtgg cggtttttat 1620
   ttttatttat gtatagtaga gttttgtagt tgagttttgt agtgtttttt tttaagaaag 1680
   ttttttttta aaagagaggt tttaaggatg tagagattag ttgttttgat taaattggtg 1740
   attttagagt tttttgataa tagtatttta ggtttttttg atttagttag gtttaagttc 1800
   gaaagtttga ttagatttta ggaattttgt aaaataatgg tttggatgat ttaatggttt 1860
   ggatgatttt ggttattgtt tgaatattgt ttgaattgtt gttttgattt ggaggatatt 1920
   aagatgagtt tttataaaga gggttattat gattataatt gttatttttt ttgttttttt 1980
   tgtcgttata aattttggat ttttgtagta tagatttgga gatagattta tgtttttttt 2040
   ttttaaataa agtgttttat gaaatattag atcgaatttt ttttagtggg gatgttttgt 2100
   tttgtgaggt ttggttggtt ggaaagaggt tcgcgggtag ttagaattgg agtcgaagtt 2160
   ttatttttgg ttttgatttt ggttttttga gcgagtttaa tacgtgtgga tttaattttt 2220
   tttttttttt tgtttttttt tttttttttt tatttttatt ttgtgaggga ataagaaaaa 2280
   tagtttgttt tgtcgatagc gcgatattgt ttcgtttgtt aaagaaaatt tgttttttta 2340
   gagttttaga gagtagtggg tttttatgtt tttaacgagt tttttaattg agggaggagg 2400
   tatttagata ggttttttat ttttatttta gatttagata tattagtttg agtttttttt 2460
   ttgtagggtt atagatttgg aatgttttat gttttttttt tttattttaa atattttggg 2520
   agatttatta gtgggttttg atttgtagga tggttgaagt ttttaggttt agtgtgattt 2580
   tgggggtatt ttatggtttt ttagatggga tttttatttt tttaggttgg gtatttttta 2640
   aattggagga gataagaata gtggtatttt tgtgtttaag tattattcgt tttttatttt 2700
   ttgttttttg aagtgtgtaa atttttcgta ttgggatttt ttttagttgg gtttcgaaat 2760
   gatatttttt tttttagttt ttattttatt atttcgaata ggaaaaataa aaaaataaaa 2820
   ataaagaaga atttaattat tattattata ataaaaaatt tttatttttt aaatttggag 2880
   agtaaagagg acgattgtta tttaaagtat ataaagtaat attaatatga atgaggagag 2940
   tagcgataat aatttttggt ggaaatattg gaggagagta ttggatattt aataattata 3000
   ataataatag ttagtatttt ttttatatat tttcggttaa aagtggggaa attagaaatc 3060
   gaataaaata ttgggaagag atatattgtg tttttggaaa aaatataata gagttagatt 3120
   taattcggtg agagggaaag tgattttttt gagaaattag ggaatgtttt atttttagat 3180
   taattttttt ttttgtaaat tgaattggtt aaagtttagt tttgttatta tttttattgt 3240
   tataattttt atttattttt ttagtttcgt ttatattttt attttttatt atttttacgt 3300
   tttataagtt gttatcgggt taggtgggga tatattcgga gagaaataga aagggatgta 3360
   ttttaaagcg agtttttttt tgagaggaaa ataatgggta gttttggaag tgtttttttt 3420
   aaaaacggaa ggagaaggtg aagaaaaaaa ttattaatag taatgggttt ttttggtttg 3480
   ggttttgatt ttttaaagag gaatttttgt tgttttgttg tagtattaaa attagggtag 3540
   gaattttgta aaatgagaaa aataaatttc gggagaaaat ttagttggga gtagtttcgg 3600
   gaaagcgata gtttttcgaa aggaggaagg gaggatgcgc gttgtagtcg gtttcggagt 3660
   ttattgttcg aacgattggg gaaaagaacg aggattttta aatttagttg cgatttttcg 3720
   tttttttttt attttgttcg tggtgcgggt ttcggagcgt ttgggaaagt tagtttgtga 3780
   agttggattt gtagaatttt ttgaatgtcg gtttaggtgg ttgtagggtt cgtagttcgg 3840
   gatgtcggag aaaggaatta taaggaaaag acgttacgtt cggagttttt tttttttttt 3900
   gtagtttcgg cggggtttag gtttcggttt tcggtttatt tcggtttaga ggttaagtag 3960
   gaatttattt cgtcgattta gggtttggga gaaggatttc gggttgcgtt tttggtattt 4020
   gggagtaaat acgagtttcg gatttgggga tatatcgtta ttaatagttt gggaaacgaa 4080
   agcgaagttc ggggtagtta ttgtagtttg gttgagagaa aggacgcggg ttgtgttttt 4140
   tggaagtaaa ggggtttgcg gtttagttgg tttgggagtt tgtggtcggc gttggaagtt 4200
   gttcgttttt ttcgcgggtt tgattttggt tttcgtcgta gttttgtcgg tcgattgttt 4260
   ttttgtatat tttgttgtcg ttttagtttt tataggatcg ggtttggagt taggttatgt 4320
   ttcggaaagt tttgggggtt ggggacgcgt aaaatttaga atcgaatttc gaagttgggg 4380
   gtaagtttag atttagacgt ttagtttttt cgggattttt tggcggagaa tttatttttt 4440
   cggaaggttc gacgtttttc ggttttgtgt tgggtaggtt tagttttttt ttcggcgtta 4500
   ttaggggcgt ttacgcggta cgttttgtaa tttaatttag tgggttttta gcggtgcgta 4560
   aaagtttgcg agtatttatt attgcgttgt ttagaaattg tgtttattga ttagattaaa 4620
   aataatagtc gtcgtgatta taaaataaaa tagagtgttt cgtgtttcgg cgggttttgg 4680
   tataataatt attttttata gaaattattt ttttttattt ttatttttat tttattattt 4740
   attcgtattt cgtttttgtc gggttatttt gggacgaatt gtatttttgg attttttttt 4800
   tcgtaaggta tattacggag aatttttttt gtttcgtgtt ttttttacga taatttagta 4860
   attattttta tgtaagtttg taagagggta ttgagttatc gtattttaag tttaattagt 4920
   tagagcggcg tttcggtatt tatttgttta gagttttttc gcgggggatt taaaaataat 4980
   aataataata ataaggattt tatgttttat gtaagagatt ttagtttttc ggaataattc 5040
   gatttttttt taatagaggt ttgaggtgga tttttatttt ttttcgtgaa gagttttttt 5100
   tttttttttt ttttaagaaa gtcgattttg gttttatttg tgttttatgt ttattatttt 5160
   tattttttcg gagaaagtcg ggttggttta tgtttttatt tattttcggg gttaagacgt 5220
   tcggaatttg tggttgcgta gattcggtat tgataggcga agacggagag aaatttattt 5280
   ttcgtcgttg ttttttagtt aaacgtgata gcgcgcgggt cggttgcgtg attcgtgacg 5340
   tttttaagtt ttataggtgt agcggttggt gagatagtcg ttatcgtttg gttgtttttt 5400
   tattttattg ggtatgtttt ggtaataaat agtaatattt aatttgtcgg agattataaa 5460
   ttaattttga gttgggaggt acgtgttttt tttgatagac gttggaagaa gatttggttt 5520
   aaagaggttt tttttggtgg ttttttttaa agtttttatt tgagttttgt tttttagcga 5580
   ggcgtatttt tggtttttgc gttttaaaga agaggtggga tagttggagg tgagttttat 5640
   tttggaggat tgagggggga aataggtttt tttttgtatt tggttttttt tggtttttta 5700
   tttcgtttta gttttgttga attttttttt gttgtgttgt tgttttttat gaacgaaata 5760
   agaatggttt cgtgtgttat ttggtaagaa tggatttttt cgaaaggttt gaaaattttt 5820
   attttgtttg gtggtttaga ttatttgtgt ttaatgagtt aagtaaattt ggttgggtta 5880
   ttatttggga gagtttagat tttttttttt tggaggggtt tgaagattat ttatgggaat 5940
   taattttatt tttaaatttt tgagaattta aagggattgg gaaggttttt ttggattttt 6000
   tttttttttt attggagatg tagcggttta gttttttttc gatgttatgt ttgtttttcg 6060
   tgttttcggt ttagggtcgt tttgttagta agtttaaagg tttttaagtt gatttggagg 6120
   gtgagggtgt ttttataaat aaatatatcg tttgggtaaa gggaaaatat ttgtttgtaa 6180
   ataaatttgg aaggtggagt gagtattata gtaaaggagg gttggttttt ttttcggttg 6240
   ttttatattt agggttggag attttttatt ttggtatttt gtagtaatgg tttggtttgg 6300
   gattttagta gagtagttat agagggttat atagttgttg atttcggggt attttttttt 6360
   tttttatttt tttttttaga ttttgatttg taggtttaag ttttttatta gaattttaga 6420
   aaaattagta tgatgggggt ataggaggta aggatgggaa atagaatagg atttttgata 6480
   tttattttaa gtattaatat tcggtgtttt aatttgggtt tgtttagatt gagatttatt 6540
   gattgtgttt gtgtttttat tgattttttt aatttatttt aaaaaaaaaa aatatatttt 6600
   agagagggaa agggtaagga gaggagatta gtggagatga aaattttagt tagtttttag 6660
   tgattgtttg attattttaa taaaattggg tattgttttg tgtatatttg gagtagggga 6720
   cgtttggtgg ttttattggg tggggggtga gggttcgtag cgatttttta gtatttttga 6780
   tttcggtttt tattttaatg tatttttatt ttgtttttat tttagagtcg ttgagagtag 6840
   gggtgatgag tagggggtgg aggggagatg ttaggaaggc gagcgtcggt taggcggggt 6900
   taggtagttt tttttttcga ggttagcgtt ggagagaatg tttgtaaggt tgcggaggtt 6960
   cgcggtgtgt ttgtgtgtgt gcgtttagat tcggtttttt gtatcgttag cgttattgag 7020
   attatttttc gattagaagt cgatcgcgtt tgaaatgatt tgtgtaggag tttttgtagt 7080
   tatcgtttgt ttagagaagt agagatggat ggaggttggg aaaggggtag agaggaggga 7140
   gttattgtag gtttgtgttg agagtcgtat tgtgatttga gtgttcggga aatttagtgg 7200
   aaatttgggg tggggggaag ggaggacggg agggtgggag ggagagagaa gggggagggc 7260
   gatagagtgt agtgggagtt agttggatag gcgattttag tattttgtga gtatcgaggt 7320
   aatttaacgt tattgtgttt agttgagttg gtttgtattt tagagagaga gagagaggga 7380
   gagagagtga gagagattga tttttatttc gaattcggga attttaattt tgaaagttgc 7440
   gtttagaaag gatttcgatt atttattggg tttttaattt tttttttttg ggggtgtaaa 7500
   ggaggagcgg ggtattgaga ttatatggtt gtcggtgttt ttggaggtta ttttgtgttt 7560
   tttggcgttt gttaattggg aagtatttag ggagagtaag cgtatagtag aggaggtgtg 7620
   tgttggaggt gggtagtcgt cgcggaggtt ttagcggtag gtgcgtttta gtaggtagta 7680
   gtagtcgtta ttttgggtaa gtagtaaatt tcgtataaat ttcggagtta ttatgtttgt 7740
   tttttcgttt tatcgtcggt ttttttgtta ggagtagtag aggatgtggt gaatgtatcg 7800
   gttttatcga acgaggtaat cgtttcggta gatggttcgg gaggtttagg agggagttcg 7860
   tcggtcgagt cggttggggc ggcgggaatg gcggatttta gttcggtttt gtattttgta 7920
   gagtatagtt tgtagagatt ttgcgcggtt cggagttttt cggtaaatgg agtttgggag 7980
   gtcgtcgcgg ttggcggagc ggtattgagg tttgtagaag tacggtttgg ttttgttaaa 8040
   tcgtattttg ttcgttttcg ttagtttaga gtttttcggg attttggcga gggcgcgggg 8100
   taggattttt ttcgcggtcg gtagtgaatg ggagcgcgtg taggggcgtt agttttgttt 8160
   ttttttgttg tgagtaggta gcggggtttt agtcgtcgtt tttttgaagt ttcgagaacg 8220
   aggcgaagaa agttgtttgt aagttaggtt ttagaagttg tttttttttt cggttttatt 8280
   tttagatttt tggttatagt aggttagggt gggatttggt cgtttgttta agtagttgtt 8340
   gaggttttgg gggattatcg tttgtgttcg ttttttaaag ttttttcgtt ttagggggaa 8400
   tttttatttg cgtatagaag agattggaag gagattagag agtaggagat aggtttatcg 8460
   ggtttgaata gttttgcggt agttattttg tgtgttttaa gtttatacgt ttgtaaattc 8520
   gggtcgtttt atgtattgcg gggattcggg ttgtagattt aatgttttta gtttaggatt 8580
   tattcgagaa tgattttgtt ttcgttaatt ttgagtttga gtttgtagtt ttagtaggtt 8640
   aggtttggtt gaagtttaga gaaatttttt tcggtttggt tattttataa aatgttcgtt 8700
   atttttcgtt cggtattaga gcgttggtgg cgggtattta gtgacgagtt tttttcggaa 8760
   cggttgtacg gtgatttttt ttatttagtt ttttgtttgg ttatttttta ttttttaacg 8820
   aattttattt aaggttggga taaagaaaag gatgtttaat tttaaaggat ttttaatgtt 8880
   aaggtttgta attggtaaaa ggtgtatatg aatttttttt tgaatttagt tttaatttgt 8940
   ataatttttt ttttttgtgt tttttttttt gtaagaggcg ggttatttta tatatttttg 9000
   tttgttgtag aaaattattt tttttgtgag gtttaataaa aagagtttcg acggtatttt 9060
   gtaatttggc gaagaaagaa attttttaaa ggatattggg tttttgtttt gttttgtttt 9120
   tggtttttat ttagtggttt ttaaaaatat ttacgtttta tttttattgg tttttttaga 9180
   agtttagata aatttgacgg gtagtttgtt tattacggag agtttgcgta ggggagatgc 9240
   gggttttatt tttttcgggt ttgacgcggg ttttttgtaa gaagttgtgt ttgtttataa 9300
   agtaatagta atattattag gaatattagt aatagtaata attttagggt tttttgtagt 9360
   tattttcggt tgggaacgga ggtgttggta gttggaaatt gggggttaaa ttgttttttt 9420
   ttgttattag aattgtgttt tttaattttt ttttcgtttt ttttttgttt tttttatttt 9480
   tatttttttt tgttttttgt ttttaga 9507

<210> 260
<211> 9507
<212> DNA
<213> Artificial Sequence

<220>
<223> chemically treated genomic DNA (Homo sapiens)

<400> 260

tttgaggata agaagtaggg ggagatgggg gtggggagga tagagagaga acgagagaaa 60
ggttggagag tataatttta gtgataggag ggagtagttt ggtttttagt ttttagttat 120
tagtattttc gtttttagtc gaaggtgatt gtagaaggtt ttagaattat tattgttatt 180
agtattttta gtagtattat tattatttta taagtaggta taattttttg taaaagattc 240
gcgttagatt cggagaaggt gaggttcgta ttttttttgc gtaggttttt cgtgataagt 300
aagttgttcg ttaaatttgt ttggattttt ggaagagtta ataaagataa ggcgtgggta 360
tttttggaag ttattgggta ggaattaaaa ataaaataaa ataaaaattt agtgtttttt 420
agagaatttt ttttttcgtt aaattataag atgtcgtcga aatttttttt attaaatttt 480
ataagaggaa tagttttttg tagtaggtag aaatgtgtga aataattcgt tttttgtaaa 540
aagaaaaata tagaagggga aggttgtgta gattgagatt aggtttaaaa gagaatttat 600
atatattttt tattagttat aggttttaat attaaaagtt ttttaaaatt aaatattttt 660
ttttttattt taattttaag taagattcgt taaaagataa aaagtagtta aatagaagat 720
tagatgaaga aagttatcgt gtagtcgttt cgaggaaggt tcgttattgg gtgttcgtta 780
ttagcgtttt gatgtcgggc gggggatggc gggtattttg tggagtgatt aggtcgaaag 840
gggttttttt gaattttagt taggtttggt ttgttaaaat tgtaggttta ggtttaggat 900
tggcgaggat aaaattattt tcggataagt tttaggttgg agatattgaa tttgtagttc 960
gggttttcgt aatgtataga acggttcggg tttgtaggcg tatgagttta gaatatataa 1020
aataattgtc gtagagttgt ttaagttcga tgagtttgtt ttttgttttt tgattttttt 1080
ttaatttttt ttatacgtaa gtaggaattt tttttagaac gggaagattt tgggaagcgg 1140
gtataagcga tggtttttta aaattttagt agttgtttaa gtaagcggtt agattttatt 1200
ttgatttgtt gtggttagag atttagaaat ggggtcgagg agaaggatag tttttgaggt 1260
ttgatttgta agtagttttt ttcgtttcgt tttcggggtt ttagggaggc ggcgattgga 1320
gtttcgttgt ttatttataa taaaagaaag tagggttggc gtttttatac gcgtttttat 1380
ttattgtcgg tcgcggggag aattttattt cgcgttttcg ttagggtttc ggggagtttt 1440
gggttggcgg aggcgaataa gatgcggttt gatagaatta ggtcgtgttt ttgtaaattt 1500
taatgtcgtt tcgttagtcg cggcggtttt ttagatttta tttgtcgggg agtttcgagt 1560
cgcgtaaaat ttttgtaagt tgtgttttgt agaatgtaaa gtcgggttgg aattcgttat 1620
tttcgtcgtt ttagtcgatt cggtcgacga gttttttttt gagtttttcg ggttatttgt 1680
cgggacggtt atttcgttcg gtgaagtcgg tgtatttatt atattttttg ttgtttttag 1740
tagggaagtc ggcggtgagg cgggggagta ggtatggtgg tttcggggtt tatgcggggt 1800
ttattgttta tttagaatag cggttattgt tgtttattag ggcgtattta tcgttggagt 1860
tttcgcggcg attgtttatt tttaatatat attttttttg ttgtgcgttt gtttttttta 1920
aatatttttt agttggtaag cgttaaagaa tataaaatag tttttaagag tatcggtaat 1980
tatataattt tagtgtttcg tttttttttt atatttttag ggagggaaag ttggaagttt 2040
agtgaatggt cgaagttttt tttgagcgta gtttttagga ttaaagtttt cggattcgag 2100
gtaagagtta gtttttttta tttttttttt tttttttttt ttttttgaaa tataagttaa 2160
tttagttgag tatagtgacg ttgggttgtt tcgatgttta taaagtattg aaatcgttta 2220
tttaattagt ttttattgta ttttgtcgtt tttttttttt tttttttttt ttattttttc 2280
gttttttttt ttttttattt taaattttta ttagattttt cgaatattta aattataata 2340
cgatttttaa tatagatttg taataatttt ttttttttta tttttttttt aatttttatt 2400
tatttttgtt tttttgagta agcggtggtt gtaaaaattt ttgtataaat tattttaaac 2460
gcggtcggtt tttaatcggg aagtaatttt agtgacgttg gcggtgtaga gaatcgagtt 2520
tggacgtata tatataaata tatcgcgggt tttcgtagtt ttgtagatat tttttttaac 2580
gttgatttcg agaaggagag ttgtttgatt tcgtttggtc ggcgttcgtt tttttgatat 2640
ttttttttta ttttttattt attattttta tttttaacga ttttggggtg aaggtagggt 2700
gaaggtgtat tgggatgggg gtcgagatta aagatgttaa gaaatcgttg cgggttttta 2760
ttttttattt agtggggtta ttagacgttt tttgttttag gtgtatataa ggtagtgttt 2820

agttttatta aaataattaa atagttattg gggattggtt agggttttta tttttattgg 2880
tttttttttt ttgttttttt tttttttgag atgtattttt tttttttagg gtgagttgaa 2940
gaggttaata aaagtataaa tatagttagt aggttttagt ttgagtagat ttagattggg 3000
atatcgaata ttggtgtttg gagtaaatgt taggagtttt gttttgtttt ttatttttat 3060
tttttgtatt tttattatat tgattttttt agggttttga taagagattt gggtttgtaa 3120
gttaaggttt ggagaaaggg atggagggag gagaaatgtt tcgaggttag tagttgtgta 3180
attttttgtg attgttttgt taaaatttta ggttaggtta ttgttgtaaa atgttaaagt 3240
ggagaatttt taattttgaa tgtggggtaa tcggggagaa agttagtttt tttttgttat 3300
ggtgtttatt ttatttttta ggtttgtttg taaatagata tttttttttt gtttaggcga 3360
tgtatttatt tgtgaaaata tttttatttt ttaagttagt ttggaggttt ttaggtttgt 3420
tggtaaggcg attttaggtc gaagatacga aaaataaata tggtatcgga agaaggttga 3480
atcgttatat ttttagtaga gggaaaggga aatttagaaa gattttttta atttttttaa 3540
gtttttagag atttggggat aaaattaatt tttataaata atttttaggt ttttttagag 3600
aggaaaaatt tggatttttt taggtaatag tttagttaga tttgtttagt ttattgagta 3660
tagatggttt gggttattaa gtaaaatagg gatttttaaa tttttcgaga gaatttattt 3720
ttgttaggtg atatacgaag ttatttttat ttcgtttatg agaagtagta gtatagtaag 3780
agaagattta gtaagattaa gacggggtga aaagttaagg agggttaagt gtaaagagaa 3840
atttattttt ttttttagtt ttttagggtg aaatttattt ttaattattt tatttttttt 3900
ttggagcgta aaagttagga gtgcgtttcg ttggagagta gggtttaggt gaagattttg 3960
aaaaggatta ttaaaagaga tttttttagg ttaggttttt ttttaacgtt tgttaagaag 4020
agtacgtatt ttttagttta aggttggttt gtggttttcg ataaattaaa tattattgtt 4080
tattattagg tatattttag taaaataaag aggtaattag gcgatagcga ttattttatt 4140
agtcgttgta tttataggat ttggagacgt tacgagttac gtaatcggtt cgcgcgttgt 4200
tacgtttggt tggggggtag cggcgggagg taaatttttt ttcgttttcg tttattagtg 4260
tcgggtttgc gtagttatag gtttcggacg ttttggtttc gggaataaat aaagatataa 4320
attaattcgg tttttttcgg aggaatgagg gtgatgaata taagatataa atagagttaa 4380
gatcgatttt tttaggaagg gggagaggag ggaatttttt acgaagggag gtgggagttt 4440
attttagatt tttattggaa ggaaatcgag ttgtttcggg ggattgaggt tttttgtata 4500
aggtatggga tttttattat tattattatt atttttaaat ttttcgcgga ggagttttgg 4560
gtaaatgaat atcgaggcgt cgttttagtt ggttaggttt gggatgcgat aatttagtgt 4620
ttttttgtag atttgtatag aaataattat tgggttgtcg tggaggggat acgagataga 4680
gggagttttt cgtaatgtgt tttgcggaga gaaaggttta agaatgtaat tcgttttaga 4740
gtggttcggt aggggcgggg tgcgagtggg tggtggagta ggggtgggag tggagagagg 4800
tggtttttgt agagaataat tattgtatta gggttcgtcg aggtacgagg tattttattt 4860
tgttttgtaa ttacgacgat tattattttt agtttgatta atgggtataa tttttaagta 4920
gcgtagtggt ggatgttcgt aaatttttgc gtatcgttgg aaatttatta ggttgagttg 4980
taaaacgtat cgcgtagacg tttttggtgg cgtcgagaga agagttaggt ttgtttagta 5040
tagagtcgga gagcgtcggg tttttcggaa gggtaagttt ttcgttaagg ggtttcgagg 5100
gagttggacg tttgaatttg gatttgtttt tagtttcggg gttcgatttt gggttttgcg 5160
cgtttttaat ttttagggtt tttcgaagta tggtttggtt ttaggttcgg ttttgtaagg 5220
attggaacgg tagtaaaatg tgtagggagg tagtcggtcg gtagagttgc ggcgggagtt 5280
aaggttaggt tcgcggggag agcgggtagt ttttagcgtc ggttataagt ttttaggtta 5340
gttgggtcgt agattttttt gtttttagag agtataattc gcgttttttt ttttagttag 5400
gttgtagtgg ttgtttcgag tttcgttttc gttttttaag ttgttaataa cgatatgttt 5460
ttaaattcga ggttcgtgtt tgtttttaga tgttaagaac gtaattcgaa attttttttt 5520
taaattttag gtcgacgaga tgagttttta tttgattttt gagtcgaggt gggtcggaaa 5580
tcgaggttta ggtttcgtcg gggttgtaag gaaaagggga aatttcgagc gtagcgtttt 5640
ttttttgtgg tttttttttt cggtatttcg gattgcgggt tttgtagtta tttggatcgg 5700
tatttaaagg attttgtaag tttagtttta tagattggtt tttttagacg tttcgaagtt 5760
cgtattacga atagaataaa ggagagacga gagatcgtaa ttagatttga gaattttcgt 5820
tttttttttt aatcgttcgg gtagtaaatt tcggagtcgg ttatagcgcg tatttttttt 5880
tttttttttc ggagaattgt cgttttttcg aagttgtttt tagttgggtt ttttttcgaa 5940
gtttattttt tttattttgt aaaatttttg ttttgatttt aatattgtaa taggatagta 6000
gaggtttttt tttaaaaagt taaaatttaa attaaagggg tttattgttg ttgataattt 6060
ttttttttat tttttttttt cgtttttaag aaagatattt ttaaaattat ttattgtttt 6120
ttttttaaaa gagaattcgt tttaaaatgt attttttttt attttttttc ggatatgttt 6180
ttatttgatt cggtggtaat ttgtggggcg tgggggtagt gaaaaataga agtgtggacg 6240
gggttgggaa aatgaataag aattataata atagaaatga tgataaaatt aaattttgat 6300
taatttaatt tgtaaaagaa agggttggtt tgagggtgag atattttttg gttttttaag 6360
ggggttattt ttttttttat cgagttaaat ttggttttgt tatatttttt ttaggaatat 6420
agtatgtttt tttttaatgt tttattcggt ttttggtttt tttatttttg atcggaagta 6480
tgtgggaaga atattggttg ttattattat gattattgag tgtttaatat ttttttttaa 6540
tatttttatt aagaattatt atcgttgttt tttttattta tgttagtgtt gttttgtgtg 6600
ttttagatga taatcgtttt ttttattttt taagtttgga gggtgagggt tttttgttgt 6660
ggtggtggtg gttgggtttt tttttgtttt tgtttttttg ttttttttgt tcggggtaat 6720
gaaatgagaa ttggaagaga ggatattatt tcgaggttta gttgagaagg attttaatgc 6780

gaaaaattta tatattttaa agagtagaaa ggtggaagcg agtggtgttt ggatataagg 6840
atgttattgt ttttattttt tttaatttgg gaagtgttta gtttagaaag gtgagggttt 6900
tatttgggag attatggagt atttttaggg ttatattgag tttaagaatt ttaattattt 6960
tgtaggttaa aatttattga taggtttttt aaaatgtttg gggtaggggg aagaaatata 7020
gggtatttta ggtttgtaat tttgtaagag agagatttaa attaatatgt ttaggtttgg 7080
ggtgaaggtg gggggtttgt ttggatattt ttttttttaa ttgaagggtt cgttggaaat 7140
ataggaattt attgtttttt gggattttgg aaagataaat tttttttggt aaacggaata 7200
gtgtcgcgtt gtcgatagag tagattattt tttttgtttt tttataaggt gggggtgggg 7260
ggaaagaaaa ggaagtaaga aagaaaggga aattaaattt atacgtgtta agttcgttta 7320
aagggttaga gttaaaatta gagatgaaat ttcgatttta gttttggttg ttcgcgggtt 7380
tttttttaat tagttaggtt ttatagagta aggtattttt attggagaga attcgatttg 7440
atgttttata aagtatttta tttaaggaga aggggtatgg gtttgttttt aagtttgtgt 7500
tgtagaaatt tagaatttgt agcggtaaag aaaataaagg agataataat tgtaattata 7560
atgatttttt ttatgaaaat ttattttgat gttttttaaa ttaaaataat aatttaggta 7620
atatttaagt aatggttaaa attatttagg ttattaaatt atttaggtta ttgttttata 7680
aaatttttaa ggtttgatta ggttttcgga tttgggttta gttgggttaa gagaatttga 7740
aatgttgttg ttaagagatt ttgaaattat tagtttaatt aggatagtta atttttatat 7800
ttttgaggtt ttttttttgg ggagagattt ttttgagaga ggatattata ggatttagtt 7860
atagagtttt gttatatatg ggtgggagtg gaggtcgtta ttttatttgg aattgtgagt 7920
gaaaatttta gtaatgattt tgaggaggtg aagggaggta ggtgggggtg tgggtgatga 7980
gggaggttaa attttaattt ggaaggattt gttattgaaa taattgtttg gtcgagattt 8040
ttaatttttt ttttttttta gttagttttt tgtggttttt tggtgttggg tttttggttt 8100
ttggtgatat tttaatagaa gggtttggaa gtagggattt tagatatttt gttttttttt 8160
ttaaattata tttaaaatat attggggtga aataattgat agagggtttt tgtggtgagt 8220
aattgattga agtttttagt ggaaaagttt ttgacgattg ttttcgtagg gattttgaat 8280
tttttggtta ttatggcgat ttggaagttt tatcgaatgt ttttaataat ggggtatttc 8340
gaggatattc gtgtaattgg ttgcgtagtt tgaggtttga aaagcgtttt tgttaatcgt 8400
ttttgatttt ttggagtttt tttagtgggt ttaacggagt agggattttt aaaattgttt 8460
ttttttttaa ataggggaga aaattgagat ttagtattag gtgggtttag gttttgagta 8520
aaagttttat ttttgaattt agaatgttga ttttagggtc gatcgtgtag agtttatggt 8580
gtattttaat cgaaattcgt ggtttaaata ttttagttag gaatttggga ttttaggtgt 8640
tatttaattt ttttatattt gttaattata tttatttgta taggaggttt tttatattag 8700
tttaggtaat tttttttttt gtttggggtt atttttttta aatggaagga gaaaagtttg 8760
agtattagaa attgtggtat taatgtttgt aagggttaat ggagtgaaaa gaatatatta 8820
tttttttttt ttttttagaa aaaaatttta attgtggatg taatagaaat attattaaaa 8880
taatattttt gattgtattg tttttttttt gaatgtttat tagaggggag ggggaagtgg 8940
atttttttat tttggggtgg atagtggata atataggttg atttttttgt gttcggttcg 9000
gaggtattgc gtttgtaggg tttggtagcg atgtaggtag taaggagaga aagggtgata 9060
gagagaaggc gtttaggttt taggtgtcga tagaggtttt tggcggtggg acgggagtta 9120
tttttttttt gggttttttt ttagttttgt tttattttgt tgttaatttt attttttttt 9180
tgattgttta ggaaagataa aatttaattt tttttttttt gtttttaata ggatatttaa 9240
tgttttttaa agttgttggt tggttttgta gtgtttttat ttattttttt tttttttgag 9300
agtttaggta gtttatagag gttttcgttt ttaatttaac gttttttgtt tttttttttt 9360
tttagtattt tgtattaata ttttagaaga gttttattta tgtttatata gttattaaga 9420
gagtaatgtg agtattttag gtttataaat ttgttagtat ttgaggtgta ataatagtta 9480
atatttatgg taggttagga aagagtt 9507

<210> 261
<211> 9515
<212> DNA
<213> Artificial Sequence

<220>
<223> chemically treated genomic DNA (Homo sapiens)

<400> 261

agtattttta ttttttaaga tttttttttt ttaaagtaat aatatggaaa atatttataa 60
tgatgaaagt atttgttttt ttgtattagt tatggtagag gagatatata ttttttatat 120
tttgaatgtg aaatgtttat tggtgttgaa aataattgtt gtatggttgt ttgtattatt 180
tgttttagtt ttttattttt ttttttttaa tttttttatt tttaagagat aggaatttta 240
aaattgtttt tttagttatt ttatattttc gtagttaaat agaaaagttt aaggggatta 300
ggatatttaa aatttataat gaaattatta ttgtttttta aaagttttag ggaaagggtg 360
agtttttata gttatagtta tttttatttt tataagtgat tttaatttag tattttttat 420
aaatttataa atgtattatt aattaaatat tatgattaaa gtaagaaatg tttttttttg 480
tttagtgttt gttttaattt cgataattat gaagagttag tttaaagata attttattta 540

tatttatttt tttaaaaata tttttgtgta aatattagtt ttttttattt tgttttttta 600
tattaagtta ttgaaattta tatggtatta aagttttaga aatttgaagg taggtgttta 660
aattgtagtt ttagatattt aatagtattt tgtttttaaa ttgatgataa aaatatttat 720
tgaatttttt ttaaaaatat aattatgaga aagtttaaga tagaaatatg gtaggaaaat 780
taggagttag attgtaatag taaaaaatgg agttgttatg ttttattatt ttttaattta 840
ttaagatttg ttatatggat gtcgtgtttt attatttaat ttagtagata ataatgtttt 900
tgatgggtaa gaggtggggt aatatttgag gatgttgaag taagatagtt tttttttttt 960
tttttttttt ttttgtttat ttataaattt aggattataa aaaataattt tggatatatt 1020
taatgattta aaaatattgt ttttttaatt ttagaaggaa agaagtaaga tagtattatg 1080
agattgtggg taaagcgttg agtatatagt ttgaagattt atattgggtt ttttattttt 1140
attttttaat taggagttta ttgtaaattt ttggttttta gaagttttta tttttttgat 1200
ttttcgttaa gtgttttgag ataatgatga taagttaaat tgtttatgaa gtaatagtat 1260
tatatatatt tatttataga tattatttta ataaattttt ataattattt ttattttgta 1320
gtttagtgta ttgaatttta ggttattaaa tagtttgttt tatcgtttag aatgagaaag 1380
gttagggtgt aaatttggta taataatttt aatttttgat ttttttaatt ttattatttt 1440
tgtgcgttgg gtagatggaa aggggaaatt tagggagatt gtgtaatgga aaaagaaaag 1500
gtggtatagt gggtatttgt aaaggaagat agtttatgga gtgttaattt ataggtgtaa 1560
aggtgagggt tagtggaagg aaggaagtta aataaattta gataatttat aattagattt 1620
ttagaagaaa gatgaaagaa gatatgaaag tgtttttagt tgattttgat ttaataatga 1680
tatttttttg tatatttttt aaaaaatagt tttgaatata tttcgaaaat tattagtaga 1740
gggttttatg ttgggtgtat ttgtttttaa ttatagattt cgatatttat tgaataatat 1800
tttttttata tgaggttgaa aaaaaaagag ggtatgaata attttttttt ttgttgtttt 1860
aaattgaaat aaatgtaaat gaaagaggaa agtacgagtg ttaattattt gggatgtttt 1920
aagtgcggtt ttatttagga gagtatgtgc gtaatttgaa aaagaaatat ggatataagg 1980
taagataaaa taacgaggaa aatgtatatt ttattcgttt aatttttttt aaatttagag 2040
tttgttttgt ttttgtgttt ttatttattg agtttttgcg ggaatagttt ttttacgtta 2100
tgtgattgag tgataacgcg aaaggtaata gaaagtaaaa gtaagagagg taggtttaag 2160
ttaggaaaaa tttttttcgt agcggttgag tttttattgt gcgttttttc gggcgtttga 2220
attttttaga tgttggaacg atcgttttta aaattagtaa atattataag aagtgaattt 2280
taaaaatgtt ttttttaata ttaggttttt aagaacggaa agaaattttt gtcgaaaaag 2340
aaattaagga gatttgtagg gtttgcgttt ataaaatttc gatagagggc gttgttgttc 2400
gtttaatgat agggaacggt tcgtgggaac ggtttatttt gtaatgttta ggatttttcg 2460
aataacgttt acgttaaaaa aaataattag gattttaatt atggttatgt attttgaagg 2520
tattttttta tatatatgtg tatattttta ggtttatgtt ttatttttaa taaaattaga 2580
tttgttattt tttaggtttt ttaaagaatt taaattttaa ttaaatattt taagatattt 2640
ttatttttaaa aatatttaa tattagtata aataaaaatt atatataaaa agtttgaaat 2700
tatttatata taaggttata aaatagttat attaatataa tattttatat tattttaatt 2760
tatgtgtaaa attatttttt gtaggttttg ttttgttaaa ttttagagtt tatttatttt 2820
agattaggta ataatatatt aaaaagatat taaaaggtgt taaaagtatt tagagaaaaa 2880
aagtgagatt tttttaaatg tattttattt gggttgtaat gatttttagg taataataat 2940
gatagaatta ttgatttgaa agggagaaaa taattatttt tattaattaa atgttttgtg 3000
tttggagaat ttgaattttt aagtttataa tatttatttt ttagattatt gatttggtat 3060
tgattttagt tttagaagtt agggggtgtg gaattcgttt taagttttat tatataataa 3120
gattaagaaa aatttttttt tattttttaa ttagttttgg aagtaaagaa gaaaattttt 3180
gttgatgttt ttagtgaaaa atttgtatag attgtgtatg atttaaggtt aatatttaaa 3240
ggggaagaaa ggaatggaga gagaaaggaa ggaacgaaaa gggaaatgaa ggaaagaaaa 3300
aaggaaaata aaaaggagga agaggagaaa aggaaagacg aaatataaaa aatttaatcg 3360
gaaataaggg gtttattttt gaagtttttt ttgaagtatt ttttttgtgt tttttttttt 3420
tgatttattt ttaaaaatta aattaaaaat ttgtagaatt gtttattttg tagtttatga 3480
agtttttttt tggttttgat tgaatcgacg tttaataagg ttgttcggag ttttgtattt 3540
aaatagcgtt tatttttgtt cgcgtagagt tgtatatagt agttttggat ttaaagataa 3600
ttattttttg tagatttatt tttattaggt ttaataattt ttattttaaa ttgttttatt 3660
ttttttcggt tttttttggt attttttttt tgttttaggc gtagagattt ttttttgggc 3720
gtttgtttcg gttatgagaa gtttttcgta ttttgaaagg gagtagaaaa atagagggga 3780
tgggaaaggg ggaatagggg aatagatgag tatttatttt atgataaaat ttagaatttt 3840
tgcgttttaa atggtgttta ttttttaagt ttggttaata ttgtttttgt tgttgatgat 3900
gttttttttt ttaaaaatta tatgaattat tttattaaga aaaattatat aaaaaaaaga 3960
agaaatttta tatgtattaa aatatttttt attatttttt tgaagatatt ttgtttaaat 4020
ttttttaaag attcgggtgg tttttttttg attttttatg attaaaatta tttataagtt 4080
atatataata gatgtaaata taaaatgttt ttggtttttg taaaaaaggt ttaaaattaa 4140
ttttgtttga acgtgtgcgt acgttgtttt taatatttga aaaaaatttg ataaaatgga 4200
gattttagtt aagaggaatg gtgtatggaa ttcgttgaaa atgtatataa ataatattta 4260
aaataataag tagtataaat tgaggtgtat ttaagttttt aaaggtttta ggtaaaatga 4320
ttagaaaata ttagaaatgg ttattaacgg tggttagttt ttttgtttta tggtgataga 4380
gataaagtta aaaaaaaaaa agttgtgtag ttattaatat agagatagtt tttaaaattt 4440
tttatattta gttttttaaa atattttttt tcggaatata tatgaatagg agggagtttt 4500

tgtgtttaaa aattttggta agtgtttttt ttttaaattt atatagaaaa taggggattg 4560
atggttttg gtttttattt atgaattgta aagatttag ttagtggtta taatgatgtg 4620
ttattttcga tatagaagtt ataaaatgta tatattatat taattatttt gaataatttt 4680
ttgttaaatg tatatttgga gtttgtagat tgtgtgattt agaggtgttt atataattat 4740
atgtgggtgt ttgggtgtaa atacgggttt atgattttta tagtatatat gtttgtgggt 4800
tgtatttaat ttttgtgtgt gtaataatgt tttttttttt tgttgaggtt tatttgttta 4860
gtttattgtt tgagataata gatataagtt gcgatgggag aggagcgtcg gatttggtgt 4920
gtatttttta tttttaatta tagatggatt attatagata gcggagtttt gagaagttag 4980
atatttgttt tttatatgaa tgtatttatt ttttagagat taggttgtta ttatgttttg 5040
gaagtttgtg gagaatgtta agtacgaaga tatttatgag gtgagtcgat atttttagat 5100
gtattttaga gttttgtaga tagagaattt gagttttttg atattttaaa tgatatatat 5160
ttttaagtta tttattcgta gattttcggt cggttttttt agtttttttt aatttttaga 5220
aaagttggtt agggaagatt tcgttgtttt gaaaagggat tttaaggttg aaatttgata 5280
attttaattt aggaatgttt ttatttattt tatttttttt tttttttttt tcgttttttt 5340
ttattttatt ttattttttt aagttttgat ggggaatgtg gtgttattga atgtgggtac 5400
gtttttagag aaaatcgaga ttttgataaa gttaggggtt tttttcgttt taagtgtgga 5460
agagaattgg gtttttaagt tttatttgta atagattcgg gtttttaatt tttattttta 5520
ataaaatttt tgaaattgat aataaatggt tttaagtaga ggatagagaa ttagcgggaa 5580
agaagtagat ggttggagat atggatggat agagtaaaat agaagtcgta gtagtttatt 5640
agtttttgtt tttttgtgtt ttgttttttt gagggttatt gcgttcgaat agaaatcgta 5700
gtcgtttttt gtgtgggtat tttttacgga aatttttgtt tacgtttaat gcgtacgggt 5760
ttttagtatt gaggttagtt taatttttcg gggtaggaga agttgcgttt agttaggagg 5820
acgttagtcg ttttcggggt tcggatcgcg ttcgttaaag ttttttttcg gttcgggcga 5880
gggttgacgc ggttagacgg tttcgttatt cgcgggcgag gcggattagg cggacgaggc 5940
ggtcgaggcg ggcgaggtgg gagaggcggg cgaggtggga gaggcggtcg aggcgggcga 6000
ggcgcggggc gcgcggcggg ttaggttggg gatcgggaag ttcgcggagt ttcgttttta 6060
agcggaatgg tttgggatag ttttaggtgg ttgttagggg aagggaggaa gggaaggttt 6120
tgttgagacg tcgaggttgg aaataaaaat ttcggaaagg cgaagcggat ttagaaaagg 6180
agggaagtcg aggcgggcga agcgggttat ggagattgtt aagaggaaaa gttggagaga 6240
aaaggaagga agagaagtag attttcgggt ggcgggcgcg ggtcgttagg ttagacgaaa 6300
ggtggaggga tgtagaggtt ttttaaagag tcgagaaaga tacgcgattt tttcggatcg 6360
ggatagtttt tgaaagttcg gcgtagagtc gtttcgaaga ttttaagagt gggcgattta 6420
taggcgcggt cggtaagttt ttgggggatt cgggttcgga cgagcgttta taggtagcgt 6480
ttggcgttta tattttttgt tttttttcga attttaatgt tggggtgtgt gtatgtgtgg 6540
aggtatgtag gagagaggag gaggagataa gagaggagag ggagaaaagt aggagagagc 6600
gcgagagagt agagaggaga gagattggtt ttgttgttga atgatgtttg gggttttttt 6660
tttttttttg tggttgtgta tcgattgtaa cgaagagtcg cgtttacgcg tttggtgggg 6720
attgtttcgg ttcggttttt tttttttttt ttaataggcg ggataatggt aaagtttaag 6780
gcgttagaat tgttttaaag aatattagcg tttaaagtgg gtatgatatt agtaagtacg 6840
gggttatggt gaaaatagtt taagttatat gtatgtagag gtattttagt gggttttaaa 6900
aagtaattag ggtaatattc gttttttata gaaagtaaag tagttaaatg gaaaaggaag 6960
gagggagaga agaaaggagg gagggagaga ggaagaggga ggggtggagt ggaagggagg 7020
gaagagagaa gagaaaagag gggagggaga gagagaaaga gaaataaata gggagagaga 7080
gggaatatta taggtggttt tatttgttta aattttttgt ttaaaaaata gaattcgtag 7140
ttcggtggaa ggagtttatt taagtttgga ttaaggttga attttaatag aaatggtagt 7200
taggttagtt gaacgggttt ttgaaatatt taaaaaggtt ttttgttgtt tttggttttg 7260
tttggaaagg ataagtttag tgtatttgaa gattgatggc gtatgtttta aattgaggta 7320
aaaatcgtaa attatgtgag tattaaatag gaaaaggttg tataggaatg aaaagttttt 7380
atttcgtttt tgttttttaa taaatgagta taatttttta tatttagtaa aagtttgtaa 7440
aagttattgt aaataattag gtttattaat ttgttttttt ttcgttttta aatgatgaag 7500
taattttttt tattttgttt ttgtaatttt tatataaagt atttaaggcg attaagtttt 7560
tcggattagg gaagtttttt tttaggtagg aggatatttt tattttatat aattttttag 7620
tattgttttt ttttattgtt ttatttaaag gagtaggggt tgttttattc gtgttttcgg 7680
tatagttaat tgaagttttg tttttttttt gttaaatttt ttttttagtt tgtttttgtt 7740
agttttttcg tttgattata tttagtaatt ttttattgaa tgaacgttat ttataatagt 7800
aggggagttt tgcgttcgtt gttgttttac gttttatttg tttttttattt tttttttaaa 7860
gtattcgtgt aatattaata gttatgaata ttaattatta tgaggcggtg taggtaagcg 7920
gagggaggaa ggggcggtcg agtagtttga gtttagtttc gggtgaaaga ggattgtatt 7980
tggagtttcg aagaacggta gaagaaaaga ggttaaaaag atagtaaaga gatagatgaa 8040
tagttttgtt tgtgtgtgat aggtatcgtt aattttgttg gggtgggggg cgggtgtaat 8100
tttttttttt tttttttgat ggtttgtttt ggaaatatat taagtttagt ttttgtgttt 8160
agttcgtttt tttgttggat tttttgattt tttttttaat tattgtttga ttttgagtag 8220
taattaggtt ttttttttta gatgttagtt tatatttatt tatttatggt gagtttggat 8280
ttacgtgtta ttagaattgt atgttttttt tttttattgt ttttttgttt ttgcgttttt 8340
gtgtgtgtgt gtgttttata ttttttgttt ttttttgttt ttttttgagc gtttttggtt 8400
tggtaattta gtattataat tggacgaggt tgtagttgtt tttttttgta ttgtgtgttt 8460

ttcgtttgag ttttatgagt tagttgggag gggagaggtc gaaaagaaaa attttttatt 8520
tgttattgtt gtttggaaat ttaggtgatt atttttgtaa aaagaattat ggggagttta 8580
ttatgatgtt tttagagaag agagggagaa ataaagttta ttttttttat atatatattt 8640
tttttttttt ttttatttta gtttatattt tagataggat taggaataag ttgtttgggt 8700
ttttattttt ttttatttta attttagttt ttttattttt atattttgag tataatagta 8760
ttagatatag aaaaatagtt ttagaaataa tttttatgaa ttataattta atttaattta 8820
ttattggttt aggatatgat ttcgaatgag atttatttta atgaaatatt atttaagtta 8880
tttaattttt tttggagagg gaggagtgat tgggaattta gattaggtgg attttttgga 8940
gtttttggaa tgattaggtt gtgtggaaag ttttttttaa ttagttagtt gtatttttag 9000
cgaaggtaga agttggtttt taattagggg gagtagtata gtaaatatta tgaagtttta 9060
tttgttgtga tttgtatttt tggaagagtt agaaaaaagt agttaaaaat atttagagga 9120
gtaaatatta tatgtaagat ttttataatt ttaattaagg tatttagaat ttttttttat 9180
atatattgtt tttgtatttt tgttttttgt ttgttttgta ttttttaaaa agcggttaaa 9240
atgttatagt taagtgggtg attttttttt tatttttttt ttaagtttgg ggtttgaaat 9300
tgttgaaatt atgtggtttt ttgagttaat ttgggggttt aagttttaga tttttgtttt 9360
ttttgtttcg ggaagagcgt ataaaagtcg ggttttttta tttgttattt gtattttttt 9420
tttttttttt tgtatttttt gttttttttt gttttatttt tttttttttt gttttttttt 9480
ttggtttttt tttttttttt tttttcgttt ttagg 9515

<210> 262
<211> 9515
<212> DNA
<213> Artificial Sequence

<220>
<223> chemically treated genomic DNA (Homo sapiens)

<400> 262

tttgggagcg gagagaggaa ggggaagaga gttagagaag gagatagaga aagagagata 60
gaggtagaga gagatagaga gtatagagag gaagaaaaaa aatataagtg ataaatggag 120
aagttcggtt tttgtacgtt tttttcggga taagggaagt aaggatttga aatttagatt 180
tttaagttga tttagagggt tatatgattt tagtaatttt aaattttagg tttggggaga 240
aaatgggaaa ggaattattt atttaattat aatattttag tcgttttttg gggagtgtaa 300
agtaggtaaa aggtaggggt ataggaatag tgtgtatgga gggagatttt aaatgtttta 360
attaaaattg taggagtttt atatatggtg tttgtttttt tgggtgtttt taattatttt 420
tttttaattt ttttagaggt ataaattata ataaataaaa ttttatagta tttgttgtgt 480
tgtttttttt gattaaaaat taatttttgt tttcgttgga aatgtagttg gttgattagg 540
aggagttttt tatataattt gattatttta agagttttaa ggaatttatt tggtttgagt 600
ttttagttat tttttttttt ttaggagaaa ttaaataatt taaatagtgt tttattgaaa 660
tggattttat tcgaaattat attttaagtt aatgatgaat taagttgagt tgtggtttat 720
aggagttatt tttaagattg tttttttata tttaatgttg ttatatttag gatgtgaaaa 780
tgaaggaatt aaagttgggg tggggggaaa taaaagttta gataatttat ttttggtttt 840
atttggagtg tgaattaagt gaaagaagaa gggggaggtg tgtatatgag ggaggtggat 900
tttgtttttt tttttttttt ttggaaatat tatggtaaat tttttatggt tttttttgta 960
gaaatagtta tttaaatttt taaatagtaa tggtaaataa aaagtttttt ttttcggttt 1020
tttttttttt agttgattta tggagtttag acggagaata tataatgtag agagaagtag 1080
ttgtagtttc gtttaattat ggtgttaaat tattaagtta aaggcgttta aaggaagata 1140
gagagagata gagagtgtga gatatatata tatatagaga cgtagagata gagagatagt 1200
gagaggaggg agtatgtaat tttggtaata cgtgaattta aatttattat ggatgaatag 1260
gtgtggatta atatttggaa aaaaaagttt ggttattgtt taaaattaaa taatgattaa 1320
aaaaaaaatt aaggagttta gtagagaagc gagttggata taggagttga gtttgatgtg 1380
tttttaggat aagttattaa aaaaaaaaaa aaaaaattat attcgttttt tattttaata 1440
agattggcga tgtttgttat atatagatag aattatttat ttgttttttt gttgtttttt 1500
tggttttttt ttttttgtcg tttttcgggg ttttagatgt agtttttttt tattcgaagt 1560
tgggtttaga ttgttcggtc gttttttttt tttttcgttt gtttatatcg ttttataata 1620
attgatattt atgattatta atattatacg agtattttaa agggagaata gaggataaat 1680
ggagcgtgaa gtagtagcga gcgtagagtt tttttattat tgtaaatgac gtttatttag 1740
tggagaatta ttagatgtaa ttagacgagg gggttggtaa aggtagattg ggaaaaaagt 1800
ttagtaggaa agaggtagaa ttttaattaa ttgtgtcggg gatacgagta aagtagtttt 1860
tgtttttttg gataaggtag tgagggagaa tagtgttggg aggttgtata gaatggaggt 1920
gtttttttat ttggagagag atttttttaa ttcggaggat ttggtcgttt tgggtgtttt 1980
gtgtgaggat tataaaaata aaataaaaag ggttgtttta ttatttggag gcgagagaaa 2040
ggtaaattgg tgggtttggt tatttatagt gatttttgta gatttttgtt aaatgtggaa 2100
gattatgttt atttgttaaa ggatagaagc gagatggagg ttttttattt ttgtgtagtt 2160
tttttttgtt tagtgtttat atagtttgcg atttttattt taatttaaaa tatacgttat 2220

taatttttaa atatattaaa tttatttttt ttaggtaaag ttaggggtaa tagaaaattt 2280
ttttggatgt tttaggaatt cgtttaattg gtttgattgt tatttttgtt aaagtttaat 2340
tttagtttag atttaaatag gtttttttta tcgagttgcg gattttgttt tttgagtaga 2400
aaatttaggt agataggatt atttgtaata tttttttttt ttttttgttt attttttttt 2460
tttttttttt tttttttttt tttttttttt tttttttttt tttttatttt attttttttt 2520
tttttttttt tttttttttt tttttttttt tttttttttt ttttttattt agttatttta 2580
ttttttgtaa agaacgaatg ttattttggt tattttttag aatttattga aatgtttttg 2640
tatgtatata gtttgagttg tttttattat ggtttcgtgt ttgttaatgt tatgtttatt 2700
ttgggcgtta atatttttta ggatagtttt agcgttttgg attttattat tatttcgttt 2760
gttaaggaaa gaaaaggagg tcgaatcgaa ataattttta ttaggcgcgt aggcgcggtt 2820
tttcgttata gtcgatatat aattataaag agaaaaggaa aattttaagt attatttagt 2880
aataagatta attttttttt tttttgtttt ttcgcgtttt tttttgtttt tttttttttt 2940
tttttttatt tttttttttt tttttttata tatttttata tatatatata ttttaatatt 3000
aggattcggg ggggggtagg aggtgtgggc gttaggcgtt gtttgtgggc gttcgttcga 3060
gttcgagttt tttaggagtt tgtcggtcgc gtttgtgagt cgtttatttt taggattttc 3120
gaggcggttt tgcgtcgggt ttttaggagt tgtttcgatt cggaggggtc gcgtgttttt 3180
ttcggttttt tggggagttt ttgtattttt ttatttttcg tttgatttag cggttcgcgt 3240
tcgttattcg ggagtttgtt tttttttttt tttttttttt ttaatttttt tttttagtaa 3300
tttttatggt tcgtttcgtt cgtttcgatt tttttttttt tttaaattcg tttcgttttt 3360
tcgaagtttt tatttttaat ttcgacgttt tagtagaatt tttttttttt tttttttttt 3420
ggtaattatt tggggttgtt ttagattatt tcgtttaaaa gcgaggtttc gcgggttttt 3480
cggtttttag tttggttcgt cgcgcgtttc gcgtttcgtt cgtttcggtc gtttttttta 3540
tttcgttcgt tttttttatt tcgttcgttt cggtcgtttc gttcgtttag ttcgtttcgt 3600
tcgcgagtag cggagtcgtt tggtcgcgtt agttttcgtt cgagtcggaa aagggttttg 3660
gcgagcgcgg ttcgggtttc ggaggcggtt ggcgtttttt tggttgggcg taattttttt 3720
tgtttcgaag agttgagtta gttttagtgt tagagattcg tacgtattga acgtgggtag 3780
aaattttcgt gaaagatgtt tatataggag gcggttgcga tttttgttcg gacgtagtga 3840
tttttaagga agtaaaatat aagaaaatag aaattaataa attgttacga tttttatttt 3900
gttttattta tttatatttt tagttattta tttttttttc gttaattttt tgttttttat 3960
ttggaattat ttgttattaa ttttagaaat tttattgaaa ataaaaatta aaagttcgag 4020
tttgttataa atgaggtttg aaaatttaat ttttttttat atttaaaacg agaaaagttt 4080
ttgattttat tagaatttcg attttttttg agagcgtgtt tatatttagt agtattatat 4140
tttttattag agtttaaaag ggtggggtgg ggtgggggga ggcgaggaaa agggaaagaa 4200
aaataaaata aataaaaata tttttaaatt aaagttgtta gattttagtt ttaaagtttt 4260
tttttagagt agcgaagttt tttttagtta gtttttttaa aagttaaaaa aagttgagaa 4320
aatcgatcgg aaatttacga gtaaatagtt taaaaatata tattatttgg ggtattaaga 4380
agtttaaatt ttttatttgt aaagttttaa ggtgtatttg ggggtgtcga tttattttat 4440
agatattttc gtatttgata ttttttataa gtttttagag tatgatggta gtttggtttt 4500
taggaggtga gtgtatttat gtgaggagta gatgtttggt tttttaggat ttcgttgttt 4560
gtaatgattt atttataatt ggaaatgggg gatatatatt aaattcgacg tttttttttt 4620
atcgtaattt atatttgttg ttttaaataa taggttggat aagtaagttt tagtaaagag 4680
aaaaaatatt attgtatata taggagttag atgtaattta tagatatata tattataaaa 4740
attataaatt cgtatttata tttagatatt tatatatagt tatataagta tttttgggtt 4800
atatagtttg tagattttaa atgtgtattt aataaagagt tgtttagggt gattaatata 4860
atatatatat tttataattt ttatatcgaa aatgatatat tattgtagtt attaattgaa 4920
gtttttgtaa tttataatga gaaattaggg gttattagtt ttttattttt tatgtagatt 4980
ttaaaggggg atatttatta agatttttaa atataaaagt ttttttttat ttatgtatgt 5040
ttcggaagag agtattttga aaggttggat atgggggatt ttaaggatta tttttgtgtt 5100
gatggttata taattttttt ttttttaatt ttatttttgt tattatagaa taaaaaagtt 5160
agttatcgtt gatgattatt tttaatattt tttggttatt ttatttaaaa tttttggaaa 5220
tttaaatata ttttagtttg tgttgtttgt tattttaggt gttgtttgta tgtattttta 5280
acggatttta tgtattattt tttttggttg gagtttttat tttattaagt tttttttaga 5340
tgttggaagt aacgtacgta tacgtttaga taaggttaat tttgagtttt ttttgtagag 5400
gttaaggata ttttatattt gtatttgttg tgtatgattt gtaagtggtt ttaattatgg 5460
agagttaaga aagggttatt cgaatttttg aaaaggttta agtagaatgt ttttaaagag 5520
atagtgaaaa atattttagt gtatgtggag tttttttttt tttttgtata atttttttta 5580
ataagatgat ttatatagtt tttaaaaagg aaaatattat tagtaataag aataatatta 5640
attagattta gaaaatgggt attatttaag gcgtagggat tttgaatttt attatgaaat 5700
gaatatttat ttgttttttt gttttttttt ttttattttt tttgtttttt tatttttttt 5760
tagggtgcgg agagtttttt atagtcggaa tagacgttta gaagaggatt tttgcgtttg 5820
ggatagaaaa gaaatgttag gagggatcga ggagggatgg ggtaatttgg ggtggaagtt 5880
attaagtttg gtgagagtga atttgtaaag ggtgattgtt tttaaattta ggattgttgt 5940
gtgtagtttt gcgcgaatag ggatggacgt tgtttgagta tagggtttcg gataattttg 6000
ttggacgtcg atttaattaa aattaaggag aagttttatg aattgtagaa taaataattt 6060
tgtagatttt taatttaatt tttaaagata agttaaagga aaagaatata aagaaggtgt 6120
tttagaagga attttagaaa taaatttttt attttcggtt gaatttttta tgtttcgttt 6180

tttttttttt tttttttttt ttttttgttt tttttttttt ttttttttat tttttttttc 6240
gttttttttt tttttttttt attttttttt ttttttttaa atgttaattt taaattatat 6300
ataatttata tagatttttt attaggagta ttagtaaaga tttttttttt tgtttttaga 6360
gttagttagg agatgagaag ggattttttt taattttatt gtgtggtggg atttgaaacg 6420
ggttttatat tttttgattt ttagaattaa aattagtgtt agattaataa tttgaaagat 6480
aaatattata gatttggaag tttagatttt ttaggtataa gatatttaat taatggaaat 6540
aattgttttt ttttttttaa attaataatt ttgttattgt tattatttaa agattattat 6600
aatttaaata aaatgtattt ggaaaaattt tatttttttt ttttaaatgt ttttaatatt 6660
ttttaatatt tttttggtat attattgttt agtttaaaat aaataaattt tgagatttaa 6720
taaaataaaa tttgtaagag ataattttat atataaatta aaataatata aggtattgtg 6780
ttagtataat tattttatag ttttatatat gaataatttt aaatttttta tgtgtaattt 6840
tattttatgt tagtattagg tattttttaa agtggaaata ttttgagata tttggttaaa 6900
atttaagttt tttaggaaat ttgaaaaata ataaatttgg ttttattaag aatgaagtat 6960
aaatttaaaa atatatatat atatatagag aaatattttt aaaatatata attataatta 7020
aaattttagt tatttttttt aacgtgggcg ttattcgaaa agttttagat attgtaaggt 7080
aaatcgtttt tacggatcgt tttttgttat tgaacgggta atagcgtttt ttgtcggaat 7140
tttatagacg taaattttgt aagttttttt gatttttttt tcggtaaagg ttttttttcg 7200
tttttgaaaa tttggtattg agaggaatat ttttaaagtt tattttttat gatatttatt 7260
aattttagaa acgatcgttt tagtatttaa aaagtttaaa cgttcgaaga aacgtataat 7320
aaaagtttag tcgttacgga gaaaattttt tttaatttga atttattttt tttgttttta 7380
ttttttatta tttttcgcgt tattatttaa ttatatgacg tagggaaatt attttcgtaa 7440
gaatttagta aatggaaata taaaggtaaa ataaattttg gatttgaagg aaattaaacg 7500
agtaaaatat gtattttttt cgttatttta ttttgtttta tgtttatatt ttttttttag 7560
gttacgtata tgttttttta ggtggaatcg tatttgagat attttagata gttaatattc 7620
gtattttttt tttttatttg tatttatttt agtttaaaat agtaaagaaa agaattattt 7680
atattttttt ttttttttag ttttatatag agggaatatt atttagtaaa tgtcggagtt 7740
tataattaaa agtaaatgta tttagtatga agttttttgt tggtgatttt cgaagtatgt 7800
ttaggattgt tttttaaaaa atgtatagaa aaatattatt attaaattaa ggttaattag 7860
gagtattttt atgttttttt ttattttttt tttaaaaatt taattatgaa ttatttaaat 7920
ttatttgatt tttttttttt tattagtttt tatttttgta tttgtgggtt aatattttat 7980
gaattatttt tttttgtaga tgtttattgt attatttttt ttttttttat tatatagttt 8040
ttttgggttt ttttttttta tttatttaac gtataaaaat aataaaatta gaaaagttaa 8100
gaattagaat tattgtgtta agtttgtatt ttggtttttt ttattttagg cggtggggta 8160
ggttatttaa tagtttgagg tttagtgtat tgaattataa aatggaaatg attgtgaaaa 8220
tttattaaaa tagtatttgt gggtaaatat atgtggtatt attgttttat aaataattta 8280
atttattatt attgttttaa aatatttagc ggggggttag gaaagtggag gtttttaaaa 8340
attagggatt tgtaataaat ttttggttag aaagtggagg tgggaaattt aatataggtt 8400
tttagattgt gtatttaacg ttttgtttat agttttatga tgttgtttta tttttttttt 8460
tttgaagtta agaagatagt atttttggat tattgagtat gtttagggtt attttttata 8520
gttttggatt tataagtagg taaaggaaag aagagaggaa ggaaaaattg ttttatttta 8580
gtatttttag atattgtttt attttttatt tattagaagt attattgttt gttgagttga 8640
ataataagat acgatattta tataatagat tttgataagt taaaaaatga taaaatatga 8700
tagttttatt ttttattgtt gtagtttggt ttttaatttt tttattatgt ttttgtttta 8760
agttttttta tgattatatt tttggggaag atttagtagg tgtttttatt attaatttgg 8820
aaataaaata ttgttgaatg tttgaaatta taatttaagt atttgttttt agatttttag 8880
ggttttgata ttatataggt tttagtgatt tggtatgagg aaataaaata ggagggatta 8940
atgtttgtat aggagtgttt ttaaaaaatg aaatgtgggt ggggttattt ttagattgat 9000
tttttataat tatcgagatt gaggtaagta ttaaatagga aagagtattt tttattttaa 9060
ttataatatt tagttgatgg tatatttgta aatttgtaaa agatattgag ttgaaattat 9120
ttgtgagaat aaaaataatt gtaattgtga aaatttattt tttttttggg atttttgagg 9180
agtaatagta attttattat agattttaga tgttttgatt tttttaagtt tttttgttta 9240
attacggggg tatgaagtgg ttaaggggat agttttgaaa tttttatttt ttggaggtaa 9300
agaggttaag gaaaggaaga tgaggggttg aagtaaatga tgtaaatagt tatgtaataa 9360
ttgtttttaa tattaatggg tattttatat ttaaaatgtg agaggtgtgt gttttttttg 9420
ttatgattaa tgtaggagga taggtatttt tattattgtg gatgtttttt atattattgt 9480
tttaagaagg agaaattttg aagggtgaga atatt 9515

<210> 263
<211> 6609
<212> DNA
<213> Artificial Sequence

<220>
<223> chemically treated genomic DNA (Homo sapiens)

<400> 263

taataaaaat ggtataaatt aaatagatat ttttaaatga tatttattaa ttgtgtgtta 60
tttttgttgt tttgttagtt tttttttttt ttgagttttt ttttaaataa agttatttgg 120
gaaattgtgt ttttttgtgg tgattggtaa aaataatatg ttttttgtag aaagaattaa 180
attaaataat agtagtagga tataaggttt taggaaaatt tatttgtgaa aaacggaaat 240
tttttttagt gaagaaaggt gaaagttatg agtaattaga aagagaaatt gaattgaaaa 300
aatatagaat gtaaattatt ttatttatat ttaaattatt ggttatcgga tgattaaaaa 360
gaagatatat aatgttatgt agaaagattg agattatgat aatagttttt atgaataatt 420
atttattttt tgtatttttt tattagttat tttagtaatt ttttgatata agtgaagtga 480
gtgtgttttt taataaggtt aatgggtagt taagatatta attttttagt ttgtataatt 540
tttgaaaaaa aaaatgaaaa aaaaaaatgt tttttaagat tttatatttt tttatatttt 600
gattttgtat tttttatagg ttagatttat gtttttttat tttattttta agttgagttt 660
agaatgttag ttattttaaa gattaatatt tatttgaaaa ttttatttgc ggtattgttc 720
gtattttaat ttattgtgtt ttttattttt tcgttttatt tttggattga aaattttata 780
ttttataatt ttagtttgtt ttttttattt ttaaagatat ttagatagaa aaatgtagaa 840
ttagttgagt ttatgaatta aattattatt taaagagaaa gagaatgagt ttttatatag 900
ggaaaggtgt ttaaatatta ttaaggttat tagggttatg aaagaagaat aagtttttat 960
atagttattg tgttgatata tatgatttta tattttattt tattaaaatt tttttattta 1020
aatagtagtt tttttggagt gtttaaagta agatgaaatt taagatatat aattttattt 1080
gatatttatt taattgtagt tttttttaatt tttatttttt ttcgtgcgaa tgtttttgtg 1140
tattatttat gtaatttaga taaattattt tttagaattc ggagttaata aataggtgat 1200
aggttattta aggtttgagg agaaagaaga gtaggaatag attgaggtat ttatttttta 1260
tgtaagttta aattttagaa tttattagtt ggttaattat tttatagtgt gtgttttttt 1320
ttcgtattta aaagtattta taattggttg ttgttaagag aatgttatag tatatttaaa 1380
ttgttaggat atttaatatt tattgtatat gtttaatgtg agatttttaa ttttgttatt 1440
tttaatagta ttttttgttt taatttttaa tatagtatat attttttagg taggattgta 1500
aatatgttaa gtttgtagtt tgaaaataaa gatcgttttg aattatttaa gtataaaaga 1560
aatttttttt tttagttaaa tttttttggg gaggatttta ttttagaagg aggtaaaatg 1620
attgaatttt agttaattta gtatttttaa agaaggtagt ttttattgat atatttttag 1680
ttaggttaat tttagtttta aaagaaaatt tttaagattg tttttatgat tttaagattg 1740
ggattttaga aagaagtttt taaatttaaa ttaaatattg tagaagttgt aattattgtt 1800
gtatatataa gagaatttag aattatattt gatttgttaa taagtaaaaa gggagttatt 1860
tttgtaaatg tggaaaagaa aattgttagg taaaatgtta gataagaaag agttgtatat 1920
gagtagattg gattcgttag ttagggcgtg ttgtagtttg taaagtaggg agtatttagt 1980
tttagttatt ttttgagagg tattttttgg attatttttg ttttaaaagt aatatttaag 2040
ttttatttaa gaatttaaaa gtcgttttat tttttatttt tcggaatagt ttaagagtta 2100
ttttttttta gaattagtta ttaattgtga ttgtggttga aagagtttat gggtaggatg 2160
gttttggtta ggtgtttatt ttgaattttt tttttagtat tttggtatta gagcgtcgtt 2220
tgagttttgc ggggattttt gtatttaatg gttaggagtt ttttattagt tagaagttta 2280
gtgtcgggaa aagggttggg tttatttgtt taagaattag tttttgagat tgatgatttt 2340
gtaagatgga ttggttaatg atttttggtt gttgcgttgt tgtggatttg gttttttttt 2400
tagtttgttc gttttttttt tttttttaaa ttttttttgg ttaggtaaga ttttttttat 2460
atttatttta tattttttttg ttttatttat tcgtggttat aaaggaaaga gtttaattat 2520
tcgatttttt tatttatttt tgagaaaaga gaaaaaaaga ataaaataaa aataagattt 2580
atttttaaaa aaatagttga agtggaaaag gtttcgagat ttttgtagtt acgttttaaa 2640
taagaattgt agaatattgt aaatttagat ttataaaaag aatatttggt ggagagtggg 2700
gtagaatttt tgtcgtattt tttaagcgtt tttaagagat aaaattttgt agcggaagat 2760
gtaaacgtaa gggtgtaggg gtgattgttt tgagaattgt tagagtgtta ttgaaattaa 2820
gtggaggtta agtcgaattt gatttttaga taattttata gtaaggtagc ggtttattaa 2880
ataggttagt tgataagttg tagttatttt ttgagtattt ttgtaaaaat ggtaagggat 2940
taattttgta atttgttttt tttatgaaag tatagttttg tttatatttc gttggagaaa 3000
taatattcgt ttttattttt tttaaaaagt tgaatttttt agtcggttta agtagtttta 3060
tattttgagg tttttaagat taaagttgcg agttttagta gggaatagtt acgtggtttg 3120
tttgcgtttc gtttgggttt ttgtttttag tttgagatat ttgtagtcgc gaattttgtt 3180
ttagtttaga aaggggcgtt ttgtttaatt aattgttttc gtcggcgagt tcgtattgag 3240
aagtttatga gcggatttta tgtgtagggt attttagcgc ggtgtataaa attcgtcgtt 3300
tttaaacgtt gtttttattt taatattgtg tattgatttt agttttttat tttgttatgt 3360
aagggatgga tttgaaacgg ttattttatt ttaatttatt ttaaaaagga aataagtatg 3420
gtattgtaaa agatgggttt tttatttaag gcgatttttt ttttggttta ttaattttgt 3480
tgtttttagt ttgttaggat ttatattaat attttttttg gggtttttcg ttttaattta 3540
tagatagaaa tgtttaaaat gttagcgtat ttaagtattt ggaattgggg tttacgaagt 3600
ttaattgttt attggatgtt ttagatagtg gggttggggg cgggggggggt tttagagcgg 3660
gtagttttta tgtttaggcg ttattaaatt tttattttat ttttttataa gttggttttt 3720
taaggttata atgcggagtt taatttgggg gtggggatga aatggttata gggttttttt 3780
tttgggttgg tattgttagt tgttagggtc gtagtaaagg cgttgcgttg tttttttttg 3840
gttttgttgt tttttttatg gattgggttt ttttttatcg aagagtgaat ttttgttttt 3900

ttcgagtatt tttcgaggcg tagttttttg gatgttgggg agcgttagat tgggtcgttg 3960
tagaggggaa aggagggttt agaagggcga gagagtaggt cgggacgtaa atttttagtt 4020
ttcgcggcgc ggttacgttt ttagaaacgt ttaggatttt cgggttgggt cgtcgcggtt 4080
tggtttttgg aattttaagg ggttcgttta tttgattatt gggtgggttt cgcggttgat 4140
attttttttg gtatgttttt ttatttcgcg ttatattatt tttttagttt tagtaatttt 4200
aaatcggttt tacggattta gagggttttt gggcgagatg agatattatt tattgtgtag 4260
aagttgttgt tattgttgtt gttatagtta tttcggatgg ggttgttatc gcggttagga 4320
tagttttttt cgatcgtttt ttgggttgcg ttagggttcg ggggcgttgt tcgtacgttt 4380
cggcggggaa ggaaatcgtt tcgcgttcgt cggaggaagg cgacggggag ggaaggggga 4440
gggcggttag gaggcgggtg gaggggtcgg tcgttcgggt taggtcgttt ttgaatggtt 4500
tgggaggacg aattgttaga tttcgaggaa gggaggtggg acgggggagg gggattggaa 4560
agcggaaatt tttttataaa atttcgaaaa gttttttttt tttacgttag gttaatgata 4620
ttgttgtttt taaatttttc gtttgtacgg aggtataaga gtttttaagt ttgtagtttt 4680
cgtttaattt ttagatattt cgcgggtttt gtagtatcgg tatcgttttt aggaggtttg 4740
gcggggtgtg cgtttagtcg ttgggcgttt tttttttgga tttcggggtt atttatatcg 4800
tttttttttt ttttcgtttt ttttttcgtt tttttcgcgc gtttttttcg cggaggtttt 4860
tttcgttcgt ttttttgttt tttttttcgc gggtcgtatc gttcgggtcg gcgtcgcgcg 4920
cgggggaagt tggcgggttg aggcgtttcg tttttttttt ttgtttcggg ttcgcgaggt 4980
tacgcgtcgt cgttcgagag atgatgtagg acgtgtttag ttcgttagtt tcgtcggtcg 5040
acgatagttt gagtaatagc gaggaagagt tagatcggta gtagtcgtcg agcggtaagc 5100
gcgggggacg taagcggcgt agtagtaggc gtagcgcggg cggcggcgcg gggttcggcg 5160
gagtcgcggg tgggggcgtc ggaggcggcg acgagtcggg tagttcggtt tagggtaagc 5220
gcggtaagaa gtttgcgggt tgtggcggcg gcggcggcgc gggcggcggc ggcggtagta 5280
gtagcggcgg cgggagttcg tagttttacg aggagttgta gacgtagcgg gttatggtta 5340
acgtgcggga gcgttagcgt atttagtcgt tgaacgaggc gttcgtcgcg ttgcggaaga 5400
ttatttttac gttgttttcg gataagttga gtaagattta gatttttaag ttggcggtta 5460
ggtatatcga ttttttttat taggtttttt agagcgacga gttggatttt aagatggtaa 5520
gttgtagtta tgtggtttac gagcggttta gttacgtttt ttcggtttgg aggatggagg 5580
gggtttggtt tatgttcgcg ttttattagt aggcggagtt ttttattttt ttagtagggt 5640
cggagattta ggtaaggatc gcgtcgttgt attttttcgt tttttaggtg gtagacggta 5700
ggtcggttag gtcgcggttt ttagtttatt tcgatttttt tttttttttt atttttcgtt 5760
tagttttttt atttattttg gtatcgttgt ttcgcgtttt tagcgttttc ggaaggtcgg 5820
tttgatttcg ttagggagag tagtttttag ggggatgcgt tttggtgagg ggtgtgtgtg 5880
cgcgtgagtg tgcgtgatag gaggggagat agagatattt agggttacgg gtaaggatcg 5940
ttttgttagc gttatttttt ttttcggttt ttaatttttg ttttttttaa aataaatgtt 6000
ttaaaataaa ttttattttt tttttttttt tatttattta tttttttttg tttttgggtt 6060
gaaatttttt taggttgttt agtttaattt tttagtggtg gtgataagaa tagtgtttat 6120
tagttttaga aaatagtcgt agagatttaa ataataatcg attttttttt ttttttttgg 6180
gtttttgtag atgttattgt ttttagagaa ggagaaaatg gatagtttag agattttgga 6240
gttggataat taaaaataaa aatatatgtt aaagattttt ttggaaatta gaagagtaaa 6300
atttaaattt aaagaaatag ggcgtggggc gtatttttaa aagagaaagc gagataggtt 6360
cgtggatagt gatttttaga cgggtagcgg tattattttt atatttttgt attttgatag 6420
aagtttgaat agttgtttgt gttttttttt tttttttttt tgacgaagaa tgtttttatt 6480
tttatttttt ttatgtatgt atttttaaga ggtcgtgtta attagttatt gaaaggaaag 6540
gtattattat ggattttttt tattttaaaa tggtaataat tagaggaatt ataagaatat 6600
ttttagaaa 6609

<210> 264
<211> 6609
<212> DNA
<213> Artificial Sequence

<220>
<223> chemically treated genomic DNA (Homo sapiens)

<400> 264

tttttaaagg tgtttttata gtttttttga ttgttattat tttaaaatag agaaagttta 60
tagtgatgtt ttttttttta gtggttgatt ggtacgattt tttgagaatg tatgtatgaa 120
aaaaataaaa ataaaaatat ttttcgttaa aaaaaaaaaa aaaaaaaata taaataattg 180
tttagatttt tattagaatg tagaggtgtg aggatggtgt cgttgttcgt ttgggaatta 240
ttgtttacgg gtttgtttcg tttttttttt taaaagtgcg ttttacgttt tgtttttttg 300
aatttggatt ttgttttttt aatttttaag aaaatttttg gtatatattt ttatttttag 360
ttatttagtt ttagagtttt tagattgttt attttttttt tttttggaaa taatgatatt 420
tgtaaaaatt tagagggggg ggggggagtc ggttattgtt taggtttttg cggttgtttt 480
ttaagattag tgagtattgt ttttattatt attattgaga aattaagttg aataatttgg 540

aaggatttta gtttaagggt aagaggaagt gggtgggtgg aaaggaggag gaggtggaat 600
ttgttttaaa atatttgttt taaggagaat aaaaattgaa agtcgaaaga aagggtggcg 660
ttgataaaac ggttttattt cgtgatttg ggtgtttttg tttttttttt tgttacgtat 720
atttacgcgt atatatattt tttattaggg cgtatttttt tggagattgt tttttttagc 780
ggggttagat cggtttttcg gggacgttgg gggcgcgagg taacggtgtt aagtgaggtg 840
ggaaggttga gcggagagtg ggagagggga ggaaatcgag gtggattggg aatcgcggtt 900
tggtcggttt gtcgtttgtt atttgagagg cgaaggggtg tagcggcgcg gtttttattt 960
aggttttcgg ttttgttgag gggtgggggg gtttcgtttg ttagtgggac gcggatatgg 1020
attaggtttt ttttattttt tagatcgaga aggcgtagtt gagtcgttcg tgagttatat 1080
agttgtagtt tgttattttg gagtttagtt cgtcgttttg gaggatttgg tagaggaagt 1140
cgatgtattt ggtcgttagt ttgagggttt gaattttgtt tagtttgttc gagggtagcg 1200
tggggatgat ttttcgtagc gcggcgaacg tttcgtttag cgattgggtg cgttggcgtt 1260
ttcgtacgtt ggttatgatt cgttgcgttt gtagtttttc gtaagattgc ggattttcgt 1320
cgtcgttgtt gttgtcgtcg tcgtcgttcg cgtcgtcgtc gtcgttatag ttcgtagatt 1380
ttttgtcgcg tttgttttgg gtcgggttgt tcggttcgtc gtcgttttcg acgtttttat 1440
tcgcggtttc gtcgggtttc gcgtcgtcgt tcgcgttgcg tttgttgttg cgtcgtttgc 1500
gtttttcgcg tttgtcgttc ggcggttgtt gtcggtttgg ttttttttcg ttgttgttta 1560
ggttgtcgtc ggtcggcgag attggcgagt tggatacgtt ttgtattatt tttcgagcgg 1620
cgacgcgtgg tttcgcgggt tcggggtaga ggagaagagc ggggcgtttt agttcgttag 1680
tttttttcgc gcgcggcgtc ggttcgggcg atgcggttcg cggaggagag agtaggagga 1740
cggacgggag ggattttcgc ggggagggcg cgcgggggag gcggggaggg aggcgggagg 1800
gggaggggac ggtgtggatg gtttcgaggt ttaaaaagaa agcgtttaac ggttggacgt 1860
atatttcgtt aggttttttg gaaacggtgt cggtgttgta gagttcgcga ggtgtttggg 1920
agttgggcga gagttgtaga tttggaggtt tttatatttt cgtgtaggcg gaaagtttgg 1980
gggtagtagt gttattggtt tgacgtgagg aggagggatt tttcgaagtt ttataggaaa 2040
gttttcgttt tttagttttt ttttttcgtt ttattttttt ttttcggggt ttaataattc 2100
gtttttttaa attatttaaa aacgatttgg ttcgggcggt cggttttttt attcgttttt 2160
tagtcgtttt tttttttttt ttttcgtcgt tttttttcgg cgggcgcggg gcgatttttt 2220
ttttcgtcgg agcgtgcggg tagcgttttc gaattttagc gtagtttagg aagcggtcgg 2280
aggagattgt tttggtcgcg gtggtagttt tattcggagt ggttgtgata gtagtaatgg 2340
taatagtttt tatatagtgg gtgatgtttt atttcgttta agagtttttt aggttcgtgg 2400
ggtcgatttg ggattgttgg ggttgggggg gtggtgtggc gcgggtgggg ggggtatgtt 2460
aagaaaagtg ttaatcgcgg agtttattta atggttaggt agacgaattt tttggagttt 2520
taaaggttaa atcgcggcgg tttagttcgg aggttttggg cgtttttgaa gacgtggtcg 2580
cgtcgcgggg gttgaggatt tgcgtttcgg tttgtttttt cgtttttttg ggtttttttt 2640
ttttttttat aacgatttag tttgacgttt tttaatattt aaaggattac gtttcggaag 2700
gtgttcgaaa gaggtagaag tttatttttc ggtggaagga aatttagttt atgggaaagg 2760
tagtagagtt agaggggggt agcgtagcgt ttttgttgcg gttttaatag ttggtaatgt 2820
taatttaagg gagagatttt gtggttattt tatttttatt tttaaattag gtttcgtatt 2880
gtgattttga aaggttagtt tgtaagagag tgaagtggga atttgatagc gtttagatat 2940
aggggttgtt cgttttgaga tttttttcgt tttagttttt attatttagg gtatttagtg 3000
gataattagg tttcgtgagt tttaatttta aatgtttgga tacgttaata ttttaagtat 3060
ttttgtttgt aagttaaaac gaagagtttt aaagagggtg ttaatgtaga ttttggtaaa 3120
ttggaagtag taaagttggt gaattagaaa ggaagtcgtt ttggataaga agtttatttt 3180
ttgtaatgtt atatttgttt tttttttgaa atgaattgag gtaaaataat cgttttaggt 3240
ttatttttta tatggtaaag taaaaagttg gagttagtat atagtgttgg ggtgggggta 3300
gcgtttgggg gcgacgagtt ttgtgtatcg cgttggagta ttttgtatat aaggttcgtt 3360
tatgggtttt ttagtacgga ttcgtcggcg ggaataatta attgagtaaa gcgttttttt 3420
ttgggttgga gtaaggttcg cggttgtaga tattttaagt tgaaggtaag agtttaggcg 3480
aggcgtaggt aggttacgtg gttgtttttt gttgagattc gtagttttgg ttttggaaat 3540
tttagggtgt ggagttgttt gggtcgattg aagggtttag ttttttggga gaagtgaggg 3600
cgagtgttat ttttttagcg aggtgtaaat aagattgtgt ttttatggga gggataaatt 3660
gtagagttga ttttttatta tttttataga aatatttaga aagtgattat agtttgttaa 3720
ttggtttgtt tagtgagtcg ttgttttatt gtaaaattgt ttgaaaatta gattcgattt 3780
gatttttatt taattttagt agtattttag tagtttttaa aatagttatt tttgtatttt 3840
tgcgtttgta tttttcgtta taggatttta ttttttggaa gcgtttagag aatgcggtag 3900
aaattttgtt ttatttttta ttaagtgttt tttttgtaaa tttgaattta tagtattttg 3960
taatttttat ttagaacgtg gttgtagaaa tttcgaaatt ttttttattt tagttatttt 4020
tttaaagata aattttattt ttgttttgtt tttttttttt ttttttttaa aaataaataa 4080
agagatcgaa tgattggatt tttttttttg tggttacgga taagtaagat aggaaagtgt 4140
gggtaaagtg taaaggaaat tttatttgat taaggggaat ttgggaaggg gaggggagcg 4200
gataagttag aggagaaatt aagtttatag tagcgtagta gttaggggtt attagttagt 4260
ttattttgta aagttattag ttttaaagat tggtttttaa ataagtgagt ttagtttttt 4320
tttcggtatt aaatttttag ttgatgaaga gtttttggtt attgggtata gggattttcg 4380
taaggtttag gcgacgtttt gatgttagaa tattgagagg ggaatttaaa atgaatattt 4440
aattagaatt attttgttta tggatttttt tagttatagt tataattgat gattagtttt 4500

aagaagaaat ggtttttgaa ttgtttcggg gagtggagga tgagacgatt tttgagtttt 4560
taagtggggt ttgaatgtta tttttggagt aaaagtagtt taggaggtat tttttaaaaa 4620
atggttggag ttagatattt tttgttttgt aaattgtaat acgttttggt tgacgagttt 4680
agtttgttta tatatagttt ttttttattt aatattttat ttaataattt ttttttttat 4740
atttgtaagg atggtttttt ttttatttgt tgataggtta ggtataattt tggatttttt 4800
tatatatgta ataataattg taatttttgt aatgtttagt ttaaatttga aagttttttt 4860
ttaaaatttt aattttgaga ttatgaaaat aattttagaa attttttttt gaggttgaag 4920
ttagtttaat tggaagtatg ttagtgggga ttattttttt tgggaatgtt agattaattg 4980
gaatttagtt attttgtttt tttttaaagt aaagtttttt ttaaaaaaat ttaattgaaa 5040
aaaaaaattt tttttgtatt tggatagttt aaaacgattt ttgtttttaa attgtaaatt 5100
tggtatgttt gtaattttgt ttgaggaatg tgtgttgtgt taaaaattga aataaaaaat 5160
attgttaaaa gtaatagagt tagaaatttt atattgaata tatataataa gtattaagta 5220
ttttggtagt ttgaatatgt tgtgatattt ttttagtaat aattaattat aaatattttt 5280
agatacggaa aagaaatata tattatagaa tggttagtta attggtaaat tttggagttt 5340
gggtttgtat aaaaggtgag tattttaatt tgtttttatt tttttttttt tttagatttt 5400
agatggttta ttatttgttt gttggtttcg agttttgaga aataatttat ttgaattgta 5460
taaatagtgt ataggagtat tcgtacggaa gggagtgaaa attagaaagg ttgtaattga 5520
gtgaatatta gatgaggttg tatattttag attttatttt gttttgaata ttttagaggg 5580
attgttgttt aaatgagaga attttagtaa ggtggaatat aaagttatgt atgttaatat 5640
aataattgtg taaaagttta tttttttttt atgattttgg tagttttaat gatgtttgaa 5700
tatttttttt tatataaaaa tttatttttt ttttttttaa atgatgattt gatttataaa 5760
tttaattgat tttatatttt tttatttgaa tatttttaaa agtaaaaaaa ataaattaaa 5820
gttataaaat gtgaagtttt taatttagaa ataaaacgaa aaagtaaaaa atataataaa 5880
ttagggtgcg aatagtatcg taagtgaaat ttttaaataa atgttaattt ttaaaatagt 5940
tgatattttg aatttagttt aaggatggga tgaggagata taggtttaat ttgtagaaag 6000
tgtaggatta gaatatggaa aagtatggag ttttgaagag tatttttttt ttttattttt 6060
ttttttaggg gttgtgtagg ttggagagtt ggtgttttag ttgtttatta attttgttgg 6120
aaaatatatt tattttattt gtattaaaga gttgttggaa taattgatga aagaatgtaa 6180
gaaataaata gttatttata ggaattatta ttatggtttt agttttttta tatagtatta 6240
tgtgtttttt ttttaattat tcgatgatta gtaatttaaa tataaatgga atgatttata 6300
ttttatgttt ttttaattta attttttttt ttgattattt ataattttta ttttttttta 6360
ttggaaggga ttttcgtttt ttataaatgg gtttttttga agttttatgt tttgttgtta 6420
ttgtttggtt tggttttttt tgtagaaaat atgttgtttt tgttagttat tataagagga 6480
tataattttt tagataattt tgtttagaaa gaagtttaga gaagaggaaa gttaataaaa 6540
tagtaaaaat aatatataat taatgaatat tatttggaga tgtttatttg atttatgtta 6600
tttttatta 6609

<210> 265
<211> 14712
<212> DNA
<213> Artificial Sequence

<220>
<223> chemically treated genomic DNA (Homo sapiens)

<400> 265

atatttggga gttgtgtgtg tatggaagtt tacgtatgtt tgtatttgaa ttgtgtgcgc 60
gtaggtggtt tgtgtgtagt ttttttttta tgatgtgtgt acggttattt tatatttttg 120
tgttattgtg tgtgagggta gatgatatgt tttggtattt ttcgagtgtg tttgtaatta 180
tatgcggggt gatatgtttt agtttgtgtg tgaggattgg ttgtgtggga gtattggtat 240
ggttgggagg ttgttatggg tattgtgggt ttagatgttt gtgttgtatg tggaggtatt 300
tgtagttggg tgtggggtag tttaggagtg ttattttatt tgtagttgta aagaatgggg 360
aaaagaatgt taggtatttt gggggatttg agataggttt cgtgttttta ttaagttttt 420
attttttttt ttgttaatga attagttatt tttgaggacg ttttgggatt gaggttttta 480
gaaggagggg gagatatgga tgaatttata gtattcgatg ggtagttaga aatcgtagtt 540
atgttgtggg gtaagtagtt ggtttgttta ggggaggtta agtgaggggg tttagaagta 600
ttatacgttt gggttattcg acgtaggagg tgttttttta ggttttttta atttaggaaa 660
tttagttttt ttttgttttt ttttttttgt gtttagaata ttttttagta gttattttaa 720
cggttgggac gtcgtttagt tttatattta aggtttatag cgggttttgt agcgcggagt 780
tttcgtgttt tttttttttg taggatcgtt gatttttttt ttataattgg aattagaggt 840
tagaagagat ggtttttcga aggattagga ttaggatacg aaggtaggtt tcgggatttt 900
agacgtttat agtaattacg ttgcgcgatt aatatttaag gatgtttcgg ttttagattg 960
agatttaagt ttgatttttt tttgttagtt gaggtttttt gtttggttgt tgcgacgatt 1020
cgagatgggt tttattcggg ttttacgcgt gtcgtaggta cgtagtacgt acgtttagta 1080
aatattagtt gtagtgagcg agtttttttt tgtaattcga tagagtatta agttttttgg 1140

agcggcgggc gtcgtcggtt agtggcgttc gcggggattt cgttttcgtt tttcgcgcgc 1200
ggtcggggtt attcggtggg gttgcgacgc gttgacgtgg gcggattaaa gttgtcgttc 1260
gttttataaa gtagtttggg aggttcgcgt cggagatcgc gttaggcgag gaggagagtt 1320
tcggcgagcg gcgagtatag gttacgggag ttgagcgtag tagagagtag tcggagttcg 1380
cgtttgattt cgcgcgggtt ttgagtcggt cgtagtttgt taggattttc ggcgtttttt 1440
tcgcgtcgtt ttacgaagag ggcgttttta tcgttcgttt agtcggagtg cgtacgggtt 1500
cgttggaaat cgtattcgtc gttttttggg taggtcgggg tattcgggta cgtagggcgc 1560
gtttggcgag cgtttcggga ggtgagtagt ggtcgcgggt agcgggtagt cgtttagcgg 1620
ggattcgtta gggttggggg cgttcgagtt cgagggggac gacgtttttc gtattcgtta 1680
cgattttttt gggtatttat tttcgggtaa gtaaattaag cgttttttta tatttttttt 1740
tcgtagtatt tcgaagagtt tttttttgcg gagcgaagtt ttgggagttt ggggacgcgg 1800
ggtttagggg gacgcgggac ggtggtgggt tgtgtagttt ttgcggtttt tttcgaattt 1860
ttttagatcg gttttttttt tttgttttcg tattttaggc gatttttttt tttttagtcg 1920
ttcggcggat gaggttatgt tcgagttatt ttcgggggtg ggggatttgg atttcgcgat 1980
agagggtgtg tgtggtgggc ggggggaaag ggtggtaaaa ggtaaagaaa tgcggtagcg 2040
tttagtaaat tggggatggg ggtagaagtt tggttttgag aacgggtttt aggggcggcg 2100
gggggagggg gagaatcgag tttgtttatt gatttgagtt ttaggttggg ggttagggag 2160
gaggaaagtt ttgttttcgg tttcgaggta tatttgtaat cgttcggaat atttggttga 2220
gttcgcgtac gagttttacg ttttttattt tcggggttga agtgtttcgg tgttttttaa 2280
ggttttgggc gtgtagggtt cggttttagg gggttagtag gagggatttc gagtgttttt 2340
atttattttt tttattttta aaataattcg tttttcgggt gattcggcgt cgcgtgcgta 2400
gttaagtaga cgtttttcga aagttttacg gttggggtta aagtcgggtg gtaggttcgg 2460
tttcgtttcg tttcgtaggt aattttgaag tcgtcgttcg gcgtttttgg ttggcggggt 2520
tagtttcgtg agttgcgtgg cgggaggttt cgggattcgt tcgtgtcgat tttgcgcgtc 2580
gcgcggataa tcggtttcgg tgtacggtgt tcgtggggcg tcggggtagg gggggatttc 2640
gtaagttttc gtcggcgtgg tttggtttta gcggtcggtt ttttagagtt ttttatagat 2700
cgggtttatg attatttgtt ggcggcgttg ggtttttttt ttttttggga gattaggttg 2760
gatatagatt tttcgttgtg agttcgcgcg ttttcgtttt tttgttgttt tcgttttttt 2820
tttatttagg aatgtcgggt gttttcgggg ggcggcgtat tttgggagag gggaagacgg 2880
tgattggagt tttgattttt ttttttatat ttagcggata tttttttcgt tttgagaagt 2940
gtaagaaatt tgtaggatta ggaagataaa ggatttggat ataggttggg tgggaggacg 3000
ggattgggtt atatcgagaa tttttttgag atttttagtt ttgacggtat gtttgtcgta 3060
ggcgtttttg tgttgggcgt tagttgcggg gttttgcgga ggaaaaaaat tgtacgattt 3120
aggttttttt tcgttttttt gtgggttttg tcgttaggtt ttagaaaata gtggggtttt 3180
ttatttttgc gtgttgatta tttacgcgta ggaacgcgtt acgtaggtgg cgtcgcggtg 3240
taaatagtag aggggcgttg agtttcgtag gttgtaggga gacggggttt tcgggggacg 3300
tacggcgaag ttttattttt cgtttttatg gaaggggata tcgaggttgg ggtggttgag 3360
ggtttgttta agtttataga gttagataag aacgtttttt ttttgtttta gcgtttttat 3420
tatcgtgggc gttttttttt ttaatttttt aaatttttag aaaggaattt ttttgttttt 3480
gtttttttcg aggggagagg ggttggggtt gtgtttgatt tcgggcgaac gttaggtgtt 3540
ggtaaatttg ggagaagttg ggaagggagt taatgaggtg agaatgaagt gaaggagaaa 3600
aaaagcggag atagttgttt cgcgggttgc gggcgggcgg ggtagttttt ttttcgcggt 3660
tcgagggcgt cgtttggagg ttagaggtaa tatcgcgtta gtttcgcgag ttttttcgtt 3720
tcgttaaggc ggggttgttt agttttagtt cgtatgtttt tatttttatt ttttaaagtg 3780
gagcggttaa agacggattt ggagattcgg aatttttatt gtataaagag ttttattatt 3840
tcgtttttta tttttttttt tttatatcga gaaagtaggg tttcgagtaa gttttatttt 3900
ttgatttcgt cggatattcg gtttgtcgtt ttttagttga attttatttt tgtgttttag 3960
tttttttatt tgtgagatga ggtagcgata gtgtttattt tacggggtgt tgggaggagt 4020
agatgcgatc gtgttaggga attttttttg tttgtttgtt tgtttgagac ggagtttcgt 4080
ttttgtcgtt taggttagag tgtagtggcg taattttcgt tcgttgtaat tttcgttttt 4140
cgggtttaag cggttttttt gttttagttt ttcgagtagt tgggattata ggcgttcgtt 4200
attacgttcg gttaattttt tgtattcgta gtagagacgg ggttttgtta tgttgggtag 4260
gttggtttcg agtttttgat tttaggtgat tcgttcgttt cgatttttta aagtgttggg 4320
attataggcg tgagttattt tgttcggtta gggaacgttt tttataaaag cggaattgtg 4380
ttcgaattgt tttagtttat tgaggaggta aattgaaata cgtttgggaa ggagaattgt 4440
cgaggtttta ttaattaatg tttaatattg tagggataag ttttagtttc gggttttcga 4500
ttttgttgtt tcgtttggat ttttgatttt ttattttttt ttttttcgtt ttaggtttgc 4560
gtttgttcgg gttttttttt ttgttttcgt ggtttttagt tttgtttttt gagcgttttt 4620
ttttttttcg ttgtgtttgt tcggtttttt ttggggtttt cggttttgtc gttttttgtt 4680
ttttttttcg gttgtttcgg ttagaggggt tcgaaggcgc gttggggttc ggggttacgt 4740
tgggagatac gttttcggtc gcgtttaatt cgagttaggc gcgggacgcg tttttcggtc 4800
gggaggagcg ggaggggacg ttcgtcgcgt tcggtttttt cgggtttcga tttttttttt 4860
gtaatttgaa taaaacgttt ttttcgttcg cgcgtcgttt taattcgtcg ttttcgtttt 4920
tttcggttgt aggcggcgtg taggattagc ggcggtcgtg taggcggagg atttcggcgc 4980
ggtttttttt gggtgtgatt tcgggcgcgt tcgtcgcgcg acgatgaggg cgcggtcgta 5040
ggtttgcgag gcgttgtttt tcgttttggc gttttagatc ggcgtgtgtt atggtattaa 5100

gtggttgtga gtagggattt cgcgtttacg cgtataggtc ggggattggg gcggcggagg 5160
gcgttttggg gttttttgga tggggcgagc gtattaagat tcgggagttt aagttggttt 5220
ggcggtcgtg ggtaagttat tttttagttt ttgttttttt ttttgtaaaa tgtttttgtt 5280
tttcggaggt ttgcgaaagg gagtgagacg ggcgtggatt ataatggaag cgtttggttt 5340
tgtgtagttg acggtggcgt aagggttacg gagtttttgg ttattttcgt aggggtagat 5400
ggaaaaagat attttgttta gggggcgtaa tttagagcgg ttagtttagg agcggttttt 5460
gttcgttagt ttttttgatt ttagcgggag tttcgttgag atatttttga tttttttatt 5520
tgggcgattt tgggaaagtg ttattttatt ttttggggtt tttttgtttt tttttgttat 5580
aggagggtga gattttttat tttttttaaa ttagatgaga agaggttttt gaaaggattt 5640
tagaagttgt tagatgttat tgtgtatatg tgtagggtat ttttttagat agaaattatt 5700
tacgtagggg taggagggag agagaggttt ggtgtcgagt tgaagggagt gtttgtttag 5760
ggggttgggg gtggtgaaga gaggagtaaa tattagtttt ttttcggagg agttgcgttt 5820
taagttgaga gagataagtt taggttgtaa ataattttat aagtgtttaa gtgttcgagg 5880
agaaattaga gcgggaattt ggaggtttag aggaaggaag tttatttggt ggggatatag 5940
atgtgagata tcgggaagtt ttttcggggt tgtgtttttg gtgattgaag cgttgtcggg 6000
agttggttgg ttcgggtaat ggagaggacg ttgtgggtag aggggttttt tgagagggaa 6060
agtttaggat aggataaggg tttggaattt ggggttaggg tagggagttt gtagataaga 6120
tagtaggggg taggttgtgg gggttgggga gggcgttgta aagggtttgt gaattagttg 6180
gaagataggg tgatagtggt tgcgtcgtta gtaaatagtt tggaggttat tggttttgtt 6240
tttttttagg ttaagttatt gattaattga cgattcgagg agggaatttc gtttttttgt 6300
tttttttgtt tataagaagt ttggttttag taatttttta gttttgggta ttagtttttt 6360
ttagttgttt ttgaggagtt ttgtgagaaa ttagttattt tttttttgta atggggtatt 6420
tagtattgtt ttagttggta gggattttcg attagcgagt ttaagttttt ttatttatcg 6480
ttattatttt atagataagg atattaaggt ttagggaagg agaggattgg ttgaaggagc 6540
gttagaggtt ttgtagtggg tttgggtgtt atttgtcgtt tataagagtt ttgtggggtt 6600
attgttatta tttttgtttt atagtgggaa tatcgagatt ttgtaaaggg tatgatgtac 6660
gtaagttttt acgttggttt gtggtgggat ggacggttgg ttaaatttga cgtttatgtt 6720
ttttgttttt attgtaaagt taggttgtta tagaaatggg tataatttat tgtgtgtttt 6780
aaataagttt ttaggttttt gggggagatg gatatataga aatataaagt ggattatggt 6840
ttgtgtttta gtagagggag tatggtagtt tagaggagga gtgattagtt ttatttaagg 6900
gagatgtata ggttaggatt ggaaggatag ggaggatttg gatggttaga gacggagtgt 6960
aaagtaggcg ttttaggcgg gaggtatagt acgagtagaa gtttggaggt cgaatagcgt 7020
atgcggggag atttggagaa attaggtatt ataagagttt atttttaagt gttttagacg 7080
ttgtttcgag agtttgggtt atgtaagtta gattatattg tatttttatg agaaattgat 7140
agtttagtaa taggggatta aagttttgtt agagtttaag atagaggggg aggtgggaaa 7200
aggggtggag tttaagaata ataacgattt gtgtatgtat gtgtatatat agatatacgt 7260
gtgtaggtta cgtgtatata tttatgtata tgtatatata tgtttatata tatatggttt 7320
tattttgtgt taggcgtcgt tttaagtgtt ttatgtatat tgttttattt aaatatttta 7380
attattttgt gaggtgggtg ttgttattat tttcgttgta tagaagagga aatagagatt 7440
ggggtatata gagatggggg attttattta gtagggtgtt atagtttaga ggtagtagag 7500
gtaggatttg agttaagttg tttagttttg tggattgttg ggtataggga gttttagtat 7560
agttgggagt agggaagtgt ttagtgaggt tggaaagtcg gggatttatt taggagtttt 7620
cgttggtttg ggttttggag agtgagttgg agatgaaagg ggtatttggt ataggaattg 7680
aaagttaata tggtttagtg gtttggaatg tttatttggt gggtgtttgg gacgtaggtt 7740
gagttggtgg gatgggggtt ggggagaggg gattagaatt tggttgttta tagaggtatt 7800
tgtttagaaa taatttgggg attttttttt attttatttt tatttaggtt aggtattaga 7860
agatttttag ttttttaagt attcggggtt ttaggtttgg gttgtttatt ttttttttgg 7920
tttttttggg tagggggagg gtttgagggg agttttattt ttttgtgaat tacggaagcg 7980
gtggggtgag aggaaaggtt taggtcggtt gggaatattt ataaaggtcg tttttttttt 8040
tgggtagttt tatatttgtt atttttacgg aggtggggtt aggaggtttt agagggttat 8100
ggcgttgtag agtagggtgg aaggagtatt agattgggat tgtaaggttt agtttgagtt 8160
ttgtaggtta atcgttttag tttttttgag ttttagtttt tttagtttta agatagaggt 8220
agaaggagtt ttattttagg atgtaggagg tttggggttt agtttgtttt tgtttttgat 8280
tggttgcgta tagatttggg taagttttgt ttttttcggg ttttagtttt tttatttgta 8340
tagtgggagt ttatgatgtt gtggtagttg tcgtttggtt ttttggttga gaagtattag 8400
attaaggttt gttagggttt tatttttgta agatgggttg tataggtggg ttagtatgtg 8460
tgaggttaag gggataggtt tttatttttg aggtttgggt agttaggtgt gaaggggatt 8520
tagtgttatt tttaggtagg attggatttg taggtttaag gttttttatt agtttttatt 8580
tttttattta tatagtgagg ataagggttg gattagtttt tttttatttt ggagttttgg 8640
gataaagttt aggagggaga gaataggagg atttttaggt tgtggggttt tagggatttt 8700
ttttttttga acgggtagtt gtagttttaa gttgggatgg ttggtgaaga aggggtattt 8760
tcgaggtttt tagatttttt ttcggttttt agatttggtg tttaattgaa gagttaattg 8820
aggagtcgtg gagggagagt ttttttaagg ttgttttagg ggagtagacg gaagtttggg 8880
gagaggttgg gtttgtttgg gtggtttaga gtttggggtt ttgttttatt ttttttgtta 8940
tatgggtatt agtatgaggt ggggtttagg aaagtttaaa gagatacggg gaaaggaggg 9000
ataaaggttt ggttgggaga ataggatttt ttcgatttaa ttgttttttt attttagggt 9060

tagtagggtt tatagatgta gaagtatggg gattttaggg taagattgag tttttaagtt 9120
agttcgtagt ttttattagt atttttttta ttttagaagt atagatgtgg gtattagggt 9180
tttttttatc gttatttgtt gattttattt tatttttttt tttgggtggg tagttgttag 9240
gttatatttt gggtggcgtt gatgggggta tttttgaggt tttttgagt ttagttttgt 9300
cgaagtttta tttggaaatg gtttggtaat attatttagt atcgtgggcg gtcgagttgt 9360
agaattgtag agagggaatt tggggtttag gttatttaaa ggagtttgaa tttggttgtt 9420
aggttttggg tttagaattt attatgtgat ttgatttttg tgtttagagg gagagggatg 9480
ttgtgaagtg taggtttagg aaggaagttt ttttttgaag gtgttttata taggcgggtt 9540
ttttagagcg gtttgagggt agggttgttg gcgttattag gaaaatattt tagtgaagtt 9600
atttggtagt ttgtgttatt aatgttaaag tttgtattgt ttttaagtat tgagtagtat 9660
ggatggggaa attgaggtgg agtaaggggt agtgatttgt ttaaggtttt atagggagag 9720
agttgttgag ttagagtttg aatttaggtt tttgttttag ttagttttag taaggttttt 9780
aatttttgaa tgagaggagg ttttgttatt tcggtagggt tttgagttcg ggtttcgttt 9840
gtgaattttg tatttttttt tgtttttttt tttatttttt tttttaggtt tttattttta 9900
gataaatcga aatgttttta attaggggtt tgttggagtg aagcgagtaa gatgtttata 9960
aggagaattt ttttattttt tattttaatt tttgattatt tttttaaggg ggatttattt 10020
ggaaacggtt attattatta attattagat gatcgtatgg tttttttcgg gagtggaatt 10080
tgttgttttt cgtttgtgat ttttgtttgt agtttttgtg ggcgggtggt ttttttagta 10140
cgtatggtag tttgtttttg ttttagcgtt agtttttaag gcgggttttt gggtttttat 10200
attgttttgg gtttattagg gtttttggat ttgtttttta tttatttttg ggtcgtttgg 10260
ggagtttgag tttcgtttgg aaggtgtttt gatagtattt tttttttgag ttttttgggg 10320
aggatggaaa gattgagatt tagggtagtt ttagggttta ttttagggtt ttagtttagg 10380
agatttaggt attaggagta ttaggttttt tttttttaga gttgtagtat ttagtattta 10440
taagttgttt gtattgagtt ttttaggaag aggtttagtt tttatcgttt tatttcgtgg 10500
tttgagtcgg agtttaggga aagatatagg aggttgtttt tattgtttga acgtttatta 10560
tgtgttatga gtttgttgtt aggttatgcg ttgatgtgtt gtcgtttacg ttgttttttt 10620
tatttgaaaa tggaggaagt tggtttagaa ggagcgggaa gaaagttggg cgagggtaag 10680
cggtagagta ggtttaggac gggttgggtg gggatcgttt agagtaaagt agagtgggga 10740
tttagagaga agtgatttta gtgtttgtag ggattgatag ttttattaag aaggatgacg 10800
gggtttgttg gcgggtgttg gataagtata gtttttaaag tagggttgat tatttttttc 10860
gggttgtttt ttgaggtgtg aagtgggagg ttttgttaat cgggaagggt tatgtttata 10920
tggagttgtt tattttatga cggtattgta cgtagggata gggtttgttg ggtggaggtg 10980
gaagagttgt tgataatcgg gaaatatatt gaggtgatga cgtttgtttt tgtgtggtta 11040
gagtttattt aggaagtaag gtagggttta gtaataggtt ttttaagaag gtgaggttag 11100
ggtaggttag aatagtttta tttaggttaa gaaatcgcgt ttgagggatg ttgtgtggtt 11160
tattttgtgt cggatgttgg tatttagata tattagatat ttttcgattt atagtttagg 11220
gggtggtagt gaggggagat tgttttggat attatagttg attttatata gggcgagtta 11280
cgtcgtttta attatagata ttaggttttg ttttttgggt ttttgggaag gtttttattt 11340
ttgggggtta tagggtgggt gggatatggt agagcgtgag aggtaggagg aaatgaaaga 11400
atttattgag tatttatcgt agtatatatt atttagtttt ggtaggattt tgagaggttt 11460
ttacgttgta gttgatgttt agagaggggt aatagtttgt ttaagtttgt atagtatata 11520
gaggatagag tttgagtgag aatttaggtt tttttatttc gtttggttta atatttgttg 11580
ggtaataagc gggggattta gttttattta tgaatttttt gtttgtttgt tttgtttttt 11640
gagatggagt tttattttat cgttaggttg gagtgtagtg ttataatttt ggtttattgt 11700
aatttttgtt ttttaggttt tgttttagtt ttttaagtaa ttgggattat aggcgcgtat 11760
tattacgttt agttaatttt ttgtatttta gtagagacgg ggttttatta tattggttag 11820
aatggttttg attttttgat tttatgattt gttcgtttta gttttttaaa ttgttgggat 11880
tatatattta tgaatgtttt aaatattttg tttacgtttt gttatttaag ttttattata 11940
gttttttttt tttttaagta gataaggttt tattgtgtta tttagattgg agtgtagtag 12000
tgtaattata gtttattgta attttaaatt ttcgggttta agtgattttt ttaagtagtt 12060
tagattatag gtatgtatta taatattcgg ttaatttttt tattttttgg tagagacggt 12120
ttttattatg ttgtttaggg tggttttaaa tttttggttt taaaggattt ttttattttg 12180
gttttagagt agttgagatt atagatatga gtaataattt ttagttttat tatagttttt 12240
tgaagtagta attattttta ttttatttta ttttaattta ttttatttta tttttttgag 12300
atagagtttc gttttatcgt ttaggttgga gtgtagtggt gtaatttcgg tttattgaaa 12360
tttttgtttt ttgggtttaa gtaattttgt tttagttttt tgagtagttg ggattatagg 12420
tatgcgttat tatgtttggt taatttttgt attttttgag tagatatagg gttttattac 12480
gttaattagg ttggtgttaa atttttgatt ttagatgatt tgttcgtttt ggttttttaa 12540
agtgttggga tattataggt gtaagttatc gcgtttggtt ttattatttt tttgtaatag 12600
atgaggaata tgaagtttag agaggttgga taatttattt aggttatata gttggtgttt 12660
aaggtttgtg ttttttttgg ggttaggtta ggagtgagaa tagttgggtt attgatttat 12720
aatgattggt agttttattt agttattaga ttagggtatt tttgaggaaa ggatttatgt 12780
tatatttatt attaaatgag tttgggaaga gttggtgttt agtaaatatt ttttttataa 12840
gtagattaag tttatttatg tttagaagta gggggaaagg tatttttggt attaggaaaa 12900
gtttgggtaa aggtatggtg gtaagatagt tttttttaga gaagttaaga atttggtgta 12960
gttggatgtt aggatgagga tgggaggttg gcgagagttg ggaggttagg ttttgagggg 13020

ttttgaatgt taagtagtag tttttggttt tgattaggta ggtttaggga ggttgtttat 13080
aggtaggaaa agattattaa ggtggtaatt gtagtagttt ttattattga ttattgatta 13140
tagagtgtta gttttgggta gaacgtgtga tatatattgt ttgatttaag tttttagtta 13200
atattgggag gttgatggga gaggagaggt taagtgattt gtttaaggtc gtaaggggag 13260
tgatatagtt tggagtttat ttaagtttgt gtagtgtttt tggtaatggg ttgttttttt 13320
tagaatttag tttaggggtt ttgggatttt tattttgtta ggttttagtt ttgattttgg 13380
gtgaggatcg ttgggtttat agtaggcggg gtatttagta tttttggttg tagaatggtt 13440
tttttttggt tttttgtcgt ttttgggttt ttgttgggtt gttgaaggtt agttgatttt 13500
gggttttaga cggtttttgt tgtgggtggg ggtattgaga gtgagtttat gtggagtttt 13560
ttaaggttgt ggggttttta agtgttgtcg atgtaggttg tttttagaga aggtttagga 13620
gaacggtaga tcgaggaggg gtttggtttt agagttttgg aatttaggtg tttagagttg 13680
ggatttggga ggtttttggg ttttttaata ttattagggt ttatagggat ttattaggtt 13740
aacgtttttt aaagtacgga cgaggtggat gaagttagta gttttaagta aagggttttg 13800
tgtttattgt atttaataga ttttttgagt tgaattttaa tattaagttt gttgttttgt 13860
tgtaggattt tttaggattt ttaaaacgta aatgtgtatt ttgatttttt aagtagggat 13920
atagtatatc gtgtttttta tatggatttt ttttgttaga gtgttttatt ggagtttatt 13980
ttgggaaata cggtttataa gtataaatat tgaggtttaa agaggggtaa tgaattattt 14040
aagattatat aataagttaa tggttaagtt tgaattagat attaaatttt ttaagtttta 14100
ggttattata aaattgagtg gttttttaat tttttattag gaattttttg tatggatatg 14160
ggattttata atataataaa tgggtttagt aattatatga aataatgatg gaaaattttt 14220
attatatgta gcgtatttag aaaggtttta gttaatagta tgtataatta ttgtgattat 14280
aagaatttag tattttttat ggtttttggt acgtagttgt ttagtgttga atgaatgttg 14340
aataaagatt aataatttat agttgtgtga gaattaaatt aggtggtgga ggtgagtttt 14400
gtgaattgta tagttgtttt gttagtataa ttattttaaa gtttaggtag gatggggttg 14460
tgattttatg ttatgaatag ggtttgaggt ttagagaggg acggtgatgt gtttaaggtt 14520
atatggggtg tgagggggat ttattgtttt gttagttgat agtttgggag ttttttttgg 14580
aaggagttag gaaattagga ggtaaagttt attgagggaa aggttggggt tagaagggcg 14640
ggtggaggtt atgtggtttt tgataatttt gttttttttt ttatcggtta tggttttttt 14700
atggtgtgta gg 14712

<210> 266
<211> 14712
<212> DNA
<213> Artificial Sequence

<220>
<223> chemically treated genomic DNA (Homo sapiens)

<400> 266

tttgtatatt atggaaaggt tatgatcgat ggaaggagag atagggttgt taggggttat 60
atggttttta ttcgtttttt tggttttagt ttttttttta atggattttg ttttttggtt 120
ttttggtttt ttttaaaaaa ggtttttaaa ttgttagttg gtagagtagt ggattttttt 180
tatattttat gtgattttgg gtatattatc gttttttttt gggttttagg ttttatttat 240
aatataggat tataatttta ttttatttaa gttttaggat aattatatta ataaaatagt 300
tgtatagttt ataaagttta tttttattat ttgatttagt ttttatatag ttgtgaatta 360
ttagttttta tttaatattt atttaatatt gagtagttac gtgttagaaa ttatgagaaa 420
tgttagattt ttatgattat aataattata tatattatta attgaagttt ttttagatgc 480
gttatatgtg gtaggagttt tttattattg ttttatgtga ttattaaatt tatttattat 540
gttgtaaagt tttatattta tgtaaggaat ttttaataaa ggattaaaga gttatttagt 600
tttgtgatga tttggaattt gaaaaattta gtatttaatt tagatttggt tattaatttg 660
ttgtgtgatt ttagataatt tattgttttt ttttgggttt tagtgtttgt atttataaat 720
cgtatttttt agaatgggtt ttagtgagat attttgataa aagggattta tgtggggaat 780
acgatgtatt atgtttttgt ttggagagtt aggatgtata tttacgtttt aaaggttttg 840
agaagttttg tagtaaagta ataggtttgg tgttaaaatt taatttaggg agtttattga 900
gtatagtgag tatagagttt tttgtttgag gttgttgatt ttatttattt cgttcgtgtt 960
ttgggaaacg ttagtttggt gggtttttat gaattttggt gatgttagag gatttaagag 1020
ttttttagat tttagttttg ggtatttggg ttttaagatt ttaggattag gttttttttc 1080
gatttgtcgt ttttttaggt tttttttgag ggtagtttgt atcggtagta tttaaggatt 1140
ttataatttt aaaggatttt atatggattt atttttagtg tttttattta tagtagaggt 1200
cgtttggaat ttagagttag ttggttttta gtaatttagt agagatttag gagcgatagg 1260
gaattaagag ggggttattt tgtagttaga agtgttgagt gtttcgtttg ttgtggattt 1320
agcggttttt atttagggtt agggttgaga tttggtaagg tagagatttt agaatttttg 1380
ggttgagttt tgagggaggt agtttattgt taagggtatt gtataggttt gggtagattt 1440
taggttatat tatttttttt gcgattttga gtaagttatt taattttttt ttttttatta 1500
attttttagt attaattgag ggtttaaatt agataatgtg tgttatacgt tttgtttagg 1560

gttgatattt tgtggttagt ggttagtgat ggaagttatt atagttatta ttttggtggt 1620
ttttttttat ttgtgggtag tttttttgga tttgtttagt taaggttaag ggttgttgtt 1680
tgatatttag ggttttttaa agtttggttt tttagttttc gttagttttt tatttttatt 1740
ttgatattta gttatattag gttttgatt tttttgaaag gaattgtttt gttattatgt 1800
ttttgtttaa gttttttttg atgttaggaa tgtttttttt tttgtttttg gatatgaatg 1860
aatttgattt gtttgtggaa gaagtattta ttaagtatta atttttttta gatttatttg 1920
gtaatgaata tgatatagat ttttttttta aggatgtttt agtttaatgg ttggataaag 1980
ttattaatta ttataaatta atgatttagt tgtttttatt tttggtttag ttttaggaag 2040
agtataagtt ttggatatta gttgtgtgat ttaggtaagt tatttaattt ttttgggttt 2100
tatgtttttt atttgttata aagaaatgat aaggttaggc gcggtggttt atatttgtaa 2160
tattttagta ttttgggagg ttaaggcggg tagattattt gaggttagga gtttgatatt 2220
agtttggtta acgtggtgaa attttgtatt tatttaaaaa atataaaaat tagttaggta 2280
tggtggcgta tgtttgtaat tttagttgtt taggaggttg aggtagaatt gtttgaattt 2340
aggaggtaga ggttttagtg agtcgagatt gtattattgt attttagttt gggcgataga 2400
acgagatttt gttttaaaaa aataaaataa aataaattaa aataaaataa aatagaaata 2460
attattattt taaaaggtta tgatgaggtt gggggttgtt gtttatgttt atgattttag 2520
ttattttgag gttaaagtag gaggattttt tgaggttagg agtttaagat tattttgggt 2580
aatatagtga gagtcgtttt tattaaaaag taaaaaaatt agtcgggtgt tgtggtgtat 2640
gtttatagtt tgaattattt gagaggatta tttgagttcg ggggtttgag gttatagtga 2700
attatgatta tattgttgta ttttagtttg ggtgatatag tgaaattttg tttatttaaa 2760
aaaaaaaaag gttatggtga gatttaaata atagaacgta ggtaaagtgt ttaaaatatt 2820
tatagatgtg taattttagt aatttgggag gttgaggcgg gtaaattatg aggttagaag 2880
attaagatta ttttggttaa tatggtgaaa tttcgttttt attaaaatat aaaaaattag 2940
ttgggcgtgg tggtgcgcgt ttgtagtttt agttatttga gaggttgagg taggatttgg 3000
gaggtagaga ttgtagtgag ttaagattgt gatattgtat tttagtttgg cgatagagtg 3060
aggttttatt ttaaaaaata aaataaataa ataaaaaatt tatagatgaa gttaagtttt 3120
tcgtttgttg tttagtaagt attggattag acggagtgag aaggtttggg tttttattta 3180
agtttgtttt ttatgtgttt gtgtaaattt gggtaaatta ttgttttttt ttgggtatta 3240
attataacgt gaggattttt tagggttttg ttaaggttga atgatatgtg ttacggtaag 3300
tatttaatag gtttttttat ttttttttgt tttttacgtt ttgttatgtt ttatttattt 3360
tataattttt aaagatagaa atttttttag agatttagaa agtagggttt ggtgtttgtg 3420
gttagggcgg cgtgattcgt tttgtgtaag attaattgtg gtgtttaggg tagttttttt 3480
ttattgttat tttttaaatt atgagtcggg aaatgtttgg tgtgtttaga tgttagtatt 3540
cggtataggg tgggttatat agtatttttt agacgcggtt ttttgattta aatggaattg 3600
ttttggtttg ttttggtttt atttttttgg ggaatttgtt gttgaatttt gttttatttt 3660
ttgggtgggt tttgattata tagaggtaaa cgttattatt ttagtgtgtt tttcggttgt 3720
taatagtttt tttattttta tttagtaaat tttgtttttg cgtgtagtgt cgttatgggg 3780
taaatagttt tatatgggta tggttttttt cggttgatag agttttttat tttatatttt 3840
agagggtagt tcggagggga tgattagttt tgttttaggg gttgtgtttg tttagtattc 3900
gttagtaggt ttcgttattt tttttggtga ggttgttagt ttttgtaggt attggggtta 3960
tttttttttg ggttttattt ttgttttgtt ttgagcggtt tttatttagt tcgttttgag 4020
tttgttttgt cgtttgtttt cgtttaattt ttttttcgtt ttttttgagt tagttttttt 4080
tatttttaaa tgagaaggat aacgtgggcg atagtatatt agcgtatggt ttagtagtag 4140
gtttatggta tatggtgggc gtttaggtag tgaaggtagt tttttgtgtt tttttttggg 4200
tttcggttta agttacgggg tgggacgatg ggggttgagt ttttttttgg ggagtttagt 4260
gtagatagtt tgtgagtgtt gggtgttgta gttttaaagg aagggagttt ggtgtttttg 4320
gtgtttgggt tttttaggtt gggattttgg ggtgagtttt ggagttattt tgggttttag 4380
tttttttatt ttttttagaa ggtttaggga gaaggtgttg ttagagtatt ttttagacga 4440
ggtttagatt ttttaggcgg tttaagggta aatgagaaat agatttagag gttttggtgg 4500
gtttagagta gtgtgaaggt ttaggggttc gttttggagg ttgacgttgg gataggagta 4560
ggttgttatg cgtattaggg aggttattcg tttatagggg ttgtaggtag agattatagg 4620
cgggaggtag tagattttat tttcgaggag ggttatgcgg ttatttggta attagtggta 4680
atgatcgttt ttaaataaat tttttttggg gaggtaatta aggattagga tgggaggtgg 4740
gggagttttt tttgtggata ttttgttcgt tttattttaa tagattttta attggaaata 4800
tttcggttta tttgggggtg ggggtttggg ggagagggtg gaagaaagga taggagggaa 4860
tgtagagttt ataggcgagg ttcgagttta gggttttatc ggggtggtaa ggtttttttt 4920
tatttaggag ttgggggttt tgttgggatt ggttggggta gagatttggg tttaagtttt 4980
ggtttagtag tttttttttt gtgagatttt gggtaagtta ttgttttttg ttttatttta 5040
atttttttat ttatattgtt taatgtttaa gaataatgta ggttttgata ttggtaatat 5100
aagttgttaa atgattttat tgaggtattt ttttggtggc gttagtaatt ttatttttag 5160
gtcgttttag gggattcgtt tgtgtaaggt atttttaaag aagggttttt tttttggatt 5220
tgtattttat agtatttttt tttttttagg tataggggtt aggttatatg atgaatttta 5280
ggttagagat ttggtagtta ggtttaggtt tttttaagtg atttgggttt taagtttttt 5340
ttttgtagtt ttgtagttcg gtcgtttacg gtgttgagtg gtattgttag attattttta 5400
ggtgaggttt cggtagagtt gggtttagag ggttttagaa ggtgttttta ttagcgttat 5460
ttagaatgta gtttggtagt tgtttattta gggagagaga tggggtgagg ttaatagatg 5520

gcgatgagag agattttgat gtttatattt gtgtttttgg gatgaggagg atgttgatga 5580
gggttgcggg ttggtttagg gatttagttt tgttttggaa tttttatgtt tttgtatttg 5640
tggattttgt tgattttgag gtgggagaat aattgagtcg aggagatttt gtttttttaa 5700
ttagattttt gttttttttt ttttcgtgtt tttttgggtt tttttgggtt ttattttatg 5760
ttggtgttta tgtggtaggg aggatggagt aggattttag gttttaggtt atttaggtaa 5820
gtttagtttt tttttaggtt ttcgtttatt tttttggggt aattttgggg aagttttttt 5880
tttacgattt tttaattaat tttttaatta ggtattaggt ttaggaatcg gggagaggtt 5940
taggaatttc ggaagtgttt tttttttatt agttatttta gtttggggtt gtagttgttc 6000
gtttaggaag aagaagtttt tggggtttta tagtttgaga gtttttttgt tttttttttt 6060
ttgggttttg ttttaggatt ttagagtgaa gagggattag tttagttttt gtttttattg 6120
tatagataga gaaatggagg ttgatgaagg gtttgagttt tgtagattta gttttgtttg 6180
gaaatgatat tgggtttttt ttatatttga ttgtttagat tttagagatg gaagtttgtt 6240
tttttggttt tatatatgtt gatttatttg tgtagtttat tttgtaaagg taaagtttta 6300
ataggtttgg gtttgatgtt ttttagttaa gaaattaaac ggtagttgtt atagtattat 6360
gaatttttat tgtgtaagtg ggaaattgaa ggttcggaaa gggtagggtt tgtttagatt 6420
tgtacgtagt taattagagg tagagataag ttagatttta ggttttttgt attttgaggt 6480
agggtttttt ttatttttat tttagagttg aggaaattga ggtttagaga agttaaagcg 6540
attgatttgt agaatttagg ttgggttttg tagttttagt ttggtgtttt ttttatttta 6600
ttttgtagcg ttatggtttt ttagagtttt ttggttttat tttcgtggga gtggtaggtg 6660
tgggattgtt tagggaaggg ggcggttttt atggatgttt ttaatcggtt tgggtttttt 6720
tttttatttt atcgttttcg tggtttatag gagagtggga ttttttttaa attttttttt 6780
tatttaggaa agttaagaag ggggtggata gtttagattt gggatttcga atatttgagg 6840
ggttgggagt tttttggtgt ttggtttggg tggaggtaag gtagggaagg atttttaggt 6900
tatttttggg taggtgtttt tatgggtagt taggttttga tttttttttt ttagttttta 6960
ttttattagt ttagtttgcg ttttaggtat ttattaggtg agtattttag gttattgggt 7020
tatgttggtt tttagttttt gtgttaagtg ttttttttat ttttagttta ttttttaaag 7080
tttaggttag cggaggtttt tgggtgagtt ttcggttttt tagttttatt gaatattttt 7140
ttgtttttag ttgtgttggg gttttttgtg tttaatagtt tatagagtta ggtagtttaa 7200
tttaaatttt gttttttatta tttttgagtt gtgatatttt gttaggtaga gtttttttatt 7260
tttgtgtgtt ttagtttttg tttttttttt tgtgtaacgg gggtaatagt agtatttatt 7320
ttatagggtg gttgggatat ttaaatggaa taatatatat aaggtattta gaacggcgtt 7380
tagtataaag tgaggttatg tgtgtgtaga tatatatgta tatatatata gatatatgta 7440
cgtggtttat atacgtgtat ttatatatat atatgtatat atagatcgtt gttattttta 7500
aattttattt ttttttttat tttttttttt attttaaatt ttagtaagat tttaattttt 7560
tattgttaga ttattagttt tttataggag tgtagtatga tttaatttat atgatttaag 7620
ttttcggagt agcgtttggg gtatttagga gtaggttttt gtggtgtttg gtttttttag 7680
gttttttcgt atgcgttgtt cggtttttag gtttttgttc gtgttgtgtt tttcgtttag 7740
gacgtttgtt ttgtatttcg tttttgatta tttaaatttt ttttgttttt ttaattttgg 7800
tttatgtatt ttttttagat agaattgatt attttttttt tgggttgtta tgtttttttt 7860
gttaggatat agattatagt ttattttgta tttttatatg tttatttttt ttaagagttt 7920
gggggtttat ttaaggtata taatgagttg tatttatttt tgtggtagtt tgattttgta 7980
gtggggatag aggatatgga cgttaggttt agttagtcgt ttattttatt ataggttagc 8040
gtgggggttt gcgtgtatta tgttttttgt agggtttcgg tgtttttatt gtggagtagg 8100
gatgatggta gtgattttat aggattttta tgggcggtaa atgatattta agtttattgt 8160
aaggtttttg acgttttttt agttaatttt tttttttttt ggattttagt atttttattt 8220
gtaaaatggt ggcggtggat gagagagttt ggattcgttg atcggaggtt tttgttagtt 8280
ggaatagtgt tgggtatttt attgtaaaag gaaggtggtt ggttttttat agaatttttt 8340
agggatagtt gggaaaagtt agtgtttagg gttgggagat tgttgagatt aggttttttg 8400
taaataagaa aggtaggaag acggggtttt tttttcgaat cgttaattaa ttaataattt 8460
ggtttgagaa ggggtagggt tagtgatttt tagattgttt gttggcggcg tagttattat 8520
tattttgttt tttaattaat ttataaattt tttgtagcgt tttttttagt ttttataatt 8580
tgttttttgt tgttttattt gtaggttttt tgttttggtt ttagatttta gatttttgtt 8640
ttgttttgga tttttttttt tagggagttt ttttgtttat agcgtttttt ttattgttcg 8700
aattagttaa ttttcggtag cgttttagtt attagaggta tagtttcgga aaagtttttc 8760
ggtgttttat atttgtgttt ttattaggtg ggtttttttt ttttaggttt ttagattttc 8820
gttttggttt tttttcgggt atttggatat ttgtggagtt atttatagtt tgggtttatt 8880
ttttttaatt tagagcgtag ttttttcggg aaggggttgg tgtttatttt tttttttatt 8940
atttttagtt ttttgagtag gtattttttt tagttcggta ttaagttttt tttttttttt 9000
tatttttgcg tgaataattt ttatttaaag gaatgttttg tatatgtgta tagtggtatt 9060
tgatagtttt tgaggttttt ttaggagttt ttttttattt gatttgaggg aggtggggag 9120
ttttattttt ttgtgataga ggaggataag gaggttttaa gaagtagagt gatatttttt 9180
taaggtcgtt taggtggagg ggttaggagt gttttagcgg aattttcgtt agggttaagg 9240
agattgacgg ataaggatcg tttttgggtt ggtcgttttg gattgcgttt tttgaataga 9300
atgttttttt ttatttgttt ttgcggaggt ggttagaaat ttcgtggttt ttgcgttatc 9360
gttagttgta taggattagg cgtttttatt gtggtttacg ttcgttttat tttttttcgt 9420
aagttttcgg gaggtaggga tattttatag aggaggaaat agaggttgag ggatgatttg 9480

tttacgatcg ttaagttagt ttaggttttc gggttttggt gcgttcgttt tatttagggg 9540
attttaaaac gtttttcgtc gttttagttt tcggtttgtg cgcgtggacg cggggttttt 9600
atttatagtt atttgatgtt atagtatacg tcggtttgga gcgttagggc gaagagtagc 9660
gtttcgtaga tttgcggtcg cgtttttatc gtcgcgcggc gggcgcgttc ggggttatat 9720
ttaggaggag tcgcgtcgaa gtttttcgtt tgtacggtcg tcgttggttt tgtacgtcgt 9780
ttgtagtcgg ggagagcgga ggcggcgggt taaggcggcg cgcgggcggg ggaggcgttt 9840
tatttaaatt ataaaggagg ggtcggggtt cggggaggtc gagcgcggcg ggcgtttttt 9900
ttcgtttttt tcggtcgggg gacgcgtttc gcgtttggtt cgaattaggc gcggtcgaag 9960
gcgtgttttt tagcgtggtt tcgggtttta acgcgttttc gagttttttt ggtcggggta 10020
gtcggaagga gggataggag gcggtagaat cggagatttt aggagagatc gggtagatat 10080
agcgagaggg agaaggacgt ttagaggata aggttgagag ttacggagat agagggggaa 10140
attcggatag acgtagattt gggacggaaa ggagaggaat gaggaattag gagtttaggc 10200
gaggtagtag agtcgaggat tcggagttgg ggtttatttt tgtaatattg agtattagtt 10260
aatagggttt cggtaatttt tttttttaaa cgtgttttag tttatttttt tagtaagttg 10320
gggtaattcg ggtataattt cgtttttata aagaacgttt tttggtcggg tagggtggtt 10380
tacgtttgta attttagtat tttgggaggt cgaggcgggc gggttatttg aggttaggag 10440
ttcgagatta gtttgtttaa tatggtaaaa tttcgttttt attacgaata taaaaaatta 10500
gtcgggcgtg gtggcgggcg tttgtaattt tagttattcg ggaggttgag gtaggagaat 10560
cgtttgaatt cgggaggcgg aggttgtagc gagcggagat tgcgttattg tattttagtt 10620
tgggcgataa gagcgaaatt tcgttttaaa taaatagata aataaaaaaa gttttttaat 10680
acgatcgtat ttgttttttt tagtatttcg tgagataggt attatcgttg ttttatttta 10740
tagatgagga aattgaggta tagaggtggg gtttagttag ggagcggtaa gtcgggtgtt 10800
cggcgaaatt aggaaatggg gtttgttcgg ggttttgttt tttcggtgtg ggaaggggag 10860
gatgggaaac gggatggtag ggttttttgt atagtgagga tttcgagttt ttaagttcgt 10920
ttttagtcgt tttattttgg agggtgggag tggaagtatg cgggttggga ttgggtagtt 10980
tcgttttagc gaagcgggga agttcgcggg gttggcgcgg tgttgttttt ggtttttagg 11040
cggcgttttc gggtcgcgga ggagaggttg tttcgttcgt tcgtagttcg cggagtagtt 11100
attttcgttt tttttttttt ttattttatt tttattttat taattttttt tttagttttt 11160
tttaaattta ttaatatttg gcgttcgttc ggagttagat atagttttag tttttttttt 11220
ttcgggagga gtagagataa agggattttt ttttaagggt ttggggagtt ggaaaaggag 11280
gcgtttacgg tagtgggggc gttggagtag aaaagggacg tttttatttg gttttgtgag 11340
tttgggtaag tttttagtta ttttagtttc ggtgtttttt tttataagaa cgaggaatgg 11400
ggtttcgtcg tgcgtttttc gaggatttcg tttttttgta gtttgcgggg tttagcgttt 11460
ttttgttatt tgtatcgcga cgttatttgc gtggcgcgtt tttgcgcgtg ggtagttagt 11520
acgtagagat gggaagtttt attgtttttt ggagtttgac ggtaaaattt ataggaaggc 11580
gaggagaagt ttggatcgta tagttttttt ttttcgtaga gtttcgtagt tagcgtttag 11640
tataggggcg tttgcgataa atatgtcgtt aaggttgggg gttttagagg ggttttcggt 11700
gtaatttagt ttcgtttttt tatttagttt gtatttaaat tttttgtttt tttggttttg 11760
taagtttttt gtatttttta gaacgagaaa aatattcgtt ggatatggag gggggaatta 11820
aaattttagt tatcgttttt ttttttttta aagtgcgtcg tttttcgaag gtattcggta 11880
tttttgggtg gggagggggc gggggtagta aaaggacgga ggcgcgcgga tttatagcgg 11940
gggatttgtg tttagtttgg ttttttaggg agaggaggag tttagcgtcg ttaatagatg 12000
gttataggtt cggtttgtgg aggattttgg aaagtcggtc gttgggatta ggttacgtcg 12060
gcgaggattt gcggggtttt tttttatttc gacgttttac gggtatcgtg tatcggggtc 12120
gattgttcgc gcggcgcgta aggtcggtac gggcgggttt cgaagttttt cgttacgtag 12180
tttacggagt tgatttcgtt aattagggac gtcgagcgac ggttttaagg ttatttgcgg 12240
ggcggggcga ggtcgggttt gttattcggt tttgatttta gtcgtgaggt tttcgagaga 12300
cgtttgtttg gttgcgtacg cggcgtcggg ttattcggga gacgggttgt tttggaagtg 12360
ggaagggtgg gtgggagtat tcgagatttt ttttgttaat tttttggagt cggattttgt 12420
acgtttaggg ttttgaggga tatcgaggta ttttagtttc gggggtggga ggcgtggagt 12480
tcgtgcgcga gtttagttag atgtttcggg cggttgtaga tgtgtttcgg ggtcggggat 12540
aggatttttt ttttttttag tttttagttt ggagtttagg ttagtaaata aattcggttt 12600
tttttttttt ttcgtcgttt ttgggattcg tttttaaagt taggtttttg tttttatttt 12660
taatttatta ggcgttgtcg tatttttttg ttttttgtta tttttttttt tcgtttatta 12720
tatatatttt ttgtcgcggg atttaagttt tttattttcg ggggtggttc gggtatgatt 12780
ttattcgtcg aacgattggg aaggaggggg tcgtttgggg tgcgggggta gaagaggggg 12840
gtcggtttgg gaaagttcga ggagggtcgt aggaattgta taatttatta tcgtttcgcg 12900
tttttttaaa tttcgcgttt ttaggttttt aaggtttcgt ttcgtaaagg ggaatttttc 12960
ggagtgttgc gggggaaagg tatggaggag cgtttggttt gtttattcgg gagtgggtgt 13020
ttaagggggt cgtggcgggt gcgagaagcg tcgttttttt cggattcggg cgtttttagt 13080
tttggcgggt tttcgttgag cggttgttcg ttgttcgcga ttattgttta tttttcgggg 13140
cgttcgttag gcgcgttttg cgtgttcggg tgtttcggtt tgtttaggga gcggcgagtg 13200
cggtttttag cgggttcgtg cgtatttcgg ttgggcgagc ggtgaaggcg tttttttcgt 13260
gaagcggcgc gagaaggacg tcggggattt tggtaagttg cggtcggttt aggattcgcg 13320
cggagttagg cgcggatttc ggttgttttt tgttgcgttt agttttcgtg gtttgtgttc 13380
gtcgttcgtc ggagtttttt ttttcgtttg gcgcgatttt cggcgcgagt tttttaaatt 13440

gttttataag gcgggcggta gttttgattc gtttacgtta gcgcgtcgta attttatcgg 13500
gtggtttcgg tcgcgcgcgg ggggcggggg cggggttttc gcggacgtta ttgatcggcg 13560
gcgttcgtcg ttttagagaa tttagtattt tgtcgagtta taaaggggga ttcgtttatt 13620
atagttggtg tttattaagc gtgcgtgttg cgtgtttgcg atacgcgtgg ggttcgggta 13680
aggtttattt cgaatcgtcg tagtaattag gtaaggagtt ttaattggta gggggaagtt 13740
agatttgggt tttagtttgg agtcggggta tttttaggtg ttggtcgcgt agcgtggtta 13800
ttgtagacgt ttgaggtttc gaaatttgtt ttcgtgtttt agttttggtt tttcgggggg 13860
ttattttttt tggtttttgg ttttagttgt ggaaaaggaa ttaacgattt tgtaagagaa 13920
agagatacga ggatttcgcg ttataaagtt cgttgtaaat tttaggtgtg gagttgggcg 13980
gcgttttagt cgttggggtg attgttgggg agtgttttgg atatagggga gggggagtag 14040
aggaggattg ggttttttaa gttaaaggag tttagagagg tatttttttgc gtcgagtagt 14100
ttaggcgtgt ggtgtttttg gattttttta tttggttttt tttgagtaga ttagttgttt 14160
atttataaat atgattgcga tttttaattg tttatcgggt gttgtgggtt tatttatgtt 14220
tttttttttt tttaggggtt ttaattttag gacgttttta aggataattg gtttattagt 14280
agaaagggaa atggagattt agtgaggata cggagtttgt tttaggtttt ttaaaatgtt 14340
tgatattttt tttttatttt tttgtagttg tagatgaagt ggtattttta ggttgtttta 14400
tatttagtta taggtatttt tatatataat ataaatattt gggtttatag tgtttatagt 14460
agttttttag ttatattagt atttttatat agttagtttt tatatatagg ttggggtata 14520
ttatttcgta tatggttata aatatattcg ggagatatta ggatatatta tttgttttta 14580
tatatagtag tatagaggtg tgaaatggtc gtatatatat tatgaagggg aggttgtata 14640
tagattattt gcgcgtatat agtttaggtg taaatatgcg tgagttttta tgtatatata 14700
gtttttaagt gt 14712

<210> 267
<211> 7497
<212> DNA
<213> Artificial Sequence

<220>
<223> chemically treated genomic DNA (Homo sapiens)

<400> 267

gttgaaaatt tgtggagatt ttgaatgata ttatgttttt ttaaggaggg tggagttggt 60
taggtgtggt ggtttacgtc gttaatttta ttattttggg aggttaaggt gggtagatta 120
tttgaggtta ggagttgaag attagattgg ttaatatggc gaaattacgt ttttattaaa 180
aaatataaaa attagttagg cgtggtggtg ggtatttgtt attttagtta tttaggaggt 240
tgaggtagag agaattgttt gaattaagga ggtagaggtt gtagtgagtt gagattatgt 300
tattgtattt tagtttgggt aatagagtga gattttaata aaaaaaaagg tggagttttt 360
ttttggatag taaaaaaaag tattattggg aaatcgtgat tttgtttagg gttggtttta 420
gatttatttt aggttgtttt gtttttattt tgtttttatt tatgtaatgt ggtttttatt 480
tttaaggtta agtgaagaat aagaagtaag agaagagttt tattttattt ttagagagta 540
tttttattga ttatagattt ttatgtggat tttttttttt ttttagcgtg ttatagatgt 600
tattttattg ttttttgatt tttattgttg ttgataagaa gttaggtgta atttatattt 660
ttgttttttt atatttaatg tatttttttt ttttggttgt tttagagatt ttttatttat 720
ggtttaaaat agtttgatgt gtgttagtgt gatatgttga gtaattattt ttttggggat 780
tttttatttt ttttagattg gtaaatttat gttttttatt atttgggtta aatttggggt 840
attattttta aaatattttt tgttttattt tttttttgtt ttttttttgg aattttattt 900
tagaaagtat aaattttgta gattagataa ttgtattaac ggggttttga atttttattt 960
tttttttttg gtttttattt ttttgttgag attttttatt tgtttattgt ttatttgttt 1020
attttatttt tttttaaata tttgaatata tttataatag ttattttaaa aatttttttt 1080
ttgttaattt tttttatatt tgggttattt taggattagt ttttattgat tgtttttttt 1140
taagtataga ttatttttgt gtgtttttta tttgtttata tttgtttgtt gggtttgttg 1200
gttggtttgt ttagaaatag agttttgtta tgtttttttg ggttggtttt gaatttttgg 1260
atttaagcga tttttttttg ttagtttttt aagtggttgg gattatagac gtgtattatc 1320
gtgtttagtt tattaaattt tttattgtta attatttatt gtagagaatt tgtattatgt 1380
tgtttttttt ttaatgggtg ttgttttatt tttataaatt tttttggttt tgtttgattt 1440
tttattattt tttgttatag tatgtgttat ggagaaataa tattttgatt tttgttttaa 1500
gatatgtttt ttagtttgat tattgttttt aaggtaaggt ttttttagta ttttaattga 1560
atttttgata tgtttagtga ggtttttttg ttttgttgtg atggaatttt aatgtttttt 1620
aataatgtat tatatgtagt aggtaatttt tgttaggttt tgtagagttt tgttatgttt 1680
atatttagtt tagttgttgg tttagattgg tggtgaattt ttatgtagat ttgcggggga 1740
gtttttgttt tatagttatt tatttttaga aattttagtt gttttaatag tttaaaattt 1800
tagttttttt ttttttaatt gaaattattg ttttgtttgt attttatttt tttatatttt 1860
agaaaaatgt tttttttttt agtagaaaaa tttggataat tgtaagatag ttgtggagta 1920
aaattaatgt gttttttttt ttttaaggat tatagtcgtg tggggtttat tgtttaatat 1980

ttgaaaagga gagttattta ggtatttgtt atttttttat ggttggaagt aaaaaatttc 2040
ggtgtatatt aatttaaagg tatattgaga tttaaacgat tgatattata atttattttt 2100
tggttagtga gtggttattt taggagttat tgtttgagga agaagttagt ttagattgga 2160
agataaatga ttatttggtt gagaagtaat tttttatttt taaagggtgg agatggttta 2220
gttgtaaatg agttttttta tattgttttg ggagattaga taaataatgg atttaattaa 2280
gaaaatagat tgatttattt ggttatagtg ttatattgaa gttaaaagta aattaatatt 2340
aagttttaga aagagaattg ggttttgtat attgtaaatg aattttatag ttaattttga 2400
gattttttat tgaagaaatt aaaaattaga tgtttaaatt aataaattta aagatatttt 2460
gatttttttt ttttttattt tattatttgt tatagttagg ttgaagttat atgtaattta 2520
ttttattatt tattaatttt tttttaggat ggtggttttg attgtaaaag aagtaaggtt 2580
tgttgtaatt tttttttttt ttaatttttt tgagtatttt ttaattatgt ttattagcga 2640
atattttatt tttaaagcga aggggtttat cgagagaata ggtatagata aggagggagt 2700
aaaggttttt ttggtaggaa aataagttat attggggtta agtaatggtt tataagtgaa 2760
aggggtggag aatgggaggg aaatggaatt atagatttgg aagagataag ttattgagtt 2820
tagacggttt ttagataggt taggaagggg tgtttgggtg gatttttttt ttatatttgg 2880
atttagtttt attcgaatat ttttagtttt ttgtttttta ttaaaattaa tttttttttt 2940
gtgagatatg tggtattttt tttttttttg ttttaggatg aagttttttg gcgtggattt 3000
aattagtttt tggggatggt ttattttttt tttaggaggt ttgttttagg tttaggttat 3060
tttattggtt ttattattaa ttgaagggta ggttttttgg ttatttatta attttttggg 3120
cggagagtaa gggtttgtaa gttgatggtt atattttatg tatttttaat attttttagt 3180
tgtgaggata cgatgtgttg agatgggtgg aatttgagtt ggagtttttt aggtggtttt 3240
aaattataat ttttatgaga gaattatata attgtttatt ttttttaata tatatattta 3300
tatttttatt ttatttacgg tagaagtgaa attttggtaa tgtttttggt ttttcggagt 3360
taattatttt ttttttttta agtgtttgga tttattttaa gaaaatgcgg gataaagaag 3420
ggtggaggta agttttcgtt tatttttttg ttttacgggg gaaggaggtt tgtgagtata 3480
agtatgtaag tatatgagag gcgtgttgtt ttttggtgtt tattatattt tttttatggt 3540
cggcgtgtat atacggcgag tagaaacgtt ttttcgtttc gttgtttgtc gttttacgcg 3600
ttttttttgt atttttcgtt cgatcgacgt agattaagta gaattttttt gggtcgcggt 3660
ttagcgatac ggagcggttt tggcgaggag ttttgttttt ttcgagtcgt gggtggcgcg 3720
gtgtttgttt tttttttttt ttttttcgga tttaaacggg gatgtatttg ggttagtttg 3780
ggaggggtcg gatttgttag ggattagcgt gggggaaggg ggtggcgatg atagtatttt 3840
ttaggttttt ggcgtttttg agtttcgttt cgtttagttt tttatcgcgt tcgttgtcgg 3900
cgagggttga cgttttggtt agtttaggtt cgagggtggg ttggagagag ggagagttcg 3960
ttttttcgat ttgggcggta tttttttttt tacgttttgc gaataattcg ttttttatat 4020
atatatatag gcgtatattt tattttttag agtacgtttt tcgggcgggt agtgagtttt 4080
ttcgttttag gaaaagagta atggaatagt ttacggtcgt tacgagtttt tggttttttt 4140
tttttttcgg tgataaacgg cgcggttata agttagttat tgtttaaaat gttttattcg 4200
cgggtttaag tttttttttt ttggttgggc gggggtttag gtttaggatc gagggttttt 4260
taatttttat aaggcgtata ggttgagcgt ttaggcggta ggaggtgtaa gggcgtatat 4320
tttcggcgaa cgtttggttg tttcggtttt tttttatgtg atttttgcg tttcgtagtt 4380
tcggagagga gttgggtgtg cgtaaaaatt gtattttttt tgggtgtttt ggtagatatt 4440
gttgggttag gtttcgaggg ttatggattt tttaagtttt tttttttatg ggttttgtat 4500
ttaagtttag tattaaggaa ttaaagttaa taggttattt cgtagaggtt tggttttttt 4560
ttaattttgt tttgtataga cgcggtagtt ttaaatttat aagtgttagt tttttatttt 4620
agttttaggg agagaagcga agtaatgagt tgagaattat ttttggattt ttgtatttta 4680
tgtatagtaa tttttttatt ttttggtttt ttttttcgtt tttttatatt gtacgtttag 4740
ggatttgttt gttagcggat ggtttcggta attcggaacg tacgtttcga gagtttacgg 4800
atgttttttg gtttggagtt tttttaaagg tttttgtatt cgcgtgtgtt cgtaattatg 4860
tagcgatgtt tttttttttt cgttttattt tatttttaga tattttttgt tattatttta 4920
tgtattcgtg tttaaaattt cgcgtttttt tttattttcg ttaggcgtag tatttttttt 4980
ttcggttgcg ttcggagggg agtagagcgg acggggcgcg cgggaggcgc gtagagtttt 5040
cgggttgtag gcgttcgttg cgtttgggga attgggttgt gggcgaggcg gttcgggttg 5100
gtttttatcg ttcgttgggt ttatcgtttg aaattttatt agcgagtttc gttattcgtc 5160
gtagacgcga gcggggggcg ggggcgcggc gaggcgtcgg cggtcgtgac gaggcgtttt 5220
cggagttgag cgtttttgtt ttgggtacgt atggcgttcg tatacggagt ttgatttgat 5280
gtagacgtaa gggggttaat atgaacgttt ttttcggtgg aatttggttt tggttttttt 5340
tgtttttgat ttggtttatt ttcgaggtta attttttatg gtggtaagtt tacgcgtacg 5400
ggcgcggggg attttggaga tcggaatggg gatcgtattg gttatataga ttttttttgg 5460
ttgcgttttt tcgcggtcgt agtagtttcg gattttggtt cgaattttgg ttattgtttt 5520
tttatcgttt tatggtgagt tttttttttt tttttagaag attattttag ttttgataat 5580
tttataaaat tatatttttt tttaaatgag agtttcgatg tttggtttat ttggatttag 5640
ttgagcgata gtaaaattag ttaatttttt attttgttga gagacgtgtt agtttggttt 5700
ttagttttag tatagacgtg tgtgtgtgtg tgtgtgtgtg tgtgtgtgtg tgtaaattta 5760
ttttgaaaat tataatcgtt ttttttattt tagtttttat ttcgttaaaa ttgttttaag 5820
gtaaatcgac gggttaattt agttagttag gagttgtaga atttaaagcg tgtgattttg 5880
tattattttt cgttttttta tattttaggt ttattttagt ggggcggaag aatattttgt 5940

tgtttttagt tagagattat ggttttaatg taaagttata gatttcgggg tttgttttta 6000
agatgttatt ttgtttataa tcgttttcgc gaagagtaat tttgttgttt ttttttgtac 6060
gttggtttgt aatttattta tttatttttg tatagtgatg gaatgttgaa ttatattagg 6120
tcgttaaaag ttttaaaggt agattttttt tttatttaga aatcgttttt tatttgtcga 6180
ttttttggtt ttggaaagag atagagttga tagttttgta atttttttgt atttaaaagt 6240
tttagaatag atattaaaat attgatttag agatattcgt tatcgtgggt taatttttaa 6300
agaatttggt aaggggtttt agttaaatta tagattagag tagggaaatt tttaggaaga 6360
tattggagtt ttttaaagga gtaaggtaat gcgttgatgg gaagaggggt ttaatttttt 6420
tttaagtcgg ttttgaatag ggaggcgttt agttttgata ttgttgatat aggattaaag 6480
attgatttat tagattgatt attttttaat aaaatgatta tttttgtttt tatgggtgta 6540
taaatattat ttattgttta ggttttttgt tatattgttt tttagttgtt atttaaatat 6600
attttttgga agttatagtt gtaacgattt tatgattata taaaataatg aaaatataag 6660
aaaaagatat tttttttgtt tgatcgtttt aaaagatatt tatggtagtt tgggttatat 6720
aaaaggtatt tttttaaata ttttacgatt aatagagaat atttgggtag agatgatgat 6780
gtaatttttt gttaaagttt ttttttaaaa agtattttat ggagttatag tattgtttga 6840
tttttttaaa tatttagtgt ttttaataat aatagaaaga atttttttta aaggattttt 6900
ataaaagtta aaaagagtta taaaatggaa atatttaaaa ggttttttag taaggaaaga 6960
atagtttgtt tgtgaggaga gtgtaatatt agatattagc gggtgttgat tagatagata 7020
tagatagttt ttaagatatt ttgttaaata atgggaacga agagttttta aggttagttt 7080
tttttaaagt taagtttaga tatattgtgt tattgagatt tgagtttttt tgagaatagt 7140
tttttatata ttttttttaa gatacgttgt tttttattcg ttatttattt ttttaaatgt 7200
cgattttgtt tttgtgtttt tgtttagagt attatttggt agatttagtt ttatgtttaa 7260
tagatagtta ttatagtatt ttggggatga tttttgtaag attatttatt acgtttgttt 7320
gttttgtcgg tttttttatt aaattaaaaa gtaaatattt ttgtttttta atattattag 7380
aaggataagg aatttatatt tgaaaagtgt tttgaatttt ttagtttttt aggaatattg 7440
agttaggttt ttttgtttac gttgttttta tttttgtgtt ggttggtttt ttatagg 7497

<210> 268
<211> 7497
<212> DNA
<213> Artificial Sequence

<220>
<223> chemically treated genomic DNA (Homo sapiens)

<400> 268

tttataaggg attaattaat atagaggtga gggtaacgtg aatagggagg tttggtttag 60
tgtttttggg agattaagga gtttaaagta ttttttaaat atgaattttt tatttttttg 120
gtgatattag aaagtaaggg tatttatttt ttaatttagt ggagaaatcg gtaaggtagg 180
taggcgtagt gagtgatttt gtagaagtta tttttaaaat gttgtgatga ttgtttgttg 240
gatatgaagt taggtttatt agatgatgtt ttgggtaggg gtatagaggt aaagtcggta 300
tttggggaag tgaatgacgg gtaagaagta acgtgttttg gggagaatgt gtggaagatt 360
gtttttaaga aggtttagat tttagtgata taatgtgttt ggatttggtt ttgggggaag 420
ttgattttag aagtttttcg tttttattat ttgatagaat gttttaaaaa ttgtttgtat 480
ttgtttaatt aatattcgtt ggtatttgat gttatatttt ttttatagat aggttgtttt 540
ttttttattg aagagttttt taaatatttt tattttgtag ttttttttgg tttttataaa 600
gattttttgg aaaaaatttt ttttattgtt attaaggata ttaaatgttt ggaagaatta 660
aatagtgtta taattttata aagtattttt tgaaaagaaa ttttaataaa aaattatatt 720
attattttta tttagatgtt ttttattagt cgtgaaatgt ttagaaaggt attttttata 780
taatttaagt tgttataggt gttttttgaa gcgattaaat aaggagaatg tttttttttt 840
gtatttttat tattttatat gattataaaa tcgttatagt tgtgattttt aaaaaatata 900
tttaagtgat agttgagaga taatgtgata aaaagtttga gtaataaata atgtttatat 960
atttatggaa atagaaatag ttattttatt gaaaaatggt tagtttgata aattaatttt 1020
tgattttgtg ttagtaatgt taaggttaaa cgtttttttg tttaaagtcg gtttaggaag 1080
gaattgaatt ttttttttta ttagcgtatt gttttgtttt tttgaagagt tttagtgttt 1140
ttttaaaaat ttttttgttt tagtttgtaa tttagttgag attttttatt agatttttta 1200
aaaattagtt tacgataacg ggtgtttttg aattaatatt ttaatatttg ttttgaaatt 1260
tttaggtgta aggaaattat agggttatta attttatttt tttttaagat tagaaagtcg 1320
gtagatggag aacggttttt agatggggag ggaatttgtt tttggaattt ttagcgattt 1380
ggtatggttt aatattttat tattatgtaa aaataaataa ataaattgta gattaacgta 1440
taaaggaaag tagtaaagtt atttttcgcg gggacggttg tgaataaggt aatattttag 1500
agatagattt cggggtttgt agttttgtat taaagttata gtttttaatt ggaggtagta 1560
aaatgttttt tcgttttatt ggggtgaatt tggaatgtgg aaggacggga aataatataa 1620
agttatacgt tttaggtttt ataatttttg attggttgaa ttggttcgtc gatttatttt 1680
gaggtagttt tggcggggtg ggggttggga tgggggaggc ggttgtagtt tttaaggtga 1740

atttatatat atatatatat atatatatat atatatatac gtttgtgtta gagttggaga 1800
ttaggttagt acgtttttta ataggataag aaattggttg attttgttgt cgtttagttg 1860
gatttaaata aattaaatat cggagttttt atttgagggg gaatgtggtt ttatggagtt 1920
gttaaagttg aaatgatttt ttgggaaaag agaaggggtt tattatgggg cgataagagg 1980
gtagtagtta gggttcgggt tagaattcga gattgttgcg gtcgcggggg aacgtagtta 2040
ggaagggttt atgtggttaa tgcggttttt atttcgattt ttaaaatttt tcgcgttcgt 2100
gcgcgtggat ttattattat gaagagttga tttcgggggt gagttaggtt aagagtagag 2160
ggagttagag ttagatttta tcgagagggg cgtttatatt aatttttttg cgtttgtatt 2220
aggttagatt tcgtgtgcgg gcgttatgcg tgtttagagt agaagcgttt agtttcggga 2280
gcgtttcgtt acggtcgtcg gcgtttcgtc gcgttttcgt ttttcgttcg cgtttgcgac 2340
gagtggcgag attcgttgat aaagttttaa acgatgggtt tagcgagcga taaaggttag 2400
ttcggatcgt ttcgtttata gtttaatttt ttaggcgtag cgagcgtttg tagttcgaaa 2460
gttttgcgcg tttttcgcgc gtttcgttcg ttttgttttt tttcgggcgt agtcgaggga 2520
gggggtgttg cgtttggcgg gggtggggag aaacgcgggg ttttagatac gggtgtatga 2580
aatgatggta agagatgttt ggggatgagg tgaggcgggg aagggggaat atcgttgtat 2640
ggttacgagt atacgcgaat ataggaattt ttagggaagt tttaggttaa agagtattcg 2700
tgggttttcg gagcgtgcgt ttcggattgt cgaggttatt cgttggtaaa taagtttttg 2760
agcgtgtaat gtgaggaaac gaggaagggg gttaggggat aaggagatta ttatgtatgg 2820
gatataagaa tttagagatg atttttaatt tattgtttcg tttttttttt tgaagttggg 2880
atgaagagtt agtatttgta aatttggagt tgtcgcgttt atgtagggta aggttaggga 2940
ggggttaggt ttttgcgaaa tgatttgttg gttttgattt tttggtgttg ggtttaaata 3000
tagagtttat agggagggga atttggaggg tttatgattt tcgggattta atttagtagt 3060
atttgttaaa gtatttagga aagatgtagt ttttacgtat atttagtttt ttttcggagt 3120
tgcggagcgt aaaagattat atagagagga atcgaggtag ttaggcgttc gtcgggggtg 3180
tgcgtttttg tattttttgt cgtttgggcg tttagtttgt gcgttttgtg gaggttaagg 3240
gattttcggt tttggatttg ggttttcgtt tagttaagag agaggggttt gggttcgcgg 3300
gtggagtatt ttgagtagta gttggtttgt agtcgcgtcg tttattatcg gaaaggaagg 3360
aagattagga attcgtggcg gtcgtgaatt gttttattgt tttttttttg gggcggaggg 3420
atttattgtt cgttcgagga gcgtgttttg gggaatagag tatgcgtttg tgtgtatgtg 3480
tgggaggcga attgttcgta gggcgtgggg gagggggtgt cgtttagatc ggaaggacgg 3540
gttttttttt tttttagttt attttcgggt ttggattggt tagagcgtta gttttcgtcg 3600
gtagcgagcg cggtgagagg ttggacgagg cgaagtttag agacgttaaa aatttgaaag 3660
atgttgttat cgttattttt tttttttacg ttggtttttg gtaggttcgg tttttttttag 3720
gttgatttag atatattttc gtttgggttc ggaaagggag gggagggaaa taagtatcgc 3780
gttatttacg attcgggaag agtagggttt ttcgttaggg tcgtttcgta tcgttgggtc 3840
gcgatttagg gaagttttgt ttggtttgcg tcggtcgggc gggaggtgta gggagggcgc 3900
gtggggcggt aggtagcggg gcgggggagc gtttttgttc gtcgtgtgtg tacgtcggtt 3960
atggggaggg tatgataggt attaaagagt aatacgtttt ttatgtattt atatgtttat 4020
gtttataaat tttttttttt cgtgaagtag gggaataaac gaaagtttat ttttattttt 4080
ttttgtttcg tatttttttg aagtaaattt agatatttag gaaagaaaag gtggttggtt 4140
tcgaggaatt agaaatattg ttaaaatttt atttttatcg tgaataaagt aggagtgtgg 4200
atgtgtatgt tgggaaaggt agatagttgt atgatttttt tatggagatt atgatttgaa 4260
attatttaaa aaattttaat ttaagtttta tttattttag tatatcgtgt ttttatagtt 4320
aaggggtgtt agaagtatat ggaatgtgat tattagttta taggtttttg tttttcgttt 4380
agggaattag tgagtggtta ggagatttgt tttttagttg atggtgaggt taatggaatg 4440
atttaaattt gaaatagatt ttttggaaaa gaaatggatt atttttaaaa gttaattaaa 4500
tttacgttag aggattttat tttggggtag gaggaggagg agtgttatat gttttataga 4560
gaagaaattg gttttggtga aaggtaaaag gttgaaaatg ttcgagtgaa gttaaattta 4620
gatgtgaaaa aggggtttat ttaaatattt ttttttgatt tgtttagagg tcgtttggat 4680
ttaatgattt gtttttttta gatttgtgat tttatttttt ttttattttt tatttttttt 4740
atttatgaat tattatttag ttttagtata atttattttt ttgttaagaa ggtttttatt 4800
ttttttttgt ttgtatttat tttttcgatg gattttttcg ttttgaagat aagatgttcg 4860
ttagtagata tggttaggaa atgtttaaga aaattaggag gaaagaaggt tatagtaaat 4920
tttatttttt ttataattaa aattattatt ttaaagaaga gttaataaat aatgaagtaa 4980
attgtatatg attttagttt agttatagta gatgatgagg taaggaaaga aagggttaga 5040
gtgtttttag atttattaat ttaaatattt gatttttgat ttttttaatg agaaatttta 5100
gaattgattg taaaatttat ttatagtata tagaatttaa tttttttttt ggagtttaat 5160
gttagtttgt ttttaatttt agtatagtat tgtagttaaa tgagttagtt tattttttta 5220
gttagattta ttgtttgttt aattttttag ggtagtgtaa gagaatttat ttataattga 5280
gttattttta ttttttaaaa atggagagtt attttttaat taaatagtta tttgtttttt 5340
agtttgaatt gatttttttt ttaggtaatg atttttaaga tagttattta ttaattaaag 5400
gataagttat agtattaatc gtttaaattt tagtgtgttt ttaaattaat gtgtatcgaa 5460
gttttttgtt tttagttatg agagagtagt aagtatttga gtagtttttt tttttaggta 5520
ttggatagta ggttttatac ggttgtgatt tttgagagaa ggaaaatata ttagttttgt 5580
tttataatta ttttataatt gtttaggttt ttttgttgaa gagaaaaata tttttttaga 5640
atgtaggaag gtagaatata ggtaaggtag tagttttagt taaggaggga gagattggag 5700

ttttgggttg ttgaagtagt tgaaattttt gaagataagt agttgtggaa tagggatttt 5760
ttcgtaagtt tatatggaga tttattatta gtttgggtta atagttaagt tgaatataga 5820
tatgataaaa ttttgtaggg tttaatagag attatttgtt gtatgtagtg tattattgag 5880
agatattgga gttttattat agtagggtag agagatttta ttgagtatgt tagggattta 5940
attgagatgt tggaaaggtt ttgttttaaa agtaatagtt aggttaaaaa gtatgtttta 6000
gagtaaaaat taaaatgttg tttttttata gtatatattg taataaagaa tggtaagaaa 6060
ttagatagga ttagggaaat ttataagaat gaaataatat ttattaaaaa aaaaataata 6120
tgatatagat tttttataat ggataattaa taataaaaaa tttaataggt tggatacgat 6180
ggtgtacgtt tatagtttta gttatttggg aagttaataa aggaggatcg tttgagttta 6240
ggagtttaaa attagtttag ggaaatatag taagattttg tttttaaata aattaattaa 6300
taaatttaat aaataaatat ggataagtga aaaatatata aaagtgattt atatttaaga 6360
gaaagtagtt aatagaaatt gattttgaga tggtttagat ataggaggaa ttggtaggaa 6420
agaaattttt aaagtaatta ttataaatat gtttaggtat ttaaaggaaa atagaatgaa 6480
taagtggata atgaataaat agagaatttt agtagaaaaa tggaagttaa aagaaagaaa 6540
tgaaaattta aaatttcgtt agtatagtta tttaatttgt aaaatttgta ttttttgaaa 6600
tggaatttta gaaagaaagt agaaagaaaa tggggtaaaa aatattttgg aaataatgtt 6660
ttaaatttgg tttagatgat ggaaaatata aatttattaa tttaagaaag gtggaaaatt 6720
tttaaaagaa taattattta gtatattata ttggtatata ttaaattatt taaagttatg 6780
gatgaaaaat ttttaaagta gttaaaggaa aaagatatat taaatgtagg agaataagaa 6840
tatgaattat atttggtttt ttattagtaa taatggaagt tagaagataa tggaatgata 6900
tttataatac gttaaaagaa aaaaaaagtt tatatagaaa tttataatta gtgaaaatat 6960
tttttgaaaa tgaagtggaa tttttttttt gttttttatt ttttatttaa ttttaggaat 7020
aagaattata ttgtatgaat aagagtaaaa tgaaaataga gtaatttgaa taaagtttaa 7080
agttaatttt agataggatt acgatttttt agtggtattt ttttttgtta tttaaaagaa 7140
aattttattt ttttttttgt tagagtttta ttttgttgtt taggttggag tgtagtggta 7200
tgattttagt ttattataat ttttgttttt ttggtttaag taattttttt tgttttagtt 7260
ttttgagtag ttgggatgat aggtgtttat tattacgttt ggttaatttt tgtatttttt 7320
agtagagacg tggtttcgtt atgttggtta gtttggtttt taatttttga ttttaggtga 7380
tttgtttatt ttggtttttt aaagtggtgg gattaacggc gtgagttatt atatttggtt 7440
aattttattt tttttaaagg aatataatat tatttaggat ttttataaat ttttagt 7497

<210> 269
<211> 6800
<212> DNA
<213> Artificial Sequence

<220>
<223> chemically treated genomic DNA (Homo sapiens)

<400> 269

gcgcgtattt cggcgcgggg agcggggtcg cgttattagt attattagtc gttattgggt 60
gagtttaggt attttgtagt agtggtgggg gcggggagtt gtggtggtag tggttgtaat 120
ttcgtatttc gggatagcgt ttttcggttt tttgagcgga gttcgttttg ttttttagaa 180
aatatttggg ttttcgtttt gggagagtag gcgggggtta gtttgcgaat ttttgtttgt 240
tttaattgtt agttgtatgt taaagtcgaa ttatatattt cgagggttat tgagagttta 300
tttttacgaa aagttaacgt ttttttttgt aatataagtt tttatttttt aaataatttt 360
ttttatttgt tttttaagtt tcgattttta tttttatttt tttggttttt tttattttaa 420
gttttttaga aaataaggtt agaattgaaa ttaagatgtt ttttggaagt aaataatttt 480
tttttttttt aaaaagttaa aaaaaataat aataaaaaat tttaagtttg gagattttac 540
ggttttgttt gattttttaa tttttgtttg gtatcgaggt tttttttttt ttttcgtttt 600
tttttttttt tttttttttt taaatgggta attcgttgtt tggaagtaat ttgggttttg 660
cggtgaggtc gtattttttt agtttttgtt ttttttttag ttattttcgt atatttattg 720
tcgaattagt tagatttagt ttttagatag attttgttag gggagtttcg agggggtaat 780
gcggagagtg cgtagcgaat cgcgggtcga taaatgtgtg tgtgttcgag tgtgtggatt 840
tttggttgcg tgtgtgtgcg tgtgtgtttg tgtgtgtagg ggagttgttt tcgggtttag 900
gacgtagtag ttttcgtttt ttttattttt cgattttttt aacggaagat tttttttagg 960
aggttcggtt taagagtttt ttgttttttt tttttttttt tcgtgcgttt cggtgtttaa 1020
ggggttagta ttatgcgcgt agaattcgga gttattttcg cggttttttt tttttcgcgg 1080
tgcggtgtcg gttgtttttt gttggtcgtt ttgaggtagg atttttgcga gtttcggttt 1140
tttggggcgg gcgggcgggt agttgttttt tttttttttt tatttttgtt ttttttattt 1200
ttttcgatta ttagatttta tttttttagt aggggttttc ggaggttttt agaggattag 1260
tttcgttgcg tcgtcggtcg ttgtaatacg gaaggggtta cgggtttagt ttagtaaatg 1320
ggattcggtt agttggatat agggttggta tttttttttt tgggtttggg aaggagagtg 1380
tcgttttagg aggtttcggg tttggtttta gttgtttttt aattttttat gatcgagaat 1440
aagtgttgtt ttttcgtttt tcgttttatt ttaatcgttt attaaaaagt gggagagaaa 1500

ttatttttaa agagtggggg ttggattatt attttatttt ttttttttgt ttaaggattt 1560
tgttttattt ttggataaag ttgatttttt gtcgagaggg ttagatattt tatgtgagaa 1620
ggtgtgagtg gtgtgtgtgt gtgtgtgtgt gtgtgtgtgt gtgtgtgtgt gtgtgtgtat 1680
tttggggttt ggaaagttta gtttaaggga tggacgaggg ggtaggaaat agaggagaat 1740
taggttagat cgtttttttt ttggggtttt tttatattcg aatttggaat ttgttgttag 1800
tgttgaggtt tgggcggttt ttttttgtta tattgatttt ggtttaaggt gtaattttcg 1860
ttttttttat ttggtaattt tttttttttt tatttcgttt cgcgcgcgtt aatatatata 1920
ttgttgtgga tataattttt gcgtttaggc gtgaggttta gggagtagtg gtttttttgt 1980
ttgttttttt aaagcggatt ttttattcgt ttgggtcggt ttatttttta gtttttcgcg 2040
tcgtttttcg tattttttag ggtaggacgt tttaagtttt tggttgtaat tgagtgtttt 2100
tttgttagtt tttgtatttt tttttttgtt ttagggagag attagagtgg ggaaaatagt 2160
tttttttttt tattaagagt ggagattttt tttggagttt tttatttttt ttttcgttgt 2220
tataagtttg ggttaaatgg gtttttttcg tttatttggc gttttttatc gttttatttt 2280
tagaaatttg gggtttaaac gtagagagtt tagtagaagg ggatgtttag attgaatgtg 2340
gtagtgtttc ggggtcgagg ggagaggatt tttgtttggt ttttttattt atttttttaa 2400
gggattgttt gcggaggagg agttgggtgt tgcgagattt taggtgtgta ttcggttgtt 2460
tttcgcgttt ttcgaacgtt ttttgaataa ttttgttatt gaggtttttt tttttttatt 2520
ttttattacg tttggttcgt atcgtggttg gtaaagaagg attaagaata tttttaattc 2580
gacgcgaagg ttttgaggga tatatagaaa gggagttacg tcgttgagtt gttttggatt 2640
gaagaggagg acgcgggaac gttttttttt attttatttt tgtggtgcgt tgagtttttt 2700
aaacgttttt agtttttttt ttcgtggtac gcgaggaaga tttttagtgt ttttatagag 2760
ttattttttt tgagtttcga aagtgttgga aaatatttag tttttttttt gggaaattta 2820
gtttttgatt agaatttttg ttttattttt ggggttgata gtttttttag aggttttatt 2880
ttatattgtt ttttcggatt tgattttttg gtaaataggc ggggatgttt tattttatag 2940
agtcgatgta tgtgtgagtt cgttgtgagt tttttgagtg ttttaaattt gtggggtttt 3000
ttttcggttg tattgggatt gaagagggaa gagggttaag gtgttttcgg gtaagcggcg 3060
gggttaagtg gagatgcgat tcgtgaggtt ttttttttcg attttttttt gggatatttt 3120
ttgtttattt ttattttgga gttagggaga tttaagtttt ggtgatcgga tgggttcgtt 3180
tttagttggt ttgggttttg ggaattggtg gatttttttt gggggtttcg ggttgggagt 3240
gggttcggtt tgtgtggttt cggttttaat aaagagattc gttgtaattc gtcgaatttg 3300
ttattaattt ttttgttgtt tgagtttcgt tttacgcgtt tcgttcgtcg cgaagtttgg 3360
aaagtgtacg cggttagtat taatttgggt cgttgattcg gaaatatgtc gtagcgtgtg 3420
tgtttatgta cgcgtgtgag tgtgtaaatg tgtacgagtg tgaatgtgta tgatgtgtgt 3480
gtacgcggta tcggttgttt ttggggagag ttgattttgt agtttgggtt gcgcgagaag 3540
tagattttgt agtttatttt tttttttgga ggaaatttga tatttagggc gggggtgggg 3600
agatagtcgg agtttttttt tttttagttg gggaaatttt agatttttat tttttaggat 3660
gcgtttttta gttttgtagc gttttgggga taattgggtt ttgggtgtgg agttttgttt 3720
agtaggcgtt ggggattata taagtatttt ttttttgaga agtttcgaag cgtttaggtt 3780
aaaggggcgg tttacggaga aaaattttgg tacgtttttg agttttatag ttttattagg 3840
gttgtttagg ttttttttgt tttttttacg gtataggttt taagttaggt ttttttatcg 3900
ttttaaagag gtttattttt tttttttttt ttgtggaagg ggttttttta ggggttatgg 3960
gcgatgtagt gcggtagggt tagatttacg tgtaagggga tttttaaaat tcgttttttt 4020
tattcgtatt cgttatttat tcgtttcgtc gtcgtttata ggtggagaag ttattagtgg 4080
ggaggaacgg tagcggaagt ttttaaggtt aatttttatt tttgaaattt tttaggaagg 4140
gaattttcgt cgttgggggt ttttttgttt tggaatcgat gcgtttagtt gcggttcgga 4200
agttagcgtt tttggtttcg tttggattta tttgtaaggg ttttggtttc gtttcgtatt 4260
tttatttttc ggtattgatc gaattaagtt tgagttgggt tggagaggtt agattggagg 4320
tagggtggta gagttttaac gataggttcg tagtgtcgcg atggtaaagt ggtttataat 4380
tttagattag gattaaggga aattggagtt tttttgggtt ttttattttt tttttttttg 4440
gtagttgtta ggtgtggggt gggagggaga gtgaaaaatt agaatttggg agaaagttgt 4500
ggggagggta gggaagggat ttttttttcg gggaagcgag atttagattt tttttttttt 4560
tttagggttt tatttttttt tttagttcgt ggaagaggtt ataggcgacg cgggcgaggg 4620
tggtattttt ttttagtttt tttggttggg agattcgatt ttttttttta ttatttttta 4680
gggtttttat tttttttttt tttttttagt ttggttgaag aacgttttta aggaaattcg 4740
ggttgttttt ttttatttgg aagtggtttt ttttatatcg gttcgtaaat tgattatgaa 4800
atatacgatg ttaattcgga gttgtatttt ttagttgggt attttcgcgc gttggttttc 4860
ggggtttcgt tttttatttt ttgttttttt ttttcgcgtt ttgtttttat tttttatttt 4920
tcgcgttggt tatttcgttt ttttggtagt ttttggcggt agcgcgtttt attcgttttc 4980
gtgttttttt cgtttatgga attaattttg gttttatttg ttgttcggtt taggttgggg 5040
agaggacgga gggtggtcgt agcgggtttt tgagtgaatt atttaggagg gattgagtat 5100
agtattaatt agaggggggt tagggggtgc gggattcgag cgagtaggaa ggaggtagcg 5160
tttggtatta gggttttgat ttaatagaat tgagatacgt ttgtaatcgt tggcgtgttt 5220
cgcgtatagg attttagcga aattagattt tttggtgagg ttgcgtgggt ggattaattt 5280
ggaaaaagaa attgtttata ttttgttatt aaaaaattta cggaggagaa gcgtagttaa 5340
ttaatagtaa atttaagaga tttcggatgt tttcgttgtt taaatttgta tgtttgaaaa 5400
ttatttgaga ggtaataaat atttgttttt tttttttttt tttagaagtt tattggaata 5460

ttaagtttag gagttgtttt ggggatggtt ggaagtgtaa tgttttttaa gtttttttta 5520
gtggttttgg ttatattttt ttttttcgtt taggttgtaa ttgaagttaa tttttggtgg 5580
taagtttatt ttatgtgcgt gttttttttt tttttttttt ttttttaaga tttgtttgta 5640
tgatgcggta atatttaatg ttggatagtg agtgtgggtg tatttattgt aagatatatg 5700
tatatatttt atttatatat agttttaaat tagaattttt ttttaatttg ttaatattgt 5760
aaagtttatt tttagttttt agggaatggg aaattataaa tttaggtttg tttttatgaa 5820
gtttttgttt agacgtgtgt ttgtagtata ttatgtaata aaaaagaata gaaatttatt 5880
ttgttagtaa ttgtaagttt atttataaaa tttatttttt gttattttta ggtagtaaaa 5940
gtgagtttta gtttttattt tttgttattc gagattatta ttaatttata gagtttgaat 6000
taggtagagt tttaggagga tagttatatt ttaagtgtag aagtattggg gtttgaaatg 6060
taattaaaag gaggttaatt tgaagtagtt aaatgttttt aagtgggggt ttttttttgg 6120
gtaggggttt tgtagataga ttgtaatata tttagaggat tatttttgac ggtattttgg 6180
gtattttttt ttttgtaaat gaatagtatt tttttgattg ttaaatatga tttttgttta 6240
ttgggtattt tgttttgggt tttttataat tatataagga gtttttttaa aatgttaaga 6300
aatatggtga atttttttta gtttgtatat ttgttttttt gtttttttat tagttaaaag 6360
gaattgttat tttttattcg tatttaattg tgtttatttt tagtatttat aagtttttaa 6420
ggtagattgg tttaatttgt ggggtaaagt gggttatttt ttttaatgtt agaggtttgt 6480
tttttaaatt gttgtttttt ttttttttga gtggattttg gaaatattgg tttaggtata 6540
tggtgatagg ggttaggaag ggagtgaaaa tattaattga ggagttagat ggtaggatgg 6600
ggattgtata atattttcgt tttttgagtg tagtttaaat agaggagttt tatgaagttt 6660
tttttatatt ttgttatgag gataagtagg agagaaaaaa gtatttgtgt gtttaaaatt 6720
atgtattgtt gatatgtttt tagggggttt atttcgagat tttacgtaaa ttttgaatgt 6780
atattttttt tttttgtagg 6800

<210> 270
<211> 6800
<212> DNA
<213> Artificial Sequence

<220>
<223> chemically treated genomic DNA (Homo sapiens)

<400> 270

tttgtaaggg ggggagatgt gtatttaaga tttacgtgaa atttcgaggt gggttttttg 60
aaagtatgtt agtaatatat agttttaagt atatagatgt tttttttttt tttgtttgtt 120
tttatgataa agtatgagaa gggttttata aaattttttt gtttaaattg tatttagaaa 180
acggaggtat tgtgtagttt ttattttgtt atttggtttt ttagttagta tttttatttt 240
ttttttagtt tttgttatta tatatttgaa ttagtatttt taaaatttat ttagaaaagg 300
aggaaatagt agtttgaggg ataggttttt gatattagag aaagtgattt attttgtttt 360
ataaattgga ttaatttgtt ttgaaaattt atgaatgttg aaagtagata taattagata 420
cgggtaggag atagtagttt tttttgattg atggaagaat agagggataa gtatatagat 480
tggagaaaat ttattatgtt ttttgatatt ttaaaagggt tttttatgtg attgtgaggg 540
atttaagata gggtatttag tgaatagaag ttatgtttga tagttagaaa aatgttgttt 600
atttataggg aaaaaaatat ttagaatgtc gttaaaaata attttttgga tatattatag 660
tttatttata aggtttttat ttagaaagaa atttttattt ggggatattt gattatttta 720
agttaatttt tttttgatta tattttaagt tttaatattt ttgtatttgg agtgtagttg 780
tttttttaga atttgtttta atttaaattt tgtaggttaa taataatttc gagtaataga 840
agataaaggt tgaaatttat ttttattatt tagaggtggt aaaaagtaaa ttttgtagat 900
aggtttataa ttattagtaa agtgaatttt tgtttttttt tattgtatga tgtattataa 960
gtatacgttt gaataagggt tttatagaaa taggtttaga tttatagttt tttatttttt 1020
aggagttgaa gataagtttt ataatattaa taagttaaaa gaaaattttg atttaaaatt 1080
atatataagt aaaatgtata tatatgtttt gtaataaata tatttatatt tattatttag 1140
tattaaatat tgtcgtatta tataaataaa ttttaaaaaa agaaaaagaa gaggaagaat 1200
acgtatatag aatgaattta ttattaagaa ttggttttaa ttataatttg ggcgaaggag 1260
aaaaatatgg ttaaagttat taggaagaat ttggaagata ttgtattttt agttattttt 1320
aaagtaattt ttgggtttaa tattttaatg gatttttgga gagggaagaa aggagtagat 1380
gtttattgtt ttttagataa tttttaagta tataagttta aataacggaa gtattcgggg 1440
ttttttaagt ttattgttga ttgattgcgt ttttttttcg tgagtttttt gatggtaaga 1500
tataggtagt tttttttttt aaattaattt atttacgtaa ttttattagg aaatttgatt 1560
tcgttgggat tttgtgcgcg gggtacgtta gcgattataa acgtgtttta attttgttga 1620
gttaaagttt tggtgttagg cgttgttttt tttttgttcg ttcgagtttc gtattttttg 1680
gttttttttt agttggtgtt gtgtttagtt ttttttgggt aatttattta ggaattcgtt 1740
gcggttattt ttcgtttttt ttttaatttg ggtcgagtaa taagtggagt tagaattaat 1800
tttatgggcg agaggagtac ggaggcgagt ggagcgcgtt gtcgttagag gttgttaagg 1860
aggcggggtg gttagcgcgg ggggtggagg atgggggtag gacgcgggag ggaagggtag 1920

ggggtggggg gcgaggtttc ggggattagc gcgcgagagt gtttagttgg gaaatgtagt 1980
ttcgaattaa tatcgtatgt tttataatta gtttacgagt cgatgtgggg aaagttattt 2040
ttagatgggg aagagtagtt cggatttttt taaggacgtt ttttagttag attagggagg 2100
ggagaaggag atgggggttt taggggataa tggagagaga ggtcgggttt tttaattaag 2160
gaaattggaa aagagtgtta ttttcgttcg cgtcgtttgt ggtttttttt acggattgag 2220
agaagggatg gaattttaga ggaaagaagg gagtttgggt ttcgtttttt cggggagaag 2280
attttttttt tgtttttttt atagtttttt tttaaatttt gattttttat tttttttttt 2340
atttatattt tggtagttgt tagggaggga ggagatggga ggtttagaga gatttagttt 2400
ttttttggtt ttgatttggg gttgtgggtt attttgttat cgcggtattg cgagtttgtc 2460
gttggaattt tgttattttg tttttagttt agttttttta gtttaattta gatttggttc 2520
ggttagtgtc gaaaggtggg ggtgcggggc gaggttagag tttttgtaga tgagtttaga 2580
cggggttaga ggcgttggtt ttcgagtcgt agttgggcgt atcgatttta gggtaaagag 2640
gtttttagcg gcgaaggttt tttttttgaa gaattttagg ggtaggagtt ggttttggaa 2700
gttttcgttg tcgttttttt ttattggtga tttttttatt tgtaggcggc ggcggagcga 2760
gtaggtggcg ggtgcgggtg ggaggagcgg gttttaaaaa tttttttata cgtaagttta 2820
attttgtcgt attgtatcgt ttatagtttt tgaaggagtt ttttttatag aaaagaaaag 2880
aaaggtgagt ttttttaagg cggtggaaga gtttggtttg gaatttgtgt cgtaaaaggg 2940
gtagagggga tttaggtagt tttggtaaag ttatggggtt taggggcgtg ttaaggtttt 3000
ttttcgtgag tcgttttttt ggtttggacg tttcggggtt ttttaaagag gaaatgttta 3060
tgtggttttt agcgtttgtt aagtagggtt ttatatttaa ggtttagttg tttttaaaac 3120
gttgtaaagt tggggggcgt attttggaga atggaaattt ggggtttttt tagttaggag 3180
agagaaggtt tcggttattt ttttattttc gttttaagtg ttaaattttt tttaggggag 3240
ggagtgggtt gtaaagtttg tttttcgcgt agtttaggtt gtaaagttaa ttttttttaa 3300
gggtagtcga tgtcgcgtgt atatatatta tatatattta tattcgtgta tatttatata 3360
tttatacgcg tgtatagata tatacgttgc gatatgtttt cgagttagcg gtttagattg 3420
gtgttggtcg cgtgtatttt ttaagtttcg cggcgagcgg ggcgcgtggg gcggggttta 3480
agtagtagag aaattgataa tagattcggc ggattatagc ggattttttt gttagagtcg 3540
aagttatata aatcgaattt atttttagtt cgaagttttt agggagagtt tattagtttt 3600
tagagtttag gttaattgag agcgggttta ttcggttatt aagatttggg tttttttggt 3660
tttagggtag aggtaggtag agggtgtttt aaagggggat cggaaaggag agttttacga 3720
gtcgtatttt tatttaattt cgtcgtttgt tcggaaatat tttggttttt tttttttttt 3780
aattttagtg taatcgagaa aaggttttat aagtttgaga tatttaaaga atttatagcg 3840
aatttatata tatatcggtt ttgtagggta aaatattttc gtttgtttat taagagatta 3900
gattcgaaaa gatagtatga aatgagattt ttggggagat tgttaatttt aggggtaaaa 3960
taaaaatttt gattagaaat tgagtttttt aaagaagggg ttaaatgttt tttaatattt 4020
tcggggttta gggaagatga ttttgtaagg atattgagaa tttttttcgc gtgttacggg 4080
gaggaggatt gggggcgttt gaggggttta gcgtattata ggagtgaggt ggaggagggc 4140
gttttcgcgt tttttttttt aatttagagt agtttaacga cgtggttttt tttttatgta 4200
ttttttaaag ttttcgcgtc ggattaaagg tgtttttgat ttttttttat taattacggt 4260
gcgggttagg cgtgatgagg gatgagggag aggaaatttt agtagtaaaa ttgtttagag 4320
gacgttcgga gggcgcgggg agtagtcgga tgtatattta aagtttcgta atatttaatt 4380
tttttttcgt aggtagtttt ttaagagaat agataaaaag gttaagtaaa gatttttttt 4440
tttcggtttc ggggtattgt tatatttagt ttaggtattt ttttttgtta gattttttac 4500
gtttaggttt taagtttttg ggagtgaaac gatgagggac gttaaatagg cggaagaagt 4560
ttatttgatt taggtttatg gtagcgaagg ggaggatggg aaattttaaa aagaattttt 4620
atttttggtg gaaaaagaaa gttgtttttt ttattttgat ttttttttaa agtaaggaga 4680
gagatgtaga aattagtagg agagtattta gttgtagtta aaaatttggg acgttttgtt 4740
ttggagaatg cggggagcgg cgcgaggagt taaagaatgg gtcggtttag gcgggtagga 4800
ggttcgtttt aggggagtag ataaaggagt tattattttt taagttttac gtttgggcgt 4860
aagggttgtg tttatagtaa tatgtgtgtt ggcgcgcgcg gagcggggta ggggaggagg 4920
gaattgttag atagagaagg cggaggttat attttgagtt agaattagta tgataaaaga 4980
gagtcgttta ggttttaata ttgatagtag gttttaggtt cgggtataga gaggttttag 5040
gggaagaacg gtttggtttg gttttttttt gttttttgtt ttttcgttta ttttttagat 5100
tgggtttttt aggttttagg atatatatat atatatatat atatatatat atatatatat 5160
atatatatat tatttatatt tttttatata ggatgtttag ttttttcggt agaggattaa 5220
ttttgtttag aggtggagta gagtttttgg atagaggaag aaaatgaaat gatagtttaa 5280
tttttatttt ttaaaggtag tttttttttt attttttaat agacgattaa agtgaggcga 5340
aaagcgggaa agtaatattt gttttcgatt atagagagtt gggaggtagt tgaggttaga 5400
ttcgaggttt tttagggcga tatttttttt tttaagttta gaagaagagg tattaatttt 5460
gtgtttagtt ggtcgagttt tatttgttgg gttggattcg tggttttttt cgtattgtag 5520
cggtcggcgg cgtagcgaaa ttggtttttt gggggttttc gagggttttt gttgggagag 5580
tggggtttgg tggtcgggag ggatgaaggg agtaggggtg ggagggagag gggagtagtt 5640
gttcgttcgt tcgttttaag gaatcgaggt tcgtaggagt tttattttaa ggcggttaat 5700
agggggtagt cggtatcgta tcgcggggaa gaaaaggtcg cggggatggt ttcgagtttt 5760
gcgcgtatag tgttaatttt ttgagtatcg gagcgtacgg gggaggggag gagaagatag 5820
gggatttttg ggtcgggttt tttggggagg gtttttcgtt gagagggtcg gggggtggag 5880

gagacgaagg ttattacgtt ttggattcga gaataatttt tttatatata taaatatata 5940
cgtatatata cgtagttaag gatttatata ttcggatata tatatatttg tcggttcgcg 6000
gttcgttgcg tatttttcgt attgtttttt cgaaattttt ttggtagagt ttgtttagaa 6060
attaaatttg gttggttcgg tagtgaatgt gcgaaggtgg ttggggggag ggtaggggtt 6120
gaggaggtgc gattttatcg taaggtttaa gttattttta aatagcgaat tatttattta 6180
aagagagaag agagaaaaga gaggcgagag aggagaaggg gtttcgatat tagataggga 6240
ttagaaagtt aaatagggtc gtggggtttt tagatttgaa gttttttgtt gttgtttttt 6300
ttaatttttt ggggggaaga ggggttattt gtttttagga aatattttaa ttttaatttt 6360
aattttattt tttaagaaat ttaaaatggg aaagattaga aaaatggaaa taggaatcga 6420
agtttggagg atagataaga aaaattgttt agaaaataaa aatttatgtt gtaggaagaa 6480
acgttaattt ttcgtaaaag tgaattttta gtgattttcg gagtgtgtga ttcggtttta 6540
atatataatt gataattaaa ataaataaga gttcgtaaat tggttttcgt ttgttttttt 6600
aaagcggagg tttaagtgtt ttttaaagga taaggcggat ttcgtttagg gggtcgggga 6660
gcgttgtttc ggagtacggg attatagtta ttattattat aatttttcgt ttttattatt 6720
gttgtaaagt gtttgagttt atttagtggc ggttaataat gttaataacg cgatttcgtt 6780
tttcgcgtcg ggtgcgcgt 6800

<210> 271
<211> 6089
<212> DNA
<213> Artificial Sequence

<220>
<223> chemically treated genomic DNA (Homo sapiens)

<400> 271

aaggtttttt agatgagttg atatttgggt gggatttgga ggataagtta gaagtttatt 60
aggtagaaaa gagttaaagg tattttagat atatgtaaag gaaaataggt tgtagtagta 120
gttttttaat tttttaaaaa attttaattt aaaatgtata tatatatata tttaatatat 180
atatatatat atatatatat atatatatat acgtatatat atagttggaa ttttatgtat 240
aatgtttttt gttatttttt gatttatttt tttttgtaaa tgttagtggt aatttattaa 300
tttgttgtga tatgtgtata agtgtttggg gaaagtggag gttgtaaagg taattttgag 360
ttagattata aagatttttg gatgttaggt tggtaagatg gaattttttt tagttttgtt 420
tttttttttt tttttgtgaa gttaaatatt ataaattttt tttttttttt ttattgtttg 480
atgaattttt tgttttttag gtttgttttt gttattttta gtttttttat ttttttagtt 540
ggttttttaa attatgttga atttttgggg ttttgtattg ggtttttttt atttattttt 600
tttggttttt tagggtgttt tttttttgtt ttgatttgaa tgtttgttta tttttagttg 660
gggatattta atttagaagt gatagtttta agtttttttt cgagtattag ttatttttta 720
gtgatttata gatattttag atttagtatg tttaaaatta agttttattt tttgagattt 780
tttgttttta gggtttttag tttgtatttt ttttattagt tatattttta taggatagga 840
tattgttttg gaggtttttt ttattttgtt tattttgttt ttaaattcgg ttttaaattt 900
attttttttt ttttttatta tttgtttagg gtaagttttt ttttttatag gtaattgttg 960
agattttttg atttgttttt tttttttgtt tttttttttg ttggttttta tgatagttat 1020
tatgaatttt ttgaaataaa aatttgatta tgtaatattg ttattttttt taatatgttt 1080
tgtgttttta gattttttat tgagtttttt ttttgtatat tttttttttt ttaacgtttt 1140
aatatttagg tattttgaat tttcgtggtt ttgttttttt tggttttaaa aatttatttg 1200
tttaatatta atttattttt ttaggatttt attttaggaa tatttttttt aggaagtttt 1260
ttttgatttt ttgagattgg ttgaggagtt tttttgtgtt ttaatagttt attgtgtatt 1320
atatattttg gtgtttaata tttttttttt tattggagtg ttttaagttt tttttttttt 1380
tttattagat gggtagttta atggggttgg ggggtgggtt aagagtagat gttgaagtta 1440
gtatttttta ggttcgaagt ttagtttagt tatggattgg ttgtgggaat ttgatatttt 1500
atttaatttt tttttgattt atttttttta ttttattaaa taatgatggt tgtgataatg 1560
atattaataa gtggagtatt tattttttgg tatttttagg ggatttagtg agttgatatg 1620
ttgaggttgt aaatgatagt gtttggtatg taatagtgtt atttatgtgt ttgattatga 1680
aatcggtttt tagatgggtt gttttaggag gggtagagat tggatttatt tgatatattg 1740
atttgttttg gttatatttt tttttgttgt gttttttttt tttttttttt tttttttttt 1800
ttatttttcg atgtatttag tatttgtatg ggttattggt atgtgtttag tatttggttt 1860
gatattcggt tataagtata ttttagtatt tatgatatga gtgaaatttt ggtttaggaa 1920
tgatggtgaa tgtagaaaat aaaggttata tttggagatt ttttggaggt agattgttta 1980
gggtttgtgt tgattttatt tgagggtgga agaggaggaa gaggaggagg aataatgtag 2040
gagggaagaa tatggttagg atagattttg tgattttttt tattaaattt ttagtttttt 2100
taattatttt tagattatta taagttattt ttatttattt aaatatttta gttaatatcg 2160
ggttatttta ttattttttt tttttttttt ttatttattt ttatcgaatt tttttaagtt 2220
ttgtttgtgt aagatatagg tttcgtattt tgggtttttt tttttttttt gtattttatt 2280
tttattttta ttgttttagt tataattgtt gtgtaataag tatttttttt aaatttagtg 2340

gtaaaataat tattatattt ataagttata gggtttagaa agttggtagg gtattattgg 2400
ggatatttat acggtttttt gtttattatg ttttagtttg gtagatataa agttagtatg 2460
atataatggt ggaggtattt ttatttatat ttttgggtat ttgggtaagg tgattttagt 2520
gttgaattta tttgggattg tgggttggag taagtatagg tggtttttgt ttatgtttgg 2580
gtttttttat agtatggtgg tttttaggtt ttttttatgg tggtttaggg ttttagtatt 2640
ggtgcgttag ttaataaaat aaagattgta ttgtttttta tcgcgaagat ttgtaatttt 2700
tgagtattat ttttgttata ttttattggt ggaagttatt ataagtttat ttagatttac 2760
gtaggtagag tatagagaat ttattttttt atgggaggag tgttaaataa ttttgtggtt 2820
atgttttaag agtattattt taatttaaat tattaatgtt ttatttggat ttttattgtt 2880
attttttttt tggtttgttt gatggcgtat tttatttatt tgtttttatt tttatagtgt 2940
gtttttttag gatagttacg ggagtttttt gaatatataa attagattag ttattttttt 3000
ttttattttt ttaatgtttt tagttgaata tattattgaa atttaagttt tttgacgtgt 3060
ttatatagtt ttgtggattt gtgtattttt attttttttt aagtttaggt tttttttttt 3120
tttttattgt agtttattat tttttttttg gggtttatgt ttatttttga gtttttgtat 3180
ttgttggttt tttttttgta gtgttggatt tttgatgttg tgttttttag gttttatttt 3240
aaatgttatt tttttagatt taaaattaat tttttaatat ttagtatttt tttttttatt 3300
attttgttta atggtttatt atttgtaatt ttgtttttgt ttatttatta tttgtttttt 3360
ttttggtttt aagaagaaag attgttgttt tttttttttt ttgtgtttac ggtgtttatt 3420
atcgtggttg gtatagaata tggaatatga atgaatattt gaatggtggt tttcgggtgt 3480
gtgtgtgtgt gtgtgtgtgt gtaatttaga aaggtaattt tgaaggatag ttaggattgg 3540
gtgtaggagg ttattatagt tgggtatatt ttgtttttta tatggttgtg ttattcgtta 3600
gttggtgatt ttgggtaaat atttttattt ttttgagttt tagtgttttt agttgaaatt 3660
tgagtttaga tttgttagat ttgggtatag ggatagtagg tttagtagga atattaagtt 3720
aggattatag ttatttttag ggagtttagt ttttggagtt cgagaggaat atttttcgga 3780
ggcgtaggcg tagtcgtttt tagagattgg gtaattttta ggtggtaggc gggatcggtt 3840
cgtgtaaata gggaataggt attcggagga tggatattcg ttggtaaaaa tataaattgt 3900
cgttcggagt tttggtatta ttttaatttt agggacggtt agattttttt tagtttattg 3960
gatgaggtgt atattaaaga agggtgttag ttttttcggg tcggttaggg tatttaaagt 4020
attttgtttt cgggagcgag gtcgaggtgt attattcgtc gcgttcgttt ggtgacgtcg 4080
cgagcgcgtt ttgagcggtt agttgcgttt tttacgtttt cgcggtttag gcgttttatt 4140
gttgtttcgc gcggggtaat tgggttttag ttcgatttag tttgtcggtt aatagtagga 4200
ttttagagga ggttgtttaa tttgggtatt ttttgtcgtt attttcgaat aggtgggagt 4260
agggaagaaa tatattgagt tagttagttc gtcgtttcgc gaaagggggc gttttttttt 4320
gaagttgggg ttggggaatt ggggtgtgtt ttgttagttt tagttttttg tttcgggttg 4380
gagggagtag ggtagtagat taaggtatta ggtttttcgg tcgagtttgg tttattttat 4440
tcgagggtag tttttggacg tttattcgtt ttgtaatttg taagttagtg acgggtgggg 4500
ggcgagcggg tttttatcgt ttttaatcgg gttgtgtttt ttagtttgtt atttgtattt 4560
cggagtaggg agggggtttt cgggagcgcg tatggaaagg cgcgagcgcg cgttttgtgt 4620
aggcggtttt gtttcgcggg cgtaggggcg ttgggcgggc gttttcggga ggcggtcggc 4680
ggagggggcg ggggagggac gggggaaagg agggaggggc gggggttgga ggtagtagcg 4740
ttttcgtatt tttcgcgttt cgtatatttg tatcggtcgt tcgcgcgtag ttcggcgtcg 4800
ttttacgtcg cgttcgtttt tttttttttt ttttcgtttc gtggttttcg tgtttttggc 4860
gaggtttagg cgcggagcgc gcggacgggc gtatcgatag acggtttcgg ggacgtttcg 4920
gttcgcgttt ttcgggcggg ttatgttgat tgtttcgtcg gggtcggttc gcgggattag 4980
tatagttcgg ttcgcggttt cggcggttaa tcgggattat gaatcggaaa gcgcggcgtt 5040
gtttgggtta tttttttttt agtttgggta tggtttattt tcggatcggg taagggttgt 5100
ttgcgttttt ttcgcgcgtg tttcgataaa gttggtacgg ttcggggagg gaggtgtgtc 5160
gtttgcgagt ttttgtcggt taatttttat agtttttttg agaggtcggg tgggtgcggt 5220
gtgggagcgg tggacgtata aaggtttttc gtaggatatt ttaggagata taggtagggg 5280
gattggttat tttgaggacg tttgagtttg gaatttttta ggattagaga tagtcggtag 5340
ggacgtttta gtttgaggga ttttggggga gggttagttg ttgtaaaggt ttagtttatt 5400
tcggggattt ttcggattcg ggtataggga tagtaggttt agtaggaata ttaagttagg 5460
attatagtta atttcgggga gtttagtttt cggagttcga gacgaatttt tttaggaggc 5520
gcggtatagt cgtttttaga gattgggtag tttttaggtg gttggcgggg tcggttcgtg 5580
taaataggga ataggtattt aggaggaagg atattcgttg gtaaaaatat aaatcgtcgt 5640
tcggagtttt ggtattattt aatttttagg ggtttttaga tttttagagt tttttttcgg 5700
gttaagggga tagtacgttt tatttttaat aaatatatat aaaggtggga cgagaggcgg 5760
tttgagatat agagggagat aagcgttaga gagaggcggg atatttcgtt ttttggaatt 5820
gggtagtaga ggcggtcggg cgcgcgagta gtttttcggt tttatttagc gtttttgtgg 5880
aggtggggtt cgggggcgtt ttggttggaa ggtgggaggg ggtagttcgg gacgcgtttt 5940
ggagttgttt gtgtaaagtc ggttattttt tgtatttaag ataacgcgtg tgaggttcgg 6000
agcggtgtgg gggcgggggg atttgggtat ggatagaggt tttggcgttg aagggttcgt 6060
ttggtaggtt ttatatattt tgtttttttt 6089

<210> 272
<211> 6089

<212> DNA
<213> Artificial Sequence

<220>
<223> chemically treated genomic DNA (Homo sapiens)

<400> 272
   ggaggggtag ggtgtgtgag atttattaag cgggtttttt agcgttagaa tttttattta 60
   tgtttagatt ttttcgtttt tatatcgttt cgagttttat acgcgttgtt ttaagtataa 120
   agggtgatcg gttttgtata agtagtttta ggacgcgttt cgagttgttt tttttatttt 180
   tttagttaga gcgttttcga gttttatttt tatagaaacg ttaggtgaag tcggagggtt 240
   gttcgcgcgt tcggtcgttt ttgttgttta gttttagggg gcggggtgtt tcgttttttt 300
   ttagcgtttg tttttttttg tgttttagat cgtttttcgt ttttattttg tgtgtgtttg 360
   ttgggggtgg gacgtgttgt ttttttggtt cgggaagggg ttttgggagt ttaagggttt 420
   ttggaaattg ggtggtgtta aggtttcgag cggcggttta tgtttttgtt agcgaatgtt 480
   tttttttttg ggtatttgtt ttttgtttgt acggatcggt ttcgttagtt atttggggat 540
   tgtttagttt ttaggaacgg ttgtgtcgcg ttttttgaga gagttcgttt cgggtttcgg 600
   ggattgagtt tttcgagatt ggttgtggtt ttggtttgat gtttttgtta gatttgttgt 660
   ttttatgttc gggttcgaag ggttttcggg atgggttgaa tttttgtagt agttagtttt 720
   tttttagagt tttttaggtt ggagcgtttt tatcggttgt ttttggtttt gggggatttt 780
   agatttagac gtttttagga tggttagttt ttttgtttat attttttggg gtgttttgcg 840
   gggggttttt gtgcgtttat cgtttttata tcgtatttat tcggtttttt agagaagttg 900
   tggaagttgg tcggtagagg ttcgtaggcg gtatattttt tttttcgggt cgtgttaatt 960
   ttgtcgaaat acgcgcggaa agggcgtagg tagtttttat tcgattcgga ggtagattat 1020
   gtttaggttg agaaagaggt ggtttaggta gcgtcgcgtt tttcggttta tagtttcgat 1080
   tggtcgtcgg ggtcgcgggt cgggttgtgt tgatttcgcg ggtcggtttc ggcggggtaa 1140
   ttaatatagt tcgttcggga ggcgcgagtc gaggcgtttt cggggtcgtt tgtcggtgcg 1200
   ttcgttcgcg cgtttcgcgt ttgagtttcg ttaggagtac gggagttacg gagcgggagg 1260
   agggagggag gagcgagcgc ggcgtggggc gacgtcgggt tgcgcgcgag cgatcggtgt 1320
   aagtgtgcga gacgcgggga gtgcgggggc gttgttgttt ttagttttcg tttttttttt 1380
   ttttttttcg tttttttttc gtttttttcg tcggtcgttt ttcggaggcg ttcgtttagc 1440
   gtttttgcgt tcgcgggata gggtcgtttg tataaggcgc gcgttcgcgt ttttttatgc 1500
   gcgttttcgg gggttttttt tttgtttcgg ggtgtaggtg gtaggttggg gagtatagtt 1560
   cggttggagg cggtaggagt tcgttcgttt tttattcgtt attaatttgt agattgtagg 1620
   gcgagtgggc gtttagaggt tgttttcggg tggagtggat tagattcggt cgagaggttt 1680
   agtgttttgg tttgttgttt tgtttttttt agttcggaat agggagttgg ggttgatagg 1740
   gtatatttta gttttttaat tttagtttta ggggagggcg ttttttttcg cggagcgacg 1800
   ggttgattgg tttagtgtat ttttttttta tttttattta ttcgagagtg gcggtagggg 1860
   gtgtttaggt tgggtagttt tttttgggat tttgttgtta gtcgatagat tgagtcgggt 1920
   tgaggtttag ttgtttcgcg cggaatagta gtggggcgtt tgagtcgcgg gagcgtgagg 1980
   ggcgtagttg gtcgtttaga gcgcgttcgc ggcgttatta ggcggacgcg gcgggtggtg 2040
   tatttcggtt tcgttttcgg gaatagaatg ttttgagtgt tttggtcgat tcgagggggt 2100
   taatattttt ttttggtgta tattttattt agtgagttag ggggagttta atcgtttttg 2160
   gaaattgggt ggtgttaaga tttcgagcgg tagtttatgt ttttgttagc gaatgtttat 2220
   ttttcgggta tttgtttttt gtttgtacgg atcggtttcg tttgttattt gggggttgtt 2280
   tagtttttag gagcggttgc gtttgcgttt tcggagagtg tttttttcgg gttttaggga 2340
   ttgagttttt tgggagtggt tgtggttttg gtttggtgtt tttgttggat ttgttgtttt 2400
   tatgtttagg tttggtaaat ttgaatttag gttttagttg aagatattga ggtttaagga 2460
   ggtgaaggtg tttgtttaga gttattagtt agcgagtggt ataattatat gggaaataaa 2520
   gtatgtttaa ttgtagtaat tttttgtatt tagttttgat tattttttaa gattgttttt 2580
   ttaaattata tatatatata tatatatata tattcgaaag ttattattta ggtatttatt 2640
   tatattttat attttgtgtt aattacgata gtgggtatcg tggatataag gaagaagaaa 2700
   ggtaatagtt tttttttttg gggttaggaa ggagatagat gataagtgaa taagaataga 2760
   gttgtagata gtagattatt aagtagggtg atgggagagg gaatattggg tgttggaggg 2820
   ttaattttag atttgaggag gtggtatttg aggtgaggtt tgaaggatat agtattaggg 2880
   gtttagtatt gtaggaaggg gattagtagg tgtaaaagtt tagaggtggg tatgagtttt 2940
   agggaggaga tggtgggttg taatagagag agagagaagg tttgagtttg aagggagatg 3000
   gggatatata gatttataaa gttgtgtggg tacgttaagg agtttggatt ttagtggtgt 3060
   gtttagttgg ggatattgga gggatgaaga agggggtgat tgatttgatt tgtgtgttta 3120
   aaagtttttc gtggttgttt taagggaata tattgtggga atggggatag gtgagtaaag 3180
   tgcgttatta agtagattag agaggagatg atagtagggg tttaggtgag atattggtag 3240
   tttgggttag ggtggtgttt ttagaatatg attataaaat tatttgatat tttttttatg 3300
   aagaagtggg ttttttgtgt tttgtttgcg tgaatttggg tgggtttgtg atggttttta 3360
   ttaataagat gtagtagaag tgatgtttaa aaattataag ttttcgcgat aaaagataat 3420
   gtagtttttg ttttgttggt tggcgtatta gtgttggggt tttgagttat tatggaagag 3480
   gtttagaggt tattatgttg tgaggaagtt taggtatagg tagaggttat ttgtgtttgt 3540
   tttagtttat agttttaggt gagtttagta ttggagttat tttgtttagg tgtttagaaa 3600
   tgtgggtgaa ggtattttta ttattatgtt atgttggttt tgtgtttgtt aagttaaagt 3660
   atggtggata gggggtcgtg taggtatttt tagtggtatt ttgttaattt tttgagtttt 3720
   ataatttgtg aatataatga ttattttgtt attaagtttg gaggggatgt ttgttatata 3780
   gtagttgtaa ttaggataat aagggtagaa gtggggtgta gagaggagaa aaggatttag 3840
   gatacggggt ttgtgttttg tatagatagg atttgaaagg attcggtggg ggtgggtaga 3900
   gaaagagaaa gaggaatgat aggtgatttc gatgttggtt ggagtatttg ggtgaatgga 3960
   ggtggtttgt aatgatttgg agatggttgg aggagttaag agtttggtaa aggaaattat 4020
   aaggtttgtt ttgattatgt tttttttttt tgtattgttt tttttttttt tttttttttt 4080
   ttatttttag atgaagttag tataagtttt gagtagttta tttttaaaga atttttaaat 4140
   atgattttta ttttttgtat ttattattat ttttagatta aagttttatt tatgttatga 4200
   atgttggggt gtatttgtga tcggatgtta ggttaggtgt taggtatata ttaatagttt 4260
   atgtaggtgt tgggtatatc ggaagatgag agaggaggag gaggaagaag aggaggaata 4320
   tagtagggaa gaatatggtt aagatagatt agtatattaa ataaatttag tttttgtttt 4380
   ttttggagta atttatttga ggatcgattt tatgattaaa tatataggta gtattattat 4440
   atgttaggta ttgttattta tagttttagt atattaattt attgaatttt ttgaaaatgt 4500
   taaggggtag gtattttatt tgttgatatt attattatag ttattattat ttgatagaat 4560
   gaggaaagtg aattagggag ggattaaata aagtattaaa tttttatagt tagtttatgg 4620
   ttgagttgag tttcgaattt agaaaatatt gattttagta tttgttttta atttattttt 4680
   tagttttatt aggttgttta tttaatggag gagggggaag gatttgggat attttaatag 4740
   gaaaaaaggt gttaggtatt aagatgtgtg gtgtatagtg ggttgttgga atatagagga 4800
   gttttttagt tagttttaga ggattaggga aggtttttta gaggaagtgt ttttaaaatg 4860
   ggattttaaa agagtgagtt ggtattaagt aagtgagttt ttgaaattag aggaaatagg 4920
   gttacggagg tttaaggtgt ttggatattg gggcgttaga gggaaggaga tatgtaggga 4980
   agaggtttaa tggggagttt agggatatag ggtatattaa ggggggtagt agtgttatat 5040
   gattaggttt ttgttttaga aggtttatgg tggttgttat gggagttaat agaggaggga 5100
   gtaagagagg agggtaagtt aggaggtttt aatagttatt tgtgggagag aaggtttatt 5160
   ttggatagat ggtggggaga gaagggggtg gatttgagat cgagtttgaa ggtagggtag 5220
   ataggataag gaaggttttt aggatagtgt tttgttttgt gagagtgtgg ttggtgagaa 5280
   aaatatagat tggggatttt gggagtagga ggttttaggg agtgaaattt ggttttggat 5340
   atgttgagtt tgaagtgttt gtaggttatt gaagggtgat tggtgttcgg ggagaggttt 5400
   ggggttgtta tttttaggtt ggatattttt agttagaagt gggtagatat ttaaattaag 5460
   gtaaggagag gatattttgg gagattaaga aggatgagtg gggaaggttt agtgtagagt 5520
   tttaggaatt tagtatgatt taaagaatta gttggggaaa tggagggatt aaaggtgata 5580
   aagataagtt tgagaggtag ggagtttatt agatagtgag agaaggaaaa ggaatttgta 5640
   gtgtttggtt ttatagggaa ggaaggagag gtagaattaa aaagggtttt attttattag 5700
   tttaatattt agagattttt atggtttagt ttaaaattat ttttatagtt tttatttttt 5760
   ttaaatattt gtgtatatat tataataaat tagtgggttg ttattagtat tttataaaag 5820
   aaatagatta gaaaataata gagggtatta tgtataaggt tttaattgtg tgtgtgcgtg 5880
   tgtgtgtgtg tgtgtgtgtg tgtgtgtgtg tgtattggat gtgtatgtgt atatattttg 5940
   ggttaggatt ttttaaaaag ttgagagatt attattgtag tttgtttttt tttgtatatg 6000
   tttagaatgt ttttagtttt tttttatttg gtaaattttt aatttatttt ttaaatttta 6060
   tttaaatgtt aatttatttg aaaagtttt 6089

<210> 273
<211> 6197
<212> DNA
<213> Artificial Sequence

<220>
<223> chemically treated genomic DNA (Homo sapiens)

<400> 273
   gaaattattt taattaatag aaaaagaggg aatttttttt aatttatttt atgaggttaa 60
   tattattttg atattaaagt ttggtagaga tataataaaa aaagagaatt ttagattaat 120
   atttttggtg aatatcgacg taaaaatttt taataaaata ttggtaaatc gaatttagta 180
   ttatattaaa aagtttattt attacgatta agttggtttt atttttggga tgtaaggttg 240
   gtttaatata tgtaaattaa taaatgtaat ttattatata aatagaatta aatataaaaa 300
   ttatatgatt attttaatag atgtagaaaa ggtttttaat aaaatttaat agttttttat 360
   gttaaaaatt tttaataaat taggtattga tgggacgtat tttaaaataa taagagttat 420
   ttatgatata tttatagtta atattatatc gaatgggtaa aaattggaag tatttttttt 480
   gaaaattggt ataagatagg gatgtttttt tttattattt ttagttaata tagtgttgga 540
   agttttggtt agggtaatta ggtaagagaa agaaataaag ggtatttaat taggaaaaga 600
   ggaagttaaa ttgtttttgt ttgtagatga tatgattgta tatttagaaa attttatcgt 660
   tttagtttaa aattttttta agttggtaag taattttagt aaagttttag gatataaaat 720
   taatgtgcga aaattataag tatttttata cgttaataat agataaatag agagttaaat 780
   tatgagtgaa tttttattta taattgtttt aaagagaata aaatatttag gaatttaatt 840
   tataagggaa gtgaaggatt tttttaagga gaattataaa ttattgttta ataaaataaa 900
   agaggatata aataaatgga agaatatttt atgtttttgg ataggaaaaa ttaatatcgt 960
   gaaaatggtt atattgttta aggtaattta tagatttaat gttattttta ttaaattatt 1020
   aatgattttt tttatagaat tggaaaaaat tattttaaag tttatatgga attaaaaaag 1080
   agttcgtatt gttaagataa ttttaagtta aaagaataaa gttggagtat tacgttattt 1140
   gattttaaat tatattataa ggttatagta attaaaatag tatggtattg gtattaaaat 1200
   agagatatag attaatggaa tagaatagag gttttagaaa taatgttata tatttataat 1260
   tatttgattt ttgataaatt tgataaaaat aagaaatggg gaaaggattt tttatttaaa 1320
   aatggtgtta ggaaaattgg ttagttatat gtagaaagtt gaaattggat ttttttttta 1380
   tattttataa aaaaagttaa tttaagatgg gttaaagatt taaatgttag atttaaaatt 1440
   ataaaaattt tagaagaaaa tttaggtaat attatttagg ttataggtat gggtaaggat 1500
   tttatgatta aaatattaaa agtaatggta ataaaagtta aaattgataa atgggattta 1560
   attaaattaa agagtttttg tatagtaaaa gaaattatta ttagagtgaa taggtaattt 1620
   atagaatgag agaaagattt tatagtttat ttatttgata aagggttaat atttagaatt 1680
   tataaagaat ttaaataaat ttataagaaa aaattaaata attttattaa aaagtgggta 1740
   aaggatatga ataggtattt ttttaaagaa gatatttatg tagttaatag atatatgaaa 1800
   aaacgtttat tattattggt tattagagaa atgtaaatta aaattataat gaggtattat 1860
   tttatattag ttagaatggc gattattaaa aagttaggaa ataataggta ttggagaggg 1920
   tgtggagaaa taggaacgtt tttatattgt tggtgggatt gtaaattagt tcgattattg 1980
   tggaagatag tgtggtgatt ttttaaggat ttagaattag aaatattatt tgatttagtt 2040
   attttattat tgggtatata tttaaaagat tattttgtta taaagattat ttataatagt 2100
   aaagatttgg aattaattta aatgtttatt aatgatagat tggattaaga aaatgtggta 2160
   tatatatatt atggaatatt atgtagttat aaaaaaggat tagtttatgt tttttatagg 2220
   gatatggata aagttagaaa ttattatttt gagtaaatta tcgtaaggat agaaaattaa 2280
   atattatgtg tttttatata taggtgggaa ttgaataatg agaatatttg gatatagcgt 2340
   ggggaatatt atatattagg gtttgttgtg gggttggggg aggggggagg gatagtattg 2400
   ggagatatat ttaatgtaaa tgacgagtta acgggtatag tatattaata tggtatatgt 2460
   atatatatgt aataaatttg tatgttgtgt atatgtattt tagaatttaa agtataataa 2520
   aaaaaattta aaattaaaaa aatttaaaaa agaaagttgt atattattaa atgagaaaag 2580
   taatttgtaa aatataattt atataatttt atttttgtgg aaaatgttta tatatatatt 2640
   tatatatgta tataaaattt tttgaaagat tatttaaaat ttaattgttg ttattttaga 2700
   ggggagaagg taggttatta aaggggattt gtattattta tttttgttat gtttaagttt 2760
   ttattataag aatgtattaa ttttgtgtaa aataataaaa gtaggttagg cgtggtggtt 2820
   tacgtttgta attttagtat attgggagat taaggttgga tgatttcgtt aagttaggag 2880
   tatgagatta gtttggatag aatagcgaga ttttgttttt aaaaataaat agatagatag 2940
   atagatagat agatagatag atagatagat agatagatag atagatagat agatagatag 3000
   atagatagat agatagatag ataaataaat aataaaagtt ttaatttttt tttttgtgga 3060
   agaaatttta aggggatagg atagtgtttt aatgaattta ttttaaatta gttttttttt 3120
   tttatataaa aattgtttaa atttgtttta ttttttaatg aatttttgta ttttttagga 3180
   gttttttagt taagtaaatg gtaaaaattg tggttaggga agaagttaag gttgaaattt 3240
   aggagttatt gttttttatt tttttagaat ttgatttttg gtttgaaata gattttattg 3300
   tttttttttt gaatgaagag agaggaggat ttaaagttgt ttgtgtagga gttataattg 3360
   attaagttta ttttataata aggtttggag ttttaaataa agatttggtt tagaagattt 3420
   tatgtggata ataaaattat atagttaaga tattgaggtg aagatttttt aaatcgtttg 3480
   gtttaggtaa tggagaaagt gaatttgtgt attaattttt tattttttgg tttagtttag 3540
   ttagagaggg ttttggagta agaggaataa ggttaaaaga aaagaataaa gattattgtt 3600
   ttagggagat tttaagaaag gtgatgcgga tgttaaagta gaaggtgtga aaatagttag 3660
   aaagaatgaa gaagatttag tatttaatag tataataggg tgattatcgt taataataat 3720
   tttattgtat atggtaatta agagtataat tagattgttt gtaatataaa ggattaatgt 3780
   ttgaggtgat ggatatttat ttattttgat gtgattatta tgtatttatt gtatgtttgt 3840
   attaaaatat tttatgtata tgtattttat aaatatatat atttattatg tatttataaa 3900
   agttaattga aatttttttt aaaagatggg gttatatggt tatatagata ttagtttgga 3960
   gttttttgag gatttttagt gttgtggttg aggttatttt tttttttagg gaatagaagg 4020
   agtagagttt tagagaagtg tgagcgaggg tttgggtgtt tttttggttt cgttatttga 4080
   gtttaagaaa gtagggattt tgagattatt tttgtgtgtt tattgaaatt tttttttttt 4140
   ttcgaaattt aaattttatt ttttttagaa aatttttttt aaataagttt atattattat 4200
   aggtagtttg tagtttttta atattttatt aaattgcgtt attttattgt tattttatta 4260
   ggttgtaaat gttgttaggg tagaaattat gttttggttt ttaaaaattt ttataatgtt 4320
   tagggtggtg ttgagaataa aataggtatt tggtaagtaa ttgtgtgatg aaaaagttga 4380
   aaggaagaaa aaattatcgg gttttttgaa gtttttagtt tttttaattt agatagagaa 4440
   tatattagat ggtaggattg tatttttata tttttaaatt agagggtgga ggtttatttg 4500
   tatttggtgt attttttttt gaaaaaataa aataaataaa aaaatgttat tttgaatgta 4560
   aatttagttt taaagtgtta tagaggataa ttaggaaagt atagtttttt ttggattttt 4620
   tttggggatt aggttgtgtg tgtgtgtgtg ttgtgtgtgt gtgtgtgtgt gtaagtgtgt 4680
   ataggtggga aaaggttgtg gtgtgatggg aggggatagg taagagggag gaaagagatt 4740
   ttatgtagtg attatgtgga ggggatttgt tatgtttttt tttttcgttt ttttttttgt 4800
   tttttttttt ttggtttttt ttttttgtat ttgtttggga ggattttgga gtgttttttt 4860
   gatcgattta attttttttt ggtatttgag ttttgattgg ttgaaggatt tagaggggaa 4920
   aaaaaatttt aattaagtag ataatttttt ttttggacgt ttggtagttt tattttattt 4980
   ttttttaatt ttgtttggga tcgtttatat attaaggaag ttgggttttg agaattttat 5040
   tttattggta ttgaggagaa tattttttcg ttttgtttat ttatttttta gttttttgga 5100
   atttttttta gttgttattt tagaggatta tgtttttttt aaagtttttt gtgtatattt 5160
   gtttttttat ttgtgttttt taatttagtt atagttggta agtaatttgg atttttattt 5220
   ggagttggga ataggtaagt gtatgtaatg tgttgggtaa agtttatttt atttttagaa 5280
   atgtaggaag ttttgatagt ttttttagga aaaattgttt tatggggtag ggtttttttt 5340
   aaatttatgg ttgattttag tgaattttta ggatttttat taataaagga ttaaatgatg 5400
   ggagcggggg tttttatttt tataaattgt tattttataa atattttttt aaagtgattt 5460
   gggggtaaat ggatagtagt tttttttacg aaattttgat gttagtattg tagagaggag 5520
   gaaggtagtt taaagtaagt tttatcgtgt gttgaatttg attttgaggt aaagtttgga 5580
   gataaaattt aatgggattt tattttgtat tttttgattt tatttttttt tttaggatat 5640
   tttaagtaat tgtttaaatt attatttttt agatataaaa aattttattt gaattattaa 5700
   gtaggtattg ggttaataaa tatgtttgaa attagatatt ttagaaattt aaacgtttcg 5760
   agaaatttat ttaatttgtt taatttataa atatttttaa attgtttaga aaattagata 5820
   acgtatatga aagtgttttg taaattataa atataaatgt taaagaatat tattattatt 5880
   tttatttagt ttttggaaat tatataaatt atttatatat gtaattgttt attagtaaat 5940
   atattagtat ataataatat atagaatgta ggttgttagg gtgttcggag ttttttttta 6000
   gtttagatat gagtttagtt ttttttttta ttagttttta ttttgattat ttatttaata 6060
   aagatataaa tttaattgtt atataaattg ttgaggaagt aggaaaagaa atttttttgt 6120
   tgagataagt tttttttgag aattgaaaat atatttttat tataaatgag tgtagtcgtt 6180
   tttataggag tgttgat 6197

<210> 274
<211> 6197
<212> DNA
<213> Artificial Sequence

<220>
<223> chemically treated genomic DNA (Homo sapiens)

<400> 274
   attaatattt ttataaagac ggttatattt atttatgata aaaatgtatt tttaattttt 60
   aaaggaagtt tattttagta aagaaatttt tttttttgtt tttttaatag tttatgtagt 120
   aattggattt atatttttgt taaatagatg gttaaaatga gagttgatga agagagagat 180
   tgagtttata tttgaattgg agagaaattt cgaatatttt agtaatttgt attttgtatg 240
   ttattgtgta ttggtgtatt tgttaatgaa tagttgtatg tatagatagt ttatgtggtt 300
   tttaagagtt aaatgagaat ggtgatgata ttttttagta tttgtgttta taatttataa 360
   agtattttta tatacgttat ttgatttttt gaataattta gaaatatttg taaattgaat 420
   aaattaaata aatttttcga gacgtttaga tttttaaaat atttggtttt aaatatgttt 480
   gttaatttaa tgtttgttta gtggtttaaa taaaattttt tatgtttagg aaatgataat 540
   ttgagtaatt atttagaata ttttgaaaaa aaaaataaag ttagaaagta taaagtaaga 600
   ttttattgaa ttttgttttt agatttattt ttaagattag atttaatata cggtagaatt 660
   tgttttgaat tatttttttt ttttttataa tgttgatatt aggatttcgt gaaaagggtt 720
   gttgtttatt tatttttaaa ttattttggg ggaatatttg tggaatagta atttataaaa 780
   ataggaattt tcgtttttat tatttagttt tttattggta ggggttttaa agatttatta 840
   aaattagtta taaatttagg aagagtttta ttttatagaa tagttttttt taggagagtt 900
   attaaggttt tttatatttt tggaaatgaa atgagtttta tttaatatat tatatatatt 960
   tatttatttt tagttttagg taagagttta ggttatttat tagttgtggt tgagttggag 1020
   gatataggtg aaaagataga tgtatataga aggttttgaa agaaatataa ttttttggag 1080
   taatagttag agaaaatttt aaaaaattgg gagatgggta agtaagacgg agaaatattt 1140
   tttttagtgt tagtgggatg gaatttttaa agtttaattt ttttggtgtg taagcgattt 1200
   taaatagagt taaggggagg tgaaatggag ttgttaaacg tttaaagaag agattattta 1260
   tttaattaaa gttttttttt ttttttaagt tttttagtta attagaattt aagtgttaga 1320
   gaaagattga gtcgattaaa agaatatttt aggatttttt tagataaatg tagaagaagg 1380
   ggattaggag aaagaggata aagaaagaag cggggggaag agatatagta aatttttttt 1440
   atataattat tgtatggagt tttttttttt ttttttgttt attttttttt attatattat 1500
   aatttttttt tatttatgta tatttatata tatatatata tatataatat atatatatat 1560
   atagtttggt ttttaaaagg gatttagaaa ggattgtgtt tttttggttg ttttttgtag 1620
   tattttgaga ttgaatttgt atttaggata gtattttttt gtttgttttg tttttttaga 1680
   aaaaaatata ttaggtgtag gtgaattttt attttttgat ttgagaatgt ggggatgtag 1740
   ttttattatt tagtgtattt tttgtttaag ttagaaaggt tagaaatttt aggaagttcg 1800
   atggtttttt ttttttttta atttttttat tatataatta tttattaaat gtttatttta 1860
   tttttaatat tattttaggt attgtggaaa tttttaaaaa ttaaaatatg atttttgttt 1920
   taataatatt tatagtttag taaggtgata ataaaataac gtagtttaat aaagtattag 1980
   aaagttataa attgtttata atagtgtagg tttatttagg ggaggttttt tgaagaaggt 2040
   agaatttgag tttcgaagaa ggaaagagat tttaataggt atataaaggt gattttaaaa 2100
   tttttatttt tttaaattta ggtggcggag ttaggagggt atttaggttt tcgtttatat 2160
   ttttttggga ttttgttttt tttgtttttt agggaggagg gtggttttag ttatagtatt 2220
   ggggattttt aaagggtttt agattgatgt ttatgtagtt atatagtttt attttttaaa 2280
   aaaaatttta gttaattttt gtggatatat agtaggtata tatatttatg gagtatatgt 2340
   atatgagata ttttgatata ggtatataat gaatgtatga taattatatt agggtaaatg 2400
   agtatttatt attttaagta ttaatttttt gtgttataaa tagtttaatt atatttttag 2460
   ttattatgta taatgaaatt attattgacg atagttattt tgttgtgttg ttgaatatta 2520
   ggtttttttt atttttttta attattttta tattttttat tttagtattc gtattatttt 2580
   ttttaaggtt tttttgagat aatggttttt gttttttttt tttaattttg ttttttttgt 2640
   tttaaaattt tttttggtta gattgaatta gaaaataaag aattaatata taaatttatt 2700
   ttttttatta tttaggttag acgatttagg gggtttttat tttagtattt taattgtgta 2760
   attttgttgt ttatatggaa ttttttagat taagttttta tttggggttt taaattttat 2820
   tgtgagatga gtttggttag ttgtggtttt tatataaata attttaggtt tttttttttt 2880
   tttatttaga aagggagtag tagagtttat tttaggttaa gagttaggtt ttaggaagat 2940
   aaggagtaat gatttttgga ttttagtttt gatttttttt ttgattatag tttttgttat 3000
   ttatttgatt agagaatttt taagggatgt aaaaatttat tgggaggtag agtagatttg 3060
   ggtagttttt gtgtaaaaag ggagagttaa tttaaaatga gtttattgag gtattgtttt 3120
   atttttttag aatttttttt ataaaggaga aagttaagat ttttattgtt tgtttattta 3180
   tttgtttgtt tgtttgtttg tttgtttgtt tgtttgttta tttatttatt tatttattta 3240
   tttatttatt tatttattta tttatttatt tatttttaga gatagggttt cgttattttg 3300
   tttaggttgg ttttatattt ttggtttggc gagattattt agttttggtt ttttaatgtg 3360
   ttgggattat aggcgtgagt tattacgttt ggtttatttt tattgttttg tataaaatta 3420
   atatattttt ataataaaaa tttaaatatg atagagataa atagtgtaaa ttttttttga 3480
   tgatttgttt tttttttttt aggataatag tagttaagtt ttagatggtt ttttaagagg 3540
   ttttatatgt atatgtaaat atgtatataa atatttttta taaaaatgag attatataaa 3600
   ttatattttg taaattgttt tttttattta gtaatatgta attttttttt ttaaattttt 3660
   ttaattttaa atttttttta ttatatttta agttttaggg tatatgtgta taatatgtag 3720
   gtttgttata tatgtatata tgtgttatgt tggtgtgttg tattcgttaa ttcgttattt 3780
   atattaagta tattttttaa tgttattttt tttttttttt ttaattttat aataggtttt 3840
   ggtgtgtgat gttttttacg ttgtgtttaa gtgtttttat tgtttaattt ttatttatgt 3900
   gtgagaatat atggtgtttg gttttttgtt tttgcgatag tttgtttaga atgatggttt 3960
   ttagttttat ttatgttttt atagaggata tgaattaatt tttttttatg attgtatagt 4020
   attttatggt gtatatgtgt tatatttttt taatttagtt tattattgat ggatatttgg 4080
   gttggtttta agtttttgtt gttgtgaata gtttttatag tagaataatt ttttgagtat 4140
   atgtttagta atgggatggt tgggttaaat ggtattttta gttttagatt tttgaggaat 4200
   tattatattg ttttttataa tggtcgaatt agtttatagt tttattaata gtgtaaaagc 4260
   gtttttattt ttttatattt tttttaatgt ttgttgtttt ttgatttttt aatgatcgtt 4320
   attttaattg gtgtgagatg gtattttatt gtggttttga tttgtatttt tttgatggtt 4380
   agtgatgatg agcgtttttt tatgtgtttg ttggttgtat aaatgttttt tttagagaag 4440
   tgtttgttta tattttttat ttattttttg atggggttgt ttgatttttt tttgtaaatt 4500
   tgtttaagtt ttttgtagat tttggatatt agttttttgt tagatgggta gattgtaaaa 4560
   tttttttttt attttgtagg ttgtttgttt attttgatgg tagttttttt tgttgtgtag 4620
   aagtttttta gtttaattag attttatttg ttaattttgg tttttgttgt tattgttttt 4680
   ggtgttttag ttatgaagtt tttgtttatg tttatggttt gaatggtatt gtttaggttt 4740
   ttttttaggg tttttatggt tttaggttta atatttaagt ttttaattta ttttgaatta 4800
   atttttttta taaggtgtaa ggaagggatt tagttttagt tttttatata tggttagtta 4860
   gtttttttag tattattttt aaatagggaa tttttttttt attttttgtt tttgttaggt 4920
   ttgttaaaga ttagatggtt gtagatatgt ggtattattt ttgaggtttt tgttttgttt 4980
   tattggttta tatttttgtt ttggtattag tattatgttg ttttggttat tgtagttttg 5040
   tagtatagtt tgaagttagg tagcgtgatg ttttagtttt gtttttttgg tttaggattg 5100
   ttttggtaat gcgggttttt ttttggtttt atatgaattt taaagtagtt ttttttaatt 5160
   ttgtgaagaa agttattggt agtttgatgg ggatggtatt gaatttataa attattttgg 5220
   gtagtatggt tatttttacg atattgattt tttttattta agagtatgga atgttttttt 5280
   atttgtttgt gttttttttt gttttgttga gtagtggttt gtagtttttt ttgaagaggt 5340
   tttttatttt ttttgtaagt tgggttttta ggtattttat tttttttgaa gtaattgtga 5400
   gtgggagttt atttatgatt tggttttttg tttgtttgtt attggcgtat aggaatgttt 5460
   gtgattttcg tatattgatt ttgtattttg agattttgtt gaagttgttt attagtttaa 5520
   ggagattttg ggttgagacg atggggtttt ttaaatatat aattatgtta tttgtaaata 5580
   gggataattt gatttttttt ttttttaatt gaatattttt tatttttttt ttttgtttga 5640
   ttgttttggt tagaattttt aatattatgt tgattaggag tggtgagaga gggtattttt 5700
   gttttatgtt agtttttaaa gggaatgttt ttagtttttg tttattcggt atgatattgg 5760
   ttgtggatgt gttataaata gtttttatta ttttgagata cgttttatta atatttagtt 5820
   tattgagagt ttttagtatg aagggttgtt gaattttgtt gaaggttttt tttgtattta 5880
   ttgagataat tatgtgattt ttgtatttgg ttttgtttat atgatggatt atatttattg 5940
   atttgtatat gttgaattag ttttgtattt tagggatgaa gttaatttga tcgtggtgga 6000
   taagtttttt gatgtggtgt tggattcggt ttgttagtat tttattgagg atttttgcgt 6060
   cggtgtttat tagggatatt ggtttaaaat tttttttttt tgttgtgttt ttgttaggtt 6120
   ttggtattag gatgatgttg gttttataaa atgaattagg gaggattttt ttttttttta 6180
   ttgattggaa tagtttt 6197

<210> 275
<211> 6675
<212> DNA
<213> Artificial Sequence

<220>
<223> chemically treated genomic DNA (Homo sapiens)

<400> 275

ttatggtgtg gaaaaaaatt tttggaaata gatttatgta ttagtttttt attttgttat 60
tagatgtttt ttatgagtag gaaaatatga tgatttgaat gatattttga agtgtgaatg 120
tttagtattt ttgagtagat agtgaaagcg gagtgtgatt gaatgtaaat gttattatag 180
atttttggat gagataggtt ttttgaaggt tgttttatag gtgtagtttt tagaagtaat 240
atttatttta tttgtaaatt ttttattttt atattttata attatttatt tatttttttt 300
ttatttaatt ttaaatcgat attttaaaaa tttgatcgaa ttattatatt ttgaaattaa 360
tattaagaag tatttaggtt tttatgttaa tagtaaagat aaaaagttgg tttttggagt 420
ggaacgtttt aataatttaa ggaggttaga ttttagtttt ttaaatatga tagttatata 480
gtttataaga tttttttttt ttttttttga gatagggttt tgttgttatt taggttggag 540
tgtagtggtg tgatttggtg atttgggttt attgtaattt ttattttttt ggtttaagcg 600
atttttttat tttaattttt tgaggagttg gaattatagg cgtgtattat tatattcggt 660
taatttttgt attttttttt tttttttttt tttgtagaaa taaggtttta ttaggttgtt 720
tagtttgatt tcgagttttt gggtttaagt gatttgtttg ttttagtttt ttaaagtgtt 780
gttattatat atttggttaa gatattttta ttaagttaat tttaaaagta gtaatttata 840
tttttaggaa atgtagttga aagggattaa tgtaaaattt aatataaaat ttaagattga 900
agttgtagtt tattgttaat aaggtaatat agaatatgtg tttaatatgt taaattaata 960
aatagtatgt ttataaaata gaagttattt ggttaagtgt gttataaatg tataatagaa 1020
atttttagaa tatataagga ataaggataa ttataggttt aaaggatttg ttgcgtgaat 1080
tatatggtgt tttaataaat atttggagat agaattttcg ttttattttt attattattg 1140
gaagtatggg atgaatttgt gttttttggt gagggttttt tattttttta ttaagtgggg 1200
tagggagatg ggataggtaa cgtattgtta gaaaattgaa ggttatttgt atgaaatgtt 1260
agttaagtaa ttagatgttt gattggattg gtttttatat tcggtttttt gatcgaattt 1320
tttttttttt tatttagatg ttttagaaat atagagaatt ttatgaagtt tgtttcgggt 1380
tcgtagagga aggggcggta atattttggt gttttgtagg gggcgaagtt atttttaatt 1440
ttattaagtc ggtttatata ttttagcgga ttttaaagtt tagatttgtt tttgtttttt 1500
taattttatt tattttatgt tttttttttt tgaattatat gtggacgttg tttagttttt 1560
tgttttgagg tttttagggg agtattcggg ttgtttaatg ggaagggttt ttagaagtag 1620
ggagtagata aggtgggggt taggggattt ttatttattt agaggtttaa ttggtttgga 1680
gaaagagtag ttttaatttt aaggttatta aaattttaga attgtatttt tttttgtttt 1740
ttttgtttta atggatataa gggtttgttt gtatgtgttt cgtaaatatg atgtgtatat 1800
gttgggagtt atagtggata gaagaggagt atatttttag ttggatgtta ttgtttttat 1860
gattattgta atgttttatt gtatagtttt tttaggagtt ttttttggtt tttaagggag 1920
atgtgcgttt ttatgtttat tatttggagg tattttcgat atttttttat tttagtttta 1980
ttcgatagaa attgagaatt tattttggtg gagtgaggag tttgtaatag atttgaatta 2040
gagttgttgt ttagatgttt agttgatagt tattttagat ttatgttata tgaatttatt 2100
tattttttag tgtttaagga aagggagaga tggcgtgcgt gtgtgtgtat gtgtgtgtgt 2160
gttttttgta ttaggtttat tttatagatg tatatataag ttgagtattt tttatttaaa 2220
gtgtttggga ttagaagtat tttaaatttt agatttttag attttggaat atttgtatat 2280
atataatgag atattttagg gatgggattt aagtttaaat atgaaattta tttatgtttc 2340
gtatatattt tatatatata gtttgaaggt aatttaatat aatattttaa atgattttgt 2400
ggatgaaaat tttgattgtg ttttgattat gttttatttt atgaggttag gtgtggaatt 2460
ttttatttga ggtattatgt tagtttttag aaatttttag attttggagt attttggatg 2520
ttagattttt gggttaggga tgtttaattt ttataatagg tgggttattt ttcgattttg 2580
tgttttaaat gatatagttt tattaaggtg tttttattta tgtttgaaat gttgagtttt 2640
ttgtgttttt tttattgttt tcgtaggtag aaagtttttt attttgtttg tattttatta 2700

gaagttttat gaggaaggtg taattaggtt taaaaaagga ggtttttggt aggaagttta 2760
ggtgtatttg ttggaatttt agattttttg agtagagttt ttggttgttg tggggtattg 2820
tagtgaattt atgtttttaa tatgtttttt atagaaaaaa gaagtgatag ggtttttgaa 2880
ttttgagggt tcgggtattt tagataaagt atcgtattat tttttcggat ggaagaaatt 2940
tttgtagaga tggttaaaga ttggtattta tataagtttt gtgttttgaa atttatagtt 3000
attagatatt tgtagaaata aagatttttt taaatgatta tttgtgtttg ggttaaggta 3060
gaattagaaa gaaggattta gtgggtagtt ggagttagtg gtggttttta tttatttatg 3120
agaaatagat ttttggaaat aggataggaa tgtttataag atttggatta atagaaagtt 3180
tatgatttta taaatttatg aattttatag tttataattt atttgatttt atttttaaat 3240
gtttaggatt atttggatta aggttggtaa gttagaaaat tgtagttttt atttttaatt 3300
ttatataatt tttattaagt aaaaaatttt tatgttttac gtgaatttgt ttttacgatt 3360
tatagatgat tggatgtttt cgggcggggt aaaggtaaat gaatttgtgt ttttattggt 3420
aaatggtgtt ggtggtggtg ttattttgtt taaaatttat aggaatttgt gttttaggat 3480
tttgtttatt gttttgatta tttagaatgt atgttaagtt tttgtttttt ttggagttat 3540
ttttattatt tttttttaag ttttattttg tattatataa gttatattgt attttaatat 3600
taaaatgtaa tattaataga taagttggag aattaagata tttttaagaa gataggggac 3660
gtttttttta atatatcgtt tgattatatt tttttttttt tgggtgttag tttaagaagt 3720
taaattaaag tataaatgaa tagagaagtt aatatattcg attttaaatt tttaggacgt 3780
tggtttgggt tatatgtggt tttttttgtg gttagtttgt gagtagtttt ttcggagggt 3840
gtgttttaat ttttagtagt tgtcgtggaa agttacgtgt tcgttaaacg tttatattat 3900
attaataaga gaaattaagg aagggatgga aaagttatta tttttttttt tttttttagt 3960
tttggtagta tagtgattta atcgttgtta ttatttttat ttagttaaag tattaattta 4020
ggttttttaa atgatggtaa aagagaaagt tttagatcgg ggtattggtt agattatttt 4080
tattagcgat gattttttcg tttttggtat ttcgcggttt ttcgtatgtt tttaagaatt 4140
ttcgggtttt gaaatttacg cgcggcggag gcgacgtttt tcgggagagg gagggtgaaa 4200
gaaggggcgg ggttatttaa atagttcgcg agtttcgcgt ttttattggt tgttagtttg 4260
tcggttatcg cggggcgggg agtttcgggg ttggagtgag gtatttgagt cgggcgcgta 4320
gtagaaagtt tacgttggtc gttgcgggga gcgcggcgtg gagaggcggg agatcgaatt 4380
tcggcgtcgt cgtcgcggtc gttgttcggt gtattagttt tttttttttt tatttttttt 4440
gcgagcgggt ttgttgggta gtcgggcgat cgtcgaacgg aaagtaagat tttcgtcgtt 4500
ttcgtttgag gaggcgtgcg gcgcgcggag attttgagtg ggagcgacgg tgttcgagag 4560
tttaggggcg cggagttcgt gagcgagaag gaagttaaat ttcgcggaat agattggtat 4620
ttcgggacgt tttgtcgtcg tagtttgggt tttgtaacgg ggtaaatttt atggtggttt 4680
tgcgatttgg ggaggggcgt gcgttacggt gatgtcgggt atttttcgta gttcgtgagc 4740
ggtttcgttt ttcgttgttt cgggttttat ttagtcgtta gggagagcgc ggggtagcgt 4800
acgtgtgcgg ttagcgaggt ttagagtgat cgcgtcgcga ttcgtaggag ttagggcgta 4860
ggttgtagtg tagttcggat ttagacgtat ttggtttgga tcgcgcgttt ttagagattt 4920
gcgcgaggtt gtgaggtttt tttttttttt ttttaagcgg tttttttggt gatcgttttt 4980
tcgttatttt tttagtttgg gtaattgggg gcgtttcgga cgattatgag agataaggat 5040
tgagggttag gaaggggaag cgagttcgtc gagaggtggc ggggattgtt tacgttaagg 5100
gttatagcgg tcgcgtttcg gtttcgtttc gtcgttttac gtttcgcggg attcgcgggg 5160
gtagttcggt cgggcgggga tgtcggggtt ggggcggagg gcgtagtggt tgtgttggtg 5220
gtgggggttg ttgtgtagtt gttgcgggtt ttcgtcgttg cggtcgtttt tgttcgttgt 5280
cgcggtcgtc gtcgtcgggg ggggttttta atagtcgtag ttttcggcgt ttcggtagta 5340
ggaggagtag tagtagtagt agtagttgtt tcgcggagag ttttttttcg ggcgattgaa 5400
gttcgcgttt ttttttatgt tggatttgta taacgttttg ttcgtcgata acgacgagga 5460
cggggcgtcg gagggggaga ggtagtagtt ttggttttac gaagtagtta gttcgtttta 5520
gcgtcggtag tcgttttcgg gcgtcgcgta ttcgtttaat cgtaagagtt ttttggtttt 5580
cggatttggt agcggcggcg cgtttttatt gattagcgcg taggatagcg ttttttttaa 5640
cgacgcggat atggttatga gttttgtgaa tttgggtaag gatttggggt aacgtaatga 5700
cgagaatatt tttttttttt tagtgtttcg ggtttagggg atggagtgag gggtggaggg 5760
agttttcggc gcgcgggttt cgtttggtgc gttagagaac gcggggatag gtaggttgtg 5820
ttttcgttat ttgcgcggcg gtgggcggta tatgcgtttt ttcgcgtcgc gggtagggtt 5880
gcgcgtcgag gtttttagag gcggttggtc ggtcgggtcg cggggtttcg ggagcgttgt 5940
ttttagcggg cgtgtatttg cggatttttt gagtacgttt cgcgtttttt tttatttttg 6000
tttagttttg gttaggttaa tttaattgta gtaggagagt ttagttcgag tgtggttatt 6060
ttgggaagag ggttttttag tacgattttt ttttttaaat tgtcgttttt tgttttttaa 6120
tttggagtgt agttttttta tcgagatttt tagggaattc gatagcggtt gggaattttg 6180
aaaatttggt atgagtatgt ttagaggtat ttacggtatt aggggaggtt gtagagggaa 6240
gttttaaaag gatcgcggaa ggcgttcgga tttcggtcgg gcggggttcg tttttgtttg 6300
gggcgggatg aaagtttaat tggggtaatt tattttgtgt ttcggtttat atgtttttgg 6360
ttattgtttt taaaggttga atttttgatt aatgtagttt aagttgtacg ggtagagaaa 6420
gggtgcgggg taggggtcgg ggttgggcgt tgagtttttt tgaatcggtt gttaggtaat 6480
agaatggaaa tttacgggcg gttaaatgga gtttcgttat aaatgttttg tggtaaattc 6540
ggatttagtt atatcgtttt tttttttttt ttttatttta ttgagttttt ttatttgtaa 6600
atgagaaggg tgtagagaag attttttttt tcggagttag tgttttcggt ttgtattgtt 6660

ttttatttat aagtt                                                    6675

<210> 276
<211> 6675
<212> DNA
<213> Artificial Sequence

<220>
<223> chemically treated genomic DNA (Homo sapiens)

<400> 276

agtttatgaa tgaaaagtag tgtaaatcgg aggtattgat ttcgaagaaa agagtttttt      60
ttgtattttt tttatttata gatgagaaaa tttagtgggg tgggaggggg gagaaagggc     120
gatgtgattg ggttcgggtt tgttatagaa tatttgtggc ggagttttat ttagtcgttc     180
gtgaattttt attttgttgt ttggtaatcg gtttagagga gtttagcgtt taatttcggt     240
ttttatttcg tatttttttt ttgttcgtgt agtttgggtt gtattgatta aaggtttagt     300
ttttggggat aatggttaag ggtatgtgag tcggggtata ggatggatta ttttagttga     360
gtttttattt cgttttaaat agaagcgagt ttcgttcggt cgggattcga gcgtttttcg     420
cggttttttt gggatttttt tttgtaattt tttttagtgt cgtggatatt tttaaatata     480
tttatgttaa gtttttaaag tttttagtcg ttgtcggatt ttttaaaagt ttcggtggga     540
aaattgtatt ttaagttagg gggtaaaagg cggtagttta gaggggaaaa tcgtattgaa     600
gagttttttt tttaggatgg ttatattcgg gttggatttt tttgttgtag ttgagttaat     660
ttggttagag ttgggtagaa gtgaaaaaaa gcgcggaacg tgtttaggga gttcgtaaat     720
atacgttcgt tggggatagc gttttcggag tttcgcggtt cggtcggtta gtcgtttttg     780
ggggtttcgg cgcgtagttt tgttcgcggc gcgggggagc gtatgtgtcg tttatcgtcg     840
cgtaggtggc gggagtatag tttgtttgtt ttcgcgtttt ttggcgtatt aggcgggatt     900
cgcgcgtcgg gagttttttt atttttttat tttatttttt gggttcggga tattgaaaag     960
ggggaaatgt tttcgttatt acgttatttt aaatttttat ttaggtttat aaagtttatg    1020
attatgttcg cgtcgttgag gaaggcgttg ttttgcgcgt tggttagtgg ggacgcgtcg    1080
tcgttgttag attcgggggt tagaaggttt ttgcggttga gcgggtgcgc ggcgttcggg    1140
ggcggttgtc gacgttggga cgagttggtt gtttcgtggg gttaggattg ttgttttttt    1200
tttttcgacg tttcgttttc gtcgttgtcg gcggataggg cgttgtatag atttagtatg    1260
aagaggggcg cggattttag tcgttcggga ggggggtttt cgcgaggtag ttgttgttgt    1320
tgttgttgtt ttttttgttg tcggagcgtc gggggttgcg gttgttggag gtttttttcg    1380
gcggcggcgg tcgcggtagc gggtaagggc ggtcgtagcg gcgggggttc gtagtagttg    1440
tatagtagtt tttattatta gtatagttat tgcgtttttc gttttagttt cggtattttc    1500
gttcggtcgg gttgttttcg cggatttcgc gaggcgtgga gcggcggagc gaggtcggag    1560
cgcggtcgtt gtggtttttg gcgtgagtag ttttcgttat ttttcggcgg gttcgttttt    1620
tttttttggt ttttagtttt tattttttat ggtcgttcgg ggcgttttta gttgtttagg    1680
ttagagaggt ggcgaagggg cgattattag gagaatcgtt tggaggggag gaaggggagt    1740
tttatagttt cgcgtaggtt tttagaggcg cgcggtttag gttaggtgcg tttgagttcg    1800
ggttgtattg tagtttgcgt tttggttttt gcgagtcgcg gcgcggttat tttgggtttc    1860
gttggtcgta tacgtgcgtt gtttcgcgtt ttttttggcg attgagtggg attcggggta     1920
gcggggagcg gggtcgttta cgagttgcgg gagatgttcg gtattatcgt ggcgtacgtt    1980
tttttttaga tcgtagggtt attatgaagt ttatttcgtt gtagggttta ggttgcggcg    2040
ataaggcgtt tcgaaatgtt agtttatttc gcgaggttta attttttttt cgtttacgaa    2100
tttcgcgttt ttgagttttc gggtatcgtc gtttttattt aaaattttcg cgcgtcgtac     2160
gtttttttaa gcggaggcgg cgaggatttt gtttttcgtt cggcggtcgt tcggttgttt    2220
agtaaattcg ttcgtagaaa ggatgggaag gaagggggtt agtgtatcga gtagcggtcg     2280
cggcggcggc gtcggaattc ggtttttcgt ttttttacgt cgcgtttttc gtagcggtta     2340
acgtgggttt tttgttgcgc gttcggttta ggtattttat tttagtttcg ggatttttcg    2400
tttcgcggta atcgataggt tggtagttaa tggggacgcg aagttcgcgg gttatttaaa    2460
tagtttcgtt ttttttttta tttttttttt ttcgagaggc gtcgttttcg tcgcgcgtag    2520
gttttaagat tcgggaattt ttggaggtat gcgaagagtc gcgaggtgtt aaaggcgaag    2580
aggttatcgt tagtaagaat gatttgatta atatttcgat ttgaagtttt tttttttatt    2640
attatttaaa aggtttaagt tgatgtttta attaaataaa aataataata gcgattaaat    2700
tattatattg ttagaattga ggaagaaaag aaaaatgata gtttttttat ttttttttta    2760
gtttttttta ttgatgtaat gtaggcgttt agcggatacg tggtttttta cggtagttgt    2820
tggaagttgg gatatatttt tcggggaggt tatttataaa ttgattatag agagggttat    2880
atgtaattta agttagcgtt ttgaggtttt ggggtcggat gtgttgattt ttttgtttat    2940
ttgtgttttg gtttggtttt ttaagttagt atttaaaaga aagagggtgt aattagacga     3000
tatattagaa aaggcgtttt ttgttttttt ggaaatgttt tgatttttta gtttatttat     3060
taatgttgta ttttaatatt gaaatataat gtgatttata tggtgtaaga taaaatttga    3120
ggagaggtga taaagataat tttaggaaga gtaggggttt ggtatgtatt ttgaatggtt    3180
aggataatag atagggtttt gaaatatagg tttttgtgga ttttagataa gataatatta    3240

ttattaatat tatttgttaa tgaggatata aatttatttg tttttatttc gttcgagaat 3300
atttagttat ttatggatcg tagaagtaaa tttacgtaaa atatgaaggt tttttatttg 3360
gtgaaaattg tgtggagttg aaaataaaaa ttgtaatttt ttgatttgtt agttttagtt 3420
taaatggttt taggtatttg agagtgaagt taaatagatt atagattgtg ggatttatga 3480
atttatgaga ttataggttt tttgttggtt taaattttat aagtattttt attttatttt 3540
taagaattta ttttttatgg gtaaatagag attattattg attttaatta tttattgagt 3600
tttttttttt agttttgttt tggtttagat ataagtggtt atttagaaga gtttttattt 3660
ttataaatat ttgatagtta tgaattttag ggtataaaat ttatgtgaat gttagttttt 3720
aattattttt atagggattt tttttattcg aaaagataat acgatatttt gtttgggatg 3780
ttcggatttt tagaatttaa gagttttgtt attttttttt tttgtaggga atatgttagg 3840
gatataaatt tattgtagtg ttttatagta gttaagagtt ttgtttagaa gatttgaaat 3900
tttagtaaat atatttaggt tttttgttaa gagttttttt ttttaaattt agttgtattt 3960
tttttataag atttttggtg agatgtaggt agagtaaaag attttttatt tacgagggta 4020
gtggggaaga tataggaaat ttaatatttt aagtatggat gagggtattt tggtggggtt 4080
atgttattta aaatataaaa tcgaggggtg atttatttat tgtagaggtt gagtattttt 4140
aatttaaaaa tttgatattt aaaatgtttt aaaatttaaa gatttttgag agttgatatg 4200
atgttttaga tggaaaattt tatatttgat tttataggat gggatatggt taaaatatag 4260
ttaaaatttt tatttataaa attatttgaa atattgtatt aaattatttt tagattatgt 4320
atatgagata tatacgaaat ataaatgaat tttatgttta gatttggatt ttatttttaa 4380
ggtattttat tatgtatatg taaatatttt aaaatttaaa aatttgaaat ttgaaatatt 4440
tttggtttta agtattttgg ataagggata tttaatttgt gtatgtattt atggggtgga 4500
tttagtgtag aagatatata tatatatgta tatatacgta cgttattttt tttttttttt 4560
agatattaag aagtagatgg gtttatgtga tatgagttta aagtgattat taattaaata 4620
tttgagtagt aattttgatt taggtttgtt gtaggttttt tattttatta agatggattt 4680
ttagtttttg tcgaatgggg ttagaatgag gaggtgtcga gagtattttt aggtgatgaa 4740
tatgagaacg tatatttttt ttaagagtta gaagaagttt ttaaaaaaat tgtgtagtga 4800
gatattataa tgattataag gatagtgata tttaattgag gatgtgtttt ttttttgttt 4860
attatggttt ttaatatgtg tatattatgt ttgcgggata tatgtaggta agtttttgta 4920
tttattggga taaaggggat aggaagagat atagttttag gattttaata attttgaaat 4980
tgggattatt ttttttttaa gttagttgga tttttaggtg gataaaaatt ttttaatttt 5040
tattttgttt attttttgtt tttgggattt ttttttatta gatagttcga gtgttttttt 5100
ggaagtttta ggatagggag ttagataacg tttatatgtg atttaagagg aggaggtatg 5160
gggtgggtgg agttggaggg ataggagtag atttgggttt tgaagttcgt tggaatgtat 5220
gaatcgattt gataaaatta gaggtaattt cgttttttgt aggatattaa aatattatcg 5280
tttttttttt tgcgggttcg gaataagttt tatgaagttt tttgtgtttt taaaatattt 5340
gggtggaaag aggaggaatt cgattaagaa gtcggatgtg ggagttaatt taattaaata 5400
tttggttgtt tggttgatat tttatgtaga tggtttttag ttttttggta atacgttgtt 5460
tgttttattt ttttgtttta tttaatgaag aaatgaggag tttttattag aaagtatagg 5520
tttattttat atttttagtg atagtgggaa taggacggga attttgtttt taagtgttta 5580
ttaaggtatt atgtaattta cgtaataaat tttttaaatt tgtagttgtt tttgtttttt 5640
atatatttttg agggttttttg ttatatattt ataatatatt tgattaaata gttttatttt 5700
tgtaaatatg ttgtttatta atttgatata ttaaatatat attttatatt attttattag 5760
taataaattg taattttagt tttggatttt atattaaatt ttatattggt tttttttaat 5820
tgtatttttt gaagatgtaa gttgttgttt ttagaattaa tttaataaaa atattttggt 5880
taggtatgta atagtaatat tttgagaggt tgaggtaggt agattatttg agtttaggag 5940
ttcgagatta ggttgggtaa tttagtaaga ttttgttttt ataaaaaaaa aaaaaaaaaa 6000
aaaatataaa aattagtcgg gtgtggtggt gtacgtttgt agttttagtt ttttaggagg 6060
ttgaggtggg aggatcgttt gagttaggga ggtggaggtt atagtgagtt tagattatta 6120
gattatatta ttgtatttta gtttgggtga tagtaagatt ttgttttaaa aaaaaaaaaa 6180
aaaaattttg taaattgtgt agttgttatg tttaaaagat tgaggtttag tttttttgaa 6240
ttattgagac gttttatttt aaagattaat tttttatttt tattattagt ataaggattt 6300
gagtattttt taatgttaat tttaaagtat aataattcga ttaaattttt aaaatatcga 6360
tttagagttg agtgaaaaga aaatagataa atggttgtaa agtatgagaa atgaaaattt 6420
gtaagtgaaa taagtattgt ttttgaaagt tatatttgta agatagtttt taaaagattt 6480
gttttattta aaagtttgtg gtaatattta tatttagtta tatttcgttt ttattgttta 6540
tttaaaaatg ttgggtattt atattttaaa atattattta agttattata tttttttatt 6600
tatgaaggat atttggtggt aaagtgaaaa attaatatat aaatttgttt ttagagattt 6660
tttttttatat tataa 6675

<210> 277
<211> 5773
<212> DNA
<213> Artificial Sequence

<220>
<223> chemically treated genomic DNA (Homo sapiens)

<400> 277

atatagatat atatatatat atatatatat atatatatat atatatatat attttttttt 60
ttttttggag ataaagtttt attttgttat ttagtttgga gtgtaatggt acgatttcgg 120
tttattgtaa tttttatttt tcgggtttaa gtaatttttt gttttagttt ttttagtagt 180
tgagattata ggtatatgtt attatgtttg gttaattttt ttgtattttt agtagagttg 240
ggtttttatt attttgttag gttggttttg aatttttgat ttcgtgattt atttatttaa 300
gttttttaaa gtgttgagat tataggcgtg agttatcgtg tttagtttaa tatatatttt 360
taatagagac gagattttat tatgttgttt aggttggttt cgaatttttg agtttaagcg 420
attttttcga tttagttttt tatagtgttg ggattatagg tatgagttat tatgtttggt 480
tttaattatt ttttgattta tttttgggag gagattattt tttcgtttaa gaagaaaaag 540
aaaaaaataa ttttaagttt ttttaatggg ttttatagtt atgtaaagat atgttattat 600
ttttttggga gtgagggtgg aaagaattag atttgtttgc gtaataatga gaaaggaagt 660
tgttttttaa gatagggttt atattagtta gaggggataa ttaggatttg agttttagat 720
agggtttcgt tttatagttt gttgtttagg ttatagtgta gtggagcgat tacggtttat 780
tgttgtttcg attttttggg tttaagtaat tttttggttt tagtttttta agtagttggg 840
attataggtg tatttagttt attttttttt tttttaaggt gttttaagag aaggagaagg 900
tttgagattt aggtgaggga tgggtagaga ttagaggttg tttggttttt tggtttggtt 960
atagtttaga tgtttaggag agaagtgttt ataggtgagg taaatatgag ttaggtgggt 1020
acggagttat ttgggagtat ttattgtatg ttagatattt gaattttaat ggtgaggttt 1080
gaggatatag tttttttatt gaaaaataga gatagggtaa ttatgttgtt agggttggtt 1140
ttatattttt gaatttaagt tgttttttta ttttagtttt ttagtaatta gaattatagg 1200
tattcgttat tgtgttcggt tattttatag ttatttatat aattaattgt ttagttataa 1260
ttttggtaag cgtgttgaag aaataatagg ttttaaataa ttatagttag tattataaag 1320
taagcgttgt atgttaggtt tatatatatt gttaatatgt ttattttttg taataatttt 1380
ttgtgggtga gattattatt tgtttgtttt attgaaagga tagtgaagat tgaggttttg 1440
tgtgatggtg ttgagagttg ggtttatgac gtttagtttt gtagtttgtg ttttgaatta 1500
ttttattttg tttttttttt tatttttata gtagagacgt tatttaagag gagtttgtaa 1560
gtttagaggg ttttttgaag gaagtgagtt gagaataata tgaaattaat tagattaagg 1620
ggtattttag gaagagagaa tggtacgtga aattgtttag gagtagtagg gtgggagtta 1680
ggaggcgtgg gttatggtaa tttatgtata aagagttatg ggaatttata ggaaaattta 1740
agggagtggt tgatatagtt agatttgtgt tgtaagtatt atttcgtagg ttgcgtaggg 1800
aataaatgag ggagtagaag gaaggggttt ttttgttcgt tgtacggtat tgcgtagaag 1860
ataggggagt taggtatttt ttgaagggtg aaaagtaagg agtagagttg ggtagtagat 1920
tagaatttag gagtttggtt tggggtttgg gtggggcgaa agaggcggag tttgaatggg 1980
gtgtgtatag gggggttgcg tgtaggggtg tgtgtatagg ttggggcggg gtttcgggag 2040
tgggttgatt gggtcggggg cggggttttt taggtgggcg gggattttgg ttatttttgg 2100
ttatattttt tttcggttcg agttggttta gcggggcggg ttcgaggggg tgtggtagga 2160
ggtgggcggg ttcgggtggg gggggggggg gcgtggaagg aggggcgggt tcgatagagg 2220
ggcgggttcg aggggcgggg cgggatagga ggtgggtcgt tcgcggatac gtcgtcgtcg 2280
cgggtatatt tttcgttttc ggtcgaggag cgttcgggtt gtttgcggat tttgtcgcgt 2340
gtaggggtcg cggtcggttg gagttgggag tgaggcggcg gaggagttag gtgaggagga 2400
gttaggtgag taggattttg tgttgggcgc ggagttacgt aggttcgagg tgagtcggaa 2460
tttttgtggg ttcgggttgc gtttttagtc gttagggggc gagaggcggc ggggttacgg 2520
ggattgtttt tttcggcgta ggggatttgg gcgttcgtcg ggcggtaggg ggtggagggg 2580
gcggtaaatt agtaattcgt agtgtatata gggttttttg tttcgtttcg tttaaagagt 2640
atttcggtcg cggagttggt tatttattgt ttatcgaggc gggggtaggt tggttttgtg 2700
tagttatttt cgggatttat tgattcgtat ttttttttag gttggttcgg taagggtggg 2760
ggaggataag cgcgtttgtt tttgcggttg ttttcgcgtc ggcggtagag atgagggatt 2820
tgaggtttcg aaaagtttag ttatttagtg ttcgggggtt tttagcgcga gtgcgggagg 2880
ttgaaggaga atttaggatt gtttgatgtt taaggtaggt tttttatttt tacgtggggg 2940
gtggtcggtt agcggttagt agttacgggt gattcgatta aggattttga tattagggta 3000
gtttggggta ggaataaatt tttcgggttt ttttatttat ttttagttta agttgtgtat 3060
ttaaagagtt gttttttttg ttaagtcgag tttggtaggg agttttatta aggaggattc 3120
gattggattc gagagttgag gtgggttaga gatagtagta tttgagttag gtagagaaga 3180
gtaatgaggg gtatagaggg atgggtaaga gagtatatgt gtttagtttt gaaagttaat 3240
ggttttagcg tttttgaagg ggtagacggt gtgattaaag agataggtag cggtagagag 3300
ggagttttag gatgttgagt tggattttgt tgggtatagg tagttattaa gggtttgtaa 3360
gttggggggt atgatatggt agatttgtag gtttttttgt ttgttttttt attttatttt 3420
atttttttat tttgtttttt ttgagacgga gttttatttt gtcgtttagg ttggagtgta 3480
gtggcgcgat ttcggtttat tgtaagtttc gtttttcggg ttcgcgttat ttttttgttt 3540
tagtttttcg agtagttggg attataggcg ttcgttatcg cgttcggtta attttttgta 3600
tttttagtag agacggggtt ttatcgtgtt agttaggatg ttttcgattt tttgatttcg 3660
tgattcgttt atttcggttt tttaaagtgt tgggattata ggtgtgaatt atcgcgttta 3720
gtcgatttgt agttttttaa aattttgttg gaagatgaag gttgaagagt cgagggagag 3780

gatgtttta gaggtttatg taagagatgg ttatgatttg ttttgagaag ggtaggggga 3840
agttagatgg attggaagtg gagtggtagt gattaaggag gaggaggtgt gataggtttt 3900
ttacgtaggg tagatttaga gatattagtg ttatttatag gtttttagga ttgtagtggt 3960
tattcgattt ttttgtttta gttgagattt agttttgagt gttttatttt ggggaataga 4020
ggcgtttttg gtagtatttg gaagaggata gttagttggg gtgtgtgtat attatagttt 4080
gatagtaata gtattcgaat tagaggtgat tggttaaggg tagatttagg gtaataggtt 4140
aatcgtttta gggtcgggta tagggaggag aatattttaa tattttgtgt gtttagtgtc 4200
gacgtacgtt ttttttttta tttttaaaat agttttatga ggatataagt tagagagaga 4260
tagagataag gaattataag ttggtgagag ttaggatttg aatttggttt tggtagatgg 4320
aaaattaggg tttgtatttt ttataaaatc gtgtgtgttt tagatggagt tggtgtataa 4380
taagtagagg tatttagggt cgcggttttg tttgttacgg aaggggtcgt tttgttagtt 4440
gtgattattt agttttggaa atgttagtaa tgttgtaagg agtggggatc ggattagatg 4500
ttatttagat gttgaagttt gattttgtgt tattttttat tttttttttt ggtttttttg 4560
taattaattt atttatttag taaaaaagaa attatgtgtg tcgagagtat gtagaagata 4620
tgtttcgtt tttgtttttt ttttaaaaaa gaatttaaa attgtttttt gtgaacgtgt 4680
gttagggttt tagtaggatt tagggagagt aggaagttta gtttagattt tttgtataat 4740
ttatcgtggg gaggttttag gttttggtta ttatagagtt ggttttagtt tgtattgtta 4800
tagtttggtt agggatttgg atatatttgt tggttatttt ttgttttagt tttttttattt 4860
gtaaaataag ggaaaagttt ttataaaggt gtacgtgtag taggagtttt tttttttttt 4920
tattttagga aggtagttgg tgggaagttt agtttgggtt tttgagagtt gtgagaagga 4980
gatgcggttg ttgttggttt tgttgggggt tttgttgagt gtgtttgggt ttttagtttt 5040
gtttttggag gtttttgagg aagtggagtt tggtatggtt tttgaggtgg gagagggtgg 5100
taggggtggg aagagtgggt attaggaggg ggttgttggg ttgagtaaag ttggaaagga 5160
tttttgttta ggttttgaga aggtggcggt agggtagggt ttaattattg agatttagtt 5220
agtgtttggt ttttagtaag tatttattta ttattgtgtt tgcgagagag gattggtttt 5280
gtagggcgta gggttttaag ttgggttgta gagttggtgg tgagtttttt gtttgggtgt 5340
gtgtgcgtgt gtgtgtgtgt tttgtgtatt gggtgtgtga tttaggaggt ttaggtagta 5400
tgtgtggtat aagtattatg agggtgatat gtttcggtgt agtatgattt tgtatgtggt 5460
attaatagta tgtgttttgt gtgtgtgtgt gttcgtatgt gtgtgtgtgt atgcgtgtgt 5520
gtgtgtgtgt gtgtgtgttt tggttattgt tatgtgtatt aaatgttgtg tgtgtgatat 5580
gttttaagag tgtggtattt gttttgggtg tggtattcgt agtatgtggt tgtgtgggtg 5640
ttaaggagtg gtggtttttt tagtatgcgt tgcgaagtgt ttgtgttttg tatgtgcggt 5700
gtgttttttg tatataggag gttgttttag atggggttgc ggggtttgtt gatttttgtt 5760
ttttgtttat aga 5773

<210> 278
<211> 5773
<212> DNA
<213> Artificial Sequence

<220>
<223> chemically treated genomic DNA (Homo sapiens)

<400> 278

tttgtgggta gagggtagag gttagtagat ttcgtagttt tatttgaggt agttttttgt 60
gtatagagaa tatatcgtat atgtagggta taagtatttc gtaacgtatg ttgaaggagt 120
tattattttt tgatatttat atagttatat gttgcggatg ttatatttag ggtaaatgtt 180
atatttttgg ggtatgttat atatatagta tttagtgtat atgatagtgg ttaagatata 240
tatatatata tatatatacg tatatatata tatatatacg gatatatata tatataaggt 300
atatgttgtt ggtgttatat atagggttat gttgtatcgg ggtatattat ttttataatg 360
tttatattat atatgttgtt tggatttttt aggttatata tttagtgtat agaatatata 420
tatatacgta tatatattta ggtaaggagt ttattattag ttttgtagtt tagtttaggg 480
ttttgcgttt tgtaaggtta gttttttttc gtagatatag tgatagatga atgtttgttg 540
gaagttaggt attgattgag ttttagtggt tgagttttgt tttgtcgtta tttttttagg 600
gtttgggtaa ggattttttt tagttttgtt tagtttagta gttttttttt ggtgtttatt 660
tttttttattt ttgttatttt tttttatttt agaagttata ttaagtttta ttttttttaga 720
ggttttaggg gataagattg gaggtttagg tatatttagt aggattttta ataggttag 780
tagtagtcgt attttttttt tatagttttt agggatttaa gttggatttt ttattaattg 840
tttttttaaa atagggaggg aaaagagttt ttgttatacg tgtatttttg tggggttttt 900
ttttttattt tgtagataag gaaattgaga taggaagtgg ttagtagatg tgtttaagtt 960
tttggttagg ttgtggtagt gtagattgga attagttttg tagtagttag agtttaaggt 1020
ttttttacgg taggttgtgt aaggggtttg ggttgggttt tttgtttttt ttgagttttg 1080
ttgggatttt ggtatacgtt tatagaaagt agttttggga tttttttttg gagggaagta 1140
gagaacggag atatattttt tgtatgtttt cggtatatat aatttttttt ttgttaaata 1200
aatgaattga ttgtagaaga gttaaaaaag aaagtgaaaa atgatataag gttaaatttt 1260

agtatttgga tggtatttga ttcgattttt attttttata gtattattga tatttttagg 1320
gttgggtggt tataattgat aaggcggttt ttttcgtggt aagtaggatc gcgattttgg 1380
atatttttgt ttgttatgta ttaattttat ttgaggtata tacggttttg taaagaatat 1440
agattttaat tttttatttg ttagagttaa gtttaaattt tgatttttat taatttgtaa 1500
ttttttgttt ttgttttttt ttggtttata tttttatagg attgttttga ggataaaaga 1560
gagaacgtgc gtcggtattg ggtatataga gtgttggaat gttttttttt ttgtgttcgg 1620
ttttagaacg gttaatttgt tgttttgggt ttgtttttag ttagttattt ttggttcgga 1680
tgttgttatt gttaggttgt gatgtatata tattttagtt ggttattttt ttttaaatgt 1740
tattaaggac gtttttgttt tttaaaatag aatatttaga attgagtttt agttgggata 1800
aaggaatcgg gtgattattg tagttttagg ggtttatggg tggtattggt gtttttggat 1860
ttattttgcg tgggaagttt attatatttt tttttttttg gttattgtta ttttattttt 1920
agtttatttg gttttttttg ttttttttta aggtaggtta tggttatttt ttgtatgggt 1980
ttttggaaat attttttttt tcggtttttt aatttttatt ttttagtaga gttttaaaaa 2040
attataagtc ggttgggcgc gatggtttat atttgtaatt ttagtatttt gggaggtcga 2100
ggtgggcgga ttacgaggtt aggagatcga gaatattttg gttaatacgg tgaaatttcg 2160
tttttattaa aaatataaaa aattagtcgg gcgcggtggc gggcgtttgt agttttagtt 2220
attcgggagg ttgaggtagg agaatggcgc gaattcggga ggcggagttt gtagtgagtc 2280
gagatcgcgt tattgtattt tagtttgggc gatagagtga gatttcgttt taaaaaaaat 2340
aaaataaaaa aataaaataa aataaaaaaa taaataaaaa aatttgtaag tttgttatgt 2400
tatgtttttt agtttgtaag tttttaatgg ttatttgtgt ttagtaggat ttagtttaat 2460
attttagggt tttttttttg tcgttgttta tttttttggt tatatcgttt gtttttttag 2520
gagcgttgaa gttattggtt tttaaaattg ggtatatgtg tttttttgtt tatttttttg 2580
tgttttttat tgtttttttt tatttgattt agatattgtt gtttttggtt tattttaatt 2640
ttcgaattta gtcggatttt ttttggtaaa attttttatt aagttcggtt tggtagggag 2700
ggtagttttt tgggtatata gtttgggttg ggagtgggtg gggaggttcg gggagtttat 2760
ttttatttta ggttgttttg atattagagt ttttagtcga gttattcgtg gttgttgatc 2820
gttgatcgat tattttttac gtgggaatgg agggtttgtt ttaggtatta gatagttttg 2880
ggtttttttt tagtttttcg tattcgcgtt ggaggttttc gggtattaag tgattgaatt 2940
tttcggggtt ttaggttttt tatttttgtc gtcggcgcga agatagtcgt agggataagc 3000
gcgtttgttt ttttttattt ttgtcgggtt agtttggggg gaggtgcgaa ttaatgggtt 3060
tcgagggtag ttgtataggg ttagtttgtt ttcgtttcgg tgggtaatga gtaattagtt 3120
tcgcggtcgg ggtgtttttt ggacggagcg ggataaaagg ttttgtgtgt attgcgggtt 3180
attgatttat cgtttttttt attttttgtc gttcggcgga cgtttaggtt ttttgcgtcg 3240
ggaggggtag ttttcgtagt ttcgtcgttt ttcgtttttt ggcggttggg agcgtagttc 3300
gggtttataa gggtttcggt ttatttcgag tttgcgtgat ttcgcgttta gtatagggtt 3360
ttgtttattt ggtttttttt tatttggttt tttcgtcgtt ttatttttag ttttagtcgg 3420
tcgcgatttt tgtacgcggt agggttcgta gatagttcga gcgtttttcg gtcgggagcg 3480
aggaatgtat tcgcgacggc ggcgtgttcg cgagcggttt attttttgtt tcgtttcgtt 3540
tttcgggttc gtttttttgt cgggttcgtt ttttttttta cgtttttttt ttttttattc 3600
gggttcgttt attttttgtt atattttttc gggttcgttt cgttagatta gttcgagtcg 3660
gagagaggtg tggttagggg tggttaagat tttcgtttat ttggagagtt tcgttttcga 3720
tttagttagt ttattttcgg gatttcgttt tagtttatat atatattttt atacgtaatt 3780
tttttatata tattttattt aggtttcgtt tttttcgttt tatttaggtt ttaggttagg 3840
tttttaaatt ttagtttatt atttagtttt attttttgtt ttttattttt tagggaatgt 3900
ttggtttttt tgttttttgc gtagtgtcgt gtagcggata ggagaatttt ttttttttgt 3960
ttttttattt gttttttgcg taatttgcgg agtgatattt ataatataaa tttgattgtg 4020
ttagttattt ttttaaattt ttttatagat ttttatagtt ttttatatat agattgttat 4080
gatttacgtt ttttggtttt tattttgtta tttttgggta gttttacgta ttattttttt 4140
tttttggaat gttttttggt ttggttaatt ttatattatt tttagtttat tttttttagg 4200
aagttttttg gatttgtaag ttttttttag atgacgtttt tattgtgagg atggagaaag 4260
aaatagggtg aagtggttta gagtatagat tatagggttg gacgttatag gtttagtttt 4320
tagtattatt atataaggtt ttagttttta ttgttttttt agtaaaatag gtaaatgata 4380
gttttattta taagaggttg ttgtaaaaag tagatatgtt agtagtatgt gtaagtttgg 4440
tatgtagcgt ttgttttgta atattagttg taattattta ggatttgtta tttttttagt 4500
acgtttgtta gaattgtaat tgaatagtta attatgtaaa tagttgtgga atggtcgggt 4560
ataatgacgg gtgtttgtag ttttagttat taggagattg aggtaggagg atagtttgaa 4620
tttaggagtg tgaggttagt tttggtaata tagttgtttt gtttttattt tttagtagag 4680
gaattgtgtt tttagatttt attattagag tttaagtgtt tggtatatag taggtgtttt 4740
tagatagttt cgtgtttatt tggtttatgt ttgttttatt tgtaagtatt ttttttttga 4800
gtatttagat tgtggttaaa ttaggaggtt aggtagtttt tgatttttgt ttatttttta 4860
tttgaatttt aagttttttt ttttttttaa ggtattttga gaggaaggga aatgggttgg 4920
gtgtatttgt ggttttagtt atttgggagg ttgaggttag aggattgttt gagtttagga 4980
agtcgaggta gtagtgagtc gtgatcgttt tattgtattg tagtttgggt aataggttat 5040
ggagcgagat tttgtttgag gtttaggttt tggttgtttt ttttagttag tgtagatttt 5100
gttttaagaa gtaatttttt tttttattgt tacgtaggta agtttagttt tttttatttt 5160
tatttttaaa gggatagtgg tatatttttg tataattgta aggtttatta aagagattta 5220

aaattgtttt tttttttttt tttttaagcg aaagaatggt ttttttttaa agataaatta 5280
gaaaataatt gaggttaggt atggtggttt atgtttataa ttttagtatt gtgggaggtt 5340
gaatcgggag gatcgtttga atttaggagt tcgaggttag tttgggtaat atagtgagat 5400
ttcgttttta ttaaaaatat atattaggtt gggtacggtg gtttacgttt gtaattttag 5460
tattttggga ggtttaggtg ggtggattac gaggttagga gtttaagatt aatttggtaa 5520
gatggtgaaa ttttagtttt attaaaaata taaaaaaatt agttaggtat ggtggtatgt 5580
gtttgtaatt ttagttatta gggaggttga ggtagagaat tgtttgaatt cgggaggtgg 5640
aggttgtagt gagtcgagat cgtgttattg tattttagat tgggtgatag agtgagattt 5700
tgtttttaaa aaaaaaaaaa aatatatata tatatatata tgtgtgtgtg tgtgtgtgtg 5760
tgtgtgtttg tgt 5773

<210> 279
<211> 3757
<212> DNA
<213> Artificial Sequence

<220>
<223> chemically treated genomic DNA (Homo sapiens)

<400> 279

ggattatttg aggagtttaa gattagtttg gtaaatatgg tgaaattttg tttttattaa 60
aaaaaaaaaa aaaaaattag ttgggtatgg tggtgtatgt ttgtaattgt agttattcgg 120
gaggttgagg taggagaatc gtttgaattc gggaggtcga ggttgtagtg agttgagatt 180
gcgttattgt attttagttt gggtgataag agcgaagttt cgttttaaaa aaaaaaaaaa 240
aattaaaaaa aattaataaa agatttaaaa aaaaaagatt atattgttag tattttagaa 300
ttattttagt gtaatgatga tagtttattg tggtattaag aagatagggg ttatagtttt 360
atttttggaa aagaaattaa taaagtggta tgtttagtta aaatatatat aattatatgt 420
aagtattaaa atgttattgt ataaatataa tataaattgt aaaagaaaat atgtttttta 480
aattagagta agtataatag tttgtgaaat aaatgttatt ttttagagtt gtttagtgta 540
taagttgtat aattttatat gggtttttag tagaggtttt tattttatgt gaggttttgt 600
aaattgggag atgatttttt tagtagtgtg gtaatagtat aatttgtttt ttttttttga 660
ttttgtgtgt attggtttta tttataaagt aatataaaaa ttaaaaggtt tggttagacg 720
cggtggttta cgtttgtaat tttagtattt tgggaggtcg aggcgggtag atgatttgag 780
gttaggagtt tgagattagt ttgagtaata tggtgaaatt ttgtttttat taaaaatata 840
aaaattagtc ggcgtggtgg gtgggtattt gtaattttag ttatttagga ggttgagacg 900
tgggattcgt ttaaatttag gaggtagagg ttgtagtgaa ttgagatcgt gttattgtat 960
tttagtttgg gtgataaagt gagattttgt tttaaaaaaa aaaaaaaaaa aaaaattaaa 1020
attttttgtt ttgaatggat tatgggatta gagttttatt aggattttta attattatat 1080
gtttttttat tttagtattt tttatttttg tttatatagt tattgtaagt gatttattta 1140
tattatagta ggtaattata gagattttta tatatttgta gttattttat ttatttttgt 1200
agttaaatat tttgaagttt taaggagatt tttgttttat ttttaattta gttttatttt 1260
tgttttttga attgtttttg tttgttgtaa ttttagtttt tttttgttag taaagtttat 1320
ttttttatta tttgaggttt ttataaaagt ttatggaatg tttatttggg tttttaggtt 1380
tatgtttttg gttatataaa gaggagtatt tttttttaaa aggatttttt tttagtttaa 1440
ataattttta atattttgtt attatttgta ttttttaggg tttatatttg tgatttttaa 1500
tggagagtga aaacgtagat ttataatgaa aattagtttt cgtcggttat tgatttttaa 1560
aagacggagg ttgttttttt ttgtttaaaa tgttttaagt gaaatattag aggaggaatt 1620
taagagattt tttgtttaat aggagtttaa ttaagtagag gtttttaagg aagtggtaga 1680
gtttaatttt tgtaagtttt tagttgggat taagattatt aattatttta ttatgtttaa 1740
tacgtaagta gtggttattt ttaataatgt taatatttat agtattatta tagtattgat 1800
tgttaaggga aaagagagtg gtagtagtgg gtttaataaa tttattttta ttagttgtgg 1860
gggagtttta atttagtttt taggatttcg gttttaaatt taaattagtt atgatgttaa 1920
aaggatagaa gtgattttgg agattttggg attaaaattt gtagttaggg atgtgaattt 1980
ttttagatta tttggagttt tttgcgagta gaaacgggag atttgtggta tgtggttttt 2040
tagggaaagg ggtgagggag tttagttttt tttttgtagt ggtaaggtgt gtagttagtt 2100
ttgatgttta taatagttag gtttttggtc gagtatagtg gtttattttt gtaattttag 2160
tattttggga ggttgaggcg ggcggattat ttgaggttag tagtttggat tagtttggtt 2220
aatatggtga aattcgtttt tattaaaaat ataaaaaaat tagtcgagcg tggtggtata 2280
cgtttgtagt tttagttatt ttggaggttg aggtaggaga atcgttgaat ttgggaggtg 2340
gaggttgtag tgagttgaga ttatgttatt gtattttagt ttggttatag agggagattt 2400
tgttttaaaa aaaaaaataa aaatgaaaaa agaaggggtt ttaaaaataa ataaatgaag 2460
aaggggtttt attatgttgt ttaggttggt ttcggatttt tgagtttaag ggatttaatt 2520
atatgggttt tttaaggtgt tgggattata ggtatgaacg atagcgtttg attttatttt 2580
tttttttttt tttttttttt tttttgagat ggagttttgt tttgttgttt aagttgtagt 2640
gtagtggtgt aattttagtt tattgtaatt tttatttttt gggtttaagt gatttttttg 2700

ttttagtttt ttgagtagtt gggattatag gtattcgtta ttatatttgg ttaattttgg 2760
tattttttag tagagatagg ggtttgttat gttagttagg ttggttttga atttttgatt 2820
ttaggtgatt cgtttatttc ggttttttaa agtgttggga ttataggtgt gagttattgc 2880
gtttggtttt attttttttt ttgataaagg agaaaattga agtaagagga ggaaaatgat 2940
ttttttttta gagatgcggt ttcgtattgt tatttaggtt agagtgtagt ggcgtaatta 3000
cggtttattt tagtttcgaa tgtttggatt taagttattt ttttatatta gttttttaag 3060
tagttgggat tataggtatg tattattatt tttagttaat tttttttttt tttttgtaga 3120
gatggggttt tattatattg tttaggttga aaagtgattt ttttggtgtg tatattttaa 3180
taattttttt gtaagtttgt tttgattgat ttttaaggtt tagtgtggtt tagtttgaaa 3240
tgtaatttga attatgtttt taaatttgtt ttgaaatgta taatggggta gagtaattta 3300
gagttataat agatttagta tgtgtttttc gtttttaaag gtagaggttt tggataagtg 3360
atttttagtt attaattttt ttttttttat tttttatttt tttgagatag agttttattt 3420
aggttggagt gtagtggcgt aatgttggtt tattgtagtt ttcgtttttc gggttcgagt 3480
aatttttttg ttttagtttt ttgagtagtt gggattatag gcgtgtgtta ttatatttag 3540
ttaattttgt atttttagta gagacgaggt tttattatgt tggttaggtt ggttttgaat 3600
ttttgatttt aggagattta ttcgtttcgt ttttttaaag tgttaggatt ataggtgtga 3660
gttattgtat tcggtttgtt agttattaat tttgatggat atatttgggt atttgatgag 3720
gtagttaagt ggggtgttgg attatttttt ttttagt 3757

<210> 280
<211> 3757
<212> DNA
<213> Artificial Sequence

<220>
<223> chemically treated genomic DNA (Homo sapiens)

<400> 280

gttagaggga aaataattta atattttatt tagttgtttt attagatgtt taggtgtgtt 60
tattagaatt ggtggttagt aggtcgggtg tagtggttta tatttgtaat tttagtattt 120
tgggagggcg aggcgggtgg attttttgag gttaggagtt taagattagt ttggttaata 180
tggtgaaatt tcgtttttat taaaaatata aaattagttg ggtgtggtgg tatacgtttg 240
tagttttagt tatttaggag gttgagatag gagaattgtt cgaattcggg agacgaaggt 300
tgtagtgagt taatattgcg ttattatatt ttagtttgag tgagattttg ttttagaaaa 360
ataaaaaata aaaaagaaag aattggtggt tagggattat ttgtttaaga tttttgtttt 420
tagggacgga gaatatatgt tgaatttatt gtggttttaa gttattttgt tttattatgt 480
attttaggat aaatttagga gtatagttta aattgtattt taagttgagt tatattgagt 540
tttagagatt aattaaggta ggtttataaa agaattattg agatatgtat attaaaaaag 600
ttatttttta gtttgggtaa tatagtggga ttttattttt gtaaaaagaa aaaaaaaatt 660
aattgggaat gatggtgtat gtttatagtt ttagttattt gggaggttga tgtgggagaa 720
tggtttgagt ttaggtattc gaggttagag tgagtcgtga ttgcgttatt gtattttagt 780
ttgggtgata gtgcgagatc gtatttttaa agaaaaagtt attttttttt ttttgtttta 840
gttttttttt ttattaagaa ggaaaatgag gttaggcgta gtggtttata tttgtaattt 900
tagtattttg ggaggtcgag gtgggcgaat tatttgaggt taggagttta agattaattt 960
ggttaatatg gtaaattttt gtttttatta aaaaatatta aaattagtta ggtgtggtgg 1020
cgggtgtttg taattttagt tatttagaag gttgaggtag gagaattatt tgaatttaga 1080
aggtggagat tgtagtgagt tgagattgta ttattgtatt atagtttggg taatagagta 1140
agattttatt ttaaaaaaaa aaaaaaaaaa aagaaggaaa atgaagttag gcgttgtcgt 1200
ttatgtttat aattttagta ttttgggagg tttatatggt tggatttttt gagtttagga 1260
gttcgagatt agtttgggta atatagtgag attttttttt tatttattta tttttgagat 1320
tttttttttt tttattttta tttttttttt tgagatagag tttttttttg tggttaggtt 1380
ggagtgtagt ggtataattt tagtttattg taatttttat tttttaggtt tagcgatttt 1440
tttgttttag tttttagagt agttgggatt atagacgtgt gttattacgt tcggttagtt 1500
tttttatatt tttagtagag acgggtttta ttatgttggt taggttagtt taaattgttg 1560
attttaagtg attcgttcgt tttagttttt taaagtgttg ggattatagg gatgagttat 1620
tgtgttcggt tagaagtttg attgttgtaa gtattaagat tgattatata ttttgttatt 1680
atagaggaaa ggttgggttt ttttattttt ttttttggaa gattatatat tataggtttt 1740
tcgtttttgt tcgtaaaggg ttttaggtgg tttaggaaga tttatatttt tagttgtagg 1800
ttttggtttt aaggttttta gggttatttt tgtttttttg gtattatagt tggtttgggt 1860
ttgaggtcgg agttttggag gttgagttgg ggttttttta tagttgatga ggatgaattt 1920
gttgggttta ttattgttat tttttttttt tttggtagtt agtgttgtga tgatgttgtg 1980
aatattagta ttgttgggga tggttattat ttgcgtgttg ggtatggtgg ggtggttaat 2040
aattttgatt ttagttggaa atttgtaaga gttggatttt gttatttttt tggaggtttt 2100
tgtttgatta gatttttgtt gggtagggga ttttttaggt tttttttttg atgttttatt 2160
tggggtattt tgaataggaa ggggtagttt tcgttttttg agaattagtg gtcgacgggg 2220

gttagttttt attatgaatt tgcgttttta ttttttattg agaattataa gtgtggattt 2280
tggaaaatgt aaatagtggt aagatgttag agattgttta ggttgagaag aggttttttt 2340
agagaaaggt gttttttttt atatgattag gaatatgagt ttaaaggttt aggtaaatat 2400
tttatggatt tttgtaaaga ttttagataa taaggagata agttttattg ataaaagaag 2460
gttaaaatta tagtagatag aagtaattta aagggtaaaa atgggattgg gttggagata 2520
agataaagat ttttttgaag ttttagaata tttggttata gaaatggatg ggatagttat 2580
aaatatgtgg aaatttttat aattgtttat tataatataa atagattatt tgtaatgatt 2640
gtatggatag aaatggaaga tgttagaata ggaaagtata tggtaattag gagttttgat 2700
gaaattttga ttttataatt tatttaagat aaaggatttt aatttttttt tttttttttt 2760
ttttgagata gagttttatt ttgttattta ggttggagtg tagtggtacg attttagttt 2820
attgtaattt ttgttttttg ggtttaagcg agttttacgt tttagttttt tgagtagttg 2880
ggattatagg tgtttattat tacgttcggt tagtttttgt atttttagta gagatagggt 2940
tttattatgt tgtttaggtt ggttttaaat ttttgatttt aggttatttg ttcgtttcgg 3000
ttttttaaag tgttgggatt ataggcgtga gttatcgcgt ttggttaagt tttttgattt 3060
ttatattatt ttgtaaatag gattagtgta tatagaatta gggaagaaag gtagattgta 3120
ttattgttat attattggaa aaattatttt ttagtttata aaattttata taaaatggaa 3180
atttttattg aaggtttatg taaagttgtg tagtttgtgt attgaataat tttagagggt 3240
gatatttatt ttataggtta ttatatttgt tttaatttaa aaagtatgtt tttttttata 3300
atttgtattg tatttatata gtggtatttt agtatttgta tgtggttata tatattttaa 3360
ttaagtatgt tattttatta attttttttt tagaaataag attataattt ttgttttttt 3420
agtattataa tgagttatta ttattatatt aaggtggttt tgaaatgtta atagtgtagt 3480
tttttttttt taagtttttt gttgattttt tttaattttt tttttttttt tgagacggag 3540
tttcgttttt gttatttagg ttggagtgta atggcgtaat tttagtttat tgtaatttcg 3600
gtttttcggg tttaagcgat ttttttgttt tagtttttcg agtagttgta attataggta 3660
tgtattatta tgtttagtta attttttttt ttttttttta gtagaaatag ggttttatta 3720
tgtttgttag gttggttttg aattttttag gtgattt 3757

<210> 281
<211> 10569
<212> DNA
<213> Artificial Sequence

<220>
<223> chemically treated genomic DNA (Homo sapiens)

<400> 281
   ttttggttaa tacggtgaaa tttcgttttt attaaaaata ttaaaaatta gtttggcgtg 60
   gtggcgggta tttgtagttt tagttattta ggaggttgag gtaggagaat ggcgtgaatt 120
   tgggaggcgg agtttgtagt tagcggaaat tgcgttaaaa aaaaaaaaaa aaaaatttat 180
   ttttttttgg gatgattttt tttttttatt tttcgttttt tttttttttt tttttttttt 240
   tgttttgttt tttttttttt tttttttttt tttttttttg agatggagtt tttgtcgttt 300
   aggttagagt gtagtggtgt gattttggtt tattgtaatt ttcgtttttt gggtttaagc 360
   gattttttta ttttagtttt tcgagtagtt gggattatag gtgtttgata ttatatttgg 420
   ttaatttttg tattttaata gagacggggt tttttttttt tttttttttt tagagataga 480
   ttttcgtttt gtcgtttagg ttggagtgta gtggcgggat tttagtttat tgtaagttgt 540
   gtttttcggg tttacgttat ttttttgttt tagttttttg agtagttggg attataggcg 600
   tttgttatta cgtttggtta atttttttgt atttttagta gagacggggt tttattgtgt 660
   tagttaggat ggtttcgatt ttttgatttc gtgatttgtt cgtttcggtt ttttaaagtg 720
   ttgggattat aggtgtgagt tattgcgttt agttgagacg ggttttattt atattgatta 780
   ggtttcgaat tttatattga ttttgcgatt ttagagaaag tgttgggatt ataggcgtga 840
   gttatcgtgt ttggttgaga tagggtttta ttatattgat ttggtttcga gtttttgatt 900
   ttgtgatttg tttgtttcgg ttttttaaag tgttggaatt ataggcgtga gttattgcgt 960
   ttagtttgta gatgtttttt ttttaaagtt tggaagttaa gggtgttaat gtagatatgg 1020
   ggttaaggtt ttaggggtag tttattatta agaattgtgt ttttaattga gatatttggt 1080
   aaattttgta ttgttttgta gagatggggt tttataatgt tatttaagtt ggttttgaac 1140
   gtttgttttt aagtgatttg ttagtttggg ttttttaaag tattgttatt ataggtatgt 1200
   atgagttatt ataatcggtt tgtttttttt ttttgagacg gagtttcgtt tttgttgttt 1260
   aggttggagt gtaacggtat gattttagtt tatcgtaatt tttgttcgtt gggtttaagc 1320
   gatttttttg ttttagtttt ttaagtagtt gggattatag gtatgtatta ttatattcgg 1380
   ttaattttgt atttttttag tagagatagg gtttttttac gtcggttagg ttagttttga 1440
   atttttgatt ttaggtgatt cgtttgtttc ggttttttaa gtgttgggat tataggcgtg 1500
   aggtatcgcg tttggttcgg tttgtttttt tttatattat tgtatattag tgtgattagt 1560
   ataaaagata attagagaat agttatatta tttattttat gggtagtaat tttgtttttt 1620
   ttatgtattg ttgtagggta tttttagttt aatgtttagt atttggagag agtataagaa 1680
   atattatttg gtcgggcgtt ttggtttacg tttgtaattt tagtattttg ggaggttgag 1740
   gtgggaggat tatttgagtt taggagtttg aaattagttt ggacgatata gtgtgattgt 1800
   ttttataaaa aattttaaga aaattagttg agtgtggtgt tttatttttg tagtttaagt 1860
   tattcgggag gttgaggtag gaggatcgtt tgagtttagg aggtcgaggt tatagtgagt 1920
   attgatcgtg ttatcgtagt ttaggttgga tgatttagta agtttgtgtt ttaaaaataa 1980
   aaataaaaaa ataaaaaaat aattatttga atgaatgagg aatcgagttt agatttattt 2040
   tttatttgtt tatttattgt gtattattta ataatattag ttgaggatta gagagttagt 2100
   aggtgggtta tttgttgtta tgatttttta taattaaata gtggaagtgg gagttattat 2160
   tgtttttggt gggttttttt tgttttgttt tgttttgttt ttgagacgga attttttttt 2220
   gttattaggt tggagtatag tggtacgttc gatttattgt aattttcgtt ttttgggttt 2280
   atgtaatttt gtttagtttt tttgagtagt tggtattata ggtacgcgtt attatagtta 2340
   gttaattttt gtatttttag tagagatggt gttttattat attggttagg atggttttaa 2400
   ttttttgatt ttatgattta tttgttttgg tttttaaaag tgttgggatt atagacgtga 2460
   gttattgggt ttagtatttt tttttttttt tttttttttt gagatggagt tttattttcg 2520
   ttatttaggt tggagcgtaa tgatatgatt ttggtttatt gtaattttta ttttttgggt 2580
   ttaagaaatt tttttgtttt agtttgtcga gtagttgtga ttataggtat ttgttattat 2640
   gtttggttaa ttaatttttt tgtattttta gtagaaacgg ggttttatta tgttggttag 2700
   gttggtttcg aattttttat tttaggttat ttgtttattt tggttttcga aagtgttggg 2760
   attatagatg ttagttatcg tatttagttt tattgtttgt tttataggtg aagaaagtga 2820
   ggtgtagatg ttttggtaaa tttgtttggt attatttatg tattatttag gtagtagaaa 2880
   tggagtcgtg ttttaaattt aggttttttt ggggtttggt ttggtggttt atgtttgtaa 2940
   ttttagtgta ttgggaggtt gagttaggag gattgtttga gtttagtagt ttgagattag 3000
   tttgggtaat atagtagatt ttagttttat agaatataat agataaaaaa aaaaaaaaaa 3060
   aaaaaaagtt ggtggtggtg gtatgtattt gtagttatag ttatttggga ggttgaggtg 3120
   gattttttga gtttaggagt ttgaggttgt attgagttgt ggttatatta ttgtatttta 3180
   gtttgggtta tagagtaaga ttttgttttt aataaaataa ataattaaat aaaaatttat 3240
   ttaggtgagg tggtttacgt ttgtaatttt aatatttggg gaggttgagg tgggcggatt 3300
   atttgaggtt aggagttaaa aattagtttg gttaatatgg tgaaattttg ttttaattaa 3360
   aaatataaaa attagtcggg cgtggtggcg gacgtttgta gttttagtta ttcgggaggt 3420
   tgaggtagga gaatcgtttg aatttgggag gtagaagttg tagtgagttg agatcgtgtt 3480
   attttatttt agtttgggta atggagtaag atttcgtttt aaaataaata aataaataaa 3540
   taaataaata aataaatata taaaaaagaa aatatataga agaggttaag aggtttaggg 3600
   tttttatttt cggaggaagg tttttttttt ttattattag tttgtatgta tttaaacgtg 3660
   ttttttgtaa cgacgatttt cgtggttgtt ttatttaaaa tgtttatgat ttttttagtg 3720
   tttattttat attgtattat ttattttttt tgagacggaa ttttattttt gttgtttagg 3780
   ttggagtgta atagtacgat ttcggtttat tataattttt attttttggg tttaagcgat 3840
   ttttttgttt tagtttttcg agtagttgtg attataggcg tgtgttatta cgttcggtta 3900
   attttttgta tttttagtaa agacggggtt ttatcgtgtt agttaggata gttttatttt 3960
   ttgattttat gatttattag tttaggtttt tttaagtgtt gggattatag gtatgagtta 4020
   tcgcgttcgg ttaatttttg tatttttaat agagacgggt tttattatgg tggttaggtt 4080
   cgtttttaat tttcgatttt aggtgattcg gtcgtttcgg ttttttaaag tgttggatta 4140
   taggcgtgag ttattaagtt cggtttttta tattgtatta ttttgtttat taattttgtt 4200
   ttttgttttt tttttttatt agatcgaggg ttttacggcg gttgggatat agtagtagat 4260
   taataaatat ttgttaagtt aattaatggt atttgggaag ttttgtagag aaggcggtta 4320
   cggtcgggtt tcgttttgtt tttttaaata ggtttcgcgt tttaggttag ggattaaagc 4380
   ggtttttagg cgcgtttttg gttcgcggga aattattgtt cggttttggt ttaggcggtt 4440
   cgttttacgg tttagggttt ttattttcga gttttaggga tttttttttt ttacggattt 4500
   tacggttatc gggattttat tttttagttg ttttatcgtt atcgcggtcg tcgttatcgt 4560
   cgtcgtagtg gcgttcgttt tgcgtatgtt tcggggcgtt aagacgcgag gcgtgtagaa 4620
   ggcgaagcgt gcgtaggcgc ggggttatcg cggttcgagt ggggcggtgt atcggacgcg 4680
   cgtgtttttt tcgggttacg tttattttcg ttcgtttagg gacgggcgcg tacgcgcgtc 4740
   gggagcggaa gggcgggttg gcgcgcgtac gaggtcgggt aggcggcgga agttggagag 4800
   gtcgtcgcgg tgttgagggg gaggggagtc ggcgagcgcg cgcgtagcgg gggcgcgggt 4860
   ggcgcgcgtg tgtgtgaagg gggggcggtg gtcgaggcgg gcgggcgcgc gcgcgaggtt 4920
   ttttttcgtt tggcggcggc ggcggttttt ttgtttcgtg aagagaagcg agacgtttat 4980
   tttgttttcg gtttcgcgcg gaggggcggg ggaggcgtcg ggaagtcgac ggcgtcggcg 5040
   gtttttggta aggaacgttg gtcgcggggg tagcgcggcg cggggtcggg ggaggcggtt 5100
   tgaatggata tatgtggtgg ttcgtgtttg taattttagt atttttggag gttgatgtag 5160
   gtggattagg agtttaagat tagtttggtt aatatggcga aatttcgttt ttattaaaaa 5220
   tataaaaaat atatatatat atatatttta gttaggtata gtggtgcgtg tttgtaattt 5280
   tagttattcg ggaagttgag gtacgataat cgtttgaatt taggaggcgg aggttgtagt 5340
   gagtcgagat tacgttattg tattttagtt tgggtgatgg agtaagattt tgtttttaaa 5400
   taaataaata aatagataga tagatatttt aatagttagt aagtatatag aaaggtgttt 5460
   cgtgttatta gttgtgaggg atatgtaaat gaagattata agaagatgcg attggatatt 5520
   ggtaatattg ttaaaagttg taaggattga tagtagtaag tgttggggtt gatgaagagt 5580
   atttggaatg ttgtttttgt gtgggaggtg ggatcgtgtg tgtagatgat ataattattt 5640
   tggaaaatta gtaaagttaa gtttatgttt ttttttataa ttattagttt tatttttgga 5700
   tatttaagaa gaattaatgt atgtgtttat taaatgattt ttatagtagt ttgatttgtg 5760
   ttggttagaa tttggtagag tataatgttt atgaaaatga gaatgtttat ataaaaagaa 5820
   cggatgattt ttttagatat ggtgattttg tgtgattgtt tttttttttt ttttttgaga 5880
   tagggttttt ttttgttgtt tacgattatg gtttattgta gttttgattt tttgggttta 5940
   aatagttttt tttatttagt tttttgagta taagttaatt taaaaaaaaa aatttgtaga 6000
   gatagggttt tgttatgttg tttaggttgg ttttgaattt ttggatttaa gcgatttttt 6060
   tgttttggtt ttttaaagtg ttgggattat aggtatgatt tattgttttt aatattatat 6120
   tatttatatg ttatatatat tatatgttat aagtatatat tgtatattat ataatatatg 6180
   tattatagac gtgatttatt atattcgtta ttttattgga agtttaataa taggtgaaag 6240
   gaagttatag tgatagttaa aatagtggtt tttttgggag agtggtattg attggggggg 6300
   tttaagaatg ttttttggaa cgttaggagg gatgtttgac gtagtgtttt ttaagttgtt 6360
   tatttatgat tttgcgatta gggtatgtat aagttagtga gagtaagtag aattatacgt 6420
   tgtttttttt agtattgagt tgaggacgtg gagtcgtttt acggtttttt attttgtgtt 6480
   gtaagtcggt tagtgtttgt ttaagtgtag gtattagtaa ttttattata ggttttggta 6540
   ttgggcgata ttgttgggga tggttttgta ttattttcgg gttgttggga ttttttttat 6600
   tgttattttg tacgtgtttg agtttttaat tcggaaatga atttgcggat gttggtggta 6660
   ttcgcgtttt tgttttattg tagtttgatt ttagaggtag tatttttagg aggagaggtt 6720
   attttaataa gtttttgtga aatttcgtgt ttttaaaaag tagatttgtt ttattcgggt 6780
   ataaaatggt tttttggggt agtattttta gtgtattttg ttttttcgtt gataaagtta 6840
   gcgttgtttt gagttggagt cgtttttgga ggttgtgacg gtatcgtatg atttgatgga 6900
   agggagaggg gagaagtttt aatgtttagt gtaaagtacg attaggtggt atacgtttgt 6960
   agttttagtt attcgggagg ttgaggtatg agaatcgttt gaatttcggg gacggaggtt 7020
   gtagtgagta gagattatgt tattgtattt tagtttgggc gatagagtga gattttgttt 7080
   taaaaaaaaa aaaatgtata atgaggagag ttataatttt agttattttt tagtgtatgt 7140
   tcgttggttt gtttattttt agagagggaa aagatggttt gtttttaatg atttggattt 7200
   tttttttttt tttttttgag atagcgtttc gtttttttgt ttaggttaga gtgtagtggt 7260
   gcggttatgg tttattgtag ttttaatttt ttggatttag gtaatttttt tattttagtt 7320
   ttttaagtag ttggggttat aggtatgtgt tattaagtat ggttaatttt tgtatttttt 7380
   gtagagatga ggttttgtta tgttgtttag gttgattttg aatttttggg tttaggttat 7440
   ttttttgttt tggtttttta aagtgttggg attataggcg tgttggattg tattttgttg 7500
   ttagttttag tttatttgat ttagaataaa gatttttaag tgaggaagaa gttataggtt 7560
   tttgaatatt ataggattta tgaagtatgt ggtagttttg ttgttttttt ttttatttta 7620
   ttgtaatatt ttgtggattt gagaagattg tggggataga aggtttagag gggagcgtta 7680
   aggagttagt gttttttttt gttttggtcg tgtttacgtt gttgggtttt ttatttatgg 7740
   aggtagttgt aggcgtattt tttttgtaga tatatttatg gattagaggt tttaggtttt 7800
   aaaattagta ttttagtaat ataaacgtaa ggtttagatt gttgttatat ggtagaggtt 7860
   agggcggtat tttataatta gatagatttt gtttttgtag tagaatgtgc gaatatttat 7920
   acggagtaga attaggttag attttgtttt tagatgaatt tttttgtagt aatatattta 7980
   ggtttgttta tgttaggggt tattttttta ggattggttg gagtgttagt tattggtttt 8040
   ttttttgttg agttttttgg tttttttttt tgttgttagt tatgaaagtg gaatgaggtt 8100
   attttgagtt ttttattttt tgtttaagaa tatattttta tggttgggtg tagtgtttta 8160
   tatttgtaat tttagtattt tgggaagttt aggttggagg attttttgag ttaggattaa 8220
   taagtgtttg agattagttt gggtaatata gagagatttt tgtttttata aaaaaattaa 8280
   gaaaaattag ttaggtatgg tgatgtattt ttatggtttt agttattcgg gaggttgagg 8340
   taggaggacg gtttgagttt tcgaggttaa ggttgtagtg ggttatgatg gttttattgt 8400
   attttagttt gggtgataga gtaagatttt gtatataaaa taaaaatttt tattttttaa 8460
   attaaaattt ttttcgttag attatttaat ataatgtaat ttttggttag gaacggtggt 8520
   ttatatttgt aattttagta tttcgggagg ttaaggcggg tggattattt gaaggttaga 8580
   cgttcgagat tagtttggtt aatgtggtga aatttcgttt ttattaaaaa taaaaaaatt 8640
   ggtttggcgt ggtggtttat atttgtaatt ttagtatttt gggaggtcga ggcggttggg 8700
   attacgaggt taggagttta agattagttt gaataatatg gtgaaagttt gtttttatta 8760
   aaaatataaa aattagttgg gtatggtggt gtgtgtttgt aattttaatt atttaggagg 8820
   ttgaggtagg agaattgttt gaatttggga ggcggaggtt gtcgtaagtt aagattacgt 8880
   tattgtattt tagtttgggt aataagagtg aaatttcgtt ttaaaaatat aaataaaata 8940
   aaaatagaat tttttttttt tttttgagac ggagttttgt tttgtcgttt aggttggagt 9000
   gcggtggtac gatttcggtt tattgtaagc gtcgtttttt gggtttatat tatttttttg 9060
   gtttagtttt tttagtagtt gggattatag gtgtttgtta ttatgttcgg ttaagttttt 9120
   tttttgtatt tttagtagag atagggtttt attatgttat ttaggatggt ttcgattttt 9180
   tgatttcgtg atttgttcgt tttggttttt taaagtgttg ggattatagg cgtgagttat 9240
   cgcgtttggt taaaaaatgg aattttttaa attaagagaa ttaaaaatgt ttttatattt 9300
   tttatagaat ttatttttag aattagaaat atgtcggaga gtttaggggt aaagagattt 9360
   attttaaaag gtagtagaaa taagatagat ttgcgatatg tttaattaga gaggggttat 9420
   taaaaaattt agggtgttag agttaggaaa tgtagtgtag tttttagaat ttgtgtttta 9480
   aagattatta aatgataaga attgttatgg gtaagattat atgaaagttt agaatgtatt 9540
   gagaaatttt attgagtgtg ggtgcgtttt tagttagcga gggtttttag ttagcgaggc 9600
   ggagaagttt ttttatagat ataagtattt tttttttttt atttgttttt gggtggttgg 9660
   aggatatttt tttatgtata ttagtattta aaggaaaagg tgacgttttt aagtgttagg 9720
   gattaaaaag tgttattatt tgtgtaatat tttatttttt ttaaatttaa gtttatttat 9780
   tttaaggaaa atttggtttt attattttta atggaaaatt agtagtattt gttataaata 9840
   tttgttttgg ttgaattgat ggaatttcgg ttagaggttt gttttttttt tttttttttt 9900
   gagatagagt gtcgttttat tgtttaggta ggagtgtagt ggtgcgattc gggtttatcg 9960
   cgattttcgt tttttaagtt taagcgattt ttttgtttta gttttcggag tagttgggat 10020
   tataggcgtg tgttattatg tttagttgat tttcgtattt ttagtagaga tggggttcgt 10080
   tttgttggtt agattggttt cgaatttttg attttaaatg atttgttcgt attggttttt 10140
   taaagttttg ggattatagg tgtgagttat tgtattcggt tagttagagg ttatttgagt 10200
   tatagttttg ttttttttgt taggaagata agtaagtgga gagtagtgtg aagggtagat 10260
   tagtattttt tgagatattt tttgtcgtga ttaggaatat ggtaggagtt tttgaaggtt 10320
   tttgtggggt agtgtttgtt tgtgagcgtg gtttattttt ttttgggtag attgattgat 10380
   tttgtgtgtt tgagatgtag gatttttttt gagagtgatt atggagaaac ggtttgggtg 10440
   gttggtgggg aaggtattgg ttttttgtgg gatgtagatt ttgatgtgat cgtatgattg 10500
   ttttttgtta tttttgtttt gtttcgtttt tggttatgaa ttttagattg attaggattg 10560
   ttttttagt 10569

<210> 282
<211> 10569
<212> DNA
<213> Artificial Sequence

<220>
<223> chemically treated genomic DNA (Homo sapiens)

<400> 282

gttggaagat agttttggtt agtttggggt ttatgattaa gggcgaggta ggatagggat 60
agtaagggat aattatacgg ttatattaga atttatattt tataggaagt tagtattttt 120
tttattagtt atttaaatcg tttttttatg attattttta gaggaagttt tatattttag 180
gtatataaga ttagttagtt tatttaggaa ggggtgggtt acgtttatag ataagtattg 240
ttttataggg gtttttaggg gtttttgtta tatttttgat tacggtagag ggtgttttag 300
ggggtgttgg tttatttttt atattgtttt ttatttgttt atttttttaa tagaaagaat 360
aggattatag tttaaatagt ttttgattgg tcgggtgtag tggtttatat ttgtaatttt 420
agaattttgg gaggttaatg cgggtagatt atttgaggtt aggagttcga gattagtttg 480
gttaataagg cgaattttat ttttattaaa aatacgaaaa ttagttggat atggtggtat 540
acgtttgtgg ttttagttat ttcggaggtt gaggtaggag aatcgtttga atttgggaga 600
cggagatcgc ggtgagttcg gatcgtatta ttgtattttt gtttgggtaa tagagcgata 660
ttttgtttta gaaaaaaaaa aaaaaaaata agtttttaat cgaaatttta ttaatttagt 720
taaaatagat atttatggta aatattgttg gtttttattt gaaagtagta agattaagtt 780
tttttaaaaa taaatgaatt taagtttaaa aaggataaag tattatataa ataatggtat 840
tttttggttt ttgatatttg gaaacgttat tttttttttt agatgttaat atgtataagg 900
ggatgttttt taattattta gagataaatg ggaaaggaaa gatatttgtg tttgtaaaga 960
ggttttttcg tttcgttggt tgaaggtttt cgttggttga agacgtattt atatttagtg 1020
aaatttttta atatatttta ggtttttata taattttatt tatggtaatt tttattattt 1080
aatagttttt aaaatataaa ttttaaaagt tatattgtat tttttggttt tgatattttg 1140
ggttttttga tagttttttt ttggttaagt atgtcgtaaa tttgttttgt ttttgttgtt 1200
ttttggaatg aatttttttg tttttgaatt tttcggtata tttttggttt taaggataaa 1260
ttttgtaaga agtgtagaaa tatttttaat ttttttgatt taaaaagttt tattttttgg 1320
ttaggcgcgg tggtttacgt ttgtaatttt agtattttgg gaggttaagg cgggtagatt 1380
acgaggttag gagatcgaga ttattttggg taatatggtg aaattttgtt tttattaaaa 1440
atataaaaaa aaagtttagt cgggtatggt ggtaggtatt tgtagtttta gttattgggg 1500
aggttgagtt aggagaatgg tgtgaattta ggaggcggcg tttgtagtga gtcgagatcg 1560
tgttatcgta ttttagtttg ggcgatagag taagatttcg ttttaaaaaa aaaaaaaaag 1620
ttttattttt gtttatttta tgtttttgag acgaagtttt atttttgttg tttaggttgg 1680
agtgtaatgg cgtgattttg gtttacggta attttcgttt tttaggttta agtaattttt 1740
ttgttttagt tttttgagta gttgggatta taagtatata ttattatgtt tagttaattt 1800
ttgtattttt aatagagata ggttttattt atgttattta ggttggtttt gaatttttga 1860
tttcgtgatt ttaatcgttt cgatttttta aagtgttggg attataggtg tgaattatta 1920
cgttaggtta atttttttat ttttagtaga gacggggttt tattatattg gttaggttgg 1980
tttcgaacgt ttgattttta ggtgatttat tcgttttggt ttttcgaagt gttgggatta 2040
taggtgtgag ttatcgtttt tggttaaaaa ttatattgta ttaggtgatt tagcggaaaa 2100
agttttaatt tgaaaggtag gggtttttgt tttgtatata gagttttatt ttattattta 2160
agttggagtg tagtggagtt attatagttt attgtagttt tgatttcggg ggtttaagtc 2220
gtttttttgt tttagttttt cgagtagttg ggattatagg agtgtattat tatgtttggt 2280
taattttttt taattttttt gtagagatag ggtttttttt atgttgttta ggttggtttt 2340

aaatatttgt tgattttggt ttaagggatt ttttagtttg gatttttttaa agtgttggaa 2400
ttataggtgt gagatattgt atttagttat agggatatat ttttagataa gagatgaaag 2460
gtttaaaatg attttatttt atttttatgg ttaatagtag gagagaggat taggaaattt 2520
agtaggggag gaattagtgg ttggtatttt agttaatttt gaaaagatgg tttttgatat 2580
aaataaattt aaatgtgtta ttgtaaggaa atttatttgg aggtaaaatt tgatttgatt 2640
ttgtttcgtg tggatattcg tatattttgt tatagaaata ggatttgttt gattatgaga 2700
tgtcgttttg gtttttgtta tgtgataata gtttgaattt tgcgtttatg ttattaagat 2760
gttgattttg gagtttgaag tttttggttt atgggtatgt ttgtagggag gatgcgtttg 2820
tagttatttt tataggtggg aagtttagta acgtgggtac ggttagggta gggggaggta 2880
ttggtttttt ggcgtttttt tttgggtttt ttgtttttat agttttttta ggtttataga 2940
gtgttgtaat gaggtaggaa aagggatagt aggattgtta tatattttat agattttgtg 3000
atatttaggg atttatagtt ttttttttat ttgaaggttt ttattttaag ttaaataaat 3060
tgaaattgat agtaaaatat aatttagtac gtttgtaatt ttagtatttt gggaggttaa 3120
ggtaggggga tggtttgaat ttagaaattt aagattagtt tgggtaatat ggtaaaattt 3180
tatttttata aaaaatataa aaattagtta tgtttggtga tatatgtttg tagttttagt 3240
tatttgggag gttgaggtgg gagggttatt tgagtttagg aggttgaggt tgtagtgaat 3300
tatgatcgta ttattgtatt ttagtttggg taaaagggcg agacgttgtt ttagaggaaa 3360
aagagaaaaa gatttaggtt attgagggta aattattttt tttttttttg aaggtggata 3420
agttaacgag tatatattgg gaagtgattg aaattatagt tttttttatt gtatattttt 3480
ttttttttga gatagagttt tattttgtcg tttaggttgg agtgtagtga tatgattttt 3540
gtttattgta atttcgtttt tcggggttta agcgattttt atgttttagt ttttcgagta 3600
gttgggatta taggcgtatg ttatttaatc gtattttata ttgagtatta gaattttttt 3660
tttttttttt ttattaaatt atgcgatgtc gttatagttt ttaggaacgg ttttaattta 3720
aagtagcgtt aatttgttta acggaaaaat agagtatatt gaagatgttg ttttaaaaaa 3780
ttattttgtg ttcgaataag gtaagtttgt tttttagaag tacggggttt tatagaggtt 3840
tgttagaatg gttttttttt ttgaaaatgt tgtttttaga gttaggttgt agtgaaatag 3900
gaacgcgggt gttattagta ttcgtaaatt tattttcgga ttgaaagttt agatacgtgt 3960
agagtgatag taaaggggat tttaatagtt cgggagtgat gtaaagttat ttttagtagt 4020
atcgtttagt gttaaagttt gtgatgaggt tgttggtgtt tgtatttgaa taagtattgg 4080
tcgatttgta gtataaagtg gagagtcgtg gagcgatttt acgtttttag tttagtattg 4140
aggggagtag cgtgtgattt tgtttatttt tattgatttg tatatgtttt gatcgtagga 4200
ttataggtga atagtttgaa gaatattgcg ttagatattt tttttgacgt tttagaaggt 4260
atttttgggt ttttttagtt aatattattt ttttagggaa attattattt tgattattat 4320
tgtggttttt ttttatttgt tattgaattt ttaataaaat gacgggtgtg gtgggttacg 4380
tttataatat atatattata taatatataa tatatattta taatatataa tatatgtaat 4440
atataaataa tataatgttg ggggtagtgg gttatattta taattttagt attttgggag 4500
gttaaggtag aaggatcgtt tgagtttagg aatttaagat tagtttaggt aatatagtaa 4560
gattttgttt ttataagttt ttttttttaa attagtttat atttaggagg ttgagtaggg 4620
agaattgttt gagtttagga ggttaaggtt gtagtgagtt atgatcgtgg gtaatagaga 4680
aagattttgt tttaaaaaaa aaaaaaaaag ataattatat agggttatta tgtttgggag 4740
agttattcgt tttttttatg tgagtatttt tatttttata gatattgtat tttattaggt 4800
tttggttagt ataaattaag ttgttatgag agttatttgg tgaatatatg tattggtttt 4860
ttttgggtgt ttaagaatga aattggtggt tataaaggga ggtataaatt tagttttgtt 4920
agttttttaa aatagttgta ttatttatat atacggtttt attttttata tagggatagt 4980
attttaggtg ttttttatta attttaatat ttgttgttgt tagtttttat aatttttaat 5040
aatgttgtta atgtttaatc gtattttttt atggttttta tttgtatgtt ttttataatt 5100
aatggtacgg agtatttttt tgtatgttta ttggttattg agatatttat ttatttattt 5160
atttatttat ttagagatag agttttgttt tattatttaa gttggagtgt agtggcgtga 5220
tttcggttta ttgtaatttt cgttttttag gtttaagcga ttatcgtgtt ttagtttttc 5280
gagtagttgg gattataggt acgtattatt atatttggtt aaaatatata tatatatata 5340
ttttttgtat ttttagtaga gacggggttt cgttatgttg gttaggttgg ttttgaattt 5400
ttgatttatt tgtattagtt tttagaagtg ttgggattat aggtacgagt tattatatat 5460
gtttatttaa atcgtttttt tcggtttcgc gtcgcgttgt tttcgcggtt agcgtttttt 5520
attaggagtc gtcggcgtcg tcgatttttc ggcgtttttt tcgttttttc gcgcggggtc 5580
gggggtagaa tgggcgtttc gttttttttt acgaaataaa gaagtcgtcg tcgtcgttaa 5640
acgaggggaa gtttcgcgcg cgcgttcgtt cgtttcggtt atcgtttttt ttttatatat 5700
acgcgcgtta ttcgcgtttt cgttgcgcgc gcgttcgtcg gttttttttt tttttagtat 5760
cgcggcggtt tttttagttt tcgtcgtttg ttcggtttcg tgcgcgcgtt agttcgtttt 5820
ttcgttttcg gcgcgcgtgc gcgttcgttt ttgggcggac gggggtgggc gtggttcgga 5880
ggaagtacgc gcgttcgatg tatcgtttta ttcgggtcgc gatggtttcg cgtttgcgta 5940
cgtttcgttt tttgtacgtt tcgcgttttg acgtttcgga gtatgcgtag agcggacgtt 6000
attgcggcgg cggtggcggc ggtcgcggtg acgatggggt aattggagaa tgggatttcg 6060
gtggtcgtgg ggttcgtggg gaaaggaggt ttttgaggtt cggaaatagg aattttggat 6120
cgtaggacgg gtcgtttgga ttaagatcgg gtagtggttt ttcgcgggtt aagggcgcgt 6180
ttgggaatcg ttttgatttt tgatttgggg cgcgaggttt atttggggag gtaaggcggg 6240
gttcgatcgt ggtcgttttt tttgtaaaat tttttaagtg ttattaatta gtttagtaaa 6300

tatttattga tttgttattg tgttttagtc gtcgtggagt tttcggttta gtggggaagg 6360
aggataagaa gtaagattaa taagtaaaat agtgtagtat gagaggtcgg atttggtggt 6420
ttacgtttgt aatttagtat tttgggaggt cgaggcggtc ggattatttg aggtcgggag 6480
ttagaaacga gtttgattat tatggtgaaa ttcgttttta ttaaaaatat aaaaattagt 6540
cgggcgcggt agtttatgtt tgtaatttta gtatttaggg aggtttaggt tggtggatta 6600
tgaggttagg agatgagatt attttggtta atacggtgaa atttcgtttt tattaaaaat 6660
ataaaaaatt agtcgggcgt ggtggtatac gtttgtagtt atagttattc gggaggttga 6720
ggtaggagaa tcgtttgagt ttaggaggtg gagattgtag tgagtcgaga tcgtattatt 6780
gtattttagt ttgggtaata agagtgaaat ttcgttttaa aaaaaataaa tagtatagtg 6840
tgaggtgaat attgggagga ttatggatat tttaaatagg gtagttacgg aagtcgtcgt 6900
tgtaaagaat acgtttgaat gtatataggt tggtggtgaa agagagaaat tttttttcga 6960
gagtgaaggt tttgagtttt ttggtttttt ttgtgtgttt ttttttttgt gtgtttgttt 7020
gtttgtttgt ttgtttgttt gtttgttttg agacggagtt ttatttattt gtttaggttg 7080
gagtgaggtg gtacgatttt agtttattgt aatttttgtt ttttagattt aagcgatttt 7140
tttgttttag tttttcgagt aattgggatt ataggcgttc gttattacgt tcggttaatt 7200
tttgtatttt tagttgagat agagttttat tatgttggtt aggttggttt ttaatttttg 7260
attttaagtg attcgtttat tttagttttt taaagtgttg ggattatagg cgtgagttat 7320
tttatttggg tgggtttttg tttaattgtt tgttttatta gagatagggt tttgttttgt 7380
aatttaggtt ggagtgtaat ggtgtaatta tagtttagtg tagttttaaa tttttggatt 7440
taagggattt attttagttt tttaaatagt tgtgattata ggtatatgtt attattatta 7500
gttttttttt tttttttttt tttttgtttg ttatgttttg tagagttgag gtttgttgtg 7560
ttatttaagt tggttttaaa ttattgggtt taagtagttt ttttggttta gttttttagt 7620
gtattgggat tataggtatg agttattaag ttaggtttta gaaggatttg ggtttaaaat 7680
acgattttat ttttattatt tgggtgatgt atgggtaata ttaggtaggt ttattagagt 7740
atttatattt tatttttttt atttgtgaaa taaataataa ggttgggtgc ggtggttgat 7800
atttgtaatt ttagtatttt cggaagttaa ggtgggtaga tgatttgagg taaggagttc 7860
gagattagtt tggttaatat ggtgaaattt cgtttttatt aaaaatataa aaaaattaat 7920
tagttaggta tagtggtagg tgtttgtaat tatagttatt cggtaggttg aggtaggaga 7980
attttttgaa tttaggaggt ggaggttata gtgagttaag attatgttat tgcgttttag 8040
tttgggtgac gagagtaaaa ttttatttta aaaaaaaaaa aaaaaaaaaa aaatgttggg 8100
tttagtggtt tacgtttgta attttaatat tttgggaggt taaggtaggt ggattatgag 8160
gttaagagat tgagattatt ttggttaatg tggtgaaata ttatttttat taaaaatata 8220
aaaattagtt ggttgtggtg gcgcgtgttt gtagtgttag ttatttagga ggttgaggta 8280
gaattgtatg aatttaggag gcggaggttg tagtgagtcg agcgtgttat tatattttag 8340
tttggtgata gagggagatt tcgttttaaa aataaaataa gataaaataa aaaaaattta 8400
ttagaaataa taatggtttt tatttttatt gtttggttat gaggaattat gatagtaggt 8460
agtttatttg ttagtttttt gatttttagt tagtgttatt gaatagtgta tagtaagtga 8520
gtaagtagaa gtgggatttg aattcgattt tttatttatt taagtagtta tttttttgtt 8580
tttttgtttt tgtttttgag atataggttt gttgggttat ttagtttgga ttgcggtggt 8640
acgattagta tttattgtag tttcgatttt ttgggtttaa gcgatttttt tgttttagtt 8700
tttcgagtag tttggattat aagggtggga tattatattt agttaatttt tttaaaattt 8760
tttgtagaga tagttatatt atgtcgttta ggttggtttt aaatttttgg gtttaggtga 8820
tttttttatt tttagttttt aaagtgttgg gattataggc gtgagttaag gcgttcggtt 8880
aaatagtatt ttttatgttt tttttaaatg ttaagtattg agttaggaat attttgtagt 8940
aatgtatgga ggaggtaggg ttgttgttta tggagtggat ggtatgattg ttttttaatt 9000
gttttttatg ttaattatat taatatgtaa tagtgtaaag aaaaataggt cgggttaggc 9060
gcggtgtttt acgtttgtaa ttttagtatt tgggaggtcg aggtaggcgg attatttgag 9120
gttaggagtt taggattagt ttgatcgacg tggagaaatt ttgtttttat taaaaaaata 9180
taaaattagt cgggtgtggt ggtgtatgtt tgtaatttta gttatttggg aggttgaggt 9240
aagagaatcg tttgaattta gcgggtagag gttgcggtga gttgagatta tgtcgttgta 9300
ttttagtttg ggtaataaga gcgaaatttc gttttaaaaa agaaaaatag gtcggttgtg 9360
gtagtttatg tatgtttata atggtagtat tttgggaggt ttaggttggt agattatttg 9420
aggataggcg tttaagatta gtttgggtaa tattgtgaaa ttttattttt ataaagtaat 9480
ataaaatttg ttaggtgttt taattaagag tatagttttt aataatgaat tgtttttggg 9540
attttggttt tatgtttgta ttgatatttt tggtttttag attttaaaaa aaaaatattt 9600
ataggttggg cgtagtggtt tacgtttgta attttagtat tttaggaggt cgaggtaggt 9660
aaattataag gttaggagtt cgagattagg ttaatatggt gaaattttgt tttaattagg 9720
tacggtggtt tacgtttgta attttagtat tttttttggg atcgtaaggt taatatggag 9780
ttcgagattt ggttaatatg gtgaaatttc gttttagttg ggcgtagtgg tttatatttg 9840
taattttagt attttgggag gtcgaggcgg gtagattacg aggttaggag atcgagatta 9900
ttttggttaa tatagtgaaa tttcgttttt attaaaaata taaaaaaatt agttaggcgt 9960
agtggtaggc gtttgtagtt ttagttattt aggaggttga ggtaggagaa tggcgtgaat 10020
tcgggaggta tagtttgtag tgagttgaga tttcgttatt gtattttagt ttgggcgata 10080
gagcgagagt ttgtttttaa aaaaaaaaaa aaaagagaga tttcgttttt attaaaatat 10140
aaaaattagt taggtgtggt gttaagtatt tgtagtttta gttattcggg aggttgaggt 10200
aggagaatcg tttgaattta ggaggcgaag gttgtagtga gttaagatta tattattgta 10260

ttttagtttg ggcgatagag attttatttt aaaaaaaaaa aaaaaagaag gggaggagag 10320
ggtagggtag gggaggggag gggagaggag ggaacgggaa gtgggggagg ggaattattt 10380
tagaggagaa tggatttttt tttttttttt tttggcgtaa ttttcgttaa ttgtaagttt 10440
cgttttttag gtttacgtta tttttttgtt ttagtttttt gagtagttgg gattataggt 10500
gttcgttatt acgttaggtt aatttttggt atttttagta gagacggggt tttatcgtgt 10560
tagttagga 10569

<210> 283
<211> 6204
<212> DNA
<213> Artificial Sequence

<220>
<223> chemically treated genomic DNA (Homo sapiens)

<400> 283
   ggttttatta cgttggttag gttggtttta aatttttgat tttaagtgat tcgtttgttt 60
   tagtttttta aagtgttagg attataggat gagttattac gttaggtttt aagatttatt 120
   tattaatatt taattaattt agttttaggt gagttttagg ttagatgata gatgttgaga 180
   ttatgaagat agggtttaag gggtttaagt ttttaggaat ttatagttta ggggaagatg 240
   tttgttagta cgtggttaga gagttagtta gatttgattt ttattttgag tttttattta 300
   gtttttagtt gtgagattag ttttgagtta gttttttatt tttttagttt tcgttttttt 360
   aattgtaaaa taagaattat aatattattt ttattgggtt ttggtgagga ttaaatgaga 420
   taatttatgt aaaatgttta gtatagtttt tggaatatag tagtaattat ttaattaatg 480
   ttaattattg ttttttttta ggaagtaggt taatatgtgg atttattaga atttaaggta 540
   gagaatgtat tttagatggg gaaataattt gtcgaaggag tttggtagga gattaatttt 600
   gttagtaaga atagaaggaa gtttagagga ggtgatgttg taatagggtt ttgaagaatg 660
   agtaagagtt tttaggtagg gattgaggaa agagatttta ggtagaagga agattattta 720
   tagaggtatg aagttgtaga aggtgtagat atgtttggag gaaggtaata tatttagtag 780
   ggtttgtggg attaggttag aaaagggggg atttaggtta gtttttgtgg aatttatttt 840
   ttttttgaga tagagtttta ttgtattatt taggttggat tgtaatggtg taattttagt 900
   ttattgtaat ttttgttttt tgggtttaag cgattttttt gttttatttt attgagtatt 960
   tgggattata ggtgttcgtt aatacgtttg gttaattttt gtatttttag tagagataga 1020
   tttttattat gttggttagg ttggttttga atttttgatt ttaggtgatt tattcgtttc 1080
   ggttttttaa agtgttgaga ttataggcgt gagttattgt gttgggtttg taggatttta 1140
   tgatttaaat aaagagggag aaagaaggaa tattaattga gtttttatta tatgtatggt 1200
   attgaattat ggtgtttttt tttttttttt tttttttttt gagatagagt tttattttgt 1260
   tgtttgggtt ggagtgtagt ggtatgattt cggtttattg taatttttgt ttttcgggtt 1320
   taaacgattt ttttgtttta gttttttaag tagttgggat tataggtatt cgttattatg 1380
   tttagttaat ttttttgtat ttttagtaga gacggggttt tattatgttg gttaggttgg 1440
   tttcgaattt ttgattttag gattcgttcg tttcggtttt ttaaaatgtt gggattatag 1500
   gtatgagtta ttgcgtttag ttatggtatt gaattaaatg gttagttttt tgtttattgt 1560
   ttgtttttga gataaggttg gttttgtttt gttgtttaga ttatagtata gtagaatagt 1620
   tatagtttat tgtaatttcg aatttttttt taagtttaag tgattttttt gttttagttt 1680
   ttagaatagt tgggattata ggtatgttat tattacgttc ggttaatttt ttgtaaagtt 1740
   ggtgttttgt tatgttgttt aggttgattt cgaatttttg gttttaaatg atttttttgt 1800
   ttcggttttt taaagtttta ggattatagg tatgagttat tgtgtttggt tagtattttt 1860
   aaaatttaat ttggattggt cggatacggt ggtttttagt attttgggag gttgaggtgg 1920
   gcggattatt tgaggttagg agtttaagat tagtttagtt aatatagtaa aatttcgttt 1980
   ttattaaaaa tgtaaaaatt agttgggtat ggtggtgggt atttgtagtt ttagttattt 2040
   gggaagttga ggtaggagaa ttgtttgaat ttgggaggcg gaggttgtag tgatttagga 2100
   ttatattatc gtattttagt ttaggcgata agaggaaatt ttgttttaaa aaaaaaaaaa 2160
   aaaaaaaatt aatttggatt ttattttgtc ggtagtaggg aattatagag atttttaagt 2220
   cgattgtgat atgattgaat tttttttttt tttttttttt gagttagggt tttgttttat 2280
   cgtttaggtt ggagtgtagt ggagggattt gggtttattg taatttttat tttttgggtt 2340
   taagtaattt ttttgtttta gttttttgag tagttgggat tataggtacg tgttattatg 2400
   gttagttaat tttttatttt gttttgagat ggagtttcgt ttttgttgtt taggttagaa 2460
   tgtaatggcg cgatttcggt ttattgtaat ttttattttt cgggtttaag agattttttt 2520
   tttttagttt ttcgagtagt agggattata ggtgtaagtt attatgattg gttgattttt 2580
   gtatttttag tagagatagg gttttattat attagttagg ttggtttcga atttttgatt 2640
   ttaggtgatt tgttcgtttt agttttttaa agtgttggga ttataggcgt gagttatagt 2700
   gtttagttta atttttgtat ttttagtaga gacggggttt tattgtgttg gttaggttgg 2760
   ttttgaattg attttgtgat ttattcgttt tggtttgaat gtttatttat ttaattttta 2820
   ggatgatgtt tttggtagtt agtggaagaa taggatagag taaggagaga ataattaggt 2880
   ggttaaggta gtggtttaag ggatagaaat tgtagtattt agttaagagg aatttggcgg 2940
   ggttgtggag aaaggttagt gtttgggttg aagagaattt ttgtaggtta aatttatagt 3000
   cgtagaggta gattttatgt gttattggtg gggaggttaa tgagggaaat agaggttagt 3060
   ttttgagaag ttagaagtag ggtagggttt gaatgaatgg aataaagtta tgggacgtaa 3120
   agtttttaaa tttgattttg aatttggtat atttttgagt ttttttgtga ttttgaaaat 3180
   gttatattat gttgttttta gttttttatt agcggataat atggtttttt ttatttgtta 3240
   tttatgattg tgagaaagaa attaggtagg gagtgtataa ggtgtgtaag tgttttgtaa 3300
   gtagtaaatt attaaataaa ttttagattt tatgttatta gggaggtagt tggtcgtacg 3360
   ggatagcgta gttataagag attataggcg gagatggggg atatttgttt tgtggtgtag 3420
   ataaatagtt taattaatat atatatattt tataggtaaa tatagtaatt tttatattga 3480
   gttgtgttta ttagagggta ataggaggtt tttaattttt attattagtg attttatgaa 3540
   ttgttttgta gatgtttttg ttttttattg gatatgaggt ggtacgagcg gtttgaattt 3600
   gtagatttag ataggtttta ggtatttggg gaattatgtg agtaaaattt tgtattgttt 3660
   ttggggttag aatttagttt tttttaaata tttagaagga tttgtgattg tttttaaagg 3720
   ttaagagtta ttgatttttt ttattagaaa ttaataagaa tagttatttt tggggagaag 3780
   gaatgatggg ggtagggaaa gatttttatt taatattttt gatttttttg aacggtttgt 3840
   attttttaaa taggaatatg tattaggttt attgaaaaaa aatatgttag ggttagttga 3900
   gtagtgagat aatgggtttt ttttagattt ttgtttttat atttgttcgt attaaataaa 3960
   tataaatatt aataatatat ttttaaagat ttaaaaaaat aaaaattaaa aattttgata 4020
   gtattagtat atatatagaa attattttat tatgtaaagt ttatttttta ttatgaaagg 4080
   taaaatgttt atatttttta ttaattattg gttttaattt agtaaaattt gtatattaag 4140
   taggtaaggt ggaaaagaaa aaggtagaat attttatgta ttttaattta gacgtataaa 4200
   aatgttaagt tttatacgtt attagttttc gtatatatcg ttttttgtat tcgtttgtac 4260
   gggttaatgg gttagttggt cggcgtttga tgtttgaagt gatggaacgg agtacggggt 4320
   taaatttatt attttttttt tacgtatttt agtaattatt ttatttttac gttatgtttt 4380
   cgggtgtgtg tgtttttgtt tacgtagaaa ttgaagttta aagtaggcgg agttatttat 4440
   gttttttttg ttgtttttag aatttaattt aggagttggg tttttagagg atttggagat 4500
   atttggggat tatttaatta gttgattttc gcgtggtaag aggtttttaa tttcgttatt 4560
   acgtggtgtt aagggtcggg aaaagggaga gcgggtagga gggagttgta ttaggggtat 4620
   ttaaagtgtt ttgacgttac gtattgttag gaatttagtt gagtttttag taggatattt 4680
   cggttatttt tttttttttt tttcgggttt ttgtgtttaa gttttcggtt tttttttcgt 4740
   tgtggcggag ggaggagtat cgaattgtcg gaatagttag tataggggcg tattagtttt 4800
   tttttggttt cgtttttttt tttagttgtt ttttttattg gttagtgggc ggggtttcgg 4860
   ttgtatcgta tacgtattgg gatttttggg tggtcgaacg agttggtttt ttatgaatta 4920
   tgtatgacgg cgtgtttcgg ttaggttggg gttgggcgag gattggttga aggggttgta 4980
   atttagcggt tttcggagtt gcggcggcgt agattgggag ggggagtcgg gggtttcgac 5040
   gtcgtagtcg agggaataag ttttaatcgg attttggata ggtatttcgg tttggcgttg 5100
   tttttttttt tcggttcgga gaggtttttc ggtttgaggg agtttcgtcg ttcgttttcg 5160
   gtatacgcgt acgttcggtt tttcggtttt tgtcggagaa ataggtgaag ggggtgtagg 5220
   gtggggtcgt tggggaggtt tggggattcg ggggtttcgt agcggtaggg ggtttttggg 5280
   atttggggga tgttgtgatg gacgttgtag tggggtcggg agagatgaag agacgcggag 5340
   ggtcgttttg agggaagatt tttcgggatg ataggagcgg gtttcggaag ggattcgggg 5400
   cgttggaggg aagtttcgtt tttcggagaa atagaacgcg ttcgaggggg tatcgtgggg 5460
   cgagggcgta ttcggttgcg gcggtaggag tgagggatag tttttcgatt ttttgttttt 5520
   tggggttttg gggacgtttc ggttatcgga gcgattgtta cgtcgacggg gattatcggc 5580
   gcgagtgggg ggtcggaaag cgtttttttt tcgttcggtc ggcggttttc gttgagttat 5640
   ttttttcgtt tgttttgttt tcgttttttt aggagttagt tgtgtttttt tggaggtagg 5700
   aataggtgtg tttgtcgttt gtagttttac gggttggttg gtcgtgggta ggttttattg 5760
   tataagaatt aagttttttg tagggaaatt gatagatcgg tatttttttt aaattttttc 5820
   gtattttttt ttaggttaaa ttatgttttt tttacgtttt cgttttgtaa aaaagagaaa 5880
   aaaagataaa ataaaatttt tattaattcg ttaagttagt tttagagaga gaaataaatt 5940
   ttttgatatt gttttttttt aaatatttgg taaagtcggt tagaagataa ataattgagt 6000
   tattgtattt attggattgt ggtgttgttt aattgtatag gacggaatga attaattgag 6060
   agtgggagtt ttttgtttta gagttaagat tttgggtaaa tgtagaggag agggaaataa 6120
   agataggttg gttttgaaaa aattatgtgt gtaaatttta tatgtatttg gggggtgtgg 6180
   ttgtattgaa gttaataaga ttta 6204

<210> 284
<211> 6204
<212> DNA
<213> Artificial Sequence

<220>
<223> chemically treated genomic DNA (Homo sapiens)

<400> 284

tgaattttgt taattttagt gtaattatat tttttaaatg tatgtaaagt ttgtatatat 60
ggttttttta aggttagttt gttttgtttt tttttttttt tgtatttatt taagattttg 120
gttttgagat agaaaatttt tatttttaat tggtttattt cgttttatgt aattaagtaa 180
tattataatt tagtaaatgt aatggtttaa ttatttattt tttggtcgat tttattaggt 240
atttggaaaa ggataatgtt aagaggttta tttttttttt tagagttggt ttgacgggtt 300
gatggggatt ttattttgtt tttttttttt ttttttataa ggcggggacg tggggggagt 360
ataatttaat ttagaaaaag atgcgaggga atttagaaag agtatcggtt tgttaatttt 420
tttataggaa atttgatttt tatgtaataa agtttattta cgattagtta gttcgtaagg 480
ttgtaggcga tagatatatt tatttttgtt tttaaaaggg tatagttggt ttttgaagga 540
gcgggaatag ggtaagcgga ggaagtggtt tagcgggagt cgtcgatcgg gcggggagga 600
ggcgtttttc gatttttttat tcgcgtcggt gattttcgtc ggcgtgatag tcgtttcggt 660
ggtcggaacg tttttagggt tttagggagt aggaaatcgg gggattgttt tttatttttg 720
tcgtcgtaat cgagtgcgtt ttcgttttac ggtgtttttt cgagcgcgtt ttgttttttc 780
gaagaacgaa attttttttt agcgtttcga gtttttttcg aggttcgttt ttgttatttc 840
gaagagtttt tttttagggc gatttttcgc gtttttttat ttttttcggt tttattgtag 900
cgtttattat aatattttta aggttttaga ggttttttgt cgttgcggag ttttcgggtt 960
tttaggtttt tttaacggtt ttattttgta ttttttttat ttgttttttc ggtagaggtc 1020
gagaggtcgg gcgtgcgcgt gtgtcgggga cgggcggcga ggttttttta ggtcgaaggg 1080
ttttttcgag tcgaggggga gagatagcgt taagtcgggg tgtttgttta ggattcggtt 1140
ggggtttgtt ttttcggttg cgacgtcgga attttcggtt ttttttttta gtttgcgtcg 1200
tcgtagtttc ggaaatcgtt gaattatagt ttttttagtt aattttcgtt tagttttagt 1260
ttggtcgagg tacgtcgtta tgtataattt atgaaaggtt agttcgttcg gttatttaga 1320
ggttttagtg cgtgtgcggt atagtcgaag tttcgtttat tgattaatga ggagagtagt 1380
taggagaaag ggcggagtta agaggagatt gatacgtttt tgtgttggtt gtttcgatag 1440
ttcggtgttt tttttttcgt tatagcgagg gaagagtcga ggatttgggt atagaagttc 1500
ggggggaggg agggaagatg atcggaatgt tttgttgaaa atttagttga gtttttggta 1560
gtgcgtgacg ttaaggtatt ttaaatgttt ttgatatagt ttttttttgt tcgttttttt 1620
ttttttcggt ttttggtatt acgtggtggc gaggttgaga gttttttatt acgcgggaat 1680
tagttagtta gatagttttt aggtattttt agattttttg gggatttaat ttttgaattg 1740
ggttttgggg ataataaaaa gaatatgggt gatttcgttt gttttgaatt ttagtttttg 1800
cgtgagtagg gatatatata ttcggaaata tgacgtggaa atagagtaat tattagagtg 1860
cgtggggaga gggtagtgga tttaatttcg tatttcgttt tattatttta agtattaaac 1920
gtcgattagt tagtttattg gttcgtatag gcgaatgtag aagacggtgt atacgaaagt 1980
tgataacgtg tagggtttga tatttttatg cgtttgaatt gaaatatatg aaatgttttg 2040
tttttttttt ttttattttg tttatttggt atgtaagttt tattgaattg aagttagtgg 2100
ttgataggaa atgtagatat tttgtttttt ataatagagg atgaattttg tataatggag 2160
tgatttttgt gtatgtgttg atattattag ggtttttaat ttttattttt ttgggttttt 2220
aaaaatatgt tattaatatt tgtgtttgtt taatacgaat aggtataaag ataaaaattt 2280
aaaaaggatt tattatttta ttgtttagtt aattttgata tatttttttt taatgaattt 2340
aatatatgtt tttgtttaga aaatgtagat cgtttagaaa ggttaaaaat attaaatgaa 2400
aatttttttt tatttttatt attttttttt tttaaggata gttgttttta ttaattttta 2460
gtgagaaaaa ttaatgattt ttaatttttg aaggtagtta tagatttttt tgagtgtttg 2520
aaggagatta gattttagtt ttagaaataa tataaggttt tatttatatg gttttttaga 2580
tatttgaagt ttgtttaagt ttatagattt agatcgttcg tattatttta tatttaatga 2640
gggatagagg tatttatagg gtaatttata gggttattgg tggtgagaat tgagaatttt 2700
ttattgtttt ttggtagata tagtttagta tggaaattgt tgtatttatt tgtagagtgt 2760
gtgtgtattg attaggttgt ttatttatat tataggatag atatttttta ttttcgttta 2820
tagttttttg taattacgtt atttcgtgcg gttaattgtt tttttgataa tatagggttt 2880
aggatttgtt tagtgattta ttgtttataa agtatttata tattttatat attttttgtt 2940
tggttttttt tttataatta tgaatagtag ataggagagg ttatattatt cgttgataga 3000
aaattaaaga taatataata tgatattttt aagattatag agaaatttag aaatatatta 3060
ggtttagagt taggtttggg ggttttacgt tttatagttt tgttttattt atttaaattt 3120
tgttttgttt ttgatttttt aagagttggt ttttgttttt tttattggtt tttttattag 3180
taatatataa aatttatttt tgcgattatg ggtttgattt gtaaaaattt tttttagttt 3240
agatattggt ttttttttat aatttcgtta aatttttttt ggttaaatgt tgtaattttt 3300
gttttttggg ttattgtttt aattatttga ttattttttt tttattttat tttatttttt 3360
tattggttgt tagggatatt attttaaaaa ttaaataaat aaatatttag gttaaggcgg 3420
gtggattata aggttagttt aagattagtt tggttaatat agtgaaattt cgtttttatt 3480
aaaaatataa aaattaggtt gggtattgtg gtttacgttt gtaattttag tattttagga 3540
ggttgaggcg ggtagattat ttgaggttag gagttcgaga ttagtttgat taatatgatg 3600
aaattttgtt tttattaaaa atataaaaat tagttagtta tggtggtttg tatttgtaat 3660
ttttgttatt cgggaggttg agggaagaga attttttgaa ttcgggaggt gaaggttgta 3720
gtgagtcgag atcgcgttat tgtattttag tttggataat aagagcgaaa ttttatttta 3780
aaataaaagt aaaaattagt tagttatggt ggtacgtgtt tgtagtttta attatttagg 3840
aggttaaggt aggagaattg tttgaattta ggaggtggag gttgtagtga gtttagattt 3900
ttttattgta ttttagttta ggcgatagag taagattttg gtttaaaaaa aaaaaaaaaa 3960

aaaaatttaa ttatgttata gtcggtttaa aaatttttat ggttttttat tatcggtaag 4020
ataaaattta agttaatttt tttttttttt tttttttgag atagagtttt tttttgtcgt 4080
ttaggttgga atgcgatggt atgatttggg gttattgtaa ttttcgtttt ttaggtttaa 4140
gtaatttttt tgttttagtt ttttaagtag ttgggattat aggtgtttat tattatgttt 4200
agttaatttt tgtattttta gtagagacgg ggttttattg tgttggttag gttggttttg 4260
aatttttgat tttaagtgat tcgtttattt tagtttttta aagtgttggg agttatcgtg 4320
ttcggttaat ttaagttaaa ttttaaaaat gttgattagg tatagtggtt tatgtttgta 4380
atttaaaagt tttgggagat cgaggtagga agattatttg aggttaggag ttcgagatta 4440
gtttgggtaa tatggtaaaa tattaatttt ataaaaaatt agtcgggcgt ggtggtggta 4500
tgtttgtagt tttagttatt ttgaggattg aggtaggagg attatttgaa tttagaaaga 4560
agttcgaagt tatagtgagt tatgattgtt ttattgtatt gtagtttgag taatagaata 4620
agattaattt tgttttaaaa ataaataata aataaaaagt tgattattta atttagtgtt 4680
atggttgggc gtagtggttt atgtttgtaa ttttagtatt ttgggaggtc gaggcgggcg 4740
aattttgagg ttaggagttc gagattagtt tggttaatat gatgaaattt cgtttttatt 4800
aaaaatataa aaaaattagt tgggtatagt ggcgggtgtt tgtaatttta gttatttggg 4860
aggttgaggt aggagaatcg tttgaattcg ggaggtagag gttgtagtga gtcgagatta 4920
tgttattgta ttttagttta ggtaataaag tgagattttg ttttaaaaaa aaaaaaaaaa 4980
aagaaaaaaa tattataatt tagtgttatg tatgtggtag gagtttaatt gatgtttttt 5040
tttttttttt ttttatttga attataaaat tttataggtt tagtatagtg gtttacgttt 5100
gtaattttag tattttgaga ggtcgaagcg ggtggattat ttgaggttag gagtttaaga 5160
ttagtttgat taatatggtg aaagtttgtt tttattaaaa atataaaaat tagttaagcg 5220
tgttggcggg tatttgtaat tttaggtatt tagtagggtg aggtagaaga atcgtttgaa 5280
tttaggagat aaaggttgta gtgagttaag attgtattat tgtaatttag tttgggtaat 5340
atagtgagat tttgttttaa aaaaaagata aattttataa aggttgattt gaattttttt 5400
ttttttaatt tagttttata aattttatta aatgtgttat ttttttttaa atatgtttat 5460
attttttgta attttatgtt tttgtggata gttttttttt tatttggaat tttttttttt 5520
agtttttatt tgagaatttt tatttatttt ttaaagtttt gttgtaatat tatttttttt 5580
aggttttttt ttatttttat tggtaaagtt aattttttat tagatttttt cggtaggttg 5640
tttttttatt tagaatatat tttttatttt gaattttaat aaatttatat attggtttat 5700
tttttgaaaa aaaataataa ttaatattga ttaagtaatt attattatat tttaggaatt 5760
gtgttaagta ttttatatag attattttat ttgattttta ttagaattta gtgagggtaa 5820
tattataatt tttattttat aattgaggaa acgaagattg agaaagtgaa agattggttt 5880
aagattggtt ttatagttag aaattaggtg gaaatttaga atgaaaatta ggtttggttg 5940
attttttggt tacgtgttaa taaatatttt tttttaaatt gtgggttttt ggaaatttga 6000
attttttaag ttttgttttt ataattttag tatttattat ttagtttaga gtttatttaa 6060
gattgaatta attaaatatt aataaataag ttttgaggtt tgacgtggtg gtttattttg 6120
tagttttagt attttgggag gttgaggtag gcggattatt tgaggttagg agtttgaaat 6180
tagtttggtt aacgtggtga aatt 6204

<210> 285
<211> 9700
<212> DNA
<213> Artificial Sequence

<220>
<223> chemically treated genomic DNA (Homo sapiens)

<220>
<221> unsure
<222> (71)

<400> 285

tttcgtgggc gggtggggag gcggggacgg cgatttgttt ttattgtgtt taggtgcgcg 60
gggattatgg ngtttggacg tttttagtag tttagttttt tttttaattt tcgattttta 120
ttcgggagtg gagatttttt tagttcgaaa gtttcgaggg gaggttagtt atgggtcgag 180
tttgtcgatg ttttattatt ggagtgcgaa aatatattta tattcgtatc gtatttagta 240
tatttagtag ttagttagtt tcggttttcg ggttagggat tgatcgggaa agcgggttgg 300
ggttgttggg aaaagttgga gttgatggta agattgtcgc ggttataaat ttattatttt 360
ttaggttagt tttgtgcggc gttgggattg tagttggcgg agtgaatttt tgggttagcg 420
cgtatgcgtc ggtaggtagt aatttagttt tttatgatta ggtgagatgt tggtgagtag 480
gtaaaaaagg aggatttgtt taagcgattt tagggagttg gttcgatcga gttttttagt 540
ttatgttcgg cggggattag tcgttaggag cgtagagtcg cgatgtttat atttgttaaa 600
atgggggatt ggtaggtacg gggggtttgt tttggttggg gttgaggtgg gtaggggtgg 660
agtaggggag ggggtcggag cggggaggag gagaggaagc gtcgtggttt agggtagggg 720
agtcgtaggt gaggcgcgtt tggtagcgtt taggagagtt tttaggagat agcgaagata 780

ggaaaaacga gcgtaggtag cgattgcggt gttgggcgta aagcgcgtag ttcgcggggt 840
tcggcgtcgg ggaagttcgg cgtacgtttt ataggtggtt tggtgggtta tttgtggttt 900
gtgttgtggt cgtttttttt attttttttt tattttaagt agaaatttgg gaggatgatt 960
aattcggaag gggggaggga gaaggtttgg ggttgtggtg cgcggcggtg aaggttttta 1020
gtttgttgcg tttgcggttt cgttttagat tttagggttg agtgttggat aggttttggt 1080
gtatttgttt tttttggtat ttttagattt cgggagattt aggatgtgtg aagaatggaa 1140
aggggtttag atggttgggt cgcggaggta gcgtgttttt gtaggagata taaagtttgt 1200
tcgaagttta gttagtaatt tattttattt tttgaaatgg gtttttgtcg tttgggttac 1260
ggatgaggga gtgtgagaat ggtgggtgag gaggtagggg ggtggtgaat ttgttaaaat 1320
taacgtttgt ggaatttggt tttgttaggt cgcggatatt ataaggtatt taaggtttgg 1380
tttttgtttt ttttgagttt cggatttcga ttttggttaa ggaagtattt gtttagtttt 1440
ttttttttaa tttttagata tttttttttc gggaaataga gcggttttgg ttaggatggt 1500
ggcggtaaaa ggtttgtttt gtcgtcgatt ttattattag aatcgcgagg ttttgttttc 1560
gttttaatta agaggtttgg tgagggtgag attttgtgtg tttaaaagtt taggggataa 1620
ttcgaattta atgttttgaa ttttttttta aaaatttgag gttttgttat ttttggtcga 1680
gttttttttt ttgttttaga tttaggttat taaaagtaaa gttttgagaa gtgtagtttt 1740
tattaagtta tttggagttg gagaaagaaa ggttttttgc gttttgtatt tttttttttt 1800
ggatttatta tttttattaa gagtgagttt ttttatattt ttttttttag ttaagtttta 1860
ttaaatggta ttttattcgt aagtttagat aaatttcgtg gtttaaggat gtaaatttta 1920
tattagagat aaatttcggg aatttatatt tttttttttt tgattttgtg ggttaaaggt 1980
tttttttaat ttttttattt ggaggttgaa gattggggga gttttttcgg agtagttttt 2040
gggttttttt tttaaatttt agtgaatttc gttgtagaat ttttaggtcg tgagaaggtg 2100
tttgttgagt aggtttggtt tgggattggg ttttttgtgg gttcgattaa ggtttgaaag 2160
tcgcgagatt gtcgtaggag gaggaaggag ggagtcgcgg gtttggtttt tgcgggttta 2220
tagttaggta agtggttgga ggtttggatt tcgggtgggg cgagcggatt tggggttcgg 2280
aggggtcgaa tttgatgggt tgggtttgtt gggttttcgg tttttattgg gtattgataa 2340
tcggagaaaa gagagtcgtt ttggtttttt attgtttttt tttttttgtg attttgtttt 2400
ttatataaat attttttttt tttcgcgacg ttttttttat gttttttacg ttaggtttgg 2460
attttcgatt tttaatttta ggtttggcgt tgttgtttcg gggttttggt ttcgtcggag 2520
tattgatatg taatgaagcg atttagcgtg gtaatgacgt tggtataaat gtagttcggt 2580
tggttttaga ggcgattttg gcgttcgttc gggtttttta ttagcgttta tatcgggggc 2640
ggtagtttag agtcggtagg ttttaatttg tattttaatg gcgttttcgg cgggaatata 2700
ataatagttt agtagtttta gcgggtgcgg agtcggagat ttgttgggcg cggggtagcg 2760
gggaggtgga aacgggggtt gaaatggggt ggtagaggat ggagggcggt ttttttaagt 2820
tcgtagaggg taagaaagcg aaatttggag tttttttaag tatatcgcgg cgacgtttac 2880
ggagtgtaaa aatttatgtt tttaggttta gggtttggtt tgtttaggtt atggtcgttg 2940
ttgggagggt gtcggtgttt ttggtagttt agattttgat tttaaattat tgtattggcg 3000
ataggttggg atagttttgt agggtttagt tttggaaagt cgattatttt ttggacggag 3060
ggcggcgtta agtttttagt tcggtttagt cgaggaattg tgtttagttt aggtagggta 3120
agggaggttt agagtatggg gagggttttt tgggtttgta aggttttttg ttagtttcgg 3180
ttttttttta gtttttggga gttttttgtt tattttgtta ggtttgggga gtttgttgag 3240
gtgtttggag ggagaatttt ttttttgtag ttttgggtgg aatggggagt ttttttagtt 3300
tttgtttttt ttttatttgt gagggaatgt tttaatttgt tatagttttt attttttttt 3360
aggaacgttc ggtggaaata tgggaggttt ggagattttc gagaaataag tttagatgaa 3420
ttaaaggttt ttattagagt aattttgagg cgggatttag tgtagttaag gagtttgtat 3480
atatagtaga gagaaaagat agaggtaatt tttttttgga ggataaacga ggaagtttag 3540
ttaggaaaat taattatttt gtatttttta gtgttatatt aggttttttt tttttttttt 3600
ttttttttta tttaaaaaaa aattttaaaa acgaaaggga taaaagtttt agaggattta 3660
gtaggtttcg gtgaggagtt taaaagttcg gattgattag tttggacgag taggtggttt 3720
ttaggttttt aggtttcgcg gtagtcgggt tttgtttcgc gagtacgttc gcgttagtag 3780
tattattaaa ttatgaagga attttttttg ttttaattaa aaaggggaat tttgggaaaa 3840
ttagagtaga ttaatatagt taaaagtcgg ttttcggtga tttattttga tgtacgtacg 3900
agacggtatt tagatttgta ggtatatata cgtttgtttt taggtttaat tttagaagcg 3960
ggtgtgtagt tttataggag ttttgtattc gtgtttacgt tgtatttagg aaattttagt 4020
ttgaatatta atttgaattg ttagttggtt ttagttttat ataaatagga gaaaaattgt 4080
ttaattttg gagttgttaa gtaatttttt tagggtataa tttttatttt atacgttgta 4140
attaagggat tgtaaatttt agtagagtta tcggagtcgt ttggtagaat ttttaatttg 4200
ggggaaagtt taaagttaat aaaattttta aggcgttgcg gttcgagggg gtggttgttt 4260
cgagcggagg cgattttttg ttgggaaaaa attttggagt gatttcgtcg gttttggttt 4320
ggtggttatg tttaattgcg aaagggataa tgatttttaa taaaattgta cggggttaaa 4380
agggtagttc ggatcgtggt agtagggtta agggagaagt gttagaagtt gggttttagg 4440
cgtggcgttt ggattttgag tagtgtcggg ttttgtagaa ggtatttttg gagaaaagga 4500
agcggagaaa gaaaggaagg ggaatgtggc ggaattgggg taagagaggg ggtgttgggg 4560
tagggtttcg cggcggagtt cgaggaaggt tttattagtt cgtaggttgg agtgcgtttt 4620
agaagtaagt tcgcggcgta gagaggtttt tatatttgtg tcggagcggt cggcggtaga 4680
ttgtggtatt ggcgagtaat tggattattg ttgatatgtt aatgaggcga ttaggttgtt 4740

ggtaaagagt tggaaaaggg aaaagttttt attattagag ggagattttc gagcgtatac 4800
gggagttttt tttttttttt tttttttttc gttttttttt tcgttttttt tttttttttc 4860
gttttttttt tttttttttt tttttttgtt tttttttttt tttttttttt tttttttttt 4920
tatttttttt tttttttttt tttttttttg cgtttatcgt cggtagttag tattttgcgt 4980
ttatttagag agtagtttta tttgggtgcg agatcgagag gggtatattc gtttacgtcg 5040
atttatgaaa atgttttgga aattgacgga taatattaag tacgaggatt gcgaggtaag 5100
cgcgacgtcg gttcgattat tcgtgcggtt ataggtttcg agtttgattt tttttttttt 5160
tttttttttt ttgtaattag ttgcgaaacg cgggtgggat aaattttttt tagcgtagcg 5220
ttcgtttttc ggattcgttc gaagaacgcg ggggagaagt tggagaggga ggacgtggat 5280
gttttttttt ttggtttttt tattttgaag ttcgtgtttt attttatttt tttttttgat 5340
taattttggt tgtgtttaag aatgggggaa aagaagaata tgggtttttg aagtaattgt 5400
tggtaattgg gtatttgttt gtgtattttt cgtgttattt ttatcgagat tgagggttta 5460
agttattagg aatagaaagg aaagagaaaa tttttttttt ttttgaattt ttttttcgat 5520
ttttaatatt tttttttttt tttatttatt tatttattta tttatttatt tttttttttt 5580
tttattttta tttttaaagc ggtttttttt tcggttttag agacggtttt tttatatttc 5640
gttgtgagtt gggttgtatt tttttgtaag cgggtttaat ttaagattat tgattttagt 5700
tgcggggttc gttttagatt ttttgtgttt tacgaagttt taagtagttt ttttttttga 5760
gttataggat cgacgtattt tacgggggtg tgtgggggtg gggtggggag atgtgttgcg 5820
tcgagtttgt tggtcgttgg gtgcgggagt attagttttt gtattagttt agaacgatcg 5880
tgggaatttt tgcgcggttg tagattattt ttggtgtcgt aagttcgttg ggtcgataga 5940
gcgcgtcgtt ttggaattta ttttaaaacg tagagttttt tgagttcgtt tttttgaggt 6000
agtaagggag attttagcgc gatttgttta ttcgtaggag gcgttgggaa cggttataat 6060
taatattgta atgagtaatg agttcgagag aggggttggg gtgtgtaggg ttggagatta 6120
tttaaataaa taggtggtag ttttaatttt tgttgttttt tttttttttt ttttttggta 6180
tttgtagttg agaatgttag gtaggaggtg tgtgtgtagg cgtaggggcg gagattttga 6240
ttttattcga aattagaaat tgggtttatg tgaacgatgt gttattcgtt tttttttttt 6300
tttttttttt gaagattatt ttaaatcgtt tttttttag gtttttattt tcggtagatg 6360
gggacggggt gatattttac gggtattggt ttttttttgg ggggtttttg tcgcgttaga 6420
ggaggggcgt agggatttta cgttagatta gtcgttttgt tttcgaggtt gggggcgagt 6480
tttgcgggta ggtaggaatg aagtcgaatt ttttagtttt ataggataat ttatattgta 6540
ttggtttggg ttttggtcgg aggtttgggt attttatttt cgtagaggtc gaaatttagt 6600
tttaggtgtg ggcgttttcg gcgggcgggg ttcgaatttt cgtgcggttt taggtgtcga 6660
ttggatgggg tgggtggtta ggtgggggat gaatttgtat ttttagaggg attttgtggg 6720
ggtaagtgtg aatgcgtgtg taagcgaggt aaaaaaagat gagagagaga ggagaataaa 6780
gttaaagttg tttttataat tttggtcgtg gtgggtaagc gtcggagcgg ggatttcggg 6840
tttcggagtt ggtttggggt agtttgattg tgtcggggtt ttgtagggat acgtaggagg 6900
cgaggtgtcg tttgttttga taatttggcg ggtagattgg taaaggtagt acgtcgggag 6960
agttagtcgt gttatttggg ggggcgttag gcgggagtgg tttagttagt cggacggttt 7020
tttgtcggga gtcgcgagtc ggtgttagcg gcgttcgggt tgggtcgttt agcgtttgtg 7080
cgcgtttcgg ttcgtatttc ggttttcgat tcggaatttt cggttttaga gagtcgaggg 7140
tagagcgacg gtggtcgggg agcgtacggt gtttttgttc gaggaaagtt taagaggagg 7200
tcgaggagag gattagtgta gcgagcgggt cggtttcggg tagggcgtcg ggttatggag 7260
gagtcgcgta gtagcgggta gtcgagtttc gagttttaga ttcggttgcg aatcggcgga 7320
gttcgcggtt agatagggtc gggaagcgtt tgtcgcgtcg agagttttac gagcgttcgg 7380
acgcggtttt aagtagtttt tattcgttac gtacgatttt cgttgtattt cgaggtttgt 7440
tttagttttt taggtttgat tgttttcgtt cgtttttttt tttttttttt ttttttcgcg 7500
tttttttttt tcgtgcgttt cggttgggtt tttttttttt tttttttttt tttttttttt 7560
tttttttttt tttttttttt tttttcgggt tttttttttt tttttttttt tttttttttt 7620
attcggggtt tttttttttc gtttatattt ttttttttat atacgtacgt agatatattt 7680
tttttttttg cgtttttttt ttcggttttt ttttttgttt tttttttttt tatttttgta 7740
tgttttgtaa ttttttaaaa tgttgttttt tttttgtgat tcgttagacg tcgcgtcgcg 7800
gtttttcgcg cgttcgttcg tttttttttt cgttcgtttt ttgtttttcg cgtttttttt 7860
ttttatttcg atttgttata ttgagatttt cgttaatgga ttgtattgag agtcggtttc 7920
ggcgcgagtt gttttttcgt tttacgttcg atttttaggt attttttttt tattaagtat 7980
tcgtgtaata ttaatagtta tgaatatttg ttattaggag gttttaggaa cgttgtttag 8040
cgcggttatt agaagtttaa gcgaagtcgt cgttaagaaa agagggggag atacggatta 8100
aggaatacgt acgtatttat atatttatat atattttttt tttttttttt ttttggggtt 8160
ttttattttt aaagaatttt gaattataat tagtcgcggt aggatagaga tcgtgggttc 8220
gattagttga aggcgtcgcg cgaatcggtg gtttaagttc ggatggattc gagtcgggtt 8280
ttcgcgtttc ggggagcgtc ggcgtcgtga ttggagtttt gggtggcgcg gggttttcgg 8340
cgttttttgt gtggggcgcg ttcgggcggc ggggtagtcg ggcgttttag gtttgtttgt 8400
ttttttttat tttgattcgt tattttagat ttttcgtaga tgttagttta tagtttttta 8460
gttatggtga gttttatttt tattttcgtt gttgtttttt taggaattgg attttggaaa 8520
tttggtgtcg tggagttgtt cgaaatttcg ggttttacgt ttagtttttt tttaggatat 8580
tttttttatt tgtatttttt attttatttt tcgttttttt ttcgtttttt tttattttta 8640
aatcgagtgt attatttttt attttagttt tcgttcgatt ttttcgagta cgtagattta 8700

tttcgtcgcg gttttttaag gcgtgttttt ttttacgttt attttgattt ttttgagttt 8760
ttatttgttt tcgagatgtt tttaatattt gatgtttaaa ttgtttgtag gaaaagtgta 8820
gcgcggtgtg tttttattt tcgggatttg cggtcgaatc gtcgagggtt cgcggttttt 8880
ggaattaatt tttttcgttt gttttgtaag ggaattgtaa tagagaaatt taaagttagg 8940
cgtttttttt gttttttggg gttggggtgg aaggggcggg ggggggggtt atagtttcgg 9000
ggtgtatttt ttttttttaa atttaataag gttgaatttt tttattttaa attttgattt 9060
gacgtttttt tttttttatt taaattataa atcgattttg tcgagaaaaa ggtttttaat 9120
agaggaagcg aaaaagatag atttcgattt tgtagttaaa atttttttta agttggtaat 9180
ttaaacgtgt gtttttgtta tagttttttt gcgaaatatg tttttattta agtagttatg 9240
aaacggtaat agtttttgaa cgtttatgat tttttttttt ttaaataaat ggaagagaat 9300
aaataataac gtagtggtag ataggtttaa atcgggggaa aagaaaaggt cgtttttgtt 9360
gtttagtatt taggaggaat tttaattttg tttaagagag attttacgtt gtagtttaat 9420
gggttgtgtt ttaggagatt tttttttatt tttttttttt tttgaaagaa atttttaaat 9480
agttttttgt gtttttttta tatatagttt ttttgcgttg ttcggtttgt tttatttgag 9540
ggtatttttt gaaaagaatt tagagttgat tttttttcgg cggttagtta acgggaacgg 9600
gcgatttttt acgtagattt gttaagtgga ttttgtgtgg atagttgtag ttcgacgttt 9660
gattttttgt tttttatttt ttttttcgtt tttttttagg 9700

<210> 286
<211> 9700
<212> DNA
<213> Artificial Sequence

<220>
<223> chemically treated genomic DNA (Homo sapiens)

<220>
<221> unsure
<222> (9630)

<400> 286

tttagaagag agcgagagga aaggtaaaga ataaggaatt aggcgtcgag ttataattat 60
ttatataaaa tttatttgat aagtttgcgt gggaaatcgt tcgttttcgt tggttggtcg 120
tcgggaaaaa attagtttta agtttttttt aggaaatatt tttaagtgaa atagatcgaa 180
taacgtagag aaattgtata tggagggggt ataagagatt atttaggggt tttttttaaa 240
gagaggaaaa gatagaaaag ggttttttgg aatatagttt attaaattgt agcgtgaaat 300
tttttttaag taaaattaaa atttttttta aatattaaat agtagaagcg gttttttttt 360
ttttttcgat ttgaatttgt ttgttattgc gttattgttt attttttttt atttatttgg 420
agagaaaaaa attataaacg tttaaagatt gttatcgttt tatgattatt taaatagaaa 480
tatatttcgt agaggggttg taataaaaat atacgtttaa attattagtt taaagaaggt 540
tttaattgta ggatcggggt ttgttttttt cgtttttttt gttaggagtt tttttttcga 600
tagagtcgat ttatggtttg gatagggaaa gggggacgtt aagttaaggt ttaaaataga 660
aagatttagt tttattgggt ttaaaagaga gaggtgtatt tcggggttgt ggtttttttt 720
ttcgtttttt ttattttagt tttaaagggt agaagaaacg tttgatttta ggttttttta 780
ttgtaatttt tttgtaaaat aagcgaagag agttgatttt agagatcgcg aattttcggc 840
ggttcggtcg taggtttcga gggtaggggg tatatcgcgt tgtatttttt ttgtagatag 900
tttaagtatt aaatgttgag aatatttcgg ggataagtga agatttaagg aaattaagat 960
gaacgtgggg agaaatacgt tttgggaagt cgcggcgagg taggtttgcg tgttcggaga 1020
ggtcgagcgg gggttggggt agggggtaat gtattcgatt tagggatgga aaagagcgag 1080
agaaaggcgg aaggtggggt gggggatgta aatggagagg gtgttttgaa ggaaagttgg 1140
gcgtgaaatt cgaggtttcg ggtagtttta cggtattagg tttttagaat ttaattttta 1200
aagaaatagt aacgggggtg gggatgggat ttattatggt tgaaaaattg tgaattaata 1260
tttgcgaaga gtttggggta acgagttagg gtggaaaaaa ataagtaagt ttggagcgtt 1320
cggttgtttc gtcgttcgag cgcgttttat ataaaaggcg tcgagagttt cgcgttattt 1380
aggattttag ttacggcgtc gacgtttttc ggagcgcggg agttcggttc ggatttattc 1440
gaatttgaat tatcgattcg cgcggcgttt ttagttggtc gaatttacgg tttttatttt 1500
gtcgcgattg gttatgattt aaagtttttt aaaaatgaaa aattttaaaa aaggaaaaag 1560
gaaaaagtat gtgtgagtgt gtgggtgcgt gcgtgttttt taattcgtgt tttttttttt 1620
ttttttagcg gcggtttcgt ttgagttttt aataatcgcg ttgggtagcg tttttggagt 1680
tttttaatag tagatattta tgattattaa tattatacga atatttaata agggaagaat 1740
gtttggaaat cgagcgtgaa gcggagaggt aattcgcgtc ggagtcggtt tttaatgtag 1800
tttattgacg ggagttttag tgtagtaaat cggagtgggg agagggagcg cgagagataa 1860
aaagcgagcg agagagggag cgggcgagcg cgcggggggt cgcggcgcgg cgtttggcga 1920
attataggga aggggtaata ttttaaaagg ttgtagggta tgtaaaagtg aaagagaaag 1980
aggtagagag ggagatcgaa ggagagagcg tagagaggga gaatgtgttt gcgtgcgtgt 2040

gtgagaaaga aagtgtgggc gagggagagg agtttcgggt ggggagaggg gagggaggga 2100
aggagggaga gttcgagaag aggaagggag ggaaagaggg agggaggaag ggagggaggg 2160
agggggaggg ggtttagtcg gagcgtacgg agggggaggg cgcgggggag ggggaagggg 2220
agaggaagcg agcgaggata attagattta aaaggttggg gtagatttcg ggatgtagcg 2280
ggggtcgtgc gtagcgggta ggagttgttt gggatcgcgt tcgggcgttc gtggggtttt 2340
cggcgcggta ggcgtttttc ggttttgttt ggtcgcgggt ttcgtcggtt cgtagtcgag 2400
tttgggattc gggattcggt tgttcgttgt tgcgcggttt ttttatgatt cggcgttttg 2460
ttcgggatcg gttcgttcgt tgtattggtt tttttttcgg tttttttttg agtttttttc 2520
ggataggggt atcgtgcgtt tttcggttat cgtcgttttg ttttcggttt tttggggtcg 2580
gggatttcgg atcgggagtc gggatgcggg tcggggcgcg tatagacgtt aggcggttta 2640
gttcgggcgt cgttggtatc ggttcgcggt tttcggtaaa aattcgttcg attggttggg 2700
ttattttcgt ttgacgtttt tttagatgat acgattagtt ttttcggcgt gttgtttttg 2760
ttagtttatt cgttaggttg ttagaataag cggtatttcg ttttttgcgt gtttttatag 2820
agtttcggta taattagatt gttttaagtt agtttcgaaa ttcgaaattt tcgtttcggc 2880
gtttgtttat tacggttaaa gttgtggaaa tagttttgat tttatttttt tttttttttt 2940
attttttttt gtttcgttta tatacgtatt tatatttgtt tttataggat ttttttaggg 3000
gtgtaagttt attttttatt tagttattta ttttatttag tcggtattta gagtcgtacg 3060
gaggttcgga tttcgttcgt cgagggcgtt tatatttgga gttgggtttc ggtttttgcg 3120
aaagtgaaat gtttaagttt tcggttaaag tttagattag tatagtatga attgttttat 3180
gagattgagg ggttcggttt tatttttatt tgttcgtaaa gttcgttttt agtttcgaaa 3240
ataaagcgat tggtttgacg tggggttttt gcgttttttt tttagcgcga taggattttt 3300
ttagggaaga gttagtattc gtgggatgtt atttcgtttt tatttatcgg ggtggggggt 3360
ttgaaaggag aacgatttaa aataattttt agaaagaaaa gggaggaggg agcgggtgat 3420
atatcgttta tataaattta atttttggtt tcgagtgaag ttaagatttt cgtttttgcg 3480
tttgtatata tattttttgt ttaatatttt tagttgtaaa tgttaaaaaa gaaaaaaaaa 3540
aaaaatagta aaagttggga ttgttattta tttatttagg taatttttag ttttatatat 3600
tttaattttt ttttcggatt tattgtttat tatagtgttg gttgtaatcg tttttagcgt 3660
tttttgcggg tggataggtc gcgttagggt tttttttgtt gttttaagaa ggcgagttta 3720
ggggattttg cgttttaaaa tgggttttaa agcggcgcgt tttgtcggtt tagcgggttt 3780
gcggtattag aggtggtttg tagtcgcgta aggattttta cggtcgtttt gggttggtgt 3840
agaagttggt gttttcgtat ttagcggtta gtaggttcgg cgtagtatat ttttttattt 3900
tatttttata tattttcgtg gggtgcgtcg attttgtagt ttagagggag aagttattta 3960
gaatttcgtg aagtatagag gatttggagc gagtttcgta gttgggatta gtggttttaa 4020
gttgaattcg tttataggaa gatgtagttt agtttatagc gaagtgtggg ggggtcgttt 4080
ttaaagtcga gaagggaatc gttttagaag tgggggtggg gggaagaaag aatgaatgaa 4140
tgaatgaatg aatgaataga aagaagaagg aatattaaag atcggagagg aagtttaaga 4200
gagaaaagag tttttttttt tttttttgtt tttggtgatt tgggttttta atttcgatga 4260
agataatacg aggagtgtat aggtaagtgt ttagttgtta gtaattgttt taaaagttta 4320
tgtttttttt tttttttatt tttaggtata gttaaaattg gttagaaagg ggagtaaagt 4380
gggatacgag ttttaaagta gggaaattaa agaagggagt atttacgttt ttttttttta 4440
gttttttttt cgcgtttttc gggcgaattc gagggacggg cgttgcgttg gggaaagttt 4500
gttttattcg cgtttcgtag ttggttgtaa ggagaggagg aggagaggga gggttaagtt 4560
cggagtttgt gatcgtacgg atgatcgagt cggcgtcgcg tttatttcgt agttttcgta 4620
tttgatatta ttcgttaatt tttaaagtat ttttatggat cggcgtgaac ggatatgttt 4680
ttttcggttt cgtatttaag tggagttatt ttttgggtga gcgtaaagtg ttggttgtcg 4740
gcggtgagcg taggaggagg aggaggaggg agaggaggtg gaggaggaga aggaggaggg 4800
agaggaggag ggtaaggagg aggaggagga agaggagggc gaggaggagg aggagggcga 4860
ggagaagggc gaggaggagg agagaaggga aagagttttc gtgtgcgttc ggagattttt 4920
ttttaatggt agaaattttt ttttttttta gttttttatt aatagtttaa tcgttttatt 4980
agtatattaa taatagttta attgttcgtt agtattataa tttgtcgtcg gtcgtttcga 5040
tataggtata aaggtttttt tacgtcgcga atttgttttt agagcgtatt ttagtttgcg 5100
aattgataaa atttttttcg agtttcgtcg cgaggttttg ttttaatatt ttttttttta 5160
ttttaatttc gttatatttt tttttttttt ttttttcgtt tttttttttt tagagatgtt 5220
ttttgtaaag ttcggtattg tttaaggttt aagcgttacg tttggggttt agtttttagt 5280
attttttttt taattttgtt attacgattc gagttgtttt tttggtttcg tgtaatttta 5340
ttagaaatta ttattttttt cgtaattaaa tataattatt aaattaagat cggcgaagtt 5400
attttaggat ttttttttaa taagggatcg ttttcgttcg aggtagttat tttttcgggt 5460
cgtagcgttt tgggagtttt attggttttg agtttttttt taggttggag attttgttaa 5520
gcggtttcgg tggttttgtt ggggtttgta attttttaat tgtagcgtgt aaaataaaag 5580
ttgtattttg aagaggttat ttgatagttt taggggttaa ataatttttt ttttgtttgt 5640
gtgaagttag ggttaattga tagtttaaat tggtgtttag gttaaggttt tttgggtgta 5700
acgtggatac gaatatagaa tttttatgga attgtatatt cgtttttgaa gttgagttta 5760
aaaatagacg tgtgtgtgtt tgtaagttta gatatcgttt cgtgcgtgta ttaaaataaa 5820
ttatcgggaa tcgattttta gttgtgttaa tttgttttag tttttttaag attttttttt 5880
ttaattaaag tagggagagt ttttttatga tttggtgatg ttattaacgc gggcgtgttc 5940
gcgaggtaga gttcggttgt cgcggaattt ggaggtttgg gagttattta ttcgtttaga 6000

ttggttagtt cgggtttttg gattttttat cggggtttgt tgggtttttt ggggttttta 6060
tttttttcgt ttttgggatt tttttttaag taagaaaaaa aaaaaaaaag aaaaggattt 6120
aatgtgatat taggaaatat agagtaatta atttttttgg ttgagttttt tcgtttgttt 6180
tttaaaagaa agttattttt gttttttttt tttgttatat gtataaattt tttaattata 6240
ttaggtttcg ttttagagtt gttttagtaa ggatttttga tttatttggg tttgtttttc 6300
gaaaattttt aggtttttta tatttttatc gggcgttttt aaaaggggat gggaattgtg 6360
gtagattgga gtattttttt ataggtggaa ggaaggtagg agttgggaga attttttatt 6420
ttatttaagg ttgtagaagg ggaatttttt ttttaaatat tttagtaaat tttttaaatt 6480
tagtagagta ggtagaaggt tttaaaaagt tagggagaag tcggagttgg taaggggttt 6540
tatagattta ggaagttttt tttatgtttt gggttttttt tgttttattt gaattgagta 6600
taatttttcg gttgaatcgg gttggaggtt tggcgtcgtt tttcgtttag aaaatggtcg 6660
attttttagg gttagatttt gtagggttgt tttagtttgt cgttagtgta gtggtttgaa 6720
gttaggattt gggttgttag gagtatcgat atttttttag tagcggttat agtttgagta 6780
ggttaaattt taggtttgag agtatgggtt tttatatttc gtagacgtcg tcgcggtgtg 6840
tttaagggaa ttttaagttt cgtttttttg ttttttgcgg gtttggaaag gtcgtttttt 6900
attttttgtt attttatttt aattttcgtt tttatttttt cgttgtttcg cgtttaatag 6960
attttcggtt tcgtattcgt tggggttgtt aaattgttgt tgtattttcg tcgagggcgt 7020
tattgaggtg tagattggga tttgtcggtt ttggattgtc gttttcggtg taggcgttga 7080
tgaaaggttc gggcgagcgt tagggtcgtt tttggagtta gtcgagttgt atttatgtta 7140
gcgttattat tacgttaagt cgttttattg tatgttaatg tttcggcggg gttagaattt 7200
cgggatagta gcgttaggtt tggggttgga ggtcggagat ttaggtttga cgtggggggt 7260
ataaaaaggg cgtcgcgggg aagagggagt atttgtgtga gagatagagt tataaagaga 7320
aagagatagt gaggggttag aacgattttt tttttttcga ttgttaatgt ttagtgggag 7380
tcgggagttt aataggttta gtttattaga ttcggttttt tcgggtttta aattcgttcg 7440
ttttattcga gatttaggtt tttagttatt tgtttaattg tgagttcgta agagttaggt 7500
tcgcggtttt tttttttttt tttttgcggt agtttcgcgg tttttaaatt ttagtcgaat 7560
ttatagaagg tttagtttta ggttaaattt atttaatagg tattttttta cggtttagga 7620
attttgtagc gaaatttatt ggaatttgag gagaaaattt aaagattgtt tcgaaaggat 7680
ttttttagtt tttagttttt aaataagaga gttaggaagg gtttttagtt tatagggtta 7740
ggaagggaaa ggtgtggatt ttcgggattt gtttttggta tgggatttgt atttttaaat 7800
tacgaggttt gtttaaattt gcggatgaaa tattatttag tgaggtttaa ttagggagaa 7860
gaatataaaa gagtttattt ttgataaaag taataaattt aagggaaaaa agtatagaac 7920
gtaaaaggtt tttttttttt taattttagg tagtttagtg aagattgtat tttttagaat 7980
tttattttta atggtttaaa tttgaaataa gagaaagaat tcgattagga gtagtaaggt 8040
tttagatttt tggggggaag tttagggtat tagattcgaa ttgttttttg ggtttttggg 8100
tatatagaat tttattttta ttaggttttt tggttggggc gaaaataggg tttcgcggtt 8160
ttgatgatga aatcggcgat aggtagagtt ttttatcgtt attattttgg ttagagtcgt 8220
tttgtttttc gaagggaaag tgtttggaag ttggaaggag agagttggat aaatgttttt 8280
ttggttaggg tcggaattcg gggtttagga aggataggag ttaagttttg gatgttttgt 8340
ggtgttcgcg gtttagtagg gttaagtttt ataggcgttg gttttggtaa atttattatt 8400
tttttgtttt tttatttatt atttttatat ttttttattc gtagtttagg cggtaaaaat 8460
ttattttagg aagtggagtg agttgttagt taaatttcgg gtaaattttg tattttttat 8520
agaaatacgt tgttttcgcg atttagttat ttggattttt ttttattttt tatatatttt 8580
agatttttcg aaatttgggg gtattaagag agataggtgt attagaattt gtttaatatt 8640
taattttggg gtttagggcg aggtcgtagg cgtagtaggt tgaaaatttt tatcgtcgcg 8700
tattataatt ttaggttttt tttttttttt ttttcgaatt agttattttt ttaagttttt 8760
atttgaggtg ggaggggggt gggggaaacg gttatagtat aaattataag tgatttatta 8820
ggttatttgt ggagcgtgcg tcggattttt tcggcgtcgg gtttcgcggg ttgcgcgttt 8880
tgcgtttagt atcgtagtcg ttgtttgcgt tcgttttttt tgttttcgtt gttttttggg 8940
agtttttttg ggcgttgtta ggcgcgtttt atttacgatt tttttgtttt gggttacggc 9000
gttttttttt ttttttttcg tttcggtttt tttttttatt ttatttttgt ttattttaat 9060
tttagttaag gtaaattttt cgtatttgtt agttttttat tttggtaagt atggatatcg 9120
cggttttgcg tttttggcga ttggttttcg tcgggtatgg gttggagggt tcggtcgggt 9180
tagttttttg gaatcgttta ggtaaatttt ttttttttat ttgtttatta atattttatt 9240
tggttataaa gagttgggtt gttatttgtc gacgtatgcg cgttggttta ggaatttatt 9300
tcgttagttg tagttttagc gtcgtataga gttgatttgg gaagtgatgg atttatagtc 9360
gcggtaattt tgttattagt tttagttttt tttaatagtt ttaattcgtt ttttcggtta 9420
gtttttggtt cgggaatcga agttgattga ttgttggatg tgttgggtgc gatgcgagtg 9480
tgagtgtgtt ttcgtatttt agtggtgggg tatcgataag ttcgatttat ggttagtttt 9540
ttttcggaat tttcgaattg ggggagtttt tattttcggg tgggggtcgg gggttggggg 9600
aagggttaga ttgttagaag cgtttagatn ttatgatttt cgcgtatttg gatatagtgg 9660
ggataggtcg tcgttttcgt ttttttattc gtttacgagg 9700

<210> 287
<211> 6550
<212> DNA

<213> Artificial Sequence

<220>
<223> chemically treated genomic DNA (Homo sapiens)

<400> 287

gaaggttgaa ttttttaatg taggtgtaag tttggttgtt gtgattagga agttgagtta 60
agttaaatat atggtttgtg ttattttgtt ttttgagtag gttttttaag gttggtgtag 120
agttaatgta ggttgggtag taattttttt tgaaattatt ttgggtaggg tatttttaga 180
ggtagtagat tgtgtagtaa agtttttttt attttttttg ggtaggatag tgtttagtat 240
agttttagaa tagtagatat tttagtagga tagaggtttt gtattaatta tagtttttgg 300
aagatgaata ttggttatga atgttaagta tatttatttt ttttaaagta tttattgttt 360
ttggaattta agttattttt aaattgagga ttatatttaa aattttgtaa taaaatttag 420
aatttaagat ttgtgtttat ttttgtattt tttttgtttt tgatttagaa tttaattaaa 480
gttaaaaata taattttatt aaattatttg tttaatgaaa attttagttt tatttttaat 540
attaattttt ttttttaaag agaaagtata aattggtttt taaagagatg agttatattt 600
tttttttata gagagataat atataattaa gagaatatat ataggttttg agttttagta 660
aaggtggtat tggtttggtt tttagttgtg aatggaaatt aaggtggtag gagatttagg 720
gtatagggtt tttgatttta gggtagtaga tttttttggt tattaggaaa gttatttttt 780
tttatagatt ttgatgttga atgaagataa agtttaattt gaagtatggt ttagttttag 840
agtgttttga tataggaaag gggtttgaag gtgtagaaat taagtttttt attgtattgg 900
aggagaaaga gaagtttttt tatttttttg taatgtttgt attttgggtg atatttatta 960
aaggggatta gtgttttgtt ttttttattt ttttattttt atgataagat agaatattga 1020
atgttgttat tcggaaaaag tgaaaaggta atttttgata tgtggagaga tttgtaaagt 1080
aaattattta ataatatttg atttaatttt tataatattt ttgatatata ggtaaaggga 1140
gtattattat tttatttatg aatgaaggta ttaaattttt tttttgtgag ttgtttattt 1200
ttttttttat gtgaagataa atgggatatt gtttaagtgt tttagttaat taaagtttta 1260
aggatttgtt tagaattagg tttattgtgt ggtaagggag ttaagaaagt agggtttagt 1320
ggttttgttg aagttttttg tatgtggagg ttatagaata tttttatagt ttattttatt 1380
tgttattttt tatgtttaaa aatcgggtta ttatttttat tattttaata tgttagtatt 1440
attattattt tttaaaaatg ataggaatga tttattttta ggtgttggga tagtgagata 1500
gagtggtatt ggatttttat tggttggagt gatgattata gagtggtttt aggaagttaa 1560
gtgttgaggt tttttttgtt atatattttt ttttgggtgg gttgtgtgag tatgattttt 1620
ttattaattt tttttttttt agatttgggt tagagtgttt tatatatata ttattgaagt 1680
tttataattt ttggaggtag atattataat tattggtata ggtgaaatag agatttttag 1740
agtgttttag ggttatatag tttttaggta aaggggttta gatattaagt taaattagtt 1800
tgttttattt ggaattttat gggtttagga gagtagaata tgtggttagt ttttttaggt 1860
cgggaggagg gattataatt taggttatat agttagaggt aggattttaa ttttggtttt 1920
tgagtttttt attttttgtt ttatattggt gaaaataatt agaaagttaa tattaatgga 1980
aaattttttt ggtagaattt ttaaggatag aaatgtaaat ttttaaagaa aaattataga 2040
ttgttttatt ttagggttgg aggagaaagt ggagatgata gtgttgaggt gtaaatttta 2100
ggtttatttg agtgggggga ggagaacgtt tcggtttttg gaaggttttg taaagtatgt 2160
aaggaagaaa atttagtata ttagggtgag aagagtgagg ttaggtttag ggttggtata 2220
gggagaatag agataaatgt tttggcggtt ttggttagaa ggattttagt aacgattgtt 2280
tttttttttg gtttgaggtt aatgagtaag gttattataa gataaggttt ttttattata 2340
atgtattttt attttatttg ggtatttatt ttatggtgtg tgtagggggg aggaggagag 2400
gaggttttgt tttttatttt tttggtattg agatttttgt gtatgtattt tggaaaagga 2460
aatacgtgtt ggttattagt aaatttttta agttgttaga tataagtttt aaaattagtt 2520
attgtatatt ttttgtgtgt agattagaaa attgaggtta gtagaggtag agggatttgt 2580
ttaaggttat aaggtattat tgtggtagag tttagattcg ggatttttgt gaagtgagtt 2640
taagttagta gggagtttgg ggtttatgtt ttagtttaat gtaaaagaaa atatatttag 2700
atgtttttat atttgcgata ttttttttta tttttaatat ttaattattt ggttgaaata 2760
gagtgaatgg ggtattagga tatagttggg ttatgtggga ggtgggtaag gagtgtttga 2820
ataattttta tttttgtaag tggtttgagg tttattttgg ttgggaggtg tagggtgttt 2880
aggttgtgtt gtgattttgt ttttgggtgt ttatttttta tatttttaga gattttttag 2940
gttttttttt tggttaggga tgtggagaaa ggtttgaggt gtgaatttta cggggtttag 3000
ttagttttgt gaggttatta agttataggt tattatgggg agaaggggtt gtgtggttgg 3060
gaaaggttat agaattgatt tttgttggta cgatttggtt ttatattttt gaagtttatt 3120
tattttgtaa agatgaaatt gaggtagaga agagaggtga tttatttcgg gagtcgttcg 3180
ttattattgt ttattattat tattattgtt ttagaaatat ttagttagtt tatatattaa 3240
gagtagtttt ttgggttatt tttttggttt tttagaaaga tttattttta taaaggggag 3300
tttaattaga attaattatt cggataattt attataatta tttttgtatt tagatttcgg 3360
attattttta ttggaaaatt aaatggatga ttgtttttta tttcgttttg tttatgtaga 3420
taagatttga gatatgtttt agtttttttt cggattagat ttttaaagag atggggttta 3480
ttgtttggtt tgtttttttc gtagcgtcgc gtttttagac gttttgaagt tggacggggt 3540

tttggttaga gagcgttagg gtcgaaggtt taggaaggac gtttgggtag gcgttgttta 3600
agcggtcgag cgacggttgt aagcgttttt tttttttttt cggcgttagt acgcggtttc 3660
gtcgggtttc gtttttagtc gtttgttatc gggtcgtcgg gtatacgttt ggggcgggat 3720
ggtggttgcg ttaaggtcgt cgaggtgttc gcggcggcgt cgaagaatta ggaattttta 3780
ttagtgtttt aagcgttatt atttaatttt cgtttcgtat tttttttttt cgagggtttg 3840
ggagtatttt agagtatttt tttttttatg atattcgtta agttggttcg gaaataattt 3900
ttatggggag ggggtggcgg tagggggcga tggtataggg ggttagaggt tattttaggg 3960
ggacgttttt gttatatata tttatttata ggacggttta taattatttt tcggtgtcgt 4020
tttttcgcgg ttttttatat atttgtttag ttatggggag tatattttag tttttagagt 4080
tgaatgggat attagagatt atattttgtg gttgtagacg aggaagcgaa ggtttagaga 4140
ggatgttatt tcgaggagtt atagagtatt gagaggacgt tttgggatta gaatttacgt 4200
ttttatatag tttagtattt ttttttattg ttttggacgg agtttttttt ttaattatgt 4260
ttttaaattt taatcgtaat ttgttttaat ttttagggtt atcgaggaat tcgaagagaa 4320
taatagtgag gagagagaga aaataggaaa agtcgagttt tatttttttt ttatttttat 4380
atcgtttttg tgttattttt tttgtttaaa tttttttttg cgttttgttt taagttttta 4440
gtagaattta agagagtttt attttaagtt tttttattta tatattttaa tttttagagt 4500
tatttgtttt ttttgtgacg ttttttattt tttttttaaa gttattttcg gtagtagttc 4560
gtttcgagcg cgtcgtttgt ttatttagtc gggagttcgg tacgcgttag gcgtacgtat 4620
tgtaataata aatttagttg aatggagagt ttgtaaggag cgggagaaag gaacggaggg 4680
gggggagagg agaggaggag ggggagttta gggagtgggt gggaggaaga ggtaagagga 4740
cgggggggag tgggggttgt agtcgttcgt tgtagtagcg gggaatgggg ggcgaggcgg 4800
ggttagggtt gcgcgtgggg ttgggtgttt tattgaaaag gcggacgtat ttcggtagtt 4860
tagtattttt ttatttttgg ttagggaacg tggaaggcgt atcgataggg attcggttag 4920
ggagggcgag tgaaagaagg aaattagaaa ggaagggagt taataaaata ataaaaatag 4980
tttgagttac ggttggagag atcgagattc ggcgtaagag agcgtagttt tagtaggaga 5040
ggaacgcgag acgcggtaga gcgcgtttag tattgatttt tgttgttgtt tttgtttttt 5100
ttttttttag aaataagaag gcgttagcgg tagttttata cgcgagcgtt acgcgaggtt 5160
ttcgaagtta attcgcgaag ggaggagggg agggaggagg aggcggcgtg tagggaggag 5220
aaaaagtatt tttatttttt ttgtttttat tttaagaagt ttttcgggga ttttgtatat 5280
attttttaat tttcgttagg gttttcgttt tatatttttt tttttttttt tttttgtttt 5340
tgtatttaag ttttttttgt gtttttttcg cgggtttcgt atttcgcgtt tcggatcgtt 5400
ttgatttcgc gattttttgg tcgtcgttgc gtatggaaag ttttgttaag atggagagcg 5460
gcggcgtcgg ttagtagttt tagtcgtagt tttagtagtt ttttttgtcg ttcgtagttt 5520
gtttttttgt tacggtcgta gtcgcggcgg tcgtagtcgt cgtagcggta gcgtagagcg 5580
cgtagtagta gtagtagtag tagtagtagt agtagtaggc gtcgtagttg agatcggcgg 5640
tcgacggtta gtttttaggg ggcggttata agttagcgtt taagtaagtt aagcgatagc 5700
gttcgttttc gttcgaattg atgcgttgta aacgtcggtt taattttagc ggttttggtt 5760
atagtttgtc gtagtagtag tcggtcgtcg tggcgcgtcg taacgagcgc gagcgtaatc 5820
gcgttaagtt ggttaatttg ggttttgtta tttttcggga gtacgttttt aacggcgcgg 5880
ttaataagaa gatgagtaag gtggagatat tgcgttcggc ggtcgagtat attcgcgcgt 5940
tgtagtagtt gttggacgag tatgacgcgg tgagcgtcgt tttttaggta ggcgttttgt 6000
cgtttattat tttttttaat tattttaacg atttgaattt tatggtcggt tcgtcggttt 6060
tattttattc gtcggacgag ggtttttacg attcgtttag tttcgaggag taggagtttt 6120
tcgattttat taattggttt tgaggggttc ggtttggtta ggttttggtg cgaatggatt 6180
ttggaagtag gtaggttgta ttttggggtg ggtagggggg tatttttgtt ttcgtttttt 6240
ttttgagtgt ttgtggaagt ggggatgttt ttaaggagat aaggggattt ttatttaaag 6300
aatttgtgaa agttggtcga ttttaagttt tagtttgtta gttttagtat tggtttttga 6360
aaatggtttt gtttaggttt ttaaggagtg aagggtagta gtgaggtgta gagatattgg 6420
tgaatcgaat attgggatat gttaaaagag atgtttattt gatagatttt ttttttagat 6480
ttttattttt ttttattatt agtttatacg tttaaattaa ttttttttgt tttttttttt 6540
atgttatagg 6550

<210> 288
<211> 6550
<212> DNA
<213> Artificial Sequence

<220>
<223> chemically treated genomic DNA (Homo sapiens)

<400> 288

tttataatat aaggaaaaga ataggagagg ttaatttgaa cgtgtaggtt agtggtagag 60
ggagatggag gtttggggaa agagtttgtt aggtagatat tttttttaat atgttttagt 120
attcggttta ttagtatttt tgtattttat tattattttt tattttttgg agatttgggt 180
aaggttattt ttagaggtta gtgttgaaat taataaatta ggatttgaaa tcgattaatt 240

tttataaatt ttttaaataa aaattttttt atttttttgg agatattttt atttttatag 300
atatttagag ggaggacgaa ggtaagaata tttttttgtt tattttaaaa tgtaatttat 360
ttgtttttaa agtttattcg tattagggtt tgattaggtc gagtttttta gaattagttg 420
gtgaagtcga gaagtttttg tttttcgggg ttgagcgggt cgtaagagtt ttcgttcgac 480
gagtaggatg agatcggcga gtcggttatg gagtttaagt cgttggagta gttgggggag 540
atggtgggcg ataggacgtt tgtttggaag gcggcgttta tcgcgttatg ttcgtttagt 600
agttgttgta gcgcgcggat gtattcgatc gtcgagcgta gtgtttttat tttatttatt 660
tttttgttgg tcgcgtcgtt ggggacgtgt tttcgaaggg tggtaaagtt taggttgatt 720
aatttgacgc ggttgcgttc gcgttcgttg cggcgcgtta cggcggtcgg ttgttgttgc 780
ggtaggttgt agttaaagtc gttgaagttg agtcggcgtt tgtagcgtat tagttcgggc 840
gaagacgagc gttgtcgttt gatttgtttg ggcgttgatt tgtgatcgtt ttttgagggt 900
tggtcgtcgg tcgtcggttt tagttgcggc gtttgttgtt gttgttgttg ttgttgttgt 960
tgttgttgcg cgttttgcgt tgtcgttgcg gcggttgcgg tcgtcgcggt tgcggtcgtg 1020
gtaaagaaat aggttgcggg cggtaggaag ggttgttggg gttgcggttg gggttgttgg 1080
tcggcgtcgt cgttttttat tttggtagag ttttttatgc gtagcggcgg ttaaggagtc 1140
gcggaattag agcgattcgg gacgcgaggt gcggggttcg cgagggggat atagagagaa 1200
tttgggtgta ggaatagaga gggaaagaaa aggaaatatg aagcgggagt tttgacggaa 1260
gttaaaaaat atatataaaa ttttcgggag attttttaga gtgggagtaa aaaaagtgaa 1320
aatgtttttt tttttttttg tacgtcgttt tttttttttt tttttttttt tttcgcgggt 1380
tggtttcggg agtttcgcgt ggcgttcgcg tgtgaggttg tcgttggcgt ttttttgttt 1440
ttaagaaaaa aaaaagtaga agtagtagta aaagttagtg ttgaacgcgt tttgtcgcgt 1500
ttcgcgtttt ttttttatta aggttgcgtt tttttgcgtc gggtttcggt ttttttagtc 1560
gtggtttagg ttgtttttat tattttgtta attttttttt tttttgattt ttttttttta 1620
ttcgtttttt ttggtcggat ttttgtcggt gcgtttttta cgttttttgg ttagaagtga 1680
gagagtgttg ggttgtcgga gtgcgttcgt ttttttaatg ggatatttag ttttacgcgt 1740
agttttggtt tcgtttcgtt ttttattttt cgttgttgta gcgagcggtt gtagttttta 1800
ttttttttcg tttttttatt tttttttttt atttattttt taaatttttt tttttttttt 1860
tttttttttt tttttcgttt ttttttttcg ttttttgtaa attttttatt tagttgggtt 1920
tgttgttgta gtgcgtgcgt ttggcgcgtg tcggattttc ggttgaataa ataggcggcg 1980
cgttcggggc gggttgttgt cgggggtggt tttaggaagg gggtgggggg cgttatagga 2040
gggataaatg gttttaggaa ttgaggtgta taggtggaaa gatttggggt gaagtttttt 2100
tggattttat taagaatttg aagtaaagcg tagggaaggg tttgggtaga aaaaatggta 2160
taggaacggt gtggaggtga ggagggagtg gggttcgatt ttttttgttt tttttttttt 2220
tttattgtta tttttttcgg gtttttcggt gattttagaa attggagtaa attacgattg 2280
aagtttagaa atatggttgg gggagggatt tcgtttagaa tagtgaaaaa aagtgttgga 2340
ttatgtgaaa acgtgggttt tagttttaag gcgttttttt aatgttttgt ggtttttcga 2400
agtggtattt tttttgagtt ttcgtttttt cgtttgtagt tatagaatat ggtttttaat 2460
gttttattta gttttaaaaa ttaggatgtg ttttttataa ttgagtaagt gtgtgggggg 2520
tcgcggggaa gcgatatcga ggagtggttg tgagtcgttt tgtaggtggg tgtatatggt 2580
agggacgttt ttttaggatg atttttggtt ttttgtatta tcgtttttta tcgttatttt 2640
ttttttataa agattatttt cggattagtt tagcggatat tataaagaag ggagtgtttt 2700
gggatatttt tagattttcg gagaagaaag atgcggggcg ggggttgagt gatggcgttt 2760
agggtattgg tggggatttt tggtttttcg gcgtcgtcgc gggtatttcg gcggttttgg 2820
cgtagttatt atttcgtttt aggcgtgtgt tcggcggttc ggtggtaggc gattgggagc 2880
ggagttcggc ggggtcgcgt gttggcgtcg agagggaggg ggaggcgttt gtagtcgtcg 2940
ttcgatcgtt tggatagcgt ttgtttaggc gttttttttg ggttttcggt tttggcgttt 3000
tttggttaga atttcgttta gttttaggac gtttggggac gcggcgttgc ggagaaagta 3060
gattaagtaa taggttttat ttttttaagg atttggttcg gagagaggtt ggggtatgtt 3120
ttaggttttg tttatatagg tagaacgaag tggagggtaa ttatttattt gattttttag 3180
tagaaataat tcgagatttg ggtgtaggaa tgattgtggt gaattattcg gataattggt 3240
tttaattgaa tttttttttg tggaggtgga tttttttaaa gggttaaggg gatgatttag 3300
ggagttgttt ttggtatgtg ggttggttga gtgtttttgg aataatagtg atgatggtgg 3360
gtagtaatga cgggcggttt tcgaagtgaa ttattttttt tttttatttt agttttattt 3420
ttgtagaata ggtaggtttt agaggtatag gattaaatcg tgttaataga ggttaatttt 3480
gtaatttttt ttagttatat agtttttttt ttttatggta atttgtggtt tgatggtttt 3540
ataggattgg ttgaatttcg tgggatttat attttaggtt tttttttata tttttgatta 3600
aggaaagggt ttaagaggtt tttggaagta taggaggtga gtatttaaaa gtagagttat 3660
aatatagttt gggtattttg tattttttag ttagagtaag ttttagatta tttgtagaaa 3720
taggaattat ttaggtattt tttatttatt ttttatatga tttagttgtg ttttaatgtt 3780
ttatttattt tattttagtt aaatagttga gtgttggggg tgggagggag tgtcgtaaat 3840
gtggaagtat ttagatgtgt ttttttttat attaagttga aatatgagtt ttaaattttt 3900
tattaatttg gatttatttt ataaaggttt cgagtttgag ttttattata atggtgtttt 3960
ataattttga ataagttttt ttgtttttat tggttttagt tttttgattt atatataagg 4020
gatgtatagt agttaatttt gaaatttgta tttaatagtt tagaaaattt gttggtagtt 4080
agtacgtgtt ttttttttta ggatgtatat atagagattt taatattagg gaagtagagg 4140
atagaatttt tttttttttt tttttttata tatattatga aatgggtgtt taaatggagt 4200

ggaagtgtat tgtagtgaag aagttttgtt ttgtagtggt tttatttatt ggttttaggt 4260
taggaggaag gatagtcgtt gttgggattt ttttggttaa aatcgttaga atatttgttt 4320
ttattttttt tatgttagtt ttgagtttga ttttattttt tttattttag tatgttaagt 4380
tttttttttt atatgttttg taaagttttt taagaatcga ggcgtttttt tttttttatt 4440
taagtaaatt tggagtttgt attttaatat tgttattttt attttttttt ttaattttag 4500
ggtagaatag tttataattt ttttttagag gtttgtattt ttatttttgg ggattttatt 4560
aagagagttt tttattgata ttggtttttt ggttgttttt attagtgtga agtaaaagat 4620
gggaggttta ggagttaaaa ttagaatttt gtttttgatt gtgtgattta gattataatt 4680
ttttttttcg atttgagaga attggttata tgttttgttt ttttaggttt atggaatttt 4740
agataaagta ggttgatttg atttggtatt taggtttttt tatttgagag ttgtgtgatt 4800
ttgagatatt ttaggggttt ttattttatt tatgttagta attatagtat ttatttttaa 4860
gagttgtgag attttaatga tatgtatgta gagtatttta gtttaggttt aaagagaaag 4920
ggattaataa gaaagttatg tttatataat ttatttagga aagggtgtgt ggtaggagga 4980
gttttagtat ttggtttttt ggaattattt tgtgattatt attttagtta atggagattt 5040
agtgttattt tgttttattg ttttagtatt taggggtgag ttatttttgt tatttttgag 5100
aaatggtggt gatattggta tattaagatg atggagatag taattcgatt tttagatatg 5160
aaaagtagta agtaagatag attatagaga tgttttgtgg tttttatatg taaggggttt 5220
tagtagagtt attgggtttt gtttttttgg tttttttgtt atatagtggg tttgattttg 5280
agtaagtttt tagggtttta gttggttaag atatttgaat aatgttttat ttatttttat 5340
atgaagggag aagtaaataa tttataaaag gaaggtttga tatttttatt tataaatgag 5400
gtggtagtat tttttttgtt tgtatgttaa gggtgttatg agaattaaat tagatgttgt 5460
tgggtaattt gttttgtaag tttttttata tattagagat tgttttttta tttttttcga 5520
atagtagtat ttagtgtttt gttttgttat gagggtgggg gagtgggaag gataaagtat 5580
tgattttttt tggtgagtat tatttagagt atagatattg tagaaagata agaaggtttt 5640
tttttttttt ttaatataat aagggattta atttttgtat ttttagattt tttttttata 5700
ttagaatatt ttggagttga attatatttt agattagatt ttatttttat ttagtattag 5760
gatttgtagg gggagatggt ttttttagtg gttaaagggg tttgttgttt tgagattaga 5820
gattttgtgt tttgagtttt ttgttatttt aatttttatt tatagttgaa ggttaggtta 5880
gtattatttt tgttgaagtt taagatttat gtatgttttt ttgattgtgt gttgtttttt 5940
tgtaaaaaga agatatgatt tatttttttg aaaattaatt tatgtttttt ttttggggag 6000
agggattgat attaaaagtg aaattaggat ttttattgga taaataattt aataaagtta 6060
tatttttaat tttagttaga ttttgaatta ggggtaaagg gaatataaag atgaatataa 6120
gttttggatt ttggattttg ttataggatt ttaagtgtaa tttttaattt gggggtgatt 6180
tgagttttag aagtaatgga tgttttgaga aaagtgagtg tgtttggtat ttatagttaa 6240
tatttatttt ttaggagtta taattaatat agggtttttg ttttgttggg gtgtttgttg 6300
ttttaaggtt gtgttagata ttgttttatt tagggaaaat gaaaaggatt ttattgtata 6360
atttattgtt tttaagaata ttttatttaa ggtaatttta gaaaggattg ttgtttagtt 6420
tgtattgatt ttgtattagt tttggagggt ttgtttaggg ggtagaatag tataaattat 6480
gtatttggtt tggtttagtt ttttggttat agtagttagg tttgtatttg tattaaggaa 6540
tttagttttt 6550

<210> 289
<211> 5964
<212> DNA
<213> Artificial Sequence

<220>
<223> chemically treated genomic DNA (Homo sapiens)

<220>
<221> unsure
<222> (843)

<400> 289

taaaaaaatt tggttaggta tagtggttta tatatgtaat tttagtattt ttggaagttg 60
aggtgggagg attgtttgag tttaggagtt taagattagt tttagtaata tagtgagatt 120
tggtttttat aaaaagtttt taaaaaatta gttgagtatg gtgatgtatg tttgtagttt 180
tagttattta gaaggttgag gtggattatt tgagttaagg aggtagaggt tgtagtgagt 240
taagatcgtg ttattgtatt ttagtttggg tgatagagta cgattttgtt tttttaatta 300
aaaaaaaaaa aaaaaaattt agtgtttttt agtttgttgg gtaataaagt atgatttagg 360
tgaaaattgt gagatatttt attttttaat tgataaataa taattgtata tatttatgat 420
gtattatatg atgttttgat atatgtatat attttagaat gggtaaattg agttaattaa 480
tatatgtatt atttttatat attttttatt atttttgtgt ggtgagaata tttaaaattt 540
atttttttag taatttttat ttatagtatg ttgttattga attgttatta ttacggtgtg 600
tgaaagtttt tgattttata gattaaataa ataattttaa gtaagttatt aaagttatta 660

tagtattagt ttttttattt gaggattaaa cgacgtttag tattttgttt aatatatgtt 720
aaatagttag tatattttaa agtgttttta aaatatagat aaaaagtaat ttaatataat 780
ataaaaatat atacgatata aaatgttttg tttttttttt tttttttttt tttttttttt 840
ttnaagagat gaggttttat tatattgtta aggttcgttt tgaatttttg ggttttagtg 900
atttttttgt tttagttttt taaagcgttg aaattattgg tatgagttat tttgtttagt 960
tgaaataagt ttttttttat aaagtttttt gttattatta ttattattag agatttttaa 1020
gtaaaattaa gattttgttg ttgatgaaga aaaggtttat aaattagtag ataatattgt 1080
tttttttaga atttttattg gaaaagggtt ttgatttaga ttttaagaga gggtttttgg 1140
attttatgta agaaagaatt tcgggcgaat ttatagagta aagtgaaagt aagtttggta 1200
agaaagggtc gagcgaggtg gtttatgttt gtaatttagt tttttgagag ttcgatcgag 1260
gcgggcggat tatttgaggt taggagtttt agattagttt ggtgaatatg gcgaaatttc 1320
gattttatta aaaatgtaaa aaatagtagg gtgttgtggc gtaggtttgt aattttagtt 1380
ttttgggagg ttgaggttta agaattgttt gaatttagga ggtagaggtt gtagtgagac 1440
gagtttgtat ttttattgta ttttagtttg ggggttagag taagattgtt aaaaaaaaaa 1500
aaaaaaaaaa aaaaaaagaa ggaaaggaat aaagaatggt tattttatag atagagtagc 1560
ggtatgggtc gtcggttgtt tatttttatg gttatttttt gattatatgt taaataaagg 1620
gtggattatt tatgagtttt cgggaaaggg gcgggtaatt ttcggaattg agggtttttt 1680
ttttttttag attatatagg gtaatttttt gacggttgtt atggtatttg taaaatgtaa 1740
attgttttgg tattggtggg agtgtttttt agtatgttaa tgtattataa ttaagttata 1800
ataagtagtg aggacgatta gaggttattt ttttggttat tttggttttg ttgggttttt 1860
gtcggttttt ttattgcgtt ttgttttatt agtaaggttt ttatggtttg tattttgtgt 1920
tgatttttta ttttattttg ttgatttaga atgtttttat tttttgggaa tgtagtttag 1980
taggttttag ttttatttta tttagttttt atttaagatg gagtcgtttt ggtttaaatg 2040
tttttgatag aatcgtgtga aataatataa atattttggt taaattttag tgtgagtgat 2100
taatattttt taattttaaa taatttttat tattagttaa tatttgttta tttttagtaa 2160
tatatacgtt gtataaagag tatagaatga gatgggtttt ttttggtatg tttatagttt 2220
gtatatattt tttattgagg gttttttttt tttgtttatt gttatgttta tagtttttta 2280
atttttatta tttttaattt tgggatgttt tttgatttag agtggtaagt aattttaaaa 2340
tataagagat aaaatatgaa tatttgtaga attttagaga aagaaagatg taaggtatat 2400
ttatttattt atttaattat gtattttaga gttgggggtt ttgttatgtt gtttaggttg 2460
gtttgtaaat ttttgggttt aagttatttt tttattttag ttttttaaag tgttaggatt 2520
ataggcgtga gttatggcgt tcggtttttt aaacgtttat taatagtttt gatattcgcg 2580
aagtgattga tatttgtttt tttttaattt ggaggttggg attaatatac gtgtttttta 2640
ttattgttta taaagcgttg ttgatacgta ggaggggagt ggtattttcg taatttattt 2700
ttttaatttg tgttaatggg atttagtata ggagatggat agtaattcgc ggttgatttg 2760
gatttagttt agttagttta gtttttattt ttttaagtgt ttttaggttt ttttgtttat 2820
tttcgttttt tgtttagtgg aaggaaaagt ttggggagaa ggaaaaaatt taaatttttt 2880
ttttaggcgt gtgaatgtat tttaagtatg ttaaatattt tagtttttta atttaattat 2940
aatatataga aaagaggtat ttttaggata tttaaaattt gtagtataat tgcgtaattg 3000
aagagcgtcg tcgtgttttc gttatcggtg cgtttttcga tttttcgcgg cgcgtaagtg 3060
cgcggtttag tagtaggtag tcggggacgg aagggtacgg ggaggtcggc gttttgttta 3120
gtatagaatt ttgggtcggt atcgtttttt tttgcgggcg atttcggaat tgtttgggat 3180
tataaatttc gattaataaa ggatttatat ttttttgggt tttttaaagg tagaaagaaa 3240
tttatttttt tgttataata agattttgtt aagagatgta aaagtaaagg tcgaagttag 3300
ggaggttgcg atagtatggg gggaggggcg gttacgatgg tgagtgtcgt tgattaatta 3360
tttgggttcg aattttaggg ttgttatttg ggcggttatt atgtgttagg tgatcggaat 3420
acggaggtga atttgtttgt cgaggtcgtt tttttttggg ggaattatga ttagttcggt 3480
tgttttggtt aagttttagt ttcggttttt ttatttgtag gaagtatttg ttttagaatt 3540
gttgtaagga gtgagatttt gttttaagat gtaaacgggt cgggcgcggt ggtttatatt 3600
tgtaatttta gtattttggg aggtcgaggc gggtagatta cggtgttagg agttcgagaa 3660
tagttttata tggtgaaagt tcgtttttat taaaaatata aaaattagtt gggcgtggtg 3720
gcgggcgtta gtaattttag ttacgtagga ggttgaggta ggataatcgt ttgaattcgg 3780
gagatagagg ttgtagtgag tcgagatcgc gttattgtat tttagtttgg gagatagaat 3840
aattcgtttt aaaaaaaaaa aaaaaaaaaa aggatgtaat cgattaatat tttgttgggt 3900
atatagtaaa gggtttagta agtatttttt ttttttttat atagttttaa gatagttcgg 3960
aagggagata gttacgttta agagtgattt tttgagtagt ttttttaaag aagtattgaa 4020
ttttgtaagg acgtttagtt gtaggtagaa agggtagggg ttttgggagt aaaggtgtat 4080
aagttaggaa gttataggag cgagttttgt atttagggcg cgggtagaga atttcggagg 4140
gatttttggt aagggttttt cgttcgggtg tttagagcgg tatgattagt attcgtttcg 4200
tttttatttt ttttaggttc gcgattggtt agcgtttttt ggttgttcgt ttttggcggg 4260
gttagagggc gtcgggggaa aggtcgagtt cgcggggtag tgggcgggga cgcgcggaga 4320
gtttggcgag gcggtttatt aatcgcgttc gggtttgagg gggcggagtc ggggcggggt 4380
ttttcggcgg gtattagaag taggtgattc gggcggggcg ggagagggta gttagtttgc 4440
ggtcgtgagt tcgttgtggt cgtcgtcgcg cgcggtttcg tgttcgttag aggtgggcga 4500
aagggtgcga gagcgcgggg gaggagtcga gtagggtacg tttcgagagc gtcgttatcg 4560
tttttaggag tttattggag cgcggagttt tttgtagggg aggaggcgag cggcgggcgg 4620

gacgcggcgg tcggcgttta ttatgtagtt tcggtttggt tagcgtcggt tgttcgcggt 4680
gttgtgaggc ggcggggttt tacggttttc gtttttcgtt tttttttcgt ttaggtgttg 4740
ttaggcgcgt tcgtttttcg ttcggttttc ggtttcgggg agaacgtacg gtttgttttt 4800
gttcggtttc gtttggatta tgcggggggt tttcgaggag gagagggcga gtgatcggtt 4860
tgcgtcgttt ttgttttggg ggcgtttttt attttgagtc gtttttattt gttgttatgt 4920
gtcgttgtta cgggtattta gtttgtcgtt aaatttttcg ggcgttagtt cggttttgag 4980
tcgcgttttt tagcggagtg atttagggac ggaggattta ggttggttgg ggattgtttg 5040
ttttttttcgg cgggattcgt ggaggtgaga atttttttt ttttttgtt ttttttcgt 5100
tttttgtgtt ttttttcggg aagttgaggg ttttggtttt cgggtattgt ttgaagggtt 5160
tagtttggaa agagagggtg ggggagtcgg tagtttggtt ataggaggcg gagcggggag 5220
gaaaggaaag gggttaattt aagagatgtg gaataaattt tttgtaaatt gaatgttggg 5280
ttaaatttat aatgatattt aattatttat tagtaattgt taatttggag ggttgaaatt 5340
ttattttgtt tttttttttt tttgagacgg cgttttgttt tgttgtttag gttggagtgt 5400
agtggtagat tttggtttat tgtaattttt gttttttagg tttaagtaat ttttttgtta 5460
gttttttaat agttgggatt ataggtacgt attaatatgt tcgattaatt tttgtatttt 5520
taatagagac ggggtttcgt tatgttggtt aggttggttt cgaatttttg attttaagtg 5580
atttttttgt tttagttttt taaagtgttg ggattatagg tatgagttat cgtttcggtt 5640
ttttgttttt ttttgtattt ttagtatgtt ggtattttat gaataattgt taagtgaata 5700
gatggttgaa aattttttaa tagaggtgta gggggatgag ggaaagaaat aagtaaatat 5760
aatggaatat atggaatagt taatggtata ttaaggagat ataaattgaa atttcggagg 5820
ttatgtagtg aaagatgttt gtttaatatt ttaataagtt tttttgagtt tagtttagtt 5880
tttatttttt ttagaaagtt tttaggtttt gggttttagg ttatggtatt tggggttttt 5940
ttggaaaagt gtttttttatt agtg 5964

<210> 290
<211> 5964
<212> DNA
<213> Artificial Sequence

<220>
<223> chemically treated genomic DNA (Homo sapiens)

<220>
<221> unsure
<222> (5122)

<400> 290

tattgataga gagtattttt ttagggaaat tttaaatgtt atagtttgag gtttaaggtt 60
tagaggtttt ttgaagaggg taggaattgg gttgagttta gaggagtttg ttgaaatatt 120
aaataagtat tttttattat atgattttcg gggttttaat ttgtgttttt ttaatatgtt 180
attaattgtt ttatgtattt tattgtattt atttattttt tttttttatt tttttatatt 240
tttgttggga ggtttttagt tatttattta tttaataatt atttatggag tgttaatatg 300
ttggagatat aaaggaaaat aagaggtcgg agcggtggtt tatgtttgta attttaatat 360
tttgggaggt tgagatagaa ggattatttg aggttaggag ttcgagatta gtttggttaa 420
tatggcgaaa tttcgttttt attaaaaata taaaagttag tcgggtatgt tggtgcgtgt 480
ttgtaatttt agttgttggg aggttggtag gagaattgtt tgaatttggg aggtagaggt 540
tatagtgagt taagatttgt tattgtattt tagtttgggt aatagagtaa gacgtcgttt 600
taaaaaaaaa ggaaaataag gtagaatttt agttttttag attggtaatt gttagtaaat 660
agttaaatgt tattatagat ttgatttagt atttaattta taaaaaattt attttatatt 720
ttttaagtta attttttttt tttttttttc gtttcgtttt ttgtaattaa attatcgatt 780
tttttatttt ttttttttaa gttgggtttt ttaggtagtg ttcggagatt agaattttta 840
gtttttcgga aaagggtata ggaaacggga gaagaatagg gagaaaggga aggtttttat 900
ttttacggat ttcgtcgaga agagtagata gtttttagtt agtttgggtt tttcgttttt 960
gggttatttc gttgagaagc gcgatttaga gtcgggttgg cgttcggaaa gtttagcgat 1020
aggttgggta ttcgtggtag cggtatatgg tagtaggtgg gggcgattta gagtgggggg 1080
cgtttttaga ataggggcgg cgtaggtcgg ttattcgttt tttttttttc gggagttttt 1140
cgtatgattt agacggagtc gggtaggggt aggtcgtgcg tttttttcgg ggtcgggagt 1200
cggacggagg gcgagcgcgt ttggtagtat ttgggcggga ggaaggcggg aggcgggggt 1260
cgtgggattt cgtcgtttta tagtatcgcg agtagtcggc gttggttagg tcgggattgt 1320
atggtgagcg tcggtcgtcg cgtttcgttc gtcgttcgtt tttttttttg tagggggttt 1380
cgcgttttag tggatttttg ggggcggtgg cgacgttttc gggacgtgtt ttgttcggtt 1440
tttttttcgc gttttcgtat tttttcgttt atttttggcg agtacggaat cgcgcgcggc 1500
ggcgattata gcggatttac ggtcgtaggt tggttgtttt ttttcgtttc gttcgggtta 1560
tttgttttta atgttcgtcg ggaagtttcg tttcggtttc gtttttttag gttcgggcgc 1620
ggttggtggg tcgtttcgtt aagtttttcg cgcgttttcg tttattgttt cgcgggttcg 1680

gttttttttt cggcgttttt tggtttcgtt aggagcgggt agttaaggag cgttgattaa 1740
tcgcgggttt ggaggagatg ggggcggggc gaatgttaat tatgtcgttt tgggtattcg 1800
ggcgggaagt ttttgttaga agttttttcg gagttttttg ttcgcgtttt gggtgtagga 1860
ttcgttttg taatttttg gtttatgtat ttttgtttt agggttttg ttttttttgt 1920
ttgtagttaa gcgtttttat agagtttagt gtttttttga aagaattgtt taaggaatta 1980
tttttaggcg tggttgtttt tttttcgagt tgttttggaa ttgtgtgaaa gaagagagga 2040
tatttattga gttttttatt gtgtgtttaa taaagtgtta atcggttata tttttttttt 2100
tttttttttt ttttgagacg agttgttttg ttttttaggt tggagtgtag tggcgcgatt 2160
tcggtttatt gtaattttg tttttcgggt ttaagcgatt attttgtttt agtttttgc 2220
gtagttggga ttattggcgt tcgttattac gtttagttaa tttttgtatt tttggtagag 2280
acgggttttt attatgtaag gttgttttcg aatttttgat atcgtgattt gttcgtttcg 2340
gttttttaaa atgttgggat tataggtgtg agttatcgcg ttcggttcgt ttatatttta 2400
agataagatt ttatttttta taataatttt gaggtaggta ttttttatag gtgaagaaat 2460
cgagattgag atttggttaa ggtaatcgag ttaattatag tttttttaag aaagggcgat 2520
ttcggtaagt aagtttattt tcgtatttcg attatttggt atatagtagt cgtttaaatg 2580
gtaattttgg gattcgaatt taggtagttg gttagcggta tttattatcg tagtcgtttt 2640
tttttttata ttatcgtagt ttttttggtt tcgattttta tttttgtatt ttttaataaa 2700
attttgttgt gataagaaaa tggatttttt tttgttttta gaagatttag aaagatgtga 2760
gtttttattt ggtcgaggtt tataatttta ggtagtttcg aagtcgttcg tagagagaag 2820
cgatatcgat ttagggtttt gtgttgggta gggcgtcggt tttttcgtgt tttttcgttt 2880
tcggttgttt gttgttaagt cgcgtatttg cgcgtcgcgg ggggtcggag ggcgtatcga 2940
tggcggggat acggcggcgt tttttagttg cgtaattatg ttataagttt taaatgtttt 3000
aaaaatgttt tttttttatg tgttgtaatt agattggaag attgaggtat ttagtatatt 3060
tgaaatatat ttatacgttt aggaagaggg tttaaatttt tttttttttt ttaaattttt 3120
ttttttattg ggtaaagggc gaaaataggt aagagggttt ggggatattt gaagggatag 3180
ggattggatt aattggatta gatttagatt aatcgcgggt tattgtttat tttttgtgtt 3240
ggattttatt ggtataggtt ggaagaataa attgcggaag tgttattttt tttttgcgta 3300
ttaataacgt tttgtgggta ataatgaaga atacgtgtgt taattttagt ttttaagtta 3360
aaaaggaata aatattaatt atttcgcgga tattaaaatt attaataaac gtttaaaagg 3420
tcgggcgtta tagtttacgt ttgtaatttt aatattttgg gaggttgagg tgggaggatg 3480
gtttgaattt aggagtttgt agattagttt gagtaatata gtaagatttt taattttaaa 3540
atatataatt aaataaatga ataaatatat tttatatttt tttttttttg gaattttata 3600
ggtgtttata ttttattttt tgtgttttgg aattatttgt tattttgggt taggaagtat 3660
tttagggttg aaaatagtgg gagttgaaaa gttgtgggta tagtagtgag taagaaaggg 3720
aagtttttag tggaaagtat gtgtagatta tgaatatgtt aagggaggtt tattttattt 3780
tgtgtttttt atgtaacgtg tgtattattg gggatggata agtgttggtt aataataaaa 3840
attatttgaa gttggagaat gttaattatt tatattggaa tttagttaga atatttgtgt 3900
tattttatac ggttttgtta gaggtatttg aattagagcg attttatttt ggatagggat 3960
tgggtaaaat aaggttgaga tttattgggt tgtattttta ggaggtgaag atattttaag 4020
ttaatagggt gagataggag gttagtataa gatataggtt ataaagattt tgttgataaa 4080
atagggcgta gtaaagaagt cggtaaaaat ttagtaaaat taagatggtt aggagagtga 4140
tttttggtcg tttttattgt ttattgtaat ttaattataa tatattagta tgttaaaaga 4200
tatttttatt aatgttaaag tagtttatat tttatagatg ttatggtaat cgttaggaag 4260
ttattttgta tggtttaaga gggggaggaa tttttagttt cgggaattgt tcgttttttt 4320
ttcggaaatt tatgaataat ttattttttg tttagtatat gattaagaaa taattataaa 4380
aatgggtaat cggcggttta tgtcgttgtt ttgtttatgg agtagttatt ttttattttt 4440
tttttttttt tttttttttt tttttttttt ttttgatagt tttgttttga tttttaagtt 4500
ggagtgtagt gggggtgtaa gttcgtttta ttgtaatttt tgttttttag gtttaagtaa 4560
tttttgggtt ttagtttttt aggaagttgg gattataggt ttacgttata atattttgtt 4620
attttttgta tttttagtaa agtcggggtt tcgttatgtt tattaggttg gtttggaatt 4680
tttggtttta agtgattcgt tcgtttcggt cggattttta aggggttgga ttataagtat 4740
gaattatttc gttcggtttt tttttattaa atttgttttt attttatttt atggattcgt 4800
tcggaatttt tttttgtatg agatttaaaa attttttttt ggggtttgaa ttaggatttt 4860
tttttagtaa aggttttgaa gaaaataatg ttatttattg gtttgtaaat ttttttttta 4920
ttaatagtaa aattttaatt ttatttggag atttttaata ataatagtga tagtagagaa 4980
ttttatggaa agaaatttat tttagttggg taaggtggtt tatgttagta attttaacgt 5040
tttgggaagt tgaggtagga agattattag ggtttaggag tttaagacga gttttggtaa 5100
tatagtgaga ttttattttt tnaaaaaaaa aaaaaaaaaa aaaaaaggag agagtaagat 5160
attttatatc gtatatgttt ttgtattgta ttgggttgtt ttttgtttat attttgaagg 5220
tattttaaaa tatgttggtt gtttaatatg tattaggtag ggtgttagac gtcgtttaat 5280
ttttagatga agaaattggt gttataatga ttttgatgat ttgtttgaag ttatttattt 5340
agtttatgga gttaaaaatt tttatatatc gtgatgatga tagtttagta ataatatatt 5400
gtaaatgaaa attgttaaga gagtagattt taagtgtttt tattatataa aaataatgaa 5460
aaatatgtag aggtaatgta tatgttaatt agtttaattt atttatttta gaatgtatat 5520
atatgttaga atattatgtg gtatattata aatatatata attgttattt gttaattaaa 5580
aaataaaata ttttataatt tttatttgag ttatatttta ttatttaata aattagaaaa 5640

tattgaattt tttttttttt tttttggttg gggggatagg gtcgtgtttt gttatttagg 5700
ttagaatgta gtggtacgat tttggtttat tgtaattttt gtttttttgg tttaagtgat 5760
ttattttagt tttttgagta gttgggatta taggtatgta ttattatgtt tagttaattt 5820
ttttaaaatt ttttgtagag attaggtttt attatgttgt tgaagttggt tttgaatttt 5880
tggatttagg taattttttt attttagttt ttagaagtgt taggattata tgtgtgagtt 5940
attgtgtttg gttaaatttt ttta 5964

<210> 291
<211> 6452
<212> DNA
<213> Artificial Sequence

<220>
<223> chemically treated genomic DNA (Homo sapiens)

<400> 291

tgtttgttgt gtttagagta gtagagtttt taatttagga gttttatgtt ttttggtatt 60
atttagaaga tagtaataga ttaatttgta agtggggtaa aattaaattt tagattttat 120
attgtttgtt attattttta taaattttgg aaattttgga atttttgtaa aatttgtagt 180
tttatttatg gtttataagt tattatggtg agggagatat ttgtgaatag ggattgattt 240
tataagtgat tagttataag tatgttttaa agatattttt aaaaattgaa ataattattt 300
agtaaagtat atagaattaa agtgtgtagt ttaatgaatt tttgtatatg gtatatattt 360
atgttattat tatttagatt aaaatatgga aaatttttag ttttttagaa ggttgtttta 420
tgattttttt tagtgttttt ttggtttgga tggtataggt tagttttgtt tgtttttgaa 480
tgttgtgtag atggaataat gtgtaatttt ttttttagaa tatatgagaa ttttttatgt 540
tttatatata ttagtttttt ttgtagttgt gaagtatttt attttatgaa tttattatta 600
gtatttattt attttattgt tgatgaatat ttgggtttta gtttgggatt attataaata 660
aagttgttag gaatatgtat ttgtttttttg gtggatatag tatttatttt tgttggatat 720
atatttagga atggaatttt tgggttatag ggatttatgt ttagcgttag tggatattgt 780
taaataattt ttaaaagtga ttggattaat gtatattttt attattaata tatgagagtt 840
ttagttttgt attattatga atagttaatt tttttttttt ttttaagtgt agttattttg 900
gtaggtatcg agtgagattt tattgtggta gtttttgttt tttttttgta tttatgtgat 960
aattaatgat attgagttta tttttttatt tagtggttat tttaatgttt ttttttgtga 1020
aggatttgtt tagatttttt gattatttat taatttttat gatgaatttt tttttttttg 1080
taatgtaagt tgagacgtgg ataataagta ttcgggtagt ttgtatatat tatttattta 1140
gtttgggaag tatagaaaat atttttagaa ataattttat taaaagtgtt aaagtttttt 1200
agttatttta tatttaaaga gttgagtgtg gagatttgtt ttttttttgt gttagtttta 1260
ttagtaagag ttttaggttt ttaaaattga tatttattgt tttttttgaa attaattttt 1320
tgaagtttag tggtatattt atttattaaa gttattggtt ttttttttgt ttatattttt 1380
tttaaatttt tttatatgtt taattttttg tagtattatt gtatttagtt ttgttttttt 1440
aatagtgtat tattagtaat ttattattag gaaatttttt tttaatagtg aatgtttcgt 1500
aatatttagg tgtttattta ttgttttttt tattttgttg taaatttttt ttttttagtt 1560
ttttgtgtaa aatttaaat ttgtttttta ttttttattg gttaaatttg tatttttagt 1620
tatgattttt ttttagtgga gtttattatt atttttattg ataaagttgt tttttgaatg 1680
tttttaattt tgtgggtaga atttttattt tattattatt ttagttaacg tgggtttaaa 1740
atttgaaatt attttttaat tttaaggtat agttagtaga aaaattagtg attatttatt 1800
aagtattata gtgtgtggta tggtatatgt aatggtttag gtgttgaaga ttatttttta 1860
gaaggatttt gtattgtatt tgggaagtta tgatattatt tatgatttat taattagttt 1920
ttattataaa tatttagtga ttatagtttt tttagtattg ggtggtaaag gaagttttta 1980
taaaagggga tagattggag tttattatta agaatgatta agattttttt tggaagatat 2040
agatgggtag aggggtaggg atttggattg gattagaagg tgtgtatagt ggaagagtaa 2100
tgggtaagat tagattagga ttagatttag ttagtttttt aagttttggt tcgttttttt 2160
tttgaatgtt aatgattaga atttatgttt gaatagtatt gttgagtttg taaagtttgt 2220
gttagataga tattagtatt ttaatatttt tgtgatttag gaattattat ttttatttta 2280
tagaaaagtt aataggtttt gaaaaagtga tattagtaaa gaatagaatt tggtgggtat 2340
agtggtttaa gtttataatg ttattaattt gggagattga gtttggtgtg ttttattttt 2400
aggtatattt ttattttttg atattataga tatttataaa taataatttt tttgttttaa 2460
gtttgatata gatattttta gaaaggtata tatattttta taaattgttt tttttattta 2520
tttatttgat aaatggatat tattttaggt ttagggtatt tagtggtttt gagggtagac 2580
gagattattt gtatattaat attgtattgg ggaaaataaa gtaaataaat aaagttgata 2640
atgtaatatt agattagggt aaatgttata aaaaatgaat agggttaggg gatagagaat 2700
gttgtgagaa tgggttgtta tgttattttt aattaagtgg ttagggaatt ttttttttag 2760
gaggtgattt ttgaataatt gaattaattt gagtgagtat gttgcgttaa gttacgggag 2820
aagagaattt aggggagaga atattagatg taaagatttt atatagaaat aattttagaa 2880
tatttgagaa atggaaagaa gttaagtgtg gttaaatgta attaggtagg ataatggtta 2940

agaaatgagt aaggagaagt taagggtttg attatgtatg atattgtatt tagaattttg 3000
gttttgaagt tttgtttttt ttgttttttt tttttttaat tgtttttttg ttgtttatat 3060
atatattttt ttatatatac gtatattatt ttatttagtt ttttattata ttttagtgtt 3120
tatgaattag ttagttaggt ttgtttttta tttagtttag gaaatgttat gtttatgttt 3180
gtttatatat atattttttt gtttgtgtgt ttttgttttt gatatttttt attttatttt 3240
agataattaa attatggttt tattttttga tttatatgga ttttattata tatttttgat 3300
gtagatgtaa ttgaggttag ttttttttat agtttattta gtatataata ttttatattt 3360
tgtgatttgt tagtgataat tatgttttta ttttttttgt tatatttttt tattttaatg 3420
tggttagttt tgtttgttta ggaaaaatta ttttatatag ggttgggtaa gataaggtat 3480
aaggtatatt tatatataag gaggatattt ttattttttt gtattttttt ttattatggt 3540
tttagtatag aaggttggat agttgttagt agttgatcgg tatttagaat tgtatttggt 3600
attttagtag gtttttagta agtgtatttt gaatgaatta gtttattgat tgttgagatt 3660
aaattttatt tggtataatt ttttttaaaa agttttatta tggttattta taatatatgg 3720
gatgtaataa agaattaata attatttgtt gagtgtatgt tagttttatt ttattttgtg 3780
tttatattat ttttatttat tttatgttat tgtttgggtt ttagttgagt atatatattt 3840
ttttttggaa agatgtattt tagtttagaa taatattgat gttagaattt tttaaagata 3900
aagttatagt ttttggtaat tgtaggtata agaaaggaag tattttttgt tatattattt 3960
ttttaaacgt tatttttgtt attttagtag gtatttatcg tttgtggttg gttgtgtagc 4020
gatttgtagg agattttata tatttttttt attttttatt tagaaatggg attttattat 4080
gttgtttaga ttggacgtga ttgaattttt gggtttaagt gagtttttcg aataattggg 4140
attataggtg tgtgttattg agtttggttt ttattagttt ttatcggtgg agtgtttgtt 4200
atttttattt gttattatta ggagtttttt aatggtgtag aatagtggtt tatttggagt 4260
aagtttatta attaatataa aatattattt aaaataagtc gataggaatt ataaaattag 4320
aaattgattt ttttaaaatg aatttatttt ttttaaattt atttaatata tttagtttta 4380
taaatagata tttgttattt tattggtagt tataatgatt tatttgaggg tttggtatat 4440
tttatataat cgtgaatata atttgtattt aataatgtga ttttagtagt attatgattt 4500
ttgtttaaat ttttttagtt tgggaaatat ttaaagagaa taatgatttt agagaagagt 4560
tggatttttt ttaagatttt tatttagatt aggatataat tacgtttaaa attgatatag 4620
tatgtaatat ggattttagt gaagaaaagt attttagaat taaattttag aagagtgttt 4680
tagggtttag tggtttaatt aaagggagtt tagaagttat ttttggataa tatataggag 4740
gtagagtaat ttggttttgt ttttggttat tatttgatat tgttagtgtt ttatttgttt 4800
atattatggg gaaggaatcg agtggttata gaatttttgg agaaaattta ttaatttttt 4860
gtttattttg taatgttttt ttgtttttat ttaataaaat atgtttttcg attatattaa 4920
tttaaatttg tatatattga attgatttaa attaggattt tttttttttt ttttttttag 4980
gatttgattt aatgaaagaa gtatatggta tttgtgaaga taaatgttat tttttttttt 5040
ttaattggaa tttttgttta ggatttgtgt atgacgtttt atttgtgatt tgttttttcg 5100
gtagttattg attttgagtt ataggaaggt tttcgaagat ttgtgttaaa tgacgttaat 5160
ggttagtttg ttttttttta ttagtttagt agtaaagcga ttgttgggtt ggaaataagg 5220
tgatgaggag gagaaatggg tagaaaaggt agttgatgtt ttggtgaaga aattaaaaaa 5280
gaaaaagggt gttatggagg aattggagaa agttttgagt agtttaggat agtcgagtaa 5340
atgtgttatt atttttagat ttttagatgg acgtttgtag gttttttata gaaaaggttt 5400
attttatgtt atatattgtc gtgtttggcg ttggtcggat ttgtagagtt attatgagtt 5460
aaagtcgttg gatatttgtg aatttttttt tggatttaag taaaaagaag tttgtattaa 5520
tttatattat tataagagag tggagagttt aggtaggttt tatttttgag aagaatttgg 5580
aaaaataaaa ataaaaaatt tttttttttg atatgtatgt aattagattt agtaatgaaa 5640
aggttgtatt agttttgtgt ttttatttta aatattttaa ttttggaagg gttattggat 5700
tttgagttta gttttttata tgtaggtgat ataattttta ggtggtttat tttaatttat 5760
tatttattgt aagtatttat atatttattt tgtttttttt tgtggatgat ttgtttttgg 5820
tttatttaag ttttaagttt tgatggtttt tttgtttgag atgttattta gagtatttat 5880
agttttaata atagtttttt tggggaatta taaaagtaat gatgtttttg acggaaaatt 5940
gttgtgttaa tagaaatgta tgaaaaataa aattattaat ttttttatta tttagagaga 6000
attattgtta atattttaat tatttttttt tttttttttt tttatgtaga aatattatgt 6060
tgtaagtatt taattgtata aagttttata gtatgtattt ttaaaattaa atattgtatt 6120
attagtattt ttttatgttt taaaattttt ttaaaatttg aaaaatatga tttttaataa 6180
ttatttaata gtttatttta taggttatta taatatttaa taattttttt attggtggat 6240
atttttttgt attttatttt aagtaatatt ttttttatgt aaatgtttag tttttatata 6300
gagattataa tttgatttta ttaattaaat tatttttatt ttatattaat ttttttttag 6360
gtttttttgt attagaagag ttgggatgta aattacggga gtgattttag tttagaaata 6420
aattttattg ttgtatcgta gtagtaacgt tt 6452

<210> 292
<211> 6452
<212> DNA
<213> Artificial Sequence

<220>

<223> chemically treated genomic DNA (Homo sapiens)

<400> 292

gggcgttgtt gttacggtat agtaatggaa tttgtttttg agttgagatt attttcgtaa 60
tttatatttt aatttttttg atatagaaaa gtttagaagg aagttggtgt gaaataaaaa 120
tagtttgatt ggtagaatta aattataatt tttgtataaa ggttaaatat ttgtatgggg 180
gaaatgttgt ttgaaatagg atataaagga atgtttatta atagagaaat tattaaatgt 240
tataataatt tgtagggtag attattaggt agttattaaa aattatgttt tttaagtttt 300
agaaaaattt taaaatatgg gaaaatgttg gtaatataat gtttaatttt aaaagtgtat 360
gttatgaaat tttatgtagt tgagtgttta taatatagta tttttgtata aagaaaaaaa 420
gaaggaaaag tggttaaaat gttaatagtg gttttttttg ggtgatgagg aaattggtga 480
ttttattttt tatgtatttt tgttgatata gtaatttttc gttaaaaata ttattgtttt 540
tataattttt taaaggaatt gttgttaaaa ttgtaggtgt tttaaatgat attttaagta 600
gagaagttat tagagtttag ggtttaaata aattagagat aggttattta taggaaaagg 660
tagagtagat atatgggtgt ttatagtgaa taatgaatta gagtaagtta tttgagaatt 720
gtattattta tatataaaaa gttaaattta aaatttaata gtttttttaa gattagaata 780
tttaagatgg aaatataaag ttaatataat ttttttatta ttaaatttag ttatatgtat 840
attaggaaga gaggtttttt gttttgtttt ttttaaattt tttttaggaa taagatttat 900
ttggattttt tattttttta tagtggtatg ggttgatata aatttttttt tgtttagatt 960
taaaaggaaa tttataaata tttaacggtt ttagtttatg atgattttgt aaattcggtt 1020
agcgttaaat acgataatat ataatatggg gtaagttttt tttgtgagaa atttgtaggc 1080
gtttatttaa agatttggga atagtgatat atttattcgg ttgttttgga ttgtttaagg 1140
ttttttttag tttttttatg gtattttttt ttttttttag tttttttatt aaagtattaa 1200
ttgttttttt tgtttatttt ttttttttat tattttgttt ttagtttaat aatcgtttta 1260
ttgttggatt agtaaaagaa aataagttgg ttattgacgt tatttgatat aaattttcgg 1320
agattttttt gtaatttaaa gttagtggtt atcgaaagaa tagattatag gtgaaacgtt 1380
atatataggt tttaagtaga agttttaatt aaaaagggag gagtaatatt tatttttata 1440
agtgttatat gtttttttta ttgggttaag ttttgaaaga gaaaggggga aaggatttta 1500
gtttaaatta atttaatata tataaattta aattaatata atcgaaaggt atattttatt 1560
aaataagaat agaaaagtat tataaagtaa ataaaaaatt gataaatttt ttttaaaagt 1620
tttgtgatta ttcgattttt tttttataat atgaataagt aagatattgg taatgttaaa 1680
tgataattag aggtaaaatt aggttgtttt attttttatg tattatttaa aaatagtttt 1740
tggatttttt ttgattaggt tattaaattt taaaatattt ttttaaaatt tgattttgaa 1800
gtattttttt ttattgaaat ttatgttata tgttatgtta attttgaacg tgattgtgtt 1860
ttgatttgaa tagaagtttt aaaagaaatt tagttttttt ttaaggttat tatttttttt 1920
aaatgttttt tagattgaga aggtttggat aaaaattatg atattattga agttatatta 1980
ttagatgtaa attgtattta cggttgtata agatgtatta aatttttaaa taaattatta 2040
taattgttag taaaatggta agtatttatt tgtaaaatta agtatgttga ataaatttaa 2100
agagaataaa tttattttaa aagggttagt ttttaatttt ataattttta tcggtttatt 2160
ttaagtgata ttttatattg gttaatgagt ttgttttagg taaattattg ttttgtatta 2220
ttaagaagtt tttgatgatg ataggtggaa gtgataggta ttttatcgat agaagttggt 2280
aaaaattagg tttagtggta tatatttgta attttagtta ttcgggagat ttatttgagt 2340
ttaggagttt aattacgttt agtttgagta atatagtgag attttatttt taaataaaga 2400
ataaaaagaa tgtgtaagat tttttgtaga tcgttgtata gttaattata gacgataaat 2460
atttattaaa gtaataagaa taacgtttgg aaaaatggtg taataaaaga tgtttttttt 2520
tttgtgttta taattgttaa aagttatagt tttgtttttg aaaaatttta gtattaatat 2580
tattttggat tggggtgtat ttttttagaa aagaatgtat gtgtttagtt aaagtttaga 2640
taataatata aagtaaataa agataatgta aatatagagt aaagtaaaat tgatatatat 2700
ttaataaata attattgatt ttttgttata ttttatgtat tgtgaatagt tatgatgggg 2760
ttttttaaaa aaagttatgt taagtagggt ttaattttaa tagttaataa attgatttat 2820
ttaaaatata tttattgaga gtttattaaa gtattagata tagttttgga tgtcgattaa 2880
ttattggtag ttatttagtt ttttatgttg gaattatggt ggggaagggt atagggaagt 2940
aaagatattt tttttatgta tggatatgtt ttgtgttttg ttttgtttag ttttatgtag 3000
gatgattttt tttaggtaaa taaaattggt tatattaaaa tagagagata taataaagag 3060
aatgaaagta taattgttat taataggtta tagagtataa agtgttatgt attaaatgaa 3120
ttgtaagaag ggttgatttt agttgtattt atattaagga tgtatgataa aatttatgtg 3180
ggttaggaag taaaattatg gtttggttgt ttgagatgag atgggaaatg ttaaaggtaa 3240
aggtatataa ataaaaaaat atatatatag gtagatatga atatagtatt ttttaggtta 3300
gataagagat aggtttggtt gattgattta tgaatattag aatataatgg ggggttagat 3360
agggtagtgt acgtatgtgt gagagagtat gtgtatgagt aatagagaga tagttaagga 3420
agagggaggt aaaaagatag gagttttaag attagaattt tgaatataat attatatatg 3480
attaggtttt tggttttttt ttatttattt tttaattatt attttattta attgtattta 3540
attatattta attttttttt attttttaag tattttaagg ttatttttat gtgaggtttt 3600
tgtatttgat gttttttttt ttaaattttt ttttttcgta atttaacgta gtatatttat 3660
ttaaattaat ttagttgttt aaaggttatt ttttgaaaga ggaatttttt gattatttga 3720

ttgaaaatag tatgatagtt tatttttata atatttttta ttttttgatt ttgtttattt 3780
tttatagtat ttattttaat ttgatattat attattaatt ttgtttattt attttgtttt 3840
ttttaatgta atgttagtat gtaggtagtt tcgtttgttt ttaaaattat tggatgtttt 3900
ggatttagaa tagtgtttat ttattaaata aatgagtaag aaaaataatt tgtaaaaata 3960
tatatatttt tttaaggatg tttatattaa atttaaaata aaaaaattgt tatttataga 4020
tatttatgat attagagaat aaaaatatgt ttggaaatga aatatattag atttagtttt 4080
ttaagttgat ggtattatag gtttgagtta ttgtgtttat tagattttgt tttttattag 4140
tattattttt ttaaagttta ttaatttttt tgtaaaatag agatgatggt ttttaagtta 4200
tagaaatatt aaagtattaa tgtttattta atataagttt tataaattta atagtattat 4260
ttaaatatga attttaatta ttagtattta gaaagaagac gaattaagat ttaagagatt 4320
ggttagattt agttttaatt taattttgtt tattattttt ttattatata tattttttga 4380
tttaatttaa atttttgttt ttttgtttat ttgtgttttt taagagaaat tttagttatt 4440
tttaatgatg agttttaatt tatttttttt tgtgaagatt ttttttatta tttaatgttg 4500
aagaggttat ggttattgaa tatttataat aaaaattaat taataagtta taaataatat 4560
tataattttt taaatatagt ataaaatttt tttaagaagt agtttttagt atttggatta 4620
ttgtatgtgt tatattatat attatgatat ttaatgaatg gttattgatt tttttattaa 4680
ttgtattttg aaattaagag ataattttaa attttaaatt tacgttgatt aagataatga 4740
taaaatgaga atttatttta tagaattaaa aatatttaga aggtagtttt gttagtagaa 4800
atggtggtaa attttattgg gaaaaggtta tgattggaga tataggttta gttaggtaaa 4860
gatgagaaat agatttaaga ttttgtataa agagttaaaa aaaaaaagtt tgtaatagag 4920
tgaaaagaat agtaagtaaa tatttaaata ttacgaaata tttattgtta ggaaagagtt 4980
ttttgatagt gagttattga taatgtatta ttaaaaagat agaattagat ataatggtat 5040
tgtagaagat taaatatatg ggagagttta aagagaatgt aaataaagaa aaggttagtg 5100
attttggtga atgggtatgt tattaaattt taagagattg gttttaaaaa gaataatagg 5160
tgttagtttt aaaaatttag aatttttatt agtaaaatta gtataggaga ggagtaaatt 5220
tttatattta attttttaaa tatggaatga ttaaaaaatt ttagtatttt tgatgaaatt 5280
atttttaaaa atgtttttta tattttttaa attggtaaag tggtatgtgt aagttattcg 5340
agtatttgtt atttacgttt taatttatat tataaaagga gaaaaattta ttataaaaat 5400
tagtgaatag ttaaaagatt tgaatagatt ttttataaaa gaggatatta aaatagttat 5460
tggatgagaa aatgggttta atattattag ttattatatg aatgtaaaaa gaaaataaaa 5520
attattatag taaaatttta ttcggtattt attagaatgg ttatatttaa aaaaaaaaaa 5580
aagattaatt gtttatgata atatagaatt ggaattttta tatattgata gtaggaatat 5640
atattagttt agttattttt gaaaattgtt taatagtatt tattaacgtt aaatatagat 5700
ttttatgatt tagaaatttt atttttaggt atatatttaa tagaaatgag tgttgtgttt 5760
attaaaaaat aagtatatgt ttttaataat tttatttata atagttttaa attggaattt 5820
aaatatttat tagtagtaga gtgggtaaat attggtaata aatttataaa atggaatatt 5880
ttatagttat agaaagaatt agtatgtgta aaatatggag aatttttata tattttgagg 5940
gaaagattat atattatttt atttatataa tatttaaaaa taggtaaaat taatttatgt 6000
tattaaggtt agaagagtat taagaagggt tatgagatag ttttttaggg agttagaaat 6060
tttttatatt ttgatttggg tgatgatgat atggatgtat attatatgta gaagtttatt 6120
aagttatata ttttaatttt gtgtgtttta ttagatgatt attttaattt ttaaaaatat 6180
ttttaaaata tatttgtgat tgattattta taaaattagt ttttatttat aagtgttttt 6240
tttattataa tggtttatga attatgagtg agattataga ttttgtaaaa attttaagat 6300
ttttaaagtt tataaaaatg atggtaaata atgtaggatt taggatttga ttttatttta 6360
tttataagtt agtttgttat tgttttttgg atggtgttaa aggatataag atttttgggt 6420
tagagatttt gttattttgg gtatagtaag ta 6452

<210> 293
<211> 6120
<212> DNA
<213> Artificial Sequence

<220>
<223> chemically treated genomic DNA (Homo sapiens)

<400> 293

gtttatagat tagtcggttt ttgagttttt cgggttgggg ttgttttatg tgagttaatt 60
ttttaggagt ttgggtggtc gtagatatta attaatttat tattttcgaa tgtgtaattt 120
aaggttagat aagtggtttt ttgcggtaaa taggattttg gggatttagg tttttttagt 180
attgagaagg tttttggttg tatggggagg gtttaaagtt tgtggttaag ttaggatgta 240
gaagataggt gtgtgtttta gtaagttagt ttcgttgatt attagtttta tatagtatat 300
aggtgcgtgt tgttatttgt gtaggtggga agttcggatt gtttgggttt agggtttttt 360
ggttttttat gttattttag tattatttag tgattgtttg ggttttatgg ttttagtttt 420
tatagttgat aggatagagg ttgatgatag cggtagttcg tgttgatggt tagcgttttt 480
gggttaagga ttttgtgtag attgattgtt tgtattttcg ggaatttggg ggagaaggag 540

ttttgttttt ttattgaggt acggagattt tgggtaattc gttaaggata tggggataag 600
agatagagcg tagatatatt tagagttttt tgtagttttt ttattatgtt attgttttag 660
gttatattta gatagttttt tgtaggagag aaaattttgg tagaggggta ggaaatattg 720
ttattgtttg ttgagtttat tgatgtatta ttgggtttta tttttaaggt agtgtttatg 780
ttattgtttt tatgttatag aggatgatat cgaggtttag agaggattat agaagttgtt 840
gagtggtgtg gttagtttgg ttttggtgtt gagcgaaaag gatggagatt tttttaaggg 900
aaggggagga gaagacgggt agaggggaaa gaagaaagga gggcgaggga aggaaattta 960
taagagaata aagtgtgggg atatggaaaa ttgaagtggg taaagtgata atgataggtt 1020
agtgggagta ggtgggtagg ttggtgggag gaggtgggtt ttttttgaat tttttagggt 1080
ttttgatgga tatgtaggtt tttgttgttt ttgttcgggt attcggtttt taattaggtt 1140
ttagggagga tgtaaaaggg gttttttttt aggttaggtt gagggttttt ttgtaaatat 1200
ttttgagggt atttgaggtt tataggtagg taggatttgt atagttaggt aaagtttgtt 1260
ttgtatagtt atattttgta ggtttaggtt aggtgttttg tgagtatgtt tatggaagta 1320
gtatatttgg tatagggtgg gttggttgag tagttttttt gagtgttttg agagttagtt 1380
ggtggttatt ggtttttttt tcgtagttgt tttggagggt attaatagta aggttaggga 1440
ggtaaatcgt ggggagtagt agatttgttt tttattttaa tagttcgatg ggggagtaga 1500
gggtagtggt agatagatgg gaaattgagg tatcgattat tttgttaata gtgtatatag 1560
ttagttttag agtttgtgtt tttaattatt atgttatatt gtttatggga gggatttgga 1620
tttttgttta cgtttatttt gagaggtttc ggttagtagt tttaggaatg ttatttagtt 1680
ttttagtatt attaggatcg agttgcgttt ttgaatgtgt ttttttttat ttgtaagtta 1740
cgaggtattt ttagagttag agttttttgt tattgtattt agttttattt taattatgtt 1800
ttttagtgta ggtagagatt tttatgttat ttgggttaga ggatatagag tatttcgttc 1860
gtttttcgtt aggtaaattt gtataggtat ggtttagatt tttgtttatg atgggttttt 1920
tttttttttt ttttttttgt gttgcgtttt ggtgttttgg ggttagattt gaggtgtttt 1980
aggtaatttt tatttgttta gaagtaatga atgattttta agtttttata tgttatagga 2040
agatggtgtg agtcgtttgt ttattgagta ttttattttg tattggggta gagttttgaa 2100
aatattattt ttttatagtt attagggaag gatattgttt tagtttttat ttgagagatg 2160
ataaaattga agttgaggag taatgtatgt aatgttatat aattatgaat ggtaaaattt 2220
aaatttaggt ttaattattt taagtagatg tttgtattag aattttttgg ggaatttttt 2280
tttttttttt agagatagcg ttttattgtg ttgtttaggt tggtttcgaa tttttggttt 2340
taagtgattt ttttgttttg gttttttaaa gtattgggat tataggtgtg agtttttagt 2400
ttggtttttg aatttttttt ttaatatgga tttttaggga gtgggggatt taggatgtgt 2460
gtttttataa aagttgtttt gaagattttt taaggggatt tgcgggtagg gataggaagt 2520
attgattata ttgtgatgtg ttttttttta gtttttagag agttcgcgat ttttagtagg 2580
gtagtgtggt tagggtgttc gagtttaggt ttaggaaggt ttaatggtta taatttatta 2640
agggtttttt gtttgtcggg tattgggttt tatttttata tgtatttttt tttttagttt 2700
tatttttttt tttgaaaggg agatgtttat agttggggaa attgaggttt agagagatga 2760
ttaaggggag ttaagaaaga gaggtagagt tttttgtagg ttttttttat tttatggatt 2820
gagagaagtt agggttagga tttttgggta gttaaagatc ggtacgtttg ttttgtattt 2880
tatttggttt ttattttttt ttgtgagatt ttagggggtg gtttcgtttt ttttttgttg 2940
ggtaaggagt ttttttttgt tgtttgatgt tttggatatt gagtttatat ttaggtaggg 3000
ttgaattttt ttttttcgta gtttttgagg ttagtaggtt ttgggtttta taattgtttt 3060
ttgttgagcg attttttggt tagatttttg ggcgtgtacg tagtagggag gtatgtattt 3120
gtgtgcgtgt gtgtttacgt atgggaatat aggtgtatgt atgtatgtat gtgtataggg 3180
tatatttatg tgagggtttt ttagattttt ttttaaggag taaatgatat ttttttatag 3240
tgttaggtgt ttatttagga tttttaaggg tgagggtgga gcgagagcgt ggaaaggttt 3300
aaggtaggtt aggtaggagt atagttttag atggtttttt aagatggtta tatagagttt 3360
agttagatat taatgttagt taagggttgg ttagggtggt tttcgggagg tgtaggggtg 3420
gggttattta agtttattta tttttttttt tatttattta ggaggtaatt ataggagttt 3480
gtttgtagtt aaaagatgtt tttagttttt ttttatttat taggtttttt ttttgggtat 3540
aggtttgagt gtgtagttta ggtagttatt ggtattagag atggaatttg tttgaattta 3600
agggagtttt ttgtggttaa ttttagtttt ataaattgga agggtgtggt aggtagtcgg 3660
agtttagtgg tttatagtgt tttttttggg ttggtaggtt tgtgtttcgg ttatttgttt 3720
tttagtaagt taggtttgga tattacggtt ggttgagtgt aagttagttt ttgttttggt 3780
agtttttttt tttgtttgtg agggtgattt tattttgatt ttatatttgt agtgatgtgt 3840
agattattta tttagttttg agtttagtta ggtaagaggg cggggttttg tatatagtaa 3900
gtaagggagc ggttggttta agggtgtttt gggtttaggt tttttggttt ttttttttta 3960
agggtttatg ggtttttttt tttttatttt tttgcgttta agtgtaggtt agattttaaa 4020
aagtagtcgg ggatggggcg agaatagagg atttttttat tgagtttata tttaatttta 4080
tttagaattt ttttttaggt ttagttgttg ttttttatta ggggtaaatg tgtttatgcg 4140
tattattttt attatttttt tgggggaaaa ttggattttt tttttttttt cgttattcgt 4200
ttttcgattt tgttagttta ttttttgaga gtttttttgt gtaagggatg taagggttat 4260
tagtattagg tttttgaaag gacgtcggag ttagttttcg gtttttaagg gttggattag 4320
tgttggaggg gggaattata aattgttttt ttaggatttt atttttttta ttattttagg 4380
atgttgaaaa aggttatttt gttttttttt ttttgtttag ttttttttcg ttttttgttt 4440
tcggttttag tagaggtcgc gtttttattg cgtttttcgt ttttgagaaa tggcgtagta 4500

agcgtttcgc ggagagtttg gaggcggggc gttttttttt gagttcgcgg ggtcgtattt 4560
cgagttagtc ggttagattt gtatttcgcg tagtattttt gttttttttg tgtagcggaa 4620
agggtaaaag gtagggattg taagcgggcg cgtatcgggt aggaagagcg gttttgcgta 4680
ggtgcgcgga ttcgggtttt tgggttttat tttcgtcgcg tatttcgggg ttcgtattcg 4740
gtttttgtcg ggtttttttc ggtcgtattt cgttttcgta tttcgttttt ttttaagttt 4800
tattcggttt aagtcgcgcg tttcgtattg agtttttata cgcgtcgcgt ttcggtcgta 4860
tttcgcgtag ttagagtaag tattggagtt tcgttttttt tcgtatttta ttcgttttcg 4920
gtttcgtttg gtttattttt ggatcgtttt cgtttcgttt cgtttttatt cgttttttcg 4980
gcgtagtcgg cgtttatttc gttttagttt cgcgatcgaa gtagggcgcg tagtagcgtt 5040
gagtgtttcg gaacgtgcgt cgcgttttta gtgttcgtcg cgttcgtcgc gtttcgggcg 5100
gggatggggc ggttagattg agcgtcgtat tcgttattta gattcgtcgg ttttagtcgt 5160
agttttttta gtcgtggttt tagcgcgtac gggcgatggc gaaggcgacg ttcggtgtcg 5220
tttttattac ggttgtacgt ttttttattt ttggtgaata gggttttagg tttaagatag 5280
tagtattttt gtgtgttttt gttatttgta tatttatatt gtttgttata gtatgtggta 5340
ttgtttattt tttggtttgt gttttttatt aaagtgtaag ttttaggagg gttgggatta 5400
agtttatttt atttattatt ttatatttag atatttattg agtgatttat atatttgtag 5460
gtttaatttt tttttttttt ttgagcgtta tatttaaaaa ttagattgtt attgggcgta 5520
gtggtttatt tttgtaattt tagtattttg ggaggtcgag gtgggtagat tatgaggtta 5580
agagattaag attattttgg ttaatatagt gaaatttcgt ttttattaaa aatataaaaa 5640
ttagttgggc gtggtgatat acgtttgtag ttttagttat ttgggaggtt gaggtagaag 5700
aattgtttga attcgggaga tggaggttgt agtgagttaa gattgtatta ttgtatttta 5760
gtttggtgat agagtgagat ttcgtttaaa aaaaaaaaaa aaaaaaaaaa ttagattgtt 5820
tcgtagatgc gtatgttttt tttttaaagg tattttaaat tattgtgttt aaatttgtat 5880
taattttttt ttgattttat tttagttagt gattttatat gtatgtatgt atatatatat 5940
atatatatat agatatatat atatattata tagtattata ttgttattat ttgtttattt 6000
ttttgttttt tttattagat tggttttgtt tttaaaattg agattatgtg gtattttttg 6060
tattatataa ttgatacgaa ggtattcgat tttttttagt tgatttaaat taatagaaat 6120

<210> 294
<211> 6120
<212> DNA
<213> Artificial Sequence

<220>
<223> chemically treated genomic DNA (Homo sapiens)

<400> 294

atttttattg gtttaggtta attaaaagaa atcgagtgtt ttcgtgttag ttatgtgata 60
taaagagtgt tatatggttt taattttgag gatagggtta gtttagtgaa ggagatagaa 120
aaatagataa ataataataa tatagtgtta tgtgatatat atatatattt gtatatatat 180
atgtgtgtat atatatatat atataaaatt attgattaga gtgaagttaa agaaaaattg 240
gtatagattt aaatatagtg atttgagatg tttttgagag aaaagtatgc gtatttacga 300
ggtaatttga tttttttttt tttttttttt tttagacgga gttttatttt gttattaggt 360
tggagtgtag tggtgtaatt ttggtttatt gtaattttta tttttcgggt ttaagtaatt 420
tttttgtttt agttttttaa gtagttggga ttataggcgt gtgttattac gtttagttaa 480
tttttgtatt tttagtagag acggggtttt attatgttgg ttaggatggt tttgattttt 540
tgattttata atttgtttat ttcggttttt taaagtgttg ggattatagg agtgagttat 600
tgcgtttagt ggtaatttga tttttaagta tggcgtttag gagagagaag aaggttagat 660
ttatagatgt gtgagttatt tagtaaatat ttgggtataa aatggtgaat aaaatagatt 720
tggttttaat ttttttgagg tttatatttt agtaggaagt ataaattagg aaatgggtaa 780
tgttatatat tgtgataagt agtatgaatg tataggtgat aagagtatat agaagtattg 840
ttgttttaga tttgaggttt tatttattag gggtgaaaga acgtgtaatc gtggtgaaga 900
cggtatcgga cgtcgttttc gttatcgttc gtgcgcgttg gggttacggt tggagggatt 960
gcggttaggg tcggcgggtt tggatggcgg gtgcggcgtt tagtttggtc gttttatttt 1020
cgttcggggc gcggcggacg cgacggatat tgggggcgcg acgtacgttt cggggtattt 1080
agcgttgttg cgcgttttgt ttcggtcgcg ggattgaagc gaggtaagcg tcggttgcgt 1140
cggaggagcg ggtaggggcg gggcggggcg ggggcggttt aggggtgggt taggcggggt 1200
cggaggcggg tggggtgcgg gaaggggcgg ggttttagtg tttgttttgg ttgcgcgggg 1260
tgcggtcggg gcgcggcgcg tgtaggagtt tagtgcggga cgcgcggttt gggtcgggtg 1320
gggtttgggg aggagcgggg tgcgggagcg gggtgcggtc gaaaagggtt cggtaggagt 1380
cgggtgcgga tttcgaggtg cgcggcgggg atggaattta ggagttcggg ttcgcgtatt 1440
tacgtagagt cgtttttttt attcggtgcg cgttcgtttg tagtttttgt tttttgtttt 1500
tttcgttgta tagagagggt agggatgtta cgcgggatgt aggtttggtc gattggttcg 1560
gggtgcgatt tcgcggattt agagaagggc gtttcgtttt taggtttttc gcggagcgtt 1620
tgttgcgtta ttttttagag gcgagaggcg tagtgggagc gcggtttttg ttggggtcgg 1680

aggtaggagg cggggaagag ttgagtaggg gaagagaggt agaatagttt tttttagtat 1740
tttggggtag tagagagggt agggttttgg aagggtagtt tgtgattttt ttttttagta 1800
ttggtttaat ttttggaaat cggaaattgg tttcggcgtt tttttagaga tttggtgttg 1860
gtggtttttg tattttttat ataggagaat ttttaggaga tgggttggta gagtcggaga 1920
gcgagtagcg aggaggagga aagaatttaa ttttttttta aagaaatgat ggaaatgatg 1980
cgtataaata tatttatttt taatagggag tagtaattgg atttgagaag gggttttggg 2040
tgaggttgga tgtgggttta gtggggaagt tttttgtttt cgttttattt tcggttgttt 2100
tttgaggttt ggtttgtatt taggcgtaga gggatggaag aagggaagtt tatgggtttt 2160
tgaggaggaa aagttaggga atttgggttt agggtatttt tgagttagtc gtttttttgt 2220
ttgttgtgtg tagaatttcg tttttttgtt tggttgagtt tagggttgaa taagtgattt 2280
gtatattatt gtagatatga agttaaagtg aagttatttt tataggtagg agggggagtt 2340
gttaagatag aggttggttt gtatttagtt aatcgtgatg tttagatttg gtttgttgag 2400
gggtaggtgg tcggggtata ggtttgttag tttaggagag atattgtggg ttattggatt 2460
tcggttgttt gttatatttt tttagtttat ggagttagag ttggttataa ggggtttttt 2520
tgggtttaga taggttttat ttttggtgtt agtggttatt taggttatat atttaggttt 2580
gtgtttaggg aagggatttg ataggtaggg aggggttggg ggtatttttt ggttatagat 2640
aagtttttgt gattgttttt taggtgaatg aagagagaag tgggtaggtt taaatagttt 2700
tatttttgta tttttcgggg attattttgg ttagtttttg gttgatattg atgtttggtt 2760
gggttttgtg tggttatttt aaggggttat ttggggttgt gtttttgttt agtttatttt 2820
aggttttttt acgttttcgt tttattttta tttttggggg ttttgagtga atatttgata 2880
ttatgagagg gtgttattta ttttttggaa aaaggtttgg ggggttttta tatgggtgta 2940
ttttgtatat atgtatatat gtatatattt gtgtttttat acgtggatat atacgtatat 3000
agatgtatat ttttttgtta cgtgtacgtt tagggatttg attaaagggt cgtttagtag 3060
aggatagttg tgaggtttag agtttgttgg ttttagggat tgcggggaaa ggagatttag 3120
ttttgtttgg gtgtaggttt agtatttaga atattaggta gtaggaagga gttttttgtt 3180
tagtagaggg gaagcgaggt tattttttga ggttttataa ggggaggtgg gggttaggtg 3240
aagtgtaaag tagacgtgtc ggtttttggt tgtttaggag ttttggtttt ggtttttttt 3300
agtttatggg gtggggaggg tttgtagggg gttttgtttt ttttttttga ttttttttag 3360
ttattttttt gagttttagt ttttttagtt ataaatattt tttttttaga agaaggggtg 3420
aaattagaaa gaggggtgta tgtgagggta gagtttagtg ttcgataaat aggaagtttt 3480
taataagttg tagttattgg gtttttttgg gtttgggttc gggtattttg gttatattgt 3540
tttgttgagg gtcgcgggtt ttttgggagt tggaggaggg tatattatag tgtggttagt 3600
gttttttgtt tttgttcgta ggtttttttg gagaattttt agggtagttt ttatgaagat 3660
atatattttg ggttttttat tttttaaaaa tttatgttaa aaaaaaagtt taggggttag 3720
gttaagggtt tatatttgta attttagtat tttgggaggt taaggtaaga agattatttg 3780
aggttaggag ttcgagatta gtttgggtag tatagtgaga cgttattttt aagaaaaaaa 3840
aaaaaatttt ttaagaggtt ttggtgtagg tatttgtttg gagtgattag atttgagttt 3900
gaattttgtt atttatagtt gtgtgatatt gtatatatta ttttttagtt ttagttttgt 3960
tattttttaa atggggattg aaatagtgtt tttttttggt gattgtgagg agatggtatt 4020
tttaaggttt tgttttagtg tagggtaaga tgtttagtgg gtaggcggtt tatattattt 4080
ttttgtagta tgtgagaatt tggaggttat ttattgtttt tgagtaggtg aagattattt 4140
ggagtatttt aggtttgatt ttaaagtatt aaaacgtagt atagggaagg gagggaggag 4200
ggaaatttat tatggataga gatttgggtt atgtttgtgt aggtttgttt ggcgggaggc 4260
gggcgggatg ttttgtgttt tttgatttag gtgatatggg ggtttttgtt tgtattgaga 4320
agtatggttg gagtgaggtt gagtgtagtg ataggaaatt ttggttttgg gggtgtttcg 4380
tgatttgtag gtagggggaa atatatttaa gagcgtagtt cggttttggt gatattggaa 4440
ggttgggtgg tatttttgga attgttgatc gaggtttttt agagtgggcg tgggtagggg 4500
tttaggtttt ttttatgggt agtgtgatat ggtggttgga agtatagatt ttgaggttgg 4560
ttgtgtgtat tgttagtaaa gtggtcggtg ttttagtttt ttatttgttt gttattgttt 4620
tttgtttttt tatcgggtta ttgaggtgag gggtaggttt attgtttttt acggtttgtt 4680
tttttggttt tgttgttggt gttttttagg gtagttgcga ggaggaagtt agtgattatt 4740
agttagtttt tagggtattt agaggggttg tttagttagt ttattttatg ttaaatatgt 4800
tgtttttatg agtatgttta tagaatattt ggtttgggtt tgtagggtgt ggttgtgtag 4860
gataggtttt atttaattgt gtaggttttg tttgtttgtg ggttttaggt gtttttaggg 4920
gtgtttgtag aagagttttt aatttggttt gggagaagat tttttttgta ttttttttgg 4980
ggtttgatta ggagtcgggt gttcgaataa aagtaatagg ggtttatata tttattagag 5040
attttggggg gtttagagaa agtttatttt tttttattag tttgtttatt tatttttatt 5100
aatttattat tattattttg tttattttag ttttttatgt ttttatattt tgtttttttg 5160
tgaatttttt tttttcgttt tttttttttt tttttttttt gttcgttttt tttttttttt 5220
tttttggagg aatttttatt tttttcgttt agtattagag ttaggttagt tatattattt 5280
agtagttttt gtggtttttt ttgggtttcg gtgttatttt ttgtgatatg agagtaataa 5340
tatgggtatt gttttgaggg taaagtttaa tgatgtatta gtgggtttag tagatagtgg 5400
tagtgttttt tgtttttttg ttagggtttt tttttttata gggggttgtt taggtgtggt 5460
ttggggtagt agtatggtgg gaaggttgta gggggttttg agtgtgtttg cgttttgttt 5520
tttgttttta tgtttttggc gggttattta gagttttcgt gttttagtgg gaaggtagag 5580
tttttttttt ttaagttttt cggggatgta gatagttagt ttgtatagag tttttagttt 5640

agggacgttg gttattagta cgagttgtcg ttgttattag tttttgtttt gttagttgta 5700
ggagttgagg ttatgaggtt taagtagtta ttggatggtg ttagggtggt atagaaagtt 5760
agggggtttt ggatttaaat agttcgggtt ttttatttgt ataggtgata gtacgtattt 5820
gtgtgttgtg tggggttggt ggttaacgag attggtttgt tagagtatat atttgttttt 5880
tgtattttgg tttggttata ggttttgagt tttttttatg taattagaag tttttttagt 5940
gttggaggga tttgggtttt tagagtttta tttgtcgtag aggattattt atttgatttt 6000
gaattatata ttcgggagtg gtgggttgat taatgtttgc ggttatttag gtttttgggg 6060
gattggttta tataaggtag ttttagttcg aggggtttag aggtcggttg gtttgtggat 6120

<210> 295
<211> 6620
<212> DNA
<213> Artificial Sequence

<220>
<223> chemically treated genomic DNA (Homo sapiens)

<400> 295

tttgtgaggt ttttttttta attttttaaa aggttatttg aaatagtaag ttagtaagtt 60
aaattttaat tttagggtta gtataagtat tgttatacga tgtaatagtt tggtgaattg 120
tgataataat tttaaaaagt tatttatagt taggtgagat ggtttatgtt tgtagtttta 180
gttttttggg aggttgagtt ggtagggtag tttgagttga gtttagtttg ggtaatatag 240
tgaaatttat tttttaagaa aaaagaaaat tagtgaatta ttattacgat gtaattgttt 300
attttttagg ttgggtgtag tggtttatgt tcgtaatttt agtattttgg gaagttgagg 360
taggtagatt gtttgagcgt acgagtttga gaatagtttg ggtaatatgg taaaatttta 420
tttttataaa aaatataaaa gttagttagg cgtagtggcg tgtatttgta gttttagtta 480
ttcgggaggt tgaggtacga gaattgtttg aatttaggag gcggaggttg tagtgagtta 540
aacgtattat tgtattttag tttgggtaat agagtaagat ttttgttaaa aaaaaaaaaa 600
ggtagtattt tttatattaa aagggtattt agagttgaag ttttaatgtt ttagttataa 660
aggaggtaga aagtttgggc gtgtggttta tgtttgtaat tttagtattt tgggaggtcg 720
aggtaggagc gtaggaggat tatttgagtt taggagttta agattagttt gggtaatttg 780
tttttttaaa aaaaaaacga ataaataaaa ttagttgggt atggtggtgt atgtttgtag 840
gtttagttat ttgggagata ggtaggagga ttaattgagt ttagaagttt gaggttgtag 900
tgagtttgtt tgtgttattg tattttagtt tgggtattag agtaagattt tgttttaaaa 960
gaggaaaaaa aaaaaggagg tagaaaaaat gtttattaat ttttgtagtt tttattaaaa 1020
taaaaagatt attttggtat ggttatgata gaaattttaa attattttag ggtttagagt 1080
ggtatttttt tgaagttggg gaaaggattt tgggtagaaa gtaaaggagg gggtagattt 1140
ttggttggtt tgatgttttg taattttggg tttgagttat ataagagtat ttagtgagtt 1200
aaggtttaaa ggaggaggga ataattattt tttgggaaag ggttatataa tttggaatat 1260
tatagatttt ttagacgcgg agttgagatt agagtgtttt ttatcgtttg atgaaataat 1320
ttagtttatt ttaaggacgt gtatttggaa aaatgattgg tttttggaaa tagtgttatt 1380
ttgttattta ggaataattt tgttgattga gttttaataa ggaaaatagg ttttaattaa 1440
ttatattttt taagtgttag aggattcgtt agataataat tttattataa taataagagt 1500
atttattgag tgtttatttt agggtagtat tttatattta ttatattatt taattaagaa 1560
gatttttatt tggtattaat ttaatattat tttggttttg ttagttgtgg ttttatttag 1620
gattttgttt aaaatttaga gaaatttgat tataagaaaa tttaataagt tataaaatat 1680
tatatttttt tttttattgt attgttttgg ttgtttaaaa aaatggttta gaaatagtat 1740
tgtattaatg tttatatttt tattttggtt attgtattat agttatgtac ggtattaaaa 1800
taagtggtat aaaggatatg aggtaataat ttgtattaaa tttgtaattt tttgtaagtt 1860
tataattaga taaaaataaa gttgaataat agtttattat ttttttaata aattatttat 1920
tttattttat tatggtaaaa agaaatatat gtttaatgta gtaaatattt tatatatata 1980
taagtaaaaa gaagaaaatg aaaatttttt aatttaatta gttagaggta tttttattaa 2040
atatttaggt atatttttat taagatgtgt gtatatggat atatagtatg tatgaatttt 2100
aattattgat tatatttgta aatgtaaaat tttatatttt tttattgaat ttaatttttt 2160
ttaaattttt atttgtttta atggtaggta atttaattta gttattttg aatagtttag 2220
ttattttaag atttaaagtt atttaaattt agtttttaga tttagttttt ttcgttttat 2280
ttattaggga ggttatattt ggattttaat tatttaatgt agttatatat tgtagatgta 2340
atttatttta ttttatcgtt ggggattagt attttgtttt tgtttgttag aattataatt 2400
ttgataatgt tttttatttt ttttagggtt agaggtttat aaggttattt cgggtttttt 2460
taatagagtt atattagtta ttttgatttt tttattgttt tggtatattt ttagtttgta 2520
aggttttttt tgagatttgt agaatattga ggttattttt tgggaaatgt ggtatagttt 2580
tttttttttg tgtaatttat agaaattttg ttagattttt tagtattttt tttttgtgta 2640
gattggttta gtgtgatggt attttaggtt ttttttttaa gatttattta agggatttat 2700
tttgtaaatt tttttgtttg atatttttga aaatataata taattgaatt tttatttttt 2760
ttggagtgaa agaagcgaat aaataagtaa gtttttatat tattttattt tttttagttt 2820

atgagttatg atataagttt aaaagttttt gtattgaagt tagattttta gatatttaga 2880
tttttttttt aaagtttttt tttgttgttt ggtttttgtt tttatattaa tggttttaaa 2940
attttatttt gtaaagtgtt aaaaattttt ttttttgaga aggagtttcg ttttgttgtt 3000
taggttggag tgtagtggtt tgatttcggt ttattgtaat ttatgttttt ttggtttaag 3060
cgattttttt gttttagttt tttaagtagt taggattata ggtgcgtgtt attacgtttg 3120
tttaattttt gtattttttt agtagagtta ggtttttatt atgttggtta ggttggtttc 3180
gaatttttga ttttaggtga tttatttaat tcggtttttt aaagtgttgg gattataggt 3240
gtgagttatt gcgtttggtt agttttaagt ttgaaagagt tatgttttta tatttggaat 3300
tttttttttg tttagtgagt atgatttttt tatttttata aaatattaat ttgagtaatt 3360
tttggtatat tttttaagaa tttatttaaa atttggtata ttaaaagttg aattgtattg 3420
ttatttgttt taatatattt taagaagaat atataataat taaaataatt tgaaatgtta 3480
ttgtttttat tttttttatt aattttggtt aaatgaaata atatattgtt ttaaatttta 3540
ttgtttattt atggtttatg attagagatt attatagttt atttacgata atgaataaat 3600
gtgttttgaa aagttgatta tcggtttggt acggtggttt atgtttgtaa ttttagtatt 3660
ttgggaggtc gaggcgggcg gattatttga ggttaggagt ttaagattag tttggttaat 3720
atggtgaaat ttcgttttta ttaaaaatat aaaaattagt tgggcgtggt ggcgggtttt 3780
tgtaatttcg gttatttcgg aggttgaggt aggagaatcg tttgaattcg ggaggcggag 3840
gttgtagtga gttaagatta tttttattgt atttagtttg gggggataga gtaagatttt 3900
attttaataa aaaagaaaaa aaaataaaaa aaagaaaaaa gaaaagttga ttatttaata 3960
ttattgtgtt gataggcgtg taatatggat ttttttgtga cgcgtaagta ttatttggta 4020
gttatatggt gtcgtttgtg tagttgggcg ttattattag ttttttgttt tgtgcgatta 4080
aaaggttagt ttcggggata gtagtttttt taacgtaaag cgattagttt tgttaggcgt 4140
cggttgtagt tggtttaagg ttgacggttt tagggtcgta tttttttttc ggttatttta 4200
gattttggaa agagagttat ggagggtttt agagcgtttt tttgtagttt tttgaggaaa 4260
ataagggttt tttttttttg gttggagtag ttaagtgttt acggggaaaa tggtttcgtt 4320
tattatttta ggtaaacgtt tttatagttg taataaatat gtgatgggtt ttttgtatta 4380
agaaaggaaa atttttttta aaaataataa aaagaggtgg ggaggggatt ttaagtttta 4440
tttaagattt tttaaattcg ttgtgtttta tgtatacgtt gaggtttaat atgaaaattt 4500
ggagcgaaag tttttacggt ttattatagg gaatgaagta aagtatttcg tcggtagttt 4560
taggtttcgt aaatcgtgga gtttttttag tataaggttt tattatgtgt tatattagat 4620
gagaagaaaa taagaggaag gatacggtag agtttttaga atggggttta gttttaggat 4680
tcgtaggata aaggaaggtc gttttttttc gtttgaggta gggaattaaa taaaatatcg 4740
gtatcgtagg tatcgtagtc gtatttttgg ggtttcgtgg ttttttcgcg cgttcgtttt 4800
gggggcgggg aagaattcgg atggaattat ttttgtagtt cggatttttc gtcgtttttg 4860
aatggtagcg gggcggagtt gttttttttt ttgtgttttg attggtttgg tttcgtattt 4920
tttttttcgt gattgggttt ttttagtgtt agttatagag cgacgttggt tttttagatt 4980
gttgtagaag gagacggcgt cgacgtttga ttggattcgc ggcgatttat tttttagtcg 5040
tgcgtttttg attcggcgtt cggaatttgt tttcggtttt agggttgcgg ggtttggaag 5100
gaggcgtatc gaggcggttc gaaaacgatt taggggagtc gaggcgtttt ttttgttatt 5160
ttatttagcg ttatgttttg gatgtttaag aggtgaaggg ggcggagggg gtggggcgtt 5220
cggtttaacg gtttggaggc gtttttaaat gaatttgatt ttttcggcgt ttttttgcgt 5280
tttttgggcg atttgtgtag ttgtattcgt tttgttggtc gtatatgtgg tcgtcggaga 5340
aataaatgta ttgttttcgt tggcgagtag ggtttttttag ggcgagtttc gtgttaggga 5400
tttgggaaat ttttgttatc gattgtgggg ggtttggggt ttaaagtatt tcggttagtg 5460
ttttatttaa taaaatattt attagatatt tattgtgcgt taggtattgt gttcggtgga 5520
gggtcgagta agatagtttt tgtttttaag tggtttatag tttatttgta tgtatagacg 5580
tgtagataag tatttgtaga ggcgttagta gagatatgcg ttatttgtaa ggaaaacgta 5640
tcgtaaataa gcgattgttt ttaggagaat ggaaattttt tgtaatgttt tagtagattt 5700
ttttttattt tttattgttt aaattggtat ttatggttat tttcgaaata gttatattta 5760
atgggtttgg ggttatgttt aaatcggcga cggaaggcgg gtacgggtgg tttggggtta 5820
attttttacg gtggcgtggt ttggatatta gaataggtgt tttgttcgta gggagttata 5880
gtgatggatg ttggatgtat tttttggaaa attataaata atttttggta tattaatagt 5940
tgtattttta taagtttttt atatagttta atttttattg aaattttggt atatgatttt 6000
attttttaaa ttattttttt gatatggagt ttcgttttgt cgtttaggtt ggagtgtagt 6060
ggtataattt cggttcgttg taatttacgt ttttcgggtt taagtgattt ttttgtttta 6120
gtttttcgag tagttgggat tatatatggg cgttattacg tttggttaat ttttgtattt 6180
gtagtagata gggttttata atgttggtta ggttggtttt aaatttttga ttttaggtga 6240
tttatttgtt tttgtttttt aaagtgttgg gattttaggc gtgagttatc gtattcggtt 6300
tataattgta gttatagata ttatatatat attaatttag atgattttta ttgggaaaga 6360
ggttagtgaa ttttgattag gttttttatt ttgaatattt agggggtagg tagaagagtt 6420
aatattgaaa ataggtagag tttttagaag ttttttttat ttattgtaat ttttgtttta 6480
acgagagtaa ttattaattt ttatcgtgat ttttatgtaa gattttattt gttgaagtaa 6540
agtatgagaa aattatttat atggtttttg gaaatattta aatattatat tgtttttttt 6600
tgtttttaaa aaaaaatagg 6620

<210> 296

<211> 6620
<212> DNA
<213> Artificial Sequence

<220>
<223> chemically treated genomic DNA (Homo sapiens)

<400> 296

tttatttttt tttaaaggta aagaaaaata atataatatt taagtatttt taaagattat 60
atgagtagtt tttttatgtt ttattttagt aaatgaaatt ttatatggga attacggtaa 120
agattaatag ttattttcgt taaagtagga attgtagtga gtggagaagg tttttaggaa 180
ttttatttgt ttttaatatt ggttttttta tttgtttttt aggtatttaa aatgaaaagt 240
ttagttagag tttattaatt ttttttttag tgaaaattat ttaaattgat atgtatatga 300
tgtttgtaat tataattatg ggtcggatgc ggtggtttac gtttggaatt ttagtatttt 360
gggaggtaga ggtaggtgga ttatttgagg ttaggaattt gagattaatt tggttaatat 420
tgtgaaattt tgtttattat aaatataaaa attagttagg cgtggtggcg tttatatgta 480
attttagtta ttcgggaggt tgaggtagga gaattatttg aattcgggag gcgtaggttg 540
tagcgagtcg agattgtgtt attgtatttt agtttggacg atagagcgag attttatgtt 600
aaaaaaataa tttaaaaaat gaaattatat gttaaaattt taatagggat tgagttatat 660
aaaagattta taaaaatata attattaatg tattagaagt tgtttataat tttttagaaa 720
atatatttag tatttattat tgtaattttt tgcgagtaga atatttattt tggtatttaa 780
attacgttat cgtgggaaat tggttttaag ttattcgtat tcgtttttcg tcgtcggttt 840
aagtataatt ttaaatttat taggtgtaat tgtttcggga atgattataa atattaattt 900
ggataatgaa aagtgaaggg gagtttattg gagtattgta aaaagttttt atttttttaa 960
gaatagtcgt ttatttgcga tgcgtttttt ttgtaagtgg cgtatatttt tattagcgtt 1020
tttgtaaata tttatttgta cgtttgtgta tatagataag ttgtgagtta tttggggata 1080
aggattgttt tattcggttt tttatcgagt ataatgtttg gcgtatagtg ggtatttaat 1140
ggatgttttg ttaaataaag tattggtcga gatgttttaa gttttaggtt ttttatagtc 1200
ggtgatagag attttttaag tttttaatac gggattcgtt ttaggagttt ttattcgtta 1260
gcgaagataa tgtatttatt ttttcggcgg ttatatatgc gattaataga acgaatatag 1320
ttgtataaat cgtttaggga acgtagagga acgtcgggaa ggttaggttt atttggggac 1380
gtttttaggt cgttagatcg agcgttttat ttttttcgtt ttttttattt tttgaatatt 1440
taggatatgg cgttgagtgg gatgataaga ggagcgtttc ggtttttttg gatcgttttc 1500
gagtcgtttc gatacgtttt tttttaggtt tcgtagtttt gaagtcgggg ataaatttcg 1560
agcgtcggat taggagcgta cgattgaaag gtaagtcgtc gcgagtttag ttagacgtcg 1620
acgtcgtttt tttttgtaat aatttgggag attagcgtcg ttttgtgatt ggtattagga 1680
aagtttaatt acgaagagga gagtgcggag ttaaattagt tagagtatag aagggagggt 1740
aatttcgttt cgttgttatt taaagacggc ggggggttcg ggttgtaagg gtggttttat 1800
tcgggttttt tttcgttttt aaggcgggcg cgcgggaaag ttacgaggtt ttaggagtgc 1860
gattgcggtg tttgcggtgt cggtgttttg tttgattttt tgttttaaac ggagggaaac 1920
gatttttttt tattttacga gttttaagat tgaattttat tttaaaggtt ttatcgtatt 1980
ttttttttta tttttttttt atttaatgtg gtatataata gggttttata ttaaaggagt 2040
tttacggttt acgagattta gagttatcgg cgagatattt tattttattt tttgtggtga 2100
atcgtgggga ttttcgtttt aaatttttat gttaagtttt agcgtatgta tgagatataa 2160
cgagtttgga aaattttaaa tggaatttag agtttttttt ttattttttt ttgttatttt 2220
taaggaaaat tttttttttt tggtgtagga aatttattat atgtttatta tagttatggg 2280
ggcgtttgtt tgaaatggtg gacgggatta tttttttcgt gggtatttgg ttgttttagt 2340
taagaggggg aggtttttgt tttttttaag gaattgtaga ggggcgtttt gaggtttttt 2400
atggtttttt ttttagagtt tgaggtgatc ggaaggagaa tgcggttttg ggatcgttaa 2460
ttttggatta gttgtagtcg acgtttggta gggttggtcg ttttgcgttg aggaggttgt 2520
tgttttcgaa gttggttttt taatcgtata gggtaggaag ttggtggtgg cgtttagttg 2580
tataggcggt attatgtaat tgttagataa tatttgcgcg ttatagagag gtttatgtta 2640
tacgtttgtt agtataataa tattaggtgg ttagtttttt tttttttttt tttttgtttt 2700
tttttttttt ttattgagat ggagttttgt tttgtttttt aggttggagt gtaatggggg 2760
tgattttggt ttattgtaat tttcgttttt cggatttaag cgattttttt gttttagttt 2820
tcggagtagt cgggattgta ggagttcgtt attacgttta gttaattttt gtatttttag 2880
tagagacggg gttttattat attggttagg ttggttttga atttttgatt ttaggtgatt 2940
cgttcgtttc ggttttttag agtgttggga ttataggtat gagttatcgt gttaggtcgg 3000
tagttagttt tttaagatat atttgtttat tatcgtaaat aaattgtagt gatttttaat 3060
tatgaattat ggatgagtaa tagaatttga aataatgtat tattttattt gattaaagtt 3120
gatgaggaag ataaagataa tggtatttta aattatttta attgttgtat gttttttttg 3180
aagtgtgttg aggtaaatgg taatatagtt tagtttttag tatgttagat tttaaataaa 3240
tttttggaaa atatgttaga aattgtttaa attgatgttt tgtaagagta agaaagttat 3300
gtttattaga taaagaaaaa attttaaata tgagaatata gtttttttag atttaagatt 3360
ggttaggcgt aatggtttat atttataatt ttagtatttt gggaggtcga gttgggtgga 3420

ttatttgagg ttaggagttc gagattagtt tggttaatat ggtgaaagtt tgattttatt 3480
aagaaaatat aaaaattaag tagacgtggt ggtacgtatt tgtagtttta gttatttggg 3540
aggttgaggt aggagaatcg tttgaattag ggagatatgg gttgtagtga gtcgagatta 3600
agttattgta ttttagtttg agtaatagag cgagattttt ttttaaaaaa aaaggttttt 3660
aatattttgt aaaataaaat tttaaagtta ttggtgtggg ggtagggatt aggtagtagg 3720
gaggggtttt aagagaagaa tttgaatatt tagaaatttg gttttaatat agaaattttt 3780
aagtttatat tataatttat agattgaaga aagtaggatg atgtaagagt ttgtttgttt 3840
gttcgttttt tttattttaa aggagataaa agtttaattg tattgtattt ttaagaatgt 3900
taggtaggag ggtttgtaga gtaaattttt taagtgggtt ttagagagaa gatttagaat 3960
attattatat tgggttaatt tgtataaagg gagaatgtta gaaagtttag taaagttttt 4020
gtagattata taagaaaagg aagttatgtt atatttttta gagagtggtt ttagtatttt 4080
gtagatttta gagagaattt tatagattag aaatgtgtta aggtaataaa ggagttaagg 4140
tgattgatgt ggttttgtta aggggattcg ggtagttttt gtgagttttt agttttggga 4200
agggtggagg gtattattag gattatggtt ttagtaaata aaagtaggat gttggttttt 4260
aacggtgagg tgagatgagt tatatttgta atgtgtgatt gtattagatg attggagttt 4320
aaatataatt tttttggtgg gtaagacgaa gggaattaaa tttgaaagtt gaatttaaat 4380
gattttaaat tttaaaatga ttgaattatt tagaagtgat taaattaggt tatttgttat 4440
taggtagaat gaaggtttaa gaaaaattaa gtttaataaa gaaatataaa gttttatatt 4500
tgtaaatata gttagtgatt gaaatttata tatgttatat gtttatatgt atatattttg 4560
atgaagatat atttagatat ttaatagaaa tatttttagt tggttaaatt agggaatttt 4620
tatttttttt tttttattta tatgtatata aaatatttgt tatattaaat atgtattttt 4680
ttttgttata ataaaataaa atgagtaatt tattaaagaa atgatgaatt attatttagt 4740
tttattttta tttaattgta gatttataga aaattataaa tttagtataa attgttgttt 4800
tatatttttt atgttattta ttttaatatc gtatataatt atagtataat gattaaaatg 4860
aggatatgaa tattggtata atattatttt tgaattattt ttttaaataa ttaagataat 4920
gtagtggaaa agaaaatata atattttgtg atttattaaa tttttttata attaggtttt 4980
tttagatttt ggataaaatt ttgagtaaaa ttatagttga tagggttaaa gtgatattaa 5040
attaatgtta agtaaaaatt tttttaatta aataatataa tgaatatgaa gtgttgtttt 5100
ggagtaagta tttagtaagt gtttttattg ttgtaataga gttattgttt aacgagtttt 5160
ttagtattta agaaatatgg ttgattgagg tttatttttt ttattgaggt ttaattagta 5220
gggttgtttt taaatagtag aataatattg tttttagaag ttagttattt ttttaggtgt 5280
acgtttttga agtagattga attgttttat taagcggtag gagatatttt aattttaatt 5340
tcgcgtttga gaaatttgtg gtgttttaga ttatatagtt tttttttagg agataattgt 5400
tttttttttt tttaggtttt gatttattaa atgtttttat gtggtttaag tttagggttg 5460
taaggtatta ggttagttag ggatttgttt tttttttttgt tttttgttta gggttttttt 5520
tttagtttta gggaagtatt attttaggtt ttggaatgat ttagggtttt tgttatggtt 5580
atattaaagt aattttttta ttttaatgaa aattgtaagg attaataaat attttttttg 5640
tttttttttt tttttttttt ttttaagata gggttttgtt ttgatgttta ggttggagtg 5700
tagtggtata aataagttta ttgtagtttt aaatttttgg gtttaattga tttttttatt 5760
tgttttttaa gtagttgggt ttataggtat atattattat gtttagttaa ttttgtttgt 5820
tcgttttttt tttggagaga taggttgttt aggttggttt tgaatttttg ggtttaagtg 5880
atttttttac gtttttattt cggtttttta aagtgttggg attataggta tgagttatac 5940
gtttaggttt tttgtttttt ttatggttag aatattaaaa ttttaatttt aaatgttttt 6000
ttgatataag aaatattatt tttttttttt tttgataaga gttttgtttt gttgtttaga 6060
ttggagtgta gtggtgcgtt tagtttatta taattttcgt tttttgggtt taagtaattt 6120
tcgtgtttta gtttttcgag tagttgggat tataggtgta cgttattacg tttggttaat 6180
ttttgtgttt tttgtagaga tggggttttg ttatgttgtt taggttgttt ttaaattcgt 6240
gcgtttaaat agtttgtttg ttttagtttt ttaaagtgtt ggggttacgg gtatgagtta 6300
ttgtatttag tttagaaagt gagtaattat atcgtagtag taatttattg gttttttttt 6360
tttttagaaa ataggtttta ttatgttgtt taaattagat ttaatttagg ttgttttatt 6420
aatttagttt tttaaggagt tgggattata ggtatgggtt attttatttg gttgtaaatg 6480
attttttaaa attattatta taatttatta aattattgta tcgtatggta atatttatgt 6540
taattttaaa attaaggttt aatttgttaa tttgttattt taaatagttt tttagaaagt 6600
taagaaaaaa attttataaa 6620

<210> 297
<211> 5832
<212> DNA
<213> Artificial Sequence

<220>
<223> chemically treated genomic DNA (Homo sapiens)

<400> 297

ttttatttta gtgattggat tcggaaagaa aaggattttg ggttttattt tggttcgtta 60

tttttcgttt tttttttttt ttaagtaaag ttattttatt tttttttaat aaattttata 120
tttaaaaagt tgatttacgg atatatcgta tgtttttcga ttttgagtcg tcggtttttt 180
tgtttcggtt atttattcgt cgagtcgttg gtaaagtttg ggttgcgttg ggttgtttcg 240
ggtcgtagga acgttcgagg ggaagagggt cgggcgggcg gcggcgtagg gtttcggagg 300
taggcgacgg tcgtagcggt atagaagacg gcggaaggcg ttgcgaaagt tcgggttgcg 360
gtagtagatg agcgggttga aggtagaatt ggtataattt agttagttta gggtaaggaa 420
agtcgggttc gggattagag aggggttttt tagggcgcgt agtacgttgg ttagaaagaa 480
gggtaattag tagagagtga aggtgtttat gatgagattt aaggtgtata gggttcggtg 540
ttttcggaga ggtaggaggc gcgcgggtcg tcggtcgtag gcgggtattt tttcgggcgg 600
agcgtacgtt tttatcgggg tcggggttag agagcgcgac ggcgtcggcg gagatttttc 660
gggcggaaag cggtttagtt tttcgcgtag taagcgtagt tggcgcgtag ttattacgaa 720
aattcgcgcg tagacgaaga gtattacgag aagaggaagg tagaaggaga cggaggagga 780
tagtagtacg tagggtatgt tggaggcgaa ggtatagtag cgcgggttgg agtggtagcg 840
ttgcgtttcg gcgtcggttt ttacgcgtta ttattggttt atgatgggcg taaacgatat 900
cgcggtcgat acgatttata ttaggattat agttgttcgg gcgtagcgtt tggtgattag 960
tgcgtcgtaa cgtagcgggt tggttatagt taggtagcgg tttacggtta gggcgtatag 1020
ggtttcgatg ttggcggtta tatatagtac gtttatcgag gtttatagtt cgtagttagt 1080
ggcgtttaac ggttagtggt tagttagcgt taaggtggtc gtcggcggta ttattaggag 1140
ttttattatt aggtcggttg cggttagcga agttacgaat acgttggtta tggtttggag 1200
tttcggagtt taggcgatgg ttacgatgat tagtaggttg ttttttacgg tggttagtat 1260
cgttagcgtt agtagggttt cggttagggt cgttttttac ggaatttttg gtagtttatt 1320
ggtgttggcg gtattgggcg ttagggtggg gaggttcggt tatggggtaa gagagttgtt 1380
ttcgtgaggt tacggagtta ttttcgggtc gcgcgtgggg cggtagggaa agaaggaagg 1440
agggggtttt ttaaattatt tggtttaggg gaggggatag taaggtatga gagcgatttt 1500
tttagtttgg gttatttttt ttagttgttt tagttagagc gtttagtttt tttttatttt 1560
aattttttcg gtgttatcgt tttgggggga ggagtagcga cgtttaaagg ggtagttgtt 1620
tatagtttgg aggggttttt ggagtcgaat tggagagtgg ggaggatggt taatgggatg 1680
ttttacgttt tttttagtta aatttggagg aagggatatc gtggtttttt tgttattatt 1740
tgtttatttt tgtcgtggtt aggtttttgg atttagtata gtattttttg ggtattggtt 1800
ttttggagtt ttgtattttt ttttttagaa gtttttatag taaggtaggg gaaggagaga 1860
ggagagcgtt tttattttta ttttttatgt attagaggta aaggtaattc gttggagtta 1920
agtaaaggat ttataggagt ttcgtattta tttgggtggt tttttttaag gtattttaaa 1980
ttaagtaata gggtttagtt agtatttttt tttttaagat tttagtttgg tgagatttgt 2040
tttttttttt ttttgtttta gttatttttt tttttttttt agttttttgt tttttttaat 2100
atttttaggt attgaggaaa tttattatat tttaagtttt tttttttttt tatttttttt 2160
aggttggttt tgaattttcg ggtttaagtt atttttttgt tttagttttt tgagtagttg 2220
gaattgtagg cgtgaattat taggaggttt aggagttttt tttttttttt tttttttttt 2280
tttttttttt tttttttttt tttgagacgg cgtttcgttt tgttatttag gttggaggtt 2340
ggagtgtaat gatgtgatgt ttgtttattg taatttttat ttttcgggtt taagcgtttt 2400
ttttgtttta gttttttaag tagttgggat tgtaggtatt cgttagtatg tttaattaat 2460
ttttatattt ttagtagaga cggggtttta ttatgttggt taggatggtt tcgatttttt 2520
gatttcgtga ttcgttcgtt tcggtttttt aaagtgttgg gattataggc gtgagttatc 2580
gcgtttggtt atgttatttt ttttttttat attttattag ttagaagtaa gttattgagt 2640
ttagtttata taattaaggg gaggggggta ttaaagaatt tgtgtggagg tatgatggtt 2700
tatttttgta attttagtac gttggaaagt ttaggtaaga ggatcgtttg agttcgggag 2760
ttggagatta gtttgagtaa tatgatgaga ttttgttttt attaaaaatt aagaaaatta 2820
gttgggcgtg tcgggagtag tggtttacgt ttgtaatttt agtattttgg gaggtcgagg 2880
tagcggatta taaggttagg agattgagat tattttggtt aaaaaacggt aaaaattttg 2940
tttttattaa aaatataaaa aattagttgg gcgcggtggc gggcgtttgt agttttagtt 3000
attcgggagg ttgaggtagg agaatggtgt gaattcggga ggtggagttt gtagtgagcg 3060
gagattgtat tattgtattt tagtttgggc gatagagtaa tatttcgttt taaaaagaaa 3120
aaaaaaaaaa agaatattag ttgggcgtgg tggttcgtgt ttatagtttt tgttatttag 3180
gaggttaagg taggaggatt gttttattcg gggaatttga ggcggtagtg aggtatgatg 3240
tcgttattgt tgtattttag tttgagcgat agtgcgagat tttgttttag aaaaaaaaaa 3300
aaaaaatata taggtgaaat ttcgttttta ttaaagatat aaaaattagt taggcgttat 3360
ggcgcgtgtt tgtaatttta gtaattcggg aggttgaggt aagagaatcg tttgaattcg 3420
aaatcgcgag gtggaggttg tagtgagttg agatcgcgtt attgtattta gtttgggcga 3480
taaatcgaga tttcgtttta aaaaaaaaaa aaaaaagtcg tgcgcggtgg tttatatttg 3540
taattttagt attttgggag gtttaggtgg gtggattatt tgaggttagg agatcgatat 3600
tagtttggtt aatatggtga aatttcgttt ttattaataa aaattagtcg ggcgtggtga 3660
tagatatttg taattttagt tattcgggag gttgaggtag gagaatcgtt tgaatttggg 3720
gtggaggttg tagtgagtcg tgatcgggtt attgcgtttt agtttgggcg atagagcgag 3780
atttgttttt aaataaataa taataagaaa atataaaatt ttgcgattat atgttaaaat 3840
tgttataata ttacgtattt gggaggagat atttagatta agtattttgt ttttttttgg 3900
agtattaatt tttgatttaa gtatttatta ttaattttaa aaatgtaatt tggtaattta 3960
gttaggtttt tttttaattt tgttgaaaat taataattga gaattaattg ggaaattaat 4020

atttacgtat tttttatggc gtttagtggt ttagagattt ttatattatc ggtggatata 4080
ttatagtaag attagattga ggattggcga ggggtgcgtc gtttttgtgt aaagcgggag 4140
aagagtgtat tatcgttagg taatatttta ggatatagga taaatattgg tgttttgaaa 4200
acgtttttgg tcgtaatatt tttagaggtt tgtattttcg agtttggaga ttatggtttt 4260
gaaagattta atttatttta ttgatgttag ttattagtta ttgttgatga ttttttaagt 4320
tcgtattttt agtttatatt tttttttttc gttgggtatt aaatttgttt atatttcgag 4380
tgtttcggta tatttcgggg tttcgttttt cgttttttat agtaggaagc ggcgagtcgt 4440
ttttagtgtt tacgtcgttt ttcggttgtt cggtttgggt tttttatttt cgtttttgga 4500
atcggttttt ttagattttt ttttttttta aaattatttg gaaacgggtt ttaaggggcg 4560
attttgcgta gaaaattaaa ttttattcgg ggcgggtagg gagtgttaga ttttaaggaa 4620
gacgtttttt atattcggtg gtgaggagtg tttggcggta cggtacggtt agcgttttta 4680
aggatttttt tttgcgtaga atatttagtc gtagttacga aaatacgtcg cgttagtttt 4740
tgattcgaaa ggaagttttt tagagagtcg tattaattat ttagtttttt ttatttttag 4800
tttgttaggt tcgcggttat cgtcggcgcg ggaggtcggt ttatttttgt tcgcgcgttt 4860
ggttttaaag cggtcgtgga attatgtttt tttaggtagg cggtggacgt atcgcgggtt 4920
ttttttgttt gaagattata gcgttatata gtaacgcgcg cgagagaaga gagtattttc 4980
gcgagaagtt taggggtggt cgtgatggcg gcggtaggag taggatttgg ttaggaagcg 5040
ggtgtcgggt ttggtttagg agcggtcgta aatgtaatag gggtagaaga gggggagatg 5100
aagtcggtgg tagcgggagt agtcgttttt tttggagagg ggatttttgt tgtttcgata 5160
gttgagttta gtttcgggga ggttgaaggc gggtaagagg ttttgtcgtt cgaggaagac 5220
gcgggaggga ggttcgtttt tcgcgaattt cgcggttttt ggagttttgt cgttcgtttt 5280
ttgcgaattt aggtttcgtc gttaatggtt cgttttgttt ttgcgtcgtt tggttcgttt 5340
ttttttaagt atatttcgga taacgttttt ttcgtatttt ttacgggcgg tgggagttga 5400
ggtttttgtc gttatttttg atttttgtat tttggtagga agttggtagt ttatatttta 5460
acgggaggtt tttatatatt ttagaaaaga aattattttg tagtgttaga ttggaagaag 5520
taacggttat tttgaaaata gggtgggaga gttgtttttt tttgaatttt ttttaggatt 5580
aattttaatt taggtagatt tgattgtaaa ggtcggttag gtttttttgt gtttcgtggg 5640
ttcgcgattg tttgatatgt ttattttttt agtattttga tagtattttg ggtatttagt 5700
ttgagattag gtttagttga aaaagttgta ggagtatgaa gttcggttgt aatttgatta 5760
ttatttttgg gaataggtat tttgaggttt gttttgagaa tttttatttg agcgttttta 5820
ttattttata gg 5832

<210> 298
<211> 5832
<212> DNA
<213> Artificial Sequence

<220>
<223> chemically treated genomic DNA (Homo sapiens)

<400> 298

tttataagat aatgaaagcg tttaagtaag aatttttaag ataaatttta aaatgtttat 60
ttttaaaaat gatgattaaa ttatagtcga attttatatt tttgtagttt ttttagttaa 120
gtttgatttt agattggatg tttaaggtat tgttaggata ttgagaaggt ggatatgtta 180
gatagtcgcg gatttacgga gtatagggaa gtttaatcgg tttttatagt taaatttatt 240
tgggttagag ttggttttgg gagaggttta aagagaggta gtttttttat tttgttttta 300
gagtgatcgt tatttttttt agtttggtat tgtaaagtgg tttttttttt ggaatatgtg 360
aaggtttttc gttaaagtgt gagttattag ttttttgtta gggtgtaagg attagagata 420
acgataggag ttttagtttt tatcgttcgt gaaaggtgcg gaaggggcgt tattcgagat 480
atgtttgaga ggggacgggt taagcggcgt agaggtaggg cgagttattg gcggcggggt 540
ttgggttcgt agggggcggg cggtagggtt ttaggagtcg cgggattcgc gaggggcggg 600
tttttttttc gcgttttttt cgggcggtag gattttttat tcgtttttag ttttttcgga 660
attgggttta attgtcggag tagtagagat ttttttttta ggaggagcgg ttgttttcgt 720
tgttatcggt tttatttttt ttttttttgt ttttgttgta tttgcgatcg tttttgggtt 780
aggttcggta ttcgtttttt ggttaggttt tgtttttgtc gtcgttatta cggttatttt 840
tggatttttc gcgagaatat tttttttttt cgcgcgcgtt gttgtgtgac gttgtagttt 900
ttaaatagaa aaagttcgcg gtgcgtttat cgtttgtttg aggaagtata attttacgat 960
cgttttaagg ttaggcgcgc gggtagaggt gggtcggttt ttcgcgtcgg cggtggtcgc 1020
gggtttggta ggttgaaggt ggaggaagtt agatggttag tgcggttttt tggaggattt 1080
tttttcgagt tagggattga cgcggcgtat tttcgtggtt acgattggat attttgcgta 1140
gaagagggtt tttgagagcg ttggtcgtgt cgtgtcgtta aatatttttt attatcgaat 1200
gtgggaaacg ttttttttgg agtttagtat tttttgttcg tttcggatgg aatttggttt 1260
tttgcgtagg gtcgtttttt ggagttcgtt tttaaatgat tttaagaggg aagggaattt 1320
agggaggtcg atttaggggg cgaggtggga aaatttaggt cgggtagtcg aggagcggcg 1380
tgggtattag aagcggttcg tcgtttttta ttgtgagagg cggaaagcga agtttcgggg 1440

tgtgtcgggg tattcggaat gtagataagt ttggtgttta gcgaaaggaa gaggtgtgga 1500
ttgaaagtac gaatttggga agttattaat agtagttggt ggttggtatt agtggagtga 1560
gttgagtttt ttagagttat ggtttttaaa ttcgagaatg taaatttttg agagtgttgc 1620
ggttaggaac gtttttagga tattagtgtt tattttatat tttaaaatgt tgtttaacga 1680
taatatattt ttttttcgtt ttatataaga acggcgtatt tttcgttaat ttttagttta 1740
attttattgt gatgtgttta tcgatagtgt aggaattttt gggttattgg acgttatggg 1800
aaatacgtag atattgattt tttaattggt ttttaattat tggtttttaa taaaattgaa 1860
gaaagattta attgagttgt taggttatat ttttaaaatt gatgataaat atttaaatta 1920
ggggttggta ttttaaagag aagtaggatg tttgatttaa gtattttttt ttaaatacgt 1980
agtattgtag tagttttaat atatggtcgt agaattttgt gtttttttgt tgttgtttgt 2040
ttgagataga gtttcgtttt gtcgtttagg ttggagcgta gtggttcgat tacggtttat 2100
tgtaattttt attttaggtt taagcgattt ttttatttta gtttttcgag tagttgagat 2160
tataggtatt tgttattacg ttcggttaat ttttgttagt agaggcgggg ttttattatg 2220
ttggttaggt tggtgtcgat tttttgattt taagtgattt atttatttgg gttttttaaa 2280
gtgttgggat tataggtgtg agttatcgcg tacggttttt tttttttttt tttgaaacgg 2340
agtttcggtt tgtcgtttag gttgagtgta gtagcgcgat tttagtttat tgtaattttt 2400
atttcgcggt ttcgggttta agcgattttt ttgttttagt ttttcgagtt gttgggatta 2460
taggtacgcg ttatgacgtt tggttaattt ttgtattttt agtagagacg gggttttatt 2520
tgtgtgtttt tttttttttt ttttgagata gagtttcgta ttgtcgttta ggttggagtg 2580
tagtagtggc ggtattatat tttattgtcg ttttaagttt ttcgggtaaa gtaatttttt 2640
tgttttagtt ttttgagtag tagggattat aggtacgagt tattacgttt agttaatatt 2700
tttttttttt tttttttttt tgagacggag tgttgttttg tcgtttaggt tggagtgtag 2760
tggtgtaatt ttcgtttatt gtaagtttta tttttcgggt ttatattatt tttttgtttt 2820
agtttttcga gtagttggga ttataggcgt tcgttatcgc gtttagttaa ttttttgtat 2880
ttttaataga gatagggttt ttgtcgtttt ttagttagga tggttttaat tttttgattt 2940
tgtgattcgt tgtttcggtt ttttaaagtg ttgggattat aggcgtgagt tattgttttc 3000
ggtacgttta gttaattttt ttaattttta gtagagatag ggttttatta tgttgtttag 3060
attagttttt aattttcgag tttaagcgat ttttttgttt gggtttttta gcgtgttggg 3120
attataggag tgagttatta tgtttttata taaatttttt aatatttttt tttttttgat 3180
tgtgtgggtt ggatttagtg atttgttttt aattaataga atgtagaaaa agaaaataat 3240
atggttagac gcggtggttt acgtttgtaa ttttagtatt ttgggaggtc gaggcgggcg 3300
gattacgagg ttaggagatc gagattattt tggttaatat ggtgaaattt cgtttttatt 3360
aaaaatataa aaattagttg ggtatgttgg cgggtgtttg tagttttagt tatttgggag 3420
gttgaggtag gagaagcgtt tgaattcggg aggtggagat tgtagtgagt agatattata 3480
ttattgtatt ttagttttta gtttgggtga tagagcgaga cgtcgtttta aaaaaaaaaa 3540
aaaaaagaaa agaaaaagaa aaagaaaaag aaaaagattt ttgagttttt tagtggttta 3600
cgtttgtaat tttaattatt taggaggttg ggataggagg gtggtttgag ttcgggagtt 3660
taaagttagt ttgggaaaaa tagagagaga gagagatttg aaatatggta aattttttta 3720
gtatttgaaa atattaaaaa aaataaaaag ttagaaggaa agagaaggta attgaaatag 3780
aagggaaagg ggatagattt tattaagttg aggttttggg agaggagata ttggttgagt 3840
tttattattt aatttaaaat attttagggg aggttattta agtggatgcg gggtttttgt 3900
gaattttttg tttgatttta gcgggttatt tttgtttttg atatataaag ggtggggatg 3960
ggagcgtttt tttttttttt ttttttgttt tgttgtggga atttttggga aaggaggtgt 4020
agggttttag gaagttagtg tttagggagt gttatgttga gtttaggagt ttggttacgg 4080
taggggtgga tagatggtgg tagaggaatt acggtgtttt tttttttaga tttagttaaa 4140
ggaaacgtgg agtattttat tggttatttt ttttattttt taattcggtt ttagaggttt 4200
ttttagatta taggtagttg tttttttaag cgtcgttatt ttttttttaa gagcggtggt 4260
atcgagggag ttggggtggg gggaggttga gcgttttggt tgggatagtt agagaagatg 4320
gtttaggttg gggaagtcgt ttttatgttt tgttgttttt ttttttgagt taggtgattt 4380
gggagatttt tttttttttt ttttttttat cgttttacgc gcgattcggg gatggtttcg 4440
tggttttacg agaatagttt ttttgtttta tggtcggatt tttttatttt ggcgtttaat 4500
atcgttaata ttagtgggtt gttaggggtt tcgtgggagg cggttttagt cggggttttg 4560
ttggcgttgg cggtgttggt tatcgtggga ggtaatttgt tggttatcgt ggttatcgtt 4620
tggatttcga gattttagat tatgattaac gtgttcgtga tttcgttggt cgtagtcgat 4680
ttggtgatgg gatttttggt ggtgtcgtcg gcggttattt tggcgttgat tggttattgg 4740
tcgttgggcg ttattggttg cgagttgtgg atttcggtgg acgtgttgtg tgtgatcgtt 4800
agtatcgaaa ttttgtgcgt tttggtcgtg gatcgttatt tggttgtgat taattcgttg 4860
cgttacggcg tattggttat taagcgttgc gttcggatag ttgtggtttt ggtgtgggtc 4920
gtgtcggtcg cggtgtcgtt tgcgtttatt atgagttagt ggtggcgcgt aggggtcgac 4980
gtcgaggcgt agcgttgtta ttttaattcg cgttgttgtg ttttcgtttt taatatgttt 5040
tacgtgttgt tgtttttttt cgtttttttt tatttttttt ttttcgtgat gtttttcgtt 5100
tacgcgcggg ttttcgtggt ggttacgcgt tagttgcgtt tgttgcgcgg ggagttgggt 5160
cgtttttcgt tcgaggagtt ttcgtcggcg tcgtcgcgtt ttttggtttc ggtttcggtg 5220
gggacgtgcg tttcgttcga aggggtgttc gtttgcggtc ggcggttcgc gcgttttttg 5280
ttttttcggg aatatcgggt tttgtgtatt ttgggtttta ttatgggtat ttttattttt 5340
tgttggttgt tttttttttt ggttaacgtg ttgcgcgttt tggggggttt ttttttagtt 5400

tcgggttcgg ttttttttgt tttgaattgg ttaggttatg ttaattttgt ttttaattcg 5460
tttatttatt gtcgtagttc ggattttcgt agcgtttttc gtcgtttttt gtgtcgttgc 5520
ggtcgtcgtt tgttttcgga gttttgcgtc gtcgttcgtt cggttttttt tttttcgggc 5580
gtttttgcgg ttcggagtag tttagcgtag tttaggtttt gttaacggtt cgacgggtag 5640
gtaatcgggg tagagggatc ggcggtttag ggtcgggaag tatgcgatgt gttcgtgggt 5700
taattttttg agtgtggagt ttattaagag aaggtgggat ggttttgttt ggagagaaaa 5760
gggaacgagg agtagcgaat taaaatggga tttagggttt tttttttttc ggatttagtt 5820
attagggtag aa 5832

<210> 299
<211> 4604
<212> DNA
<213> Artificial Sequence

<220>
<223> chemically treated genomic DNA (Homo sapiens)

<400> 299

aacggtttaa ggtttcggtt tttttttttt tttttttttt tttttgggg tttttttttt 60
tttttttttt atgagatagt ttcgtttcgt tgtttaggtt ggagggtagc gatattattt 120
tgttttattg taatttttat tttttggttt tagttatttt tttgttttag ttttttgagg 180
aattgggatt ataggtgtat gttattatat ttggttaatt tttatatttt tagtagagac 240
ggggttttat tatgttagtt aggttggttt tgaatttttg attttaagtg atttatttgt 300
tttggttttt taaagtgttg ggattataag tgtgagttat cgtgtttgat ttattttaag 360
ttttttagaa tttgattttt tttacgttta ataaagagta agtattggtt taataggatt 420
aataaggatt aattttttag gtgtttttga gttaaaaaaa aaaattggtt cgtttgtgtg 480
ttattatagt tttagttaaa atatttaata aagttttatt tagttgtgtt tattttaatt 540
aattaagtgt gtgattttgg ttaagttatt taaatttttt gagtttttat tatttagtgg 600
agttataagg tatagtggtt tatatttgta attttagtat tttgggaggt tgaggtagaa 660
gaattgtttg aggttaggag ttttagagta gtttggttaa tatagagaga ttttgttttt 720
ataaataatt aaaaaaaaaa ttatttaggt atggtggggt gtatttgtat aggttttagt 780
tattcgggag gttgaggtgg gagggttatt tgagtttagg agtttaaagt tgtagtgagt 840
tgagatcgta ttattgtatt ttattttggg taatagagtg agattttgtt ttaaaaaatt 900
ttttaaaaga ttaaaaatat aatttattgg ggttattaat aattaaaaag atttgggtta 960
atattttgtt attgttgtta tttgtgtttt tattttggat aagtatgttt ttttttttat 1020
aaattttatt tttttttaaa gatgggggtt ttgttatgtt gtttaggttg gttttgaatt 1080
tcggggttga agtgattttt ttgttttagt tttttaagta gttaggatta taggtatgtg 1140
ttgagttttt gggtaagtaa attaagattt ttgagttata tttgtttttt tttattaaag 1200
agtattgttt tagttgttat aataaaatat tataatttag atggtttaag taatagaaat 1260
ttgttatatt tttggagttt agaagtttga gattagcgtg ttagtatgat tgggttttgg 1320
ggagggtttt ttttttttgc gtttttgttg tgtttttatg tggtggagag agagagaatt 1380
tttttttttt ataaggttat taattttatt atgggggttt tgttttataa attttattta 1440
aatttaatta ttatttaaag gtttttttta aatattatta cgttggggtt ataattttta 1500
atatatgaat ttgggggtga tattatagat attaatgttt attttatagg gtttttataa 1560
ggtttaaggt aggtattaat ataggaaagt atttaaagtt gggtttggtt tgggtaggta 1620
gtttaatgat ttaataaata ttgagtattt atttggagtt aagtattgta tgtgttatat 1680
gaagcgatat tgggaaatga gttatatgta gttaattttt ggttttttgg aggttttgaa 1740
ttagaagggg atacgtatat aatcgtatgt gtgtgtattt atatatatag gtgatatgat 1800
gtatttgaga gaatttagtt taggaattag gaaaattttt aaggagtgat attttagttg 1860
tattttgaag gatgaggaat ggagaggagg ttaattttag gttttaaagt gaatttttgc 1920
gtaaaagtta tgaggtagta aggtgtaggg gtttttaatg atttaaggga acgtattgtg 1980
gggttgggat tatgatggtt agaggaggtt ttgataagag gttagttaaa gagtagagaa 2040
aaattttaag gagttttaag aaagggaagt gttacgatga gttttgtgtt tggaaatgtt 2100
ttaggcggtt atatcgtagt ttcgggattg gttgggattt ggatagatat ttggatatta 2160
gttagaaagt tacgatagga aattaggcga ggacgaggta attggggatg ttgtggagta 2220
gagacggaga gaaaatgggt gtattcgaga taagttaggg ggaaaaaatg taaggatttg 2280
gaaatgaatt tggggcgcgg taggaaggta tgacgggttg ttttgtaggt tttatttgta 2340
aattacggcg attagtgaaa gatttggagg aggaaggtgg atatattttt taataaaaaa 2400
aatttttttt gaaattttat attaatattt taaaagtaaa ttagattttt ttagatatgt 2460
ttttgagttg atatttgtta attagttaga attagaaatt ttttttattt ttatttagtt 2520
ataatatacg ttatagttga ggtgagagga aagaaaaggt tgttttttta ggaataaaga 2580
gtggtatttt tagtatcgtg ggtaaagttt ttttaagttt aatagtagtt aaaatagcgt 2640
tttttataaa taatatttag ttaaaagttt ttgggtttgt gattgtttta acggttaagt 2700
tcggatgagg gtttttggag tcgtagtttt cgggaaacgt cgattggttt tttatttgga 2760
ttttattcgt ttaggtagtt tagagcggtt ttaggtcgtt cgtttttttg ttaattatag 2820

tttcgcgatt gcgtttagtt tttaggtttc gtttttcggt taagcggcgt gtttttattg 2880
tacggcgttt gggtttcgcg cgtcgggatt tcggttttag tcgttttgtt ttgtttcgag 2940
gtttttaggt tcgtttttgg gtttcgcgcg ttaggatttc ggtttcgatc gttttgtttc 3000
gtttcgaggt ttttaggttc gtttttttgt tttcggcgtg ttttcgcggt ttcgttttta 3060
ggattcgcgc gtcgggattt tggtaagttt tagtcgttcg gtttcgtttt ttcggtttta 3120
cggttttcgc ggtttttttt ttaagtttta tacgtcggga ttttggtaag ttttagtttt 3180
tagttttatt tttaggtttc gtttattcgg ttagcggttg gttttcgcgg tttcgttttt 3240
gtgttttgcg agtttttatt ttgggagtat tgcggtcgtc gtgtttcgtt tttttttagc 3300
gcgcgtacgt ttttttgcgg ttcgtcgttt tagacgcggg aagagtttgg tcggtttcga 3360
gtcgttggtt tgtagttttt ttgtgttttt cgaggttttt ttgttttcgt tttgcgagga 3420
gttttttatt cgaaggcggg acgatgtcga ataatcgata gtcgaggaat cggtagtcga 3480
gaaatcgttt cgggaacgag tttcgttttc gttcgttatg gaatcggatg gtcgcggtgt 3540
ttgggtttat agtcgcgtcg cgttcgatcg tttggagaag attgttgttt gttcgcgttg 3600
gtaaagacgg agttttttgg gggtggttgc gagggtacgc gtcgtatagt ttttgggggc 3660
ggtagaattt ttttaaattt ttcgtttttt tttttcgttt tcgcgttgta gtcgggtcgg 3720
cgtgcggtta gtacgtgtcg ggggatatag tattaataat ttttgttttt tatttatttt 3780
atttttgggc gatttatcgg tttttttttg ttttgaattt aattgaaacg gtagtttttg 3840
aattttagcg ggttgaagaa tcgtttggag gtgtggttaa aaaaatgttt atttcggtcg 3900
cgtttttaga gtttgaatcg ggttggggtt ggggttgagg tttttgtatt ttttattcgg 3960
ttttggatta tttcgttgtt tttcgggagt tgtggcgaat tgggttgtcg ggtcgtttcg 4020
gagatttttt aaattagaag tagttgttat tttaagttaa attggttttt ttgatatttt 4080
tttcgtgtta gttttttcga gtgagatggg atggagtatc ggatatttat tatagttgta 4140
gattgaagat ggtacggaat tttttatttt tttagtttgt ttaaaagatt gtatgtttgg 4200
tgttttcgtt tttagtgatg ttgtttattg ttttttttta tgttgttata ttatgggagt 4260
ttttttaggg tttttatttc gtttaaagat atttttatga gtgttatttt ttgttttttt 4320
tcgtgtagtt ttttttagta tataaattaa gaaggtttgt aaaatttaag aaaggtttgt 4380
tttattttta ttttttttga aaagtatttt tattgagtat ttgttgtgtg tatagttaag 4440
tatttttgta tgaagattat tggtagtttt tttgttattt gtagttgtta aaagtttata 4500
ttttttttgg attttttttt tttttgattt ttaatttgta gaatttaagt tatttagttg 4560
tataaagtat aagtaatata attttttttt tttttttttt tttt 4604

<210> 300
<211> 4604
<212> DNA
<213> Artificial Sequence

<220>
<223> chemically treated genomic DNA (Homo sapiens)

<400> 300

aaaaaaggaa agaaaagaaa aagttgtgtt atttatattt tatgtaatta ggtaatttaa 60
gttttgtaag ttagaaatta aaggggaaaa aagtttaaaa aggatataaa tttttgatag 120
ttataaataa taaaaaaatt gttagtgatt tttatataga aatatttagt tgtatatata 180
gtaagtgttt aataaaaatg ttttttagaa gaaatgaaaa tgagataaat tttttttggg 240
ttttataaat ttttttggtt tgtatattag aaagagttat acgaaaaaaa gtaagaagta 300
atatttatga aaatgttttt ggacggaatg aaaattttga gaggattttt ataatgtgat 360
agtatgaagg aaaataataa atagtattat taagagcggg gatattagat atatagtttt 420
ttgagtaaat taagaaaatg agaaatttcg tgttattttt aatttataat tatggtagat 480
attcgatgtt ttattttatt ttattcgaaa aagttggtac ggagaaaatg ttaaaaaggt 540
tagtttaatt tagagtggta gttgttttta atttagaggg ttttcggggc ggttcggtag 600
tttaattcgt tatagttttc ggaaagtagc ggggtaattt agggtcgggt aaaaaatgta 660
gaagttttag ttttaatttt agttcgattt aaattttgga ggcgcgatcg ggatgaatat 720
ttttttagtt atatttttag acggtttttt agttcgttga agtttaaaaa ttatcgtttt 780
agttggattt agggtaaggg gaggtcggta agtcgtttaa aaataaagtg aatgagaagt 840
agaagttgtt aatattatat ttttcggtac gtgttaatcg tacgtcgatt cgattgtagc 900
gcgggaacgg aaggaggaaa cggaagattt gaaaagattt tgtcgttttt agaaattgtg 960
cgacgcgtgt tttcgtagtt atttttaaga agtttcgttt ttattaacgc gagtaggtag 1020
tagttttttt taggcggtcg agcgcggcgc gattgtgggt ttaggtatcg cgattattcg 1080
gttttatggc gggcgggaac gaggttcgtt ttcggagcgg tttttcggtt gtcggttttt 1140
cggttgtcga ttattcggta tcgtttcgtt ttcggatgaa aggtttttcg tagagcggaa 1200
gtaaggaagt ttcgggaaat atagggaagt tgtaggttag cgattcgaaa tcggttaagt 1260
ttttttcgcg tttgggacgg cgggtcgtag aggggcgtgc gcgcgttggg gaggggcggg 1320
gtacggcggt cgtaatgttt ttaaaatagg gattcgtagg gtataggggc ggggtcgcga 1380
gagttagtcg ttggtcgagt gggcggattt taggggtggg gttggaggtt gaaatttgtt 1440
aaagtttcgg cgtgtgggat ttaggggagg ggtcgcgaga gtcgtgggat cgagggggcg 1500

gggtcggacg gttgaaattt gttaaagttt cggcgcgcgg gttttagggg cggagtcgcg 1560
agaatacgtc ggggatagag gggcgggttt aggagtttcg gggcgggata ggacggtcga 1620
aatcgaagtt ttggcgcgcg gggtttaggg gcgggtttag gggtttcggg gtaggatagg 1680
acggttgaaa tcgaggtttc ggcgcgcggg gtttaggcgt cgtgtagtga gagtacgtcg 1740
tttgatcgaa gggcggggtt tgaaggttga gcgtagtcgc gaggttgtag ttggtaggag 1800
agcgggcggt ttgaagtcgt tttgggttgt ttggacggat gaagtttagg tggaaagtta 1860
gtcgacgttt ttcgggagtt acgattttag ggatttttat tcgagtttaa tcgttggaat 1920
aattataaat ttagaaattt ttagttgagt gttatttata aaaagcgttg ttttgattgt 1980
tgttggattt ggaaaagttt tgtttacgat attgaaggta ttattttttg tttttaagaa 2040
agtaattttt tttttttttt tattttagtt gtggcgtata ttgtaattga gtaaaaataa 2100
ggaaagtttt taattttaat tagttaataa atattagttt aaaggtatgt ttgaaaagat 2160
ttggtttatt tttaaaatat tggtataaaa ttttaaaaag ggtttttttt gttagagagt 2220
gtgtttattt ttttttttta gattttttat tgatcgtcgt aatttataga taagatttat 2280
agagtaattc gttatgtttt tttgtcgcgt tttaagttta tttttaagtt tttgtatttt 2340
ttttttttaa tttgtttcga atgtatttat ttttttttcg tttttgtttt ataatatttt 2400
tagttgtttc gttttcgttt ggttttttgt cgtagttttt tagttgatat ttaagtgttt 2460
atttaggttt tagttagttt cgagattgcg gtgtggtcgt ttaaaatatt tttaaatata 2520
aaatttatcg tggtattttt tttttttaaa attttttaaa gttttttttt gttttttgat 2580
tagttttttg ttaaaatttt ttttggttat tatggtttta attttatagt gcgttttttt 2640
aggttattaa aagtttttgt attttgttgt tttatggttt ttgcgtaaag atttattttg 2700
gaatttggaa ttggtttttt ttttattttt tattttttag aatatagttg aagtattatt 2760
ttttaaaggt ttttttagtt tttagattgg atttttttag gtgtattata ttatttgtgt 2820
atgtagatat atatatatac gattatgtgc gtgttttttt ttagtttaaa atttttaaaa 2880
ggttagagat tggttgtatg tgatttattt tttaatatcg ttttatgtgg tatatgtagt 2940
gtttggtttt aggtaggtgt ttagtgtttg ttgaattatt gaattattta tttaagttag 3000
atttagtttt aagtgttttt ttatgttaat atttgtttta agttttatag ggattttatg 3060
aaatgggtat tagtatttgt agtgttattt ttaaatttat atattaaaag ttgtaatttt 3120
aacgtgatag tatttggagg ggatttttgg gtggtagtta gatttagatg gggtttatga 3180
ggtagagttt ttatgatggg gttaatgatt ttgtaagaag agagagtttt tttttttttt 3240
attatatgag gatatagtaa gaacgtaaaa gaagagggtt ttttttaaaa tttagttata 3300
ttggtacgtt aattttagat ttttagattt tagaaatatg ataaattttt gttgtttaag 3360
ttatttagat tatagtattt tgttataata gttaagatag tgttttttaa taagaggagg 3420
taggtgtggt ttagagattt taatttattt gtttaagggt ttagtatatg tttgtagttt 3480
tagttatttg ggaggttgag gtaggagaat tattttagtt tcggggttta aggttagttt 3540
aggtaatata gtaagatttt tatttttaga gaaaagtaag atttgtaaaa agaaaagtat 3600
atttgtttag gatggaggta tagataataa tagtggtaga atattaattt agattttttt 3660
gattattaat ggttttagta aattatattt ttggtttttt gaggggtttt ttgggataag 3720
gttttatttt gttgtttagg atggagtata gtggtacgat tttagtttat tgtagttttg 3780
aatttttggg tttaagtgat ttttttattt tagtttttcg agtagttggg atttgtgtag 3840
gtgtatttta ttatgtttgg ataatttttt ttttaattat ttgtagagat agggtttttt 3900
tatgttggtt aggttgtttt agaatttttg attttaagta atttttttgt tttagttttt 3960
taaaatgttg ggattatagg tgtgagttat tgtgttttgt agttttatta aataatgaaa 4020
atttaaggag tttaaatgat ttggttaaaa ttatatattt ggttagttag aataagtata 4080
attaaataag gttttattaa atgttttaat tgaaattatg atagtatata aacgaattaa 4140
tttttttttt taatttagag gtatttaaga agttagtttt tattagtttt gttaagttag 4200
tgtttatttt ttgttgagcg tgggagaaat taagttttag aaaatttaaa atgggttagg 4260
tacggtggtt tatatttgta attttagtat tttgggaggt taaggtaggt ggattatttg 4320
aggttaggag tttaaggtta gtttggttaa tatggtgaaa tttcgttttt attaaaaata 4380
taaaaattag ttaggtgtgg tggtatgtat ttgtaatttt agttttttag gaggttgagg 4440
tagaaaaata gttggaatta ggaggtgaag gttgtagtga ggtaagatgg tgtcgttgtt 4500
ttttagtttg ggtaacggag cgagattgtt ttataaaaaa aaaaaaaaaa aaaattttaa 4560
aaggaaaaga aagaaaagga aaaggatcga aattttaaat cgtt 4604

<210> 301
<211> 12968
<212> DNA
<213> Artificial Sequence

<220>
<223> chemically treated genomic DNA (Homo sapiens)

<400> 301
   ttttttgtga tttagtgttg ttattaatat ataaggttga tagagtaatt ttaaatttat 60
   aatattatat gtagatattt aaaggaaaga aaaagagttt tatgtgattt tgtttaatgg 120
   gtttatttat agttgtaaat gtatattgta agtgatttat atttgggaat tattatgtaa 180
   taattatttt taataataat tgatataatt tattagaatt tgtgagaggt ttcgatgtaa 240
   ataatttatt tgtttagtag agagaaggga aggagagtaa gtaggggaaa atgtttttaa 300
   aatgttttat tattggaagt aaatgattat aaatgttttt gcgttaagtt agggtttggt 360
   tttaattttt ttttgaattt taatgttttt atatttttta aatatatttt aaatttttat 420
   taaataaatt atattttaat tatatacgga gtttaaaaga ggtgatttta tgatagatat 480
   ttagtattag ttagtgaagg agaatattgg ttgaaggtat ttaaaattac gtgtataagg 540
   aatagtttat tttttatttg gtgatttatt attttaagtt atttttgtgt taaagttata 600
   aaaattttta gtgttaaaaa gtgtaagaag aaatgagtta gtaaaaaaaa aaaagtcggg 660
   ggaaagttaa tttttaaatt tgtattattt ttattttttt atttttgttt attttagaat 720
   gtatatttgg aatgaatttt tttagagacg tttacggtat gttaaatttt aagttatatt 780
   tttaaataaa gtatatattt ttatagatgt gtatatgatt ttatatttat gtatttttat 840
   gtataatttg tgatataatt taattaaata attaaggttg tttttgttga ttttaatata 900
   tttaaaagta gttttttata ttgtaaatgt tatattatat aaaatatttt aatgaaagtt 960
   taaatgatat ttttagtttt atagcgttta ttataggtgt attttgatgt tattaaatgt 1020
   ttttatataa ttttttaaaa ataaaataga gtttggtttt ttttatagtt tagtttatta 1080
   tttgtgttat agtagtggta atgtaagatt tttatgagag taaaatgttt attaagtatt 1140
   tttaaatttt aaaaagatta attttaaaat gtttttagtt tgttatttgt gaggtatata 1200
   atgggtttat gaagtgaaag tgaaattatt atatattaat tatagtattt tatagaaata 1260
   tttagatgga ggaaggatgt aatggaagaa ttaagggttt tttttttttt ttttatagta 1320
   attagatttg aaaagtgaag taattttaaa gttattttag aagaaaaaaa tgtttaatta 1380
   aattttaatg tttaaaattg gatagtgtgt atggtaggtg atttgtatat atatgttatt 1440
   ttttattaaa attgtttttt atttttgtga tattaatata aattgtagtt atttggtttt 1500
   aaattatagt aagatatatt ttatttttta aaatgtaaat ggttatttaa aatagaaata 1560
   atttttttag acgtattgta tttttataaa tgtgattttg gaaatatatt gttcgtaaag 1620
   ggtaatttta agagaattgt tgagatttta attttatttt tatgtatatt ttggggaggt 1680
   ttttgaaaga gtaaaaaggt aaaagattta gttatttttt gagattattt atagtttttt 1740
   atttaaagtt gtagaagaaa aacgttaatg tattgttatt gttttttaat gaaattattt 1800
   tttttttaat taattaagta gtatttttta aatttattgt agaaatgtat attttatttt 1860
   tgttgtgagt cgtttttaat tttttttttt tttttttttc gtaataaaat ttggaaaaat 1920
   ttgttacgta gtattcgttt aacgtatcgt ttcgttgtat tttttttttt tttaattttg 1980
   tattatgata aaagtaaatt ttagtattat tttagggaaa tattgggaat gaatttggta 2040
   atgttttttt tttttttttt tttaggtaaa ggaaggggaa ttttgtattg tcgttaaaat 2100
   gagcgttttt ggtggaaagg aattttttat ttcgtatacg ttgtggttgt ttttgaaagt 2160
   gtaggtttcg taaaatgttc ggtcgattaa ggggtttttt tttttcggag tttttgtttt 2220
   tttttattta ttttagattt ttagtaagga agggtagagg ttggtttgtt tttttttttt 2280
   aagagggtat cgcgttgcgt agttaatagt ttgcgtcgtt tcggggaaat ttaaatgttt 2340
   cgcgatttta tcgagttaag gagtaaaagt tgtgattgtt attagttttt tgtcgggttt 2400
   taaattcggg gtttatcggt tgaaattttt agttgagtag tttttacgac gttgtttttg 2460
   acggttaaat tcggtgtatg taaggttggg attaattttt tttattcgat aaaggaattt 2520
   gatttttttt tgggagatcg tagttcgagt tcgattcgta gttttcgtcg cggggtttag 2580
   ggttttaggt aagcggttcg agatttagag gagttttttt ggattttttc gaggggtaaa 2640
   ttattcgagt ttgttatttt ttttggagtt aaaagtttat ttttggagtc gttatatttt 2700
   cgtgtttttt ttcgtatttc gggggtttcg ggggatgtac gtgggggtgg ttttaggagt 2760
   tttgggtggg atttcgggga aatgtattgg agattaggcg aaaaggtttt ttttgtttag 2820
   aatttgtttt tagagcgggt cgcgttcgaa ttcgtaggta aaggcgttgc ggtttttgcg 2880
   ggcgggcggg cgcgcgcgat gtattttttt tagggataga gttagaaagt ggacgtttgg 2940
   gcggggggag ggggggattt ataattggaa ttaggtttaa aaaaattatt ttaaaagaaa 3000
   aatgtagtaa gttttttttt cgagatttta ggtttattaa aataaaattg aattcgggtg 3060
   ttggtagtgt ttagggggag cggaggcgga gagggttgag gtcgcgggtc gttcgtttta 3120
   gtttttcgga agttcgggtt tttgaagtat agatagttaa gagcggttcg agttaagttt 3180
   taggaatagg ggtttcggga agagatttcg atcgggtcgt atagcgggga ggtttggttc 3240
   gagagttttt atttggtcgg ttgcgatttt atattggaat ttcggaaacg cgttttggtc 3300
   gttttagttt aagtgggttt tggggcggtt ttttgttttt tattttcgaa cgttagtttt 3360
   tttcgggcga ttcgaaggtt acgaggcggg tcgtatagtt tcgcgtcgtc gggggcgttt 3420
   cgggagtttc ggggattgcg cgcggcgttt agggagggcg gttgttgttt tttggcgttc 3480
   gttagagttc gcggcgcgtt tcgatagttt cgcgtagttc gggttcgttc gggaaaggtt 3540
   tttacgttag ttaatttcgg tttttttttg cgttcgggtt cgcggtaacg ttcggggata 3600
   gtttttgcgc ggtagcggtt atagtatttt agttttgggg gagggaggag cgggcgttag 3660
   gggtttcgtg aggtcggcga gggattttcg cgtcggggag tttaattgag gggtttcgcg 3720
   gggattataa agtagttcgc ggtggaacgg ggtcggattt cggggattgg gttgtagttt 3780
   cggggtggat gagaggtttc gggaggtgta gggttcggta gggagggcgt ggagggattt 3840
   atggtgacgg gagtagtttt cgttgggagg gagtttcgtc gggcggggtc ggagttggta 3900
   gcgtacgtcg gatcgggggc ggggtttttc gagtttcgcg aaaatgttcg tagtatcgcg 3960
   gggatcggcg tagggggaag cggtcgttcg tacgcgaagt cggggtgggt ttcgtatttg 4020
   gggtcgttcg agcgtacggg ggcggggaga ggagcggggg tcgttttcgg ggagtcggag 4080
   gggagacgcg gggttcgggt aggttgttcg gttgttgcgg gtgtcggcgt tataaggttc 4140
   gggagtaagt cgcgtggttg tttttaattt tttttttagt cgcgcgtttt tggggttttt 4200
   tttgattagg aaatttttcg ttggtcgttg acgtacgtag taagtgagac gcgcggaggg 4260
   atcgagtcgc ggttcgggcg tagcggtaaa aaatatttat tatagaaatc gatttaaggg 4320
   aagacgttga gagtaaacgg tacgcgcgga cgtcgttcgg ttttatgtaa ggaattagcg 4380
   tcgtttcgtc gtagttgtta gtcggacggt gtaggagcgc ggagtggttt tttcgggttt 4440
   cgcggttaaa tcgtattttc gttttggaag cggaggtttg gttttgggcg gtcgcgtagg 4500
   atttggggtc gttcgcgggt tgtagttcgg ttcgggagtt tcgtggcggt tattgcgttt 4560
   gtttgtagcg gttcgcgggg tcgggttttt tcgggtgcgg cgcgtcggtc gggttttttt 4620
   cgggtcggcg gggtttgttt ttttttttta ggttttcgga tcgtggttat tcgcggagtc 4680
   gtataagttt gcgtgtggtt gcggaaaggg gttcgtacgc gggcggggtt tcgggttagt 4740
   atgtaggttt ggatgcgggg cgcgggggtg gttttcgatt tttcgttcgg gttgcgtata 4800
   gtagcgcgcg gcgcggcggc gggggcgcgc gggttaattt taggagtcgg cggcggcgag 4860
   tcgcgttttg gttgatatcg gaaaggtttg tgattggtcg tcggtcgttt ttatgtaaac 4920
   gagttgaggg aatggaattt tttcggtcgg gtttatagta aaagtacgtt tatttttagg 4980
   tatttttatt tatagagtta gcgggcgcgg gcgggacggg cgtttcgcgg tcggatttag 5040
   ttagggtatt acgttgttcg gttttgcgtc gttaggtatt ttttttcggg gtttttaggg 5100
   acgttagaag gaagttaatt tttgttgttt ttttttggtt tgcgttggat tttttttttt 5160
   ttgttgtttt tttttgtttt tttttttttt tttttttgaa ttaattggtt ttttggttgg 5220
   atgtttttaa tttttttttt ggttgcgaat ttttttttaa ttgttttttt tttataatag 5280
   ggggagaaag tgttttgtgg ttcgaggcga gtcgtgaagt tgcgtgtgcg tggtagtgtg 5340
   cgtggtagga tgtgcgtgcg tgtgtaattc gagtcgttcg atttgtttcg atttgcgtcg 5400
   cggagttttt ttttaaggtt cgttttattt gttgcggtta cgcggcgttc gtgggtgttc 5460
   gtgtttcgga gtagttaatc ggcgggtgtt gggcgacggt ggaggagtat cgtttcgttg 5520
   ttgttcgagt tagggttgag ttatttagtt gatgtagata gtggttgttt ttcgaagagt 5580
   gcgtgtttgt atgtgtgtga ttttgcggtt gtttaatttt taataaatta gaggattagt 5640
   tataaattta attaatattt ttaaattcga gtttatagat gtgggagagt tgtagaattt 5700
   tgagtgttat cgattgggtt tttttatgat tgttgtttta agattagttg aagatttttg 5760
   aaacgttgaa ttttttgtat tgagcgtttt gatagaattt attgagagaa tagagaatat 5820
   gataagtatt tttagtttag tattgtttta attattgaag ttgattttta aggttattta 5880
   aaaaaatttg tagcgtatat taatggattt ttgttgtgtt taaatttttt atagattgta 5940
   ttgtaaatat tttatgaagt agagtatatg tatatattta tatatacgtg tatatatatt 6000
   agtagtatta tttttggaag ttttagtttt tgtttttcgg gattgaagtt agttttgtat 6060
   gataaaagtg gttttgttac gggagataat tgtgttttgt tgggatttta gataaaattt 6120
   atttgtaaaa aattgatagg tattaattat tggaattttt aaataatgtg tttgttgatc 6180
   gttttatttt tcgtataaat attttaggaa gtgtatgaga attttgtttt taggaatttt 6240
   tttaatagtt aaagatagaa tttaattttt gtaagtaaga ttcgtggaag atagttttta 6300
   ttttttaatg tattaagtaa tcggttgtta ggagtttatt ttgggttaga ggtcgattcg 6360
   tagaattaga acgttttttt tttttggatt gttagtaatt tagttttttt ttttttttaa 6420
   ttattttcgt ttttttttat ttttcgtagt aataaaggtt tttgaacgtg tatgttggtt 6480
   tttcgggagt tgtttgttga agattcgcgt ttttgtcgtc gtttggtagg agttgtttgt 6540
   agggttttaa tttaatcggt ttgttgtgat gcgtattttc gtagatgtta gtacgagtcg 6600
   tcgttttacg tcgtttttta tcgcgttgag tttaggtaag atgagcgagg cgttgtcgtt 6660
   gggcgtttcg gacgtcggcg ttgttttggt cggtaagttg aggagcggcg atcgtagtat 6720
   ggtggaggtg ttggtcgatt attcgggcga gttggtgcgt atcgatagtt ttaatttttt 6780
   ttgtttcgtg ttgtttacgt attggcgttg taataagatt ttgtttatcg tttttaaggt 6840
   attggttcgg agagggtggt gggaggatag gcggaggtcg gcgaggtcgg gggagattcg 6900
   cgagggtagc ggttaggcgg gcggggtcgg attttggaaa ggggttttcg tagtttcggg 6960
   tcggtttttt tagtttggga tgtgggggat gttgtgttgt cgtggcgatt ttcgggaggt 7020
   attcgtttta gttttttggg gcggtttttt aggagggttg aaaatattag ggtgttttta 7080
   aagatattta ttgggagtcg gtcgggattt ttttggtagg ggttttagtt ttgatataaa 7140
   gtaggaaaag ttataattta gttcggtaga gatagttcgt ttgttaattt attgttgttt 7200
   ttatcgttgg attaatattt aaattatttt gtttttttta gatgaaagga ttaggaaata 7260
   aaatagtaag atttttttgt ttttaagtta gtgaaaagaa attaattagg gaaattgtta 7320
   gggatttttt tttttttttt ttaatcgttt tataggaatt agtatgttta aagtttatag 7380
   cgggcgtagt taagtttgta aaagtttttt gaagagtttt ttgagttttt tagtttgttt 7440
   ttgttagaag tcgtaagtat cgaattttgt tttttttttc ggaatcgttt tgaatgttat 7500
   tattagttta tttagattaa gttaggggta gcgaaaggtg acgaggttgt tttcggagag 7560
   ttaggggttt tagcggttcg tttttttgtt agaggagggg gcggcgcgta gtcggagatt 7620
   ttagcgcggc ggttttaaag attttttttt attttttatt tttgattatt tttaaagttt 7680
   tgagttgttt ttaaaaattt tgatttttaa taggggtatg aaaataattt taggcgtttt 7740
   taaataatta ttttattata ttagtagtaa gtagtaggaa tttaattttt gtagtggaga 7800
   ttttgttttg tttttggtgg gtttgaaaag atgcgattaa gtaaaattag tgcgggttat 7860
   tttttgttcg tttaaagggc gaattaaggt aggtggtttt aagtgtaaag ataagttatt 7920
   aaaaaaaaag tttaaataat tgtttttttt aaagggagat taatttaata taagtttttt 7980
   atagttttaa gttttagttg ggttttgatt ttattataac gacgaatatt tattttttaa 8040
   aaaagtattg ttgcggttat ttttaagttt tatatagttt ttatatttag attgtgattt 8100
   ttaaaataaa acgaaatttg tagttggttg tgtttataga ttggtttttt tttttattta 8160
   tttttttaaa agtagttttt tttgtttgtt ttgtttttta gtgggggtgg aaatgaggag 8220
   aaggaacgaa ggaacgtggt ggtttgtttg attttttttt tttttttttc gtttatttag 8280
   atttgggttt gggagattat tttattttta aattatttta attttttgtt taaatggatt 8340
   tagcgatgat tttttagggt attcgtattg agaggggatt taatagggtt gggttatggg 8400
   gttattatta gataaagagt agaggtaaga cgggttgata attattaaag gttattaaaa 8460
   tttggttgtt ttaatgtatt agggttttaa gagatttgtg gttttttttt gggggggaaa 8520
   ttagggtttt ttttgaattt ttttttttgt tagggaattt atatttaaat atattaaaag 8580
   attaaatgat taataaataa tttttaggaa ttggagtaat ttagagtttt aagttattat 8640
   atgaaagtat ttattttttt ttaaaaaatg aaaaattata tatattgttt ttatgagttg 8700
   ttagaatttt ttttagtaga aattataata attatagagt ttttttttta aaatgatttt 8760
   tggtaattta tttaaaaata attaatttgt tttattgagt gataatttat aggttattaa 8820
   aaatttaaat atagagtttt tgaaaaaagt ttaagtagat tttttgagaa tatttagggg 8880
   aaaggaaagg atagtagaga aattagatta tttgattatt gtttgttatt attttaattt 8940
   ttataaaaag aggttttttt ttggaggaga tttgtttatt gtataattag aagagtagat 9000
   aaaatgttta gtgagatagt aaacgtgaag ggataaaatg ttagggataa aagtaagtaa 9060
   aatattaaaa tttgtttata tttcgggttt atttagtgtg tgtttttggt tttattttat 9120
   ttgtttattt aattatttta taagtagttt taatagaaat tattatttta aaatttggat 9180
   tttaaataag tcgtaggtaa tttttttagg aagttaatta gtaaataatt attatatttt 9240
   ttatggtaaa gagaagtaaa tgtgaaataa tgataaggtt tgatgatatt gatgaaaaga 9300
   ttattgaagg tttttttagg aattttttga agttttgtag gtaaattttt tacgttaatt 9360
   ttttgaatta aagagagatt taaaattatg aatttttttt aaaatagatt ataataattt 9420
   tatttgttta ttttgagttt taatttgttt tttttttatt tttttttttt tatggaatat 9480
   ttttattttt tatagtatga gagatatatg ttttttatta ttaagaaaat tttaagtttg 9540
   ttgtgtgttt tgttgtattt atagaattag tttaaaaagt tgttttgatg atttaggatg 9600
   tatttggtaa tattatggtg ttatagtttg tttaggtttt ggtatattag agtttaaaga 9660
   cgtaaacgtg tttttagtag ggaagttcgg aggttttgga ttttgatggt aatatttttt 9720
   ttataaaaag gaaataagta atatttttgt cggtaagatt atagttttta gttatgtttt 9780
   aaatattatt ttagttgaat tttgtgtttt attttatagt tttttttgta atattggaag 9840
   gttaacggat ttgaagtaaa tgaattgtaa ttgtaatttt aatttaatta gaattggttt 9900
   aaaatgttag tttggatcgt gaataggtta gttaagaata gtattgggag gtttatgtaa 9960
   tatagaagag agttgatatt tggtttaatt ttttgatatt ttaaaaatag agaagtaaaa 10020
   taaaatatga gatatttttg gttagtaagt ttttttttgt ttgtttggtt tgtgttaaaa 10080
   tgtaaatgtt tgtgtttttt tatttattgt ttatagtttt ttattttagt tgtttttttt 10140
   ttttgtttga agttagatat ttttatattt tttttatatt gtttattagt gataaaattt 10200
   gtattaaatt agtacgtatg ttagttttga attaaaaaga aattttttaa atttggttta 10260
   agtatgtttg ttaataattg gacgatatat agtattaatg aattgttttg gatttattag 10320
   ttttttttta tttattcgtt tttattaaaa aaaaattttt agagattaat ataaggttaa 10380
   gtgaatgata tgtaggtgtt taaatatggt tgattttgtt gtatttaaaa aaaaagagga 10440
   gtgggagtta aatttttaat tttttttttt ttaagtttta atattaatag ttttttttta 10500
   tttagttttt atgataattt tattttatgt ttgtttcgat ttggcgaagg ttttttgaat 10560
   ttgttaaatt attaatgagg atagtattgg aaagatgaga gaggtatagg tttggagtat 10620
   tgtgagggga atagtagtag gaggtagata atttagaata tatttttgta gttataaaag 10680
   aagatttatt tatttttatt tttttataga tttagattta ataatgagtt atttagtttg 10740
   aaatttacgt agtggaggta gtttttgtat atttgttgaa tgttgagtta taatagagaa 10800
   gtttgggtta ttatagggag aggaggatga ggaagtaaaa atatgtgttt aaagagggta 10860
   ttttaatatt gtgagttaat tttataacgg gaagtgaaaa ttatgaaaaa aaggggaaaa 10920
   tttgttgtga gagattaaaa agagtaaaat aaagtaagtt tatttttcgg gaatagaaaa 10980
   aaaaaaaaaa tagtagtaat aattaaataa ttgattaggg agatttattt tcgtgatatt 11040
   tttttaaaat aaaaagggaa ttgtcgtatt attgttttta attaaatggt aataaatata 11100
   tttataatgg gaattaaata gttaattaga gggtgtttgg ggaataaata gtagagtttt 11160
   aaaatttttt atatggaaaa tatgtttaat aattggaatg aaaggattaa aaagagagaa 11220
   aaggtttaga ttttgtttgg tagtgtaagt tggataaaat agaggaaaat gtttttttgt 11280
   ttttatgaga ttagattatt ttggtgtagt tttggggttg gaagtatgtt tattttttat 11340
   agtgagttag ttcgtgtttt gtgttataag gattgaagta gttttaatga ataaaaataa 11400
   tagatagtta tttggaggtt atagggaaag ggtgatggtt aagtagatta tgaaaatggg 11460
   aattttgaga aatatgattt cgatattata taagtgagtt tatggtagtt ttttttatat 11520
   atagaaaaag aggtttgaaa atatatatta taaaaaatta tgtagtagtt attaaggtaa 11580
   aaattgggaa gtaggaggta gaggaaggag tgataatatt agattaagga atgttttatt 11640
   gttattaatt taaaaatagt aattaaaaat gatgatgtaa ttggaatgag taatataaat 11700
   gttattattg attttatcgt aaagaatgtt agaaatgatt agaataaata tagtttgggg 11760
   tatttcgatt tgaagttgta tgattgtttt gaaaaataga aataaaggaa atatagttaa 11820
   tagtaaatgt ttagattaag aaaatagttt ttagaaggtt taaatgatag ttaagaaatt 11880
   gatgttattg tataaagtat ttttaggtta tgttttttag agtattttat tttggaattt 11940
   atttttttaa ttatttgagt ataaaaatat ttatttatta gtttttttta atagttttga 12000
   taagatttta tatttattta gttatattaa ataaattttt ggttaagatt agttgggtta 12060
   gtagaaaatt ttaggtattt gtgaggatat gagtttatat acgttgagat ttatagatat 12120
   aaaaatgtaa tttaatttta tttttgaatt gaggggagtg tatagaagtg aatgtttcgt 12180
   ttttttgagg tttgttgatt ttgtaattag taaacgaagg gtgtattttt gatttttttt 12240
   ttttgtgtgt tagaatttat tgttagtaaa atttaagata atagcgatga gtaggaggta 12300
   ttaaagatga attgtagagg gatagtttaa gttatttaag aatcgttagt aagatgaaat 12360
   ttatttttag tagaaattgg gtttttttgt gtttttttgt tttgttttat tttttaaaag 12420
   taaagtttgt attttgttta gtttgttagt ggaggtttga gtaagtaaaa gatgggttgg 12480
   attataaatt tataaatata ggatgttttg ttttttaaac gggagaaatt aagaagagat 12540
   gtttgtattt aggagacggt atagttattt aaaatttttg atattttgtt atggttagtt 12600
   ttgttttaat tgtgttatgt gatttattat ggttttatga ggtaaattta gtatatgtgt 12660
   tattatttga aaatttatat atagttatat ataatgtatt aggttggtgt aaatgtaatt 12720
   gtagtttttg gagttaaaaa ttgtaaatat tgtaattatt tttgtattaa tttaaaatat 12780
   aataaaaaag tgtttttttt ttataatttt tttgttgttt tgtttaagtt attattgtta 12840
   tttttttgta atttaaaaag aaattattga atatatattt aattttagaa taattattat 12900
   ataaatgttt taaaagtgta tgtataatat ttttgatgtt tgtatttgtt ttttgattgg 12960
   tgtttagg 12968

<210> 302
<211> 12968
<212> DNA
<213> Artificial Sequence

<220>
<223> chemically treated genomic DNA (Homo sapiens)

<400> 302

tttaaatatt agttaaagga taaatgtaga tattagggat gttatatata tatttttagg 60
gtatttgtgt agtgattatt tttaaattaa atgtatattt aatgattttt ttttaggttg 120
tagaggaata gtaatgataa tttaagtaaa ataatagaaa agttataaag agaagatatt 180
tttttattat attttaggtt ggtgtaaaag taattgtagt gtttgtaatt tttaatttta 240
aaaattgtag ttatatttgt attaatttaa tatattatgt ataattatat gtaaattttt 300
aaatagtgat atatatatta aatttatttt ataaagttat agtaggttat atagtatagt 360
tggaataaga ttaattatag taagatatta aggattttga gtagttatgt cgttttttga 420
atataagtat ttttttttaa tttttttcgt ttgagaaata gaatattttg tgtttgtaag 480
tttataattt aatttatttt ttatttgttt agatttttat taataggttg aataaggtgt 540
agattttatt tttagaaaat aaaataaaat aaaaaaatat aaaaaaattt aatttttgtt 600
aaaagtagat tttattttgt tgacgatttt taagtaattt aaattgtttt tttatagttt 660
atttttgata ttttttattt atcgttatta ttttgagttt tattagtaat gaattttagt 720
atataagaaa aaaaaattag aaatgtattt ttcgtttatt aattataaaa ttaatagatt 780
ttagaaagac gggatattta tttttatgta ttttttttaa tttaggggtg gaattgggtt 840
gtatttttgt atttgtgagt tttagcgtgt gtaaatttat gtttttatag atgtttgagg 900
ttttttgtta gtttagttga ttttgattag gaatttattt aatgtaattg agtaagtatg 960
aagttttatt agggttattg gagaaaattg gtagataaat gtttttatat ttaggtaatt 1020
aagaaaatga attttaaagt aaaatatttt gaaaagtatg atttaaagat attttgtata 1080
atagtattaa ttttttggtt attatttagg ttttttagaa attatttttt tggtttagat 1140
atttattatt ggttgtattt tttttgtttt tattttttaa agtagttata taattttaaa 1200
tcgaaatgtt ttaagttgta tttattttga ttatttttga tattttttac ggtaaagtta 1260
gtagtgatat ttatattatt tattttaatt atattattat ttttggttgt tgtttttgaa 1320
ttggtaatag taaagtattt tttaatttag tgttattatt tttttttttg ttttttattt 1380
tttaattttt attttaataa ttgttatata attttttgta gtatatattt ttaggttttt 1440
ttttttatat gtaaaaaagg ttgttataga tttatttgta tgatatcgaa gttatgtttt 1500
ttaagatttt tatttttatg atttgtttag ttattatttt ttttttgtgg tttttagatg 1560
attatttatt gtttttattt attagaattg ttttagtttt tgtggtatag aatacgaatt 1620
aatttattat gagagatgaa tatattttta attttaagat tatattagga taatttagtt 1680
ttatagaagt agggaggtat ttttttttat tttatttaat ttgtattgtt aaataaggtt 1740
tgggtttttt tttttttttt aattttttta ttttaattgt tggatatgtt ttttatatag 1800
gagattttaa aattttgtta tttatttttt aagtattttt taattagtta tttagttttt 1860
attataagtg tgtttgttgt tatttaatta agagtagtga tacgataatt ttttttttgt 1920
tttaaaaaga tattacggaa ataaattttt ttgattagtt atttggttat tgttattgtt 1980
tttttttttt ttttattttc gaaaaatggg tttgttttat tttgtttttt ttagtttttt 2040
ataataaatt tttttttttt ttttatgatt tttatttttc gttgtaaagt tagtttatag 2100
tgttgaaatg ttttttttga atatatattt ttattttttt attttttttt ttttgtggta 2160
atttaggttt ttttattgta atttaatatt tagtaggtat atagagattg tttttattac 2220
gtgggtttta aattgaataa tttattgttg ggtttggatt tgtaaaaagg tgaaaataaa 2280
taaatttttt tttataatta taggaatgta ttttgaatta tttgtttttt attgttgttt 2340

tttttatagt gttttaagtt tgtgtttttt ttattttttt aatattattt ttattggtaa 2400
tttggtaaat ttaggagatt ttcgttaagt cgaaataaat atagaataaa attattatgg 2460
aggttaaata agaaaaggtt gttgatgttg gaatttaaga agaaaaaaat tagaagttta 2520
atttttattt tttttttttt ttaaatgtag tagaattaat tatatttggg tatttatata 2580
ttatttattt ggttttgtat tgatttttga gggttttttt ttggtggagg cgggtgggta 2640
aaggggaatt agtaaattta gggtaattta ttaatattgt atatcgttta attgttaata 2700
agtatattta agttaggttt aaaaagtttt tttttaattt aaaattaata tgcgtgttaa 2760
tttgatatag gttttgttat tgatgggtaa tatggagaga atgtaaaaat atttaatttt 2820
aaatagaaaa aagaaataat tggaatgaaa agttataaat aataggtaag gaaatataaa 2880
tatttatatt ttaatataga ttaaataggt aagaagggat ttgttggtta aagatatttt 2940
atattttgtt ttattttttt gtttttaaag tattaggaag ttgggttaaa tattaatttt 3000
tttttatatt atatggattt tttaatattg tttttggtta gtttgtttac ggtttaaatt 3060
ggtattttaa attaatttta attaggttga aattatagtt ataatttatt tattttaaat 3120
tcgttaattt tttagtgttg taaagaaggt tgtggagtgg aatatagaat ttagttgaga 3180
tgatatttaa ggtatagtta gggattgtga ttttgtcgat aaaaatatta tttatttttt 3240
ttttatgaaa agaatattat tattagaatt taagattttc gaattttttt gttgaaggta 3300
cgtttacgtt tttggatttt ggtgtattag ggtttgaata agttataata ttatggtatt 3360
attaaatata ttttaagtta ttaaaatagt tttttaggtt aattttgtaa gtatagtaaa 3420
atatatagta aatttaaggt ttttttagta gtaaaaaata tgtatttttt atattgtaaa 3480
aaataaaggt gttttatggg agggaaggag tagggggaga ataggttggg gtttaaaata 3540
agtaagtagg gttattgtaa tttattttaa agaaaattta taattttaaa ttttttttta 3600
gtttagaaga ttgacgtaga ggatttattt atagaatttt aggagatttt taagaaggtt 3660
tttagtgatt tttttattaa tattattagg ttttattatt gttttatatt tgtttttttt 3720
tattataggg aatataataa ttatttattg gttaattttt tagggagatt gtttgcggtt 3780
tatttaagat ttaaatttta aagtaataat ttttgttgaa gttgtttgtg aggtggttgg 3840
gtgggtagat agagtgaagt tagggatata tattaaatga gttcggatgg taggtaggtt 3900
ttgatgtttt gtttgttttt atttttaata ttttattttt ttacgtttat tgttttattg 3960
aatattttgt ttattttttt agttatgtag tgagtaggtt ttttttagga agaagttttt 4020
ttttatgagg attggggtgg tgatagataa taattaaatg gtttgatttt tttgttattt 4080
tttttttttt tttaaatgtt tttaggggat ttgtttgaat tttttttaag agttttgtat 4140
ttggattttt agtagtttat agattattat ttaataaaat aaattaattg tttttaagtg 4200
aattgttaaa agttatttta aaaaggaaat tttatggtta ttatggtttt tgttagaaag 4260
aattttgata gtttataaaa gtaatatgta taatttttta ttttttagaa gaaaataaat 4320
atttttatgt aatggtttga gattttgaat tgttttaatt tttgaagatt atttattaat 4380
tatttagttt tttaatatat ttgagtgtgg gttttttggt agaggaggga gtttagaaga 4440
gattttgatt ttttttttaa aaggaagtta taaatttttt agagttttag tgtattgggg 4500
tagttaggtt ttaatggttt ttggtggtta ttagttcgtt ttgtttttgt tttttatttg 4560
atagtgattt tatggtttaa ttttgttggg ttttttttta atgcgggtgt tttaaagagt 4620
tatcgttgag tttatttagg taaaggttta gaatgattta aaagtaaaat ggttttttaa 4680
atttaaattt aagtaaacgg agggagggaa aaaagagtta agtaagttat tacgtttttt 4740
cgtttttttt ttttattttt atttttatta aaaaataaaa taaataaaaa aaattgtttt 4800
tagaaaagtg agtggaaaaa gaggttaatt tatagatata gttaattgta aatttcgttt 4860
tgttttagaa attataattt aagtgtggaa attgtataaa gtttaaaaat aatcgtaata 4920
atattttttt aaaaagtgaa tattcgtcgt tgtaataaga ttaagattta gttgaggttt 4980
ggaattataa aaagtttata ttaaattaat ttttttttaa aggggataat tatttaaatt 5040
ttttttttag tagtttattt ttatatttgg aattatttgt tttgattcgt tttttaaacg 5100
aataggaaat gattcgtatt gattttattt agtcgtattt ttttaaattt attaaagata 5160
aaatagaatt tttattgtag aaattaaatt tttattgttt gttgttagtg tagtagggtg 5220
attgtttaga aacgtttggg gttgttttta tatttttgtt gaaggttaag gtttttaaag 5280
gtagtttaaa gttttgggga tgattagggg tgaaaagtga agggagattt ttggagtcgt 5340
cgcgttgaag ttttcggtta cgcgtcgttt ttttttttag taggaaagcg ggtcgttggg 5400
atttttggtt tttcgaaaat agtttcgtta tttttcgtta tttttggttt aatttaggtg 5460
agttgatgat agtatttaga acggtttcgg gaaaagaaat aggattcgat atttgcggtt 5520
tttggtagga gtaggttaaa aagtttagag aattttttag aaaatttttg taaatttaat 5580
tacgttcgtt gtggattttg gatatgttaa tttttatagg gcggttagag aaggggaaaa 5640
aaaatttttg atagtttttt tagttagttt ttttttattg atttgaaggt agaaaagttt 5700
tattatttta ttttttgatt tttttatttg aggaagataa aatggtttgg gtgttaattt 5760
agcggtaggg gtagtagtaa gttagtagac gggttgtttt tgtcgggttg ggttataatt 5820
ttttttattt tgtattaaag ttaaggtttt tgttagggag gtttcgatcg gtttttagtg 5880
gatatttttg gggatatttt gatgttttta gtttttttgg gaggtcgttt tagaaagttg 5940
agacgagtgt ttttcgaggg tcgttacggt aatatagtat tttttatatt ttaagttagg 6000
aagatcgatt cggggttgcg gggtttttttt tttagaattc ggtttcgttc gtttggtcgt 6060
tgttttcgcg gatttttttc ggtttcgtcg gttttcgttt gtttttttat tatttttttc 6120
gggttagtat tttgaaagcg atgggtaggg ttttgttgta gcgttagtgc gtaggtagta 6180
cggagtagag gaagttgggg ttgtcggtgc gtattagttc gttcgggtgg tcggttagta 6240
tttttattat gttgcggtcg tcgtttttta gtttgtcggt tagggtagcg tcggcgttcg 6300

gggcgtttag cggtaacgtt tcgtttattt tgtttgggtt tagcgcggtg gaaggcggcg 6360
tgaagcggcg gttcgtgttg gtatttacgg ggatacgtat tataataagt cgattgagtt 6420
aggattttgt aaatagtttt tattagacgg cgataggggc gcggattttt agtaagtagt 6480
tttcgggaga ttaatatata cgtttagggg tttttattat tgcggggggt gggggggggc 6540
gggggtggtt aggggaggag ggagattaag ttattaatag tttaggaggg gaaaacgttt 6600
tggttttgcg gatcggtttt tgatttagga tgggttttta gtaatcgatt gtttagtgta 6660
ttaaaaagtg gagattattt tttacgaatt ttgtttgtag aggttaagtt ttgtttttgg 6720
ttgttagaaa agtttttgaa ggtaaaattt ttatatattt tttaaaatat ttatgcgaag 6780
agtaaaacga ttagtaaata tattatttgg aagttttagt agttaatgtt tgttagtttt 6840
ttgtaggtga gttttgttta aagttttaat agaatataat tatttttcgt aataaggtta 6900
tttttattat gtaaaattgg ttttagtttc gaaaagtaag agttgagatt tttaaaggta 6960
gtgttattaa tgtatgtgta cgtatatata aatatatata tatgttttat tttataaaat 7020
atttataata taatttgtgg agaatttaaa tataatagaa atttattaat gtacgttgta 7080
gattttttta agtagttttg aaaattagtt ttagtagttg gagtagtgtt gagttagaag 7140
tatttgttat gtttttgtt tttttaatga atttgttaa aacgtttagt gtagaaaatt 7200
tagcgtttta gagattttta gttaatttta aaataataat tataagaagg tttagtcgat 7260
gatatttagg gttttatagt ttttttatat ttgtgaattc gggtttgggg atgttggtta 7320
agtttgtggt tggttttttg gtttgttggg agttgagtag tcgtagagtt atatatatgt 7380
aaatacgtat ttttcggaag gtagttattg tttatattag ttgggtgatt tagttttgat 7440
tcgggtagta gcgagacgat atttttttat cgtcgtttag tattcgtcgg ttagttgttt 7500
cgaggtacga atatttacga gcgtcgcgta atcgtagtag gtggagcggg ttttgaggga 7560
gggtttcgcg gcgtagatcg aaatagatcg ggcggttcgg gttatatacg tacgtatatt 7620
ttgttacgta tattgttacg tatacgtaat tttacggttc gtttcggatt atagagtatt 7680
tttttttttt gttgtaaaag gaaaataatt ggggaaaagt tcgtagttag gaaagaagtt 7740
gaaaatattt agttaagaag ttagttaatt taaaaggaag aaaggggaaa aataaaaaaa 7800
aataataaaa aaaggaaggt ttaacgtagg ttaaggagaa gtagtagagg ttgatttttt 7860
tttggcgttt ttaggagttt cggaaagaag tgtttggcgg cgtagggtcg ggtagcgtgg 7920
tgttttggtt gggttcggtc gcggggcgtt cgtttcgttc gcgttcgttg gttttatgaa 7980
tgagagtgtt tggaaatgaa cgtgttttta ttgtaagttc ggtcggagga attttatttt 8040
tttagttcgt ttgtataggg gcggtcggcg gttaattata ggttttttcg gtattagtta 8100
gggcgcggtt cgtcgtcgtc ggtttttgga attggttcgc gcgttttcgt cgtcgcgtcg 8160
cgcgttattg tacgtagttc gggcggggag tcggaggtta ttttcgcgtt tcgtatttaa 8220
gtttgtatgt tggttcgggg tttcgttcgc gtgcggattt tttttcgtag ttatacgtag 8280
gtttgtgcgg tttcgcgagt ggttacggtt cggagatttg gaaaaagaaa gtaggtttcg 8340
tcggttcgag gaggattcgg tcggcgcgtc gtattcggag aggttcggtt tcgcgagtcg 8400
ttgtaggtag gcgtagtggt cgttacgagg ttttcgaatc gggttgtagt tcgcggacgg 8460
ttttagattt tgcgcggtcg tttagggtta ggttttcgtt tttagggcgg gggtgcgatt 8520
tggtcgcggg gttcggggga gttatttcgc gtttttgtat cgttcggttg gtagttgcgg 8580
cgaagcggcg ttgatttttt gtatgaggtc ggacggcgtt cgcgcgtgtc gtttgttttt 8640
agcgtttttt tttgggtcgg tttttgtaat gggtgttttt tatcgttgcg ttcgggtcgc 8700
ggttcgattt tttcgcgcgt tttatttgtt gcgtgcgtta gcggttagcg aagagttttt 8760
tagttaggaa agattttaag aacgcgcggt tggaaggaaa gttgaaagta gttacgcggt 8820
ttgttttcgg gttttgtagc gtcggtattc gtagtagtcg gatagtttgt tcgggtttcg 8880
cgtttttttt tcggtttttc ggaagcggtt ttcgtttttt ttttcgtttt cgtgcgttcg 8940
agcggtttta ggtgcggaat ttatttcggt ttcgcgtgcg ggcggtcgtt tttttttgcg 9000
tcggttttcg cggtgttgcg ggtattttcg cggagttcgg agggtttcgt tttcggttcg 9060
gcgtgcgttg ttaatttcga tttcgttcgg cggggttttt ttttagcgga ggttgttttc 9120
gttattatga gtttttttac gttttttttg tcgggttttg tatttttcgg ggttttttat 9180
ttatttcggg gttgtaattt agttttcgga tttcggtttc gttttatcgc gggttgtttt 9240
gtggttttcg cggagttttt taattaagtt tttcggcgcg ggggtttttc gtcgatttta 9300
cggggttttt gacgttcgtt tttttttttt ttagggttag ggtgttgtgg tcgttgtcgc 9360
gtagggattg ttttcgggcg ttgtcgcggg ttcggacgta ggagggggtc ggggttgatt 9420
ggcgtggagg tttttttcgg gcgggttcgg attgcgcgga gttgtcggga cgcgtcgcgg 9480
gttttggcgg acgttagggg gtagtagtcg ttttttttgg acgtcgcgcg tagttttcgg 9540
agttttcgga acgttttcga cggcgcgggg ttgtgcggtt cgtttcgtgg ttttcgggtc 9600
gttcgggaag aattagcgtt cgaggataaa agataggaag tcgttttaga gtttatttga 9660
gttggaacgg ttaaggcgcg ttttcgaggt tttaatatag agtcgtagtc ggttaggtgg 9720
ggattttcgg attaggtttt ttcgttgtgc ggttcggtcg gggttttttt tcgaagtttt 9780
tgtttttggg gtttgattcg ggtcgttttt ggttatttgt gttttaggag ttcgggtttt 9840
cggggggtta aggcgggcgg ttcgcggttt taattttttt cgttttcgtt ttttttgggt 9900
attgttagta ttcgagttta gttttgtttt aatggatttg gggtttcgga aagaaaattt 9960
attatatttt ttttttaaaa tgattttttt aagtttaatt ttagttgtaa attttttttt 10020
tttttcgttt aaacgtttat tttttaattt tgtttttgag aagagtgtat cgcgcgcgtt 10080
cgttcgttcg taggggtcgt agcgtttttg tttgcgggtt cggacgcggt tcgttttaga 10140
ggtaagtttt gggtaaggga aattttttcg tttggttttt aatgtatttt ttcgagattt 10200
tatttagggt ttttggggtt atttttacgt gtatttttcg gaattttcga gatgcgggag 10260

ggagtacgag ggtgtggcgg ttttaaaagt aggtttttga ttttagggga aatagtagat 10320
tcgggtgatt tgtttttcgg aaaggtttag ggaggttttt ttgggtttcg ggtcgtttgt 10380
ttaaaatttt aaatttcgcg acgggggttg cgagtcggat tcgggttgcg gttttttagg 10440
agggagttaa gtttttttat cgagtaagga aagttggttt tagttttgta tgtatcgagt 10500
ttagtcgtta gaggtagcgt cgtgggagtt gtttagttag gagttttaat cgataaattt 10560
cgagtttgaa gttcgataaa aagttgatag taattatagt ttttgttttt tgattcgatg 10620
ggatcgcggg atatttgggt tttttcggag cggcgtaggt tgttaattgc gtagcgcggt 10680
gtttttttga aaagaagaaa tagattaatt tttgtttttt tttattgagg atttaaaatg 10740
aatggaaaga ggtaggggtt tcggggaaag ggaatttttt agtcggtcgg gtattttacg 10800
gagtttgtat ttttaaggat agttatagcg tgtacgaagt gaggaatttt tttttattaa 10860
gagcgtttat tttagcgata atatagaatt tttttttttt tgtttaaggg agaaaggaaa 10920
ggaaatatta ttaggtttat ttttagtgtt tttttggagt aatgttagaa tttatttttg 10980
ttataatgta aaattaaaaa aaaaaaaaat ataacgaagc gatacgttgg gcggatgtta 11040
cgtgatagat ttttttaaat tttgttgcgg ggagagggag ggaggagaat tgaaaacggt 11100
ttataatagg aatgaaatgt atatttttgt agtggattta aaaagtattg tttggttagt 11160
tggaagaaaa ataattttat tggaaaatag taatagtgta ttgacgtttt ttttttataa 11220
ttttaggtaa agaattatag gtggttttag aagataatta aattttttat ttttttgttt 11280
ttttaaaagt ttttttaaaa tatgtataaa gatgggatta gaattttaat aattttttta 11340
aaattatttt ttgcgagtaa tatattttta aaattatatt tgtaaaaatg tagtacgttt 11400
aaaagaatta tttttatttt aaatgattat ttatatttta aaaaatagaa tgtattttgt 11460
tatggtttgg aattaaatag ttgtagtttg tattgatgtt atagggatgg aaaataattt 11520
tgatgaagag taatatgtat gtataaatta tttgttatat atattattta gttttgaata 11580
ttggaattta attaaatatt tttttttttt gaagtggttt tgagattatt ttatttttta 11640
aatttaatta ttatgagagg ggggaggaga gtttttagtt tttttattat attttttttt 11700
tatttgaatg tttttgtggg atgttatggt taatgtgtga tgattttatt tttattttat 11760
aaatttattg tgtattttat aagtagtaag ttgaggatat tttaaaatta atttttttaa 11820
aatttaggaa tgtttagtaa atattttatt tttataaaaa ttttgtatta ttattgttat 11880
aatataggta ataagttggg ttgtgaggga agttaagttt tgttttgttt ttagagagtt 11940
gtatagaggt atttaatagt attagaatgt atttatggta aacgttgtag aattgagggt 12000
attatttaag tttttattga aatattttat atggtataat atttataata tggagaattg 12060
tttttaagta tattggaatt agtagaaata gttttaatta tttggttaaa ttatattata 12120
agttatatat agagatatat agatataaaa ttatatatat atttatgaag gtgtgtattt 12180
tatttaaaaa tataatttgg aatttaatat atcgtggacg tttttagaag gatttatttt 12240
aagtatgtat tttgaaataa tagaaagtag gaaaataaaa gtaatgtaag tttaaaaatt 12300
aatttttttt cgattttttt ttttttgtta gtttattttt ttttatattt tttagtattg 12360
gagattttta tagttttgat ataaaaatga tttgaaatag taaattatta aataagaaat 12420
aaattgtttt ttatgtacgt ggttttaggt atttttaatt aatgtttttt tttattagtt 12480
aatattgaat atttgttata gaattatttt ttttaagttt cgtgtgtgat taaaatgtga 12540
tttatttaat gaagatttaa aatatatttg gaaggtgtag gaatattaga atttaaggga 12600
aaattaaaat taaattttaa tttaacgtaa aaatatttat gattatttat ttttaatgat 12660
aggatatttt agaaatattt ttttttgttt attttttttt tttttttttt gttaggtagg 12720
taaattattt atatcgaaat tttttataag ttttaataga ttatattaat tattattaga 12780
aataattgtt atatgataat ttttaaatat ggattattta tagtatatat ttataattat 12840
aggtaaattt attaagtaaa gttatatgaa gttttttttt ttttttttga atatttatat 12900
ataatgttgt gaatttgaaa ttattttatt agttttgtat attgataata gtattgagtt 12960
ataaagag 12968

<210> 303
<211> 6328
<212> DNA
<213> Artificial Sequence

<220>
<223> chemically treated genomic DNA (Homo sapiens)

<400> 303

cggggatacg ggttggtttt gcgtttttat ttatagtatt ttgtcgtttt ggagaatttt 60
ggagtgggtt tggaattgtt atacgttttt ttaggcgtta agtataaggt taggtttagt 120
atgtgttttt ttgtagtcgt atttttagtt cggttgagta cgatgttatt ttatattgaa 180
gatttttttt ttgattaaat ttgaaattaa atttttatgt tttggaaaat gtattaggtt 240
atgtttttta ttttttttta ttttttgttt ttaggttttt ttttttcggt agaagaaaag 300
agaatttgtt tttaaagatg agttttaggt ttggaaaaat atttcgagag gtggtagttg 360
cgtgtttttt tggtgtttgt atgaaatttt tatagttttt taggttgtga agtttttttt 420
ttttatttaa ggaggggagg gtaggttttt gggggagttt attttttttt aggttttatg 480
tattttaatt atttcgttgg gttgggtaga gagttttgta ttgggatttt gagtagattc 540

gggtttaaat tttagttttg ttattggtta gttatgtatt tttttggggg ttttttagtt 600
ttagtgtgtt tagttattgt ttagtggatt ttgggtagtt tttatttagt ttaggtcgtg 660
aggaggtttt gatgagatag ggttaaatta gagtttaaat tcgagggaga agtgagggag 720
atgtagaaag ggtaatgttt gtttgtttgt aagtttttaa tatagggatg gtggtttttt 780
ggcgggggag ttttttgttt agttgttttt tggatgtttc ggttttagga ttttaattag 840
gttatttgag tttttgggta ttggttttcg ttttggttag tttttggggg aggaagagta 900
ggtagatttg ggggagttta tttttttttt tttttggagg gaggatggag aacgttgggt 960
gtggggatag gggataaagg tattttttgt ttgtaggatg tttattagga ttggattagt 1020
gggggtttag atttttttat ttcggtaaaa aaaaaaaata aaaaaaatta aaaaaaaaat 1080
ttagtttagg tagaagtaaa gttttaaggt atggagattt ttatcgaggt tgtgggggtg 1140
gtgagtagag tagaatgtga aggtggggtg aagttttttc gtcggttatt tttgtttttg 1200
tttttttatt ttcgttggtg ttcgggatta gtaagtttta gaggtttttt tagttttatt 1260
tagttgagtt gttttgatta gtgtttttgt ttttaattag gggcgaagtg gtttggggtt 1320
ggttggagga agagttagtg tttagaaaag gaggaggggg agtagatagg aggggagagt 1380
agaggagcgg ggaggaaaaa gggttggaga taatattttt tttttttttt cggaaaattt 1440
ttgtttatta ttagtttttt tgttgtttcg gtaaagttta gtaatattgt tttcggatag 1500
gttttttttg ttttcggttt ttgtttgcgg ggtgcggggt tataggtaga gttttcggtt 1560
tggttatagg gagggagttt agtttatgta tttcgatttg gttttagagt tggtaggttt 1620
agtttttgta atttgtgtag atcgtttgtt acgttatgtt atgaggattt gcgggttgtt 1680
ggttttattt ttgtaatttc gggggtttgt atacggagta tggttttcga gagaagggga 1740
ggggggagga ggggagggat gcgtagatag aggagggggc gtttagtttt tttagtttgt 1800
cgttttatag tttttaggat ttgtttgtaa atggtagatt gtatcgtttt tttttaaggg 1860
attatattgg tattttcgcg ttttttgtgt agatttaaag tttgtgatag ttattaattt 1920
ttttttttta tcgtttttaa gttatttttt tttagtgttt gtttgttgaa aagaaagttt 1980
cggttttttg aatttataaa cgttttatta agattagagt aagttaatag aaattttttt 2040
ggtttgtagg ttttgtgaga taagaaagtt tttttttatt ttttaagtgt ttttgtatgg 2100
ggtttttgaa gagggttcgg gtttagtgga gtttgggtgt tcgttttgga gattttttag 2160
ttggcggggg taagggtttt ggggtgtagt tgttggagga atggttggaa ggggttgtga 2220
ttaggttttg ttagtatagt gtttttttgt ttataaaaat cggagttttt tttagagata 2280
ggtgggatat ttttgtttat gggttaagaa atagggttta tagatggaag agattgggtt 2340
tttggtgggt acgaaatgga gataggatta gagagtaggt gttttttaag ttaaggtagt 2400
gtttttttag cgtaagtttg tgatattttg atagattttt ttgggcgaga atattttttt 2460
ttggtttttt tttttttttt aaattgggga gggttgtggt ggggttgggg gcgggggtgg 2520
gaagggggaa gaatttggat ggggaaggga ggagtttagg tatgtgtgtg ggtggggata 2580
ttggggtttt ttaagtgttt tatgtagttt agggagtttt ttttttgcgg gattttggta 2640
agagtttggg aggtttttgt tttagggtta tttagttttg agatttttta tttttttttg 2700
gtttttggtt gagaatttag tttttttagg tttgtttgtt gttgggataa ttttggaggt 2760
tgaagttagg ggtttcgaag ggagggaaga gaggaatttt ggttaggaag attttggcga 2820
ttattatgta gtggatattg gtatttaggg ttgggtttgt tagttagata ttttcgtttt 2880
aggttaaggg ttttgggtaa aggatttttt attattgaag ttggtttttt gatttgagga 2940
aagagtttac ggaagaaagt agggttatag gaatgtaagg gtatgtggta ggtatatagt 3000
gggtatttta attgtttttt atagtttgtt tttaaagtcg tttttagtag tttttattta 3060
tttgggtatt ttgtagataa tattagggaa gggatatttt ttattagggg ttgggagttt 3120
ttttttagtt tttttggagt tagtttagag tagttttggt ttttgtttat ttttgtattt 3180
atttcgagtg ttttattgga gtattgattt aggggtggtg ggtttgggga tgtttatggg 3240
tttgtttttt tatatttatg gttaagatgg gaaatttgtg tttagatttg ttagggttgg 3300
attagagggt taggaggtta aagtatttag tttttaggtt tatagtggtg agattatatt 3360
tttttgtagt attgtgaaga aggggaggtc gcgagttggg tgggtattta ttttttgtat 3420
ttaggatatg tatgtaaatt aatatatgta aattaagtat tttaaacgat ttatttaaag 3480
attgggacgt ttgggatttt ttttttagga ggtgaaatat ttttaatttt tttttatttt 3540
tttttttttt tatttttttg agtttttttt atttagattg ggtgtagggg tgtagagttt 3600
tttttttttag tagggtttta agtttaagtc gggggtaatg ggaggagggg gtgtttgagg 3660
agtatttttt ttttttggga gttttgtaga ggaagagggt ttttggtaat tagtattttt 3720
tatttttatt tttttagcgg gcgatttttt agagaaggtt ttttagtgag tttggaatac 3780
gtgtcgagtg tttggagtgt tattgtgtgt atgtgagagt atattggtga ggttgtttat 3840
gtatgttatt gtggataaag atagatgcga tcgtgtgtgt gtggggattg cgtatatgtt 3900
tttgttagtg tgtgtgatta cgagttagtt gggtatgtgt gtttgtgggt attaggtgta 3960
tggttgtgcg tatatgtgtg cgtaagagtc ggtgtatttg tgttttagcg cggttaaggt 4020
tttgattgag atgttgtgtg tgcgggggtg ggtgtgattg aatgcgggta cgggtgttcg 4080
tttgtttata cgtgtgtttg cgtgtttggg tgtgtaggtg gttttgcgta agcgtttgtg 4140
tgtagggggt cggtatatgg gggaggatgt ttatggttgg gtaatcgtag gttcgtttga 4200
ttttttgtta tttaggattg tgtttatacg gcgtttgggt tatagtgtgt ttgagttgtc 4260
gtgtgcgttt tcgttgcggg gtgtgggttg ttacggggta attgtatttt tttgcgtttt 4320
tatggttagg ggtatagggg gcgtcgggtt ttttttgggg gtttttttgt gtgcgtgtgt 4380
aattatgtgt ttagtttata tttttatagt gcgtttttgt gtttttttgg gtatttaatt 4440
attgtacgtg tgttcgggat tttagcgata agtgtttcgg tgtttttgcg tgcggatttg 4500

tgtttttcgg ggaggtttgc ggtttaggtt cgatttttgc gatttgtttt aggtaggttt 4560
gtatgtgcgc ggcggtcgcg tgttgtatag tgtgagggaa cgtgtattaa acgttcgcgg 4620
gatatttgtg ttcgtttagt taagagtgta ttcgtgtgcg cgagcgggtt tttgggacgt 4680
cgtcgtggtc gggggcggtt ttgcgagggg agggggttat agggattggt cggcgtcggt 4740
ttcgtgcgta cggaggcggg ggcggggggc gggggtcgcg aggggggagg cggtacgaaa 4800
agggcggcgc gcgcggcggc ggcggtagtt tttcggtagc ggcggtggag agcgtagcgc 4860
gtagttcggt gtagttttgg tttttttttc gttgcgcgtt cgcgtttttt ttcgcgttcg 4920
taattagaag tttagtgcgg cgttaggagt cggattcgcg ttcgtatcgt tttcgggatc 4980
gcgatttcgg tcgtttagag atgatcgcga tcgaagtttt tttgcgcgtt tttttgtttt 5040
tgttggtttt cggttatagt atttatggtg agtttttcgg cggttcggtt cgcgtttttt 5100
ttggggaagt ttgcgatttt tcgtcggtcg ttcggtgttt cgtacgtttc gtttcgtgag 5160
ttttaatttc gttcgtttcg tttagtttta agtttcgcgt tgtgtttgtt tcgttttatt 5220
tattacgttc gtatgttaag gttcgtttta ggggttttag ggcgtagggg atgtgtgaga 5280
tttttagttt tttttcgttt tgtagaagtg gcgttagagg ggtgtcggtg cgttgagtaa 5340
ggaggtatgg aaaaatgggg tgttgttgtg gagtttttta tgaaaatatt ttggggtgta 5400
attttggggt gtttttgtgt gagcgttttg gaataggttt cggggcggga gtgggggacg 5460
attttgtcgt tttagttttt aatttttaga gaagtttagt ttgagttttt tttgcgtggc 5520
gcgtttttgt aggtgagggg ttgcgatatt tttgtttgta gcggttattt gtttttgata 5580
gcgagagagt tgtttttttt ttgtagcgtt tttttattta ttgtattagt ggttgtaagg 5640
gggttgttta gaaagttggg gagttggttg agtaaatgta tatagtaggt gtttgttaaa 5700
gtgttagaat ttttaagtag tgttgttgta attttagttc gttaagtaaa attttgtgtc 5760
gttatcgttt ttttaatgcg aagagtttgg ggtaggggaa agagggaaga tttgatttgg 5820
gtgttcggtt taatagggat gaattttaaa aagaatgtag acgttttatt ggggcgttta 5880
ttaggtgaat ttgtttgggt tttttcggag ttgaatattt taaaatttgt tttttttttt 5940
agtttaggtt gtaaaagtga cggttaggtt gtatgtttag ggtttaatat attttttttt 6000
acggttttcg ttttcgttgt taggaggatt tgaggtgtcg tatttgtttt tgggagttcg 6060
aggagaaggt gttttttttg tttttaagaa cgagggagag tgtattgtgg ttattttatt 6120
gttgggaacg tggtagagat gttttttttg agtgtattga gttttattta tgttcggata 6180
ggaagtgttt attgtgtgtt aggttggtag gatttaagag ggttgttggt gttttcgagg 6240
gtagtggtgg gttgtgtggg cgaagatagt ttttgttttg gtttggggtt tggtggggtg 6300
aattgtataa tttttttgt attttagg 6328

<210> 304
<211> 6328
<212> DNA
<213> Artificial Sequence

<220>
<223> chemically treated genomic DNA (Homo sapiens)

<400> 304

tttgaggtat aagaaaggtt atataattta ttttattagg ttttaggtta aggtaaaggt 60
tgttttcgtt tatatagttt attattgttt tcgagggtat taatagtttt tttgggtttt 120
attagtttgg tatatagtag gtatttttta ttcgagtatg gatggggttt agtgtattta 180
ggaaaaatat ttttgttacg tttttagtaa tgaaatgatt atagtatatt ttttttcgtt 240
tttaggagta ggagaaatat ttttttttcg gatttttaaa agtaaatacg atattttaag 300
tttttttaat agcggggacg gggatcgtgg agggaaatat gttgagtttt gggtatgtag 360
tttgatcgtt atttttgtaa tttgagttga aaagaggagt agattttgag gtgtttaatt 420
tcgggaaagt ttagataggt ttatttggtg ggcgttttag taaggcgttt gtattttttt 480
taaggtttat ttttattaag tcgggtattt aggttaaatt tttttttttt tttttatttt 540
agatttttcg tattaaaaaa acgatgacga tatagggttt tgtttagcgg gttgaggttg 600
taataatatt gtttagagat tttgatattt taataggtat ttattgtgtg tatttattta 660
attagttttt tagtttttta gatagttttt ttataattat tggtgtaatg agtggggggg 720
cgttgtagga agggggtaat tttttcgttg ttagagatag atggtcgttg tagatagaag 780
tgtcgtagtt ttttatttat agaaacgcgt tacgtaggaa gggtttaggt tgggtttttt 840
tgggaattgg gggttaagac ggtaaagtcg ttttttattt tcgtttcgag atttgtttta 900
gaacgtttat atagggatat tttaaagttg tattttagag tatttttata gaagatttta 960
taatagtatt ttattttttt atattttttt gtttagcgta tcgatatttt tttggcgtta 1020
tttttgtagg gcgggaaggg gttgggggtt ttatatattt tttgcgtttt gggatttttg 1080
ggacgggttt tggtatgcga gcgtggtggg tagggcgaga taggtatagc gcggggttta 1140
gggttaggcg ggacgggcgg agttggggtt tacgagacgg ggcgtgcggg gtatcgggcg 1200
gtcggcgggg agtcgtaggt ttttttagag ggggcgcgag tcgggtcgtc ggggaattta 1260
ttataggtgt tgtggtcgaa agttagtagg agtaagagga cgcgtaggag ggtttcggtc 1320
gcggttattt ttgggcggtc ggggtcgcgg tttcgggagc ggtgcgggcg cgggttcggt 1380
ttttggcgtc gtattgggtt tttggttgcg gacgcgaaag ggggcgcggg cgcgtagcga 1440

ggggaaagtt agggttgtat cgggttgcgc gttgcgtttt ttatcgtcgt tgtcggggag 1500
ttgtcgtcgt cgtcgcgcgc gtcgtttttt tcgtatcgtt ttttttttcg cggttttcgt 1560
ttttcgtttt cgttttcgtg cgtacggggt cggcgtcggt tagtttttgt gatttttttt 1620
tttcgtaggg tcgttttcga ttacggcggc gttttagaag ttcgttcgcg tatacgggtg 1680
tatttttggt tagacgggta taggtatttc gcgagcgttt ggtatacgtt tttttatatt 1740
gtatagtacg cggtcgtcgc gtatatatag gtttgtttag gataagtcgt aggaatcgaa 1800
tttgggtcgt agattttttc ggagagtata aattcgtacg taggaatatc gaaatattta 1860
tcgttgaagt ttcgggtata cgtgtaatgg ttagatattt aggaaaatat aaaggcgtat 1920
tatgaagatg tgaattaagt atataattat atacgtatat agaaaaattt ttaggaaaag 1980
ttcgacgttt tttgtatttt tggttatgaa gacgtagaaa aatgtagttg tttcgtggta 2040
atttatattt cgtaacggaa acgtatacgg taatttaaat atattgtagt ttagacgtcg 2100
tatagatata gttttaggtg gtagagggtt aagcggattt gcggttattt aattatgggt 2160
attttttttt atgtgtcgat tttttgtata tagacgttta cgtagagtta tttgtatatt 2220
taaatacgta aatatacgta taaatagacg gatattcgtg ttcgtattta gttatattta 2280
ttttcgtata tatagtattt tagttagggt tttagtcgcg ttgaaatata aatgtatcga 2340
tttttacgta tatatgtacg tatagttatg tatttgatat ttataagtat atatatttag 2400
ttgattcgtg gttatatata ttgataaaaa tatatacgta gtttttatat atatacgatc 2460
gtatttgttt ttgtttatag tggtatgtat gggtagtttt attaatgtgt ttttatatat 2520
atataatagt attttaggta ttcggtacgt attttagatt tattgggaga ttttttttga 2580
ggggtcgttc gttggagagg tggaggtaga ggatgttgat tgttagagat tttttttttt 2640
tgtagagttt ttagggagag ggagtgtttt ttagatattt ttttttttta ttgttttcgg 2700
tttaggttta gagttttgtt ggagaggaaa gttttgtatt tttgtattta gtttggatgg 2760
gggaggttta gaaagatagg gagggagagg ggtaggaggg gattaaagat attttatttt 2820
ttgggggaag ggttttaggc gttttaattt ttgggtgggt cgtttaggat atttaatttg 2880
tatatattaa tttgtatata tattttagat gtagggagtg ggtgtttatt tagttcgcgg 2940
tttttttttt tttatagtgt tgtagggggg tgtgatttta ttattgtgaa tttggaggtt 3000
ggatgttttg attttttggt tttttagttt agttttggta ggtttgagta tagatttttt 3060
attttagtta tagatgtggg aaggtaggtt tatgggtatt tttaggttta ttatttttaa 3120
gttagtgttt tagtaaggta ttcgggatgg gtatagggat ggatagagat tagagttgtt 3180
ttgggttggt tttaaggagg ttggagggaa atttttagtt tttggtggaa gatatttttt 3240
ttttgatgtt atttgtagaa tgtttagata ggtaggggtt gttgggaacg gttttgggga 3300
taggttgtag ggagtagttg gggtgtttat tgtgtgtttg ttatatgttt ttgtattttt 3360
gtgattttat tttttttcgt ggattttttt tttaagttaa gaaattaatt ttagtgatgg 3420
aaggtttttt gtttaaggtt tttggtttag aacgagagtg tttggttgat aggtttaatt 3480
ttgggtgtta atgtttattg tatggtgatc gttaaggttt ttttgattag ggtttttttt 3540
tttttttttt tcgaggtttt tgattttagt ttttaaaatt attttaatag taggtagatt 3600
tggaagagtt gagtttttag ttagaagtta aggaggggtg gaggatttta gggttgggta 3660
gttttggggt aagggttttt taggtttttg ttaaagtttc gtagagagga gattttttgg 3720
attgtatgga gtatttaggg ggttttagtg tttttattta tatatatatt tagatttttt 3780
ttttttttat ttaggttttt tttttttttt attttcgttt ttagttttat tatagttttt 3840
tttagtttag gaaagggagg gaggttagag gagggtattt tcgtttaagg gagtttgtta 3900
aaatgttata ggtttacgtt gaaagggtat tgttttggtt tggggggtat ttgtttttta 3960
attttatttt tatttcgtgt ttattaaggg tttagttttt tttatttgtg agttttgttt 4020
tttagtttat aaataggaat gttttatttg tttttgaaag gaatttcggt ttttgtaagt 4080
agaaaagtat tgtgttgata gggtttggtt ataatttttt ttagttattt ttttaatagt 4140
tgtattttag agtttttgtt ttcgttagtt agaggatttt taaggcgggt atttaggttt 4200
tattggattc gggttttttt taagagtttt atgtagggat atttgaggga tgggagggga 4260
tttttttgtt ttataggatt tataagttag gaagattttt gttggtttgt tttaatttta 4320
ataaagcgtt tgtaaattta ggaggtcgga gttttttttt tagtaagtag atattgagaa 4380
agggtagttt aggagcggta gggaggggag gttggtgatt gttatagatt ttaagtttgt 4440
atagaaggcg cgaaaatgtt aatgtagttt tttggggaga agcggtatag tttgttattt 4500
gtaagtagat tttggggttt gtggaacggt aggttggagg agttggacgt tttttttttt 4560
atttgcgtat tttttttttt tttttttttt tttttttttc gagagttatg tttcgtgtat 4620
aggttttcgg gattatagag atgaggttag tagttcgtag gtttttatag tatggcgtgg 4680
taggcgattt atataggttg tagggattgg atttgttaat tttggggtta gatcgagatg 4740
tatgagttgg attttttttt tgtagttaag tcgaaggttt tgtttatggt ttcgtatttc 4800
gtaggtaaga gtcgaaagta aggggaattt gttcggaggt agtgttgttg ggttttgtcg 4860
gggtagtaga ggagttggtg atgagtaaaa gtttttcgag ggaaggaaag aagtgttgtt 4920
tttagttttt tttttttttc gtttttttgt tttttttttt tgtttgtttt tttttttttt 4980
tttttaaata ttaatttttt ttttagttag ttttagatta tttcgttttt gattaaaggt 5040
agaaatattg attagggtag tttagttggg tggagttaga ggggtttttg agatttatta 5100
gtttcgggta ttagcgggga tgggagggta agagtagggg tggtcggcga gggggtttta 5160
ttttattttt atattttgtt ttgtttatta tttttatagt ttcggtgggg atttttatgt 5220
tttgaggttt tgtttttgtt tagattaggt tttttttttg gttttttttg tttttttttt 5280
ttgtcggggt gggggatttt gagttttat tgatttagtt ttagtgggta ttttatagat 5340
aagagatgtt tttgtttttt gtttttatat ttaacgtttt ttattttttt tttaagagag 5400

aaggagggtg ggttttttta gatttgtttg tttttttttt ttaagggatt gattaaggcg 5460
aggattagtg tttagagatt taggtggttt agttagagtt ttagaatcgg aatatttagg 5520
agatagttgg gtaggaggtt ttttcgttaa gaagttatta tttttgtgtt gagggtttat 5580
aggtaagtag gtattgtttt ttttgtattt tttttatttt tttttcggat ttagatttta 5640
atttaatttt gttttattag agttttttta cggtttgggt taggtgggga ttgtttagag 5700
tttattgggt agtggttgag tatattgagg ttgggggatt ttaagagagg tgtatggtta 5760
attagtggta gagttggaat ttgaattcgg gtttatttaa aattttagtg taaggttttt 5820
tgtttagttt agcggggtgg ttagagtgta tggagtttgg aggaaagtgg gtttttttag 5880
aagtttgttt tttttttttt gggtgggagg gggaagtttt ataatttggg aggttgtgag 5940
ggttttatgt aaatattaag gagatacgta gttgttattt ttcggagtat ttttttaaat 6000
ttgaagttta tttttggaga taaatttttt ttttttttat cgaggagagg ggatttgggg 6060
gtagagggtg gagggaaatg aaagatataa tttgatatat tttttagaat atgaaggttt 6120
ggttttaagt ttaattagag aaaaggtttt tagtgtgaag tggtatcgtg tttagtcgag 6180
ttgggagtac ggttgtagag ggatatatgt tgagtttggt tttgtgtttg gcgtttgaag 6240
aggcgtatga tagttttagg tttattttag ggttttttag ggcggtaggg tgttgtgggt 6300
gggggcgtag agttagttcg tattttcg 6328

<210> 305
<211> 7046
<212> DNA
<213> Artificial Sequence

<220>
<223> chemically treated genomic DNA (Homo sapiens)

<400> 305

aggtattcgt taaaaatttt ttgggatgtg aattttttgt ttttttagat tttggatgtc 60
ggaatatttg ttttttgaga tgtatttgta ggagtatttt tgtgtggaat atttgaaagt 120
tattttgaaa gtaaaggtta gagataggaa ttgaaatagg gtttaatttt taaataagaa 180
ggtataagat attgtagttg atggaattgt ggtgagtatg ttttttaagg gtggtgagta 240
tgtttgtgtg ggtatagaag atatatgtac gaatattttt attttatttt atttagatgg 300
agttttattt ttgttgttta ggttggagtg tagtggcgta atttcggttt attataattt 360
ttgtttttcg ggtttaatga tttttttgtt ttagtttttt gagtagttgg gattataggt 420
atgcgttatt atgtttggtt aattttgaat ttttagtaga tatgggattt ttttatgttg 480
gttagggtgg tttcgaattt ttgattttag gtgatttatt tgtttcggtt tcgggattat 540
aagtgtgagt tattatgttt agttaaatat ttttattttt aatagtttaa tgaatgtata 600
atgattaaat gtttagtatg tattaaatta tgattttatg tagttttttt aattatatta 660
attatgattt tatatggatt atttaagtat attaattata attttatgta tattattgtt 720
taagatagta atgatataaa atttttttaa aattatttat gtaaaagaaa taagttagtt 780
aaaaggaaaa tattaagtaa aaatgtatag gtggtattta aatatggtaa aaattgtgaa 840
gtagtttaat gaataattga attttgggaa atgttagatt agagaattgg aaatattttt 900
ttaaataaat atttgttatt tatgattatt ttatggatga gtgtttaaga aatagagatg 960
tatatgttgt atgtttttaa aatgtatagg atgaatttat attgattatt atattttttg 1020
tgttaaatta attattattt tttggattat agggagaagt tgtgtttttt tatagttttt 1080
ggagatgtgt gatgtgttat aggtttaata attatattat atattttttt ataaaagtgt 1140
tatatagagt agaaatgtat aatagttatt aatttaagat taaattaggt ttagtggtgt 1200
ggatagtttt atttgtttta tgataaaata tttttttatt ttttgtgtga tttttaagta 1260
atttaacgat tgttagtttt gttgagttaa atgtaatatt ttatattaaa tttattggtt 1320
tggtaaagtt ataaatttta ttacgtaaaa gttgagaaag gaaagttgga gaatgttaaa 1380
aatagtataa tttgttattg tgtagtattt gtattggggg tttagtatgt tttatttata 1440
gatatttatt aatatagaga tatagaaaga aatatataaa aaatagtttt attaaatatt 1500
ttttagtatt taagtgtagt tttaaaagta agaataggtt aggagtggtg gtttacgttt 1560
gtaattatag tattgtggga ggttaaggta agaggattgt ttgaggttag gattttaaga 1620
ttagtttagg taatatagtg agatttttat ttttacgaaa aaattttaaa atttagttgg 1680
gtatggtggt ttgagtttgt tgttttagtt atttaggagg ttgaagtagg agtgttattt 1740
gagtttagga ggttgaggtt gtagtgagtt ataattgtat tattgtattt tagttttgga 1800
gatagagtga gattttgttt ttaaaaaaat taaaattgag aaaaaaaaaa aaggtaagaa 1860
tagttatagt aaatttttta ttggggaaaa aaaaaaattt ttttttttat attttttttt 1920
tttttttttt ttttttttga gagtgattgt ggttaaaagg agtatttttt ttttgtagtt 1980
ttttgagggg tggggtgggg ttatgaagtt attttttata tttatttttt tgtttagttt 2040
tttttatttt tagttttttt tttcgtattt ttgtttagta gtgttttaag tggaggaggg 2100
gtgggggtat taagtttgta aaattggttt gttggggttt tttttttttt tttatttttt 2160
gatttttggg aaaatgtttt gttgggaggt tgtttggtag tgttttagtt gttttttgtg 2220
ggtttgaatg gggttttttt ttgtttttat aggaggaaaa gggagttgtt gttagaggga 2280
gaaatggaga gatggataga gaaggtaggt gttatttttc gtttttgata tataaagaaa 2340

aagatacgga aattttttt ttttttttt tttttttatt ttttttttt ttttttttt 2400
tttttttttt ttttttttat atatatatat atatatatat atatatatat atatatatag 2460
gcgcgcgcgc gcgcgcgtag gtatacgttt tgtaaattta ggatttaaag agataggggt 2520
attattatat ttggtacggt ggggtttttt taggtttgaa attttgtatt tttttttatt 2580
atttattttt ttcgagttcg agaagggtta ggtgtgggcg gatggttggt tacgttttgt 2640
gtttttaatt tatatttacg ggatgatata gacggggcgt ggtgagtgtt gttggaggcg 2700
tttgggtagt tttattttgt tttatttttt tatttgaagg ttgggcgttg ttggaatttg 2760
taggggtagt tttagtaagg tggggtggcg tggagtgggg tgggagaagg gattttagtt 2820
gaagtagaat ttaggttgga tttgagaata ttggggaggg tatgggcggt ggttttcggg 2880
taggggtttt gagaatatgt tggttttgat tgttgttagt gtttggttaa agttgttaaa 2940
aggttaaaaa aaaaaaagta gggggagttt ttgttaagat atatttttta ggttattttt 3000
ttttcgcggg agtgttgggg gggaggcgtt gtttggaatt tgtgaatgtg atattagttt 3060
tttttttttt ttttaaggtc ggttttggag agggaggtta gggtattttt gtttggtata 3120
ggcggtagcg ttggttttcg gtttagtgtc gtttgttttt cgggagttgt ggtttttttg 3180
ggtttcgggg ttaggttgag gtaagcgtat agcggaggtt aggggtttcg gtagaggttt 3240
tggggatagg gtggaggtat ttttgggtgt gggtgtgggt gtgggtgtgg gagggagagt 3300
ttttgttttt ttttttttta tttttaattt ttgttttagt ggttttttta gaggatgtat 3360
gttattatgg atttgtcgtt gttattgttt ttgttttttt agttgtgatt tcgagggagg 3420
tttggggatt tgagtttgtt taaatttacg gttttgttgt tgggataaaa attgtttttt 3480
tgattttaga aggaggaggg aaaaaaggtt ttttagtatg tgtgttgtgt tagttttgga 3540
aatttattcg tgtttgaatt ttatttttta tttttagaaa aattggagta aaataaaaag 3600
aggagatgga taaagtgtgt atttgatggt atttttgggg aagagatttt aaatttattt 3660
tataggtttt attgggttat tgtgagcgtt ttggtggaga ataaataaaa attttgggtg 3720
tttagttgtt taatttgaaa aatgggatta gcggaaaaag ttaatgtgtt ttatgtattt 3780
tttgtttttt ttattaaggt atgatgttat ttgtatagta attgttttgt gtgtatttta 3840
gggggggatt ttaaggttag atagatagga aattgttttg aaaatgtaaa tatattatta 3900
aatgtgaagt attatttgat tttttgttcg aatggtattt tttttttagt attatttttt 3960
ttgtatattt atttaatttt gtataagaat atttttttgt tttaaatgaa gatatttttt 4020
taaaaaaaag agttttagaa aatatgtttt tgtttgtgcg gggaataaat agaatatttt 4080
gaggtgtatt tttttttttt atgttaggta atattttttg atttttttcg gtttttaagt 4140
taggttgcgt ttttttttgt tatttagaag ggtttttttt tttgttttag taaaatatta 4200
gtttttgtag tttttttattt tttttggtg tttttttttc gttatgtttt aagattggtg 4260
gtatcgatta atttaagat ttaagtttg tgtgaaaaat atttttgttt tttaattagt 4320
ttattagttt ttttcgtagg ggaagtgtgg atatataaaa gaatttatcg gggtttttta 4380
ttagtgatag ggaaaagatt gggtatgtgt ttaaacgagt tttgatgtta tttttaagtt 4440
tttttttttg ttaatttttt acggattttt tttcgataga tgtaaagaat tttagtaaaa 4500
aagattcgta ggaaggggtt tgaagagaaa agtacgttga tttgttaaaa tagtttgatt 4560
tttagtagtg gggtagtgac gagggagagt atttttttgt ttgattgaga ttagaatcgg 4620
agagatataa aaggaaaatg aagcgagtaa taattaaaaa aaatttttcg tatataataa 4680
tataatttat ttaaattgtg gtttatattt tttatattaa tggtatgatt ttttttttgg 4740
agtttttttt tttgattttt gaatttcggg gttggtagtt tgtaaagggg aagcggattt 4800
tagtattgta cgggtaggtt tagtaaaggt ttttaatggg tatttttttt tttttagttt 4860
tgttttcgaa ttgttagacg gcgggcgttt gtttttgaag ttagtagtga ttttttttcg 4920
ggtttggttt tattttcggt tgtacgttgt ttgttggtga ttaataatat aataatattg 4980
tttggggttg gaataaagtc ggagttgttt atttttattt taataggggt ttaatataaa 5040
aagtcggtag agagttgttt aagttagacg cgtttttgta tttgcgttag gcgaacgggt 5100
tttgcgtttt ttgtagtttt agttttttat cgtcgcgtgc gtttgtagac gtttcgttcg 5160
ttgttttttt ttttggtagg cgttgttttt ttttttcgtt aaaagggtat ttgggttgaa 5220
ggatcgtttt gagatttgag gaattcgtag cgttttgagg gatttgaagt tgtttttttt 5280
cgtttttttt tgggtttagt ttgaacggga ggtttttgat tttttttttt tagaatggat 5340
tatttgttta tgattttttt tttgttgttt gtggtttgtt aaggagtttt agaaataggt 5400
aggtacgttc gttgatttgt aagtttcgga attataagtt agtgtgtttt tatttatttt 5460
tatgtttttt ttgtttttat tttttttcgt tttttttatg attgtagttt agagagtaag 5520
tgtttgagaa ttattgttga aagttatttt aagtttttta gtgtaaaatg taaaattttt 5580
ttattgaata taattaggtg taattgatta taatatgata ttaaaataat ttatcgtttt 5640
attattatta ttttattatg tgtttttttg gtttttaaaa aatgagaaga gtatggatat 5700
atataattta gttaaatgta tgtttgtaat atatgtgttt atataggtat ataggttata 5760
taggaattta aattttattt aaatattatt ttaatagtgt gttaacgtgt aaaatattta 5820
agtattttag tttgaagtta aggaattgta tttagtcgtt taagtaatgt atgtttagta 5880
aaattatttg tagagtaaaa gtttgttgat taattatcgt tttttttttt tttcgttatt 5940
atttttcgta ggcggttttt gggtgaagta gatgtttttt ttaaaatttg ttattattga 6000
ttttaggttt tttttggtag gtttttggta tttaaaatag tgtgagtttt ttttttagtt 6060
ttatttattt gtgttgggag gggagttagg ataatttttg gttgtcgaag gatttaggta 6120
gtgcgtgtgt atttgttcgg gtttttttcg tttttagggt tagtgtattt tttttgtttt 6180
ttcgtgattt tgattaaaga gaaaggatgt taagggaatg aaaattttgg aatgtgtttg 6240
attatttgaa atgtataaaa ttgggtagat aagttgtatg gttaaattgt taggaggaag 6300

aggtaaggta gtagtggaga agggggaggt agtggatttt atataagttt gatgtttagg 6360
gattcggaat ttaaaatttt tttagtttat ttttagtttt ttgatttgtt ttttagtttt 6420
attttaggtt attggttttt atggagttat ttttttgaat tgaatattga atagttaatt 6480
ttttttttta attatttttt ttatttaatt ttgaaagata tatattattt ggggtatttt 6540
gtgttttata tagtatgtga agtggatggg tattttttaa agagagggtt attttgaatg 6600
gggaagtggt tttaaagtta ggaataattg tgattttttg tttttagtta tgtgttaatg 6660
ttaagtaagt tttagtggat agtgttgttt tattaagttt tttgtagaag ttagtcggat 6720
ttttaatagg tagtatttta tagtattttt ttgagtttgt tttaagaggg gtgggggaag 6780
ttttttttta ggtgtttatt ttttttgtat ttgtgtaatt tttttgaagg tggataagtt 6840
aagggtatga gggggaggta aaaggtgaat ttatgttaag gagggaaaaa aataaagagt 6900
tttttttttt gtgttttttg ttgatggtag gttgtgtgtt ttatttgttt ttatttgttt 6960
tgttagtttt gattttattg tgatttagta tgtttttcgg cgtttgagga gatatttttt 7020
attgatttgt tttttttttt tttagt 7046

<210> 306
<211> 7046
<212> DNA
<213> Artificial Sequence

<220>
<223> chemically treated genomic DNA (Homo sapiens)

<400> 306

gttggggaga gaaagagtag gttagtgggg gatgtttttt taaacgtcga gagatatgtt 60
ggattatagt agagttagag ttagtagagt agataaaagt agatgaagta tatagtttgt 120
tattagtaag aaatatagaa aaaagggttt tttatttttt ttttttttta atatgagttt 180
atttttttgtt ttttttttat gtttttggtt tatttatttt tagggagatt atataaatgt 240
agaggagata aatatttgaa aagggatttt ttttattttt tttgaagtag gtttagggaa 300
atgttgtgga atgttgtttg ttgaaaattc ggttggtttt tataaggaat ttggtaggat 360
agtattattt attgaagttt atttaatatt ggtatatgat taaagataag aaattatagt 420
tatttttagt tttggggtta tttttttatt taggatgatt ttttttttag ggggtattta 480
tttattttat atgttgtgta gggtataggg tattttagat gatgtatatt ttttaaaatt 540
aggtggggaa aatgattgga gagagaaatt aattatttaa tatttaattt aggagggtaa 600
ttttatagaa attagtgatt taaggtgggg ttgagaagta ggttagggga ttgaaggtag 660
gttgggggga ttttgaattt cgaatttttg ggtattaggt ttgtgtggga tttattgttt 720
tttttttttt tattattgtt ttgttttttt tttttaataa tttagttatg tagtttattt 780
gtttaatttt gtatatttta aatgattaga tatattttag gatttttatt tttttgatat 840
tttttttttt tagttagggt tacgaaaaga taaaaaaagt gtattgattt taaaaacggg 900
ggggattcgg gtagatgtat acgtattgtt taaatttttc ggtagttaag aattatttta 960
gttttttttt tagtataggt gaataaagtt gaaaagagag tttatattgt tttgggtgtt 1020
aaaaatttgt taaaagaaat ttaaagttaa tgatgataaa ttttaaagaa aatatttgtt 1080
ttatttagaa atcgtttgcg gggggtggtg gcgggggggg ggggaggcgg tagttagtta 1140
atagattttt gttttgtagg tgattttatt gaatatatat tgtttgaacg attggatata 1200
attttttggt tttaagttgg aatgtttaaa tattttatac gttaatatat tattaaaata 1260
gtgtttaaat aagatttaag tttttatatg gtttgtgtat ttgtataaat atatatatta 1320
taaatatata tttgattaaa ttgtatatgt ttatattttt tttatttttt aaaagttaag 1380
gaaatatata atggaataat aataataaaa cgataagtta ttttaatgtt atgttatagt 1440
taattgtatt taattgtatt taatagagga attttatatt ttatattaga agatttaaag 1500
tagtttttag taataatttt tagatatttg ttttttaagt tgtagttata aaaagaacgg 1560
ggaaaaatag aagtaagaaa agtatgaagg tggataagaa tatattaatt tgtaatttcg 1620
agatttataa gttaacgagc gtgtttattt gtttttggag ttttttggta agttataaat 1680
agtagagaga aaattatgag taaataattt attttgaaaa aaagggatta aaaatttttc 1740
gtttaaattg aatttaaagg aaaacgaaga aaaatagttt taggtttttt aaagcgttgc 1800
gggtttttta gattttaaag cgatttttta gtttaagtgt ttttttaacg gggagaaaag 1860
gtagcgtttg ttaggagaga aggtagcgag cggagcgttt gtaggcgtac gcggcggtgg 1920
agagttggga ttgtaggagg cgtaggattc gttcgtttgg cgtagatgta gaggcgcgtt 1980
tgatttggat agttttttgt cggtttttta tattgaattt ttattagagt gggggtaaat 2040
agtttcgatt ttattttagt tttagataat gttattgtgt tattagttat taataggtaa 2100
cgtgtagtcg gagataaggt taggttcgaa aggaaattat tgttaatttt agaggtagac 2160
gttcgtcgtt tgataattcg ggggtagggt taagaaaaag aaaatattta ttagagattt 2220
ttgttaaatt tgttcgtgta gtgttggagt tcgttttttt tttgtaagtt gttagtttcg 2280
gagtttaaga attagaagag gggattttag aaaaaaagtt atattattgg tatgaaaagt 2340
atgagttata gtttaaatag attgtattgt tgtgtgcggg gaattttttt taattgttgt 2400
tcgttttatt ttttttttat gttttttcga ttttagtttt agttaaataa gggaatgttt 2460
tttttcgtta ttgttttatt attgggggtt agattatttt ggtagattaa cgtatttttt 2520

tttttaagtt tttttttgcg ggtttttttt gttgaagttt tttgtattta tcggagaaag 2580
attcgtgagg gattggtaag aaagggagtt taaaaataat attagagttc gtttaggtat 2640
atgtttagtt ttttttttat tattgatgag aagtttcgat aagttttttt gtgtgtttat 2700
attttttttg cggaggaggt tgataaattg attgaaaagt aaaggtgttt tttatataga 2760
atttaaattt taagattggt cggtgttatt aattttaaga tatggcggga gaagaatatt 2820
agggggagat gaagagttat aggggttgat gttttattag gataaaaagg aaaatttttt 2880
taaatggtag aaaaaaacgt aatttggttt gggggtcgag gagggttaag gaatgttgtt 2940
taatatagga aggaggaatg tattttagaa tattttattt atttttcgta taagtaggga 3000
tatatttttt gggatttttt tttttggggg ggtgttttta tttagggtaa aaaggtgttt 3060
ttgtataagg ttaagtgaat atgtaaggaa ggtggtgttg agaaggaaat gttattcgaa 3120
taaggaatta gataatattt tatatttaat aatgtgttta tatttttaaa ataatttttt 3180
gtttatttaa ttttgaaatt tttttttgaa gtatatatag ggtagttatt gtataggtga 3240
tattatgttt taataaagaa agtaaaaggt gtatggaata tattggtttt tttcgttagt 3300
tttatttttt aggttagata attgagtatt tagaattttt gtttgttttt tattaaagcg 3360
tttatagtgg tttagtaaga tttatgggat aaatttagag ttttttttta ggggatgtta 3420
ttaaatatat atttgtttta tttttttttt ttgttttatt ttagtttttt tggggatgga 3480
gggtggaatt taagtacgga tgaattttta agattggtat aatatatatg ttgggaaatt 3540
tttttttttt ttttttttta aaattaaaag gatagttttt attttaatag taaagtcgtg 3600
ggtttggata gatttagatt tttagatttt tttcgaagtt atagttgggg gaatagggat 3660
agtggtagcg ataggtttat aatgatatgt attttttaaa agagttattg aagtaagagt 3720
tggagatggg agagagagag gtaagaattt ttttttttat atttatattt atatttatat 3780
ttagagatgt ttttatttta tttttagggt ttttgtcggg gtttttggtt ttcgttgtgc 3840
gtttatttta gtttagtttc ggggtttagg gaggttatag ttttcggagg ataggcggta 3900
ttgagtcgga agttagcgtt gtcgtttgtg ttaggtaagg gtgttttgat tttttttttt 3960
aaagtcgatt ttgggagagg agaggagagt tgatgttata tttataggtt ttaagtagcg 4020
tttttttttt aatattttcg cggaagaaag gtggtttggg aaatatgttt tggtagggat 4080
tttttttatt tttttttttt taattttttg gtaattttga ttaaatattg ataatagtta 4140
ggattaatat gtttttaagg tttttattcg gaaattatcg tttatgtttt ttttaatatt 4200
tttaggttta gtttgggttt tattttagtt ggagtttttt tttttatttt attttacgtt 4260
attttatttt gttgaggttg tttttgtagg ttttagtaac gtttagtttt taggtggaga 4320
agtggggtaa aatgaaattg tttaagcgtt tttaatagta tttattacgt ttcgtttgtg 4380
ttatttcgtg aatatgaatt ggaaatataa aacgtggtta gttattcgtt tatatttggt 4440
ttttttcgag ttcggggaaa gtaaatagta aggaagaatg taggatttta gatttggaag 4500
ggttttatcg tgttaaatat aatggtgttt ttgttttttt gaattttgaa tttgtaagac 4560
gtgtgtttgc gcgcgcgcgc gcgcgtttgt gtgtgtgtgt gtgtgtgtgt gtgtgtgtgt 4620
gtgtgtgtga gagagagaga gagagagaga gagggagaga gagagagata ggagaagaga 4680
agagagagag agaattttcg tgtttttttt tttgtgtgtt aggggcgagg ggtggtattt 4740
gttttttttg tttatttttt tatttttttt tttggtagta gttttttttt ttttttgtag 4800
gggtagaggg aagttttatt taagtttata gaaggtagtt agggtattgt taggtagttt 4860
tttagtaaga tattttttta agaattaaga aatgagggga gaaggagaat tttaataaat 4920
tagttttata agtttgatgt ttttattttt tttttattta aggtattgtt agatagagat 4980
gcggggagaa gaattagagg tgaaaagagt tggataaagg agtgaatatg aaggatagtt 5040
ttatagtttt attttatttt ttagaggatt gtagggggaa aatgtttttt ttggttatag 5100
ttatttttag aaaggaaggg gaaggaaggg agagatgtaa agaggaggat tttttttttt 5160
ttttaataga aagtttgttg tggttgtttt tgtttttttt ttttttttta attttaattt 5220
ttttggggat agggttttat tttgttttta aggttggagt gtagtggtgt agttatagtt 5280
tattgtagtt ttaatttttt gggtttaagt gatattttta ttttagtttt ttgagtagtt 5340
gggataatag gtttaagtta ttatgtttag ttaagtttta aaattttttc gtagagatag 5400
ggattttatt atgttgttta ggttggtttt gaaattttgg ttttaagtaa tttttttatt 5460
ttggtttttt atagtgttgt gattataggc gtgagttatt atttttggtt tatttttgtt 5520
tttgaggtta tatttgaatg ttggaaagta tttgataaaa ttatttttta tgtatttttt 5580
tttgtatttt tgtattaata gatatttata aataaaatat gttgagtttt taatatagat 5640
attatatagt agtaaattgt attgttttta gtatttttta gttttttttt tttaattttt 5700
gcgtaataaa atttgtaatt ttgttaagtt aataaatttg gtatgagatg ttgtatttag 5760
tttagtaaag ttaatagtcg ttaagttgtt tgaaaattat ataaagaatg aggaagtatt 5820
ttattatagg taaagtgaag ttatttatat tattaggttt gatttagttt tgggttggta 5880
attattatat atttttattt tatgtagtat ttttgtgaaa aaatgtgtaa tatagttatt 5940
aggtttgtga tatattatat atttttagga gttatgaaga aatatagttt tttttatag 6000
tttaaaaaat aatggttgat ttagtatagg aaatatgatg gttaatgtaa atttattttg 6060
tatattttaa aaatatataa tatatgtatt tttatttttt ggatatttat ttataggata 6120
attatagata gtaagtgttt atttaaaaga gtatttttaa ttttttaatt tagtattttt 6180
taaagtttag ttatttatta aattgtttta taatttttat tatatttggg tgttatttgt 6240
atatttttat ttaatatttt ttttttaatt gatttgtttt ttttatatga ataattttaa 6300
gaaaatttta tattattatt attttaagta ataatgtata tgaaattatg gttggtatgt 6360
ttaaataatt tatatgaaat tatggttggt atggttaaaa aaattatatg aaattatagt 6420
ttaatatatg ttaaatattt aattattata tatttattaa gttattaaaa ataaaaatgt 6480

ttggttgggt atggtggttt atatttgtaa tttcgaggtc gaggtaagtg gattatttga 6540
ggttaggagt tcgagattat tttgattaat atggagaaat tttatgttta ttaaaaattt 6600
aaaattagtt aggtatggtg gcgtatgttt gtaattttag ttatttaaga ggttgaggta 6660
ggagaattat tgaattcggg aggtagaggt tgtggtgagt cgagattgcg ttattgtatt 6720
ttagtttggg taataagagt gaaattttat ttaaataaaa taaaataaaa atgttcgtat 6780
atgtattttt tatgtttata tagatatgtt tattattttt ggggaatatg tttattatag 6840
ttttattaat tgtaatattt tatatttttt tgtttaagaa ttaagttttg ttttaatttt 6900
tgtttttgat ttttattttt aagataattt ttaaatattt tatataaaaa tgtttttata 6960
gatgtatttt aaaaaataga tgtttcggta tttagaattt agaaaaataa aaggtttata 7020
ttttaaaaag tttttaacgg atattt 7046

<210> 307
<211> 7306
<212> DNA
<213> Artificial Sequence

<220>
<223> chemically treated genomic DNA (Homo sapiens)

<400> 307

gttaatattt tttttttttg agtttattat tagtttttga ttaggtaatt ttttatttag 60
atattttggt gaattgttag tgtagaagga gaataaattg gtaagtgatt ttgagtgagt 120
ttatttttat ataggaaggg tgtttgaaat tttttatttt tagaatttta gaaagatagt 180
taattttttt ttgtttagaa tggattgagg ttgttttcga ttaggttgtt tgagtttttt 240
tatttttatt tattatagtt atatttgttt gggtttttgt tttttttttg gagatgtttt 300
ttgagagtag gtttttttag tttttttata gtttttttga ggtttgttaa aagagattgg 360
tagagaacga ttagtatttt atgtttagat aatttatata attttttttg aaaagaatgt 420
taaaatttag tttttaagtg aatttacgag gagtggaatt aaagggattt ttgtattttt 480
tttttggaat ttaatttaga atttatgttt ttatggggtg ggaagagaat agagagatgg 540
gaagtatgaa gaggggaaga taaagaagta ggaataggaa tatatgggaa aaataattat 600
aggtttttag agttaaaagg attttaagga atatgttatt tggaggtttt taaacgtttt 660
gtttttagta ttagaatttt tttattttta ttttttacgt aagaaattat ataatgagtt 720
ttattatata aaatagatga aaagagaaag agaaataaat agttgaatga aaatagttaa 780
aatagatgaa atttattgtt gaaatattgg cgagggaatt tttattattt tttattattt 840
ttagattgtt gaagaatata aagtgaaaat tgttaatttt gtttaagtat tattttaaag 900
atgggaaaat tgaggtttgt aatatggaga aatgatatat ttaaggttat atagttaatt 960
agtggtagag ataggattaa aatatatgtt tttagtttgt taggttagaa ttttttggga 1020
gttgagttag aaaaaaaagt taagaatgtt ttttttttga atatggttaa aaagagaggt 1080
tggtggataa tgtaatggga gagcggatac gtgggagaaa taacggagaa tagattttat 1140
tttagaatag agtatagaag agtttgaatt attagggata agtagagata attgaaattt 1200
ataaatatgg aggagattta ggaagtttta tagtaaggtt ataatgtgaa ttgttaggaa 1260
taaattaaaa ttggagaaaa agggatttgg aaataggtgt taaaagagaa ttttttatta 1320
ttttttttat ataaatgtcg tattattggt ttaggatggt agtgggatag taaagataag 1380
taaaatatgt ttttttagga ttggttgtaa tttttttatt atattttttt aagatggatg 1440
tgattttggt atagtttttt gttggagatt tgaaagtaat atttatattc gtttttgaaa 1500
ttttaagtat ttgggaaata gatatgagag ttaggaaaac gcgaatatat ttgttttgtg 1560
tgtgtgtgtg tgcgcgcgcg tgtgtgcgtg tgtggagttt cgtttttatc gtttaggttg 1620
gagtgtggtg gtttgatttt ggtttattgt aattttcgtt tttcgggttt aagcgatttt 1680
tatgttttag gtttttaaga agttgggatt ataggtaggc gttattatgt ttggttaatt 1740
ttgtattttt agtagagata gggttttatt atgttggtta ggttggtttt aaatttttga 1800
ttttaggtga tttgtttgtt ttagtttttt aaaatttggg gataataggc gtgaggtatt 1860
acgtttggtt aatatttgta ttattaatgg ttaagtttat tgagtaatat gatttggatt 1920
taattttaaa atggtttttt tttatttttt gttttagatt gaatttattt tttggttata 1980
ggtttttgtg agtttttttt gtggaagaga gaggagaatg aatgaggttc gtagatagat 2040
agattataga gagatgtttt ttttttaagt tttattaata atgtgttatg tatttttttg 2100
tttatgaaaa taaaagattt aagggggtta ggatataaaa agatagaaat attttttgtg 2160
ggttggtagg aatatgtgat agagggagag tttcgttttt ttttttttat attttttatt 2220
ttttaatatt atataattta attagggaga gaaggattag agtaagaagg atatttataa 2280
atacgtgtag aattatttgg ttattagttt tggtttagtg gttttatgag tttatgtgga 2340
tggtagatag gagttagaaa taaaaggatg tatttatagt ttgtgtttgt aaatttgtaa 2400
taaatgtttt cgttattttt attatatatt tttgaagttt tttttgagtt tgtttatttt 2460
taatatgtat tatagagtgt ttatattaat aattttatag gcgttttgaa aatattttat 2520
gaatggtgtt tgtagaagta aatagtgaga gttatttgag aggagaagtg gaggaaaaac 2580
ggtgtgggaa agtttttatg atttttttaa tattattttt ttttttaagg tttatttttt 2640
ttttgaatta gaattttatt tttttttagt ttttgtatgt ttgttttggg ttaggatttt 2700

aagagagaaa tgagagtagg aataaaaggt ttttggggtt ggtgtttggt agaaggtaaa 2760
aatatgtaga gaataagtag agattttgta tttgtttatt atttatttta aatatttggg 2820
tttgattaaa gttttttttg attttttttt atgtaagata ttttttttta tggtatttgg 2880
aatttaaata ataattataa atattttgaa ttaggaaata gttttttatt ttttagaatt 2940
aatattaatt agttattttt tagggattta ttttttttta tgaattaaat tttaaagaag 3000
tggatatttg aatagatttg aggtgttaaa attttcgttt attttgtttt ttttttttat 3060
ttgggaaagt taaaagtata aaagagtagg tagaaaatgt tagatttttt ggtattattt 3120
ttttgtaatt attagtttta atttattgta atttttattt tagtttgttt tgtagatata 3180
tagtggtgtt tttgatggag taagagatat tttaagattg tagagaagtg gaaggagggg 3240
tagtaatagt tttttttgtg aagtaatgga atttatagaa gggttcggtg atttagtttt 3300
tatgggttaa gaagtttgaa agtattttag tgttgttgag ttcgagtttg tttgttgggt 3360
agttaggtaa ggaaaaaagg gttagagttt gaagataagt tgagtatttg gttttgtatt 3420
tatattagtt ttgaaaagag gtgatggggg tgttatgtag gagtttagta aagacggtgt 3480
agggttgtta ggtagtagag ttgttttatt ttgtttttta gttggtttta gaaattaagt 3540
aaaatagtta aataattgtg tttgtttgaa ggtagggatg tagataggtt attttttttt 3600
gttgtatttt tggttttaaa attttttttt tttttttttt tagaataaaa tataatatag 3660
ggagaggata gaatttgtta atagattgag gttagtaaaa agttagattg gtttttgaat 3720
tggagggaag gaggttgttt gattttggag gagtagaaaa aaataagtta ggagattaga 3780
gatgggttat agtttaaata gagaggttcg attttgtatt ttttaaatac gtaatgttta 3840
cggtatacga tttaagtttt ttgtttgaat tttaaatttg agcggataga tgagggggcg 3900
tagaggaaaa ataggttttg gattttataa attggtttga aatgaaaagt tagtaaaaat 3960
tattgggtgg tagcgtggtg tgcgggcgta gttttgtagt gtttgttttt gttgttatcg 4020
ttggggttga attcgtttgg ggattttggt tttttttttt gaatggttat aagtgtcgtt 4080
aagtaaaata tttttgcgcg ggtgggtgag ggtttttcgg gttgttggtg cgtttaagtt 4140
tgaggtaggg atttggcgaa tttttgaggg gttttagttc gagtataatt tggttagttt 4200
aaatattgag gaaatatttt tattatttta ttttttaatg tcgagtaaat tttcgtttcg 4260
ttagtttaat tattttattt tagttttttt tttattattg ttttttttcg aagttttttt 4320
cgggaaaagg aggggagaag gatttggacg attggggtag tgcggaagtt tttataggtg 4380
ttagtcggaa cgggcggtcg gggatttggg ggggtggggg tgggggtggg ggtgggaggg 4440
gcggtttgcg gtttcgagtt tacgggttta gtttcgggag ggaggggtcg tcgcgggagg 4500
gggttgtaag gaggttgtcg cgttgtttcg gagggtgttt gcggcggttg aatttttgtg 4560
ttgcgattgt gtcgcgttta gagtagtagt aatagcgaag atgcgaggtt attatttgtt 4620
tgatttttgt cggaaatttg gtacgggtta atttttttcg attattacgt taagaagttg 4680
taaggattag tcgaagattc ggaggggtta gggcgagggc gcgttttttc gcgcgttgtt 4740
tcgttttttt ttcgttcggt cgttcgagtt ttcggttttt ttttcgttcg cgtttatttt 4800
tttcgttcgt tttttttttt tggtttttat tagaagggta atagggcgag ggggttcggc 4860
gaaattcgga tcggagtagt tggatatgta cggtgttcgt cgggcgtagg ggtcgattat 4920
acgtagtcgc gtagtttagt attcgcgtgt tagtttcgtt cgcgatttcg ggttcggggt 4980
tgtggcgtcg atttcgattt aggtagttag tagttcgcgc gggagtcgga tcgtcgtcgg 5040
aggagttcgg acggtatgtt gagttttttt tttggttgaa gttcgagtgc ggagaagttc 5100
gggtaaacgt aggttaagga gattaaagcg gcgaagtcgc gagatagcgg ataagtagcg 5160
gaggagaagg aggaggaggc gaatttagag aggggtagta aaagaagcgg tggtggtggg 5220
cgtcgtggtt atggcggcgg ttatcgttag ttcgtttatt cgttagaaga ggtaagttcg 5280
cgagcgcgag aaatttaacg tttgtaagtg tgttagtagt tttagtaaag gtaagattag 5340
ttgcgataaa aataagttaa atgttttttt tcgggttaaa tttttcggtt ttaagaagag 5400
gcgtagaaga agattaggtc ggaaagggtt tttgtttttt tttttttata ttttacgagt 5460
gaggtggggg cggtggggag taggtatgta gtataggtga gtttgttggt ggtttatttt 5520
gtgtagaaga ggttatttag tttgtgtgcg tgcgtgggaa ggcgttggtt tttttgtttt 5580
cgtaatagta atacgtttgt tgtattgtaa ttgttttggg ataggcgtag agtttggggg 5640
tatattttgt tttgggtggt ttgtttttag tttcggatat gttatatatg tgtatgttcg 5700
gtgggttttg ggtaggcgtg atttagatat ggttgtgata tgtatggcgt ggaaattgta 5760
ggcgtagagt tgggtagata ttattttttc gtttttagga gagtttgggg aatttttttt 5820
ttgtttttat ttttgtttta gatttttttt ttttagtttt tatttttaag ggatgtgggt 5880
ttttgttgtt taggtattgc gtttttttga tgatttttaa gttttttggg atagatttga 5940
tttgtggtgt agtttcgaaa tgtttgttgg ggcgggggga ggggggattt ttagaatttt 6000
gttgtaaaga gttgagagat ttggttagat ttagtgtaat cggttatatg ttattaaaat 6060
gcggtgttga agaggggatt taagtttata tttagattgt ggtgtttttg aatattttta 6120
gtggtaggag tagcgcgata ttttagcgat tggaaattta gttttaggtg ttaggttgcg 6180
ttaaggtaga tttttaggcg agtatgtagt ttatttgtta tttttaatta aatgaatttt 6240
ttatattttt ttttgttttt tattagataa atattataaa aaaaataaga gttatcgatt 6300
tgttattttt ttatttatgt gtgttatatt ttgggatgga aaagtagata taacgttttt 6360
tgtttgtata aagtttttaa tgtattaatt gtaaattagg tttgttttaa aaaggtttaa 6420
gtggttgtaa agtatgttta tgtggttcga ataattatat aaatattatt ttagcgtagg 6480
agtttatatt tagtaaaaga aaattttgga taatatggtt gttattggta aatgagatat 6540
ttgattgatt tatggttatt tataattgaa aattatttta attaagaatt tatatttttg 6600
ataattatat ttttgatatg aaatttgaaa aattagtatg tattgatttt ttttttttta 6660

ttaataaaaa ttttgttttt tttatatata tttttattaa tgataattaa tatgataaga 6720
tttgttaatg gtggttgtat tttttttttt ttttttttgg tataaaaggt ttaatttttt 6780
aaatatagtt tttttaatgg ggttttacgt tatttatgta ttttttattt ttttagaata 6840
aaggagatta tttatttaaa ttgtggttat tttgaaggaa atttttgtta gtgggttttt 6900
ggtttttggt cggttggtag ataattttga attatttttt tatttttttt ttttttttta 6960
tatgaaaagt agtataattt taaaaggtag aatagttatt tgttaattta ttataaatat 7020
ttagtgagat agagttttat tttgtaaatt aaaggatagg ttattagaaa ttatagatat 7080
ttttagaagt tttattaatg tttttagata gaaataaaat gtttttttag tttgaaaatt 7140
tattttgaaa tttagagttt gttttttttt ttttagatat tttaaaagtg tagtttgatt 7200
ttttttatta agatttgtaa atgaaattag aaattgaaat ttttagtttt tttaattata 7260
taggtataat ttattaattg atttaaatta ttgttttttt tgtaga 7306

<210> 308
<211> 7306
<212> DNA
<213> Artificial Sequence

<220>
<223> chemically treated genomic DNA (Homo sapiens)

<400> 308

tttgtaaaga gaataatgat ttggattaat tgataaattg tgtttatgta gttgaaaaga 60
ttaggaattt taatttttaa ttttatttat aaattttaat ggaaaaagtt aaattatatt 120
tttaaggtgt ttggaagaaa gaaaataaat tttgagtttt agaataaatt tttaaattaa 180
agaaatattt tatttttatt tggaaatatt ggtaaagttt ttgaaagtgt ttatagtttt 240
tgataatttg ttttttggtt tatagagtgg aattttattt tattgagtat ttgtaatggg 300
ttggtaaatg gttattttgt tttttgaaat tgtgttattt tttatgtgaa aggaaaagga 360
gaaatagaaa gataatttag agttgtttgt taatcgatta aggattaaaa atttattagt 420
aaagattttt tttagaatga ttataatttg aataaataat tttttttatt ttaagaaggt 480
agaaaatgta taaataacgt ggaattttat taaggaaatt gtatttggaa aattgagttt 540
tttatattaa aaaagggagg agggagatat aattattatt aataaatttt gttatattaa 600
ttattattag tgaaaatgtg tgtgaaaaga gtagggtttt tgttggtagg aaggaaaaaa 660
ttagtgtata ttaatttttt aaattttata ttagaagtat gattattaaa aatatgagtt 720
tttaattaaa atgattttta attatgaata attatagatt aattaagtgt tttatttatt 780
aataatagtt atgttattta aaattttttt ttattgaata tagatttttg cgttgaaata 840
atatttatgt gattattcga gttatataag tatgttttgt aattatttaa gtttttttga 900
aataagttta atttatagtt aatgtattaa aaattttgtg taaatagaaa acgttatgtt 960
tgttttttta ttttagggtg tagtatatat aaatggagaa ataatagatc ggtgattttt 1020
attttttttg taatatttat ttagtaaaag gtagaaaaga gtgtaaaaga tttatttggt 1080
taagaatggt aaataaattg tatgttcgtt tgaaggtttg ttttgacgta atttgatatt 1140
tagagttgaa tttttagtcg ttggaatatc gcgttatttt tattattgaa aatgtttaaa 1200
agtattatag tttgggtatg agtttaaatt ttttttttaa tatcgtattt tagtagtatg 1260
taatcgatta tattaagttt aattaaattt tttagttttt tgtaataagg ttttagaaat 1320
tttttttttt ttcgttttag taaatatttc gaagttgtat tataagttaa gtttgtttta 1380
aaaagtttaa aagttattaa agaaacgtaa tgtttagata gtagaagttt atattttttg 1440
gaggtggaag ttggagggga agggtttgag gtagagataa aagtaagaga gggatttttt 1500
aggttttttt ggggacggga ggatggtgtt tgtttagttt tgcgtttgta atttttacgt 1560
tatatatatt atagttatgt ttgggttacg tttgtttaga gtttatcggg tatgtatata 1620
tgtgatatgt tcgaagttga gggtaagtta tttagggtag agtatgtttt taggttttgc 1680
gtttgtttta gggtagttgt aatgtagtaa gcgtgttatt gttacggagg taagaaagtt 1740
agcgtttttt tacgtacgta tataggttag gtggtttttt ttgtataaga tgggttatta 1800
ataaatttat ttatgttata tatttgtttt ttatcgtttt tattttattc gtaaagtatg 1860
gggaaaagaa agtaaaagtt tttttcgatt tggttttttt ttgcgttttt ttttggagtc 1920
gaagagtttg attcgggaaa agatatttaa tttgtttttg tcgtagttgg ttttgttttt 1980
gttggggttg ttgatatatt tgtaggcgtt ggatttttcg cgttcgcggg tttgtttttt 2040
ttgacggatg agcgagttgg cgatagtcgt cgttatggtt acgacgttta ttattatcgt 2100
tttttttgtt gttttttttt gggttcgttt tttttttttt ttttttcgtt gtttgttcgt 2160
tgtttcgcga tttcgtcgtt ttggtttttt tagtttgcgt ttgttcgggt tttttcgtat 2220
tcgggtttta gttaaggagg gggtttagta tgtcgttcga gtttttttcgg cggcggttcg 2280
gttttcgcgc gggttgttgg ttgtttgggt cggagtcgac gttatagttt cgggttcggg 2340
atcgcgggcg agattggtac gcggatgttg aattgcgcga ttgcgtgtgg tcggtttttg 2400
cgttcggcgg atatcgtgta tgtttagttg tttcggttcg aatttcgtcg gattttttcg 2460
ttttgttgtt tttttgatgg gggttagagg agggaggcgg gcggagggag tgagcgcggg 2520
cgggagagag gtcgggagtt cgggcggtcg gacggaggag ggacgaggta gcgcgcgggg 2580
gagcgcgttt tcgttttggt tttttcgagt tttcgattag tttttgtaat tttttggcgt 2640

gataatcggg aaaagttggt tcgtgttagg ttttcgatag ggattaaata ggtaatggtt 2700
tcgtattttc gttgttgttg ttgttttggg cgcggtatag tcgtagtata ggaatttagt 2760
cgtcgtaggt atttttcgga atagcgcgat agtttttttg tagttttttt tcgcgacggt 2820
tttttttttt cggggttgag ttcgtaggtt cggagtcgta ggtcgttttt tttattttta 2880
tttttatttt tattttttta agttttcggt cgttcgtttc ggttaatatt tgtaggggtt 2940
ttcgtattgt tttagtcgtt taaatttttt tttttttttt ttttcgaaag gagtttcggg 3000
aggaaatagt gatggaggag gaattgagat gaggtagttg ggttgacgga gcgggggttt 3060
gttcggtatt gggggatgga gtggtggggg tgttttttta gtgtttgagt tgattagatt 3120
gtgttcgggt tggagttttt tagaagttcg ttagattttt gttttaggtt tggacgtatt 3180
aataattcgg agaattttta tttattcgcg tagaggtatt ttgtttggcg atatttgtag 3240
ttatttaggg gaggaggtta gagtttttag acgggtttag ttttagcggt agtagtagaa 3300
gtagatattg tagagttgcg ttcgtatatt acgttgttat ttagtggttt ttgttgattt 3360
tttattttaa gttaatttgt agggtttaaa atttgttttt tttttgcgtt tttttatttg 3420
ttcgtttaga tttgggattt aagtagagag tttgggtcgt gtatcgtaag tattacgtat 3480
ttgggaaatg tagagtcgaa tttttttatt taagttgtgg tttattttta attttttaat 3540
ttattttttt ttgttttttt aaagttaggt agtttttttt tttttaattt aggaattagt 3600
ttggtttttt gttggtttta gtttattaat aagtttgttt ttttttttgt gttatatttt 3660
attttggagg aaaagaaaga agaggttttg aggttagaaa tgtagtaaag agaaatggtt 3720
tgtttatatt tttattttta aatagatata attatttgat tattttattt gatttttgag 3780
gttaattagg aagtaaaata gagtagtttt attatttggt agttttatat cgtttttatt 3840
ggatttttat atggtatttt tattattttt ttttagaatt aatgtagatg taaaattaga 3900
tgtttagttt atttttagat tttagttttt ttttttttgt ttgattattt aataaataag 3960
ttcggattta gtaatattga aatgttttta ggttttttag tttataagag ttgaattatc 4020
ggattttttt gtagatttta ttgttttata ggggaagttg ttgttatttt tttttttatt 4080
tttttgtaat tttgaaatgt tttttgtttt attagaagta ttattgtgtg tttgtaaggt 4140
agattggggt aaaggttgta gtgggttgag attgatagtt ataggagaat gatgttaaag 4200
ggtttgatat tttttattta tttttttgtg tttttggttt ttttaaatga aaagggagga 4260
taaggtagac gagagtttta atattttaag tttatttaag tatttatttt tttaaaattt 4320
gatttatagg gaaaaataaa tttttgagga atgattgatt gatattgatt ttgaagagta 4380
ggaggttatt ttttagttta aaatgtttgt gattgttgtt taaattttag atattatgaa 4440
gggaaatatt ttatataaag aggggttagg agaagttttg attaagttta aatatttggg 4500
gtgggtgatg aataggtata aggtttttgt ttattttttg tatgttttta ttttttgtta 4560
agtattaatt ttaagggttt tttgttttta tttttatttt ttttttggga ttttgattta 4620
gagtaggtat gtaaaagtta aagaaggatg aagttttgat ttaggaaggg gatagatttt 4680
agaagaagag atggtattga gaaaattata agggtttttt tatatcgttt tttttttatt 4740
ttttttttta gatagttttt attgtttgtt tttgtaaata ttatttataa ggtattttta 4800
ggacgtttgt agaattattg gtgtggatat tttgtggtat atattagagg taaatagatt 4860
tagagaagat tttaggaata tataataaga atggcggggg tatttattat aaatttgtaa 4920
atataaattg taaatatatt tttttgtttt taatttttgt ttattattta tataagttta 4980
tggaattatt gggttaaggt tgataattag gtgattttat acgtgtttgt agatgttttt 5040
tttgttttaa tttttttttt tttgattaaa ttgtataata ttgaaaaatg aagagtatgg 5100
gggaggggga acgagatttt ttttttgtta tatgtttttg ttaatttata aaagatattt 5160
ttgttttttt atattttgat ttttttgaat tttttgtttt tatgaatagg aaaatgtatg 5220
gtatattatt aatgagattt gagaaggggg tatttttttg tgatttgttt gtttacggat 5280
tttatttatt tttttttttt ttttataaag agaatttata aaggtttgtg attagagggt 5340
aaatttaatt tggggtagaa agtgaaagaa aattattttg aaattgggtt taaattatat 5400
tgtttaatga atttaattat taataatgta gatattggtt aggcgtagtg ttttacgttt 5460
gttattttag aattttggga ggttgaggta agtagattat ttgaggttag gagtttgaga 5520
ttagtttggt taatatggtg aaattttgtt tttattaaaa atataaaatt agttaggtat 5580
ggtggcgttt gtttgtagtt ttagtttttt gggagtttga ggtatgagaa tcgtttgaat 5640
tcgggaggcg gaggttgtag tgagttaaga ttaggttatt atattttagt ttgggcgata 5700
agagcgaaat tttatatacg tatatacgcg cgcgtatata tatatatata aagtagatat 5760
gttcgcgttt ttttaatttt tatgtttatt ttttaggtgt ttaaaatttt agagacgaat 5820
gtgaatatta tttttaaatt tttaatagga aattatgtta aagttatatt tattttgaga 5880
aggtataatg aaagaattgt agttaatttt agagaagtat attttgtttg tttttgttgt 5940
tttattgtta ttttgggtta atagtacgat atttgtgtag gggaagtaat aaagaatttt 6000
tttttgatat ttatttttaa attttttttt ttttagtttt gatttgtttt tagtagttta 6060
tattgtggtt ttgttatgga attttttgga ttttttttat atttgtaaat tttagttatt 6120
tttgtttatt tttggtggtt taaatttttt tatgttttat tttaaggtag ggtttatttt 6180
tcgttgtttt ttttacgtgt tcgttttttt attgtattgt ttattaattt ttttttttga 6240
ttatgtttag aaagaaagta tttttagttt ttttttttaa tttaattttt aaagagtttt 6300
agtttggtag gttggaaata tatgttttaa ttttattttt gttattaatt ggttgtgtga 6360
ttttgagtat gttatttttt tatattatag gttttaattt ttttattttt aaagtaatat 6420
ttagataaga ttggtagttt ttattttgta ttttttagta atttaaaagt aatgaaaagt 6480
aataaaagtt ttttcgttaa tgttttaata gtagatttta tttattttaa ttgtttttat 6540
ttaattgttt attttttttt ttttttttat ttgttttata tagtggagtt tattgtatga 6600

ttttttacgt aaaaaataaa aatgaaaaga ttttggtatt aaaaataaaa cgtttggaaa 6660
tttttagatg atatgttttt taaagttttt ttggttttgg gagtttatga ttattttttt 6720
tatatgtttt tgtttttatt tttttatttt ttttttttta tattttttat ttttttgttt 6780
ttttttattt ttatgagaat ataaatttta aattggattt taaggaaaaa atgtaaaagt 6840
ttttttgatt ttatttttcg tgggtttatt tgagaattag gttttagtat tttttttaga 6900
gagagttatg tgagttattt gggtatagaa tgttgatcgt ttttgttaa tttttttttgg 6960
taggttttag aggagttgta aggaagttga ggggatttgt ttttaagagg tatttttagg 7020
agagagatag aaatttaaat agatgtgatt gtgatgagta aaaatgagag aatttagata 7080
gtttaatcgg gagtagtttt agtttatttt gagtagaaag gaattggttg tttttttgaa 7140
attttgaaaa tgagaaattt taaatatttt ttttatgtga agataagttt atttagaatt 7200
atttgttagt ttgttttttt tttatattaa tagtttatta aaatatttga gtgggaagtt 7260
atttggttaa ggattgatga tgagtttaaa gaaaggaaat gttggt 7306

<210> 309
<211> 2821
<212> DNA
<213> Artificial Sequence

<220>
<223> chemically treated genomic DNA (Homo sapiens)

<400> 309

tggtgattta gaaatacgga acgtgtttat ttagtatgag tgtttttttt taattttttc 60
gttgtatgtt tgttagggtg ttatatattg tagttttttt ttttggattt ttatattttt 120
aggtatttag tagggaatcg ttgtcgggat atagatagaa aattaagcgg gaggagacgt 180
cggttgttgt atttgatttt tagatatagt ttagatggtt ttgttttttg atagagattt 240
tttgtttagg gatattagaa gatgatagtt taaattggag tgttttgttt ggttgttttt 300
tattgttttt ggaatagtgt gatatttttt tttgtttttt atatttttgt tttgattttt 360
ttaatatttt tttgggcgtt tattttattt agttgttgtt ttgtagtagt tattgcggtt 420
gttatttttt ttttttaggg ttggggagta tatttgggag tggggttttt ttatttcgtt 480
taattatatt tttttttttt ttattttttt ttttttgttt cgtgtagttt tttaaaatga 540
taaatttatt ttttttgtag tagaagggag ggttttgtaa gtttgggtgg attaggtgag 600
gagtggaggt tttttttttt aattcggggt tgggggtggc gacggggggg cgttggtgtt 660
ggagggatta gtagggaggg cggaaattat ttcgtttttt aatcgttgtt gagagtttta 720
gtgtatcggt aattacgtta agatcgagaa gttttaatta agtcgcggaa ggttcgggaa 780
ttagcgagtt gtatttaaga aaaagcgttt cgatgttttt ttaattttat ttgttttggt 840
tcggtttttt agtattttat ttttgtaatt tattttagtt ttttttttta ttatttttaa 900
attattatta tttatacgtt ttgttttttt ttgaagaggt atattaggag ggggattata 960
taggtggagg gggtgtagtg gggtgggaac ggagcgtttg attagagatt ttaggggaga 1020
agattggttt ttttagatgt tgatttttaa agtatttgat aattatcggt agaagtcgag 1080
agcgggcggc gtcggcggcg gtatcggttc gcgggtagtt ggagttcggc gttgagtttt 1140
gtttagcgcg attcggatcg acggacgcgt agagattttg tcgggattcg ttttttagtc 1200
ggcgttcggg gagtcgtcgg tcggttttat ttttcgttcg ggagtcgggt agggcgagaa 1260
ggtagcgcga gtggcggtgg cgattttcgt cggggttttt gcgacgagcg gtgattagta 1320
gcgttcgtag tcgcggtttc gttttttatt cggacgtggt taagcggcgg cggttacgat 1380
tttcggtttt tcgggatcgt tcggttgcgt tttgggcgta tttgcgcgtt tggtttcgcg 1440
tttcgcggag agggcgatgg gtttcggtta ggattgagcg tttttttttt tttttttagt 1500
ttcgtttggt attttttttt ttgtttcgag gcgagttacg gtttgcgtcg tcgttttttt 1560
tttcggtgtt ttcggttcgt cgttttttcg cgtttttttt tttcggattc gttttcgggg 1620
ttattatggt cgcggttatc gttagcggtt tgattcgtta gaagcggtag gcgcgggagt 1680
agtattggga tcggtcgttt gttagtagga ggcggagtag ttttagtaag aatcgcgggt 1740
tttgtaacgg taatttggtg gatatttttt ttaaagtgcg tattttcggt tttaagaagc 1800
gtaggttgcg gcgttaaggt ttgtgggggt tatggtcggg agatgcgttt cgggtcgttt 1860
ttttttcgtt taaacggagt ttttttaggt tttttttttt tttcggtttt ttcggtttat 1920
ttcggtttcg gttttttttt tgagtatttt gggtgtcgtc gcggggattt tggagttcgt 1980
tgtcgggtaa tttgtagtcg tcgagaagtg tagtcgttag agatggtaag ttggttgttt 2040
ttcgtcggcg gtttttgttt ttcgcgtttt tgtaggttgg gttttgggaa atttgttttt 2100
tttgagtttc ggtttttcgg gaggataggg attggtattg gttttggaac gcggggtatt 2160
tttttttgtt atattcgggg tcgagttatc gttcgtagtt tgatttgagt tggtgggggt 2220
ttttatgggg tgggtttggt tttatttggt ttttggggtt ttttagaaat ttttcgggtt 2280
tttagatttt ggataaatgt ggatattttg gcggtgtttt tagcgggagt tgttgtagaa 2340
ggagtggagg gtgtgtgaat tatggtttag ttcgttgagg tttagtagaa ggttgtttag 2400
cgcgtagttt tgggttttgt agttttttag ttaaattttg ggatggggat agaggtttag 2460
ggagtttaaa gaaagaattc gaattgcggg gagcgggggg tagaagtaaa aatttttaat 2520
atgtgttagt tttaattgaa aaattggcga attgtttgag atataagtcg ttattatata 2580

aaggtgatta gaatagttat tttattatag gtattttttt gagaaattag tatttaggat 2640
gatttttttt tattgttttt gaaatgttag ggtttgagaa ggtattttat ttaggagtta 2700
ggcgtttttg tttttttttt tgtttgtttt tgtatttagt tgttaaggtt ggttgttttg 2760
cgagtatgtg gtgtgttttg tgggggaaat gggtattgtt ggttttttat aatcgagtgg 2820
g 2821

<210> 310
<211> 2821
<212> DNA
<213> Artificial Sequence

<220>
<223> chemically treated genomic DNA (Homo sapiens)

<400> 310

tttattcggt tatgaaaagt tagtaatgtt tatttttttt atagaatata ttatatgttc 60
gtaaaataat tagttttagt aattggatgt agagataggt agagagagag ataaaagcgt 120
ttggtttttg aataggatgt ttttttagat tttggtattt taaaggtagt gaaagaaggt 180
tattttaggt attagttttt taaagaaata tttgtagtaa ggtaattatt ttagttattt 240
ttatatagta acggtttgta ttttaggtaa ttcgttagtt ttttaattaa aattgatata 300
tattagggat ttttattttt gtttttcgtt tttcgtaatt cggatttttt ttttagattt 360
tttgagtttt tgtttttatt ttaggatttg attgaagggt tgtagagttt agggttgcgc 420
gttaagtagt tttttgttga attttagcgg attgggttat agtttatata ttttttattt 480
tttttatagt agttttcgtt gggggtatcg ttaggatatt tatatttgtt taaggtttgg 540
gaattcggga ggtttttagg agattttaga gattaagtga ggttaggttt attttatgga 600
aatttttatt agtttagatt agattgcgag cggtggttcg atttcgggtg tggtaggaag 660
gggtgtttcg cgttttagag ttagtgttag tttttatttt ttcgggaagt cgagatttag 720
ggggaatagg ttttttagag tttagtttgt agggacgcgg agggtaggag tcgtcggcga 780
ggagtagtta atttgttatt tttggcggtt gtatttttcg gcggttatag gttgttcgat 840
agcggatttt agggttttcg cgacggtatt tagagtgttt agaagggagg tcggggtcgg 900
ggtgggtcgg gaaaatcgag ggagggaagg agtttggaga agtttcgttt aggcggggag 960
ggggcgattc ggggcgtatt tttcgattat gatttttata gattttggcg tcgtaatttg 1020
cgttttttga ggtcgaagat gcgtattttg gagaagatat ttattaggtt gtcgttgtag 1080
agttcgcggt ttttgttggg gttgtttcgt tttttgttgg tagacggtcg gttttagtgt 1140
tgttttcgcg tttgtcgttt ttggcggatt aagtcgttag cgatggtcgc ggttatggtg 1200
gtttcgggaa cgggttcggg gagggagggc gcgggaggac ggcgagtcgg gggtatcgga 1260
ggggaaggcg gcggcgtaga tcgtggttcg tttcggggta gaggagaggg tgttaggcgg 1320
gattggggag aggggaaggg gcgtttagtt ttgatcggga tttatcgttt ttttcgcggg 1380
gcgcggggtt aggcgcgtag atgcgtttag ggcgtagtcg gacgatttcg ggaagtcgga 1440
ggtcgtggtc gtcgtcgttt ggttacgttc gagtggagag cgggatcgcg gttgcgggcg 1500
ttgttggtta tcgttcgtcg taggaatttc ggcgggggtc gttatcgtta ttcgcgttgt 1560
tttttcgttt tgttcggttt tcgggcggga ggtagagtcg gtcggcggtt tttcgggcgt 1620
cggttggagg gcgggtttcg gtagggtttt tgcgcgttcg tcggttcggg tcgcgttggg 1680
taggatttag cgtcgggttt tagttgttcg cgggtcggtg tcgtcgtcgg cgtcgttcgt 1740
tttcggtttt tgtcggtgat tgttaagtgt tttggaaatt agtatttgga gagattaatt 1800
tttttttttg agatttttaa ttaaacgttt cgtttttatt ttattatatt ttttttattt 1860
atgtggtttt ttttttgatg tattttttta gaaagaaata aaacgtatag gtaataatga 1920
tttaaaaata ataagaaaga aagttaagat ggattgtagg gatggggtgt taagaggtcg 1980
agttaaagta ggtggaattg aaggaatatc ggggcgtttt tttttagatg taattcgttg 2040
gttttcgagt ttttcgcggt ttggttgggg tttttcggtt ttaacgtggt tatcggtgta 2100
ttgaaatttt tagtagcggt tggagggcga ggtggttttc gttttttttg ttaatttttt 2160
tagtattaac gttttttcgt cgttattttt aatttcgggt tggagggagg agtttttatt 2220
ttttatttag tttatttaaa tttgtaggat tttttttttt gttgtaggaa gggtaggttt 2280
gttattttga aaggttatac ggagtaaaaa ggagggaatg gaaagagaga aggtgtgatt 2340
aagcgagata gaaagatttt atttttaaat atgtttttta gttttgggag gaggagatga 2400
tagtcgtagt agttgttata gaatagtagt tgggtaggat aaacgtttag gagaatgttg 2460
aagaagttag ggtagaaata taaggggtag aggagggtgt tatattattt tagggatagt 2520
gaaaagtagt tagataggat attttaattt gagttgttat tttttggtgt ttttgggtaa 2580
agaatttttg ttagggagta aagttatttg aattgtgttt gaaaattaaa tataatagtc 2640
ggcgtttttt ttcgtttgat tttttgtttg tgtttcgata gcggtttttt attggatatt 2700
tgagaatgtg aaggtttaag aagaagggtt gtagtgtatg gtattttaat aaatatatag 2760
cgagggagtt gggagagaat atttatgttg gatgaatacg tttcgtgttt ttagattatt 2820
a 2821

<210> 311

<211> 6661
<212> DNA
<213> Artificial Sequence

<220>
<223> chemically treated genomic DNA (Homo sapiens)

<400> 311

ttaaatttag ttttttttgt tttttataat agttgttttt tttttttttt ttttataggt 60
aatagtattt tttagttatt ttagttttag aggttagatt aagtggatag aataaagtta 120
agtagggtag agaaagggtt tttagtagtg gaaagaatat gaagatgttt ttttatatta 180
ttcgaatata tatgttttga ttttcgattt gtttatttat taatttattt atatatttat 240
ttatttattt atttattaat ttatttattt atttatttat taatttattt atttatttgt 300
ttatttattt agtaatttat ttttattcgt ttatttattt atttatttat gtatttattt 360
aatttattta ttaatttttt ttgtttttaa tttatttatt tttaatttat ttatttatta 420
atttttttat atatttattt attaattggt gtttttatta attcgtttat ttttgtttat 480
aggtgttatc gaggtttttt aatttgtttt tttagttagt agtggaggat aggtttttag 540
gtttttttaag ttttaggttt tttttttttt gttttatttt tttgtttttt atattaagat 600
tttatttttt tagattgatt ggtagttttt gaaaatgttt gtatagaagg taatgagggt 660
tggagggagt gagaagaata gagtgtatta tgagttttag atagttagga ttagttgtgt 720
aatttttagg gtttcgttta aaataaaaat gtataatttt ttgtttaaaa gttatgatga 780
attttaggat aattatagta gagtattgaa ttaggaatta ggatttttta agggttgggt 840
tgtttgtgat tgtattggcg atatttttat aaagtttatt ttgaaggtag gagacgggtg 900
gagagaaata gggggatggt aagggggata cgaaataggg agagggagga gggggaagag 960
gatggacgtt tattaggttt tatttggtgt ttgatttatg ttattttatt ttttttttaa 1020
attatttttt gaagttgatt gtatatttta tagaaaagga aattgaggtt cggagaggag 1080
aattatttat ttaaggtttt agttagtaga cggtaggtgt ttgaatgtaa atttaggttt 1140
ttgtttgttt cgggaggggg tgggggtgag ggaaatagga gaatgtgatg ggaaaattcg 1200
agatggagtt agtttgggtt agaaatattg ggagttgtgg gagacggaga ggggtagggt 1260
gggattatag ggagtaggag cggggaattg gaggtgaatt tggttttttt aaatttttag 1320
tttattttgt ttttagggga atcgggaaat tgcgggggaa ttggaaggga gtttttagaa 1380
taaggattta gaagattggt atttggggtt tgggatttag gtttttaaat cgtgggttat 1440
ggggtagttt tatttttgta aaagtattga gggtagaagt taatgatttg ggaagttatt 1500
gaattagggg atttcggagg taggttgtta gtgtttgata gtattagtta gaatgtttga 1560
tttggggtga taatggtttg gaggggtggg tgagttaagg gttaaatgag tgttcgtgag 1620
ttatgatgtt tgttttgtat aattgataat tgaatatcgg tgagttaggg ttttagtagt 1680
tgtaattagt atttcgggtg ttagttagaa attaataaat agttaaattt ttgtagtttc 1740
gtttagttta tttatcggcg ggggatcgat taattattaa tttttatttt ttttcggtag 1800
agtttttatt tttttataga ggttaggtta agatttttag tagatttttt tagaggacgg 1860
tttgaaagga aggtagagag ggtattggga ggaggtagtg ggagggcgga gggcgggggt 1920
tttcggggtg ggcgtttagg gtagggtagg tggtcgcggc gtggaggtag ggagaatgcg 1980
attttttaaa attttcgtcg atatggatat ggtcgattat agtgttgtat tggatttagt 2040
ttatattatt ttggaatttg agaatgtgta ggtgttgacg atgggtaatg gtaggtgggg 2100
gtagatgtgt ttaggtgtgt tagtgggggt aggtgtgttt gggtttagga gtagattttt 2160
ggtatttaat tttggggtgg gaggagaatg atataaaatg gtaggttggt tttataggtt 2220
agtataggtg ttgttaagtg aagtttatgt gtttaggtat agtgattata ggtattttgg 2280
gtgaagggag gtttgtaagg gttaattttt agtaaaagtc gatttcggtt atttttttta 2340
ggttttttta gtttttattg ttttatagtg gttttgtttg gcgtttggta tagtgacgtg 2400
atggtgagtt tttttttggt gtttagtttt agcgatttag tttagtacgg ttttttcgtg 2460
aattttttgg gtttaggttt agagagacgg taagggatgt tgtatttttg gagatggtgg 2520
ttggagatat aatcgtattt ttcggtgttt ttgggatttt tttagggaaa tgaaattggt 2580
atttagggaa aatggagttt ttagggaagt tttgttaatt acgaagttaa tttagtattg 2640
tgtgtgttgt gtgtgcgttc gtgtgtgata gtgagttttt atgtaggttg tatgggtggg 2700
gtgatgtttt taggaattta tttgtatatg tgtgtttatt tgtttttgtg tgtgagtttt 2760
gggtttattt ttttttgtat ttattgagtg ggtttgtgtt tgtgttttag gagttgttcg 2820
tgttgatttt gtttatgtat gtaagtgtgt atgtgtgtgt atttgtgttt gtggatgttt 2880
gtatatgtgt gttgtgtgtg cgggttatag agtatatgcg tttgtgtatg cggatttgtt 2940
ggagtgtttt gttttttttg tatttttatt ttgtatgggc gttttgtcgt gtgtttatat 3000
ttgtatttgt tgtgtatata tgtagttttt tgtgttgcgg gcgggggtta gcggtttttg 3060
gtgtgtacga ttgtatagat ttaaatgtag gattttgttg ttgttattta ttaagtgaga 3120
tttatattag taatatgttc gtttgttttt gagtagattt tgttgtcgtt gcgtttcgtt 3180
agattgaggt atttttttcg atattattgg agtatatttg gaggggtgga tagtttttta 3240
tagggaggta ggggaaaaga ggaggttcgg aaattttttt tggagggaag agttttatcg 3300
gttttaggtt agttttagag gagagggggt aggtagttgg ttgaggttag tttgttattt 3360
tgtttttttt tgtgttttgg agttatttag ttagtatgag gttgtagttt tagttgaggt 3420

ttggaatttt gtggttagtt tagttagggt gaggaggtag ttgttgggta ttgtttgttg 3480
ttagtttagt aggtgtttat ttgtttttgt cgtttagtta cgtgtgattt tgggtatgtt 3540
atttttttta ttttggtttt tgtatttttt ataaaatagg ttttattttt ttaggtttgt 3600
tggttggacg gtttttaggt ttgtttgagg attacgttag gagcgtaagg taaaaatata 3660
ttagagatag taaataagtt gttgttgtat taggtggagt gttgtgtggt tttacgagga 3720
ttattgtttt gttttgggtt gtttttcgtt tttattattt tttttaagtt taggaattgt 3780
gtgtttttga ggtagaggtt aagtttttga attgggagtt ttaaagataa gattattttt 3840
agttttggaa ttagaaagat ttgggtttaa attcggtata gattttttta atttgtgtga 3900
atgtggtaag gtaatttttt ttttgagttt tagttttttt atttataaaa tgggaataaa 3960
taatagtgtt tgttttttag ggttggttag aggataaata agatgaggtg tttgggtgtt 4020
tagttttggt aaagggtttg gttttattat gggttatttt aagggttttg agtatttgta 4080
agtagggttt ttatattttt tattggagtt tgaagtggtt tgaattgggg ttatgtgaag 4140
ttgttttttg aagtgttttg agtatatttt gggttggtgt ttgaaaggtt ttaaggatat 4200
ttttttttcg tttttttttt attttaatgg agtagtagtt ttgggtggtt ttttgtttat 4260
tagaggttta gtagatattt taatatgtgt ggtattggtt ggagtagttt agtaagtttg 4320
gattataaat ttatggtttt gttttacggg aagtagtgcg atgatattag gaagttattt 4380
ttttttttta agttttagtg tttttatttg tggaatagag gtgttcggtt gattttagag 4440
gggagatgat agagttgata gtttttagtt tattttgttt tagaggtggg agttattttt 4500
ttagtttttt gttgaatggt taggagttgt gggggttagt ttttttcggg ggtcggggga 4560
ggagaggtcg agggagaaag ggtatttacg tggtttaata attattttta tttttaggaa 4620
taatttatta agtggagggg gttgtatttt tggggatatt tgggagtttg cgatggtaga 4680
aatgagatgc ggtagtgttt gtttgtgtaa ggagtttgga agttttttgt ttcgagagtt 4740
tttttatgtg gaaaagtttt tattttatga atattgatat agtgtttatt atgtgttaag 4800
tattttatat atatatatga gaatttatcg aatttttata acgaatttag gggaatatta 4860
cgttttttat tttataagtg aggaaataaa agtatagaga agtaaacgaa tatagttaat 4920
gttatacggt taaaaagtgg gtaagttgga attgaatttt gggtagaagg attttagagg 4980
ttgtgttgtt atttatagtg tatggttttt tttgggtttt aattggatgt ttttgaggaa 5040
tttaaggttg gatttaggat ttttatgaat atacgtgtgt gtgtgtgtgt ttgtgtatgt 5100
gtgtatgttt gtgtatgtga gtatgtgtgt gtggattgtg tgtatgtgta tgtatatgtg 5160
tggattgtat gtgtgtatat atttgtatat gtgtgtgttg atgtgtttgt atgtatgtat 5220
gtgtgtggat tatgtgtaag tgtgtgtaat gtatgtggat tgtgtgtatg tgtggattgt 5280
gtgtatgtgt gtgtatgtgt gtggagcgtg tgtgtgttta tgtgtggatt ttgtgtttat 5340
gtgtgtgtat tgtatgtgta tatatgtgtg tatatatgtg tgtgtgtgcg tgtgtttgtg 5400
tggtgtgtgg attgtgtggt gtgtgtgaat tgtggtgtgt gtgtgcgtga ttgtgtggtg 5460
tgtgtgcgcg tgtgtgtgat tgtttggtgt gtgtgaattg tggtgtgtgt ggtgtgtgtg 5520
tgtttgtgtg tggtgtgtgt gcgtgtgtgg gaattgtgtg gtgtgtgtgg attgtggtgt 5580
gtaaggtgtg tgtgtgtttg tgtgtggtgt gtgcgtgtgt gtggattgtg tggtgcgtgt 5640
gtgtgtttgt gtgtggattg tgtggtgcgt gtgtgtggat tgtgtggtgc gtgtgtgtgt 5700
ttgtgcgtag cgtgtgtgcg tgtgtgtgga ttgtgtggta tatacgtttg tattttttcg 5760
ggggttgatt tattgtattt gttatttttt ttaaaggggt ttatttttgt tgaaagattt 5820
gttgataaat aaagggggtt tagttgttgt tattttttta aatttggaaa taaatttttt 5880
aaatttttta aattttttaa atttggaaat agattagtta aattgttgtt ttttatggtt 5940
agagaaaatg aggtttagaa gaaattgaag tagggaaagc gaagttgttt aagtttatag 6000
gcgagttagt ggtagtatta gtagtagtgt ttaggatttt tggtatttag tttaagattt 6060
tttttttatt tgtttttacg gtttttttta tatttgtgta gatgtagtag tttttgtttg 6120
tgggatggga gggatgggag atgtgtttag atttttttag gaggttatat gagtgtgatt 6180
gttttcggtt taagtttttt tcgtttttta gagaatttgc ggggtttttg ggtatataag 6240
ttgagattta tttagttttg gttttttggt aagaattgag ggataggatt tggttttggg 6300
gaaaatgtag gggaatgttt ttttttttta tagttttttt gcgagttagg aggtcggttt 6360
ttattttaga aggtggttag gtttttatgt ttttttagag aaagttgggg ttcgtggttt 6420
ttattatagg aagacgtaga tttttagaaa taagtttgtg aagttataat tagttttagt 6480
ttatagatgt gaaattgaag ttttaaaaag ttaggaggtt attgagtggg gaggtgatgg 6540
agtgggaata gtttttagat ttggttgagg tcgaagtttt ggagagattt tcgtaaggtt 6600
tttttagatg tttgatattt tgtttttttt gaagttttat tttttttttt tttggcgtag 6660
a 6661

<210> 312
<211> 6661
<212> DNA
<213> Artificial Sequence

<220>
<223> chemically treated genomic DNA (Homo sapiens)

<400> 312

tttgcgttag gagagaaggg agtgaggttt taggaagaat agaatgttag gtatttaagg 60
gagttttgcg gggatttttt tagggtttcg gttttagtta gatttggggg ttgtttttat 120
tttattattt ttttatttag tgattttttg attttttgga gttttagttt tatatttgta 180
aattggggtt ggttgtgatt ttatagattt gtttttgagg gtttgcgttt ttttgtggtg 240
gaggttacga gttttagttt tttttaggaa ggtatgaaaa tttggttatt ttttggggtg 300
ggagtcggtt ttttaattcg taagggggtt gtggaaggga gaaatatttt tttgtatttt 360
ttttagaatt aggttttgtt ttttagtttt tgttaaggga ttagggttgg gtggatttta 420
gtttgtgtgt ttaggggttt cgtaaatttt ttgaggaacg agaaagattt gggtcgagaa 480
tagttatatt tatgtggttt tttaggaggg tttggatata ttttttattt tttttatttt 540
ataggtaaaa gttattgtat ttatataaat gtgagggggg tcgtagaggt aggtggaaag 600
aggattttgg gttgggtgtt aggagttttg ggtattattg ttgatgttgt tattaattcg 660
tttgtaaatt tgggtaattt cgtttttttt gttttaattt tttttgggtt ttattttttt 720
taattataaa aggtagtagt ttgattggtt tgtttttaaa tttaaaaaat ttaaaaaatt 780
taaaaaattt gtttttaaat ttaaaaaaat agtagtagtt gaattttttt tatttgttag 840
taaatttttt agtaaagata gatttttttg aagaagatga taaatgtaat gggttagttt 900
tcgggaaaat gtaagcgtgt gtgttatata gtttatatat acgtatatac gttacgtata 960
agtatatata cgtattatat agtttatata tacgtattat atagtttata tataagtata 1020
tatacgtatt atatagttta tatatacgta tatattatat ataagtatat atatatttta 1080
tatattatag tttatatata ttatatagtt tttatatacg tatatatatt atatataagt 1140
atatatatat tatatatatt atagtttata tatattaagt agttatatat acgcgtatat 1200
atattatata gttacgtata tatatattat agtttatata tattatatag tttatatatt 1260
atatagatat acgtatatat atatatatat atatatatgt gtatatatat agtatatata 1320
tatgaatata gagtttatat ataagtatat atacgtttta tatatatgta tatatatata 1380
tatagtttat atatgtatat agtttatata tattgtatat atttgtatat agtttatata 1440
tatgtatata tataaatata ttaatatata tatatataaa tgtgtgtata tatatagttt 1500
atatatatat atatatatat atatagttta tatatatatg tttatatgta taaatatata 1560
tatatatata gatatatata tatatacgtg tatttataga aattttgaat ttaattttgg 1620
attttttagg ggtatttagt taagatttag agagggttat gtattgtagg taatagtata 1680
gtttttgaag tttttttgtt tagggtttaa ttttagtttg tttatttttt agtcgtgtga 1740
tattgattgt gttcgtttat ttttttatgt ttttgttttt ttatttgtaa aatggggaac 1800
gtgatatttt tttaggttcg ttatggggat tcggtgaatt tttatatgtg tatgtgaagt 1860
gtttggtata tagtaagtat tatattagtg tttatgaaat aaaaattttt ttatatagga 1920
aaattttcgg agtagaggat ttttaggttt tttgtataag tagatattgt cgtattttat 1980
ttttgttatc gtagattttt aggtgttttt aggagtgtag ttttttttat ttggtgaatt 2040
atttttggag atggggataa ttgttaagtt acgtgggtgt tttttttttt tcggtttttt 2100
ttttttcgat tttcggggag agttgatttt tatagttttt ggttatttag taggaggttg 2160
gggaagtagt ttttattttt gaggtaggat gggttggggg ttgttagttt tattattttt 2220
tttttgaggt tagtcgagta tttttatttt atagatgagg atattgaggt ttagagagga 2280
aaagtggttt tttggtgtta tcgtattgtt tttcgtgaag tagagttatg ggtttatggt 2340
ttaggtttgt tgggttattt tagttagtgt tatatatgtt gaggtgtttg ttgggttttt 2400
aatgaataag gagttattta gggttgttgt tttattgggg tggggagagg acgggggaga 2460
aatatttttg gagtttttta gatattagtt taggatgtat ttaaaatatt ttagaggata 2520
attttatata attttaattt agattatttt aaattttagt gaggaatgtg gggattttgt 2580
ttatagatgt ttagagtttt tgagatgatt tatggtgggg ttaggttttt tattagggtt 2640
aagtatttag gtattttatt ttatttattt tttggttagt tttgggagat agatattatt 2700
atttattttt attttatagg tgaggaaatt gaggtttaga gaggaaattg ttttgttata 2760
tttatatagg ttggaaggat ttatgtcgga tttgagttta gatttttttg gttttagagt 2820
tggaggtggt tttgtttttg ggatttttag tttaggggtt tggtttttgt tttaggagta 2880
tatagttttt gggtttggaa aaagtgatgg gggcggggag tagtttaggg tagggtagtg 2940
gttttcgtgg ggttatataa tattttattt agtgtagtag tagtttgttt gttatttttg 3000
gtgtgttttt gttttgcgtt tttggcgtgg tttttagata ggtttaaaag tcgtttagtt 3060
agtaggtttg ggggaatgaa gtttgttttg tagaagatat agaagttagg ataggggagg 3120
tgatatgttt aaggttatac gtgattggac ggtaggggta ggtgagtatt tgttgagttg 3180
ataataagta gtgtttagta gttgtttttt tattttagtt gagttgatta taagatttta 3240
gattttagtt ggagttgtag ttttatattg gttgagtggt tttaagatat agaaggaagt 3300
agggtggtag gttgatttta gttagttgtt tgtttttttt tttttgaggt tggtttggga 3360
tcgatggggt tttttttttt aggaggggtt ttcgggtttt tttttttttt ttattttttt 3420
gtggagaatt gtttattttt ttagatatgt tttagtgatg tcggagggga tgttttaatt 3480
tggcgagacg tagcggtaat aaaatttgtt tagagataaa cggatatgtt gttgatatga 3540
attttatttg gtgagtggta ataatagagt tttgtatttg ggtttgtgta gtcgtgtata 3600
ttagagatcg ttgattttcg ttcgtagtat agggaattgt atatatatat agtaggtata 3660
aatatgggta tacgataaaa cgtttatata ggataaagat gtaggaagaa tagggtattt 3720
taataggttc gtatgtataa acgtatgtgt tttatgattc gtatatatag tatatatgta 3780
taaatattta tagatataag tatatatata tatatattta tatatataag taagattaat 3840
acgggtaatt tttaagatat aaatatagat ttatttaatg aatataaagg aaaatagatt 3900
tagaatttat atatagagat aaatgaatat atatatgtaa atgggttttt gaaggtatta 3960

ttttatttat ataatttata tggaaattta ttattatata cgaacgtata tataatatat 4020
atagtgttga gttggtttcg tagttagtaa aatttttttg agagttttat tttttttaag 4080
tgttaatttt atttttttag gaaagtttta aagatatcga gaaatgcggt tatgttttta 4140
attattattt ttagggatat aatatttttt gtcgtttttt tgaatttagg tttaaggggt 4200
ttacgaaggg gtcgtgttgg gttgaatcgt tggagttggg tattaagggg gagtttatta 4260
ttacgttatt gtgttaagcg ttaagtagag ttattgtgag gtagtgagga ttggaagggt 4320
ttgggaggaa tagtcgggat cgatttttgt tggaaattgg tttttgtagg ttttttttta 4380
tttagaatgt ttgtgattat tgtgtttggg tatatgggtt ttatttggta atatttgtgt 4440
tggtttgtag gattaattta ttattttgta ttattttttt tttattttaa agttgagtgt 4500
taaagatttg tttttggatt taggtatatt tgtttttatt ggtatatttg ggtatatttg 4560
tttttattta ttattgttta tcgttaatat ttgtatattt ttaaatttta gggtggtgta 4620
ggttgggttt agtgtagtat tgtagtcggt tatgtttatg tcgacgaggg ttttggagag 4680
tcgtattttt tttgttttta cgtcgcggtt atttgtttta ttttgggcgt ttatttcgaa 4740
ggttttcgtt tttcgttttt ttattgtttt tttttagtgt tttttttgtt ttttttttaa 4800
atcgtttttt gggaagattt gttgggagtt ttggtttagt ttttgtgaag gggtggaggt 4860
tttgtcgggg aggggtgggg gttaatggtt aatcggtttt tcgtcggtgg ataggttggg 4920
cggggttgta gggatttggt tgtttgttgg tttttggttg atattcgggg tgttaattat 4980
aattgttggg gttttaattt atcgatgttt agttattaat tgtataaggt aggtattatg 5040
atttacgggt atttatttga tttttgattt atttattttt ttaagttatt gttattttaa 5100
gttaggtatt ttaattgata ttattaggta ttgatagttt attttcgaga ttttttaatt 5160
taataatttt ttaaattatt gatttttatt tttaatgttt ttgtagagat aaggttgttt 5220
tatggtttac gatttagaaa tttaaatttt aggttttaga tgttaatttt ttggattttt 5280
gttttgggag ttttttttta gttttttcgt agtttttcgg tttttttggg agtagaatgg 5340
attggaagtt tgggagggtt agatttattt ttaatttttc gtttttgttt tttgtgattt 5400
tattttgttt tttttcgttt tttatagttt ttagtgtttt tggtttaggt tggttttatt 5460
tcggattttt ttattatatt tttttgtttt ttttattttt attttttttc ggagtaggta 5520
gagatttgga tttatattta ggtatttatc gtttattaat tgggattttg ggtaaatgat 5580
tttttttttc gagttttagt tttttttttt gtaaaatgta taattaattt taaagggtgg 5640
tttgagaagg aaatgagatg gtataaatta agtattaagt ggggtttggt agacgtttat 5700
tttttttttt tttttttttt tttttgtttc gtattttttt tgttattttt ttgttttttt 5760
ttattcgttt tttattttta gagtgggttt tgtgggggta tcgttagtgt agttataggt 5820
aatttaattt ttagaaagtt ttggtttttg gtttagtgtt ttgttgtggt tgttttaaaa 5880
tttattatga tttttgaata agggattgtg tatttttgtt ttggacgggg ttttgaaaat 5940
tatatagttg gttttggttg tttgaagttt atggtgtatt ttgttttttt tatttttttt 6000
agtttttatt gttttttgtg tagatatttt taagggttgt taattaattt ggggaagtaa 6060
agttttgatg tagaaggtag agagtgaggg tagaggaaga aaagtttgag atttgggagg 6120
tttgggagtt tgttttttat tgttggttgg aagggtaggt tggagggttt cggtgatatt 6180
tgtgagtaag agtgggcgaa ttagtgggag tattaattaa tgagtgagtg tgtgggggaa 6240
ttgatgagtg ggtgaattag gagtgagtga attagagata gggagaatta atgagtgagt 6300
tgagtgaatg tatgaatgag tgagtgaatg aacgaatgag ggtaaattat tgagtaagtg 6360
agtaaatggg tgggtgaatt aatgagtgaa tgggtgggtg aattaatgag tgagtgaatg 6420
agtgaatgtg tgggtggatt aatgagtgag taaatcggga gttagggtat gtgtgttcga 6480
gtggtatgag gaggtatttt tatgtttttt ttattgttag aagttttttt tttgttttgt 6540
ttagttttat tttgtttatt tgatttgatt tttgaagttg agatgattgg ggagtgttat 6600
tgtttgtagg gaaaggggag agggagtaat tgttgtggag gataagagag gttagatttg 6660
a 6661

<210> 313
<211> 7355
<212> DNA
<213> Artificial Sequence

<220>
<223> chemically treated genomic DNA (Homo sapiens)

<400> 313

attgtggtga gtgggtatta tttaaataat aatttttagt atattttttt ttgtaattta 60
aggtggttat tttaaaattt atatattaag atatagaata aagggtagtt ttgaagtggg 120
ttattttagt atgaagtatt attcgttata tgaaaatcga ttattggttt ttaaatttat 180
ggcgttttaa tggggtaagt ttagtgtttt aaatataggg tgtattatag tattgagtta 240
tttgggttaa ataagagtga tttagaaggc gcgtttaggt ttttttatag agtaaaagag 300
gtttaagttt agagatcggg agaatttggg cggaacgtaa agaagttttt tttcgagtaa 360
atttaaaaat tagtggtttc gaatttcgaa gtagtaaagt taataggaaa gggagaatcg 420
ttgttcggtt ttagtgaatt cgtggggatg tttagtaaaa gttttggtcg ttagaaagag 480
attcgtgggt tttgattttt ggtttaggta aaataggggg aggcgtcgag attagttcga 540

gtattagcgg attttgagag aaattgatcg taatttttat cgtttttttt ttttttttta 600
atttggatgg gttgatttta ttcgtcggtg atttacgacg attgtagcgt tagttatagt 660
ttggcgtttg gtgttttttt tttttttaag tttttttagt ttttttattg ttatcggcgt 720
ataagtaagt gtcgagtcgg ttttcgtaag tcggattagt ttttcggcgt tcgaggttat 780
tacgggtagt agatttttgg tttttagcga ggttgcgcgc gttcgtttcg ttttcgatcg 840
tcgagtagag ttgttagtag aagtaggcgt cggttatttt atataatttt gttgttcgta 900
gggtgtaaga gcgggaaaag tgcggagtag ggaatttttt tgttgcgttg ttttttacgc 960
ggagtttgtt ttttattgtt gaaaagtgtc gggttttggg aagtgttttt ttttttattt 1020
tttatcgaag cgtttattgt cgtcgtggtc gtagttataa attttgttat aaattataat 1080
gataggtgta ttgatatgta tcgtgagagt tttagttgtt taataatttc gttttttggt 1140
cgttgtgagc gtttttattt tatttggtat tatattaggt attgattttt agtagtatta 1200
agtgcggtga gtaagtatta gggttcggtt gttttggagg cgtagcggtt gcggcgggtc 1260
ggcgggttcg gggaagcggg cggtggtcgt ttagagaata tttttttttc ggtaggagat 1320
cgtttggttt tgtatttcgg gtttgcggtt gggtttttaa gttgagttgg gtaatttttt 1380
agtatcgtaa gaaagggcga gttagattta tttggtattt tttttttagg aggagtaggg 1440
gatggcgtcg gcggagtttg gggaggttgt tttggttagt ttttcgggtt agagggtgga 1500
ggagaggagg agggagagga aagggtagtt gaggatttgg aagaaatgag aagtcgtgtt 1560
cgattatttt ttttttaata ttggtttatt tttattttaa gatatttggg ttttggagtt 1620
taaatgagta aaggtattaa agggcgagaa aagaaaggtt ttaaaaaaag ggtgaggggg 1680
tgatcgaaaa gatgagttat tgaatagttg attattgttt aagtgattta tgattcgttt 1740
ttgtttttta ggtttaagta gagtttataa ggagtaggga tttttgaaaa gaaataagtt 1800
tttttattag ttttgtaatt atatttttag gttttgtttt aagagggatt atttttggga 1860
aattgtattt ttgggggatt gtaaagttat ttttagtaag gatagtatta ttattggttt 1920
ttgtttttgg ggaaatggat attaaggtag tggattgtta ttgagtggga atttttttga 1980
gataatgtaa atagttagga aaatagattt tggtttttta ggaagatgtt taatgtttgt 2040
atttttgagg tttgtatata gaattttttt taggaatatg ttatagaagt aaatatatat 2100
ataaagttat ttgttttaga agaattggtt agtttttaag taagttattt taacgtgatt 2160
ggtttattta tttagattaa ttttgttaat ttgggaggta ggagggtgaa tgttttgttt 2220
ttatagttgt aaatggaatg atagagtagt agtaagtttt ggtagtttga gtgttcgatg 2280
tgattattag gtttttagag ggttaagatt gaaagtagga tattagaaaa gtttgaaata 2340
taattaaatt tagagtgttt ttttttttag tagggggtaa atatatgtaa gtattagtta 2400
tagaatgaag atttagttgt ttatttgata tttagtaatt tttaatagag aatgatatat 2460
aaaattaatt aaaagaaaaa ttaagtttag ttttgttaag tagatatttt atttatatat 2520
atttaagaat aataatggaa ataattttta taatatagta ttttggtatt aagggtttgt 2580
tttttattaa gataaatggt atatttattt atattttaaa ataagataaa ataaaaaggg 2640
taataatatt atttattaaa gattaattag aagaatttta aaatttgggt taggttgttt 2700
ggtgtaggtg gagggaaaat tagtgaatta ttttggtatt tgtggttaaa tttattaggt 2760
tatataagtt gtttagatga tgtggtatat tatgatgatt aggatggaag ttagggagag 2820
tttttaggaa ggaaagttgt ttgaaagata gattaagaat tgagattagg gagttgattt 2880
atgagttttt tttattaaat agataaagtg attgaatggt attagttata ttttaatttt 2940
ttaggagttt tttagaagaa gtagggaggt agggagagaa gggaaatttt tataaagaag 3000
ggaatataaa aggggtagga ttttttttag tttttttttt tttttttaat tttgggttta 3060
tttattatgt tatgaaaggt ttttttattg gttgtggttt gggtgggagg tagggtagaa 3120
tagattattt agagtagatg ttaataatat ttatttatat aatagattta aggtaaaaat 3180
aataaagtta taaagtattt tttatttatt tatgttttat gtattttaaa ttttagattt 3240
ttttgtttat ttttagtggt atttgtttaa aagtagggga ggttaaaagt aaaaggttta 3300
gaaaaatgta aatgaaagag aagaaatatt ttttagtttt aaaatgagtt tggaagtttg 3360
taagtttttt tgttggtgtt tattttttag ttttttatat tgtagatttt atattgttga 3420
tttatagagt ttatttagtt gatttttatt ttgttatcgt taagtggatt atttggattt 3480
ttatgtaaga tgttgtgatt ttgaaattag gtggggggag attttttttt tttttttttt 3540
tttttttttt tttaagtttt ggttgttttg agtatgttat aggattttta ttttttatta 3600
aaattttgtg ttgatttaat gattgattga ttgatttatt tattaattta tttatttaat 3660
aagtatttat tgtattaatt atatgtaggg tattgtgttg gatgttagga atagtagata 3720
aatgatatag tttttgtttg taaggagttt atagtttagt gggcgaatta gttaataaaa 3780
tgtttgaggt tataagtatt tttttatata gaagaaaggt taaatggata attttgaaga 3840
aagtaaattg tatttggagg tagagggaag tttttttttt tagttatagt tgagttaaaa 3900
agaaggaaat tttttttata tttaggaaaa attttttttg atatttttag aggtttaaat 3960
aagttgaatt ttataaaatt tgttaggcgt agtggtttat gtttgtaatt ttagtatttt 4020
gggaggttaa gacgggagga ttataagagt ttaggaattt aagattagtt tgggtaatat 4080
tgtaagattt tgtttttata aaaaaaaaaa aaaaaaaatt aagagttggc gtagtggttt 4140
atatttgtaa ttttagtatt tagggaggtt taggcgggtt gattatttga ggttaggagt 4200
ttaagattaa tttgattaat atggtgaaat tttgttttta ttaaaaatat aaaaaattag 4260
ttaggtgtgg tggtaggtgt ttgtaatttt agttatttag gaggttgagg taggagaatt 4320
gtttgaattt agaggcgtag gttgtagtaa gttgagatta tgatattgta ttttagtttt 4380
ggtaatagga gtaaaatttt atttaaaata aaataaaata aaatattaag atatgtagtt 4440
aggtatggtg ggtatttgta gttattgtag agtagttggg attttgaaat attttgaaat 4500

agttattaat attgtagaat agttgggatt ttgaaatatt ttgaaatagt tattaatatt 4560
gtagaatagt tgggattttg aaatattttg aaatagttat taatattagg ttaaagtggg 4620
aggatcgttt gagtttagta aattgaggtt gtagtgagtt atgtttatgt tattgtattt 4680
tagtttgggt aataagtaag atattgtatt aataaataaa tagataagta aataaattta 4740
gatttagtat tttgtttatt ttttcgtcgt gaagtgggta ggaagtagag agtatgggtt 4800
agttttttta tattgattgg ttttgttaat gatatttttt ttggggtttt ttgttttttt 4860
tttgataggt tatttggagt ttgggggtcg gtttagtttt tttaggattt agtagatatt 4920
ggaggtggta gtgaaggata tagtggtagt taatgttatt tgagtagggt tagtaggttt 4980
tggagttttt tgagtgtata atgtagaagg ttgtttatta tgaaaattta ggattgtttg 5040
gaggttatgg ttatagtaaa attacggata tttacggtta tagtattttt tattagtttt 5100
atttattttt tgttgttgtt agttttttgg atattgatta tttaggtttt gtttgtttta 5160
tttagagttt tgtttttttg agagttttag tttataaagg agttgaattt aatggtagtt 5220
gtatgcggtc gggtattggg aatagttagg gtgggggtgg tggtagttag ttttttggtt 5280
tgaatttaga gtagtagtta ttataatttt tttttttatt atcgattttg tttttatttt 5340
tatttattaa ttttggaggt ggagtgtttg ttaagaagtt taaaggtggg tttaatgttt 5400
ttagtttttt agttattatt agtaagtaga tttttttttg gatgaaagag tttcgataga 5460
attttaagta gaagaatagt tgtgttattg taggtagttt tttgaggtgg tttattgtta 5520
gattaagttt tttttagatt gatttaagga agtttagtta gggttggaaa tgtaattttg 5580
gaggttatat gtttaaattt ttatttacgt taaaatgttt ttttttttag tgtttttgat 5640
tggggttatg attttgtagt gatagggtgt ttttttttat tttaggttgt tggtgttgtt 5700
gttggatagg ttttatagtt attaatagag agtgtttttt tatatgggta tatttgtttt 5760
tttgggttgt taattataat tttatttttt tttttattta atagtttttt aagatttgaa 5820
gataggtatt ataattttta agtttaggtg attatatagt ttatattata cgaattttat 5880
ttttttaaat ttataaaaat taaagtttta gaaagtatta tattaagtat tttttaagta 5940
ttatttaatt taatttttta aagaattttt tgaggtaggt atatgataat atttttattt 6000
tatagataag aaaattgtta gagaggatag gtaatttgtt taagattttg aaattgtaaa 6060
gtggtggatt tgaatttagt tagtttggtt ttaggggttg ttaagtataa ttatgagttt 6120
ttatttggtt tattttagtt taattttttt attttagtaa atttataatg gggaggtgtt 6180
tgttttagta ggagaggata ttttgggaga tagtaatagt tttggatttt tttaattgga 6240
aggggagttt ttttagttgg ggtttttttt aggtttattt agggtatgta gaagataggt 6300
atggttagga attttgaggg ttgttttttt tttttgttta gattgtttgg tttttgaaat 6360
tttttgattt tgtggtattt gatgtggttt atttttaggt agatgaattt gtttttaggt 6420
ttagggtaag tttggggttt ggggtgtggt ttgttattag ggatttggtt tgtttgatgt 6480
tttttggggt tgttgttttt agggagaggg tattattttg tttgtaattt tttttttttg 6540
tttttttttt tttagttgta ggtttaggtt ttttttttta ggagaaattt atttgttttt 6600
tttgggaggg agtggataag tagttgagag gtggtagggt agtaaaagtt agtgttgagg 6660
ttgttgtttt tagtattgtg aatatttaaa atgtttttag tttggttttg gattttttaa 6720
aatatttagg tagtgttttt ttttgtttgt ttgtttggtt ggtttttttt tattttttat 6780
tttttggttt gttatttttt tggttttgag tgtagttggg ttgtagtggg gcgttaagat 6840
ttttattttt atattttgtt gggggttggt ggggggtttt gtttagaggt tagtttttaa 6900
gtaaggtagg ttggagttat tttttttttt tttttttttt tatattttat ttttggtagt 6960
agaggttggg aggagtgttt aaaggagttt ggtttttttt tgatagaggg aggttttatt 7020
agttgttttt ggttttttat taaatttttt tatggttttt gggtgggtat gggatgatgg 7080
attaggttgt aggggagagg gtgggtagag tgaattggat tttagaaggt tgatggaggt 7140
tttaggtgcg attgataggt aggtttagta gggggttggt aggtaggtga attttttttt 7200
ggaattatat tttttaaacg gttttttttt tttttagtta ggattggagg tggggggagg 7260
gaggaggaaa agagaattag ggaagtattt ttttttagtt ttgagggttt ttatttattc 7320
gtttagcgtt tttttttttt ttttttttgt ttagg 7355

<210> 314
<211> 7355
<212> DNA
<213> Artificial Sequence

<220>
<223> chemically treated genomic DNA (Homo sapiens)

<400> 314

tttgggtagg gagggagaga gggagggcgt tgagcgagtg ggtggggatt tttaggattg 60
gagaggggtg tttttttggt tttttttttt tttttttttt ttttattttt aattttggtt 120
ggggagggga agggtcgttt gagaggtatg attttaaggg ggaatttatt tatttattaa 180
ttttttatta ggtttattta ttagtcgtat ttgaaatttt tattagtttt ttgagattta 240
gtttattttg tttatttttt tttttgtagt ttagtttatt attttatatt tatttaaagg 300
ttatgaaaga gtttaatgag agattaggag tagttggtaa ggtttttttt tgttaaagaa 360
gggttagatt tttttgaata ttttttttag tttttgttgt taggggtggg gtatggagaa 420

ggaaaggaag aggaaatagt tttagtttgt tttgtttagg agttggtttt tgggtaaagt 480
tttttattaa tttttagtaa agtatgggga tggagatttt ggcgttttat tgtagtttaa 540
ttgtatttaa agttagggag gtgataaatt aaaaaataaa aaataaaaaa aaattaatta 600
aataaataaa taaaaaaaaa tattgtttgg gtatttaggg gagtttaagg ttaggttgga 660
agtattttga gtatttatag tgttaggagt agtagtttta atattggttt ttattatttt 720
gttatttttt agttgtttgt ttattttttt ttagggaaga taaatggatt tttttgggga 780
gggagggttt gagtttgtag ttgaggaagg aggggtagga aaaggaggtt gtaggtagga 840
taatattttt tttttaggag tagtagtttt agaaaatatt aggtaaatta gatttttgat 900
agtaagttat atttaggttt ttaaatttgt tttggatttg gaagtaagtt tatttatttg 960
aagataaatt atattagata ttataaggtt agaaaatttt aggaattaag tagtttaagt 1020
agagagaagg aatagttttt agagtttttg gttatgttta ttttttatat gttttgggtg 1080
gatttagaga aggttttaat tggaggggtt ttttttttag ttagagaaat ttaaggttgt 1140
tattgttttt tagaatattt ttttttatta ggataggtat ttttttatta tggatttgtt 1200
ggagtgggag aattgggttg aggtgggtta aatagagatt tatggttatg tttagtagtt 1260
tttaaagtta gattgattgg atttaaattt attattttgt agttttagaa ttttgggtaa 1320
gttgtttatt ttttttaata gtttttttat ttgtaaaatg gggatgttat tatatattta 1380
ttttaaaagg ttttttgaaa aattaaatta gataatgttt agaaagtatt tagtatggta 1440
ttttttaaga ttttagtttt tatgagttta agggaatgag attcgtgtga tatgggttat 1500
ataattattt aggtttgggg attgtaatat ttatttttaa attttgaaga gttgttgggt 1560
ggaagaggga atggagttat aattagtagt ttaggaaaat agatatgttt atataaggaa 1620
gtatttttta ttaatagtta taagatttgt ttagtaatag tattagtagt ttggaatgga 1680
agggagtatt ttgttattgt aaggttatga ttttaattag gaatattaaa aaaaagaata 1740
ttttgacgtg agtaggagtt tagatatatg atttttaagg ttgtattttt agttttgatt 1800
aggttttttt gggttaattt ggagggggtt tggtttggta gtaggttatt ttagggagtt 1860
atttgtagtg gtatagttgt ttttttgttt ggagttttgt cgagattttt ttatttaggg 1920
gaagatttgt ttgttgatgg tggttgagga gttagaagta ttgggtttat ttttgggttt 1980
tttggtaggt attttatttt taggattggt gggtgaagat gggggtaggg tcggtggtgg 2040
aggagggggt tgtggtggtt gttgttttga gtttagatta ggaggttggt tgttattatt 2100
tttattttgg ttgtttttag tgttcggtcg tatgtagttg ttattgagtt tagttttttt 2160
gtgggttggg gtttttagag gggtagagtt ttggatggag taggtagaat ttggatagtt 2220
agtgtttagg gagttggtag tagtaggggg tgggtagggt tggtgggggg tgttgtagtc 2280
gtaagtgttc gtagttttgt tgtagttata gtttttaaat agttttgggt ttttatagta 2340
agtagttttt tgtattgtgt atttaggaag ttttagggtt tgttgatttt gtttaaataa 2400
tattgattat tattgtattt tttattgtta tttttaatgt ttgttgaatt ttgagagagt 2460
tgggtcggtt tttaggtttt aggtgatttg ttagaagaaa agtaagaggt tttagaggga 2520
gtgttattgg taagattagt taatatagaa ggattagttt atgttttttg ttttttattt 2580
attttacggc ggggagatgg gtaaggtgtt ggatttgagt ttatttattt atttatttat 2640
ttattaatat agtgttttgt ttgttgttta ggttggagtg tagtggtatg aatatggttt 2700
attgtagttt taatttattg ggtttaagcg atttttttat tttagtttgg tattaatagt 2760
tattttagag tattttaaag ttttagttat tttgtagtat taatagttat tttagagtat 2820
tttaaagttt tagttatttt gtagtattaa tagttatttt agagtatttt agagttttag 2880
ttattttgta gtagttatag gtgtttatta tgtttggtta tatgttttag tgttttgttt 2940
tgttttgttt tagatggaat tttgtttttg ttgttaaggt tggagtgtag tgttatgatt 3000
ttagtttatt gtaatttacg tttttgggtt taagtaattt ttttgtttta gttttttgag 3060
tagttgggat tataggtatt tgttattata tttggttaat tttttatatt tttagtagag 3120
atagggtttt attatgttgg ttaggttggt tttgaatttt tgattttagg tgattaattc 3180
gtttgggttt ttttaagtgt tgggattata ggtgtgagtt attgcgttag tttttagttt 3240
tttttttttt ttttttgtag agatagggtt ttataatgtt gtttaggttg gttttgagtt 3300
tttgggtttt tgtgattttt tcgttttggt tttttaaagt gttggaatta taggtatgag 3360
ttattgcgtt tggtagattt tatgaagttt aatttatttg aatttttgga agtattagaa 3420
ggaatttttt ttaaatgtaa gaagagtttt ttttttttta gtttaattgt agttgaggga 3480
aagggttttt ttttattttt agatataatt tatttttttt aagattgttt atttagtttt 3540
ttttttgtgt ggaaaagtat ttatagtttt agatattttg ttggttgatt cgtttattaa 3600
attgtaagtt ttttgtaggt aggggttgtg ttatttgttt attgttttta atatttagta 3660
tagtgttttg tatgtagtta gtgtaataaa tgtttgttga ataggtgaat taataagtgg 3720
attaattagt taattattgg gttagtataa ggttttgatg ggaaatgaaa gttttataat 3780
atgtttagaa tagttagaat ttaaaaaaaa aaaaaaaaaa aaaaaaaaaa agggtttttt 3840
tttatttggt tttaaaatta tagtattttg tatgaaagtt tagatagttt atttaacgat 3900
gatagggtga aaattagttg agtaagtttt atgaattagt agtgtaaaat ttataatgta 3960
gaaaattagg ggataaatat taataggagg atttgtagat ttttagattt attttgaggt 4020
tagaaaatgt tttttttttt ttatttatat ttttttagat tttttatttt tgattttttt 4080
tatttttaaa tagatattat tggaaatggg taaagagatt tagaatttga aatgtatgga 4140
atatgggtga gtggagaatg ttttataatt ttattatttt tgttttggat ttattgtata 4200
aatagatatt attgatattt gttttggata atttatttta ttttattttt tatttaaatt 4260
ataattaatg agagaatttt ttatgatatg atgaatggat ttagaattag aagaagaaaa 4320
aaaagttaag aaaaatttta ttttttttgt attttttttt ttatgggggt tttttttttt 4380

tttttgtttt tttatttttt ttgaaaggtt tttaaagagt taaagtataa ttaatattat 4440
ttaattattt tgtttgtttg ataaagaaaa tttataggtt agttttttgg ttttaatttt 4500
tgatttgttt tttaggtaat tttttttttt agaaattttt tttggttttt attttggtta 4560
ttatgatgta ttatattatt tagatagttt atgtggttta atggatttga ttataggtat 4620
tagaatgatt tattagtttt ttttttattt gtattaaata gtttaattta aattttaaaa 4680
tttttttaat tagtttttgg taggtagtgt tgttgttttt tttgttttgt tttgttttag 4740
aatataagtg agtatgttat ttgttttgat gaggaatagg tttttaatat taagatatta 4800
tgttgtagaa attgttttta ttattgtttt taaatgtatg taggtaggat atttatttaa 4860
taaagttgga tttaattttt tttttgatta attttatata ttattttttg ttaaaagtta 4920
ttggatatta aatggatagt taagttttta ttttgtgatt aatatttata tatgtttgtt 4980
ttttgttgaa gaaagaggta ttttggattt ggttatgttt taggtttttt tggtattttg 5040
tttttagttt taattttttg gaggtttaat ggttatatcg gatatttaga ttattagaat 5100
ttgttgttgt tttgttattt tatttataat tgtggaagta aaatatttat ttttttgttt 5160
tttaagttga taaagttgat ttagataggt gaattagtta cgttaaaata gtttatttag 5220
gaattgatta gtttttttgg gatagatggt tttatatata tatttgtttt tatggtatat 5280
ttttagaaaa agttttgtgt atagatttta aaagtataaa tattgggtat ttttttgaaa 5340
aattagagtt tgttttttta attgtttata ttgttttaag aaatttttta tttaatgata 5400
atttattatt ttaatgttta tttttttaaa agtaagagtt agtgatggtg ttgtttttat 5460
tggaaatggt tttgtagttt tttaaaaatg tagtttttta aaagtgattt tttttaaaat 5520
aaaatttgag aatatagtta taaaattggt aaaaaggttt attttttttt agaaattttt 5580
attttttgtg aattttgttt gaatttaaaa agtaggaacg ggttataaat tatttggata 5640
atggttaatt gtttagtggt ttattttttc gattattttt ttattttttt tttaaagttt 5700
tttttttttc gttttttggt atttttgttt atttgggttt taagatttaa atgttttggg 5760
gtgaagatga gttagtgttg ggggaggggt ggtcgagtac ggttttttat tttttttaag 5820
tttttagttg tttttttttt tttttttttt ttttttttat tttttagttc ggggaattgg 5880
ttagggtagt ttttttaaat ttcgtcggcg ttattttttg ttttttttgg gaaaggggtg 5940
ttaaataggt ttggttcgtt tttttttgcg gtgttgggaa gttgtttaat ttagtttgga 6000
ggtttaatcg taggttcgga atatagggtt aaacggtttt ttgtcggaag gaaggtattt 6060
tttgagcggt tatcgttcgt tttttcgggt tcgtcggttc gtcgtaatcg ttgcgttttt 6120
aaagtagtcg aattttgata tttgtttatc gtatttaatg ttattagaga ttaatattta 6180
atatgatatt aaataaataa aaggcgttta tagcgattag gggacggggt tattaagtag 6240
ttggagtttt tacggtgtat attaatgtat ttgttattgt ggtttgtagt aaaatttatg 6300
attgcgatta cggcggtagt aaacgtttcg gtaaggaatg aaaagaaaaa tattttttaa 6360
ggttcggtat tttttagtag tggaaagtag gtttcgcgta ggaggtagcg tagtaaaaga 6420
attttttatt tcgtattttt ttcgtttttg tattttgcga gtagtaggat tatataaagt 6480
gatcggcgtt tgtttttgtt agtagttttg ttcggcggtc ggaggcggga cgaacgcgcg 6540
tagtttcgtt ggggattaaa aatttgttgt tcgtggtggt ttcggacgtc gggaggttag 6600
ttcgatttgc ggaggtcggt tcggtatttg tttgtacgtc ggtggtagtg gaaaagttga 6660
gggggtttgg gaagggaggg gatattaggc gttaggttgt gattagcgtt gtaatcgtcg 6720
taaattatcg acgggtagag ttagtttatt taagttgaaa gagaggggga aggcgatgga 6780
ggttgcggtt agtttttttt aaaattcgtt agtgttcgag ttggtttcgg cgtttttttt 6840
tgttttgttt aaattagggt tagaagttta cggatttttt tttggcggtt aagatttttg 6900
ttgagtattt ttacgaattt attggaatcg agtaacgatt tttttttttt tgttggtttt 6960
gttatttcgg gattcgaaat tattggtttt tgggtttgtt cgaaaggaga tttttttacg 7020
tttcgtttaa gttttttcga tttttaagtt tgagtttttt ttgttttata agaagatttg 7080
gacgcgtttt ttaaattatt tttatttagt ttagataatt taatattgta atgtattttg 7140
tatttaaggt attgggtttg ttttattaaa gcgttataaa tttgaaggtt aatgatcggt 7200
ttttatgtaa cgggtggtat tttatattga agtgatttat tttaaagttg ttttttgttt 7260
tatgttttga tgtatgaatt ttgagatggt tattttgagt tgtagaggga aatgtattgg 7320
aaattattat ttaaatgatg tttatttatt ataat 7355

<210> 315
<211> 7219
<212> DNA
<213> Artificial Sequence

<220>
<223> chemically treated genomic DNA (Homo sapiens)

<400> 315

gtgggggtag tgtttttaaa atttttattt agttttgagt ttttaaagtt tttgagtttt 60
aattattgtg tttggagata ttatgggagt ggggatattt gattttttag gtttggattt 120
tttgagagtt ttatttgttt ttttatattt tgtcgttttt atattagggt tgttttattg 180
ttttataatt ttttttggag ttttagttgt taaagatttt aatgaggtta ttttttattt 240
ttttatttta ttaaatttta agttttgttg tgttaagtag tttggttatt tgtaaaggtt 300

ttttttttag atttaagcga attatagaaa tttttaggta tgtggttatt tttaattttg 360
tttaagttta tgtataataa tatggaggga gttaagtgtt aattttaatt gttattttta 420
tttttaaatt tatttagttt ttttttaatt attttttttt tattgaaatt tgaaaaaaat 480
tttaaataat ttttaatttt tgttataagt aaataagaag tggtttttga tttttttttt 540
tattatattt agagttagaa aattattgaa ataaaggggt tgatggtatt tatagtgaat 600
gtcgttaaag aattataaga tatgtttatt aattagtttt ttttaaatag tattattttt 660
taatttaaat agttttttcg aaaagatgtt atttgaaaag tttggggtat tgatatgaat 720
atgatttttt ttgtattttg gatatttgga attatttaaa tttgaggtaa tcgttgtagc 780
gaatttttat ttagtgaata aaagtttaaa gttaaaggtt atttatggtt ttgtagagat 840
gggatttgag tggttattta cgagtacgtg attttaataa attttgtttt atggtttgag 900
agttgataag ttaaaatata atttttatta taaattaggt taagagtata taagtgtaga 960
tgttgggttt ttggagtagt aataggaacg tgagaggttt tagttagttt taggttaaga 1020
aggagacggt tttattttta gtttttttgt ttattaggaa agaaaaatag taagttattt 1080
attatttttt ttataataat tttagaggtg ggagattttt ttgtttttga tttttttgtt 1140
taggatgggg ataagtttta tcgaagttaa ttttaatttt agtttaaatt taaattaata 1200
aatagaatga gtatttcgat tttttatgtt tttttttttt gatagggata gttaggaatt 1260
gagggttttt attttataag agggatttat tgtttttttt ttttttttta agagtatttt 1320
ttttagaagt ggaataaaat agtggtattt tgggtgggga aagaaagaga ggatgagggt 1380
gaagttgttt tgtttttgag atttttgata ttgttttttt ataataatat taggaaagat 1440
tgaaaattta aaattggata ttagtttttg ggaggattat tgtttagtaa gattgttttt 1500
tgggtttttt agtagtaatt ttgaaaaatt gatgttatat ttatagagtt ggataataat 1560
ttaaagtatt gttatttatt tttaaattta ttgatttttt ttttttttgg ttattatttg 1620
ggtttttatt tttttttgga aattgttttt tgttttgaat tggagtagaa gtggtaagtt 1680
ttagttatgt ttgatttttt tttgaagagg agaagatagg ttttgggggt attggttggg 1740
gttgaggaat ttgaaattaa gttgaagttg gaaagggagg ttgaattgtt tttagggtgg 1800
gagagatagg aagtaggggt gaggggtaga tggagcggtt attttttttt tagtggattt 1860
gtatttattc ggttcgattg cgtagaagga aatatataaa ggtttatttt tttttcgttt 1920
ttcgtttttt tattgggaaa attttgttat aggcgatttg attcgggttt agtaattagt 1980
gaggttttgt ttaagggtgc gtggagtggg ggaggcgatg gagatcgaaa tttgttataa 2040
ttcgtggttt tgtttttgag ggattgcggt tttcggtttt gcggagaatt ttgcgtagtt 2100
ttcgggggcg ggtatttgaa gtagggaggt ttgggtattg gtggggtatt tgggttttgt 2160
tatttttttg tgtgttatat gtgtttgttt tattattttg tggaatttaa gtgattttcg 2220
tttttattgt aataggttta ggattatgag agagcgtcgt tgtaagtatt ttttcgtttt 2280
tttcgaatcg cgaagagacg gtattgatcg cggggaagtg gtgggttaag gggttaagtt 2340
agcggagtgg gcggatagaa gaaaggtttt gtttttttta ttttattttt attttttatt 2400
ttcggtttcg ttttgcgagg ttagttcgtt tcgggaggtg gaggcgttaa atcgaggttt 2460
cggaaagttt ttttggggtt tggtttatag atatttgttt gagagaacgg ttcgcggggt 2520
ttcgtttttg tttttttggg tttttttaga ttttcgcggt ttttttttgt tttgtatagg 2580
tgtagtttcg ttaaggcgtt tatttttgtt aagagtttgt taattagtag tttgttttat 2640
tagcggtttt aaattaacgt agtaaatatt aaggtaatgc ggtttttttc gtagtttaat 2700
tttttttaat ttggtttagt ttggttaatt ttttgttttt aaaaaaattt attgaaaata 2760
aaaatttaat tttttgtggt cgtgggtgtg ttgagttgtt cgtttttgag ttttatgtgg 2820
atattttttt ttcgtttttt tttcgatttg tttgtagcgg tgtgggtttt gggtttggga 2880
tattggttga gcgaggaggg atacggattt tttttttatt tttgtttttt tgtaattgta 2940
gttggagaat tttcgtttta gaggcgttgg tgttgtaagg aggtatagga gggtggagtg 3000
gggtggatgc ggtggggtgg ggttagagat tcgtgggatt tttggtttgg atgtttggat 3060
ttttttgagt tgtttgtgtc gcgaaagata ggtatatttt tgattaggtt tgtgaagttt 3120
tttggaggat tattttatta agacgatggt attggaggga gagttataga aagaattgtg 3180
gttttttttt tattgtaaaa cggaagtgat tttattttaa tgggagttgg aatatgtgag 3240
ggttgtagga attagttttt tttttttttg gttggaaaag ttggggttgg ttttagagat 3300
aggttttttg gtttcgttgg gttgggtagt ttagtcgatt ttttgtagat tgtgtatatt 3360
tttagaagag taattatttt tatatattag gttggtttaa gtgaaagggg ttttgggttt 3420
tagtttggaa aatttggtgt tttggggatt tttggttttg tttttttttt tttttgtatt 3480
ggttttgggt gtttattttt gtagaagttt ttcgttagta aatttatatt tagcgttttg 3540
tagttgaata tagtataaaa agttttagag attaaaagta ttagtatggg tagttgagcg 3600
ggaggtgaat atttaacgtt tttgtttatt aataattcgt tggttttgat ttgtttgaat 3660
aagtcgagta ataaggtgaa atgtaggtta tagcgtttaa taaatatgaa aatgtgtata 3720
tttatttcgg tttttagtcg gtttagtagg tttttttaat ttgtttggat agagtttagg 3780
tttagtgttt taggaaggtg gatatttggg agtttttggt atttagggaa attgggggga 3840
tttttttggg ataaatagat tttgtagttt tatagttttt gttagaattg gcgtttttaa 3900
aatttttacg aaataggtaa agtggtggaa gtaaaggatg gggggatgag taaagtgtta 3960
ggtttggagg gggagtattt ttgttttatt ttattaggtt taggtttagg tttgaaatta 4020
aattttgaat tatgtgggag gtaggaaggg taattaagat agttaaaaaa aaatttaata 4080
tttttagttt atagttaggt atttaaaagt tttattcgag ttagggtatt tttgtgtttt 4140
tgaaatttat cgaggttttg agtgagtttg cgttttttat gttttgaatt gagggataag 4200
tattgtatag tgtcgaggcg ttggggcggg tgaaatttgt gaatttttgt atttttgtgt 4260

gttgttgtcg gtaaagtaaa aataatgaaa gtttgtgata tgtttgtaaa tgatttcgaa 4320
tgatttttcg ttttgttttt tattattagc gcgtcggttt tagtttaagg cggttcggtg 4380
tttgtgagta gcgtttgttt cggggtttgt agtagtagcg gaacggagag gttagttcgg 4440
attagggttt atgaaggtaa atgcgcgatt ttttggagaa gtagttttgg tagataattt 4500
ggtttggtcg ggtagtagga aaaggagtag atataggata tttttttttc gttttttttt 4560
tttttgtcgt tgagggtatt tgtttttttt tggaaaatat tatttggtaa ggaggagggt 4620
tatttggatt ttggtggggg agggtggtta ataaagtcgt tatttttggg atggattatt 4680
tttttttttt tttttttttt tttttttttt aagaagaata ttttggttgt tcgtttgttt 4740
ggtaattttg attttggtag aagaatgagg gaatttattg ttttaaattg tcgttaagtt 4800
tattaggtta tttgaattgt tttagaaagt gcgggtggtt gcgtcgaacg gtggtggttt 4860
agaggaagag attggggtcg gtagcgattt aggtatttta ttttgggtgg gatttagagg 4920
ttgtaacgtt gtttatatat attttgtaga atcgaatttg tgtggtattc gtatagttat 4980
agattcgatt ttaggggaat atatggtcga tgtaaaaatt ttacgttttt tcggaatagt 5040
tagagattaa agtgcgatat ggagattaga agtagtcggc gttggttagt cgtttcgttt 5100
tgttttatta ttttggattt taggaggatt agttgcgttt ggtgatatag agtagttttt 5160
tttttttaga agttttttat ttttaaatag agtatttttt gggtgttgaa aagaaagaaa 5220
gatagaaaga gagaaagaga gagagagaga gaaagagaga atgtaagttt aattggttgt 5280
atggatgtag ggttaaaggg ttaggttttg gggtattagg gagtgaggta taaggttagt 5340
ttgtttagtt ttagttttgt tttttaggaa tatgaggtgt aaaggtattt aaatgggggt 5400
ttgtttgtat ttggggtttg tgggaagaaa gtaagtttta aagaagttta gtggggagtt 5460
ttagggtgta ttttgataag gtggaggtgt ttttgttatt attttagttt atttttagtt 5520
atatgggtaa gggtagcgag ggttttttgt tattttggta gggtttagtt ttggttggga 5580
attttcgggt ttgggtattg gtagaaagta tggcggttat ttattgttta atttgattta 5640
agttggttag attttggtaa tttttgggtg attttgatga agataaagtt aggacggcgg 5700
tttttgtatg gtagaatttt tgttttcgtc ggttgtaggt agggcgggta ggtaggaatt 5760
tttttcgttt ggggtatttt gtttaattta gaggcgagtt tatttattta ttttttattg 5820
ttttgtattt taataggagg atttattttt ttttgagttg tgtttatttg gtgtcggggg 5880
gcgggggttg tatttagttg ggggtgagtg gaagggttac ggaaggttgg taaaattagt 5940
ggtagataaa agttgggatt atttgagggg aatggggtgt tggggattgg aattatatta 6000
atatttggta ggggttttta aatgtgttat agtaagttat ttgattatac gtatgttatt 6060
tagttaaatt tgtgaaaatt atgagatgtt tattaattcg gtgataaatt tgttttttcg 6120
ttattggttg gtttggttat atggttgttt aattttattt agttgatagt aagtaggagg 6180
gttttatgga aggagaaaaa aagataatac gagaaaaatt agtatttttt attttttgaa 6240
attaatggtt atgagttcgt atatggtgaa ttttaagtat gtggatttta agtttttttc 6300
gtgcgaggag tatttgtagg gcggttattt aggcgagtag ggcgtcgatt attacggcgg 6360
cggcgcgtag ggcgtagatt tttagttttc ggggttttat ttacggttcg atttcggtga 6420
gtagtttttc ggaggtagcg gtttcgggtt tggttcggcg ttgtttgcgc ggggttacgg 6480
ataagagtta ggcggtttcg gcggttatta cgtcgtttta ggagagtttt gtttagtttt 6540
ttcggcgttt tcgtcggcgt tttgtttggg cgttcgggtt tatagttagt tcgattttaa 6600
gtagtcgttt ttcgggacgg tatttaagta gtcggtcgtg gtttattttt ggatgaagaa 6660
ggtgtacgtg aattcgggta aggttagggt ttagtaattt ttttgtttat attttagttc 6720
gttagtttgg gttttttgga agggggtgcg agtaggtggg ggcgtgtgga gtttttatgg 6780
gcgtcgtaat tatttttttt ataaattttt atagttgagg gagtaggtta ggattatgtg 6840
gttggttgtt cggttgtggg cgtaaaaggg ggtggggatg ggggggtggg ggaggatttt 6900
atttttagag tagggggaag gttgtggagg agcgggggat ttttaaaatg tttgagggtt 6960
tcggatttgg tggtgggttt agaagaagga gtatatttgg ggatttcgta agtttggggt 7020
atgtgggtgt gtttgaggag gtgggtggga gtgagcgtgt gcgtcgggga gagggcggga 7080
gggaggaagt aagcgagttt gggagcgcgc ggggagggtc gcgggtttcg ggcgcgttta 7140
ggaagtgagc ggcggaggcg aggggtttaa ttagtggtcg ggcgttgatt tgtttgtttt 7200
gtttgttttg tttcgtagt 7219

<210> 316
<211> 7219
<212> DNA
<213> Artificial Sequence

<220>
<223> chemically treated genomic DNA (Homo sapiens)

<400> 316

attgcgagat aaaatagata ggataggtag gttagcgttc ggttattagt taggtttttc 60
gttttcgtcg tttatttttt ggcgcgtttc gaggttcgcg gtttttttcg cgcgttttta 120
agttcgtttg tttttttttt ttcgtttttt tttcggcgta tacgtttatt tttatttatt 180
tttttaaata tatttatata ttttaggttt gcgggatttt taaatgtgtt tttttttttg 240
ggtttattat taggttcgga atttttaagt attttggaaa tttttcgttt ttttatagtt 300

ttttttttgt tttgaaaatg gagttttttt ttattttttt atttttattt ttttttgcgt 360
ttatagtcga gtagttagtt atatggtttt gatttgtttt tttagttata aaaatttatg 420
gggagagtaa ttgcggcgtt tatggaagtt ttatacgttt ttatttattc gtattttttt 480
ttagaggatt taggttaacg ggttgggatg tggatagaaa ggttattgga ttttagtttt 540
attcgaattt acgtgtattt tttttattta ggggtagatt acggtcggtt gtttgagtgt 600
cgtttcggag ggcggttgtt tggggtcgga ttgattgtag gttcgggcgt taggtagggg 660
cgtcggcgga ggcgtcgggg gagttgggta aggttttttt ggagcggcgt agtgatcgtc 720
ggggtcgttt ggtttttgtt cgtgatttcg cgtaggtagc gtcgagttag gttcggggtc 780
gttgttttcg aaaggttgtt tatcgaagtc gggtcgtggg tagagtttcg ggggttggaa 840
gtttgcgttt tgcgcgtcgt cgtcgtagta gtcggcgttt tgttcgttta ggtagtcgtt 900
ttgtaaatat ttttcgtacg gagggaattt ggggtttata tatttggagt ttattatata 960
cgaatttatg attattaatt ttagaaggta gaaaatatta atttttttcg tgttgttttt 1020
tttttttttt ttatagggtt tttttatttg ttgttaattg aataaagttg ggtagttatg 1080
tgattaggtt agttaatggc gagggagtaa gtttattatc gggttggtga gtattttata 1140
atttttataa atttaattaa ataatatacg tgtaattaag tagtttgtta tggtatattt 1200
gagagttttt gttagatatt aatgtagttt tagtttttag tattttattt tttttaggta 1260
atttagtttt ttgtttgtta ttgattttgt taatttttcg tggttttttt atttattttt 1320
agttgaatgt aattttcgtt tttcgatatt aagtaggtat agtttaaggg agaatgaatt 1380
tttttattgg ggtatagagt aatgaaaggt gggtgggtga attcgttttt gagttgggta 1440
gagtgtttta ggcgaggagg gtttttgttt gttcgttttg tttgtagtcg gcgggagtag 1500
ggattttgtt atataaaggt cgtcgttttg gttttgtttt tattagggtt atttaaaagt 1560
tattagaatt tggttagttt gaattaaatt aggtaatgag taatcgttat gttttttatt 1620
agtgtttagg ttcgggggtt tttagttaaa gttgggtttt gttaaaatag tagggggttt 1680
tcgttgtttt tgtttatgta gttgggggtg ggttgggatg gtggtaaggg tatttttatt 1740
ttgttaaaat gtatttaga gtttttattt gggttttttt gaagtttgtt tttttttat 1800
aggttttaaa tataagtaag tttttatttg ggtatttttg tattttatgt ttttggaggg 1860
tagagttggg attgggtaag ttggttttgt attttatttt ttagtatttt aaaatttagt 1920
tttttggttt tgtatttatg taattaatta ggtttgtatt tttttttttt tttttttttt 1980
tttttttttt ttttttgttt tttttttttt ttagtattta gaggatattt tgtttaaagg 2040
tgaggagttt ttggagagga aaagttgttt tatgttatta ggcgtagttg atttttttgg 2100
agtttaaggt aataaaatag aacgaggcgg ttgattagcg tcggttgttt ttagttttta 2160
tgtcgtattt tggtttttgg ttatttcgaa gaaacgtgaa gtttttgtat cgattatata 2220
tttttttaga atcgaatttg tgattatacg gatattatat aaattcggtt ttatagggta 2280
tatatagata acgttataat ttttgggttt tatttagagt gaggtattta ggtcgttgtc 2340
ggttttaatt tttttttttg agttattatc gttcgacgta gttattcgta ttttttgaga 2400
tagtttaggt agtttaatgg gtttggcgat aatttgaagt aatgagtttt tttatttttt 2460
tgttagggtt agggttatta agtaggcgaa taattagaat atttttttta agaaagaaaa 2520
aaagaaagaa agaaagaaaa ataatttatt ttaaggatgg cggttttatt aattattttt 2580
ttttattagg atttaaataa tttttttttt tattaagtgg tattttttag aaaggaataa 2640
atgtttttag cggtagaaga aaaagaagcg agaggggaat atttgtgttt tatttttttt 2700
tttattgttc gattaagtta ggttgtttgt taaagttatt tttttaagaa gtcgcgtatt 2760
tatttttatg ggttttgatt cgggttggtt ttttcgtttc gttgttgttg tagatttcga 2820
ggtaggcgtt gtttataggt atcgagtcgt tttgggttga ggtcggcgcg ttaatggtga 2880
agggtagggc gaggggttat tcgaaattat ttataaatat attataaatt tttattattt 2940
ttgttttgtc gatagtagta tataagagta taaaagttta taaattttat tcgttttagc 3000
gtttcggtat tgtgtagtgt ttgtttttta gtttagggta tggagagcgt agatttattt 3060
agagtttcga tggattttag gggtataggg atgttttggt tcgagtgggg tttttgggtg 3120
tttaattgta ggttgaaggt gttagatttt tttttaattg ttttgattgt tttttttatt 3180
ttttatataa tttaaaattt agttttaagt ttgggtttgg gtttggtggg gtggggtagg 3240
gatatttttt ttttaagttt ggtattttgt ttattttttt attttttgtt tttattattt 3300
tatttgtttc gtagggattt tgggggcgtt agttttaata agggttatga ggttgtagaa 3360
tttgtttatt ttaggagagt ttttttaatt tttttgaatg ttaaagattt ttaagtattt 3420
atttttttgg gatattgggt ttaggtttta tttagataaa ttgggggagt ttgttgagtc 3480
ggttggagat cggggtgaat atatatattt ttatatttgt tagacgttgt gatttgtatt 3540
ttattttatt gttcgatttg tttagatagg ttaaagttaa cgagttattg atgaataaaa 3600
gcgttaaata tttatttttc gtttaattgt ttatattaat gtttttgatt tttagggttt 3660
tttgtgttgt gtttagttat agggcgttga atgtgggttt gttagcgaga agtttttgta 3720
gagataagta tttagagtta gtgtagggga ggagagaagt aagattagag gtttttagga 3780
tattagattt tttagattgg agtttagagt tttttttatt tgagttagtt tggtgtatag 3840
gggtagttgt ttttttaggg gtgtgtatag tttataaagg gtcgattaga ttgtttagtt 3900
tagcggggtt aaaaaatttg tttttgaggt tagttttagt ttttttaatt aagaaggagg 3960
gagattggtt tttgtagttt ttatatattt taatttttat taaaataaaa ttattttcgt 4020
tttgtagtga gggaggggtt atagtttttt ttgtgatttt ttttttaata ttatcgtttt 4080
aatgagatgg ttttttagga ggttttatag gtttaattag aaatgtattt gtttttcgcg 4140
gtataggtaa tttagaaaga tttaaatatt taagttaagg attttacgag tttttggttt 4200
tattttatcg tatttatttt attttatttt tttatgtttt tttgtagtat tagcgttttt 4260

ggggcggaaa ttttttaatt gtagttatag agaagtagag gtgaaaagaa ggttcgtatt 4320
ttttttcgtt tagttagtgt tttaagttta gggtttatat cgttgtaaat agatcggggg 4380
aggggcgggg aaggaatgtt tatataaagt ttagaagcgg gtaatttaat atatttacgg 4440
ttatagagga ttgagttttt gtttttagtg aattttttta aaaatagaag gttggttagg 4500
ttagattagg ttaagaaggg ttgggttgcg gaggggatcg tattgttttg gtgtttattg 4560
cgttggtttg aagtcgttaa taagataggt tgttaattag taagttttta atagaagtga 4620
gcgttttggc ggagttgtat ttgtgtagag tagggaaggg tcgcgaaggt ttagggaggt 4680
ttaaggagat aagggcgaga tttcgcggat cgttttttta ggtaggtgtt tgtgaattag 4740
gttttagaaa agtttttcgg gatttcgatt tagcgttttt atttttcgaa gcgggttaat 4800
ttcgtagagc gaggtcgggg gtggagggtg ggggtggggt gggggaggta gggttttttt 4860
tttattcgtt tatttcgttg gtttggtttt ttagtttatt attttttcgc gattagtatc 4920
gttttttcgc gattcggaag aagcgggaag gtatttatag cggcgttttt ttatggtttt 4980
gaatttgttg taatgaaaac ggggattatt taaattttat aggataataa agtagatata 5040
tataatatat agaaaaatga taaggtttag atattttatt aatatttaga tttttttgtt 5100
ttaggtgttc gttttcggga attgcgtaaa gtttttcgta gagtcggaga tcgtagtttt 5160
ttaagagtag agttacggat tatgatagat ttcgattttt atcgtttttt ttattttacg 5220
tatttttggg taaaatttta ttagttattg ggttcgagtt aggtcgtttg taatagggtt 5280
tttttagtgg gaaaacgaga agcgaggaga aaatgggttt ttgtgtgttt ttttttgcgt 5340
aatcgggtcg ggtgggtgta ggtttattgg ggagggggta gtcgttttat ttgtttttta 5400
tttttgtttt ttattttttt tattttggga gtaatttagt tttttttttt agttttagtt 5460
tggttttaaa ttttttagtt ttaattagtg tttttaaaat ttgttttttt ttttttaaag 5520
aagaattagg tatggttggg atttgttatt tttattttaa tttaaaataa agagtaattt 5580
ttagggggaa atgaggattt aaatgataat taaggaggag gaaaattaat gggtttaaag 5640
gtgaatggta atgttttaga ttgttattta gttttatggg tatgatatta attttttagg 5700
attgttgttg gagagtttag aagataattt tattaagtag tggttttttt agaggttggt 5760
gtttagtttt aagtttttag ttttttttga tattattata ggaaagtagt attagaaatt 5820
ttagggataa agtagtttta tttttatttt tttttttttt ttttatttag aatattattg 5880
ttttatttta tttttgaagg agatgttttt aagggggagg agaggggtag taagtttttt 5940
ttgtgaggtg agagttttta atttttaatt gtttttatta ggaagggagg gtatgggaga 6000
tcgagatgtt tattttattt gttaatttag gtttaggtta aaattagaat taatttcgat 6060
gagatttatt tttattttag gtagaaaagt taggaataga gaaatttttt atttttgaag 6120
ttgttgtgga aaaagtaata agtgatttat tgtttttttt ttttagtggg taggggagtt 6180
gggagtagaa tcgttttttt tttagtttgg agttggttgg agttttttac gtttttattg 6240
ttgttttagg aatttagtat ttgtatttgt atgtttttaa tttaatttat ggtgggaatt 6300
atattttggt ttgttaattt ttaagttata aaataaagtt tattggaatt acgtgttcgt 6360
aaatagttat ttaggtttta tttttgtaga attataaata atttttggtt ttaaattttt 6420
atttattaaa taaaggttcg ttgtaacggt tattttagat ttaaatagtt ttaaatattt 6480
aggatgtaag ggaggttata tttatattaa tattttaggt tttttaaata gtattttttc 6540
ggagaagttg tttaagttgg gagatagtgt tatttggaaa aggttagttg gtgggtatgt 6600
tttatgattt tttggcggta tttattgtga gtattattaa tttttttatt ttaataattt 6660
tttagtttta gatgtgataa agaggaaggt taaaagttat tttttatttg tttatgatag 6720
aagttggaag ttatttgaga ttttttttaa gttttaatgg agaaagggtg gttagggaga 6780
ggttgggtgg gtttgggggt gggagtagta gttggaatta atatttggtt ttttttatat 6840
tgttgtgtat gggtttggat aaagttgaag gtagttatat gtttagaggt ttttgtaatt 6900
cgtttagatt tgaaaggaaa gtttttgtaa gtgattaaat tgtttaatat agtaagattt 6960
ggggtttggt aaagtgagaa agtggagaat agttttatta gaatttttgg taattaaagt 7020
tttaaagaag gttataaaat aataaaatag ttttggtgtg aaggcgatag ggtgtgagga 7080
agtaaatgag atttttaagg aatttaagtt taggaagtta gatgttttta tttttatggt 7140
gtttttaagt atagtgatta aggtttagga attttggggg tttaaaattg ggtgggagtt 7200
ttaggagtat tatttttat 7219

<210> 317
<211> 18679
<212> DNA
<213> Artificial Sequence

<220>
<223> chemically treated genomic DNA (Homo sapiens)

<400> 317

ttagtagtaa atttgaaaga tttttagagt atagttttat tttcggtacg tttttaaatt 60
tagattttat attttgtagt tttttgattt ttttaaaaga tttttttttt aaaagaaaaa 120
ataaaaatta gtttataatt tgttttgagg ttgaggtatt tatttggcga tttgtaggtt 180
tataaggtag gttttaggga aagtatttta attttggggt gattttttgt tacggttttg 240
aagttattat agaaaaatgg agaatgaaga aacgagaata ttaaagggtt tatttaaagg 300

cgcgttttat tttcgtttaa tattttgatt tgcgtttaaa attaaaagaa tcgtttagtt 360
tgtgtattta tttttattta aaatcgtttg tgtagtaggt ttttttatgt tttagtattt 420
tatggaatta aattatatta tattaaaatg ttattagttt aaaatatatt aaataaatgt 480
aagtttgggt cgacgtatga atatttaaag tttatattat acggtgaatt agaagaaaag 540
ggggtgagga gagttaatag aatatcgtat aaatattgat agaaataaag tattttatgt 600
gtaaattttt tttagtaaaa tatggtttta ttatttatat ttattatggt attgttaaat 660
ttgaaaagaa atataaagag aaatgaattg atttacgttt gtttttaata ttaaataatt 720
agaatgttag taaatttttt taatgtttta ttttggattt ttaggtgaga gtggattgtt 780
gggacgtgtt ggtaaatttt aaatataatt gattgtttgt tttttatatt tatatattgc 840
gaatgtaatt ttaggagagt gaattttatt ttaaaatttt ttaagagttg tttttaaata 900
gcgtttttgt gtatatgtat acggtatata tttgacgttt attatgtgta tttgtgcgtt 960
ggaaattagt cgtggaattg aattgaatta gtggatttga gttgaaatga atttatttaa 1020
acgattttag gtttgcgttt tttgaggttt tttttagtaa tatttttaat gaaagtaaga 1080
aaagtagtaa agtaaaataa taatagtaat aatttattat gagtattagg ttattcgaat 1140
atagatttat tgcgtatttt agcgtaattt aagtattttc gggtggcgag tcgttggtat 1200
atggtgtttt agtgagttcg tattcggagt tcgaagtttt gtaggcgtag aggattttat 1260
tgttaattga aatgggtgtt ttgatttaaa ttaattttta gttttttttt gtttcgggcg 1320
tcgtttgaaa tttcgaggcg acggttggtt cgcgatgtgt attttttgcg ttttgcgttg 1380
ggggagttgt aggtggagag gcgtttttat ttatttcggt atcgttttta gggaagtttt 1440
aagtttatat cgtaagggat taattcgtcg cgattcggcg tttattttta atgttgatat 1500
agtttaggtt cggtaaacga agtagggcgg ggttgggtat taggggcgtc gtttgtcgaa 1560
gatgcggtat tacgtgcgcg ttgtacggat ttcgaatttt gtgagtttag tttcggttat 1620
aggttttcga agaattgggt tttcggtttt agggcggggt tttttttttg ttttttgttt 1680
ggatgtcgat ttttatttac gttttgcgtt agtttcgggg tcgagtttta gtgttagagt 1740
cgttttttga ggtttttttt agggttcgta tagagggagg tttagtggcg gacggcgatg 1800
gtttagtttt tgttggattt cgggttaggg gtttggggaa gaggggtatc gggtcggaaa 1860
tgagttttag ttcgggtttt acgagttttg gagtttttaa cgatgttttt tggttgagtt 1920
aaagagttcg atttggtttt ttcgtttggc gatgtcggtt tttttttttt ttgttcgtag 1980
cgttttagtt ggagggtagt tcgtagcgtg tttaggttaa ttttttttgc gttttttttt 2040
tttttttgtt attttttgtt ttcggttttt gttttttttt ttttatagaa tgttagagcg 2100
tttaggggtt tggaggagtt ttcgtattag tttttttttt gggtttttaa tttgtttttt 2160
ttatagttta gtttttttttc gtttatattt tttttttgtt tagatttgt tcgtattttt 2220
tcgtcgattg agttagtatg tgtttttgtt tttaaatttt ttttttcgtg taatattaaa 2280
taaattaagg tgtgttgagt tggagaagtt ttggtaacgt ggatagaagg aatggtttat 2340
tttgttttgg ggatattttc ggttattata gttcggtttt tttttgaggc ggcgttagtt 2400
tcggtttcgt ttcgtaggtc gttgttacgg aggcgttcgt tcgttgtttc gttttttggc 2460
ggtgtttgcg tgtgttcggg tgtgggtata tgtatttgtg tttgtaaatt agggcgggtt 2520
ttttaaatcg aaagttgcgc ggagttgggt ttttgggatt ttagggttgc gggtaaggga 2580
gatgggggat tttttcggga tttcgatttc gatttgagtt tagatttttc gcgttttggt 2640
tttttgtttc gtatttttag ggtcgtagtg ttaatttcgt gtttttcgaa gtggttcgtt 2700
atttgtttcg tcgaggggag tcgttttttg ggttaacgtt gcgggagttc ggacggttcg 2760
aagtatgttg gttaggcgta aggggagatc gatttatagg ggagagggtg ggtaagggga 2820
cggcggttgg gtttcggtat ttcgcgaggg gggttttgcg attttgattt agtagcggcg 2880
ttttcgggtt cgcgaggttt tgacgattag cgggcgtttt ttcgggaatt atatttgttt 2940
ttcgttggtt tcgagcgttt agagcgcgaa gcggcggagt acggcgaatt tacgattcgt 3000
gatgtttggt ttaatttttt gttttttatg ggtgcgggta ggaacggagt agcggttgat 3060
gttttttttt taattttaaa gttttataaa agttaaaata tatacgtaaa tttttattag 3120
cgtattttat aaatataaaa attaaagata atatatgaaa atataattcg attttttatt 3180
aaaacgtata gtttaaaaaa ataaaattta aatcgaagga aataaaatat atataatacg 3240
aaatttttta aggtaatata tatatagacg atatttattt atattaaatt aaaatatatt 3300
taaatatcgc ggagaaggaa aagttttata aacgatatat aaaaagatag tttataagga 3360
taaagtataa aattatataa aattaaaatt tattttaaag attaggattt atagagtaaa 3420
attcgttaaa attaatttta cgaaaaaata atacgtaaaa tattcgaaat aaaagaatat 3480
tttatggaaa aatgaaaatt tatagaaaat aaataaaaat aaaataagtt ttttaaatgg 3540
aaataataat aataataaaa tattaatatt tttcgttttt agttcggggt gaaatgttaa 3600
tttcgggagg gtttttgatt tttaggcgcg gataggtcgt tttttttagg cgttgttttt 3660
cggcgcgggc gaggcgggag ggcgggaagt ttgggttggg gttgggttgc ggagggaaag 3720
agtcgagtat tcggagatgg ttaaaaatta aatttcgggg tagggatacg ggttcgtagg 3780
ttgtagggag gaggcggcgt tggggaattg cgtagattat ttggtggggg ggtcgttcgg 3840
gaatttatag ttatattttc gattgtggcg aaatttgttt agtggatatg tgatgtttag 3900
ttgtcgtcgg gggttttttt tttttcgagt tttaggtttt tttttttagg ttgcgattta 3960
gtttcgtgat tatttttttt aaaaaataaa taatattttt gttgaggacg atttattttt 4020
taaaattgtt attgttcggc gggttaggag tttttttacg gagcgttttt tcgttttgga 4080
gaattttttt tttgcgatta tttcgttgta gggttttata ggtttttagt ggttaggttt 4140
agtagttgaa ttaatttaag gttggggagg ggaataggga ggggaggttt gagtttggtt 4200
cgaagagaga gggttggata tgttatattt ttgttcggtt tttgtggatt tgattttttt 4260

tttggaatta agttttgggg ttttgggatt ttataacgtt ttagggttcg agggtaatgc 4320
gattttattt atgggtcggg gtaaggtgtt tggaattttc gttaattttt agaaattatt 4380
gagatgtttt gttttgttcg ggtgttttaa agcggaagtt cggattttat ttttagtatt 4440
ttatagttta atttttatgt tttgggttag aatggaattt gatgtgggga ggggttttgg 4500
gattagaaga ttggatggga tttgggggtt ttagggattt ggcgttgttt tagggttagg 4560
ggtttagtaa tagttaggat tttaataaat tggggtgggg gttggatggt tgggaatttt 4620
ggggtcgggc gtcgttttag gggtgaatcg gagtaaatcg gaacgtcgtt tcggtgtttg 4680
gtaatattgt tagtttttgt aatttttttt aaaagtattt gtttggtcga gtagttaatt 4740
tttttaaaaa taaaaattcg gtttgcgttc gtttaagtag cggggtttgt atatggagat 4800
gttataggtt tcgcgtatag cgtagagggt cgcgattttt aagcgtatgt tttaatagaa 4860
ggtgcggttg gaagattcgg gttttcgggt ttcgtttcgg tttgtttttt ttcgtaggtg 4920
cgttggttag cgttcggcgt aggttgaagt tttttttttt tttttttaat ttttataaaa 4980
gtttggggcg gcgcggtagt agtattgttg ttttttcggt ttttcgtttt atttcggtcg 5040
ggtcgggttc gttacgtttg gcgcgttgag taggttcggt cgcgtagcgt ttattttttt 5100
ttcgtttcgg gtcggtagtg tggggcggcg cgttgggggc gcggcgtgtt tggggatatt 5160
ttgtgatgtt ggcgagaata ggatatgatt ttatatggcg agaagttttt tagtttttta 5220
attattttac ggtgttttcg gacgtttttt ttttatttaa aacgtttagt tttagtttag 5280
tgattagtta ttttagttcg gcgggggagc ggaaggtttg aattatttcg atttgtgagc 5340
ggtttttggt attaaaaaaa aaaaaaaaaa aaaaaaaaaa agaaaaaaaa aggtataaaa 5400
aagtggaaat ttttttttgt ttattttatt aagttttgaa aaattaaaat ggatttagag 5460
aaaaattatt cgatttttcg gattagtagg ataggtagga tcgcgatacg ggttttagat 5520
gtgtaaattt cgggatacgg ttaggttttg gtttttacgt agggtgattt tggaggagga 5580
gggggcgcgt cgtattttcg tagagattgt ttaattgcgg cgtgtttgtt atgttttttt 5640
atttatttgt ttatatattt ttgtattagt ttaaatattt gtgagtttgt ttgtgttttt 5700
ttttgttttt gaatgtttac gttggcgttt tttcgggggt ttgtaagtta gggtttgtgt 5760
gtttatataa gggatttagt gtgtttatta tagtatatgt atttttaaaa gatgtaaaaa 5820
tatatgagtt tgtgtttaaa agttgtttgt ttgtgtgtgt gtgtgtgtgt gtgtgtgtgt 5880
atttttatat tcgtggggta ttcgtgtgtg tttataagtt agtatttgtg tatatttttt 5940
tgggtattta aaatgtgtgt ttattatgag tgttatttat gtaattcgga aatatttgag 6000
tgtatattta ttatttaaag ggtttgtatt tatttgttat attttaagtg atttttggat 6060
gtgagaaaga agtggttata ttgatttttt tatttttgga tgttaacggt tgttcgatgt 6120
atatacgtta atgtttatat tgtgttttta ggtaaatttt tagacgtggg tttggggttt 6180
atggagattc gtggtgttta gtatgtgtat tgtaaggttt agtgtttatt ttattgggag 6240
ataggattag gaatgaaggt gtgaatttag ggtatgtagt tttttggtgt gtagtgagta 6300
ttgtttaggt tattatttgt ataaataggt tttttgtggt aggtataatt ttgtagatga 6360
tagcggatat ttgtagggtt agttgtgata tttttaggga tttttagttt atatttattt 6420
ttatttagat aagtttttga tattggcgtg tgtatttgtg tgtatatata gatatatata 6480
tattgtgttt ttaggtttta ttttgtgggt gtcgtattag tggaggagag tatagtgggt 6540
ggatgtatgt gtatagtttg ggtatttagt tttagggttt gtatgcgtat aggttttttt 6600
ggtttaggtt tttatgtgtt ttttataggt ttgagaagta ggtagtagga tgtgtttatg 6660
tttgagagga gatagtaggg atagtaggtt tttatttgtt ttgaggtatg aattaggtat 6720
gggggggttt ttttatttat taggttattg tgtaattttt atataaatat ttttttggtt 6780
ttggtaagaa gttttttttt tttatttttg tgttttttga gaatttttat ttttttttta 6840
gttttaaata taagtttaag agtaaaaagg ataaatttag attttaagtt taggataatt 6900
tttggggtat aattgtgttt ttaaaattgt ggttggagag aagtgagggt agagatttga 6960
ggtagaagag tgtttttagg atgagggttt aggaaaagtt atttggaaat tttttttttt 7020
tatttttaat ttttagaagg tttttttttt tggtagtggg ttttttagta gatggatttt 7080
tagagagatt tgagtttgga agtgggtgtt gtgtatgtgt ggggaatgag gcggggatgt 7140
gtgagtgtta tggagagggt ttgcgtagtt ttcgggttgg ttttttcgat aagttgtgta 7200
gaagaatgta atttatggcg gtcgatgggt tttttgatta gtttaattgt ttgaataatt 7260
atttaattaa taaagtattt tatttttttg tatagttgga ttgggtgggg tttggggtgg 7320
ggtgagatac gggagtttta ggttggttaa gttttaaaat ttagagagtt acgttttttt 7380
tttattgatg ggttggggaa gagttgtttg tttggaaaag ggcgatagtt taagtgagat 7440
tgtattttta tttgtttatt ttagtttggg ttagggttgg agaagagagg gaagttgaga 7500
agttgtttaa gttggtgttt aaagggttgt ttaggtttgg tttttatgag agtttttttt 7560
tttttttaat ttttgggtat tttatgggtt aatagatttt tttttttttt ttttagaatt 7620
atattttatt ttttatttta attttagttt ttttatttgt ttaatgggga gggagtgtgt 7680
ttttttggtt ttaagtttaa aataaggtta ggttaatatt aagtagggag ttatttaagg 7740
attttttttt ttaatgtttt ttttgttgta gggaaaggaa atttagaggt tcggagaggg 7800
gaaggatttt gttcgagatt ataaagtatg agtaagggag ggaataggaa tggaaggtga 7860
tttttttgtt tttagatttt ttgtttttgt tgggagaaga gatagatttt tttttttttt 7920
tatggatttt tttggagaaa aattattatt tggataagta gtagtttttt tttgttatgg 7980
gaatttttga gtttttagat tcgggtagat ttggatggat tgagtatttt tttattgggt 8040
attgatattt agttttatag ttttggaatg agtttggaat ggagtgatag atattaataa 8100
tttttattta tgaagtttta ttttatagag tttaagaagt tatattttag gatttgtttt 8160
ttgtattaaa taaacgggga agtagaggtt ttagaagggg ttgtttgttt taagtttttg 8220

ggagagagag tagttttagc gtttttggtt tgtaggatat gttggtaatg ataatatatt 8280
tcgattaagg tgattttata tgatttttga aaaatttggt taaattttta tttttttttt 8340
atttttttta agaatttttg aaaataggga taggttaagt agagtagatt tttgatttag 8400
gggtaggaat tatgtaggaa tttgttgtgt ttttatttgt tgtataattt taggttaatt 8460
tttttttttt tgatatttaa ttttttttat tttttaaaaa tgaagtttag atagaattta 8520
gtgggttgag atagttatgt tggtagtatt tttgattttt ttagaattga gtaattagtt 8580
tgagttttat tgggttttat tttgttagaa gtaagaatag ggaaatgggt tggatttggg 8640
ttgagagagt cgatgttagg ttattagaat attgtagtag tttttatatt ttgaagttac 8700
ggtaatgtag tgcgttggga ggcgtggaaa gggaagtcgg gagggggtgt gtttttagtg 8760
aaacgaggtt tttggttttt tatatattat ttttaatggg ttgtttttta gtattaaaat 8820
ttttaaaggt aattcggttt atataaattg agtttttttt tggatagttt tttttcgcgg 8880
ttttttgttt ttagcgttta gtatagggtt cggtttaaag ttttgttatt gggagaaaga 8940
aagcgtatta ttgtagatta gagtgaggtt cgattgcgtt gagagtcgcg gtgagagttg 9000
ttagtttcgt ttttttggat ttaggttgcg gttcgtgggc gtttagagat aggattgagg 9060
cgtagtagtt cggttttatt atggatttgt tgtgtagttt cgagttagtt acgtaatttt 9120
tttgggttgt cgtttggtga tttgtaaggt tgggttaggt ggttacggta tgaggttcgg 9180
tcgcgttttt tggggttgga attttttaga ggtcgttcgt tcgggcggtg atgaattatt 9240
tttatttaga gggaaatttt tttttttaag aacgtcgaga gttgcgggaa ggggttgttt 9300
tgggtgtata ggtaagacgg gttttgtgtg tgttacgtag tagaatattg tttcgaagtc 9360
gaatatttat tgtgtgaaag ggagaatcgg cgttattttt gttcgggtcg tgtttcgggg 9420
attttatgta agttttacgg gtattcgaag gaaaatttgc gtgtgcgggt atattttagt 9480
cgagtttttc gatttggtag tatcgttttt tttttattat ttcgagagcg gttttggaga 9540
ggcgtttttg agaaaggttt ttagttcgta gttttttttt tattttagat ttgttgtgtg 9600
attttggata agtcgtattt tgtttttggg tttcgggttt tgtattcgtg aaatggagaa 9660
agtatagttt cgaggttttt ttagttttaa gaatttagag aatttgggtt tgtaggtttg 9720
agtttagtaa gagaggttgg gggtgggggt gttattggtt gtatttttta gggcgtttcg 9780
tattttcgtt tttttagtag gtgtgtaagt cgttatgttg agtttgggtc gaggatttga 9840
attttggtta ttttcgattt attgggcgtt atcgggtgaa gttagtgtag gaagtttatt 9900
ttattttagt ttttttttgt tgttgcgatt tttttgtttt aaggtcgggg gtgaaagggg 9960
aagattcgga taggcggggt tttagataaa gaagagataa cgtcgttatt ttatttcggc 10020
gcgtattgta gttttttggg tttttagagt ttcgtttggg gtcgtttttc gtagttgtgt 10080
tttggtgggg ggtggggacg ggtgttagcg tcggaaattt gtttattggt tagggatagt 10140
tgggtgtgga atagggatag aaggtgggtt taagagtcga ttttagtgta ggagtttgag 10200
aagtagagta tgcgtagaga gaaggtagag gaagggaaat cgaggtagag aataaaataa 10260
gaggataatt taggaatttt tttatgatta gtttgtattt ttttttataa gtttagtttt 10320
ggagtaggta ttttaaagtt tttgttttgt ggatttatgt ttaatattag cggattatta 10380
tttatgaaat agaaattgag ttttcggatt atttttatga gttgttgggg taagggaatt 10440
tttagcgtcg tttttgagtt agggtttaga tggtttatag aattatagtt ttttgggtga 10500
agaatgattg ttatacgttt tttttttaag gtttagtttt tttgattgtt gttttagagt 10560
tttaaaattt ataaagaagc ggattcgtta gttaaagatt ttttgtgtag taagagagtt 10620
ttgttataaa tttatttatt ttgtttatat agggatagga attttttttg tagagggaag 10680
gttagttttt tattaattgg aagttgtcgt attgtagcgc gtttggtcgt tttgtttgat 10740
ttttgtagat agaatgcgtt tgtttattcg ttcgttttag ggatttttat tagttttgga 10800
cgcgatttgg tttattcggt ttttaagaag ttttgagtcg ggtgcggaat tttttcggtg 10860
gatagaagcg taatattagt ttttaaagtt tttggtagtt tttgatttga ggtaggaaag 10920
agggttagtt attttgtttg ggtaatttgg tttaaagttt tagttttttt atttaggtgc 10980
gttttggtgc gttttagtta ttaattttaa aggagtgtga gatatttttt tttttttttt 11040
aacgtttttg tttttcgtta atgtagtaat gagggttttg taggtggagt gagggaggag 11100
ggtggacgac gaggagttag tggtggttta gtaggtattt gataaaatta ggaaataaaa 11160
ttaggaaata ttttcgtgga aggagttttt agtaaaattt agttaatttt attttgttta 11220
ttttatagaa gaggaaagtg aggttagaat tggtagatat gtatatattt cgatgtatat 11280
aattcgattt tattacgagt ttgatggtat aggattttta ttgcgttttt tgtttttttt 11340
taatatatat tttttgtttt ttggtttttt tggagatttt ttttattttt attttttaat 11400
cgattgtttt taagttttga gagcgtttta ggattttagt atcgttgtgg ggaggtttag 11460
ttcgcgtggg attcgtagcg ggatttttta ggtattgcgt gggttttttt ttttttttta 11520
tagtcgcgat ttattaggtt ttagtttagc gcgtttgtta atgaacgtgg cgtcgtcgtt 11580
tgggtttcgt tttttatttt tttttattgt ttaggtgttc gttaattagg cggagtttgt 11640
gggttgttta gttaatgggg cggtgtttcg ggttagggtc ggttgacgtt attttgattg 11700
gtttggtgga gcgtgattgg taggttagtt ttcgtttcgt tttcgttcgg agggagttga 11760
gtttcgggcg gcgttgttta tacgtagcgg gttttgaaag cggtttcggg gcggcgcggt 11820
ggcgtattgt gcgcgtcgag taggcggagg gttagcgacg gcggcggcgg cggtaacgta 11880
ggtagttaga gtcgattgcg gttttatttt agtttcggta gtttcggcgg ttttggtggt 11940
tgggattttt tgtttttttt tttgtttttt attttacgtt tggggtaggt acggcggtcg 12000
tttcggaaga ggtagcggaa agatagtgga attagattta cgagggaggg cggcggggtt 12060
cgaagagttg gttggtacga ggtagatatt tcggcgagga agagttcgtc gttggtgttt 12120
tcgtttttgg ttttagcgat gggcgagtag cgggggtcgc gggaattttg aaggtagcgg 12180

gttttagtgt tggagggttg tacgttttcg gggatttcgg agtcgcggtt ttagcgttag 12240
gggcggaagt atttttaaat tcgttaggat ttcgcgttgt cggagaggaa gttttttgta 12300
atttcgttcg agacgtcgga gagttagttg gagagaggcg gagggggtaa gttcgagttt 12360
tagagattcg gagaagtaga gaaagaaagg atttggcgga gatcgagggg tcgaggaggg 12420
attttagaag tatcgaggtc gcggaattag tttggtagtt ttcgggtttt ttcgtttttt 12480
gagtgaagtt aagggtcgat tgtttcggcg gtttttttaa agttgttttt ttttggggtc 12540
gggggttgtt ggttgtttcg gaggcgggtt tttacgttat gttttcgaag tcgcgggcgt 12600
tttggtcgta gttatttcgg gttgggttcg gagttgttgt ttgagttagt tagggaggga 12660
agttagtcgg agttgggtat tcgtttttag ttcgggtgcg ttagtattat ttttcgtttt 12720
ttcgcgtagt ttcgggcggt ttttagtagg gaggttttga gagagatttc gaagttttag 12780
tagtgttttt tgagtttagt tcgtatagtg tttggtttgg tggcgaggtt ttttattcgt 12840
tttgggtagt tttttggcgt cgggaggcgg cggcgtgggt cggtttgtag tttggagcgt 12900
ttcgatggaa atatattgta agtacgattc gtttgtattt atgtatagta agtaggcggc 12960
gagttcgttt tttttatttt ttcgtcggga tcgggttggg ggtgtcggat cgtgggttag 13020
gtattaaggt cggaaggatt tcggattttt gtagtcgcgt agttaatttt gtaaagtgta 13080
gggattttag gtaggggatt tgaaataacg tatttggtcg ttcgggttga ggtatttggg 13140
aatttcgttt tcggtaacgc gtagtttggg tagcgagttg tagtttttta aggtttcggc 13200
ggcgatattt tagttttttc ggagaatttt ggtttggatt cgtagagggt ttcgggttag 13260
gttagttgga gggtagtaag ttgtagggag aggttagtta gagttttaga tagcgtttta 13320
tatttttatt tcgatggcgt tttttttagt tatggggttg atttagattg gcgtttggag 13380
ggatagtggg gttttgattc gtgtttgtat gtgtggagga agaggttgag agttaagcga 13440
ggatatcggg gttagggttt taaaggcgtt ttggagttga ggagggggtt ttgaaaaatt 13500
aaggaagtag cggtttttga gtttagtttt tgtttaggag tcggttagtt tatatagaga 13560
tttttaaaga ttttattggg tttttttttg taggacgttt ttttttatgg tttttttttt 13620
ttaaaaaaag aggataagaa acgaaaaaga aagaaagaaa gaggaagtta attagttatt 13680
acgtaggtat tttcgttttt gaaagacgta gcgtgtgtag gagacgggaa gggttattta 13740
gggcgagggt aaggtcggtt tggttgtttg gttcgtaggg attttgttaa tttatttatt 13800
attatagttt tttgtgagat gagcgttttt tttacggtga gttaacgtcg gattagattc 13860
ggagatttta aggaaatttt aagtggaacg aatcgcgttt tttttttgtt tgttgtagag 13920
ttaggttgga ggaaggtgat aggtttcggt agtttcggtt cgggtgtcgt tcggggtttt 13980
tgcgggtttt aggttaggga gttgttttcg ggttaggagt cgtttttatg tttggttttt 14040
ttatttcgtt tttttttatt ttaatagttt gggatttagt ttttttagtc gtaggttttt 14100
tatttaataa aggtagtaga gattcggttg gagtgaaggt gagtttttcg gggcgagtat 14160
gttcgtgttt gttacgtagt gttgagtggt ttggtaagta ggggaaagtt tgtgtaggta 14220
gttagggttg gttgcgggta ggttagtttt tttttgtgtt aattttttga gtttgaagta 14280
atttattaaa gcggtgggga gttttgtgta gaggagtttt ttgttttggt tttagtttaa 14340
acgatttttt aaggtttggg gtttgcgttt aatcggtttt tagtttgtgg tgattttagt 14400
gttttttata gttttttttg tttcggattt tagtttggtt ttggggagga tttgattttg 14460
taatgtaagt ttaaaataaa ttagttaggg taatgttttg agaggtgagt aagtttttgg 14520
tttagttaag gagtaattgt ggggatagat ggggagtatt agtttttgta tttttttttt 14580
tattggtgga aatttatagg tttttattgt gttttttcgt ttagcgttcg gtggtttaga 14640
ggttgtgttt tagtttttgg ggagatagta ttagatttgg tggtttttcg gttgagtatt 14700
agtgtggaga aggcggtttt ttttaggtgt aggttagtta atcgttaatg tttgtgtttt 14760
tttgttcggt ttttaatcgt ttttttcgtg ggaagtgttt ttttttattt ttaatttttt 14820
tggtagtata gtgggggttg ggttttagtt agtttagttt ttagagtagg tgtaagattt 14880
tggttttttt ggaagtattt tcggttttga ggtttaaagt tgtttttttg tataatgtag 14940
gtttgttttg gggttttaat ttgttagtaa tggggagtgg ggggtatagt agattttgga 15000
aggtggttga ggtagtagag gtttagatta agttttattt cgttattttt tttttggttt 15060
agttagtttt ttttttttgt tatcgttttt tgttttcgtt tttttattat tgtttttttt 15120
tattattgtt ttagagtcgg atagtttttt tggagaggag tagggatttt taggaaatgg 15180
gtttttgagt ttggaggagt ttttatttat tattttaggt ttattatttt tattagtacg 15240
ataatttttt gttttttttt tgagcggttt ttttaggtat ttttattagt tataaaatat 15300
ttatagttag ggttttattt gacggtggtt taaattttta gttttattta ttttgtagaa 15360
tatgatattg aggttagaga gataaaagga ttgatttaag aagtttatat tggtatagtt 15420
ggagttggtt tttgggtttt tttgattatt tgataaaggt attaaaatga agttttagag 15480
ggtttataga aggggagagg gagggattta gattttagag agagggtagt gggtatatat 15540
atatgtaaag tttttttttt gtttagttta attatgtata tgtttattgt ttgatttttt 15600
atatgtatta atattattag ggagattttt tttttgtttt ttttttgtgt ttttgtttgt 15660
ttgtttgtgt atttttttat tttttttggt gtatttagga aaaattttaa tgattttgtg 15720
ggggttttgg agtttaattt gtggttgagt tagtaaagtt ttttttttta aaattttatg 15780
tttagttatg gtttttgggg gtattgggag ggtttgtggt gggtagtatg attttattag 15840
ggtttgagga gtatttgtgg aggttagttt tttttatatt agagttataa aggttttagg 15900
ggtttttgta atttagtttt ttttttttta ttttttttta aatattgttt agtttgtttt 15960
gttttgtggt ttgttttttt tttgtatttg tgggtttgtt ttttggtttt tatttgattt 16020
gtttgttttt gtttttgtgt tagttttttt tttacgggat gagtttttat ttaattggag 16080
tttataggaa gtataggttg ggatttgggt tttttaagaa gatgtagggt tagagttggt 16140

attttagaaa ataaaggttt tttgtttaga gaagtttttt gttttttaga agtttttttt 16200
tgggtttttt ttattttttt tttttattgt ttttcgttgt gtgttatttt cgtttttttt 16260
tttttggttt attttagttt ttttattttt tttttttttt ttttgtttgt ttaatttgtt 16320
gttttttttt taggtttttt tttttttggt tgtttgtttt atttagtttt tattttttat 16380
atttttttta atatattttt tttttttttt tttttttttt tgttttgttt cgttttattt 16440
agattttgga ttttattttt tattaatttt agagtaagtg atttttttta tattatagtt 16500
gtaaattttt ttattattat tttttaagag ttttattggg taggattgtg atggtggaga 16560
aggttaaggg gttttgggga ttttttaagg aagttgagga aggtaatagg tttttaggtt 16620
attttaagtt tgttgttagt tgtggattta tagatgagat tgtgaaagag tattattatt 16680
ttagtttatg tggttaaggg tagagggttt ggggatttgt ttttattttg agagatttgg 16740
ttttaggaga tttttagggt ttttttatga tataaaatag tttttagtgt ttataaatgt 16800
tttgtaggat tgtttttgga atttaagttt tttgaaggtt gttattgtgt tttgggtaga 16860
aagttttaat tgttagtgat atagagaata ggttattgta gatggattat aatatttgga 16920
tttttaggtt atattaaaag tgtgttgggt gtttaagttt tttttgtttg ttttttgttt 16980
ttttagatga gtttatatta attttgaatt tatttttttt tatttaaatt tagtttgttt 17040
ttgaaagatt tgtataattt ttttaaatag gtgtttttag ttttagaata aagttttttg 17100
aaggtttttg gttagggtgg ttgtagtttt tttgttttag aaaaattatg taattatgtt 17160
tttaattgta aaattttatt tggaatggtt tttttttttt ttatgttttt tagaataatt 17220
aaaattgaat aaagattatt ttgaaaatta gaaatttagt ttagaattgt aattaagtat 17280
tttagttatt tataaaattg tgtttttttt tgaaataagt ttgagtttgg acgtttttgg 17340
gaaatattta tgggaaattt gagtggattg tttggatagt ttttaggaaa tatattatat 17400
tatgggggtt tttgtttttt ttattttaga tgaggtttat gaatttgggt tgtagtttag 17460
tattggggag aaatagttga gatgaggtgg ttattttata ttttagagtt gagtatatat 17520
gtgtgtgaga tttttgaggt tttttttttg tacggtttag ttttagttta gttttggttt 17580
aggaggagtt tttttttttt ttgtttttga gttgttagta atattaataa atgttttgat 17640
ttggttttgg gttttagttt ttagtaacgt tagtgagtga ttaattaata gttataaggt 17700
taatgatgtt gttgtatcgg attattttgt gtttattggt ttggtttttt atttatttga 17760
gtttttgtgt ttttttagat ggattttgat gtaagagaag ggtggaggag gtattttttc 17820
gggttttttt tatttaaaat ttattttagg atttttgtaa aatttgggat ttagaggttt 17880
ttgtaggttt ttttattttt tttggattaa aataaagtta tatgttaatt gtaaaatatt 17940
gtaaaaattt tagggaaatt tttttataat aaattttcgt gtagtggagt ggaatgtgtt 18000
gtgtgtagaa gatgttatga tttatttttt ttgtgtgttt tgagaaggta tagtggttaa 18060
gtttgtatgt taattttatt tttttaaagg gagggtttta aataaggtaa tggttgtttt 18120
ttttttttag tattttgtag gtgttgttaa atttttaaaa aattttttag tttttgaatt 18180
agattgttga ttaaaaataa aattttttga gatataagtt tattggagtt gtttttttta 18240
tttttaaaga tatttgttaa aaggaaacga gttttatttt tttttttttt aaatatgatg 18300
tattttaatg tatatttttt ttttaggatt ttttttagat tttagtatta tataataagt 18360
tgtgtttatt tcgtttatat tgagattttt atatatttta ttgtttagaa aaattacggg 18420
gaaaatgtta ataatagtat ggggatgttt gttgttaata gtttaataga ttggttgtag 18480
atggattaag gttaagatta attttaaaaa gtaatttggg gatttcgtgt agtttgtacg 18540
attggatttt aggcgcgaga tatttagttt gtcggttatt tgtgattaga ggggttttgg 18600
gtttttatag cggtgttttt tttatgtgat tggttttaat tatttttttt tttttttttt 18660
gtttttgatt tgttttagg 18679

<210> 318
<211> 18679
<212> DNA
<213> Artificial Sequence

<220>
<223> chemically treated genomic DNA (Homo sapiens)

<400> 318
   tttgaaatag attagaaata aaaggaaagg gaaagggtag ttagaattag ttatatagga 60
   gaagtatcgt tgtgaggatt tagagttttt ttgattataa atggtcggta ggttggatgt 120
   ttcgcgtttg gagtttaatc gtgtaaatta tacgaagttt ttagattgtt ttttaaaatt 180
   aattttagtt ttggtttatt tgtagttagt ttgttgagtt attgatagta aatattttta 240
   tattattatt agtatttttt tcgtaatttt tttaaataat gaaatgtgtg aagattttaa 300
   tgtaaacgaa atgggtataa tttattatgt gatgttgagg tttaaggaag attttgggga 360
   gaaaatatgt attaaaatat attatattta aagaggaaaa aaataaaatt cgtttttttt 420
   taataaatgt ttttaaaagt gaaaagaata attttaataa atttatattt tagggaattt 480
   tgtttttaat tagtaattta atttaagggt taaggaattt tttaaaaatt taatagtatt 540
   tgtagagtat tgagaaaaaa aaataattat tgttttgttt aaaatttttt ttttaagaag 600
   gtgaggttga tatgtagatt tgattattgt atttttttaa agtatataaa agaaatgggt 660
   tatagtattt tttatatata gtatatttta ttttattgta cggggatttg ttataaagga 720
   gtttttttgg aatttttata atgttttgta attgatatat agttttattt tggtttaggg 780
   aaaataaaga aatttataaa agtttttgga ttttaggttt tataagggtt ttgggataga 840
   ttttaagtaa aaggagttcg agaaagtgtt tttttatttt tttttttata ttaaggttta 900
   tttggaagga tataaggatt taagtaaatg aagagttaaa ttagtaaata tagagtgatt 960
   cgatgtagta atattattga ttttatagtt gttaattagt tatttattga cgttgttgaa 1020
   gattggagtt taaagttaag ttaaagtatt tattgatgtt gttggtagtt tagaggtaaa 1080
   ggaaggggaa attttttttg aattaagatt ggattaggat tggatcgtat aagggaaaga 1140
   ttttaaaggt tttatatata tatatattta attttaaaat atgaaatagt tattttattt 1200
   tagttatttt tttttagtat taaattataa tttaggttta tgaattttat ttgaaataaa 1260
   gggagtaaaa atttttataa tataatgtat tttttaaaaa ttgtttaagt aatttattta 1320
   agttttttat gaatattttt taaaaacgtt taggtttagg tttattttaa ggaaaaatat 1380
   aattttatag ataattgaag tatttagttg tagttttaag ttggattttt gatttttaaa 1440
   atgattttta tttagttttg attgttttaa aagatataag aaaaaggaaa attattttaa 1500
   ataggatttt gtagttagaa atataattgt atagtttttt taaaatagaa agattgtagt 1560
   tattttggtt aagagttttt agaaggtttt gttttggggt tgggaatatt tatttaaaaa 1620
   ggttgtatag attttttaag agtaggttgg atttaagtaa aagaaaataa atttaaaatt 1680
   ggtataagtt tatttgggaa aataaagggt aggtaaagga agtttaaata tttagtatat 1740
   ttttgatatg atttgggagt ttaggtatta tggtttattt gtaatagttt gttttttatg 1800
   ttattgataa ttgagatttt ttgtttaaaa tatagtggta atttttagag gatttaaatt 1860
   ttaaaagtag ttttgtagga tatttatgag tattgggggt tgttttgtat tatgggaaaa 1920
   ttttaaaggt tttttagagt taggtttttt aaggtgagag taggttttta aattttttat 1980
   ttttgattat ataggttgag atagtaatgt ttttttataa ttttatttat aggtttatag 2040
   ttgataatag atttggggtg atttgggagt ttgttatttt ttttaatttt tttgaaaggt 2100
   ttttaagatt ttttggtttt ttttattatt ataattttat ttaatagagt ttttagaggg 2160
   tggtggtgag aaggtttata attatggtgt gaggaaggtt atttattttg gaattggtaa 2220
   gaagtgaagt ttaaagtttg aatgaggcga ggtaggatag ggaaagaaaa gaggaaaaaa 2280
   gaggtgtgtt aagagaagtg taaagagtgg gaattgaatg agatagataa ttaggagaga 2340
   gagagtttgg aaaggaagta gtaggttagg taggtagagg agagggaaaa ggaatggaga 2400
   agttagaata ggttaagaaa agaaagacga aggtggtata taacgggagg taataagaag 2460
   agaaaatgga ggaagtttag gaaggggttt ttggaggata ggggattttt ttagatagag 2520
   gatttttgtt ttttggggtg ttagttttgg ttttgtattt ttttggagaa tttaagtttt 2580
   agtttgtatt ttttatggat tttagttagg tgaggattta tttcgtgaaa gggggattga 2640
   tatagagata gaggtaaata aattaggtga agattaggaa atagatttat agatataaga 2700
   aaaagataga ttataggata gggtagattg ggtagtgttt ggaggggagt gaggagagaa 2760
   agattgaatt gtagaggttt ttgaggtttt tgtgattttg gtatggaaag ggttggtttt 2820
   tatagatgtt ttttaggttt tgataaggtt atgttattta ttataggttt ttttaatatt 2880
   tttaaagatt atggttgagt ataggatttt ggggaaaggg attttgttga tttagttata 2940
   gattgagttt taggattttt ataagattat taaaattttt tttagatgta ttagaaagaa 3000
   tgaaggggta tatagatagg taaatagggg tatagggaaa gggtagggaa gaagtttttt 3060
   taatggtatt gatatatgtg gagagttagg tagtagatat atatatggtt agattagata 3120
   gagagagagt tttgtatatg tgtatattta ttgttttttt tttggaattt gagttttttt 3180
   tttttttttt ttgtgaattt tttgaagttt tattttaatg tttttgttag atggttaggg 3240
   agatttaaga attagtttta gttgtgttaa tgtgaatttt ttgagttagt ttttttgttt 3300
   ttttgatttt aatattatat tttgtaaaat gggtgaagtt gaaggtttag gttatcgtta 3360
   ggtaggattt tggttatgag tgttttataa ttggtaaaga tatttagggg aatcgtttag 3420
   gaagagagta ggaggttatc gtgttgatga aaatgatgag tttggaatag tgggtgggga 3480
   tttttttaaa tttagaagtt tattttttgg aagtttttgt ttttttttaa agaagttatt 3540
   cggttttgaa gtagtggtgg ggggaagtag tggtaggggg acggaggtag ggagcggtaa 3600
   taggaagaag aaattgattg ggttaggagg aaggtgacga agtgggattt gatttgagtt 3660
   tttattattt tagttatttt ttaggattta ttgtattttt tattttttat tgttagtaag 3720
   ttggggtttt agagtaggtt tatattgtgt aagaaaatag ttttgggttt tagaatcgag 3780
   agtgttttta gagaagttaa ggttttgtat ttgttttgga agttagattg gttggggttt 3840
   agtttttatt gtgttgttag ggagattgaa aatgggaggg agtatttttt acggaggggg 3900
   cgattgaagg tcggataagg aggtatagat attagcggtt ggttaatttg tatttgaaag 3960
   aagtcgtttt ttttatattg atgtttagtc gagagattat taggtttgat gttatttttt 4020
   taagggttag ggtataattt ttgggttatc gagcgttggg cggaaggata tagtgagggt 4080
   ttgtggattt ttattagtga ggagagaagt gtaggggttg gtgtttttta tttgttttta 4140
   tagttgtttt ttggttgagt taagggtttg tttatttttt agagtattgt tttaattggt 4200
   ttgttttggg tttatattgt aagattaggt tttttttaga gttaggttgg agttcgaggt 4260
   agaaaaggtt gtggagggta ttggggttat tatagattgg aaatcggttg ggcgtaggtt 4320
   ttaaattttg aggaatcgtt tgggttggga ttagaatagg gggttttttt gtatagagtt 4380
   ttttatcgtt ttggtggatt attttagatt tagaaaattg atataaagag aaattgattt 4440
   gttcgtagtt agttttggtt gtttatataa gttttttttt gtttgttagg ttatttagta 4500
   ttgcgtggta gatacggata tgttcgtttc gggaagttta tttttatttt agtcgggttt 4560
   ttgttgtttt tgttaaatag gggatttgcg gttaggaaag ttggatttta ggttgttggg 4620
   atggggggga gcggggtggg aggattaggt atggggacgg tttttagttc gggagtaatt 4680
   ttttgatttg aagttcgtag agatttcgag cggtattcga gtcgaggttg tcgaagtttg 4740
   ttattttttt ttagtttggt tttgtagtaa atagaaagga aacgcgattc gttttatttg 4800
   gaattttttt gaaatttcgg aatttaattc ggcgttaatt tatcgtgaga ggagcgttta 4860
   ttttatagga ggttgtggta atgggtgaat tggtaggatt tttgcgggtt aggtagttag 4920
   gtcggttttg ttttcgtttt aaatgatttt tttcgttttt tatatacgtt gcgtttttta 4980
   gaggcggggg tgtttgcgtg gtaattaatt ggtttttttt tttttttttt ttttttcgtt 5040
   ttttattttt tttttttaaa ggggagaagt tatgagaaaa ggcgttttgt agagaaggat 5100
   ttaatggggt ttttaagggt ttttgtatga attggtcggt ttttaagtag aagttgaatt 5160
   tagaaatcgt tatttttttg attttttaaa gttttttttt taattttagg acgtttttgg 5220
   agtttagttt tcggtgtttt cgtttagttt ttaatttttt tttttatata tatagatacg 5280
   ggttagggtt ttattatttt tttaagcgtt agtttggatt agttttatgg ttgaaaaagg 5340
   cgttatcgga gtaggggtgt ggggcgttgt ttgagatttt gattggtttt tttttataat 5400
   ttattgtttt ttaattggtt tggttcggag ttttttacgg gtttaagtta gggtttttcg 5460
   aaagagttga aatgtcgtcg tcggagtttt gaaaggttgt agttcgttgt ttaagttacg 5520
   cgttgtcgga ggcgggattt ttaggtgttt tagttcgggc ggttaagtgc gttgttttag 5580
   gttttttgtt tgggattttt gtattttgta aagttagttg cgcggttgta gaggttcgag 5640
   attttttcgg ttttagtatt tgatttacgg ttcggtattt ttaattcggt ttcggcggga 5700
   gagtgagaga agcgagttcg tcgtttattt attatgtatg gatgtaaacg ggtcgtgttt 5760
   atagtgtatt tttatcgggg cgttttagat tgtaggtcgg tttacgtcgt cgtttttcgg 5820
   cgttaagggg ttgtttaggg cggataggga gtttcgttat taggttaggt attgtgcgag 5880
   ttgggtttag aaaatattgt tggagtttcg gggttttttt tagagttttt ttgttggaga 5940
   tcgttcggag ttgcgcggag aggcgggaaa tggtgttagc gtattcgggt taggagcggg 6000
   tgtttaattt cggttggttt ttttttttgg ttggtttaag tagtagtttc gggtttagtt 6060
   cggggtagtt gcggttaagg cgttcgcggt ttcgggggta tagcgtaggg gttcgttttc 6120
   gggatagtta gtagttttcg gttttaggaa ggagtagttt tgaggaggtc gtcggaataa 6180
   tcggtttttg atttatttta gggggcggag agattcgggg gttgttaggt tggtttcgcg 6240
   gtttcgatgt ttttgaggtt ttttttcgat ttttcggttt tcgttagatt tttttttttt 6300
   ttgttttttc gagttttggg ggttcggatt tgtttttttc gttttttttt aattggtttt 6360
   tcgacgtttc ggacgaagtt gtaaagaatt tttttttcgg tagcgcgggg ttttggcggg 6420
   tttggagatg ttttcgtttt tgacgttggg gtcgcgattt cggagttttc gggagcgtgt 6480
   agttttttaa tattgagatt cgttgttttt aaagttttcg cggttttcgt tgttcgttta 6540
   tcgttgggat taggaacggg gatattagcg acggattttt tttcgtcggg gtgtttattt 6600
   cgtgttaatt aatttttcga gtttcgtcgt tttttttcgt gggtttgatt ttattgtttt 6660
   ttcgttgttt ttttcgggac ggtcgtcgtg tttgttttag gcgtggagtg aggaatagag 6720
   gagagagtag agggttttag ttattaaggt cgtcggggtt gtcggggttg agatggagtc 6780
   gtagtcggtt ttgattgttt gcgttgtcgt cgtcgtcgtc gtcgttagtt tttcgtttgt 6840
   tcggcgcgta tagtgcgtta tcgcgtcgtt tcggagtcgt ttttaggatt cgttgcgtgt 6900
   ggatagcgtc gttcggggtt tagttttttt cgggcggggg cggggcgggg gttaatttgt 6960
   taattacgtt ttattaagtt aattagagtg gcgttagtcg gttttggttc gaaatatcgt 7020
   tttattggtt aagtagttta taggtttcgt ttgattagcg gatatttggg taatgggaga 7080
   gaatgggggg cggggtttag gcggcggcgt tacgtttatt gataaacgcg ttggattaag 7140
   gtttagtgga tcgcggttgt ggggagggga aaggggttta cgtagtgttt ggagagtttc 7200
   gttgcgggtt ttacgcgggt tggatttttt tatagcgatg ttgggatttt gggacgtttt 7260
   taaggtttga gagtagtcga ttgaagggtg agaatggggg aagtttttaa aaaaattaaa 7320
   aaataaaaaa tgtatgttgg aggaagataa ggagcgtagt agggattttg tattattagg 7380
   ttcgtgatgg gatcggattg tgtgtatcgg agtgtgtgta tatttgttag ttttgatttt 7440
   attttttttt tttgtaaaat gggtagagtg gggttaatta gattttattg aaggtttttt 7500
   ttacggaaat attttttaat tttatttttt aattttatta agtatttatt aaattattat 7560
   tggtttttcg tcgtttattt ttttttttta ttttatttgt agggttttta ttgttgtatt 7620
   aacgaaaggt agagacgttg ggggaagggg gagaatattt tatatttttt taaagttagt 7680
   aattggggcg tattagggcg tatttgggtg gaggggttaa ggttttgggt taggttgttt 7740
   aagtagagtg gttggttttt ttttttgttt tagattagga attgttagag gttttgggag 7800
   ttggtgttgc gtttttgttt atcggagaaa tttcgtattc ggtttaaggt tttttgagaa 7860
   tcgaatgagt taagtcgcgt ttagggttaa taagggtttt tgaagcgggc gggtggatag 7920
   gcgtatttta tttgtaaggg ttaagtaaag cggttaggcg cgttgtaatg cggtagtttt 7980
   tagttgatgg gaaattgatt ttttttttat aggggaagtt tttgttttta tgtagataaa 8040
   atggatggat ttgtagtaga atttttttgt tatataaggg atttttgatt agcgagttcg 8100
   tttttttgtg ggttttaagg ttttgagata ataattaaag aaattggatt ttgaaaagga 8160
   agcgtgtgat agttattttt tatttaagaa gttatagttt tgtgagttat ttgggttttg 8220
   gtttaagaac gacgttgagg atttttttgt tttagtaatt tatgggagtg gttcgaagat 8280
   ttaattttta ttttatgagt gatgattcgt taatattaaa tataaattta taaaataagg 8340
   gttttgagat gtttgtttta ggattgggtt tgtaaggaag agtataggtt gattatgaga 8400
   ggatttttgg gttatttttt tgttttattt tttgtttcgg tttttttttt tttgtttttt 8460
   ttttacgtat gttttatttt ttaaattttt atattggggt cgatttttgg gtttattttt 8520
   tgtttttgtt ttatatttag ttgtttttga ttagtagata agttttcggc gttgatattc 8580
   gtttttattt tttattaggg tatagttgcg gaaaacggtt ttaggcggga ttttggggat 8640
   ttaggaggtt gtagtgcgcg tcggggtgga gtggcggcgt tatttttttt ttgtttggaa 8700
   tttcgtttgt tcggattttt tttttttatt ttcggttttg gagtaggaag atcgtaatag 8760
   tagagaggga ttgggatgga atagattttt tgtattggtt ttattcgatg gcgtttagtg 8820
   ggtcggaaat aattagaatt tagattttcg gtttagattt aatatggcgg tttgtatatt 8880
   tattggagag gcgggagtgc gggacgtttt gagggatgta gttagtgata tttttatttt 8940
   tagttttttt tgttgagttt aggtttataa atttaggttt tttagatttt tagagttaaa 9000
   agggtttcga agttatgttt tttttatttt acgggtgtag aattcgaggt ttagaggtag 9060
   gatacgattt gtttaaagtt atatagtaag tttggggtag aagaaaggtt gcgggttgga 9120
   aatttttttt aaaagcgttt ttttagagtc gttttcggga tggtggggga ggggcgatgt 9180
   tgttaaatcg aagaattcgg ttggaatatg ttcgtatacg taggtttttt ttcggatatt 9240
   cgtgaggttt gtatagaatt ttcggggtac ggttcgagta ggagtagcgt cgattttttt 9300
   ttttatatag taggtgttcg gtttcgggat agtgttttgt tgcgtggtat atataggatt 9360
   cgttttattt gtgtatttag agtagttttt tttcgtaatt ttcggcgttt ttgggggaag 9420
   gagttttttt ttggataggg gtgatttatt atcgttcgag cgggcggttt ttgggaagtt 9480
   ttaattttaa aaggcgcgat cggattttat atcgtgatta tttagtttag ttttataggt 9540
   tattaaacga tagtttagag aggttacgtg attagttcga ggttgtatag taagtttatg 9600
   gtggagtcgg gttattgcgt tttagttttg tttttgaacg tttacgagtc gtagtttggg 9660
   tttaggagag cgagattagt agtttttatc gcggttttta acgtagtcgg gttttatttt 9720
   gatttatagt gatgcgtttt ttttttttta gtaatagggt tttgggtcgg gttttgtgtt 9780
   aggcgttggg ggtaggagat cgcgaaagga aattatttag gaagaaattt aatttatgtg 9840
   aatcgaattg tttttgaaag ttttagtatt gaaagatagt ttattgagaa taatgtgtga 9900
   gaagttagag atttcgtttt attggggata tatttttttt cggttttttt ttttacgttt 9960
   tttagcgtat tgtattatcg tggttttagg atgtgggggt tgttatagtg ttttagtagt 10020
   ttgatatcga tttttttaat ttaagtttag tttatttttt tgtttttgtt tttgataaag 10080
   tgaggtttaa tagggtttag attggttgtt tagttttgga aagattaggg atgttgttag 10140
   tatggttgtt ttaatttatt gagttttgtt taaattttat ttttaaaaaa taaaaaaaat 10200
   taagtattag agggaaagaa attgatttga aattatatag taagtggagg tatagtagat 10260
   ttttgtatga tttttgtttt tggattagga gtttgtttta tttgatttgt ttttgttttt 10320
   aggagttttt gaagaggatg gagaaaaaat gagaatttaa ttaggttttt taagaattat 10380
   gtaaaattat tttagtcggg gtgtgttatt attattagta tgttttgtag gttaggggcg 10440
   ttgggattat tttttttttt aaagatttga gataggtagt tttttttaaa gtttttgttt 10500
   tttcgtttgt ttaatataga gaataaattt tgagatgtga ttttttaggt tttgtgagat 10560
   gaagttttat ggatgggggt tattaatatt tattatttta ttttaggttt attttagaat 10620
   tgtgagattg gatgttaatg tttagtggga aagtatttaa tttatttagg tttgttcgga 10680
   tttaaggatt taagggtttt tatggtaagg agagattatt atttgtttaa gtagtagttt 10740
   ttttttaagg gggtttatgg agagaggggg gaatttgttt ttttttttag taggaatagg 10800
   aggtttaagg gtagagaggt tattttttat ttttattttt ttttttattt atgttttatg 10860
   atttcgaata agtttttttt ttttttcgaa tttttgagtt tttttttttt atagtaagga 10920
   agatattaga ggagaaggtt tttaagtggt tttttgttta gtgttgattt gattttattt 10980
   tgggtttaga gttagaagga tatatttttt ttttattggg tagataggga aattgaggtt 11040
   ggggtaagaa atggaatatg gttttgaagg aagaaaaagg aagtttattg gtttatgaaa 11100
   tatttagaag ttgggagaag gaagaagttt ttataggaat tagatttgga taattttttg 11160
   agtattagtt tggatagttt tttagttttt tttttttttt tagttttgat ttaggttgga 11220
   gtggatagat ggaagtgtag ttttatttga attgtcgttt ttttttaggt aggtagtttt 11280
   tttttaattt attagtgagg agggggcgtg gttttttaga ttttaaggtt tggttagttt 11340
   agggttttcg tattttattt tattttaagt tttatttagt ttagttatgt agaggaataa 11400
   aatgttttat tgattaaatg attatttagg taattgaatt aattaaagag tttatcgatc 11460
   gttatgaatt atattttttt atatagtttg tcgggagagt tagttcgagg attgcgtaga 11520
   ttttttttat gatatttata tattttcgtt ttatttttta tatatatata atatttattt 11580
   ttaggtttag gtttttttga aagtttattt gttgagaagt ttattgttag ggagaagggt 11640
   tttttgagaa ttgaggatgg agaagaggag tttttaggta attttttttg agtttttatt 11700
   ttgggagtat ttttttgttt tagatttttg tttttatttt tttttagtta taattttgga 11760
   aatatagtta tgttttagaa gttgttttgg gtttggggtt taagtttatt ttttttgttt 11820
   ttaggtttgt atttgaggtt ggaggagaga tgggggtttt tagagggtat agagatgggg 11880
   gaagggggtt ttttgttagg gttaagagag tgtttgtgtg ggagttatat agtggtttag 11940
   tgagtgaaag aggtttttta tatttgattt atgttttagg gtagatggag gtttattgtt 12000
   tttgttattt ttttttagat ataaatatat tttgttattt attttttagg tttgtgggag 12060
   gtatatgggg atttaagtta gggaggtttg tgcgtatata ggttttagag ttgaatgttt 12120
   agattatgta tatgtattta tttattgtgt ttttttttat tggtgcggta tttataggat 12180
   aaaatttgag gatatagtgt gtgtgtgttt gtgtgtatat ataaatatat acgttagtgt 12240
   tagaggtttg tttaggtgaa agtgaatgta ggttgggggt ttttagaaat gttataatta 12300
   attttatagg tgttcgttgt tatttgtaag gttgtgtttg ttataggaga tttgtttgta 12360
   taaatggtag tttgaatagt gtttattgta tattaagagg ttgtatgttt tgaatttata 12420
   tttttatttt tggttttatt ttttaataga atggatattg ggttttataa tgtatatgtt 12480
   aaatattacg gatttttata ggttttagat ttacgtttag gagtttgttt agagatataa 12540
   tgtgaatatt ggcgtgtatg tatcggatag tcgttgatat ttaaaaatgg aaaggttagt 12600
   gtagttattt tttttttata tttaaaaatt atttgggata taataggtga atgtagattt 12660
   tttaaatagt ggatgtatat ttagatattt tcgaattata taaataatat ttatagtaga 12720
   tatatatttt ggatatttaa aagaatatat ataagtatta atttataaat atatacgaat 12780
   attttacgag tataaagata tatatatata tatatatata tatatatagg taaataattt 12840
   ttggatataa atttatatat ttttatattt tttaaaaata tatatattgt aatgaatata 12900
   ttgagttttt tatataaata tataggtttt aatttgtaga ttttcggaag gacgttagcg 12960
   tgaatattta gaaatagaga aaaatataga taaatttata gatatttgga ttgatgtaga 13020
   agtgtgtgaa tagataggtg aaaaaatata ataaatacgt cgtagttaga tagtttttac 13080
   gaaaatgcgg cgcgtttttt ttttttttag ggttattttg cgtggggatt aaggtttggt 13140
   cgtgtttcgg agtttgtata tttggagttc gtatcgcggt tttatttgtt ttgttggttc 13200
   gaggagtcgg ataatttttt tttaaattta ttttgatttt ttaggatttg atggaatgga 13260
   tagggaaaag tttttatttt tttgtgtttt tttttttttt tttttttttt tttttttttt 13320
   ttttttggtg ttaggggtcg tttataggtc ggaataattt aagtttttcg ttttttcgtc 13380
   gagttggggt agttgattat tgagttgaaa ttaaacgttt taggtggaaa aaaagcgttc 13440
   gaaggtatcg tgaaatgatt aaggaattaa agagtttttc gttatgtgag attatgtttt 13500
   gttttcgtta atattataag atgtttttag atacgtcgcg tttttagcgc gtcgttttat 13560
   attgtcggtt cggagcgagg aaagggtagg cgttgcgcgg tcgggtttgt ttagcgcgtt 13620
   agacgtggcg gattcggttc ggtcggagta gagcgggaag tcgggagagt agtagtgttg 13680
   ttgtcgcgtc gttttagatt tttatagggg ttggggggag ggaaggaagg ttttagtttg 13740
   cgtcgggcgt tagttagcgt atttacggga aggggtagat cgaagcggag ttcgggagtt 13800
   cgggtttttt agtcgtattt tttattaaga tatgcgtttg ggggtcgcgg ttttttgcgt 13860
   tgtgcgcggg gtttgtgata tttttatgtg taaatttcgt tgtttagacg agcgtaggtc 13920
   gggtttttgt ttttgaagaa attaattgtt cggttaagta ggtgtttttg aaaggggttg 13980
   taaaggttgg tagtgttgtt aggtatcgag gcgacgtttc ggtttatttc gatttatttt 14040
   tagggcggcg ttcgatttta gggtttttag ttatttaatt tttattttaa tttgttgggg 14100
   ttttgattat tgttgggttt ttgattttag ggtagcgtta gatttttaaa gtttttaggt 14160
   tttatttagt tttttagttt tagaattttt ttttatatta agttttattt tggtttagag 14220
   tatggaggtt aagttgtgaa atattggaga tgaaattcgg gttttcgttt tggagtattc 14280
   gggtagagta gaatatttta gtagtttttg gagattggcg ggagttttag atattttatt 14340
   tcggtttata agtggaatcg tattgttttc gagttttgag acgttgtgga gttttagagt 14400
   tttaggattt gattttagag aggaaattag atttatagag atcgagtaga ggtgtggtat 14460
   gtttagtttt tttttttcgg gttagattta agtttttttt ttttgttttt ttttttagtt 14520
   ttgagttagt ttagttgttg ggtttagtta ttgaaagttt gtggggtttt gtagcggggt 14580
   ggtcgtaggg gaaaggtttt ttagggcggg ggagcgtttc gtaggaggat ttttggttcg 14640
   tcggataatg gtagttttgg agagtgaatc gtttttagta agagtgttgt ttgttttttg 14700
   gagggggtgg ttacgaggtt gggtcgtaat ttagggggaa gagtttagag ttcgaggaaa 14760
   ggggggtttt cggcggtaat tggatattat atatttattg agtagatttc gttataatcg 14820
   gaaatgtggt tgtggatttt cgggcggttt ttttattaag tggtttgcgt agttttttag 14880
   cgtcgttttt tttttgtagt ttgcgggttc gtgtttttat ttcgagattt gatttttggt 14940
   tattttcgag tgttcgattt ttttttttcg tagtttagtt ttagtttagg tttttcgttt 15000
   tttcgtttcg ttcgcgtcgg gaggtagcgt ttgggaggag cggtttgttc gcgtttggag 15060
   attaaaagtt ttttcggaat tgatatttta tttcgggttg ggggcggggg gtgttggtgt 15120
   tttgttgttg ttgttgtttt tatttagagg gtttgttttg tttttgtttg ttttttgtga 15180
   gtttttattt ttttatggga tatttttttg tttcgggtgt tttgcgtgtt gttttttcgt 15240
   gggattgatt ttagcgggtt ttgttttgta agttttggtt tttggggtga gttttgattt 15300
   tgtgtaattt tgtattttgt ttttgtaggt tgtttttttg tgtgtcgttt gtaaggtttt 15360
   ttttttttcg cggtgtttag atgtgttttg atttggtgtg agtgggtgtc gtttgtgtgt 15420
   gtgttgtttt agggaatttc gtgttgtgtg tgttttgttt ttttcgattt gaattttatt 15480
   tttttgaatt gtacgttttg gtgggggatc gggttatatt tttatgtgtt gtttttagtt 15540
   tttgtgtttg tgagatgcgt tggtgagagt ttgcgtgtgt attttggttt ttgtggggtt 15600
   ttgggattag ggaggggata ttagtcgtta tttcgttttt gttcgtattt atgaagggta 15660
   gaggattagg ttaagtatta cgaatcgtag gttcgtcgtg tttcgtcgtt tcgcgttttg 15720
   gacgttcgag attagcggga aataaatgtg gttttcgagg gggcgttcgt tggtcgttaa 15780
   aatttcgcgg gttcggaggc gtcgttgttg ggttagaatc gtagagtttt tttcgcgggg 15840
   tatcgaggtt tagtcgtcgt ttttttgttt attttttttt ttgtgagtcg gttttttttt 15900
   gcgtttggtt agtatgtttc ggatcgttcg gattttcgta gcgttggttt aaggggcggt 15960
   ttttttcggc ggagtaggtg acgaattatt tcggaggata cggaattgat attgcggttt 16020
   tagagatacg ggataagggg ttagaacgcg gggagtttag gtttagatcg gagtcgggat 16080
   ttcgggaaaa ttttttattt tttttgttcg tagttttaga attttagaaa tttaatttcg 16140
   cgtagttttc ggtttggaga gttcgttttg gtttgtaagt atagatatat gtgtttatat 16200
   tcgaatatac gtagatatcg ttagagagcg agatagcgag cgggcgtttt cgtagtagcg 16260
   gtttgcgggg cggaatcgga gttggcgtcg ttttaagggg aaatcgaatt gtagtgatcg 16320
   gaaatgtttt taggatagaa taagttattt tttttgttta cgttgttagg atttttttag 16380
   tttaatatat tttggtttgt ttaatgttat acgaggagaa agatttagag gtagagatat 16440
   atgttggttt aatcggcgag gaaatacgag tagagtttaa atagaaagaa atatggaacg 16500
   gggggagatt agattgtggg gaaggtaaat tggaggttta gaaaaggggt tggtgcggaa 16560
   gtttttttag gtttttgggc gttttggtat tttgtgagaa ggagaaagta gaagtcgaga 16620
   atagagagtg ataggaaaag aaaagagacg tagaaggggt tggtttaggt acgttgcgag 16680
   ttgtttttta gttaagacgt tgcgggtaag ggagggggag gtcgatatcg ttaggcgagg 16740
   ggttaaggtc gggtttttta atttagttag gaaatatcgt tgggggtttt agggttcgtg 16800
   gagttcgaat tggggtttat tttcggttcg atgttttttt tttttagatt tttagttcgg 16860
   aatttagtaa aggttgggtt atcgtcgttc gttattgggt ttttttttgt gcgagttttg 16920
   ggagaagttt tagggaacga ttttggtatt ggggttcggt ttcggagtta gcgtagagcg 16980
   tgggtgggag tcgatattta ggtaggaggt aaagaagagg tttcgtttta aagtcgaggg 17040
   tttagttttt cgaaagtttg tggtcggagt tggatttata ggattcgggg ttcgtatagc 17100
   gcgtacgtgg tatcgtattt tcggtaggcg gcgtttttaa tgtttagttt cgttttgttt 17160
   cgtttgtcgg gtttgaattg tgttagtatt gagggtggac gtcgagtcgc ggcgagttgg 17220
   ttttttgcgg tataaatttg gggttttttt aggggcggtg tcgggatgga tgggggcgtt 17280
   tttttatttg taattttttt agcgtagagc gtaaaaagtg tatatcgcgg gttagtcgtc 17340
   gtttcggggt tttaggcggc gttcggggta aaaaggagtt gaggattggt ttgggttaaa 17400
   atatttattt tagttggtag tggggttttt tgcgtttata gggtttcgag tttcgggtgc 17460
   gggtttattg gggtattatg tgttagcgat tcgttattcg gaaatatttg ggttgcgtta 17520
   gaatacgtaa tgggtttgtg ttcgggtaat ttggtgttta tggtgggtta ttattattgt 17580
   tgttttattt tattgttttt tttgttttta ttagaaatgt tattgagaga agttttaaag 17640
   agcgtagatt tggggtcgtt tggatgggtt tattttaatt taaatttatt aatttaattt 17700
   agttttacga ttagttttta gcgtataggt atatatggta gacgttaagt gtatgtcgta 17760
   tgtatatgta tagagacgtt atttgaaggt aatttttgga gaattttgag ataaaattta 17820
   tttttttgga gttatattcg taatatatga atatggaaaa taggtaatta gttgtattta 17880
   aaatttgtta atacgtttta gtaatttatt tttatttaag aatttaaaat ggggtattga 17940
   aaagatttat tggtattttg gttgtttaat attgaaaata agcgtaaatt agtttatttt 18000
   tttttatatt tttttttaaa tttaataata ttataataag tgtaagtaat aaaattatat 18060
   tttgttaaag aaagtttgta tataggatgt tttattttta ttagtattta tacgatgttt 18120
   tgttggtttt ttttattttt ttttttttta gtttatcgtg taatgtgagt tttaaatatt 18180
   tatgcgtcgg tttagattta tatttattta atgtgtttta aattaataat attttaatgt 18240
   gatataattt gattttatga agtattggaa tataaaaaag tttattgtat aagcgatttt 18300
   gagtgagggt gagtgtataa gttaaacgat ttttttagtt ttggacgtag attagagtgt 18360
   taaacggaag tgaagcgcgt ttttaagtaa gttttttaat attttcgttt ttttattttt 18420
   tatttttttg taataatttt agagtcgtgg tagaggatta ttttaggatt ggggtgtttt 18480
   ttttggagtt tgttttgtga atttgtaaat cgttaagtga atattttagt tttagggtag 18540
   gttgtgggtt gatttttatt tttttttttg ggagggaagt tttttgggaa ggttaggggg 18600
   ttataggata tgaagtttgg atttgagaac gtgtcggaaa taaaattgtg ttttaaaaat 18660
   tttttaggtt tgttgttga 18679

<210> 319
<211> 6120
<212> DNA
<213> Artificial Sequence

<220>
<223> chemically treated genomic DNA (Homo sapiens)

<400> 319

tttttagtta ttgttatcgt tatttaagaa ttttaaattt gttatttata ataaagttga 60
tttgaagtgt tatttttttt tggaggaatt ggggagaaga atgggaaaaa agatgggagt 120
gattgtataa tgttagtatt ttgtgttttt ggtttagtat ttggattgga tggaggatgt 180
aagtatagtt taaaagtaag aataagtata tttaggggtt ttatgataat ttagggtatt 240
atttgaaagt aagaatttag tagttaaggg agaaatcgta tatattaggt taggggtttt 300
taattttggg gttatagatt ggtattggtt tatggttttt taggaattgg gttatatagt 360
aggaggtgag taagtattat tgtttaagtt ttattttttg ttagattagt ataagtatta 420
aatttttata ggaattcgaa ttttattgtg aattgtgtat gtaagggatt taagtttttc 480
gttttttacg ggaatttaat gtttaatgat ttggggtgga atagttttat tttgaaatta 540
gtttttcgtc gttatcgatt tatggaataa ttgtttttta cgaaattttt ttttggtgtt 600
aaaaaggtag gagattattg tattagatga tgtatatatt ttgtttttta ttttagggtt 660
tttatttatg gttatttagg agatttttaa ggttataagt taagtagatg gagagagtat 720
tttgaaattt tgtttatttt gtagtaatat gttgttaggt ttgaaatatg gagttatgag 780
gtattttgaa agttaataat atttatagtt tattaagtat ttattatgta ttaagtgttt 840
tattatatta ttaatatatt aattttatga agtaggtgtt attaaaattt tattttattt 900
agaaattgag gtatagagat ttgtttaaga ttgtaaggaa taagcggtag gattagattt 960
ttttattata ttttatattt taaattattt agttataaat tttttaatat gataggttgt 1020
tatttagagg tattaaatgg ttgtttgttt tgtttttttt ttgatgttaa ttagtttgat 1080
tagtattgat taaagattaa gttagagagg tagttttttt tttttttaat tttattttat 1140

tttttttttt ttttgaaata gggtattgtt ttgttgttta gtttggagtg tagtggtata 1200
aatggtttat tgtagttata attttttggg tttaagtaat ttttttattt tagtttttta 1260
ttgttttata tttatatttt ttatgtttaa aataaatttt tttttgtttt ttgtttgtat 1320
taaattataa gtttttaaaa tgaatttttt tcgtatttta tttggtatat attttgtttt 1380
ttgaatagag tgtatttttt attttatttt attttggaga cggagtttcg ttttgttatt 1440
taggttggag cgtagtggta taattttagt ttatcgtaat tttcgttttt cgggtttaag 1500
taattttttt gttttaattt tttgagtagt tgggattata ggcgtatgtg gttacgttta 1560
gttaattttt tgtattttag taaagatggg gttttattat gttttttagg ttggttttta 1620
atttttgagt ttaggtaatt tattcgtttt agttttttaa agtgttagga ttataggtgt 1680
gagttatcgt agtcggttat tttttgaata gattttaaat atttagaggt tagggatgat 1740
tatttaatat atatatattg aatatttatt atgtgttgga ttcggtgtta gggttttatg 1800
tatatatttg agagttttat atttttggat ttgaattttt tattttttat tggaattatg 1860
ttttttttag tttcggtaag taagagggaa gatcgggagg gttaaatttt atattagggt 1920
ttattttagg gagggaaggg atttggttgg ggggaggggg attttgagga gtgaaattat 1980
tttttgtgta gttagttttt gtgttgatag aaagagtaga aaaggaggtg gggtggaggg 2040
agtatagtta gggattaggg gattattgag gttttggaga tgagatcgtt aggagttttt 2100
ttttattatg agtttttttt atttttgtag ttggaggagt tttttttagt tttagtgttg 2160
ttttggggcg agagagattg aataagttgt ttgggtggga agagaatgga ggaagttgat 2220
agggatgggc ggggttcgtg ggggggttga ttaggaattt agttttttgt ttagtattta 2280
ggtatttagt ttttagttta tttttatttt ttttagtttt tatttttttt aggaatttaa 2340
ggttttagtt ttttttttaa attttagtta tttttttttt attagttttt tttttttagg 2400
ggatggaggt tgagagattt taggaagaag aggatggtga gtaggtgagt tgggtacggg 2460
gttggggagg ttgatattgg gaaagaaggg aggtgagagg atttggggta gaaatgtagg 2520
gatatagggg ttttgaaagg tttgggtaaa ttgaggtagg aatagagata tatagagagg 2580
aaacgggtta ttggtttagt tttttgttta tttttttcgt tttaattatt attgtttgat 2640
tagaatggat atattttggt attagggttt tttttaggat gaggaaggtt ggtttttttt 2700
aaattttatt attcggtttt ggcgagttgt tttttatttt tttttttttt tagggattcg 2760
ttatataggt attttatttt atttagggag agtttcgatt ttagtgttta tattttgatt 2820
ttattattaa ggtttatttt attgtgggtt ttttttttat tttttttaga ttttttttgg 2880
gattttttat tgtatttttt tttttttgat ttaaagtttt taattacgtt attttgttta 2940
tattttttta cggtttttgt ttgttaattt ttttgggttt ttgagttttt tatatttttt 3000
tttttagttt tgggatttcg ttcggttttt ttgttttttt tgtagattga attttttttg 3060
gatttggtgg ttgaagttta gtttcgtcgt ttggaggagt agagggttat tttttatatt 3120
ttttaaaatt ttttaagttt agtttttgtt ttatttcgtt ttttcgagga tagagaatag 3180
ttttatagta ttatttttag ttattaggta agatattttt ttaggaggta aatttaggtt 3240
ttttggtttt ttggttttttg tttttttttgg ggtttatttt ttgttttttt ttaggtattt 3300
tatcgttttt ataggttttt tatatgtttt tttatattaa tttttgattt ttttaagaat 3360
tttttttttt tagattttta ttaaagtttt tttttttttt ttattttttt agtgttagcg 3420
gatggaagtt tagcggttag agtttttttt ttttttaggg gggtaagagt ttttggagtt 3480
gttgttgaga gtttagggtg ggggtcgaat ggaggagtaa aggtttcggt tttttatata 3540
tatttgttga gatttgagtt ttaattagtt tttttttgtt attggtttta aagttgggta 3600
gtttattgta tgtttttaat ttttgtttgg taggggtatt agagattgaa agatacggta 3660
taaattttaa tatttatttt ttatattatt ttttttggga attggatagg gtgaaagttt 3720
ttaaattttg ggaataggcg agatggaata gggatttaat ttttcgttta taggtttatg 3780
ggagtttgag gtagtaaggg ggattttagg tatttatttt aaggagtggt tgggagtttt 3840
ttttttaatt tgtggggata ttattagttg ttaagttatt aggtagtagg gtttgagggt 3900
ttaggttttt atttgagagg ttataatttg agagatagtt ttattttttt ttttagtaag 3960
aagggaaggt gggtgggtat ttgagagatt aagattattt ttttagtttt attattagta 4020
ttttcgattt ttgagattga ggggtttacg ggttgtgaat ggatttttag ttttgtttat 4080
tttttttttt tattgttgtt gagtttgttt gatgttttgg ttgtgtgaat aaatataatt 4140
tttttttgga ttgtagattg gtatttgggg ggtttaggcg gggtgaaagg taggaaggtg 4200
aggttagagg tttttttttt ttttagtttg gttagaggtt agtttttttt ttcggttggt 4260
taattattgg tttattaagt agcggttgga gattttttta attatttttt ttagtttttt 4320
ttttcggttt taattaagta gaatagggag gggagttatt agaataagaa atatgaattg 4380
agttgtcggt gaatttaggt attttagcgg tttgagttta tatcgtttag atttttgatt 4440
taggatttag gtgatagacg tttttagtcg ttaatatagt tttattttta ggtcgcggaa 4500
gtttagttag gggggttttt tatatttttt agatggttcg tttttttttt ttattttttt 4560
tttttttttt ttttttttat taggttttag tttttttgtt tttgtgtttt ttttttcgtt 4620
tttagttttt ttttgttttt tttttttttt tttttttttt tttttttcgg tttttttttt 4680
ttagtttttt tttttcgttt tttttttttt tttttttttt tttttttttt ttttttatat 4740
atattttcgt ttgggttttt tttttttttt cggttttatt tttttcgtcg tcgggggtcg 4800
gggtttttttg tgggggggttt agtcggtatt ttaggttttt ttttagtgtc ggggtgaatt 4860
ttcgggggag tcgggagtcg ggggtagacg ggcgggggtt ggggcggagg gagtagcggt 4920
tttagcgagt ttggggggag aagtaattag gcggggggag gggcggagta gggagggggt 4980
tttagggttt ttttttagtt atggacgaac ggttattggg gtcgtttttt ttaggcgggg 5040
gtcggggggg tttaggattg gtgagtgggg agtttggggg ttttggcgag tttttcggtg 5100

gcggagattt cggtgggggt agcggggggg tttcgggagg tcgagggaag taagatatcg 5160
gggatatttt gtagtagata atgattatta tcgattagag tttggacgag gtttaggtta 5220
agtgagtgtt tttatttcgg gattttatat agatttagta aatttcgttt atatgttttg 5280
aattttttgg gagttttttt taattttagg gtttttttta ggatttaata gttttttttt 5340
ttttttattt ttgggagttt atagaaaagt gatttttttt aaattttttt ttatttttag 5400
gttttgaaat ttttatagag ggaattttcg gtggttcggt tttttatttt taattttttg 5460
tcgatttttg tagttttttg gaatagtttt attttcggga gttttttttg gtttttagat 5520
taagaaattg tttttgggtt acgttatttt tttttttaat tttttattta gtttttttat 5580
tttttttaga tgtggagatt tttatatttt ttttagagtt tttaaagttt ttttttattg 5640
tttagttaga tattaggtgt attaaagttt tttatttgtt tagttttagg atttttttat 5700
atattttata ttgatttttt tagttagttc ggtattttta gttttatttt gttattttaa 5760
atttgatttt tttttaattt tagttttgt tttttttttt ttgaaattta ttaagttatt 5820
tttttttttt tatttatttt tagatttttt tttttatttt tttagattat tttttaaagt 5880
tcgtagtttt taatttgttg tttgtatttt ttttgtgttt ttttgaagtt gaggagtttt 5940
tttatgtttt gggagttgat ttttttttaa gaatttttta ttttattttt aatttatttt 6000
atttttaatt cgtttttttt tttcgtagat tgattttttt ttttttttgt ttttaggttt 6060
tttgttttgt ttttttagtt ttttaatttt tttttttttt tatttttatt ttttttaagg 6120

<210> 320
<211> 6120
<212> DNA
<213> Artificial Sequence

<220>
<223> chemically treated genomic DNA (Homo sapiens)

<400> 320

ttttgggagg agtgggagtg gggaaagaga aaagttgagg agttagagaa atagagtagg 60
gggtttgaga ataaggaggg aggaggttta gtttgcggag ggaggaagcg gattgggggt 120
ggaatgagtt gggggtggaa tgaggagttt ttgggaaaag attagttttt agagtatggg 180
gaagtttttt agttttaggg agatataggg aagatgtagg tagtaggtta aaggttgcgg 240
gttttgggag atggtttaga aaggtaggag gaggaatttg ggagtggatg gagaaaggaa 300
agtgatttgg taggttttag agggagagag atagaggttg gggttgagaa gagttagagt 360
ttgaggtggt agagtggggt tgggggtgtc gagttaattg gggagattag tgtagggtgt 420
gtgaaggggt tttggggttg agtaggtggg aggttttgat gtatttagtg tttggttgag 480
tagtggagag gagttttagg ggttttggag agggtgtgga ggtttttata tttggagaga 540
atgagggggt tgggtggaga gttaggggag aagataacgt agtttaagaa tagtttttta 600
gtttgggagt tagagggggt tttcggggat ggggttgttt taggagattg taggggtcgg 660
taaaaggtta ggagtgggga gtcgggttat cgggggtttt ttttgtgaag gttttagggt 720
ttgggggtga agggaggttt gagagggatt atttttttat gggtttttag gaataaggag 780
agaagagagt tgttggattt tggagagggt tttggagttg gggggggttt ttagaagatt 840
tagaatatgt gaacggggtt tgttgggttt gtgtggggtt tcggagtggg ggtatttatt 900
tggtttgggt ttcgtttagg ttttggtcgg tgatggttat tatttgttgt agaatgtttt 960
cgatgttttg tttttttcgg tttttcggga ttttttcgtt atttttatcg gggttttcgt 1020
tatcgggagg ttcgttaggg tttttaggtt ttttatttat taattttagg ttttttcggt 1080
tttcgtttgg agggggcggt tttagtagtc gttcgtttat agttgggggg gggttttgag 1140
gttttttttt tgtttcgttt tttttttcgt ttggttattt ttttttttaa attcgttggg 1200
gtcgttgttt ttttcgtttt aattttcgtt cgtttgtttt cggttttcgg ttttttcggg 1260
ggtttatttc ggtattgaag ggagatttgg gatatcggtt gggtttttta taggagattt 1320
cggttttcgg cggcggagaa aatggagtcg gagagagaga ggaggtttaa gcgggggtgt 1380
gtgtgagaga gagggaggag ggaggaggga gaaggggggg gagcgaggga gggaggttgg 1440
gggaggggag tcggagagga agaggagggg agaagagagg aggaataggg aggagttggg 1500
ggcggagaga gagatataga aatagaggaa ttgagattta gtggaggagg ggagagggaa 1560
gaggggatga ggggaggaga cgggttattt gaaagatatg ggaaagtttt ttggttggat 1620
tttcgcggtt taggagtggg gttgtgttgg cggttggggg cgtttgttat ttgggttttg 1680
aattagggat ttaagcgatg tggatttagg tcgttggaat gtttgggttt atcggtagtt 1740
tagtttatat tttttgtttt aatgattttt ttttttgttt tatttaatta aaatcgaaga 1800
ggggggttgg gggagataat tagggaggtt tttagtcgtt gtttaatgag ttagtaatta 1860
attagtcggg gaggggagtt ggtttttggt tagattgggg agagaaaagg tttttggttt 1920
tattttttta ttttttattt cgtttgggtt ttttagatat tagtttgtag tttagagggg 1980
aattatattt atttatataa ttaaaatatt agatagattt agtagtagtg gggagggagg 2040
gtgggtaggg ttgaaggttt atttatagtt cgtaaatttt ttagttttag ggatcggggg 2100
tgttggtagt gggattggga gaatagtttt aattttttag gtgtttattt attttttttt 2160
tttattggga ggaagggtag agttgttttt taggttataa ttttttaggt ggaggtttga 2220
gtttttagat tttattgttt agtagtttga taattggtgg tgtttttata agttagggaa 2280

aagattttta gttatttttt gagatgggtg tttgggattt tttttattgt tttaagtttt 2340
tatggatttg tgggcgggga gttaaatttt tgttttattt cgtttgtttt tagagtttga 2400
ggatttttat tttgtttagt ttttagggaa ggtgatgtgg gagatgaata ttgagatttg 2460
tgtcgtgttt tttagttttt ggtatttttg ttaagtaaga gttgagggta tgtaatgggt 2520
tgtttagttt tgagattagt ggtaaggaag ggttggttgg ggtttaagtt ttagtaggtg 2580
tgtgtggggg gtcgggattt ttgttttttt attcgatttt tattttgaat ttttagtagt 2640
aattttagga gtttttgttt ttttggaggg aggggaggtt ttgatcgttg ggtttttatt 2700
cgttggtatt ggaggagtgg agggagaggg agagttttgg tgagggtttg agaggaggag 2760
gtttttgaga ggattaaggg ttggtatggg gaggtatata ggaaatttgt gaaggcgatg 2820
gggtgtttag ggagaaatag gagtagagtt ttaaagagaa taggggttaa gagattagga 2880
ggtttgggtt tgttttttgg ggggatgttt tatttggtga ttgaggatag tgttgtaaag 2940
ttgttttttg ttttcgagag gacggagtgg ggtaggggtt aggtttgagg ggttttgggg 3000
ggtgtagaaa gtggtttttt gtttttttag gcggcgggat tgggttttag ttattaggtt 3060
tagaaggagt ttagtttgta gggagagtag ggaggtcgag cggggtttta gggttgggga 3120
gaggggtgtg gagggtttag agatttagag aggttggtag ataggagtcg tgggggagtg 3180
tggatagggt gacgtgatta gggattttgg attagaggag agggggtgta atggggaatt 3240
ttaaggggag tttggaggag gtggggagag ggtttataat ggagtgggtt ttggtaatgg 3300
ggttaggatg tgggtattag ggtcggggtt ttttttgggt gggtaggggt atttgtgtgg 3360
cgggtttttg gaggaggagg ggatggagga gtagttcgtt aaggtcgagt ggtggagttt 3420
ggagggggtt agtttttttt attttgaggg gggttttgat gttaaaatat gtttatttta 3480
gttaagtagt ggtggttgaa gcgggaggag tggatagggg gttaggttag tggttcgttt 3540
tttttttgtg tgtttttgtt tttgttttag tttgtttaag ttttttaagg tttttgtgtt 3600
tttatatttt tgttttaggt ttttttattt tttttttttt ttagtgttag tttttttaat 3660
ttcgtgttta gtttatttgt ttattatttt tttttttttg gggtttttta gtttttattt 3720
tttagagggg agaaattggt gggggagggg tggttgggat ttgggaggag ggttggaatt 3780
ttgggttttt gaggggagtg ggggttggaa ggggtggggg tgagttgggg gttggatgtt 3840
tgggtattga gtaggaagtt gggtttttgg ttagtttttt tacgggtttc gtttattttt 3900
gttaattttt tttatttttt ttttatttaa atagtttgtt tagttttttt cgttttaggg 3960
tagtattgag attgggaaaa atttttttag ttgtaggagt ggagggggtt tatggtgggg 4020
aaggattttt ggcggtttta tttttagagt tttagtagtt ttttaatttt tggttgtgtt 4080
ttttttattt tatttttttt tttgtttttt ttgttaatat aggaattagt tatataggaa 4140
gtggttttat tttttagaat tttttttttt ttagttaggt tttttttttt tttaagatag 4200
attttggtgt aggatttggt tttttcgatt ttttttttta tttatcggga ttgggagggg 4260
tatggtttta gtgggaagtg gaggatttag atttagggat gtggagtttt taaatatata 4320
tataaaattt tagtatcggg tttaatatat agtaagtatt taatatatat gtattgaata 4380
attatttttg atttttaggt atttaaaatt tatttaggag atggtcggtt gcggtggttt 4440
atatttgtaa ttttagtatt ttgggaggtt gaggcgggtg gattgtttga gtttaggagt 4500
tggagattag tttggggaat atggtgaaat tttattttta ttaaaatata aaaaattagt 4560
tgggcgtggt tatatgcgtt tgtaatttta gttatttagg aggttgaggt aggagaattg 4620
tttgaattcg ggaggcggag gttgcggtga attgagattg tgttattgcg ttttagttta 4680
ggtgatagag cgagatttcg tttttaaaat aaaataaaat aaaaaatata ttttatttag 4740
gagataagat gtgtattaaa tagagtacgg gaagggttta ttttggaaat ttatagttta 4800
gtgtagataa ggggtagggg aaagtttatt ttgggtataa gagatataga tatggaataa 4860
tgagaggttg aggtaggaag attgtttgag tttaggaggt tgtggttgta gtgagttatt 4920
tgtgttattg tattttaggt tgggtaatag agtaatattt tgttttagaa aaagaaagaa 4980
atgaaatgaa attgaaaagg gagaggatta tttttttggt ttggtttttg attaatgtta 5040
attaggttgg ttggtattaa ggaaggagta gggtagataa ttatttggta tttttaaatg 5100
gtaatttgtt atgttaggga gtttatagtt gagtgatttg gaatgtgaaa tgtgatgaag 5160
agatttggtt ttgtcgttta ttttttgtaa ttttgggtag atttttgtgt tttaattttt 5220
gagtgaaata gggttttaat agtatttatt ttatagggtt gatgtattaa taatgtaatg 5280
aagtatttga tgtatagtga atatttaata aattgtagat attattggtt tttaaaatgt 5340
tttatgattt tatgttttaa atttagtaat atattgttgt aaggtggata aagttttaag 5400
atattttttt tatttatttg atttgtggtt ttaggaattt tttaagtggt tataagtaaa 5460
agttttagga tgagggataa agtgtgtgta ttatttagtg tagtggtttt ttattttttt 5520
ggtattaggg aagagtttcg tggaagataa ttattttatg ggtcggtggc gacggagggt 5580
tggttttagg atgaaattgt tttattttag attattaagt attagatttt cgtaagaagc 5640
gagaaattta gattttttgt atgtatagtt tataataggg ttcgagtttt tatgagaatt 5700
taatgtttat gttgatttga taggaggtgg agtttgggta gtaatgtttg tttatttttt 5760
gttgtgtggt ttagttttta agaggttatg gattagtatt agtttgtggt ttaagggttg 5820
gggatttttg atttagtgtg tgcggttttt tttttggtta ttagattttt gtttttagat 5880
aatattttaa attattatag ggtttttaaa tatatttatt tttgttttta aattatattt 5940
atatttttta tttaatttaa atgttgagtt aaaagtataa aatgttgata ttatgtagtt 6000
atttttattt ttttttttat tttttttttt aattttttta aaaaaaggta atattttaaa 6060
ttagttttat tatgggtgat agatttaggg tttttaaata gcgatagtag tggttagaag 6120

<210> 321

<211> 6700
<212> DNA
<213> Artificial Sequence

<220>
<223> chemically treated genomic DNA (Homo sapiens)

<400> 321

tagtcgattt agttttcgag atttttttac gtcgttttaa aattaaaacg gagtttaata 60
cgaagttggg tgaagtcgta gtttgtagga gttagggaga tgcgttttgt tcgggatttt 120
tcgggttttg ttgagacgga aaggatcgta gggaagatag gataattggt ttttaatttt 180
gatcgttatt ttcgaaattg tattttataa gtatgttttt tattttcgtg gtttgatggg 240
gatatttttt ttgattaaga ttcggaataa ttatttattt aatgggattt tttagaggga 300
agcgttggtt agaatttttt taggttgaga tgtaattatc gttattgagt ttgttttttt 360
ttagggaaat tttataaaat tattttgaga attttagttt tttttggtcg gtatttgaga 420
ttgtatagat ttaaattgaa aggtgatcga aggggaggag tagttgttcg tgcggaggaa 480
gtttttgggg tcgaagtggt ttcggtcgag tatgtcgtaa aggaggtatt attgcgttga 540
gcgtttattg tatttttatt tttaaattat taaggtaaaa ttatttttag ttaaaaatgt 600
ttgaaagatt ttttaaaata gtgggagtgg gcgttagggg gaagagatgg tttaggaaat 660
ttttcgtagg gcgttttagg gatgagttga gggaagtcgt atgacgcgcg gtttttcggt 720
gttatagtta aggatagata ttttcgtaaa atttagaatg aaaaaagagt acgttttttt 780
tgggagcgtt gtttttttgg gattggggtt ttttattttt attttttttt tttttttgat 840
cggtttcgta gttttacgat aagttagttg gtttggtttt tgatttgggt ttcggttcgt 900
attttcgggg cgttttgttg tgttatagtc gttcgacgtt tttagattcg gttaggttat 960
tagggtagat tggaggttcg cgttttttta ttcgatattt ttcgtttatt ttattttttt 1020
ttttttcgaa aataggaaag ggaagaaaat tgaaacgggt tttttggttt gtagttttag 1080
cggagtcggg attttatagt tttgagtggt atatgtcggt taatttttta aagtcgggtt 1140
ttcgtgtggg gagaaatata tacgtaggaa tgtataagta tcgcgtgttc gtaattgtcg 1200
ttgggaggtt tggcggttgt gatgggatat gtatttattc gggtaatacg tttataggtg 1260
atatttattc gattagttaa tagatattta ttgcgtattt acgacgttat ttggtatttt 1320
tttttttttt ttagttgttg ttgtttagtt tagcgttagt ttagagacgg gtttatcggt 1380
cgtttttttt tataagaaat tcgtgttcgg cgtgaagagc ggacgataaa tattttttaa 1440
gtttagcgaa atgtattaat tataggagcg gttgggagtt cgcgatacgg ggatttgttt 1500
ttttagggat aggagttttt tatttgtaag ggattcggtt tggggaattg gaatagataa 1560
ggggtaattt ttttttttat tatttttaat gagaatattg ttcggggttg agatatttgg 1620
gggacgtcgt tgtttatttt attaaatttt ttttagggat agaaaagcgt ttttatcgaa 1680
aacggattta aatatcgttt ttatttttgt taaggaagtt tggataggga ggttgtggtt 1740
tttaattcgt taggtaaaag gaattttatt agttcgtatt ttttattttt attttgagta 1800
ttgagtgttt gcgagaaatt cgttttgcgt tagtaatttt gggtttcggt tttagttttc 1860
gcgtagacgc ggaggagaga ttggcggttt tcgttgggga aattgagttc ggcgtcggtt 1920
cgggacgtgt aagaggggga aagggggaga ttttagattt cggtttttcg agggtttttt 1980
tggggagtta ttcgtatttt tgtgttttcg ttttcggcgg tagatttcgc ggcgtttgtt 2040
taagagcggg tattcgaaag tacgtttcga atacggagaa gcgtttttcg ttttttgttt 2100
cgtgatttgc gttttttttt ttttttatcg tattagatgg aggattttgg tttttttcgt 2160
ttttggtaaa atgttttagt taatattggg ttaatagttt tagtttttag atttgtaatt 2220
tgaatattag gagcgggttt tttttttttt atttgtgttt gaggtttttt ggtcggggat 2280
tgcgcgcgtt tttttaggtt agggaggagg ggtcggggcg atttgagaaa gatataacga 2340
agcgaatgga gtttttgaat ggggaaaggg gagtggatat taggaggttt ttgtagaaat 2400
gtaggatcga tttattcgcg agttgataat gttacgcgtt agatttggtt tgacggtggt 2460
aacgtttggt atttggggga taggggtttt tttttggttt tagtttttaa attttgcgta 2520
gaatagaacg gttggtattt taagtgtttt ttttttattc ggatttttat agatataagt 2580
ttttttattg agttgcgtag aggtggattc gtataggagg gaatatcgcg taggaatttt 2640
tgtaaatata aattttagta gtaaagtagt ttagattgat tggaatatat tatttttagt 2700
tattattatt taagtttttt ttggttttcg gagttggaaa tagtttttgg gtttttggcg 2760
ttttttttgg atatagatga aaattgtagt ttatttttaa gtcgtttgtt tataagtttt 2820
taggaagatg taaaatttaa aaatgtttgt attaaattaa cgggaaatgt ataaattttc 2880
gtcgtttatg atgtaaaatt ttatatatta gtattttatt gtataattgt attaaaagta 2940
tatttatata tatacgtata tattttgata tatatttagt gaaatatttt aaaatatttt 3000
aaagttatag ttttttttat tgattttatt ttagtaaata tggtaattat aatattatat 3060
aattaagtat agacgatttg gttaaaatgt ttgttcgatg gttttcgagt tttttgaatt 3120
aggtgtttga gaattatata gttatattgt atatataatg tttttttatt cgggatatag 3180
aaataggttt atattacgcg gatttttttt ttttaggaaa ggacgatgtt gtttttatat 3240
tttttgaaag taattataga gttttggata cgaagttatt gtatgtaaat tttttaaatt 3300
tgtaaacgag ttatagggta ttagaaatat tttatttgaa gaagttatat tcgttaaaaa 3360
aaaaatgttg ttgaataagg ttgtaattta ttattttatg aagagtttta ttttttataa 3420

gaaatgtcgg gtttttttcg aagataacga gagaaaataa gattttaaga aattttttcg 3480
gaaggatatt gatttggatt ttgtgatagt gtttttattt cgtaaattag aaagggaaaa 3540
aaaaattagt ttttattatg atgtgggtcg atttattata ataataattt aattgaggcg 3600
aatttttgta agagtttaaa agggtggggg ggggggggga gtttaaaatt aagagttttt 3660
taatgttgtg cgtttatttg ggattttgaa agtatagaat tcgtaagcga ttaagatttt 3720
ttttttattt tagagtagat agtttcgtat ttatttaatt tggttttaag tatttcgtgt 3780
aaatataaag gttgtgttta aattatattt gaaatatatg agagatatta ttaatgtttt 3840
ttttagaatt tttaattata tgtaggtttg aatcgatcgg attagagtta gaagtatcgg 3900
cgttttattt ttttaggtgt ttcgtaaatg ttagtaaaag taggtaagta gtatgatttc 3960
gttagagtgg agcgttttgt ttgcggtggt gtttatttgt ttggtttttt ataaattaag 4020
tgatcggttt gggtttcgcg gttcgggata gtcgtattgg taaattttta ttttttaaag 4080
ttagagtagt tagtaaattt ttttttagat agggcgattc ggttgacgtt tttgattcgg 4140
ttaggaggta aagattaaaa cgttagagta gtagttttgt tattgaggag cgtcggtagg 4200
gtcgcgggta gagggggttg gagaatgatt tgttagagtt taaggtcgat gtggcgcggg 4260
gcggtttcga gagcgtcggg tttttgcgtg gttacggtcg tcgttgttaa ttttcgcggg 4320
gatttagttt tgttttttat tgattttttt tgtaaaagcg tagtagaatt ttgattagtc 4380
gtattagttt cggcgaattc gagtatgtta atttatttat atggattatt acggaggaat 4440
agcgggcgtt gagttattaa aatatttgtt ttaaaagatt atttttaagt atttttgtag 4500
gtaggtaggt tcgttttagg cgcgtaaatt cggttacgta ttaagaagcg gttgtttttc 4560
gaatattgta aattttagtt aagttttcgg tgtcgcggag agagtagtga aaagaaatgt 4620
cggaggtggg ggtagatttt agtttagata tatatatttg cgcgtatata tatatatata 4680
tatataagat tcgcgcggag aaggtattaa aattttggta tttcgagagt acgataaatt 4740
tatatatttg gaagtttcgg gtttttcgtt tttttcgtag tatttttcgt ttttttattt 4800
tatcgttcgt tttttggttg cgattttttt tttttttttt ttttttcgtt tcgttattta 4860
gtttagtgtt ataatttttt ttttttttta aaatcgggtt taattagttg tttgttaatt 4920
ttgggattgt tgtgttgtga ttggcgggtg gttttaaggt gaggcggagt atttattaaa 4980
gagattttgg gttgggagtt ggagagtcga aagcggagtt cgaaattgat tggaaatttt 5040
agtggcgcgg agattcgtta gttttaattt cggaaatttt tttttgtagg aggagaagag 5100
aaggggtgta agcgttttta tttttgtttt tttttttttt tttttttttt tttttaattc 5160
gttttttttt atttggagcg ggtagttgtg aattggttat ttcgcgtttt tttaagtgtt 5220
cgtcgcggta gtcggtcgac gcgttagttt tttcgggagt cgtttgtttc gtattcgggt 5280
agtcgagggg agaggagttc gcgtttcgag ttttcgagtc gtcgcggttt ttcgtttttt 5340
tcggttatta gttttttgtt tcgggttcgc gtatgaattt tttggatttt tttatgaaga 5400
tgatcgacga gtaggagaag ggtttgttcg gcgtttttag ttttattatg ttcgaggatt 5460
tcgcgggttc gttttgttcg tcgggtttcg gttcggatat cgagaatacg cggttttagg 5520
agaatacgtt ttttaagggc gagttcgatt tgaagaagga gagcgaggag gataagtttt 5580
tcgtgtgtat tcgcgaggcg gttagttagg tgtttaaagg ttacgattgg acgttggtgt 5640
ttatgtcggt gcgcgttaac ggttttagta agaataagtc gtacgttaag cggtttatga 5700
acgtttttat ggtgtgggcg taggcggcgc gtaggaagtt cgcggattag tattcgtatt 5760
tgtataacgt cgagtttagt aagacgttgg gtaagttttg gaggtaggat tcggcggggg 5820
cggcgcggta gggtgggtat cgcggcggtt gggggcgttg gttagggttg atttgtttcg 5880
tttcgttttt tatcgttcgg gagttgtcgt ttcgggagtc ggcgggatgg ggttgggagt 5940
gggaatgggg tgtaattgtg gtttagagtt tgataaagtt tttgggttgt tcgcggggac 6000
gcggaggagg ggggtggtaa gtggaagagg tgagggaggt agttggagga tggacgaaga 6060
ttggtgggag acggaaggag ggggttgtta gtttgttttt tagtcgtttg gaagtttaat 6120
cggggcgggg aagtgaaatt tgtttttttt ttattcggtt ttttaaaatt gtattttttc 6180
gtgtagtttt attgtttacg gagatggggt aagggagaaa tcgaggttgg aggagatttt 6240
tggtaggaat tgggaggcgg gaggagggag gttattggaa ataggtggga gtgtatggtg 6300
gggggtgaga attggggatt tttttgtagt ttaagtaatt tgggggaaag tttttaaagg 6360
gggttggggt tgggggcggt aagtcgagta gtaaaggcgt ttagggggta gtatcgggag 6420
tcgtttttat ttttagcgtt tttaaaatag aaatagaagg ggaggggagg gagggggcgg 6480
ggagtgatcg tttaggttag attgtaataa tttatttatt tatttatttt taagaaaagt 6540
tatgagttgt ggttgtaggt aggagggaag atggagttgt gtgtagagga agtcgagtgg 6600
tttgggtcgt cgtttttttt tcgtcgattt gatagtttgg cggattttat tgattttttt 6660
tttttttttt ttttgtgttt ttcgtttcgt ttcgagtaga 6700

<210> 322
<211> 6700
<212> DNA
<213> Artificial Sequence

<220>
<223> chemically treated genomic DNA (Homo sapiens)

<400> 322

tttgttcggg gcggggcggg gggtatagag aaaaagaggg agaggggtta gtgaaattcg 60
ttaaattgtt aggtcggcgg ggaggaggcg gcgatttaga ttattcggtt ttttttgtat 120
ataattttat tttttttttt gtttgtaatt atagtttata atttttttta aaaataaata 180
aataaataag ttattgtagt ttgatttgag cggttatttt tcgttttttt tttttttttt 240
tttttatttt ttattttgga aacgttggag atgaaaacga ttttcggtgt tgttttttaa 300
acgtttttgt tgttcgattt atcgttttta attttaattt tttttgaaaa ttttttttta 360
aattatttaa gttgtaagaa ggtttttaat ttttattttt tattatatat ttttatttat 420
ttttagtagt tttttttttt tcgtttttta gtttttgtta agggtttttt ttaatttcgg 480
tttttttttt gttttatttt cgtggatagt ggggttgtac gagagagtgt agttttaaga 540
ggtcgggtag gagggaggta agttttattt tttcgtttcg attgagtttt taggcgattg 600
gagagtaggt tggtagtttt tttttttcgt tttttattag ttttcgttta ttttttagtt 660
atttttttta ttttttttat ttattatttt tttttttcgc gttttcgcga gtagtttaag 720
aattttgtta aattttgagt tatagttata ttttattttt atttttaatt ttatttcgtc 780
ggttttcgga acggtaattt tcgggcgatg ggaggcgggg cggggtaaat tagttttgat 840
tagcgttttt agtcgtcgcg atgtttattt tgtcgcgtcg ttttcgtcgg gttttatttt 900
tagagtttgt ttagcgtttt gttgagttcg gcgttgtgta agtgcgggta ttggttcgcg 960
agttttttgc gcgtcgtttg cgtttatatt atgaaggcgt ttatgggtcg tttgacgtgc 1020
ggtttgtttt tgttggagtc gttgacgcgt atcggtatgg gtattagcgt ttagtcgtag 1080
tttttgagta tttggttgat cgtttcgcgg atgtatacgg ggaatttgtt tttttcgttt 1140
ttttttttta gatcgggttc gtttttgggg aacgtgtttt tttggggtcg cgtgttttcg 1200
gtgttcgagt cggagttcga cgggtagggc gagttcgcgg agttttcgga tatggtgggg 1260
ttgggggcgt cggataggtt ttttttttgt tcgtcggtta tttttatgaa ggggtttagg 1320
agatttatac gcgggttcgg ggtagggggt tggtggtcgg gaaaggcgag aagtcgcggc 1380
ggttcgggga ttcgaggcgc gggttttttt tttttcggtt gttcggatgc ggagtaagcg 1440
gttttcgggg aagttggcgc gtcggtcggt tatcgcggcg agtatttagg aaggcgcggg 1500
gtggttagtt tatagttgtt cgttttaagt ggggggaggc gaattggaga ggaggaggag 1560
gggaggaaaa agagtaaaag tgggggcgtt tgtatttttt tttttttttt tttgtaaaga 1620
aaagttttcg gggttgaaat tggcgagttt tcgcgttatt gaagttttta gttagtttcg 1680
agtttcgttt tcggtttttt aatttttagt ttagggtttt tttaataaat atttcgtttt 1740
attttagagt tattcgttaa ttatagtata gtagttttag ggttggtagg tagttgattg 1800
gattcgattt tggggaggga ggaggattgt ggtattgggt tgggtgacga ggcggagggg 1860
gaggagaggg gaggggatcg tagttaaagg gcggacggta gggtgggggg gcggggggtg 1920
ttgcggggaa ggcgggggat tcgggatttt taagtgtgta agtttgtcgt attttcggaa 1980
tgttagaatt ttagtgtttt tttcgcgcga attttgtgtg tgtgtgtgtg tgtgtgcgcg 2040
taagtgtgtg tgtttagatt aggatttatt tttattttcg atattttttt ttattgtttt 2100
tttcgcggta tcggggattt agttggagtt tgtagtattc gaaaagtagt cgttttttaa 2160
tgcgtagtcg agtttacgcg tttggagcga gtttgtttgt ttgtaaaagt gtttagaaat 2220
agttttttga agtaaatgtt ttggtgattt aacgttcgtt gttttttcgt aataatttat 2280
ataaatagat taatatgttc gggttcgtcg gggttggtgc ggttggttag gattttgttg 2340
cgtttttgta aagggagtta atggaaagta aagttaagtt ttcgcgaagg ttggtagcgg 2400
cggtcgtggt tacgtaggag ttcggcgttt tcgaggtcgt ttcgcgttat atcgattttg 2460
agttttgata agttattttt tagttttttt tattcgcgat tttgtcgacg ttttttagta 2520
atagggttat tgttttgacg ttttggtttt tgttttttgg tcgggttaaa aacgttagtc 2580
gagtcgtttt gtttggggga gagtttgtta attgttttgg ttttgagaga tagaagtttg 2640
ttaatgcggt tgtttcgggt cgcgaggttt aggtcggtta tttggtttgt aaaagattaa 2700
ataaatgggt attatcgtag ataaaacgtt ttattttggc ggagttatgt tgtttgtttg 2760
tttttgttgg tatttacgaa atatttgaag gggtgaagcg tcggtgtttt tgattttagt 2820
tcggtcggtt tagatttgta tgtagttggg agttttgggg gaggtattgg tggtgttttt 2880
tatgtatttt aagtgtgatt tgagtataat ttttgtgttt atacgaaatg tttagagtta 2940
ggttgggtaa gtgcggagtt atttgttttg ggatagaaga aaagttttgg tcgtttgcgg 3000
gttttgtgtt tttagaattt taaataaacg tatagtattg ggaaattttt aattttaaat 3060
tttttttttt tttttatttt tttgggtttt tgtaaaaatt cgttttaatt aaattattgt 3120
tgtggtgaat cggtttatat tataataaaa attagttttt tttttttttt ttaatttgcg 3180
aagtgaggat attattataa gatttaaatt aatgtttttt cgggagaatt ttttaaaatt 3240
ttgttttttt tcgttgtttt cggaagaagt tcgatatttt ttataggaaa tggaattttt 3300
tataaggtag taagttatag ttttgtttaa tagtattttt tttttaacga atgtaatttt 3360
tttaaatggg atgtttttgg tgttttatag ttcgtttata aatttaagag atttgtatat 3420
aatagtttcg tatttagggt tttgtgatta tttttagaaa gtgtaagaat agtatcgttt 3480
ttttttagga gaaaaaagtt cgcgtagtgt ggatttattt ttgtgtttcg ggtagaaggg 3540
tattgtgtgt atagtataat tgtgtaattt ttagatattt gatttaggag attcggagat 3600
tatcgggtag atattttagt taggtcgttt gtatttagtt gtatgatatt gtaattatta 3660
tgtttgttgg aataaagtta gtgaagaggg ttatgatttt aagatatttt ggaatatttt 3720
attaaatata tgttaagata tatacgtata tgtataaata tatttttaat gtaattatgt 3780
aataagatat taatatgtag agttttatat tataaacgac ggggatttat atatttttcg 3840
ttgatttgat gtaagtattt ttgaatttta tatttttttg aaaatttatg aataggcgat 3900
ttaaaaatgg attgtagttt ttatttgtat ttagaggggg cgttaaaagt ttagaaattg 3960

tttttaattt cgagaattag ggaaaatttg gatggtggtg gttgggggtg gtgtgtttta 4020
gttaatttgg attgttttgt tgttgagatt tatatttgta ggaattttta cgcgatattt 4080
ttttttgtgc ggatttattt ttgcgtagtt tagtgaggga atttgtgttt gtagagattc 4140
gggtgaaaaa agagtatttg agatgttagt cgttttattt tgcgtagaat ttggggattg 4200
aggttaggag aaggttttta ttttttaggt gttaagcgtt gttatcgtta ggttaagttt 4260
agcgcgtggt attgttagtt cgcgagtgga tcggttttgt atttttgtag ggtttttttg 4320
gtgtttattt tttttttttt atttaggggt tttattcgtt tcgttgtgtt ttttttaggt 4380
cgtttcgatt tttttttttt gatttgggaa gacgcgcgta gttttcgatt agaggatttt 4440
agatataggt agaaggaaag gaattcgttt ttgatgttta ggttatagat ttggaaatta 4500
aggttgttga tttagtgttg gttagagtat tttgttaggg gcgaaaggag ttagggtttt 4560
ttatttgatg cggtgaggga gggaaagggc gtaggttacg aggtaggaga cggagggcgt 4620
tttttcgtgt tcgggacgtg ttttcggata ttcgtttttg agtaagcgtc gcgaggtttg 4680
tcgtcgggag cggagatata gaggtgcgag tggtttttta gggaagtttt cgggaaatcg 4740
aggtttggga tttttttttt tttttttttt gtacgtttcg gatcggcgtc gggtttaatt 4800
tttttagcgg gggtcgttaa tttttttttc gcgtttacgc gggagttgag atcggagttt 4860
agagttgttg gcgtagggcg agtttttcgt aaatatttag tgtttaggat gggggtgagg 4920
gatgcgagtt gatggggttt tttttatttg gcgaattaag gattataatt tttttattta 4980
aattttttta gtaaaagtgg ggacggtgtt taaattcgtt ttcggtaggg acgttttttt 5040
gtttttaggg aaagtttggt ggggtgagta gcggcgtttt ttaaatattt tagtttcgga 5100
taatgttttt attagaggtg atgggagaag gggttatttt ttgtttattt tagtttttta 5160
ggtcgggttt tttgtaggta gaagattttt atttttggag ggataggttt tcgtgtcgcg 5220
ggtttttagt cgtttttgta attagtatat ttcgttgggt ttggagagtg tttatcgttc 5280
gttttttacg tcggatacga gttttttgtg gaagggagcg gtcggtaagt tcgtttttgg 5340
attggcgttg ggttggatag taatagttaa ggggaaaagg aaagtattag atagcgtcgt 5400
aggtgcgtaa taaatatttg ttgattgatc gaatagatgt tatttgtgga cgtgttgttc 5460
gagtgagtgt atgttttatt atagtcgtta gattttttag cgatagttgc gaatacgcga 5520
tgtttgtgta tttttgcgtg tgtatttttt tttatacggg agttcgattt tgggaagttg 5580
atcggtatgt gttatttagg gttgtggaat ttcgatttcg ttaaaattgt agattaagag 5640
gttcgtttta gttttttttt tttttttgtt ttcgaaaaaa agaaagatga ggtggacggg 5700
aggtgtcggg tgagggggcg cgaattttta atttgttttg gtgatttggt cgggtttggg 5760
ggcgtcgggc ggttgtaata tagtagggcg tttcgggggt acggatcgga gtttaagtta 5820
gagattagat tagttggttt gtcgtggagt tgcggagtcg attagggaaa aaggaggggt 5880
gggagtggaa ggttttagtt ttaaagggat agcgttttta aagggagcgt gttttttttt 5940
tattttgggt tttgcgaaaa tatttgtttt tagttgtggt atcggggagt cgcgcgttat 6000
gcggtttttt ttaatttatt tttagaacgt tttgcggaaa attttttagg ttattttttt 6060
tttttaacgt ttatttttat tattttaaaa agttttttag gtatttttag ttgaaggtga 6120
ttttgttttg gtgatttaaa agtaaagata tagtaagcgt ttaacgtagt ggtgtttttt 6180
ttgcgatata ttcggtcggg gttatttcgg ttttaagagt ttttttcgta cgagtaattg 6240
tttttttttt tcgattattt tttagtttag gtttatgtag ttttaggtat cgattaaaga 6300
gggttgagat ttttaggatg gttttgtggg gtttttttag gggagggtag gtttaatggc 6360
gatgattgta ttttagtttg gagggatttt aattagcgtt tttttttaag agattttatt 6420
gggtgggtga ttatttcgga ttttaattag agaaagtgtt tttattaagt tacggaaata 6480
gaggatatgt ttgtaaggtg taatttcgaa ggtaacggtt agagttaaag attaattatt 6540
ttgttttttt tgcgattttt ttcgttttaa taggattcgg gaagtttcgg gtagagcgta 6600
tttttttggt ttttgtaagt tacggtttta tttagtttcg tgttgggttt cgttttggtt 6660
ttggagcggc gtgagggagt ttcgggggtt gggtcgattg 6700

<210> 323
<211> 6526
<212> DNA
<213> Artificial Sequence

<220>
<223> chemically treated genomic DNA (Homo sapiens)

<400> 323

tgattttatt gatttttatt ttgattttat taggtttttt gtttgggttg agagattgaa 60
tttgtagttg tttttttgtt tttgtgtagt tggaaggtag tttttttatt gtagatttag 120
aattggaggt tttagaggta aagggatagt gtattttgta tgtttcgaat ttagagtaag 180
agtttagggg agggtggtag ttttggtatt ttaggattat tttgttgtag gagtaggggt 240
aggtagttta gtagtatcgg ttttttagga gtgggtttgg atttgttttt tttaaggttt 300
tagggtttta ttttagtttt gttattagta ggtacgtggt tttgggtata tgattgtatt 360
tttgggattt agggttttta tgtgtaaagg agggagtgga ggggagagag tagtatgttt 420
tttcgaaggc gcgatagagt agaagtaggt agaggggaag ttgcggtttt ataaggtaaa 480
tttatggttc gaggggaagt tgatagttag gttaggtgtt ttttagtagg gatggggagg 540

gcggaggcgg tatagttgga gttcggattg tggtacgtcg ttatcgtgtt gtttcgggga 600
atttcgattc gttttttgcg aaagcgtttt cgaacgcgaa tttagagttt gtgaacgcgt 660
cggtaagttt tttatttttt tatcgtcgtt cgtcgtaggt gggttcgttt taggggtttt 720
ttttgcgttt tatttcgttt tttaagttat ttagtcgatc gtttcgttta cgttttcgtg 780
attttagata tttataatta cgatttcgtt tttatgtata tttaacgtac gacggtgtat 840
agacgtgtat ttgatttttg cggattaggt gttttaagcg tatagcggtt attcgcggaa 900
tcgcggttcg gggttagtga gtcgtttgta gtttttcggg ttaggggatt ttttagaatt 960
tcgggaaagt tattcgaagt ttattcggga tgcggttttg gttttcggtc gcgtgatttc 1020
gtttttaggt gtagaacgac gtttttttag ggtttatagt tgttaggttg ggttttgttt 1080
tcgtattttt cgggaagatt agggacgggg ttatataggt cgtggttgcg gagacgtttt 1140
ttcgggttat ttcgcgatta ggagggagtc gagtcgttcg tttttttcgc gtttttcgtt 1200
ttattttagt tagttggttt tattttgcgc gggataattg ggacgggagg gaggcgacgg 1260
gagggcggcg gttttagaga gattgcgcgt ttgttagcgg taatttgttt aagtggacgg 1320
gacgggtcgg gtcgttgcgg gttggggtta tcgaggtcgc ggtttttatt ttaattagat 1380
ttgggatcgc gggggagttc ggttcggtcg ttaaatcggg ttggttggcg ttagggtttc 1440
gggaggtgcg gttcggcggg gaagtcgtga tgggaagcga ttttgtttag ggagtgtttt 1500
ttattattat attttttacg tttaggtagt gatcgacggt ttggcggtat ttttatagcg 1560
ggtttatagt acggggttat atacgttttt tgagtttagt ttgggtatat tcgtttgtta 1620
tcgagggttt aagttagttt gtagttcgcg tttcgttatt cggacgtaag ttcgtcgttc 1680
gttttgttac gcggtcgtat agttcgagtt ttttgttgtt tattgttcgc ggggttacgt 1740
aatttcggtt ttgtatatag aggaggaagc ggcggggatt ttttgttatt tggaggttta 1800
attcggggaa ggttttagta gtgtttttta attttgcgag aggtgacggg gtcggaggac 1860
ggaagggtag ttacgttagg tttaggatcg cggttttatt cggtttaggt cgttttcgtt 1920
ttcgttttat tttattgttg cgggaagcgc ggggcgtata gatcgcgcgt tattagttta 1980
agttcgttcg acgttttggt cggagatcgt ttgcgagagg gaggggcggt cgagcgcgcg 2040
tttttcgttt gtttgcggga tttggtcgat gcgaagtgat aggggtcgga gttttgttgt 2100
ggagtttcgg tcgtttggcg ttagtcgttt tcgttcgcgt tttttttttt tagtttcggg 2160
cggttcgcga ggcgtttttc ggggtttttt aaaggaatac gtatattcgg ttttcggggt 2220
ttcgttaggg gagggggttt ttgggaaggg ggagggggtc ggtttaggtg ggggaggggt 2280
ttagcggagg ttttacggcg tcgttttagg gttttttagg tttttttagc gttagttttc 2340
ggtgagtttc gggtattttt agttggagag tttggttggg ggtcgttata gattttttag 2400
agcggttgta gtaggatttt agggtgatcg cgggatcgcg gggcggggcg agtggtggga 2460
ttttggttcg gatagagttt cggaaagtta gagggaaggt cggtgttatt gtaggtgggg 2520
tcgtcgtttt tagatgtagt cgttgtattg tcgtcgttag gtttttcggt taaataattt 2580
aggtttgagt gttttaaatt ttttaggttt cggatttttg gattagttat ggatggatta 2640
atgaaatgga ttaatgttta aaacgtatat atttagatgt atatatattt ttataaggat 2700
aaaagtatgt gtttatatat atatatatat atatatatat atatacgcgc gcgcgcgcgc 2760
gcgcgatttt gcgaatgtat ttacggattt ttaggtcggt atttgggttt tcggttaagg 2820
aaagttgttt tggttattat ttggttattt tatcggttgc gtaaacgtgt ttataacggg 2880
ttttttttcg agaggttata tttcgtttaa ggtggagtta gtaggtattt gtttgtggaa 2940
aattgtagtg gattttgttt cgtttgcgta gattgcgtag ttcggagtta aagattcgtt 3000
ttggttagag aggaggtatt aagagaggtg cgatttagtt aagaacgagg tcgtggagaa 3060
aattttgtgg gggcggcgtt tatatatgcg gtgatgagga gttgtgaggg ttttggggtc 3120
ggtttcgaaa agtagggata acggtcgtag aggttatatt gttttcgtcg gtttcgaatt 3180
cgtattgtta ggagagattc ggtttttaaa cgttgttttt aacgtttaat aacgaagtgt 3240
tttgcgatcg gtgtgcgtag tgggcgggcg tttgtatggg ttaatgttgg atgtacgcgt 3300
ttattacgcg gttgtacggg tgggaatgtt attcgtggtt agtgtatgtt ttgttttttt 3360
ttttagaaaa cgatttttag gaattagttt gagtgttcgc gttcgagttc gattggaagt 3420
aggtgcgtgg tcgttttatt tttttcgtgt atattttgag ttatagtttt cgttgcgtat 3480
agagggtatt atacggggtt cgatatatat atatatatat atatatatat atatatatat 3540
atatatatta ttattttatg ggggggaaat tgaggttgtc ggaagataga aaacgaagat 3600
tgattataga agagggggtt ttaattatag gtgttcgtag atattttaat ttttttacgc 3660
gttatttagt attttttaat ttaggagacg tgcgttttcg agagatgaga agaaattaaa 3720
aaagtggttg gtagggcgaa gagaggaagt cggtatttcg gaagaaggag attttcgttt 3780
tcggtatcgg atagggcgaa aattcgttgg gtcgcgggtg gagaattcgg ggttcgttcg 3840
cgcggtgtcg ttttgtgttt agcgaaaggt gcgtttattt ttttggtttc gaggattcgt 3900
ttttttcggt cgttggaaag tttagggcgg ggttcgggtt tgcggagaat tttaggtttt 3960
tttcgagttt ttcggtgaag gcgggcgcgg gtagggggtt ttcgcgtttt tttagcgtaa 4020
ggtcgcgttt cgttttcgtt cgcgtcggtt ttagtagggt tcggttatgg ttttatttat 4080
tagttttcgt tatttgggat tcgttttcgg atttcggtcg ttcgagtttt tcgattcgtt 4140
cgtttatcgg tttcgttttt tagtgtttgt tgtttttttt cggggcgtag ggatttttag 4200
gcggcgttac gattcggagt tgggtcggag gttaggttgg ggttcgggtt agagcggtcg 4260
ggtttttggg cgcgggagaa ggagggcgtt tttttttcgt tcggggtcgt gttaatgttt 4320
tgtatttggg gtcggcgtgc ggttgcgggt ttttttttaa ggtttcggga ttcggttttt 4380
tttcgtttta ggttttttcg gcgggttagg tcggtttttc gcgtttttta gttcggcgta 4440
ggtagtcgta ggcgcgggcg ggcggtgggg gtcggggtag gaggaggggt ttcggggttc 4500

ggcggttcgt tattggttaa tattttattt tgttgatatg tttttttatt gttgaggttt 4560
tcgatatttt tttttaggtt ttttttttcg gtcggagttt ggtttgagtt gttaaaattt 4620
cgttttcgga gatttataat tggtttaaaa agcgtaaaat tagtaattaa ggggggtttg 4680
gttcgttagc gtaggggatt cgagttgggt aggatatgtg agatagttat agttttttag 4740
agattacgat aagatttaat tagtcgcgcg tggttttcgg cgtcggagcg ggttagttta 4800
gttcggttta gttcggtttc gcgtagagtt ttcgtcgttt tcgcgtatag agtcgggtgt 4860
tttttgcggt gcgtcggacg ggaagtttcg aggagtagtt gttgcgttcg ttattcgggt 4920
cgttcgttta tcgcgcgcgt cgtcgttcgg gtcgggggtt cgagtcgcgc gttttcggtt 4980
tcggtttcgg ttttcgggcg tttgggtcgg atgtttcgat gagagagtcg gcgttggcgg 5040
ttagcgttat ggtttattat tcgttttacg cgttacggtt cgtcgatttt tttatgttcg 5100
tttttttggc ggcggcgtag tttttttttt tttcggtatt cgcgttgtcg ttcggcgcgt 5160
tggttaagtc gttgttcgat tcgggtttgg cgggggcggc ggtcgcggcg gcggcggcgg 5220
tagtagcggt cgaggcgggg ttgtacgttt cggtattggg ttcgtattcg ttcgtcgcgt 5280
atttgcgttt ttttaagagt ttggagttcg aggacgaggt ggaggacgat tttaaggtga 5340
cgttggaggt taaggagttg tgggattagt tttataagtt aggtacggag atggttatta 5400
ttaagttcgg gaggtagggt tgtcggtcgg ttggaaggcg cgcgggcggg cgggcgggtt 5460
ggggtacggg attgtacgga ttagagtaga gttggggatt ttcggttttc ggttttcggt 5520
tttaggtttt ggttttgacg ttacgtttcg tttttacgga taattaagtt gatttttttc 5580
gtttggtatc ggagcgattt ttttttaaaa taaaaacgtt aaaatttttc gaatgaaata 5640
aaacgaaaat atattgttaa agaatagttt taatcgtttt gagttttagc gtagaggagg 5700
tttcgcggtt tggttttggc ggtttttttc gtttcgcgtt ttcgtttttt tgcgttcggg 5760
ttcgttttcg agttcggggg aaatttagtt ttttttagat tttgtttcga tttcgaagtt 5820
tttagtggga tttgggttta gttaatttta cgttggttgt tcggtttttg gtagcgagtt 5880
tcgggttagt cgagtttttc gttttatcgg gttgggagtt ttttttttat cgcgggtatt 5940
cgagttttgg acgtattcgt cgttttaggt tttaaagttt gaagaaagtt atagtttcgg 6000
tttttttatt tttcgattta gttatcgaga atttaggttt cgggtttatt tttttttttc 6060
gaattgtacg gttaggatgt tttattaggt tttgtagttt agtttttaat ataaatattt 6120
tttagattta tttttgggag ttttcgtttt aagtgggtat tttttttgaa cgaatttcgg 6180
ggagattaag gagaagcgag aatattttta gagggtttag attttttttt tgggtattag 6240
tttttatttt agattaggga gaaagagggt ttttagattt gagtataagg tttaaaggag 6300
gttgtttagg agagggtttg ataggtagaa atgggatttt ttgggagtaa taattatagg 6360
tggggtcgtt cgggtagtga tgagagggta gagtagtcga ttatagggga aatagttagg 6420
cggtagcggt gtgcgtaacg aggagggata aataaaggag gagtggggtt ttggaattta 6480
gaatagtcgg ttttaatggg attttttttt tttttttttt tttagg 6526

<210> 324
<211> 6526
<212> DNA
<213> Artificial Sequence

<220>
<223> chemically treated genomic DNA (Homo sapiens)

<400> 324

tttgggaggg agggaaggga ggagatttta ttggaatcgg ttgttttagg ttttaggatt 60
ttattttttt tttatttatt ttttttcgtt gcgtatatcg ttgtcgtttg gttgtttttt 120
ttgtggtcgg ttgttttgtt tttttattat tgttcggacg attttatttg tggttgttgt 180
ttttaggaga ttttattttt gtttgttaga ttttttttta aatagttttt tttaggtttt 240
gtgtttagat ttggggattt tttttttttt tgatttgaga tgaaagttga tgtttaggag 300
agaagtttag gtttttaga aatatttcg tttttttta atttttcga aattcgttta 360
ggggaaatat ttatttggga cggaagtttt tagaaataaa tttggagggt gtttgtattg 420
ggaattgggt tatagagttt gataagatat tttggtcgtg tagttcgggg gaagagggtg 480
aattcgaggt ttggattttc ggtggttgag tcgaagggtg gaggggtcgg ggttgtggtt 540
ttttttaggt tttaaggttt ggggcggcgg atgcgtttag ggttcggatg ttcgcggtgg 600
agaaagggtt tttagttcgg tgaggcggag ggttcggttg attcggggtt cgttgttagg 660
aatcgagtaa ttagcgtggg gttagttggg tttaggtttt attaggggtt tcggggtcgg 720
agtagagttt gagagaggtt gaattttttt cgagttcgga gacggattcg gacgtagaag 780
agcgagagcg cggggcggag gagatcgtta gggttaagtc gcggggtttt ttttgcgttg 840
ggatttagaa cggttggggt tattttttag tagtatgttt tcgttttatt ttattcggag 900
gattttagcg tttttgtttt aaaaaaaaat cgtttcggtg ttaaacgaga aaagttaatt 960
tggttgttcg tgggagcgaa gcgtggcgtt agggttagaa tttggagtcg ggagtcggga 1020
gtcgggagtt tttagttttg ttttgattcg tgtagtttcg tgttttagtt cgttcgttcg 1080
ttcgcgcgtt ttttagtcgg tcggtagttt tatttttcgg atttggtgat gattattttc 1140
gtgtttagtt tgtggaattg gttttatagt tttttggttt ttagcgttat tttggggtcg 1200
ttttttattt cgttttcggg ttttaggttt ttgagggagc gtagatgcgc ggcgggcggg 1260

tgcgggttta gtgtcgagac gtgtagtttc gtttcggtcg ttgttgtcgt cgtcgtcgtc 1320
gcggtcgtcg ttttcgttag gttcgggtcg ggtagcggtt tggttagcgc gtcgggcggt 1380
agcgcgagtg tcgggaagaa ggagggttgc gtcgtcgtta gaaaggcgga tatggggaag 1440
tcggcgggtc gtggcgcgtg gaacgggtgg taagttatgg cgttggtcgt tagcgtcggt 1500
ttttttatcg ggatattcgg tttaggcgtt cgggggtcgg ggtcggggtc gggggcgcgc 1560
ggttcggatt ttcggttcgg gcggcggcgc gcgcggtgga cggacgattc gggtggcggg 1620
cgtagtagtt gtttttcggg gtttttcgtt cggcgtatcg taaggggtat tcggttttgt 1680
gcgcgggggc ggcgggggtt ttgcgcgggg tcgggttggg tcgggttgag ttggttcgtt 1740
tcggcgtcgg ggattacgcg cgattggtta gattttgtcg tgatttttgg aaaattgtga 1800
ttattttata tgttttgttt agttcggatt ttttgcgtta acgagttagg ttttttttga 1860
ttgttgattt tacgtttttt ggattaattg tgggttttcg ggggcggggt tttgatagtt 1920
taggttaagt ttcggtcggg agggggggtt tgggagaagg tgtcggaagt tttagtagta 1980
agaaaatatg ttaatagaat aaaatattaa ttaatgacgg gtcgtcgggt ttcgagattt 2040
ttttttttgt ttcggttttt atcgttcgtt cgcgtttgcg gttgtttgcg tcggattgga 2100
gagcgcggag ggtcgatttg attcgtcgaa agggtttgag gcgaggggag ttcgggtttc 2160
ggggttttgg ggaaggattc gtagtcgtac gtcggtttta agtgtaaagt attgatacga 2220
tttcgagcgg agagggggcg tttttttttt ttcgcgttta gaggttcggt cgttttggtt 2280
cgagttttag tttggttttc gatttagttt cgggtcgtgg cgtcgtttgg gggtttttgc 2340
gtttcggaaa gagatagtag gtattggaaa gcgaggtcgg tgggcgggcg agtcgggagg 2400
ttcgggcggt cgaggttcgg gagcggattt taggtggcgg gagttggtga gtggagttat 2460
ggtcgagttt tgttggggtc ggcgcgggcg ggagcgggac gcggttttgc gttggggaaa 2520
cgcggggatt ttttattcgc gttcgttttt atcggaagat tcggaaggaa tttggagttt 2580
ttcgtaggtt cgggtttcgt tttgagtttt ttagcggtcg ggaggagcgg attttcgggg 2640
ttaggaaggt gagcgtattt ttcgttgagt atagggcggt atcgcgcggg cggatttcgg 2700
attttttatt cgcgatttaa cgaattttcg ttttattcgg tatcggagac gaaagttttt 2760
ttttttcgaa atatcggttt ttttttttcg ttttgttaat tattttttta gttttttttt 2820
atttttcgga agcgtacgtt ttttaagtta aggagtgttg agtaacgcgt gagggagttg 2880
gggtgtttgc gagtatttgt gattgaagtt tttttttttg tgattagttt tcgttttttg 2940
tttttcggta gtttagtttt tttttttatg aagtgatggt gtgtgtgtgt gtgtgtgtgt 3000
gtgtgtgtgt gtgtgtgtgt gtgtcgggtt tcgtgtggtg ttttttgtgc gtagcgagag 3060
ttataattta aggtgtgtac ggggagagtg aagcgattac gtatttgttt ttaatcgggt 3120
tcgggcgcga atatttaggt tgatttttga aggtcgtttt ttgaaggaaa agatagagta 3180
tgtattagtt acgggtgata tttttattcg tataatcgcg tggtgggcgc gtatatttaa 3240
tattgattta tgtaggcgtt cgtttattgc gtatatcggt cgtagggtat ttcgttgttg 3300
aacgttggga gtagcgtttg aaggtcggat tttttttggt agtgcggatt cgggatcggc 3360
ggaagtagta tggtttttgc gatcgttatt tttatttttc ggggtcgatt ttagggtttt 3420
tataattttt tattatcgta tatgtgagcg tcgtttttat agggtttttt ttacgatttc 3480
gtttttggtt aggtcgtatt ttttttagta tttttttttt ggttagaacg aatttttgat 3540
ttcgagttgc gtagtttacg tagacggggt aggatttatt gtagtttttt ataggtaaat 3600
atttgttggt tttattttag gcgagatgtg gtttttcggg gagggattcg ttgtggatac 3660
gtttgcgtag tcggtgaggt gattagataa taattagggt agtttttttt agtcgggaat 3720
ttaggtatcg gtttaggaat tcgtgaatat attcgtaaag tcgcgcgcgc gcgcgcgcgc 3780
gtgtgtgtgt gtgtgtgtgt gtgtgtgtgt gtagatatat atttttgttt ttgtaagagt 3840
atgtgtatat ttaaatgtgt gcgttttaga tattagttta ttttattagt ttatttatag 3900
ttggtttagg ggttcgaggt ttgaagagtt tgaaatattt aggtttgggt tatttgatcg 3960
agaaattggg cggcgatagt gtagcggttg tatttgggga cggcgatttt atttatagtg 4020
gtatcgattt tttttttggt ttttcggggt tttgttcggg ttaggatttt attattcgtt 4080
tcgtttcgcg gtttcgcgat tattttgagg ttttgttgta gtcgttttga agagtttgta 4140
gcgattttta gttaaatttt ttagttagga atgttcgagg tttatcgaaa gttggcgttg 4200
gggaggtttg ggaggttttg gagcggcgtc gtgaggtttt cgttgggttt tttttttatt 4260
taggtcggtt tttttttttt ttttaggaat tttttttttt ggcggagttt cgggggtcga 4320
gtgtgcgtgt ttttttaaga ggtttcgggg ggcgtttcgc gggtcgttcg gggttggagg 4380
gggagggcgc gggcgggggc ggttggcgtt aggcggtcga ggttttataa taaagtttcg 4440
gtttttgtta tttcgtatcg gttaggtttc gtagataggc gggggacgcg cgttcggtcg 4500
tttttttttt tcgtaggcgg ttttcggtta gggcgtcggg cgggtttggg ttggtggcgc 4560
gcggtttgtg cgtttcgcgt ttttcgtaat agtgaaatgg ggcgggagcg gggacggttt 4620
gggtcgggtg gggtcgcggt tttgagtttg gcgtggttgt tttttcgttt ttcggtttcg 4680
ttatttttcg tagggttaag gggtattgtt gggatttttt tcgggttgag tttttaggtg 4740
gtagggggtt ttcgtcgttt ttttttttgt gtgtagggtc ggggttgcgt gatttcgcgg 4800
gtagtgggta gtaggaagtt cgggttgtgc ggtcgcgtgg tagagcggac gacggatttg 4860
cgttcgagtg acggggcgcg ggttgtagat tggtttaagt tttcggtggt agacgaatgt 4920
gtttaggtta agtttagggg acgtgtgtgg tttcgtgtta tgggttcgtt gtgagggtgt 4980
cgttaggtcg tcgattattg tttggacgtg aggagtgtgg tggtgaagga tattttttgg 5040
atagagtcgt tttttattac ggttttttcg tcggatcgta tttttcggag ttttggcgtt 5100
agttagttcg gtttagcggt cggatcgggt tttttcgcgg ttttaggttt ggttggggtg 5160
ggggtcgcgg tttcggtgat tttagttcgt agcggttcgg ttcgtttcgt ttatttaaat 5220

aaattgtcgt tgataggcgc gtagtttttt tggagtcgtc gttttttcgt cgtttttttt 5280
tcgttttaat tatttcgcgt agggtggggt taattggttg ggatggggcg ggggacgcgg 5340
ggagagcggg cggttcgatt tttttttggt cgcggggtgg ttcggggaag cgttttcgta 5400
gttacggttt gtgtggtttc gtttttggtt ttttcggggg atgcgagggt aaggtttagt 5460
ttggtagttg tgggttttgg aagggcgtcg ttttgtattt aagggcgaga ttacgcggtc 5520
gagaattaag gtcgtatttc ggatggattt cgggtgattt tttcggagtt ttggggaatt 5580
ttttaattcg gggagttgta gacgatttat tggtttcggg tcgcggtttc gcgggtggtc 5640
gttgtacgtt tgagatattt ggttcgtaga agttaggtgt acgtttgtgt atcgtcgtgc 5700
gttggatgtg tatggaggcg agatcgtggt tatggatgtt tgggattacg ggagcgtgaa 5760
cggggcgatc gattgagtga tttgagagac ggggtagagc gtaggaagga tttttaggac 5820
gggtttattt gcgacgggcg gcggtggggg agtggggggt ttgtcggcgc gtttataggt 5880
tttgggttcg cgttcggaaa cgttttcgta gggagcgggt cgggattttt cggagtagta 5940
cggtgacggc gtgttataat tcgggtttta gttgtgtcgt tttcgttttt tttatttttg 6000
ttaaggggta tttggtttgg ttgttaattt tttttcgaat tatgggtttg ttttgtggat 6060
tcgtagtttt tttttgtttt gtttttgttt tgtcgcgttt tcggaagggt atgttgtttt 6120
ttttttttta tttttttttt tatatatgag gattttgaat tttagaggtg tagttatgta 6180
tttaaggtta cgtatttgtt ggtggtagag ttgggatggg attttgggat tttaaagaga 6240
gtaagtttaa atttattttt aagaagtcgg tgttgttgag ttgtttattt ttgtttttat 6300
agtaaggtga ttttgagatg ttagggttgt tatttttttt tgggtttttg ttttgaattc 6360
gagatatata ggatgtattg tttttttgtt tttagagttt ttagttttag gtttgtagtg 6420
ggagggttgt tttttagtta tataggggta agggagtagt tgtaaattta gttttttagt 6480
ttaggtagga agtttgatgg agttaggatg gggattagtg gggtta 6526

<210> 325
<211> 6092
<212> DNA
<213> Artificial Sequence

<220>
<223> chemically treated genomic DNA (Homo sapiens)

<400> 325

agtaggacgt tttttagttt ttatttttta gattagggta ggtattgggt ttttttttta 60
gttttttagg attttaagga gttgtcgcgg aattttggtt ttgggtcgta gtttttttag 120
gaggttttgt taagtttggt attttgtagt tcggtttggg ttttgttaag gttcgagtcg 180
gattcggttt tatttagagg tcggtttagt atttttttgt cgtcgtcgtt tcgtattcgg 240
ttatcgggtt tttttttagt ttttattttt gattttcgcg gtcgatttcg aggcgcgtta 300
ggtcgggttt agcgttcgcg gttttttggg tcgggataag gagggcgggt gagtagaagg 360
gtcgtgttta gggtttggaa gtgtaaggtc gcgtggtggg tatggtaggg aagcggagcg 420
tgggtttgtg aggcgcgtgt gcgtttgcga tttcgggatc ggggttttta aatgaatagc 480
gcgtatagtt gggagtaggg tttggggagc ggggttttgc ggtcggggat tcgtagaagt 540
cgttgtttgg aagcgggcgt cggtaggggg tggttagatt cggtcgtgga gcgttttagt 600
tttcgggtac ggcggggtta ggcgagtttt tttggttgtc gagtttcggt tgagagtcgg 660
gttgttcgtt tttgtttttc gtttttttaa tttaaattat gcggggtttt tagtttttat 720
tcggttcgcg gggttcgagg gttaagagga gggtattggg tcgcgcggtg gaggagtcgg 780
cggttcgcgt ggggttcggg attttgggga tcgcgacgtc ggggaatttt ggagtttttt 840
ttttcggggg atagcggcgg tagattgatt gatagcggcg acgttagcgc ggaggggggg 900
cggttttaga gggagggagg gaggaggagg gaattgaggt tttaaagtgt agttttggag 960
attaagggtt cgttttagag tcgggatgtg tttagtttcg agggtacggt aggtagttgg 1020
attttgattt agcggcgcgg agtcggagtt tcggtcgttt cgcgtttttc ggattcgaat 1080
ttgtagggga tttcggtcgg ttggcgtttt cgtatgcggt atcgtaatta gggtgagtgt 1140
gggttttagt cgagtgggga cgagttgggc gtgcggggtt ggggttatag ggtttagggg 1200
ttaggttgcg gggacggata ggaggatgag ttggaaaggg gtgagttacg cgattcgggg 1260
aagaatttta ggggcggtta aagttgtagg aaggcggcgc gaagtgtttt gggtcgggtc 1320
gtcgtttcgg ttcgatattt agtagttggg gtacgagttt ttttttttga gtaacgcgat 1380
gaattgggtt tagagattta agagtataga aatatcgata gcgttagtat tttaagaata 1440
aggtcgtgat aggcggagcg gtggtttgga aattggtgtt tgtagaatat gtaaggttaa 1500
ggaagagggt ttcggtattg gagcgaaaat tcgtgtagat atgtgagtga ttagaatatt 1560
agatttagag tttttgatgt aatagattag ttaaaattaa agtaaggaat attatatata 1620
aataatgatc gatgattaaa gattattgga atgtgaataa ttattaaaga aagggttaat 1680
cggtataatt gatataatac gtgaattttt aaataatata attatagtta tttttagaat 1740
taagtgaaaa tagtaattat aatttttaaa aggattaaag ataaagtaat tttaaataga 1800
ggaataatgt aaggattgtt tattaaaatt aattgtatta atataatata tagtaatagt 1860
gtaataaatt attaaattgt aggaaattag taaacgaatt gtaggagaaa gatgaataat 1920
gaaaatatta ttaagggaaa tattattagt ggtagtgata taataatata atgtattatt 1980

gatattatag tgaattatta aaagaaatat taattaaaat tttaggagta atagtaatat 2040
aaataaagat atattaataa gaattgttgg agtaatatga taatttatga ttaatgtatt 2100
aatagttata agaatattat aaataatggt tgagtaataa gaagtattgt attaatgtga 2160
tgaaaaataa ttagagtatt aagagttata ataatattat aaagtatgat aaaataataa 2220
gaagtaatga tgtgatgagt aattagcgag tttagatttg taatattata aataatgata 2280
aagtaataag atattgtcgg ggtgtgttga agaataatat ttggagtgtt atgagtaagt 2340
ttaataaaat aaattgtggt tagataataa gaagtattat aaggatgtga gggaaattaa 2400
tatgaatttt gagaataatt atataataag ttatggtaaa taagaagtta ttgaaatagc 2460
gtgataaagt ataattaaaa tgaagagtaa gtaacggaaa cgtattaggg atataaagta 2520
agaataaaaa attataatag attaacgtga tttattgttt attatgttat ttcgatattg 2580
gaaatagtat tatgaatgtg ttaggcggtt ttgaaatgat ggaatagtga taggaaaata 2640
ataaatttag taatagtaag ttaacgaatt aaagtagggt tttaattata tagtattaaa 2700
gatataaagt agtaataatt tacgcgatag gatattttta aagttttcga attgattaaa 2760
atattattat ggtaataagg ataattaaaa tattttaaaa agtacgaaat agtaatagtt 2820
tttttagtgt aagaaatata tttttagtaa tttaaaaatg aaaagagaaa tgttgtcgtt 2880
tttggaaaac gaggtagttg aaagtaatag attttatcgt aaaggtagcg gaggttgcgg 2940
gtcgtattcg cggaggagtg gatgtgaagg acgagagcgg atcgttatgc ggtgttgagt 3000
gcgggatttc gcggtgttgg cggcggtcga gcgcgtttaa gttcgaggtt cgagaggatc 3060
gcgaggacgg tcgtttcggg ttagtcgggc gtggaaaaaa ggaaattgtt gggaaggggg 3120
taagttgttt cgaggtttgg atgtggtttc gtggcggaga gaaaaggaag aggtaaattt 3180
tgagttcggg agggagagta ttgagtaggt ttcgttttta gttcgagttg cgggatcggc 3240
gttagtggac gcgggcggtg aattagtttt ttatagggta gggttgagcg cgtaggattg 3300
agagcgtagg gcgcgagtcg tagggtttcg ttgtacggtt tcgggtgtga taagagttta 3360
gtagaggatt ttatggttat gcgggttaag cgcgagacgg tttttttttg cgatttcgta 3420
ggtcgttata tttgggatta gcggatcgtt tggtcgttgg agtcgatttc gtcggggttt 3480
tagatatagt tggatttagc gcgttggtcg gagcgggtag ttattttatt ggcgtagcgg 3540
ttttgtgtgg ggatttttag gttttcgagg ttcggttcgc gttcgtacgt ggttcgaggc 3600
gggtgttggc gttggttatg tttagcgagt tttatttttt gtaggttgtg gtcgaagatt 3660
ggttcgtttg ttaattttat ttttatgggt tttttttaat ttagattggt ttcggttttt 3720
ttttattttt tgggttgtat ttggtttgtt tattttgcga ggttttggga gtcgaagttt 3780
aagttcggga tgaggaggtt tttgaataag atgggcgaga ggtttgagtt cggggatgtt 3840
tcgggagggt tcggggagag cgggaagaag gtaggaagag aaaggcgggg tatttcgttt 3900
taagaagggt cgttagattt atagaaagtt tcgttggagc gtttaggttt agtaggatgg 3960
ggaagagtag ggaggtggaa aggggaagcg gagtaagcgg ggtatatttt taagggatgc 4020
gggtaggttg gttttttagg ttgtgttttt atttcgaggt tttaggattt gtttcgggta 4080
gggagtaggt atttgagtag gcgagggaat tagttaagat agtcgaagaa atgtagttgt 4140
gttaggtagg gttaagcggt ttttgtgaat gggtttagtt tgttggtttg gttttttatt 4200
gggaagggtg tgggttttta ggtttaaagt agagttttgt ggcgttaaag tgtgtttgag 4260
tgagtttgta ggttcgaatt cgagtattag gtaggggagg tttgggttga gggtagagtt 4320
tggattagtt atttaggaga gtgtggttgt ttaggttggt gggatgttgt gtgaggggtt 4380
gtgtttgggt gtgaggatgt ttgtgttaga acgggaagag gatgtgtgtg gacggggagt 4440
gtgttggagt gaaagttttt ggtttaggaa gtttttattt tagtgttgtt tgaggggttg 4500
ggtgagggta gatgggtgag ttgggtttga ggttttgagg ttttttttgt atataggtgt 4560
ggagttgtag gagtttgggt tttgtttttt agtttggttt tttttttttg tatggttagg 4620
aggtgttttg gttgttttgg gtttatagag aggtttggat tttagttatt ggggaatagt 4680
cgagaaggtt taggttttag gttttatcgg agtttagtag gagtgaggga ttcggggtgt 4740
agttgtggta atgtcgagag ggcggttttt tttttagatt tatttattat agaattttat 4800
atttaggggt aggtagatat tgaattgtcg tagatttttt taggtttata gtttgaaggg 4860
aggaggcgag ggatttgttt tttttgcggg tttttttttt tagtttagga gggggcgggg 4920
tttacgtgtt ttagggttgt tttcgcgttt cgtacgaagt ttcggaatcg gttggagagg 4980
gttaggggtt ttttggcgag tgtggatttg ggcgagtgat tcgttgtgtg ttgtgttcgt 5040
aggagatgtt gtaggataag ggtttgttcg agagcgagga ggtttttcgg gtttcgggtt 5100
tagcgttcgg agaggttagc gtagttaacg ttttcgagtt cgcgttggta gcgtcgggtt 5160
ttagcggagt cgcgttaggt agtttttcgg gattcggggt cgacgtcgtc gtcgtcgtcg 5220
tcgcggagta ggtagggttg cgttagtcgt cgggtagaag tcgggcgtcg gtttgtttgc 5280
ggggtcgttt gtgttcgttt tttcgttcga ttgtcggtag gattcgtttt taggaggatt 5340
tggttgtatt taggacggtt tgttcgttcg ttttttattg gatatggttc gttttttttg 5400
ttttttgggt ttttttttat tttgagtttt cggggttatt ttgggataag taagagttag 5460
agttagagat agaggagagg gcggtaagag agagggagat tttcgtagta tttgagcgag 5520
tttggattaa gtgtttagtt ttaataattc gtagagggtt tgggatttaa cggcgagggg 5580
gtaggaggag gttatttcgt cggtgcgtac gggagtcgtt gcggggattc gaggtttttg 5640
ggacgtcgtc gagggtttta tagtgataat cggacgatta tagcgtacgg gatgtcgttt 5700
ggggatttgt gtgtattatt tgtaggcgcg cgtttggtcg agggtgcgtg cgtttttttt 5760
tgtttgtgtt tgtttcggga gtttgtggcg atggttcgtt atagaaatgt ttatgagtga 5820
gtaggagtgt cgtttaattt gcgttggtta ttatgtgtgt tttttgaggg ttttttcgta 5880
ggtgtagttt taaaggagga agttttagtt atcgattaat ttttttatat ttacgaatag 5940

tttaaaatcg ttttgtgaaa cgaaatttgg agttatgggt ttcgggcggg tagggtttcg 6000
tatagttttt cgggtttggt tttttttttt ttaggtttcg cgtggagttt tgggtttggt 6060
agatataagt tttttttttt tttattttta ga 6092

<210> 326
<211> 6092
<212> DNA
<213> Artificial Sequence

<220>
<223> chemically treated genomic DNA (Homo sapiens)

<400> 326

tttggggatg ggagggagag aaatttgtgt ttgttaggtt tagggtttta cgcggagttt 60
gaggaaagaa gaattagatt cgaagagttg tgcggaattt tgttcgttcg gagtttatgg 120
ttttagattt cgttttatag aacgattttg aattattcgt gaatataaaa gaattaatcg 180
gtggttaaaa tttttttttt taaagttgta tttacggagg aatttttaga ggatatatat 240
agtggttagc gtaggttaga cgatattttt gtttatttat gaatattttt gtggcgggtt 300
atcgttatag gttttcgggg tagatataga tagggagagg cgtacgtatt ttcggttagg 360
cgcgcgtttg tagatagtgt atatagattt ttaggcggta tttcgtgcgt tgtgatcgtt 420
cgattattat tgtgaagttt tcggcgacgt tttagaggtt tcggattttc gtagcggttt 480
tcgtgcgtat cggcggggtg gttttttttt gttttttcgt cgttgggttt taggtttttt 540
acgggttgtt ggagttggat atttggttta ggttcgttta gatgttacgg gggttttttt 600
tttttttatc gttttttttt ttatttttag ttttagtttt tgtttgtttt agagtagttt 660
cgagggttta aagtggagaa ggatttaaaa ggtagagaaa gcgagttatg tttagtggag 720
aacgaacggg tagatcgttt tgaatgtagt taggtttttt tgaaagcgag ttttgtcgat 780
aatcggacgg aaagacggat ataggcggtt tcgtagatag atcgacgttc gatttttatt 840
cgacggttgg cgtagtttta tttgtttcgc ggcggcggcg gcgacgacgt cggtttcggg 900
tttcgggggg ttgtttagcg cggtttcgtt gaggttcggc gttgttagcg cgggttcggg 960
ggcgttggtt gcgttggttt tttcgagcgt tgggttcggg gttcggaagg ttttttcgtt 1020
ttcggatagg tttttatttt gtagtatttt ttgcgggtat agtatataac gagttattcg 1080
tttaggttta tattcgttag aggatttttg gtttttttta gtcgatttcg ggatttcgtg 1140
cggggcgcgg aggtagtttt ggagtacgtg gatttcgttt ttttttgagt tggaggaggg 1200
aattcgtaag gaaggtaggt ttttcgtttt ttttttttag gttgtggatt tgggagggtt 1260
tgcggtagtt taatgtttgt ttgtttttgg gtgtggggtt ttgtgatgag taggtttagg 1320
ggagggatcg ttttttcggt attgttatag ttgtatttcg agttttttat ttttattgag 1380
tttcggtgga gtttgaggtt tgggtttttt cggttgtttt ttaataattg gggtttaggt 1440
ttttttgtgg gtttaggata gttaggatat tttttggtta tgtaggaaag gagagttagg 1500
ttggagggta gggtttaggt ttttgtagtt ttatatttgt gtgtagagag ggttttagga 1560
ttttaggttt agtttattta tttgttttta tttaattttt tagatagtat tgaggtagag 1620
attttttgaa ttaaaggttt ttattttaat atatttttcg tttatatata tttttttttc 1680
gttttggtat agatattttt atatttaaat atagtttttt atatagtatt ttattagttt 1740
gagtagttat atttttttgg gtgattgatt taggttttgt ttttagttta ggtttttttt 1800
atttggtgtt cgagttcgaa tttataggtt tatttaggta tattttagcg ttatagggtt 1860
ttgttttggg tttaaagatt tatatttttt ttagtgaagg attaggttag taggttgggt 1920
ttatttatag gaatcgttta gttttgtttg gtatagttat attttttcgg ttgttttggt 1980
tgattttttc gtttatttag gtgtttgttt tttgttcgaa gtaggttttg gggtttcggg 2040
atgagagtat agtttgagag gttagtttgt tcgtattttt taagggtgtg tttcgtttgt 2100
ttcgtttttt ttttttattt ttttgttttt ttttattttg ttggatttgg gcgttttagc 2160
ggagtttttt gtaggtttga cggttttttt tgaggcggaa tgtttcgttt tttttttttt 2220
attttttttt cgtttttttc gggttttttc ggagtatttt cgggtttagg tttttcgttt 2280
atttgtttta gaggtttttt tatttcgggt ttagatttcg atttttagga tttcgtaaag 2340
taaataagtt aagtgtaatt taggagataa aaaggggtcg ggttagtttt gggttgggaa 2400
aaatttataa agataaggtt gataggcgga ttagttttcg gttatagttt gtagggagtg 2460
agattcgttg gatatggtta gcgttagtat tcgtttcggg ttacgtgcga acgcgggtcg 2520
agtttcggaa atttaggagt ttttatatag ggtcgttgcg ttagtagggt agttgttcgt 2580
ttcggttagc gcgttgggtt taattatatt tagggtttcg gcgggatcgg ttttagcgat 2640
taagcgattc gttggtttta gatgtggcgg tttgcggggt cgtaaggagg ggtcgtttcg 2700
cgtttggttc gtatggttat ggggtttttt gttgggtttt tgttatattc ggagtcgtgt 2760
agcggagttt tgcggttcgc gttttgcgtt tttagtttta cgcgtttagt tttgttttgt 2820
gaggagttgg tttatcgttc gcgtttattg acgtcggttt cgtagttcgg attggggacg 2880
aggtttgttt agtgtttttt ttttcgggtt tagggtttgt tttttttttt ttttttcgtt 2940
acgaagttat atttagattt cgggatagtt tgtttttttt ttagtaattt tttttttttt 3000
acgttcggtt ggttcggggc ggtcgttttc gcggtttttt cggatttcga gtttgggcgc 3060
gttcggtcgt cgttagtatc gcggggtttc gtatttagta tcgtatagcg gttcgttttc 3120

gttttttata tttatttttt cgcgggtgcg gttcgtaatt ttcgttgttt ttgcgatggg 3180
gtttgttgtt tttagttgtt tcgtttttta aagacgatag tatttttttt tttattttta 3240
ggttattggg gatatgtttt ttgtattagg ggaattgtta ttatttcgtg ttttttaaaa 3300
tattttaatt gtttttatta ttatagtagt attttgatta gttcgggaat tttgagaata 3360
ttttatcgcg tggattatta ttattttgta tttttaatat tgtatggtta aagttttgtt 3420
ttgattcgtt agtttattgt tattaaattt gttgtttttt tgttattgtt ttattatttt 3480
agggtcgttt ggtatattta taatattatt tttaatatcg aggtgatata gtgaataata 3540
ggttacgtta atttattgta atttttattt tttgttttgt atttttaata cgttttcgtt 3600
gtttgttttt tattttgatt atattttgtt acgttgtttt aataattttt tgtttattat 3660
aatttgttat ataattgttt ttaaaattta tgttaatttt ttttatattt ttataatatt 3720
ttttgttatt taattataat ttgttttgtt aggtttgttt ataatatttt aggtattatt 3780
ttttaatata tttcgatagt attttgttat tttgttatta tttgtaatgt tatagatttg 3840
aattcgttag ttatttatta tattattatt ttttgttatt ttgttatatt ttgtaatatt 3900
gttgtagttt ttaatatttt gattattttt tattatattg gtataatatt ttttattatt 3960
taattattat ttataatgtt tttgtgatta ttaatatatt ggttataaat tgttatatta 4020
ttttagtaat ttttgttaat atgtttttat ttatattatt attgttttta agattttagt 4080
taatattttt tttaatagtt tattgtagta ttaataatat attatattat tatattatta 4140
ttattaatag tatttttttt aatagtattt ttattgttta tttttttttt atagttcgtt 4200
tgttagtttt ttatagtttg gtaatttgtt atattattat tatatgttat attagtgtaa 4260
ttggttttaa taaatagttt ttatattgtt tttttattta gaattatttt gtttttaatt 4320
tttttaaaaa ttgtgattat tatttttatt tgattttgaa agtgattatg attgtattat 4380
ttggaagttt acgtgttata ttagttatgt cgattagttt tttttttggt aattgtttat 4440
attttaataa tttttaatta tcgattatta tttatatata atgttttttg ttttaatttt 4500
gattagttta ttgtattaaa ggttttgagt ttagtgtttt gattatttat atatttatac 4560
ggattttcgt tttagtatcg ggtttttttt ttttggtttt gtatattttg taaatattaa 4620
tttttaggtt atcgtttcgt ttgttacggt tttgttttta gggtgttggc gttgtcgata 4680
tttttatgtt tttggatttt tagatttaat ttatcgcgtt atttagagag gagggttcgt 4740
gttttaatta ttgggtgtcg ggtcgaggcg gcggttcgat ttaaggtatt tcgcgtcgtt 4800
tttttgtaat tttgatcgtt tttgaagttt tttttcgggt cgcgtgattt attttttttt 4860
agtttatttt tttgttcgtt ttcgtagttt aattttgagt ttttgtggtt ttagtttcgt 4920
acgtttagtt cgtttttatt cggttggaat ttatatttat tttaattgcg gtgtcgtatg 4980
cggaggcgtt agcggttcga ggttttttgt aggttcgggt tcgaggagcg cgggacggtc 5040
ggggtttcga tttcgcgtcg ttgggttagg gtttagttgt ttgtcgtgtt ttcggggttg 5100
gatatatttc ggttttggag cgggtttttg atttttaagg ttgtattttg gggttttaat 5160
tttttttttt tttttttttt tttttggggt cgtttttttt tcgcgttgac gtcgtcgttg 5220
ttaattagtt tgcgttcgtt gtttttcggg gaggggggtt ttagggtttt tcggcgtcgc 5280
ggtttttaag atttcgagtt ttacgcgagt cgtcggtttt tttatcgcgc ggtttagtgt 5340
tttttttttg gttttcgggt ttcgcgagtc gggtaggggt tgggagtttc gtatggttta 5400
gattagagag gcgggaagta agggcgagta gttcggtttt tagtcgaggt tcgataatta 5460
ggagggttcg tttggtttcg tcgtgttcga agattaggac gttttacgat cgggtttggt 5520
tattttttgt cggcgttcgt ttttaggtag cggtttttac ggattttcgg tcgtagagtt 5580
tcgtttttta agttttgttt ttagttgtgc gcgttgttta tttgggagtt tcggtttcga 5640
ggtcgtaggc gtatacgcgt tttataggtt tacgtttcgt ttttttatta tgtttattac 5700
gcggttttgt atttttaggt tttgggtacg gtttttttgt ttattcgttt tttttgtttc 5760
ggtttagggg gtcgcgggcg ttggattcgg tttggcgcgt ttcgaagtcg gtcgcggagg 5820
ttaggggtga ggattgggag gaagttcggt ggtcgggtgc ggggcggcgg cggtagggaa 5880
gtgttgggtc ggtttttgag tggagtcgag ttcggttcgg gttttgatag agtttaggtc 5940
gggttgtaag gtgttaggtt tggtagagtt ttttggggaa attgcggttt agggttagga 6000
tttcgcggta gttttttgag attttggaga gttgaagagg aagtttaatg tttgttttag 6060
tttaggaagt ggagattggg gagcgttttg tt 6092

<210> 327
<211> 1334
<212> DNA
<213> Artificial Sequence

<220>
<223> chemically treated genomic DNA (Homo sapiens)

<220>
<221> unsure
<222> (93)

<400> 327

tattttaggt ttcgtttttt ttcggttttt attttttttt ttgggtcgtt ttcgtagttt 60

ttcgtcggga tgggatttcg ttgggggcgg ggntgagggg ttagatcgga ataattcggt 120
tattagagcg aattgaatta atttttttat acgagcgtcg ttaggtttgt tttttttttt 180
tttcgcgggg tcgtaggtgg ggaggttcgg gaaaagggtc gggatttttt ggtttcggac 240
gttgtgtaga gatcgaaggt ttggttcgga ttggggtagg atagggattt cggggatagg 300
gtttcgcgta ttttagtttt atatttttga gttttaagac ggcggcgggg atttttcggg 360
gtgggggtcg gggcggtttt gggcggtgtt tgggtttttt aggtttcgtt ttttcggagt 420
tcgtttcgtt tcgttttttt tcgcggggtt ttgcgcgtta aaaggcgcgc gggtcgggtt 480
ggttgtattt gcgttgtatt cgggttgagt gcggtcgcga cggggcgggc ggattttcgg 540
gcgtttagag ttggtttcgg gttttcggag tttggcgttc gaattttttt ttcgattttt 600
attcggattt cgatttcgat tcggtttggt tcggttttcg ttttcgtttt tcgggtcgat 660
ggattatttt tattttaatt cgtacgattc gtgcgtggcg gttatggagg cgttcgttta 720
cggcgatttt ggcgtttgta gttagttcgg cggtttttaa tatagttttt tgcggttcgt 780
ttttttcgcg gtagggtcgt tttgtttcgc gttcggtttt tttaattgcg tatttggcgt 840
tttacgcgat tattagttcg cgttttattc ggtaggtgag cgtagtttcg gtttaatttt 900
ttttttaatt tttgtagaat ttttgggttc gttcgttaag tgaatgggat tgtttttttt 960
agtcgttttt attttgtttt tggttagatc gtttaatgtt tcgagagatt tttagaatac 1020
gaggtttttt ttcgtaggaa tgagttttat tttttttattt tttttgtttt tttttttcgt 1080
tgttaaattt ttgtttcgtg ttttttaggt taaggaaata tatttttatt tagttttggg 1140
ttttttatta ggtttttttt ttattatttg taaagattga ttattaaatt tttataagcg 1200
ttggattttt gaaaaaggtt tttagtttag ttttattatt atttattatt ttattttatt 1260
atcgttgttt tacgagatgt atgattttta gtaaggattt ttttaggtag ttgagttgtt 1320
tagacgggaa gttt 1334

<210> 328
<211> 1334
<212> DNA
<213> Artificial Sequence

<220>
<223> chemically treated genomic DNA (Homo sapiens)

<220>
<221> unsure
<222> (1242)

<400> 328

aagtttttcg tttgggtagt ttagttgttt ggagaggttt ttgttggggg ttatgtattt 60
cgtggggtag cggtgatggg gtggggtgat aagtggtgat agggttgggt tgggagtttt 120
ttttaaagat ttagcgtttg tagaggttta gtggttagtt tttgtaggtg gtgaggggaa 180
ggtttggtaa ggggtttaga gttggatggg gatatatttt tttgatttga agggtacgag 240
atagagattt ggtaacgaga aaaaggaata ggagagatga gagggtgagg tttatttttg 300
cggaggagaa tttcgtattt tgggggtttt tcggggtatt ggacggtttg gttaaggtag 360
gaatgggggc ggttgaaggg gatagtttta tttatttggc gagcgggttt agggattttg 420
taggaattgg agggagggtt gggtcggggt tgcgtttatt tgtcgagtag ggcgcgggtt 480
ggtggtcgcg tagggcgtta agtgcgtagt tggaggagtc gagcgcgggg tagggcggtt 540
ttgtcgcggg gaaagcgggt cgtagggggt tgtattggaa gtcgtcgggt tggttgtagg 600
cgttaaagtc gtcgtaggcg gacgttttta tggtcgttac gtacgagtcg tacgaattga 660
ggtaggagta gtttatcggt tcggggggcg ggggcggggg tcgggttagg tcgggtcggg 720
gtcggggttc gggtggaggt cggaagggag gttcgagcgt taggtttcga ggattcgagg 780
ttagttttga gcgttcgaga gttcgttcgt ttcgtcgcgg tcgtatttag ttcgggtgta 840
acgtaagtgt agttagttcg gttcgcgcgt tttttaacgc gtaggatttc gcggaggggg 900
gcggggcggg gcgagtttcg aagggcgggg gtttaggagg tttagatatc gtttaaagtc 960
gtttcgattt ttatttcgaa gggttttcgt cgtcgtttta gggtttagag gtgtggagtt 1020
gggatgcgcg ggattttatt ttcggagttt ttattttgtt ttaattcgga ttagattttc 1080
ggtttttata tagcgttcga ggttagaggg tttcgatttt tttttcgggt tttttttatt 1140
gcggtttcgc ggaagggagg ggaggtaggt ttggcggcgt tcgtgtgaag aagttaattt 1200
aattcgtttt gataatcgag ttatttcgat ttggtttttt antttcgttt ttagcgaagt 1260
tttatttcgg cggaagattg cggaagcggt ttaggaaagg ggatggagat cgggaagaga 1320
cggaatttgg gatg 1334

<210> 329
<211> 804
<212> DNA
<213> Artificial Sequence

<220>
<223> chemically treated genomic DNA (Homo sapiens)

<220>
<221> unsure
<222> (71, 77, 83, 520, 788)

<400> 329

ggtcgggttt tgggatttgt tggttcgatc gttagtcgtt ttggggtttt gggaataggg 60
aaatgcgcgt ntcgagncgg tgncggtttc gttgttttta gtttttattg agtttgttgg 120
ttattgttta acgtttttat ttacgtttta gtttgtttag taaatcgatc ggattgtgag 180
agttaggtcg ttgttttgtc gttattgagt aaagttaata tttttttagt atatggttta 240
aaatttttgt tagagatcgg gaaaaagaaa taaataaata aataaataat aataaaattt 300
ttaagttttt ttttttaagt ttttttattt ttatttttat tttttatttt ttcgttttta 360
gtaaattttt tagtttggga aaagtgcggc gcgggatagc gcggcggggt gggtggggga 420
tttgttgtcg ggagatgcgg cggcggtcgc gcggttgatt gcgattcggg agtttcggga 480
gcggttcgcg taggtaagtt ttttcgtcgt cgttcggggn tcgggttggt cgtagcgtat 540
cgtcggcggt tgtcgggtag gaggagtttc ggtttttttc gggcggttga aggaggggtt 600
gagttttagg atttttaggt taggtcgtgt tgaaagtaga tatttcgagg tttcgtttag 660
tttgcgttgg agggaagaag gcgttggagg tgcggtagcg ggtagtgggg ggatcgggga 720
attgaaaggt gttagttttt tattttagaa aagaaagaaa gagagaagga gagagagaga 780
gagagagnag agagagagag agag 804

<210> 330
<211> 804
<212> DNA
<213> Artificial Sequence

<220>
<223> chemically treated genomic DNA (Homo sapiens)

<220>
<221> unsure
<222> (17, 285, 722, 728, 734)

<400> 330

tttttttttt ttttttnttt tttttttttt tttttttttt tttttttttt ttttttttaa 60
agtagaggat tggtattttt taatttttcg gtttttttat tgttcgttgt cgtattttta 120
gcgttttttt ttttttagcg taggttgggc gaggtttcgg ggtatttgtt tttaatacgg 180
tttggtttag gggttttgag gtttagtttt ttttttagtc gttcgagggg aatcgaggtt 240
ttttttgttc ggtagtcgtc ggcggtgcgt tgcggttagt tcggntttcg ggcggcggcg 300
gagaaattta tttgcgcggg tcgttttcgg agttttcgag tcgtagttag tcgcgcggtc 360
gtcgtcgtat ttttcggtag taggtttttt atttatttcg tcgcgttgtt tcgcgtcgta 420
ttttttttag gttgggaagt ttgttgaaag cgaggaggtg ggaagtgggg gtggggatgg 480
ggggatttgg gagaggaagt ttaaggattt tgttgttgtt tgtttgtttg tttgtttttt 540
tttttcgatt tttagtaaag attttagatt atatgttggg agaatattag ttttgtttaa 600
tggcgatagg gtagcggttt gatttttata gttcgatcga tttattaaat aaattgaaac 660
gtgggtgggg acgttaaata gtaattagta gatttaatga aaattgaaag tagcgaggtc 720
gntatcgntt cggngcgcgt atttttttgt ttttaaggtt ttagggcgat tggcggtcgg 780
gttagtaagt tttaaagttc ggtt 804

<210> 331
<211> 2357
<212> DNA
<213> Artificial Sequence

<220>
<223> chemically treated genomic DNA (Homo sapiens)

<400> 331

tcgttgcggc ggcggcggcg gcggttatcg aatcgcggtt atagagtttg taatagtaat 60
agagttatgg tttaagttgg ttagcggggc gggtaggtag tagacgcggt aggcgcgcgg 120
gtcgcggtag gggagtcgga gattttagaa ttttaagaaa gagaggggcg agaggtggtc 180

gaggcgggcg ggttggggta ttgcgttttt ttaacggcgc ggattttttt tggaaattaa 240
tattaaaaaa aaaaaagtcg aggacgtaga ggggaaggtg gggggtaaga ggggaaggcg 300
agatatatat atatatatat acgtatacgt atatacggat atatatatac ggagagagag 360
agagagagag atagagtttt atagtgagag gaaggaaggt aatagtcgtt agtagtcgat 420
gtgaagatcg gatttcgtgc gtttttcgtc gtttttgttt ggttatatcg atgttgtgtt 480
cgtcgtttgt tcgttcggat tacgatgaac gcgtagttga ttatggaagc gatcggcgag 540
ttgtacgggg tgagttatga gtcggtgttc gtttttgtcg atttgttggg cggtagtttt 600
tacgcgcgta gtttcgtggc gtatcgcggt agttatttgt ttttcgcgta ttcgcgtttt 660
atgggtatgg cgtttttgtt ggacggcggt agcggcggcg gagattatta ttattattat 720
cgggtttttg agtatagttt ggtcggtttt ttgtatttta ttatgattat ggtttgcgag 780
atttttttag gtatgagtat gtttattatt tatattattt tgattttttt gtagtcgttg 840
ttttttattt ttatagtttc ggataagttt ttttattatt attattatta ttattattat 900
tattattcgt attattatta gcgtttggcg ggtaacgtga gcggtagttt tacgtttatg 960
cgggatgagc gcgggttggt ttttatgaat aatttttata ttttttatta taaggacgtg 1020
gtcggtatgg gttagagttt ttcgtttttt tttagtttcg gtttgggtag tatttataat 1080
ttttagtaag ggttttttta ttatgtttat tcgggggtcg ttatgtttat cgataagatg 1140
tttattttta acggtttcga agtttattat tcggttatgt tcggtcgtta cggggagtag 1200
tattttacgt ttatttcggt cggtatggtg tttattaacg gttttttttc gtattatttt 1260
tacgtttatt tgaacgttta gggttacggg taatttttgg gtatagttcg ggagtttaat 1320
ttttcggtga tcggcgcgta ggttagtaat ggaagtaatt tagggtagat ggaagagatt 1380
aatattaaag aggtggcgta gcgtattatt atcgagttta agcgttatag tattttatag 1440
gttattttcg cgtagagggt gttttgtcgt ttttagggga ttttttcgga tttgttgcgt 1500
aattttaaat tttggagtaa atttaaattc ggtcgggaga tttttcggag gatgtggaag 1560
tggttgtagg agtcggagtt ttagcgtatg ttcgcgtttc gtttagtagg tgagtcggtt 1620
aaggagttgg gcgcgcggga gataaggtat acgaaagtcg aagggattaa aggaggaagg 1680
gtaggagaga gggttgggtg cgttttattt cgttcgtttt tatttttaga agggggattt 1740
tttgtgtttg gaagggttag tatagggttg tgggagtaaa ttgtttattt tagggtttag 1800
ggtgttgggt tgtggaaggt ttcgagagtt aagcggtcgg gtttcggcga ttgaagaggt 1860
ttatttgcgt ttagggatag cgagggaagt tcgtacggtt tgaggcggag agaaattttc 1920
gtatttggtg agatattttg cgttattttt tagtttagcg attcgggttt gggaagaggg 1980
atgtaacgcg tgcgtggaat cgtacgtaaa tgtttttcgt aggtattttt gatatatttt 2040
aggcgttggg ttggagcgtg tcggagtagc gtcgatgttt ggtcgaattg gttgttgtga 2100
ttttttttcg cgtttatttt tttggcgttg gttggggtga ttttaattta agagtatggg 2160
taaaaagttt tagagttatt gggcggtggg tgtgtagtat agacgagttg taagggttag 2220
gacgtttttt gcgtatggaa ttggttgtgg aaaggagagg gaggttgatc gttttaaggt 2280
tttagtgaga gttgtaaagt aatagttatg ttttgggatc gatttcgttg tcgatttttt 2340
gaggatttttg tattttg 2357

<210> 332
<211> 2357
<212> DNA
<213> Artificial Sequence

<220>
<223> chemically treated genomic DNA (Homo sapiens)

<400> 332

taaaatgtaa agtttttagg gagtcggtag cggagtcggt tttaggatat ggttattgtt 60
ttgtagtttt tattgggatt ttgggacggt tagttttttt ttttttttta tagttaattt 120
tatgcgtaga gagcgtttta atttttataa ttcgtttgtg ttatatattt atcgtttaat 180
ggttttgaaa ttttttgttt atatttttag attgaggtta ttttagttag cgttaaaaga 240
atgagcgcgg gagggggtta tagtagttag ttcggttaaa tatcgacgtt gtttcggtac 300
gttttagttt agcgtttggg atgtgttaga ggtatttgcg aggaatattt gcgtgcgatt 360
ttacgtacgc gttgtatttt tttttttaaa ttcggatcgt tgaattgaga ggtggcgtag 420
agtgttttat taggtgcggg gatttttttt cgttttaggt cgtacgagtt tttttcgttg 480
tttttgagcg taaatgagtt tttttagtcg tcgaggttcg gtcgtttagt tttcggagtt 540
ttttatagtt taatattttg agttttggag taggtaattt gtttttatag ttttgtgttg 600
gtttttttag gtatagggag tttttttttt aggggtaggg gcgggcggga tgaagcgtat 660
ttagtttttt tttttatttt tttttttttg gttttttcgg ttttcgtgta ttttattttt 720
cgcgcgttta gttttttggt cggtttattt gttaagcgga gcgcggatat gcgttggaat 780
ttcggttttt gtagttattt ttatattttt cggaaggttt ttcggtcgga tttgagtttg 840
ttttagggtt tggggttgcg tagtaggttc gagagggttt tttgggagcg gtagagtatt 900
ttttgcgcga agatggtttg tgggatgttg tagcgtttga gttcggtggt gatacgttgc 960
gttatttttt tggtattgat tttttttatt tgttttgaat tatttttatt gttgatttgc 1020
gcgtcggtta tcgaagggtt gggttttcgg gttgtgttta ggagttgttc gtggttttgg 1080

gcgtttaggt gggcgtgggg atggtgcgga ggaaggtcgt tgatgggtat tatgtcggtc 1140
gaggtgggcg tgaggtgttg tttttcgtgg cggtcgagta tggtcgggtg gtgggtttcg 1200
aagtcgttgg gggtgagtat tttgtcggtg ggtatggcgg ttttcgggtg ggtatagtgg 1260
gggagttttt gttgggagtt gtggatgttg tttagatcgg agttggagag gggcgagagg 1320
ttttggttta tgtcggttac gtttttgtgg tagggggtat agaggttatt tatggaggtt 1380
agttcgcgtt tatttcgtat gagcgtgaag ttatcgttta cgttgttcgt taggcgttgg 1440
tggtggtgcg ggtggtggtg gtgatggtgg tggtggtgat ggtgggggaa tttgttcgag 1500
attgtggaga tgggaggtag cggttgtaga ggggttaagg tggtgtaggt ggtgggtatg 1560
tttatatttg ggggagtttc gtaggttatg gttatggtgg gatgtagggg gtcggttagg 1620
ttgtgtttag gggttcggtg gtggtggtgg taattttcgt cgtcgttgtc gtcgtttagt 1680
agggacgtta tgtttatgga gcgcgggtgc gcggggggta ggtggttgtc gcggtgcgtt 1740
acggagttgc gcgcgtgggg gttgtcgttt agtaggtcgg taggggcggg tatcggttta 1800
tggtttattt cgtgtagttc gtcgatcgtt tttatggtta gttgcgcgtt tatcgtgatt 1860
cgggcgagta ggcggcggat ataatatcga tgtggttagg tagaggcggc gaggggcgta 1920
cggagttcgg tttttatatc ggttgttggc gattgttgtt tttttttttt ttattgtggg 1980
gttttgtttt tttttttttt ttttttcgtg tgtgtgtgtt cgtgtgtgcg tgtgcgtgtg 2040
tgtgtgtgtg tgtgtttcgt tttttttttt tattttttat tttttttttt gcgttttcgg 2100
tttttttttt tttaatatta atttttaaag aggattcgcg tcgttgggag agcgtagtgt 2160
tttagttcgt tcgtttcggt tatttttcgt tttttttttt tttaaaattt tgaggttttc 2220
ggtttttttg tcgcggttcg cgcgtttgtc gcgtttgttg tttgttcgtt tcgttggtta 2280
gtttgagtta tggttttgtt attgttatag attttgtggt cgcggttcgg tagtcgtcgt 2340
cgtcgtcgtc gtagcgg 2357

<210> 333
<211> 698
<212> DNA
<213> Artificial Sequence

<220>
<223> chemically treated genomic DNA (Homo sapiens)

<400> 333

atttaattat gtttaatgtt agatagtaat aaagtagaag ttgtcggtcg ggtttcgcgg 60
aaatggcgga gtataatttt ttgaatttcg ggtttgtggg gtcgttggta aatatttata 120
cgggagatat tttttatttt tttaattttc gcgcgttcgg ggcgtagttt ttcgggttgt 180
tttcgttgtt ttatttacgt cgcgataacg tgtgtttttt gttttggtcg tcggcggagt 240
cgtgtaatgg ttattcgtag ttttatttcg gtagtttagt gttttttaat ttttttttcg 300
gtcgtacgtg cgagttggcg cgcgtggagg acggtaaggg ttattatcgc gagtcgtgcg 360
tcgagggtgg cggcgggggt ttgaagcgtg aggagcgcgg gcgcgattcg ggagtcgggt 420
tcggggtagc gttgttttcg ttggagtcgt cggggtcgtt tgcgttcggt tttaagtacg 480
attacgcggc gggcggcggc ggtggcgacg gcggcggcgg cgtaggattt tcgtacgatt 540
cgtttttttg ttagtcgttg gaattcgatt ttagttcgtt tttgtttaac gagggtaata 600
agggcgtcgg cgtaggcgat ttcggtagtt tggtatcgtc gttgaatttc ggcggcgggt 660
tttcggttag cggtaaggat ttcggttatt taagcggt 698

<210> 334
<211> 698
<212> DNA
<213> Artificial Sequence

<220>
<223> chemically treated genomic DNA (Homo sapiens)

<400> 334

gtcgtttgag tggtcggggt ttttatcgtt ggtcgagagt tcgtcgtcgg ggtttaacgg 60
cgatattaag ttgtcggggt cgtttgcgtc ggcgtttttg ttgttttcgt tgagtaggga 120
cgaattggag tcggatttta gcgattggta ggagggcggg tcgtgcggag gttttgcgtc 180
gtcgtcgtcg tcgttatcgt cgtcgttcgt cgcgtagtcg tatttgaagt cgagcgtagg 240
cggtttcgac ggttttagcg ggagtagcgt tgtttcgggt tcggttttcg ggtcgcgttc 300
gcgtttttta cgttttaggt tttcgtcgtt attttcggcg tacggttcgc ggtagtaatt 360
tttgtcgttt tttacgcgcg ttagttcgta cgtgcggtcg aagggagggt tgagagatat 420
tgggttgtcg aggtagggtt gcgggtagtt attgtacggt ttcgtcgacg gttaggatag 480
ggagtatacg ttgtcgcggc gtgggtagga tagcgaaggt agttcgggaa gttgcgtttc 540
ggacgcgcgg aagttgggga agtagaaggt gtttttcgtg tggatgttta ttagcggttt 600

tataaattcg ggatttagga gattatgttc gtttattttc gcggggttcg atcggtagtt 660
tttattttat tgttatttga tattaaatat agttaaat 698

<210> 335
<211> 350
<212> DNA
<213> Artificial Sequence

<220>
<223> chemically treated genomic DNA (Homo sapiens)

<400> 335
   gtgggttata agtagtttcg gttttttttt ttttcgttaa gttgtttgat tttttttttt 60
   aggcgcgcgc gtatatatta tatttatata ttttaaaatt aagattcgtt ttataggatt 120
   tgggaaaaga aaaagaaaaa aaagttttta attattattt ttttttcgtc gatttttttt 180
   tatttttcgt ttttttttta gcgggtagtt aaggagagtt agaggcgggg gaggggagag 240
   ggaggagaag cgacgtaagt gggtagtttt ttagcgtcgg cgaggcgcgg gaggaggaga 300
   agtagtgggg aaggcgtagt cgtttatttg cggggtaggg cgcggaggag 350

<210> 336
<211> 350
<212> DNA
<213> Artificial Sequence

<220>
<223> chemically treated genomic DNA (Homo sapiens)

<400> 336

ttttttcgcg ttttgtttcg taggtgagcg gttgcgtttt ttttattgtt tttttttttt 60
tcgcgtttcg tcggcgttga aaagttattt atttgcgtcg tttttttttt tttttttttt 120
tttcgttttt agtttttttt ggttgttcgt tggaggggag gcggggggtg agggggagtc 180
ggcgagaagg aggtggtgat tgagggtttt tttttttttt ttttttttta gattttgtag 240
gacgagtttt ggttttgggg tgtgtgagtg tggtgtgtgc gcgcgcgttt ggagggaagg 300
attaggtaat ttggcgagag aaggaaggat cggggttgtt tataatttat 350

<210> 337
<211> 1791
<212> DNA
<213> Artificial Sequence

<220>
<223> chemically treated genomic DNA (Homo sapiens)

<400> 337
   atcgagttag tagcgggcgg cgtttagtcg cgttcgtagt tttttttttt ttttagtcgg 60
   ggagagttag tttcgttttt tatatttttt gtcgttagcg atttgtagtt tcgtattgtt 120
   tttttttttt gtattttttt tttagttatt cgagggttta gaaattaagt ttttcggttt 180
   tttcgttatt cgttgggttt tatcgaggta ggtgggtatt cgtcggaggt ttttagttcg 240
   attttgaatt aagcgttttg gattgtttag attcggtggg taaggggatt ggggaggttt 300
   tgcgtatagt cgcgtggaac gggaggggat aagataaatt gttggatatt ttttcgtgga 360
   atgagaagtg gggggtgcgt gggtgggaag gtattttcgg agggaaaggt taaagggaag 420
   gattagaaag agaggaagga agagtcggga aggaacggaa gggaatttag agtcgagggt 480
   ggtggggttg ggttagggga tgcgtattgg gttcggggtc gcgcggttta ggcgggtatt 540
   ggttagtgga tggtagggtt gggcgagtta gaattgagag ttcggtttta tagcgtagcg 600
   cgtttcgagg ttttttgtcg ttatttgaat atttattaga ttgatcgttt tttattttta 660
   tttaacgttt attttatcgg cgagtttcgt tttttagtgt agttttaatt tcgggttttt 720
   attttttttt tttcggttcg tttttttttt tttttttttt ttcgtcggtt gttttttttt 780
   tttttttttt ttattttttt tttttttgtt ttttttttgt cggtatcgga gtgataggtt 840
   cggggttttt ttcgtcgaag ttcggggttt tagcgttggc gaattataga gtggtggaat 900
   ttattgtttt tgtttgataa gttatttatt tttcggcgcg ggagggggga ggaggtttgg 960
   agggggtttt gtagttttta gagagatata tatcgggagt cgaggtttta gttttcggtc 1020
   gagtttttta gtagtcgtaa tttatttggg gttagtttag agttgttagc gtcgttcggt 1080
   tttttttttt tttttcggtt tttcggtcgc ggcggcgtgc gtttgttttt ttcgggggcg 1140
   ggggtttggt tcgcgcgttt ttttttcgta ggcgttattt cggatatttt cgggattgtt 1200
   atttttttgt taatttcgtt aattcgttag tatttggaga ggttcggttt ttttttcggc 1260
   gttttttgat cgttttcgtt tcgcgcgttt ttcgattatc gtttttcgga tgaataggtt 1320
   ttaggggagt tgagcgagtc gtttttttcg tttagtttta gttttggttg tagttgtagc 1380
   gcgagttatg cgtttttagt gtatttcggt tcggtttatc gtttcggggt tattttgttg 1440
   atcgtttagt ttcgagtttc gatagtggta agttgcggtt attgcggttg taagtttcgg 1500
   ttaattcgga ggagttttag cggggagcgt agtgttgcgt ttttcgtttt cgcgcgcgtc 1560
   gtagtagtcg ggcgtttatt tatttttttt tttttatcgt tttttttttt tttttttaag 1620
   ttttgaagtt gagtttgaga ggcgatacgg cggcggcggt cgcgttgttt tcgttttttt 1680
   gtttttttat ggatatgtat tgtaaagtag attttttttt cgcgatgtat cgtgagtatt 1740
   cgcgttcggt ttttgtttcg gttcgggttt ttcgttttaa ttttgtttag t 1791

<210> 338
<211> 1791
<212> DNA
<213> Artificial Sequence

<220>
<223> chemically treated genomic DNA (Homo sapiens)

<400> 338

attggatagg gttgggacgg agagttcgag tcgggatagg agtcgggcgc gggtatttac 60
ggtgtatcgc ggagaagggg tttgttttgt agtgtatatt tatggggagg tagaggagcg 120
ggagtagcgc ggtcgtcgtc gtcgtgtcgt tttttaaatt taattttagg atttgaggag 180
aaaagggagg gacggtgggg gagggaggat gagtgaacgt tcggttgttg cggcgcgcgc 240
gggggcgggg ggcgtaatat tgcgtttttc gttggggttt tttcgggttg gtcggagttt 300
gtaatcgtag tagtcgtaat ttgttattgt cggggttcgg ggttgggcgg ttagtagaat 360
ggtttcgggg cggtgggtcg ggtcggggtg tattgggggc gtatggttcg cgttgtagtt 420
gtagttaggg ttgaagttgg gcgggggagg cgattcgttt agtttttttg gaatttgttt 480
attcgagagg cggtggtcgg agagcgcgcg gggcgggggc ggttagaggg cgtcgggagg 540
ggagtcgggt ttttttaagt gttggcgagt tggcgaagtt ggtagagaag tagtaatttc 600
ggggatgttc gaggtggcgt ttgcgggagg ggagcgcgcg ggttaggttt tcgttttcgg 660
aaaaggtagg cgtacgtcgt cgcggtcgaa gggtcgggag ggagggaggg agtcgagcgg 720
cgttggtagt tttgggttgg ttttaggtgg gttgcggttg ttagaagatt cggtcggaga 780
ttggagtttc ggttttcggt gtgtgttttt ttaaaagttg taaagttttt tttagatttt 840
tttttttttt tcgcgtcggg agatggatga tttgttagat aaaggtaata gattttatta 900
ttttgtgatt cgttagcgtt ggagtttcga gtttcggcga ggagggtttc gagtttgtta 960
tttcggtgtc ggtaagggga gggtagggga gggagaaatg ggagggaggg agggagggag 1020
tagtcggcga aggaagaggg agggagggga gcggatcggg ggagggggaa tgggagttcg 1080
ggattaaaat tatattgaga aacgaaattc gtcgataaga taaacgttag ataaggataa 1140
agagcggtta gtttaatgaa tatttaggta acgatagagg gtttcggagc gcgttgcgtt 1200
gtgaagtcgg gtttttagtt ttaattcgtt tagttttgtt atttattggt tagtgttcgt 1260
ttgggtcgcg cggtttcggg tttagtgcgt atttttagtt ttaattttat tattttcggt 1320
tttgagtttt ttttcgtttt ttttcggttt tttttttttt tttttttggt tttttttttt 1380
ggtttttttt ttcggaggta tttttttatt tacgtatttt ttatttttta ttttacggaa 1440
aagtgtttag tagtttgttt tgtttttttt cgttttacgc gattgtgcgt agggtttttt 1500
tagttttttt gtttatcggg tttgggtagt ttagaacgtt tggtttagaa tcgagttgaa 1560
gattttcggc gagtatttat ttgtttcggt gggatttagc ggatggcggg agagtcgggg 1620
gatttggttt ttgaattttc gggtgattgg gaagggggta taggggaggg aaatagtgcg 1680
gagttgtagg tcgttggcgg tagaggatgt gggagacgag gttggttttt ttcggttggg 1740
gagaagagga ggttgcggac gcggttgagc gtcgttcgtt gttagttcgg t 1791

<210> 339
<211> 12133
<212> DNA
<213> Artificial Sequence

<220>
<223> chemically treated genomic DNA (Homo sapiens)

<400> 339

tatttaatat tttttttttt ttcgtaaaaa aaaaaaaaaa aaaaaaaatt tttatcggtt 60
ttattttttt ttagattttt tgggatgatt aaaaggtttt tgtttataat agattaggga 120
gcgggttatt tagttaaatg ttttttagaa aaaaataaga cgtatggggg attgggagaa 180

tcgttttggg agttcgcggt cgtgtttgga gtgcgcggtt attagtggcg atcgagtggc 240
gtcggtagga ttgtgagttg gtgtgtttag gtcgggttgg gttgatgcgg atacgggagg 300
tagtaggtat tcgggggaaa cgtttatatt ttaaggcgtt gtaaaacggg gaatgtattt 360
gaagtaaacg ggttttgcgt ttgatagaaa attggttatt tttttaagtt gtacgtcggt 420
tttgagtaga gttgaattgt ggtttttaga agtagtgtat tcgttgcgtg tttttttttt 480
tttagtaaag tttttgattt tggggagggt ttgtgttttc gttcggagtt tataagagag 540
gagatagggt ttttcgatat tggttttttt aagggaggat gattgttttt tttttttttt 600
cgaagtttat tgtgaagagt ttgtaatttt tagaattagc gttggttttg ttggtttttt 660
aagttttgga ttaaaggttt tggggtttgt cgtttgtttt tttttcggag ttggttttag 720
ttttggtggg gaggtcgggg ttttagtgag tttttcggcg aggttttaac gagtggcgtg 780
ggtgtttttc gtaggtaatt ttggttttat tagtttgatc gtcggtttat tttgtttata 840
gtgacgttat ttttttcgag gttgaacggg gttttcggtt tatgtgaagt gagagttgtt 900
ttttttaaat gtcgttgttt ttttaaggcg gttttagcgg ttgtatattt ggttgtgcgg 960
gggttttttg gatttggttg gtagtcgttt gcgaaatatc gttttttagc ggtttgtagt 1020
tttttttttt tttgcgttta agatgggttt gataagggtt cgggattaga ttagaatttt 1080
tgggggttga agattttttt ttatttttat tttataaagt gtgtggggtt tgcggagagt 1140
ggttttggat tttaaggcgt taaggtttta gattttgaag tggaagggaa atgcgcgaag 1200
taggagtttt ttgtgtttgt gtagaaggag gtttttcggt cgtcggtttt tgttgtacgt 1260
ttggatagat tttttattgt attggtggta gattttttat ttgtttttta gatttttttt 1320
tattttggtt tggaggtgta tagtgtaatt cggaggagcg ttcggggaag atagttttta 1380
atcgatttta ttcgttttgt ttttaaagag tgatatagtt tagaggtggt ttagaatttt 1440
aattcggggt gattttaaga gtttataggt ttatttaggt ttagggtttt aatttttttt 1500
ttaggagata agagtgtaga tgtaagttat tttttgatta taaatgttaa ttttttgttt 1560
aagttgtttt tttaattttt ttttggaaag aattgtggta gggagaaggg cgttttggta 1620
taaatatggg tatttatttt ttttatggtg taatattgat ttattattaa tggtaattgt 1680
tttaattgat ttaaagatga tggaaaatat tagttatatt aaatatttat aagtaaaata 1740
aaggttggtt tgtagagtac ggttttttta ttttatttgt tttattttag ttttagagtt 1800
ttaggtatat tttttatttt ttggggttcg gattattttt ttgtattatt tttattttgt 1860
ttaatttttt aatgataaaa tattttaaaa tattgatagt tttttgtttt aaggagcggg 1920
tttgaaagta gaaattttaa agataaaaaa tagaattttg tgagaataga tttaaaatta 1980
tgtttgatat atatattttt gtttgtgagt aaaaatttat ttaattttaa ttaataaggg 2040
agtgattttg agtttaaagt tattgaaagt tgaaatggaa aggtttttaa attttgaaga 2100
tatattttgt ttattttggt ttattgtaga tttattcgtt cgtaaggaat tgtttgtttt 2160
tagtttgtat gtttttttta tgtataaata aagattatga aagggtattt gatgggtata 2220
aaattttcga ttagattaaa tagttttgtt ttttttaggg tagggaaaat tgggtaaata 2280
agtttttagt agtttttttg gttaagaggt aaaagggcga tattcgatat tgttggagcg 2340
ttaagttagt gttagggcgt ttgcggtaag cgagtgtagc gggtatttta tttagttttg 2400
ggtacgttta gttttggttt gaaataagat ttttattatg tgagaaagta aattttaggg 2460
cgttggttag tggttgttag ttttgaatag tattttagta atttagagtt aaaagttatg 2520
ttttgttttg ttttgttttt tcggttattt ttaattttta tttttaagtt cgaaaatagt 2580
tatttttgtg gaaaggtaat gtttaaattt taggttatgt tgggatgttg tagttaaagt 2640
agttagaggc gttaggtttt taatttgata atgatgttgt atttgaatag ttgcgtaaag 2700
gtttaaaagg tttgttttat tgttatcgcg gagtttttta aaagaggtta ttgtttgggc 2760
gagagtatcg agagggaata ggcgaaaggg ttttaaggtc gaaaataatg tttaacgttt 2820
gttttaatta ggttagggga gatttttttt tgggaagagg gataaagggt tttttgggaa 2880
aggtttttgt aaaggagcgg gcgggttatt aggtttttta gaatgttgag gcgttttaat 2940
gaattttagg ttttaaagaa atgttttatt gaggtggaaa tttaatttat ttataagttt 3000
agttttgggg tttgaaattt aaggttagtt tatgttttag cgagtacggg gaaaagagaa 3060
tatagatgtt ttttcggatt gagaattggt ttatggttag agtttttggg atcgttttag 3120
ttttttggtt ggtaggaagg gaatttggta ggtcgaagga cggttttaag ttttggtcgg 3180
ttttttattt agggtaagtt tttggaaatt aaggttaaag tttttcgttg ttgtttcgag 3240
gttcgggttt tcgtagggcg gtttattcgt ttgtagtaag agagggaggg aggaagttgt 3300
tgaagttttt tataaaattt tgagttttta tggtggtagt ggtggtagtg gtggtagtgg 3360
tggtagtggt ggttttttaa tgagtcggat tataatttta gattatattt tttttataga 3420
agtttttttt attaaaggaa atagagaatt aagattttta tttttttttt tttttttatt 3480
taagtaaagt tttgttaaat aattttagaa attgtttaaa attgtaaaat ttaatttttg 3540
ataaaagtat agtttttaat atttgataat ttgtattaaa ataaaaatga tttttttggt 3600
tagtagcgaa gggaaaaaaa ttaatagtat aatttttaat aatttataaa gcgaacgtta 3660
ttatggtaaa tttaattatt aattttattt tttataaagt ttcgattgat ttgttaataa 3720
agtatttttt cgtgtgtggt aatagcgggt ataatttttt aaaaattttt agaagtaaga 3780
aaattattaa gtagtttaag aatgtagatg agaattttat tgatcgtttt gatttttttt 3840
aatatgtatt aataaatttt ataatttttt gtattttgta tttgttagaa attagtgttt 3900
ttataaaatt tataaaagga aaatattttt ttttgtagaa gaattaaaat gatatttttg 3960
tatttttttt tgtttttttt ggtttagtta gttttttaaa atttttggat tttgagggtt 4020
agagtttttt ttttttttaa attatttttt tttttttttt tttttgtttg ttttttatta 4080
ttagggtgtg gggagagaaa tttttgttta gtttttttat agggagttcg agggacgttt 4140

tcgcggatag tgaattgttg gtaaagaatt gtttttgggt tgaaagtttt tagaggttcg 4200
gtttcgtaga ggttagggtg tggttttttt gagagtcgaa gtttcgtgta gtttttcggg 4260
gcgtatagga attcggagtt aggtaggagg atcgagcggg aggataacgt agggattaat 4320
ttatttatgg ggattcgggt tcggttagtt gtttggtttt tcggttttcg cggttttttt 4380
tgttttcgtt tttttagaga tattttggtc gttttttagt tttaagggat tttttttttg 4440
gattttgggg gtgggatgtt ttagggttcg cggaattcga tttatttttt cgttgggttg 4500
ttcgggatta aattaattag tttgcgtttt tattcgtttg ttttggattc ggttttttta 4560
agcgcgtttt gtttggttgt gtgtgggggg aatgtttttg tgcgttggcg ttagggtatt 4620
ttggtttttt ttttcgttcg tgcgtgttta tttggaggtt tttagagttg agattttttt 4680
ttcgtcggtt gggtttaggg gtcgaagcgg gggacgcgag tggggcgggt tggtcgagcg 4740
agttttggag aggcggatag gagggcggcg gagagcgttg ggtcggttgt ttttagcgcg 4800
tattatcgcg ggcgcgtagt agatgtcgtt gttgttcgtg tttattcggt cggtcggtta 4860
ggttttggag tacgtgattc gagaggaggt tgcggtttaa ggttattttt aaattttatt 4920
ggtttggttg ttatgtggtc ggtagaggta tttataatta tacggggaat gtttttttag 4980
agatgttagt ttataaagga tataattttt tttttttaaa ttttttttta aaatgttttt 5040
ttttaatagt ttttttgttg ttaatatttt tttagtagat tttttgatta gtgtttgtag 5100
gagtgatagt ttttatttta gtagcgttag tatgtatatg ttattattta gcgtagatat 5160
ggggatttat ggaatgtaaa tttgtggatt gttttcgttt ttggttaaaa gagaagtgaa 5220
ttattaaaat atgggtatga atgtgtattt ttatatattt taagtagata gttggataga 5280
ttcgaataga ttttgtcgaa tagagtaatt tgttatatag taagttttta tttgtttttt 5340
tattattaat attaaggaag aatttaattg ttgtatgtat tttgataagc gtaataaatt 5400
tatttcggtc gaggtttttt cgtattagag gttggttttt gagttttgtt tcgttgagaa 5460
ttttgaggtt ttcgtttttg gatattttag attgagttag atttacgtta tcgggaaaat 5520
ttaagagtat aataatagtt tcgaaggtag ttttattgtt atgttttagt ttaattttcg 5580
tggcgcggtt aagtcgtagt ttttcgttgt ttagttgtag atggaaaaga agatgaacga 5640
gttcgtgagc ggttaggagt ttattaaagt tttttaggtg gagagtttcg aggttaaagg 5700
cggttttttc gaagagagga gttgtttggt tgaggttttc gtgtttagtt tcgaagtgta 5760
ggagaaggaa agtaaaggtc ggtatgagta gagttgttat tttagcgggg cgcgtagttc 5820
gggaattcgg tagagaggga gtgtcggggt gtttagcgtc gagtcggagt tcgatttggt 5880
aggtgttgtt tcgtttggtt ttagaggggt gattttagtt ttgagatagt tttcgttttt 5940
tttttgttgt tttggttttt ttcgttagtt ttggttttac gttgatggcg ttcgggtaga 6000
ggaaaagttt gtcggtttta ttttttttga gttagatttg aatataataa aagagcgtaa 6060
aggagatttg cggtttataa atacgattat agagtttttt tttttttgtt tagatttgta 6120
gttttagttt tgttttgagt ttaatgatta tgttaaggtg atttagggta tcgtgttcgt 6180
gtttaagtgt atgtatttcg tattttgcga gtttgggggt ggtgagggga aagagatggt 6240
tgggttggtt ggtgtttgag ttgggaaata gggtttattg tttttgttgg gttaggtaat 6300
tttggttttg tttaggaaaa gtgttgtagt ttttgtaatt cgtcggtaaa gagggtaaag 6360
gcggaggggg agagtggaat tcgtattgtt ttttttgtaa ggttagtttt agggttgggt 6420
taaggtaaag agttagggat ttggtttttt gagaaggaat tttttttttt ttttttagtg 6480
tttagaggtg ggttatagta gggggttttt tttttggggg aatgttttag tgtgggggta 6540
agaagatatg aaaattaagg aaattttggg gaatgtaata atatttaggt aaggtggggg 6600
aaggtgtttc gtttttttat ttattttttg aaagagaatg ggtatttata aatttgattg 6660
ttaggatttt tgattgttta ggagaggtgg ggaagaagtg gtagatttgg ggatttgagg 6720
tagtagtggg gttggtaggt ttgtttaggt cgtggcgtat tttttttcgt ttttgttagg 6780
agttgaattt ttagaatgtt gttggggaga tttggaaagt ttattatttt attaatgttt 6840
tgtataagtg agaaagttga aagagagagc gagaatttaa atgtagattg ttttgttatt 6900
atgttattta agtaatgtgg tattaatgta agattttttt ttaaggttta ttttatgtga 6960
gtaatgttta atattagtat tttttaaagc ggtttggttg ttagtagggt tacggttaag 7020
ggtatatttg aatagtttga agaaaaaaat aaaaacgaaa attaaaatta aaattaaaat 7080
aaaaataaaa ataaaaataa ataaataaaa aattttttga tttttttttt tttttttttt 7140
taattttgtt agaggaaatt aagtttgata tattaattag taattggttt attgtaaaga 7200
gtggtagaaa gaagaggtgt ttttatatta agtattaaac gttggaatta gaaaaagagt 7260
ttttgtttaa tatgtatttt attcgcgagc gtcgtttaga gattagtaag agcgttaatt 7320
ttatcgatag gtaggttaag atttggtttt aaaatcgtcg aatgaaattt aagaagatga 7380
gtcgagagaa tcggattcga gaattgatcg ttaattttac gtttttttag gtttgaggtc 7440
ggtttgaggt cggttagagg ttaggattgg agagggggta tcgcgtttta gggtttagtg 7500
ttggaggatt gggaaagcgg aaataaaatt tttatcgttt tttgtttgtt gttttgttgt 7560
attttgtttt tttgttagaa tgtgattttg gggttattat gttcgtgttg taagtgattt 7620
gtaattttta tgagtatata tatatatata tatatatata tataaaaatt tagtacgtgt 7680
aatttattat ttttttatcg taatgtaggg taattattat tgcgtatttt tatttgggtt 7740
ttaatttatt ggaattgtag agtatttatt tatttattta tttagtaatg tgattttttt 7800
atgttttttt taatataaaa ggtttatgtg tgtggttagt ttatgaattt atggtatttt 7860
gaatatattt agtattttaa aaatgatata tatatttaaa aaaaaaagat taagaaaatt 7920
tataagttgg agggaggggg atttaaaaag tatattataa tgtatttttt tataaatgaa 7980
tttagtagtt gtttttggtg agatggata ttggcgattt atgttttgta gttttttttt 8040
tgtggtgtat ttgtggtttg gtagaagtat aatagtaatt tgtttttttt gtgtatgttt 8100

tggtcgtatg tataatgtaa taaattttgg aaatgagttt attttttttg ttttttgaaa 8160
tggaaatatg ttatggtgga aatgaaagtt tatggtgaga ttattttttg gttatatttt 8220
ttgtttgggg tatttgggta ggggagtgat agattagtag gggaagggag atgggggaga 8280
aaagttggag gaggtttagg gtgttggatt ttggtagtgg ttgggggaga ggaattataa 8340
gttagtttga gagtgaagtt tttataattg ggaggaaggg gagttttttt tttttttttt 8400
tagtttttag tgatagtaat ataattgcgt ttttaatggg tgtgagtttt tttttggttt 8460
gaatttggta agtaagttta tattttaagt tatttttttt ttaaagtttt ttatttgtgt 8520
gttttttttt ttttggtttt ggttgtgttg tttttaatgt ttttagtggt attttggtga 8580
tttttggttg gcgagtaatt attaggggtt aggttgaagt tagttttgtt tatttggaag 8640
ttgtcggttt ttattatagg agtaaggata aatagtagtg tagtattgta gcggatttaa 8700
ttttgttttt ttttttttag ttttattttt attttaagcg taagatagtt agattttaga 8760
gaagtcgagg atgggtgagt ttttttattt tatttcgttt tgattttttt gtggacgggt 8820
tttatgttta gttattattt tttagtggtt tatatgaaat tttgttaaag gaagaaatga 8880
aaagattttt tttagttagt ttttttttta tttaattata aaatgttggt gggaattgtt 8940
gtatttggga tgtaagaaaa tgtagaaagg gtgattgaaa attttgtaaa tgaatatgat 9000
tttttatgaa gtttaatgtt ttattcgttg ttatggttta ggggattttt attagttttt 9060
atcggtttta ataggatttt tattagagag tttagattta tttagttttg tcggggaaaa 9120
gggagaagag gttttgtagg agaaagttaa tagaaaattc gttatttgag gttttgtttg 9180
tagttttaaa tagtttttag tagttttata aaatatattt tttttatttt ttttttttaa 9240
atgttttttt aagaacgatt tatttaggtg ttataagttt ttagttaggg agtttttggg 9300
atattggtat ttaaaatttt aaattttcgt tttaaaatta ggaatatttt aagatagaat 9360
ttttttaggg ggttattttt tgggggtggg ggagagggtt tggaattaat gttagattga 9420
aaacgttgta atagttttgt tttagattta atatcggttg ggtaggagga gggtaatttt 9480
tatttagtcg ttttttcgat tatgtgggga atattattag ttgttgatag cgggttatgt 9540
attcgaggaa agtttgagtt ataaggtaaa ggtattttat ttggaataaa attagaaagt 9600
tattggtaaa ggtaattaat taggttataa atagttattt attcgttttt ttttcggggt 9660
tggaggtggg tcgggagttt tttaagggtg agttgggtaa tttgtagagt aaggaatatg 9720
tttttcgttg tcggcgtttc ggtcgttttt gtttgggttt ttagtcgggt tttcgaggtt 9780
ttgtattatg gatttgggag tgataatggg tatttttttt agatttaagg ttgtttaatt 9840
ttatttttgt agggggaaaa aaattagaag ggagtgtttt aaggatttag ttattcggtc 9900
gaatttttta gatattttgg gagttttttt cgaggttttt aagtgcgaat cgcgcgaagc 9960
ggttttgttc ggggagattc gttgaggtag ggttgaggcg gcgggcggga gtaagttgtt 10020
ttagtatttg ggttttgttt tgtggcgtgt ttttttttag ggttttttta gtattatcgg 10080
agaagacgaa gtattttggt cgttattgtt cgtgttgcgt taattcgttc ggtcgttcgt 10140
tttttcgagg gtaggtagaa gtttttttgt gttttttatc gtcgcgtttc ggttcgtttt 10200
tcgggtcgcg gcgtgtgttt agttttacgt cggggtgtgt gtggtcgcgc gggcgtgtgt 10260
gagtgtggta gggggagggg gtttttcgat ttgttttatt cgttcgtttt attagggata 10320
tattaattta taattaaata tattttataa aatttttatt gaaaggtata atattattat 10380
agaggttttt tatttgtttt aaataatacg ataagttgtg agtaagcgtg tgtgtgggga 10440
tgtgtgggga ggggtgggtg tgagtaggga gagaggcgag gggagaatag tttttttcgg 10500
gcgttagggg tcgtttcgag ggttcgtttg tttcgggcga tatcggtttg gcgttttcgc 10560
ggtcgtttcg tgtgttttgg attcgtcgtt cgcggttcgg aagttggaga gttagcgacg 10620
gggttcgatt gcgggatcga gggttgtaag aagaagcgaa taaatagttt ttagcgtttt 10680
ttttggatgc ggtcgcgttt gtggttttgg tagtcgttgg gcgggttagg ttaggtcggg 10740
tcgggtcgag tcgggtatat ggatttgggt ttgcgggttt taattgcggc gtttatgttg 10800
atgatttttt ttttaattat agtagttttt agtttagcgg attgatttat tttcggtatt 10860
ggtaaatatg attacgtggg tcgcgcgatt aatggtggag gttgtagttt gcgaattagt 10920
cggtggttcg ggcgtcggcg gggagttgtt cggcggcgga tagtgtaatg ttgggtggga 10980
gtgcgggacg ttttaaaatg ttttttagtg gtatttttag taattattac gtggattcgt 11040
ttataggtta tgagggcgac gaggtgttcg cggcgcgttt cgggtcgtcg gggttaggcg 11100
cgtagggtcg gtttgtaggt gtggttgatg gttcggtcgt tatcgtcgtc gagttcgttt 11160
cgtgtagttt tgtttttaga tcggtcgtgt tttttgtttc gtggttcgcg gtgttttttt 11220
agttttcggt agcggcggcg atgagcggtt tttattattc gtacgttttt tcgtcgtttt 11280
tggtcgtttt tgttttcgag ttcggtcgtt acgtgcgttt ttggatggag tcgttgttcg 11340
gtttttcggg cggtgcgggc ggtggcggtg gtggtggagg cggcggttcg ggtcgcggtt 11400
ttagttttgg ttttagcggt ttagttaacg ggcgttatta cgggattaag tttgaaattc 11460
gagcggtttc ggttttcgtt acggtcgttt ttattatttt tttttttttt atttttttat 11520
tttttttttt taaacggatt gagtgtttcg tggttcggga gttttagggg agtagcggtt 11580
tcgagttttc gtgtaattcg tttttgtagg agaaggcggt agcggcgacg gggggaatcg 11640
ggtttggggt agggatcggg gtcgcgattg ggacgggcgg ttcgtcggag tttttagttt 11700
gtagcgatta ttcgatttta ggttgttcgt tgaaggagga ggagaagtag tattcgtagt 11760
cgtagtagta gtaatttgat ttaagtaagt gtaaaagaaa ttgttttttg atttattgtt 11820
gaaatttgta aggttcgaat gtgtaaaatt gatagtttta ttaatttata aaaacgttta 11880
gacgtttatt taagtttagg cgaataatat gtatttataa aaagagtttt ttataattat 11940
ttattttggg cgtttagtta gtacggtaaa tagagcgcga gtattaaggt tttttatgat 12000
aattttttat aagttgtgaa aagcgattat ttttggtgaa attaatttaa cgattttttt 12060

tttttatttt gtggtttttt tttgtttttt tttttttttt tttttttcgt ttttaaattt 12120
tttttttttgt aga 12133

<210> 340
<211> 12133
<212> DNA
<213> Artificial Sequence

<220>
<223> chemically treated genomic DNA (Homo sapiens)

<400> 340

tttataaaga gggaggtttg gagacgggga gagaggagag gaggggaggt agggagagat 60
tatagggtgg ggaagagagg tcgttaaatt aattttatta aggatggtcg ttttttataa 120
tttgtgggga attattataa aaagttttaa tgttcgcgtt ttgtttatcg tattaattga 180
gcgtttaggg taggtggtta tgggaagttt tttttatgga tgtatgttgt tcgtttaggt 240
ttgggtaggc gtttagacgt ttttataggt tagtaaaatt attagttttg tatattcgag 300
ttttataggt tttagtaata aattaggggg taattttttt tgtatttatt tgggttaagt 360
tgttgttgtt gcggttgcga atgttgtttt tttttttttt ttagcgaata gtttgggatc 420
gggtggtcgt tgtaagttga gggtttcgac gagtcgttcg ttttagtcgc ggtttcgatt 480
tttgttttag gttcggtttt tttcgtcgtc gttgtcgttt ttttttgtag gaacgagttg 540
tacgagaatt cggggtcgtt gtttttttgg gatttcgggg ttacggagta tttagttcgt 600
ttggaggagg aggataagga agtggaggag gaggaggtgg tggaggcggt cgtggcgggg 660
gtcggggtcg ttcgggtttt aggtttaatt tcgtagtggc gttcgttggt tgggtcgttg 720
gggttagggt tgggatcgcg gttcggatcg tcgtttttat tattatcgtt atcgttcgta 780
tcgttcggga agtcgggtag cggttttatt taggagcgta cgtagcggtc gggttcggag 840
gtagaggcgg ttaggggcgg cgggggaacg tacgggtggt agaggtcgtt tatcgtcgtc 900
gttgtcgggg gttgggaggg tatcgcggat tacgaggtag agaatacggt cgatttgggg 960
gtaaaattat acgaggcgaa ttcggcggcg gtggcggtcg ggttattagt tatatttgta 1020
ggtcggtttt gcgcgtttgg tttcggcggt tcgaagcgcg tcgcgaatat ttcgtcgttt 1080
ttatggttta taagcgagtt tacgtagtag ttgttgaggg tgttattgga agatattttg 1140
aggcgtttcg tatttttatt taatattata ttgttcgtcg tcgagtagtt tttcgtcggc 1200
gttcgagtta tcgattagtt cgtaggttgt agtttttatt attggtcgcg cggtttacgt 1260
gattatattt attaatatcg ggagtaaatt agttcgttaa attgggggtt gttgtgatta 1320
aaaaaaaaat tattaatata agcgtcgtaa ttagagttcg taggtttagg tttatgtgtt 1380
cggttcggtt cggttcgatt tggtttggtt cgtttagcgg ttgttaggat tatagacgcg 1440
atcgtattta gaggaggcgt tggggattat ttgttcgttt ttttttgtag ttttcggttt 1500
cgtagtcggg tttcgtcgtt gattttttag ttttcgagtc gcgggcggcg agtttagggt 1560
atacggagcg gtcgcggggg cgttaggtcg gtgtcgttcg aggtaggcgg gttttcgggg 1620
cggtttttag cgttcgaggg gagttgtttt tttttcgttt ttttttttat ttatatttat 1680
ttttttttat atatttttat atatacgttt gtttatagtt tgtcgtgttg tttaaaatag 1740
gtggaagatt tttgtaatga tattatgttt tttaataaaa attttatgag gtgtatttga 1800
ttatagatta atgtgttttt aataaaacgg acggatggag tagatcggag ggtttttttt 1860
ttttgttata tttatatacg ttcgcgcggt tatatatatt tcgacgtgag gttgggtata 1920
cgtcgcggtt cgaggggcga gtcggggcgc ggcggtggag gatatagagg ggtttttgtt 1980
tgttttcgga agggcgggcg gtcgggcgag ttggcgtagt acggatagtg gcggttaggg 2040
tgtttcgttt ttttcgatga tgttggaaag gttttggaag agaatacgtt atagggtaga 2100
atttaaatgt tagagtagtt tgttttcgtt cgtcgtttta gttttgtttt agcgagtttt 2160
ttcgggtagg gtcgtttcgc gcggttcgta tttaaaggtt tcggaggaga tttttaaaat 2220
gtttaaaaaa ttcggtcgaa tggttaggtt tttgggatat ttttttttga tttttttttt 2280
tttatagagg tgaggttgag tagttttgaa tttgagggaa atgtttattg ttatttttaa 2340
atttatggta taaagtttcg gaagttcggt tgggagttta gataaaagcg gtcggggcgt 2400
cggtagcgga gggtatattt tttgttttat aagttgttta gtttattttt ggagggtttt 2460
cggtttattt ttagtttcga aaaggaagcg ggtaaatggt tatttatggt ttgattgatt 2520
atttttgtta atagtttttt gattttgttt tagatgggat gtttttgttt tgtagtttag 2580
gtttttttcg gatatatggt tcgttattaa tagttaatgg tattttttat atgatcggag 2640
aaacggttaa ataaaaatta tttttttttt gtttaatcgg tgttaagttt ggggtaaagt 2700
tattataacg tttttaattt agtattgatt ttaagttttt ttttttattt ttaggaaatg 2760
gttttttaaa agagttttgt tttggaatgt ttttggtttt ggggcggaag tttaaaattt 2820
tgaatgttag tgttttagag attttttggt tgaggattta tagtatttaa atggatcgtt 2880
tttagggaaa tatttaaaag gaagaaatgg aaaggatgtg ttttgtaaaa ttgttggaag 2940
ttatttgaaa ttgtaggtag gattttaggt aacgaatttt ttgttagttt ttttttgtaa 3000
ggtttttttt tttttttttt cgataggatt agataagttt gggtttttta gtggagattt 3060
tgttgaagtc ggtggaagtt ggtgggagtt ttttggatta tggtagcgaa tggaatatta 3120
gattttatgg gaagttatgt ttatttgtaa aatttttagt tatttttttt gtattttttt 3180

gtattttaga tgtagtagtt tttattagta ttttgtaatt aaatagagga aagattgatt 3240
ggggaaaatt tttttatttt tttttttaat aaaattttat gtgggttatt aaaaggtaat 3300
gattgagtat aaaattcgtt tataaggaga ttaaggcgaa gtgggatggg aaaatttatt 3360
tattttcggt ttttttgggg tttggttgtt ttgcgtttgg gatgggggta gggttgaggg 3420
aaagggggta gaattggatt cgttgtagtg ttatattgtt gtttgttttt gtttttgtaa 3480
tggaggtcgg taatttttag gtgggtaaga ttagttttaa tttagttttt gatgattgtt 3540
cgttagttag aaattattaa gatgttattg aaagtattaa aaataatata attaaaatta 3600
aagaggagaa aatatataaa tgggaagttt tgaggagaaa gtggtttggg gtataggttt 3660
atttattagg tttaggttag aggaaagttt atatttattg agagcgtaat tatgttatta 3720
ttattgggga ttgggaaagg aaagaggaga tttttttttt ttttaattat gaaaatttta 3780
tttttaagtt agtttataat tttttttttt taattattgt taaaatttaa tattttaggt 3840
tttttttagt tttttttttt tatttttttt tttttattag tttattattt ttttgtttaa 3900
atgttttaaa tagggagtgt aattagaaga taattttatt ataggttttt atttttatta 3960
taatatattt ttattttaga aagtagaggg agtgaattta tttttagagt ttattgtatt 4020
atatatgcga ttagaatatg tatagaaagg ataggttgtt gttgtatttt tattaaatta 4080
tagatgtatt ataagggaaa ggttataagg tataaatcgt taatatttta ttttattaag 4140
gataattgtt aaatttattt gtgaaaagat atattgtaat gtgtttttta agtttttttt 4200
tttttaattt gtgggttttt ttaatttttt ttttttaaat atatatgtta tttttaaagt 4260
attggatgta tttaaaatgt tatgagttta tggattaatt atatatatag attttttgtg 4320
ttaggaaaga tatgaaaaag ttatattgtt ggatggatgg atggatggat gttttatagt 4380
tttaataagt taagatttaa atgaaaatgc gtaataatag ttattttgta ttacgatgaa 4440
aaaataataa attatacgtg ttaagttttt atatatatat atatatatat atatatatat 4500
ttatagggat tatagattat ttgtagtacg aatataatga ttttaaagtt atattttagt 4560
aggaaaataa aatataataa aataataaat aaagagcggt gaaggttttg ttttcgtttt 4620
tttagttttt tagtattggg ttttggaacg cggtgttttt tttttaattt tggtttttga 4680
tcggttttag atcggtttta gatttaagaa aacgtgaggt tggcggttag ttttcggatt 4740
cggttttttc ggtttatttt tttgagtttt attcggcggt tttgaaatta aattttgatt 4800
tgtttgtcgg tgaggttaac gtttttattg atttttaggc ggcgttcgcg ggtgaggtat 4860
atattgaata agaatttttt ttttaatttt agcgtttggt gtttagtgta agggtatttt 4920
ttttttttgt tattttttgt agtgagttaa ttgttggttg gtgtattaga tttgattttt 4980
tttaataaaa ttaaagggag aagaagaaaa aaattaagag gttttttgtt tgtttgtttt 5040
tgtttttgtt tttgttttgg ttttggtttt ggttttcgtt tttgtttttt tttttagatt 5100
gtttaaatgt atttttggtc gtgattttgt tggtagttag gtcgttttgg aaaatgttag 5160
tattaaatat tatttatatg aagtgggttt tggaagggaa ttttatattg atgttatatt 5220
atttaaatga tatgatggta ggatagtttg tatttgggtt ttcgtttttt tttttaattt 5280
ttttatttgt ataaaatatt agtagaatag taaatttttt agattttttt agtaatattt 5340
taagggttta gtttttaata gggacggagg ggaatacgtt acgatttgga taagtttatt 5400
aattttattg ttgttttagg tttttaaatt tgttattttt tttttatttt ttttaggtag 5460
ttaggaattt tgatagttag gtttataggt gtttattttt ttttaaaaga taaatgggga 5520
agcgagatat ttttttttat tttgtttggg tattgttgta ttttttagaa tttttttaat 5580
ttttatgttt ttttgttttt atattaaagt atttttttag aaagggagtt ttttattgtg 5640
gtttattttt aagtattggg ggagaggagg agggtttttt tttaaaaagt tagatttttg 5700
gttttttgtt ttagtttagt tttaaggttg gttttgtagg gagggtaatg cgggttttat 5760
tttttttttt cgtttttgtt ttttttgtcg acggattgta aagattgtag tatttttttt 5820
aaataaagtt aaggttattt agtttagtaa aggtagtaag ttttgttttt taatttaagt 5880
attaattagt ttagttattt tttttttttt attattttta agttcgtagg atgcggggtg 5940
tatatatttg aatacgaata cggtgttttg gattatttta atatgattat tgggtttaag 6000
gtaaaattgg aattgtaaat ttgagtagga gaaaagaggt tttgtggtcg tgtttatgag 6060
tcgtaggttt tttttgcgtt tttttgttgt gtttaggttt agtttaggaa aaataaaatc 6120
ggtaagtttt ttttttgttc gggcgttatt agcgtggggt taggattggc ggagagggtt 6180
agggtagtag gggagaagcg gggattattt tagggttgag attatttttt taaaattagg 6240
cggagtagta tttgttaagt cgggtttcgg ttcggcgttg ggtatttcgg tatttttttt 6300
ttgtcgggtt ttcgggttgc gcgtttcgtt ggggtggtaa ttttgtttat atcgattttt 6360
gttttttttt ttttgtattt cgggattgga tacggagatt ttagttaggt agtttttttt 6420
ttcgggaagg tcgtttttgg tttcggggtt ttttatttgg gagattttgg tgggtttttg 6480
gtcgtttacg ggttcgttta tttttttttt tatttgtagt tgggtagcgg agagttgcgg 6540
tttggtcgcg ttacgagggt tgagttggag tatgatagtg gagttgtttt cggggttatt 6600
attgtatttt tgggtttttt cggtggcgta ggtttgattt agtttaaaat atttagggac 6660
gggaatttta gggtttttaa cgggataaga tttagggatt agtttttggt acgaagggat 6720
ttcggtcgaa atgagtttgt tgcgtttatt agaatatatg tagtaattgg attttttttt 6780
aatgttggtg gtgaaggagt aagtggggat ttgttgtgta ataggttgtt ttattcgata 6840
agatttgttc ggatttgttt aattgtttat ttgaggtata taaggatgta tatttatatt 6900
tatattttgg tggtttattt tttttttggt tagagacggg agtagtttat aggtttgtat 6960
tttataggtt tttatgtttg cgttaggtgg tggtatgtat atgttggcgt tgttggaata 7020
aaaattgtta tttttgtagg tattgattaa ggaatttatt aaaaaagtat tagtagtagg 7080
agagttgttg ggaaaggata ttttggggaa gaatttgaag aagagagatt gtgttttttg 7140

taggttgata tttttaggaa aatatttttc gtgtaattgt ggatgttttt gtcgattata 7200
tgataattaa gttaatgaga tttgaaaatg gttttgagtc gtagtttttt ttcgggttac 7260
gtgttttaga gtttggtcgg tcggtcgggt aagtacggat aatagcgata tttattacgc 7320
gttcgcgata gtgcgcgttg gagataatcg gtttagcgtt tttcgtcgtt tttttgttcg 7380
tttttttagg gttcgttcgg ttagttcgtt ttattcgcgt ttttcgtttc ggtttttggg 7440
tttaatcggc ggaaggaaag ttttagtttt aggggttttt aagtggatac gtacggacgg 7500
ggagggaagt tagagtgttt tggcgttagc gtatagaagt atttttttta tatataatta 7560
ggtagaacgc gtttaagagg gtcgggttta ggataagcgg gtgggggcgt aggttggtta 7620
atttagtttc gggtagttta gcgggaaggt gagtcggatt tcgcgggttt tgagatattt 7680
tattttaggg gtttaaagga aagatttttt ggggttaaga agcggttaag gtgtttttag 7740
gagggcggag gtaaaaaggg tcgcgagggt cgggaggtta agtagttggt cgggttcgga 7800
tttttatgga tgggttggtt tttgcgttgt tttttcgttc ggtttttttg tttggtttcg 7860
ggtttttatg cgtttcggag ggttatacga ggtttcggtt tttagaggaa ttatattttg 7920
atttttgcgg ggtcggattt ttgggagttt ttagtttaga gataattttt tgttagtagt 7980
ttattattcg cgaaagcgtt tttcgggttt tttgtaggag aattaaatag aggttttttt 8040
ttttatattt tgataatgga gggtaaatag gaaggaagaa aggaaaaaat aatttaagaa 8100
aaggggaaat tttgattttt agaatttaga agttttagaa aattgattgg gttaagaagg 8160
atagagagaa atgtaaaggt gttattttgg tttttttgta aagaaaaatg tttttttttt 8220
atggattttg taaaagtatt ggtttttgat aagtgtaagg tataaaaggt tgtggaattt 8280
attaatatat attaagaaga attagggcga ttaataaaat ttttatttat atttttgggt 8340
tatttggtaa tttttttgtt tttgaaggtt tttaaagagt tatgttcgtt gttgttatat 8400
acgggaagat attttattaa taaattaatc gagattttgt aaaggataag attaatagtt 8460
aaatttatta taatggcgtt cgttttatga attattgaaa attatattat tgattttttt 8520
tttttcgttg ttaattaaga aggttatttt tgttttaata taaattgtta aatattaaaa 8580
gttatatttt tgttagaagt tgagttttat agttttggat aatttttgaa attatttgat 8640
aaggttttgt ttgggtaagg gaggggaagg aggtgggaat tttagttttt tgtttttttt 8700
ggtgagggaa atttttatga agaggatatg gtttggggtt gtggttcggt ttattaaaga 8760
attattatta ttattattat tattattatt attattatta ttatggaaat ttagagtttt 8820
atgagaaatt ttaataattt tttttttttt ttttttgttg taggcgggtg agtcgttttg 8880
cggaagttcg ggtttcgggg tagtagcggg gagttttaat tttgattttt aaagatttgt 8940
tttgagtgag gggtcggtta gggtttggag tcgtttttcg gtttgttagg tttttttttt 9000
gttagttaag aaattaaagc ggttttagaa attttgatta tgaattagtt tttagttcgg 9060
gagggtattt gtgttttttt tttttcgtat tcgttggggt atgggttggt tttggatttt 9120
aggtttagag attgaatttg tgggtaaatt gggtttttat tttaatagaa tatttttttg 9180
gggtttgagg tttattaaag cgttttagta ttttggggga tttagtagtt cgttcgtttt 9240
tttataaaaa ttttttttaa aaaatttttt gttttttttt ttaaaaagga attttttttg 9300
gtttggttga aataagcgtt gaatattatt ttcggttttg ggattttttc gtttgttttt 9360
tttcggtgtt ttcgtttaaa taataatttt ttttaaaaaa tttcgcggtg gtaatgagat 9420
agatttttta aatttttacg tagttgttta aatataatat tattgttagg ttaaaagttt 9480
ggcgttttta attgttttgg ttgtagtatt ttagtatagt ttggggttta aatattattt 9540
ttttataggg gtggttgttt tcgagtttgg gggtgggggt tgaggatggt cggaaaaata 9600
aaataagata aaatataatt tttgatttta ggttgttaag gtattgttta aagttaatag 9660
ttattgatta gcgttttgaa atttgttttt ttatatagta gagattttgt tttaagttag 9720
ggttaagcgt gtttaaagtt gaatgaagtg ttcgttgtat tcgtttgtcg taggcgtttt 9780
gatattagtt tggcgtttta gtagtatcga gtgtcgtttt tttgtttttt ggttagaaga 9840
gttgttggga atttgtttgt ttaatttttt ttattttaag aaaggtaaga ttgtttaatt 9900
taatcgggaa ttttatgttt attaaatgtt tttttataat ttttatttat atatgaagaa 9960
aatatatagg ttaaaaataa gtaatttttt gcgggcgggt aggtttgtag tgagttagaa 10020
taggtaggat gtgtttttag aatttgggga tttttttatt ttagttttta gtggttttga 10080
gtttaaagtt atttttttat tggttagagt taaataggtt tttatttata gataaaaatg 10140
tgtatattag atataatttt gaatttgttt ttataaaatt ttatttttta tttttaaaat 10200
ttttgttttt agattcgttt tttgagataa gagattgtta atattttgag atattttatt 10260
attaaaaaat tgagtaagat agaaataata taaaagaatg attcgagttt taaaggatgg 10320
aaagtgtgtt taagatttta aggttgaggt gagataaatg gaataaaaag gtcgtgtttt 10380
ataggttagt ttttattttg tttatgagta tttaatgtag ttgatgtttt ttattatttt 10440
tagattagtt ggagtagtta ttattaataa taaattaata ttgtattata aaggagatga 10500
atatttatat ttgtattaag acgttttttt ttttgttata gtttttttta gggaaaaatt 10560
aaaagaatag tttgaataag aaattagtat ttgtaattaa aaagtggttt gtatttgtat 10620
ttttgttttt tggaagaaaa gttgaagttt tgggtttgag tggatttgtg ggtttttagg 10680
gttatttcgg attagggttt tgagttattt ttgggttgtg ttattttttg gggatagagc 10740
ggatggagtc gattaaaagt tgtttttttc gggcgttttt tcgggttgta ttgtgtattt 10800
ttaagttaag gtgaaaggaa atttaggggg taaatgggga gtttattatt aatgtaataa 10860
ggggtttatt tagacgtgta ataagggtcg gcggtcggag aatttttttt tgtatagata 10920
taaaagattt ttatttcgcg tatttttttt ttattttaga gtttgagatt ttggcgtttt 10980
aggatttaag gttatttttc gtaagtttta tatattttgt aagatggggg tggggagagg 11040
tttttagttt ttagaggttt tggtttgatt tcgggttttt gttaggttta ttttgggcgt 11100

agggaggaaa ggaattgtag atcgttgaga agcggtgttt cgtaggcggt tgttagttaa 11160
gtttaagggg ttttcgtata gttagatgtg tagtcgttgg ggtcgttttg agaggataac 11220
gatatttagg gagggtagtt tttattttat atagatcgag gatttcgttt agtttcggga 11280
aaggtagcgt tattatagat aaggtggatc ggcggttaga ttagtggagt taaggttgtt 11340
tgcggggaat atttacgtta ttcgttggag tttcgtcggg gggtttattg gagtttcggt 11400
ttttttatta aagttgaggt tagtttcggg agaggggtaa gcggtaggtt ttagggtttt 11460
tggtttaggg tttggagggt taataaggtt agcgttaatt ttgggggttg taggtttttt 11520
ataatgaatt tcggggaggg aaagggggta gttatttttt tttagagagg ttaatgtcgg 11580
ggagttttgt tttttttttt gtgaatttcg aacggggata taggtttttt ttaaggttag 11640
gggttttgtt ggaggaagga gggtacgtaa cgaatgtatt gttttgagaa gttataattt 11700
aattttgttt aagatcggcg tgtagtttgg aagggtggtt agttttttgt taaacgtaaa 11760
gttcgtttgt tttaaatata tttttcgttt tataacgttt tgaagtataa acgttttttt 11820
cgagtgtttg ttgtttttcg tgttcgtatt agtttagttc ggtttgggta tattaattta 11880
tagttttgtc ggcgttattc ggtcgttatt ggtagtcgcg tattttaggt acggtcgcgg 11940
gtttttagaa cgattttttt agttttttat gcgttttatt tttttttgag agatatttgg 12000
ttaagtggtt cgttttttga tttgttataa ataaaagttt tttaattatt ttaggaggtt 12060
tgagaggggg taaaatcgat gaaggttttt tttttttttt ttttttttgc gggggggagg 12120
gggatgttaa gta 12133

<210> 341
<211> 8047
<212> DNA
<213> Artificial Sequence

<220>
<223> chemically treated genomic DNA (Homo sapiens)

<220>
<221> unsure
<222> (2539, 3845)

<400> 341

aatttggaaa gagagaggga ggagaggaaa tgtgatatga gttagtagag tttttttata 60
tatttttttt tttcgtttaa ttgtaataaa gtaaaagtag ttcgggtagt tttttaattt 120
tttagtttaa aagaggaatt attagcgtag gttttggagg tgtttggggg tagtttttag 180
tagtttaata tgtttaattt gttttttttt tttttttttt ttttttgttg atttttagtt 240
ttttttaatt ggttagttcg ggttttgttt ttaaaatatt agtattttta aatgatattt 300
atttttgtgt taggtgtgtt aaattttatt tttttttatg tttaaatttt gaaaggagga 360
gtgtattttt ttggaatgcg gtgtgtgtag cgggggtagg tatgtgttta gatagttttt 420
tttattatat ttatgggaaa ttggaagttt tttaggtgag ttatgttttg gtgtttttaa 480
atagtatttt ttgtatttag gttttttggt agattaggag gttttttgcg atttgatttt 540
ttgattagga gttttatgga gattaaagga gtttatttat atataaaata ttaggtattt 600
ggagtagaat ttatttttta atttttaaat aaaaaagttt aattatagat taatcgggtt 660
aattgagtag tcgttttaga atagtaaagt tttaattaaa tagtgtgatt atgaggggag 720
ttgagtaatt attgaaattc ggtttttttc gtaagaggga ttttagtttt cgtttagtgg 780
tataaaaggg gttagcgggg taatcgtttt ttttttgttt agaaaagtga gatttggggg 840
tggtgggggt agggtcgggt agttgcggtc gcgtttaggt agttgagtat ttaggttagg 900
cgttgaggtt gagtattgag ttttgaggtt ttagtgtttg ggttttcgag tagttttagg 960
aggtatcgag ttgtagttgt agcgcggtag ttaggaatgg agtatgtgta tttgtgtgcg 1020
agggttggtg agttataaat attcgcgtag agttagggga tttggggaga gagaggagat 1080
agggagaata taattaagga ggatttttgg aaaatattgt ttttaaataa aaattttaga 1140
ttttattttt tttattttaa atattttttt ttaggaaatt aatatatttt ttagagttga 1200
gtggaaagta aaggttagta aggtgaggtt tgtttacgaa tttttttagg ttgggtttag 1260
ttttatttat ttttttttta gtttagagtt tatatgtttt tagttttttt ttggtttaaa 1320
ggggagtaag gttggagtgg tagtatttaa tttgtttagg gaaattttta agtgtttagg 1380
ttgtaatgtt atttttattt ttggggattt taggttagtg attgtatttt atttaggttt 1440
aaggcgtagg tagtaaattt tttttttata attagcgtta ttaaaagtat gtgttttgta 1500
ttggagagga ttggagaagt aagtggtaat tttttttttg tatttttttt ttgtgttttt 1560
tgaattttgt ttttgaattt attaattaaa gtttttttga aagtgaaaat ttgtttttga 1620
tattaagttt ttttcgaatg tttgatttgg gaggttaagg ggttagagta gtagtaagga 1680
gatttgtaag tttgatgggg ttggggggta gtgttttttg atttttttag gttcgtttta 1740
tagaaataaa gaataagttt tttttttttt tttttaagat ttgtgtatat ttatagagta 1800
taattgtagt tttaataaaa tatttgtttt tgtttttgtt gatgagtttt tataattgtt 1860
tttagataaa tttaatagga aaatataaat atatttagaa aaatttaagt tttagttttt 1920
tttttataga tagttatttt tttgttaaaa aaataaaatt aatatgaaat gggaaaaaga 1980

aaatataatt taatgtaggg aagatcgggt ttatttgatt tgtttttttt ttttggagcg 2040
aattttttta gtaaatttta gttgtataat taaagattta agagaaagaa gagggttttt 2100
ttttttttta ggaatgaaag gtaattgggg gggggggagg tgttgaaaat taattttgtt 2160
aaaggttttt aagtagtgga ggaatttatt tgtatttttg tttttttttt tttttttttt 2220
ttagtatttt tgtttttttt atttagtagt tggtttttgt ttattataaa tcggcgagat 2280
tttttagttt ttgttttttg tttagggacg ttataaaata tttaattttt tggggttgag 2340
gtttatattt tgttttttag gtcgttaatt tttttgagtt tatcgatttt aggttttaat 2400
agagaatttt tttaattttg gggttttttt tttttttaga tatggttgag gaggggttta 2460
aggaaattta ggtatttttg ttgggggttg tagtttatgt tgttttagaa gttttttttt 2520
ttttttaagg ttttttttnt tttttattta gatataaaag agatttttaa atatatagaa 2580
aattaatatt tattgaaagt gaaattttat taagatattt tattttttat tatttaattt 2640
gttgtatatt gtaataattt aatattttga aggaaatatt attgatatga tttatttttt 2700
tagtaatttt tttttgaaat tggagggggg gatagagtta tttttttttt cggttttttt 2760
tatttgttat attattattt ttagggtaga aaggaggttg ggaattttga gagaggttat 2820
ttgtgtttag gggtgtattg atttgttttt tagtttagtt tttagagtat ttttgttttg 2880
ttgttttttt tttatttttt gttttttagg gggtttgaag ttttagttag ttgttggaaa 2940
tttttgttta ggtatgttat gaattcgagg atagttggat tttagggtaa aaagtaattt 3000
atattggtgt ttatgtttgt gtatttgtta agttatttta aaaatgagat aatttattta 3060
aaattttttt ttttaattta aaaatttttt tttatttttt tttttttatt tttttgggga 3120
gaagaaagtg tgatttagaa ggaggaattt ttataattat aggagttttt tttttttttt 3180
tttaaatatc gataaatttt tgtttttttt ggaggattgg ggttgtattt tttaaaatta 3240
atataaattg tttggtagga agatttttta attttttaac gaggtttatg tattaattaa 3300
tatgttgttt ttattagttt ttgtatattg tttgtttttg ggttcgaaat tttatttttt 3360
ttttttagcg atattttagg gaaattttaa cgttatagaa gatgaataaa cgagtttttt 3420
tttagtatag tttcgtttat ggttttattg ttatttattg attatttcgt tttgttatat 3480
agaaggaaaa gattataaaa ttcggcgata ttcgggtttt tgttatagtt agtttttttt 3540
ttttttaatt tatttaaaaa tggtgggggg ggggggatat gagttttttt tgtttttcgt 3600
atatttttta ttattttttt ttgttaattt aaggtatttt tagtttatat tacgatatta 3660
aattaagttt ggattaatta ggagaaaatt tttatttgta tatttttcgt tgttttagtt 3720
cgtttattgc ggttttttta taagtttcgg atttagttgt tttttcgtaa tcgttttttt 3780
ttttttttta gttttaagat gggggaataa aatgggcgtt gaggttgttg agaggtttat 3840
ttttnattac gggtgattgg tatttgggtg agagtttttt agaatttttt tttgaagttt 3900
ttaagtttta tagtttaggt ttaggggcgt ttttattggc ggtgttttga gtttggaggg 3960
ggtagggggg tttttgatta aggtaggggt ttgagtttgt aggaagttaa ttgtttatta 4020
gagttttatt agtttttttt ttttggttgg attttatgtg aattatttta taggttttta 4080
ttggttttaa ataataaatt tttattaata taaataggag ttgatgtttt tagaaagatt 4140
tttagagttt tcgttttttt tttttttttt tttttttttg tttttttttt ttcggtgtta 4200
tttttttata ttttattttt tttttttttt tttttattgt attttttata tatttttttt 4260
tttaggtaaa aatgttggaa gagttttttt aatagagggg ttttgagaag gaggtagaag 4320
ggagttagga ggtaaaggag agtagatgcg tggtttattt gtttatttta gttttgttat 4380
tgtattttga agttttgttt ttttagattt agttgttttt tatattttta ttatatttaa 4440
gatttttagt tatattttat ttatttaggg gaataggttt tatgaaattt ttttttattt 4500
ttgatttttt tttaattatt ggtgaaaaaa atattagttt agtgatggga tttattttat 4560
ggagtgttaa aataggattt tttgtaggat ttttagggat tttttttttg tgttgggagg 4620
gagttttttt tttggagaaa ttaggatgta attttagaag ggggtagata taaaatatga 4680
aattagattg aaaatatagg ggttttttta gttttggttt tggttatagt atagtaaagt 4740
tagttggggt tgagagattt tttggtagta tagttttttt tttttgagtt ttaaggtttt 4800
gtttagggat atgtattttt tttttattat taaattagtt tttttatata tatatatata 4860
tatatatatt ttttaaaaga aatttaagtt ttatttttaa agtatatatt tagttttaat 4920
tagggaatta atagaagtag aagcgatttt tttttttttt tttgatattt ggtttgcgat 4980
tggttgggag ggggttagtt gattttgtta ttttgtttgt tttggattgg agtcgttttt 5040
ataattattt agtttcgagt ataaattgga gatagaaata aatattaaag aaattataga 5100
tcgattaggt aaaggtaaag ggatgaattt ttattttatt aatttttttt tattttgtta 5160
tttcgtcggg ggttaggacg ttttttttaa cgtcgttttt aattttatcg tttatgattt 5220
agtgaggtat ttttcgattt atggagcggt cgttgtttag aatcggattt attcgatttt 5280
tttttattcg ttataggaga atgtcgtgtt tagttttagt cgggggtcgt atgattatgg 5340
atttaatttt ttttattagg agaaagatat gtttttaaat tgtagataaa atattttagg 5400
atataatata tagattttaa tcgtttagga ttttagtttt gagtagggta ggattgcgtt 5460
ttaggattag aaagttagta tttagattta tttttggatg tagcgaatga attcgtatag 5520
tggtgagttt tatagtttcg agatataaat taaataaatt gaattggttt tatgatcggt 5580
ttttttagaa gaacgggcgg agagagtttt ttatggtttt ataaaaataa ttacgttcgt 5640
ttttttatcg agatagggtt tttttttttg tttttttagt tcgtttttcg ttcgtttgta 5700
ggggtttggt ttttttggtt tttgacggat tggagggttt taaggcgggt tgagggggta 5760
ggaatagggt aggggatgag gggagggagt agaagatagg ggagtttttt aaagtggagt 5820
tagtttgagg agaagggagg gggagaggag aggtagataa gtggggagaa gtttttaagg 5880
aattaggatg tttggggaga tgggggagtt tagtttttga gaaagggggt ttttattttt 5940

attatttggg attttttaaa aatttgtttt tagatttgta gttttttgtt tgttttttgt 6000
ttcgtttttt cgtttttttt tttattatat attttttttt gggatatata ggttttattt 6060
tataggaggt ttgaagttgg ggttttcgtt gtatttttag tgggggtggg gtaaggtaaa 6120
agggagaagg ttagagtttt aaggaggttg tgagttgttg gttaggttgg gagggttagg 6180
attttgttag gcgaaatagt ttgtagagga cgttttgttg attgttttta gtgtgttttg 6240
tgttcggatt tttaggggtc ggttacggag cggatcggag gcgcggtcgt tagatttatt 6300
cgcggtatta gattttggaa ttggagaagg aattttattt taatcgttat ttaacgcggc 6360
gtcggcgtat cgagatcgtt aacgcgtttt gtttgatcga gcgatagatt aaaatttggt 6420
tttagaatcg tcggatgaag tggaaaaaag aatttaattt tatatttatt ttttcggggg 6480
gcggcggagg ggttatcgtc gatagtttgg gcggaaaaga ggaaaagcgg gaagagatag 6540
aagaggagaa gtagaaagag tgattaggat tgtttttgtt attttttttt tttttttttt 6600
ttcgtttttt attaattttt ttttaattat atattttgta tttattattg gtataattga 6660
tgtgttttga ttttttaaaa taaaattagg gagttaaacg tggatttgaa agttagtttt 6720
ggattttttt ttttatcgta taattttttt ttattacgcg tttttttttt ttcgtttttt 6780
tgttagttcg ttttcggttt gtttataggt tttttttttc gtttaggttt tgggggttcg 6840
gattttgaat ttagatttta tagattgttt tttaagtgag gatttggttt ttttattttt 6900
tcgacgtttt tattttcgtt tttcgtgtag agagtcggtt tttgggtttg ttggggtttt 6960
tgttttaggg ttttagggtt cggtttggta gtcggggagg gtcggaggtt taaggagggc 7020
gcgttttggt tttatattaa tttttagggt tttttcgtag tttttgttta gtttttttgt 7080
tttagtaaat gtttagttta ggtaaattgt atttaaagaa ttttgggggt tattatggta 7140
ttttataaat tgtgatcgtt tttgtgtgaa gatttttagt tgtatttgtg gtttttgtat 7200
ttatatttat gtttagtatc gttagtgttt ttatttaatt ttaaaaaagg aaaaaaaaga 7260
gggaaaatta taaaaagaga gaaaaaaagt gaatgacgtt tgtttagtta gtaggagaaa 7320
ataaataaat aaataaattt tttcgtgtta tttttttgta taaatttaat ttttgggttc 7380
gttttcgaat atttaataaa attgatatta tttttaaaat tttatatcgg gaattttgtt 7440
aattcgttcg ttcgtcggtt ttagggtgag agttggagta ggttttaagc gttttcgcgt 7500
ttgtcggttt tttgtaagtt tttagttttt tgtattcgtt tttttcggag tttaggcgtt 7560
cggtttcggt tttttgttcg ttttcgcgat cgtattgagg ggttgggagg tttttttggg 7620
ttttttgcgc ggagtaatat atatattttg cgggaaaaag tcgcgtagga ttttgttgcg 7680
gggggagttg ggattagtcg gtttgggtat taattttacg taggtttttt tttttgtttg 7740
tggtagtcgg tgttttcggc gttcgaattt tgaggcgttt cgaaagatta gtaaagaagt 7800
attatttagg tattagggtt ttttttattt attttcggga gttggaggcg gggggcgtcg 7860
tgagagtaag ttttaggttc gcgttaagtt ggttgttgcg aaacgtagcg gaggcggaga 7920
gcggagggaa agagaagatc gcgggattgg agttttgtcg gtagggattg agcgatttaa 7980
ggatatttcg agagagaaag tttggaaatt gagttttcgt ttgttttggt atttttgacg 8040
tggtcgg 8047

<210> 342
<211> 8047
<212> DNA
<213> Artificial Sequence

<220>
<223> chemically treated genomic DNA (Homo sapiens)

<220>
<221> unsure
<222> (4203, 5509)

<400> 342

tcggttacgt taggagtatt agagtaggcg agagtttagt ttttaggttt tttttttcga 60
gatgtttttg aatcgtttaa tttttgtcga tagggtttta gtttcgcggt tttttttttt 120
ttttcgtttt tcgttttcgt tacgtttcgt aatagttagt ttggcgcggg tttggggttt 180
gtttttacgg cgtttttcgt ttttaatttt cgggagtggg tggaggagat tttggtgttt 240
ggatagtgtt tttttattgg tttttcgggg cgttttaggg ttcggacgtc gaggatatcg 300
gttgttatag gtaggaagaa aggtttgcgt ggggttggtg tttaaatcga ttgattttag 360
tttttttcgt aataaaattt tacgcggttt tttttcgtag ggtgtgtgtg ttgtttcgcg 420
tagggagttt agagaggttt tttagttttt tagtgcgatc gcgagaacga atagaaggtc 480
ggggtcgggc gtttgggttt cggggaaggc gagtgtagag ggttgggggt ttgtaggggg 540
tcggtaggcg cgggagcgtt tggagtttgt tttagttttt attttgaggt cggcgggcga 600
gcgaattaat agaattttcg gtataaagtt ttaaaaataa tattagtttt attaaatatt 660
cgagaacgga tttagaggtt ggatttatat aggagggtaa tacgaaggga tttatttatt 720
tatttatttt tttttattgg ttaaataaac gttatttatt tttttttttt ttttttgtaa 780
tttttttttt tttttttttt tttttgaaat tggataggaa tattgacggt gttaaatata 840
aatataaata tagagattat aaatatagtt aaaaattttt atatagaaac ggttatagtt 900

tgtaaaatgt tataatgatt tttaggattt tttaaatata atttgtttgg gttgggtatt 960
tgttggggta gaggggttag gtagggattg cggggaggtt ttgggggttg gtgtggggtt 1020
aaggcgcgtt ttttttgggt tttcggtttt tttcggttgt taggtcgggt tttgaggttt 1080
tggagtagag gttttagtag gtttaggagt cggttttttg tacggggggc gggggtgggg 1140
gcgtcgaagg agtgggggag ttaagttttt atttggaggg taatttgtag agtttgagtt 1200
tagggttcga gtttttaagg tttggacggg gaaaagggtt tgtagataag tcgagaacga 1260
gttagtaagg gagcgaggag gaggaggcgc gtggtgaaag agagttgtgc ggtgagggag 1320
ggggtttaga gttgattttt aggtttacgt ttgatttttt aattttgttt tagggaatta 1380
aaatatatta attgtgttag tgataaatat agagtgtgtg attaggggag agttggtggg 1440
gagcgaggga gaaagggaga gaggggtggt agggatagtt ttggttattt tttttgtttt 1500
tttttttttg ttttttttcg tttttttttt ttttcgttta ggttgtcggc ggtggttttt 1560
tcgtcgtttt tcgagagagt ggatgtgaga ttagattttt ttttttattt tattcggcgg 1620
ttttggaatt agattttgat ttgtcgttcg gttaggtaaa gcgcgttggc gatttcgatg 1680
cgtcggcgtc gcgttaggta gcgattgaag tgaaattttt tttttagttt tagggtttgg 1740
tatcgcgagt agatttggcg gtcgcgtttt cggttcgttt cgtagtcgat ttttaaagat 1800
tcgggtataa aatatattaa aagtaattag taaagcgttt tttgtagatt gtttcgttta 1860
gtaaagtttt gattttttta atttggttag tagtttatag tttttttagg gttttagttt 1920
tttttttttt gttttgtttt atttttattg ggggtatagc gaggatttta attttagatt 1980
ttttataagg tgggatttgt gtgttttaaa gaaaggtgtg tggtgggggg gggggcgggg 2040
gggcggggta aaaggtaaat agaaagttgt aagtttaaaa gtagattttt gaaaaatttt 2100
agataatggg gatgagggtt ttttttttta aggattgggt ttttttattt ttttaagtat 2160
tttagttttt tggaaatttt tttttattta tttgtttttt tttttttttt tttttttttt 2220
tagattgatt ttattttggg gggttttttt attttttgtt tttttttttt attttttgtt 2280
ttgtttttgt ttttttagtt cgttttgggg ttttttaatt cgttaggggt taagggggtt 2340
aggtttttgt aagcgagcga gaggcgagtt gagggggtag aagagggggt tttgtttcgg 2400
tgaggagacg agcgtgattg tttttatggg gttataaaaa atttttttcg ttcgtttttt 2460
tagggaagtc ggttataaag ttagtttagt ttatttaatt tatatttcgg agttgtaaaa 2520
tttattattg tgcgaattta ttcgttgtat ttaggggtaa atttggatat tggttttttg 2580
gttttggggc gtagttttgt tttgtttaga attaaaattt tgagcgattg aggtttgtgt 2640
gttatgtttt aaggtgtttt gtttgtagtt tgagagtatg tttttttttt ggtaaaagga 2700
attagattta tagttatacg gttttcggtt ggaattgaat acgatatttt tttgtggcga 2760
ataaaaggga gtcgagtaga ttcggttttg ggtaacggtc gttttatagg tcgagaaatg 2820
ttttattgga ttataggcgg tggaattgag ggcgacgttg gggaggacgt tttggttttc 2880
ggcgaggtgg taggataagg aagggttagt gaagtaggaa tttatttttt tgtttttatt 2940
tggtcggttt atgatttttt taatatttat ttttgttttt agtttgtatt cggaattaga 3000
tggttatagg gacggtttta atttaggata gataaaatga taaagttagt tgattttttt 3060
ttagttaatc gtaagttaga tgttaggaag ggggaaaaaa atcgtttttg tttttgttga 3120
ttttttagtt gagattaagt gtgtgttttg aaagtaagat ttggattttt tttaaaaaat 3180
atatatatat atatatatat gtaagggaat tggtttggtg gtgaaggagg ggtgtatgtt 3240
tttgagtagg gttttgggat ttagggaagg agagttgtat tattaggaaa ttttttagtt 3300
ttagttaatt ttgttgtgtt gtggttagag ttagggttag agaaattttt gtatttttaa 3360
tttggtttta tgttttatat ttgttttttt ttaaaattat attttgattt ttttaaggga 3420
agaatttttt tttagtatag gaggggggtt tttagaaatt ttgtaggaaa ttttgttttg 3480
atattttatg gagtgagttt tattattaaa ttagtatttt ttttattaat aattaaaaaa 3540
aaattaaaag tagaaagaaa ttttataaaa tttgtttttt tgagtaagta gagtgtggtt 3600
gagagttttg aatgtagtag ggatgtgggg aataattagg tttaaagggg taagatttta 3660
gggtatagtg gtagagttgg agtgggtaaa tgggttacgt atttgttttt ttttgttttt 3720
tggttttttt ttgttttttt tttaggattt ttttgttggg aaaatttttt tagtattttt 3780
gtttaaaagg gaaaatgtat aaaaaatgta atagaaaaag gagaaaaaga agtaaaatgt 3840
agggaggtgg tatcggaggg ggaaagatag agagaaagag agagagagga agaacgaaaa 3900
ttttgagaat ttttttagag atattaattt ttgtttatgt tggtgagaat ttattatttg 3960
gagttagtga gggtttgtgg ggtaatttat ataggattta gttagaaaga gaaggttggt 4020
ggggttttga tggatagtta gttttttgta ggtttaagtt tttgttttgg ttagaagttt 4080
ttttgttttt tttagattta gggtatcgtt agtggaggcg tttttggatt tgagttatga 4140
ggtttggaga ttttagaaag gggttttaag gagtttttat ttaagtgtta gttattcgtg 4200
gtnggggatg ggttttttag taattttaac gtttatttta tttttttatt ttggggttaa 4260
aaaaagggaa gagacggtta cgaaaaggta gttgaattcg aggtttgtga gggagtcgta 4320
gtaggcgaat tgaaatagcg gaaagtgtgt agatgggggt ttttttttga ttagtttaaa 4380
tttaatttga tatcgtgata taggttgaaa atgttttgag ttgataaaga agggtggtgg 4440
ggagtgtgcg gggagtagag aaagtttata tttttttttt tttattattt ttgggtgggt 4500
tggggggggg gaaagttggt tataataaga attcggatgt cgtcggattt tatgattttt 4560
tttttttgtg taataaagcg gagtaattaa tgggtagtaa taaagttata aacggattta 4620
tgttgagaaa aaattcgttt atttattttt tgtaacgtta aggttttttt ggagtgtcgt 4680
taggggggaa aaataaagtt tcgaatttaa aagtagatag tgtgtaggag ttggtggaga 4740
taatatgtta gttaatatat gaatttcgtt agggggttga agagtttttt tgttagataa 4800
tttgtgttgg ttttagaaaa tatagtttta attttttaga aaggatagaa atttgtcggt 4860

atttggaagg gaaggggaaa gatttttata attataaagg tttttttttt tagattatat 4920
tttttttttt ttaaggaaat gaaggggaaa gggtagagga ggatttttaa attaagaaaa 4980
aaaattttag atgagttatt ttatttttgg ggtgatttgg taggtatata aatatagata 5040
ttaatgtgag ttgttttttg ttttagaatt tagttgtttt cgaatttatg gtatgtttga 5100
gtagaggttt ttaatagttg gttgaggttt taggtttttt ggagggtaag aggtggggga 5160
gagataataa ggtaggagta ttttggggat tgagttaggg aataagttag tgtatttttg 5220
gatataagta gtttttttta gagtttttag tttttttttt gttttaaggg tagtggtgta 5280
ataagtagaa gaggtcggaa gagagagtaa ttttgttttt ttttttaatt ttagagagag 5340
attgttagag ggataaatta tgttagtgat atttttttta agatattaaa ttgttgtaat 5400
gtataataaa ttgaatgatg gaagatagga tgttttaatg aggttttatt tttaatgagt 5460
gttgattttt tatgtattta aaaatttttt ttatgtttgg gtgggggang ggaaggattt 5520
tggaagaaag aaggggtttt tggggtagta tggattatag tttttagtaa ggatgtttag 5580
gtttttttga gttttttttt aattatgttt ggagaggaaa ggggttttaa agttggggag 5640
atttttattt ggggtttaaa gtcggtgggt ttagggaggt tggcgatttg agaggtagaa 5700
tgtgagtttt agttttagaa agttaagtgt tttatgacgt ttttaaatag agggtagaga 5760
ttgaaaggtt tcgtcgattt ataatgaata gaaattagtt gttgggtgaa aaaggtagaa 5820
atgttggaag ggggagagaa ggggaaatag aaatataaat aaattttttt attgtttaaa 5880
gatttttggt aaaattaatt tttaatattt tttttttttt taattatttt ttatttttga 5940
ggaaagaaga agtttttttt ttttttttag atttttaatt atgtagttga aatttgttga 6000
aaggattcgt tttaaggggg gagagtaggt tagatgagtt cgattttttt tatattggat 6060
tatatttttt tttttttatt ttatgttaat tttatttttt tggtaaaaga atggttgttt 6120
atagggggaa agttgggatt tagatttttt taaatatatt tatatttttt tgttaaattt 6180
gtttgaaaat aattatggaa atttattagt agaagtaaga atagatgttt tattaaaatt 6240
gtagttgtgt tttgtaaata tgtataagtt ttgggaagga ggggagagaa tttgtttttt 6300
gtttttataa aacgagttta aagaaattag agaatattat tttttaattt tattaggttt 6360
gtaggttttt ttgttattgt tttggttttt tagtttttta aattaggtat tcggaagggg 6420
tttggtgtta agagtagatt tttattttta gagaagtttt aattagtgga tttaaaaata 6480
gaatttaaaa aatatagaaa aagaatataa gggaaaaatt gttatttgtt tttttagttt 6540
tttttaatgt aaaatatatg tttttgatag cgttaattat aggagagaaa tttattgttt 6600
gcgttttgga tttgagtggg gtgtagttat tggtttggag tttttaagga taagggtggt 6660
attgtagttt gggtatttgg ggattttttt aggtaagtta aatgttatta ttttagtttt 6720
attttttttt aggttaggga aaggttgggg gtatgtgagt tttgaattgg ggaaagggtg 6780
ggtgggattg gatttagttt gaagaagttc gtgggtaggt tttattttgt tggtttttgt 6840
tttttattta gttttgggaa atatattaat tttttgggga gggatatttg aagtgagagg 6900
gatgagattt ggaattttta tttggggata atatttttta gaggtttttt ttagttgtgt 6960
tttttttgtt tttttttttt ttttagattt tttaattttg cgcggatgtt tgtaatttat 7020
taattttcgt atatagatat atatatttta tttttgattg tcgcgttgta attatagttc 7080
ggtgtttttt gaggttgttc ggggatttaa gtattggagt tttaggattt agtgtttagt 7140
tttagcgttt ggtttgggtg tttagttgtt tggacgcgat cgtagttgtt cgattttgtt 7200
tttattattt ttaagtttta tttttttgag taagagaaga acggttgttt cgttggtttt 7260
ttttgtgtta ttgggcgagg gttaaaattt tttttacgga gaaagtcgag ttttagtaat 7320
tgtttagttt tttttataat tatattattt ggttaaggtt ttgttgtttt ggagcgattg 7380
tttagttggt tcgattggtt tatgattaga tttttttatt tagaaattaa aaaataaatt 7440
ttattttaaa tgtttggtgt tttgtgtata aataagtttt tttgattttt atggaatttt 7500
tagttaaagg attagatcgt aaagagtttt ttggtttgtt agagaatttg aatgtaggga 7560
atgttgtttg ggaatattaa agtatggttt atttaagggg tttttagttt tttatagata 7620
tgatggggga agttgtttga gtatatattt gttttcgtta tatatatcgt attttaaaaa 7680
ggtatatttt tttttttaaa gtttaggtat ggagagaagt gaaatttgat atatttaata 7740
tagaaatgga tattatttgg gggtgttaat attttgggga tagggttcgg gttgattagt 7800
tgaagggagt tggggattag tagggagggg gaaaggagga agagggtaga ttgggtatgt 7860
taagttgttg agaattgttt ttaaatattt ttaaggtttg cgttggtgat tttttttttg 7920
agttaagagg ttgagaggtt attcgagttg tttttgtttt attataatta aacggagaag 7980
aaaggtatat ggaagggttt tgttaattta tgttatattt tttttttttt tttttttttt 8040
ttagatt 8047

<210> 343
<211> 8896
<212> DNA
<213> Artificial Sequence

<220>
<223> chemically treated genomic DNA (Homo sapiens)

<400> 343
   tttggtggga cgtatgatag tttaggttta ggaaggggat tcgtatttta attataaaaa 60
   ttgtgttatt cggttttttt tttgggggta tgtggttgga gagaatggcg ggagattaat 120
   aatattcgtt tttttaggtt tattaatttg aaagaattgg gcgtttagat ttgtgtgtgt 180
   ttagtagttt tagggttgtt cgtttgagtt ttaggttcgg agattggata gaggaggtat 240
   tcggagatcg agtgtttatt ttggattttc gagttttttt cggttagttt gagcgagata 300
   attatggttt gtagtttttg tttatttaag gcgtgaaatg tttaggagtt ttattttttt 360
   tttttttttt ttttaggcga atttttggtt ttttattttt cgaggtgggg aaatttgtgg 420
   tgagttatcg tcgtgaggtt ggagttggcg tttaaagggg aagggcggtg ttagatgaga 480
   gtgggttttt tgtttggggt attttaggtt ttcgcgggcg gattattcgt tttatttgta 540
   tagagttgga gtttggggta gttttatgtt agatcgtttt tcggtgtcgc ggagggaagt 600
   tagagcgggg agggagtttg tcggtagacg gattttagag cgtgggagtt gggtagggtt 660
   ggtttttggt tttgtgtcgc gggataagtg gcgatttcgt attagtttat ttattattat 720
   cggttattgt tgttggtagt tagggttgcg ggtagagcgg ttagcgttag tagtagcgta 780
   gtagaaattt agttaggtaa tagcgtttag agttttatgg tttgtttggt tttgttcgtt 840
   ttgtgttgta gttgcggcga ttttggttgt tgttcgcgac ggtgggacgg gacgcggcgg 900
   tggcggtagt ggcgagagtt cgtagtttgg ttttaataat tttgggggcg ggcggcggat 960
   agtatggttc gcggtttcgg ttggtgggtt gaggcggtta cggcgggcgg tatcgttttt 1020
   tatagttgcg gcgttggttg aagtgacggg aggaggttgt tttttcgggt cgtattgtgg 1080
   tattagggcg tatcggttag gggcgtagat aatgggtagc gagcggggtt gtggttttcg 1140
   ttcgtttgag tcgagagttg acgggttggt cgtcggggtt tattttcgtt ttttgttcgg 1200
   tggttttcgt tttcgggttt gtattggtcg cggatttttt tttttgggat atttttttac 1260
   gcgcgttcgt tttatttagt tgagcgttcg gagttcgtcg aggattagta ttcgttaggg 1320
   ggcgcggcga gagtgtgttg cggggagagt tcgttagggg tttagttcgt ttcgtcgttc 1380
   gcggtggggt tgttttttcg acgttatttc gggttttttc gacgtttttt ttttttttta 1440
   ttttgagtgt cggaaggaaa gttgtggttt tgggtagcga tttcggggag atgtttaatc 1500
   gtttaggaat tttcgatggt tggatagaga aggaaaaagg ggagacgggg gcgtgagtgc 1560
   ggttgtggtt agggaaattt cggggatgag ttgtgggggt gagttaatta aaaggagttt 1620
   agcggtggag agaaatttaa agtaataggt agtttgttta tgtgcgttag aagaagcgta 1680
   taagtggttg tgtgtgtgtg tgtgtgtgtg tgtgtgtgtg aagtggaata acgtgaatat 1740
   gggtgggagt gagaatgtgt tgtgttttga gggtgtattt ttttgtgtgt tggtacgtgt 1800
   agaaattttt tgttttgacg aaggatgtta ggaaattttt taaagaattt tggaatttag 1860
   tttaagttag aaaaggttat ggagttattt tgttatagtt atcgtttggg gtagtgtgtt 1920
   aggaaggagg tggtaattgt cgataggaga tttagaagta tgattgaggg atgtagagtg 1980
   tttggattgt gtgtggagtg ggtggatgag tatgtgtatg ggaagattaa atatgtgtgt 2040
   atgcgttatt ttgttgtttt ttgtatttcg tggtatttta tggcgtgtta tattgtggta 2100
   tgtgttaatt agtgtatgtt attgggggag agagtcgagt ttataattaa gatggtattg 2160
   aggtgaattt agtttggagt ggttaagttt ttgaggttta ggtttagaaa ggatgggaaa 2220
   tggtgcggat gtttattgta tgtatggtga tggagaggat tgggaagata agggttgttg 2280
   gttatattga atttagatga ggaaatttgt gtttttatgt gtttgttttt ttttggatgt 2340
   ttttgttaag tttttgtttt ttttttaaag ttttagtttt tattatattg ggatatttag 2400
   agtgtttgtg attattttat ttattgtata tttttttttt tttttttagg gatttttatt 2460
   tagttttaag gttatttagg ttttttatga agaagtgata ggttgggcgc ggtggtttat 2520
   gtttataatt ttcgtatttt gggaggtcga ggtaggtgga ttatgaggtt aggagatcga 2580
   gattattttg gttaatatag tggaatttta tttttaataa aaatataaaa attagttgag 2640
   tgtggtggcg tatatttgta tttttagtta tttaggaggt tgaggtagga gaatggtttg 2700
   aattcgggag gtggagattg tagtgagtcg agattacgtt attgtatttt agtttgggtt 2760
   atagagtaag attttatttg aaaaaaaaaa aaaaagtggt tgtttttttt tatatgggga 2820
   atagggggta ttgttagttt ggttattatg ttttattatt atatatatgg tttttggaag 2880
   gagggaagat tttttttttt tttttatttt gtagaattaa aatagattaa ggtatttgtt 2940
   taaggtttac gtttaataaa tgaattaatg gtagagttag attaaaattt agattaattt 3000
   gatttaaagt ttatgttttt tcgattttat tagttgtttg ttagtgtgta gtaattatag 3060
   gttgggaatt ttttttaaaa ttgatgggta aaaataattt tttgggtttt tttgtttttt 3120
   ttttgagttt tagtttttat tttttttatt tttttgtttt tttaggatgt tttttgattg 3180
   agaaattttt ttaggtttga atttaaagtt ttaagtttta gaaatgatta ggattttttt 3240
   ttaaggtagg gaaattattt taaggaataa atgttaggta agttttatta atattttcgg 3300
   gtatttgtgt tttaaatgag atgtatatta atttaaatga agtttgatgg aagttatttt 3360
   agaaagaaat gttttttttt tttttttttt tttttttttg tttagtttag tatatttggt 3420
   ttttttaagg atttatttta gagggaaaag gtgtagtggt ttagtatttt tcgtagattt 3480
   aaaagttagt agtttttgta gagagtattt agtatagata gtttaagtaa atggataatg 3540
   tattacgggg agtttgtttt ttatttttta tgagtttttt tttattgggg tttgggttat 3600
   attttttggt ttggtggatt gatataatat cgtgagggtg ggaggttggt tggaaaatat 3660
   ggaagttttg tttttgacga ttgttgttaa gtagaggtgg gaagagagga aaggagttat 3720
   tgtgttttgt tttaatattt tatatattag gagtaggggg taagaggtta aggtttttag 3780
   atttgggtta tattgtttat gatttggttg atattttttt tttaaaatat gaaatgtttt 3840
   ataaatttgt atgttatttt tgtgtagggg ttatgttaat ttttatatta ttttaatttt 3900
   agtatatgtg ttgttaaaat aagtatttaa tttttttatt tagattaaat aattatgata 3960
   ttaattgttt tttttttttt tttttgtatt atttatttat tttttttttg gaatatatat 4020
   ataagtatgt agatatgttg taatagtatt tatttgaaaa atttaaaaat tttattagat 4080
   tttatattat tgtttaagat tgttttattt tagtgtttta ttttattgta aaaattaaaa 4140
   gggttgattt aattattaga gttaattttt tttttttttt tttttttttt tttttttttt 4200
   gagacggagt tttgtttttg ttgtgtagat tggagtgtag tggtatgatt tcggtttatt 4260
   gtaattttta tttttcggtt ttaagcgatt tttttgtttt agcgtatcga gtagttggga 4320
   ttataggcgt ttattaatat ttttatttaa tttttgtatt tttagtagag gtggggtttt 4380
   attatgttgg ttaggttggt ttcgaatttt tgatttcgtg atttgtttac gtcggttttt 4440
   taaagtattg ggattatagg tatgagttat tgcgtttagt ttagagttat tttttttaaa 4500
   atagaagttt gattagttta tttttttgtt taaaattttt tagcgagatt ttatgttatt 4560
   taaagtaaag ggtttttttt tttagttttt tagttttatt gtttattatt tttttttttg 4620
   cgaatttttt tttattagtt ttattggttt tttttttaat tttttgaatt aagtatgttt 4680
   ttttttaagg gtttgtgtat ttgttttttt gttttttaaa atgttttttt ttagattttt 4740
   ttatgtattt gtttttttag tttgtttttt tttttttttt ataaaataga attttattat 4800
   taattttttt tattttgttt tatttttttt tgtattagtt ttgatcgtgg atgattttaa 4860
   aggtttgaag ttgaaaatta gtagtaaatt atttttttgg aataggttta gaggtgagat 4920
   gttagagggg agaggggttt tgttttgatt ttgggatttg gggaggaagt agggttttag 4980
   ggtttagagt ttgtgggtag aggagtttat ttttttttga agtattcgta ggtagtattg 5040
   tttttttggg ttgggaaggt atagtttatt tattaggttt gagtatagaa gggggtaaaa 5100
   ttttgtttaa ggtaatttta tggatttgtt ggagtggatt gttgaaagtg gggttttttt 5160
   gagagtttag tatgtagtcg gttcgtagat tttttggtgt ttttaggtgg tggtttacgg 5220
   tgttgtagta gaaggcgtat taagcggagg gtgggtagcg tagttgagtt tatcgttacg 5280
   ggcgtattta ttttttttgg gtttataatt tgggtttttc gggtttggtt ttttttatta 5340
   gcggtttagt aatagatgag ttattcggga tttcggttta aggtaaatat ggggggagga 5400
   ggaggaggag gtttttgagg ttttagttat cgttttagtt gttcgatatt ttttaatttt 5460
   taggggtttt tatttttttt tttgattttc gttttttagg gttttagtgt tttttttata 5520
   ttgttttcgt tatatttttt atttttagga tgggaagggt agttttttat tttttttatt 5580
   gaggggaagg ggaattagtt tgtttttagt ttttttttgt agtgatgaaa gttttttatt 5640
   tttttttttt tgttattggt tttgatttac gttaaggttt ttttggtttt cgtttttttt 5700
   tttttttgga gttggtcgcg tgtttggggt attaggtaat gggtgtgtat ttatagtttt 5760
   ttttaggttt cgtttgtttt atcgtttttt agtcgtttgc gggttagtgt ttaggttagt 5820
   cggtcgggtt gagggtattg cgggttagtt taggttattt agtttgtcgg tttgtggttt 5880
   tttgttttag agttgggttt tagtagagag aaggagtagg attttgggtt tttggatttg 5940
   gaatagtttt gtttggggag atttagggtt attaggaagt tgaaatttat tgtagtagtt 6000
   ttgtttttcg tttttttttg gtatttggtt gtatgttgga gtttgataag ggtagaatta 6060
   gaaggtaaaa aataatttta aatttaaggg ggatttagag atatatggag aaatataaat 6120
   gtttatgagg tttttatgga gaagagaggt ttaggtaatt ttattttttt tttttaagga 6180
   tttggtagga attttagggt gattgtaaga aaaaggataa ggatattggt ggggattatg 6240
   aatttaggtg taaggatgaa aattatgaga tggggtttgt agattttggt gatttttaaa 6300
   attatatatg gtttggtttt tgttttttaa atgggaagga gggttaggaa ttttagtgtt 6360
   tttatttttt tattttagag gtagggaatt agagaaaagt agtttggttt aggttgttta 6420
   gtgttaggga ttatggtatt tttatttttt tagattgttt atagtttggt acgtaaaggt 6480
   gatttgtaaa tattttaata gatgtaagtt tgttcgattt tttgttttag attgttgtgt 6540
   tcgttattta atagtgattt ttgtagtacg ggtaggattt ttagtagtat agattggagg 6600
   ttggttgttt agtagagttg taacgttatt attaaggaag aggtgattgt ggttgtgggt 6660
   attatttttg tttttttgtt tgtttggggg tagtttgagt taatgtgttt ggggcgcgtg 6720
   gggttagtcg tgttttgggt tgcggtttgg taggggcggg gggcggttgg aaagtttggg 6780
   gttttttttt ggtaaaaata gtttatagtg gttagagttt cgagggtgag ttaggcggga 6840
   gtacggtggc gtttaattcg ttcgttgtta ttgggttttt ttaaggcggt ttgtttttgt 6900
   ttgtgtattt taagattagg ttttttttcg gttgtttttg ggtttggttt aattttggat 6960
   tttaaggttt tttttatttt ataagaagta agtttatagt ttggagtagg aatttaggtt 7020
   atttaaagtg tagatcgggg gaatttcgag aagggtagga tatttttgtt ggtaagtatt 7080
   tttgttaggt aaggaagtat cggggtaggt tgataatttt tggtgttatt tgaatttttg 7140
   ttagggttaa attttgaggt taaagttgtt ttttttaaag gggtaggcgt agggtttgga 7200
   ggagggtggg aattaagttg tttagacgtg tgtttttttt gttcgttttg agtatttcgg 7260
   gtgcgtttag gttgtgggtg ttatatgtga ttgttttata tcgagaagtt taaagattgt 7320
   ttggttttat ttttattttg ttaaaggtat tttttagtta ttttgatttt ggtggagaaa 7380
   ttgacgtaaa gaggagtgat gaaagatgtg gttatttggg ttttattttg gttttttagg 7440
   aaggttatta tatttttttt gtagttttgg cgattttaga ttaggtgaga ggaattttgg 7500
   ttattttttt ttttagtggg gttaagtttg aatttaggcg tcgggggaga gggtgtgtga 7560
   gagagagaga gtgtgtgtgt gtgtgtgtgt gtgtgtgtgt gtgtgtgcgc gcgcgcgcgc 7620
   gcgcgttggg aggaggggga ggttgtattc gtttggttaa ggttgggagt ggagggtagg 7680
   gtggggaagt tttaggttcg ggagggggtt gtagtttcgt tagttcggta gagttatttt 7740
   gagttcggtg agagtaaagt tagagttttt agttttttgt tcgtcggttt gttagttttt 7800
   tcgattattt tttttttttt tttttttttt tttttcggcg gtcgcggcgg cgttggggaa 7860
   gcggtgaaga ggagtggttc ggttttggaa gaatgcggtt ttgataaggg gatagaattt 7920
   agcgtagttt ttttacggtt taagtagtat tagtgaagtt taggtaattt aatcgtgttt 7980
   gtttcggatt tcgtatttaa attattggag gtaattagat ttgggaaagt agggagggta 8040
   gggacgtgta tacgaggaag agataggtac gtgtgtagtt agatagatag atagatacgt 8100
   atttatttat agaagtatag agacgtatag agagttagag atattgttag agataaagat 8160
   atagagaata aaggagtgtt tttagattta gaatgtaatg aatgtgagag ttaaggggtg 8220
   aggattagac gggcgattat ggaggagggg agtttgtagt taggtaaggt gggggatagg 8280
   gaagaggatt tagtttgtgt tacggttttt tttaggaggg agttaggttt ttttttttat 8340
   tttttttgta tttggagttt tttttttttt tttgttgttt agggaggggg tagtgtgagt 8400
   ggggaattta tttttagatt ttttttatgg agtttatttg tttttgatga tatttggagg 8460
   tgatattttt ttaggtggtg gggaattttt tttttatttt tttttttagt tttgtgaggg 8520
   cggggaagtt agtcgtgggg ttttggaaaa gggagtagta ggcgaggttg tggagtaagt 8580
   gtttgtgagt ttgtgtgttt gagtttgtaa gtgagtgtgt gtttgtgtgt cgggcgttgt 8640
   gtttgattgg gtaaggtttt aggggtgttc gtttgagttt tgagttggga taacgttttg 8700
   attttttttt tttaagattt ttaagtttta gggattttgg gaattaaggg gtgttttttt 8760
   ttgttttgtt tgaggaggaa tgagaagggt tagtagggtg gtatcggtgg gttaggagtt 8820
   cgaagaaggg cgttatttgg ttttttaagc gagtcgtttt atttttgaat tcgtattaag 8880
   tttttcgttt cgtagg 8896

<210> 344
<211> 8896
<212> DNA
<213> Artificial Sequence

<220>
<223> chemically treated genomic DNA (Homo sapiens)

<400> 344

tttgcgggac gggagatttg atgcgggttt aggaataggg cggttcgttt ggggaattaa 60
gtgacgtttt ttttcgggtt tttaatttat cggtgttatt ttattgattt tttttatttt 120
tttttaaata aaataagaag aaatattttt tgatttttag agtttttgag gtttggaggt 180
tttaaagaag aagagttaag gcgttgtttt agtttaggat ttaagcgagt atttttggaa 240
ttttgtttaa ttagatataa cgttcggtat atagatatat atttatttgt aaatttagat 300
atatagattt ataaatattt gttttataat ttcgtttatt gttttttttt ttagggtttt 360
acgattagtt ttttcgtttt tatagggtta gggagaggga tagggaggag gttttttatt 420
atttaagggg gtgttatttt taggtgttat taagggtaag taggttttat gagaaaggtt 480
taagaatgag ttttttattt atattgtttt ttttttgagt agtaaggaaa gaggagaggt 540
tttaggtgta ggaaagatgg ggaggagagt ttggtttttt tttaaaaggg atcgtggtat 600
agattgagtt ttttttttta ttttttattt tgtttaatta taagtttttt ttttttatag 660
tcgttcgttt ggtttttatt ttttggtttt tatatttatt gtattttaaa tttgggggta 720
tttttttatt ttttgtgttt ttatttttag taatattttt ggttttttat gcgtttttgt 780
gtttttgtgg gtggatgcgt gtttgtttgt ttgtttgatt atatacgtgt ttgttttttt 840
ttcgtgtgta cgtttttgtt ttttttgttt ttttaggttt aattattttt agtggtttgg 900
gtgcggggtt cgagataggt acggttgggt tgtttgggtt ttattagtgt tgtttaaatc 960
gtggggagat tgcgttgggt tttgtttttt tgttagagtc gtattttttt agggtcgggt 1020
tatttttttt tatcgttttt ttagcgtcgt cgcggtcgtc gggaggaagg gagggaggaa 1080
gggagggagt gatcgagaga gttagtaagt cggcgagtaa aggattgggg gttttggttt 1140
tgtttttatc gagtttaggg tggttttgtc gggttgacgg agttgtagtt ttttttcgag 1200
tttggggttt ttttatttta ttttttattt ttagttttga ttaggcgaat gtagtttttt 1260
ttttttttta gcgcgcgcgc gcgcgcgcgt atatatatat atatatatat atatatatat 1320
atatattttt ttttttttat atattttttt tttcgacgtt tgggtttagg tttggtttta 1380
ttgaagaagg aggtggttag agtttttttt atttgatttg gggtcgttaa ggttgtaggg 1440
agggtgtagt aattttttta gggagttaga atggggttta gatggttata ttttttatta 1500
tttttttttg cgttagtttt tttattaaga ttagggtggt tggaaggtgt ttttgataag 1560
atgaggatgg agttagatag tttttgggtt tttcggtgtg ggatagttat atgtggtatt 1620
tatagtttga gcgtattcgg ggtatttaga gcgggtagga gaagtatacg tttgggtagt 1680
ttggttttta ttttttttta agttttgcgt ttgttttttt ggaggggata gttttggttt 1740
tagagtttgg ttttagtaga ggtttaagta gtattaaaga ttgttagttt gtttcgatgt 1800
ttttttgttt ggtagggatg tttgttaata gaggtatttt atttttttcg ggattttttc 1860
ggtttgtatt ttgggtggtt tggatttttg ttttaagttg tagatttgtt ttttatggga 1920
tgaggagggt tttgggattt aaaattgagt taagtttaag ggtagtcggg aaagagtttg 1980
gttttggggt gtataggtaa aggtaaatcg ttttagggag atttagtggt agcgaacggg 2040
ttaggcgtta tcgtgttttc gtttgattta ttttcgaagt tttaattatt gtgggttatt 2100
tttgttagag aggggtttta ggttttttag tcgtttttcg tttttgttag atcgtagttt 2160
aaggtacggt tggttttacg cgttttaggt atattgattt agattgtttt taagtaagta 2220
gaggaatagg gatgatattt ataattatag ttattttttt tttggtggtg gcgttgtagt 2280
tttgttgggt agttagtttt tagtttgtgt tgttggaggt tttgttcgtg ttgtaggggt 2340

tattgttgga tagcgggtat agtagtttga gataggagat cgagtaaatt tatatttgtt 2400
gggatattta tagattattt ttacgtgtta ggttgtgagt agtttgggga ggtgaaagtg 2460
ttatggtttt tgatattgga tagtttggat tagattgttt ttttttgatt ttttattttt 2520
gggatgggga agtgggagta ttagagtttt tgattttttt ttttatttag aggataagag 2580
ttaaattatg tgtggttttg gaggttatta gaatttgtaa gttttatttt atagttttta 2640
tttttatatt tggatttatg atttttatta gtgtttttat tttttttttt gtagttattt 2700
tgggattttt gttaggtttt tggaagaggg gaatgaggtt gtttaagttt ttttttttta 2760
tggggatttt atggatattt gtattttttt atgtgttttt gggttttttt taggtttggg 2820
attatttttt gttttttgat tttatttttg ttaggtttta gtatatagtt aggtgttaag 2880
aaggggcgag ggtagaattt attataatag gtttaatttt tttggtgatt ttaggttttt 2940
ttaagtagga ttgttttaaa tttaaggatt tagagttttg tttttttttt ttattgaagt 3000
ttaattttgg gataagaggt tataggtcgg tagattgggt ggtttgagtt ggttcgtagt 3060
gtttttagtt cgatcggttg gtttgagtat tgattcgtag gcggttagag ggcggtggga 3120
taggcggggt ttggagaggg ttgtgggtgt atatttattg tttaatattt taagtacgcg 3180
gttagtttta agaaagggga gggacggaaa ttagagagat tttgacgtgg gttaggttta 3240
atgataggag ggggaaagtg gggggttttt attattgtag ggagggatta ggagtaagtt 3300
ggtttttttt ttttttaatg agaagagtgg agggttgttt tttttatttt agggatggag 3360
ggtgtaacga gagtaatgtg ggaagaatat tggggttttg gaggcggggg gttaaagaaa 3420
agagtggggg tttttggaga ttgggggatg tcgagtaatt gaggcggtgg ttaaagtttt 3480
agaagttttt tttttttttt ttttttatgt ttgttttggt tcggggtttc gggtgattta 3540
tttgttgttg agtcgttgat aggggaggtt agattcgggg aatttaaatt ataggtttag 3600
gagggatgga tgcgttcgtg gcggtgagtt tagttgcgtt gtttattttt cgtttaatgc 3660
gttttttgtt gtagtatcgt aggttattat ttggaggtat taaagggttt gcgggtcgat 3720
tgtatattgg atttttagga aggttttatt tttagtagtt tattttaata aatttatggg 3780
attattttag atagaatttt gttttttttt gtatttaggt ttaatggatg ggttgtgttt 3840
ttttagttta agggggtagt gttgtttgcg ggtgttttaa aggagggtag gtttttttgt 3900
ttataaattt taaattttgg agttttgttt tttttttaga ttttaaagtt aaggtaaagt 3960
tttttttttt tttaatattt tatttttaat tttattttag gggggtggtt tgttattgat 4020
ttttaatttt aagtttttaa agttatttac ggttaaaatt gatatagaga aaaatgaagt 4080
agggtaaagg agattagtag tgggatttta ttttataaag ggggagggaa aataaattga 4140
aggaataaat atatggagag atttgaggaa gagtatttta gaagatagaa aagtaggtgt 4200
atagattttt agaaaggagt atgtttggtt taaaggatta gaaaagaggt tagtgaggtt 4260
agtggggaga aattcgtaag gaagagagtg gtaggtaatg aagttggagg gttaggaagg 4320
aggttttttt attttgagtg atatggggtt tcgttggaaa attttgagta gagaaatgaa 4380
ttaattagat ttttgtttta ggaaagatgg ttttgggttg ggcgtagtgg tttatgtttg 4440
taattttagt attttgggag gtcgacgtgg gtagattacg aggttaggag ttcgagatta 4500
gtttggttaa tatggtgaaa ttttattttt attaaaaata taaaaattag gtgggagtgt 4560
tggtgggcgt ttgtaatttt agttattcgg tacgttgagg taggagaatc gtttgaaatc 4620
ggaaggtgga ggttatagtg agtcgagatt atattattgt attttagttt gtataataag 4680
agtaaaattt cgttttaaaa aaaaaaaaaa agaaagaaag aaagaaagaa attggtttta 4740
gtaattaaat taattttttt gatttttgta gtaaaatgga gtattgaaat ggagtaattt 4800
tgggtagtaa tgtggggttt aatgaagttt ttgggttttt tagatgggta ttattgtagt 4860
atgtttgtat gtttatgtgt atgttttagg agagagataa gtggatgatg taggaaagaa 4920
aagaggggat agttgatatt atgattattt gatttaaata gaaaagttgg gtgtttgttt 4980
tggtagtata tatattaaaa ttggaatgat atagagatta gtatggtttt tgtataagga 5040
tgatatgtaa atttgtgaag tattttatat tttgaaaaag aaatgttagt taggttataa 5100
atagtgtgat ttagatttag ggttttattt tttttgtttt ttattttttgg tgtgtggaat 5160
gttggaataa agtatagtgg tttttttttt ttttttttat ttttgtttga taatagtcgt 5220
taaagatagg gtttttatat tttttagtta gttttttatt tttacggtgt tgtattaatt 5280
tattaggtta aaagatgtga tttaggtttt agtgggaaga aatttataag ggataaagga 5340
taggtttttc gtgatatatt gtttatttat ttgagttatt tatgttgggt gttttttgta 5400
gggattattg gtttttggat ttacggaggg tgttggatta ttatattttt tttttttggg 5460
gtggattttt ggaagggtta gatatattag gttgggtaaa agggaagaaa aaagggaaag 5520
aaggatattt ttttttaaaa taatttttat taggttttat ttgggttaat atgtatttta 5580
tttaaaatat aagtgttcgg gaatattaat gaaatttatt tggtatttat tttttagagt 5640
gatttttttg ttttagaagg gaattttagt tatttttggg atttgagatt ttaggtttag 5700
gtttggggaa attttttagt tagaaggtat tttaaaagat aagggagatg aaaaaaatga 5760
gagttagaat ttaaaaggga ggtagaaagg tttaaaaaat tatttttatt tattaatttt 5820
gagaagggtt tttagtttgt aattgttgta tattggtaag tagttggtaa ggtcgaaaga 5880
gtatgggttt tgagttaaat tggtttgggt tttaatttgg ttttattatt gatttattta 5940
ttagacgtgg attttggata agtgtttgat ttatttttaa ttttgtaaaa tggggagaga 6000
gaagagattt tttttttttt taggggttat gtgtgtggtg gtggggtatg ataattaggt 6060
tggtagtgtt ttttattttt tatataggga aaagtagtta tttttttttt tttttttaga 6120
tgaagttttg ttttgtagtt tagattggag tgtaatggcg tgatttcggt ttattgtaat 6180
ttttattttt cgggtttaag ttattttttt gttttagttt tttgagtagt tggaaatata 6240
ggtgtgcgtt attatattta gttaattttt gtatttttat tagagatggg attttattat 6300

gttggttagg atggtttcga ttttttgatt ttatgattta tttgtttcgg ttttttaaag 6360
tgcggggatt ataggtatga gttatcgcgt ttagtttgtt atttttttat aggaggttta 6420
aatggttttg aagttgagta ggagtttttg ggagagaaga gaaaagtgta taatggatga 6480
gatggttata ggtattttgg gtattttagt gtggtgggaa ttagagtttt aggggaaaga 6540
tagaaatttg gtagaaatat ttaaagagaa gtaaatatat ggaggtataa gtttttttat 6600
ttaggtttaa tgtagttagt aatttttgtt tttttagttt tttttattat tatatatata 6660
gtggatattc gtattatttt ttattttttt tgagtttagg ttttagagat ttagttattt 6720
taggttgggt ttattttaat attattttgg ttgtaggttc ggtttttttt tttaatgata 6780
tgtattggtt gatatatatt atagtgtgat acgttatagg atgttacgag gtataaaggg 6840
taatagaatg acgtatatat atatatttaa tttttttatg tatatgttta tttatttatt 6900
ttatatatag tttagatatt ttgtattttt taattatgtt tttgagtttt ttgtcgatag 6960
ttgttatttt ttttttgata tattgtttta ggcggtgatt gtgataaggt gattttatga 7020
ttttttttga tttgagttaa attttaaaat tttttggaaa gttttttaat atttttcgtt 7080
agaataagga gtttttgtac gtattaatat ataggaggat gtatttttag aatatagtat 7140
atttttattt ttatttatat ttacgttgtt ttattttata tatatatata tatatatata 7200
tatatatagt tatttgtgcg ttttttttgg cgtatatgag taaattgttt gttgttttag 7260
gttttttttt atcgttaggt tttttttggt tagtttattt ttataattta ttttcgggat 7320
ttttttgatt atagtcgtat ttacgttttc gttttttttt tttttttttt tgtttagtta 7380
tcgggggttt ttgggcggtt aagtattttt tcggagtcgt tgtttagaat tatagttttt 7440
ttttcgatat ttaggatggg ggagagaggg gacgtcggag gggttcgggg tgacgtcgag 7500
gggataattt tatcgcgggc ggcgaggcgg gttgggtttt tggcgggttt ttttcgtagt 7560
atattttcgt cgcgtttttt ggcggatgtt agttttcgac gggtttcgga cgtttagttg 7620
agtgaggcgg gcgcgcgtgg gagggtgttt taaggggagg ggttcgcggt tagtgtaggt 7680
tcggaggcgg gggttatcgg gtagggggcg ggggtgagtt tcgacggtta attcgttagt 7740
tttcggttta gacgggcggg aattatagtt tcgttcgttg tttattgttt gcgtttttaa 7800
tcggtgcgtt ttggtgttat agtgcggttc ggaggggtag ttttttttcg ttattttagt 7860
tagcgtcgta attataagag gcggtgtcgt tcgtcgtggt cgttttagtt tattagtcgg 7920
gatcgcgagt tatgttgttc gtcgttcgtt tttagggttg ttaaagttag attgcgaatt 7980
ttcgttattg tcgttatcgt cgcgtttcgt tttatcgtcg cgggtaataa ttaaagtcgt 8040
cgtaattgta gtatagagcg ggtaaagtta ggtaggttat ggggttttgg gcgttgttgt 8100
ttggttgggt ttttgttacg ttgttgttgg cgttggtcgt tttgttcgta gttttggttg 8160
ttaatagtag tggtcgatgg tggtaagtga gttggtgcgg ggtcgttatt tgtttcgcgg 8220
tatagagtta ggggttaatt ttatttagtt tttacgtttt gggattcgtt tgtcgatagg 8280
tttttttttc gttttgattt tttttcgcga tatcgaaggg cgatttggta tgaaattgtt 8340
ttagatttta gttttgtata agtggggcga atgattcgtt cgcggaggtt taagatattt 8400
taggtaggga gtttattttt atttagtatc gttttttttt tttgagcgtt aattttagtt 8460
ttacggcggt ggtttattat aggttttttt atttcgggaa gtgaagggtt aggagttcgt 8520
ttagaaagga ggggagaaga gggtgggatt tttaagtatt ttacgttttg ggtgggtaag 8580
aattgtaggt tatgattatt tcgtttaggt tgatcggaag aggttcggag atttaaggta 8640
gatattcggt tttcgggtat tttttttgtt tagttttcgg atttagggtt taggcgagta 8700
gttttgggat tattgggtat atataagttt ggacgtttag tttttttaaa ttagtgagtt 8760
tgggagagcg ggtattatta atttttcgtt atttttttta gttatatatt tttaggaaga 8820
ggatcgggtg gtatagtttt tatggttagg gtgcggattt tttttttgag tttgagttat 8880
tatacgtttt attagg 8896

<210> 345
<211> 15734
<212> DNA
<213> Artificial Sequence

<220>
<223> chemically treated genomic DNA (Homo sapiens)

<400> 345

gggttttcgg gaacgtggtt gggtttgtgg ggtgacgcgg tagggagttt ttattttatt 60
gttttttaag gttggaaagt agggggaggt ttaagtagaa aagttgagga ttttagtttt 120
ttgcgttagt attttattta aggcgaattt ttcgattttg tagaaatata tagaatagaa 180
tgggttaggg atgagaggta tttaggcgaa agtgttttag ttgtgttgcg atagggggtt 240
gtgggagggt aaaaaaaaaa aaaaaggtga tcgcgggttg aggaaaataa agtttttatt 300
ttaaataatg gggtggtaga agtttgcggt cgttaaggcg agtttatttt taggggtaga 360
agttttggag gttattggtt ttttattagg tgtattttta tgtttgttcg gtagtttgga 420
gaaaattttt gtttcgaaaa aggttaattc ggagaagtcg tatgtgattt ttgagagtgt 480
tagataagtt tttgtgttgt atatttggag tacgttgtta taaatttagt tgtaatttcg 540
tagtaatttt ttatatatga aaattagatt ttttgtagaa gatattttgt aaatatgtaa 600
aaatgtattg gtaaatatta atattttttt aaaaagttgg aatcgaggat aaattttgac 660

ggagtaatta ttttattggg gttgttatta aatatatttt aaatgaaaaa attttattat 720
attttgaagg atcgatttaa attttttttt ttttttatat ttaatttttt ttattatttt 780
tttattagtt ttttatattt tttttttttt ttaaatatat agtgcgtggt gagtggggtg 840
aattttagag aaagtaggta ttgtttgttt gttgcgtaaa ttttgtttat tatagttatt 900
agtttttgtt gaaggttggt ttttaaatta tgacggagta aagtaataag gtaagaaatg 960
agcgtttatt tagtgagttg ttttttgtag cgggagtttt ttttataatg ttagagtcgt 1020
ttttagtttt aaggttagag gttgtttatt atgaataaat ttaagaatat tgtaatttaa 1080
agggtgtgtg aaaaggtttt aatatttttt taatagggga agggttattt taatggttgg 1140
atttagaaaa ttttaaaagt atgttgttta gtaatagatt gtatttgata gatttaaaat 1200
agatatatta aatatttttg aaaatgttga tatttttaaa gatgaatggg aaaagaatat 1260
tattgtttgt ggaaaaattt tttaattatg tataaatgta tggatatgag aagttttgat 1320
tttttttttt ttttttgtta aagttagata tgtaaaagta gttttatttt tttttttgtg 1380
ggattgtagt ttaattttat cgttggataa tttagagttt tgaaagtatt gagagtgttt 1440
tttagatata aattgtttat tagtagtttc gattttagga tttggtattt ttttattagg 1500
aatttgtttt ttataatagt ttatttttta ttgtttataa gggtttattt agaaggagta 1560
tttagtgaat tgtagtttaa tttttgtcga aggttttaat ttatgggttt ggtgtttatt 1620
ttggttttat agggttggta ggttattatt cgggtagaaa tgggtaatat ttttttttaa 1680
ataaaatatg ggttttggag gtgattgtgt aatcgttgta taaggtttgg ttgaggtaag 1740
taggtattga agatttaggt taaagttatt ttgagggaga gggagaaatt tagtagttgg 1800
gaagaatggt aaaaaagaaa gaaggtaaat gagtacgagg gaggaaaagt gtttggaagt 1860
cgatttatat tataattgaa tcgttttttt tattttttgg ggtggggaat tttaggaaag 1920
tcgaggcgtt taataaatgg aatattttat tgagaaattt aatattttag tgttaaagtt 1980
tagagagtat gggagttttg ggggtcggtt agggatgttg ggggttagga gattaattga 2040
gagggttaag gaggcgttta atttgagttt ttgttgcgtt agtatttttt tatttgaatt 2100
agtagtagtc ggttcgtttg tagtcggtta gtttatcggt agtgtttcgt tgcgtttttt 2160
atttagtaag ttttggtggg agttggaagg tagttttggg ttagagaggg gttgttatat 2220
atgagtttat atattttttt taaaagttag aaggagttta agtttaatat ttcgtttggt 2280
ttattataag gggaaaaaag ttatattaaa gaaatatatt tggtttatta tagaaaggtt 2340
attgaagatt taagtgttaa cgttacggtt tgcgaggttt atagtaggtt ttttagtttt 2400
tttatttttc ggttggggta gttttttgta gtagatgcga tataaattga tttttgtaat 2460
tttggttggt acgtgtaggt ttttggattt tatttagttt agaagtttat ttggttgttt 2520
tacggggttt tgatttgagt tgtttgttgt agggtattta gtttgttttg ttagttaaat 2580
tggtagggtt tttaagtttt aggatattgg gggattttgg aagatttaag gaaaagattg 2640
aggggtgtgt gaattttttc gaaatgatgg attaaattgt gggaatatat agatatgtat 2700
tttttataga atgagttaaa atttatttgg attttttaga agggttttga ttttttaaaa 2760
ggttaggaaa gataggttaa gattattgtt ttttatttta tttgtatagt ttttattatt 2820
ttaaatatat attggatttt tttagatgtc ggtttgggaa gttgtttttt gtatttagtt 2880
tggagaggta tgattttagg tttaggtttt ggtttattga tttgttacgg tttattattt 2940
ttgaggaaat ttcgaaggtt ttgggattta tttgttaaat gatatagttt ttttgagagg 3000
tatgggtagt gttgaagagt aaagtttttt ttttaggtag tttttagttt tgtggtattt 3060
ttaggtggta ttgttaaggg taatagtttt agttagtttt atttttttgg tgagtagggt 3120
aaggattagg tagattcggg aagttaaaga tagggttatt ggttgcggtg ttttgaagtt 3180
tttttgtatt taggttcgtt cgttttatgg gtttagaggg tagagaattt gttttggttt 3240
tgggcgtagg agtatgcgaa gagcgaagga gagttaatga ttttttggta tagattttgt 3300
tttgattttt ttaaagtcgt tgttgtagtt ttaagatttt gttaggttgg agagaattgg 3360
tttttttatt tttttttttt ttttgttttt tttttttttt tttttttttg ttcgtttttt 3420
tttttatttt tttttttttt tttttatttt tttttttcgt attttgatgt ttagttttta 3480
cggtcgggtt atcgtggggc gtcgaggttg aagtggttgg agcgtcgcgt tagcggtgga 3540
gcggtttttt gggttcggag ttcggagtag ttattgttcg tagcgatcgt agttgtttga 3600
attttagaag ttagagttgg agttttattt agttagtttt ttttgcgttt ttagggtttt 3660
tttaggtttt ttttttattt tgggttggat ttatattggg aggaaggagg taaagtgtcg 3720
cgtatgattc ggatgtaaga gtttttttta gttgaaggta gcggtttgag cggtttattt 3780
tttgggatgt cggcgtttag taatgttagt ttcgattggg gtcggagggg cgggtaggtt 3840
tagagtattt tggagatttg tagtttacgg gttaggggta aggtagcgat ttttcgggtt 3900
ttattggatt tggtcggaaa aggttgtatt ttttgagatt agtatgaggt ttatatttgg 3960
gggttagtat tttaggtcgg ttatttttag ttggttaaaa tagagttttt gagtttgttg 4020
agtaaagggt ttgattttta tatgttttta taataggatt atgagtttag ttcggttttg 4080
tagggaaagg atttttttat cgaggtttcg ttaaaatgta tatattgtag gagtaaggat 4140
tgttttagtt gagggtatgg gagggagggg aagaggggag gggatagaga ggtaggagtg 4200
tagtgatatt ggtttttgtt gtagttttta ggagtatatg agtgttaaag ttaataggag 4260
agtaggaata taaagtagcg gaggtgtata tcgggtatat ttttttaagt tttttatttt 4320
gggttagtta aggattttaa ggttagtatt ttattttttc gaaatgattg tgggtttaga 4380
gtttttttta ggtgatgagg ggatgtttgg atttggtttt ggttttggga gtgggagtgg 4440
ggtcgttttt ttattaaggt tggtagtcgt tttttatttt tagtttcgtt agtttattaa 4500
agtttattga taggagttgt agagaaggta agtcgggtga gtataatatt agaggagaag 4560
attttaattg gattaacgga ttaggggaga aaagtgttgg gggttagggg ttagggaata 4620

attattgtat tttggggaaa aatttgtgga tattggaagt agaggtaagt tagtttttag 4680
tttattttgg gtttttgata ttttgaggat tttgacggtt atagggttag gttcgttatt 4740
tttgaggagg tagaatttag ataaggggtt agttttagat ttagagttga atgggaagcg 4800
cgtgagtttt cgatacggtc gttttagtgg ttagagtata tcggggatta gtggtagttt 4860
ttgcgatagg gatggtgagt atttaaggta gattgggcgc gtattataaa tattttattt 4920
gttaattgcg cggggtttaa gggttttttt aagggatttc ggtatggggt ttacgttcgg 4980
gtaggggcgg ggcgagttta atggttaggt tttttattag ttacgttggg gtagttttta 5040
tagttttcgg ttttcgggtt aaggtgtcgg ggtgcgtttt ttggtttatt aatatagatt 5100
atatatttat attaatcgcg ggttttgagg gcgttttcgg agagcggttt cgcgtttacg 5160
aaattaaatt gggagtggtc gcgcggaaat tttggttcgg gattggttgc gggcgttcgt 5220
cgcggtgcgg ggggattgtt aatcgtattt agtatgtttt gtataagaaa tgttagttag 5280
aaagggttat ttgttttttt cgttaaatta ttttataata atgttatgtt cggagagttc 5340
gtcgcgaatt tttttttggt cgattcgttt attagttcgg gtagaggcga ggtaggcggc 5400
ggtggtggtg gcgcgggggg cggcggcggt ggcggttatt acgtttacgg cggggtttat 5460
ttgtcgttcg tcgtcgattt gttttacggg ttgtagagtt gcgggttttt ttttacgttg 5520
ggcggtaagc gtaatgaggt agcgtcgtcg ggtagcggtg gcggtggcgg gggtttaggt 5580
ttcggggcgt acggttacgg gttttcgttt atagatttgt ggttagacgc gtttcggttt 5640
tgtcggatgg agtcgtttga cgggtcgtcg tcgtcgtttt agtagtagtc gtcgttttcg 5700
tcgtaattat tttagttagc gtcgtaggtt atttcgtgtt ttttcgcgta gaatattaaa 5760
gaagagagtt tttattgttt ttacgattcg gcggataaat gttttaaagt ttcggttatc 5820
gtcgtcgaat tggttttttt ttcgcggggt tcgtcgttcg acggttgcgt tttgggtatt 5880
tttagcgggg tgttagtgtt tggttatttt cgtttttttt aggtttacgg tatcgttaag 5940
ggttatggta gcggcggcgg cggcgcgtag taattcgggg ttggttcgtt tttcgcgtag 6000
ttttcggggc gcggtttcga tttttcgttc gcgttagttt tcggttcggt cgatgcggtt 6060
cggaaggagc gagttttcga ttcgtcgtcg ttttttacgt tggtttgcgg tagcggcggg 6120
ggttcgtagg gcgacgagga ggcgtacgcg tcgttttcgg tcgcggagga gtttttttcg 6180
gtttttttcg agagtagtaa agtttcgtcg gagaaggatt ttttgggtaa gtagggttgt 6240
agagggttgt agttaggcgg gtagataggt agatataagg aggagaagga ttagaaaatt 6300
aggagttcgc gtagtagtcg gtcggttttg gtttaagttg taggtaggtt gattttgtga 6360
atttgttttt taatatttgg gcgtggggac gtagtaaaat ttatgttcgg tttagcgttt 6420
tatagtaaga cgttttcggc gttggtttta gttttttttg attagggacg aggatattag 6480
cgagtaggtt tttttttgtg cgtttttttt tgtggtcggg aggatttaga gttttggttt 6540
ttgtttagtt tgcgcggcgc ggtttacgcg gggggagggg gagggaggga aagtagttcg 6600
ttcgtagata gcgcggatgt ttgtaaggta tttaaaataa gtagtcgtta gcgttaataa 6660
ataagtttat taatcggcga agttcgagtt tacgattttt tatgtttttt ttaaagttgt 6720
tgaggggcgg gaattttcgt tgcgggaaga agaaaaggta aattcggttt ggaagcgggg 6780
ggttttgagt tgagagttag agaagggtta tttttttttt tttggatttc ggaatcgttt 6840
agttatttat ttttggtttt tggagaaatt tgagggaggg tttttgattt tcgaatcggt 6900
tttttttgtt ttttttattg gattaatgat gttttttttt ttttttttat cgagttttgg 6960
gtaattaggg ttttggggtg agatagttaa gttgtttggt ttatttttta agtaagtatt 7020
tcgcgttttt agtttggggg ttataggaaa tgtttgtttg ttatatggta agaggtaaag 7080
aaaagcgtta agtttaagat gtatagtttg tttttttagg tttttttttt tgtaagtatt 7140
tacggtttag cgttaaaata ggtgtttgga aaagtggggg aaatgtaaat tggaagggtt 7200
atgtagattg aaggtttatt taatttttgt tatgattttt ggaggaattg tttgttttta 7260
gtaagttaaa aacgggggta cgattttttt ttttgtgatt tgggatattt tttttatggg 7320
agaaacggag gtaatttatt ttcgcgggta gttcgtgtgg tttcgattta attatttttt 7380
ttttattttt ttatatgtta ggtaatttta aaggtgaaaa cgtagttaat tggtttacgg 7440
taaagagtgg tcggaagaag cgttgttttt atacgaagta ttagatattg gagttggaga 7500
aggagttttt gtttaatatg tattttattc gagagcggcg tttagagatt agtcgtagcg 7560
tttattttac ggatagataa gtgaaaattt ggttttagaa tcgtaggatg aaattgaaga 7620
aaatgaatcg agaaaatcgg attcgggagt ttatagttaa ttttaatttt ttttgatgaa 7680
tttttaggcg acgcggtttt tttatttttc gagcgttggt tttttttttt tgtttttagg 7740
ttttgtttag gaattcgtat ttgtgttgga gttttgtttt ttttttttat attcgttatt 7800
ttttgggtcg ttatatttgt gtagggttgg tttgttttga ttttttgttt ttttgtgttt 7860
gtttggtgtt ggtttatttg ttgttttttg ggggaaaaag ttatattatg ttaaaatttt 7920
atagagatag atattgtttt aagtgttaag ttttgattgg gttgggtttg ttgttttggg 7980
gttttattgt tcgaaatggt ttttgttttc ggtcgagttg gttttttgtt tagtttgggg 8040
taaatttagt cggaaggtcg aggttttatt gttggcgttg aggtgtttgg tttgaggtta 8100
atggtgtaaa ggagtcgtta tcgggtatgt ttgtttggag tgttgtgttg tgtttaatta 8160
ggggatatag gtttttgggt tttttttttt tttttttttt ttttttttgg ttaagagaag 8220
ggtttatagg tatggatatg taggttggta aacgggtttg attttggttg atttaaaaag 8280
tgagaaagaa agtaaaaaag gttaattttt tttttttttg taagatattt tagttttaaa 8340
aagaaaaaaa aagaattatt aagagaaggg gatttttttt ttagtttttg taaggtttta 8400
tattgtttga ttaaaatgtt ttatttattt ttaaattttt atattttttt ggttgtagat 8460
aaataatgta gttttgttta tgtatttgga attagtggat ttttttgtta ttaaaattgt 8520
tattattggt aatatgtgat aagtatatta taattttttt tattttgtga agttgttttt 8580

ttaaatcgtt ttgaataaaa agtttttttt ttgtttgttt ttgttttttg aaatttatag 8640
aagtttagga ggattggggt aagcggaata aattagagaa gggagatatt gtttggattt 8700
tttttatatt gtgaagttat atgtataaaa gggttaaatt tgtagatgta gaaaaagaaa 8760
aaaattataa atataaattt gtataaatgt ttattattat gaagaattgt taattttgtt 8820
ttaagtaaat gtattttatc gttattataa atgttagttt tagttttatt tatttttaat 8880
tttaaattag aataaattaa tattgtattg ttgttgtgcg tggaaaaaga cgatgtttat 8940
gtttttatag aataaaagtt gtggaatgaa gttttttaat tttatttttt ttttgatttt 9000
ttatttttat tttttatttt atttcgttta ttatttttat aataggagga ttaattcgag 9060
tttttgtaat tattttaggt atttatttaa atattattta gacggtcgta atttgtttgg 9120
gttttatagt tttggtgtcg taaggtttgt cggtttttgt ttagttttat ggtttgttag 9180
tatatttgga ttgtggttgt tttggattag tgattttagt tgagagggga gttatataga 9240
tagaggaagt taaataggat ttttttcggg agttttaggg aggtttgtag aggtcggtta 9300
ttttgcgggt gtttgggtta tagatgattt ttaatttgaa tcggggagta ggtttggttt 9360
atagcgtatt tttagtcgtt ggaggtagcg gttaggtgga ttcgggttgg attgtatggg 9420
ggtttttttt ttttttagta cggttttttt tatttggcgg tattttttag ggttacgcgt 9480
tttttgtttg ttagagtggg gggggcgggg gttaagggga gggggcgtag gagcgcgcgt 9540
ttcggcgggc gggtagttag gagggagagg gggagagcga gaggcgggag gtcggggtta 9600
gatagggagt cgggatttgt atcgtttttg gcgttttcga gttttttacg gttatcgttt 9660
tttggcgtcg gttcgttggg ttgtaagatt tgggaaagcg gcgcgtatat tgtttttcgg 9720
agttgttttc gtttcgtttg tttttcgttt ttattttttt ttttttgttt tttttttttt 9780
tttttttttt tttttttttt ttaggaatat tttattgggg tttgtggttt tttttttttt 9840
gacgtatttg cggtcgtttg gggtattttg ttgtattggc gggcgtaggt tgtttagggg 9900
ttgggttggg tcgggttagg cgcgatggta gggttttttt tttggcggcg gcggtagcgg 9960
cggaggcggc ggcggcggcg ggcgaggtag tttttcgcgg gtagtattag aattggtcgg 10020
tgatttaggt agttttttgt tgttgggatt tatttttttt tcgatagtac gatattgttt 10080
ttattatttt agttgaaatc gtttttaggt atttttgcgc gcgggggtcg ggtcgggcgg 10140
ggtattacgg ttttggtcgt gttaggtttg cggtggtaat ttcggttttt tttgtttagg 10200
agtttcgtgt tttttttcgt agcgttttgt tagtcggtcg gttttttttt tttattatat 10260
atttttattt ggttatagta ggtagggtta ggttggttgt tttttcgcgg acgtcggggg 10320
cgtggtagga attttgggtt ttgggttatt taaggttagg ggcgttcgtt tttattttga 10380
gttttttatt ggaggtttgc gtttgtttag ggattttttg ttattttttt gtaaggttgg 10440
aatatatata tatatatata tatatatata tatatatata ttagtttcgg gtgtgagttt 10500
aggaaagaat ttttttttag tggagattta ggttggatta ggaaggatag ggtaggttgg 10560
gattttggtt ttgttattta tttttttttt tgtaagttcg gtcgttgtta ggagtcgttt 10620
tttttttttt tttttttttt tttgtttttt tttttttttt tatttttttt tttttttttt 10680
tttttttttt ttgttttttt ttgttttttt ttttttcgtt ggtttttttt ttatttttcg 10740
tttttgttag attttatttt atttttttat ttttttttta aagtattttt ggggaggggt 10800
ttagagttgg ttttaggaga gtttaggggg ttttattttt ttttgcggtt taagtagagg 10860
ttgggaagtt tgtcgaggaa gggttcgcgt ggggcggttg tcgagggcgg tgggtttggg 10920
tttggtgtgt tattttgtag ataaggtttc ggttatgatt acgatcggat ggcggtagtt 10980
ttgcgttttt tcgtttgtcg ttaacggttg attaggagat ttgattaggg gatattcgtt 11040
gtagttttgt gtgtttatat cgagggtagg gaggttttcg ttagttttat tggggattgg 11100
agttgaggtt taattagttt cgttggagtt tggttattcg ttgggaagtt tttagttggt 11160
ttttttcggt ttgtcggtta tagtttgggg tttgatcgta ttgagggaga ttggttaggg 11220
taaagtttga ttgtttgttt attagtggtt tttattaggt ataaaatatg ttttatatag 11280
tatttttgtg tagttgggtg ttggagaagg ggattttttt aagagttagt atgttttaaa 11340
tgggtaaaaa gaattttaaa ttttagagtt attgatataa atttgagtta tcgttttaaa 11400
ttgtagttta tagatgggag ttagtttgat tatttttttt atagaaaata gaaaataaat 11460
tatttttttt tatttttaat atatatagtt atagattcgt gtttataaag ttgttttttt 11520
tttttgtttg gattggaggt ttaggaattt tttcgttgta tagttatcga tttgttgttt 11580
gaaaggatta tatattttat tttttttaat ttttttttgt tattttttgg attagggtat 11640
tggatttatt ttagagatta tagttttaga aattttagtc gagaaggggt cgttttaaat 11700
tatatttatt tattattttt ttatgtatac gtaatttttt ttatttcgaa aataaaagta 11760
gataaaaaag ttagattatt ttggttttaa atttttgggt tgaaggaaga taatatttgg 11820
ttttttgatt ttttttcgtt ttttaggtat tagtaatatt tgtttaatat ttaacgtttg 11880
ttaattgttt agtaattttt ttattttggg gttaaggttg tttaaaggaa tagttttttt 11940
atttaaaaaa tattgaaata attagtgttt ttttaggtat aaaacgtgga aaaggtattt 12000
agatatgttt agtttatttg tagatattta aaattgggag ttagggtttt ttttagggtt 12060
aattttttaa atgaaaattt tagaggtttg gcggattgcg gggtatttag gatacggtgg 12120
attgtggtgg gggtgggtat gatttagagg aatttatggg gtaaggaaaa tattaggggg 12180
agggagatga ggcggatagg acggggttat ttcggagttt attgtgtcgg ttattttttt 12240
tttttaggcg cgggtgtagg aaggggtatt tagtcggtat tcgcgcggtt tggtagtttc 12300
gttggtattt gggagtttta gtcggaagtg tgttagggtg tttgaggggg ggattattgg 12360
aattgttggt gaggatgaag gtaaaagaga gagagagaga tggaagcgtt cgaggtcgtt 12420
agtttcgtcg ttagggaagt gggttaatga aaaatatatt gttgtaggta tagtatttat 12480
acgtgaattt gattattttt gttttaggag tcgttgtttt ttgttaggaa ttgggggtag 12540

ggggagtttt tttttaatta acggagtggc ggcgattttt taatttattt ttaacgggtg 12600
agaaataaat ttttttaacg tggttaggtt taggaatggg attggagtcg atgttttttt 12660
atttttttttc gtttaatttt ttagttttgg ttttgagttg tggttgtttt tttttgggtt 12720
ttgtattttt tcgtcgagtt ttcgggtttc gtaggtaatt aaggcgaggt tcggagtagt 12780
agttggaaag ggaggaagga gttttgaaag gtttacgcgg tttcgggata ggttatatcg 12840
gtgcgggttt tttaggtttc ggagttgcgg ggttttttag gcgaggttgt ttttttttaa 12900
atcgaatttg tttttttattt atgttatttg tagttttttt tttagttggg atttacggag 12960
cgtaattagg tttgtagcgt ttatggttag agtttttgag gttggagtat agggttgggt 13020
cgttagtcgt ttgcgtttgg gaattttgat tgttagttga tgagaaaggc gggttgggcg 13080
cgcgtgtgcg tggggtcgag ggtcggggat cgagcgcgtc gtataattaa ttaggttttt 13140
aaaattttcg ttttggtggc ggttggtcgt ttttttttgg ttagtttttt cgtggggttt 13200
tcgtagtaaa ggcgaattta agggttgttc gggcgttttt cgttttaggc gggtagttgt 13260
ggggatttat attcgcggta ttttttgagc ggtcggtttt tgtttggagt gttttggtag 13320
ggtcggcggc ggtttcgttt gggacggatt ttgcgttgaa tttgattttt cgagggcggt 13380
cgcgggtaaa ttcgtttttt tcggggatcg tagggattat ttatagggag ttcgttaatt 13440
aaatataata gtttaatttt tttaagtttt cgtaaatttt tatagttggg agttacggcg 13500
aggtaaacga atttgttggt cgttttcgat ttttcgttag tttgtgtggt ttttgaaata 13560
ataatttttt atgaaatatt ataaatatag atttaaatat agtagagcga taatgcgatt 13620
tggttgtttt tttatggttt taattattgt ttaattttat gtgaggggtt tcgttggtcg 13680
tattcgtacg cgggattcgc gtttttttga tggcgtgatt aattgtgata taaaatagtt 13740
cgtttaagaa gtgtgtgtat ggggggggag acgggagagt atagagataa ggttagattt 13800
gattttttaa tcgtcgttgg ttataattaa aataaatttt atcgtagagc ggtacgattt 13860
ttttatataa aaatatatgg tttttgttat aaaaattatg attgtaaaat atcggattat 13920
taatagcgtg cggagtgatt tacgcgttat tgttttgttg gacgggtacg tgacgcgtac 13980
ggttaatggg ggcgcgggcg tcggtaattt attaggtgat tgtatttttt tttcggtgtt 14040
attaagttgt tatatgaaat ttgtagtttt ataattttcg tgggtcgggt cgggcgggtt 14100
aggcgttggg tacggtgatg gttattattg gggttttggg taattattac gtggattcgt 14160
ttttgttggg cgtcgacgtc gcggatgagt tgagcgttgg tcgttatgcg tcggggattt 14220
tgggttagtt ttttcggtag gcggcgacgt tggtcgagta tttcgatttt agttcgtgta 14280
gtttttagtt taaggcgacg gtgtttggcg tttcgtggaa tttagtgtac gcggcgggcg 14340
ttaacgttgt attcgttgcg gtgtattatt attattatta ttatttttac gtgtattttt 14400
aggcgttcgt ggcggcggcg gcgtcggacg gtaggtatat gcgtttttgg ttggagttta 14460
cgttcggtgc gttttttttc gcgggtttgt tttttagtcg gttttatggt attaaatttg 14520
aatcgttgtc ggttagaagg ggtgattgtt ttacgtttga tatttatatt ttgtttttga 14580
ttgattatgt ttgtggtttt tttttagttg atagagaaaa ataatttagc gaaggcgttt 14640
tttttgaaaa taatgttgag aatgagagcg gcggagataa gttttttatc gattttagta 14700
agtgtttttt ttttttaaat tcgtcgtcgt ttttacgtcg gtttttcgga tttgttggtt 14760
cgttaggttt ttttcgagtt tgttttcgtt ttcgttggaa gtttttcgag ttggggttag 14820
gagttagaag ttggtgtttg ggacgtttta gatagggttt taagtttgga gagtagtgaa 14880
gagcggttcg tagggttacg ggagaggagg cggttgttgt agcgagaggg ggcggggcgg 14940
gtatttcggg acgagttaag attggtcgtt tttttttttg gttgtttagg tttaggatcg 15000
agatattttg ggtcgttttt cgaaagtagt gtagtttaga gagttttttg tataattaga 15060
ttgttcgtga gcggcggtag ttagggtagt cggagttggg acgttggggg agacggtcga 15120
ttttttttat tttttgtttt cggttagtgg cggcgtaaat tttgttaaga tgaggttgcg 15180
ggcgattcgg gttataaggg tttttatgat agattattta aataagttat agacgtgatt 15240
agcggtttta gggcgttttg acggtttgtt tagtttcgaa ggttttttag gaaggttaaa 15300
taaggagtgg ggggcgtaga gggataggtt gggaaagaaa gacgaagtta gtgaacggga 15360
tagaggaatt ttaattttgt tatagaatat aaggtagtat gttttttttt tgcgtggtaa 15420
ggagatttgt ttttaaattt tattttatat agcgttttga gagtgggagg aaggagaagg 15480
gggataagag gatagagtag gagaaaggaa ggtttcggag gggagggcga gttaaagttt 15540
tattgcgtgt aattttaagt gattgtttgt gcgtttgttt gttagggttt tattgtgttc 15600
gaggtttgat tgtttttttt aattagttta gtagagtttt gtatttcggt tagagatttt 15660
atgtaggagg tttatttgtt ttagcgggat gtgcgttttt tgtttttaaa tttagtgttt 15720
tttttttttcg taga 15734

<210> 346
<211> 15734
<212> DNA
<213> Artificial Sequence

<220>
<223> chemically treated genomic DNA (Homo sapiens)

<400> 346

tttgcgggga agagaaatat tgggtttagg agtagaagac gtatatttcg ttggggtaaa 60

tgagtttttt gtatggggtt tttggtcgaa gtgtagaatt ttgttgaatt ggttaggaaa 120
ggtagttagg tttcggatat aatggaattt tggtagatag acgtatagat agttatttaa 180
aattgtacgt agtaaaattt tggttcgttt tttttttcga gatttttttt ttttttattt 240
tgtttttttg tttttttttt tttttttttt atttttaaaa cgttgtatag aatgaaattt 300
ggaaataggt ttttttgtta cgtagaggga aagtatgttg ttttgtgttt tgtagtaaga 360
ttaggatttt tttgtttcgt ttattgattt cgtttttttt ttttaatttg tttttttacg 420
ttttttattt tttatttaat ttttttggaa ggttttcgga gttgggtaag tcgttagggc 480
gttttaaggt cgttgattac gtttgtggtt tatttgaata atttgttatg gggatttttg 540
tggttcgggt cgttcgtagt tttattttgg taggatttac gtcgttattg gtcgaaggta 600
agaagtggaa ggaatcggtc gtttttttta gcgttttagt ttcggttgtt ttggttgtcg 660
tcgtttacgg ataatttagt tgtataaaag gttttttggg ttgtattgtt ttcgaagaac 720
ggtttaaagt atttcggttt tgggtttggg tagttaagga gaggggcggt tagttttggt 780
tcgtttcgaa gtgttcgttt cgtttttttt cgttgtagta gtcgtttttt ttttcgtagt 840
tttgcgggtc gttttttatt gttttttaga tttggggttt tatttgaggc gttttaaata 900
ttaatttttg gtttttggtt ttaattcgag aggtttttag cgaggacgaa ggtaggttcg 960
agagaaattt ggcgggttag tagattcggg aggtcggcgt ggaggcggcg gcggatttga 1020
agggaggaga tatttattgg gatcgatggg gggtttgttt tcgtcgtttt tatttttagt 1080
attgttttta gagaaggcgt tttcgttggg ttgttttttt ttattaattg gaggagaatt 1140
ataagtatag ttagttaggg ataaagtgtg agtgttaagc gtgggatagt tatttttttt 1200
ggtcgatagc ggtttaggtt taatgttata aggtcggttg gagggtaagt tcgcgaagga 1260
gagcgtatcg ggcgtgggtt ttagttagga gcgtatgtat ttgtcgttcg gcgtcgtcgt 1320
cgttacgggc gtttgggggt gtacgtaggg gtggtggtga tggtggtggt atatcgtagc 1380
gggtatagcg ttggcgttcg tcgcgtgtat tgggttttac gaggcgttaa atatcgtcgt 1440
tttggattgg aagttgtacg ggttgaagtc ggggtgttcg gttagcgtcg tcgtttgtcg 1500
gggaggttgg tttagggttt tcggcgtata gcggttaacg tttagtttat tcgcggcgtc 1560
ggcgtttagt aggaacgagt ttacgtagta gttgtttagg gttttagtgg tggttattat 1620
cgtgtttagc gtttggttcg ttcggttcga tttacggaaa ttatgaaatt gtagatttta 1680
tgtaataatt tggtggtatc gggggggaag tatagttatt taataagttg tcggcgttcg 1740
cgtttttatt ggtcgtgcgc gttacgtgtt cgtttagtag aataataacg cgtaaattat 1800
ttcgtacgtt attaatggtt cgatgttttg tagttataat ttttatagta aaagttatat 1860
gtttttatgt aaagggatcg tgtcgtttta cgatggggtt tgttttaatt gtggttaacg 1920
acgattaaaa gattaaattt agttttgttt ttgtattttt tcgttttttt ttttatatat 1980
atatttttta agcggattat tttatattat aattaattac gttattaaga aggcgcgggt 2040
ttcgcgtgcg agtgcggtta gcggagtttt ttatataaaa ttagataata attgaagtta 2100
taaaaaagta gttaaatcgt attgtcgttt tattgtattt aaatttatat ttatgatatt 2160
ttataaggag ttattgtttt agaagttata taggttggcg ggaagtcgga aacgattaat 2220
agattcgttt gtttcgtcgt ggtttttagt tgtaaaaatt tacgaggatt tggaaaggtt 2280
agattgttgt gtttggttgg cgagtttttt gtaaataatt tttgcggttt tcgggagagg 2340
cgagtttatt cgcggtcgtt ttcgaaaagt taaatttaac gtaggattcg ttttaaacgg 2400
agtcgtcgtc ggttttatta gggtatttta ggtagggatc ggtcgtttag ggagtatcgc 2460
gggtgtaggt ttttatagtt attcgtttgg agcgaggggc gttcgggtaa tttttaaatt 2520
cgtttttgtt acgaggattt tacggaggag ttggttagga gggagcggtt agtcgttatt 2580
agggcgaagg ttttgagggt ttggttggtt gtgcggcgcg ttcggttttc ggttttcgat 2640
tttacgtata cgcgcgttta gttcgttttt tttattagtt ggtaattagg atttttaggc 2700
gtaggcggtt ggcgatttag ttttgtgttt tagttttaga ggttttaatt atgagcgttg 2760
taagtttggt tgcgtttcgt gaattttagt tggggaaaaa attataagtg gtatgaatgg 2820
aaggtaagtt cggtttggga aaaggtagtt tcgtttaaga gatttcgtag tttcggaatt 2880
tgggaggttc gtatcgatgt ggtttgtttc ggggtcgcgt gagtttttta gggttttttt 2940
tttttttttt agttgttatt tcgggtttcg ttttggttat ttacggggtt cggagattcg 3000
gcggagaggt ataaggttta aagagaggta gttatagttt aaggttaggg ttggaaatta 3060
gaacggggag gggtaaaagg gtatcgattt tagttttatt tttgggtttg gttacgttgg 3120
ggaagtttat tttttattcg ttgggggtaa attaaaaggt cgtcgttatt tcgttaattg 3180
gaaggaaatt ttttttgttt ttaattttta atagaaagta gcgattttta gaataggggt 3240
aattaaattt acgtgtggat attgtgtttg taatagtgtg ttttttatta gtttattttt 3300
ttggcggcga ggttggcggt ttcgggcgtt tttatttttt tttttttttt ttgtttttat 3360
ttttattagt agttttagta attttttttt taaatatttt gatatatttt cggttgggat 3420
ttttaaatat tagcgaggtt gttaagtcgc gcggatatcg attgggtgtt tttttttgta 3480
ttcgcgtttg gaagagggaa gtggtcgata taatgaattt cgaaatggtt tcgttttgtt 3540
cgttttattt tttttttttt aatatttttt ttgttttata aattttttta ggttatgttt 3600
atttttatta tagtttatcg tgttttaaat atttcgtagt tcgttaggtt tttgagattt 3660
ttatttaaaa aattaatttt ggaggaaatt ttggttttta attttaagtg tttgtaaatg 3720
ggttgggtat gtttggatgt tttttttacg ttttatgttt gagaagatat tgattatttt 3780
agtatttttt aagtaaaaaa gttgtttttt taaatagttt tagttttaaa ataagagagt 3840
tattgaataa ttagtaggcg ttgagtgtta agtagatgtt attggtgttt agaaagcgga 3900
gaaaaattag aaaattaaat attgtttttt tttagtttaa aggtttggag ttagaatagt 3960
ttgatttttt tgtttgtttt tattttcgaa gtgaaagaga ttgcgtatgt ataaagaaat 4020

aataaatgag tataatttaa agcggttttt tttcgattga gatttttgaa attgtgattt 4080
ttgaaataaa tttaatgttt tggtttagaa aatggtaggg aggagttgaa gggaatgggg 4140
tgtgtggttt ttttagatag taggtcggtg gttgtgtaac gaaagagttt ttgggttttt 4200
agtttaggta gggaaaggaa atagttttgt gggtacgaat ttgtaattgt gtgtgttggg 4260
agtagggaga gatagtttgt tttttgtttt ttgtagggaa aatggttaga ttggttttta 4320
tttgtagatt ataatttgga acgatgattt aagtttatat taatggtttt ggaatttggg 4380
attttttttg tttatttaaa atatattggt ttttggaagg gttttttttt ttagtattta 4440
gttgtataaa ggtgttgtat ggaatatatt ttgtatttaa tggaagttat tagtaagtaa 4500
gtagttaagt tttgttttag ttagtttttt ttaatgcggt taaattttaa gttgtgatcg 4560
gtaggtcggg aagagttagt taagagtttt ttagcgaatg gttaggtttt agcgaggttg 4620
gttgggtttt agtttagttt tttagtgagg ttggcgaagg tttttttgtt ttcgatatgg 4680
gtatataaag ttgtagcgaa tgttttttaa ttagattttt tagttagtcg ttagcgatag 4740
gcgaagaaac gtaaggttgt cgttattcgg tcgtgattat aatcgaggtt ttgtttgtag 4800
agtaatatat taggtttaaa tttatcgttt tcggtagtcg ttttacgcgg gttttttttc 4860
ggtagatttt ttaattttta tttgagtcgt agaggaaagt gagatttttt aggttttttt 4920
gaagttagtt ttgggttttt ttttaaggat gttttgggaa ggagataagg aggtgagata 4980
gaatttggta gagacggaag atgaaaaaaa gattaacgga agaaaagaaa gtaggaagga 5040
gtaaaggaaa gaagaaaaaa agaaagaaga gataaaagag gaagggaaga tagggaaaag 5100
aaggaagaaa agagaggcga tttttagtag cggtcgagtt tatagagaga agggtaagtg 5160
ataaggttag gattttagtt tgttttattt tttttagttt agtttgagtt tttattggaa 5220
ggaggttttt ttttgagttt atattcggga ttggtgtgtg tgtgtgtgtg tgtgtgtgtg 5280
tgtgtgtgtg tgttttagtt ttataagaga atggtaggag gtttttgggt aggcgtaggt 5340
ttttagtggg aggtttagga tggaagcggg cgtttttgat tttgaatggt ttaaagttta 5400
gaatttttat tacgttttcg gcgttcgcga aggagtagtt aatttaattt tatttgttgt 5460
gattaggtgg aggtgtgtgg tggaagggga aagtcggtcg gttggtaaag cgttgcggag 5520
aaagatacga ggtttttgag tagggaaagt cgaggttgtt atcgtaggtt tggtacgatt 5580
agggtcgtga tgtttcgttc ggttcgattt tcgcgcgtag aggtatttgg agacgatttt 5640
aattgaagta atgaaggtag tgtcgtgttg tcgagagaaa ggtggatttt aataatagga 5700
aattatttaa attatcgatt agttttggtg ttgttcgcga agggttgttt cgttcgtcgt 5760
cgtcgtcgtt ttcgtcgttg tcgtcgtcgt taaggagaga attttgttat cgcgtttggt 5820
tcggtttagt ttagttttta ggtaatttgc gttcgttagt gtaatagagt gttttaggcg 5880
gtcgtaaatg cgttaaggaa ggggaagtta taggttttag taaggtattt ttgggaggga 5940
gagggaggaa aagagaggga ggaaaggtag ggagagagga ataaaggcgg ggagtaggcg 6000
agacgagagt agtttcgaga agtagtgtgc gcgtcgtttt tttaaatttt gtagtttagc 6060
gagtcggcgt taagaggcgg tagtcgtgga aggttcgaaa gcgttaggga cggtatagat 6120
ttcggttttt tgtttggttt cggtttttcg tttttcgttt tttttttttt ttttttagtt 6180
gttcgttcgt cggggcgcgc gtttttgcgt tttttttttt tagttttcgt tttttttatt 6240
ttggtaagta ggaaacgcgt ggttttaagg agtgtcgtta ggtgggggag gtcgtattgg 6300
gggaggggga ggtttttatg tagtttagtt cgggtttatt taatcgttgt ttttagcggt 6360
tagagatgcg ttgtgggtta ggtttgtttt tcgatttaga ttgagggtta tttgtgattt 6420
agatattcgt aaaataatcg gtttttgtaa gtttttttgg ggttttcgaa agaaattttg 6480
tttggttttt tttgtttatg tagttttttt tttaattgaa attattggtt taagatagtt 6540
ataatttaga tatattagta agttataaaa ttgaataaaa gtcgataaat tttacggtat 6600
tagggttata gggtttagat aaattacgat cgtttaggta atatttaaat agatgtttaa 6660
agtaattgta ggggttcggg ttagtttttt tgttgtaaaa gtggtgaacg ggataaagtg 6720
gaaggtgaag ataagaagtt aaaaaagagg taaaattaaa aagttttatt ttatagtttt 6780
tattttataa gaatataaat atcgtttttt tttacgtata gtagtaatat aatattaatt 6840
tattttgatt taagattaga agtaaataga gttagaatta atatttataa taacgataga 6900
atgtatttgt ttaaaataag attggtaatt ttttataata atagatattt atatagattt 6960
gtatttatag tttttttttt tttttgtatt tataggtttg atttttttat gtatgtaatt 7020
ttatagtata aaggaaattt aaataatgtt tttttttttt agtttatttc gtttatttta 7080
gtttttttag gtttttgtga attttagaaa gtaaaaataa ataaaaaaaa aattttttgt 7140
ttaaggcgat ttaaaaaaat aattttataa gatagggaga attgtggtgt gtttgttata 7200
tgttattagt ggtaataatt ttaatgataa aaaaatttat taattttaaa tgtataaata 7260
aaattatatt atttatttat agttagaagg atatggaaat ttagaggtaa atgaaatatt 7320
ttagttaggt aatgtaagat tttatagaaa ttggaagaga agtttttttt ttttggtaat 7380
tttttttttt tttttttaaa gttgggatat tttatagagg aaggaaaaat taattttttt 7440
tatttttttt tttatttttt aaattagtta aagttaagtt cgtttgttaa tttgtatgtt 7500
tatgtttgta agtttttttt ttggttaagg aagaaaggaa gaaagaaaaa agaaatttag 7560
gggtttgtat tttttgatta aatatagtat agtattttag gtagatatgt tcggtggcgg 7620
tttttttgta ttattgattt taggttagat attttagcgt taataatggg atttcggttt 7680
ttcggttagg tttgttttag gttgggtagg aaattagttc ggtcgaagat aggggttatt 7740
tcgagtagtg ggattttaag atagtaaatt tagtttagtt aggatttgat atttaggata 7800
atatttattt ttatagaatt ttagtatgat atggtttttt tttttagaaa ataataaata 7860
aattagtatt aagtaaatat aaagaaataa aaagttagaa taaattagtt ttgtatagat 7920
gtaacggttt aggagatggc gagtgtggga gggaggaata gggttttagt ataggtgcga 7980

gtttttgggt agagtttgaa gatagaggga ggggattagc gttcgggaag tgaaaaaatc 8040
gcgtcgtttg gagatttatt aggaaaaatt aaagttggtt gtgagttttc ggattcggtt 8100
ttttcgattt atttttttta gttttatttt gcggttttga aattagattt ttatttgttt 8160
gttcgtgagg tggacgttgc ggttaatttt taggcgtcgt tttcgagtaa ggtatatatt 8220
gaatagaaat ttttttttta gttttagtgt ttggtgtttc gtgtaggggt agcgtttttt 8280
tcgattattt tttgtcgtga gttagttggt tgcgttttta tttttggaat tgtttggtat 8340
gtaagagaat aaagagggga tgattaagtc gaggttatac gggttgttcg cgggggtgaa 8400
ttgttttcgt tttttttata agagagatgt tttaagttat agagaagaga gtcgtgtttt 8460
cgtttttggt ttgttgagag taagtagttt ttttaaaagt tatgataaaa attgagtggg 8520
tttttaattt atatgatttt tttaatttat atttttttta tttttttaaa tatttgtttt 8580
agcgttaagt cgtagatgtt tgtagaagga aaggtttggg agggtaggtt gtatattttg 8640
aatttaacgt ttttttttgt tttttgttat atggtagata agtatttttt gtagttttta 8700
ggttaggagc gcggggtgtt tatttggaag atgggttagg tagtttggtt gttttatttt 8760
agggttttga ttgtttaaga ttcgataagg ggagaaagaa gggtattatt ggtttaatgg 8820
ggaaggtagg aaaaatcgat tcgggggtta agggtttttt tttaagtttt tttaggagtt 8880
agggatagat agttgggcga tttcgaggtt taggggaagg gaaatggttt ttttttggtt 8940
tttagtttag ggtttttcgt ttttaggtcg gatttgtttt tttttttttt cgtaacgaag 9000
attttcgttt tttagtaatt ttgaaaaaag tatgggggat cgtaaattcg aatttcgtcg 9060
gttaatgggt ttatttattg gcgttggcgg ttgtttattt tggatgtttt ataaatattc 9120
gcgttatttg cgggcgagtt attttttttt tttttttttt ttttcgcgtg ggtcgcgtcg 9180
cgtaggttgg gtagggatta gggttttggg ttttttcggt tataggaaag agcgtatagg 9240
agggggtttg ttcgttggtg ttttcgtttt tagttagggg gagttgaggt tagcgtcgag 9300
gacgttttgt tgtggggcgt taagtcggat atgaatttta ttgcgttttt acgtttaaat 9360
attaaaaagt aagtttataa ggttagtttg tttgtagttt gggttaaggt cggtcggttg 9420
ttgcgcgggt ttttagtttt ttgatttttt ttttttttgt gtttgtttgt ttgttcgttt 9480
gattgtagtt ttttgtagtt ttgtttattt agggaatttt ttttcggcga ggttttgttg 9540
ttttcggaag ggtcggggga gagtttttc gcggtcgagg acgacgcgtg cgtttttcg 9600
tcgttttgcg agttttcgtc gttgtcgtaa gttagcgtgg ggggcggcgg cgaatcgagg 9660
gttcgttttt ttcgggtcgt atcggtcgag tcggaggtta gcgcgggcgg gagatcgaaa 9720
tcgcgtttcg ggggttgcgc ggggaacggg ttagtttcga gttgttgcgc gtcgtcgtcg 9780
tcgttgttat agtttttggc ggtgtcgtag gtttgagaaa ggcggaagta gttaggtatt 9840
ggtatttcgt tggaggtgtt tagggcgtag tcgtcgggcg gcgggtttcg cgggaaggga 9900
gttagttcgg cggcggtggt cgagattttg gggtatttgt tcgtcgagtc gtagaggtag 9960
taggagtttt tttttttgat gttttgcgcg aaagagtacg aggtggtttg cggcgttggt 10020
tggggtggtt gcggcggggg cggcggttgt tgttggggcg gcggcggcgg ttcgttaggc 10080
ggttttattc ggtaagatcg ggcgcgtttt agttataggt ttatgggcga gggttcgtag 10140
tcgtgcgttt cgggatttag attttcgtta tcgttatcgt tgttcggcga cgttgtttta 10200
ttgcgtttgt cgtttagcgt ggggaagagt tcgtagtttt gtagttcgta gggtaggtcg 10260
gcggcgggcg gtaggtagat ttcgtcgtgg gcgtagtaat cgttatcgtc gtcgtttttc 10320
gcgttattat tatcgtcgtt tgtttcgttt ttgttcgagt tgatgagcga gtcgattaaa 10380
aaagagttcg cggcgggttt ttcgagtatg atattgttgt gggataattt ggcgaaggga 10440
gtagatagtt ttttttggtt gatatttttt gtgtaaaata tgttgaatac gattagtaat 10500
tttttcgtat cgcggcgggc gttcgtagtt aatttcgagt tagagttttc gcgcgattat 10560
ttttagtttg gtttcgtagg cgcggggtcg tttttcgagg gcgtttttag agttcgcgat 10620
tgatataaat atgtaatttg tattgatggg ttaggagacg tatttcgata ttttggttcg 10680
aaggtcggga gttgtggggg ttgttttaac gtggttggtg gggggtttgg ttattgggtt 10740
cgtttcgttt ttattcggac gtgagtttta tatcggggtt ttttagaagg gtttttgggt 10800
ttcgcgtagt taataagtgg ggtgtttatg gtgcgcgttt agtttgtttt gggtgtttat 10860
tatttttgtc gtagaagttg ttattagttt tcggtgtatt ttaattattg aagcggtcgt 10920
gtcggggatt tacgcgtttt ttatttagtt ttggatttgg aattggtttt ttgtttgaat 10980
tttgtttttt taaaagtggc gaatttggtt ttatggtcgt taggattttt agagtgttag 11040
gagtttagag tgaattagaa gttgatttgt ttttattttt agtatttata gatttttttt 11100
taaaatgtag tggttgtttt ttagttttta atttttaata tttttttttt ttagttcgtt 11160
gatttagtta ggattttttt ttttggtgtt gtgtttattc ggtttgtttt ttttataatt 11220
tttgttagtg gattttggtg ggttggcggg attgggggtg ggaagcggtt gttagttttg 11280
atggaaagac ggttttattt ttatttttaa ggttagagtt agatttaagt atttttttat 11340
tatttaggga agattttaag tttatagtta tttcggggaa gtaaaatgtt ggttttgggg 11400
tttttggttg gtttaaagtg agagatttgg aggggtgtgt tcggtgtata ttttcgttgt 11460
tttatgtttt tgtttttttg ttagttttgg tatttatatg tttttggagg ttataataag 11520
aattagtgtt attatatttt tatttttttg tttttttttt tttttttttt ttttttatat 11580
ttttagttag ggtagttttt gtttttgtag tgtgtgtatt ttaacgaggt ttcggtgagg 11640
aaattttttt tttgtaaggt cgggttgggt ttatggtttt gttataagag tatgtggagg 11700
ttaggttttt tgtttagtag gtttaggggt tttgttttag ttaattggaa atggtcggtt 11760
tggggtgtta gtttttaagt gtgagtttta tgttagtttt aggaaatgta atttttttcg 11820
gttagattta gtggagttcg ggaggtcgtt gttttgtttt tggttcgtgg gttataggtt 11880
tttaggatat tttaggtttg ttcgtttttt cggttttagt cggagttggt attattgaac 11940

gtcggtattt taggaaatag atcgtttagg tcgttgtttt tagttgggaa agatttttgt 12000
attcgggtta tacgcggtat tttgtttttt tttttttagt gtgaatttag tttaaggtgg 12060
aagaggagtt tgagaggatt ttgaaagcgt agagagagtt ggttaggtag agttttagtt 12120
ttggtttttg agatttaagt agttgcggtc gttgcgggta gtggttgttt cgagtttcgg 12180
gtttagaagg tcgttttatc gttagcgcgg cgttttagtt attttaattt cggcgtttta 12240
cggtgattcg gtcgtaagga ttgagtatta gggtgcggag gaggaggatg gagagaaaaa 12300
gagagagata gaaggagaga cggatagagg gagagaaaag agagagaaat aggaggggag 12360
agagaataag aaagttagtt ttttttagtt tgatagggtt ttgaagttgt agtagcggtt 12420
ttagagggat taagataggg tttgtgttaa gagattatta attttttttc gtttttcgta 12480
tgtttttgcg tttaaggtta aggtaaattt tttatttttt aggtttatga gacggacgaa 12540
tttgagtgta gaaaagtttt agagtatcgt agttagtggt tttattttta gtttttcgga 12600
tttgtttggt ttttattttg tttattagaa gggtggaatt ggttgggatt gttgttttta 12660
gtaatgttat ttggaaatgt tatagagttg ggggttgttt aggaaggaag ttttgttttt 12720
tagtattgtt tatgtttttt agaaaagttg tgttatttgg taagtgggtt ttaagatttt 12780
cgaggttttt ttaaggataa tgagtcgtgg taggttagtg ggttaggatt tgggtttaga 12840
gttatgtttt tttaggttaa atgtagaaag taatttttta ggtcgatatt tgggaaggtt 12900
tagtatatat ttgagatagt ggaggttata taaataaagt aaaagataat gattttaatt 12960
tatttttttt aattttttag ggagttagga tttttttgga aaatttaagt aagttttgat 13020
ttattttgtg aaaaatgtat atttgtatat ttttataatt tgatttatta tttcgaagga 13080
gtttatatat tttttagttt tttttttggg ttttttagag ttttttagtg ttttgggatt 13140
tgggggtttt gttaatttgg ttggtaagat aagttgggtg ttttgtagta gatagtttag 13200
gttagggttt cgtagggtag ttaaatgggt ttttaggttg gatagaattt agggatttgt 13260
acgtgttaat taaggttata aaagttagtt tgtatcgtat ttgttataaa aggttgtttt 13320
agtcgaagag tagaggagtt gggaggtttg ttatgggttt cgtaagtcgt ggcgttggta 13380
tttgggtttt tagtgatttt tttgtggtga gttagatgtg tttttttagt atgatttttt 13440
tttttttgta ataaattaaa cgggatgttg ggtttgagtt ttttttggtt tttaaaaaga 13500
gtgtgtaagt ttatgtatgg tagttttttt ttggtttagg attgtttttt aatttttatt 13560
agggtttgtt gagtgggaaa cgtagcgaga tattgtcggt gagttggtcg gttgtaggcg 13620
ggtcggttgt tgttggttta aatgagagga tattaacgta gtagaagttt aagttaaacg 13680
tttttttggt ttttttagtt ggttttttag tttttagtat ttttgatcga tttttaaggt 13740
ttttatgttt tttagatttt aatattggag tgttaggttt ttttaataaa tattttattt 13800
attgagcgtt tcggtttttt tgagattttt tattttaaga gatgaagaaa gcgatttagt 13860
tgtagtgtgg gtcggttttt aagtattttt tttttttcgt gtttatttgt tttttttttt 13920
ttttgttatt ttttttagtt attaaatttt tttttttttt ttagggtaat tttgatttaa 13980
atttttagtg tttgtttgtt ttaattaggt tttatgtagc ggttatatag ttatttttag 14040
agtttatgtt ttatttagga ggaaatattg tttattttta ttcgggtaat aatttgttag 14100
ttttatgggg ttaaagtaaa tattaggttt atggattaag gttttcggta aggattaggt 14160
tgtagtttat taggtatttt ttttgagtag gtttttgtaa gtagtgagaa ataaattatt 14220
atgaaaaata agtttttaat gaaaagatat taggttttga gatcgaaatt gttagtaaat 14280
aatttatatt tggagggtat ttttaatgtt tttaagattt tgaattattt agcggtggag 14340
ttgggttgta gttttataga ggaaaaataa aggttgtttt tatatatttg gttttagtaa 14400
aaaaagaaaa aaaaattaga gttttttata tttatgtatt tatgtatagt tagaaaattt 14460
ttttataagt aatgatgttt tttttttatt tatttttaag aatgttaata tttttaggga 14520
tatttgatgt gtttgttttg aatttattag gtgtaattta ttattaaata atatattttt 14580
aaagtttttt aaatttaatt attaagatgg tttttttttt gttagggaag tgttggggtt 14640
tttttatata ttttttgaat tgtaatattt ttagatttgt ttataataaa tagtttttgg 14700
ttttagaatt ggaggcggtt ttggtattgt agggagggtt ttcgttgtag agagtagttt 14760
attgggtggg cgtttatttt ttgttttgtt gttttgtttc gttatagttt gggagttagt 14820
ttttaataaa gattagtaat tatgataggt aaaatttacg taataaatag gtagtgttta 14880
ttttttttgg gatttatttt atttattacg tattgtatat ttgggaaaag aaaaagatgt 14940
gggaaattaa tggaaaaata gtagaaaaaa ttaaatatag gagaaagagg gaatttgaat 15000
cgatttttta aaatgtaatg gaattttttt atttaaaatg tgtttgatga taattttaat 15060
ggagtgattg tttcgttaaa atttattttc ggttttagtt ttttaaaaaa gtattagtat 15120
ttattaatat atttttgtat atttgtaaaa tattttttat aaaagattta atttttatat 15180
gtaagaaatt gttgcgaaat tgtagttggg tttataatag cgtattttaa gtatgtaata 15240
tagagatttg tttggtattt ttaaaaatta tatgcgattt tttcgaattg gtttttttcg 15300
gaataaaggt tttttttaag ttgtcgggta gatatagaga tgtatttagt aggaaattag 15360
tgatttttag gatttttgtt tttgggaata agttcgtttt agcggtcgta gatttttatt 15420
attttattgt ttagaatgga aattttgttt tttttaattc gcgattattt tttttttttt 15480
ttttgttttt ttataatttt ttatcgtagt atagttggaa tattttcgtt tagatgtttt 15540
ttatttttag tttattttat tttgtgtatt tttataaaat cgaaagattc gttttgagtg 15600
aaatgttggc gtaaggagtt aaaattttta atttttttat ttaggttttt ttttgttttt 15660
taattttagg gagtaatggg gtgggggttt tttatcgcgt tattttataa atttagttac 15720
gttttcgaga gttt 15734

<210> 347

<211> 10697
<212> DNA
<213> Artificial Sequence

<220>
<223> chemically treated genomic DNA (Homo sapiens)

<400> 347

ttttttaaga ttagatgtag gatatttagt ttcgggtttt tggttgtttc ggagttatag 60
ggtttggggc gttttggaag tggggatata tttattattt tagttttaag ttaggagagg 120
gacgacgttg ttatttgtgg cgcggaagag gcggttttgt ggttttaggt agatgtttta 180
ggtttaggtt cggtttgggc gtttggaggg atcgttgggt ttcgtagtta gagtttttcg 240
ttagtattta cggtattcgg ggattatttt taagggaaaa attttttaaa tttatttata 300
ttaaaggttt tttttttttg tagttttgga gggttataaa ttttttttta gatagttcgt 360
tgtgatttaa attatgcggt ttgttgttat ttatcgttcg ttcggagata cgcggtttcg 420
gcgtcgtaag tttcgcgata ttgtttagag gtgttcgacg tttggagttt cggtttcgta 480
gggtttacgg gattcggggg atttcgggtt tatatcgacg tttttagtta gattttggtt 540
tttcggtgtg agttagagaa tagaagtttt ttttttattt attattttta attttttatt 600
gttttcgttt tcgtttattt tagttttaaa tttggcggtt ggtaagggaa gcggtttttt 660
ttagggtata tataaaaagg tttaattttg tttttagcgg gtaggggttt tgaattaaag 720
ggtggaggga ttagagtcga tatttttatc gttattttta tttttatttt attaaggcgc 780
gggagaggat gaggtcgttt gagatattcg aagttaaggt tgtttaaggt ttatttggtt 840
ttttttttag ttaagggagg ggattcgtag ggtaggcgtc ggtttcgtta gttttattcg 900
gttgttaaaa gataaatcgg gtttgtgttt ttttggttgg ttagtgggtt ttgttagttt 960
tttggttggt gggttcgtgg tgaaggaatt gaatcgaatt ttttggaaat tgttgatgtt 1020
tgtaagtgag tgtcgatttt tttttttatt ttcgattttt ttaagcgaag tttatttaaa 1080
tatgatttgg gaaaaatttg gtagtgggat tggggttaaa tttttaagga attatatcgt 1140
cgtaattttc gtttttgttg gattttttat atcggtttaa ttcgattttt tttttaagag 1200
ggggtggtta gggttaggtt atgcgttttt gttgtagcgt ttgaatattg gtttttgttt 1260
tacgggttat tgattagttt gggtttttaa gtcgtaggag gaagttttgg tagttttttt 1320
aaaggtgttg tggttttcgt ttttgggtgg gggtggggtt ttaggaaatt gtttgcggtg 1380
ttaggtaaat taaggtgttg ttagagtttg gtcgagggtt agaggagttg acgaggttaa 1440
ggggtttggt ggtgttgggg ataaatattg tggttgtagt ttttattttt tttcggcgtt 1500
tttggagtag ttttttatag tttttttgcg gcgaggtgat tttttggtgg ttataaggtt 1560
cgatttaagg agttgtttta taggaggttt tgagggagtt agttttagaa ggtttttggt 1620
ttttttgaga ttttgttttt gggtaattta aaggatatgt agagaagtta agttggtata 1680
tggtattgaa atattttagg gtttttaggt tgttttttgg atttgcgggt aggaaatcgg 1740
gagagtgggt ttagtattat tgtttatttt tttaaagatt aaagaggatg tcgttttttt 1800
gtataatgaa aatgaagttt ttgtattttg atttggaggg gtcgatttta gggtaaagtg 1860
ttttatttaa atagtttttt ttaatttatg ggagtgatta tttagtattt ttgggaatat 1920
ttatttatgt tttttaaatt tagagaaata gagtttagga cgagaattga gttgaaatat 1980
ttttatttta ttttatttta gttaaagttg ggggtggtgg gagaaggtgt ttattatttt 2040
aaagagttga gggagagggt tttatagttt agaggaaatt ggaagtttgg ataatgtttt 2100
agtatttttt tatatatatt atattatatt atatatatat atatatatat atatatatac 2160
gtaaatattt tttttgaggt tatttattaa gaattttatt ttcgaattga atataattta 2220
ggaaatattt ttttgggagt tggaatgtgt tagtttgttt ataggattgt agatttatag 2280
tatagaaaat tataattata aattttgttt gtggggtatt tgtataattt ttttgttatg 2340
tatttaagaa ttggtatata tttttattta tttttttgaa tatatatttt tttattgagg 2400
tagggagaga tattttagat tggcggatgg ttgagttcgt tgtgtattta tatatttttt 2460
taaggagata gatagagggc ggtggatcga attatagata gattcgtagg gatttggttt 2520
tagaaaggag tcggtttttg gtttgttttg ttcgtttgtt ttttttttta aataaacgga 2580
ttagacgaat aaacgtttat aaatattttt gggtttagat aagagcgatg aggcgttatt 2640
tttttttatg ttcgatttta ggcgtttagg ttcgtttgta gtggttttat ttttagataa 2700
gtaatttgta gaggtgaggt taatttgggg gttgtgattt taaagataag gtaattattt 2760
ttttgttttt gatataggga aatgtttttt tttttattag attttatttt gtttagattt 2820
tattgagaaa attatattat ttagtgttag gatggagata ttgtgggtag ggaggaggta 2880
gggaaggttg aggtttaggg tttagtttta gtagtaggta gggaggtgta tgtaggtatt 2940
tttttttatt tttttttttt tgtttaaatg gtttttagtt ttttttagaa gtaagtttag 3000
ttatatttag atatggaata tttatttttt tttattatta gggggtttaa atttgttttt 3060
ttaaatgttt attaaatatt tttatagagt taggttagga agtaattgga ggttatttag 3120
ggatggttat tttgttatta aggtatagtt aagaagttgt tttgagttta gagagttttg 3180
gagattacgt tgtaattaaa taagttttta tttttggtga tttaagttta gttttttgtt 3240
atttttttta gtgttttgta ttatttttag tgggtagaat ttggttagaa aaaggagttt 3300
aaaggaagga taaggtagag gagggttagt ttggtttgat atttgatttt gttttatgtt 3360
ttggtttttt tttgttttta ggtgtgaagg ttgttatttt ttattatttg gtttgtgtat 3420

gtatattaga atgttgggaa tgtattgtgg ttttgttttt tagtttgggt ttaatgttta 3480
ttttttttgg atgaaagagt tagagatata tattttttga tatatttaaa tttgtaaggc 3540
ggtaaagttg atgatataaa tacggggtgt gggagggaat tgtagcgatg ttgagatata 3600
gatagaaggg agaggggatg ttggttgatg gttggaagta gtttgttgag tttttaagag 3660
aacgtaaacg attttttttt tttattaaat taaagtaaaa attaagtttt gggaaagaat 3720
taattttaaa aagaattggt agtttttata gagatttaga agaggagtga ggatgtattt 3780
ttttttagtg gtttttgatt gttttaatta ttaggagggt ttaaagttta taattggaag 3840
aagaatgatt ttttttttgt gtttagattt attgggatat ttttgatttt tgaaaatttt 3900
atagtttagg ttatttttaa gtttttgggg attgtagagg gtattggagt gagggttgta 3960
ggtaggatag ggattagata aataaagtta tttaggttga gggatatttt ttattttttt 4020
attttatatt attaagtgag gagagggtta gttttttttt ttagtgtttt tttttttgtt 4080
aagggtgagt agttgtatat tagaattagg tagaatgttt tgggtagtta ttttcgtcgt 4140
cgttttttaa gaagttttta gtttagttat agagttagaa ggggaagttt gggtttagtt 4200
ttttttgtta gttttttttt ttttgggttt tttaggtagt gggatttggt ttgttttttt 4260
agattagaag ggttgtggag aagtaggaat aataagtcga ggtttggagg agatagagta 4320
gatttgataa ataggaaaaa cgatgtttaa aatttgggga ggagggtgtt ttttttttgt 4380
ttattttagc ggagaatttt ttttattttt tatcgatata aaatttagaa aataaatttt 4440
ttttagtttt tttttaaggc gattcggaga tatttattta attttttttg gagagaaggg 4500
gtcgaagtaa cgtaggggtt aatttttttg aaaatataat taattttttt taattttgcg 4560
tggggtgggg gagagatata tttatttaat tattcgggag tggggtggtt ttaatttaaa 4620
cgtttttttt ttttttaaag gtattgagtt tgttttatag attttacgta ggtggaatag 4680
gacgcgttgt gttgttgggg agagagagga gaaaggcgaa ggagaaggag gaaaaaaaaa 4740
aggttgatag gtttgtgcgc gggtttttcg tgcgggttgc gttttttttg ggtgttattt 4800
gataattttt gtttttgtta ttggttagtg ttggattaga tggttgtatt tttttttggt 4860
ttttattggt atttcgttat ttttttttag ttaaaattta atttcggata tattattaat 4920
aggcgcggcg ttcggattat aaaatataat aaattataaa ttcggcggag tagtagcggt 4980
cgcgcgcgtt ttttttttta atgagttttt atttcgtgaa ttttattttt ttcgttattt 5040
tggttagcgg gtaggagttt tttttgggtt agttatcgtt ttattcgtcg ggttatgcgg 5100
attcgttgag atattatttc gcgttttacg ggttagggtc gggttaggat aagggttttg 5160
ttattttttt ttattattcg tcggcgggcg gtggttacgg tcgagcggcg ttttgcgatt 5220
acgggtcggc gtcggttttt tatcgcgaga aagagtcggt ttgcgtattt ttcggcgtcg 5280
acgagtagtt ttcgttttat ttcgagtcgc ggaagtcgga ttgcgcgtag gataagagcg 5340
tgttcggcga gatagaagag tagaagtgtt ttatttcggt ttattcgtgg atgtagcgga 5400
tgaattcgtg taatagtgag tgagattttt cgtcgtcgtc gtttcggttt ttttgggcgt 5460
ttatttcggg atatagaatt agtgagcgtt ttttgtttta attttttata gggtgttggg 5520
tgggtatttt agttaggaga tagaagaaga ttgggcgtcg gtcgggggtt tttcgttgtg 5580
ttttttatta ggtaggagtt ataaagtttg taaagtttta gtcgtattgg gagttatggg 5640
gagtaagtgt gtttttttgg ggcgtttggt tagagtcggg gttttaggat agggaaggga 5700
gtaggagttt gtagttattg gtttggtttt attttgagtt tttaattttt tttttttagt 5760
tttggtaggg ttttttaatt aaagacggtt ttattaaaaa cggagtgcgt tatgtaaggg 5820
ggtagggggt gacgttgaag aggaagagtt gagtttgttt gggattgtgt ttttttgtgg 5880
gtggggagtt ggggtaagat gaggtattag gaaaggggtg gtagttagag gagggaggag 5940
gaataagggg aaggaagaga gagggagtag gcggagcgag agaggagaaa ataggcgcgg 6000
ggttttaggt tggattattt gttggtttta agttttaatt gatgtttatt agtcggattc 6060
gaaagtgagg agtcgttaat gtggattatg gatcgattta cgttattacg gattaacggt 6120
ttggatttat tattgggttg gggggatgac ggggggtggg aataagatgg atggaaaggg 6180
agggaaagat aagatgtaat taggaagaga tatatttgat tagttttttt ttttaggttt 6240
aggtgggagt ttaaattgtt tttttttttt tttatttaga gtttagatag acggtatagg 6300
tttaggagat taggagggaa tttggagggg gcgttggagg agtgcgaaac gggggaaggg 6360
agtcggtaga atagagggta tttcggtatt ggttggagtt tgtgttcgag ggtcgattag 6420
gggagggggt tttgggttcg gtgatcgtag gttttagtat ttttattttg cgtaataggt 6480
tttttttttg ggtttagcgg tcggcgaggt cgttagatat atatacgtta ttagacgttg 6540
gagttggaga aggagtttta ttataatcgt tatttgacgc ggcggcggcg tatcgagatc 6600
gcgtacgttt tgtgtttgac ggagaggtag attaagatat ggttttagaa tcgacgtatg 6660
aagtggaaaa aggagagtaa attgtttagc gcgttttagt ttagtgtcga ggaggaggaa 6720
gaaaaatagg tcgagtgaag gtgttggaaa gggagggagg acgcgagggg aaaggtttgt 6780
ggggagttac gggcgttaga gagattcggg aaggaaggtt ttcgggtggg ggagttagga 6840
gatttgtttt tcggcgtaga taggcggggt ttagcgtttt tttggacgtt ttcgttcgta 6900
tagttttcgg cgggtgtttt gaggttttat tattcgagtt tatttagtat ttcgcgcgtt 6960
tttttttttc gaggaattcg ttttagtttg attaggtttt ttggtgagaa ttgaggagcg 7020
gatttatttg atgttttttg gaagtagagt aaaatgtttt tgtttttgtc gcgttttatt 7080
ttgtttatgt ttttcgtgta cggtttaatg gtagattcgt tgttttttta gcgggggttt 7140
tgaagatttt ttgattttag atttgtcgtt ttttttattt tttttttaaa gttattggaa 7200
ggagtatata ttatttagaa gtaagaagag gagttttaga agaaaataaa gttttatttt 7260
attaattttt tatgtgttgt gtttgtagtt ttgttttagt tttggacgtg aaatatttcg 7320
atgatgatga tgatgatgat gatgataata ataataataa taataataat aataataata 7380

ataaagatgt gaaaattcga cgttcggtta tttttaattt ttttttgtta tttttttttt 7440
tttttttaat ttttagtttt tttatttttt ttgagttaga attttttagt aaaggcgaga 7500
gttttggttc gtcgaagcga gttttgttcg attgggaagt tatagttgag ataaaggttg 7560
gagcgattag ttttgtttag gttttagcgt tgcgtttttt tggtgttgag gtttatagtc 7620
gttttttttc gtgtagattt gggggatgga gaggtgttgt gtttttttta ttttttagtt 7680
ttttttcgtg tatatatagt attaagggag ttattgaggt ttttaaagtt ttttggcgga 7740
gacggagtta tagtttgggg gtttttgtag cgtttttttg tttttatttt cgtagcgatt 7800
tttggttttt ggttcgttta tatggttagg ttttttcgtt agttttggtt tttcgttttt 7860
ggtttgtggt tttggagatt gaaaaatggg gttattgggt attttttatt ttgtagttga 7920
aggttttagg tgtttttttt tattttaagt agtagattag ttttaagtag gagattagtt 7980
ttaagtaggg gaatgtaaag ttattagtta ggtataggtc gaaggttata attttttttt 8040
gttttttgga gattttaggg taggaggtta ggtgatgggg tttttagtgt tattttttta 8100
atttagaatg gtttattttt tttttgtttg tatcggtatt tagaaggtaa gttgcggtta 8160
ttgtttttt ttatttttc gtttttcgt ttttttttt attttaaag aaaatagttt 8220
ttttttaata ggagattagt tgcggcggtt tttggtcgtt tagcgtgttt agtaaagtat 8280
gcgggttttt ggaatttttt tttcgggaag gcggttgtta ttaggtaaat ttttaaattg 8340
tttagttgcg agtgaggggt ttgtagtggg gttttcgaat gtaatagtcg aggaggaggg 8400
gaagggattt cgttttataa atttttagat atatgttatt atttaaatta agtagtagac 8460
gcggtggtcg ttgtagtttt ttcggtaatt aggtcgttta gttttagttg tgttcggttt 8520
ttggggtttt tgttttgttt tgttattttt tacgtttggg ttaaggatta ttaaggaggt 8580
tcggaagggg atttcgaatt tttaagtggt ttaggagaag tgaacgtttt tttttttttt 8640
ttaagttttt cgtttagttt tgtcggttcg gtttgggcgt tgtggatcgg taatagtagt 8700
tttattagga tgtaggtcgg attaagtttt tggggaaaat tcgttgtaat aattgttttt 8760
tatatataat ttttaatttt ttttttattt tcgtttttaa ttcggagatt tgtaaagttt 8820
aagaaaggga aaagtaattt tatttttgat ttttttattt aaagttaaaa taataacgat 8880
aatgatgatg tagggagaaa gaattaaata gagaggttaa ttttatttat aagaaagtgg 8940
gtaaaaagtt taatttttgt tttaaaaggt ttaattttgt ggttgagttt tggtcgggtt 9000
tttttttttt tcggcgattg tagggagttt gtagggagtt agtagggagg tagtcgttcg 9060
tcgttatggc ggttttacgt tcgcgttttc gtcggtcgtc gggaggtggc gcgttttgtt 9120
tagaggttaa agttaatttt tttttgttt ttttttttt ttttttttt tttttttc 9180
ggaggttcgt attgaatcgg ttttaacgat tttcggatcg ttattatttg taattataga 9240
gtatgaatta ttttttgagg ttattagcga gaatttacga ttggttaata aaagtacgtg 9300
attttttaac gtttttttat ttttttttta attttttttt ttatatttgg tcgtatatat 9360
agtaaaacga agtatagtgt atcgttataa tttattaata tattataaat cgtgaagtat 9420
agggttataa cgattacgat ttataaatta agttttttaa aattatttaa atgagttcgt 9480
attttgtaaa ttttttttcg gggcgttatt taaatggttc ggattattag ttgttaaatt 9540
atggtagtgg tagttttttg agcggttttt atagggattt cgttgttatg tatatcggtt 9600
tttacggtta taattataat gggatggatt ttagcgttaa tcgtttttcg gtttttttta 9660
gttattttgg ggcggtgggc gagagttcgc gcgttttttt cgcgttcgtt taggagtttc 9720
gttttaggta agcggtttcg agttgttttt tgttttcgtt cgagtttttg ttttgtatta 9780
acggcgatag ttacggcgtt aagttttttg tttcgttttt tttcgattag gcgattttag 9840
ttagttttag cgttaatttt atcgaaatag acgaggttag cgcgttttcg gagtttgagg 9900
aagcggtaag ttagttaagt agttttagtt tagttcgggc gtagttagag tttatggtta 9960
tttttatagt cgcgttcgag gggtagattt cgtagatatt tttttggatg aggaagtttt 10020
atattagtta tggtaatttt tttttttttt tttgtttttt cggttttttt tttttgcgtt 10080
ttggggtggg atggggggga taaggagagt ttggttttat tttaaatata gatttatttt 10140
tttaaggggg agaagtattt aagaggggtt ttatagtttc gtagatagag ttaaaaaggg 10200
tttttttgtt aagagggggg tatttttaat aatttgtttt tagtttcggt ttaggttttt 10260
attagcgtag ttgaattagt tttaggataa aggatggggt ttatataatg tggggaaggg 10320
ttttgtttat ttttgaaatt ttgagattgt cgttagtaaa gaaggggtga atagtaaaag 10380
gagtttttcg gggtaaaagt gtaatcgttt tttttttttt cggggcgagc ggtcgtttga 10440
gtttagttaa gggtgaggta ttaggagcga gtaagagata aagtcgggcg tattggttgt 10500
ttgtagaagt gggggatgta gtagaaaggg gtgtagaaat cgagtttgaa tgtgggtatt 10560
tggagtagag aggattggag ttgtggtgag ttgggtgtcg ggacgtttgg gttttgtttt 10620
ttcgtttttt tttatttagt gttttttttt ttaatcggaa taatgttgtt ttgcggtttt 10680
tttttatttg ttttaga 10697

<210> 348
<211> 10697
<212> DNA
<213> Artificial Sequence

<220>
<223> chemically treated genomic DNA (Homo sapiens)

<400> 348
   tttggagtag atagaggaaa gtcgtaaggt aatattattt cggttaaggg aggggtattt 60
   gggtgggagg gggcggggga gtaggattta ggcgtttcgg tatttagttt attatagttt 120
   taattttttt tattttagat gtttatattt aagttcgatt tttgtatttt tttttattgt 180
   attttttatt tttgtaggta gttagtgcgt tcggttttgt tttttgttcg tttttagtgt 240
   tttatttttg gttgggttta ggcggtcgtt cgtttcggga gggaggagag cggttgtatt 300
   tttgtttcgg aaagtttttt ttattgttta tttttttttt gttagcgata gttttaaaat 360
   tttaggggta agtaggattt ttttttatat tatatgaatt ttatttttta ttttaaagtt 420
   ggtttagttg cgttaataaa aatttaagtc gaagttggaa ataaattatt aaaaatattt 480
   tttttttaat agagagattt tttttggttt tgtttacgaa gttatgaggt tttttttaga 540
   tatttttttt ttttagaaaa atgaatttat atttagggta aagttaagtt ttttttgttt 600
   tttttatttt attttaaaac gtagagaagg aaagtcgaga agataaaaaa gagagagaga 660
   attattatgg ttgatgtgaa gtttttttat ttaggggaat atttgcggag tttgtttttc 720
   gggcgcggtt gtggaggtgg ttatgggttt tggttgcgtt cgagttaggt tggggttgtt 780
   tagttggttt gtcgtttttt taggtttcga ggacgcgttg gtttcgttta tttcggtgaa 840
   attggcgttg gagttggttg aggtcgtttg gtcggagggg gacgaagtag agggtttggc 900
   gtcgtggttg tcgtcgttgg tgtagggtag ggattcgggc gaggataggg agtagttcga 960
   agtcgtttgt ttgaagcggg gtttttgggc gggcgcgggg aaggcgcgcg agttttcgtt 1020
   tatcgtttta aagtggttgg aggaggtcga ggagcggttg acgttgaggt ttattttatt 1080
   gtaattgtag tcgtaagagt cggtgtgtat ggtagcggga tttttgtaag agtcgtttag 1140
   agagttgtta ttgttataat ttagtaattg atagttcggg ttatttggat aacgtttcga 1200
   gaaggagttt ataaagtacg agtttatttg ggtgattttg gagggtttga tttgtggatc 1260
   gtggtcgtta taattttgtg ttttacgatt tatgatgtat taatgaatta tagcgatgta 1320
   ttgtatttcg ttttgttatg tatgcggtta aatatggggg ggggggttgg aaggggggtg 1380
   ggggggcgtt agggaattac gtgtttttgt tgattagtcg taaattttcg ttgatgattt 1440
   taagaggtaa tttatgtttt atggttataa ataatgacga ttcgagaatc gttagggtcg 1500
   atttaatgcg agttttcgag agagggggaa aaaaggagaa gggggagaaa tagagagaag 1560
   gttggttttg gtttttgagt agagcgcgtt atttttcggc gatcgacggg agcgcgggcg 1620
   tgagatcgtt atggcggcgg gcggttgttt ttttgttggt tttttgtagg ttttttgtag 1680
   tcgtcgggag agaaagaaat tcggttaagg tttagttata gggttgagtt ttttagagta 1740
   ggggttgggt tttttattta tttttttgtg ggtggggtta gtttttttat ttggtttttt 1800
   ttttttatat tattattgtc gttattattt taattttgga tgagaagatt aaagatagag 1860
   ttgttttttt tttttttggg ttttgtaagt tttcgaattg gaggcggaaa tgggggaagg 1920
   gttggaagtt gtgtgtgaaa aataattatt gtaacgggtt ttttttagga gtttagttcg 1980
   gtttgtattt tggtggaatt gttgttatcg gtttatagcg tttaggtcga gtcggtaggg 2040
   ttgggcggga ggtttgaaaa agaaggagaa gcgtttattt tttttgggtt atttggaagt 2100
   tcgaaatttt ttttcggatt tttttggtgg tttttagttt aggcgtgggg agtgatagag 2160
   taggataggg attttagaaa tcgagtatag ttggggttaa gcggtttagt tgtcgaaaag 2220
   attgtagcgg ttatcgcgtt tgttgtttag tttgagtagt aatatgtgtt tgagagtttg 2280
   taaagcggga tttttttttt tttttttcgg ttattgtatt cggaggtttt attataggtt 2340
   ttttattcgt agttaggtag tttgagaatt tgtttggtgg tagtcgtttt ttcggaaaag 2400
   gggttttaag gattcgtatg ttttgttgag tacgttgggc ggttagagat cgtcgtagtt 2460
   ggttttttat taggagggga ttattttttt taaaaataag aaaggaagcg agaggcggga 2520
   ggaataaagg aaaatagtga tcgtagtttg ttttttgggt gtcggtgtaa gtagagagag 2580
   aatgagttat tttgggttga gaggatgata ttaaaagttt tattatttgg ttttttgttt 2640
   tggggttttt aaggagtaag ggaaagttgt ggttttcggt ttgtgtttgg ttggtggttt 2700
   tatatttttt tatttgaggt tgatttttta tttgaggttg atttgttatt tgaggtggga 2760
   aggggtattt ggagttttta gttgtagggt gagagatatt tagtggtttt attttttagt 2820
   ttttagagtt ataggttagg agcggagggt taaggttggc gaaaaaattt ggttatgtgg 2880
   acgggttaaa gattaggggt cgttgcgaag gtgaggatag aaaagcgttg tagaggtttt 2940
   tagattatgg tttcgttttc gttagagagt tttaggggtt ttagtggttt ttttagtgtt 3000
   gtgtatatac gaggaaaggt tagagaatga gagggatata gtattttttt attttttaag 3060
   tttgtacggg gagaagcggt tgtgagtttt agtattaggg aaacgtagcg ttggggtttg 3120
   ggtaggattg atcgttttag tttttatttt agttgtaatt ttttagtcga atagggttcg 3180
   tttcggcggg ttagggtttt cgtttttgtt gaagaatttt ggtttaggaa agatggagag 3240
   gttgggggtt gaggagagag gaaaaaatgg taggggagga ttggaggtga tcgagcgtcg 3300
   agtttttata tttttattat tgttgttgtt gttgttatta ttattattat tattattatt 3360
   attattatta ttattatcga agtattttac gtttagagtt aagataagat tataaatata 3420
   atatatagaa aattaataaa atagaatttt gttttttttt gaggtttttt tttttatttt 3480
   taggtagtat gtgttttttt tagtggtttt ggggaggggg tgggagagac gataggtttg 3540
   ggattaggga gtttttaagg ttttcgttga ggggatagcg aatttattat tgaatcgtgt 3600
   acgggggata tggataaaat gagacgcgat agggataaga gtattttgtt ttgtttttag 3660
   gaaatattaa gtgagttcgt tttttagttt ttattaggaa gtttgattag gttgaggcga 3720
   gtttttcggg aaggaagggc gcgcgggatg ttgggtgggt tcgagtagtg aggttttaga 3780
   gtattcgtcg ggagttgtgc gggcgggggc gtttaggaga gcgttgggtt tcgtttgttt 3840
   gcgtcggaga gtaggttttt tggttttttt attcgagagt tttttttttc gggttttttt 3900
   gacgttcgtg gttttttata ggtttttttt ttcgcgtttt tttttttttt ttagtatttt 3960
   tattcggttt gttttttttt ttttttttcg gtattgagtt gagacgcgtt gagtagtttg 4020
   tttttttttt tttattttat gcgtcggttt tggaattata ttttgatttg ttttttcgtt 4080
   aggtataggg cgtgcgcgat ttcgatgcgt cgtcgtcgcg ttaggtagcg attgtagtga 4140
   aatttttttt ttagttttag cgtttggtaa cgtgtgtatg tttggcggtt tcgtcggtcg 4200
   ttgggtttaa aggaggaatt tgttacgtag agtggagatg ttgaggtttg cggttatcgg 4260
   gtttaggatt ttttttttta gtcgattttc gaatatagat tttagttagt atcgggatgt 4320
   tttttatttt gtcggttttt ttttttcgtt tcgtattttt ttagcgtttt ttttagattt 4380
   ttttttagtt ttttaggttt gtgtcgtttg tttagatttt agatggggga ggggaggagt 4440
   agtttgaatt tttatttgag tttgggggga ggggttggtt aggtgtgttt ttttttagtt 4500
   gtattttgtt tttttttttt tttttattta ttttgttttt atttttcgtt atttttttaa 4560
   tttaatgata aatttaggtc gttaattcgt aatgacgtag atcgatttat agtttatatt 4620
   aacggttttt tattttcgag ttcggttaat ggatattagt tgggatttaa ggttaataaa 4680
   taatttaatt tgagatttcg cgtttgtttt tttttttttc gtttcgtttg tttttttttt 4740
   tttttttttt tttatttttt tttttttttt tgattattat tttttttttg gtgttttatt 4800
   ttgttttagt tttttattta tagggaaata tagttttaga tagatttaat tttttttttt 4860
   tagcgttatt ttttattttt ttgtatgacg tatttcgttt ttaatggagt cgtttttggt 4920
   tggggaattt tattagggtt gggagagggg ggttgggggt ttaaagtaaa gttaagttag 4980
   tgattgtagg tttttgtttt tttttttgtt ttggaatttc ggttttggtt aggcgtttta 5040
   ggagagtata tttgtttttt atggttttta gtgcggttgg gattttgtaa attttgtaat 5100
   ttttgtttaa taggggatat agcgagagat tttcggtcgg cgtttaattt ttttttattt 5160
   tttaattgag atgtttattt agtattttgt gggagattgg ggtagggggc gtttattagt 5220
   tttgtgtttc ggggtgggcg tttaggggag tcggggcgac ggcgacggga agttttattt 5280
   attgttgtac gaatttattc gttgtattta cgggtagatc ggagtggagt atttttgttt 5340
   ttttgtttcg tcgaatacgt ttttgttttg cgcgtagttc gattttcgcg gttcggggtg 5400
   gaacgggggt tgttcgtcgg cgtcggagag tgcgtaggtc gatttttttt cgcggtagaa 5460
   ggtcggcgtc ggttcgtagt cgtagggcgt cgttcggtcg tagttatcgt tcgtcggcgg 5520
   gtaataggag gaagtggtaa agtttttgtt ttggttcggt tttggttcgt agggcgcggg 5580
   gtaatgtttt agcgggttcg tatagttcga cgaatagagc ggtagttggt ttaggaagga 5640
   tttttgttcg ttggttagag tgacggggaa ggtggagttt acgaaatagg aatttattgg 5700
   gaggggaggc gcgcgcggtc gttgttgttt cgtcgggttt atgatttgtt gtgttttata 5760
   gttcgagcgt cgcgtttatt agtagtatat tcgagattgg gttttagttg aggggggatg 5820
   gcgaggtgtt agtgagggtt agaaggaaat ataattattt gatttaatat tgattaatgg 5880
   tagaggtagg aattgttaaa tagtatttag gaggagcgta gttcgtacga gggattcgcg 5940
   tataaattta ttaatttttt tttttttttt tttttttttc gttttttttt tttttttttt 6000
   taataatata acgcgttttg ttttatttac gtaggatttg tgaaataggt ttagtgtttt 6060
   tgagggagga gggaacgttt agattgagat tattttattt tcgggtgatt aaataaatat 6120
   gttttttttt tattttacgt aggattggaa aaagttaatt gtgtttttag aaaagttaat 6180
   ttttgcgttg tttcgatttt ttttttttag ggagggttgg gtaaatgttt tcgaatcgtt 6240
   ttgagagaga attaaagaag gtttgttttt agggttttgt gtcggtagaa ggtgagaaga 6300
   gtttttcgtt aagataagta aagaagaaat attttttttt ttaggttttg aatatcgttt 6360
   tttttgttta ttagatttgt tttgtttttt ttaagtttcg gtttattgtt tttgtttttt 6420
   tataattttt ttggtttgag ggggtaggtt agattttatt gtttagaaga tttagaagag 6480
   gaggagttgg taggagggat tgagtttaag tttttttttt tgattttgtg gttagattag 6540
   gagttttttg gaaggcggcg acgagggtgg ttgtttaaaa tattttgttt ggttttagta 6600
   tgtagttgtt tatttttggt aggggaaggg gtattgggga aagagattag tttttttttt 6660
   atttggtgat atggagtgaa ggagtgaggg gtatttttta gtttaggtgg ttttatttgt 6720
   ttgattttta ttttgtttgt aatttttatt ttagtatttt ttataatttt aaaagattta 6780
   agaatagttt gggttatagg atttttagga attagggatg ttttagtaaa tttgggtata 6840
   aggaagaaat tatttttttt ttagttgtgg gttttgaatt tttttgatag ttgaggtagt 6900
   tagagattat tgggaagaaa tgtattttta tttttttttt aagtttttgt aggagttgtt 6960
   agtttttttt aaaattaatt tttttttaag atttggtttt tgttttagtt tgatagggag 7020
   agaaggtcgt ttgcgttttt ttggggattt agtaggttgt ttttagttat tagttagtat 7080
   tttttttttt ttttgtttgt attttagtat cgttataatt tttttttata tttcgtgttt 7140
   gtattattag ttttgtcgtt ttataaattt aggtgtgtta gagaatgtat gtttttggtt 7200
   tttttattta aggaaaataa gtattgagtt taggttggga gatagggtta tagtgtattt 7260
   ttaatatttt aatatatata tataggttaa gtagtgaagg gtgataattt ttatatttgg 7320
   ggataagaag gggttagggt atggaataaa gttagatgtt aggttaggtt gatttttttt 7380
   tgttttgttt tttttttggg tttttttttt tggttaagtt ttgtttattg aagatggtat 7440
   aagatattag ggaaggtggt aggggattga gtttggatta ttaagggtgg gaatttattt 7500
   ggttgtaacg tggtttttaa ggttttttaa atttagggta attttttggt tgtattttaa 7560
   tggtaaaata gttattttta gatggttttt aattattttt taatttgatt ttgtgggagt 7620
   gtttgataag tatttaagaa aataaattta aattttttga taatgaagaa ggataaatat 7680
   tttatattta ggtatggttg ggtttgtttt tagagaggat taggggttat ttgaataggg 7740
   ggagaaaagt gggagagagt atttgtatgt atttttttgt ttattgttga ggttgagttt 7800
   tgggttttag ttttttttgt tttttttttg tttataatgt ttttatttta gtattaaata 7860
   gtgtggtttt tttagtgaag tttgaataaa ataagatttg gtgaggaagg gagtattttt 7920
   ttgtgttaga agtagggaaa taattgtttt atttttagag ttatagtttt taaattaatt 7980
   ttatttttgt aggttgtttg tttggagatg gagttattat aggcgggttt gggcgtttgg 8040
   agtcgggtat gaaagaaaat agcgttttat cgtttttatt taagtttaga ggtatttatg 8100
   agcgtttatt cgtttgattc gtttgtttgg gaaaaagagt aggcgggtag ggtaggttag 8160
   agatcgattt ttttttggag ttagattttt acgaatttgt ttgtggttcg gtttatcgtt 8220
   ttttgtttgt ttttttgggg gaatatgtaa atatatagcg ggtttagtta ttcgttagtt 8280
   tggggtgttt ttttttgttt taatgaggaa gtgtgtgttt agagagatgg atagaagtgt 8340
   gtattaattt ttgagtatat aatagaggga ttgtatagat attttatagg taggatttat 8400
   aattgtaatt ttttatgttg tgagtttgta gttttgtggg taagttaata tattttagtt 8460
   tttaggaaaa tgttttttaa gttgtgttta gttcgggagt aaaatttttg ataggtaatt 8520
   ttaggaggga tatttgcgtg tgtgtgtgtg tgtgtgtgtg tgtgtgtggt gtggtgtggt 8580
   gtgtgtggga gggtgttgag atattgttta ggtttttaat tttttttaag ttataaagtt 8640
   ttttttttta gttttttgag gtgatgagta ttttttttta ttatttttaa ttttggttgg 8700
   ggtggggtgg gatgaaaata ttttaattta attttcgttt tggattttgt ttttttgggt 8760
   ttggggaata taaataaatg tttttagggg tattgagtga ttatttttat aggttgaggg 8820
   aggttgttta gatgagatat tttgttttaa aatcggtttt tttaaattaa agtataaaga 8880
   ttttattttt attatgtaaa ggagcggtat tttttttgat ttttagggga gtgagtagta 8940
   gtgttgaatt tattttttcg gttttttatt cgtaggttta ggggataatt tgagggtttt 9000
   gaagtgtttt aatattatgt attagtttga tttttttata tgttttttgg attgtttaaa 9060
   agtaaaattt tagaggggtt aaggattttt tagggttggt ttttttaggg ttttttatag 9120
   agtagttttt tgagtcgggt tttatagtta ttagaaagtt atttcgtcgt aggaaagtta 9180
   taaagagttg ttttagaggc gtcgaaagag ggtgagggtt gtagttatag tgtttgtttt 9240
   tagtattatt aggtttttta atttcgttag tttttttagt tttcggttag attttggtaa 9300
   tattttggtt tgtttggtat cgtaggtaat tttttggaat tttattttta tttaggaacg 9360
   gggattatag tatttttaaa ggaattgtta gagttttttt ttacggtttg aaagtttaag 9420
   ttaattagta attcgtgaga taggggttaa tatttaaacg ttgtagtaga ggcgtatagt 9480
   ttagttttgg ttattttttt ttgggaagga ggtcgagtta agtcggtgtg gagggtttaa 9540
   taggaacgga aattacgacg gtgtggtttt ttaaggattt ggttttagtt ttattattaa 9600
   atttttttta aattatattt aggtagattt cgtttgaaaa gatcgagaat ggaggagggg 9660
   atcggtattt atttgtagat attaatagtt tttagaaaat tcggtttaat ttttttatta 9720
   cggatttatt aattaaggag ttggtaaaat ttattaatta attaggagaa tatagattcg 9780
   gtttgttttt tgataatcga gtggaattga cggggtcggc gtttgttttg cgggtttttt 9840
   tttttggttg agaagaaaat taaataaatt ttgaatagtt ttggtttcgg atgttttaag 9900
   cggttttatt ttttttcgcg ttttggtggg gtgggggtgg ggatggcggt gggggtgtcg 9960
   gttttgattt ttttattttt tgatttaggg tttttgttcg ttggaagtag aattagattt 10020
   ttttatatat gttttagagg aagtcgtttt ttttgttagt cgttaggttt ggggttagga 10080
   tgggcgggga cgggggtagt gggaaattgg ggatgatggg tggaggagag atttttgttt 10140
   tttaatttat atcggggaat taagatttag ttgagagcgt cggtgtgggt tcgaaatttt 10200
   tcgaatttcg tggattttgc gaggtcgggg ttttaggcgt cgggtatttt tggatagtgt 10260
   cgcggggttt gcggcgtcga agtcgcgtgt tttcgaacgg acggtagatg gtagtagatc 10320
   gtataatttg ggttatagcg aattgtttaa gggaggattt ataatttttt aagattatag 10380
   gagaaaaaga tttttagtat gggtggtttt gggaaatttt ttttttgaga atagttttcg 10440
   agtgtcgtgg gtgttggcgg agaattttag ttacgaagtt tagcggtttt tttaggcgtt 10500
   taggtcgggt ttgaatttag ggtatttatt tggggttata gagtcgtttt tttcgcgtta 10560
   taagtggtag cgtcgttttt tttttagttt aggattgaga taataaatat atttttattt 10620
   ttaaaacgtt ttagattttg taatttcggg gtaattagag gttcgaggtt gaatgttttg 10680
   tatttggttt taagaaa 10697

<210> 349
<211> 3001
<212> DNA
<213> Artificial Sequence

<220>
<223> chemically treated genomic DNA (Homo sapiens)

<400> 349

attttttgtt agaagtatgt tgtttaagcg tattagttgt gggtttatcg gttttttttt 60
ttgggagttt cgaatgttga ggtttagtat gtaggcgagt ggatttaggg atttagagat 120
tttaatattt agtgtttagt ttttttggtg ttttttggag agttaagttt ttaggttttg 180
gttttttttt tagttttttt gattttttat tttttttagg gttttaagtt gttatatttt 240
agttttataa tgaatagggt tttgttttta gagttgagta attcgggggg ggtttatata 300
ttagtttttt ttttgatttt atataggacg ttaaagagag taggttttat tcggtggtag 360
tttagggcgt gtttttttgg gaggagtggg agtggggaaa gagaaaagtt gaggagttag 420

agaaatagag tagggggttt gagaataagg agggaggagg gttagtttgc ggagggagga 480
agcggattgg gggtggaatg agttgggggt ggaatgagga gtttttggga aaagattagt 540
ttttagagta tggggaagtt ttttagtttt agggagatat agggaagatg taggtagtag 600
gttaaaggtt gcgggttttg ggagatggtt tagaaaggta ggaggaggaa tttgggagtg 660
gatggagaaa ggaaagtgat ttggtaggtt ttagagggag agagatagaa gttggggttg 720
agaagagtta gagtttgagg tggtagagtg gggttggggg tgtcgagtta attggggaga 780
ttagtgtagg gtgtgtgaag ggttttgggg gttgagtagg tgggaggttt tgatgtattt 840
agtgtttggt tgagtagtgg agaggagttt taggggtttt ggagagggtg tggaggtttt 900
tatatttgga gagaatgagg gggttgggtg gagagttagg ggagaagata acgtagttta 960
agaatagttt tttagtttgg gagttagagg gggttttcgg ggatggggtt gttttaggag 1020
attgtagggg tcggtaaaag gttaggagtg gggagtcggg ttatcggggg ttttttttgt 1080
gaaggtttta gggtttgggg gtgaagggag gtttgagagg gattattttt ttatgggttt 1140
ttaggaataa ggagagaaga gagttgttgg attttggaga gggttttgga gttggggggg 1200
gtttttagaa gatttagaat atgtgaacgg ggtttgttgg gtttgtgtgg ggtttcggag 1260
tgggggtatt tatttggttt gggtttcgtt taggttttgg tcggtgatgg ttattatttg 1320
ttgtagaatg ttttcgatgt tttgtttttt tcggtttttc gggatttttt cgttattttt 1380
atcggggttt tcgttatcgg gaggttcgtt agggttttta ggttttttat ttattaattt 1440
taggtttttt cggttttcgt ttggaggggg cggttttagt agtcgttcgt ttatagttgg 1500
gggggggttt tgaggttttt tttttgtttc gttttttttt tcgtttggtt attttttttt 1560
ttaaattcgt tggggtcgtt gtttttttcg ttttaatttt cgttcgtttg ttttcggttt 1620
tcggtttttt cgggggttta tttcggtatt gaagggagat ttgggatatc ggttgggttt 1680
tttataggag atttcggttt tcggcggcgg agaaaatgga gtcggagaga gagaggaggt 1740
ttaagcgggg gtgtgtgtga gagagaggga ggagggagga gggagaaggg gggggagcga 1800
gggagggagg ttgggggagg ggagtcggag aggaagagga ggggagaaga gaggaggaat 1860
agggaggagt tgggggcgga gagagagata tagaaataga ggaattgaga tttagtggag 1920
gaggggagag ggaagagggg atgaggggag gagacgggtt atttgaaaga tatgggaaag 1980
ttttttggtt ggattttcgc ggtttaggag tggggttgtg ttggcggttg ggggcgtttg 2040
ttatttgggt tttgaattag ggatttaagc gatgtggatt taggtcgttg gaatgtttgg 2100
gtttatcggt agtttagttc gtattttttg ttttaatgat tttttttttt gttttattta 2160
attaaaatcg aagagggggg ttgggggaga taattaggga ggtttttagt cgttgtttaa 2220
tgagttagta attaattagt cggggagggg agttggtttt tggttagatt ggggagagaa 2280
aaggtttttg gttttatttt tttatttttt atttcgtttg ggttttttag atattagttt 2340
gtagtttaga ggggaattat atttatttat ataattaaaa tattagatag atttagtagt 2400
agtggggagg gagggtgggt agggttgaag gtttatttat agttcgtaaa ttttttagtt 2460
ttagggatcg ggggtgttgg tagtgggatt gggagaatag ttttaatttt ttaggtgttt 2520
atttattttt tttttttatt gggaggaagg gtagagttgt tttttaggtt ataatttttt 2580
aggtggaggt ttgagttttt agattttatt gtttagtagt ttgataattg gtggtgtttt 2640
tataagttag ggaaaagatt tttagttatt ttttgagatg ggtgtttggg atttttttta 2700
ttgttttaag tttttatgga tttgtgggcg gggagttaaa tttttgtttt atttcgtttg 2760
tttttagagt ttgaggattt ttattttgtt tagtttttag ggaaggtgat gtgggagatg 2820
aatattgaga tttgtgtcgt gttttttagt ttttggtatt tttgttaagt aagagttgag 2880
ggtatgtaat gggttgttta gttttgagat tagtggtaag gaagggttgg ttggggttta 2940
agttttagta ggtgtgtgtg gggggtcggg atttttgttt ttttattcga tttttatttt 3000
g 3001

<210> 350
<211> 3001
<212> DNA
<213> Artificial Sequence

<220>
<223> chemically treated genomic DNA (Homo sapiens)

<400> 350

tagggtgggg gtcgaatgga ggagtaaagg tttcggtttt ttatatatat ttgttgagat 60
ttgagtttta attagttttt ttttgttatt ggttttaaag ttgggtagtt tattgtatgt 120
ttttaatttt tgtttggtag gggtattaga gattgaaaga tacggtataa attttaatat 180
ttatttttta tattattttt tttgggaatt ggatagggtg aaagttttta aattttggga 240
ataggcgaga tggaataggg atttaatttt tcgtttatag gtttatggga gtttgaggta 300
gtaaggggga ttttaggtat ttattttaag gagtggttgg gagttttttt tttaatttgt 360
ggggatatta ttagttgtta agttattagg tagtagggtt tgagggttta ggtttttatt 420
tgagaggtta taatttgaga gatagtttta tttttttttt tagtaagaag ggaaggtggg 480
tgggtatttg agagattaag attatttttt tagttttatt attagtattt tcgatttttg 540
agattgaggg gtttacgggt tgtgaatgga tttttagttt tgtttatttt ttttttttat 600
tgttgttgag tttgtttgat gttttggttg tgtgaataaa tataattttt ttttggattg 660

tagattggta tttggggggt ttaggcgggg tgaaaggtag gaaggtgagg ttagaggttt 720
tttttttttt tagtttggtt agaggttagt tttttttttc ggttggttaa ttattggttt 780
attaagtagc ggttggagat ttttttaatt atttttttta gttttttttt tcggttttaa 840
ttaagtagaa tagggagggg agttattaga ataagaaata cgaattgagt tgtcggtgaa 900
tttaggtatt ttagcggttt gagtttatat cgtttagatt tttgatttag gatttaggtg 960
atagacgttt ttagtcgtta atatagtttt atttttaggt cgcggaagtt tagttagggg 1020
gtttttttat attttttaga tggttcgttt ttttttttta tttttttttt tttttttttt 1080
tttttattag gttttagttt ttttgttttt gtgttttttt tttcgttttt agtttttttt 1140
tgtttttttt tttttttttt tttttttttt ttttcggttt ttttttttta gttttttttt 1200
ttcgtttttt tttttttttt tttttttttt tttttttttt tttatatata ttttcgtttg 1260
ggtttttttt tttttttcgg ttttattttt ttcgtcgtcg ggggtcgggg ttttttgtgg 1320
ggggtttagt cggtatttta ggtttttttt tagtgtcggg gtgaattttc gggggagtcg 1380
ggagtcgggg gtagacgggc gggggttggg gcggagggag tagcggtttt agcgagtttg 1440
gggggagaag taattaggcg gggggagggg cggagtaggg agggggtttt agggtttttt 1500
tttagttatg gacgaacggt tattggggtc gtttttttta ggcgggggtc gggggggttt 1560
gggattggtg agtggggagt ttgggggttt tggcgagttt ttcggtggcg gagatttcgg 1620
tgggggtagc gggggggttt cgggaggtcg agggaagtaa gatatcgggg atattttgta 1680
gtagataatg attattatcg attagagttt ggacgaggtt taggttaagt gagtgttttt 1740
atttcgggat tttatataga tttagtaaat ttcgtttata tgttttgaat tttttgggag 1800
ttttttttaa ttttagggtt ttttttagga tttaatagtt tttttttttt tttatttttg 1860
ggagtttata gaaaagtgat tttttttaaa ttttttttta tttttaggtt ttgaaatttt 1920
tatagaggga attttcggtg gttcggtttt ttatttttaa ttttttgtcg atttttgtag 1980
ttttttggaa tagttttatt ttcgggagtt ttttttggtt tttagattaa gaaattgttt 2040
ttgggttacg ttattttttt ttttaatttt ttatttagtt tttttatttt ttttagatgt 2100
ggagattttt atattttttt tagagttttt aaagtttttt tttattgttt agttagatat 2160
taggtgtatt aaagtttttt atttgtttag ttttaggatt tttttatata ttttatattg 2220
atttttttag ttagttcggt atttttagtt ttattttgtt attttaaatt ttgatttttt 2280
ttaattttag tttttgtttt tttttttttg aaatttatta agttattttt ttttttttat 2340
ttatttttag attttttttt ttattttttt agattatttt ttaaagttcg tagtttttaa 2400
tttgttgttt gtattttttt tgtgtttttt tgaagttgag gagttttttt atgttttggg 2460
agttgatttt tttttaagaa ttttttattt tatttttaat ttattttatt tttaattcgt 2520
tttttttttt cgtagattga tttttttttt tttttgtttt taggtttttt gttttgtttt 2580
tttagttttt taattttttt ttttttttat ttttattttt tttaaggaaa tacgttttaa 2640
attgttatcg aatgaagttt gtttttttta gcgttttgtg tgaaattaag gagaaaattg 2700
gtatgtgggt ttttttcgga ttgtttaatt ttgggaatag aattttgttt attatagggt 2760
tagagtgtga taatttgggg ttttgaggaa agtaaggagt tagggggatt ggggaaggaa 2820
ttaaagtttg ggaatttggt tttttaggaa gtattaggag gattgagtat tgggtattgg 2880
ggtttttggg tttttaagtt tattcgtttg tatgttaggt tttagtattc ggagttttta 2940
ggaggaggag tcggtggatt tatagttgat gcgtttggat aatatgtttt tggtagaggg 3000
t 3001

<210> 351
<211> 25
<212> DNA
<213> Artificial Sequence

<220>
<223> PBX2 detection primer

<400> 351

gtttttagaa gatttagaat atgtg 25

<210> 352
<211> 21
<212> DNA
<213> Artificial Sequence

<220>
<223> PBX2 detection primer

<400> 352

ccactaaatc tcaattcctc t 21

<210> 353

<211> 18
<212> DNA
<213> Artificial Sequence

<220>
<223> PBX2 detection oligonucleotide

<400> 353

tgggatatcg gttgggtt                                                    18

<210> 354
<211> 18
<212> DNA
<213> Artificial Sequence

<220>
<223> PBX2 detection oligonucleotide

<400> 354

tgggatattg gttgggtt                                                    18

## Description

### Field of the Invention

The levels of observation that have been well studied by the methodological developments of recent years in molecular biology, are the genes themselves, the translation of these genes into RNA, and the resulting proteins. The question of which gene is switched on at which point in the course of the development of an individual, and how the activation and inhibition of specific genes in specific cells and tissues are controlled is correlatable to the degree and character of the methylation of the genes or of the genome. In this respect, pathogenic conditions may manifest themselves in a changed methylation pattern of individual genes or of the genome.

The present invention relates to nucleic acids, oligonucleotides, PNA-oligomers and to a method for the diagnosis and/or therapy of diseases which have a connection with the genetic and/or epigenetic parameters of genes associated with development and, in particular, with the methylation status thereof.

### Prior Art

The ordered progression of embryonic development is controlled by a complex genetic regulatory system. Initial identification of key genes involved in development were in model organisms such as Drosophila. This was followed by the identification of highly conserved human homologues e.g. the frizzled gene and its human homologue. Examples of important transcription factors involved in development include the homeobox, MAD, PAX, EMX and MSX gene families (see, e.g. Terzic J, Saraga-Babic M.. Expression pattern of PAX3 and PAX6 genes during human embryogenesis. Int J Dev Biol 1999 Sep;43(6):501-8)

Genes involved in development have been implicated in a wide range of diseases. For example, the homeobox containing genes (HOX), are primarily involved in normal development. However, the HOX genes have also been implicated in normal adult cellular functioning and a wide range of disease including diabetes and cancer ('Homeobox genes in normal and malignant cells' Cillo C *et. Al*. J Cell Physiol 2001 Aug;188(2):161-9. 'Homeobox genes in leukemogenesis' Buske C and Humphries RK Int J Hematol 2000 Jun;71(4):301-8. 'HOX genes in human cancers.' Cillo C Invasion Metastasis 1994-95;14(1-6):38-49). Other developmental related diseases comprise apoptosis related diseases (see, e.g.Sano M, et al. Involvement of EAT/mcl-1, an anti-apoptotic bcl-2-related gene, in murine embryogenesis and human development. Exp Cell Res 2000 Aug 25;259(1):127-39; Muller S, et al. Retention of imprinting of the human apoptosis-related gene TSSC3 in human brain tumors. Hum Mol Genet 2000 Mar 22;9(5):757-63), syndromes associated with congenital heart disease (see, e.g. Gelb BD. Genetic basis of syndromes associated with congenital heart disease. Curr Opin Cardiol 2001 May;16(3):188-94) epilepsy (see, e.g. Doose H, Neubauer BA, Petersen B. The concept of hereditary impairment of brain maturation. Epileptic Disord 2000;2 Suppl 1:S45-9, diseases related to histone deacetylation (see, e.g. El-Osta A, Wolffe AP. DNA methylation and histone deacetylation in the control of gene expression: basic biochemistry to human development and disease. Gene Expr 2000;9(1-2):63-75) Currarino syndrome (see e.g. Hagan DM, et al. Mutation analysis and embryonic expression of the HLXB9 Currarino syndrome gene. Am J Hum Genet 2000 May;66(5):1504-15 Erratum in: Am J Hum Genet 2000 Sep;67(3):769), diseases related with the development of the brain and limb girdle muscular dystrophy (Wnt7a and CAPN3, see, e.g. Fougerousse F, et al. Human-mouse differences in the embryonic expression patterns of developmental control genes and disease genes. Hum Mol Genet 2000 Jan 22;9(2):165-73; Hum Mol Genet 2000 Mar 1;9(4):659), dwarfism (see, e.g. Iwata T, et al. Highly activated Fgfr3 with the K644M mutation causes prolonged survival in severe dwarf mice. Hum Mol Genet 2001 Jun 1;10(12):1255-1264), and others (see, e.g. Hanel ML, Wevrick R. The role of genomic imprinting in human developmental disorders: lessons from Prader-Willi syndrome. Clin Genet 2001 Mar;59(3):156-64).

5-methylcytosine is the most frequent covalent base modification in the DNA of eukaryotic cells. It plays a role, for example, in the regulation of the transcription, in genetic imprinting, and in tumorigenesis.

The importance of methylation in developmental genes has been well established. Aberrant genomic imprinting (parental origin specific methylation) results in abnormal embryonic development. For example, in mice the IGF2 gene is differentially methylated according to the parental origin of the allele. The allele is thought ot be relevant during late embryonic development and act to regulate growth. Aberrant methylation results in gross abnormalities, the resultant foetus being unviable, or dying at birth. Furthermore, this gene has been shown to be significant in the development of human cancers and growth disorders such as Beckwith-Wiedemann (Transactivation of Igf2 in a mouse model of Beckwith-Wiedemann syndrome. Sun *et. Al*. Nature 389, 809 - 815. 1997).

Aberrant DNA methylation within CpG islands is common in human malignancies leading to abrogation or overexpression of a broad spectrum of genes (Jones, P.A. Cancer Res 65:2463-2467, 1996). Abnormal methylation has also been shown to occur in CpG rich regulatory elements in intronic and coding parts of genes for certain tumours (Chan, M.F., et al., Curr Top Microbiol Immunol 249:75-86,2000). Using restriction landmark genomic scanning, Costello and coworkers were able to show that methylation patterns are tumour-type specific (Costello, J. F., et al., Nat Genet 24:132-138, 2000). Highly characteristic DNA methylation patterns could also be shown for breast cancer cell lines (Huang, T. H.-M., et al., Hum Mol Genet 8:459-470, 1999). Genome wide assessment of methylation status represents a molecular fingerprint of cancer tissues.

Therefore, the identification of 5-methylcytosine as a component of genetic information is of considerable interest. However, 5-methylcytosine positions cannot be identified by sequencing since 5-methylcytosine has the same base pairing development as cytosine. Moreover, the epigenetic information carried by 5-methylcytosine is completely lost during PCR amplification.

A relatively new and currently the most frequently used method for analyzing DNA for 5-methylcytosine is based upon the specific reaction of bisulfite with cytosine which, upon subsequent alkaline hydrolysis, is converted to uracil which corresponds to thymidine in its base pairing behaviour. However, 5-methylcytosine remains unmodified under these conditions. Consequently, the original DNA is converted in such a manner that methylcytosine, which originally could not be distinguished from cytosine by its hybridization behaviour, can now be detected as the only remaining cytosine using "normal" molecular biological techniques, for example, by amplification and hybridization or sequencing. All of these techniques are based on base pairing which can now be fully exploited. In terms of sensitivity, the prior art is defined by a method which encloses the DNA to be analyzed in an agarose matrix, thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15;24(24):5064-6). Using this method, it is possible to analyze individual cells, which illustrates the potential of the method. However, currently only individual regions of a length of up to approximately 3000 base pairs are analyzed, a global analysis of cells for thousands of possible methylation events is not possible. However, this method cannot reliably analyze very small fragments from small sample quantities either. These are lost through the matrix in spite of the diffusion protection.

An overview of the further known methods of detecting 5-methylcytosine may be gathered from the following review article: Rein, T., DePamphilis, M. L., Zorbas, H., Nucleic Acids Res. 1998, 26, 2255.

To date, barring few exceptions (e.g., Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) the bisulfite technique is only used in research. Always, however, short, specific fragments of a known gene are amplified subsequent to a bisulfite treatment and either completely sequenced (Olek A, Walter J. The pre-implantation ontogeny of the H19 methylation imprint. Nat Genet. 1997 Nov;17(3):275-6) or individual cytosine positions are detected by a primer extension reaction (Gonzalgo ML, Jones PA. Rapid quantitation of methylation differences at specific sites using methylation-sensitive single nucleotide primer extension (Ms-SnuPE). Nucleic Acids Res. 1997 Jun 15;25(12):2529-31, WO 95/00669) or by enzymatic digestion (Xiong Z, Laird PW. COBRA: a sensitive and quantitative DNA methylation assay. Nucleic Acids Res. 1997 Jun 15;25(12):2532-4). In addition, detection by hybridization has also been described (Olek et al., WO 99/28498).

Further publications dealing with the use of the bisulfite technique for methylation detection in individual genes are: Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97/46705, WO 95/15373 and WO 97/45560.

An overview of the Prior Art in oligomer array manufacturing can be gathered from a special edition of Nature Genetics (Nature Genetics Supplement, Volume 21, January 1999), published in January 1999, and from the literature cited therein.

Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example via a confocal microscope. Cy3 and Cy5 dyes, besides many others, are commercially available.

Matrix Assisted Laser Desorption Ionization Mass Spectrometry (MALDI-TOF) is a very efficient development for the analysis of biomolecules (Karas M, Hillenkamp F. Laser desorption ionization of proteins with molecular masses exceeding 10,000 daltons. Anal Chem. 1988 Oct 15;60(20):2299-301). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones.

MALDI-TOF spectrometry is excellently suited to the analysis of peptides and proteins. The analysis of nucleic acids is somewhat more difficult (Gut I G, Beck S. DNA and Matrix Assisted Laser Desorption Ionization Mass Spectrometry. Current Innovations and Future Trends. 1995, 1; 147-57). The sensitivity to nucleic acids is approximately 100 times worse than to peptides and decreases disproportionally with increasing fragment size. For nucleic acids having a multiply negatively charged backbone, the ionization process via the matrix is considerably less efficient. In MALDI-TOF spectrometry, the selection of the matrix plays an eminently important role. For the desorption of peptides, several very efficient matrixes have been found which produce a very fine crystallization. There are now several responsive matrixes for DNA, however, the difference in sensitivity has not been reduced. The difference in sensitivity can be reduced by chemically modifying the DNA in such a manner that it becomes more similar to a peptide. Phosphorothioate nucleic acids in which the usual phosphates of the backbone are substituted with thiophosphates can be converted into a charge-neutral DNA using simple alkylation chemistry (Gut IG, Beck S. A procedure for selective DNA alkylation and detection by mass spectrometry. Nucleic Acids Res. 1995 Apr 25;23(8):1367-73). The coupling of a charge tag to this modified DNA results in an increase in sensitivity to the same level as that found for peptides. A further advantage of charge tagging is the increased stability of the analysis against impurities which make the detection of unmodified substrates considerably more difficult.

Genomic DNA is obtained from DNA of cell, tissue or other test samples using standard methods. This standard methodology is found in references such as Fritsch and Maniatis eds., Molecular Cloning: A Laboratory Manual, 1989.

### Description

The object of the present invention is to provide the chemically modified DNA of genes associated with diseases associated with development genes, as well as oligonucleotides and/or PNA-oligomers for detecting cytosine methylations, as well as a method which is particularly suitable for the diagnosis and/or therapy of genetic and epigenetic parameters of genes associated development, and diseases associated with those genes. The present invention is based on the discovery that genetic and epigenetic parameters and, in particular, the cytosine methylation pattern of genes associated with development are particularly suitable for the diagnosis and/or therapy of diseases.

This objective is achieved according to the present invention using a nucleic acid containing a sequence of at least 18 bases in length of the chemically pretreated DNA of genes associated with development according to one of Seq. ID No.1 through Seq. ID No.350 and sequences complementary thereto and/or a chemically pretreated DNA of genes associated with diseases associated with development according to one of the sequences according to the genes according to table 1. In the table, after the listed gene designations, the respective data bank numbers (accession numbers) are specified which define the appertaining gene sequences as unique. GenBank was used as the underlying data bank which is located at internet address http://www.ncbi.nlm.nih.gov

The chemically modified nucleic acid could heretofore not be connected with the ascertainment of genetic and epigenetic parameters.

The object of the present invention is further achieved by an oligonucleotide or oligomer for detecting the cytosine methylation state in chemically pretreated DNA, containing at least one base sequence having a length of at least 13 nucleotides which hybridizes to a chemically pretreated DNA of genes associated with development according to Seq. ID No.1 through Seq. ID No.350 and sequences complementary thereto and/or a chemically pretreated DNA of genes associated with diseases associated with development according to one of the sequences according to the genes according to table 1. The oligomer probes according to the present invention constitute important and effective tools which, for the first time, make it possible to ascertain the genetic and epigenetic parameters of genes associated with development. The base sequence of the oligomers preferably contains at least one CpG dinucleotide. The probes may also exist in the form of a PNA (peptide nucleic acid) which has particularly preferred pairing properties. Particularly preferred are oligonucleotides according to the present invention in which the cytosine of the CpG dinucleotide is the 5^{th} - 9^{th} nucleotide from the 5'-end of the 13-mer; in the case of PNA-oligomers, it is preferred for the cytosine of the CpG dinucleotide to be the 4^{th} - 6^{th} nucleotide from the 5'-end of the 9-mer.

The oligomers according to the present invention are normally used in so called "sets" which contain at least one oligomer for each of the CpG dinucleotides of the sequences of Seq. ID No.1 through Seq. ID No.350 and sequences complementary thereto and/or a chemically pretreated DNA of genes associated with diseases associated with development according to one of the sequences according to the genes according to table 1. Preferred is a set which contains at least one oligomer for each of the CpG dinucleotides from one of Seq. ID No.1 through Seq. ID No.350 and sequences complementary thereto and/or a chemically pretreated DNA of genes associated with diseases associated with development according to one of the sequences according to the genes according to table 1.

Moreover, the present invention makes available a set of at least two oligonucleotides which can be used as so-called "primer oligonucleotides" for amplifying DNA sequences of one of Seq. ID No.1 through Seq. ID No.350 and sequences complementary thereto and/or a chemically pretreated DNA of genes associated with diseases associated with development according to one of the sequences according to the genes according to table 1, or segments thereof.

In the case of the sets of oligonucleotides according to the present invention, it is preferred that at least one oligonucleotide is bound to a solid phase. It is further preferred that all the oligonucleotides of one set are bound to a solid phase.

The present invention moreover relates to a set of at least 10 n (oligonucleotides and/or PNA-oligomers) used for detecting the cytosine methylation state in chemically pretreated genomic DNA (Seq. ID No.1 through Seq. ID No.350 and sequences complementary thereto and/or a chemically pretreated DNA of genes associated with diseases associated with development according to one of the sequences according to the genes according to table 1). These probes enable diagnosis and/or therapy of genetic and epigenetic parameters of genes associated with development, and associated diseases. The set of oligomers may also be used for detecting single nucleotide polymorphisms (SNPs) in the chemically pretreated DNA of genes associated with diseases associated with development according to one of Seq. ID No.1 through Seq. ID No.350 and sequences complementary thereto and/or a chemically pretreated DNA of genes associated with diseases associated with development according to one of the sequences according to the genes according to table 1.

According to the present invention, it is preferred that an arrangement of different oligonucleotides and/or PNA-oligomers (a so-called "array") made available by the present invention is present in a manner that it is likewise bound to a solid phase. This array of different oligonucleotide- and/or PNA-oligomer sequences can be characterized in that it is arranged on the solid phase in the form of a rectangular or hexagonal lattice. The solid phase surface is preferably composed of silicon, glass, polystyrene, aluminum, steel, iron, copper, nickel, silver, or gold. However, nitrocellulose as well as plastics such as nylon which can exist in the form of pellets or also as resin matrices are possible as well.

Therefore, a further subject matter of the present invention is a method for manufacturing an array fixed to a carrier material for analysis in connection with diseases associated with development genes in which method at least one oligomer according to the present invention is coupled to a solid phase. Methods for manufacturing such arrays are known, for example, from US Patent 5,744,305 by means of solid-phase chemistry and photolabile protecting groups.

A further subject matter of the present invention relates to a DNA chip for the analysis of diseases associated with development genes which contains at least one nucleic acid according to the present invention. DNA chips are known, for example, for US Patent 5,837,832.

Moreover, a subject matter of the present invention is a kit which may be composed, for example, of a bisulfite-containing reagent, a set of primer oligonucleotides containing at least two oligonucleotides whose sequences in each case correspond or are complementary to an 18 base long segment of the base sequences specified in the appendix (Seq. ID No.1 through Seq. ID No.350 and sequences complementary thereto and/or a chemically pretreated DNA of genes associated with diseases associated with development according to one of the sequences according to the genes according to table 1.), oligonucleotides and/or PNA-oligomers as well as instructions for carrying out and evaluating the described method. However, a kit along the lines of the present invention can also contain only part of the aforementioned components.

The present invention also makes available a method for ascertaining genetic and/or epigenetic parameters of development genes associated with diseases by analyzing cytosine methylations and single nucleotide polymorphisms, including the following steps:

In the first step of the method, a genomic DNA sample is chemically treated in such a manner that cytosine bases which are unmethylated at the 5'-position are converted to uracil, thymine, or another base which is dissimilar to cytosine in terms of hybridization behaviour. This will be understood as 'chemical pretreatment' hereinafter.

The genomic DNA to be analyzed is preferably obtained form usual sources of DNA such as

cells or cell components, for example, cell lines, biopsies, blood, sputum, stool, urine, cerebral-spinal fluid, tissue embedded in paraffin such as tissue from eyes, intestine, kidney, brain, heart, prostate, lung, breast or liver, histologic object slides, or combinations thereof.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behaviour.

Fragments of the chemically pretreated DNA are amplified, using sets of primer oligonucleotides according to the present invention, and a, preferably heat-stable polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).

In a preferred embodiment of the method, the set of primer oligonucleotides includes at least two oligonucleotides whose sequences are each reverse complementary or identical to an at least 18 base-pair long segment of the base sequences specified in the appendix (Seq. ID No.1 through Seq. ID No.350 and sequences complementary thereto and/or a chemically pretreated DNA of genes associated with diseases associated with development according to one of the sequences according to the genes according to table 1). The primer oligonucleotides are preferably characterized in that they do not contain any CpG dinucleotides.

According to the present invention, it is preferred that at least one primer oligonucleotide is bonded to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminium, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualized by means of matrix assisted laser desorption/ionization mass spectrometry (MALDI) or using electron spray mass spectrometry (ESI).

The amplificates obtained in the second step of the method are subsequently hybridized to an array or a set of oligonucleotides and/or PNA probes. In this context, the hybridization takes place in the manner described in the following. The set of probes used during the hybridization is preferably composed of at least 10 oligonucleotides or PNA-oligomers. In the process, the amplificates serve as probes which hybridize to oligonucleotides previously bonded to a solid phase. The non-hybridized fragments are subsequently removed. Said oligonucleotides contain at least one base sequence having a length of 13 nucleotides which is reverse complementary or identical to a segment of the base sequences specified in the appendix, the segment containing at least one CpG dinucleotide. The cytosine of the CpG dinucleotide is the 5^{th} to 9^{th} nucleotide from the 5'-end of the 13-mer. One oligonucleotide exists for each CpG dinucleotide. Said PNA-oligomers contain at least one base sequence having a length of 9 nucleotides which is reverse complementary or identical to a segment of the base sequences specified in the appendix, the segment containing at least one CpG dinucleotide. The cytosine of the CpG dinucleotide is the 4^{th} to 6^{th} nucleotide seen from the 5'-end of the 9-mer. One oligonucleotide exists for each CpG dinucleotide.

In the fourth step of the method, the non-hybridized amplificates are removed.

In the final step of the method, the hybridized amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualized by means of matrix assisted laser desorption/ionization mass spectrometry (MALDI) or using electron spray mass spectrometry (ESI).

The produced fragments may have a single positive or negative net charge for better detectability in the mass spectrometer. The aforementioned method is preferably used for ascertaining genetic and/or epigenetic parameters of genes associated with development and diseases.

The oligomers according to the present invention or arrays thereof as well as a kit according to the present invention are intended to be used for the diagnosis and/or therapy of diseases associated with development genes by analyzing methylation patterns of developmental genes associated with diseases. According to the present invention, the method is preferably used for the diagnosis and/or therapy of important genetic and/or epigenetic parameters within developmental genes associated with diseases.

The method according to the present invention is used, for example, for the diagnosis and/or therapy of diseases associated with development genes.

The nucleic acids according to the present invention of Seq. ID No.1 through Seq. ID No.350 and sequences complementary thereto and/or a chemically pretreated DNA of genes associated with diseases associated with development according to one of the sequences according to the genes according to table 1 can be used for the diagnosis and/or therapy of genetic and/or epigenetic parameters of development genes associated with diseases.

The present invention moreover relates to a method for manufacturing a diagnostic agent and/or therapeutic agent for the diagnosis and/or therapy of diseases associated with development by analyzing methylation patterns of genes associated with diseases associated with development, the diagnostic agent and/or therapeutic agent being characterized in that at least one nucleic acid according to the present invention is used for manufacturing it, possibly together with suitable additives and auxiliary agents.

A further subject matter of the present invention relates to a diagnostic agent and/or therapeutic agent for diseases associated with development genes by analyzing methylation patterns of development genes associated with diseases, the diagnostic agent and/or therapeutic agent containing at least one nucleic acid according to the present invention, possibly together with suitable additives and auxiliary agents. Such diseases associated with development are, e.g. diseases related to homeobox containing genes (HOX), e.g. diabetes and cancer, apoptosis related diseases, syndromes associated with congenital heart disease, epilepsy, diseases related to histone deacetylation, Currarino syndrome, diseases related with the development of the brain and limb girdle muscular dystrophy, dwarfism, and others.

The present invention moreover relates to the diagnosis and/or prognosis of events which are disadvantageous to patients or individuals in which important genetic and/or epigenetic parameters within development genes associated with diseases, said parameters obtained by means of the present invention may be compared to another set of genetic and/or epigenetic parameters, the differences serving as the basis for a diagnosis and/or prognosis of events which are disadvantageous to patients or individuals. Such diseases associated with development are, e.g. diseases related to homeobox containing genes (HOX), e.g. diabetes and cancer, apoptosis related diseases, syndromes associated with congenital heart disease, epilepsy, diseases related to histone deacetylation, Currarino syndrome, diseases related with the development of the brain and limb girdle muscular dystrophy, dwarfism, and others.

In the context of the present invention the term "hybridization" is to be understood as a bond of an oligonucleotide to a completely complementary sequence along the lines of the Watson-Crick base pairings in the sample DNA, forming a duplex structure. To be understood by "stringent hybridization conditions" are those conditions in which a hybridization is carried out at 60°C in 2.5 x SSC buffer, followed by several washing steps at 37°C in a low buffer concentration, and remains stable.

The term "functional variants" denotes all DNA sequences which are complementary to a DNA sequence, and which hybridize to the reference sequence under stringent conditions and have an activity similar to the corresponding polypeptide according to the present invention.

In the context of the present invention, "genetic parameters" are mutations and polymorphisms of development genes associated with diseases and sequences further required for their regulation. To be designated as mutations are, in particular, insertions, deletions, point mutations, inversions and polymorphisms and, particularly preferred, SNPs (single nucleotide polymorphisms).

In the context of the present invention, "epigenetic parameters" are, in particular, cytosine methylations and further chemical modifications of DNA bases of genes associated with diseases associated with development and sequences further required for their regulation. Further epigenetic parameters include, for example, the acetylation of histones which, however, cannot be directly analyzed using the described method but which, in turn, correlates with the DNA methylation.

In the following, the present invention will be explained in greater detail on the basis of the sequences and examples with reference to the accompanying drawing, without being limited thereto.

### Figure 1

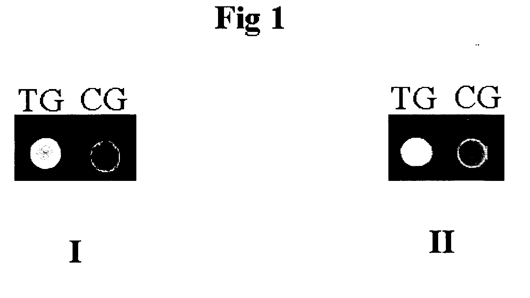

Figure 1 shows the hybridisation of fluorescent labelled amplificates to a surface bound olignonucleotide. Flourescence at a spot shows hybridisation of the amplificate to the olignonucleotide. Hybridisation to a CG olignonucleotide denotes methylation at the cytosine position being analysed, hybridisation to a TG olignonucleotide denotes no methylation at the cytosine position being analysed. It can be seen that Sample II had a higher degree of methylation than Sample I.

### Seq. ID No. 1 trough Seq. ID No. 350

Sequences having odd sequence numbers (e.g., Seq. ID No. 1, 3, 5, ...) exhibit in each case sequences of the chemically pretreated genomic DNAs of different genes associated with diseases associated with development. Sequences having even sequence numbers (e.g., Seq. ID No. 2, 4, 6, ...) exhibit in each case the sequences of chemically pretreated genomic DNAs. Said genomic DNAs are complementary to the genomic DNAs from which the preceding sequence was derived (e.g., the complementary sequence to the genomic DNA from which Seq. ID No.1 is derived is the genomic sequence from which Seq. ID No.2 is derived, the complementary sequence to the genomic DNA from which Seq. ID No.3 is derived is the sequence from which Seq. ID No.4 is derived, etc.)

### Seq. ID No. 351 trough Seq. ID No. 354

Seq. ID No. 351 trough Seq. ID No. 354 show sequences of oligonucleotides used in Example 1.

### Example 1 :Methylation analysis of the gene PBX2.

The following example relates to a fragment of the gene PBX2 in which a specific CG-position is to be analyzed for methylation.

In the first step, a genomic sequence is treated using bisulfite (hydrogen sulfite, disulfite) in such a manner that all cytosines which are not methylated at the 5-position of the base are modified in such a manner that a different base is substituted with regard to the base pairing behavior while the cytosines methylated at the 5-position remain unchanged.

If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The chemically converted DNA is then used for the detection of methylated cytosines. In the second method step, the treated DNA sample is diluted with water or an aqueous solution. Preferably, the DNA is subsequently desulfonated. In the third step of the method, the DNA sample is amplified in a polymerase chain reaction, preferably using a heat-resistant DNA polymerase. In the present case, cytosines of the gene PBX2 are analyzed. To this end, a defined fragment having a length of 718 bp is amplified with the specific primer oligonucleotides GTTTTTAGAAGATTTAGAATATGTG (Sequence ID 47) and CCACTAAATCTCAATTCCTCT (Sequence ID No. 48). The single gene PCR reaction was performed on a thermocycler (Eppendorf GmbH) using bisulfite DNA 10 ng, primer 6 pmole each, dNTP 200 µM each, 1.5 mM MgCl2 and 1 U HotstartTaq (Qiagen AG). The other conditions were as recommended by the Taq polymerase manufacturer. In the multiplex PCR up to 16 primer pairs were used within the PCR reaction. The multiplex PCR was done according the single gene PCR with the following modifications: primer 0.35 pmole each, dNTP 800 µM each and 4,5 mM MgCl2. The cycle program for single gene PCR and multiplex PCR was as followed: step 1,14 min 96 °C; step 2, 60 sec 96°C; step 3, 45 sec 55 °C; step 4 ,75 sec 72 °C; step 5, 10 min 72 °C; the step 2 to step 4 were repeated 39 fold.

The amplificate serves as a sample which hybridizes to an oligonucleotide previously bound to a solid phase, forming a duplex structure, for example TGGGATATCGGTTGGGTT (Sequence ID No. 49), the cytosine to be detected being located at position 470 of the amplificate. The detection of the hybridization product is based on Cy3 and Cy5 fluorescently labelled primer oligonucleotides which have been used for the amplification. A hybridization reaction of the amplified DNA with the oligonucleotide takes place only if a methylated cytosine was present at this location in the bisulfite-treated DNA. Thus, the methylation status of the specific cytosine to be analyzed is inferred from the hybridization product.

In order to verify the methylation status of the position, a sample of the amplificate is further hybridized to another oligonucleotide previously bonded to a solid phase. Said olignonucleotide is identical to the oligonucleotide previously used to analyze the methylation status of the sample, with the exception of the position in question. At the position to be analysed said oligonucleotide comprises a thymine base as opposed to a cytosine base i.e TGGGATATTGGTTGGGTT (Sequence ID No. 50). Therefore, the hybridisation reaction only takes place if an unmethylated cytosine was present at the position to be analysed.

### Example 2: Diagnosis of diseases associated with development genes

In order to relate the methylation patterns to one of the diseases associated with development, it is initially required to analyze the DNA methylation patterns of a group of diseased and of a group of healthy patients. These analyses are carried out, for example, analogously to Example 1. The results obtained in this manner are stored in a database and the CpG dinucleotides which are methylated differently between the two groups are identified. This can be carried out by determining individual CpG methylation rates as can be done, for example, in a relatively imprecise manner, by sequencing or else, in a very precise manner, by a methylation-sensitive "primer extension reaction". It is particularly preferred that the determination be carried out in the manner described in Example 1, bisulphite treatment of genomic DNA followed by fluorescence hybridisation analysis on an oligomer array, thereby enabling the simultaneous analysis of multiple positions within the genome. It is also possible for the entire methylation status to be analyzed simultaneously, and for the patterns to be compared, for example, by clustering analyses which can be carried out, for example, by a computer.

Subsequently, it is possible to allocate the examined patients to a specific therapy group and to treat these patients selectively with an individualized therapy.

Example 2 can be carried out, for example, for diseases associated with development, such as diseases related to homeobox containing genes (HOX), e.g. diabetes and cancer, apoptosis related diseases, syndromes associated with congenital heart disease, epilepsy, diseases related to histone deacetylation, Currarino syndrome, diseases related with the development of the brain and limb girdle muscular dystrophy, dwarfism, and others.

**Table 1 List of preferred genes associated with development according to the invention**

| ***Gene*** | ***Genbank Entry No.* *(http.*//*www.ncbi.nlm*.*nih.gov)*** |
|---|---|
| ACCPN | n.a.* |
| ADFN | n.a.* |
| AFD1 | n.a.* |
| AHO2 | n.a.* |
| AIH3 | n.a.* |
| AMCD1 | n.a.* |
| AMCD2B | n.a.* |
| AMCN | n.a.* |
| AMCX1 | n.a.* |
| AMDM | n.a.* |
| ANOP1 | n.a.* |
| ASMD | n.a.* |
| ATD | n.a.* |
| AXIN1 | n.a.* |
| AF009674 | n.a.* |
| BDB1 | n.a.* |
| BDC | n.a.* |
| BDE | n.a.* |
| BDMR | n.a.* |
| CHH | n.a.* |
| CHRD | AF076612 |
| CHX10 | n.a.* |
| EED | U90651, AF070418, AF078933, AF080227 |
| EPHB2 | L41939, AF025304 |
| FZD8 | n.a.* |
| GLI4 | n.a.* |
| HNF4B | n.a.* |
| HOXA3 | n.a.* |
| HOXA6 | n.a.* |
| HOXB9 | n.a.* |
| HOXC9 | n.a.* |
| HOXD8 | n.a. * |
| IHH | L38517 |
| LMX1A | n.a.* |
| MEIS3 | U68385 |
| ORW2 | n.a.* |
| PKHD1 | n.a.* |
| RIEG2 | n.a.* |
| SFRP2 | AF017986 |
| SOX10 | n.a.* |
| TBX15 | n.a.* |
| TBX18 | AJ010278 |
| TBX7 | n.a.* |
| TFCOUP2 | M62760, M64497, U60477 |
| WNT14 | AF028702 |
| WNT15 | AF028703 |
| WNT3 | n.a. * |
| WNT6 | n.a.* |
| WNT7B | n.a.* |
| WNT8A | n.a.* |
| FGF2 | NM_002006 |
| LHX2 | NM_004789 |
| TBX6 | NM_004608 |
| ZIC3 | NM_003413 |
| ACVR2B | NM_001106 |
| APC | NM_000038 |
| BDNF | NM_001709 |
| BMP1 | NM_006129 |
| BMPR1B | NM_001203 |
| BMPR2 | NM_001204 |
| EDR2 | NM_004427 |
| FGF16 | NM_003868 |
| FGF3 | NM_005247 |
| FGF4 | NM_002007 |
| FGFR3 | NM_000142 and NM_022965 |
| FRZB | NM_001466 |
| FZD7 | NM_003507 |
| GLI2 | NM_005270 |
| HNF3A | NM_004496 |
| HOXB4 | NM_024015 |
| HOXB8 | NM_024016 |
| HOXC8 | NM_022658 |
| HOXD1 | NM_024501 |
| INHBB | NM_002193 |
| LMX1B | NM_002316) |
| MADH3 | NM_005902 |
| PAX1 | NM_006192 |
| PAX9 | NM_006194 |
| PBX3 | NM 006195 |
| PROP1 | NM_006261 |
| PTCH2 | NM_003738 |
| RARG | NM_000966 |
| RXRA | NM_002957 |
| SFRP5 | NM_003015 |
| SOX1 | NM_005986 |
| TGFBR1 | NM_004612 |
| WNT2B | NM_004185 |
| GSC | n.a.* |

| | |
|---|---|
| • "n.a." designates genes for which no meaningful sequences could be identified | |

## Claims

1. A nucleic acid comprising a sequence at least 18 bases in length of a segment of the chemically pretreated DNA of genes associated with development according to one of the sequences taken from the group of Seq. ID No.1 to Seq. ID No.350 and sequences complementary thereto.

2. A nucleic acid comprising a sequence at least 18 base pairs in length of a segment of the chemically pretreated DNA of genes associated with diseases associated with development according to one of the sequences according to the genes ACCPN, ADFN, AFD1, AHO2, AIH3, AMCD1, AMCD2B, AMCN, AMCX1, AMDM, ANOP1, ASMD, ATD, AXIN1 AF009674, BDB1, BDC, BDE, BDMR, CHH, CHRD AF076612, CHX10, EED (U90651, AF070418, AF078933, AF080227), EPHB2 (L41939, AF025304), FZD8, GLI4,GSC, HNF4B, HOXA3, HOXA6, HOXB9, HOXC9, HOXD8, IHH (L38517), LMX1A, MEIS3 (U68385), ORW2, PKHD1, RIEG2, SFRP2 ( AF017986), SOX10, TBX15, TBX18 (AJ010278), TBX7, TFCOUP2 (M62760, M64497, U60477), WNT14 (AF028702), WNT15 (AF028703), WNT3, WNT6, WNT7B, WNT8A, FGF2 (NM_002006), LHX2 (NM_004789), TBX6 (NM_004608), ZIC3 (NM_003413), ACVR2B (NM_001106), APC (NM_000038), BDNF (NM_001709), BMP1 (NM_006129), BMPR1B (NM_001203), BMPR2 (NM_001204), EDR2 (NM_004427), FGF16 (NM_003868), FGF3 (NM_005247), FGF4 (NM_002007), FGFR3 (NM_000142&NM_022965), FRZB (NM_001466), FZD7 (NM_003507), GLI2 (NM_005270), HNF3A (NM_004496), HOXB4 (NM_024015), HOXB8 (NM_024016), HOXC8 (NM_022658), HOXD1 (NM_024501), INHBB (NM_002193), LMX1B (NM_002316), MADH3 (NM_005902), PAX1 (NM_006192), PAX9 (NM_006194), PBX3 (NM_006195), PROP1 (NM_006261), PTCH2 (NM_003738), RARG (NM_000966), RXRA (NM_002957), SFRP5 (NM_003015), SOX1 (NM_005986), TGFBR1 (NM_004612), WNT2B (NM_004185) and sequences complementary thereto.

3. An oligomer, in particular an oligonucleotide or peptide nucleic acid (PNA)-oligomer, said oligomer comprising in each case at least one base sequence having a length of at least 9 nucleotides which hybridizes to or is identical to a chemically pretreated DNA of genes associated with diseases associated with development according to one of the Seq ID Nos 1 to 350 according to claim 1 or to a chemically pretreated DNA of genes according to claim 2 and sequences complementary thereto.

4. The oligomer as recited in Claim 3; wherein the base sequence includes at least one CpG dinucleotide.

5. The oligomer as recited in Claim 3, **characterized in that** the cytosine of the CpG dinucleotide is located approximately in the middle third of the oligomer.

6. A set of oligomers, comprising at least two oligomers according to any of claims 3 to 5.

7. A set of oligomers as recited in Claim 6, comprising oligomers for detecting the methylation state of all CpG dinucleotides within one of the sequences according to Seq. ID Nos. 1 through 350 according to claim 1 or a chemically pretreated DNA of genes according to claim 2, and sequences complementary thereto.

8. A set of at least two oligonucleotides as recited in Claim 3, which can be used as primer oligonucleotides for the amplification of DNA sequences of one of Seq. ID 1 through Seq. ID 350 and sequences complementary thereto and/or sequences of a chemically pretreated DNA of genes according to claim 2, and sequences complementary thereto and segments thereof.

9. A set of oligonucleotides as recited in Claim 8, **characterized in that** at least one oligonucleotide is bound to a solid phase.

10. Use of a set of oligomer probes comprising at least ten of the oligomers according to any of claims 6 through 9 for detecting the cytosine methylation state and/or single nucleotide polymorphisms (SNPs) in a chemically pretreated genomic DNA according to claim 1 or a chemically pretreated DNA of genes according to claim 2.

11. A method for manufacturing an arrangement of different oligomers (array) fixed to a carrier material for analyzing diseases associated with the methylation state of the CpG dinucleotides of one of the Seq. ID 1 through Seq. ID 350 and sequences complementary thereto and/or chemically pretreated DNA of genes according to claim 2, wherein at least one oligomer according to any of the claims 3 through 5 is coupled to a solid phase.

12. An arrangement of different oligomers (array) obtainable according to claim 11.

13. An array of different oligonucleotide- and/or PNA-oligomer sequences as recited in Claim 12, **characterized in that** these are arranged on a plane solid phase in the form of a rectangular or hexagonal lattice.

14. The array as recited in any of the Claims 12 or 13, **characterized in that** the solid phase surface is composed of silicon, glass, polystyrene, aluminium, steel, iron, copper, nickel, silver, or gold.

15. A DNA- and/or PNA-array for analyzing diseases associated with the methylation state of genes, comprising at least one nucleic acid according to one of the preceding claims.

16. A method for ascertaining genetic and/or epigenetic parameters for the diagnosis and/or therapy of existing diseases or the predisposition to specific diseases by analyzing cytosine methylations, **characterized in that** the following steps. are carried out:
- in a genomic DNA sample, cytosine bases which are unmethylated at the 5-position are converted, by chemical treatment, to uracil or another base which is dissimilar to cytosine in terms of hybridization behaviour;
- fragments of the chemically pretreated genomic DNA are amplified using sets of primer oligonucleotides according to Claim 8 or 9 and a polymerase, the amplificates carrying a detectable label;
- amplificates are hybridized to a set of oligonucleotides and/or PNA probes according to the Claims 6 and 7, or else to an array according to one of the Claims 12 through 15; the hybridized amplificates are subsequently detected.

17. The method as recited in Claim 16, **characterized in that** the chemical treatment is carried out by means of a solution of a bisulfite, hydrogen sulfite or disulfite.

18. The method as recited in one of the Claims 16 or 17, **characterized in that** more than ten different fragments having a length of 100 - 2000 base pairs are amplified.

19. The method as recited in one of the Claims 16 through 18, **characterized in that** the amplification of several DNA segments is carried out in one reaction vessel.

20. The method as recited in one of the Claims 16 through 19, **characterized in that** the polymerase is a heat-resistant DNA polymerase.

21. The method as recited in Claim 20, **characterized in that** the amplification is carried out by means of the polymerase chain reaction (PCR).

22. The method as recited in one of the Claims 16 through 21, **characterized in that** the labels of the amplificates are fluorescence labels.

23. The method as recited in one of the Claims 16 through 21, **characterized in that** the labels of the amplificates are radionuclides.

24. The method as recited in one of the Claims 16 through 21, **characterized in that** the labels of the amplificates are detachable molecule fragments having a typical mass which are detected in a mass spectrometer.

25. The method as recited in one of the Claims 16 through 21, **characterized in that** the amplificates or fragments of the amplificates are detected in the mass spectrometer.

26. The method as recited in one of the Claims 24 and/or 25, **characterized in that** the produced fragments have a single positive or negative net charge for better detectability in the mass spectrometer.

27. The method as recited in one of the Claims 24 through 26, **characterized in that** detection is carried out and visualized by means of matrix assisted laser desorption/ionization mass spectrometry (MALDI) or using electron spray mass spectrometry (ESI).

28. The method as recited in one of the Claims 16 through 27, **characterized in that** the genomic DNA is obtained from cells or cellular components which contain DNA, sources of DNA comprising, for example, cell lines, biopsies, blood, sputum, stool, urine, cerebral-spinal fluid, tissue embedded in paraffin such as tissue from eyes, intestine, kidney, brain, heart, prostate, lung, breast or liver, histologic object slides, and all possible combinations thereof.

29. A kit comprising a bisulfite (= disulfite, hydrogen sulfite) reagent as well as oligonucleotides and/or PNA-oligomers according to one of the Claims 3 through 5.

30. The use of a nucleic acid according to Claims 1 or 2, of an oligonucleotide or PNA-oligomer according to one of the Claims 3 through 5, of a kit according to Claim 29, of an array according to one of the Claims 12 through 15, of a set of oligonucleotides according to one of claims 6 through 9 for the diagnosis of diseases associated with development genes, in particular diseases related to homeobox containing genes (HOX), like diabetes and cancer, apoptosis related diseases, syndromes associated with congenital heart disease, epilepsy, diseases related to histone deacetylation, Currarino syndrome, diseases related with the development of the brain and limb girdle muscular dystrophy, dwarfism, and the like.

31. The use of a nucleic acid according to Claims 1 or 2, of an oligonucleotide or PNA-oligomer according to one of Claims 3 through 5, of a kit according to Claim 29, of an array according to one of the Claims 12 through 15, of a set of oligonucleotides according to one of claims 6 through 9 for the therapy of diseases associated with development genes, in particular diseases related to homeobox containing genes (HOX), like diabetes and cancer, apoptosis related diseases, syndromes associated with congenital heart disease, epilepsy, diseases related to histone deacetylation, Currarino syndrome, diseases related with the development of the brain and limb girdle muscular dystrophy, dwarfism, and the like.
